# EUROPEAN PATENT APPLICATION

(11) **EP 1 548 106 A1**
(43) Date of publication of application: **29.06.2005**
(21) Application number: 03788093.7
(22) Date of filing: 13.08.2003
(51) Int. Cl.: C12N 15/09, C12P 21/02, C07K 14/00, C07K 16/00, G01N 33/15, G01N 33/48, G01N 33/50, G06F 17/30

(54) **HUMAN-ORIGIN PROTEINS FORMING DOMAIN AND USE THEREOF**

(30) Priority: 13.08.2002 JP 2002236129
(71) Applicant: Riken, Wako-shi, Saitama 351-0198 (JP); Kazusa Dna Research Institute, Kisarazu-shi, Chiba 292-0818 (JP)
(72) Inventor: KIGAWA, Takanori, Yokohama Institute, RIKEN, Yokohama-shi, Kanagawa 230-0045 (JP); YOKOYAMA, Shigeyuki, Yokohama Institute, RIKEN, Yokohama-shi, Kanagawa 230-0045 (JP); OHARA, Osamu, KAZUSA DNA RESEARCH INSTITUTE, Kisarazu-shi, Chiba 292-0818 (JP); NAGASE, Takahiro, KAZUSA DNA RESEARCH INSTITUTE, Kisarazu-shi, Chiba 292-0818 (JP); KIKUNO, Reiko, KAZUSA DNA RESEARCH INSTITUTE, Kisarazu-shi, Chiba 292-0818 (JP)
(74) Representative: Mackenzie, Andrew Bryan
(86) International application number: PCT/JP2003/010288
(87) International publication number: WO 2004/016781

(57) **Abstract**

To provide a protein forming a CAP-Gly-like domain, which forms a meaningful domain in three-dimensional structure analysis, information concerning the structure thereof, etc.

There are provided (1) a protein comprising an amino acid sequence represented by SEQ ID NO:1 or a salt thereof; (2) a protein comprising an amino acid sequence represented by any one of SEQ ID NOS:3, 5, 7, and 9 or a salt thereof; (3) a protein comprising an amino acid sequence derived from the amino acid sequence represented by SEQ ID NO:7 by deletion of 0 to 10 amino acid residues from the N-terminal and deletion of 0 to 5 amino acid residues from the C-terminal and having 92 to 106 amino acid residues, or a salt thereof; (4) a protein comprising an amino acid sequence derived from an amino acid sequences of any one of the proteins (1), (2) and (3) and having deletion, substitution or addition of one to several amino acids and having a function substantially identical with that of protein (1), (2) or (3), or a salt thereof; a production method thereof; polynucleotides encoding the same; antibodies against the above proteins; a screening method using the same; and a screening method using the three-dimensional structure thereof.

SEQUENCE LISTING

<110> RIKEN/Kazusa DNA RESEARCH INSTITUTE

<120> Polypeptide which composes human domain and the use
      thereof

<130> P02-0086PCT

<140>
<141>

<150> JP2002-236129
<151> 2002-08-13

<160> 22

<170> PatentIn Ver. 2.1

<210> 1
<211> 91
<212> PRT
<213> Homo sapiens

<400> 1
Leu Ala Met Pro Pro Gly Asn Ser His Gly Leu Glu Val Gly Ser Leu
  1               5                  10                  15

Ala Glu Val Lys Glu Asn Pro Pro Phe Tyr Gly Val Ile Arg Trp Ile
             20                  25                  30

Gly Gln Pro Pro Gly Leu Asn Glu Val Leu Ala Gly Leu Glu Leu Glu
         35                  40                  45

Asp Glu Cys Ala Gly Cys Thr Asp Gly Thr Phe Arg Gly Thr Arg Tyr
     50                  55                  60

Phe Thr Cys Ala Leu Lys Lys Ala Leu Phe Val Lys Leu Lys Ser Cys
 65                  70                  75                  80

Arg Pro Asp Ser Arg Phe Ala Ser Leu Gln Pro
                 85                  90


<210> 2
<211> 273
<212> DNA
<213> Homo sapiens

<400> 2
ttggccatgc ctcctgggaa ctcacatggt ctagaagtgg gctcattggc tgaagttaag 60
gagaaccctc ctttctatgg ggtaatccgt tggatcggtc agccaccagg actgaatgaa 120
gtgctcgctg gactggaact ggaagatgag tgtgcaggct gtacggatgg gaccttcaga 180
ggcactcggt atttcacctg tgccctgaag aaggcgctgt ttgtgaaact gaagagctgc 240
aggcctgact ctaggtttgc atcattgcag ccg                              273


<210> 3
<211> 101
<212> PRT
<213> Homo sapiens

<400> 3
Asn Thr Ala Pro Val Gln Glu Ser Pro Pro Leu Ala Met Pro Pro Gly
  1               5                  10                  15

Asn Ser His Gly Leu Glu Val Gly Ser Leu Ala Glu Val Lys Glu Asn
             20                  25                  30

Pro Pro Phe Tyr Gly Val Ile Arg Trp Ile Gly Gln Pro Pro Gly Leu
        35                  40                  45

Asn Glu Val Leu Ala Gly Leu Glu Leu Glu Asp Glu Cys Ala Gly Cys
    50                  55                  60

Thr Asp Gly Thr Phe Arg Gly Thr Arg Tyr Phe Thr Cys Ala Leu Lys
65                  70                  75                  80

Lys Ala Leu Phe Val Lys Leu Lys Ser Cys Arg Pro Asp Ser Arg Phe
                85                  90                  95

Ala Ser Leu Gln Pro
            100


<210> 4
<211> 303
<212> DNA
<213> Homo sapiens

<400> 4
aacactgcac ccgtccaaga gagtccaccc ttggccatgc ctcctgggaa ctcacatggt 60
ctagaagtgg gctcattggc tgaagttaag gagaaccctc ctttctatgg ggtaatccgt 120
tggatcggtc agccaccagg actgaatgaa gtgctcgctg gactggaact ggaagatgag 180
tgtgcaggct gtacggatgg aaccttcaga ggcactcggt atttcacctg tgccctgaag 240
aaggcgctgt ttgtgaaact gaagagctgc aggcctgact ctaggtttgc atcattgcag 300
ccg                                                               303


<210> 5
<211> 106
<212> PRT
<213> Homo sapiens

<400> 5
Asn Thr Ala Pro Val Gln Glu Ser Pro Pro Leu Ala Met Pro Pro Gly
1               5                   10                  15

Asn Ser His Gly Leu Glu Val Gly Ser Leu Ala Glu Val Lys Glu Asn
            20                  25                  30

Pro Pro Phe Tyr Gly Val Ile Arg Trp Ile Gly Gln Pro Pro Gly Leu
        35                  40                  45

Asn Glu Val Leu Ala Gly Leu Glu Leu Glu Asp Glu Cys Ala Gly Cys
    50                  55                  60

Thr Asp Gly Thr Phe Arg Gly Thr Arg Tyr Phe Thr Cys Ala Leu Lys
65                  70                  75                  80

Lys Ala Leu Phe Val Lys Leu Lys Ser Cys Arg Pro Asp Ser Arg Phe
                85                  90                  95

Ala Ser Leu Gln Pro Val Ser Asn Gln Ile
            100                 105


<210> 6
<211> 318
<212> DNA
<213> Homo sapiens

<400> 6
aacactgcac ccgtccaaga gagtccaccc ttggccatgc ctcctgggaa ctcacatggt 60
ctagaagtgg gctcattggc tgaagttaag gagaaccctc ctttctatgg ggtaatccgt 120
tggatcggtc agccaccagg actgaatgaa gtgctcgctg gactggaact ggaagatgag 180

tgtgcaggct gtacggatgg aaccttcaga ggcactcggt atttcacctg tgccctgaag 240
aaggcgctgt ttgtgaaact gaagagctgc aggcctgact ctaggtttgc atcattgcag 300
ccggtttcca atcagatt 318

<210> 7
<211> 96
<212> PRT
<213> Homo sapiens

<400> 7
Leu Ala Met Pro Pro Gly Asn Ser His Gly Leu Glu Val Gly Ser Leu
1               5                   10                  15

Ala Glu Val Lys Glu Asn Pro Pro Phe Tyr Gly Val Ile Arg Trp Ile
            20                  25                  30

Gly Gln Pro Pro Gly Leu Asn Glu Val Leu Ala Gly Leu Glu Leu Glu
        35                  40                  45

Asp Glu Cys Ala Gly Cys Thr Asp Gly Thr Phe Arg Gly Thr Arg Tyr
    50                  55                  60

Phe Thr Cys Ala Leu Lys Lys Ala Leu Phe Val Lys Leu Lys Ser Cys
65                  70                  75                  80

Arg Pro Asp Ser Arg Phe Ala Ser Leu Gln Pro Val Ser Asn Gln Ile
                85                  90                  95

<210> 8
<211> 288
<212> DNA
<213> Homo sapiens

<400> 8
ttggccatgc ctcctgggaa ctcacatggt ctagaagtgg gctcattggc tgaagttaag 60
gagaaccctc ctttctatgg ggtaatccgt tggatcggtc agccaccagg actgaatgaa 120
gtgctcgctg gactggaact ggaagatgag tgtgcaggct gtacggatgg aaccttcaga 180
ggcactcggt atttcacctg tgccctgaag aaggcgctgt ttgtgaaact gaagagctgc 240
aggcctgact ctaggtttgc atcattgcag ccggtttcca atcagatt 288

<210> 9
<211> 40
<212> DNA
<213> Artificial Sequence

<220>
<223> Description of Artificial Sequence:Primer

<400> 9
ggtgccacgc ggatccctga ccaccgagaa cagattccac 40

<210> 10
<211> 45
<212> DNA
<213> Artificial Sequence

<220>
<223> Description of Artificial Sequence:Primer

<400> 10
tatgctagcg gccgctcatt actgattgga aaccggctgc aatga 45

<210> 11
<211> 124
<212> DNA
<213> Artificial Sequence

<220>
<223> Description of Artificial Sequence:Primer

<400> 11
gaaattaata cgactcacta tagggagacc acaacggttt ccctctagaa ataattttgt 60
ttaactttaa gaaggagata tacatatgca ccatcatcat catcatctgg tgccacgcgg 120
atcc 124


<210> 12
<211> 66
<212> DNA
<213> Artificial Sequence

<220>
<223> Description of Artificial Sequence:Primer

<400> 12
ttcagcaaaa aacccctcaa gacccgttta gaggccccaa ggggttatgc tagcggccgc 60
tcatta 66


<210> 13
<211> 33
<212> DNA
<213> Artificial Sequence

<220>
<223> Description of Artificial Sequence:Primer

<400> 13
ccagcggctc ctcgggaaac actgcacccg tcc 33


<210> 14
<211> 33
<212> DNA
<213> Artificial Sequence

<220>
<223> Description of Artificial Sequence:Primer

<400> 14
ccagcggctc ctcgggattg gccatgcctc ctg 33


<210> 15
<211> 34
<212> DNA
<213> Artificial Sequence

<220>
<223> Description of Artificial Sequence:Primer

<400> 15
cctgacgagg gccccgacgg ctgcaatgat gcaa 34


<210> 16
<211> 36
<212> DNA

<213> Artificial Sequence

<220>
<223> Description of Artificial Sequence:Primer

<400> 16
cctgacgagg gccccgaaat ctgattggaa accggc 36

<210> 17
<211> 227
<212> DNA
<213> Artificial Sequence

<220>
<223> Description of Artificial Sequence:Primer

<400> 17
gctcttgtca ttgtgcttcg catgattacg aattcagatc tcgatcccgc gaaattaata 60
cgactcacta tagggagacc acaacggttt ccctctagaa ataattttgt ttaactttaa 120
gaaggagata tacatatgaa aggcagcagc catcatcatc atcatcacga ttacgatatc 180
ccaacgaccg aaaacctgta ttttcaggga tccagcggct cctcggg 227

<210> 18
<211> 187
<212> DNA
<213> Artificial Sequence

<220>
<223> Description of Artificial Sequence:Primer

<400> 18
cggggccctc gtcaggataa taattgattg atgctgagtt ggctgctgcc accgctgagc 60
aataactagc ataacccctt ggggcctcta aacgggtctt gaggggtttt ttgctgaaag 120
gaggaactat atccggataa cctcgagctg caggcatgca agcttggcga agcacaatga 180
caagagc 187

<210> 19
<211> 20
<212> DNA
<213> Artificial Sequence

<220>
<223> Description of Artificial Sequence:Primer

<400> 19
gctcttgtca ttgtgcttcg 20

<210> 20
<211> 104
<212> PRT
<213> Homo sapiens

<400> 20
Gly Ser Ser Gly Ser Ser Gly Leu Ala Met Pro Pro Gly Asn Ser His
1               5                   10                  15

Gly Leu Glu Val Gly Ser Leu Ala Glu Val Lys Glu Asn Pro Pro Phe
            20                  25                  30

Tyr Gly Val Ile Arg Trp Ile Gly Gln Pro Pro Gly Leu Asn Glu Val
        35                  40                  45

Leu Ala Gly Leu Glu Leu Glu Asp Glu Cys Ala Gly Cys Thr Asp Gly
    50                  55                  60

Thr Phe Arg Gly Thr Arg Tyr Phe Thr Cys Ala Leu Lys Lys Ala Leu
65                  70                  75                  80

Phe Val Lys Leu Lys Ser Cys Arg Pro Asp Ser Arg Phe Ala Ser Leu
                85                  90                  95

Gln Pro Ser Gly Pro Ser Ser Gly
            100

<210> 21
<211> 419
<212> PRT
<213> Homo sapiens

<400> 21

<210> 22
<211> 1260
<212> DNA
<213> Homo sapiens

<400> 22
atgaataggc acctctggaa gagccaactg tgtgagatgg tgcagcccag tggtggcccg 60
gcagcagatc aggacgtact gggcgaagag tctcctctgg ggaagccagc catgctgcac 120
ctgccttcag aacagggcgc tcctgagacc ctccagcgct gcctggagga gaatcaagag 180
ctccgagatg ccatccggca gagcaaccag attctgcggg agcgctgcga ggagcttctg 240
catttccaag ccagccagag ggaggagaag gagttcctca tgtgcaagtt ccaggaggcc 300
aggaaactgg tggagagact cggcctggag aagctcgatc tgaagaggca gaaggagcag 360
gctctgcggg aggtggagca cctgaagaga tgccagcagc agatggctga ggacaaggcc 420
tctgtgaaag cccaggtgac gtccttgctc ggggagctgc aggagagcca gagtcgcttg 480
gaggctgcca ctaaggaatg ccaggctctg gagggtcggg cccgggcggc cagcgagcag 540
gcgcggcagc tggagagtga gcgcgaggcg ctgcagcagc agcacagcgt gcaggtggac 600
cagctgcgca tgcagggcca gagcgtggag gccgcgctcc gcatggagcg ccaggccgcc 660
tcggaggaga agaggaagct ggcccagttg caggtggcct atcaccagct cttccaagaa 720
tacgacaacc acatcaagag cagcgtggtg ggcagtgagc ggaagcgagg aatgcagctg 780
gaagatctca aacagcagct ccagcaggcc gaggaggccc tggtggccaa acaggaggtg 840
atcgataagc tgaaggagga ggccgagcag cacaagattg tgatggagac cgttccggtg 900
ctgaaggccc aggcggatat ctacaaggcg gacttccagg ctgagaggca ggcccgggag 960
aagctggccg agaagaagga gctcctgcag gagcagctgg agcagctgca gagggagtac 1020
agaaaactga aggccagctg tcaggagtcg gccaggatcg aggacatgag gaagcggcat 1080
gtcgaggtct cccaggcccc cttgcccccc gcccctgcct acctctcctc tcccctggcc 1140
ctgcccagtc agaggaggag gcccccagag gagccacctg acttctgctg tcccaagtgc 1200
cagtatcagg cccctgatat ggacaccctg cagatacatg tcatggagtg cattgagtag 1260

## Description

### Technical Field

The present invention relates to a human-derived protein forming a domain, a polynucleotide comprising the same, an antibody against the protein, and a method for screening an active compound using them.

### Background Art

Recently, genome nucleotide sequences of various model organisms, as represented by the human genome project, have been decoded one after another. "Structural genomics" is rapidly recognized as a new research area, and large-scale projects are progressing around the world. With respect to a large number of genes extracted from a mass of information about genomic sequences, structural genomics aims at systematic clarification of three-dimensional structure of a protein encoded by each gene and defining structure-function relationship.

In structural genomics, the number of proteins to be analyzed is considered to be 100,000 kinds, and it is not realistic to have a goal to determine the three-dimensional structures of all the proteins at present technical level.

Thus, it is first necessary to narrow down the number of target proteins to a reasonable number and select "representative structures". As a comprehensive three-dimensional structure analysis, projects having selected analysis targets from various viewpoints are initiated: for example, a) specifying relatively small sets of proteins as targets; b) specifying the kind of organisms having a small genome size such as hyperthermophilic archaebacterium, extreme thermophile, and mycoplasma; c) specifying life phenomena, such as proteins involved in signal transduction or gene expression, and proteins as disease-associated gene products.

In the flow of this research, as the first step, it is one goal to determine one or more representative three-dimensional structure regarding all the families, the number of which is predicted to be about 10, 000 (classified as family when amino acid sequences have about 30 to 35% homology). When one representative three-dimensional structure (basic structure) is obtained, structures of other proteins belonging to the same family can be analogized by modeling based on homology.

In this project, attention is drawn to the type of three-dimensional structure or the topology (basic structure: fold) of a functional domain, and research to clarify the correlation with a function for a basic structural unit of protein receives attention.

A protein having a plurality of domains is formed by combining functional domains like a module, and thus it is frequent that one domain appears in various proteins with the combination of different domains. Further, even if homology is not detected on a primary sequence, it is not unusual that proteins have the same basic structure. Therefore, the number of types of basic structures must be much smaller than the number of protein families, and it is expected that individual basic structures are associated with molecular functions. The number of the basic structures is predicted to be about 10,000 to 20,000, and if the analysis targets are within this number order it is sufficiently possible to determine the three-dimensional structures of all the target proteins.

Thus obtained information regarding the three-dimensional structure and functions of protein provides new findings for elucidation of vital functions, and makes a dramatic progress in developing drugs etc. (e.g. development by rational drug design or virtual screening). Therefore, such information is very useful in the industry.

However, the fact is that three-dimensional structure analysis of protein requires a lot of time, labor and cost. In structural genomic research aiming at comprehensive and systematic structure analysis, it is an important challenge to attain high throughput structure analysis.

For the analysis of a three-dimensional structure of protein, NMR method and X-ray crystallography are mainly used.

To analyze a three-dimensional structure of protein using NMR, it is preferable that a sample has a molecular weight of about 20,000 or less (about 200 or less amino acid residues). In the case of analysis of the three-dimensional structure by X-ray analysis, the properties of proteins are limited due to preparation of crystals.

When a protein is randomly cleaved to obtain a protein suitable for structure analysis and a cleavage site exists in an amino acid sequence having a β-sheet or α-helix structure, many proteins modify their physiologically significant structures, become a string-like shape not taking a structure, or aggregate. In this way, it is meaningless to analyze a three-dimensional structure of protein not having an original in vivo structure. Therefore, it is desirable to obtain a protein forming a significant domain for three dimensional analysis.

To express a protein having a domain suitable for structural analysis (hereinafter referred to as "a protein forming a domain"), information regarding the position of domain boundary is necessary. In general, such domain boundary is predicted using amino acid sequence homology or the like as a clue. Even if protein expression is conducted based on the amino acid sequence of the thus predicted domain region, there is very low probability that a protein forming a domain actually having a structure (folding) is obtained and thus domain expression is one of bottlenecks in structure analysis.

A protein forming a CAP-Gly-like domain of the present invention has not been obtained so far, and the structure information thereof is unknown. Thus, these cannot be used for the drug discovery.

### Disclosure of the Invention

In view of such circumstances, the present invention has been accomplished, and the present invention provides a protein described below, a production method thereof, a polynucleotide coding therefor, an antibody against the protein, and a screening method using them.
(1) A protein consisting of an amino acid sequence represented by SEQ ID NO:1, or a salt thereof.
(2) A protein consisting of an amino acid sequence represented by any one of SEQ ID NOS:3, 5, and 7, or a salt thereof.
(3) A protein having an amino acid sequence derived from an amino acid sequence represented by SEQ ID NO:5 by deletion of 0 to 10 amino acid residues from the N-terminal and deletion of 0 to 5 amino acid residues from the C-terminal and having 92 to 106 amino acid residues, or a salt thereof.
(4) A protein consisting of an amino acid sequence derived from an amino acid sequence of a proteins according to any one of the above (1), (2), and (3) and having deletion, substitution or addition of one to several amino acids and having a function substantially identical with that of the protein according to the above (1), (2), or (3), or a salt thereof.
(5) A polynucleotide comprising a polynucleotide encoding an amino acid sequence of any one of proteins according to the above (1) to (4).
(6) The polynucleotide according to the above (5), containing a nucleotide sequence represented by any one of SEQ ID NOS:2, 4, 6, and8 .
(7) A recombinant vector containing a polynucleotide according to the above (5) or (6).
(8) A transformant which is transformed with a polynucleotide according to the above (5) or (6).
(9) An antibody against a protein according to any one of the above (1) to (4).
(10) A method for producing a protein or a salt thereof according to any one of the above (1) to (4), comprising the steps of culturing the transformant of the above (8) and producing the protein.
(11) A method for producing a protein or a salt thereof according to any one of the above (1) to (4), characterized by using a cell-free protein synthesis system.
(12) A method for screening a substance interacting with a protein or a salt thereof according to any one of the above (1) to (4)and/or a naturally existing protein or a salt thereof containing an amino acid sequence of a protein according to any one of the above (1) to (4), comprising the steps of bringing a candidate substance into contact with the protein of any one of the above (1) to (4); and confirming whether the candidate substance interacts with the protein.
(13) A method for assaying a protein or a salt thereof according to any one of the above (1) to (4) using an antibody of the above (9).
(14) A method for screening a substance interacting with a protein or a salt thereof according to any one of the above (1) to (4) using an assay method of the above (13).
(15) A method for specifying a gene associated with a protein according to any one of the above (1) to (4), comprising the steps of expressing the protein according to any one of the above (1) to (4) in a cell; and examining an expression status of the gene in the cell.
(16) A method for screening a substance interacting with a protein or a salt thereof according to any one of the above (1) to (4) and/or a naturally existing protein or a salt thereof containing an amino acid sequence of a protein according to any one of the above (1) to (4), comprising the steps of determining an active site of the protein using information concerning three-dimensional structure of the protein according to any one of the above (1) to (4); and specifying a compound interacting with the active site on a computer.
(17) The screening method according to the above (16), wherein the information concerning three-dimensional structure of the protein is three-dimensional structure information of a protein consisting of amino acid residues from amino acid 8 to amino acid 98 among three-dimensional structure information described in any of three-dimensional structure coordinate tables 1 to 20.
(18) The screening method according to the above (17), wherein, among three-dimensional structure information described in three-dimensional structure coordinate table 1, a part of information corresponding to amino acid residues (Val26, Lys27, Glu47, Arg67, Lys83 and Ser86) is used.
(19) A method for screening a substance interacting with a protein or a salt thereof according to any one of the above (1) to (4) and/or a naturally existing protein or a salt thereof containing an amino acid sequence of a protein according to any one of the above (1) to (4), wherein a compound interacting with a specified active site is prepared as a candidate compound by a screening method according to any one of the above (16) to (18), the method comprising the steps of bringing the candidate substance into contact with a protein according to any one of the above (1) to (4); and confirming whether the candidate substance has interaction with the protein.
(20) A method for presuming a three-dimensional structure of a protein with an unknown structure, wherein homology modeling is conducted on the protein with an unknown structure comprising an amino acid sequence having 30% or more homology with an amino acid sequence of a protein according to any one of the above (1) to (4), by using information concerning three-dimensional structure information of a protein having amino acid residues from amino acid 8 to amino acid 98 among three-dimensional structures of a protein described in any of three-dimensional structure coordinate tables 1 to 20.

### Brief Description of the Drawings

Fig. 1 shows SDS gel electrophoregrams of Examples 1 to 4. Fig. 1A shows an SDS gel electrophoregram of Example 1, Fig. 1B shows an SDS gel electrophoregram of Example 2, Fig. 1C shows an SDS gel electrophoregram of Example 3, and Fig. 1D shows an SDS gel electrophoregram of Example 4;
Fig. 2 shows SDS gel electrophoregrams of respective Comparative Examples. Fig. 2A shows an SDS gel electrophoregram of Comparative Example 1, Fig. 2B shows an SDS gel electrophoregram of Comparative Example 2, and Fig. 2C shows an SDS gel electrophoregram of Comparative Example 3;
Fig. 3 shows one-dimensional nuclear magnetic resonance spectrum and ¹H-¹⁵N HSQC spectrum of the protein represented by SEQ ID NO:1. Fig. 3A shows a one-dimensional nuclear magnetic resonance spectrum and Fig. 3B shows a ¹H-¹⁵N HSQC spectrum;
Fig. 4 shows one-dimensional nuclear magnetic resonance spectrum and ¹H-¹⁵N HSQC spectrum of the protein represented by SEQ ID NO:3. Fig. 4A shows a one-dimensional nuclear magnetic resonance spectrum and Fig. 4B shows a ¹H-¹⁵N HSQC spectrum;
Fig. 5 shows one-dimensional nuclear magnetic resonance spectrum and ¹H-¹⁵N HSQC spectrum of the protein represented by SEQ ID NO:5. Fig. 5A shows a one-dimensional nuclear magnetic resonance spectrum and Fig. 5B shows a ¹H-¹⁵N HSQC spectrum;
Fig. 6 shows one-dimensional nuclear magnetic resonance spectrum and ¹H-¹⁵N HSQC spectrum of the protein represented by SEQ ID NO:7. Fig. 6A shows a one-dimensional nuclear magnetic resonance spectrum and Fig. 6B shows a ¹H-¹⁵N HSQC spectrum;
Fig. 7 is a graph showing measurement results of viable cell count using 293 cells and HeLa cells; and
Fig. 8 is a photograph, instead of a figure, showing a detected image of western blotting in Example 9. Lane 1 is a lane of only a supernatant of HeLa cell extract (control), lane 2 is a lane of a product wherein IKK-gamma (1-419) and a FLAG sequence were expressed in a HeLa cell (control), and lane 3 is a lane of a product wherein IKK-gamma (1-419) and addition of a FLAG sequence to a CAP-Gly-like domain protein (464-554) were expressed in a HeLa cell.

### Best Mode for Carrying Out the Invention

### (Protein of the present invention)

A protein of the present invention is a protein forming a domain with a three-dimensional structure. More particularly, the present invention relates to a CAP-Gly-like domain protein represented by any one of SEQ ID NOS:1, 3, 5, and 7, or a protein having an amino acid sequence derived from an amino acid sequence represented by SEQ ID NO:5 by deletion of 0 to 10 amino acid residues from the N-terminal and deletion of 0 to 5 amino acid residues from the C-terminal and having 92 to 106 amino acid residues (that is, a protein wherein 0 to 10 amino acid residues and 0 to 5 amino acid residues are added to N-terminal and C-terminal of SEQ ID NO:1, respectively).

Hereinafter, the protein of the present invention will be described in terms of functions and the like.

CAP-Gly is an abbreviation of cytoskeletal-associated-protein-glycine-conserved domain, and constitutes a protein having a role for combining an intracellular minute organ or a chromosome with an intracellular microtubule. A CAP-Gly domain includes highly preservative region abundant in glycin comprising about 42 residues [Riehemann K., Sorg C. Sequence homologies between four cytoskeleton-associated proteins. Trends Biochem. Sci. 18: 82-83(1993)]. As a protein containing this domain, there are known: restin, a protein of 160 kDa pertaining to an intermediate filament connecting an intracellular endoplasmic reticurum to a microtubule (also called as cytoplasmic linker protein 170 or CLIP-170); dynactin of vertebra [dynein associated polypeptide of 150 kDa (DAP)]; Drosophila glued complex which is a main component of activator I; yeast protein BIK1 which is considered necessary for the formation and stabilization of microtubules at mitosis and for spindle fusion at pairing; yeast protein NIP100 (NIP80); human protein CKAP1/TFCB; alp11 of *Schizosaccharomyces pombe* protein; and F53F4.3 which is presumed a protein of *C. elegans*.
Further, with respect to the relation between a CAP-Gly-like domain protein and diseases, Examples have shown that CAP-Gly-like domain has cell proliferation inhibition effect and that CAP-Gly-like domain binds to IKK-gamma. It is accordingly predicted that the CAP-Gly-like domain is a causative gene of cancer-related diseases. In other words, in Example 8, the CAP-Gly-like domain protein exhibited proliferation inhibition effect in HeLa cells (uterine cervix cancer cells) and 293 cells. In Example 9, the protein exhibited an ability to bind to IKK-gamma. Here, IKK-gamma is a factor which binds to a transcription factor NF-kB to inhibit transcription enhancing function of NF-kB. Further, NF-kB is formed as a heterocomplex from p50 and p65 derived from oncogenes of the *Rel* family. The CAP-Gly-like domain binds to IKK-gamma and exhibits proliferation inhibition activity, and it can be thus considered that its binding to IKK-gamma inhibits transcription enhancement of NF-kB. In this way, inhibition of cell proliferation is controlled by the CAP-Gly-like domain, and therefore the protein of the present invention has a function as a cancer suppressor gene product. It is a well-known fact that when a mutation in a cancer suppressor gene and lowered proliferation inhibition activity lead to the onset of cancer (Yoichi Taya et al., Bio Science Term Library, Cancer Gene-Cancer suppressor gene, pp. 113-115, Yodosha, 2000). Retinoblastoma gene is well known, which causes retinoblastoma (Weinberg RA. The retinoblastoma protein and cell cycle control. Cell 81, 323-330 1995). Particularly, a gene (KIAA0849) used of the present invention is known as a causative gene of human Turban tumor syndrome (Nature Genet. 25, 160-165 2000). Still, it is unknown which domain of the protein expressed by KIAA0849 is a cause to the disease. However, in consideration of the cancer suppressing genetic function of the protein of the present invention, as exemplified by the present invention, CAP-Gly-like domain protein is predicted to be a cause of the disease. As application of the present invention, screening of drugs for Turban syndrome and optimizing a drug using three-dimensional structure information are assumed. It can be effectively used for the prevention and treatment of various cancer-related diseases.

Further, the present invention provides: a protein or a salt thereof consisting of an amino acid sequence represented by any one of SEQ ID NOS: 1, 3, 5, and 7, or an amino acid sequence derived from the amino acid sequence represented by SEQ ID NO:5 by deletion of 0 to 10 amino acid residues from the N-terminal and deletion of 0 to 5 amino acid residues from the C-terminal, which further comprises by deletion, substitution or addition of one to several (1 to 9, preferably 1 to 5, more preferably 1 to 2) amino acids and which has a function substantially identical to that of the protein consisting of an amino acid sequence derived from the amino acid sequence represented by SEQ ID NO:5 by deletion of 0 to 10 amino acid residues from the N-terminal and deletion of 0 to 5 amino acid residues from the C-terminal.

It should be noted that the term "having a cell proliferation inhibition function substantially identical to that of the protein of the present invention" means to have the same cell proliferation inhibition function as the protein of the present invention has. Here, such molecular functions include functions and activities of the CAP-Gly-like domain protein in addition to connectivity with IKK-gamma.

IKK-gamma is an abbreviation of inhibitor-kappaB, and binds to NF(Nuclear Factor)-kB. NF-kB is a transcription factor that binds to an intron enhancer of immunoglobulin kL chain gene, and is a heterodimer composed of two kinds of subunits, p50 and p65. Here, both p50 and p65 exhibit homology with oncogene Rel and belong to the Rel family. As its function, NF-kB was initially considered to be specific to a B cell and to control the expression of a cell specific gene that is expressed as the B cell differentiates. Thereafter, the same effect was also observed to a non-B cell. Further, NF-kB binds to transcription control regions of various genes in addition to an immunoglobulin gene, and activates the transcription of these genes. In a non-B cell, an inactive NF-kB binds to IKK-gamma thereby to form a complex. IKK-gamma is inactivated by phosphorylation induced by extracellular stimulation. As a result, NF-kB is dissociated and transferred into each nucleus thereby to be activated, so that it functions as a transcription factor. As subunits of IKK-gamma, IKK-α, IKK-β, and bcl-3 are known.

### (Sequence of protein)

The sequencing of the protein of the present invention is conducted as follows: i) presuming a domain region having a function of interest based on known protein sequence information; ii) preparing domain candidate sequence patterns having a basic pattern as the amino acid sequence of the thus presumed domain; iii) expressing a protein of each sequence pattern, evaluating structural stability of the obtained protein forming the domain, using a protein having a good result as a domain of interest, and defining each protein according to the amino acid sequence of the domain of interest. In other words, the protein of the present invention is empirically selected so as to have a stable structure when a part (having a function of interest) of the full length protein is fragmented and expressed as a protein forming a domain. There exist domain candidates in the order of 100 at the stage of domain region prediction, but the number of domain candidates is narrowed down due to various factors and in fact carefully selected in the order of 10 to several tens. Therefore, use of thus selected proteins in three-dimensional structure analysis enables highly accurate and reliable structure analysis.

### (Presumption of domain region)

A method for presuming a domain region in the full length protein is not particularly limited, and any of the following methods can be used: information science methods such as bioinformatics or computational science methods (see the specification of Japanese Patent Application No. 2001-309434), combination of deleted DNA library and GFP (see the specification of Japanese Patent Laid Open No. 2002-262873), and experimental methods such as limited breakdown (proteolysis) by protease. By using more accurate methods, the efficiency to select a domain of interest from domain candidates is improved.

### (Production of domain candidate sequence pattern)

The above domain candidate sequence patterns are produced by extending or shortening the position of domain boundary to the N- or C- terminals on the basis of the above presumed domain region.

For example, prepared is a domain candidate sequence pattern having about several tens of kinds of new boundaries as the N-terminal, which are provided by extending several to tens of residues to the N-terminal or shortening several to tens of residues the C-terminal, from the position of the amino acid residue in the domain boundary at the N-terminal of the presumed domain region. Similarly, produced is a domain candidate sequence pattern having several kinds of domain boundaries as the C-terminal, which are selected in the domain boundary at the C-terminal of the presumed domain region.

### (Extending and shortening of domain boundary)

As a method for extending or shortening a domain boundary of the above presumed domain region, employed is, for example, a method for synthesizing individual PCR primers capable of producing cDNAs corresponding to the above domain candidate sequence patterns and performing the creation by PCR. In particular, 2-step PCR method described in Japanese Patent Laid Open No.2003-9880 is suitable.

### (Extraction of target domain from domain candidate sequence pattern)

In order to select a target domain having actually stable three-dimensional structure from the above domain candidate sequence patterns, protein synthesis is performed using cDNA of domain candidate sequence pattern produced as mentioned above.

An expression system for the domain candidate sequence pattern is not particularly limited, and any of conventionally known expression systems are usable.

Next, it is determined whether the obtained protein actually has a stable three-dimensional structure, and when a protein confirmed to have such three-dimensional structure is used as a protein in the present invention.

Examples of indicators for the stability of three-dimensional structure of protein include: biochemical indicators such as an indicator whether a synthesized domain protein is detected as soluble protein by SDS gel electrophoresis, etc. and also detected as uniform band corresponding to a proper molecular weight; and spectroscopic methods having as an indicator fluorescence strength of GFP fused at the C-terminal side, NMR spectrum, and CD spectrum.

The conventional process of determining a protein sequence has problems in that, for example, (1) a protein forming a target domain is not expressed in the above protein synthesis, or (2) though the protein is expressed, it causes aggregation or has low solubility, etc. The present inventors have overcome these problems and completed the present invention.

### (Confirmation of having stable three-dimensional structure)

Regarding the above NMR spectrum, the determination for folding of a protein forming a domain is shown below.

When a protein forming a domain is not folded in 1D spectrum, signals derived from methyl group proton such as Val, Leu, and Ile are observed around 0.8 ppm. However, when a protein is folded, the environment of methyl group proton is changed and signals are shifted to higher magnetic field side (around 0.7 ppm to -0.5 ppm).

The determination in ¹H-¹⁵NHSQC can be made by visual evaluation of the cross peak convergence degree and the uniformity of signal strength. In other words, when cross peaks are densely gathered, the status is considered not forming a three-dimensional structure. Conversely, when dispersed, the status is considered forming a stable three-dimensional structure. In this way, the stability of three-dimensional structure is evaluated.

### (Vector)

A (recombinant) vector of the present invention can be obtained by ligating (inserting) a gene of the present invention into a proper vector. The vector for inserting the gene of the present invention thereinto is not particularly limited as long as it can be replicated in a host cell. Examples thereof include plasmid DNAs and phage DNAs.

Specific examples of the plasmid DNAs include *E. coli*-derived plasmids (e.g. pRSET, pBR322, pBR325, pUC118, pUC119, pUC18, and pUC19), *Bacillus subtilis*-derived plasmids (e.g. pUB110 and pTP5), yeast-derived plasmids (e.g. YEp13, YEp24, and YCp50). Specific examples of the phage DNAs include λ phage (Charon4A, Charon21A, EMBL3, EMBL4, λgt10, λgt11, and λZAP). Further, animal viruses such as retrovirus and vaccinia virus and insect virus vectors such as baculovirus can be used.

To insert a gene of the present invention into a vector, a method is employed, which comprises first cleaving a purified DNA with a proper restriction enzyme and inserting the gene to a restriction enzyme site of a proper vector DNA or a multicloning site to ligate to the vector.

It is necessary for the gene of the present invention to be incorporated into a vector so that the gene exhibits its function. Hence, in addition to a promoter and the gene of the present invention, an enhancer or the like including a cis-element, a splicing signal, a poly A addition signal, a selective marker, and a ribosome junction sequence (SD sequence) can be ligated into the vector of the present invention, if desired. Further, examples of the selective makers include a dihydrofolate reductase gene, an ampicillin resistance gene, and a neomycin resistance gene.

### (Transformant)

A transformant of the present invention can be obtained by introducing a polynucleotide of the present invention into a host so that a gene of interest can be expressed therein. Due to easiness and good efficiency, vectors are used for transformation in many cases. Herein, a host is not particularly limited, as long as it can express DNA of the present invention. Examples thereof include bacterium belonging to genus *Escherichia* such as *Escherichia coli,* genus *Bacillus* such as *Bacillus subtilis,* genus *Pseudomonas* such as *Pseudomonas putida*, genus *Rhizobium* such as *Rhizobium meliloti.* Further, yeasts such as *Saccharomyces cerevisiae* and *Schizosaccharomyces pombe*, and animal cells such as COS cells and CHO cells can be used. Or insect cells such as Sf9 and Sf21 can be used.

When a bacteria such as *E. coli* is a host, it is preferable that the recombinant vector of the present invention comprises a promoter, a ribosome junction sequence, a gene of the present invention, and a transcription termination sequence while autonomously replicable in the bacteria. In addition, a gene to control the promoter may be contained.

Examples of *E. coli* include *E. coli* K12 and DH1 and examples of *Bacillus subtilis* include *Bacillus subtilis.* As a promoter, anyone can be used as long as it can be expressed in a host such as E. coli. Promoters derived from *E. coli* or phages such as trp promoter, lac promoter, P_{L} promoter, and P_{R} promoter can be used. Artificially designed and modified promoters such as tac promoter can be used. A method for introducing a recombinant vector into a bacteria is not particularly limited, as long as it is a method for introducing a DNA into bacteria. There are, for example, a method using calcium ion (Cohen, S.N. et al. (1972) Proc. Natl. Acad. Sci., USA 69, 2110-2114) and electroporation method.

When yeast is a host, *Saccharomyces cerevisiae, Schizosaccharomyces pombe,* and *Pichia pastor* is, for example, are used. In this case, a promoter is not particularly limited as long as it can be expressed in yeast. Examples thereof include gall promoter, gal 10 promoter, a heat shock protein promoter, MFα1 promoter, PHO5 promoter, PGK promoter, GAP promoter, ADH promoter, and AOX1 promoter. A method for introducing a recombinant vector into a yeast is not particularly limited as long as the method can introduce a DNA into a yeast. Examples of the methods include electroporation method (Becker, D.M. et al. (1990) Methods. Enzymol., 194,182-187), spheroplast method (Hinnen, A. et al. (1978) Proc. Natl. Acad. Sci., USA 75, 1929-1933), and lithium acetate method (Itoh, H. (1983) J. Bacteriol. 153,163-168).

When an animal cell is a host, a monkey cell COS-7, Vero, Chinese hamster ovary cell (CHO cell), a mouse L cell, a rat GH3, and a human FL cell can be used. As a promoter, SRα promoter, SV40 promoter, LTR promoter, and CMV promoter can be used, and further an early gene promoter of human cytomegalovirus may be used. Examples of the methods for introducing a recombinant vector into an animal cell include electroporation method, calcium phosphate method, and lipofection method.

When an insect cell is a host, an Sf9 cell, an Sf21 cell or the like may be used. As a method for introducing a recombinant vector into an insect cell, calcium phosphate method, lipofection method, and electroporation method may be used, for example.

### (Antibody)

Using the protein of the present invention as an antigen, an antibody against the antigen can be prepared.

### [Production of a polyclonal antibody against the protein of the present invention]

An animal is immunized using the aforementioned antigen. In the case of a rabbit, a dose per animal of an antigen is 100 to 500 µg using, for example, an adjuvant. As the adjuvant, Freund's complete adjuvant (FCA), Freund's incomplete adjuvant (FIA), aluminum hydroxide adjuvant, etc. are used.

Immunization is carried out by administration to mammalians (e.g. non-human mammalians such as a rat, a mouse, and a rabbit). Administration is conducted intravenously, hypodermically, or intraperitoneally. In addition, immunization interval is not particularly limited, and it may be several-day to several-week interval, preferably 2- to 3-week interval. At such interval, an animal is immunized 1 to 10 times, preferably 2 to 3 times. After 6 to 60 days from final immunization, antibody titer is measured. On a day when the greatest antibody titer is exhibited, blood is collected to obtain antiserum. The antibody titer is measured by ELISA (enzyme-linked immunosorbent assay), RIA (radioimmuno assay), or the like.

When purification of an antibody is needed from antiserum, the purification can be conducted by properly selecting a well-known method such as ammonium sulfate precipitation method, ion exchange chromatography, gel filtration, and affinity chromatography, or combination thereof.

### [Production of a monoclonal antibody against the protein]

An animal is immunized using the aforementioned antigen. If necessary, an adjuvant (commercially available Freund's complete adjuvant, Freund's incomplete adjuvant, etc.) may be mixed in the same manner as above to perform immunization effectively.

Immunization is carried out by administration to mammalians (e.g. a rat, a mouse, and a rabbit). A dose per mouse of an antigen is 50 µg. Administration is conducted mainly intravenously, hypodermically, or intraperitoneally. Further, immunization interval is not particularly limited, and it may be several-day to several-week interval, preferably 2- to 3-week interval at least two to three times. Then, antibody-producing cells are collected after final immunization. As an antibody-producing cell, there are a spleen cell, a lymph node cell, a peripheral blood cell, or the like, but a spleen cell is preferable.

### [Cell fusion]

To obtain a hybridoma, cell fusion of an antibody producing cell and a myeloma cell is performed. As a myeloma cell to be fused with an antibody producing cell, an established cell line can be used, which is generally available and derived from an animal such as a mouse. Preferably used is a cell line which has drug selectivity, and has properties whereby it is unable to survive in HAT selective medium (containing hypoxanthine, aminopterin, and thymidine) without the fusion but able to survive only with the fusion with an antibody producing cell. Specific examples of myeloma cells include mouse myeloma cell lines such as P3X63-Ag.8.U1(P3U1), P3/NSI/1-Ag4-1, and Sp2/0-Ag14.

Next, cell fusion of the above myeloma cell with an antibody producing cell is performed. In an animal cell culture medium such as DMEM and RPMI-1640 medium without inclusion of serum, antibody producing cells and myeloma cells are mixed in the ratio of 15:1 to 25:1. Fusion reaction is performed in the presence of a cell fusion accelerator such as polyethylene glycol, or by electric pulse treatment (e.g. electroporation).

### [Selection and cloning of hybridoma]

From cells treated by cell fusion, a hybridoma of interest is selected. For example, the treated cells are cultured in a medium containing hypoxanthine, aminopterin and thymidine, and growing cells are obtained as a hybridoma.

Next, screening is performed to determine whether an antibody of interest exists in a culture supernatant of increased hybridoma. The screening of hybridoma may be performed, without particular limitation, in a conventional manner. For example, a part of culture supernatant grown as hybridoma in a well is collected, and screened by ELISA (enzyme-linked immunosorbent assay), RIA (radioimmuno assay) or the like. Cloning of fused cells is performed by limiting dilution method or the like, and finally a hybridoma of a monoclonal antibody producing cell is established.

### [Collection of monoclonal antibody]

As a method for collecting monoclonal antibodies from established hybridoma, a conventional cell culture method or the like may be employed. In the cell culture method, the hybridoma is cultured for 3 to 10 days under ordinary culture conditions (e.g. 37°C, 5% CO₂ concentration) in an animal cell culture medium such as RPMI-1640 or MEM media containing 10% bovine fetus serum, and antibodies are collected from resultant culture supernatant.

In the above antibody collection method, antibodies can be purified, if necessary, by properly selecting a well-known method such as ammonium sulfate precipitation method, ion exchange chromatography, affinity chromatography, and gel chromatography, or combination thereof.

### (Production of the protein of the present invention)

The protein of the present invention can be obtained by culturing a transformant and collecting the protein from the culture. The term "culture" means, in addition to a culture supernatant, any of a cultured cell, a cultured fungus body, and a matter of crushed cell or fungus body. "A method for culturing a transformant of the present invention" is performed according to a conventional method used for culturing a host.

As a culture medium for culturing the transformant obtained by using microorganisms such as E. coli and yeast as a host, any of natural medium and synthetic medium may be used as long as it contains carbon source, nitrogen source, mineral, etc. which can be utilized as resource by the microorganisms and it effectively cultures the transformant. As the carbon source, used are carbohydrates such as glucose, fructose, sucrose and starch, organic acids such as acetic acid and propionic acid, and alcohols such as ethanol and propanol. As the nitrogen source, used are ammonium salts of inorganic or organic acids such as ammonia, ammonium chloride, ammonium sulfate, ammonium acetate, and ammonium phosphate, or other nitrogen-containing compounds as well as peptone, meat extract, and corn steep liquor. As inorganic matters, monopotassium phosphate, dipotassium phosphate, magnesium phosphate, magnesium sulfate, sodium chloride, ferrous sulfate, manganese sulfate, copper sulfate, and calcium carbonate may be used.

Culture is carried out preferably at 37°C under aerobic conditions such as shaking culture or aerobic culture with stirring for 6 to 24 hours. During the culture period, pH is kept at 7.0 to 7.5. The pH is adjusted preferably by using inorganic or organic acid, alkali solution, etc. During the culture, antibiotics such as ampicillin and tetracycline may be added to the culture medium if necessary.

When a microorganism is cultured which has transformed with an expression vector having used an inducible promoter as a promoter, an inducer may be added to the medium if necessary. For example, when a microorganism transformed with an expression vector having used Lac promoter is cultured, isopropyl-β-D-thiogalactopyranoside (IPTG), etc. may be added to the medium. When a microorganism transformed with an expression vector having used trp promoter is cultured, indole acrylic acid (IAA) may be added to the medium.

As a medium for culturing a transformant obtained by using an animal cell as a host, RPMI1640 or DMEM media, which are commonly used, or these media having bovine fetus serum added thereto may be used. Culture is carried out at 37°C for 1 to 30 days in the presence of 5% CO₂. During the culture period, antibiotics such as kanamycin, penicillin etc. may be added to the medium.

After the culture, protein is extracted by crushing a fungus body or a cell when the protein is produced in a fungus body or a cell. Further, the protein of the present invention is produced outside a fungus body or a cell, the culture medium is used as it is or the fungus body or cell is removed by centrifugation, etc. Thereafter, the protein of the present invention can be isolated and purified from the above culture medium by using either alone or proper combination of biochemical methods commonly used for isolation and purification of protein, such as ammonium sulfate precipitate, gel chromatography, ion exchange chromatography, affinity chromatography, etc. During or after this purification process, the tag sequence, which was used for purification by protease treatment can be removed.

### (Method for producing a protein forming a domain using a cell-free protein synthesis system)

Using a cell-free protein synthesis system, the present invention provides a method for producing: a protein comprising an amino acid sequence represented by any one of SEQ ID NOS:1, 3, 5, and 7; and a protein comprising an amino acid sequence derived from the amino acid sequence represented by SEQ ID NO:5 by deletion of 0 to 10 amino acid residues from the N-terminal and deletion of 0 to 5 amino acid residues from the C-terminal and having 92 to 106 amino acid residues.

A cell-free protein synthesis system is a system in which proteins are synthesized in vitro by using a cell extract. "A cell-free protein synthesis system" includes both a cell-free translation system for synthesizing proteins on ribosome through reading of information of mRNA, and a system including both a cell-free transcription system for synthesizing RNA using DNA as the template and a cell-free translation system. Since a cell-free protein synthesis system can modify a system easily, it has an advantage to easily construct an expression system suitable for a target protein. Further, a cell-free protein synthesis system is described in detail in Japanese Patent Laid Open No. 2000-175695.

### [Cell extract]

A crude cell extract may be an extract from eukaryotic or prokaryotic cell in a state of high protein synthesis activity such as bacteria (e.g. *E. coli),* fungi (e.g. budding yeast), wheat germ, rabbit reticulocyte, murine L-cell, Ehrlich ascetic cancer cell, HeLa cell, and CHO cell (Clemens, M.J., Transcription and translation- a practical approach, (1984), pp. 231-270, Henes, B.D. and Higgins, S.J. eds., IRL Press, Oxford).

A crude cell extract preferably contains a component required for protein synthesis such as ribosome and tRNA. For preparation of a crude extract, a method described, for example, in Pratt, J.M. et al., transcription and translation - a practical approach, (1984), pp. 179-209, Henes, B.D. and Higgins, S.J. eds., IRL Press, Oxford, can be used. More specifically, the preparation can be conducted by crushing with a French press (Pratt, mentioned above) or crushing with glass beads. A preferable cell extract is *E. coli* S30 cell extract. S30 extract can be prepared from E. coli BL21 Codon Plus strain in accordance with generally known methods such as a method of Pratt et al. (above mentioned), or S30 extract commercially available from Promega or Novagen can be used. The cell extract derived mainly from *E. coli*, wheat germ, and rabbit reticulocyte.

### [Dialyzer]

A dialyzer which enables shaking or agitating while having internal and external dialysates isolated from each other via a dialysis membrane can be used. Examples of a small-scale reaction apparatus include Dispo Dialyzer (registered trademark) (manufactured by Spectrum) and Slidealyzer (registered trademark) (manufactured by Pierce).

Further, examples of a large-scale reaction apparatus include Spectra/Por (registered trademark) dialysis tube (manufactured by Spectrum).

### [Internal dialysate]

In addition to a concentrated cell extract such as E. coli S30, an internal dialysate of a cell-free protein synthesis system, liquid for synthesis of protein, may contain DNA or RNA (mRNA and the like) encoding the target proteins, ATP (adenosine 5'-triphosphate), GTP (guanosine 5'-triphosphate), CTP (cytidine 5'-triphosphate), UTP (uridine 5'-triphosphate), buffer solutions, salts, amino acids, RNase inhibitors, antibacterial agents, RNA polymerase if necessary (in a case where DNA is used as template), and tRNA.

In addition, it can contain ATP regenerating systems such as combinations of phosphoenolpyruvate and pyruvate kinase, or creatine phosphate and creatine kinase, polyethyleneglycol (for example, PEG#8000), 3',5'-cAMP, folic acids, RNase inhibitors, and reducing agents (for example, dithiothreitol). On the other hand, an external dialysate (that is, protein synthesis substrate solution) can use the same composition of the internal dialysate excluding cell extract, RNase inhibitors, DNA or RNA, and RNA polymerase. For example, it may contain buffer solutions, ATP, GTP, CTP, UTP, salts, amino acids, and antibacterial agents. The concentration of added components can be determined arbitrarily.

### [Buffer solution]

As the buffer solution, buffer agent such as Hepes-KOH and Tris-OAc can be used, for example. Examples of the salts include acetates (for example, ammonium salts, magnesium salts, and the like) and glutamate salts. Examples of the antibacterial agents include sodium azide and ampicillin. Examples of the amino acids include 20 kinds of amino acids that construct proteins. In a case where DNA is used as a template, RNA polymerase is added to the reaction system, and a commercially available enzyme such as T7 RNA polymerase can be used.

The internal dialysate is put inside the dialysis membrane, and the external dialysate is put outside the membrane. By shaking or stirring of the closed system in which substances can transfer through the membrane in dependence on the cutoff molecular weight, a target protein thus produced can be collected from the internal or external dialysates. For the reaction conditions such as temperature, stirring rate, and so forth, any condition can be applied depending on the kind of proteins. In the case of protein synthesis, the temperature to be applied is usually approximately 25 to 50°C, preferably 37°C. However, the temperature for cell-free protein synthesis system using a fungus extract derived from *Thermus thermophilus* may exceed 50°C. Further, the shaking rate or stirring rate may be low, and, for example, 100 to 200 rpm can be applied. While observing the production of the target protein, the reaction period can be properly determined.

In the cell-free protein synthesis system, it is desirable to exchange the external dialysate for a fresh external dialysate when the reaction rate is reduced. Moreover, the use of a dialysis membrane with a cutoff molecular weight of more than 10,000 Da, preferably more than approximately 50,000 Da, enables higher output of the proteins.

### [Purification of protein]

Since the quantity and the number of kinds of mixed contaminants are extremely small, compared with the isolation from living cells, purification of the produced proteins can be achieved with relative ease. Depending on the properties of the proteins, conventionally known purification methods can be used either alone or, if necessary, in combination. Common techniques can be used, such as ammonium sulfate or acetone precipitation, acid extraction, anion or cation exchange chromatography, hydrophobic interaction chromatography, affinity chromatography, gel filtration chromatography, HPLC, electrophoresis, and chromatofocusing. During or after this purification process, a tag sequence used for purification can be removed by protease treatment. Identification and quantitative determination of the produced proteins can be achieved by activity assay, immunological assay, spectroscopic measurement, amino acid analysis, and the like, and, if necessary, comparing with a standard sample.

### (Screening method)

As a screening method of the present invention, there is a method for screening a compound having interaction with a protein of the present invention, which comprises a process of bringing a candidate substance into contact with the protein or a salt thereof, and a process of confirming whether the protein interacts with the candidate substance. Here, the expression "having interaction" means to inhibit or strengthen molecular function and/or physiological activity of the protein by combining the compound with the protein and so on. In this screening method, the protein is brought into contact with the candidate substance, and it is determined whether molecular function or physiological activity of the protein is changed.

### [Searching for an interactive substance using NMR]

When NMR is used to search for an interactive substance, the presence of interaction can be determined based on the existence of signal changes of the protein before and after addition of the candidate substance. In other words, when an interactive candidate substance interacts with the protein, it is expected that a chemical shift value, a line width, the number, etc. of NMR signals derived from the vicinity of interactive site of the protein may be changed, and thus the presence of interaction can be determined by detecting such changes. In particular, ¹⁵N-labeled protein is prepared with relative ease, and ¹⁵N-HSQC spectrum obtained therefrom has relatively high resolution and sensitivity. Further, the spectrum is less affected by NMR signals derived from added interactive candidate substance, and thus it is very useful.

### (Assay method)

The protein of the present invention can be assayed by using, for example, an antibody of the present invention. Examples of a method for assaying a protein using an antibody include sandwich immunoassay, competitive method, immunometric method, and nephelometry method. Further, it is also detectable using labels such as radioisotope, an enzyme, and a fluorescent material.

### (Screening method using assay method)

The antibody of the present invention is specifically combined with the protein of the present invention, and thus it can be used for screening a compound having interaction with the protein of the present invention. As a screening method therefor, known screening methods are usable.

In addition, according to the assay method of the protein of the present invention using the antibody of the present invention, diseases involving the protein of the present invention can be prevented and diagnosed.

### (Three-dimensional structure analysis)

The three-dimensional structure of the protein can be analyzed by NMR structure analysis, X-ray structure analysis, etc.

### (NMR)

A sample used for NMR is not particularly limited, but a sample in which ¹²C or ¹⁴N in the protein is labeled with a stable isotope, ¹³C or ¹⁵N nuclear is used preferably (multi-nuclear and multi-dimensional NMR measurement). Stable-isotope labeling of a protein is a common technique, and is described in Clore, G.M.& Gronenbom, A.M., Science, 252, p.1390-1399, 1991, or the like. In particular, analysis using a protein sample having a main chain labeled with ¹⁵N uniform stable isotope is easily and preferably carried out. In addition, a protein having the skeleton of the main chain labeled with at least two or more kinds of isotopes of ¹³C, ¹⁵N, and ²H may be used (National Publication of International Patent Application No. 2001-514239).

It is preferable to measure ¹⁵N-¹H spin coupling constant by observing IPAP-HSQC spectrum, etc. The term "IPAP-HSQC spectrum" is a measurement method for reading ¹⁵N-¹H spin coupling constant effectively by simultaneously observing two HSQC spectra of level and reverse phases, and adding both spectra thereby to prevent overlapping of the signals.

Chemical shift attribution is performed by two or more kinds of NMR methods. For example, 2D, DOQ-COSY, TOCSY, NOESY, and HSQC are well known as two dimensional NMR, and HNCO, HCACO, HNCA, HCA(CO)N, HN(CO)CA, HNHB, CBCANH, H(CA)NH, HBHA(CO)NH, HCCH-COSY, HCANH, HCCH-TOCSY, HCACON, ¹⁵N-NOESY-HSQC, ¹³C-NOESY-HSQC are well known as multi dimensional NMR. A general technique of NMR is known, and described in, for example, "NMR of Protein" (Yoji Arata, Kyoritsu Shuppan, Co., Ltd., 1996); "Basic Biochemical Experiment Method Vol. 3, Protein I, Detection and Structure Analysis Method edited by Japan Biochemical Society" Chapter 18, Three-dimensional structure analysis by NMR (Tokyo Kagaku Dozin, Co., Ltd., Feb. 2001); Takashi Ito et al., Journal Vol. 21 of Japan Agrochemical Society, pp. 450-459, 1996; and Toshiyuki Tanaka, Chemistry and Industry Vol. 49, No. 2, pp. 155-158, 1996.

When NMR is used for three-dimensional structure analysis, it is a common method that the distance between protons is estimated in accordance with the scale of nucleus overhauser effect between individual protons of the protein, and based on the distance information, the three-dimensional structure is determined. It is possible to obtain a three-dimensional structure with precision by adding information concerning a chemical shift value, a scalar coupling value, a residual dipolar coupling value, hydrogen bond or the like.

Many programs for structure analysis from NMR data are known. Structure analysis is performed preferably by using NMR Pipe, PIPP, Capp, Felix, NMR View, and XEASY for chemical shift attribution, and by using X-PLOR, CNS, DYANA, and DYNAMO as three dimensional calculation softwares.

### (X-ray crystallography)

When X-ray crystallography is used for three-dimensional structure analysis, an electron density map is calculated based on an X-ray diffraction image of crystallized protein, and the three-dimensional structure is determined. In other words, the protein is crystallized and mono-colored X-ray is applied to the crystal, and based on the obtained X-ray diffraction image, the three-dimensional structure of the protein is clarified (Blundell, T.L. and Johnson, L.N., PROTEIN CRYSTALLOGRAPHY, pp. 1-565, (1976) Academic Press, New York).

### (Screening method based on three-dimensional structure information)

Next, the present invention provides, using information concerning three-dimensional structure of the aforementioned protein, a method for screening a compound having interaction with the protein or a salt thereof, which comprises a process of determining an active site of the protein, and a process of searching for the compound having interaction with the active site on a computer.

### (In silico screening)

Regarding drug design based on three-dimensional structure of a molecule, there are many reviews including Drug Development, Vol. 7 "Molecular Design" (Hirokawa Shoten). Specifically, screening is first conducted by a computer on the library (e.g. about 150,000 kinds) of low molecular compounds (1000 or less molecular weight) stored in a relational database such as Oracle by use of a flexible ligand binding simulation software such as FlexiDock and FlexX. The three-dimensional structure of a chemical compound of this library is designated by a program such as CONCORD, and it is possible to select a substance that can be inserted into an active site. Among the selected substances, a compound that is fit into the active site more precisely is visually selected by using a simulation program such as Insight II or MOE. Computer softwares used in a series of the above processes are the following commercially available ones.

FlexiDock: Tripos Inc.FlexX: Tripos Inc.CONCORD: Tripos Inc.Oracle: Oracle Corp.Insight II: Molecular Simulations Inc.MOE: Chemical Computing Group Inc.

Another method is to design candidate compounds including unknown substances by a computer. As such method, the following methods are known: a method for searching for a compatible compound by aligning a chemical group such as methyl and ethyl in an active site; and a method of aligning an atom in an active site by a computer program.

### (Wet screening)

To select a major candidate compound having interaction with the protein of the present invention, the candidate compound obtained by in silico screening is brought into contact with the protein of the present invention and the molecular function or physiological activity of the protein of the invention is determined. Based on the three-dimensional structure data of the candidate compound and the protein of the invention, the candidate compound is modified so as to have more desirable structure.

The selected compound is synthesized and actually interacted with the protein for screening. With respect to a compound that has changed the activity of the protein, further testing relating to in vitro activity, in vivo dynamics, or toxicity is performed by animal tests.

### (Pharmaceutical Agent containing an interactive substance)

A substance interacting with the protein of the invention can be used as a preventive and/or therapeutic agent to diseases involving the protein. Such a pharmaceutical agent can be orally or parenterally administered to the whole body or locally.

When the drug of the invention is orally administered, it may be prepared in any type of formulation such as a tablet, a capsule, a granule, powder, a pill, trochiscus, internal use liquor, a suspension, an emulsion, and syrup, and may be prepared as a dried product which is dissolved again at administration. Further, when the drug of the invention is parenterally administered, a formulation such as an intravenous injection (including intravenous drip), intramuscular injection, intraperitoneal injection, subcutaneous injection, and suppositories may be selected. In the case of formulations for injection, the drug may be provided in the form of an ample with a unit dose or a container for large volume administration.

These formulations can be produced by conventional methods by properly selecting an excipient, an extender, a binder, a wetting agent, a disintegrator, a lubricant, a surfactant, a dispersant, a buffer, a preservative, a solubilizing agent, an antiseptic, a corrective, an analgesic agent, a stabilizing agent, and an isotonic agent, which are commonly used for formulation.

The above various formulations may contain a pharmaceutically acceptable carrier or additive. Examples of these carriers and additives include water, pharmaceutically acceptable organic solvents, collagen, polyvinyl alcohol, polyvinyl pyrrolidone, carboxyvinyl polymer, sodium alginate, water-soluble dextran, carboxymethyl starch sodium, pectin, xanthan gum, gum Arabic, casein, gelatin, agar, glycerol, propylene glycol, polyethylene glycol, Vaseline, paraffin, stearyl alcohol, stearic acid, a human serum albumin, mannitol, sorbitol, and lactose. Additives to be used are selected from the above-mentioned property or in combination according to the type of formulation of the invention.

A dose of the drug of the invention can be varied depending on the age of a recipient, administration path, and the number of administration times, and thus it can be changed in a wide range. In this case, the effective dose of the protein of the invention and the effective dose to be administered in combination with suitable diluent and pharmacologically usable carried are selected in the range of 0.01 mg to 1,000 mg per 1 kg of the body weight for one time, and the administration is conducted preferably once to several times per day for one day or more.

### (Description of Sequences)

The sequence numbers of the description indicate the following sequences.
SEQ ID NO:1 represents an amino acid sequence of a CAP-Gly like domain protein (KIAA0849 protein (464-554)).
SEQ ID NO:2 represents a DNA sequence encoding the protein of SEQ ID NO:1.
SEQ ID NO:3 represents an amino acid sequence of a CAP-Gly like domain protein (KIAA0849 protein (454-554)).
SEQ ID NO:4 represents a DNA sequence encoding the protein of SEQ ID NO:3.
SEQ ID NO:5 represents an amino acid sequence of a CAP-Gly like domain protein (KIAA0849 protein (454-559)) (amino acid sequence having amino acid residues NTAPVQESPP and VSNQI added to the N- and C- terminals of the amino acid sequence of SEQ ID NO:1).
SEQ ID NO:6 represents a DNA sequence encoding the protein of SEQ ID NO:5.
SEQ ID NO:7 represents an amino acid sequence of a CAP-Gly like domain protein (KIAA0849 protein (464-559)).
SEQ ID NO:8 represents a DNA sequence encoding the protein of SEQ ID NO:7.
SEQ ID NO:9 represents a nucleotide sequence of a primer used for the following Example.
SEQ ID NO:10 represents a nucleotide sequence of a primer used for the following Example.
SEQ ID NO:11 represents a nucleotide sequence of a primer used for the following Example.
SEQ ID NO:12 represents a nucleotide sequence of a primer used for the following Example.
SEQ ID NO:13 represents a nucleotide sequence of a primer used for the following Example.
SEQ ID NO:14 represents a nucleotide sequence of a primer used for the following Example.
SEQ ID NO:15 represents a nucleotide sequence of a primer used for the following Example.
SEQ ID NO:16 represents a nucleotide sequence of a primer used for the following Example.
SEQ ID NO:17 represents a nucleotide sequence of a primer used for the following Example.
SEQ ID NO:18 represents a nucleotide sequence of a primer used for the following Example.
SEQ ID NO:19 represents a nucleotide sequence of a primer used for the following Example.
SEQ ID NO:20 represents an amino acid sequence in which a plurality of amino acid residues are added to the N- and C- terminals of the amino acid sequence of SEQ ID NO:1.
SEQ ID NO:21 represents an amino acid sequence of IKK-gamma (1-419).
SEQ ID NO:22 represents a nucleotide sequence of cDNA of the protein represented by SEQ ID NO:21.

### [Examples]

The present invention will be described in detail by showing Examples below, but the scope of the present invention is not limited by them.

### [Example 1]

### (1) Presumption of domain

The presumption of a domain was conducted in the following manner.
First, 1) <SCOP method> when a region having homology with a sequence contained in protein database SCOP (Version 1.55) was detected from query sequences, such region was predicted as a domain. BLASTP was used as homology detection method, and it was determined to be homologous when E-value had 0.1 or less hits.
2) <PFAM method> when a region having homology with a sequence profile contained in protein motif database PFAM (version 6.5) was detected from query sequences, such region was predicted as a domain. HMMER was used as homology detection method, and it was determined to be homologous when E-value had 0.1 or less hits.
3) <ProDom method> when a region having homology with any of consensus sequences contained in protein motif database ProDom (a version obtained through the Web January, 2001) was detected from query sequences, such region was predicted as a domain. BLASTP was used as homology detection method, and it was determined to be homologous when E-value had 0.1 or less hits.
4) <NR method> homology search on query sequences was conducted by BLASTP in protein sequence data set (NCBI-nr), and when E-value had 0.1 or less hits, such homologous regions were grouped and predicted as domain.
5) <PASS method> homology search on query sequences was conducted by BLASTP in protein sequence data set (NCBI-nr), and the frequency of detecting homology was calculated. Peaks and troughs were used to indicate high frequency parts and low frequency parts, and one peak was predicted as domain so that a trough part is a domain boundary.
6) <No Hit method> regions (residual regions) that were not detected as domain by using any of the above 1) to 5) methods were predicted as domain.
7) <Differential domain boundary setting method> regarding the above six domain predictions, when a hit region was overlapped, high priority was given to 1), and followed by 2), 3), 4), 5), and 6) in this order. When the deviation of the domain boundary includes 30 residues or more and such residues are present in more length in the N- or C-terminal side in accordance with definition by lower priority method, though a hit region was overlapped, such differential sequence was predicted as other domain.

Among domain regions presumed by any of the above methods, regions having one or more Low-Complexity regions (sequence region with low complexity) or having the full length of less than 30 residues were removed.

Regarding the amino acid sequence of KIAA0849 protein, the presumption of domain region was conducted in the above-mentioned manner. According to NR method, the range of 496th to 539th of amino acid residues was presumed as domain region. This domain region had no Low-Complexity region (sequence region with low complexity) and has the full length of 30 or more residues. Hence, this region was finally considered a result of domain presumption by bioinformatics.

### (2) Producing Constructs

With respect to the protein (here referred to as presumed domain) having amino acid sequence of 496 to 539 amino acid residues of this KIAA0849 protein, protein synthesis reaction was conducted using a cell-free protein synthesis system described below. Thereafter, the obtained sample was subjected to SDS gel electrophoresis by conventional method to examine protein expression status.

As a result, a protein of interest was not expressed from information concerning amino acid sequence of the presumed domain.

Therefore, using the amino acid sequence of the presumed domain as a standard, constructs were systematically produced by extending or shortening the domain boundary position of the presumed domain, relative to amino acid sequence of KIAA0849 protein, by addition or deletion of several residues at the N- and C-terminals, respectively.

In other words, using, for example, 496th amino acid residue of KIAA0849 protein as a standard, a pattern having 10 residues added to the N-terminal side and 10 residues deleted from the C-terminal side was prepared. Also, using 539th amino acid residue of KIAA0849 protein as a standard, a pattern having 5 residues deleted from the N-terminal side and 5 residues added to the C-terminal side was prepared.

Using the constructs thus produced, protein expression for individual patterns was performed and their expression statuses were evaluated by SDS gel electrophoresis.

### [Example 1]

In this example, among constructs produced in the above manner, KIAA0849 protein (464-554): (SEQ ID No. 1) having a CAP-Gly like domain suitable for three-dimensional structure analysis was studied. Results of the study are described.

### (1) Construction of expression vector

### (i) First PCR

Using a recombinant *E. coli* culture solution containing a plasmid wherein cDNA (DDBJ accession No. AB020656.2) encoding KIAA0849 protein was cloned in a plasmid pBluescriptII SK+, PCR was performed by use of 5'-primer 1 (SEQ ID NO:14) and 3'-primer 1 (SEQ ID NO:15). The composition of the PCR reaction solution is shown in Table 1. The program followed a conventional PCR protocol.

**[Table 1]**

| Composition of reaction solution for first PCR | | | |
|---|---|---|---|
| Composition | Concentration | Amount | Final concentration |
| Template plasmid | (×1/10) | 3 µL | (×3/200) |
| 5'-primer 1 | 0.25 µM | 4 µL | 0.05 µM |
| 3'-primer 1 | 0.25 µM | 4 µL | 0.05 µM |
| dNTPs(Toyobo) | 2 mM | 2 µL | 0.2 mM |
| Expand HiFi buffer solution (containing 15 mM magnesium chloride) (Roche) | (10×) | 2µL | (1×) |
| Sterile distilled water | | 4.85 µL | |
| DNA polymerase (Roche) | 3.5 U/µL | 0.15 µL | 0.02625 U/µL |
| Total amount | | 20 µL | |

### (ii) Secondary PCR

Next, second PCR was performed using the first PCR product obtained in the foregoing reaction, 5'-primer 2 (SEQ ID NO:17) having His tag sequence in the downstream of T7 promoter sequence, 3'-primer 2 (SEQ ID NO:18) having a T7 terminator sequence, and universal primer-U2 (SEQ ID NO:19). The composition of the PCR reaction solution is shown in Table 2. The program was the same as the above first PCR.

**[Table 2]**

| Composition of reaction solution for second PCR | | | |
|---|---|---|---|
| Composition | Concentration | Amount | Final concentration |
| First PCR product (template) | (×1/5) | 5 µL | (×1/20) |
| 5'-primer 2 | 2 µM | 0.5 µL | 0.05 µM |
| 3'-primer 2 | 2 µM | 0.5 µL | 0.05 µM |
| Universal primer U2 | 100 µM | 0.2 µL | 1 µM |
| dNTPs (Toyobo) | 2 mM | 2 µL | 0.2 mM |
| Expand HiFi buffer solution (containing 15 mM magnesium chloride) (Roche) | (10×) | 2 µL | (1×) |
| Sterile distilled water | | 9.65 µL | |
| DNA polymerase (Roche) | 3.5 U/µL | 0.15 µL | 0.02625 U/µL |
| Total amount | | 20 µL | |

As a result, a linear double stranded DNA fragment was amplified, which can express a fusion protein of His tag sequence and KIAA0849 protein (464-554) under the control of T7 promoter.

### (iii) Cloning

The DNA fragment obtained by the above second PCR reaction was cloned in a vector pPCR2.1 (Invtrogen) with TOPO TA-cloning kit (Invtrogen), and thereby an expression vector P011213-03 was constructed.

### (2) Expression of KIAA0849 protein (464-554)

### (i) Synthesis of ¹⁵N-labeled CAP-Gly-like domain by cell-free protein synthesis method using dialysis

*E. coli* S30 extract was prepared from *E. coli* BL21 codon plus strain according to a method of Zubay et al. (Annu. Rev. Geneti. 7, 267-287, 1973).

The protein synthesis reaction was conducted overnight at 30°C in the scale of 3 mL of reaction solution having the composition of Table 3 and 30 mL of external dialysate having the composition of Table 4.

**[Table 3]**

| Composition of reaction solution | |
|---|---|
| Composition | Final concentration |
| Hepes-KOH (pH 7.5) | 58 mM |
| DTT | 1.8 mM |
| ATP | 1.2 mM |
| CTP | 0.8 mM |
| GTP | 0.8 mM |
| UTP | 0.8 mM |
| Creatine phosphate | 80 mM |
| Creatine kinase | 0.25 mg/mL |
| Polyethylene glycol (average molecular weight 8000) | 4.0% |
| 3',5'-cAMP | 0.64 mM |
| L(-)-5-formyl-5,6,7,8-tetrahydroforic acid | 68 µM |
| *E. coli* total tRNA | 175 µg/mL |
| Potassium glutamate | 210 mM |
| Ammonium acetate | 27.5 mM |
| Magnesium acetate | 10.7 mM |
| [¹⁵N] labeled amino acid mixture | 3 mg/mL |
| L-[¹⁵N] cystein | 1 mM |
| L-[¹⁵N] tryptophan | 1 mM |
| L-[¹⁵N] glutamine | 1 mM |
| L-[¹⁵N] asparagine | 1 mM |
| Sodium azide | 0.05% |
| T7 RNA polymerase | 66.6 µg/mL |
| S30 extract | 30% |
| Template DNA (P011213-03) | 1 µg/mL |

**[Table 4]**

| Composition of external dialysate | |
|---|---|
| Composition | Final concentration |
| Hepes-KOH (pH 7.5) | 58 mM |
| DTT | 1.8 mM |
| ATP | 1.2 mM |
| CTP | 0.8 mM |
| GTP | 0.8 mM |
| UTP | 0.8 mM |
| Creatine phosphate | 80 mM |
| Creatine kinase | 0.25 mg/mL |
| Polyethylene glycol (average molecular weight 8000) | 4.0% |
| 3',5'-cAMP | 0.64 mM |
| L(-)-5-formyl-5,6,7,8-tetrahydroforic acid | 68 µM |
| Potassium glutamate | 210 mM |
| Ammonium acetate | 27.5 mM |
| Magnesium acetate | 10.7 mM |
| [¹⁵N]-labeled amino acid mixture | 3 mg/mL |
| L-[¹⁵N] cystein | 1 mM |
| L-[¹⁵N] trypptophan | 1 mM |
| L-[¹⁵N] glutamine | 1 mM |
| L-[¹⁵N] asparagine | 1 mM |
| Sodium azide | 0.05% |

### (ii) Determination of expression status by SDS gel electrophoresis

After the termination of the synthesis reaction, SDS gel electrophoresis was performed by a conventional method, and the expression status of the obtained protein was determined.

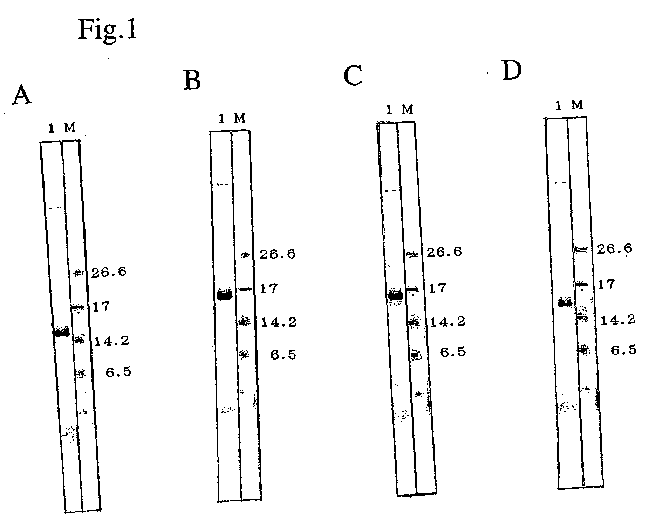

Results thereof are shown in Fig. 1A. According to Fig. 1A, it was confirmed that KIAA0849 protein (464-554) (CAP-Gly-like domain protein having an amino acid sequence represented by SEQ ID NO:1) was expressed. In Fig. 1A, the first lane is a lane of a protein containing a fraction of interest in purification, and M is a marker lane.

### [Examples 2 to 4]

In Examples 2 to 4, among constructs produced in the above manner, KIAA0849 protein (454-554) (Example 2), KIAA0849 protein (454-559) (Example 3), and KIAA0849 protein (464-559) (Example 4) were studied. Results of the study are described.

Specifically, primers used for the first and second PCRs of Example 1 were changed to those shown in Table 5 and except that, an expression vector was constructed in the same manner as Example 1. The obtained linear double stranded DNA fragment was used as a template DNA for protein synthesis.

**[Table 5]**

| Sequence numbers of each primer used for first and second PCRs | | | | | |
|---|---|---|---|---|---|
| | First PCR | | Second PCR | | |
| Example No. | 5'-primer 1 | 3'-primer 1 | 5'-primer 2 | 3'-primer 2 | Universal primer |
| 1 | SEQ ID NO:14 | SEQ ID NO:15 | SEQ ID NO:17 | SEQ ID NO:18 | SEQ ID NO:19 |
| 2 | SEQ ID NO:13 | SEQ ID NO:15 | SEQ ID NO:17 | SEQ ID NO:18 | SEQ ID NO:19 |
| 3 | SEQ ID NO:13 | SEQ ID NO:16 | SEQ ID NO:17 | SEQ ID NO:18 | SEQ ID NO:19 |
| 4 | SEQ ID NO:14 | SEQ ID NO:16 | SEQ ID NO:17 | SEQ ID NO:18 | SEQ ID NO:19 |

After the termination of the synthesis reaction, SDS gel electrophoresis was performed in the same manner as in Example 1 to determine the expression level of the obtained proteins.

Results thereof are shown in Fig. 1B to Fig. 1D. Fig. 1B shows an SDS gel electrophoregram of Example 2, Fig. 1C shows an SDS gel electrophoregram of Example 3, and Fig. 1D shows an SDS gel electrophoregram of Example 4. In Figs. 1B to 1D, the first lane is a lane of a protein containing a fraction of interest in purification, and M is a marker lane, respectively. In Fig. 1B, a band is observed at a position corresponding to KIAA0849 protein (454-554). According to this result, it is found that KIAA0849 protein (454-554) was expressed in Example 2. Further, according to Fig. 1C, it was found that KIAA0849 protein (454-559) was expressed in Example 3 in the same way as Fig. 1B. Furthermore, according to Fig. 1D, it was found that KIAA0849 protein (464-559) was expressed in Example 4.

### [Comparative Examples 1 to 3]

Among constructs produced in the above manner, expression vectors were constructed, using a similar way as described in the above Example 1, regarding polypeptide a (comparative example 1) having an amino acid sequence of 474th to 539th amino acid residues of KIAA0849 protein, polypeptide b (comparative example 2) having an amino acid sequence of 454th to 539th amino acid residues, and polypeptide c (comparative example 3) having an amino acid sequence of 454th to 549th amino acid residues. The obtained linear double stranded DNA fragments were used as template DNAs for protein synthesis. At this time, instead of primers used for the first PCR in Example 1, primers of amino acid sequence properly designed for these comparative examples were used.

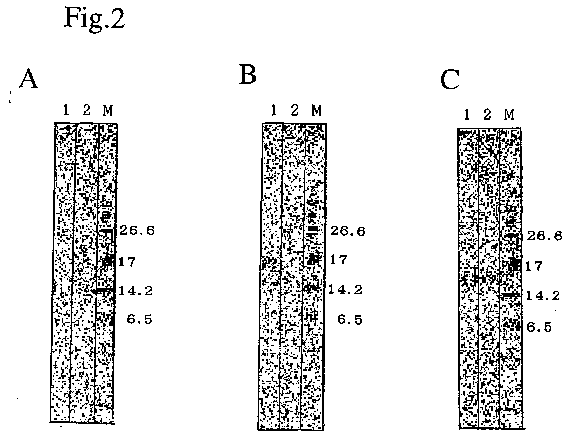

Then, the expression levels of the obtained samples were determined by SDS gel electrophoresis. Fig. 2 shows SDS gel electrophoregrams in comparative examples. Fig. 2A shows an SDS gel electrophoregram of comparative example 1, Fig. 2B shows an SDS gel electrophoregram of comparative example 2, and Fig. 2C shows an SDS gel electrophoregram of comparative example 3. Here, the first lane is a lane of the entire products, the second lane is a lane of a supernatant, and M is a marker lane, respectively.

In Fig. 2A, there appeared no band at a position of MW=11.1 kDa, a molecular weight estimated based on the amino acid sequence of polypeptide a. Therefore, according to Fig. 2A, it was found that a protein of interest was not expressed in comparative example 1.

Further, in Fig. 2B, there appeared no band at a position of 13.1 kDa, which is a molecular weight estimated based on the amino acid sequence of polypeptide b. According to Fig. 2B, it was therefore found that a protein of interest was not expressed in comparative example 2.

Next, in Fig. 2C, a band appeared at an upper position from the position corresponding to 14.2 kDa, which is a molecular weight estimated based on the amino acid sequence of polypeptide c. According to Fig. 2C, it was thus found that a protein of interest was not obtained on a good expression level.

### [Example 5]

With respect to respective proteins of interest in the above Examples 1 to 4 (proteins represented by SEQ ID NOS:1, 3, 5 and 7, respectively) were evaluated in terms of structural stability.

### (1)Purification of ¹⁵N labeled domain

The proteins of interest synthesized in the above Examples 1 to 4 were purified.

To purify ¹⁵N labeled domain protein, the affinity of histidine tag and nickel was utilized. The operation was conducted at 4°C. First, after the termination of synthesis reaction, 3 ml of the reaction solution was diluted with 4.2 ml of washing buffer solution [50 mM sodium phosphate (pH 8.0)/300 mM sodium chloride/10 mM imidazole], collected and centrifuged at 1960 g for 5 minutes to remove precipitates. Next, the obtained supernatant was passed through 0.8 ml of Ni-NTA resin (QIAGEN) for adsorption, and passed through 9.6 ml of washing buffer solution to thereby remove contaminants. Finally, the resultant product was passed through 4 ml of elution buffer solution [50 mM sodium phosphate (pH 8.0)/300 mM sodium chloride/500 mM imidazole], and thereby the sample was liberated from the resin. According to the above procedures, 0.88 mg of purified sample was obtained.

### (2) Sample preparation for structural stability evaluation

To make the purified sample a solvent suitable for NMR measurement, substitution by 20 mM sodium phosphate (pH 6.0)/100 mM sodium chloride solution was conducted. Thereafter, the sample was concentrated to 0.25 ml (sample concentration: 0.28 mM). For the above operations, an ultrafilter (VIVASPIN 2; SARTORIUS) was used. Finally, 0.03 ml of heavy water was added, thereby obtaining a sample for structural stability evaluation.

### (3)Structural stability evaluation by NMR measurement

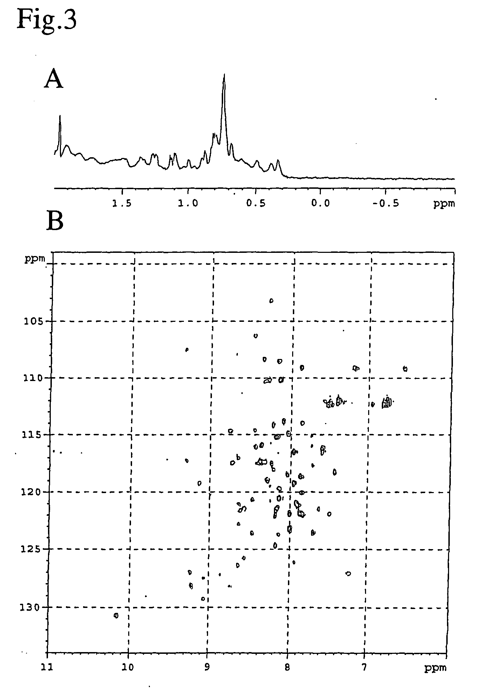

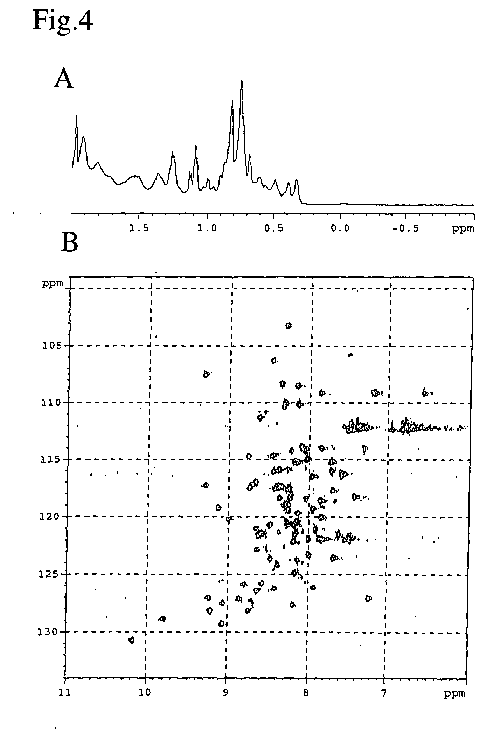

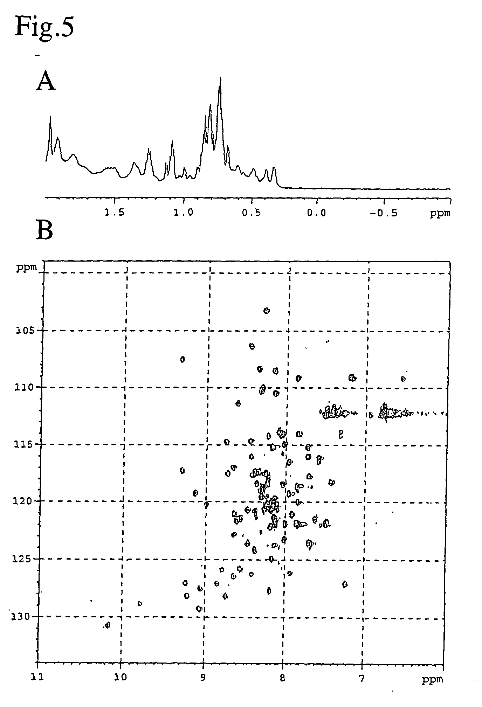

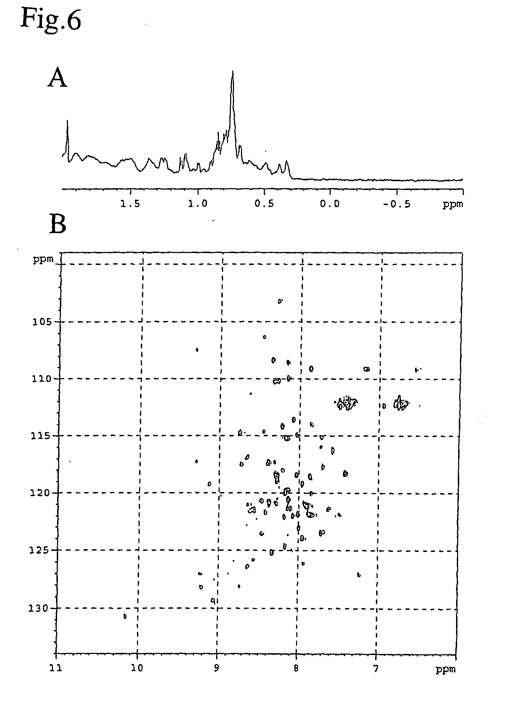

As a sample tube for NMR measurement, a symmetrical microtube (for 5 mm probe) manufactured by Shigemi, Inc. was used. The NMR measurement was conducted by a 600 MHz-NMR instrument (Avance 600 manufactured by Bruker) at 25°C. For the evaluation, one-dimensional spectrum of ¹H (hereinafter abbreviated as 1D spectrum), and ¹H-¹⁵N two-dimensional HSQC spectrum (hereinafter abbreviated as ¹⁵N-HSQC spectrum) were used, and the conditions therefor were shown in the table 6 below. The results of the NMR measurement are shown in the figures 3 to 6.

**[Table 6]**

| NMR measurement conditions for structural stability evaluation | | | | |
|---|---|---|---|---|
| Spectrum | Accumulated times | Center frequency | Spectrum width | Data point number |
| 1D | 128 | ¹H: 2822 Hz | ¹H: 8013 Hz | ¹H: 8192 |
| ¹⁵N | 16 | ¹H: 2822 Hz | ¹H: 8013 Hz | ¹H: 2048 |
| HSQC | | ¹⁵N: 7085 Hz | ¹⁵N: 2190 Hz | ¹⁵N: 128 |

Figs. 3 to 6 show one-dimensional nuclear magnetic resonance spectra and ¹H-¹⁵N HSQC spectra of the proteins represented by SEQ ID NOS:1, 3, 5 and 7, respectively. In Figs. 3 to 6, figure A shows a one-dimensional nuclear magnetic resonance spectrum, and figure B shows a ¹H-¹⁵N HSQC spectrum.

In Figs. 3A to 6A, signals shifted to higher magnetic field were recognized at a higher magnetic field side (around 0.7 ppm to -0.5 ppm) in the methyl region of one-dimensional nuclear magnetic resonance spectra. In Figs. 3B to 6B, signals of removed amide proton were recognized at a range of 7 ppm to 9 ppm in ¹⁵N HSQC spectra.

Since the appearance of these signals is characteristic to a protein forming a stable three-dimensional structure, it was determined that the proteins obtained in the above examples 1 to 4 formed a stable three-dimensional structure.

In the examples 1 to 4, as mentioned above, amino acid sequences of constituent element (domain) having structure and function of a protein were predicted from full-length KIAA0849 protein by a computer, and all constructs of various kinds were produced on the basis of the predicted region for protein expression. The expression statuses of the actually obtained proteins were confirmed by SDS gel electrophoresis. Then, whether or not they have a stable three-dimensional structure was evaluated by NMR measurement, and all the proteins were confirmed to have excellent structural stability.

Therefore, according to the examples, it has been confirmed that an amino acid sequence of CAP-Gly-like domain having actually stable three-dimensional structure (folding) was correctly determined.

Moreover, use of such domain-forming protein having a small molecular weight enables highly accurate three-dimensional structure analysis with relative ease.

Then, using KIAA0849 protein (464-554) of Example 1 that is a CAP-Gly-like domain protein having an amino acid sequence represented by SEQ ID NO:1, the three-dimensional structure analysis was conducted as follows.

### [Example 6]

### Structure determination of CAP-Gly-like domain protein contained in cancer suppressor gene (KIAA0849) product involved in human Turban tumor syndrome

### (1) Purification of ¹³C¹⁵N-labeled domain by NMR measurement

A protein represented by SEQ ID NO:20 having all the carbon and nitrogen nuclears substituted by stable isotope carbon 13 and nitrogen 15 was produced by the above cell-free protein expression system. The protein represented by SEQ ID NO:20 is a protein derived from the protein represented by SEQ ID NO:1 by addition of amino acid residues represented by GSSGSSG and SGPSSG to the N- and C-terminals, and these additional sequences do not affect three-dimensional structure of the protein. Therefore, analysis of three-dimensional structure of the protein represented by SEQ ID NO:20 provides three-dimensional structure of the protein of the present invention represented by SEQ ID NO:1.

The obtained high purity preparation was concentrated to a standard concentration of 0.8 mM using a protein concentrator with an ultrafilter membrane for a high-speed centrifuge, and thereafter diluted 10 times with preparation buffer solution for NMR analysis. These concentration and dilution processes were repeated three times, and the buffer solution for purifying preparation was completely substituted by the preparation buffer solution for NMR analysis. The used preparation buffer solution for NMR analysis was composed of 20 mM sodium phosphate, 100 mM sodium chloride, 1 mM dithiothreitol, and pH thereof was 6.0. After complete substitution by the preparation buffer solution for NMR analysis, the final concentration of the preparation was about 0.8 mM. The obtained preparation was injected to a test tube for NMR measurement with an outer diameter of 5 mm, and then preserved at 25°C for 2 hours for stabilization.

### (2) NMR measurement

For NMR experiment, DRX600 and DRX800 manufactured by Swiss Bruker were used. All the measurements were conducted at 25° C. In NMR experiment having a purpose of main chain signal attribution, a two dimensional spectrum of ¹H-¹⁵N HSQC, and three dimensional spectrum of HNCO, HN(CO)CA, HNCA, CBCA(CO)NH, HNCACB, HBHA(CBCACO)NH, H(CCCO)NH, C(CCCO)NH, and ¹⁵N-edited NOESY were measured. In addition, in NMR experiment having a purpose of side chain signal attribution, two dimensional spectrum of ¹H-¹³C HSQC and three dimensional spectra: HCCH-COSY, HCCH-TOCSY, ¹³C-edited NOESY for aliphatic side chain; and HCCH-COSY, ¹³C-edited NOESY, HNHB, and HN(CO)HB for aromatic side chain were measured.

### (3) Analysis of measurement data

The measurement data were subjected to Fourier transform using work stations Octane2 and Origin3800 manufactured by Silicon Graphics, Inc. in America, and respective two dimensional and three dimensional spectra were obtained. Based on the obtained spectrum data, Cα and Cβ of carbon nuclear at α and β positions as main chain signals of amino acid residue; C' of carbon nuclear of carbonyl group; Hα and Hβ of hydrogen nuclear at α and β positions; HN of hydrogen nuclear of amino group; and N of nitrogen nuclear of amide group were attributed in a chain reaction manner. In this method, a signal having a chemical shift value identical to that of Cα signal of an adjacent residue on HN(CO)Ca was searched for on HNCA, and the linkage with Cα signal of a residue adjacent to itself was clarified. This process was repeated, and thereby all Cα signals were attributable in a chain reaction manner, except that signals were not observed due to proline residue or from any cause. By conducting the same procedure, Cβ signals by C(CCCO)NH. CBCA(CO)NH, and HNCACB, Hα and Hβ signals by H(CCCO)NH, HBHA(CBCACO)NH and ¹⁵N-edited NOESY, and C' signals by HNCO were attributed, and thereby more precise attribution can be conducted. Further, using the obtained spectrum data measured for main chain attribution information and side chain attribution, attributions of carbon, nitrogen, and hydrogen nuclears at γ, δ, ε, ζ, and η positions were conducted. According to the above procedures, attribution data of signals regarding to almost all the amino acid residues was obtained. Moreover, distance limitation data was obtained from 1142 signals on ¹⁵N-edited NOESY, 2158 signals on ¹³C-edited NOESY for aliphatic side chain, and 209 signals on ¹³C-edited NOESY for aromatic side chain. From the chemical shift values, obtained during main chain attribution, of Cα, Cβ, C', Hα, Hβ, HN, and N signals, φ and ψ angle data of 42 residues were obtained using a software TALOS, which predicted with high precision φ and ψ angles, dihedral angles of polypeptide main chain. Furthermore, according to signal patterns of HNHB and HN(CO)HB, data of χ angle, which was a dihedral angle of side chain in 35 residues was obtained. Based on these signal attribution data, distance limitation data, φ, ψ, and χ angle limit data, a domain structure was calculated using CNS, a software for protein three-dimensional structure calculation. Based on the obtained three-dimensional structure, NOE group which did not meet provided distance limit was compared and reviewed, and then optimized. This process was repeated, and finally calculation was conducted using all the angle limits and 2667 distance limits, thereby obtaining 20 energetically stable three-dimensional structures. In these structures, the convergence of amino acid residues forming two dimensional structure was 0.29 Å relative to atom group of main chains and 0.76 Å relative to all atom group including side chains except hydrogen atom.

### (4) Structure coordinates

Structure coordinates are shown in the following three-dimensional structure coordinate tables 1 to 20.

The following three-dimensional structure coordinate data is described in accordance with the format of protein data bank (PDB). ATOM of the first column indicates that this column is a column for atomic coordinate; the second column indicates the order of atoms; the third column indicates the distinction of atom in amino acid residues, etc.; the fourth column indicates amino acid residues, etc.; the fifth column indicates the number of amino acid corresponding SEQ ID NO:20; the sixth, seventh, and eighth columns indicate coordinates of atom (unit: Å, in the order of a, b and c axes); the ninth column indicates occupancy of that atom (anytime this figure is 1.00 in the invention); the tenth column indicates temperature factor of that atom. The final line indicates the final line of this table.

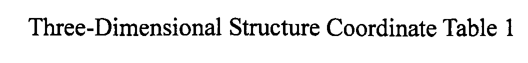

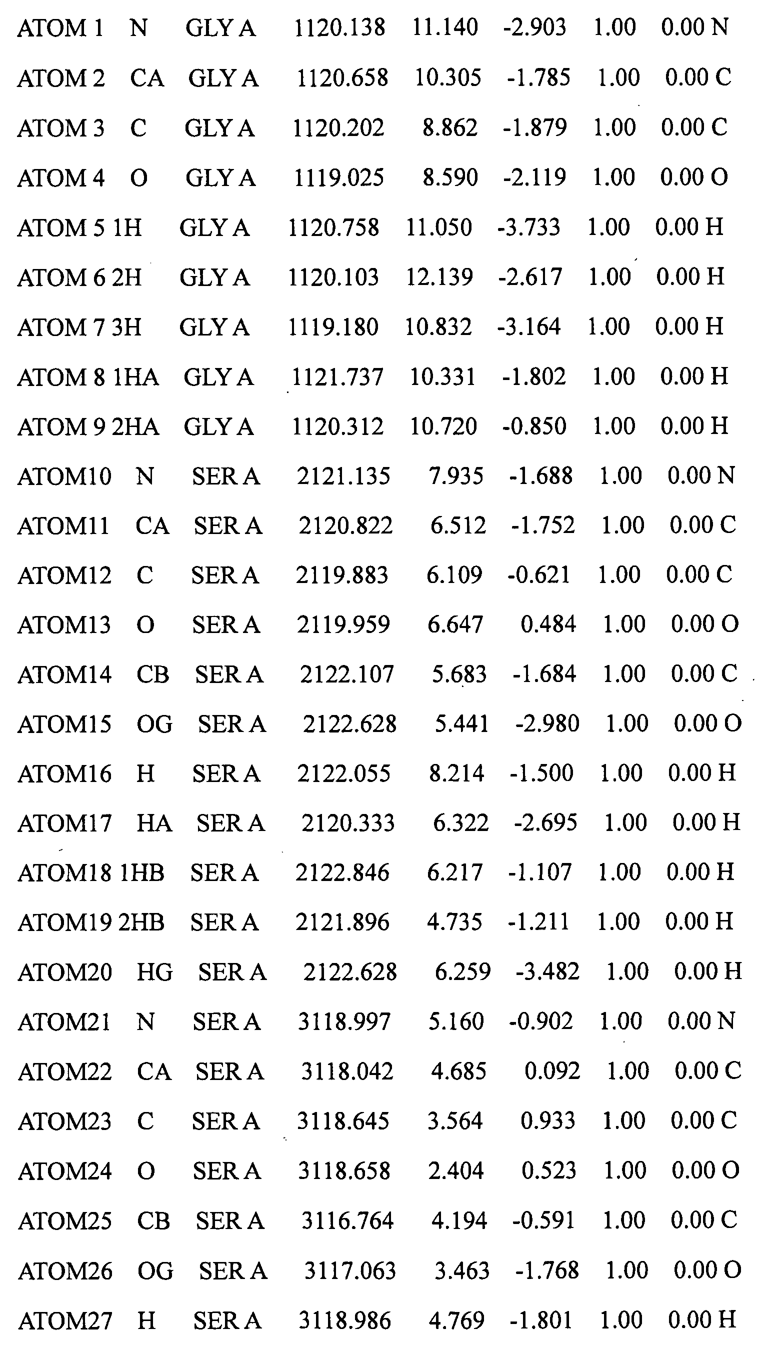

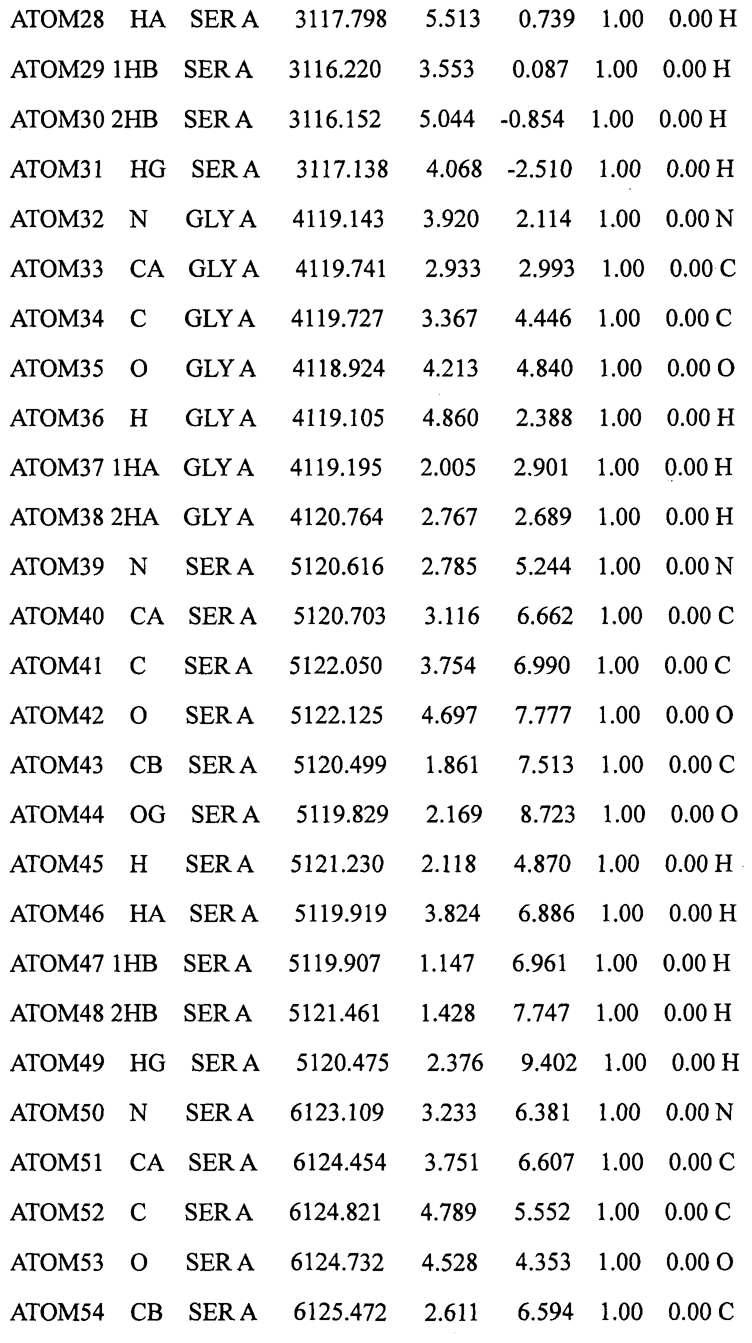

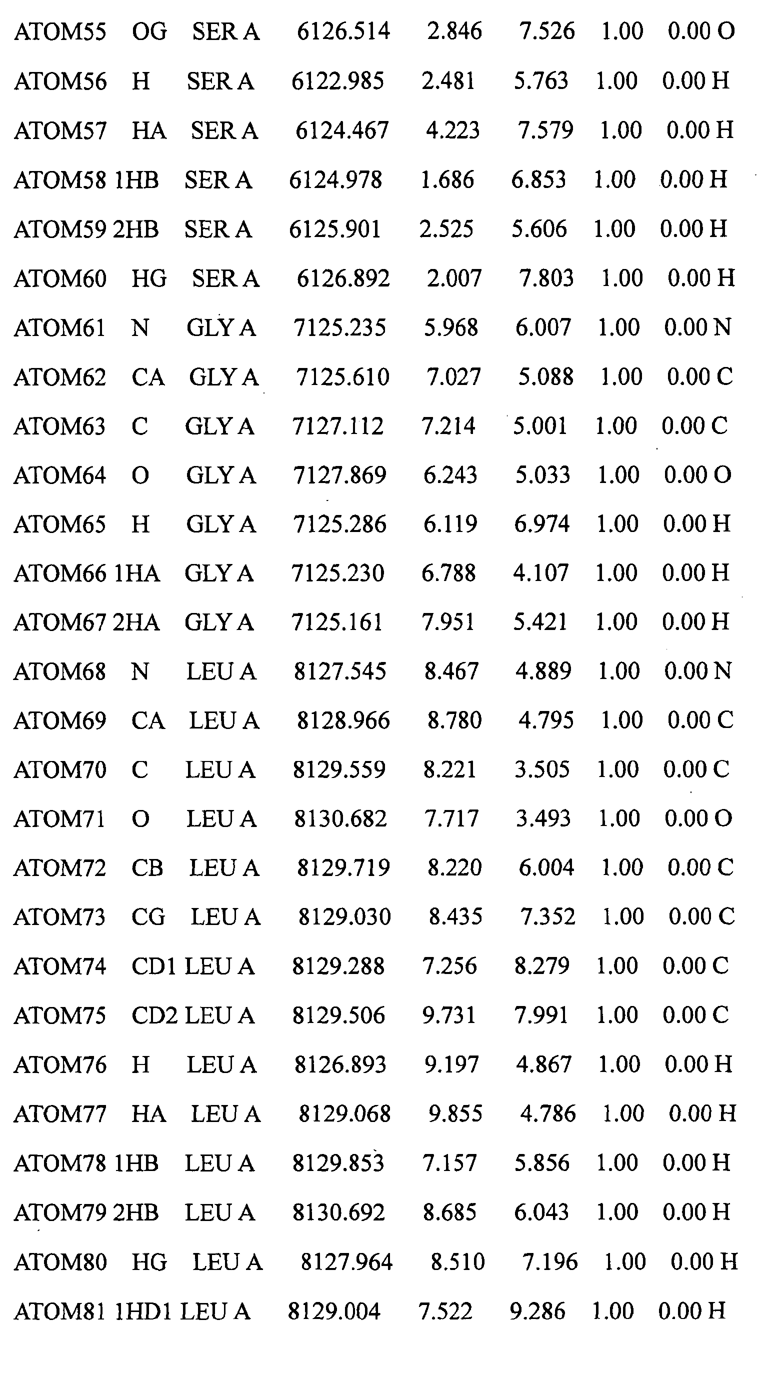

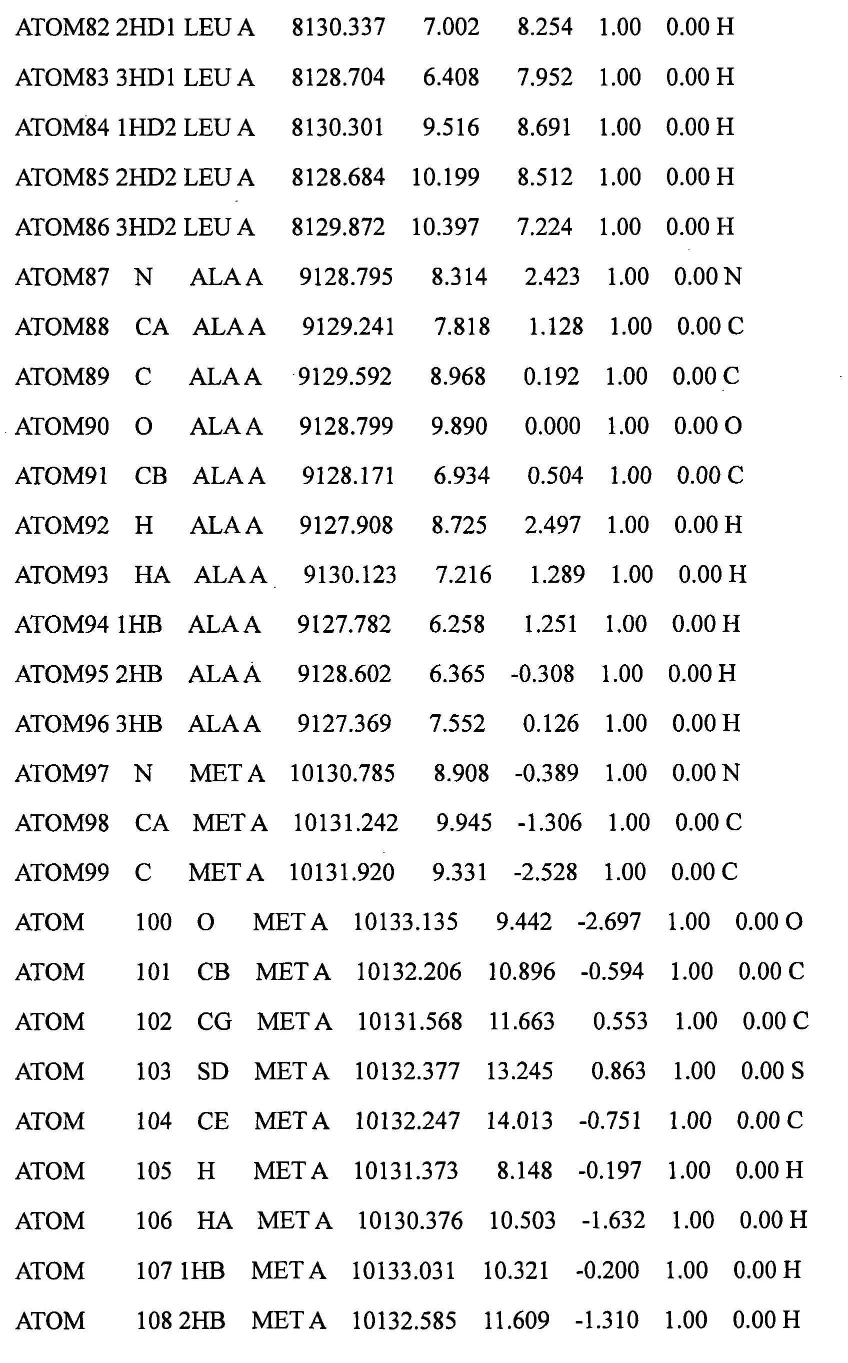

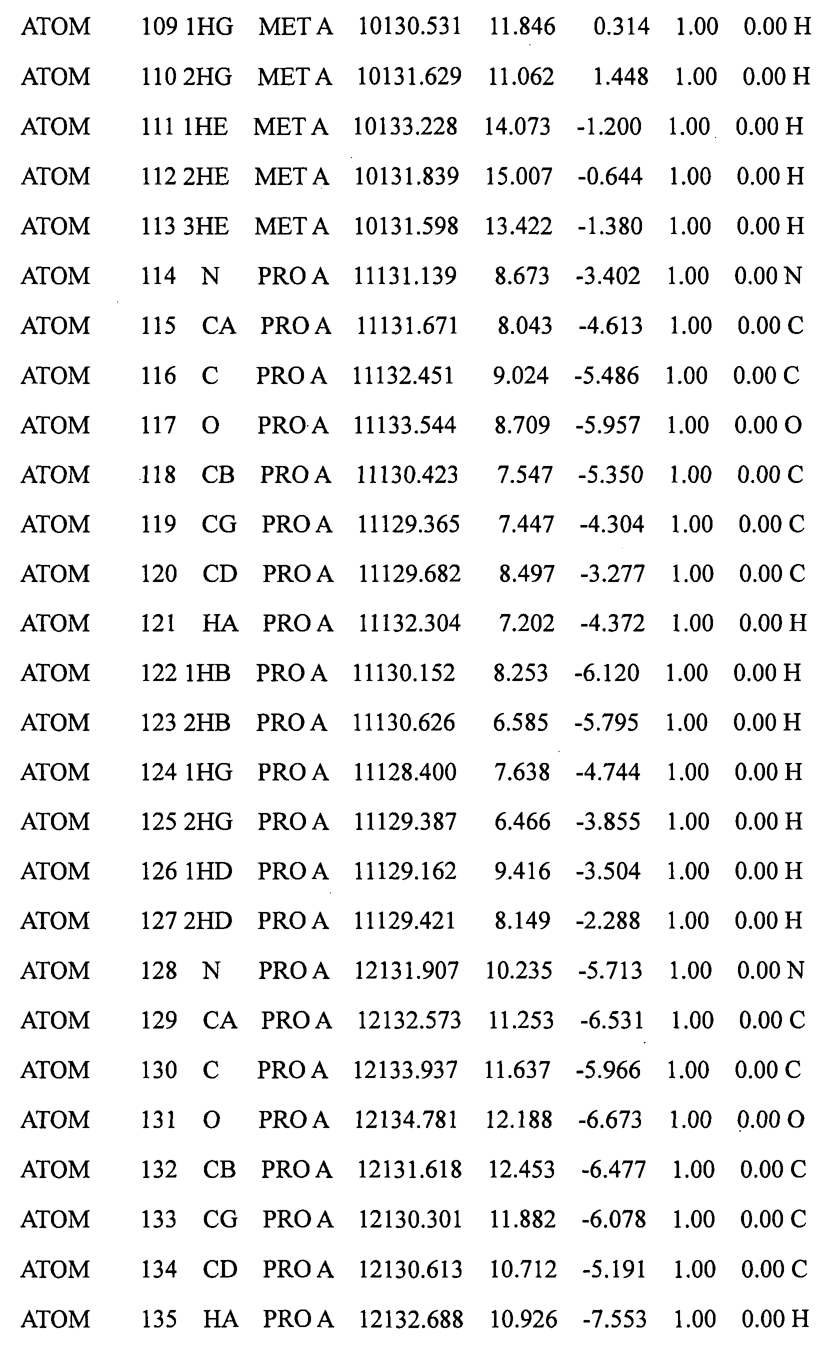

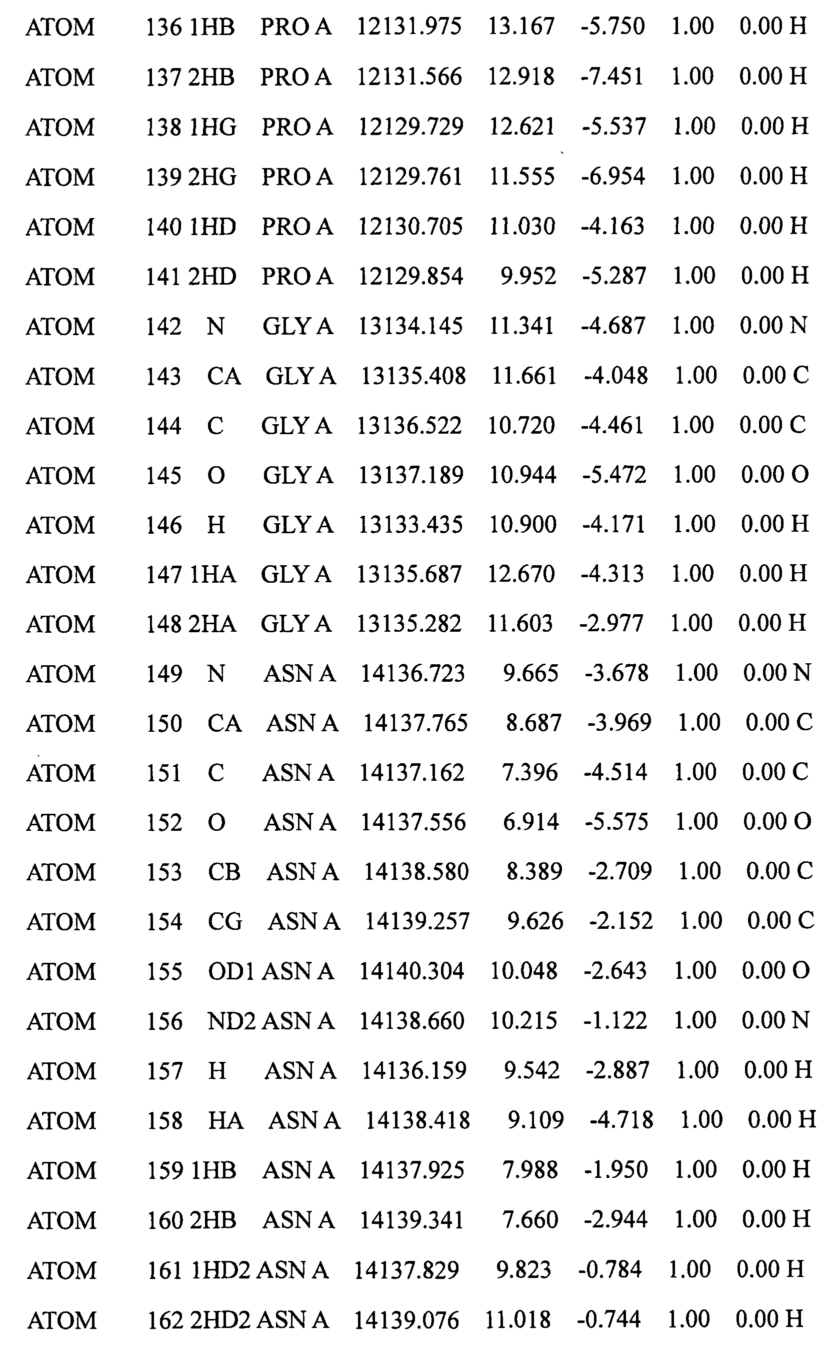

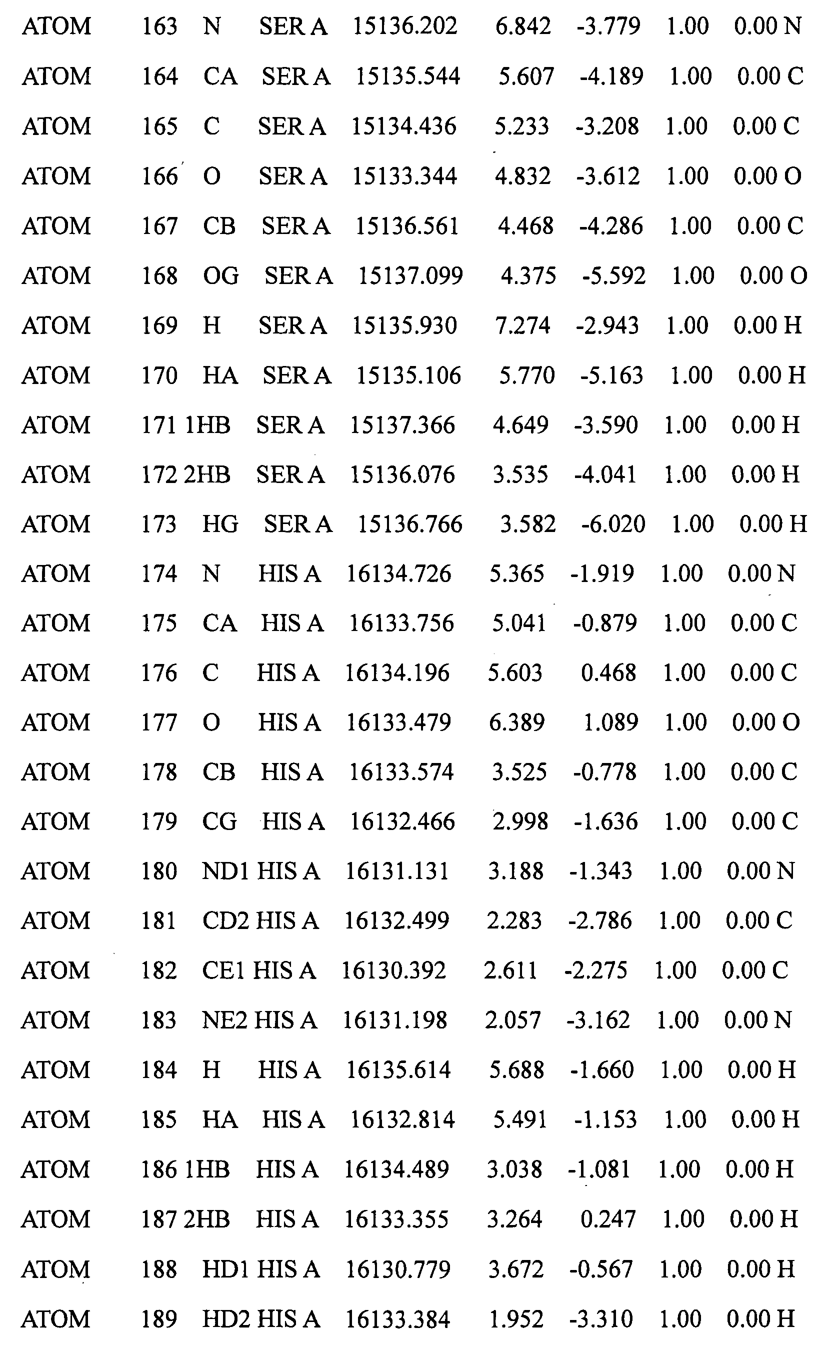

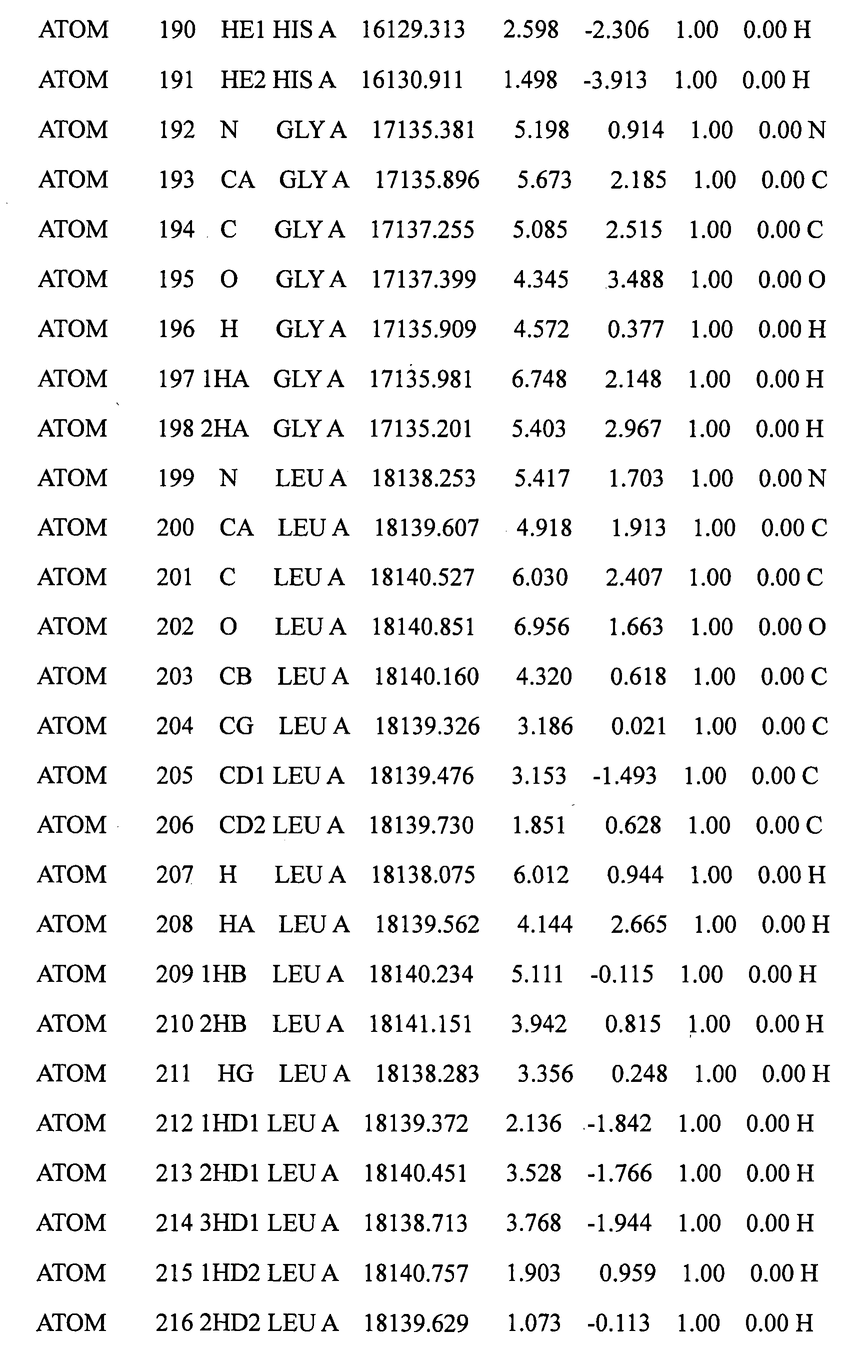

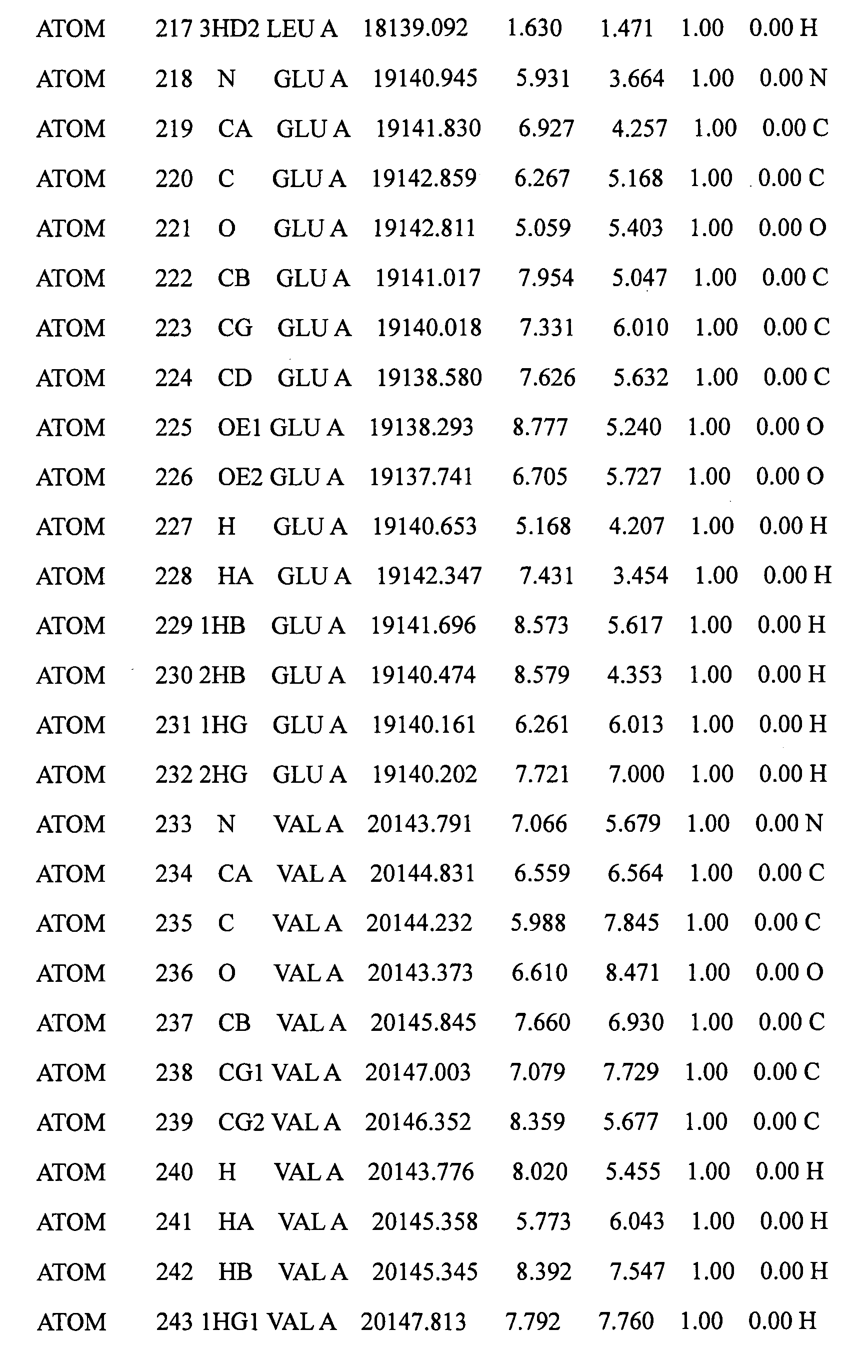

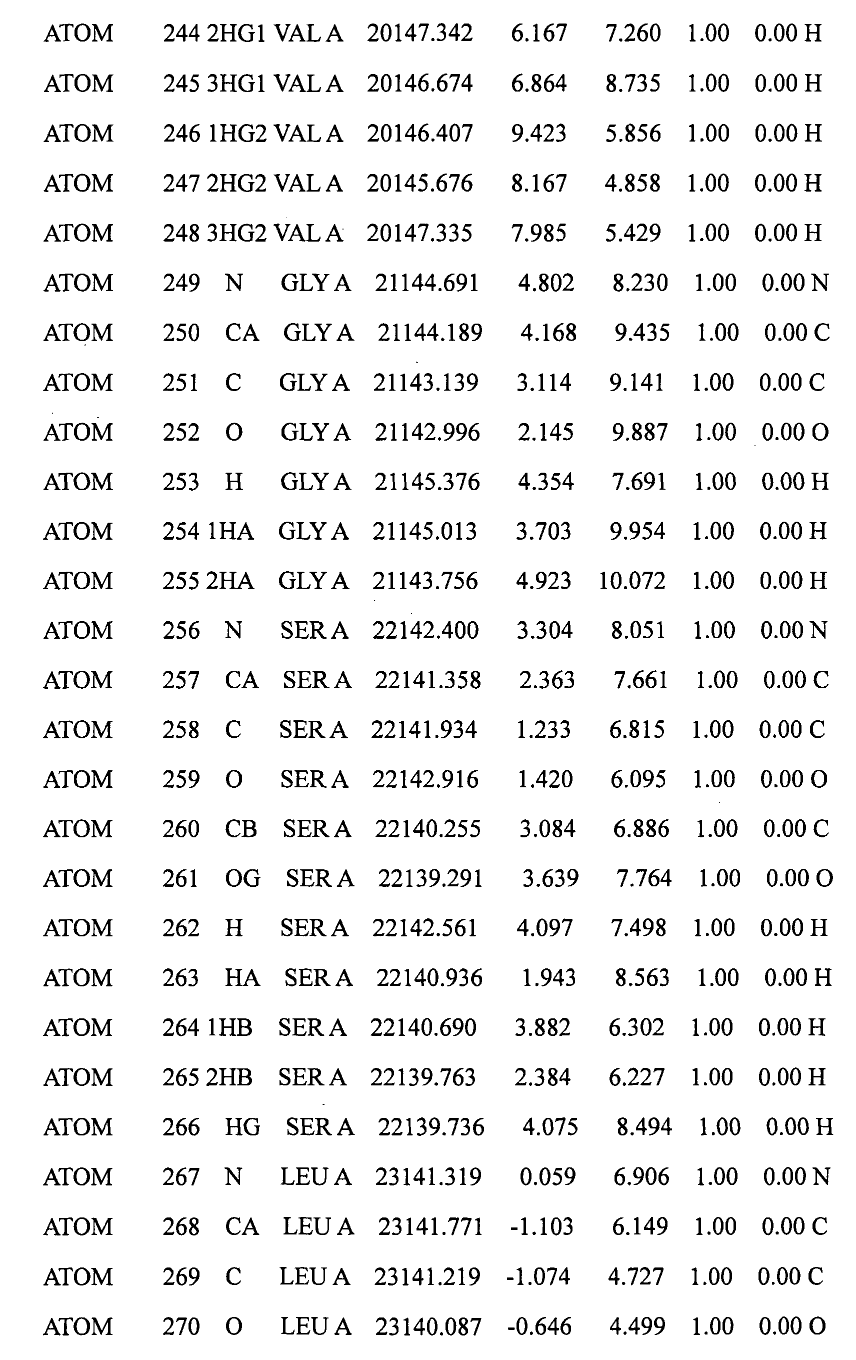

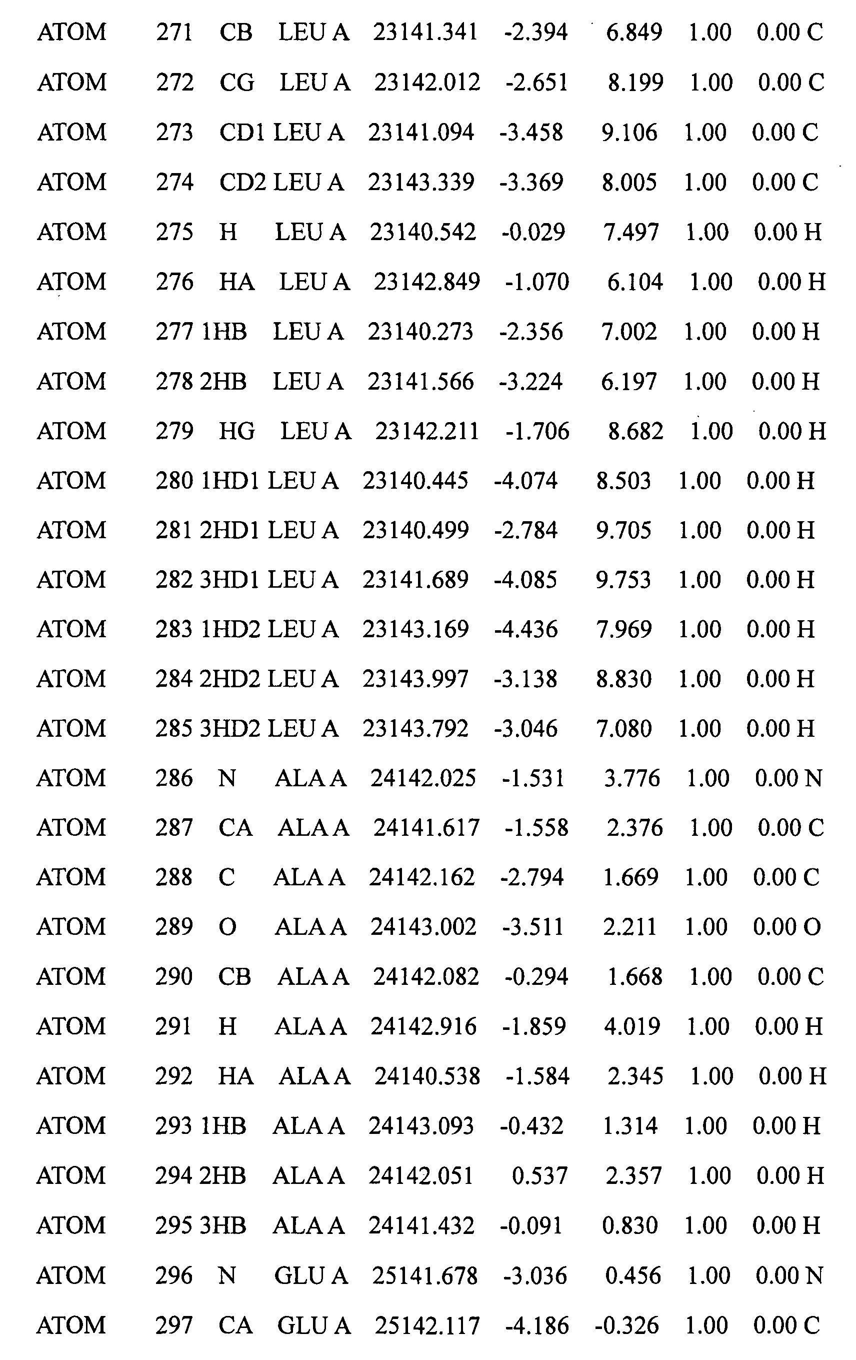

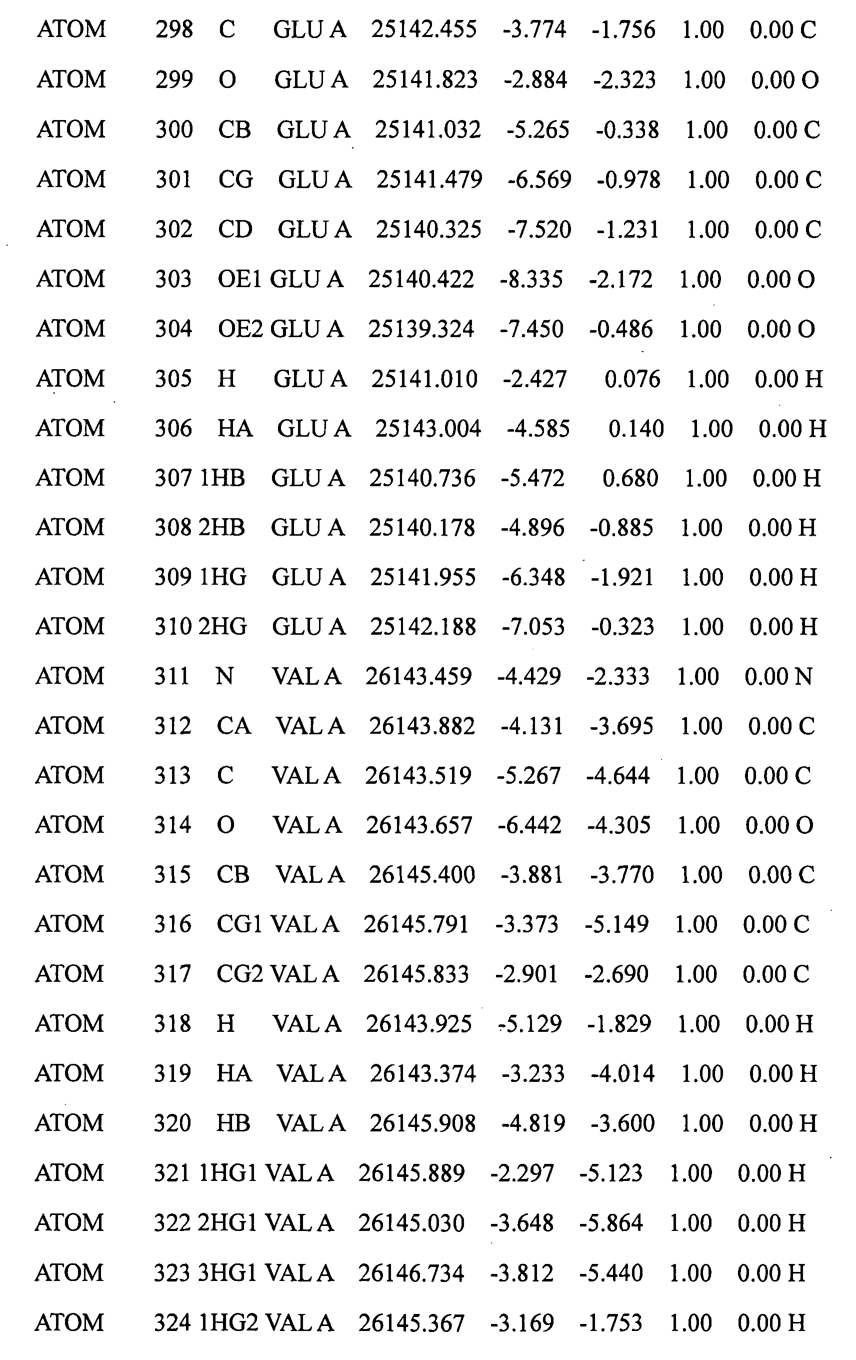

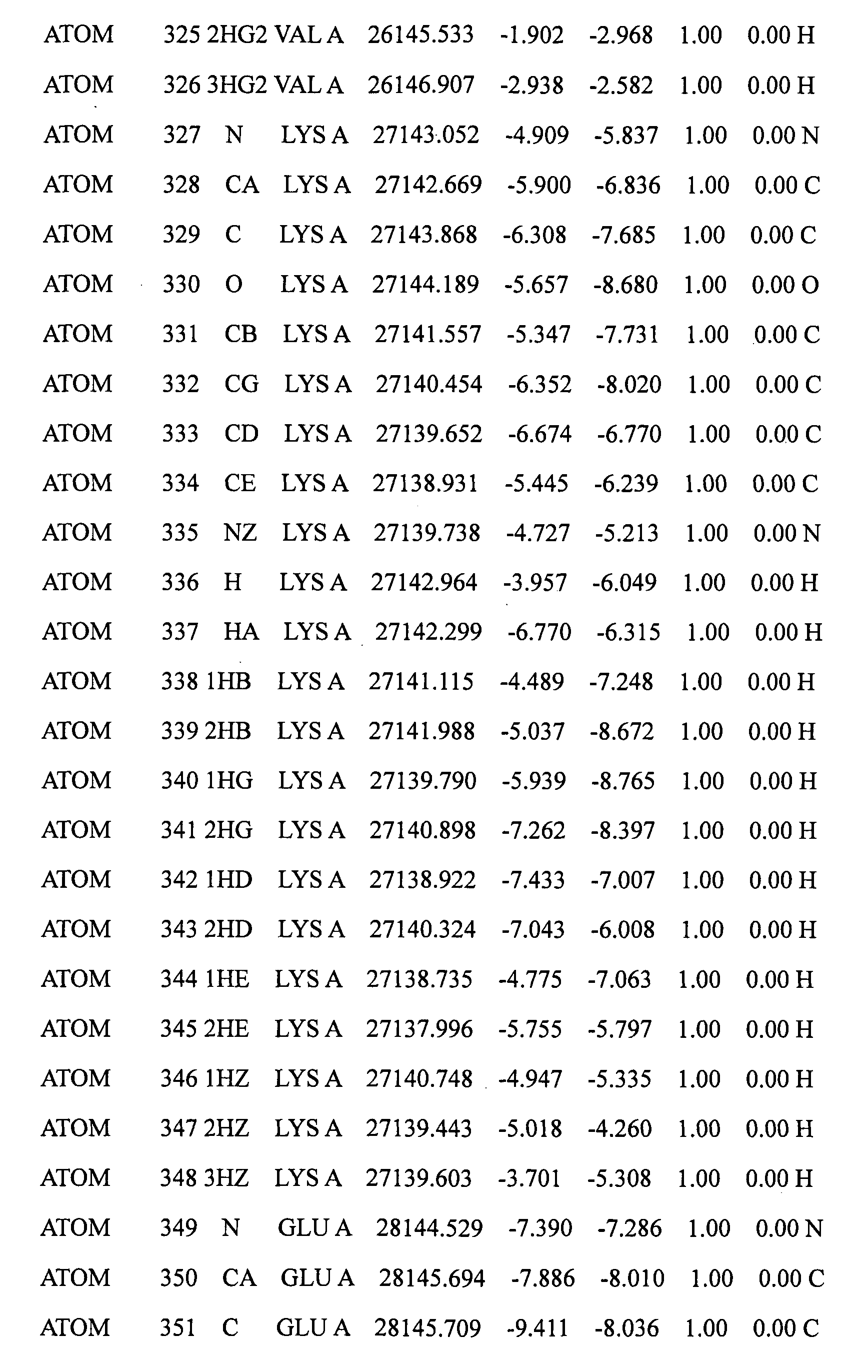

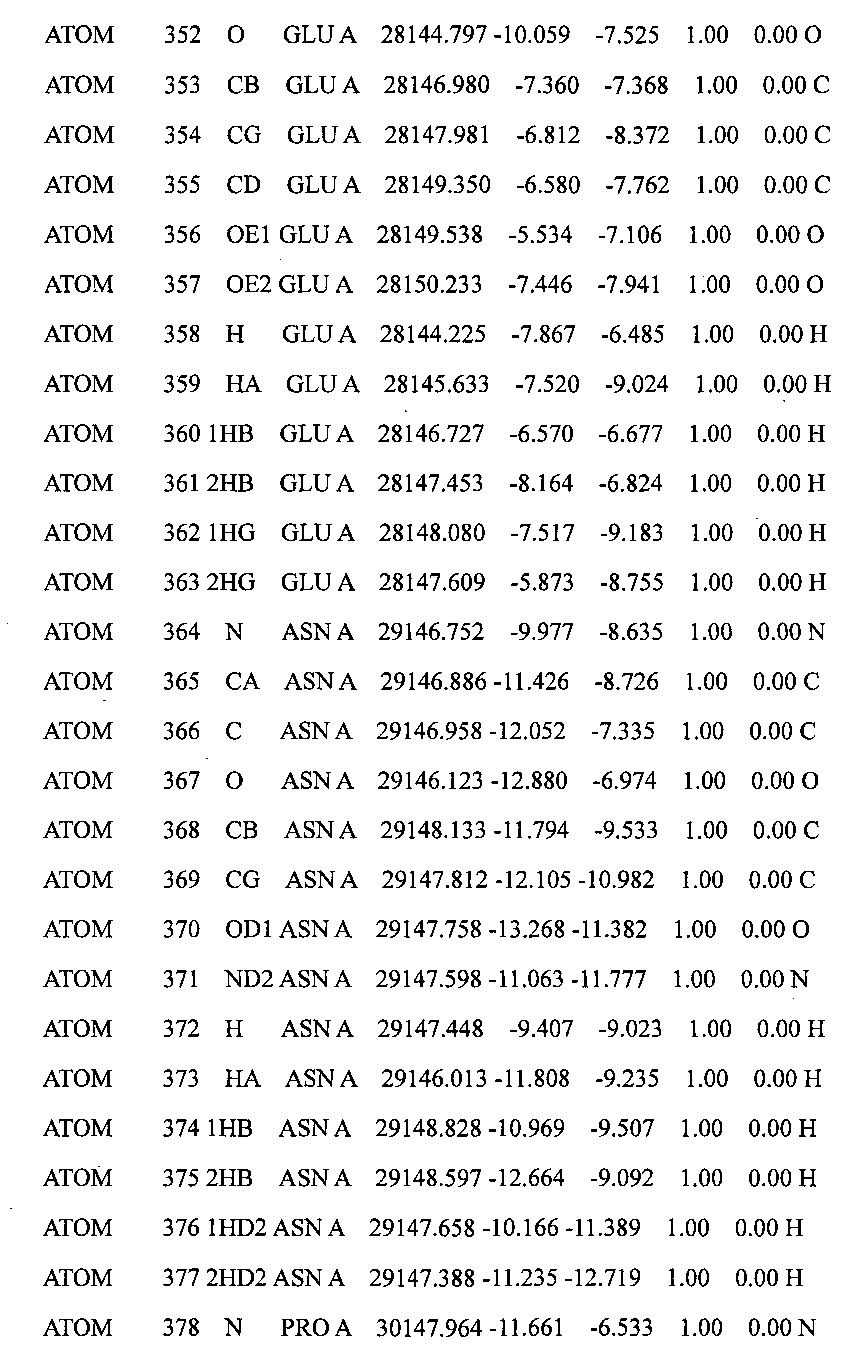

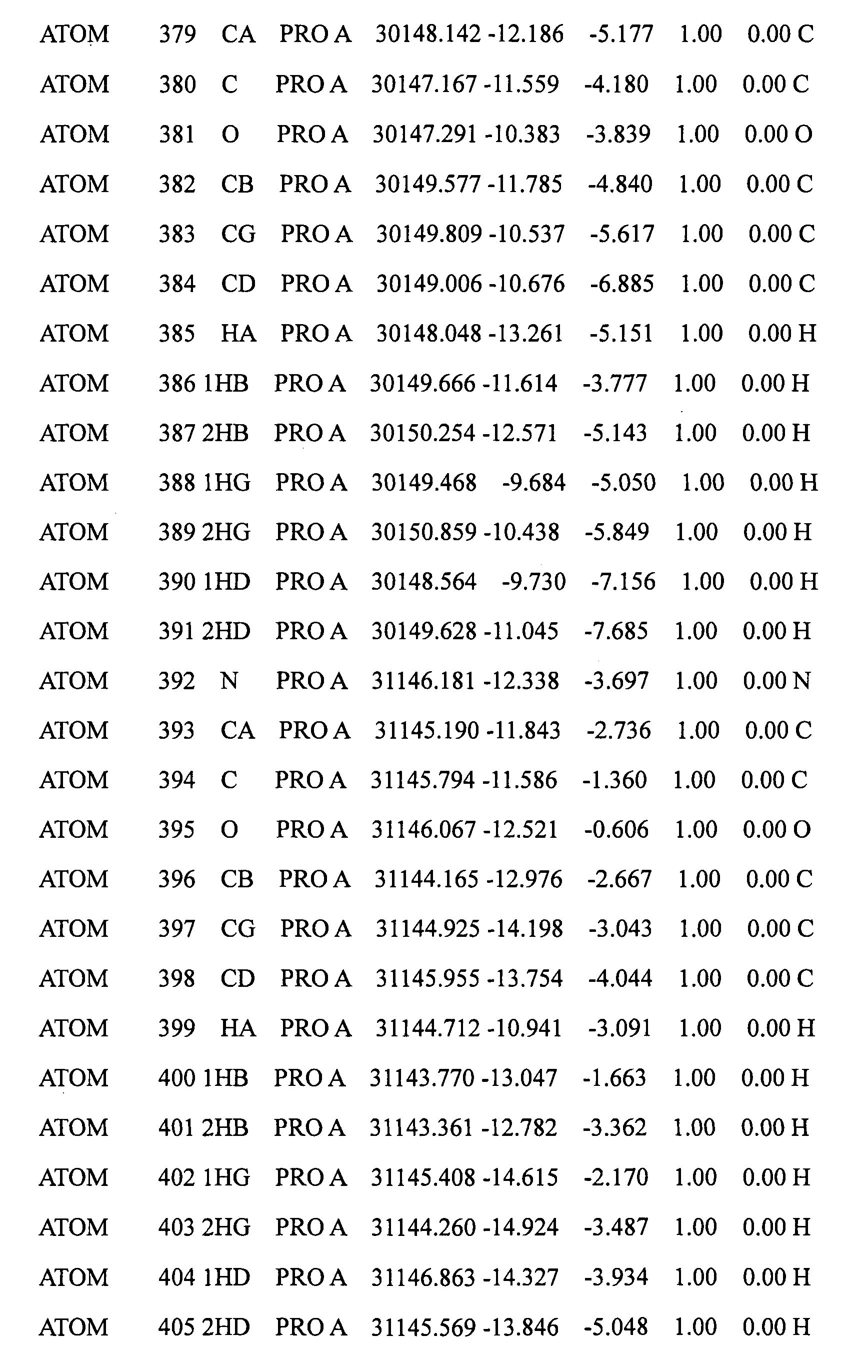

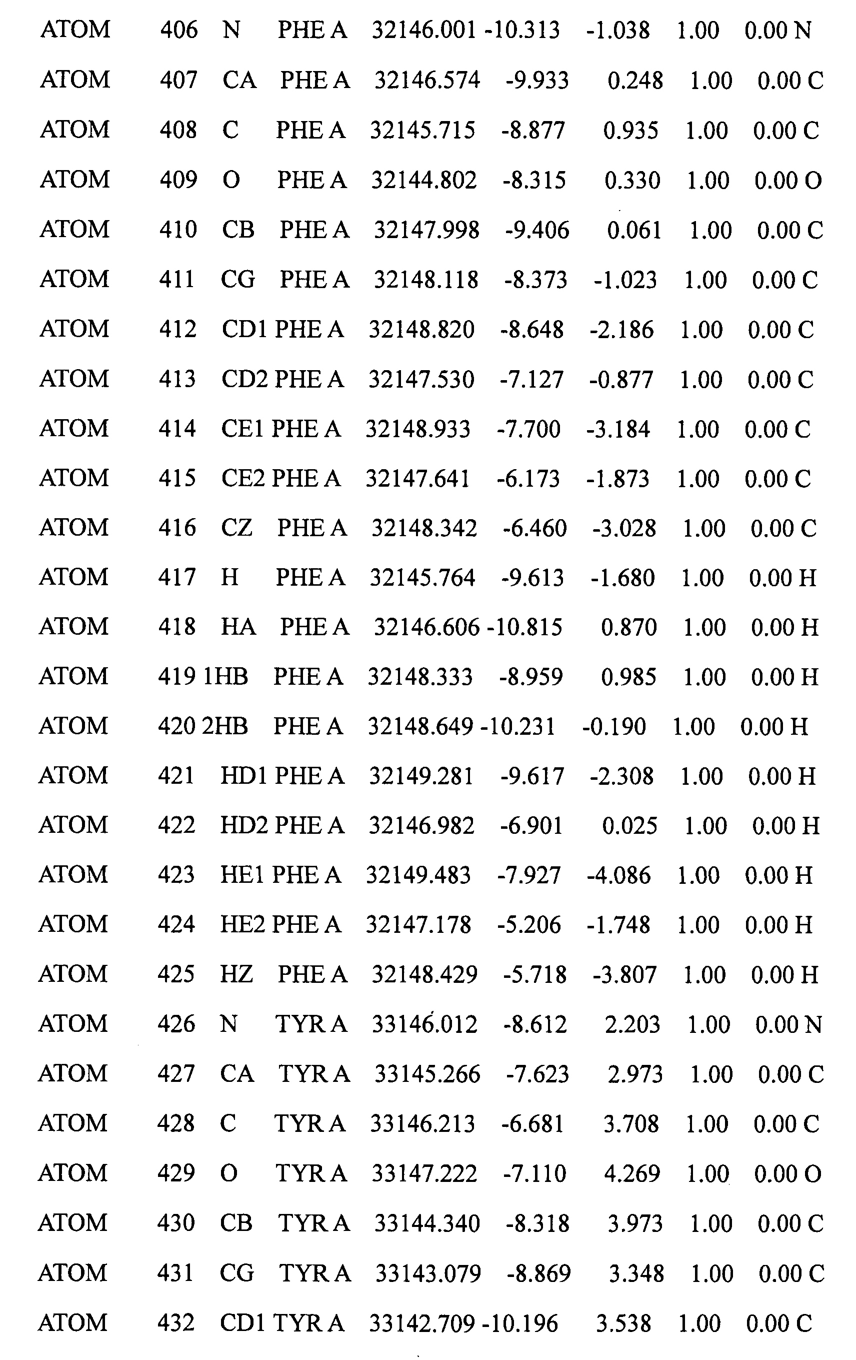

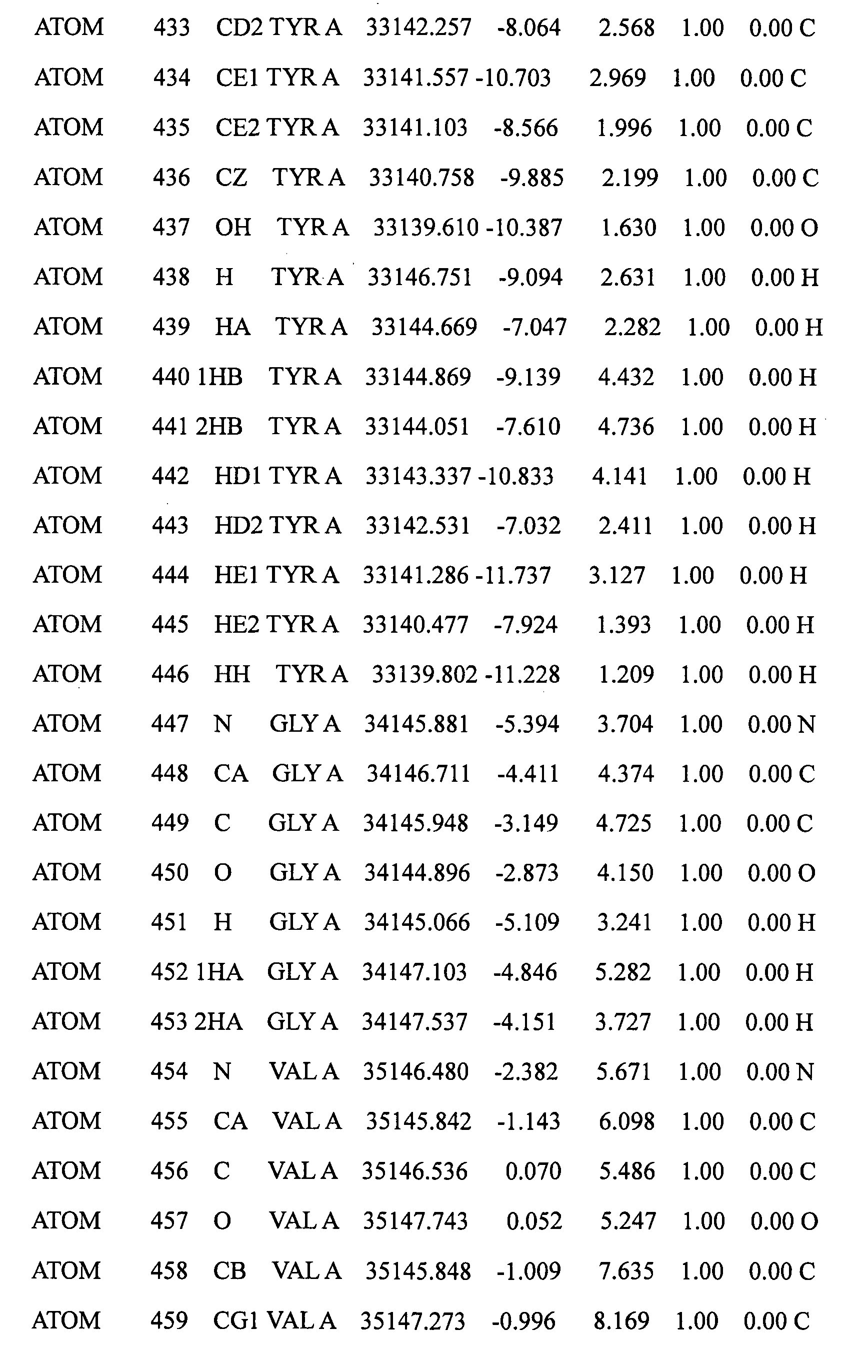

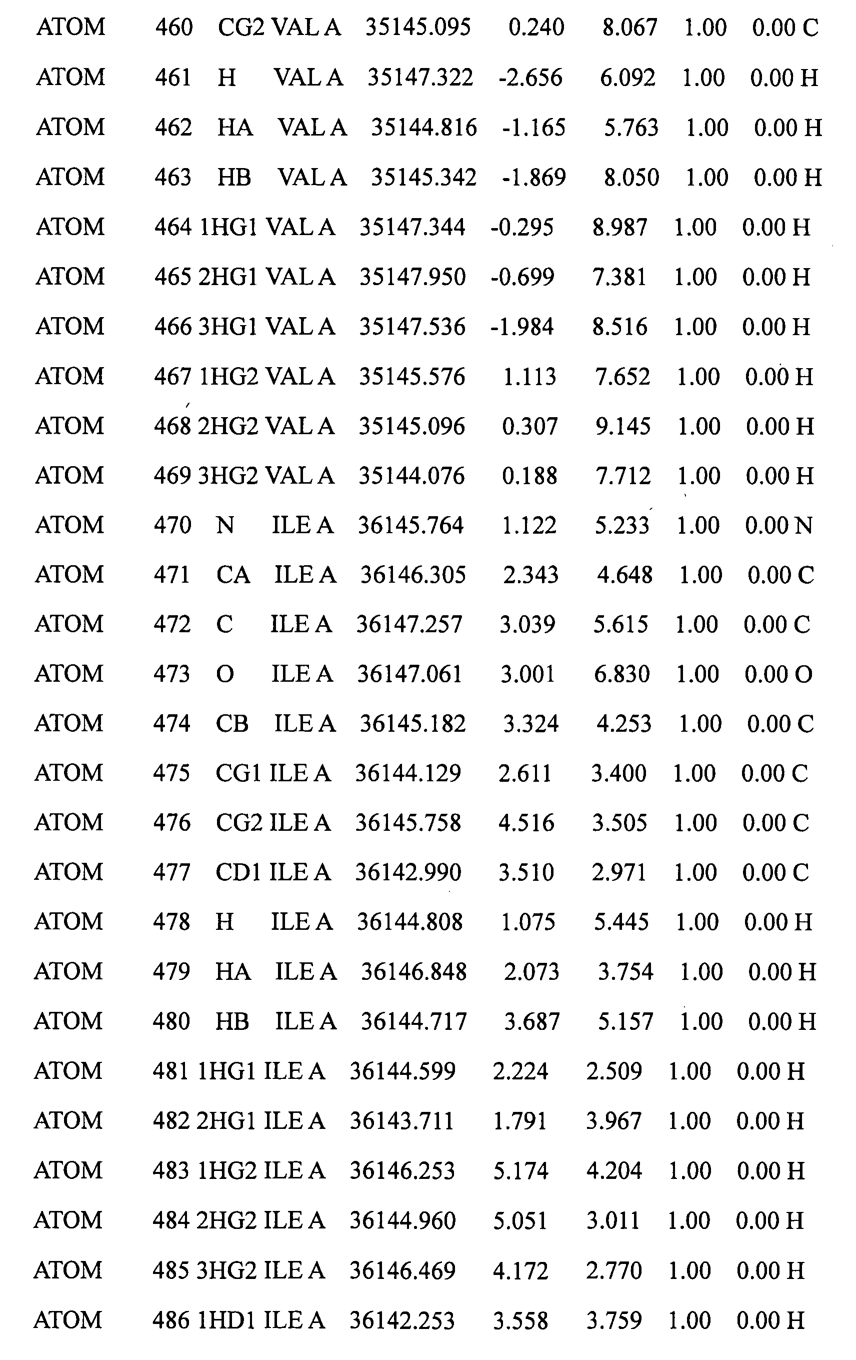

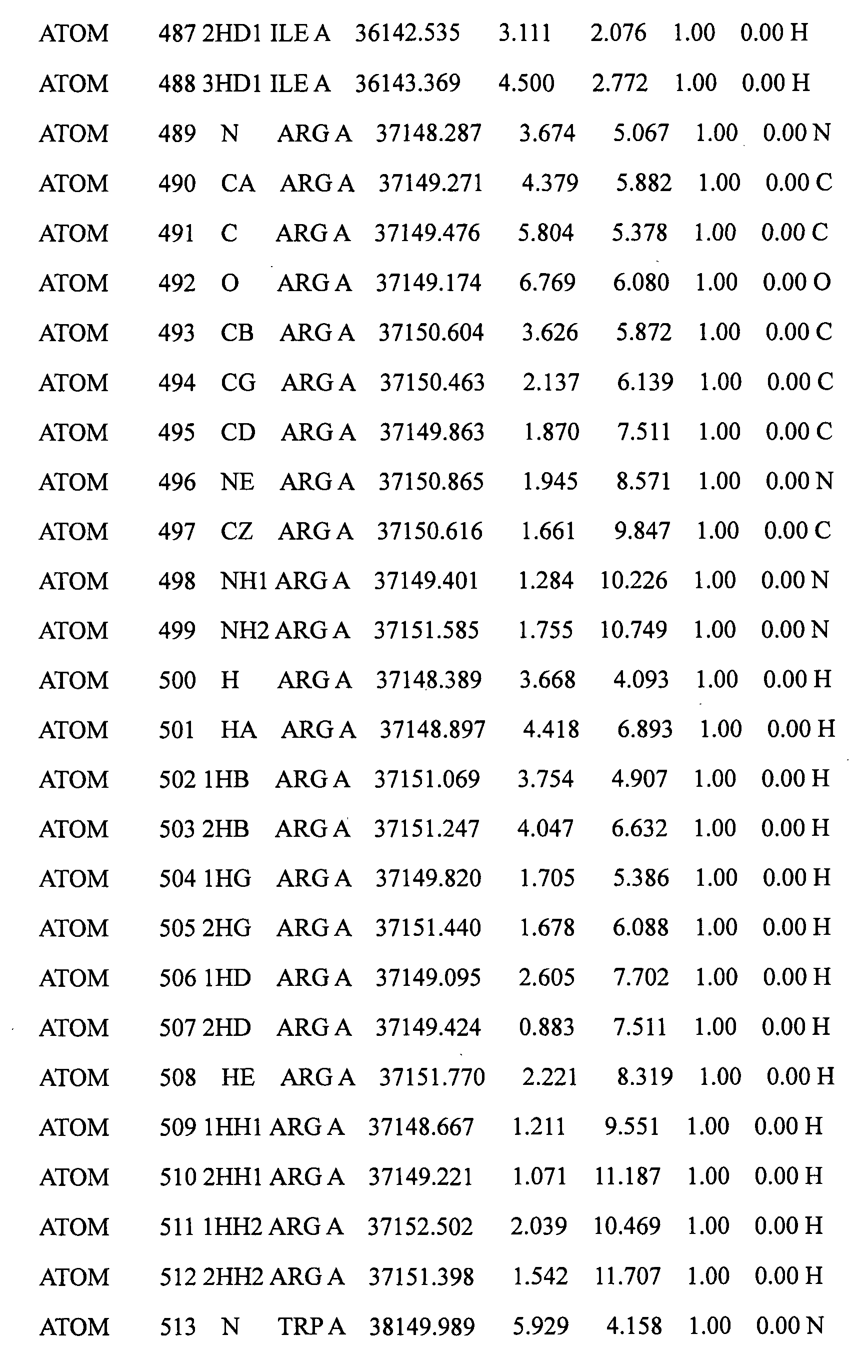

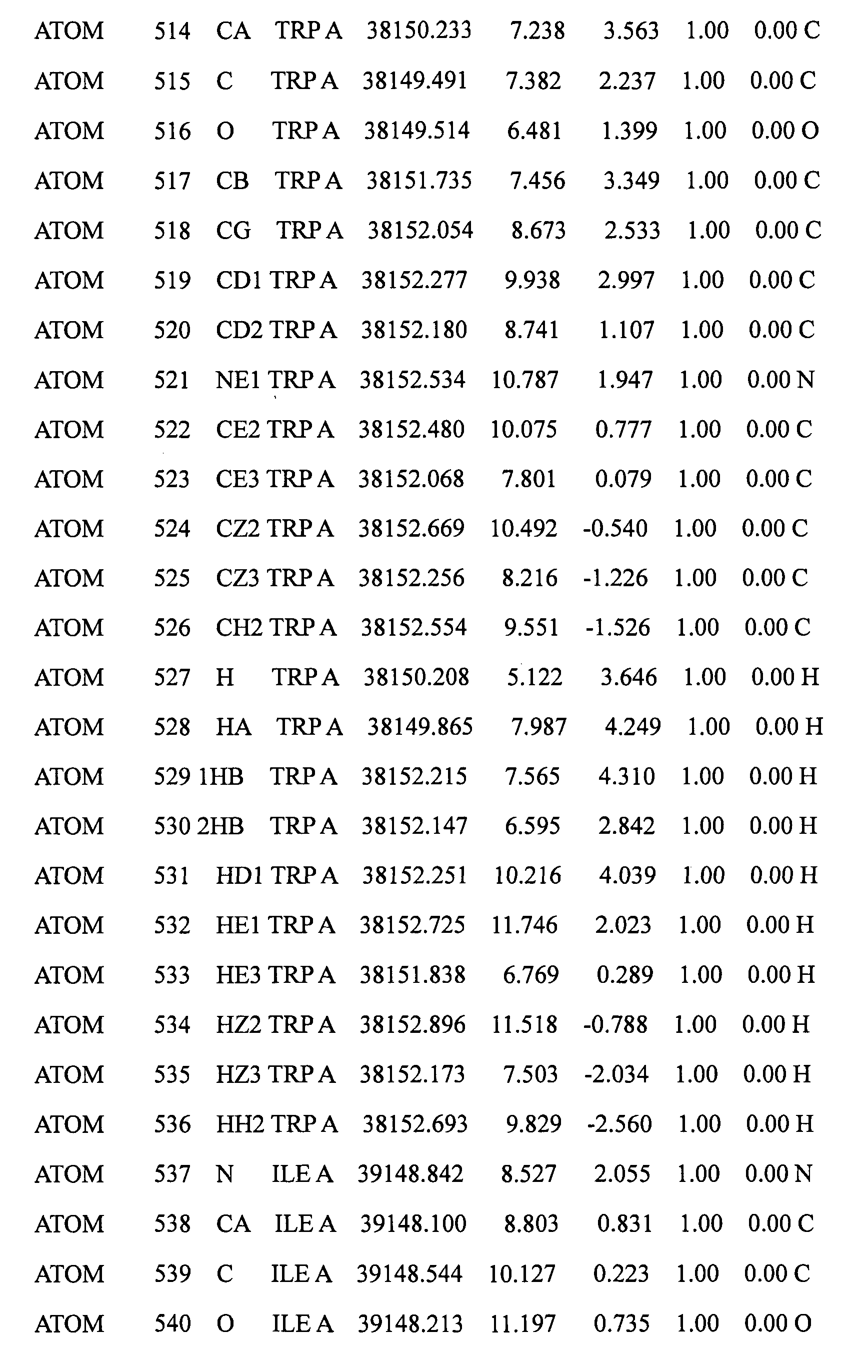

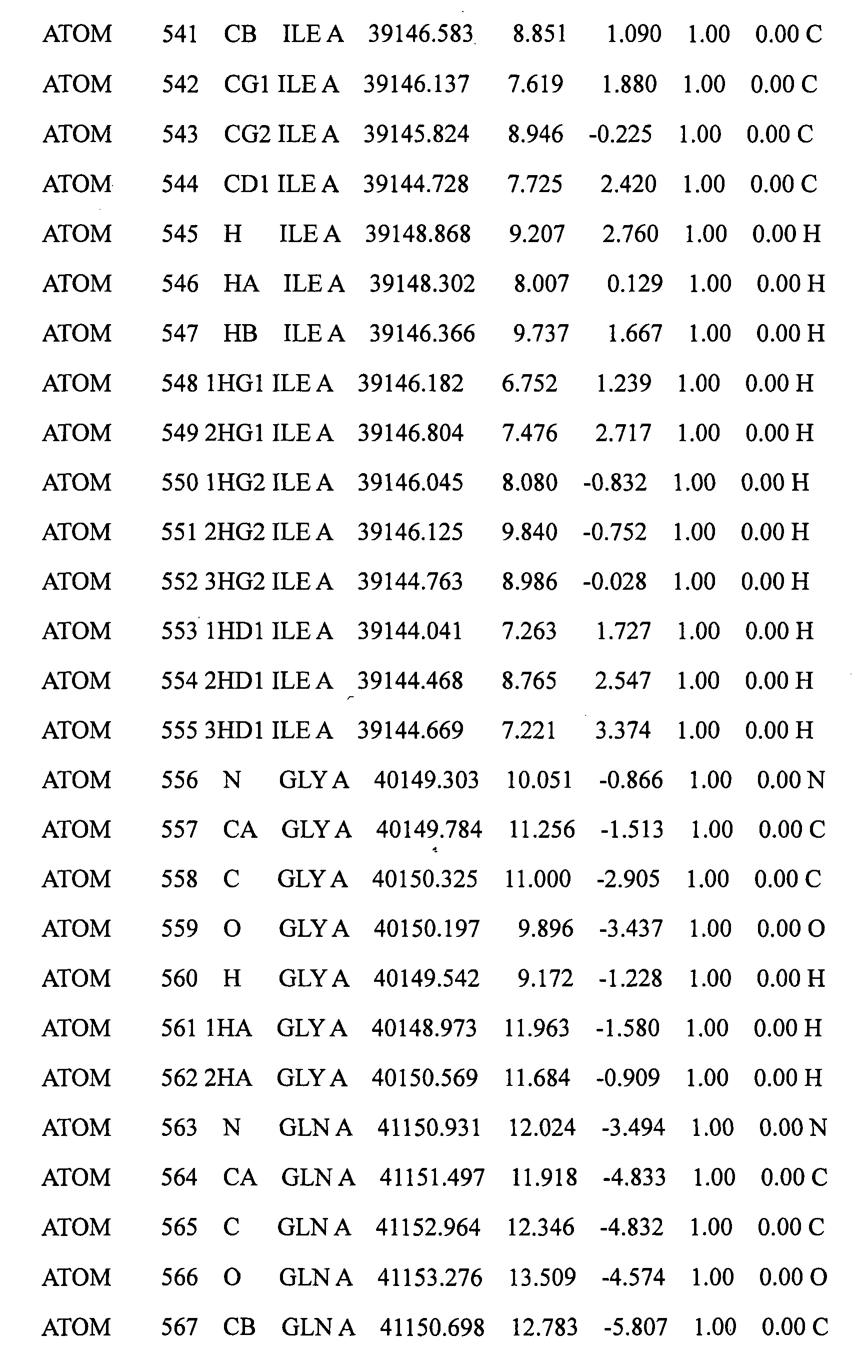

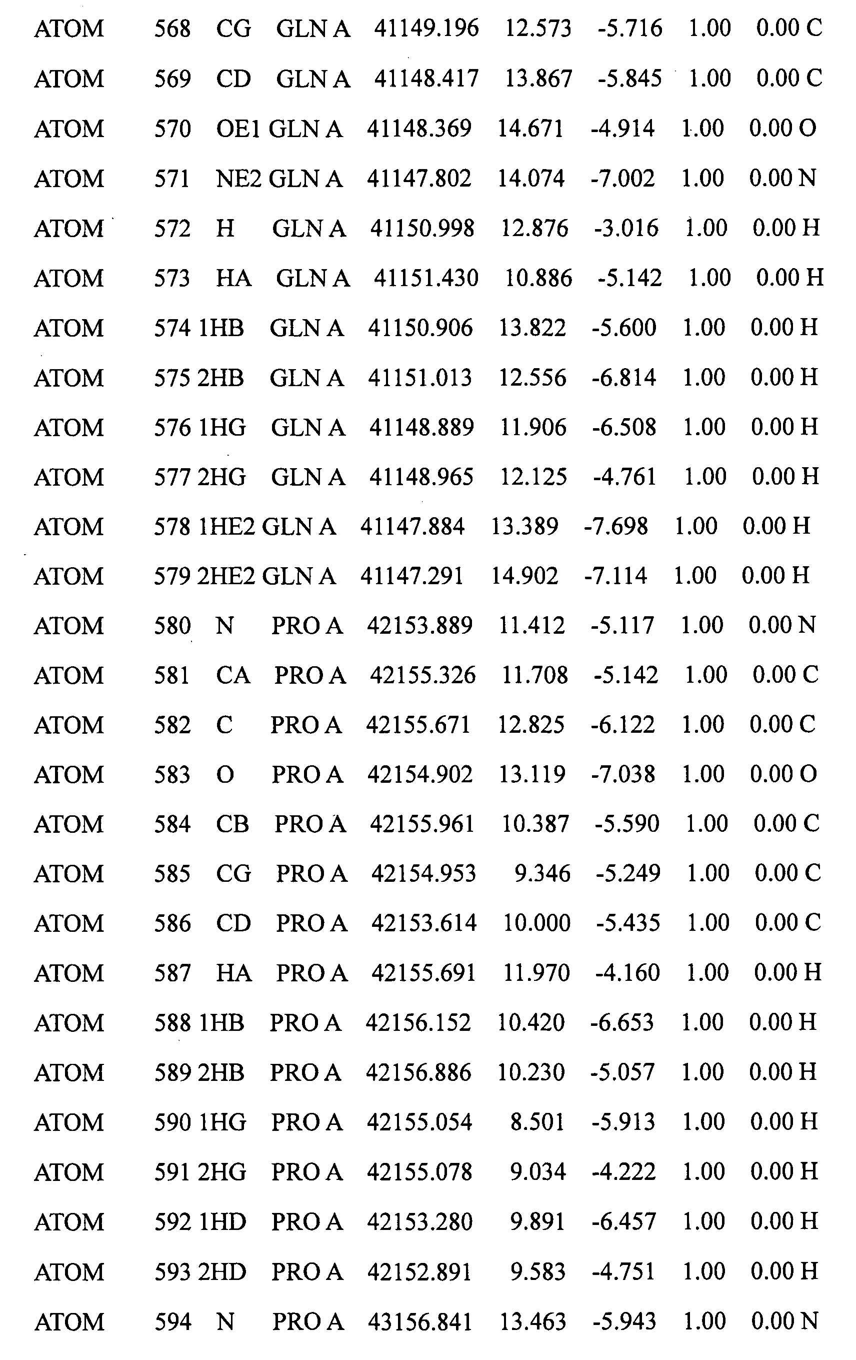

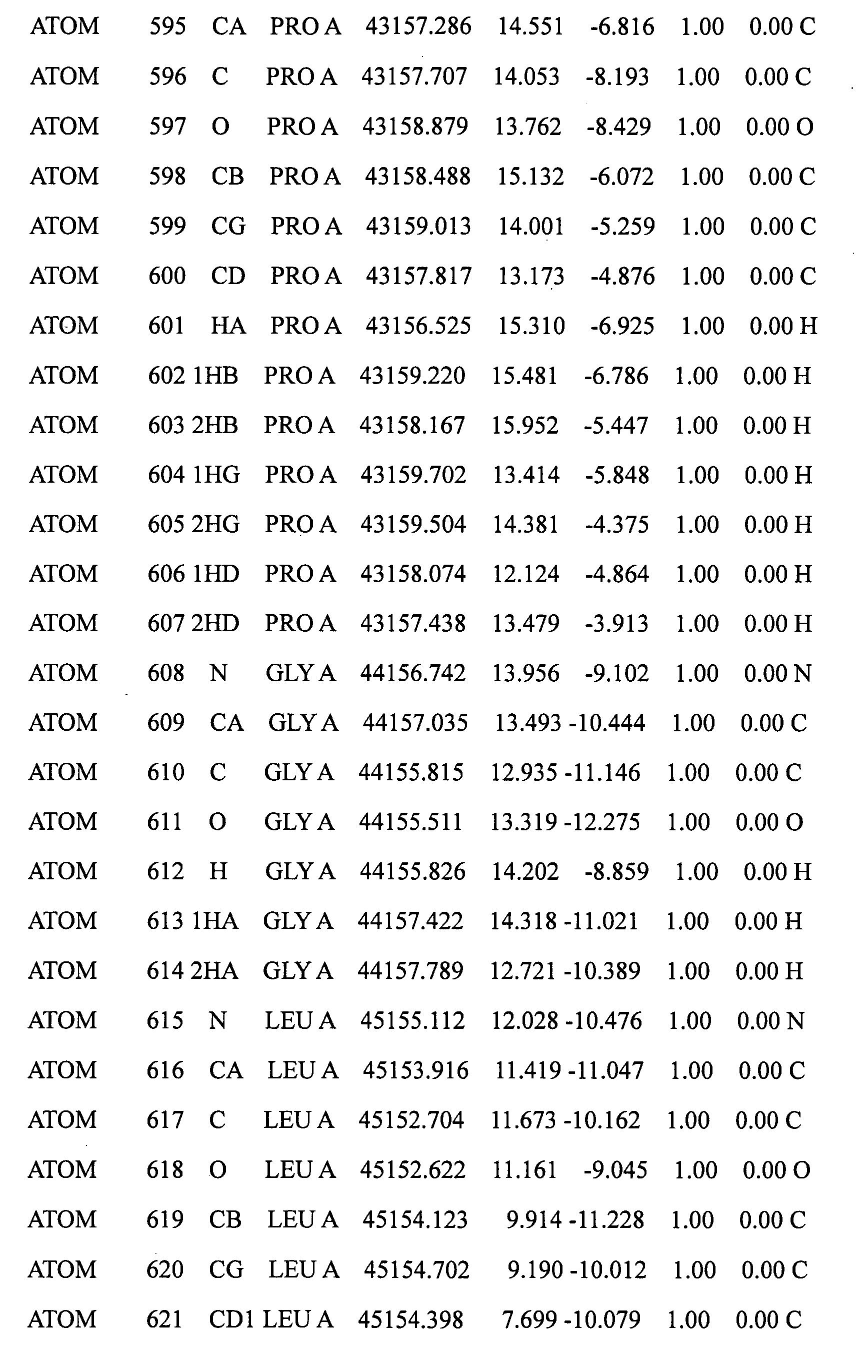

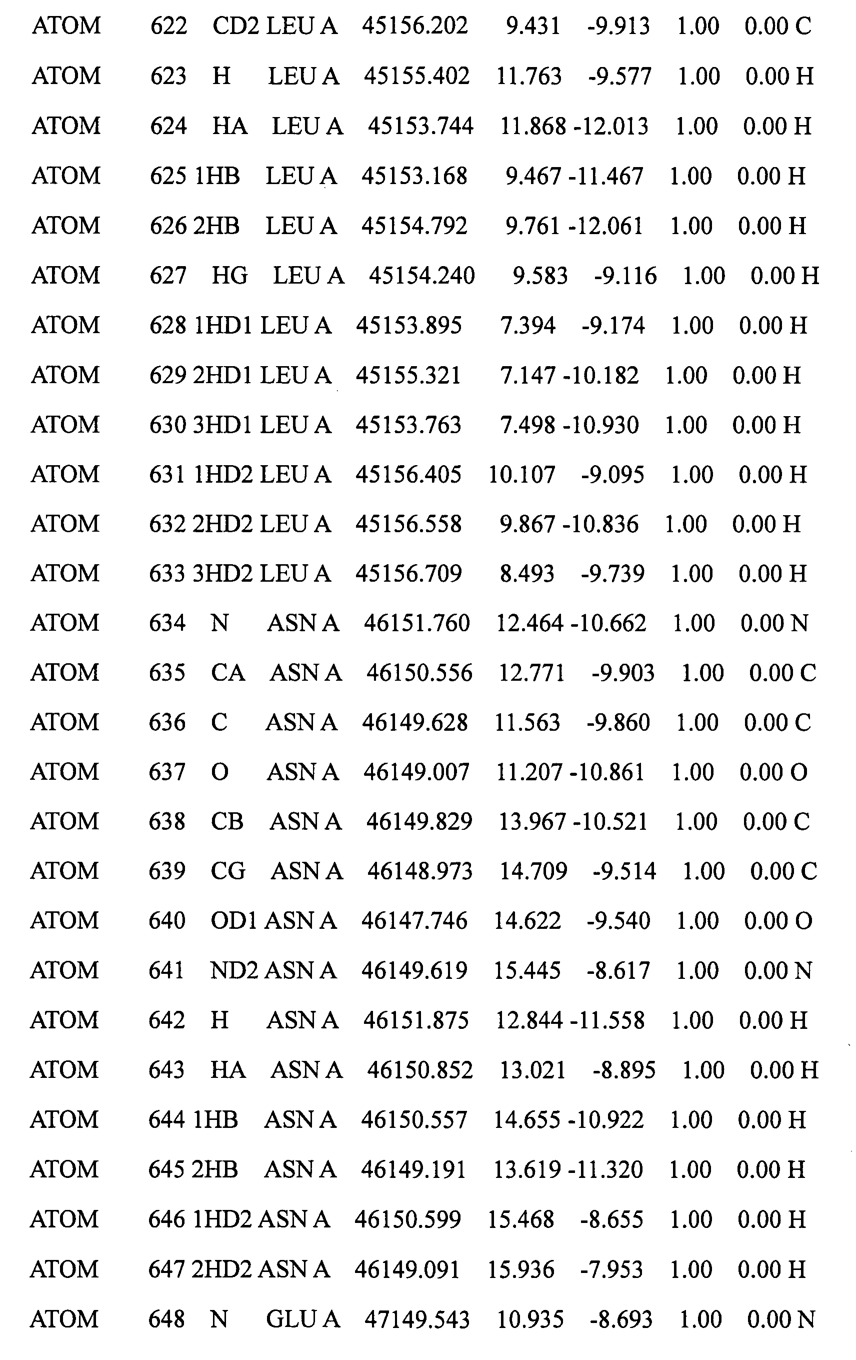

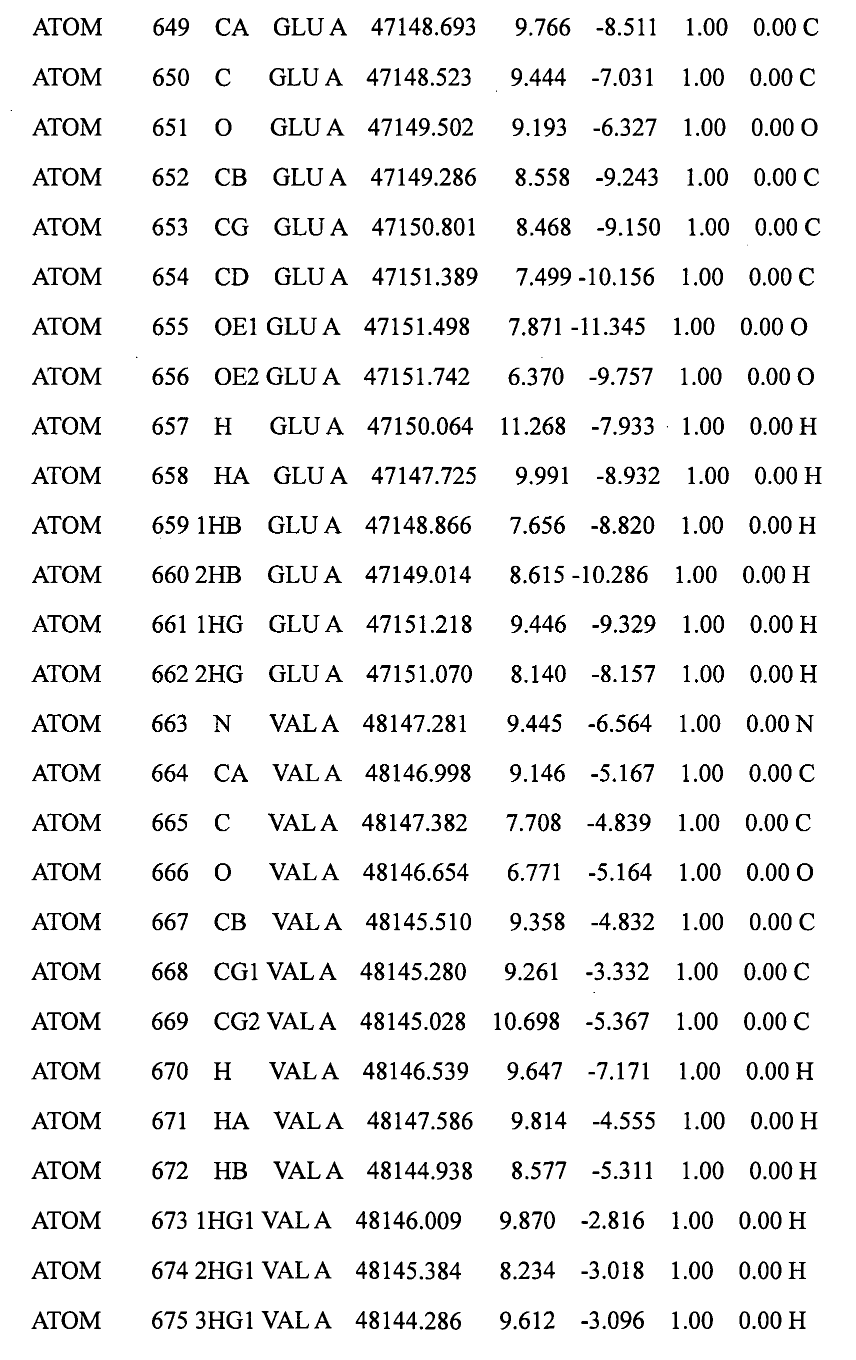

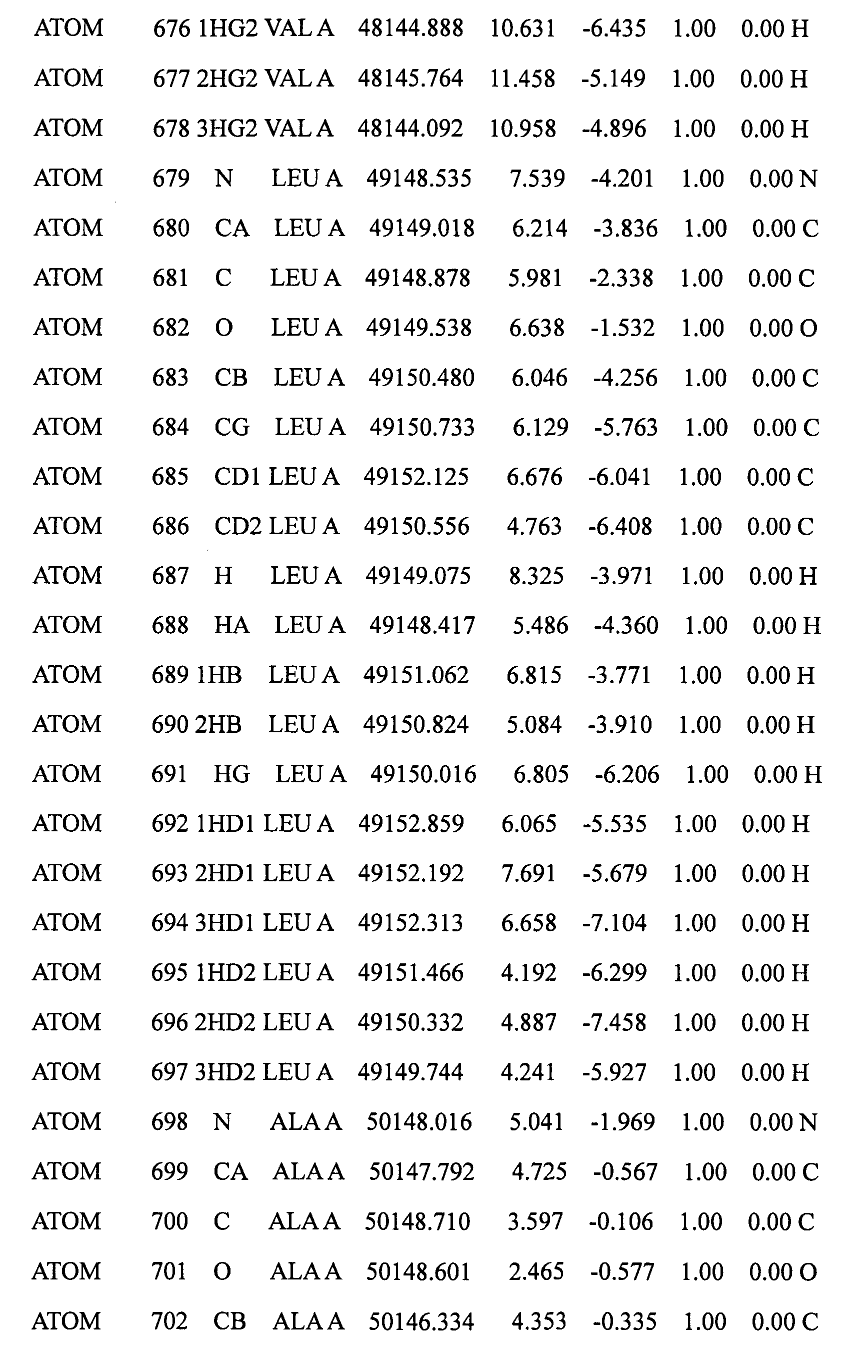

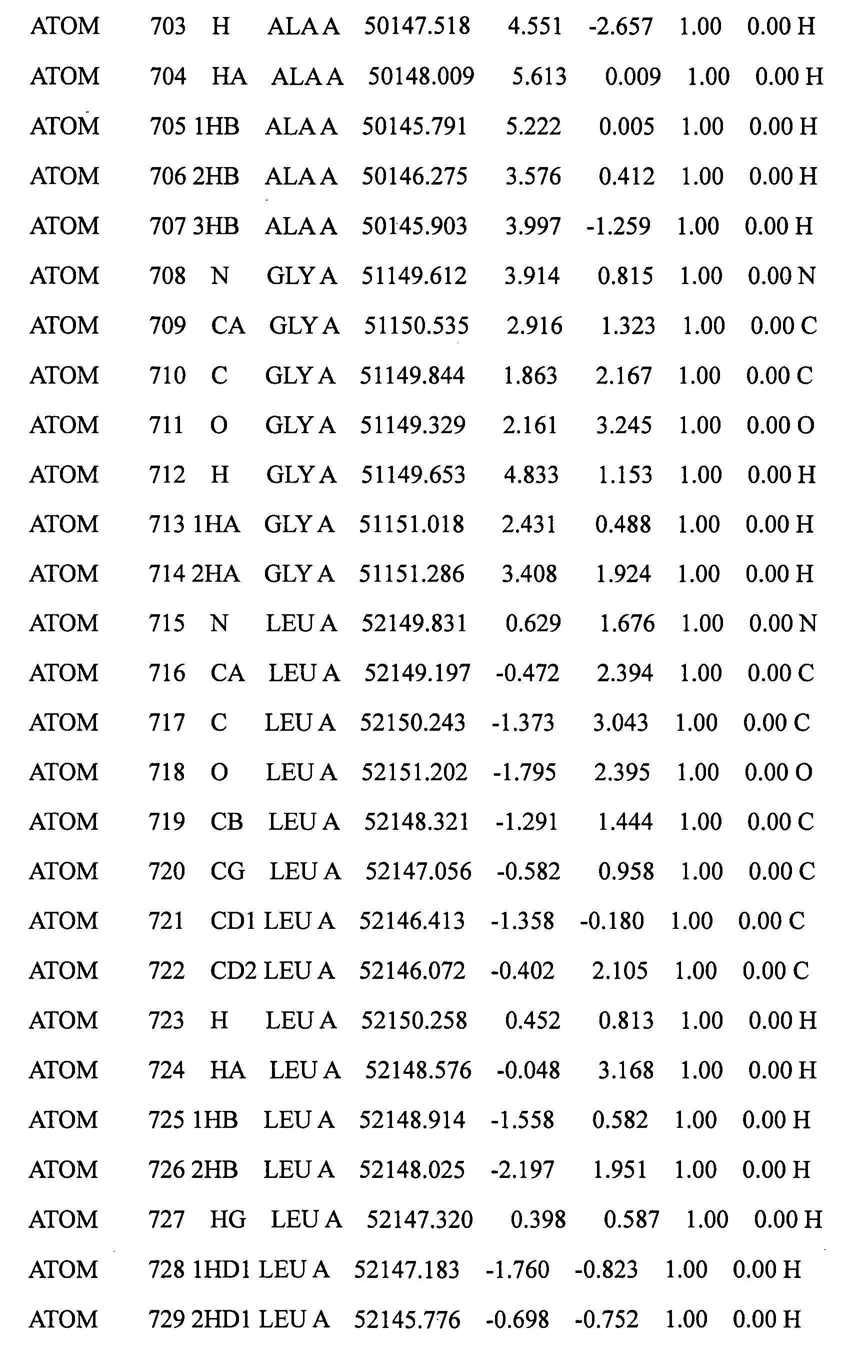

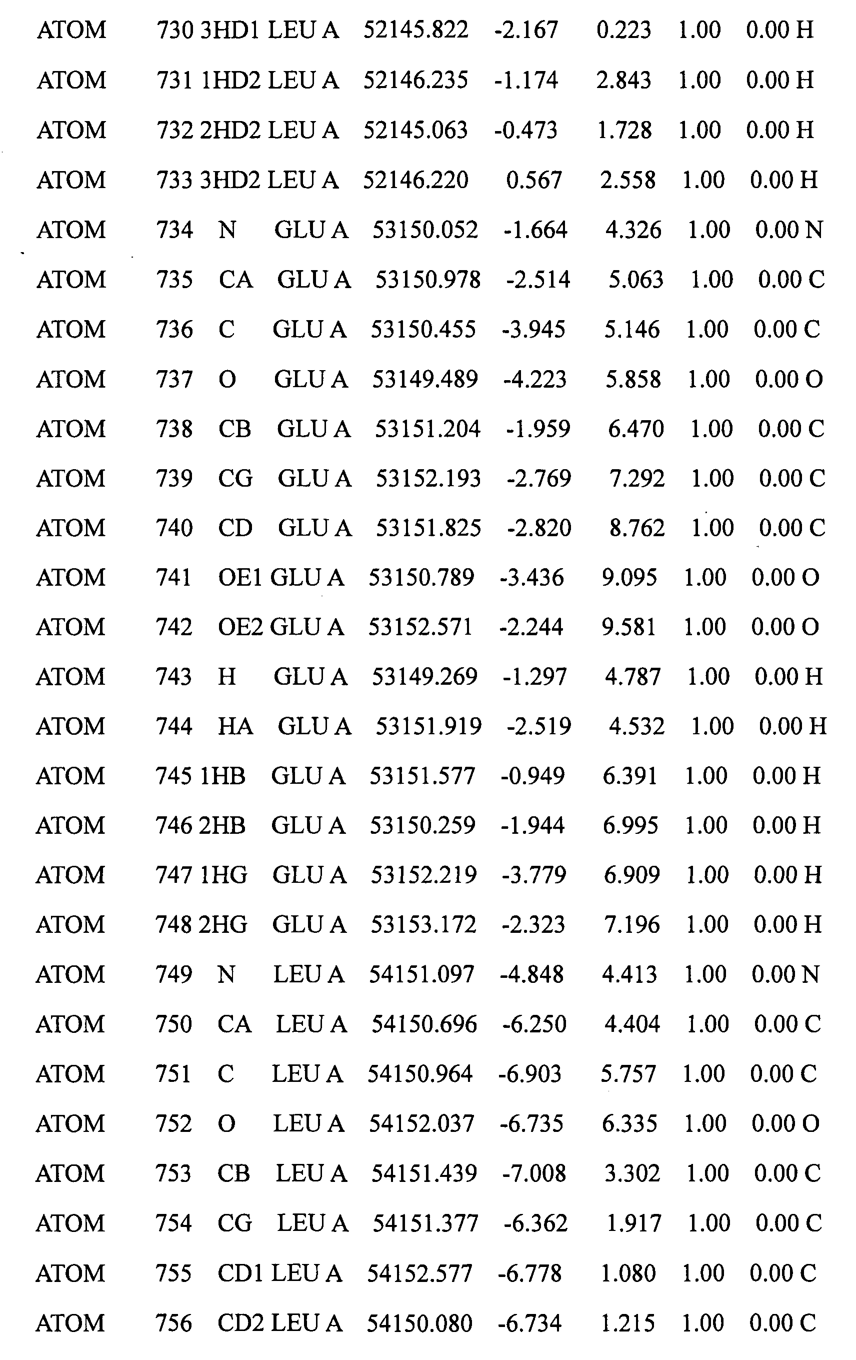

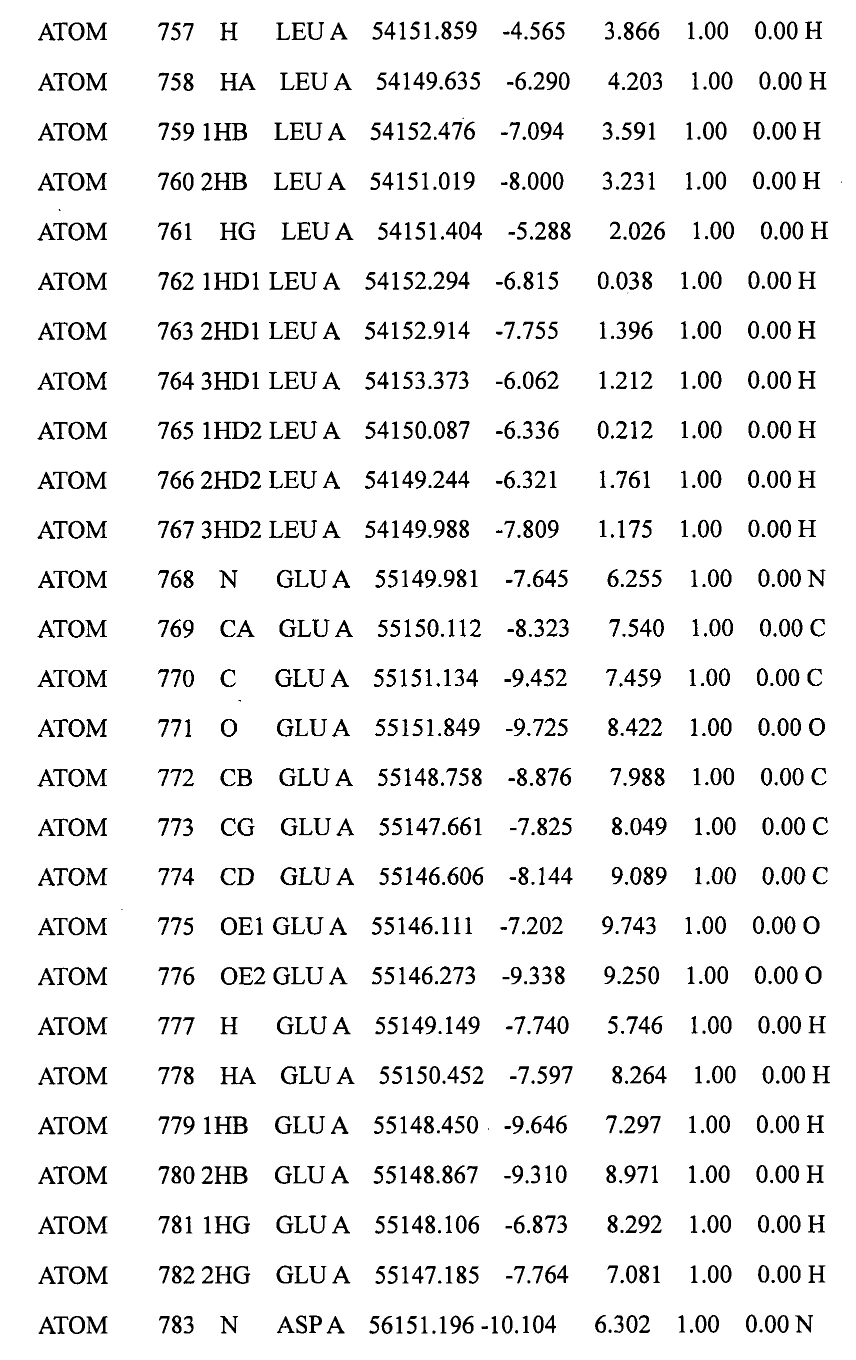

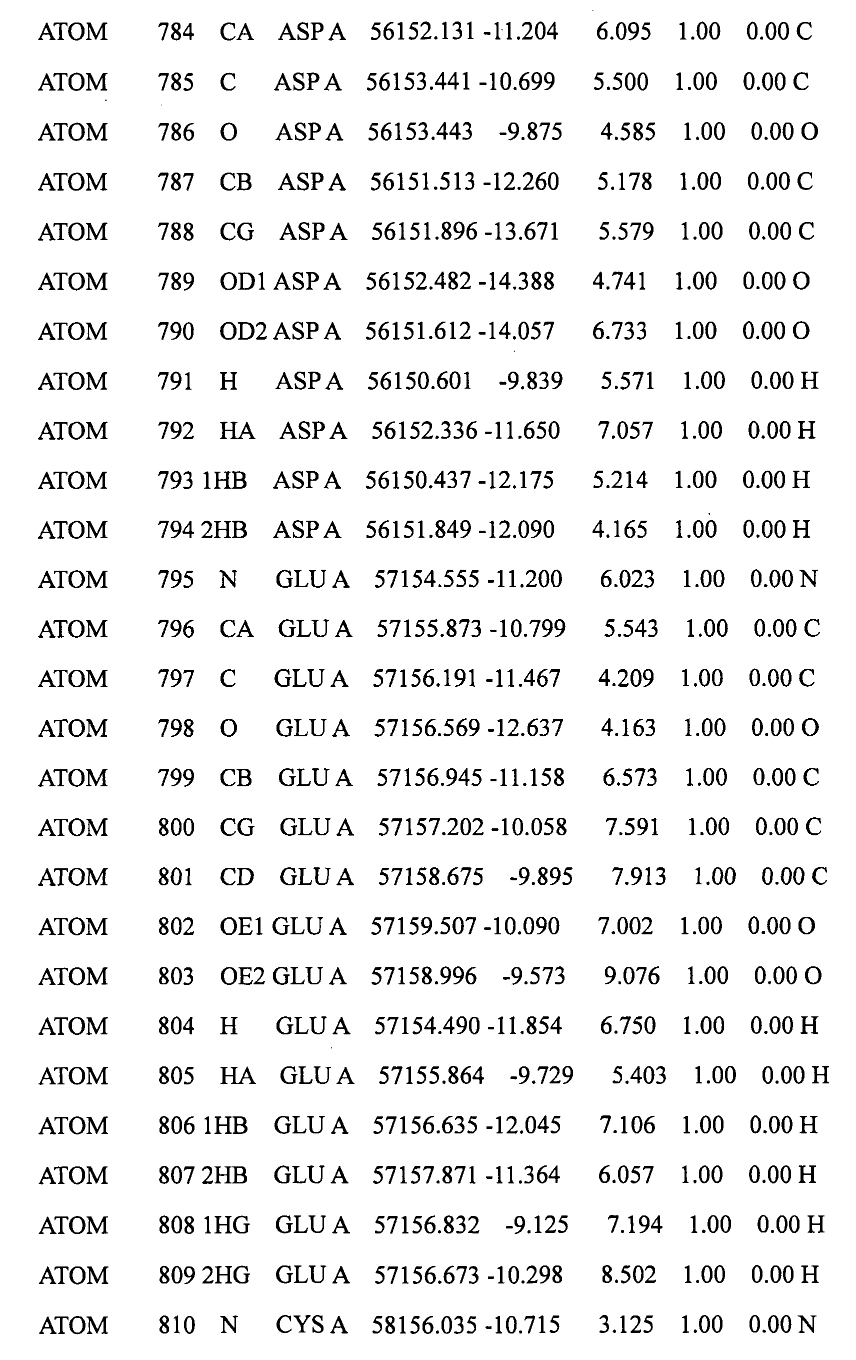

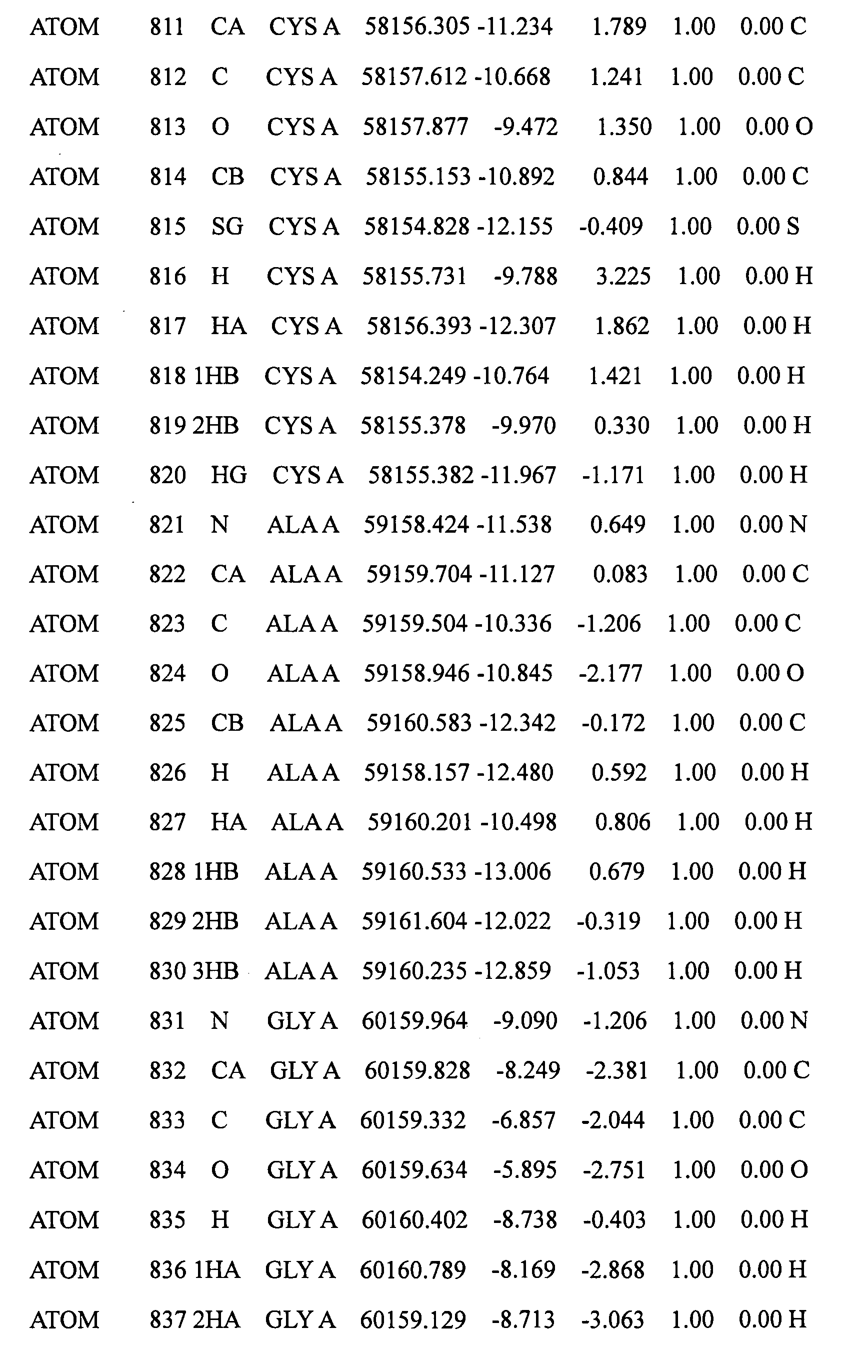

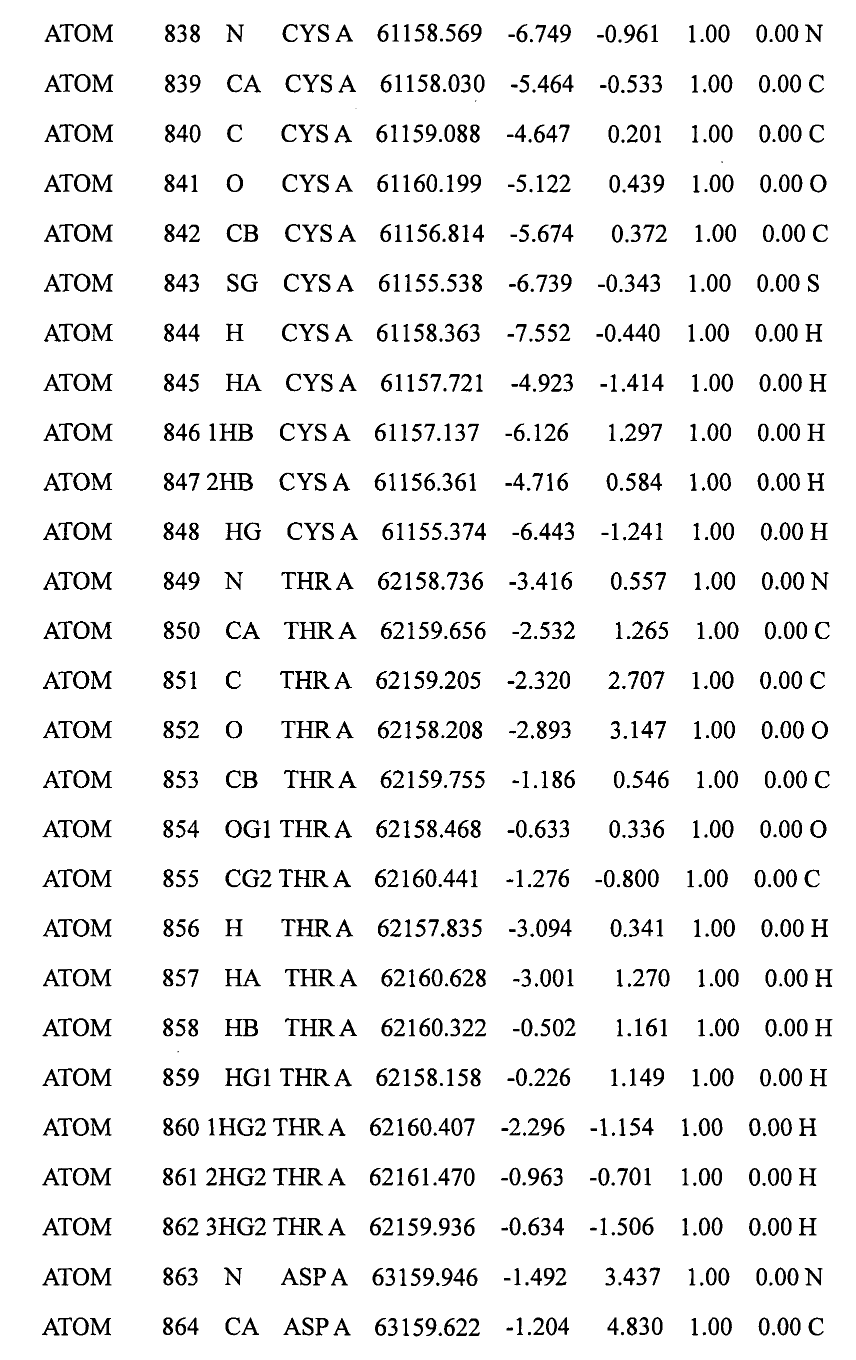

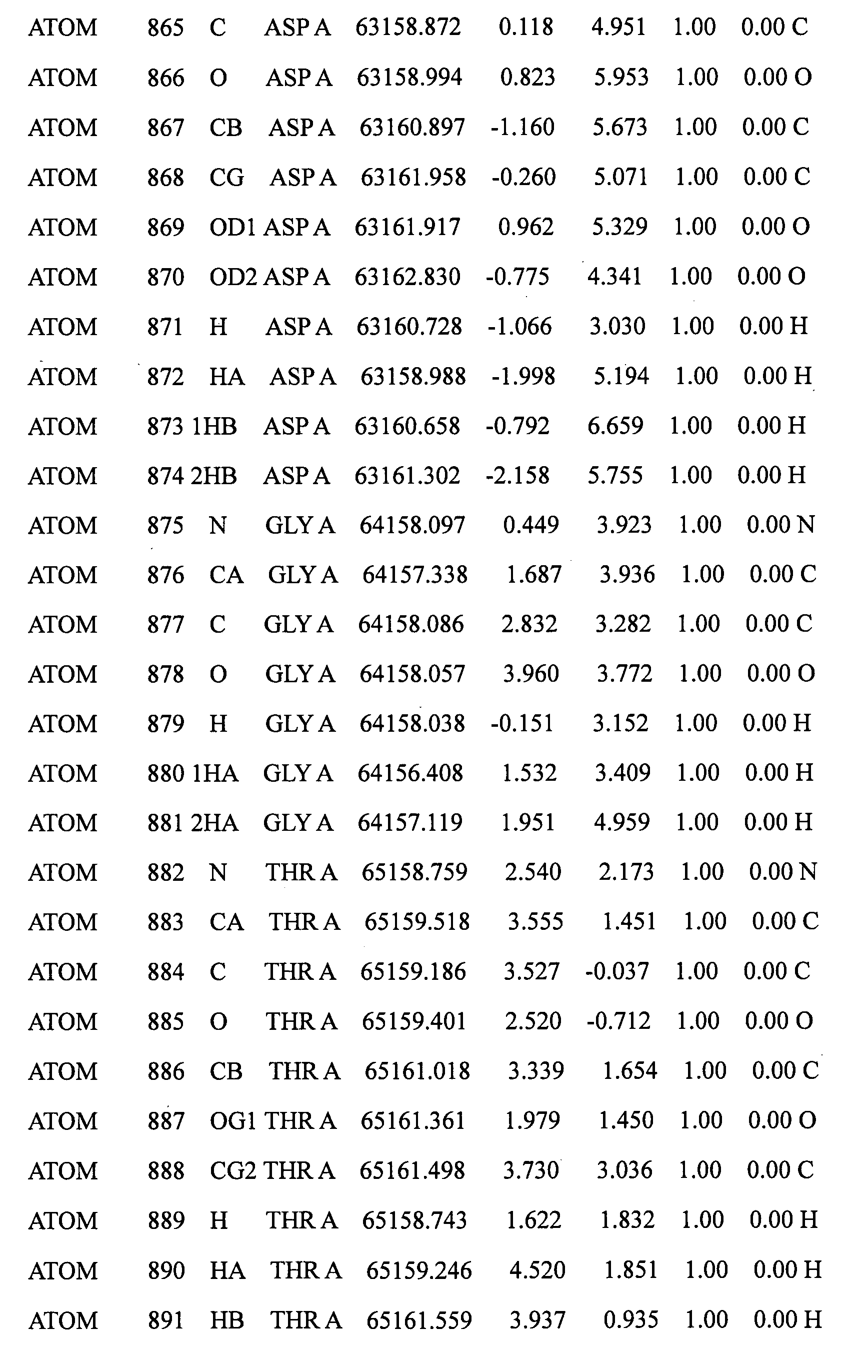

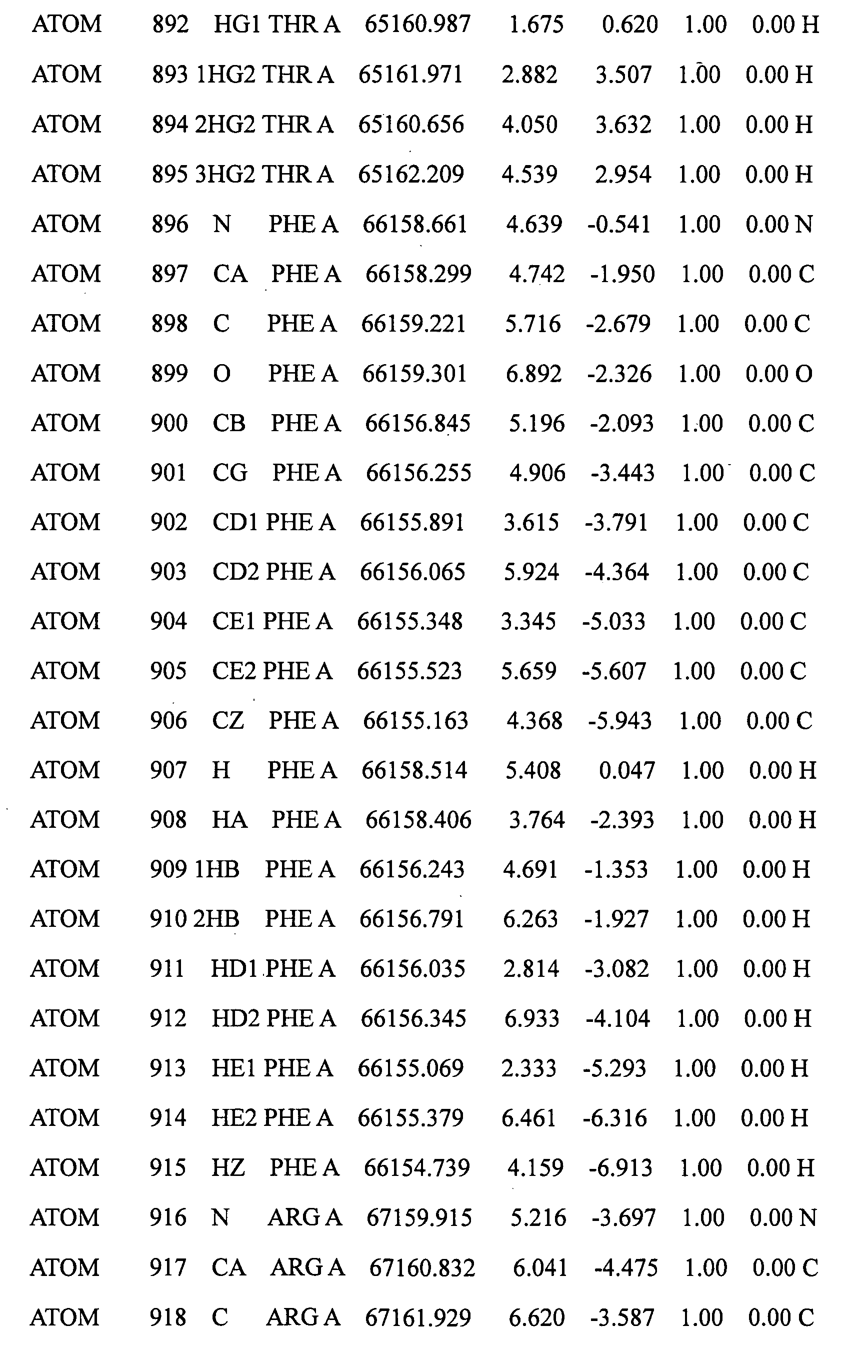

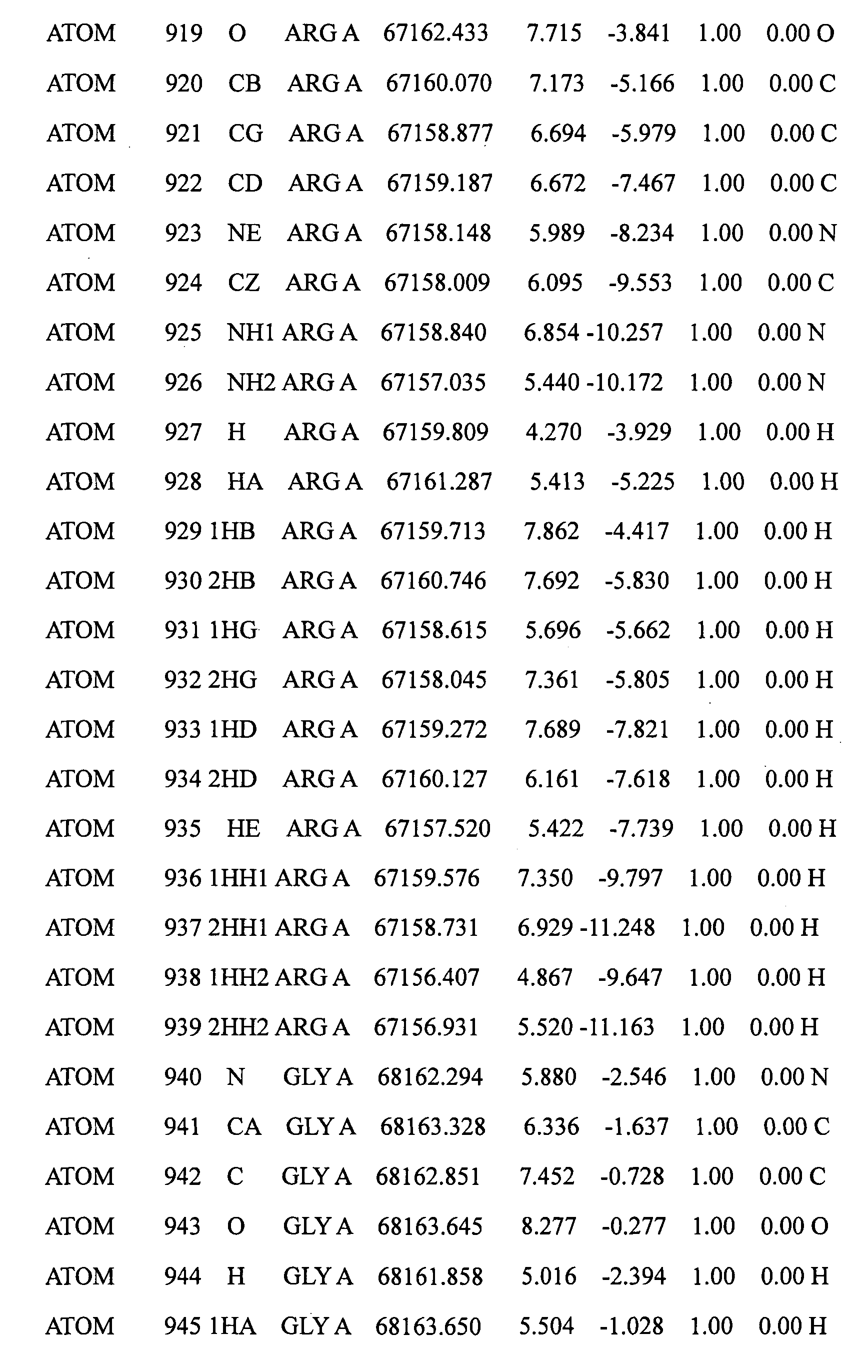

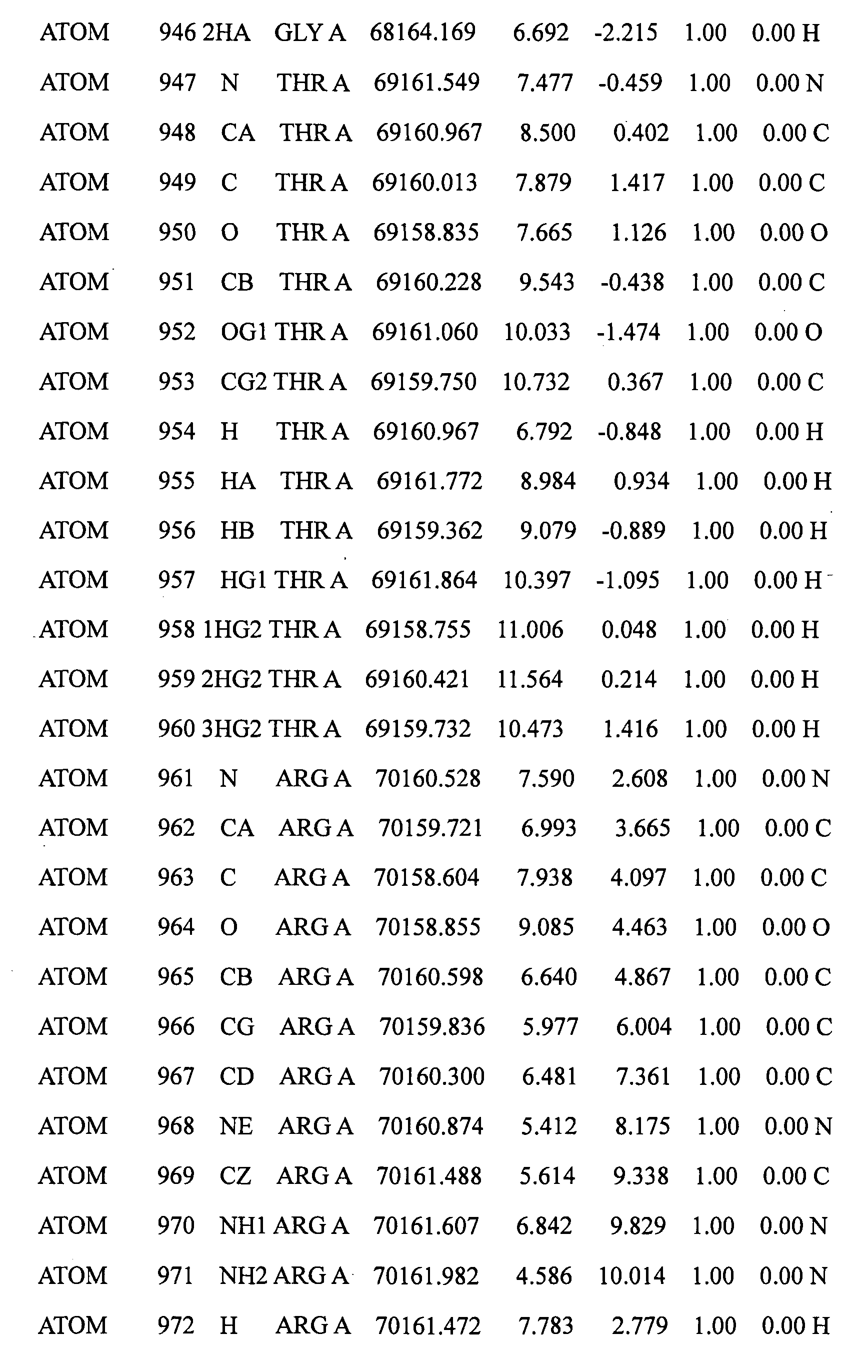

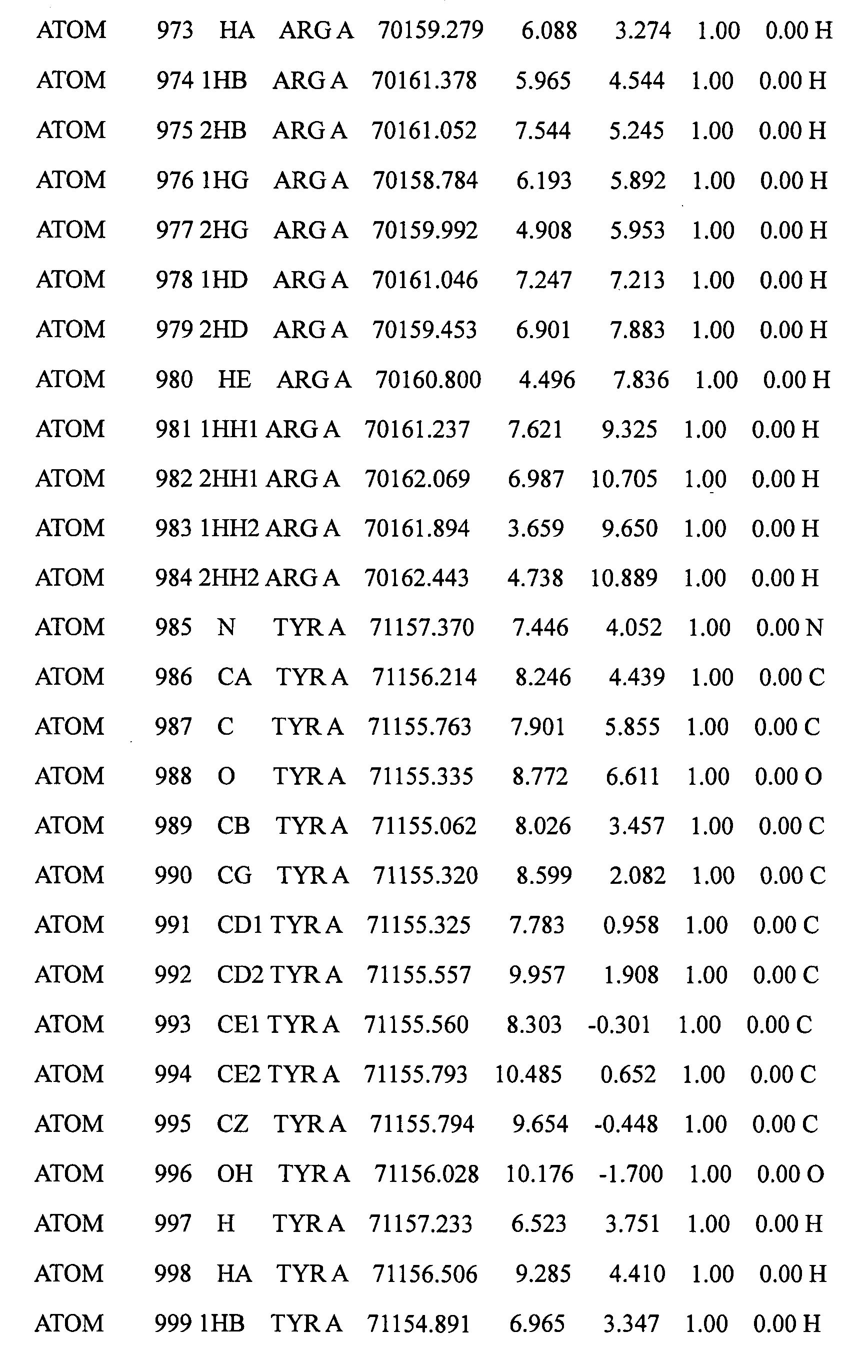

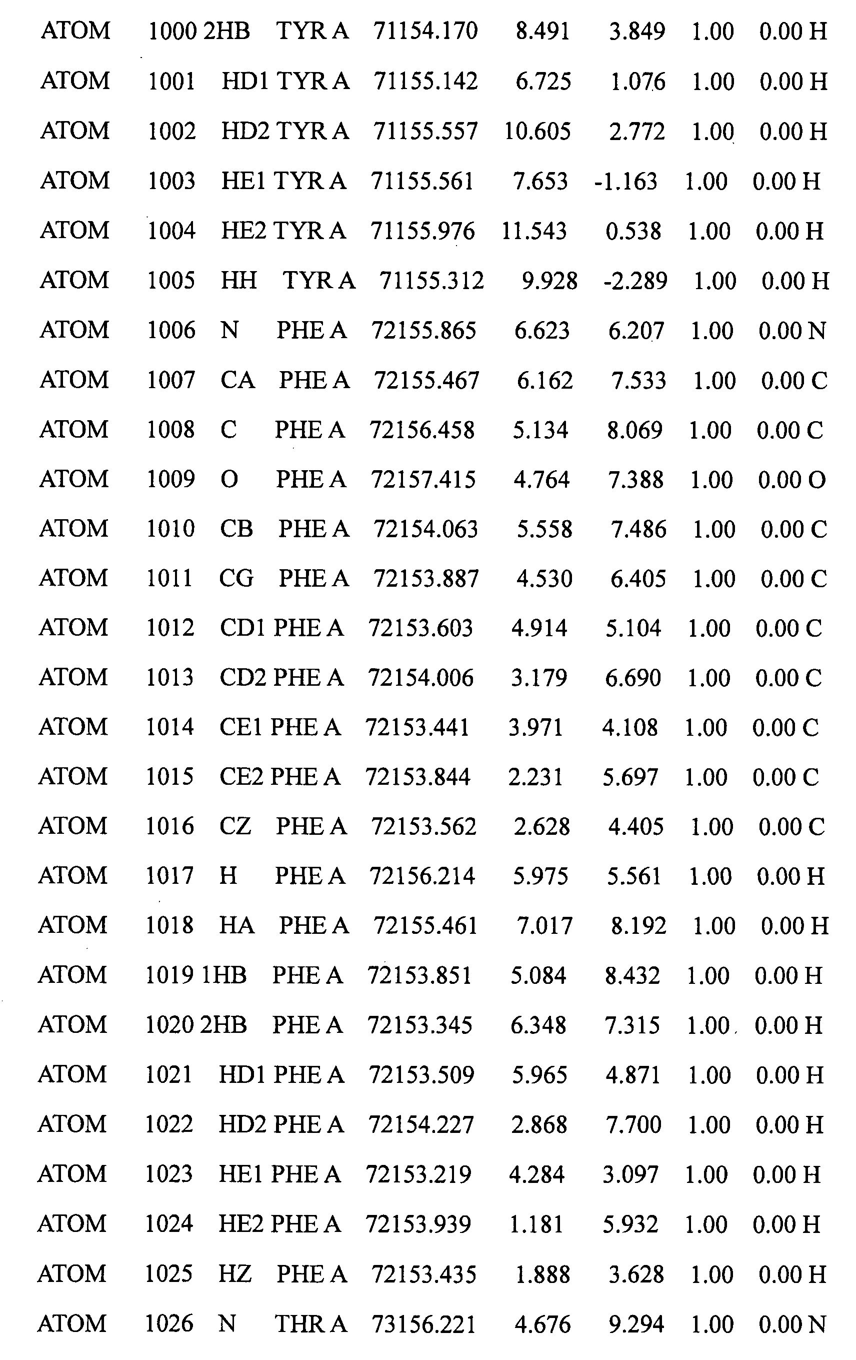

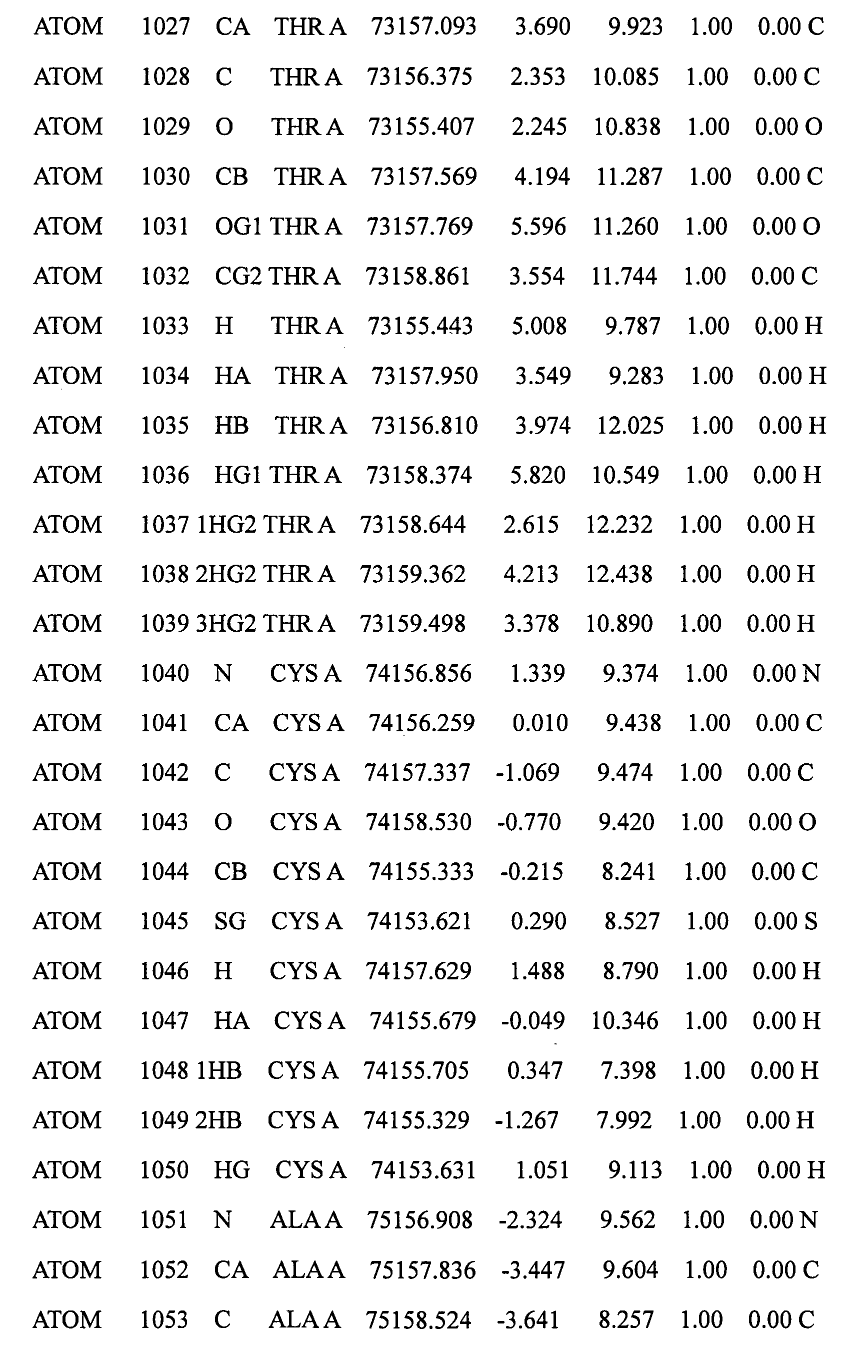

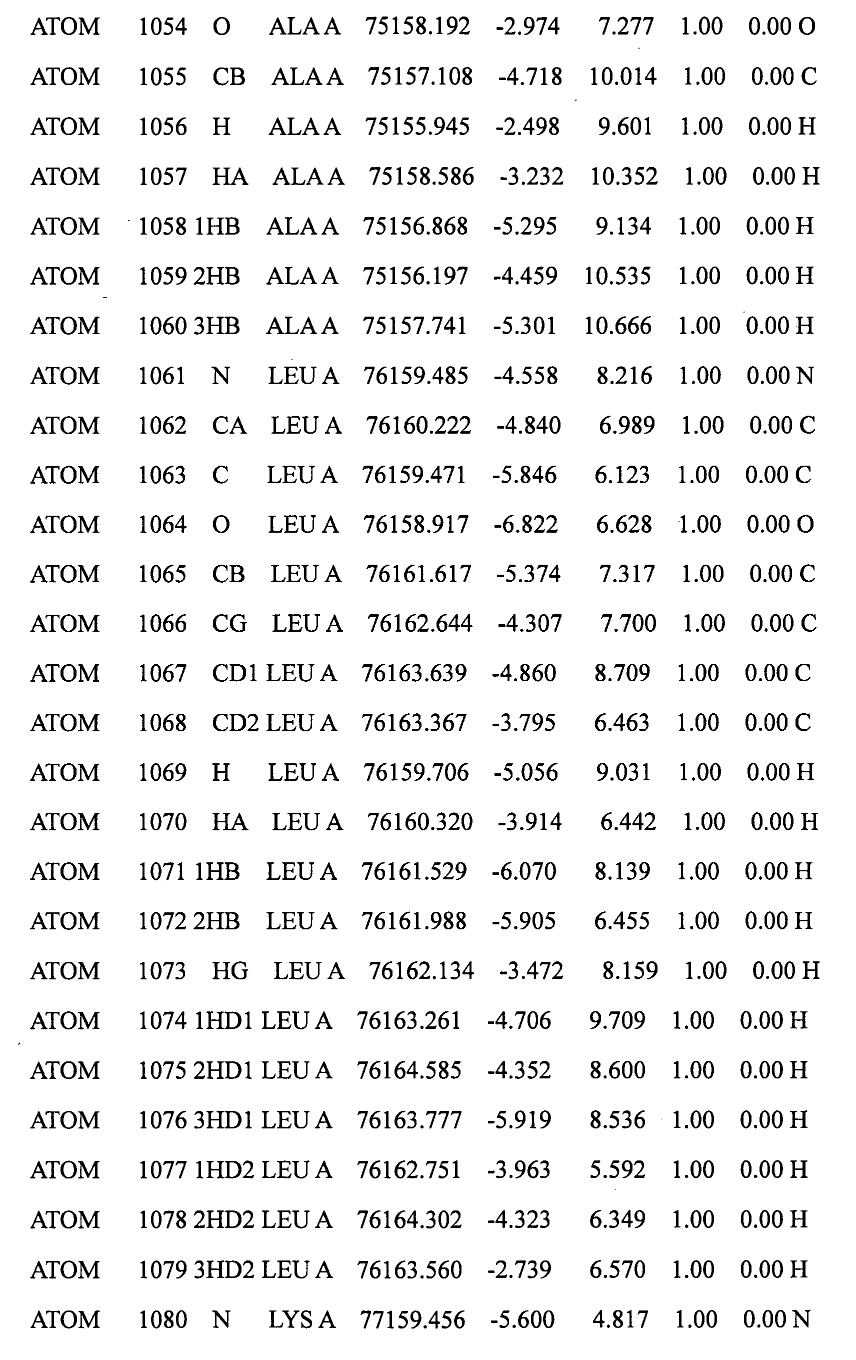

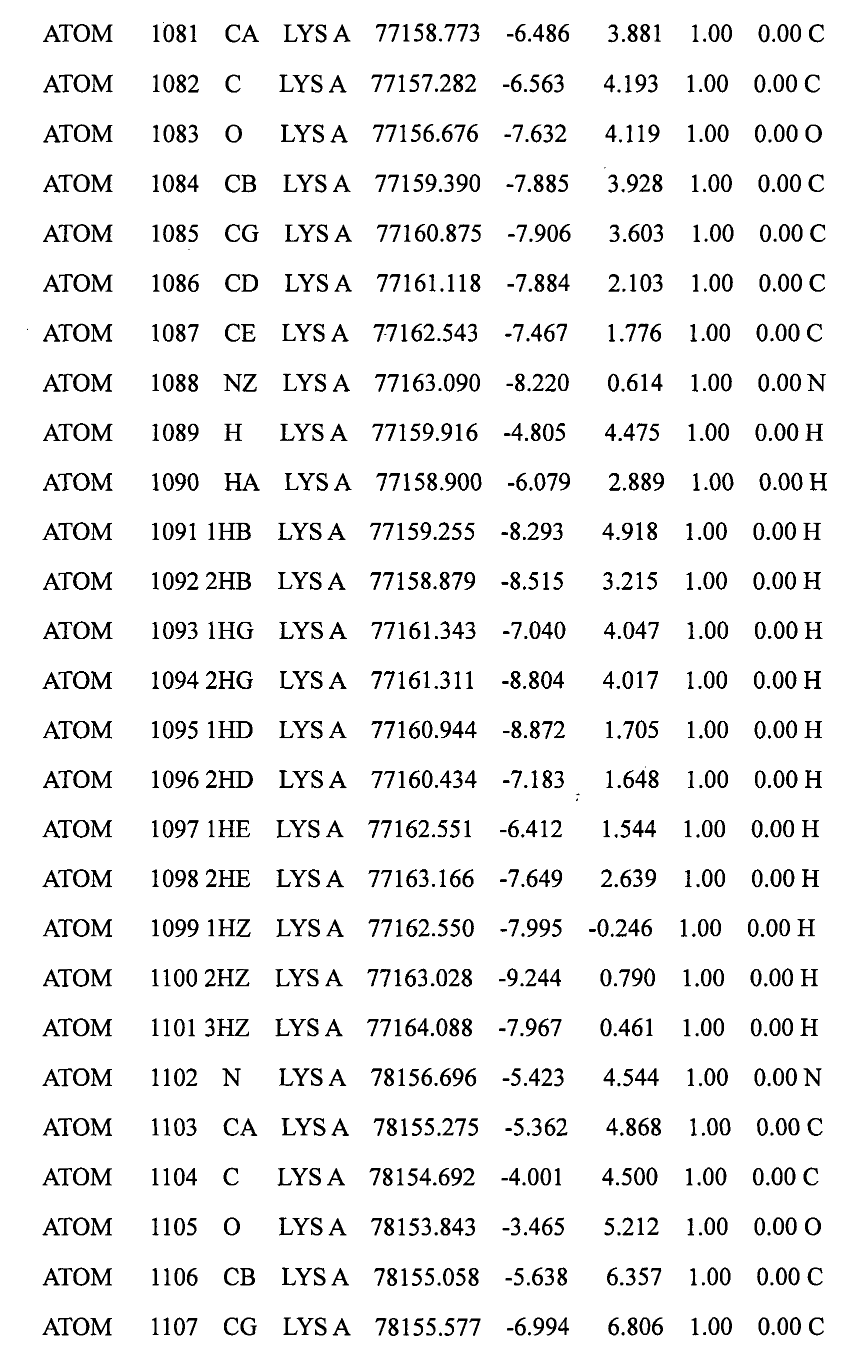

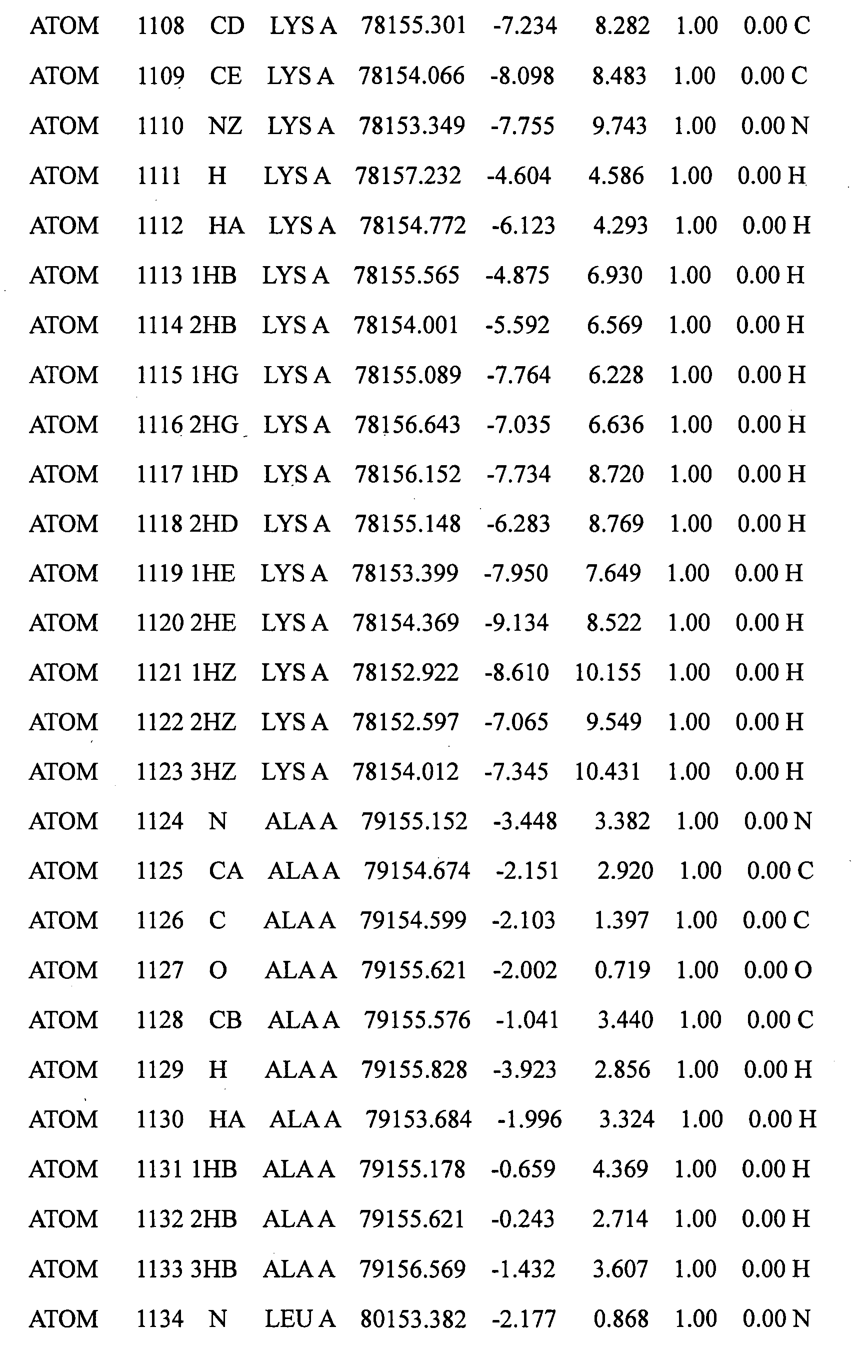

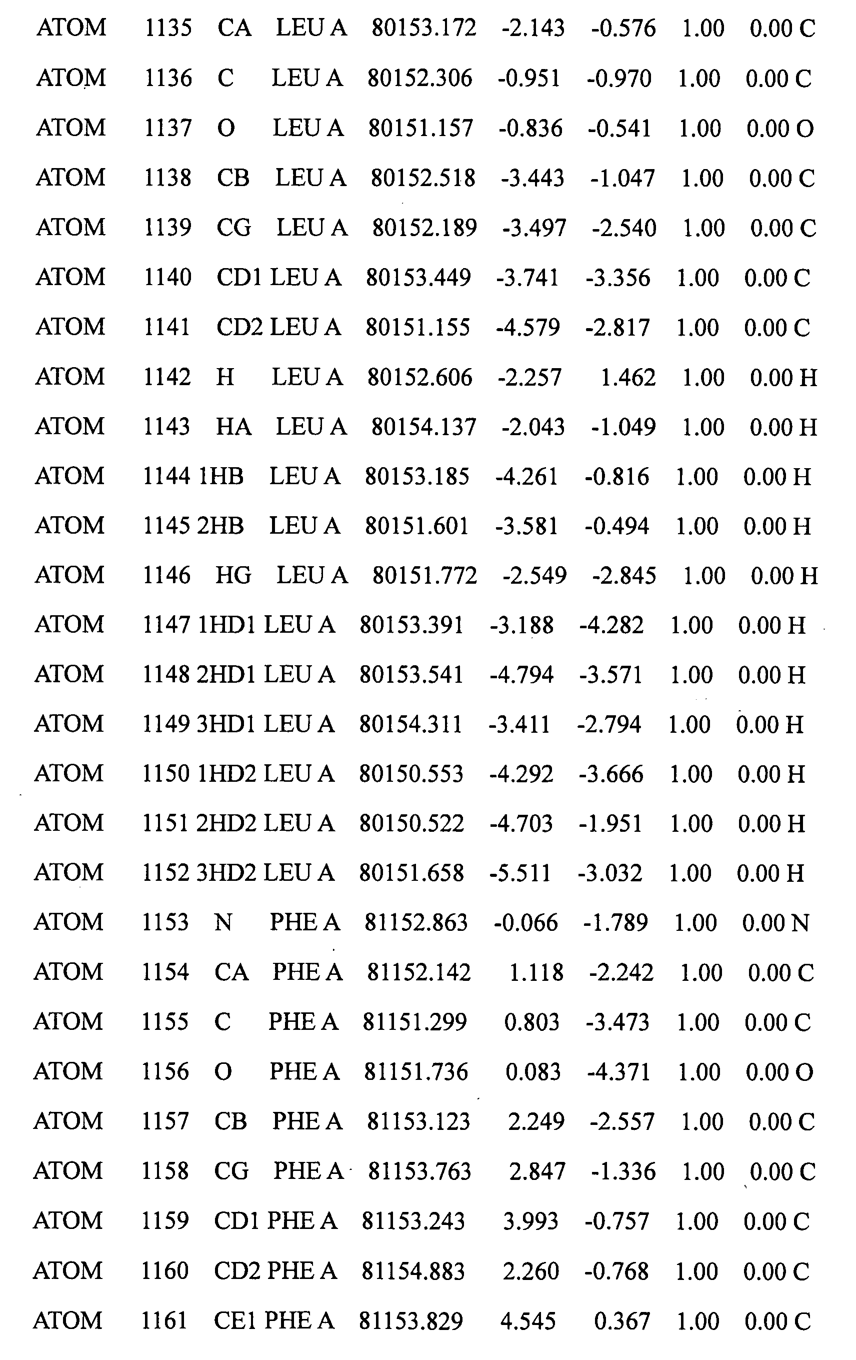

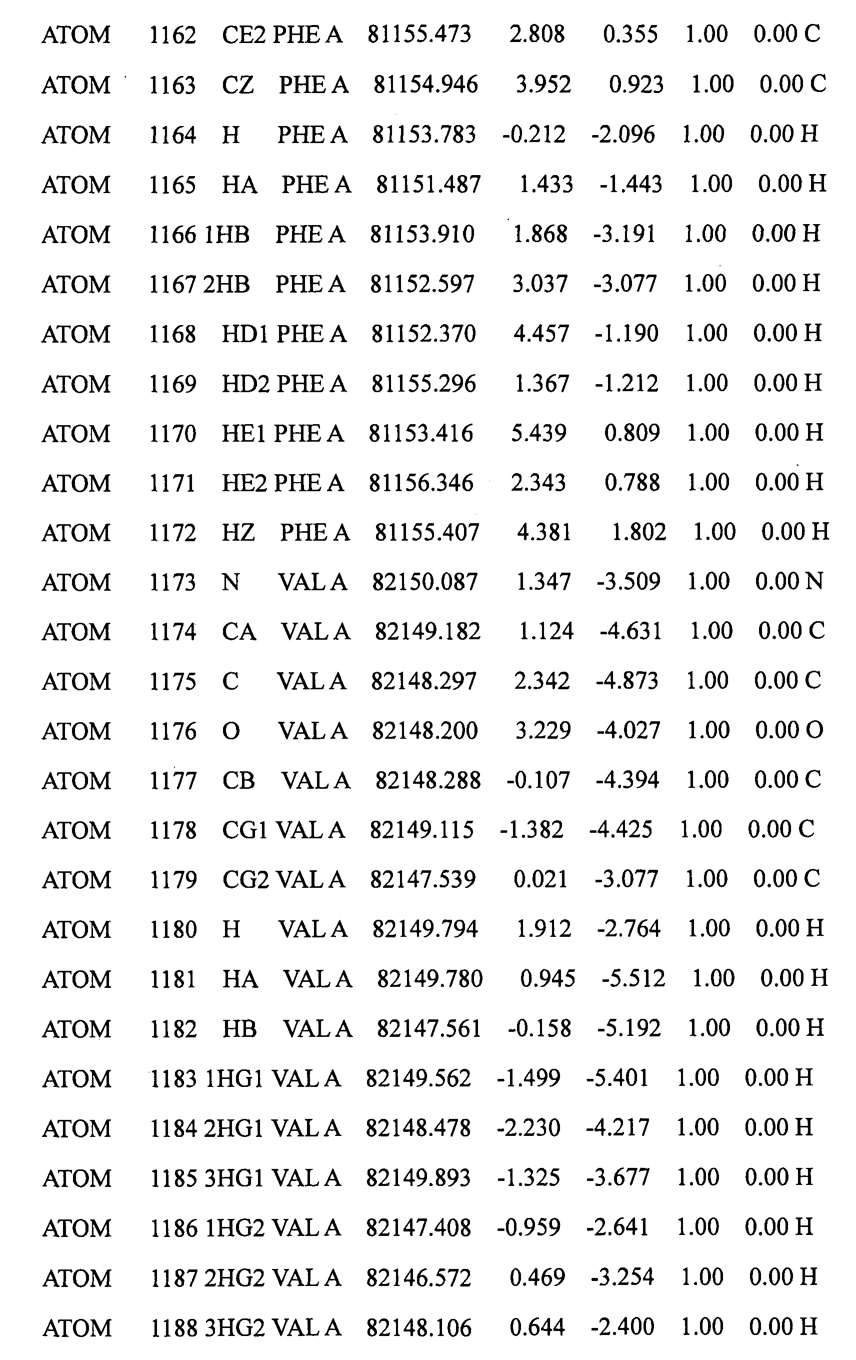

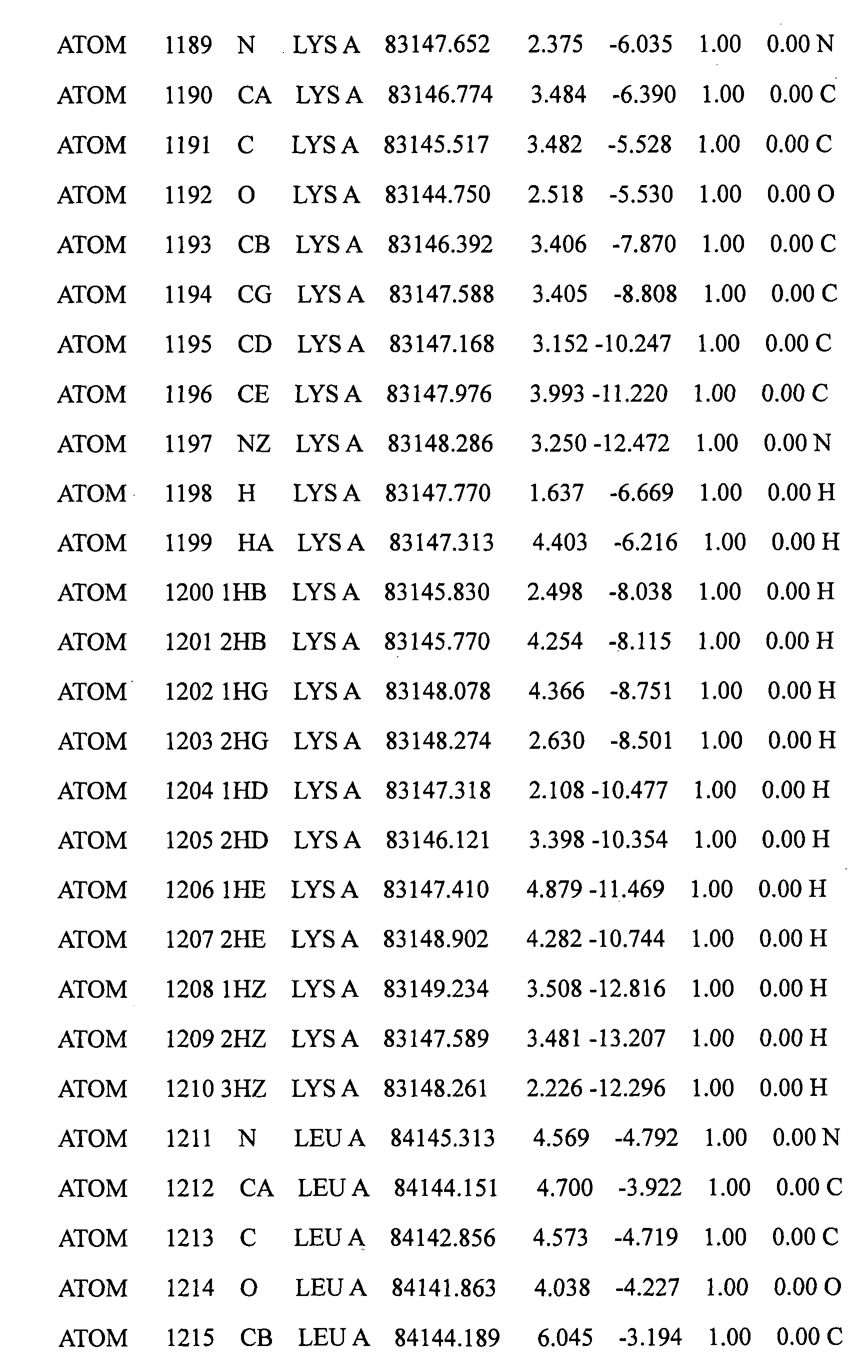

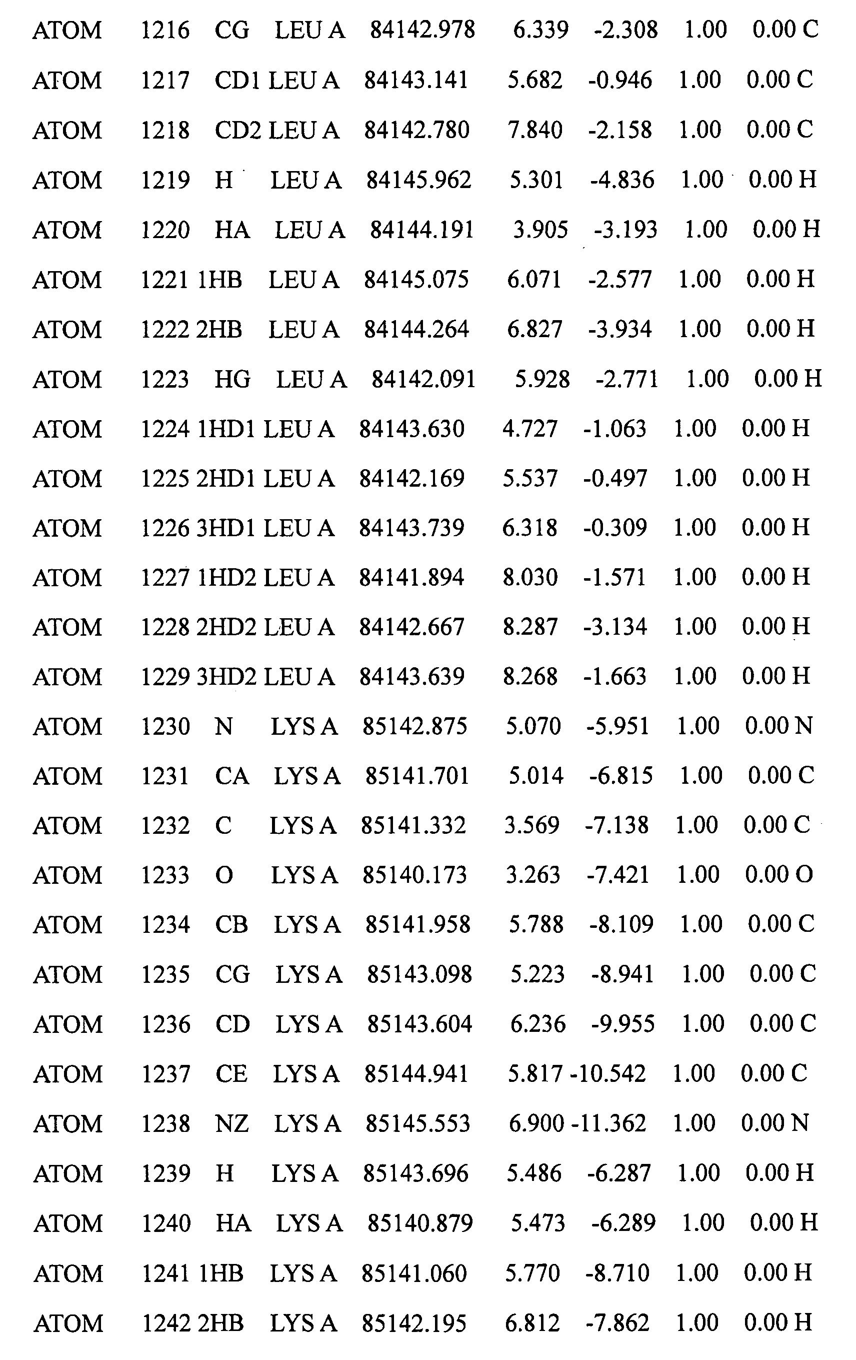

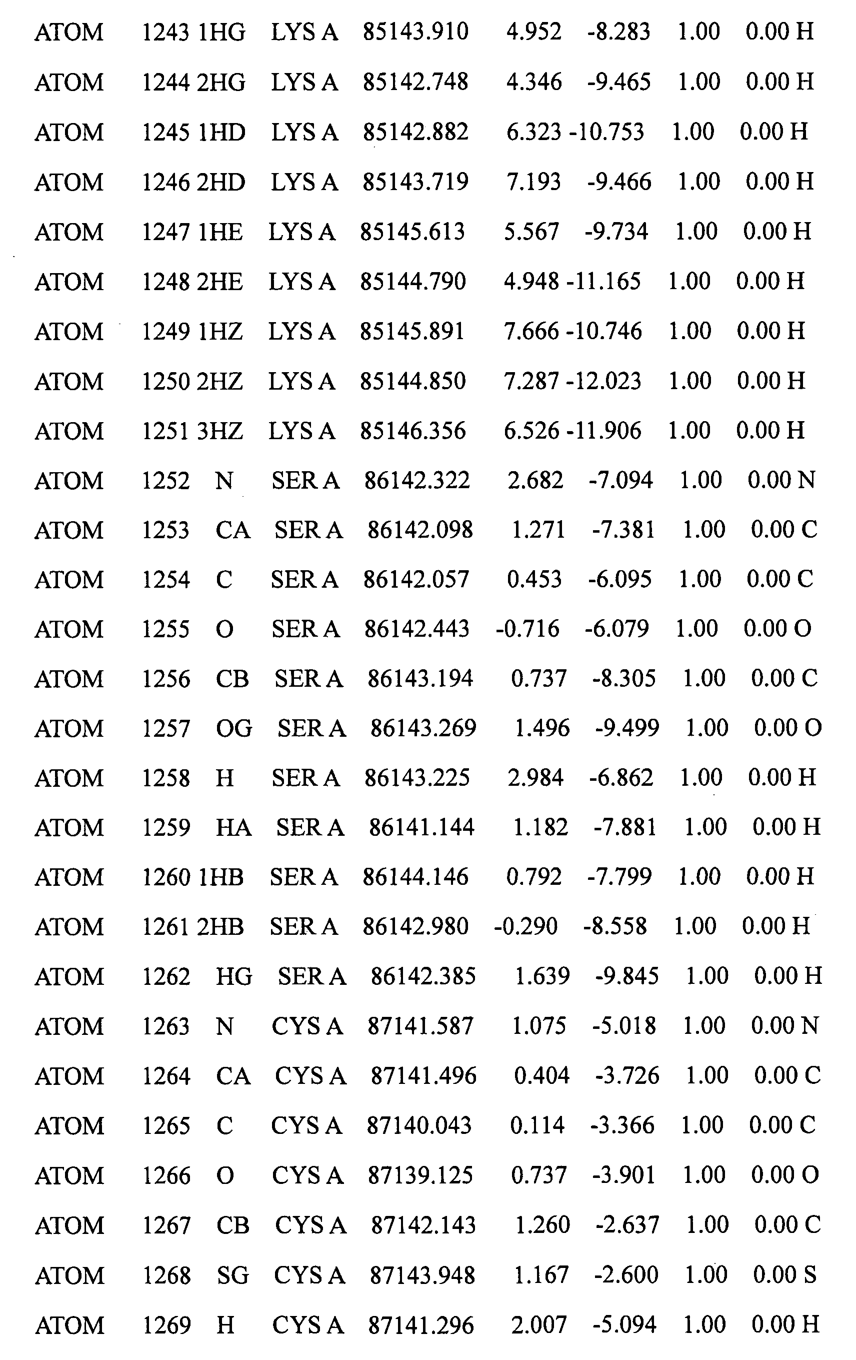

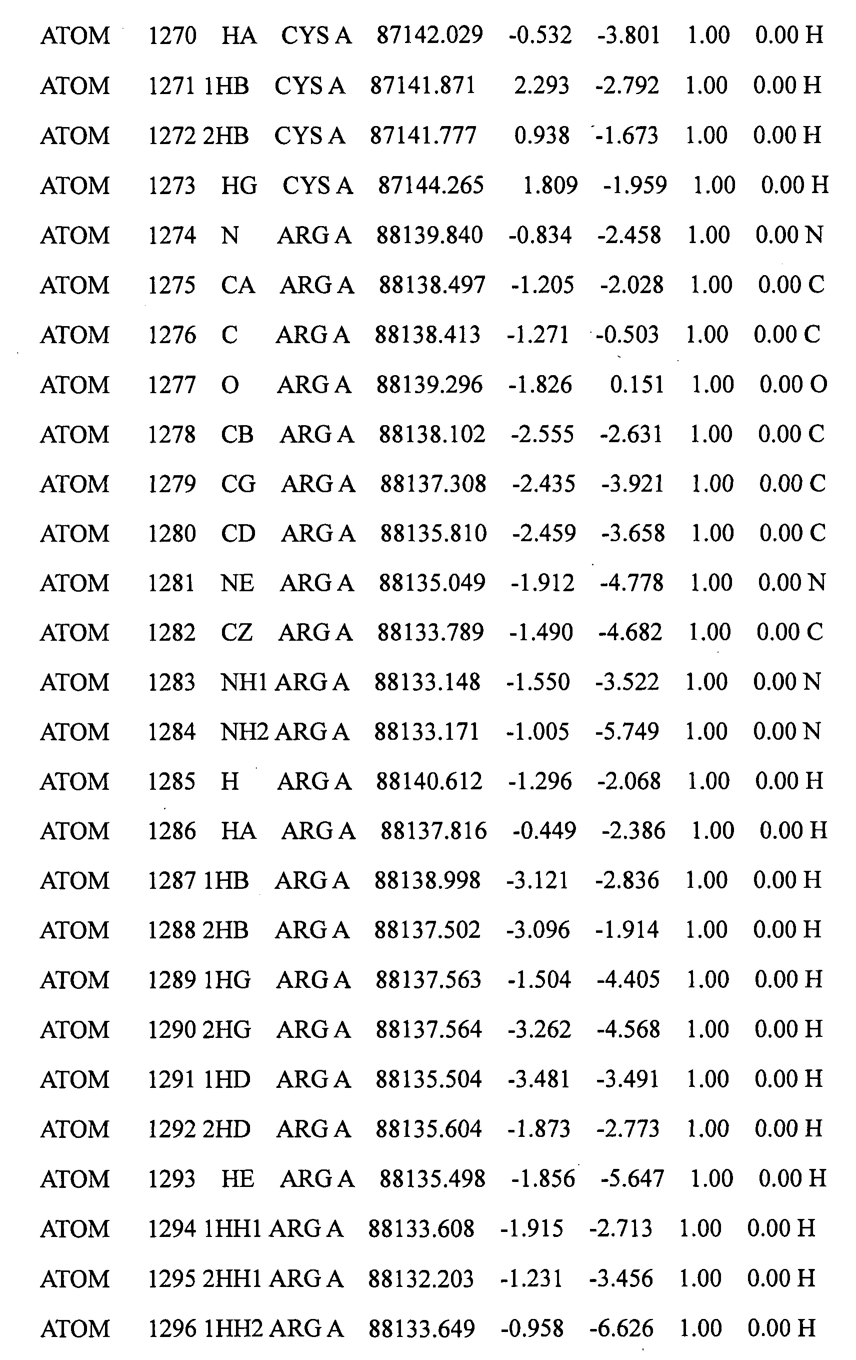

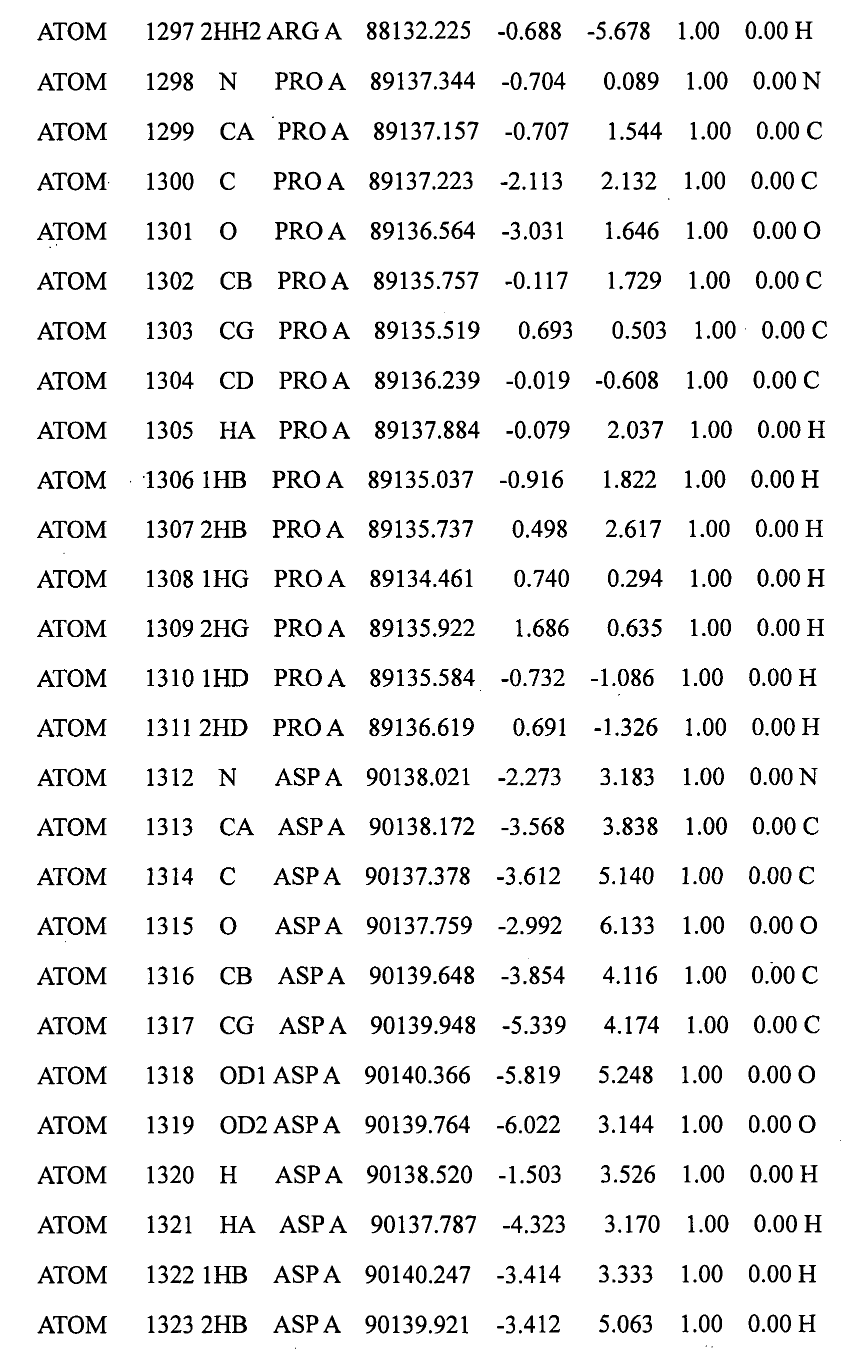

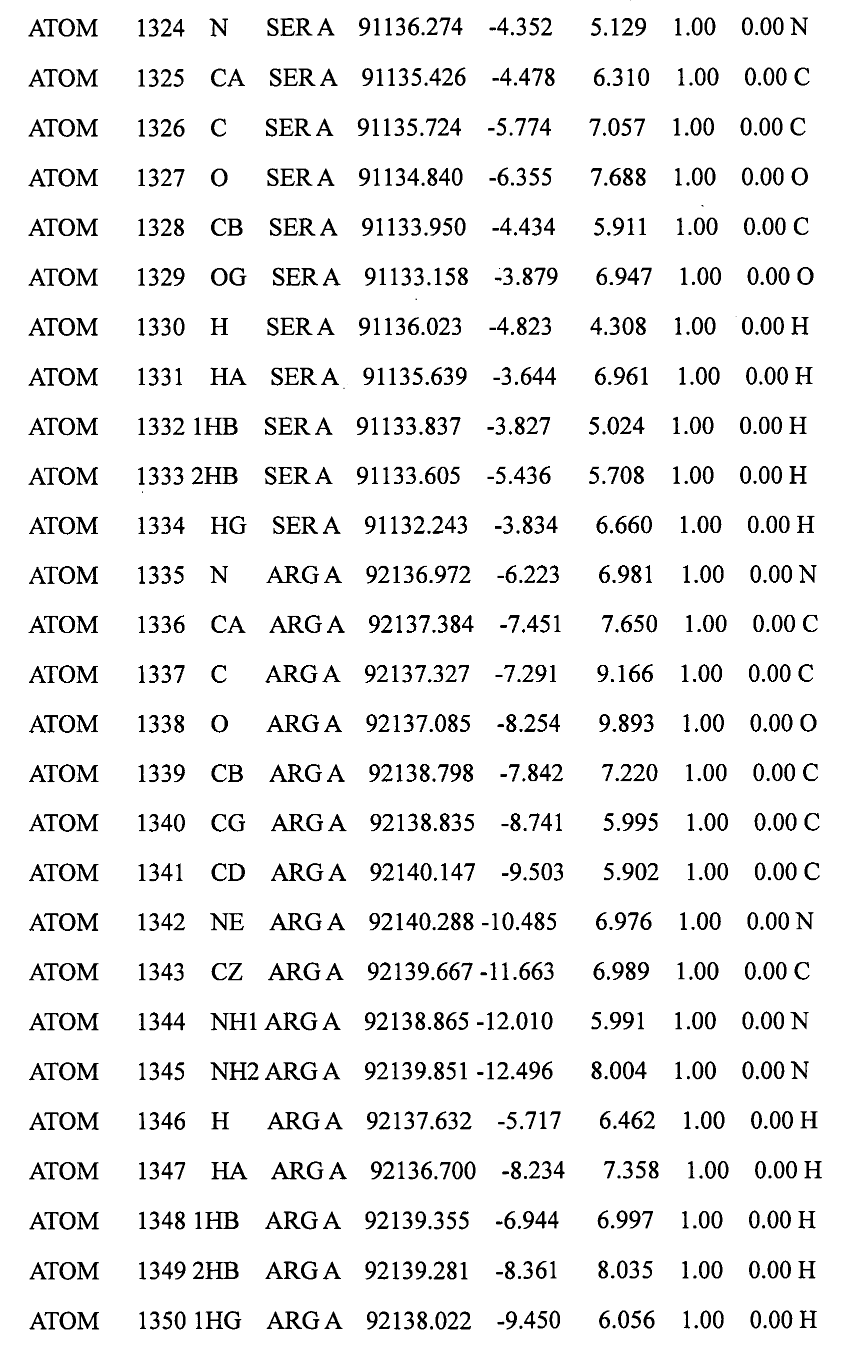

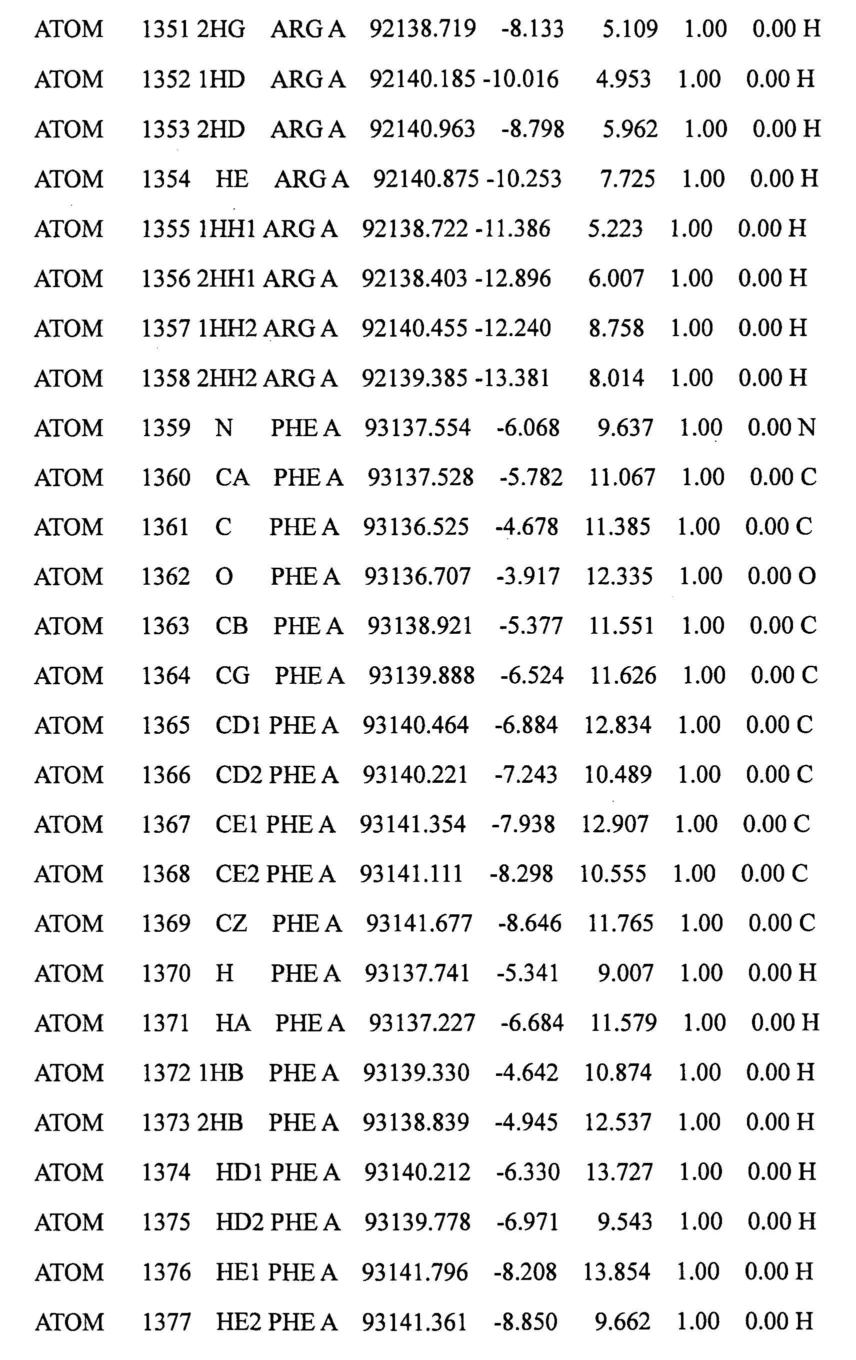

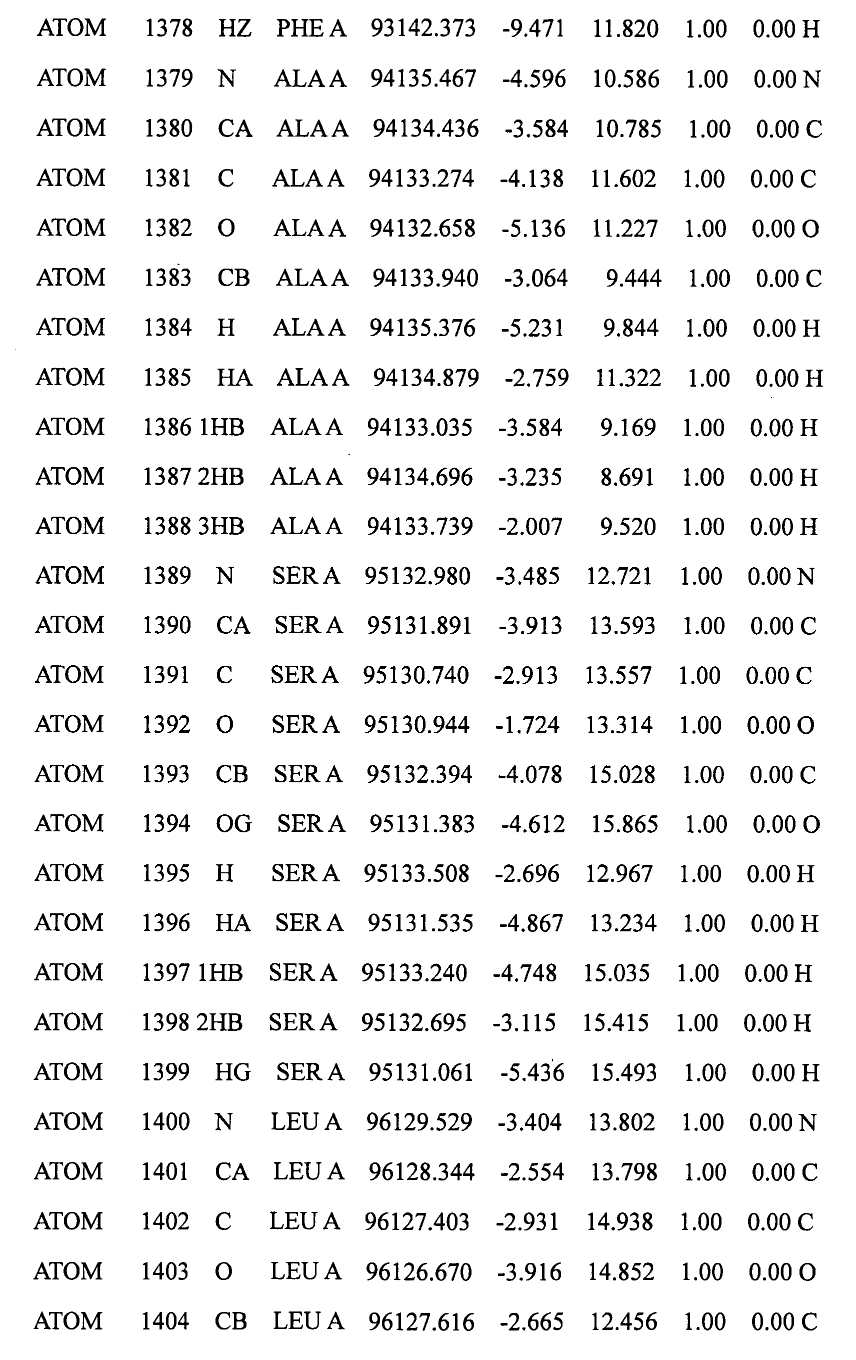

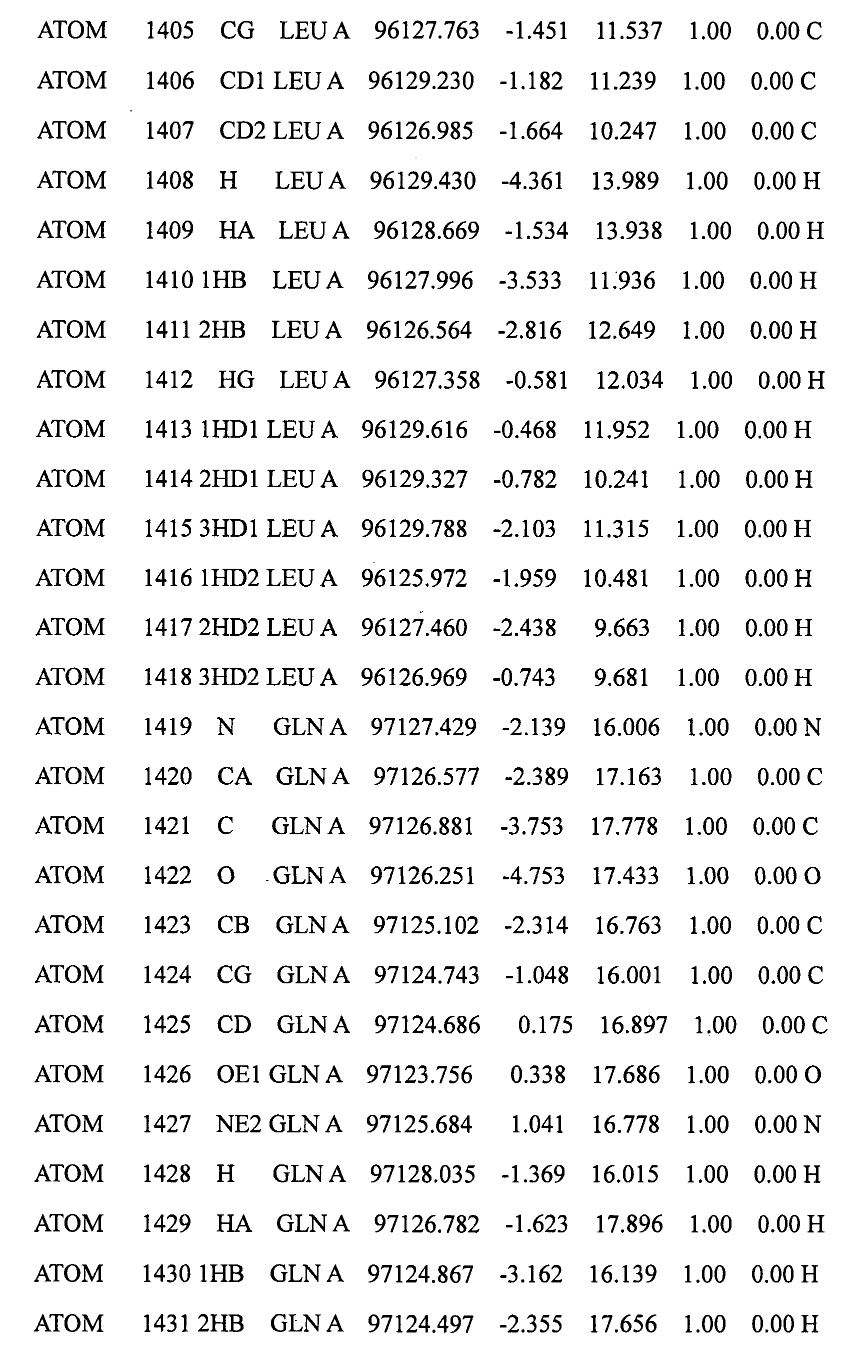

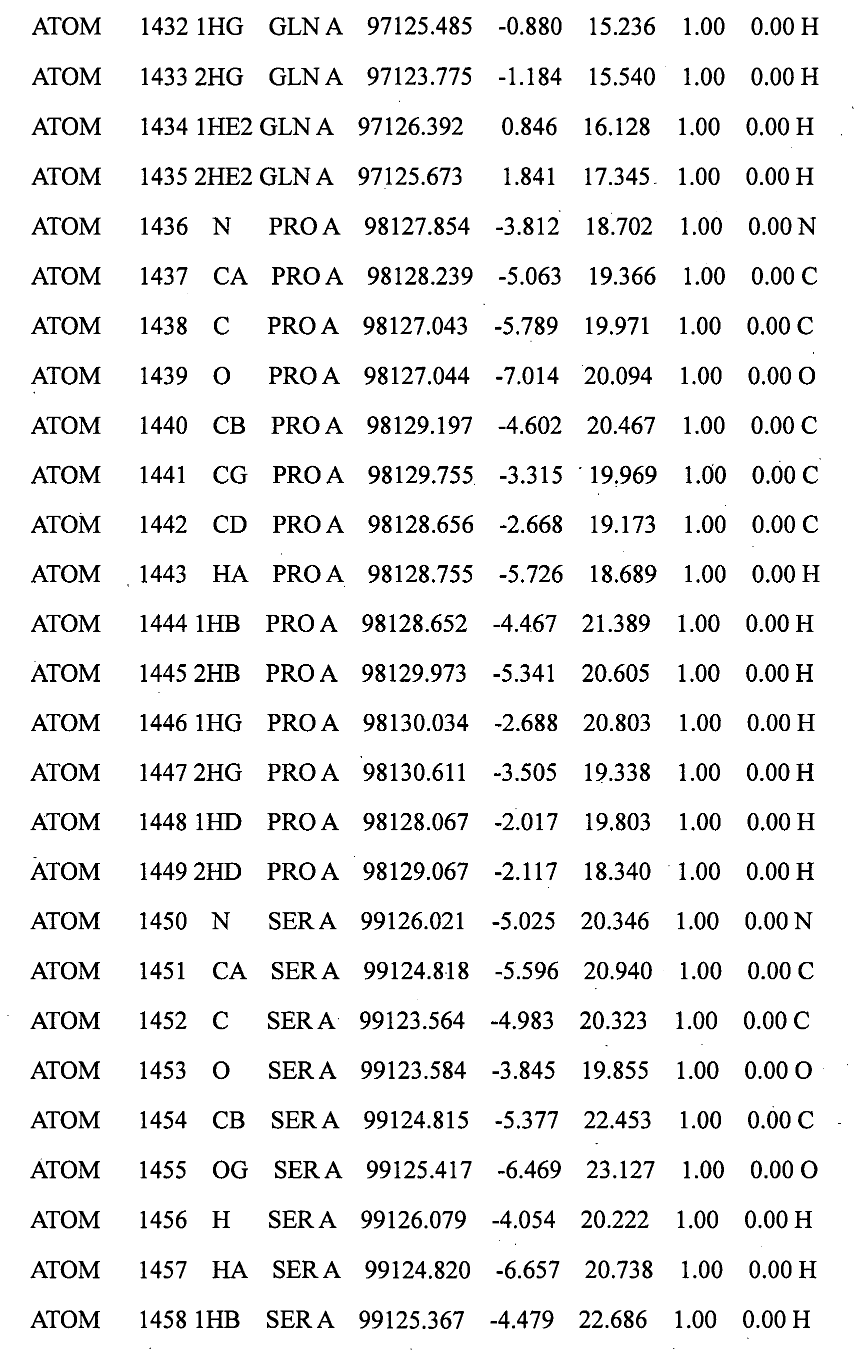

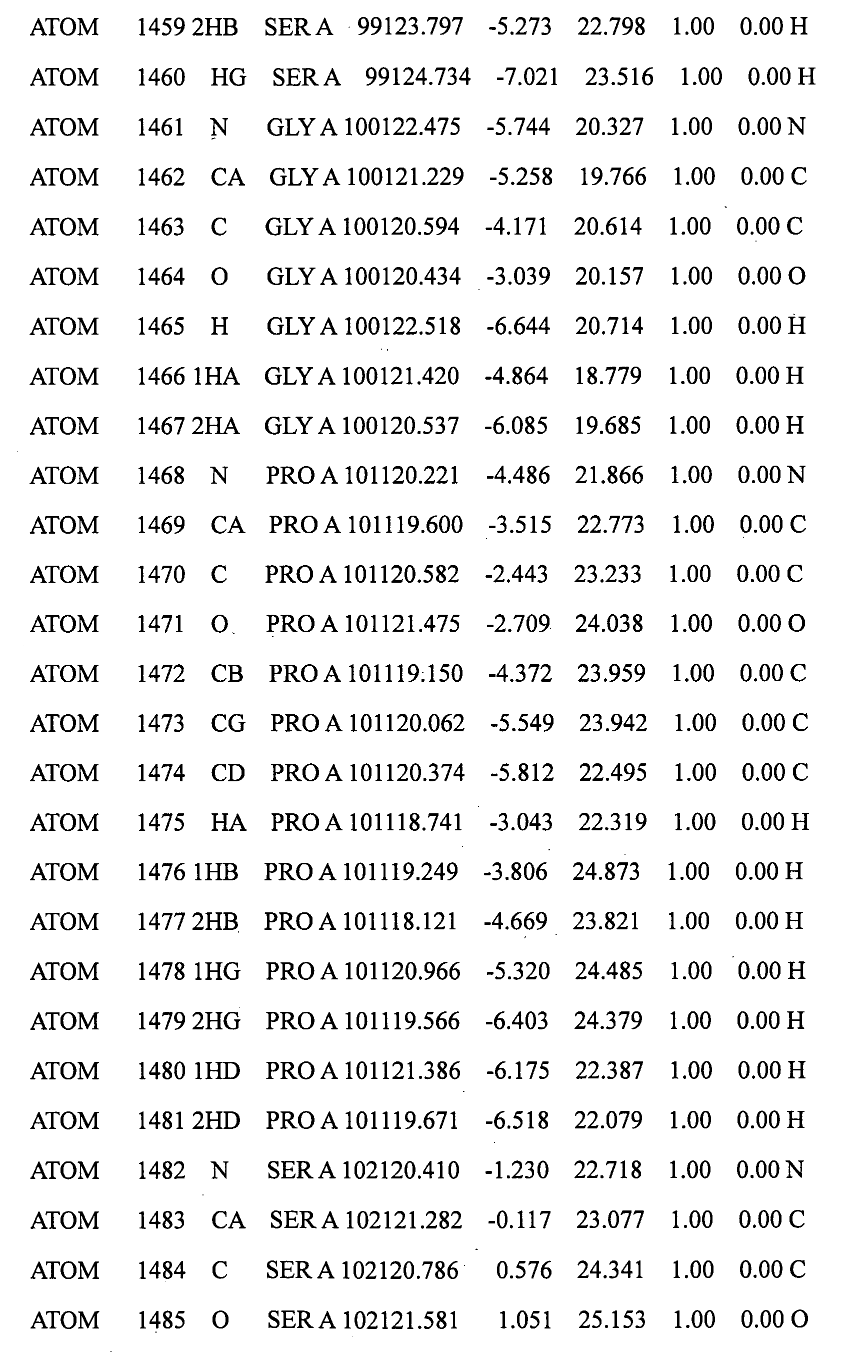

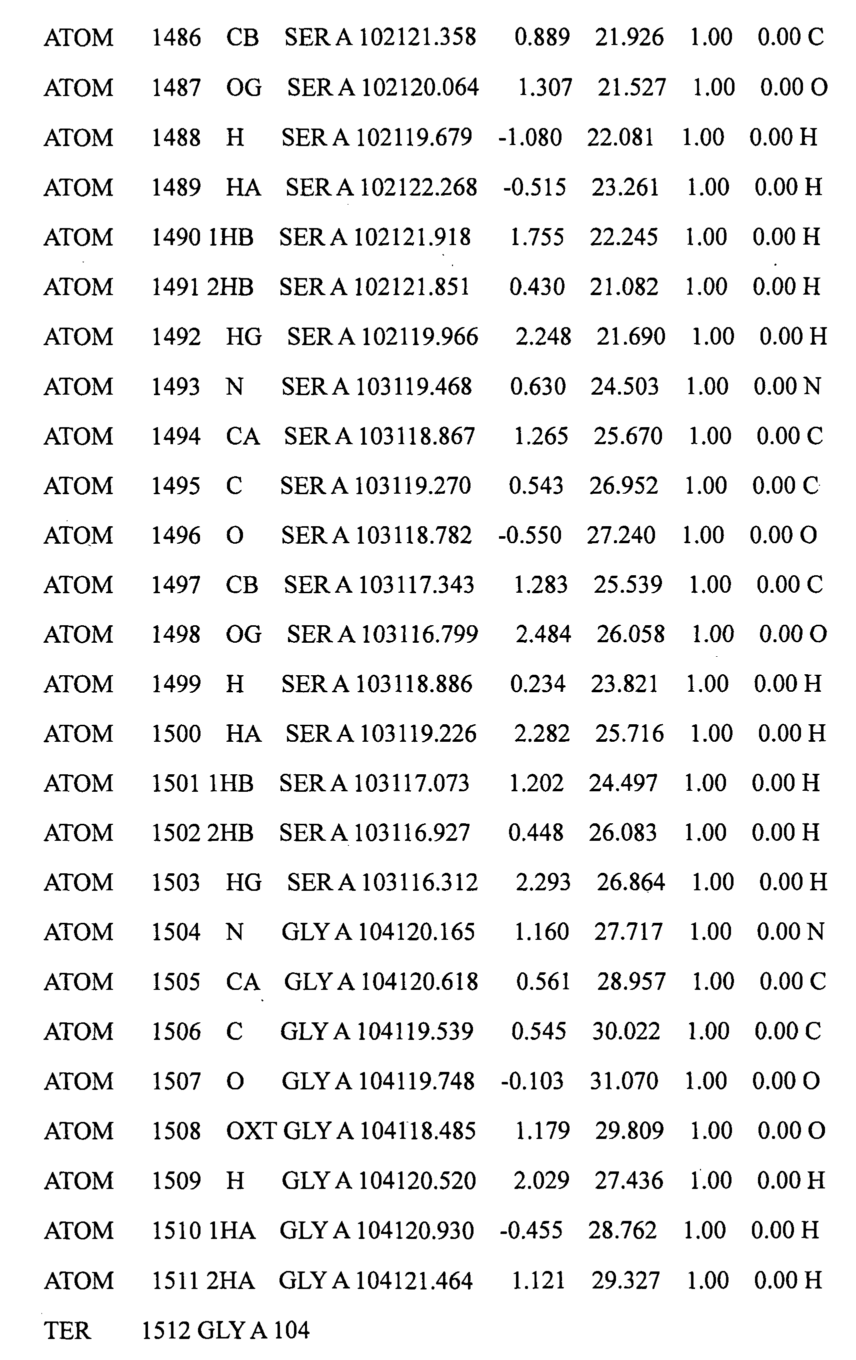

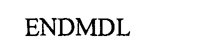

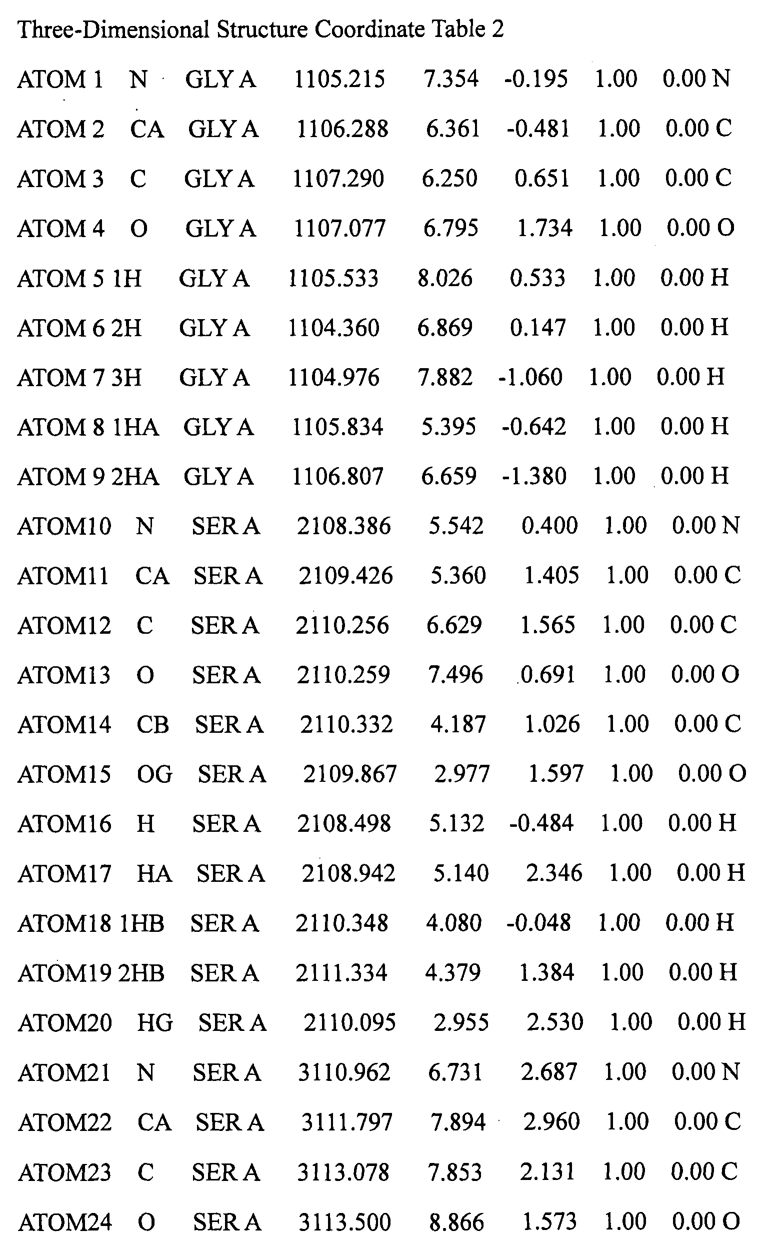

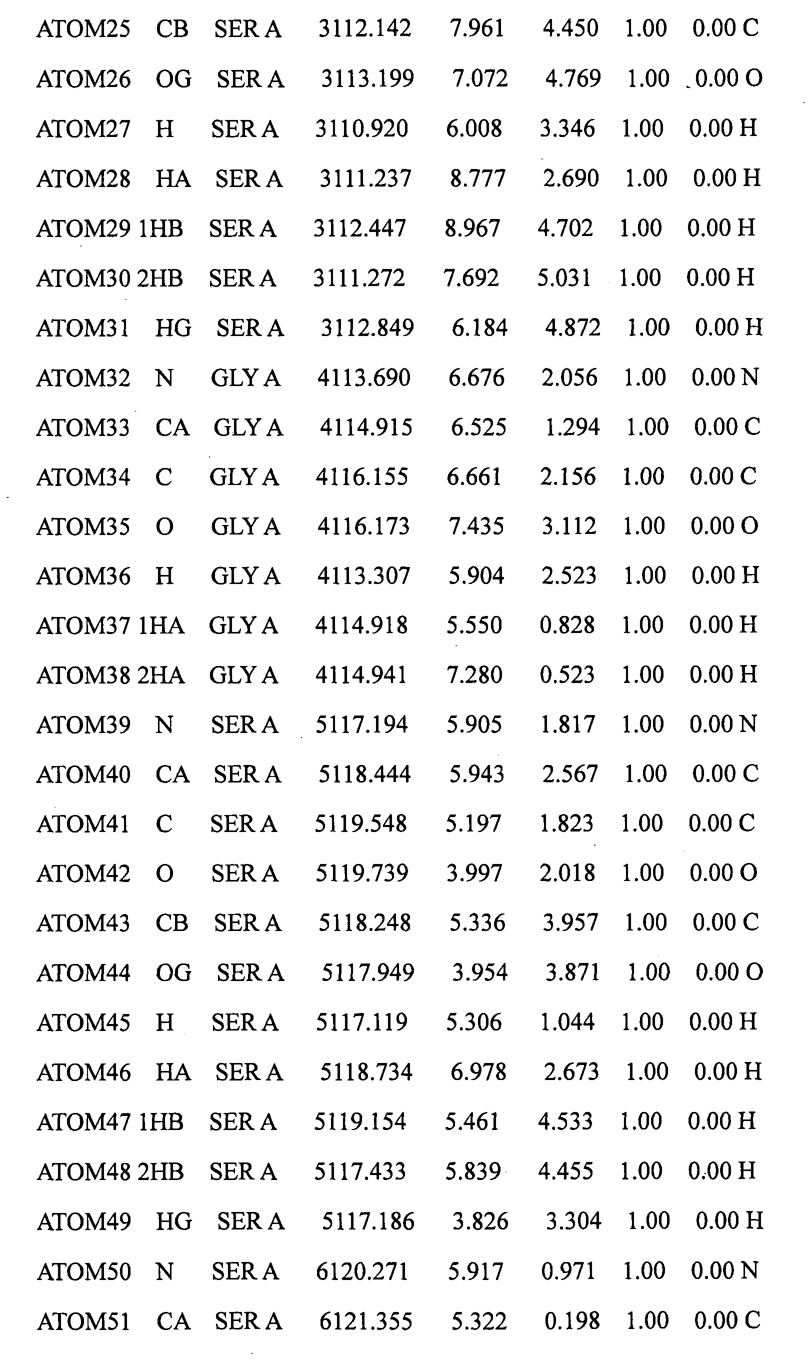

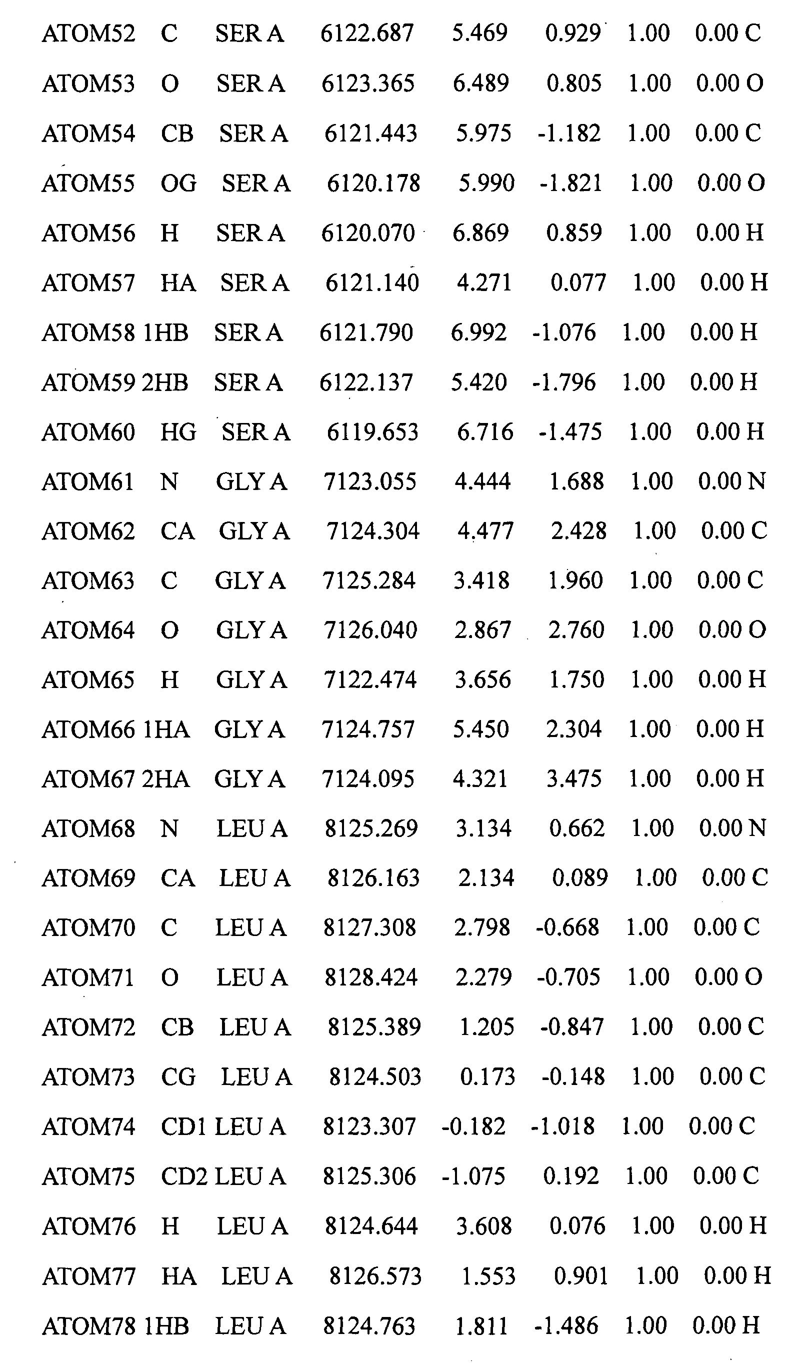

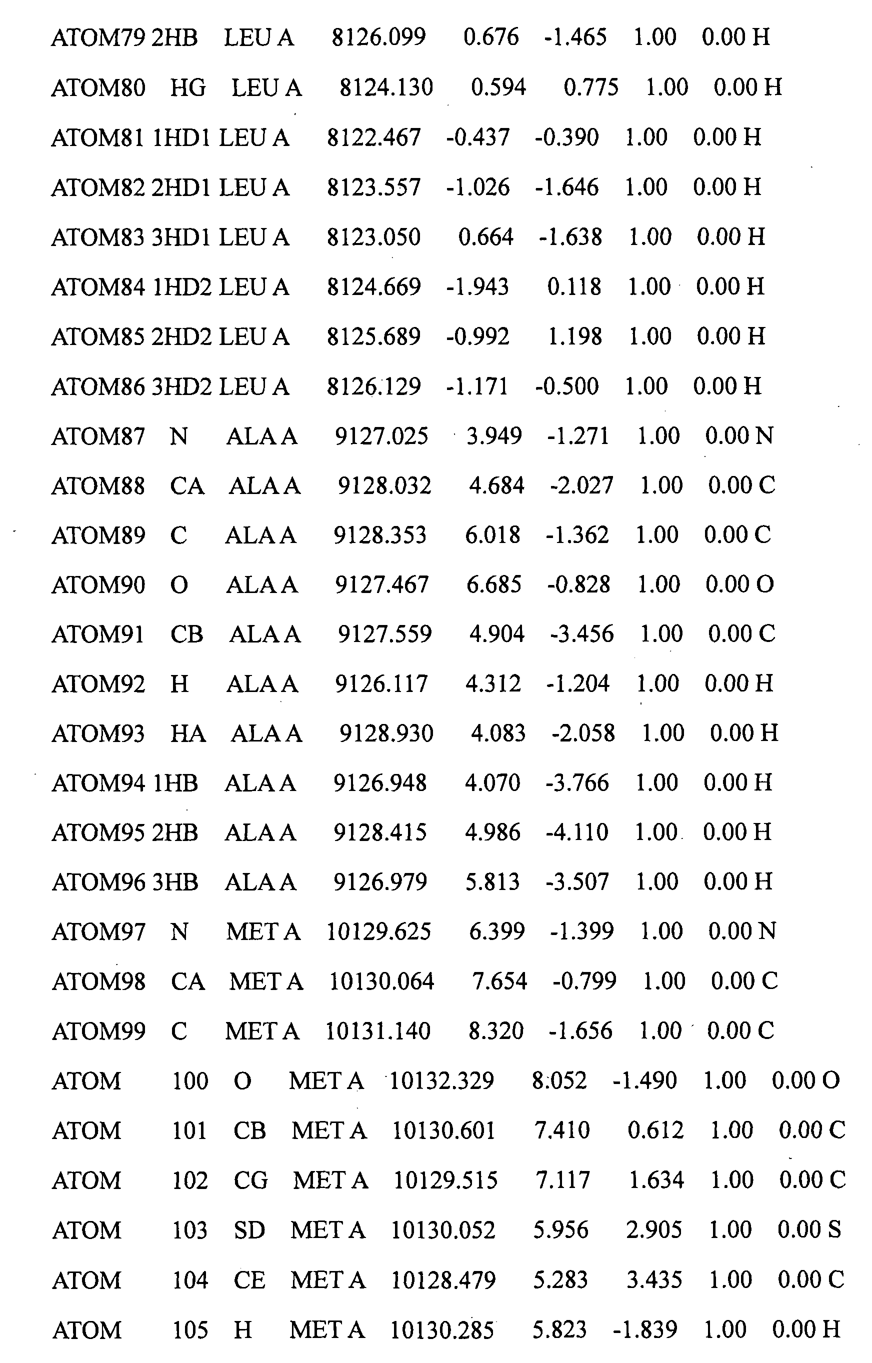

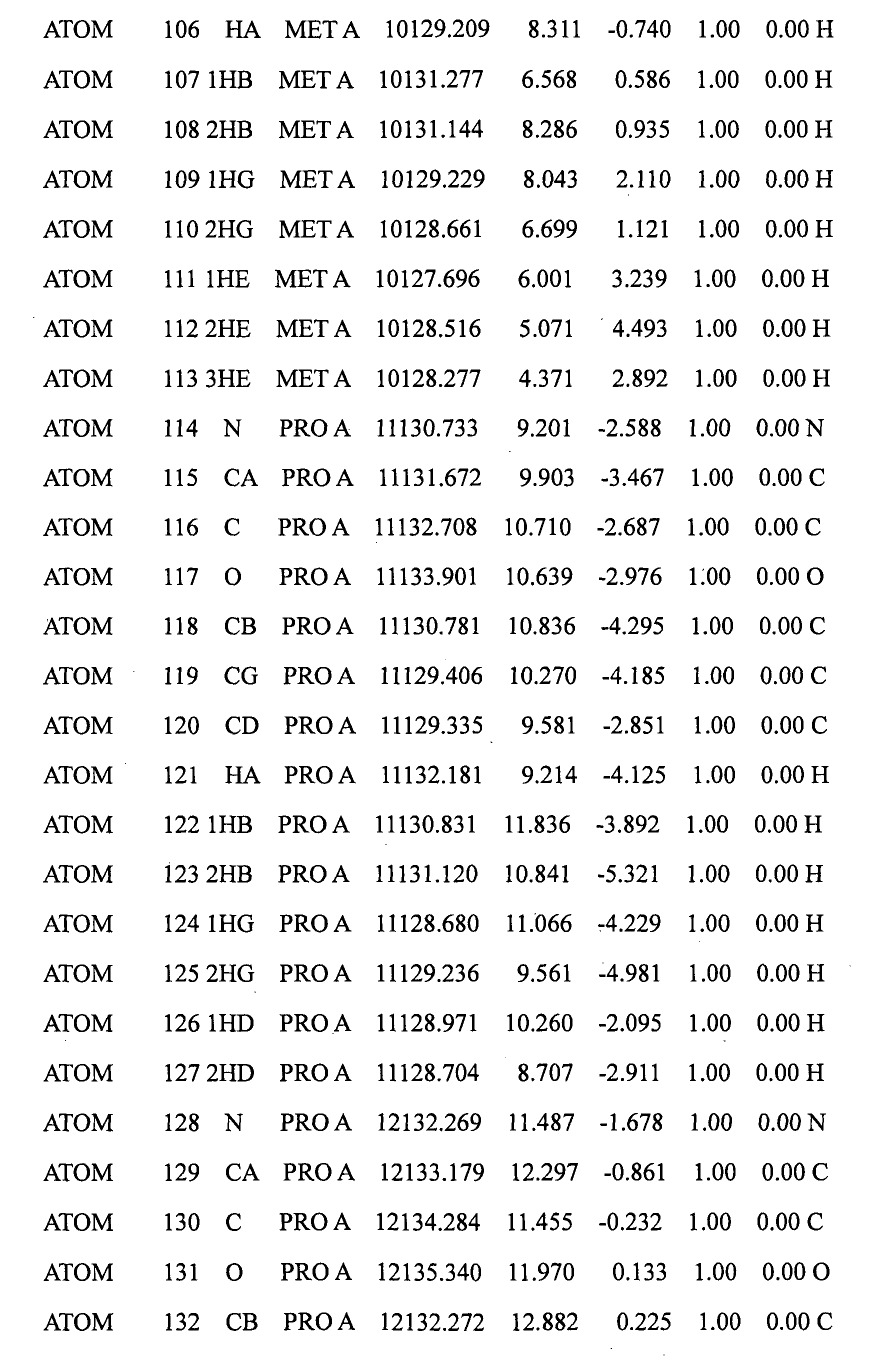

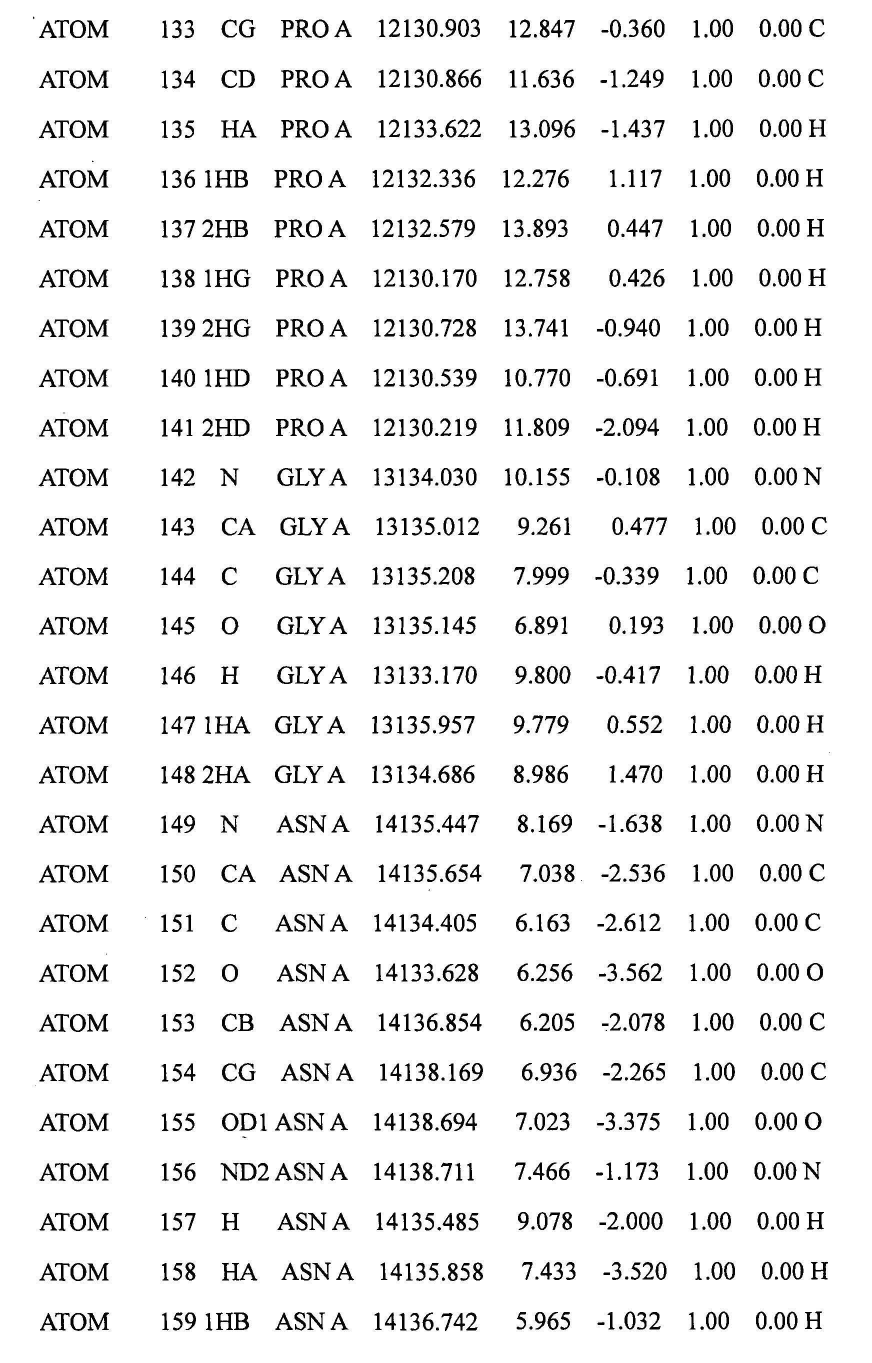

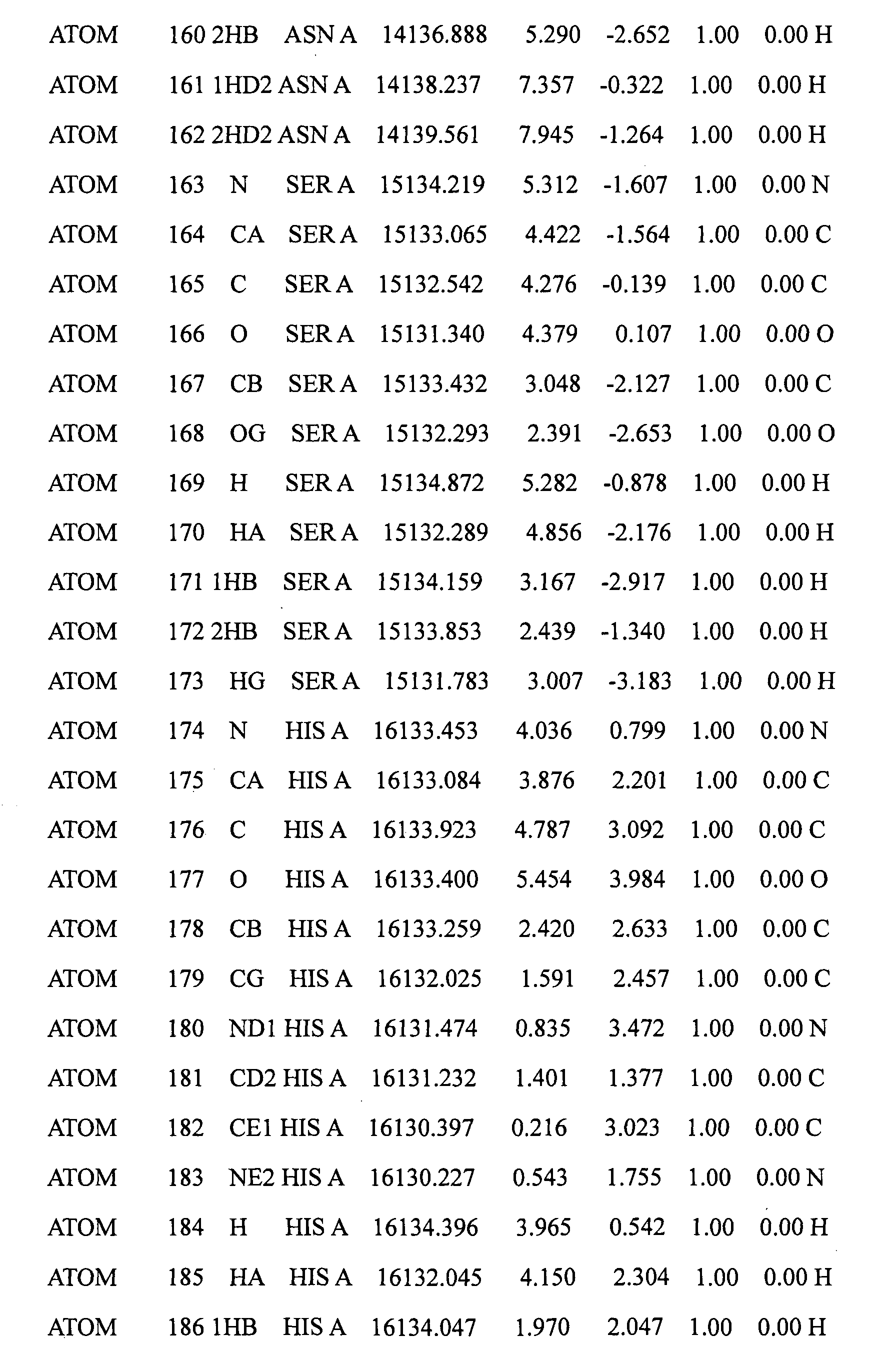

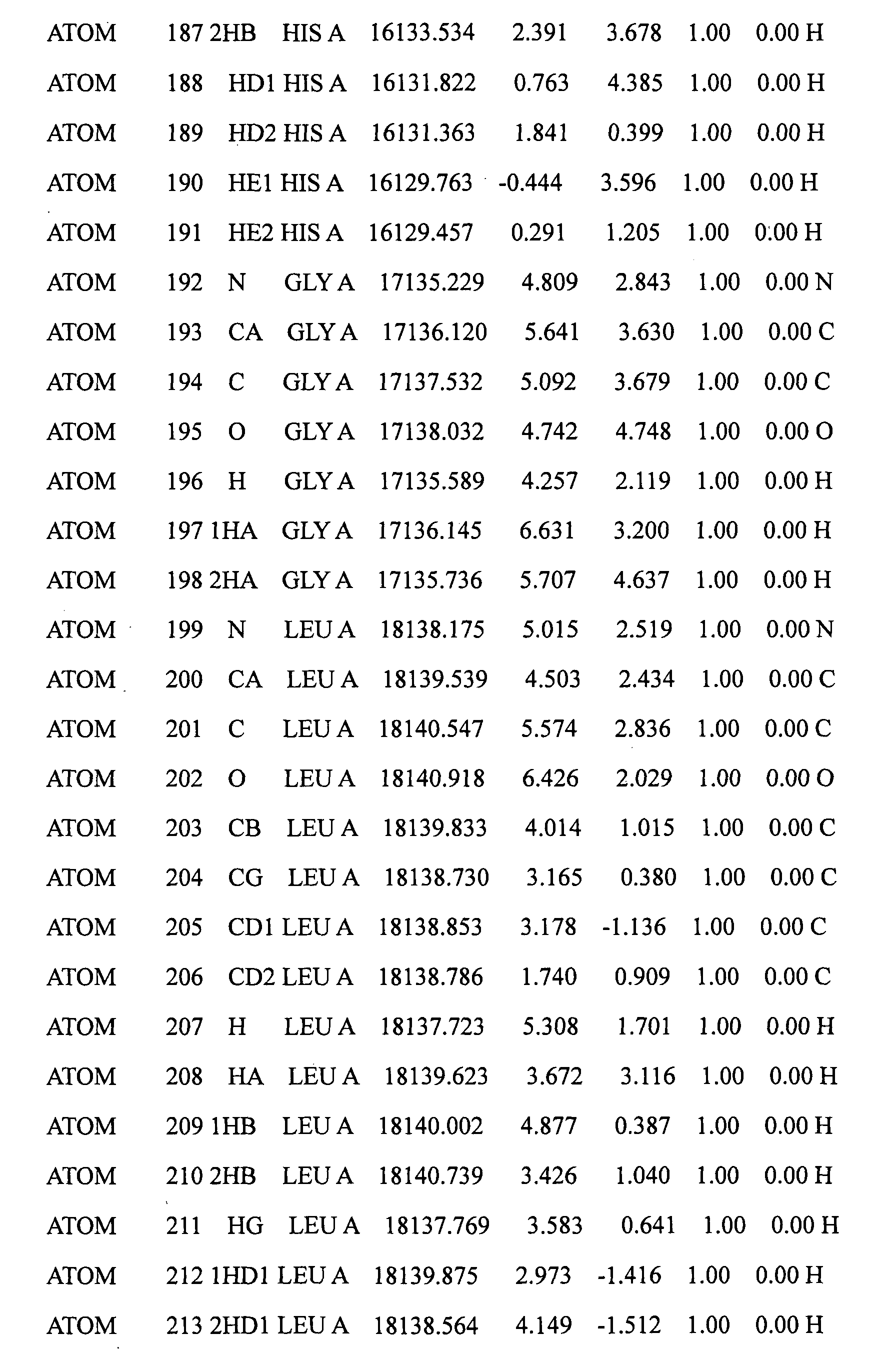

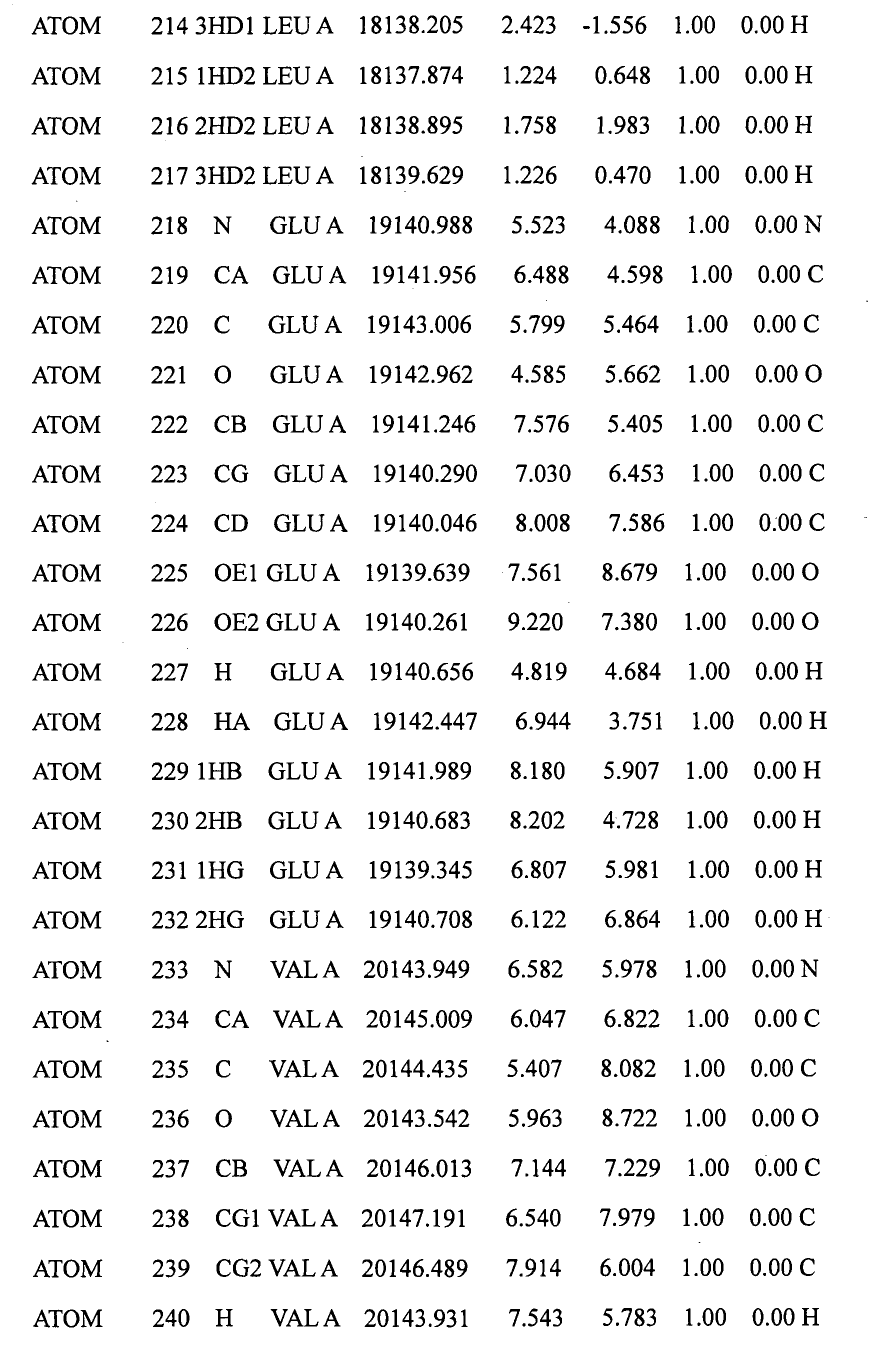

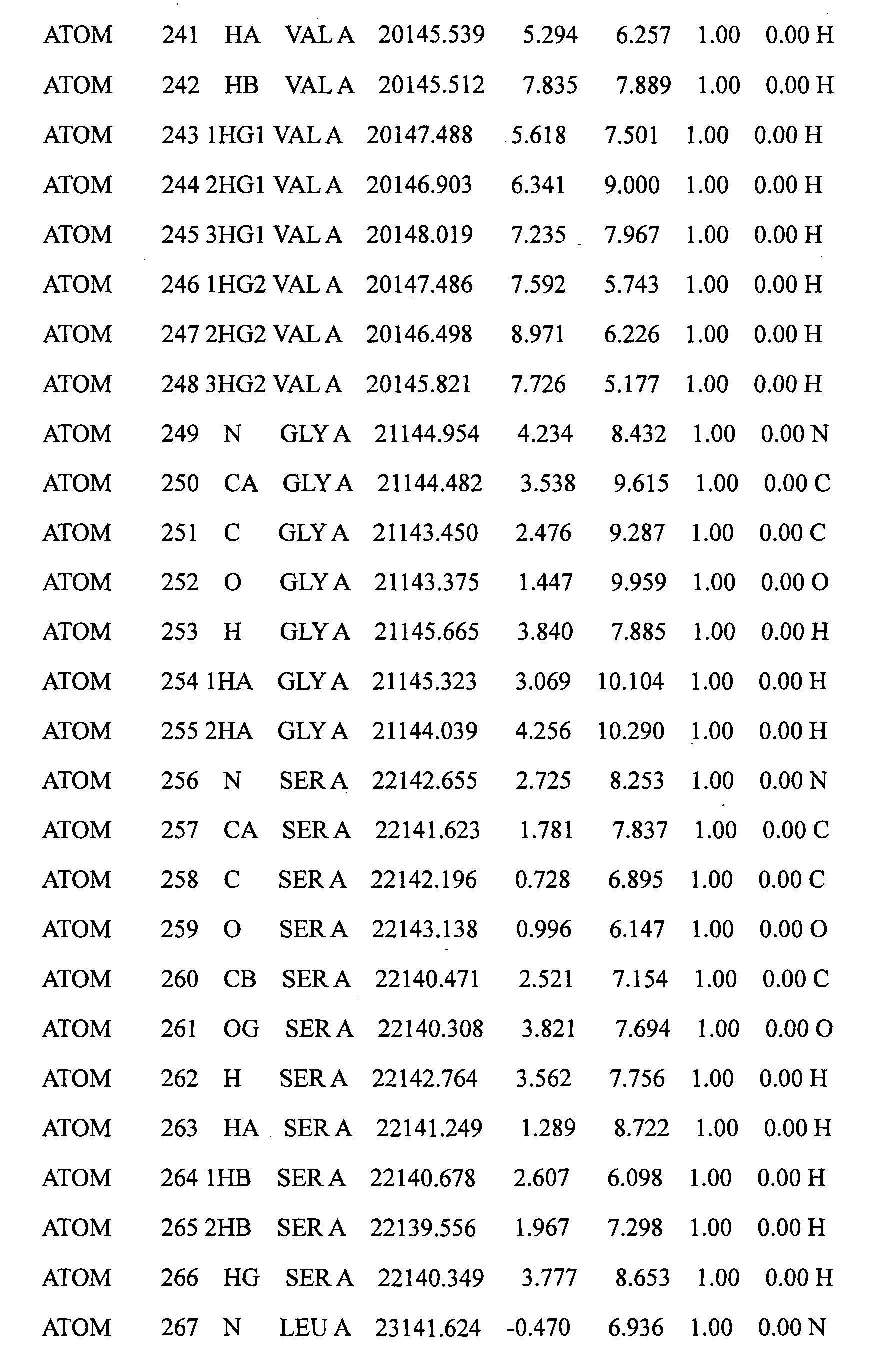

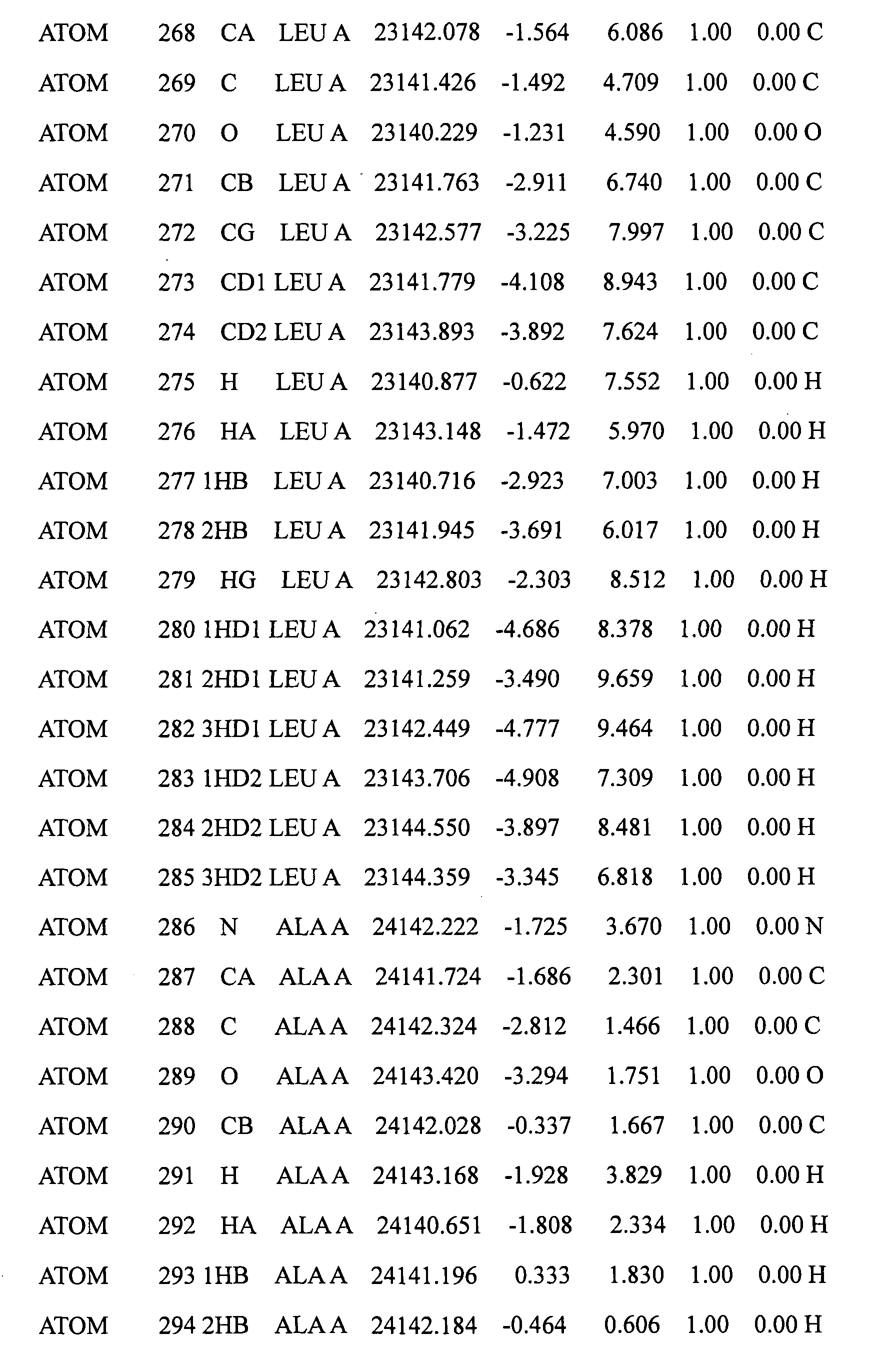

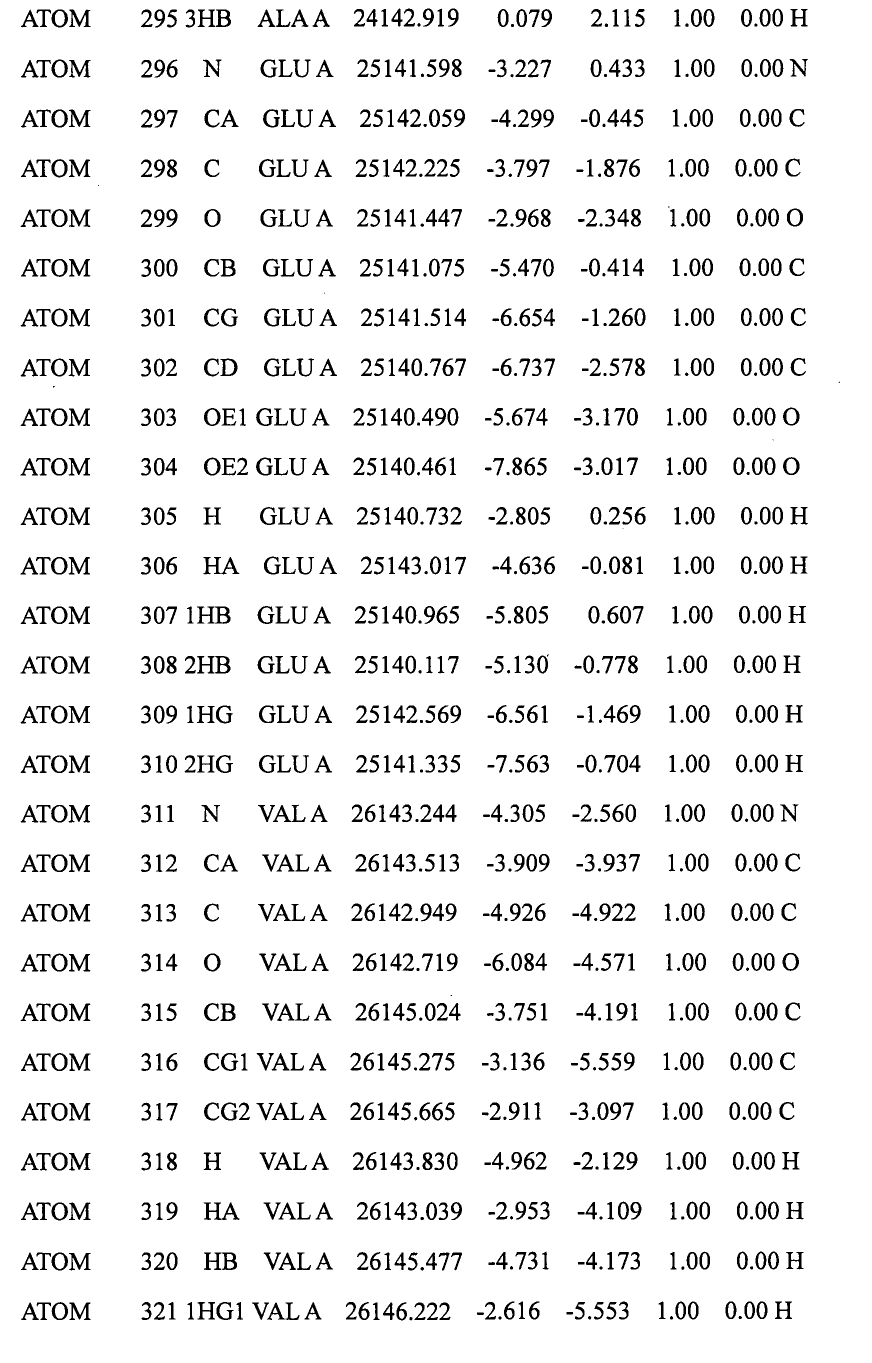

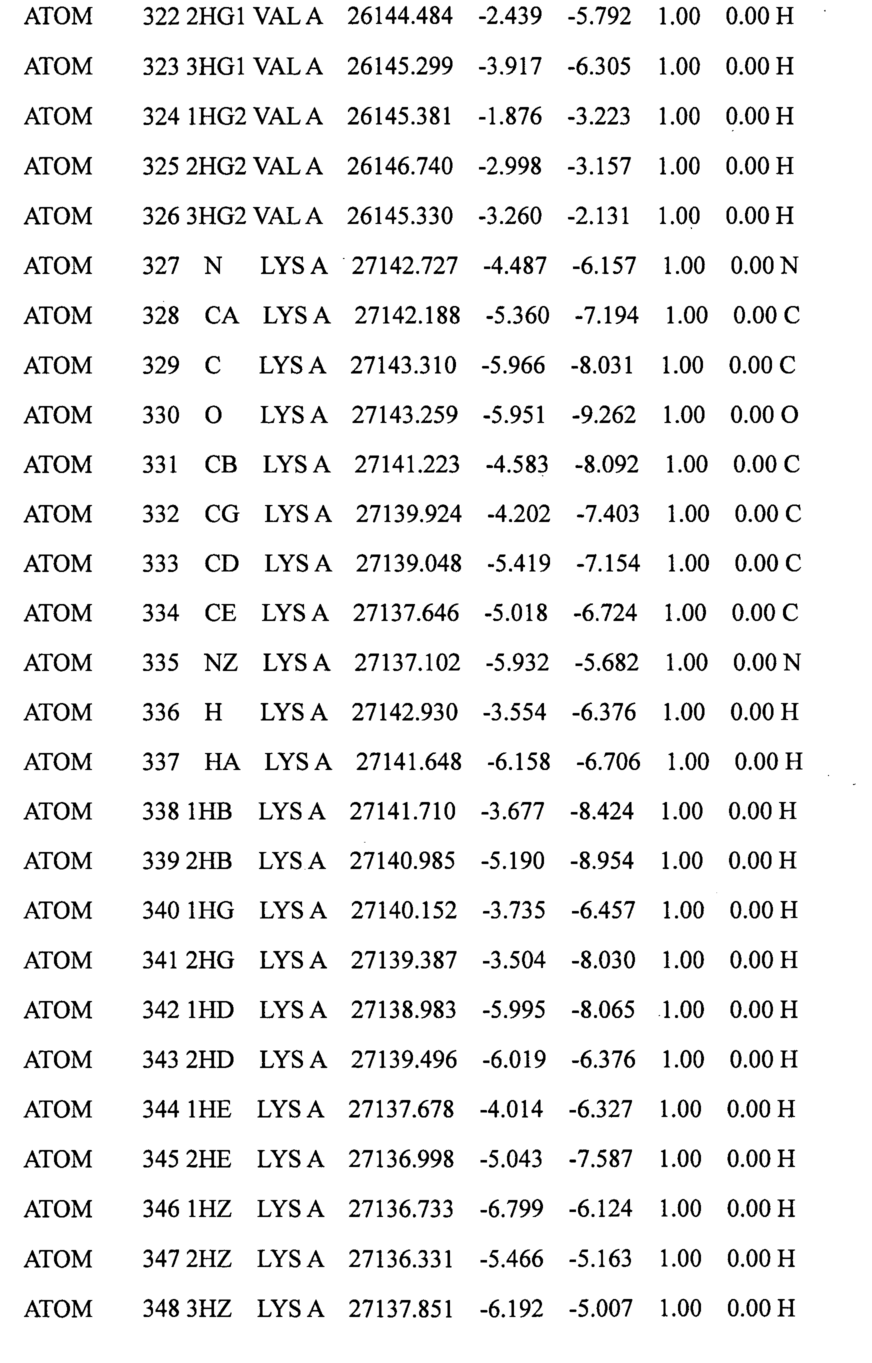

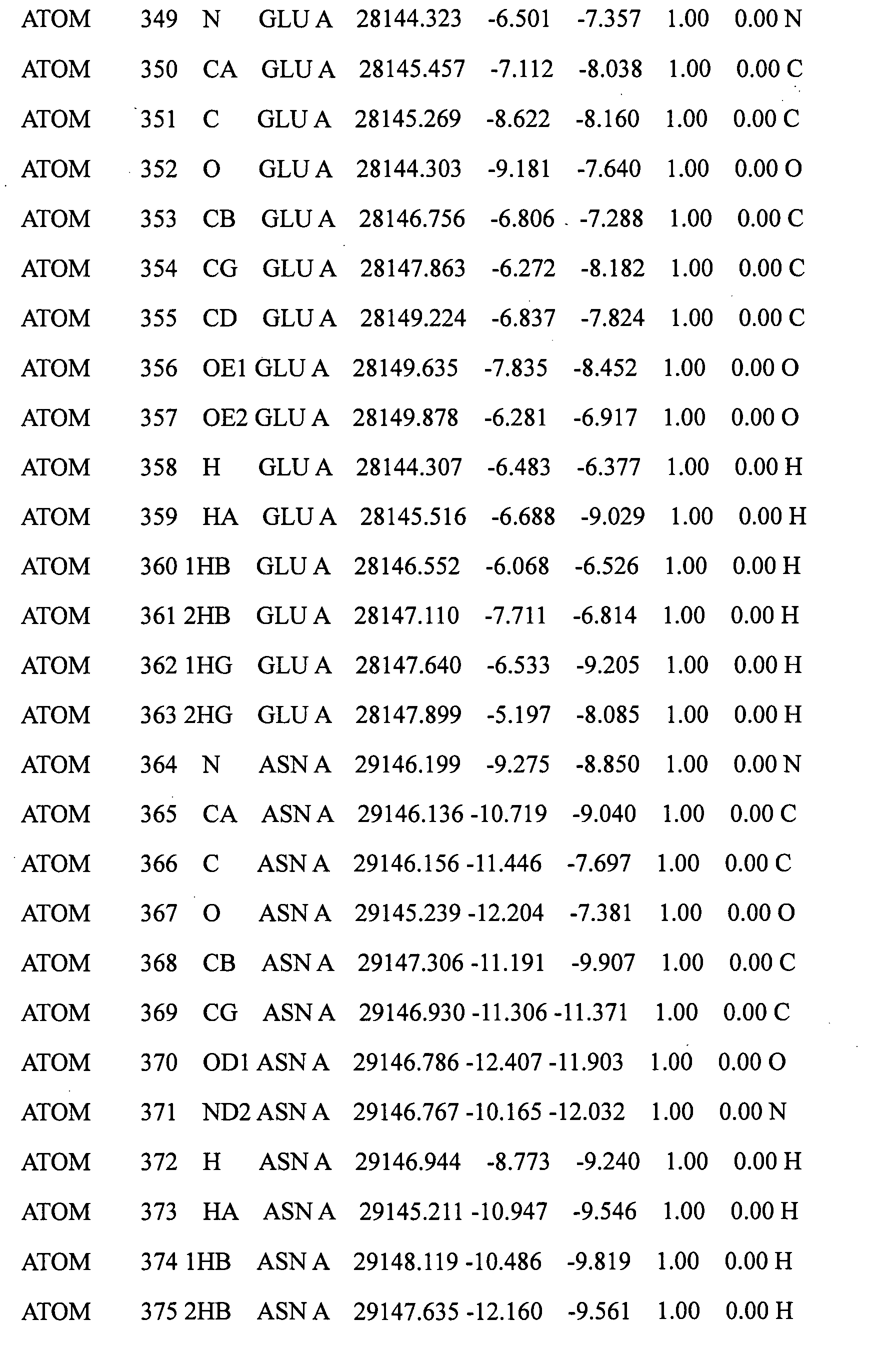

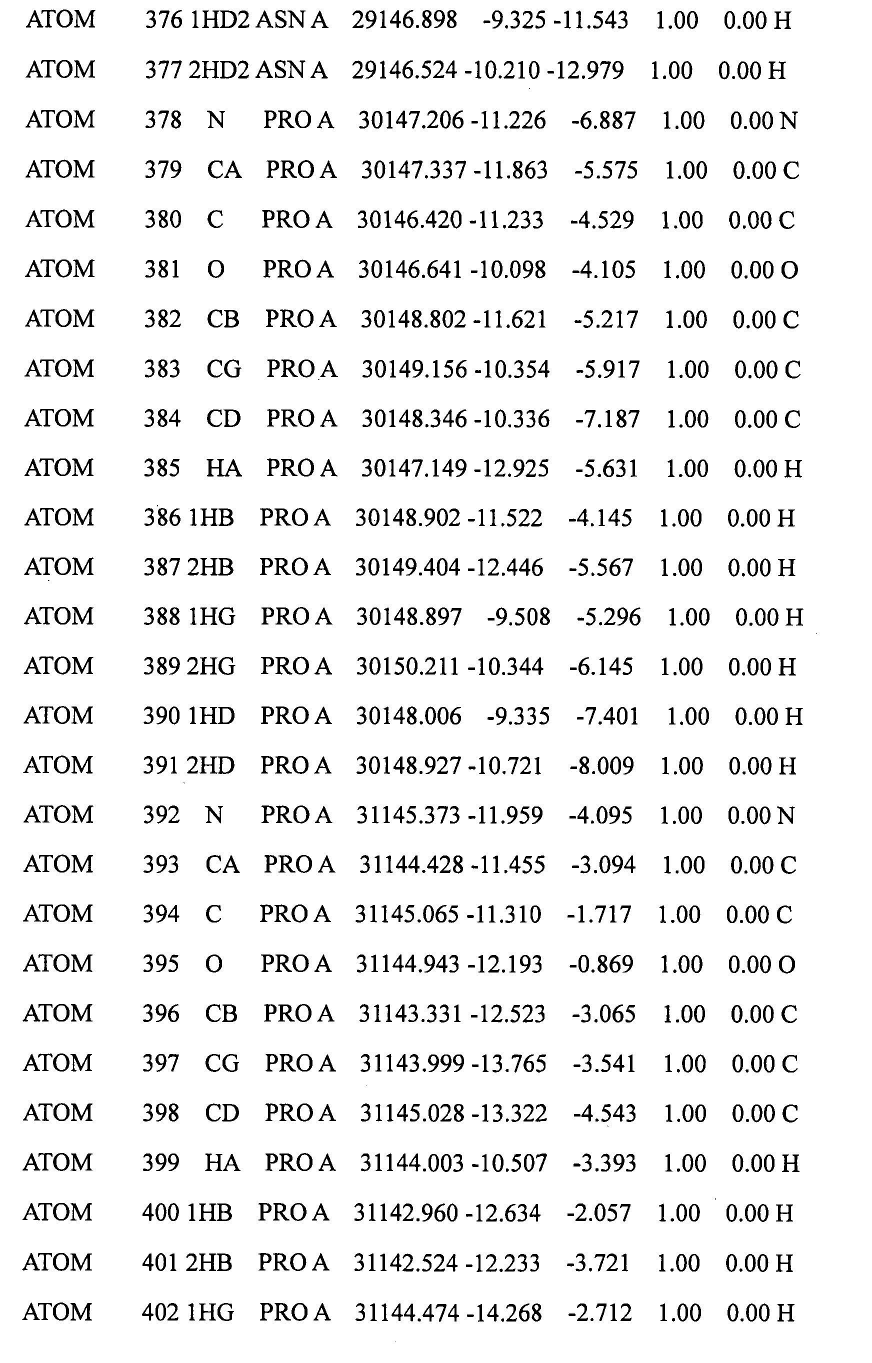

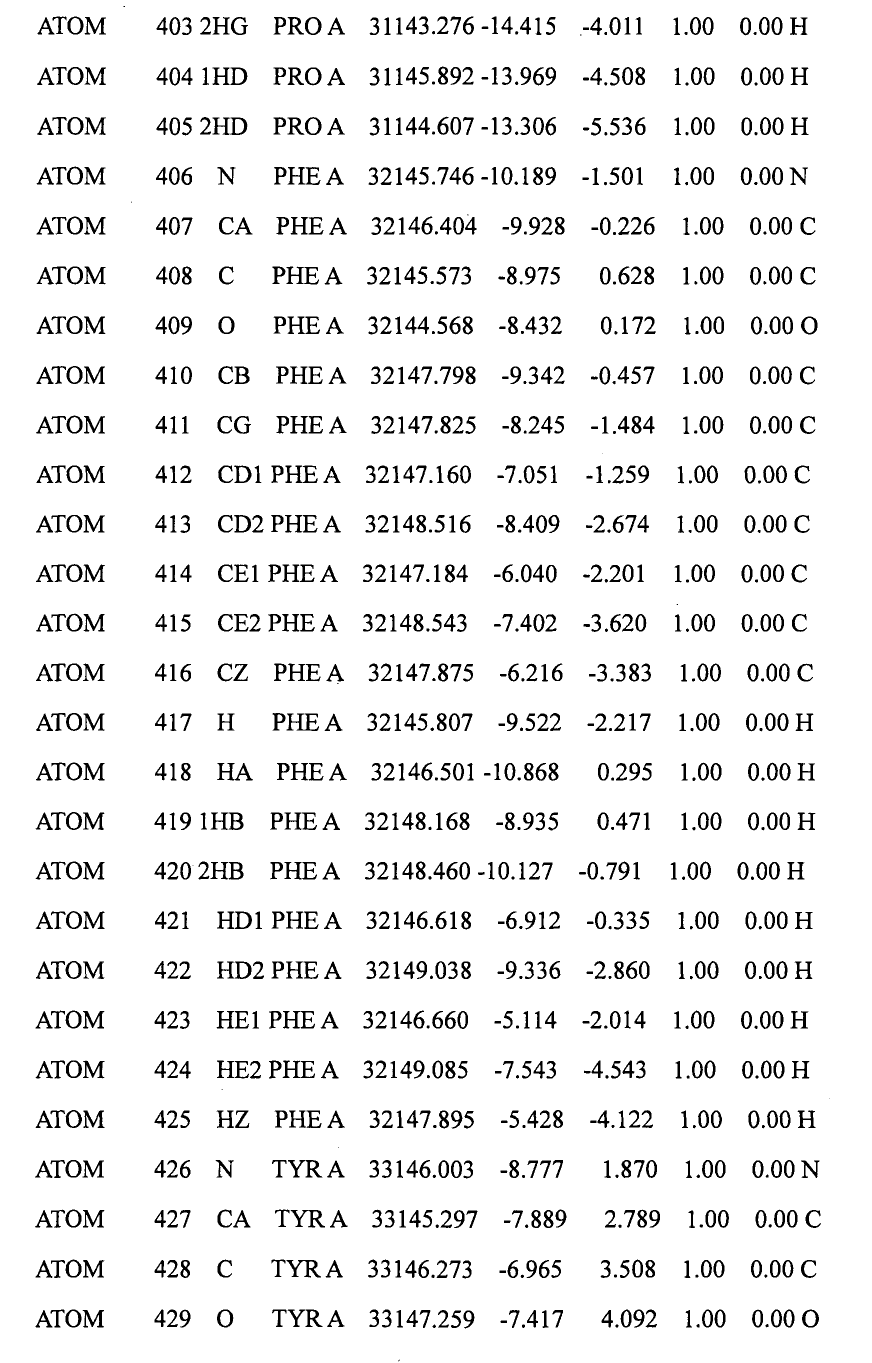

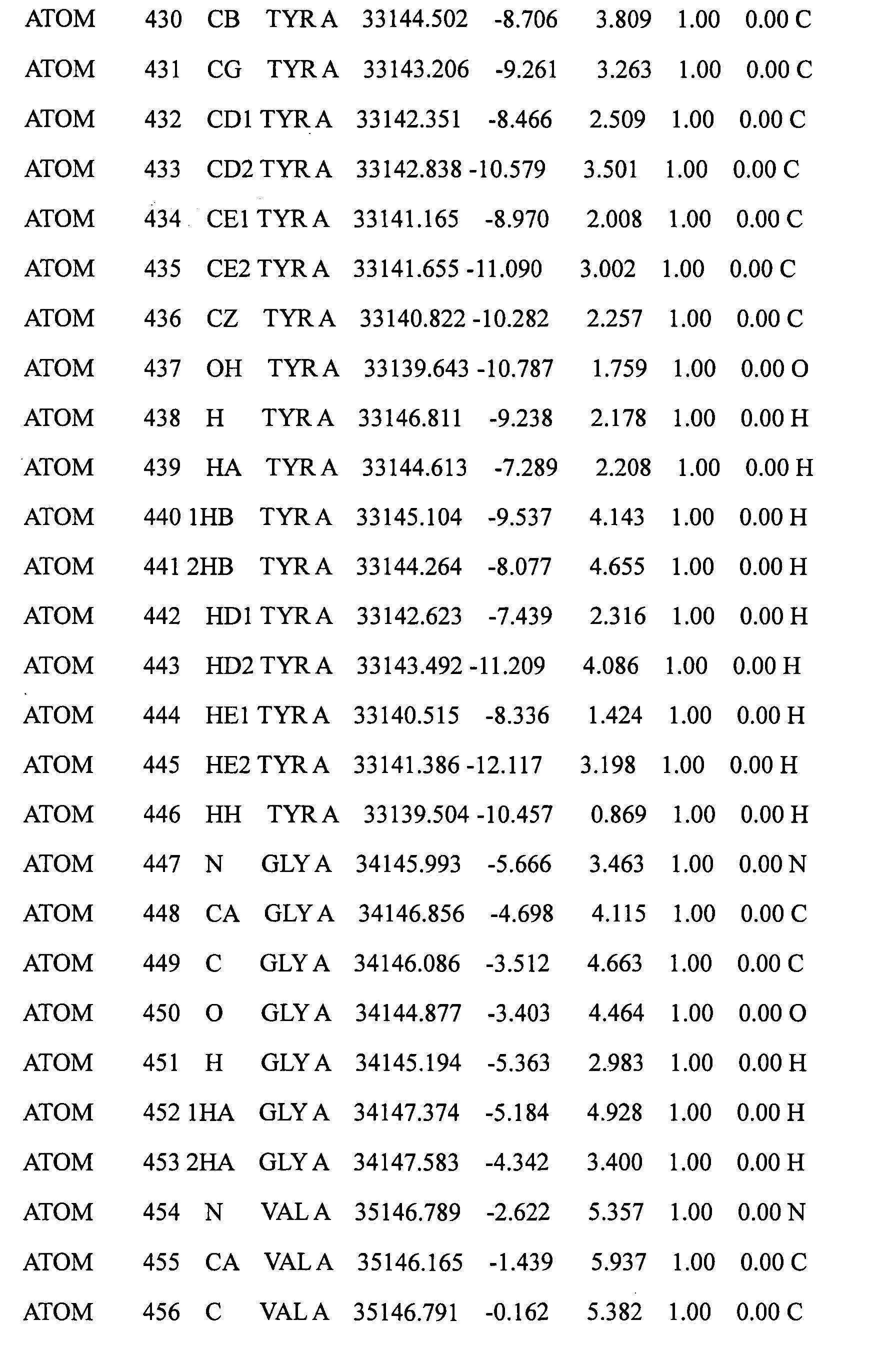

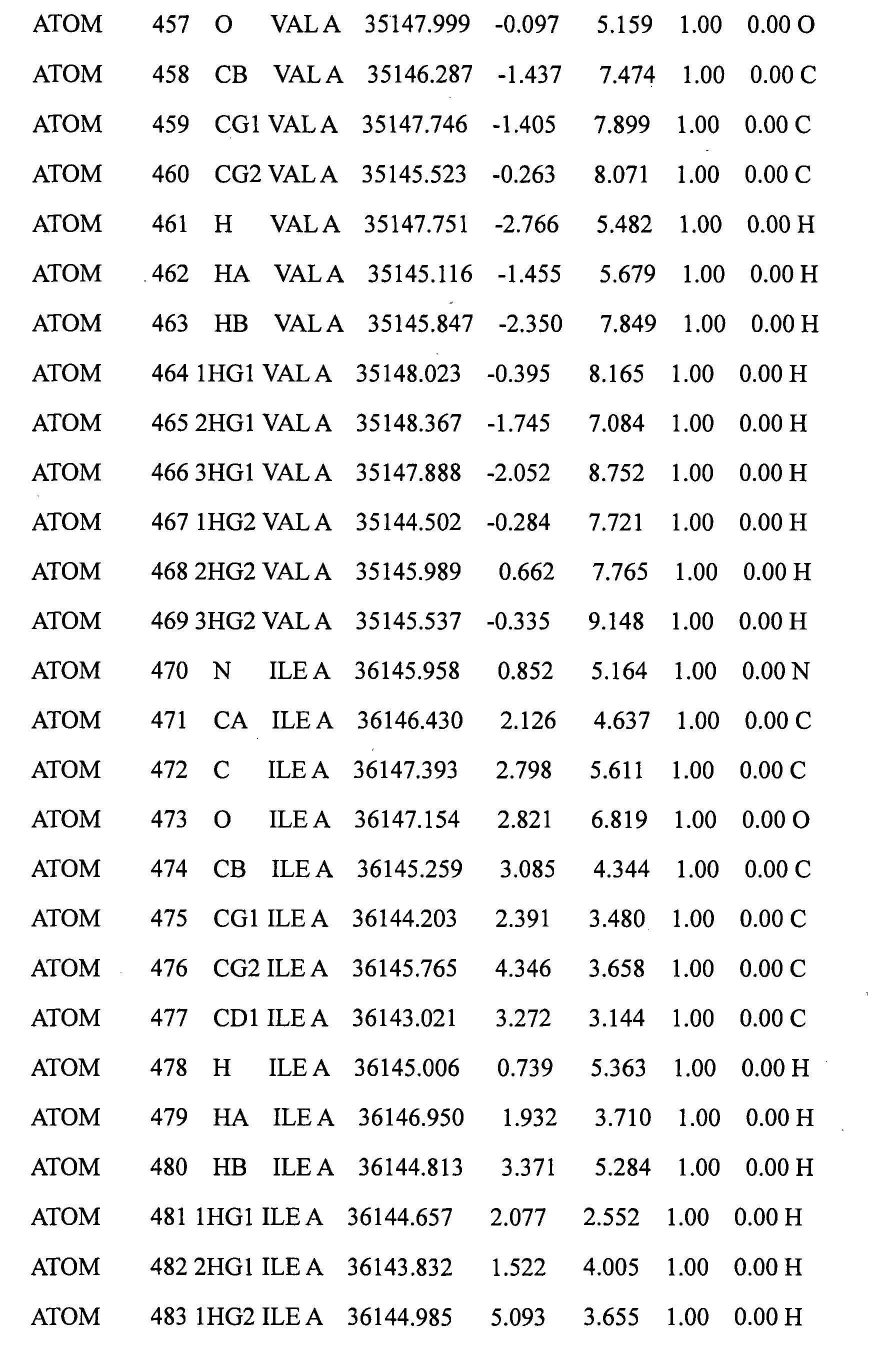

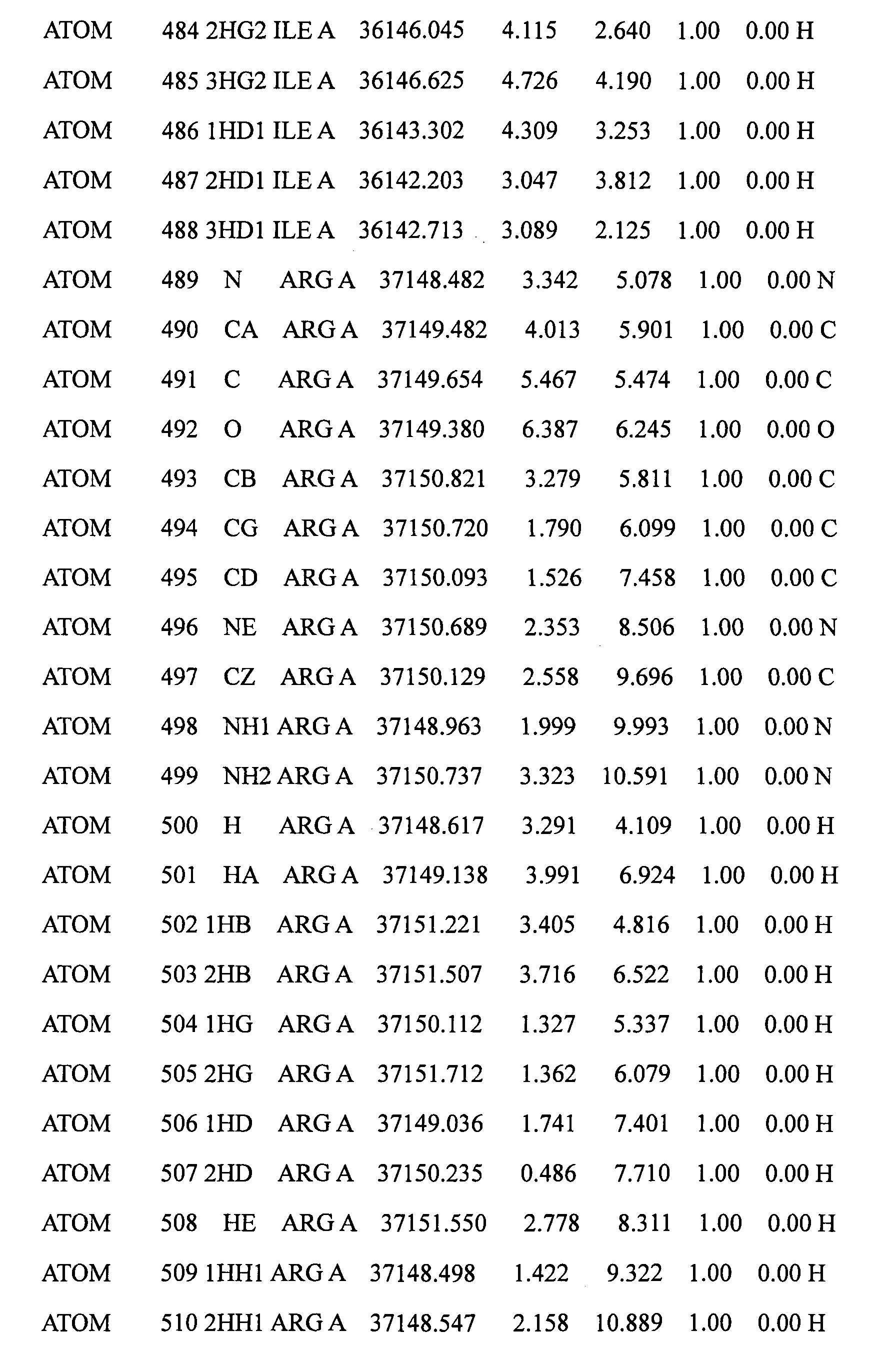

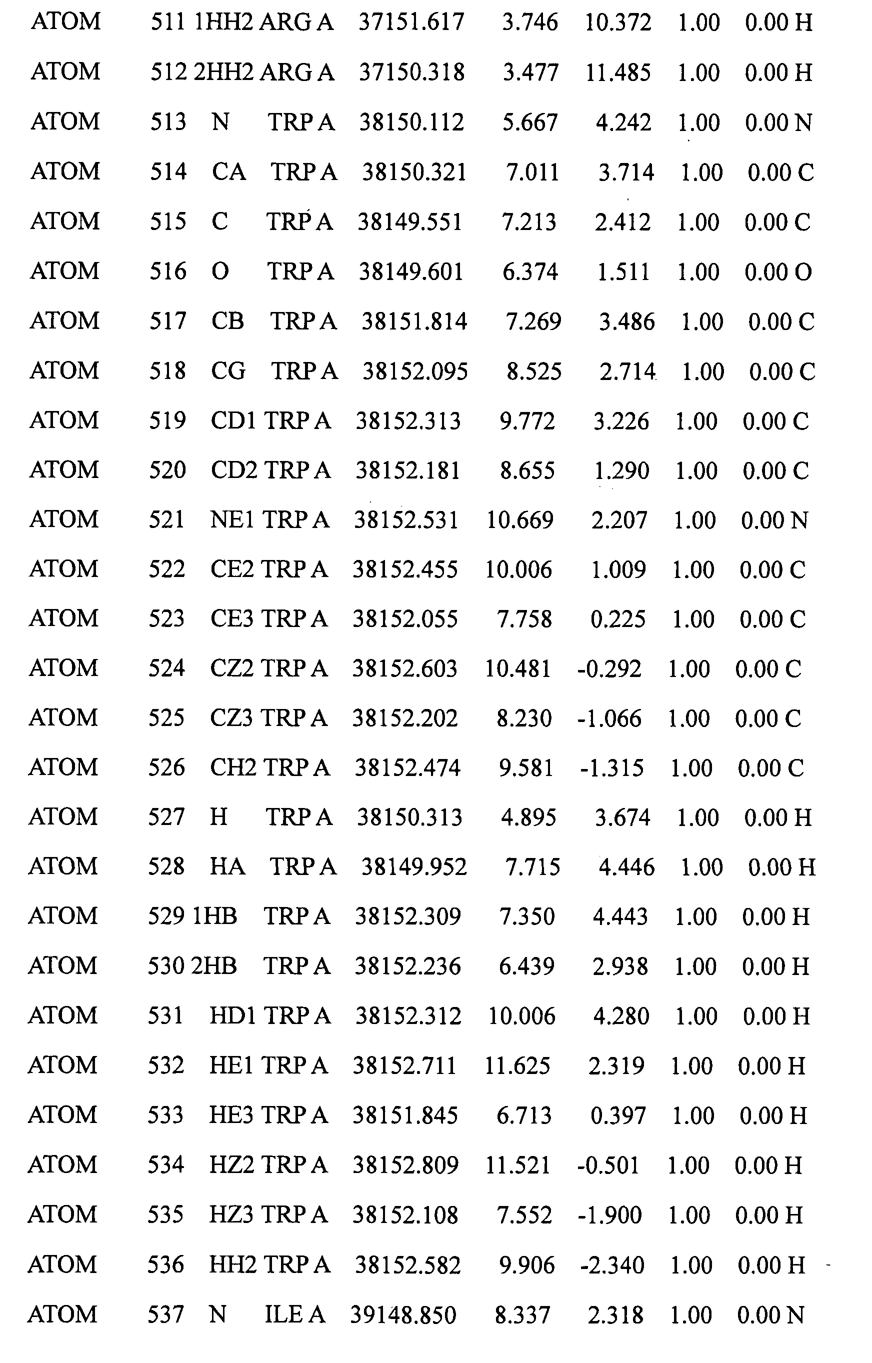

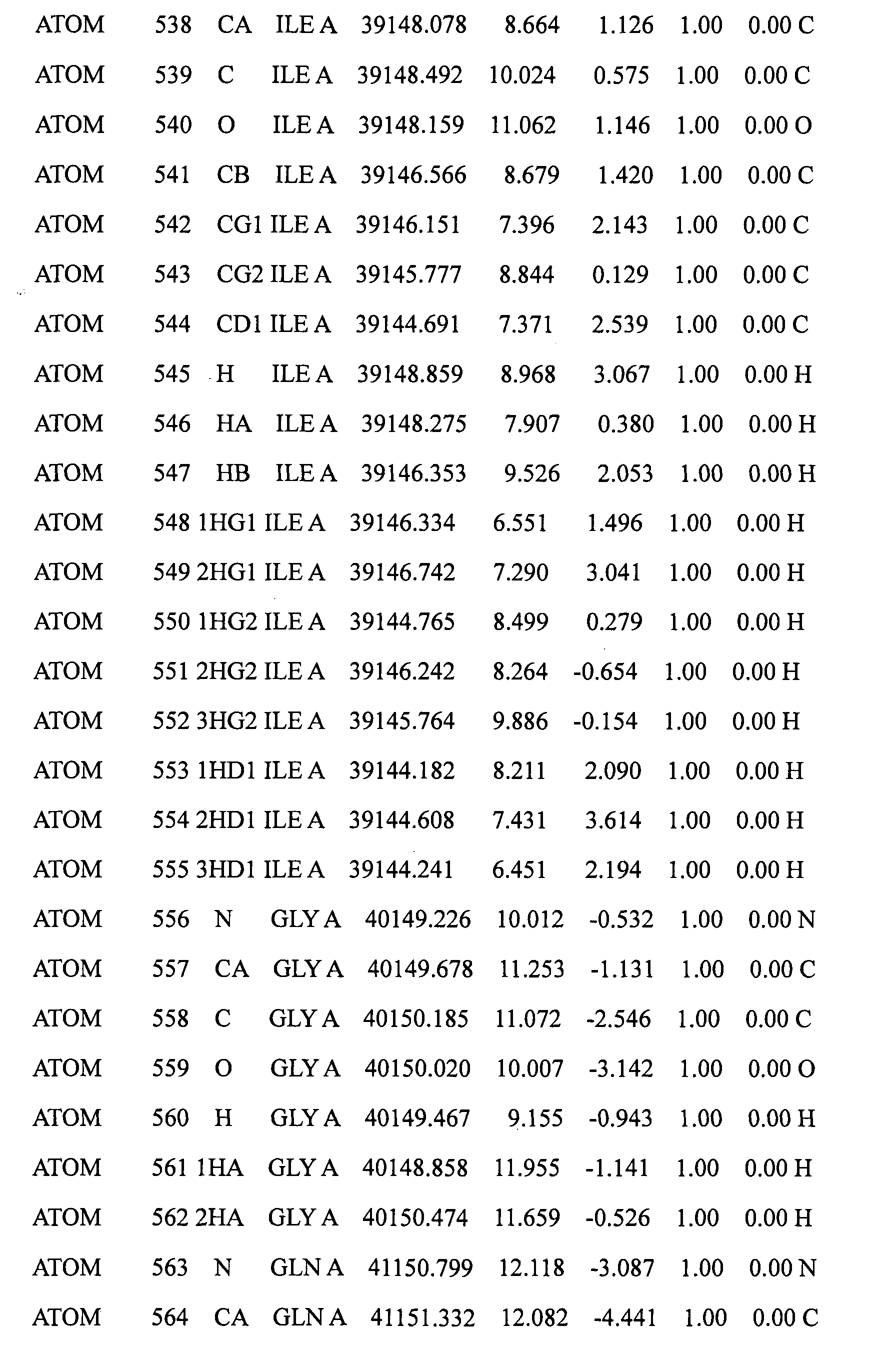

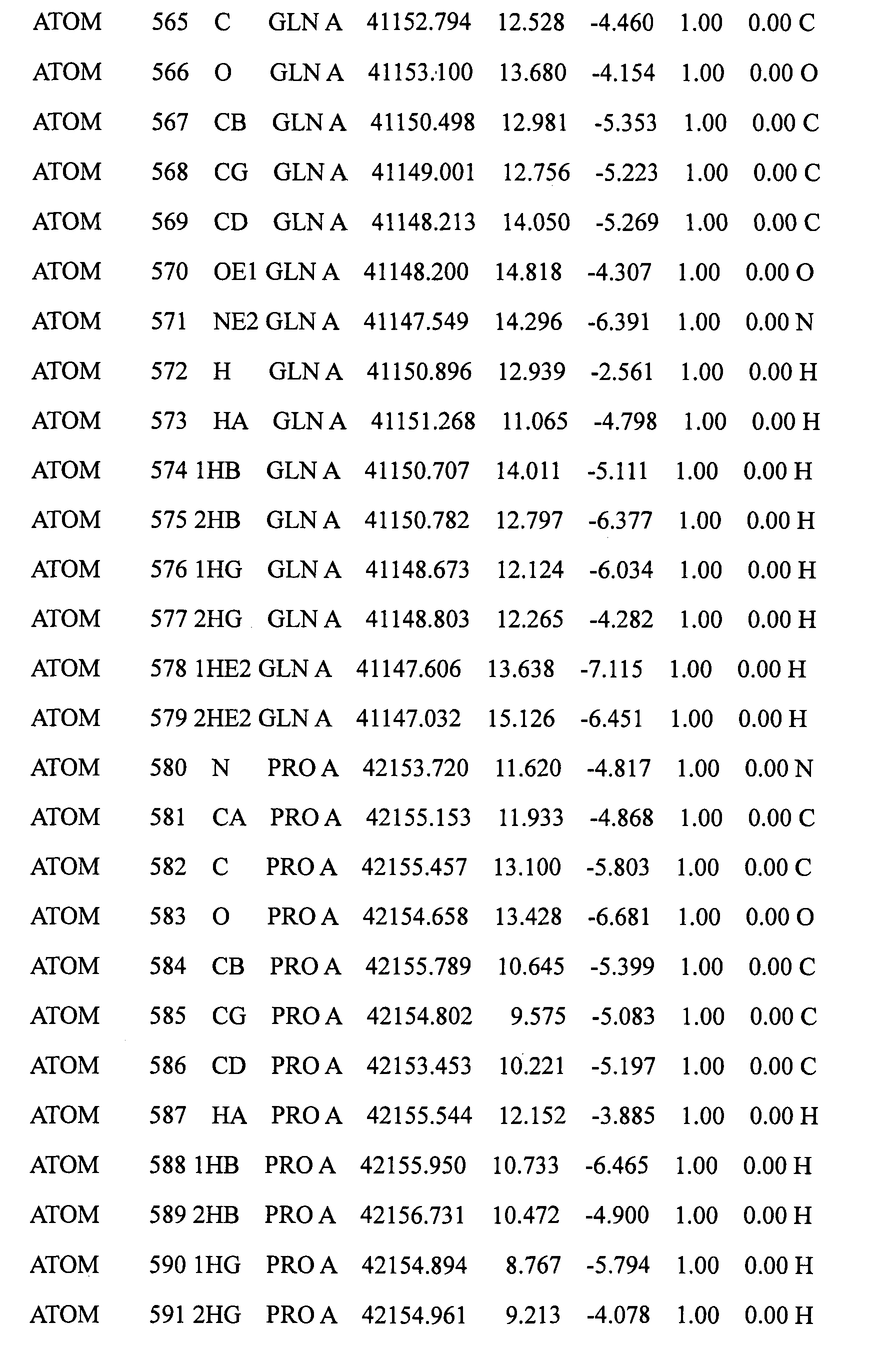

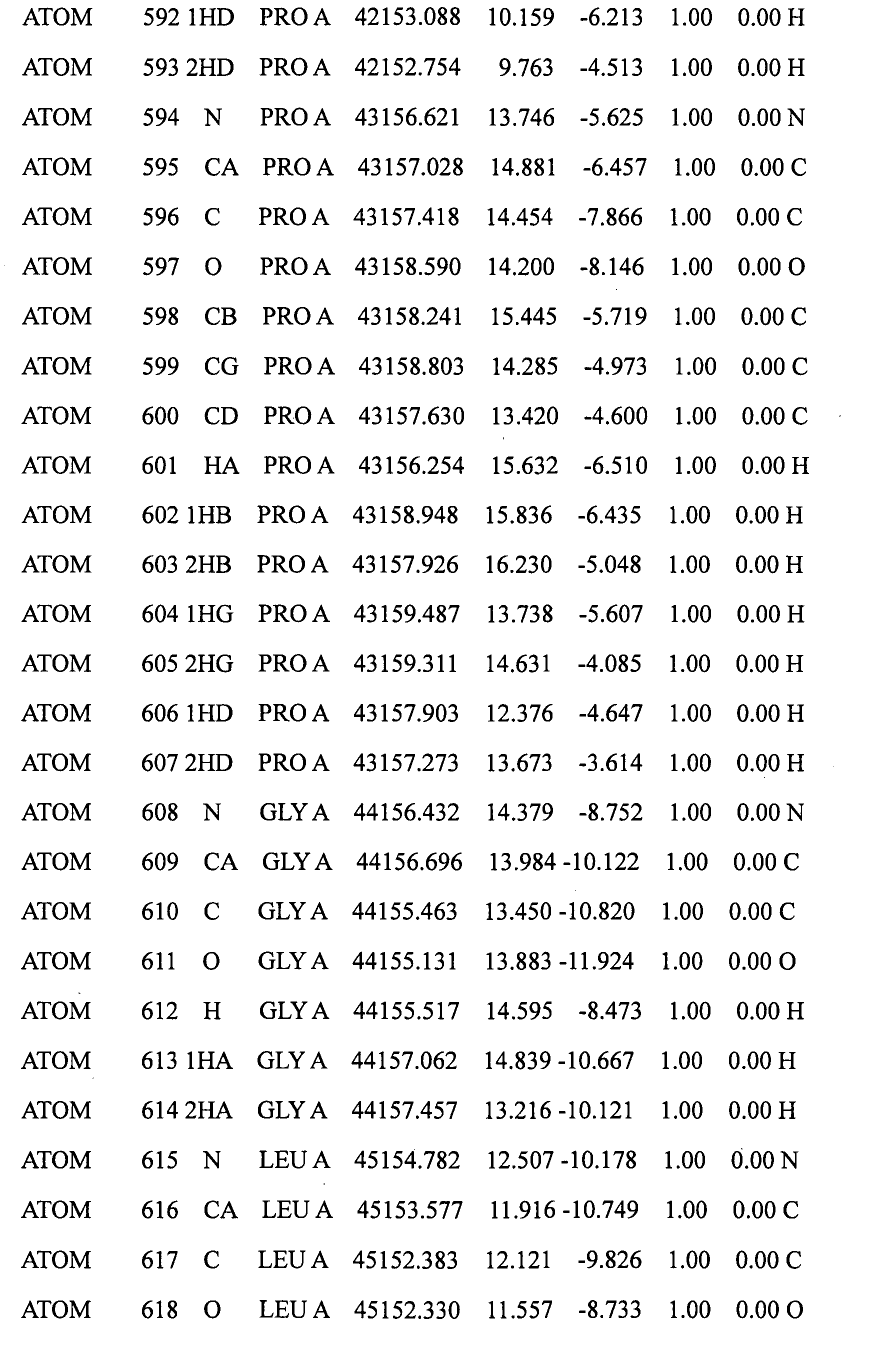

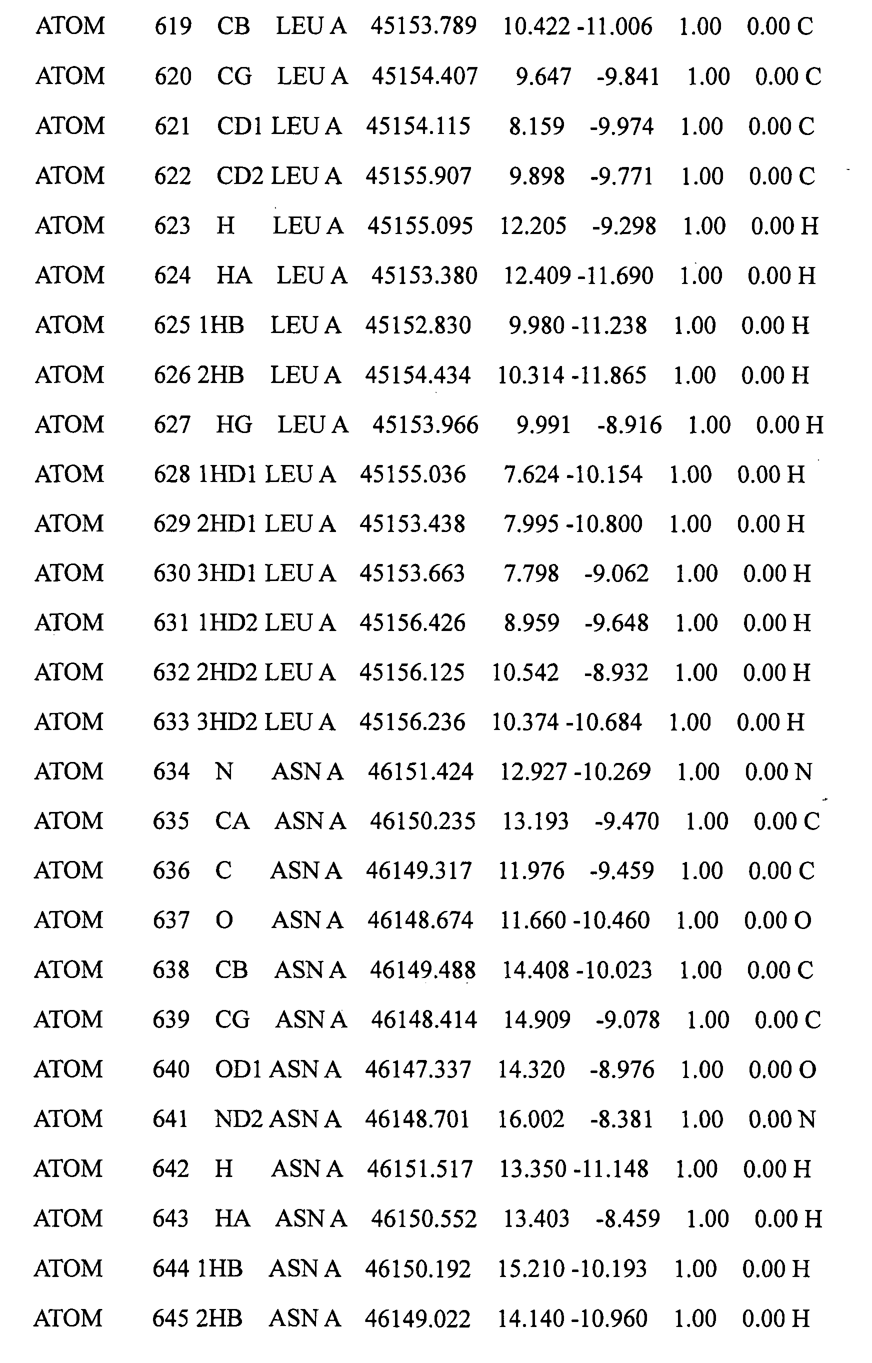

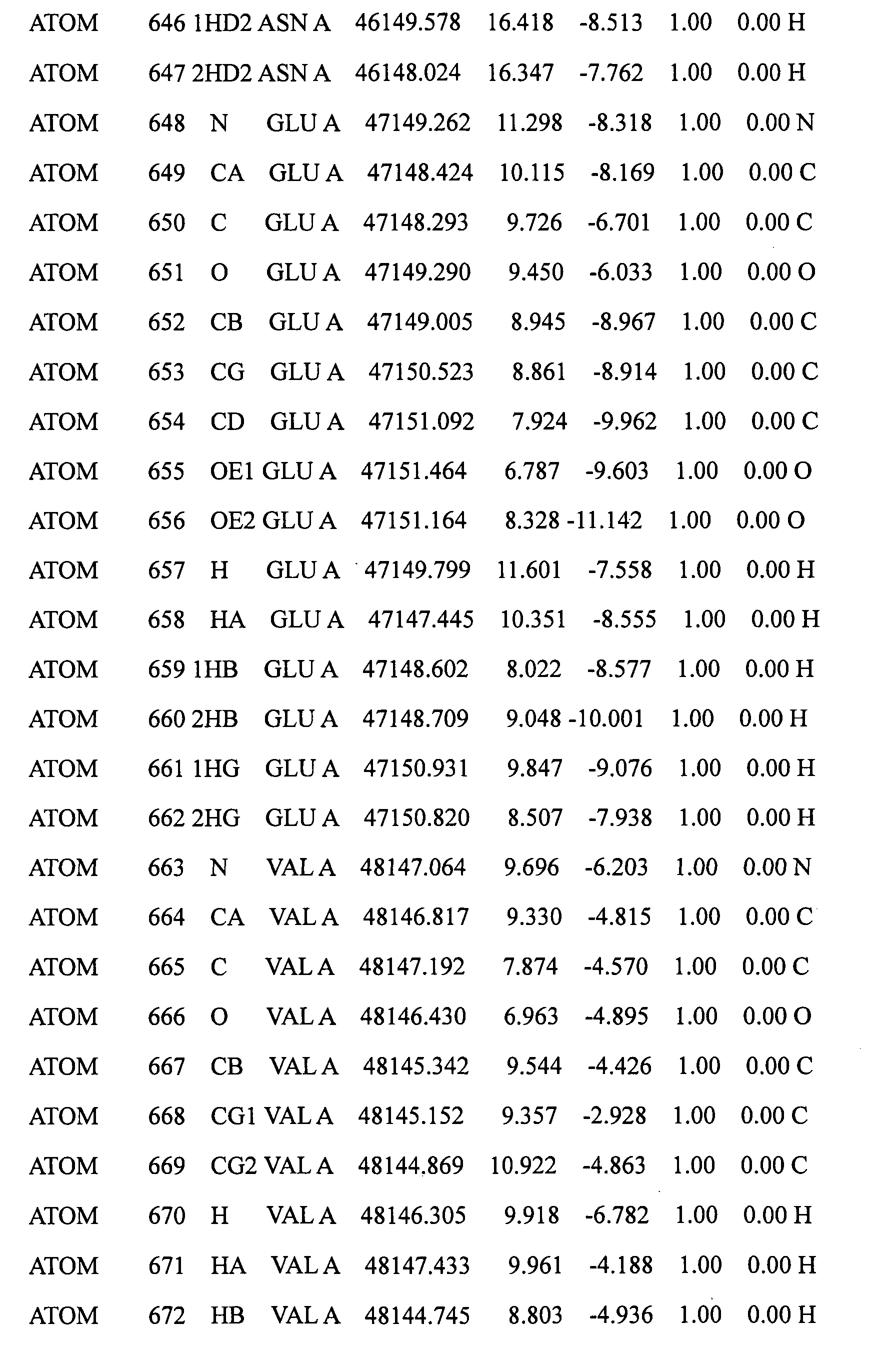

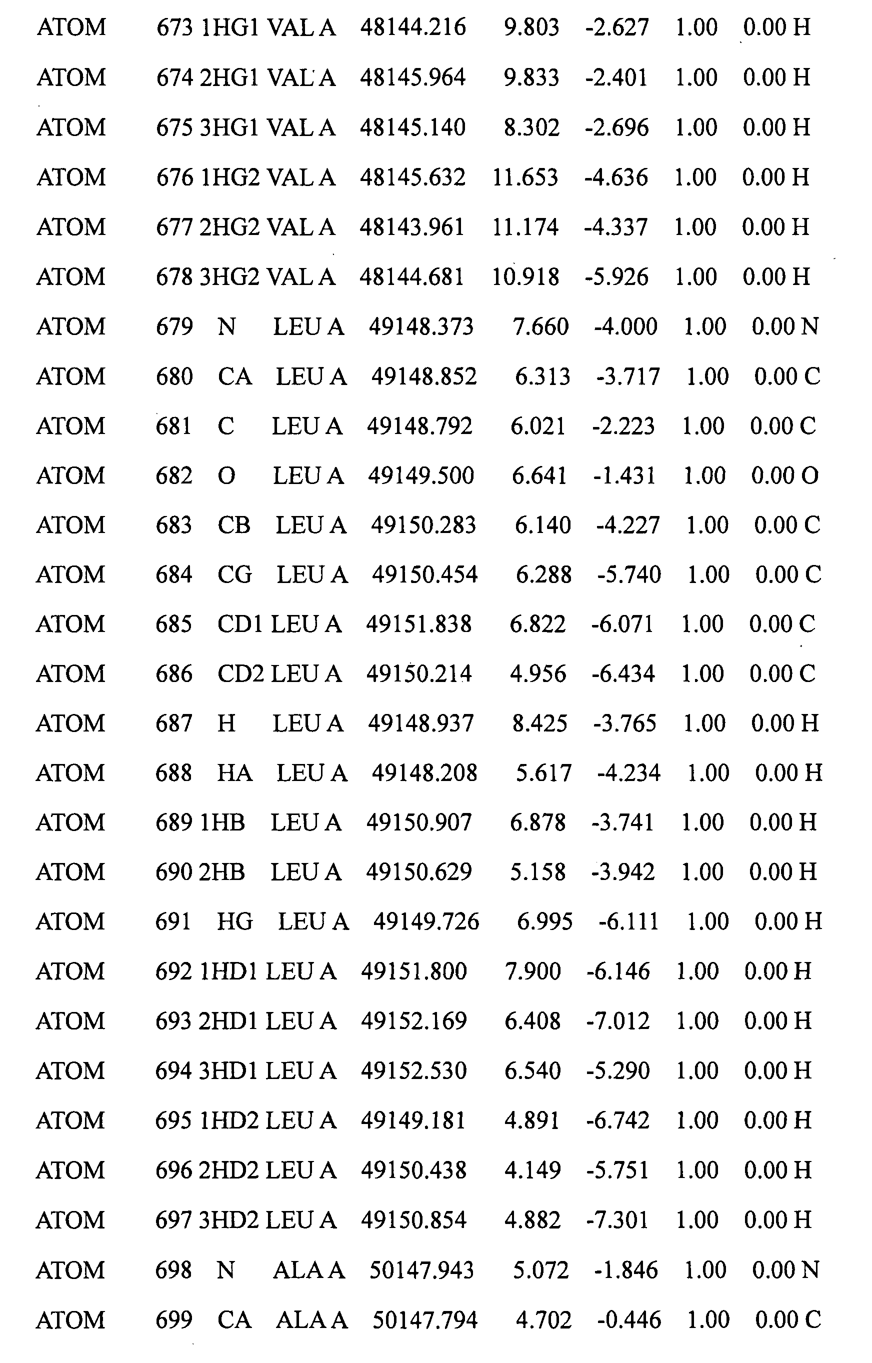

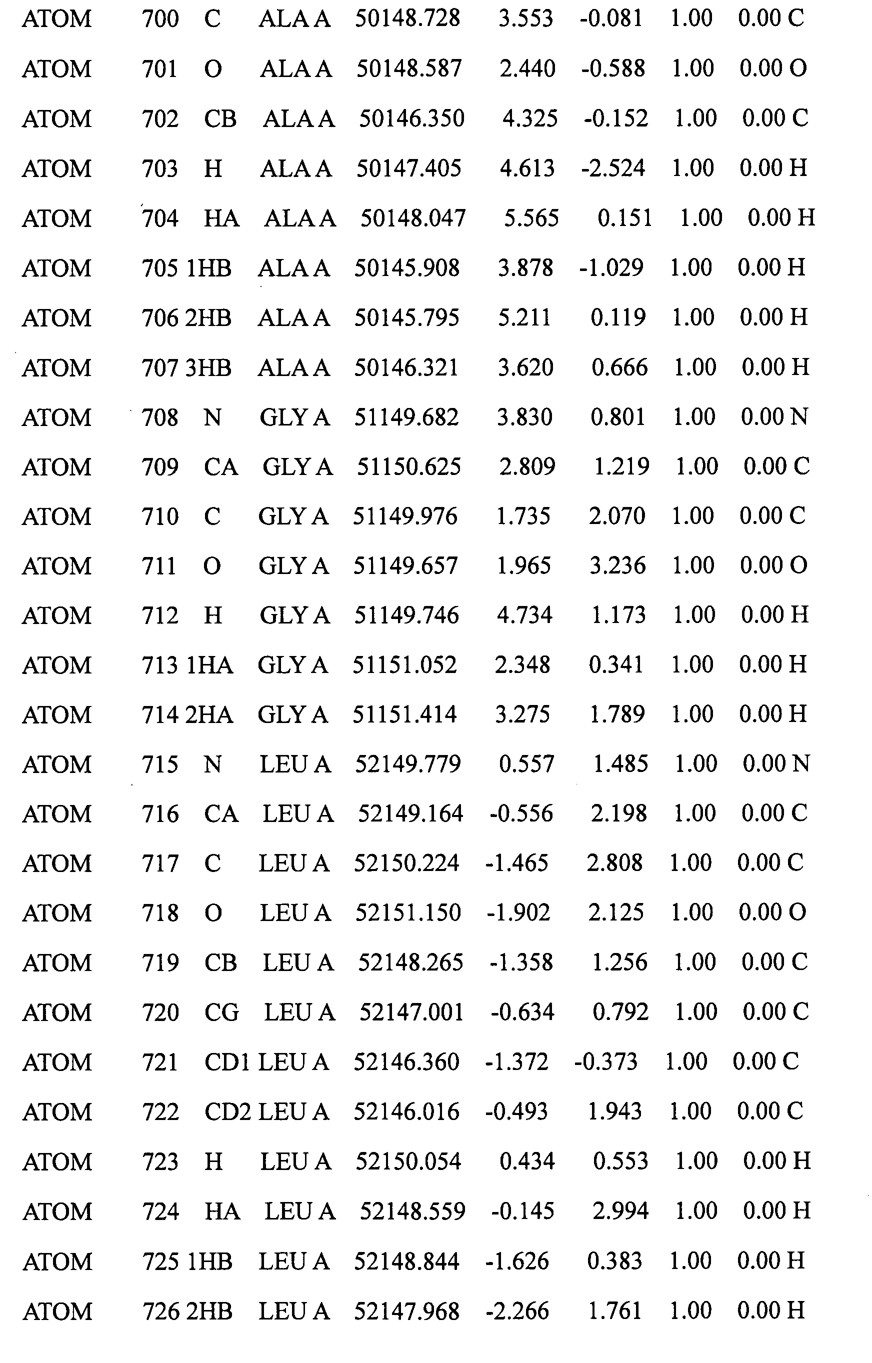

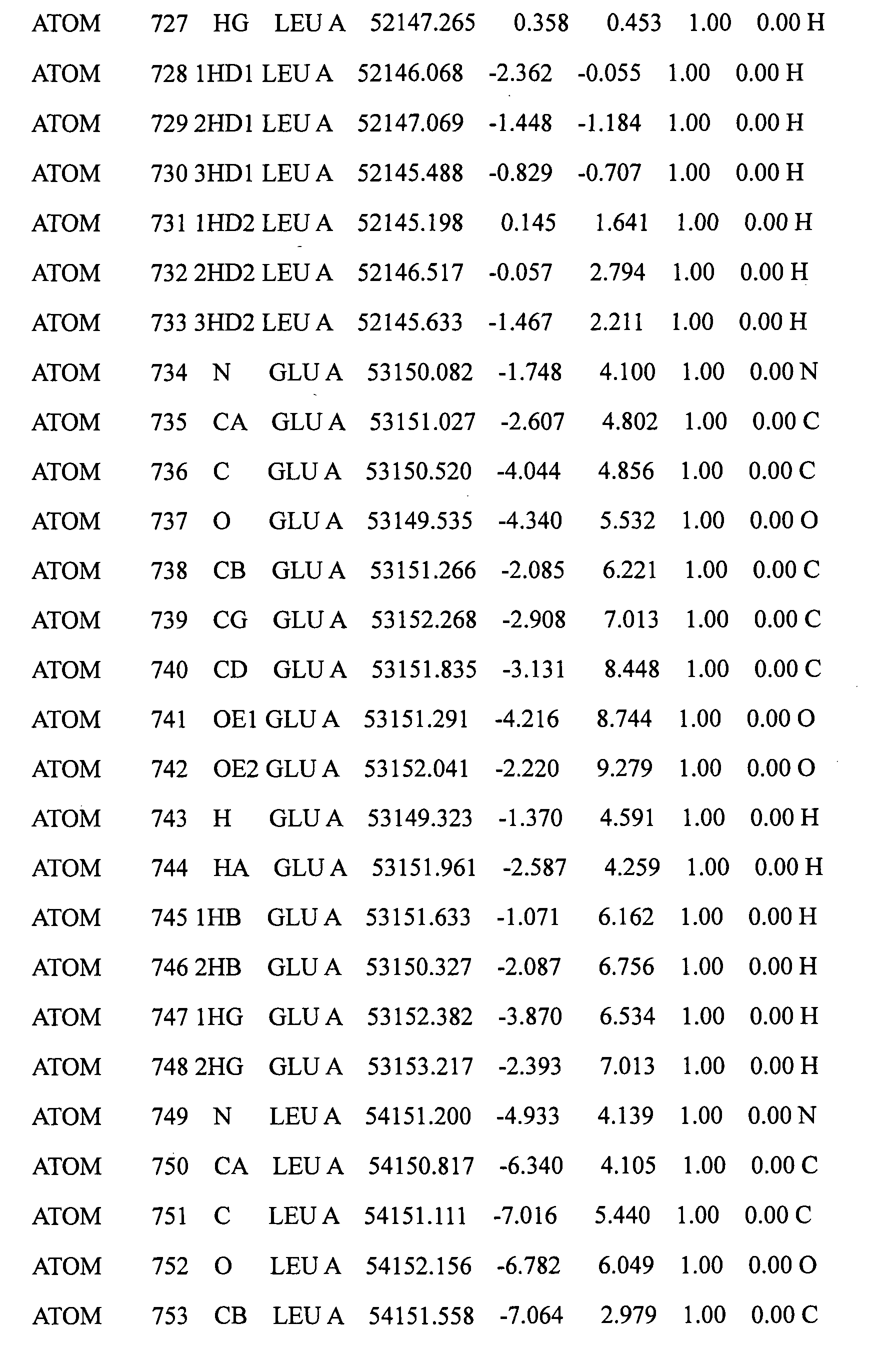

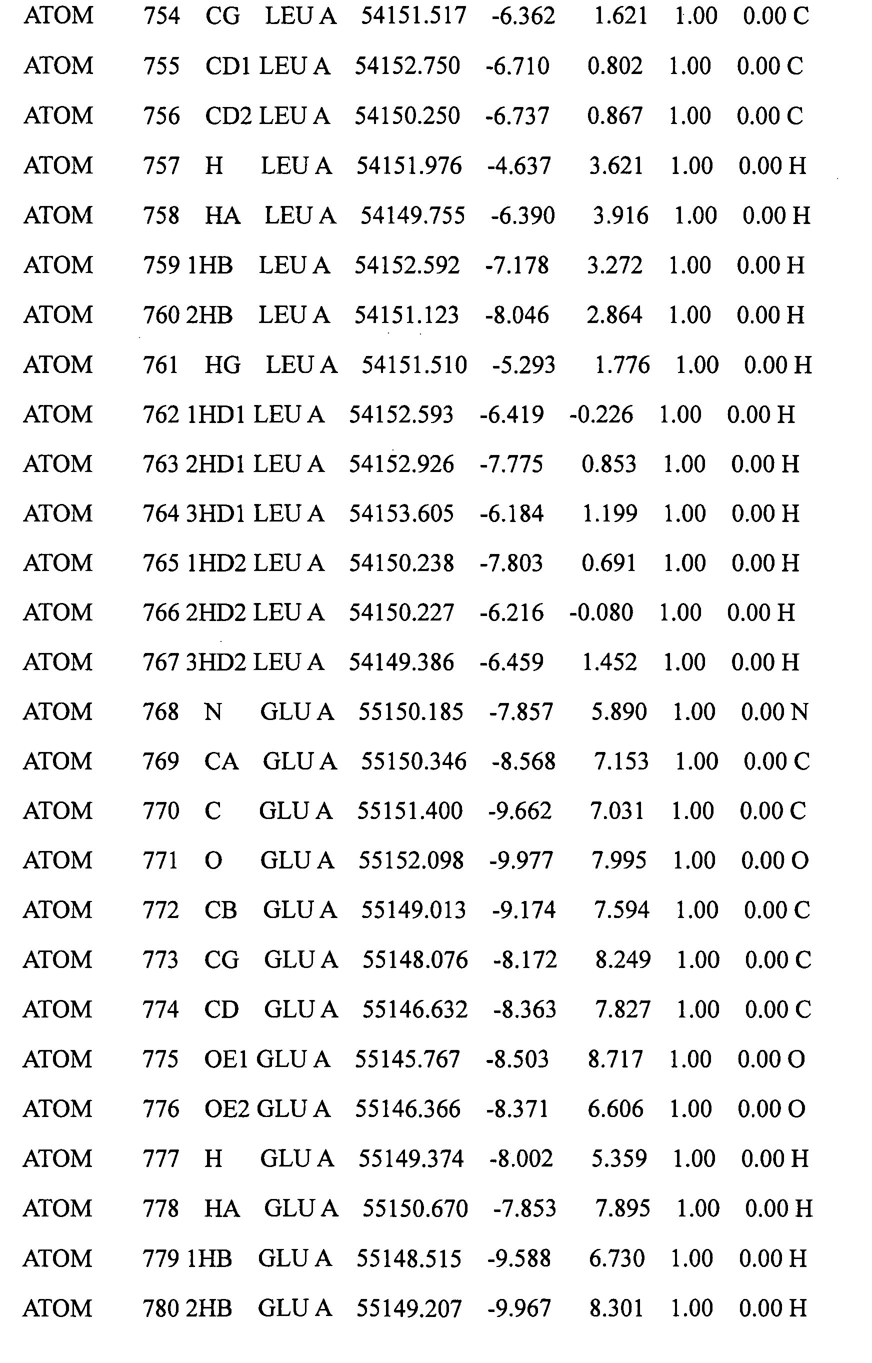

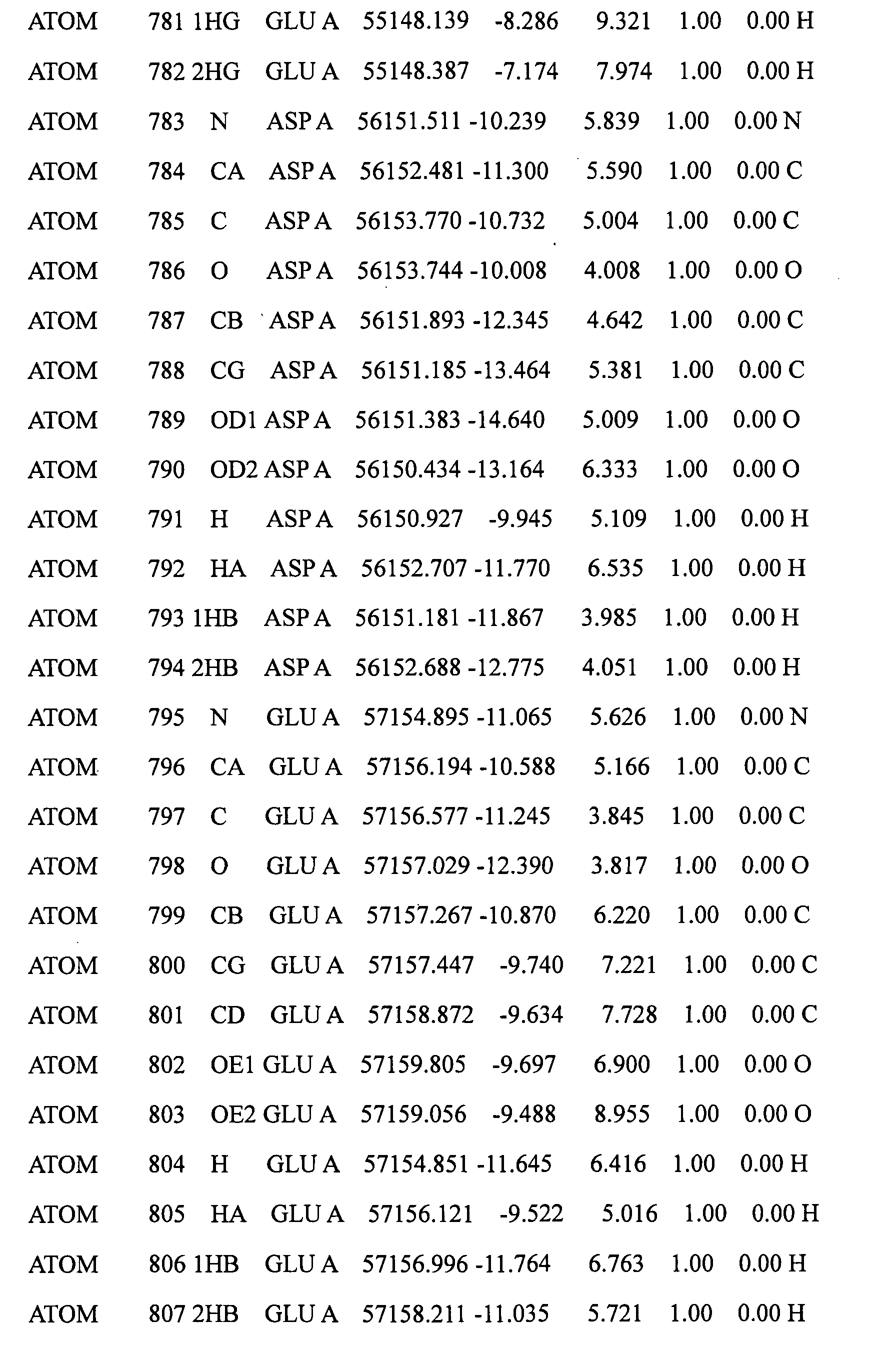

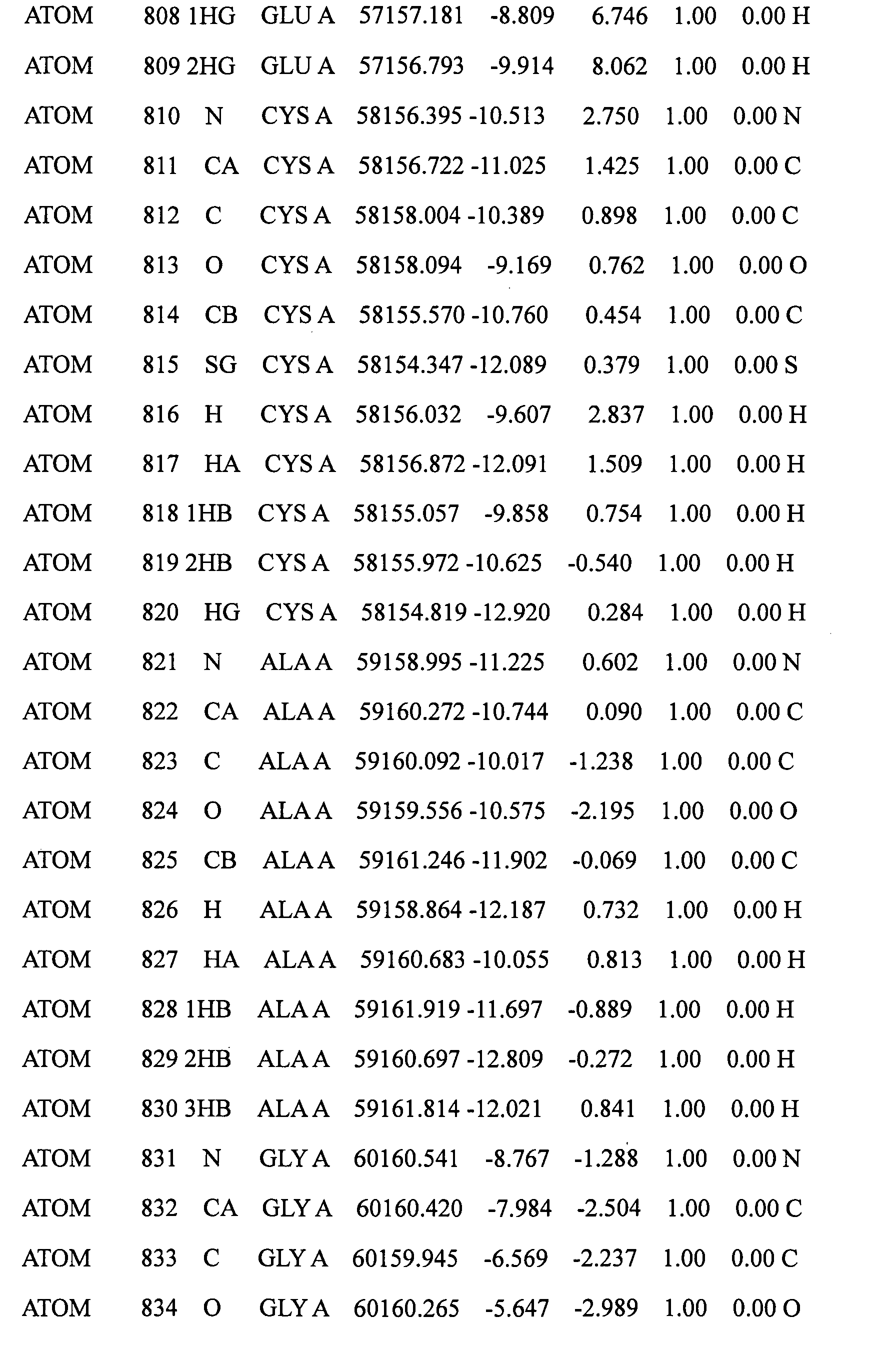

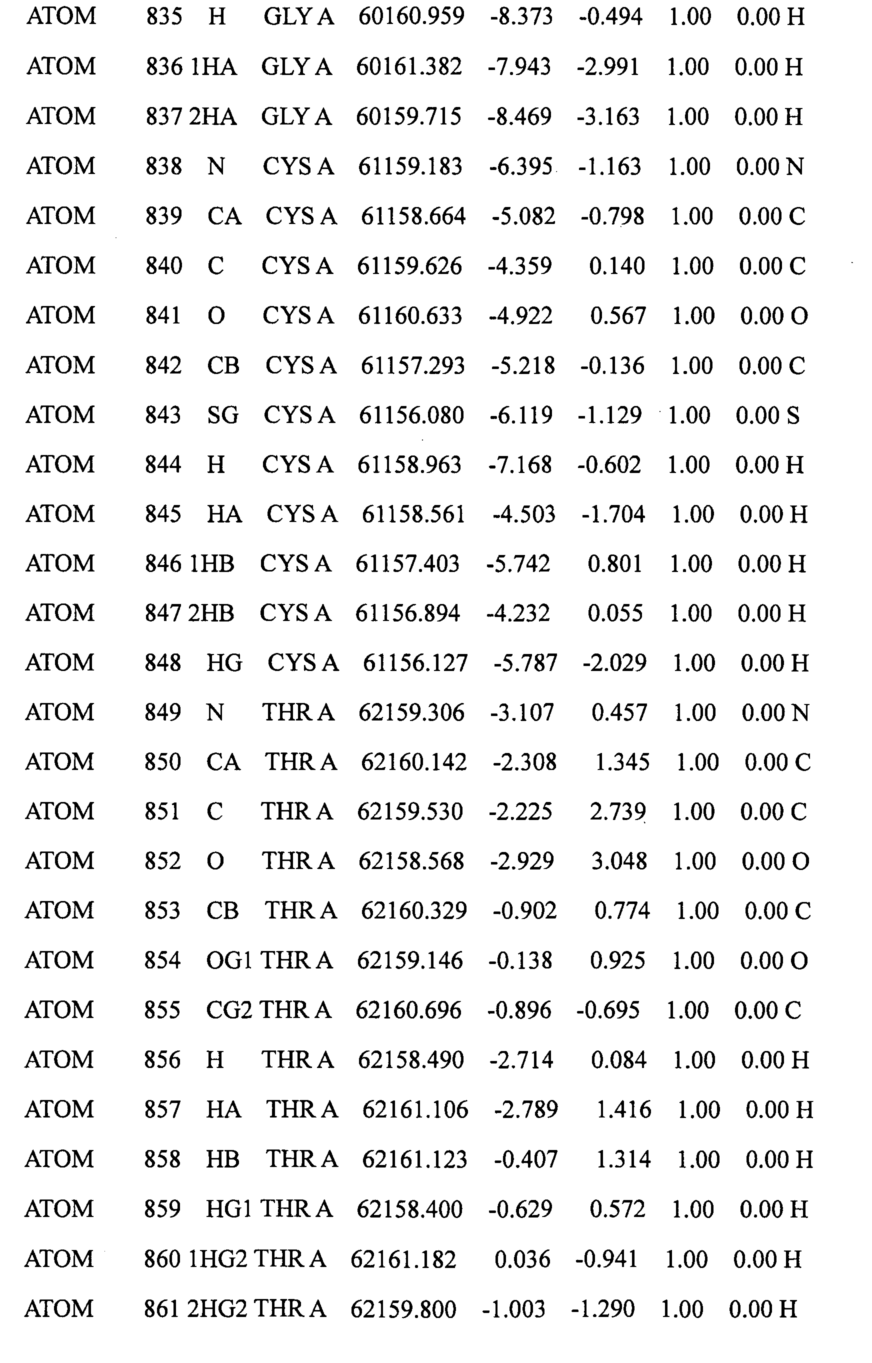

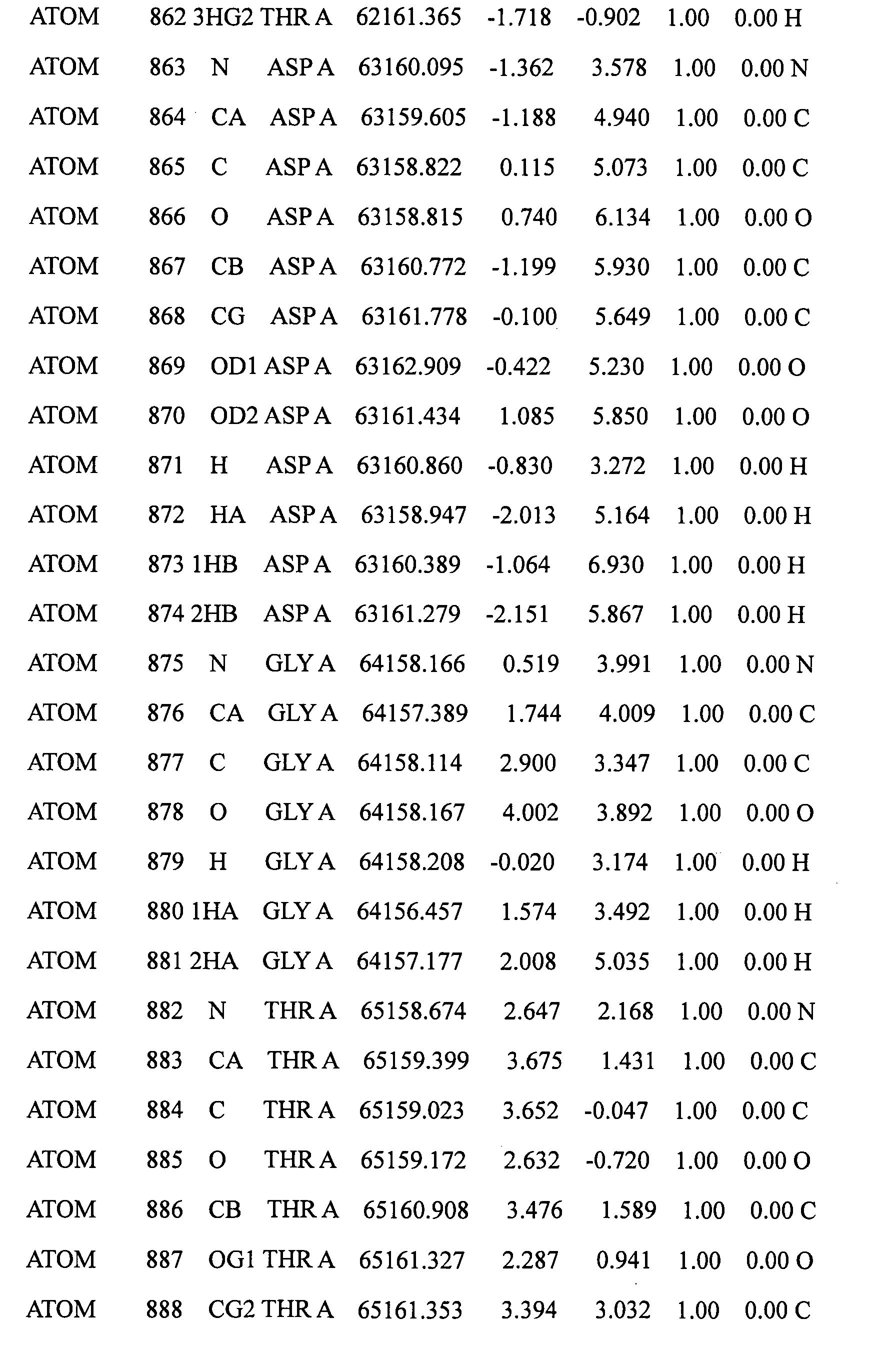

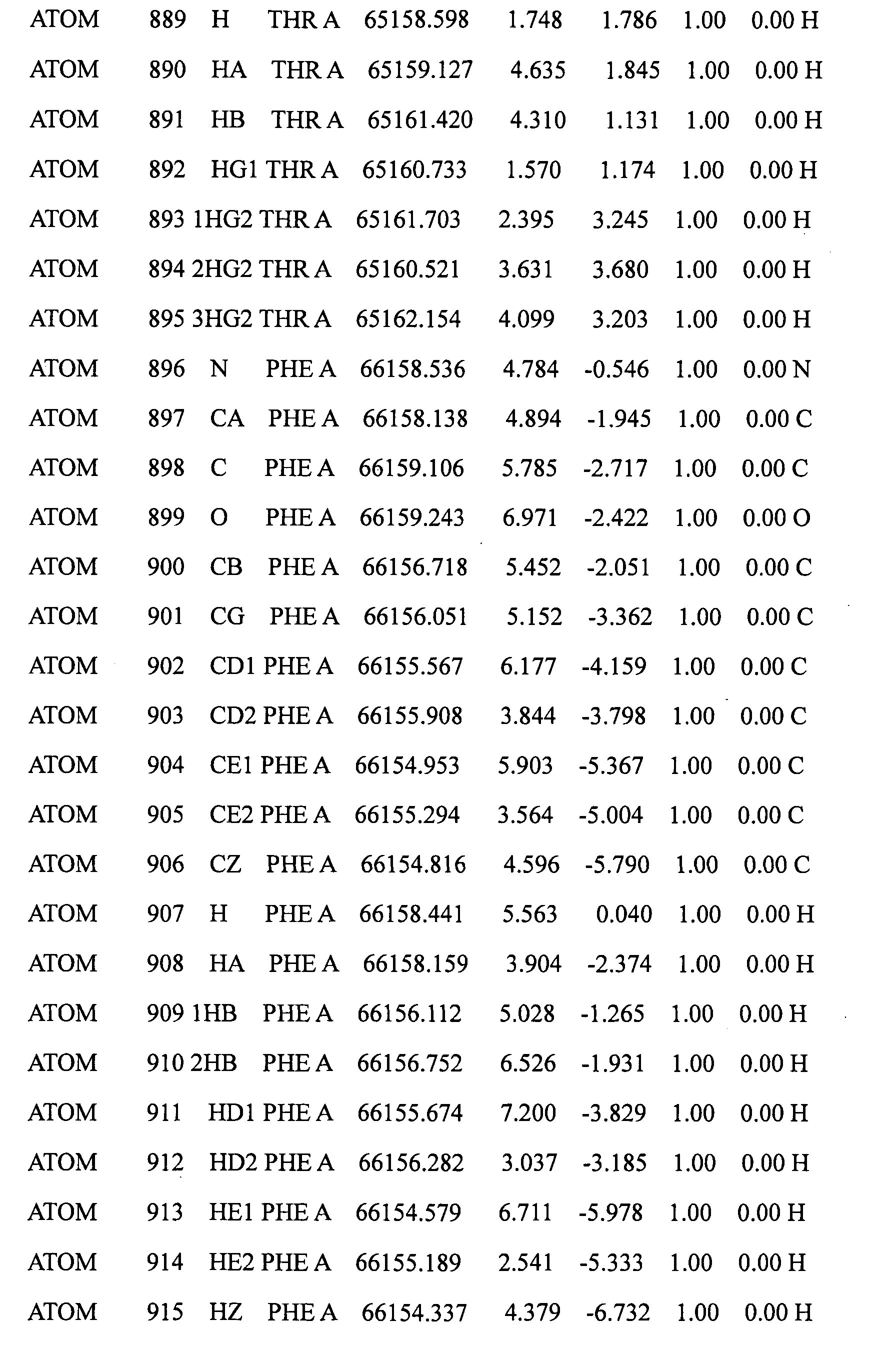

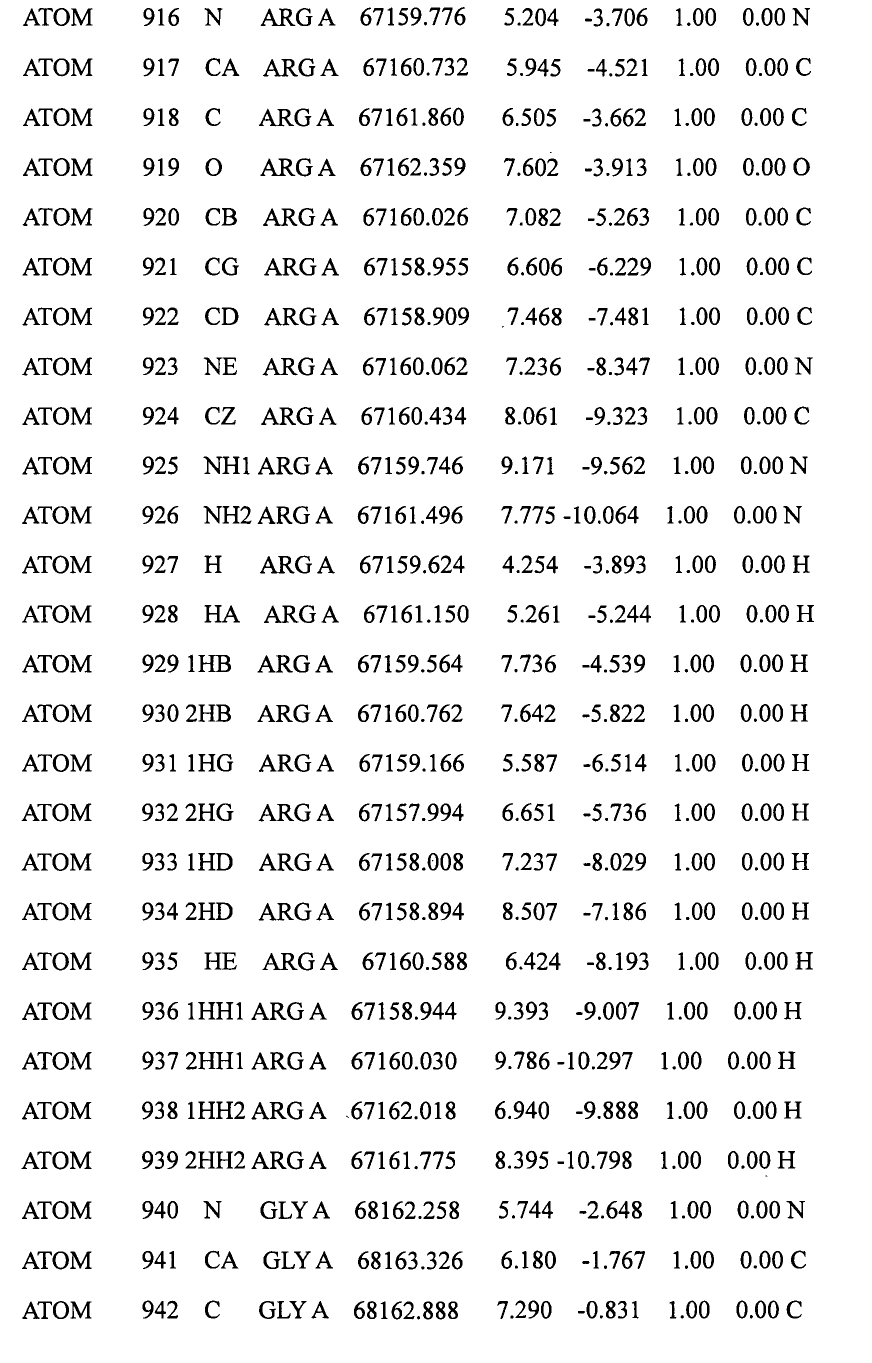

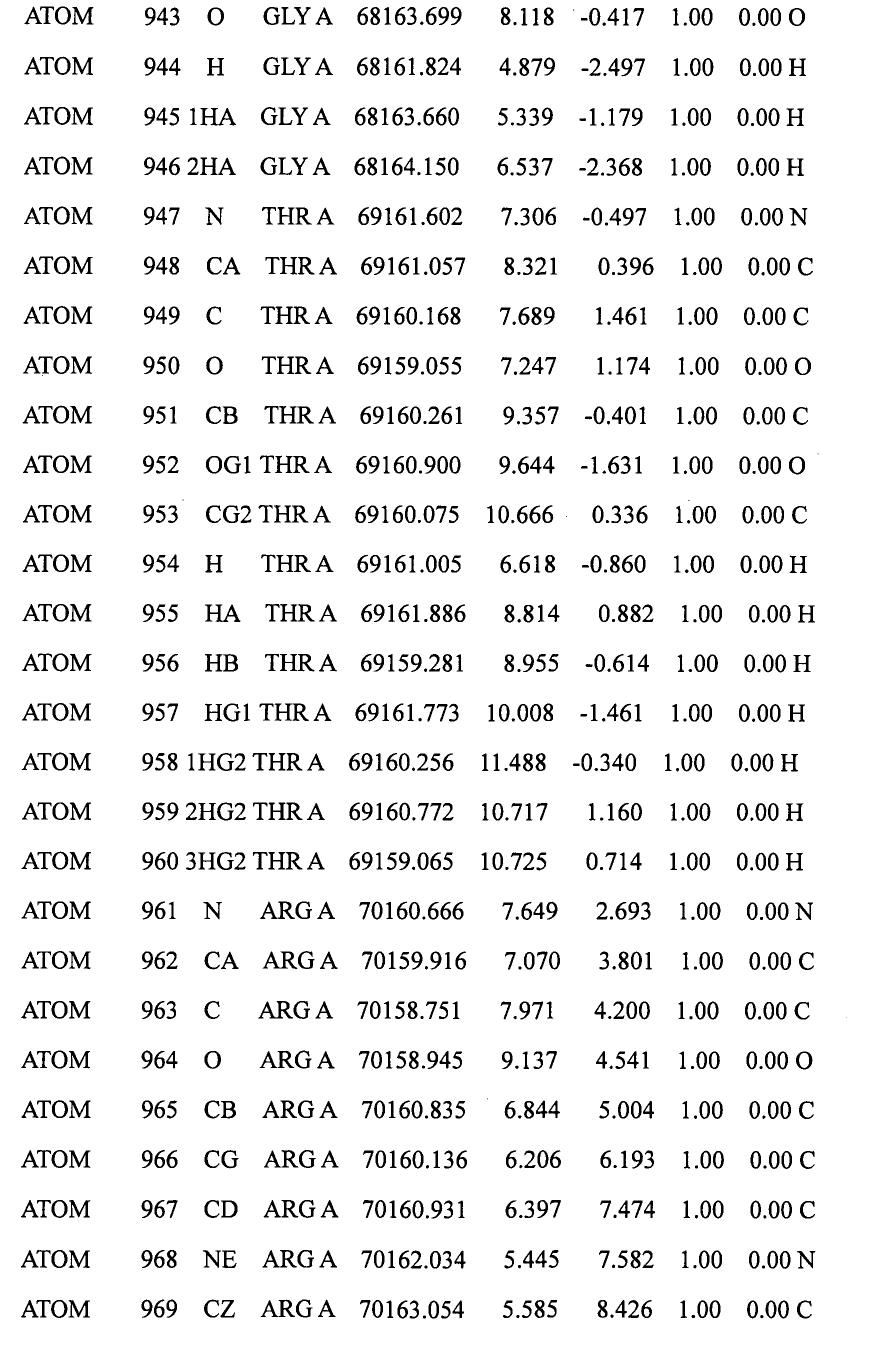

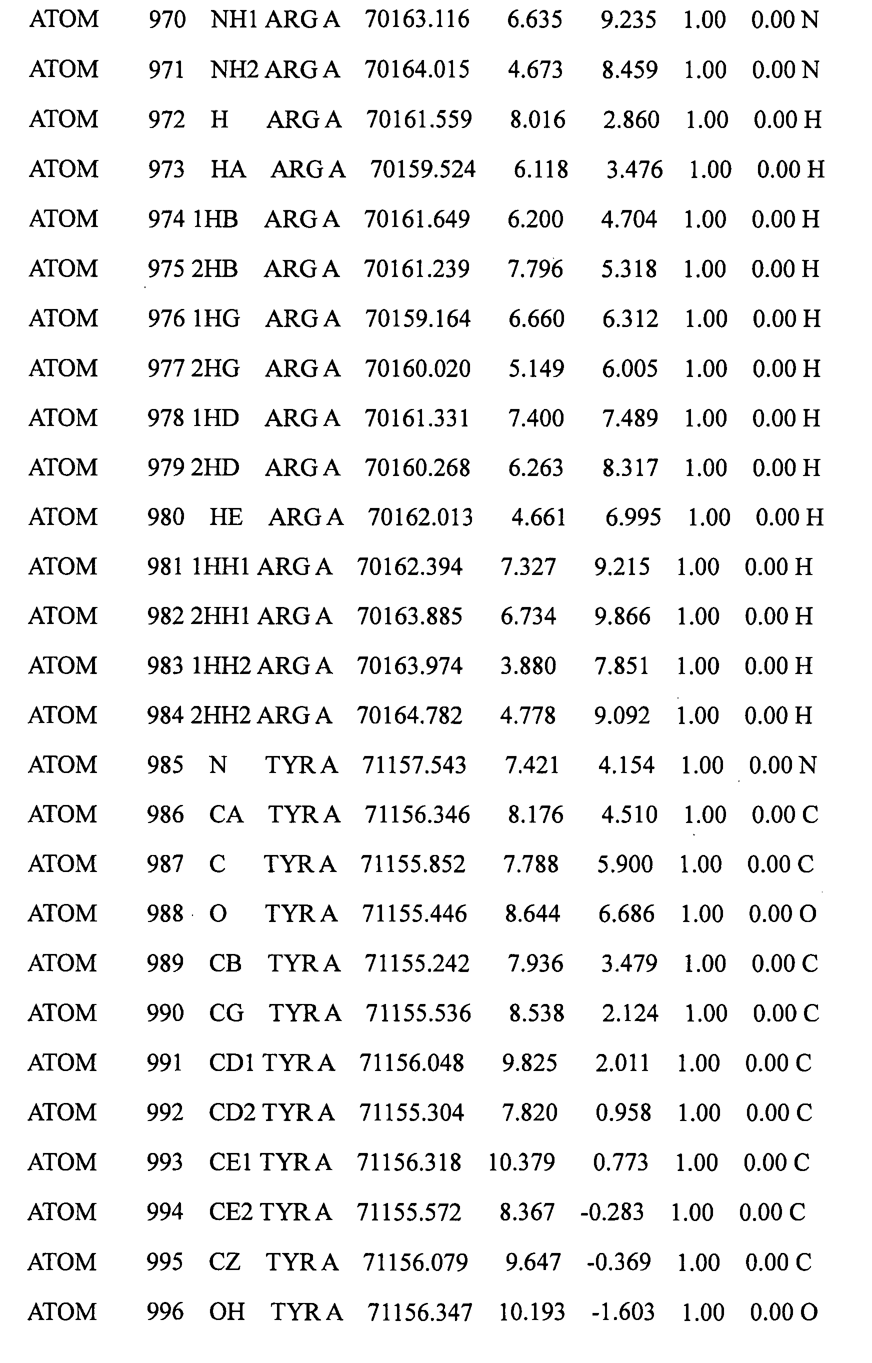

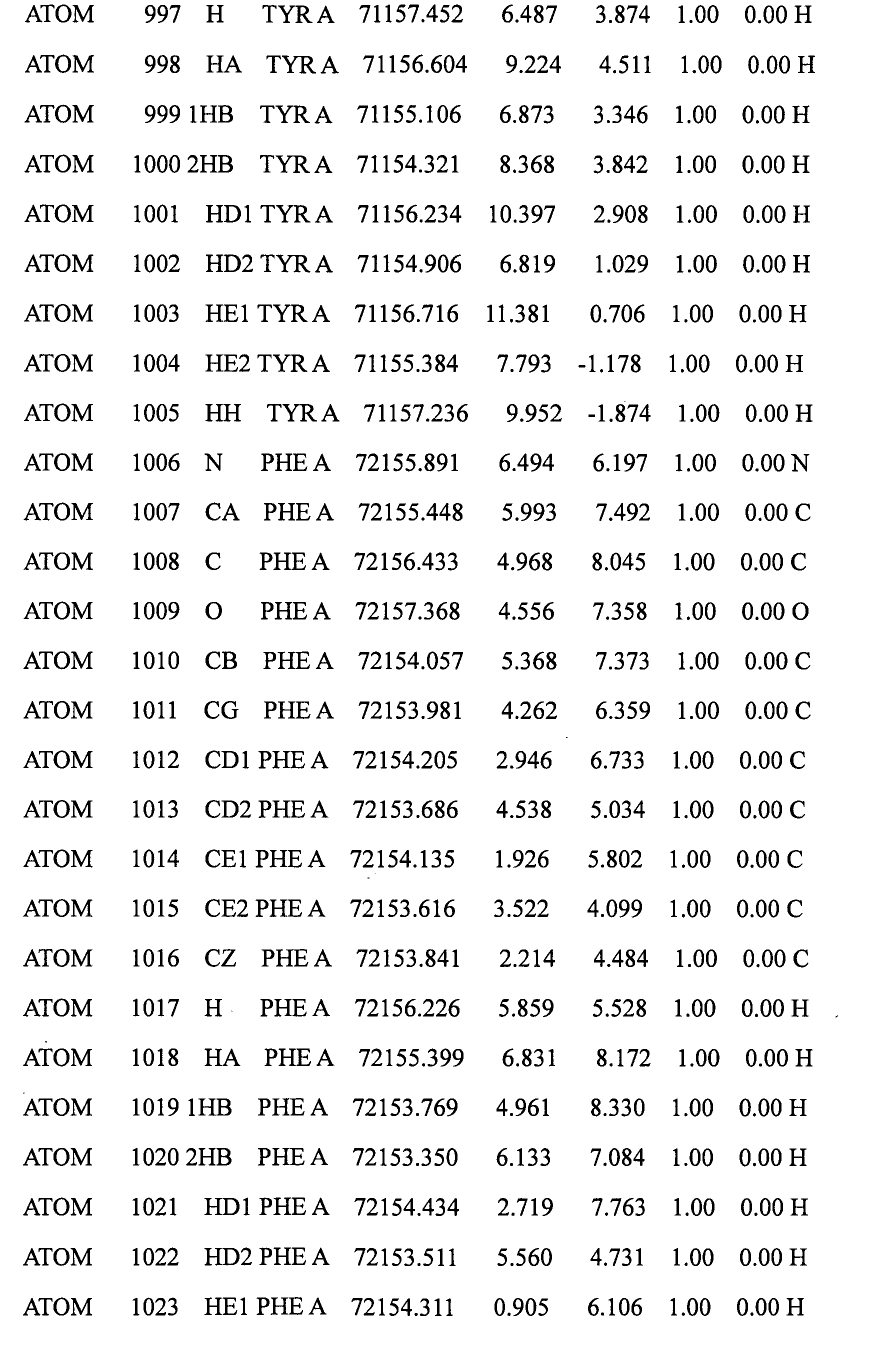

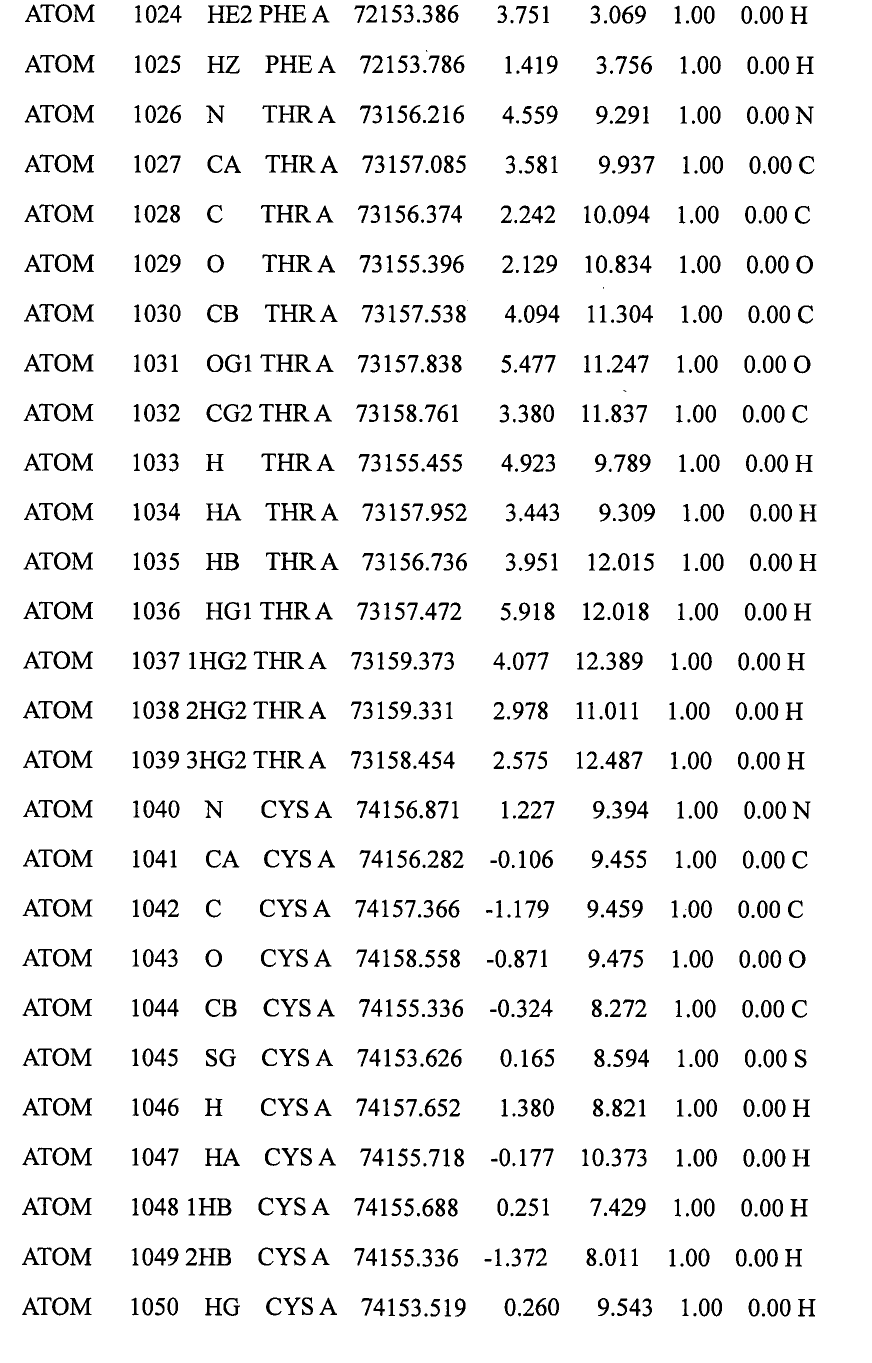

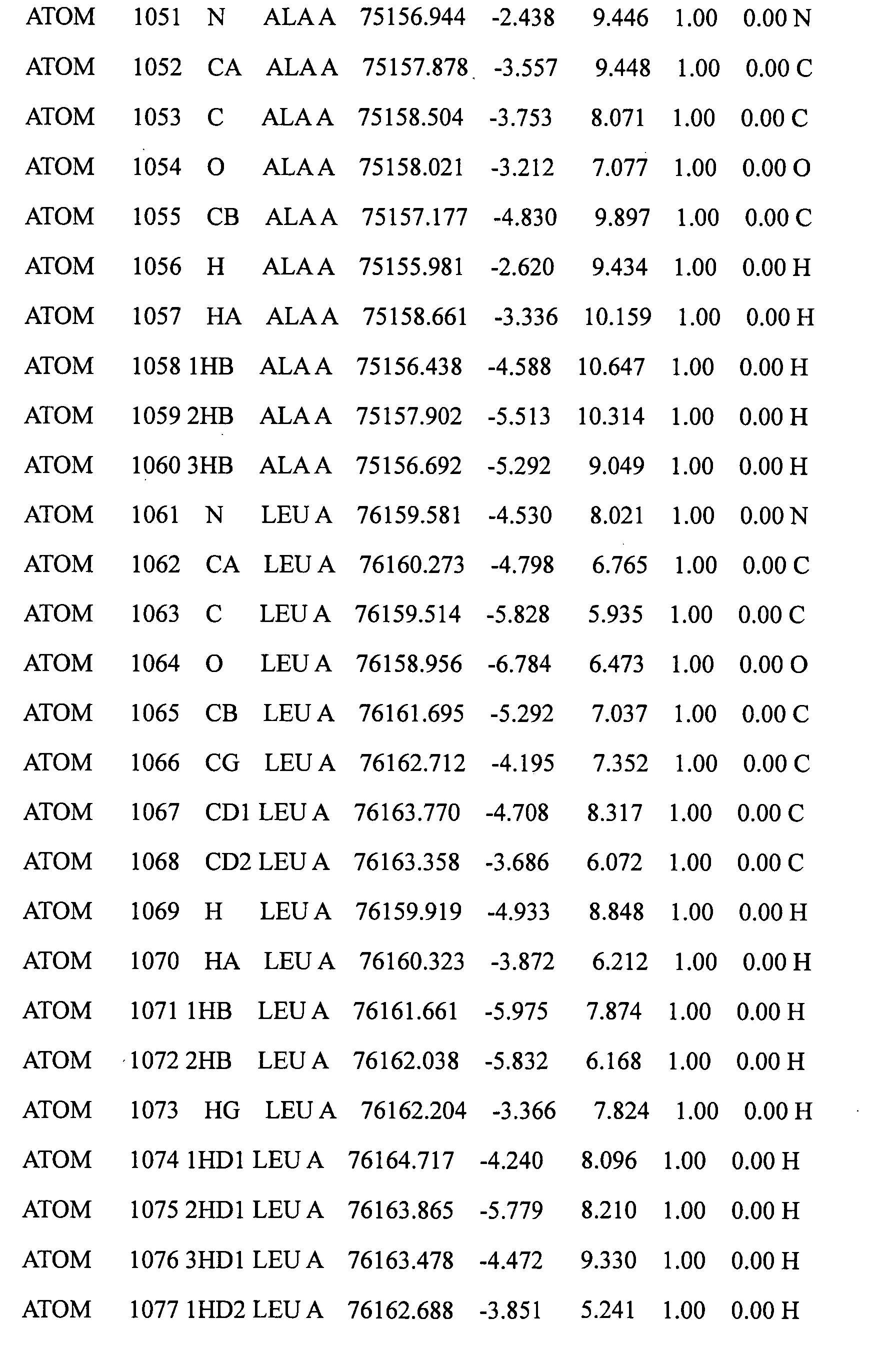

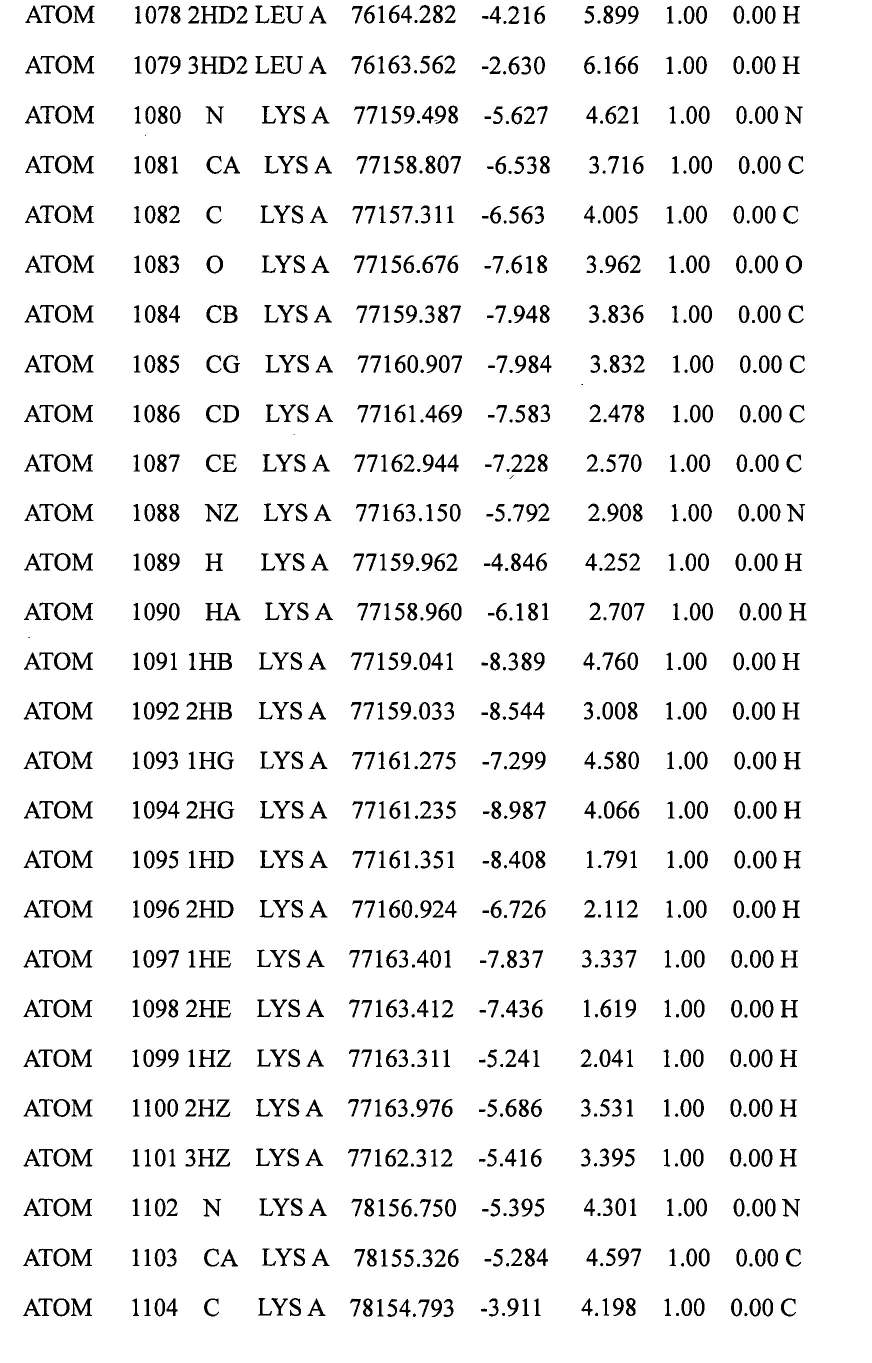

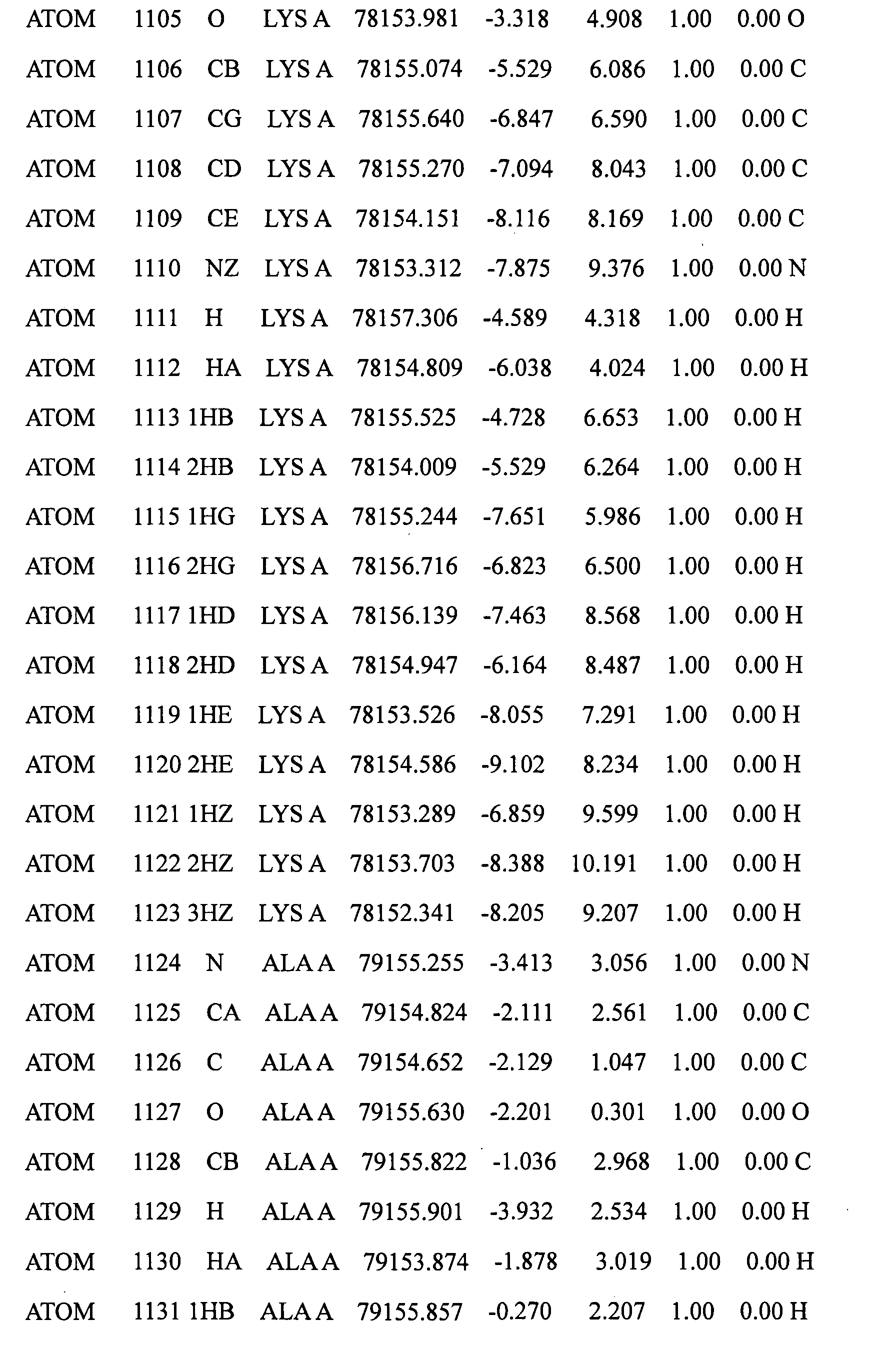

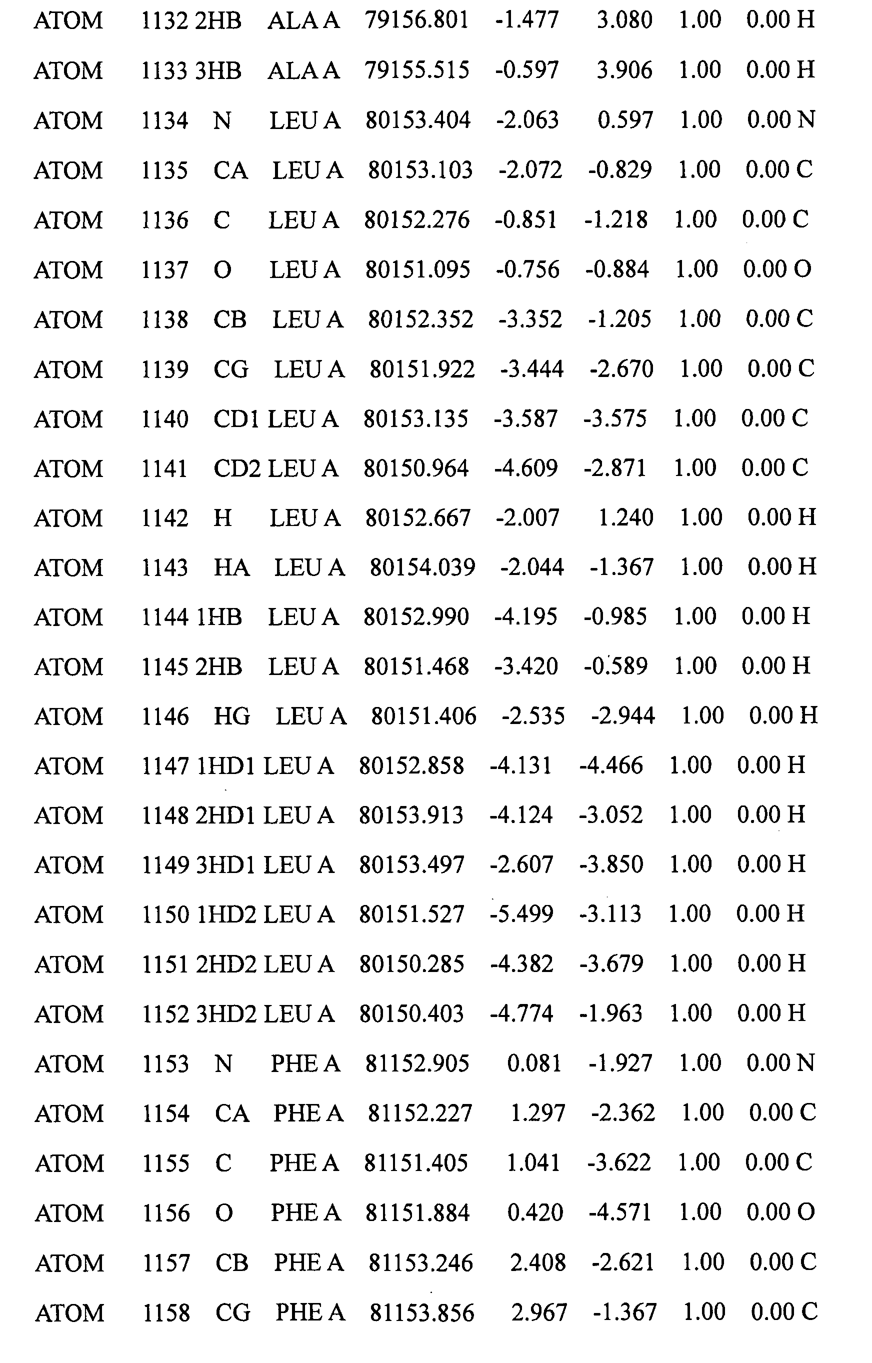

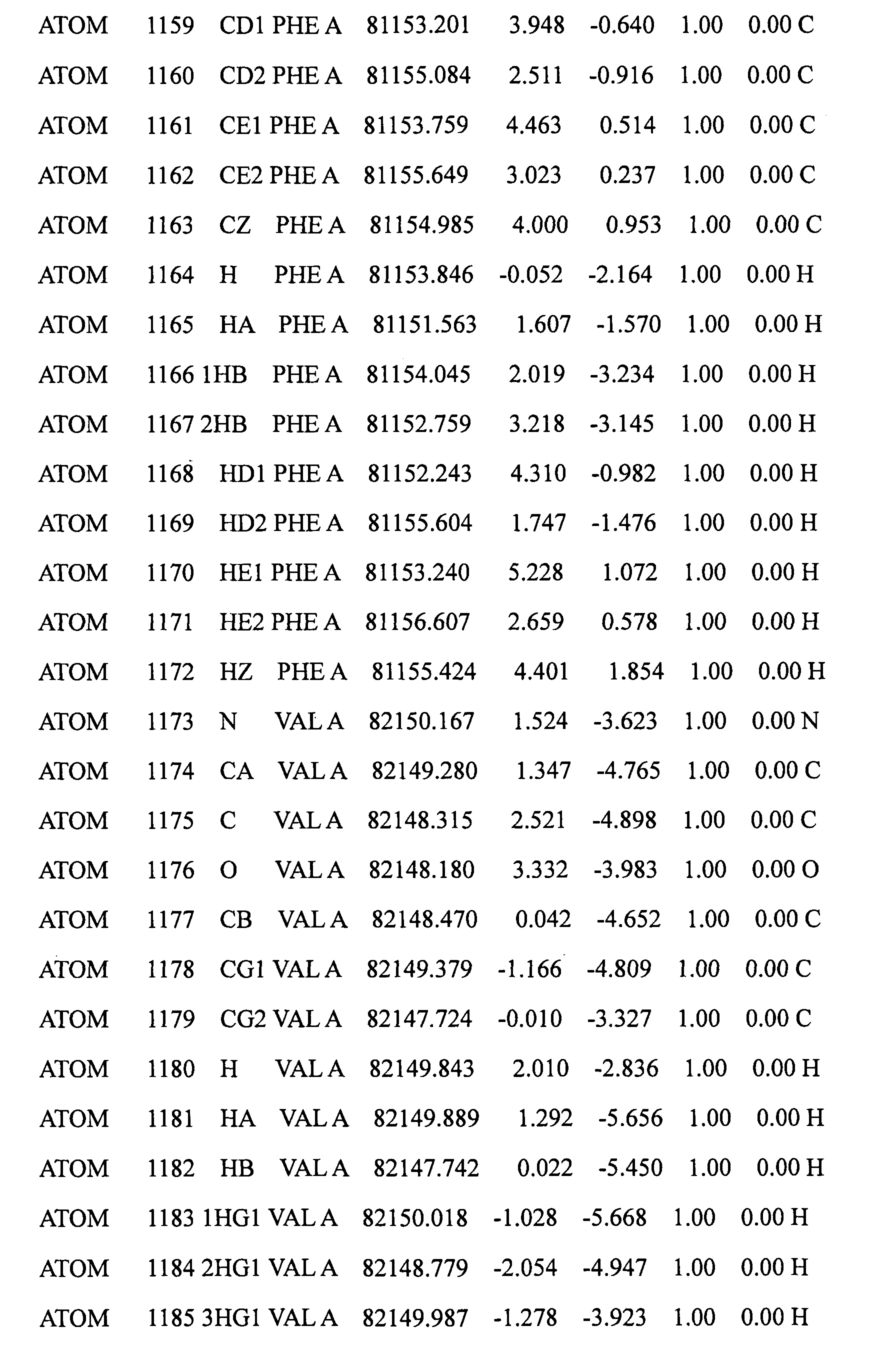

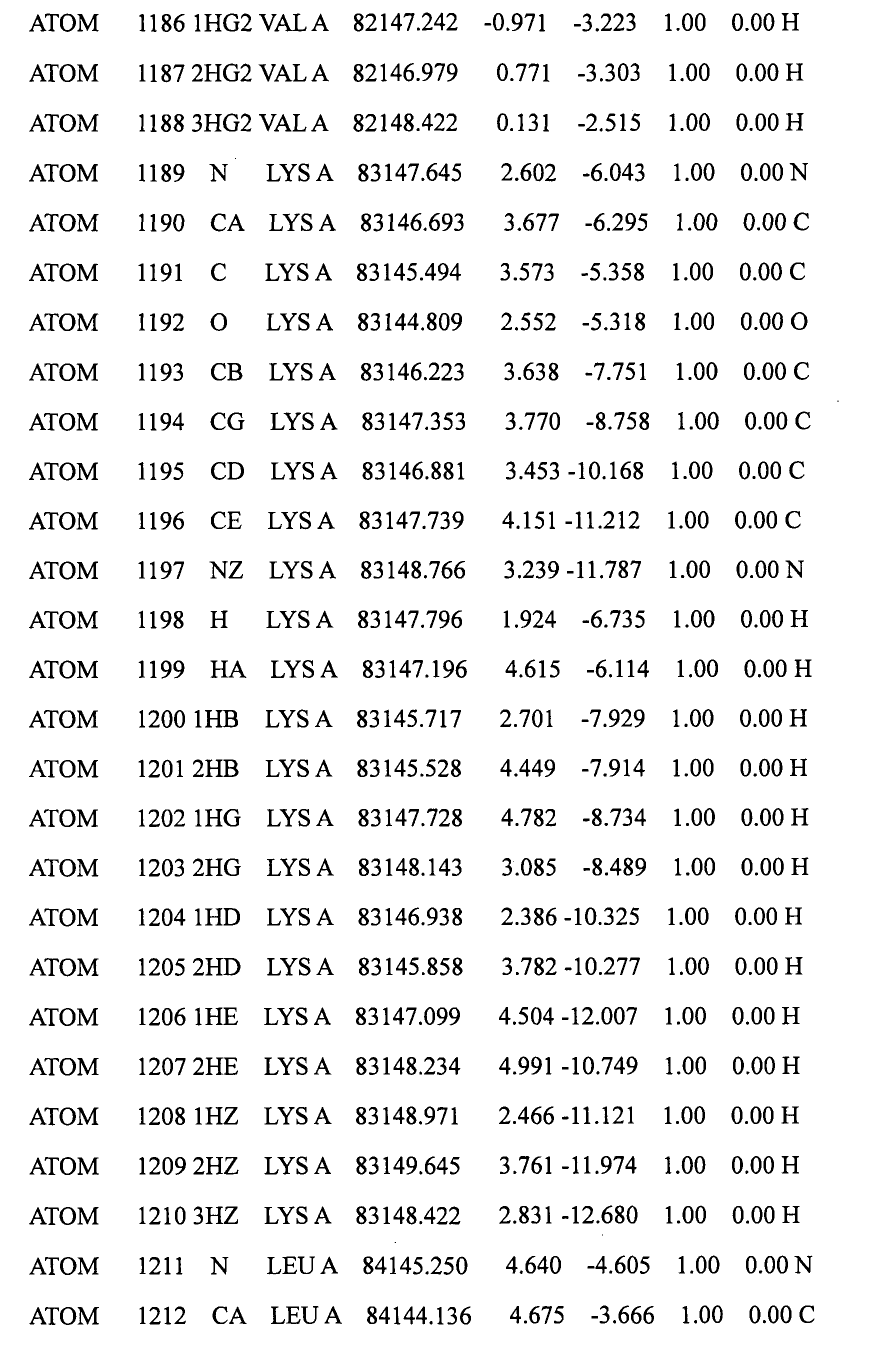

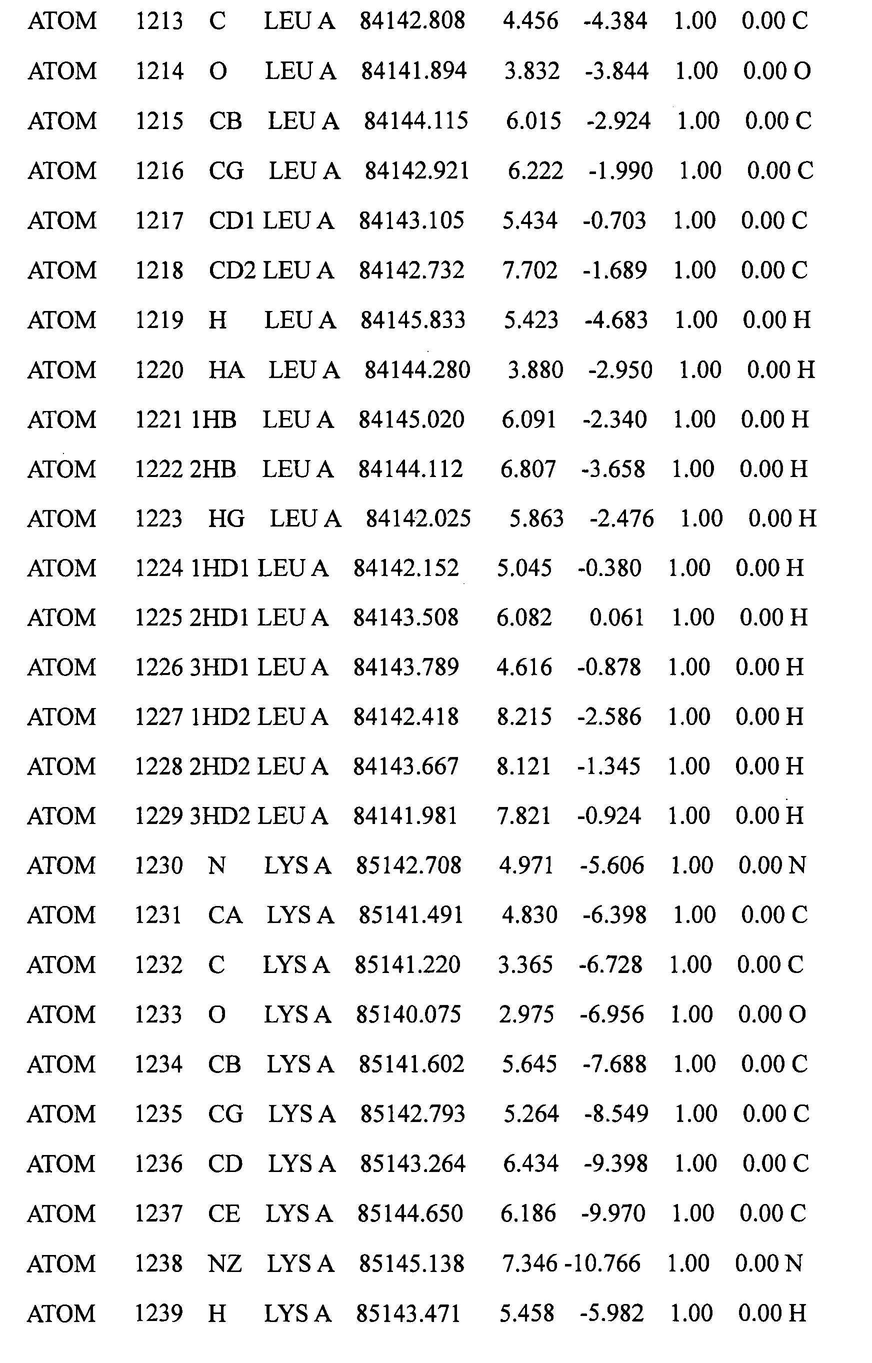

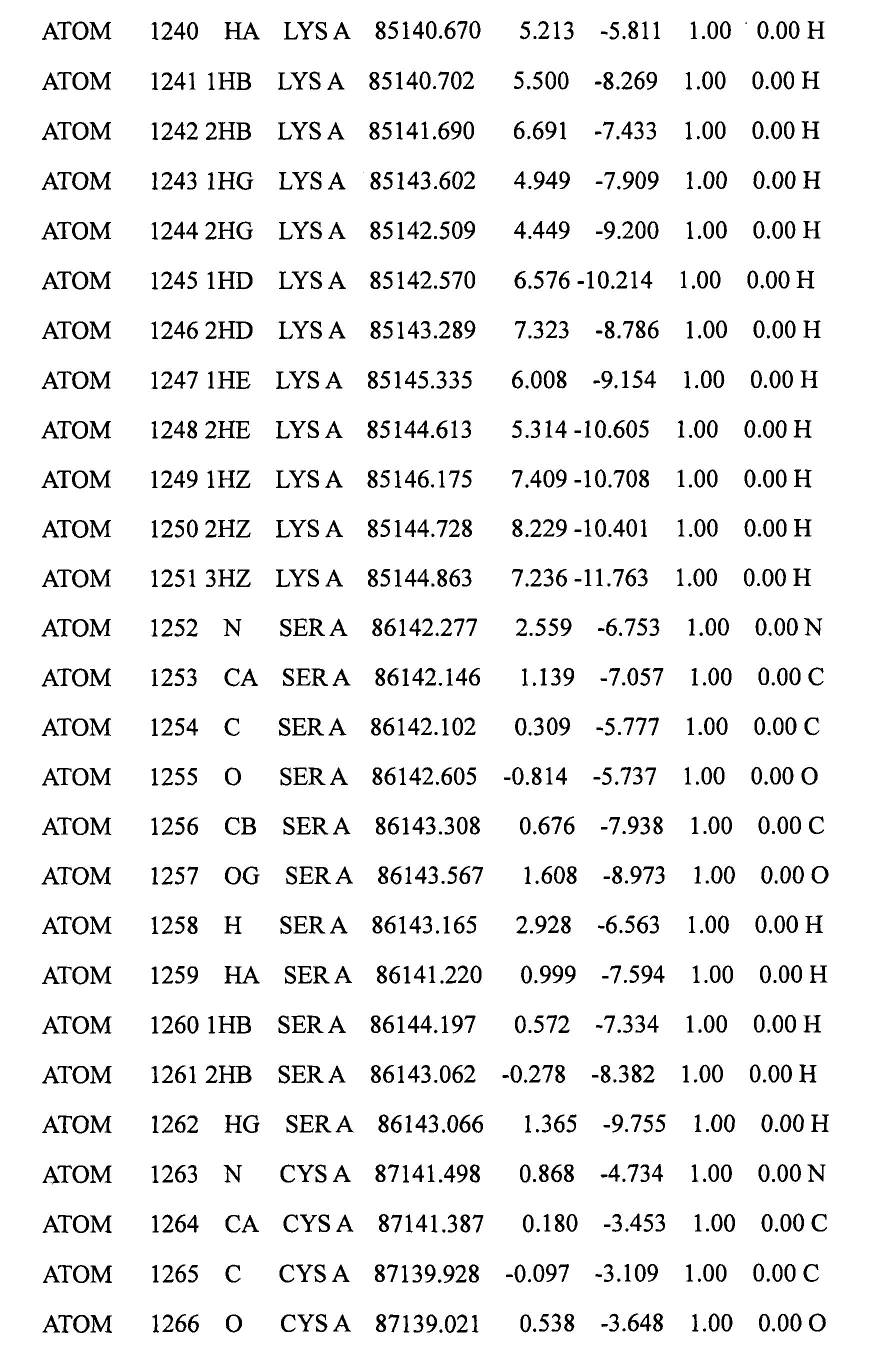

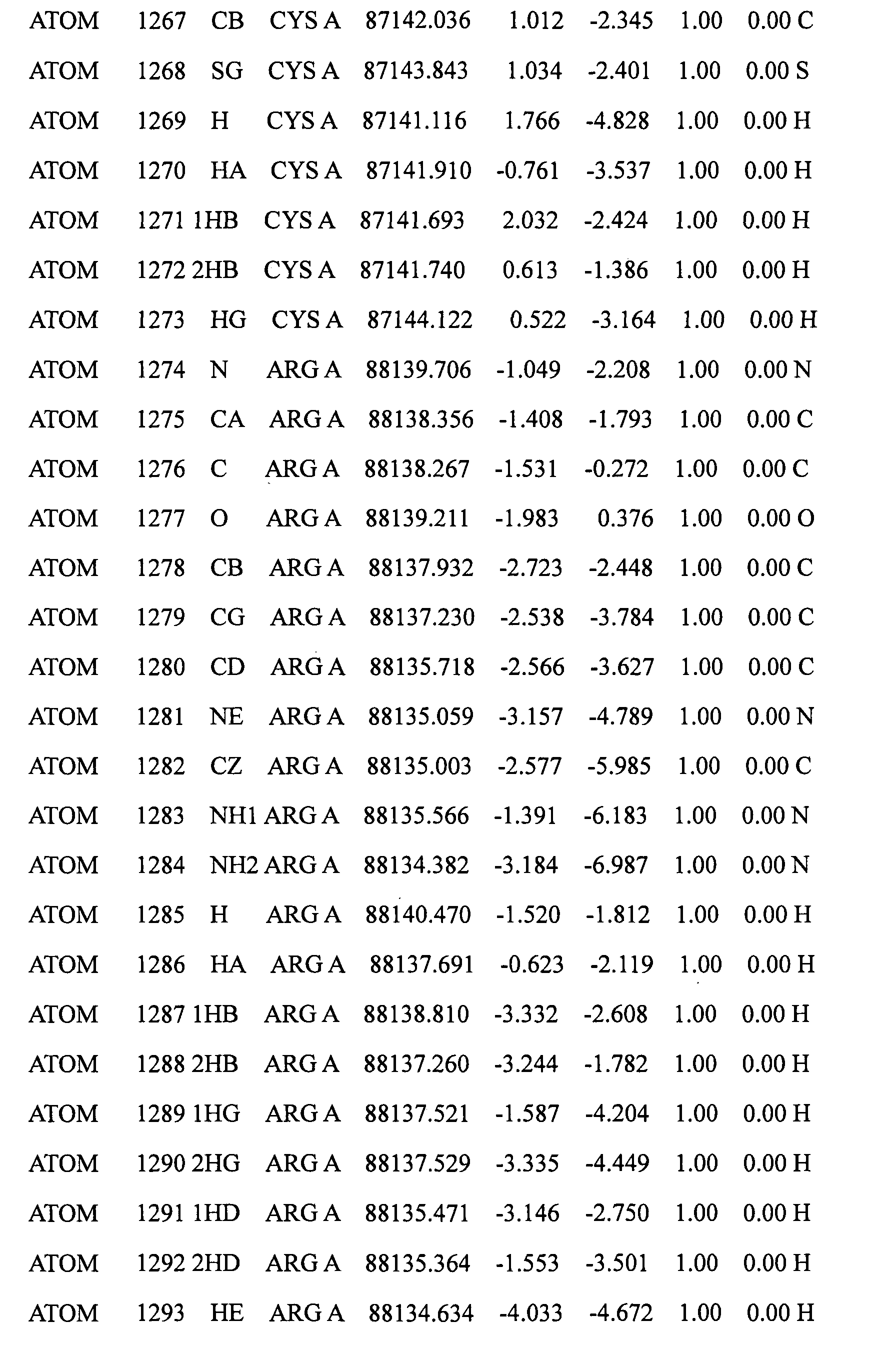

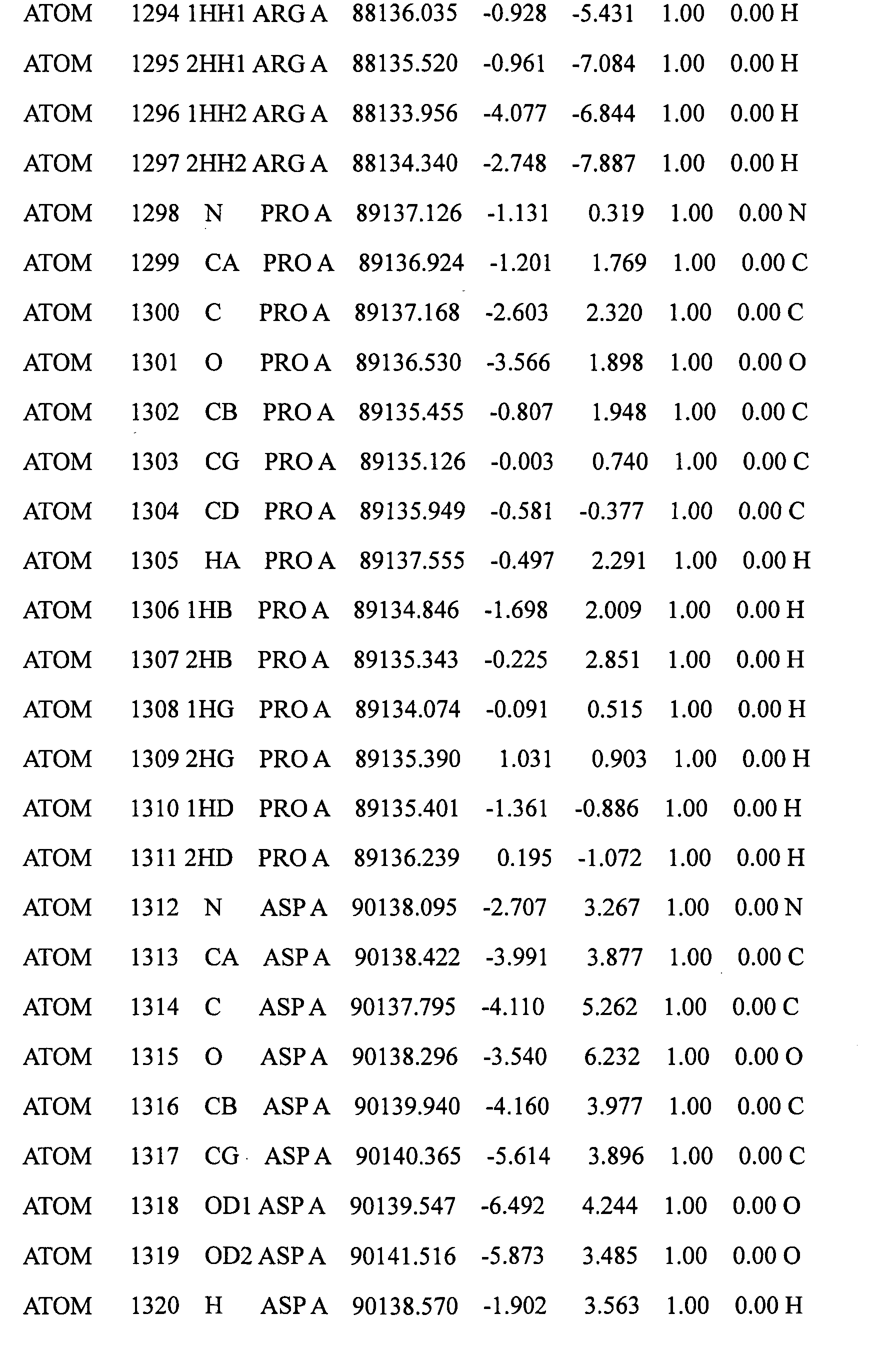

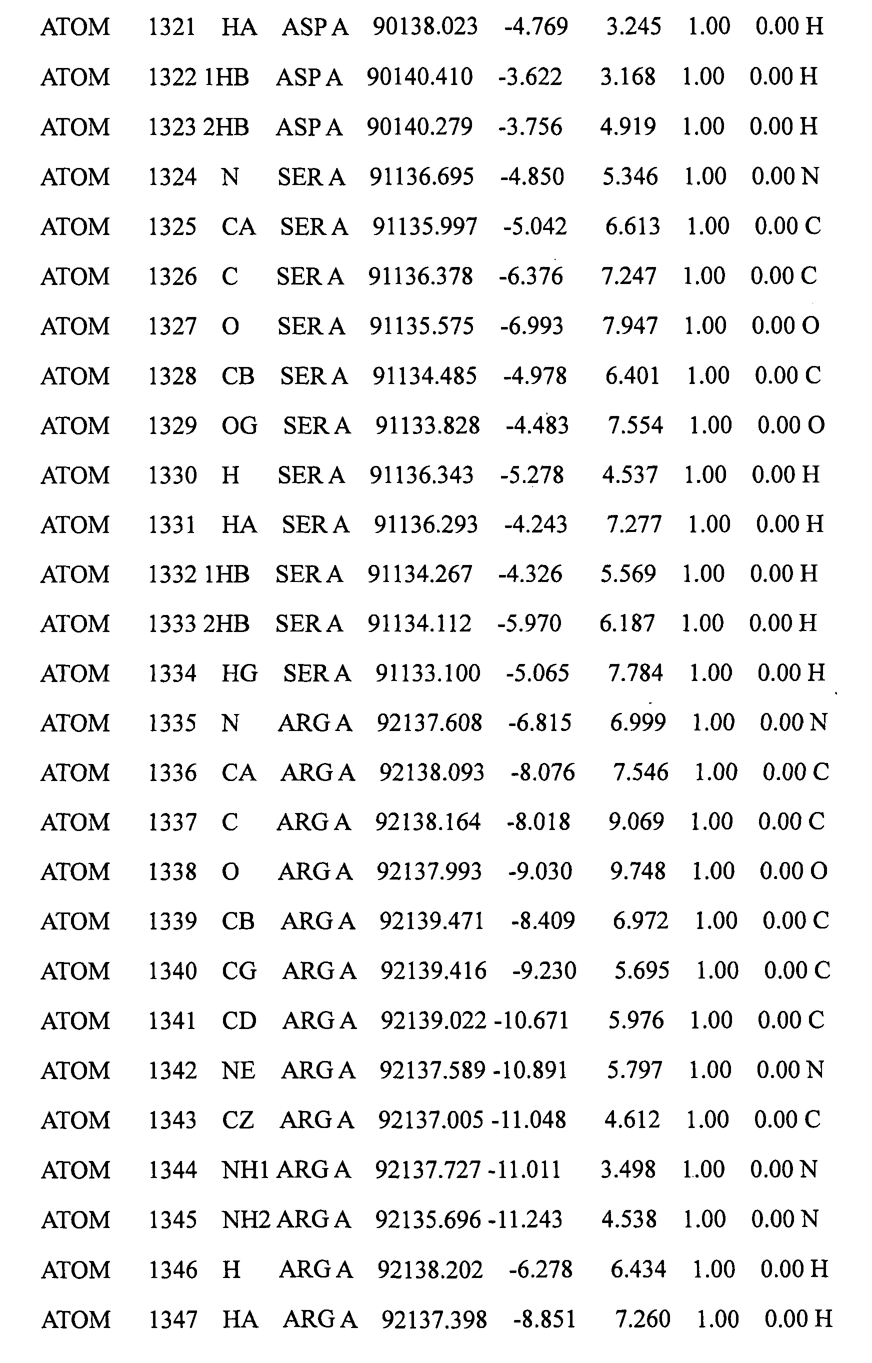

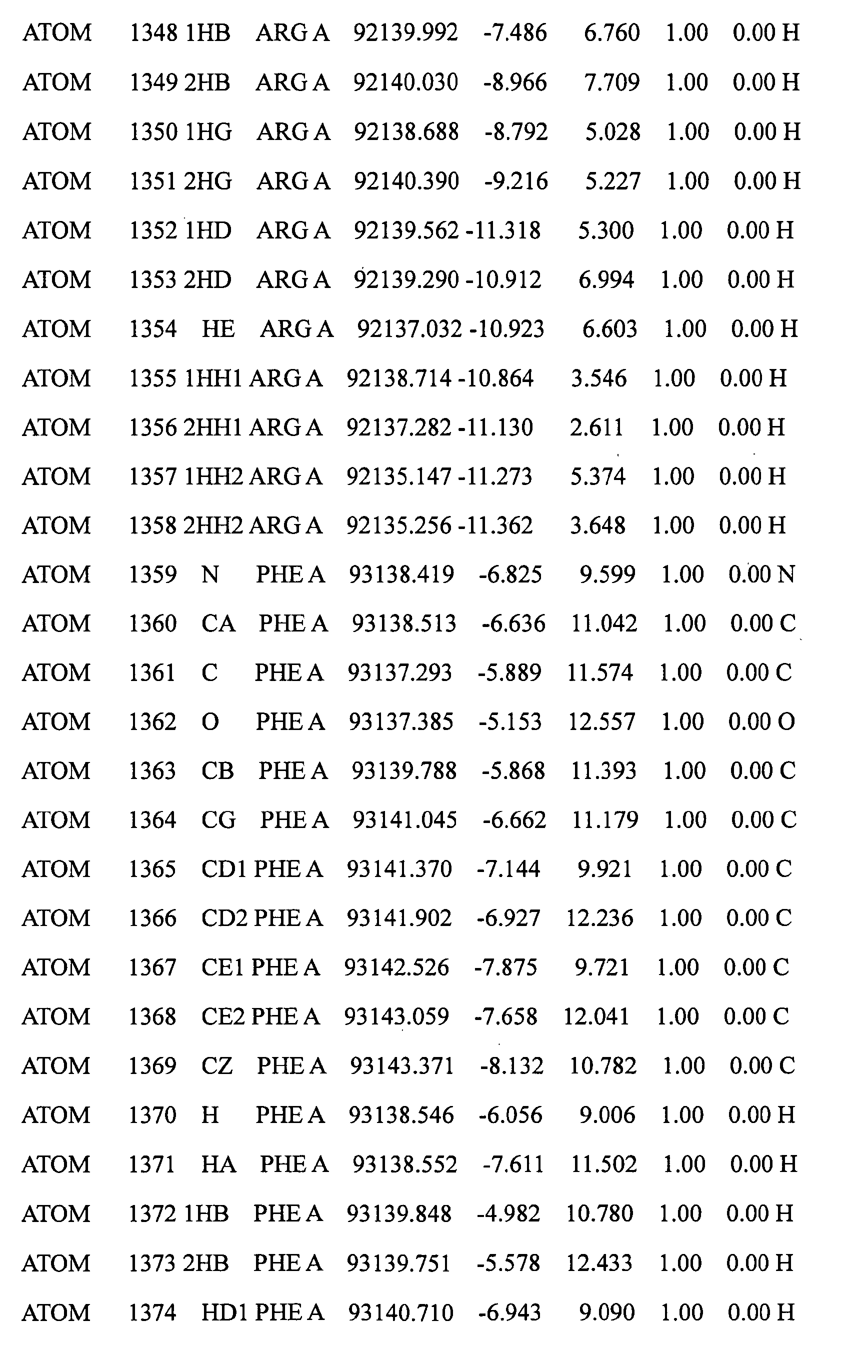

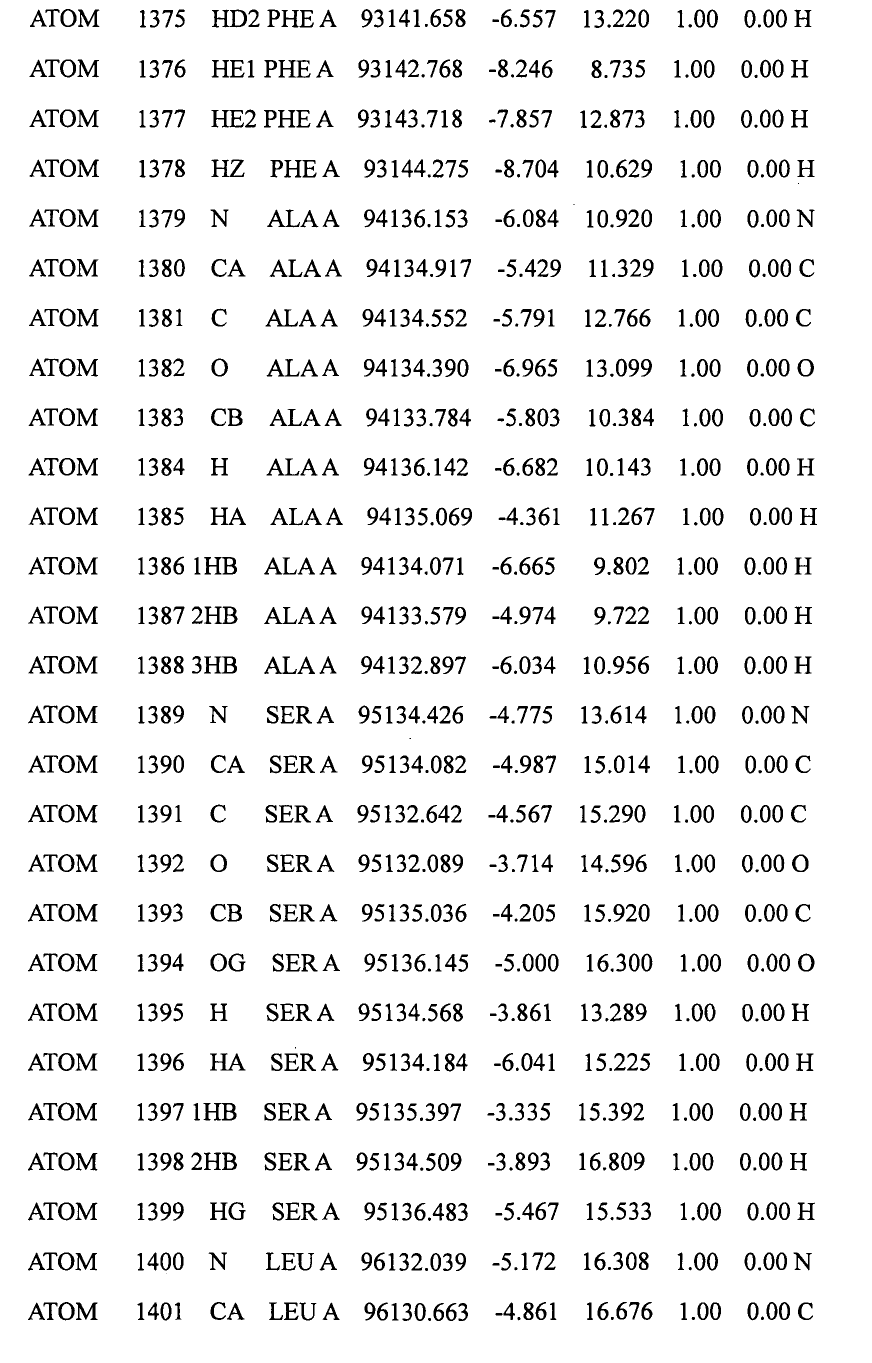

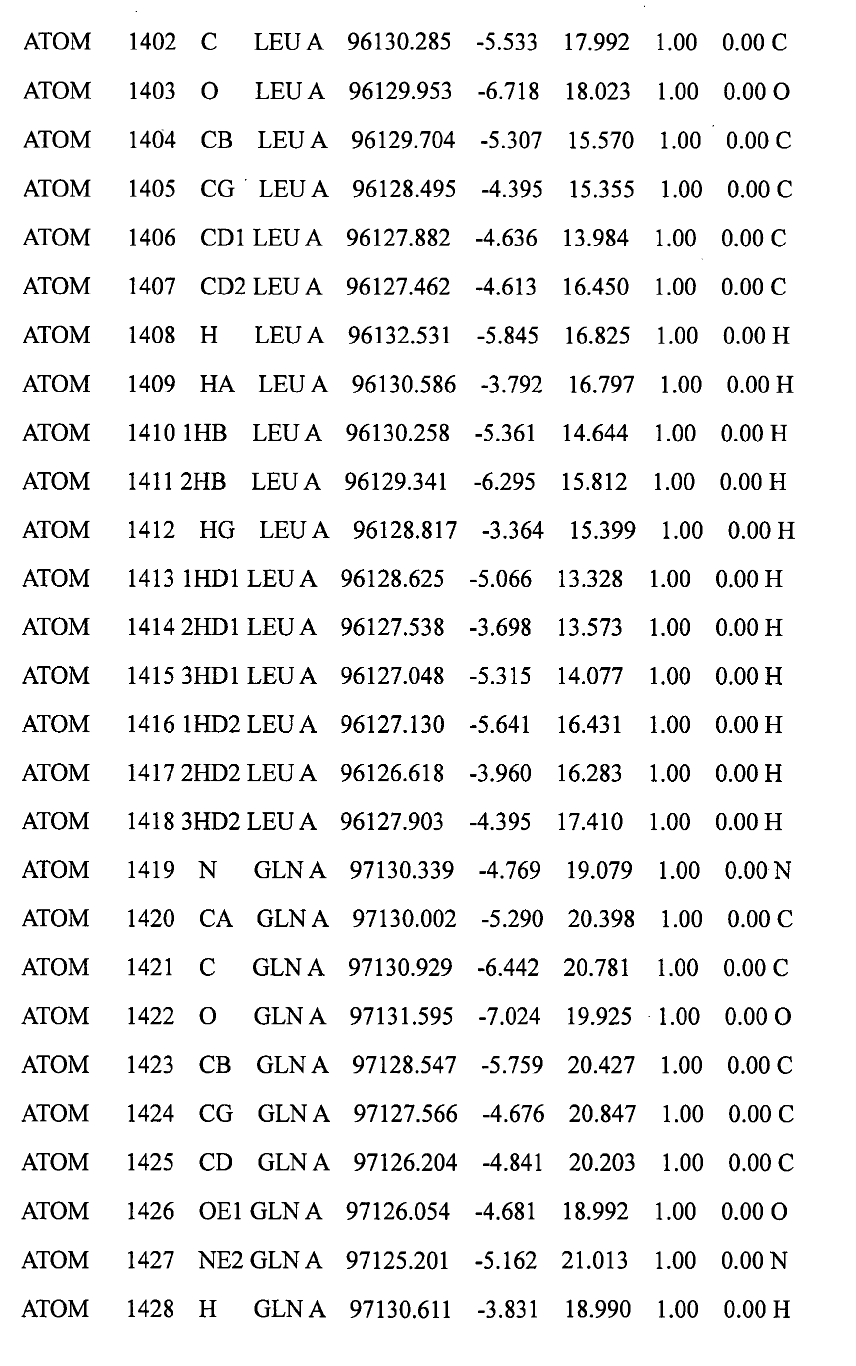

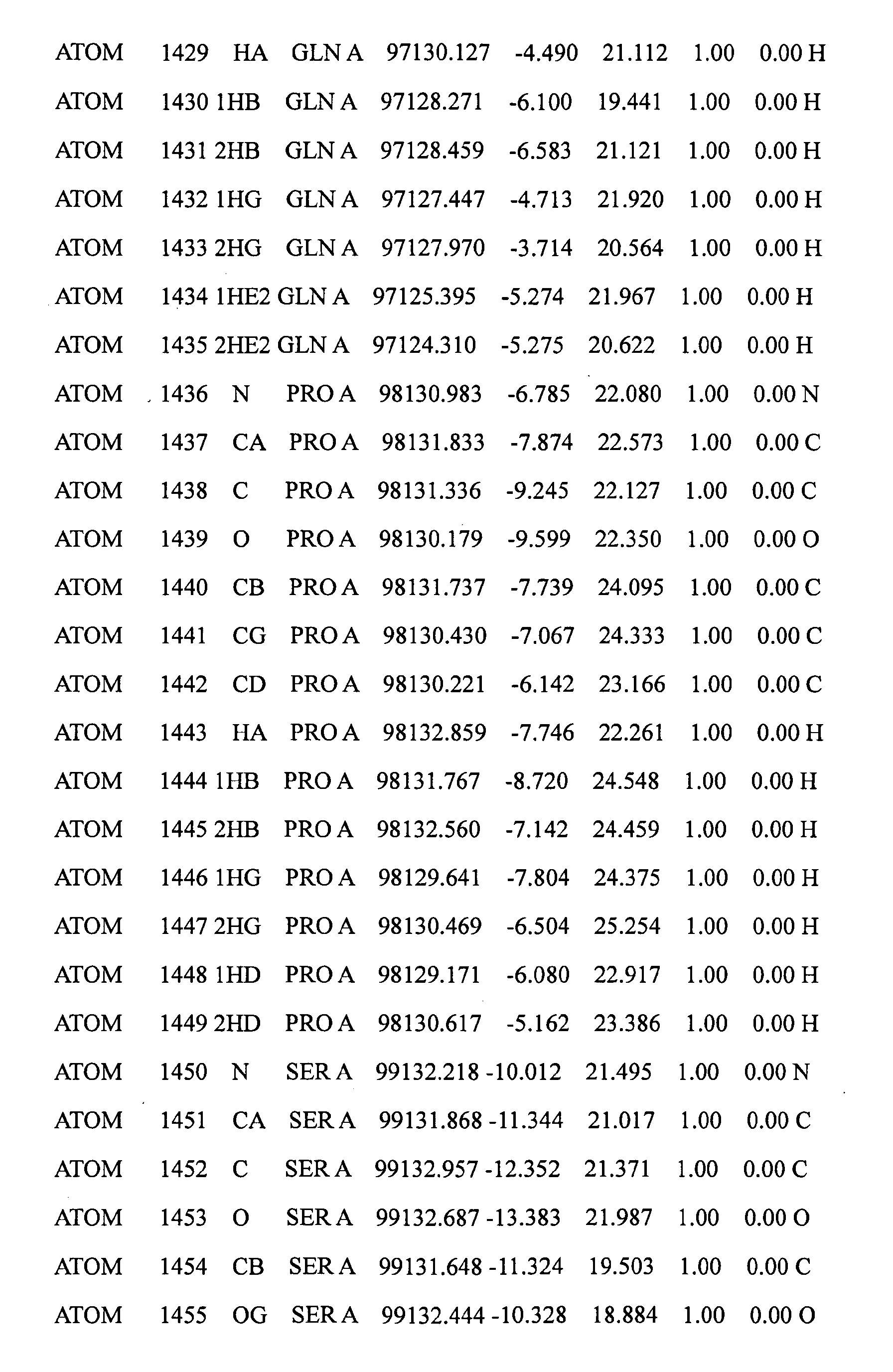

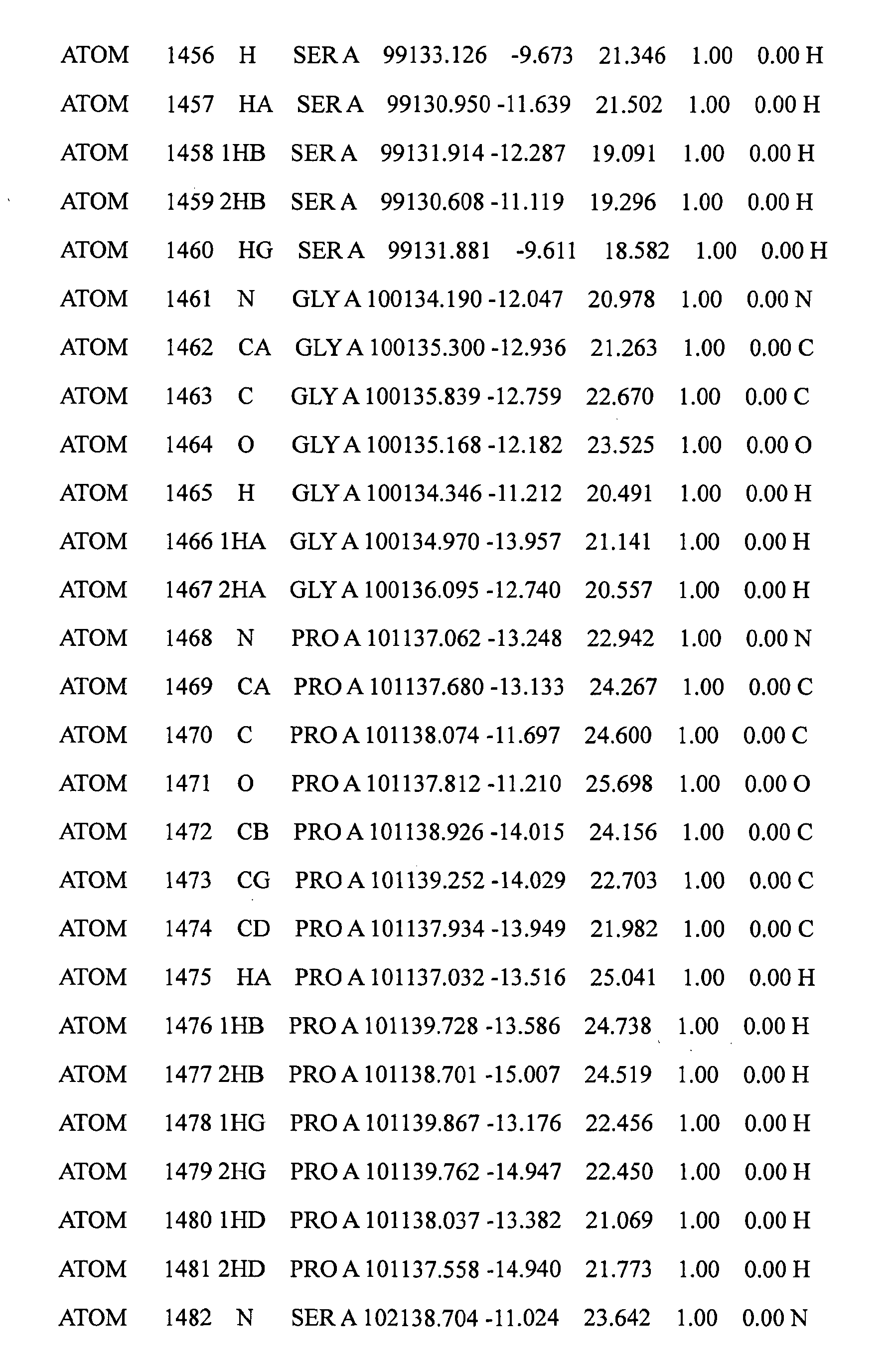

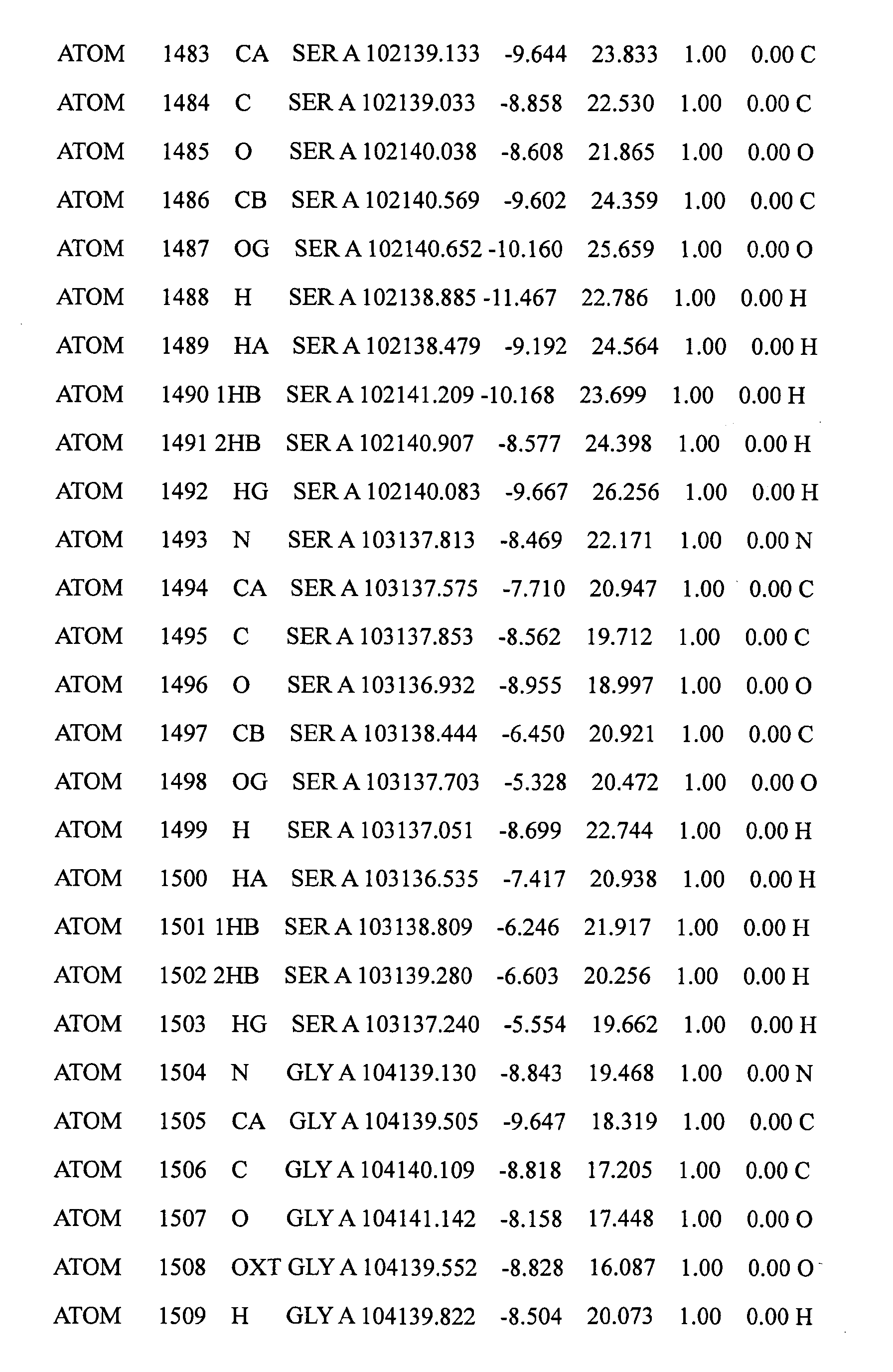

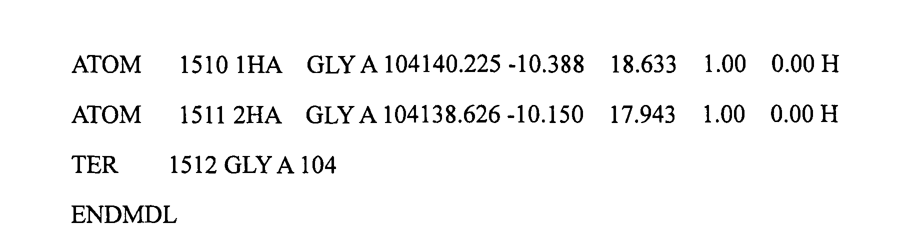

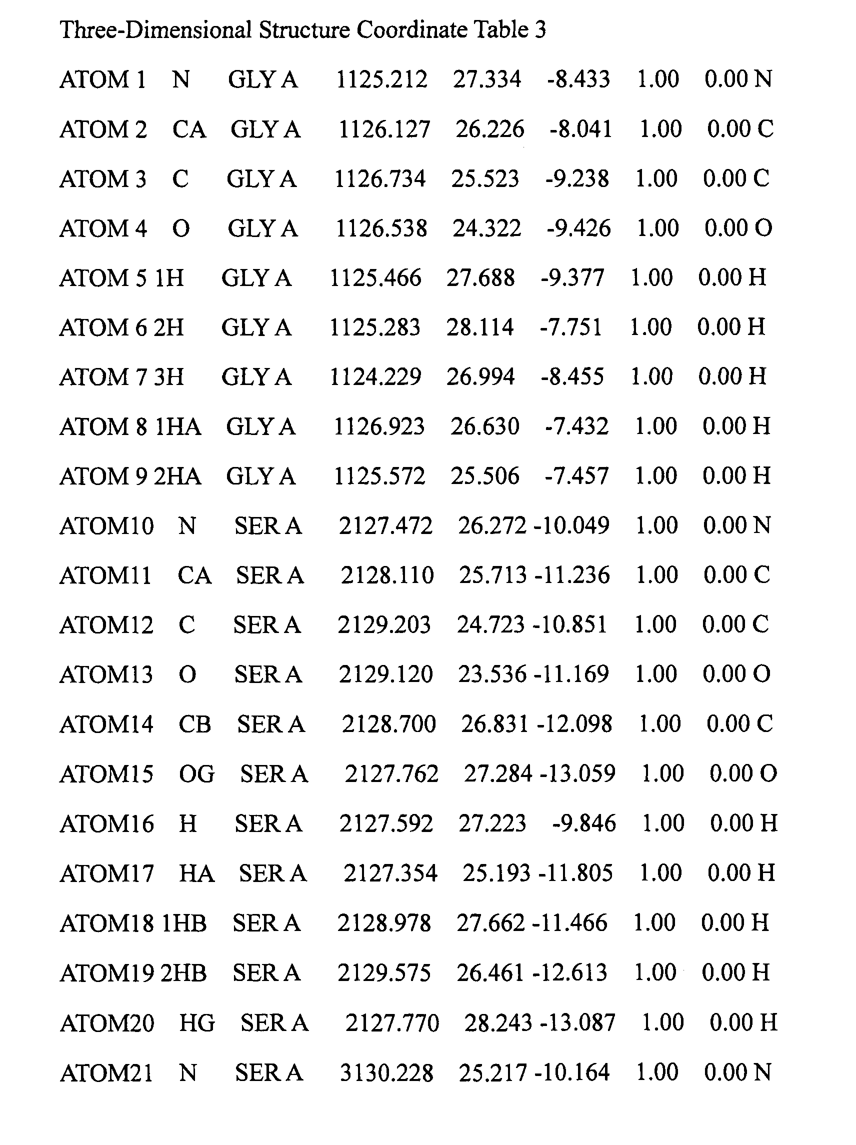

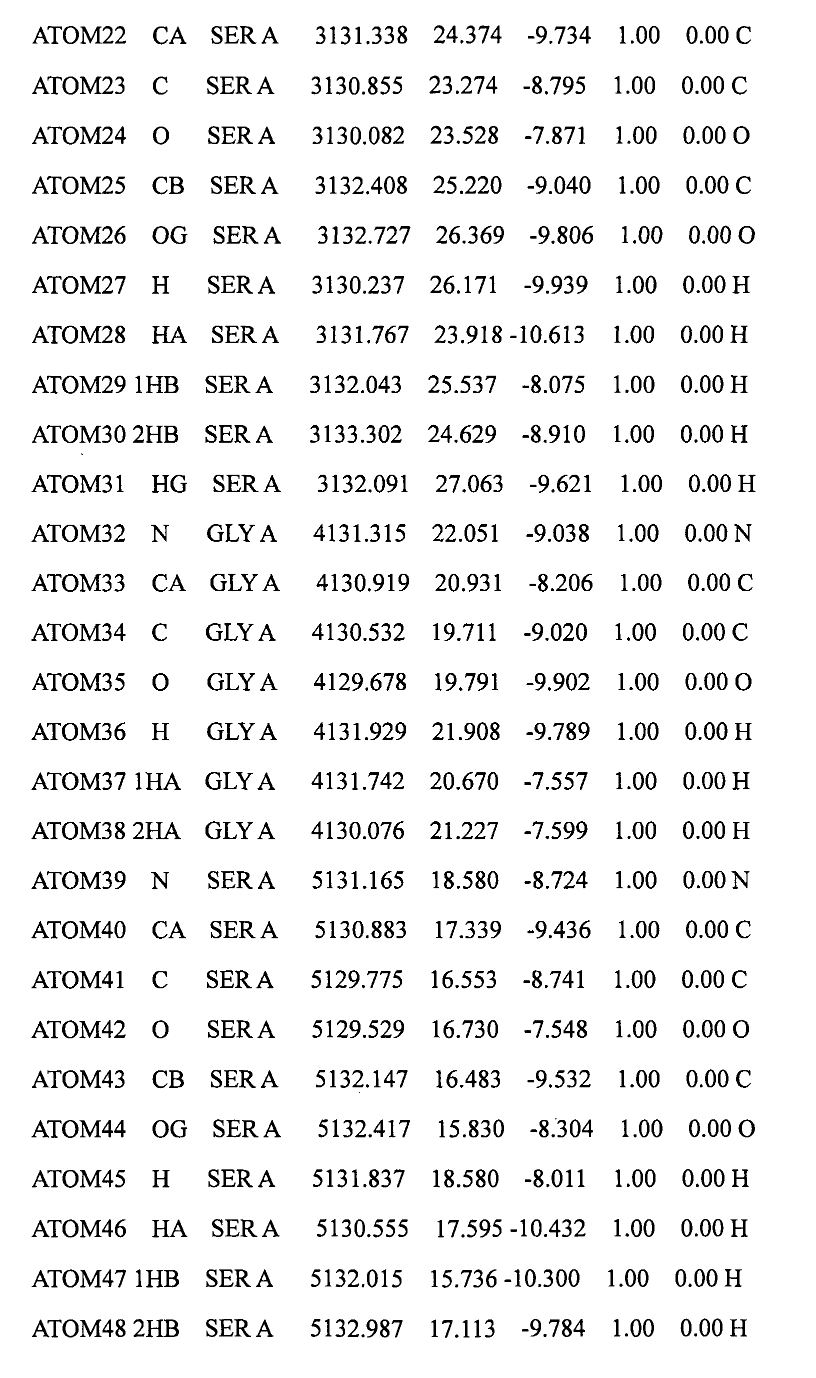

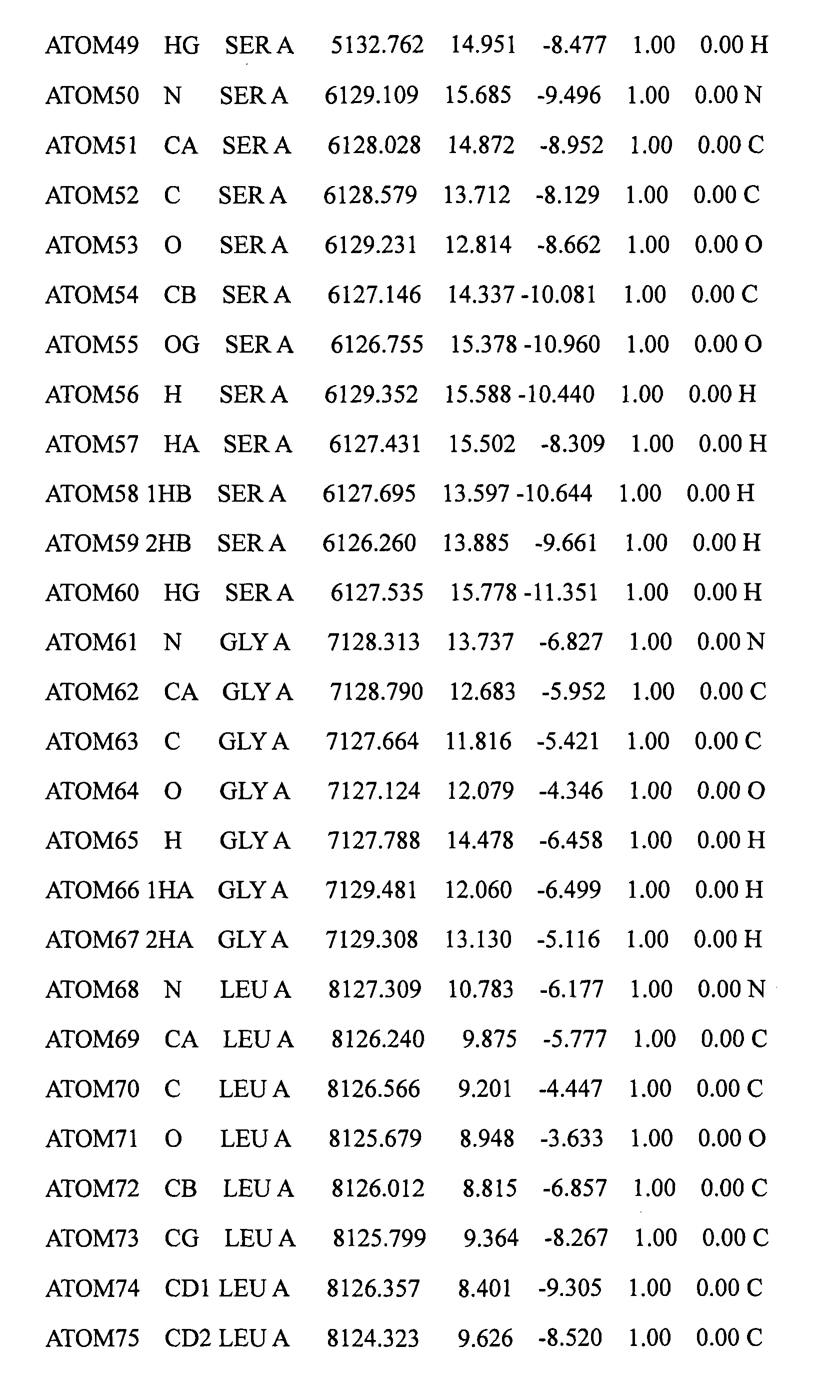

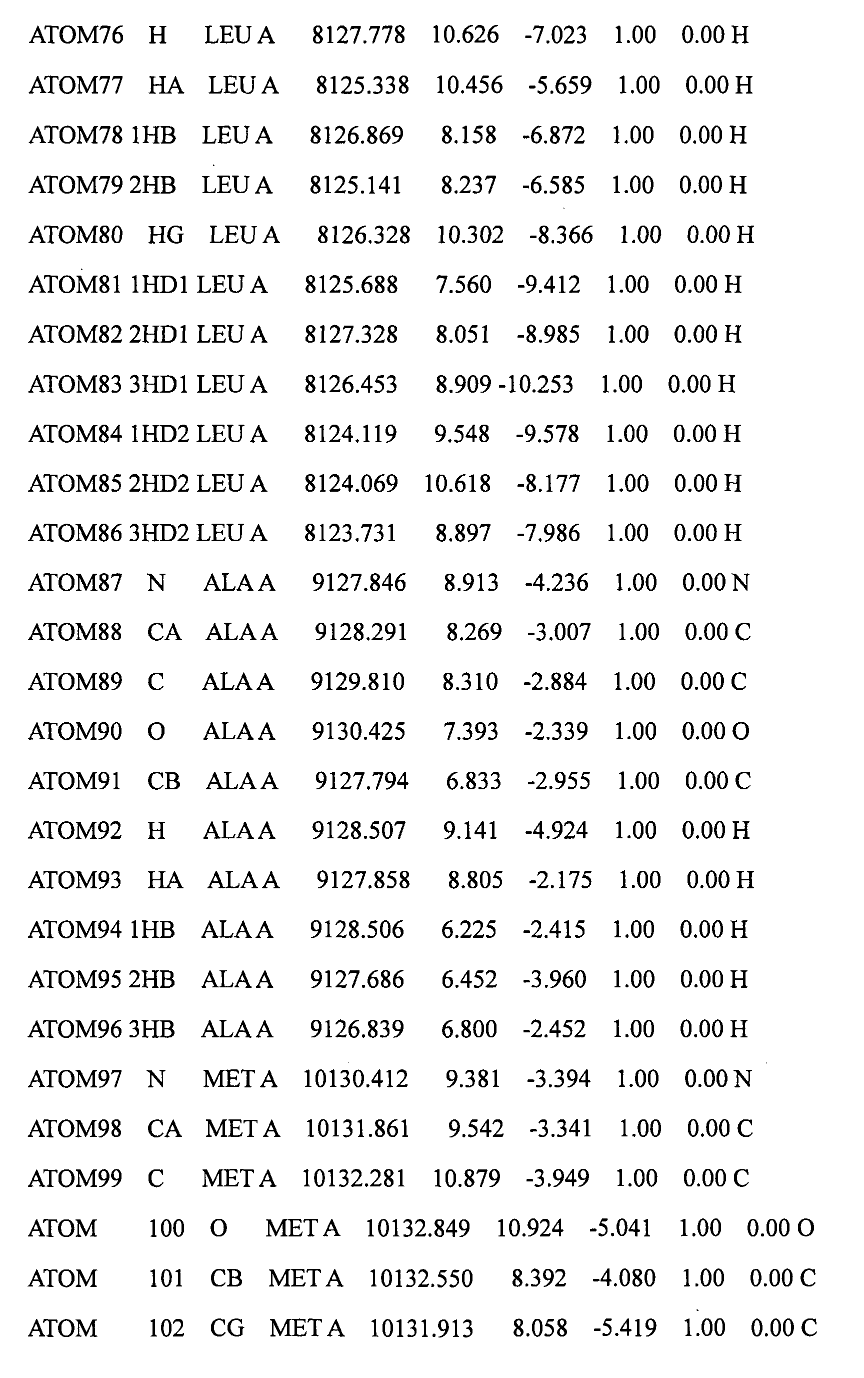

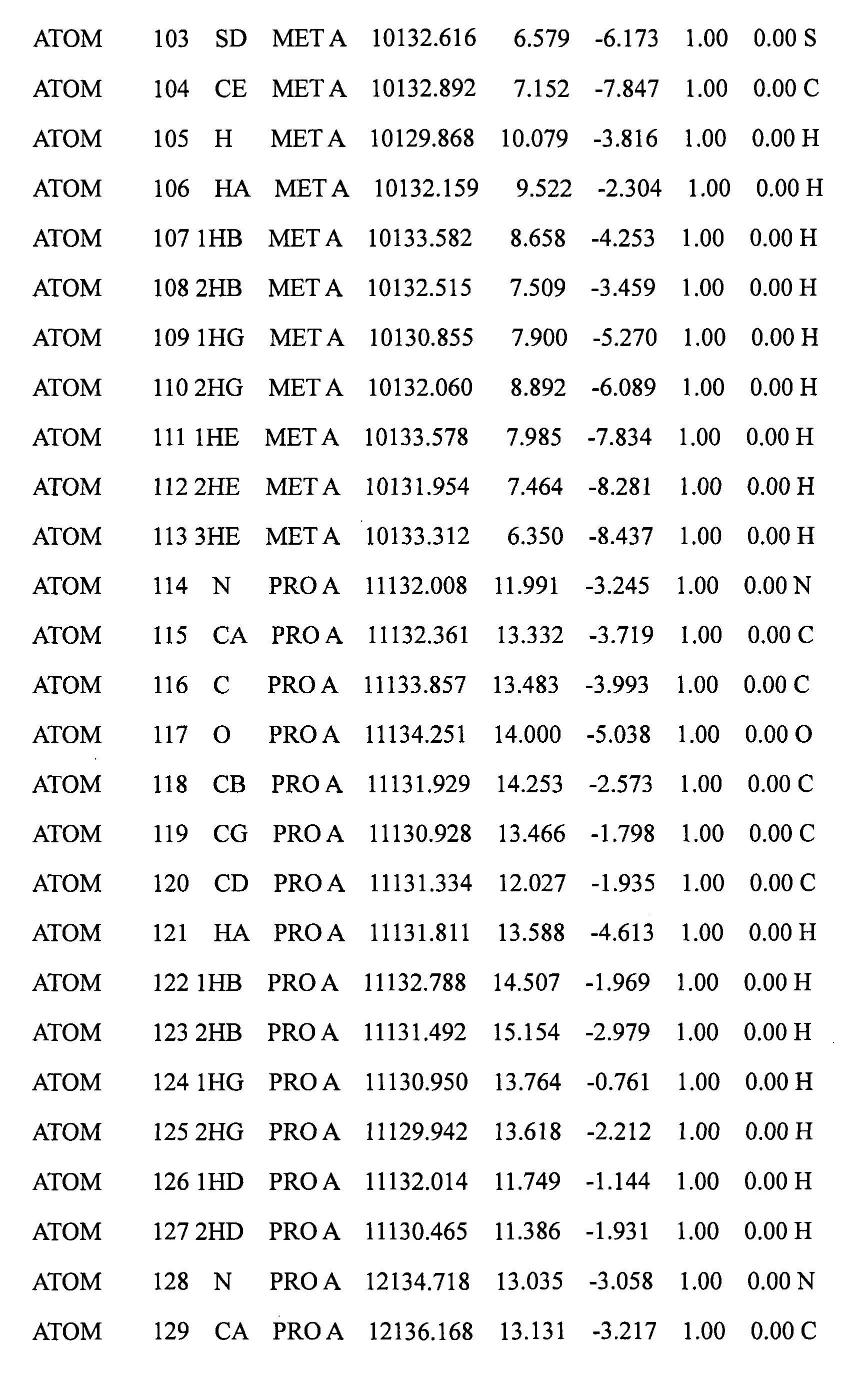

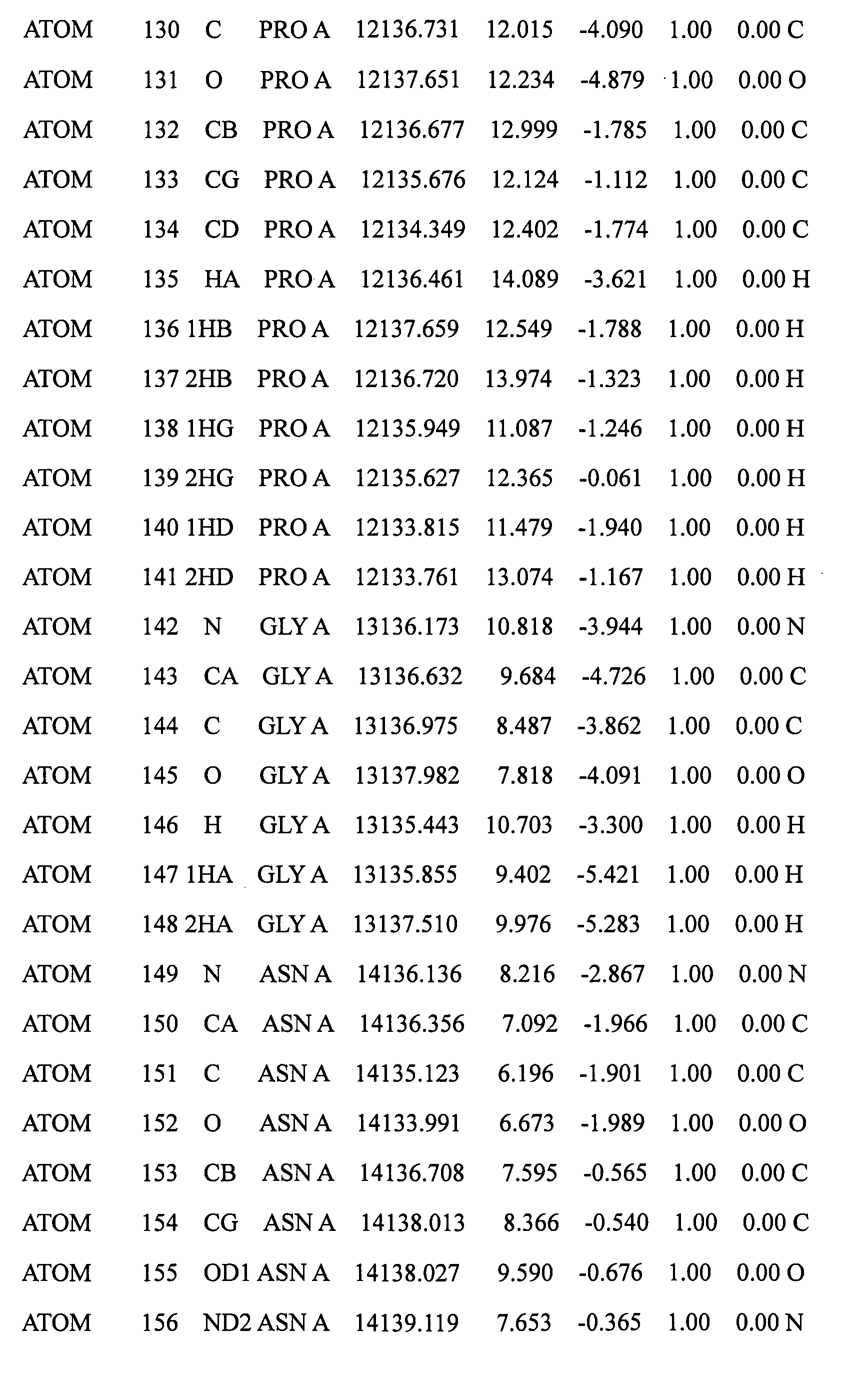

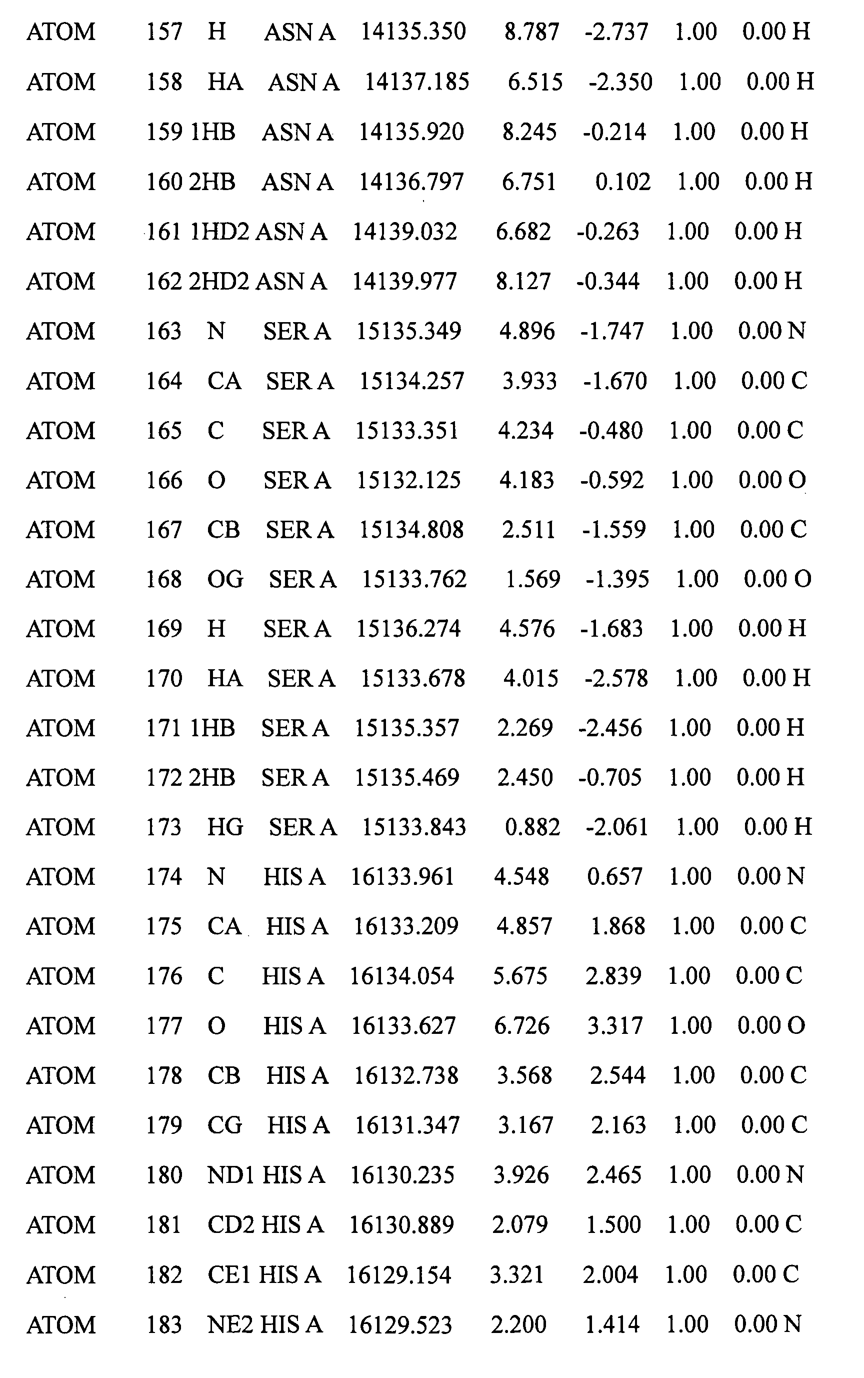

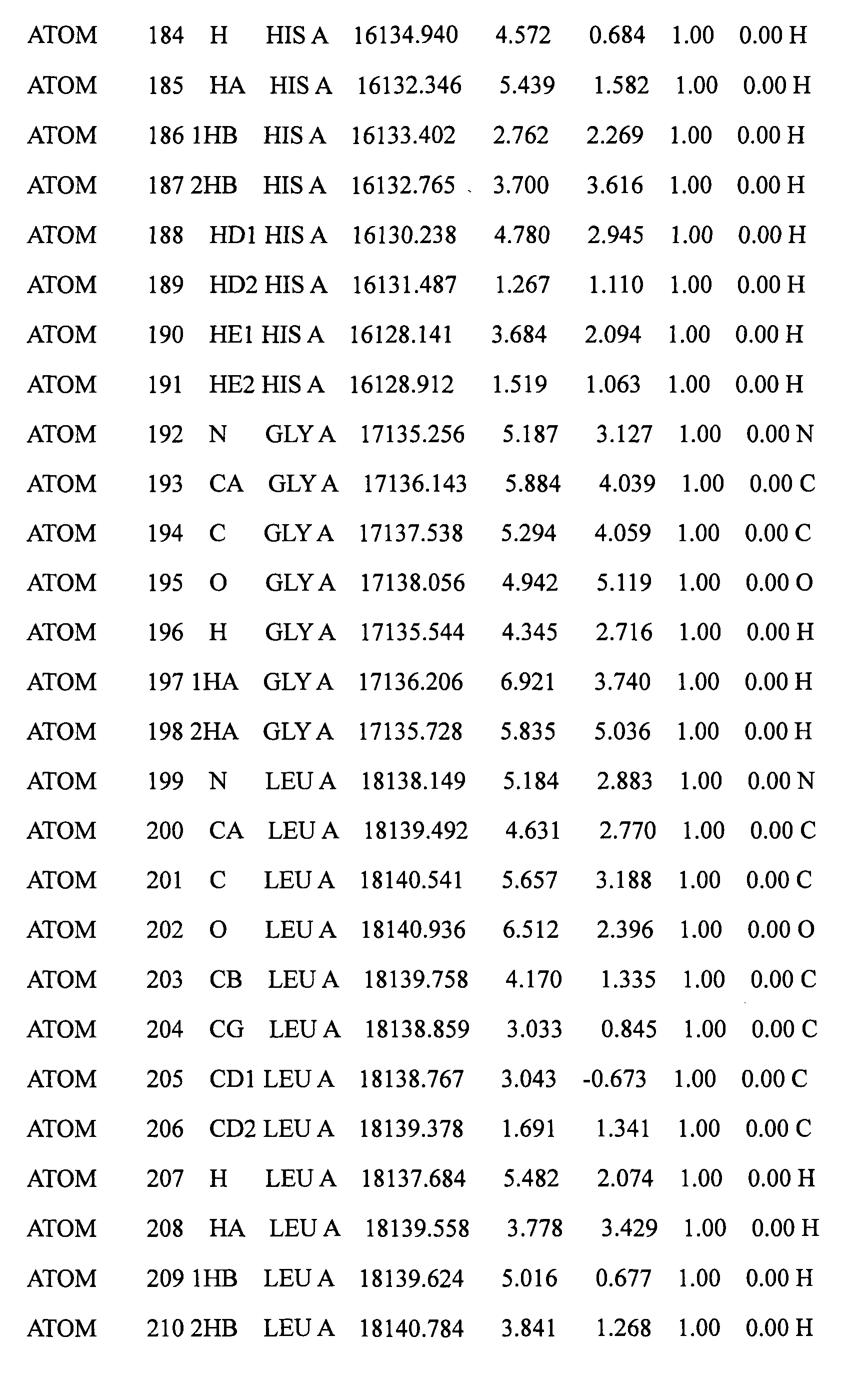

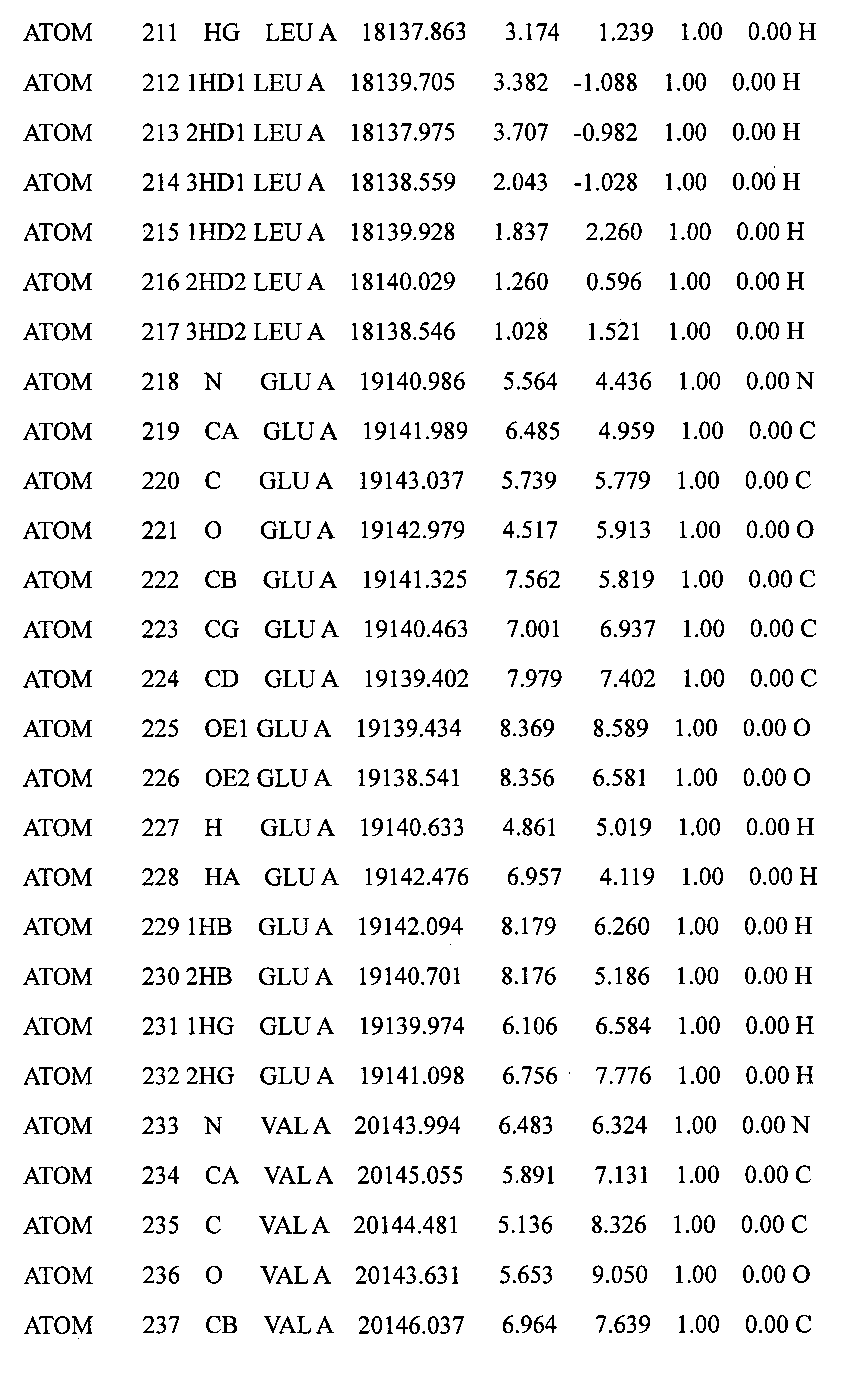

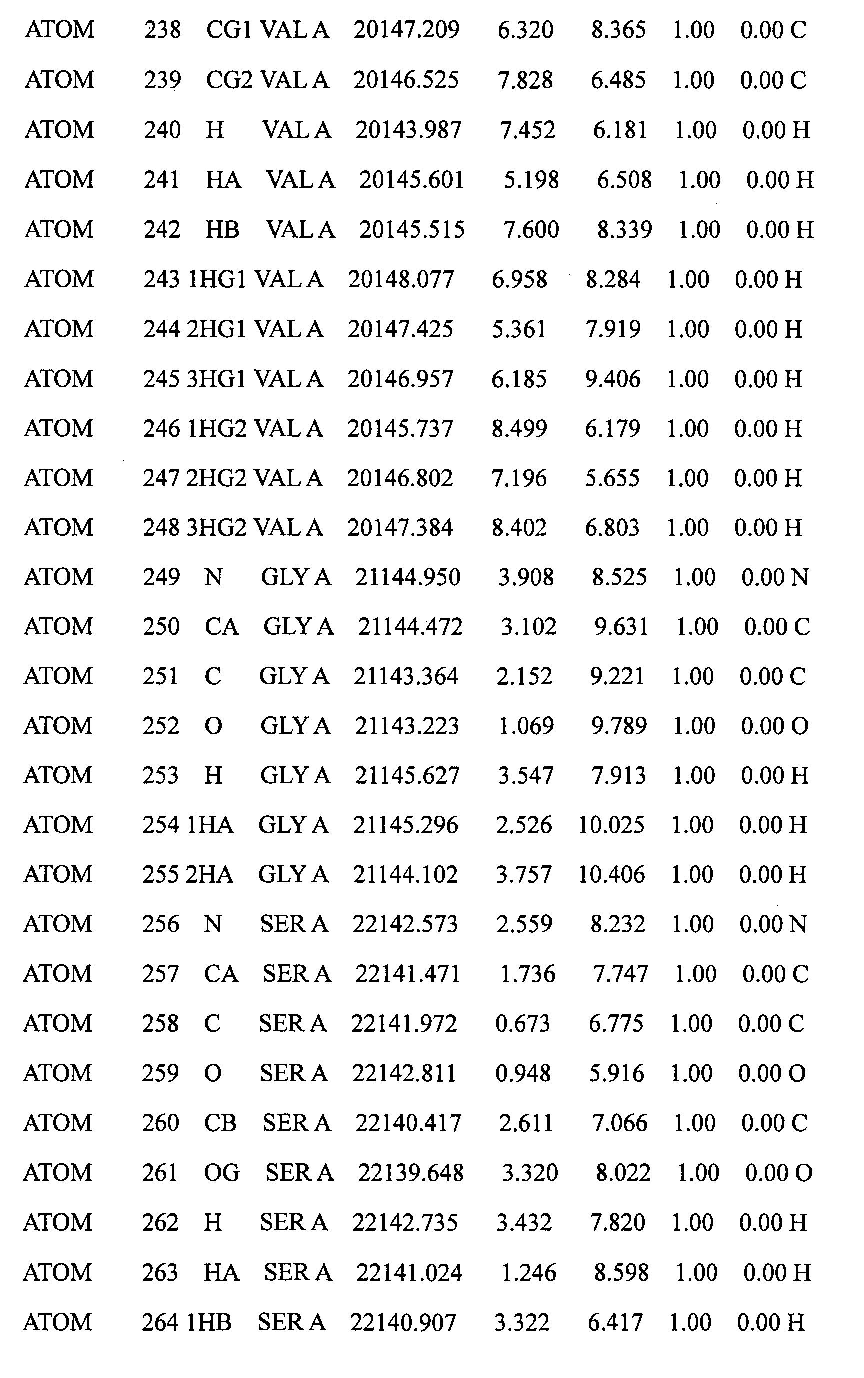

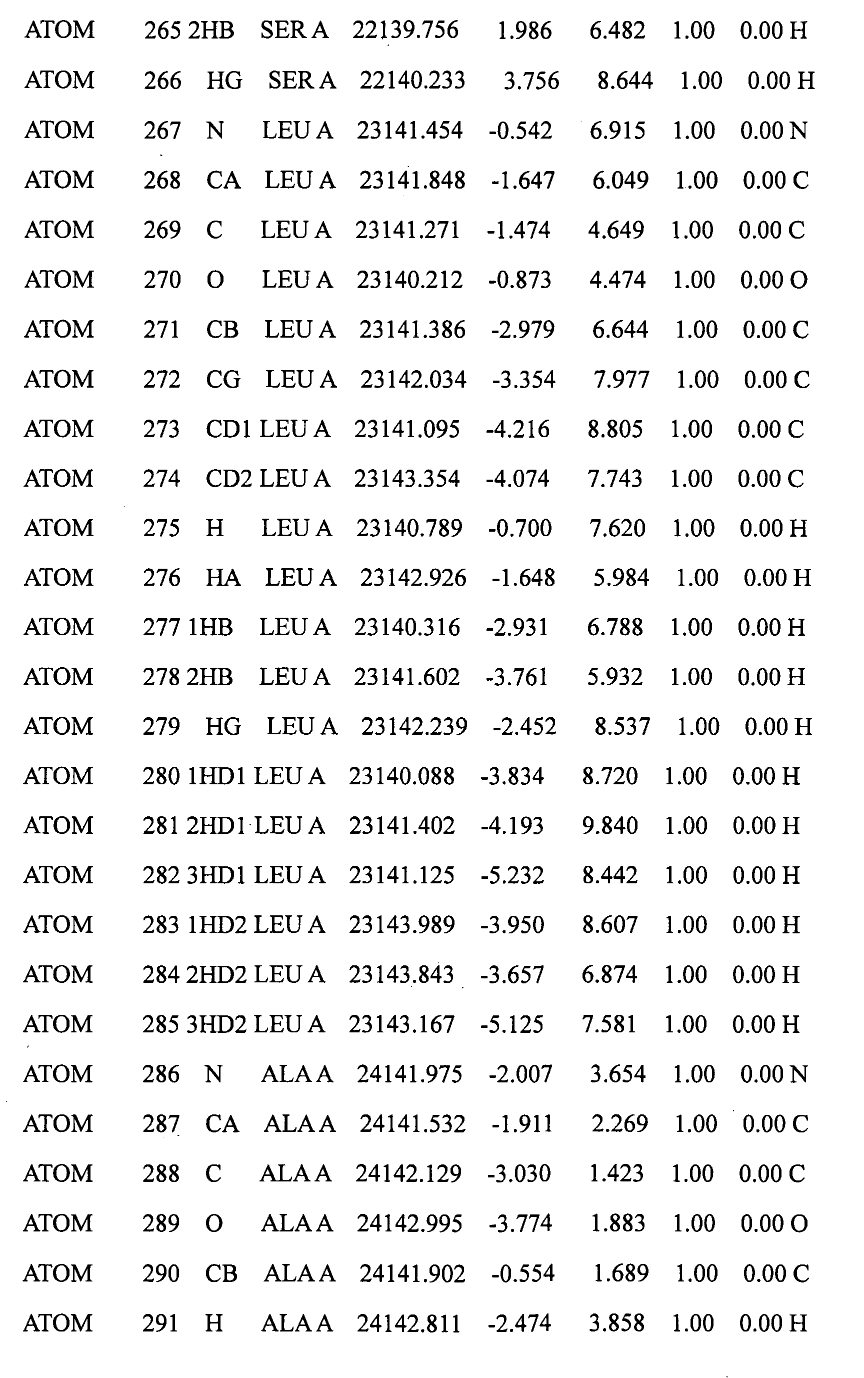

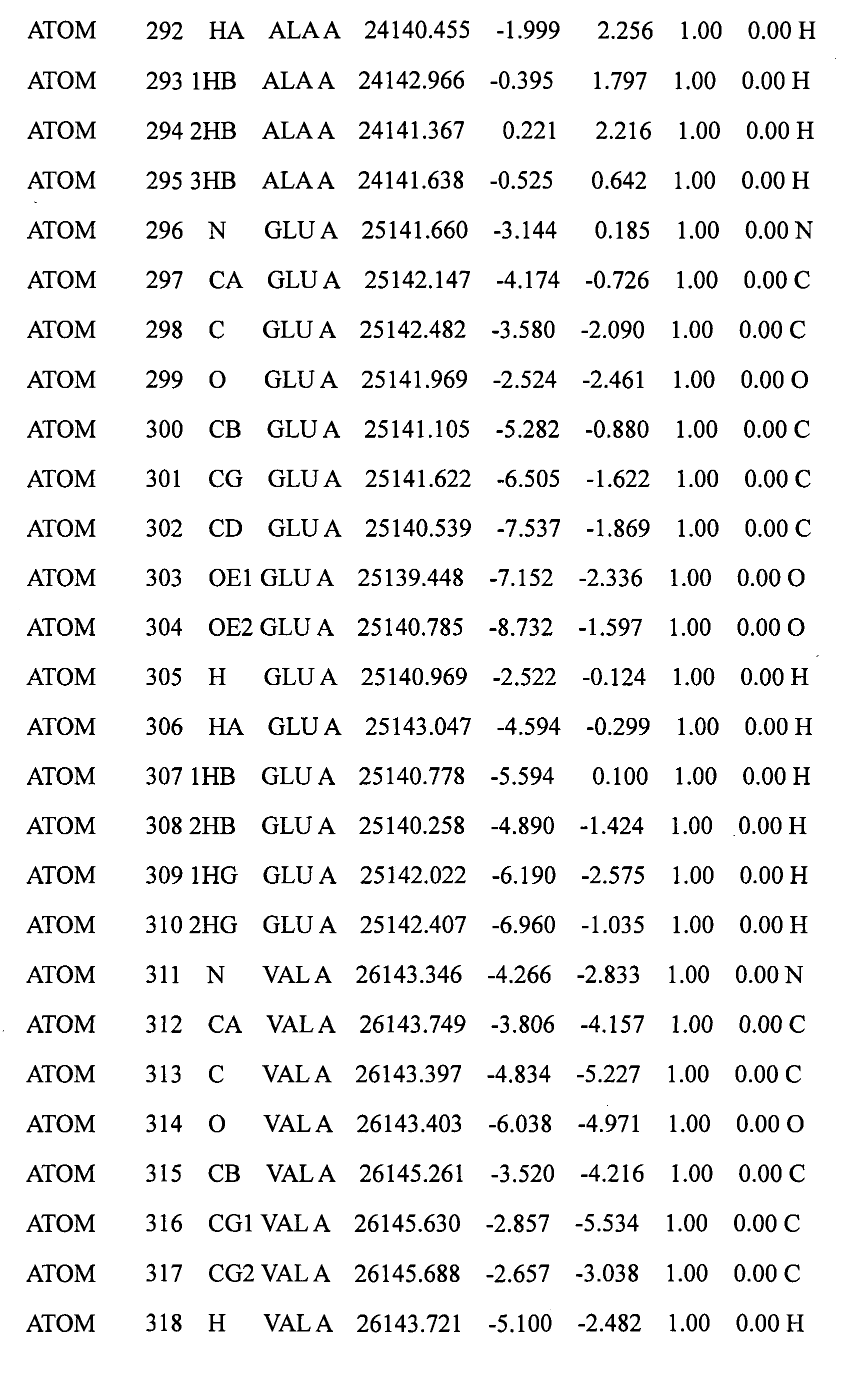

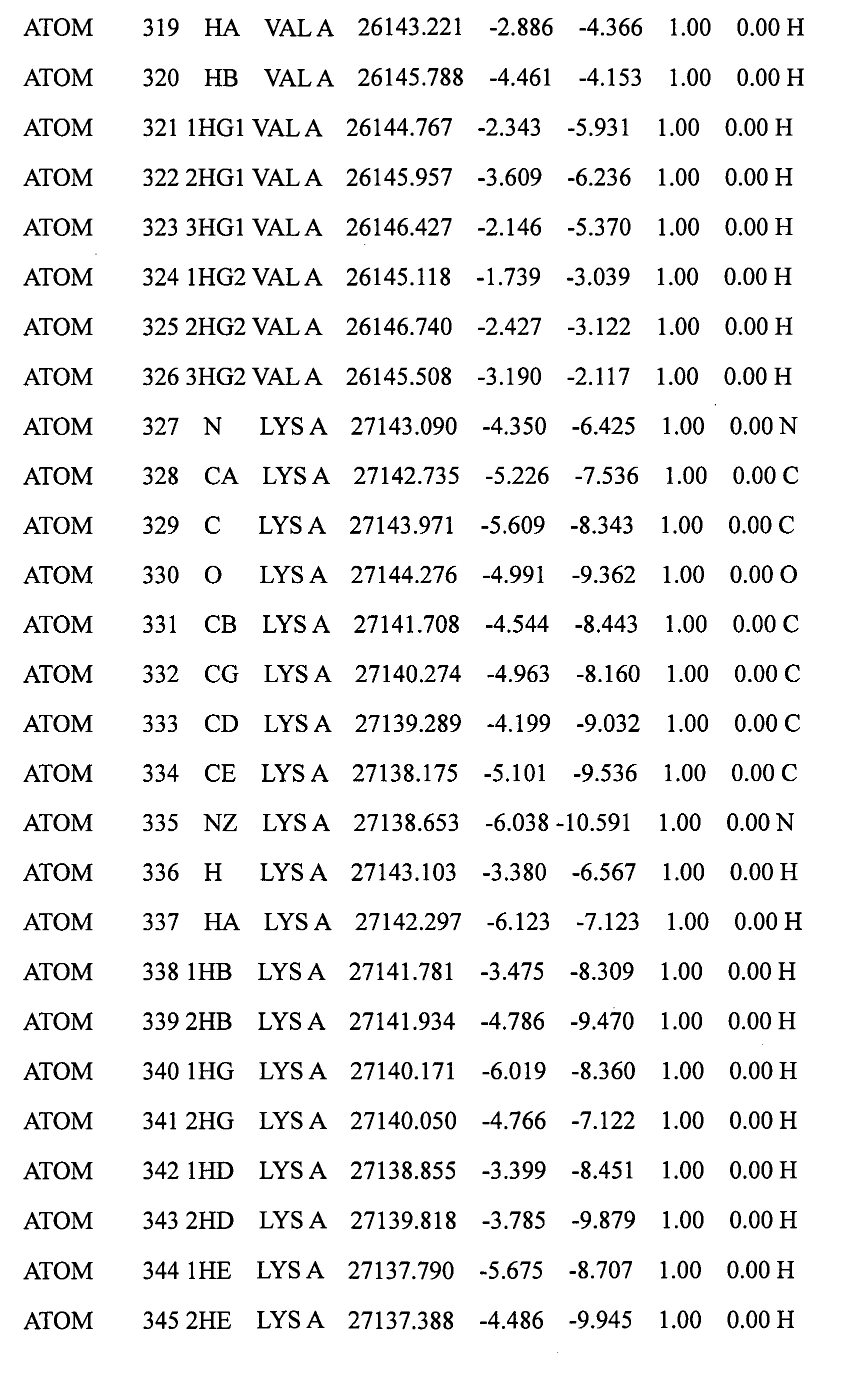

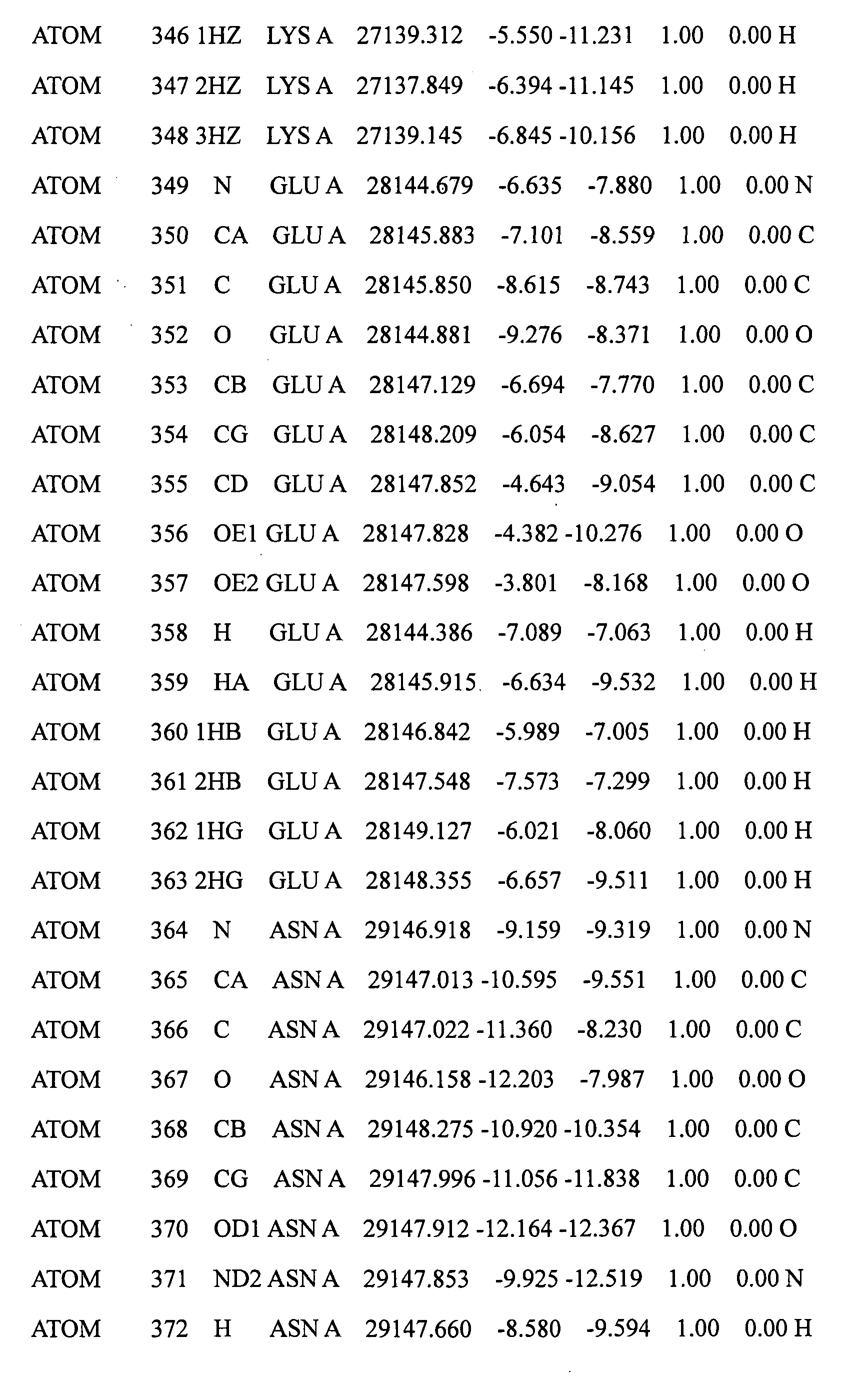

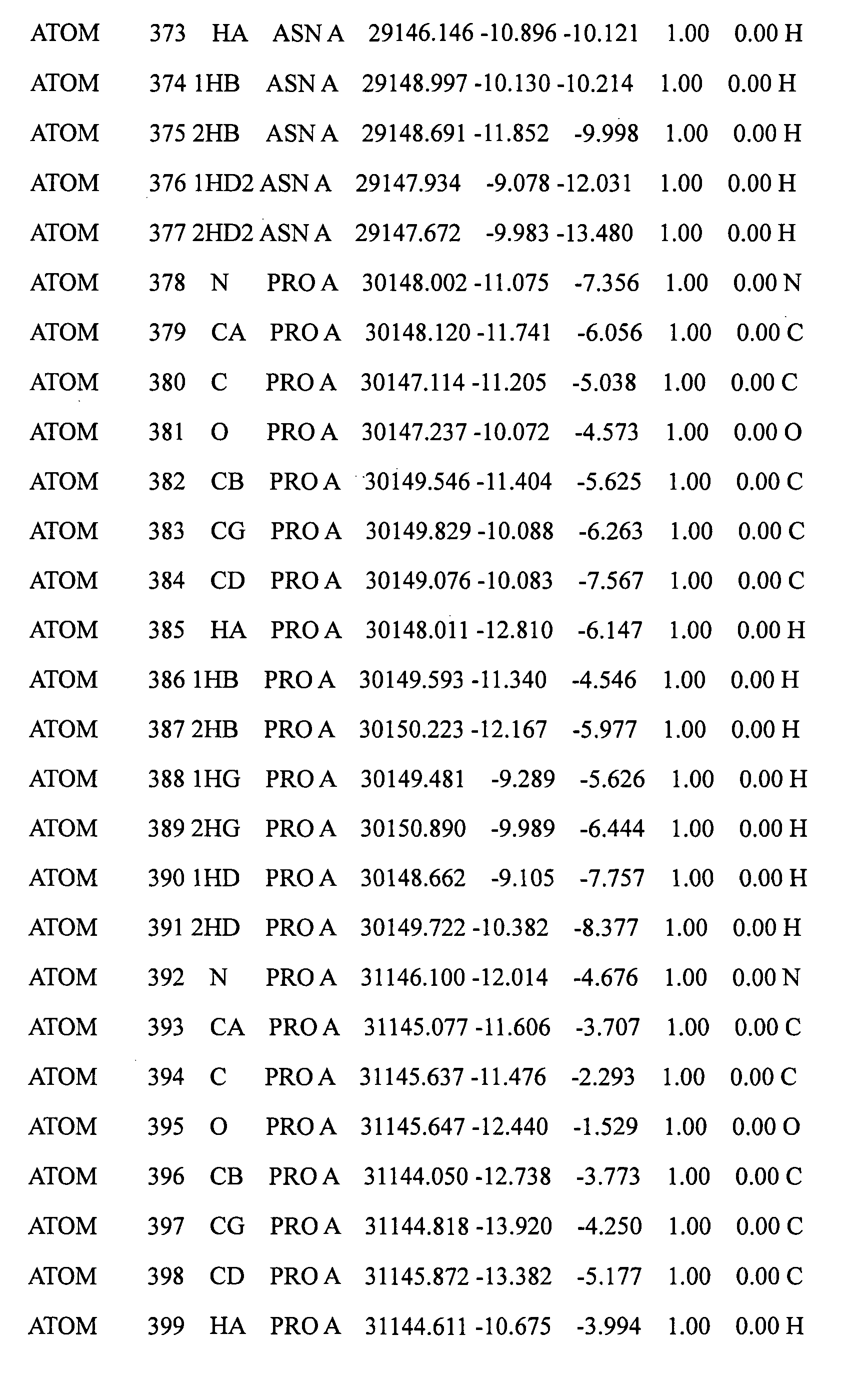

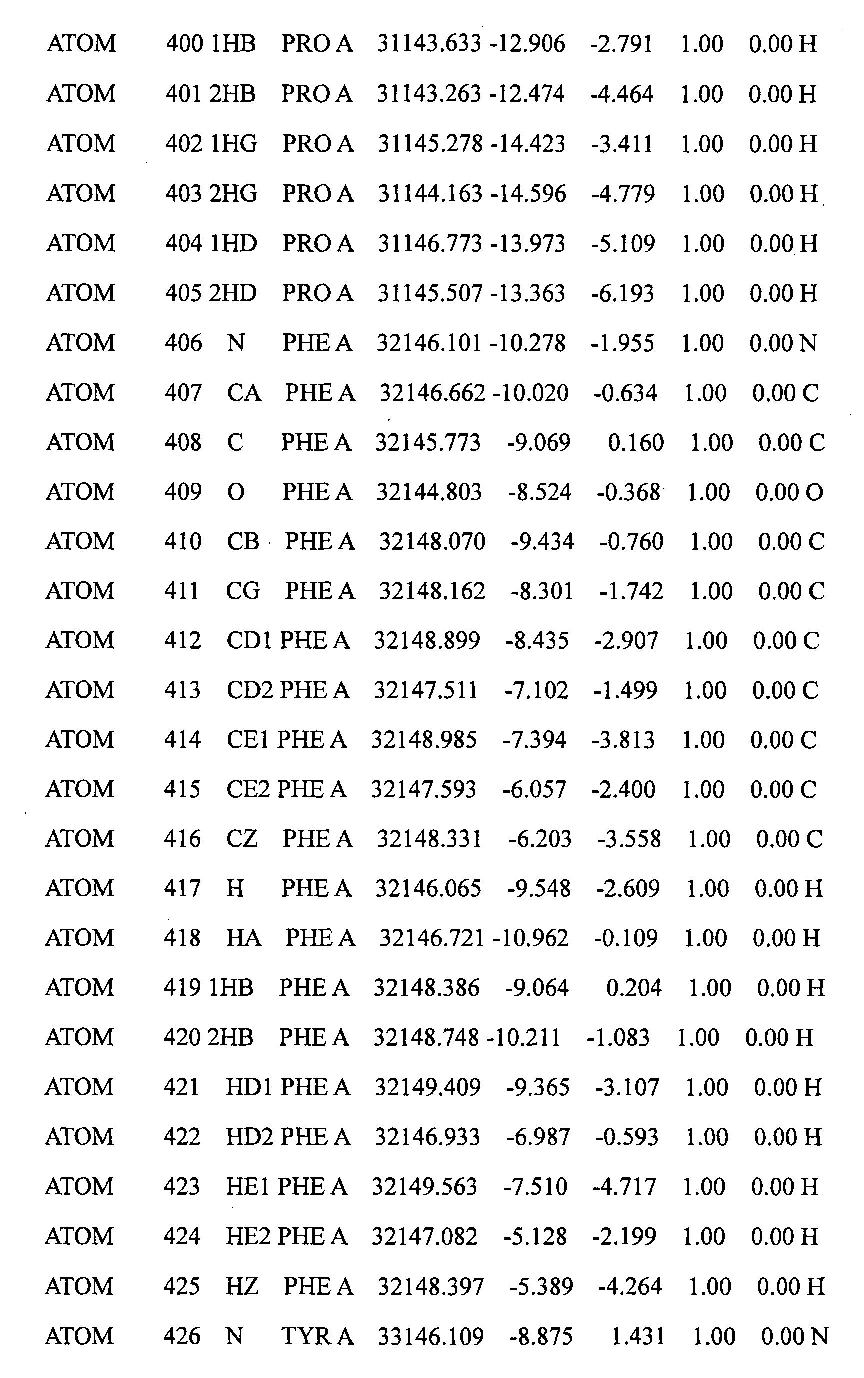

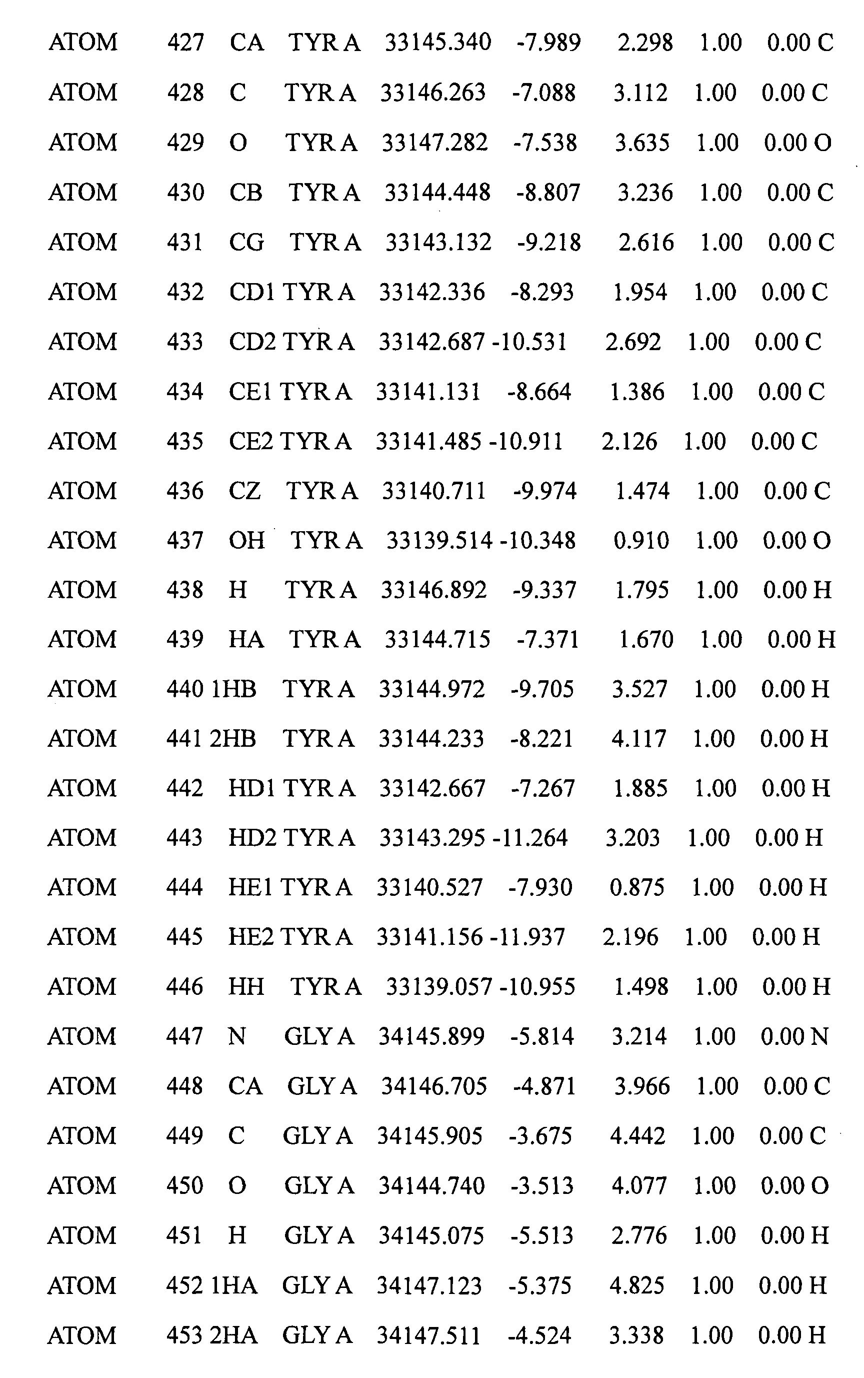

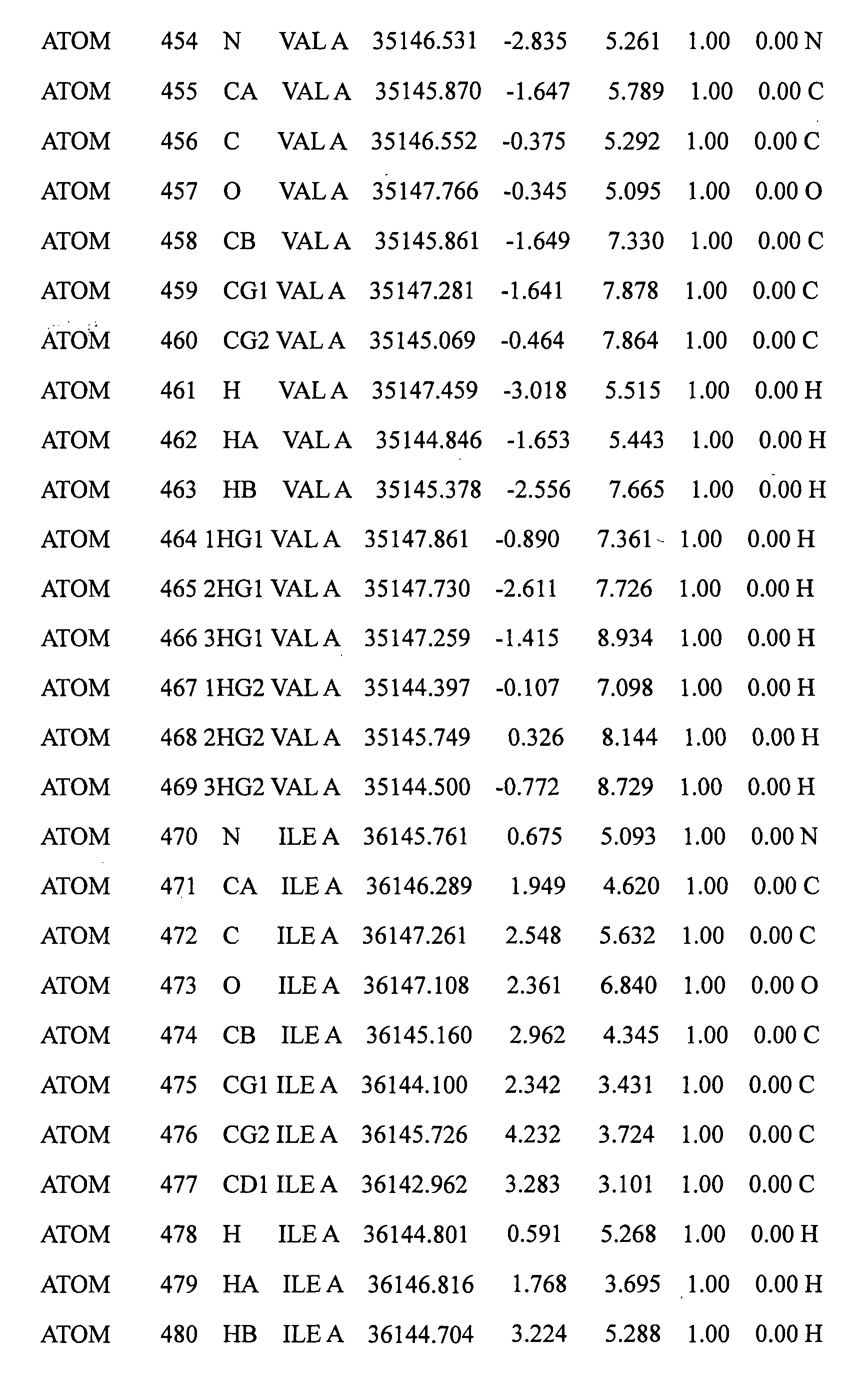

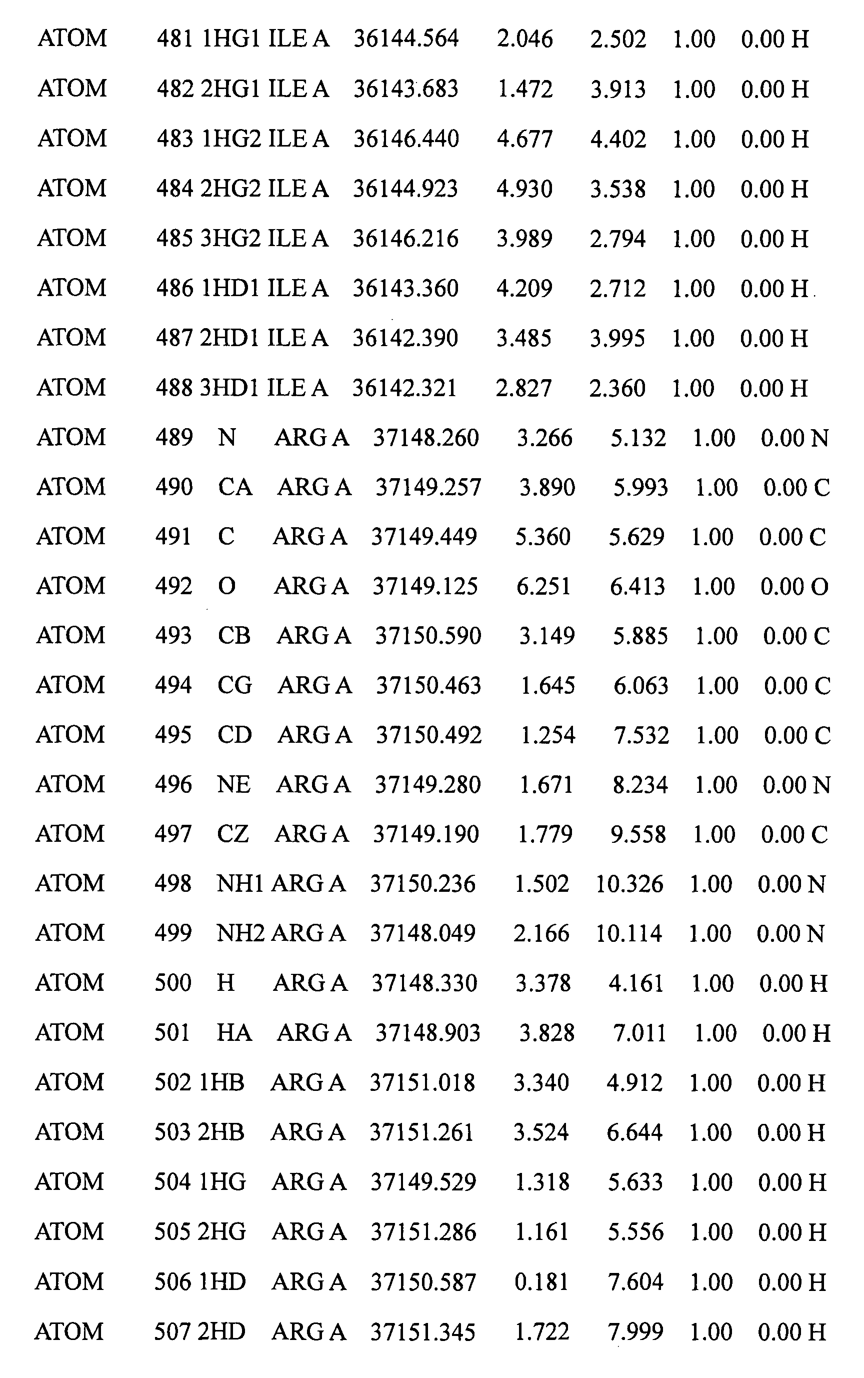

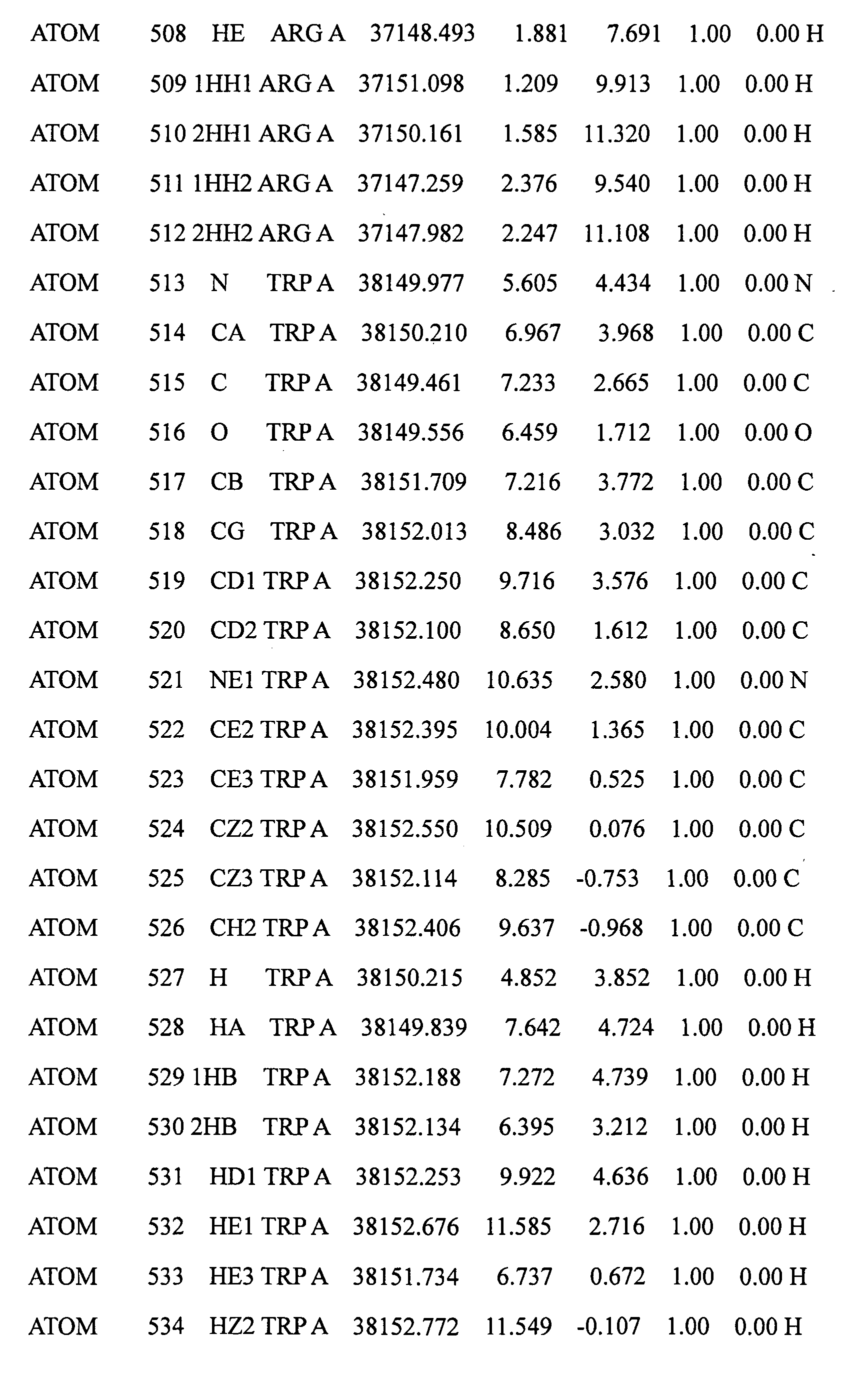

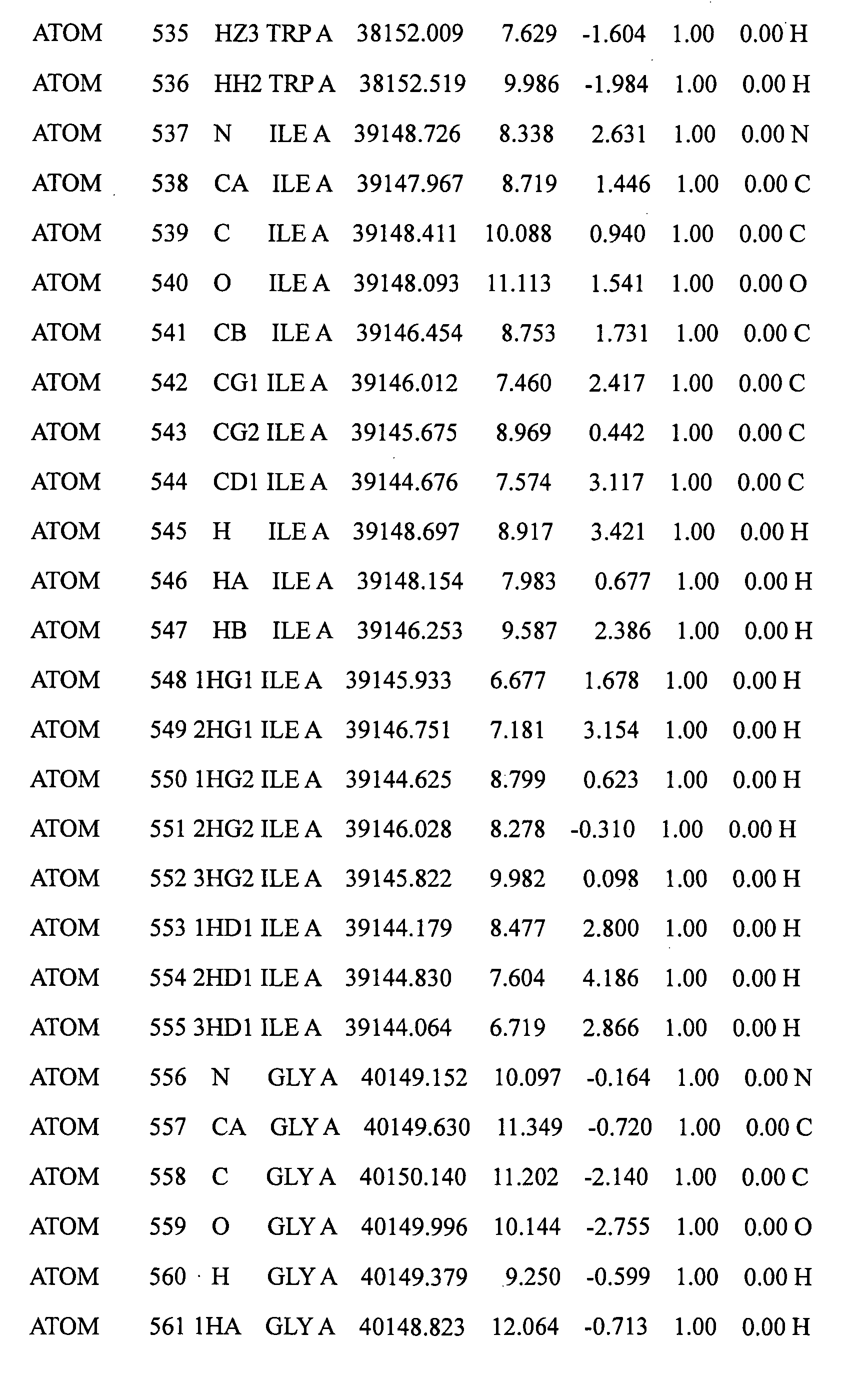

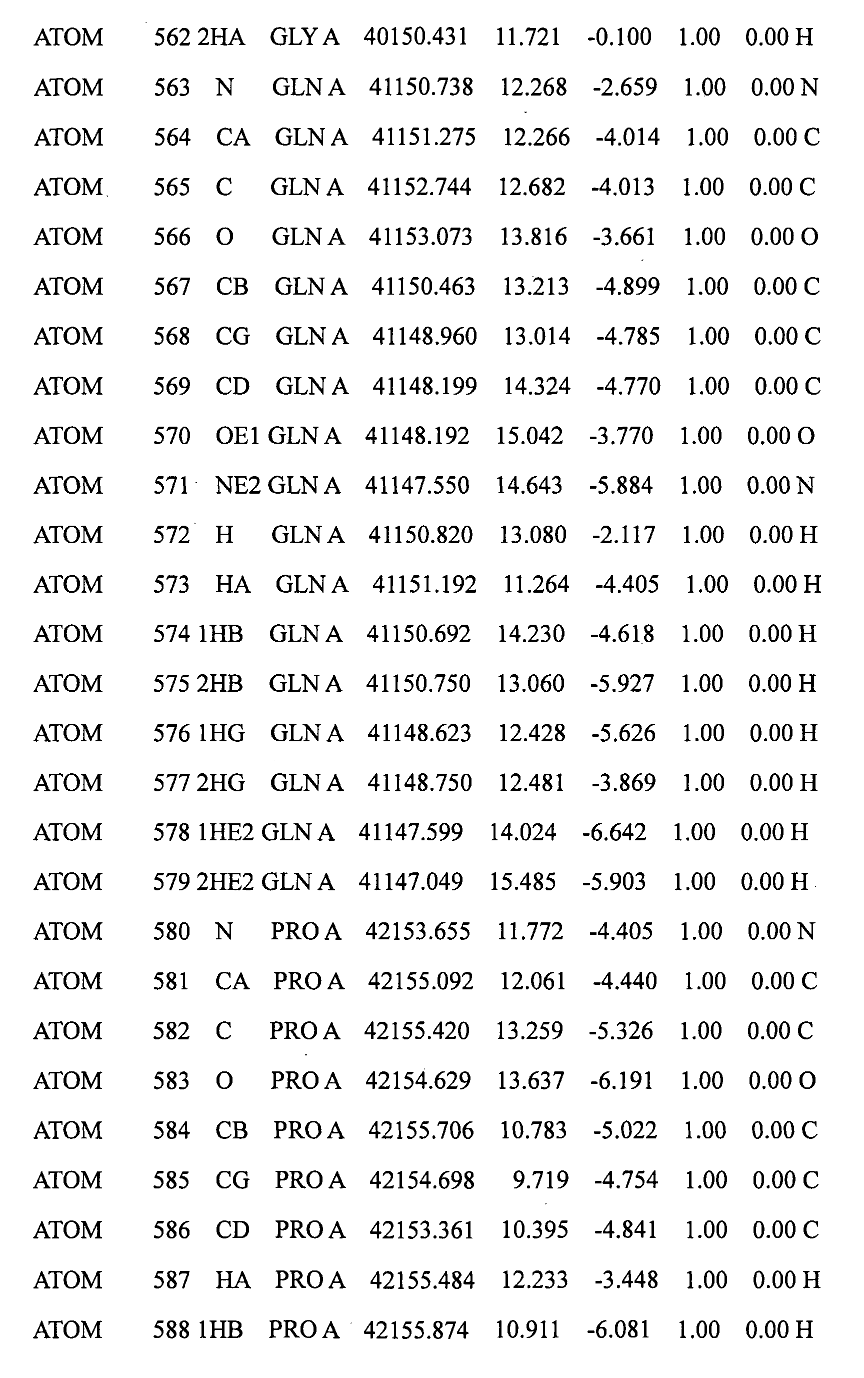

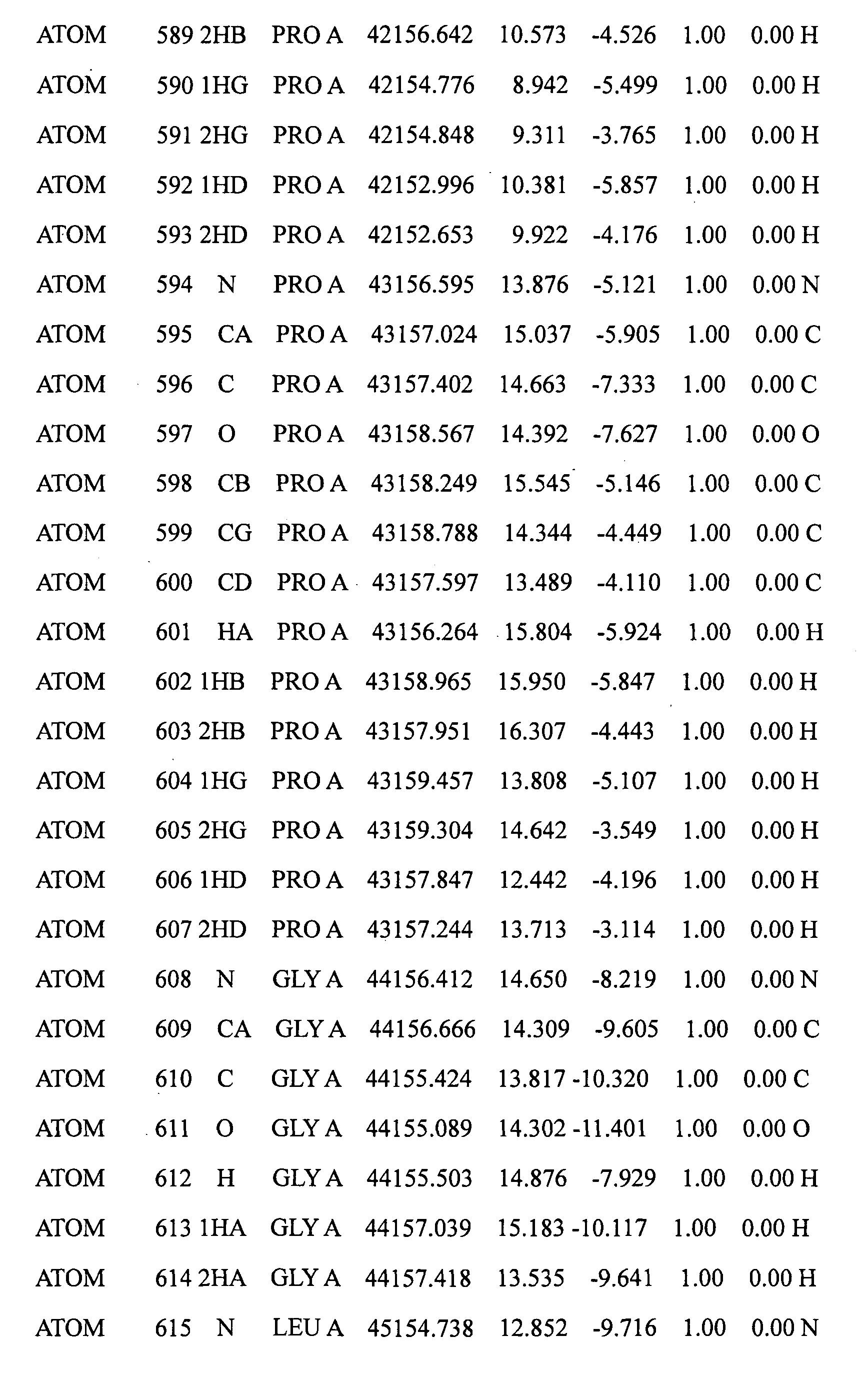

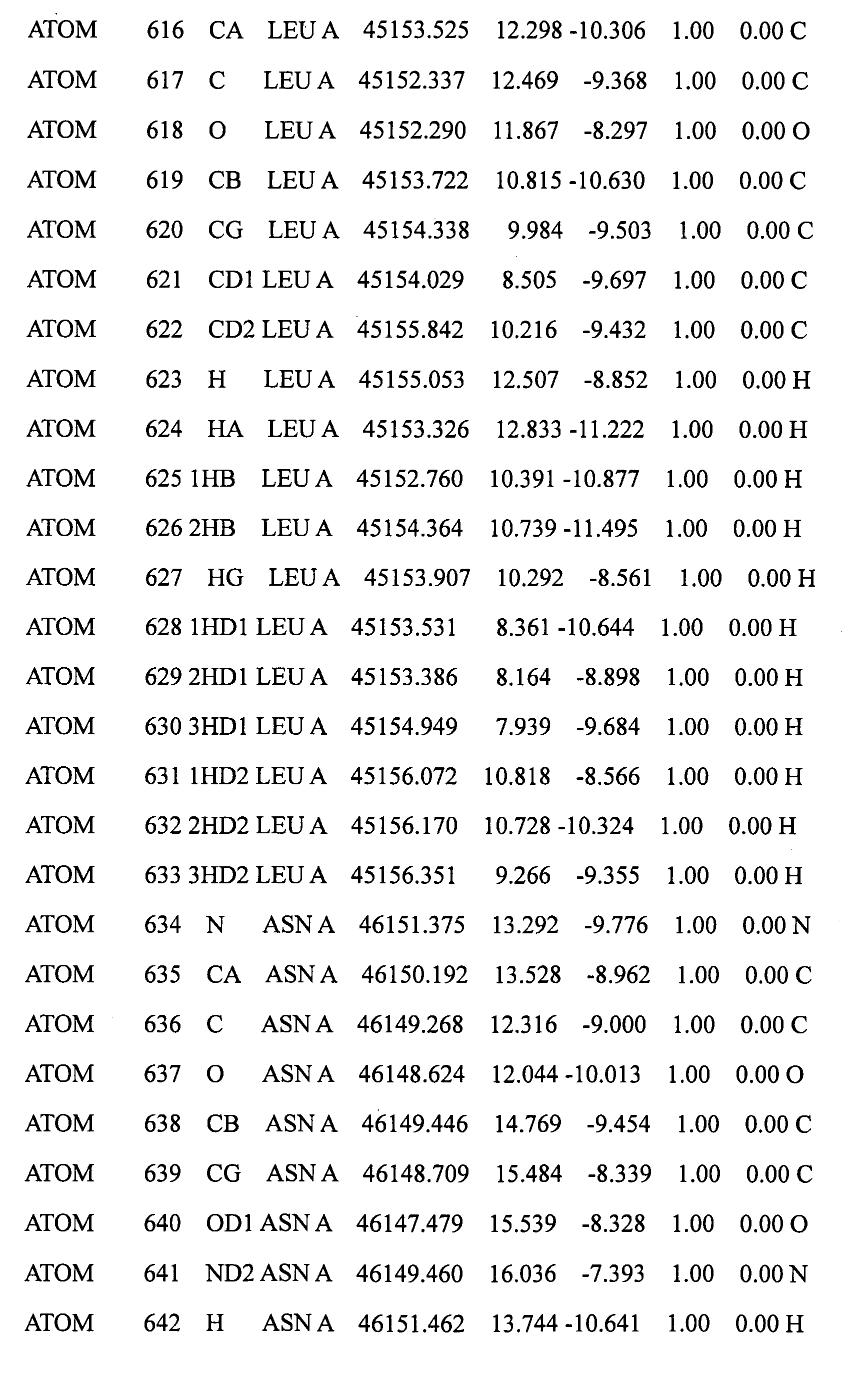

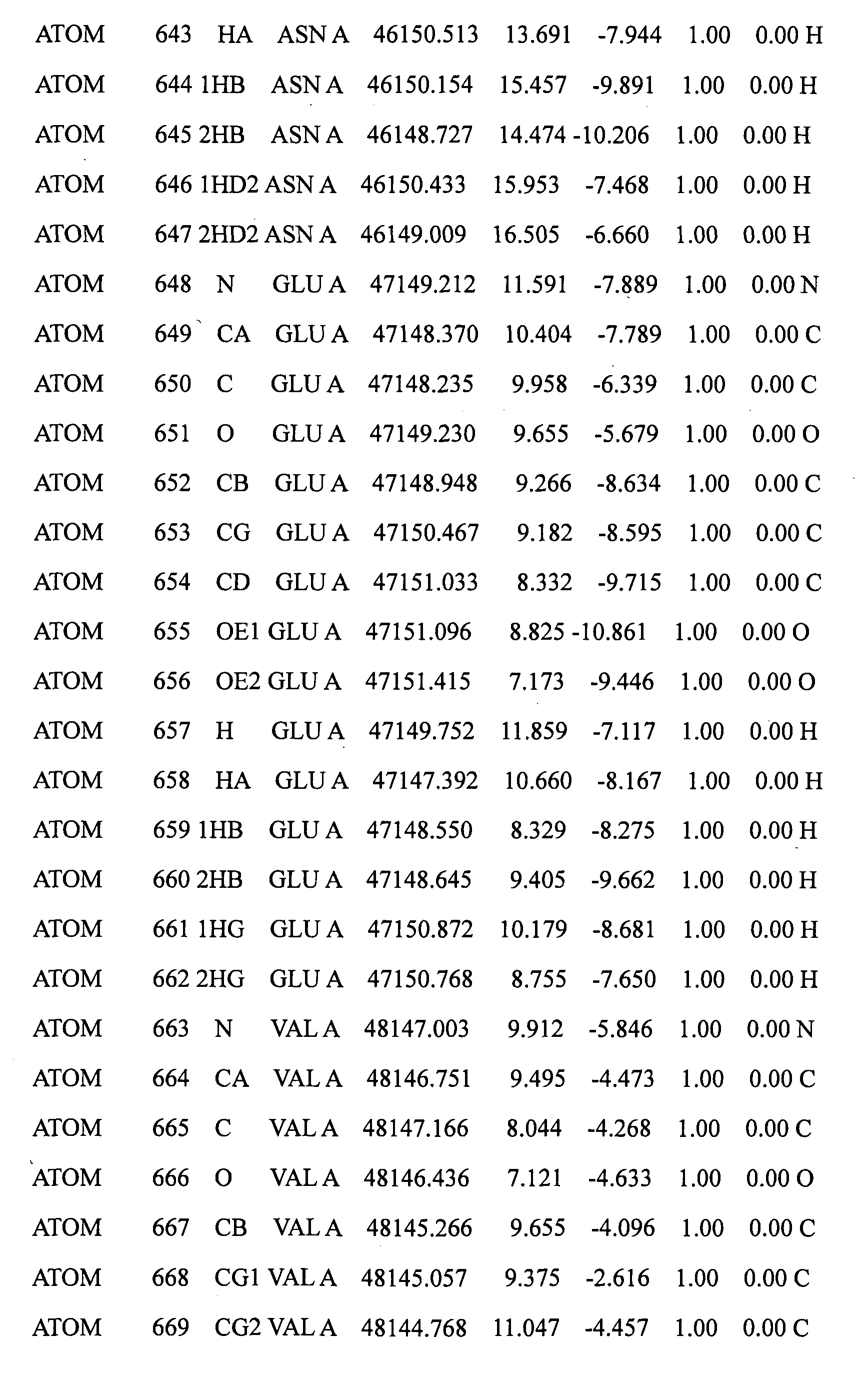

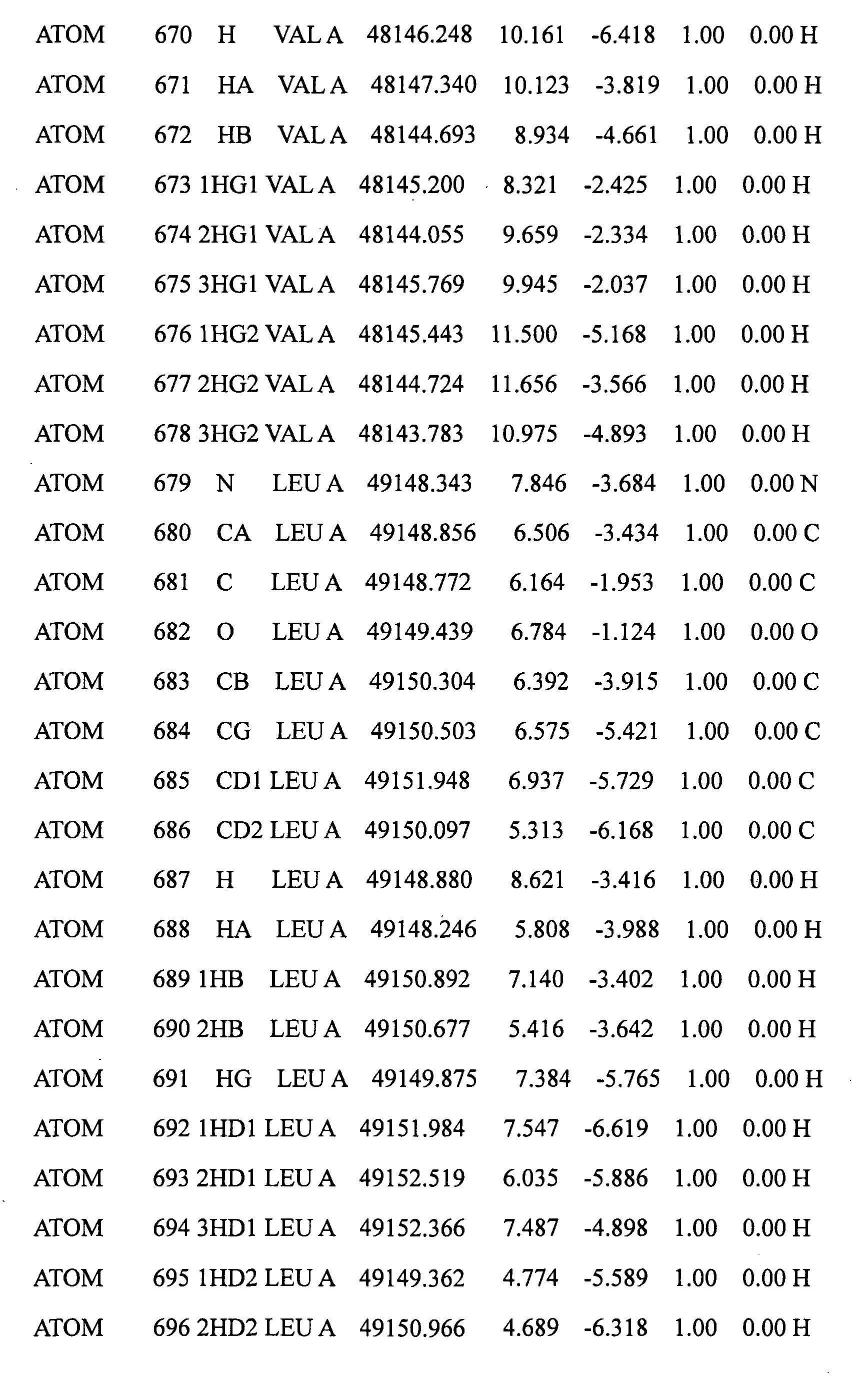

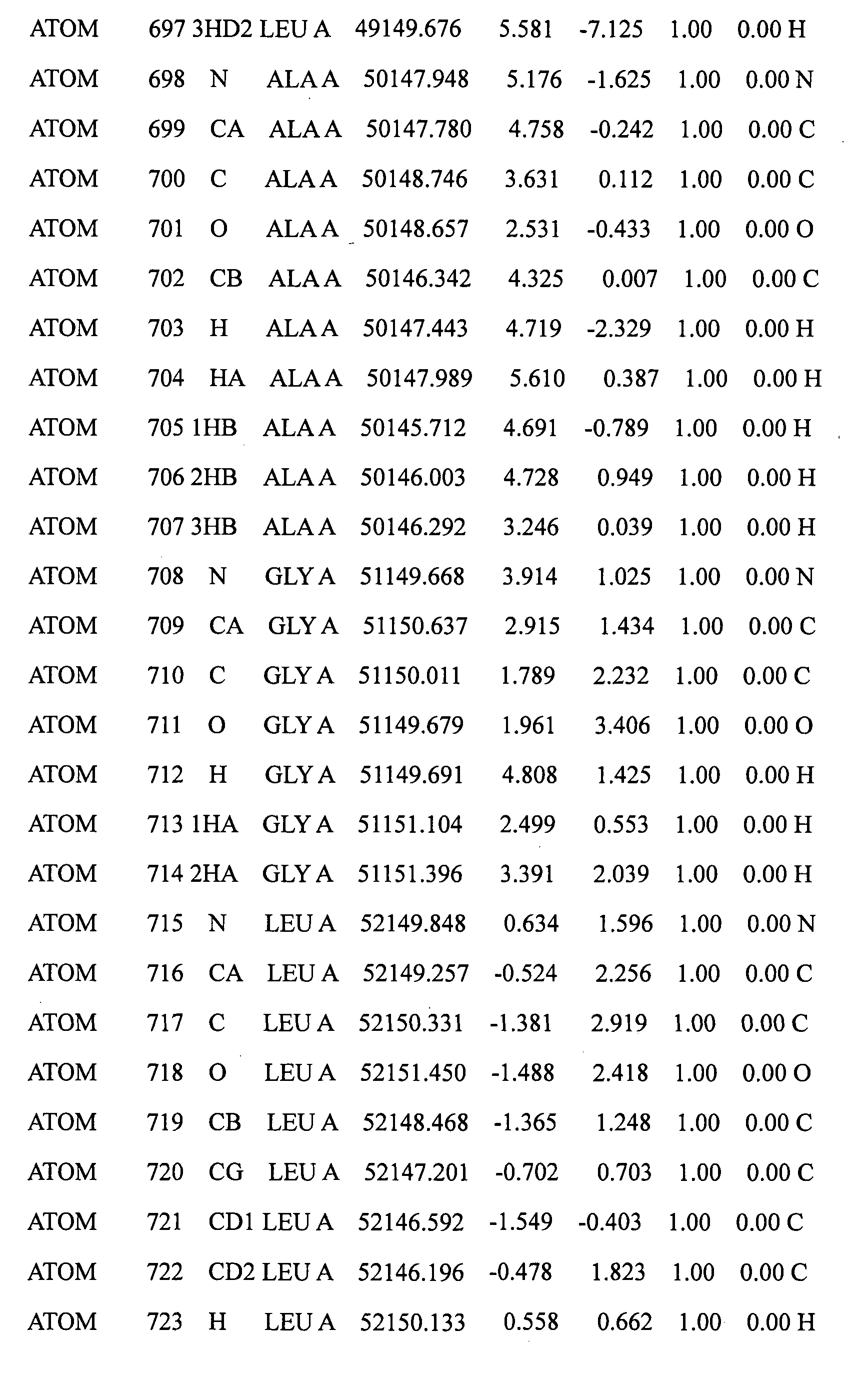

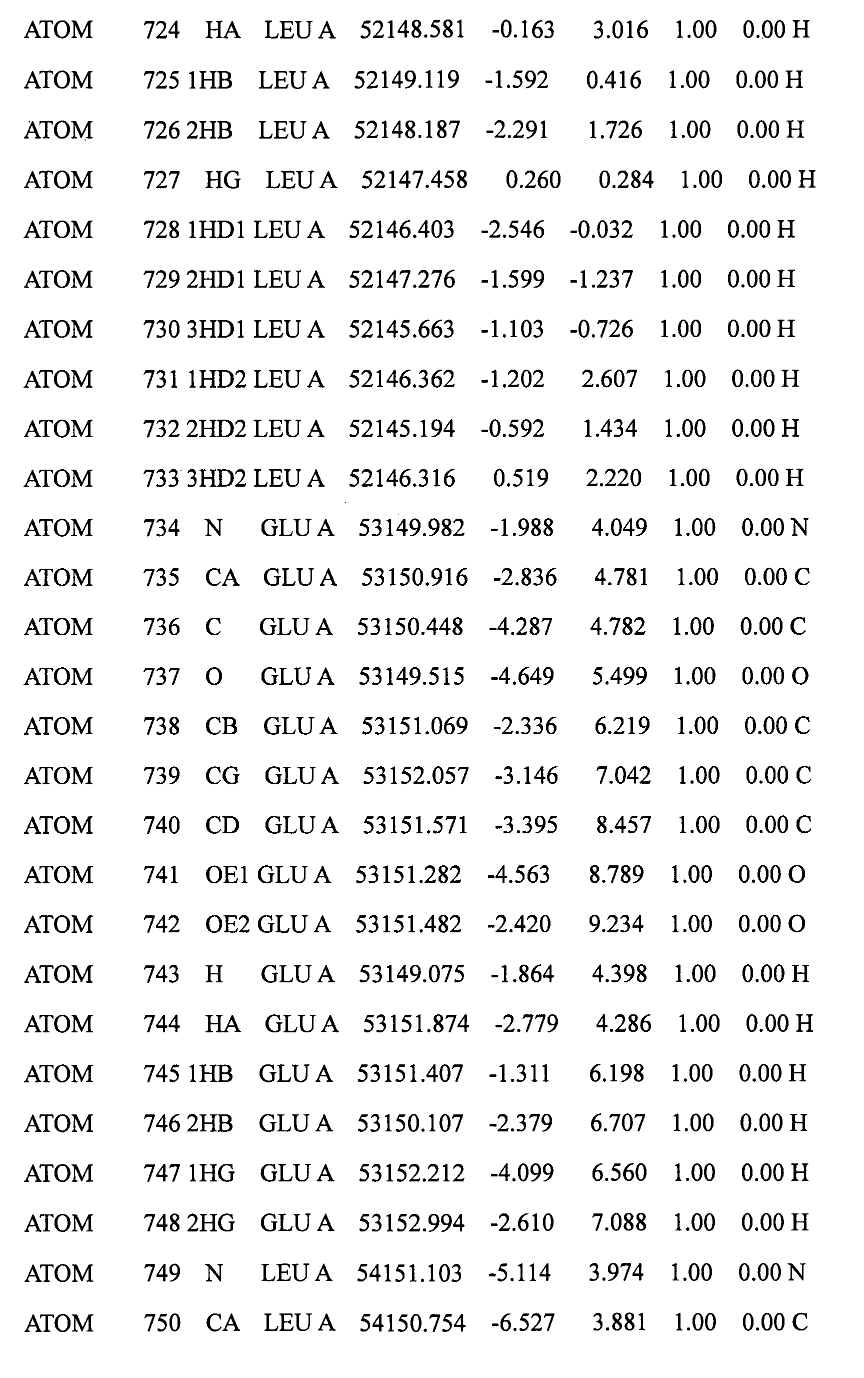

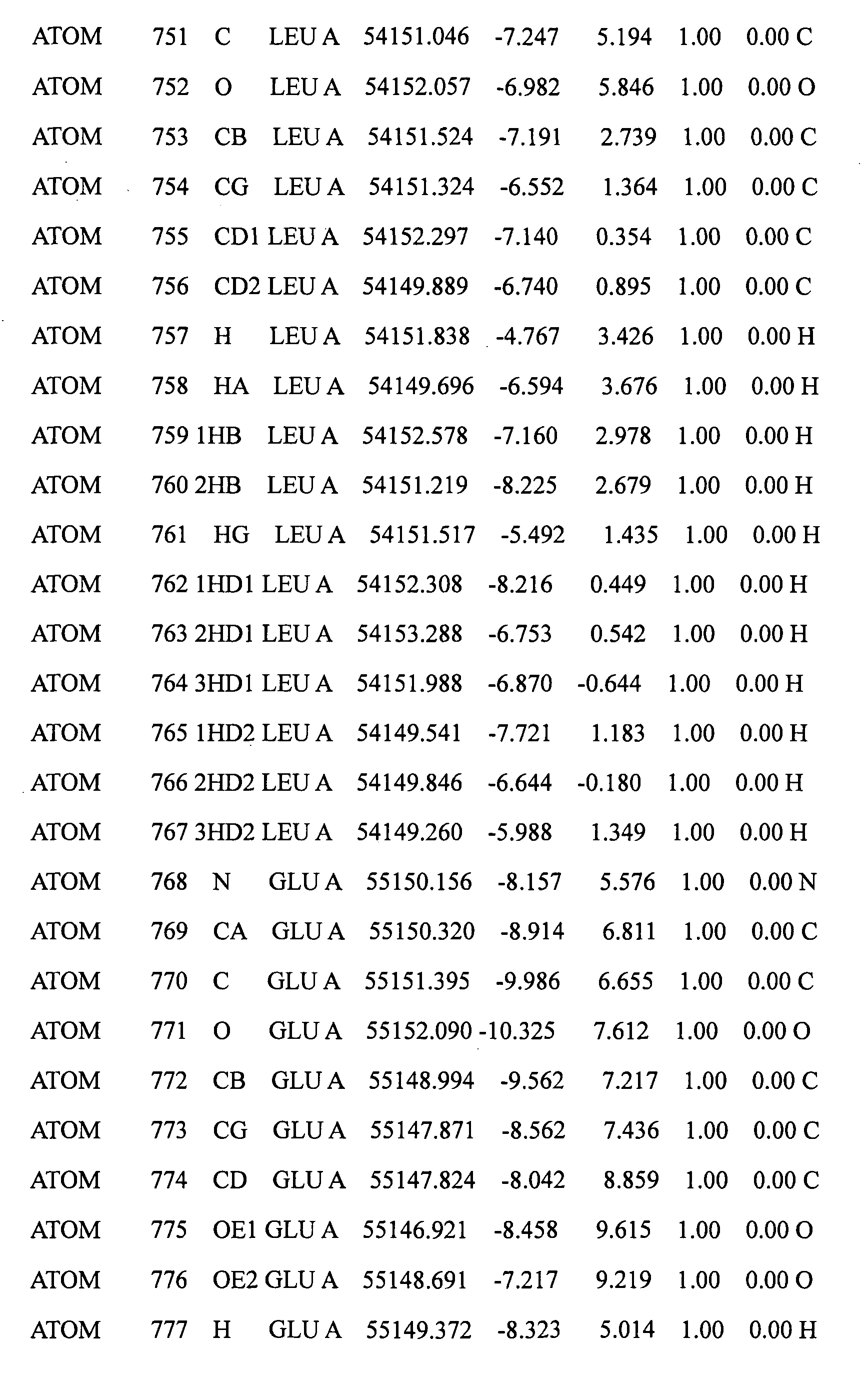

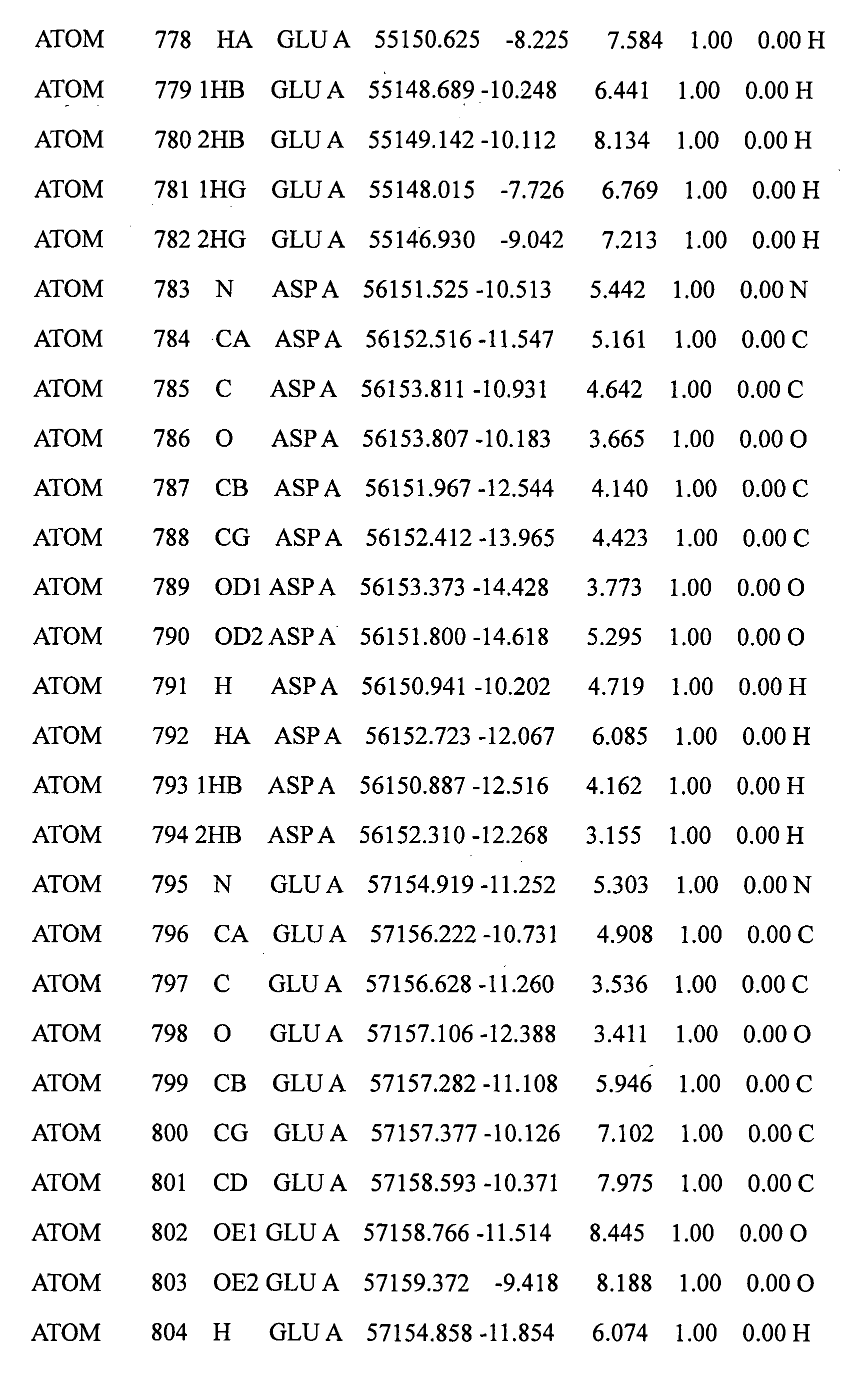

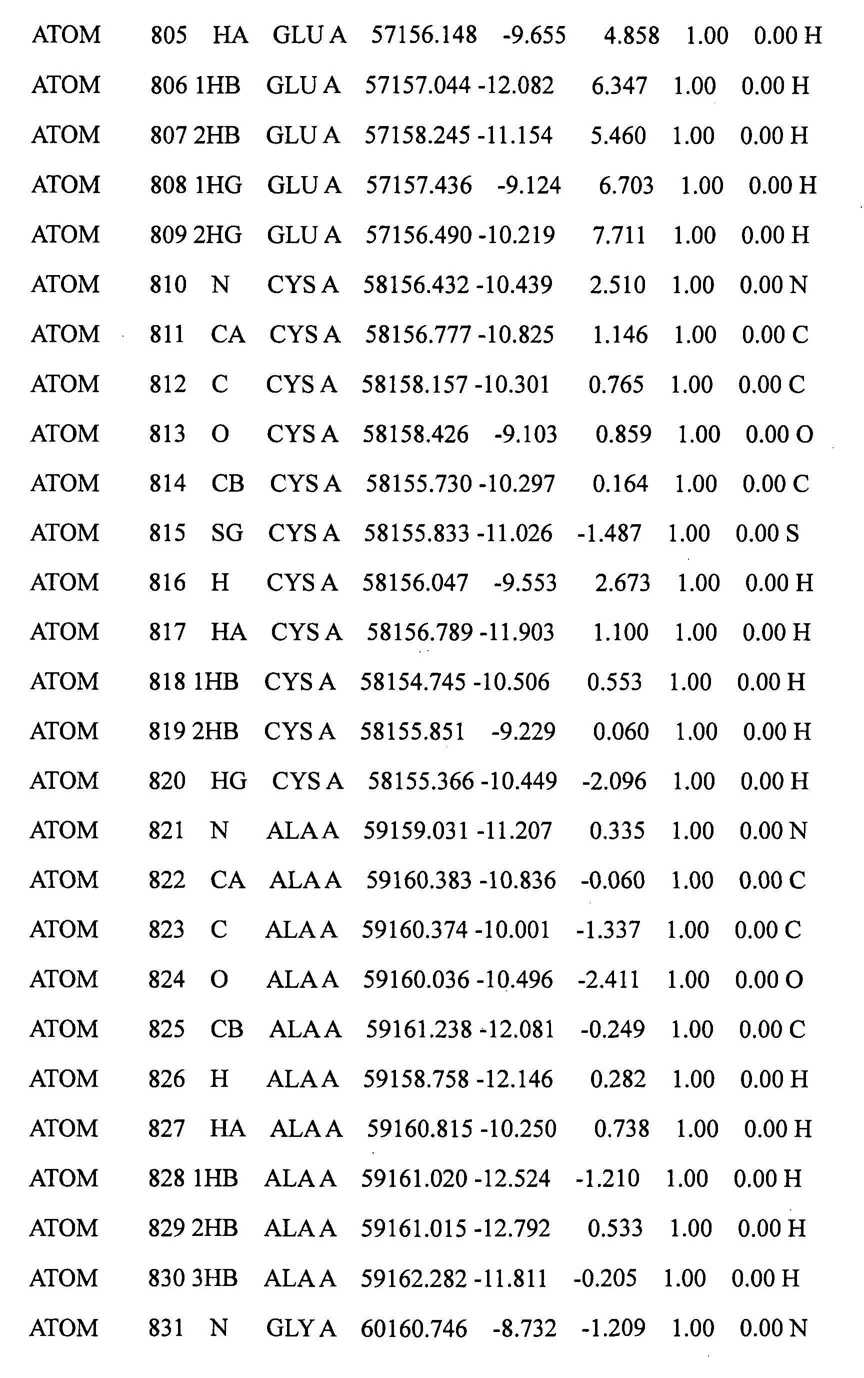

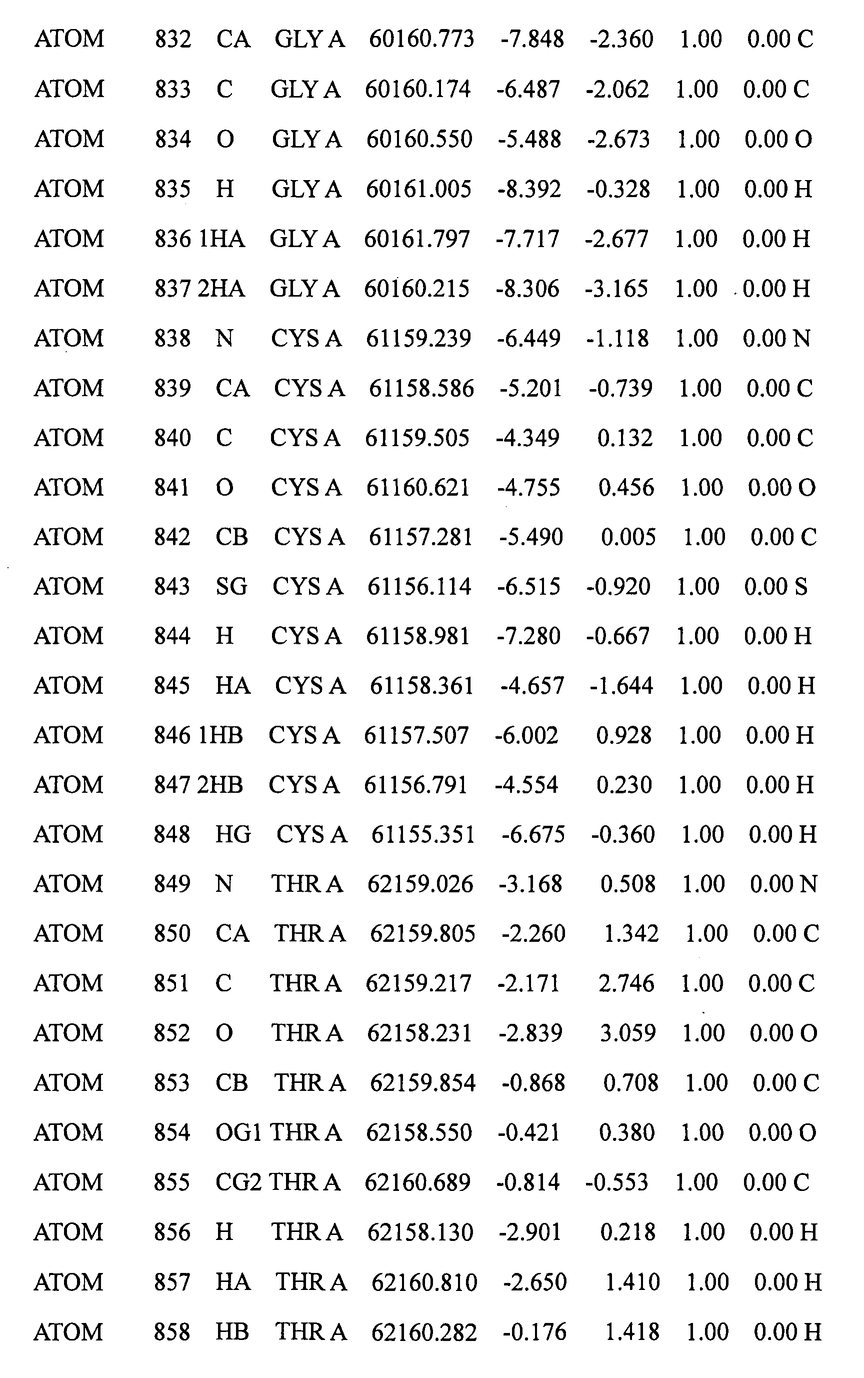

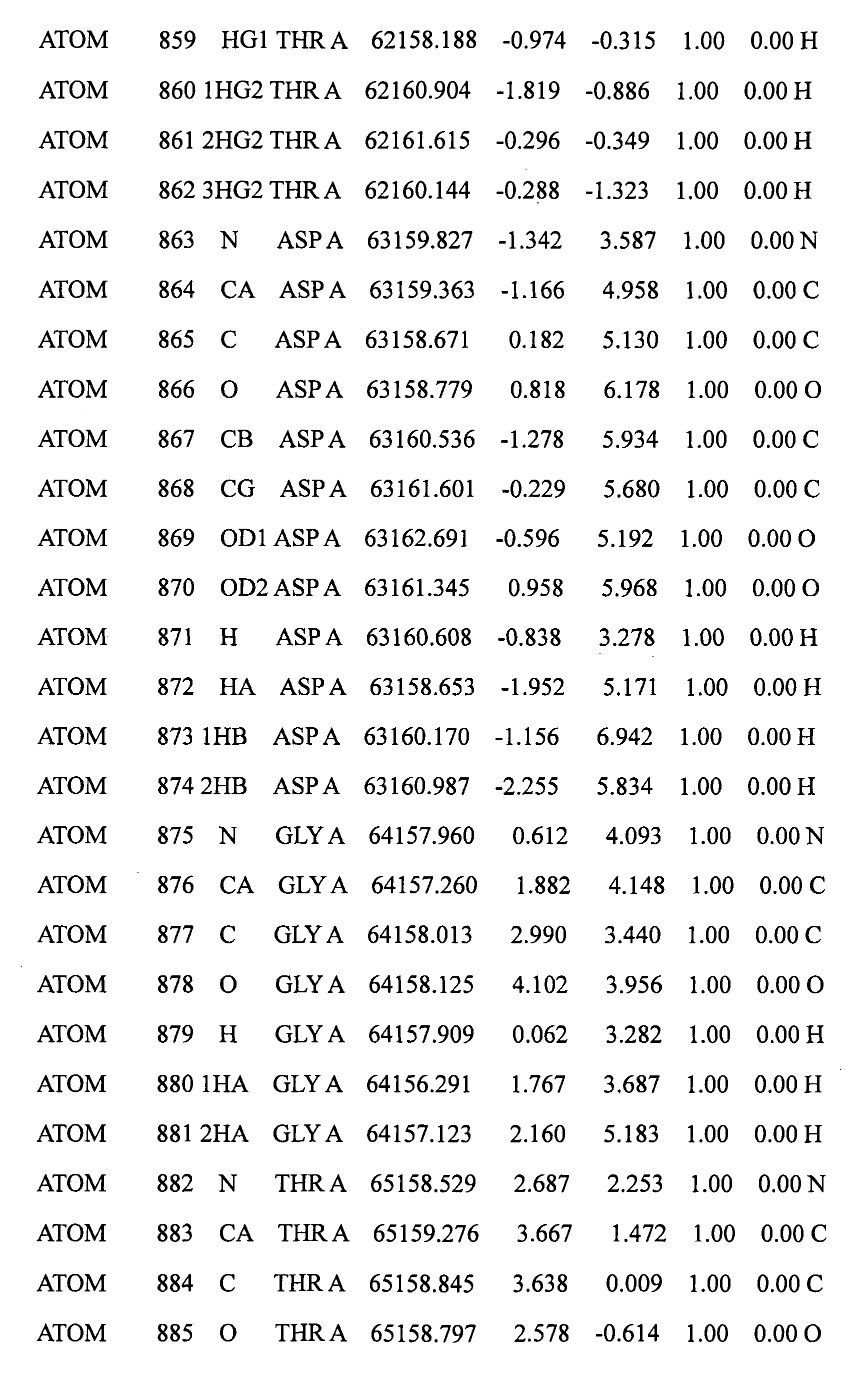

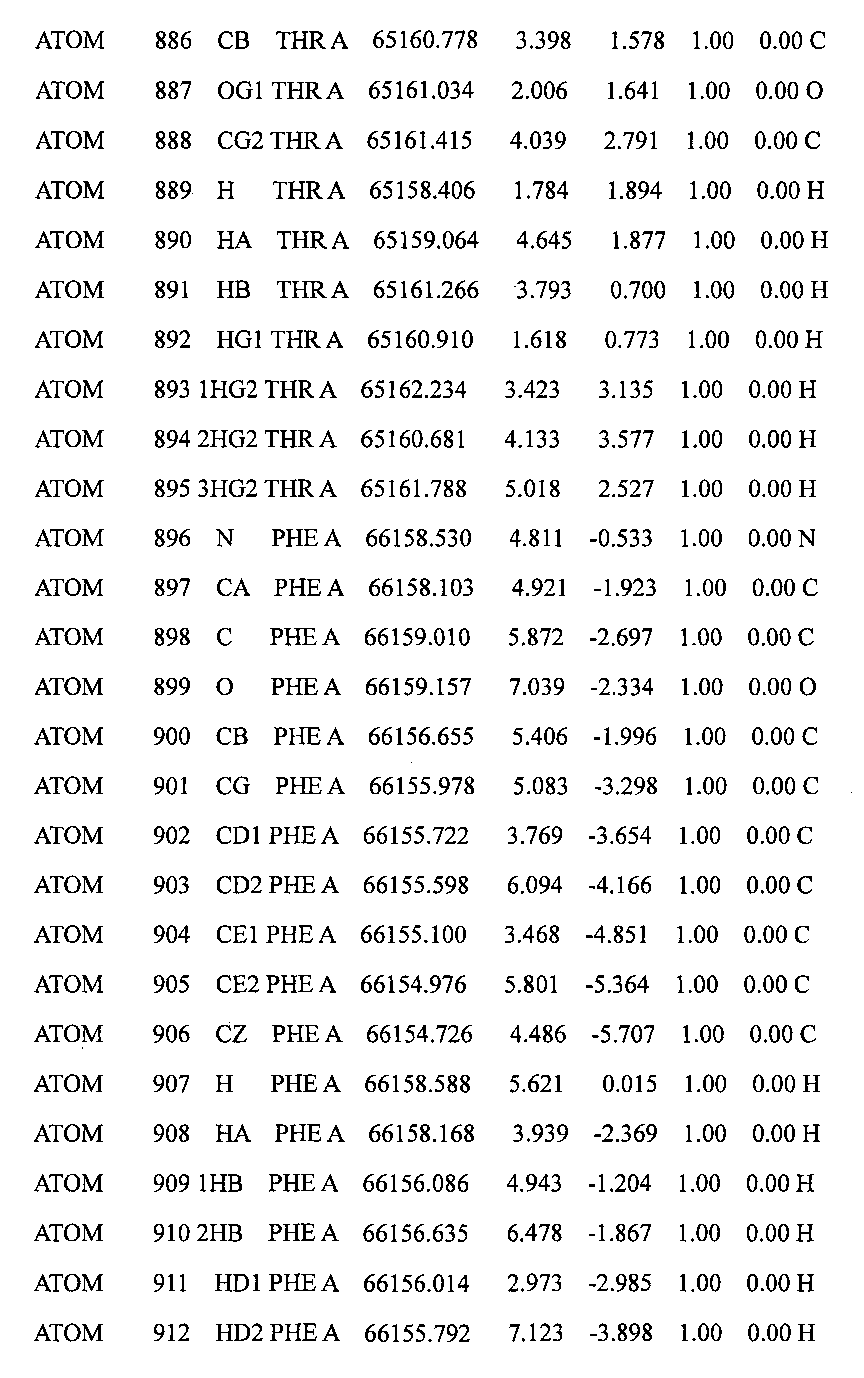

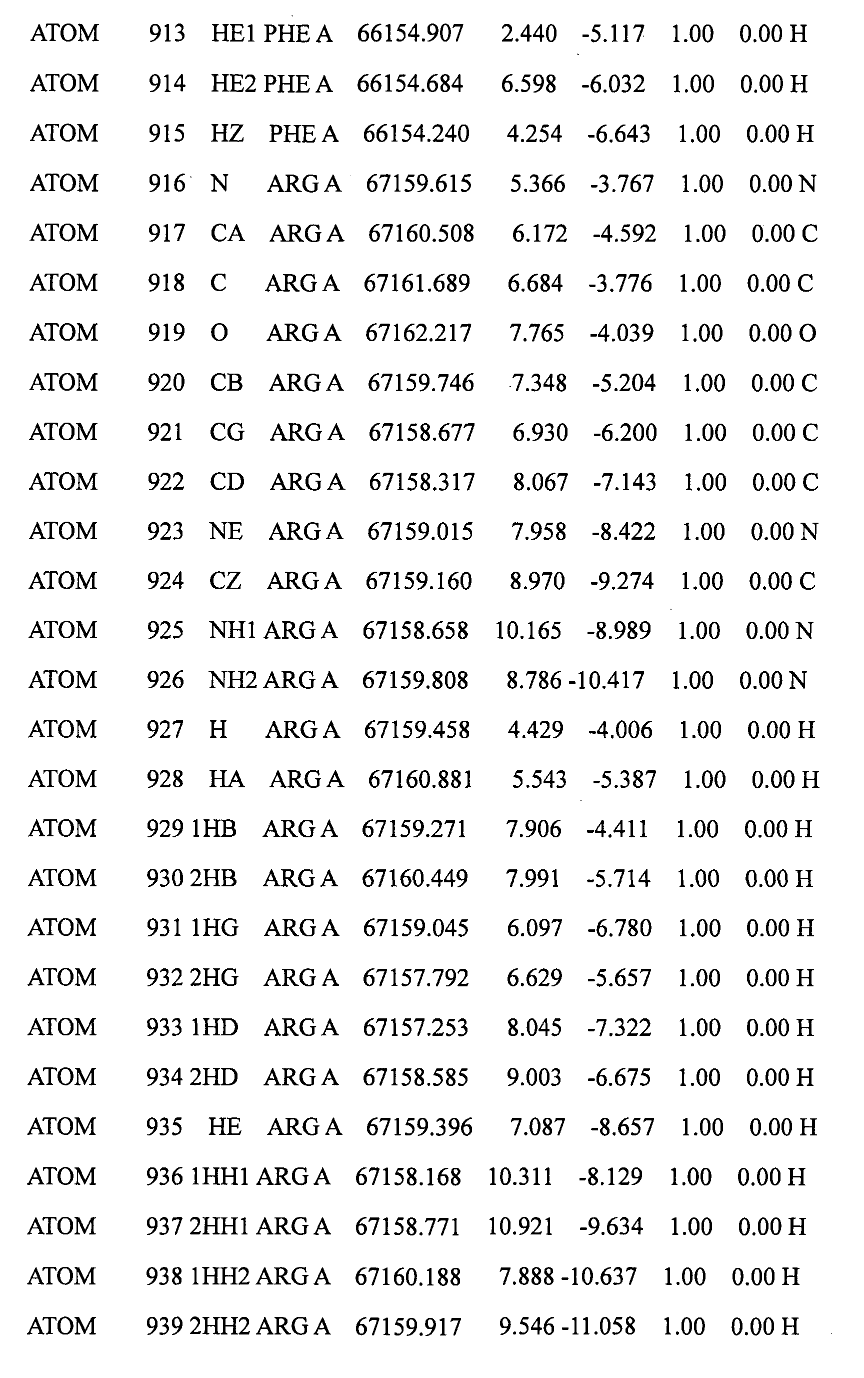

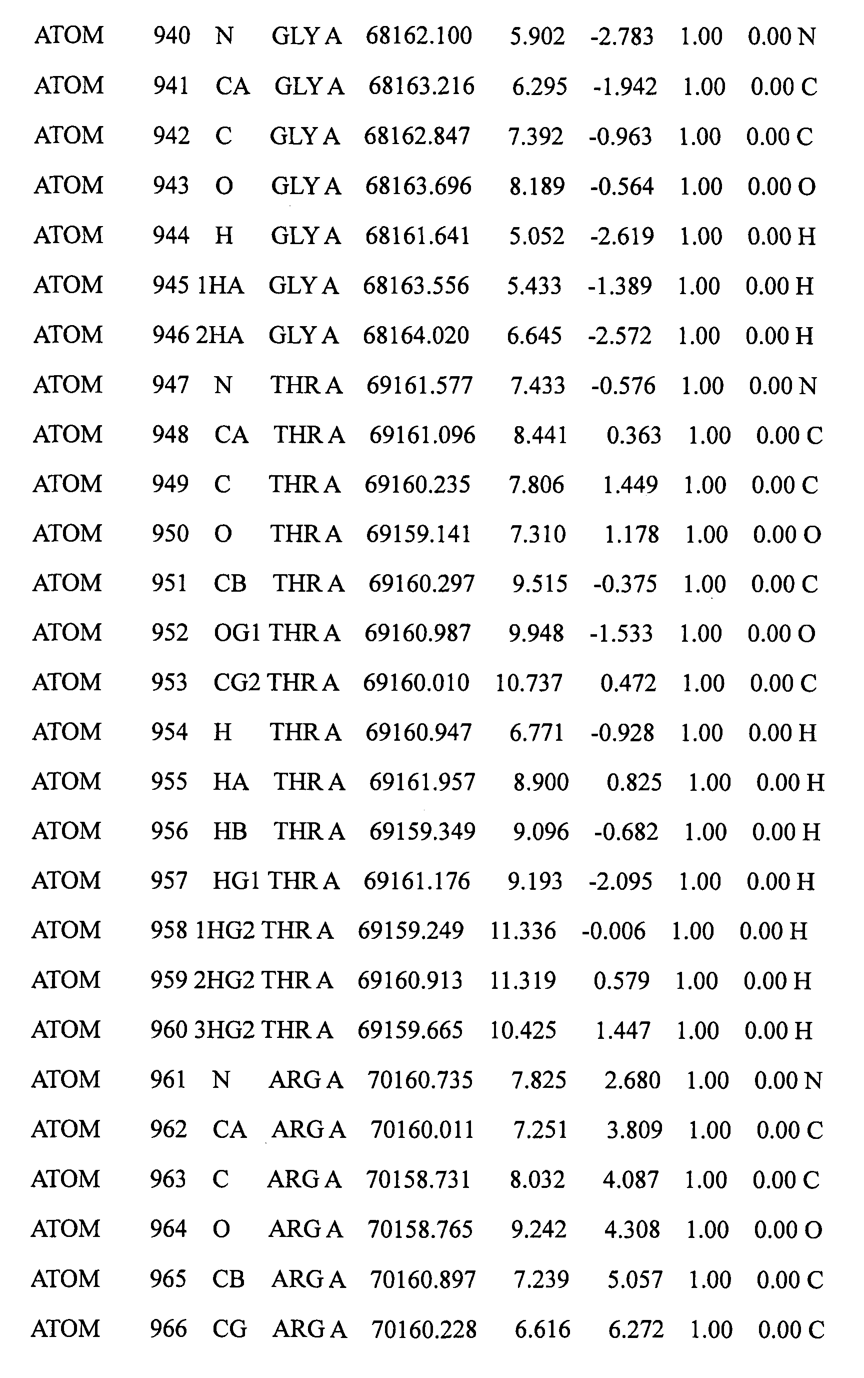

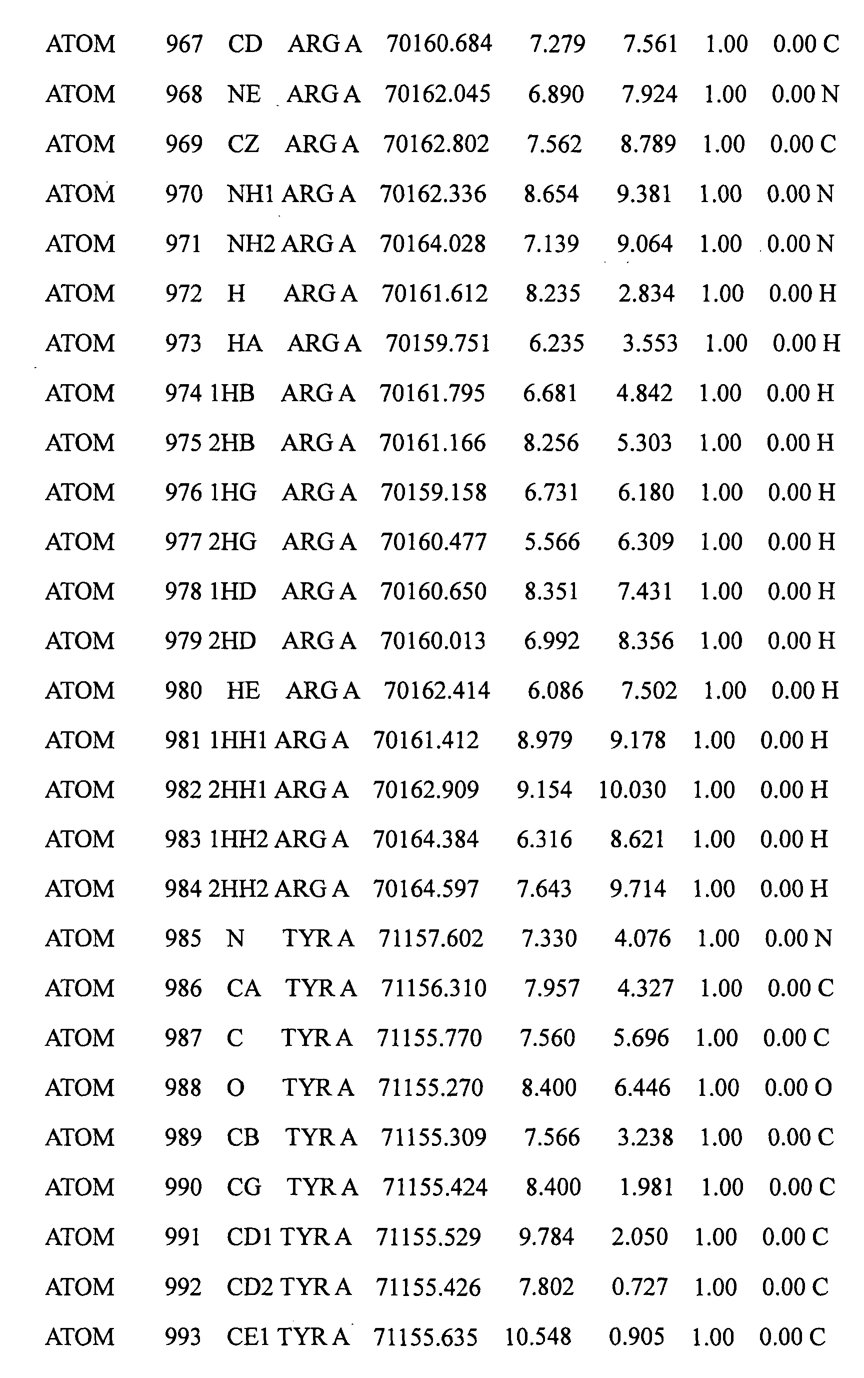

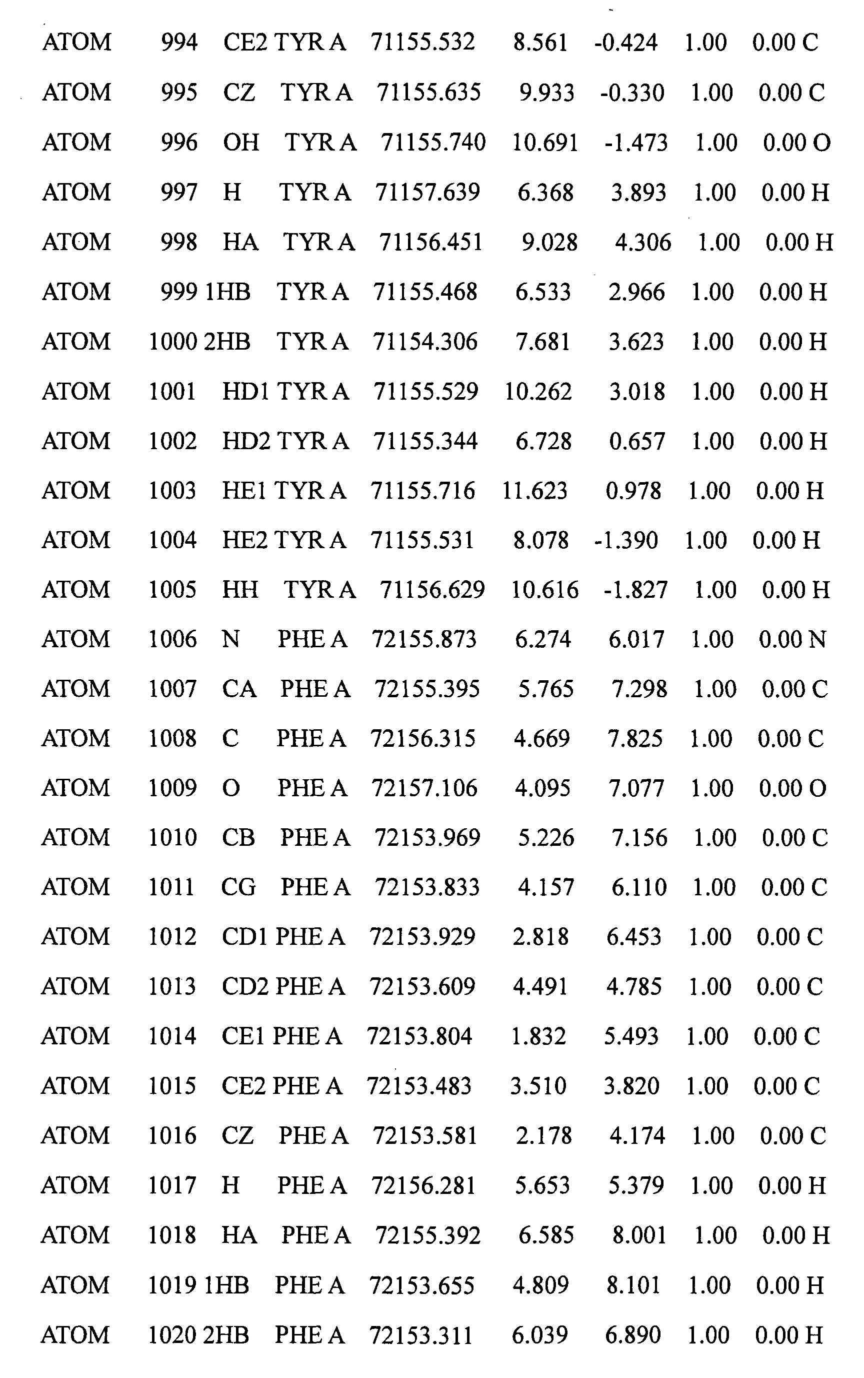

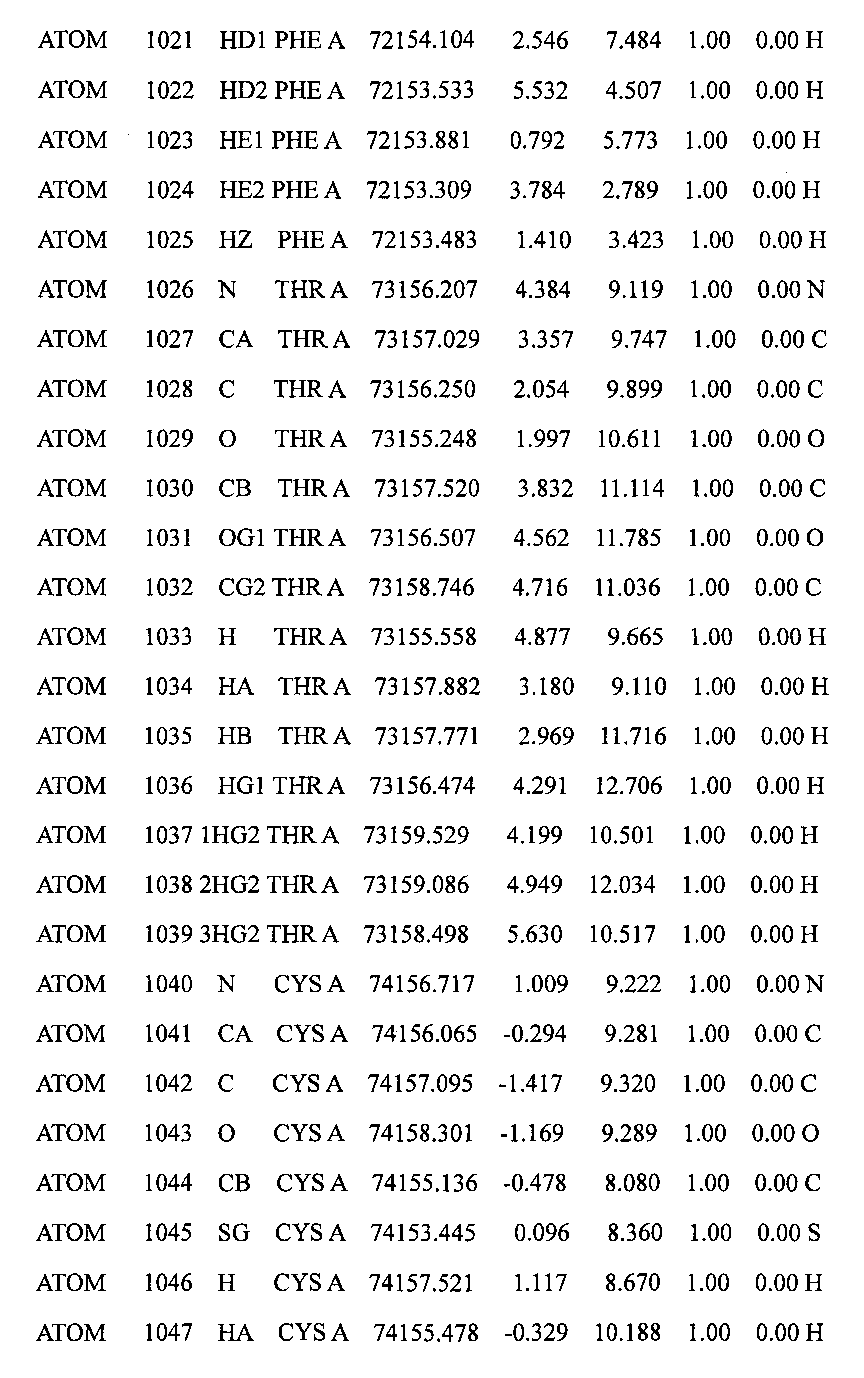

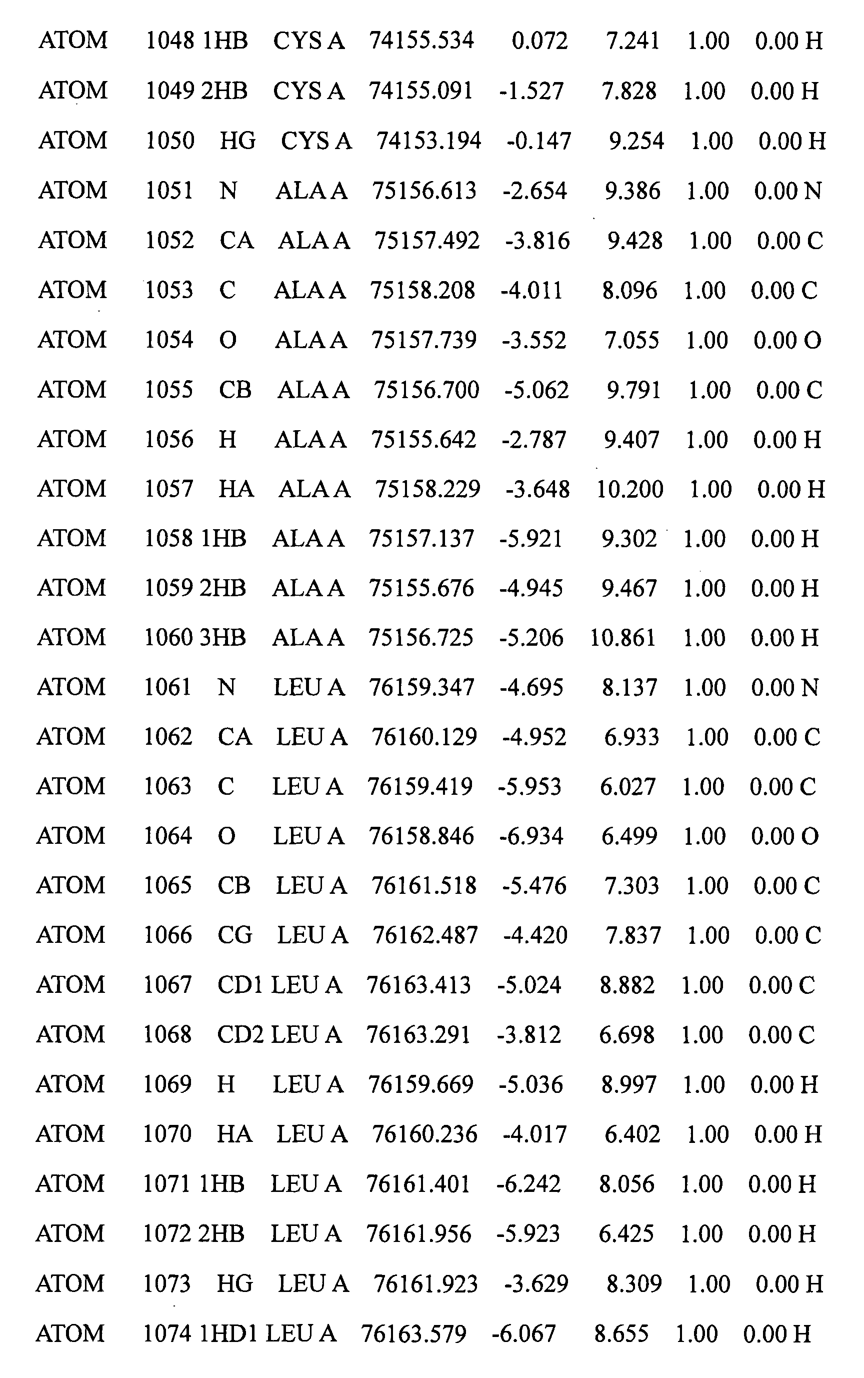

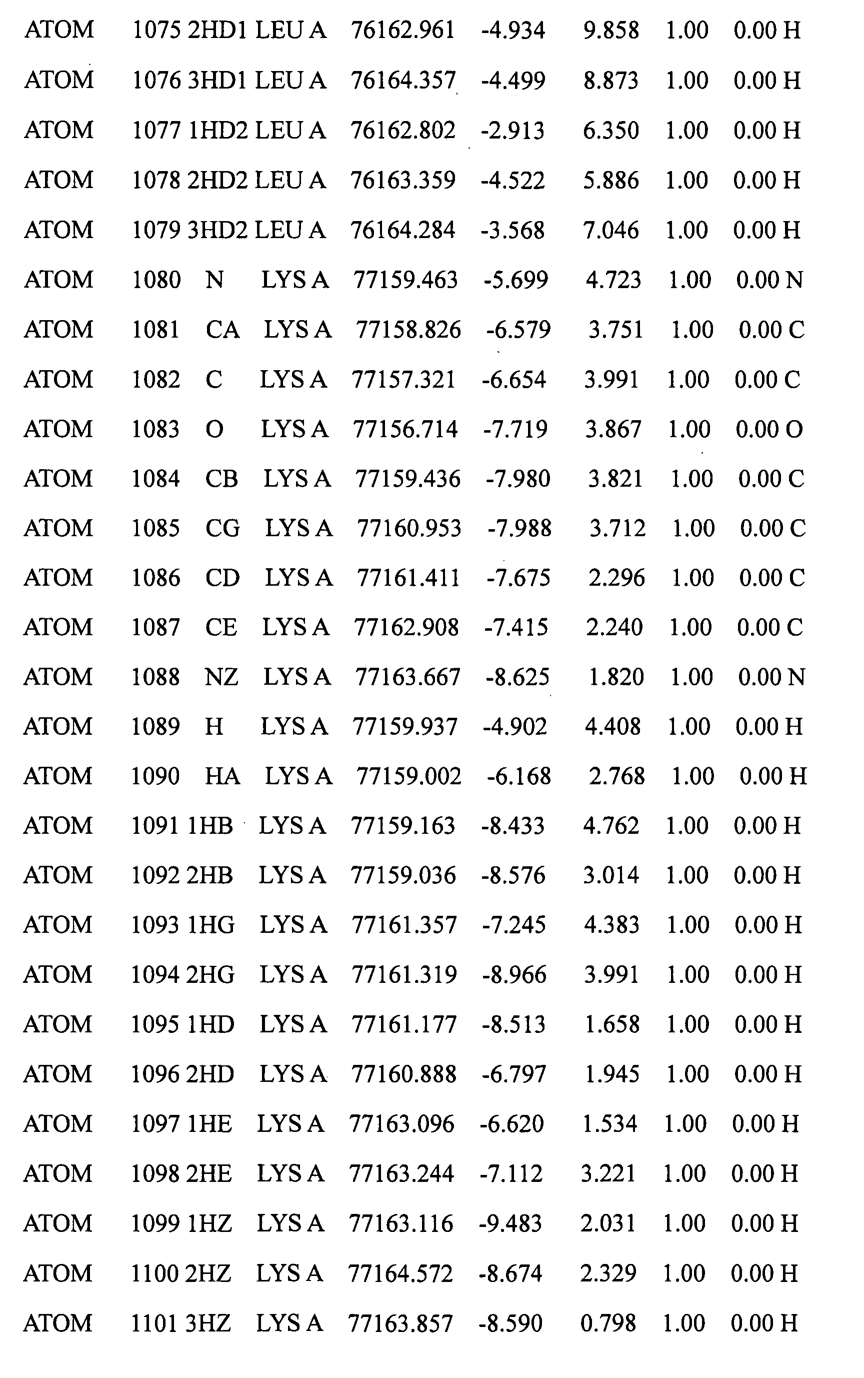

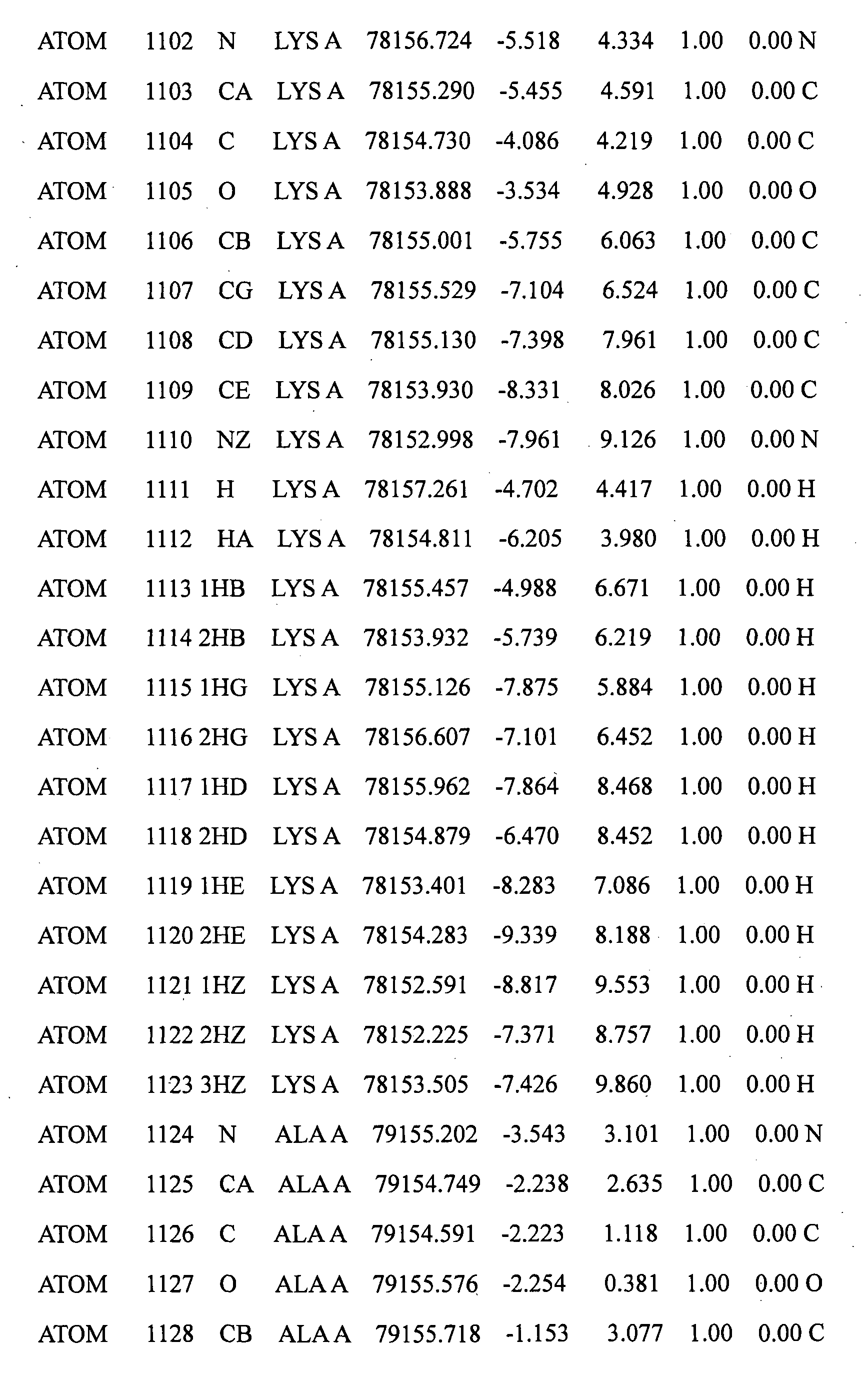

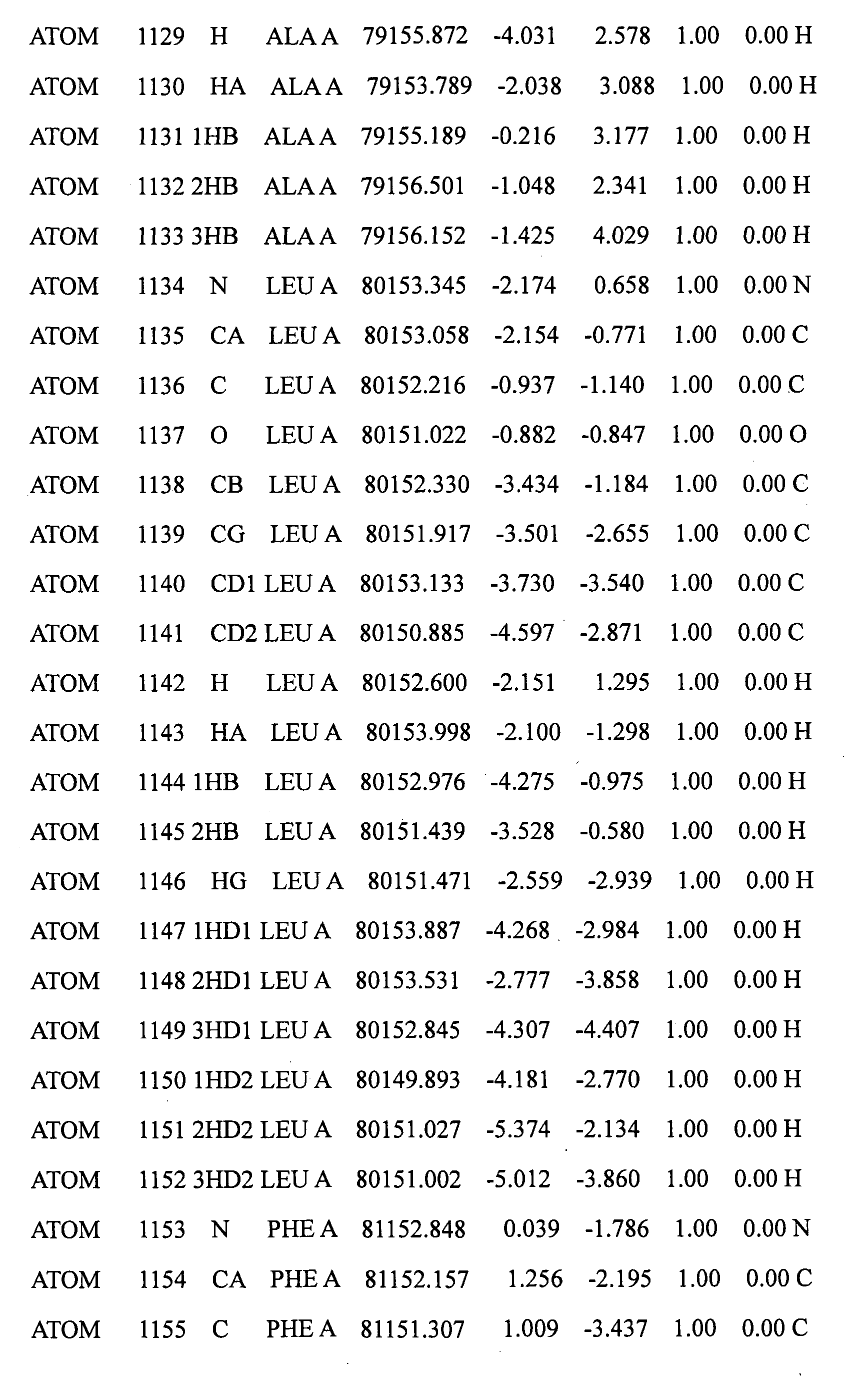

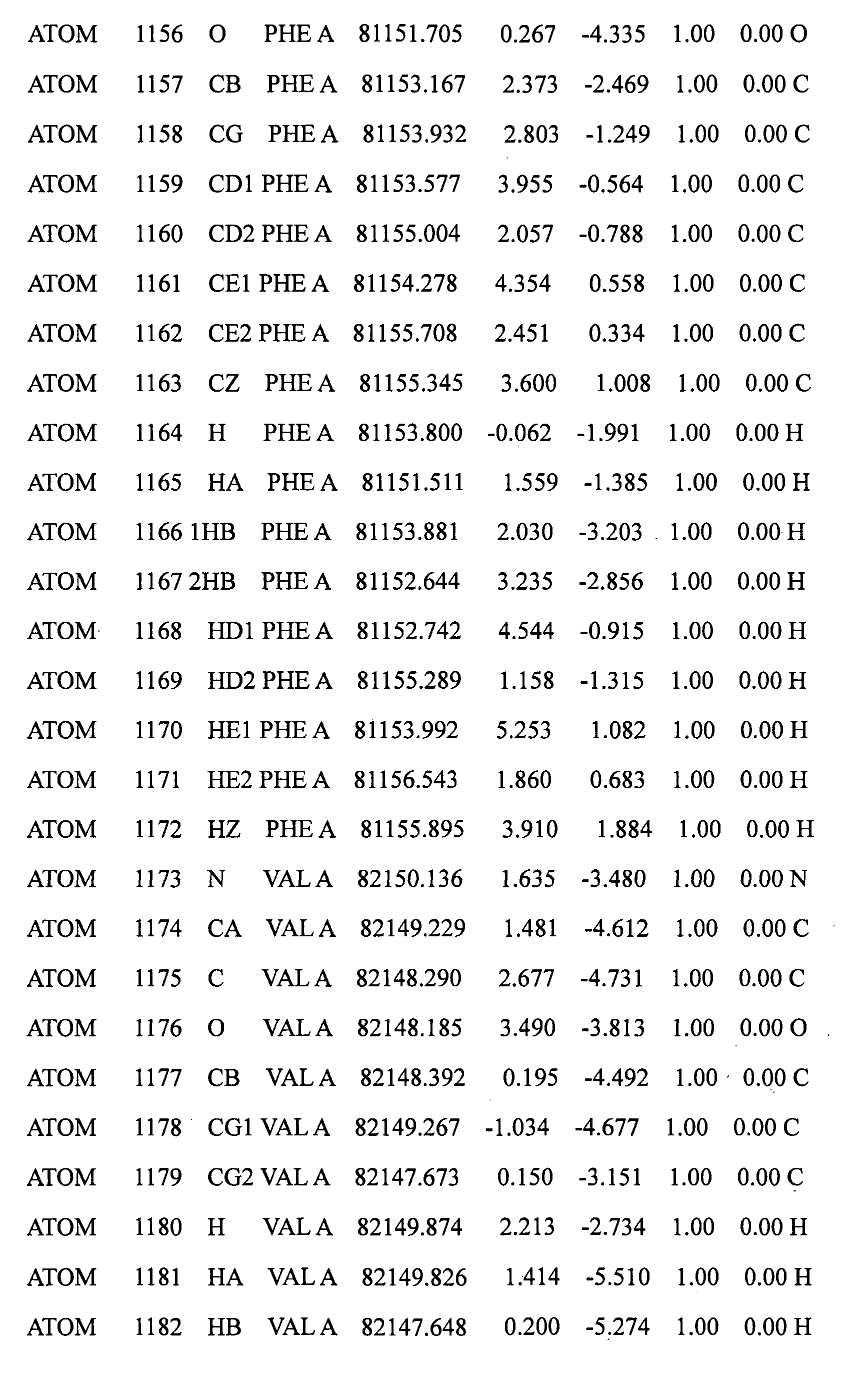

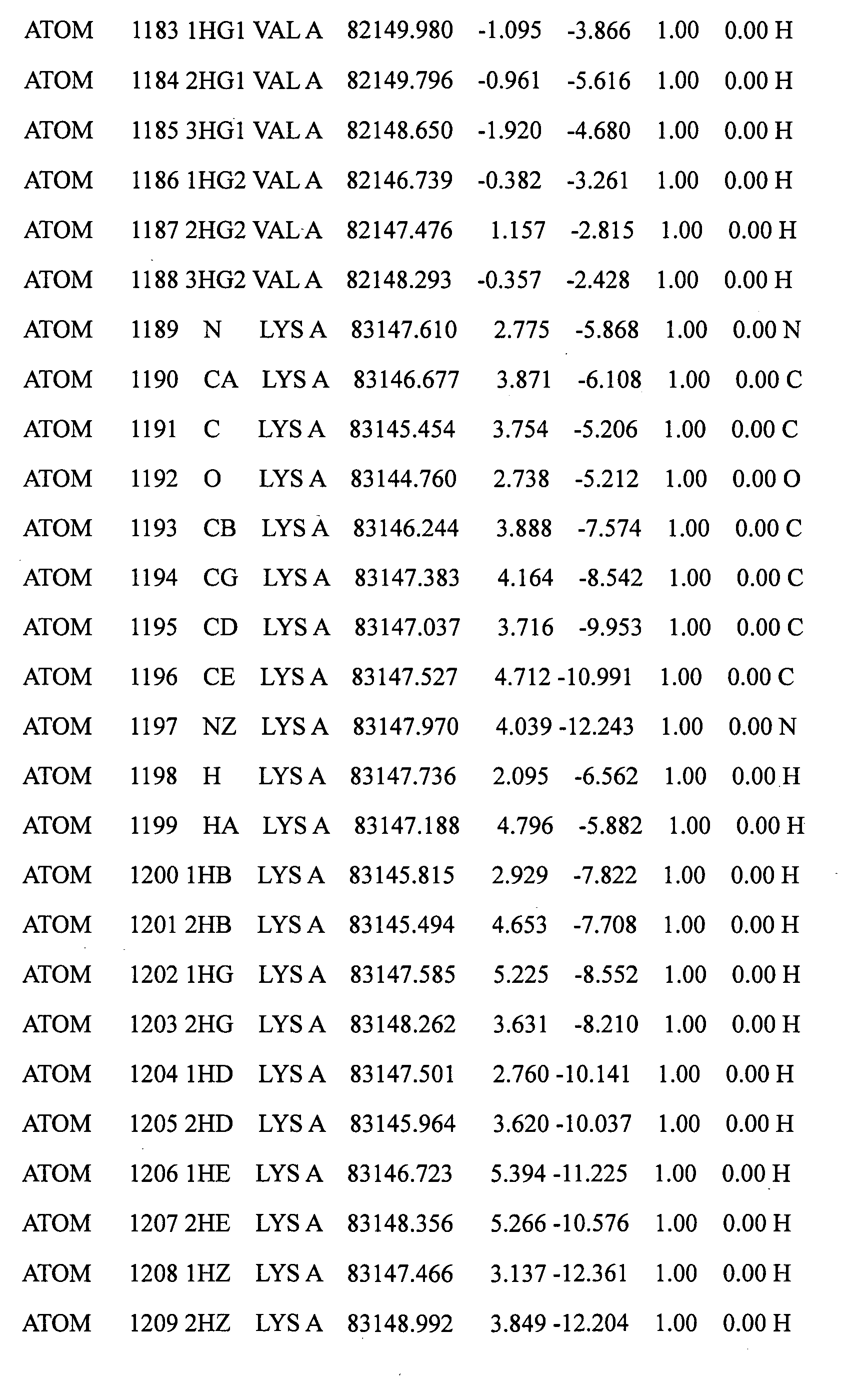

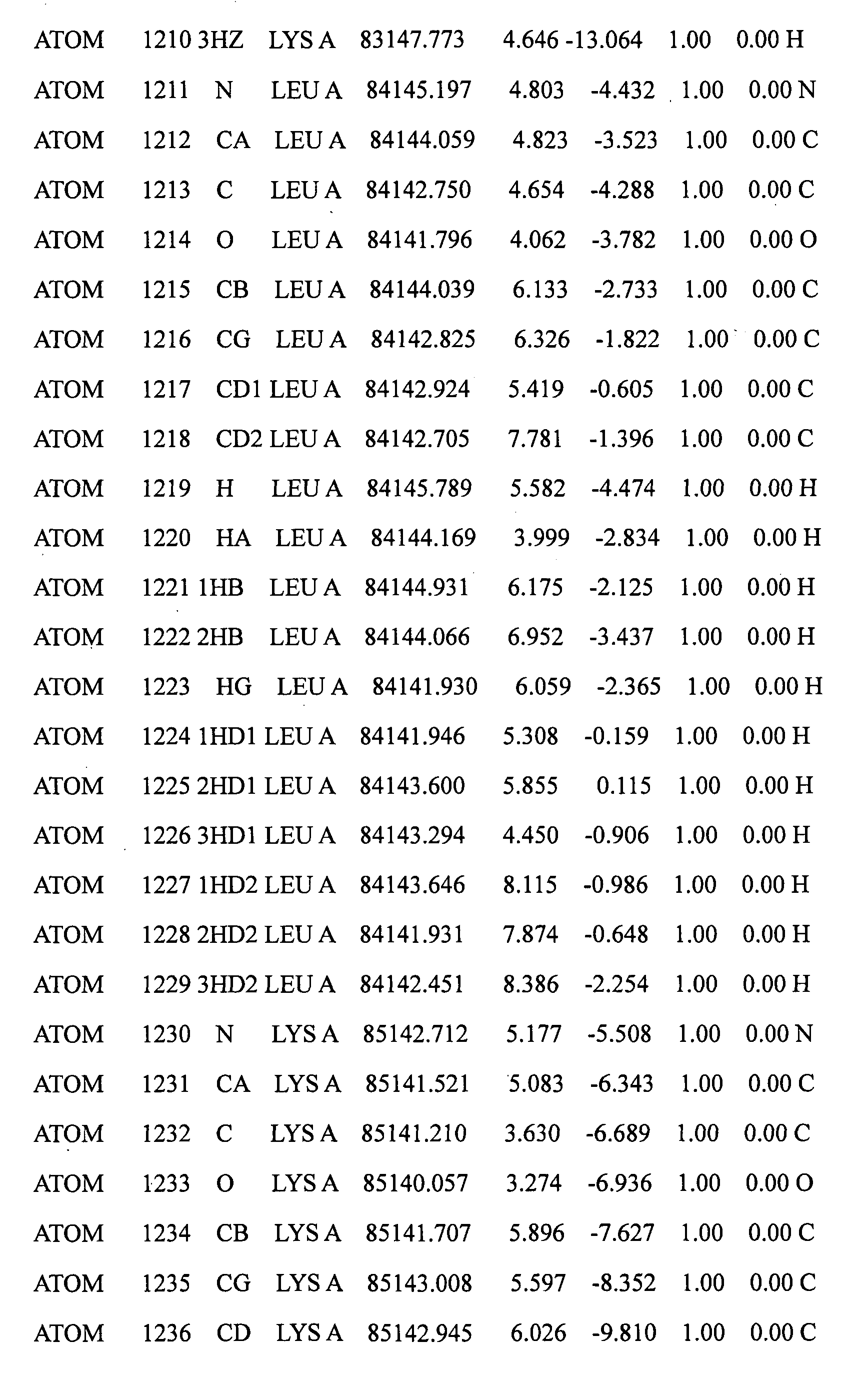

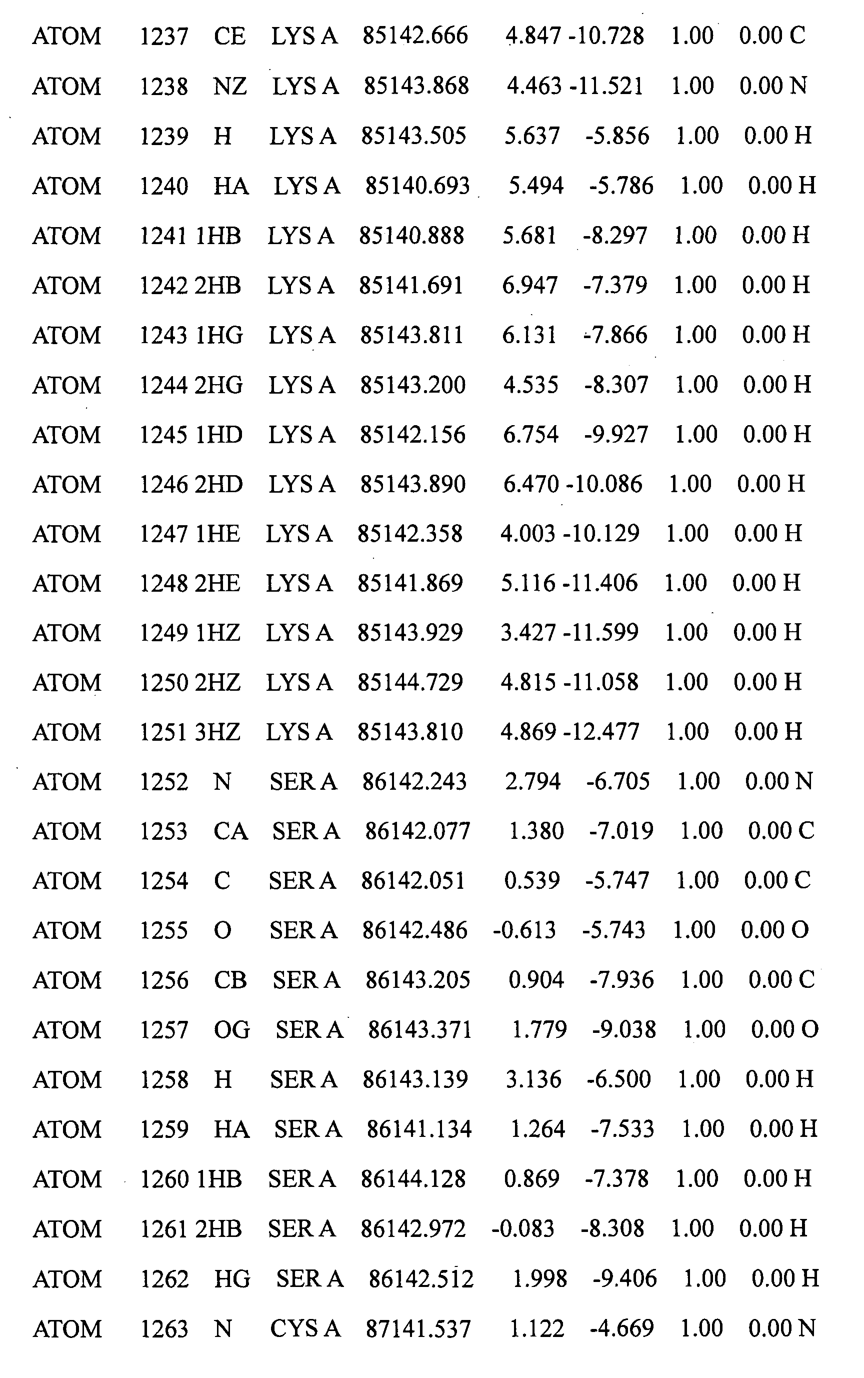

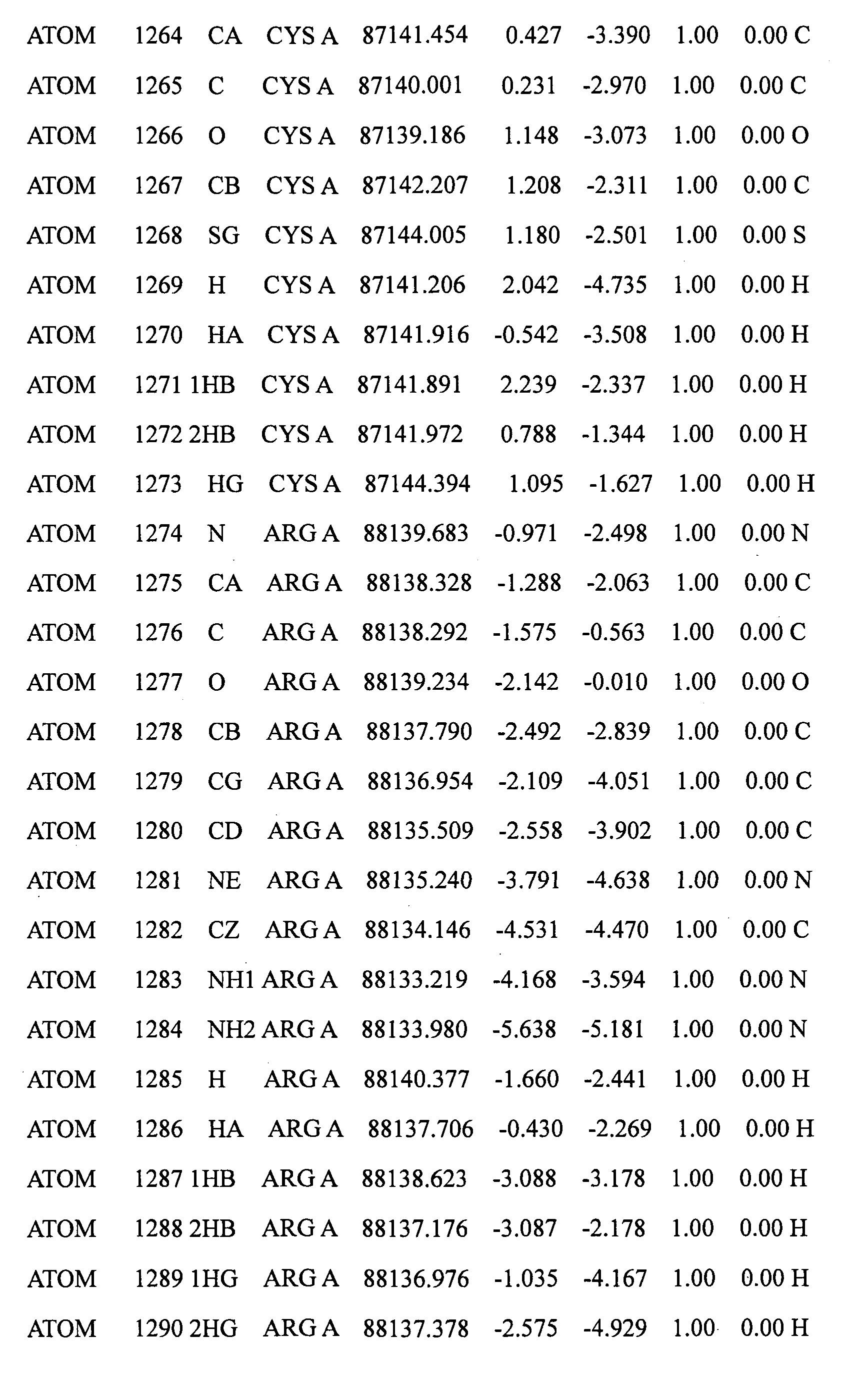

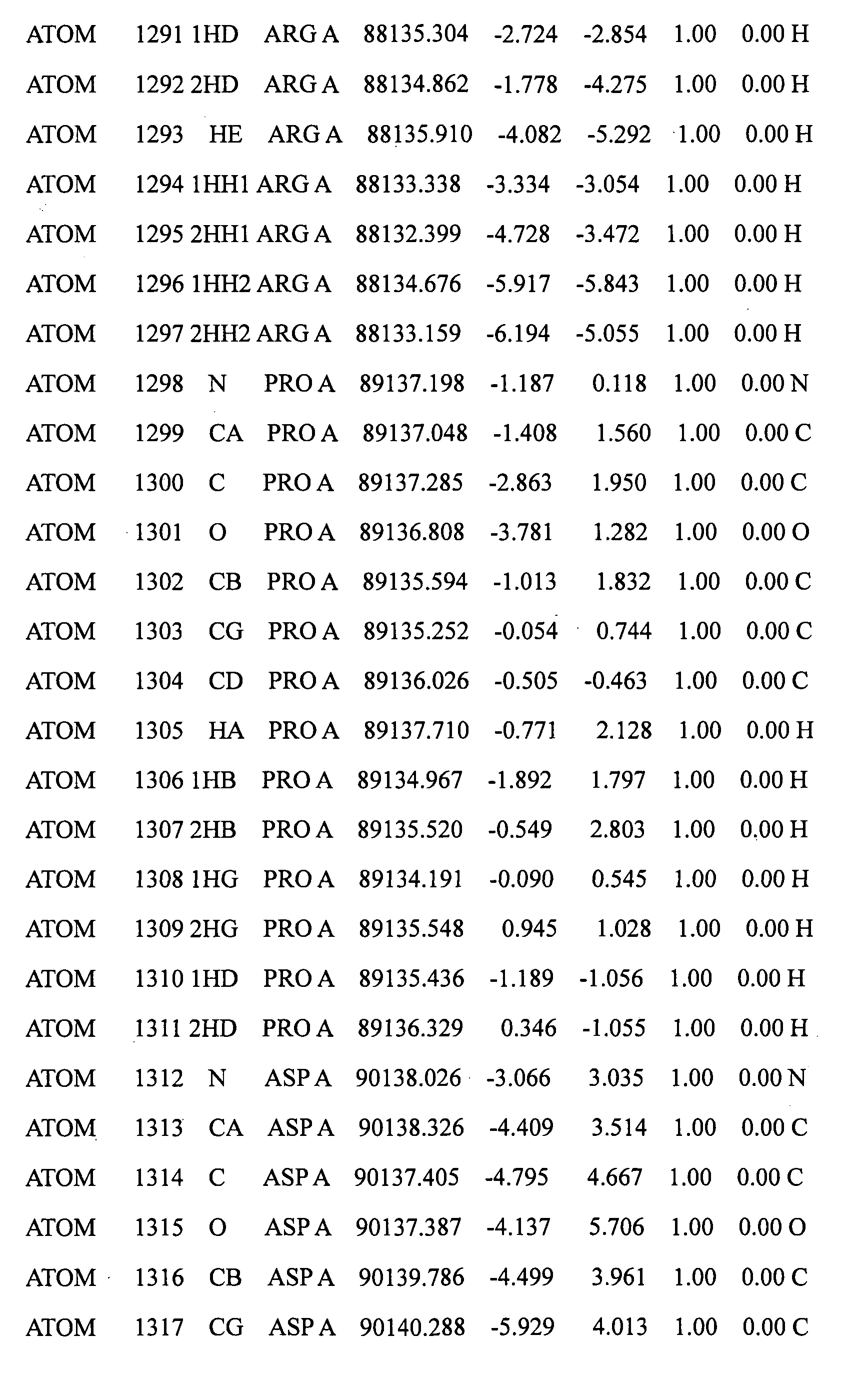

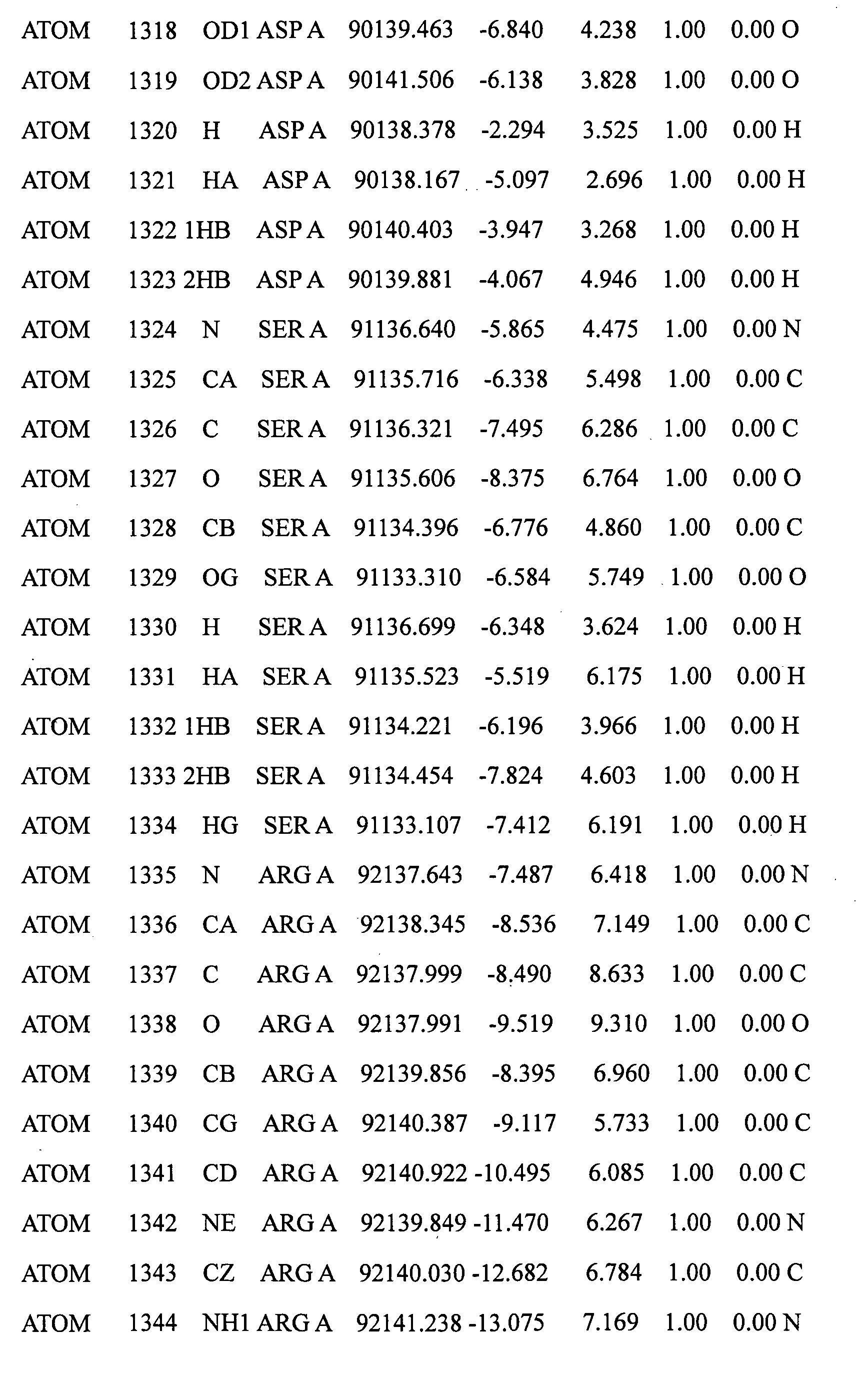

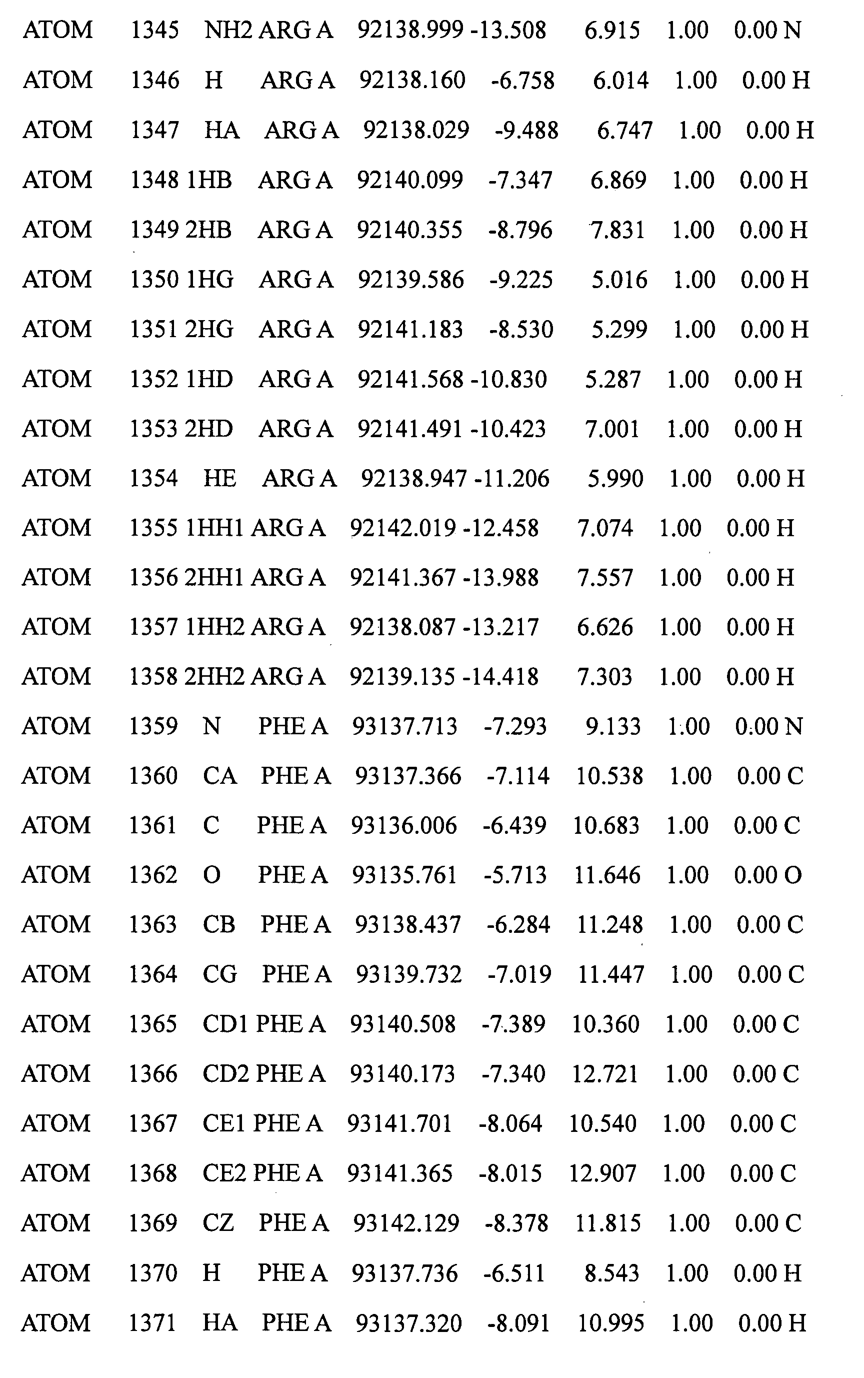

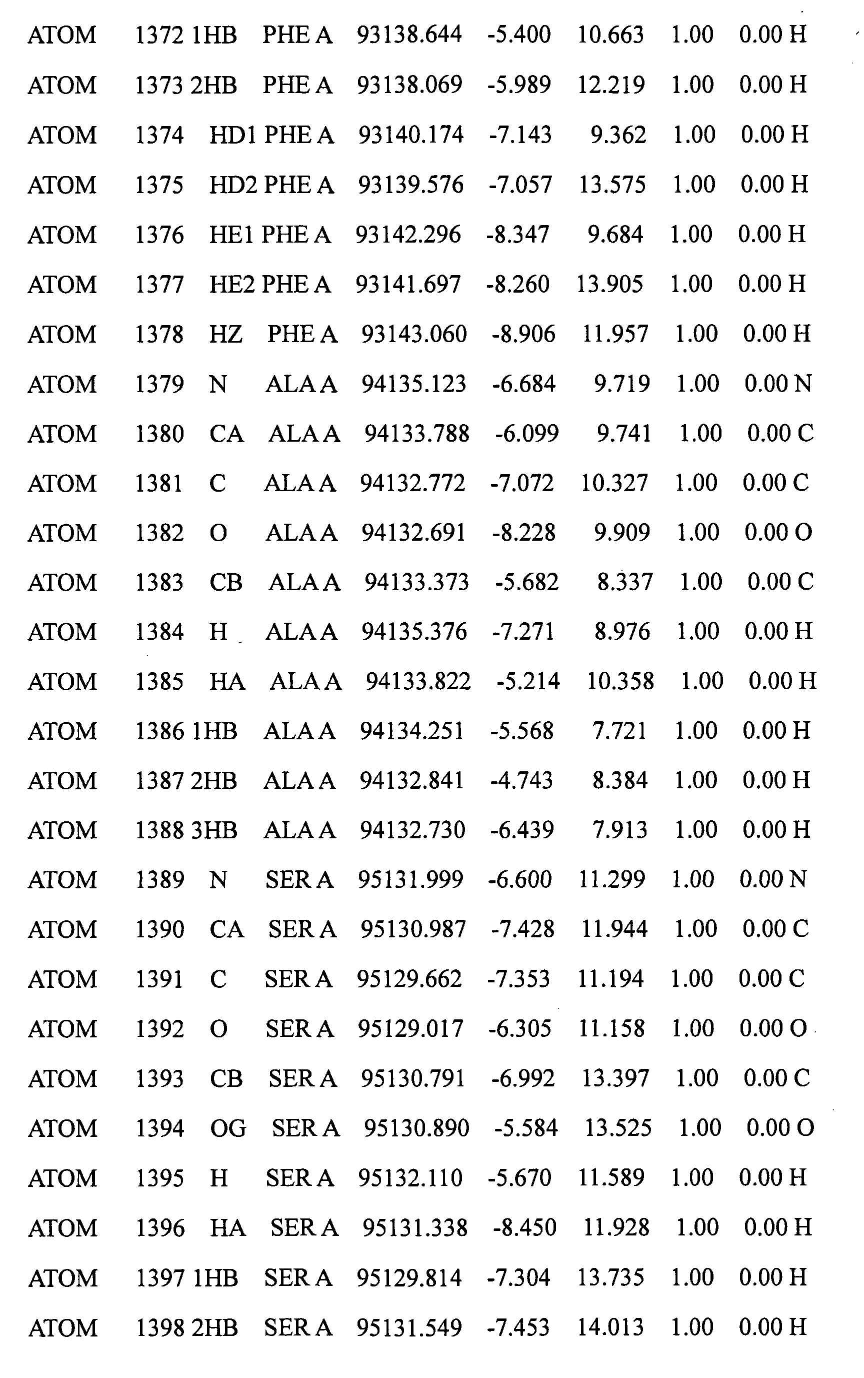

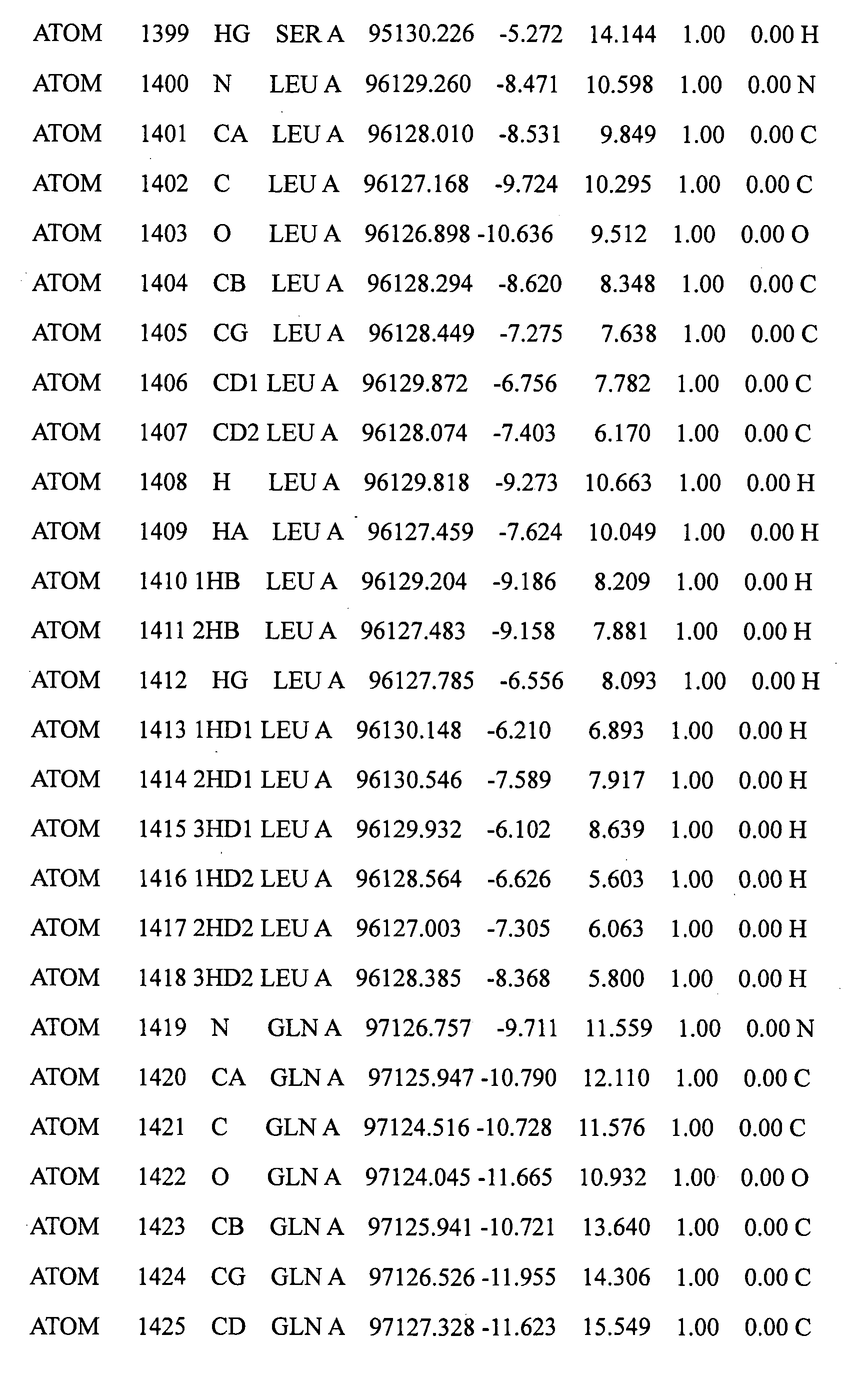

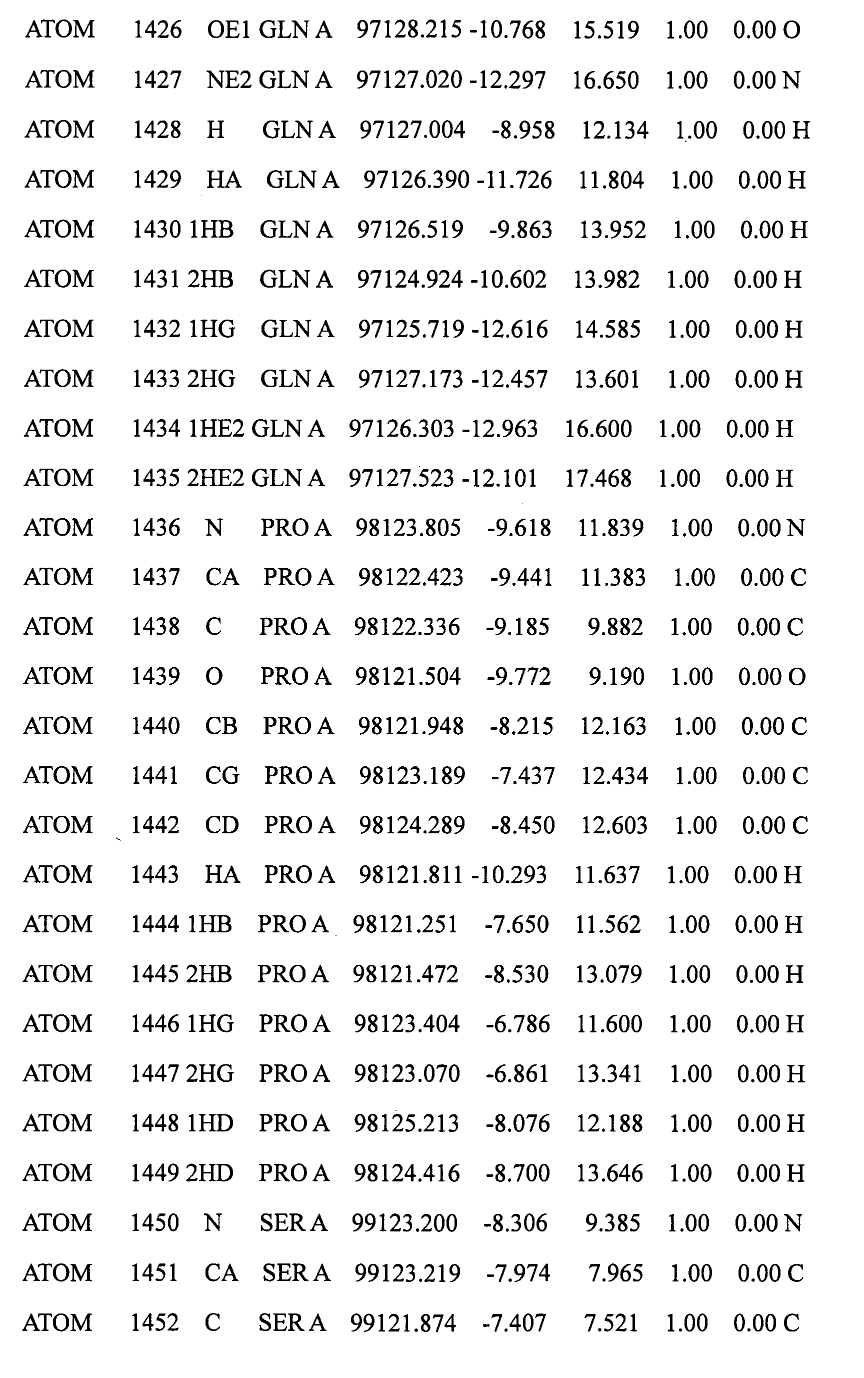

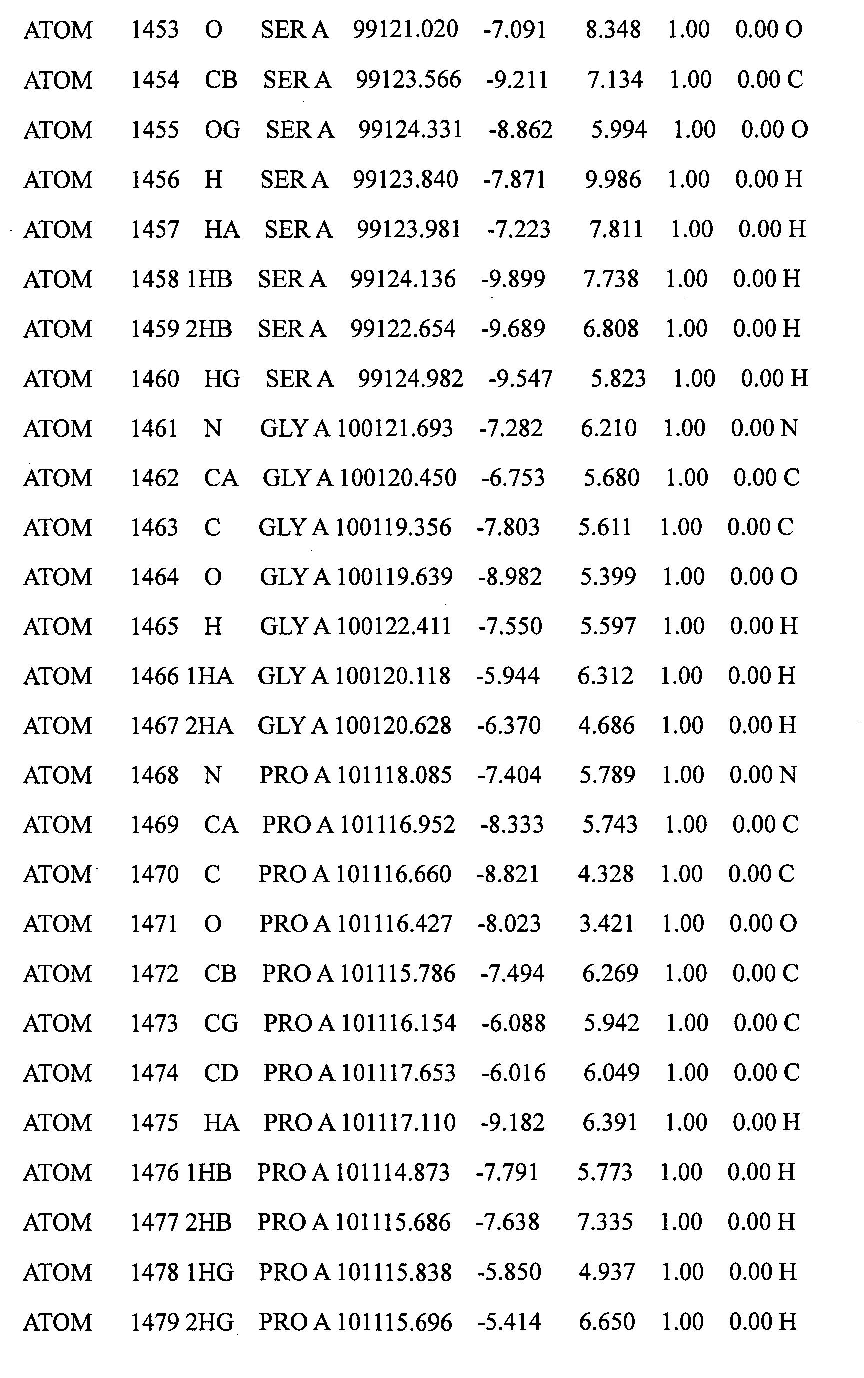

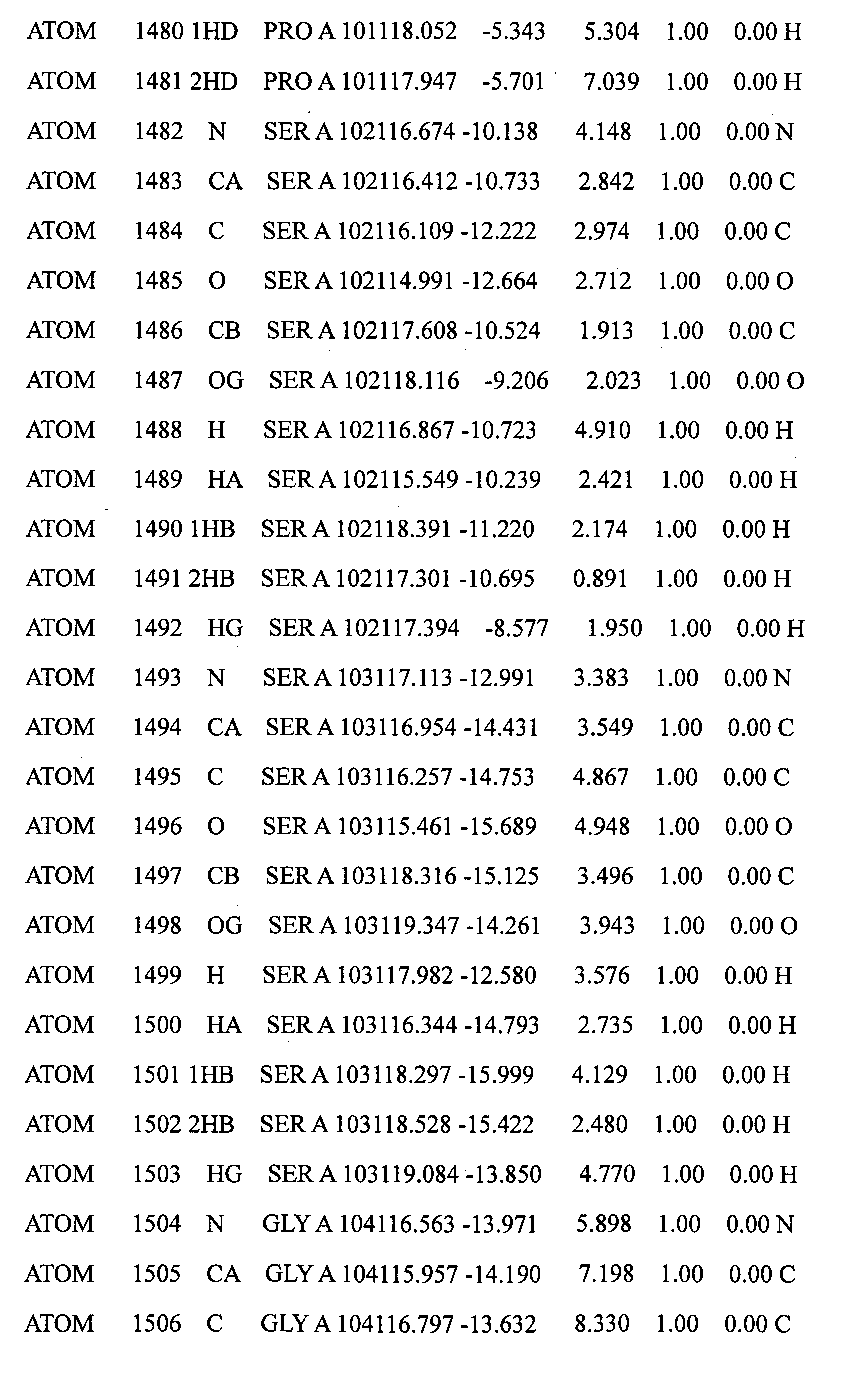

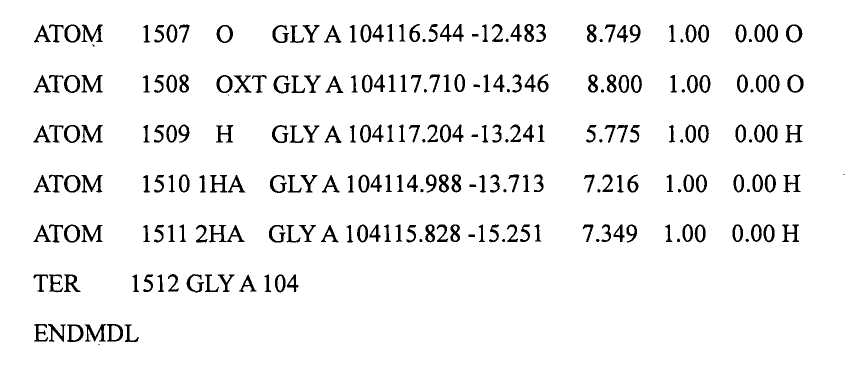

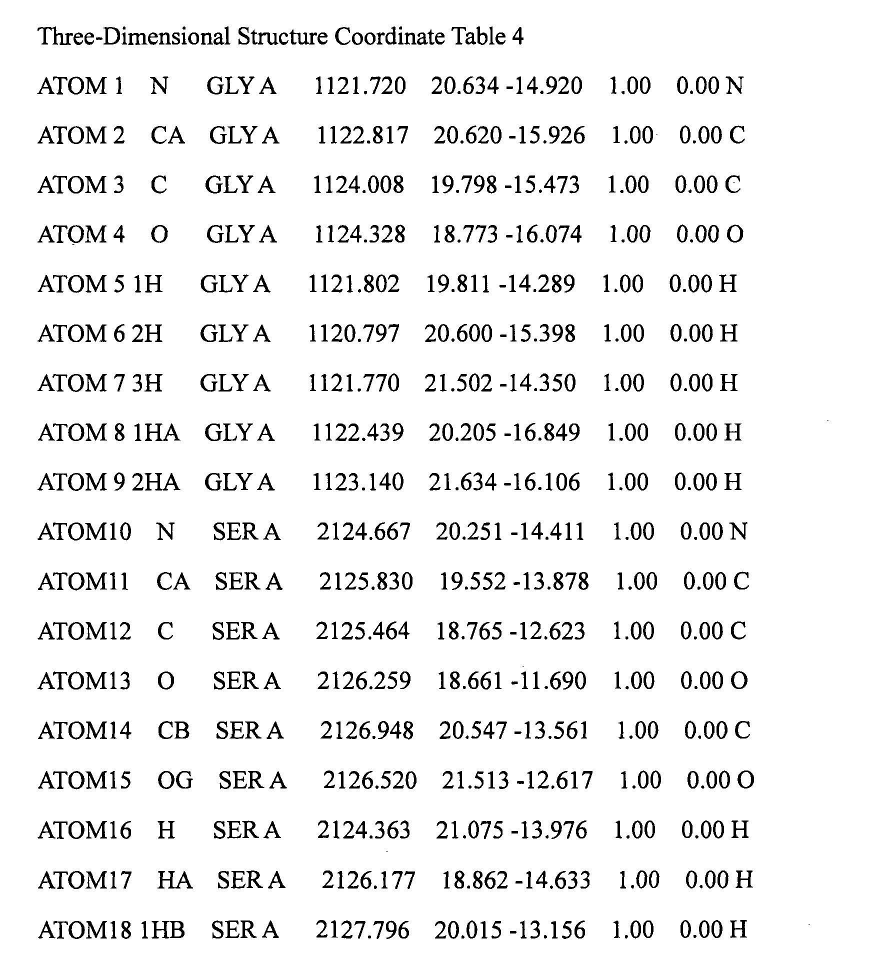

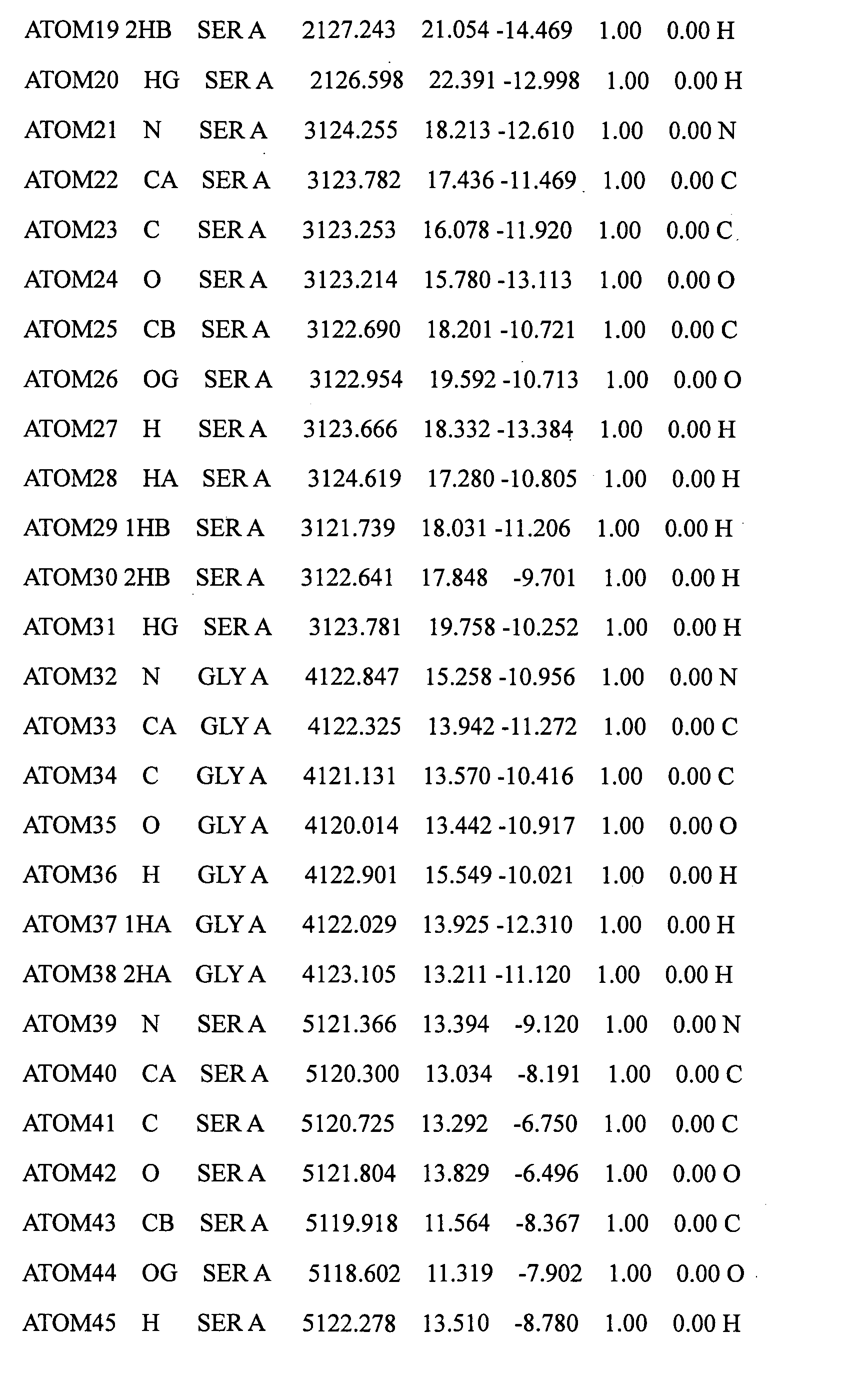

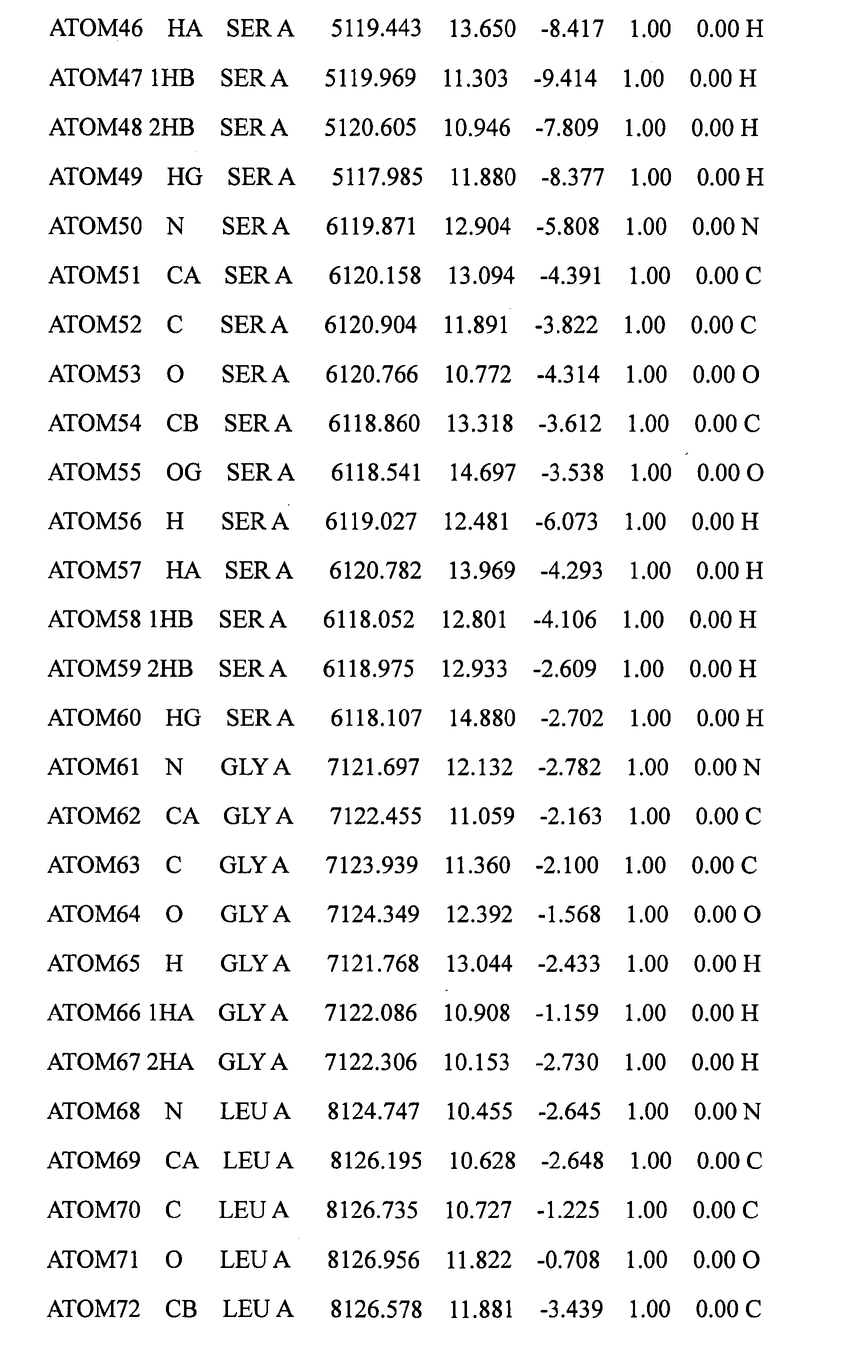

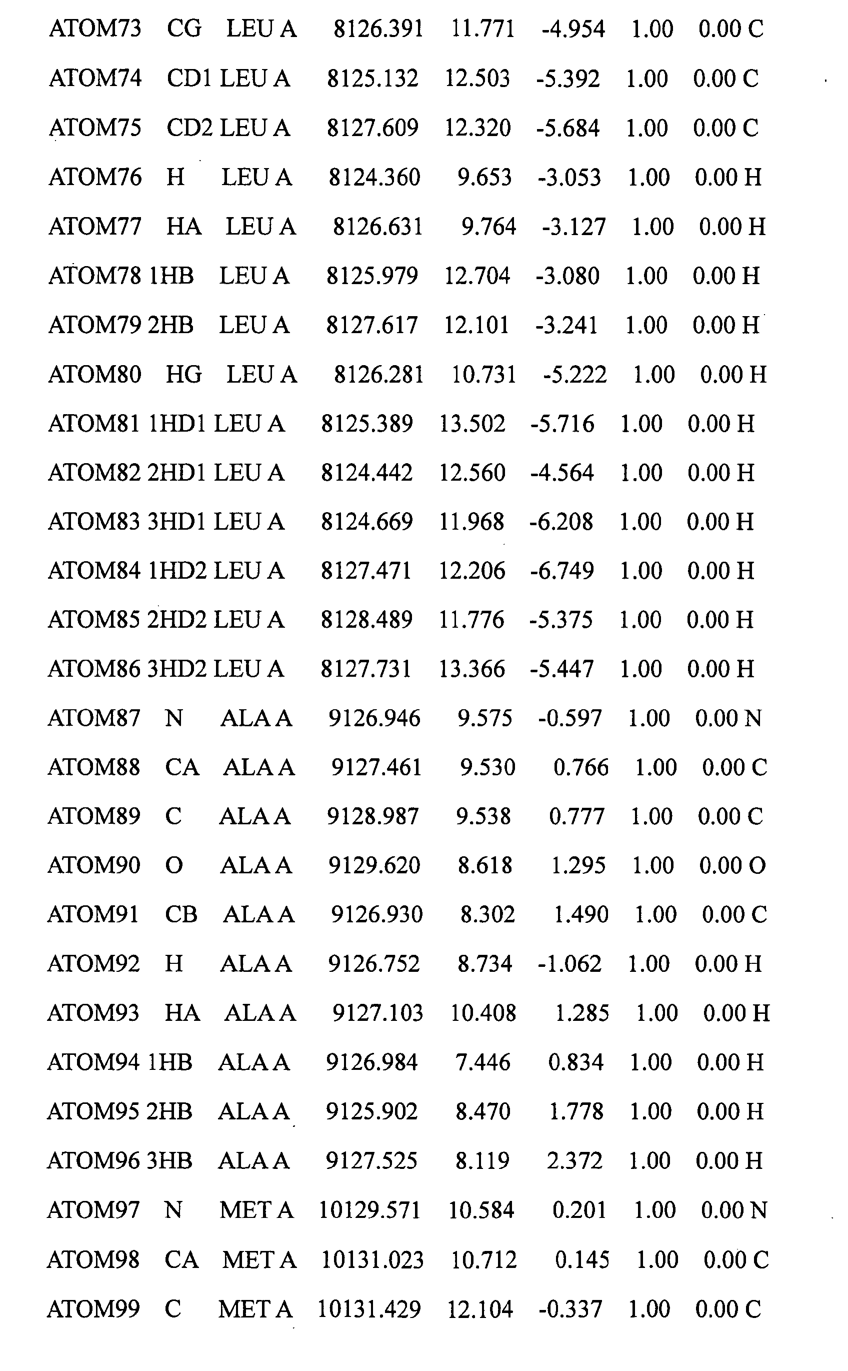

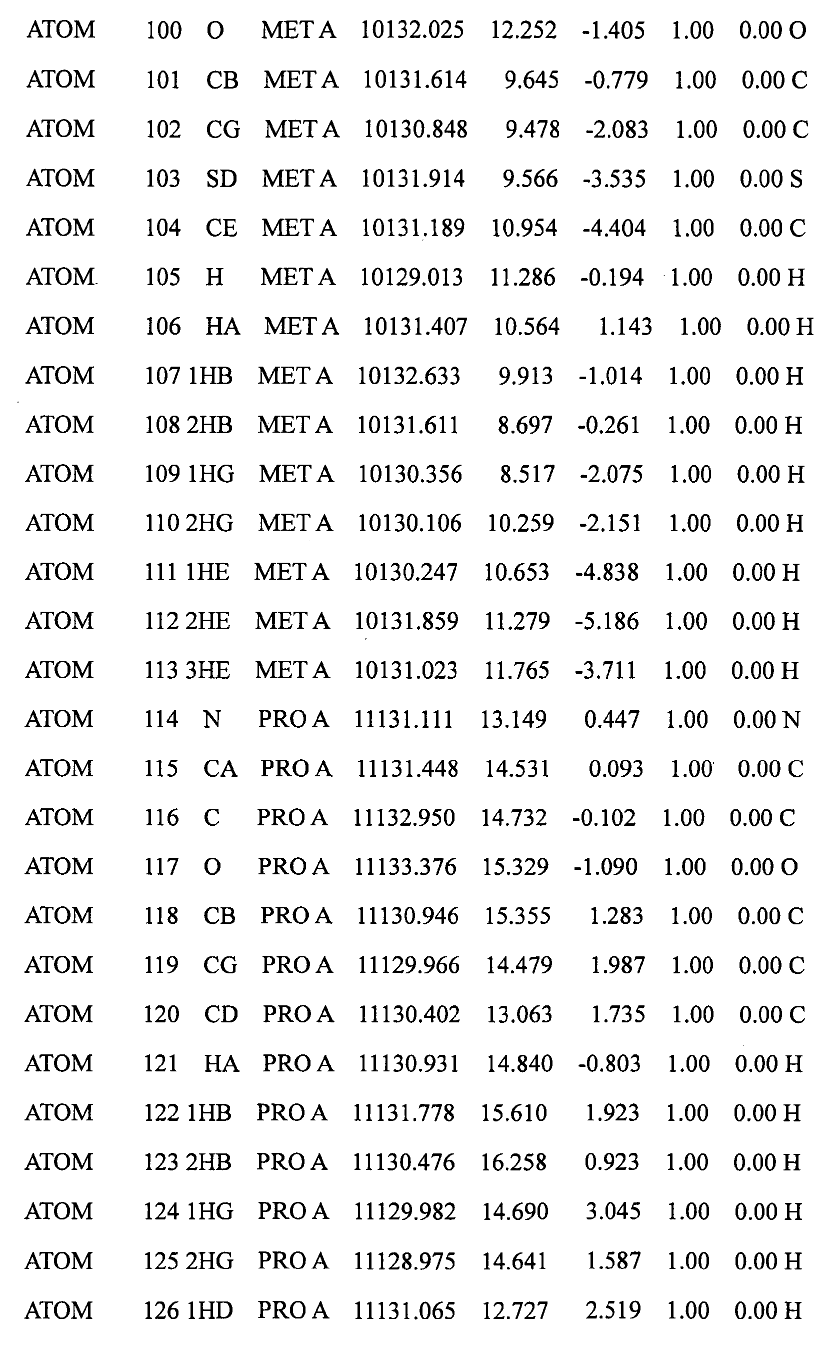

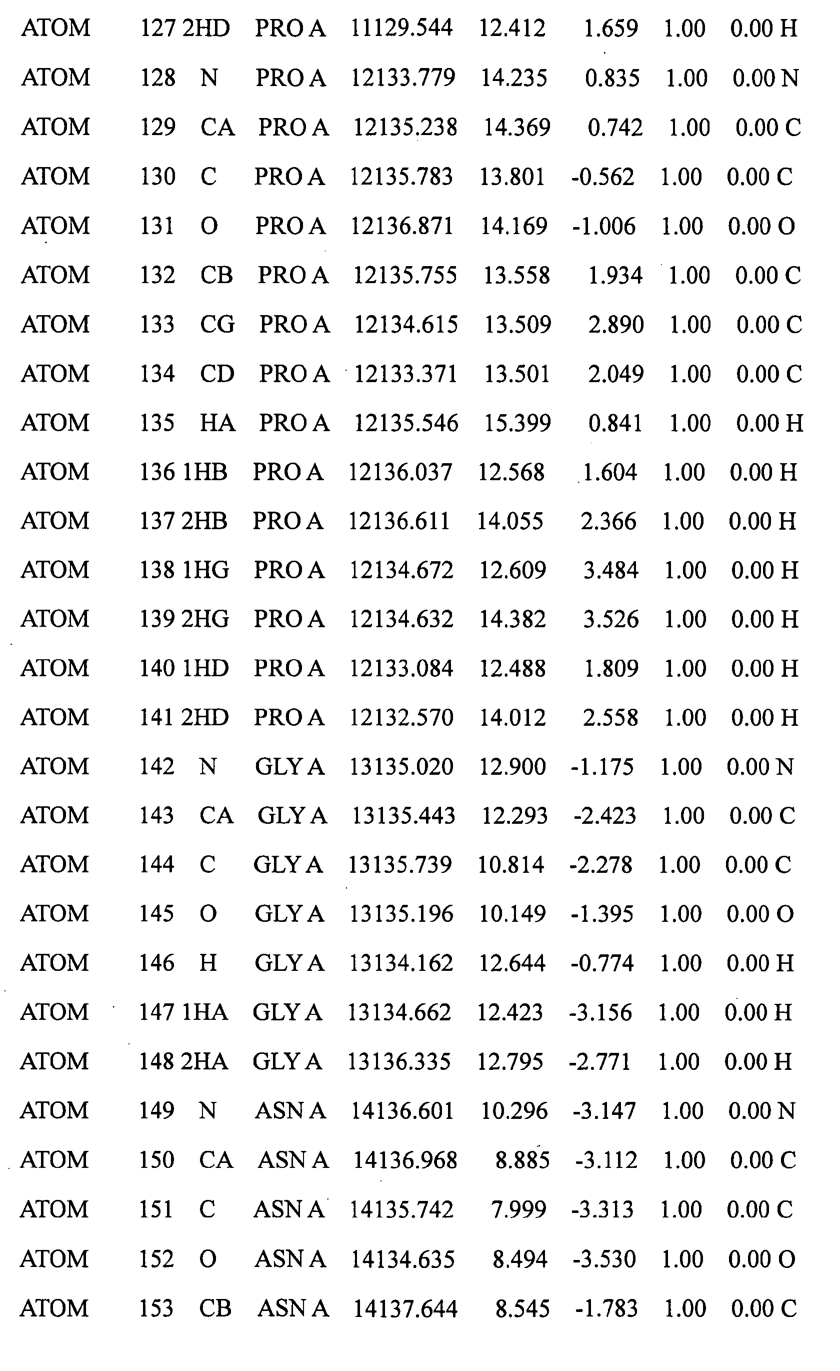

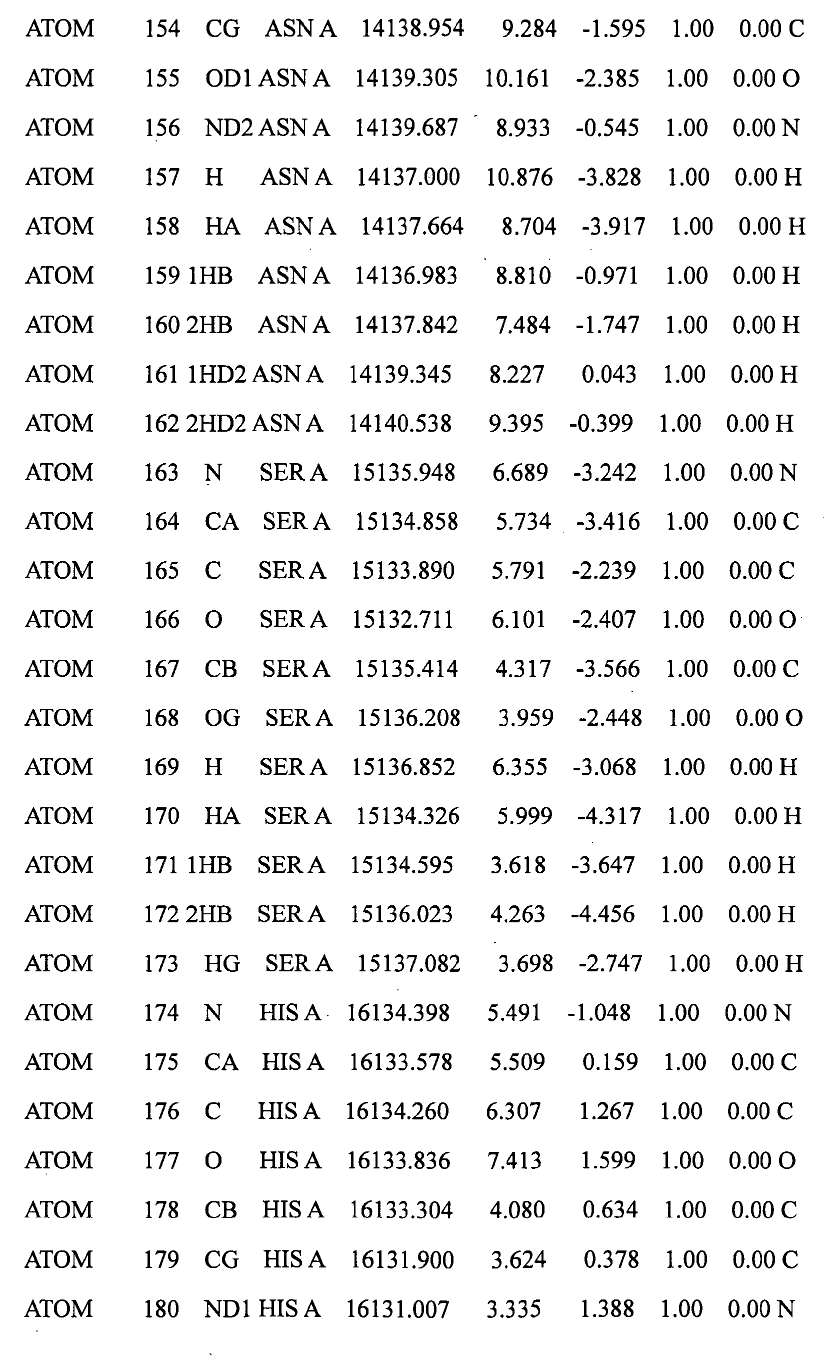

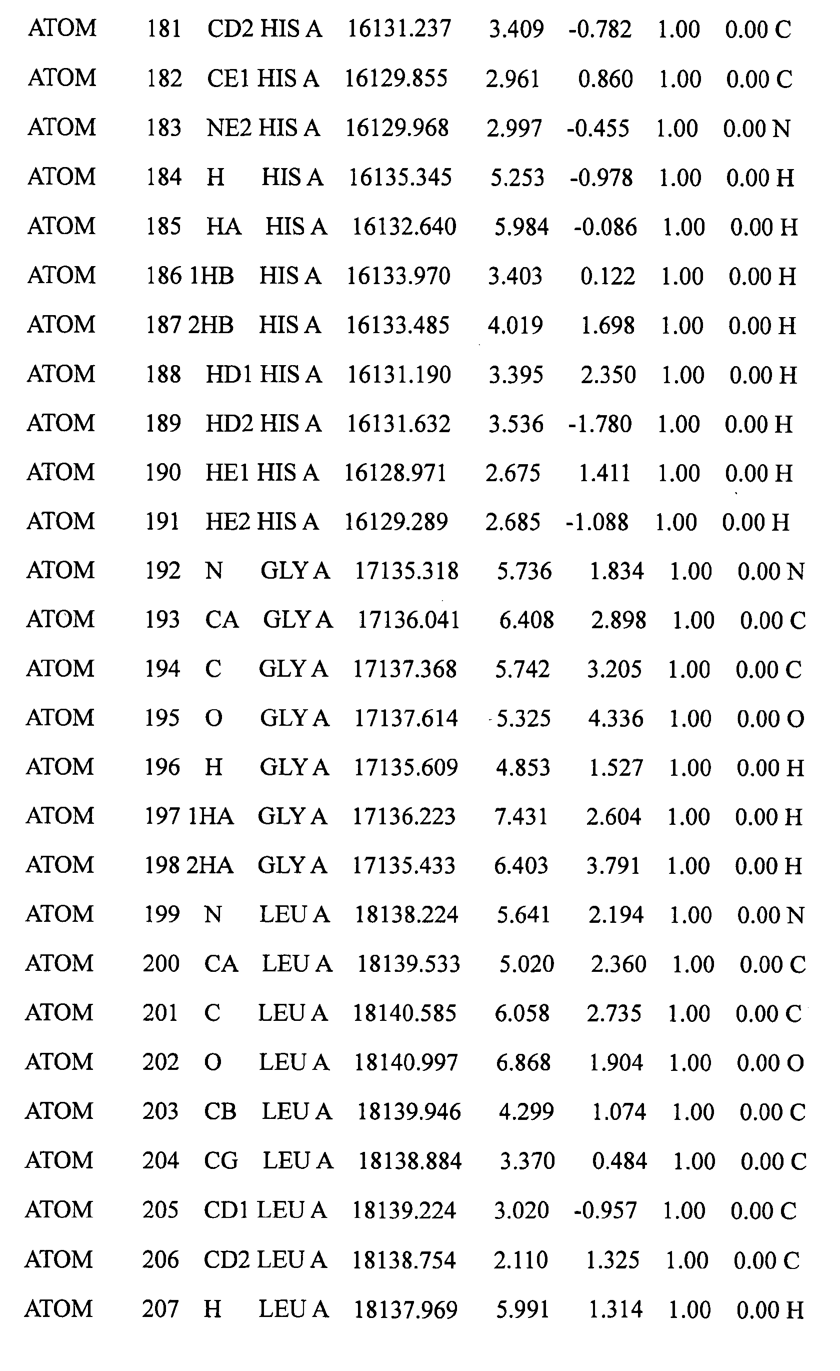

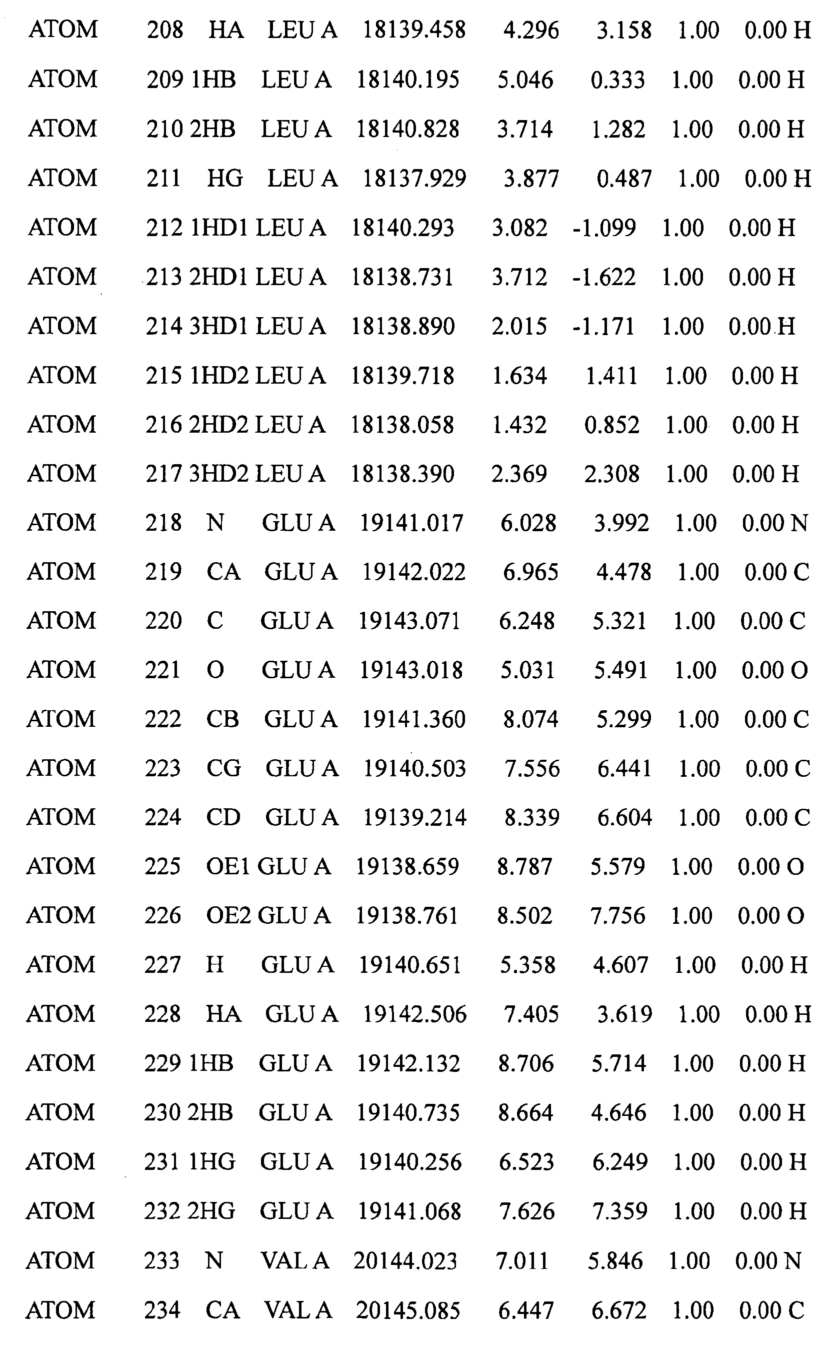

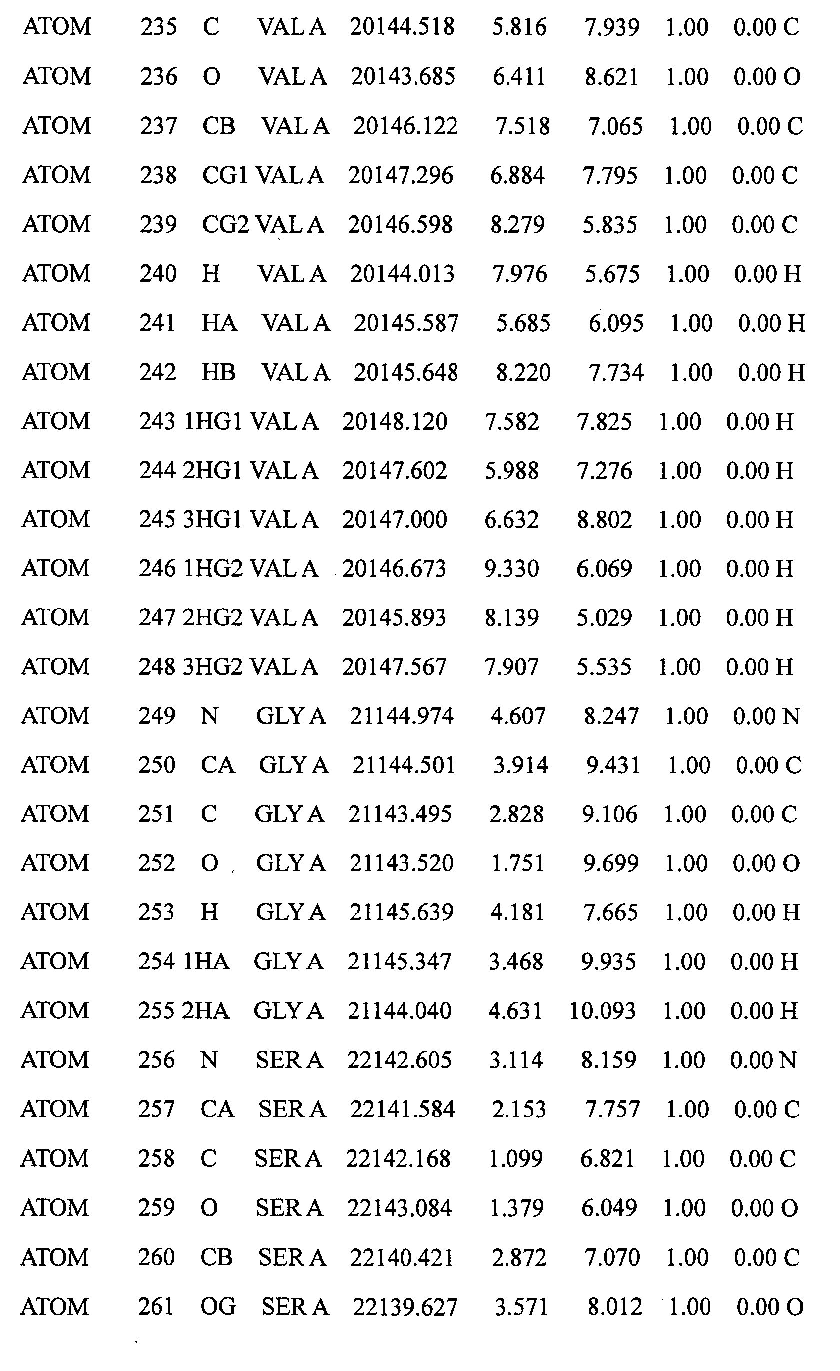

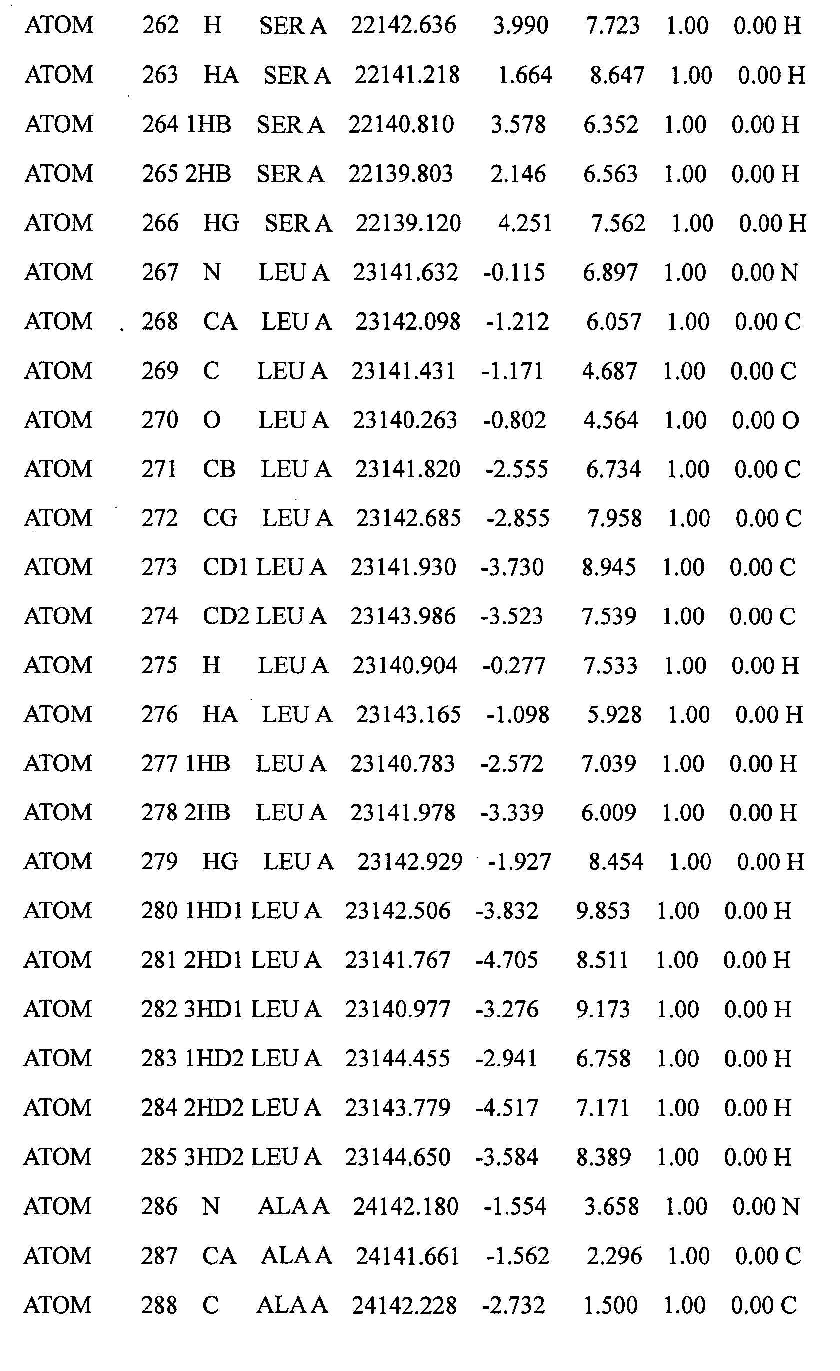

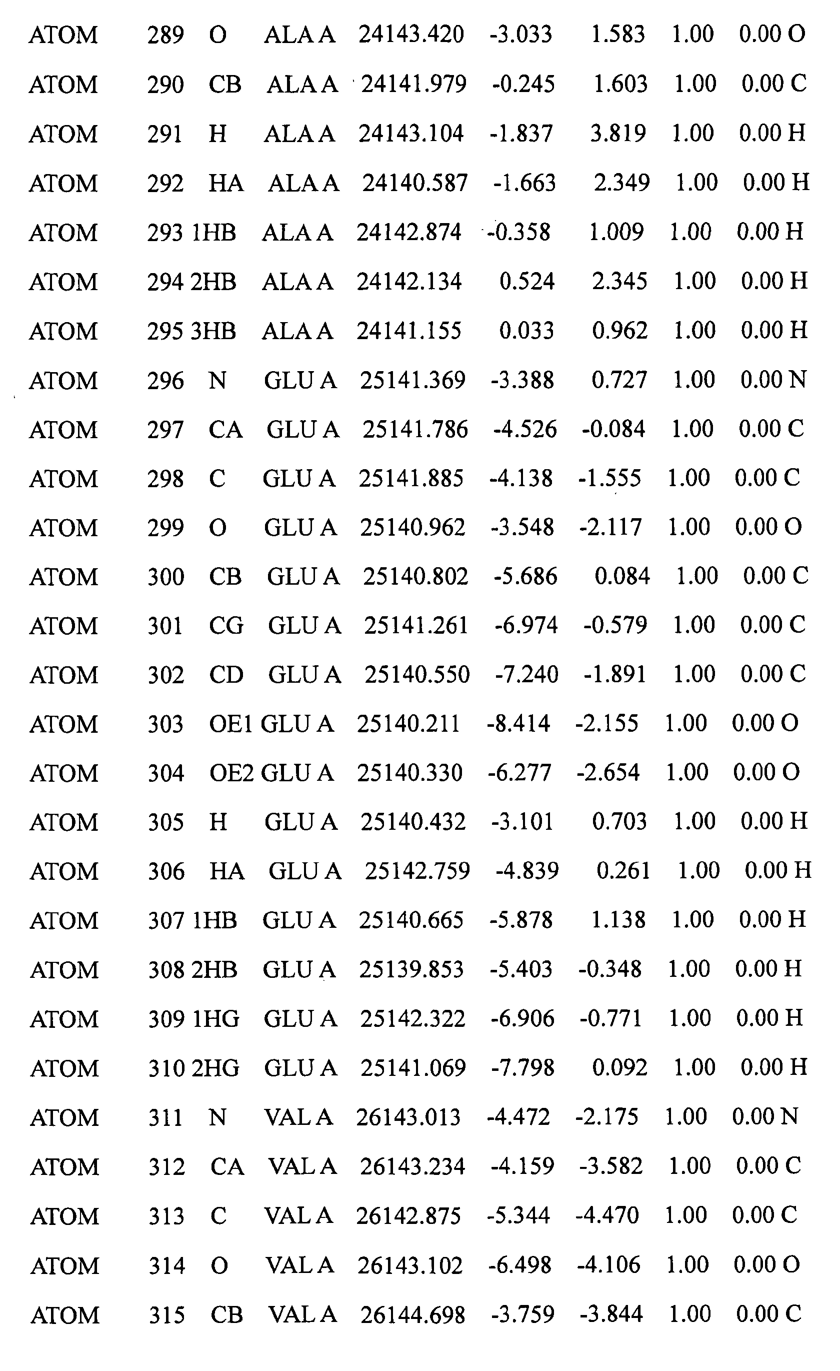

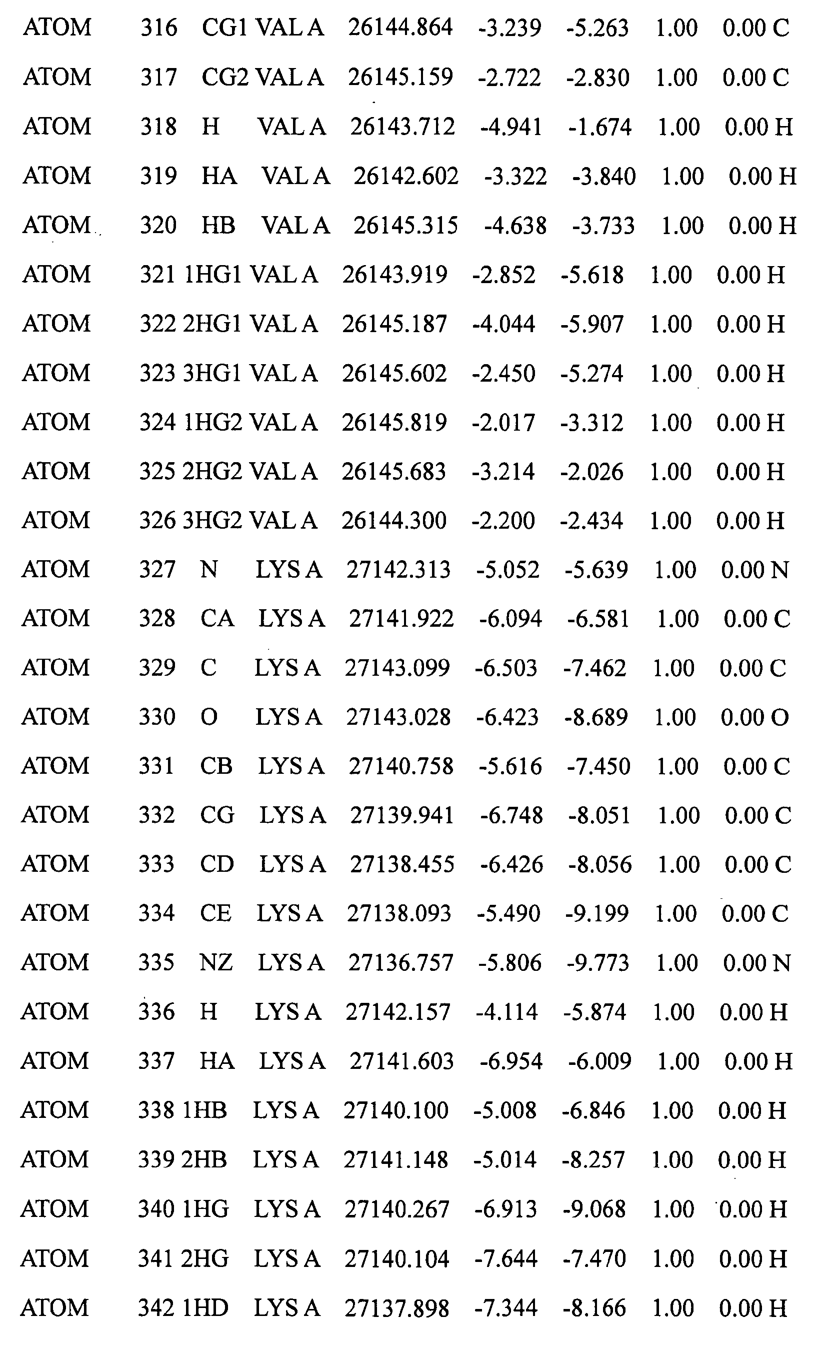

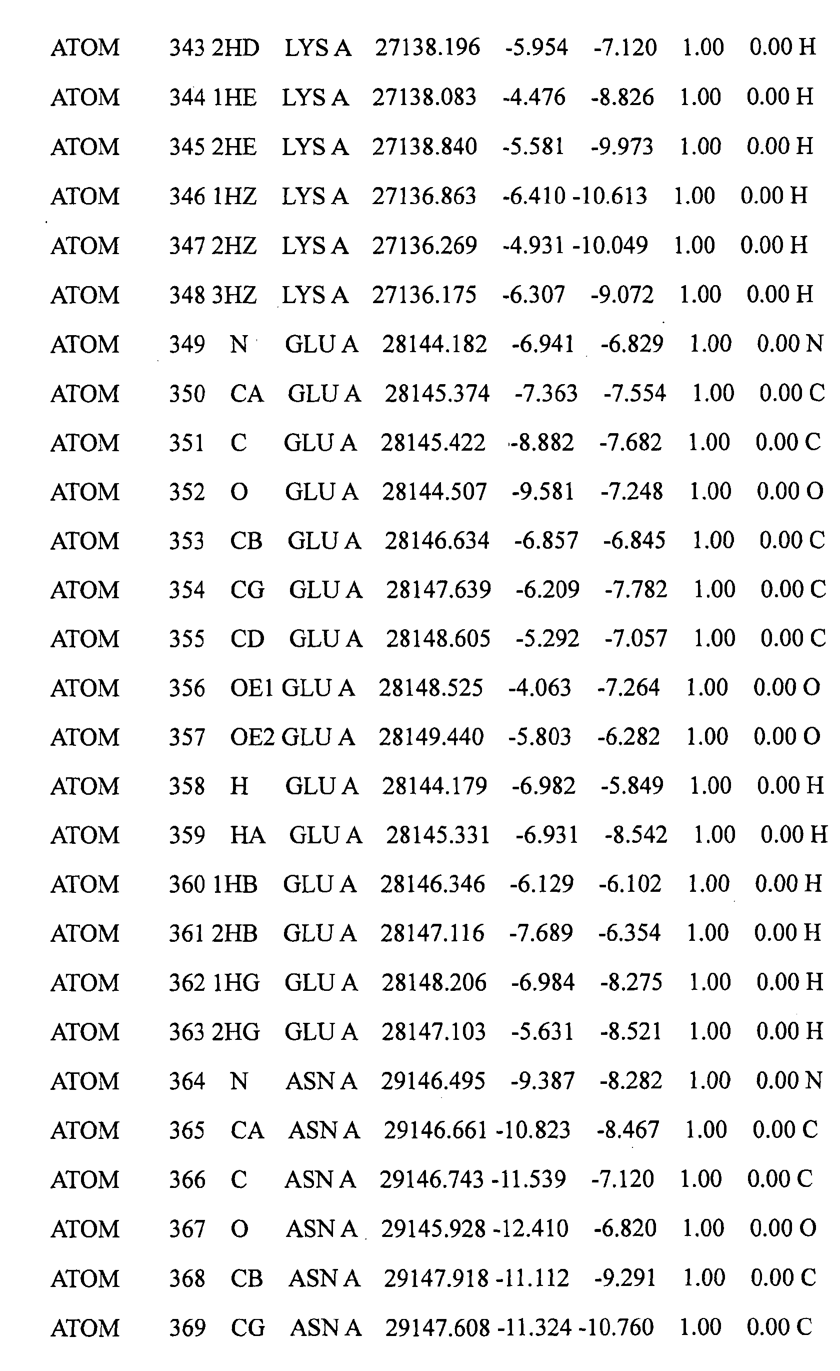

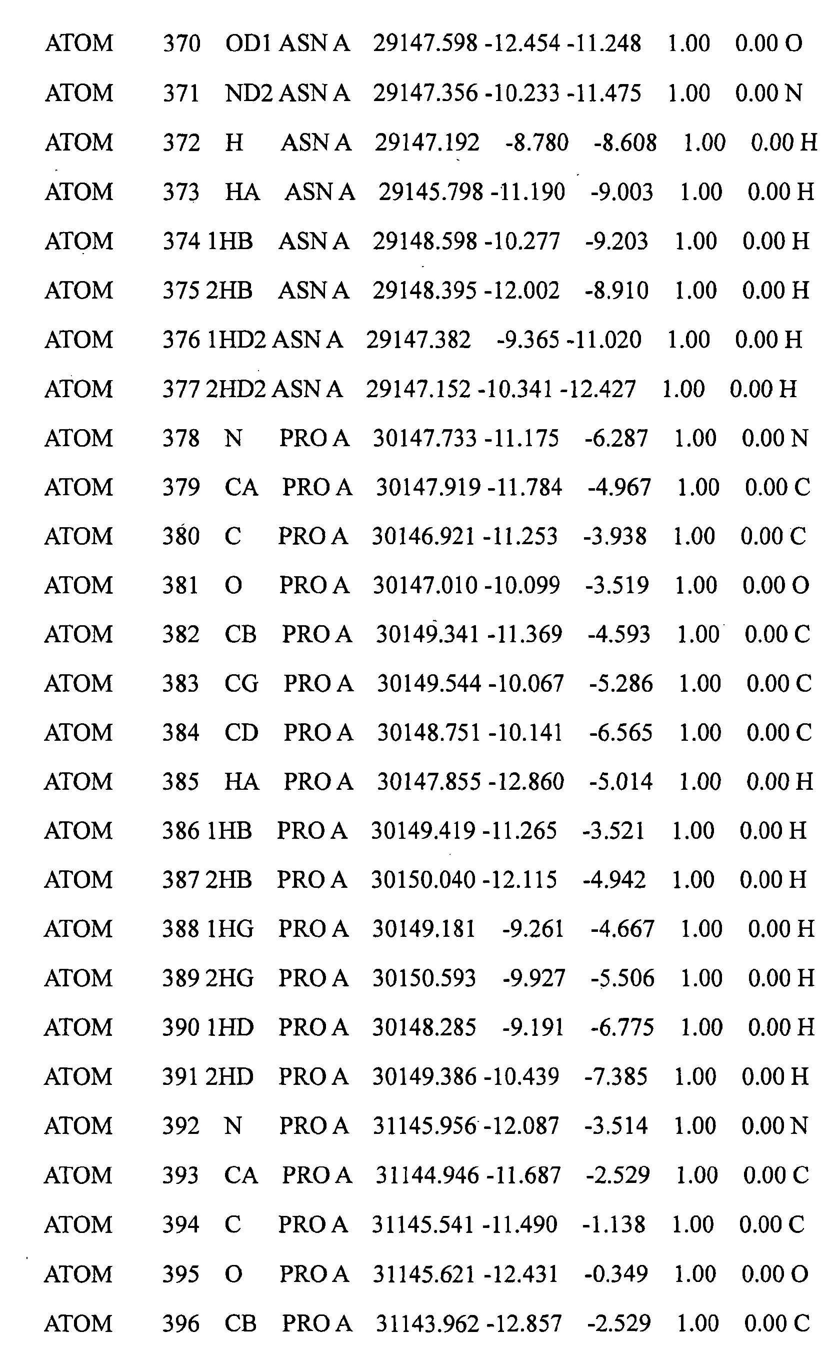

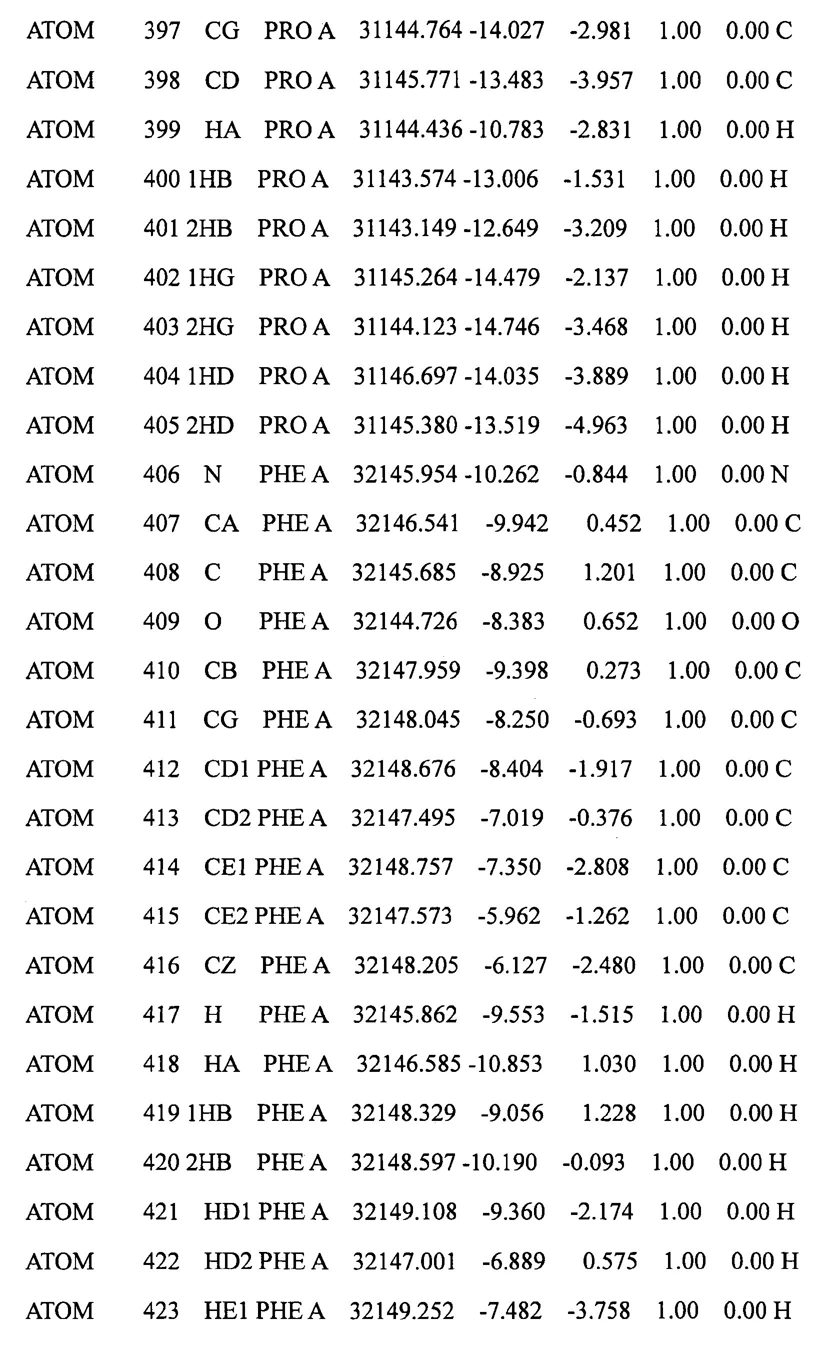

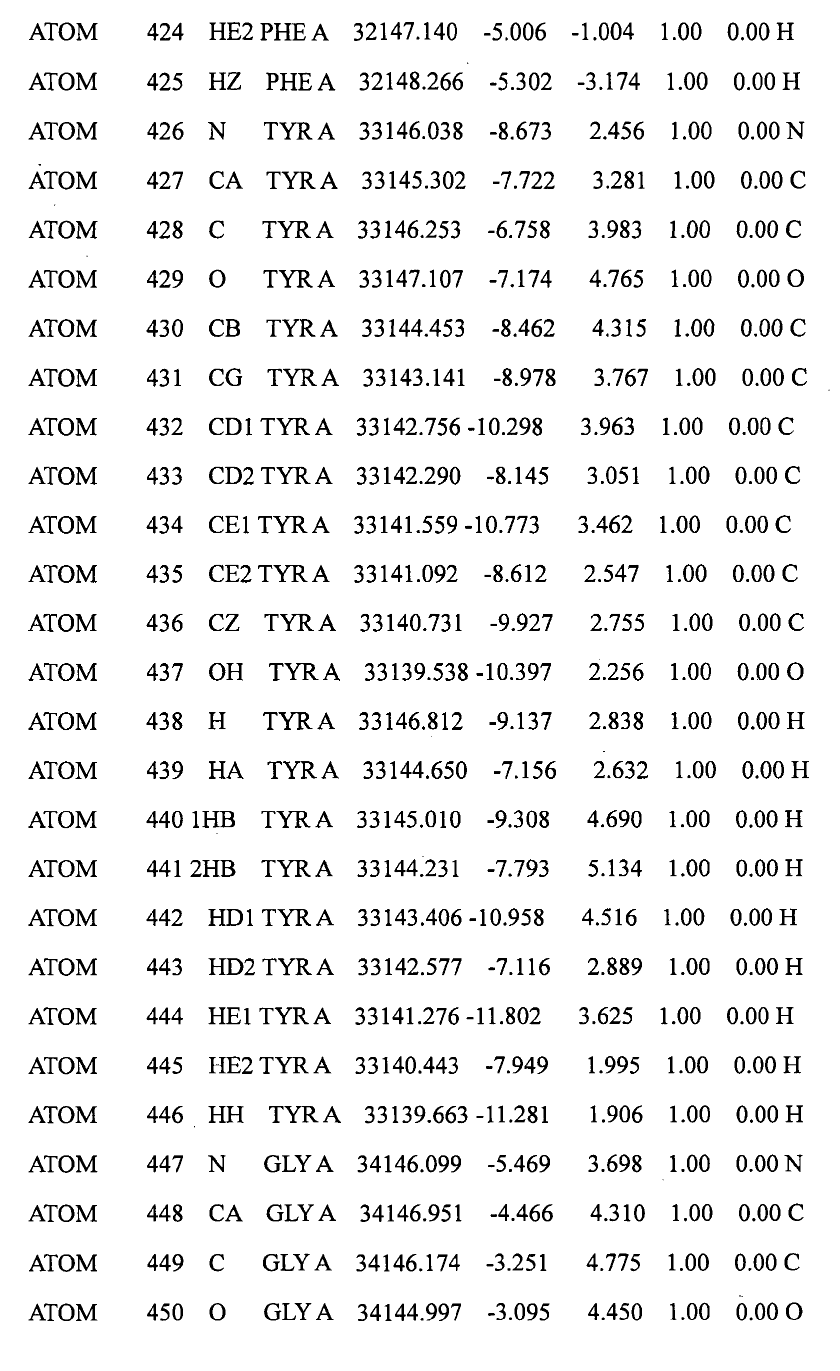

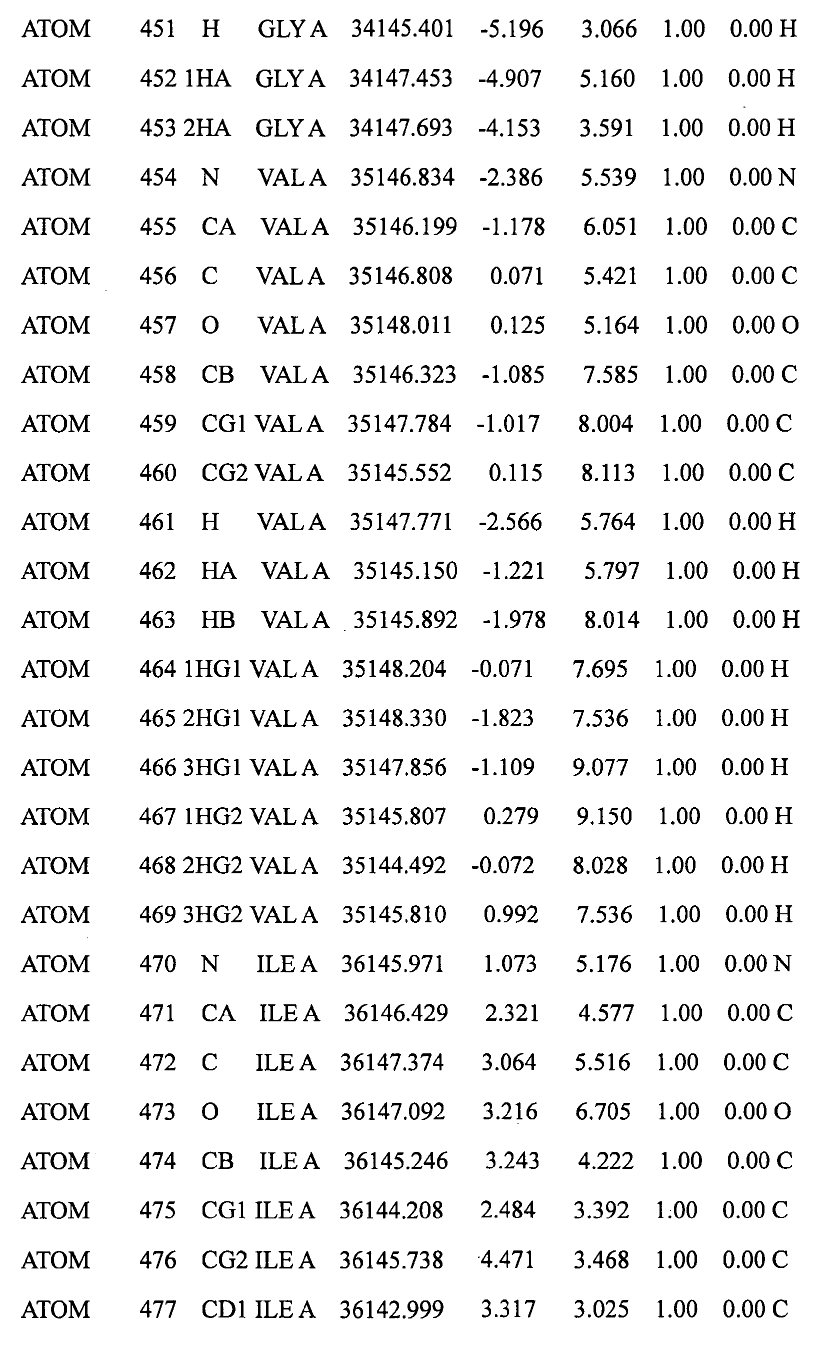

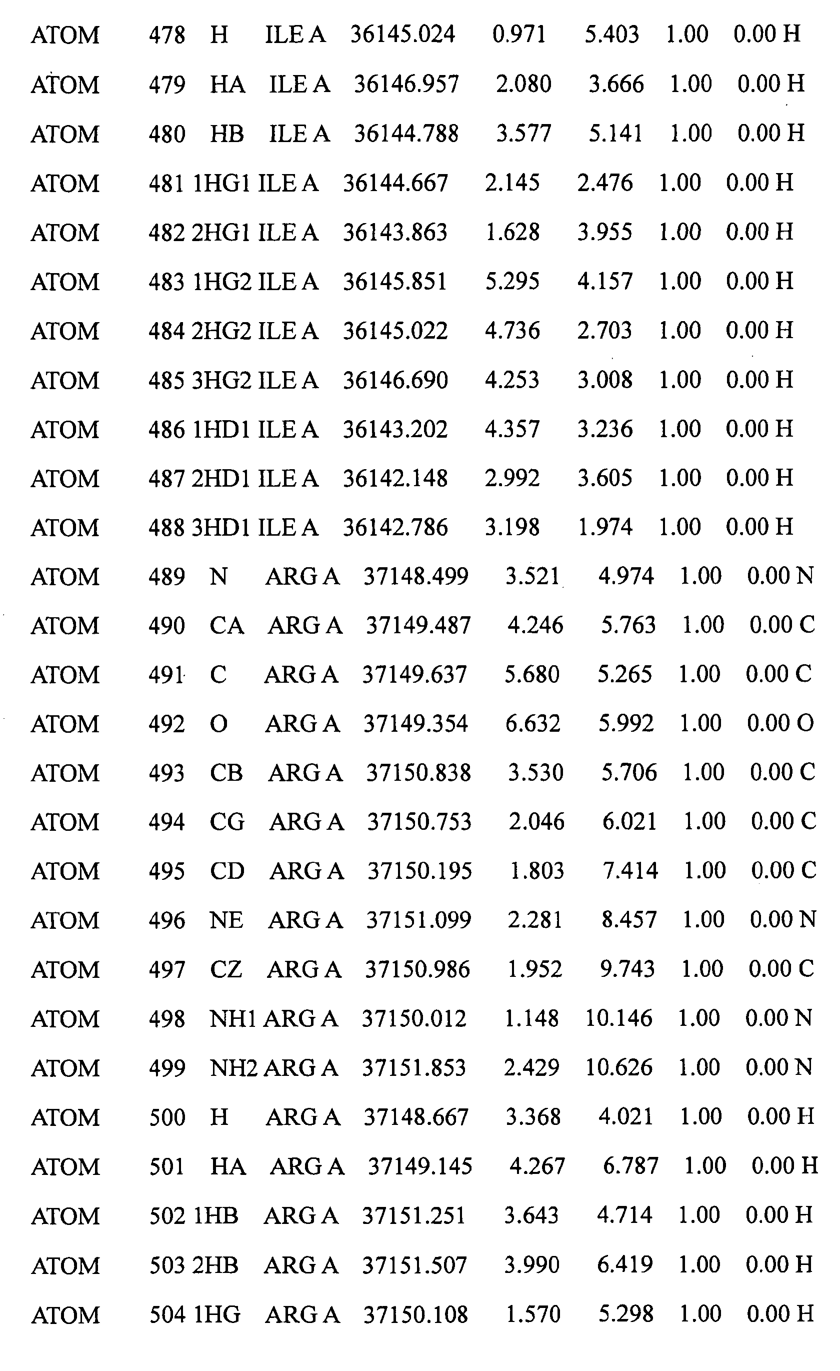

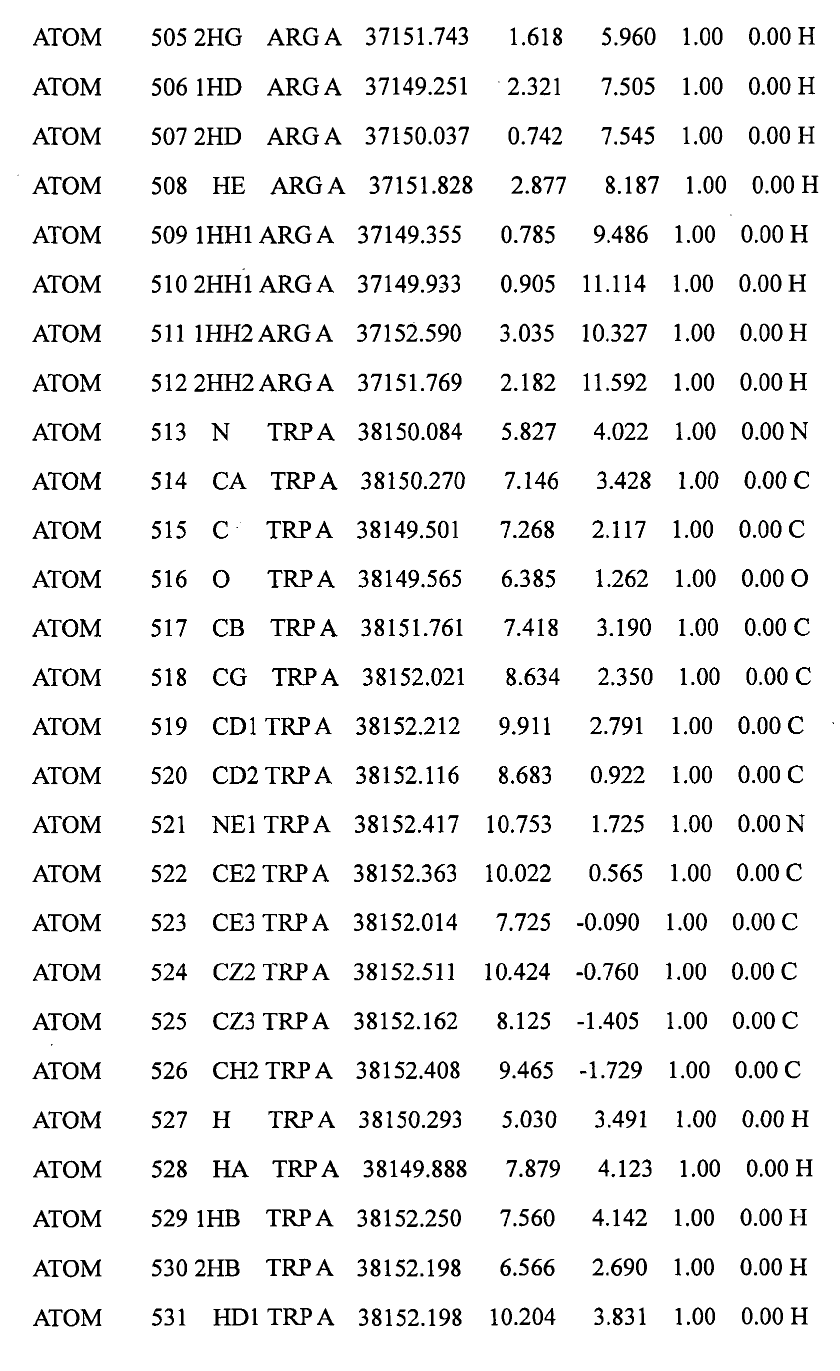

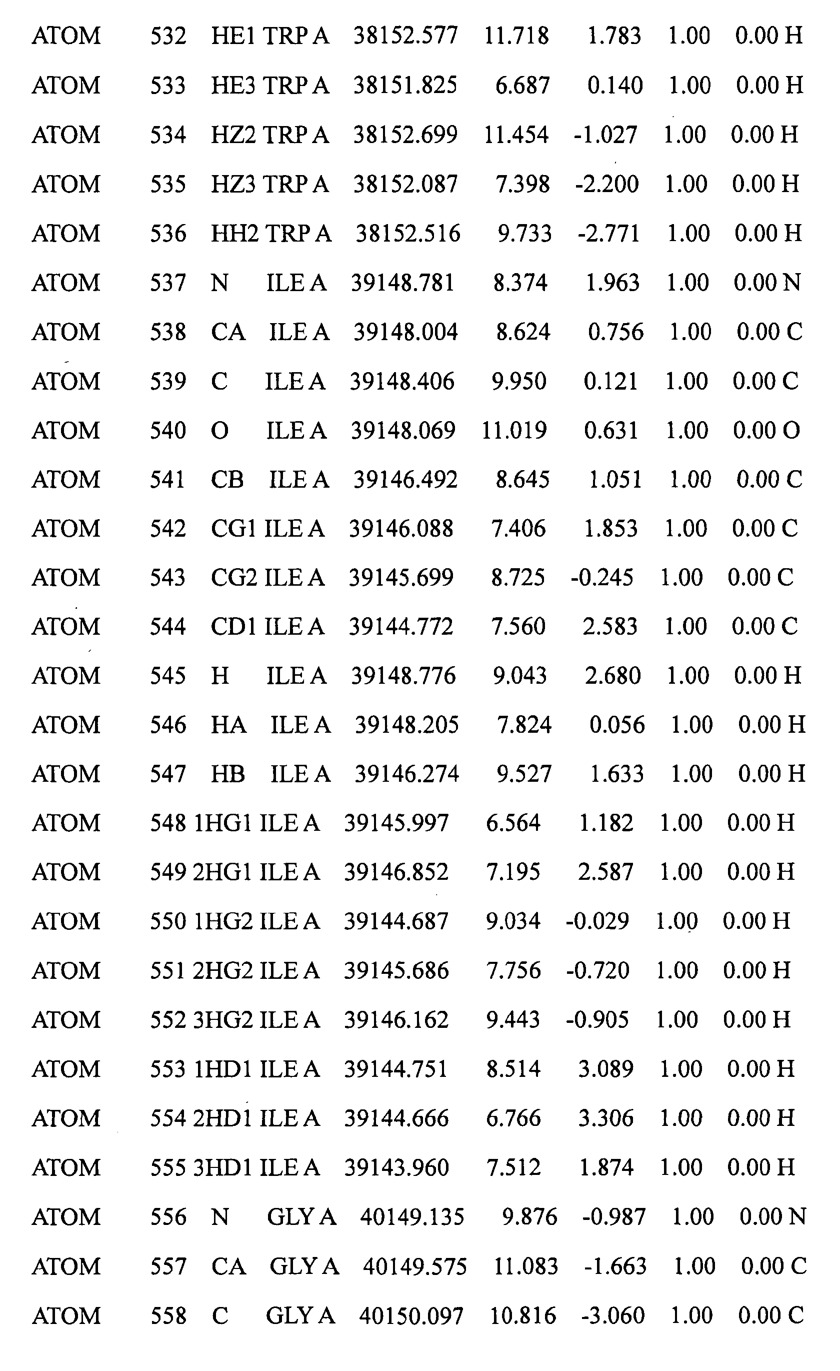

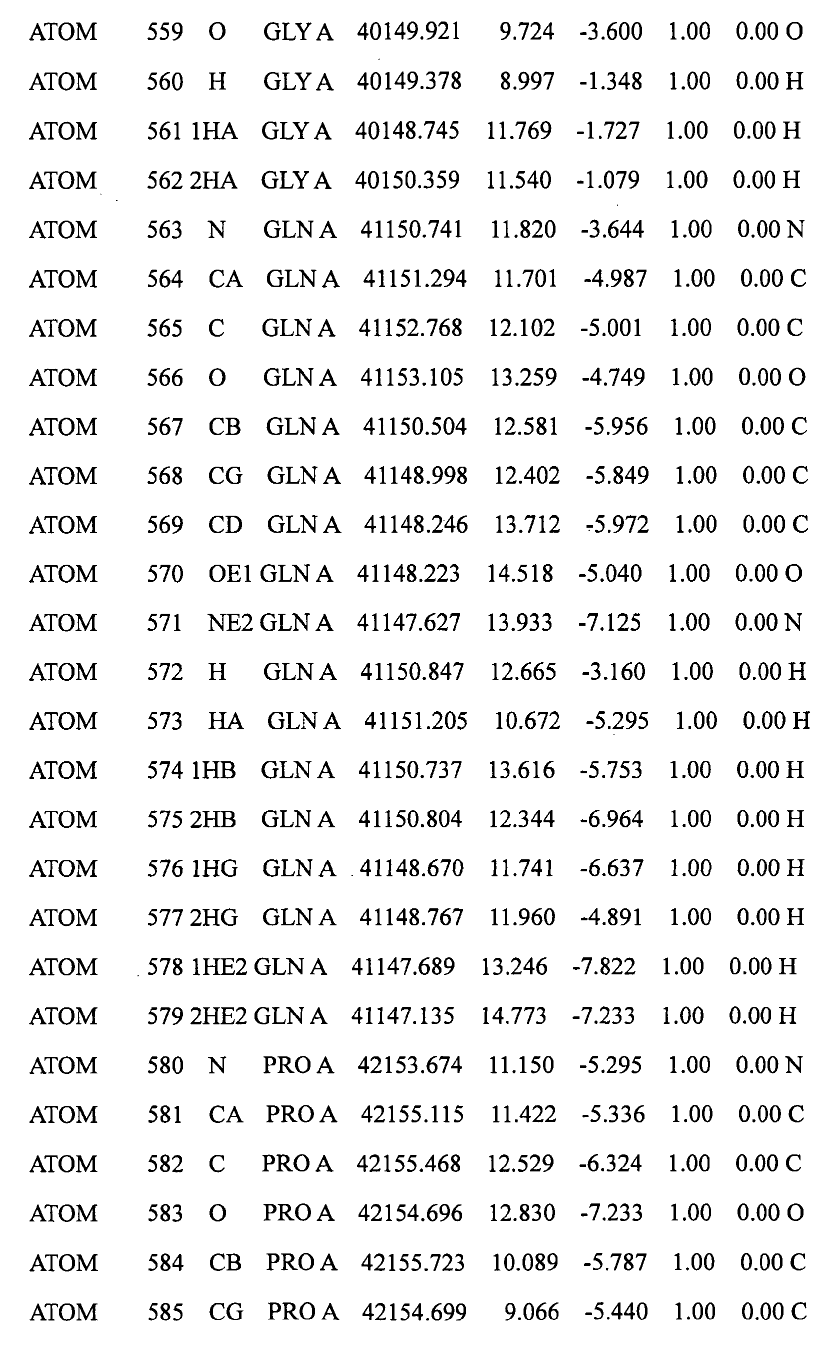

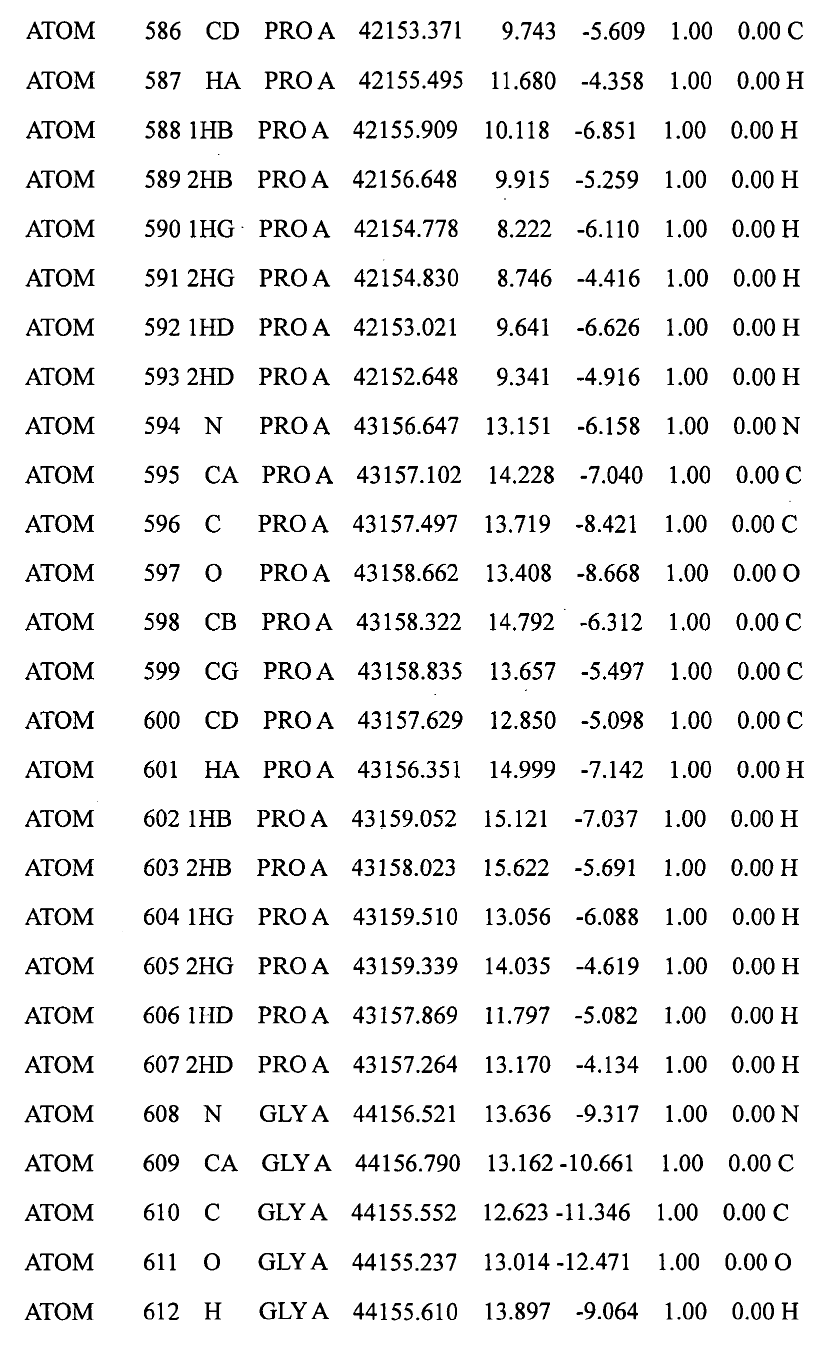

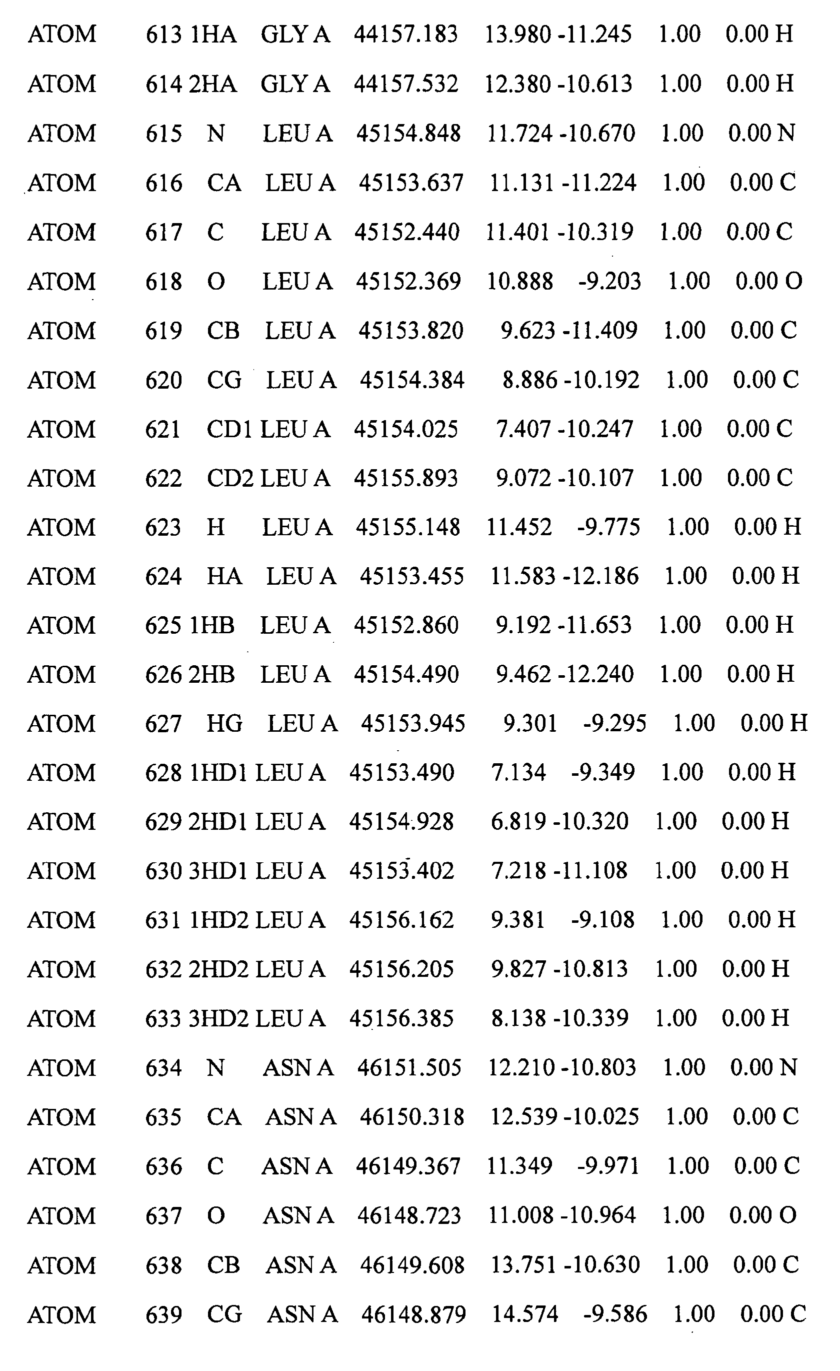

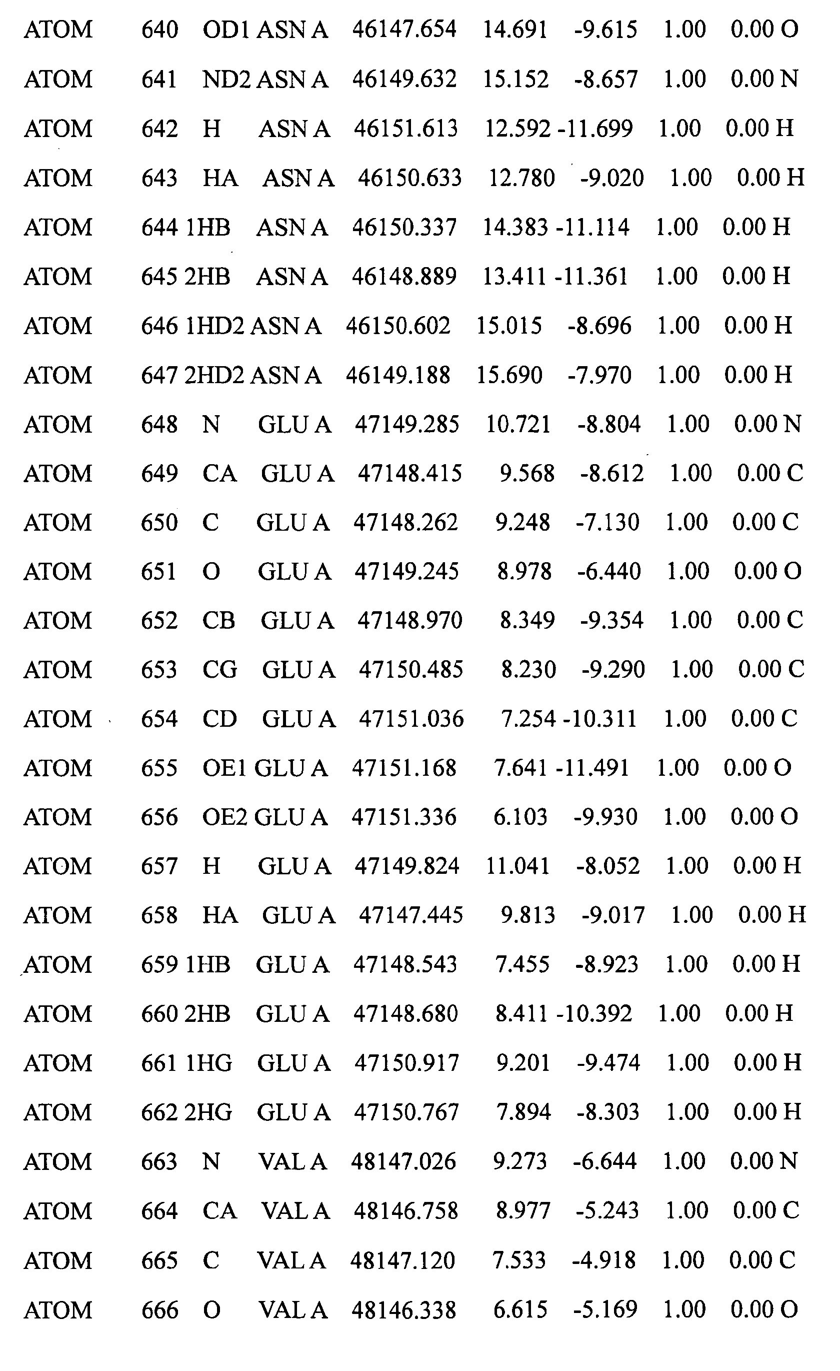

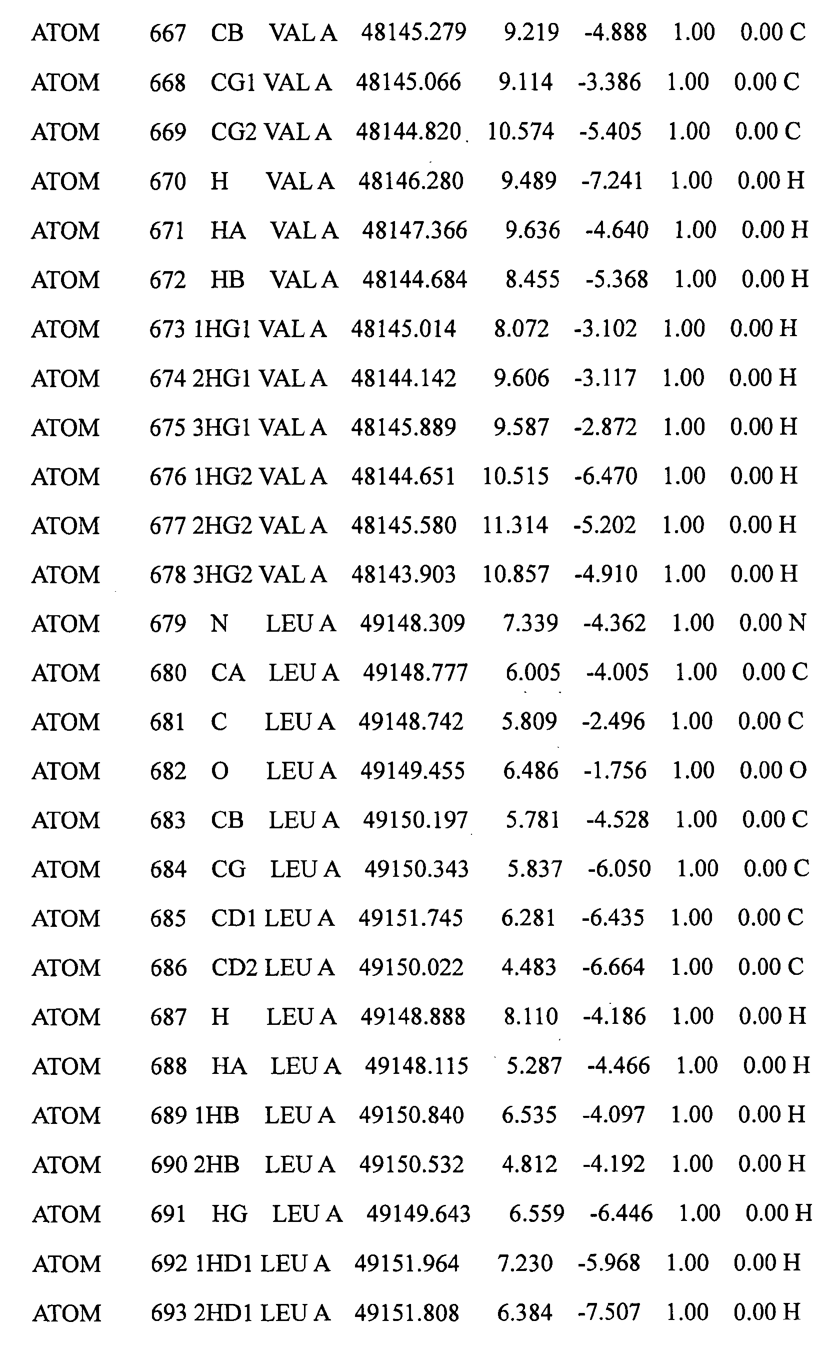

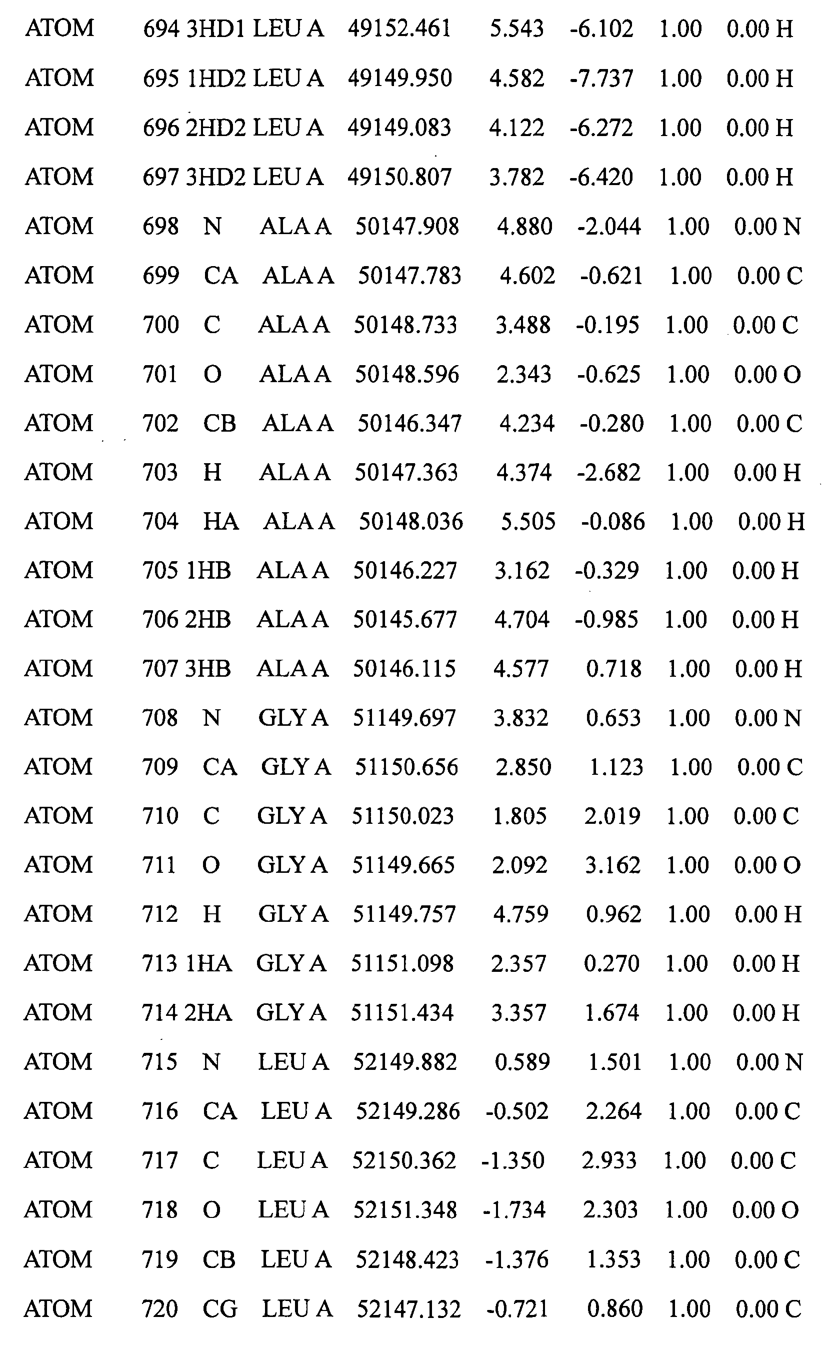

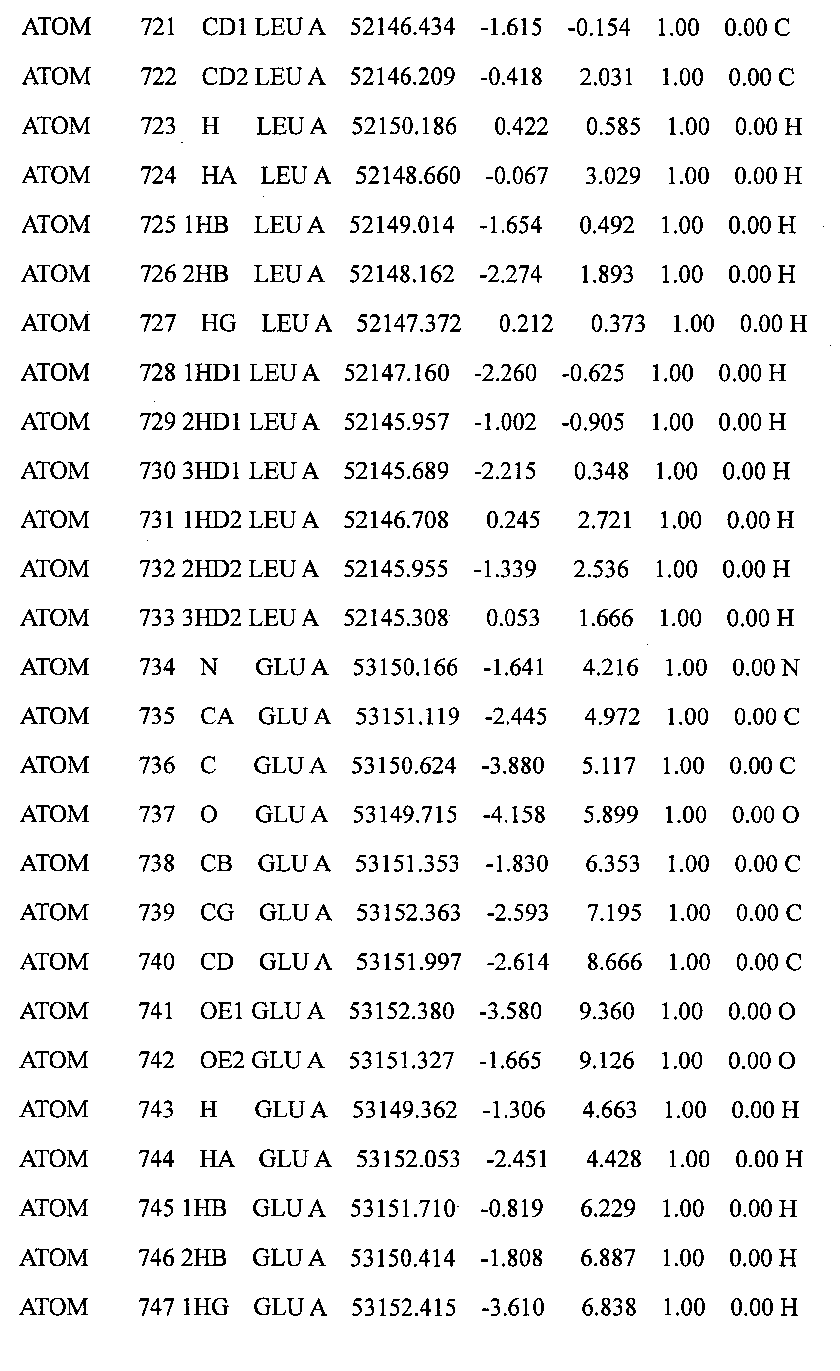

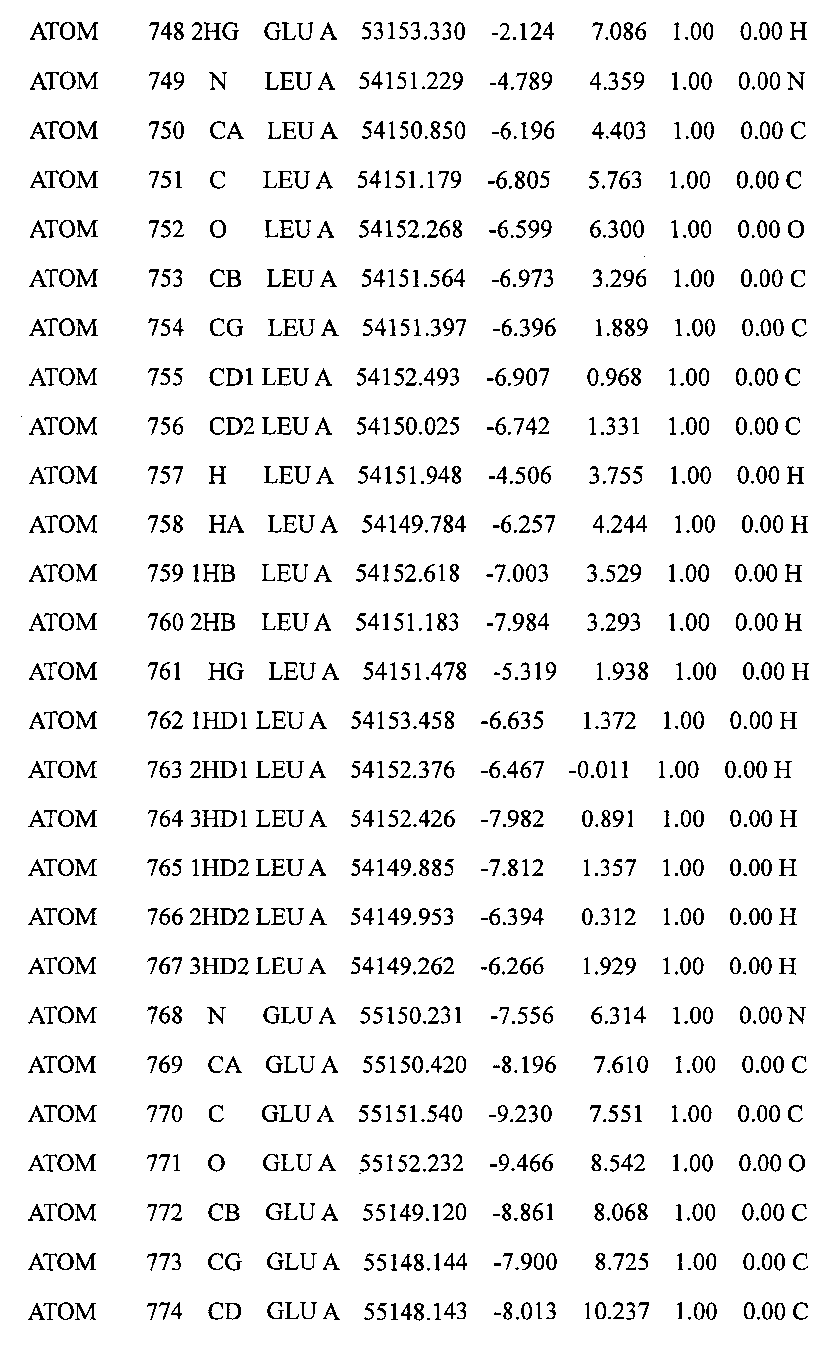

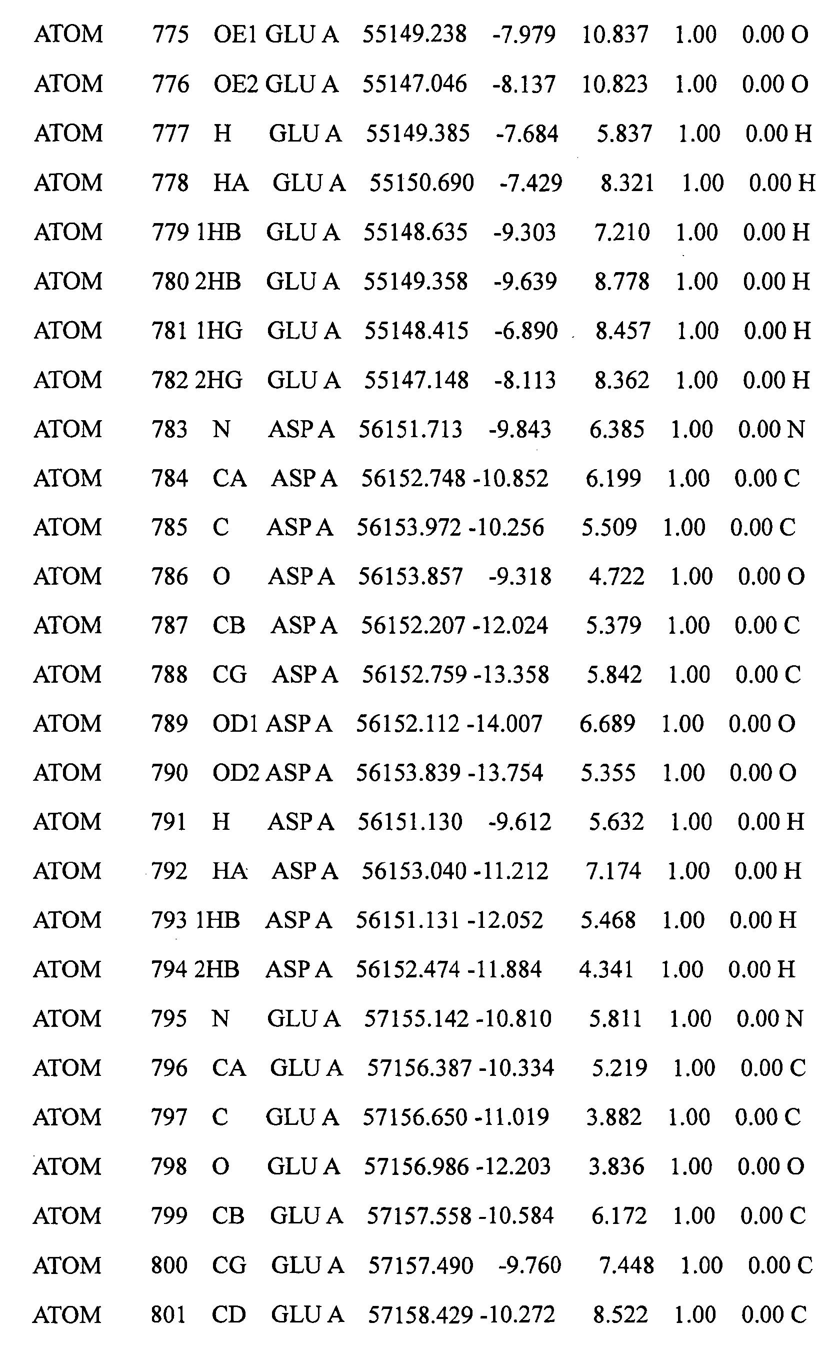

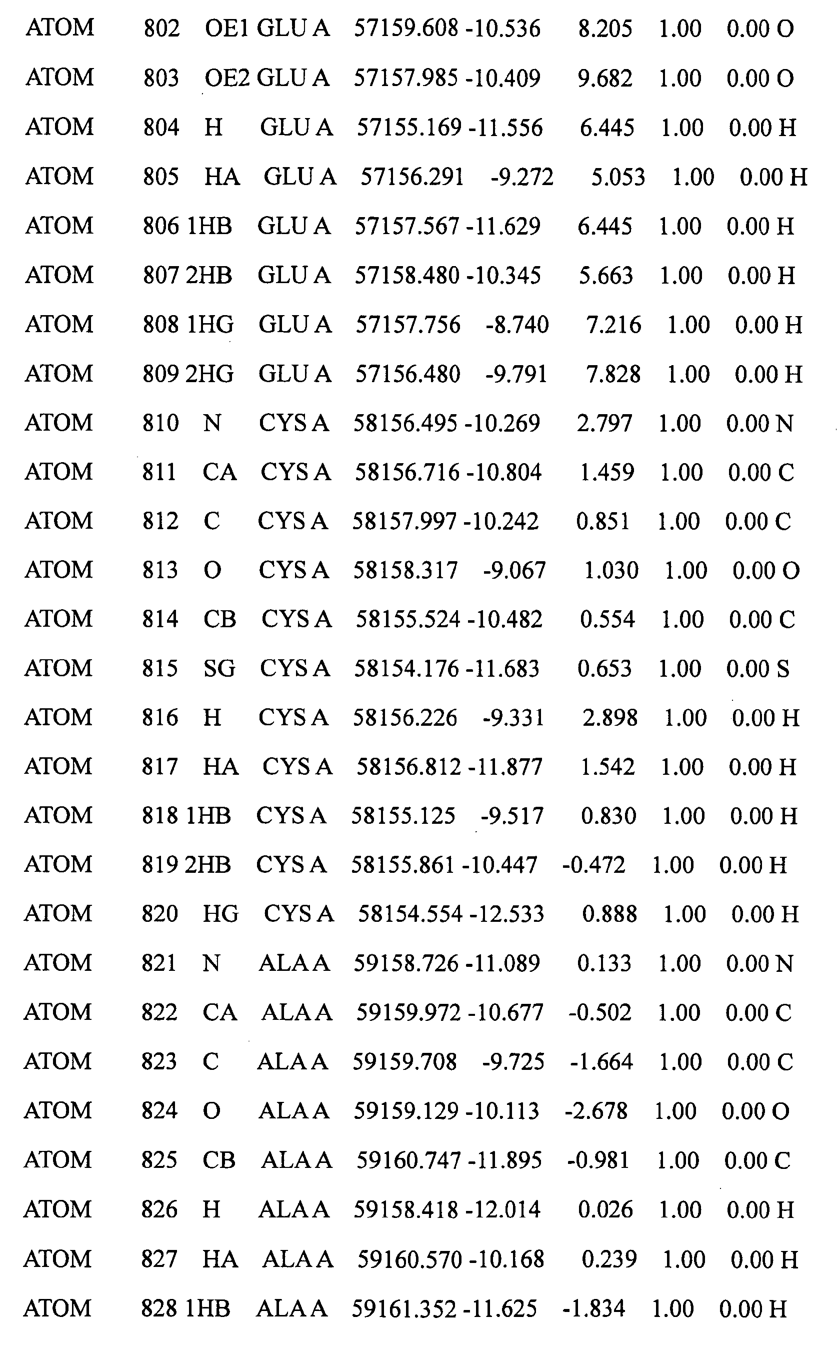

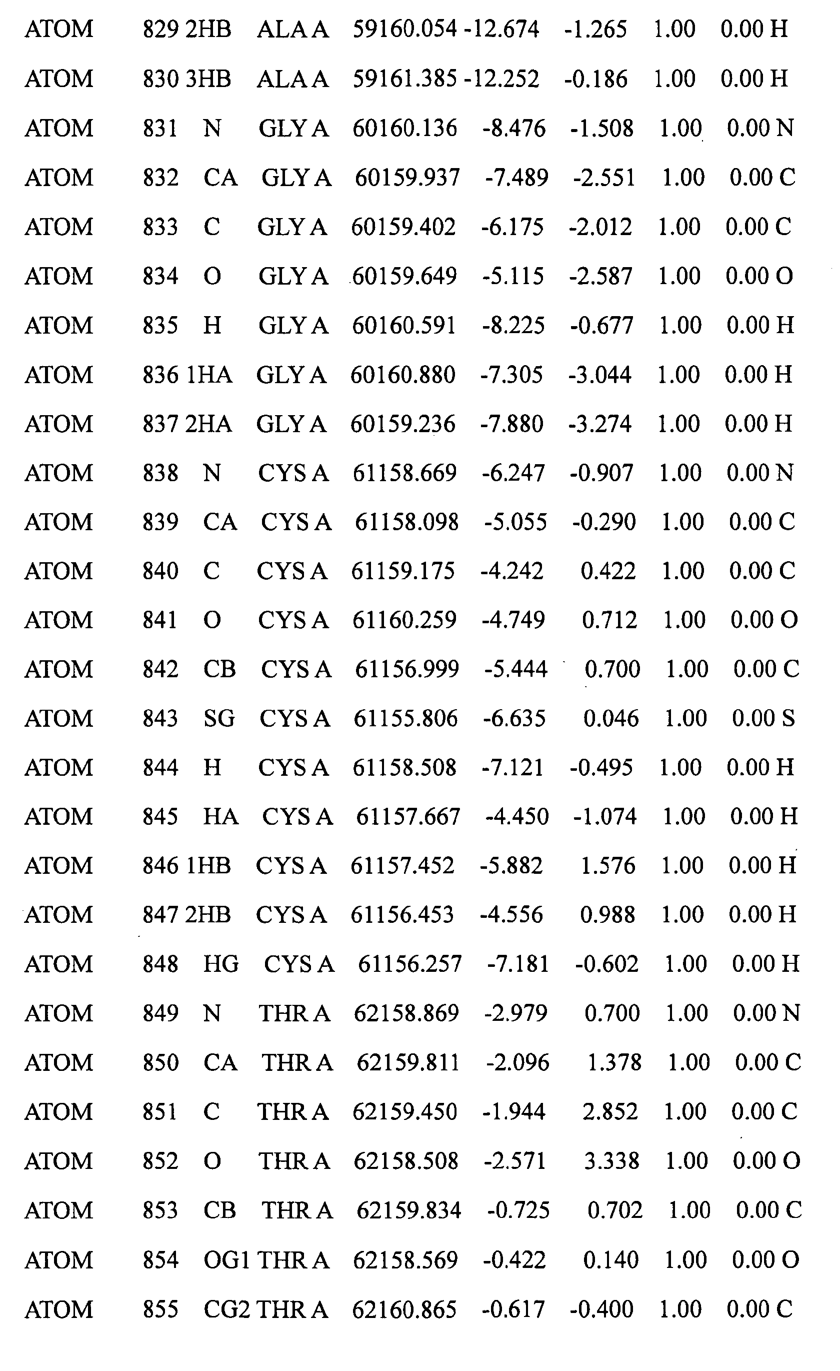

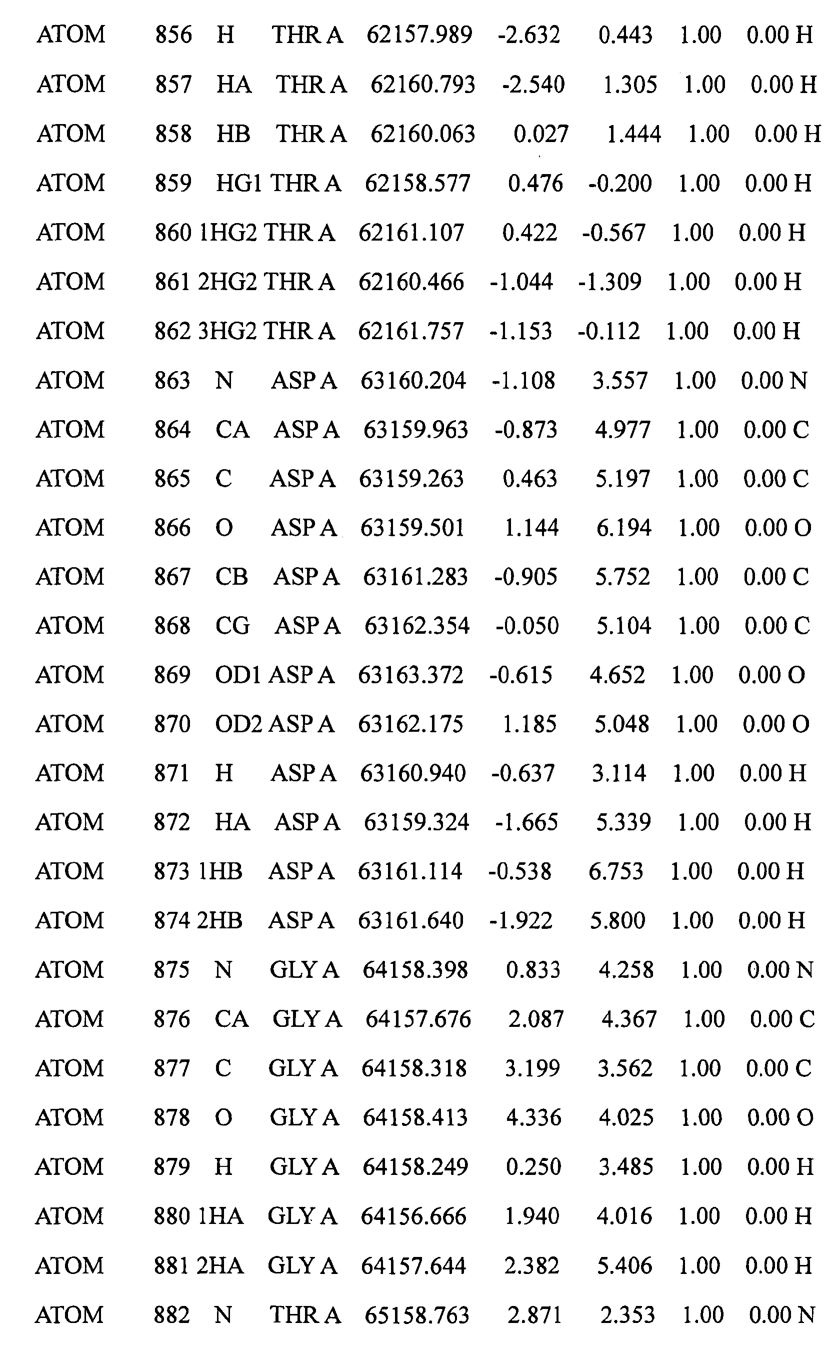

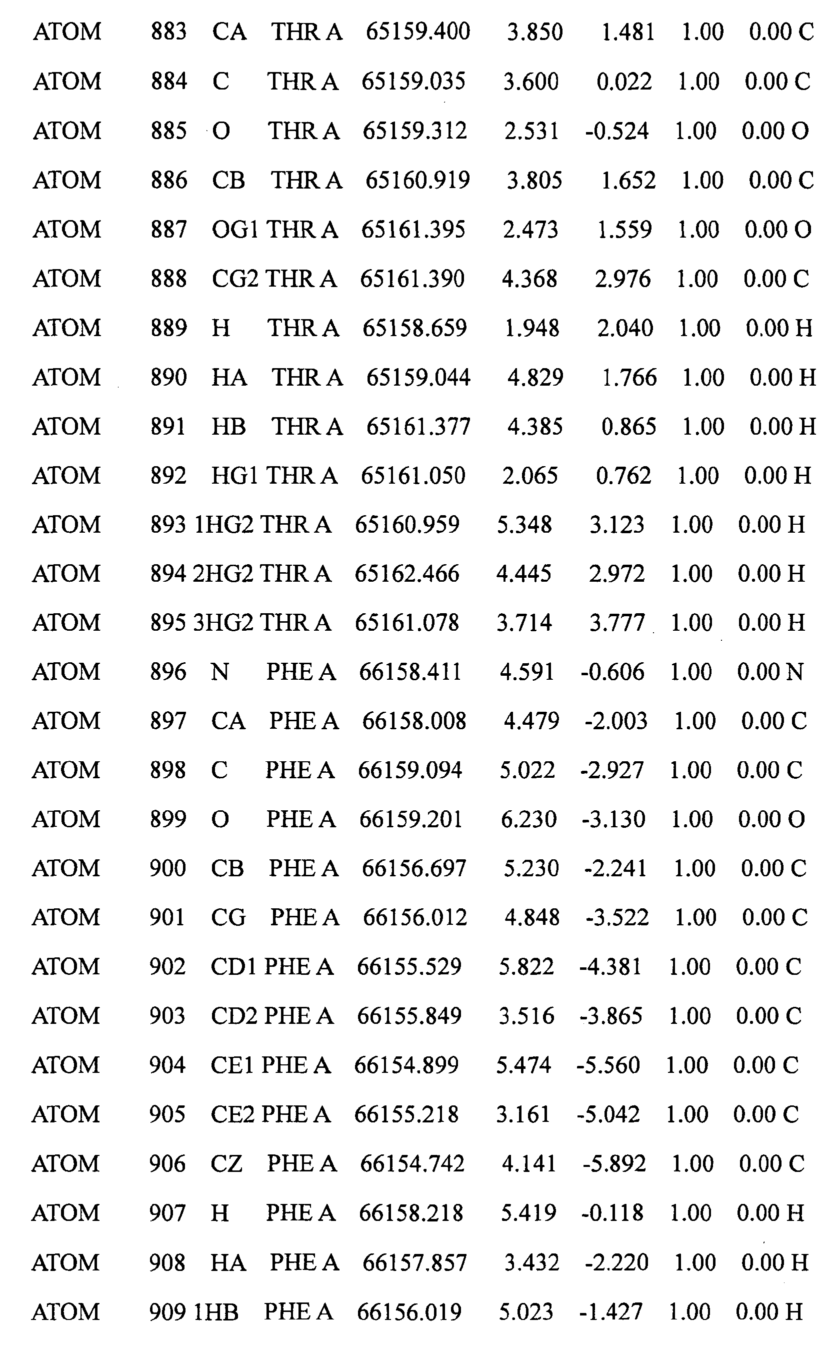

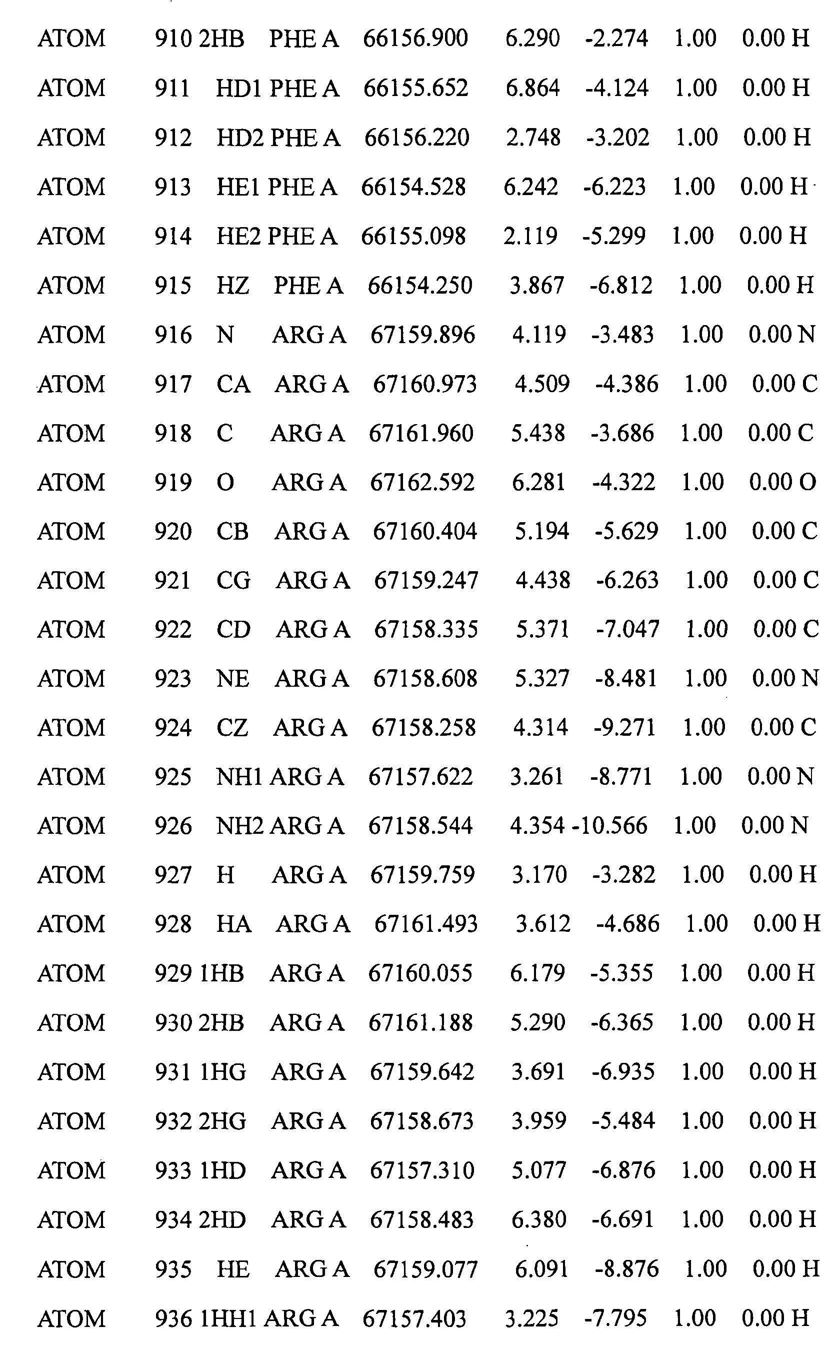

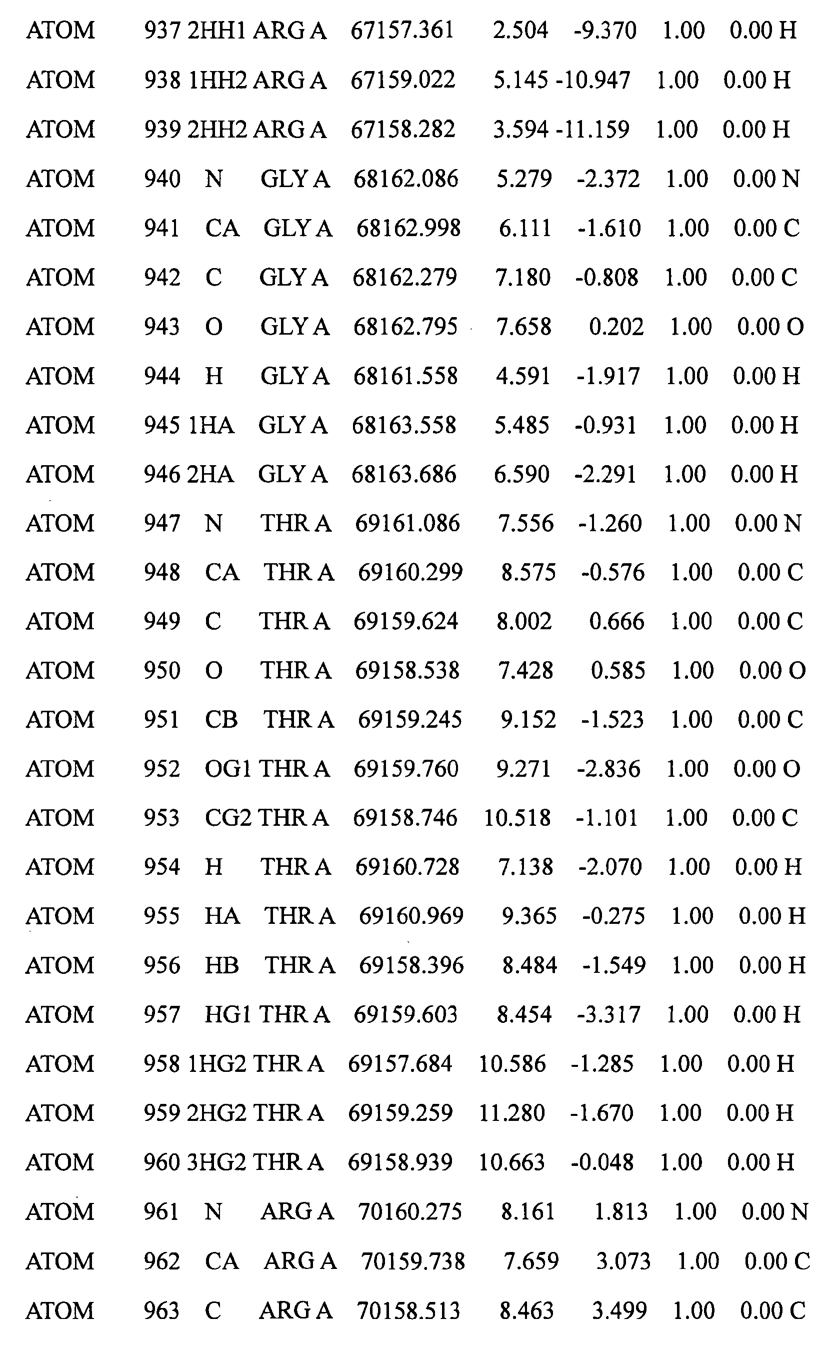

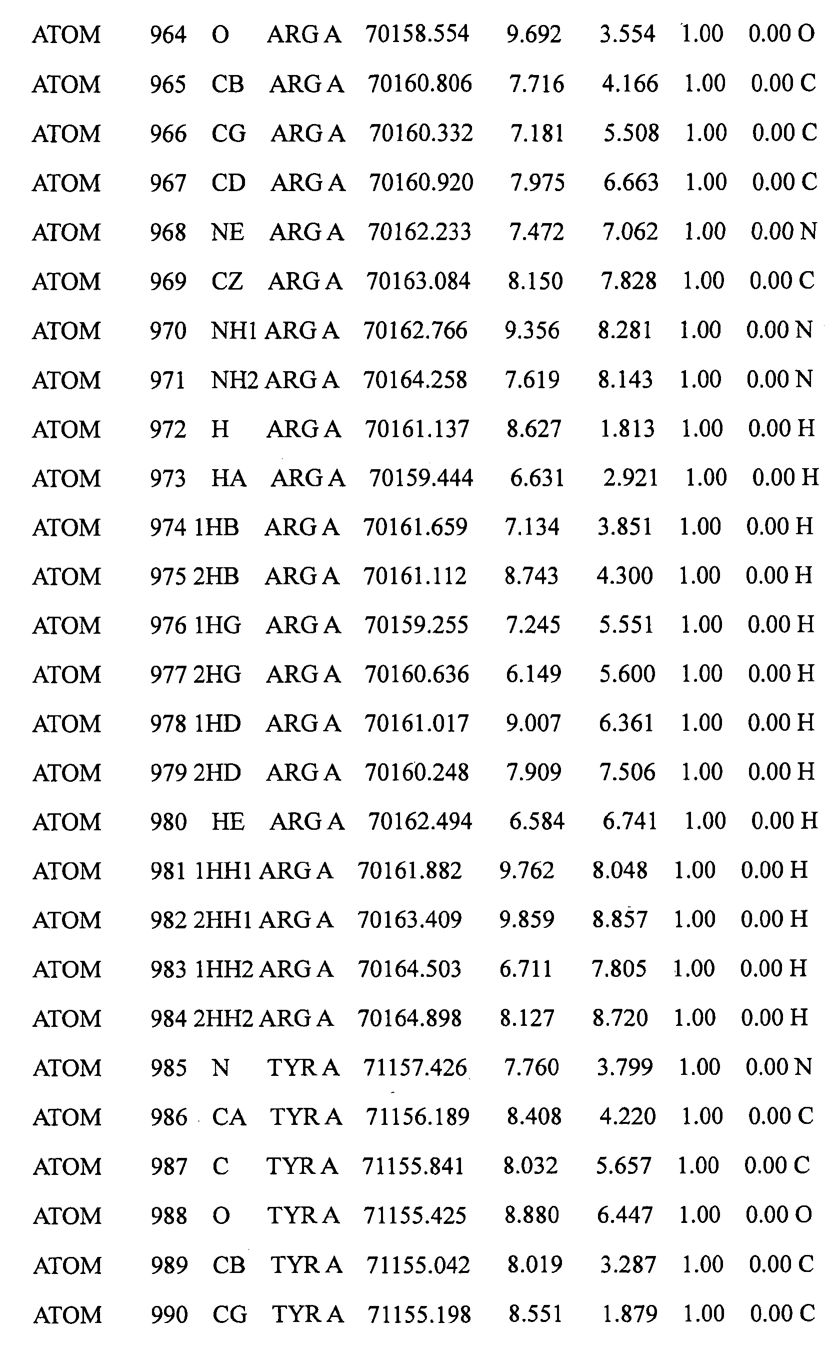

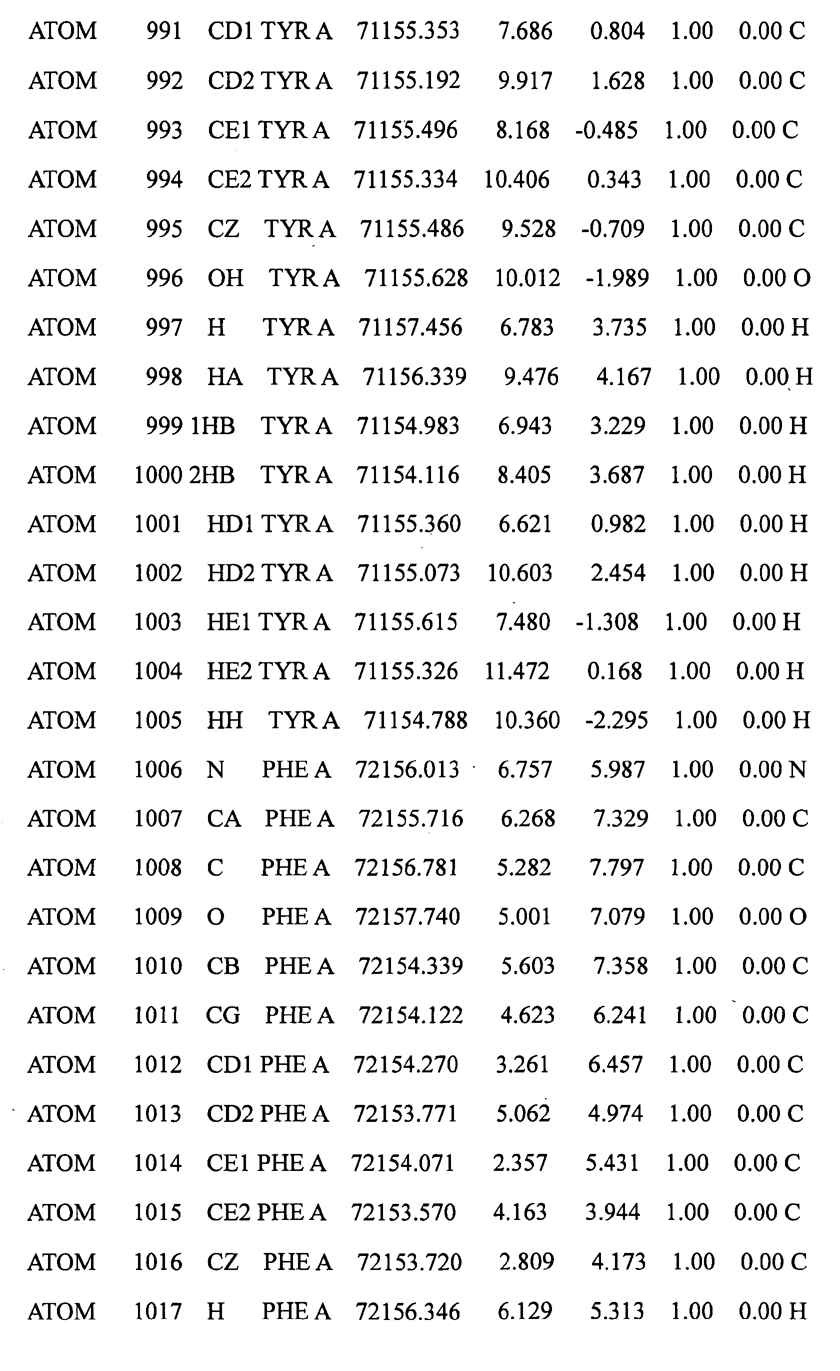

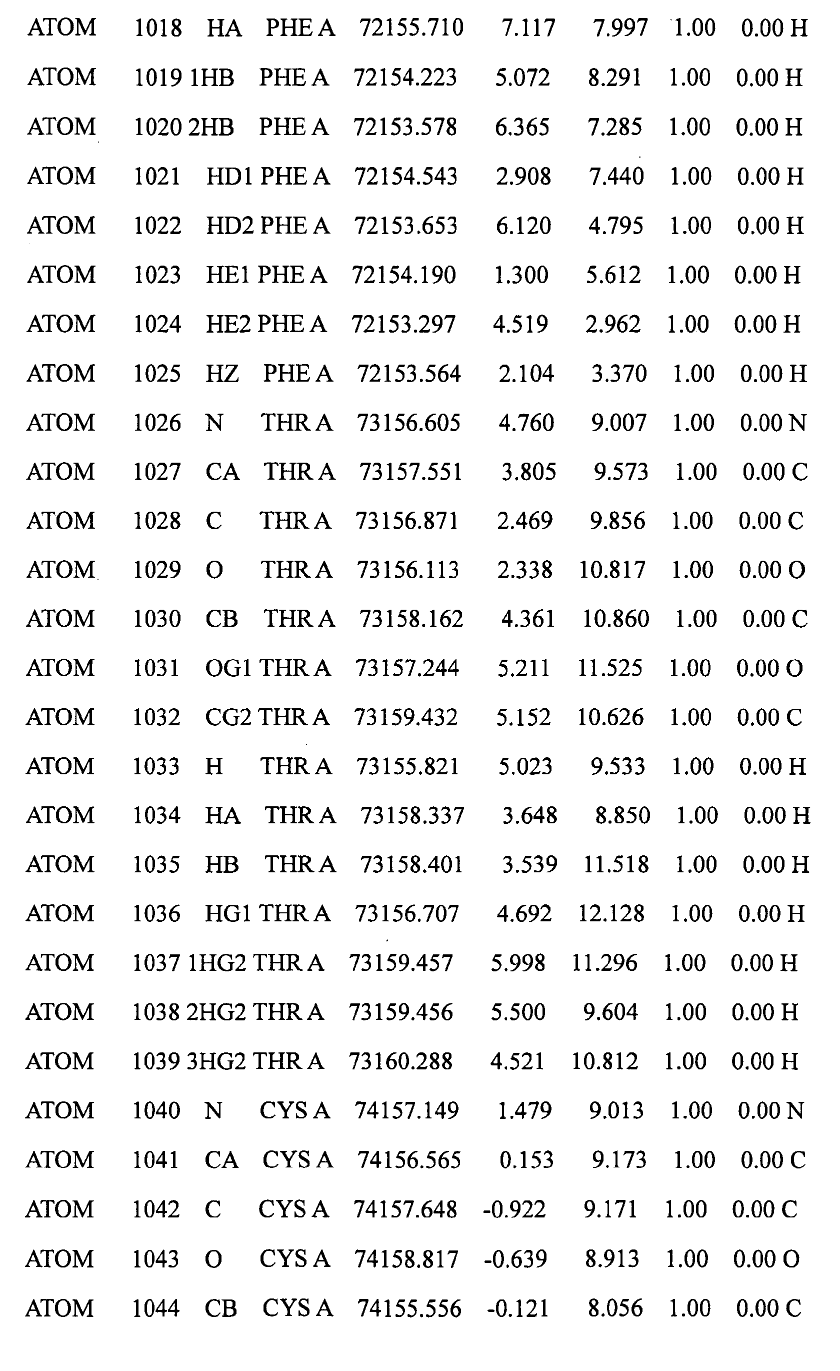

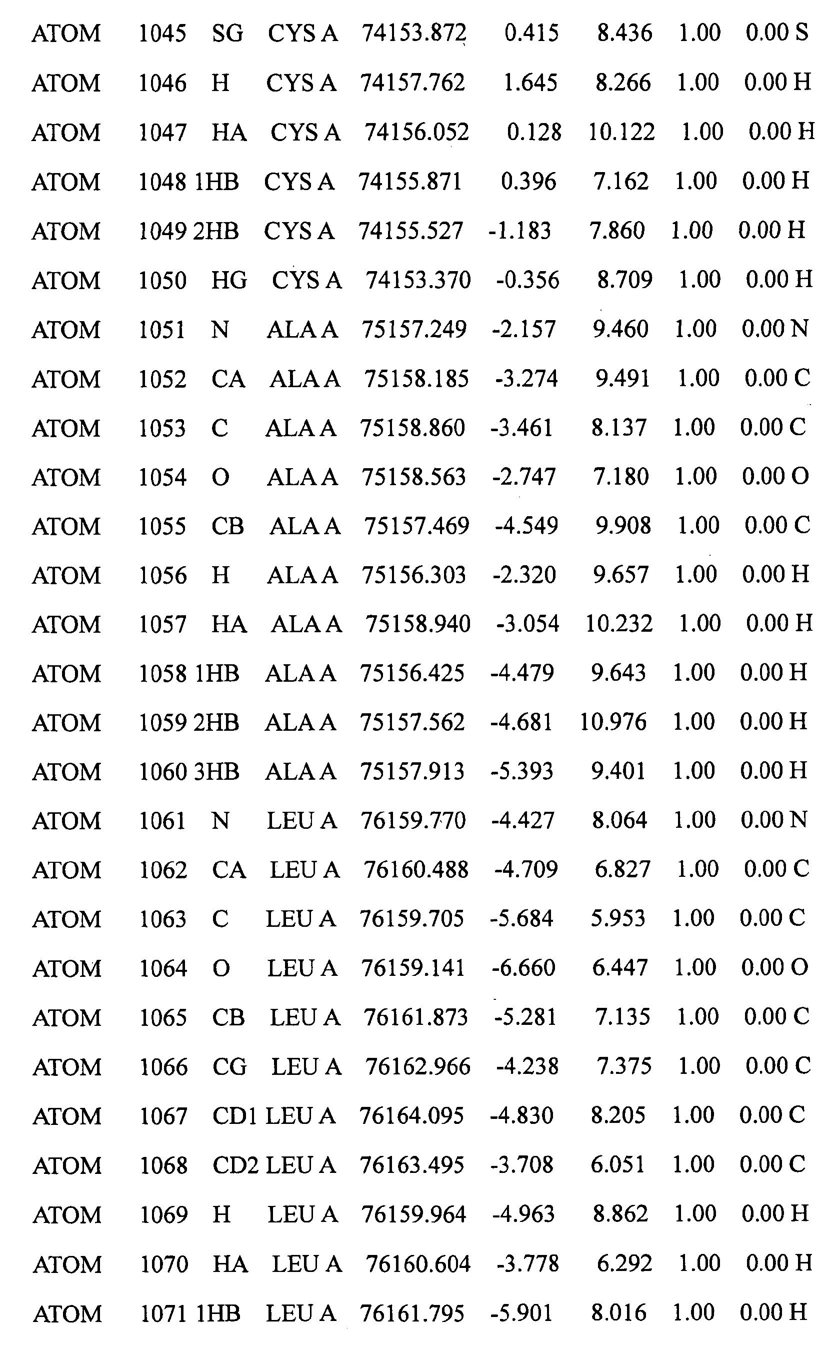

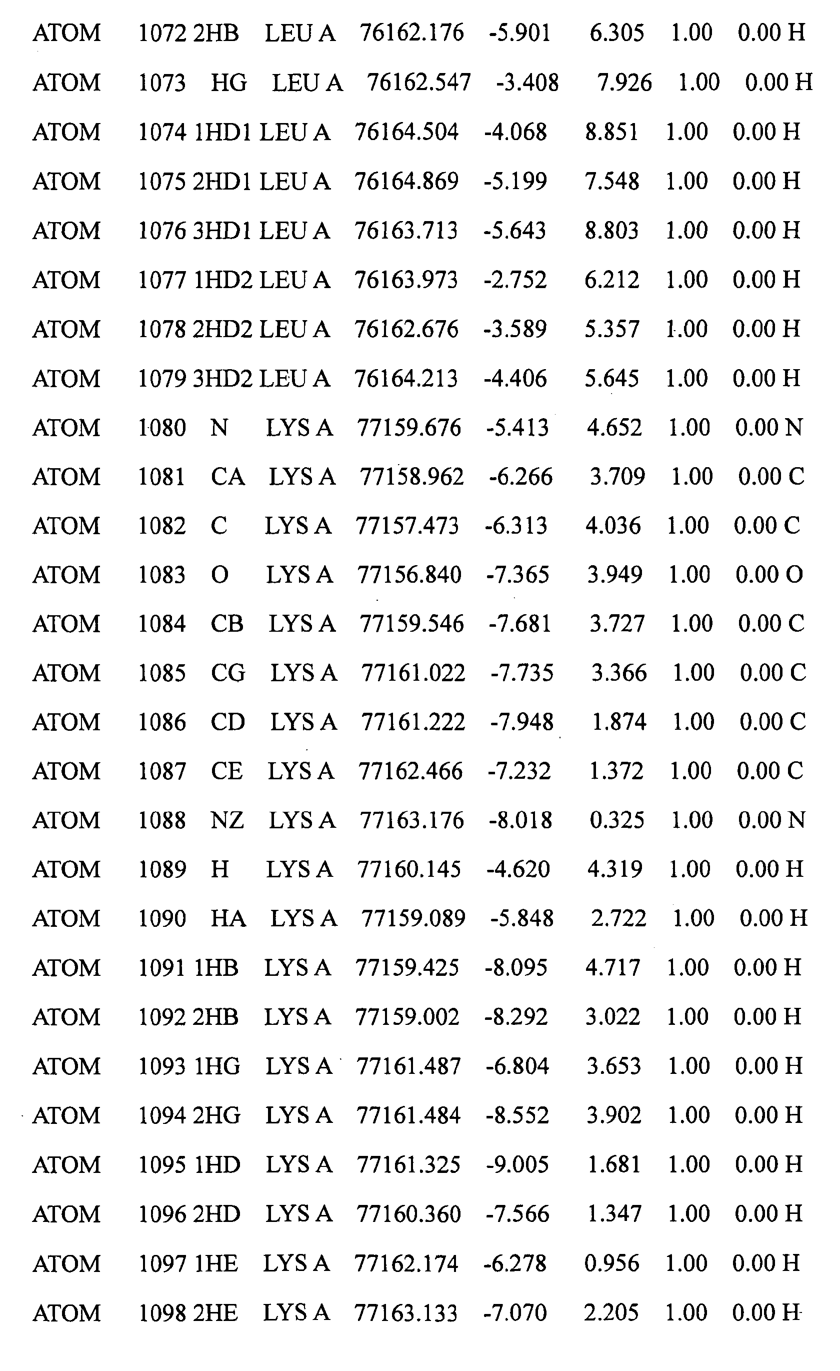

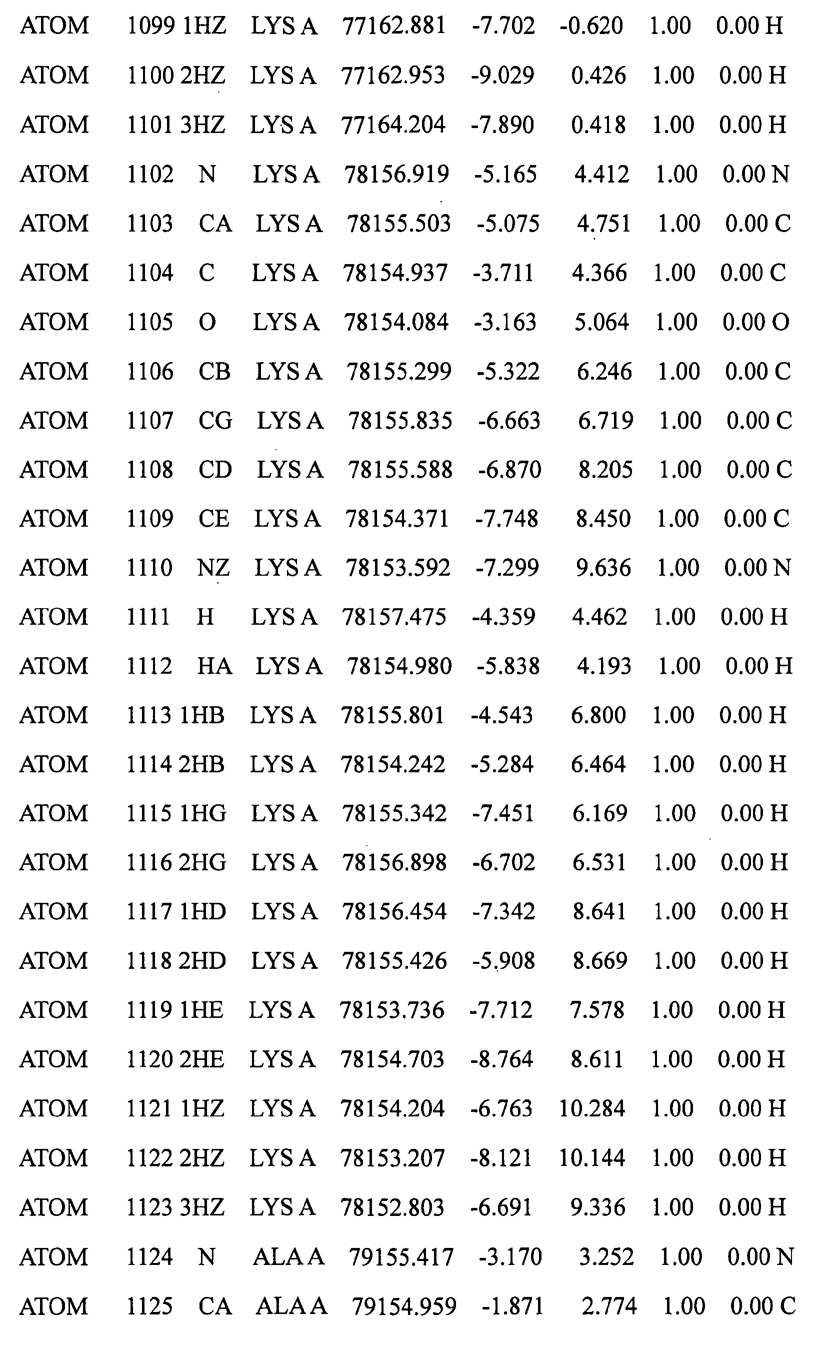

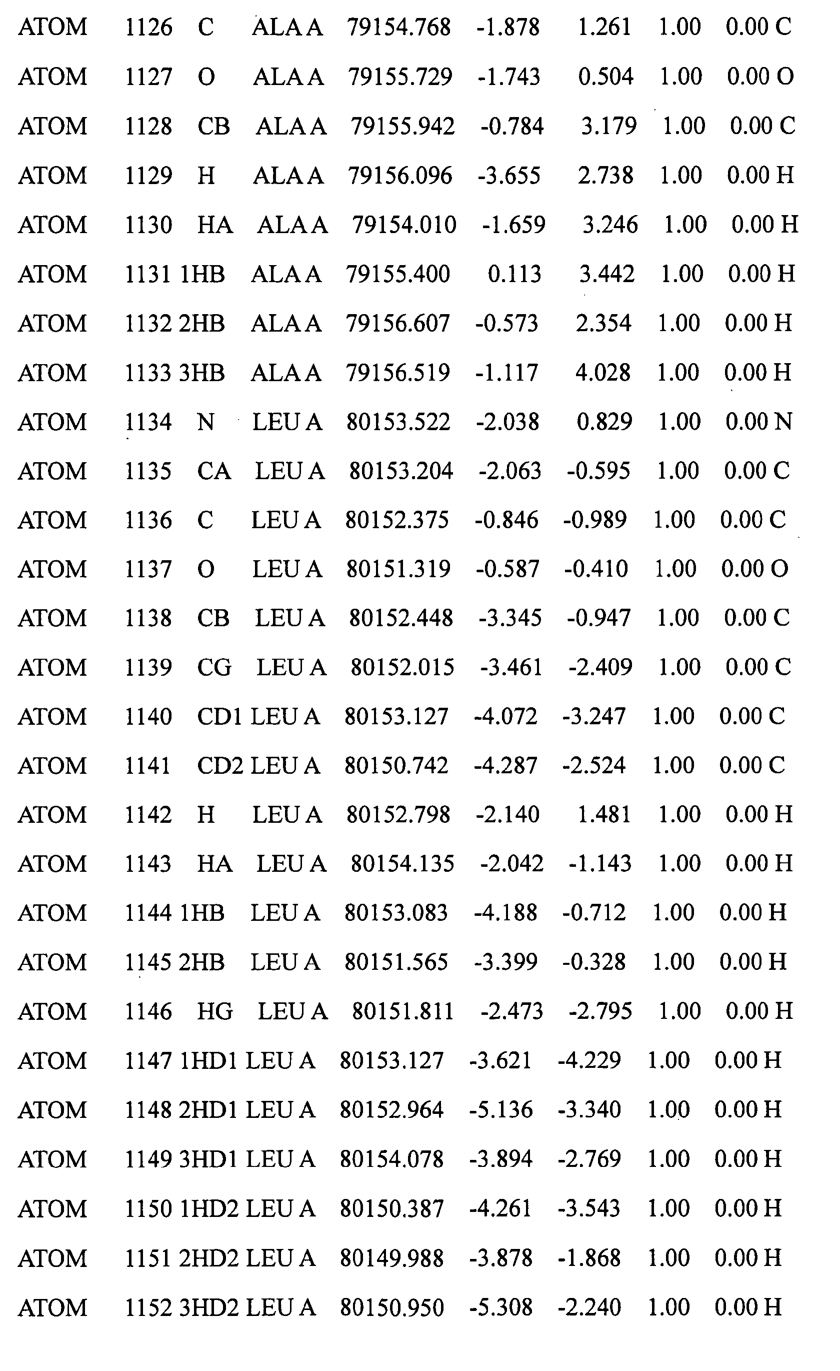

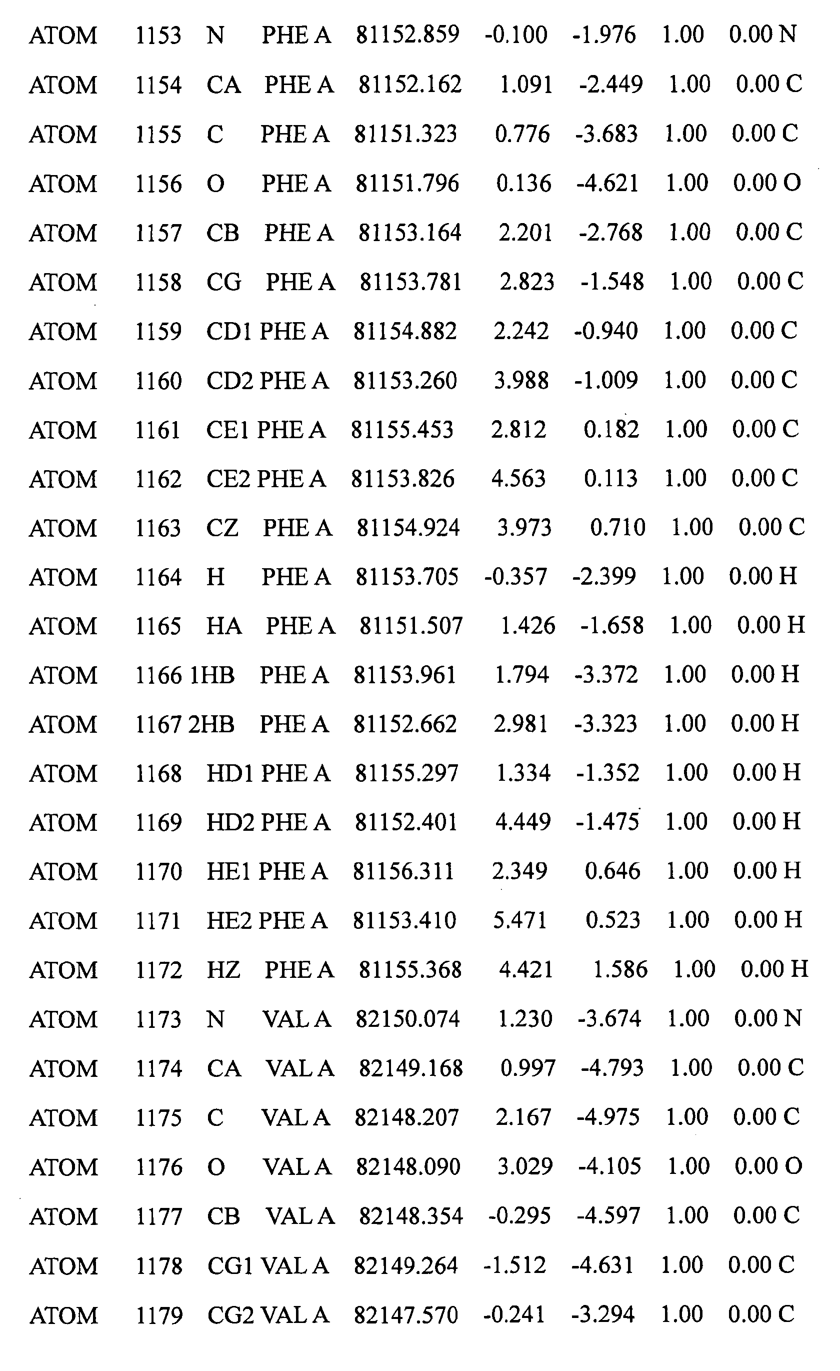

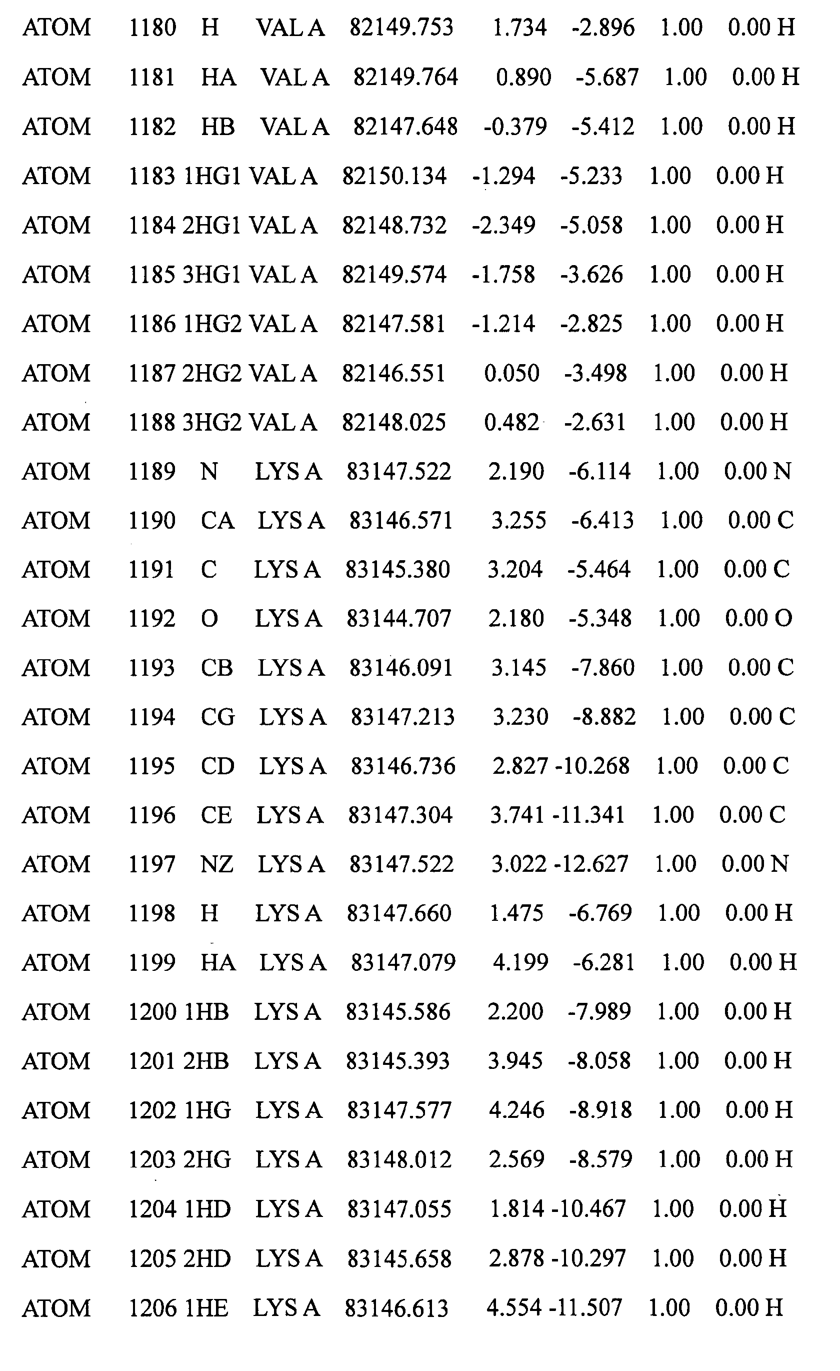

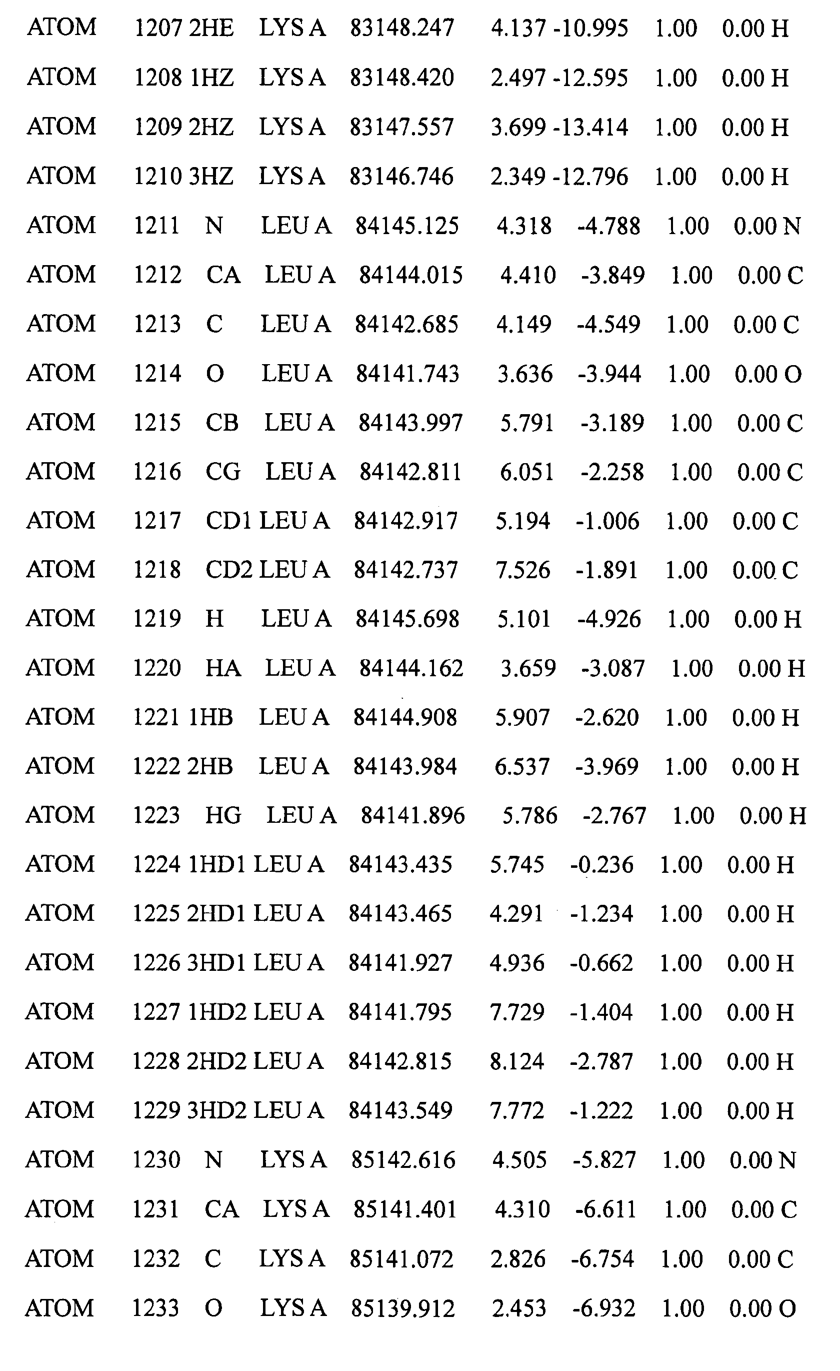

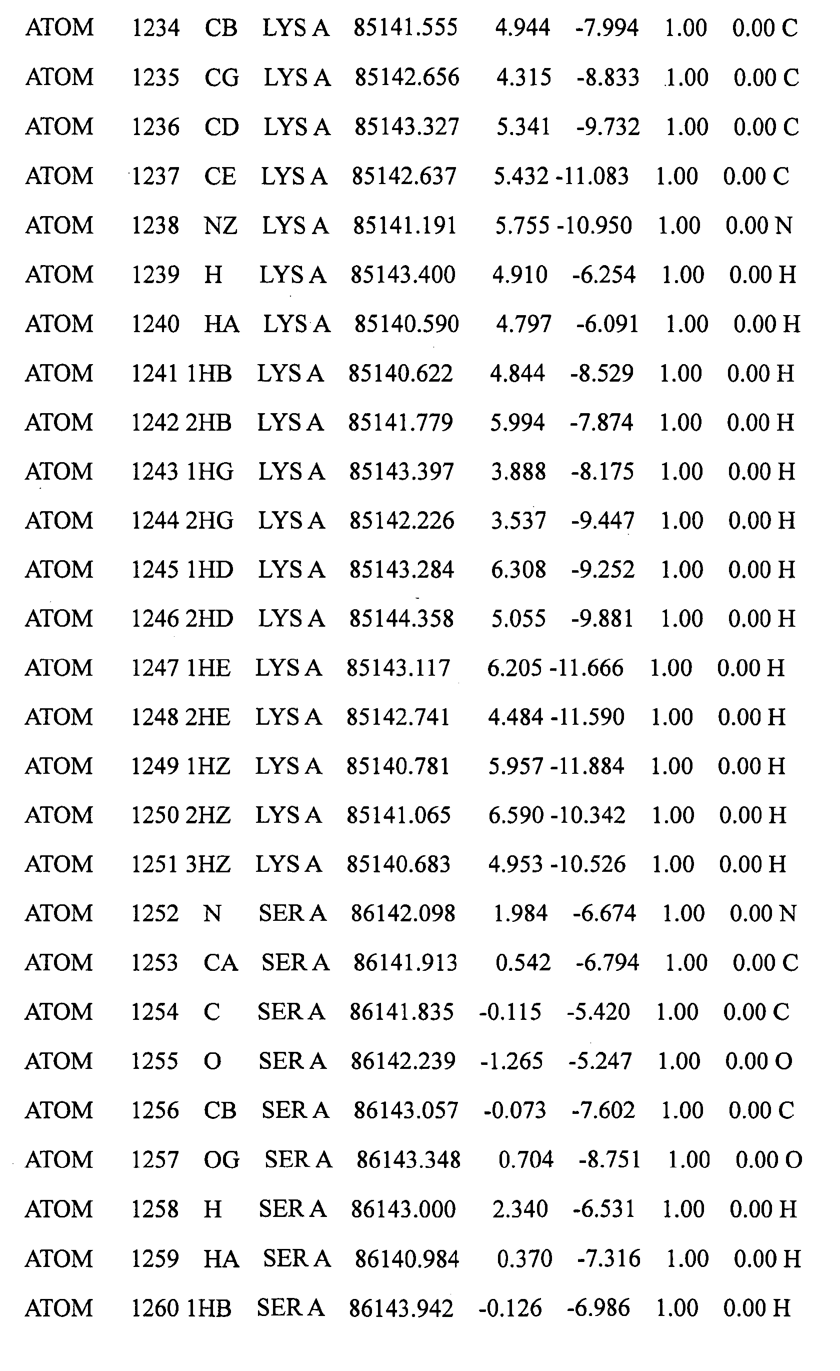

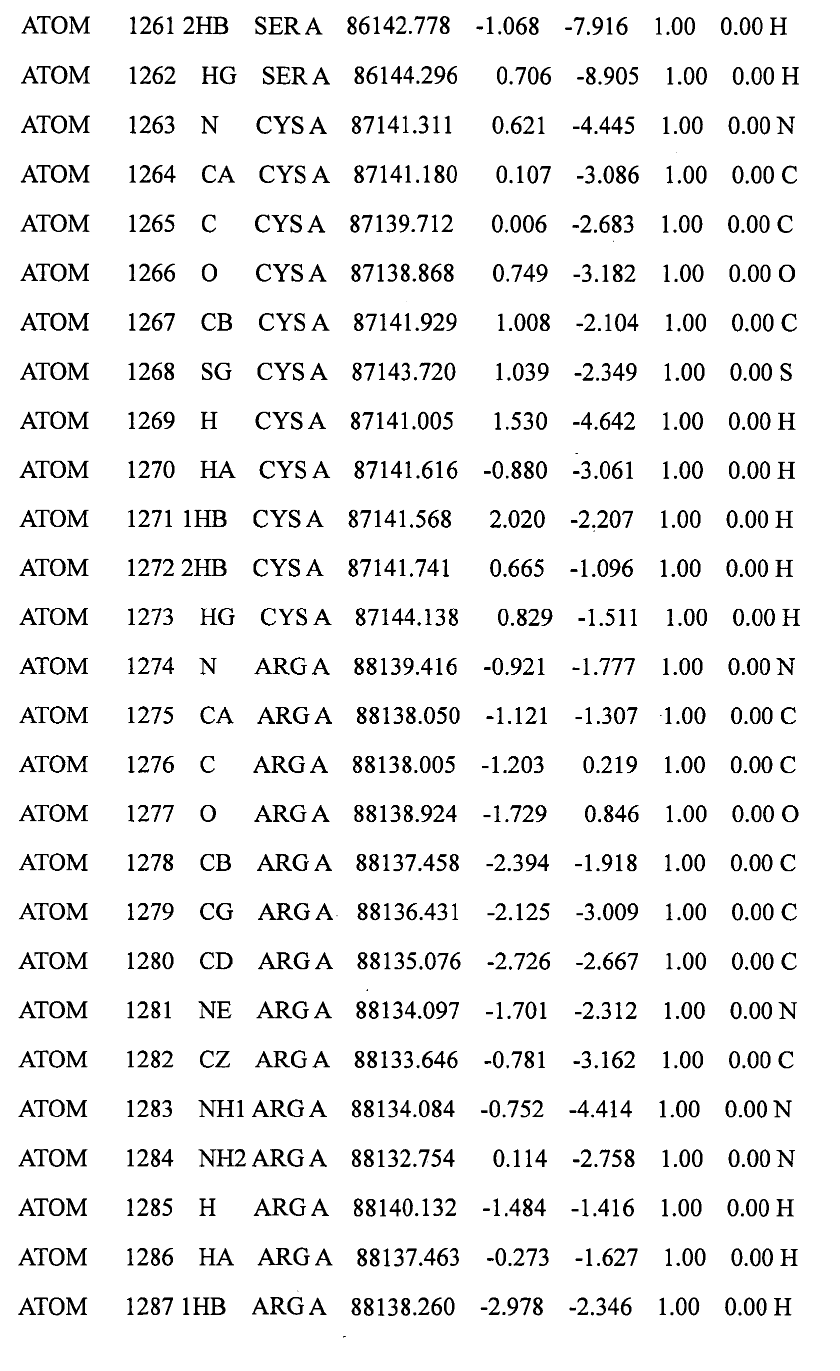

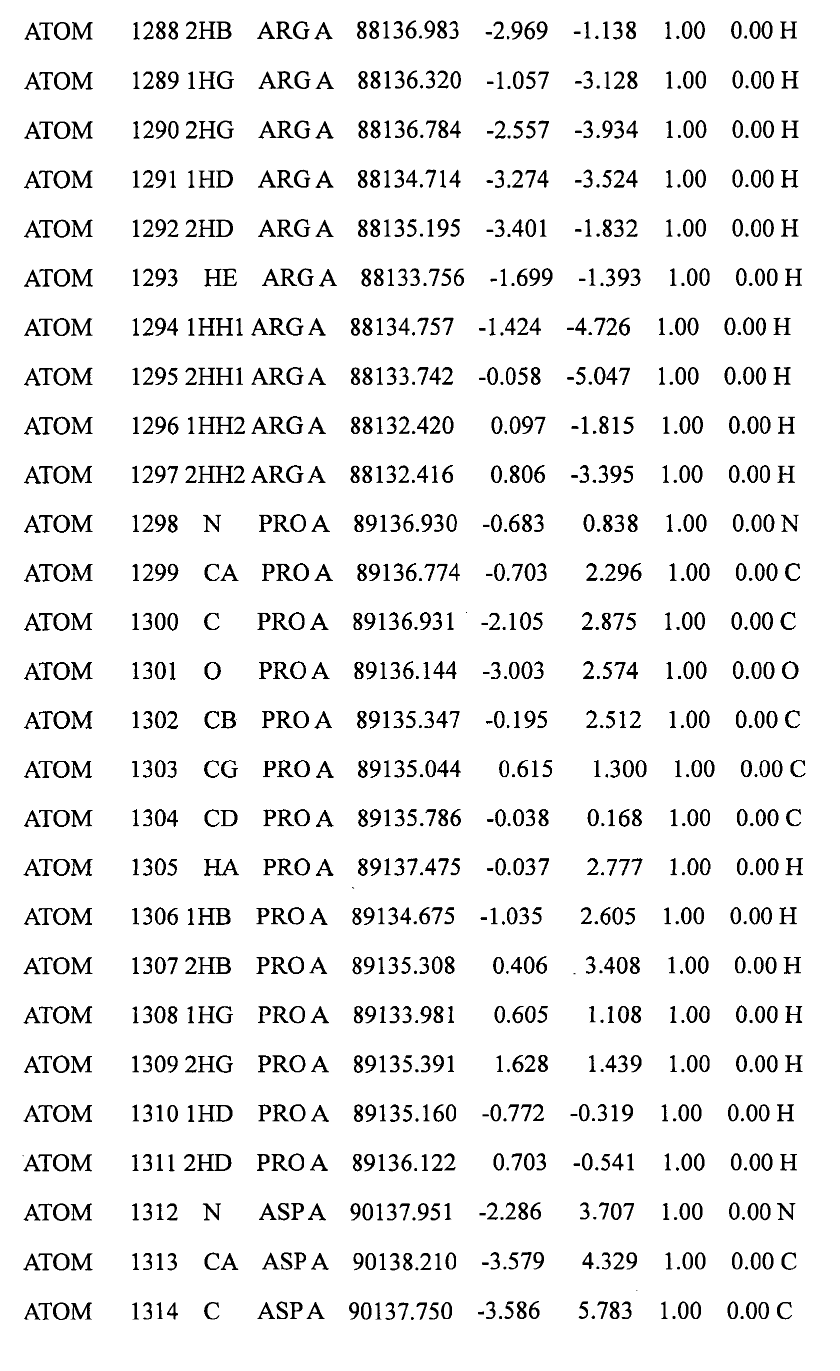

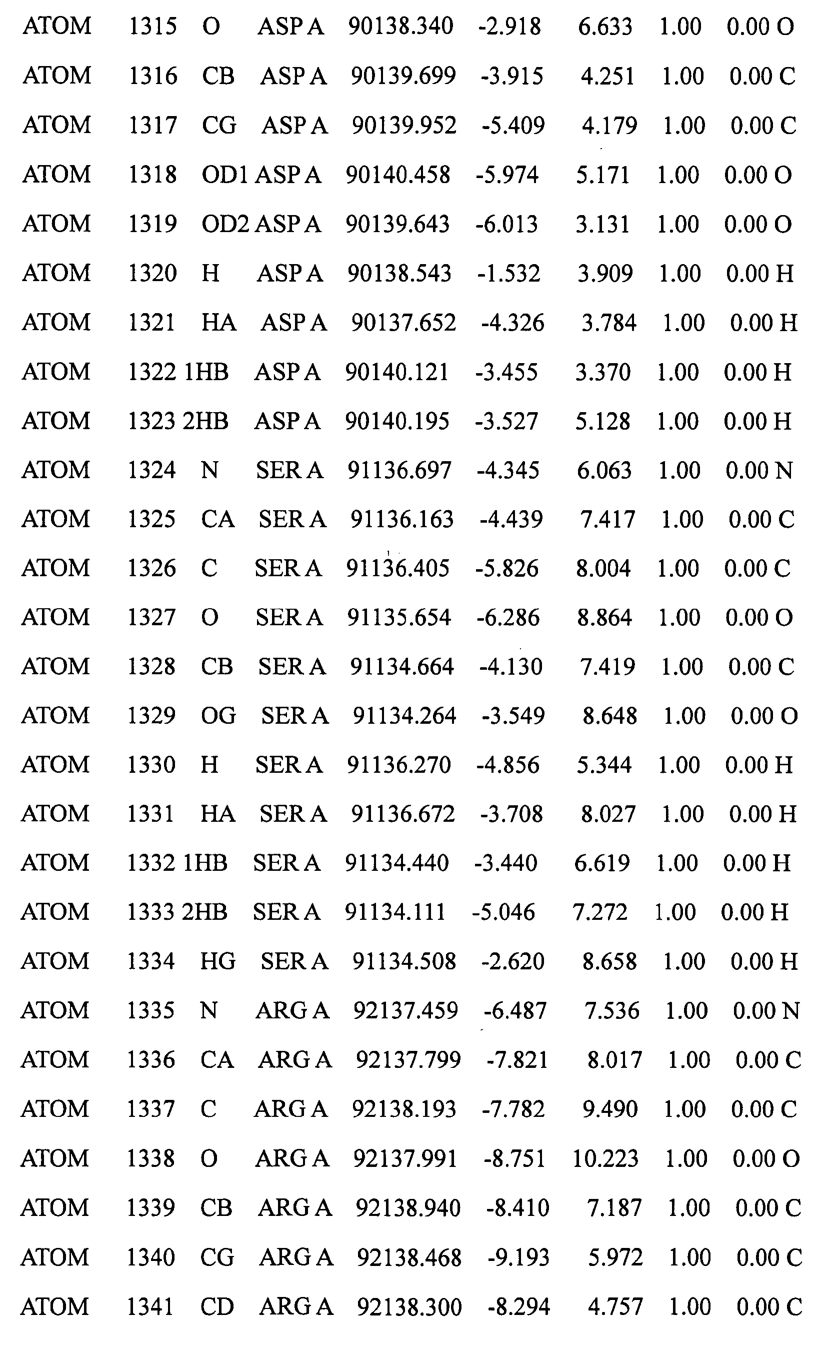

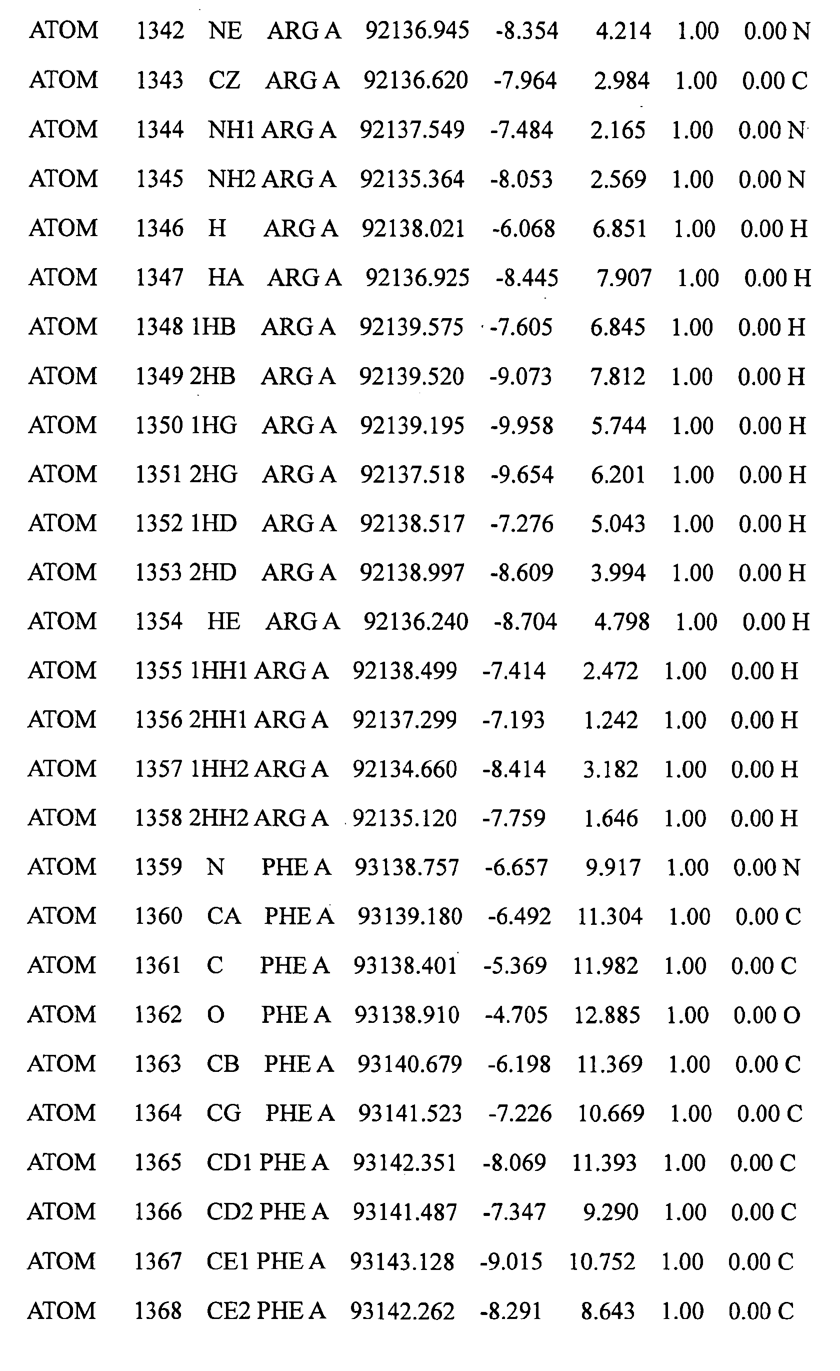

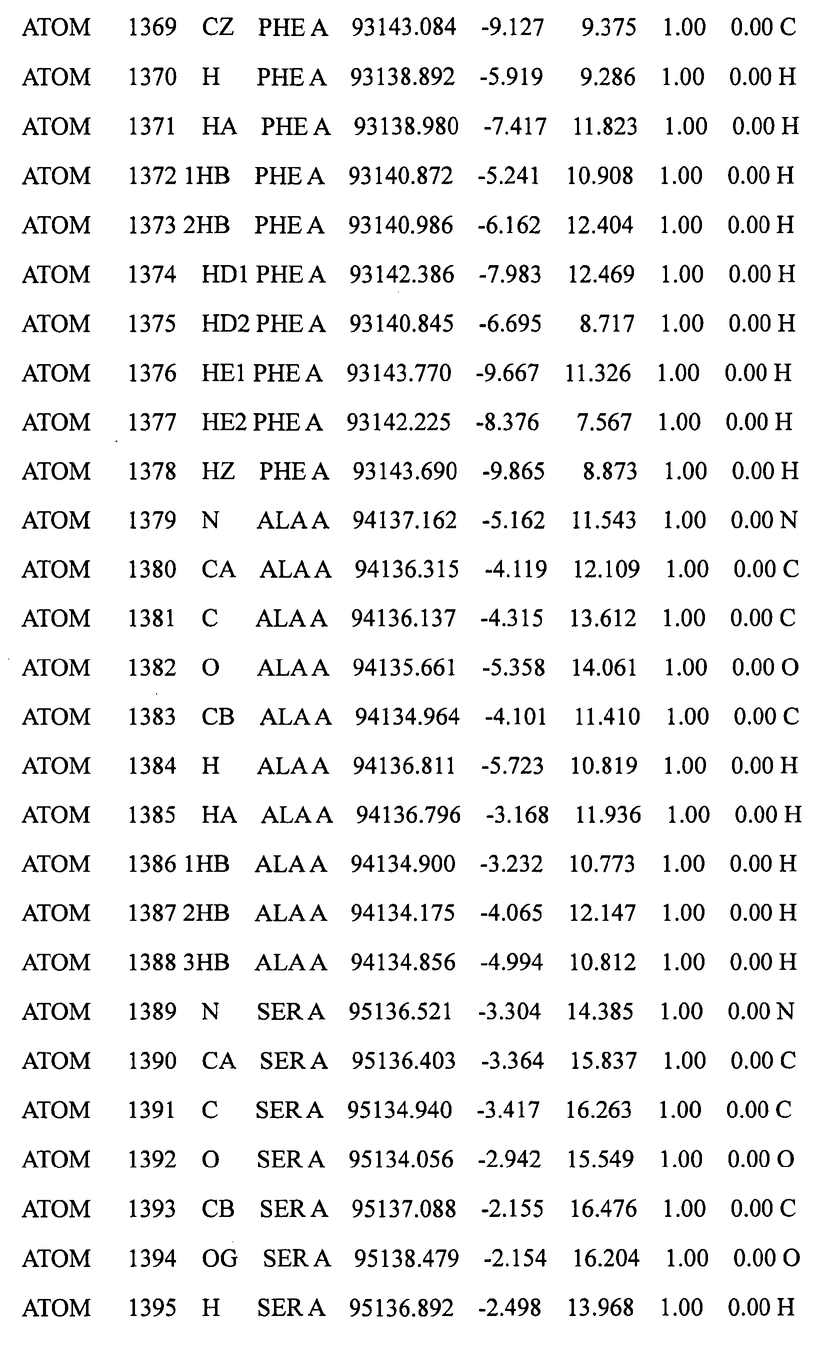

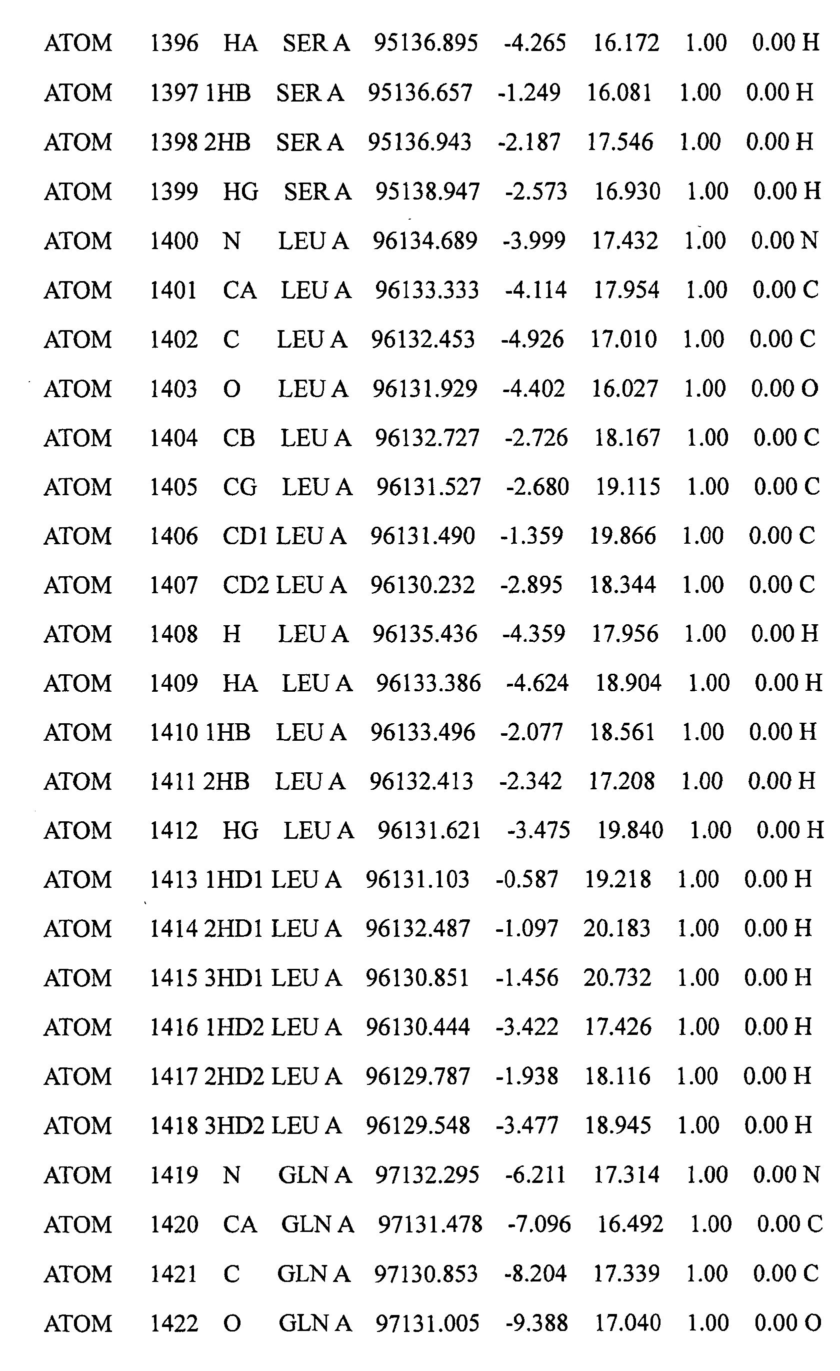

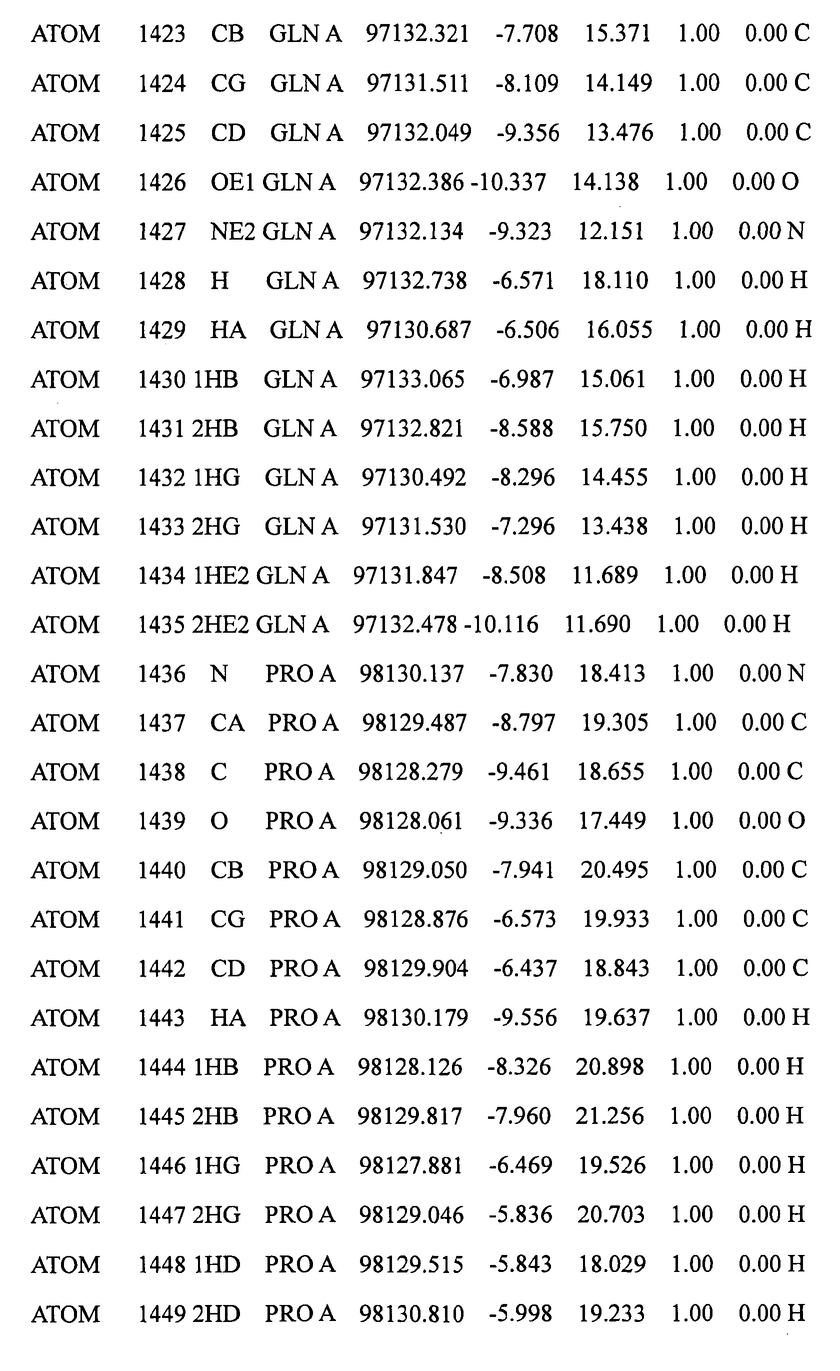

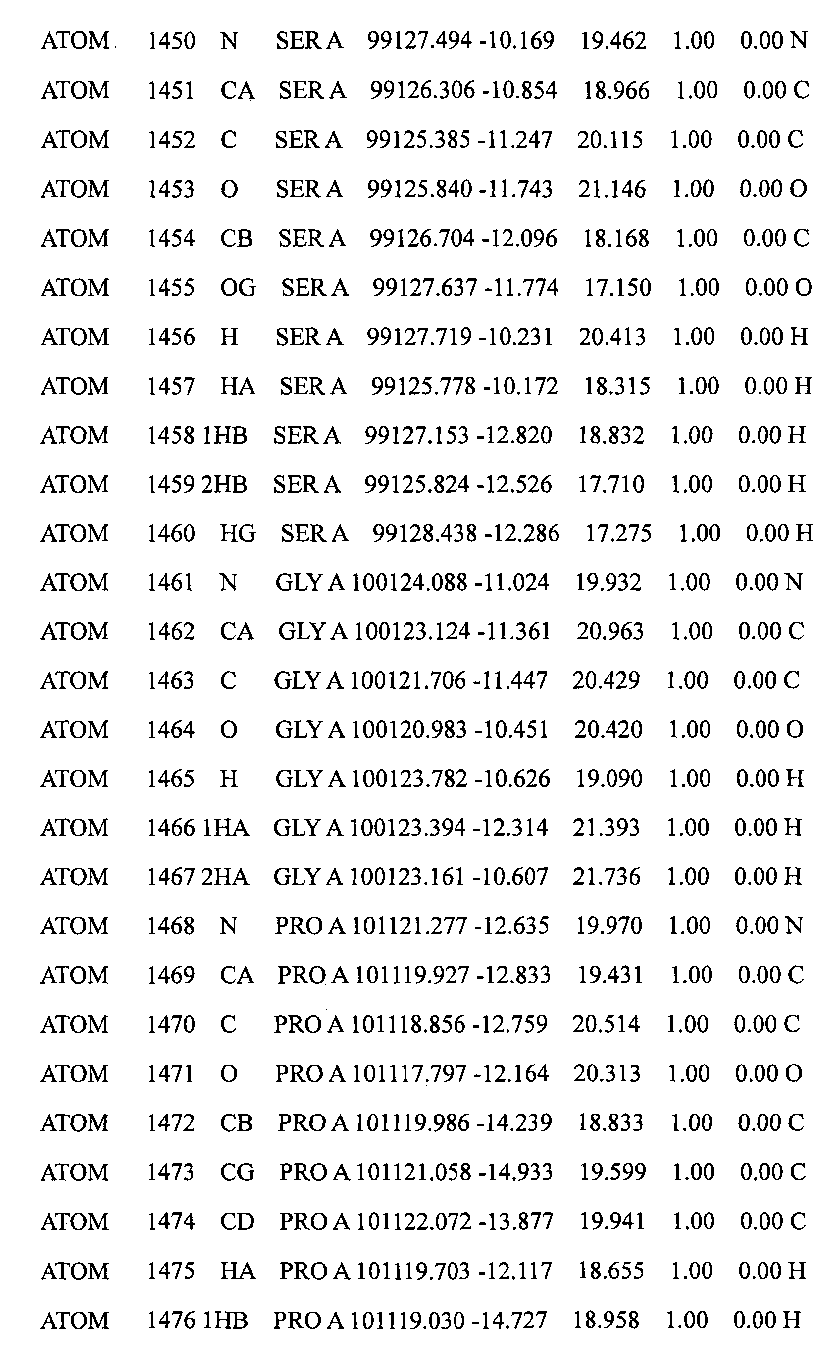

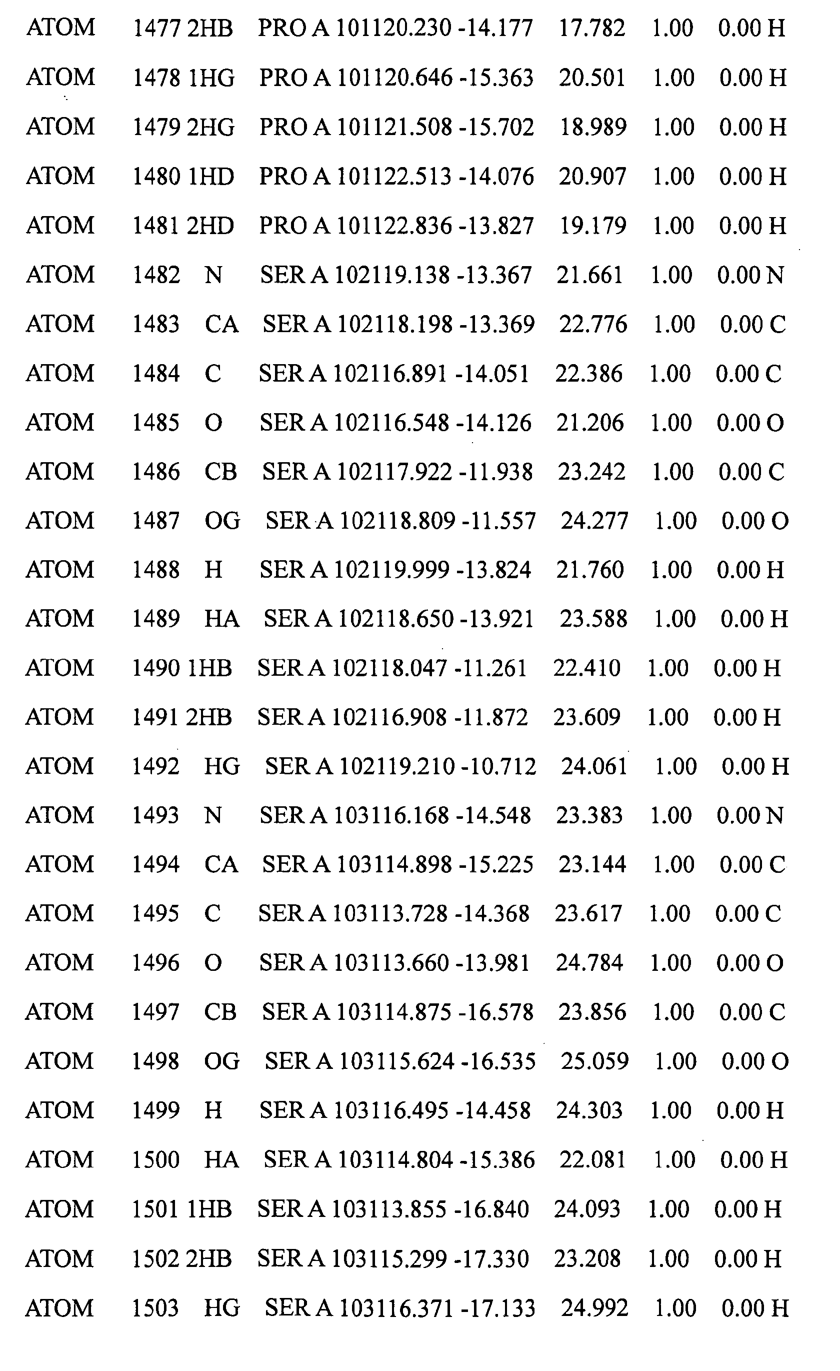

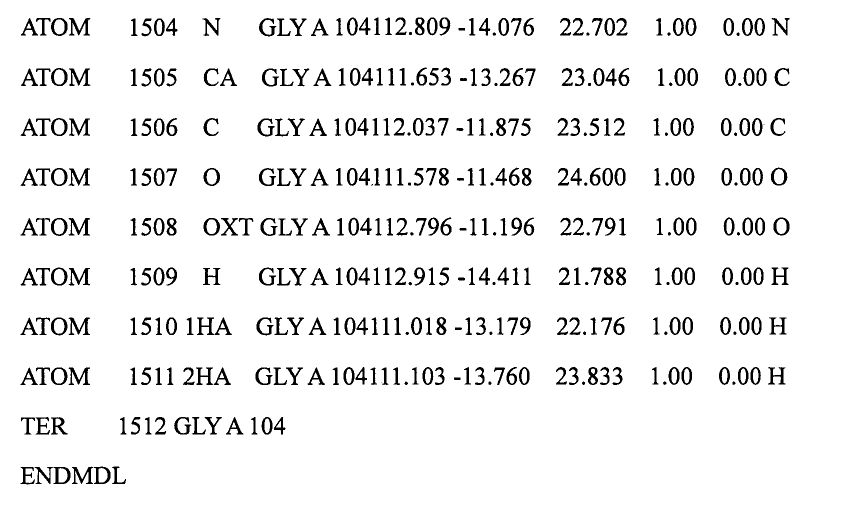

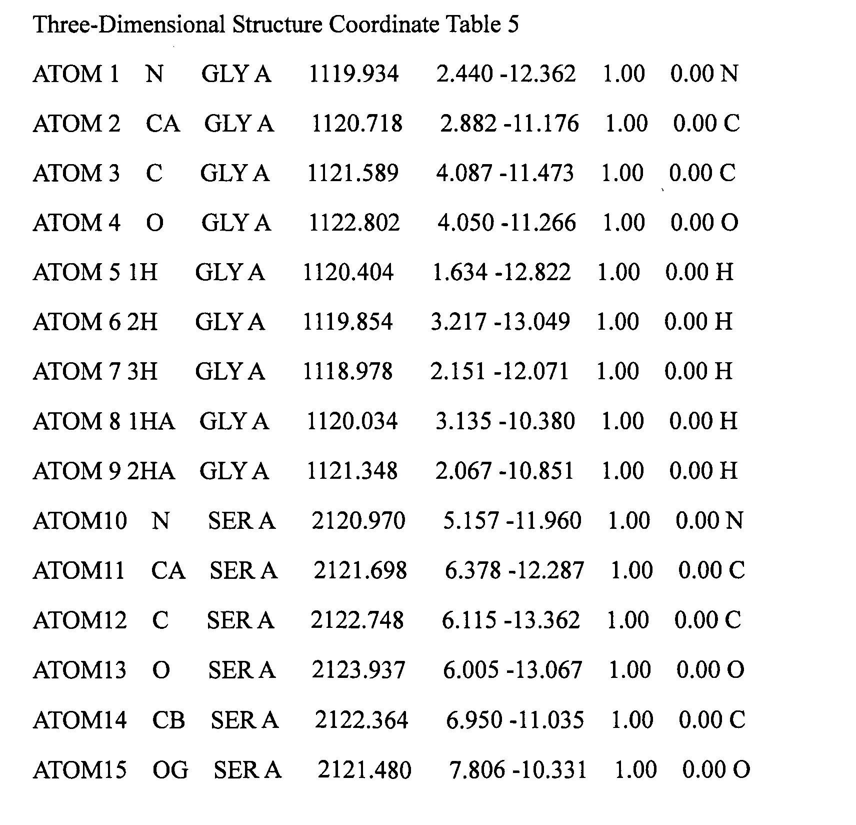

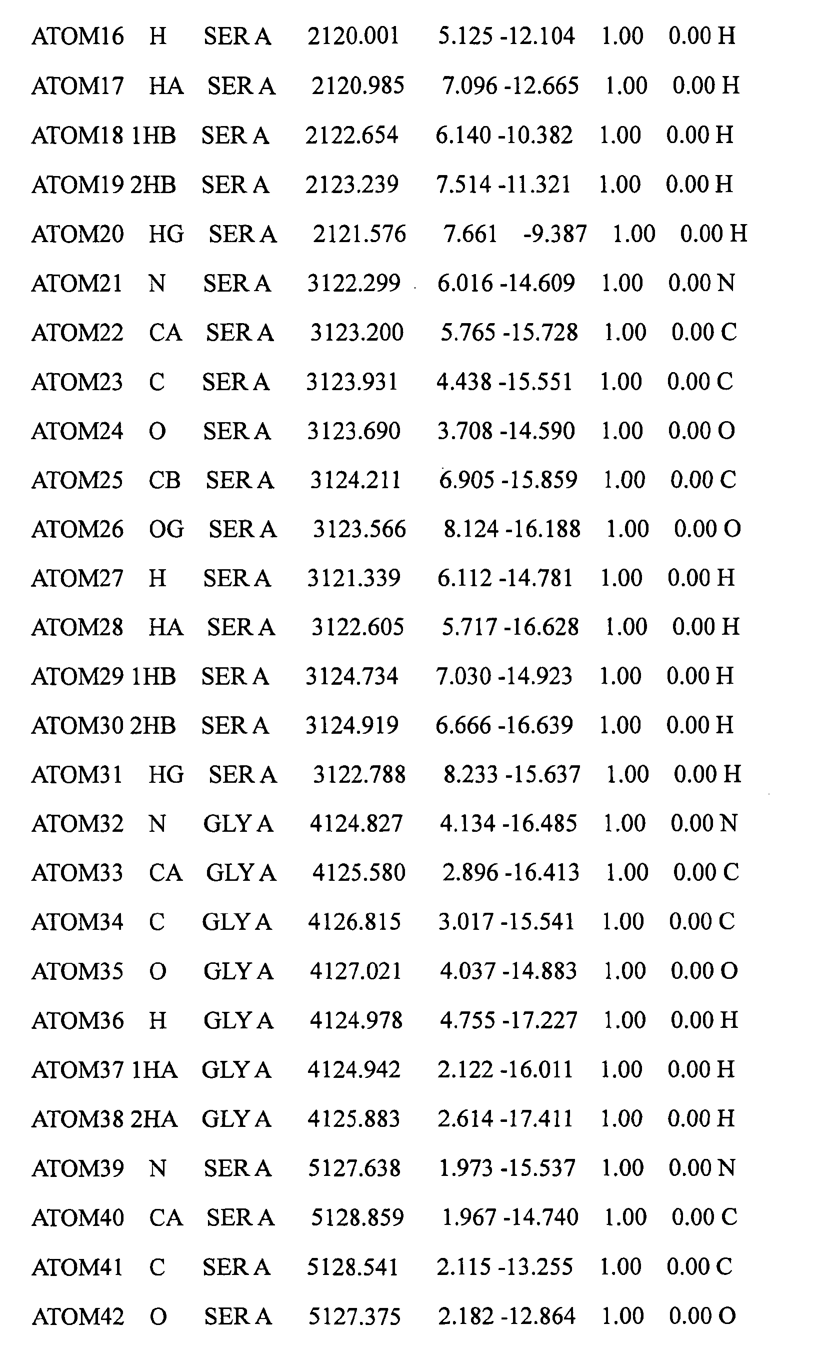

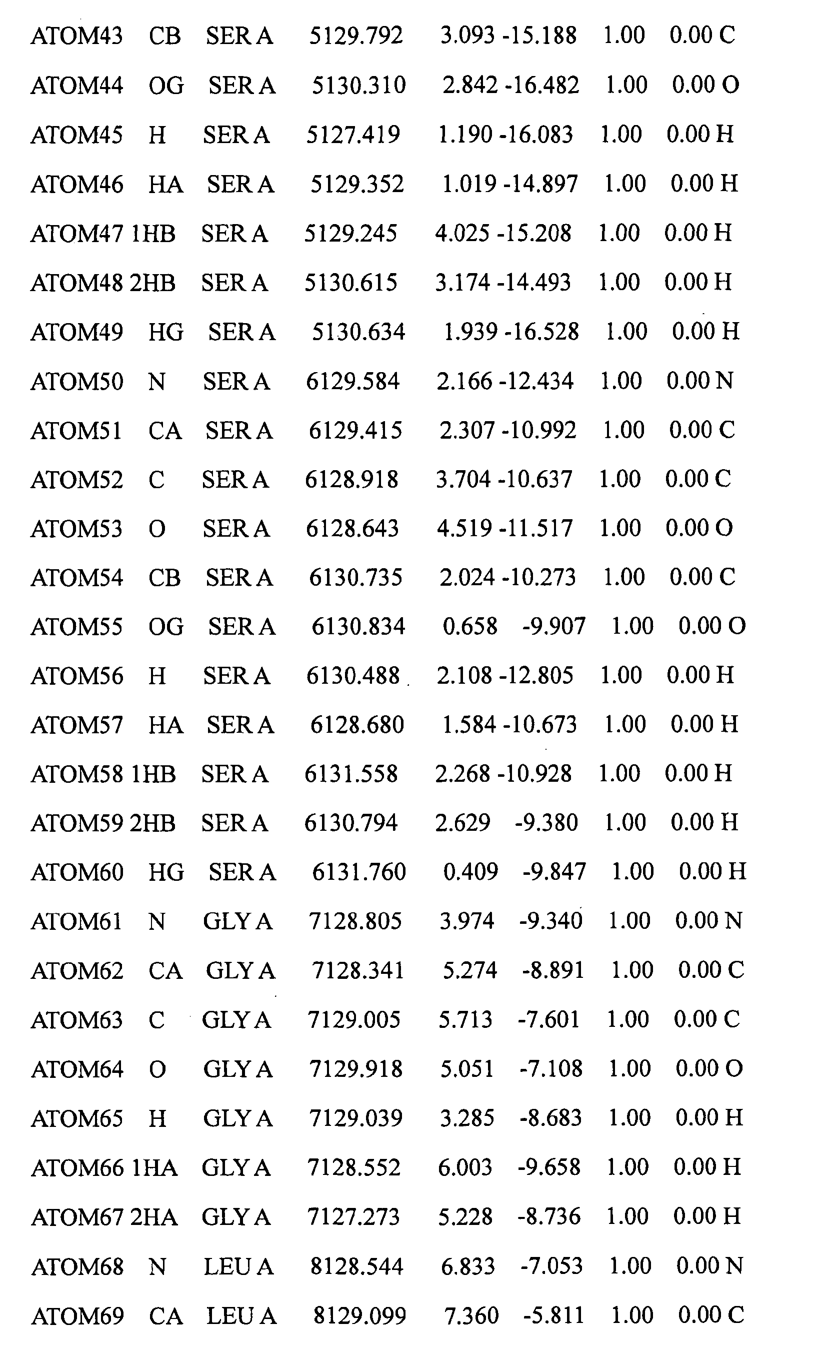

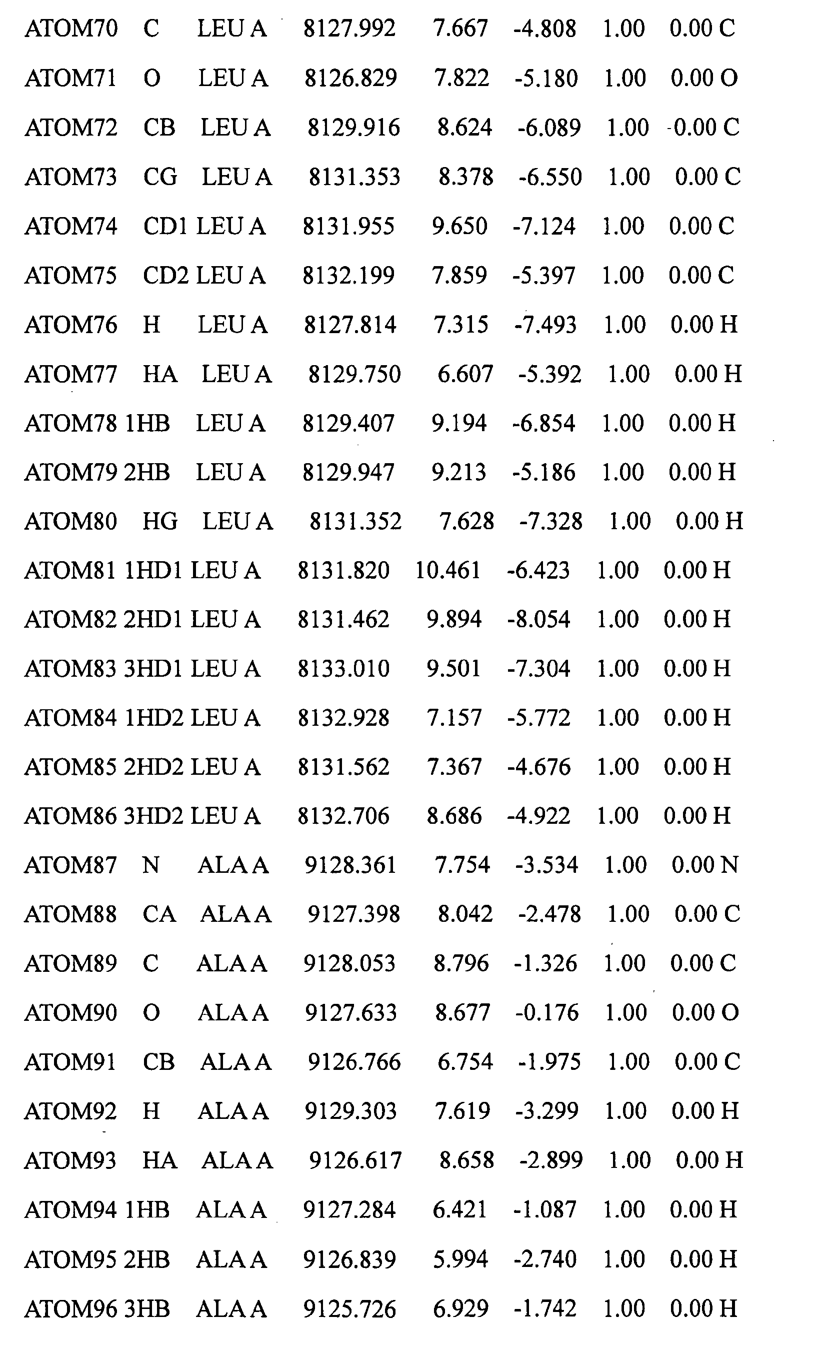

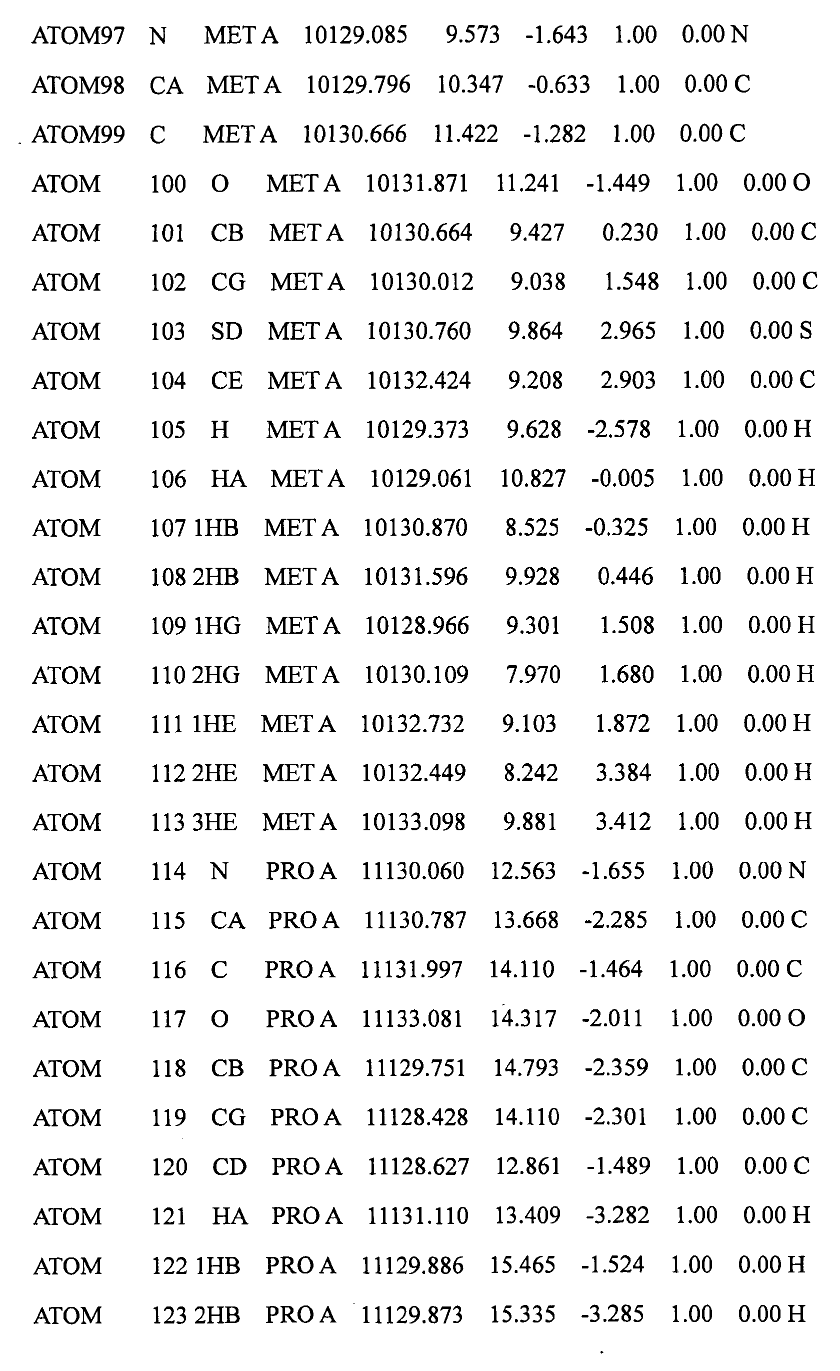

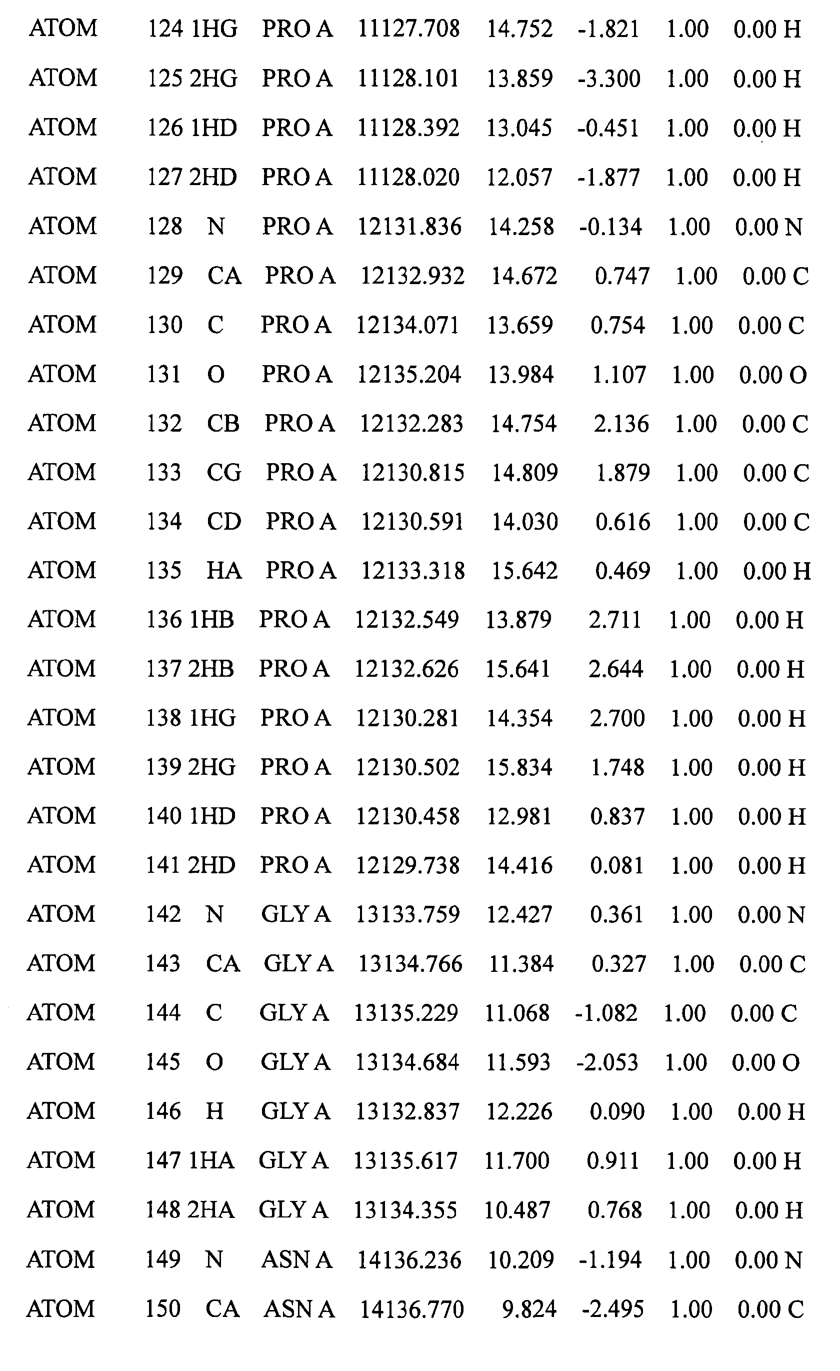

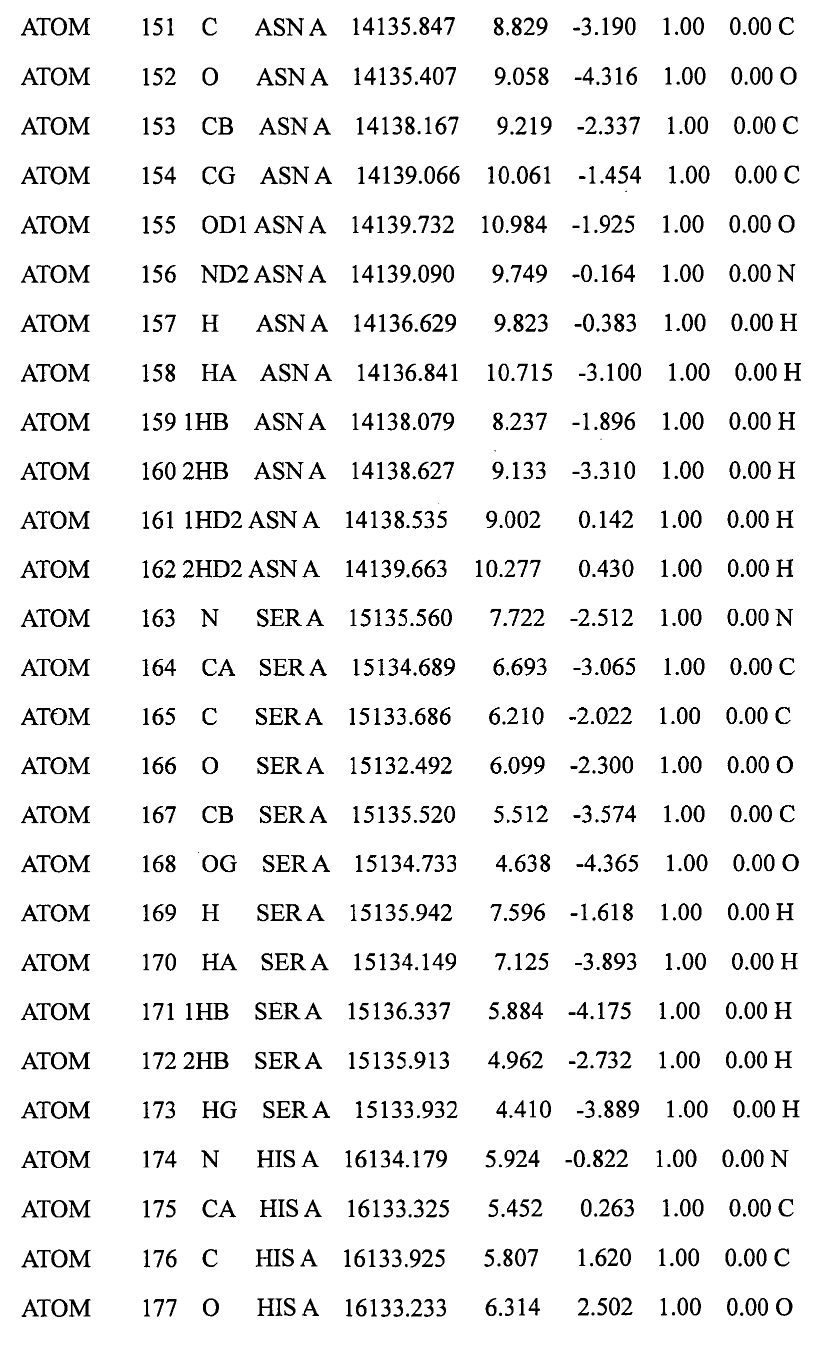

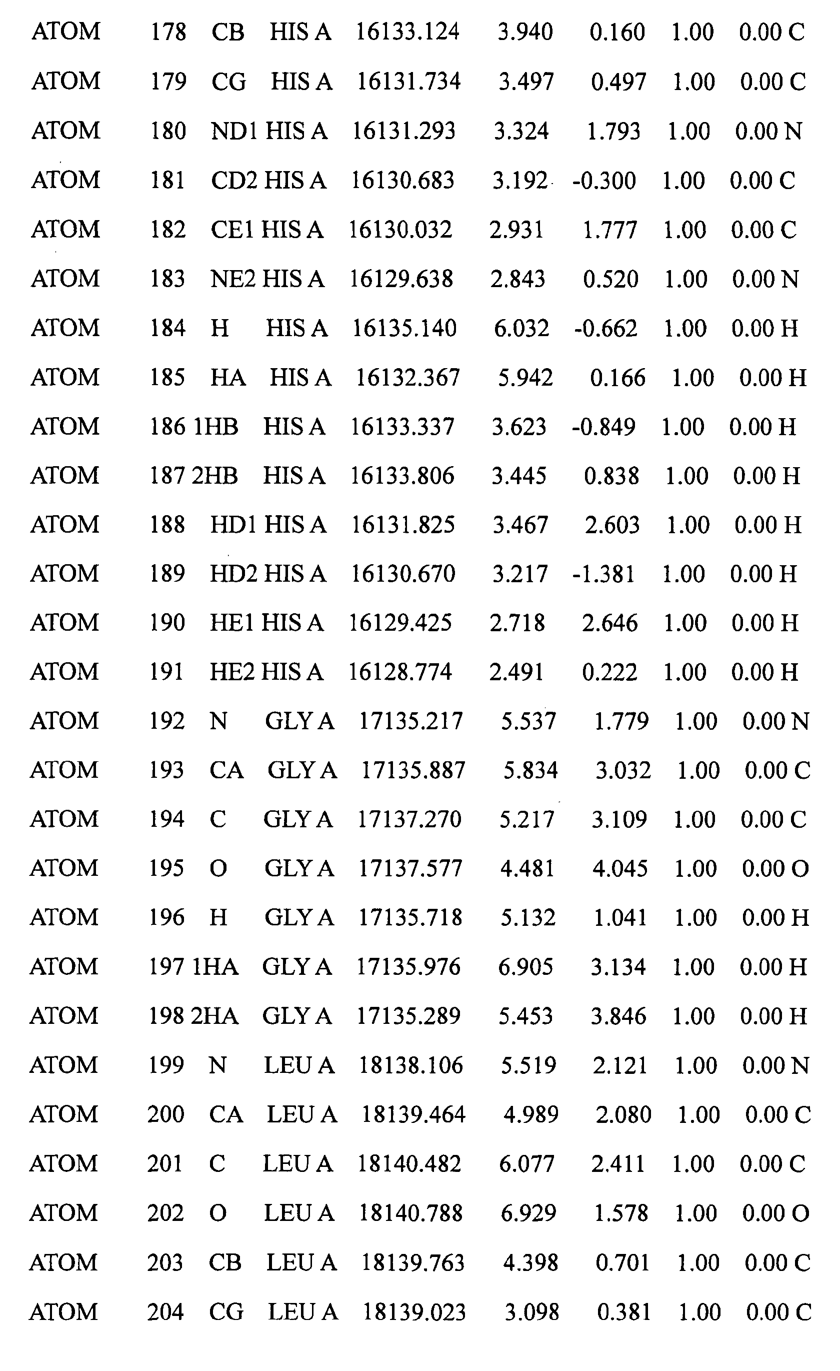

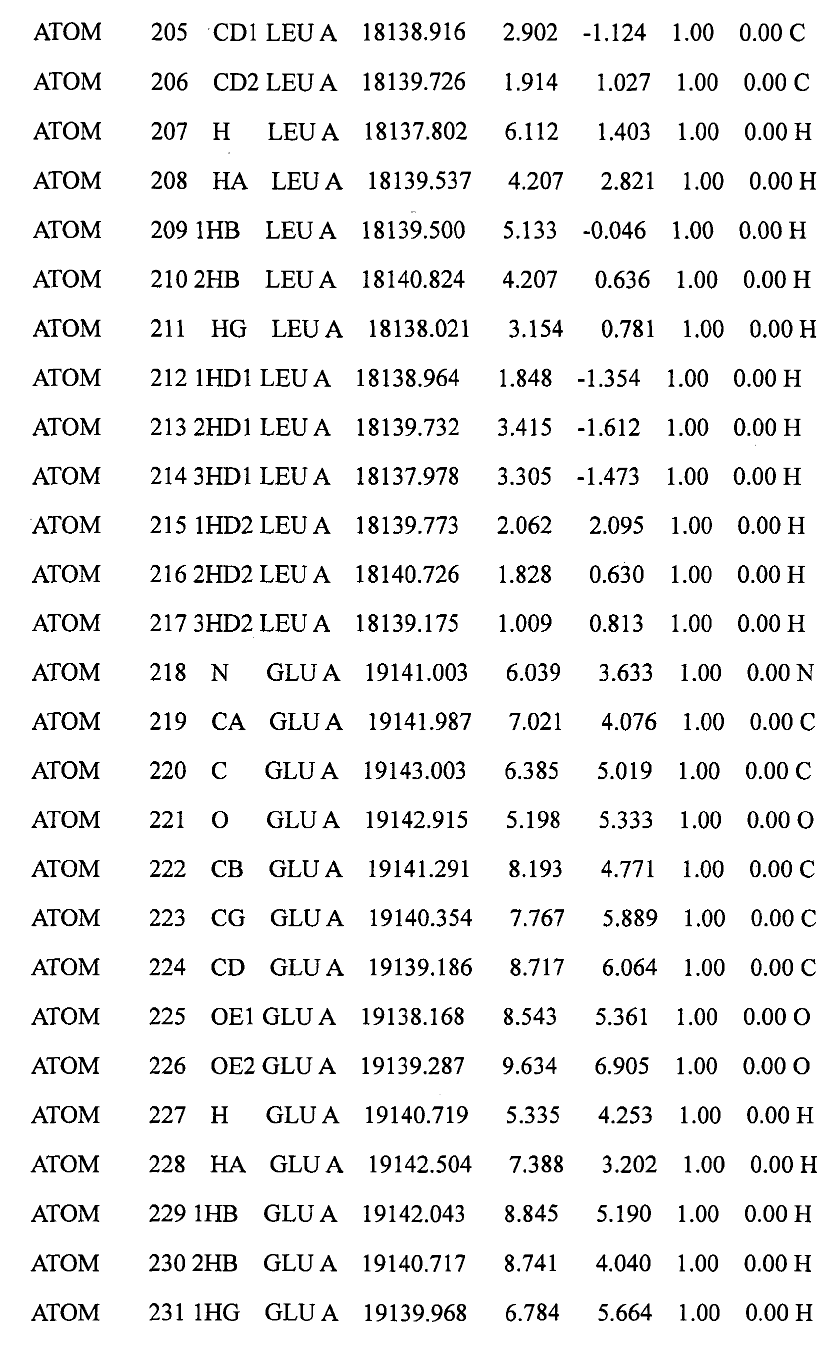

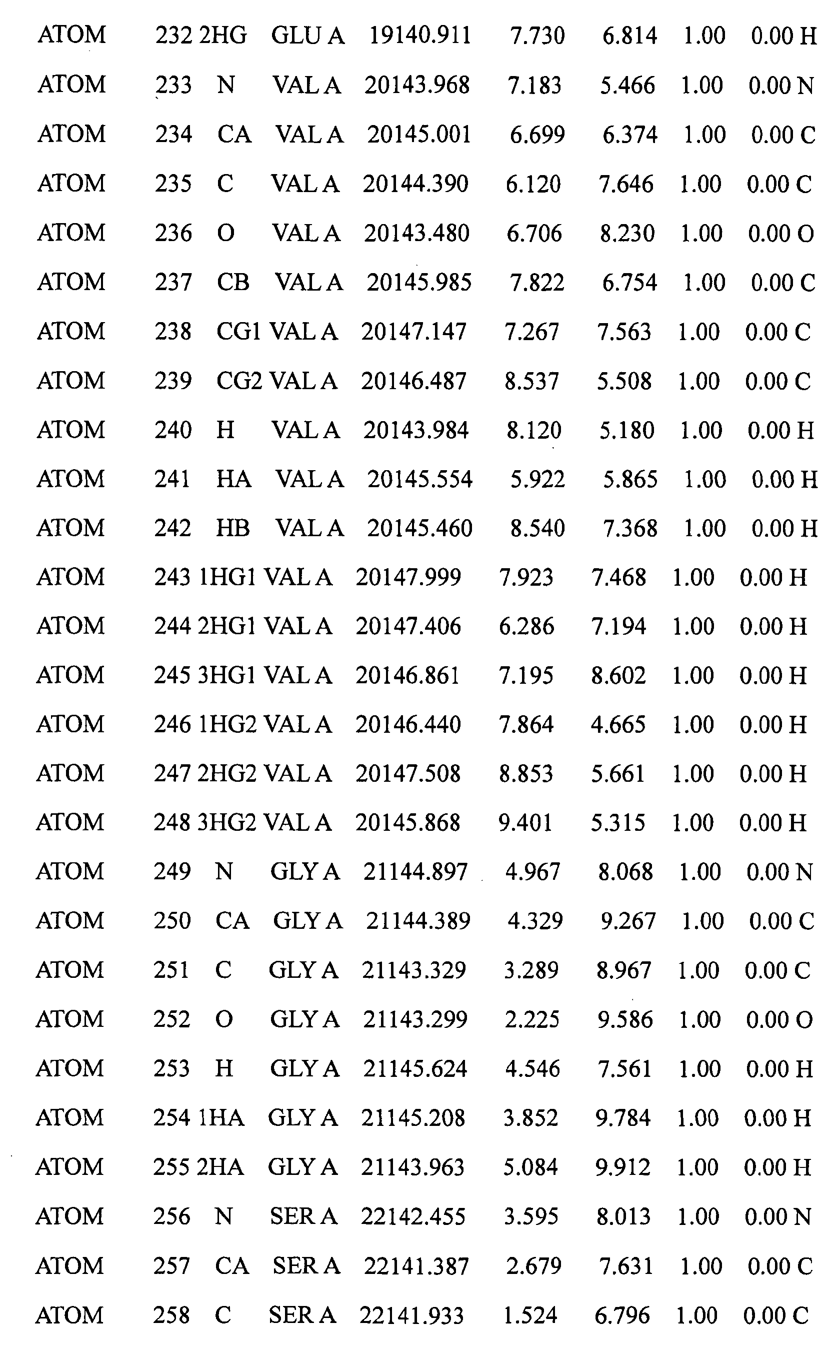

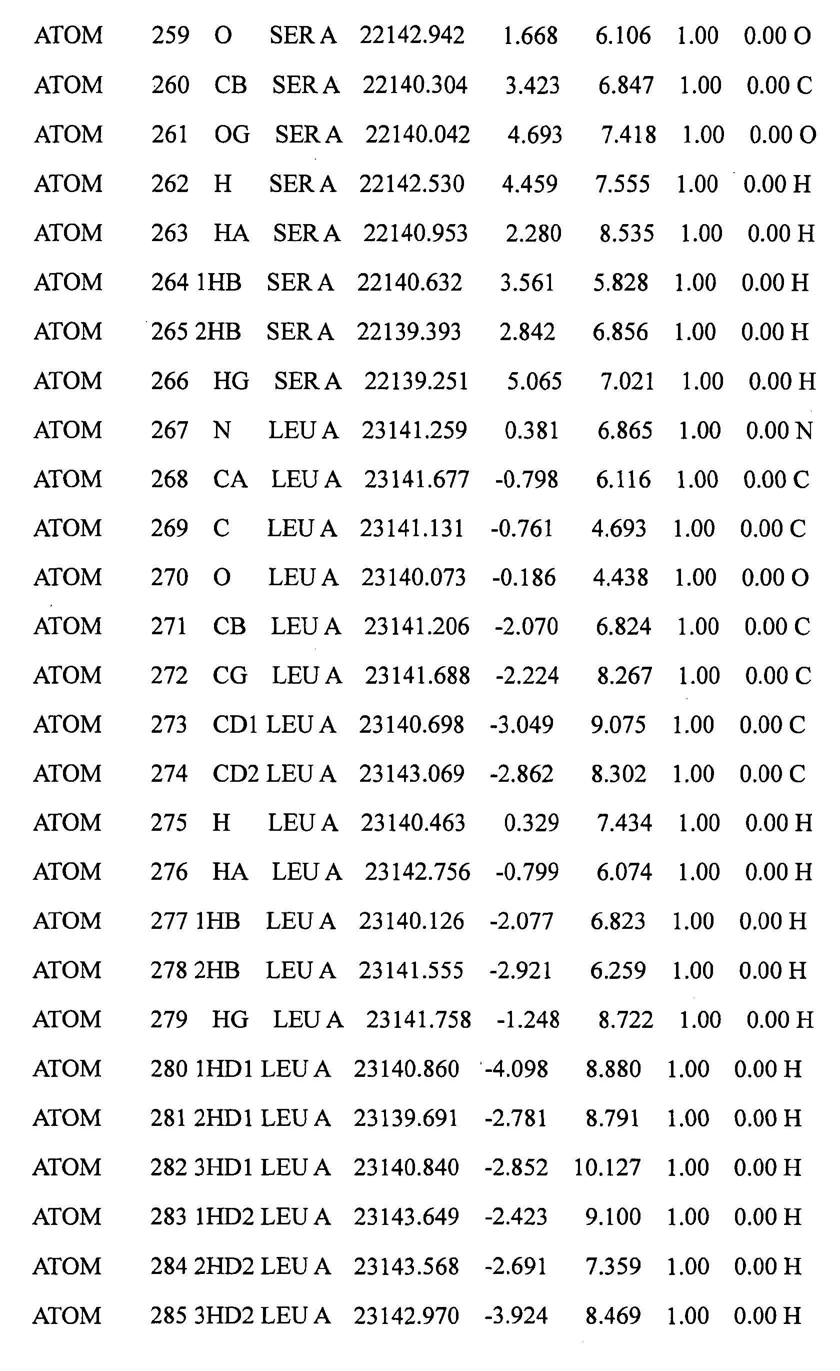

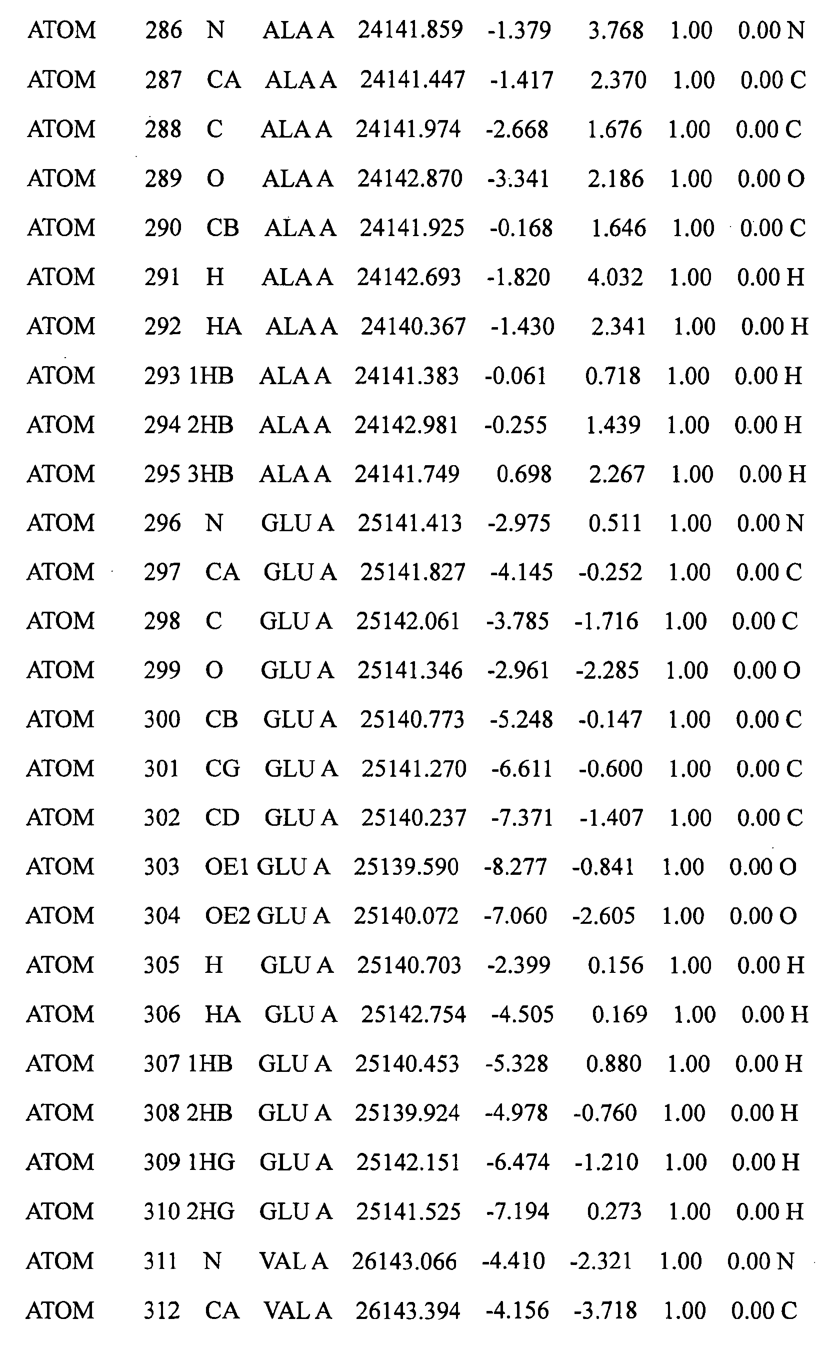

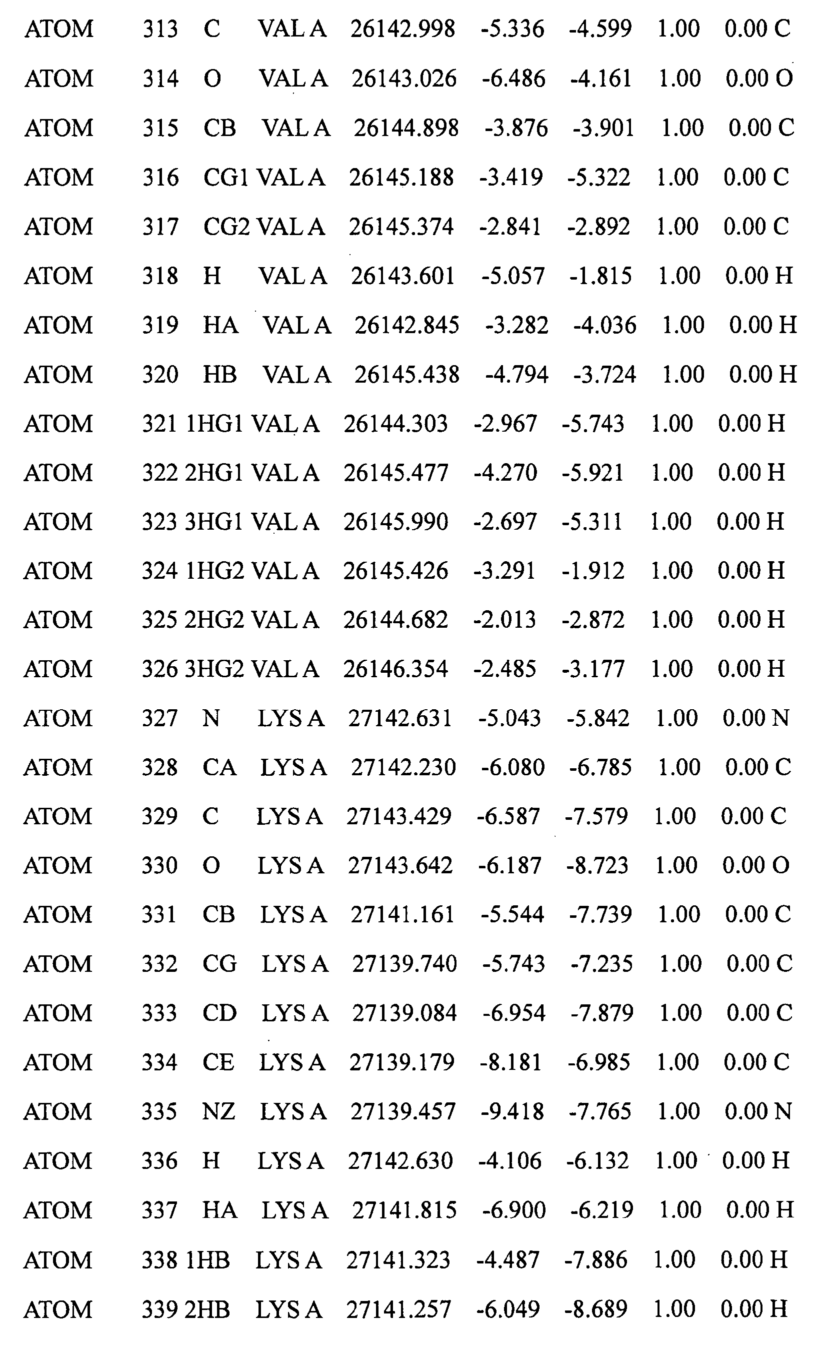

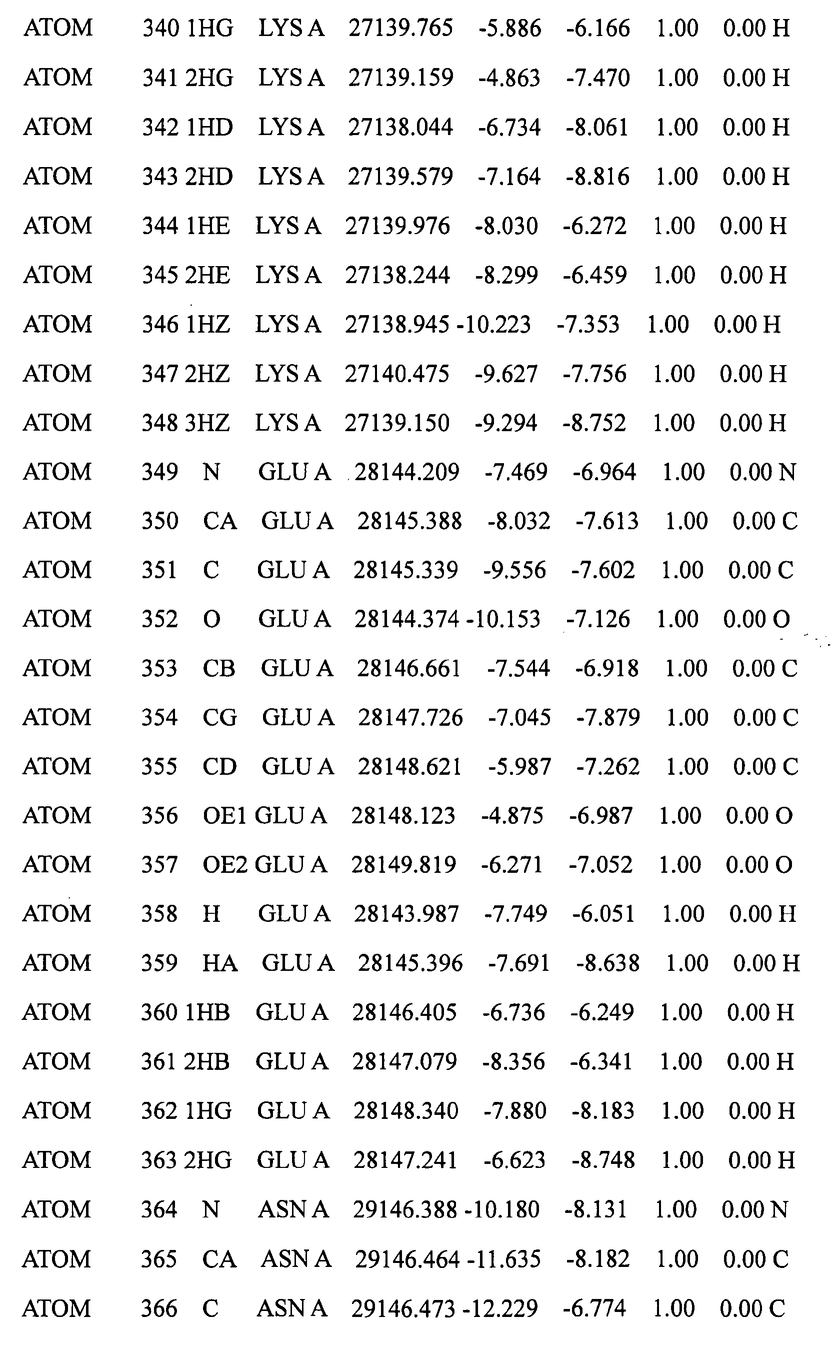

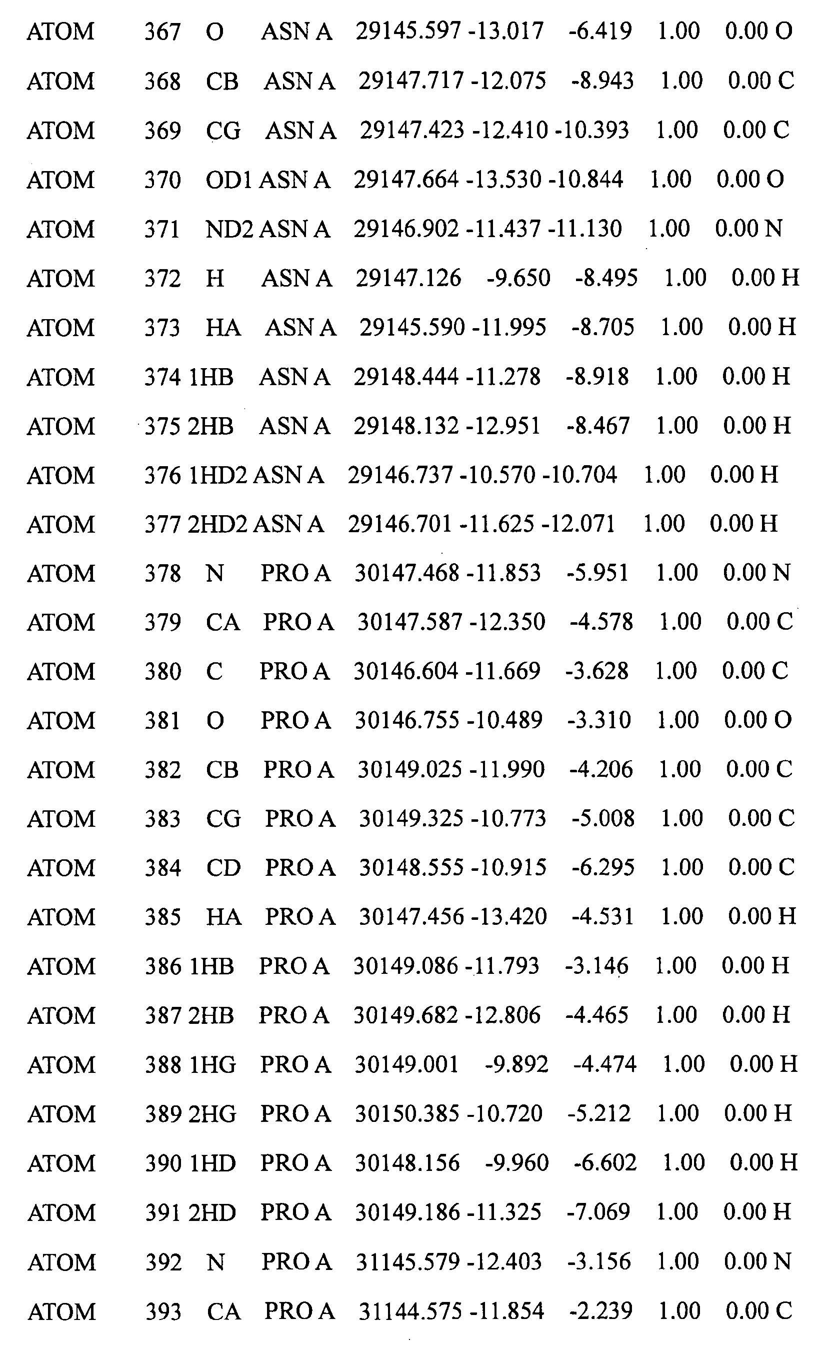

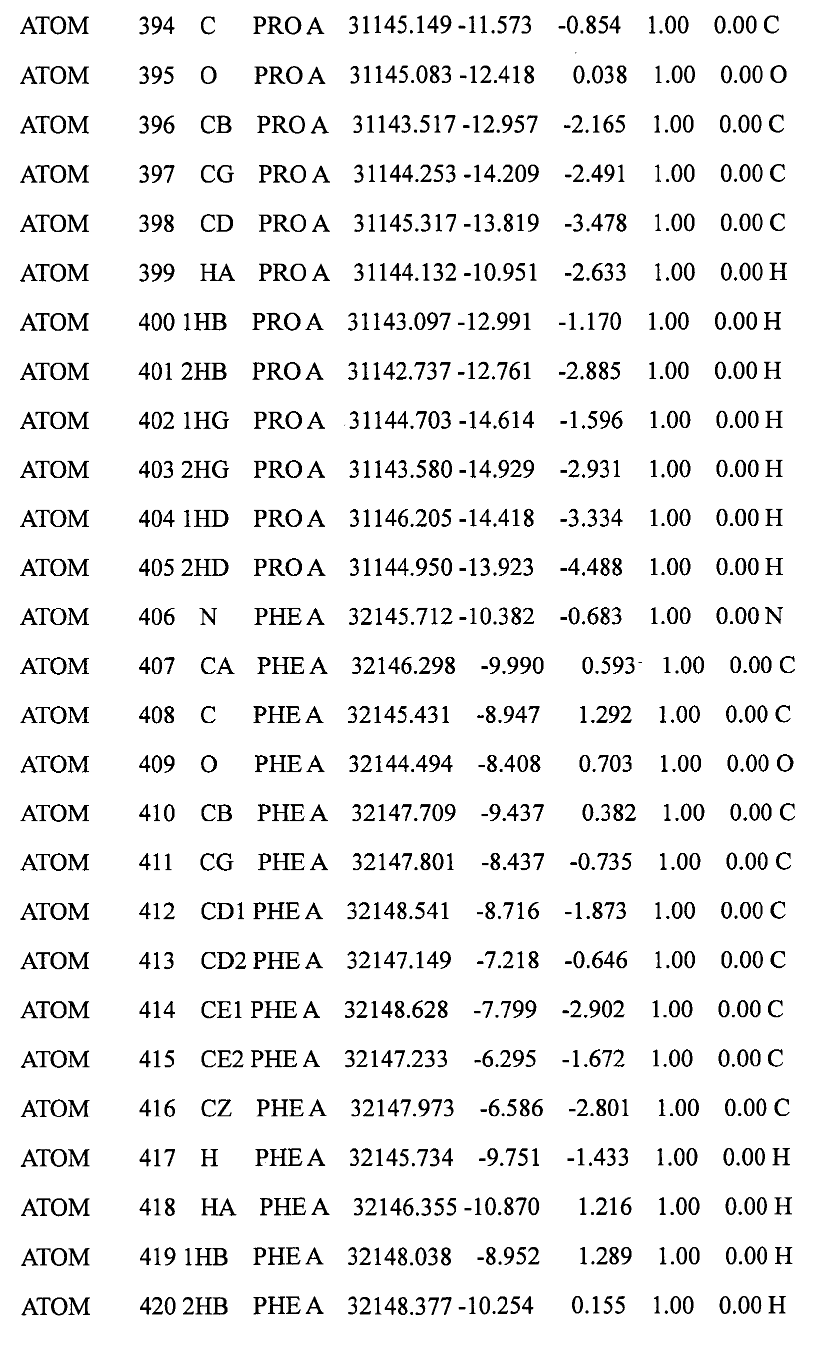

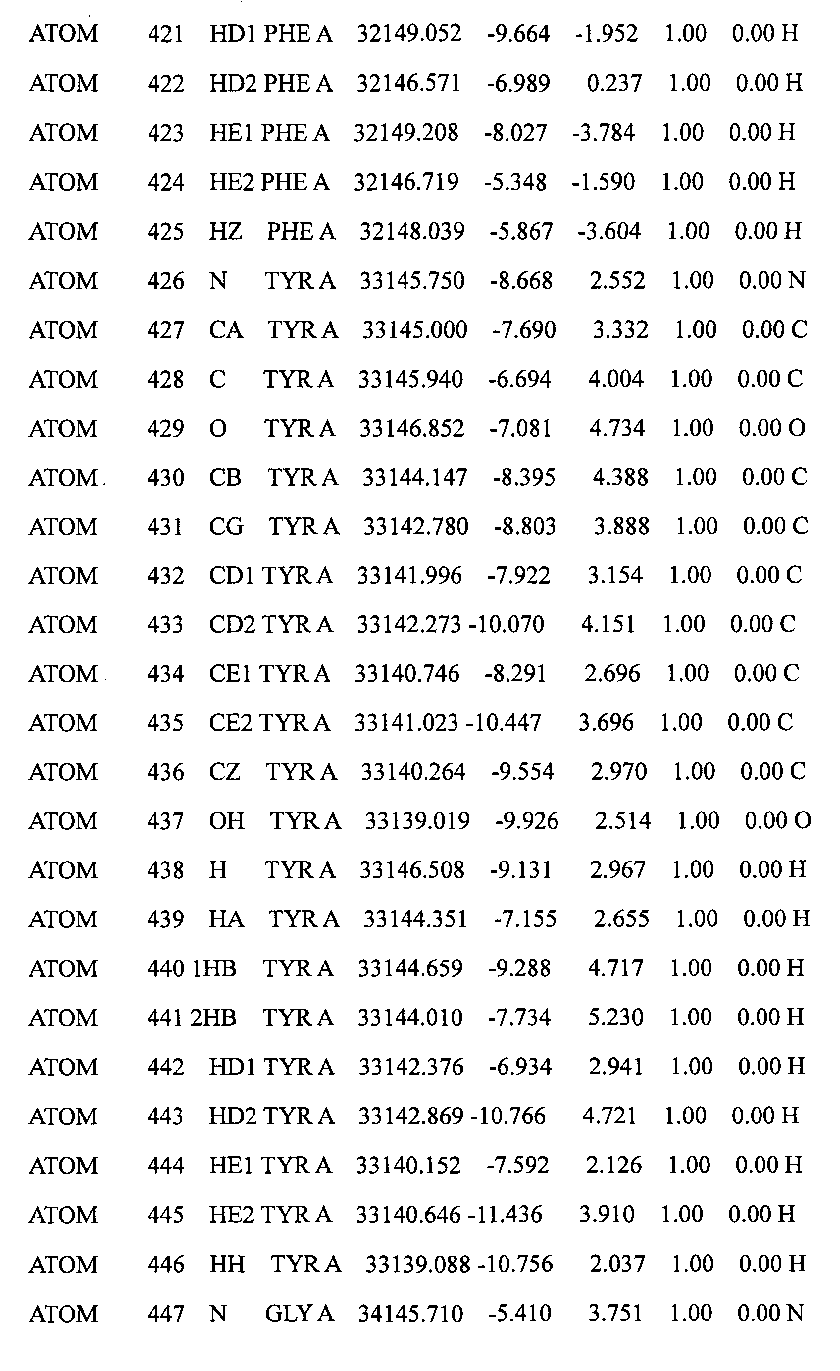

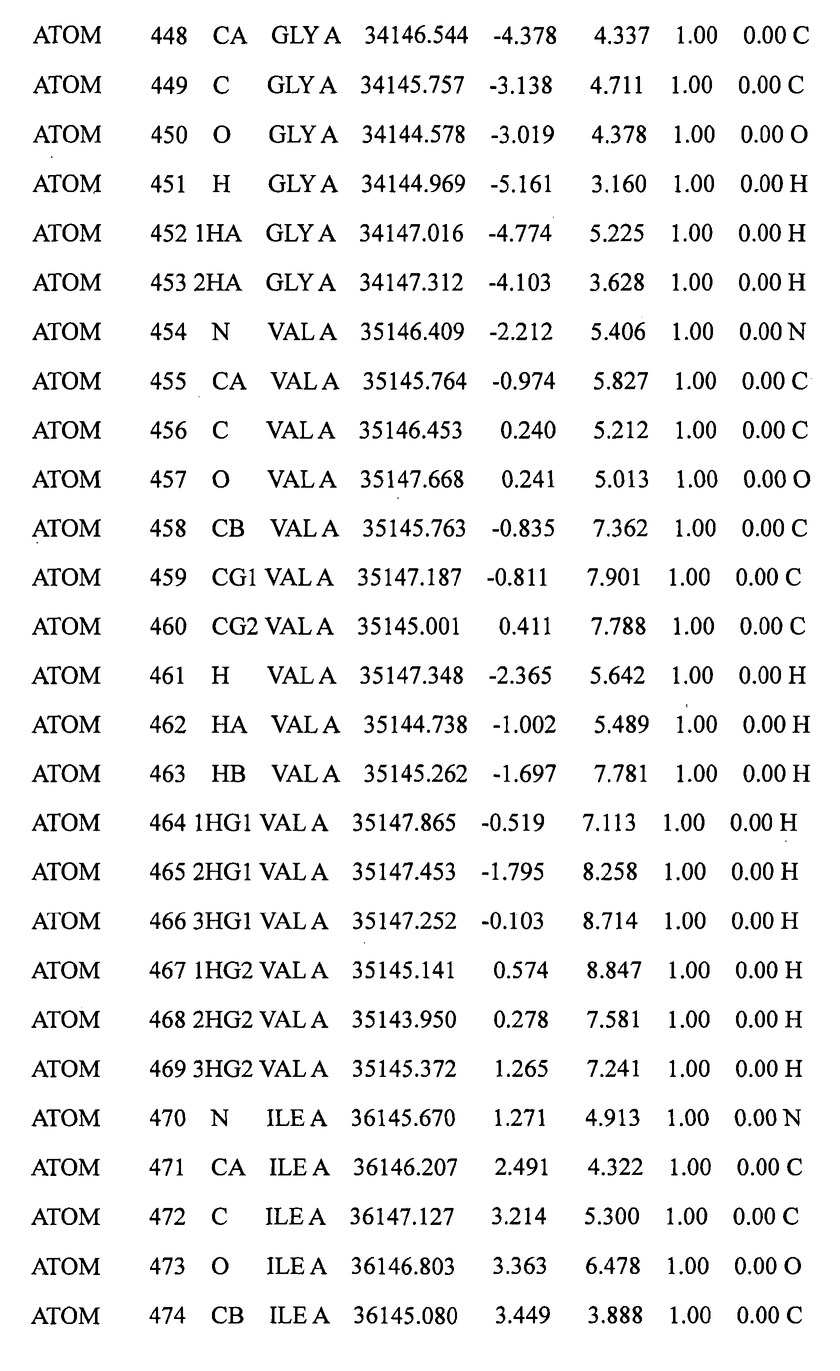

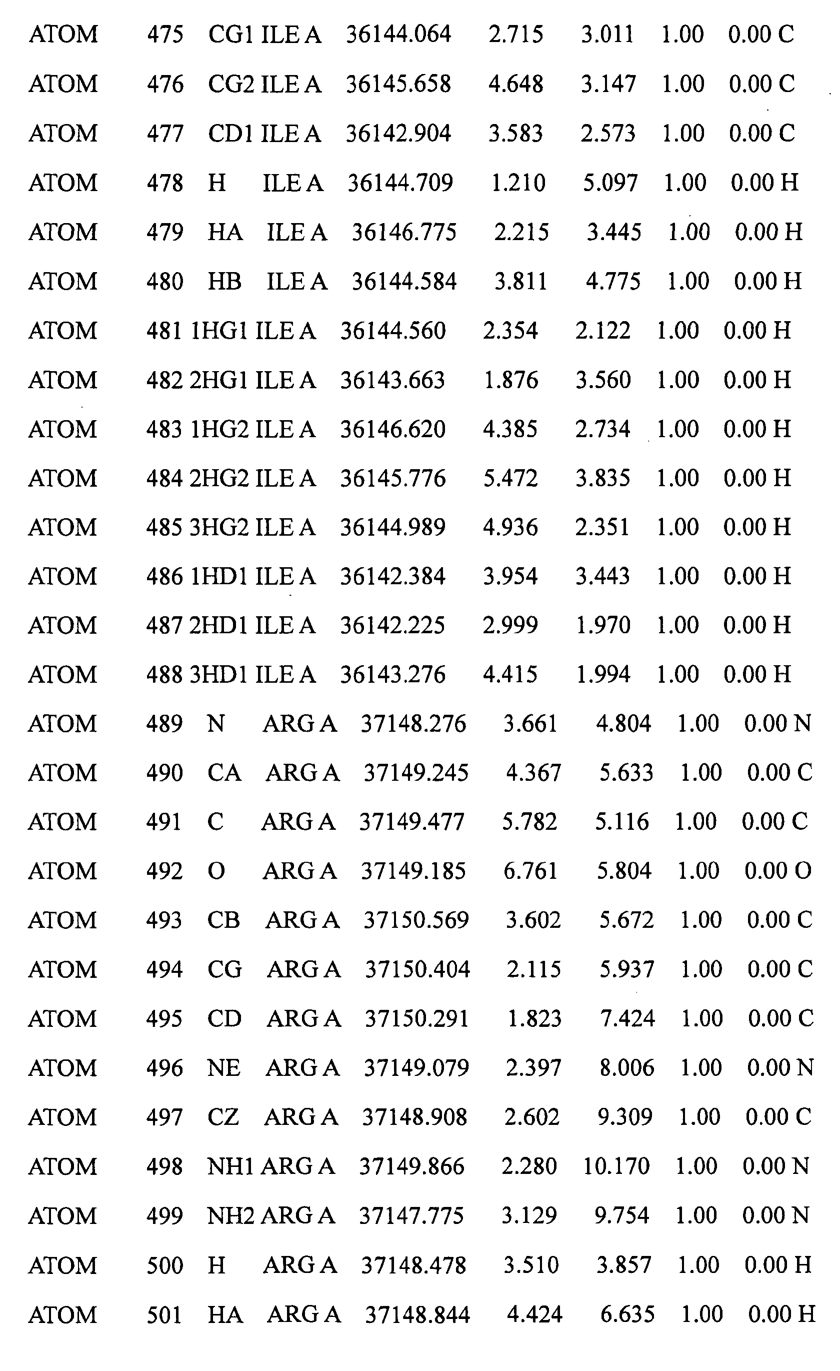

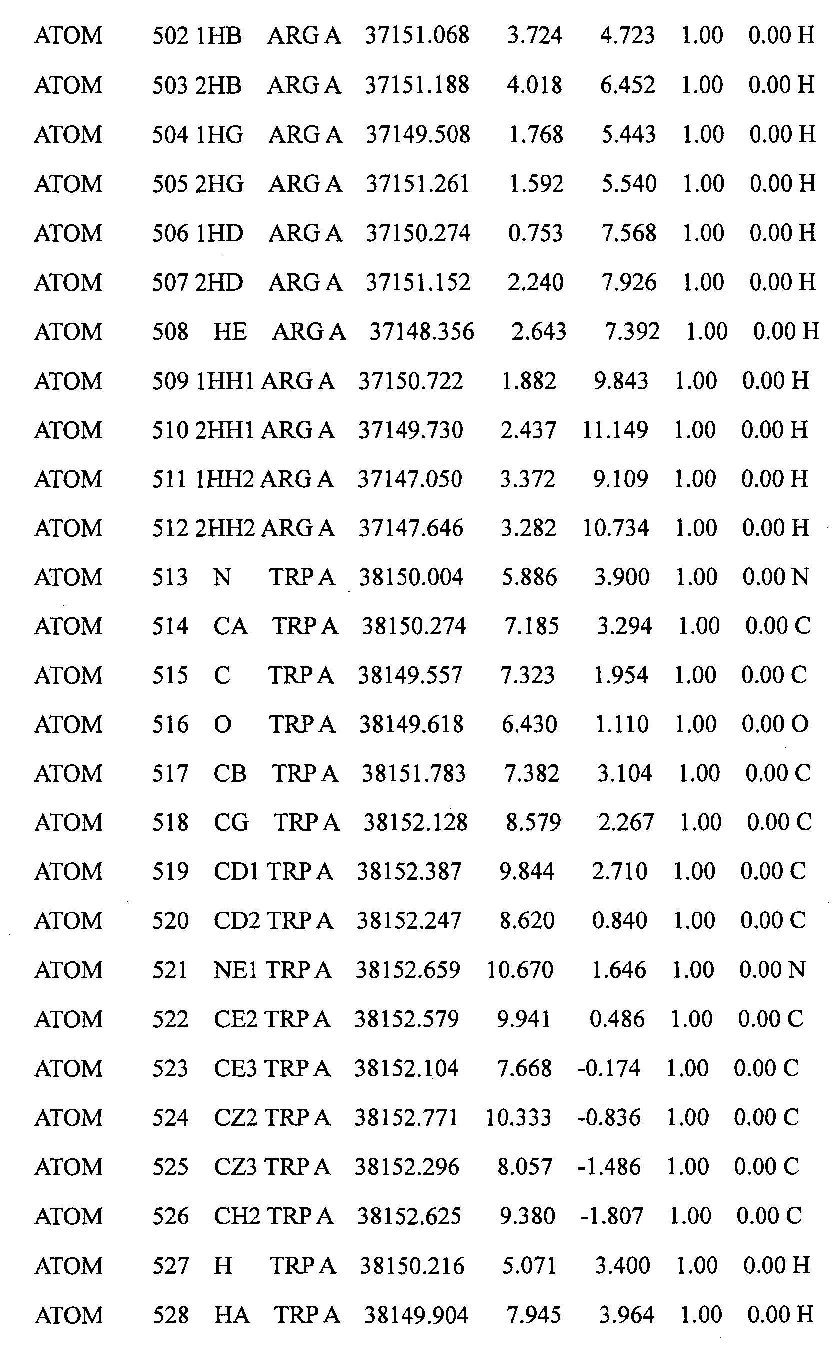

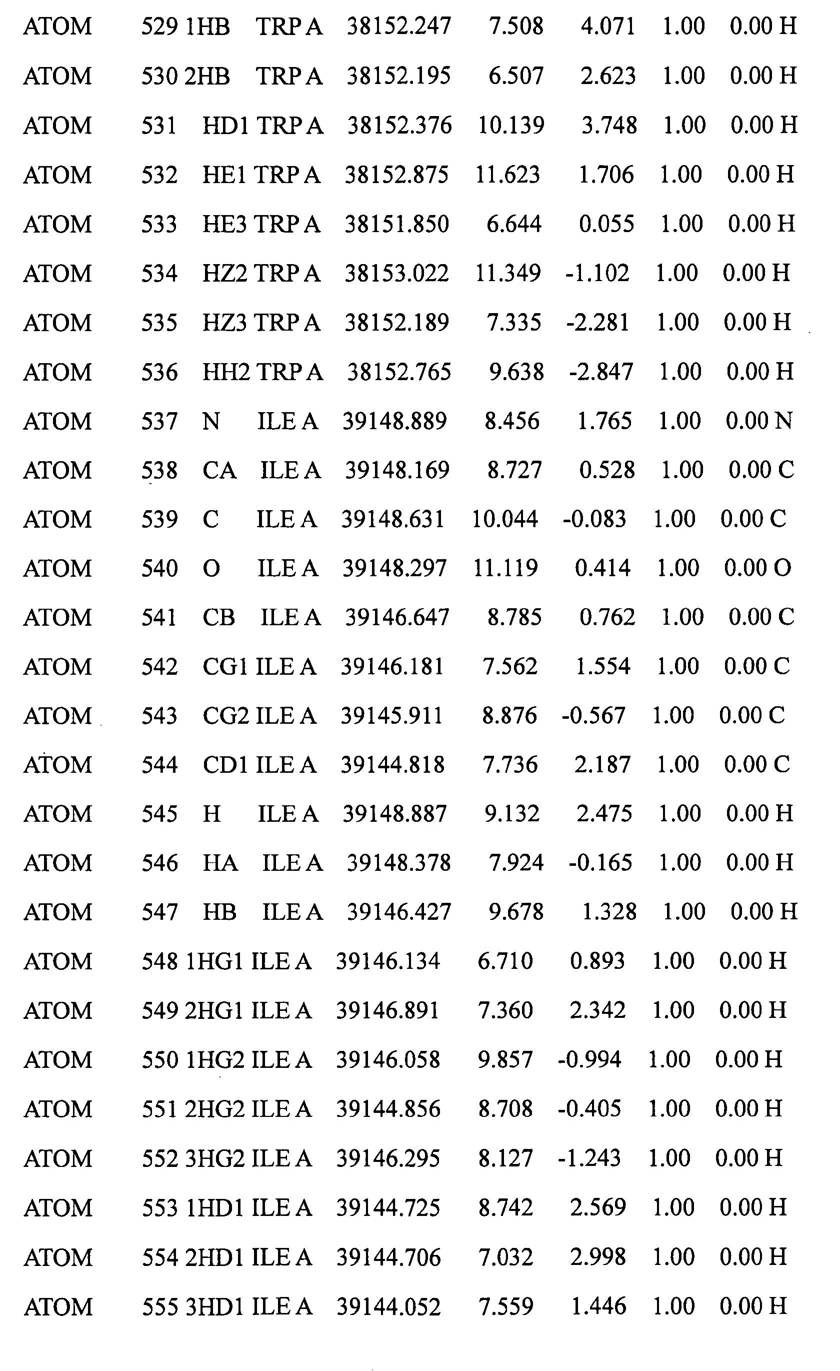

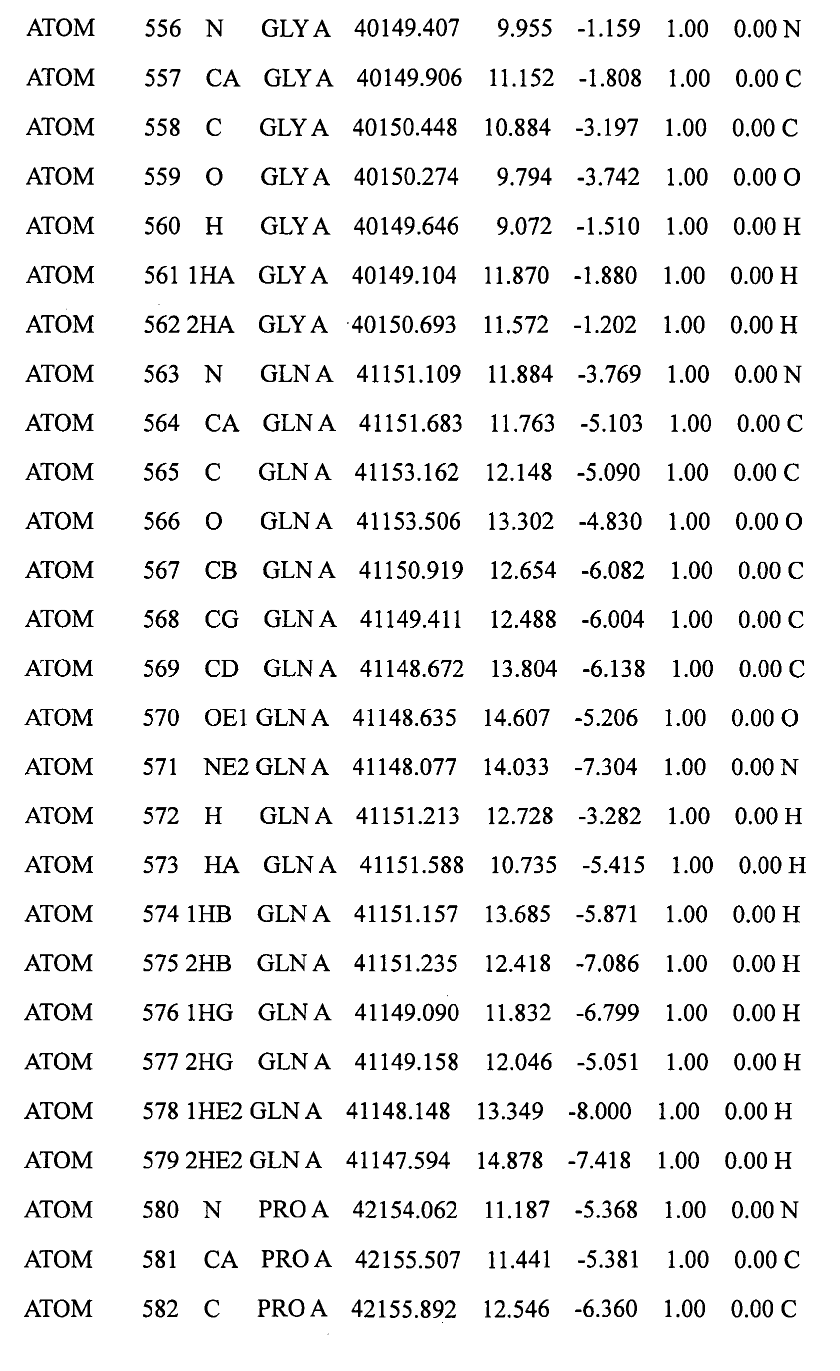

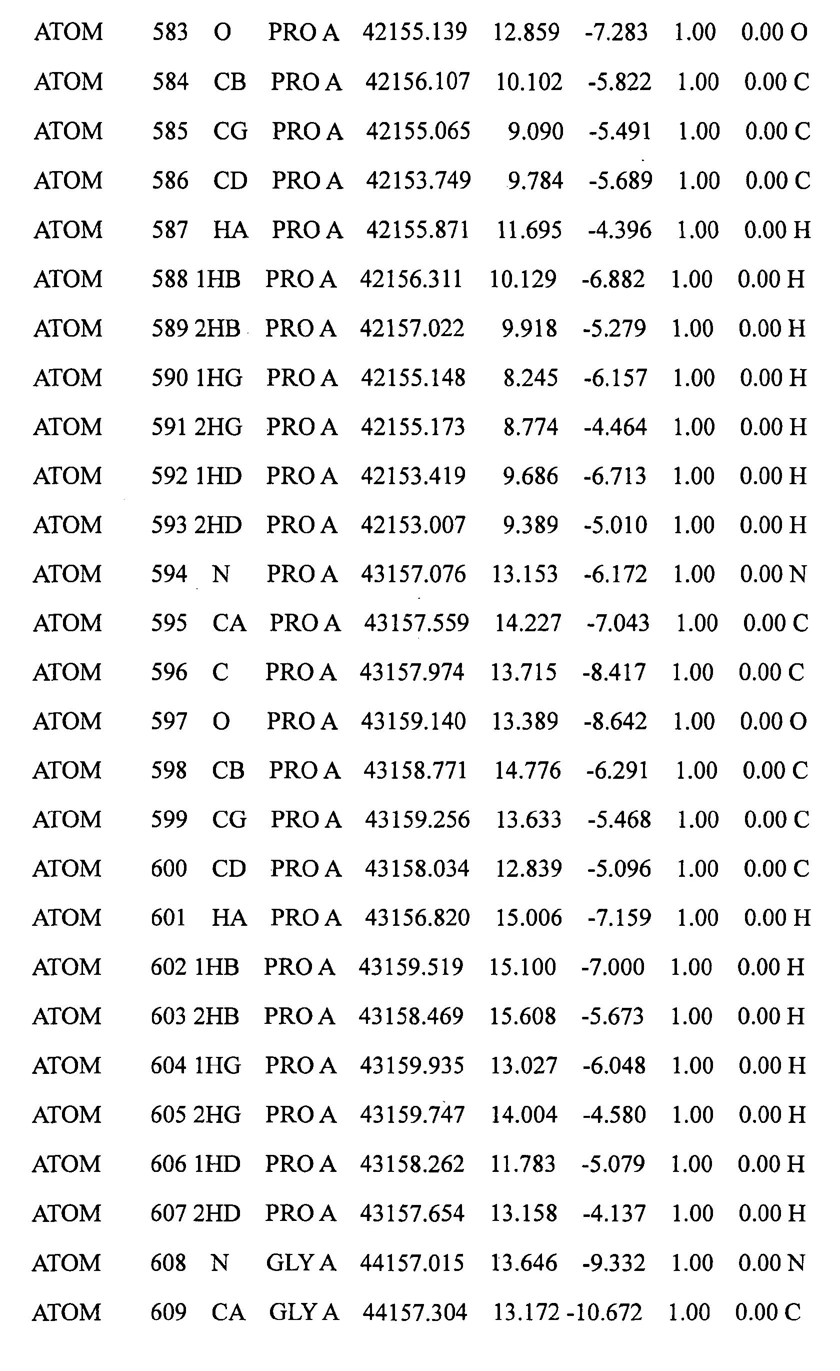

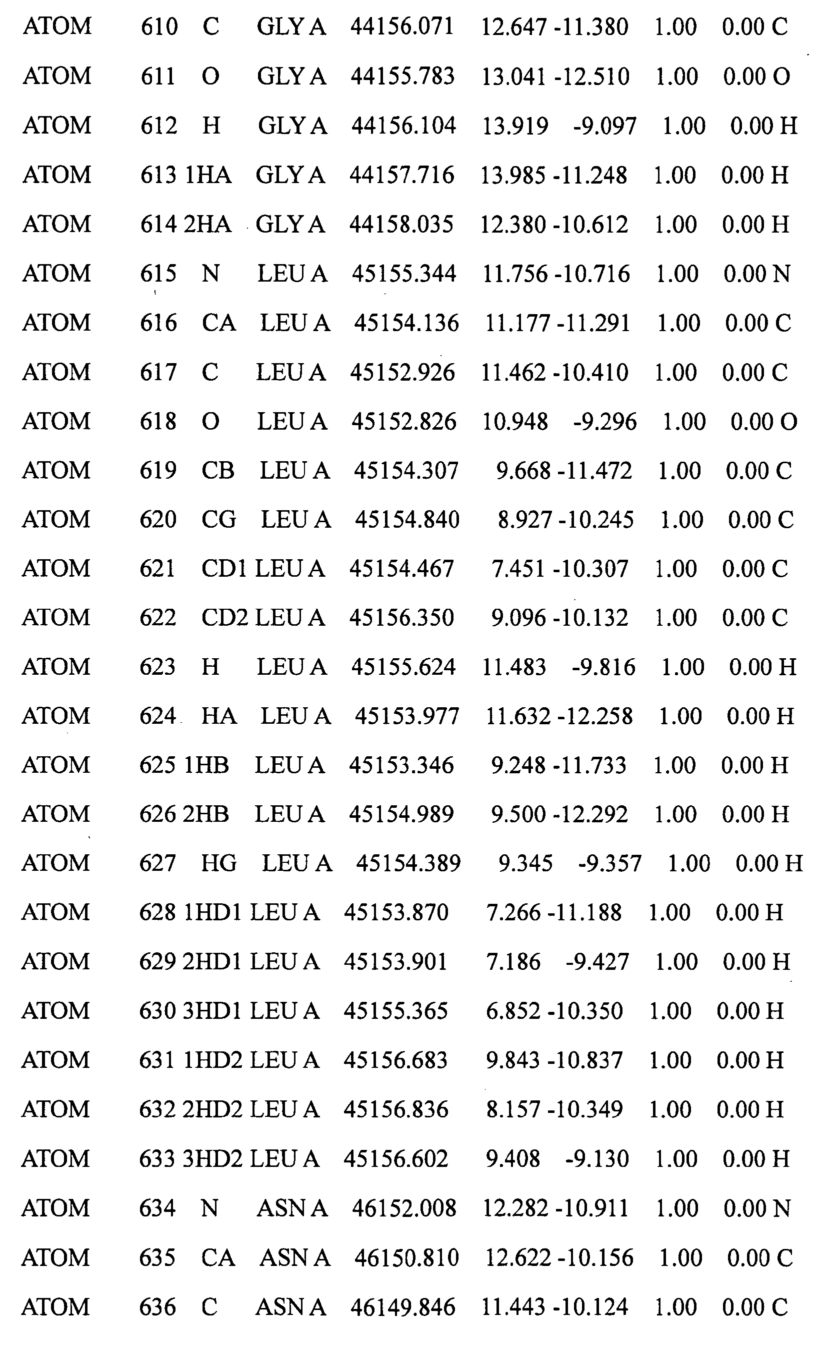

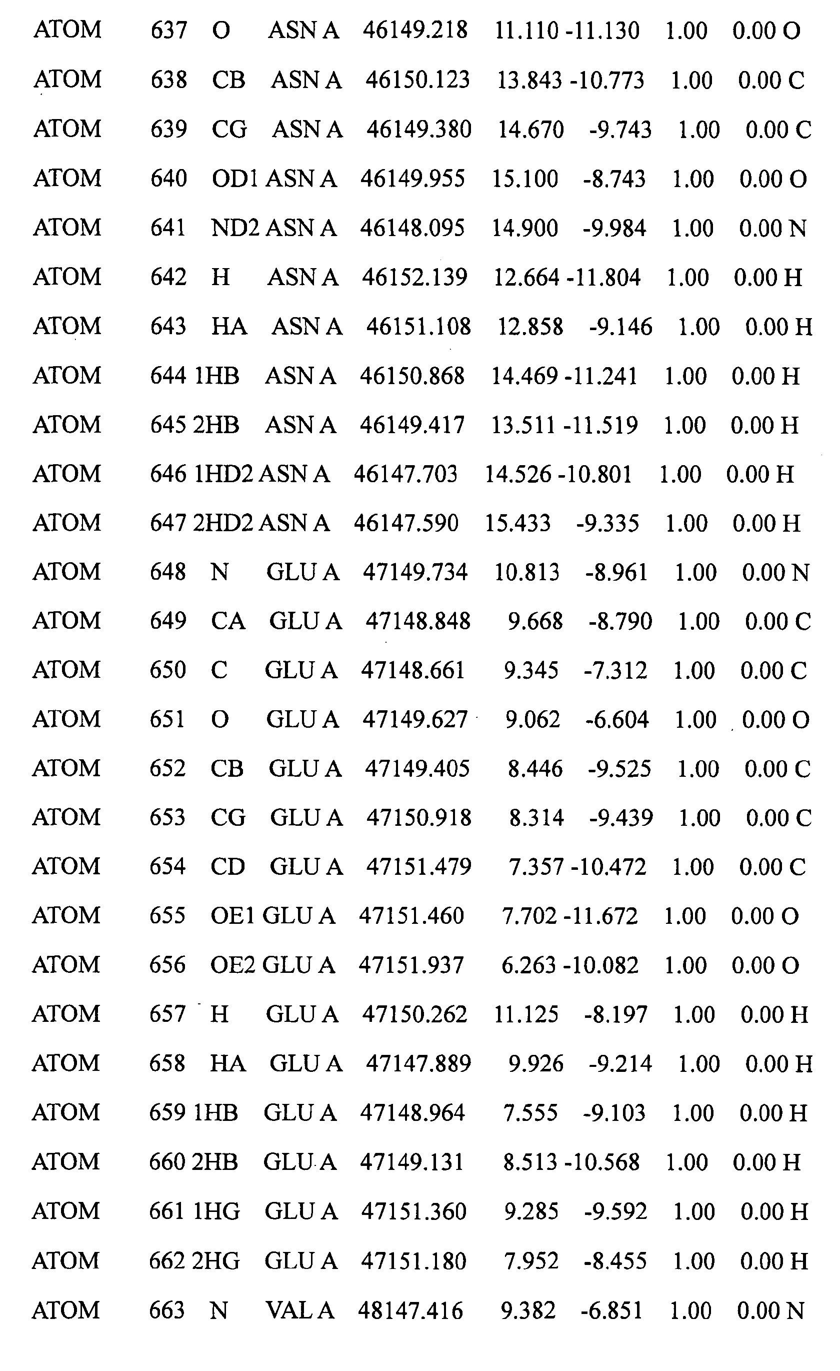

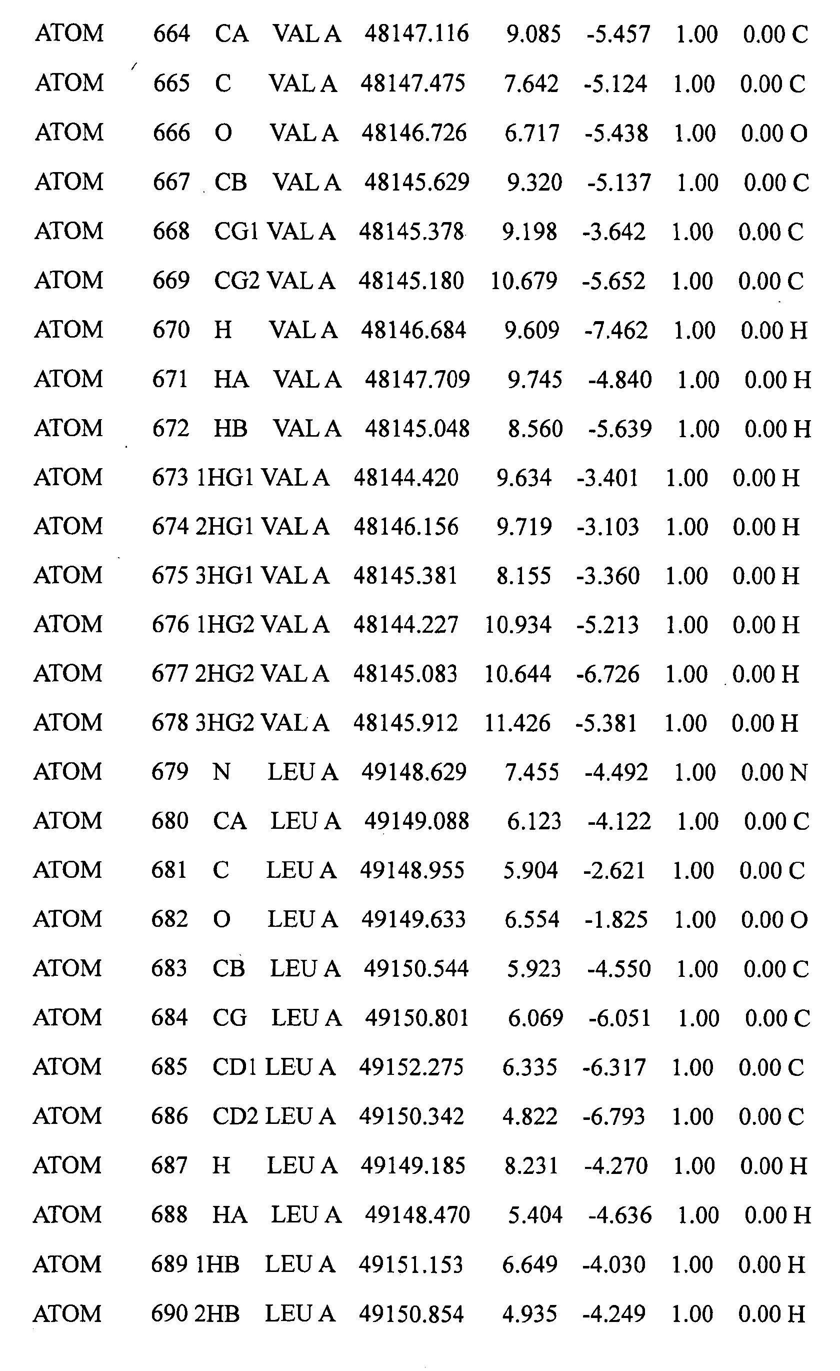

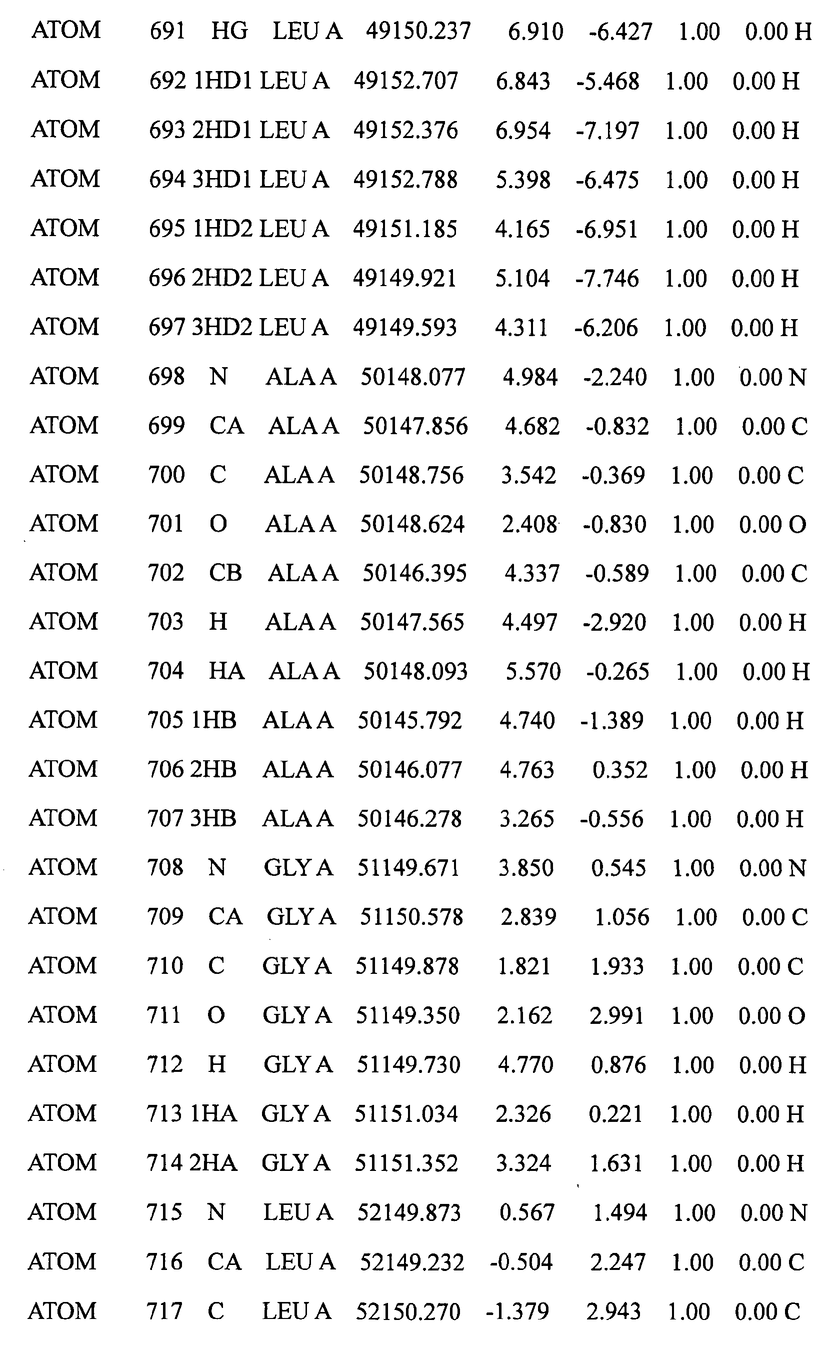

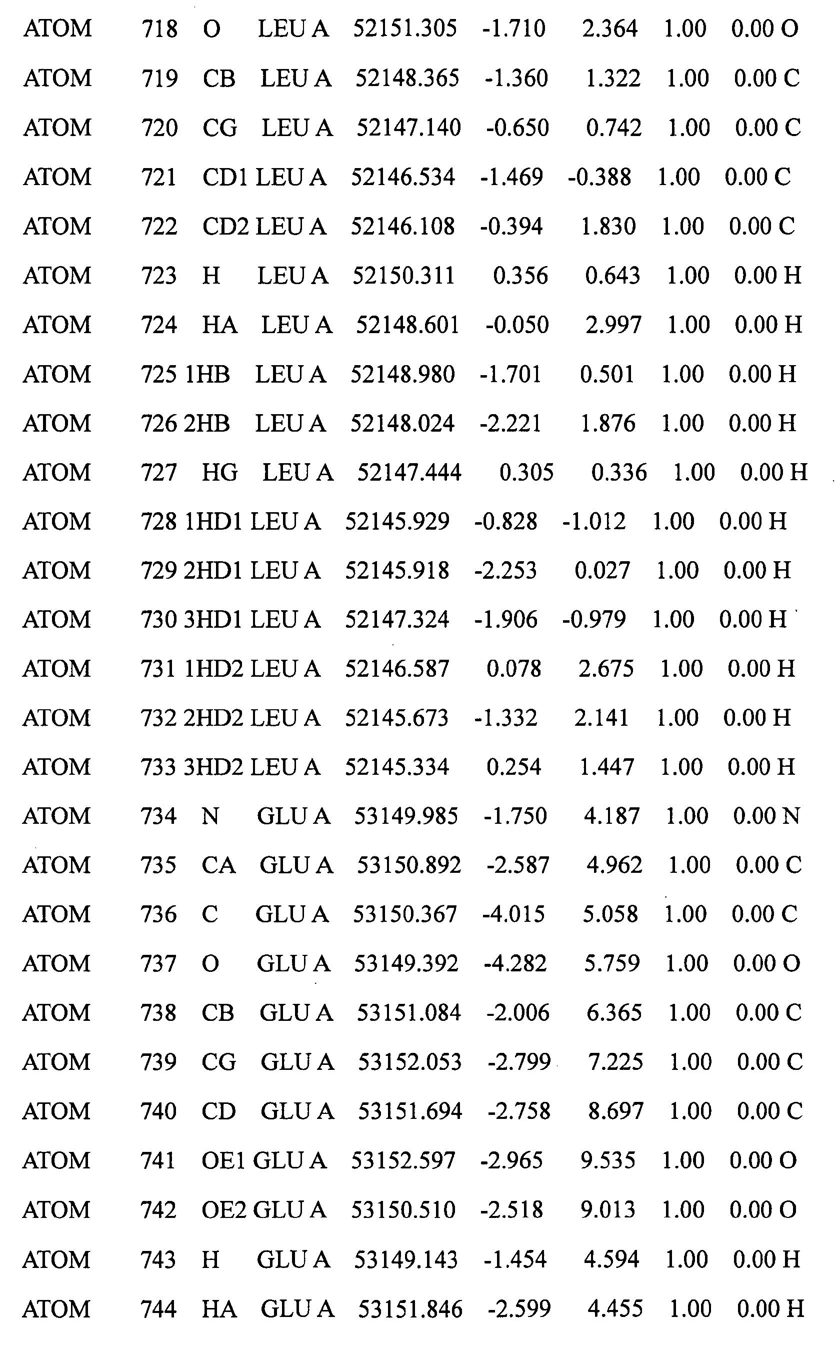

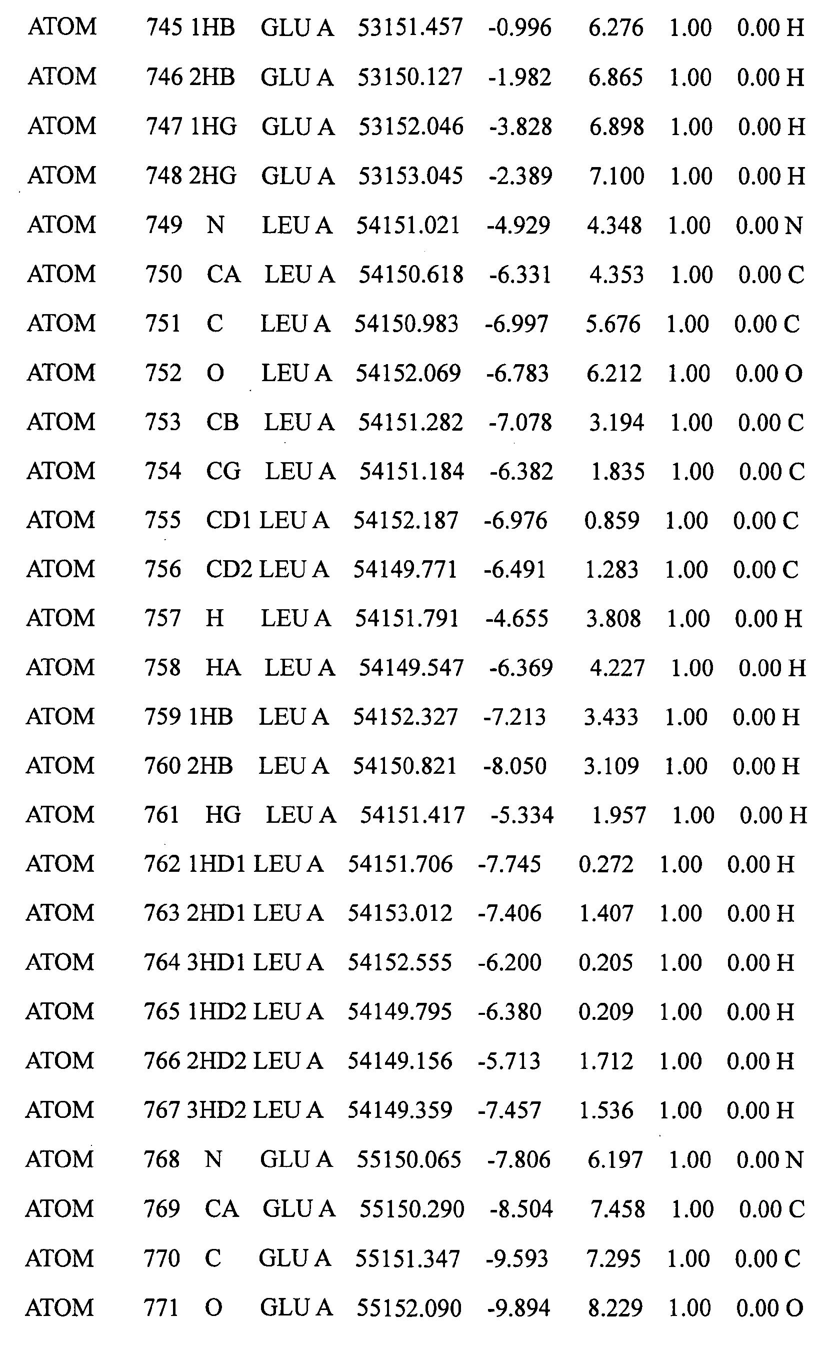

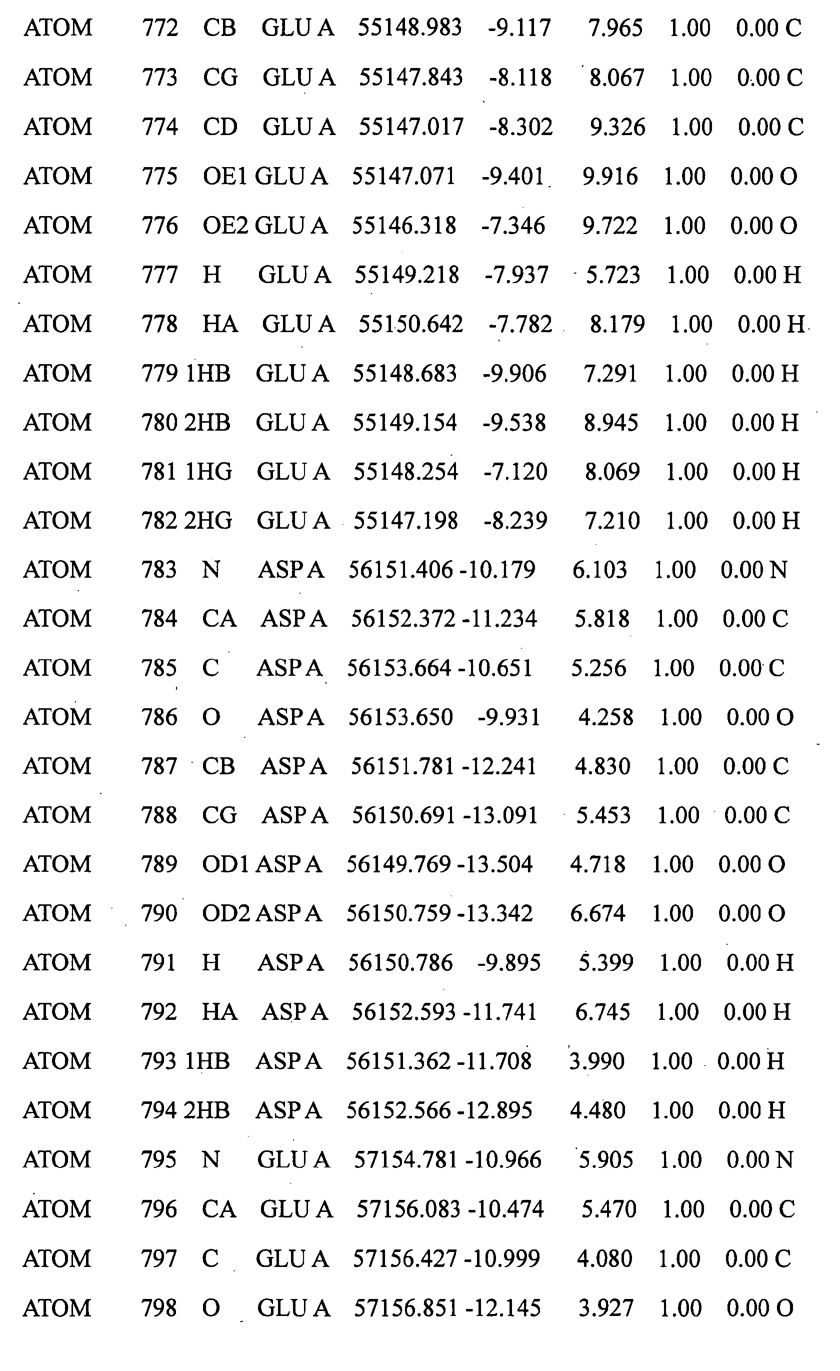

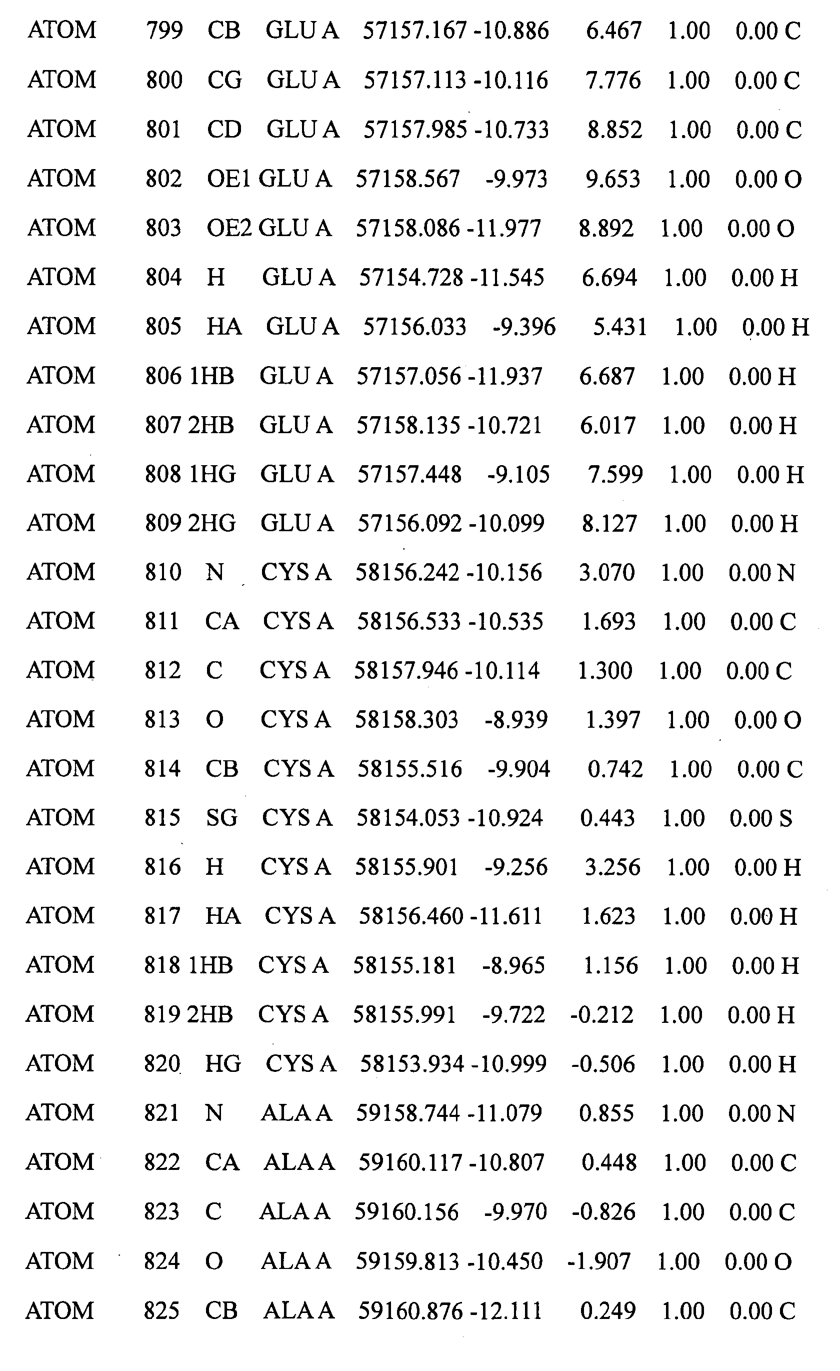

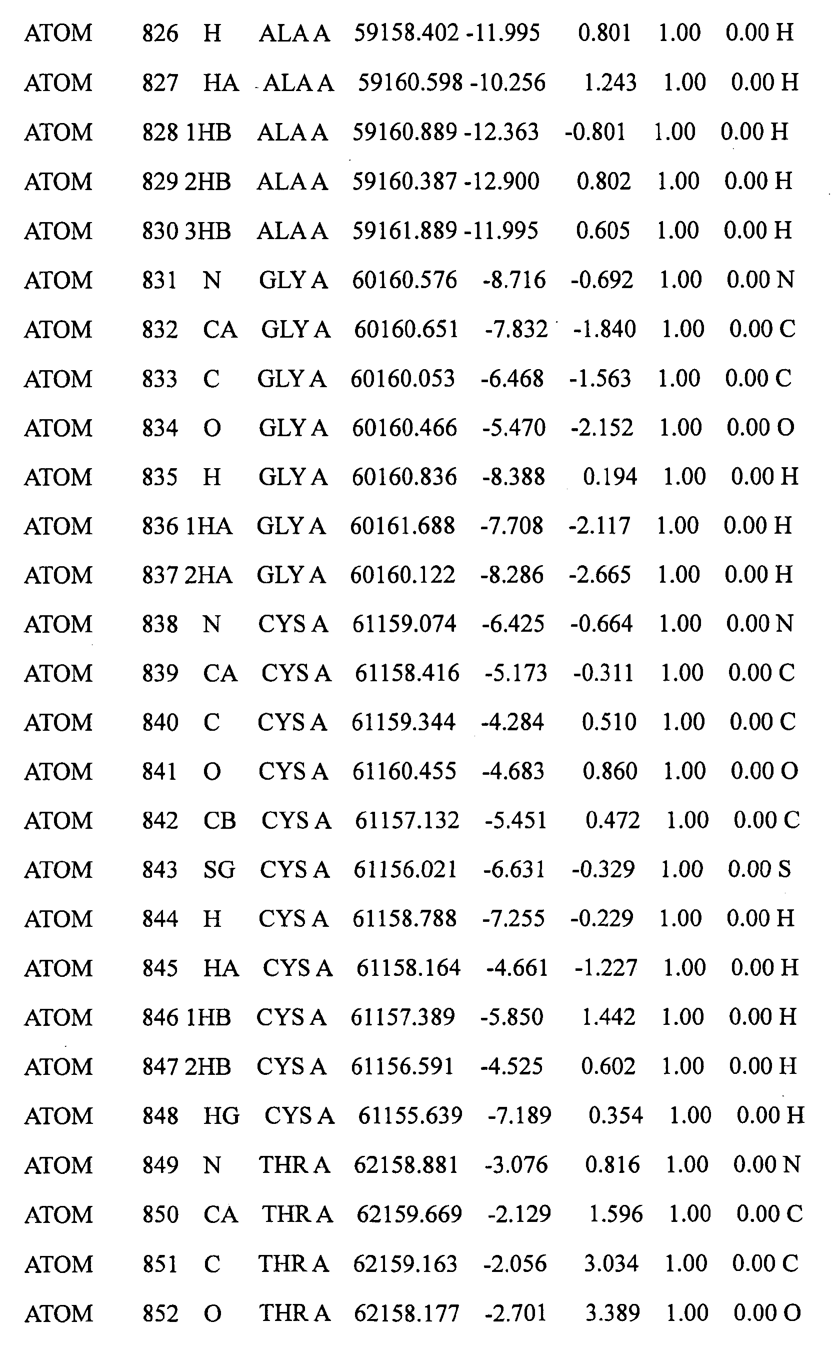

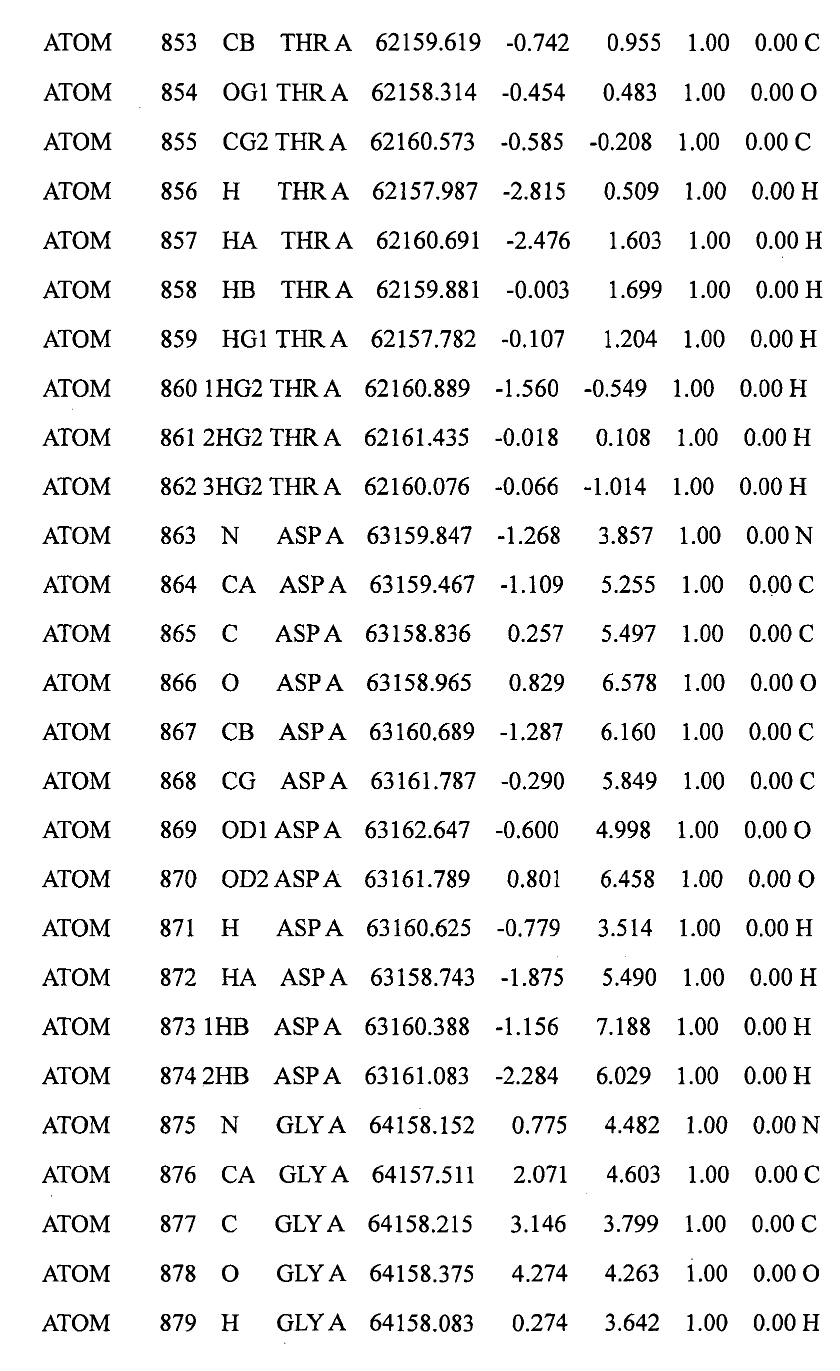

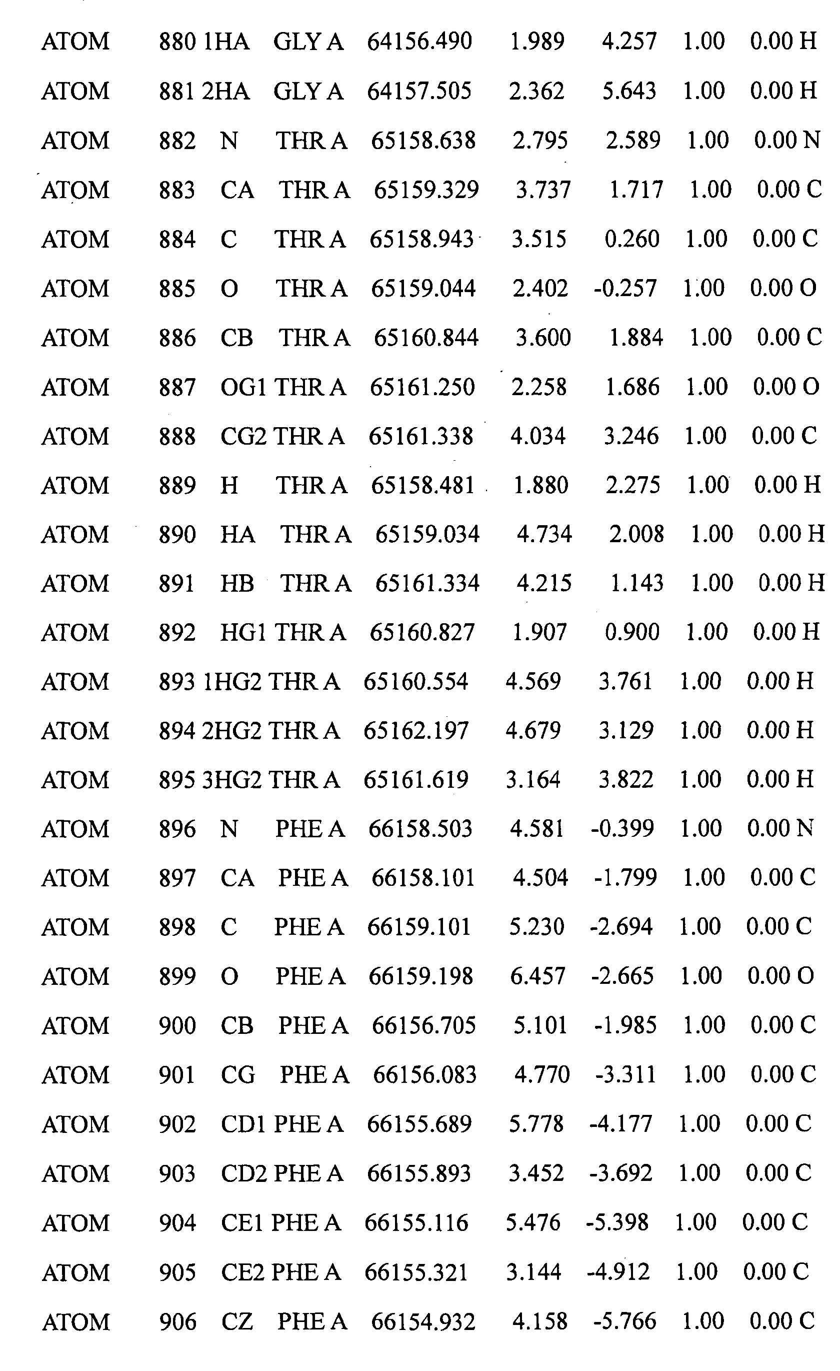

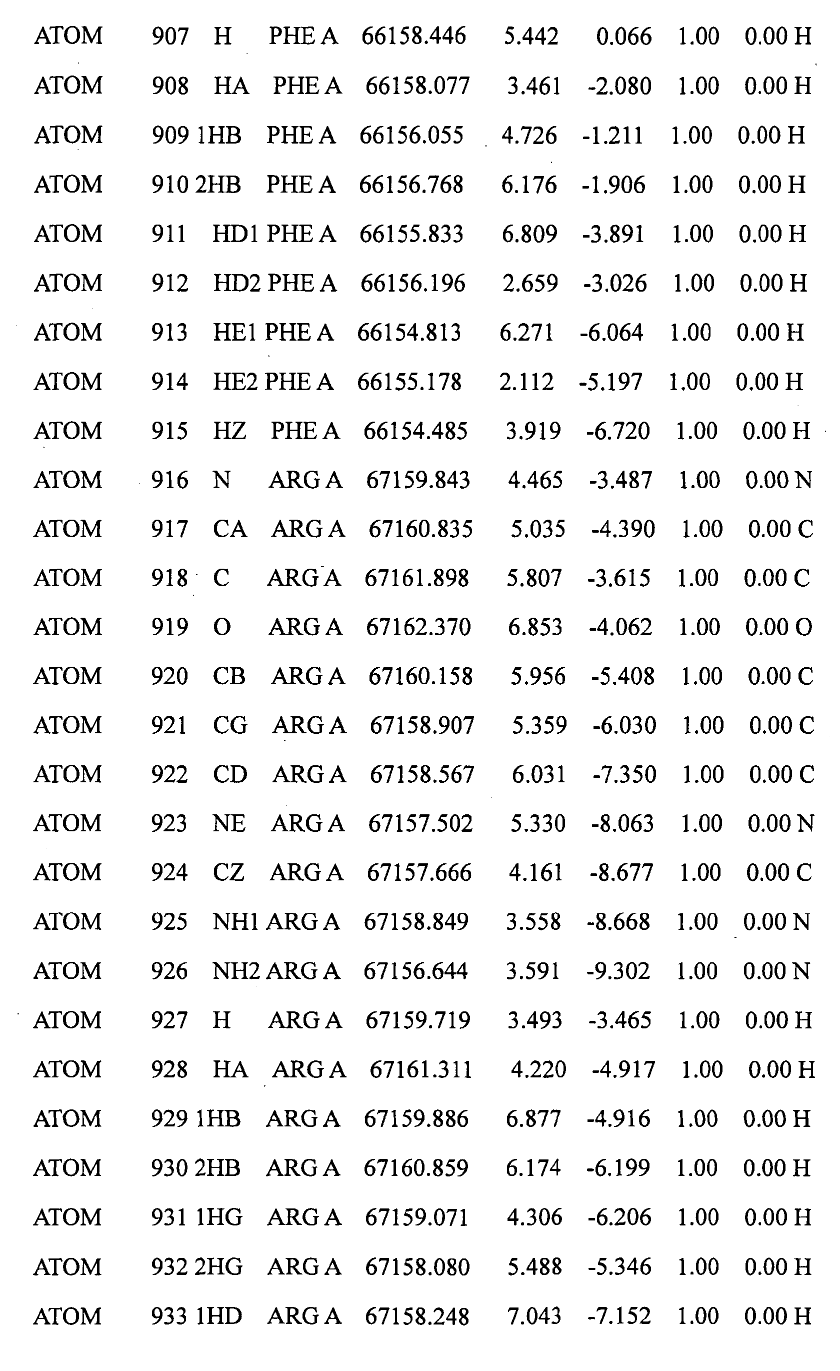

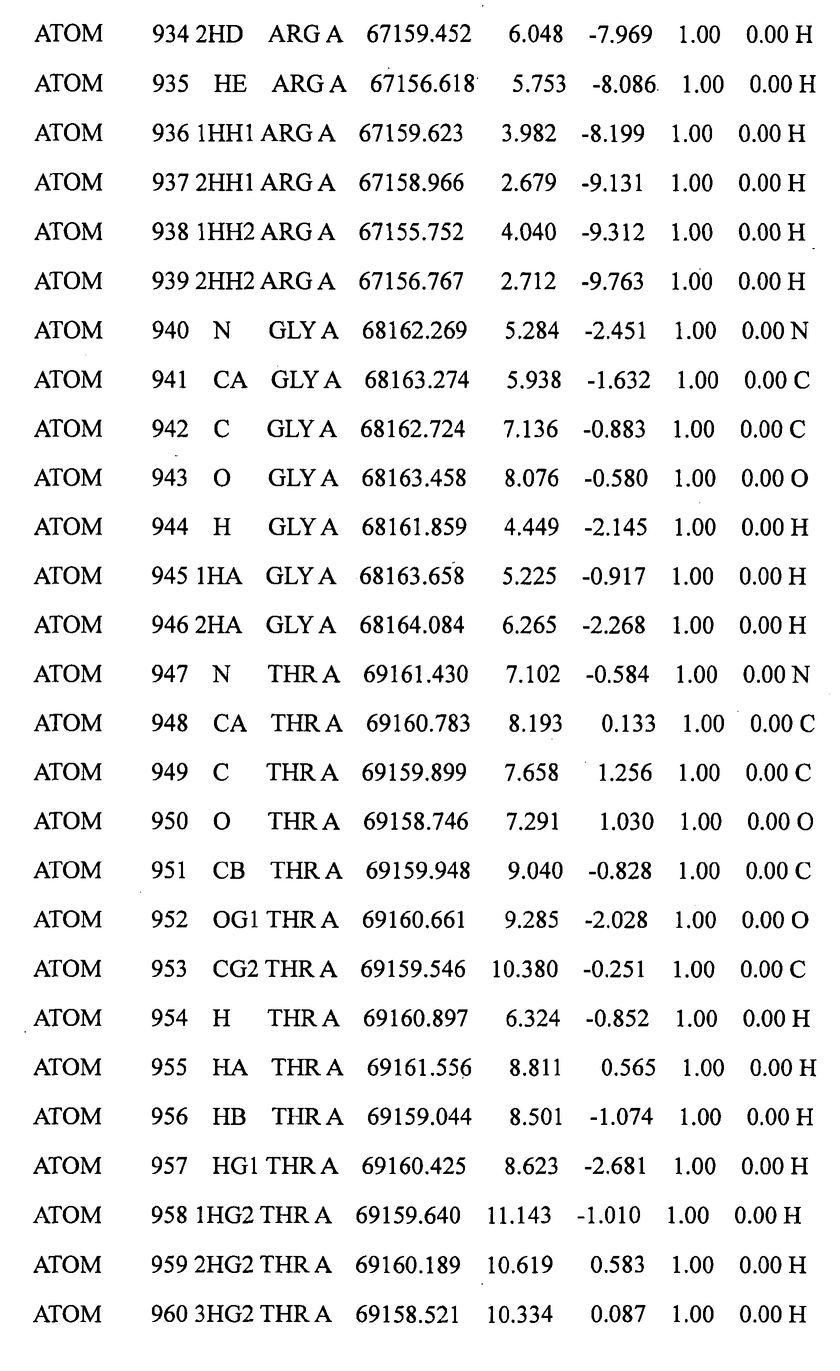

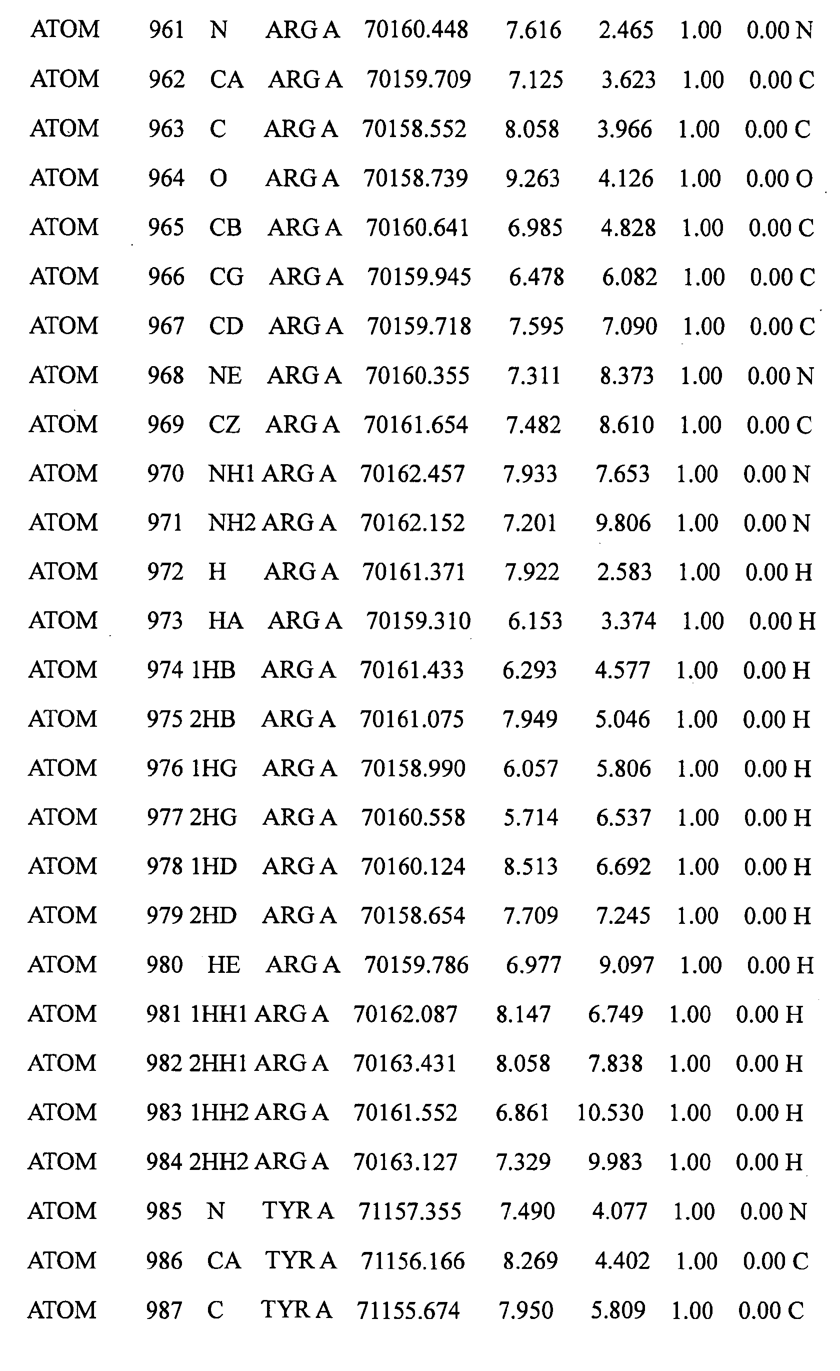

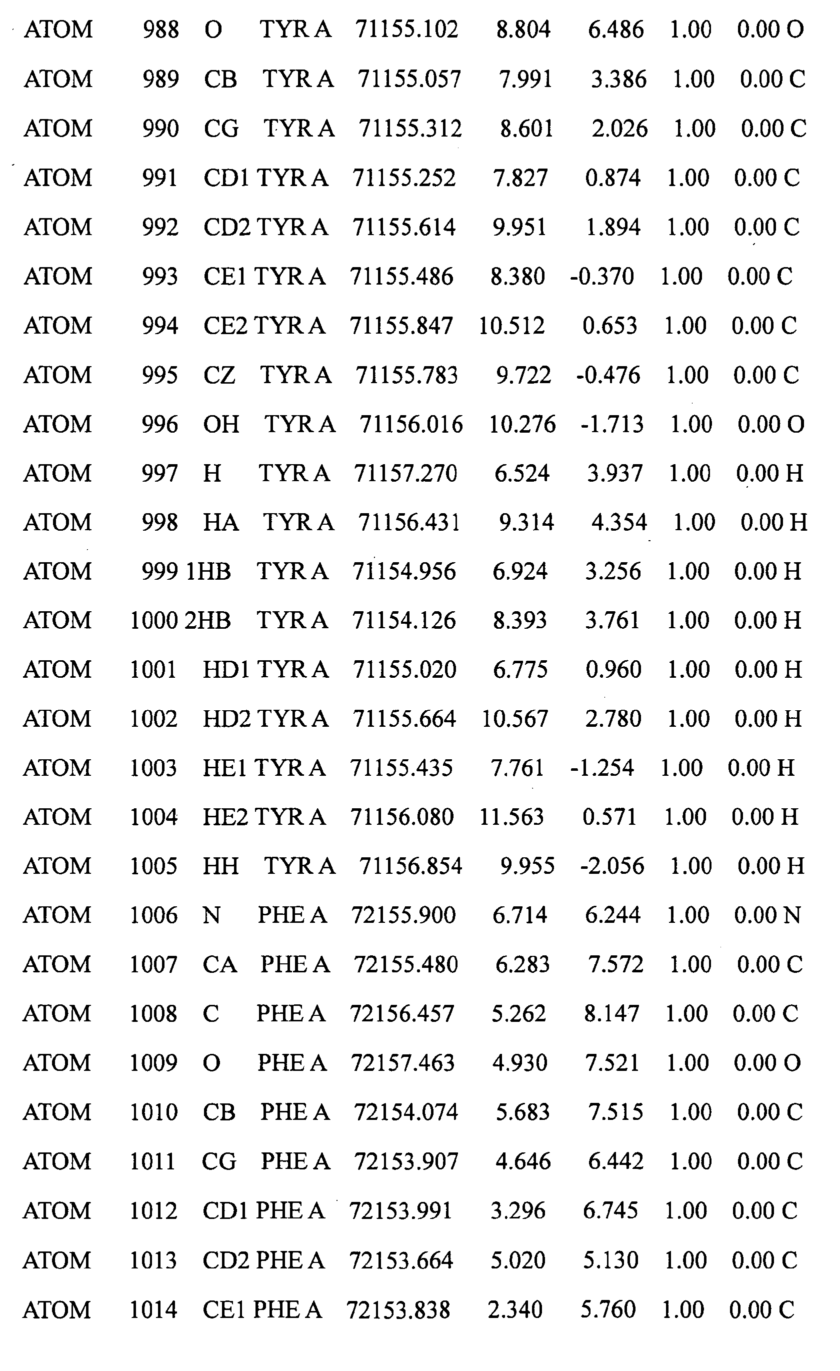

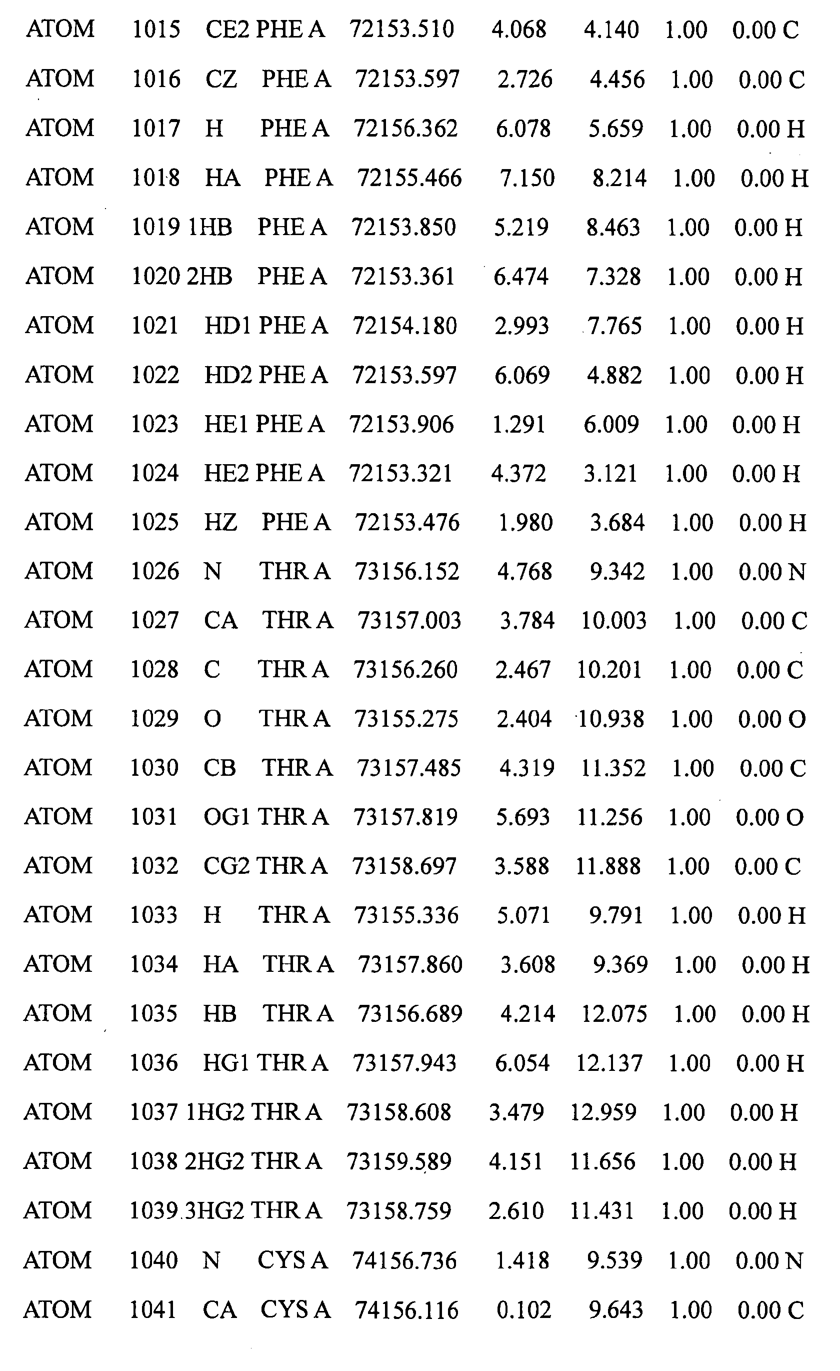

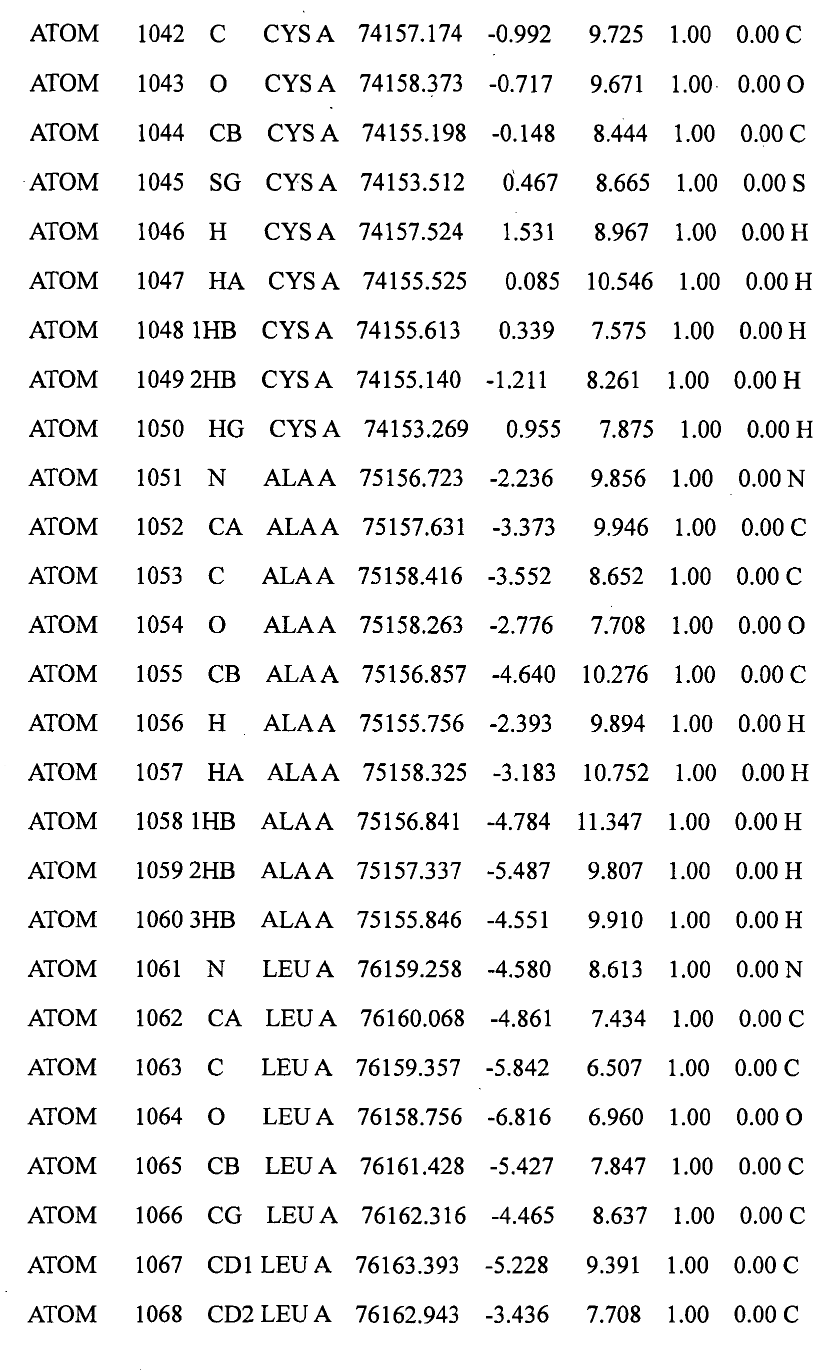

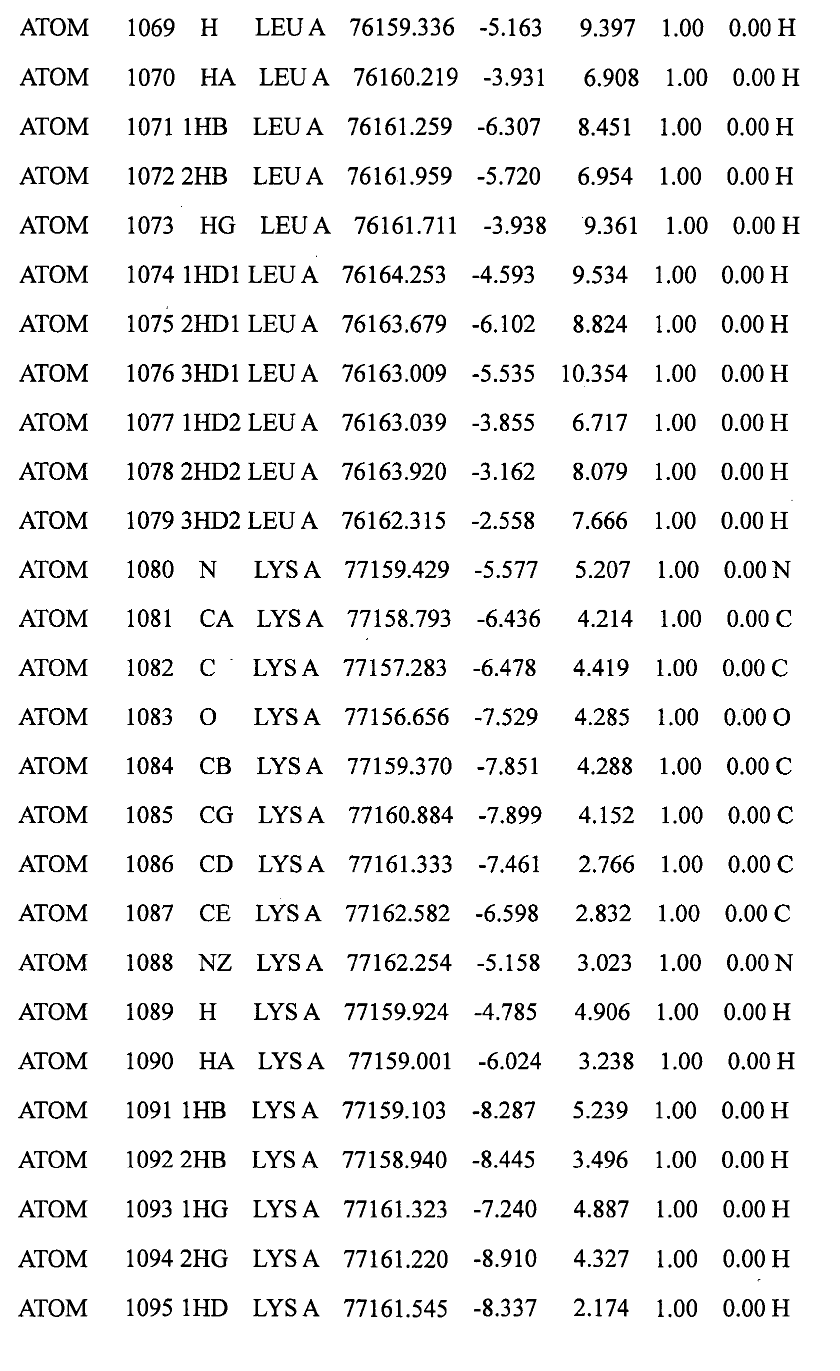

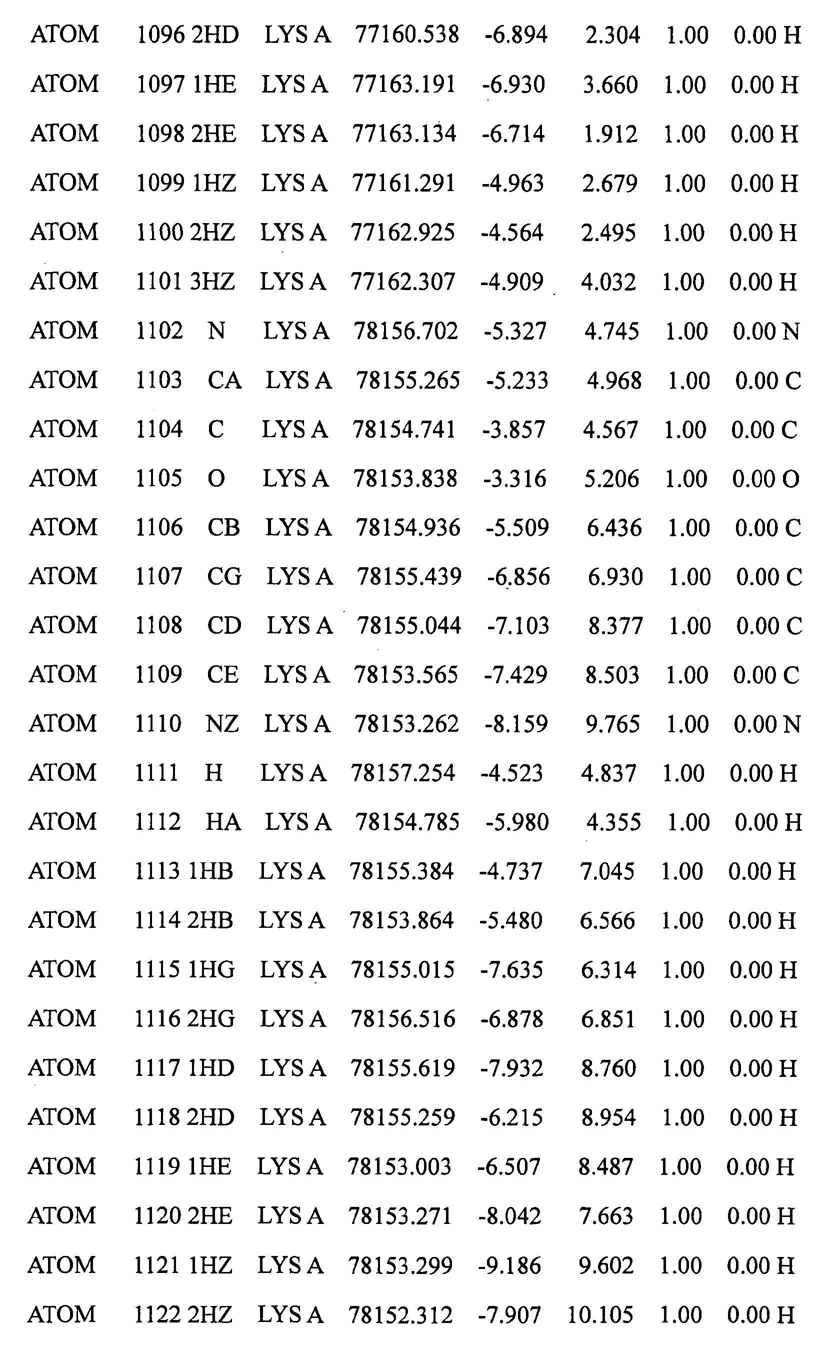

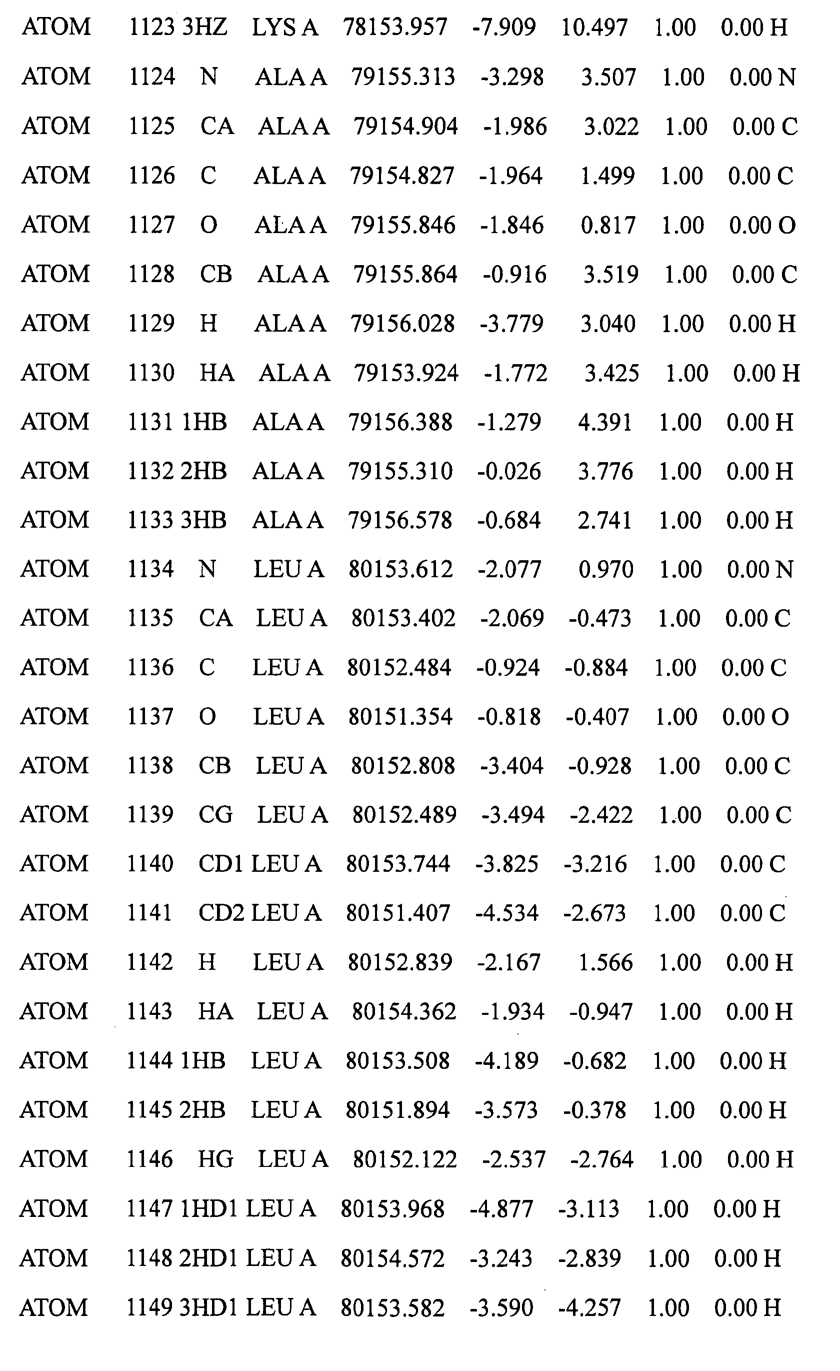

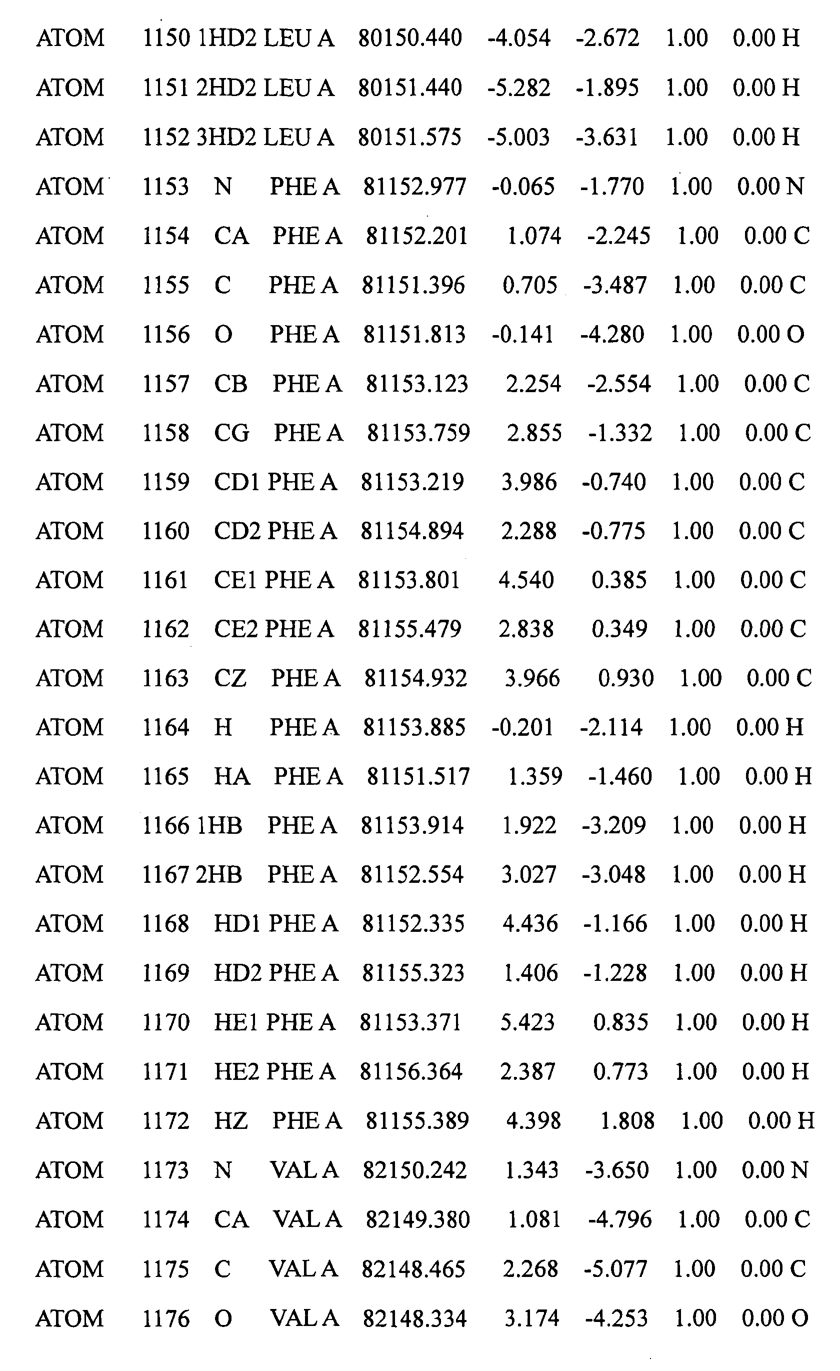

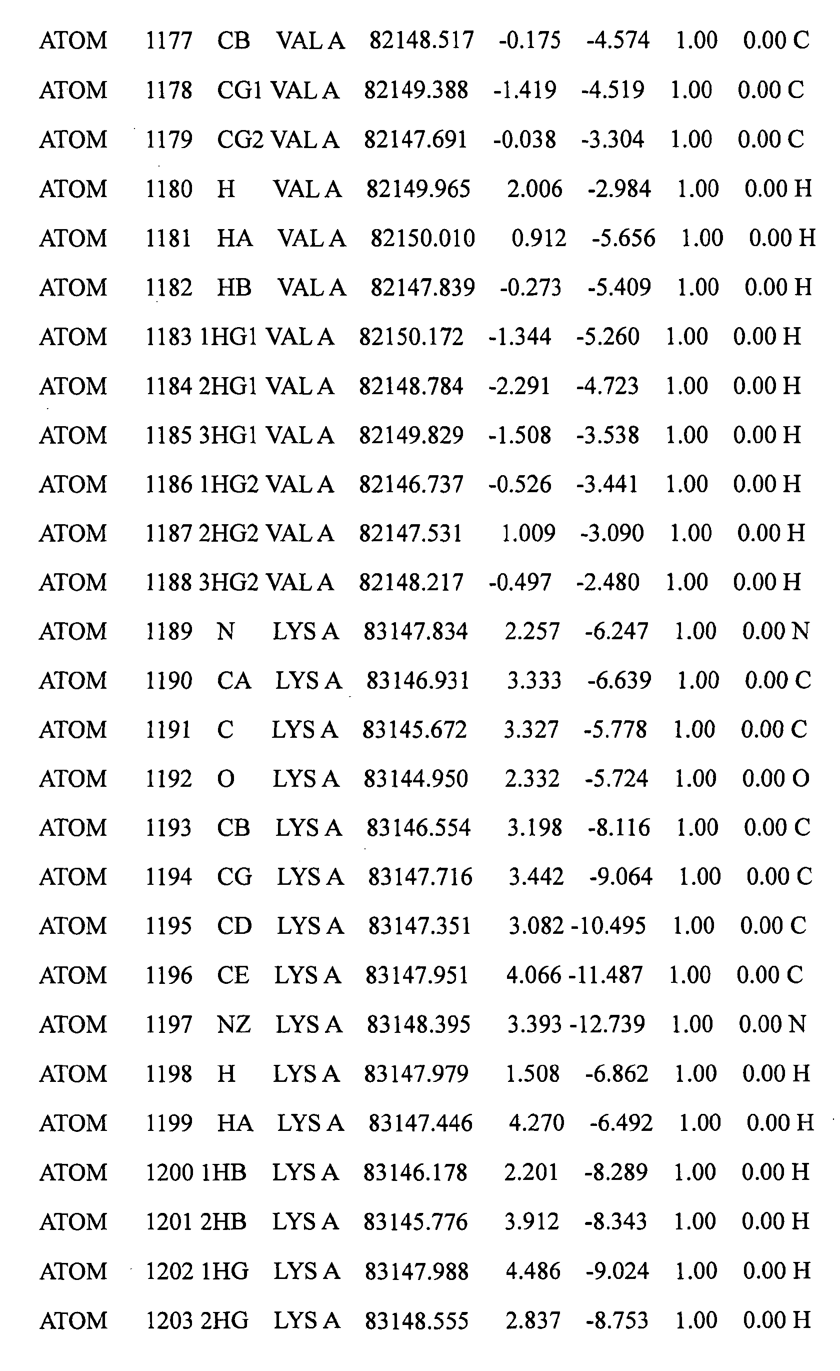

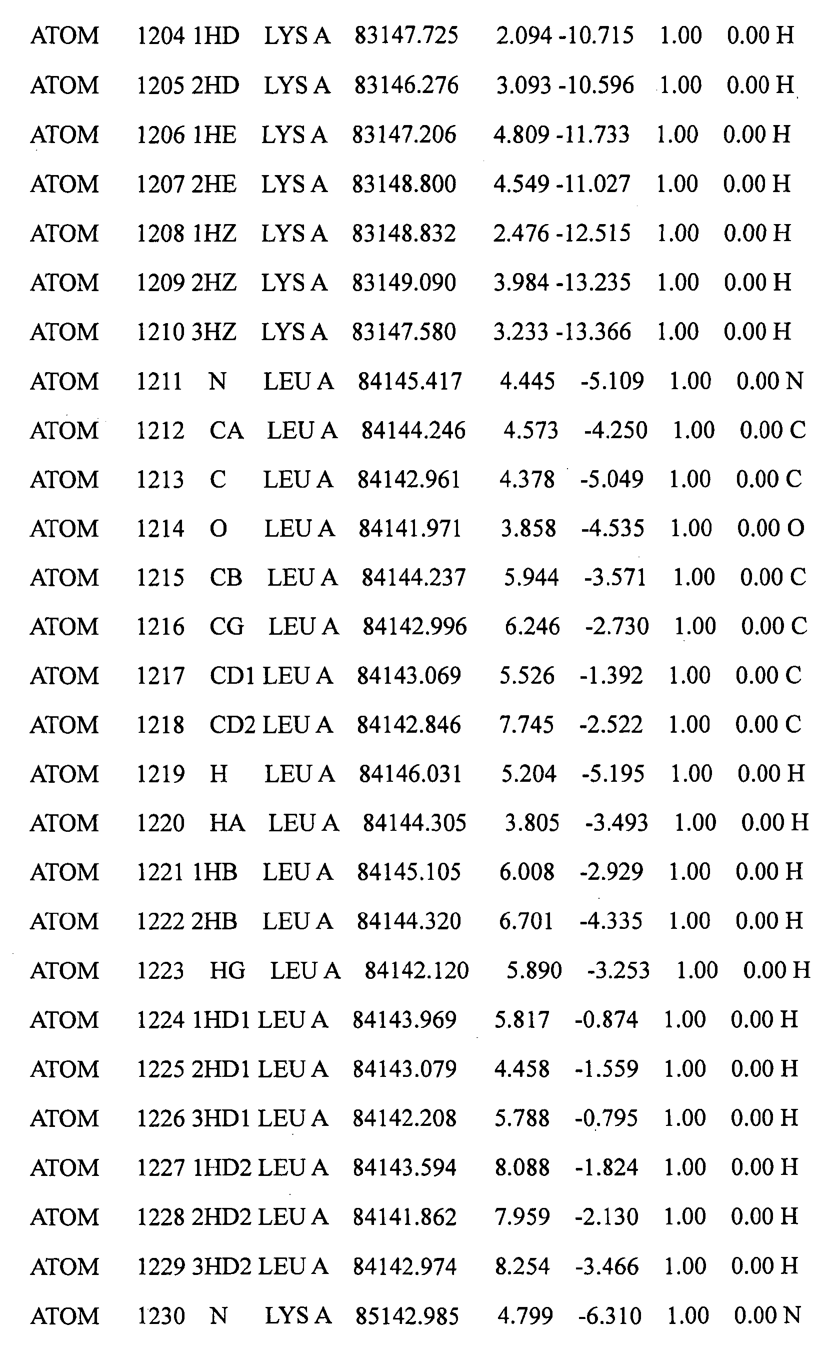

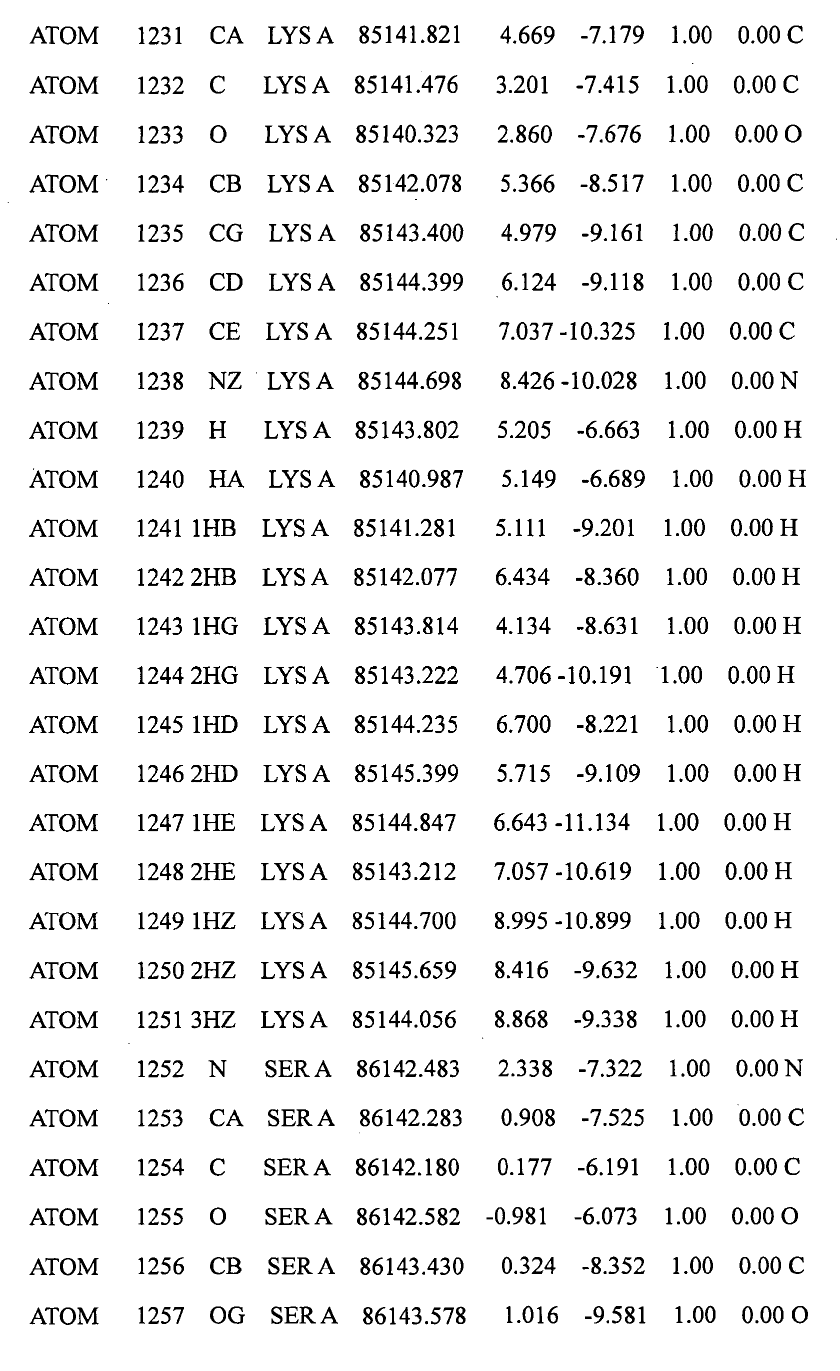

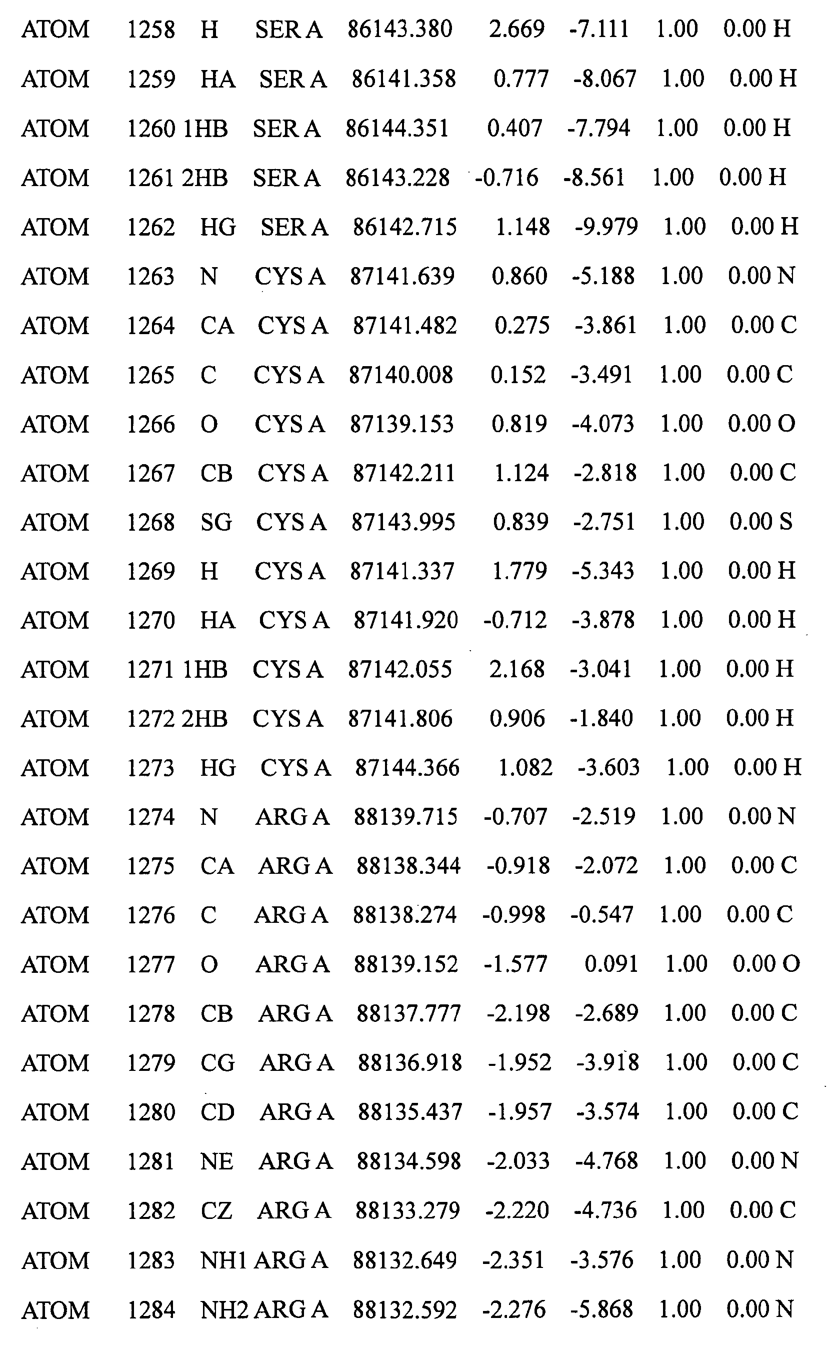

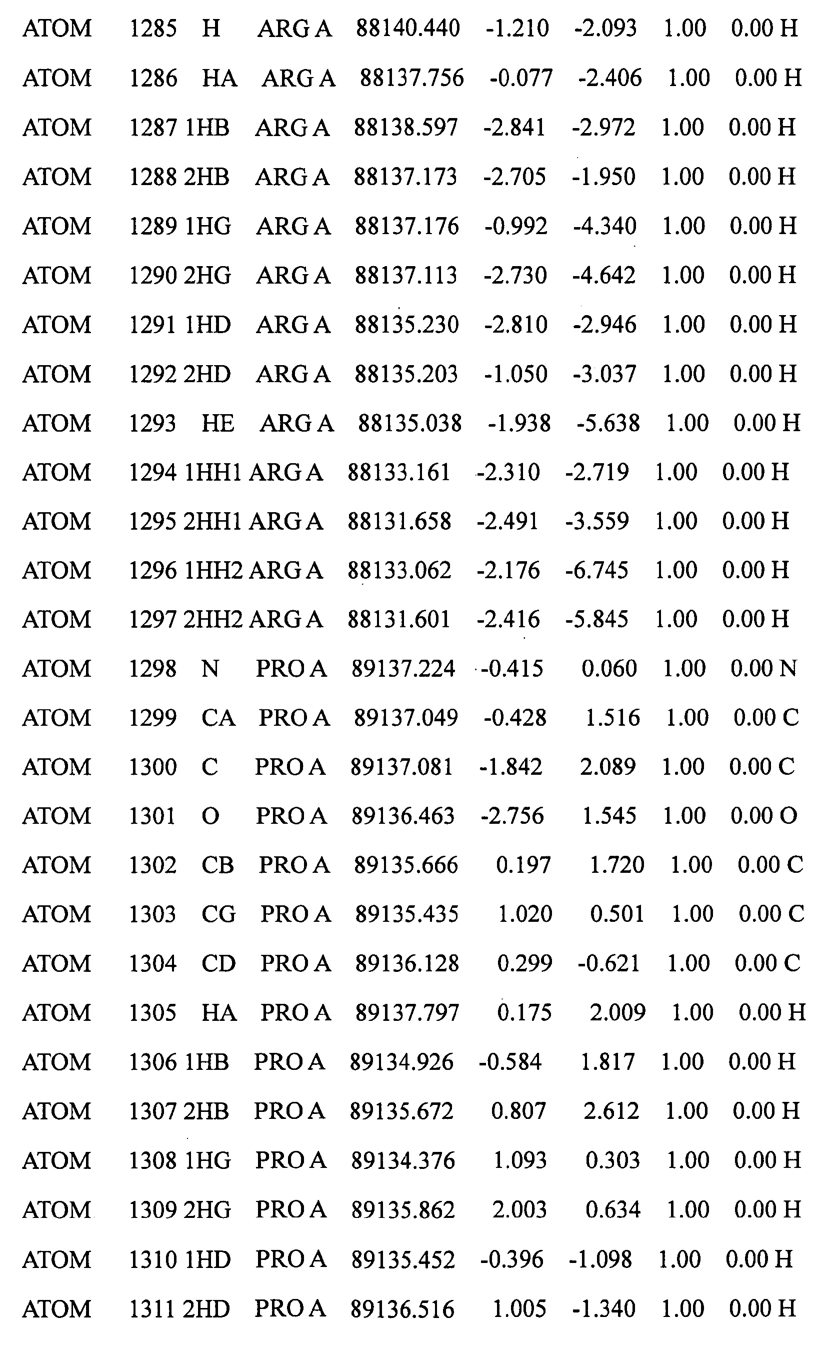

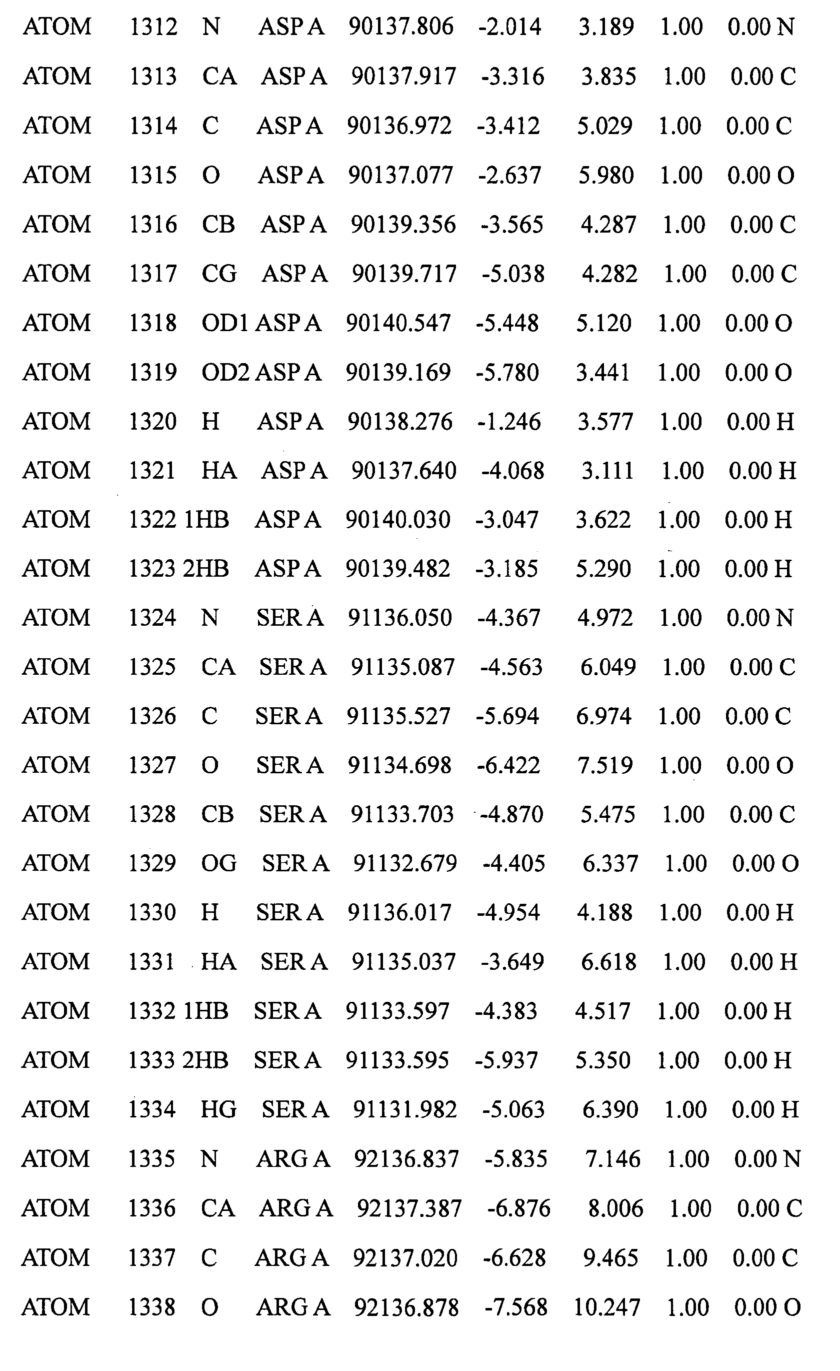

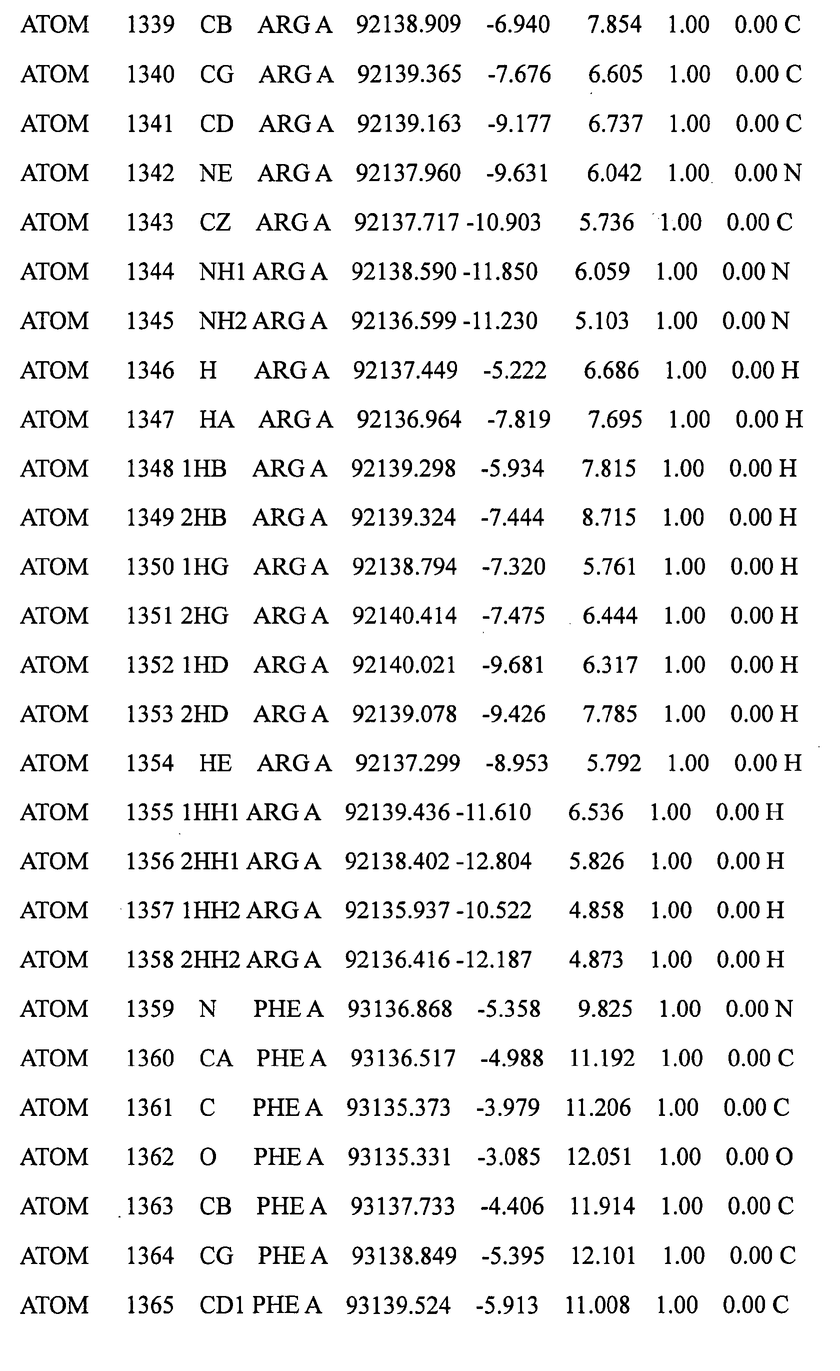

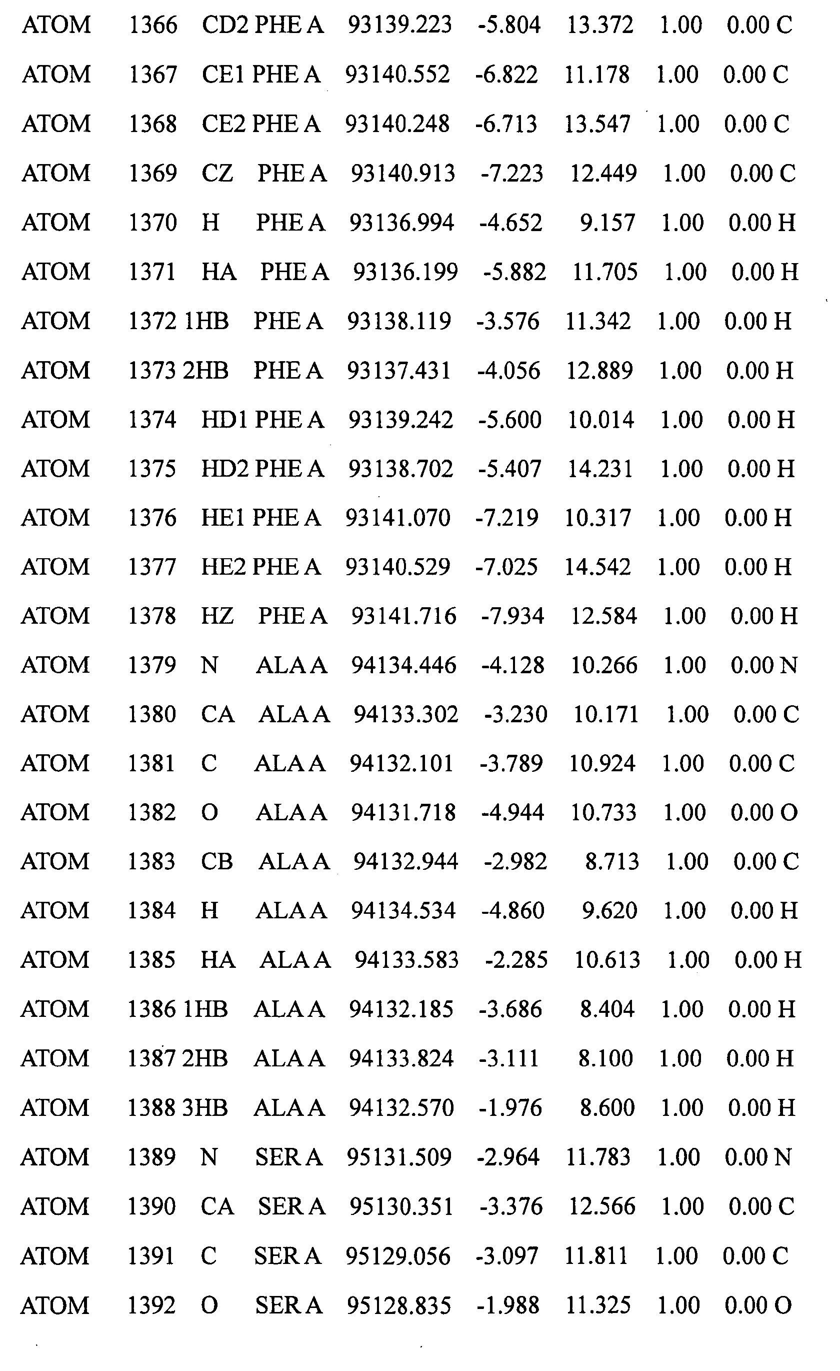

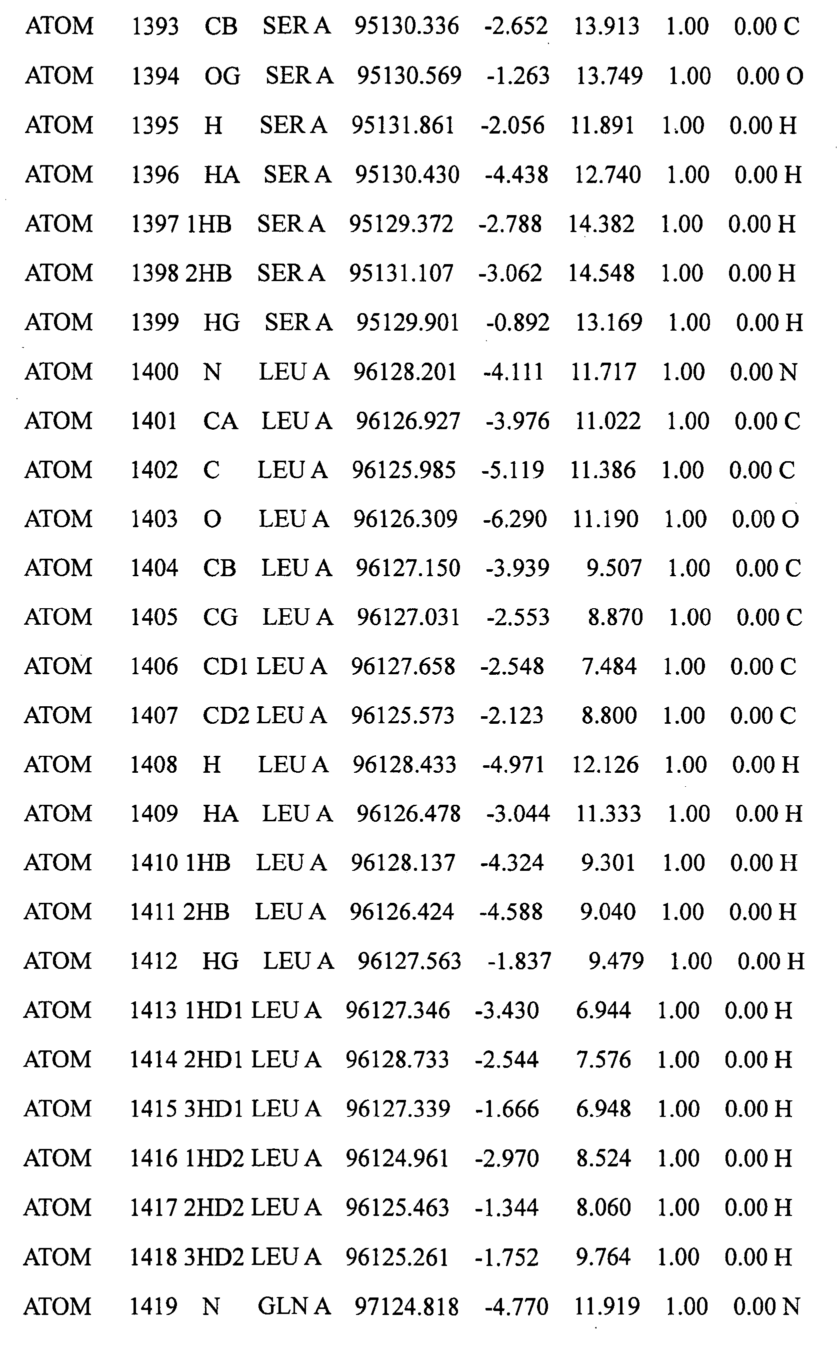

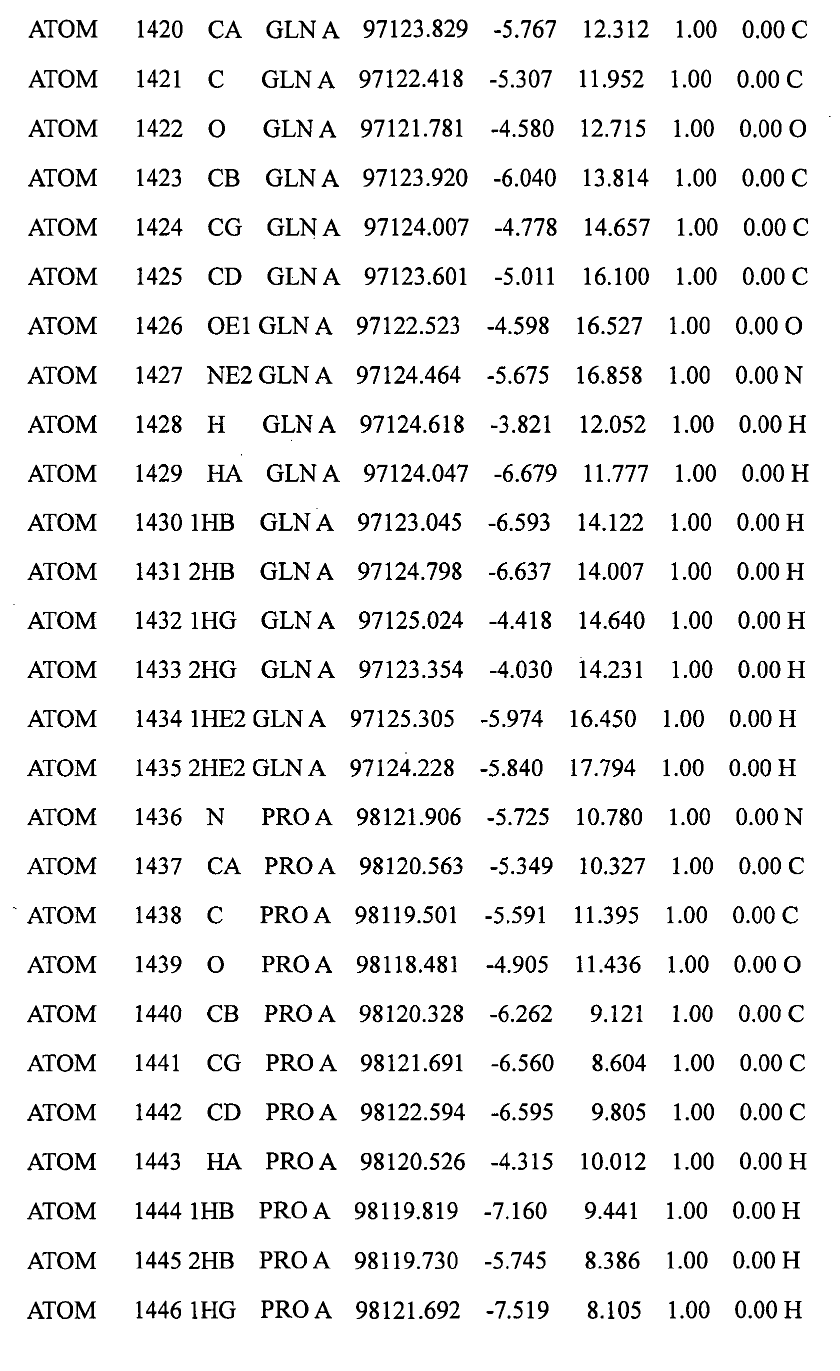

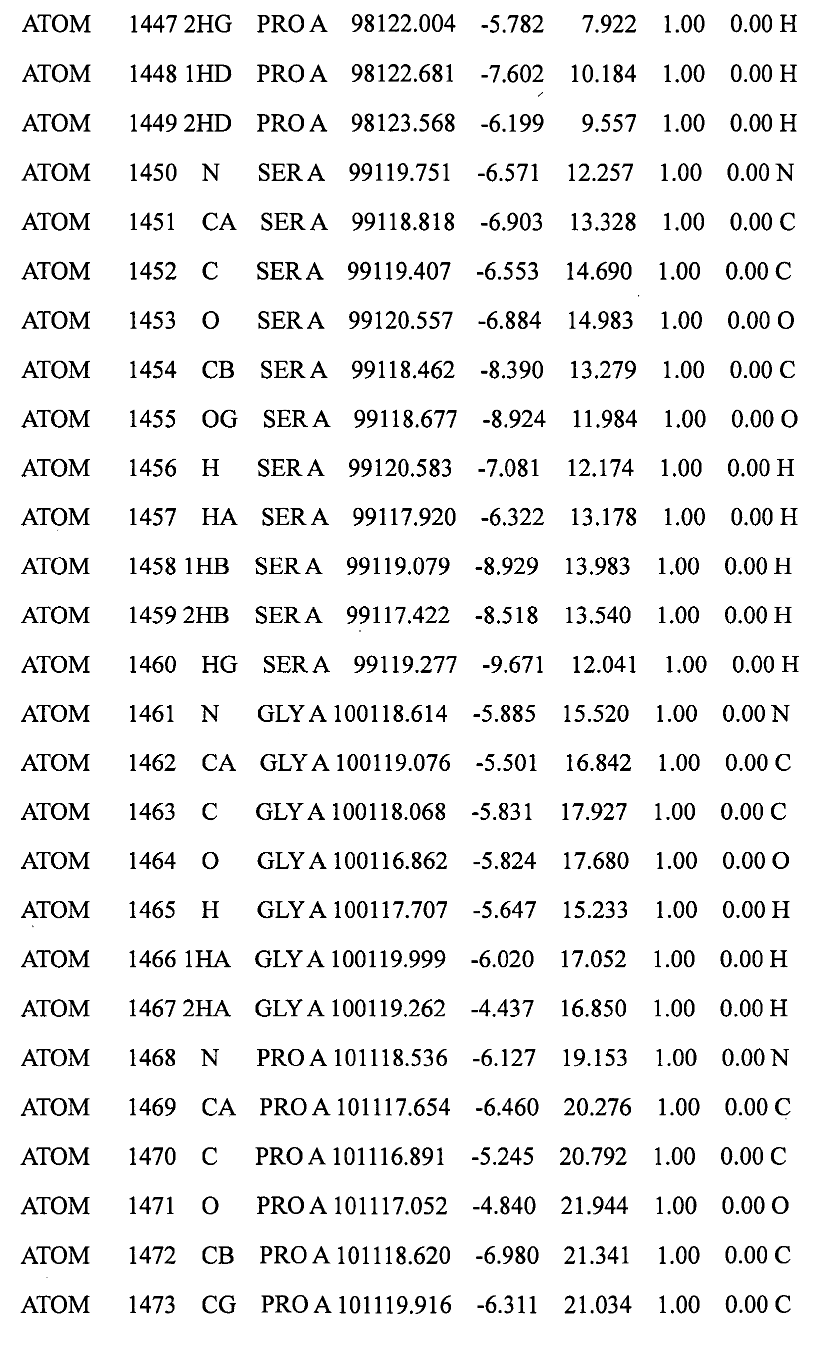

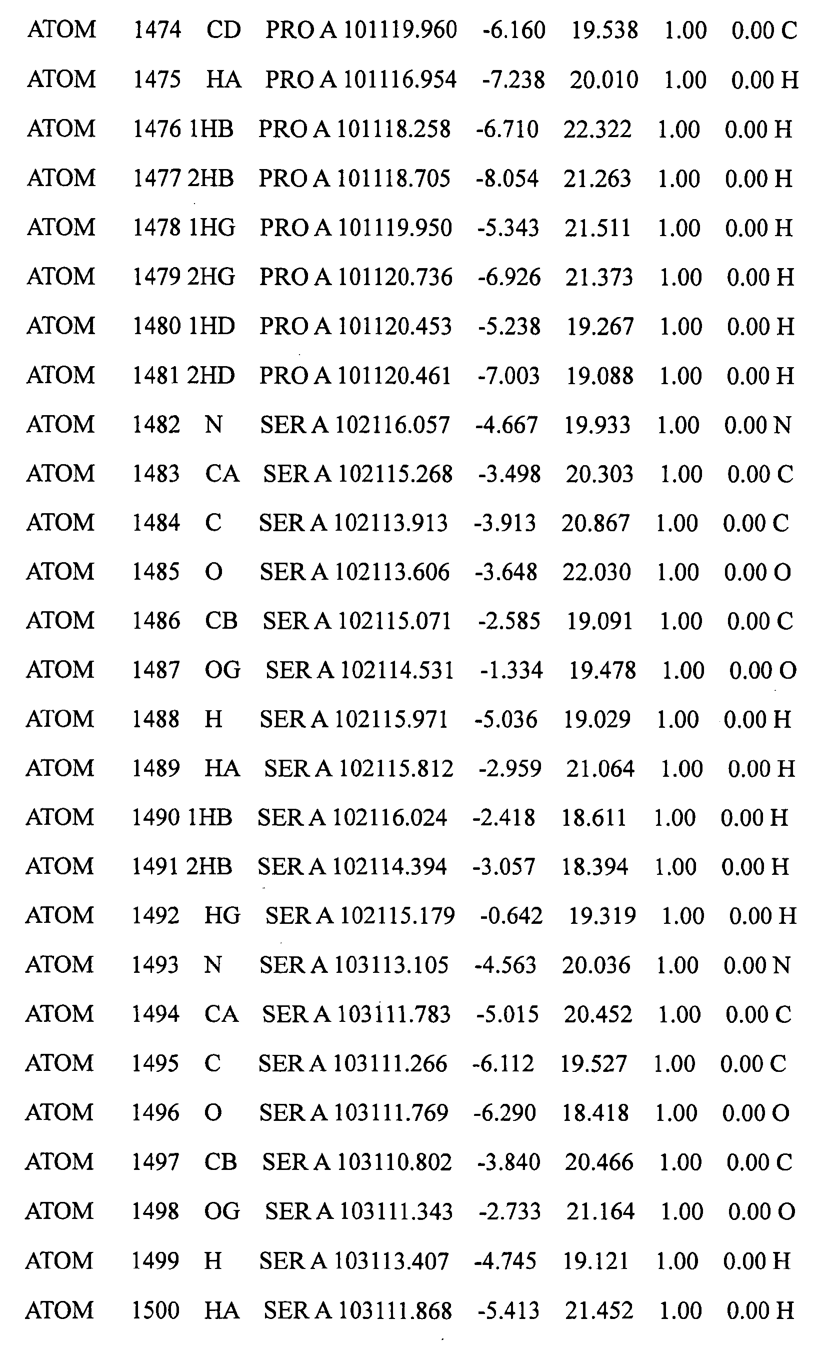

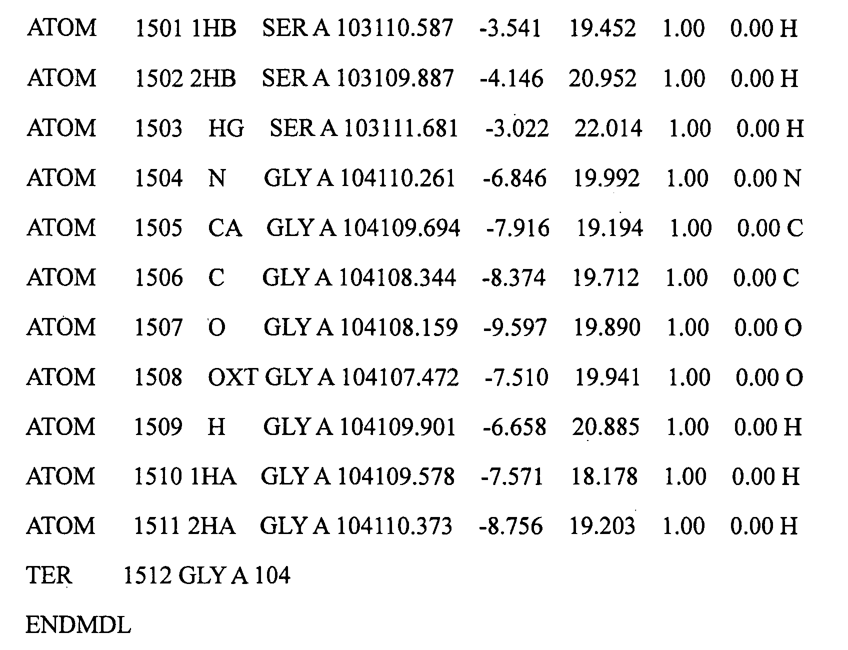

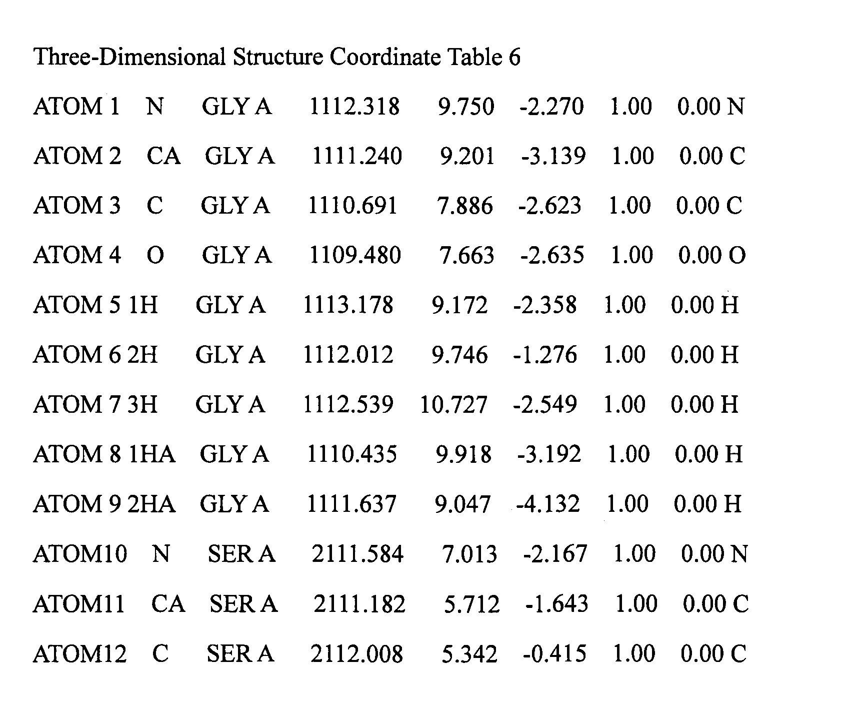

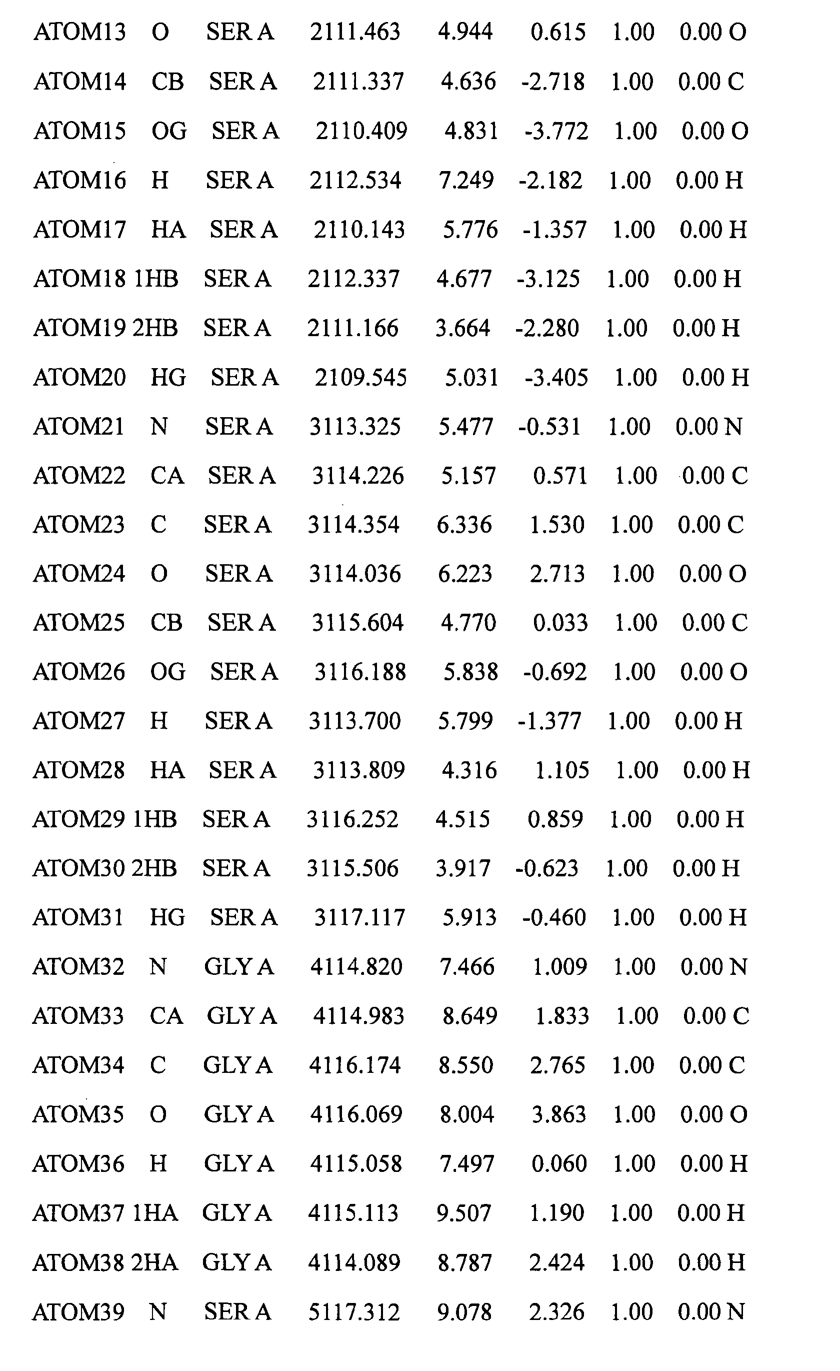

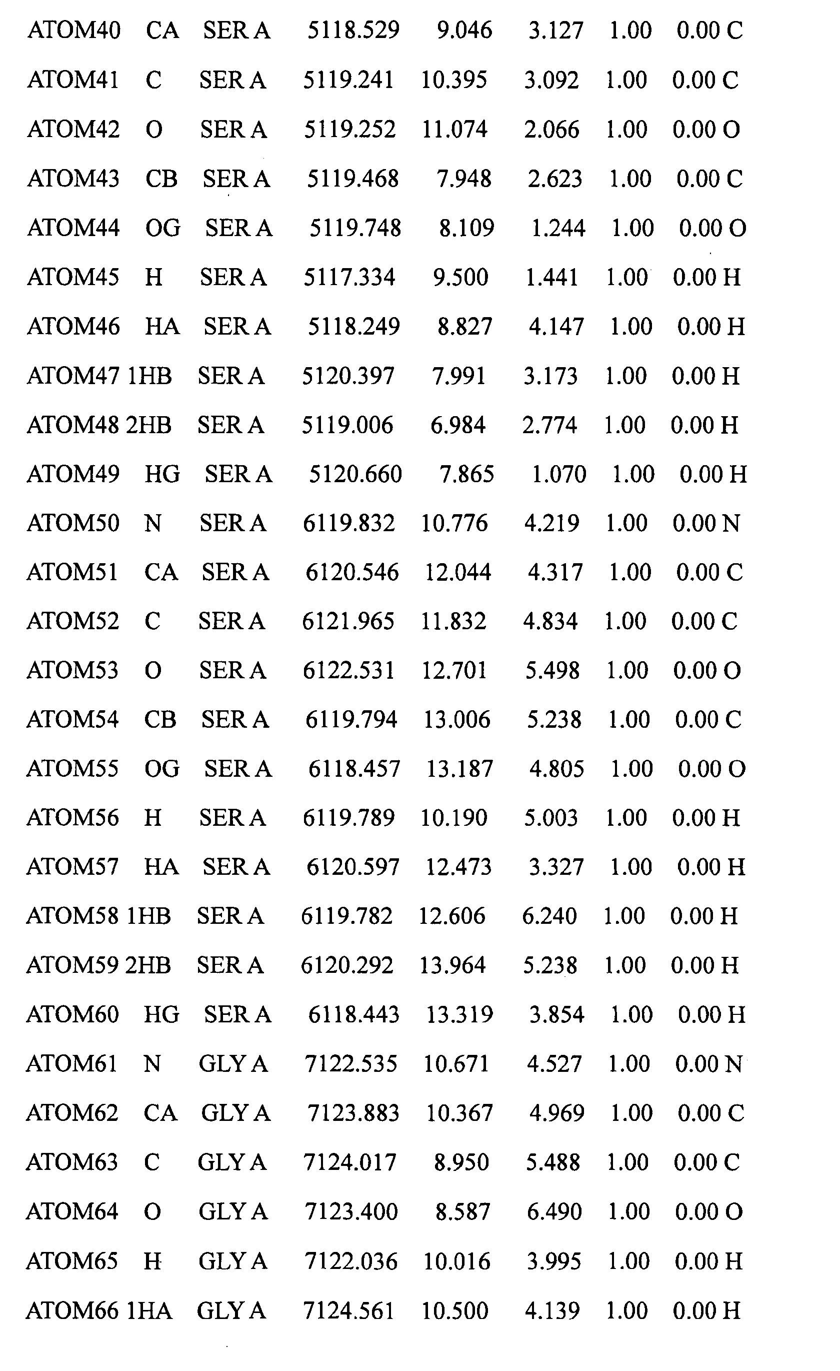

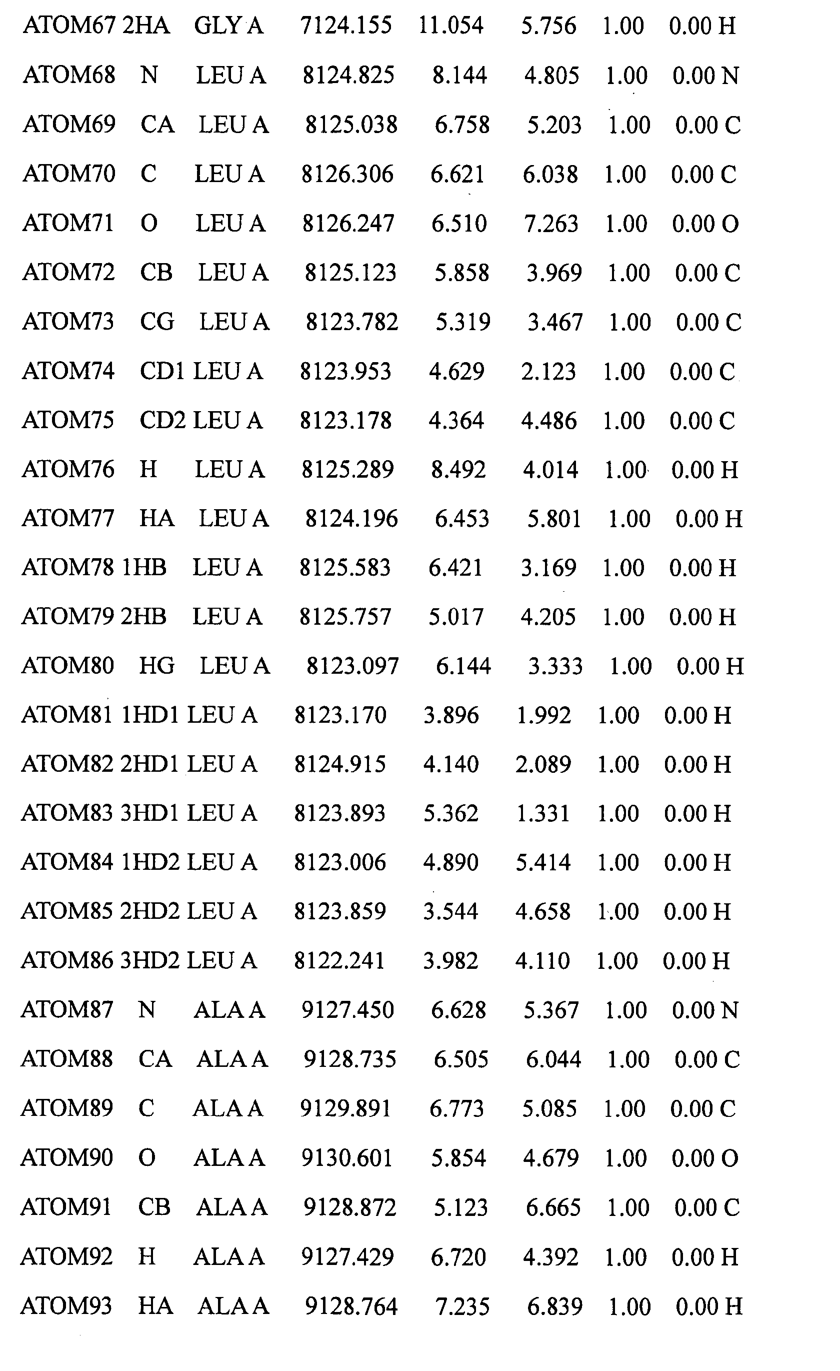

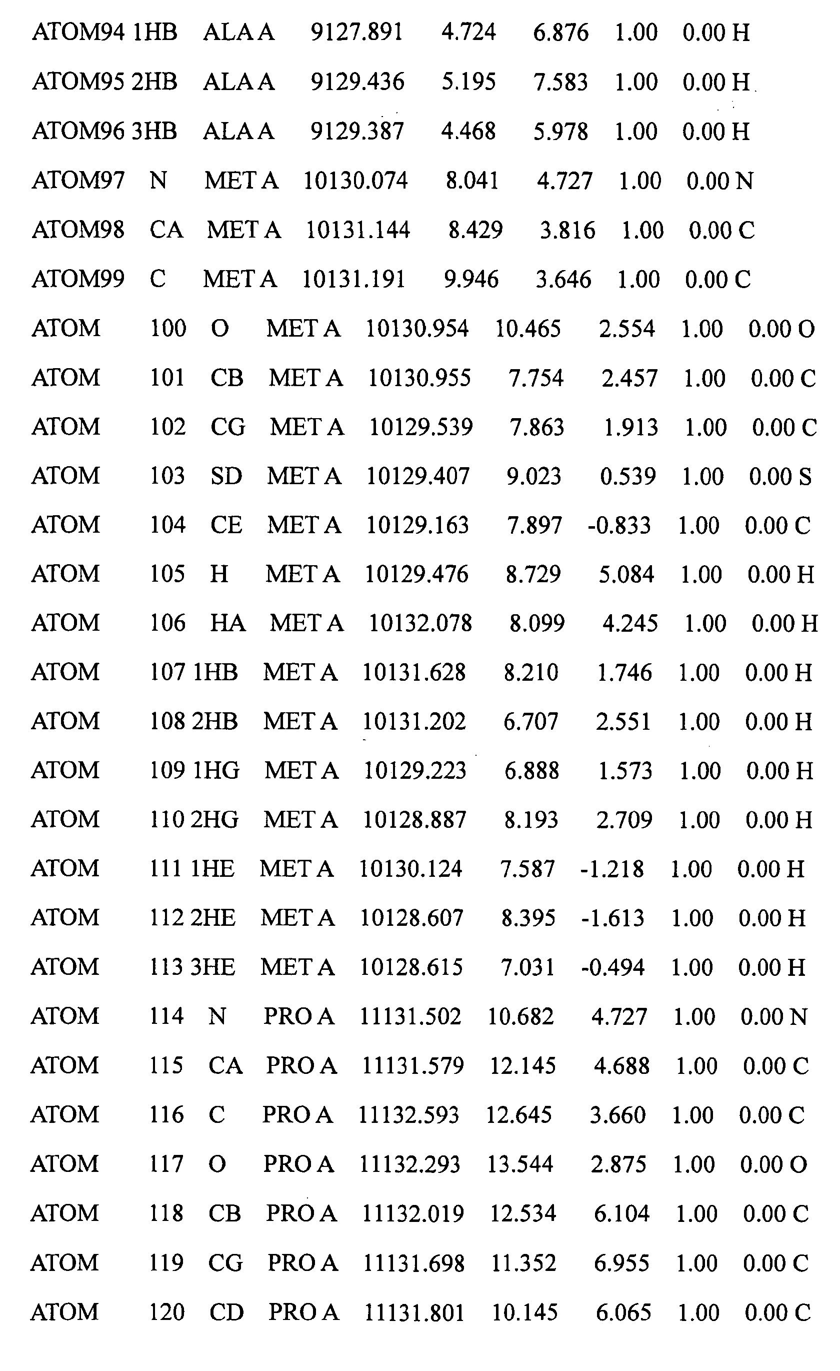

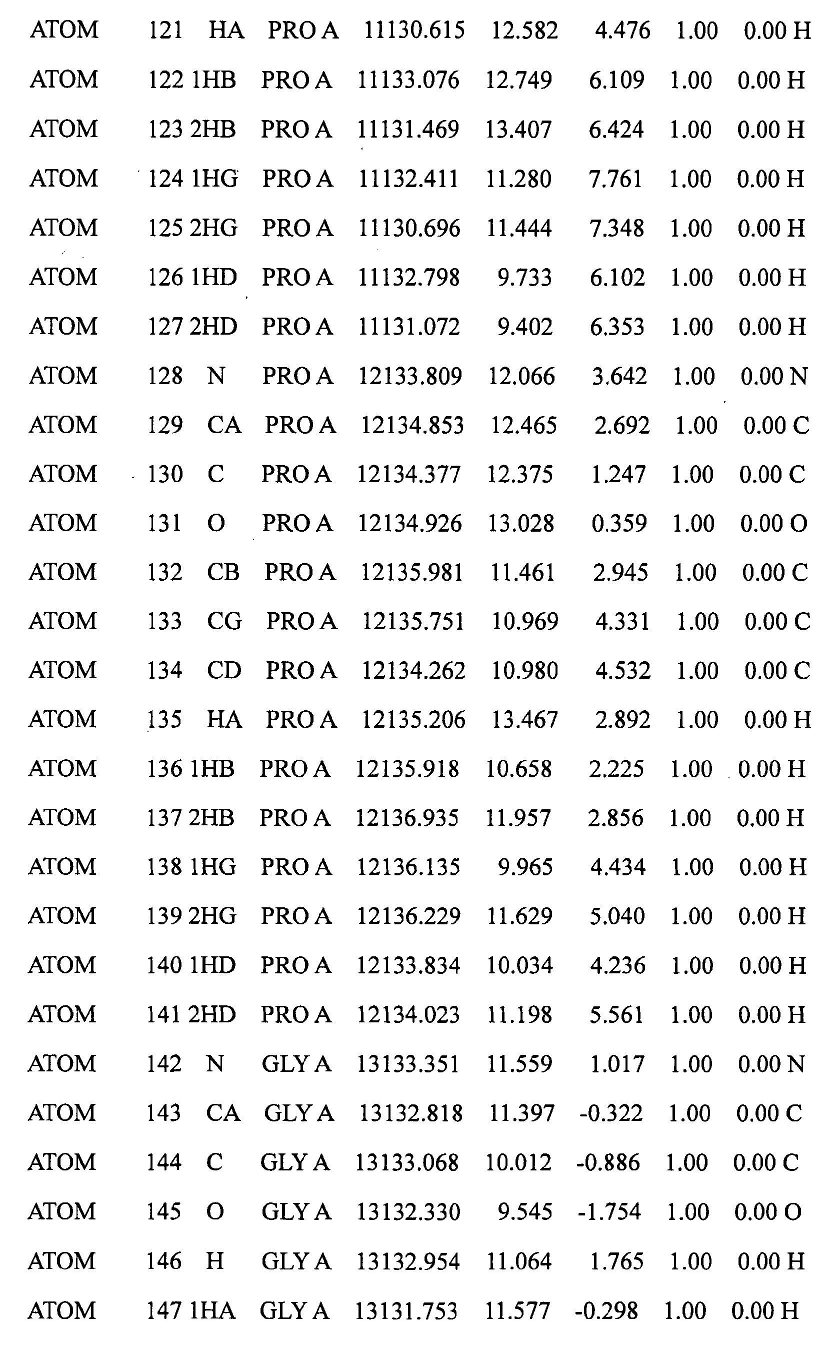

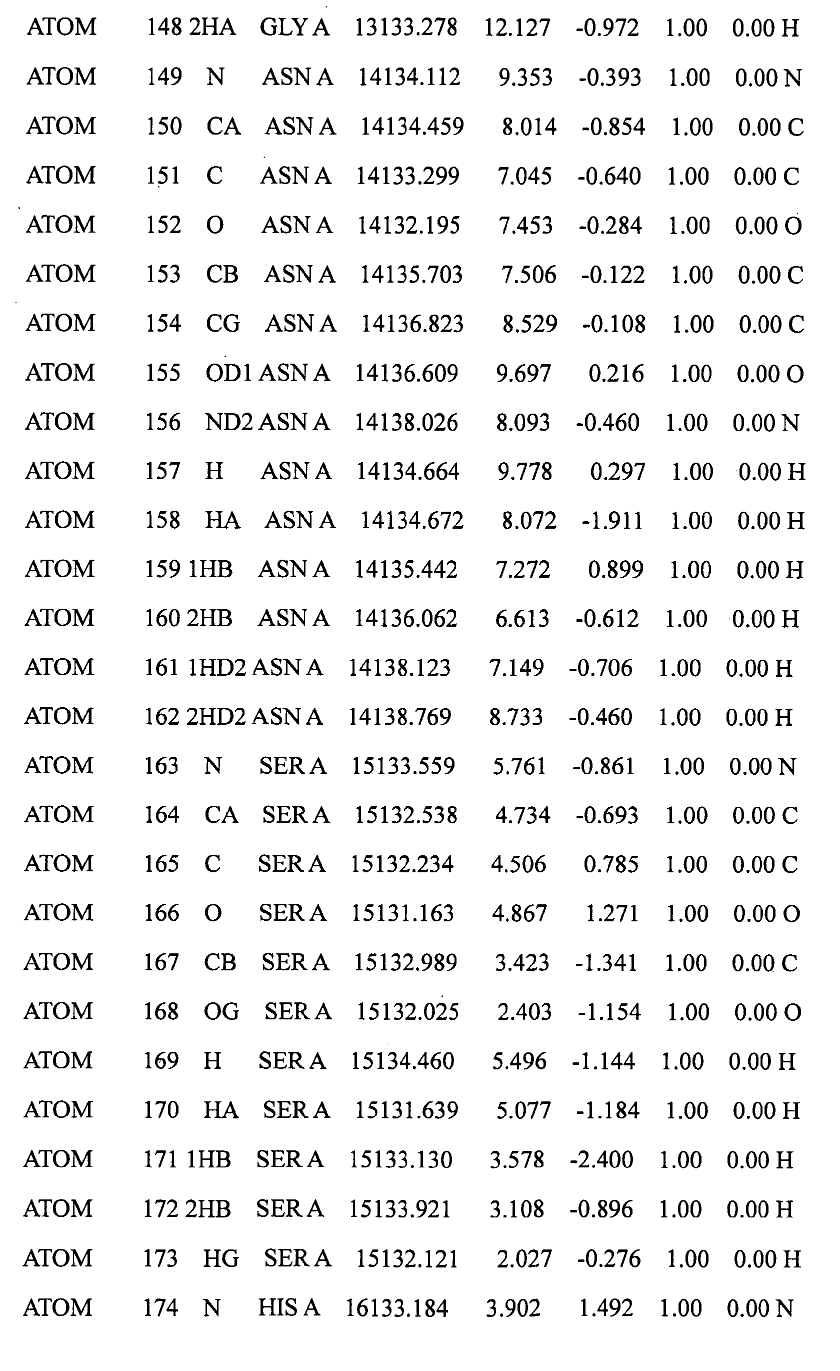

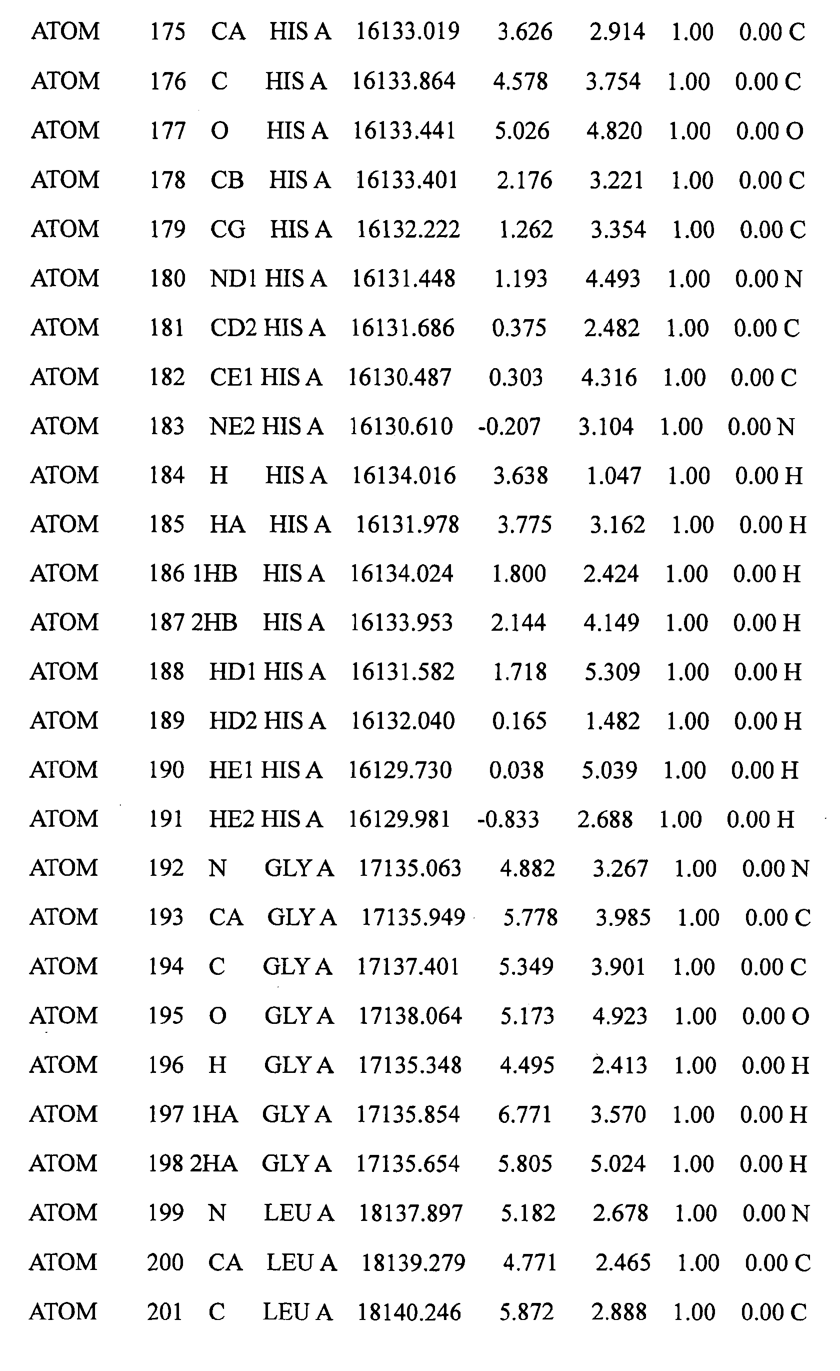

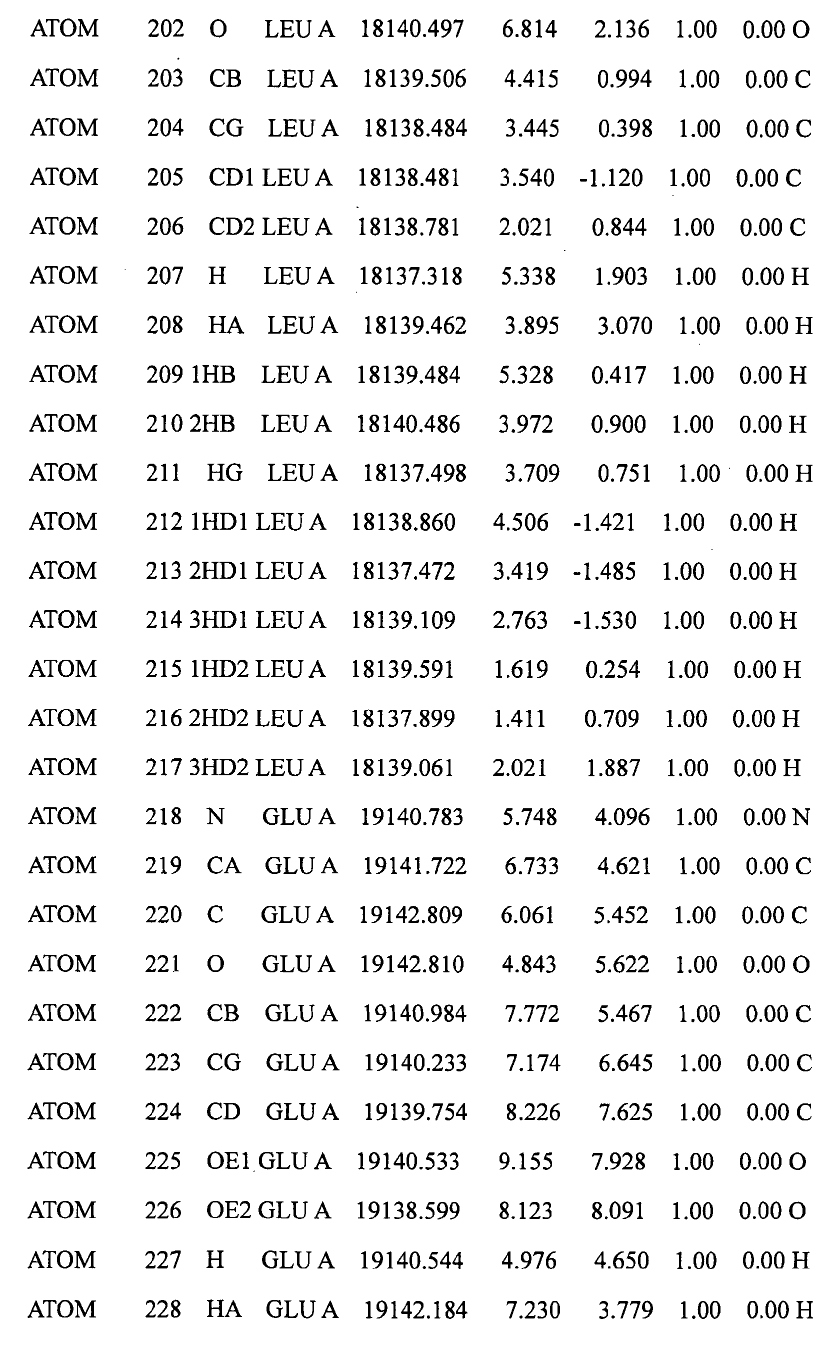

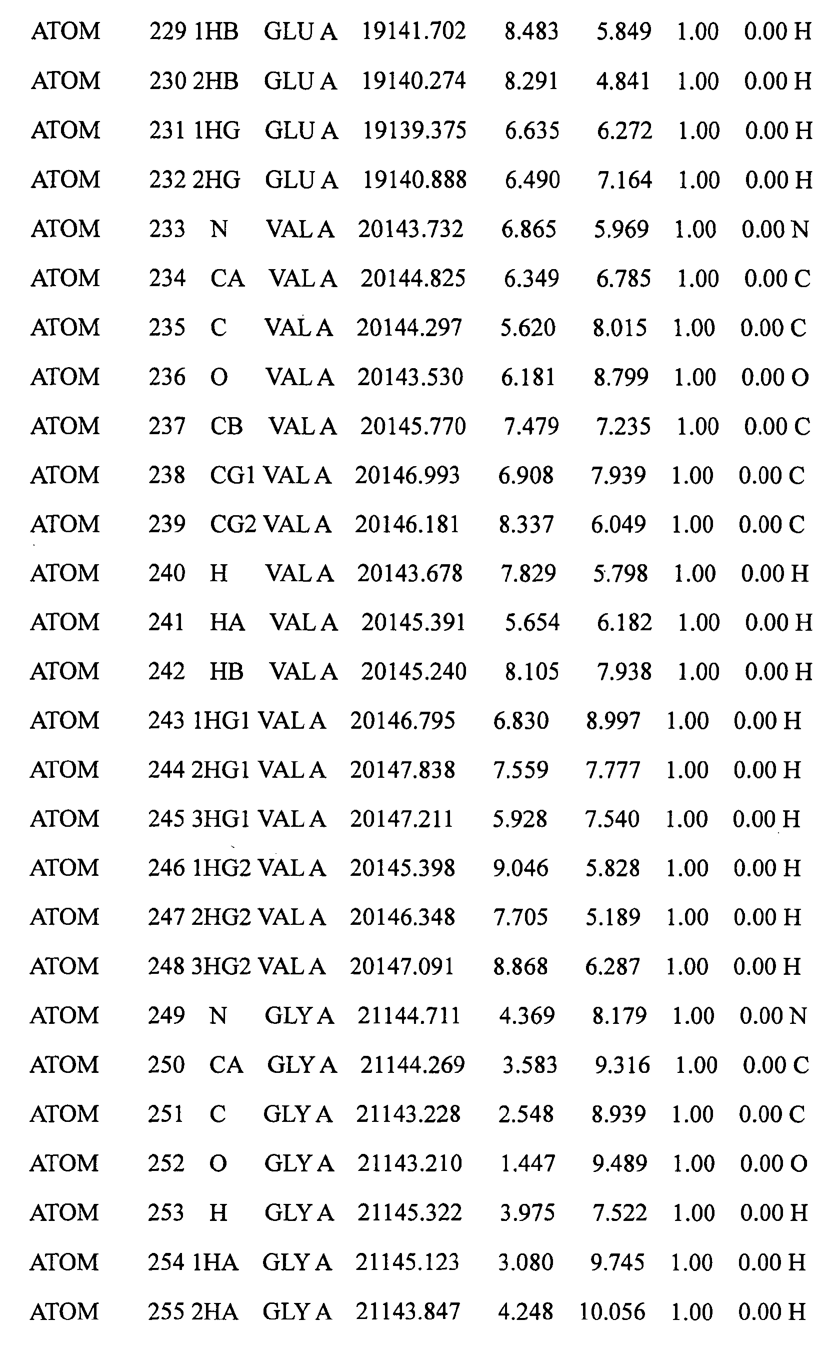

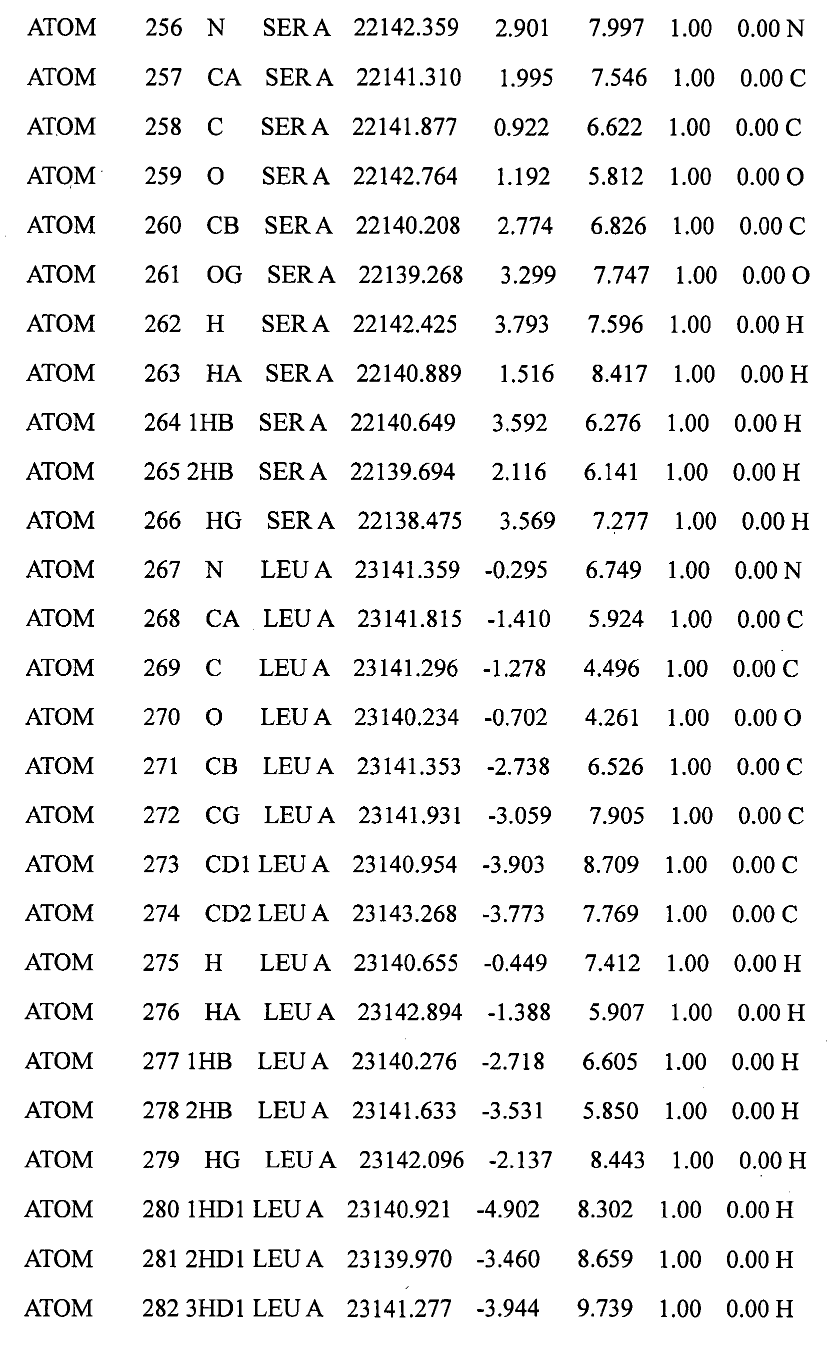

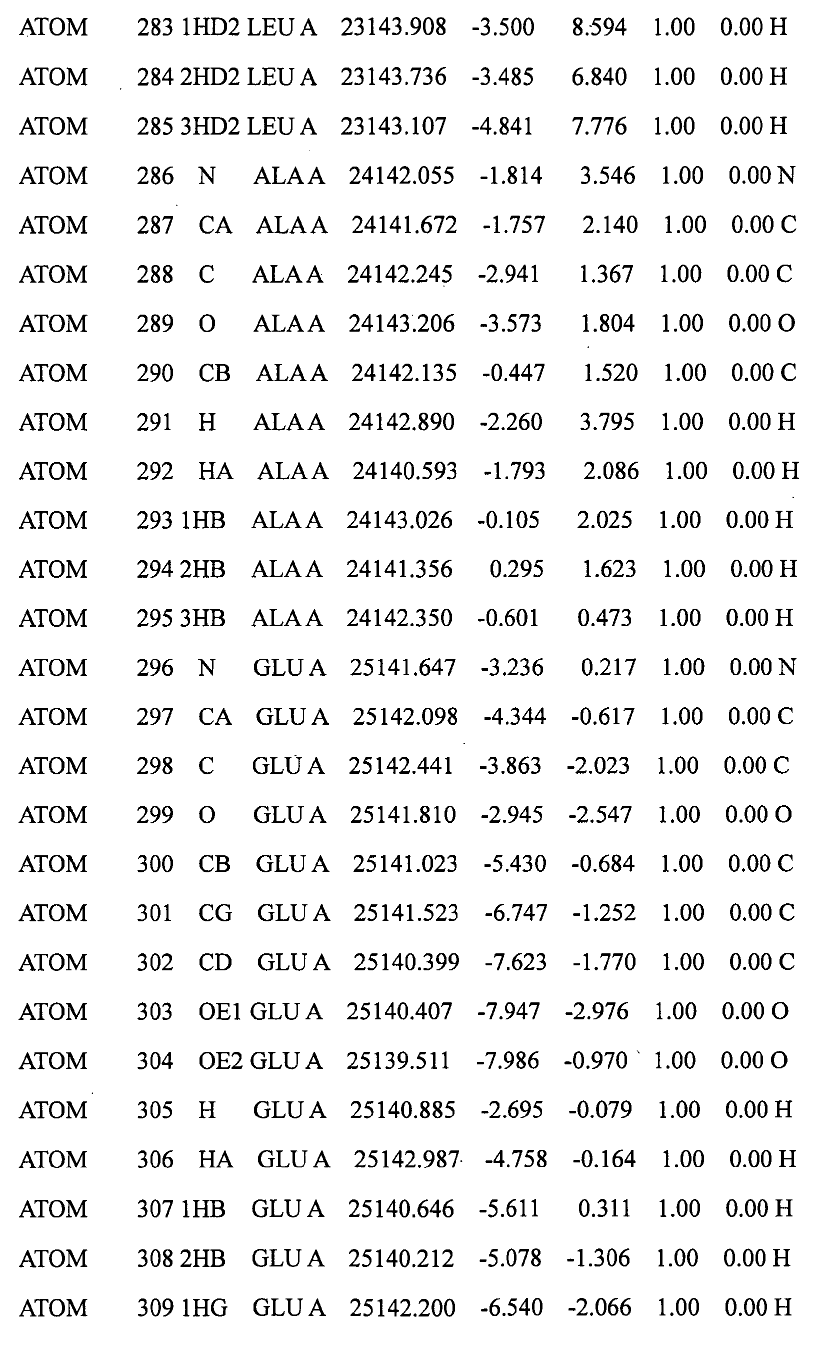

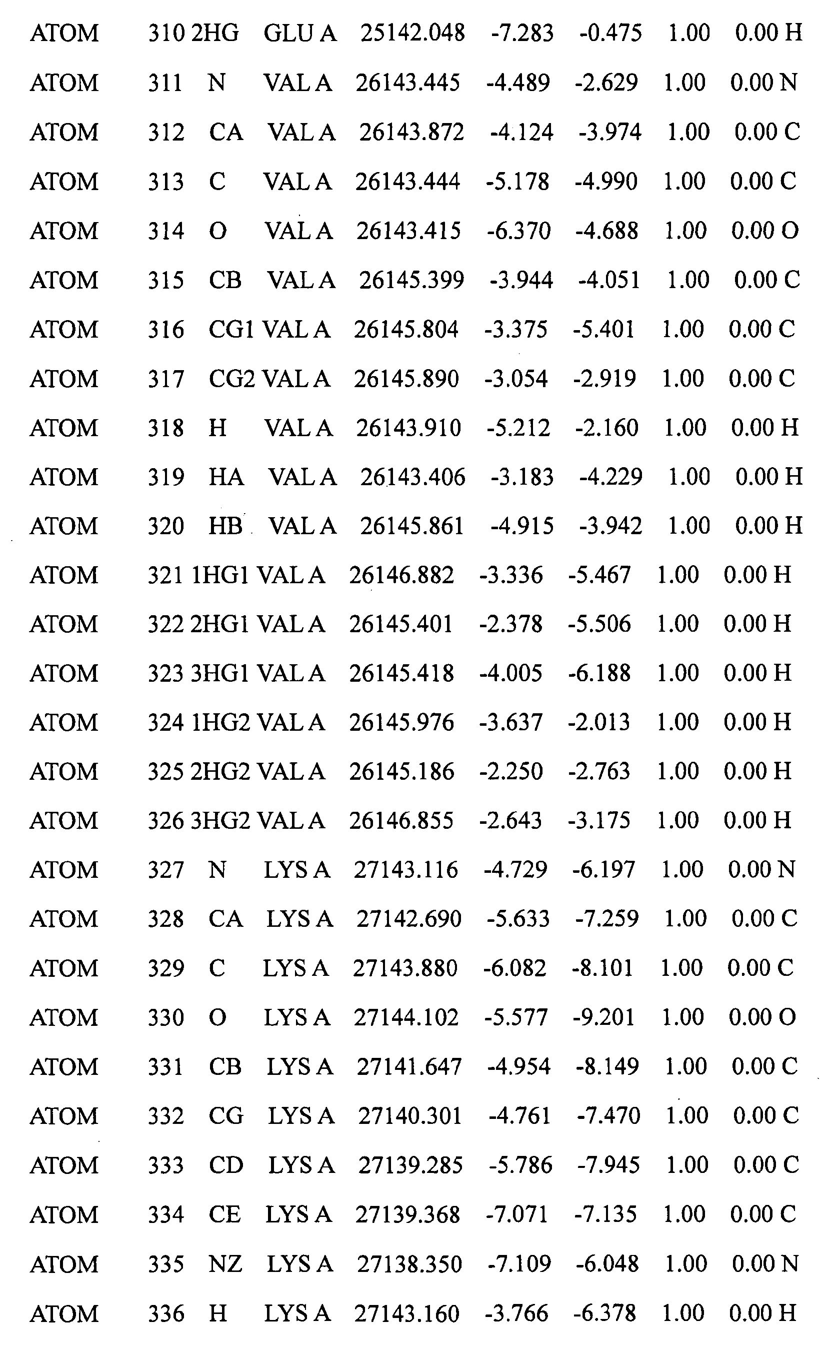

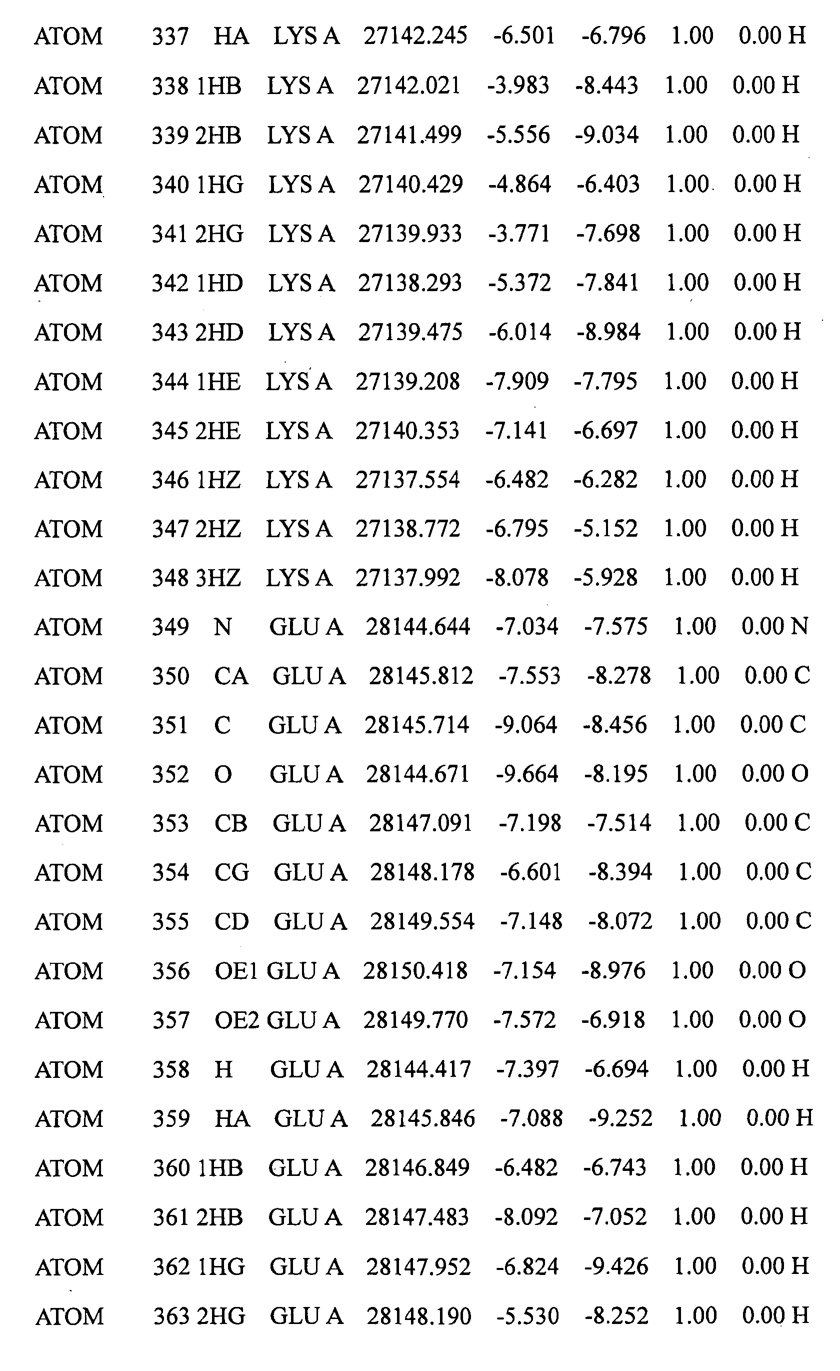

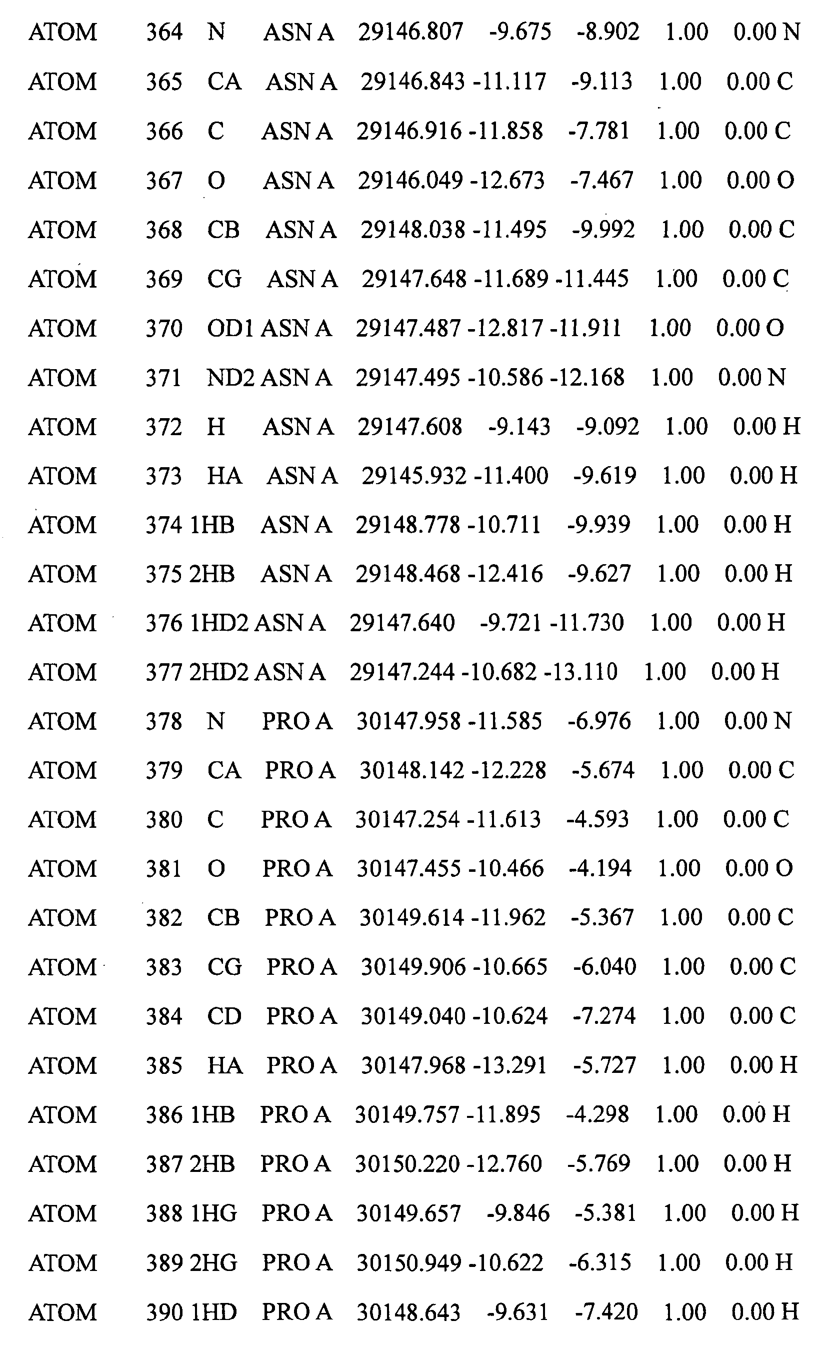

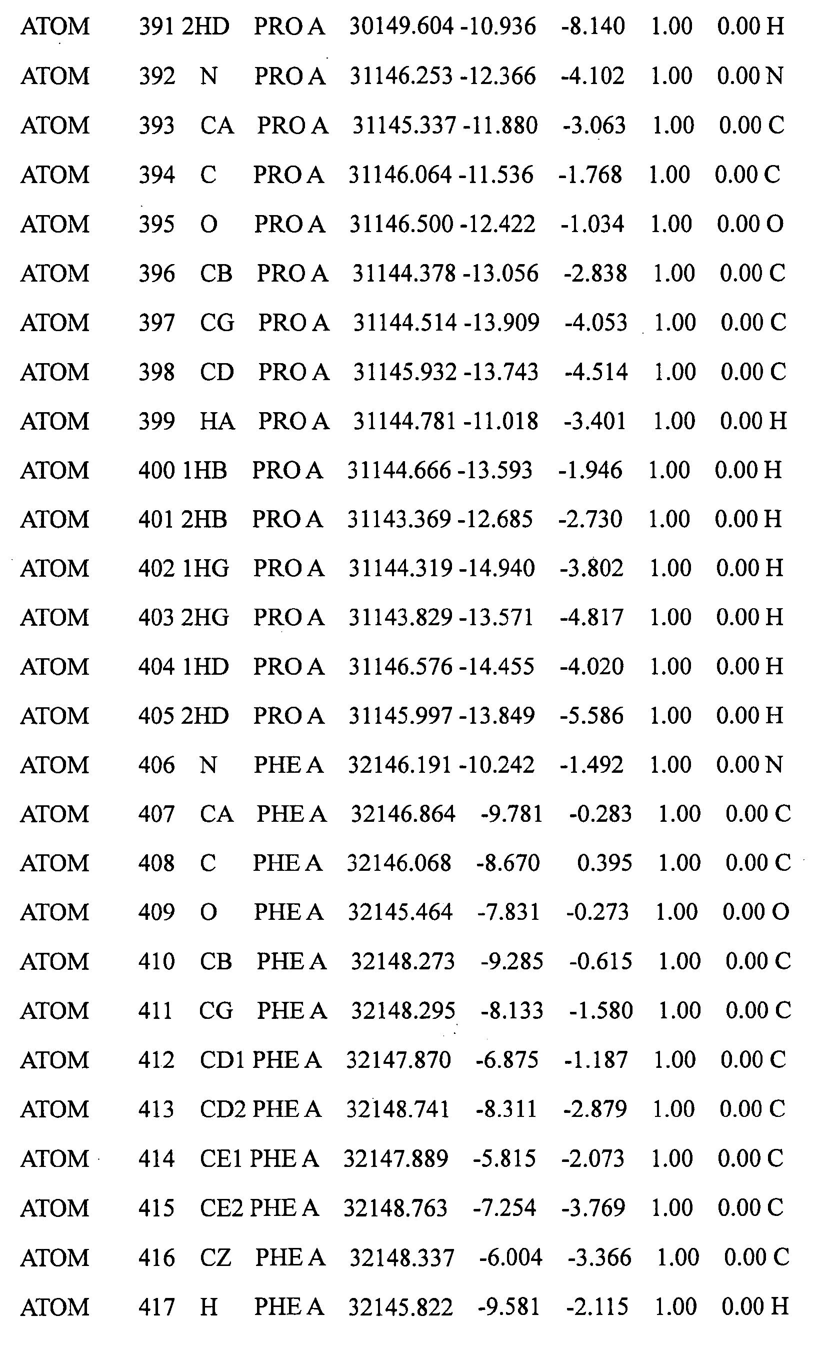

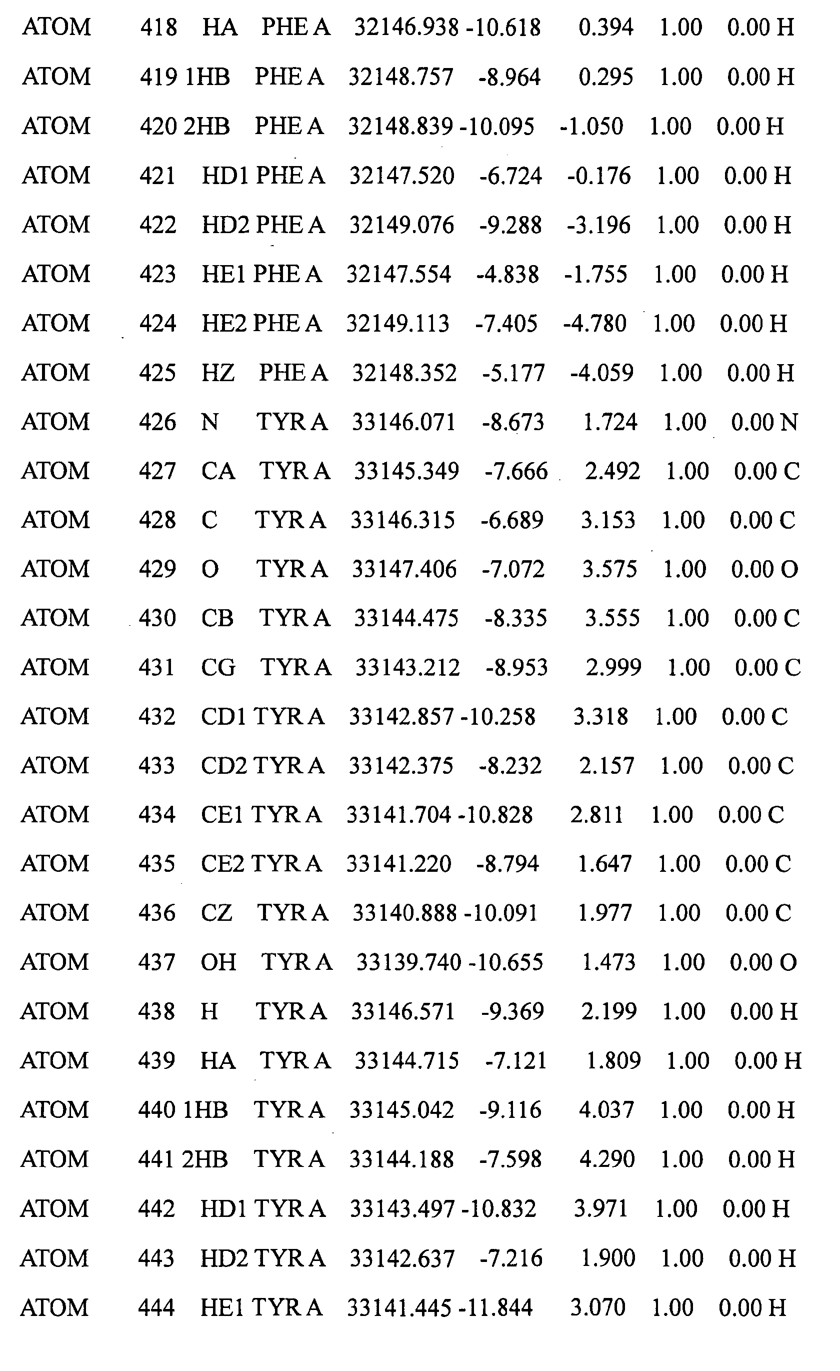

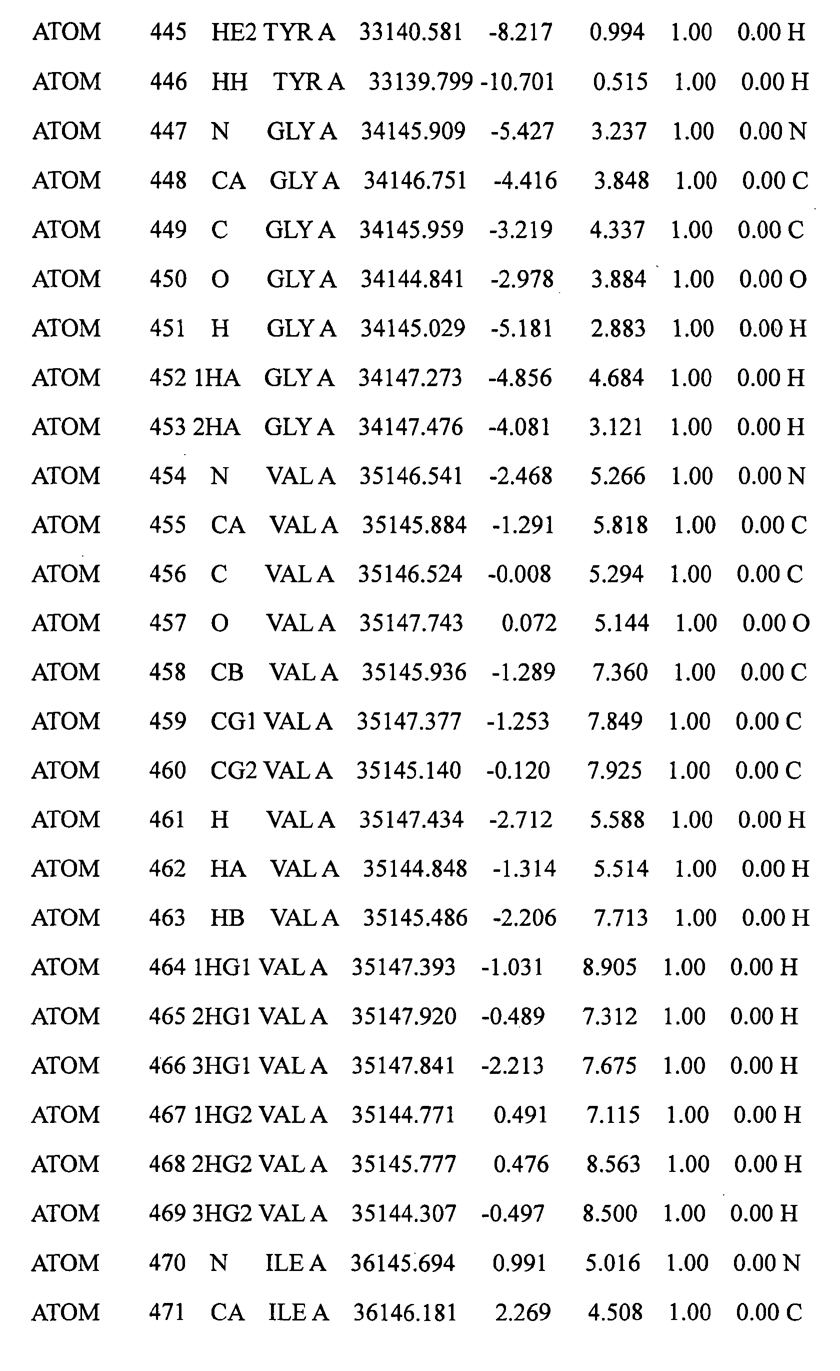

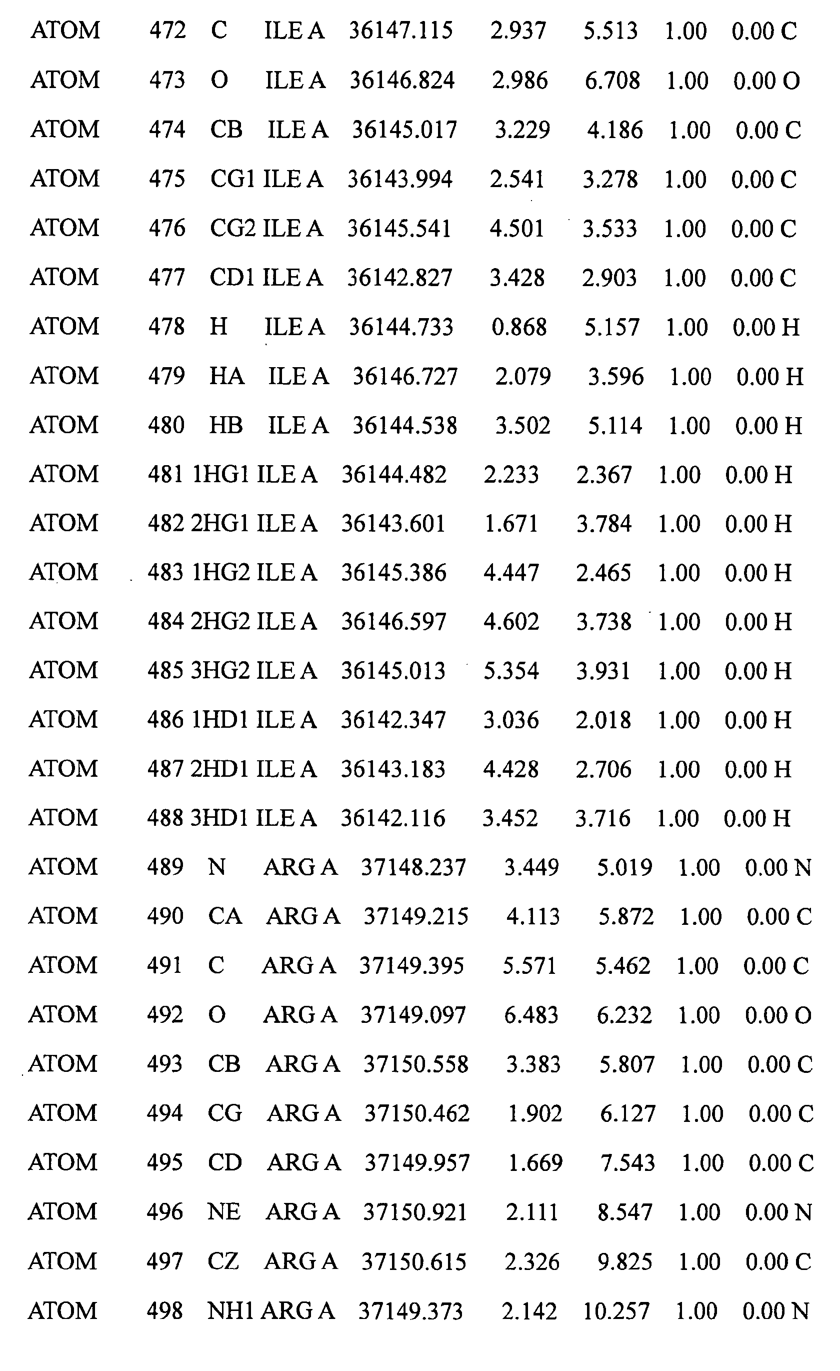

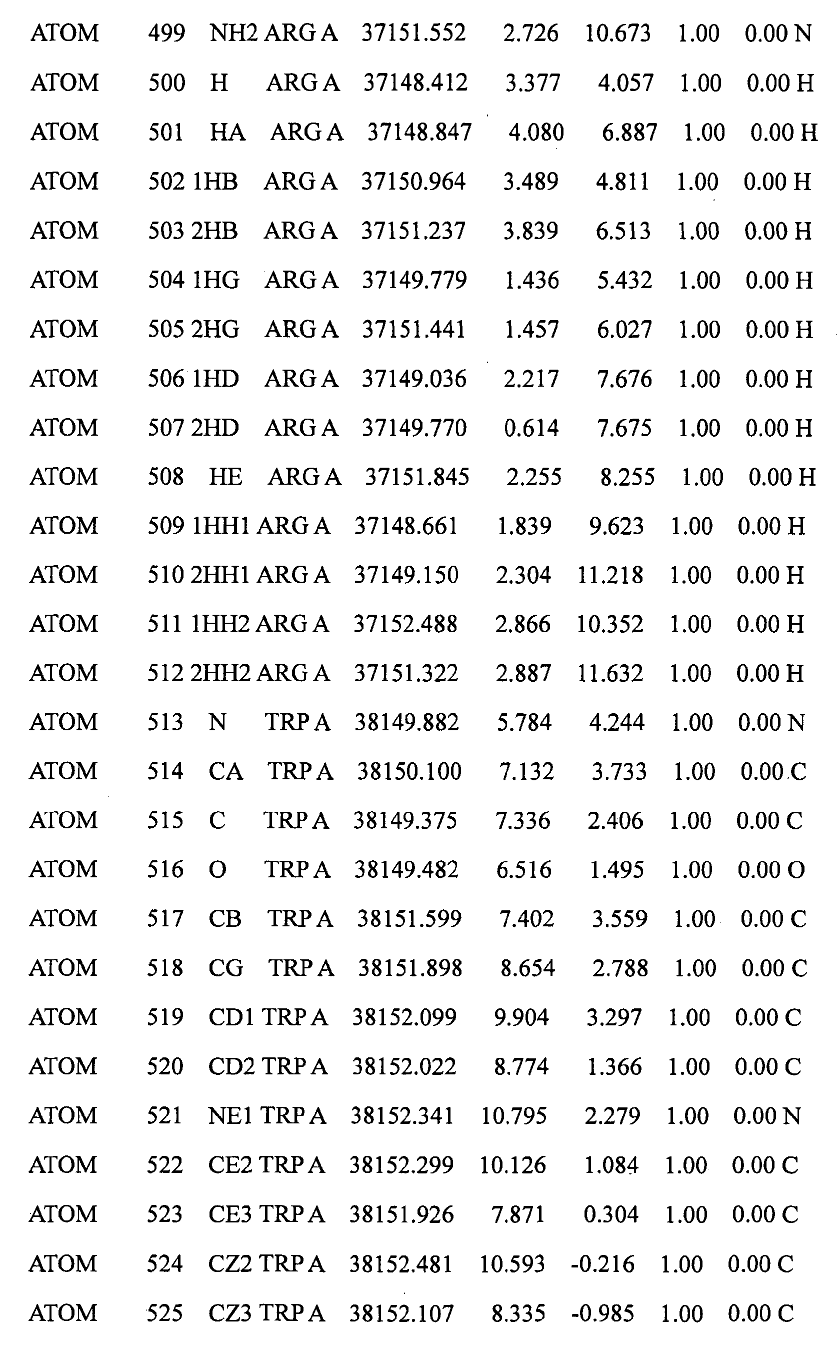

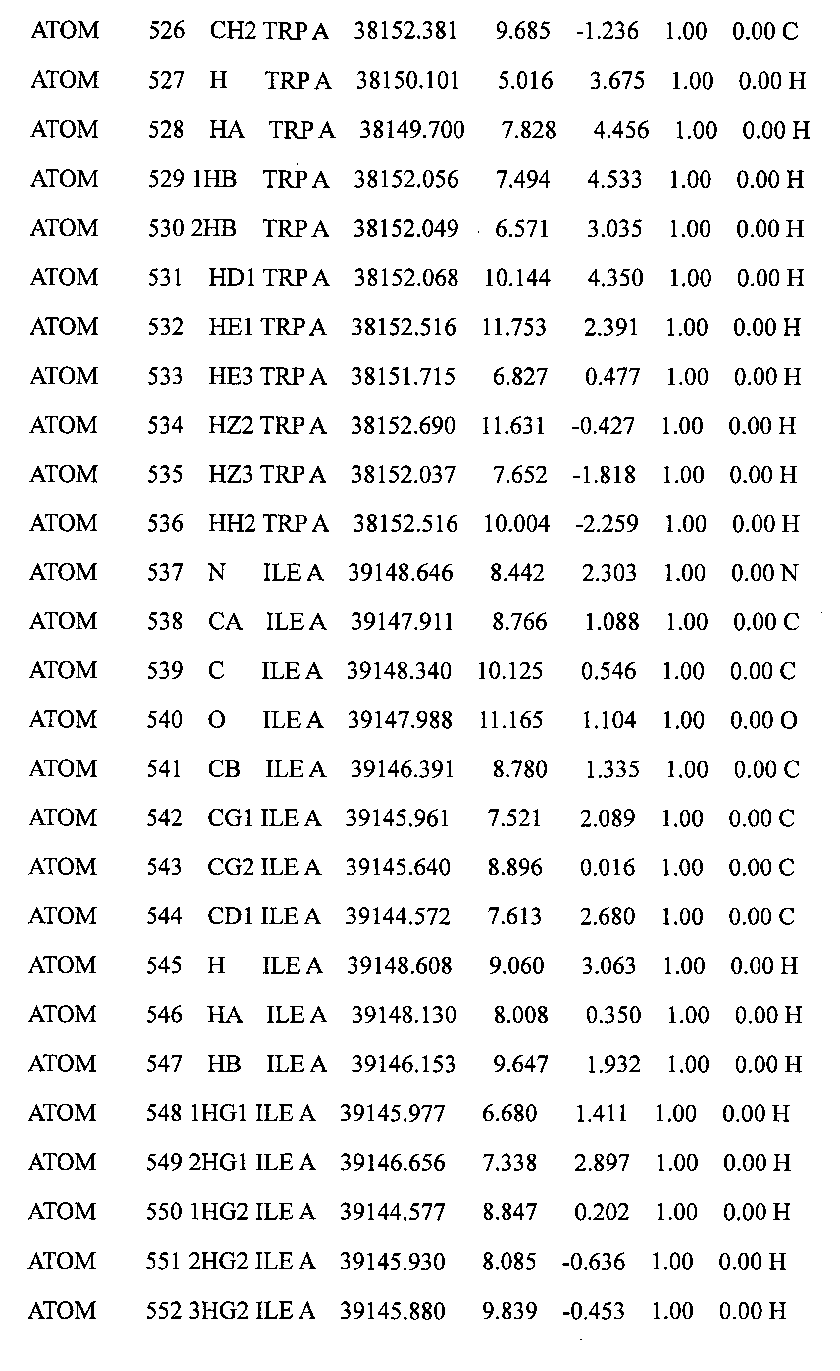

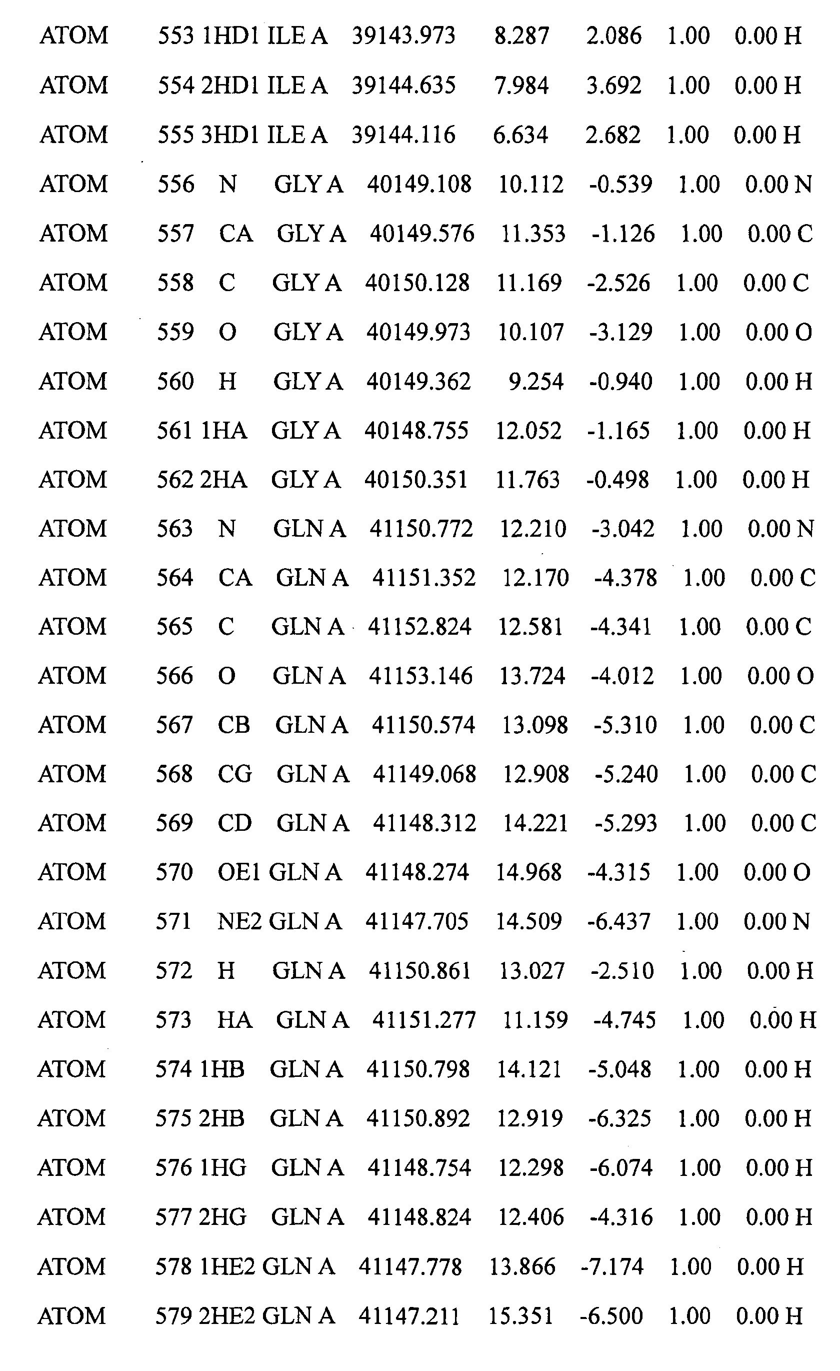

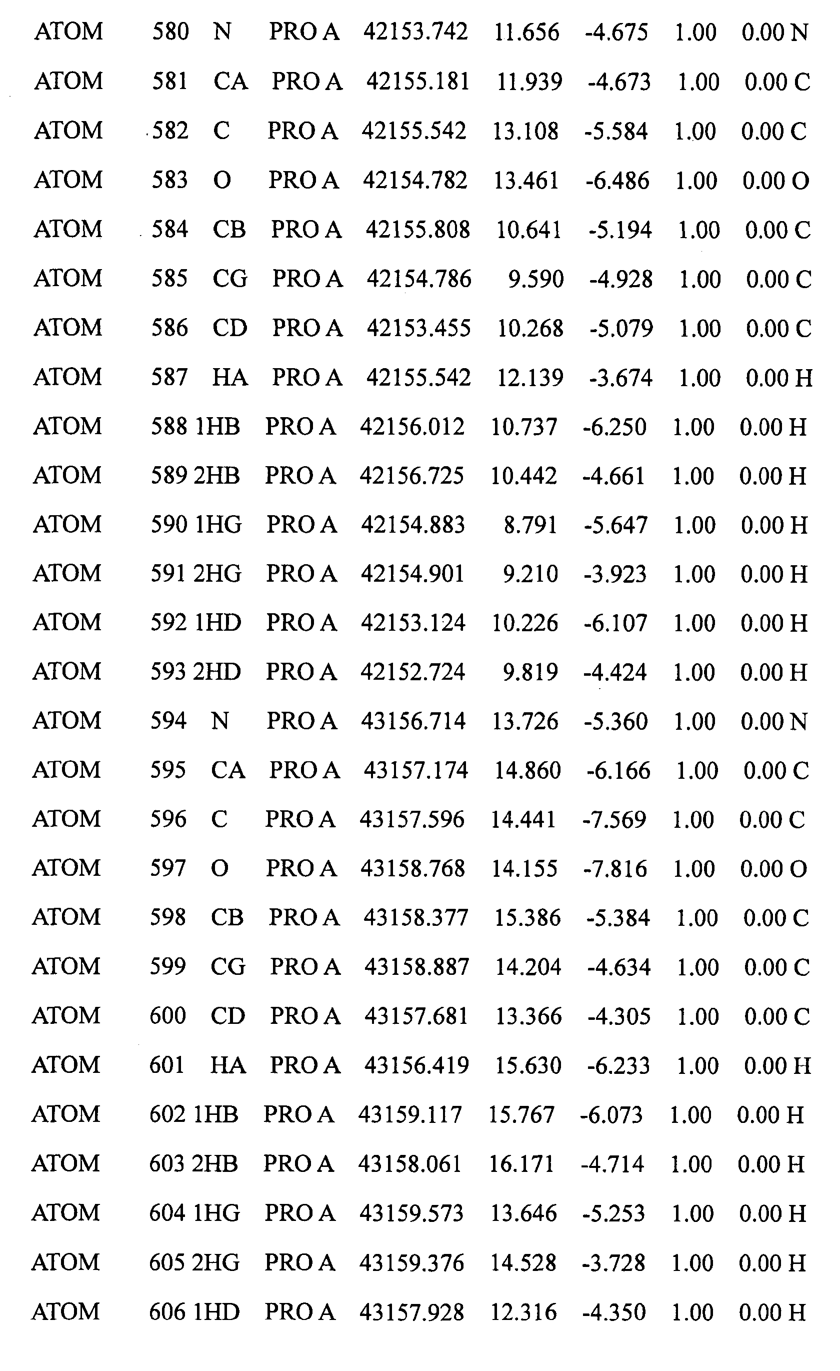

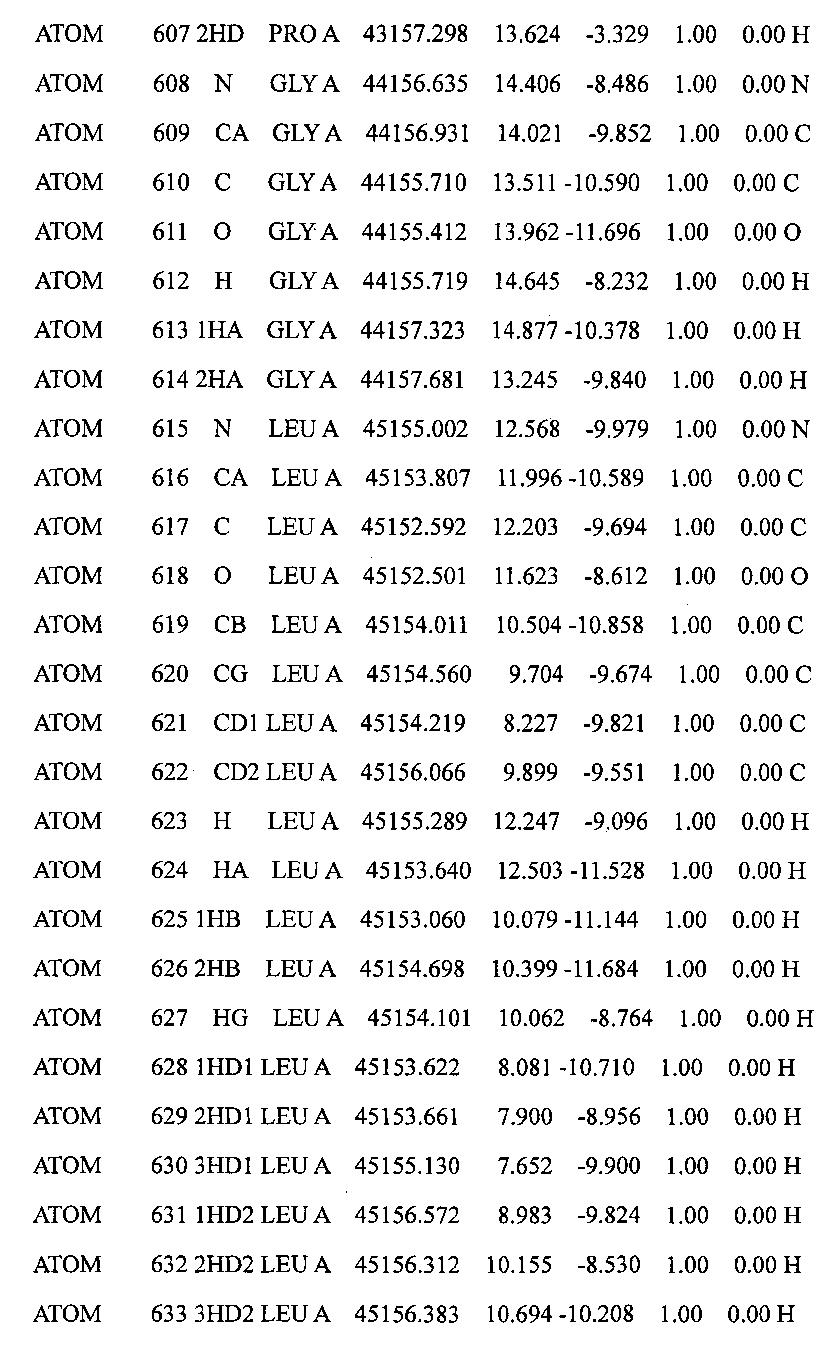

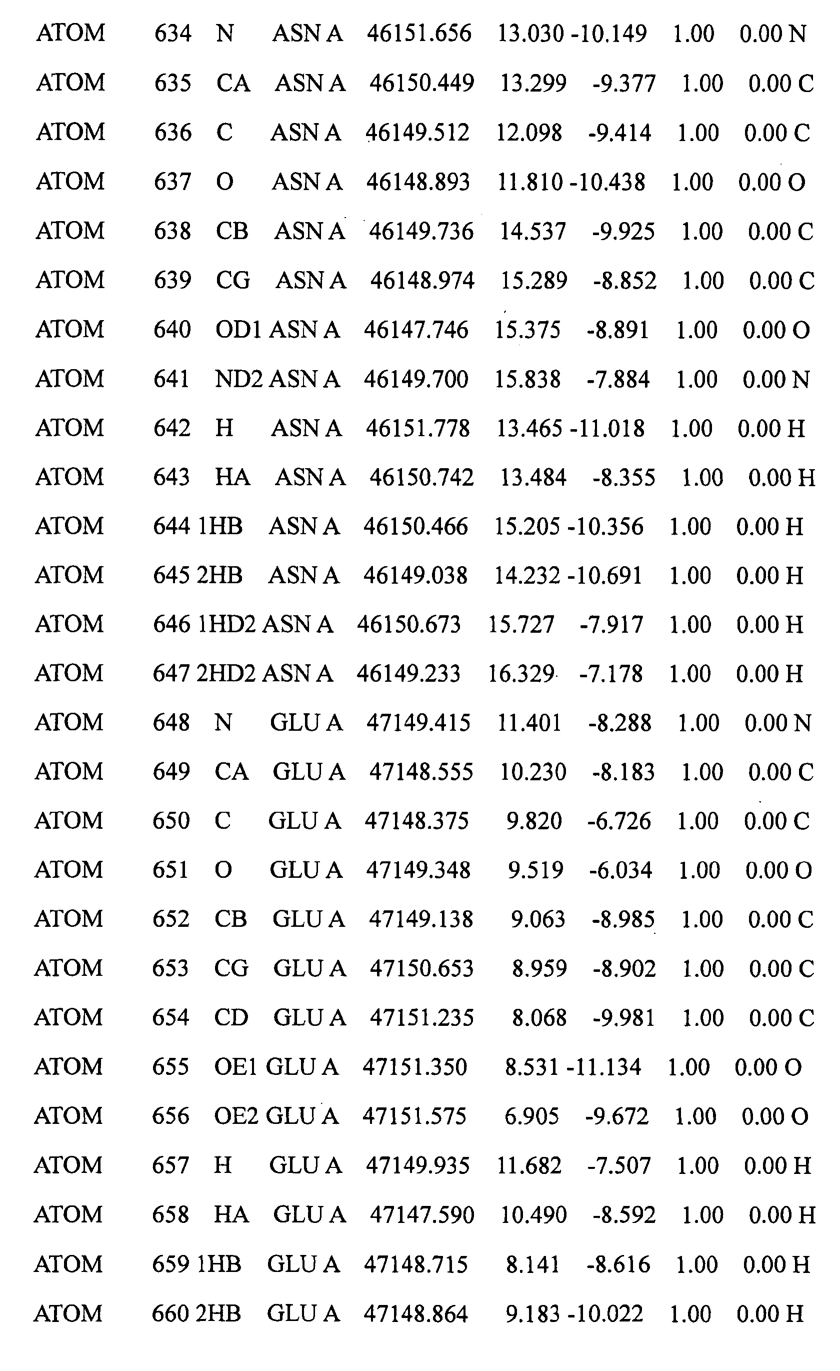

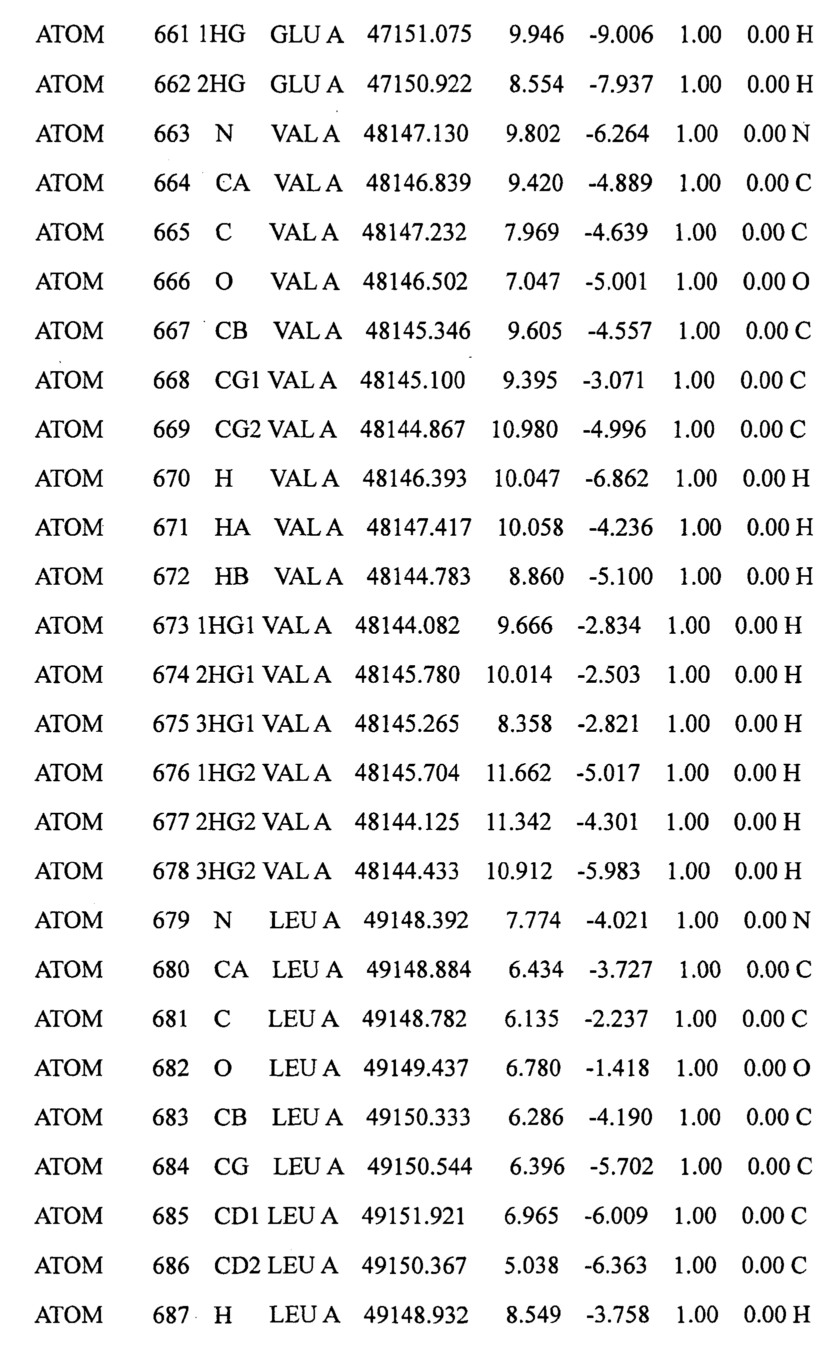

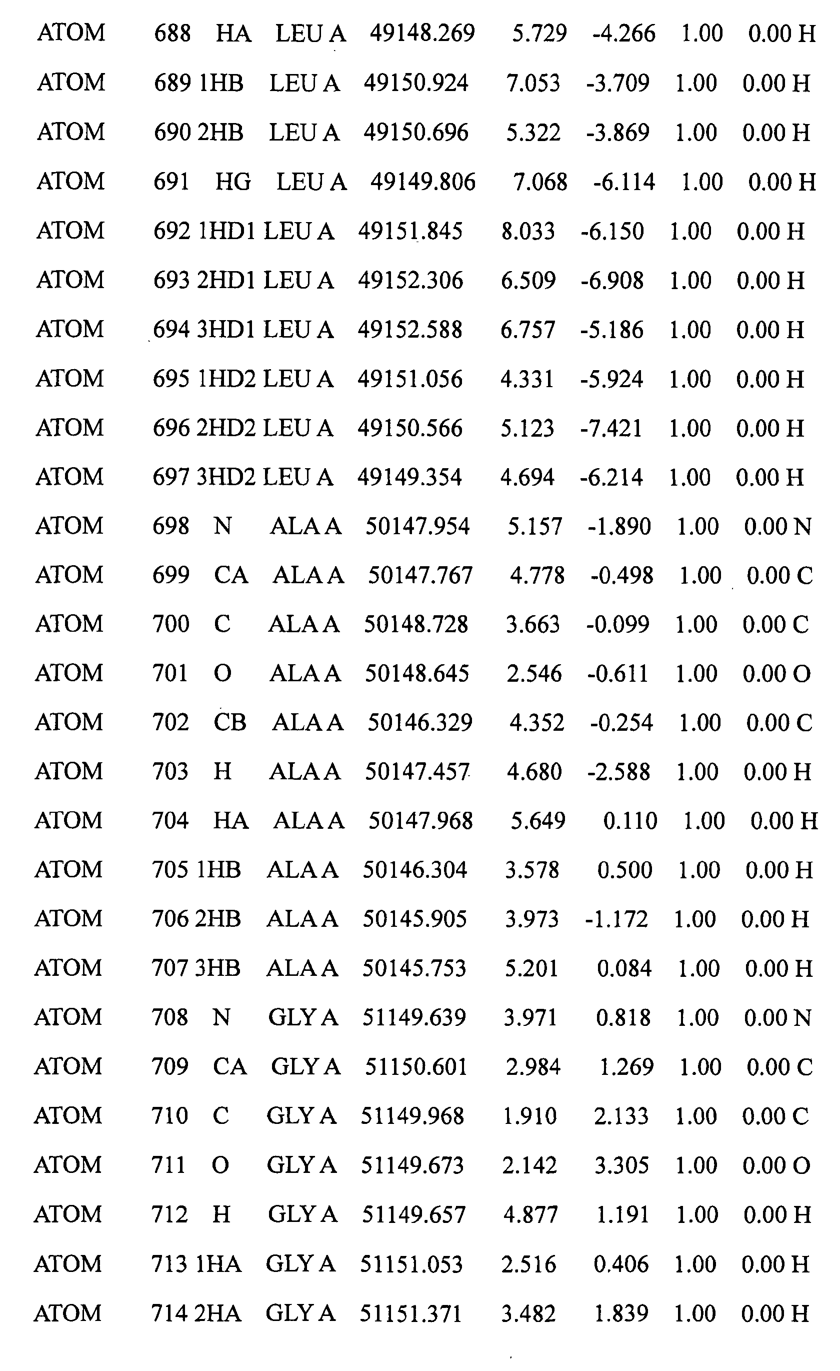

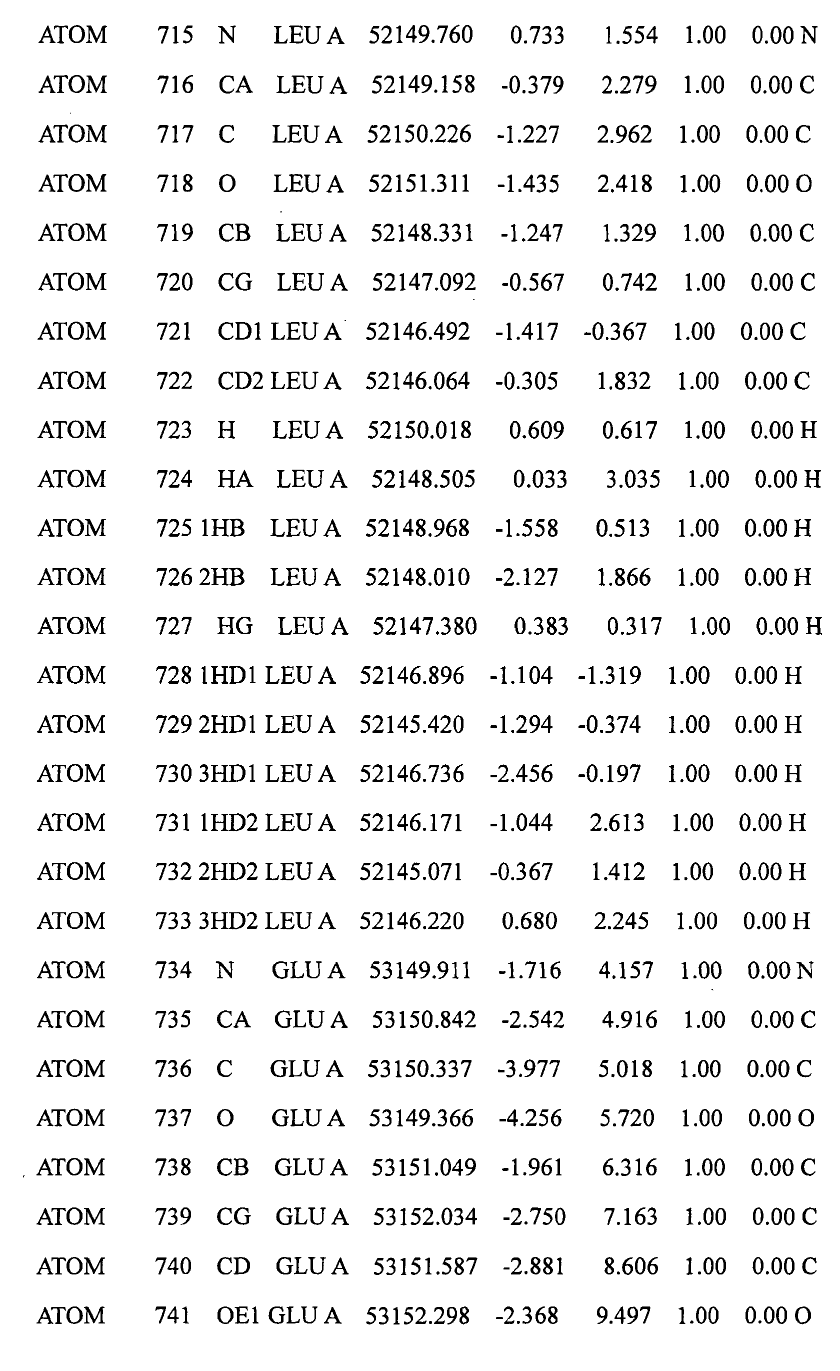

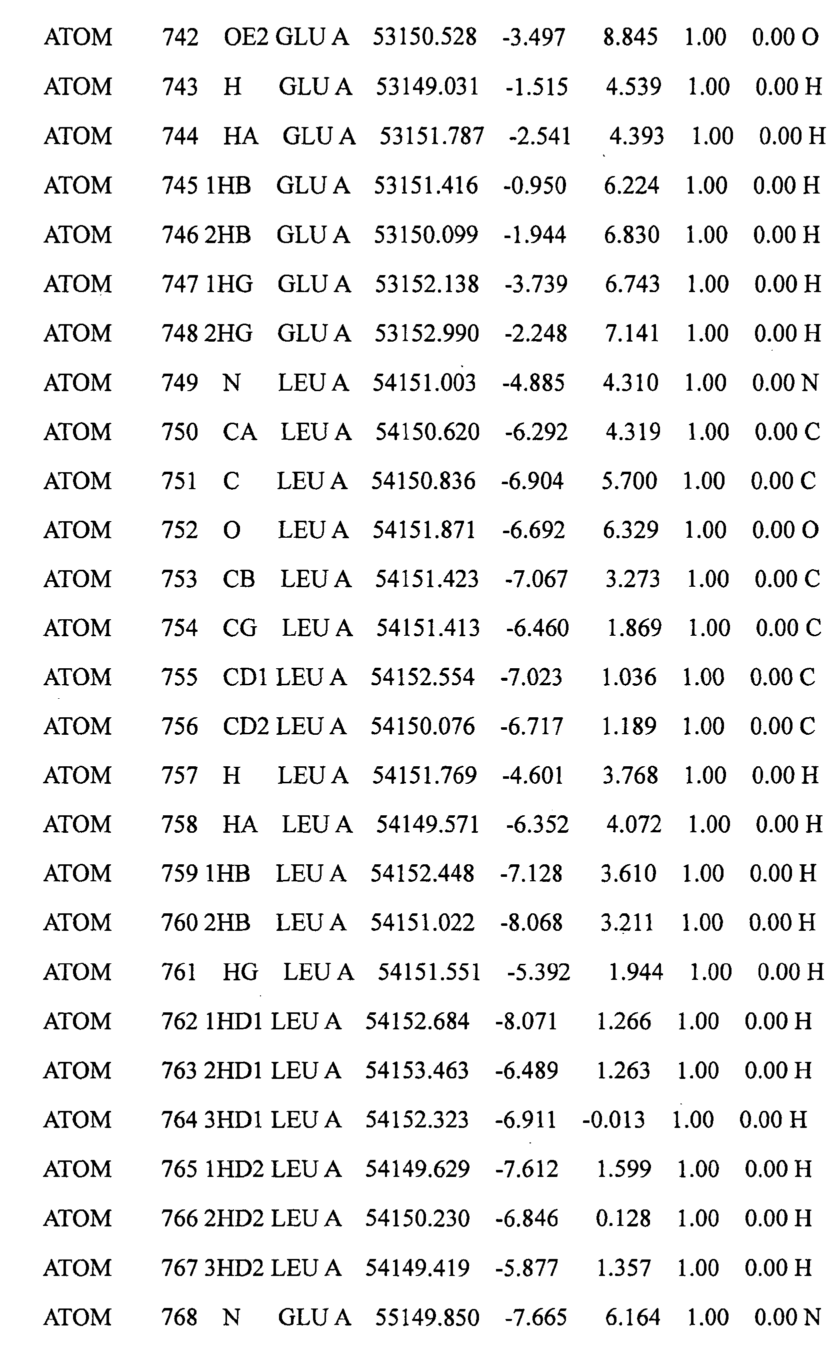

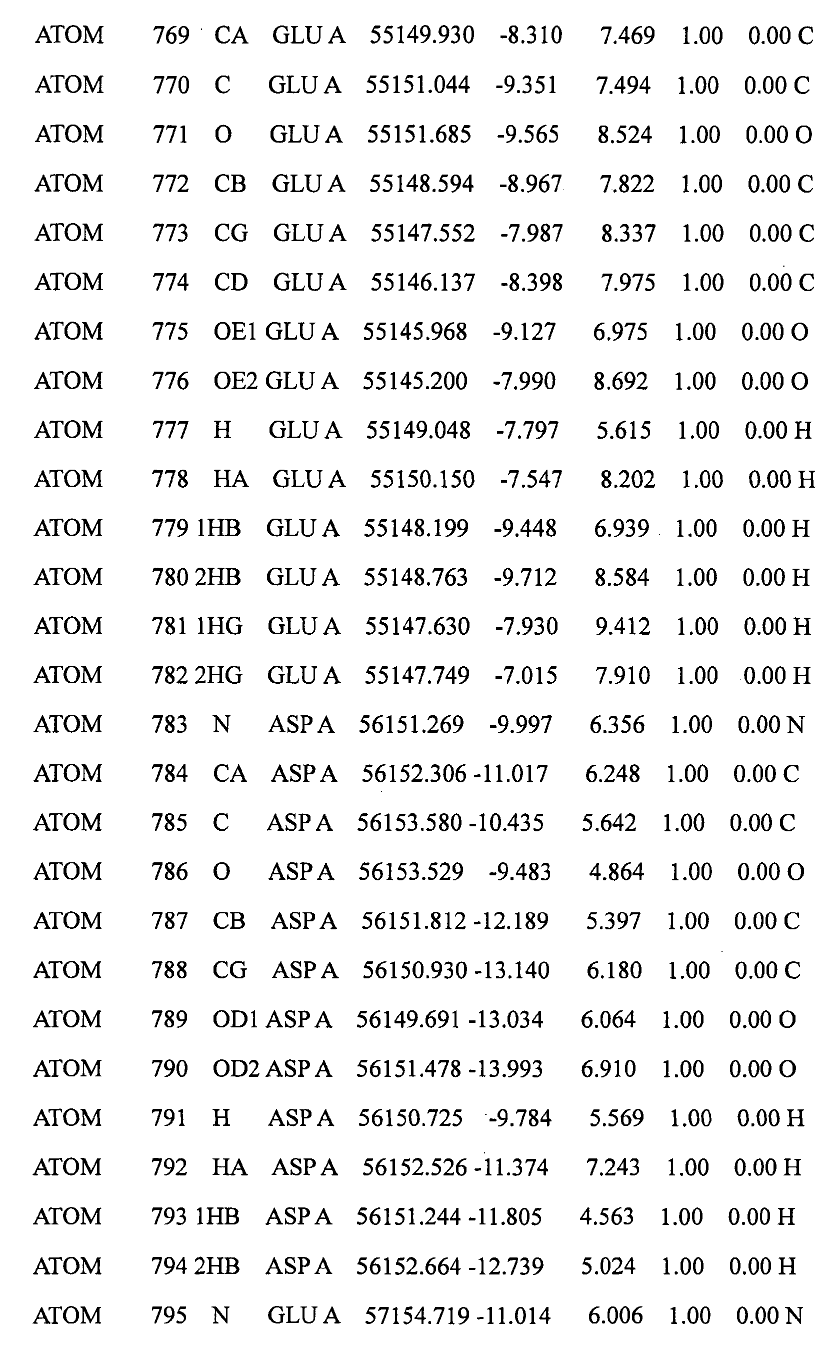

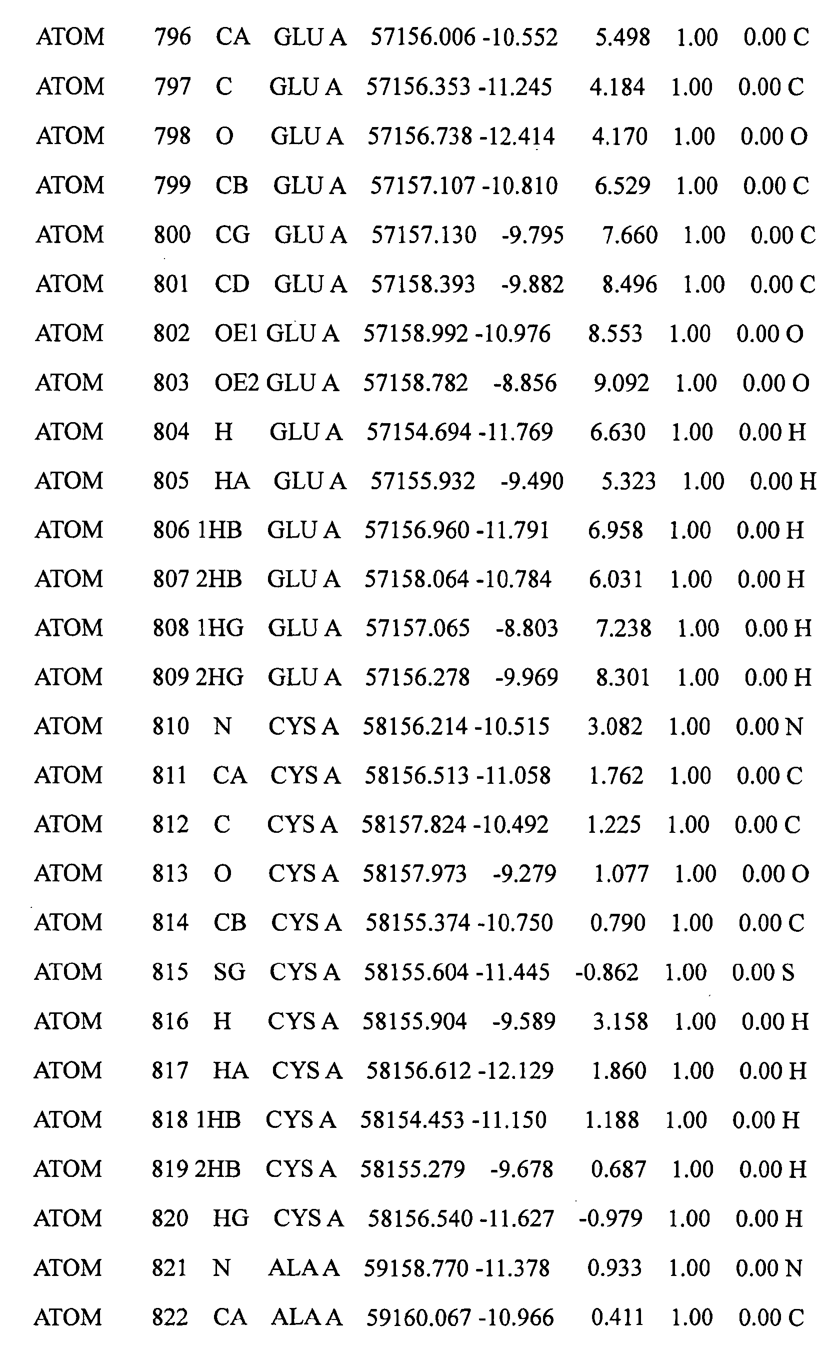

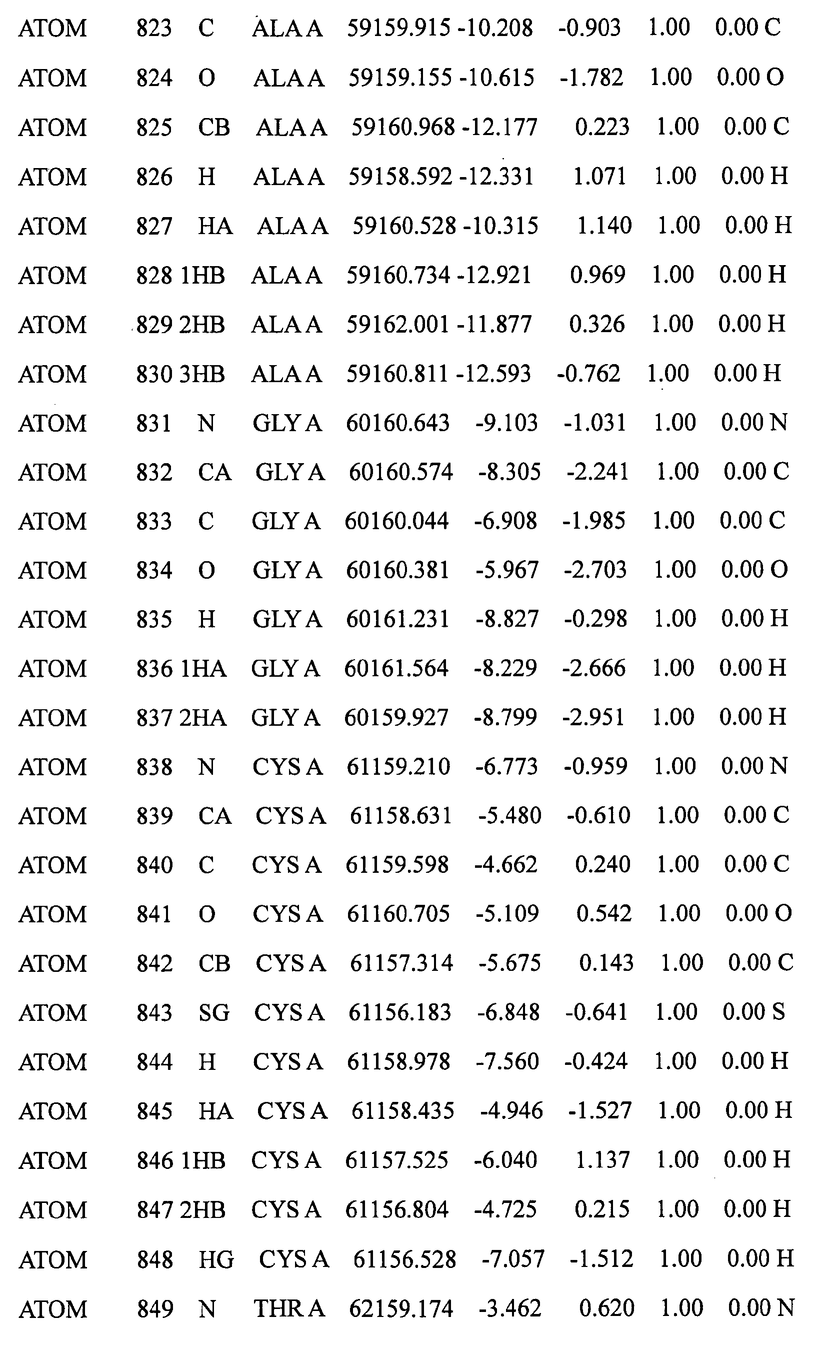

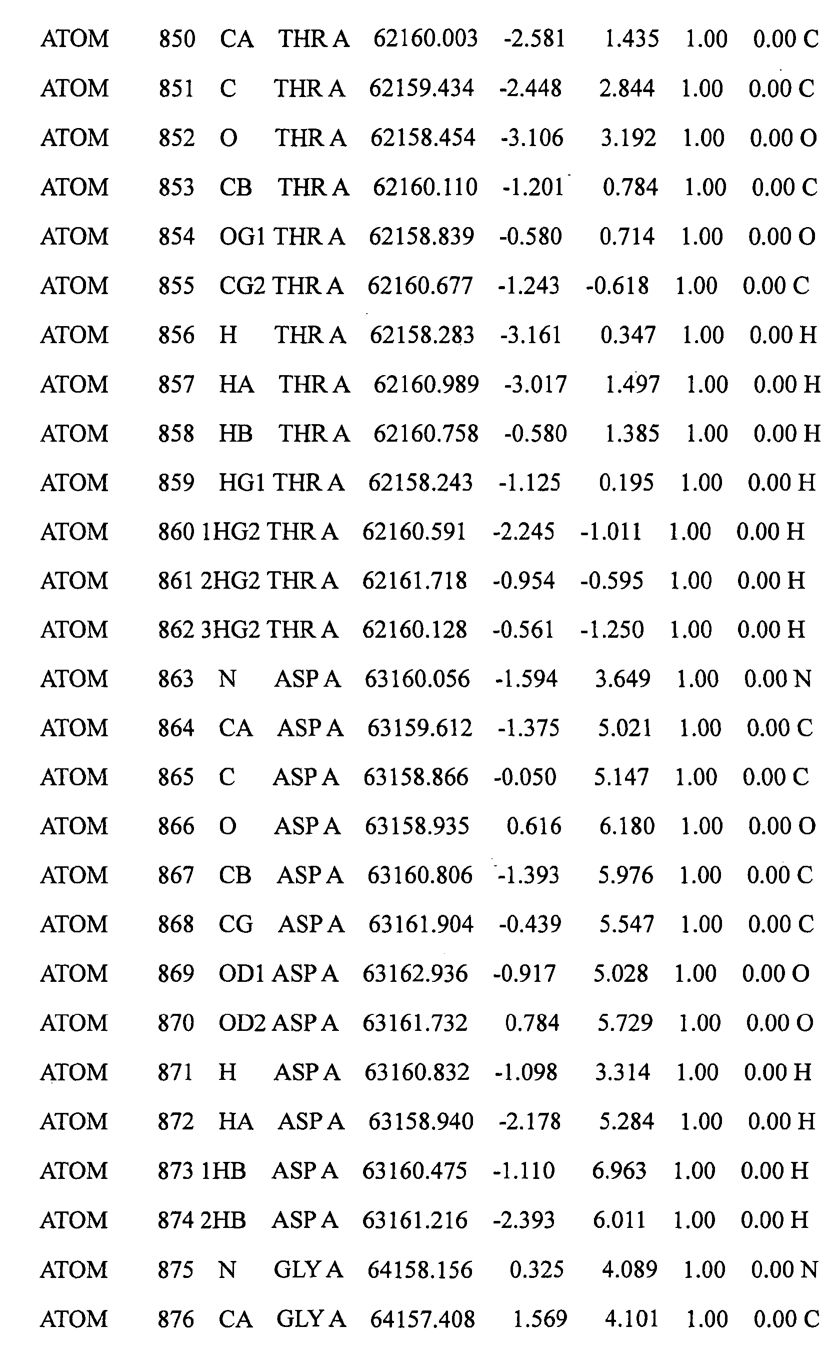

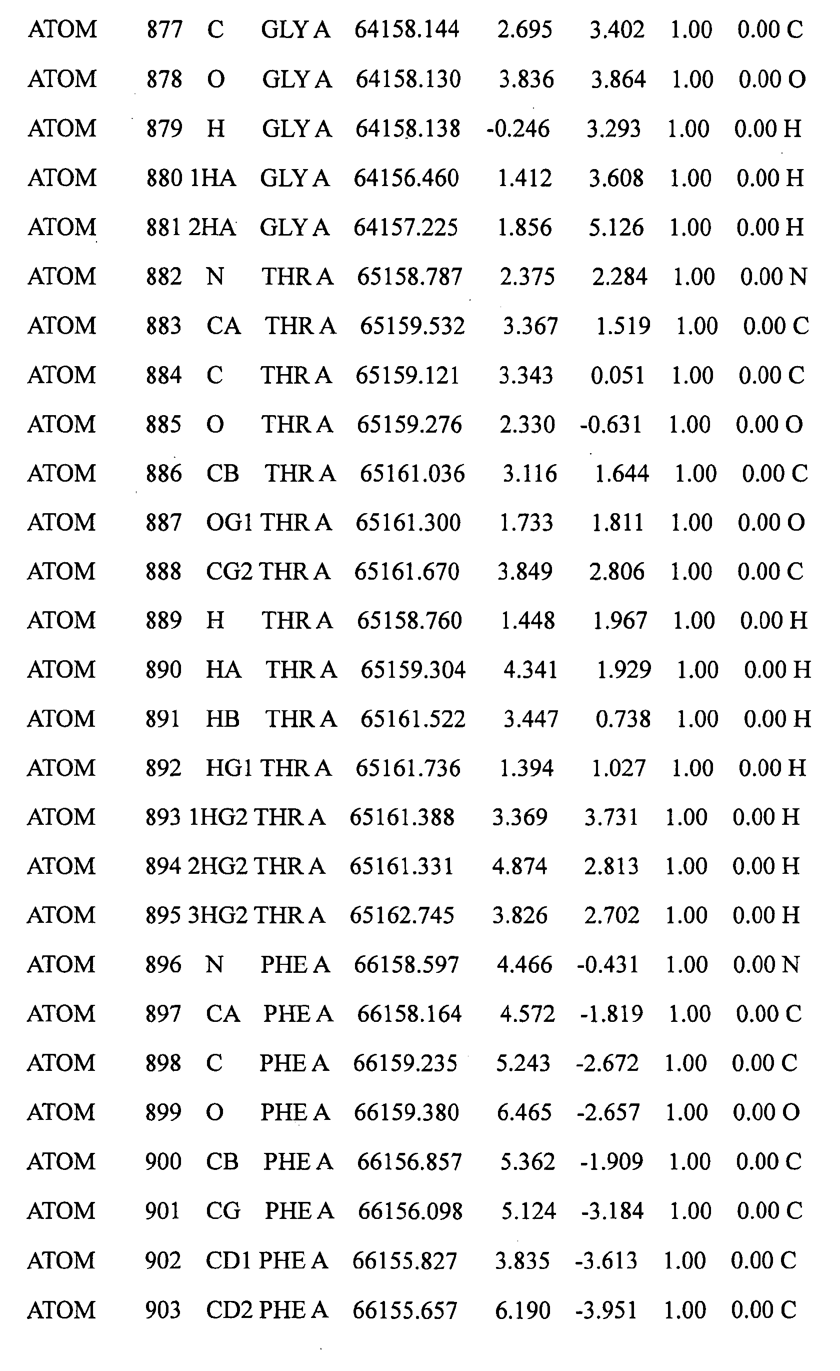

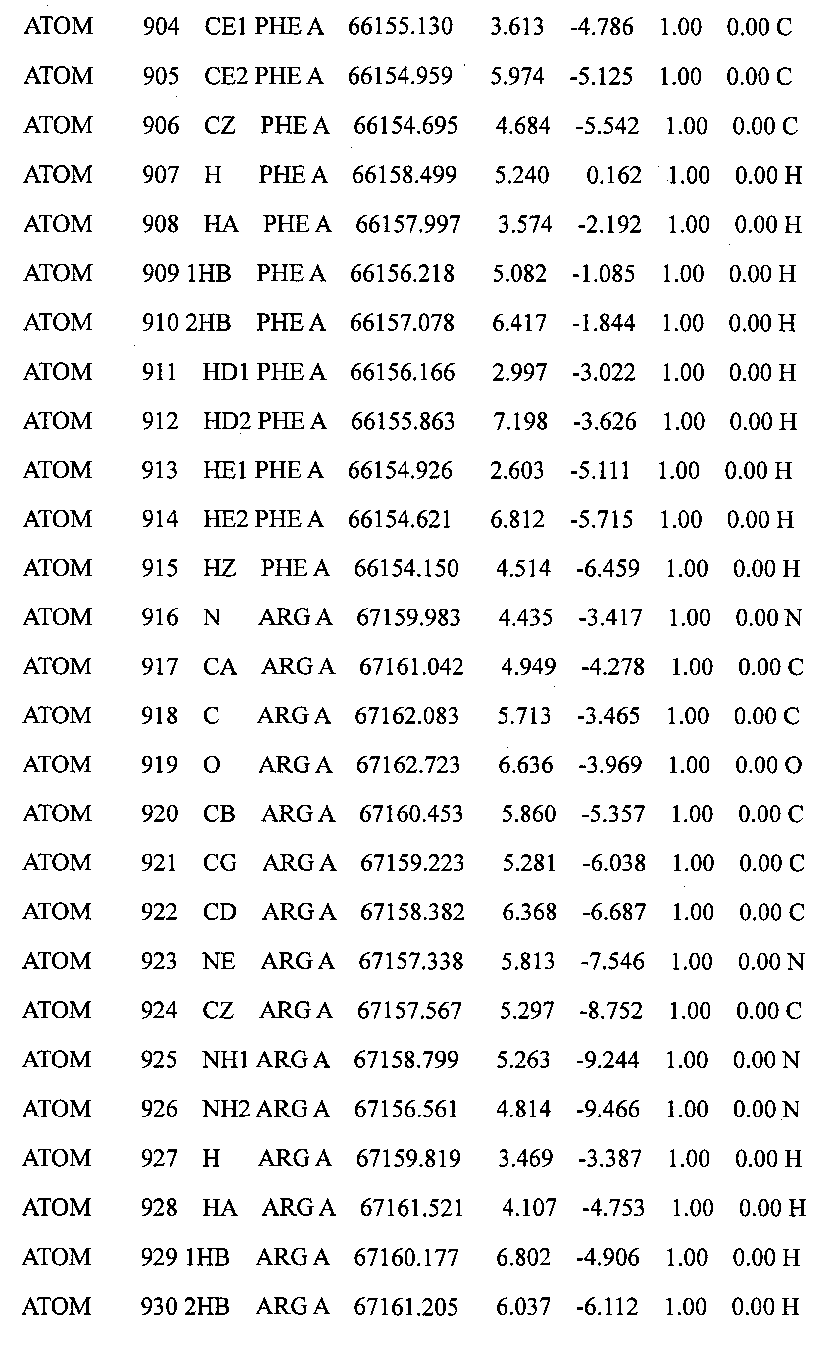

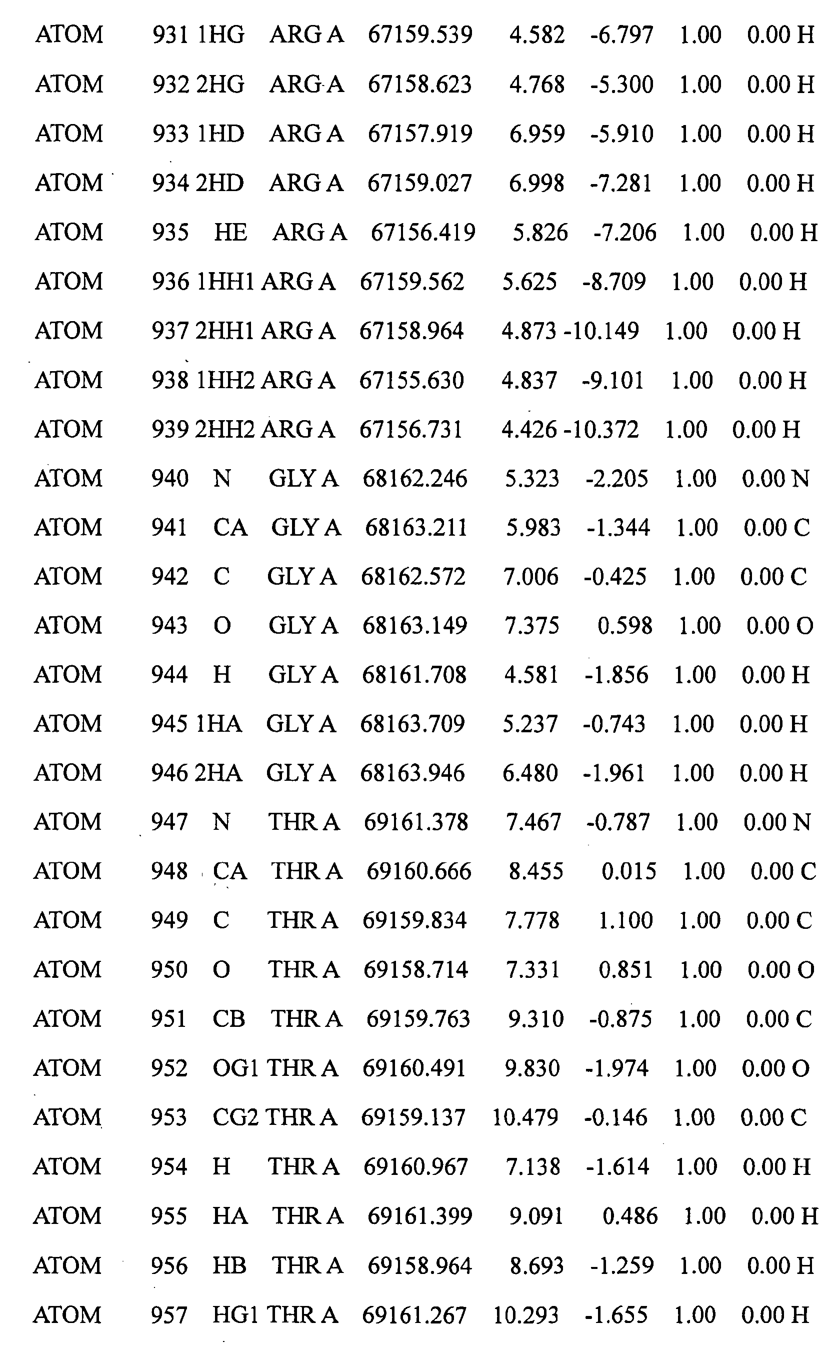

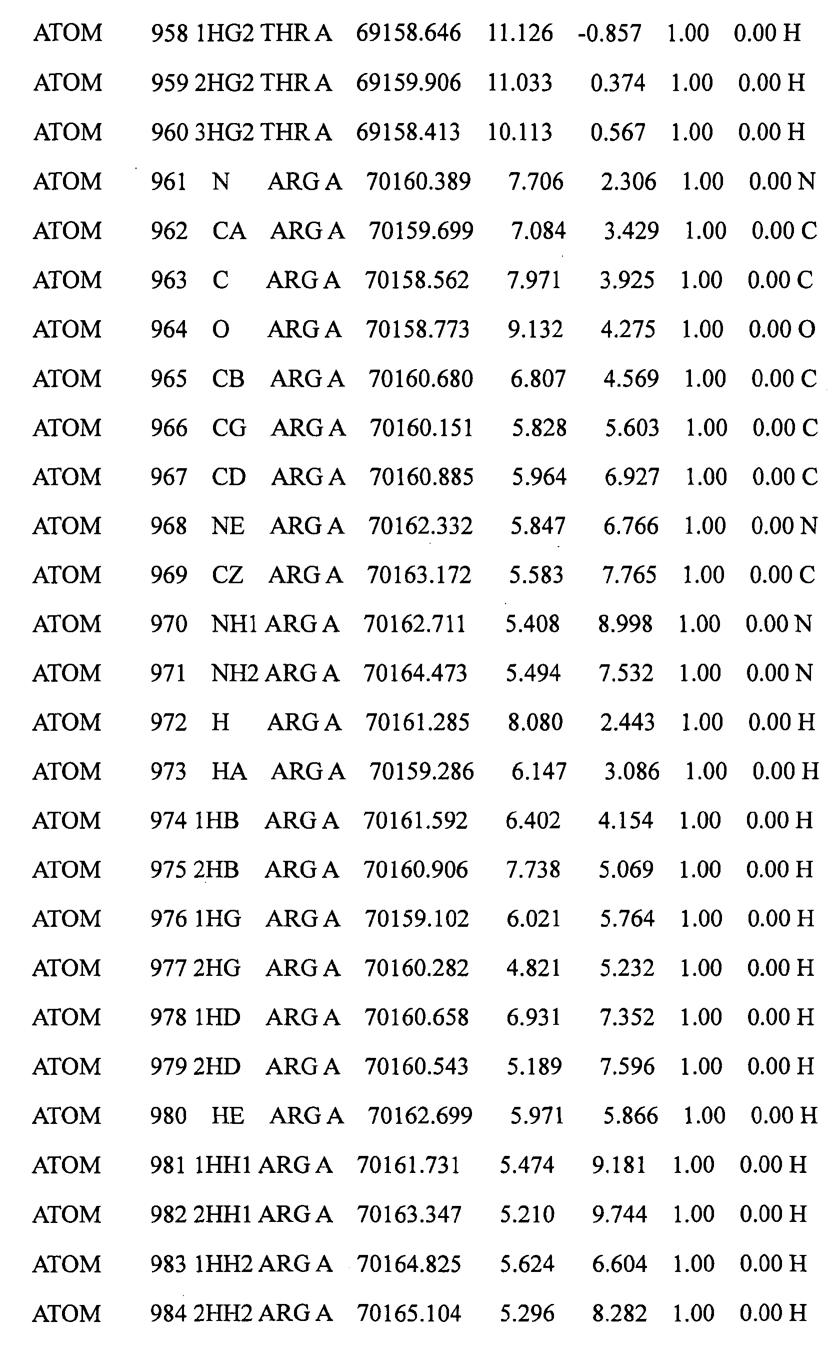

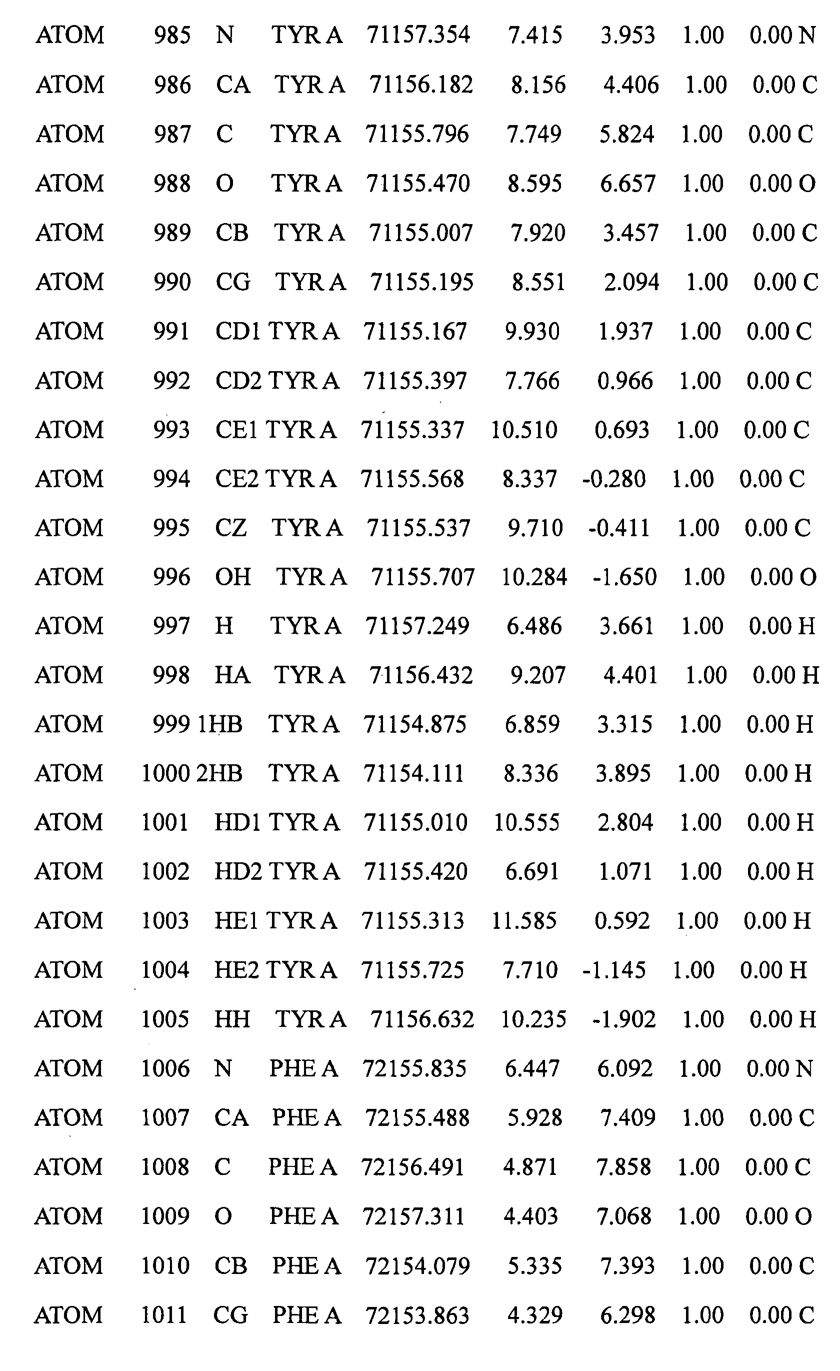

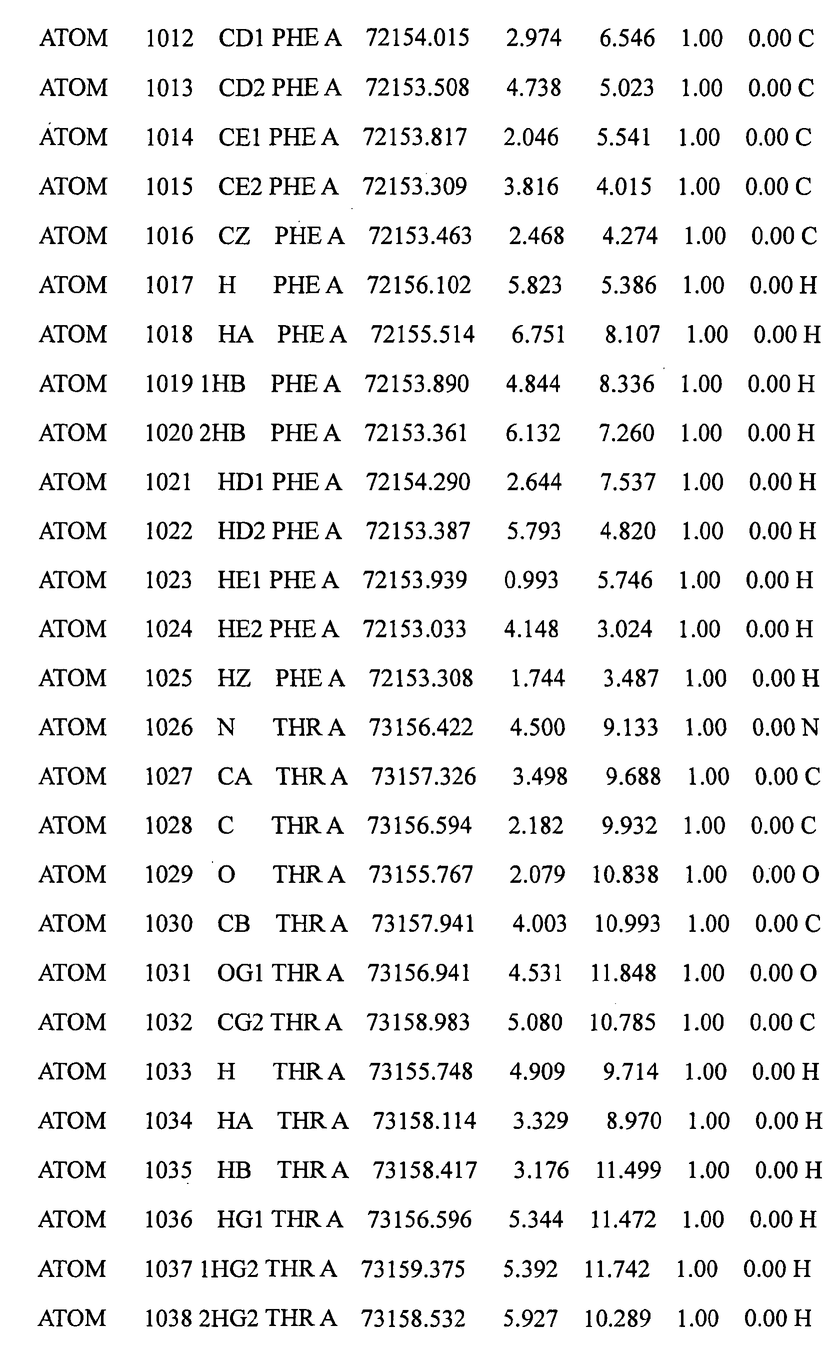

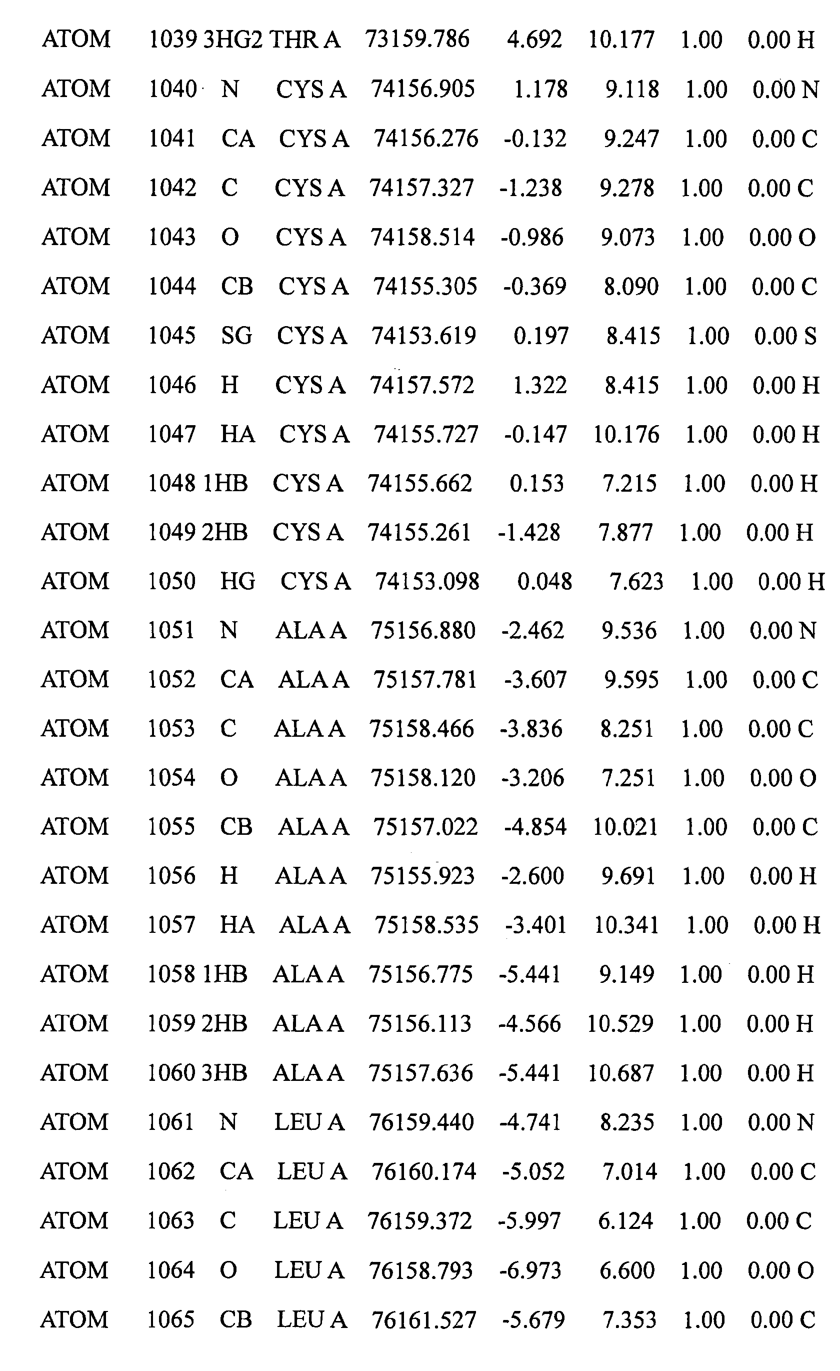

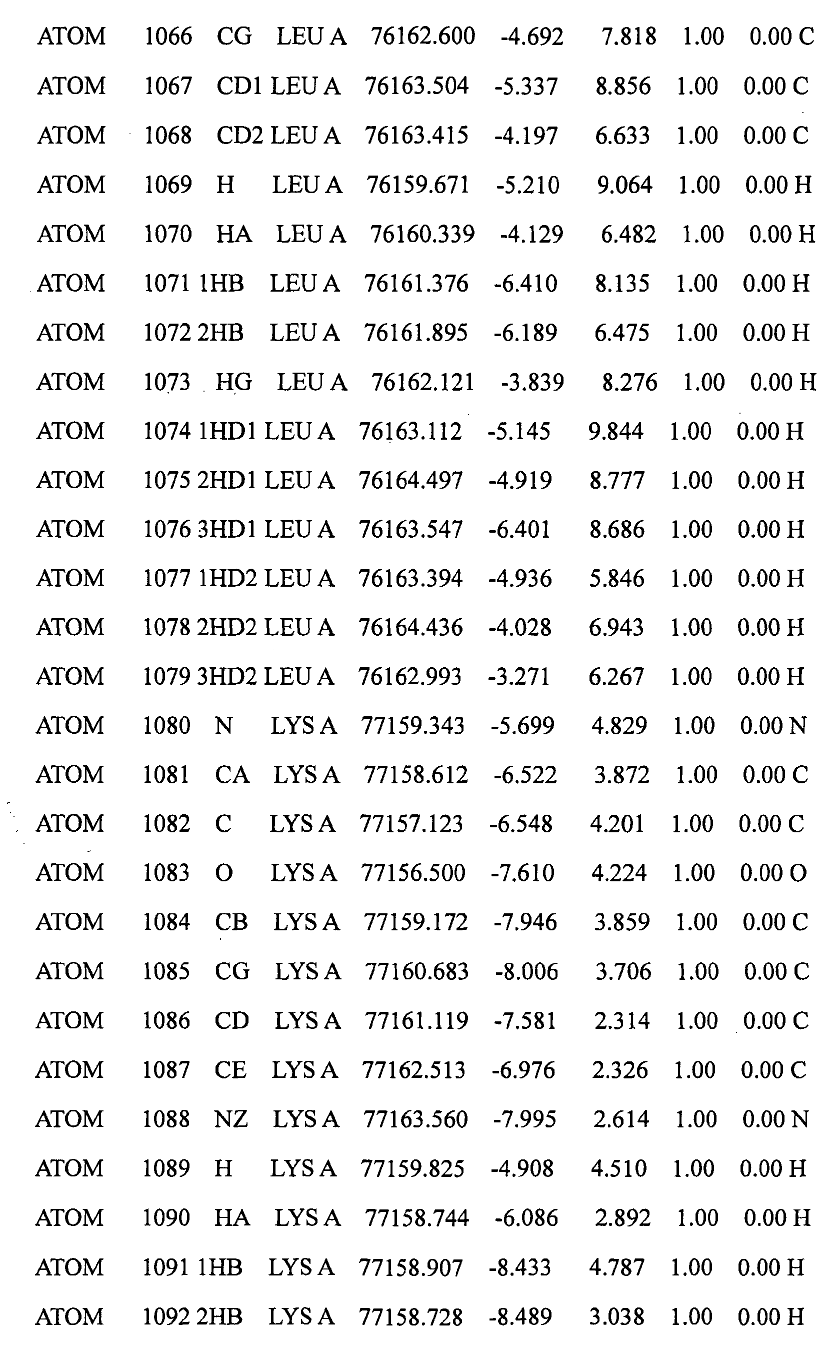

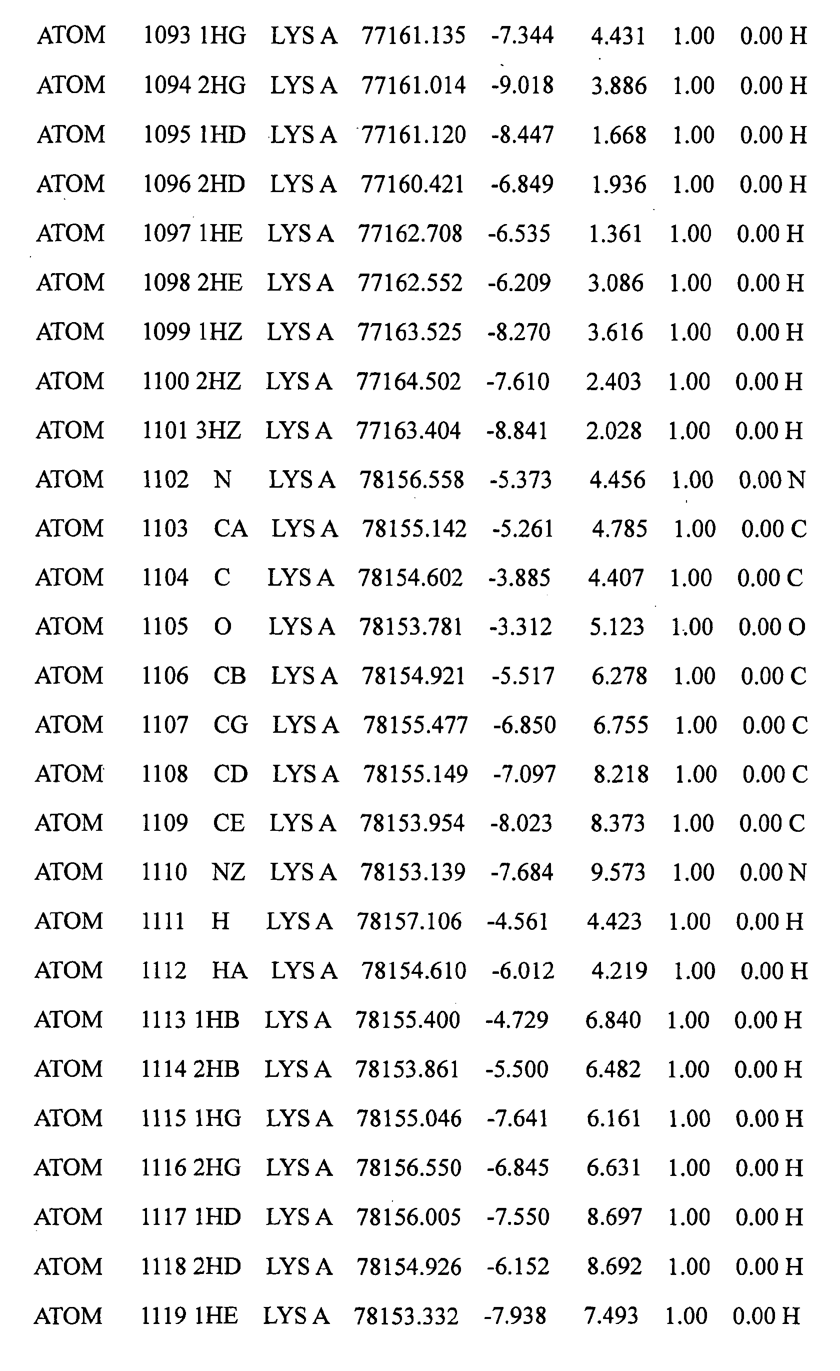

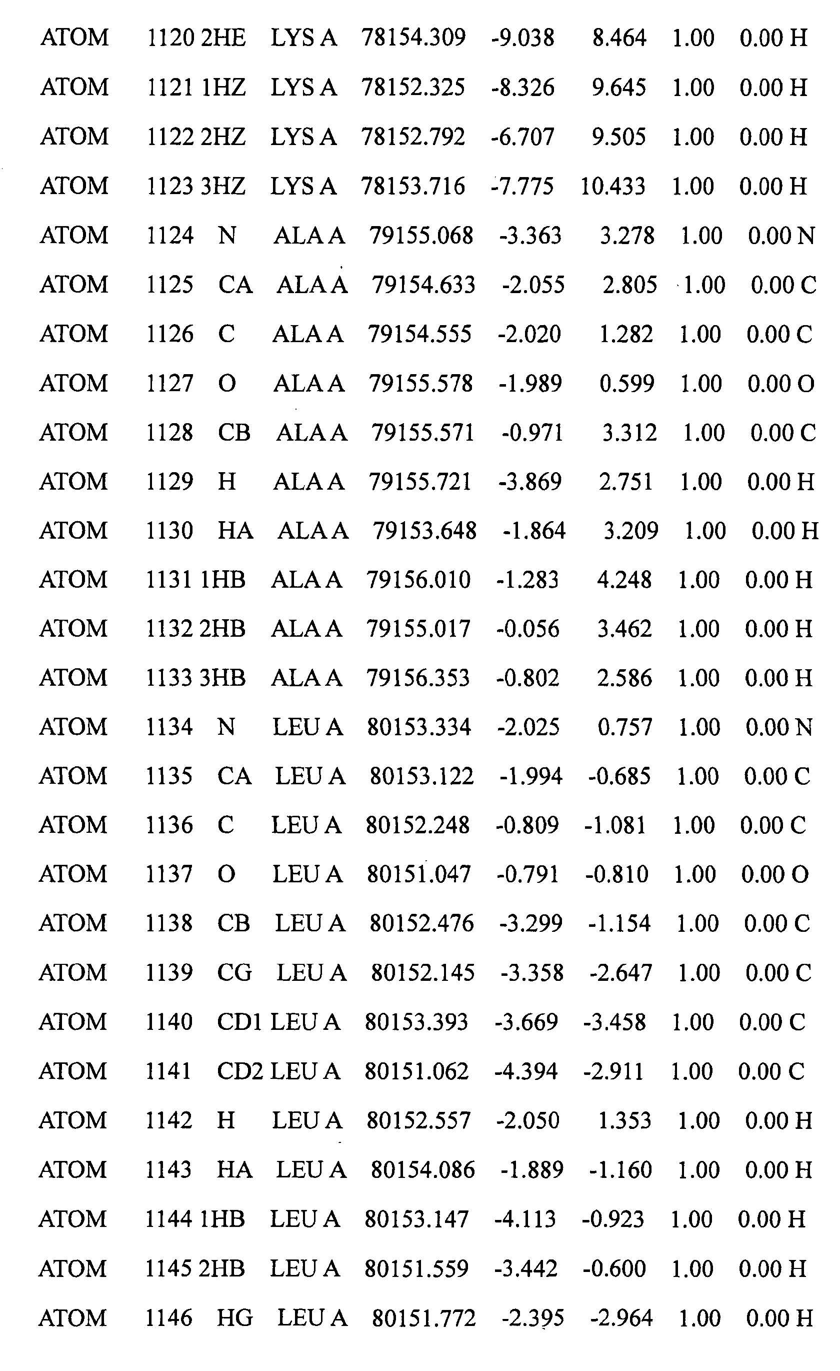

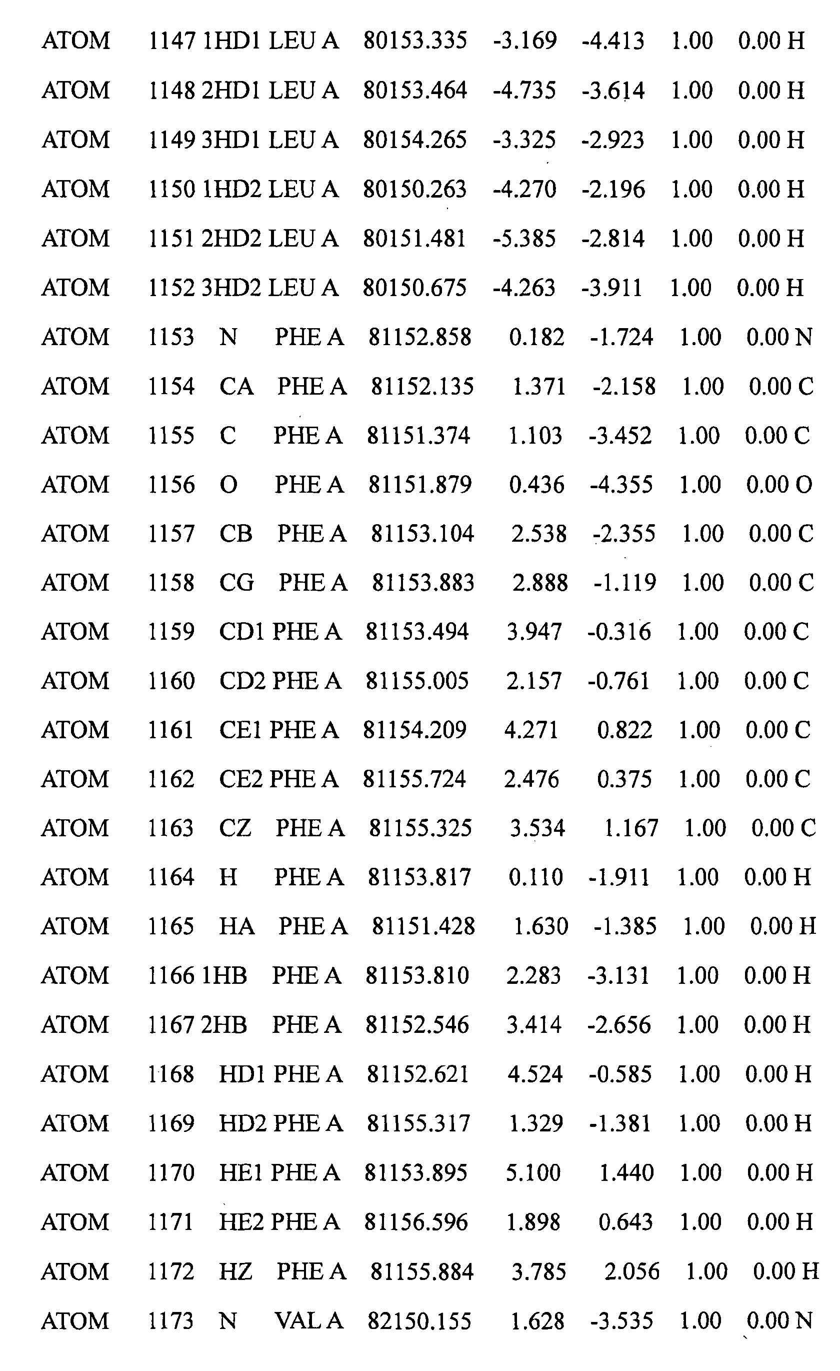

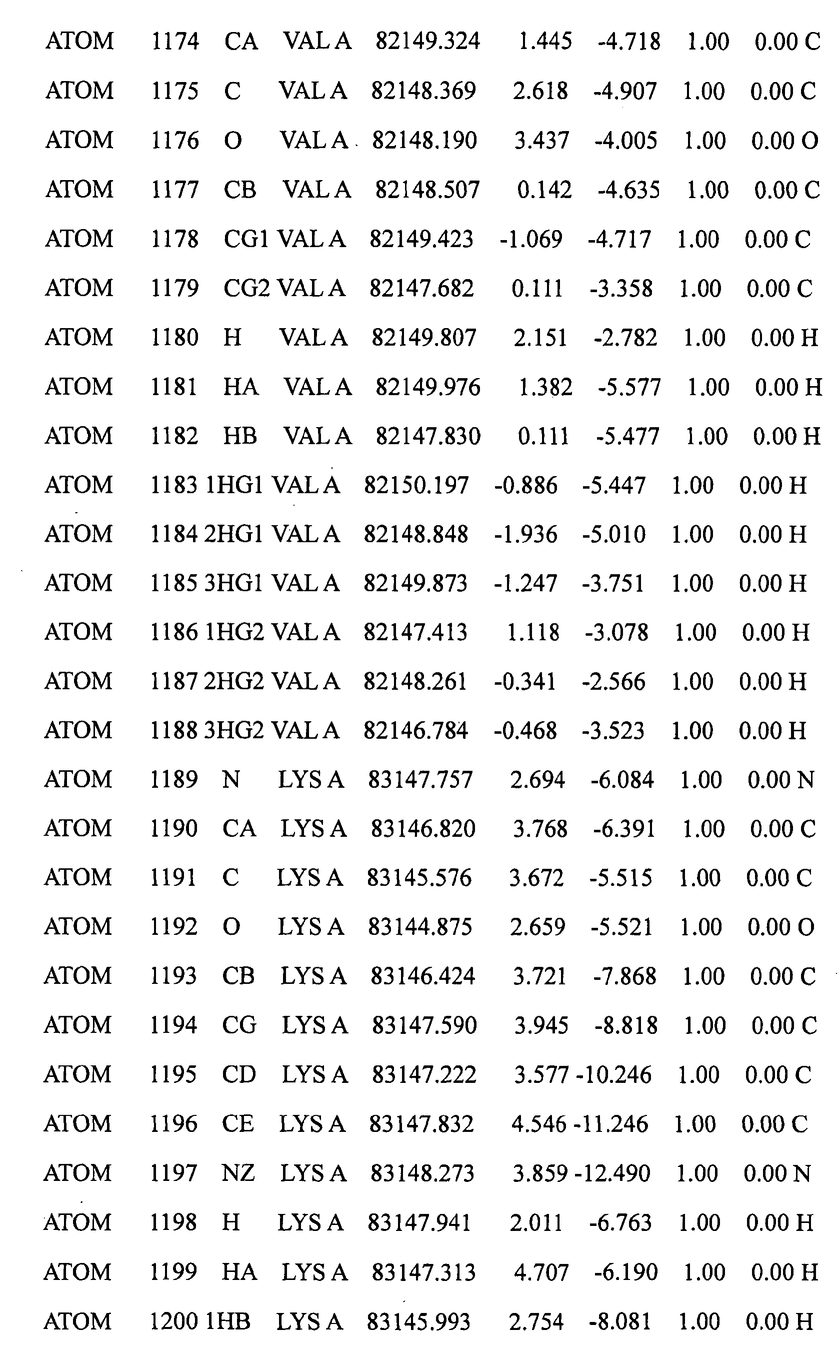

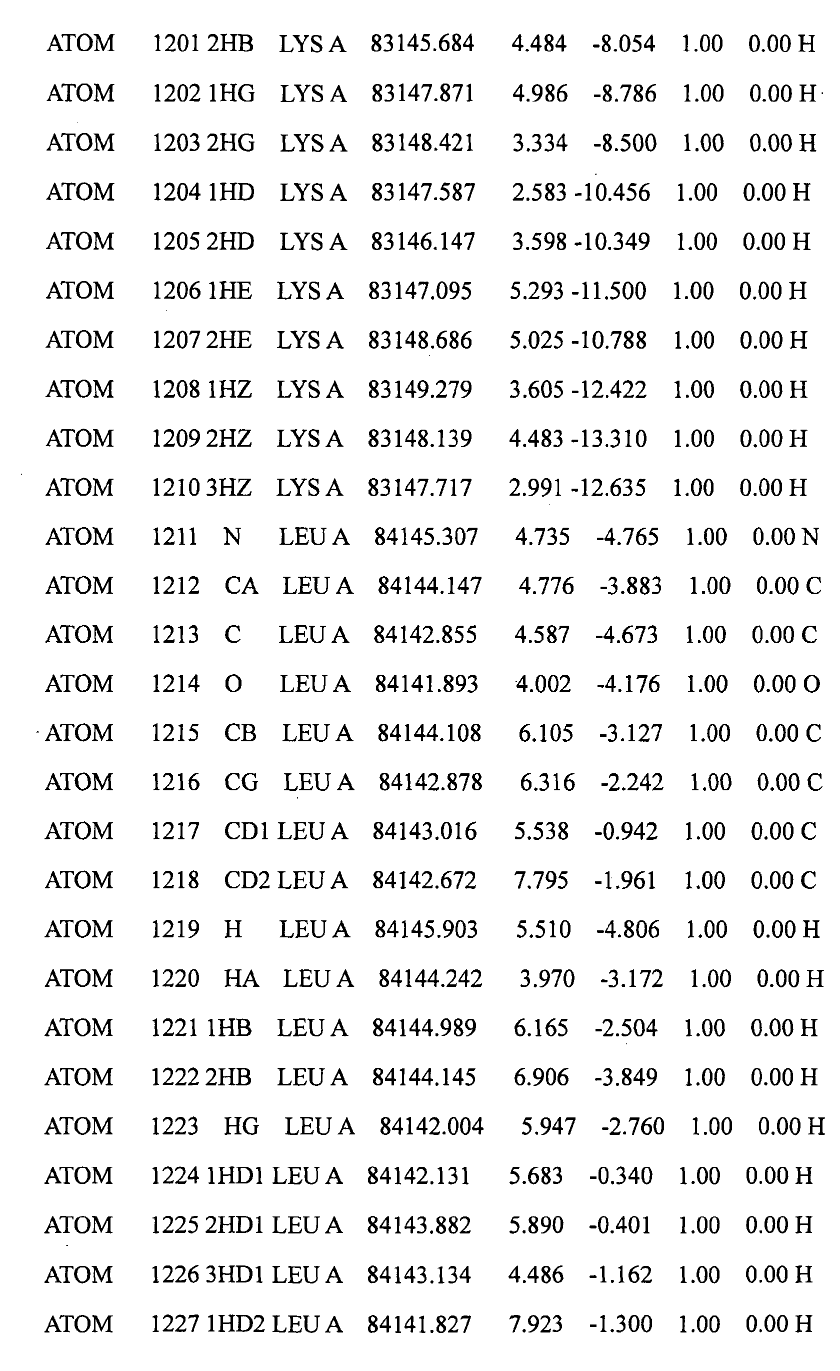

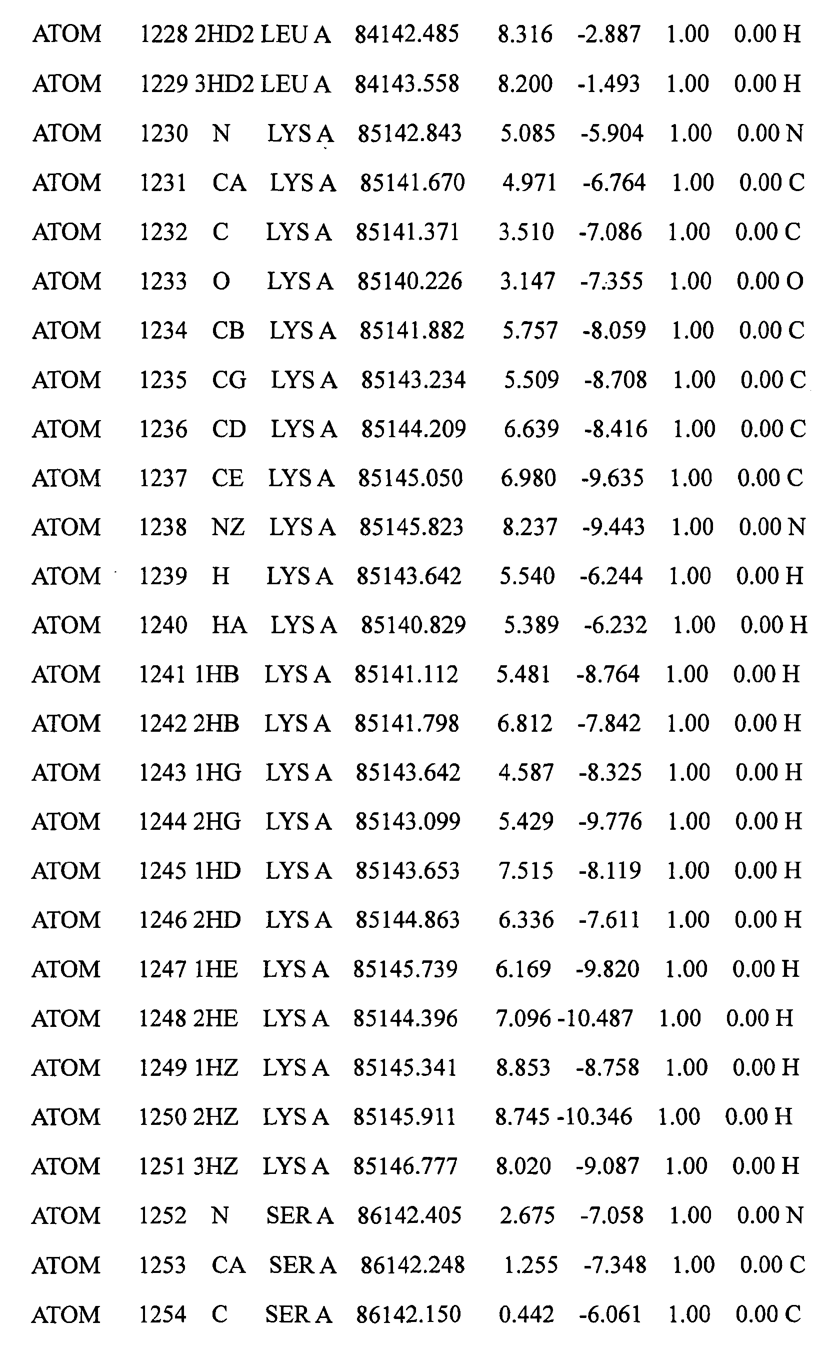

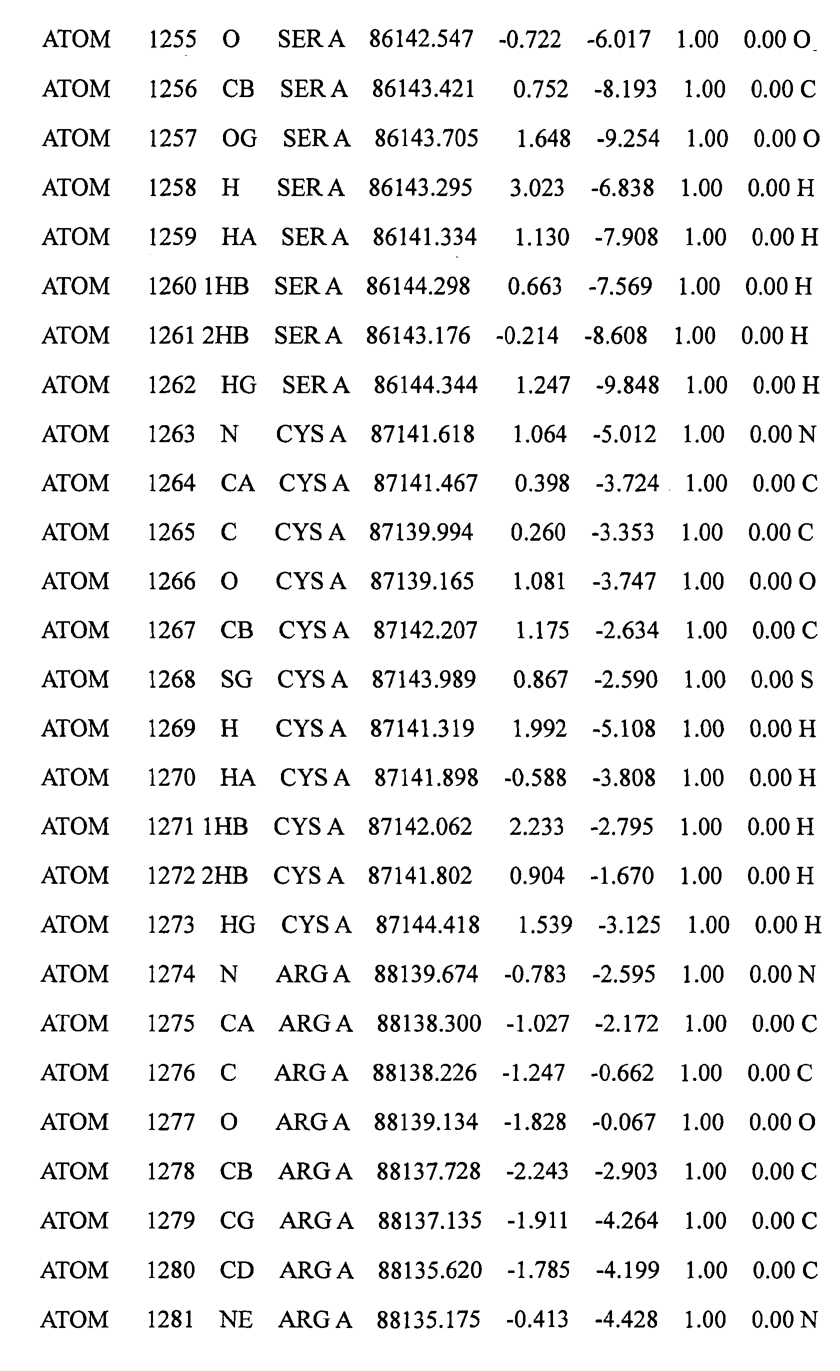

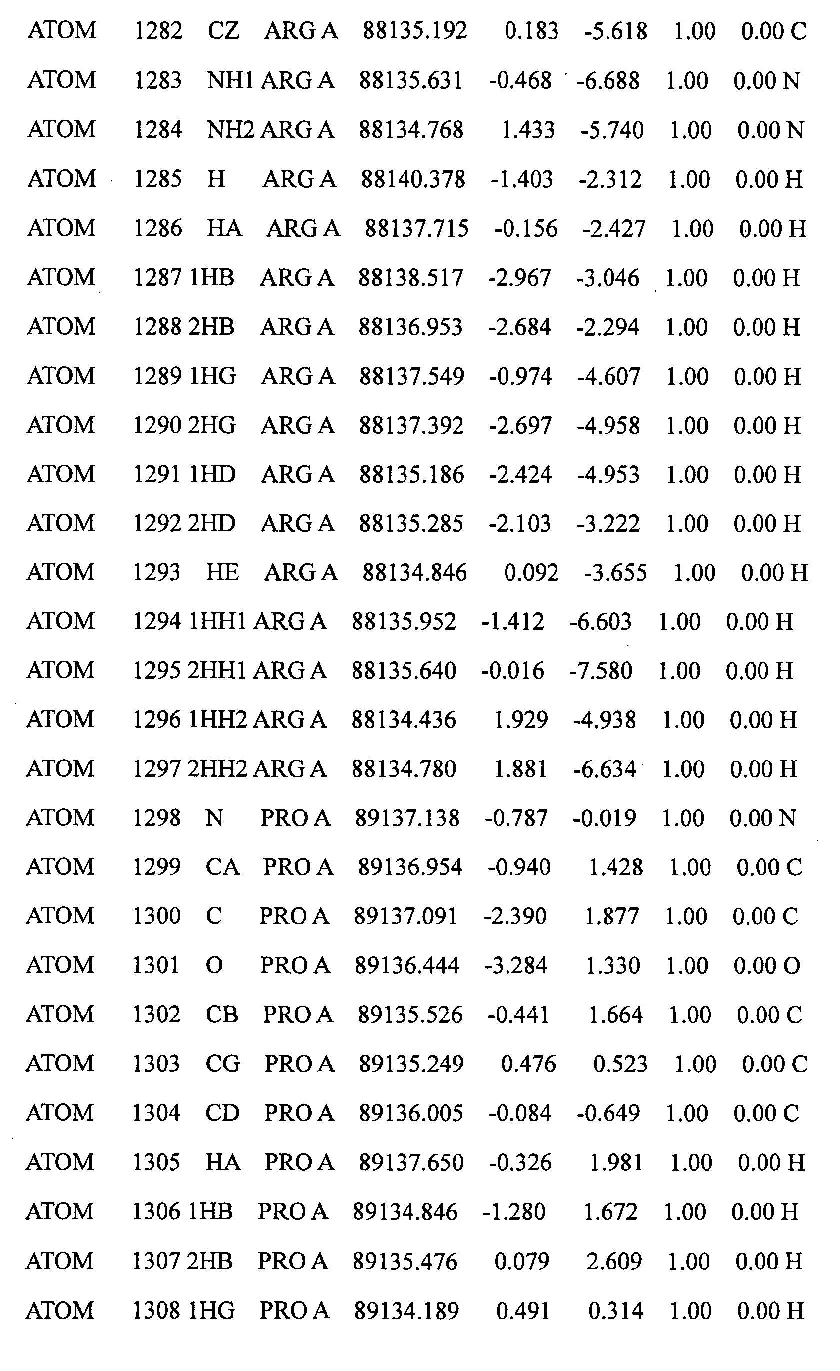

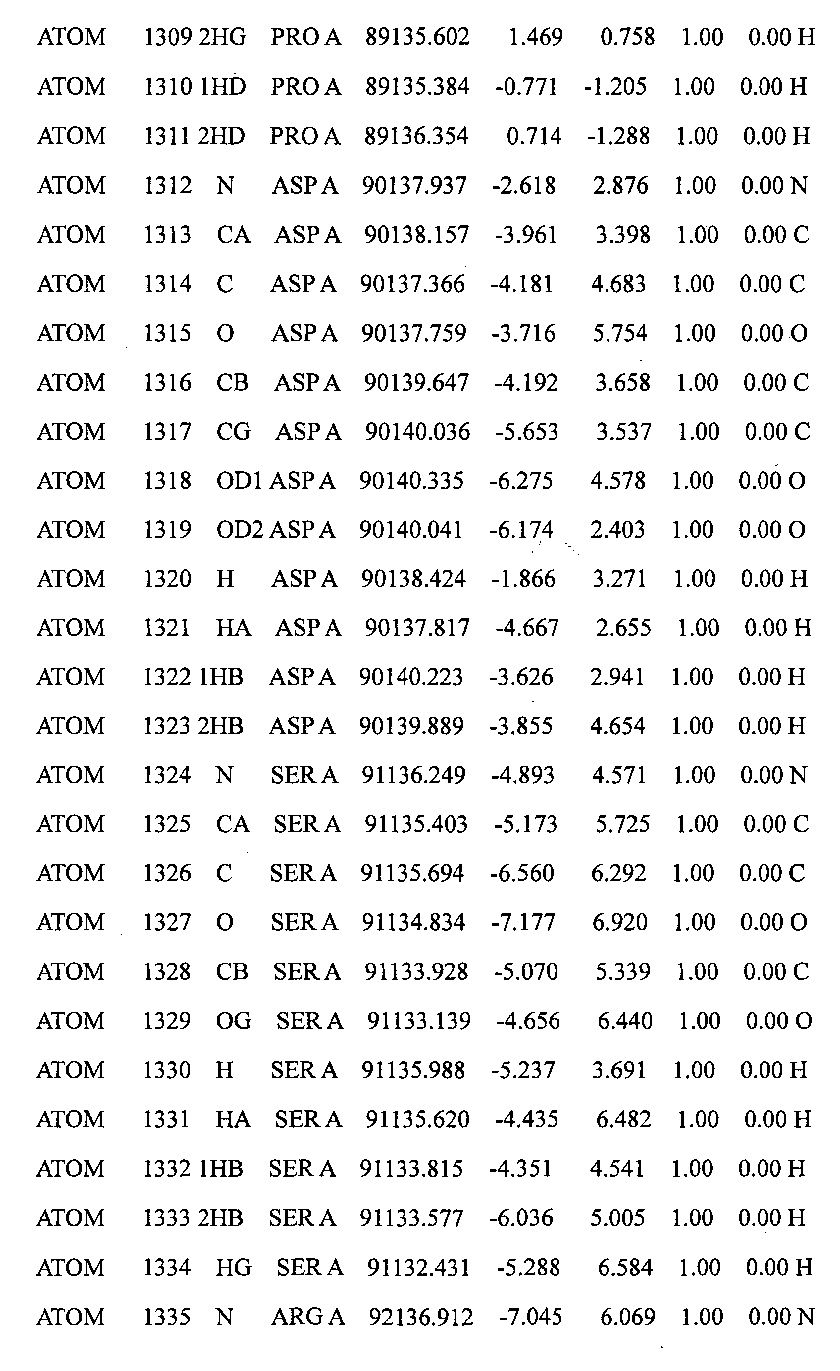

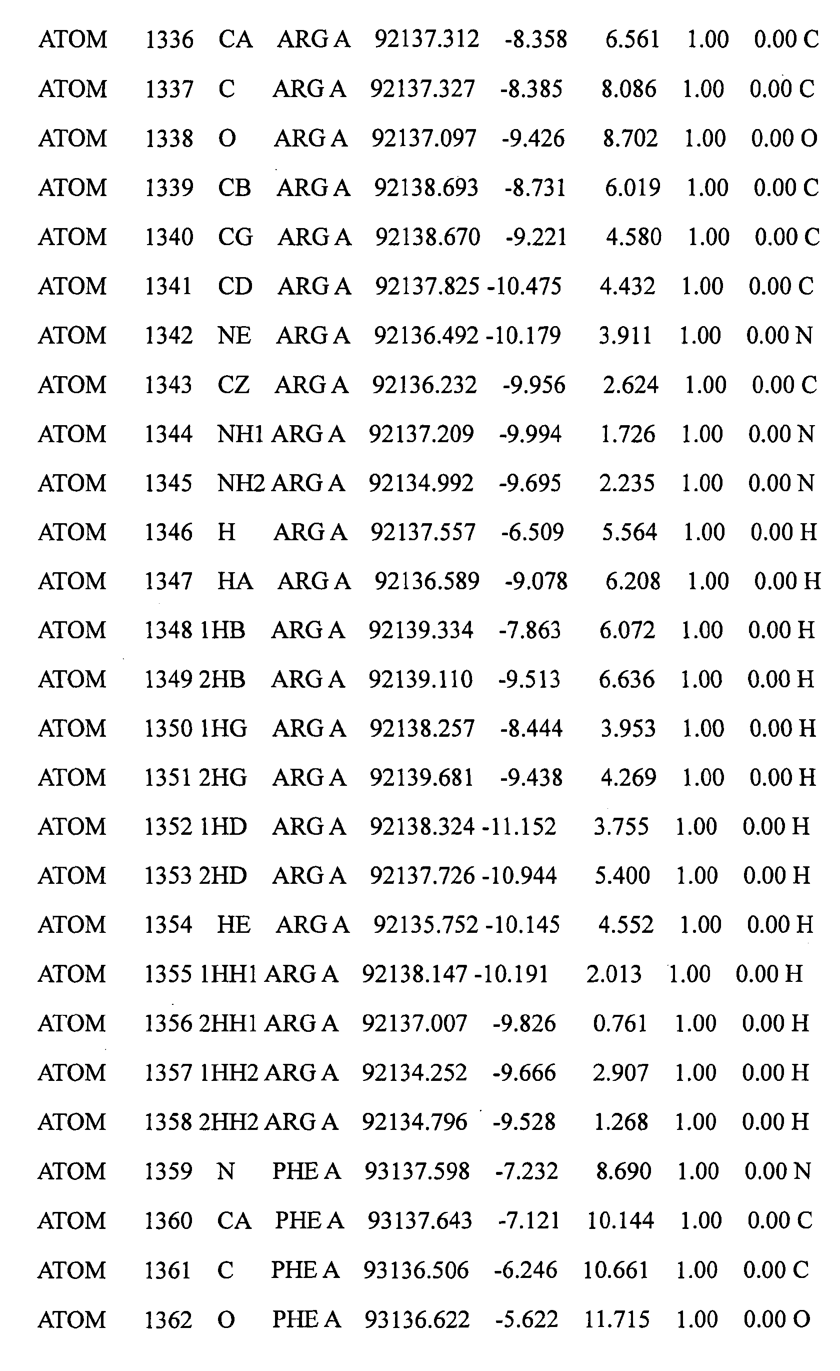

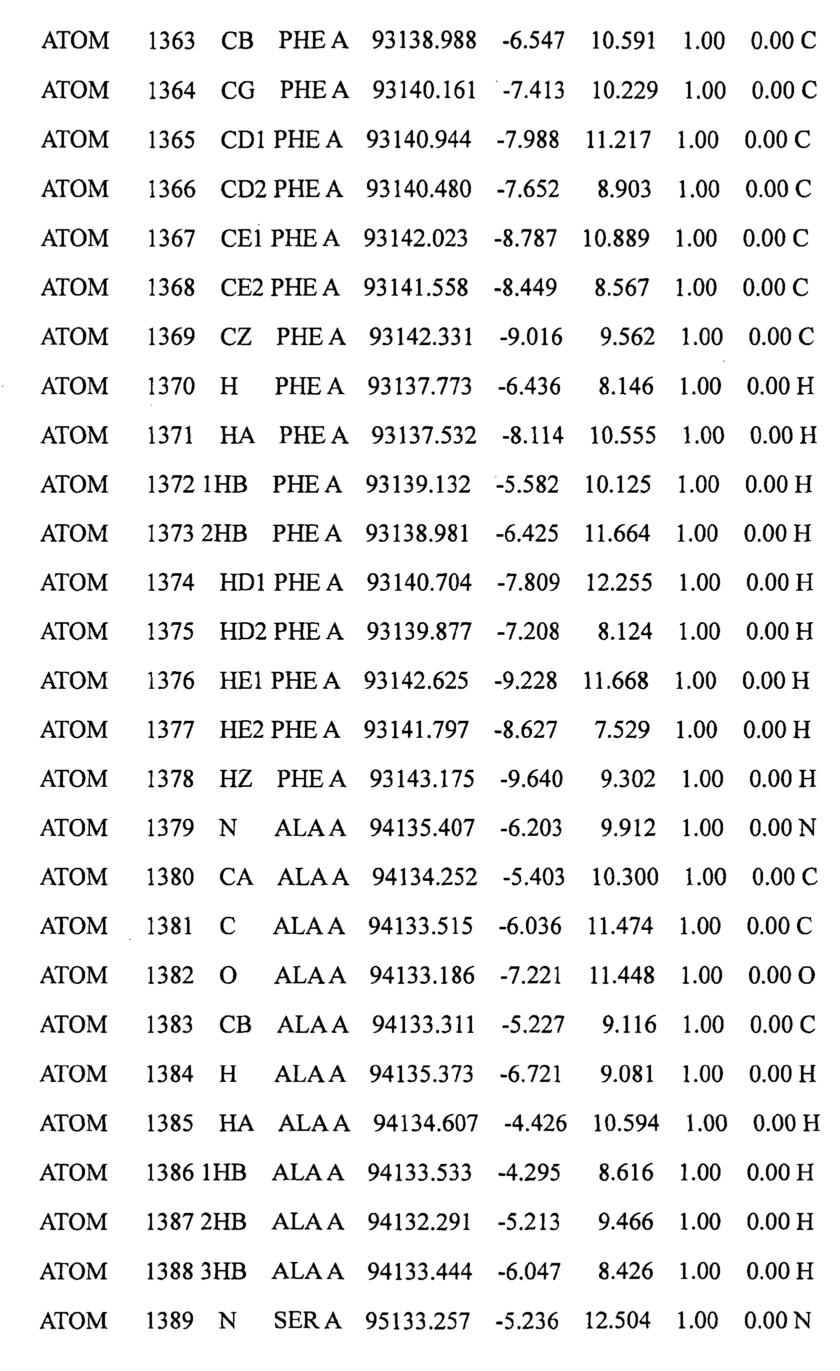

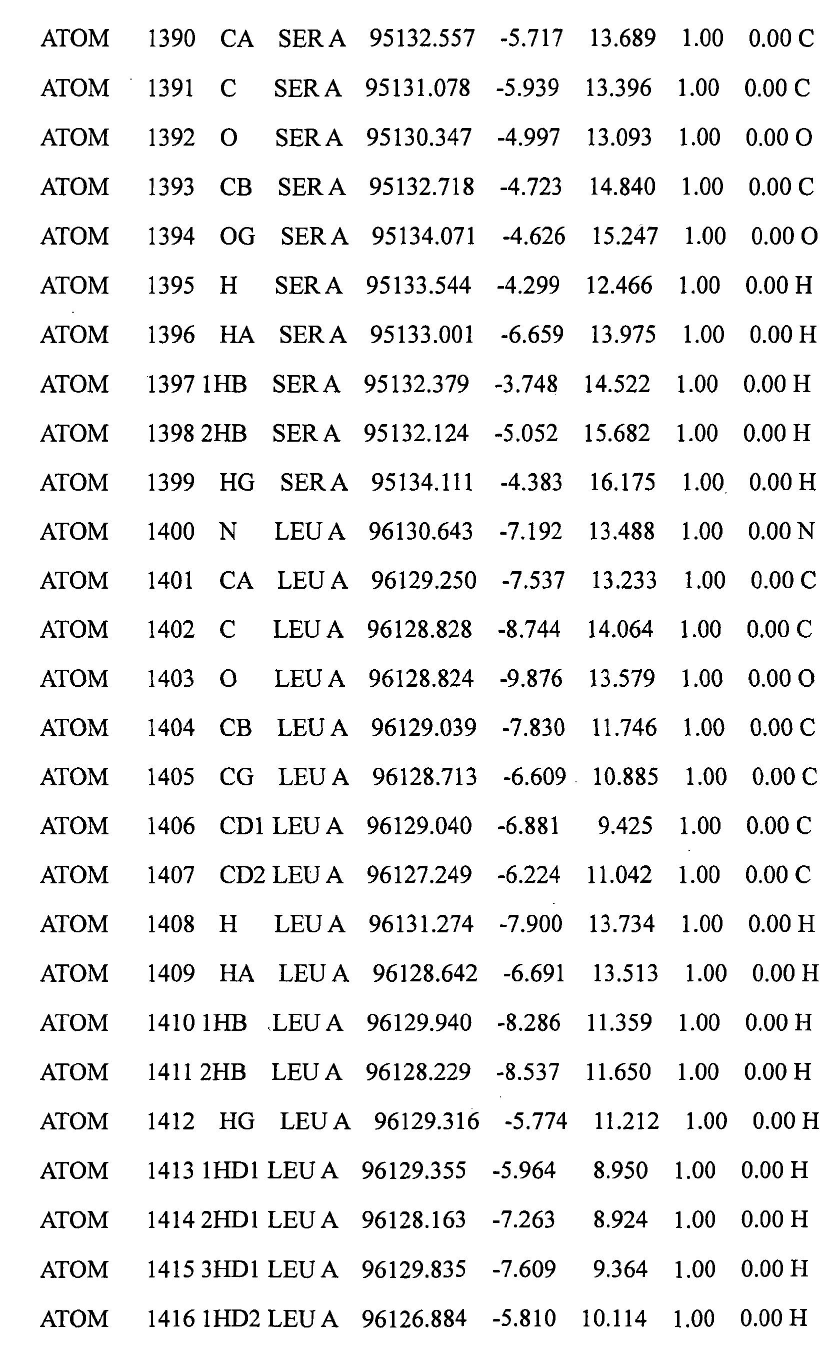

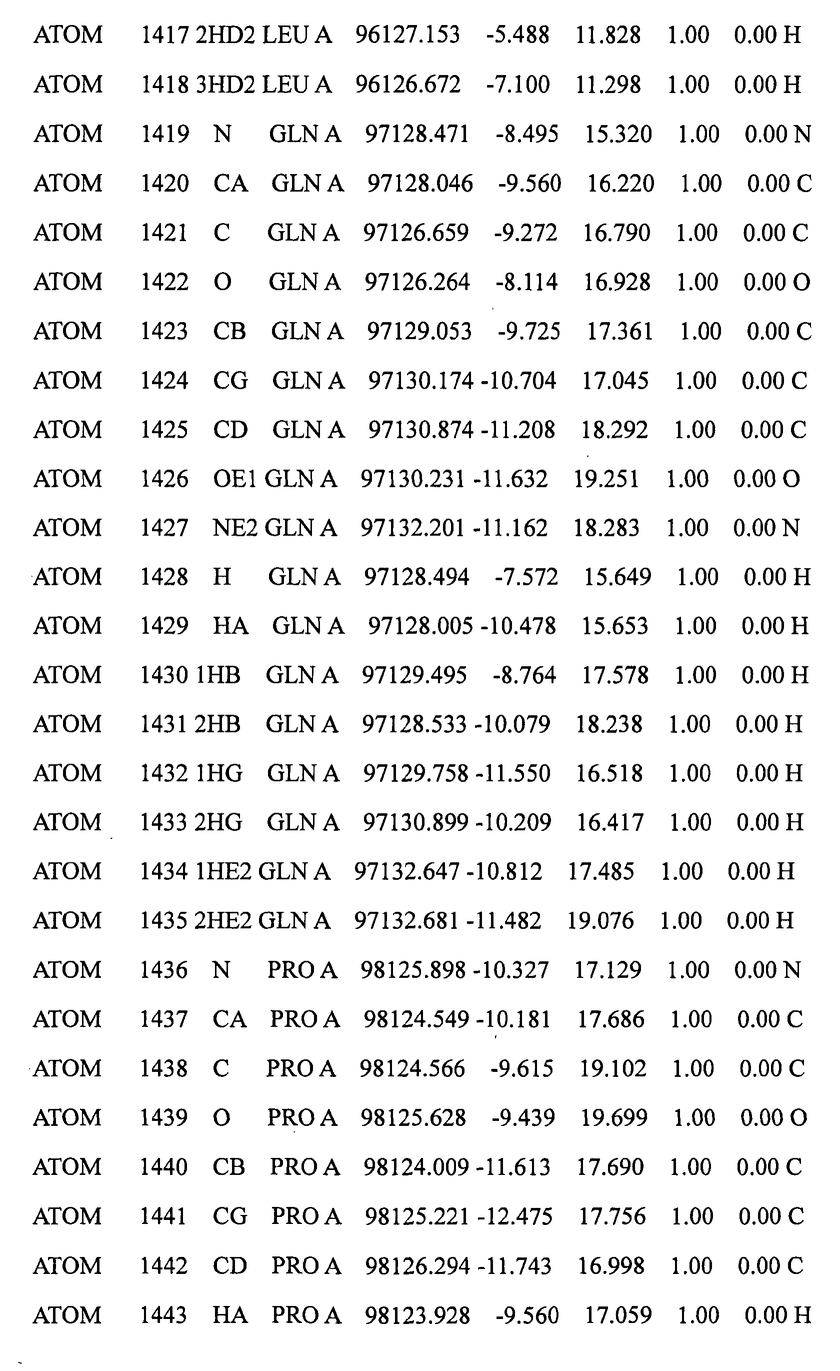

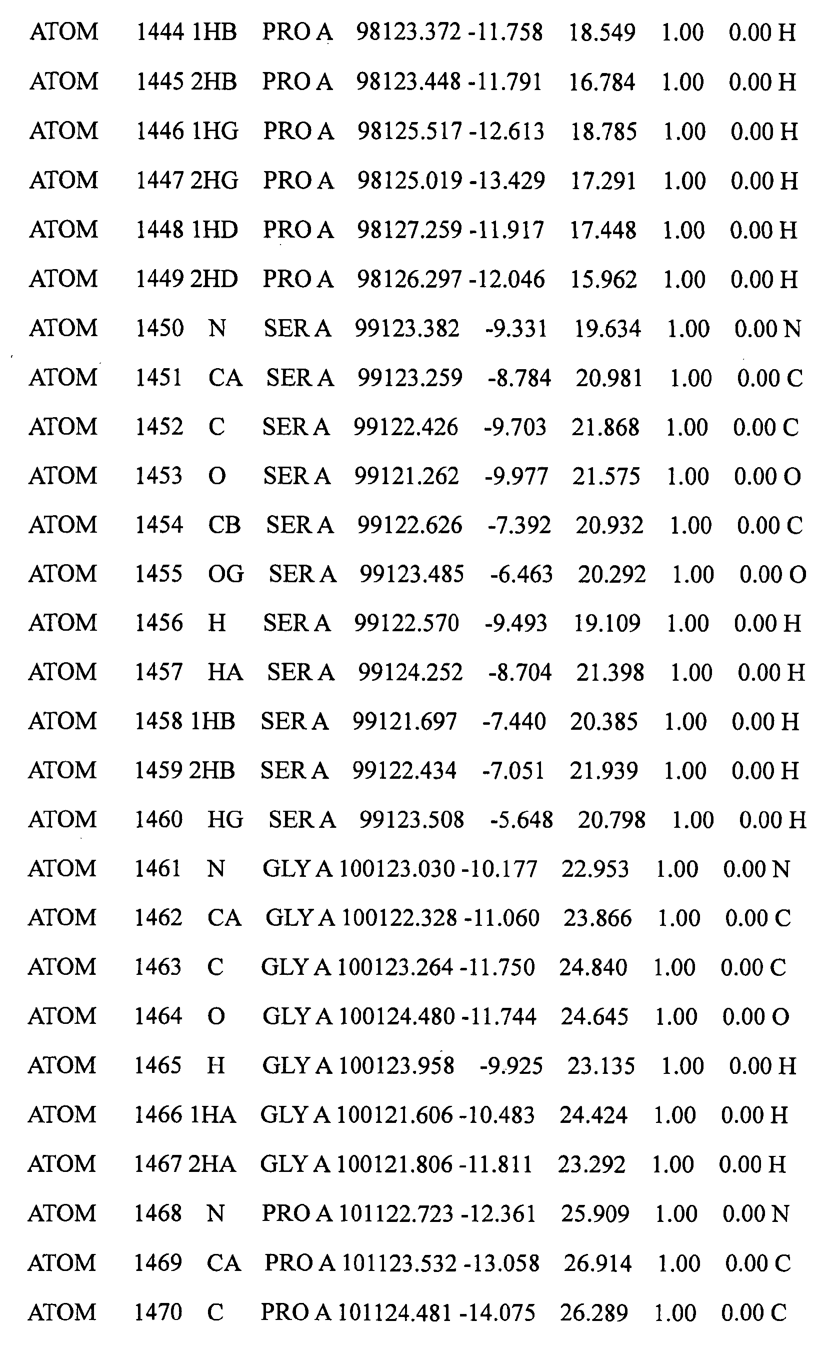

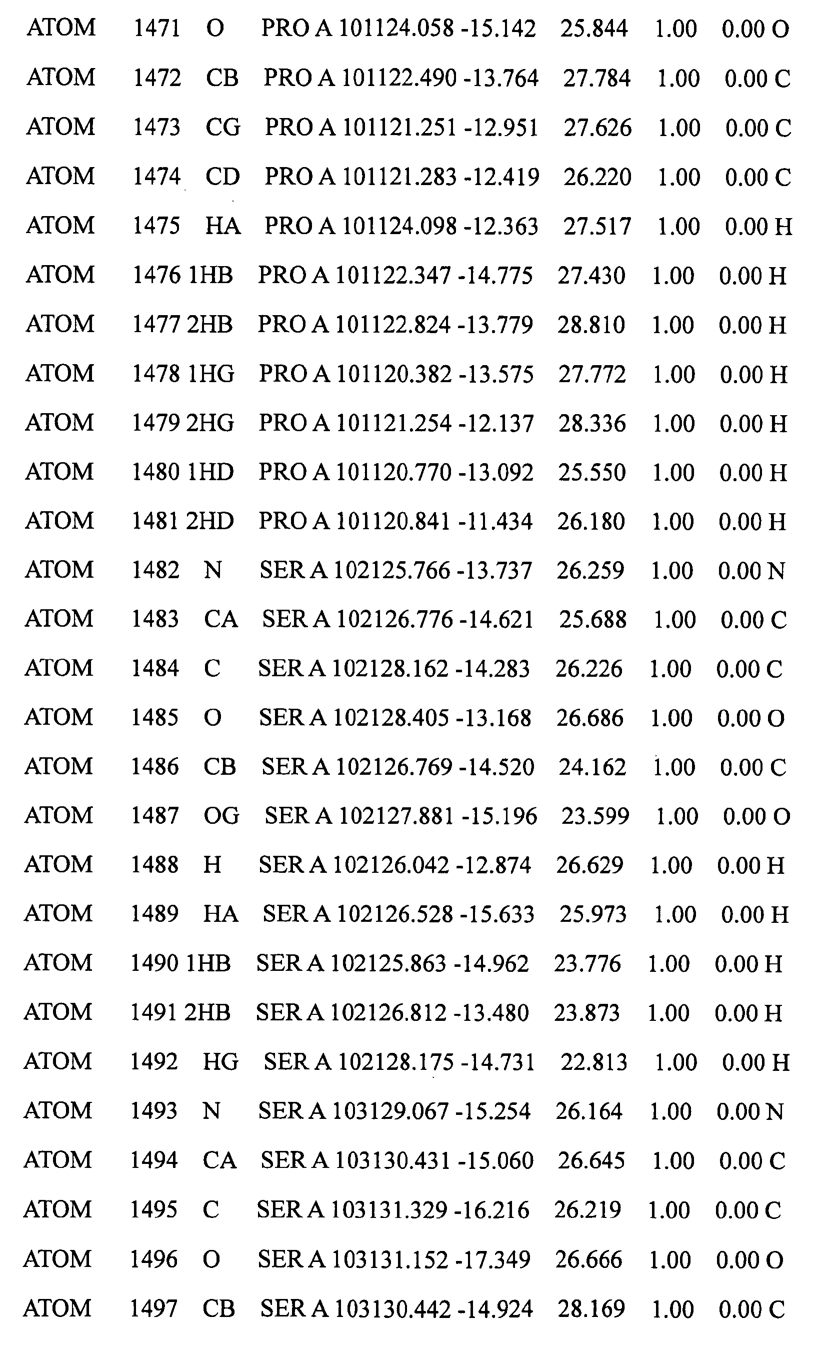

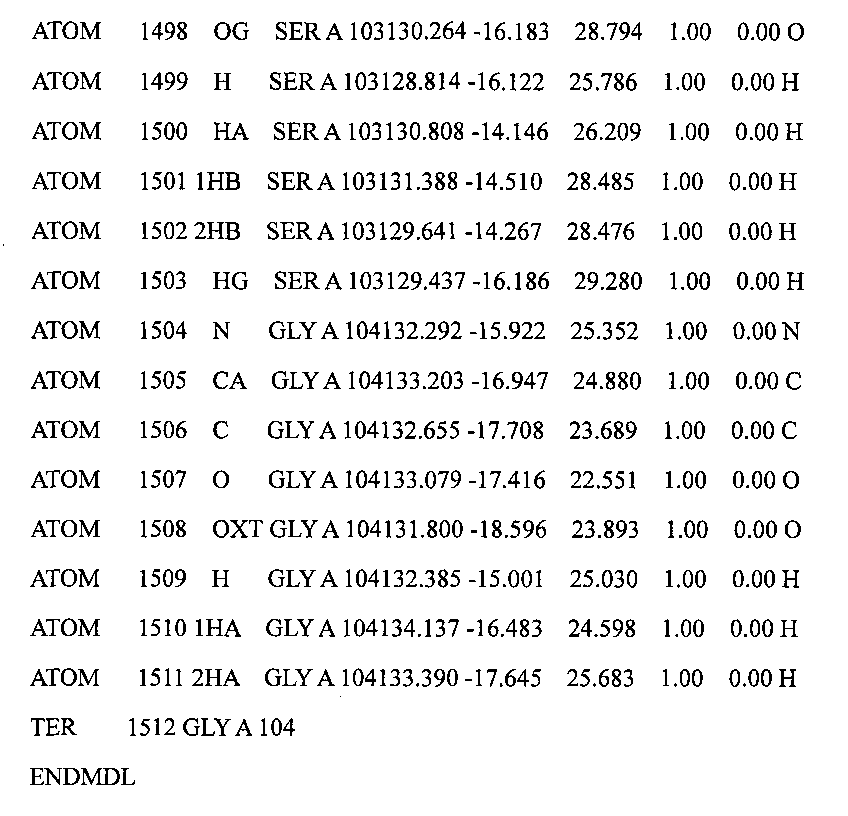

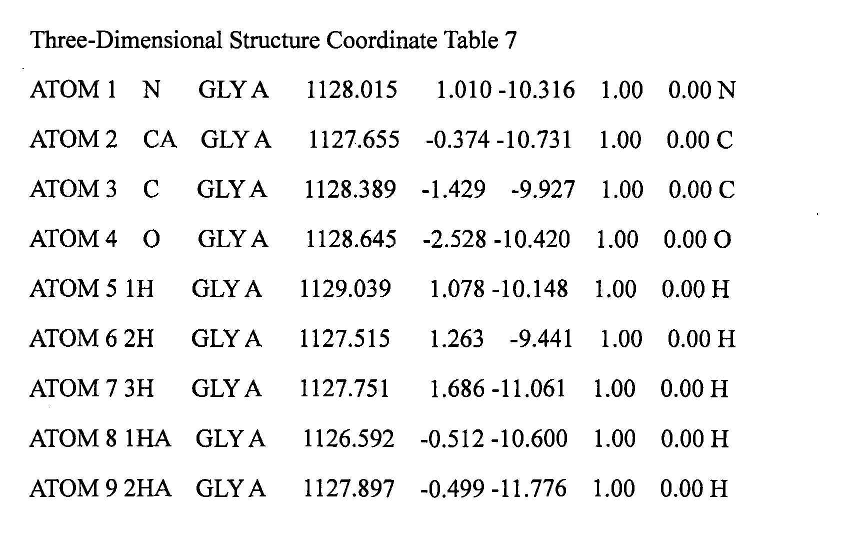

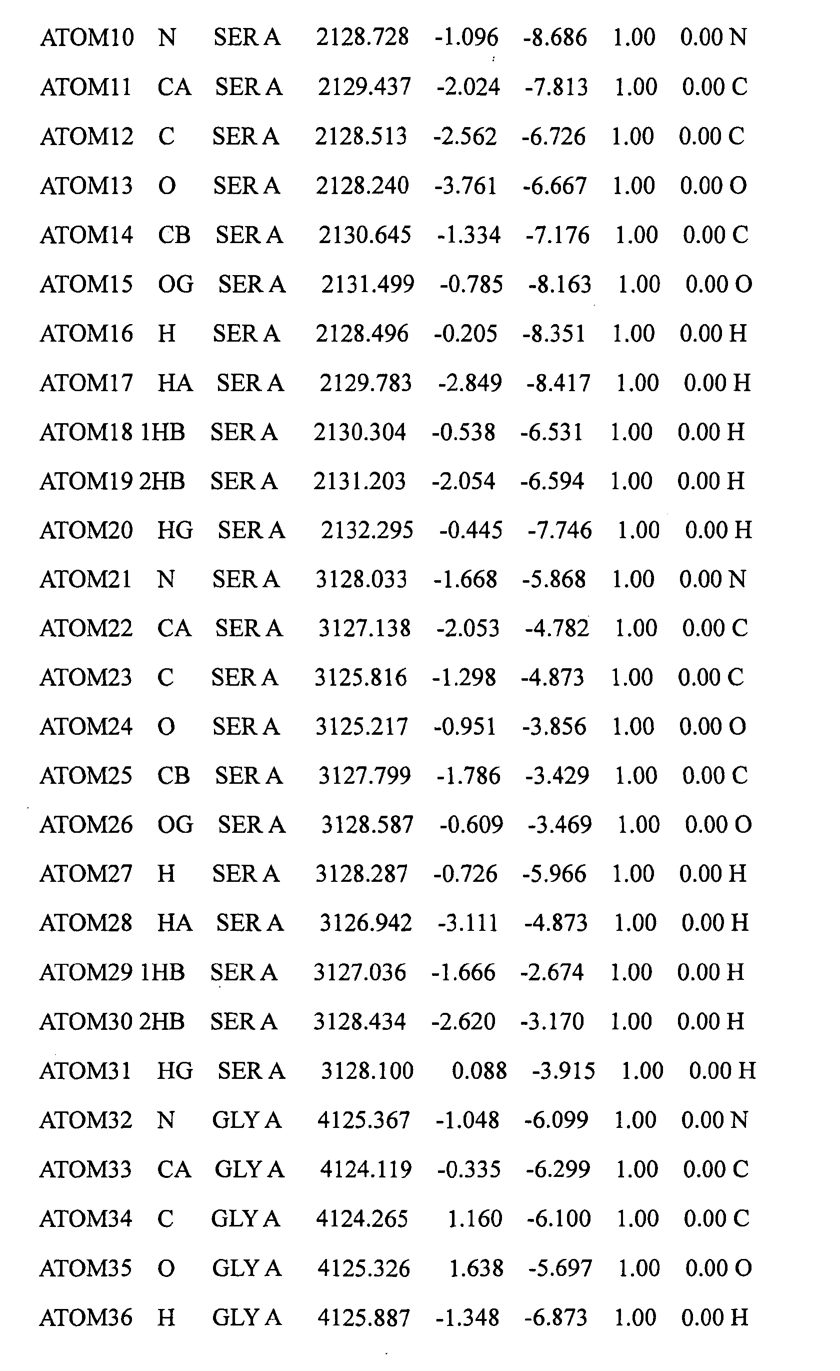

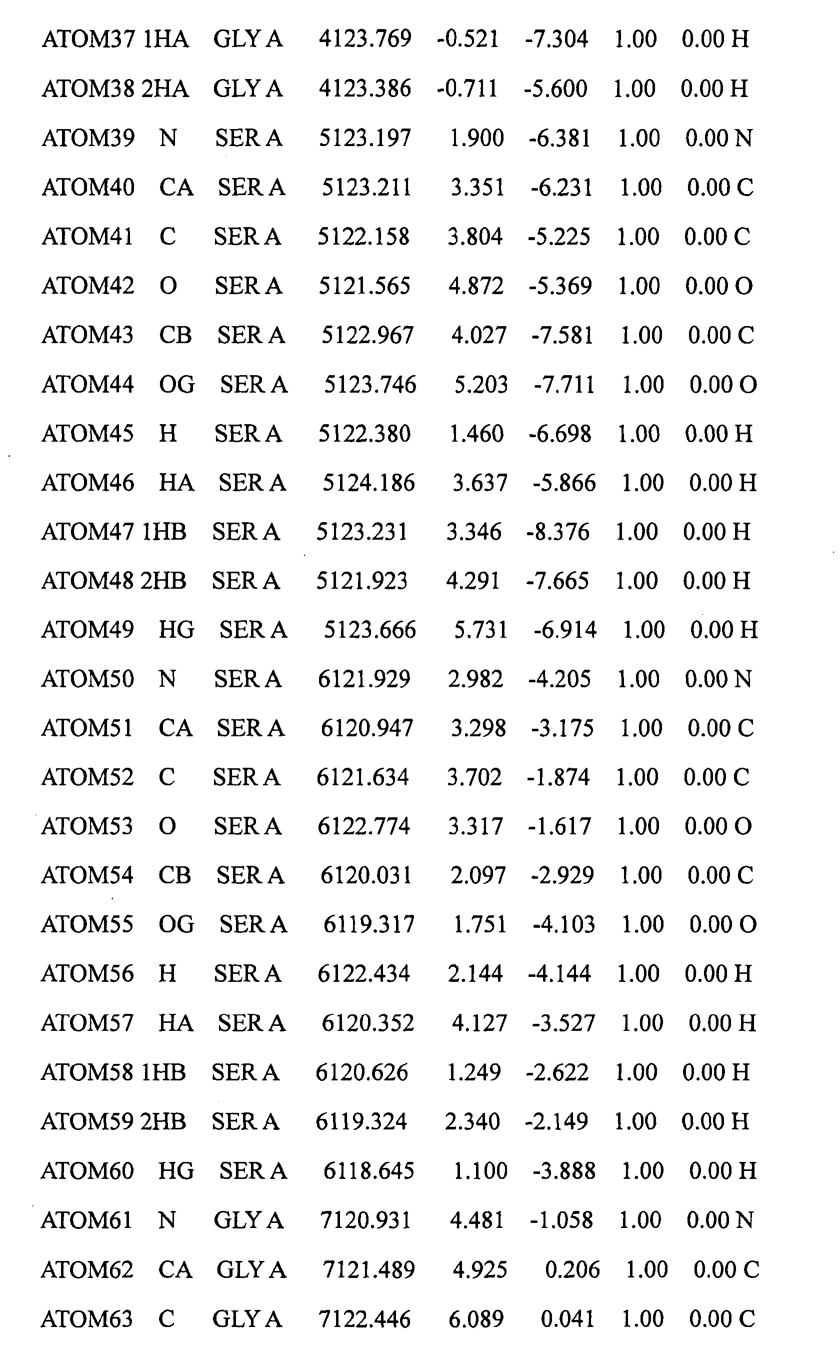

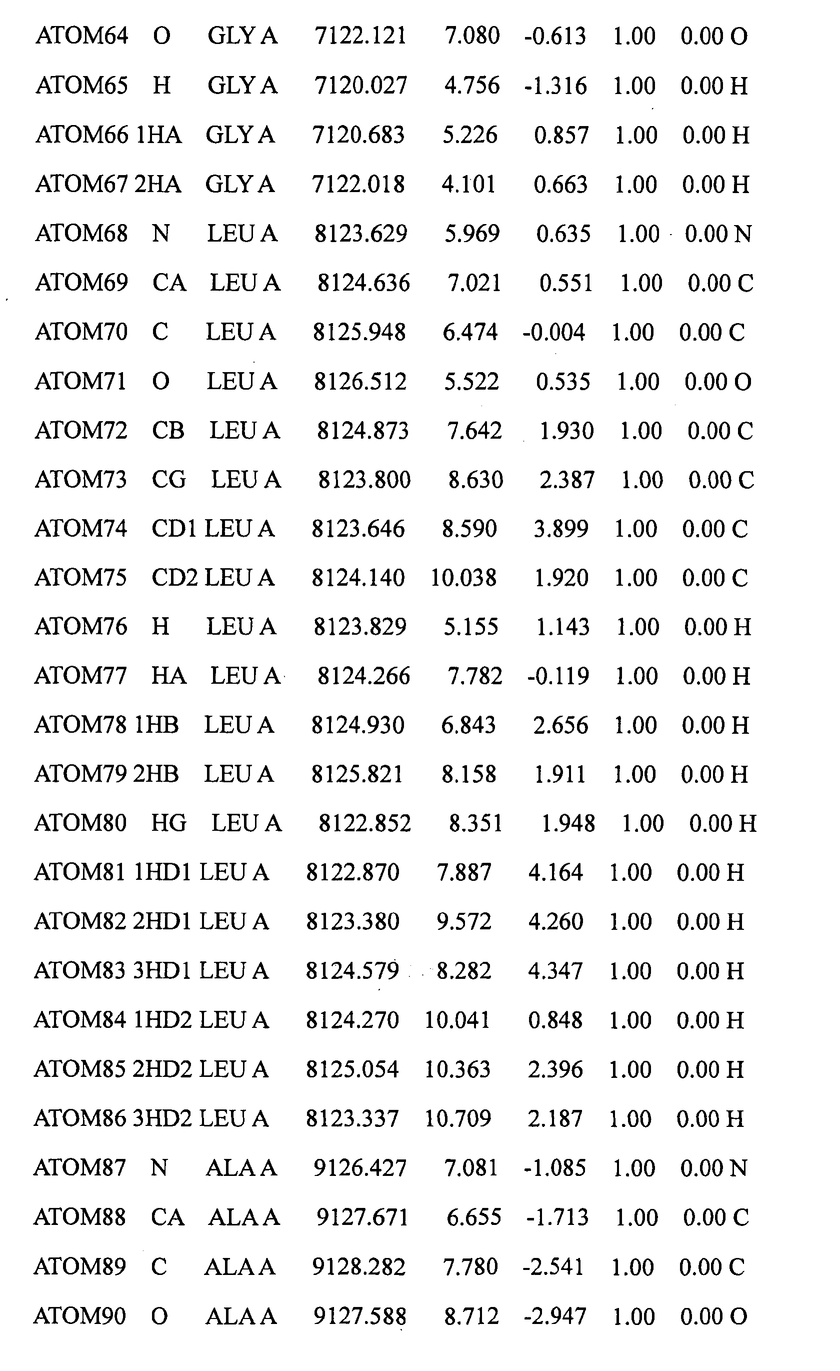

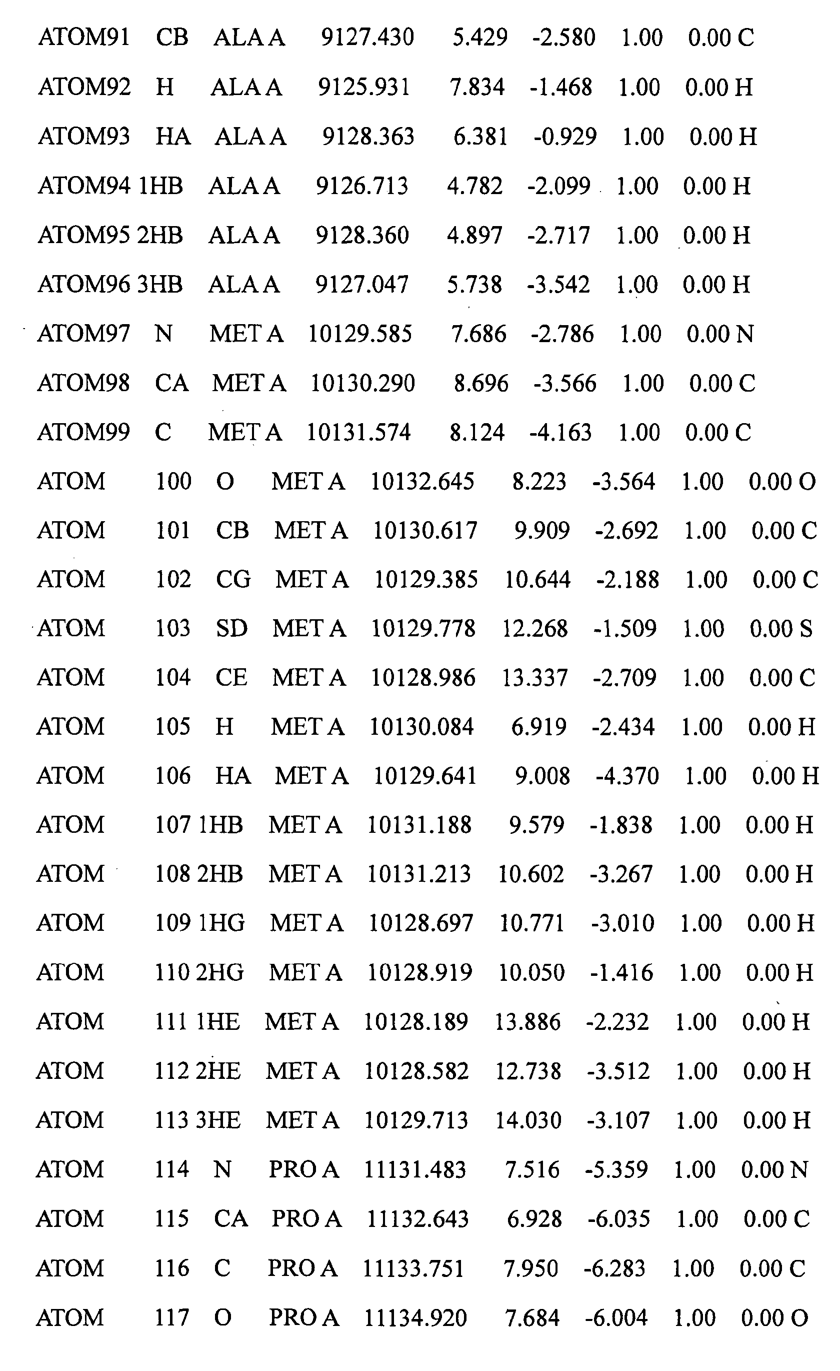

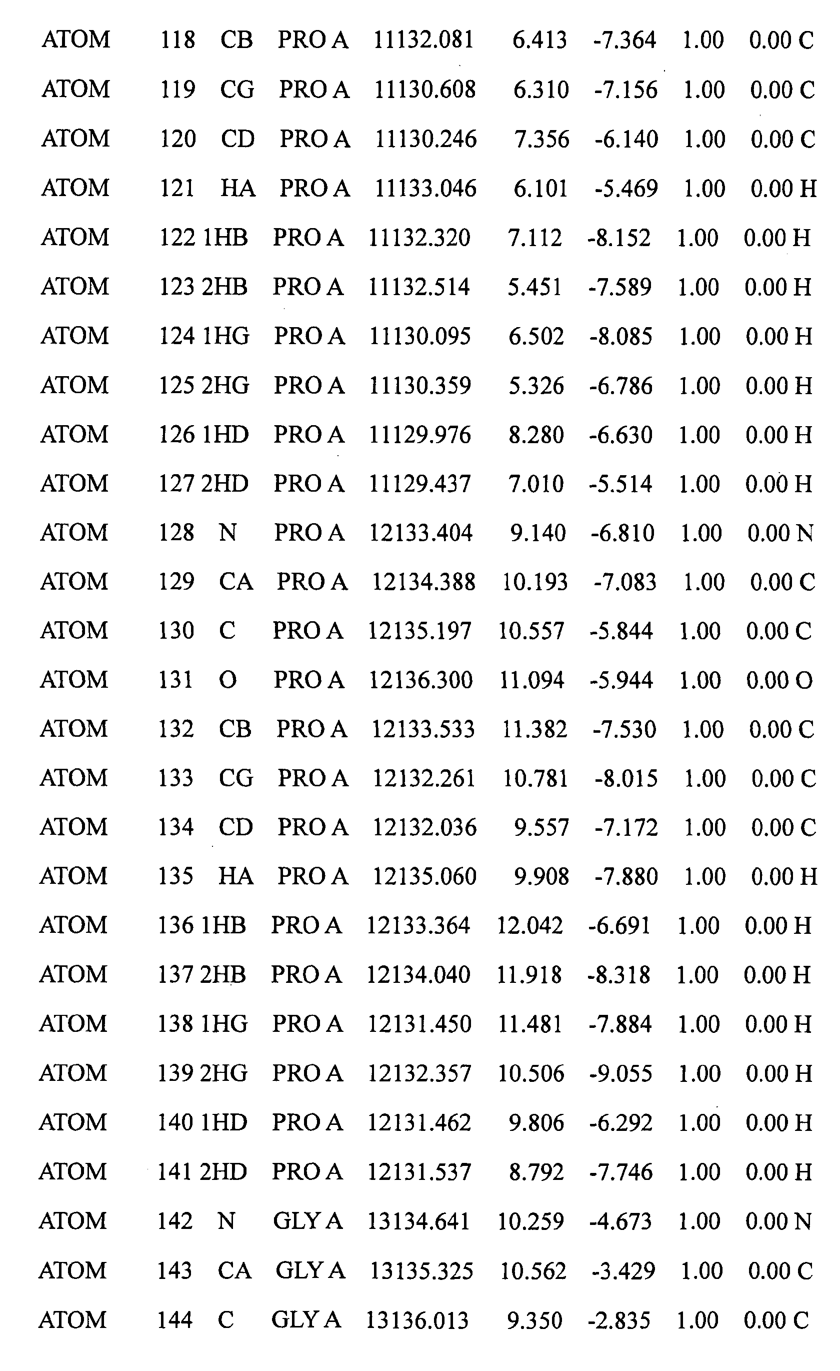

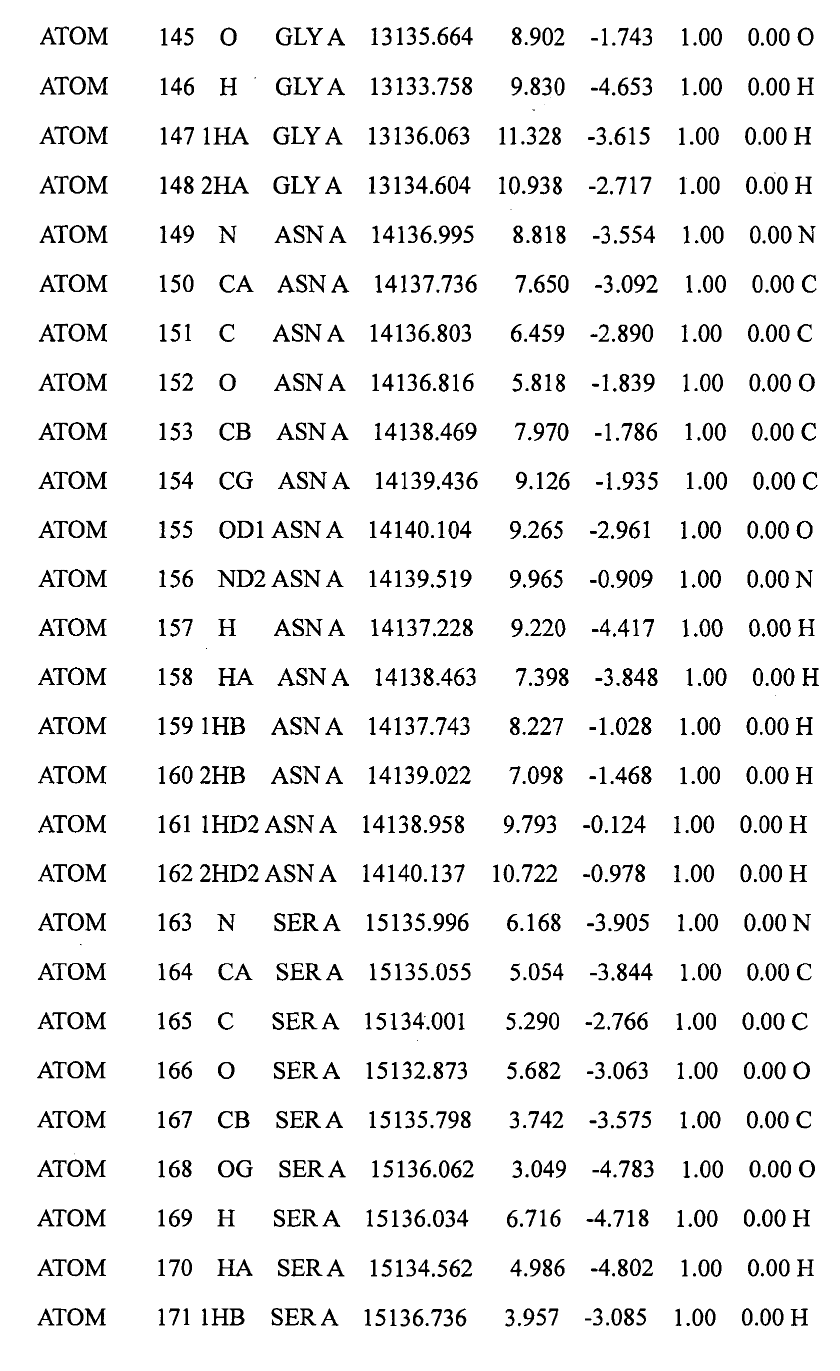

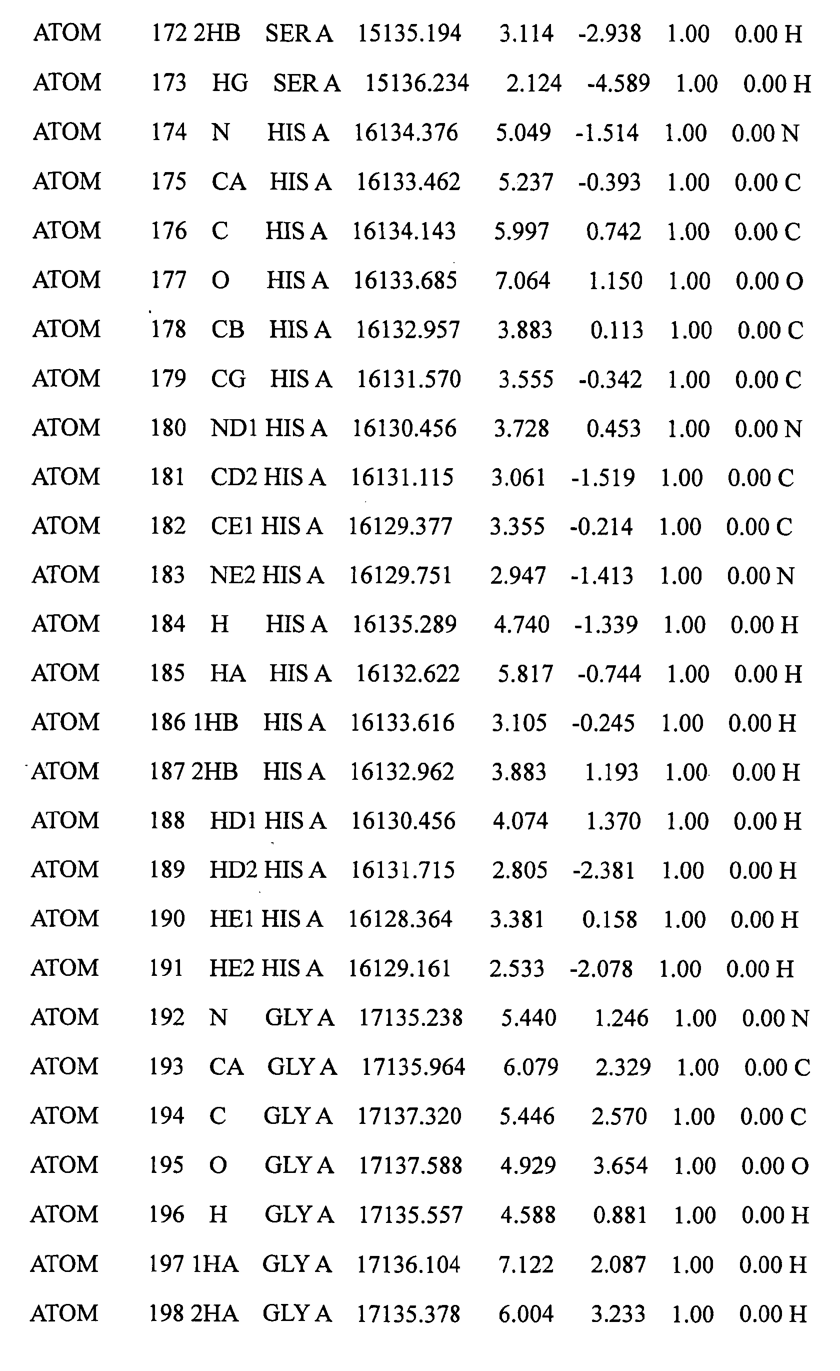

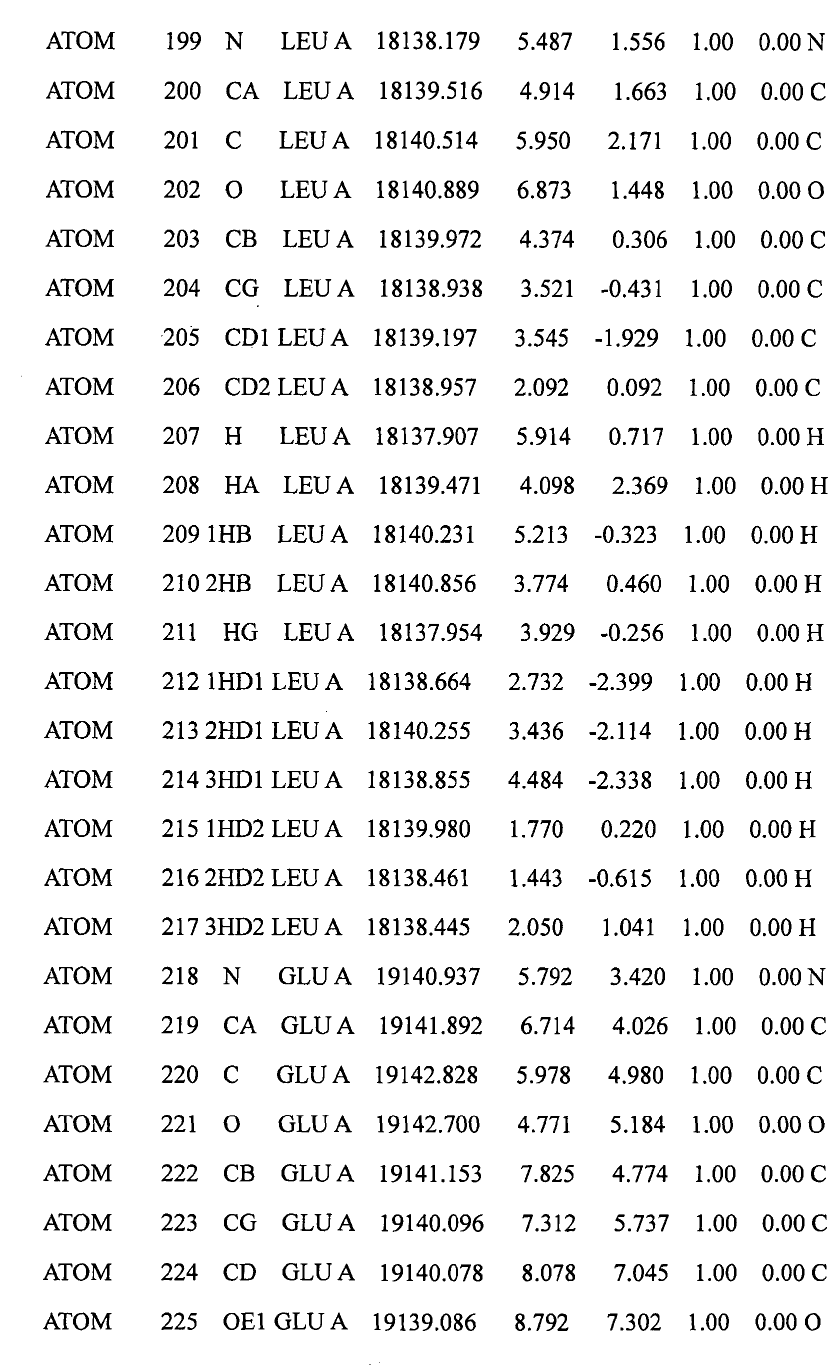

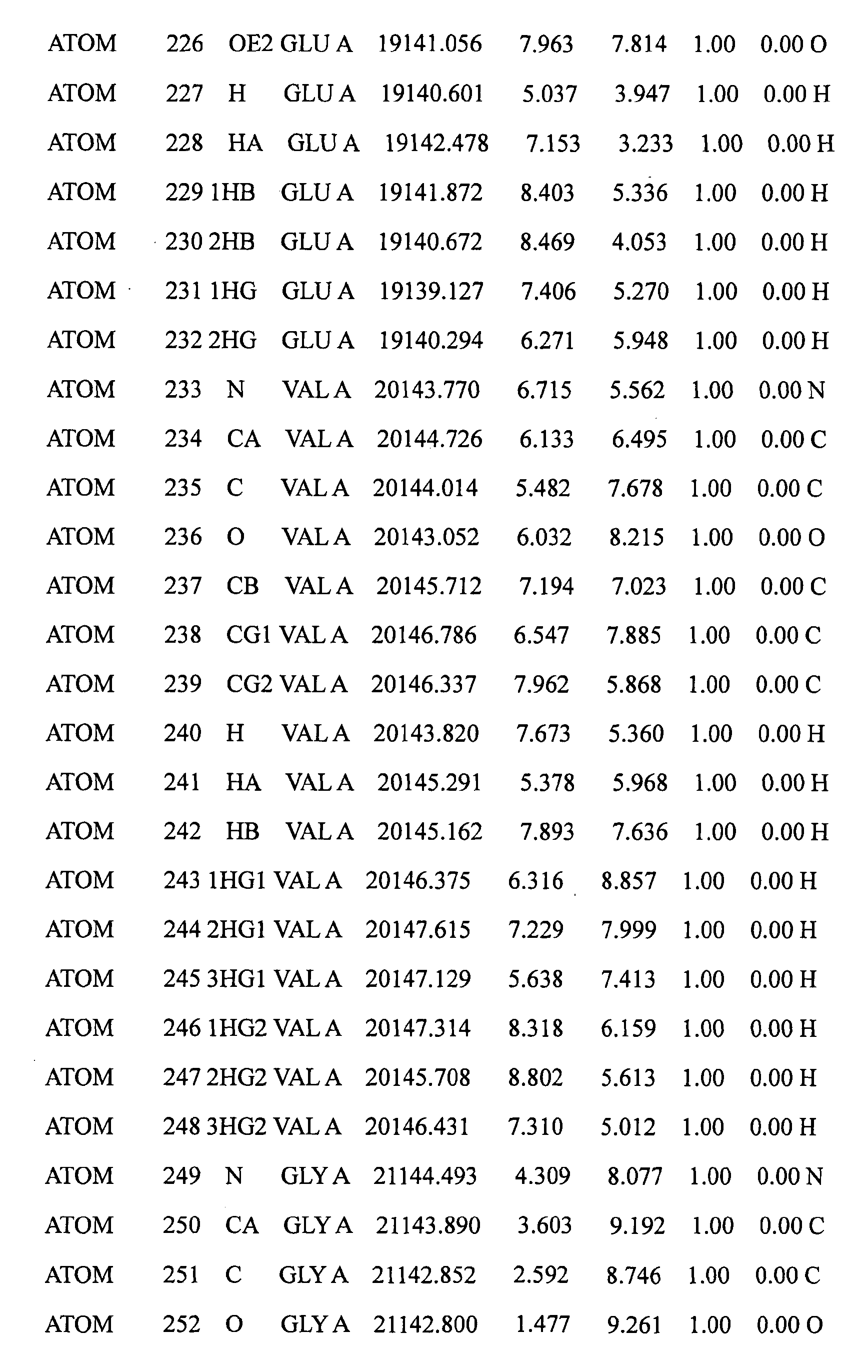

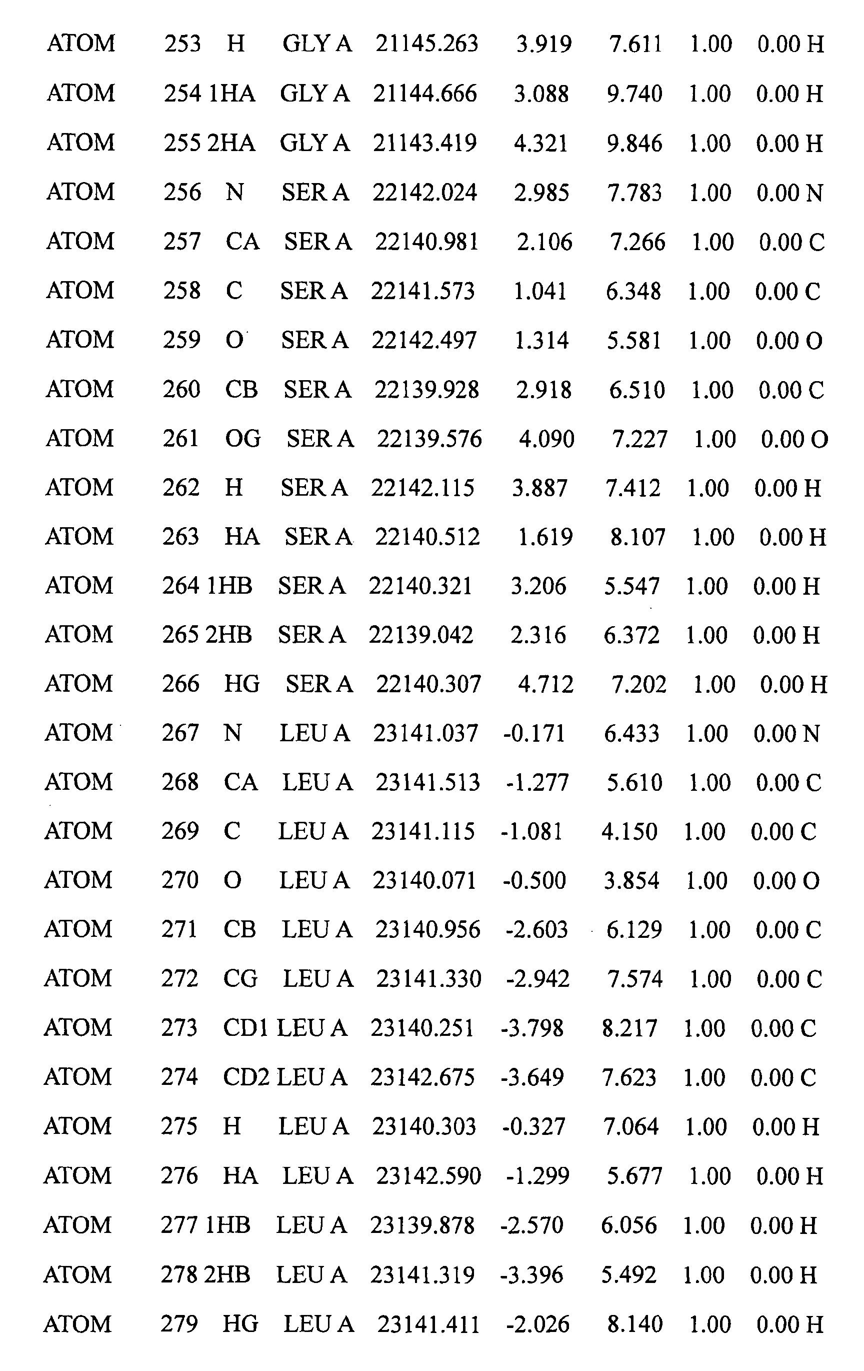

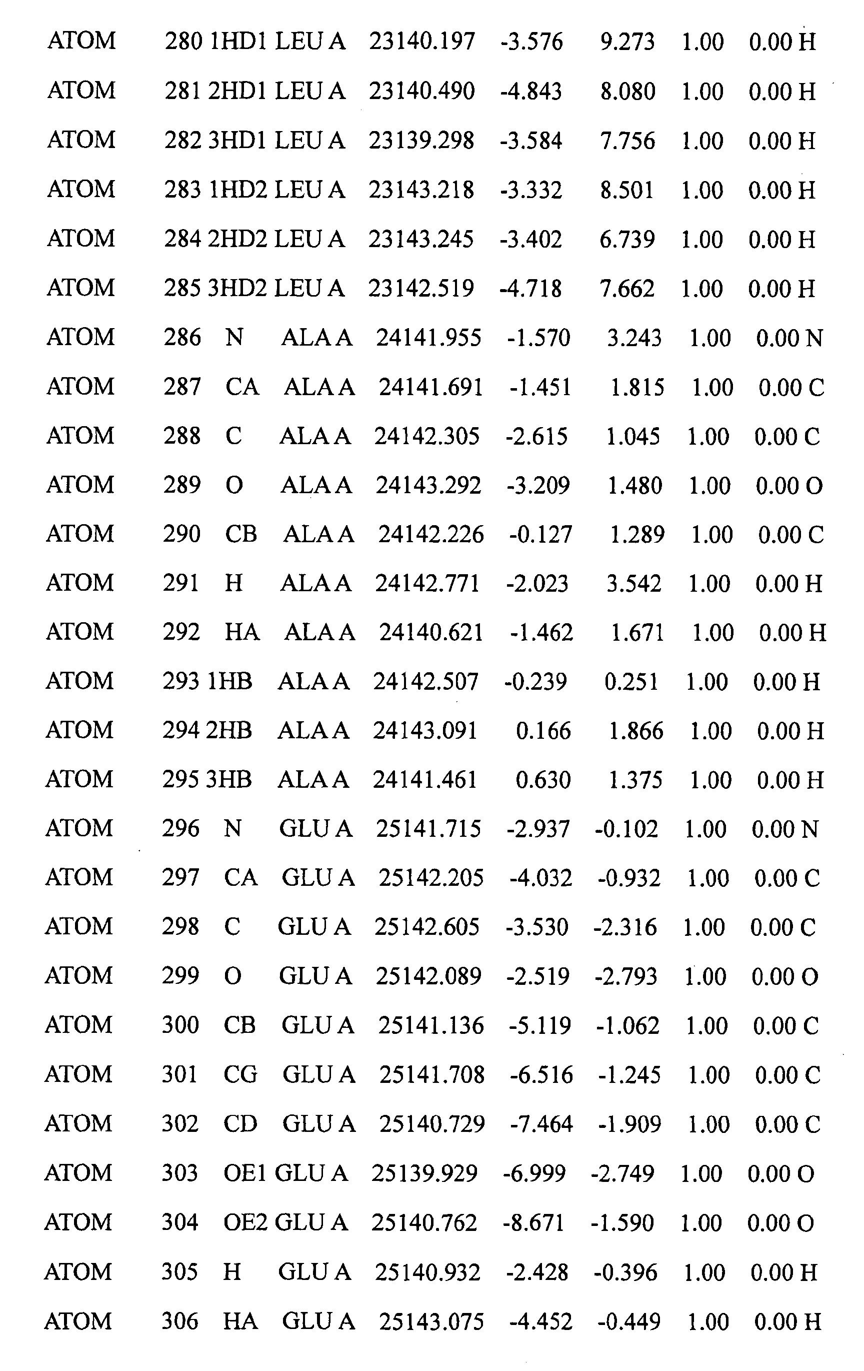

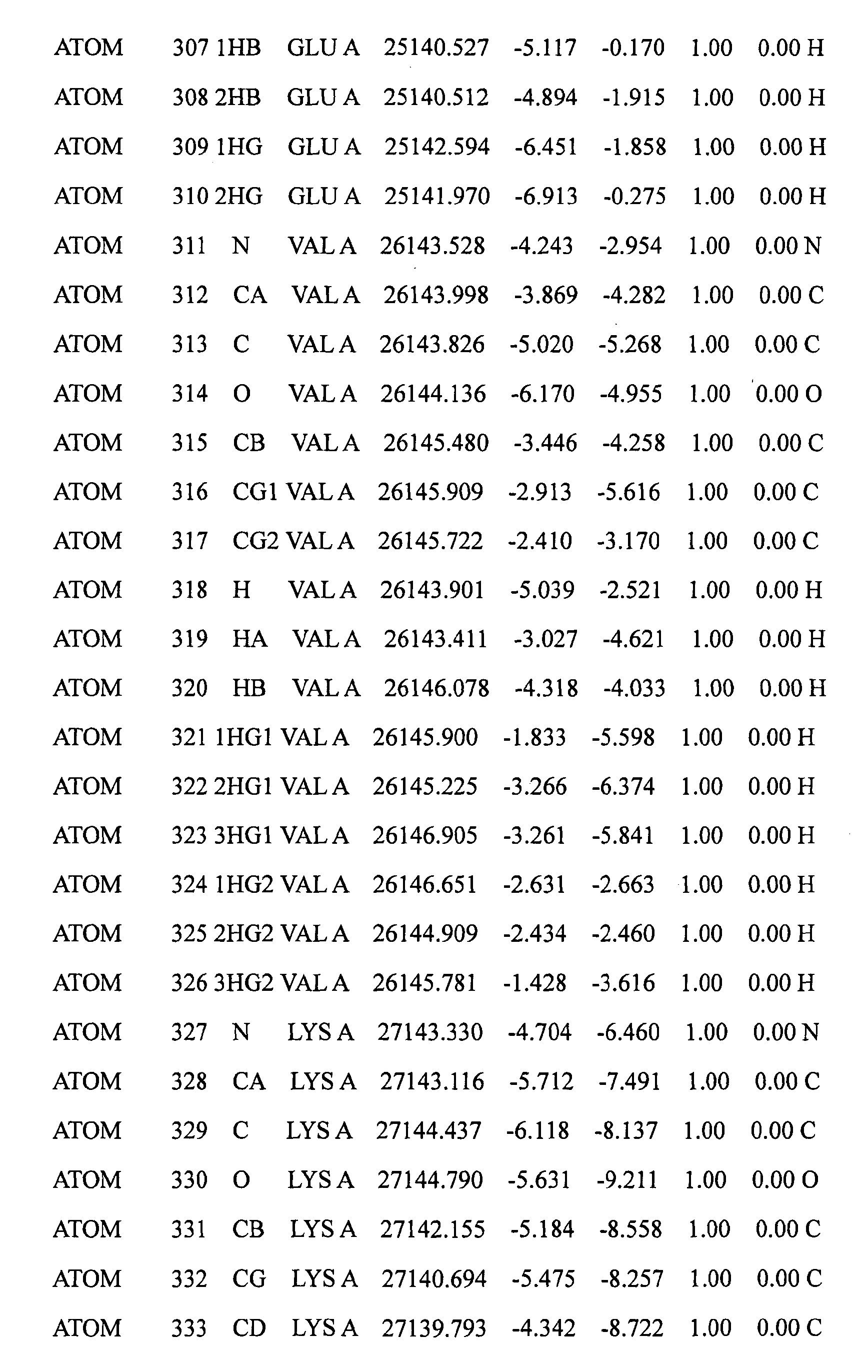

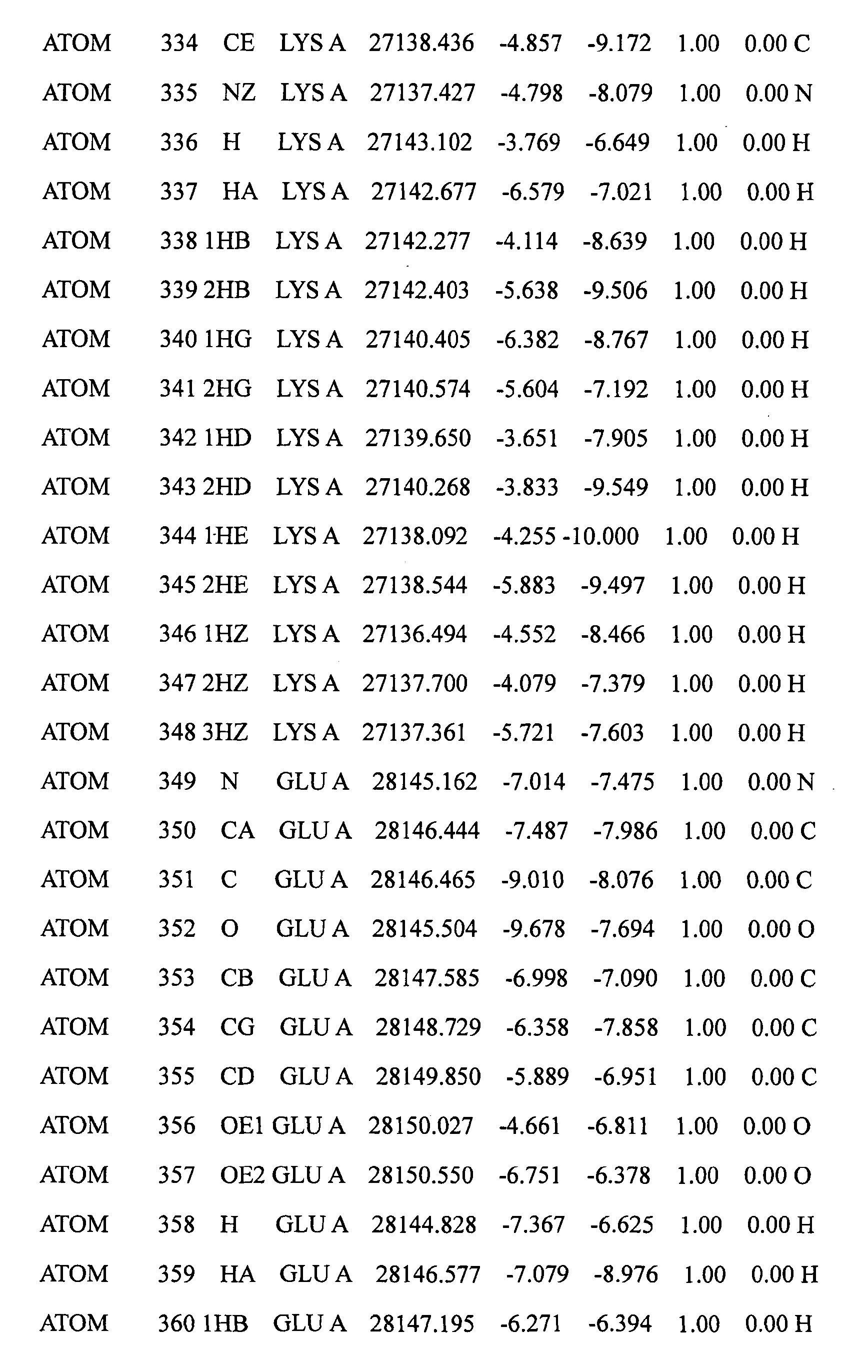

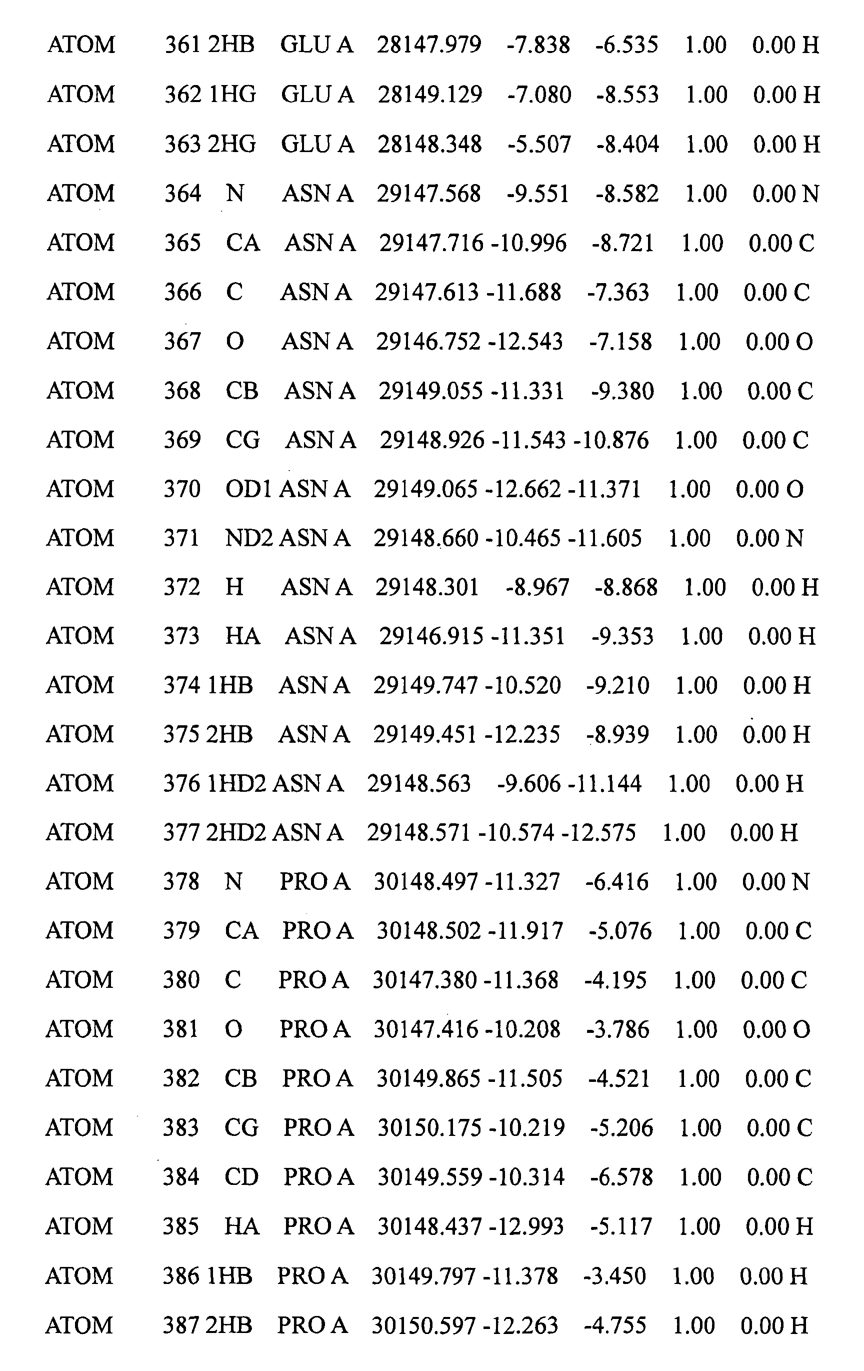

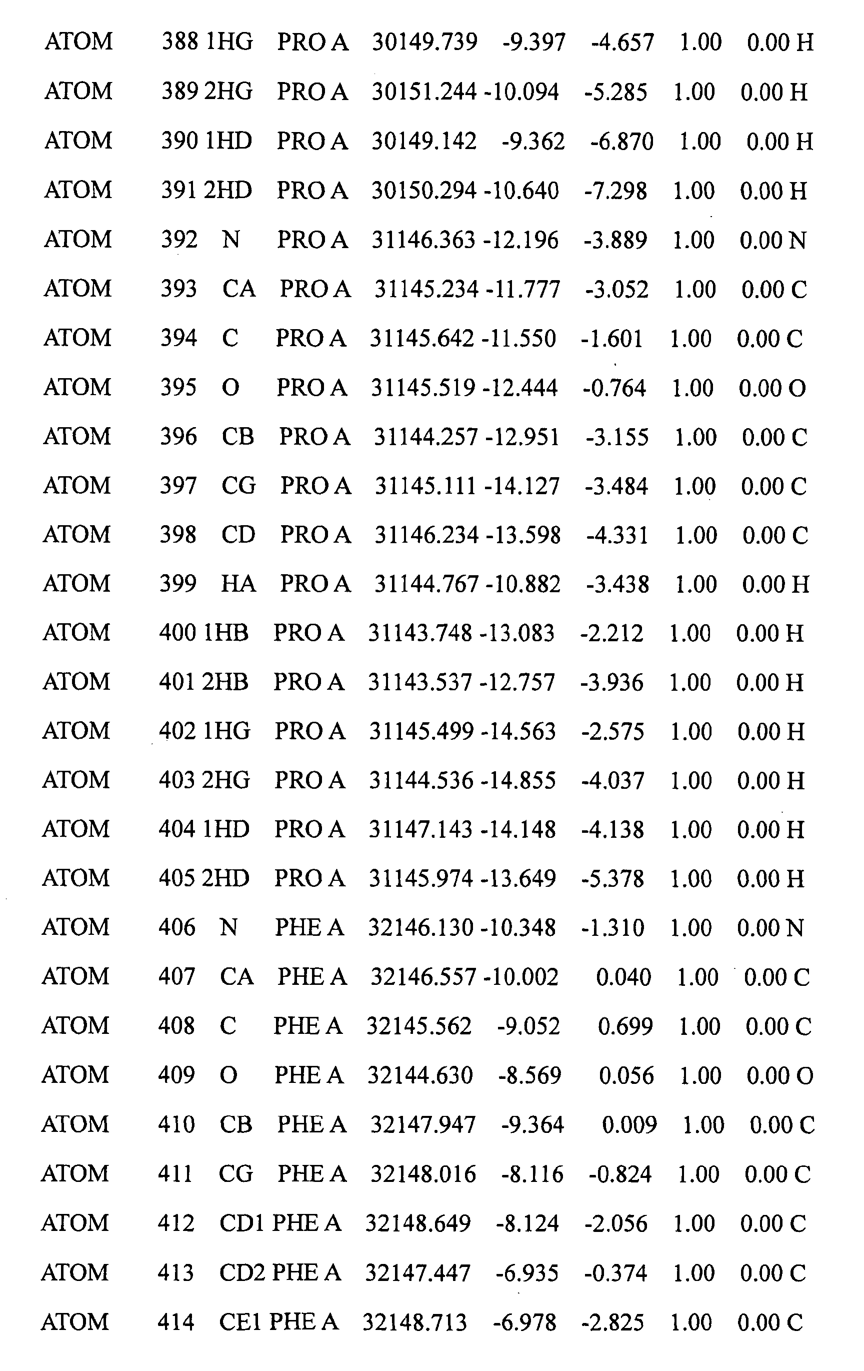

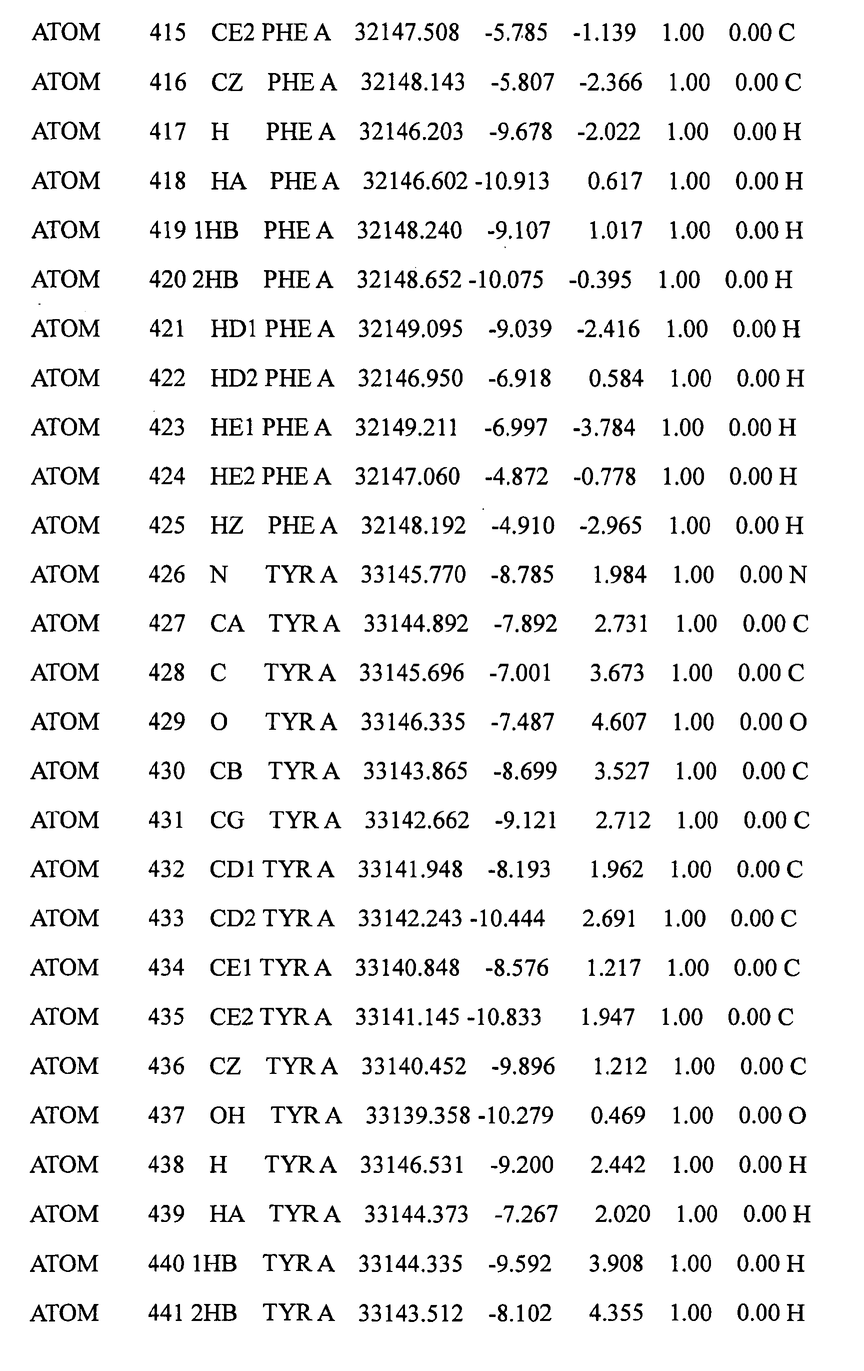

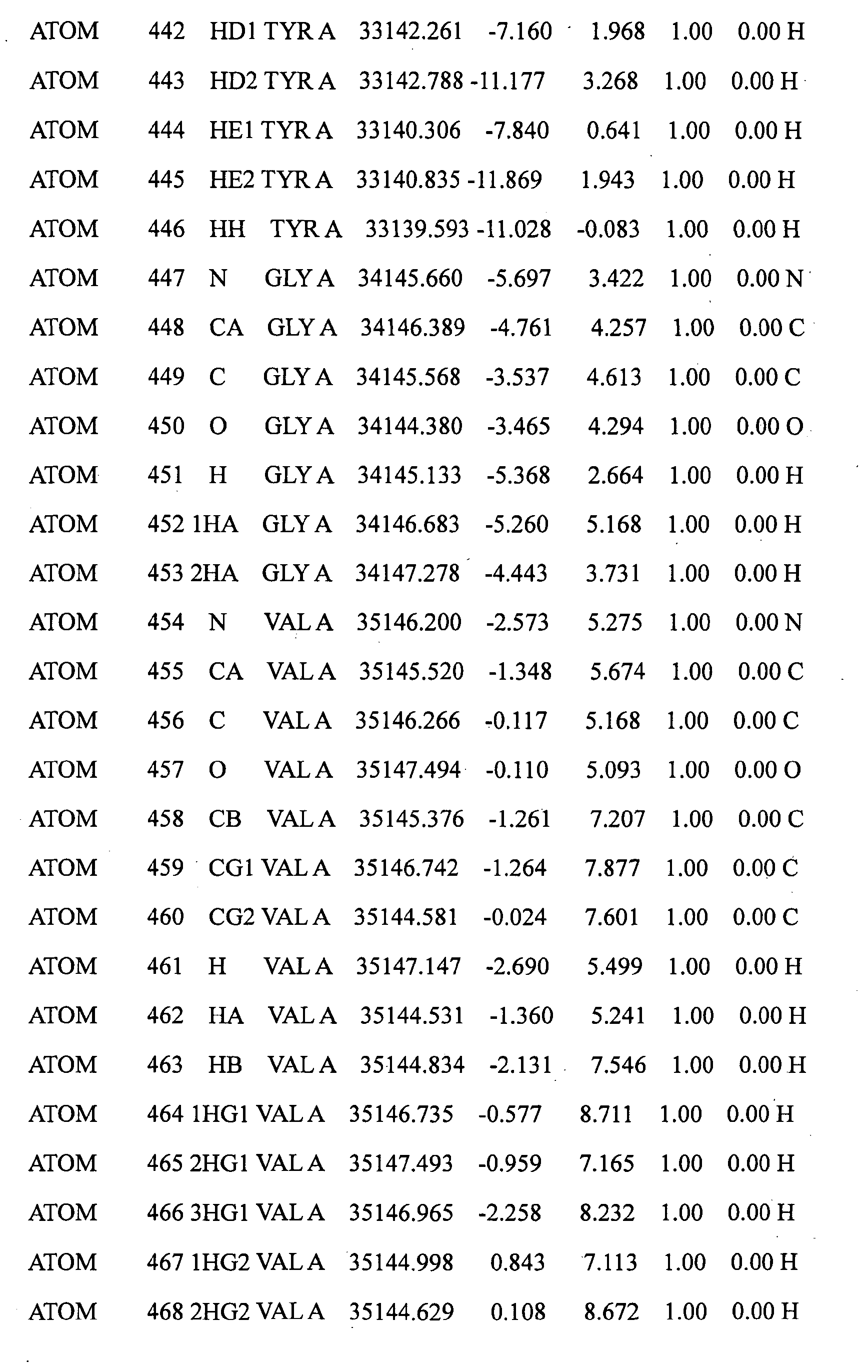

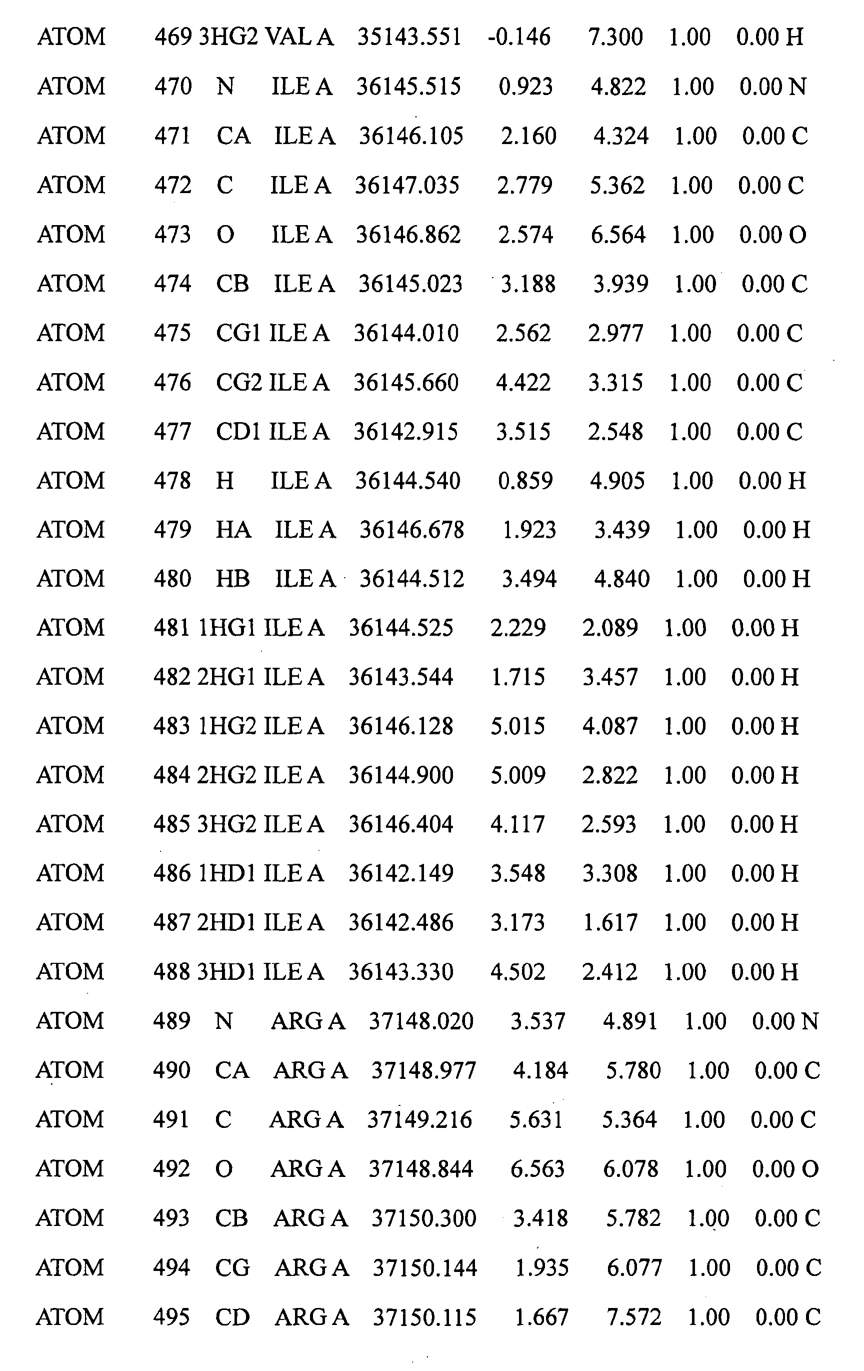

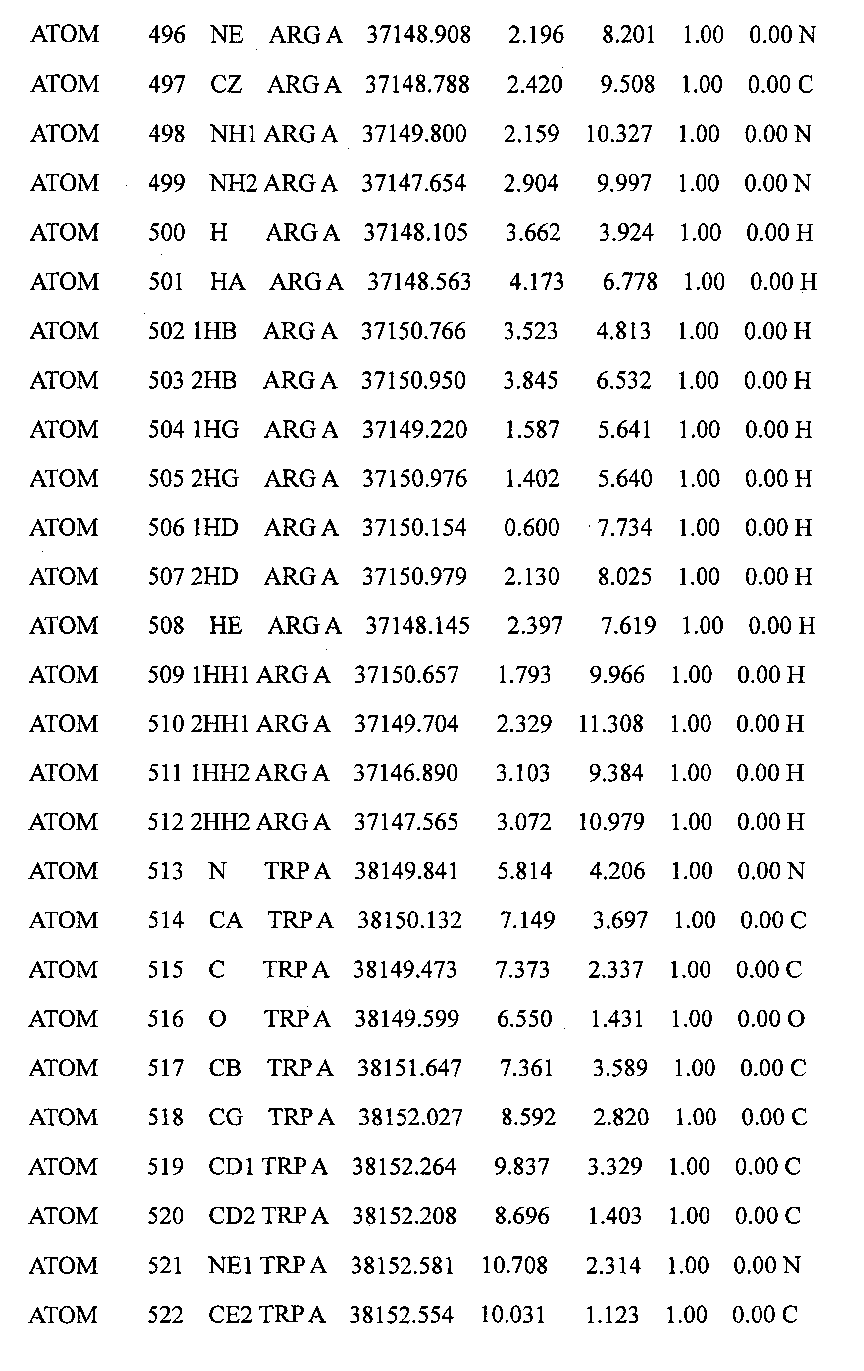

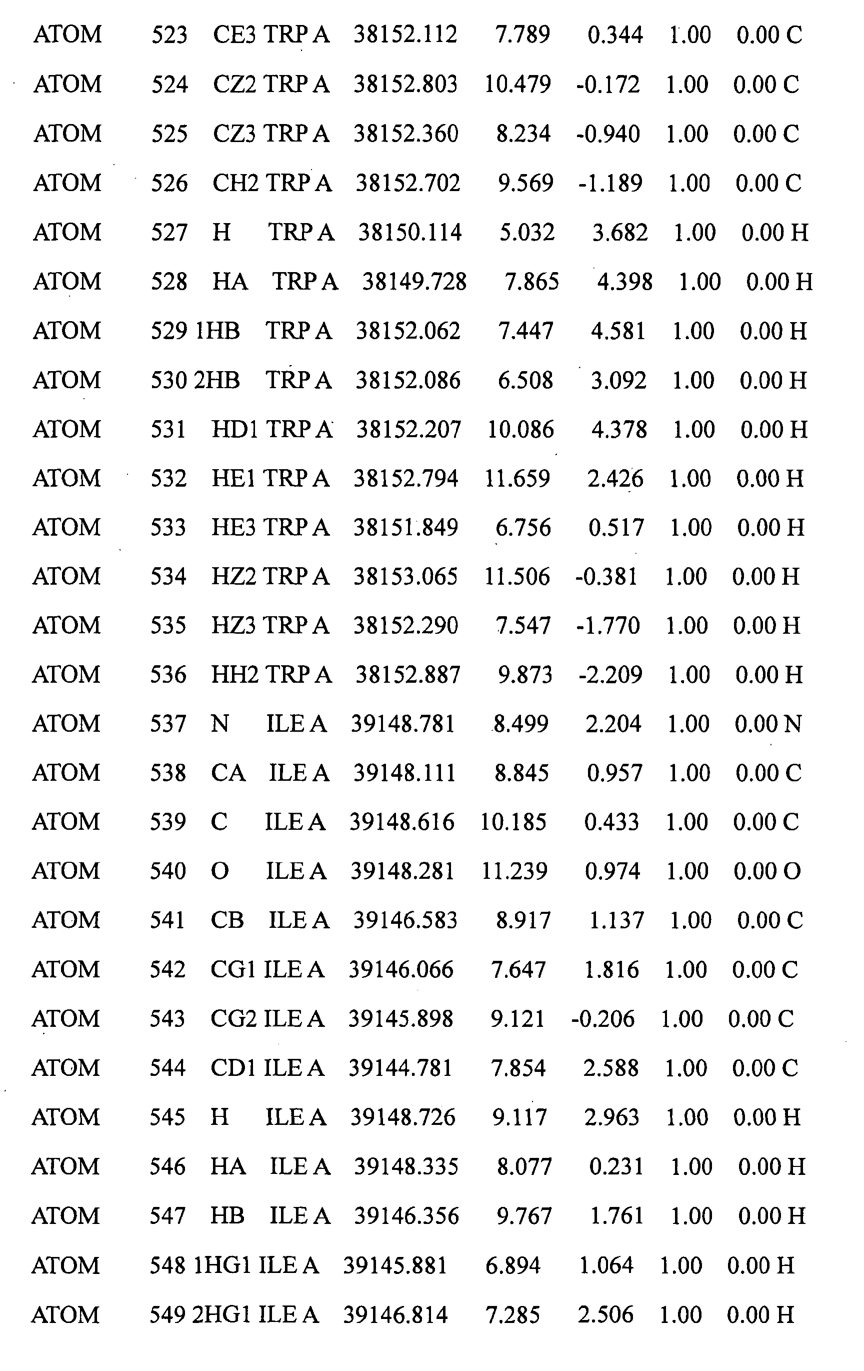

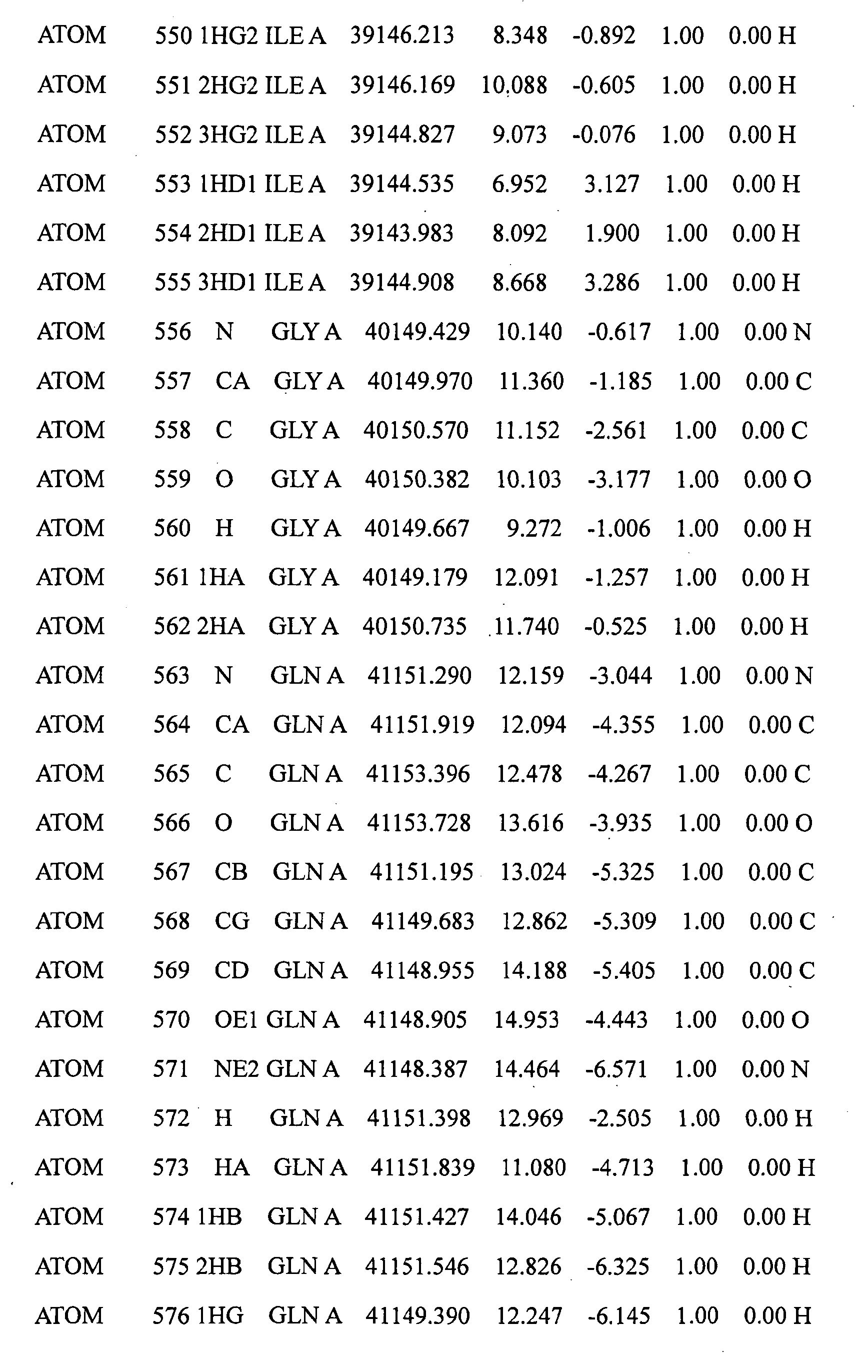

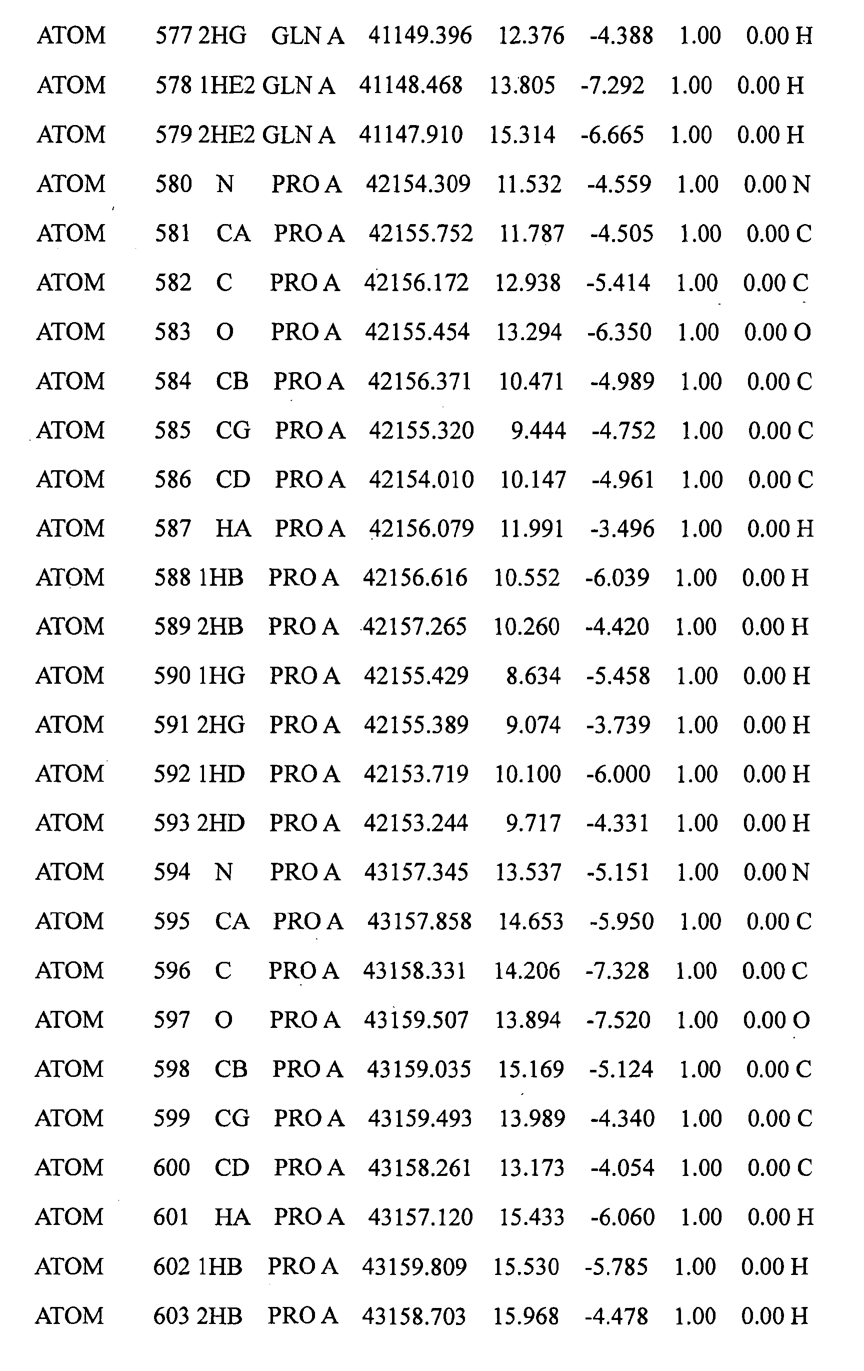

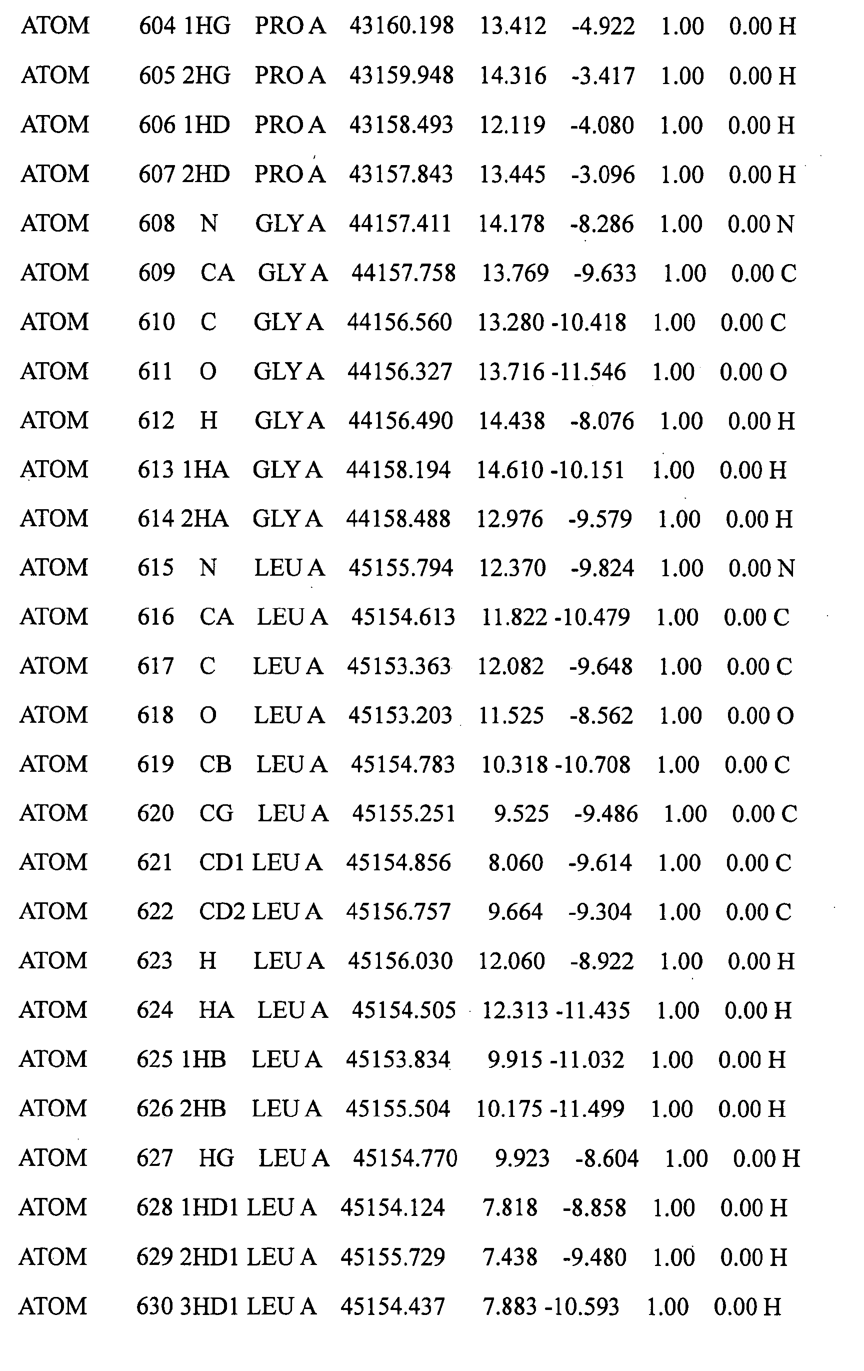

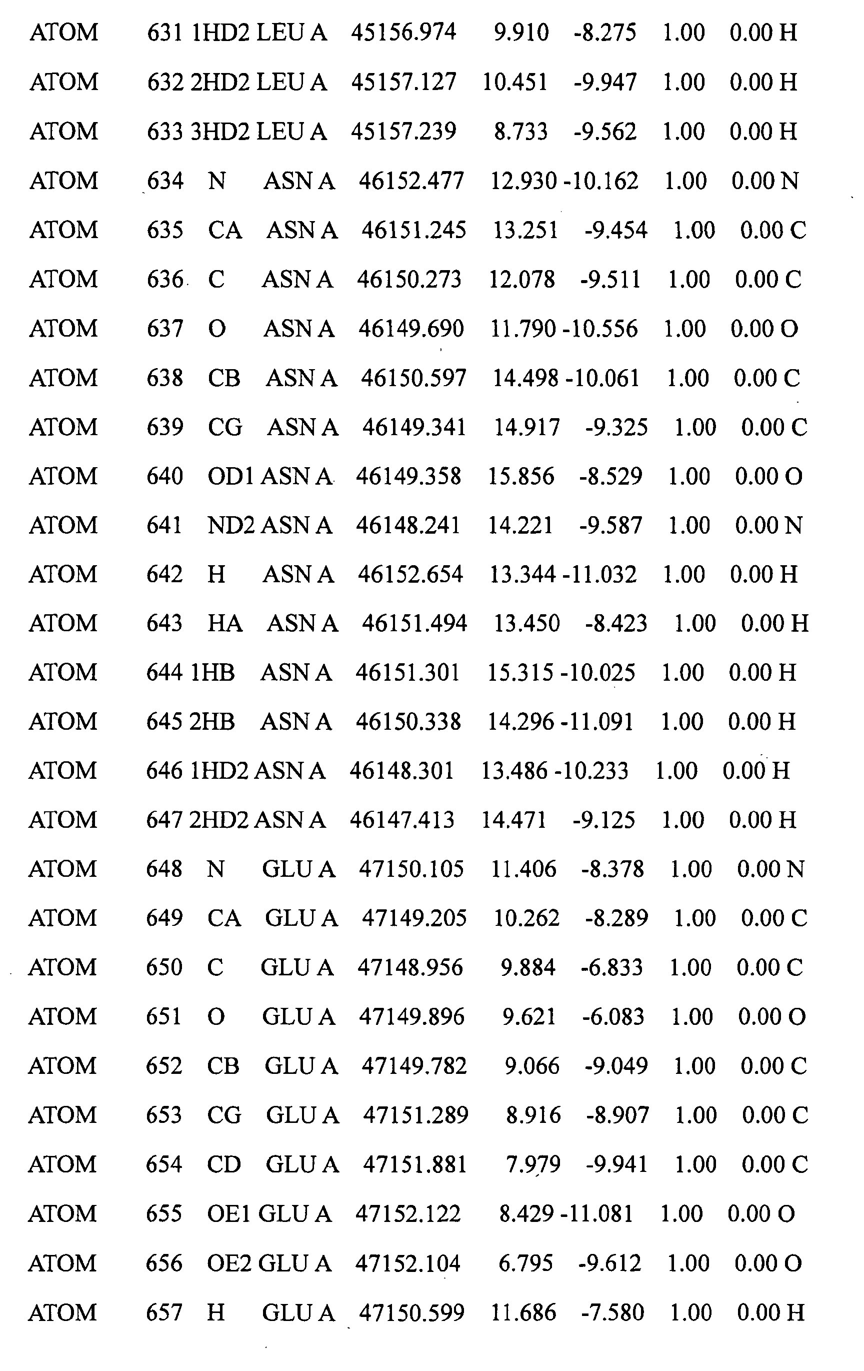

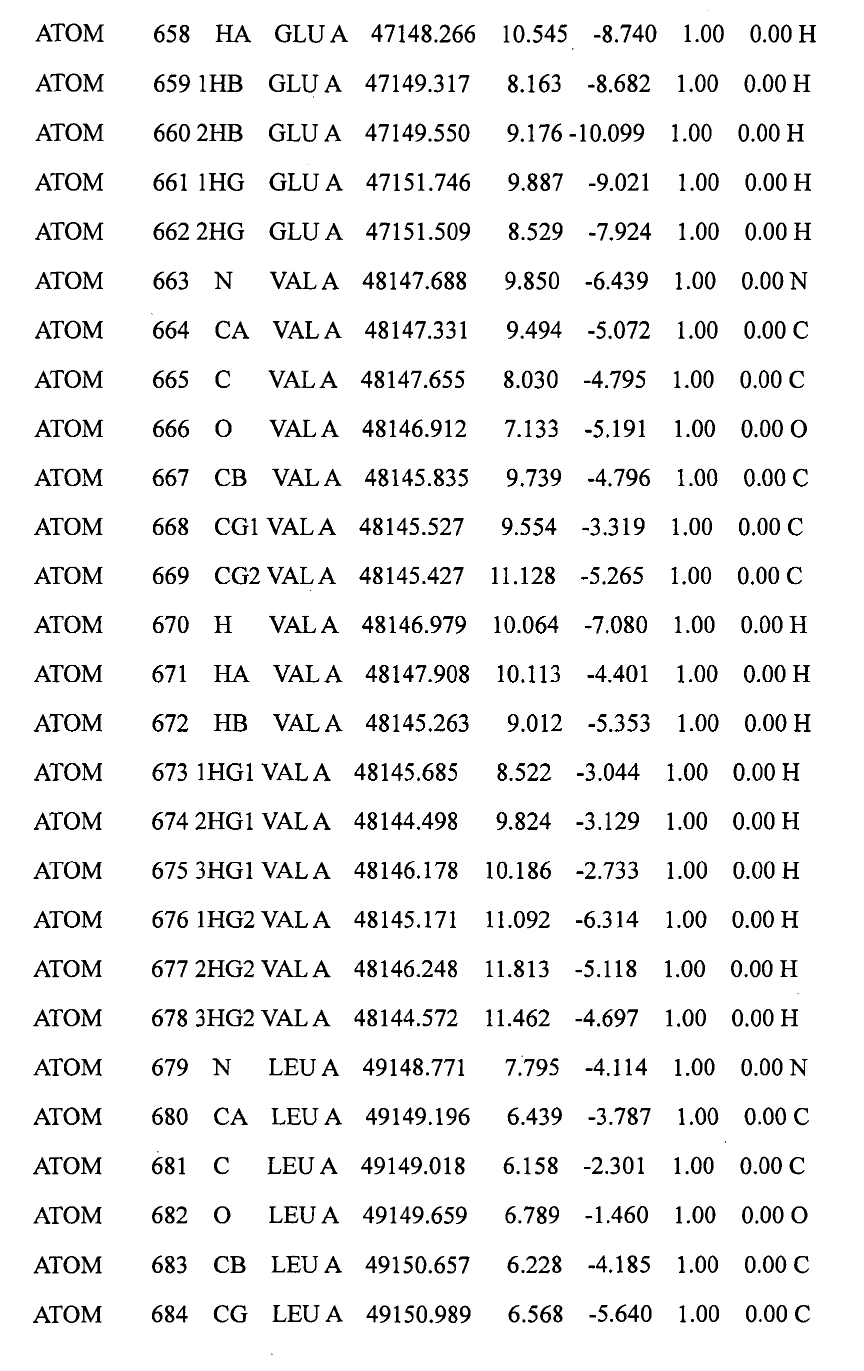

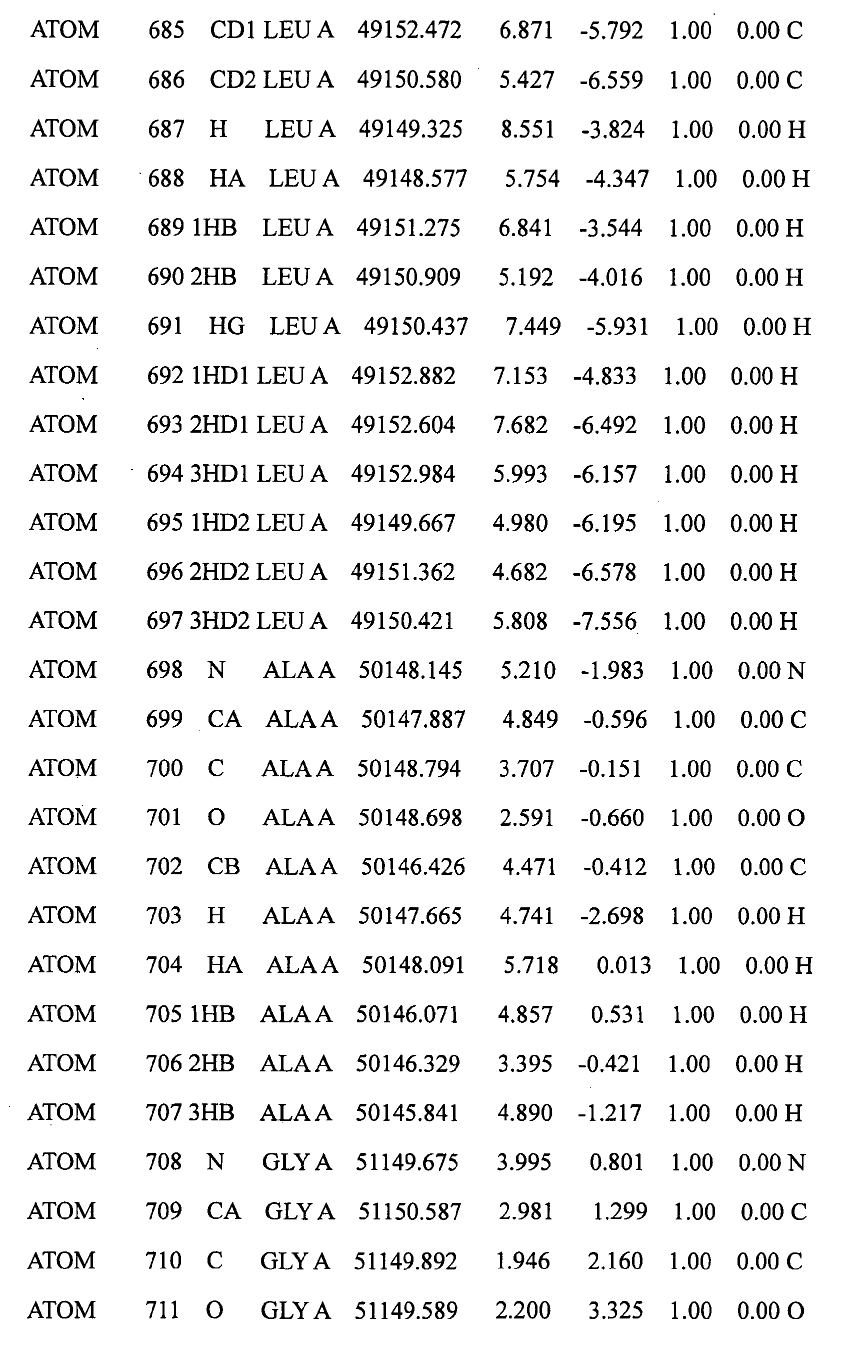

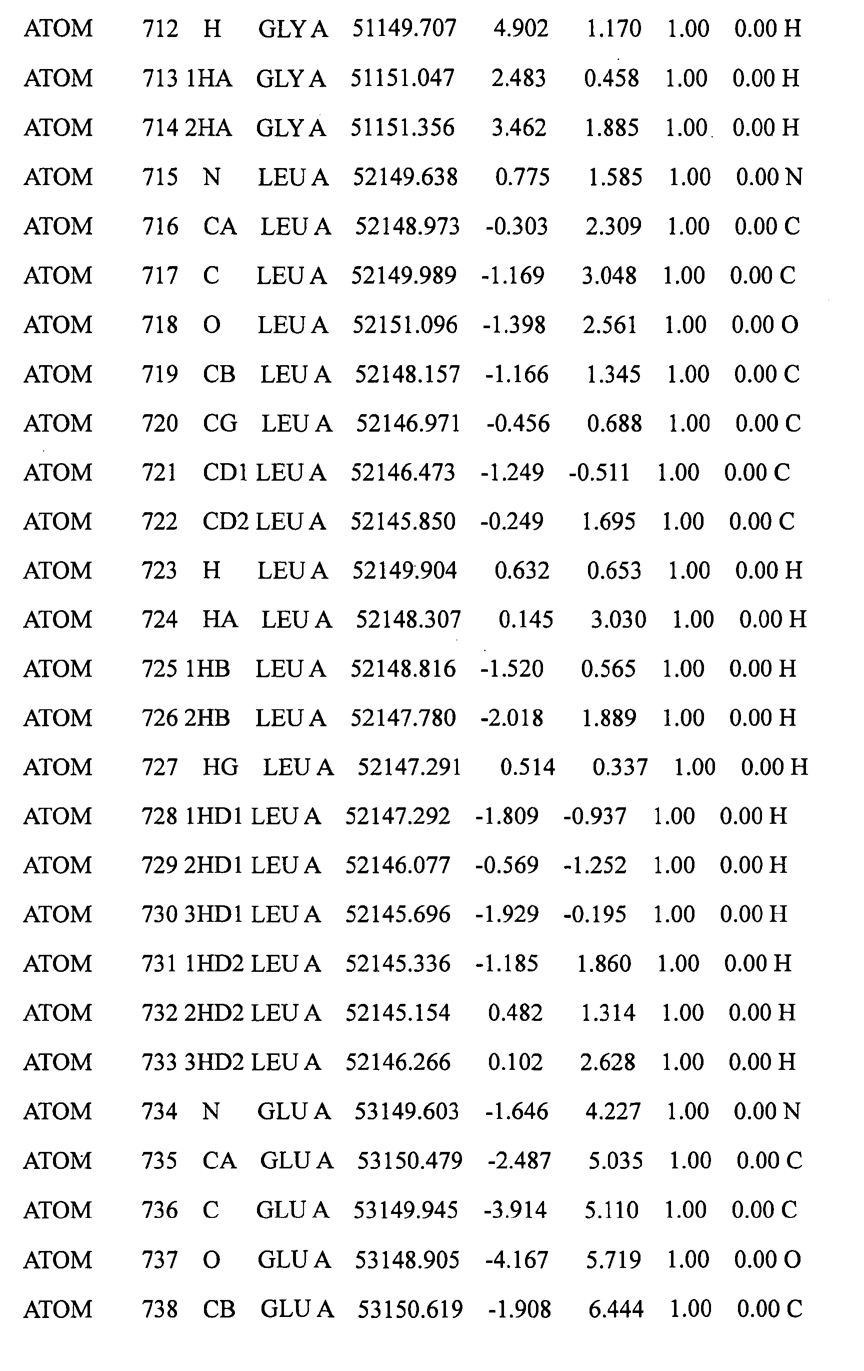

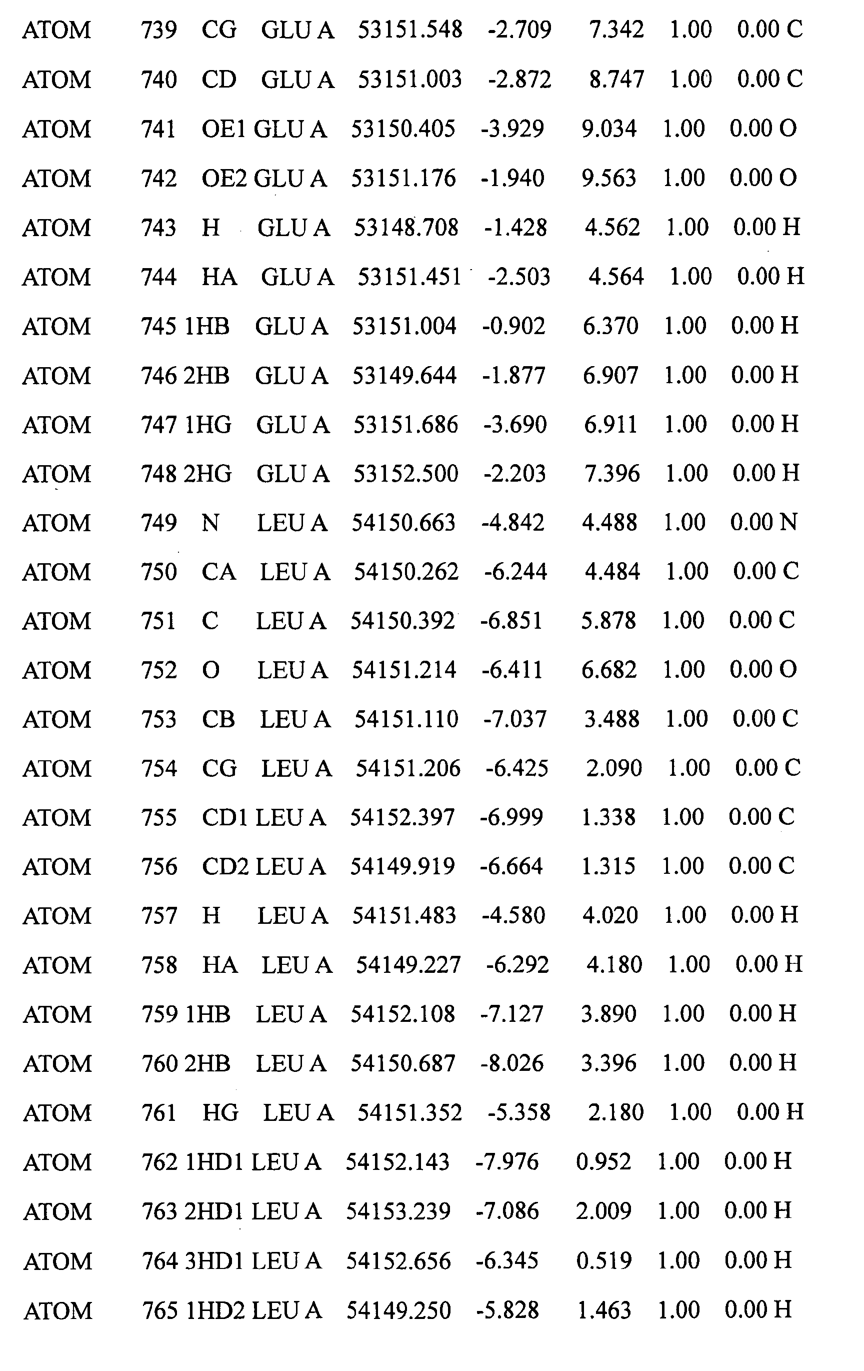

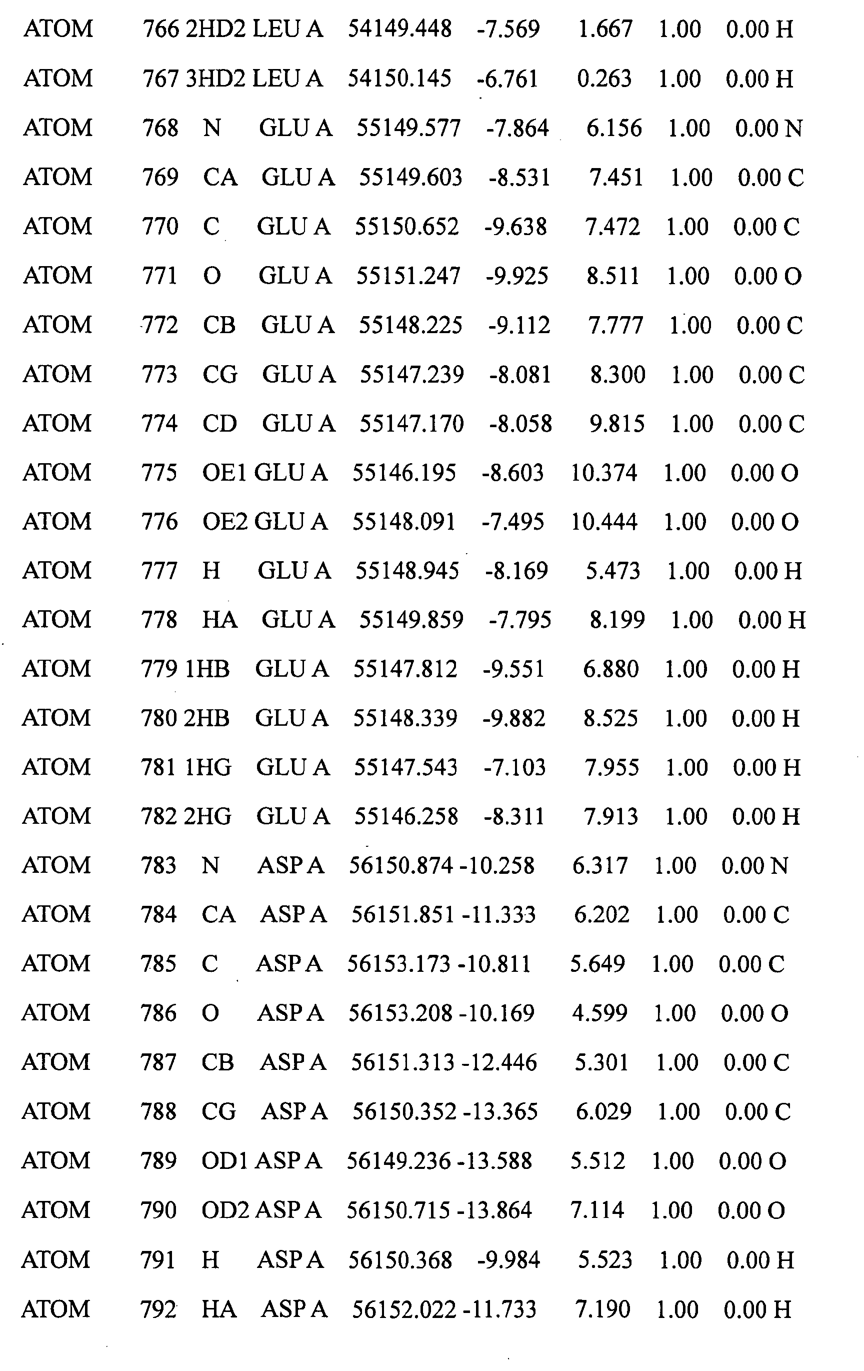

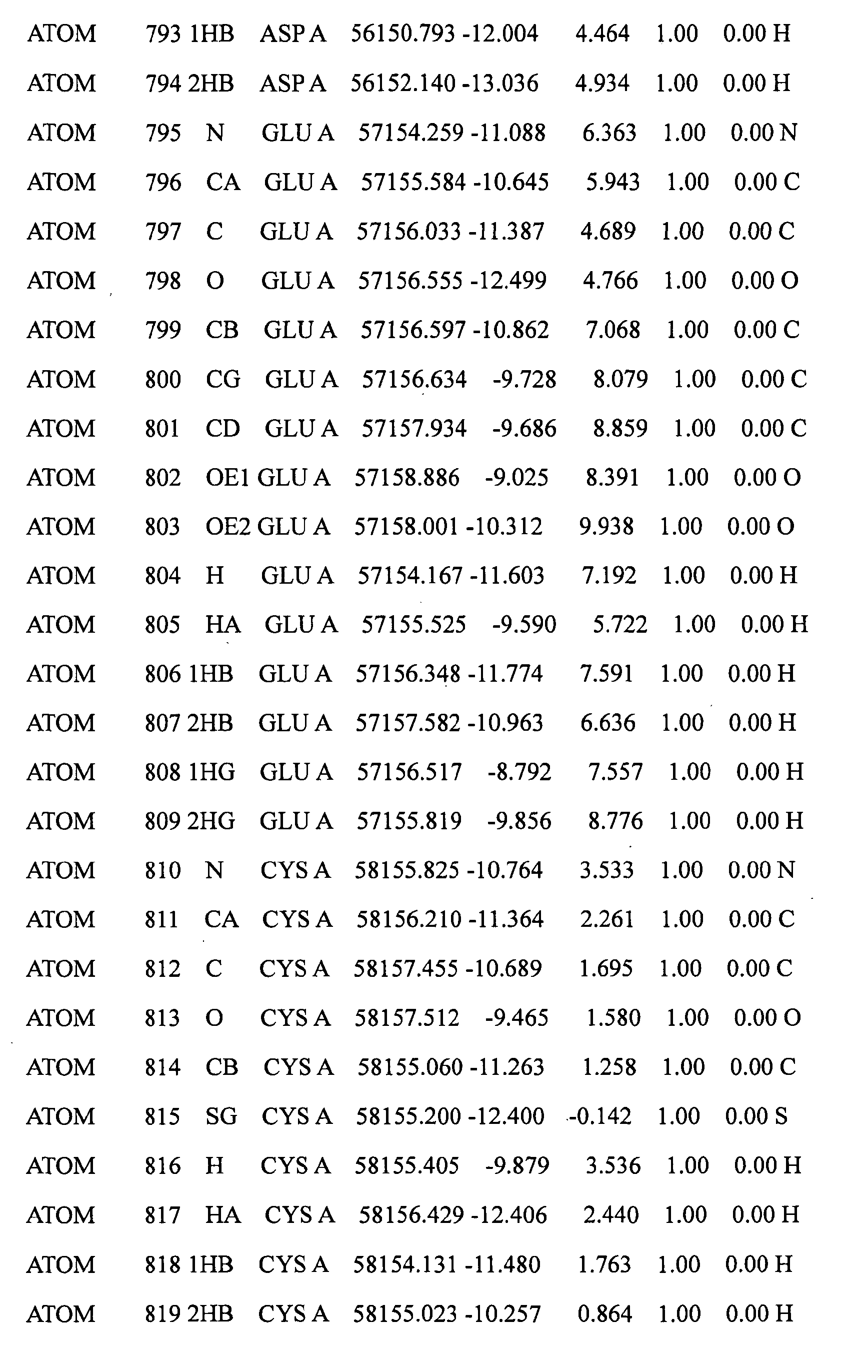

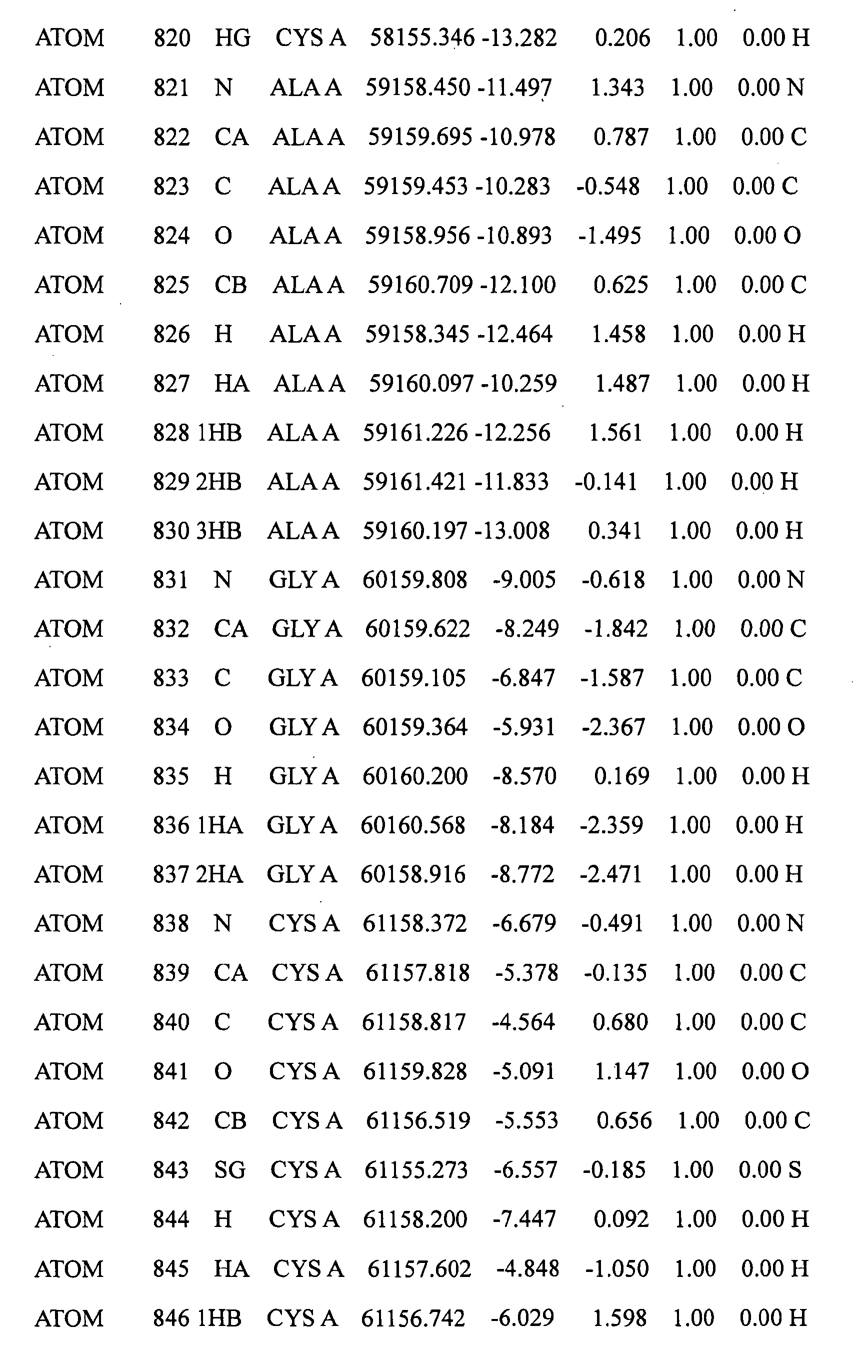

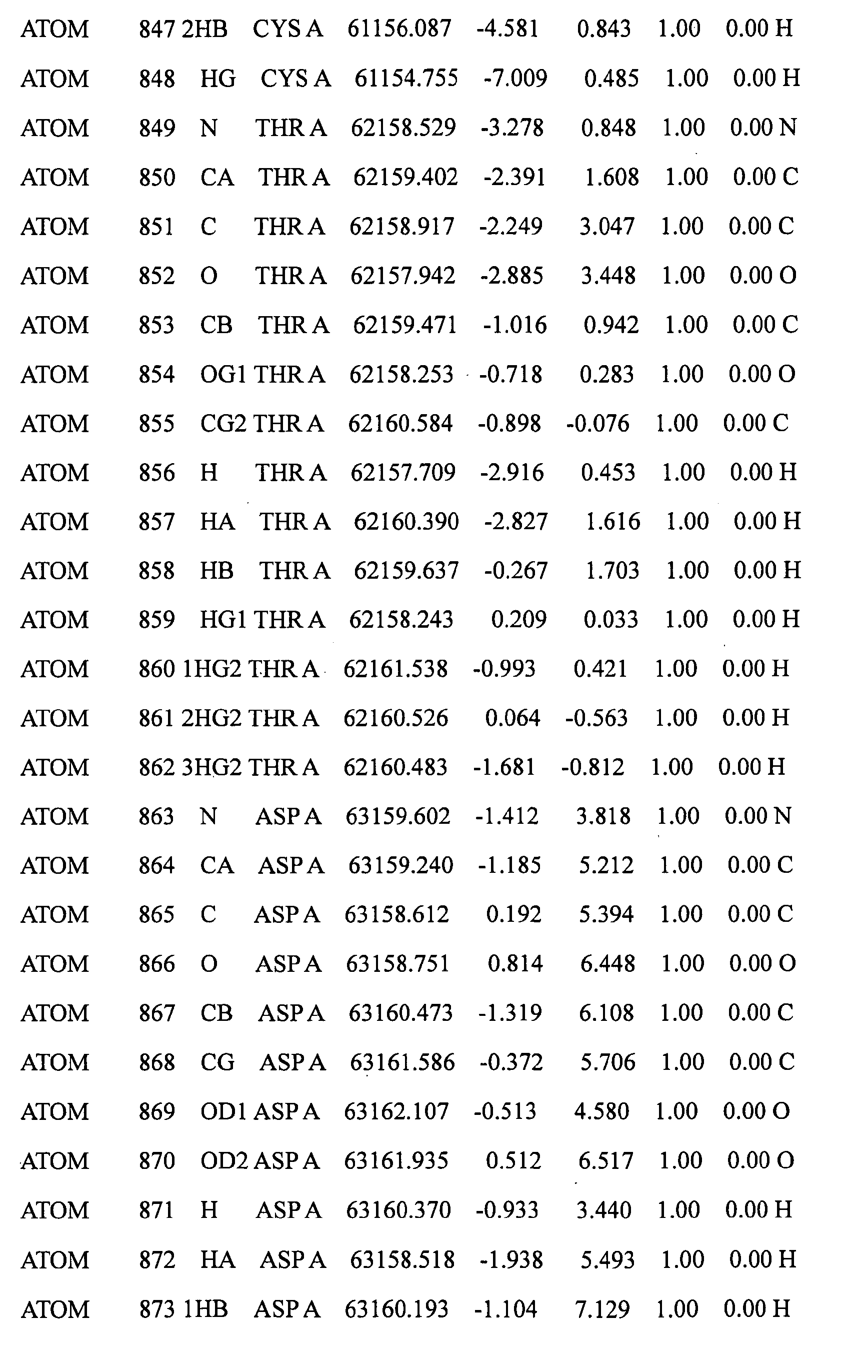

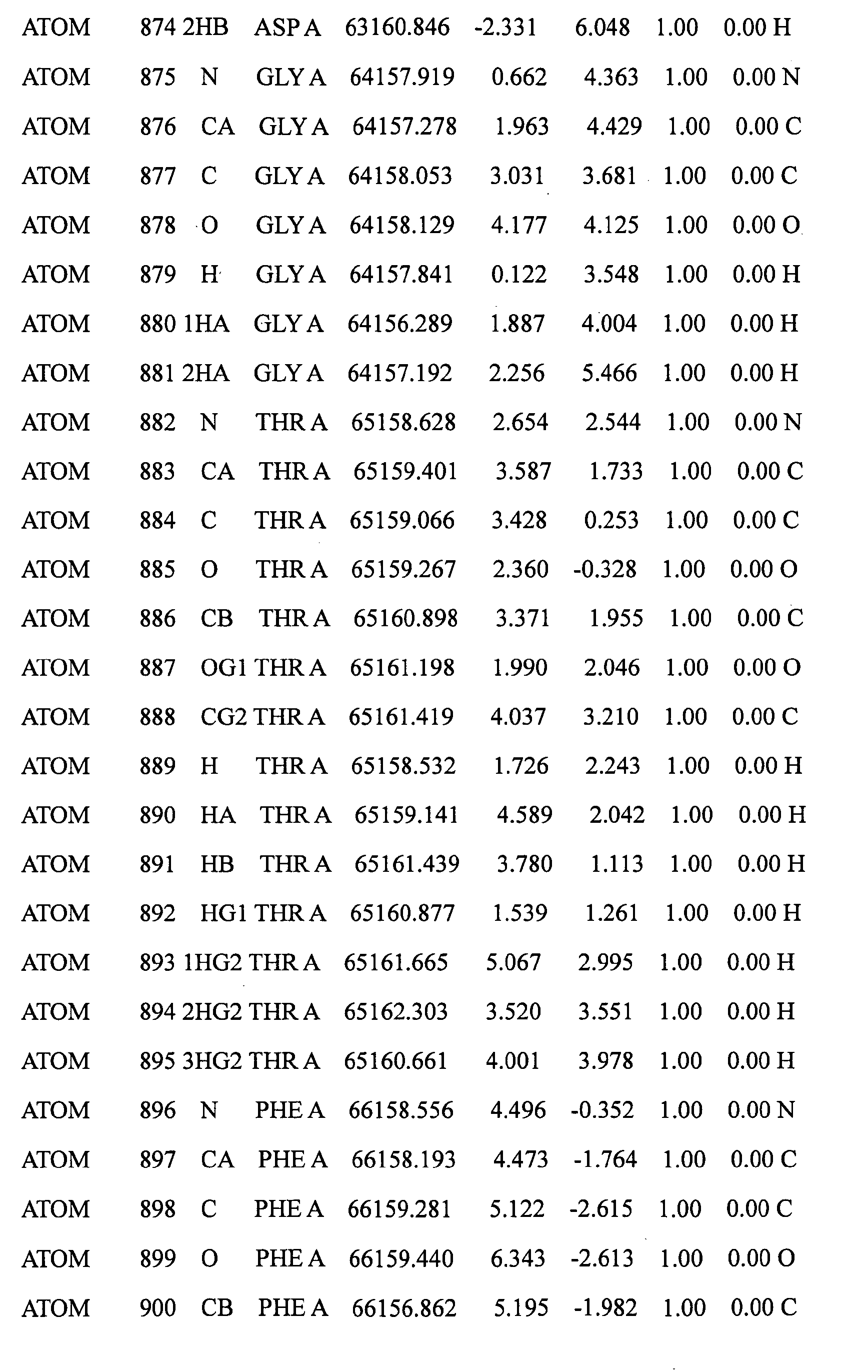

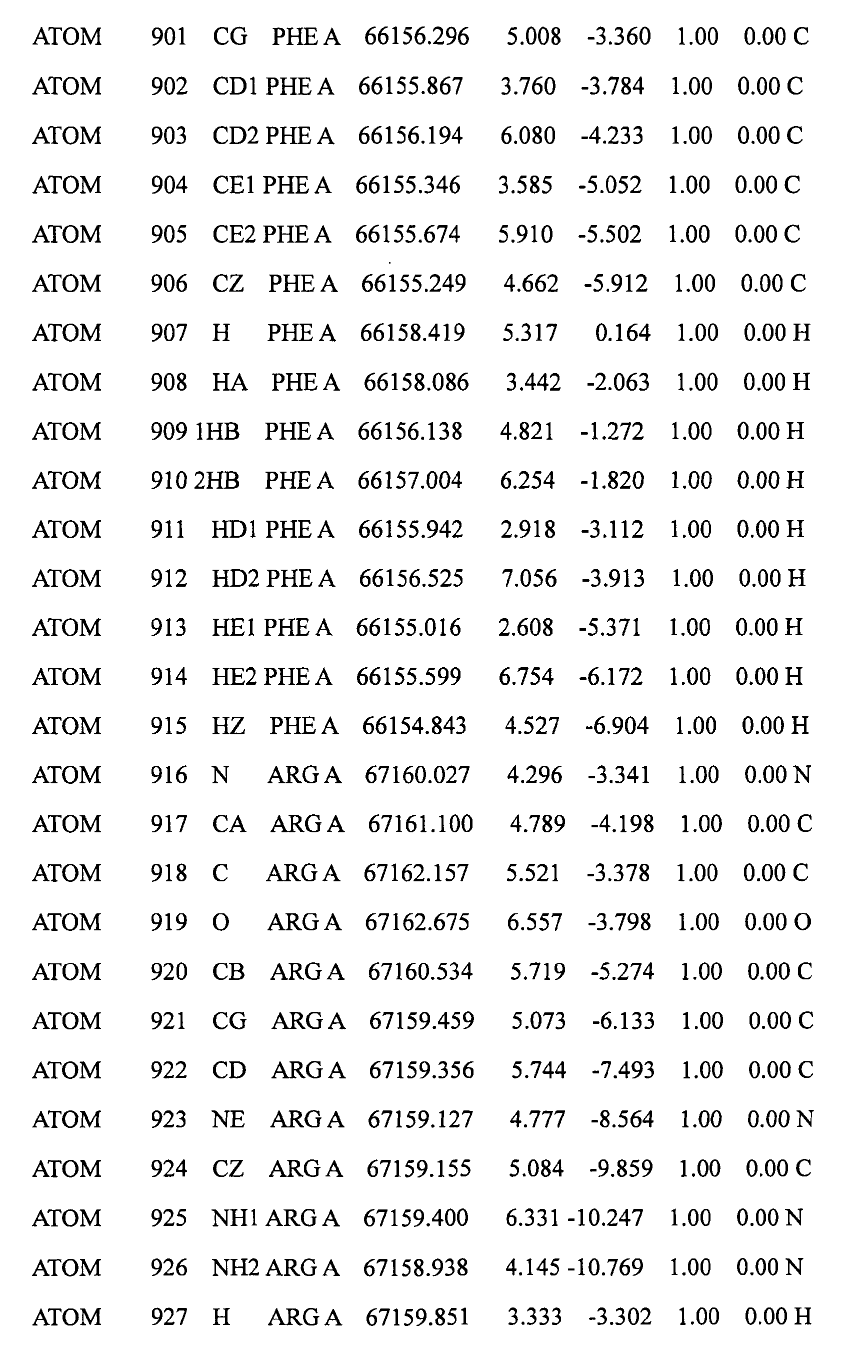

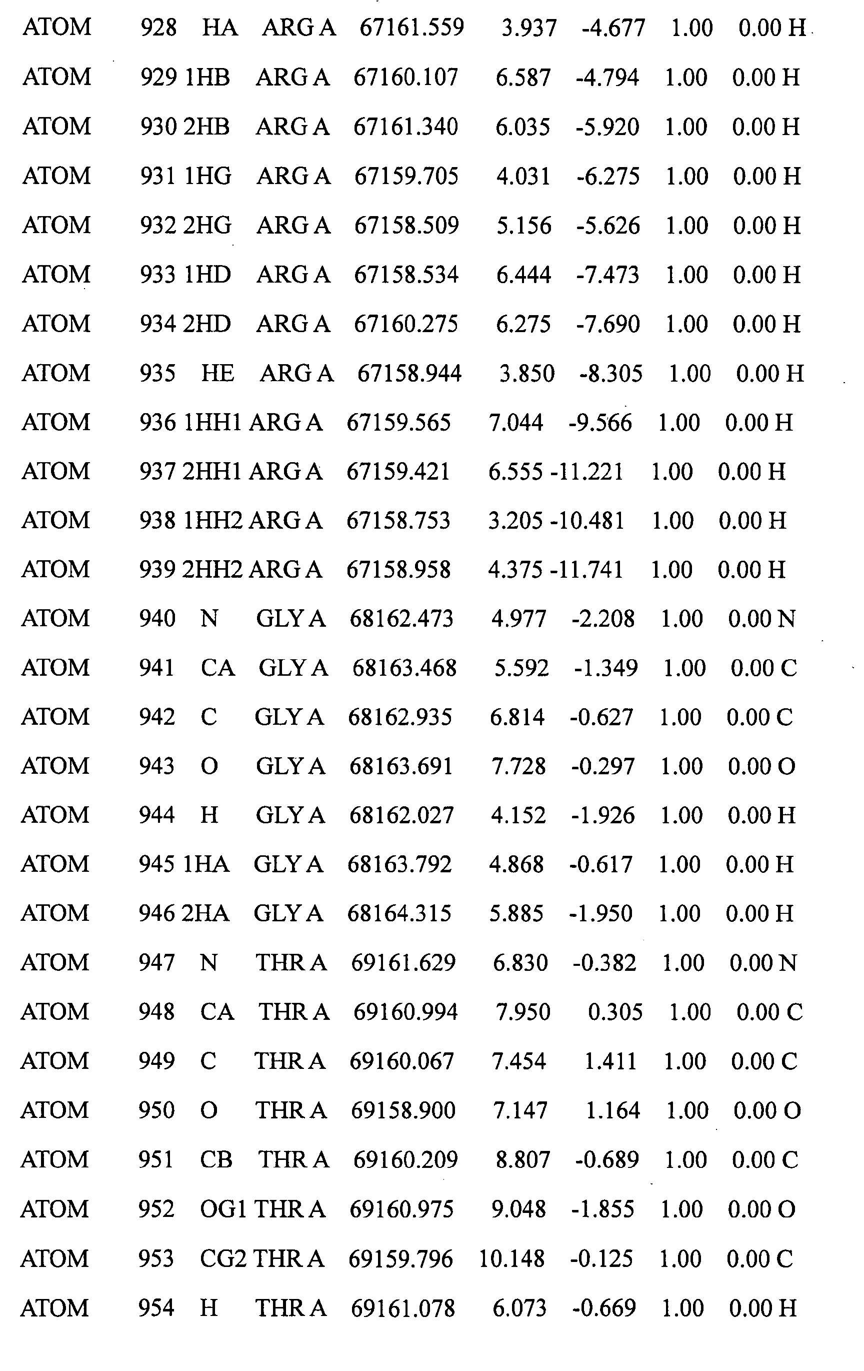

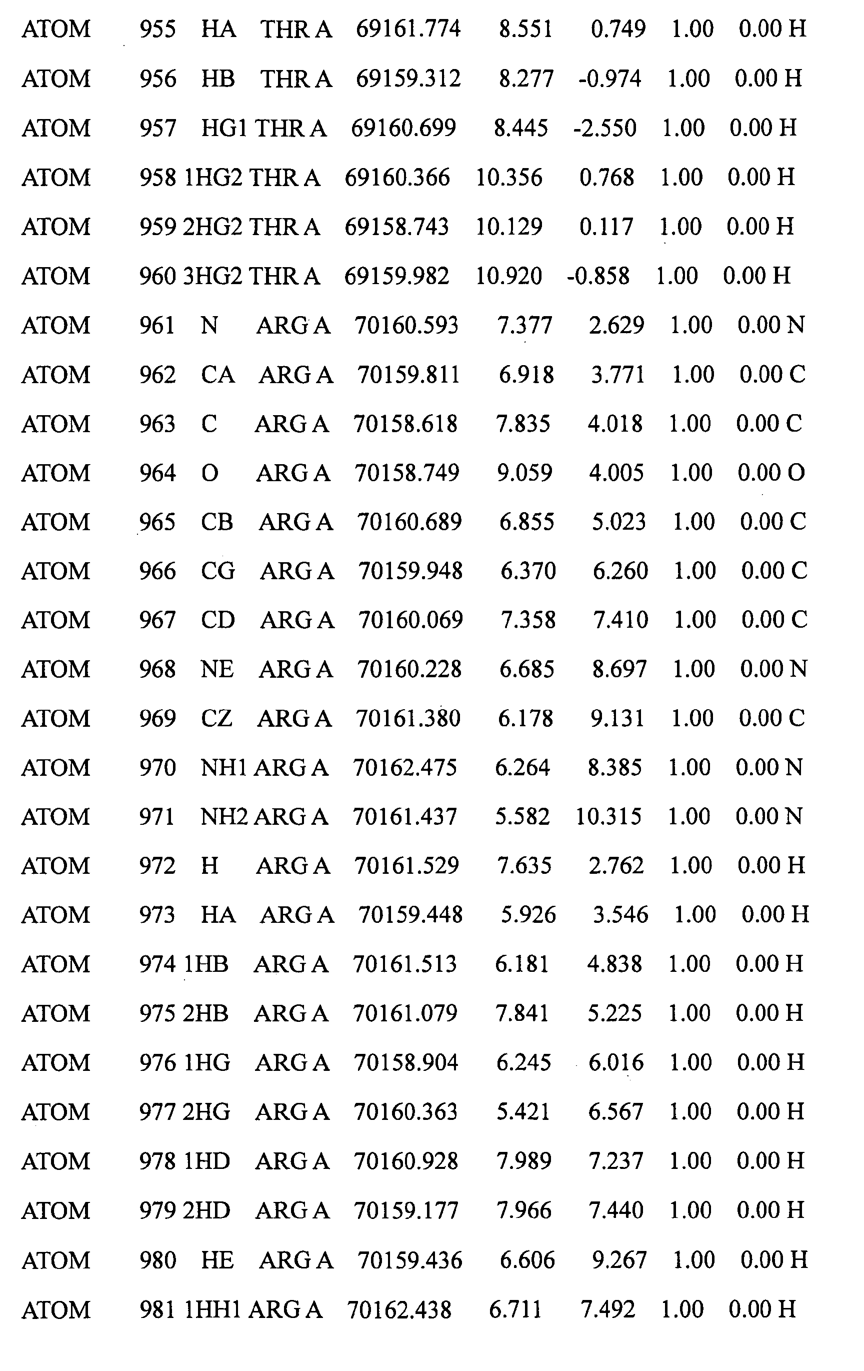

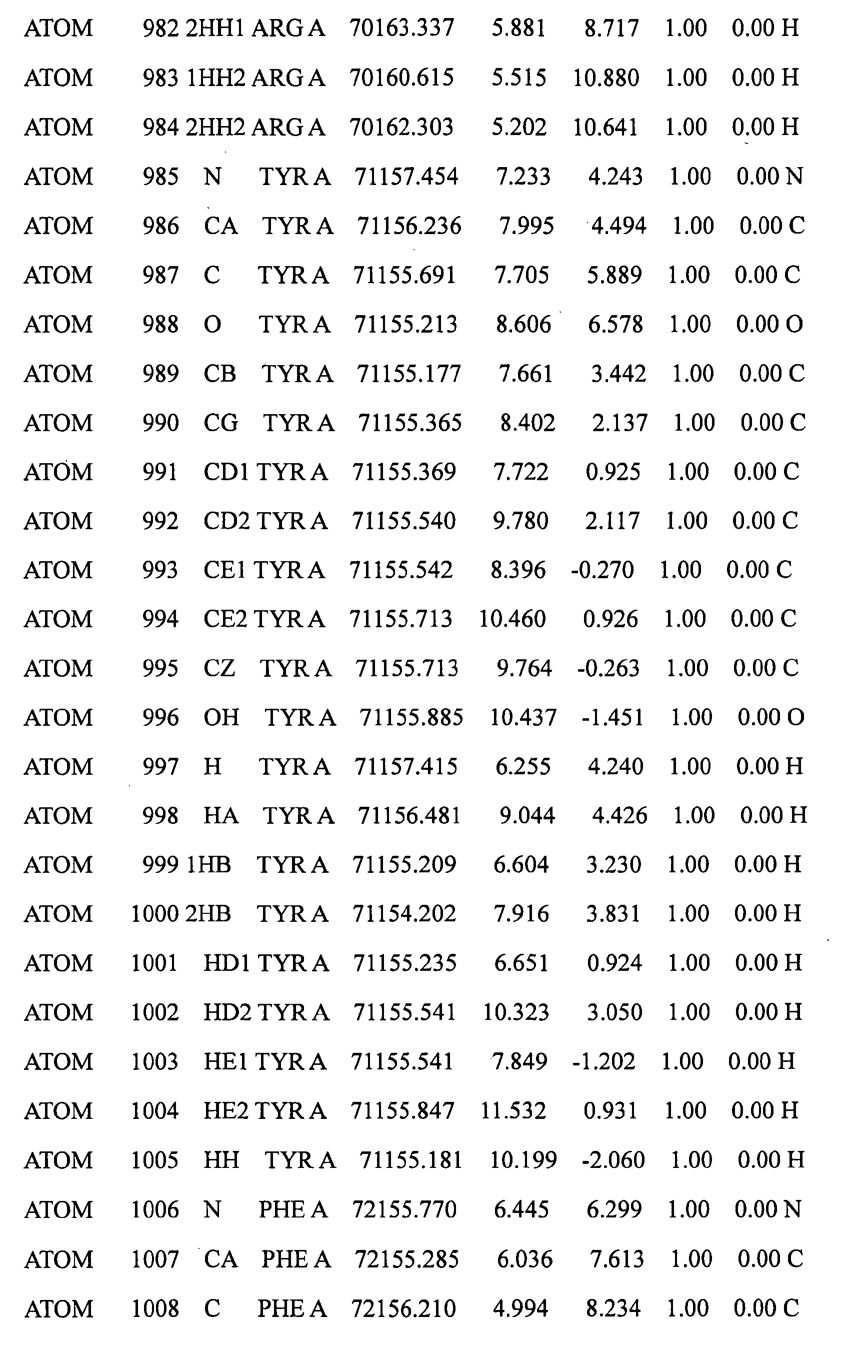

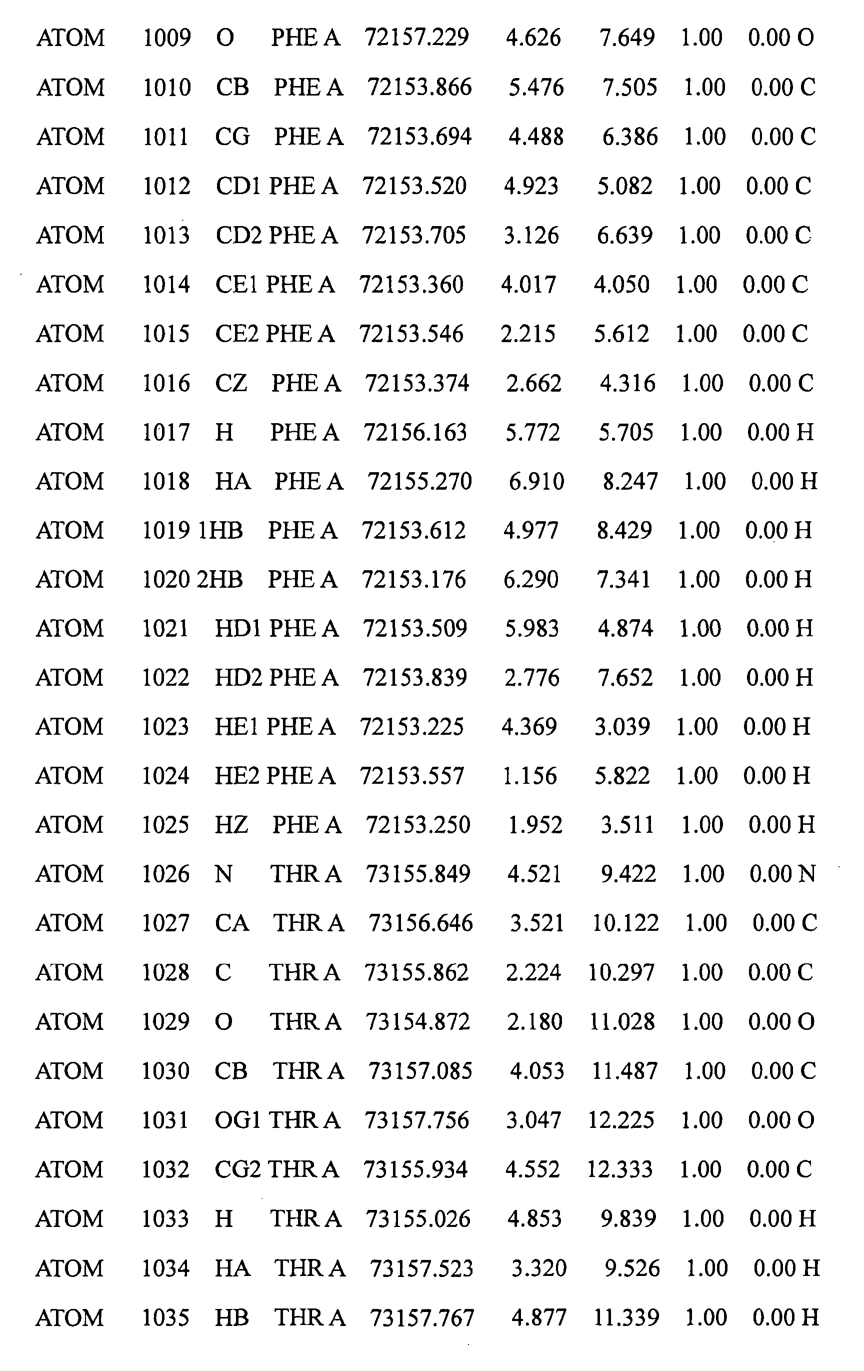

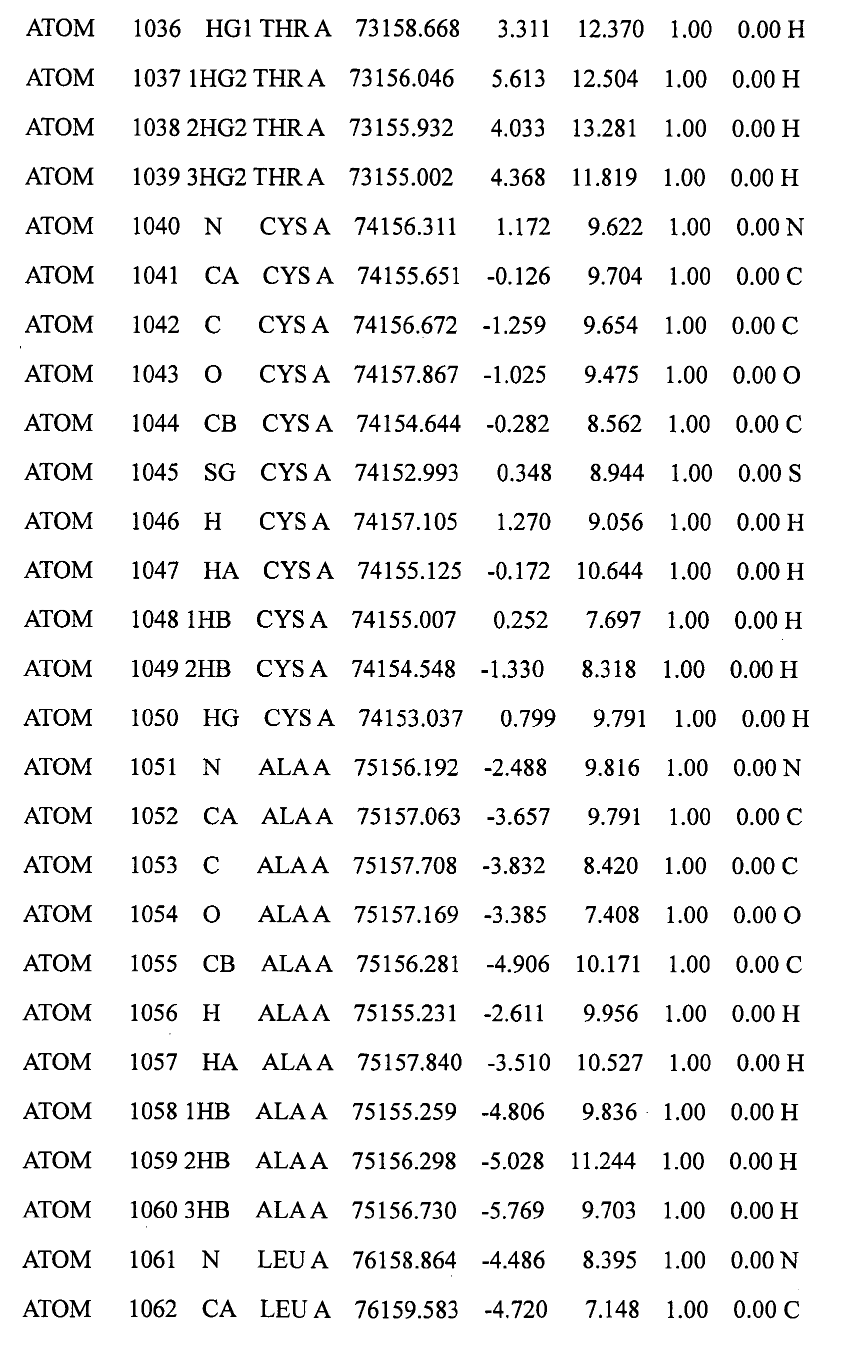

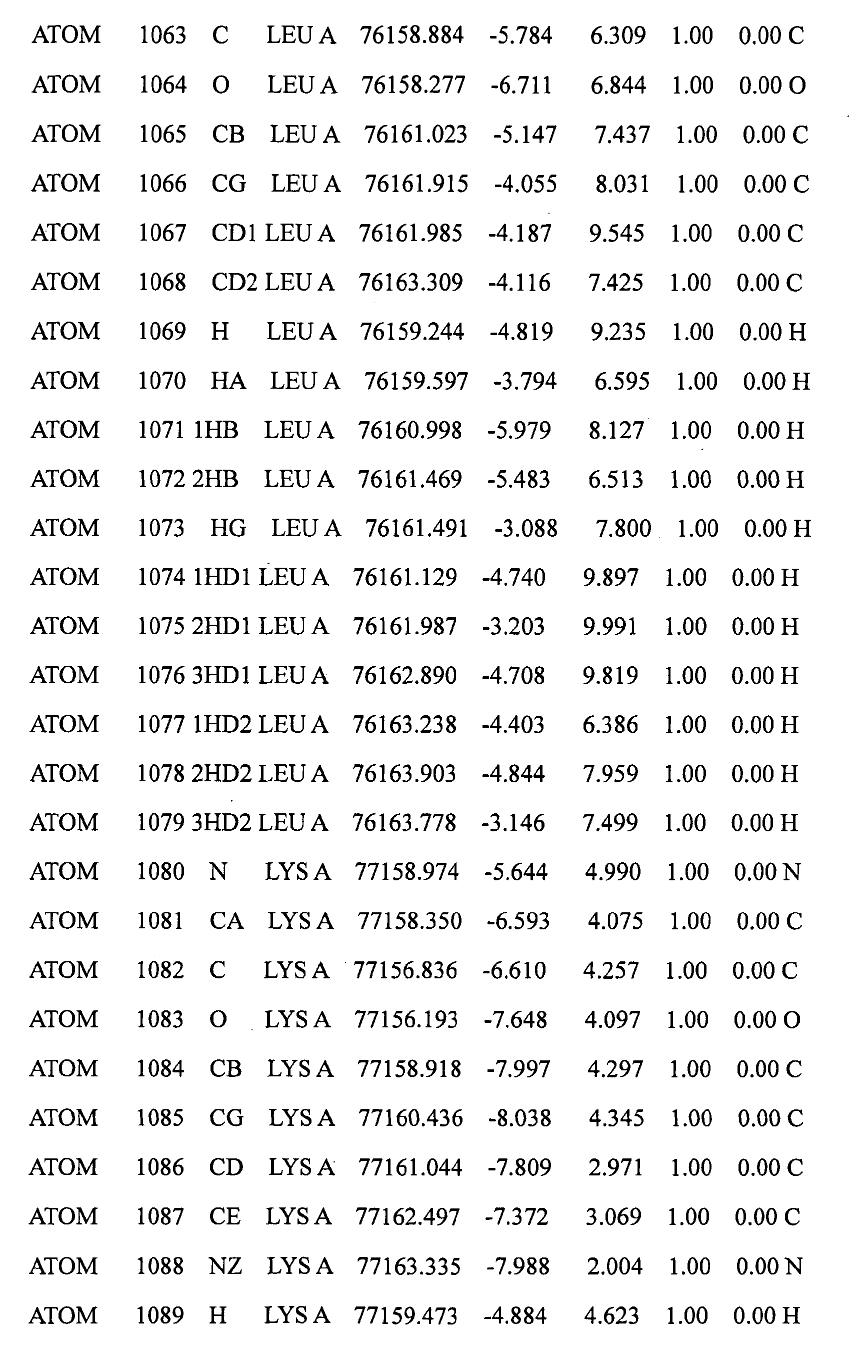

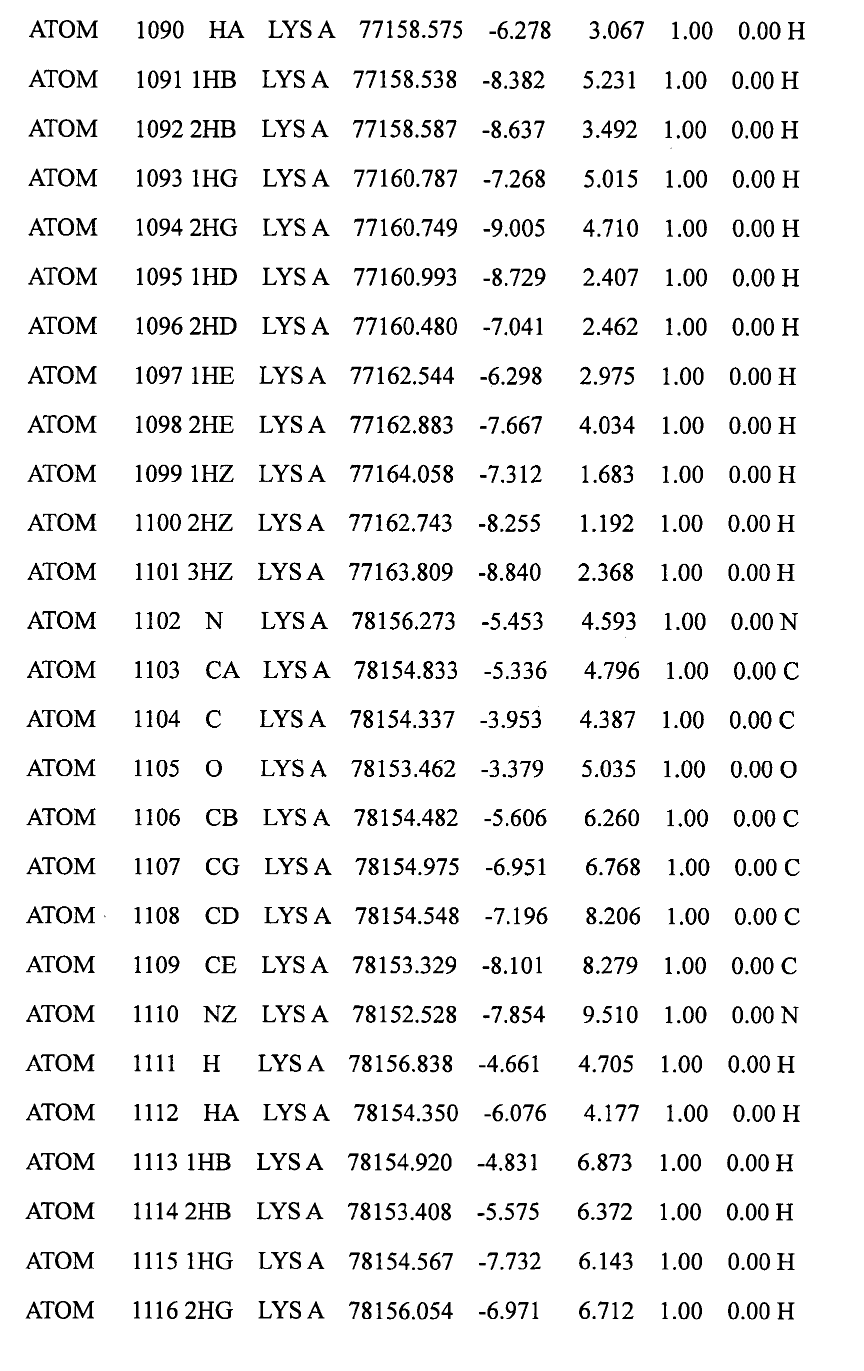

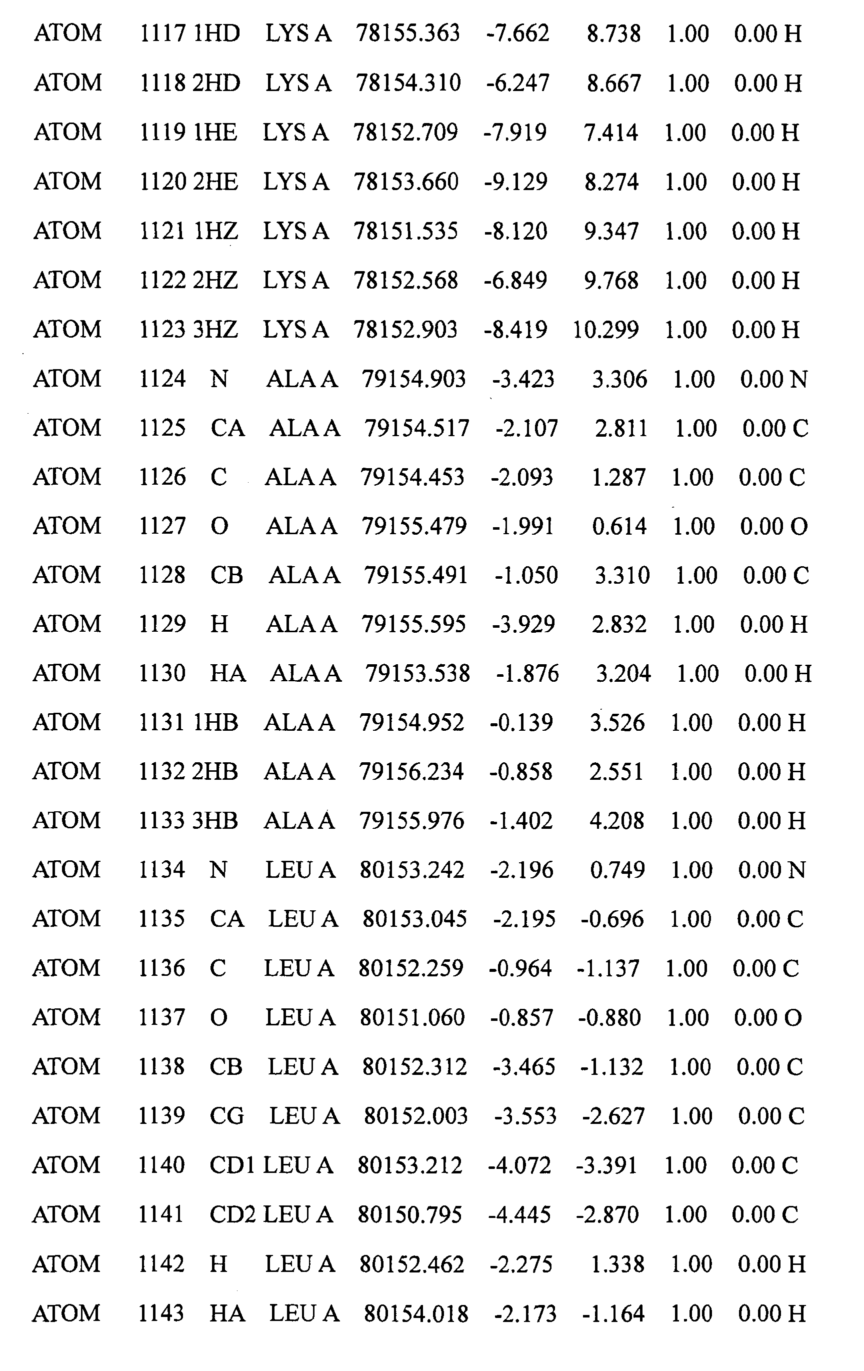

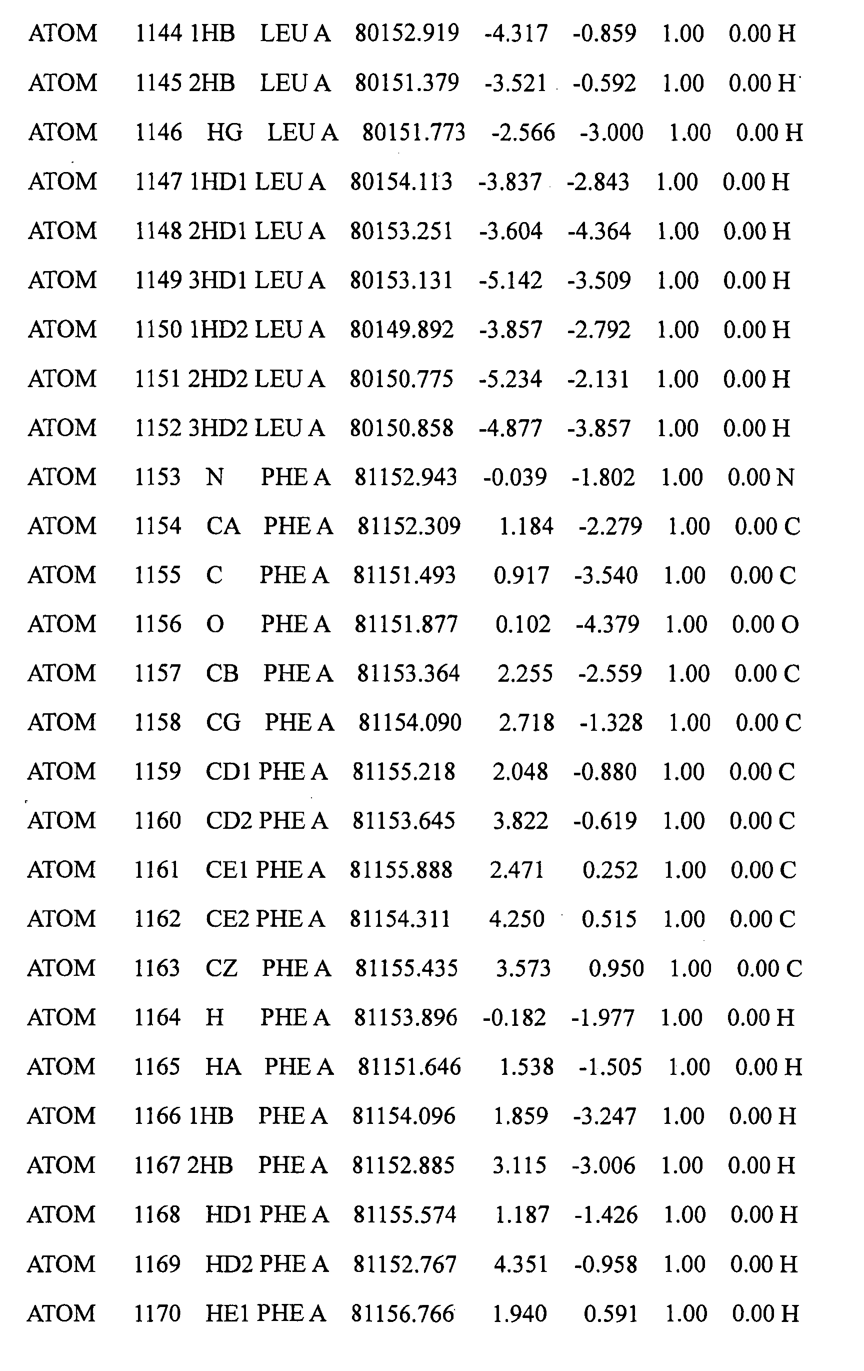

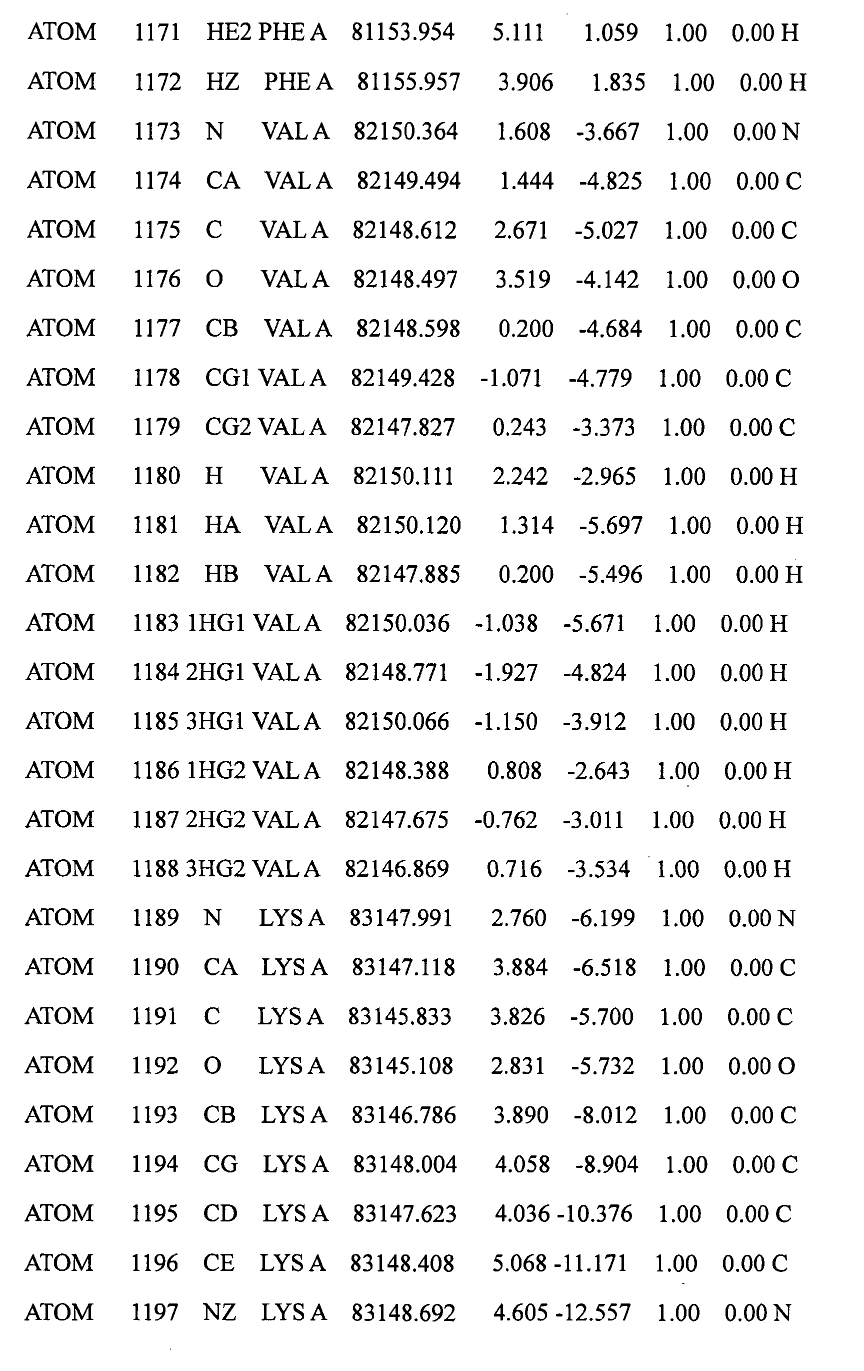

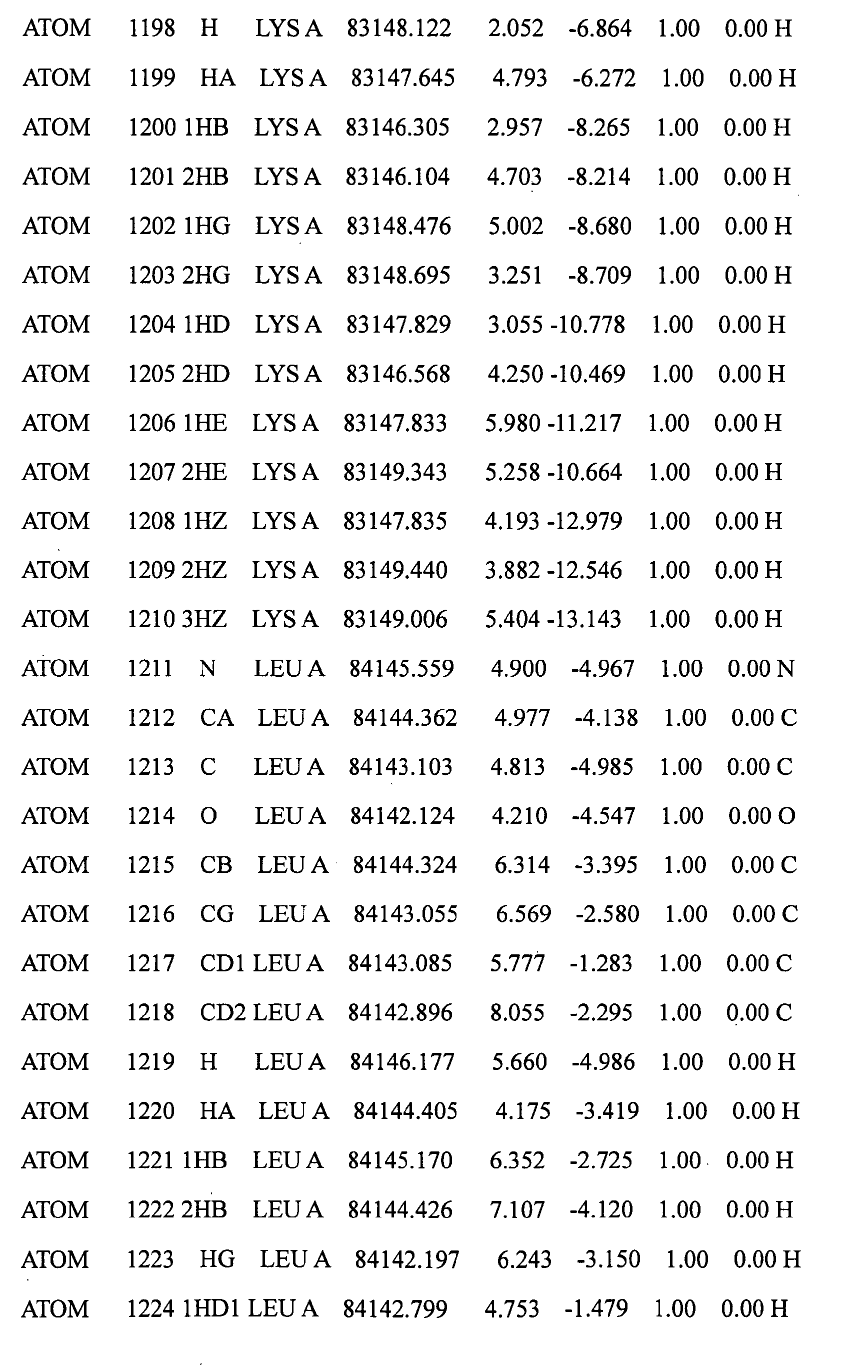

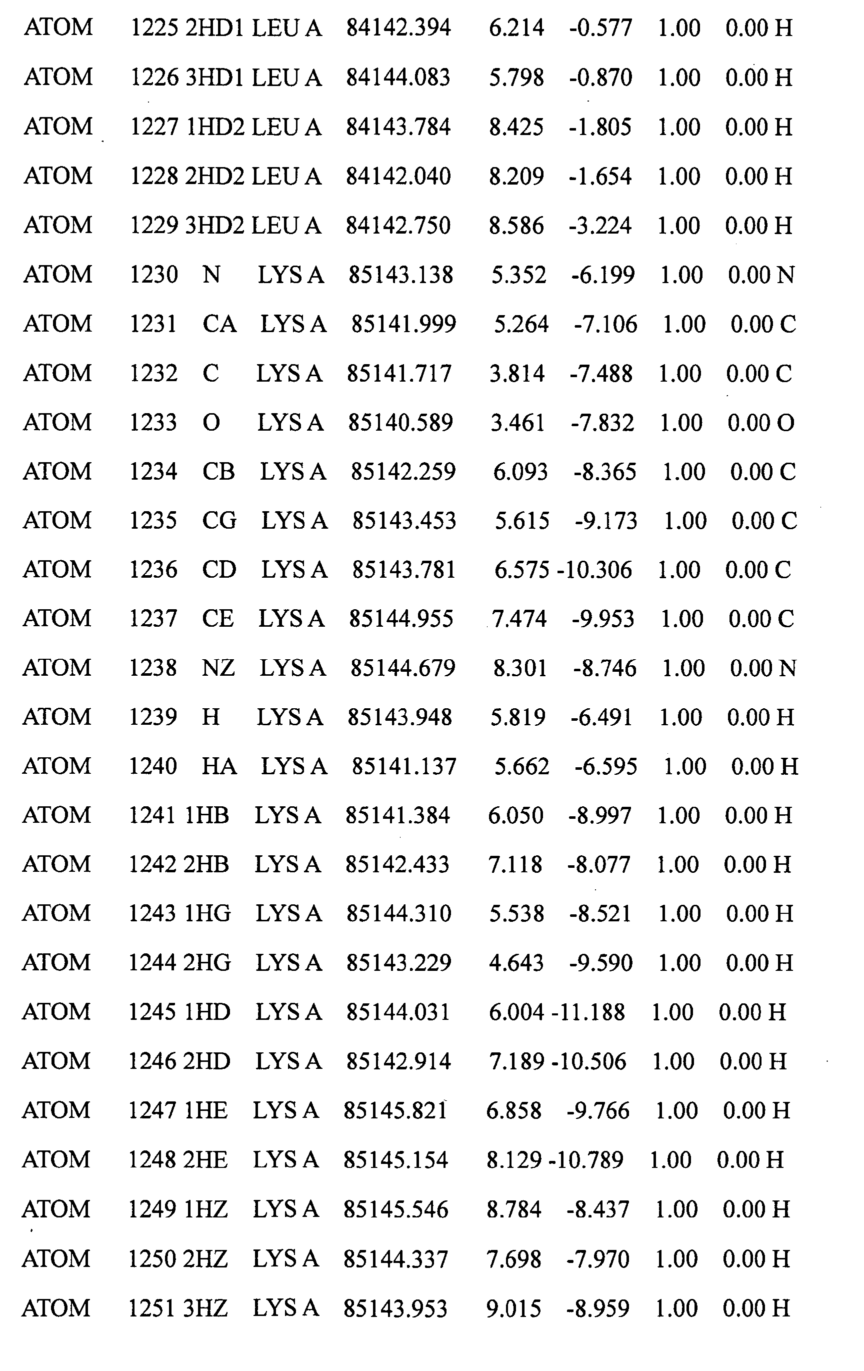

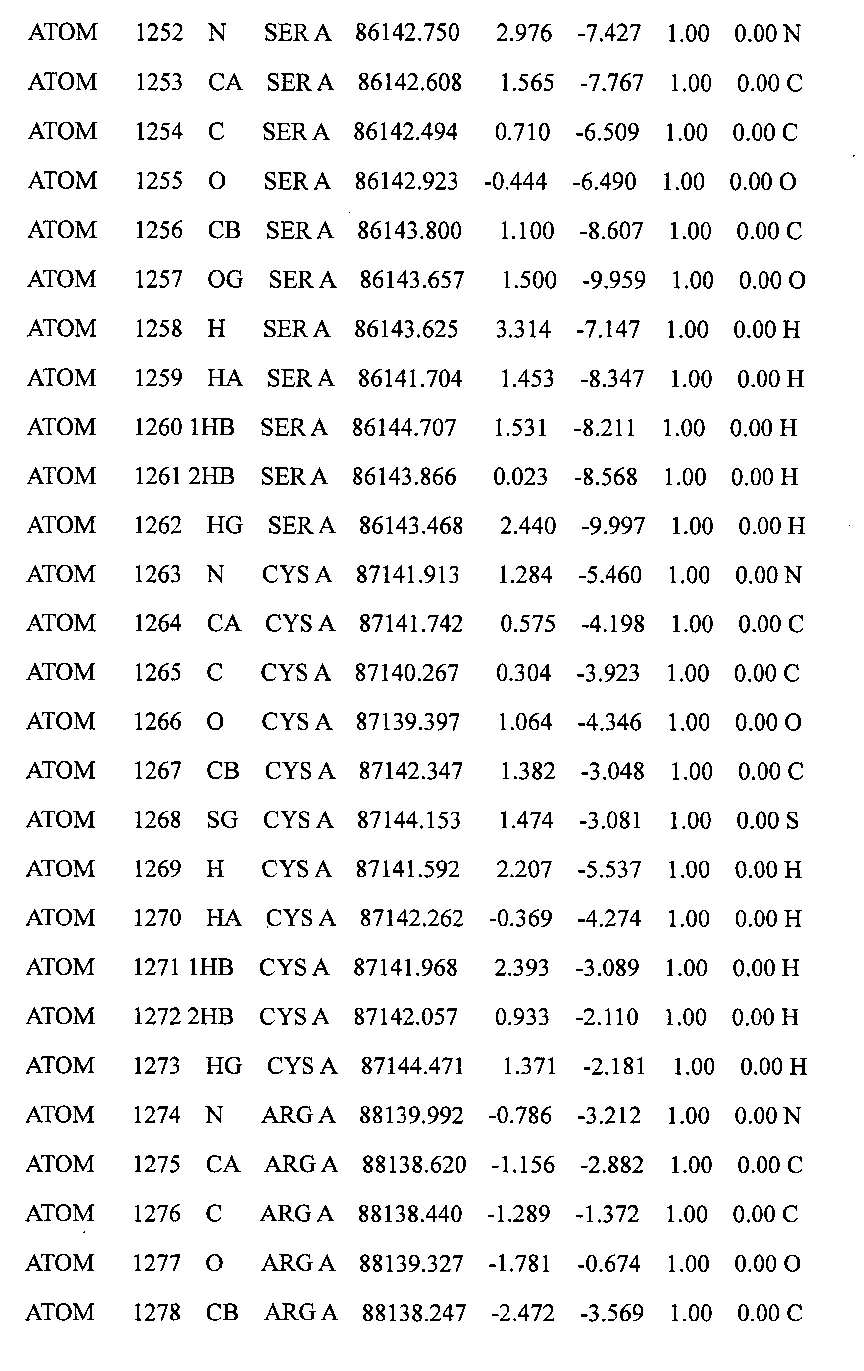

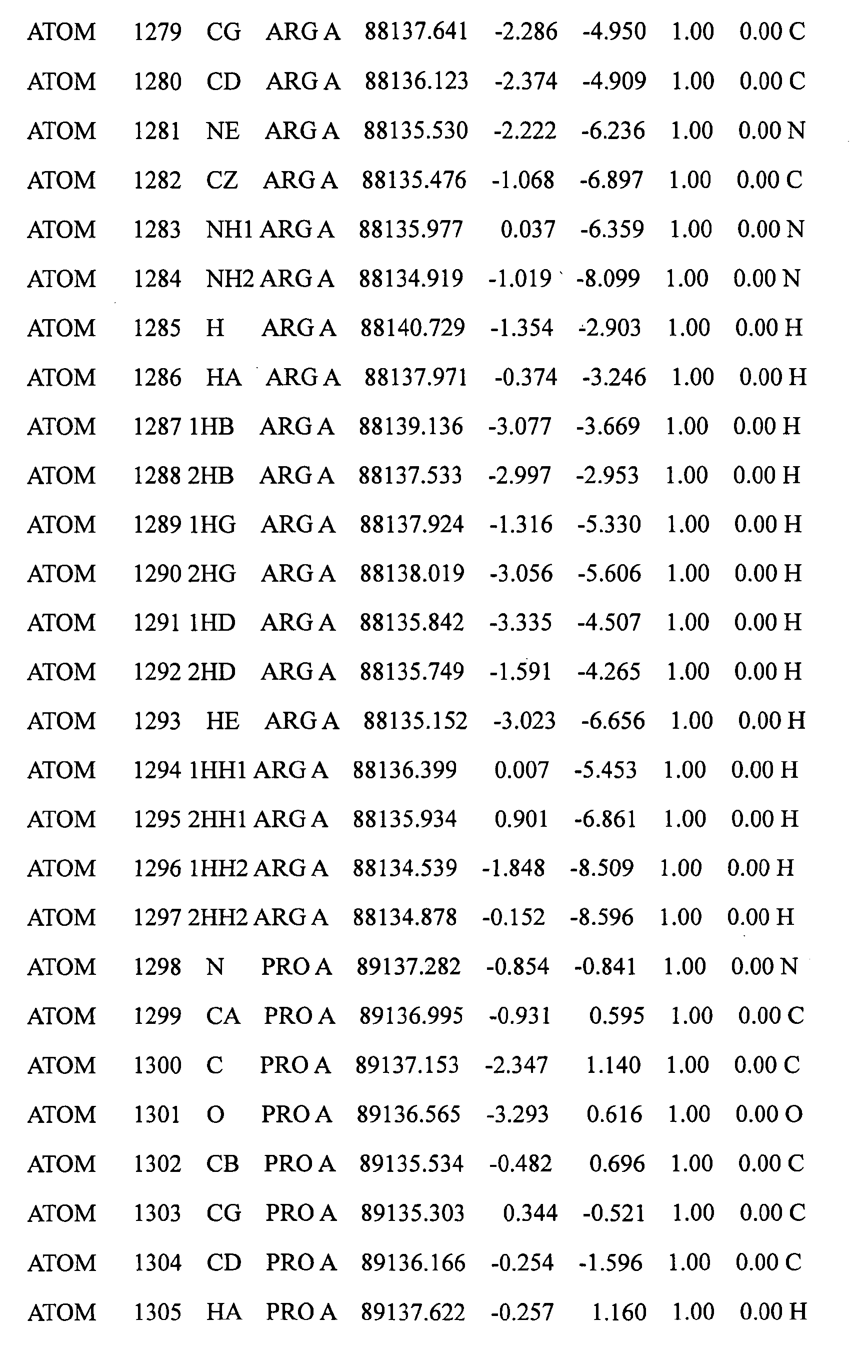

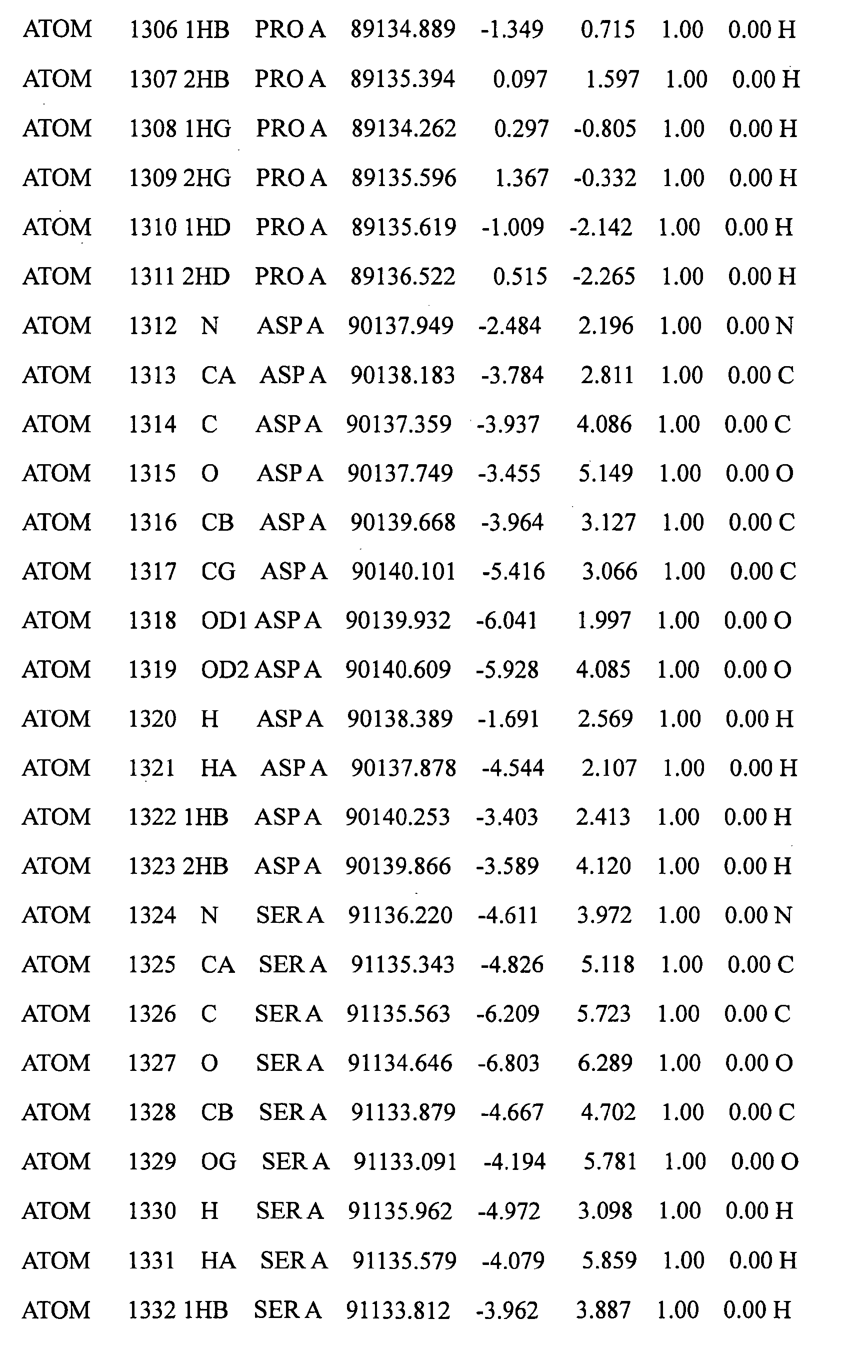

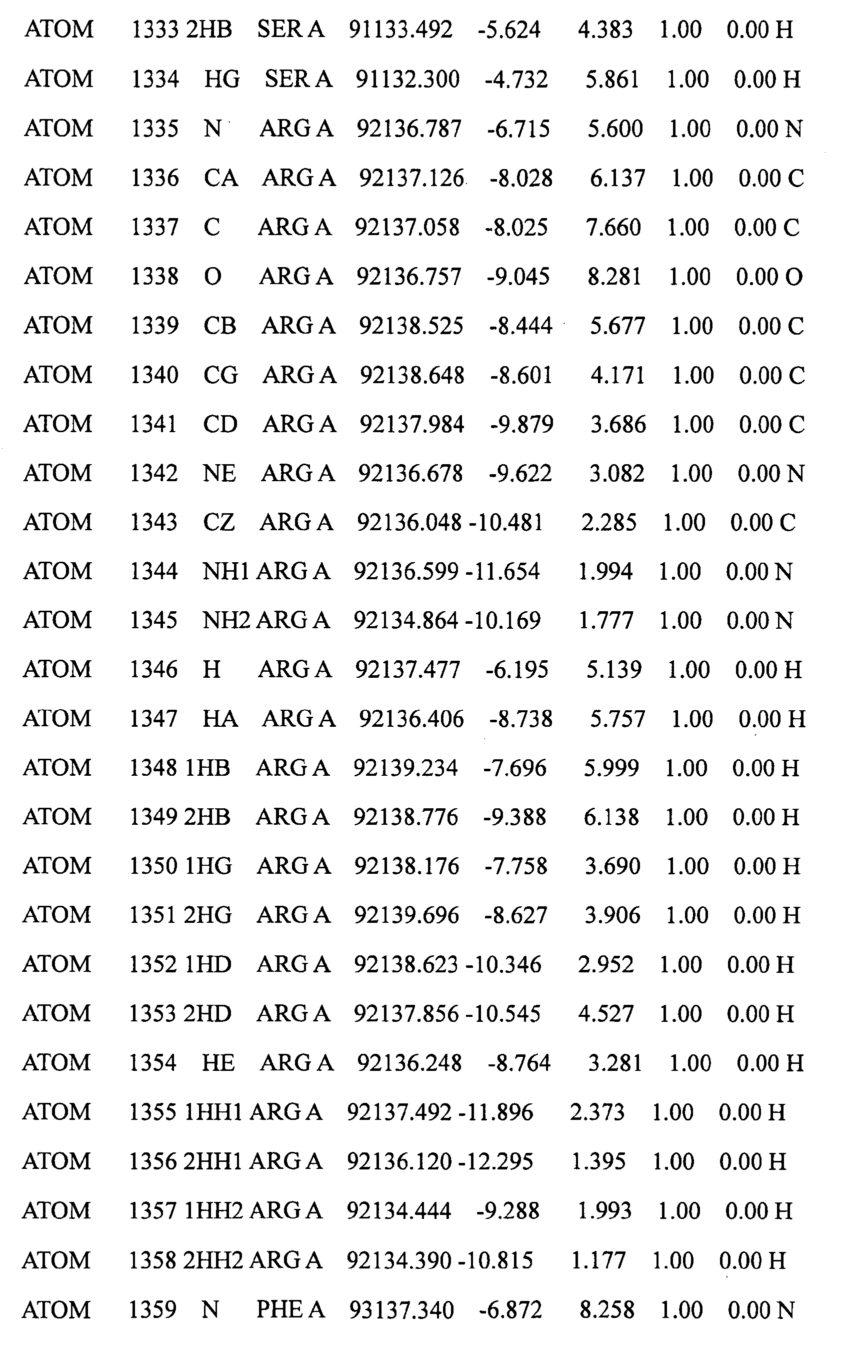

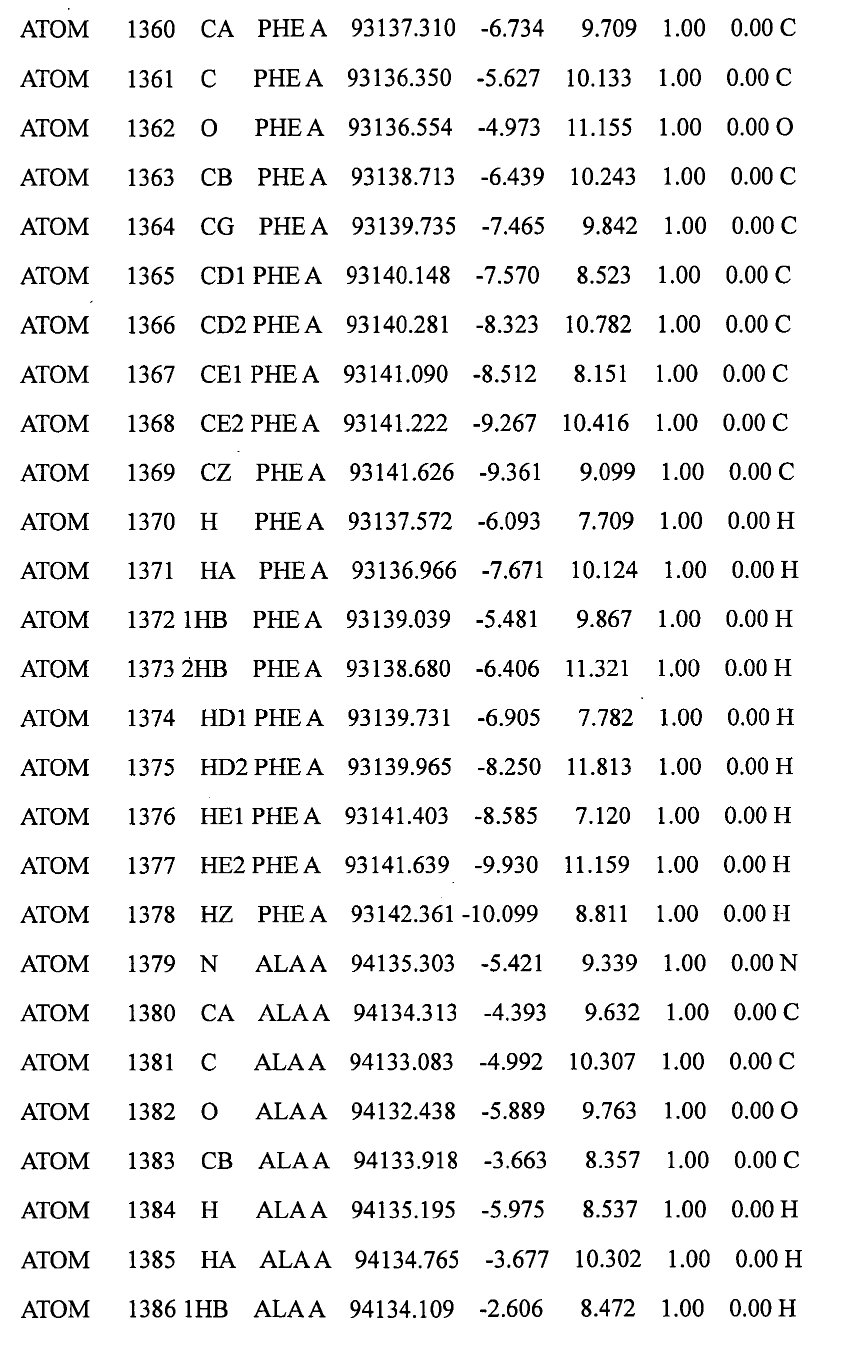

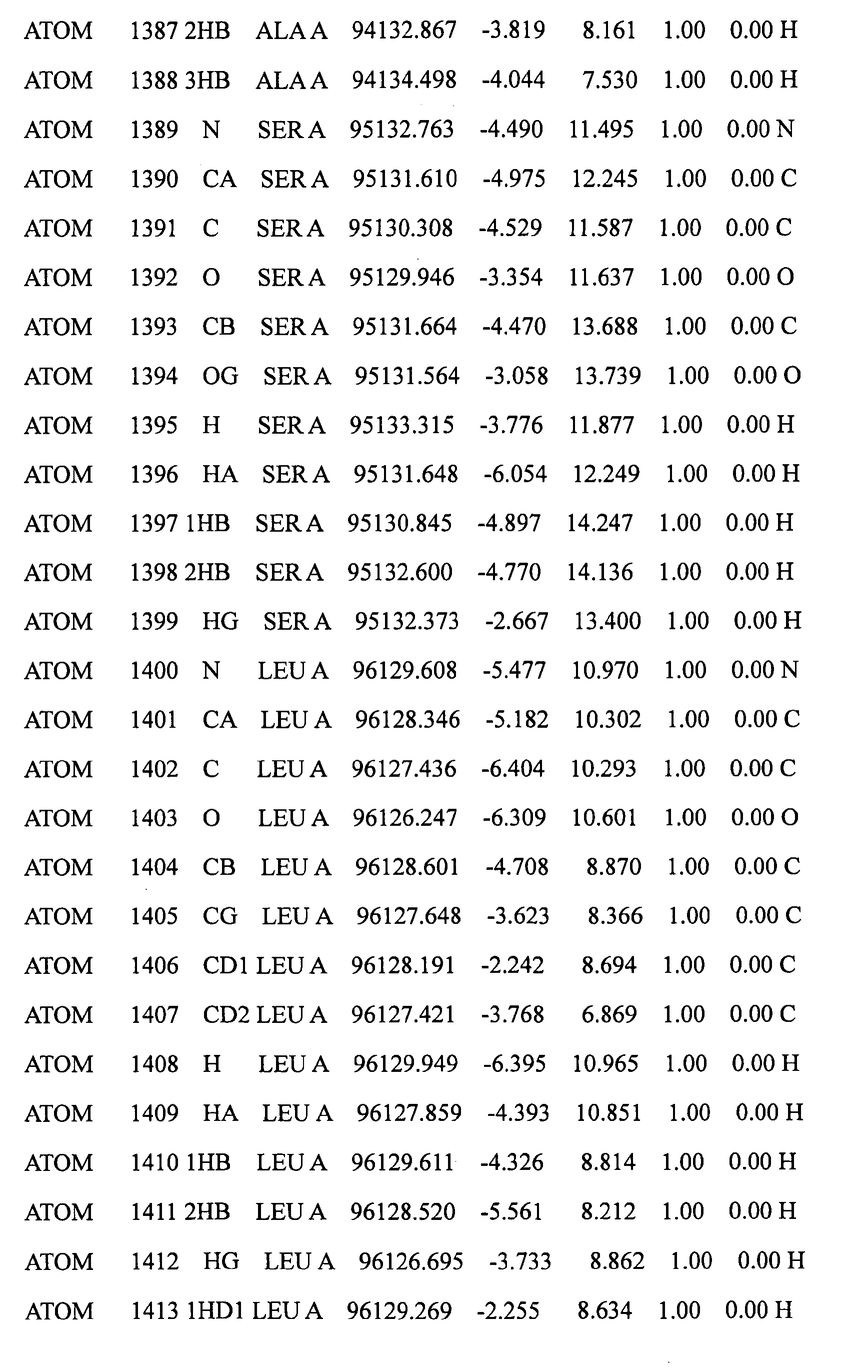

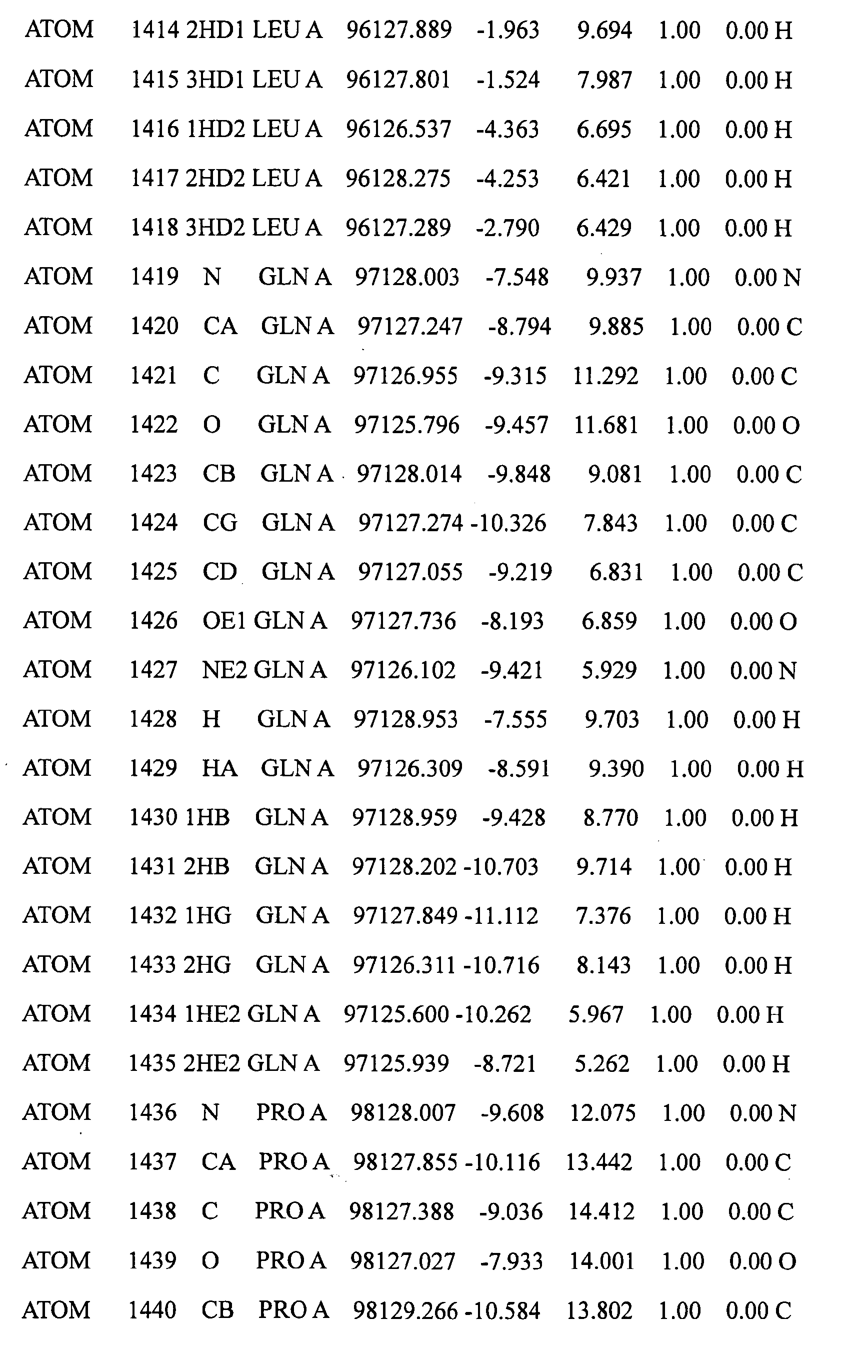

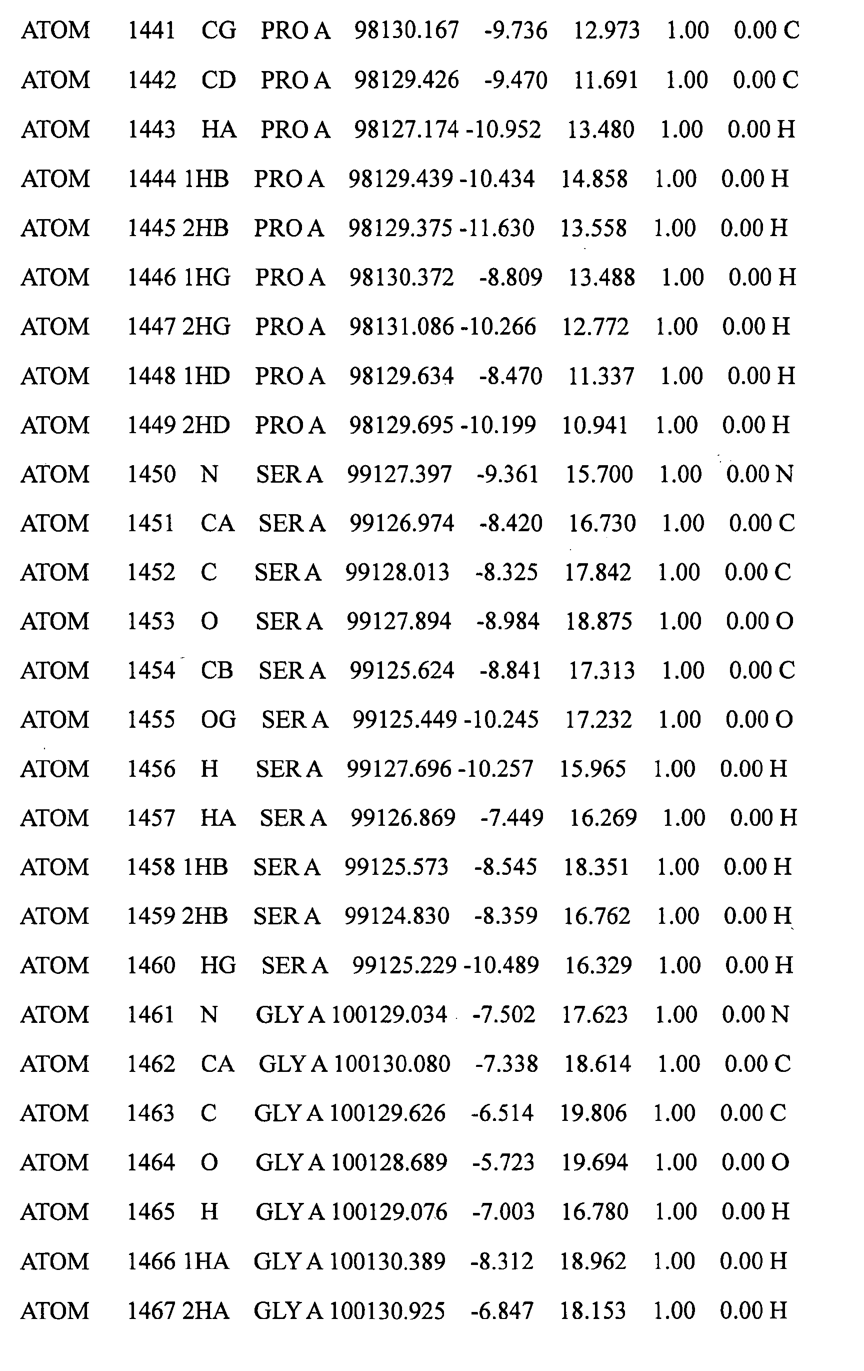

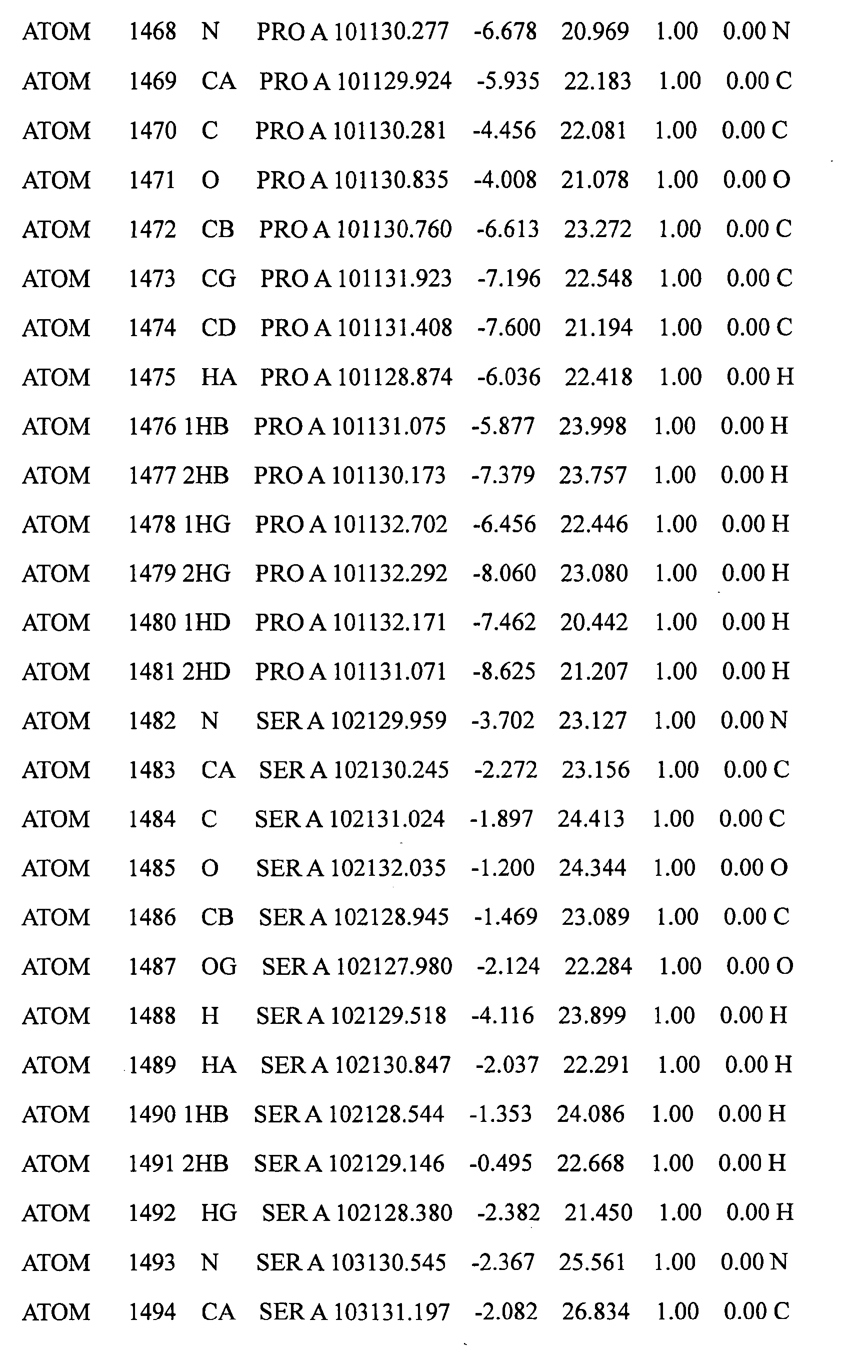

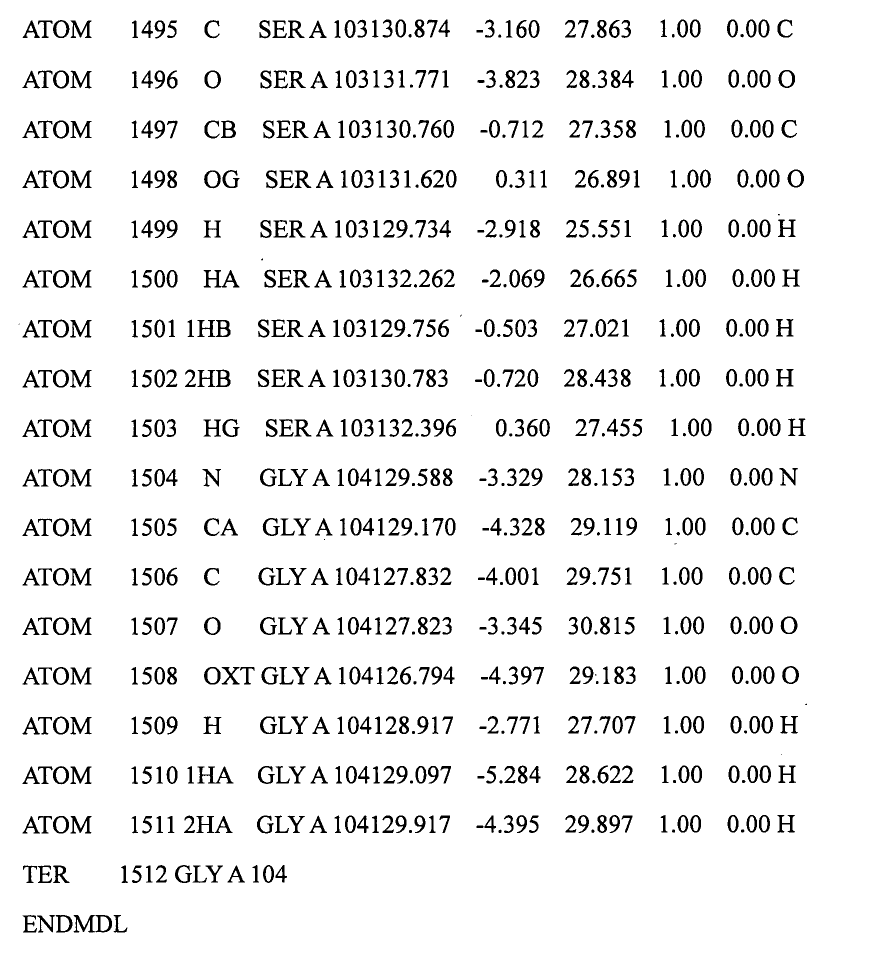

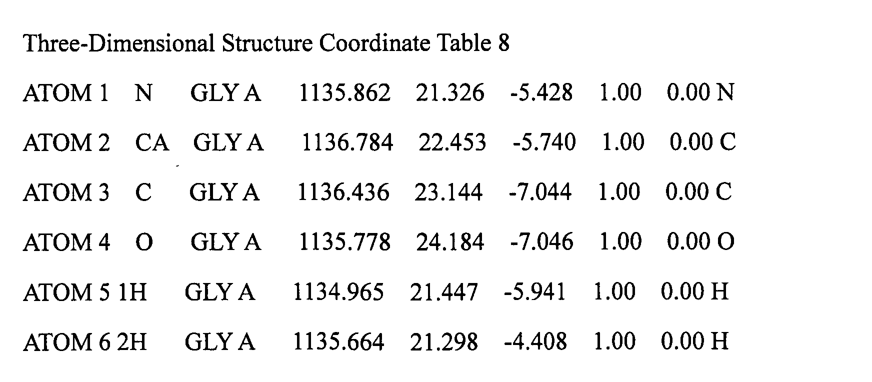

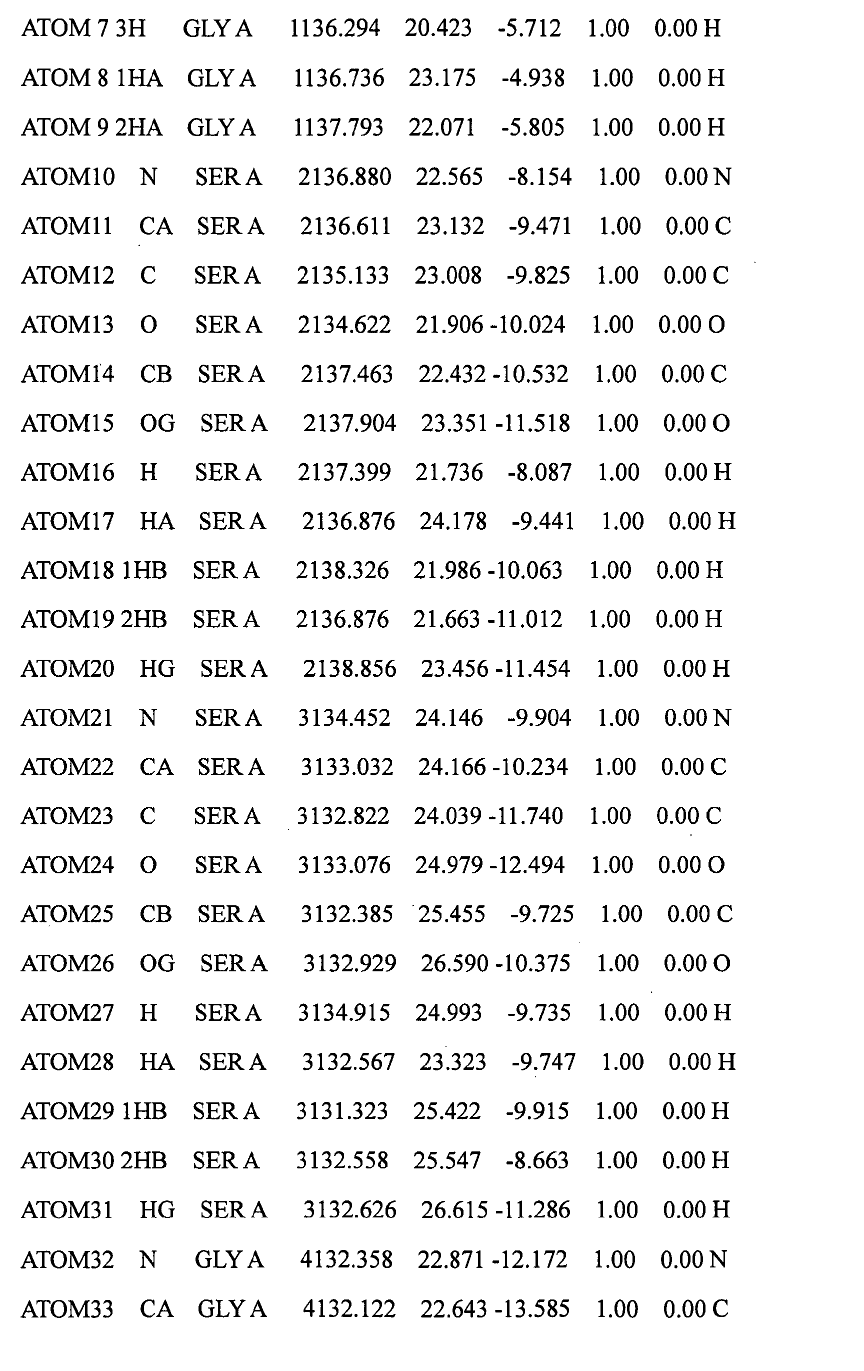

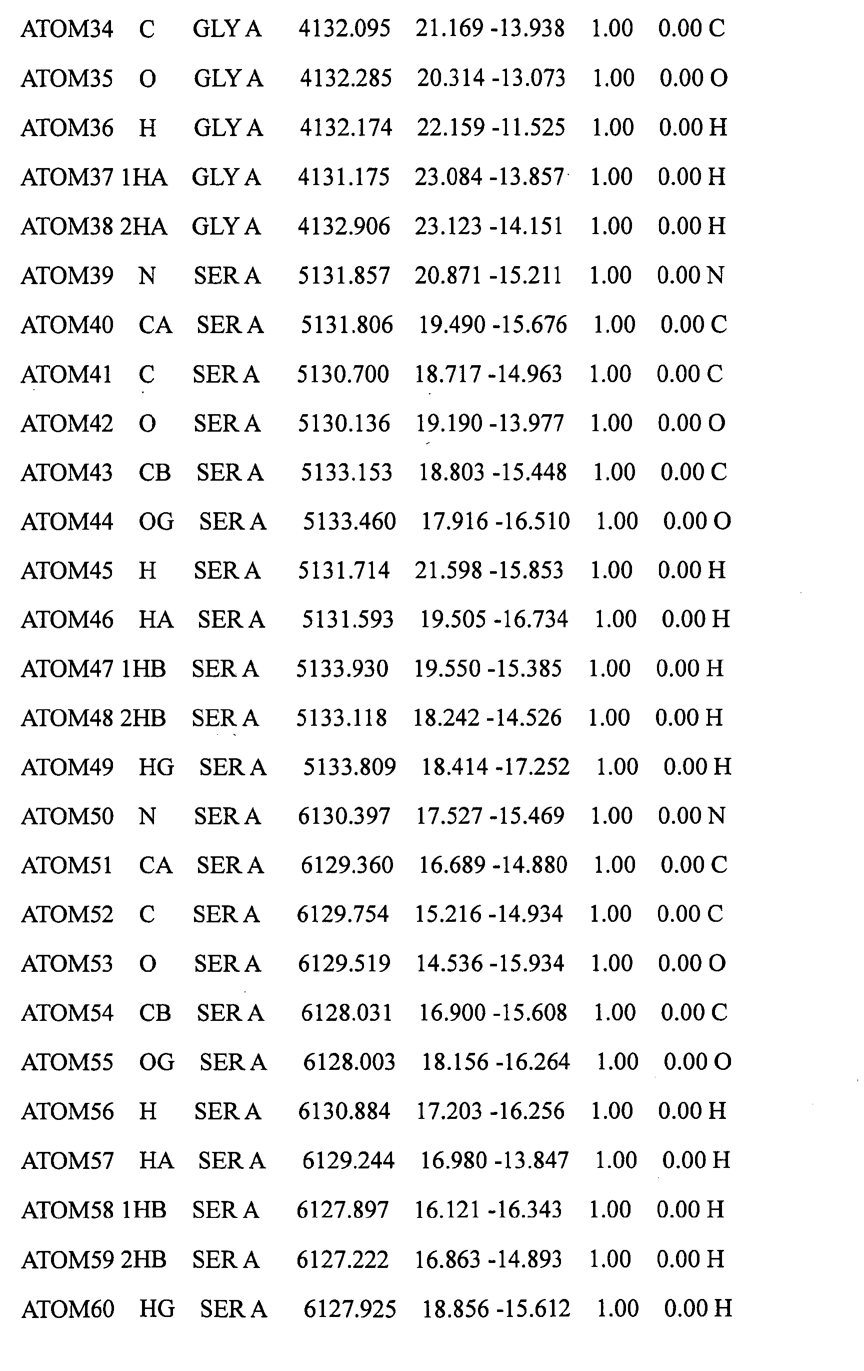

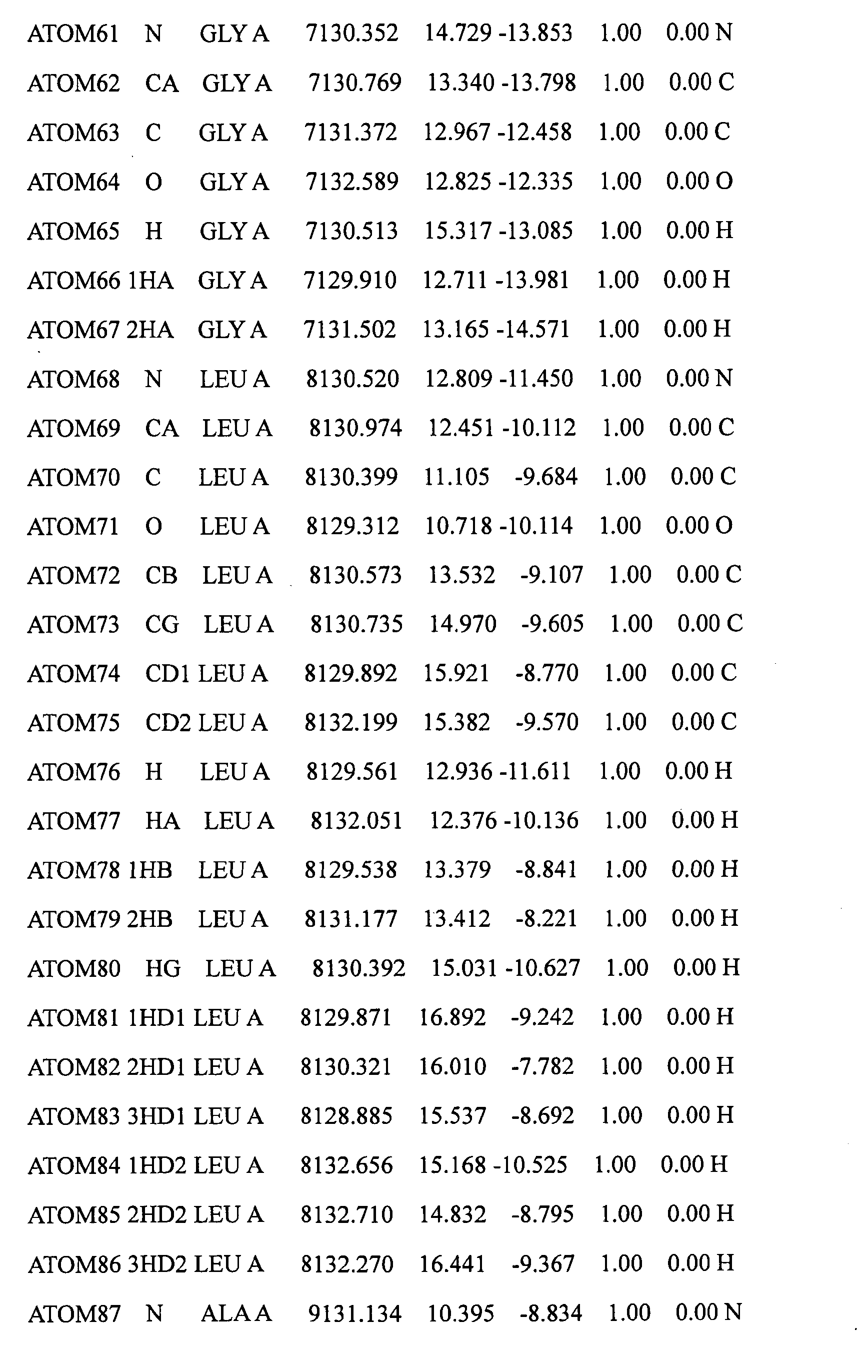

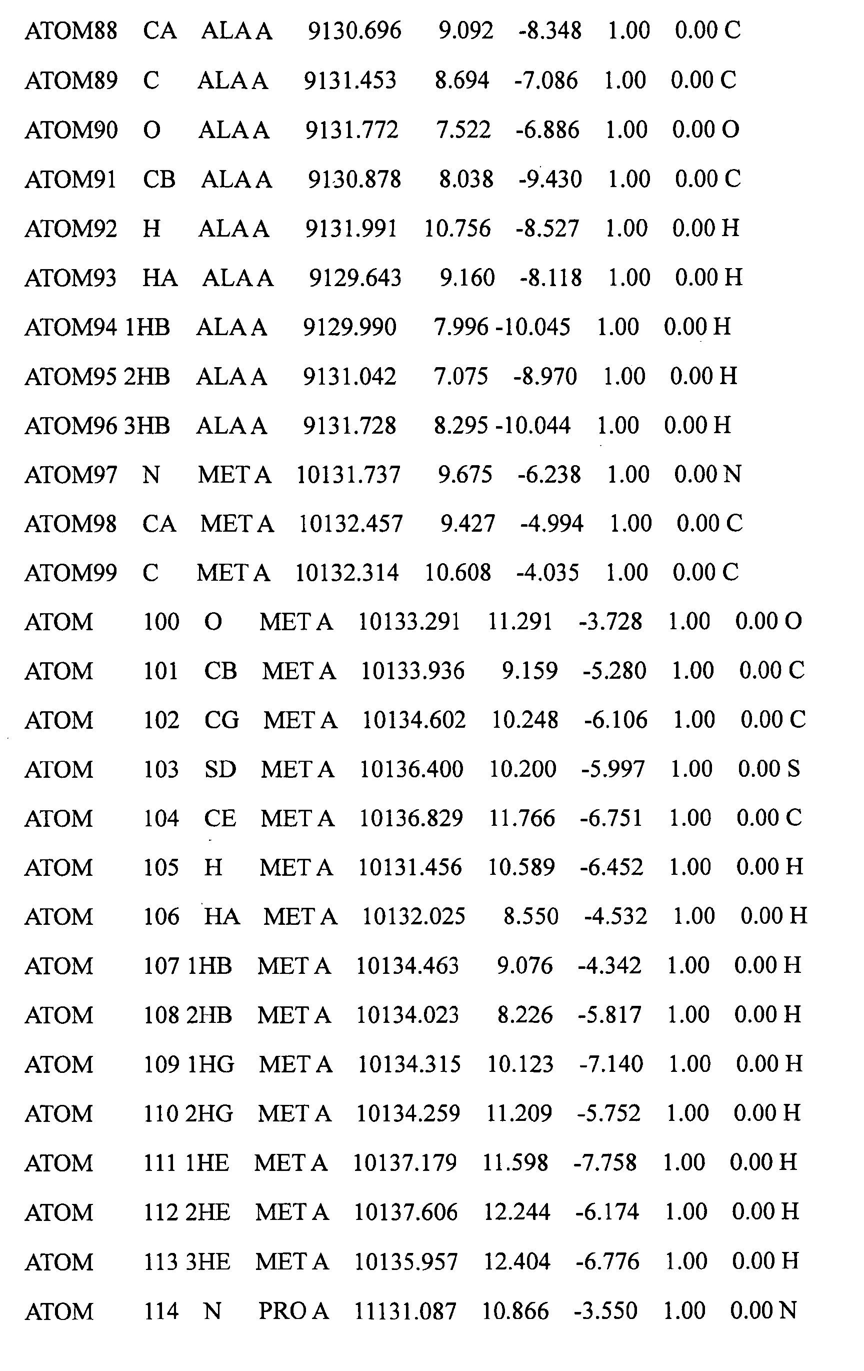

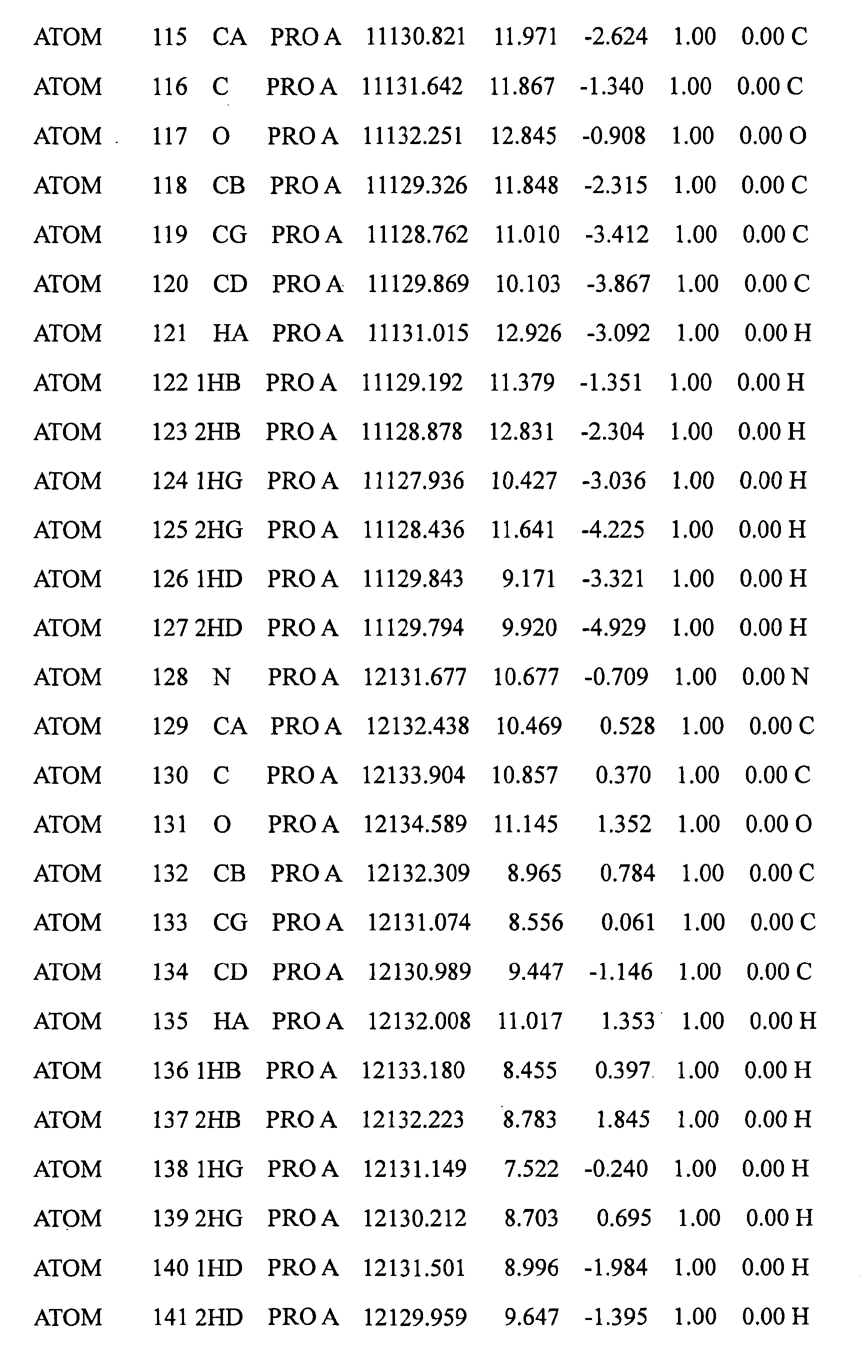

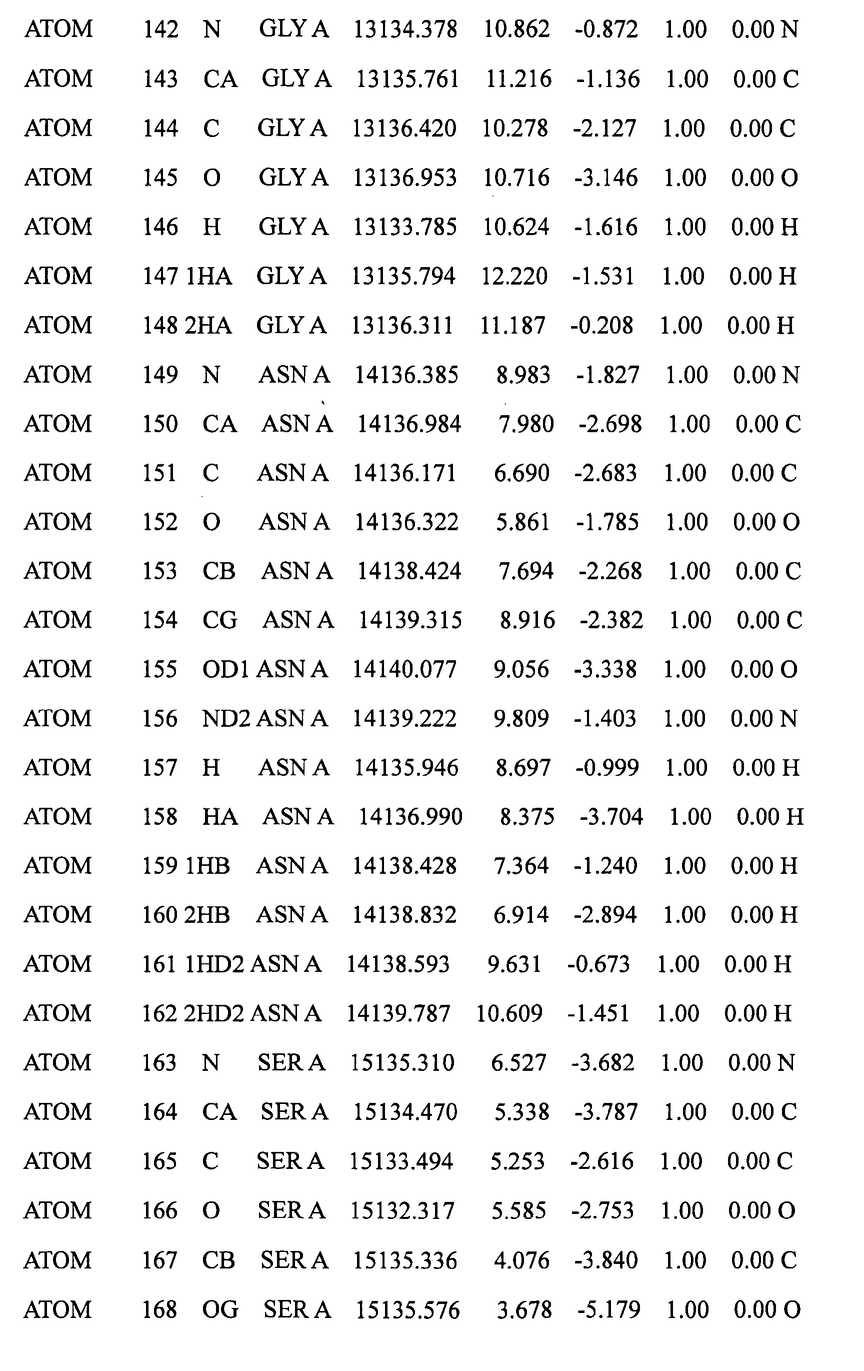

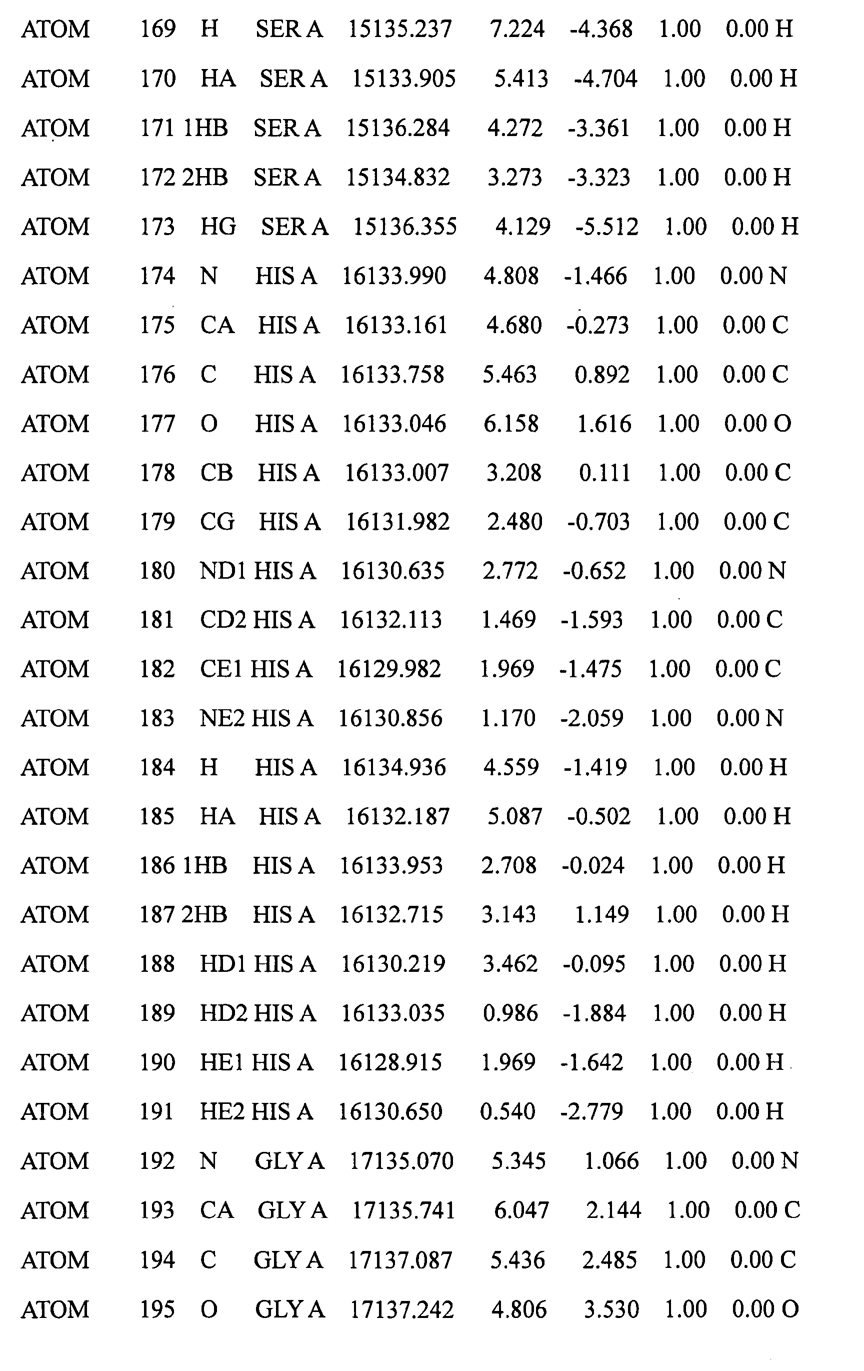

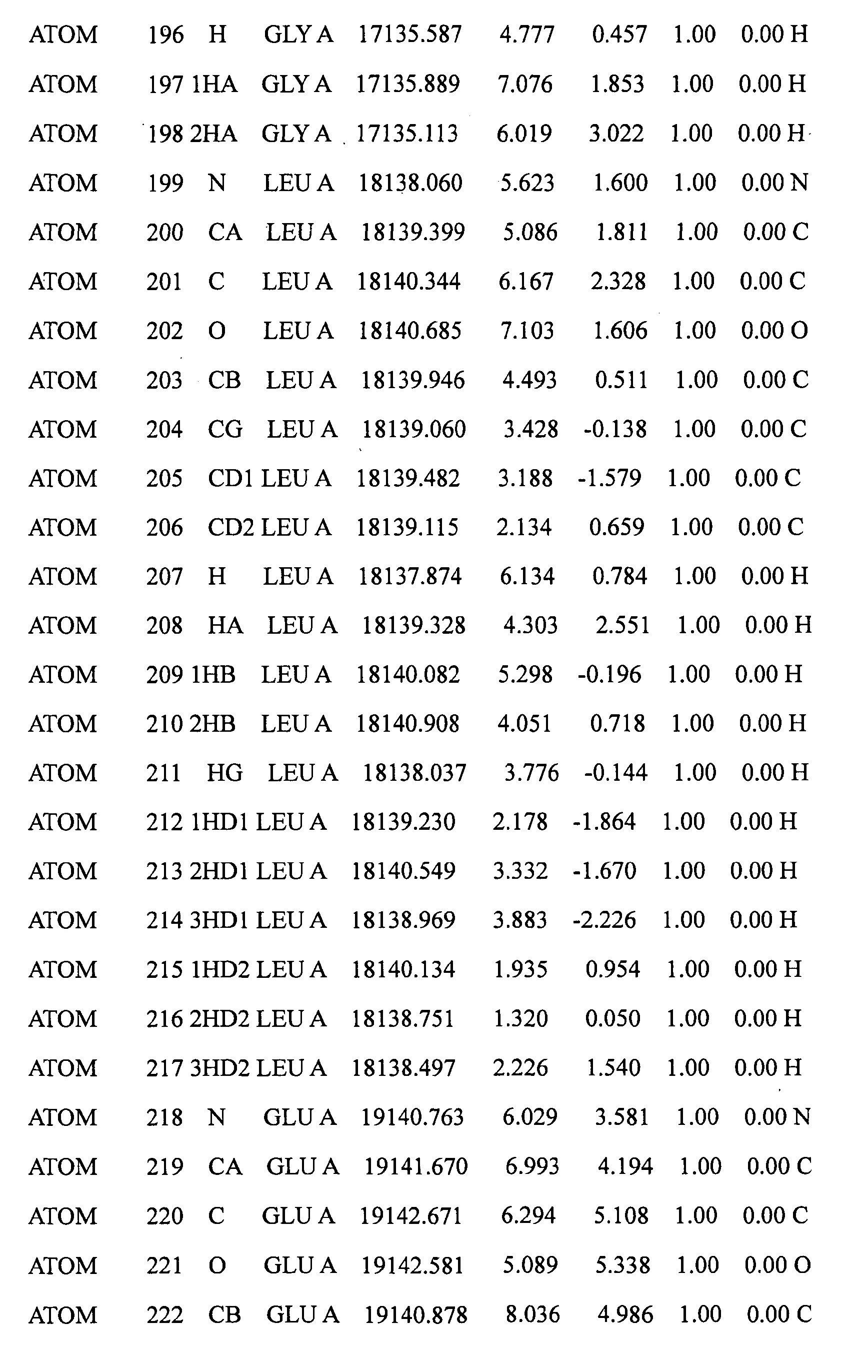

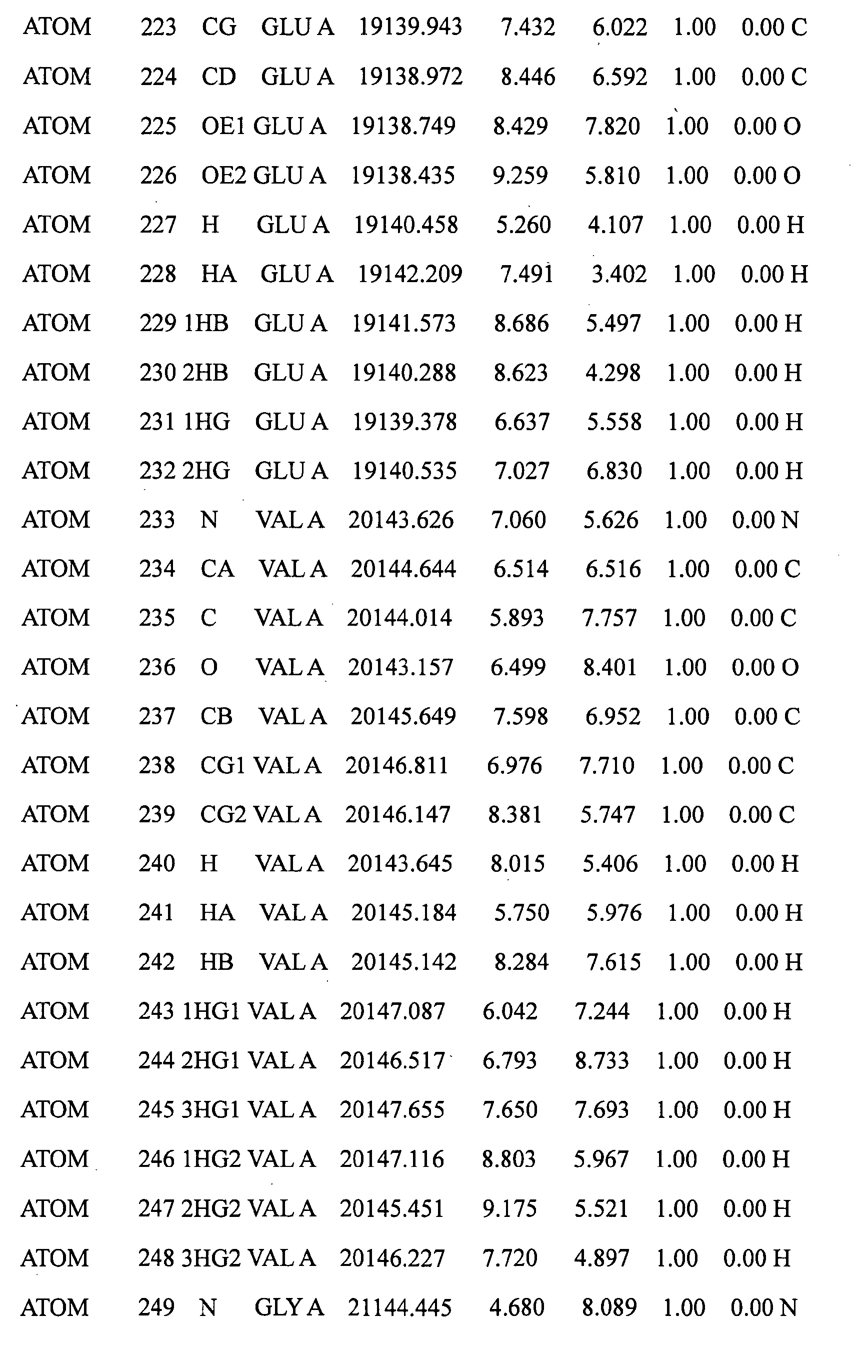

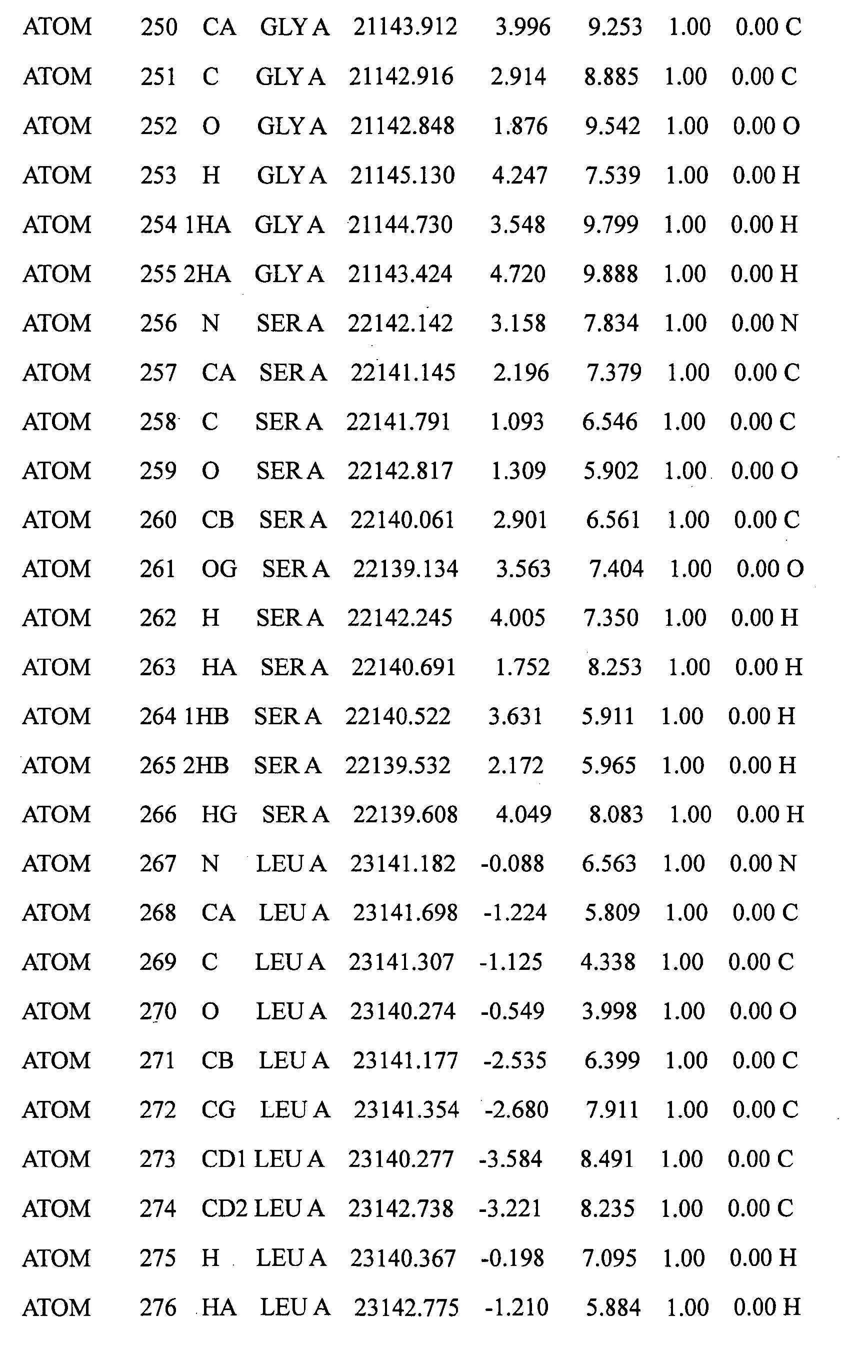

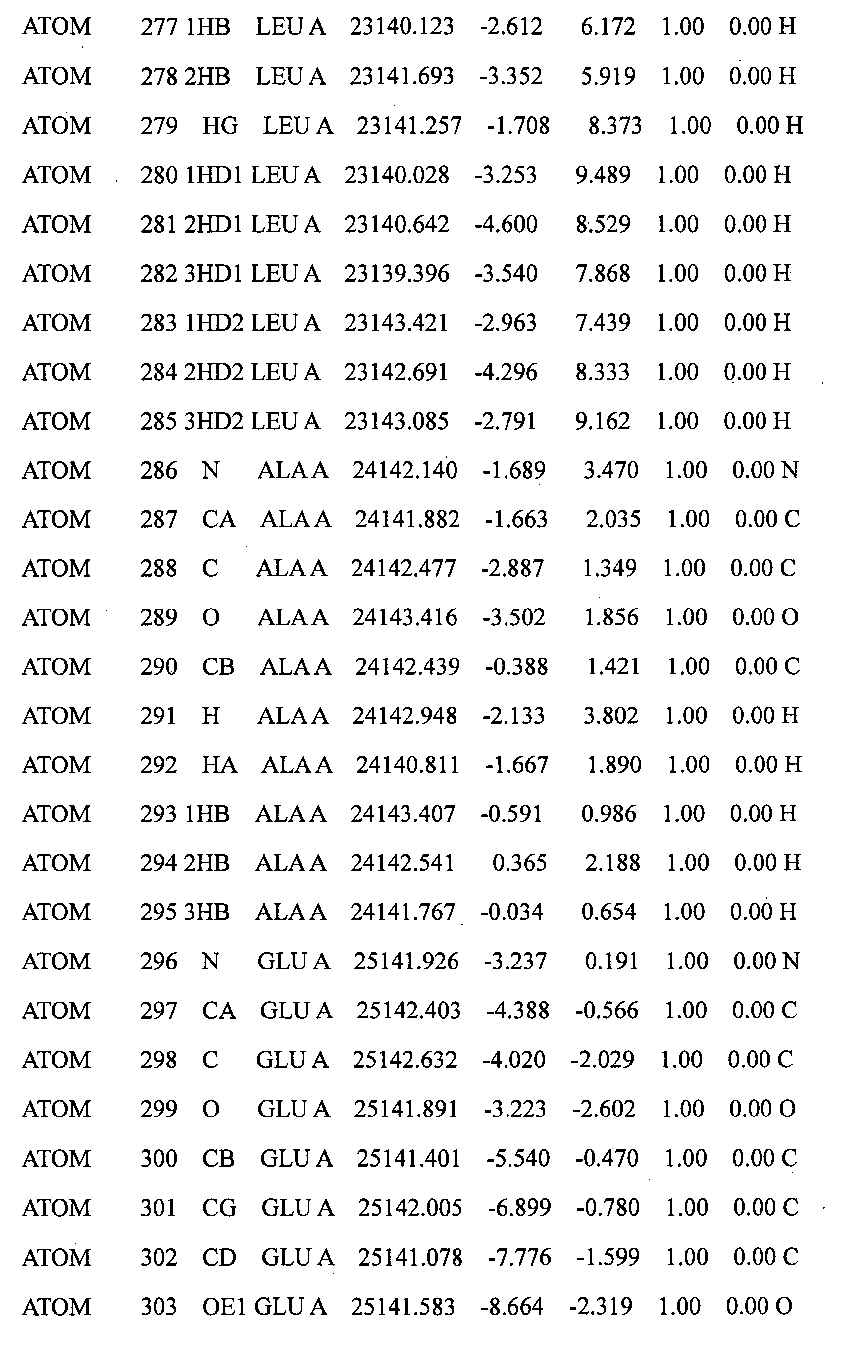

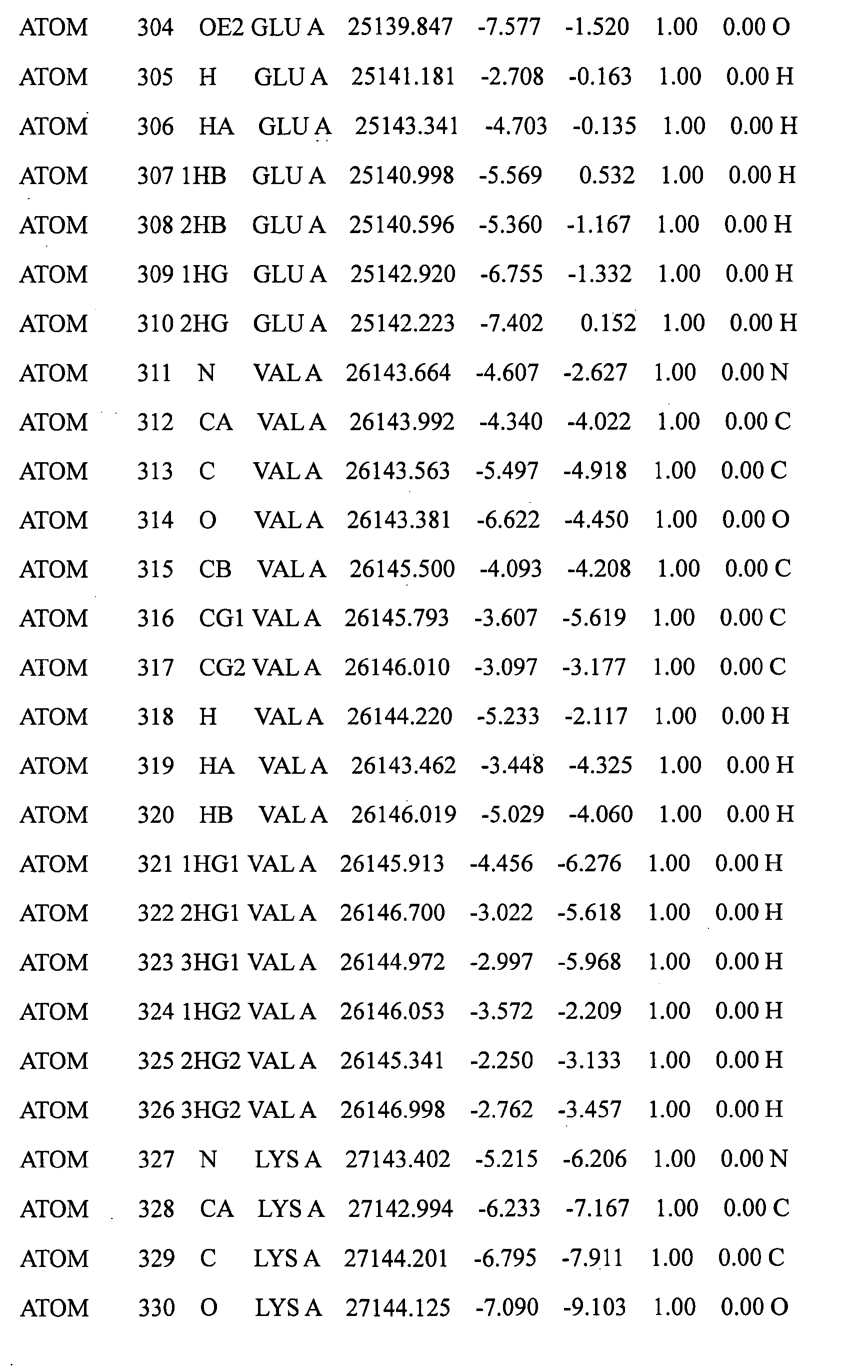

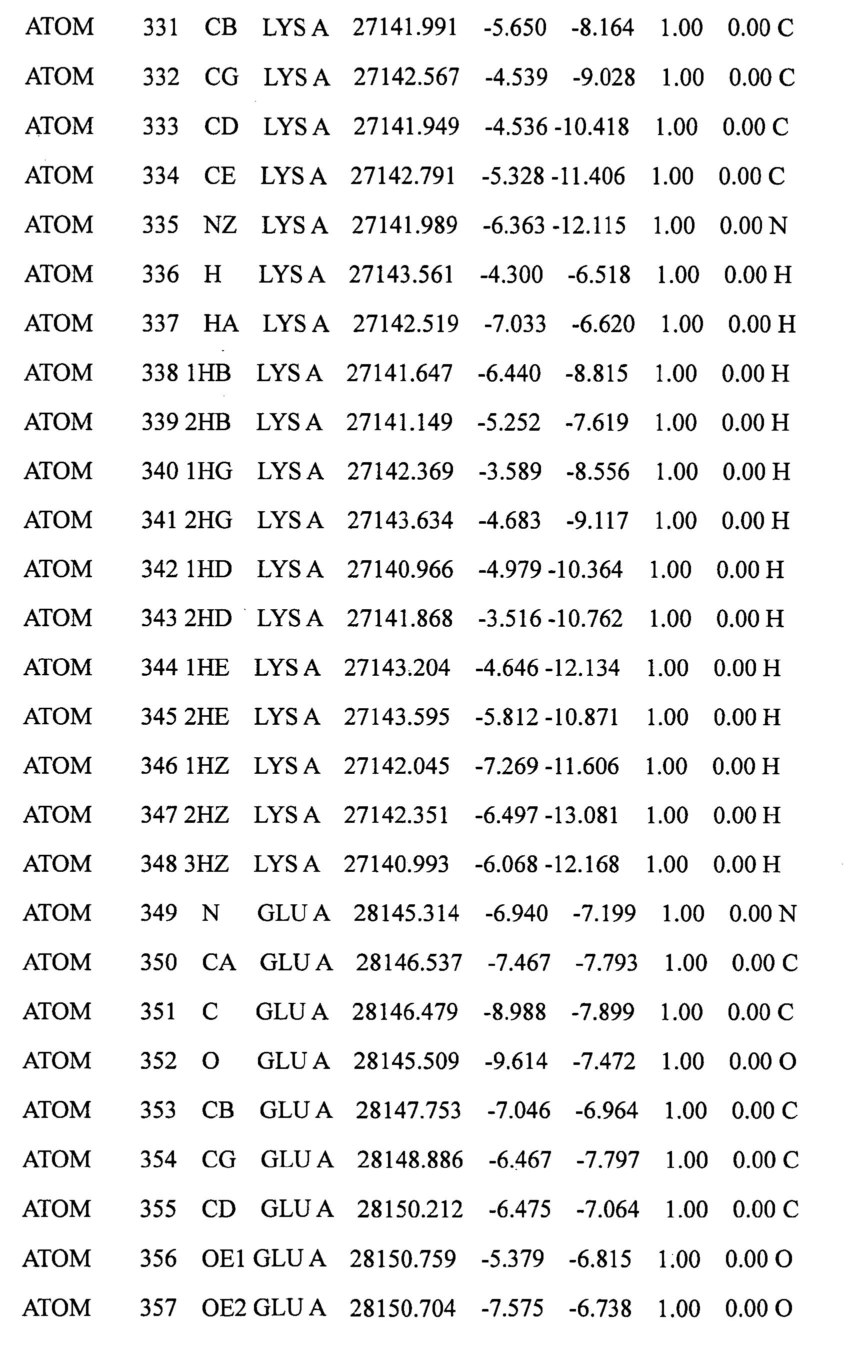

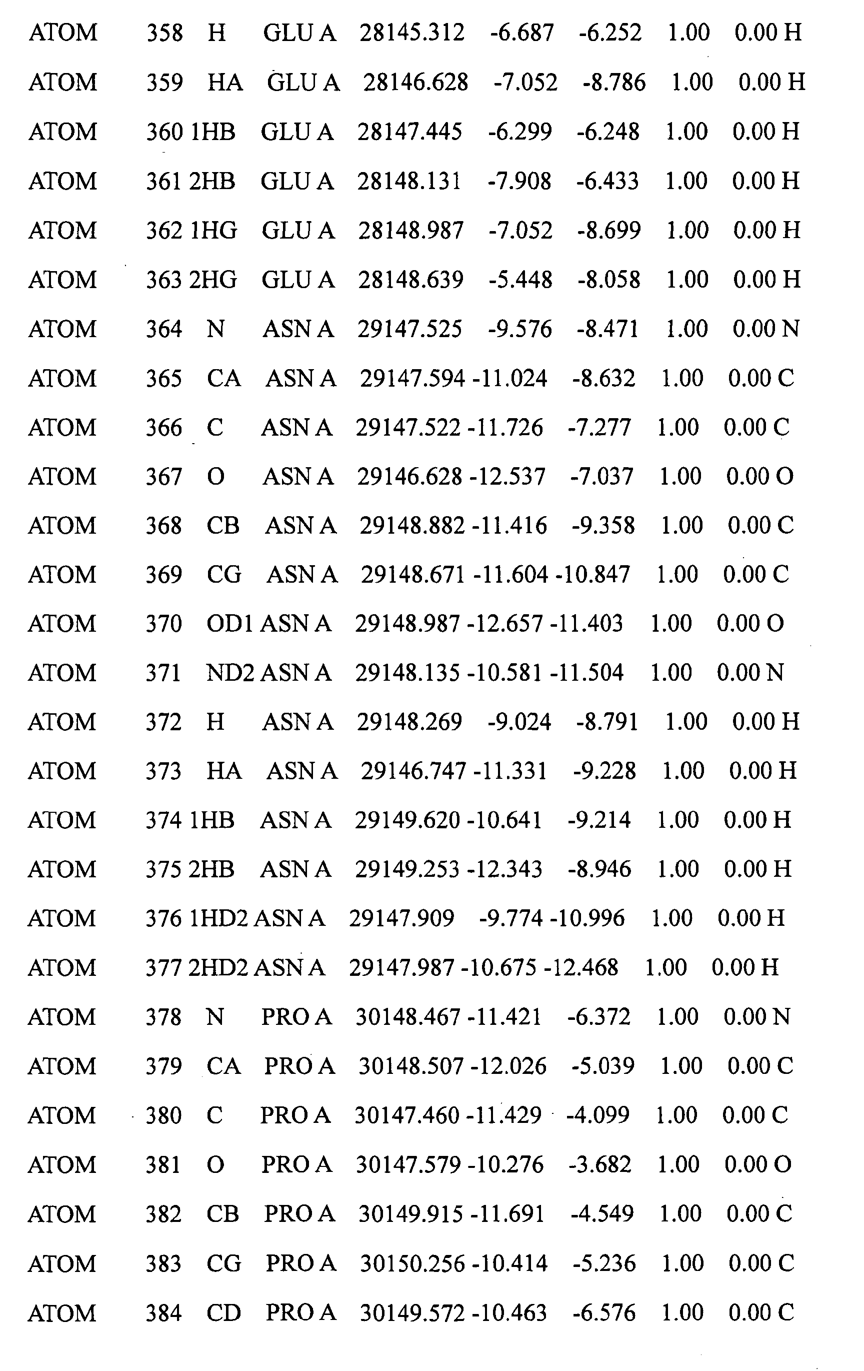

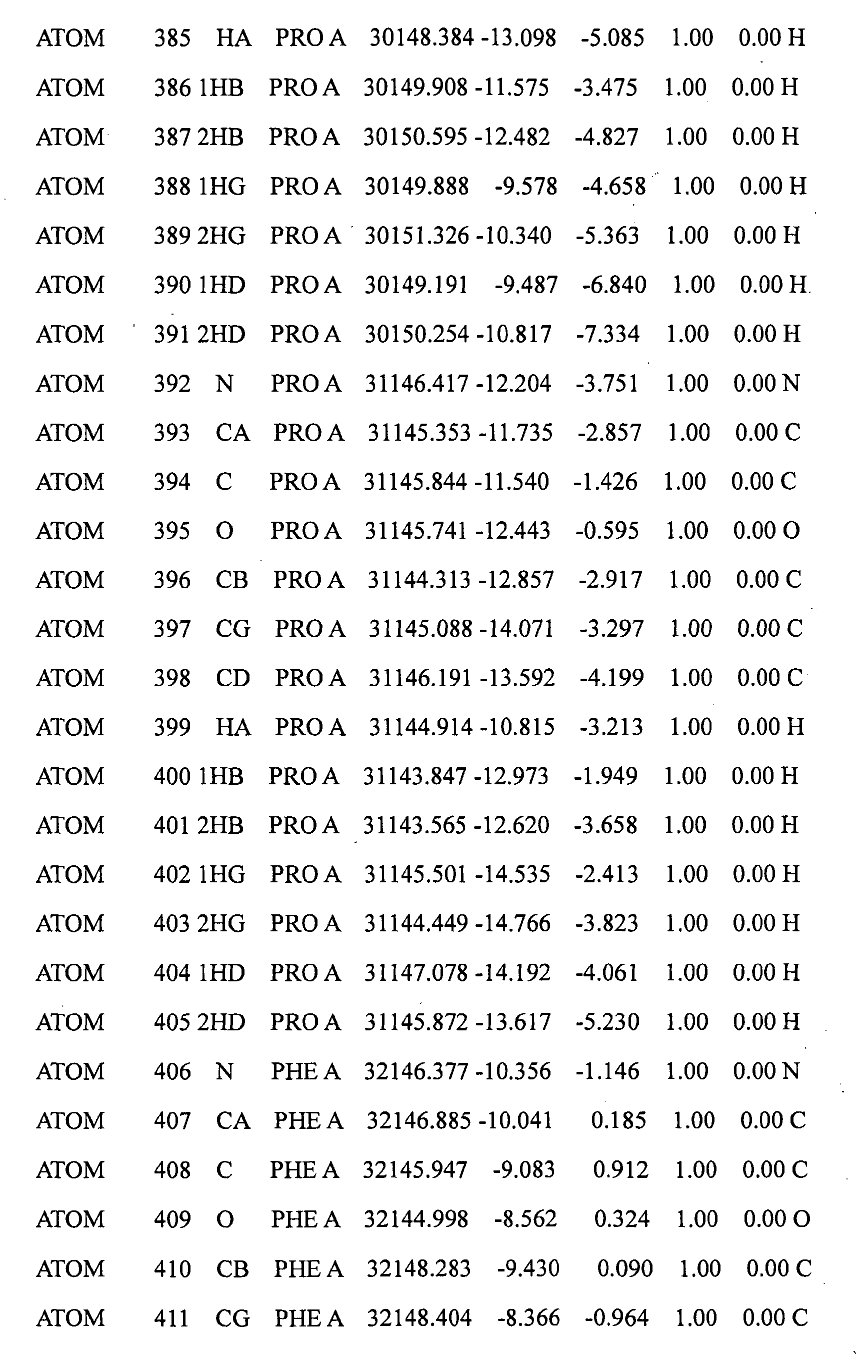

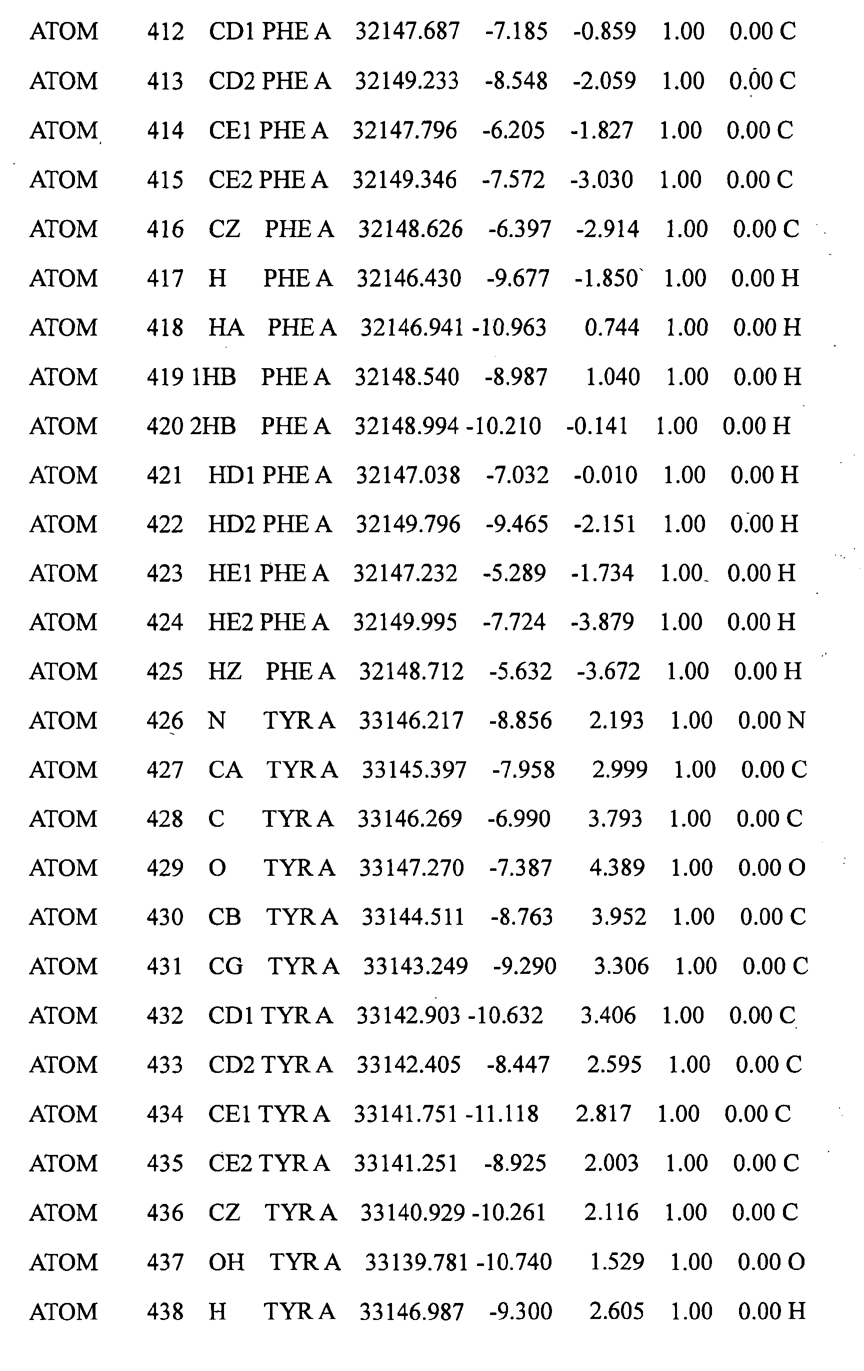

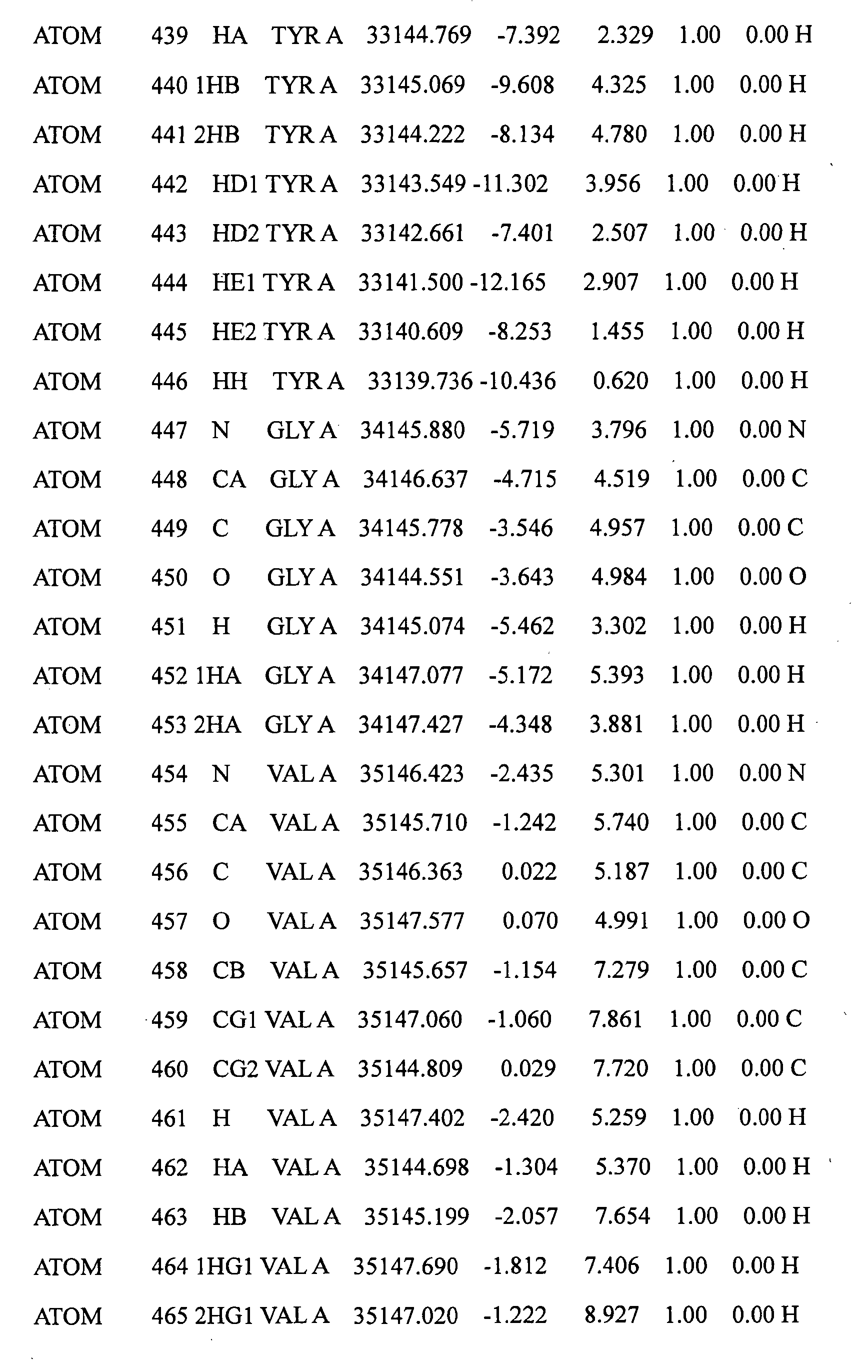

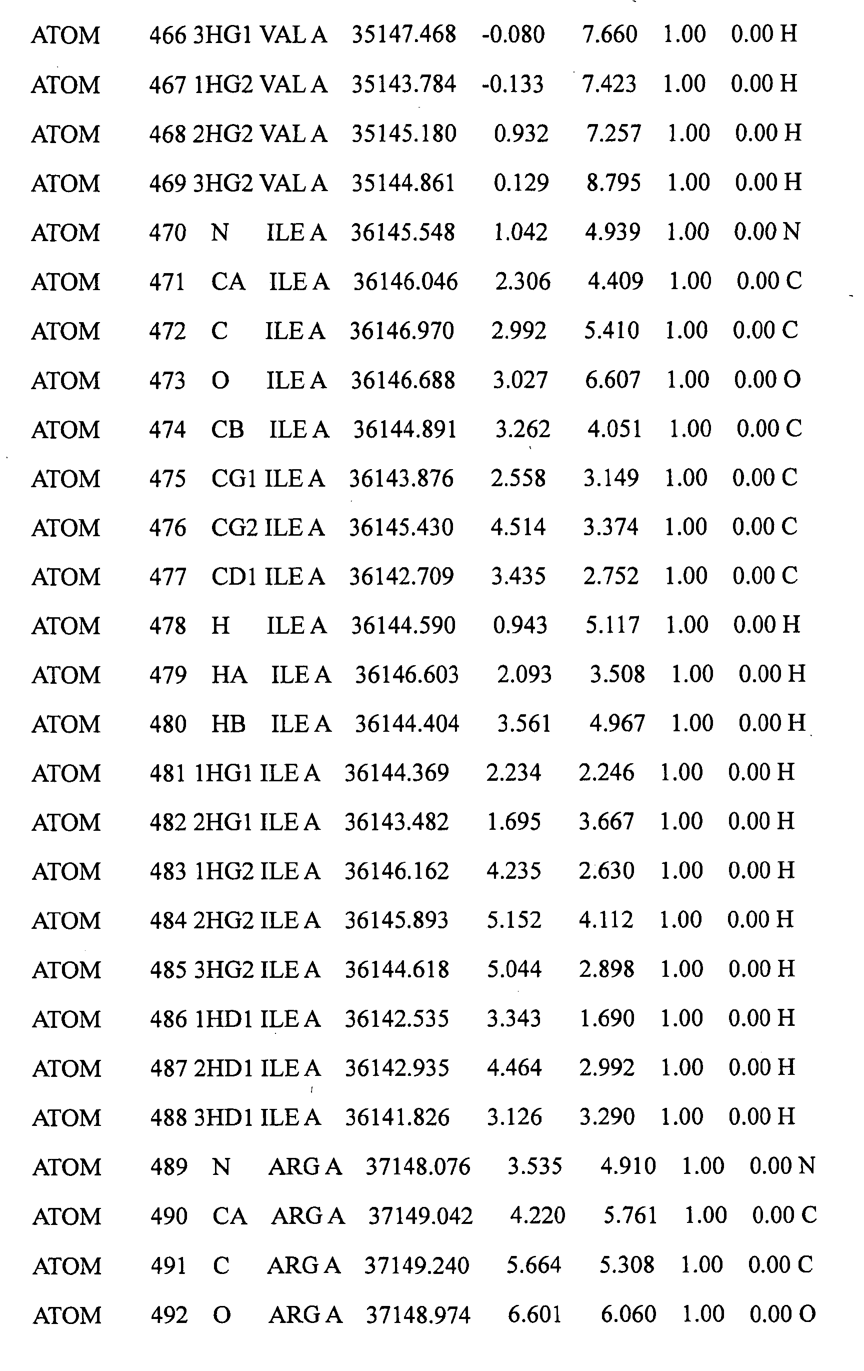

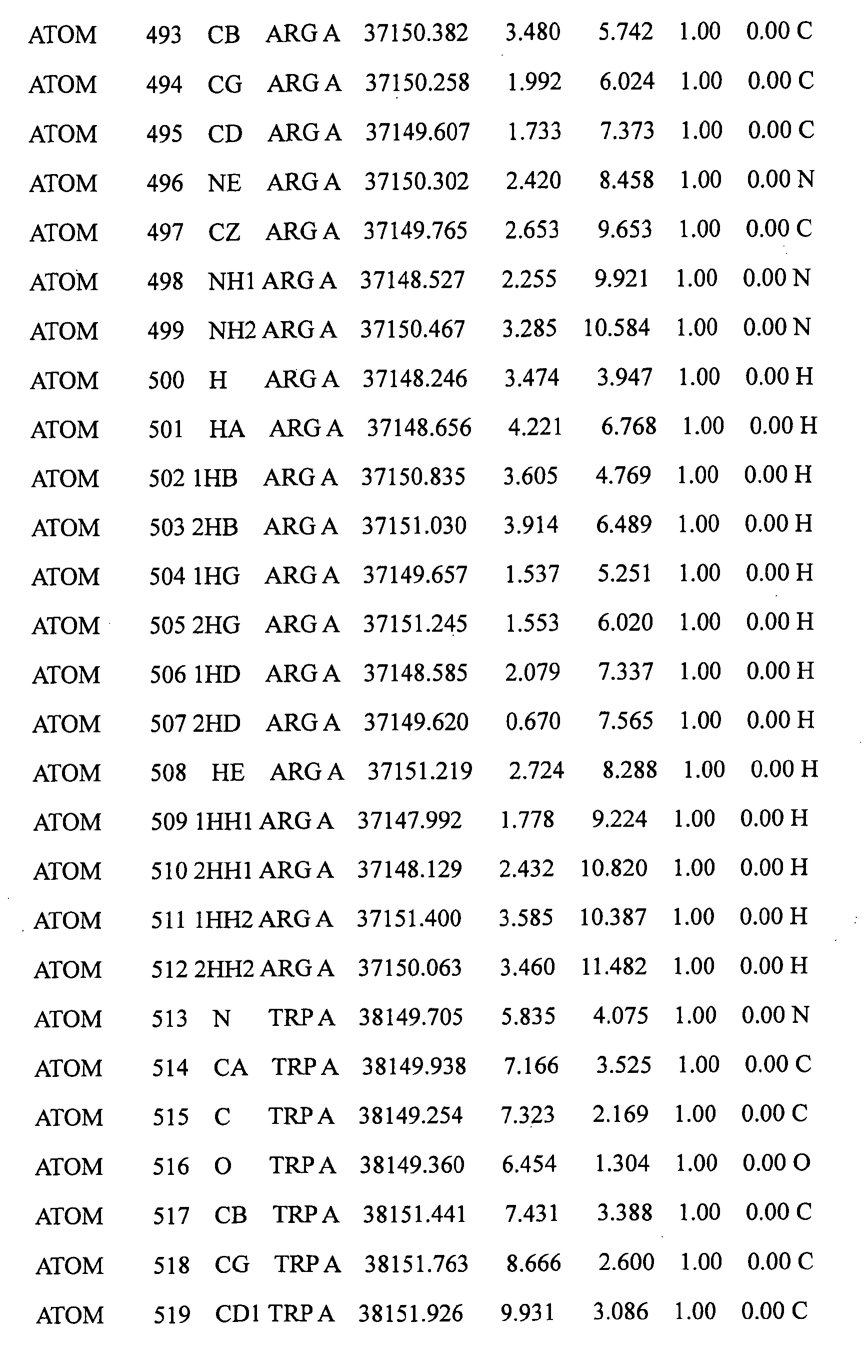

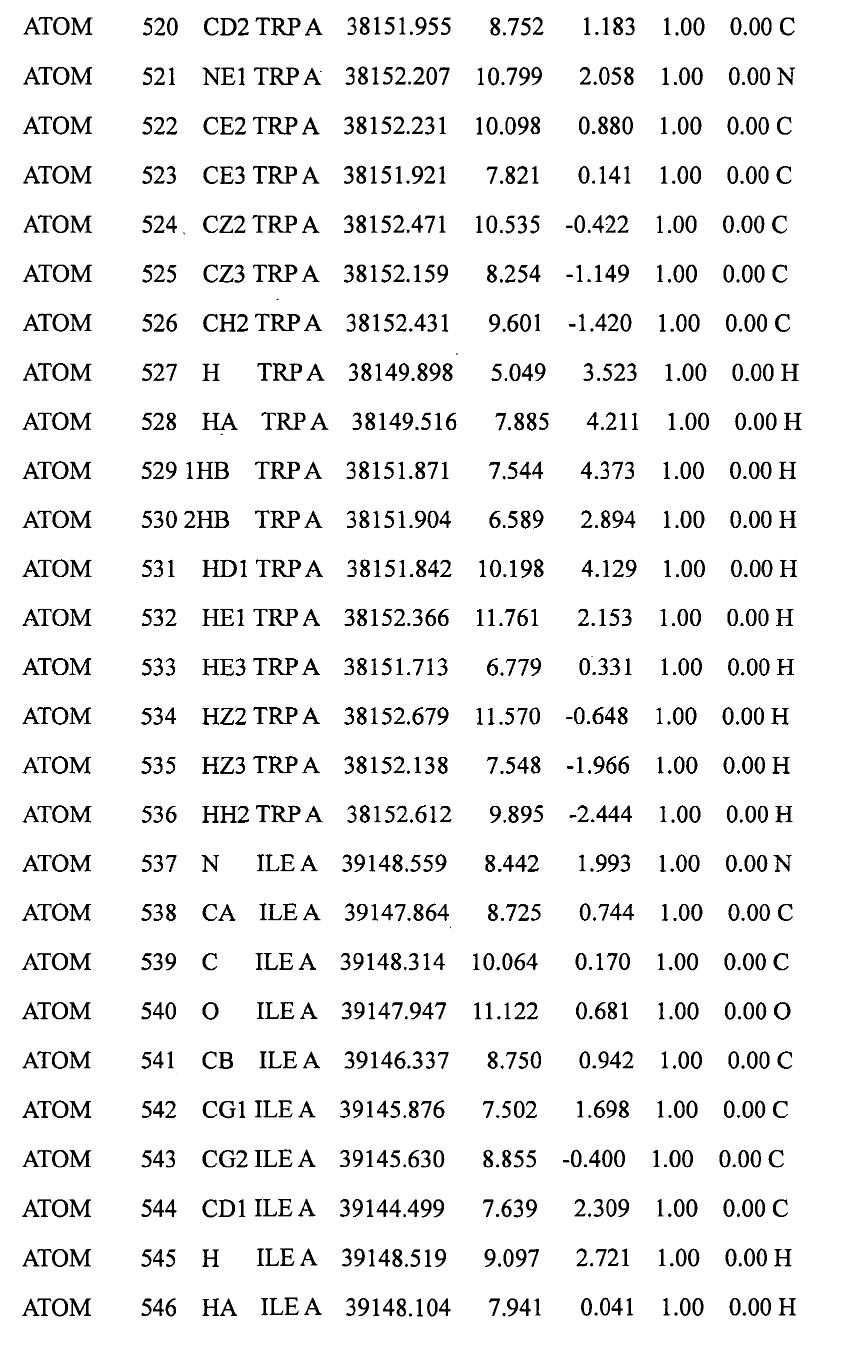

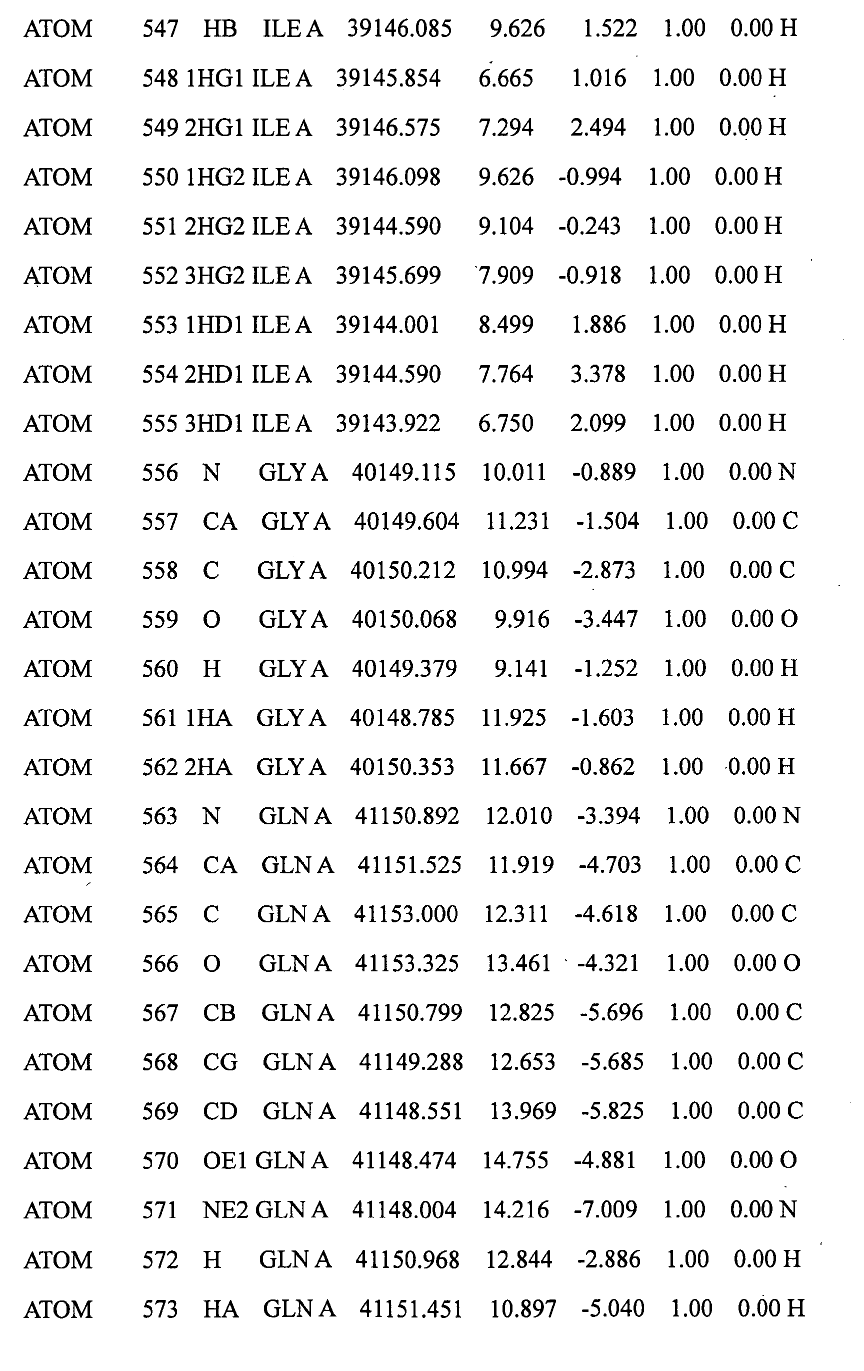

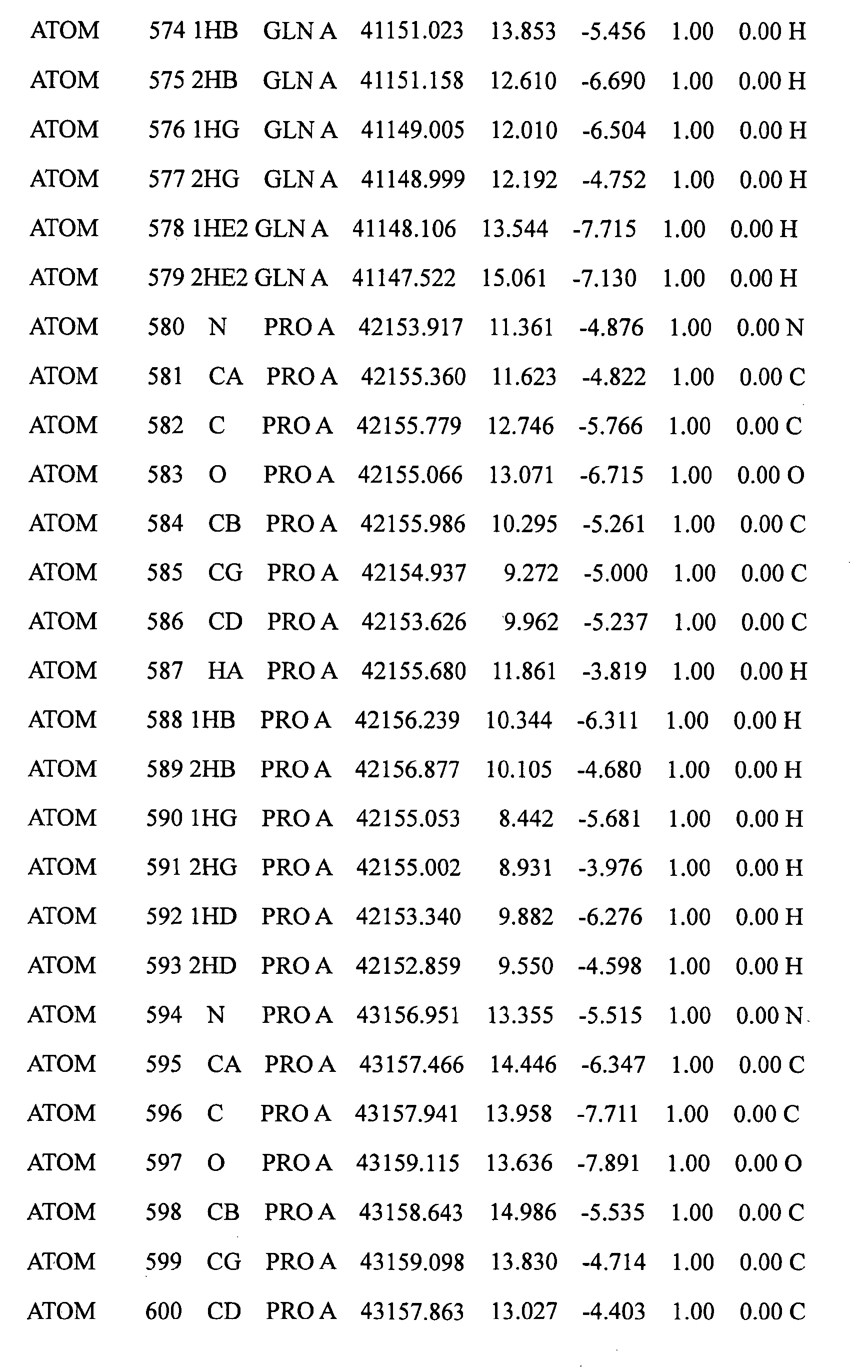

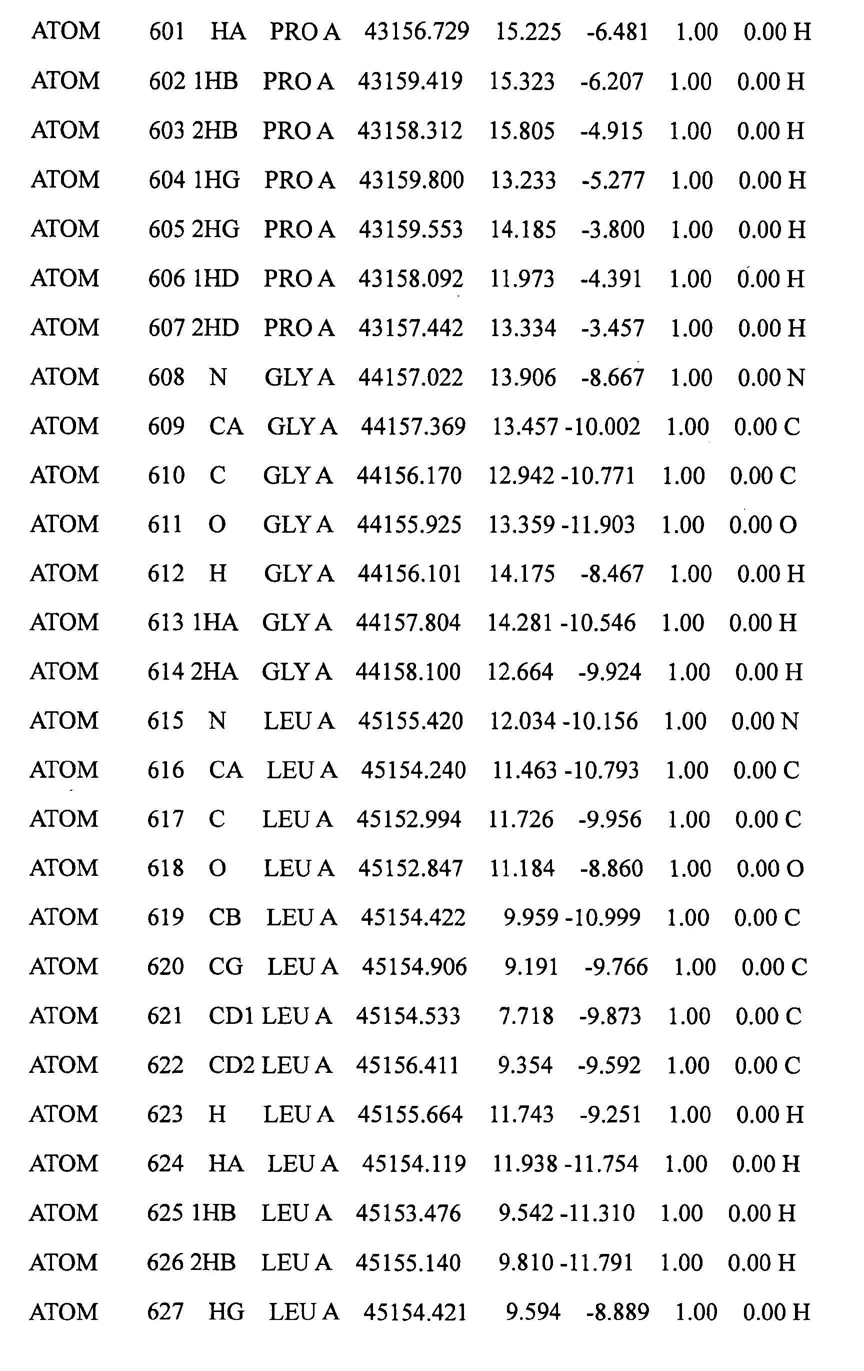

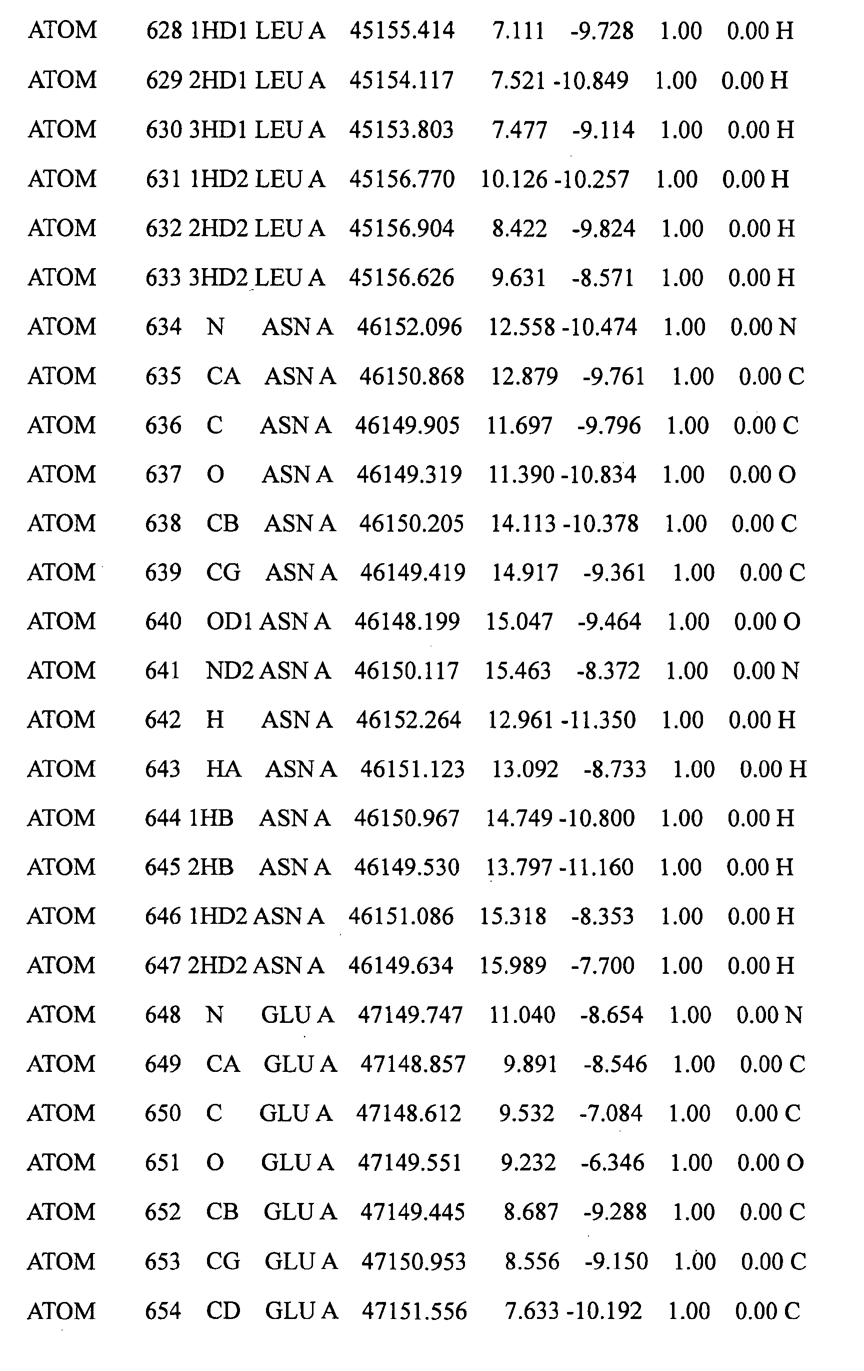

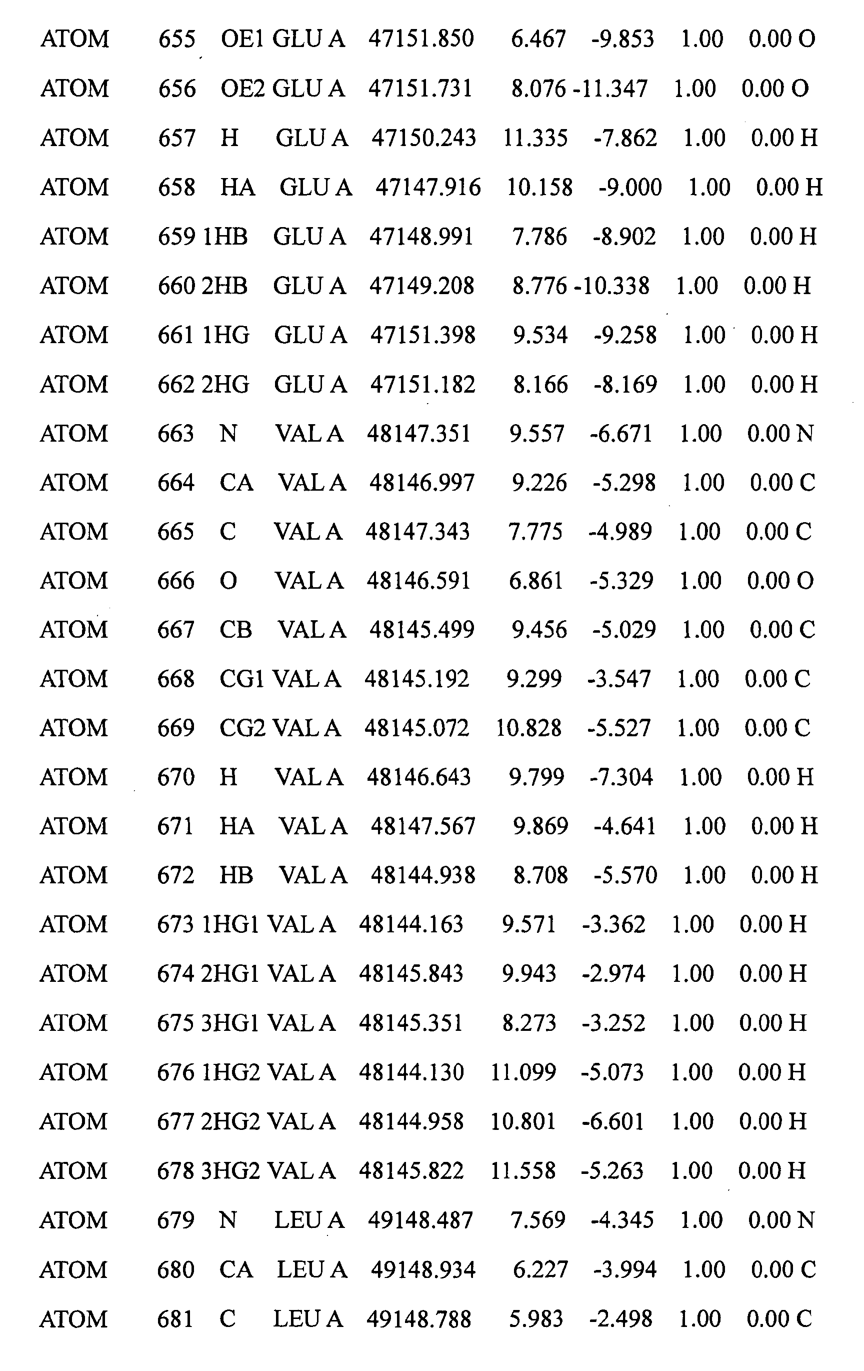

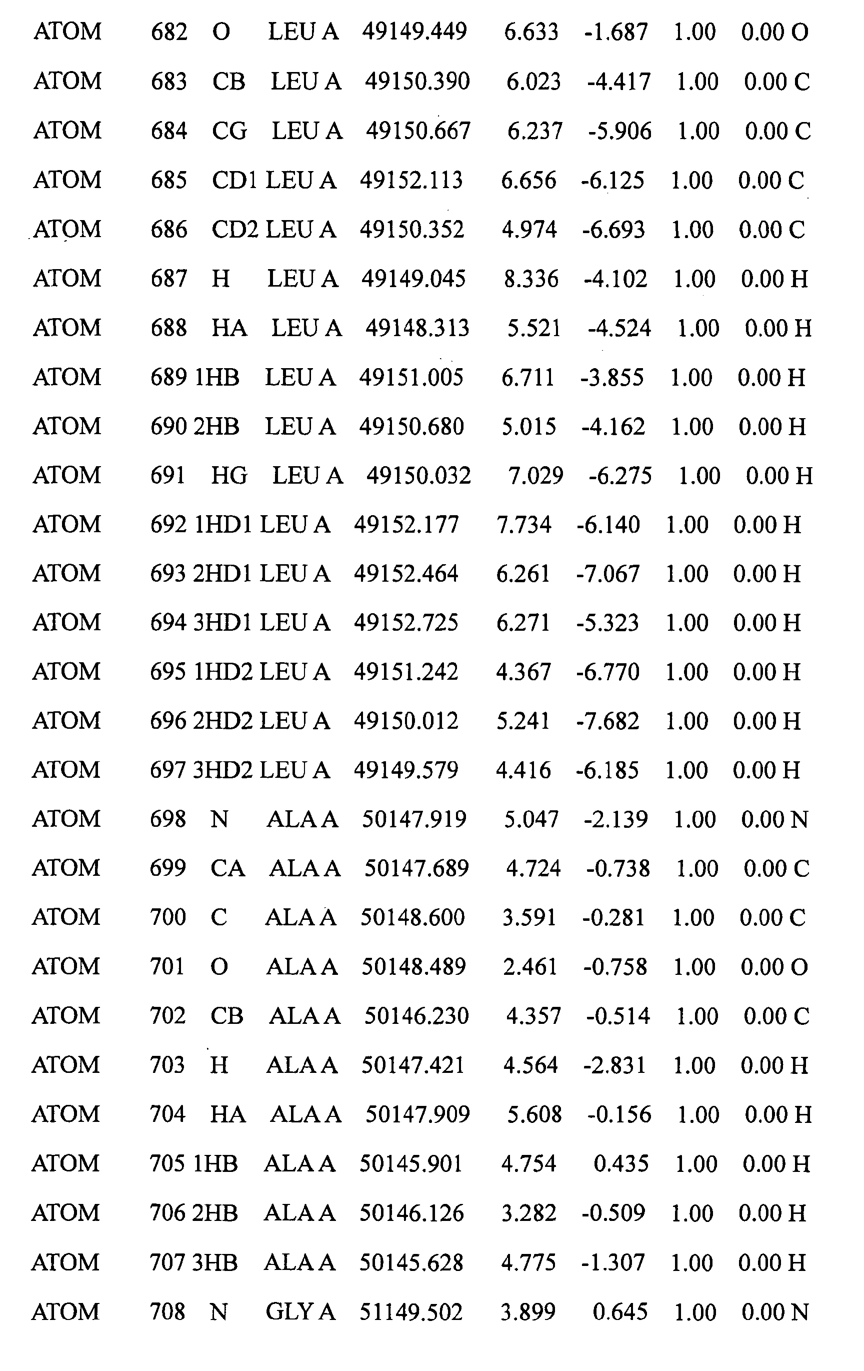

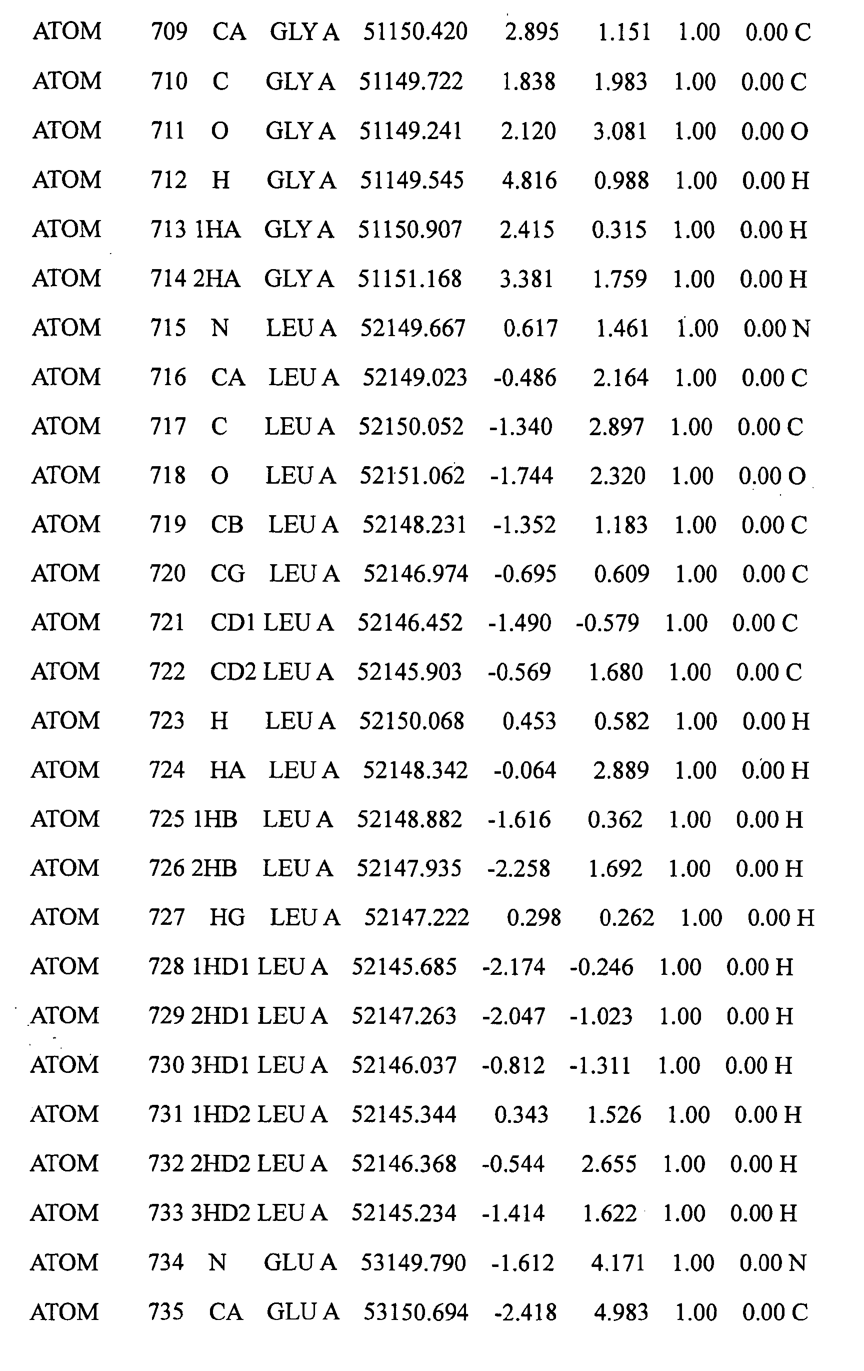

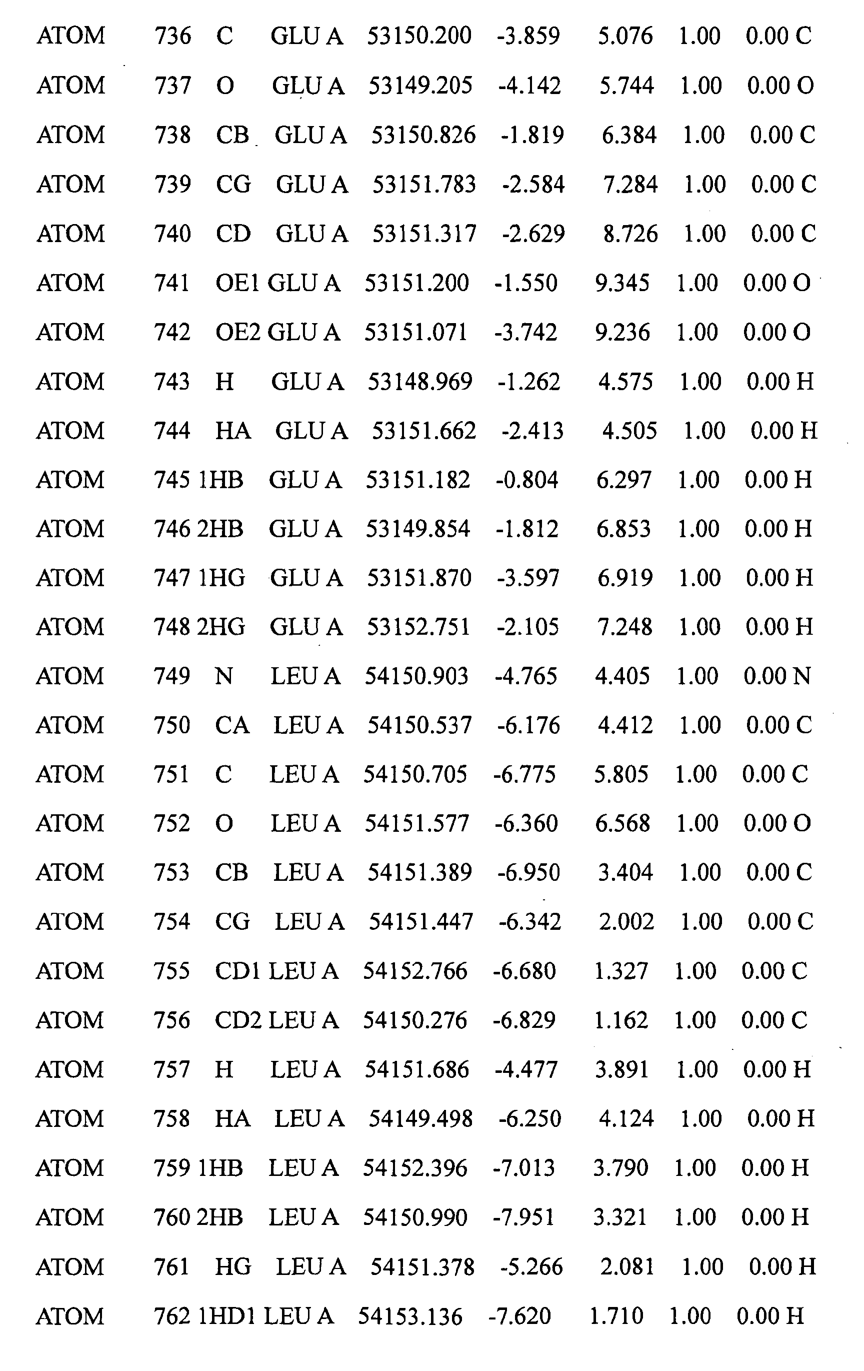

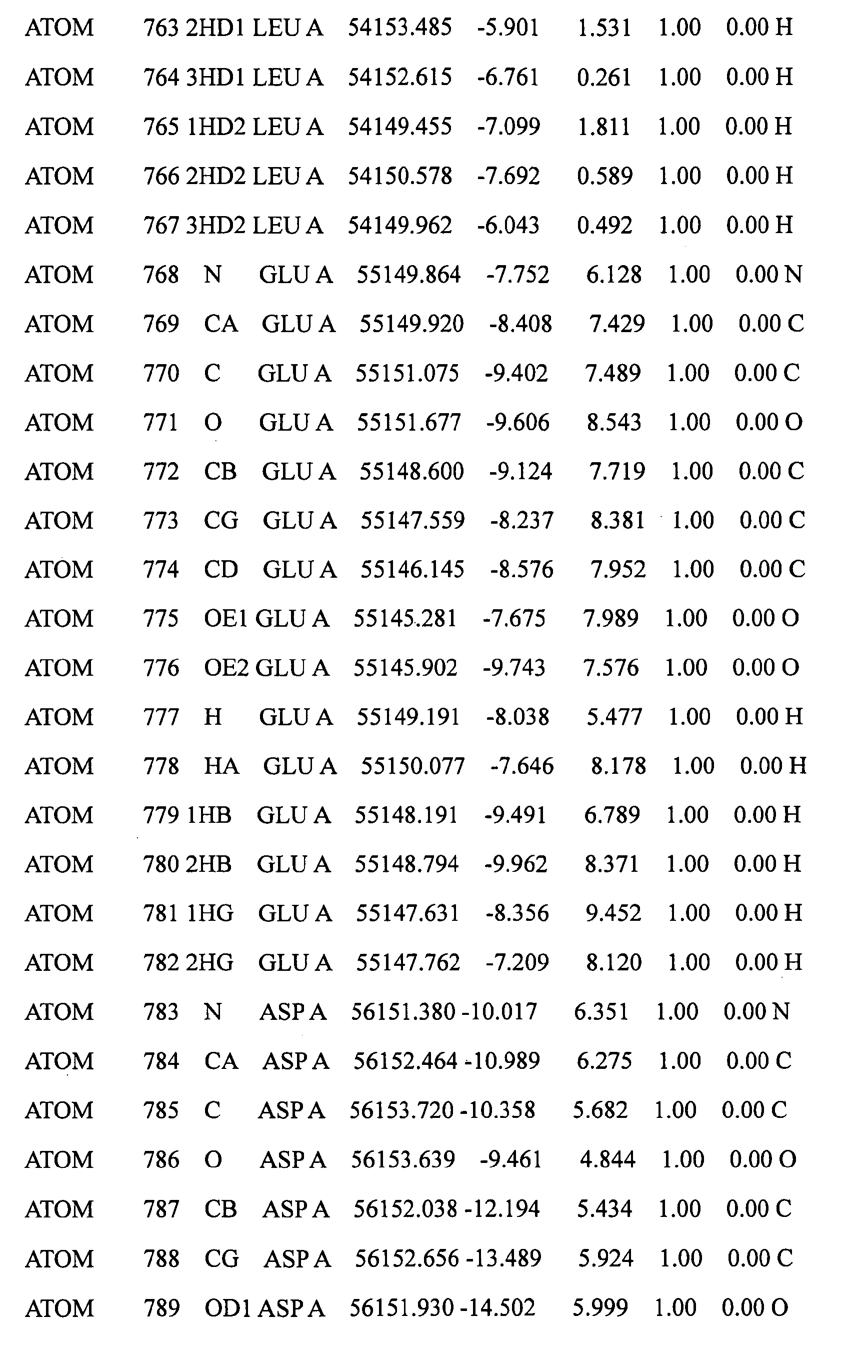

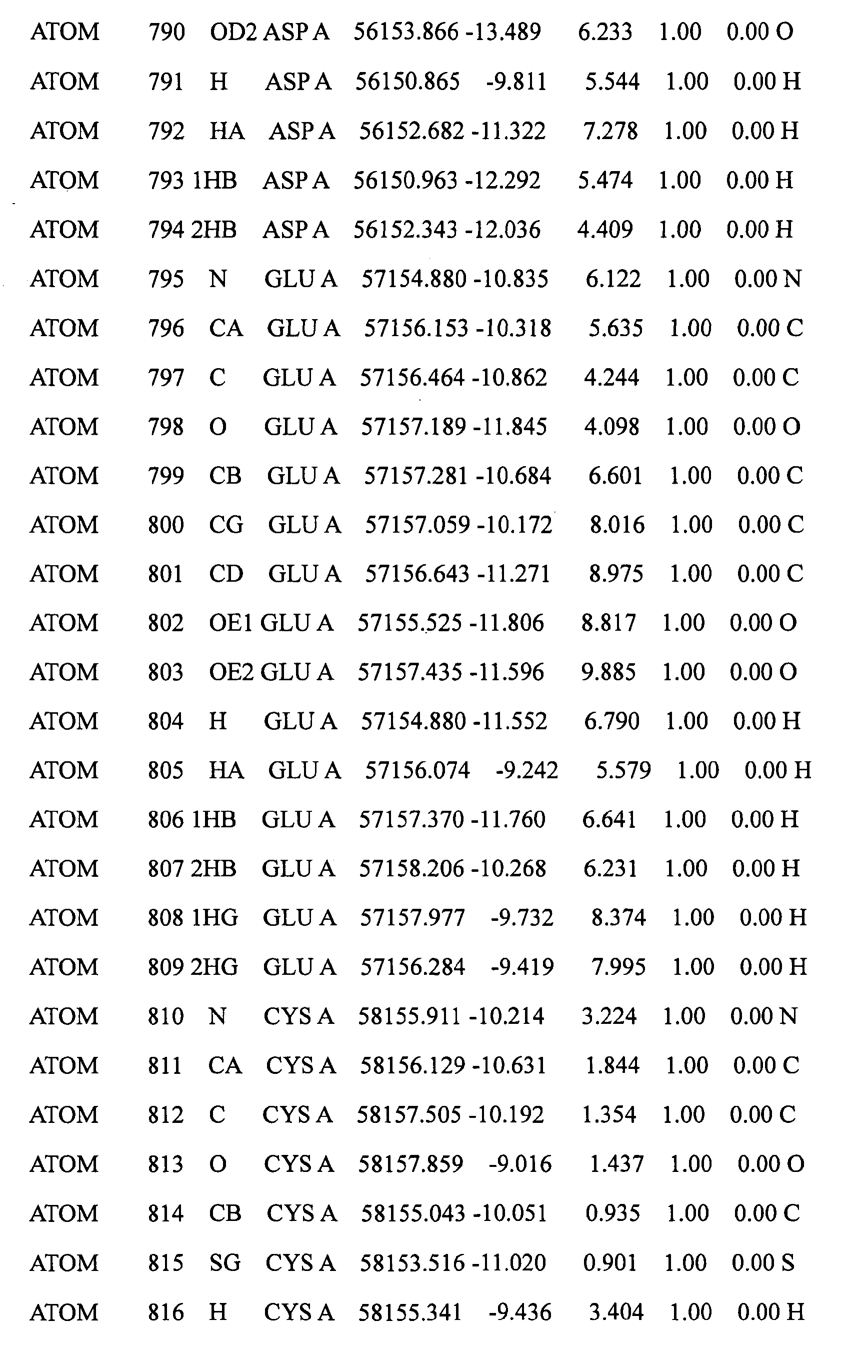

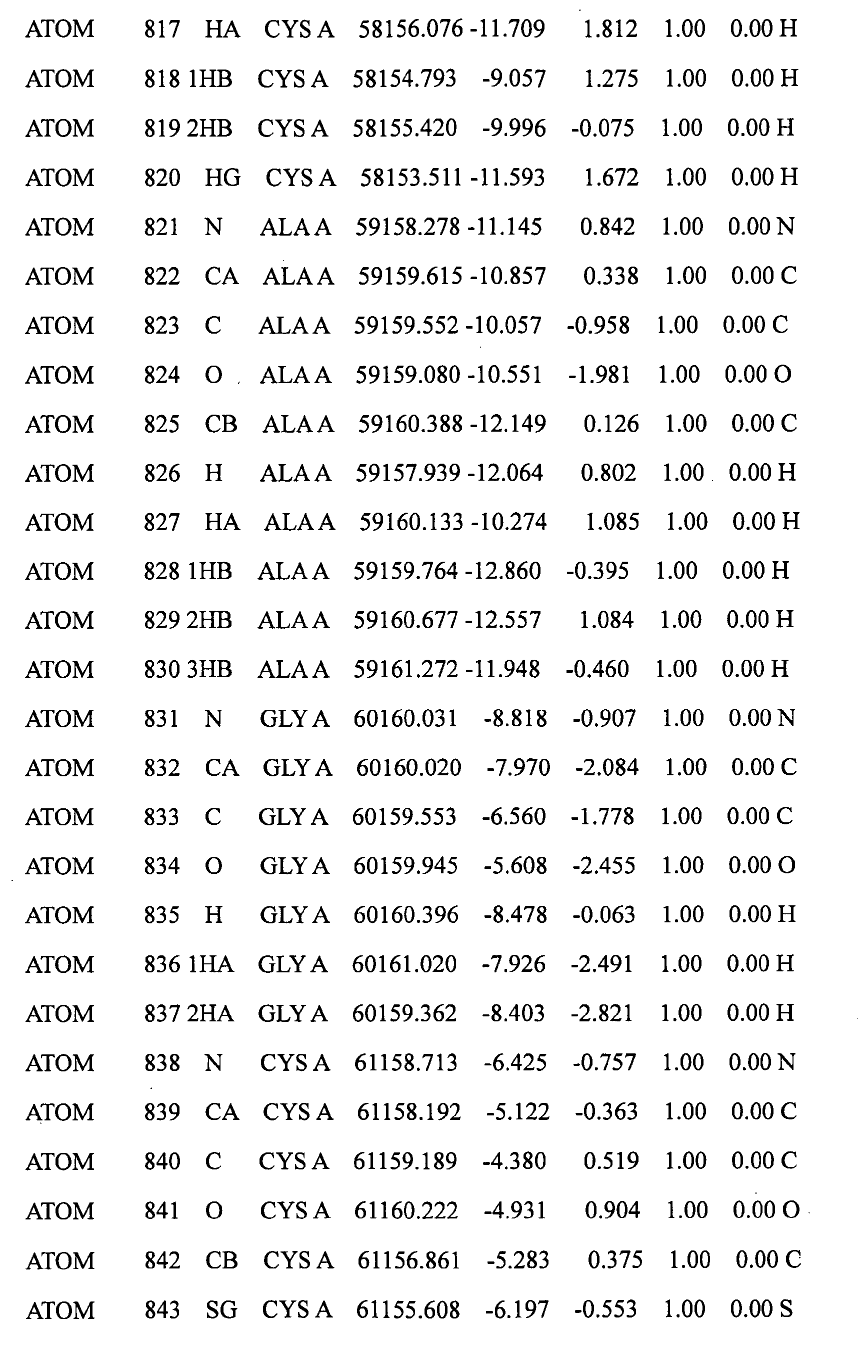

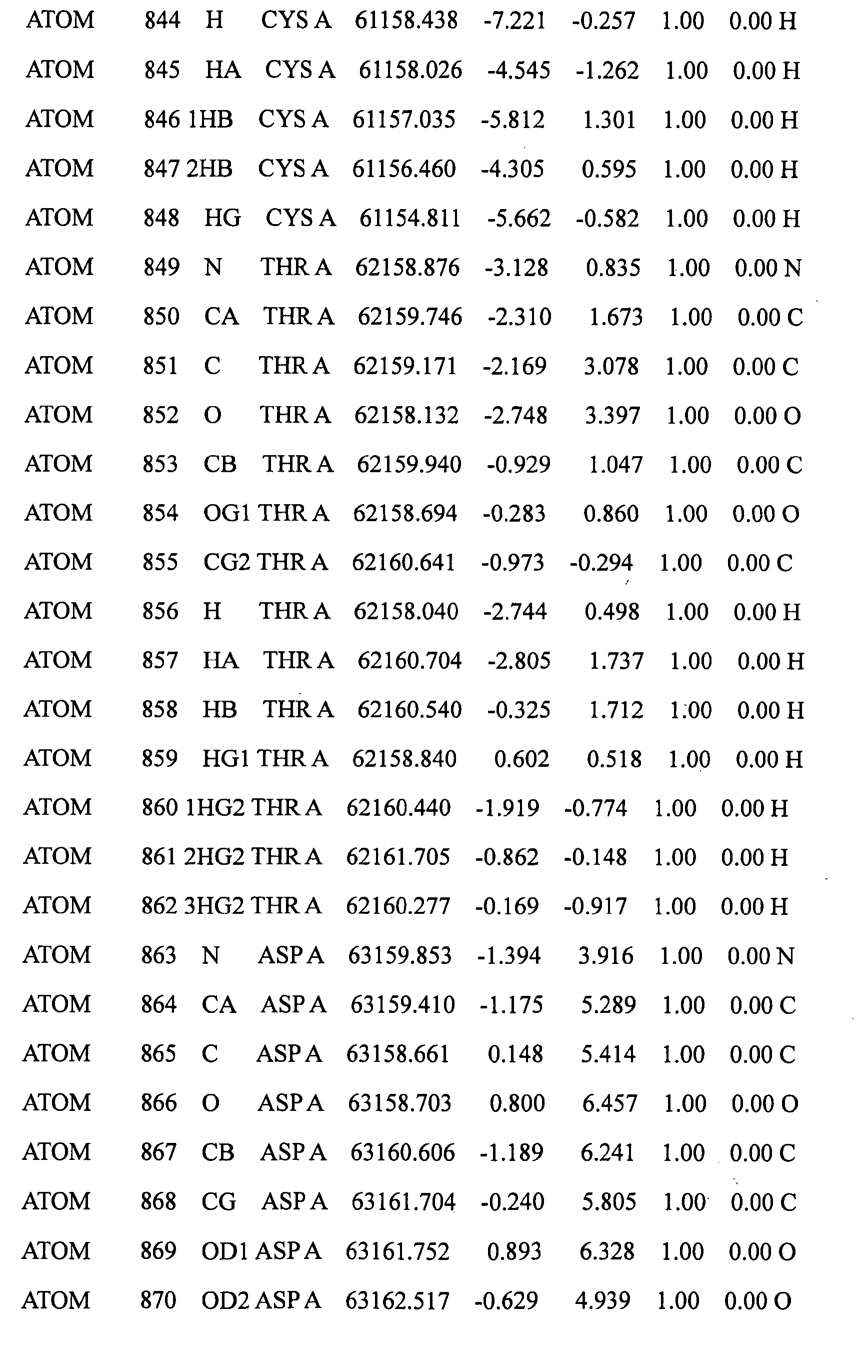

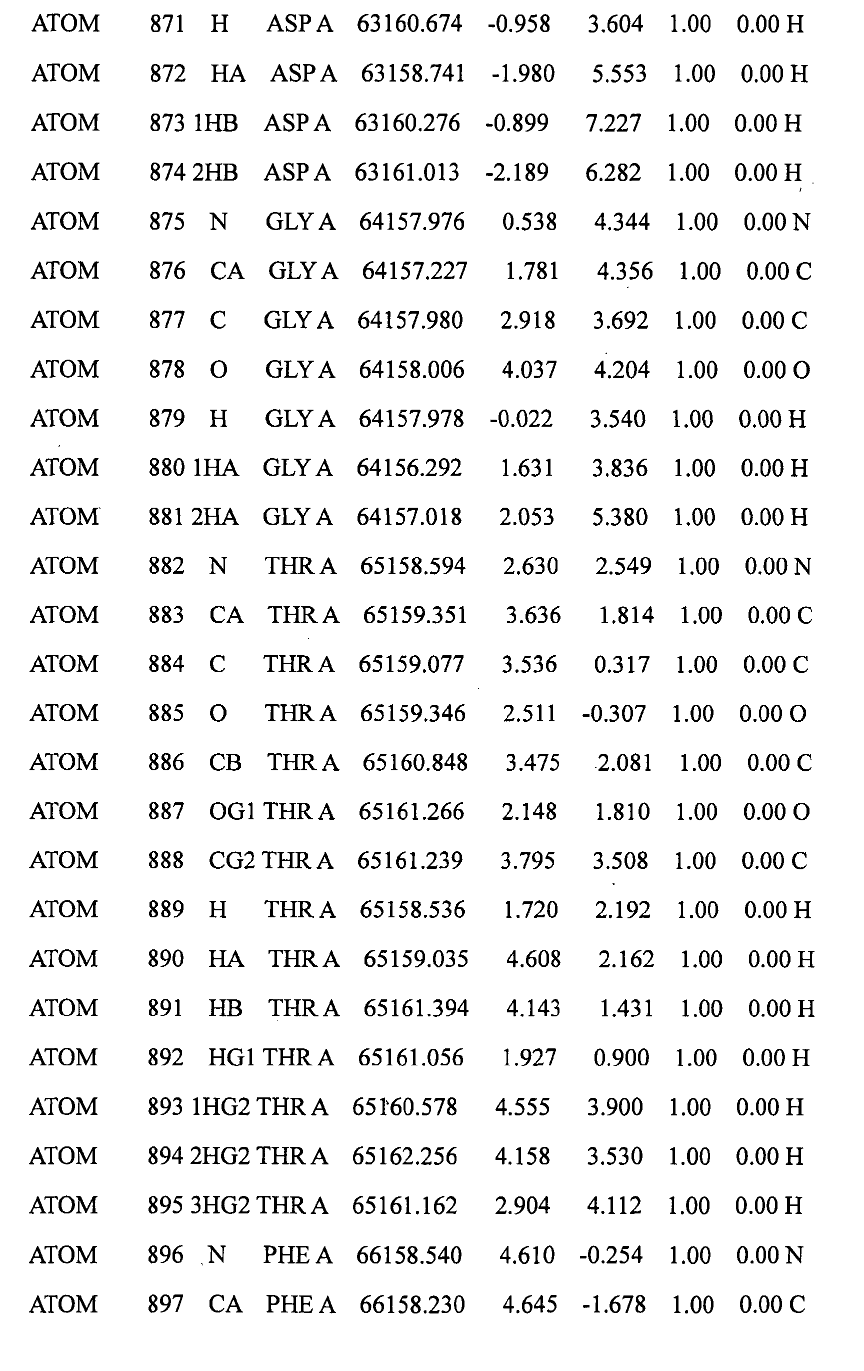

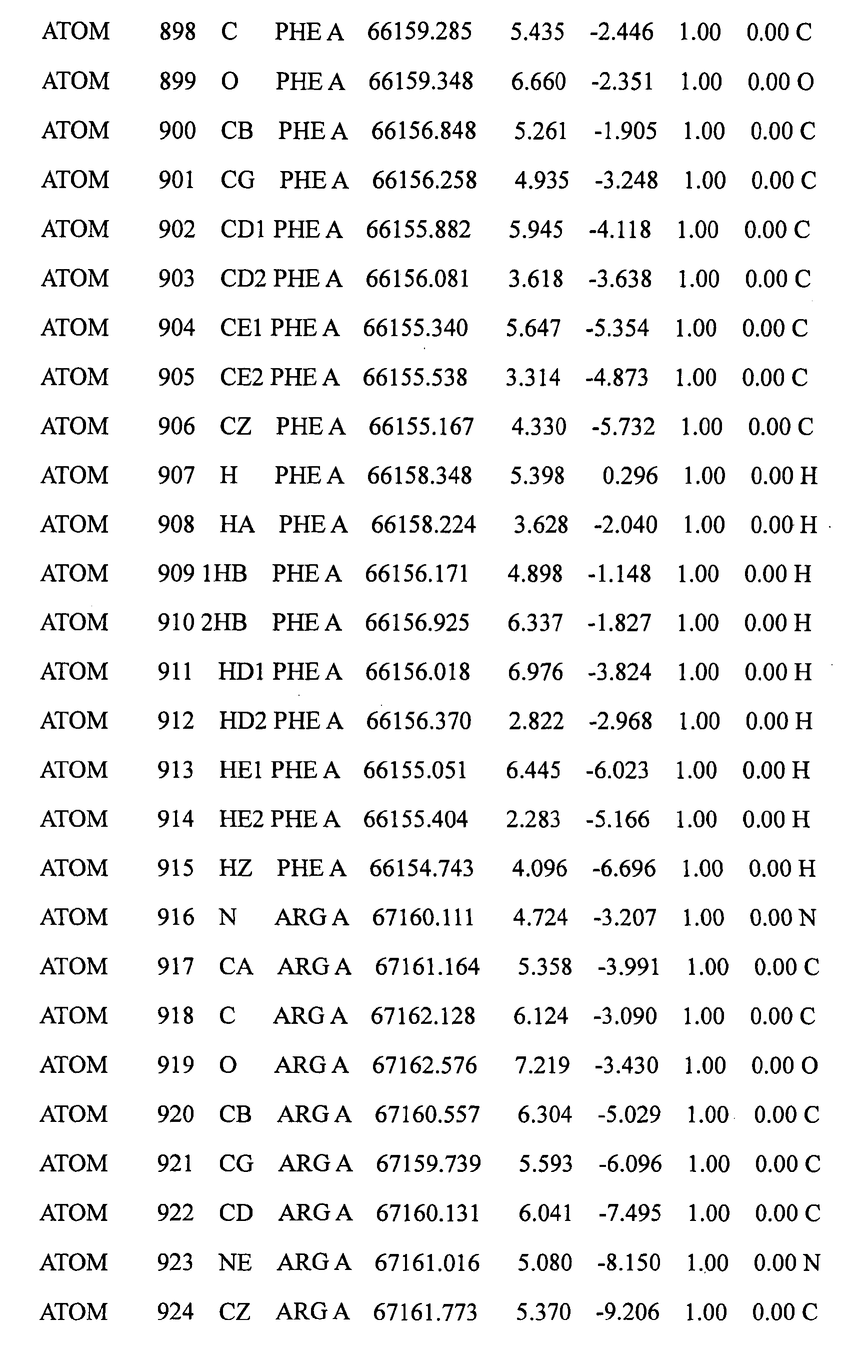

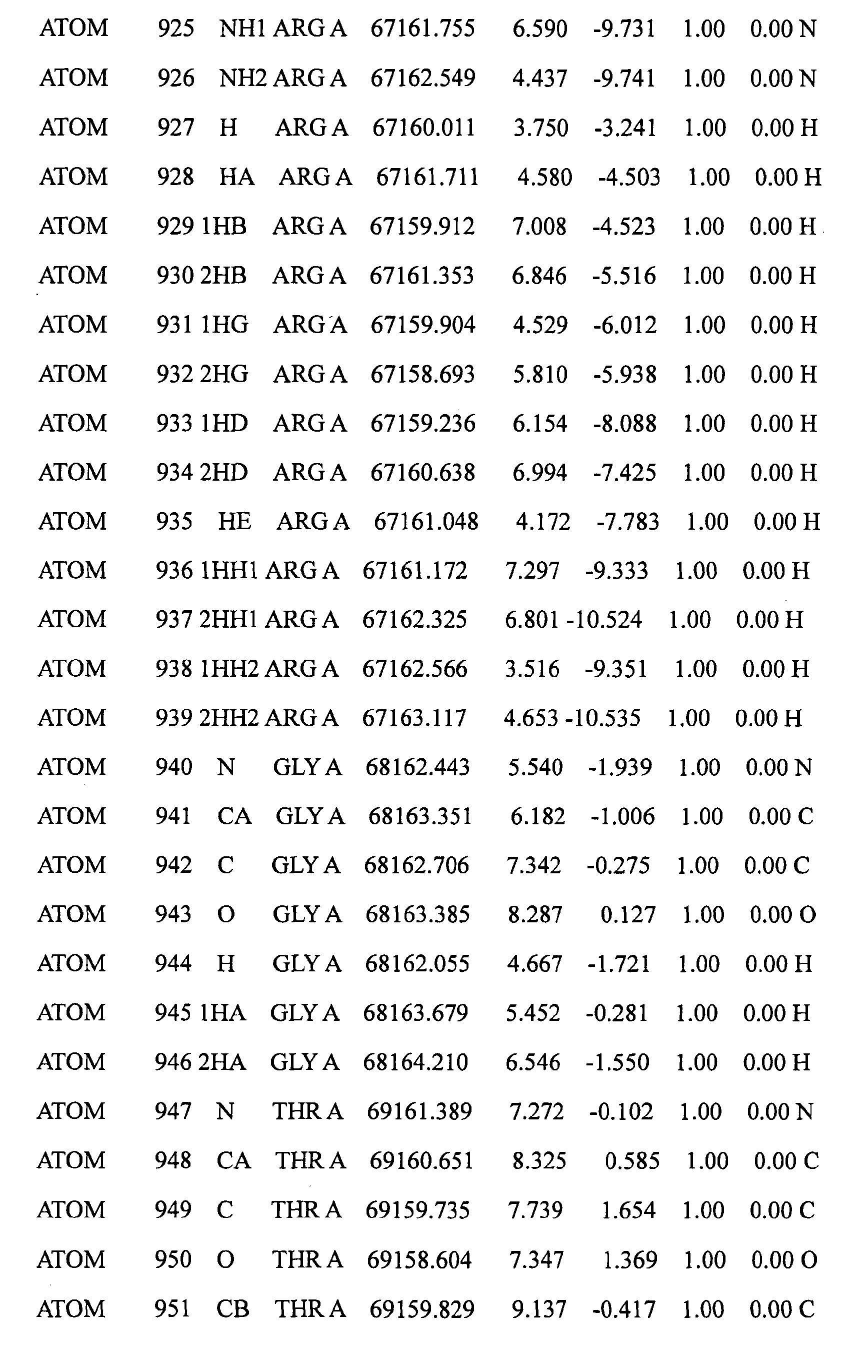

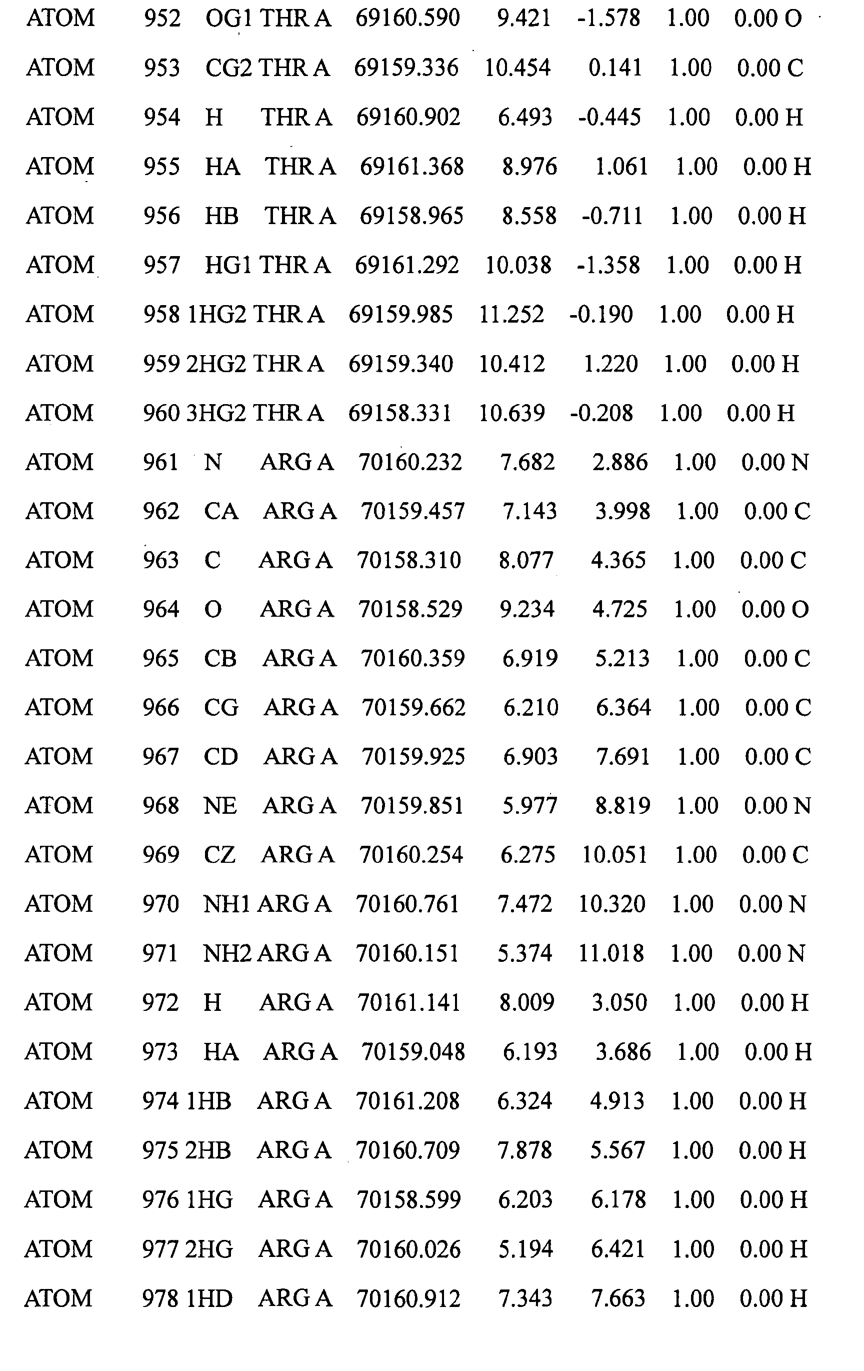

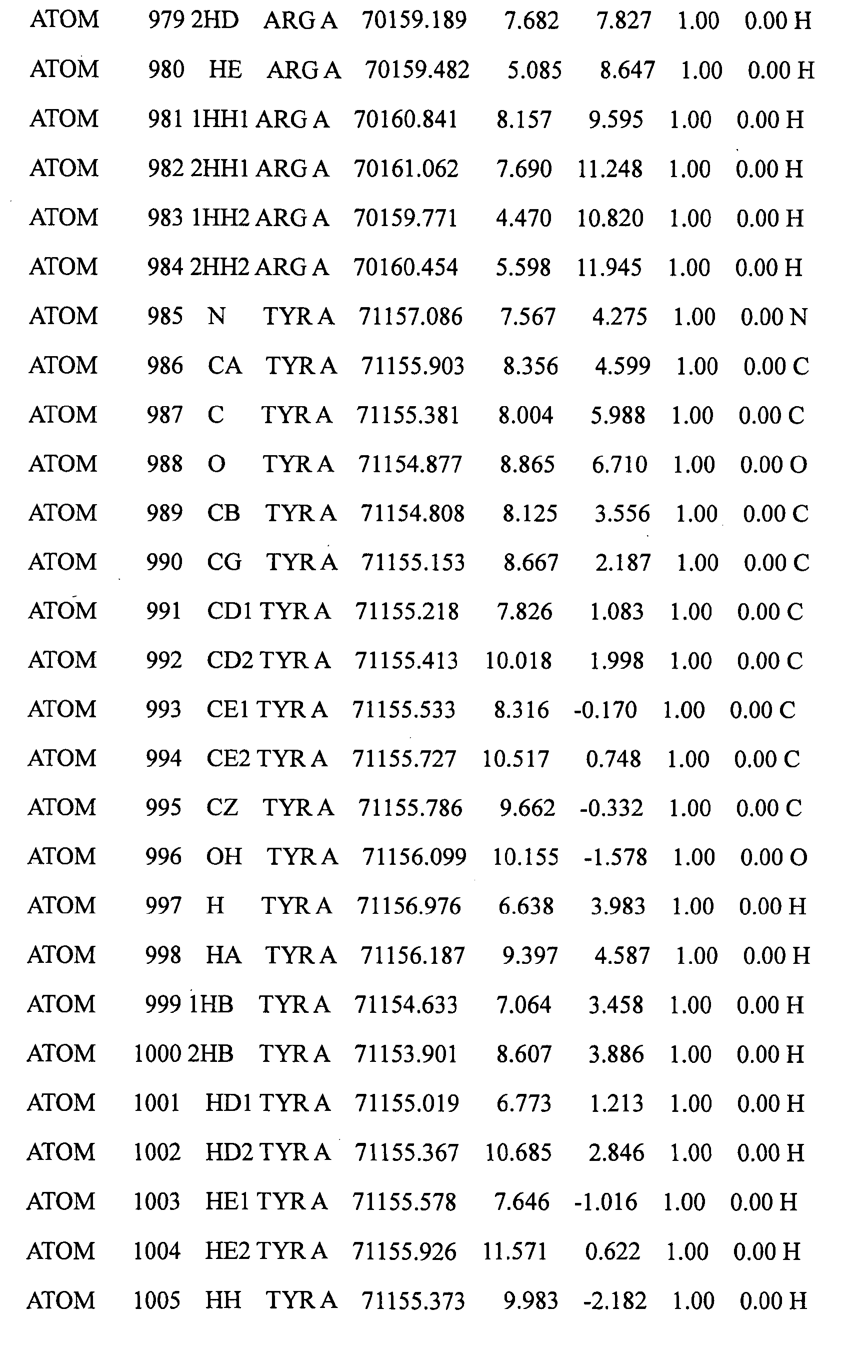

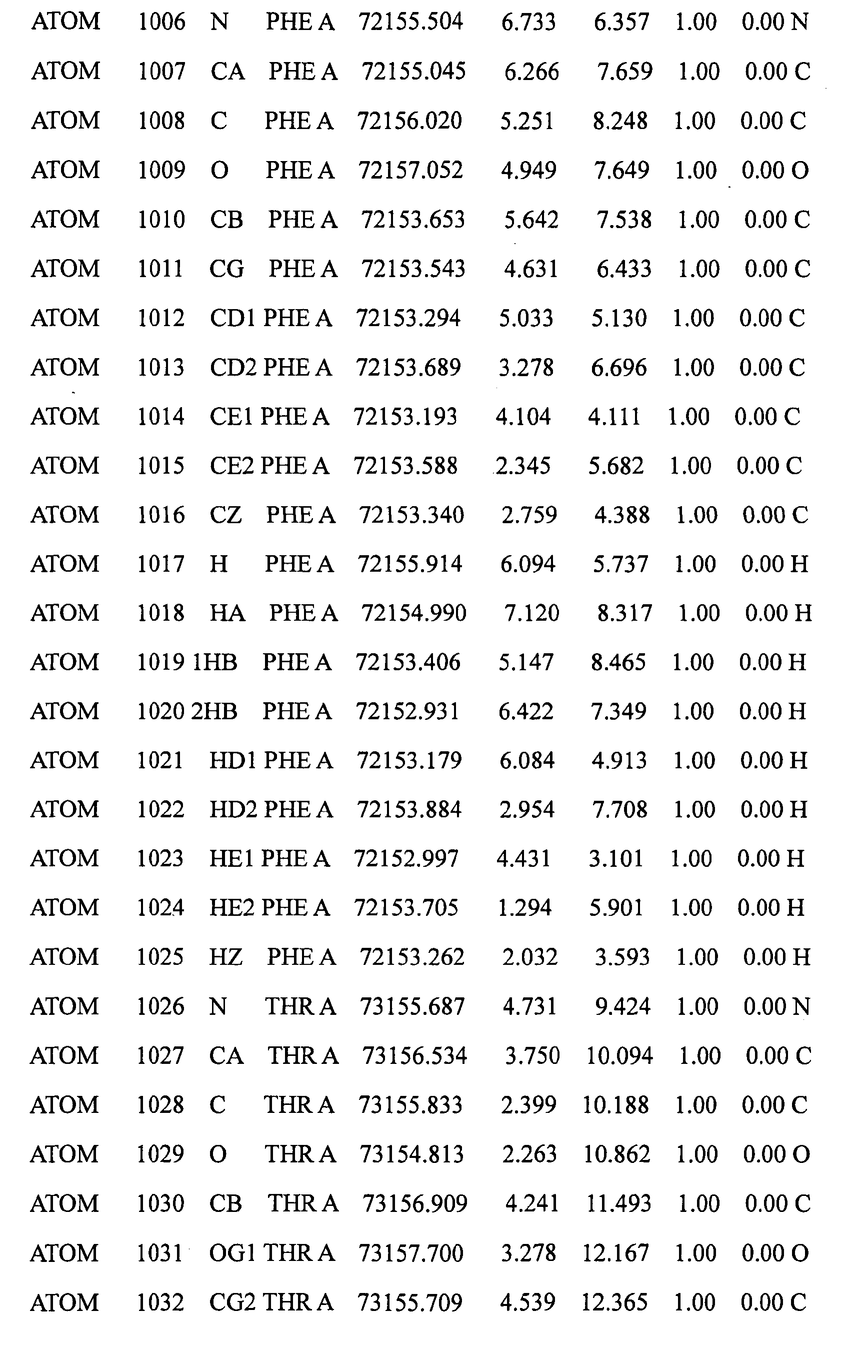

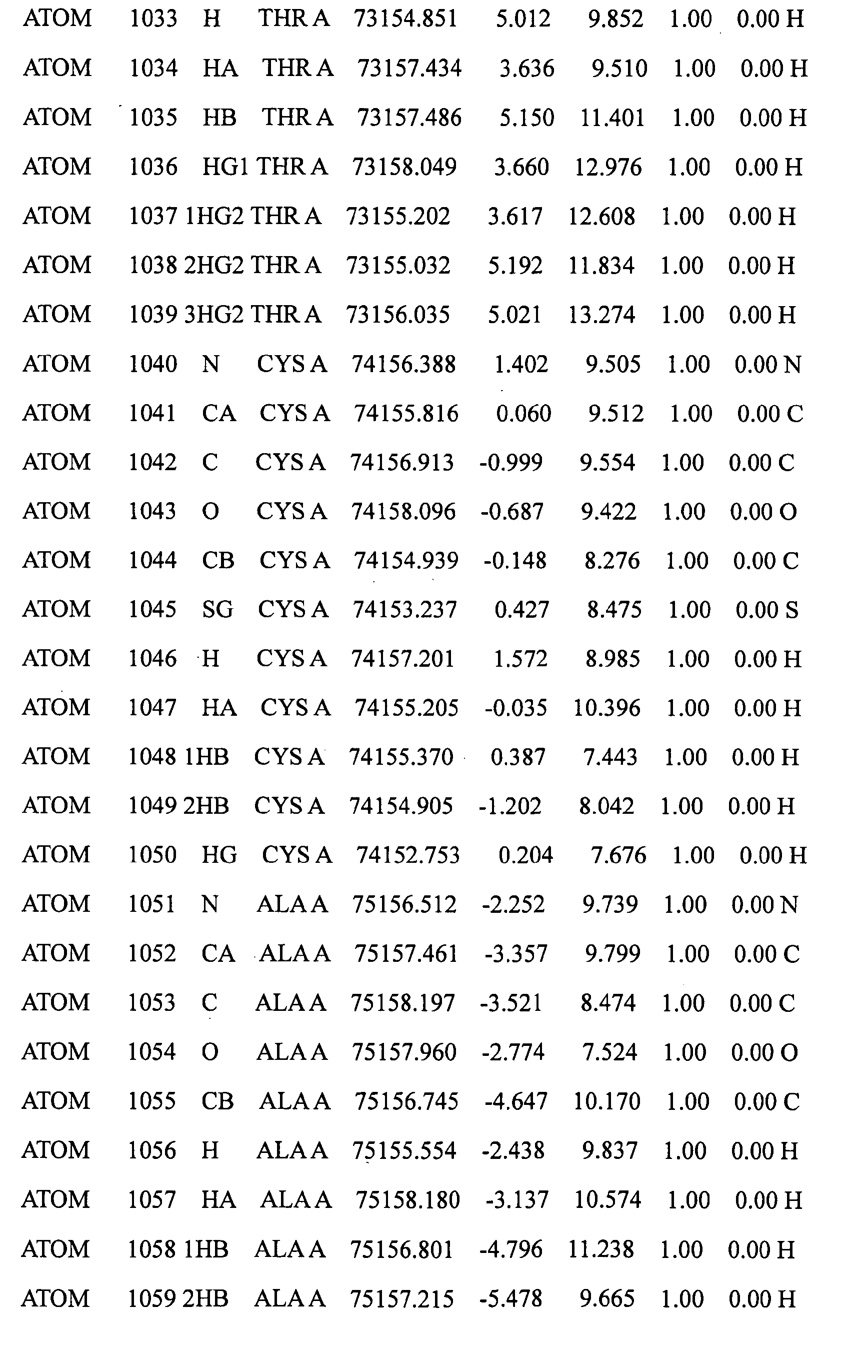

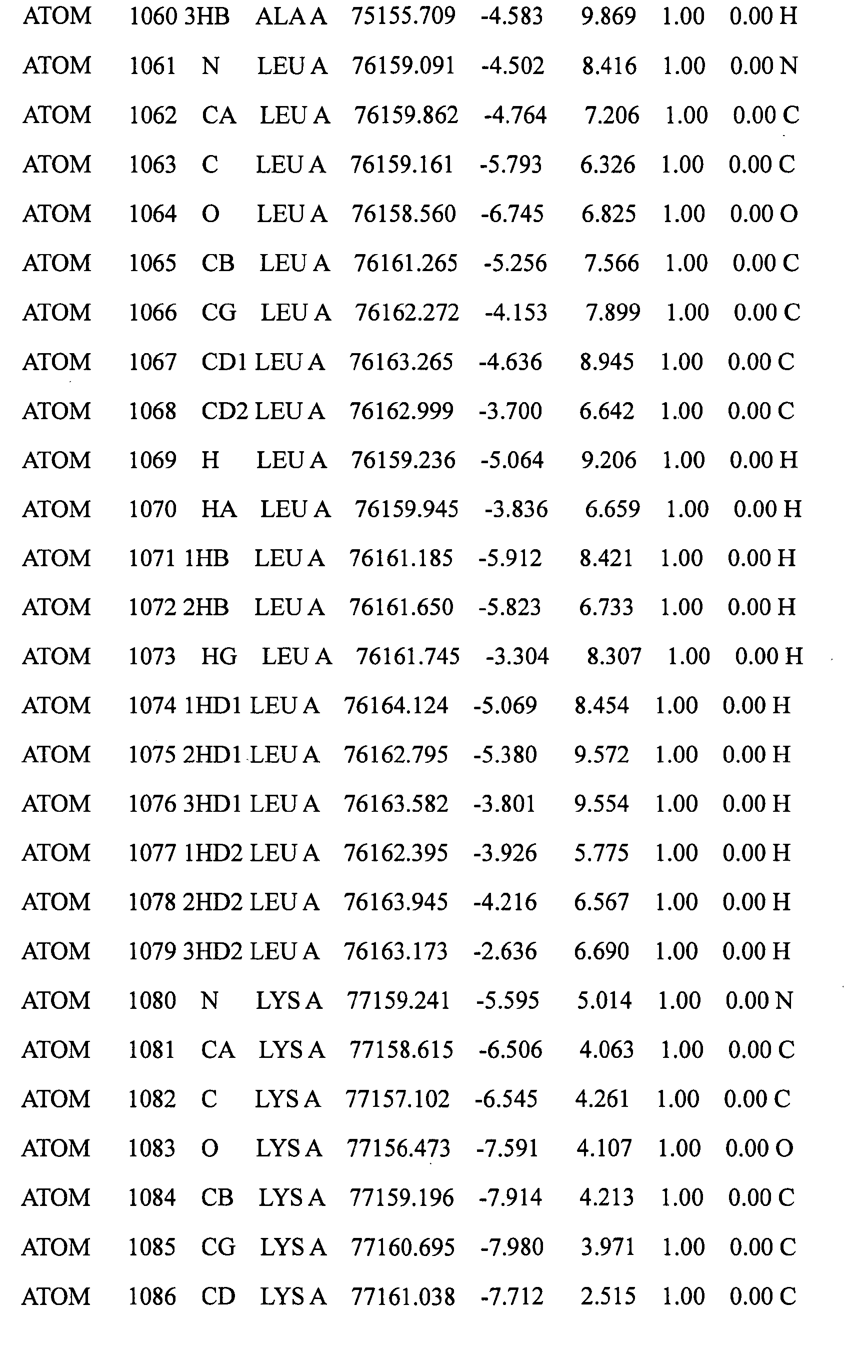

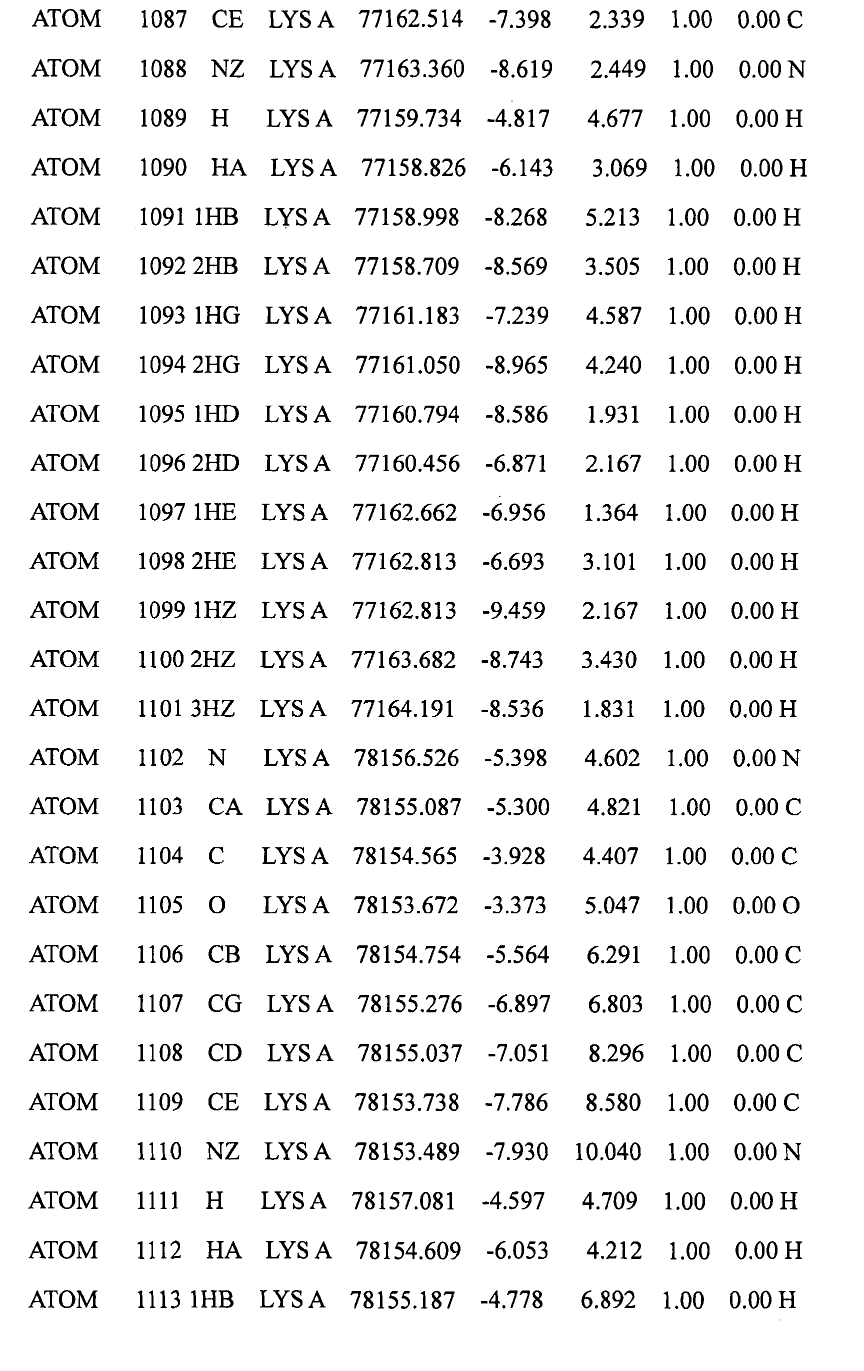

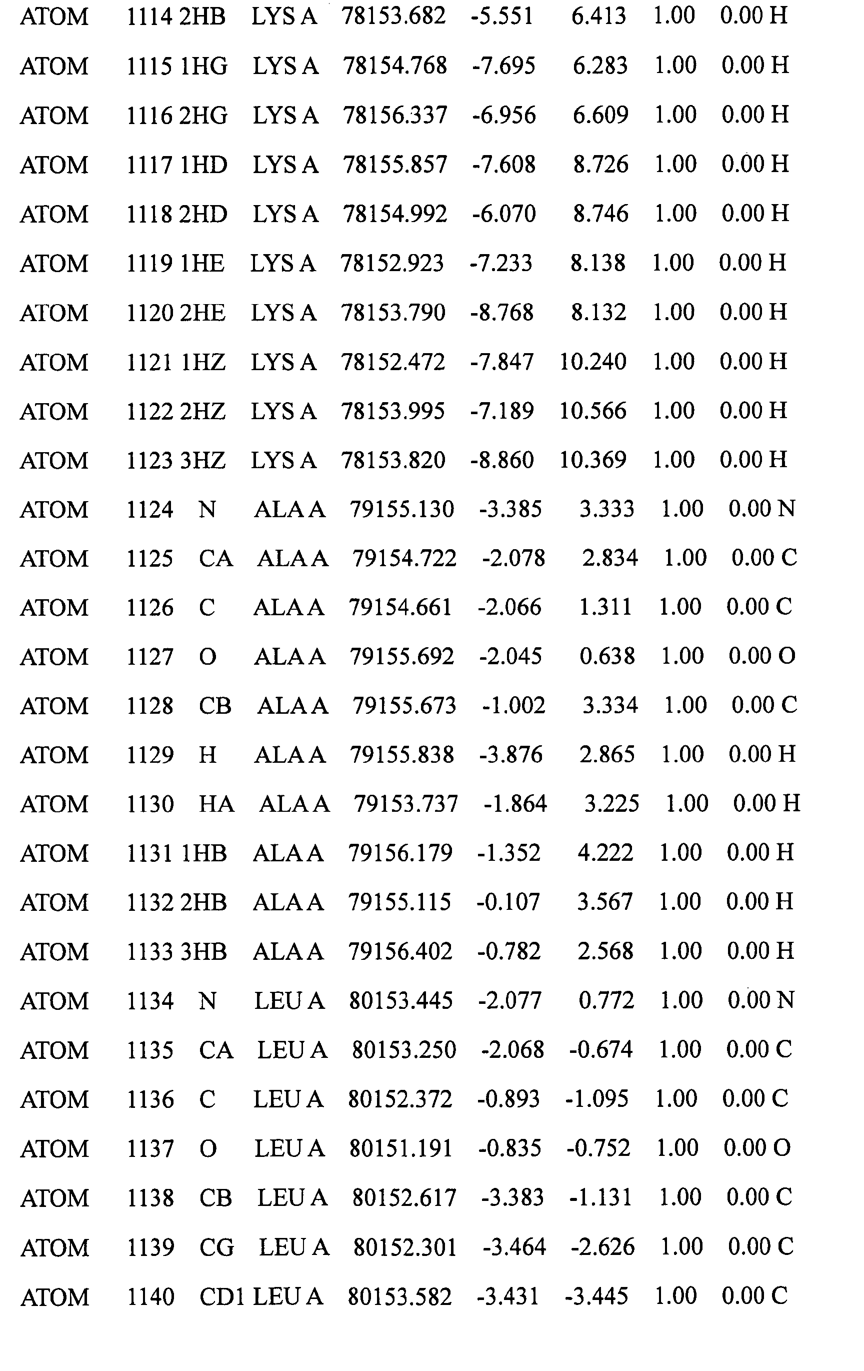

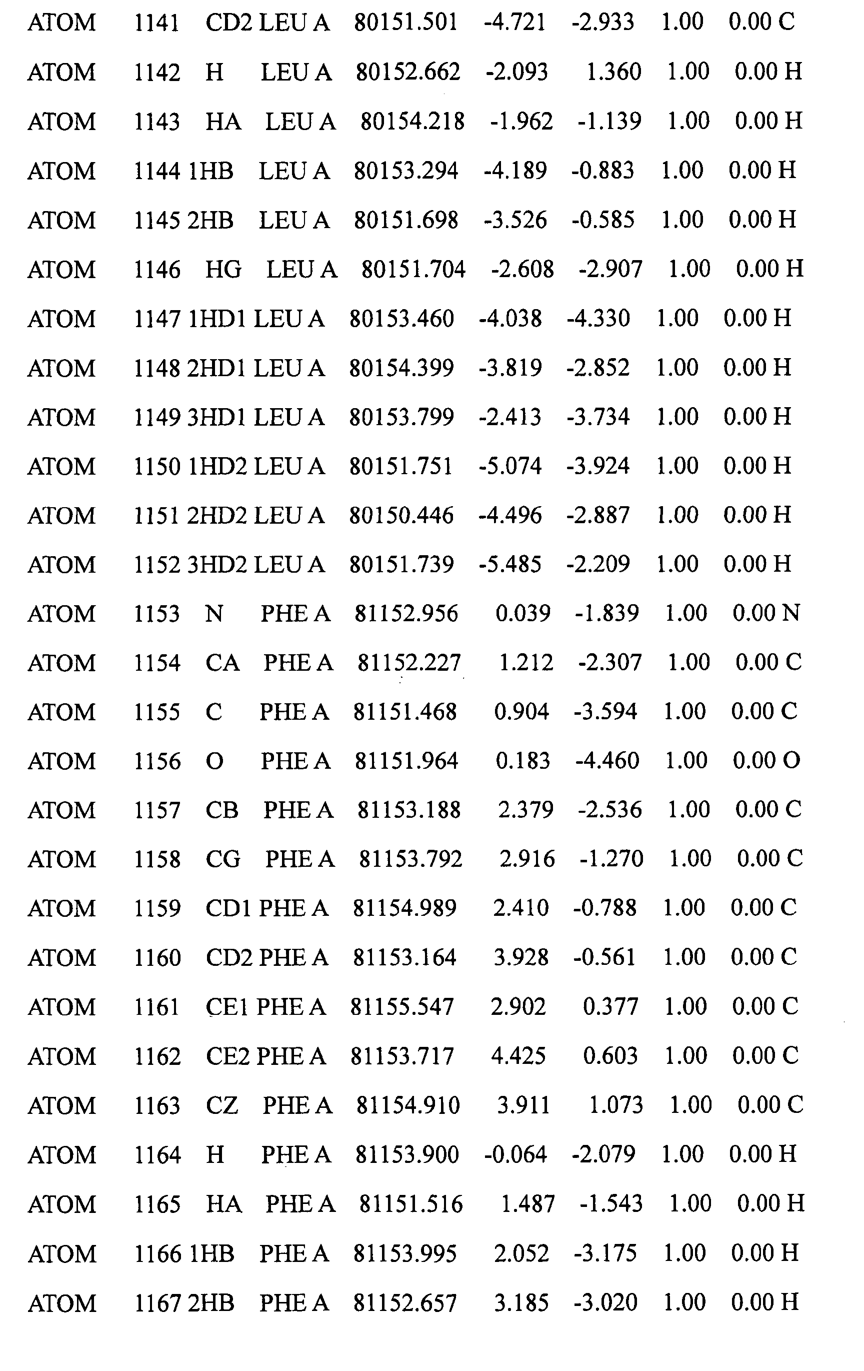

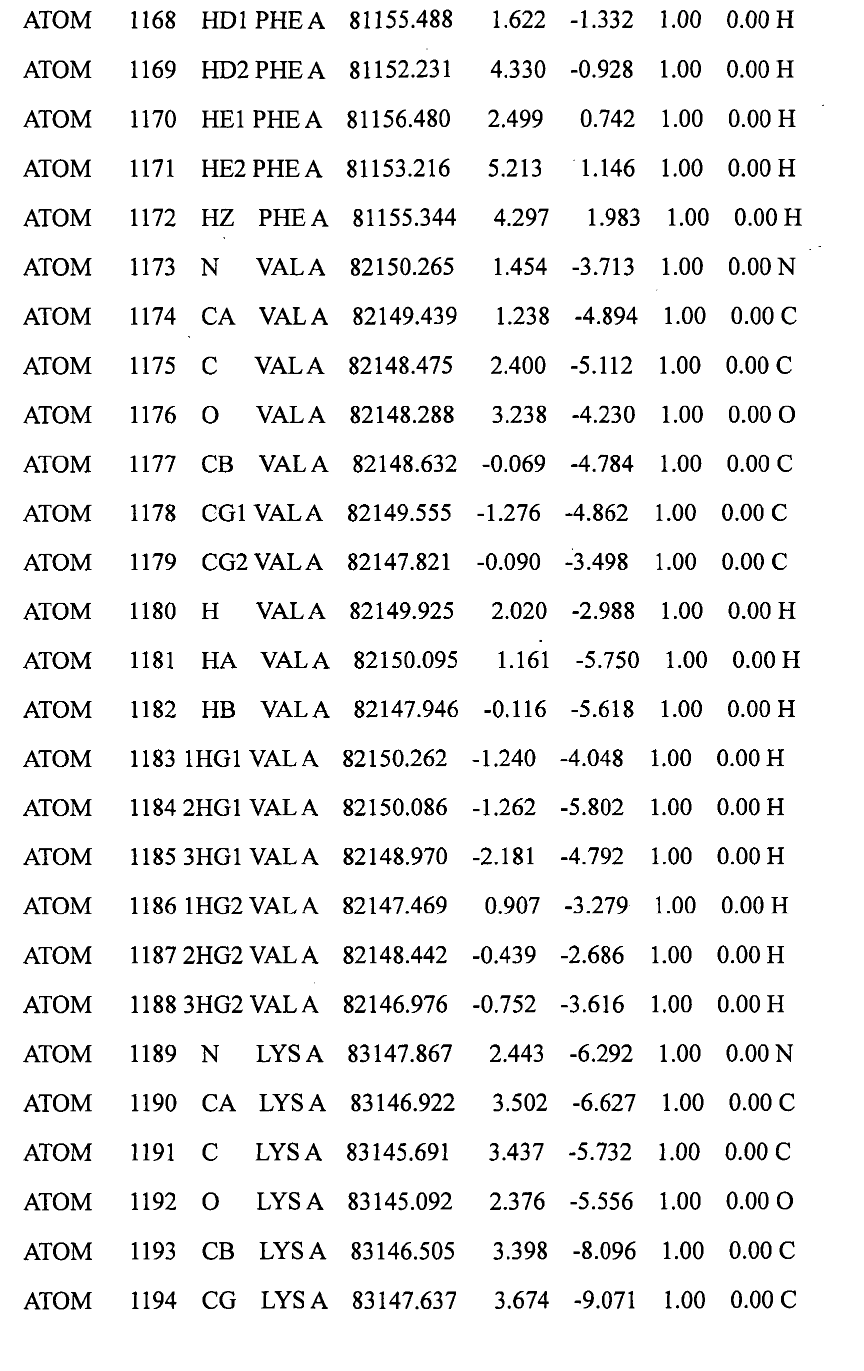

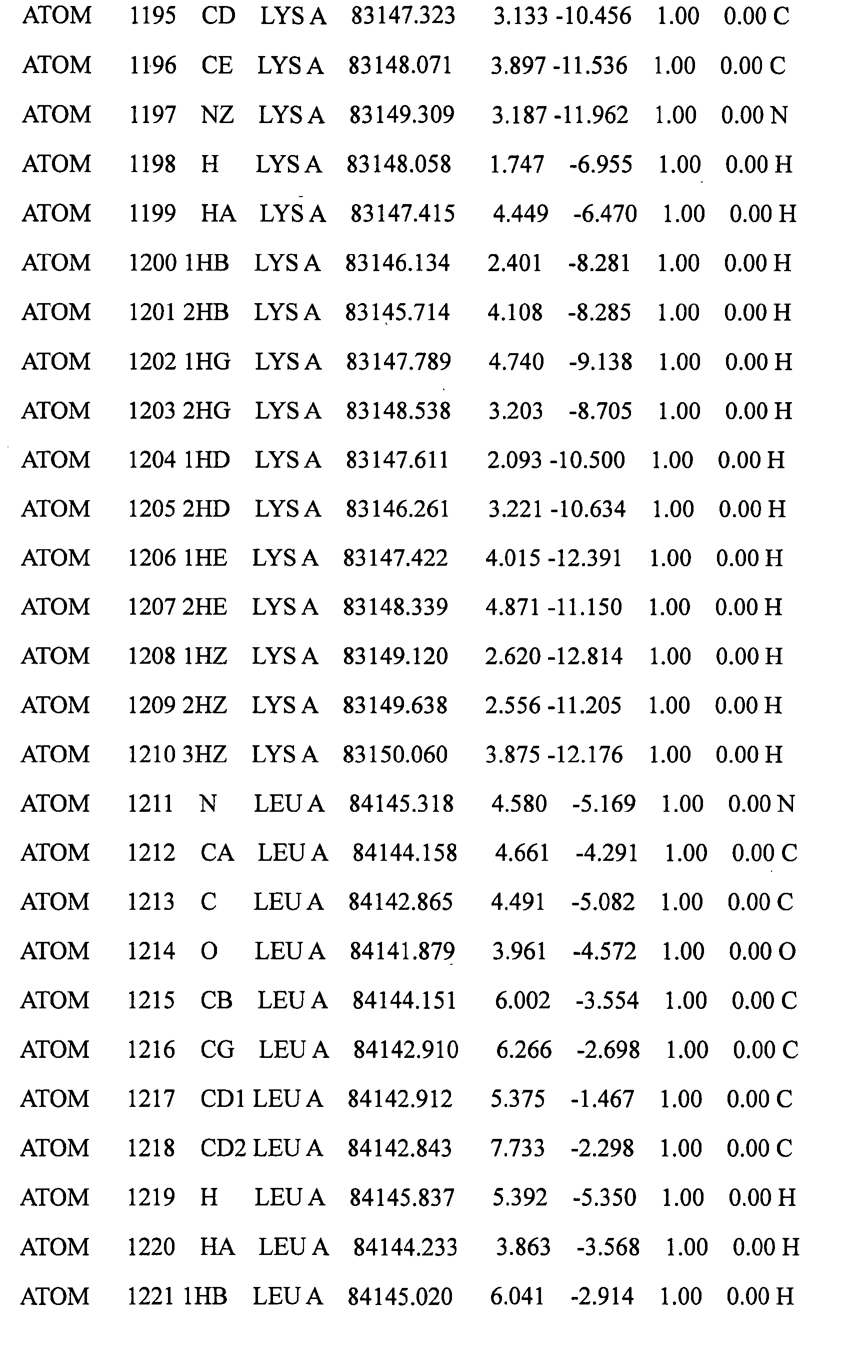

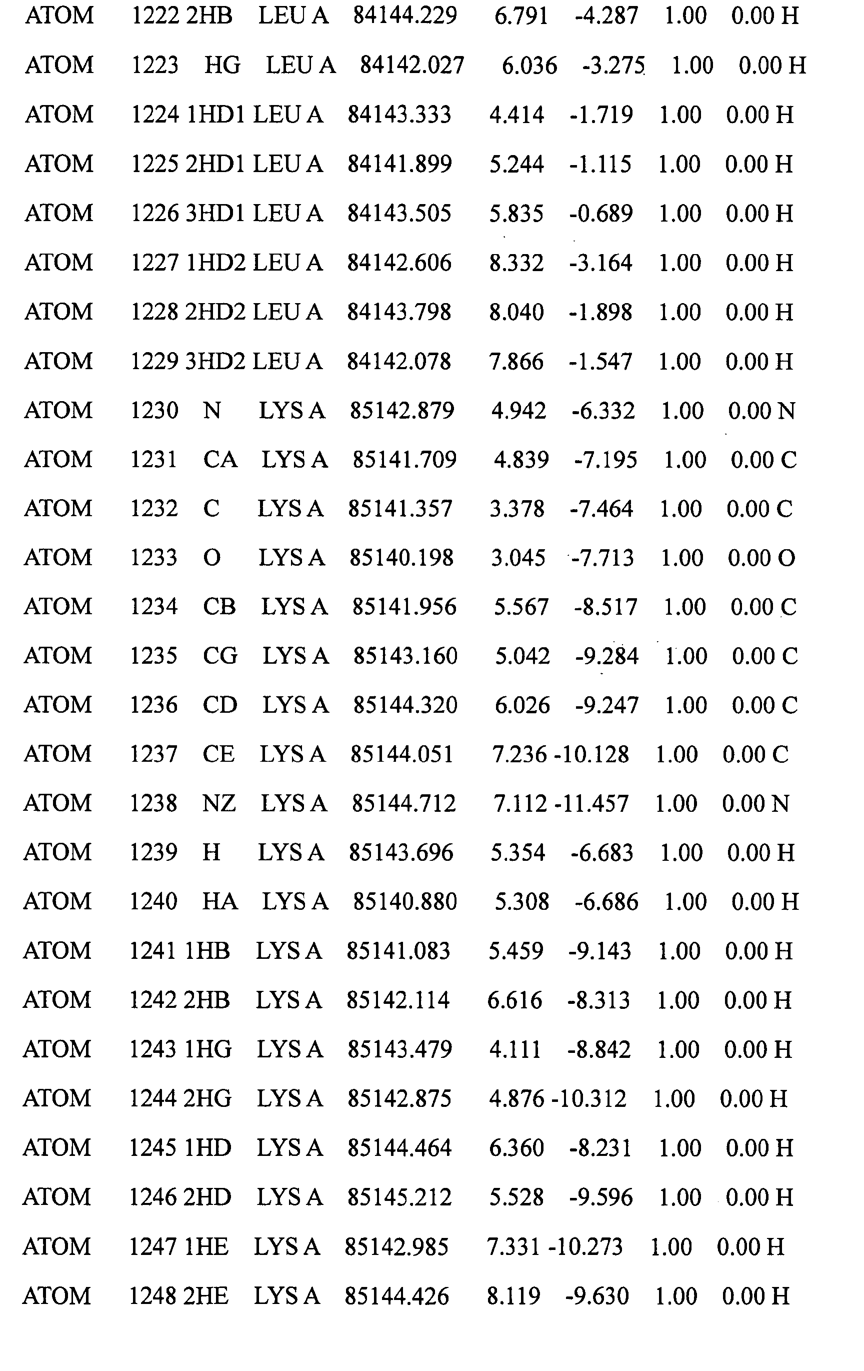

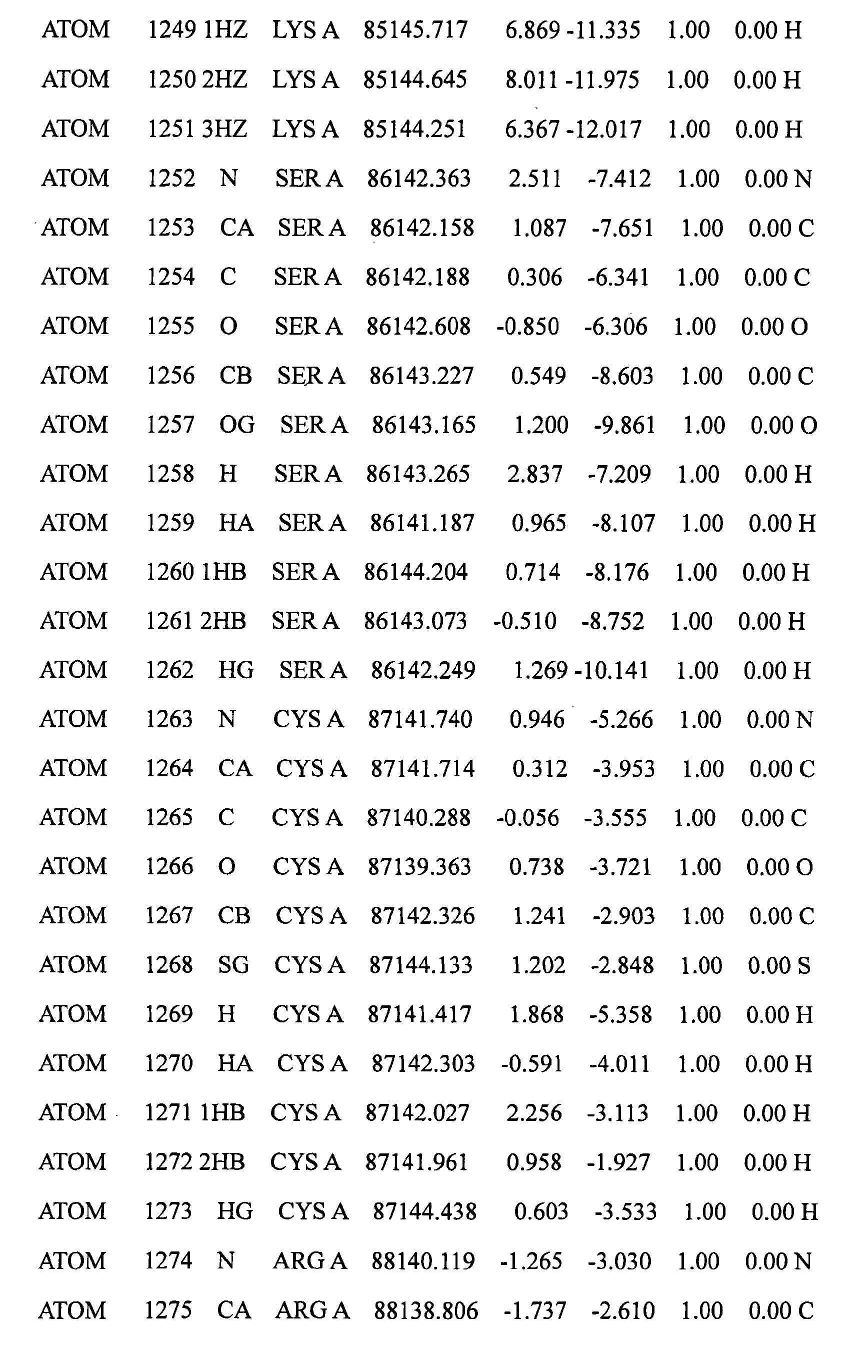

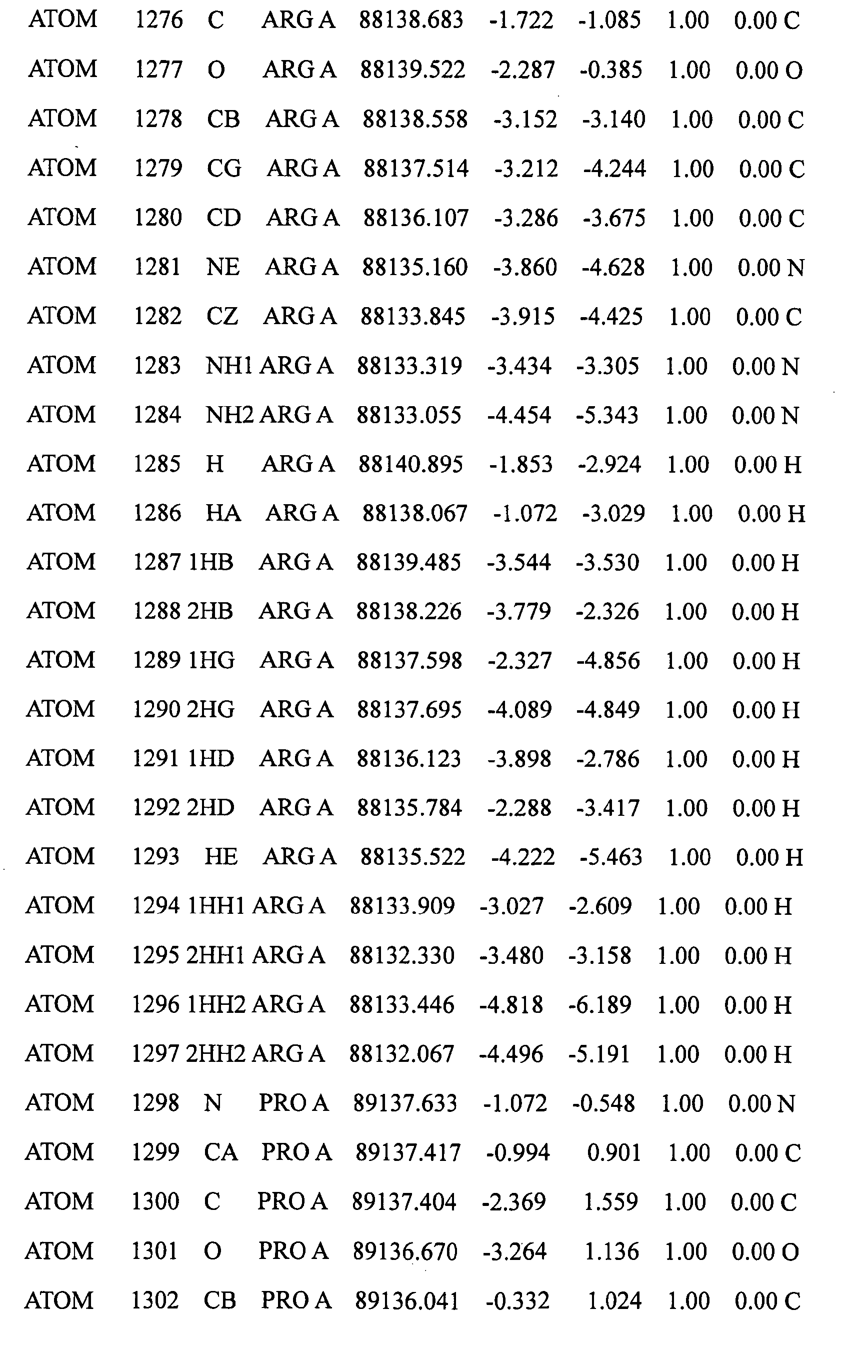

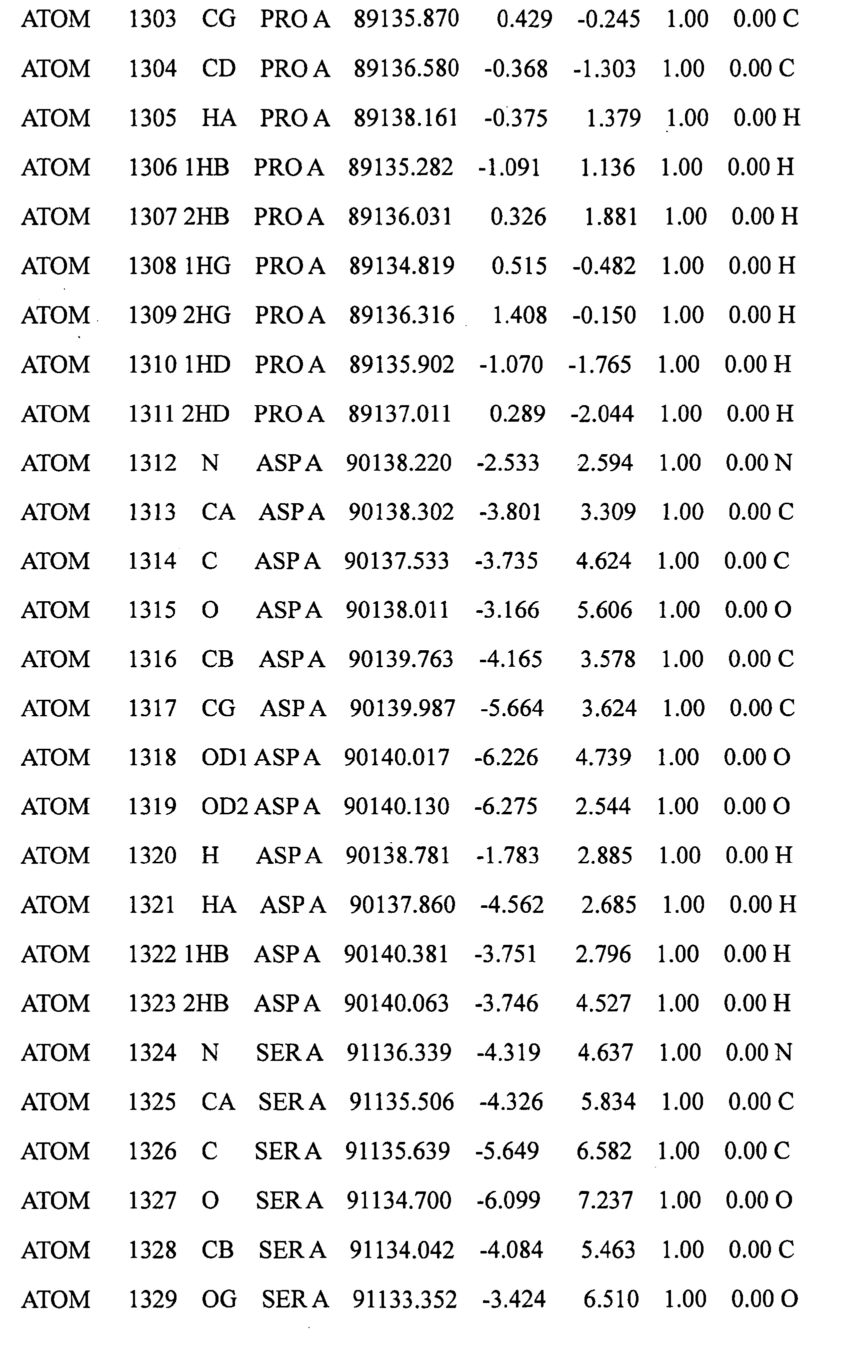

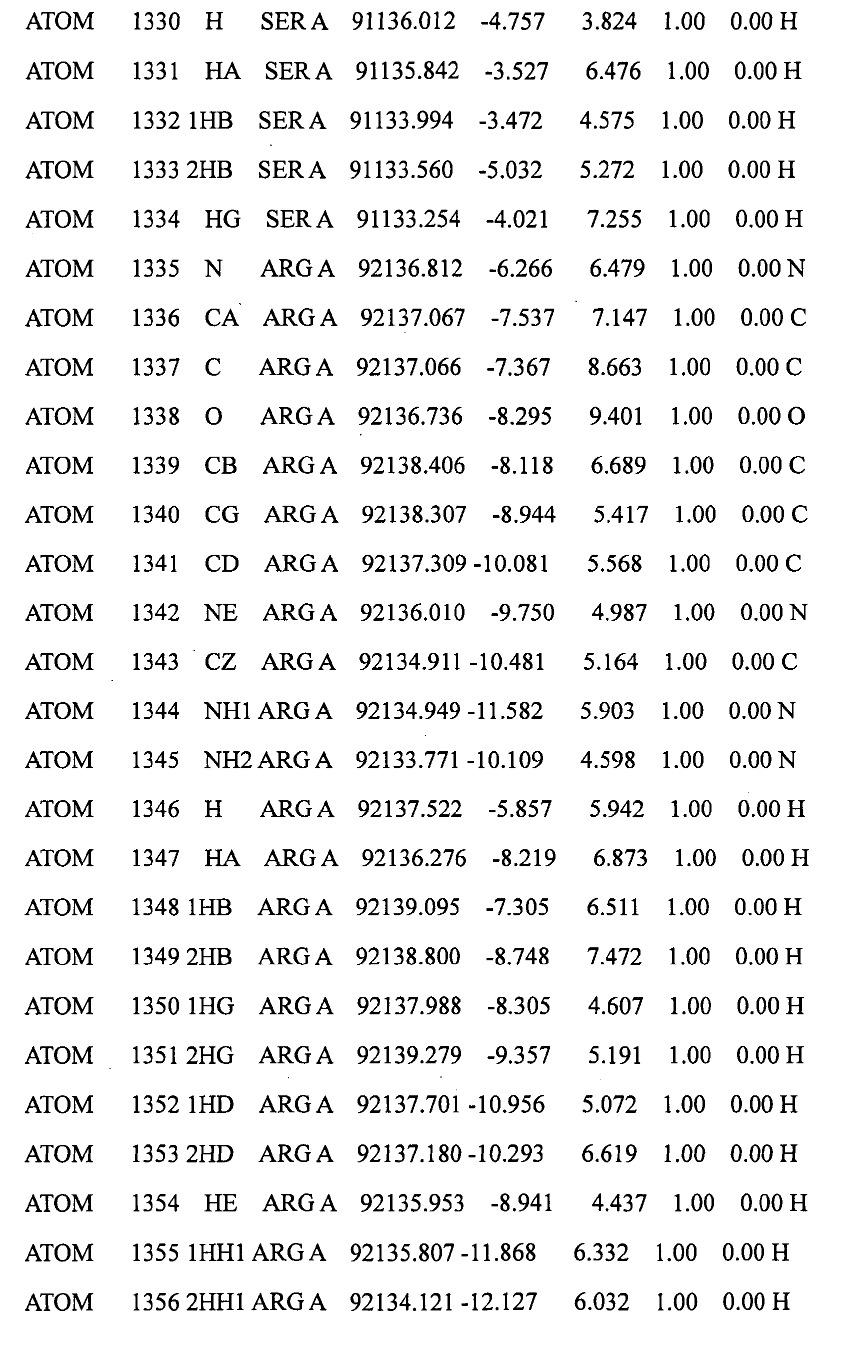

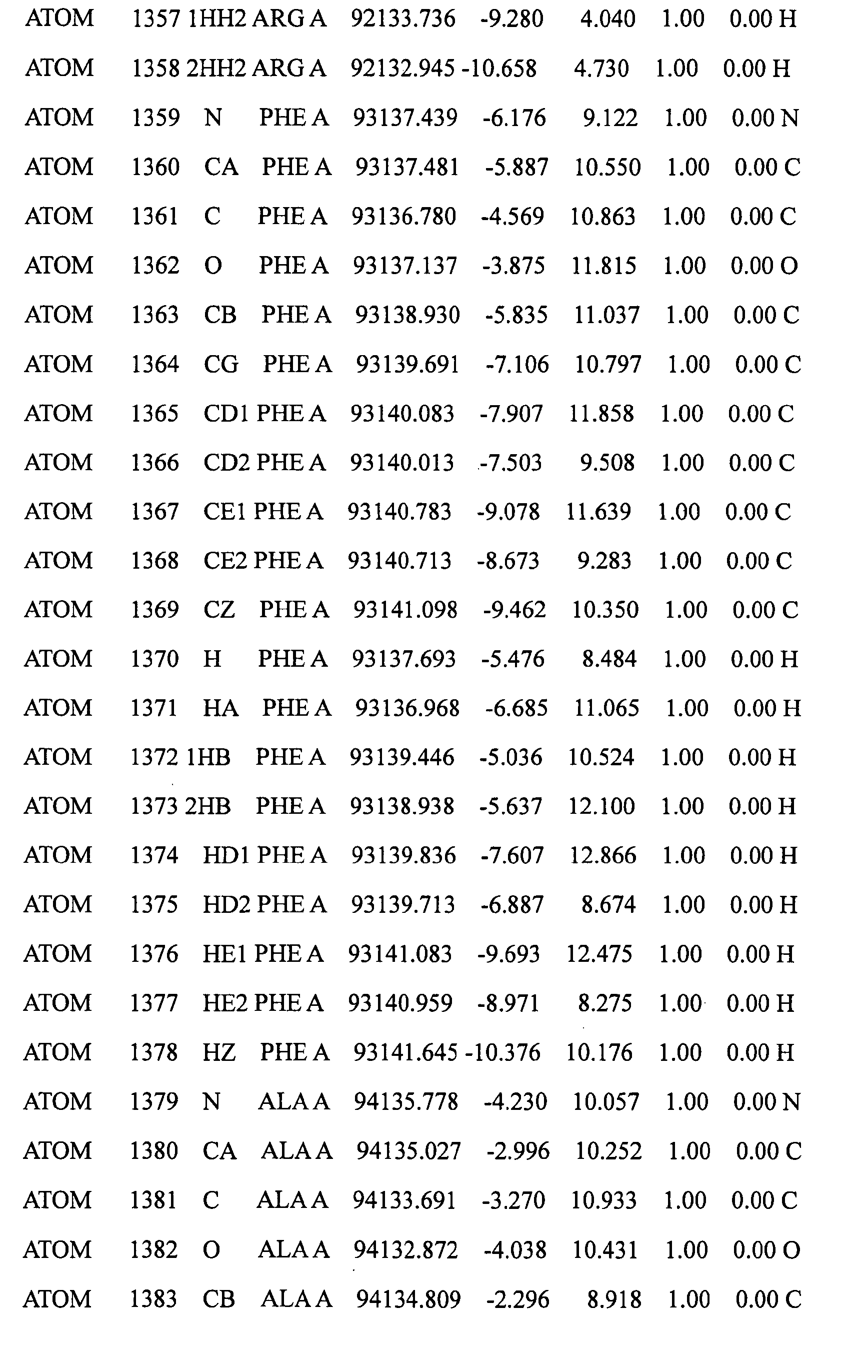

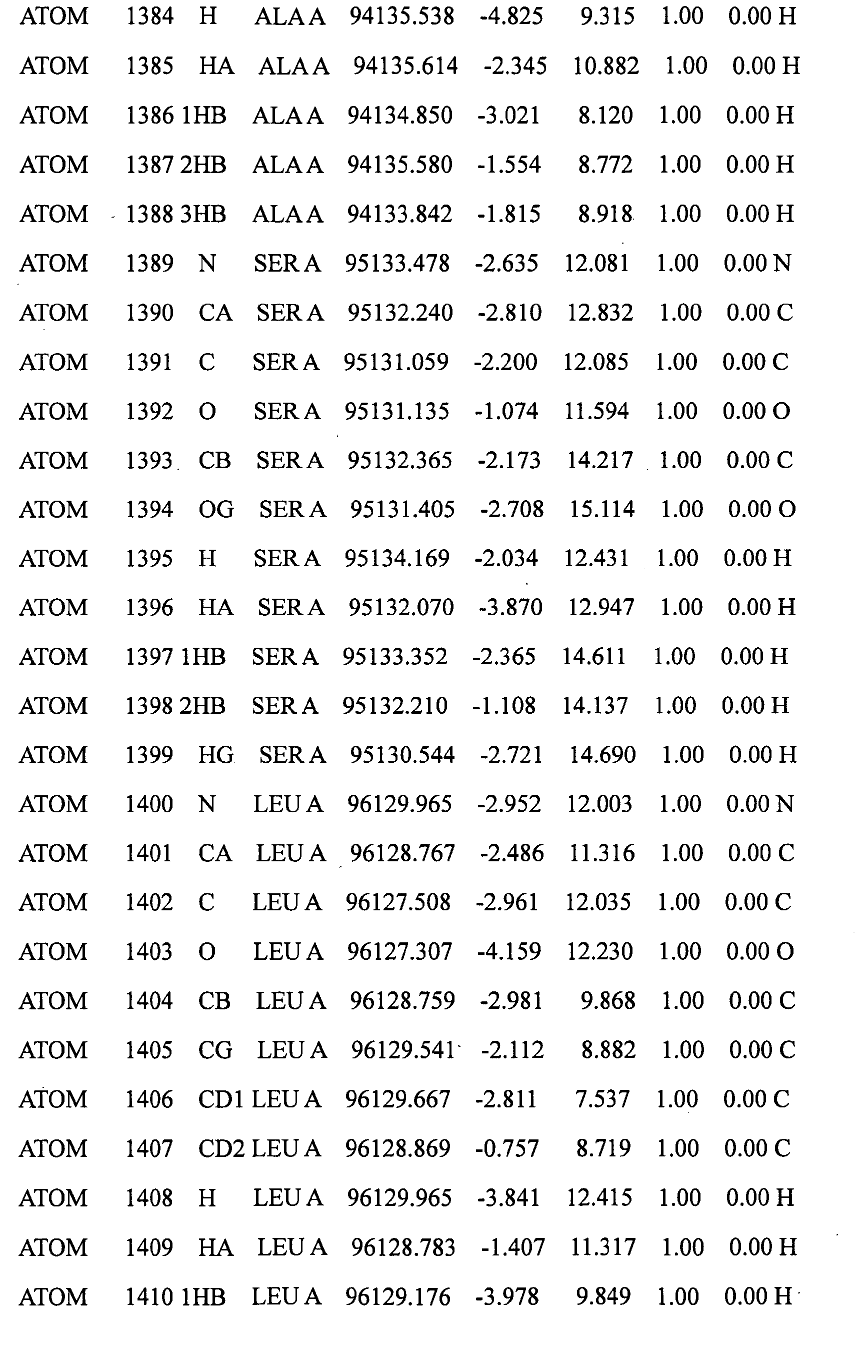

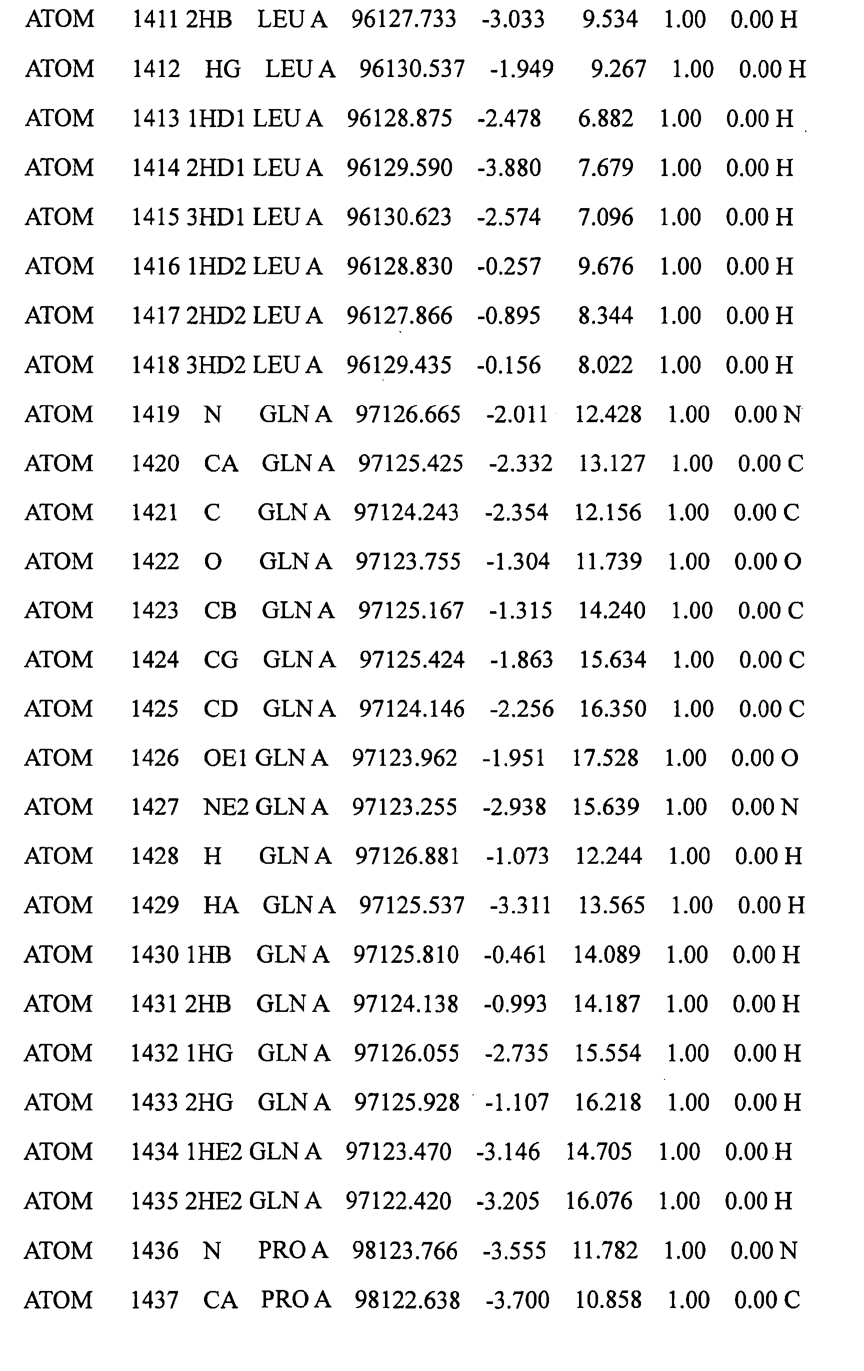

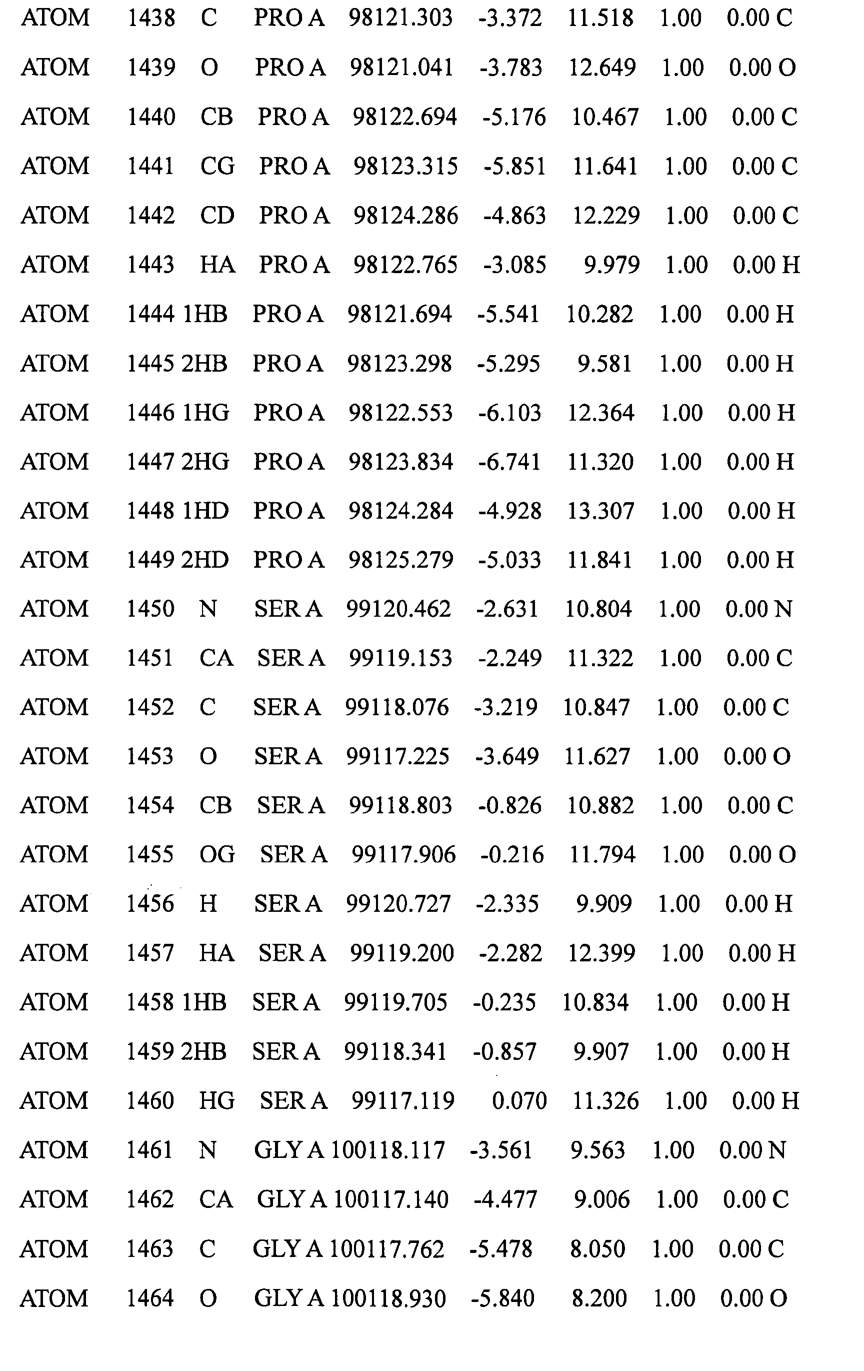

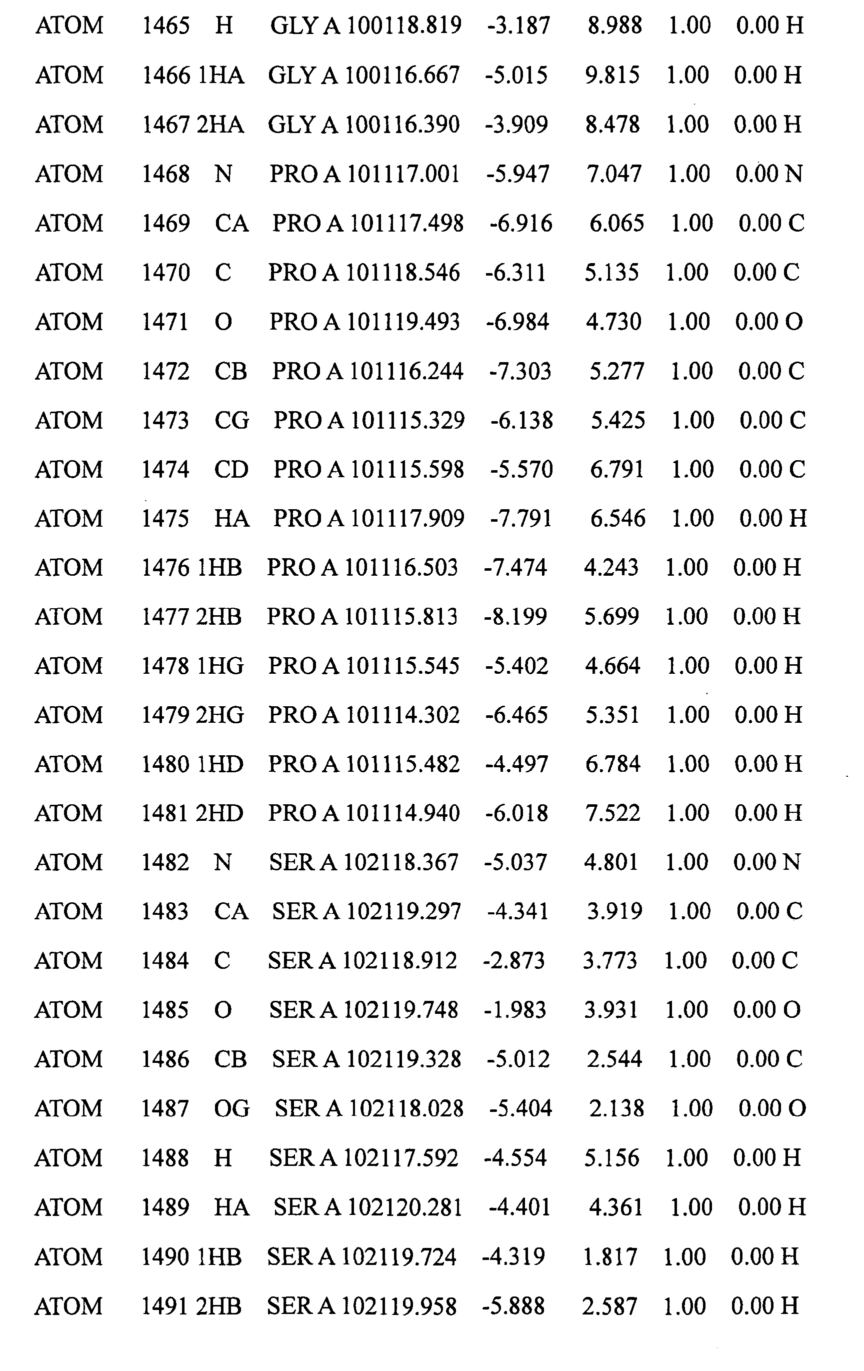

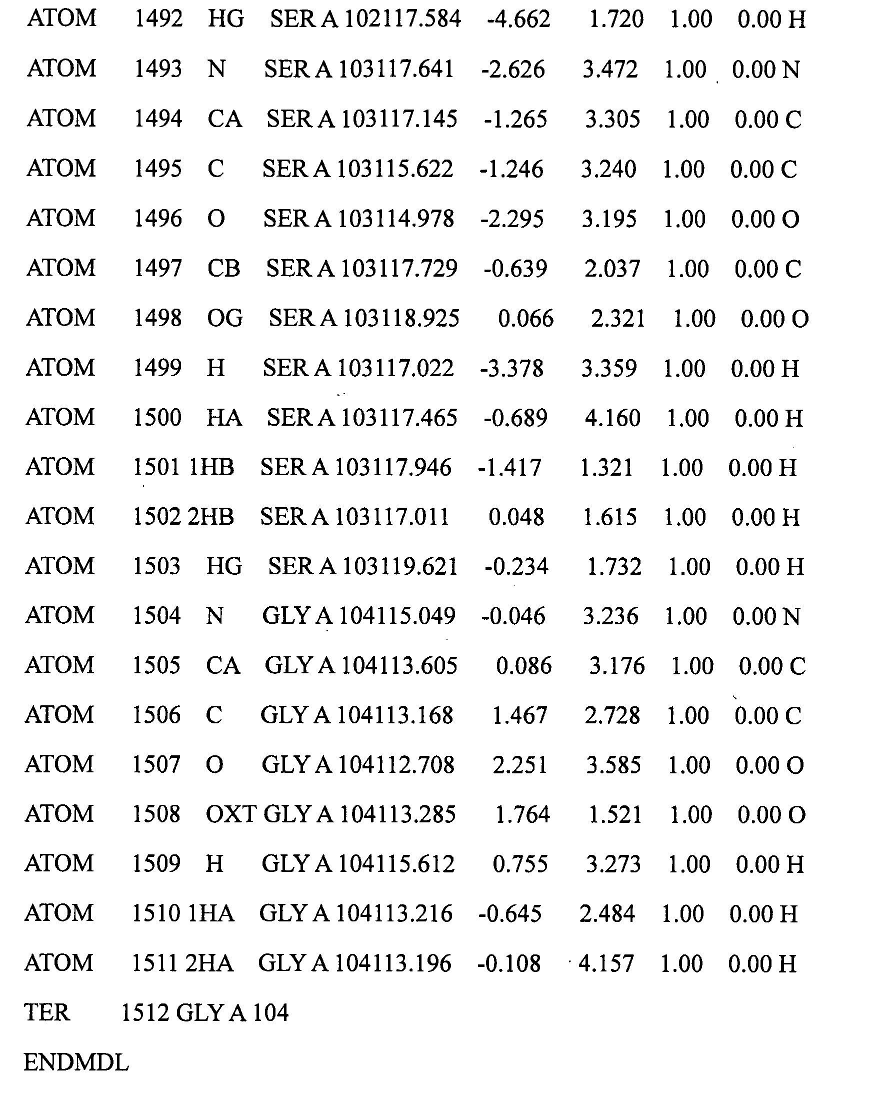

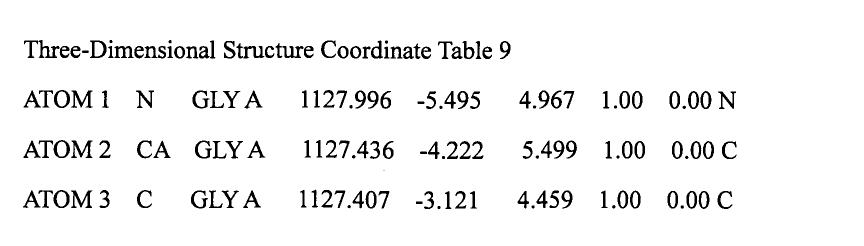

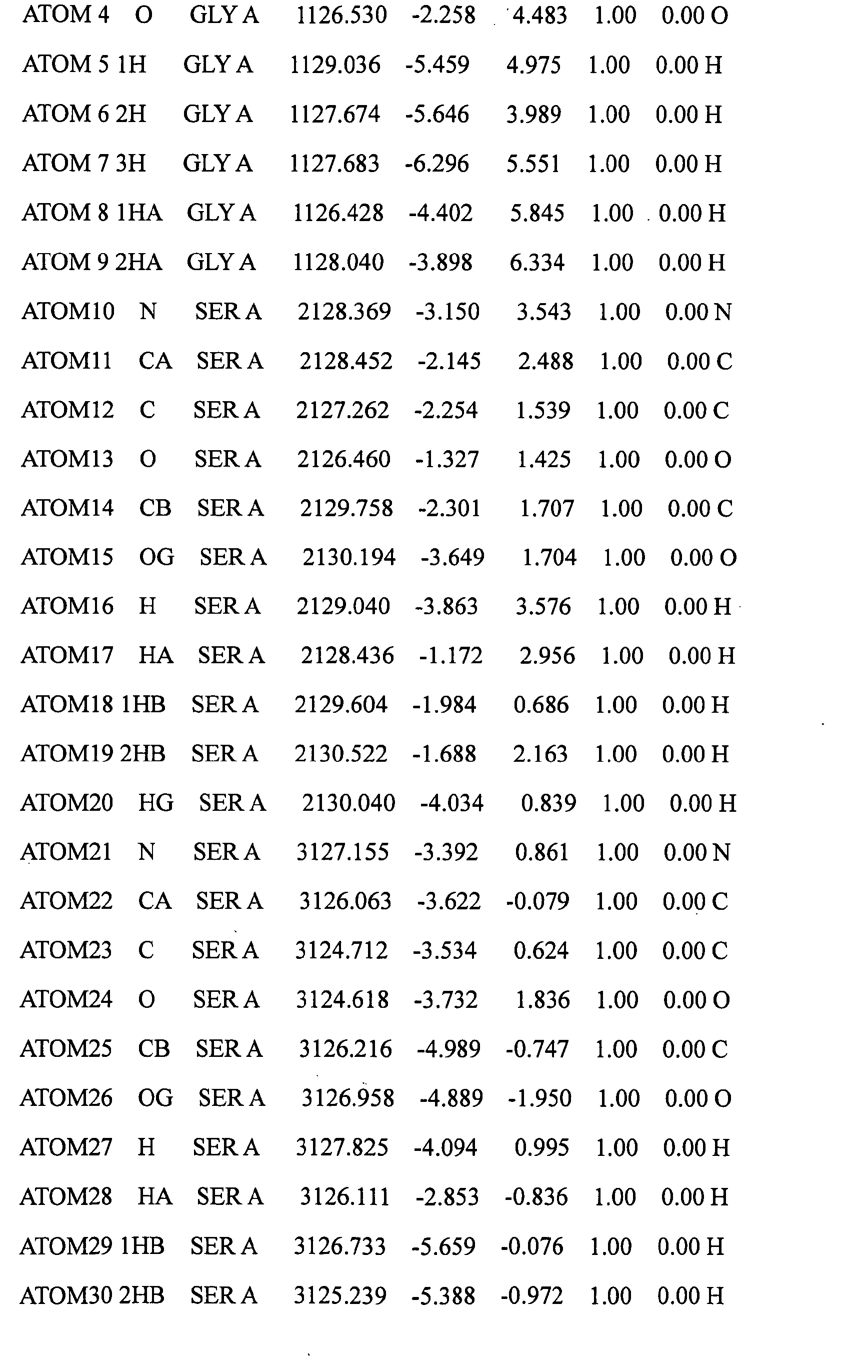

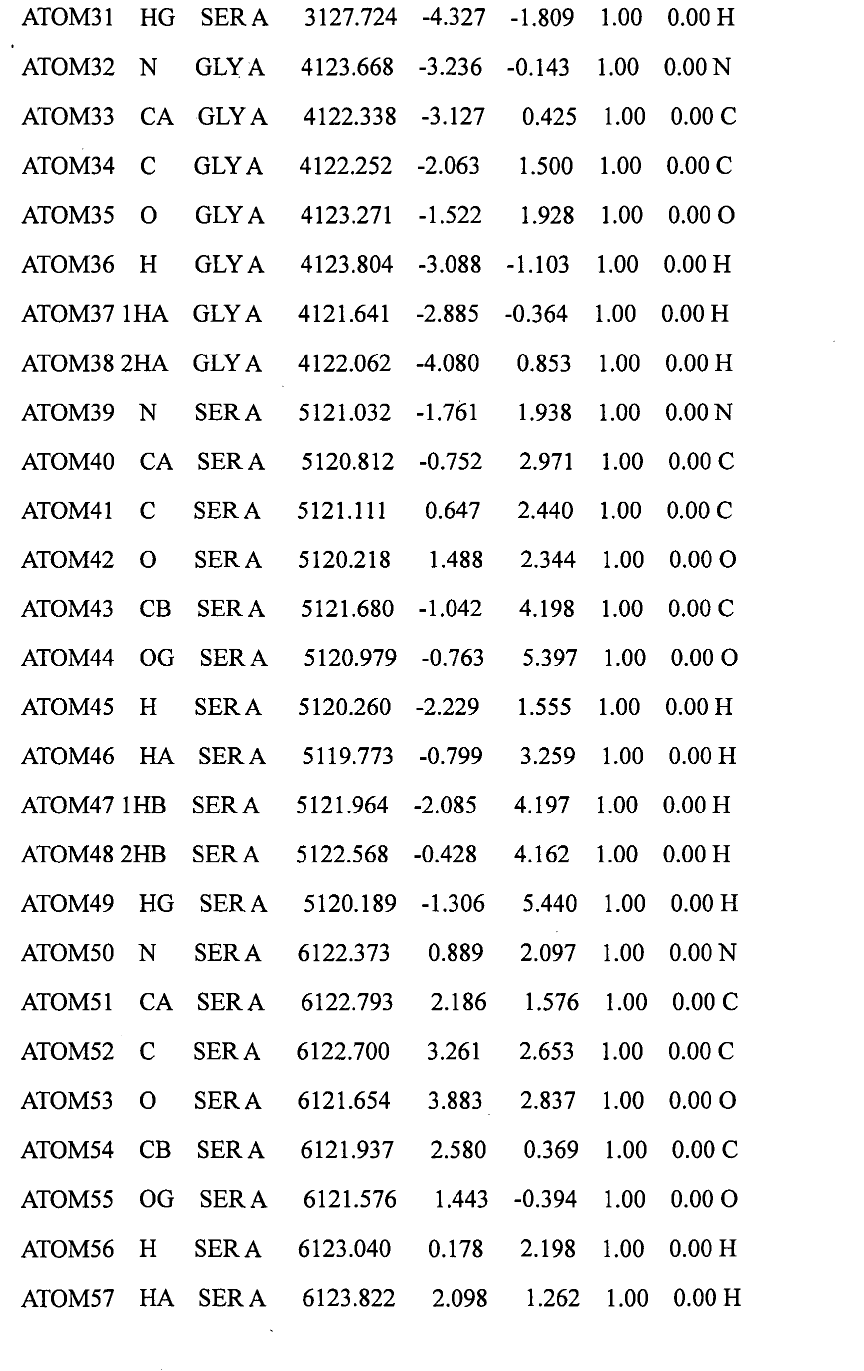

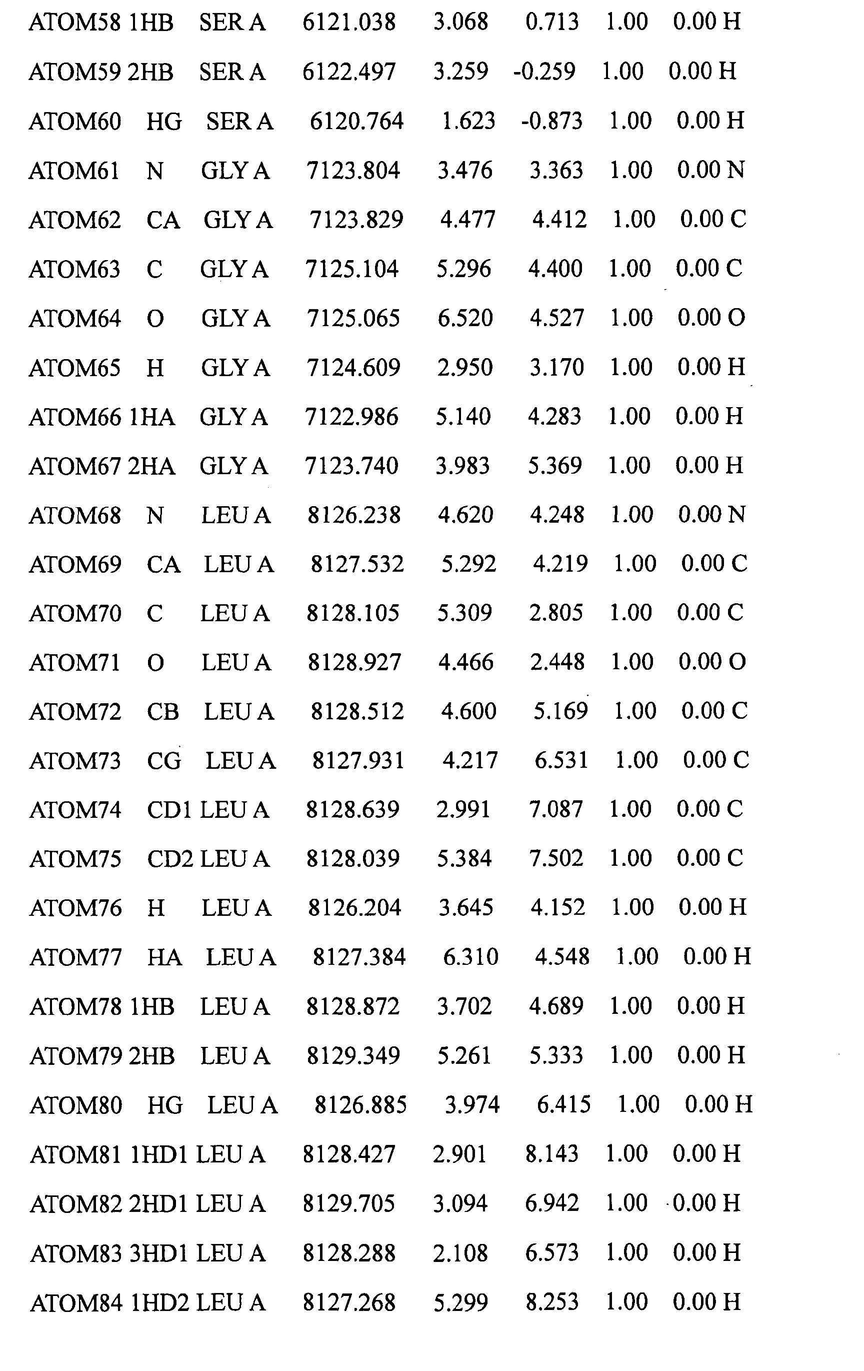

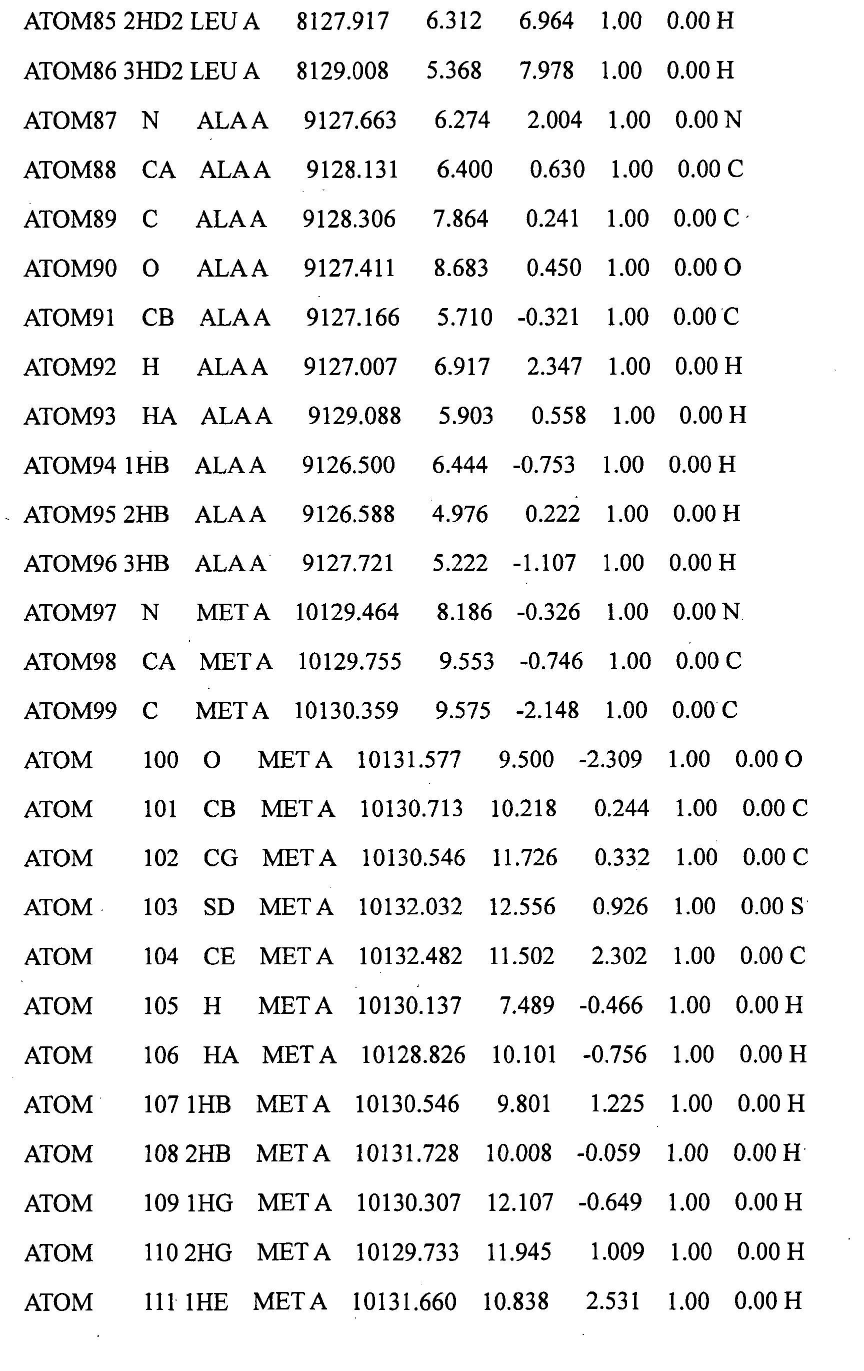

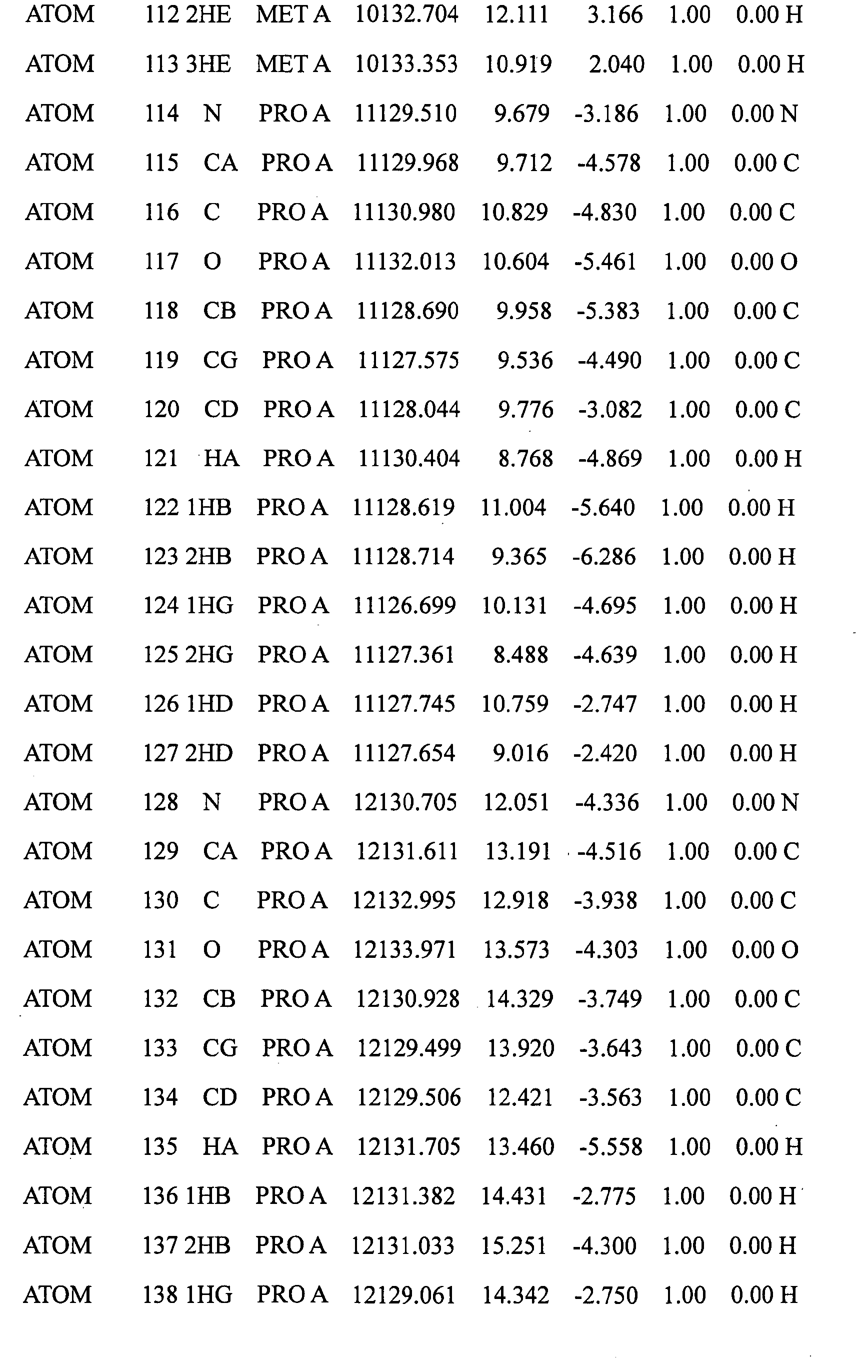

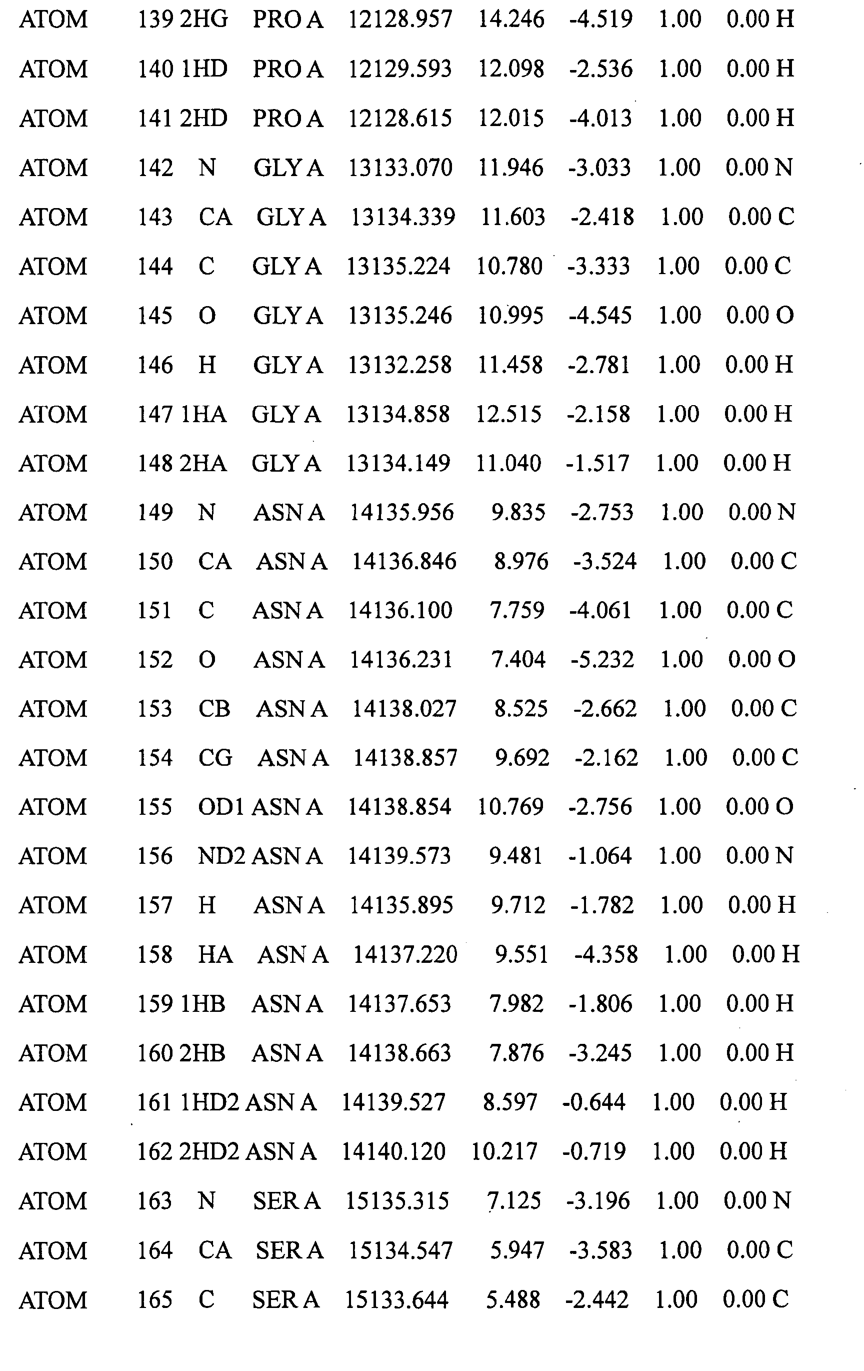

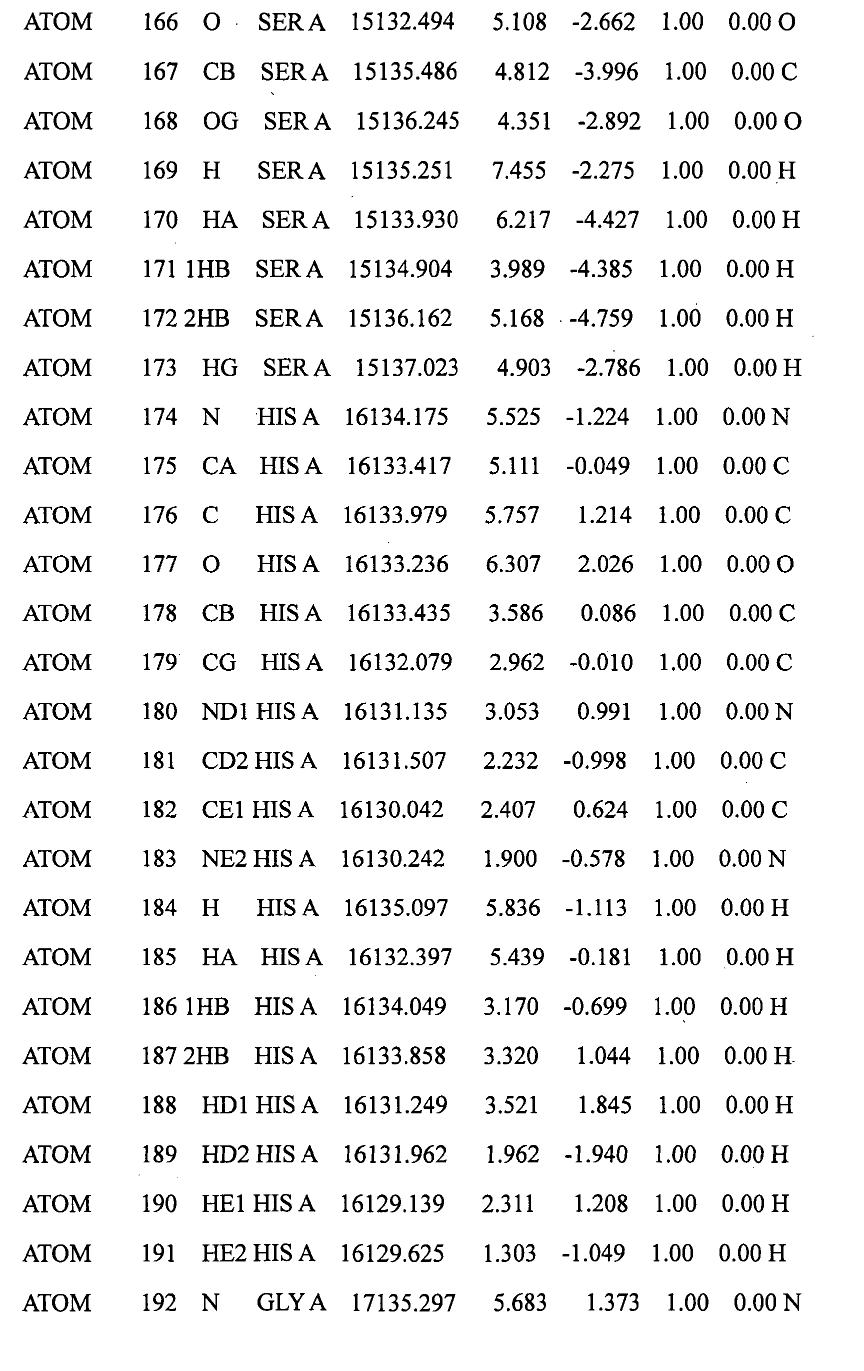

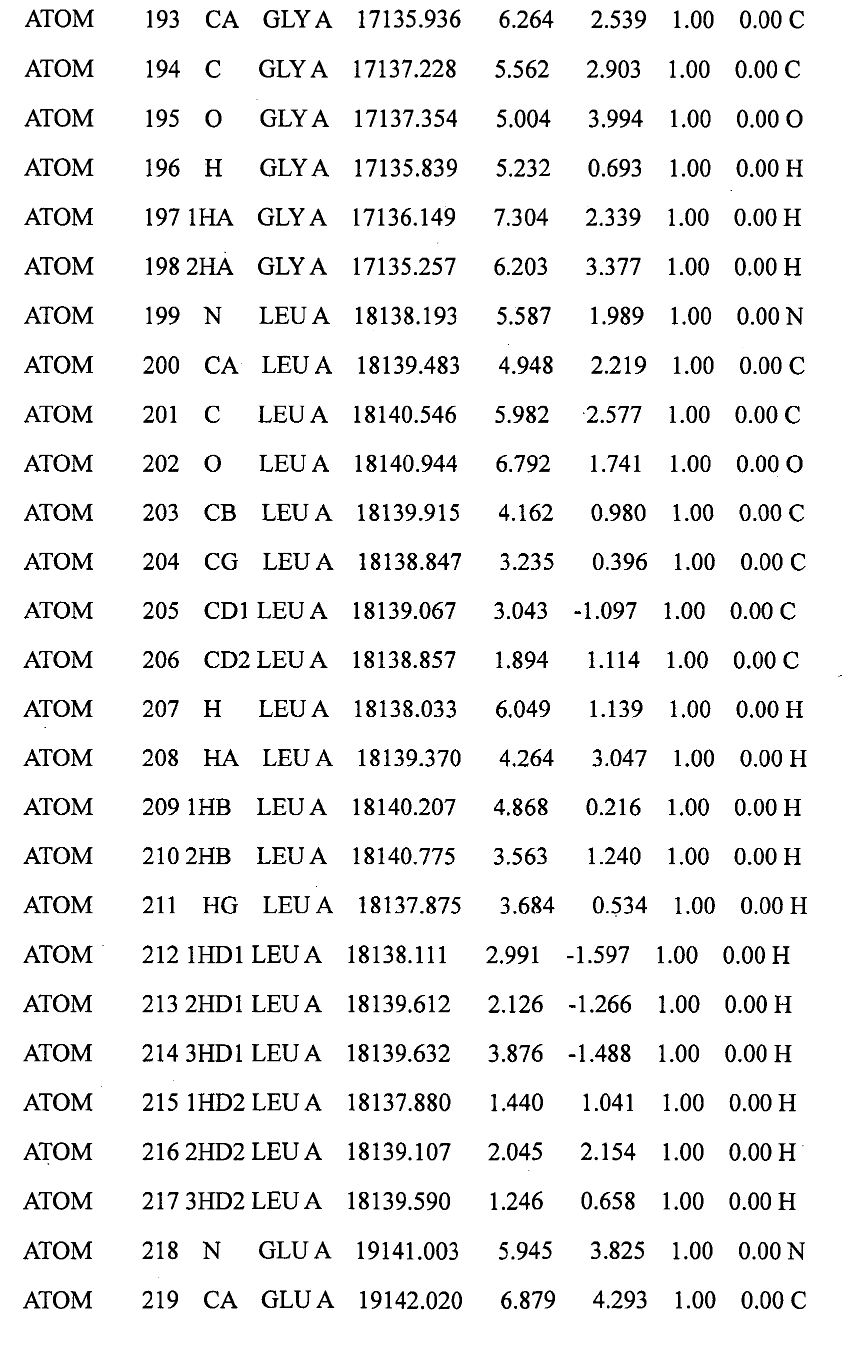

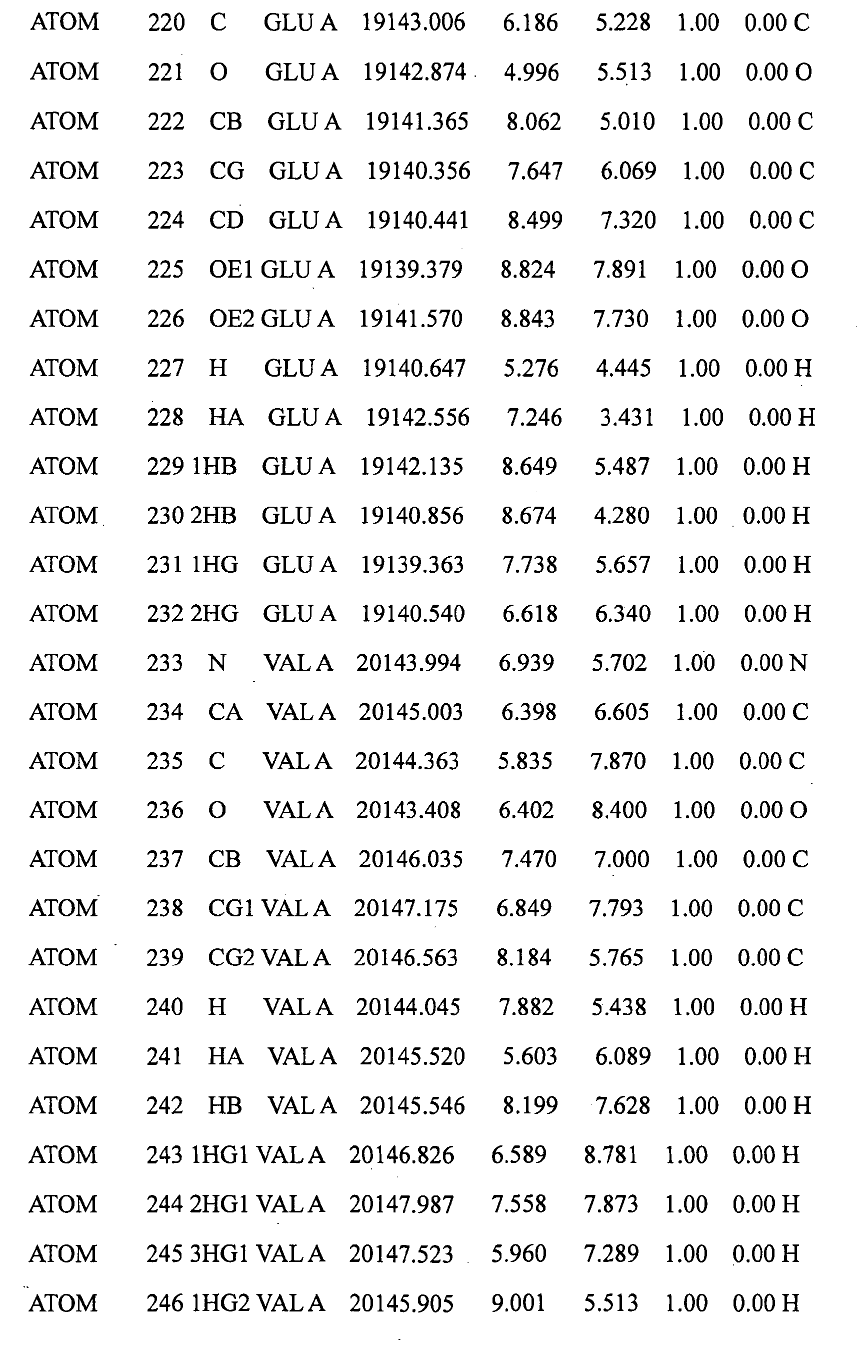

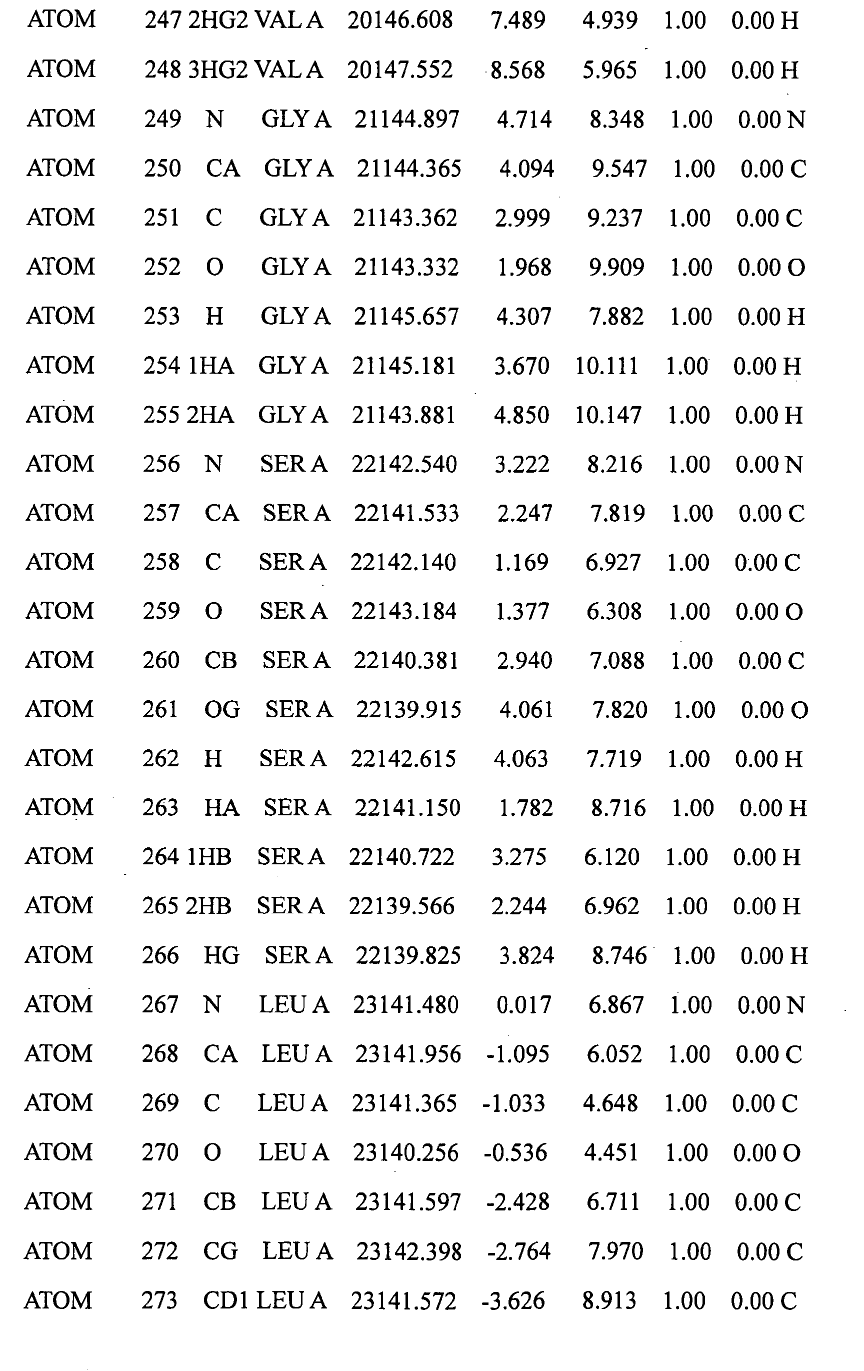

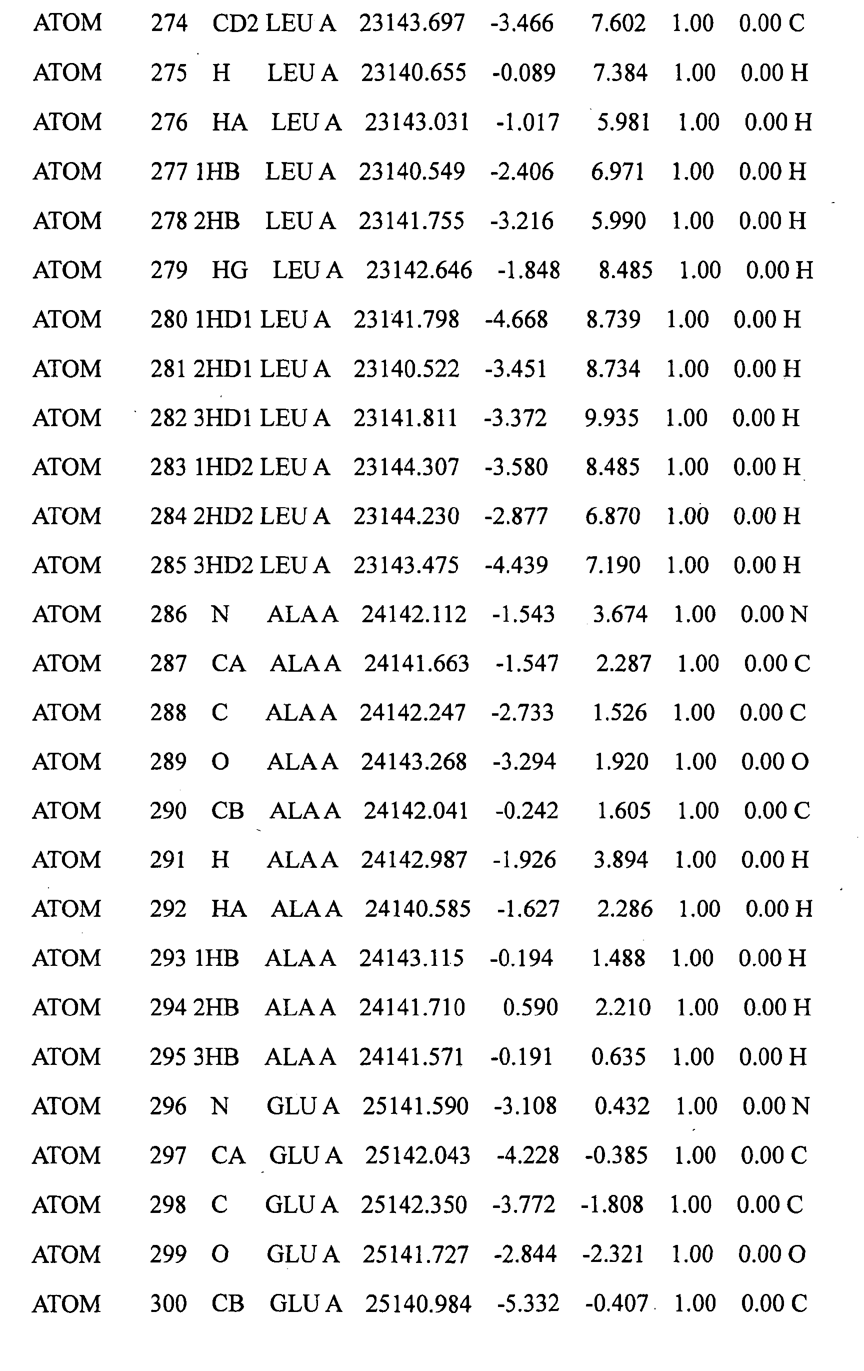

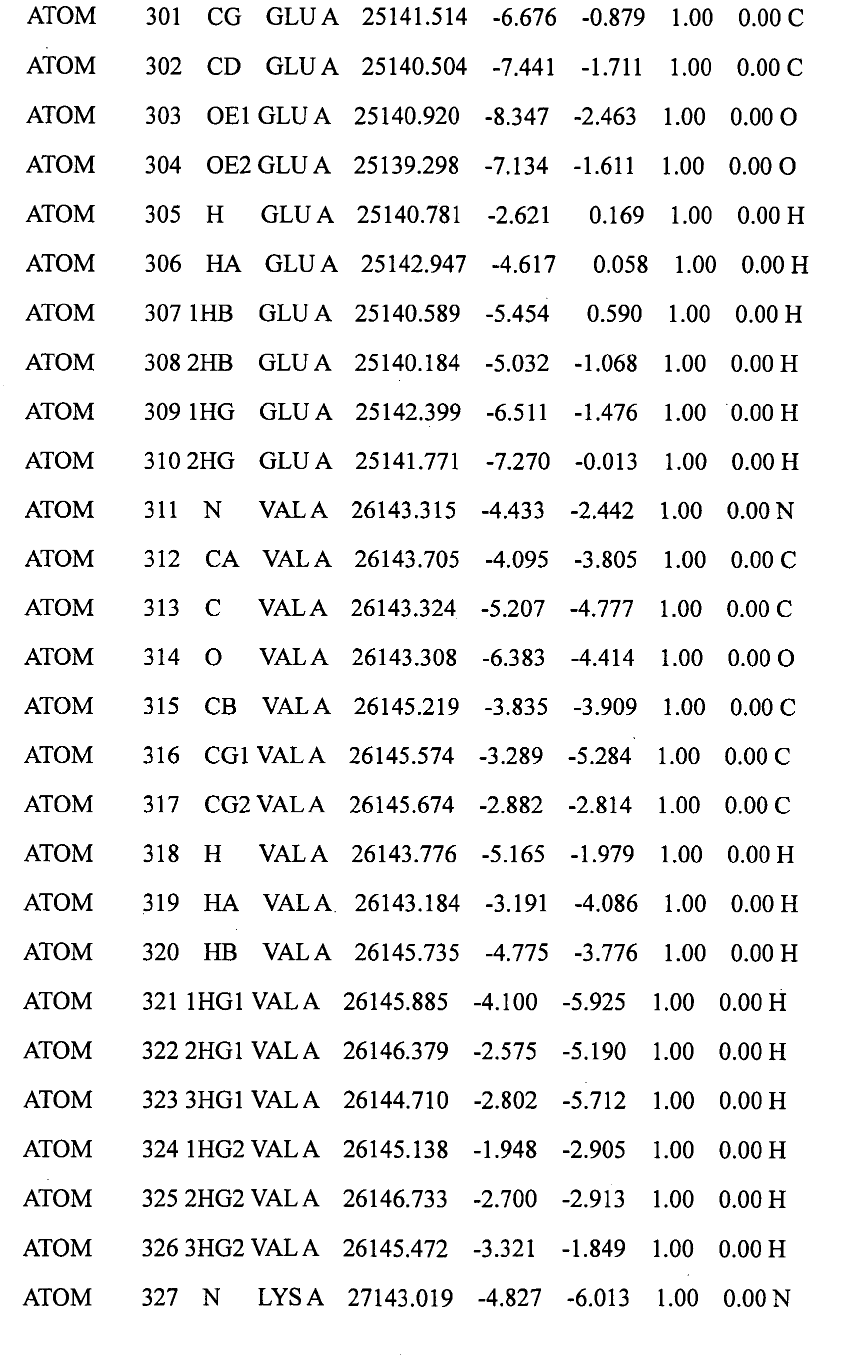

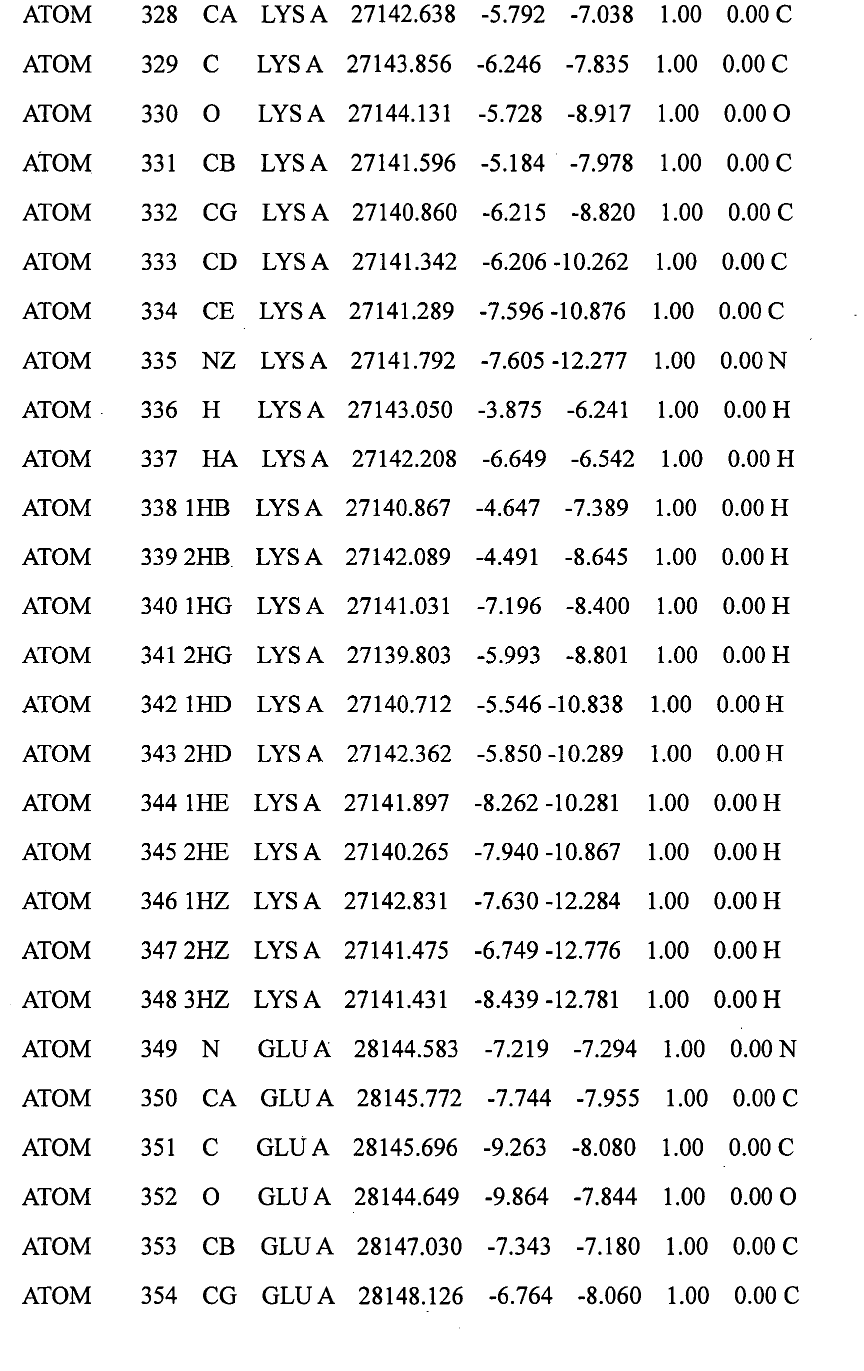

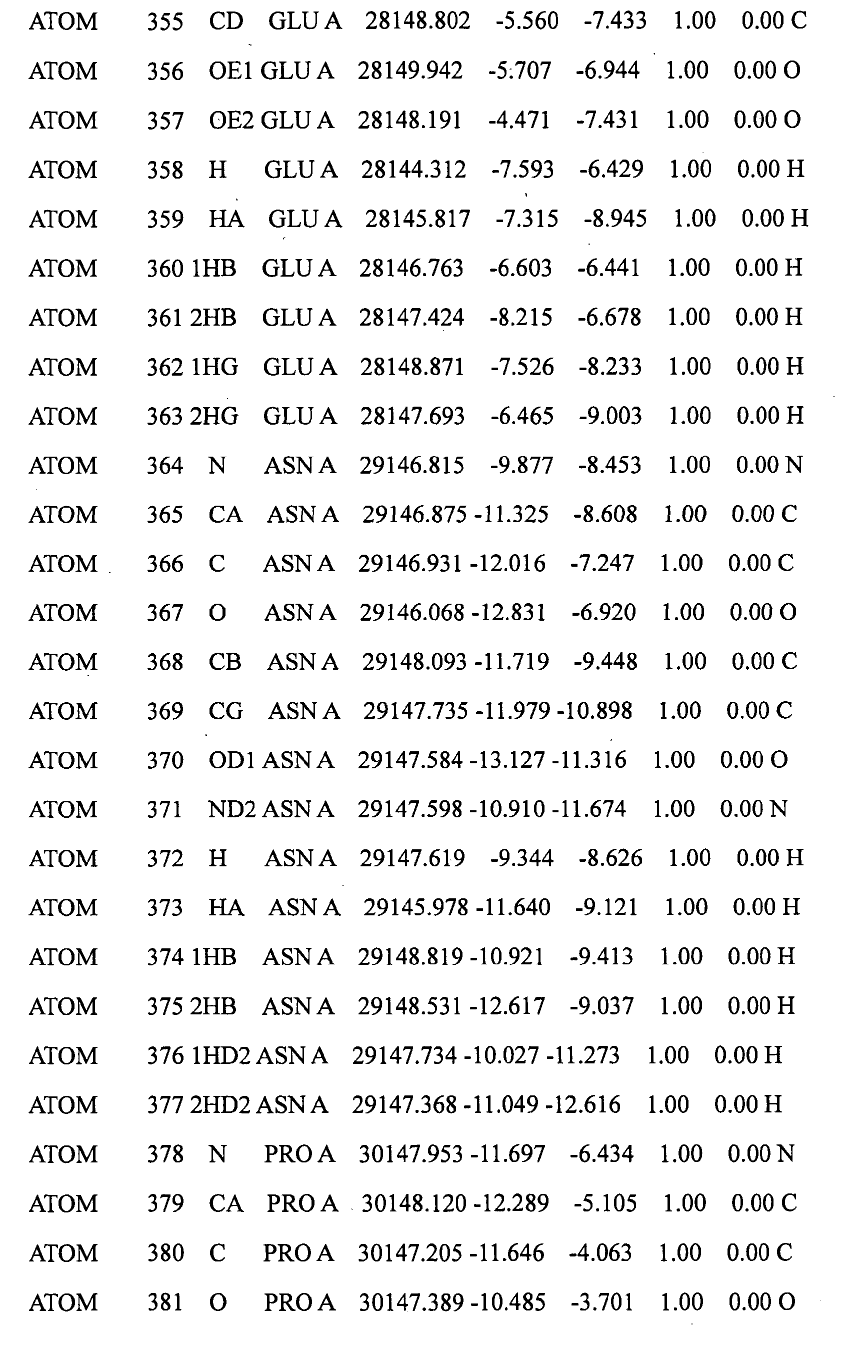

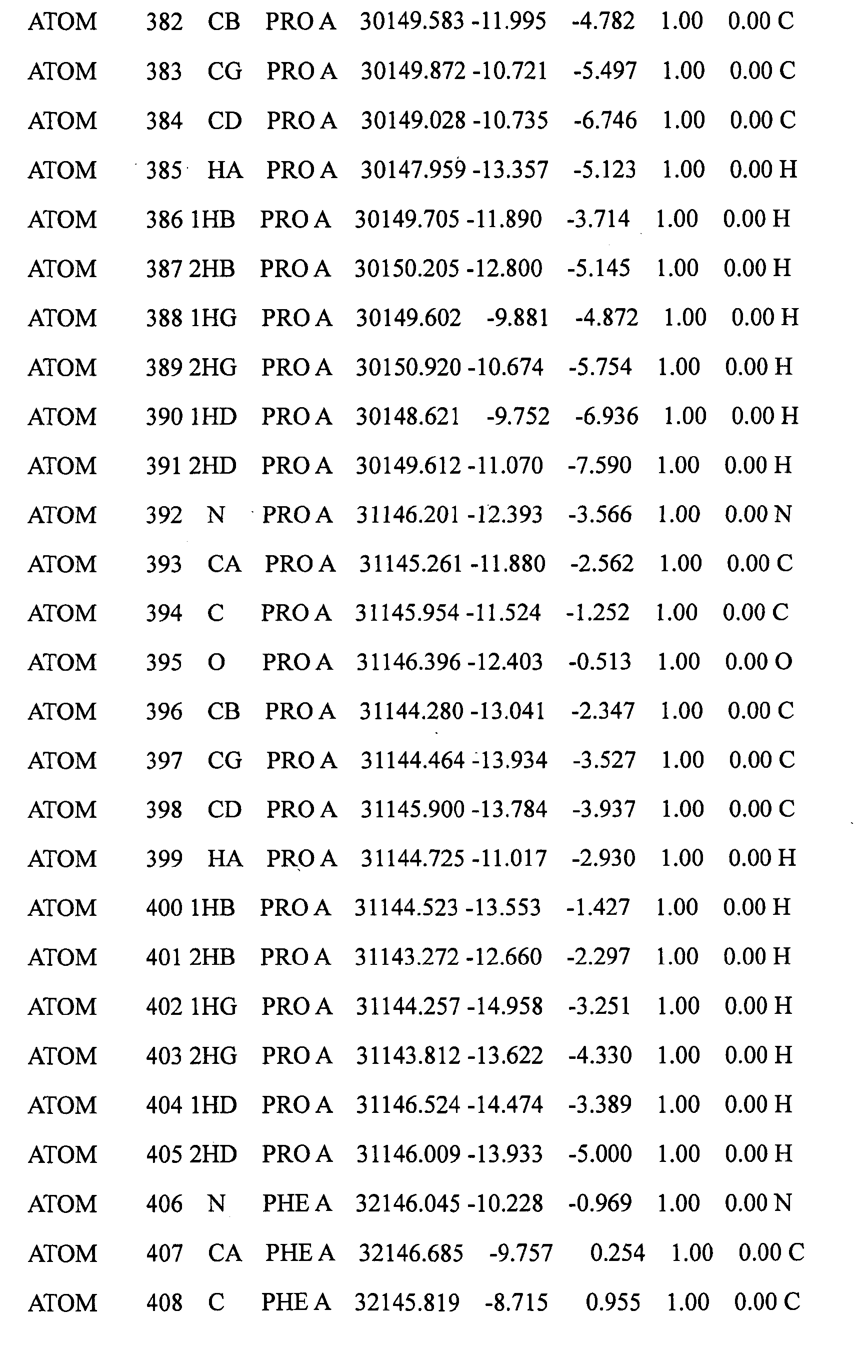

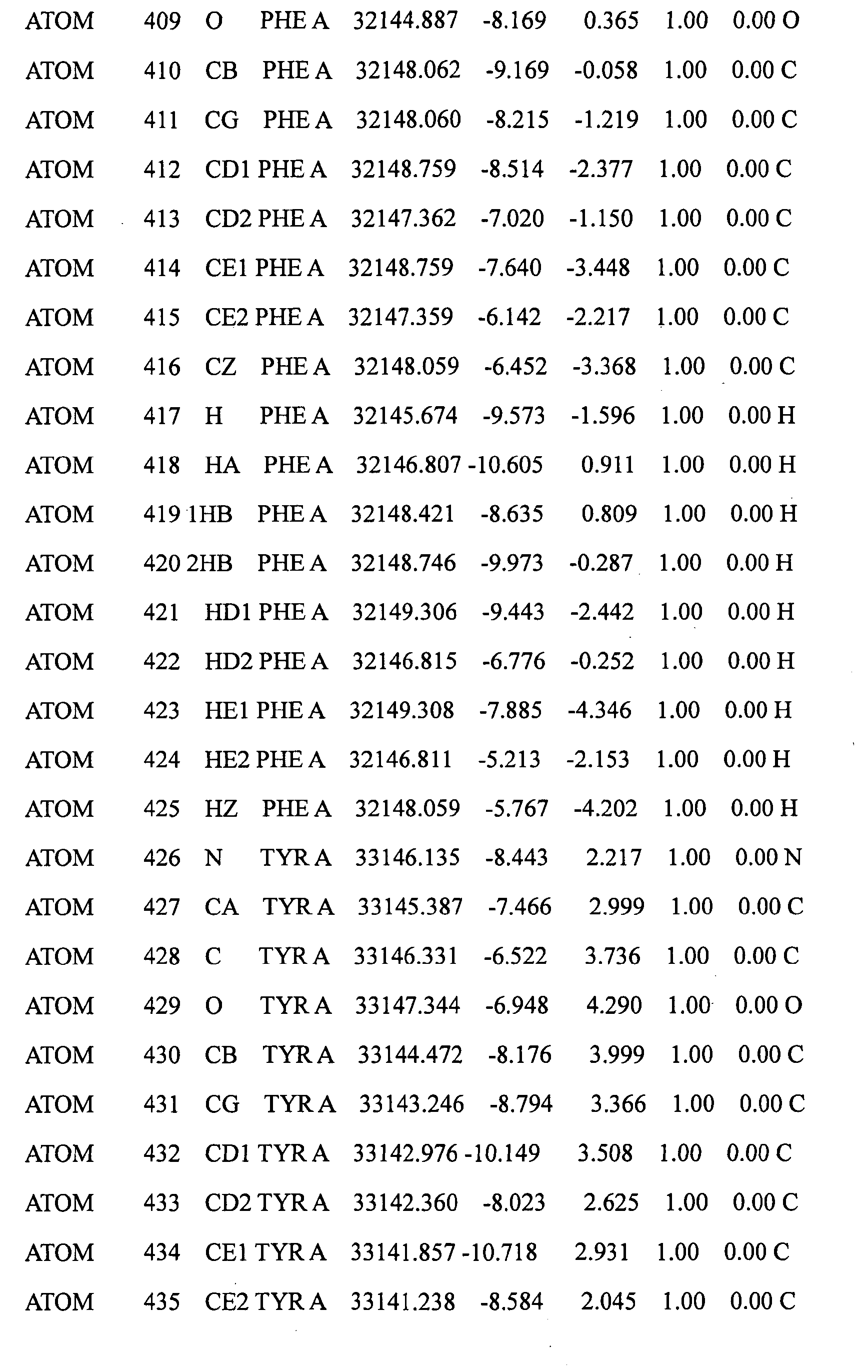

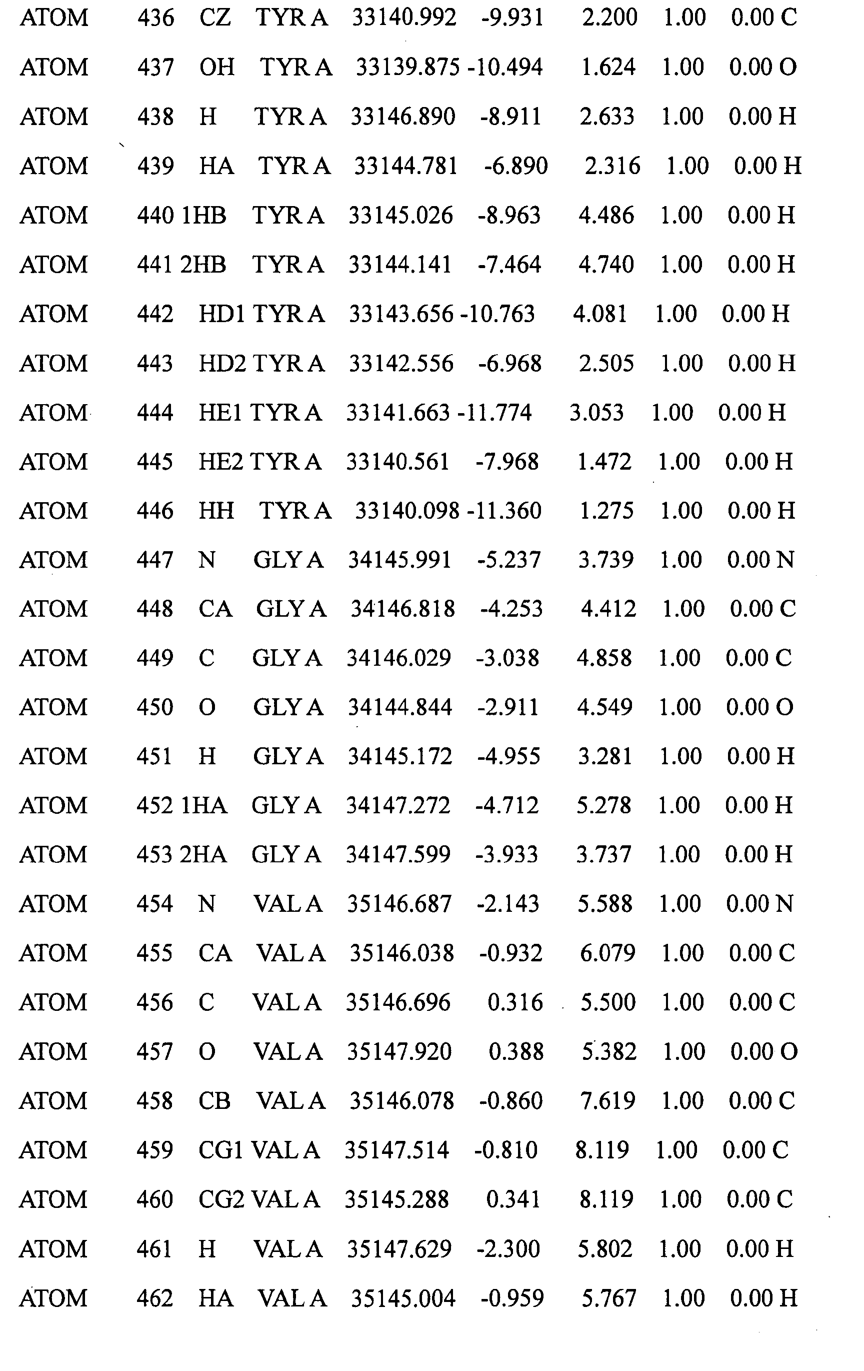

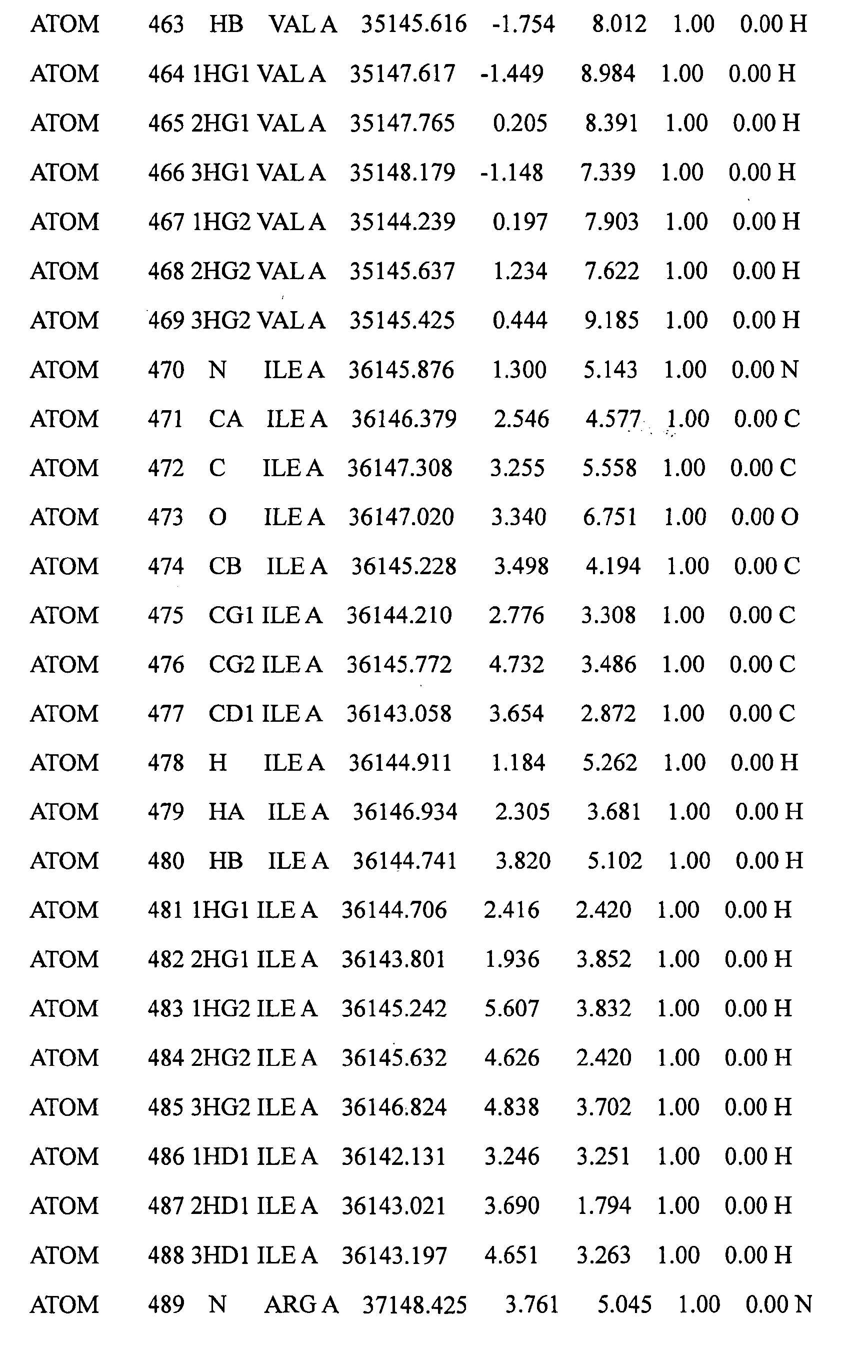

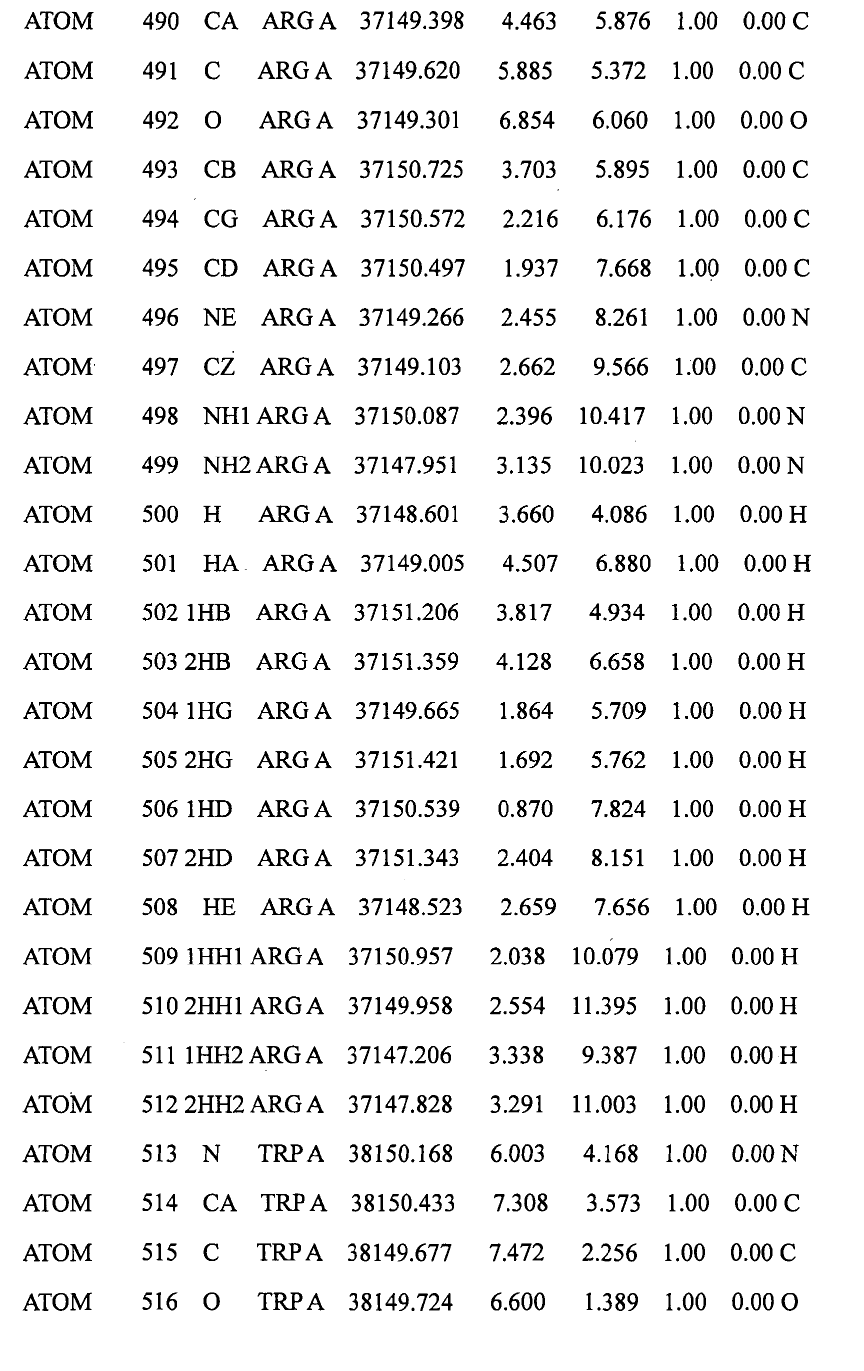

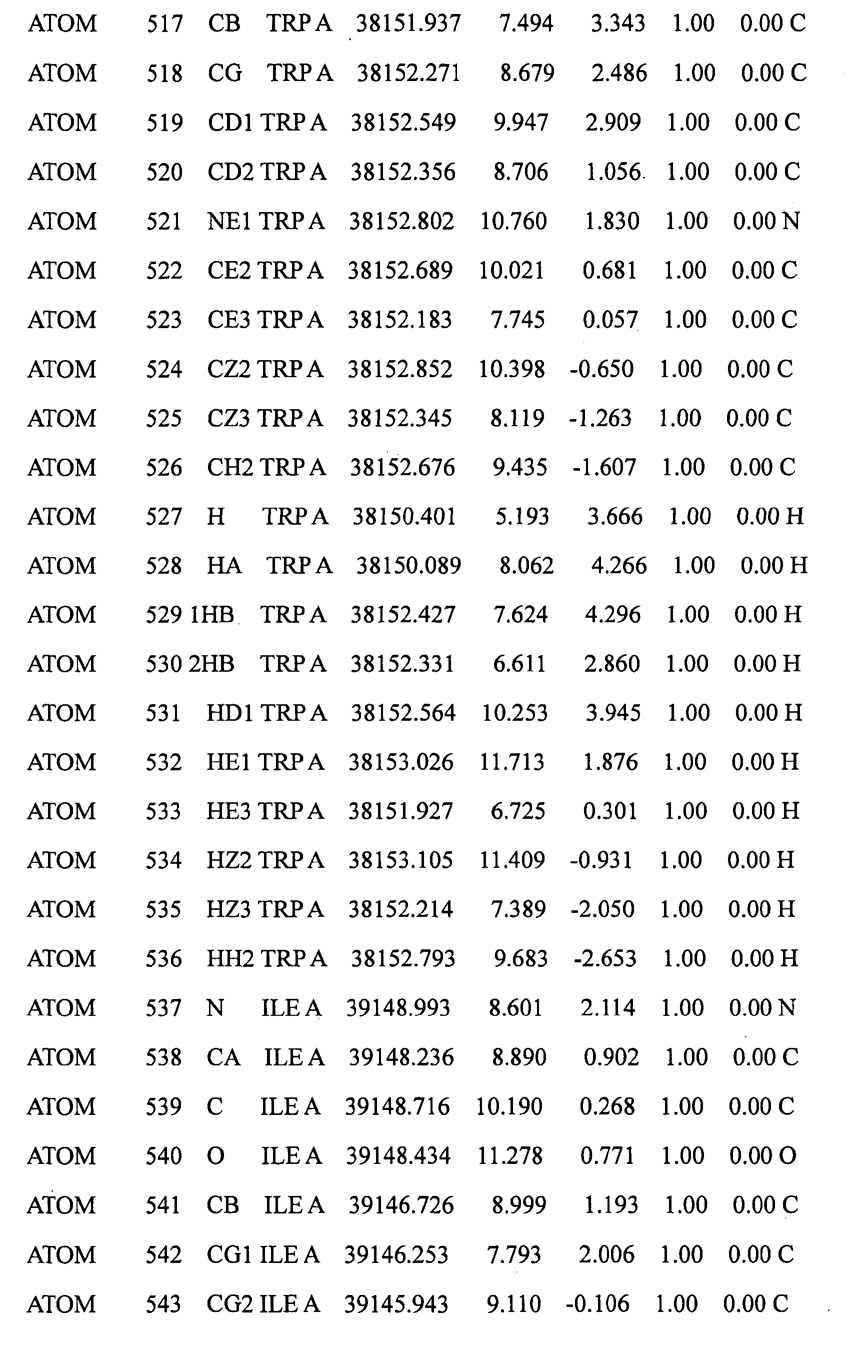

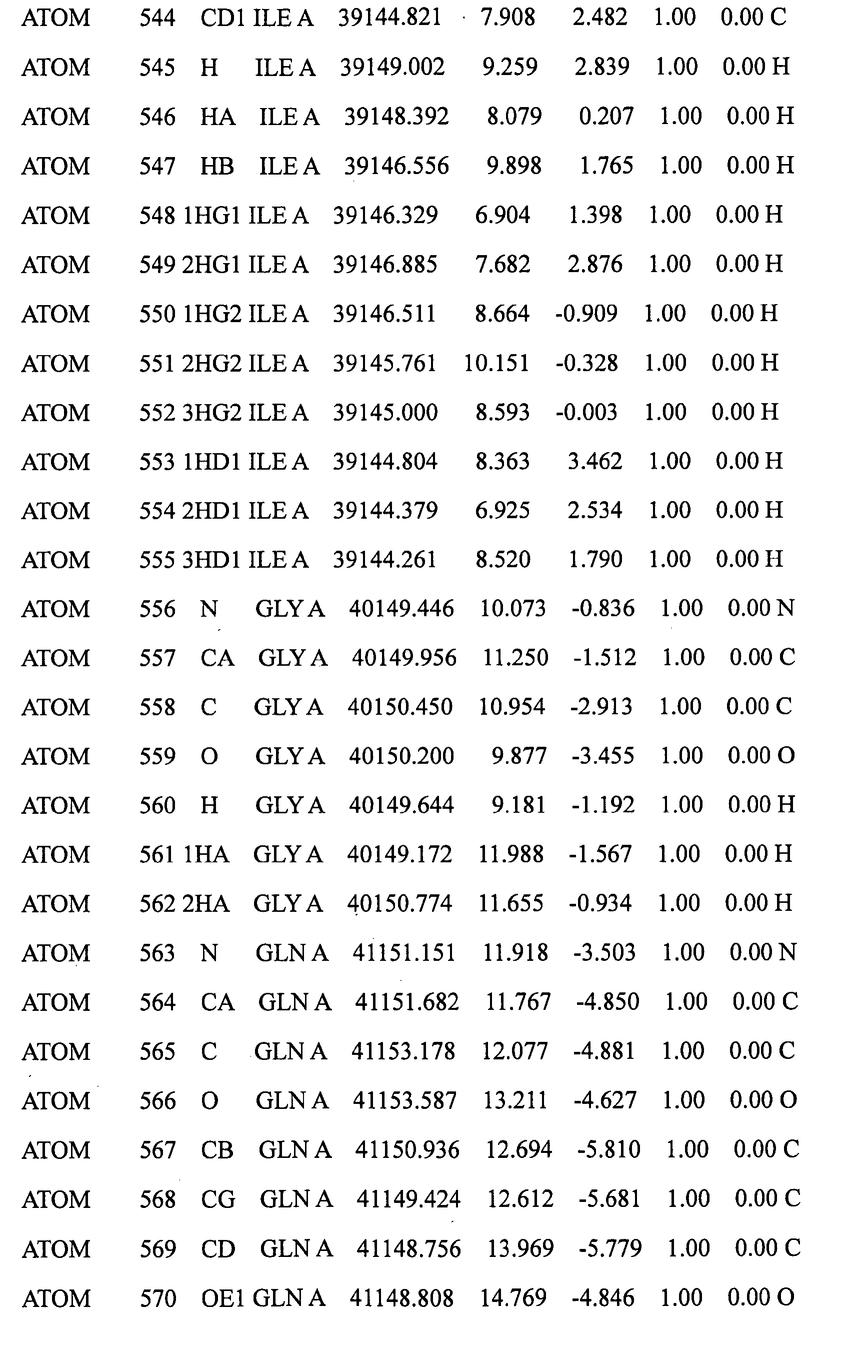

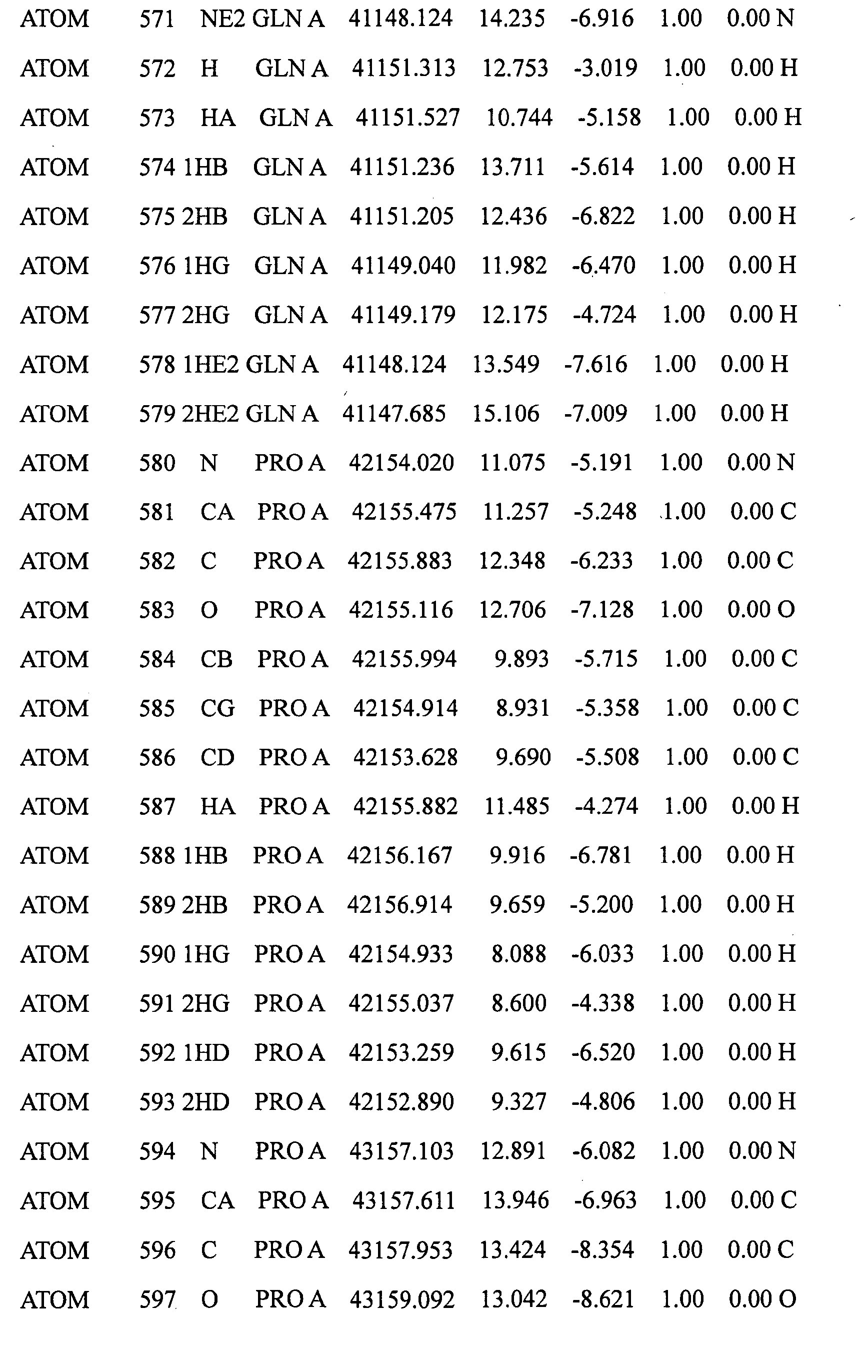

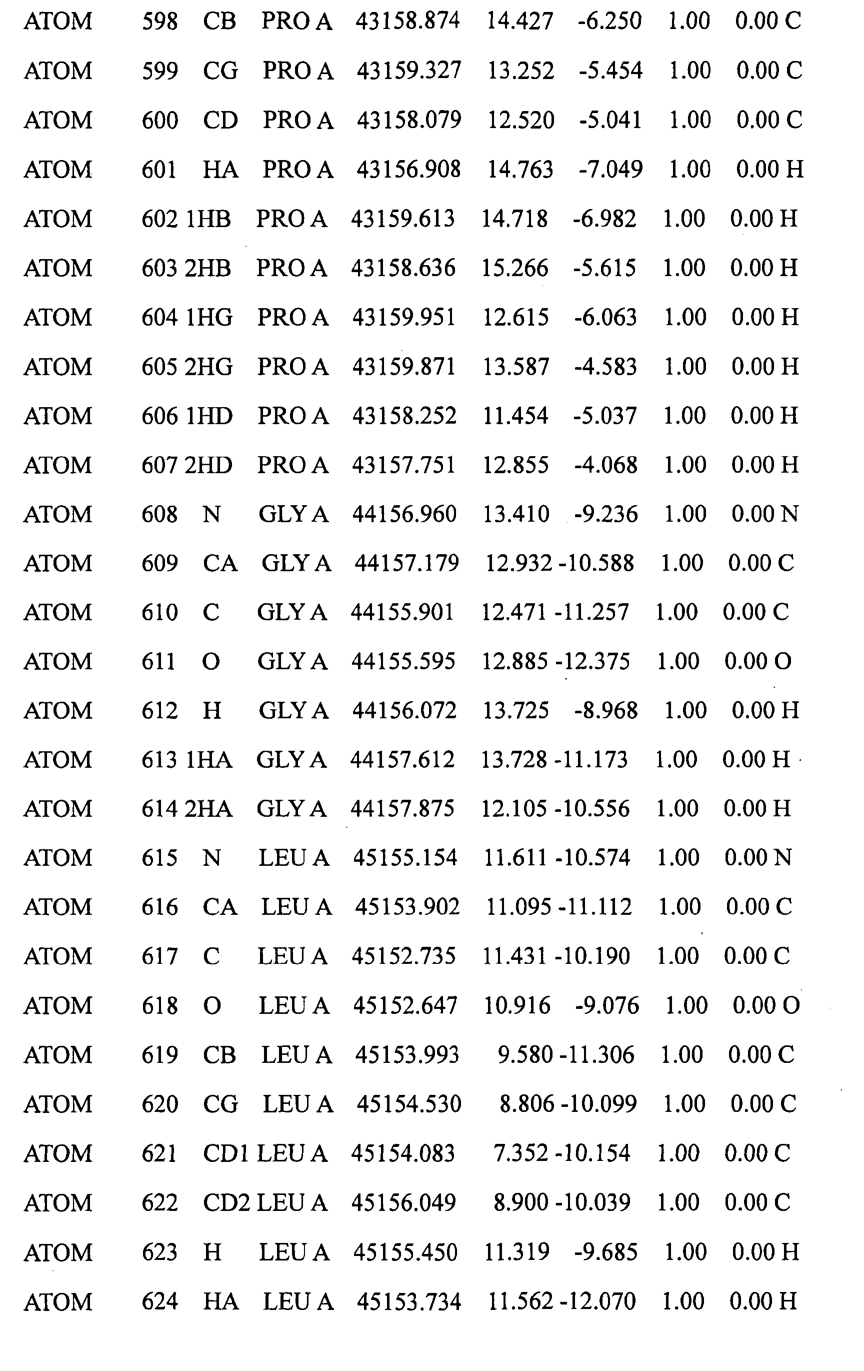

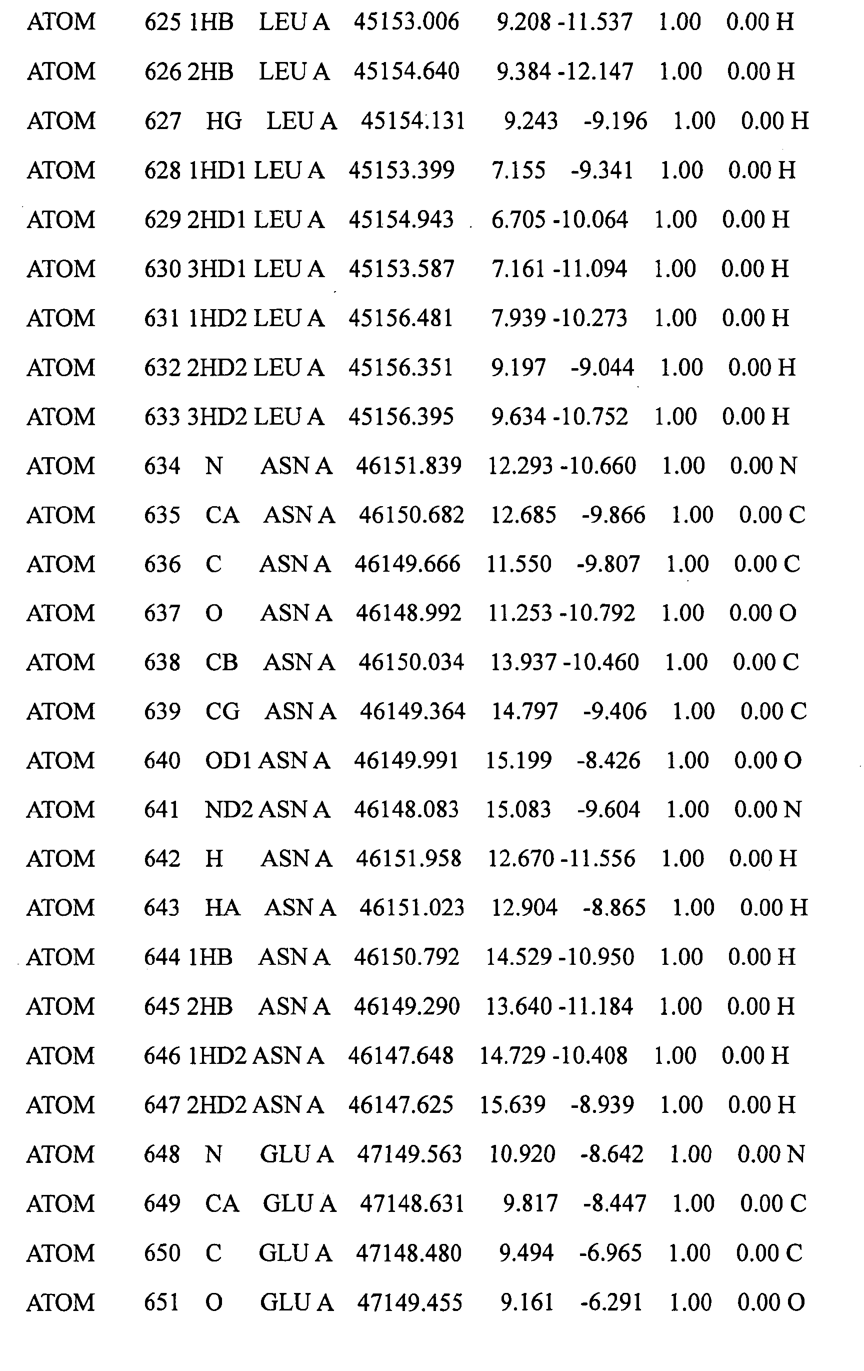

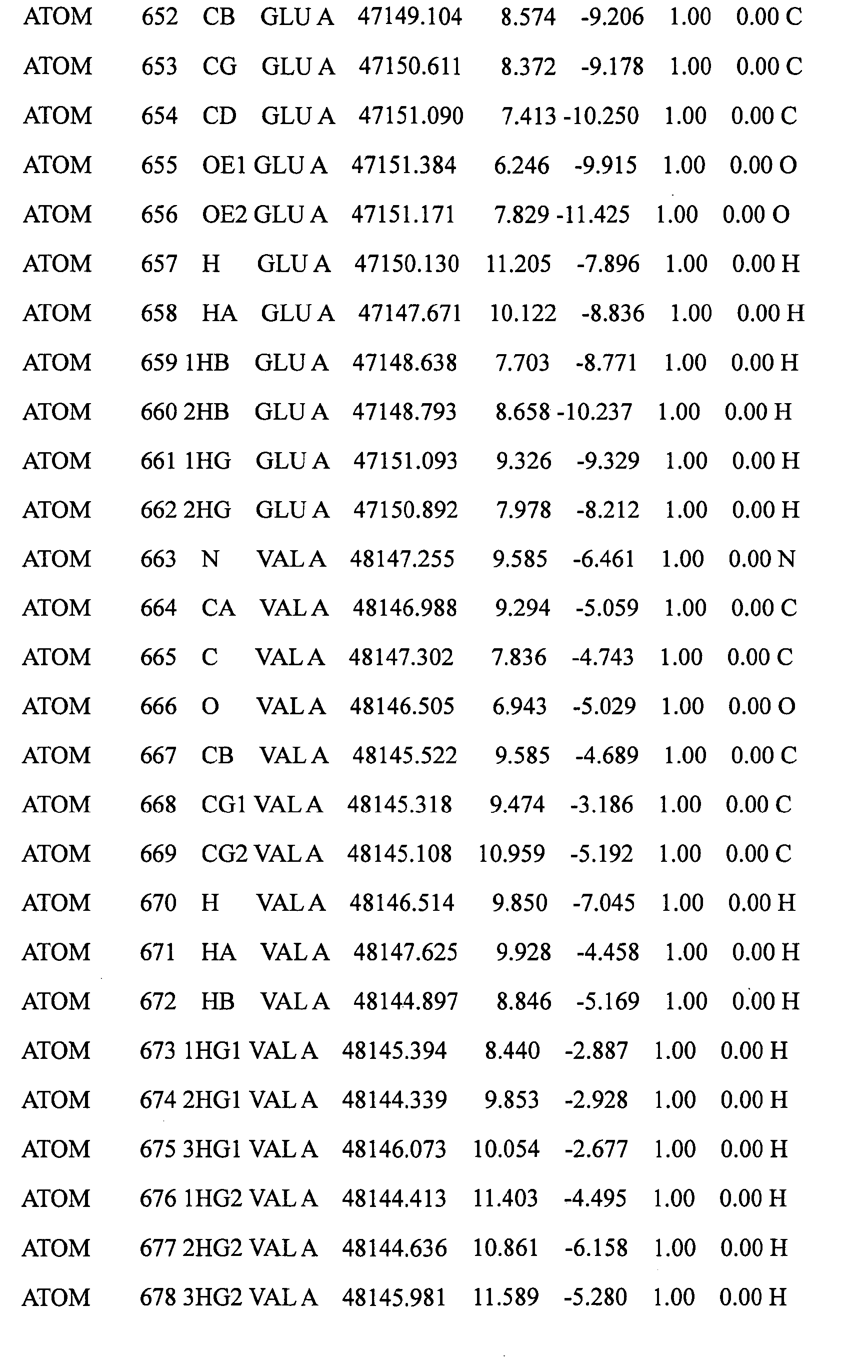

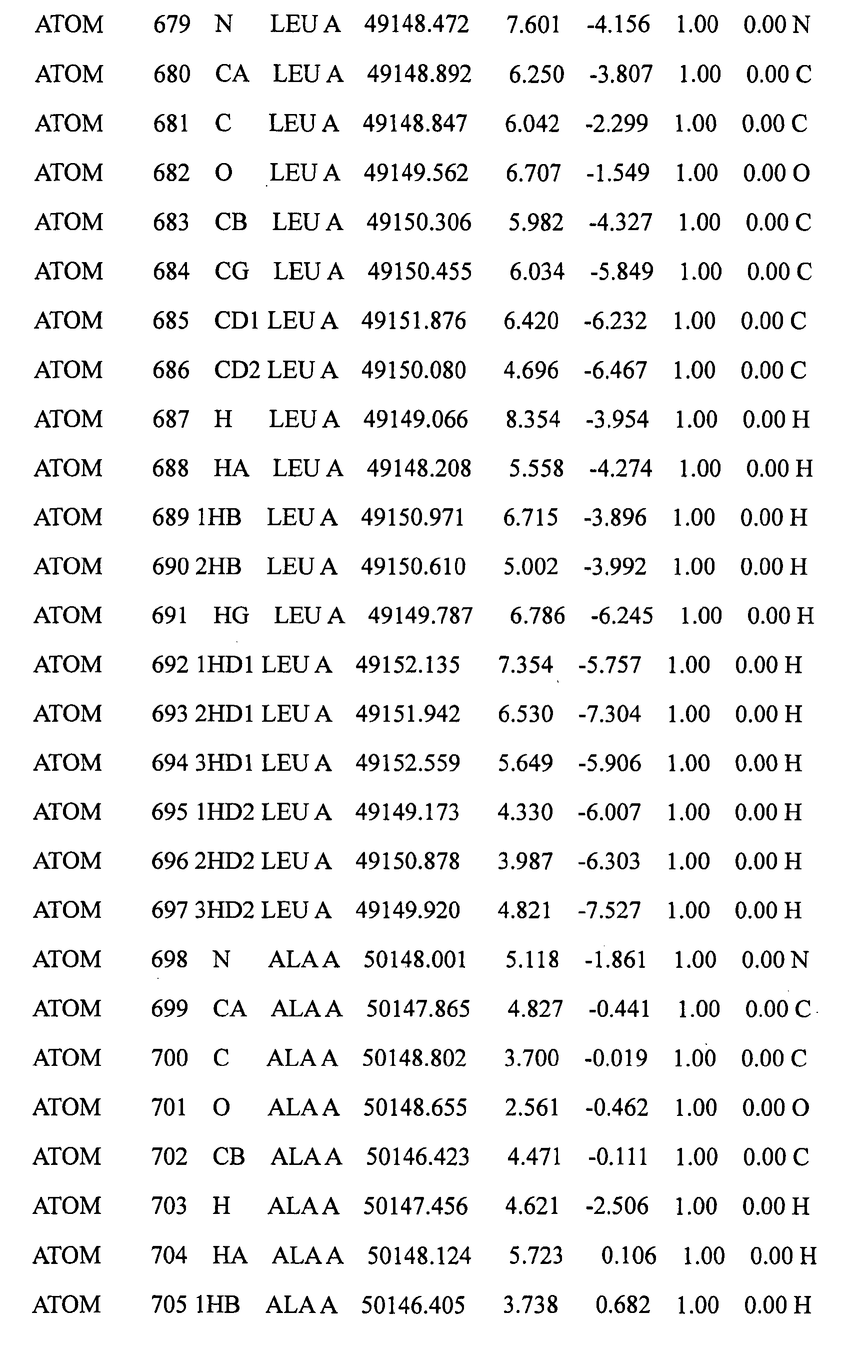

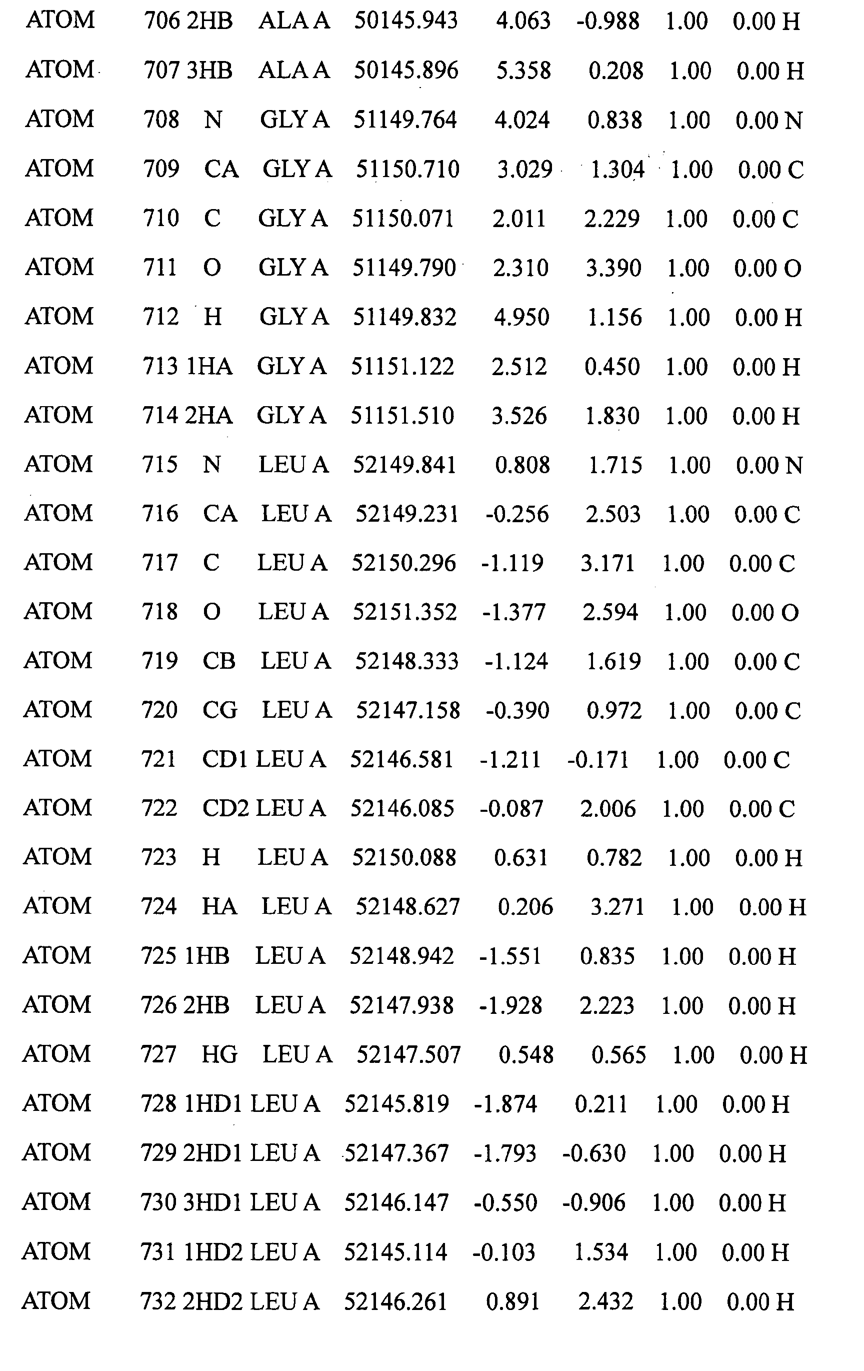

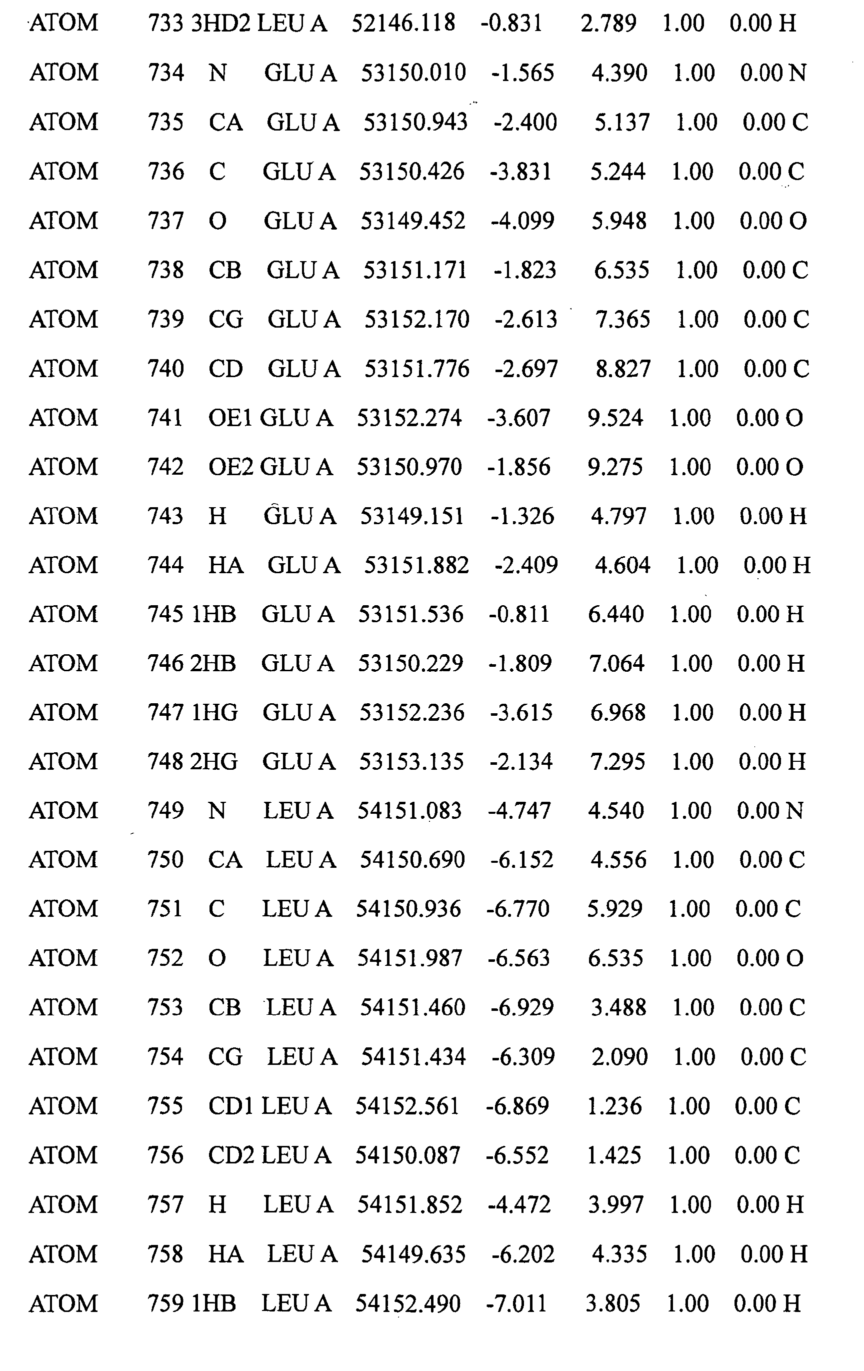

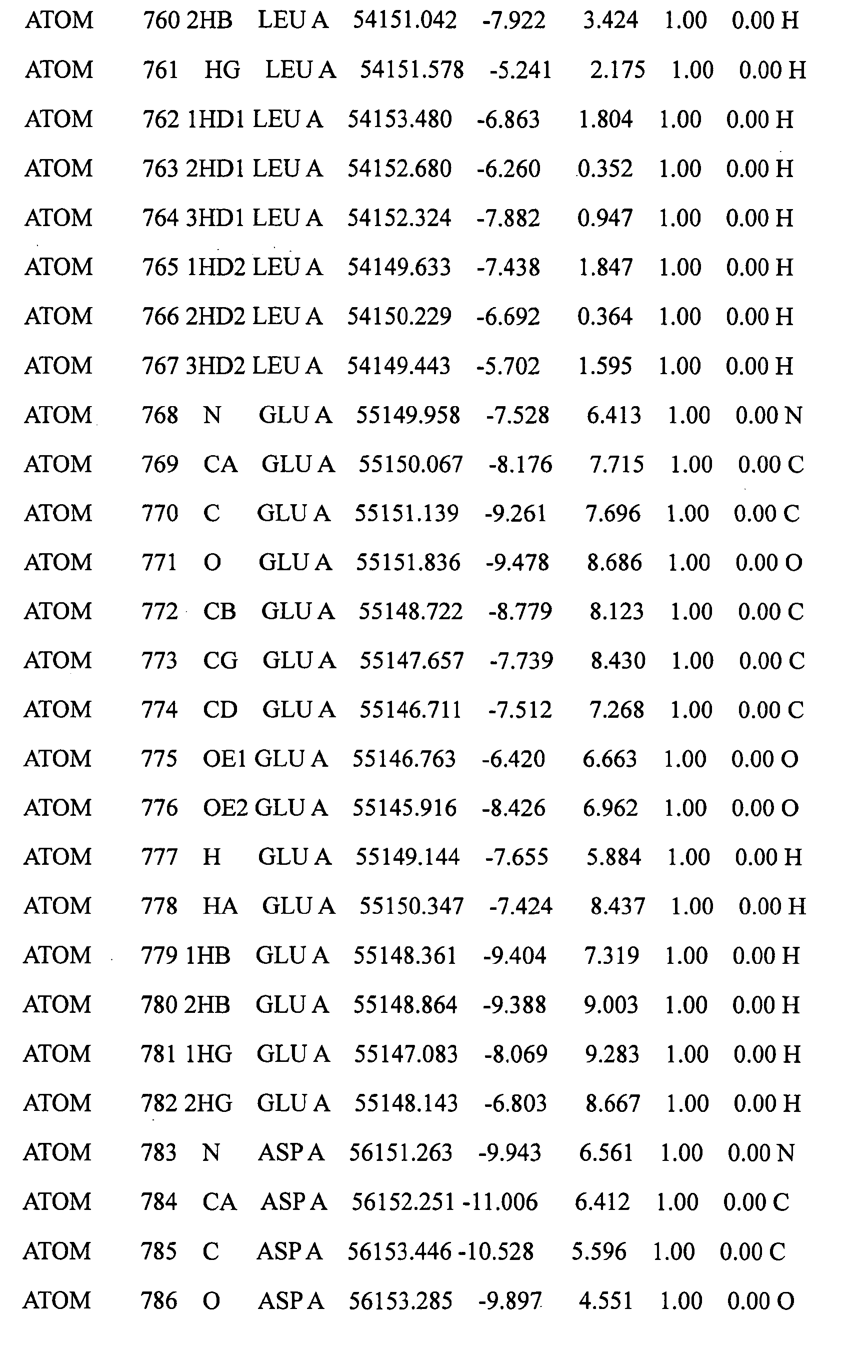

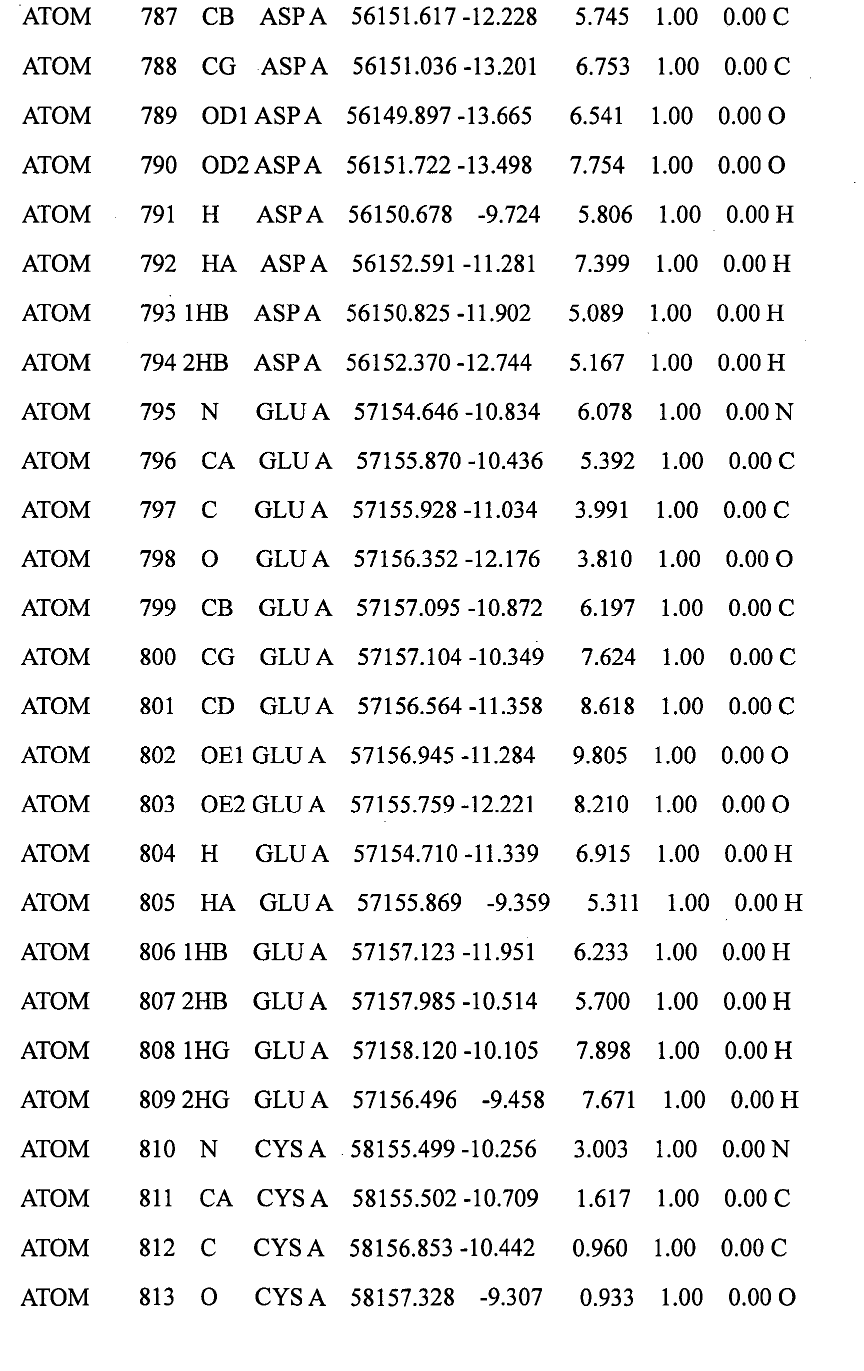

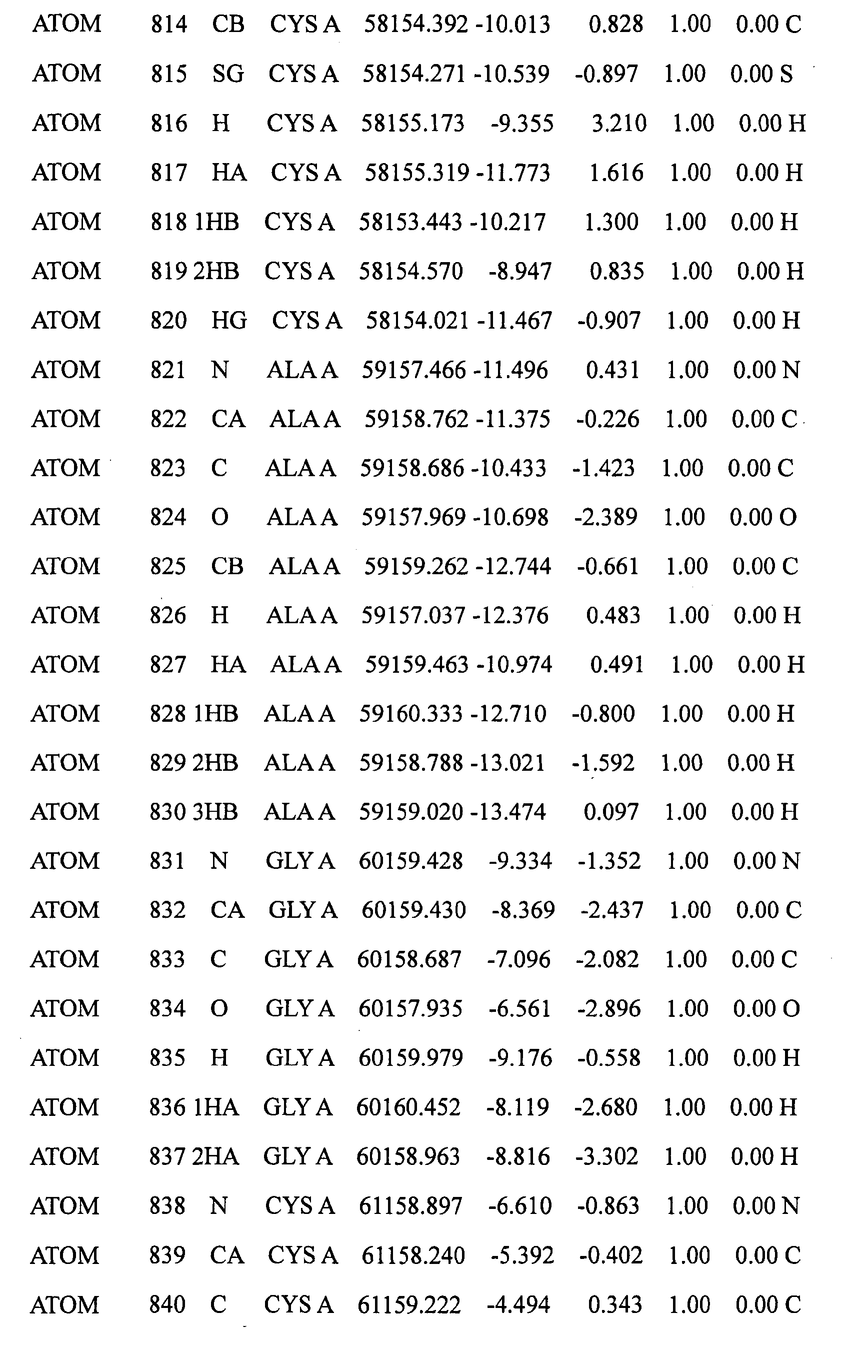

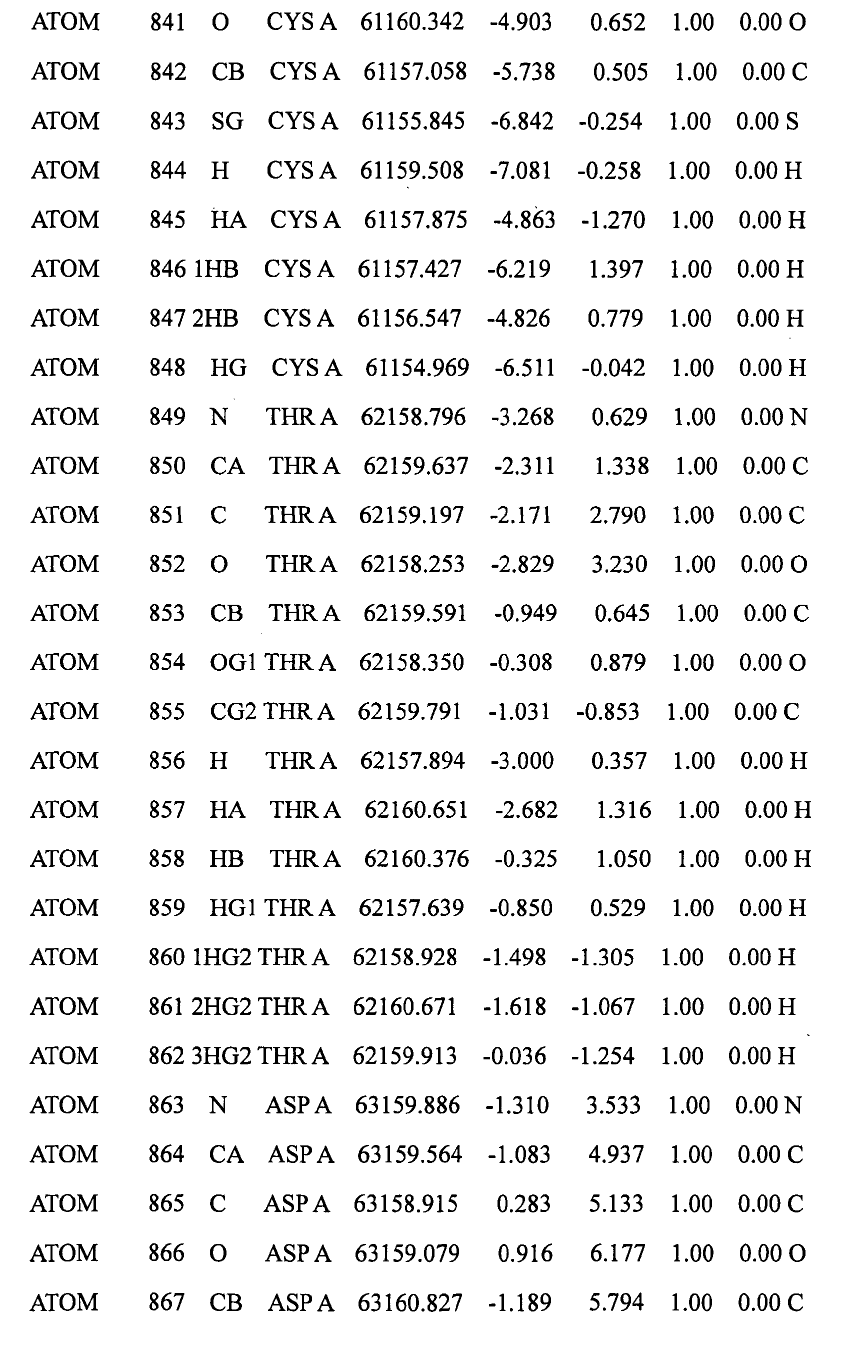

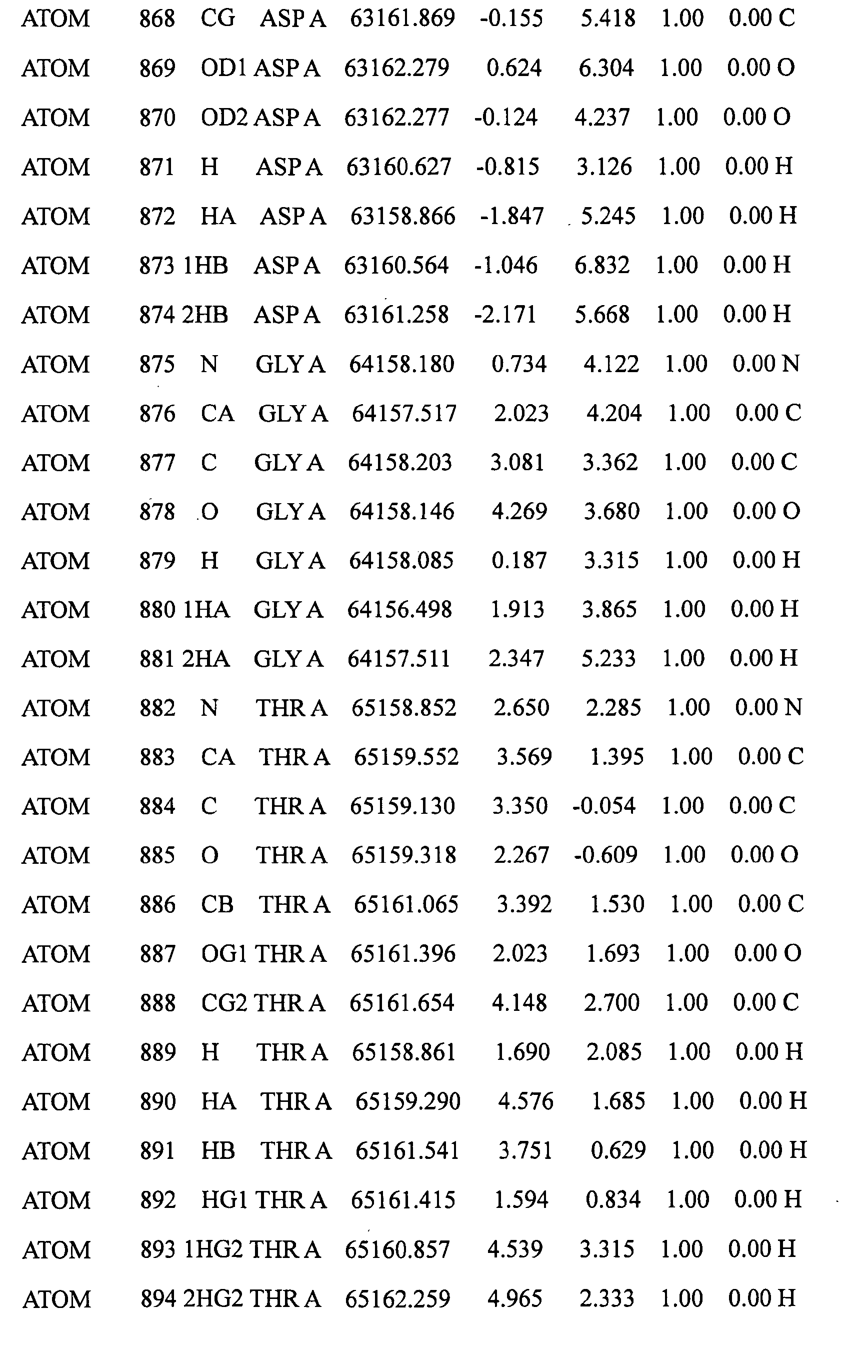

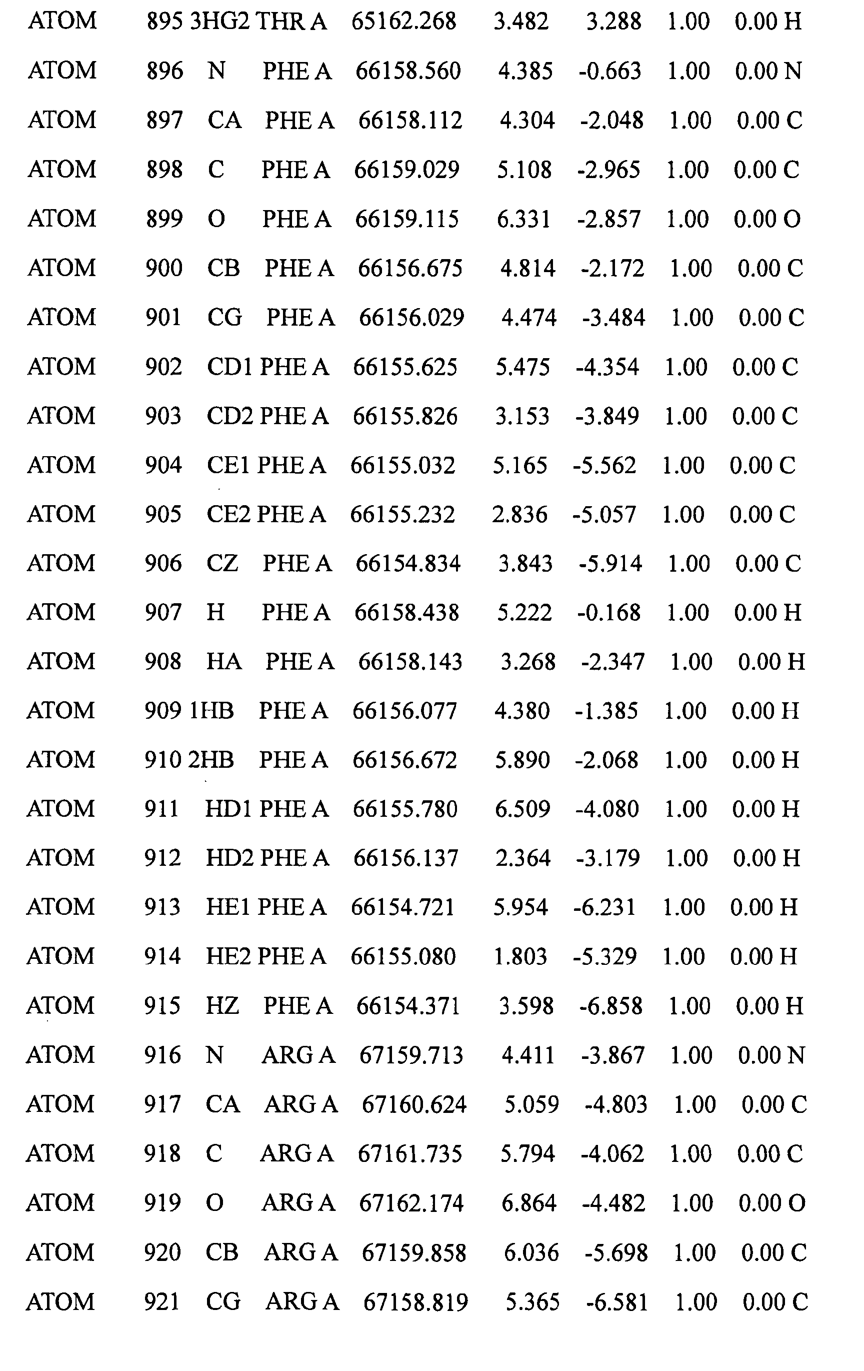

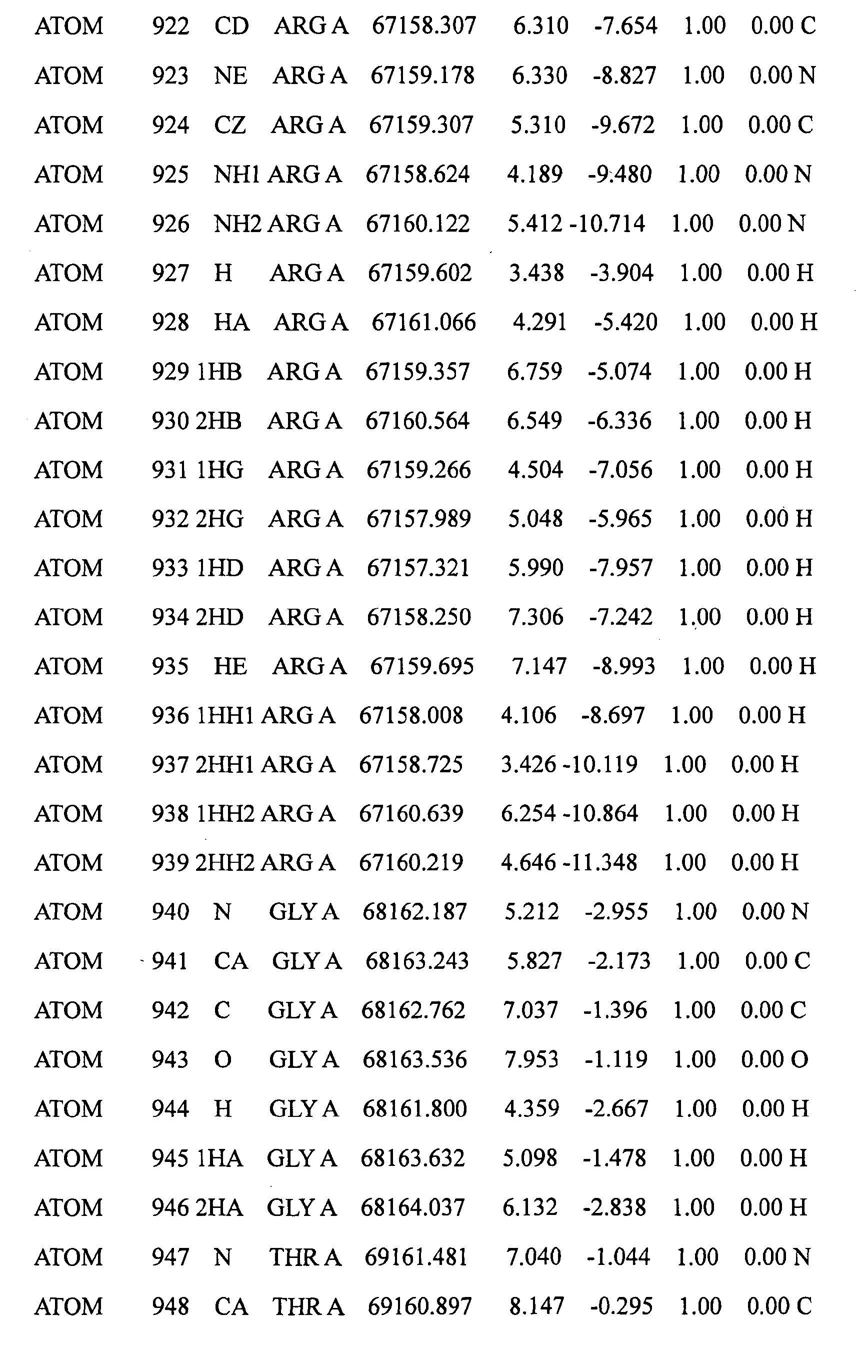

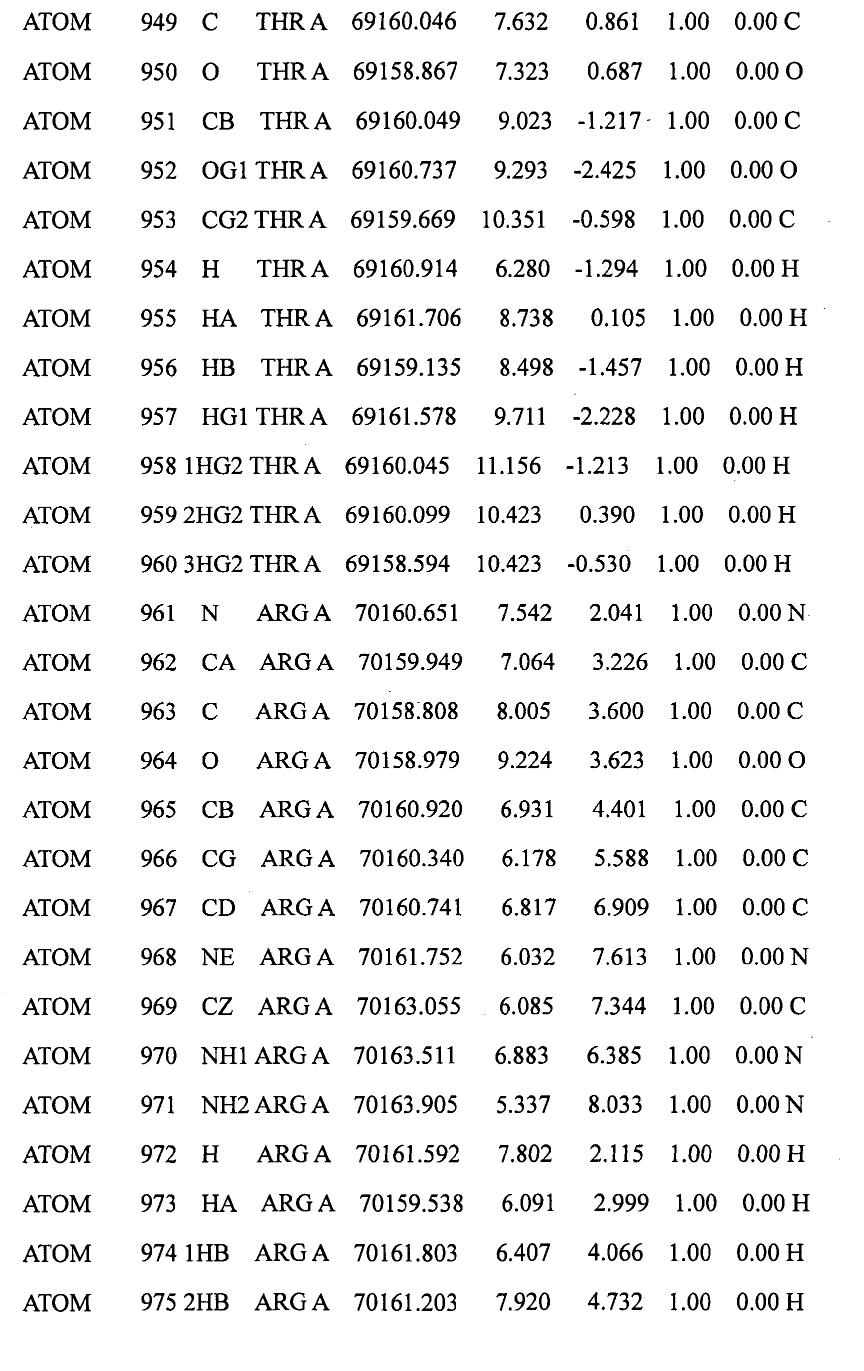

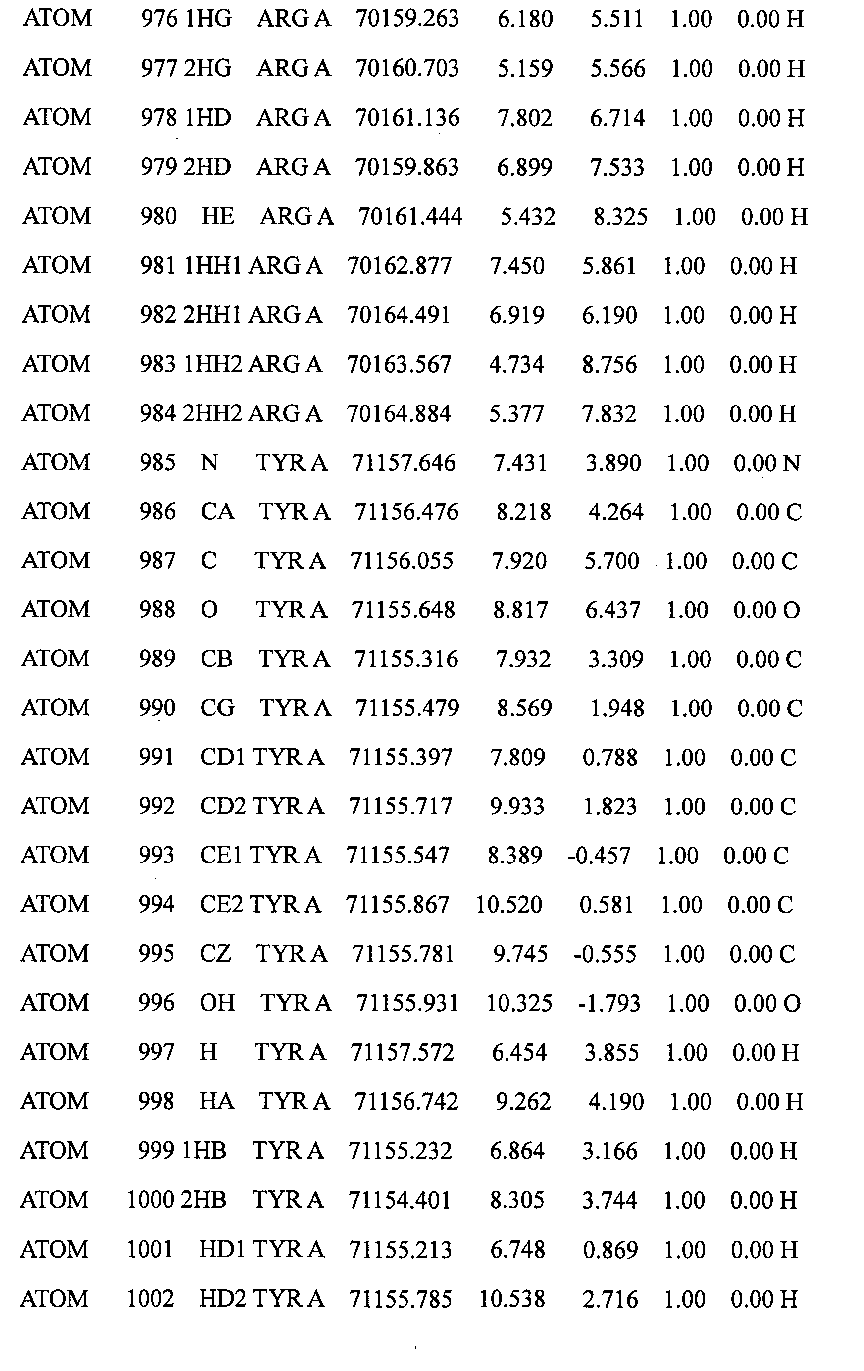

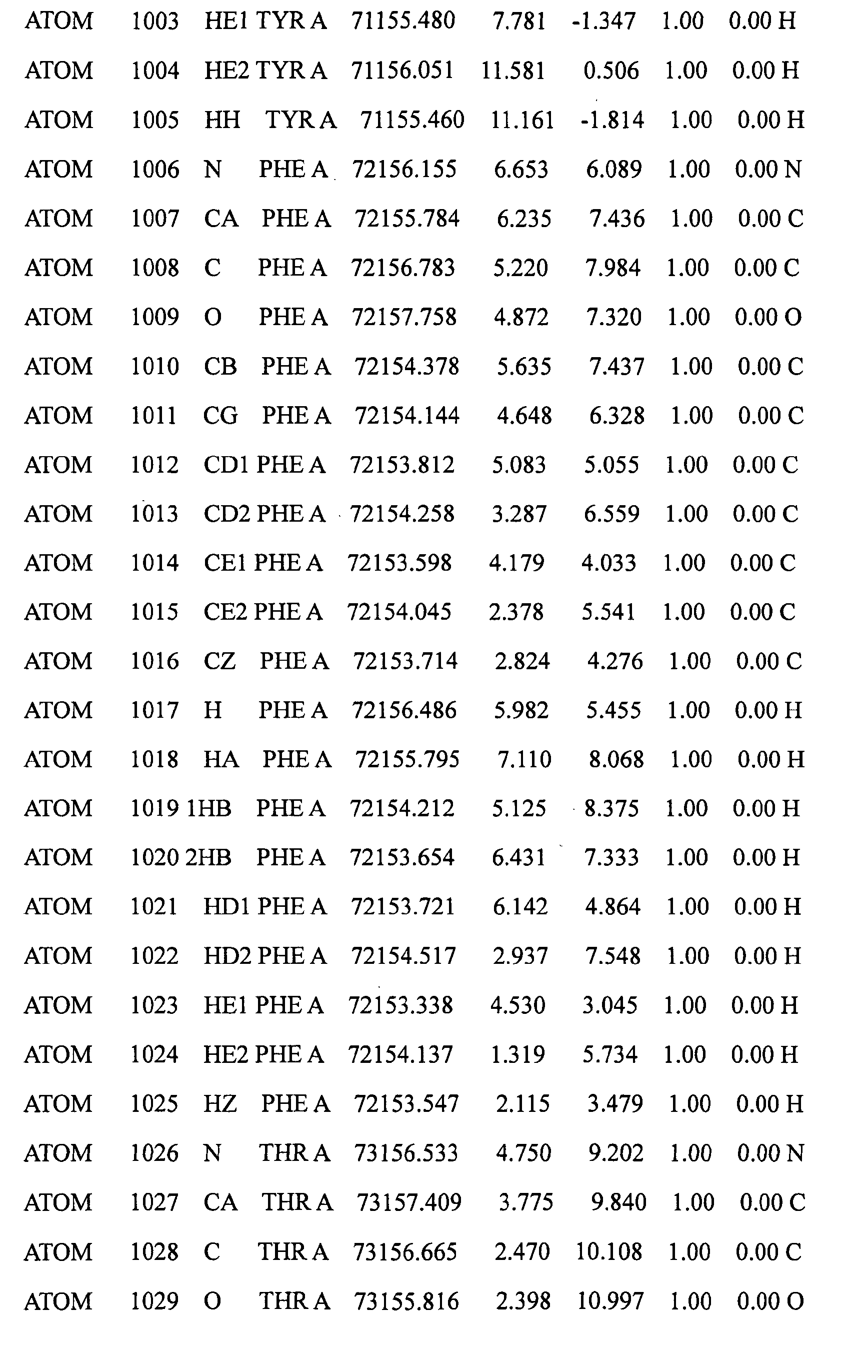

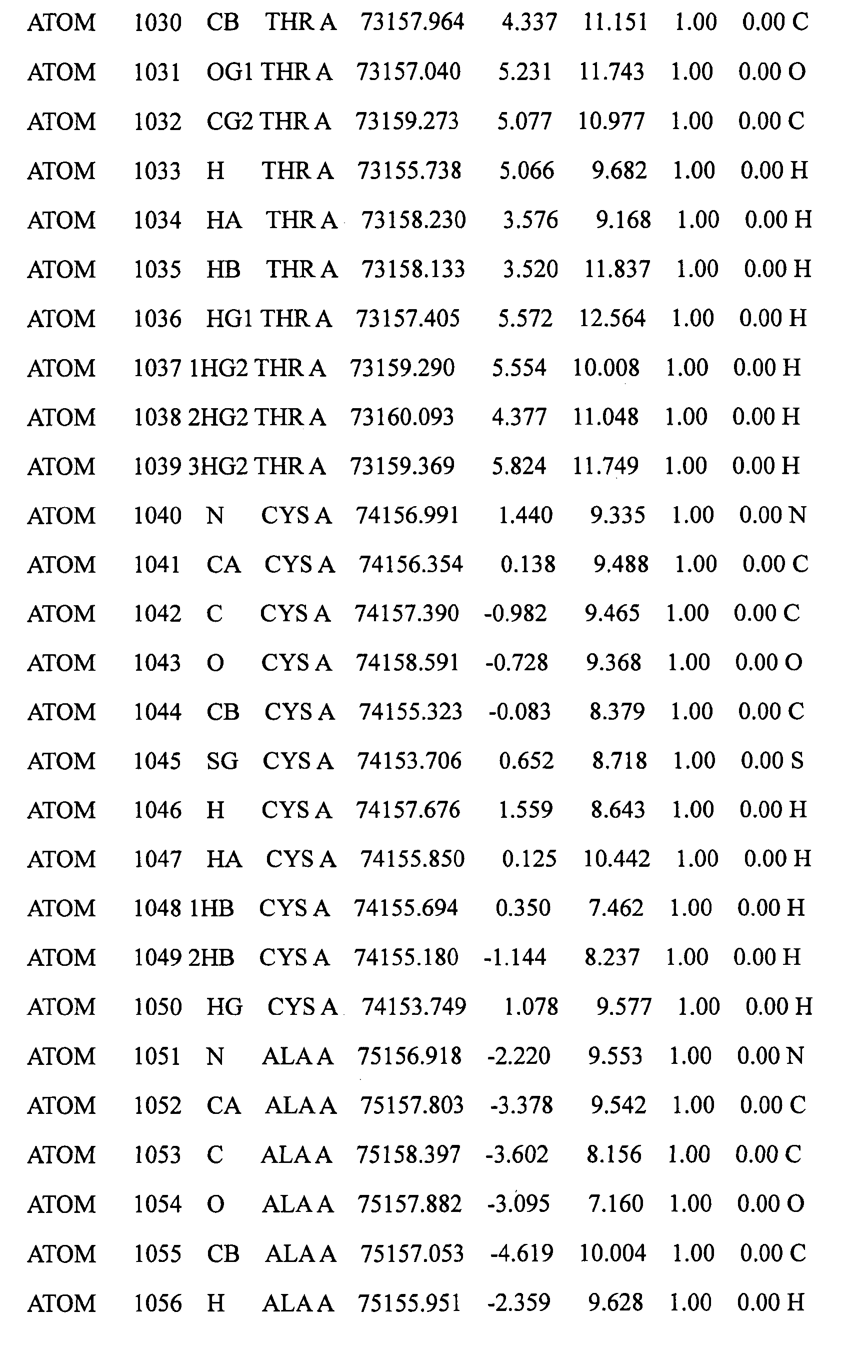

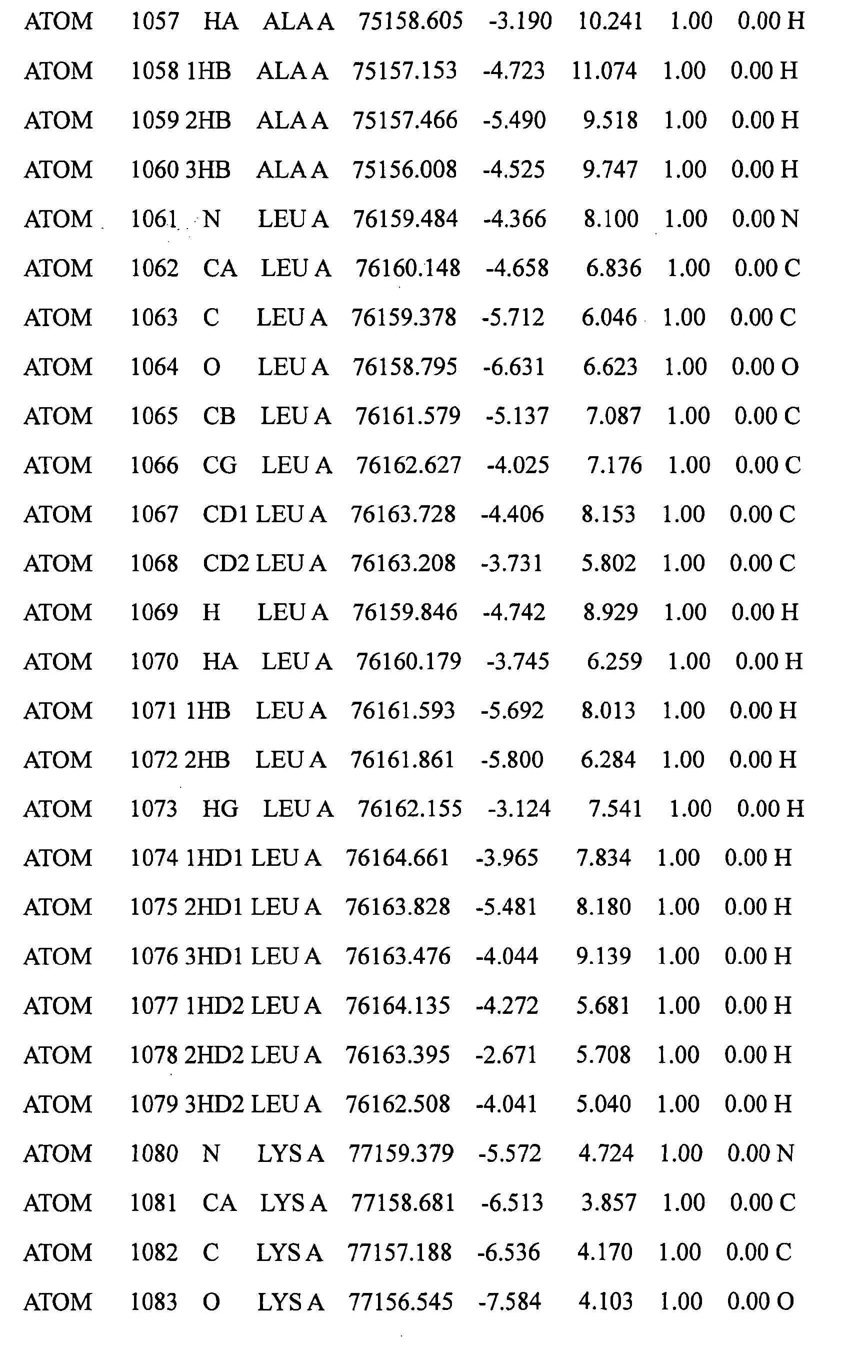

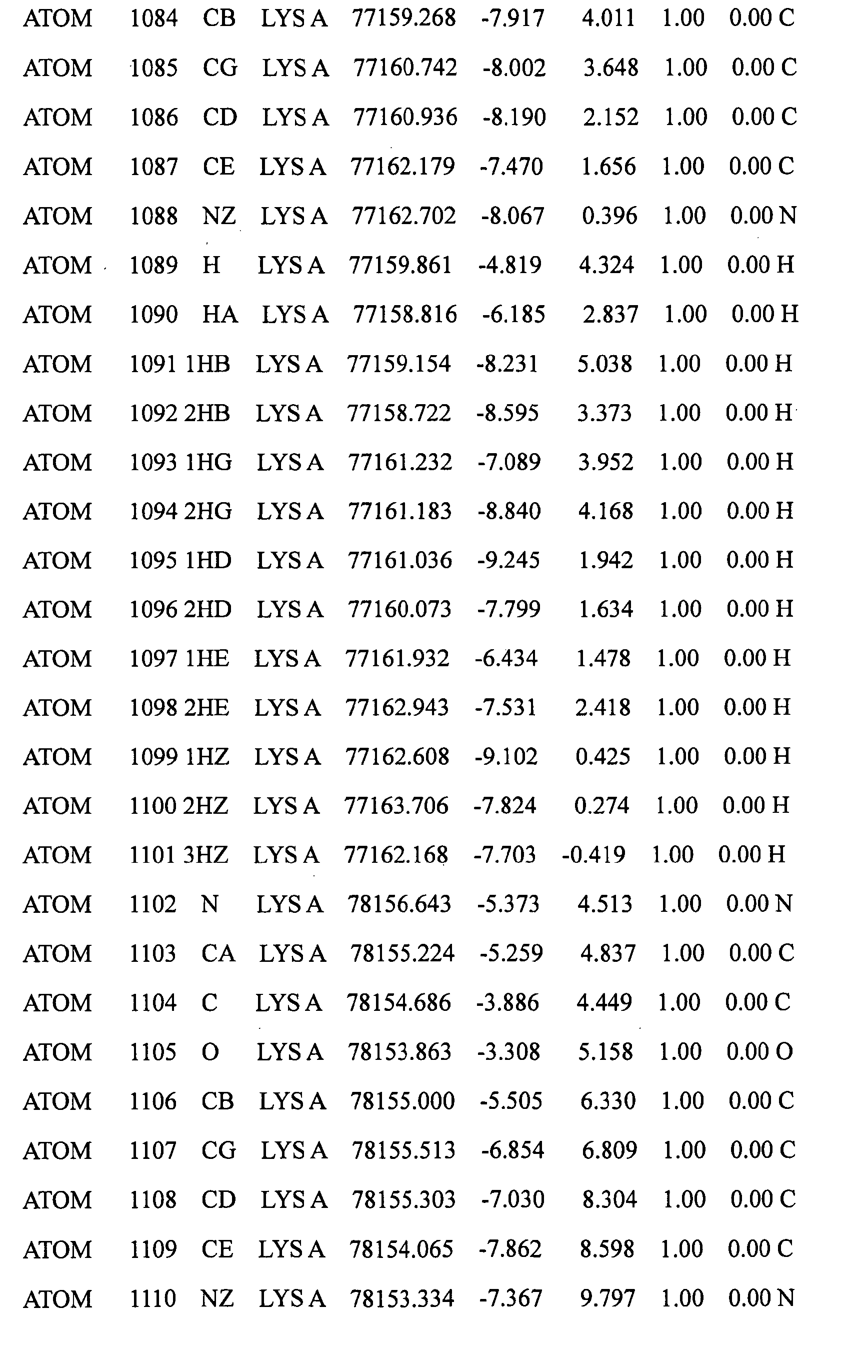

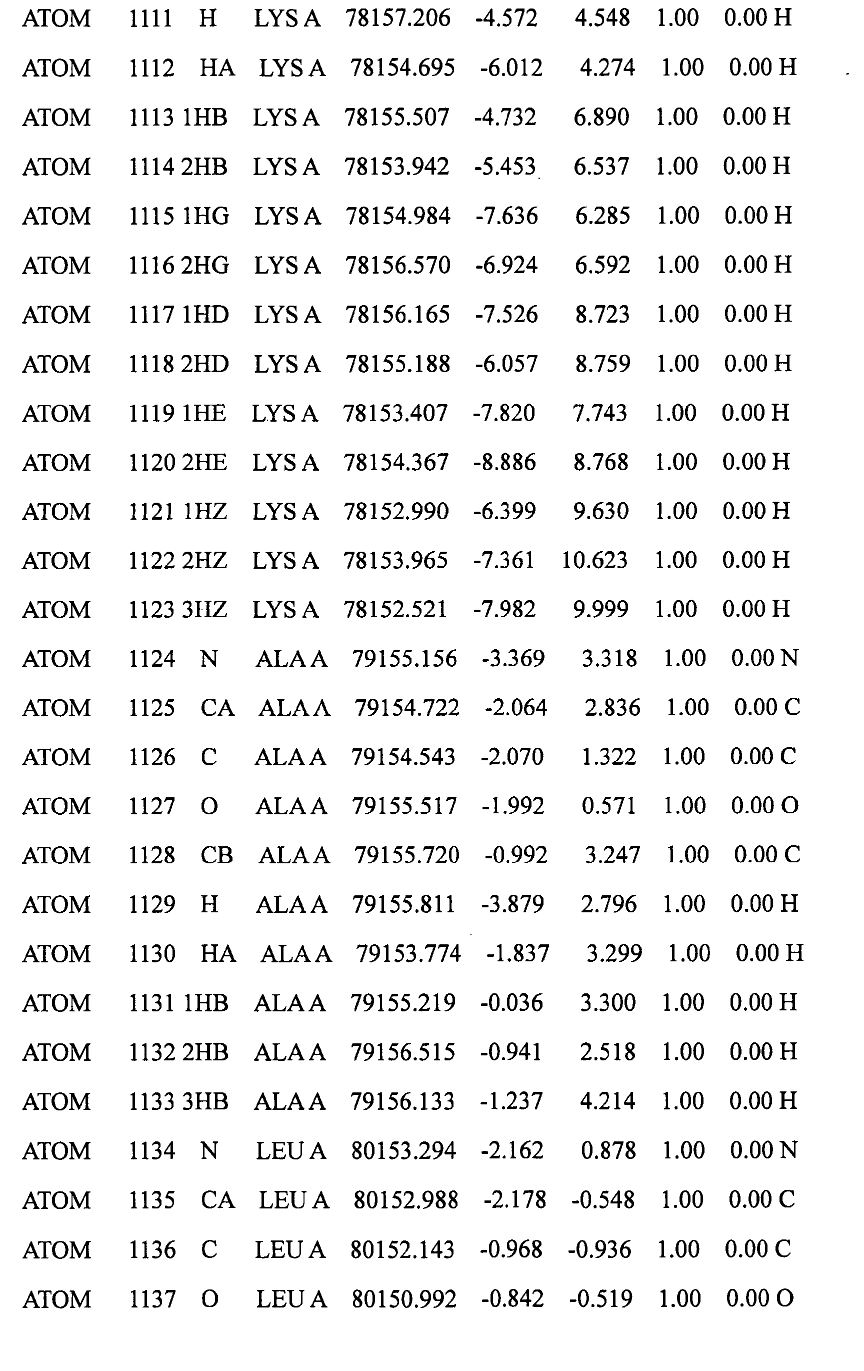

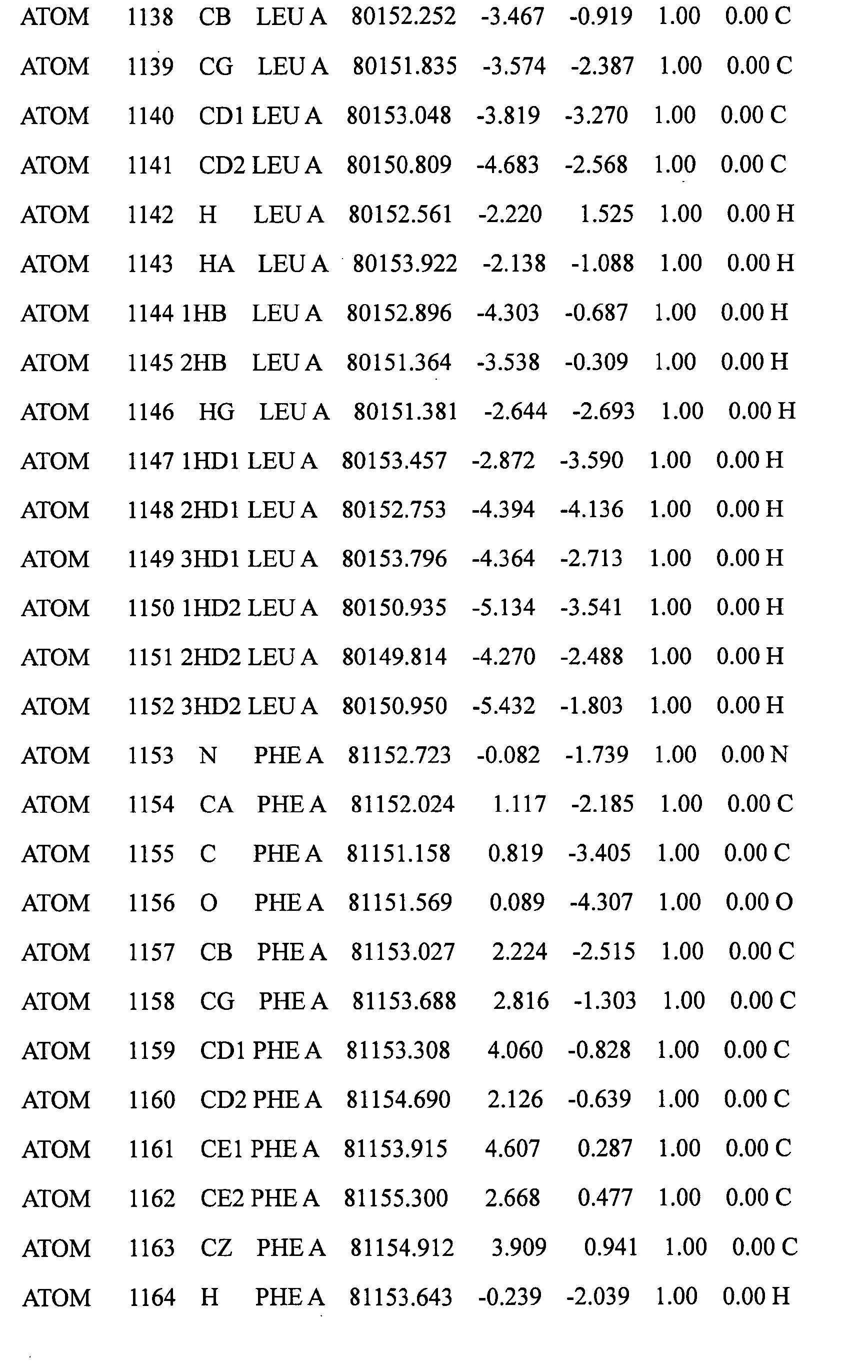

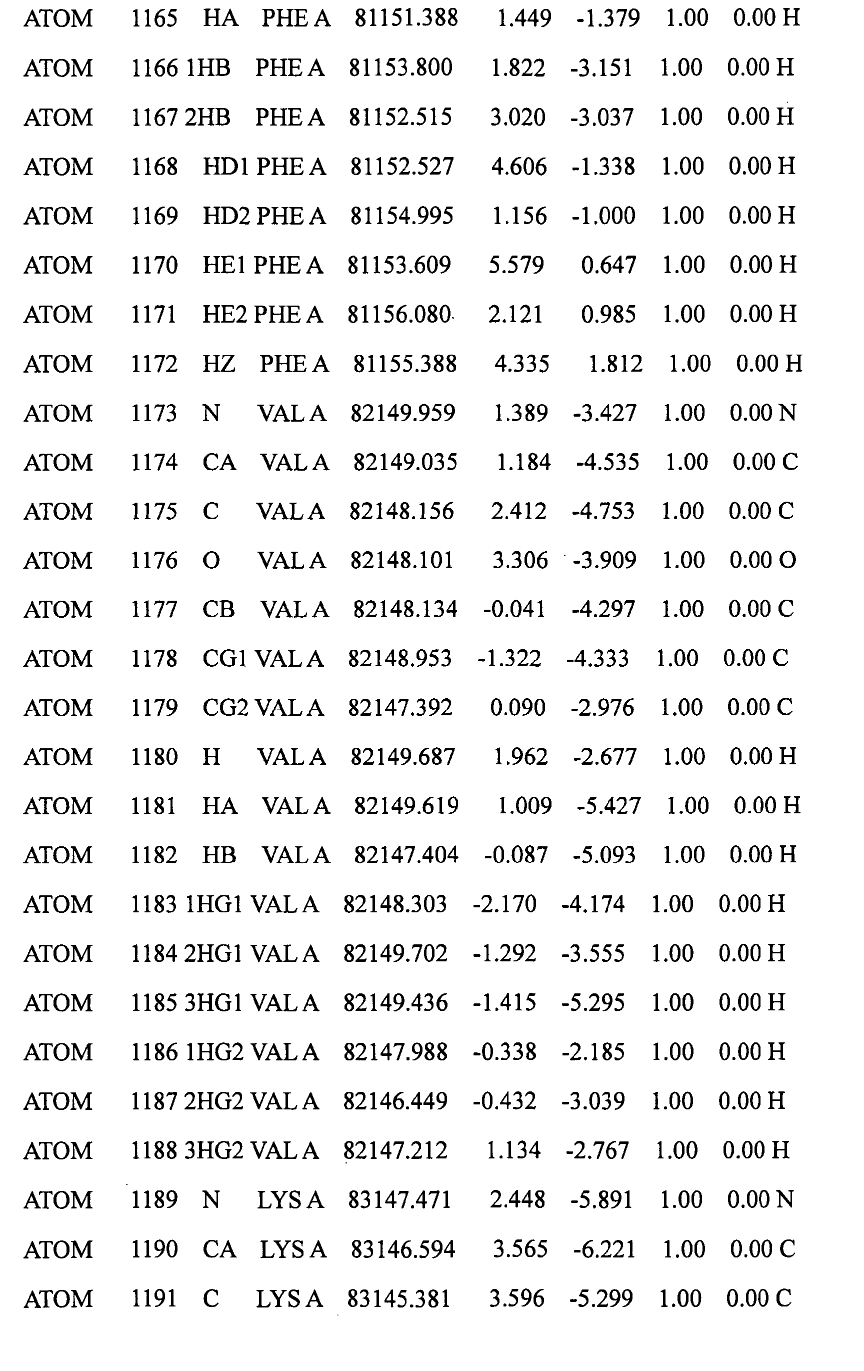

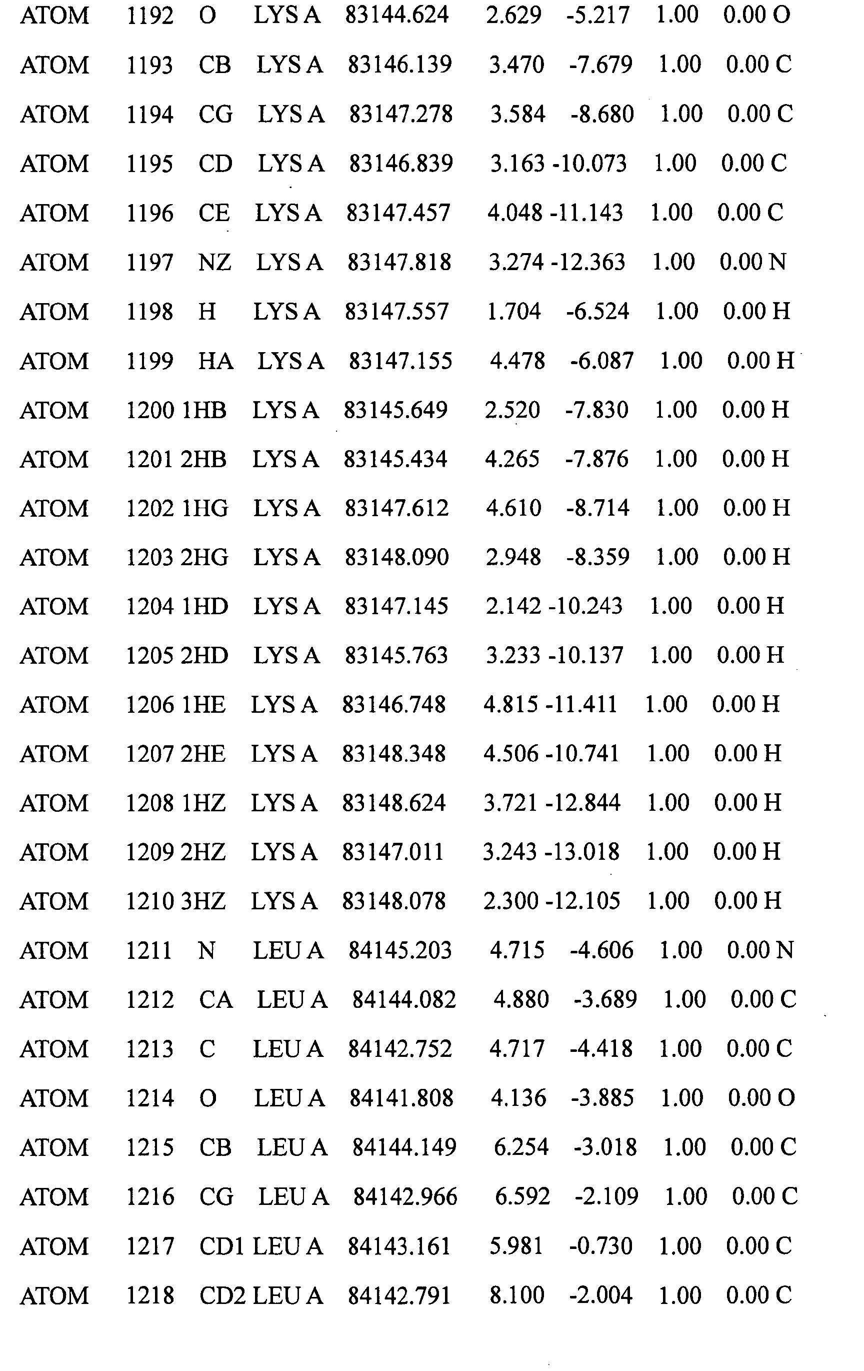

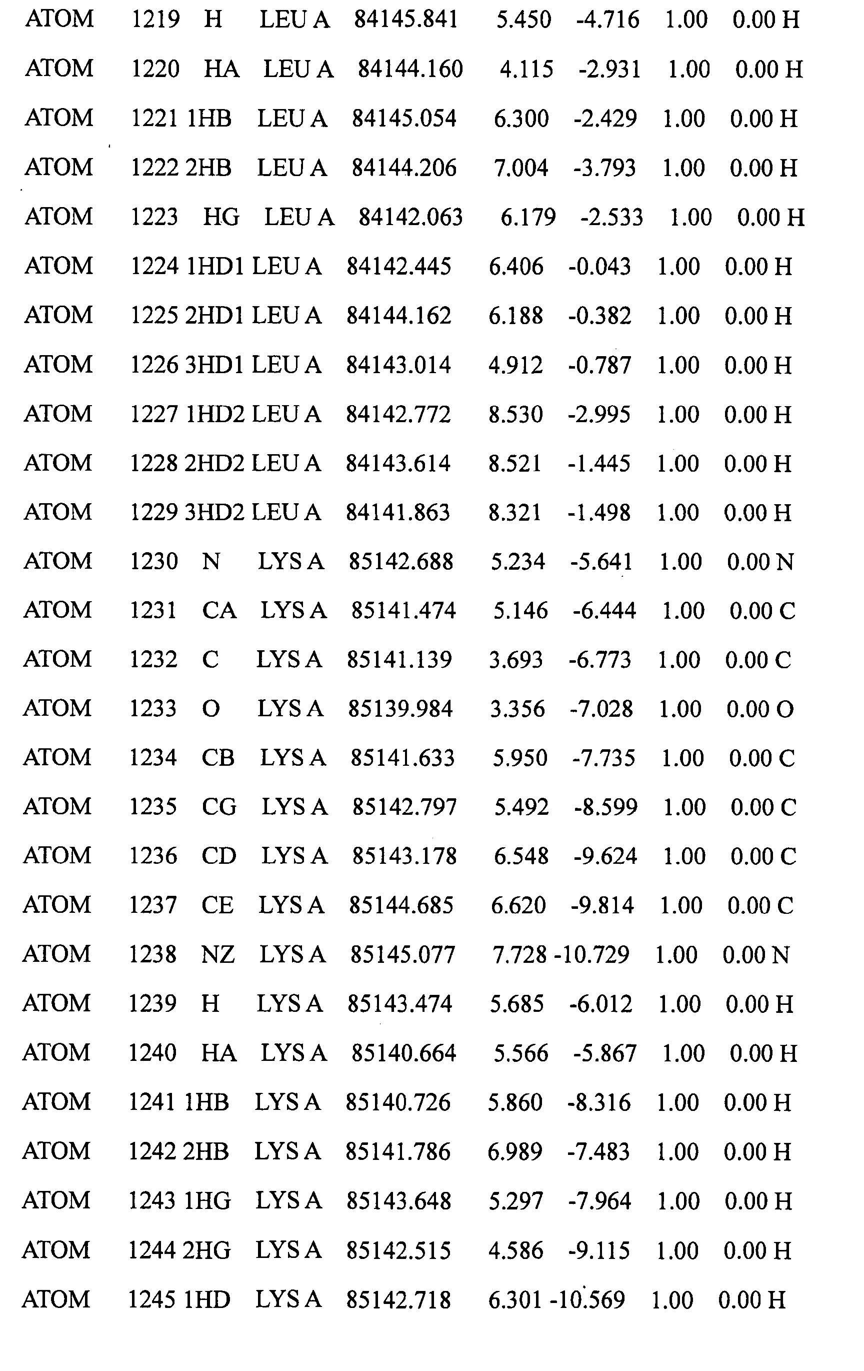

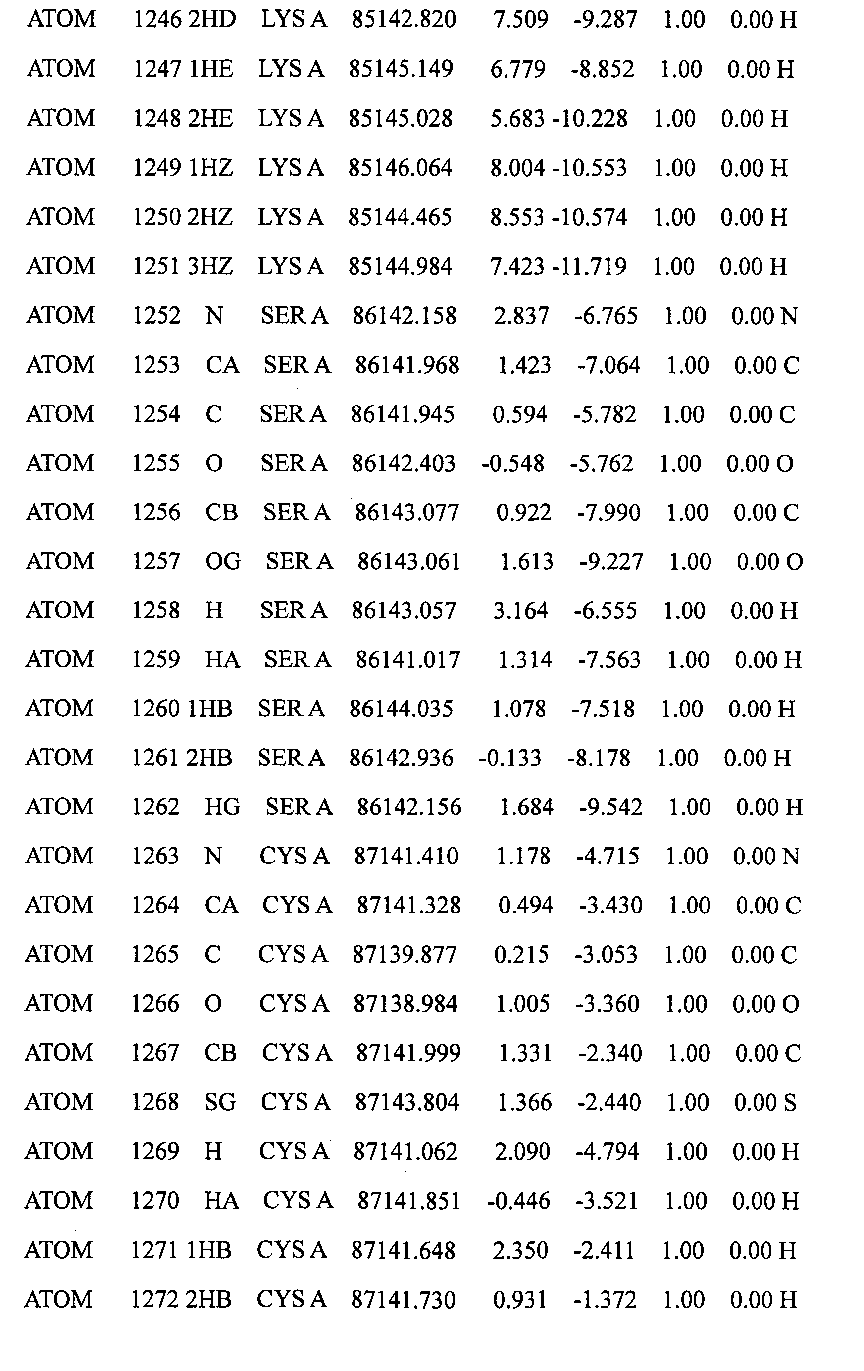

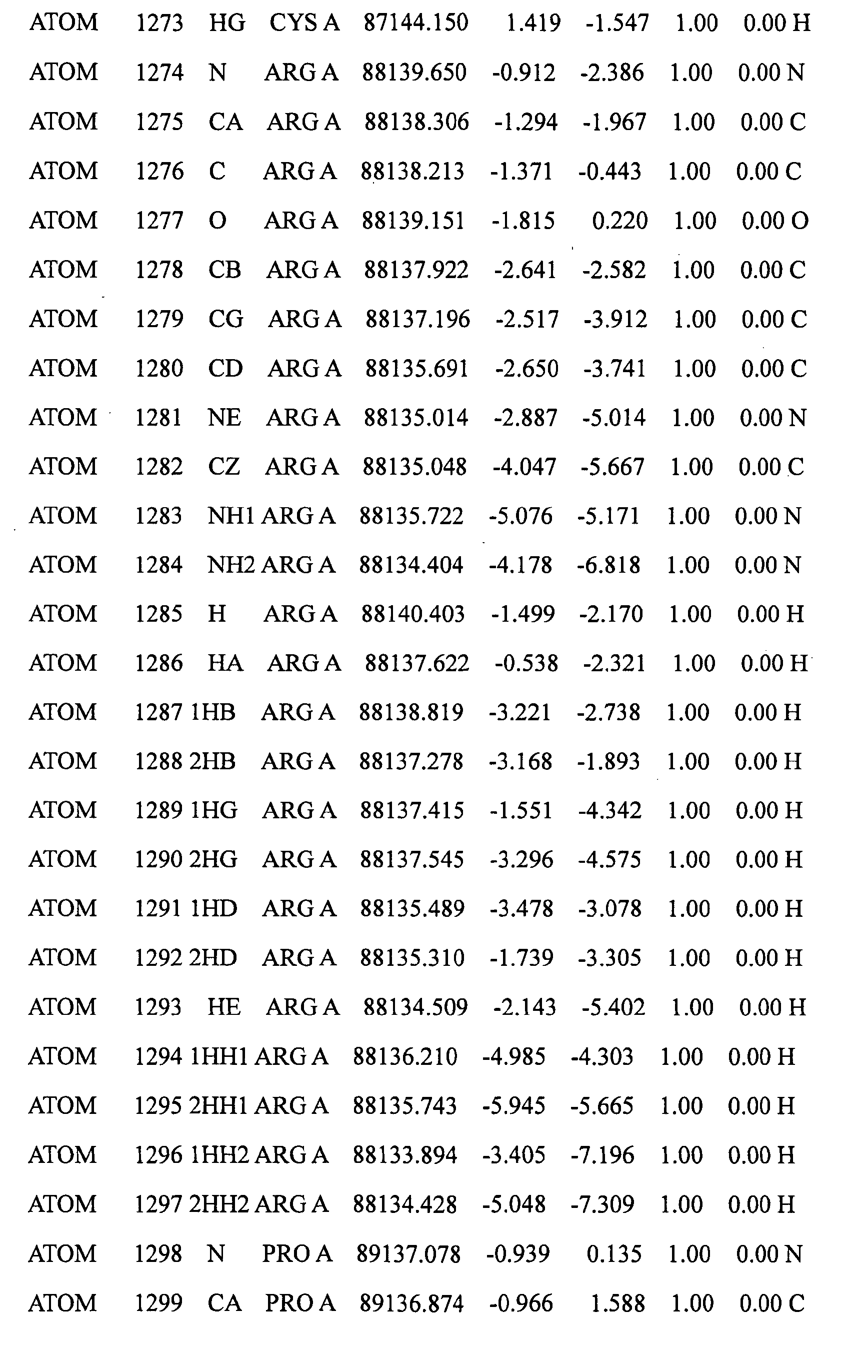

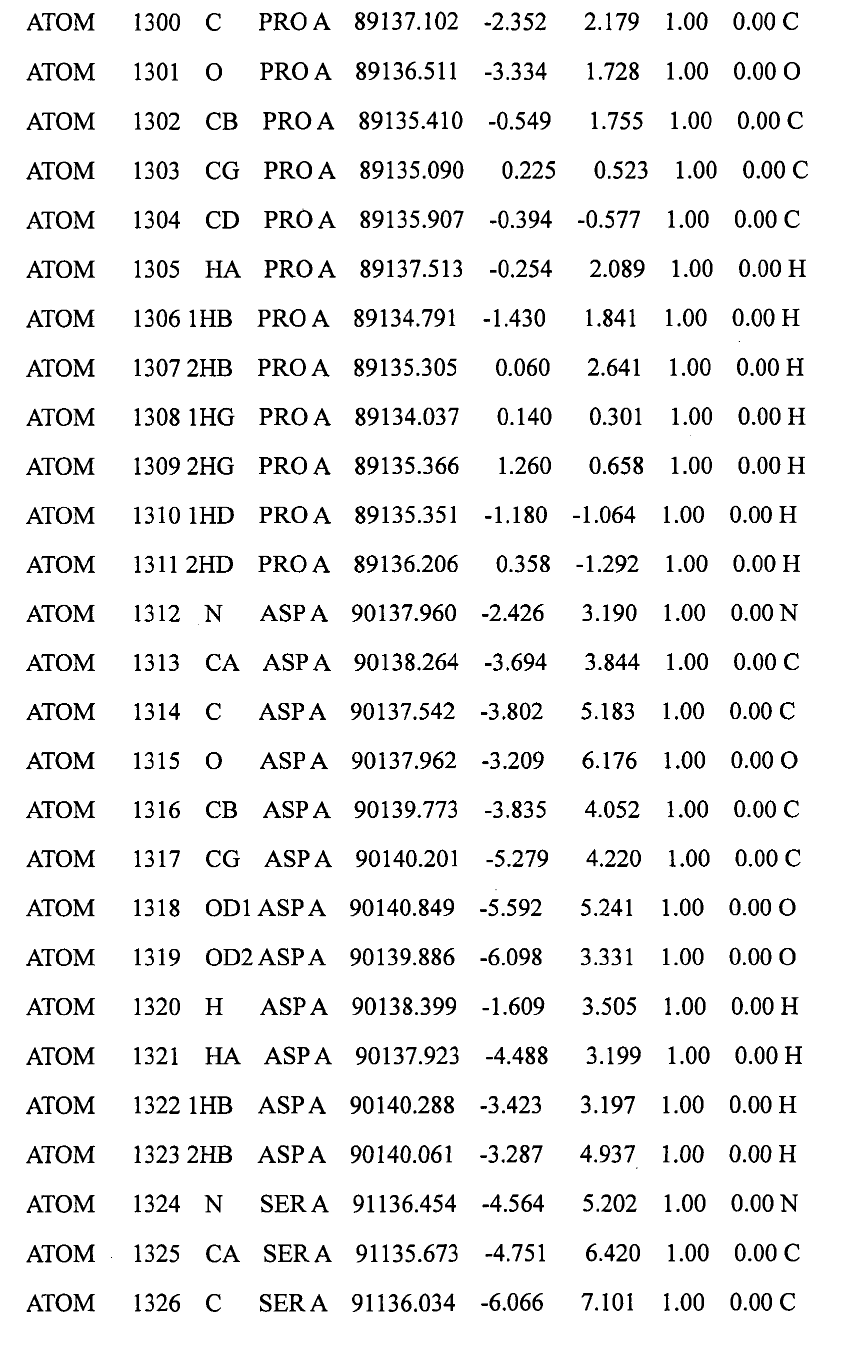

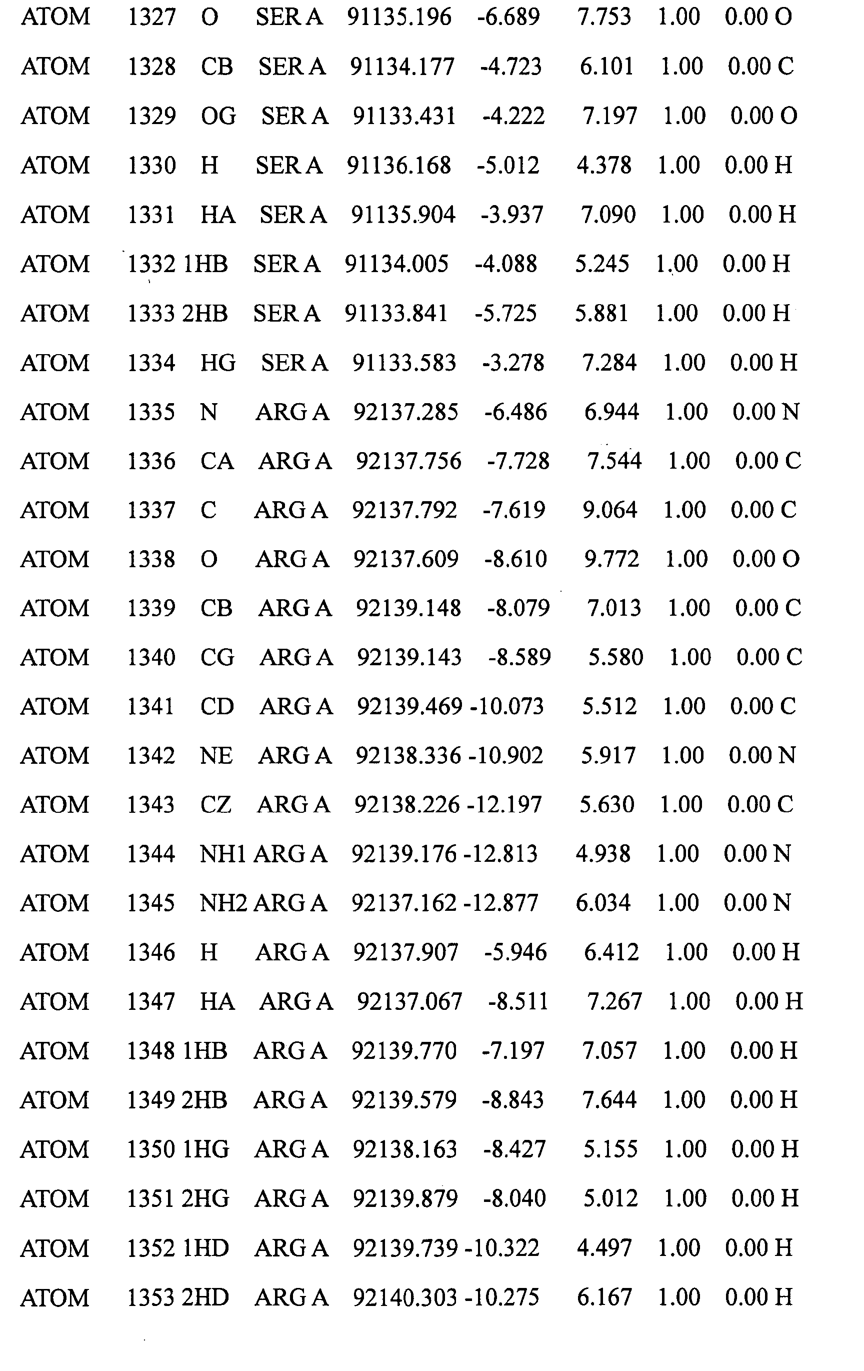

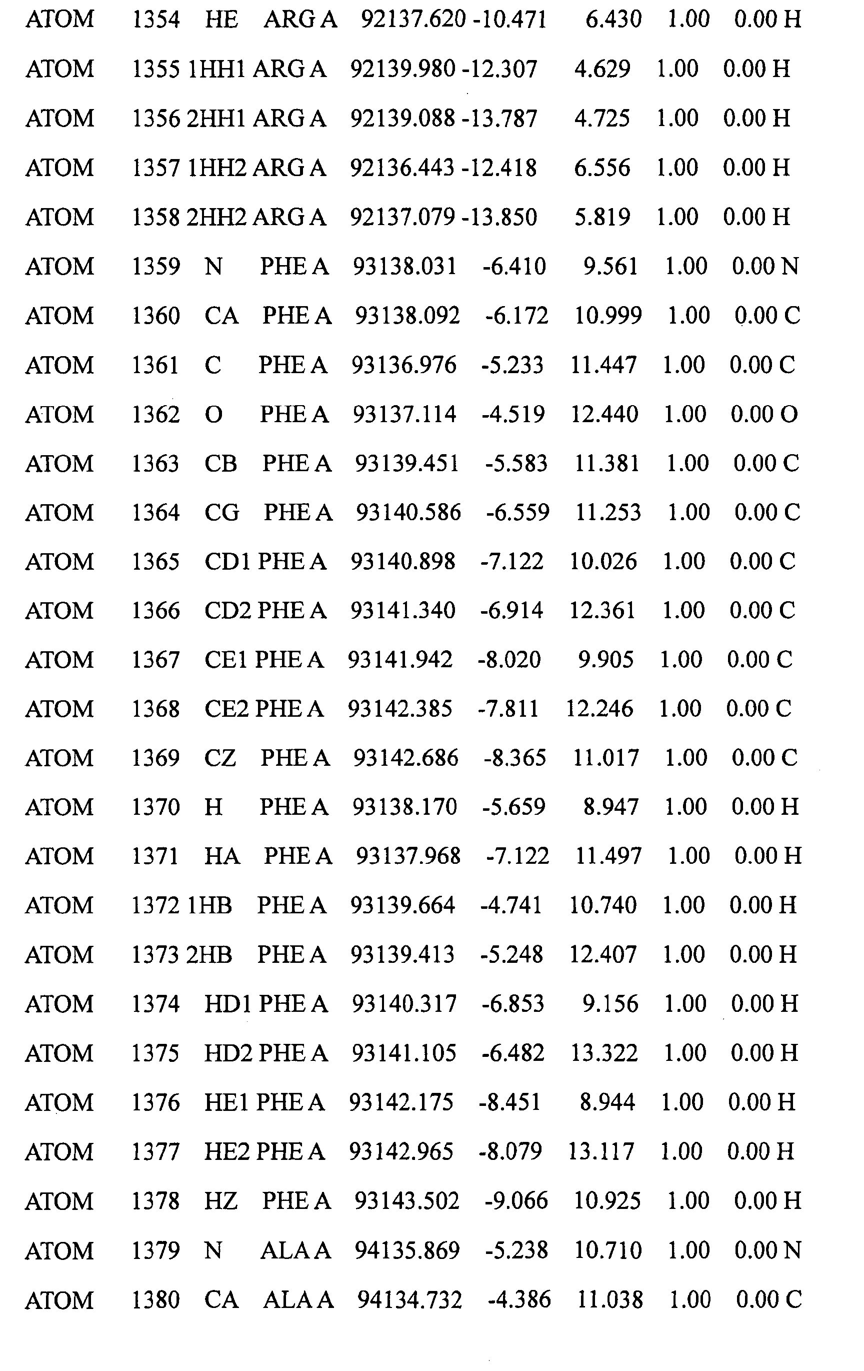

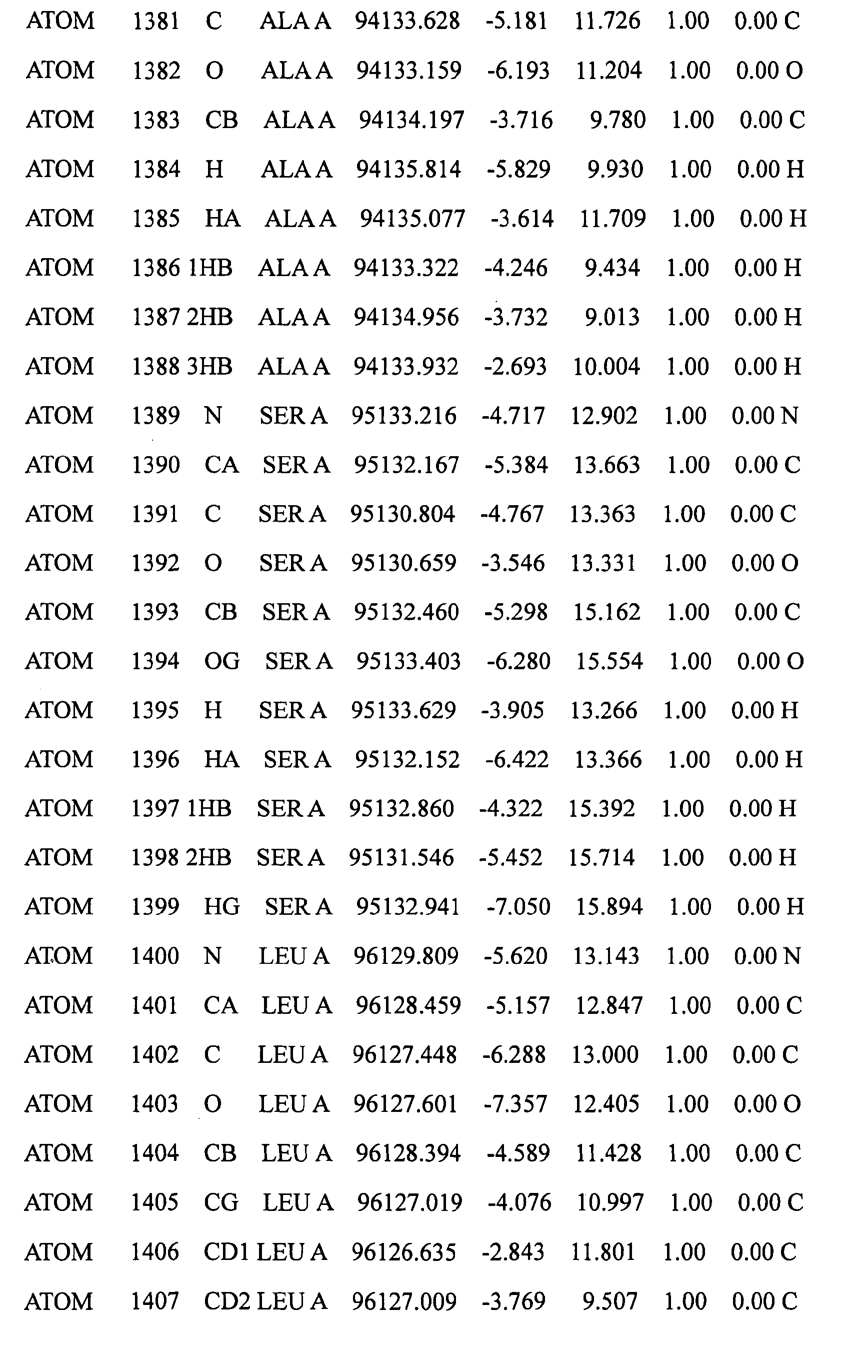

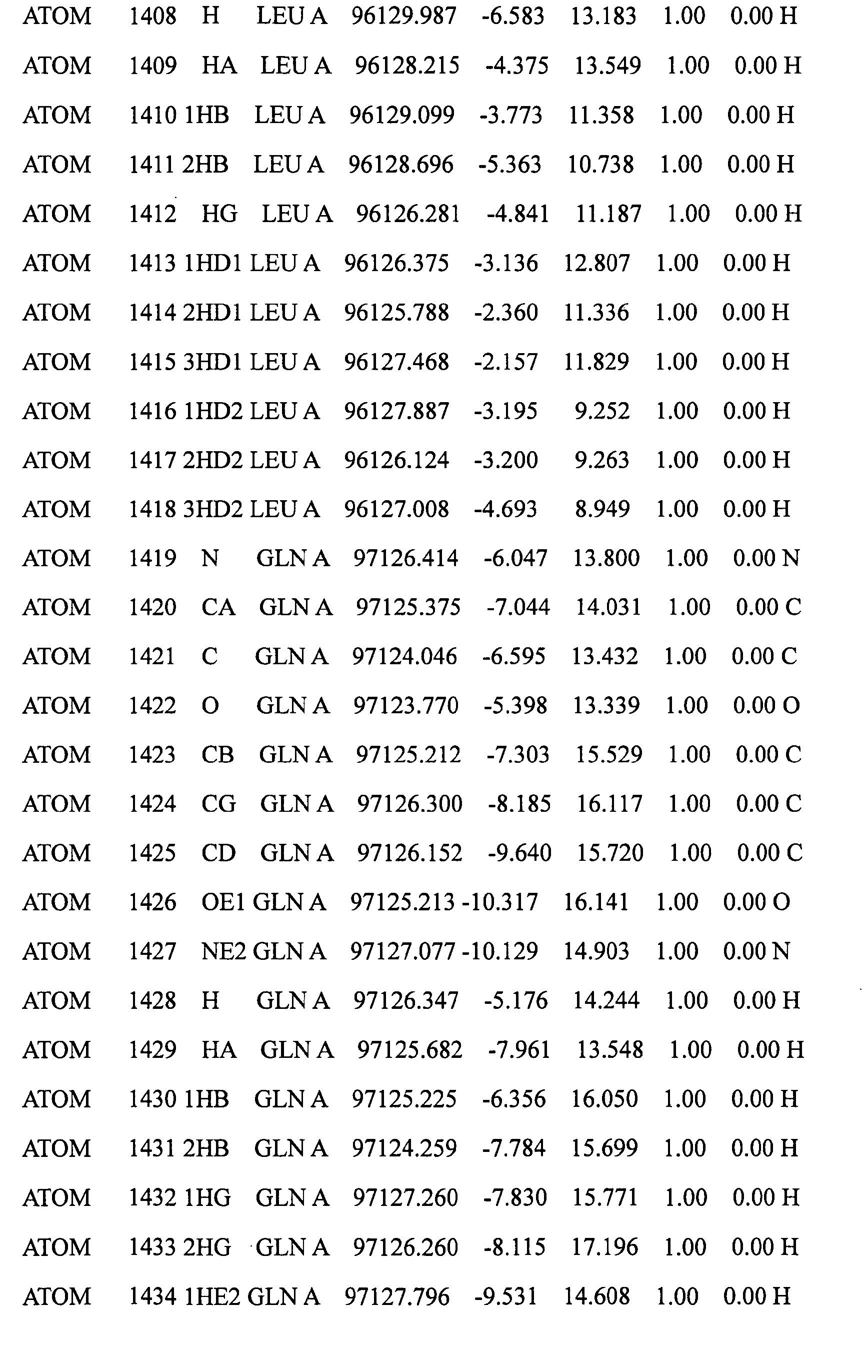

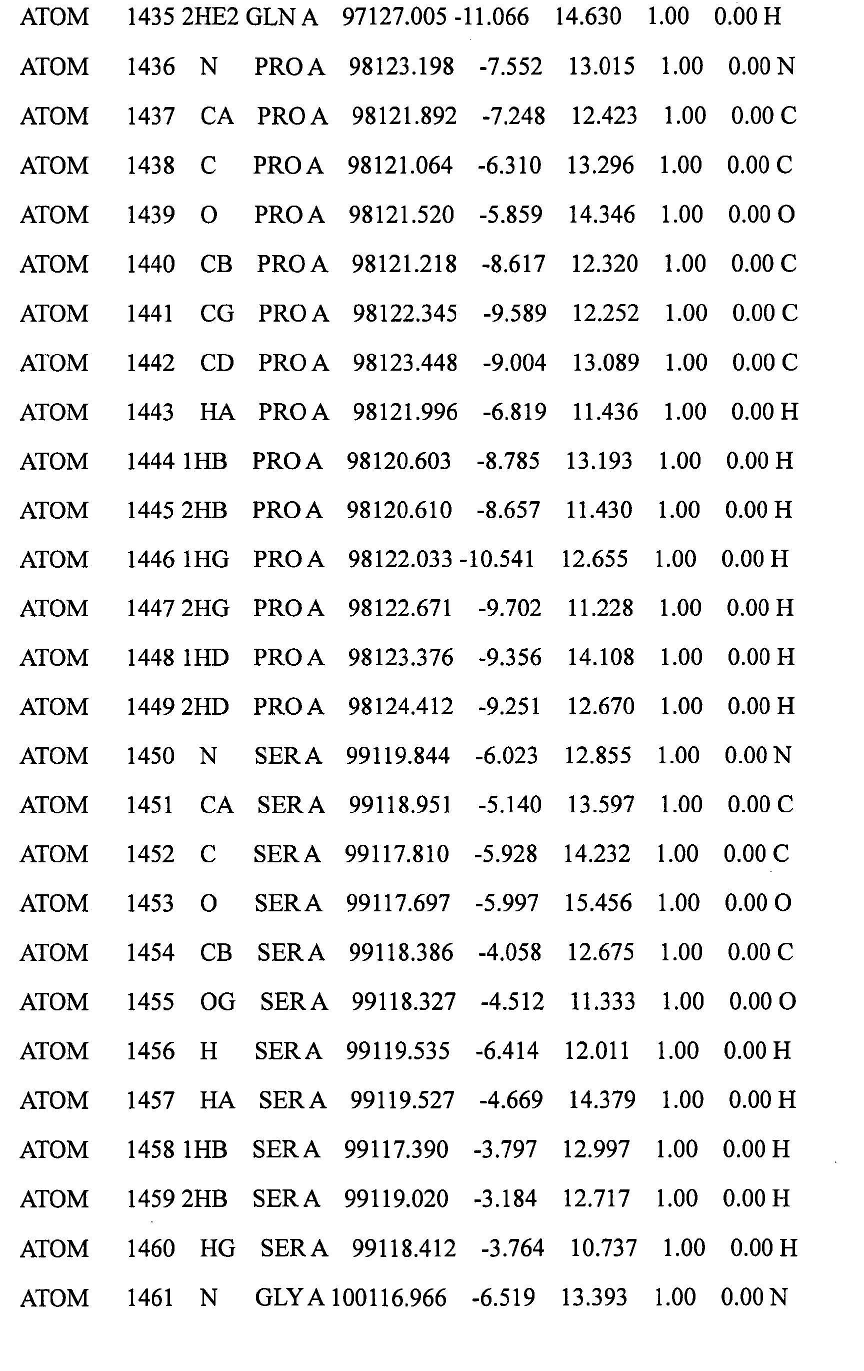

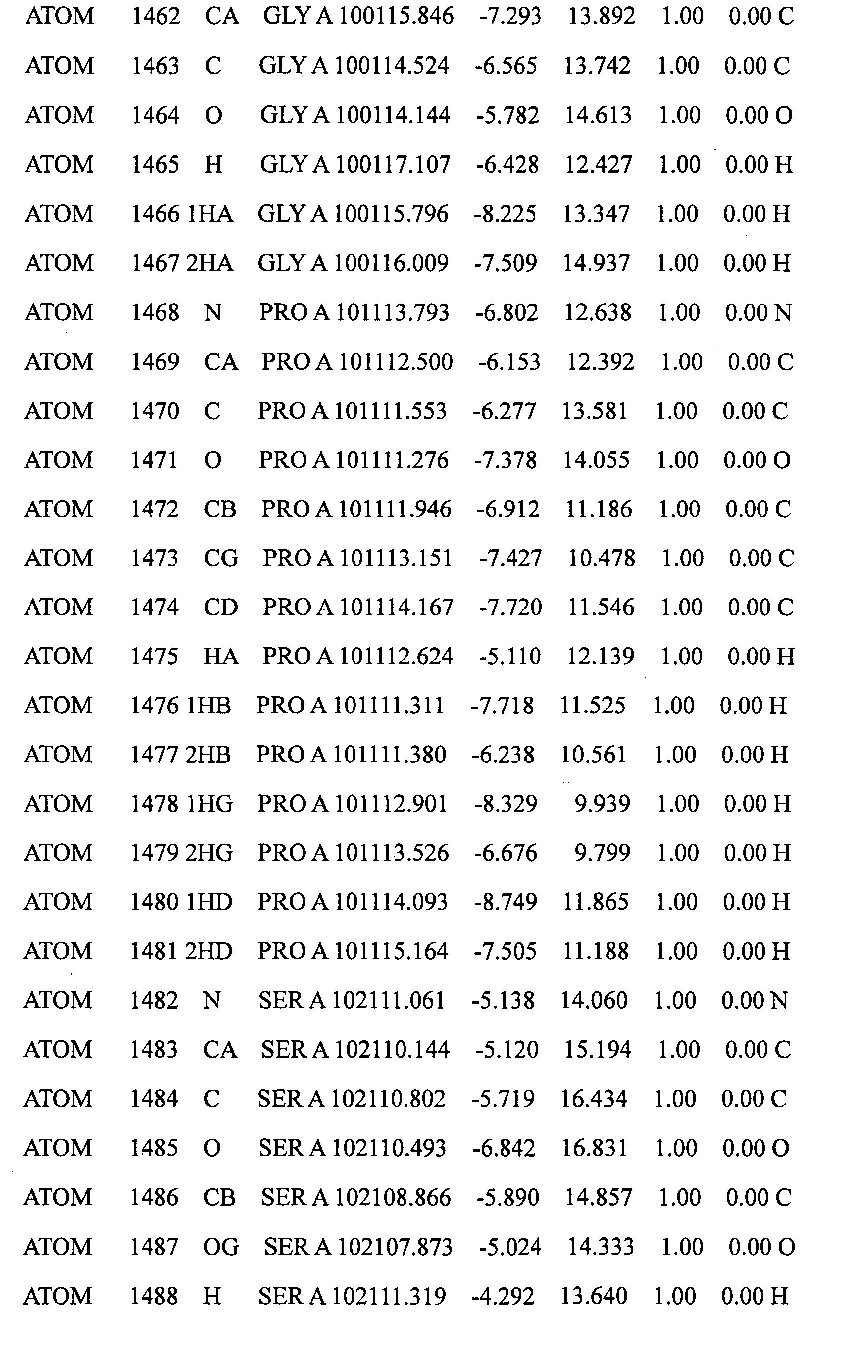

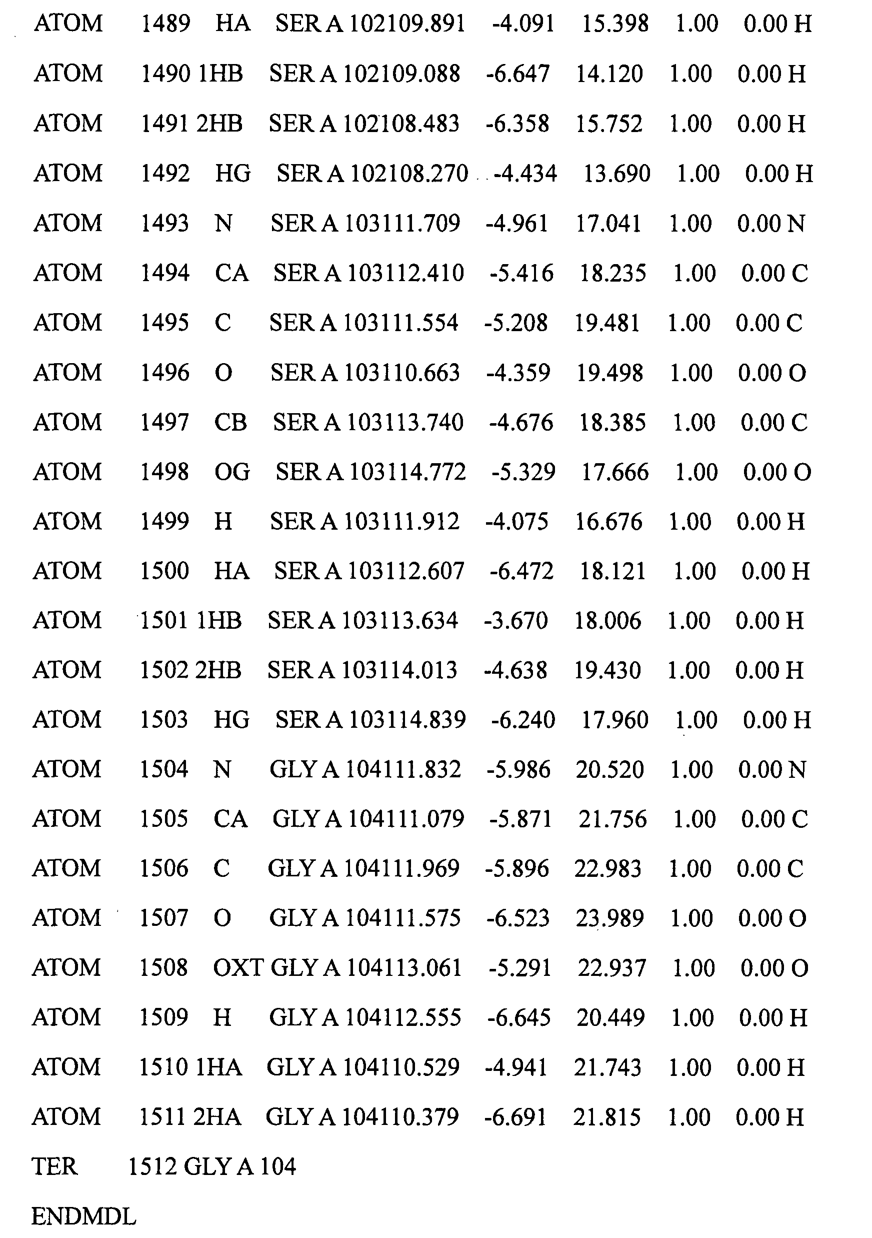

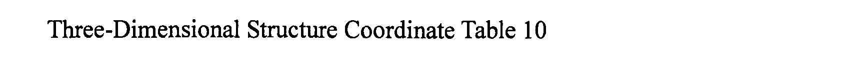

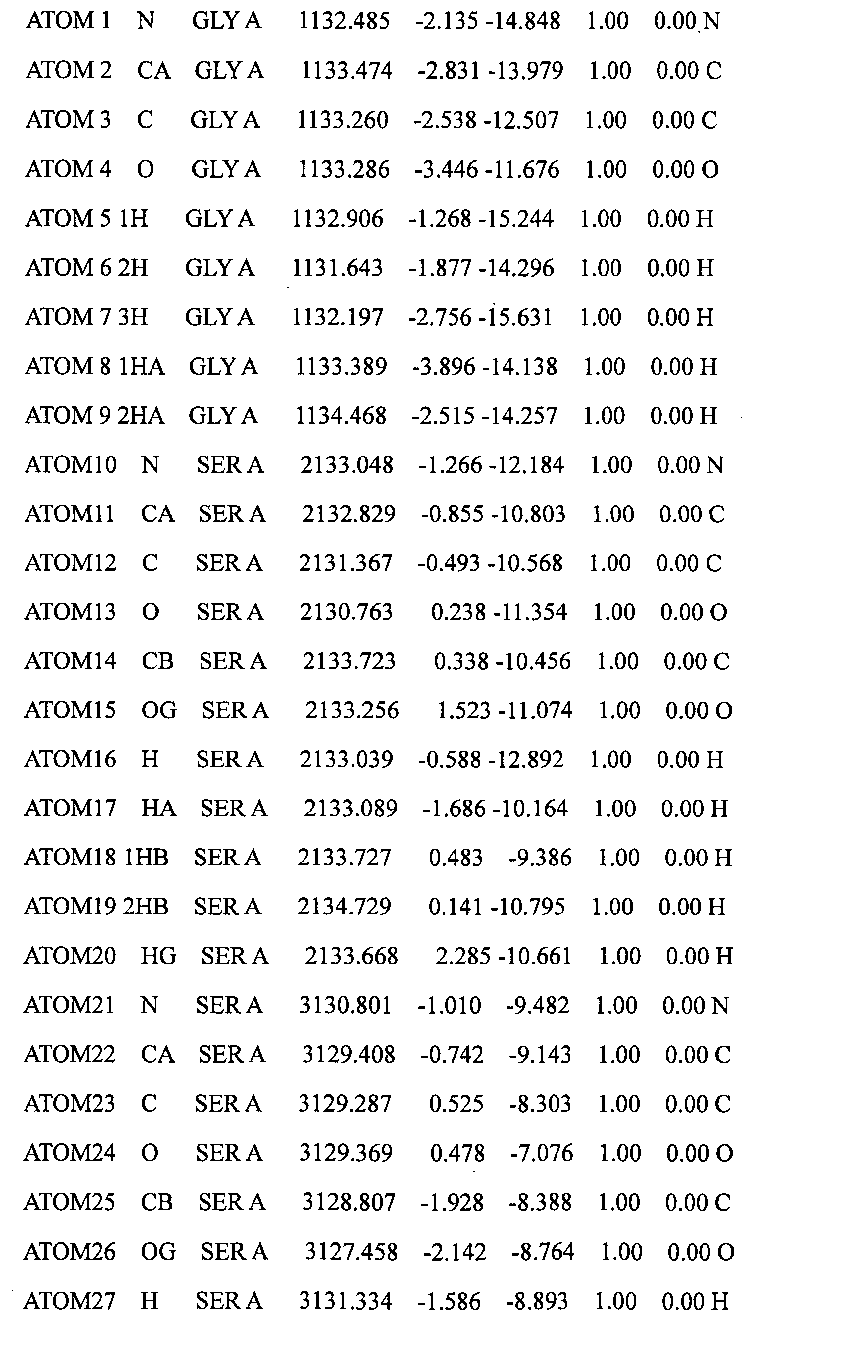

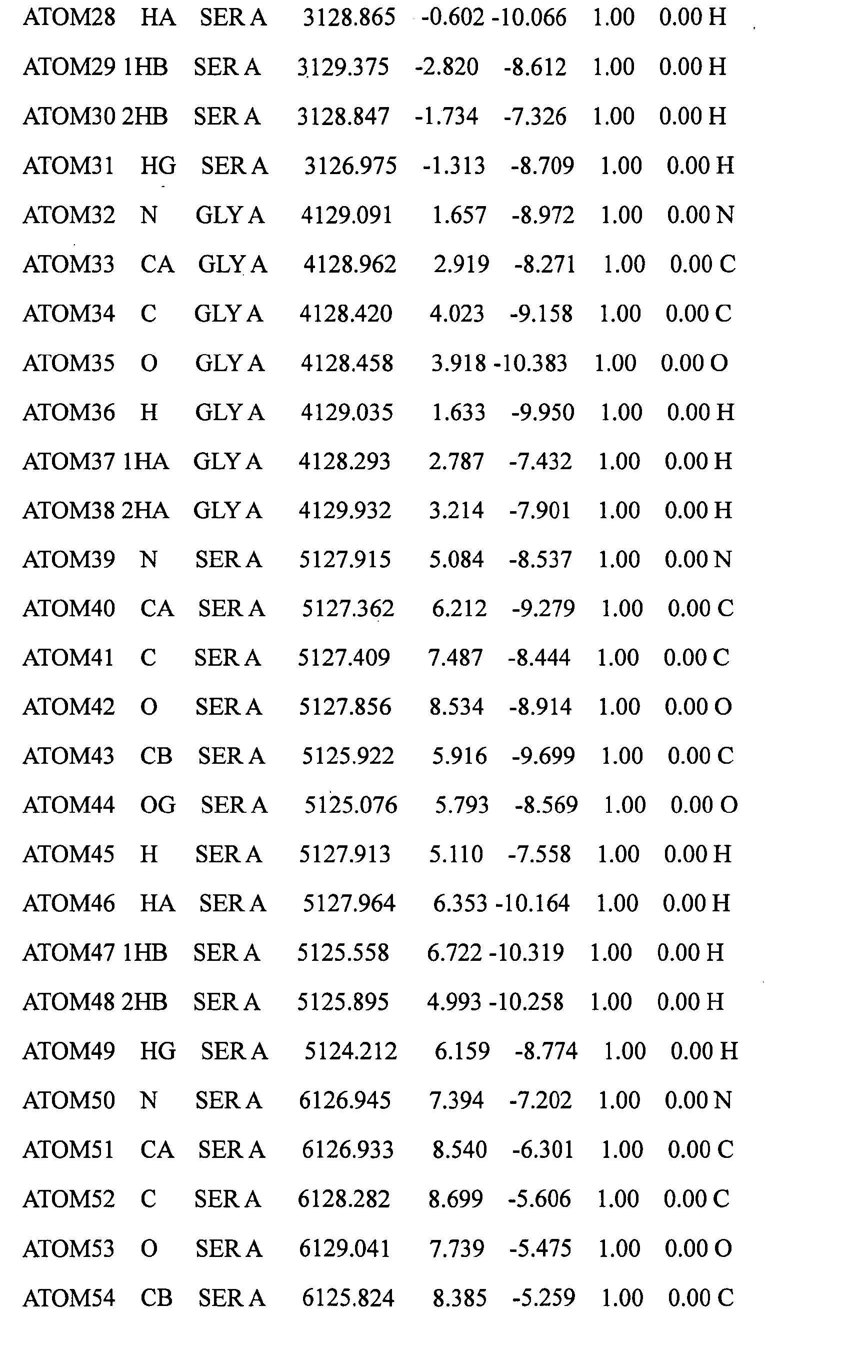

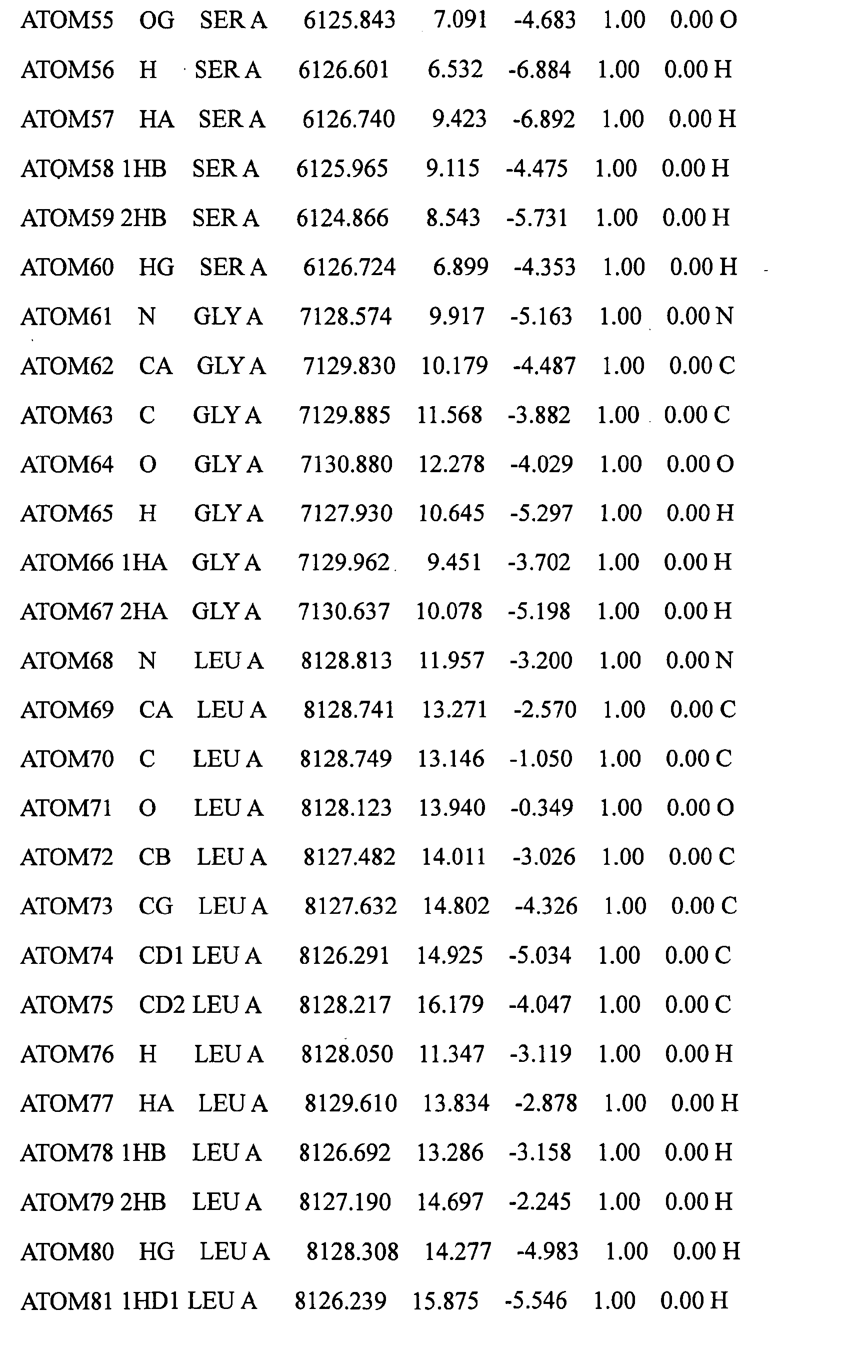

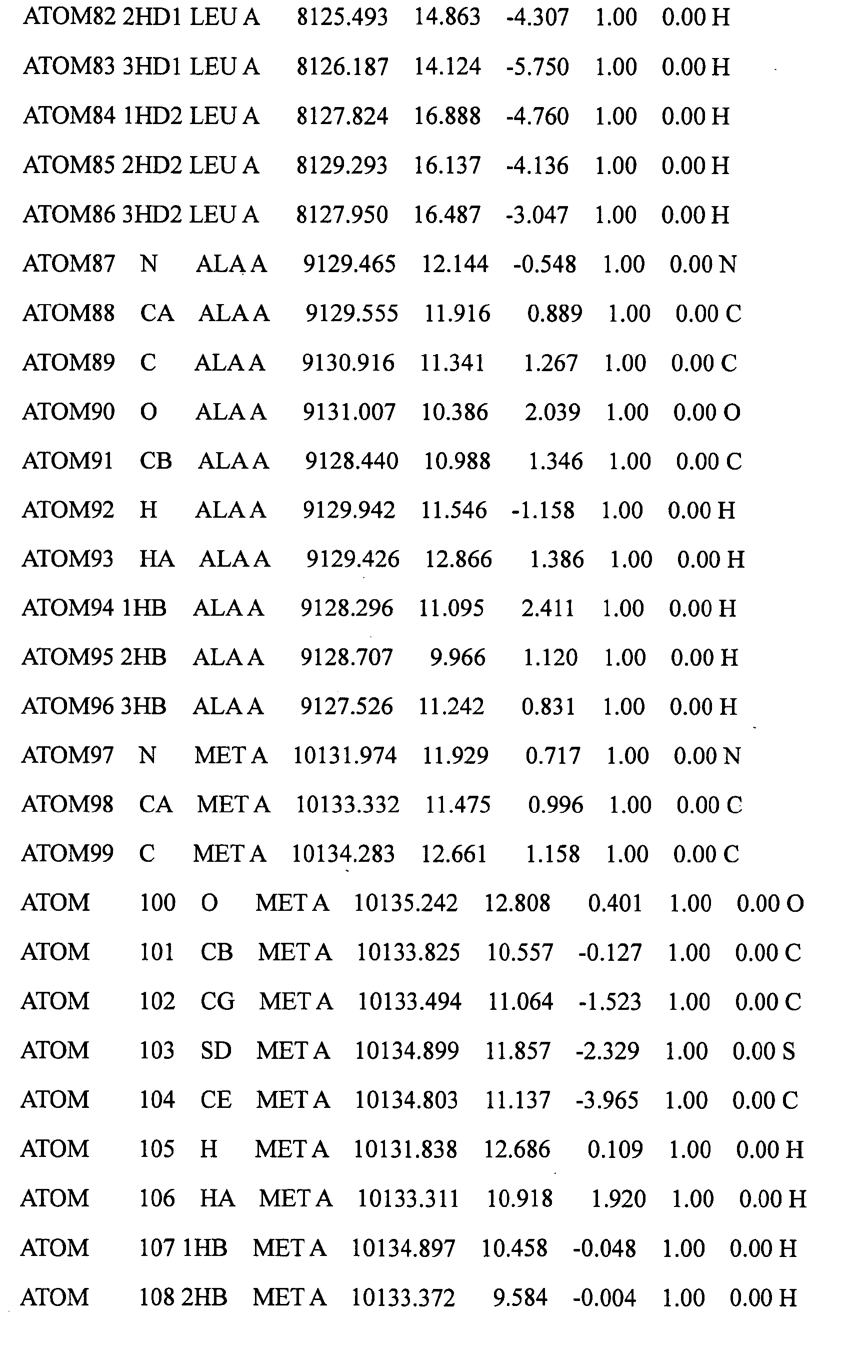

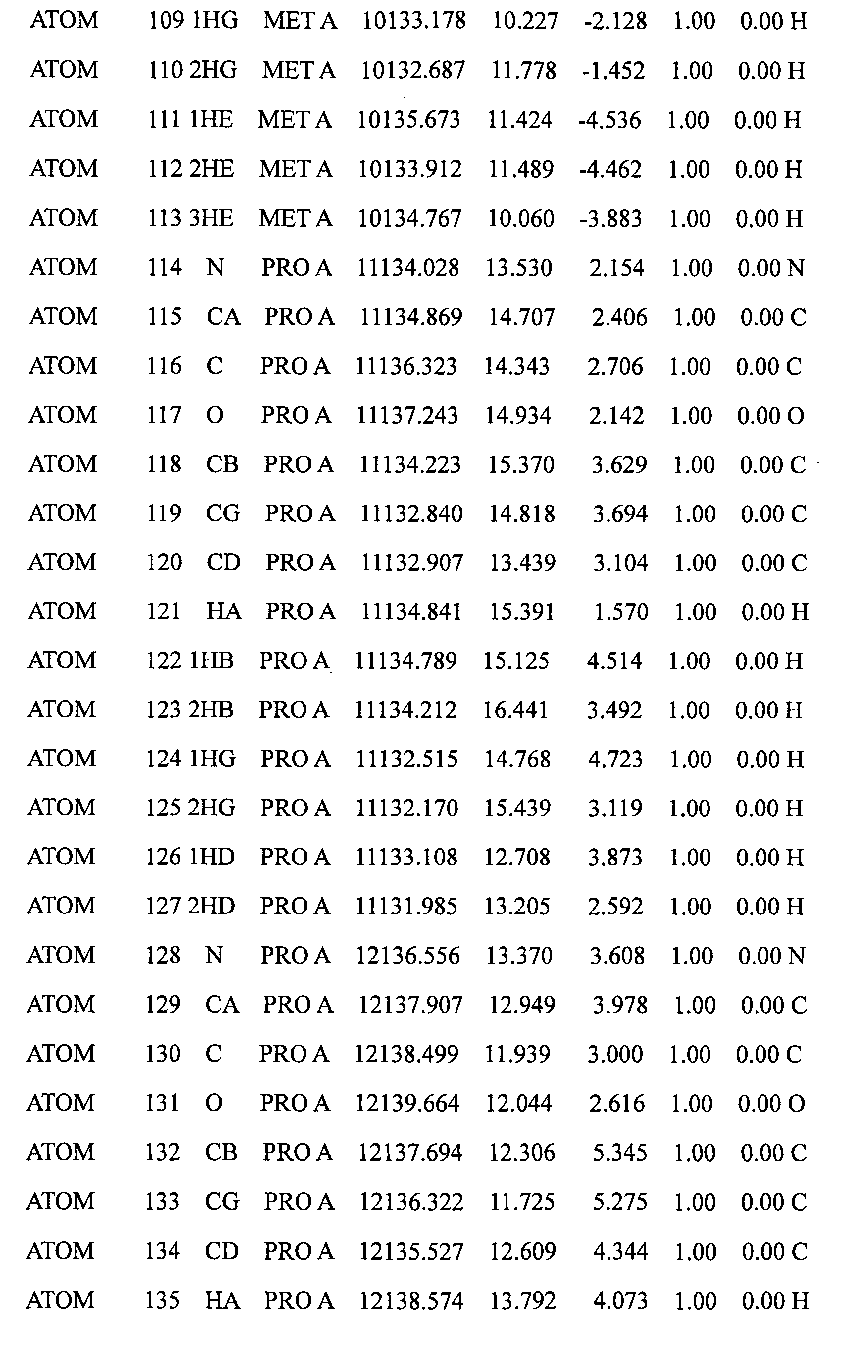

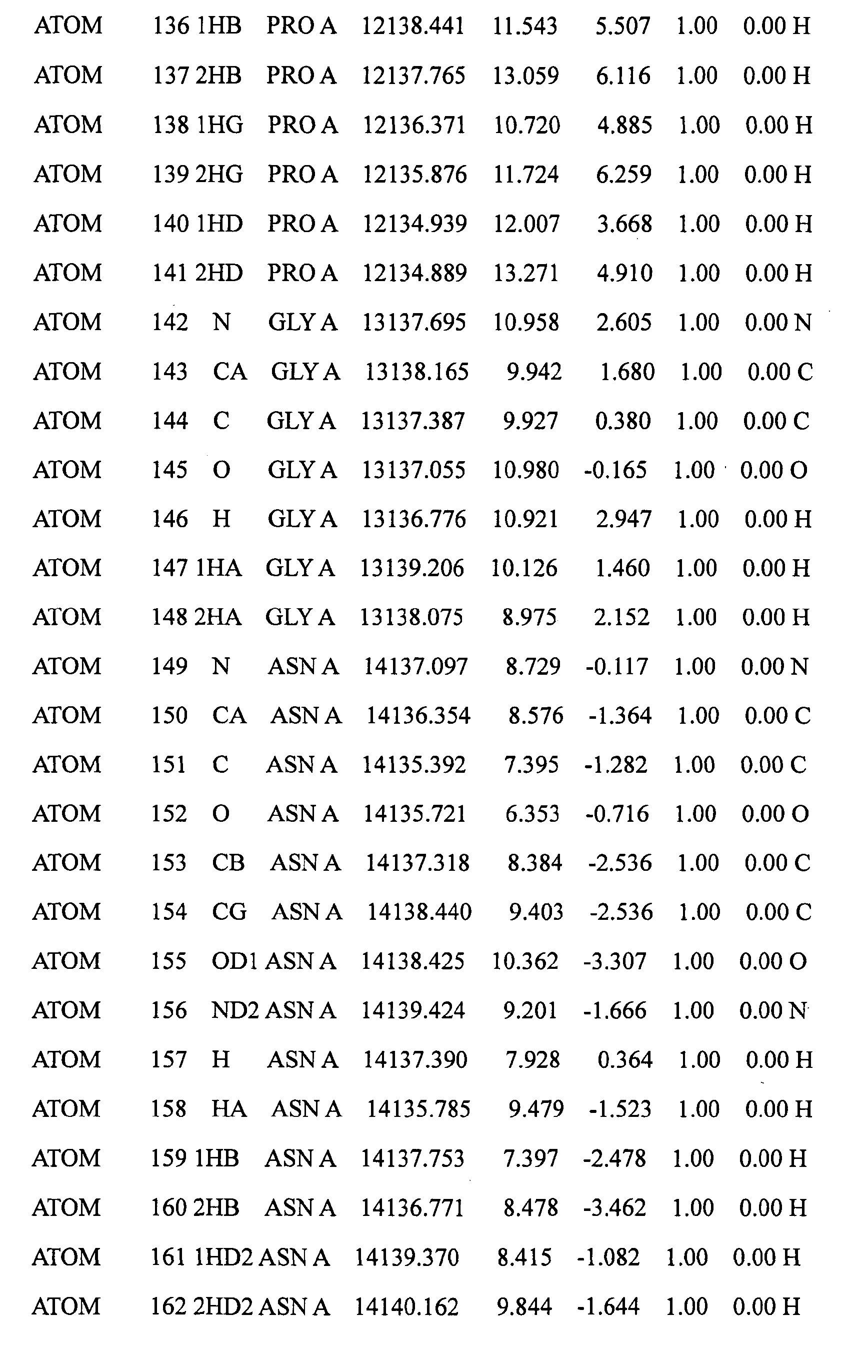

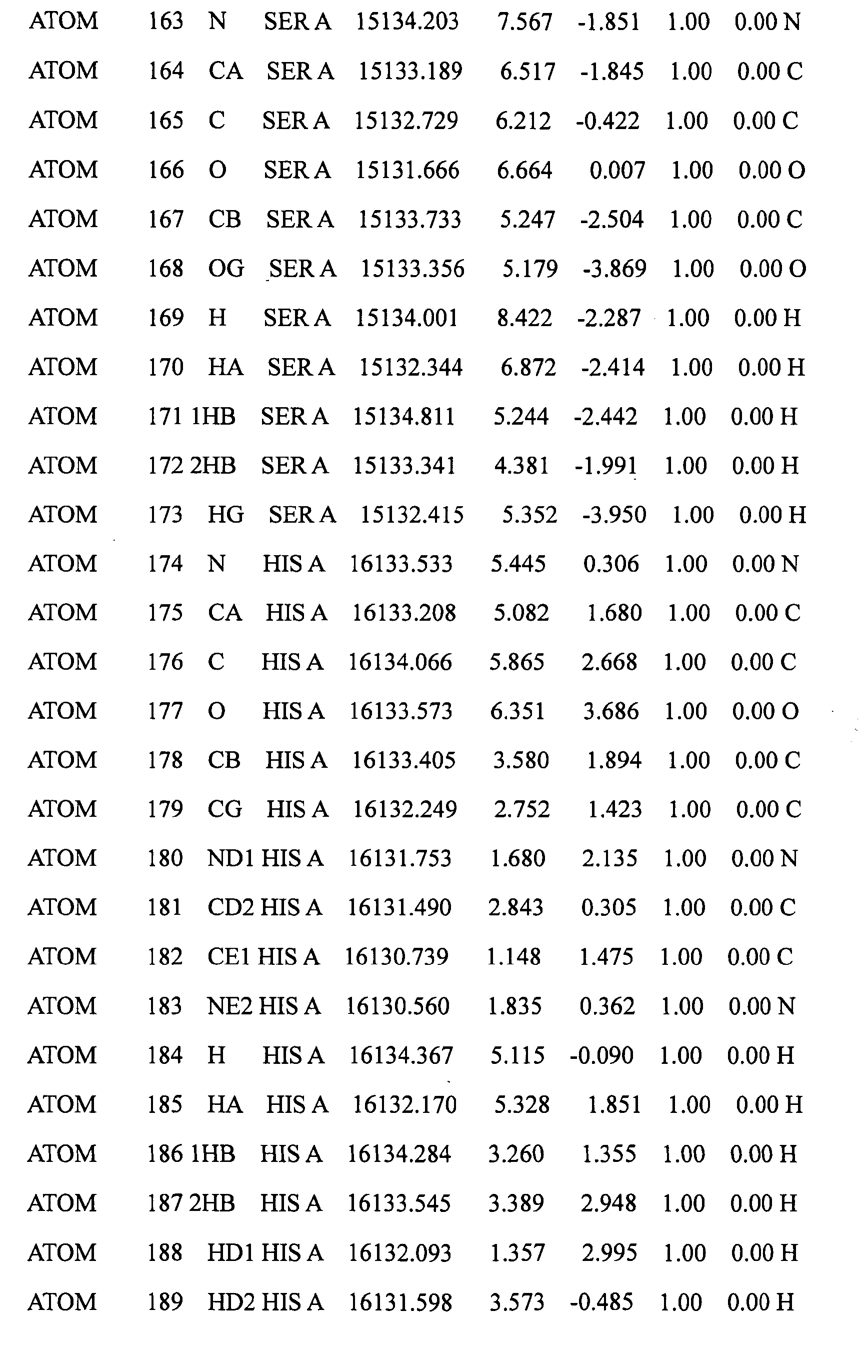

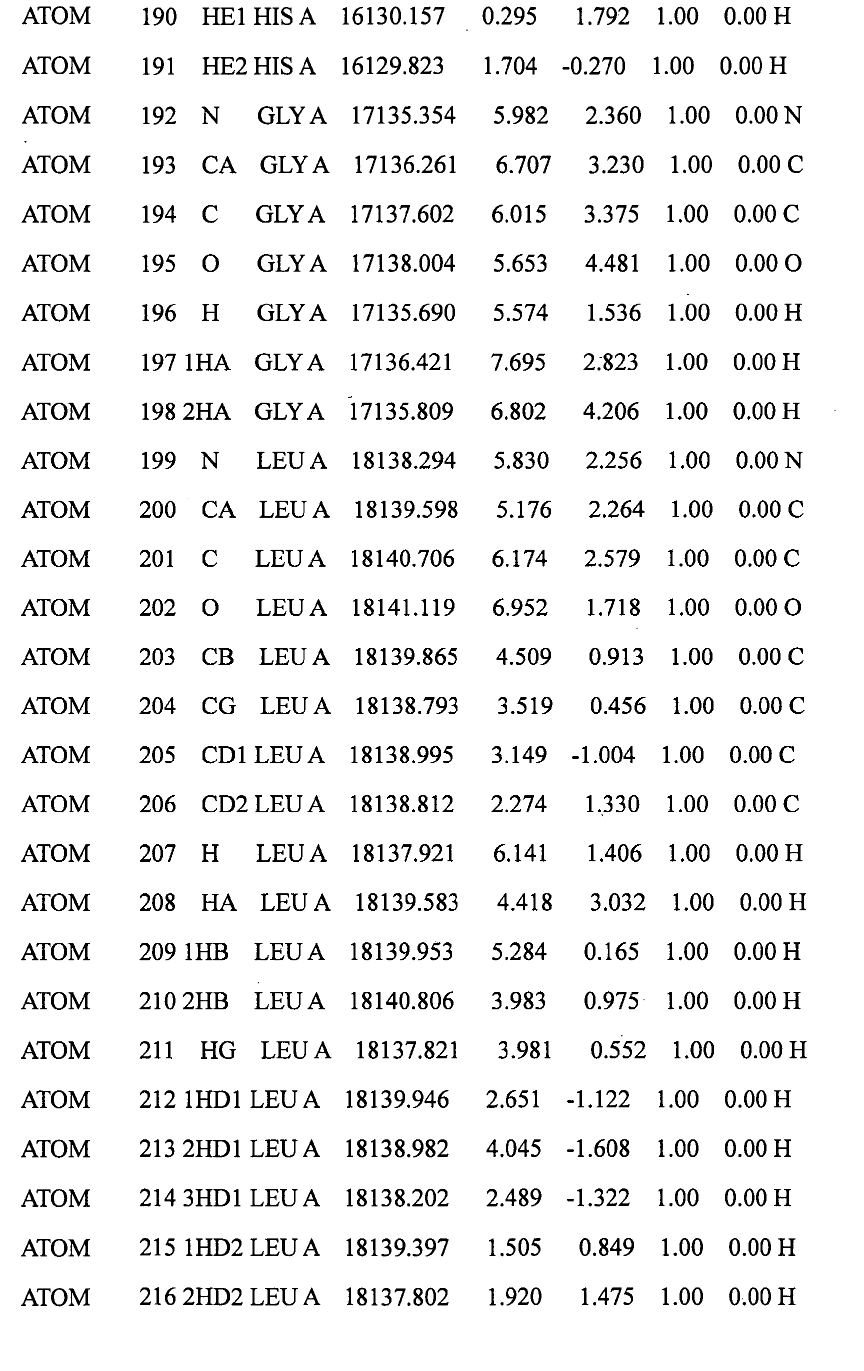

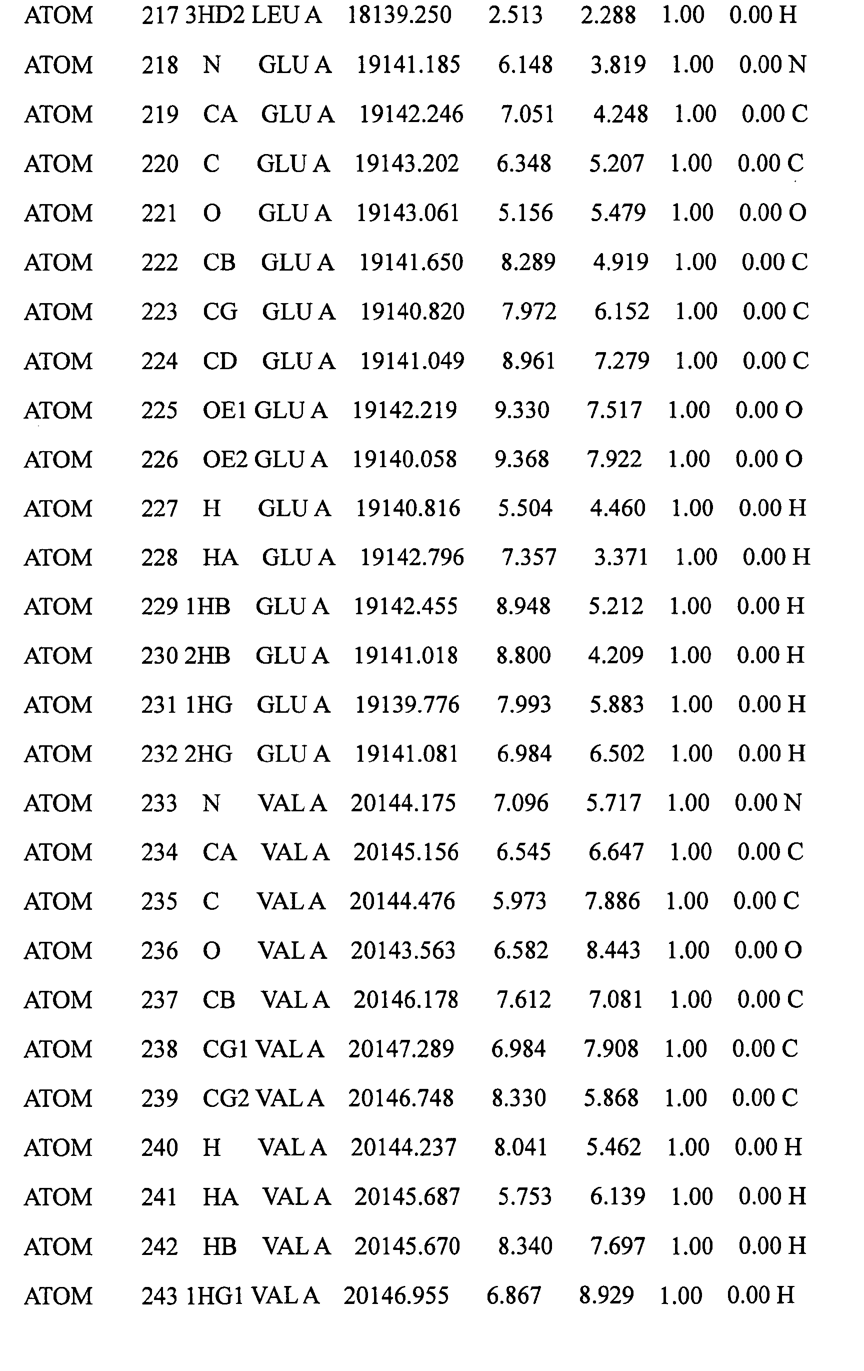

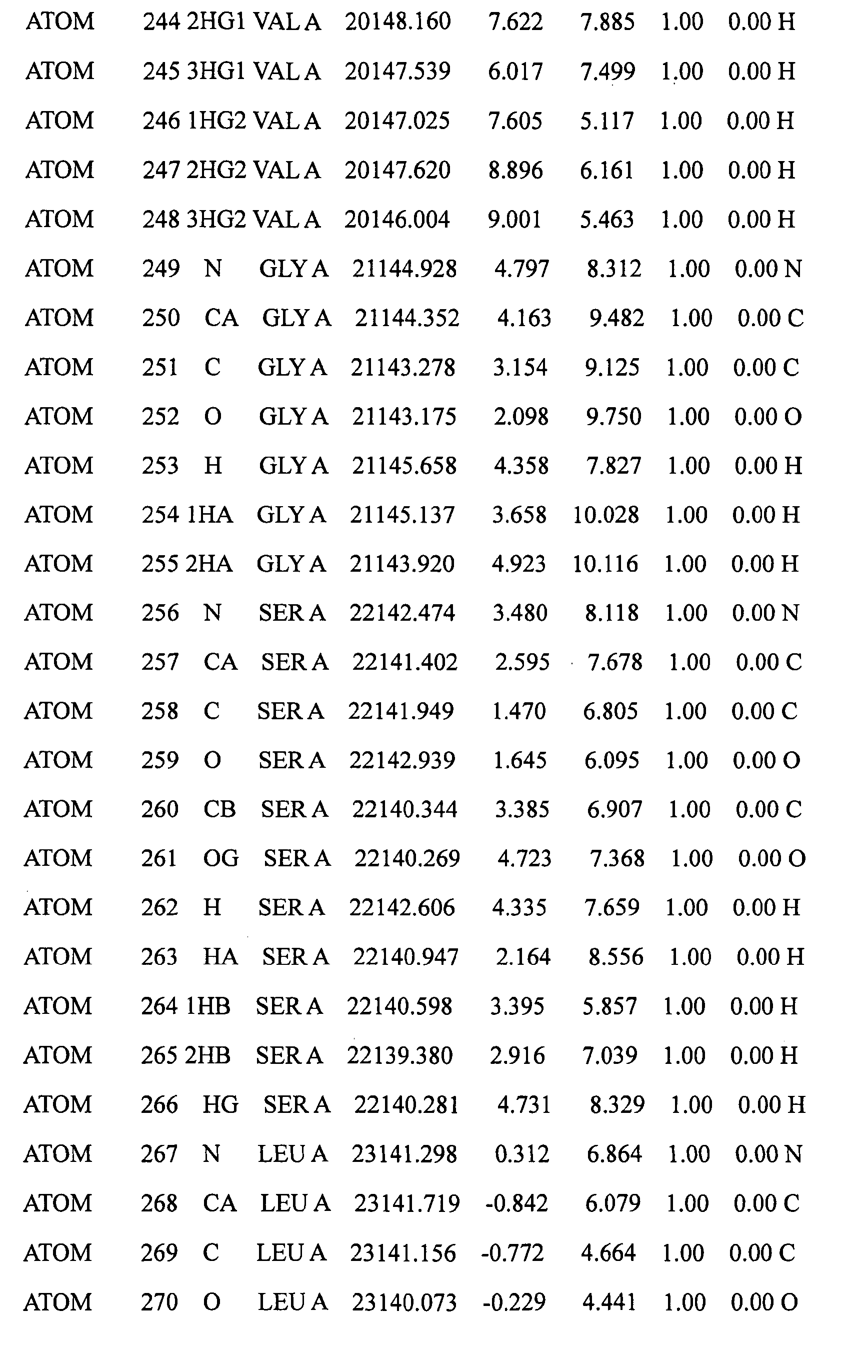

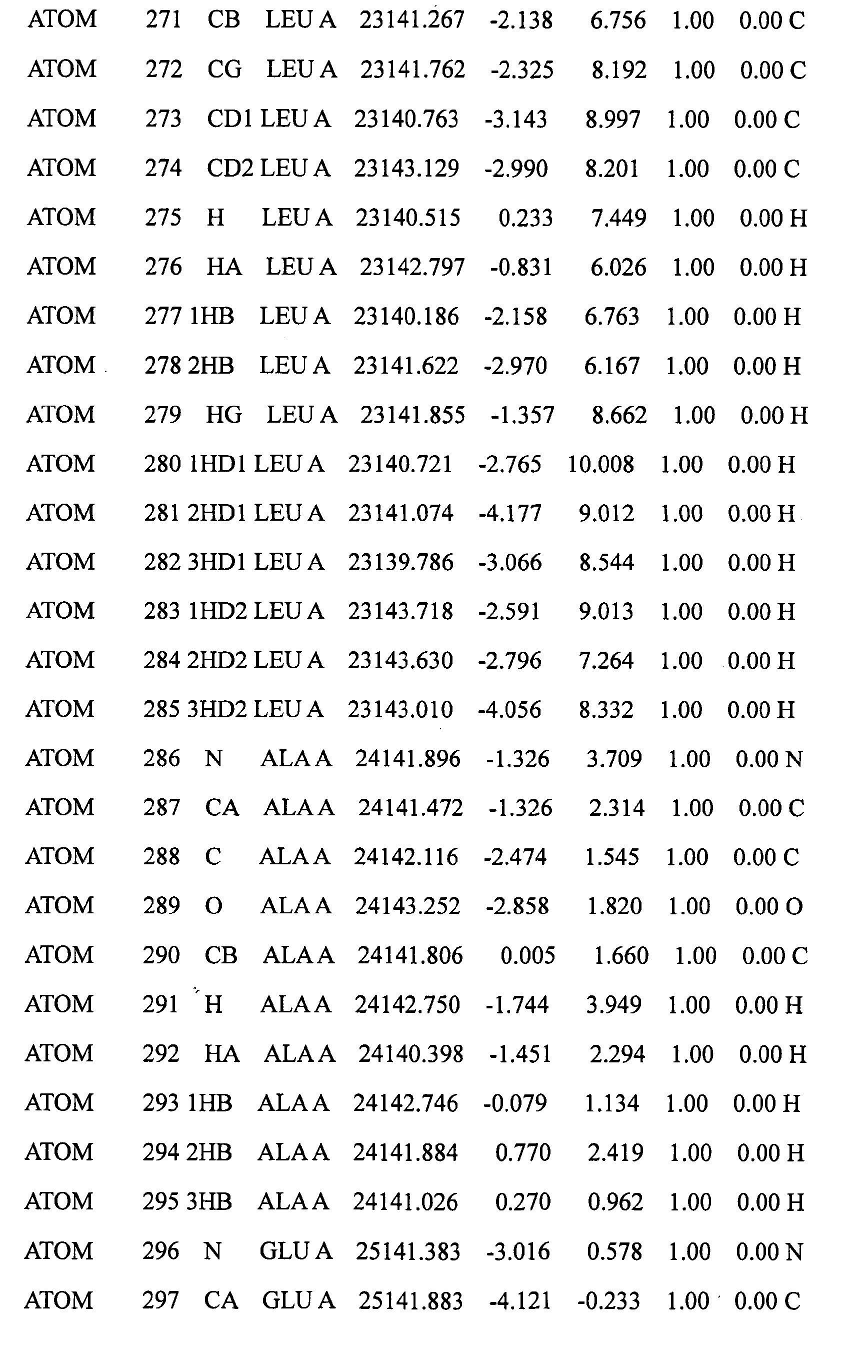

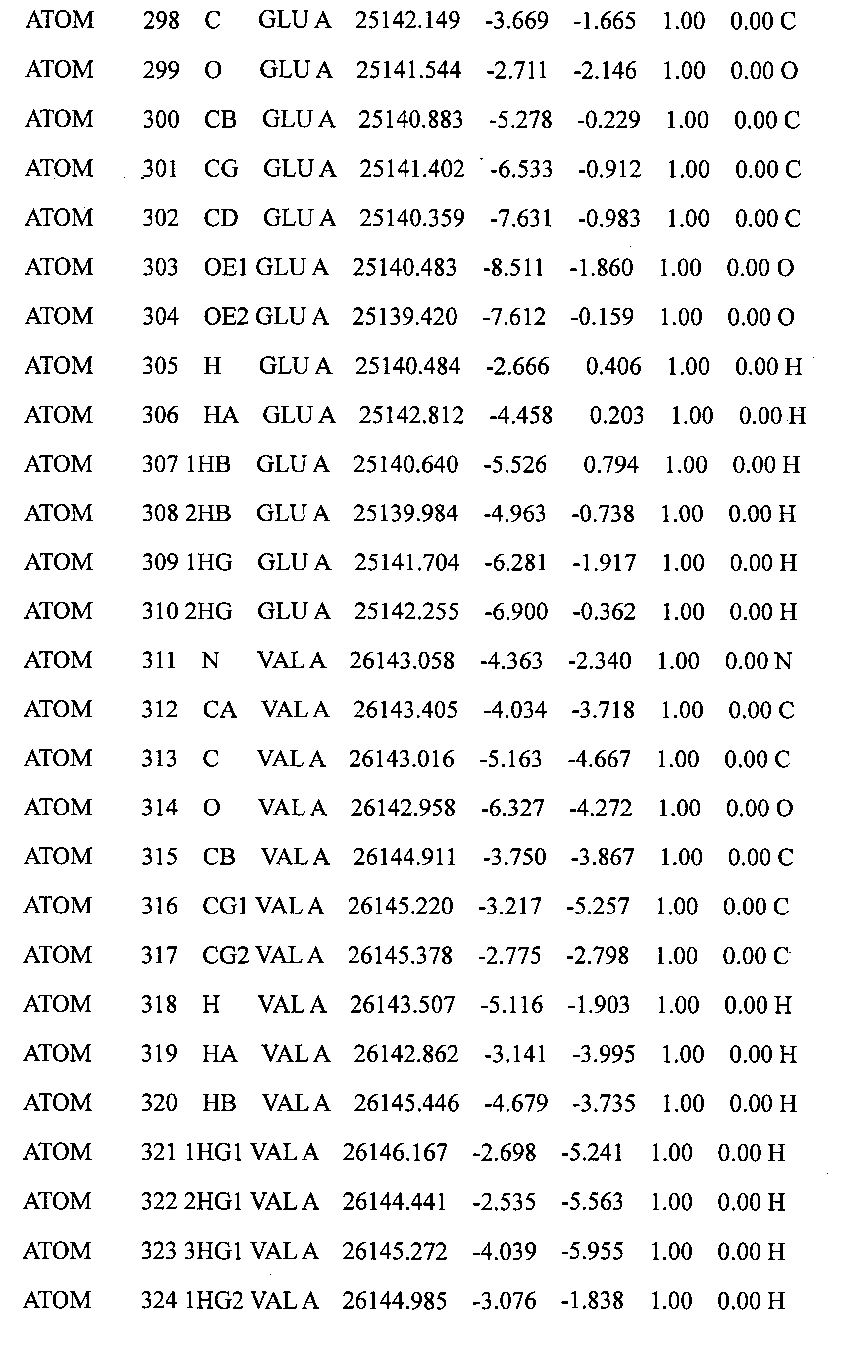

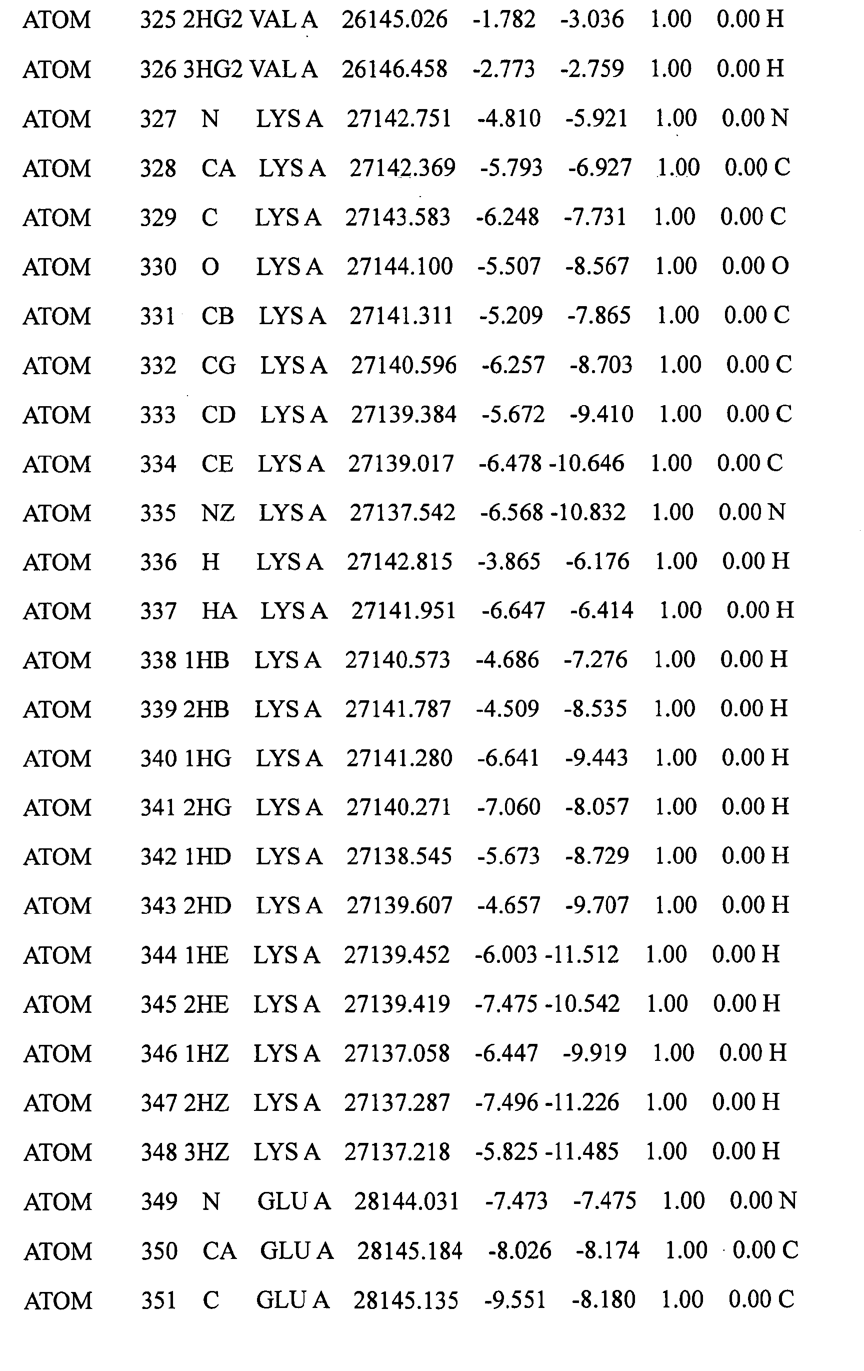

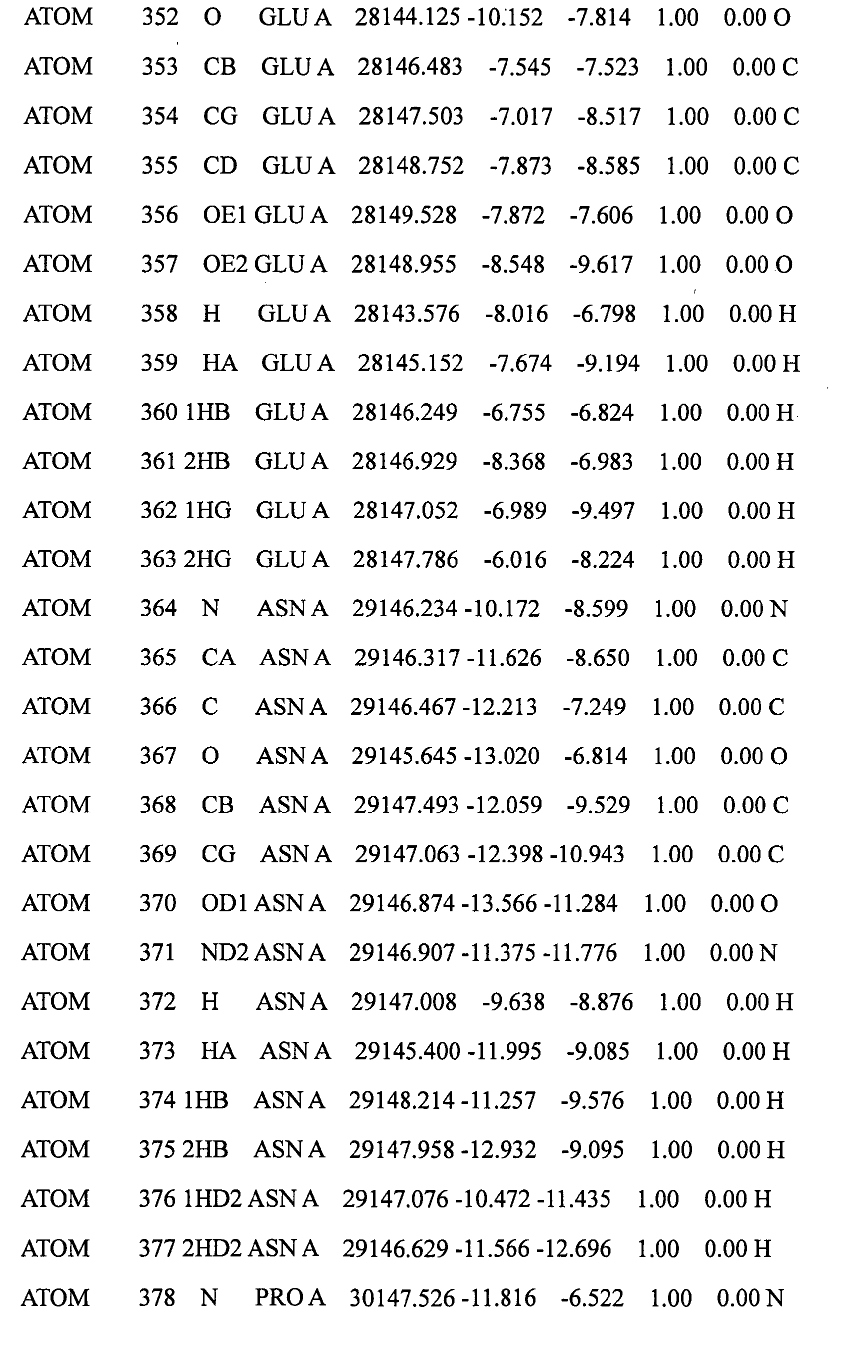

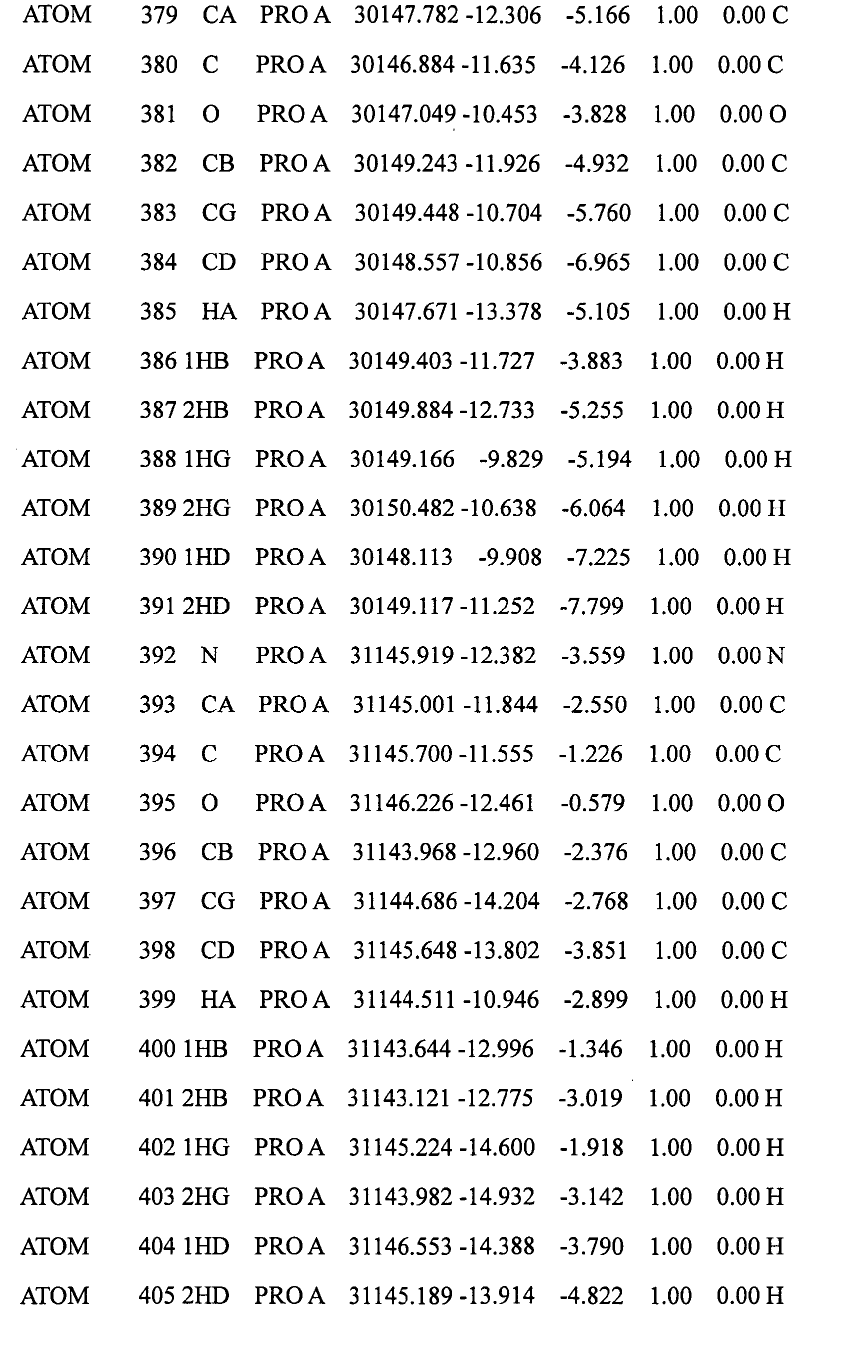

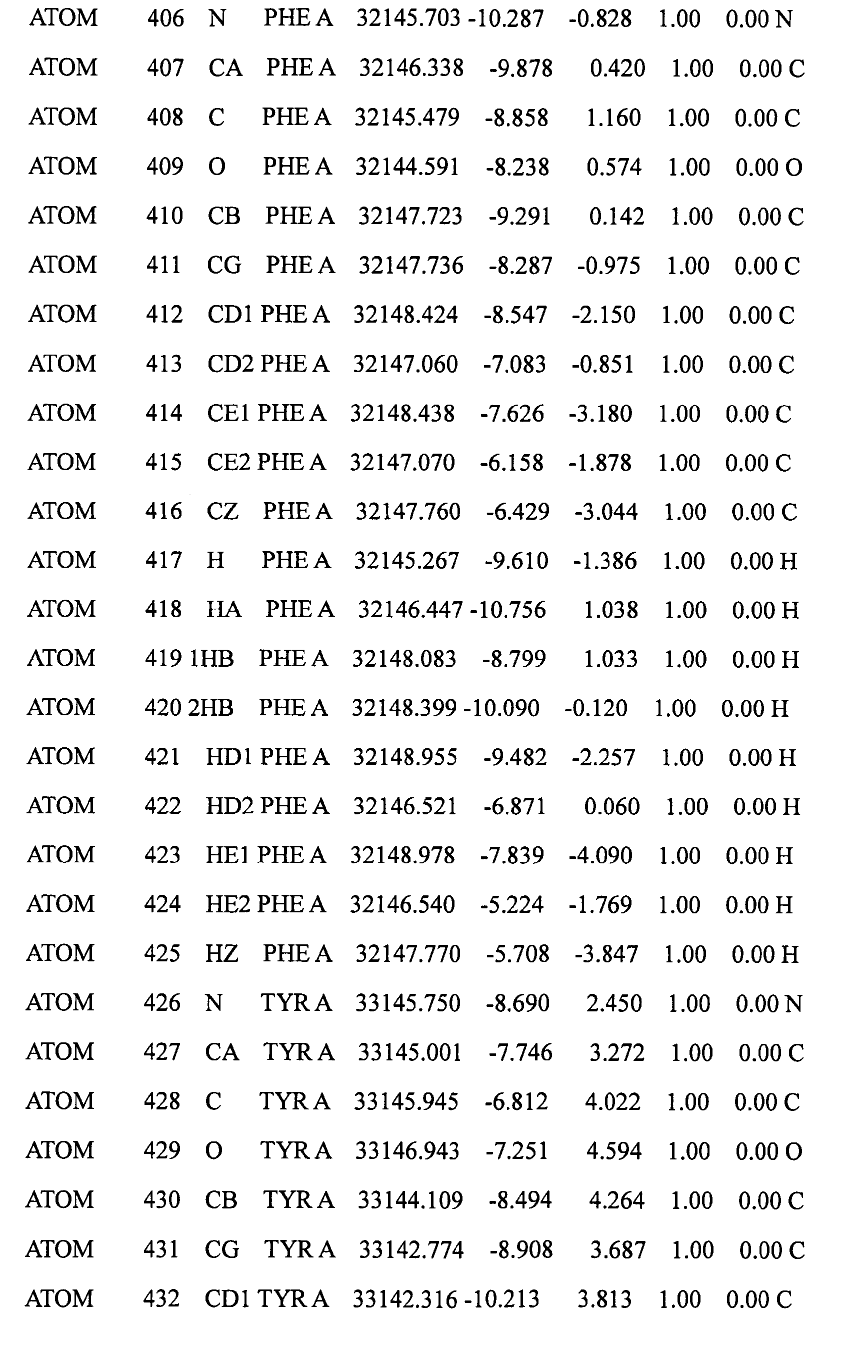

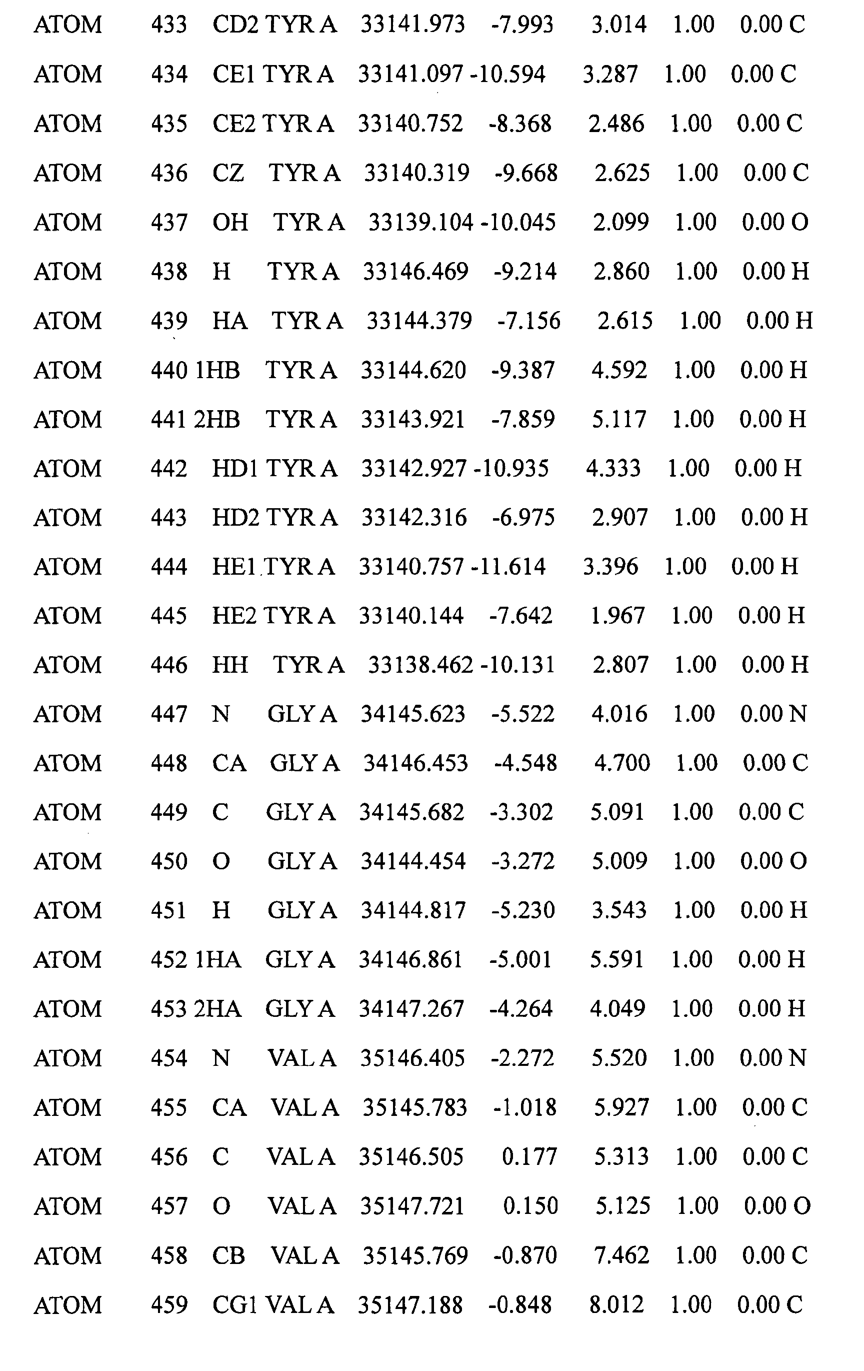

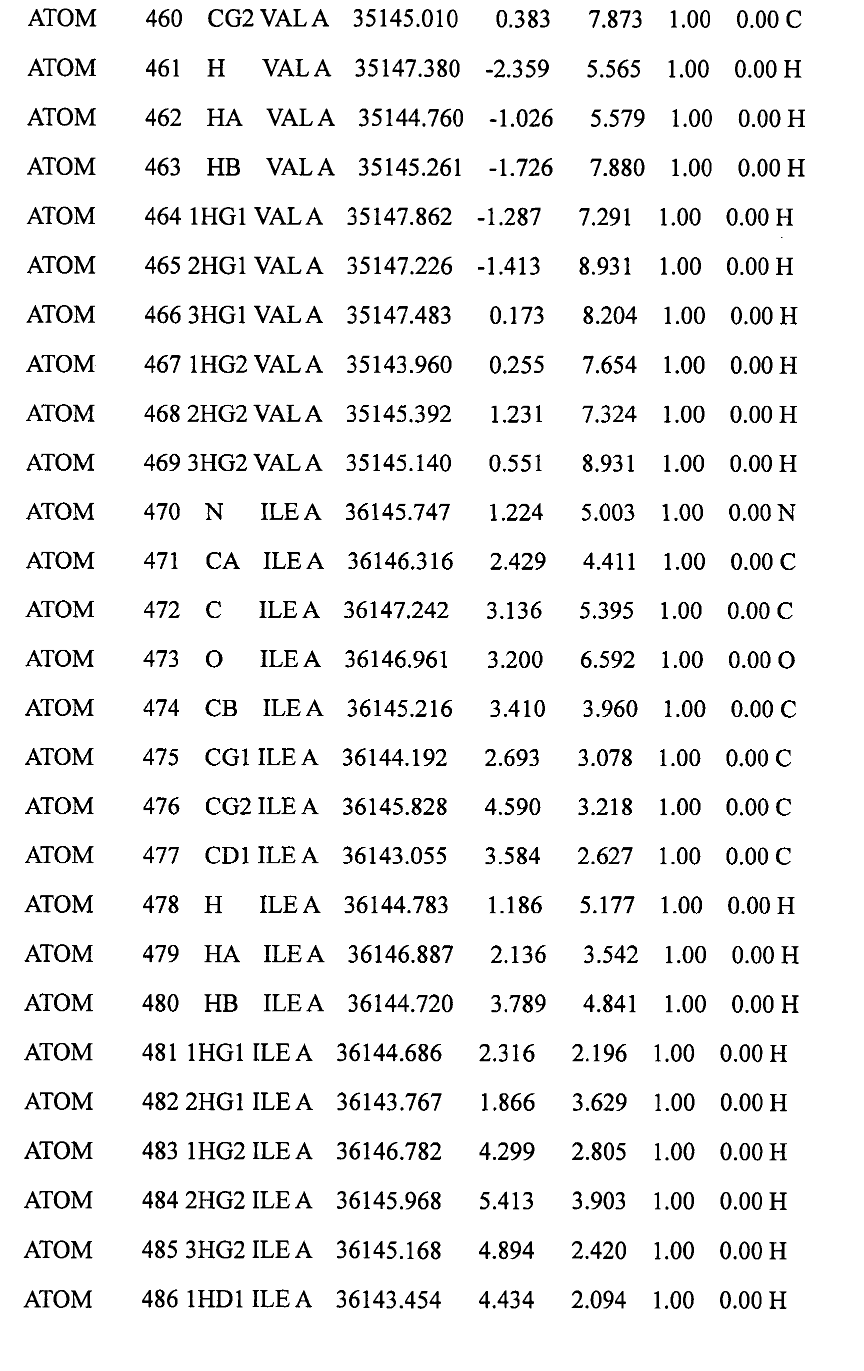

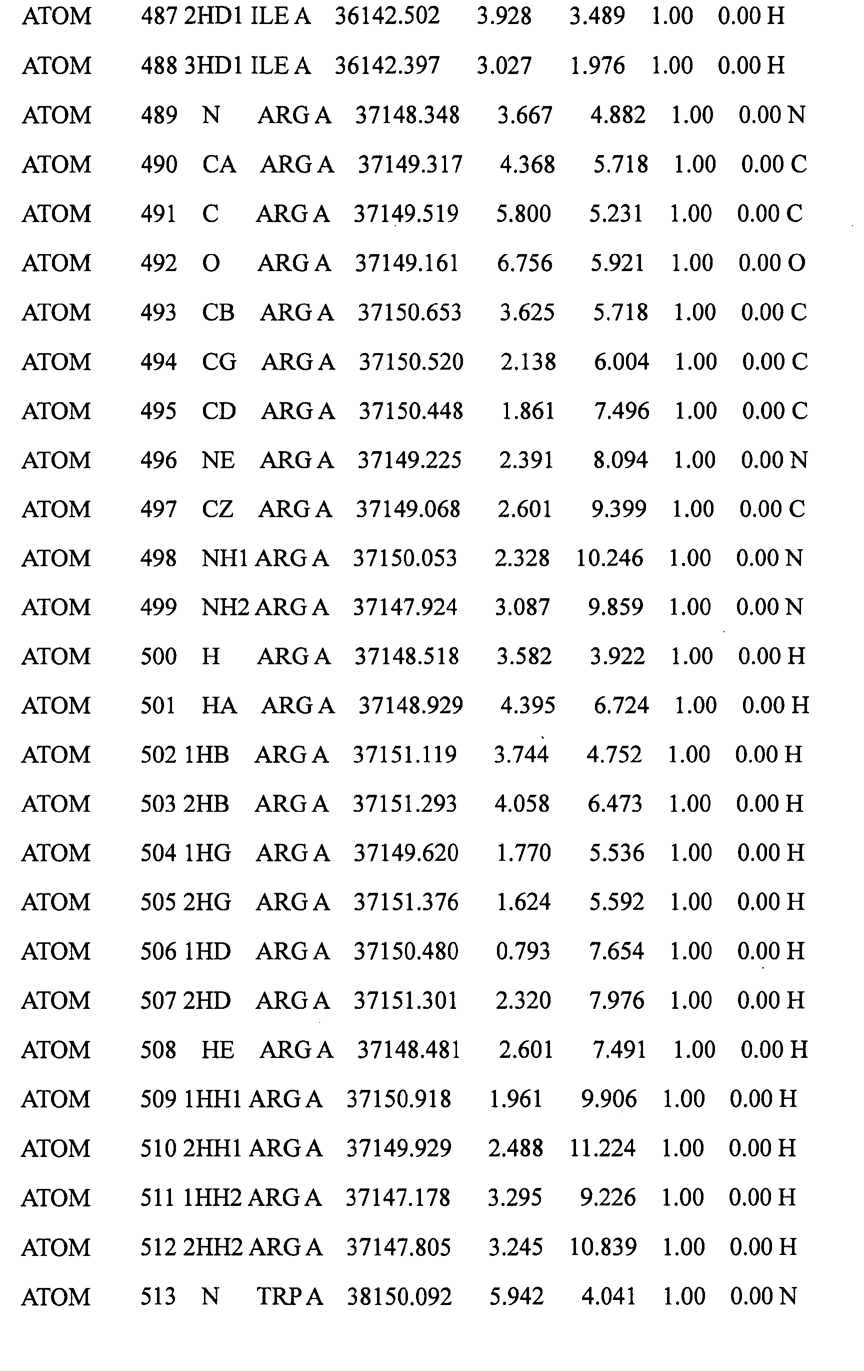

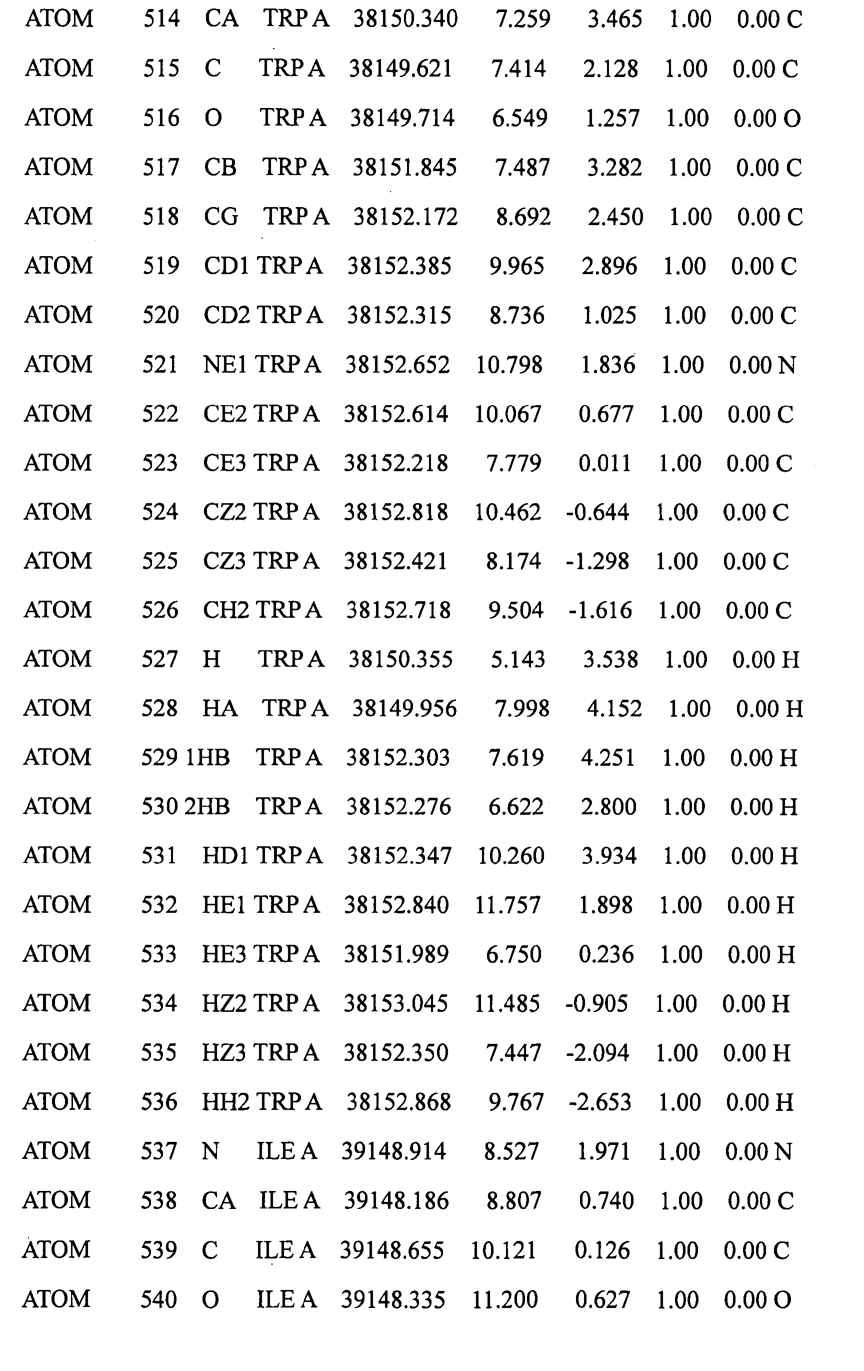

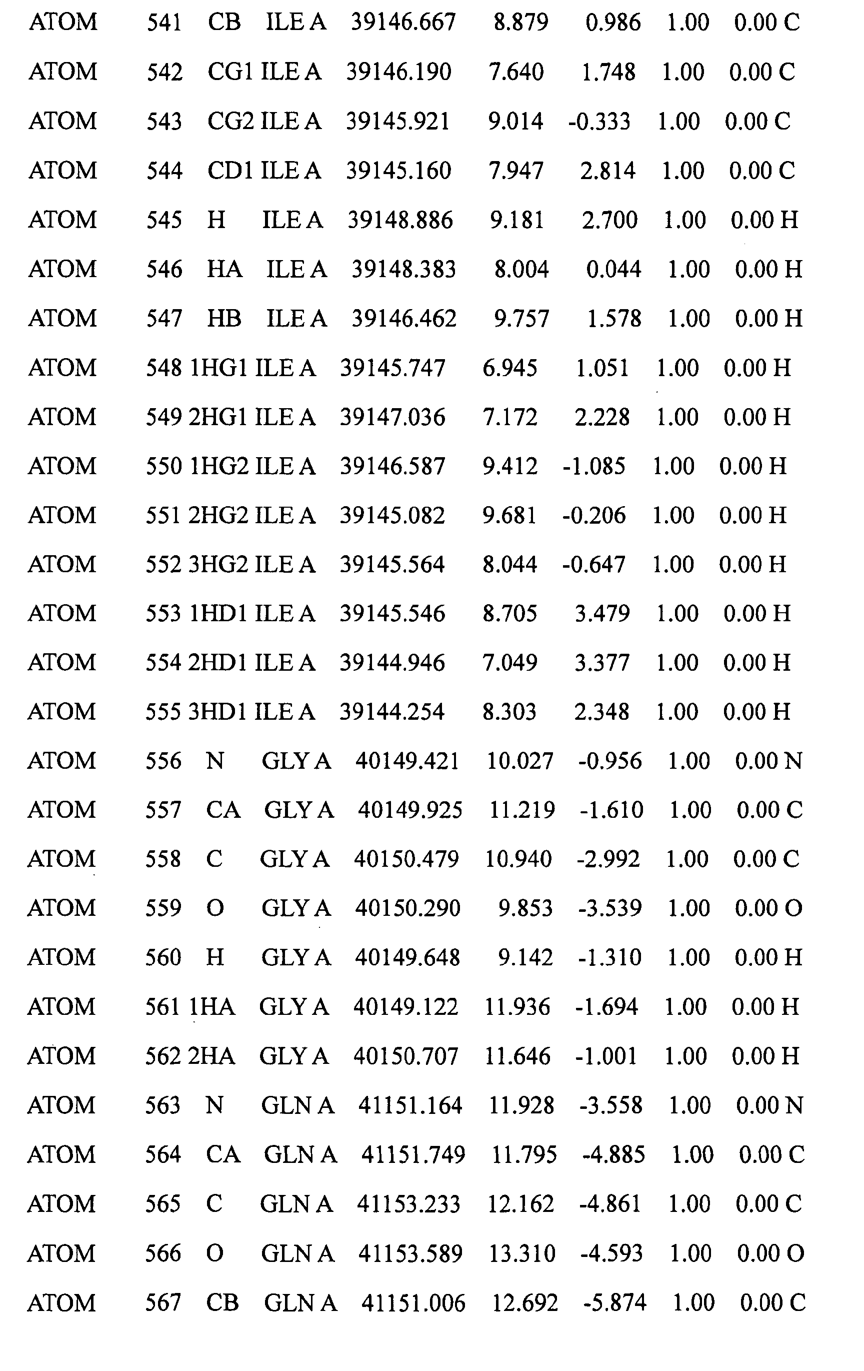

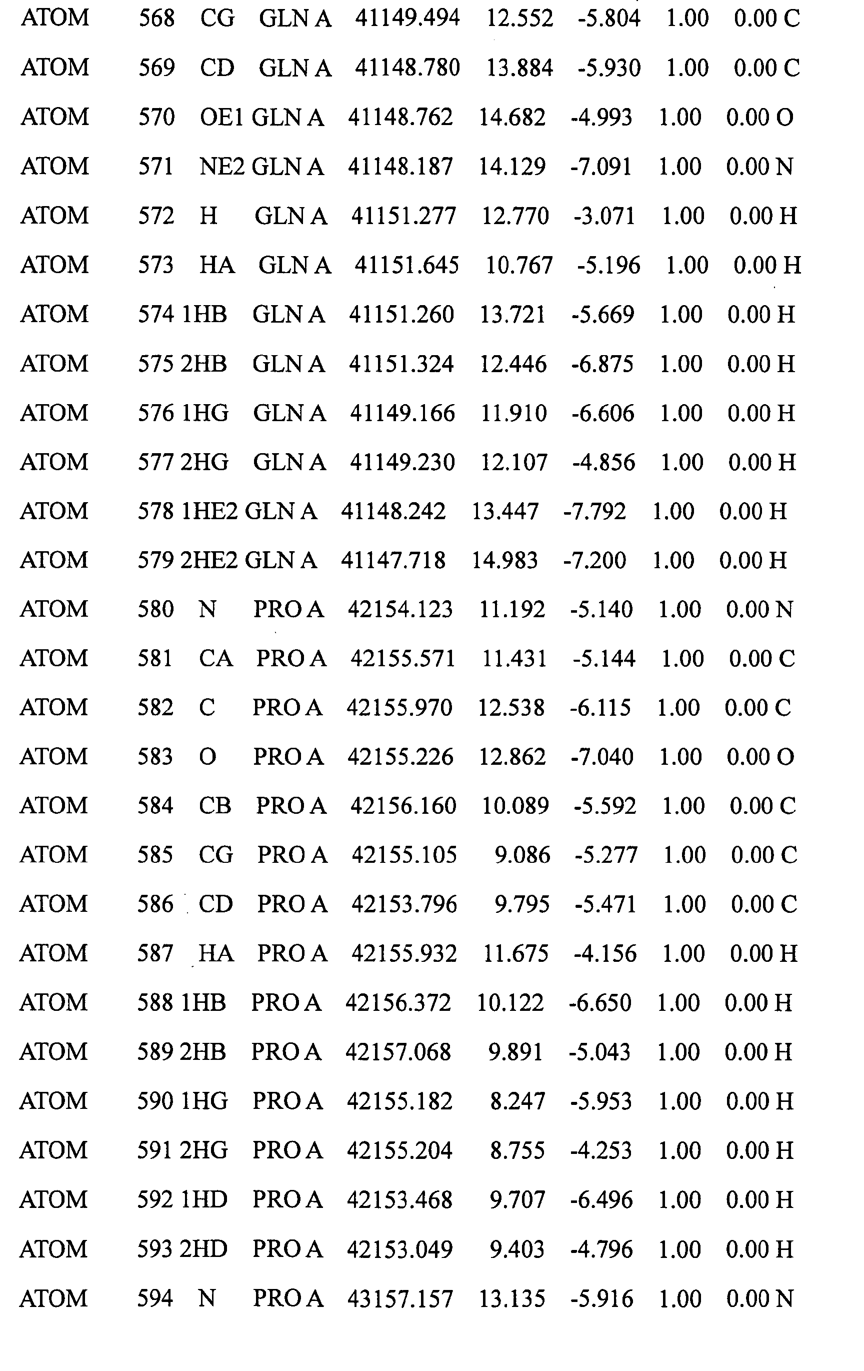

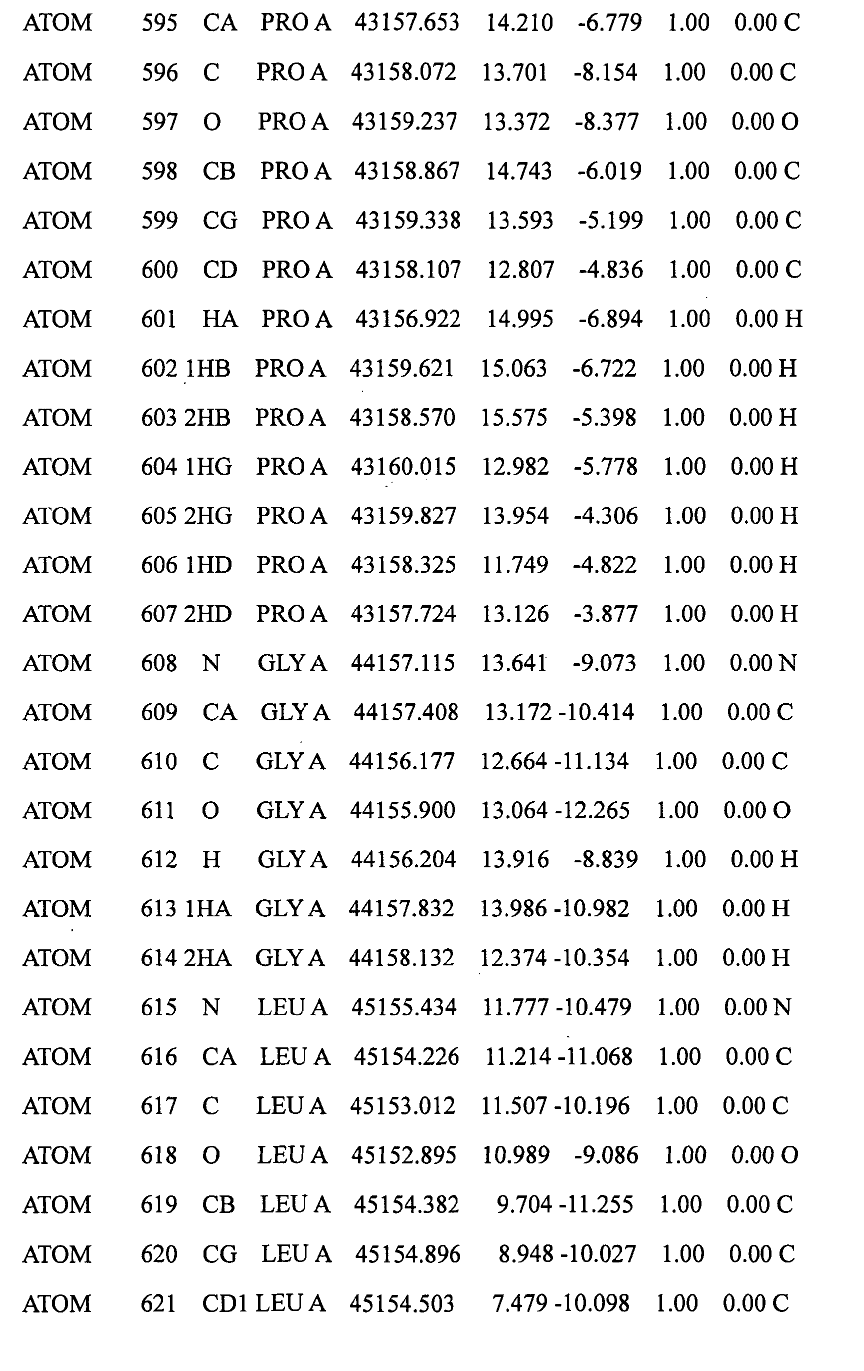

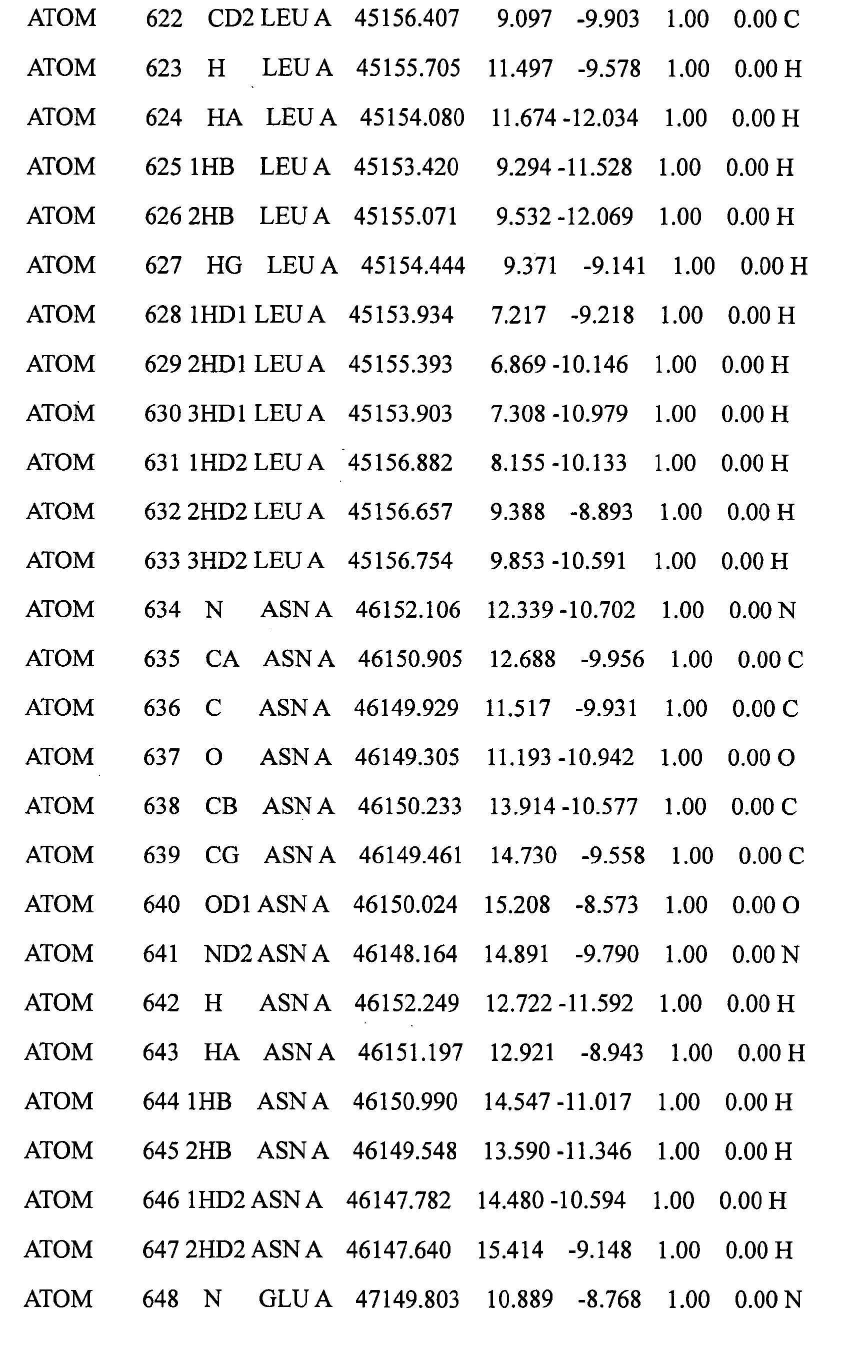

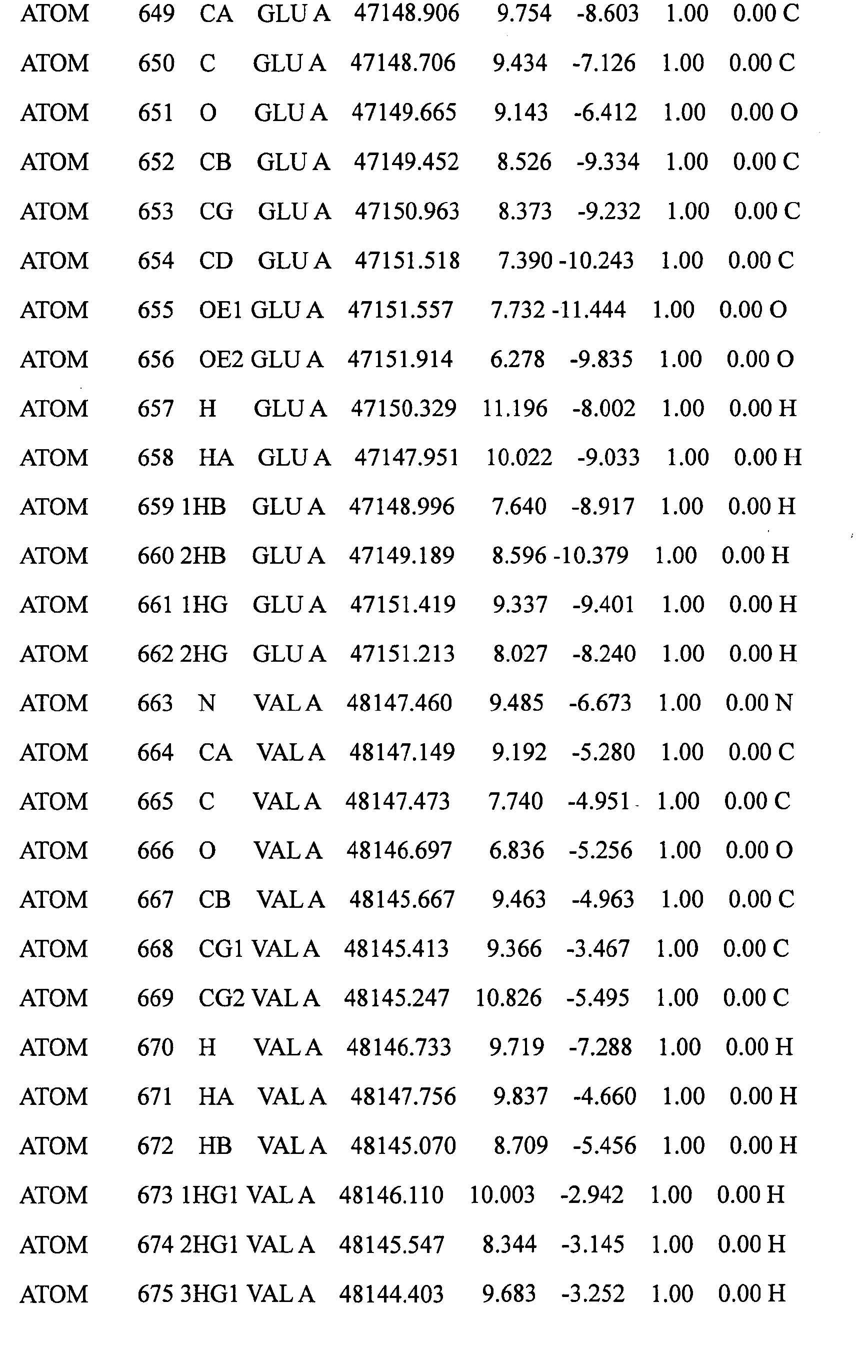

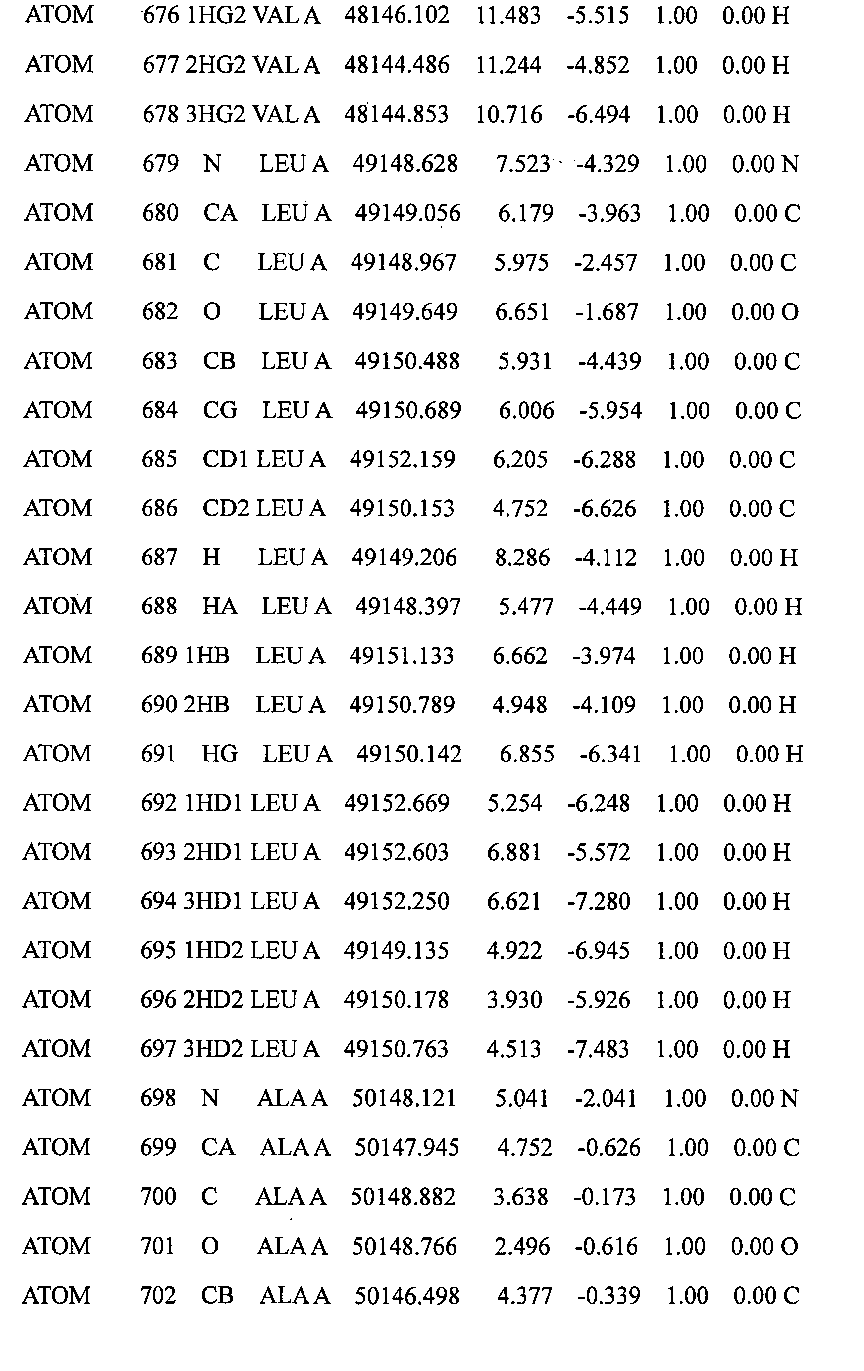

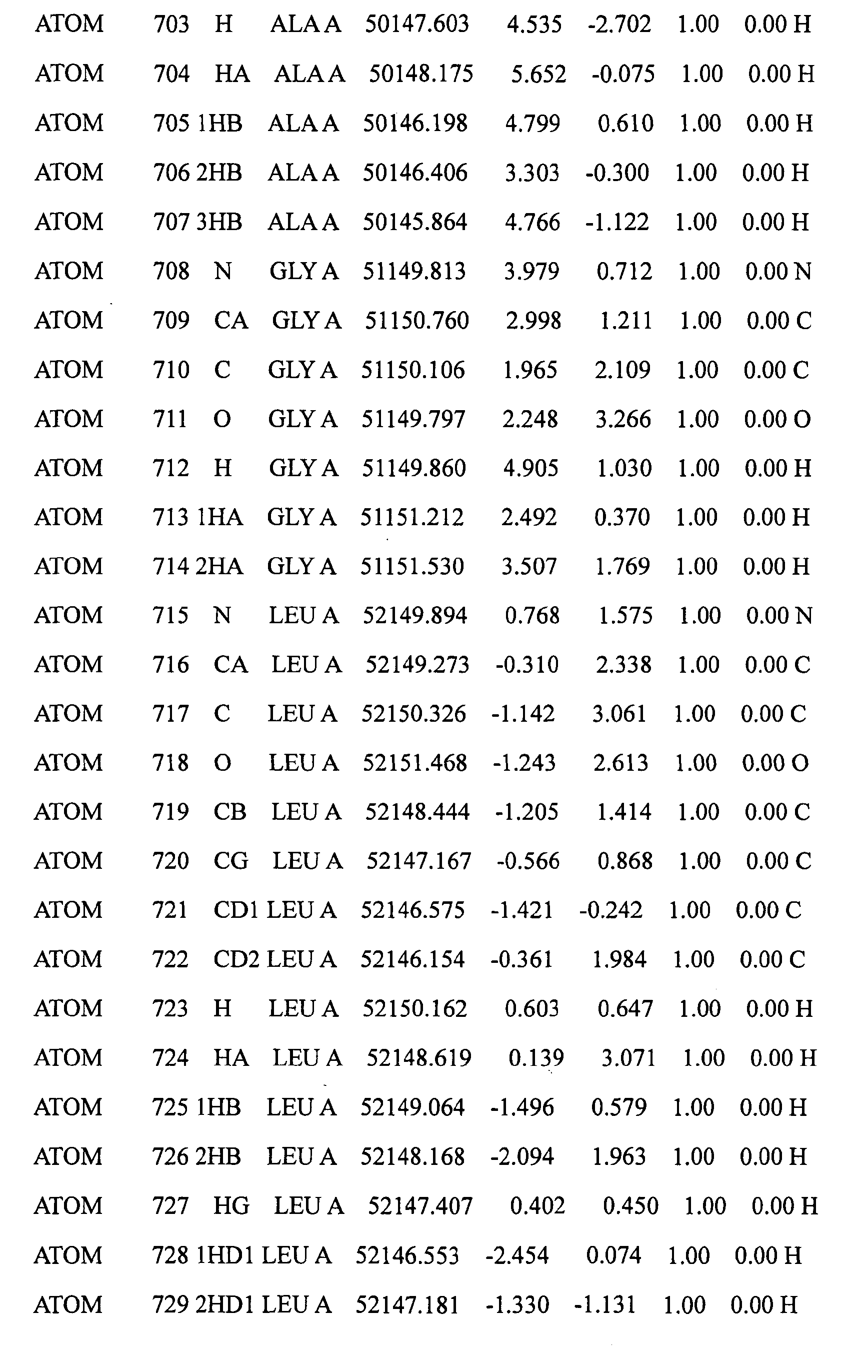

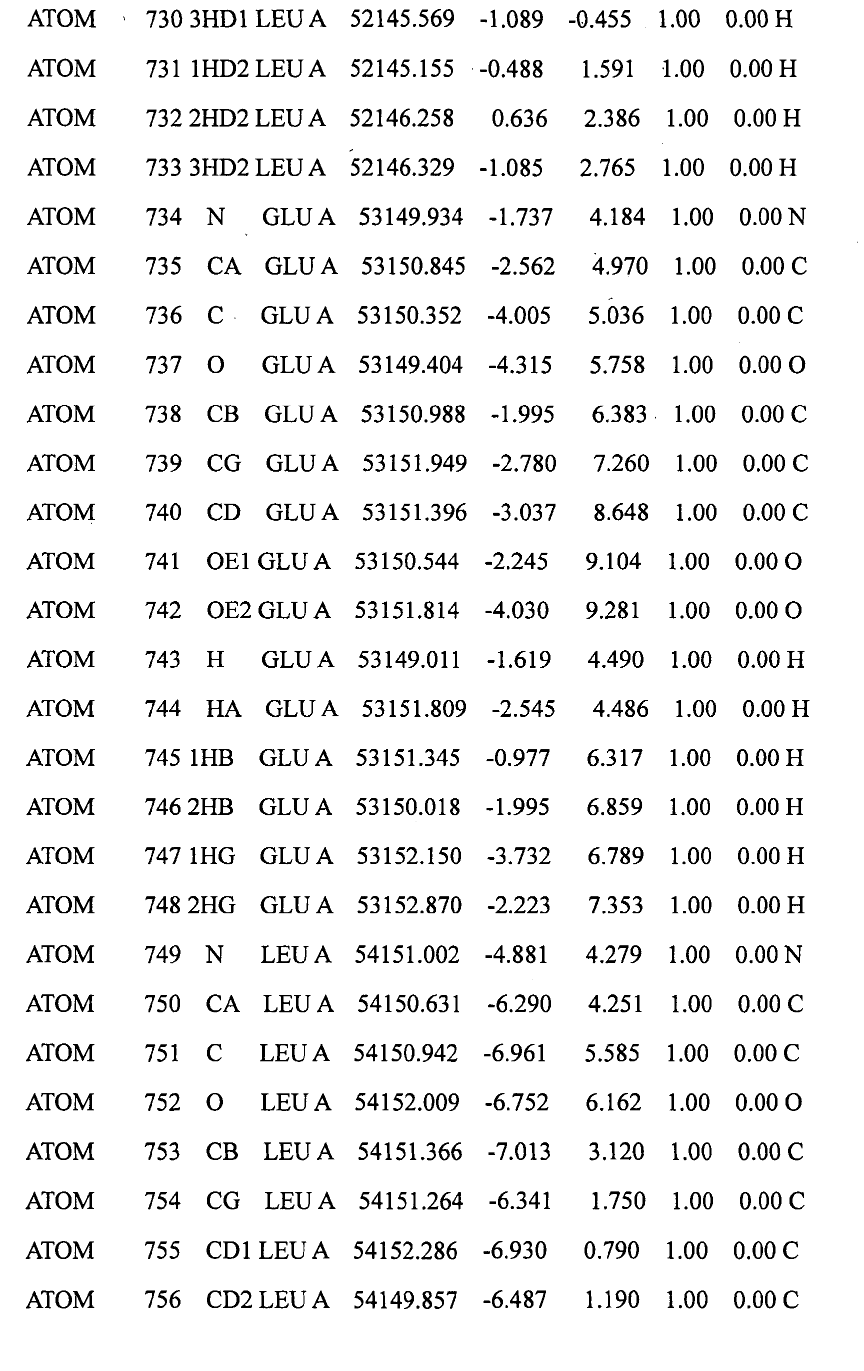

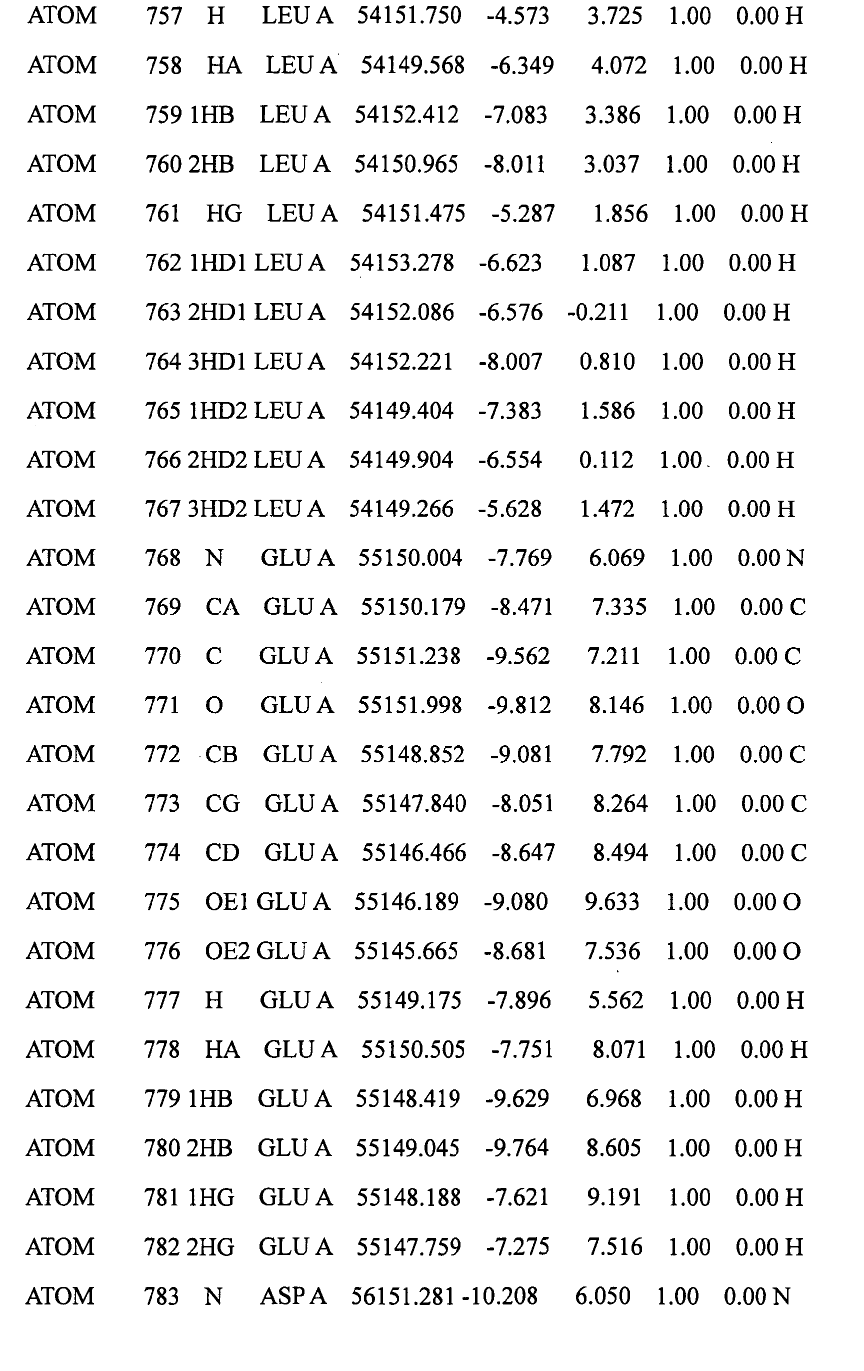

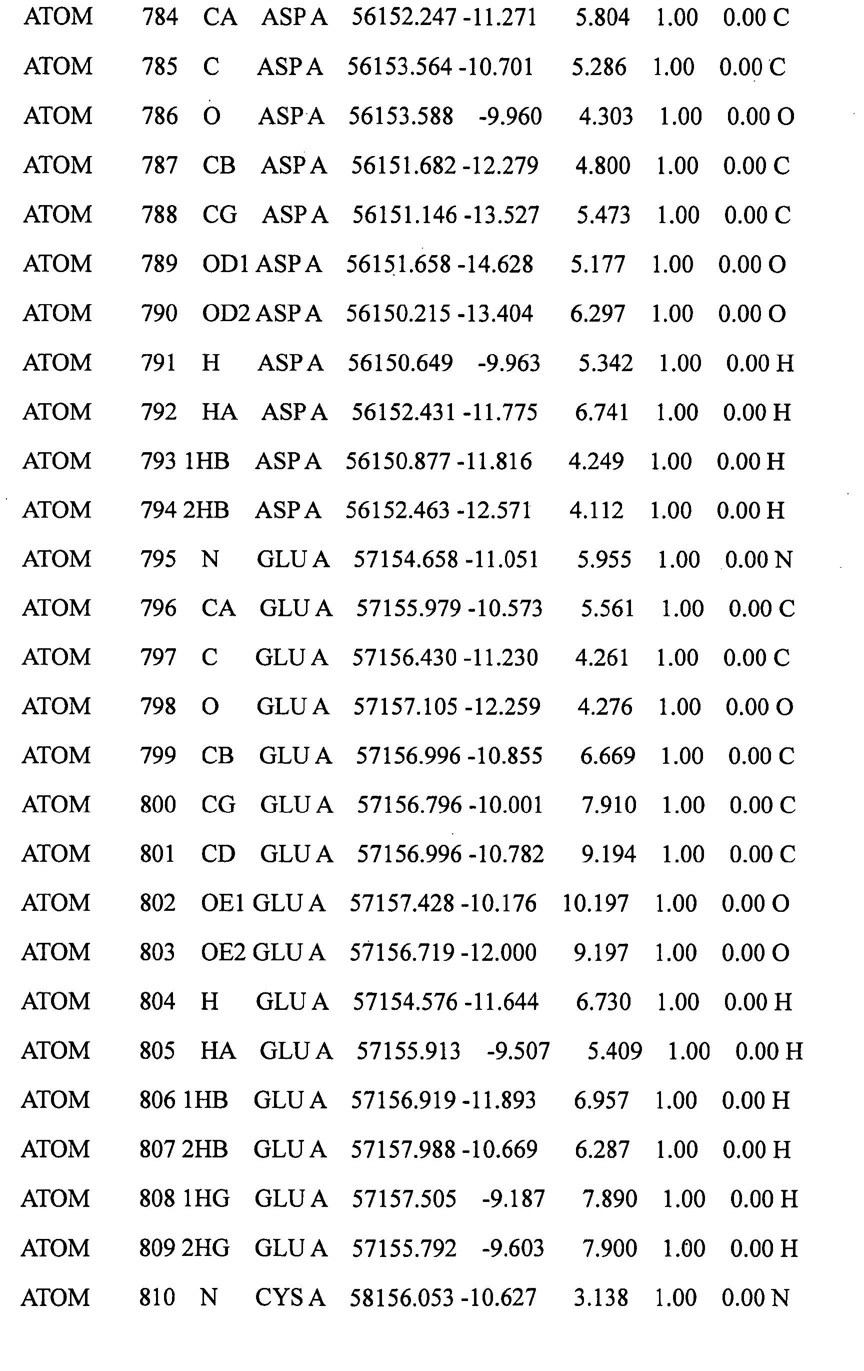

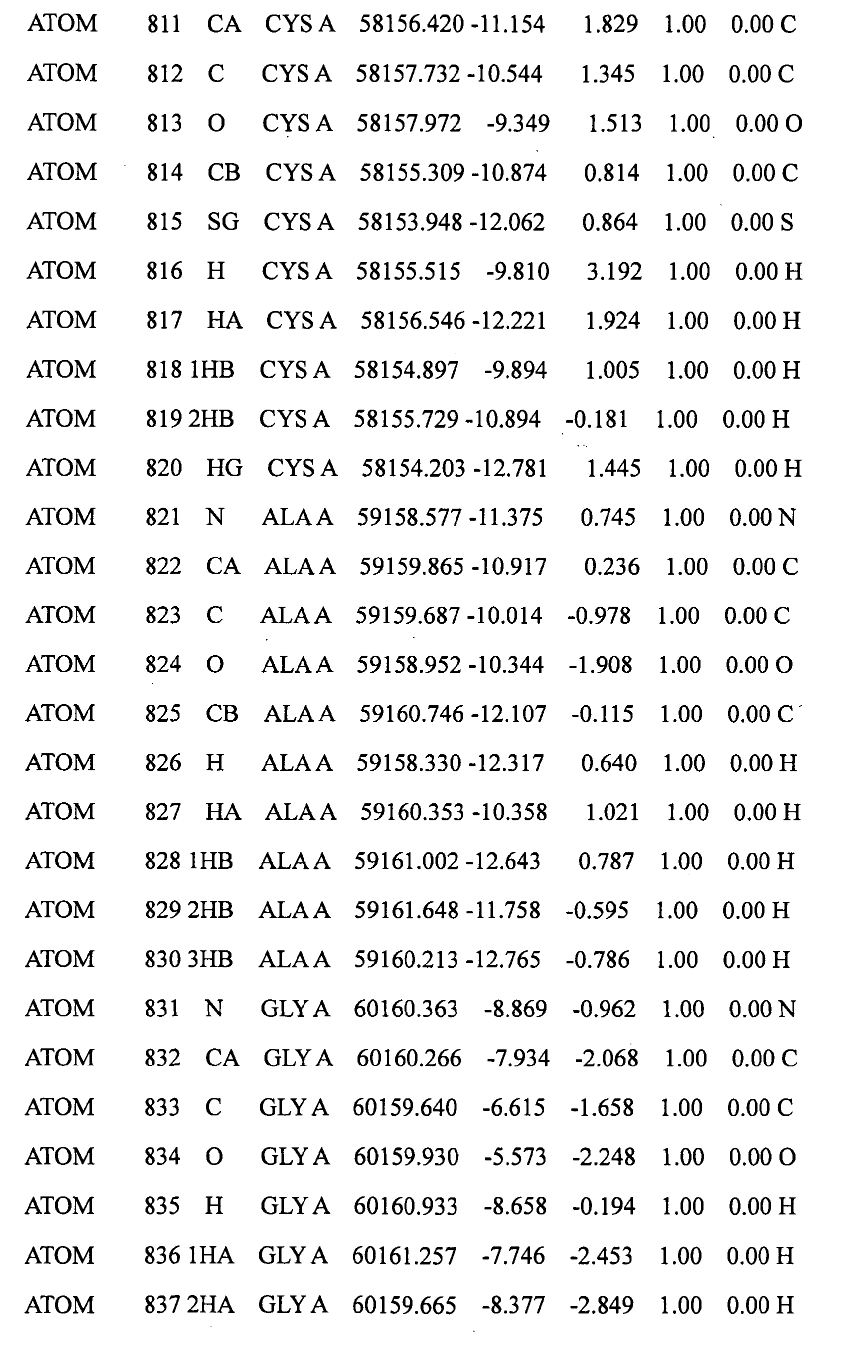

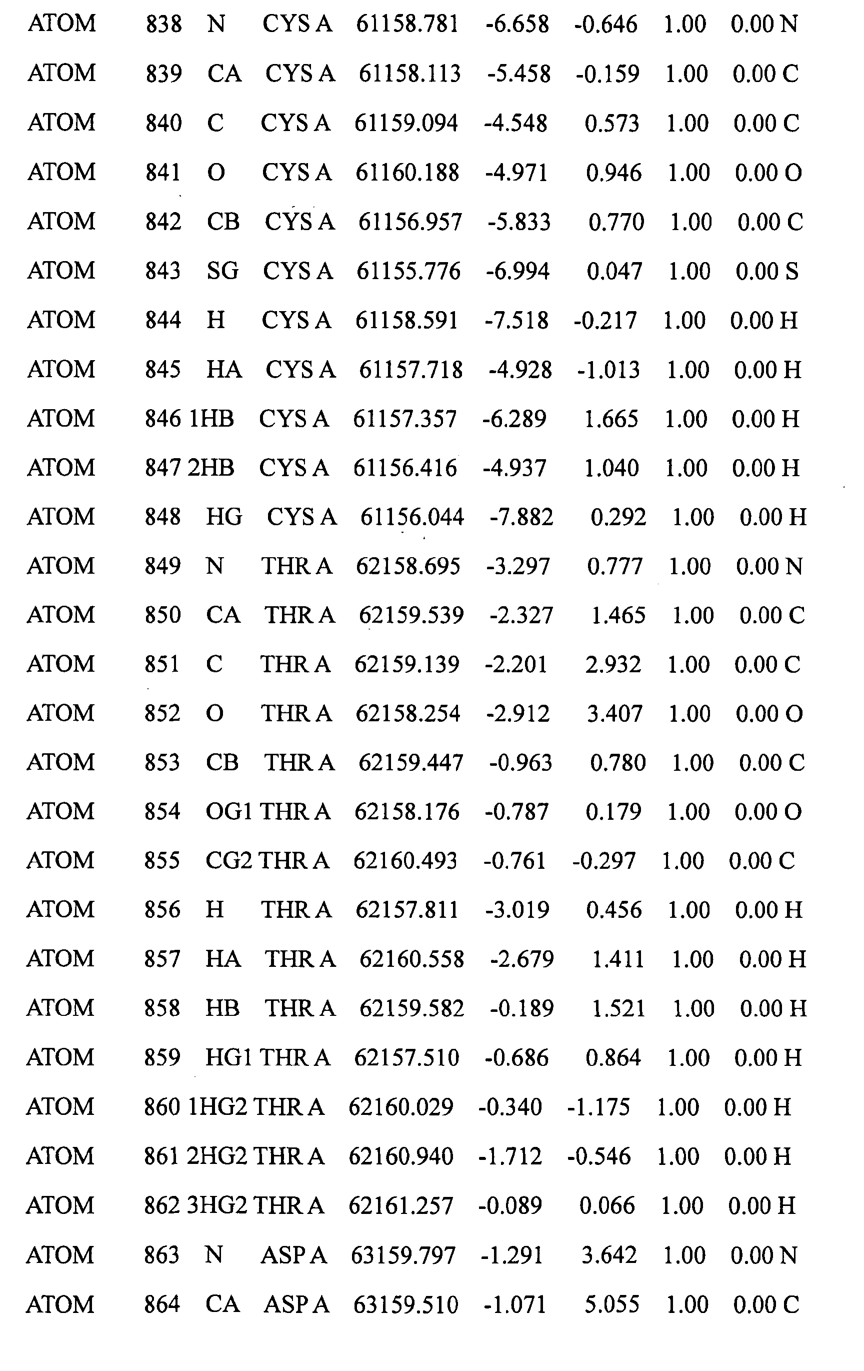

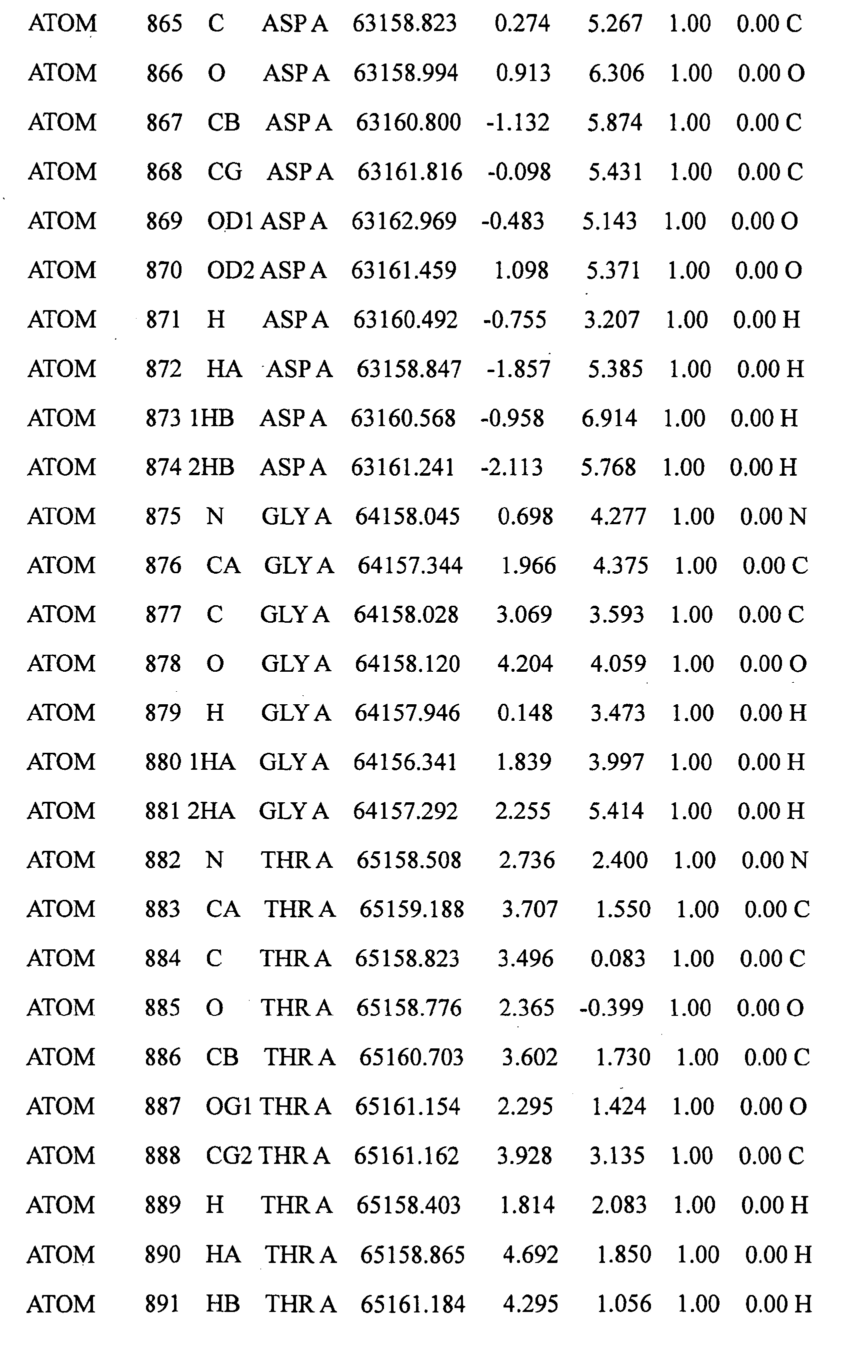

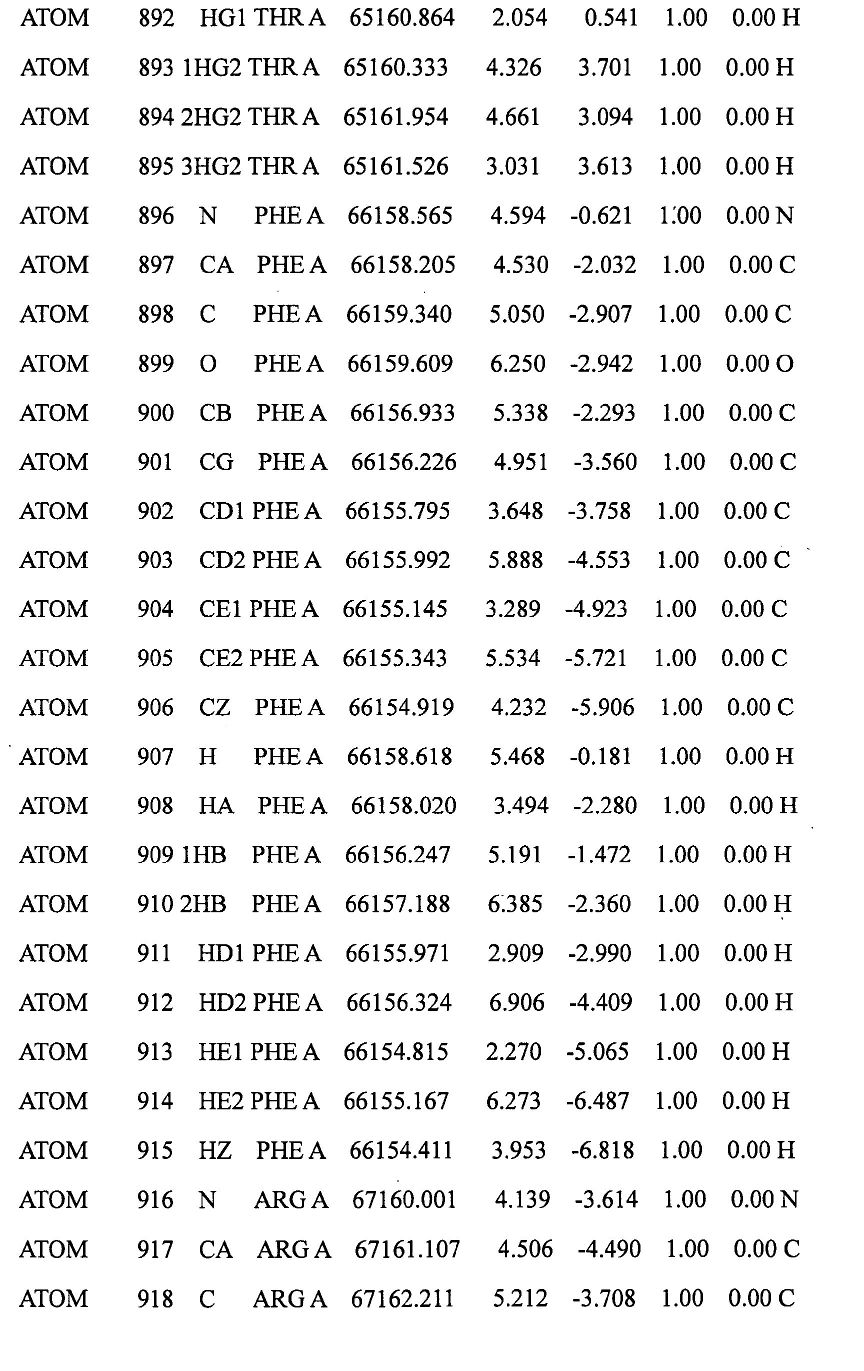

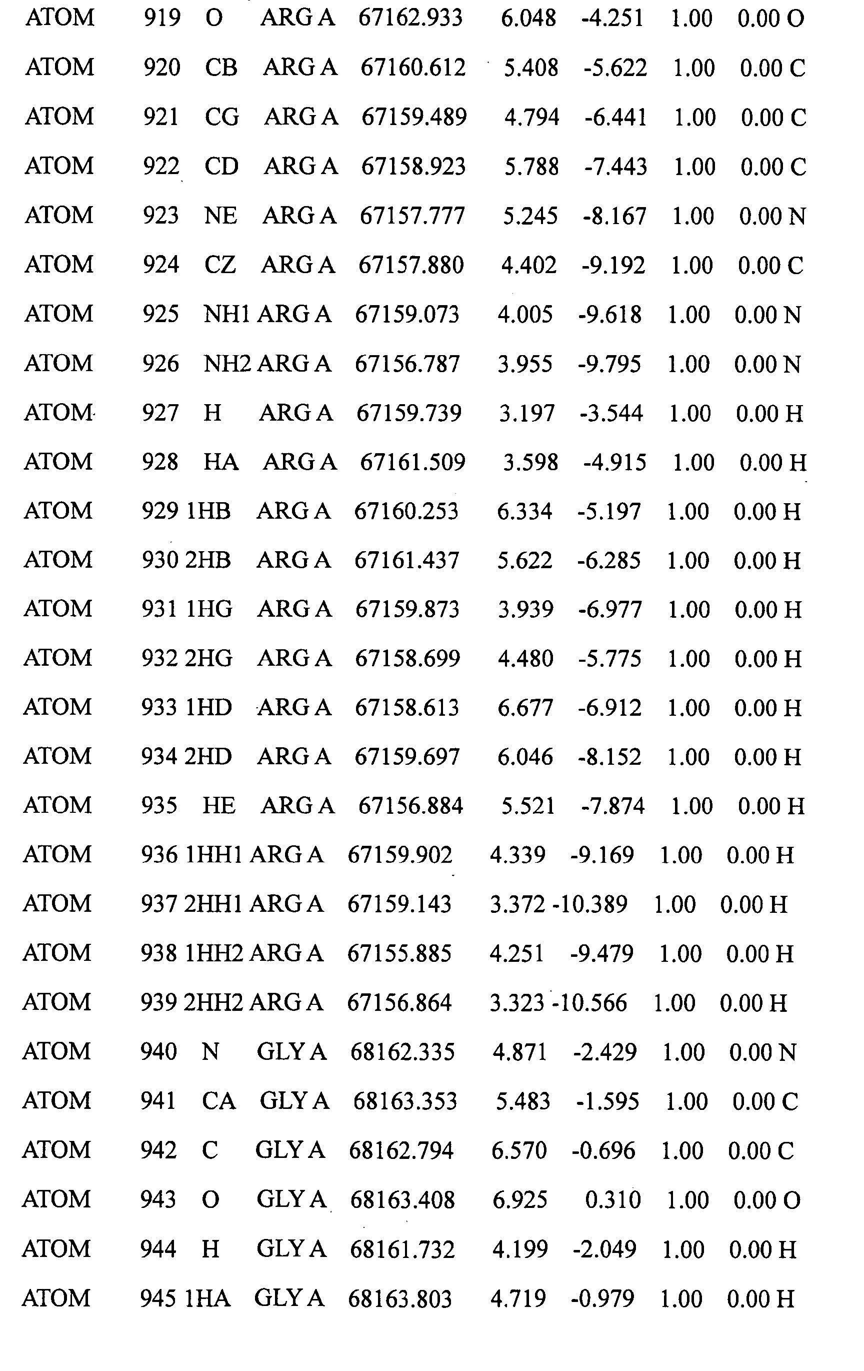

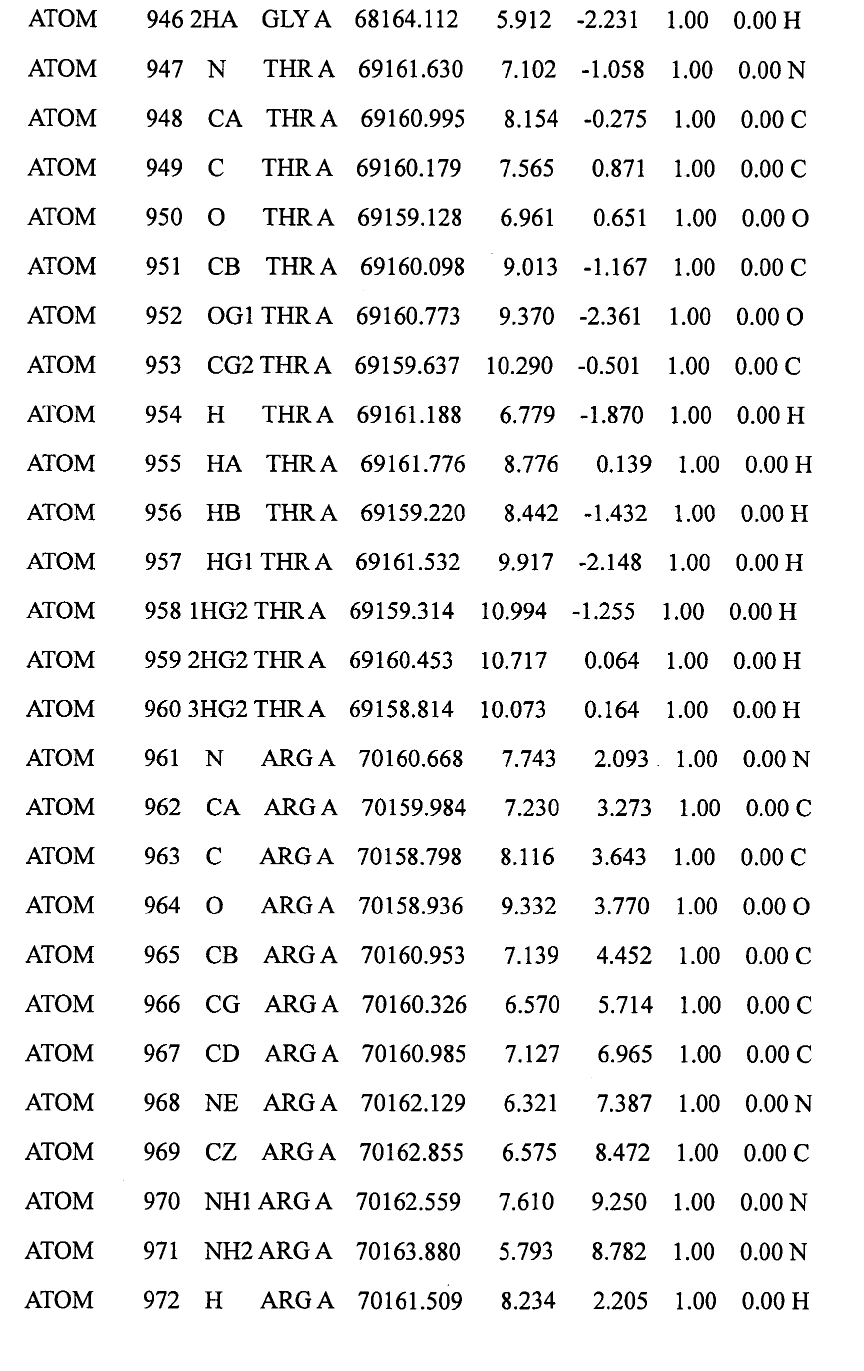

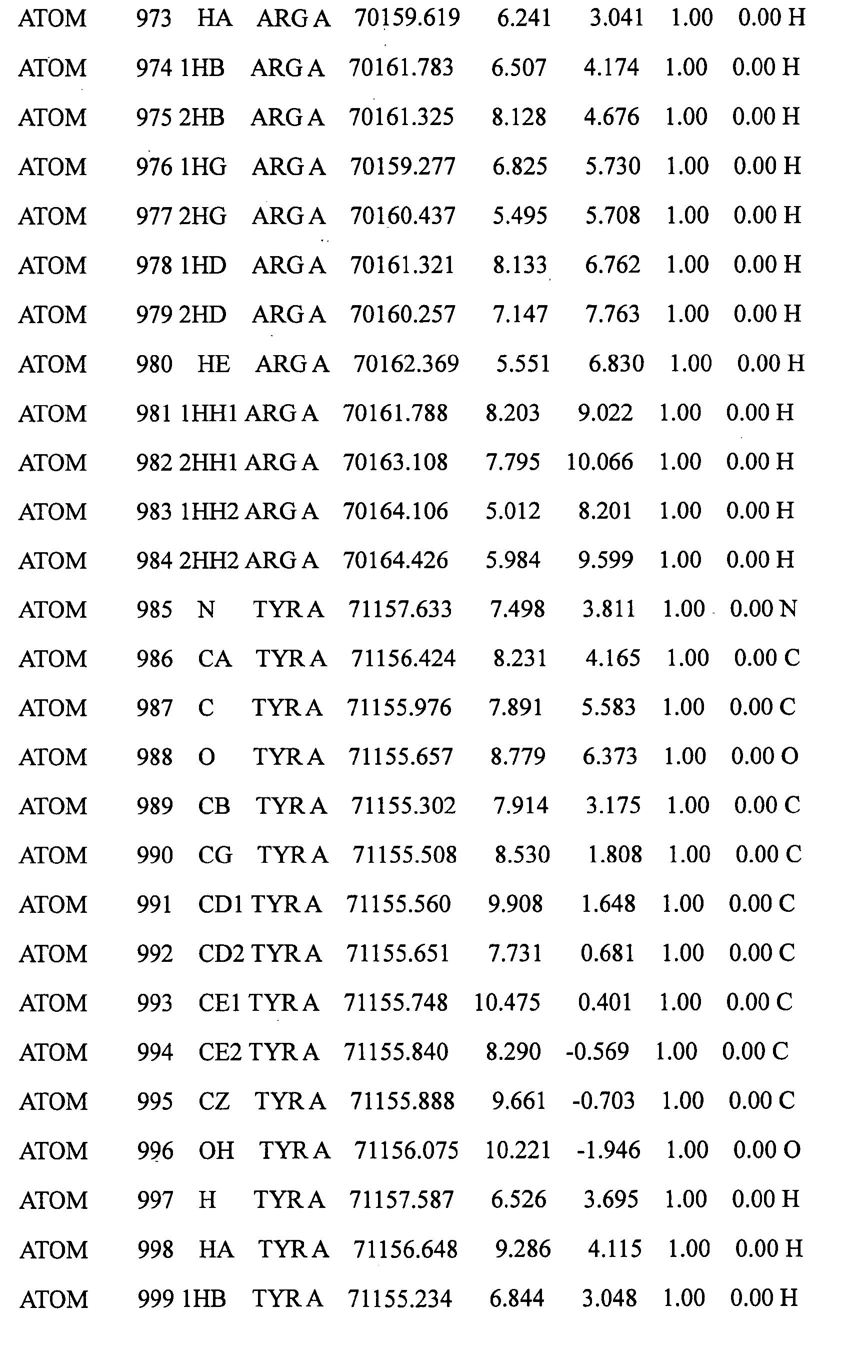

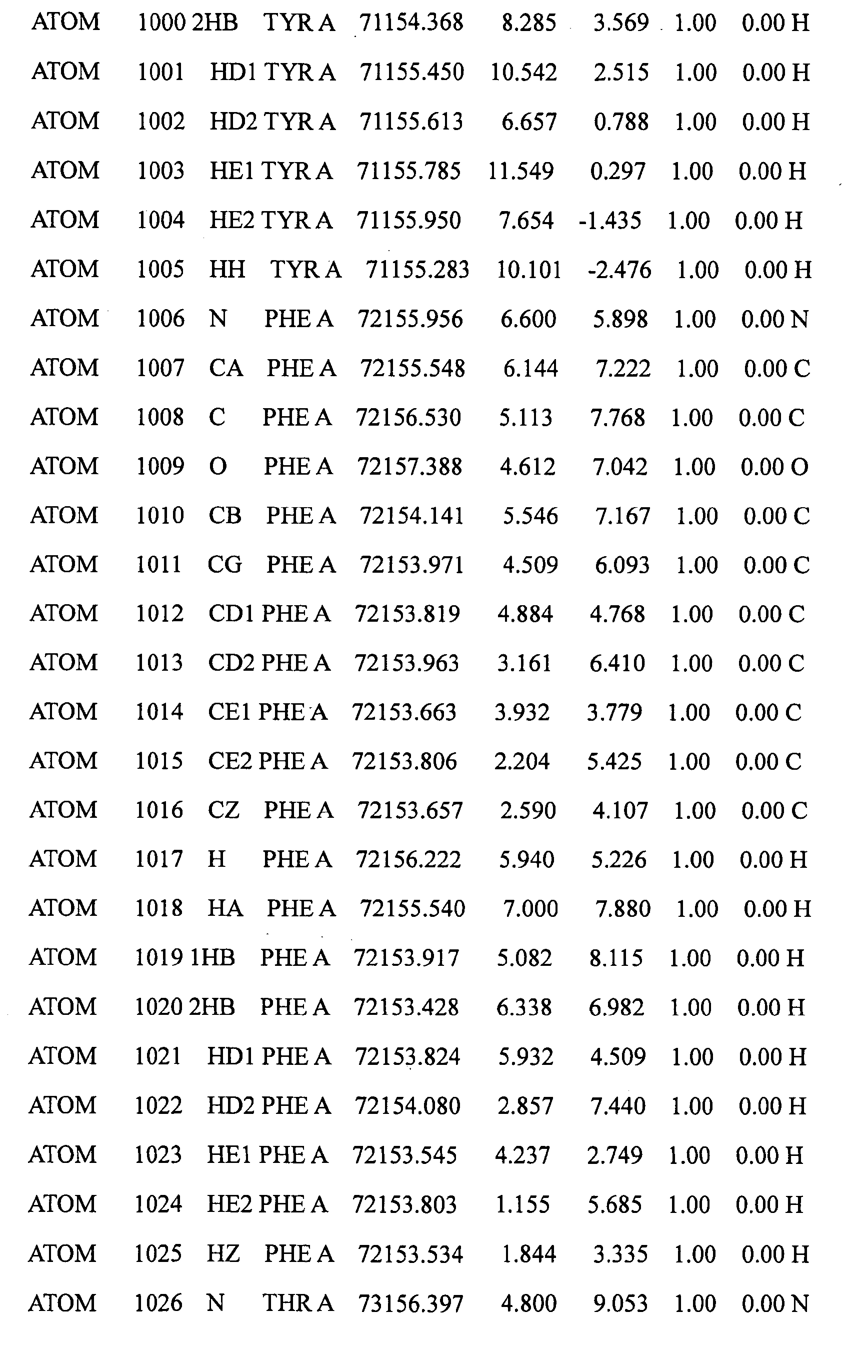

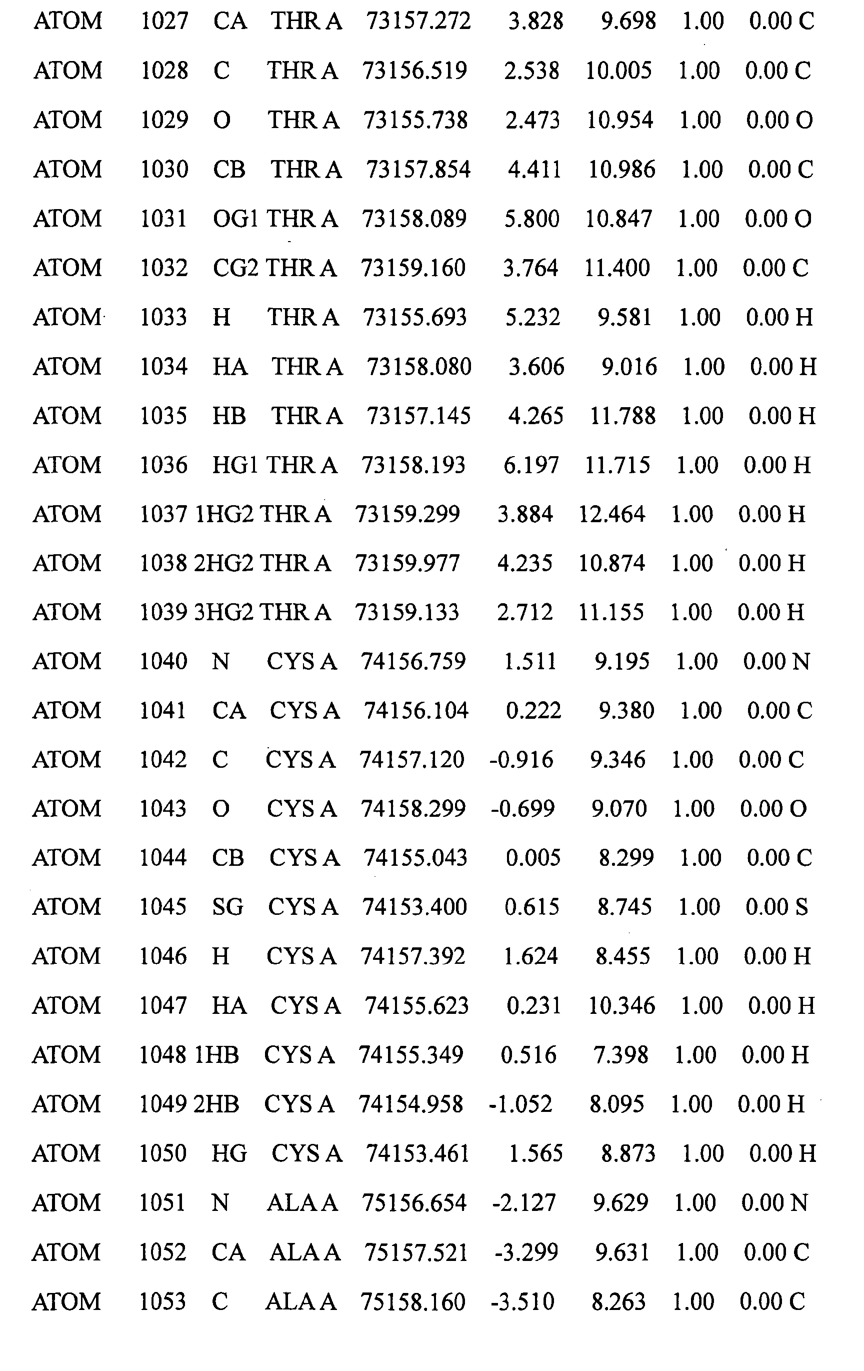

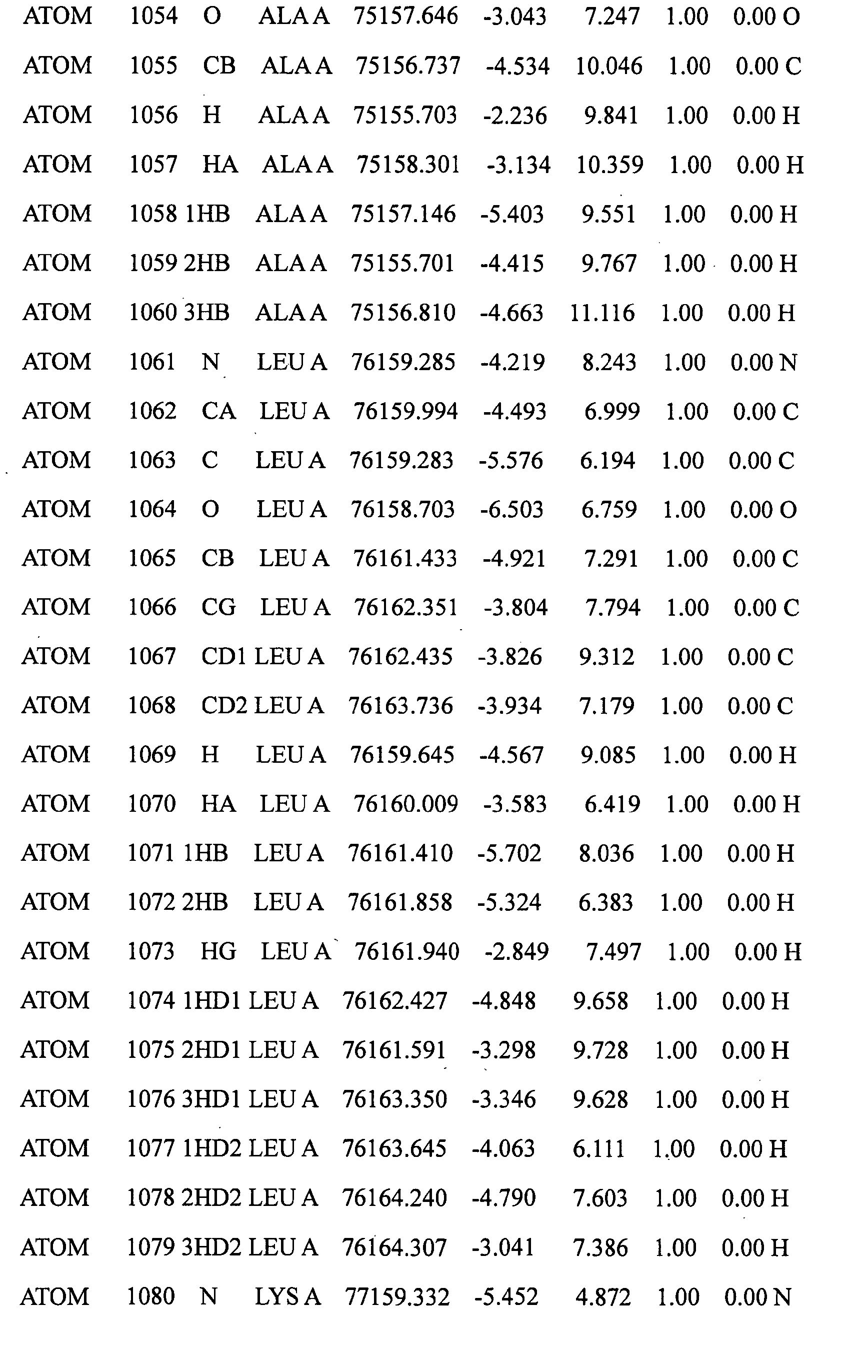

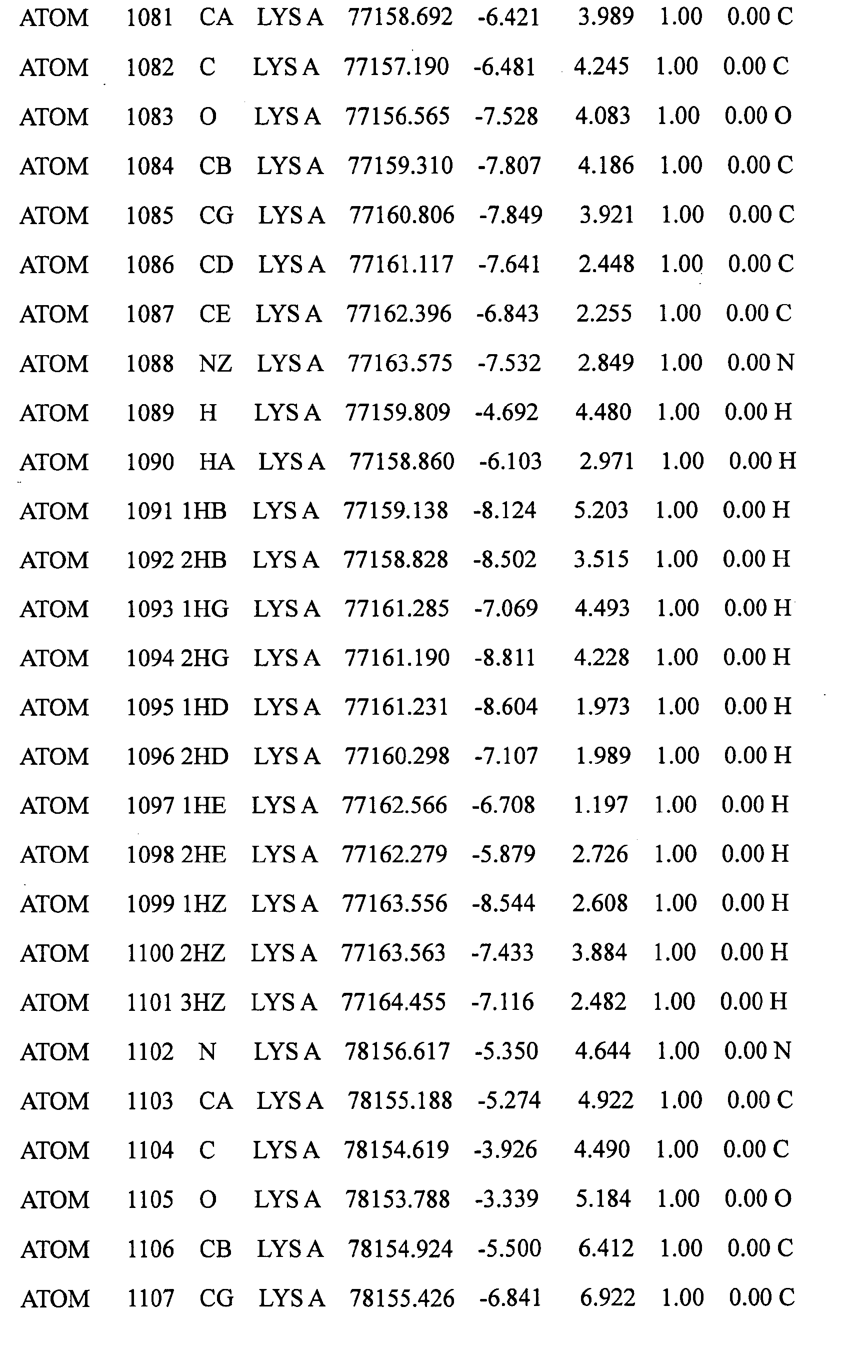

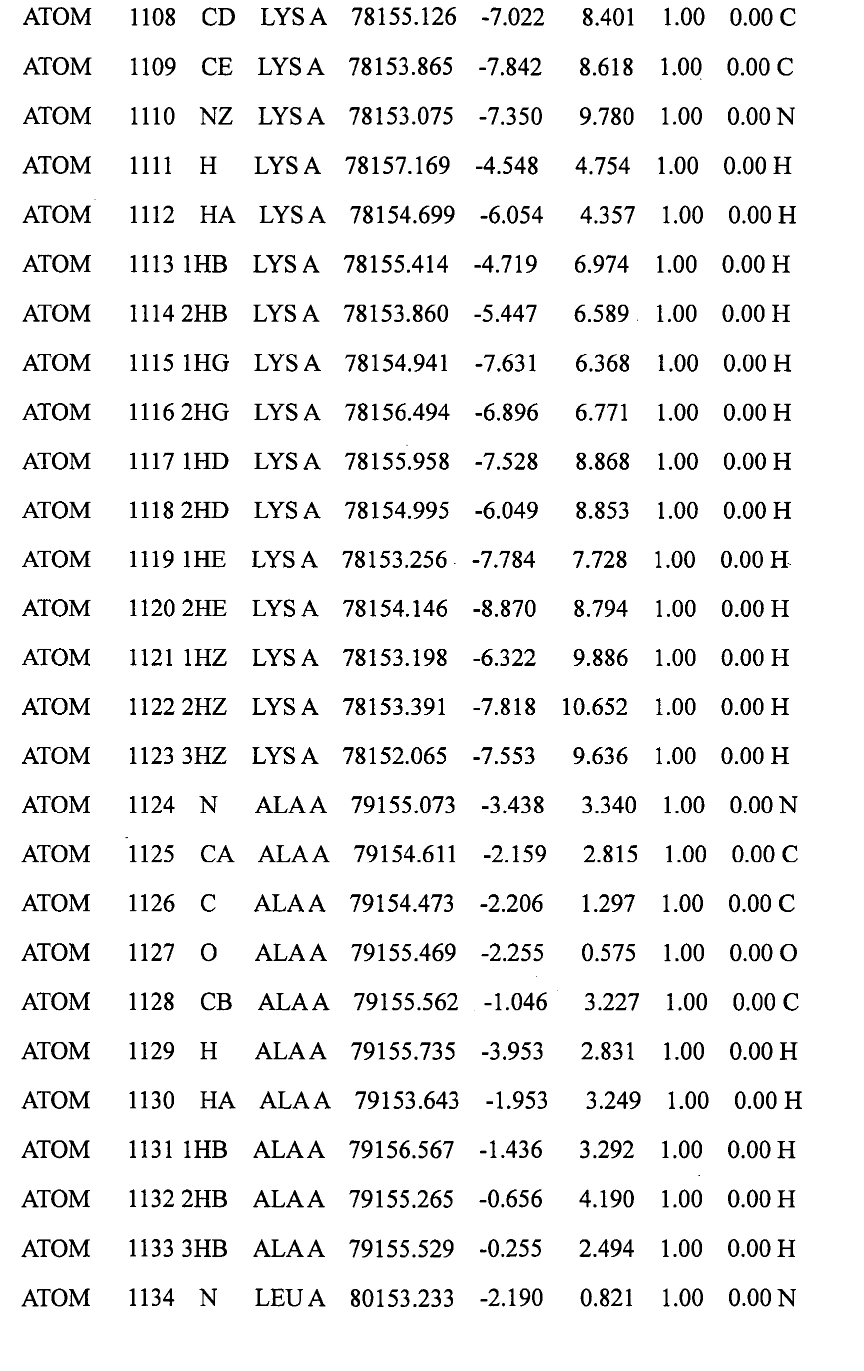

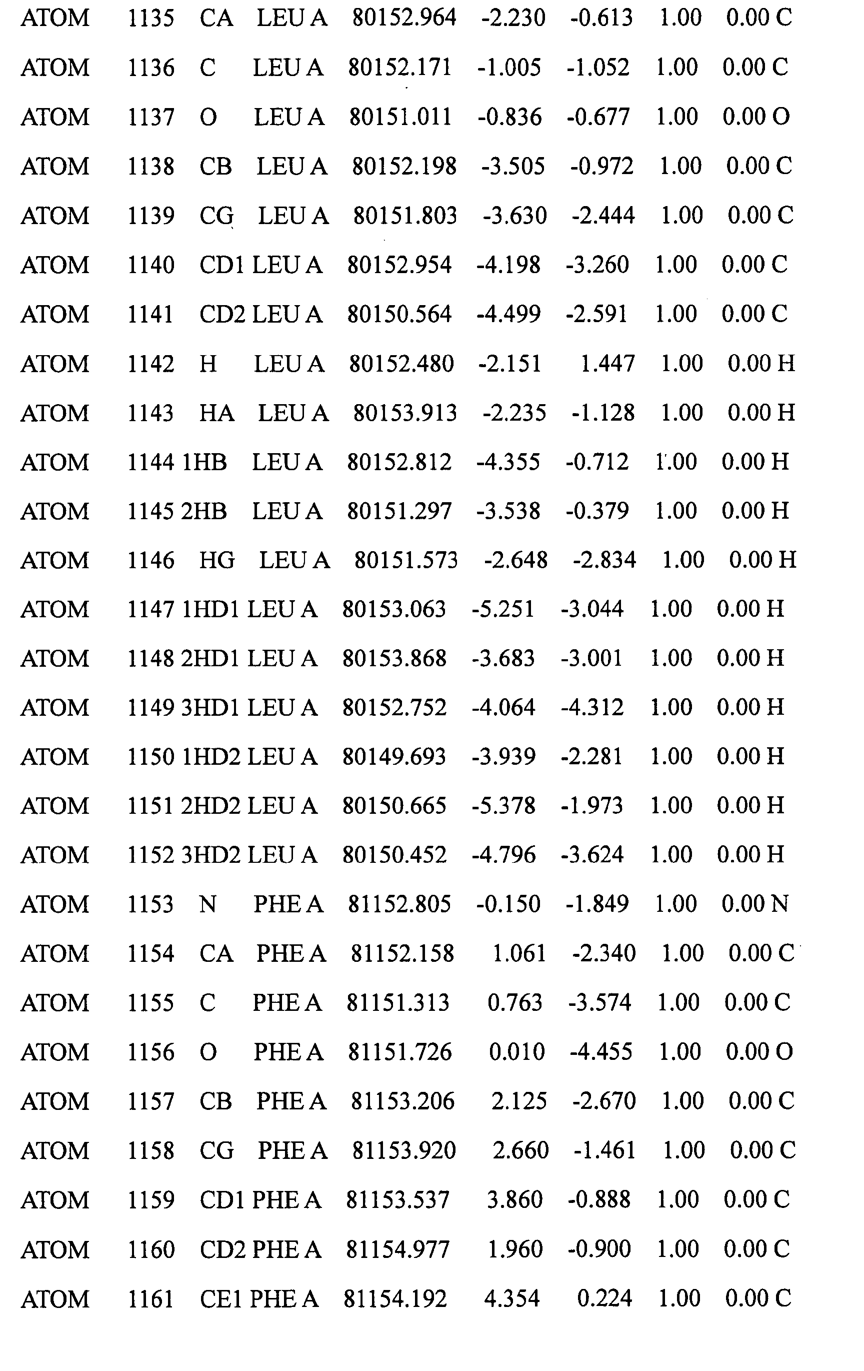

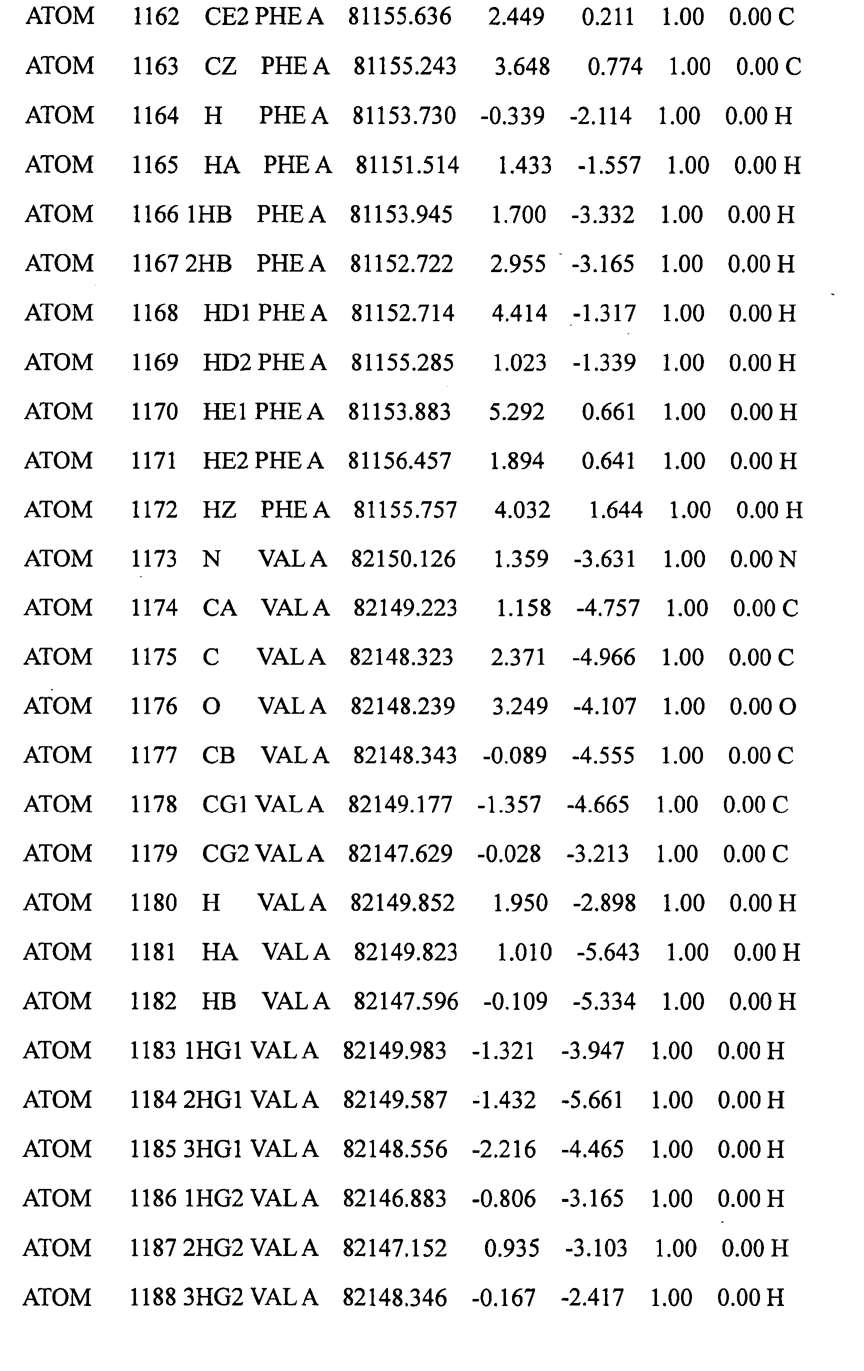

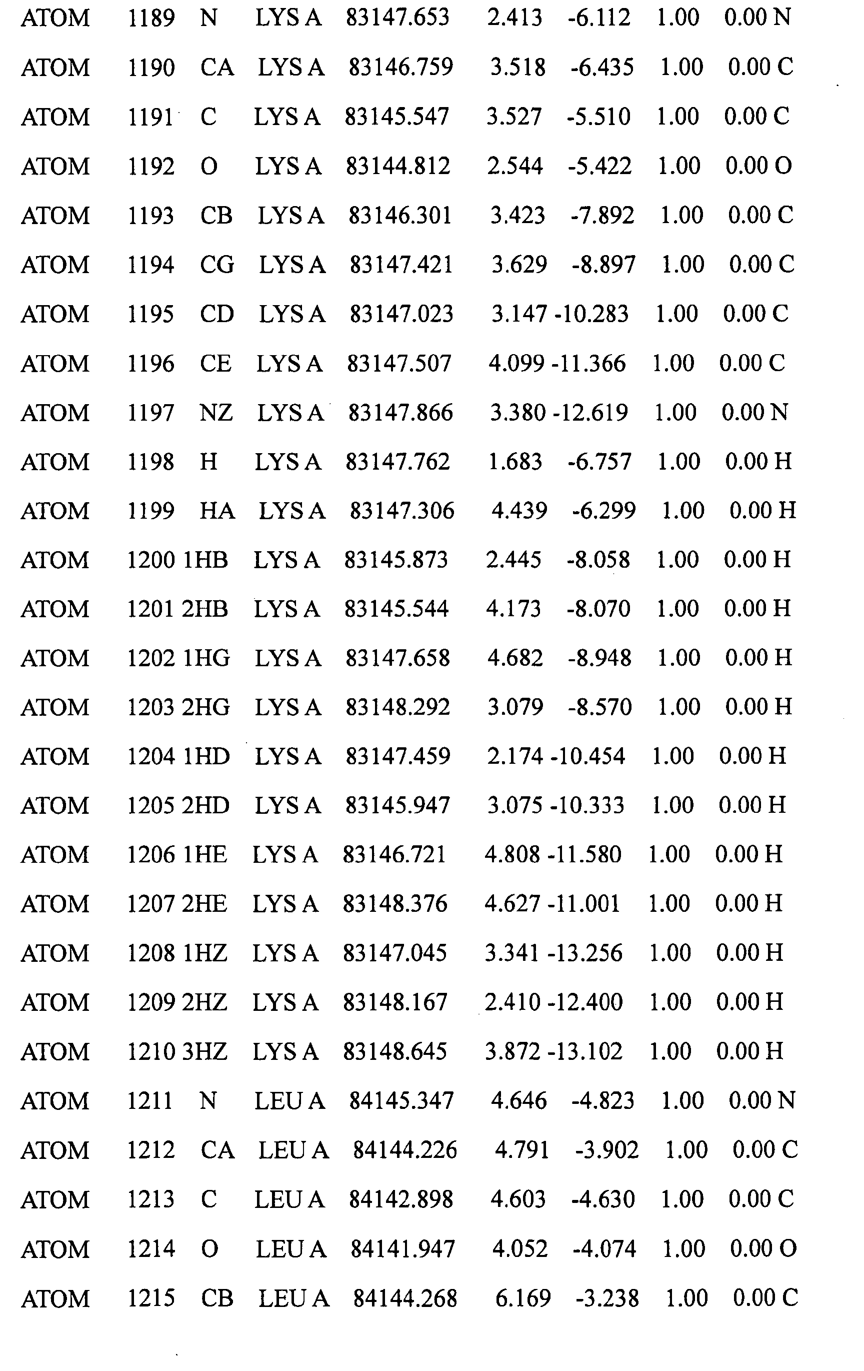

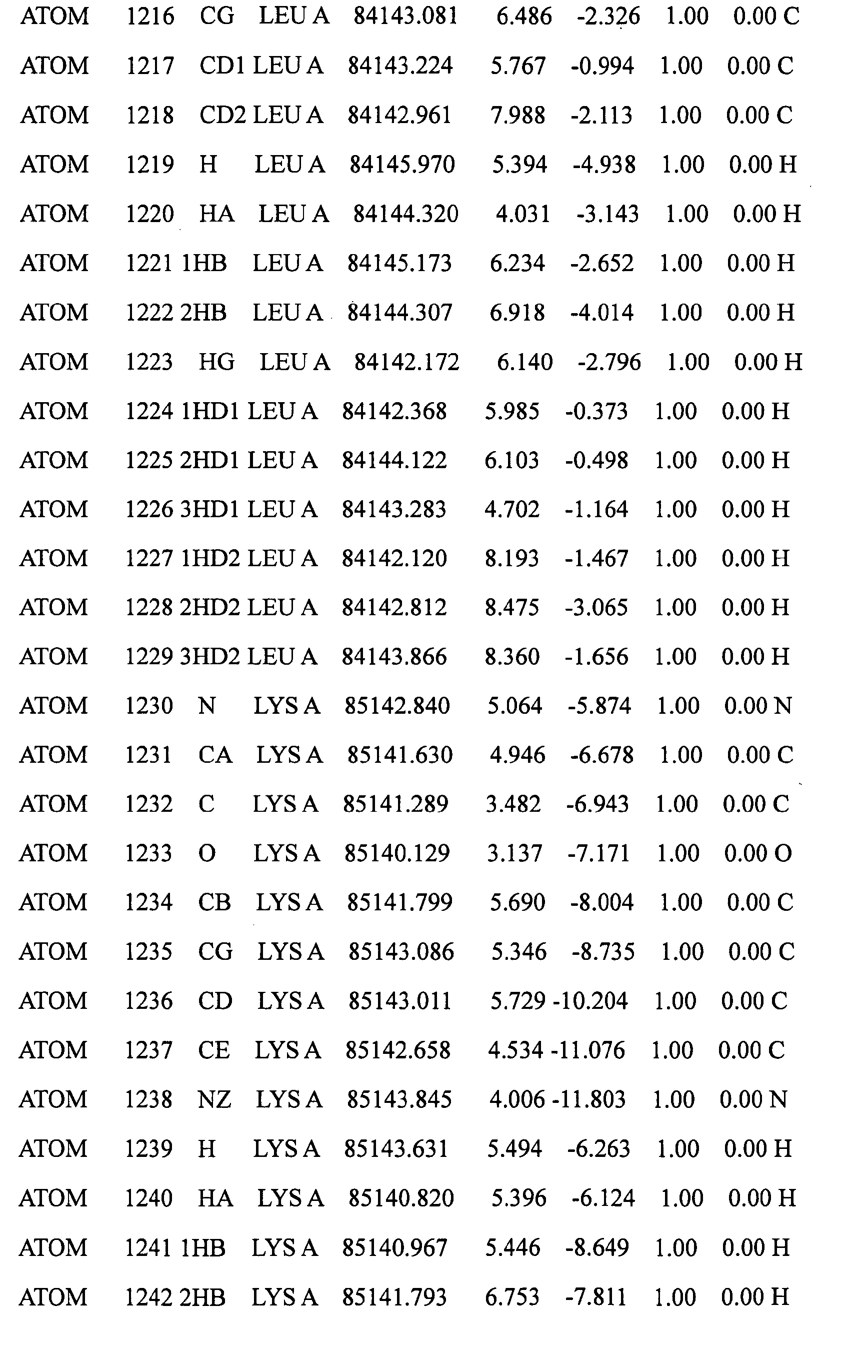

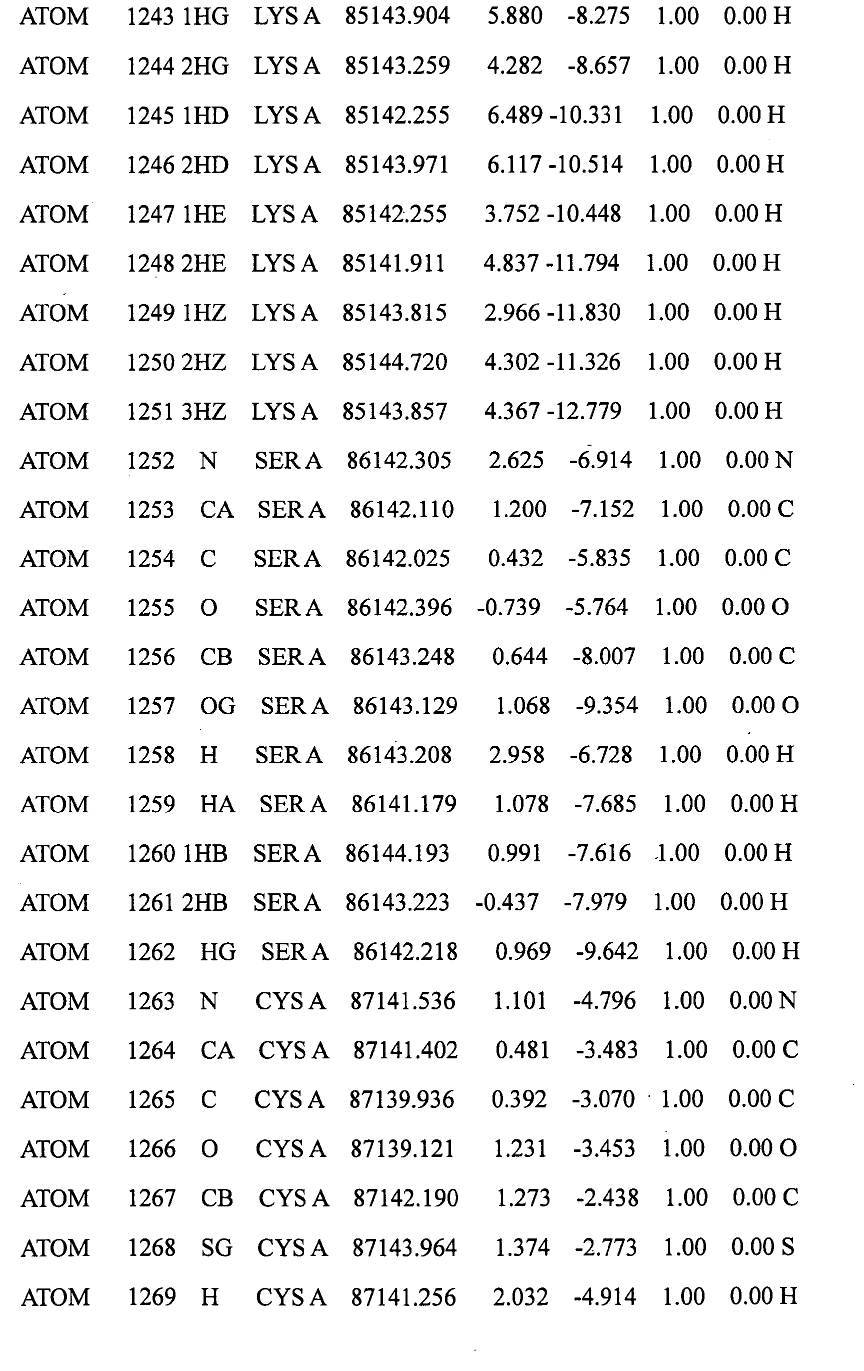

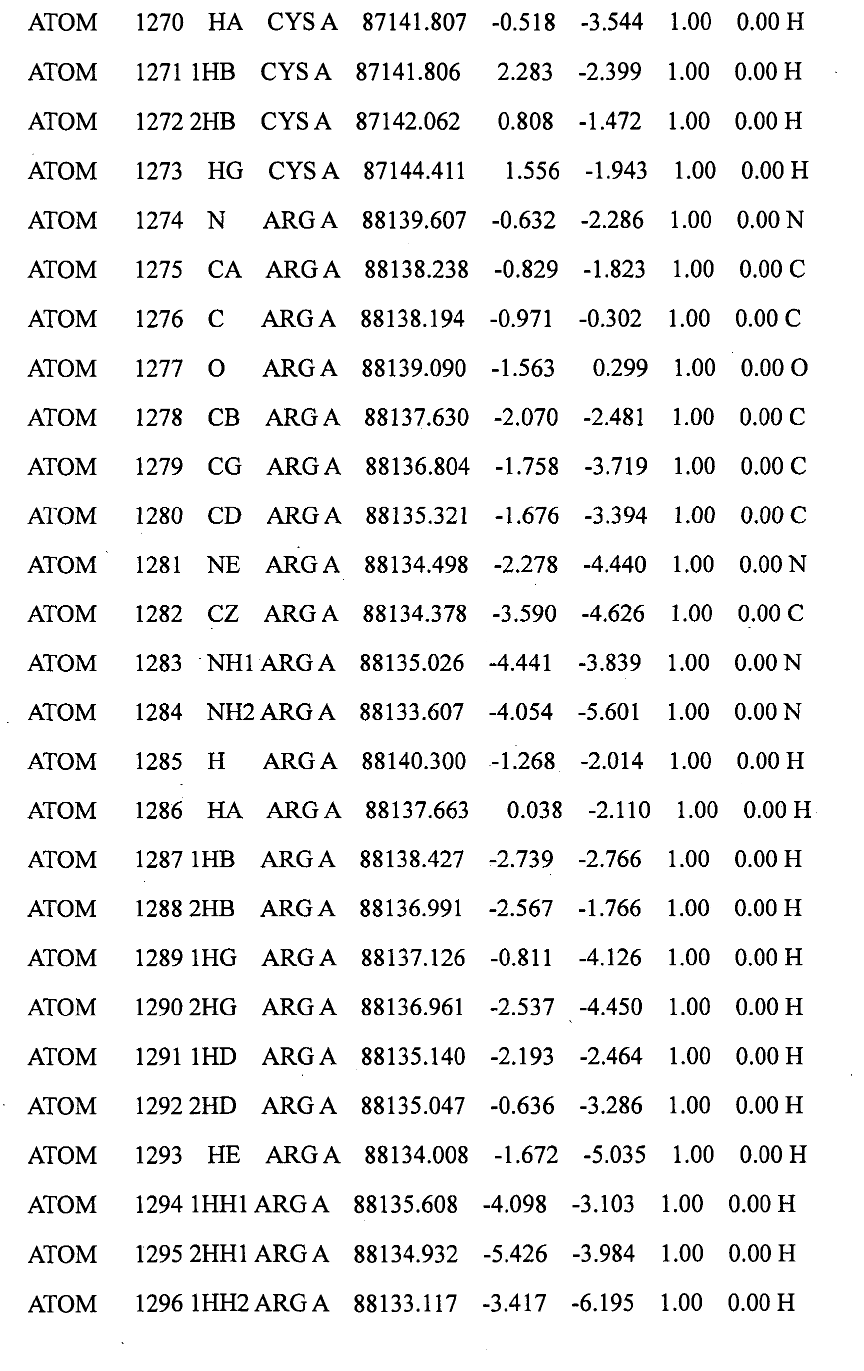

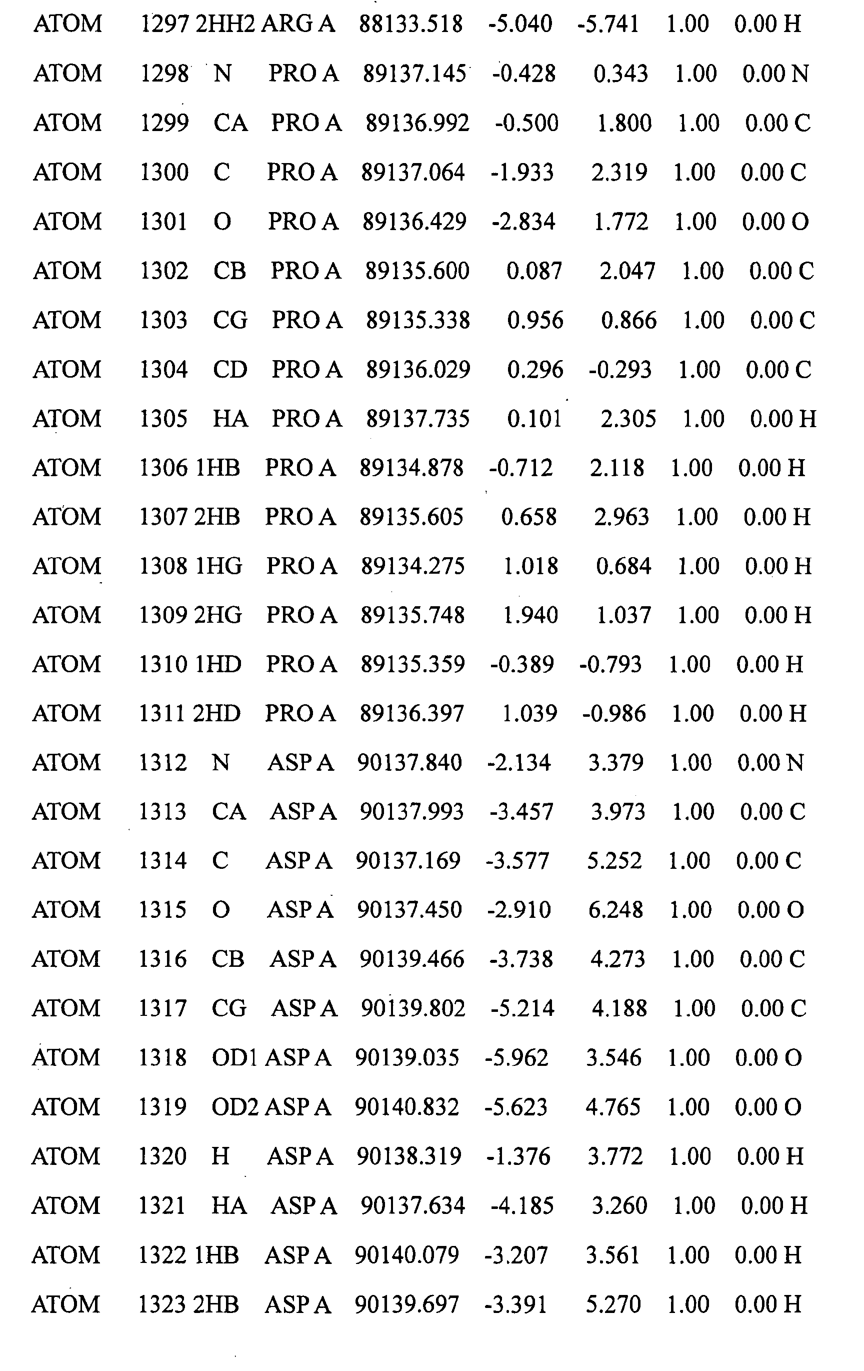

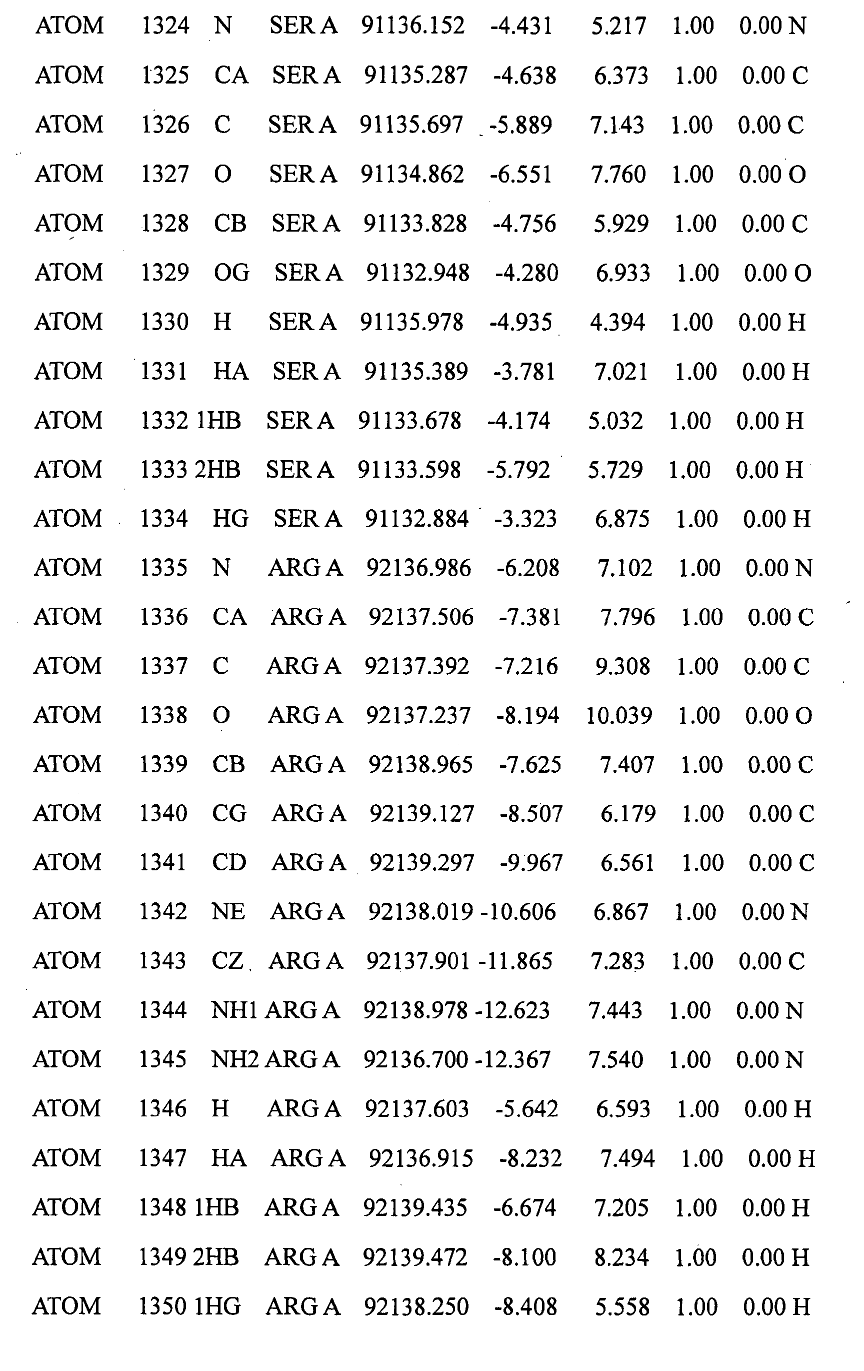

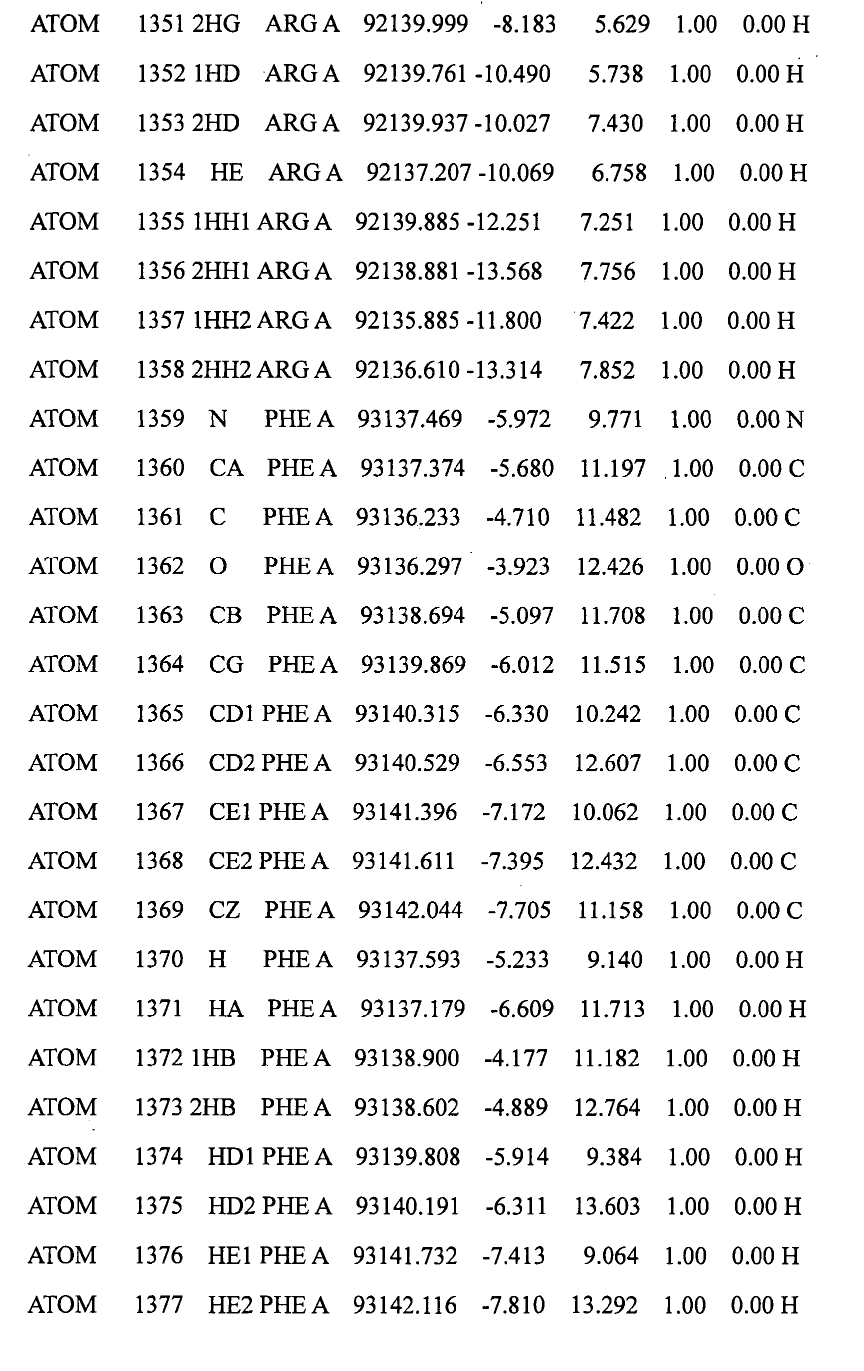

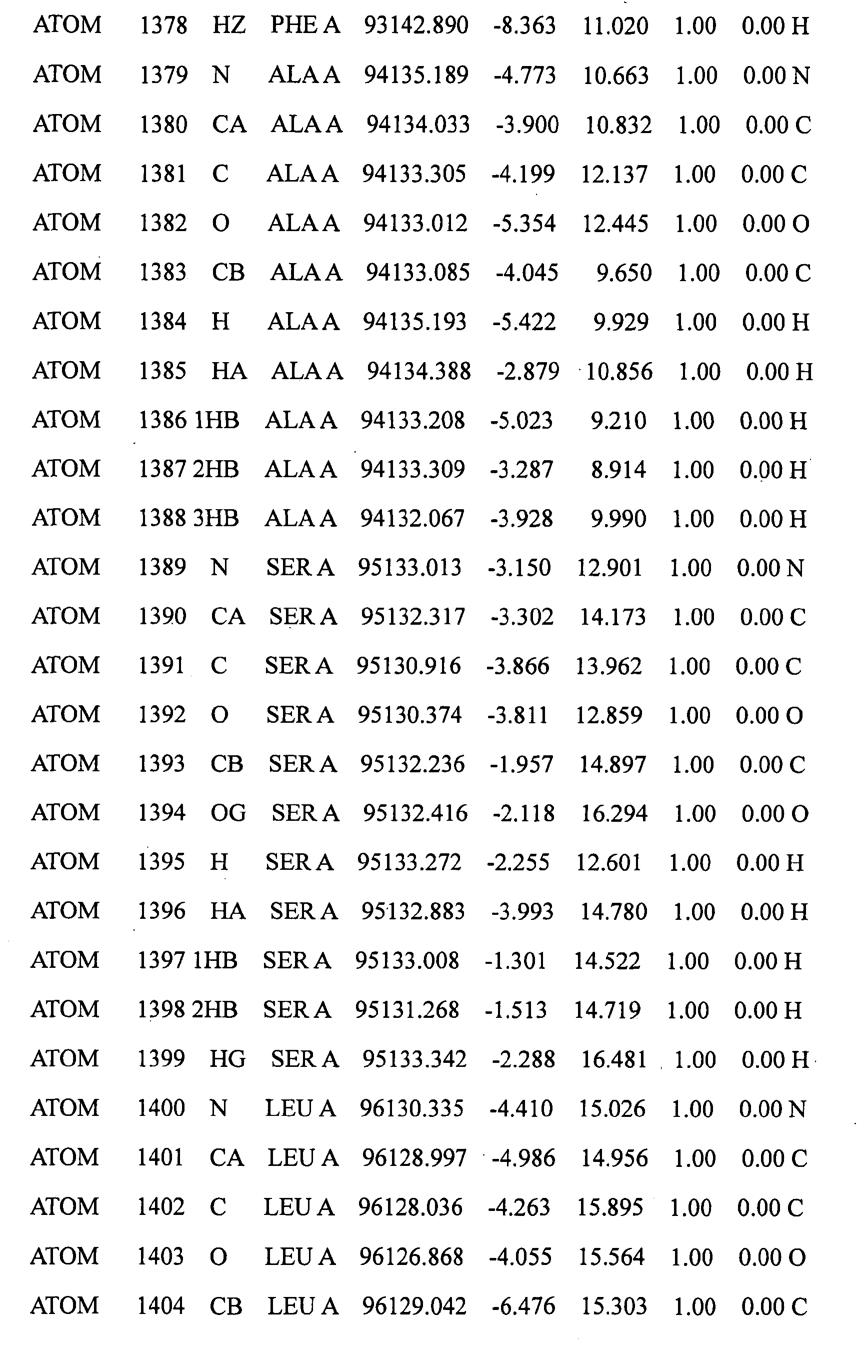

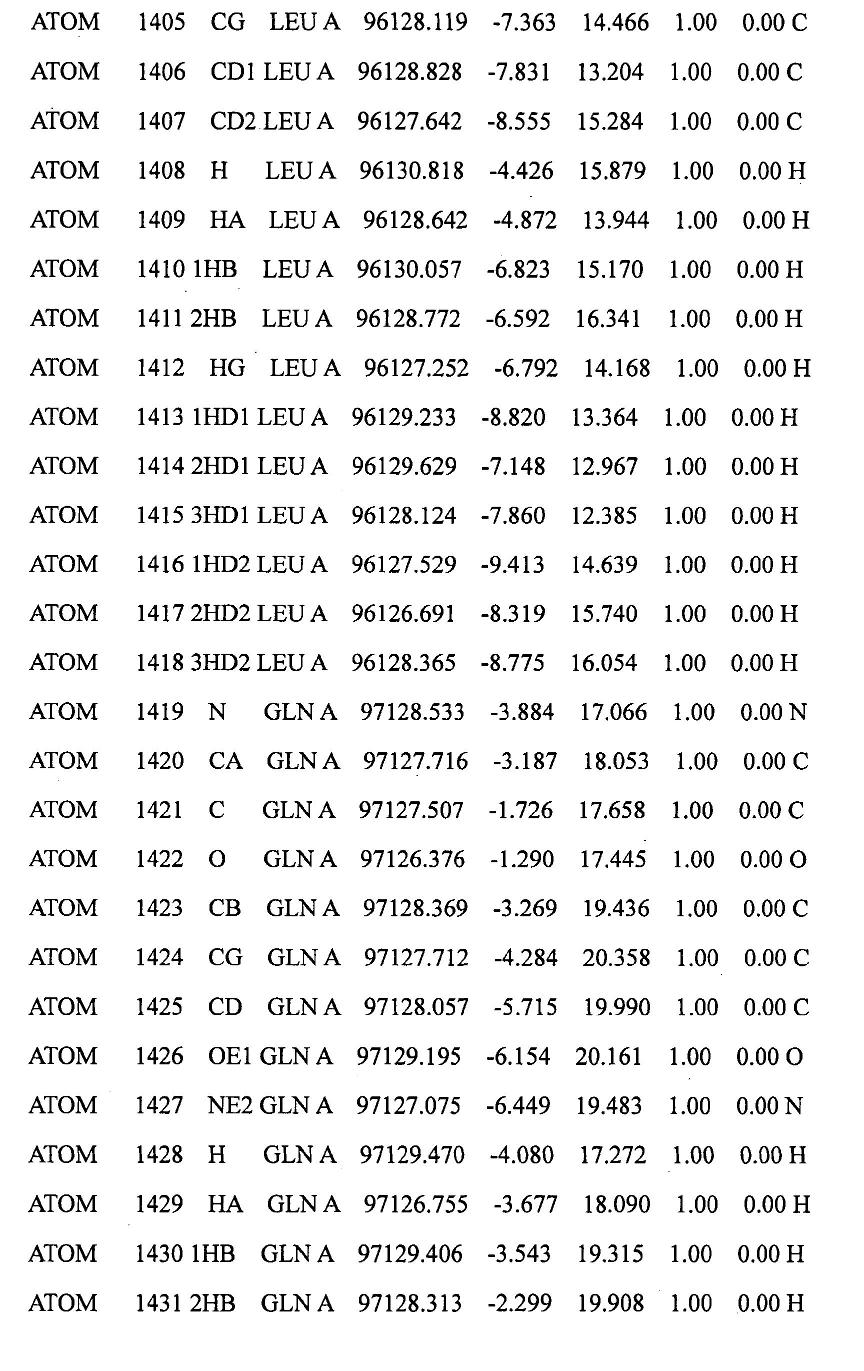

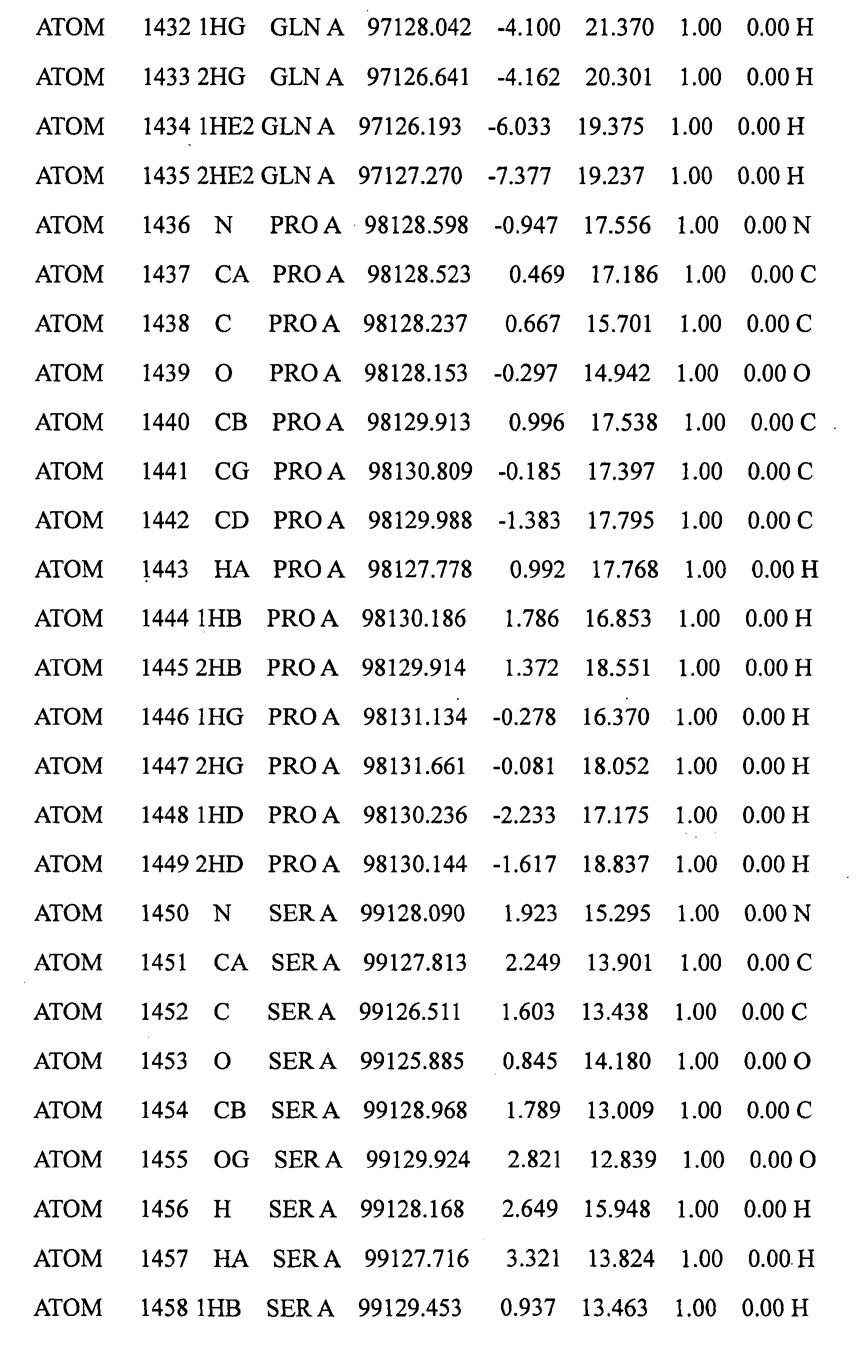

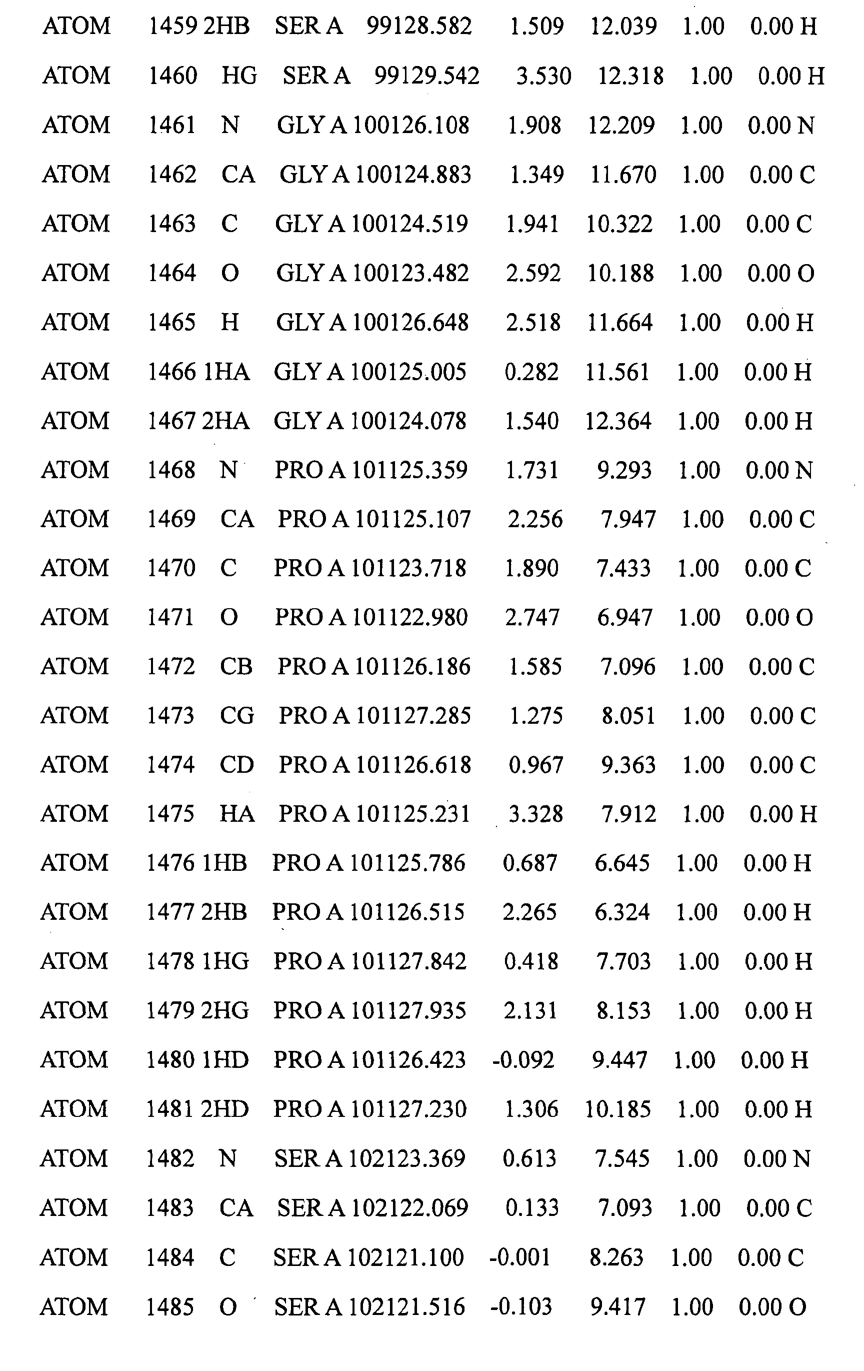

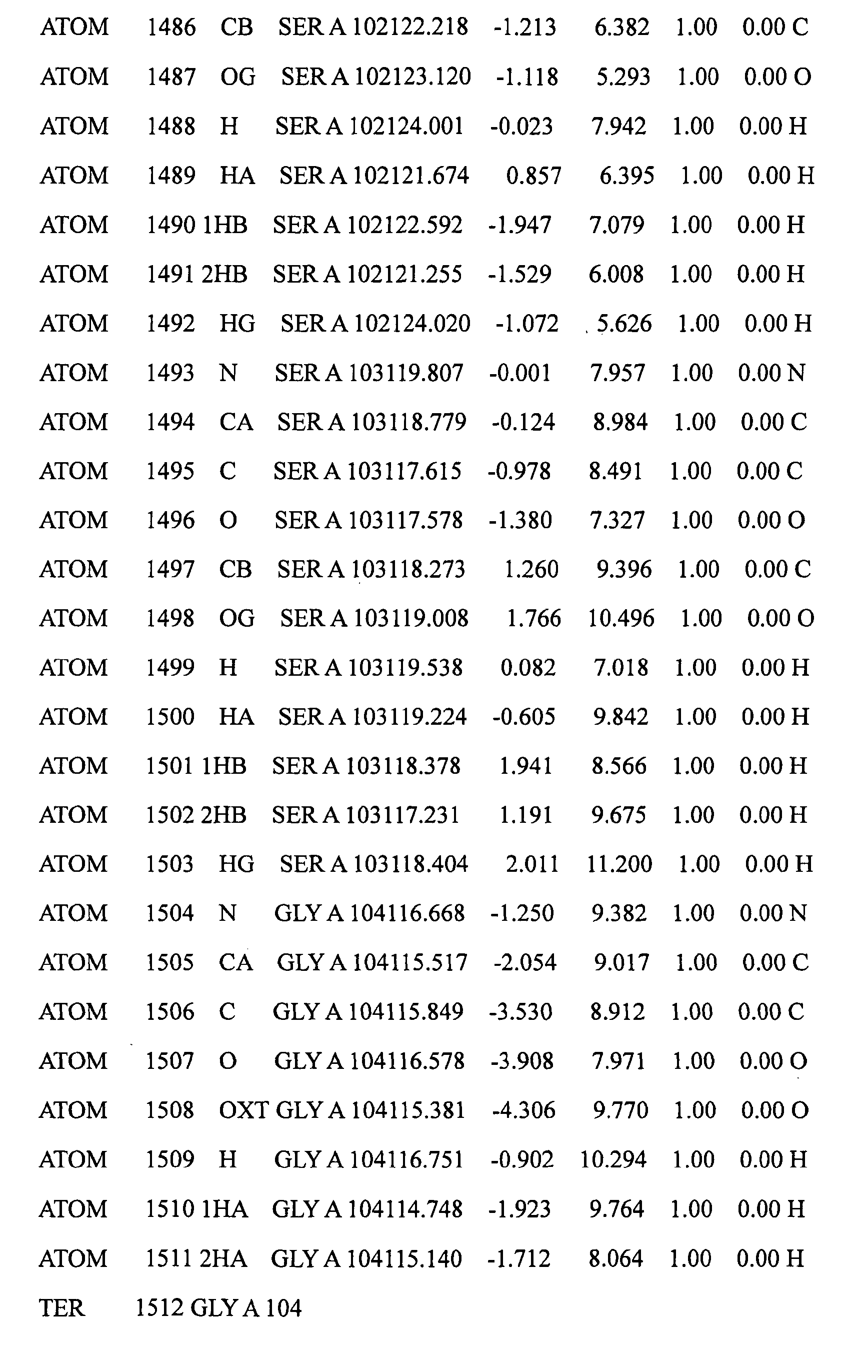

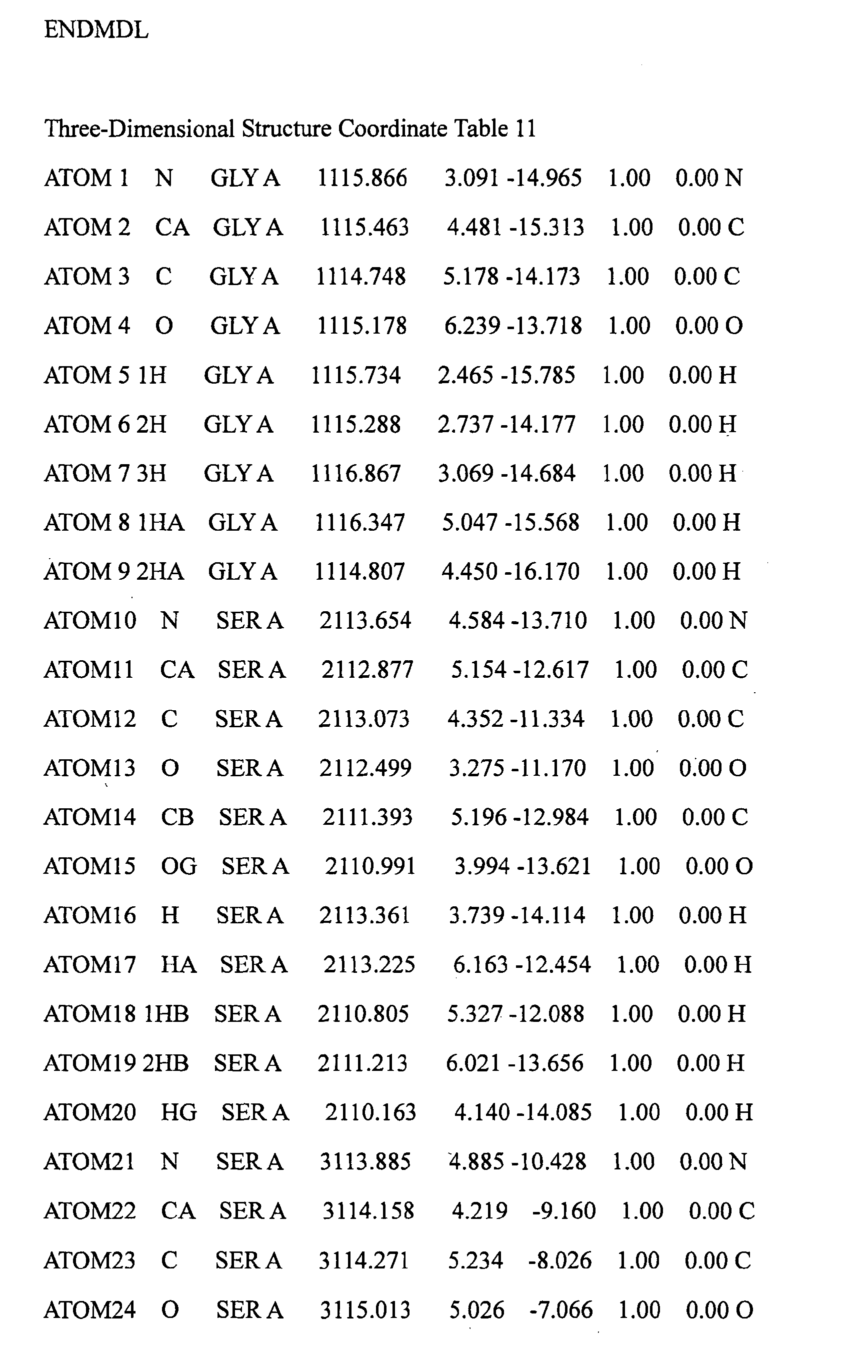

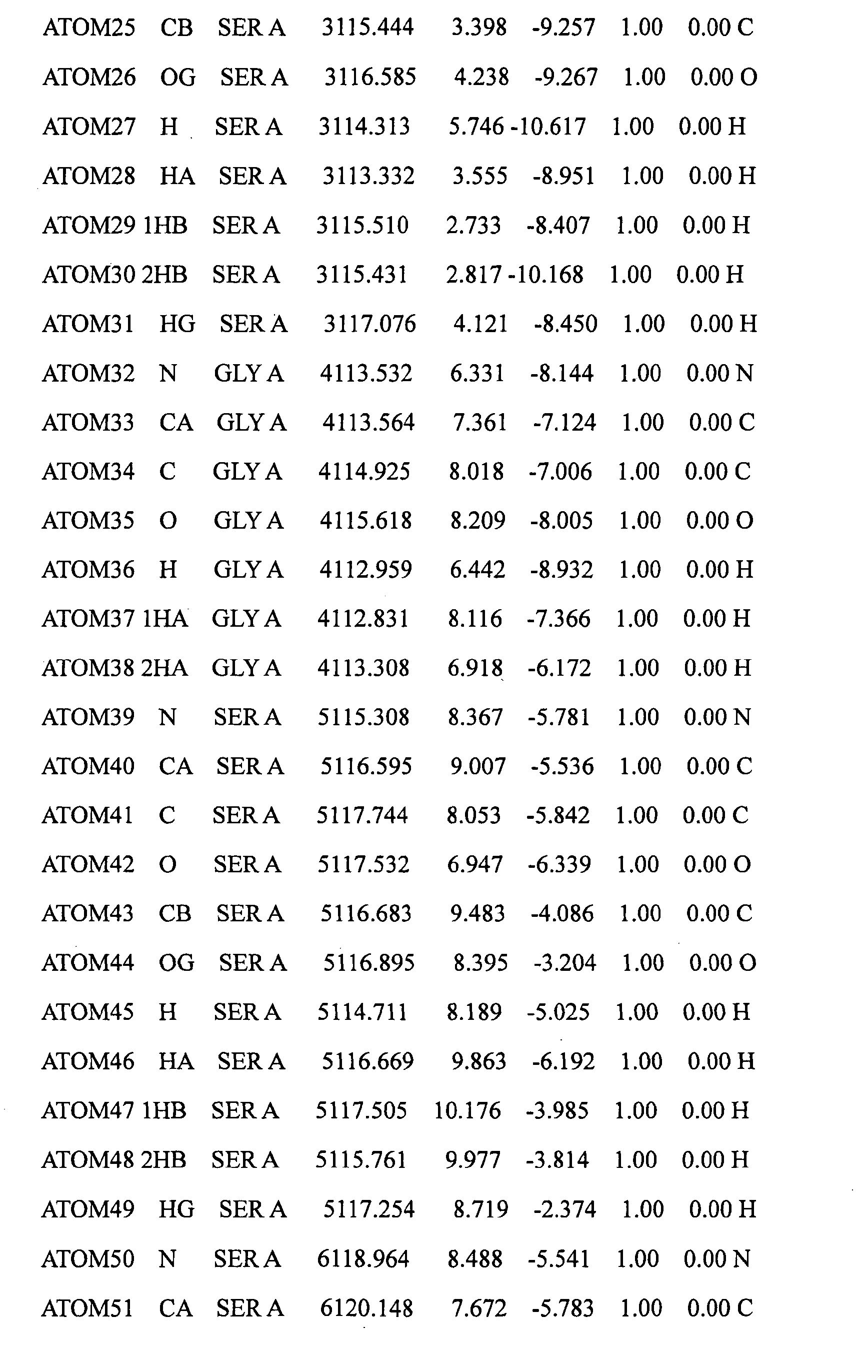

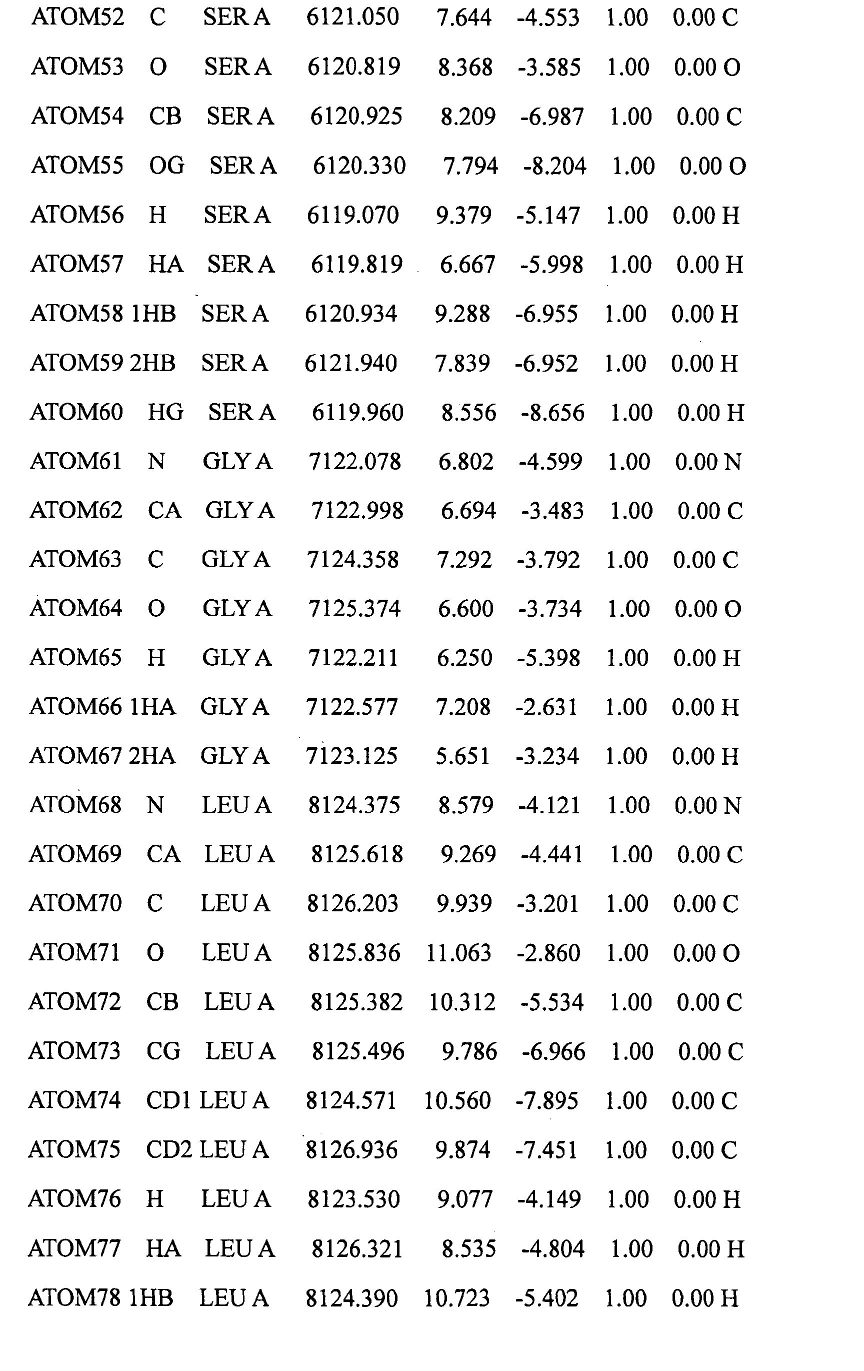

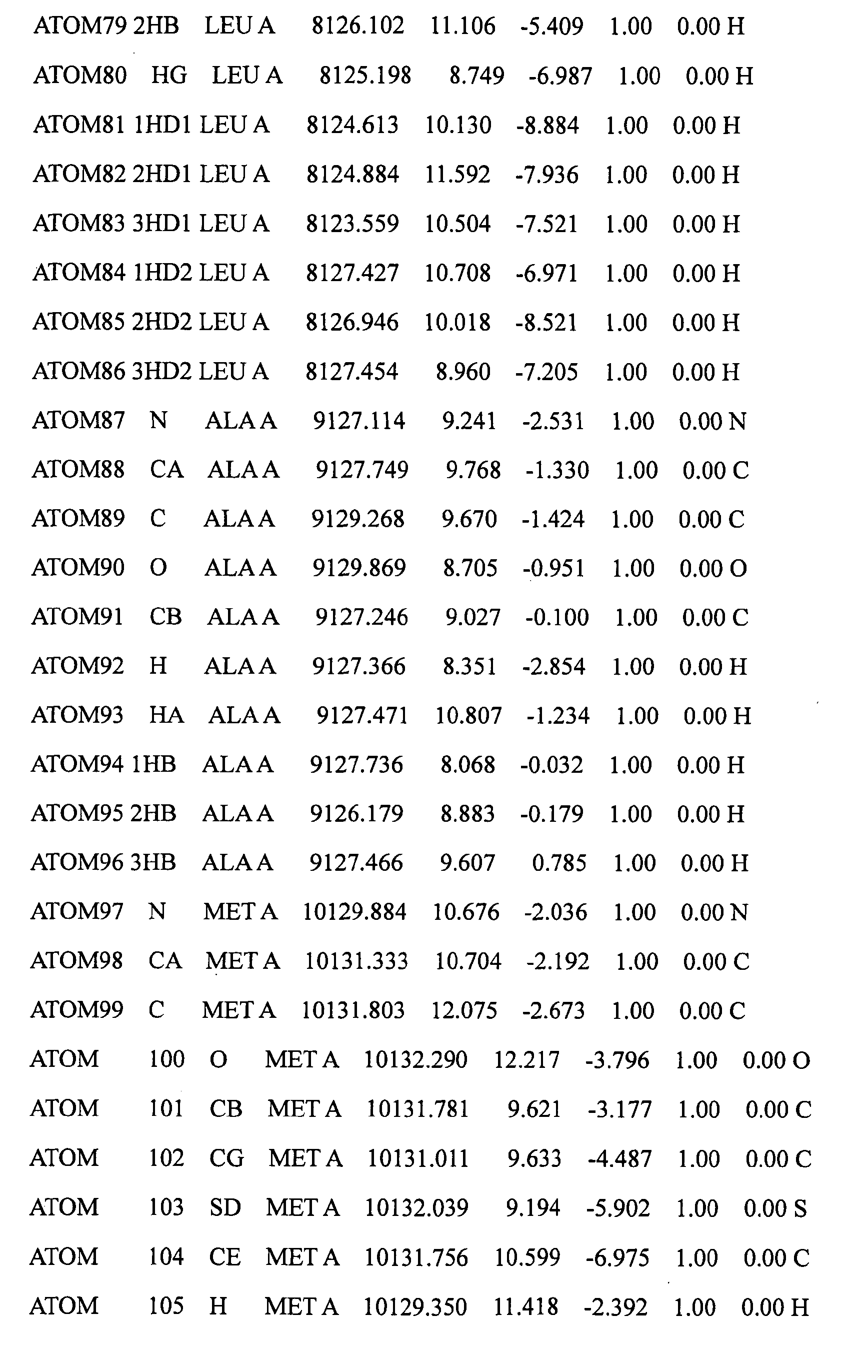

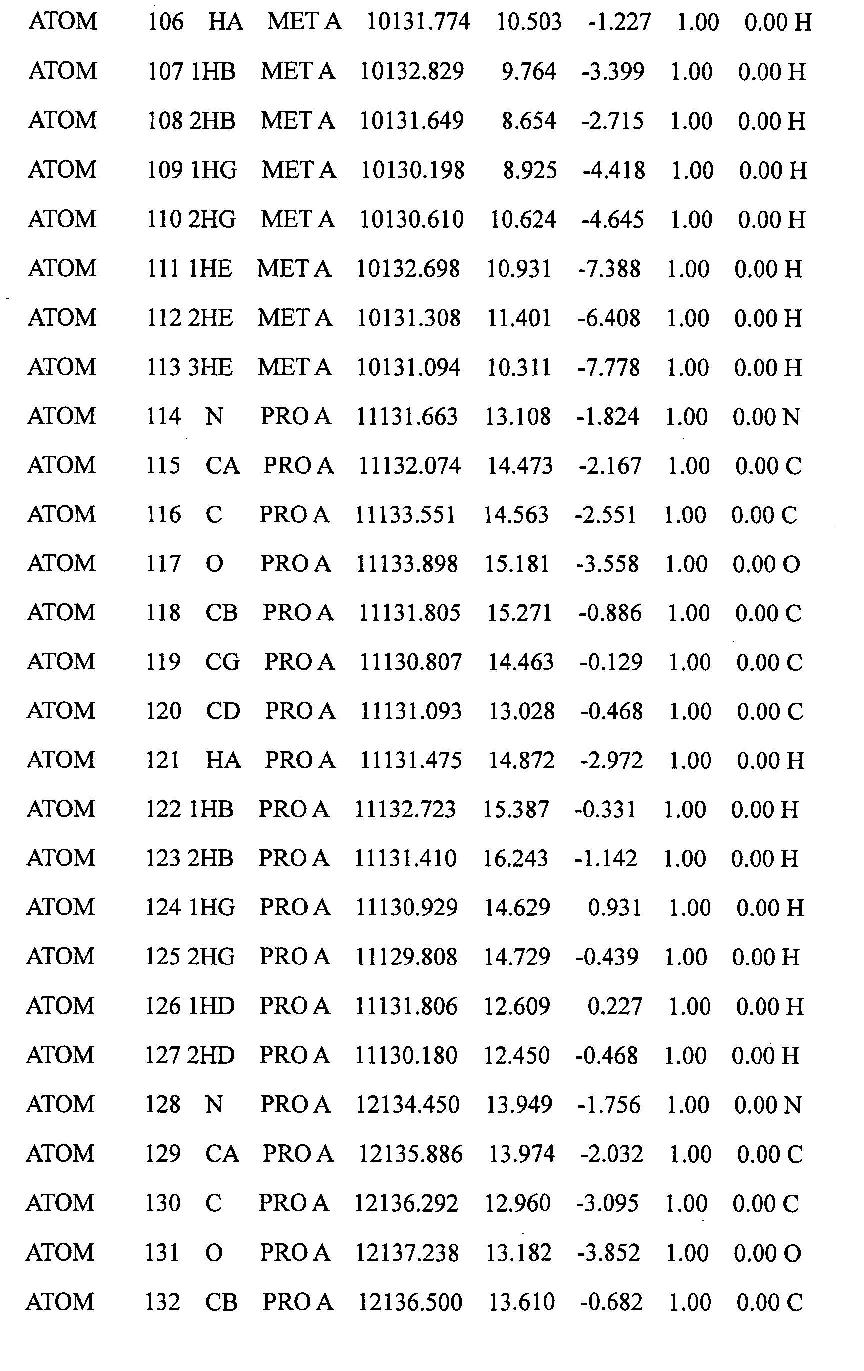

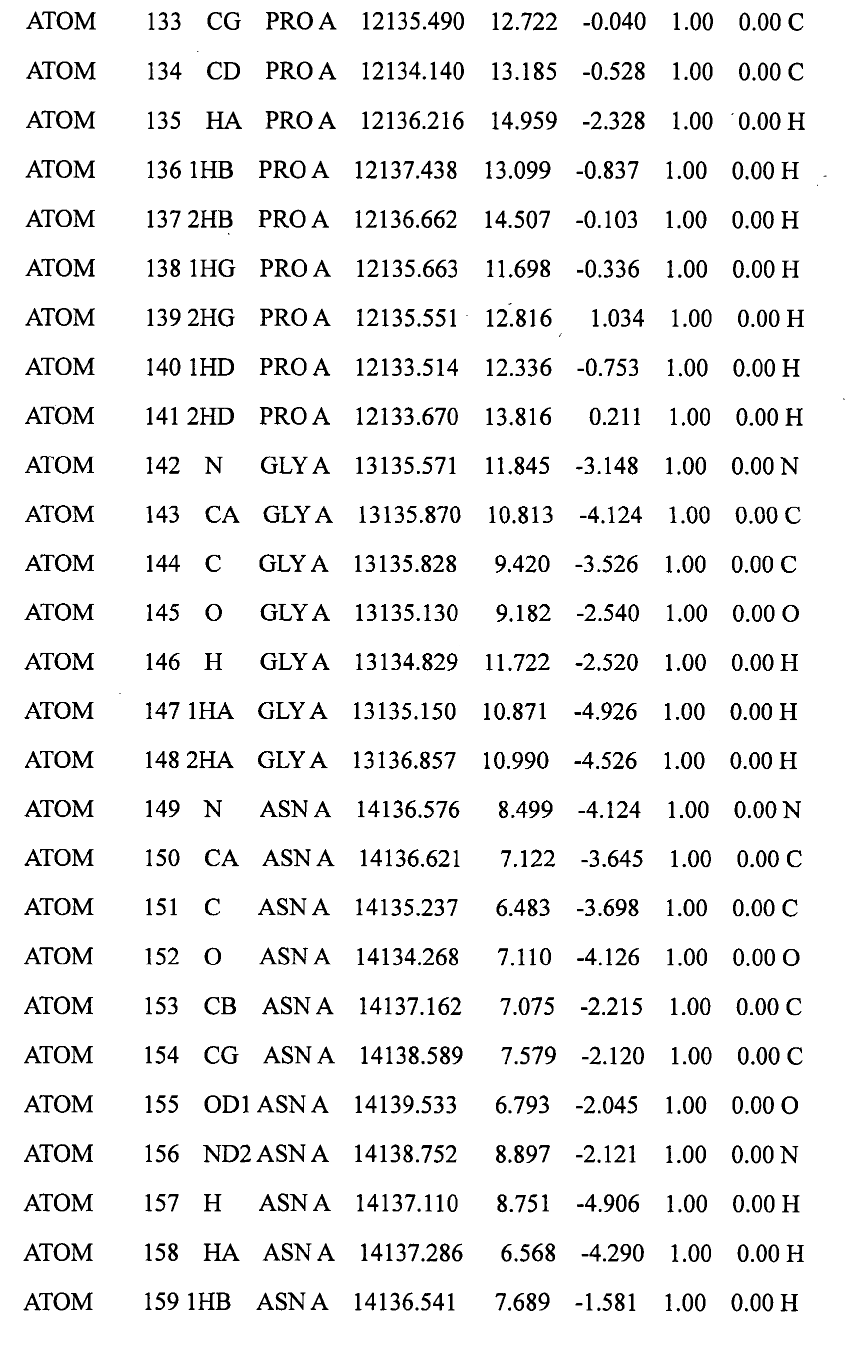

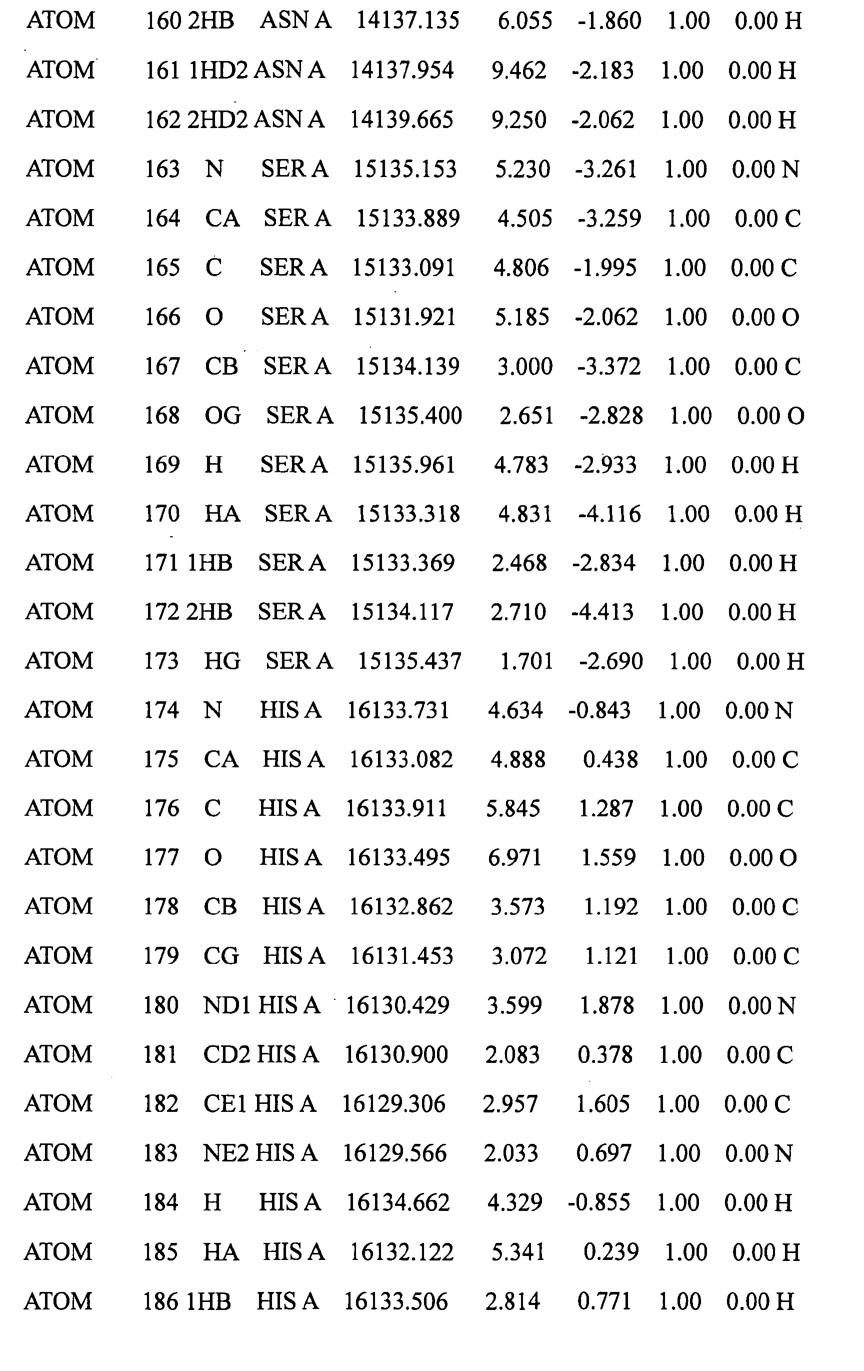

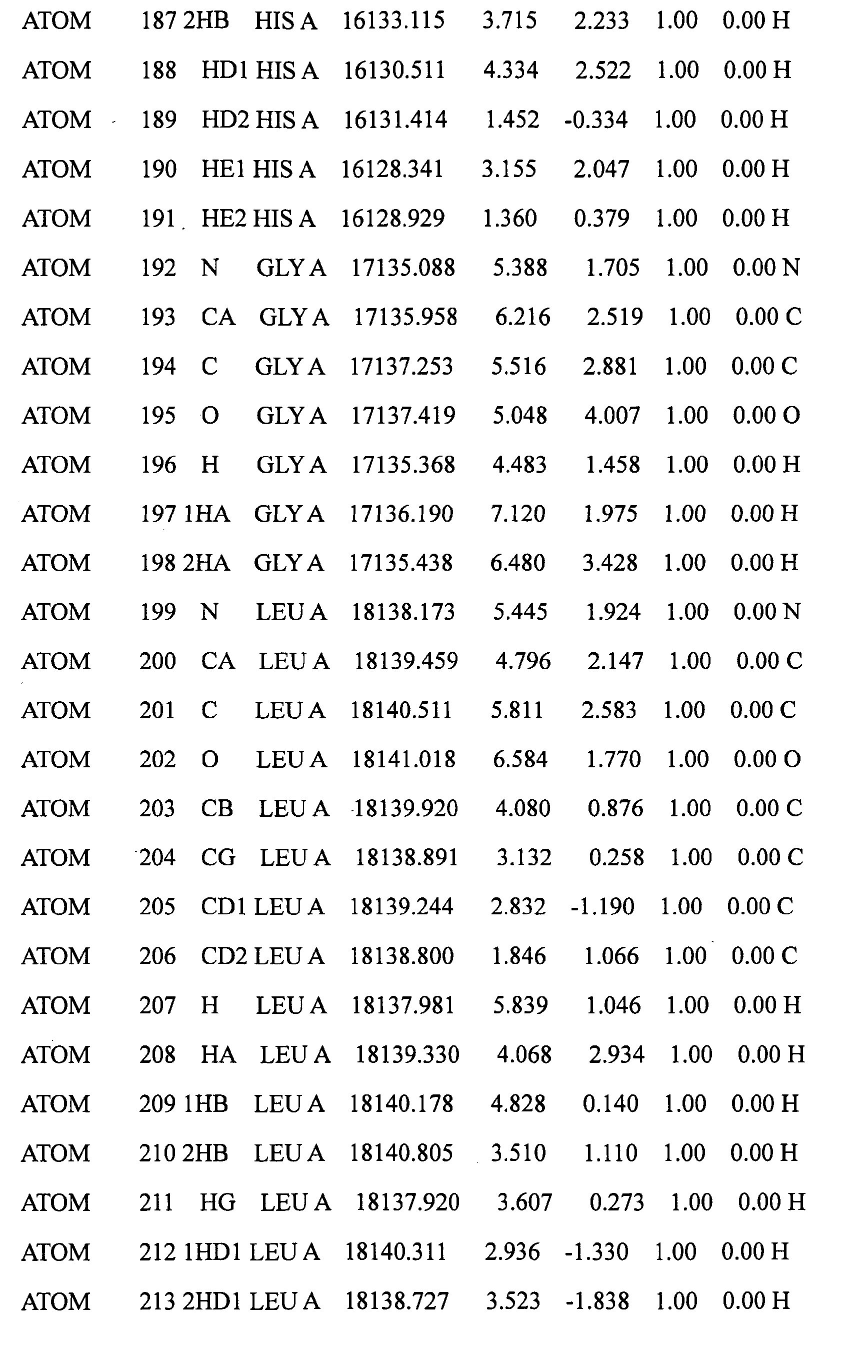

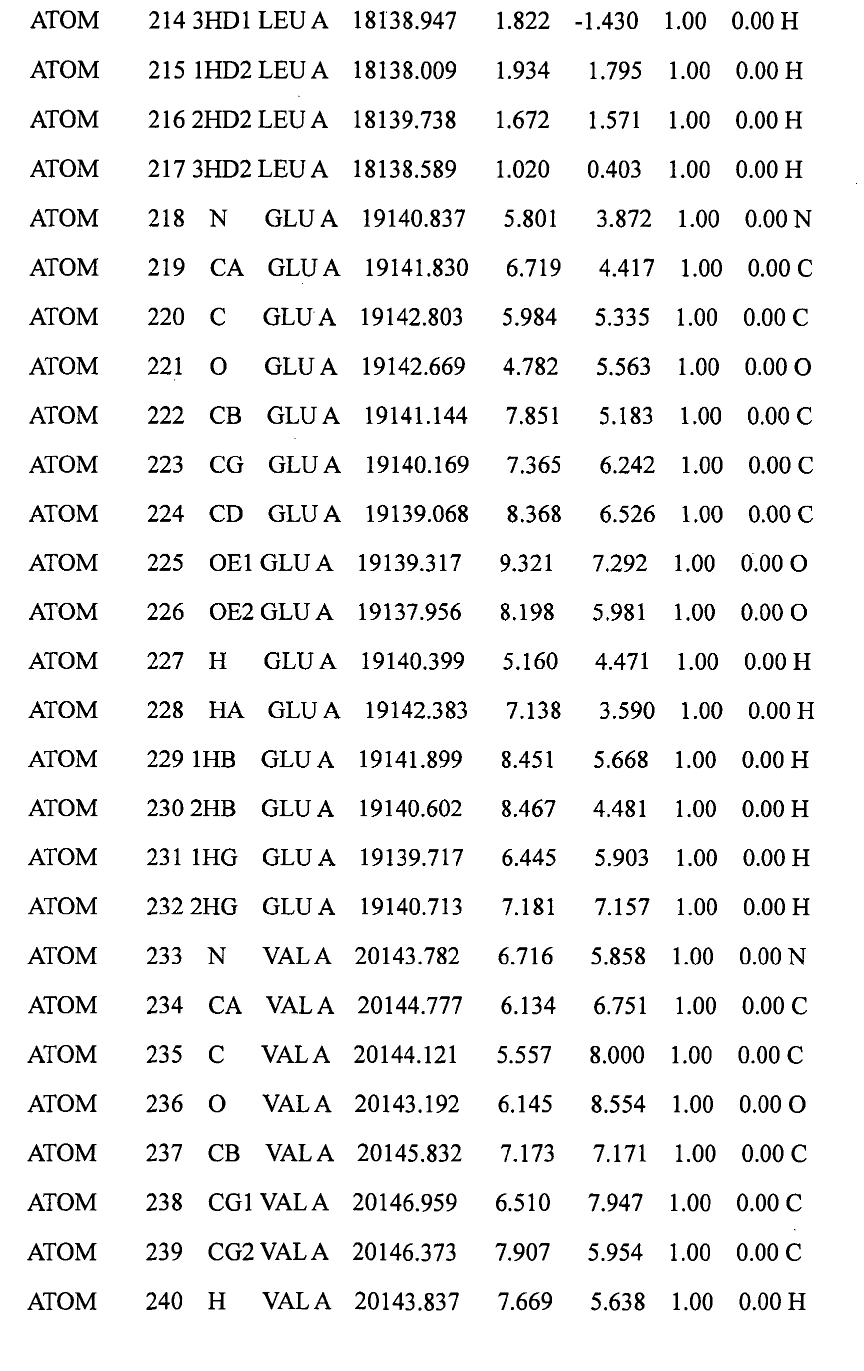

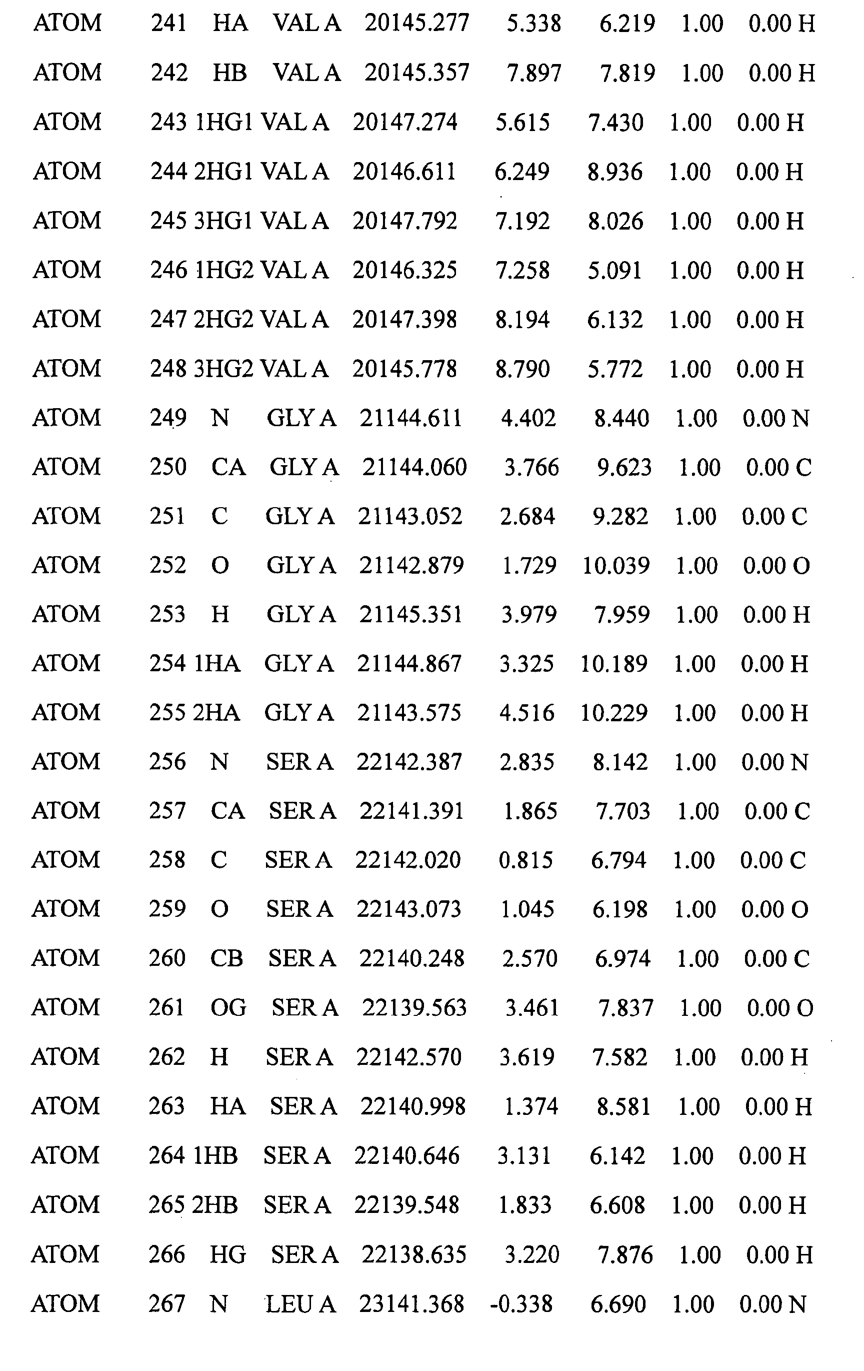

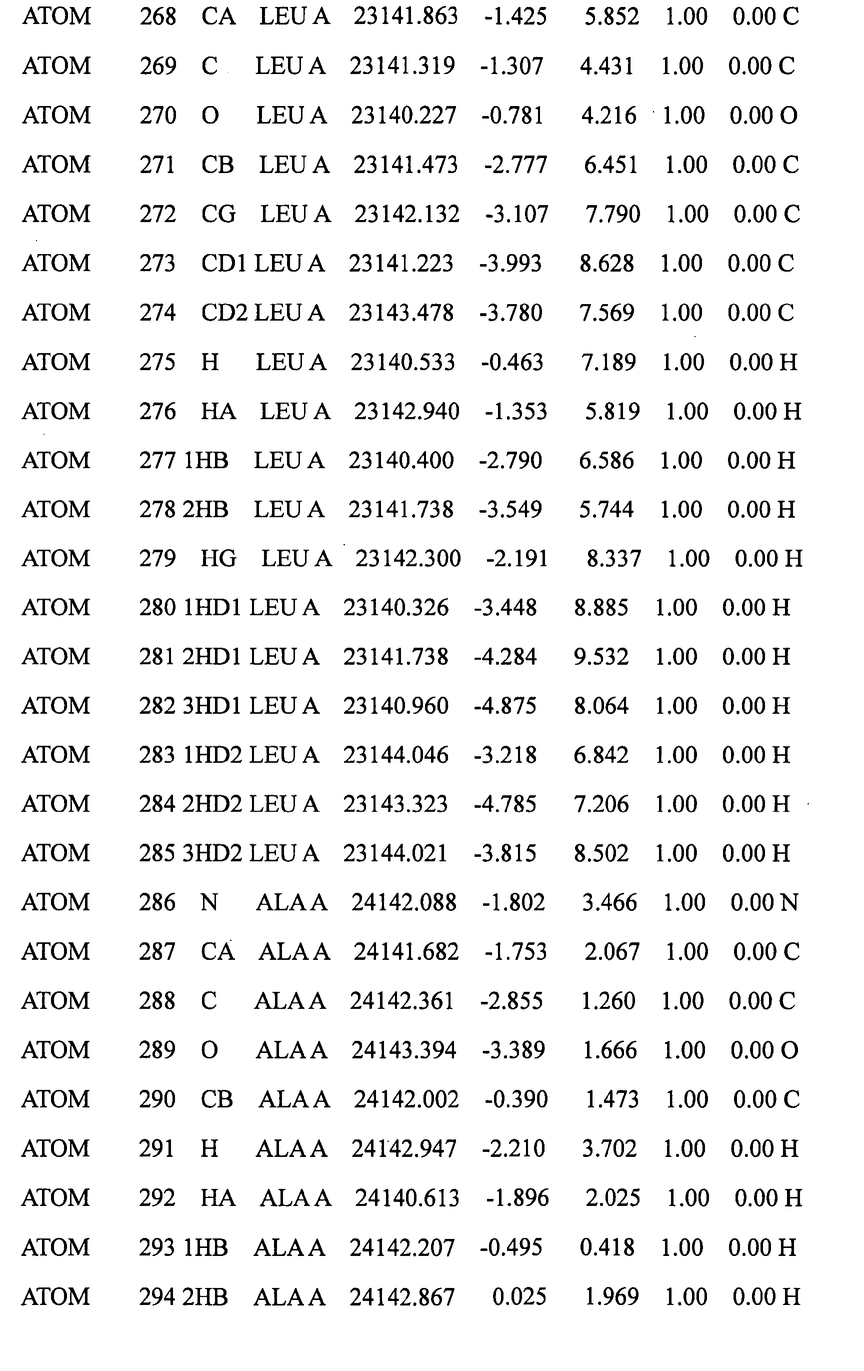

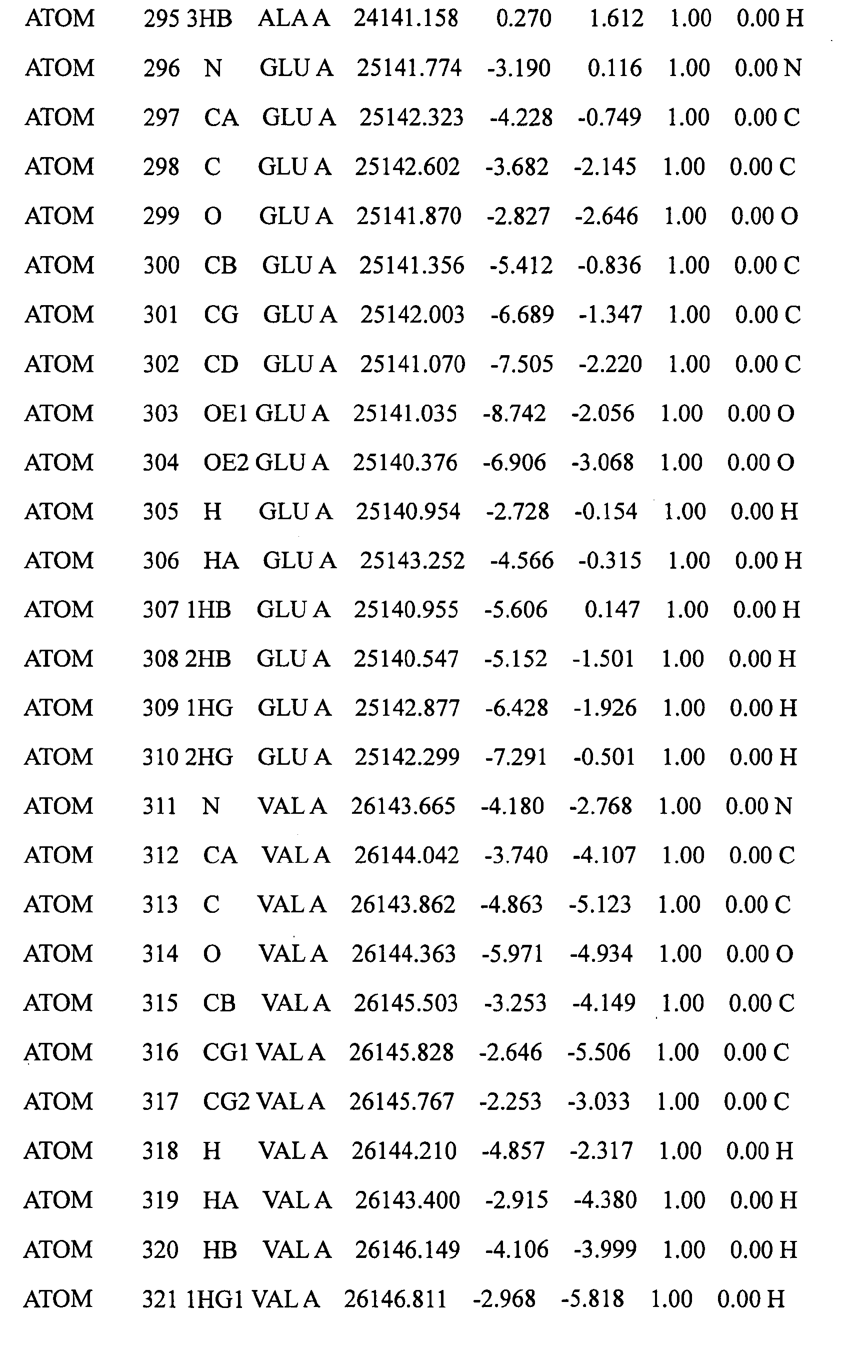

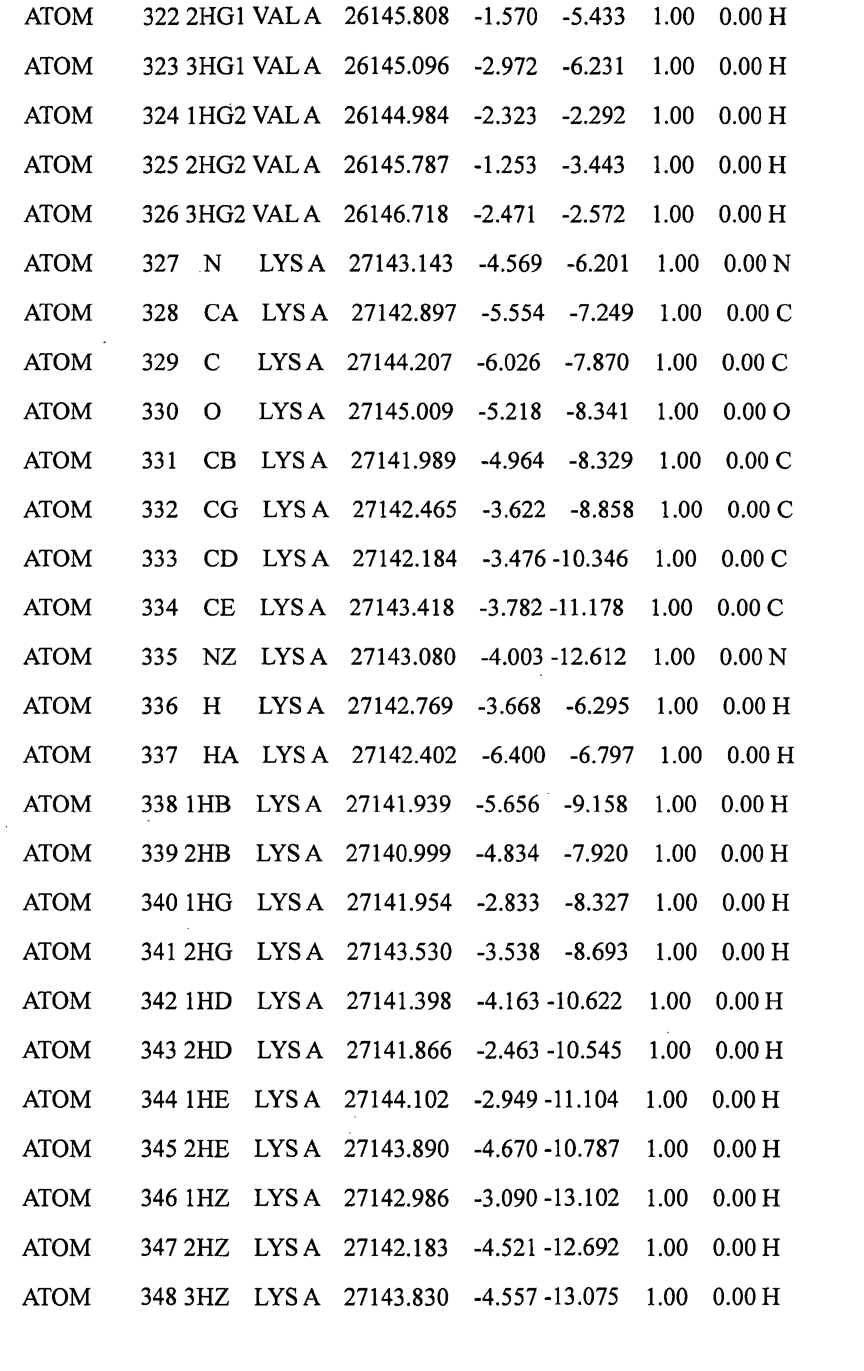

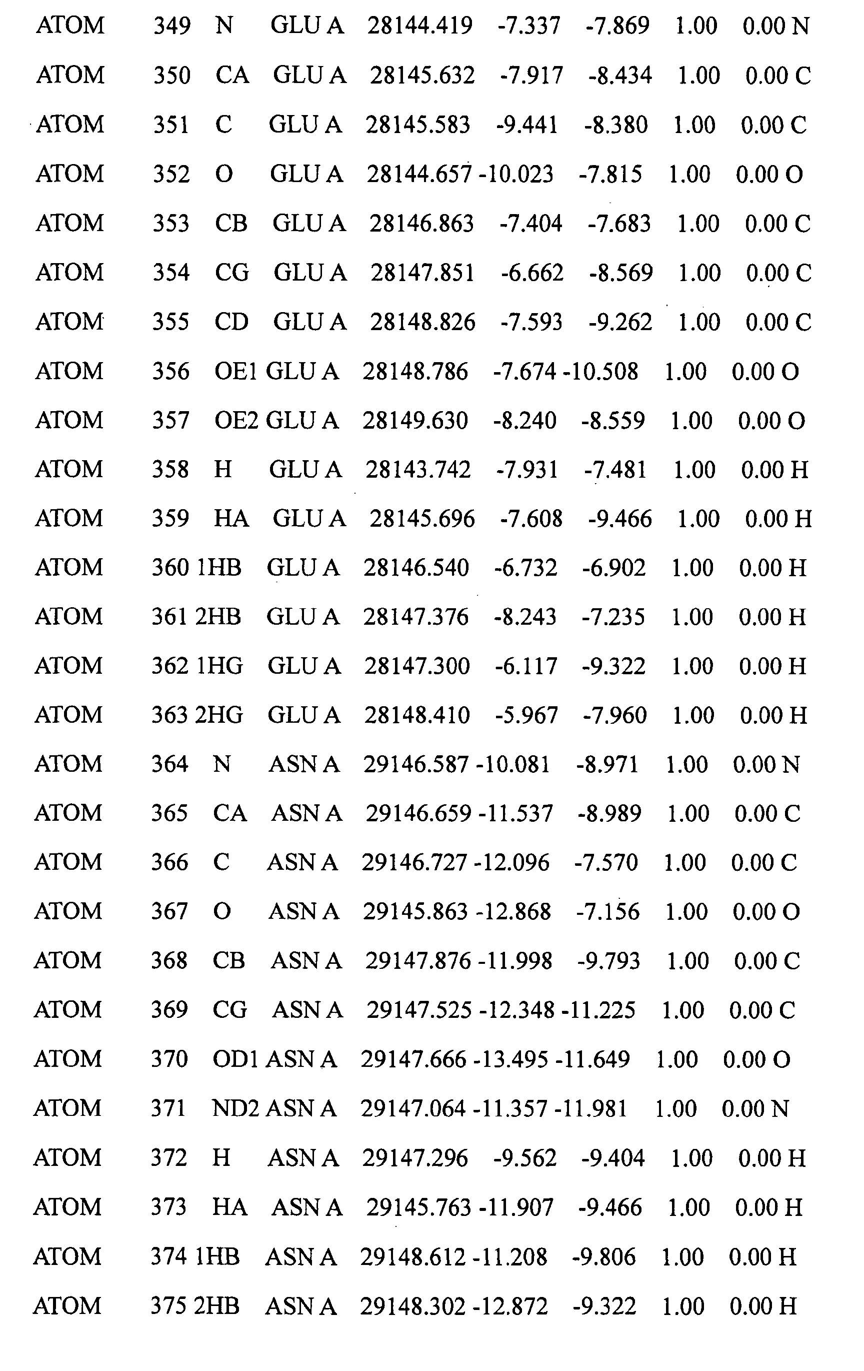

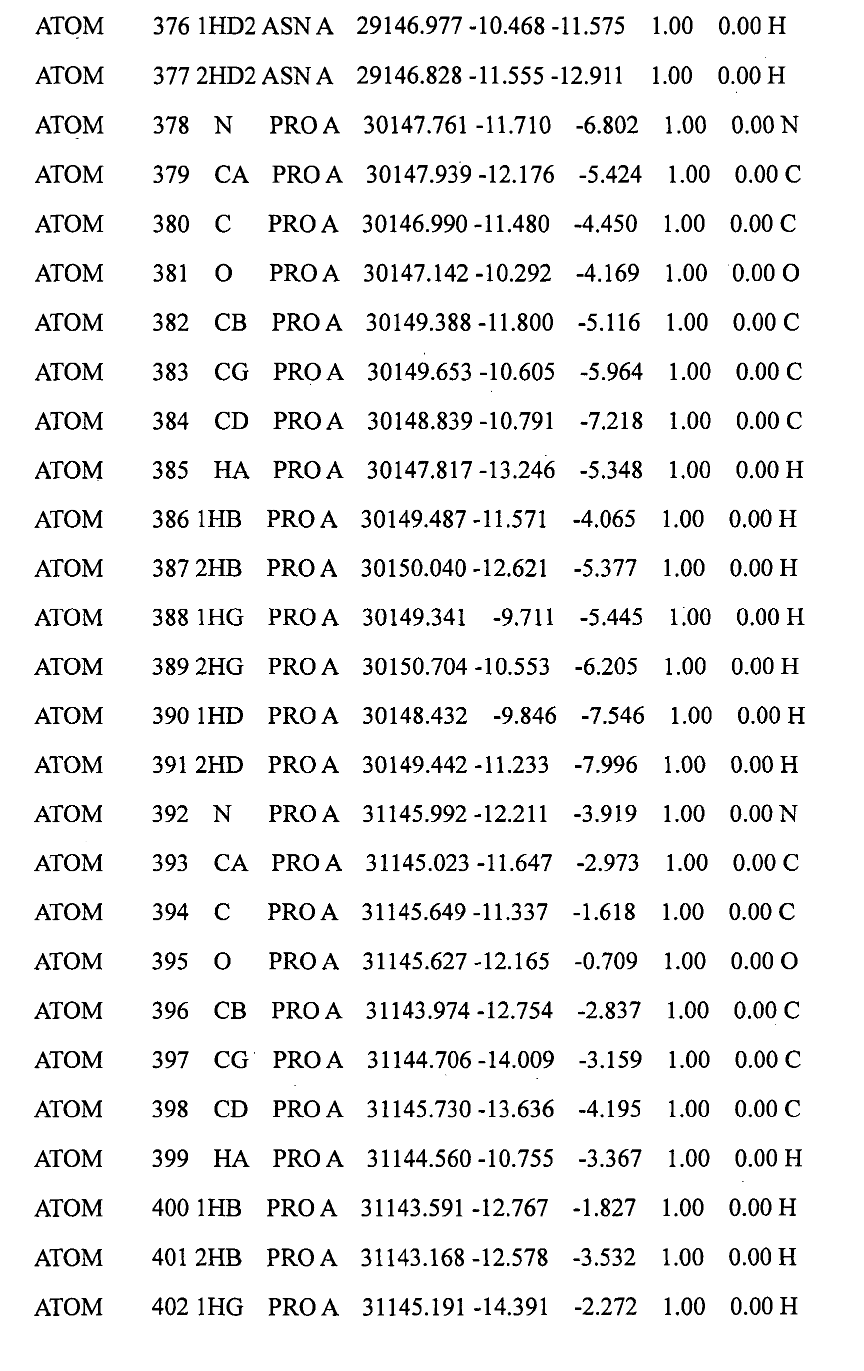

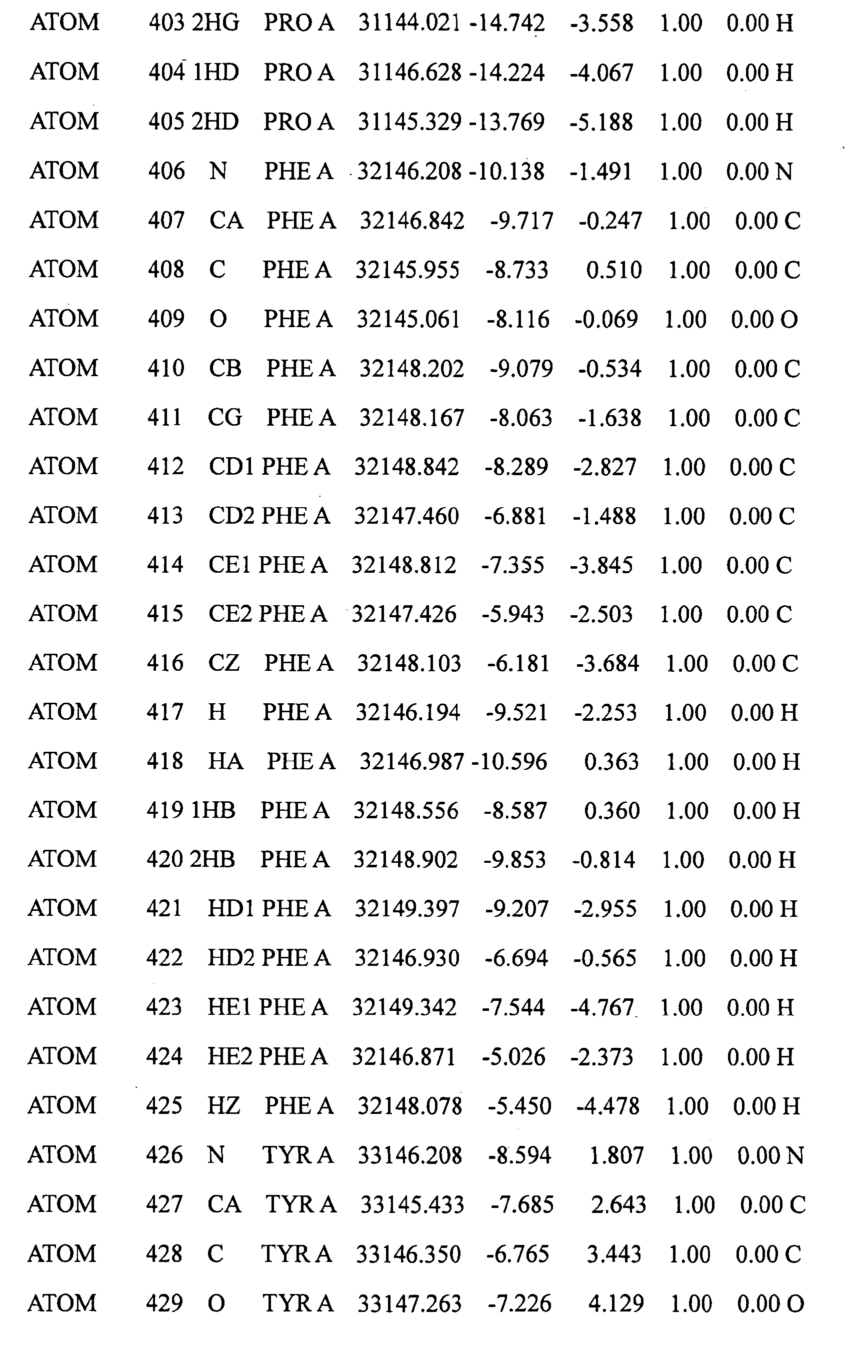

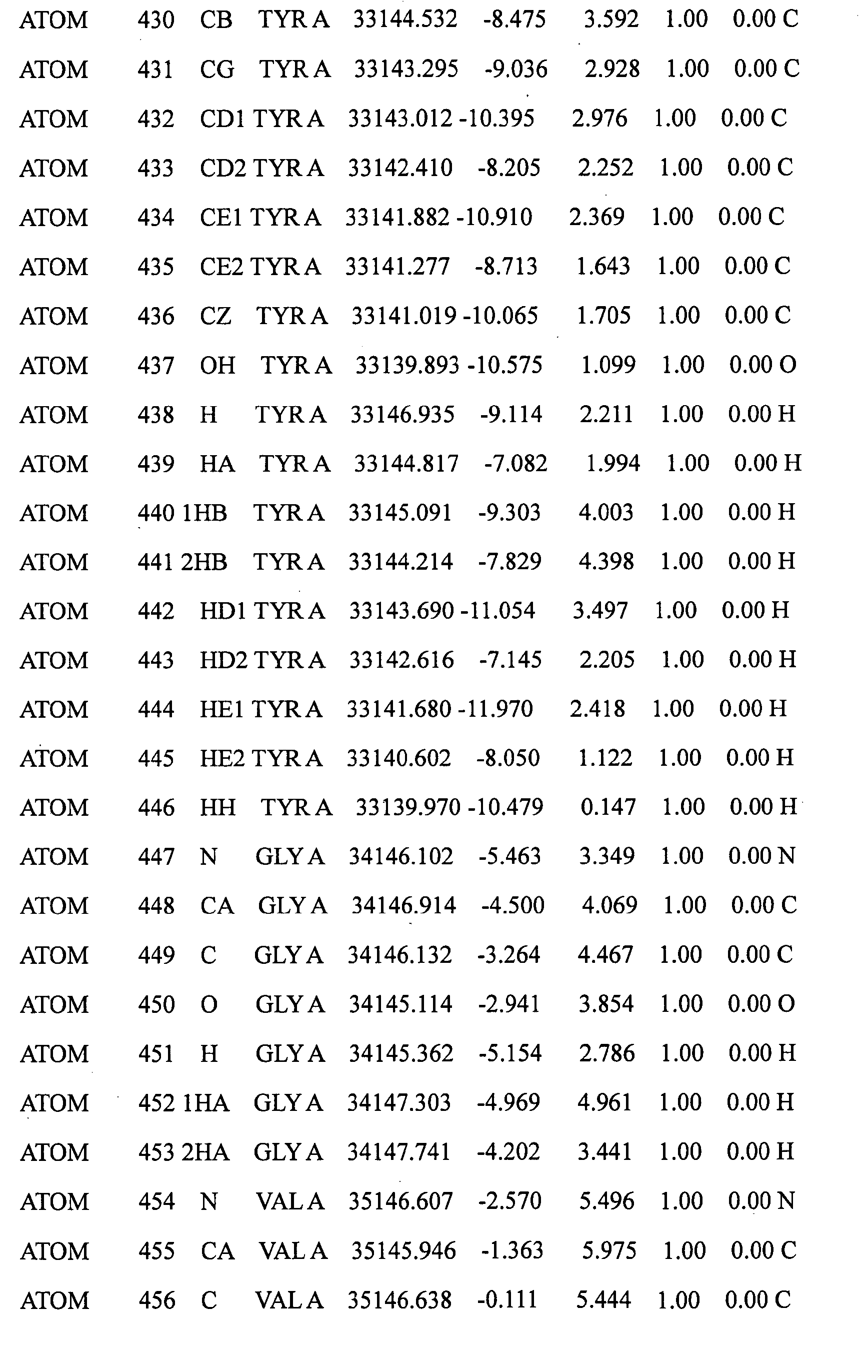

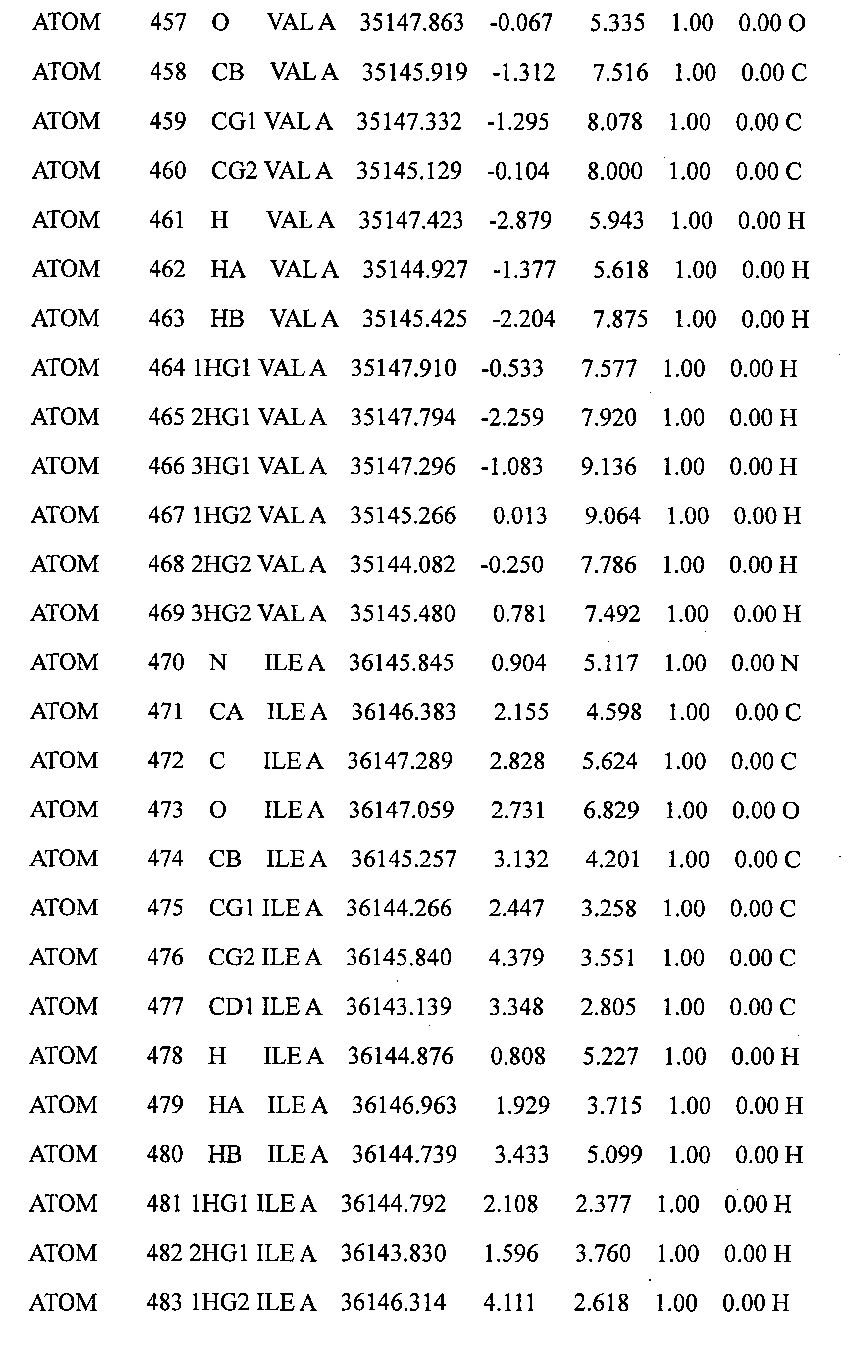

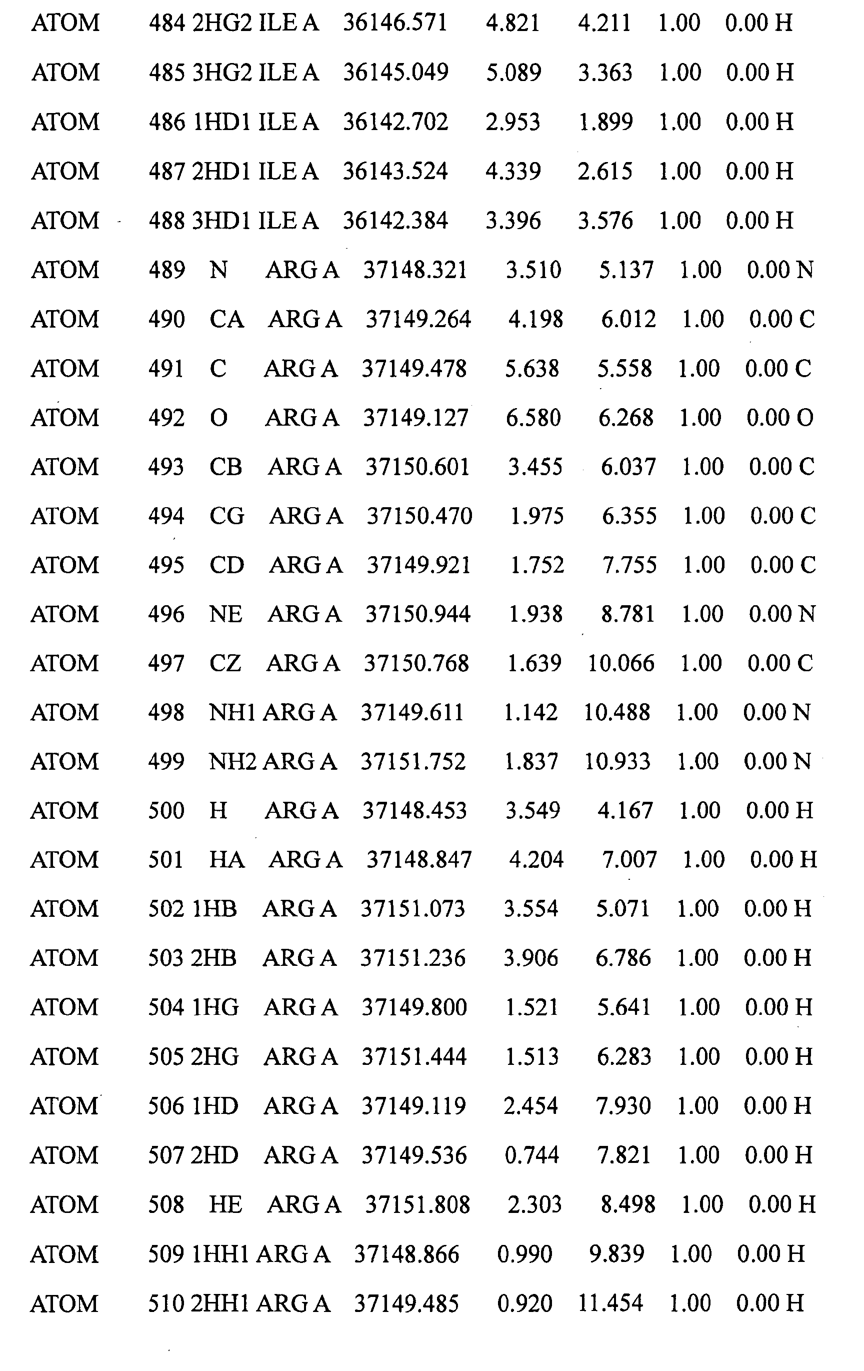

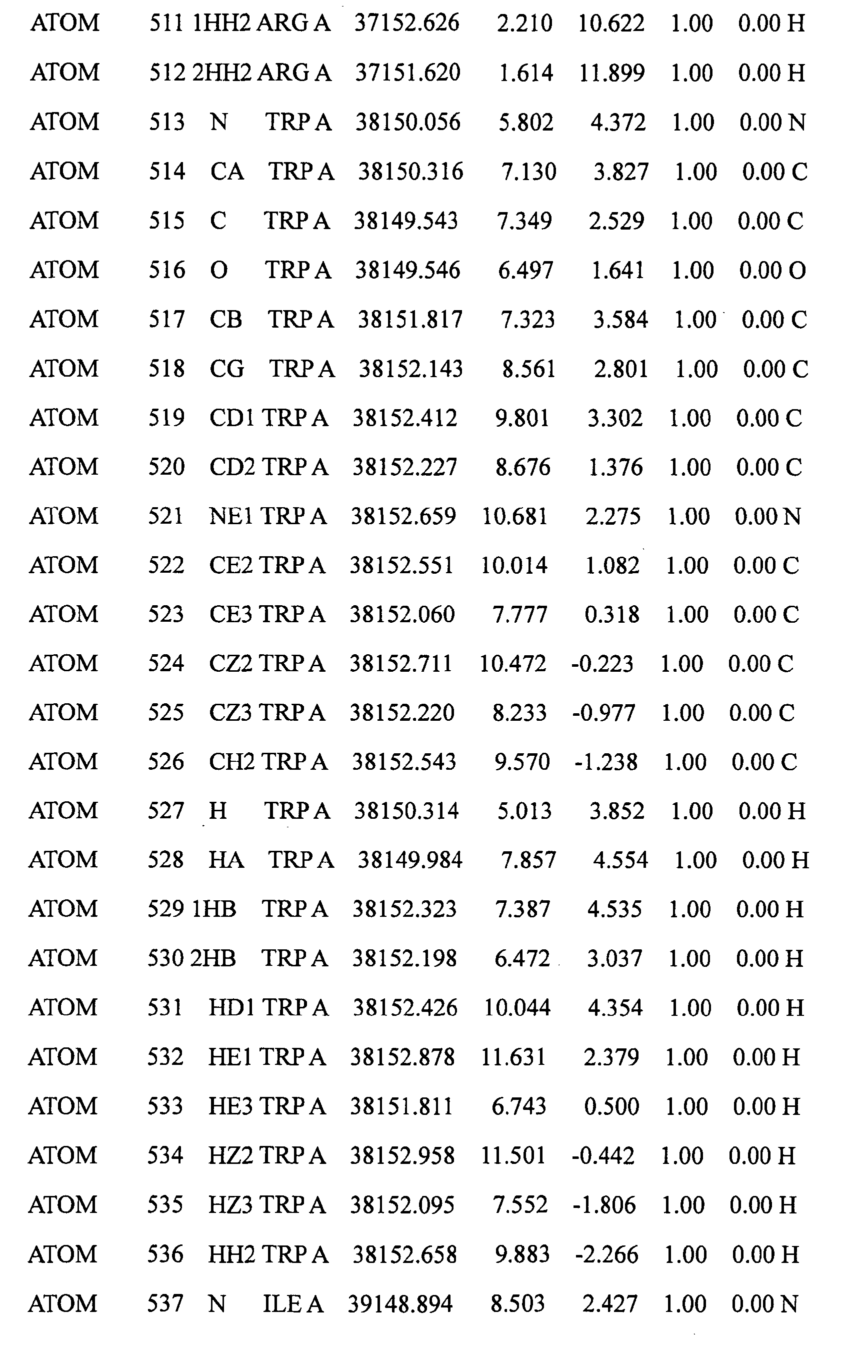

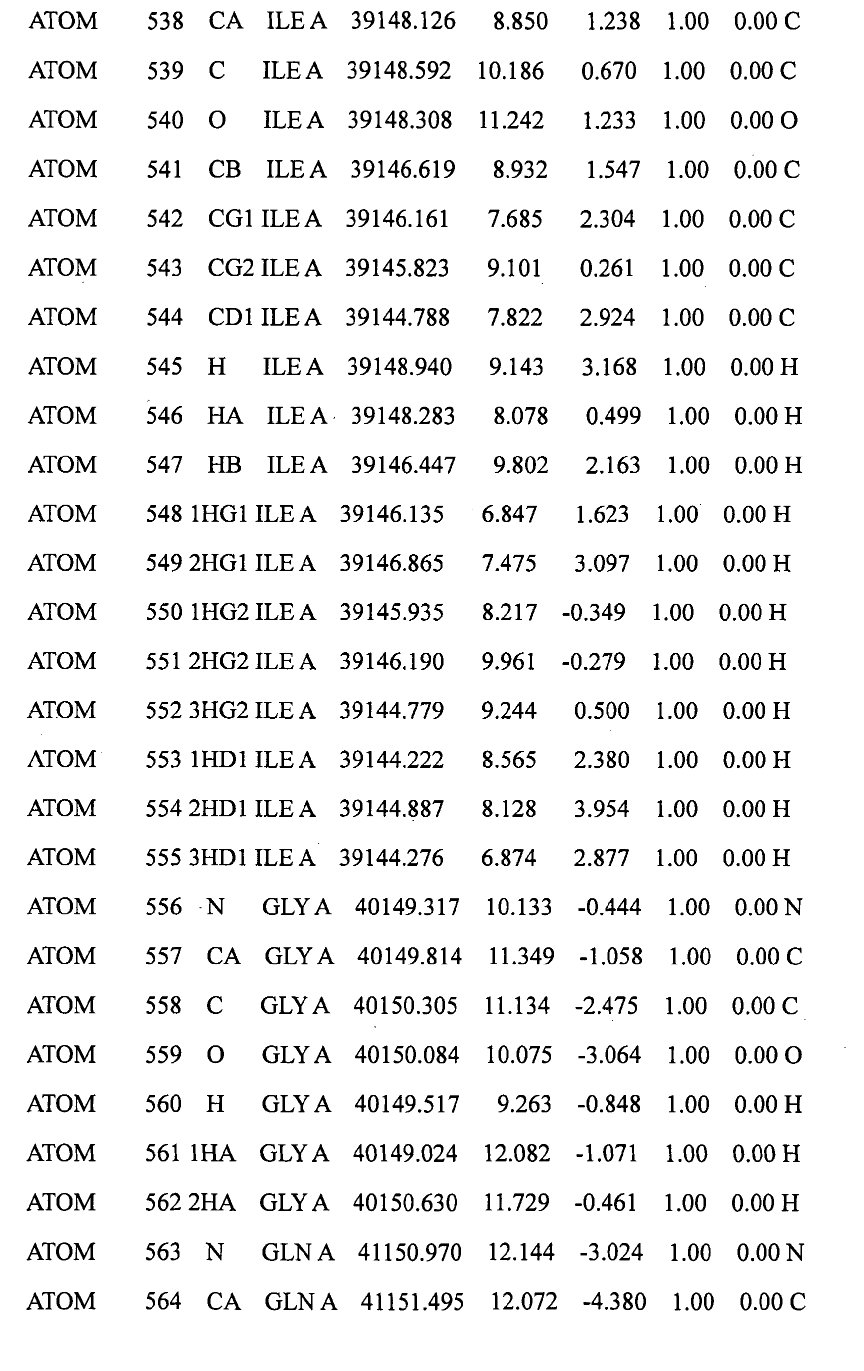

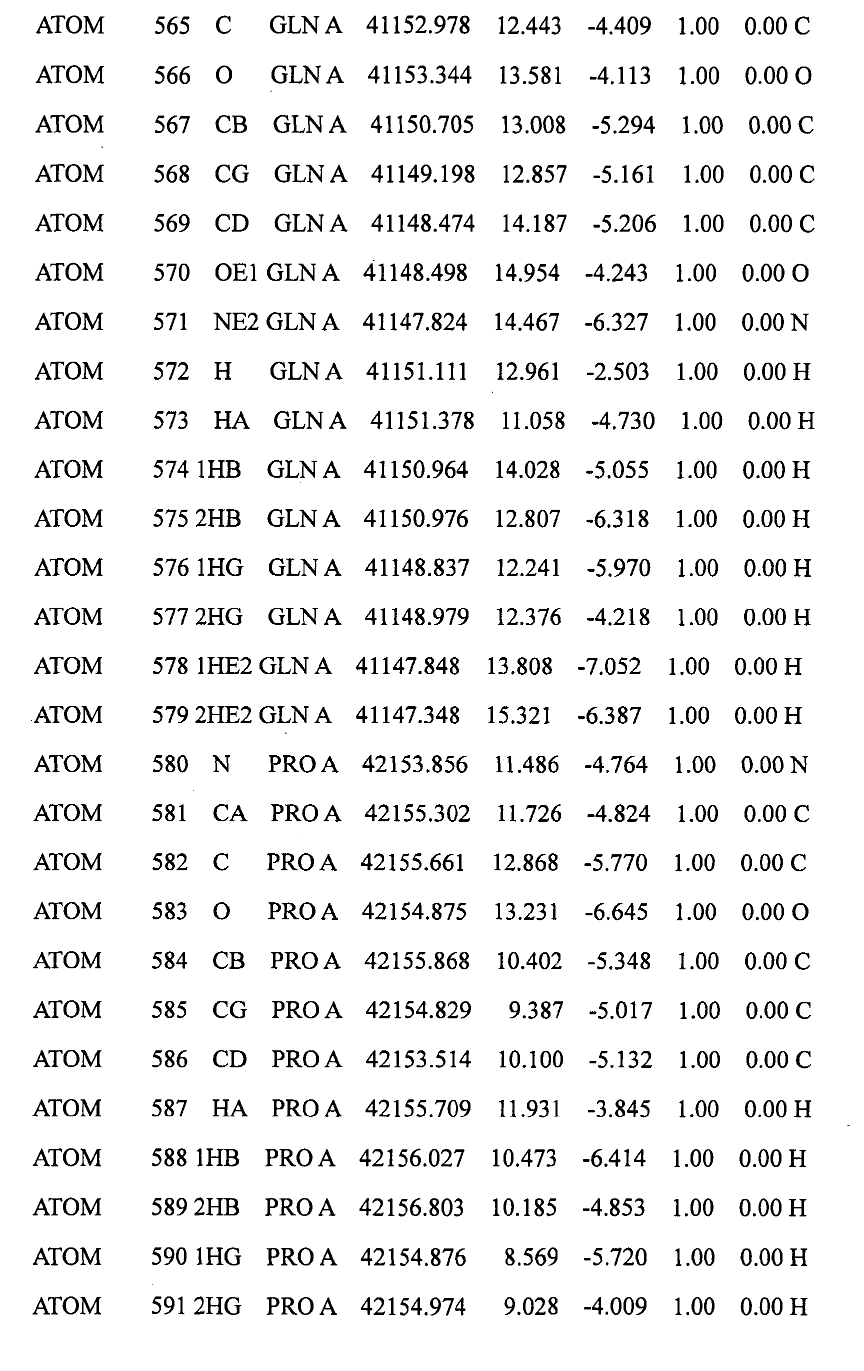

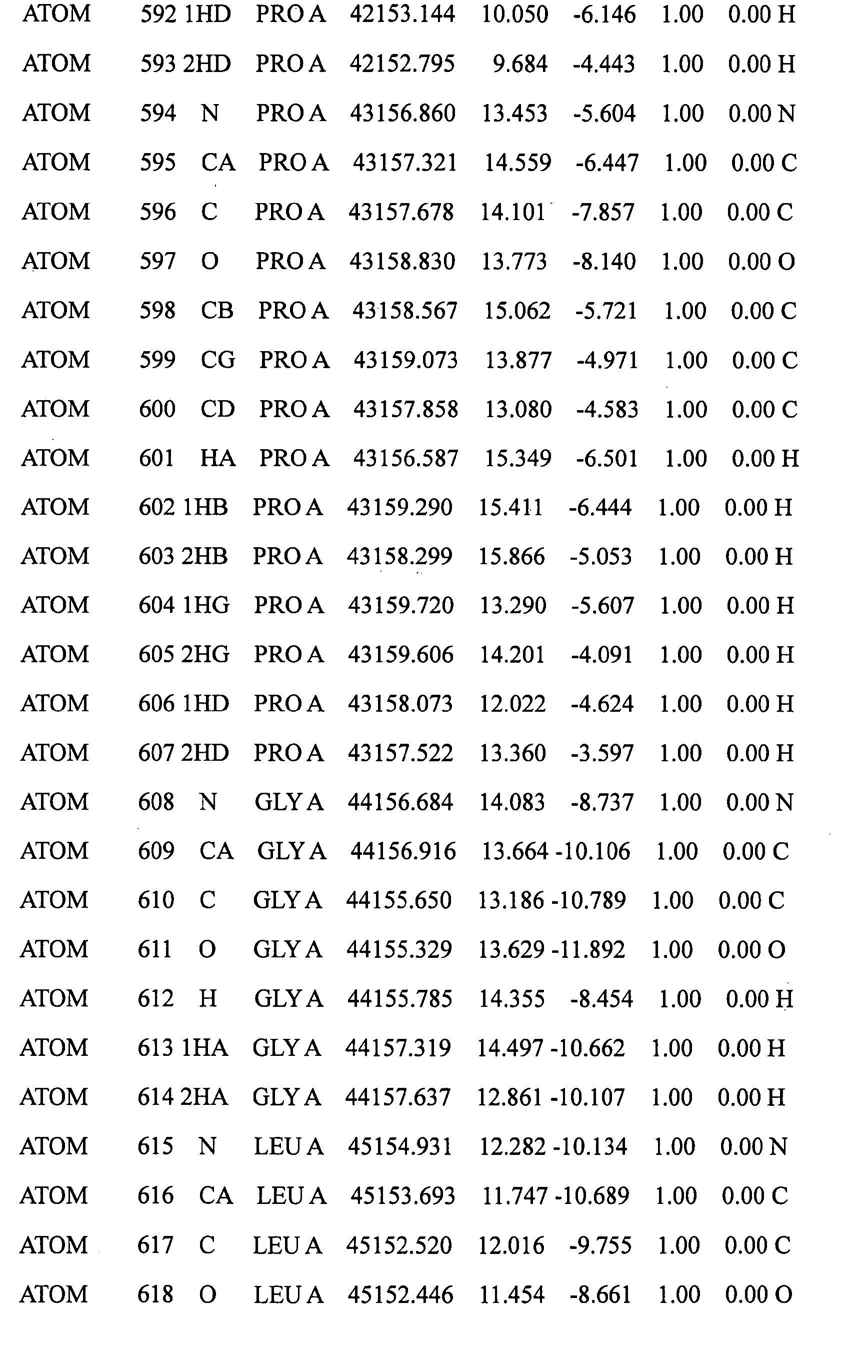

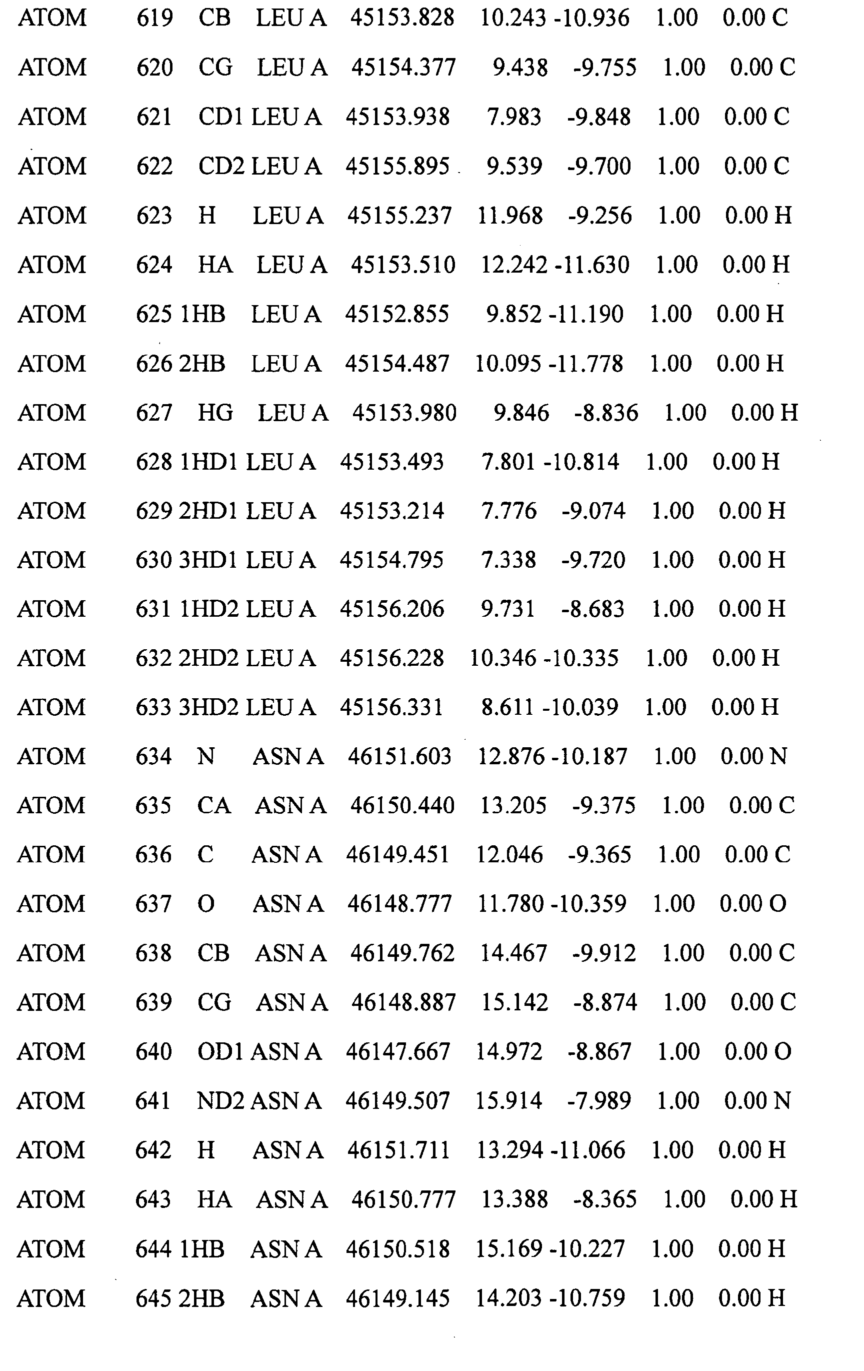

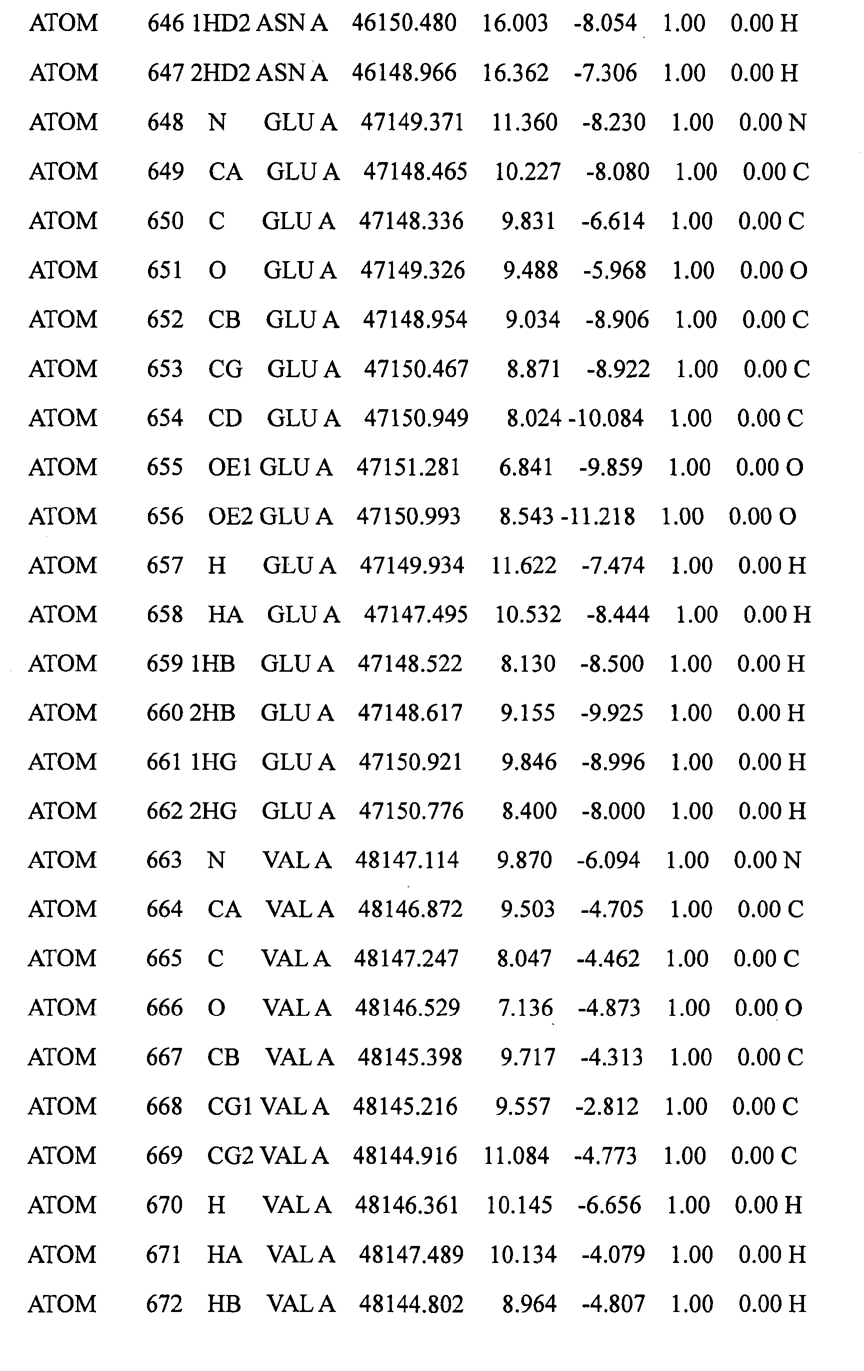

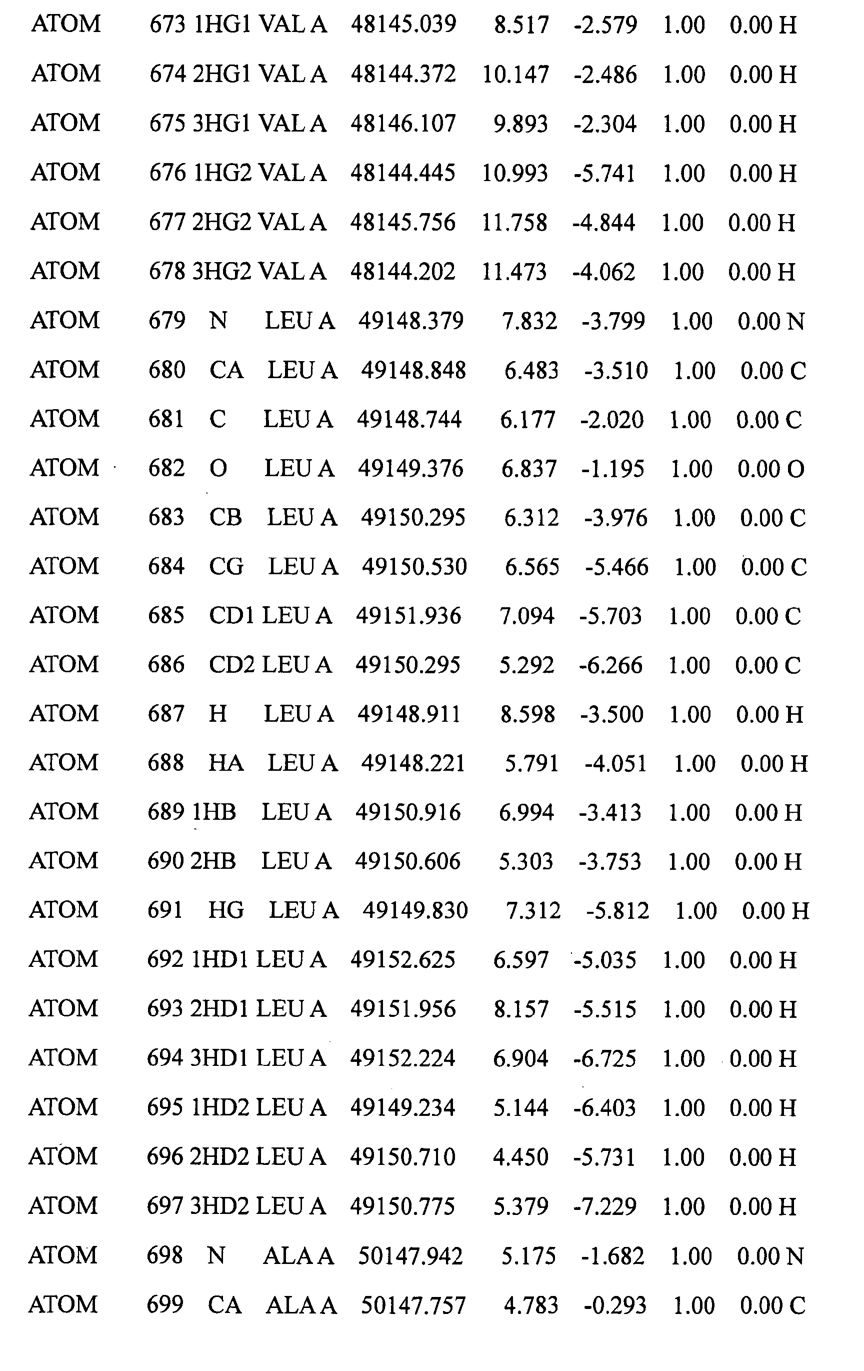

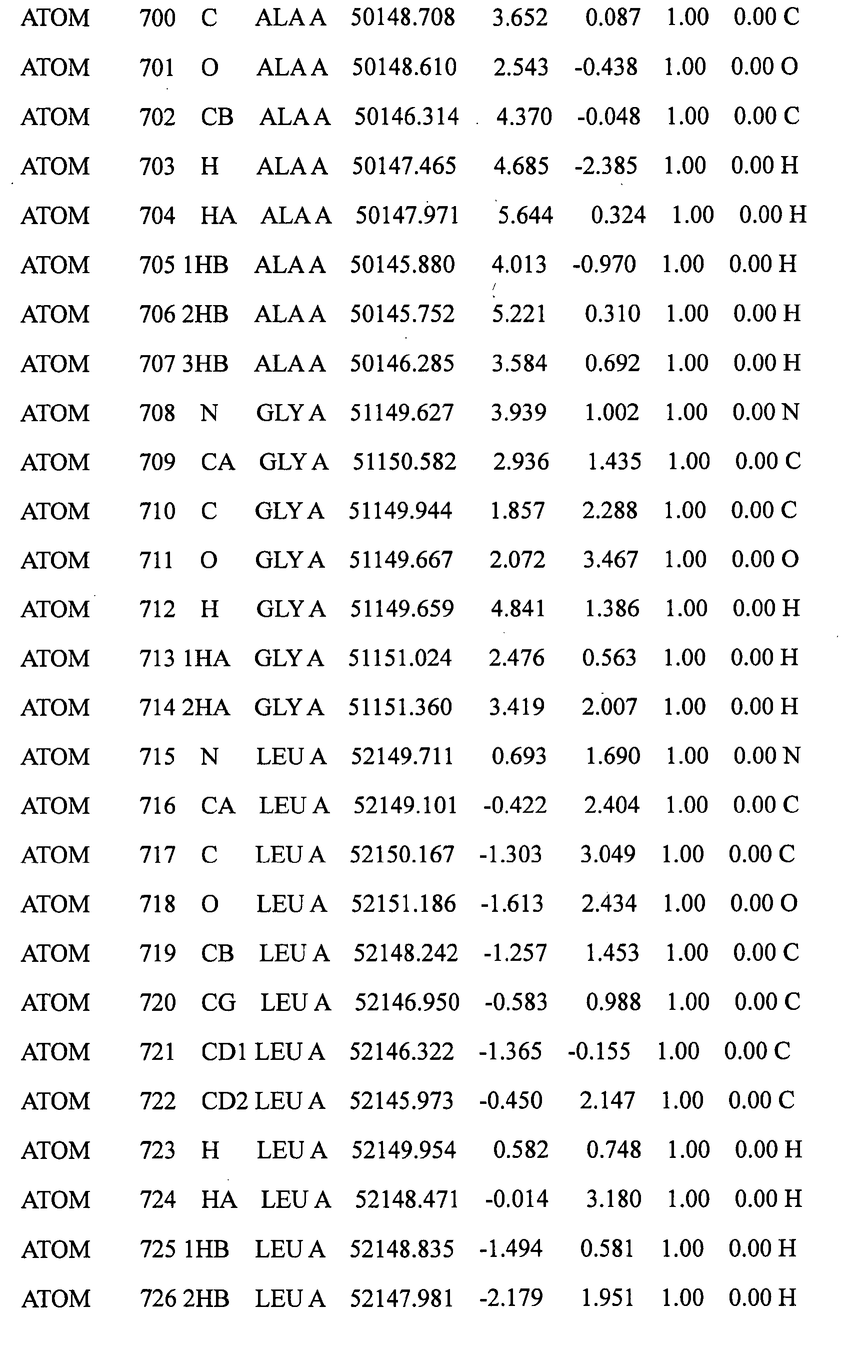

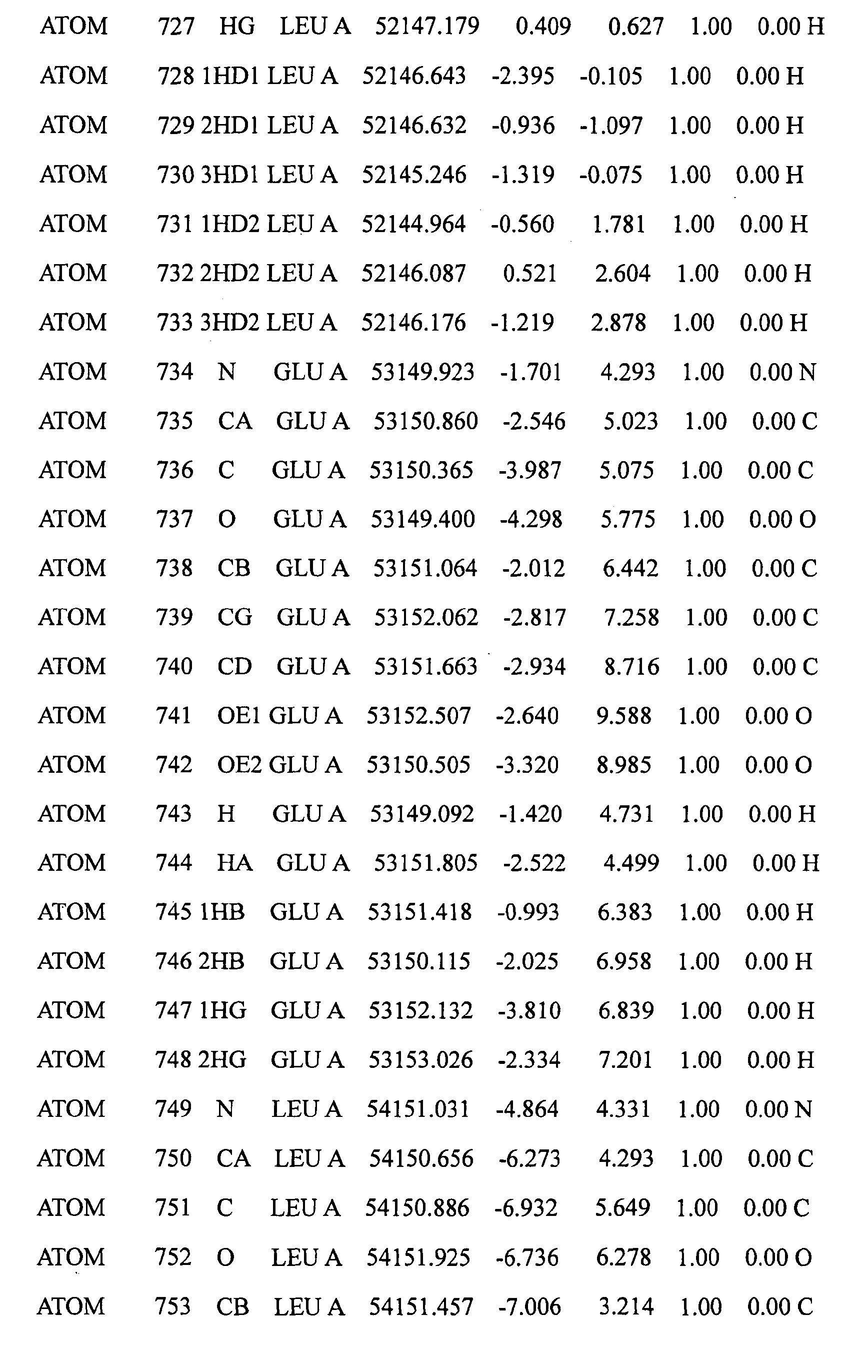

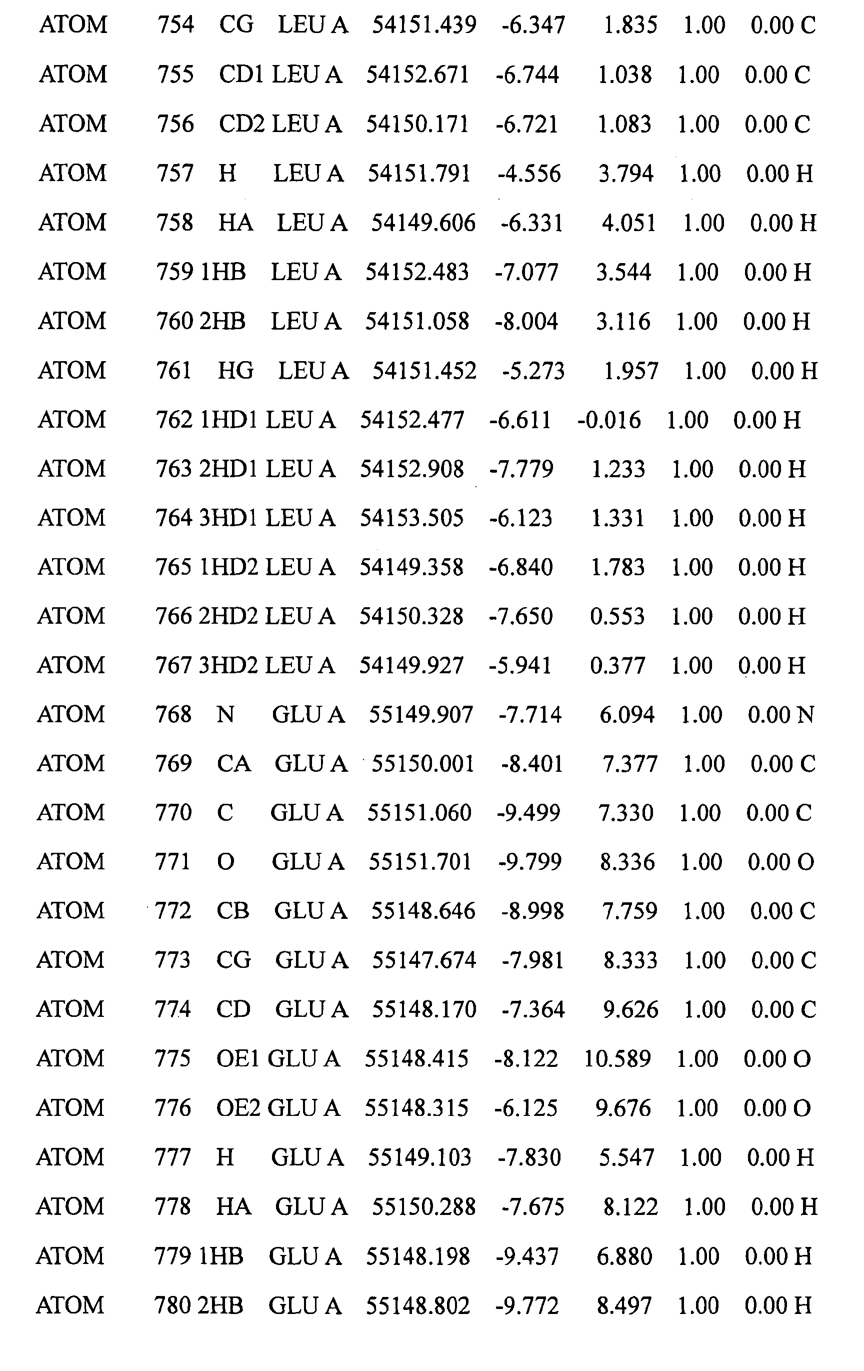

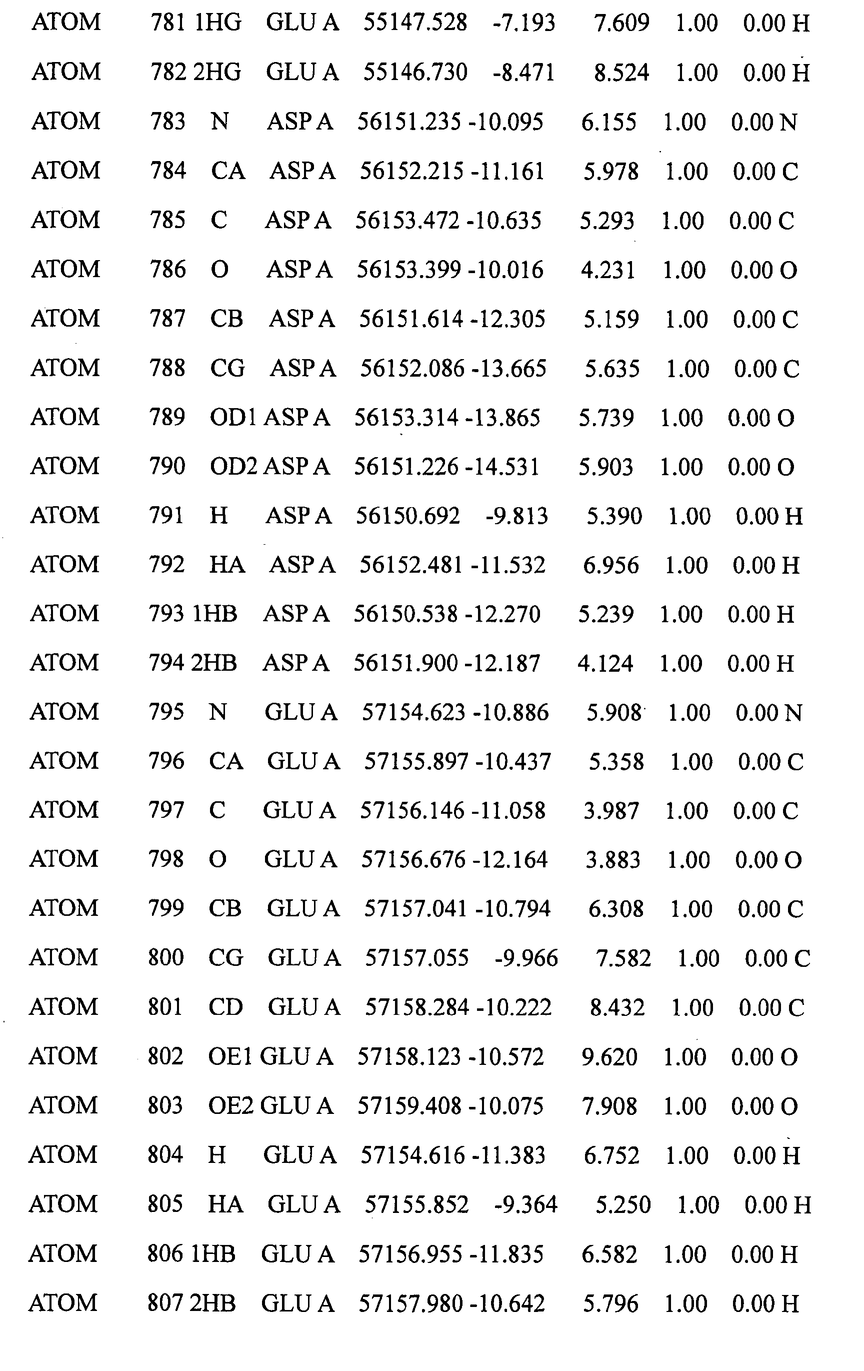

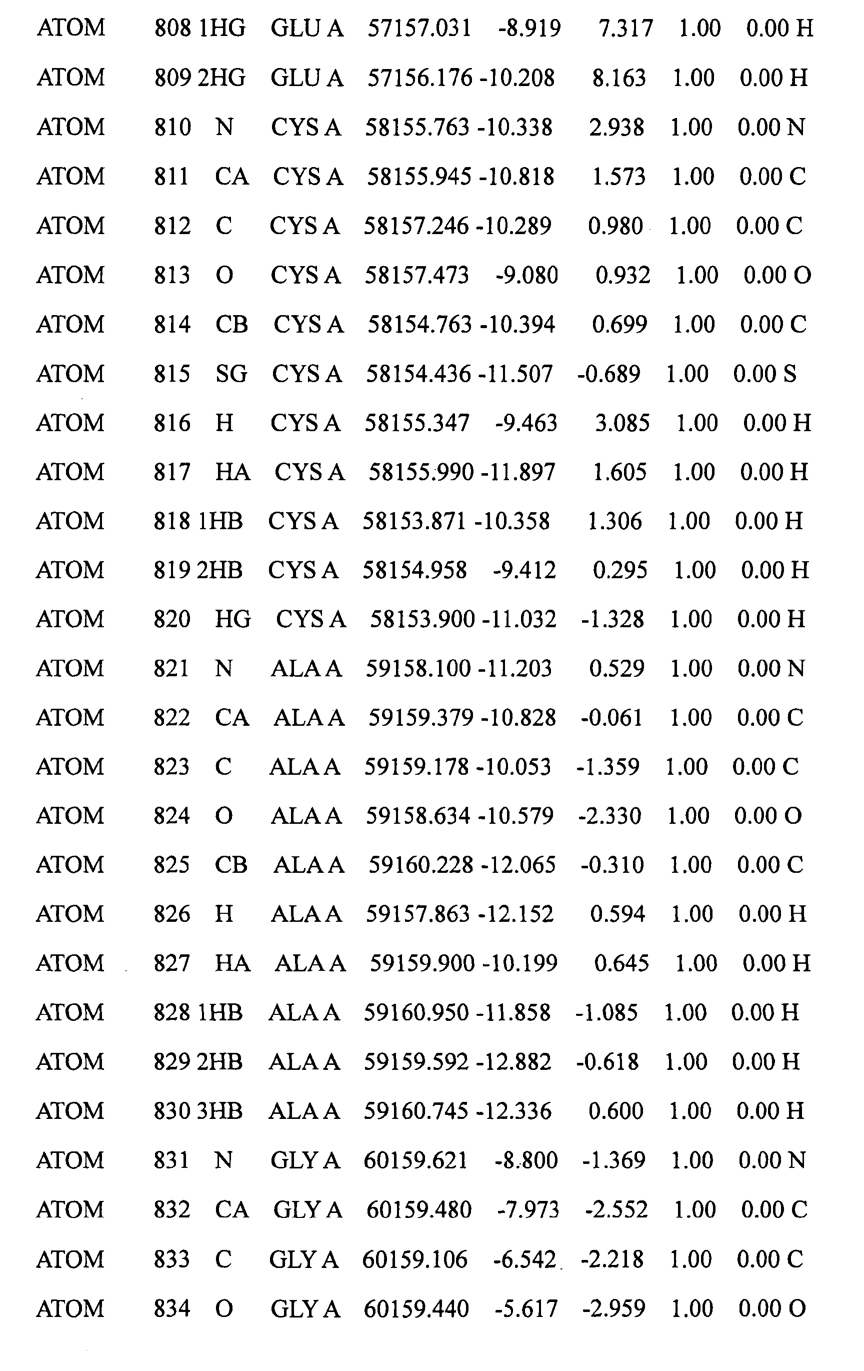

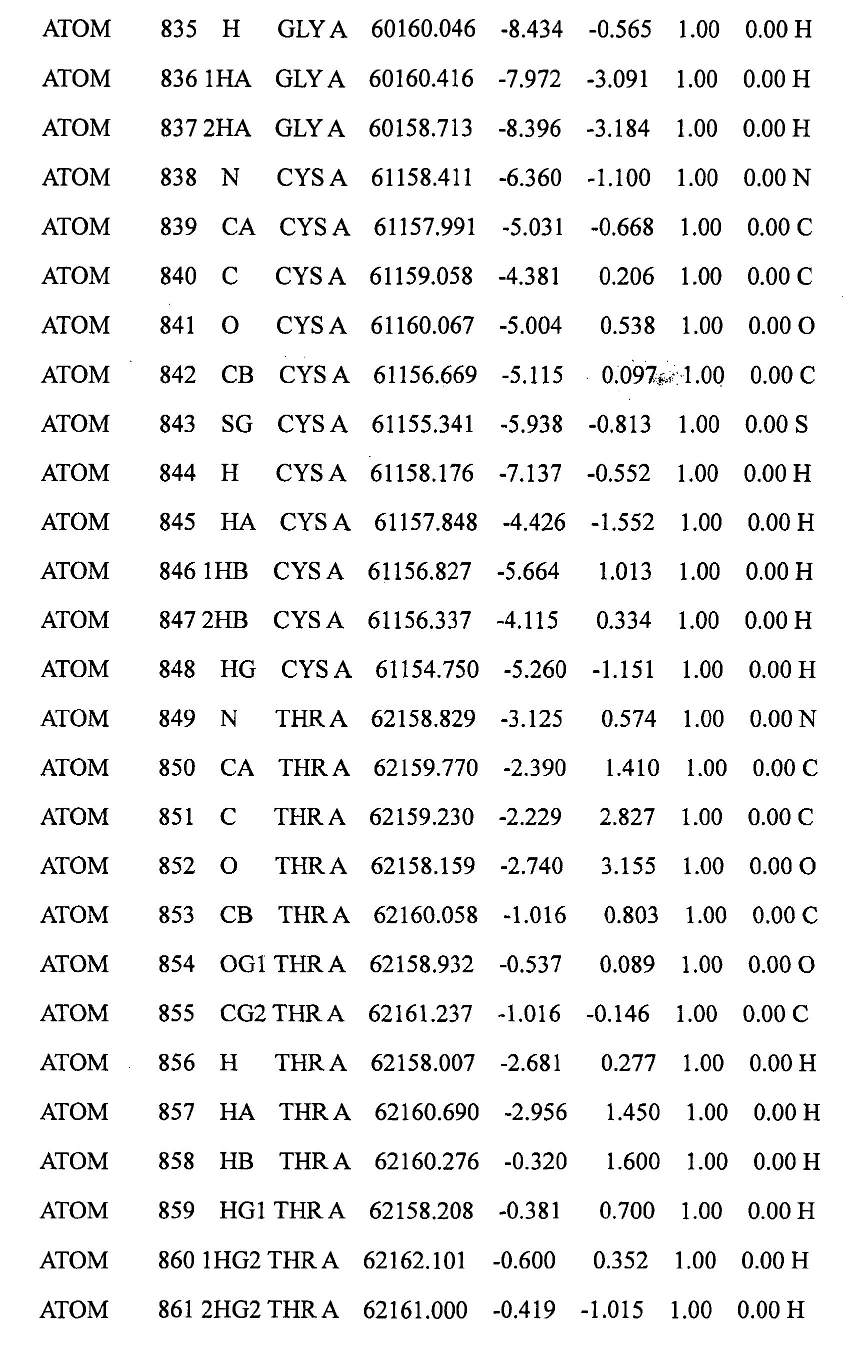

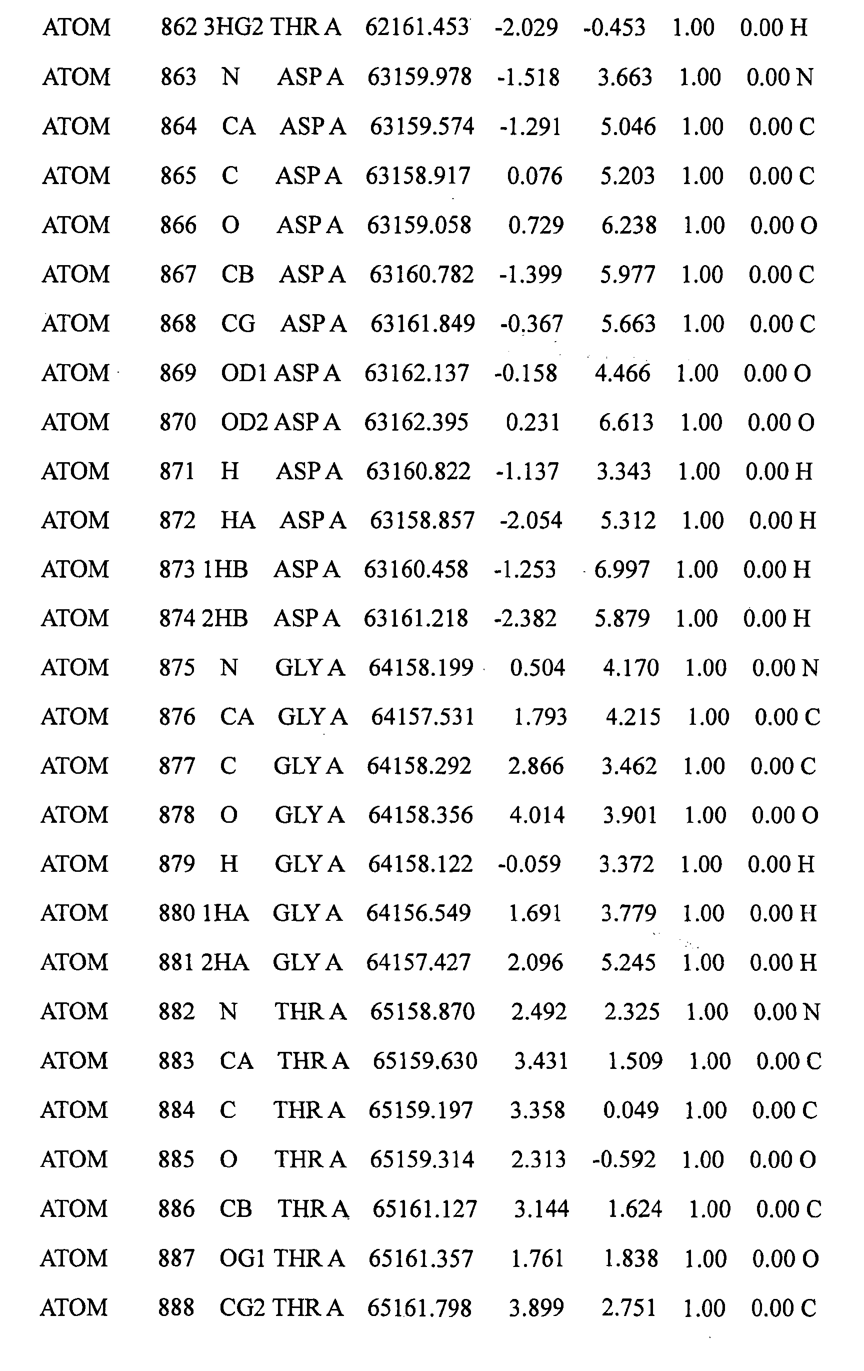

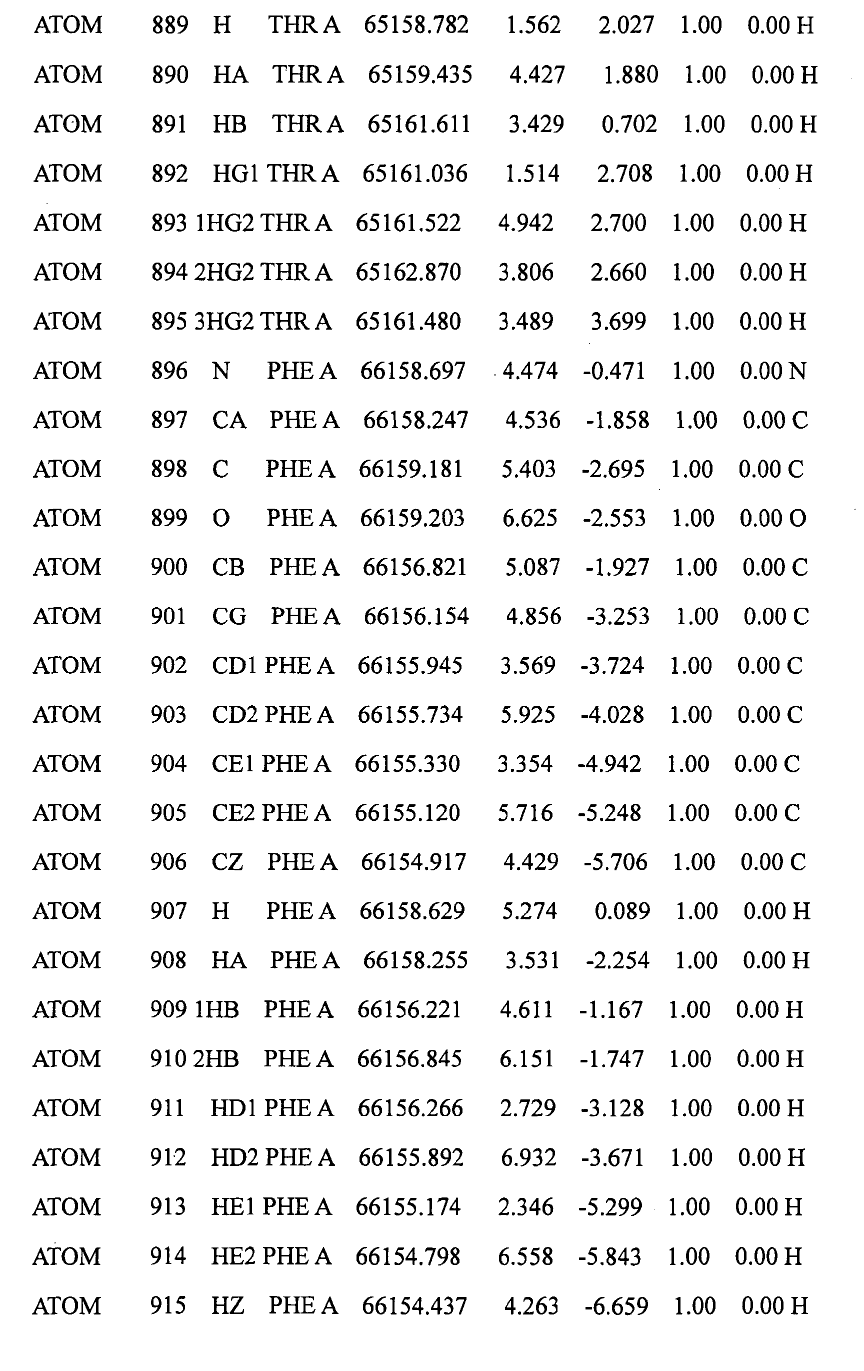

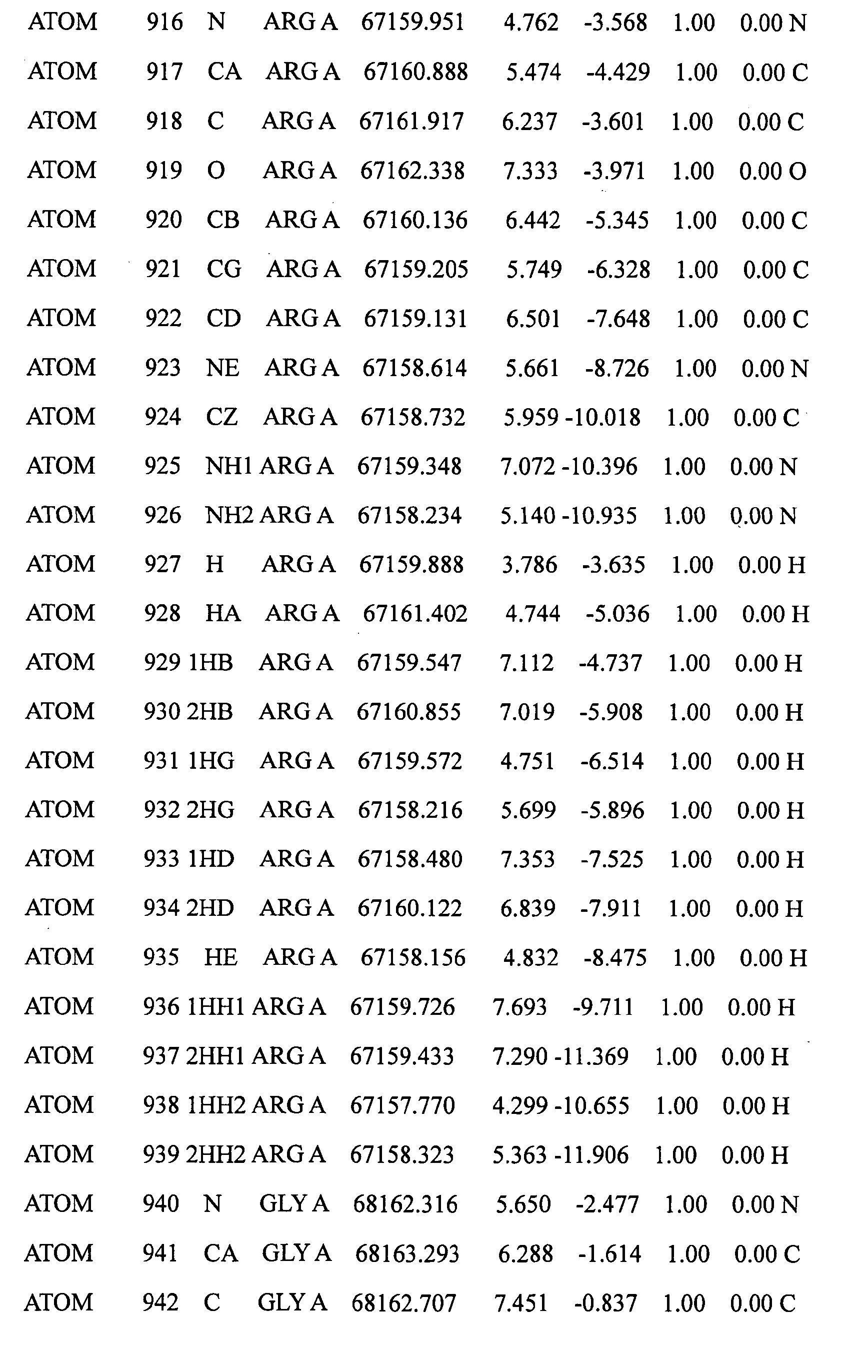

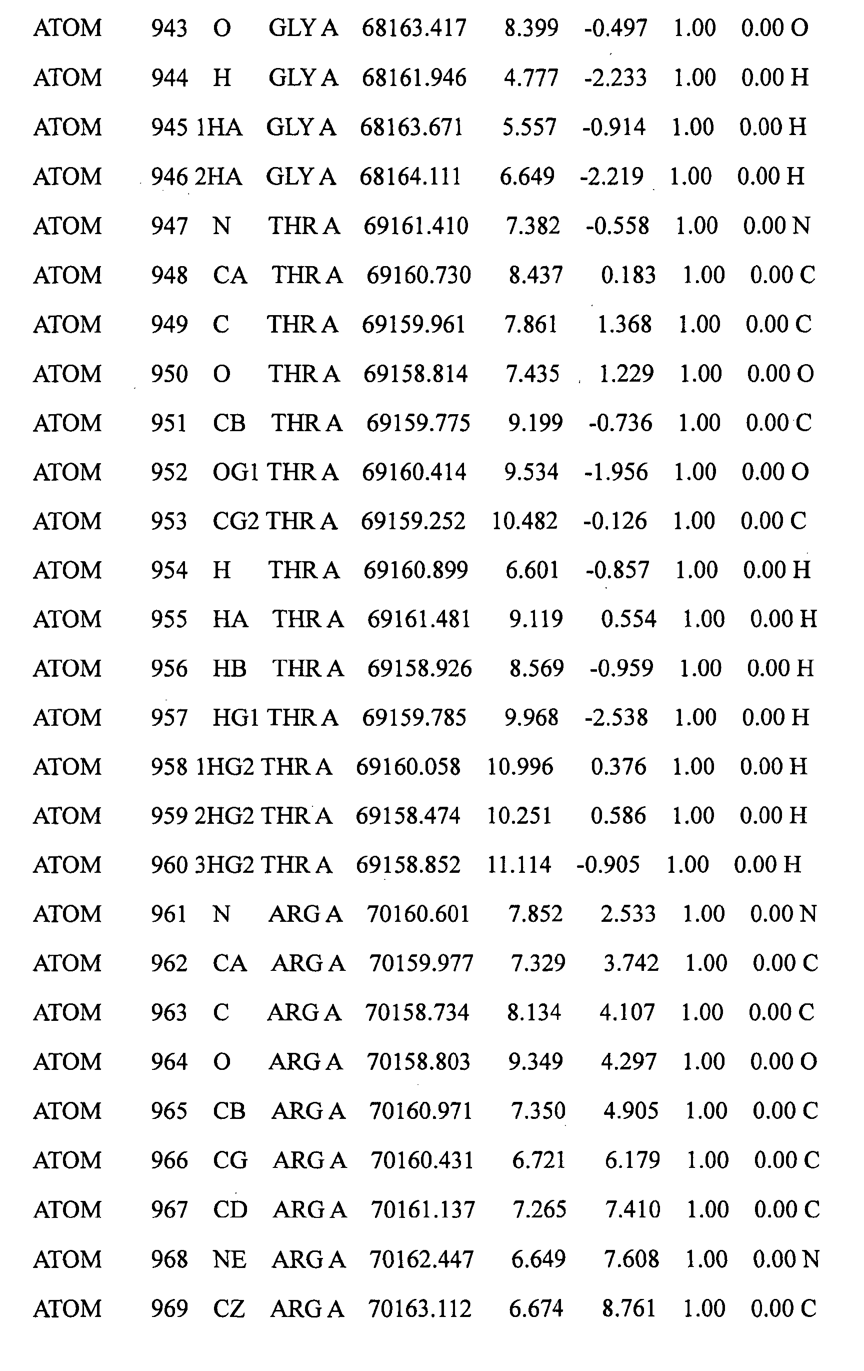

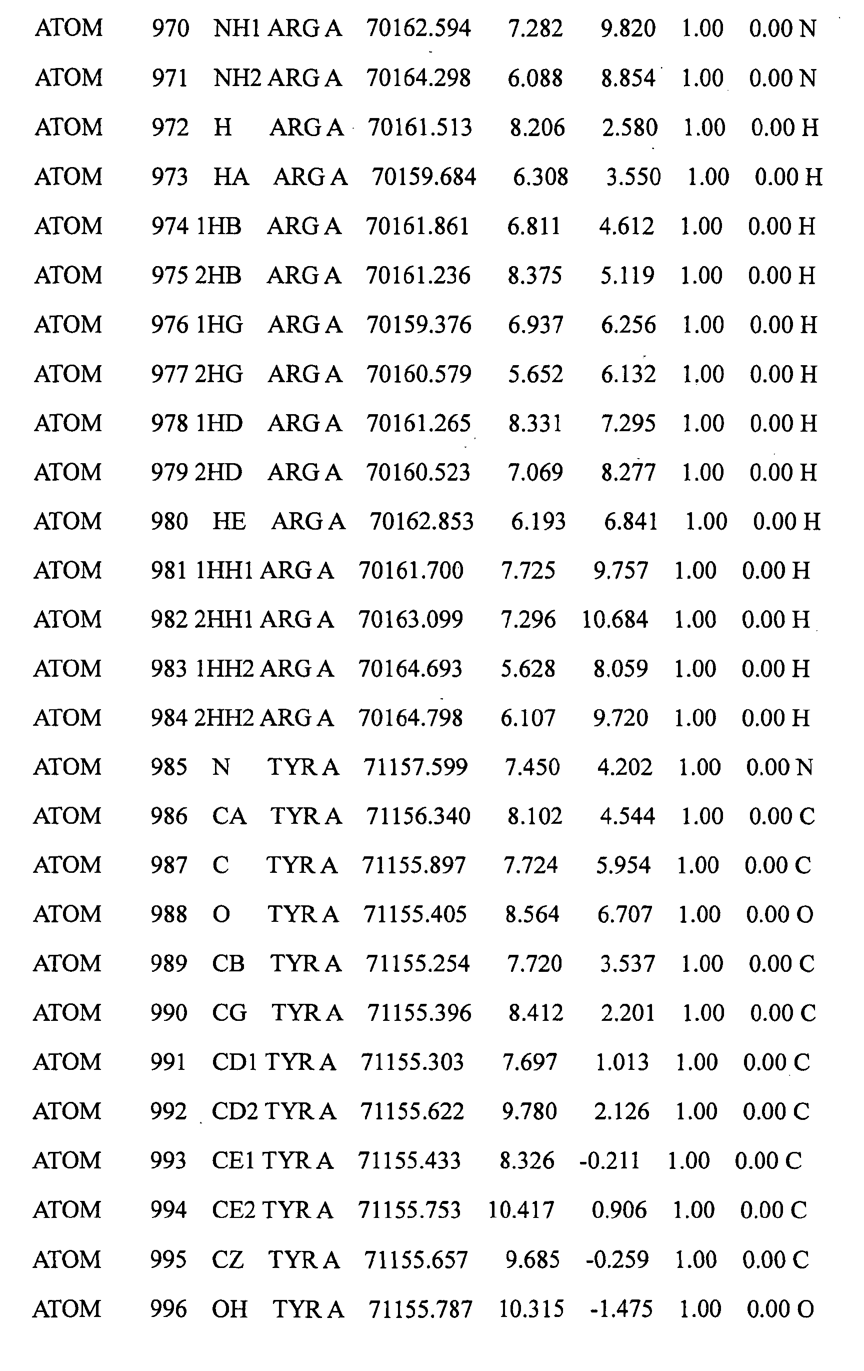

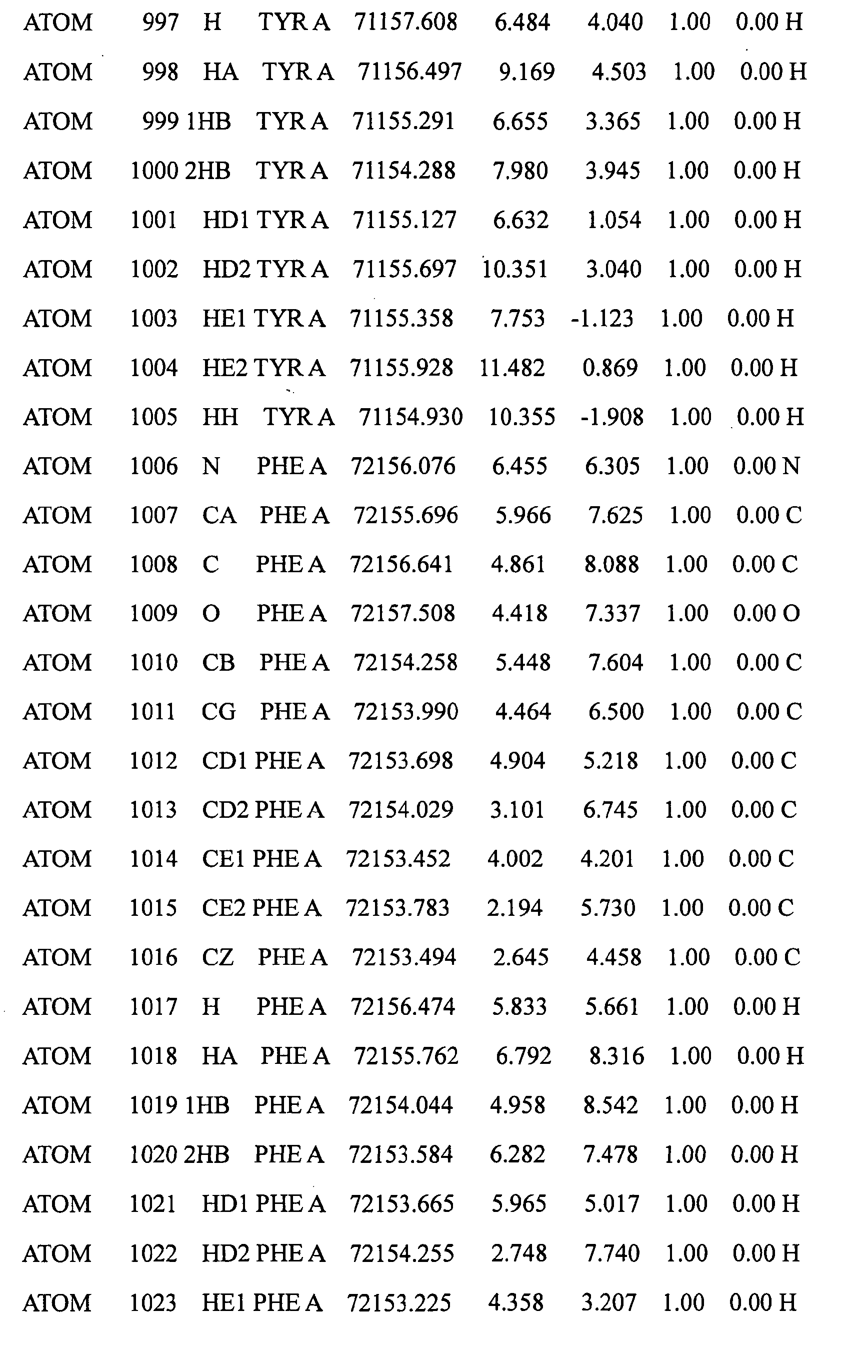

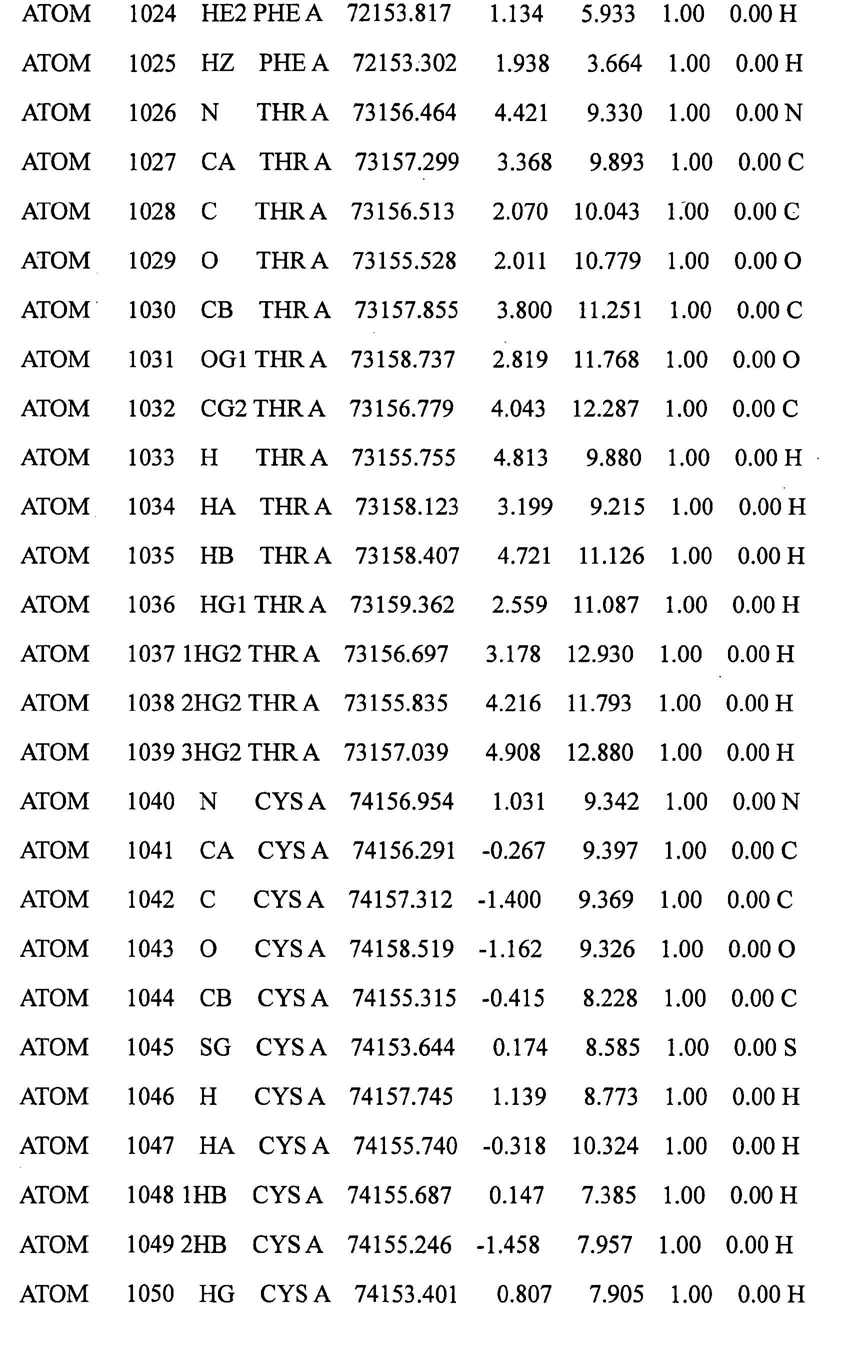

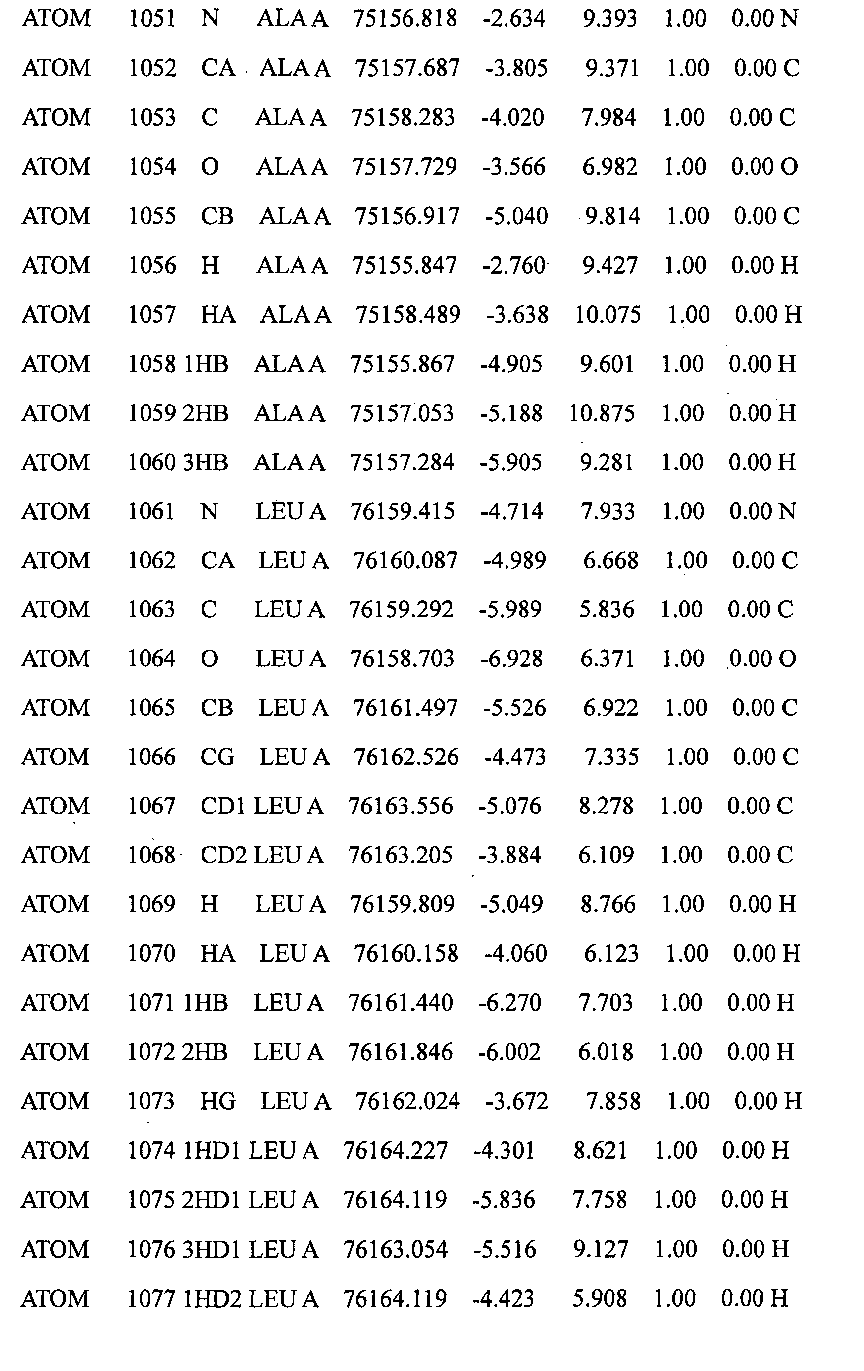

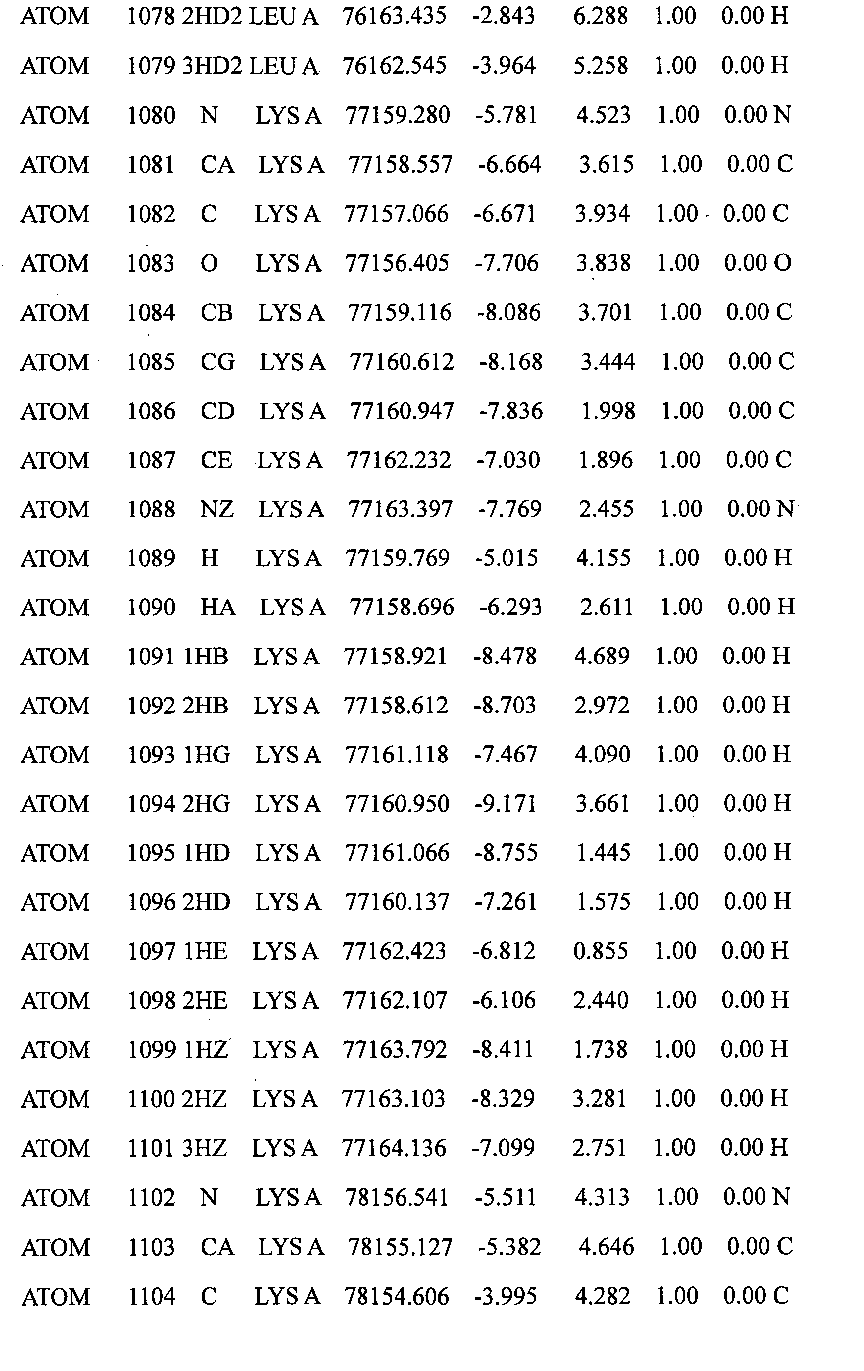

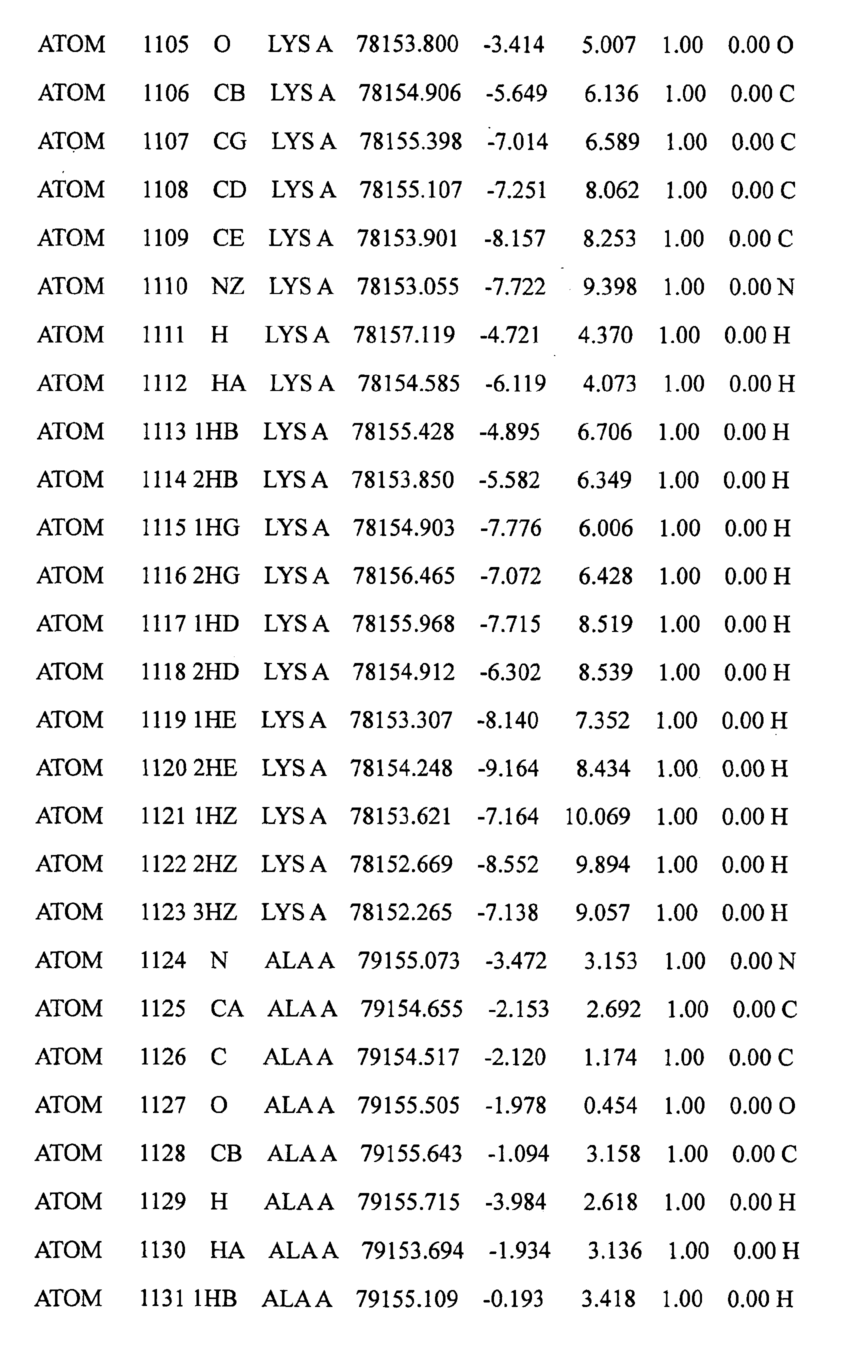

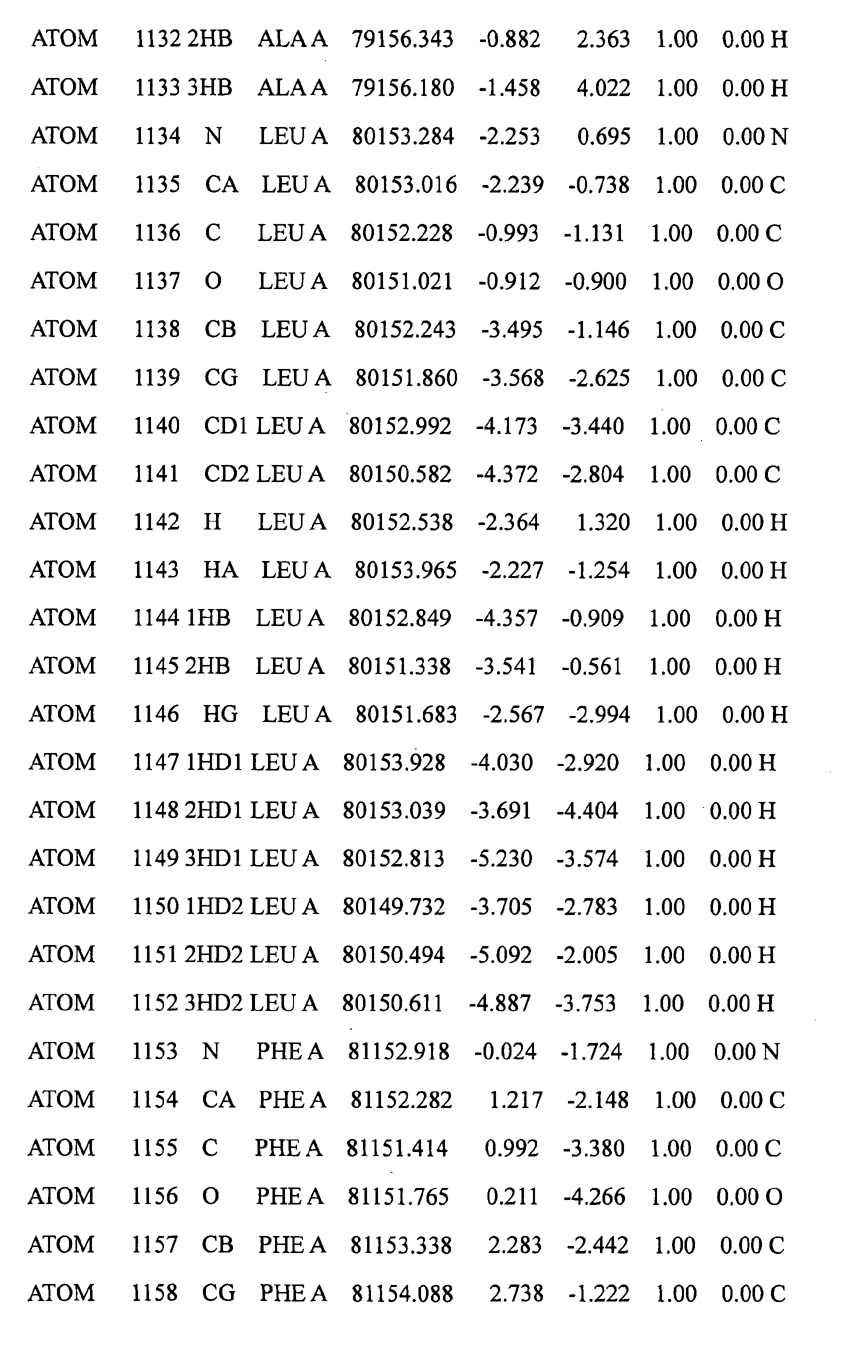

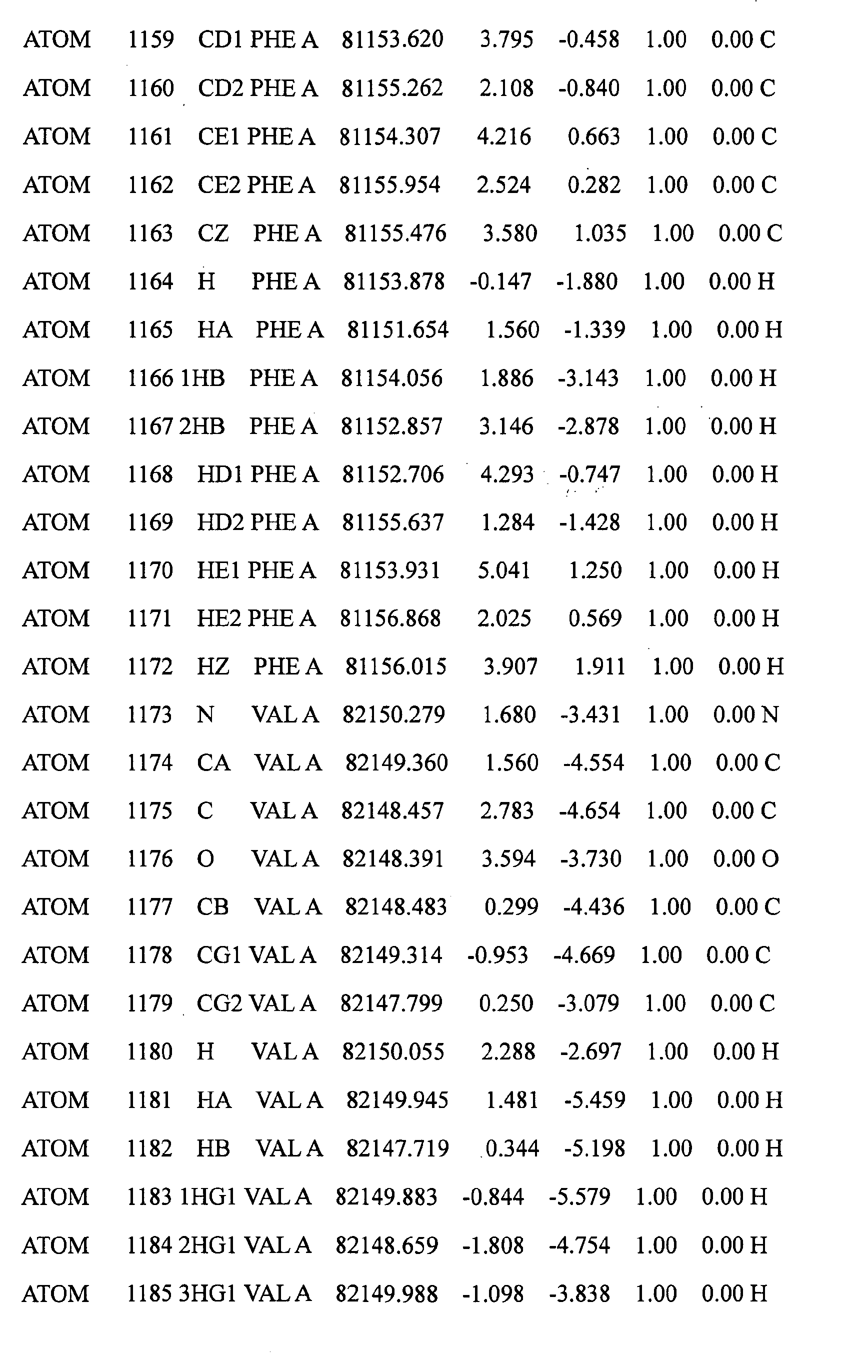

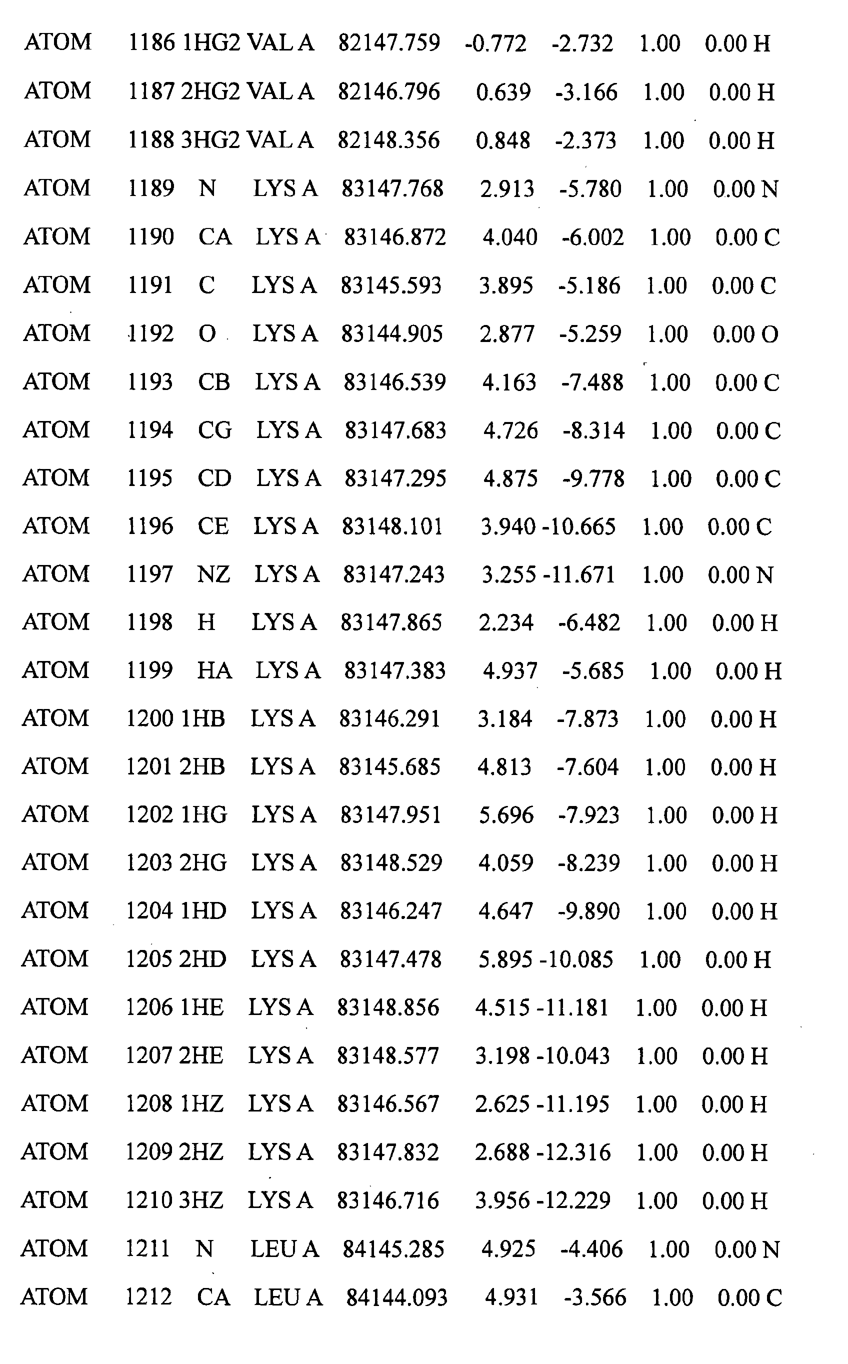

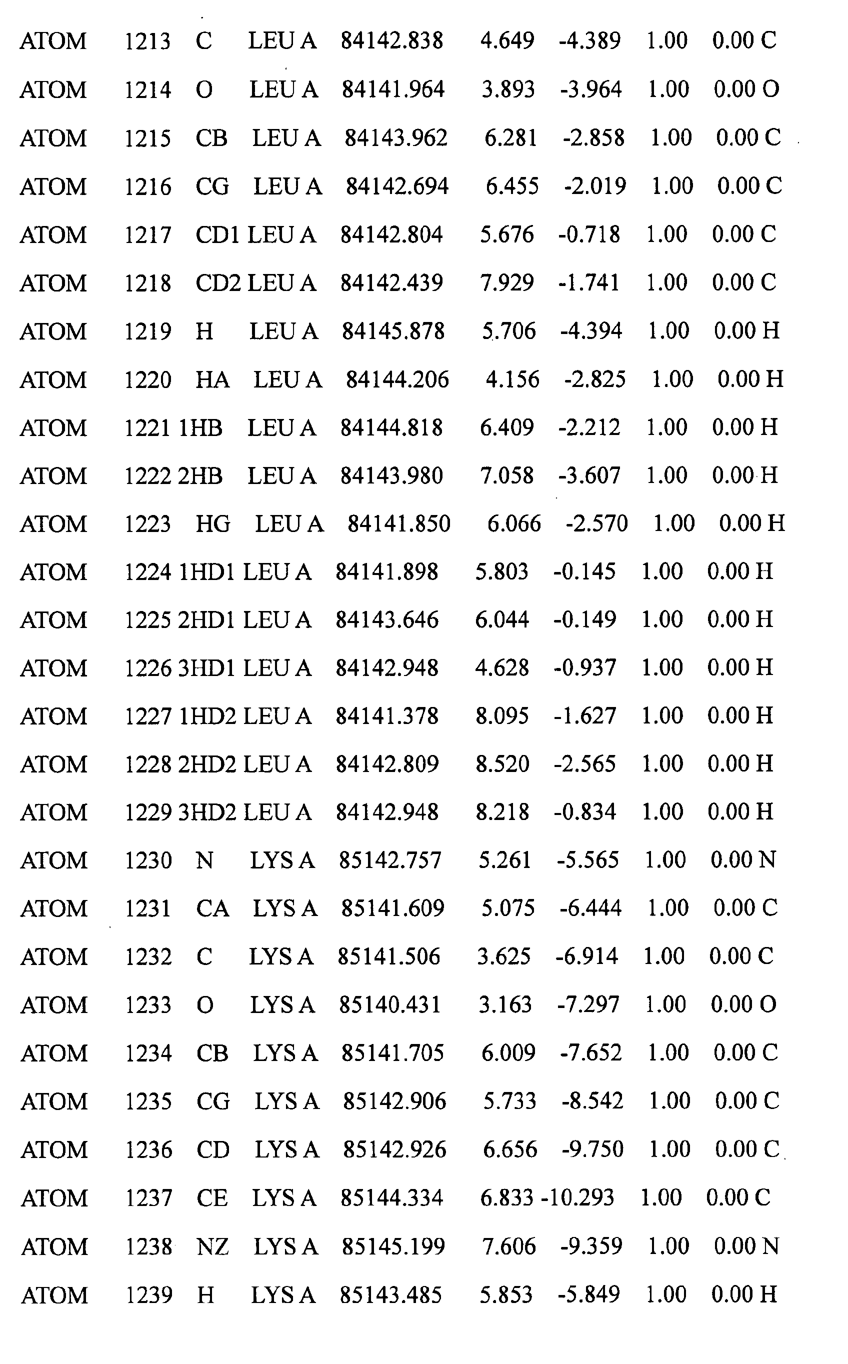

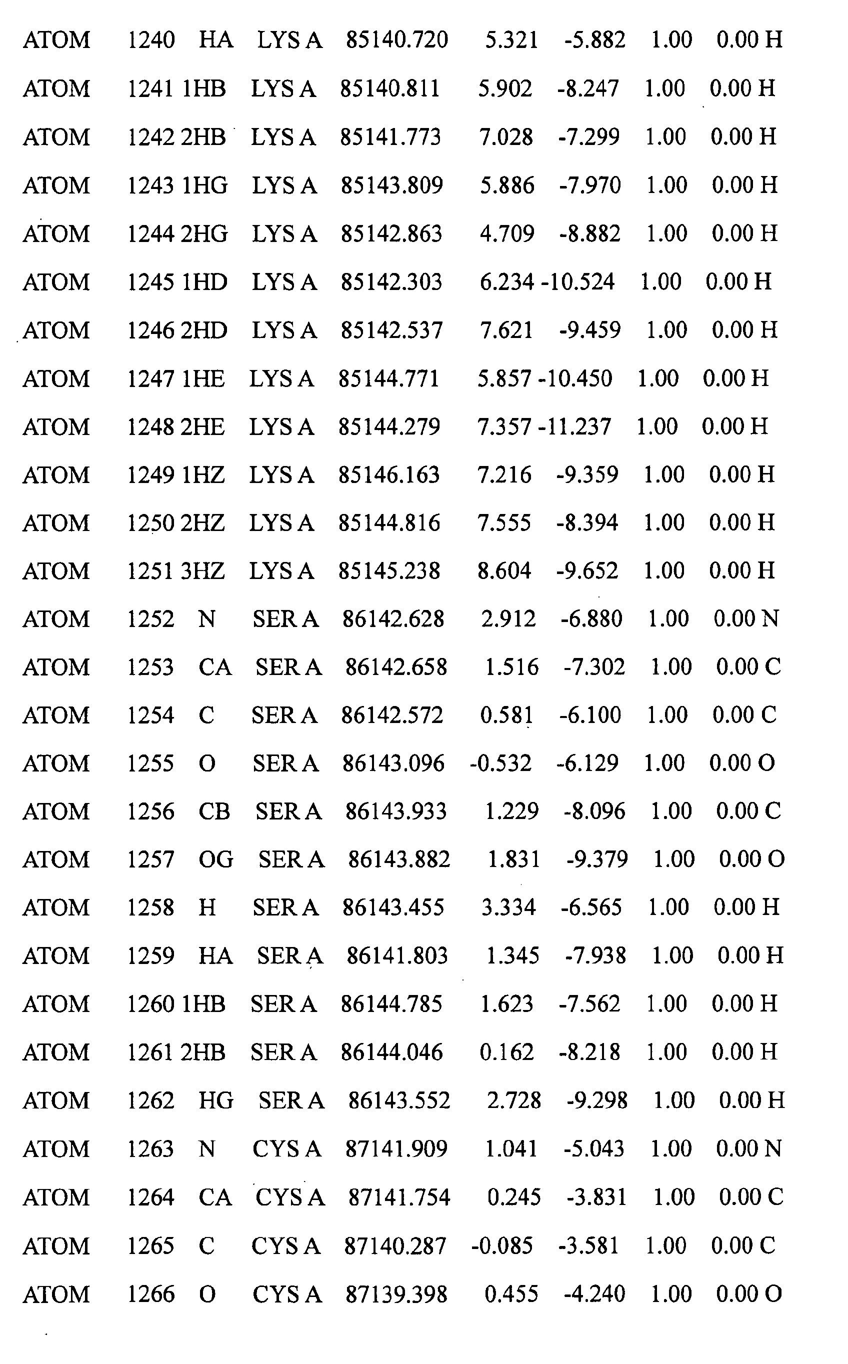

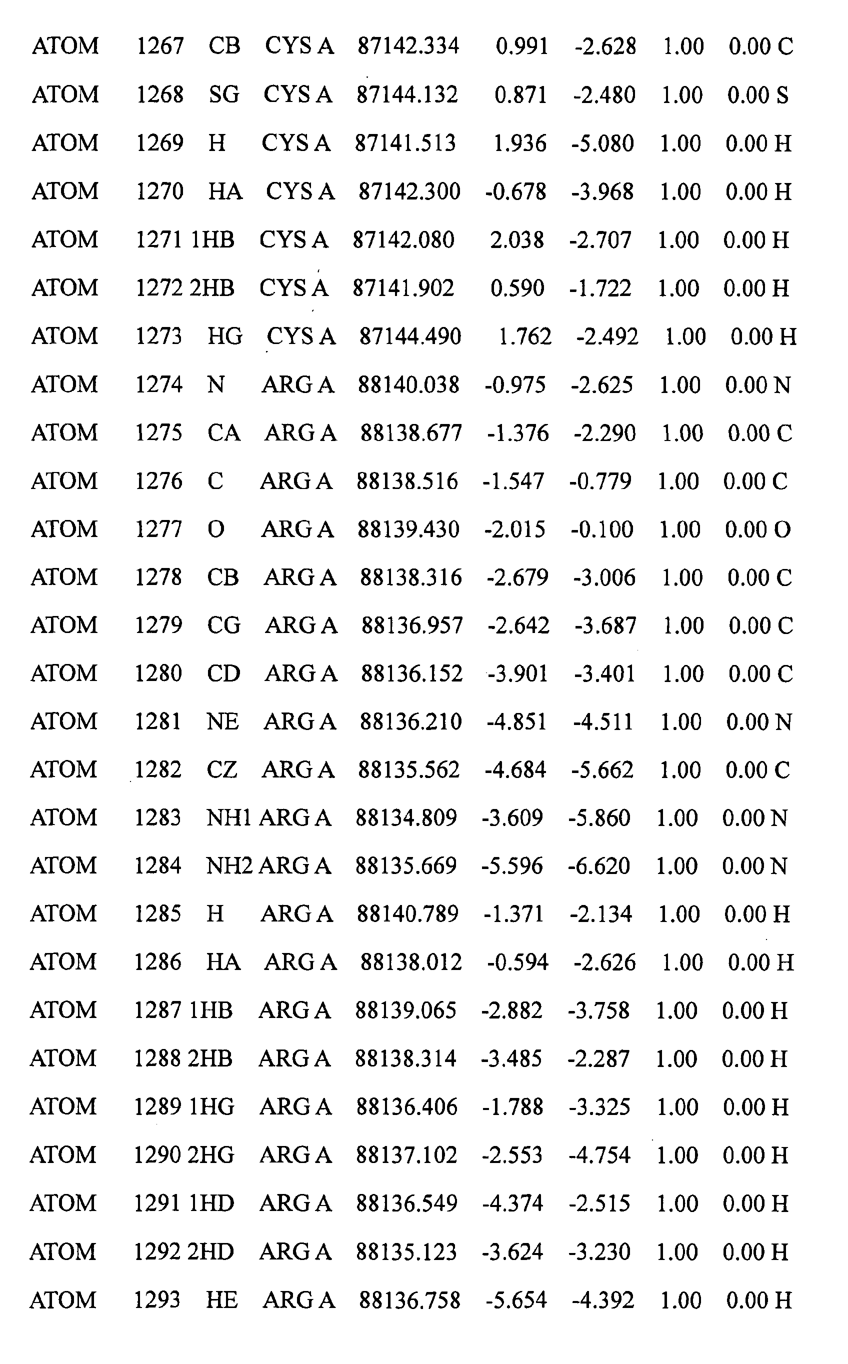

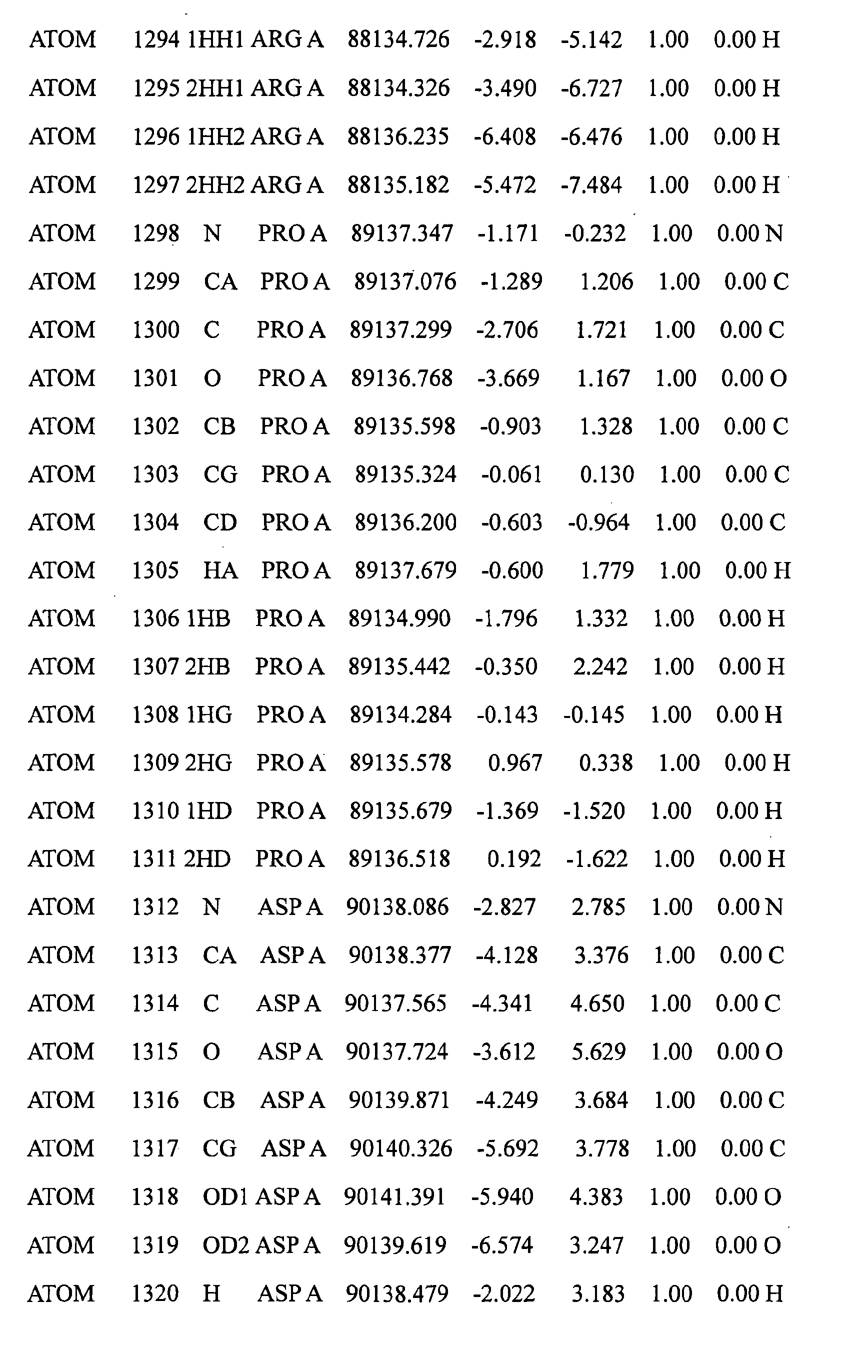

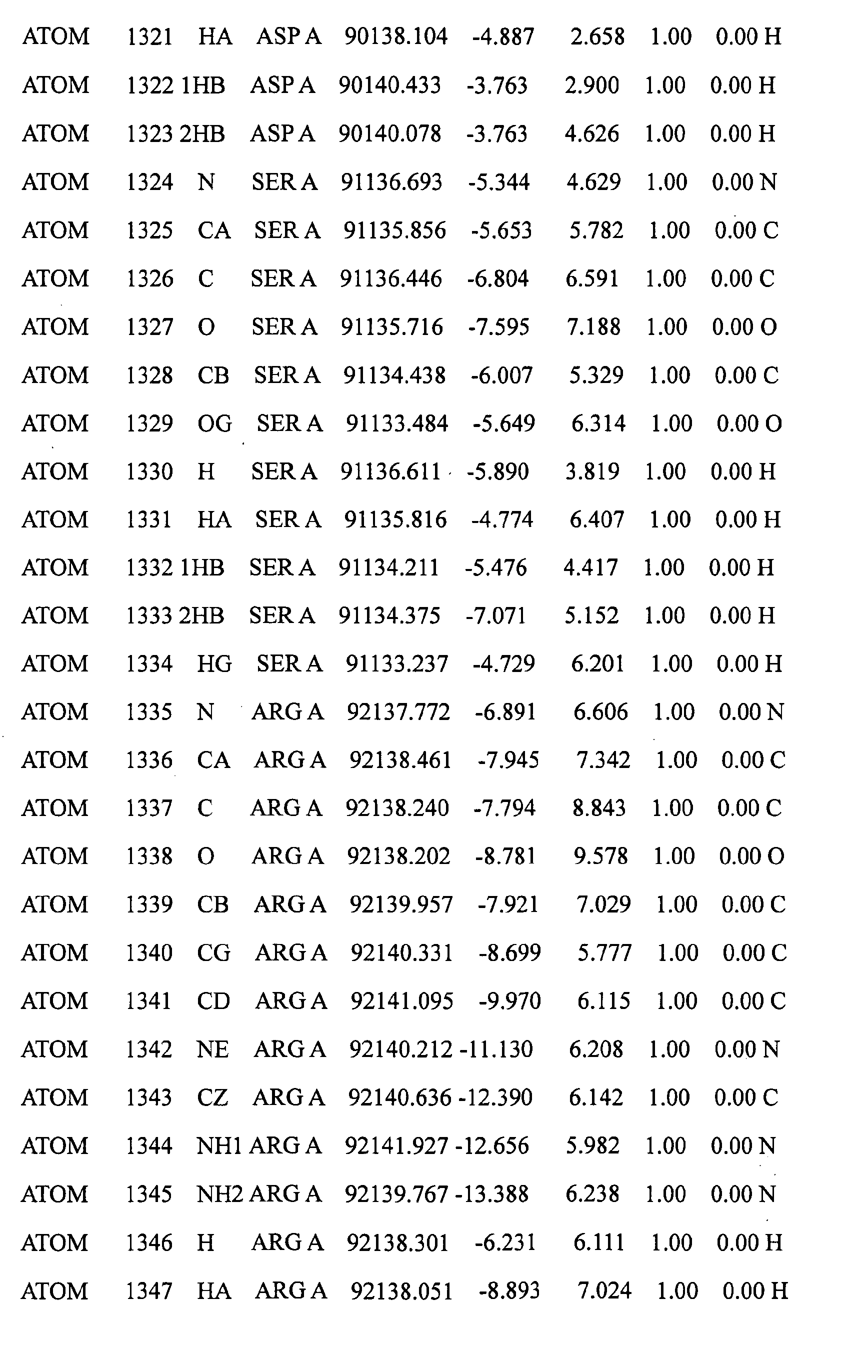

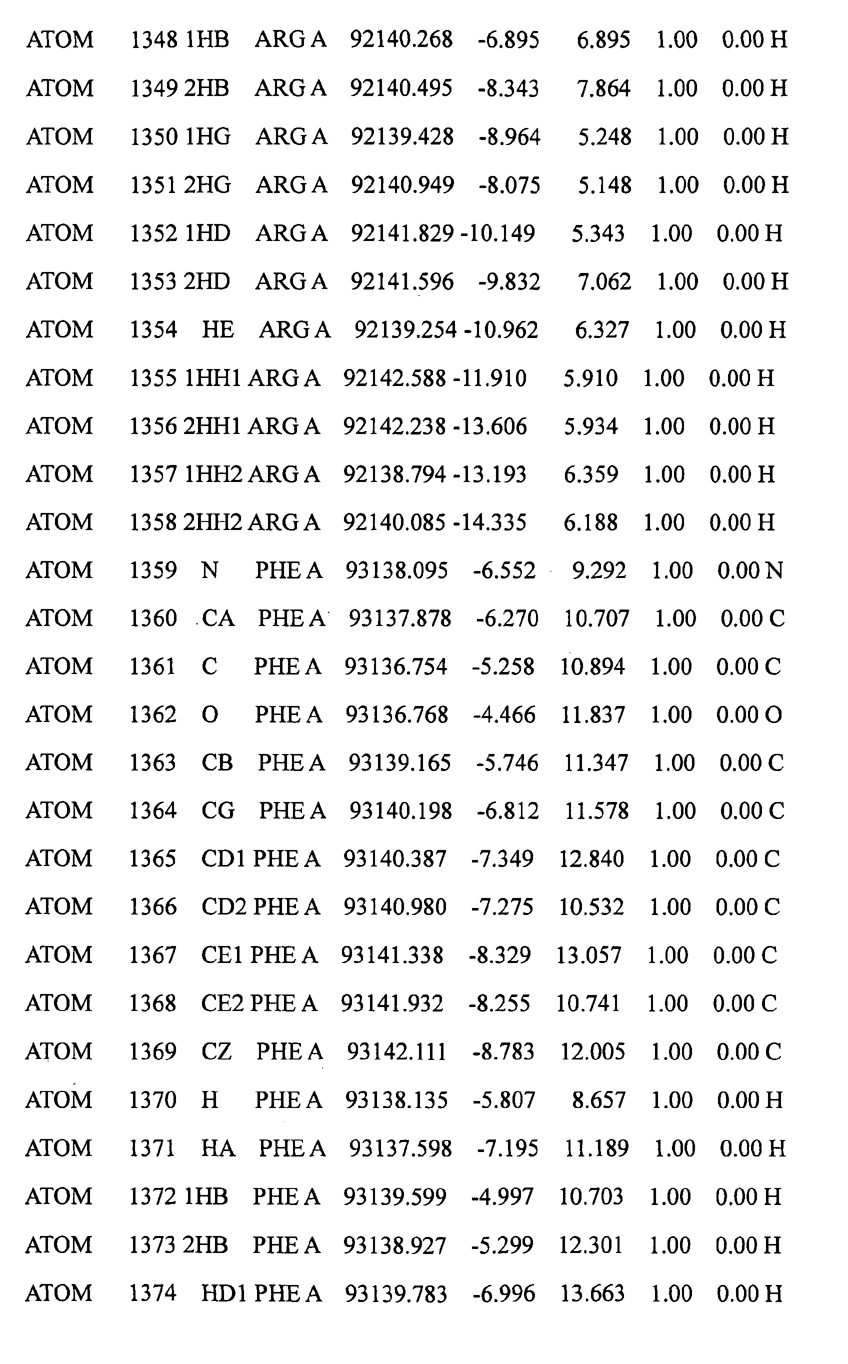

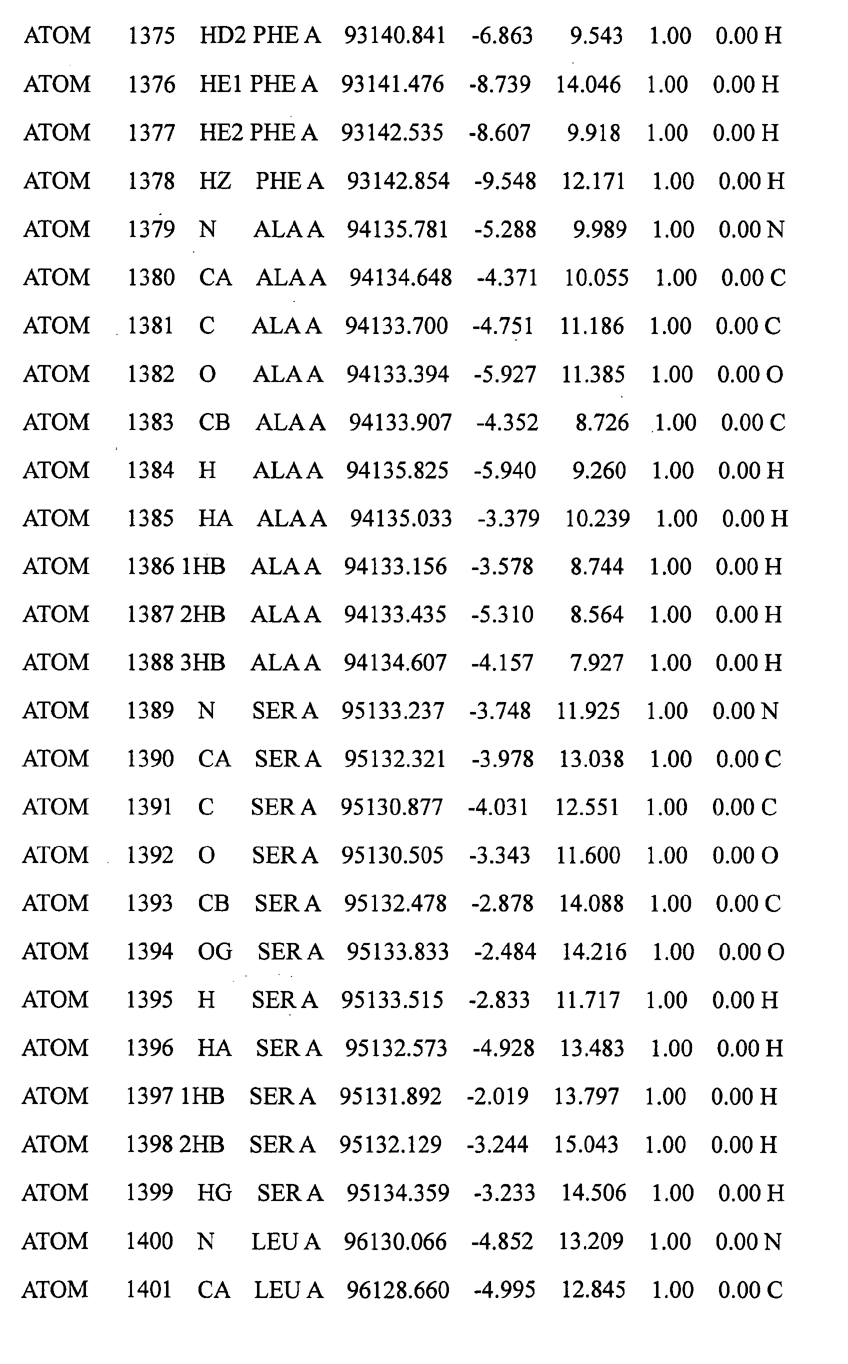

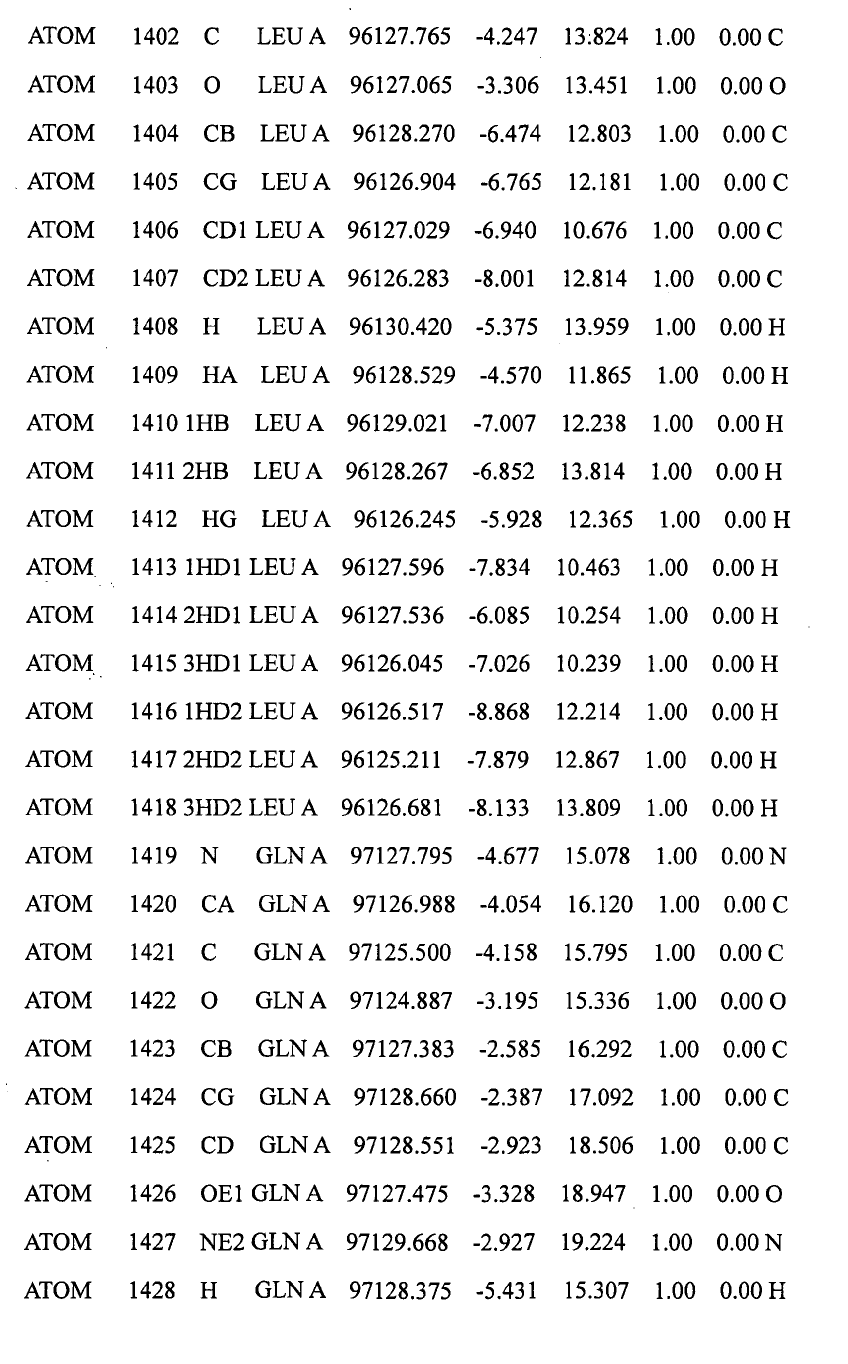

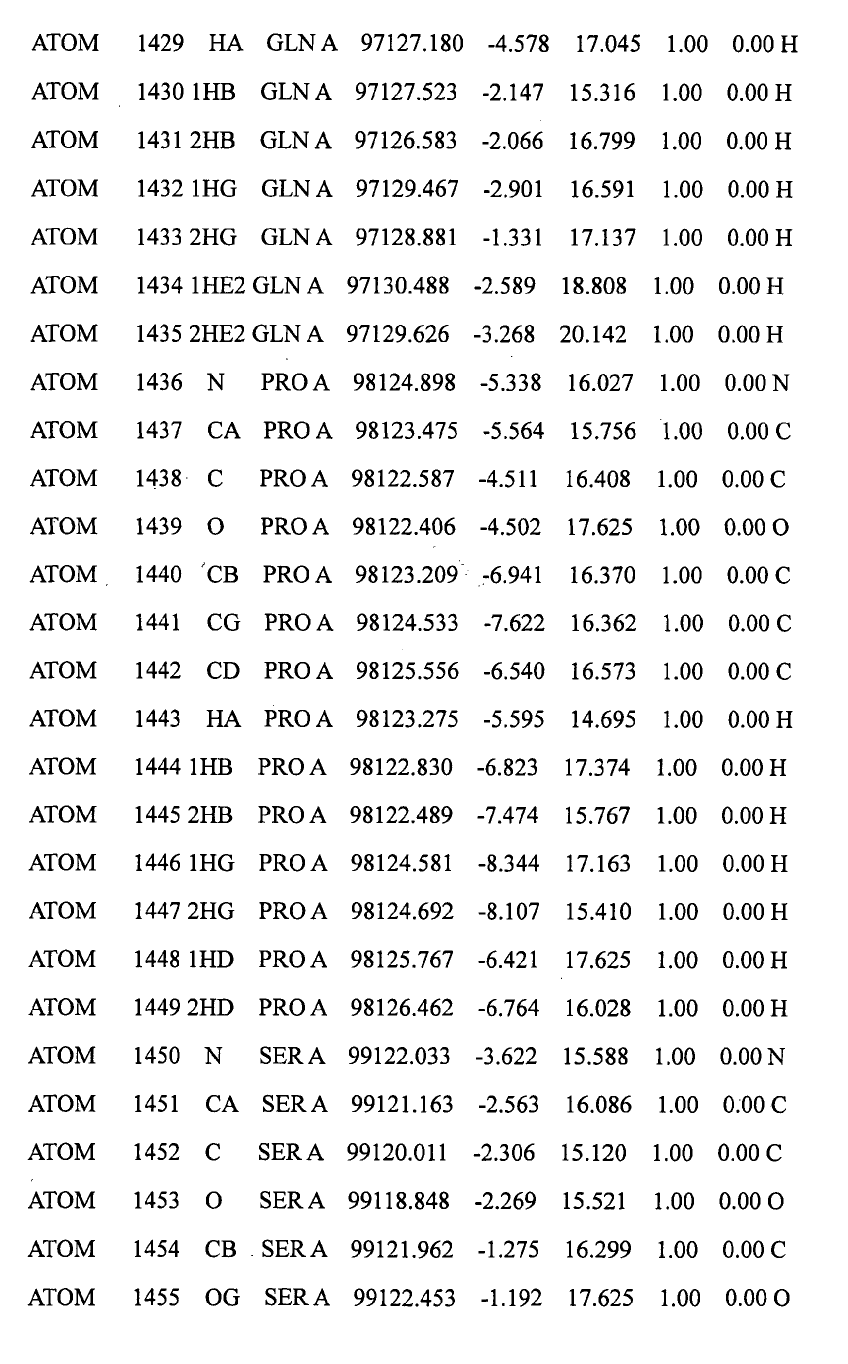

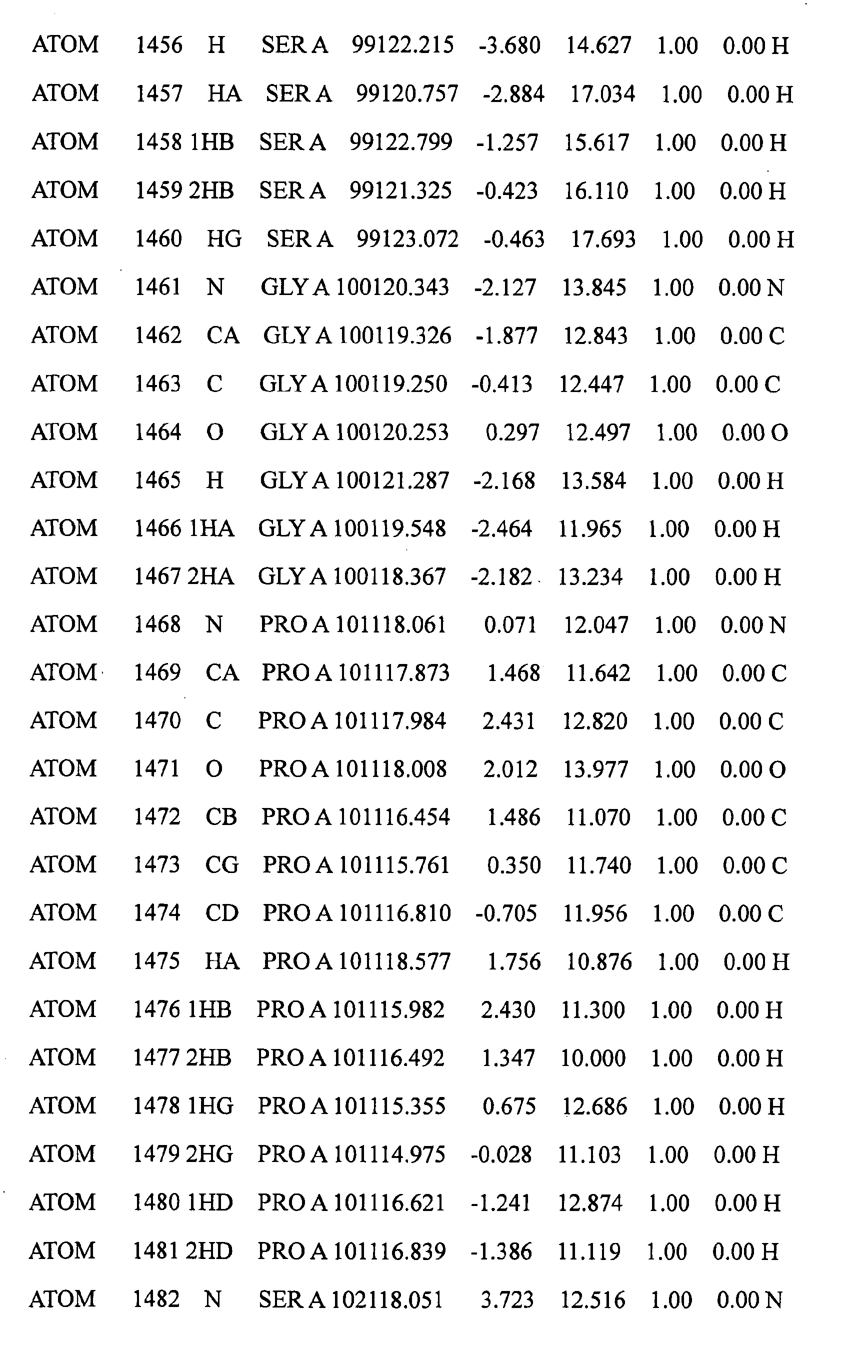

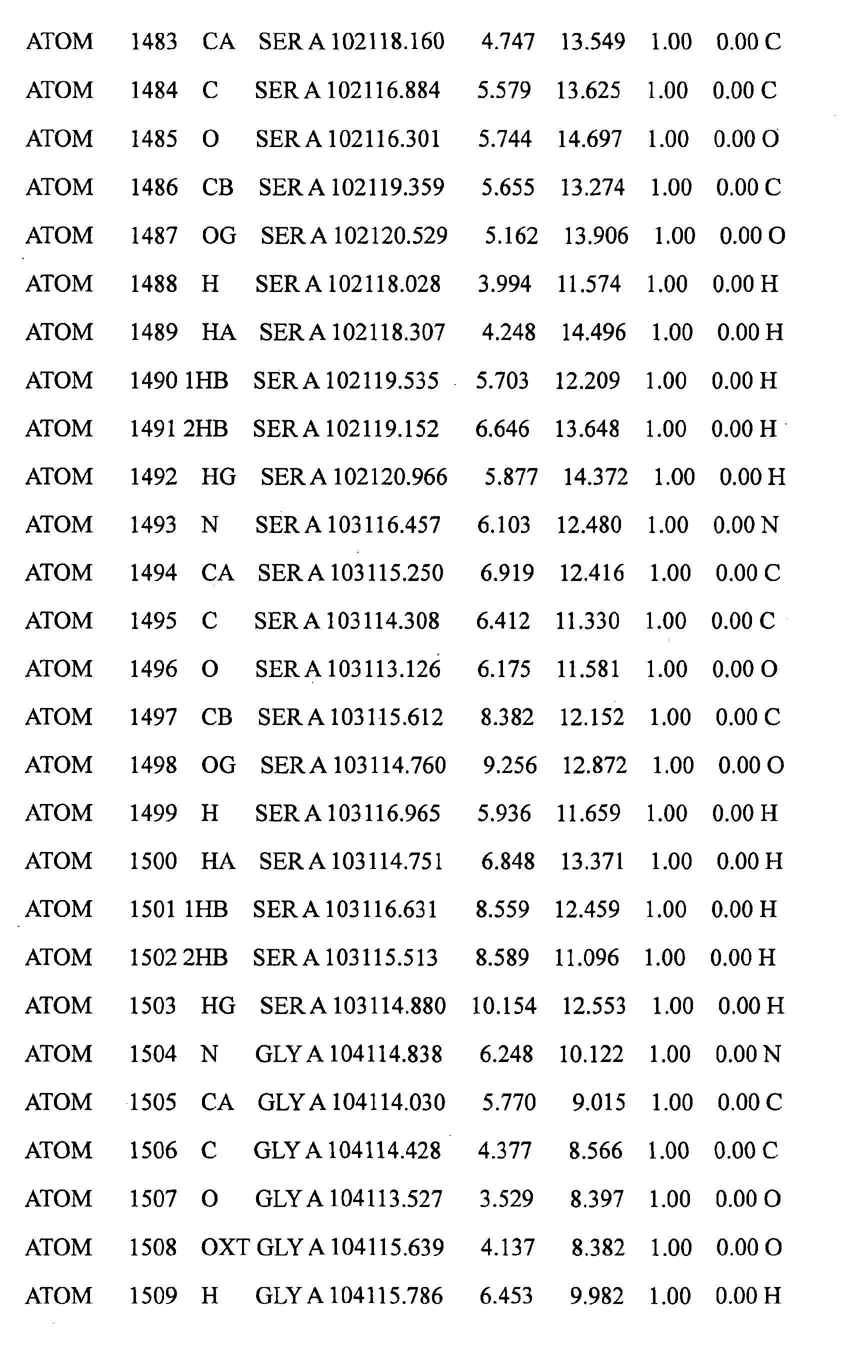

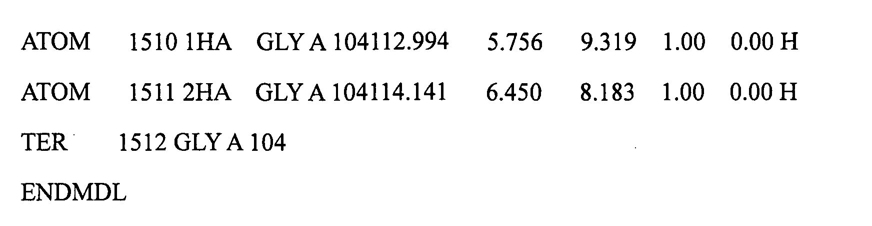

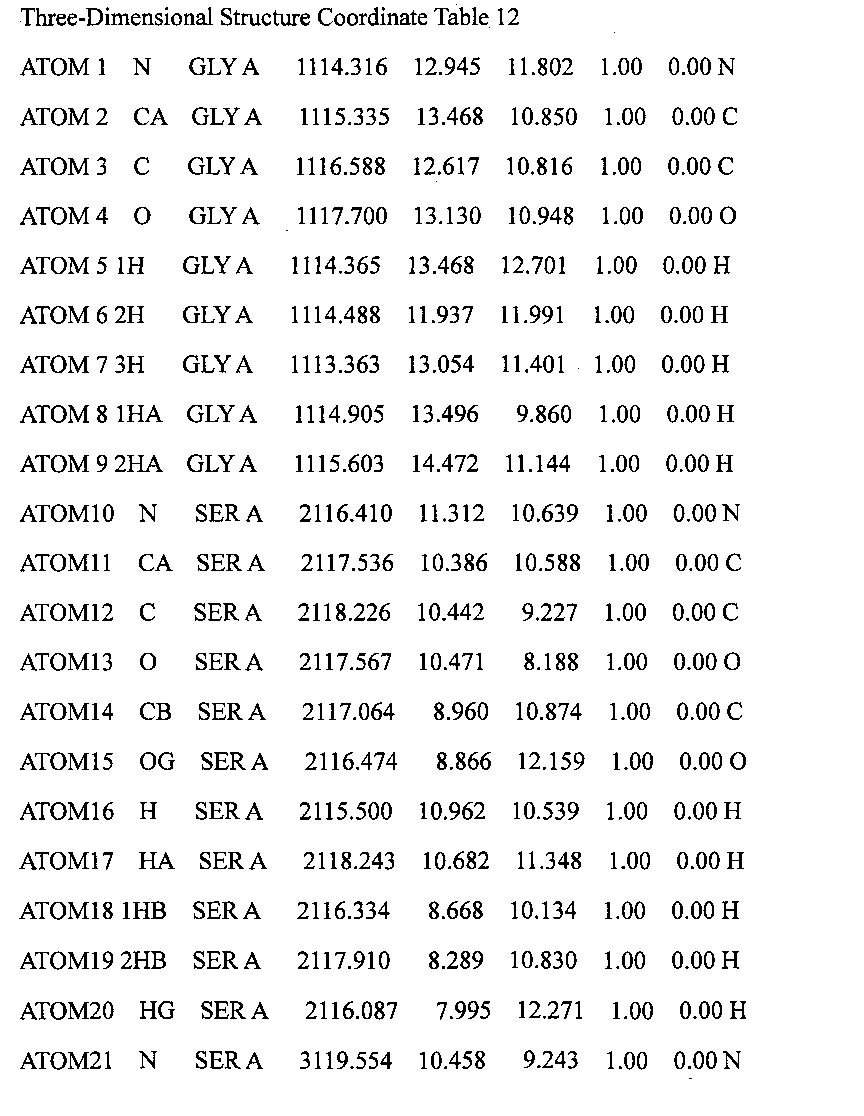

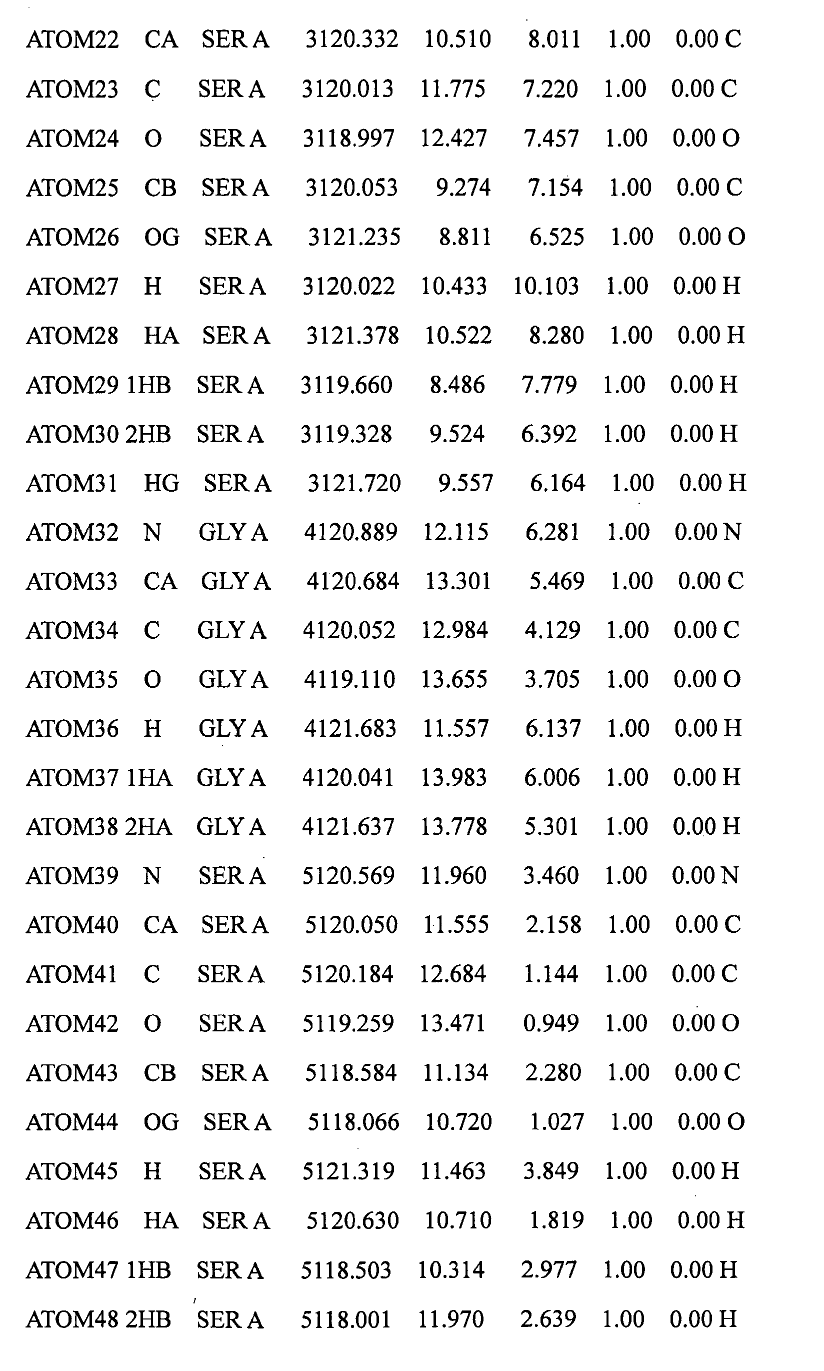

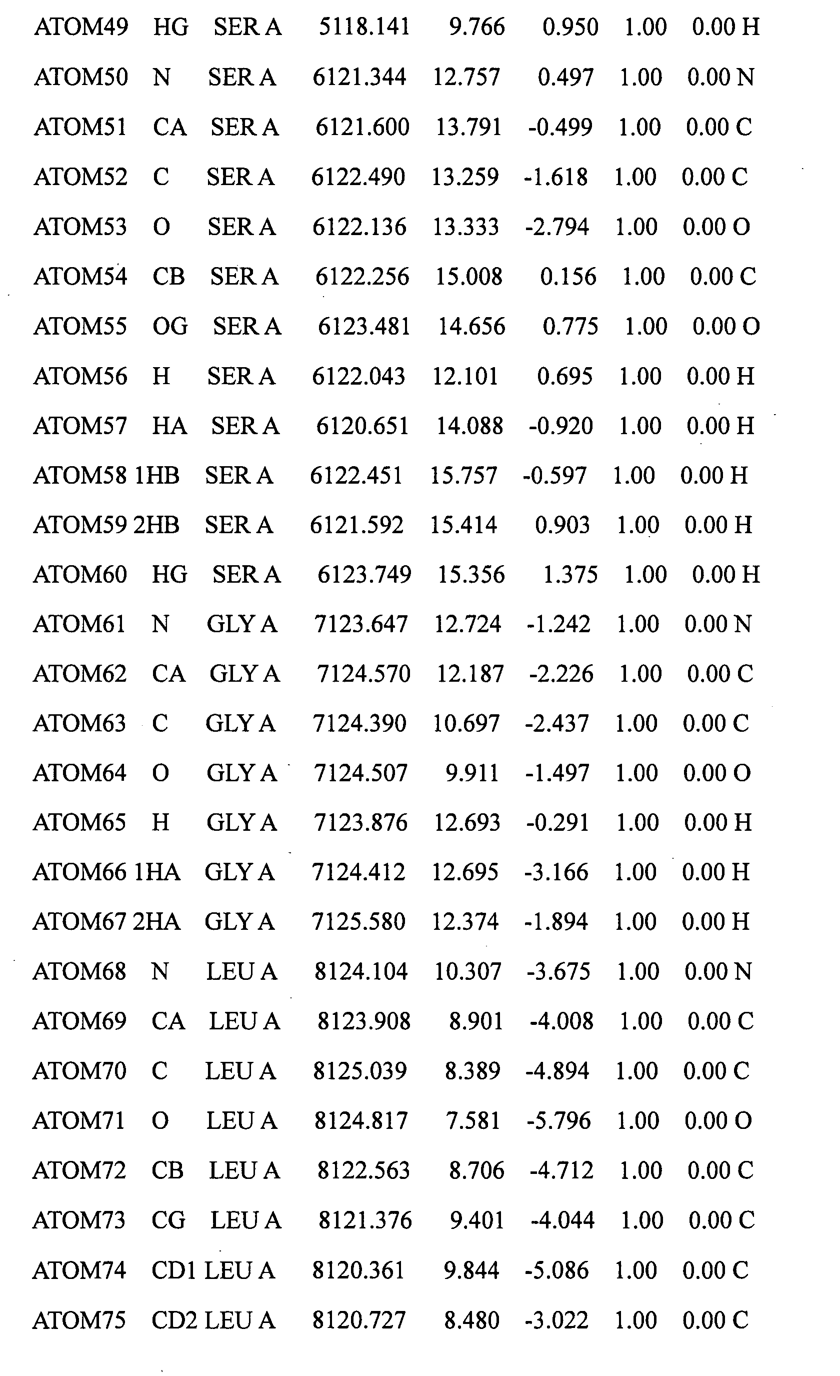

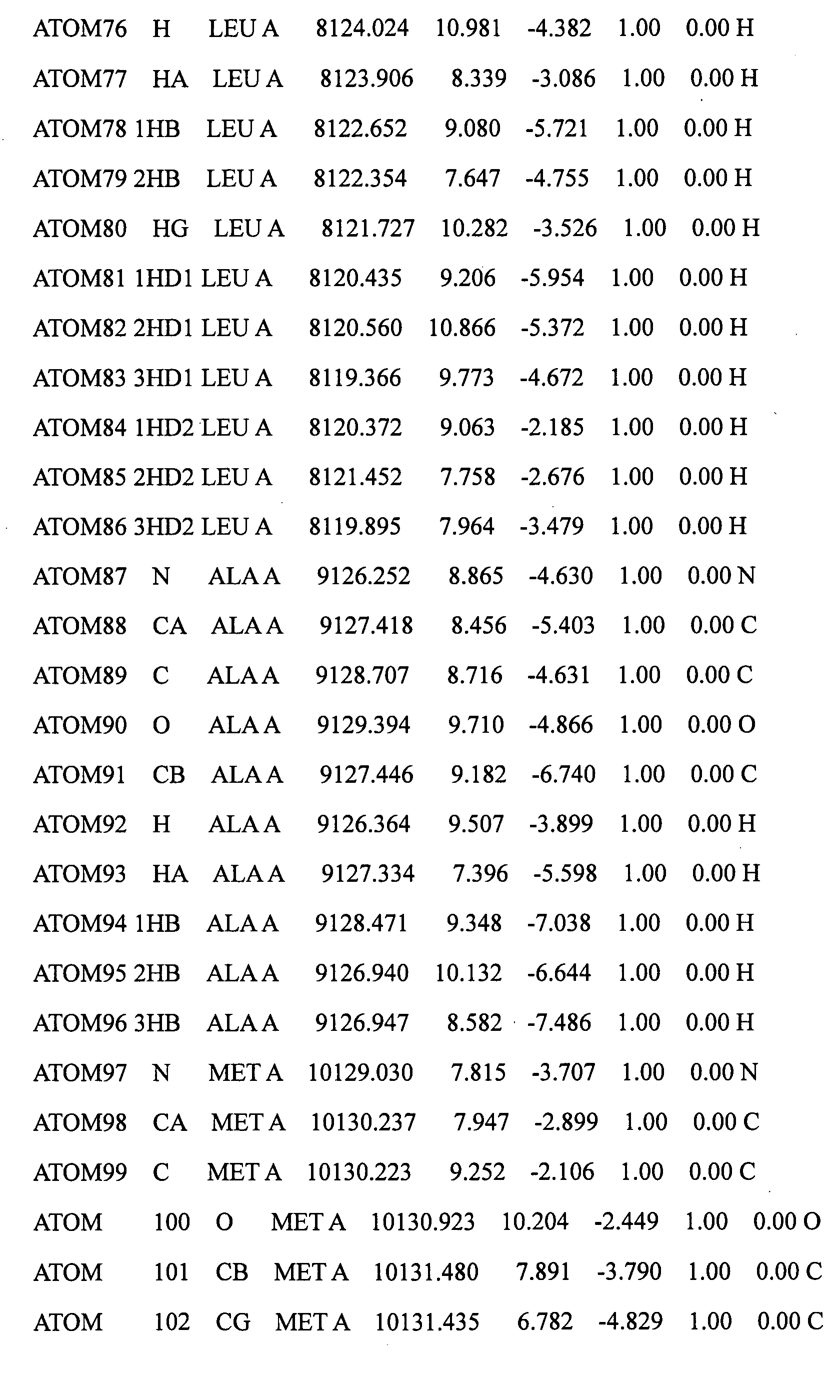

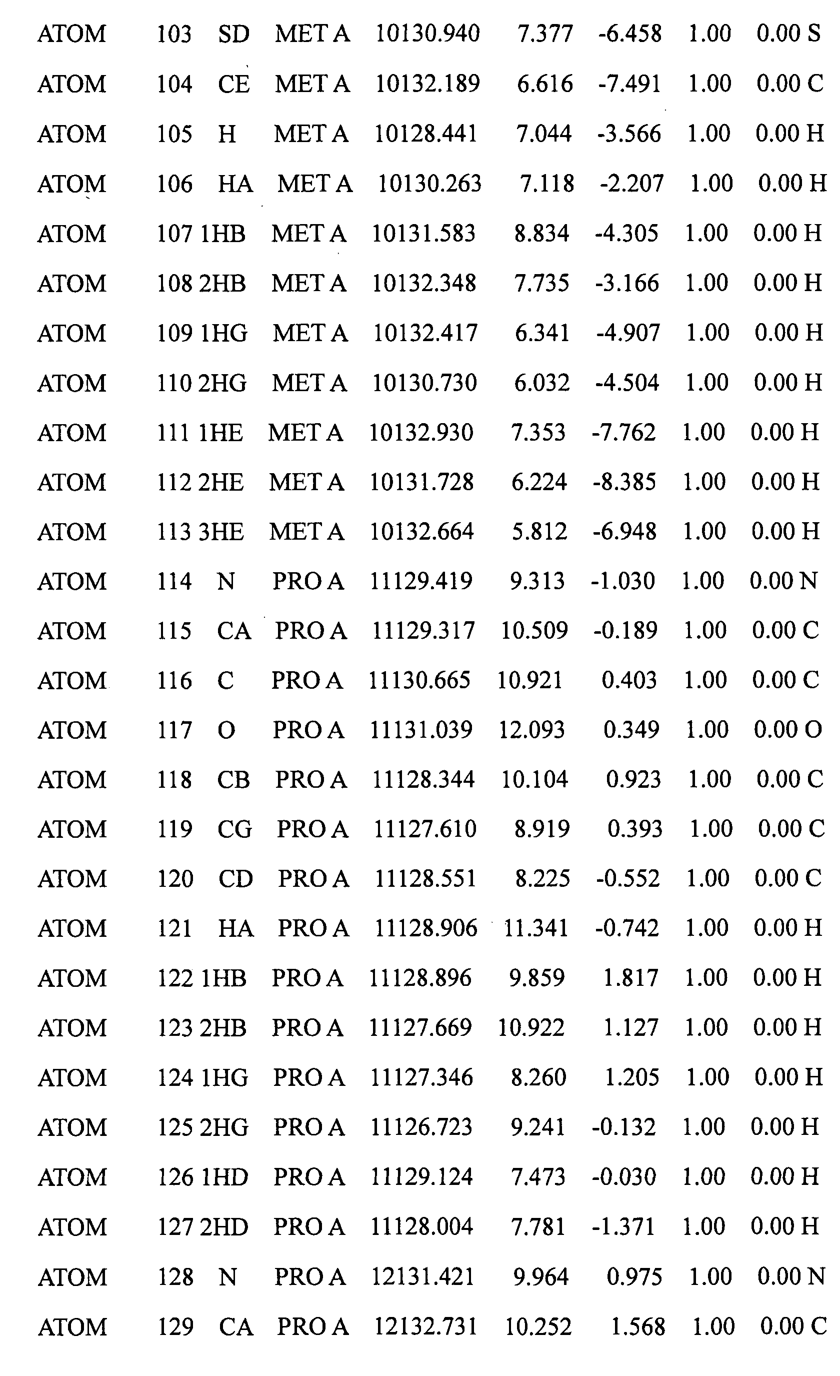

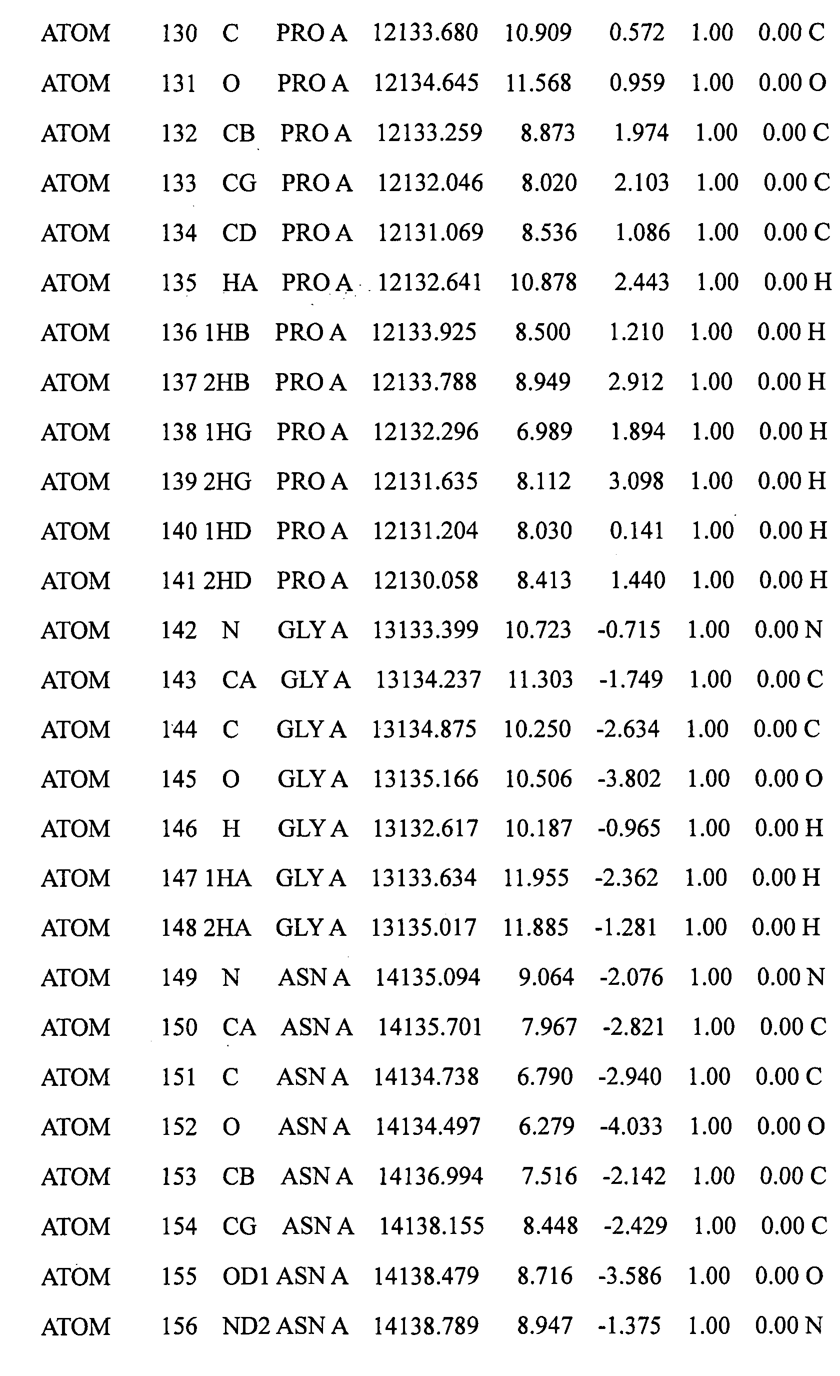

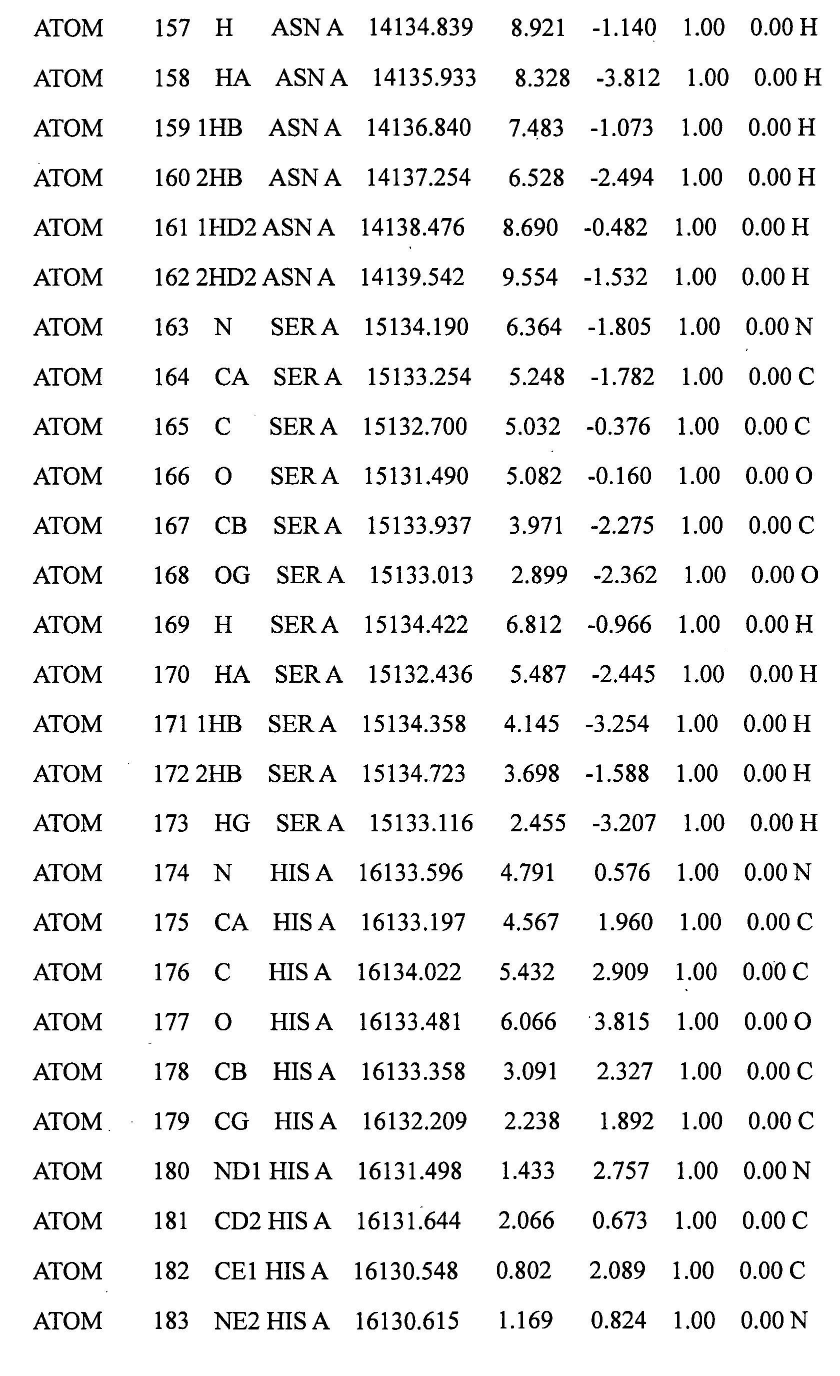

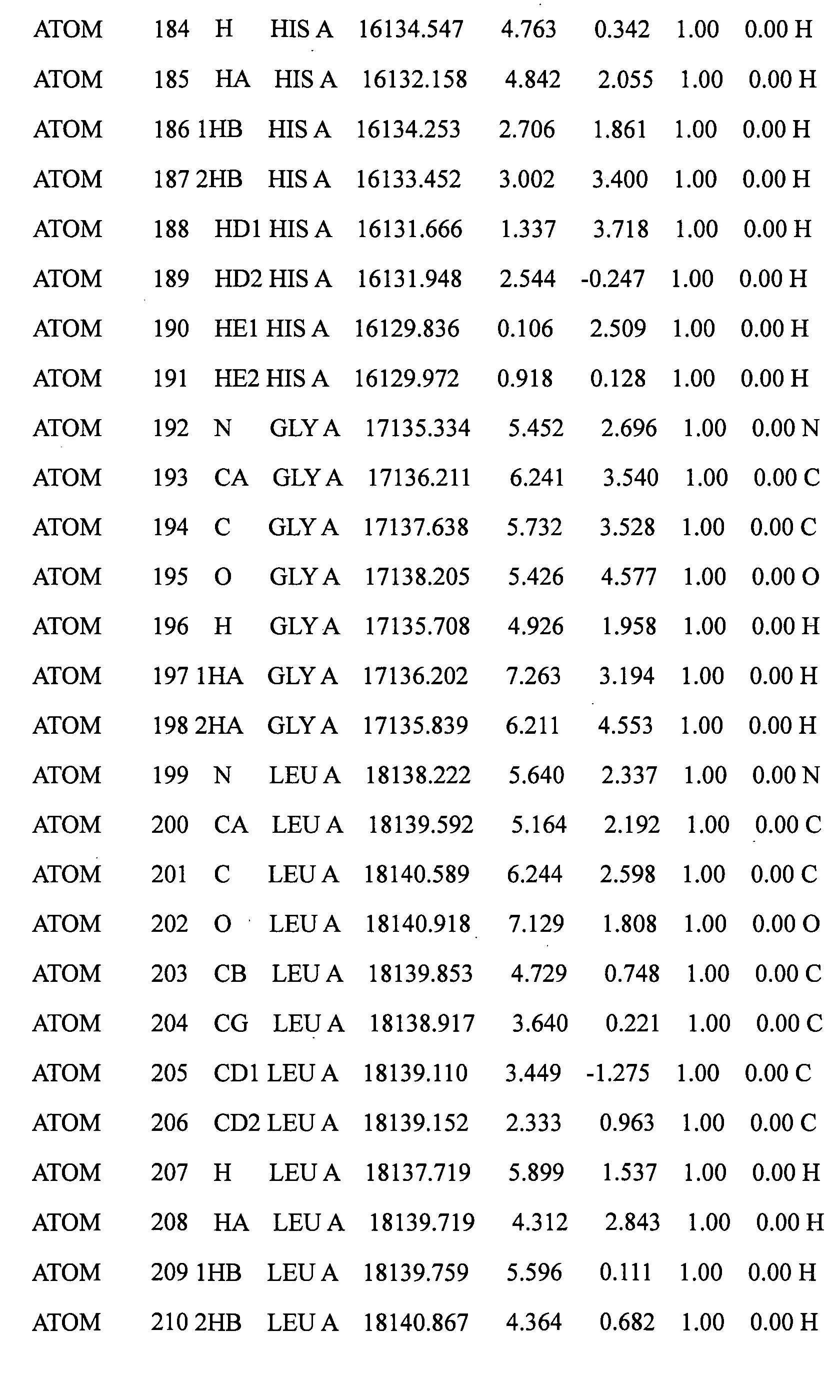

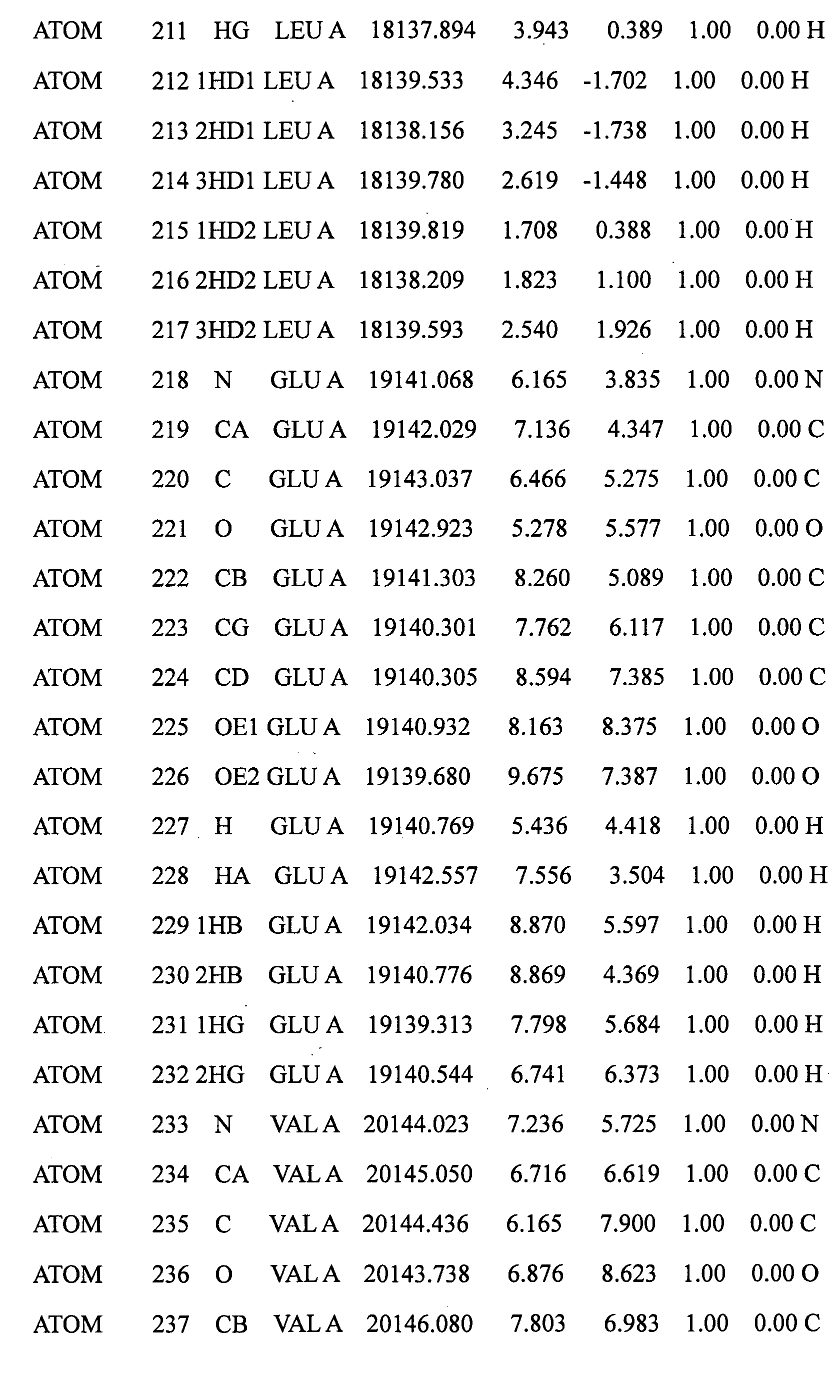

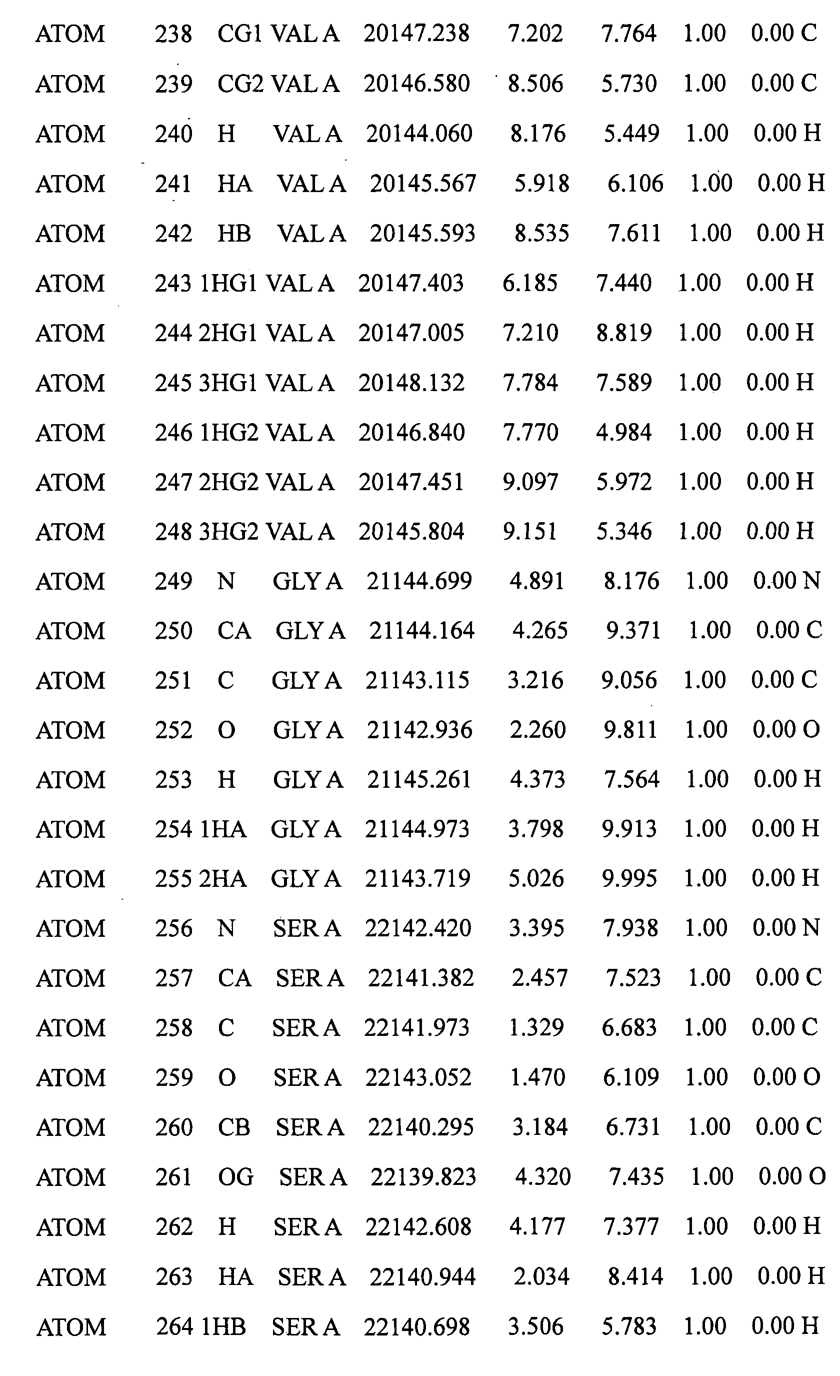

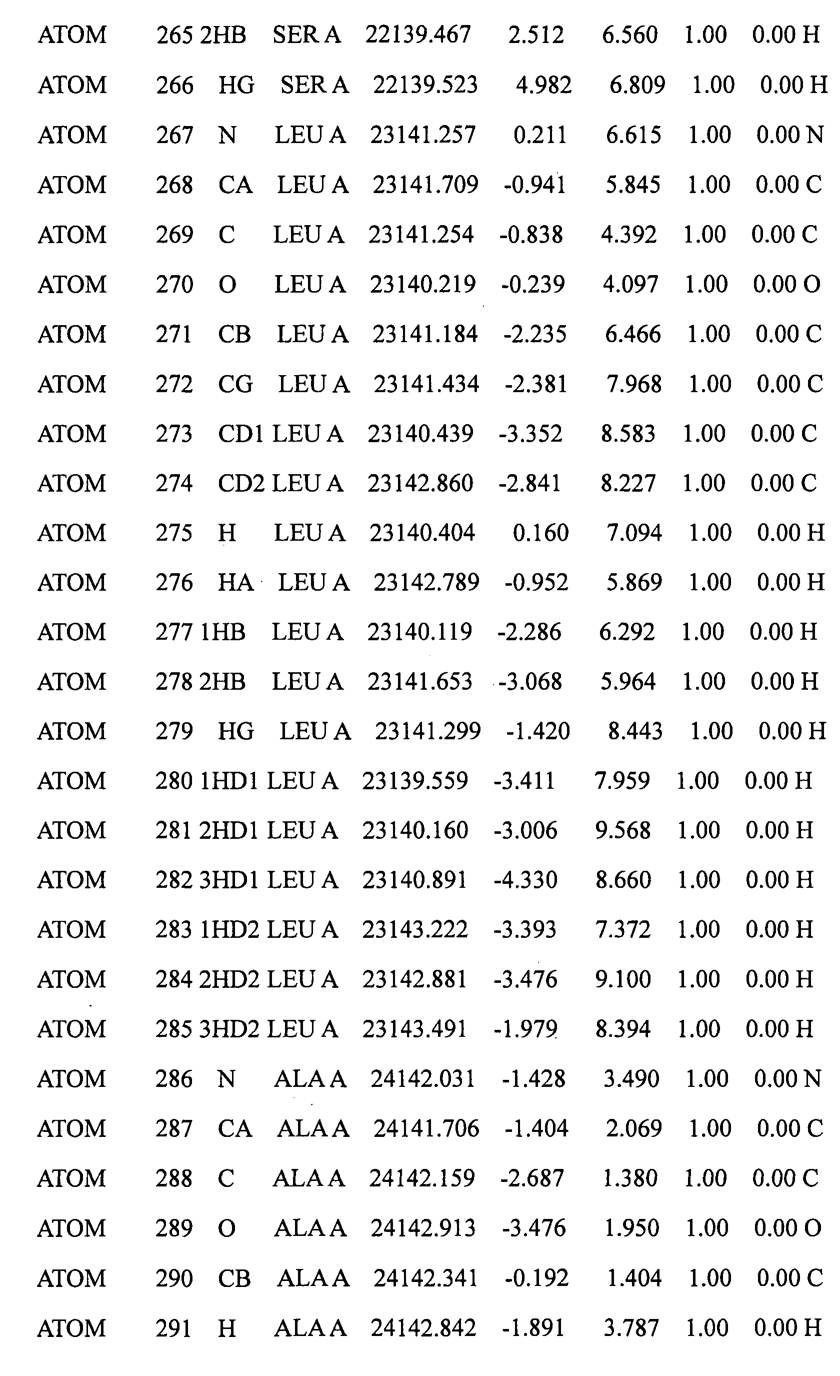

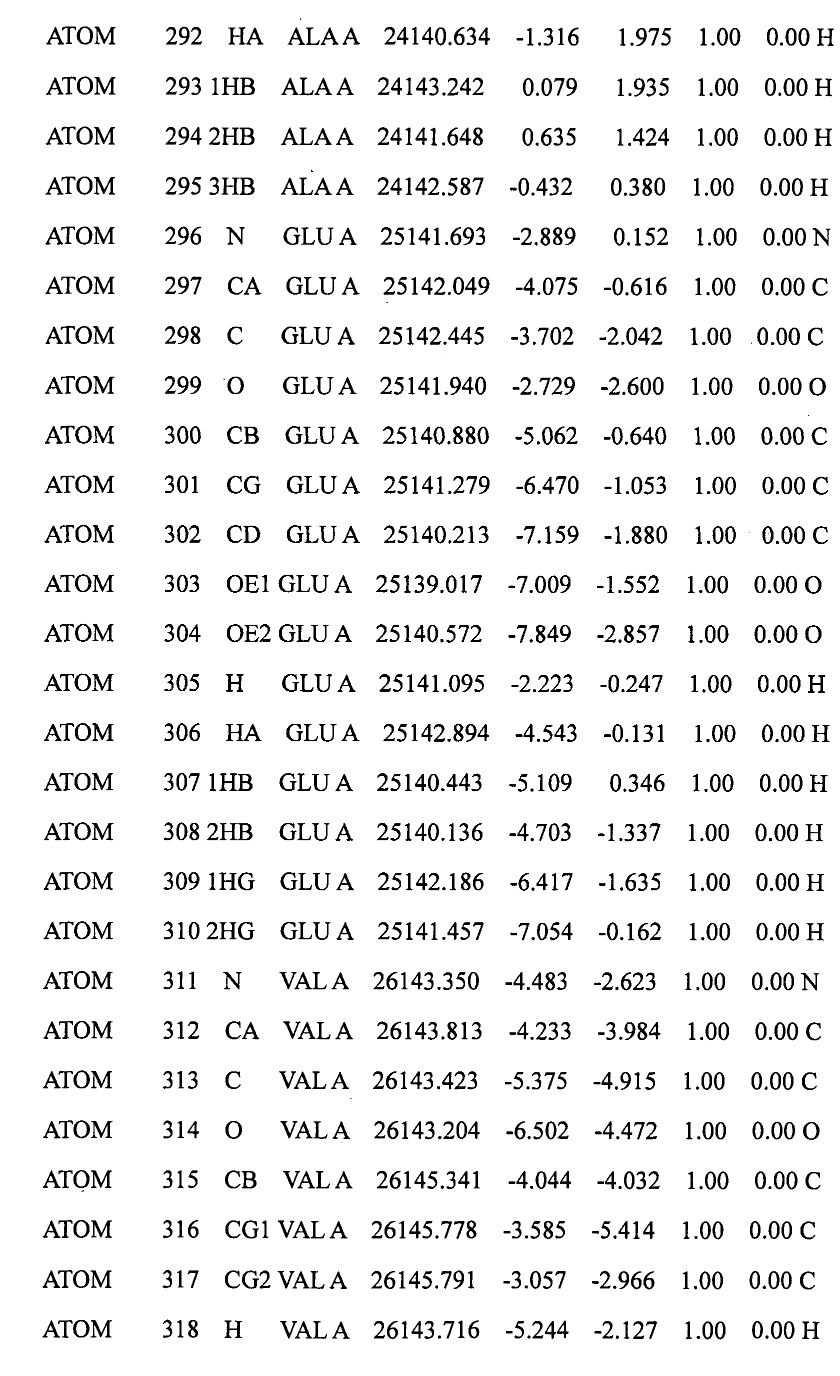

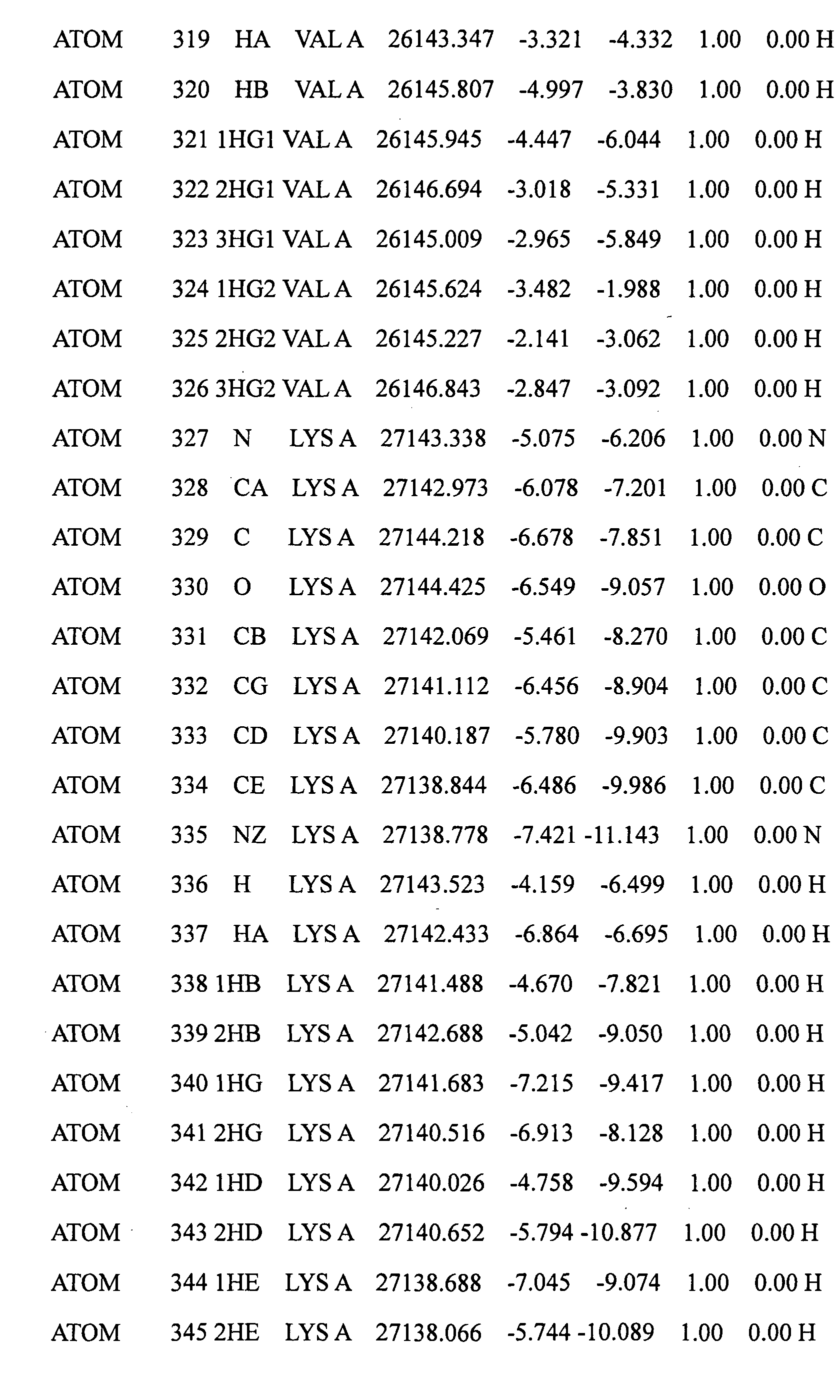

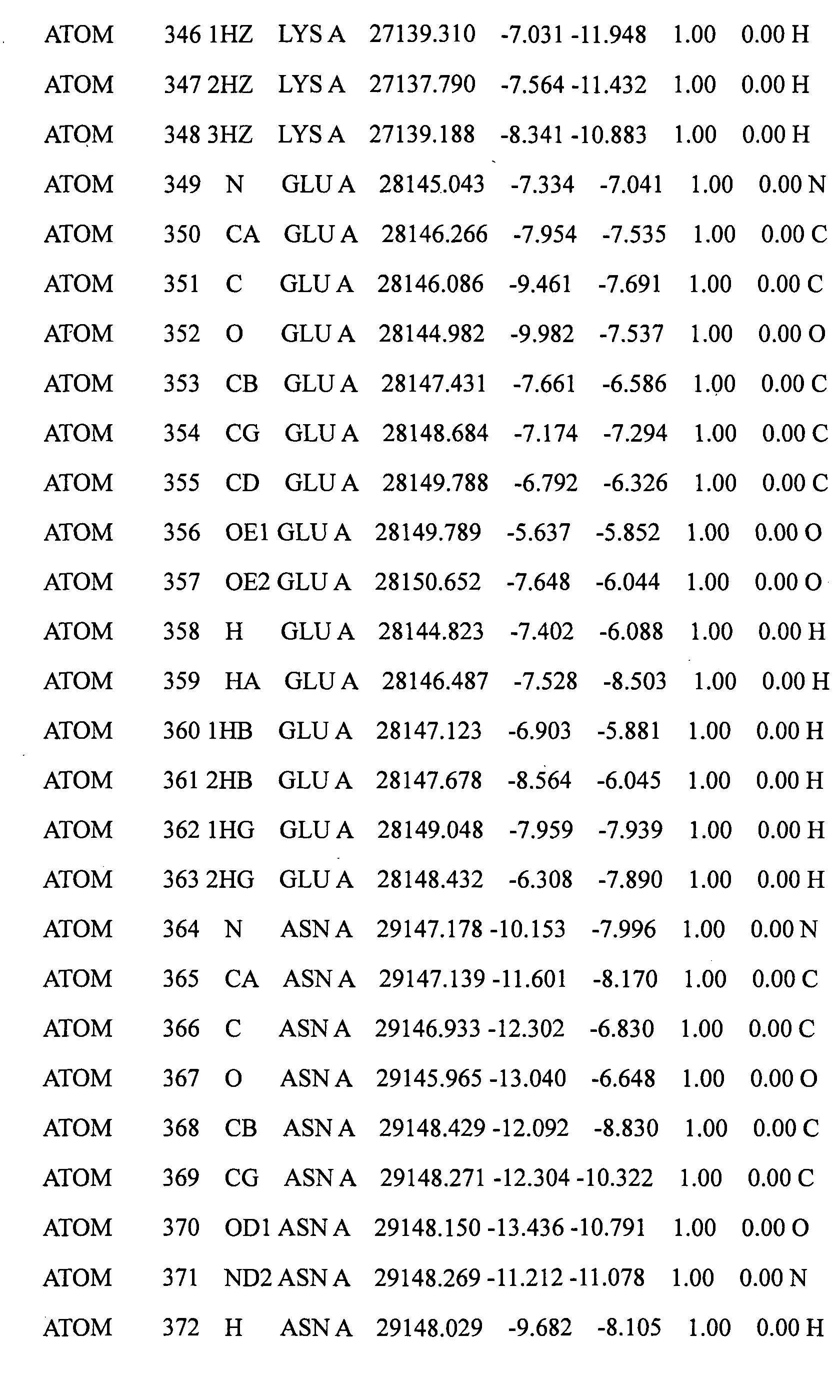

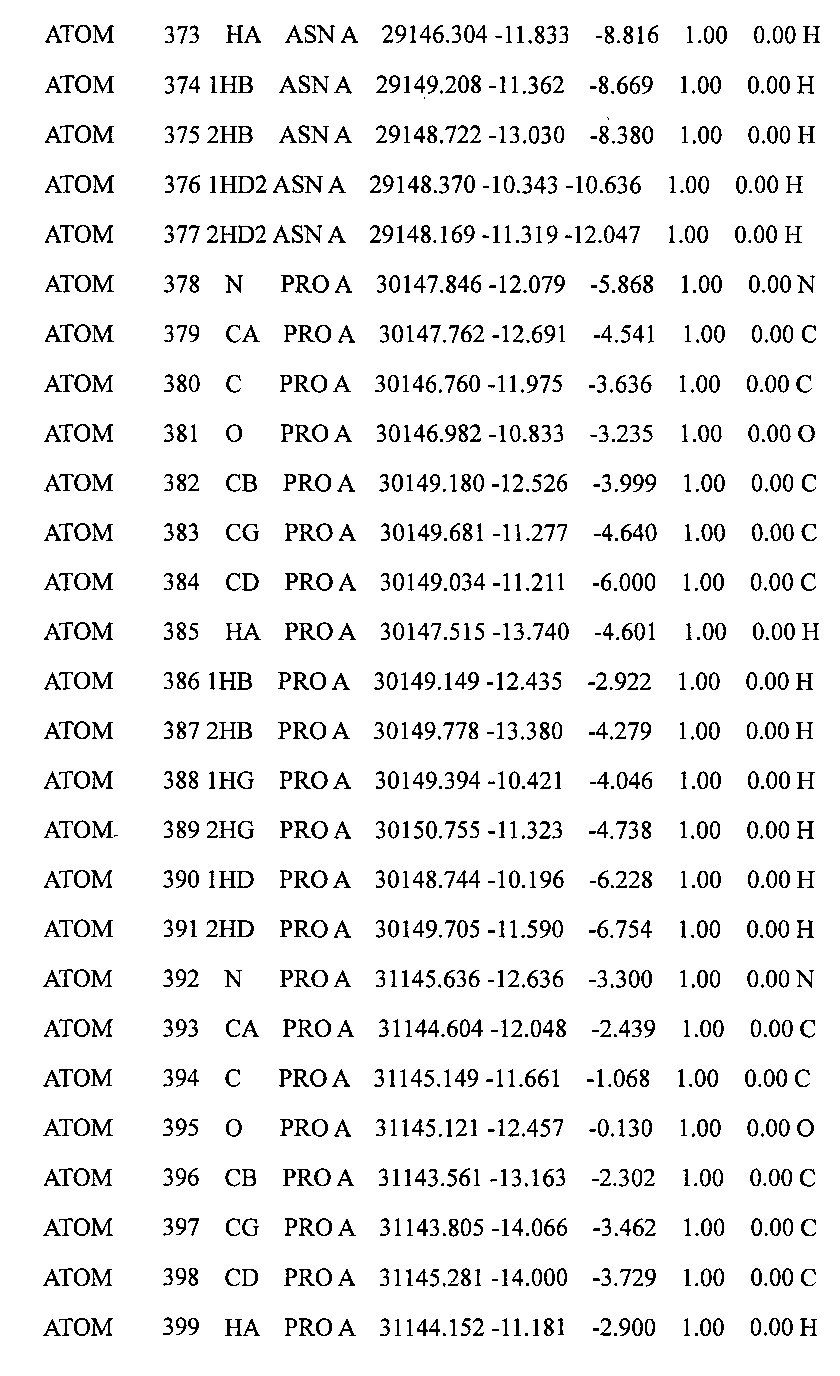

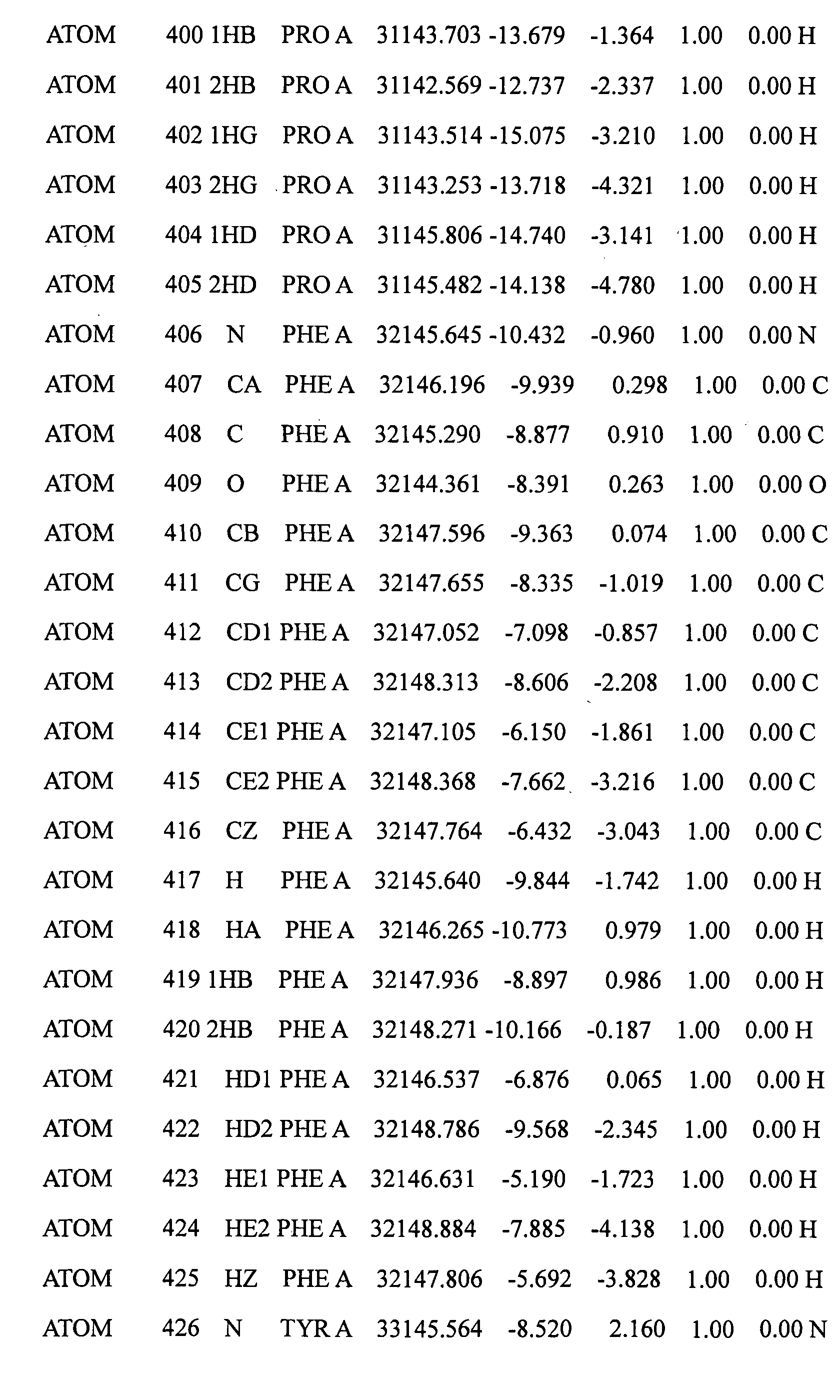

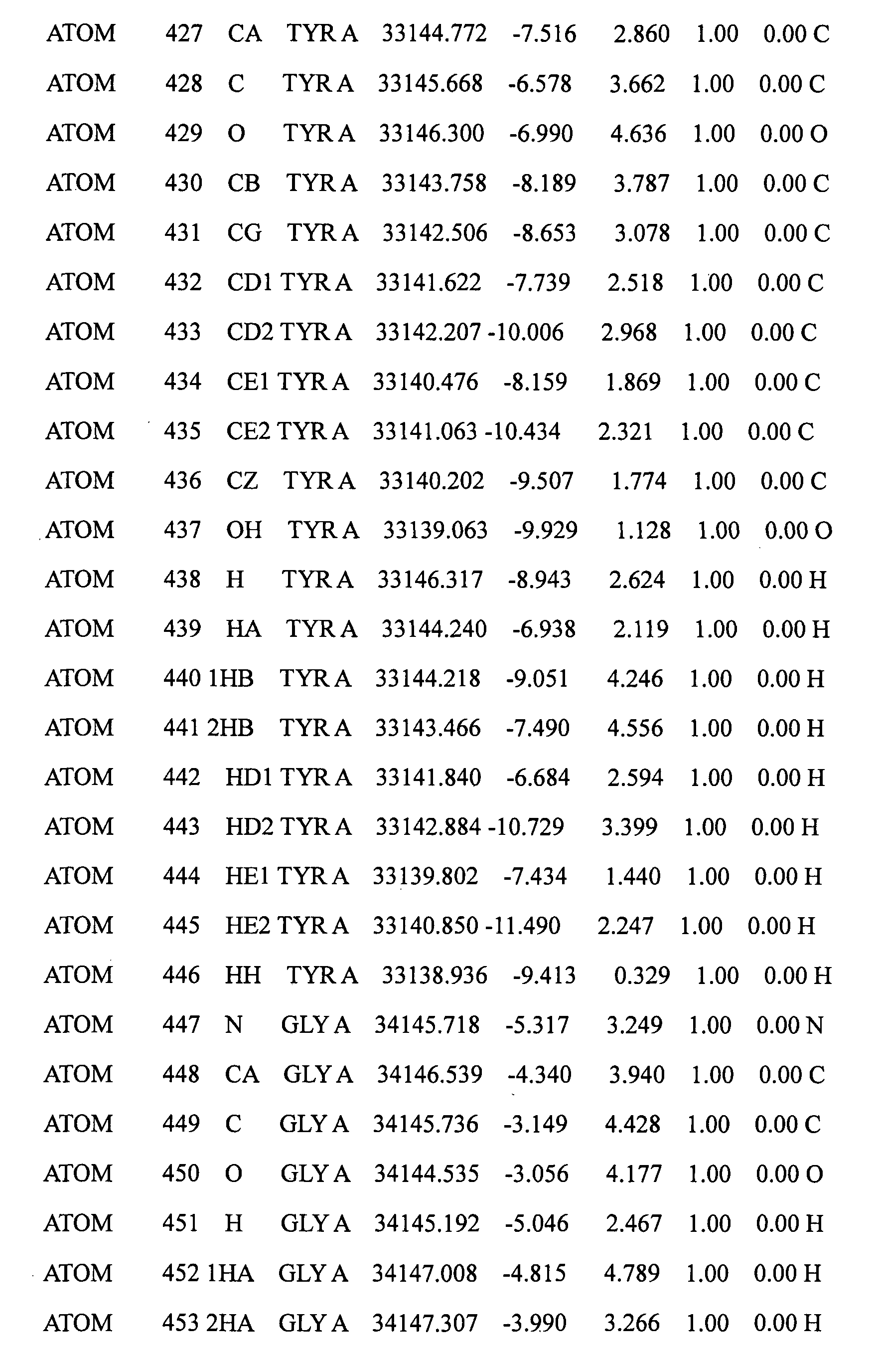

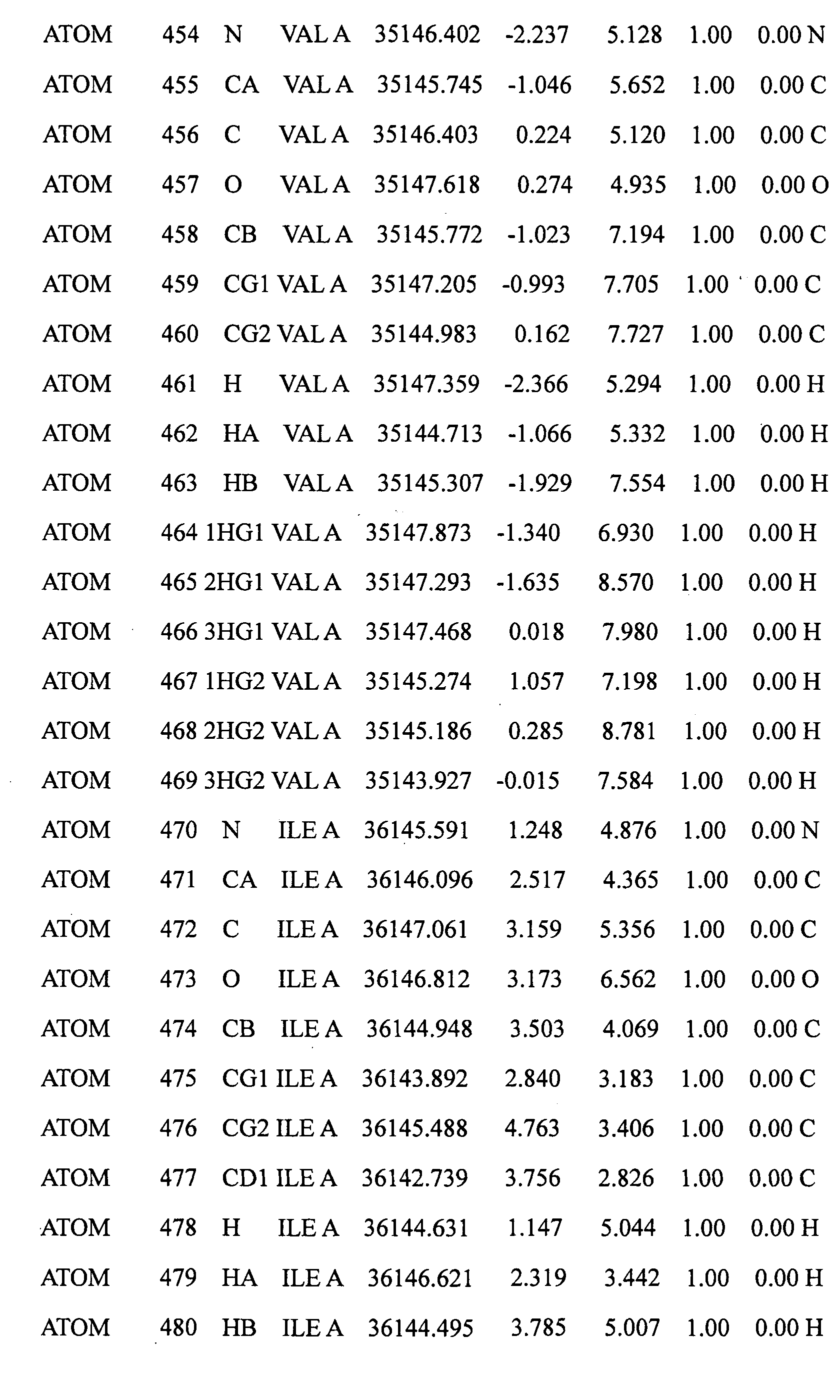

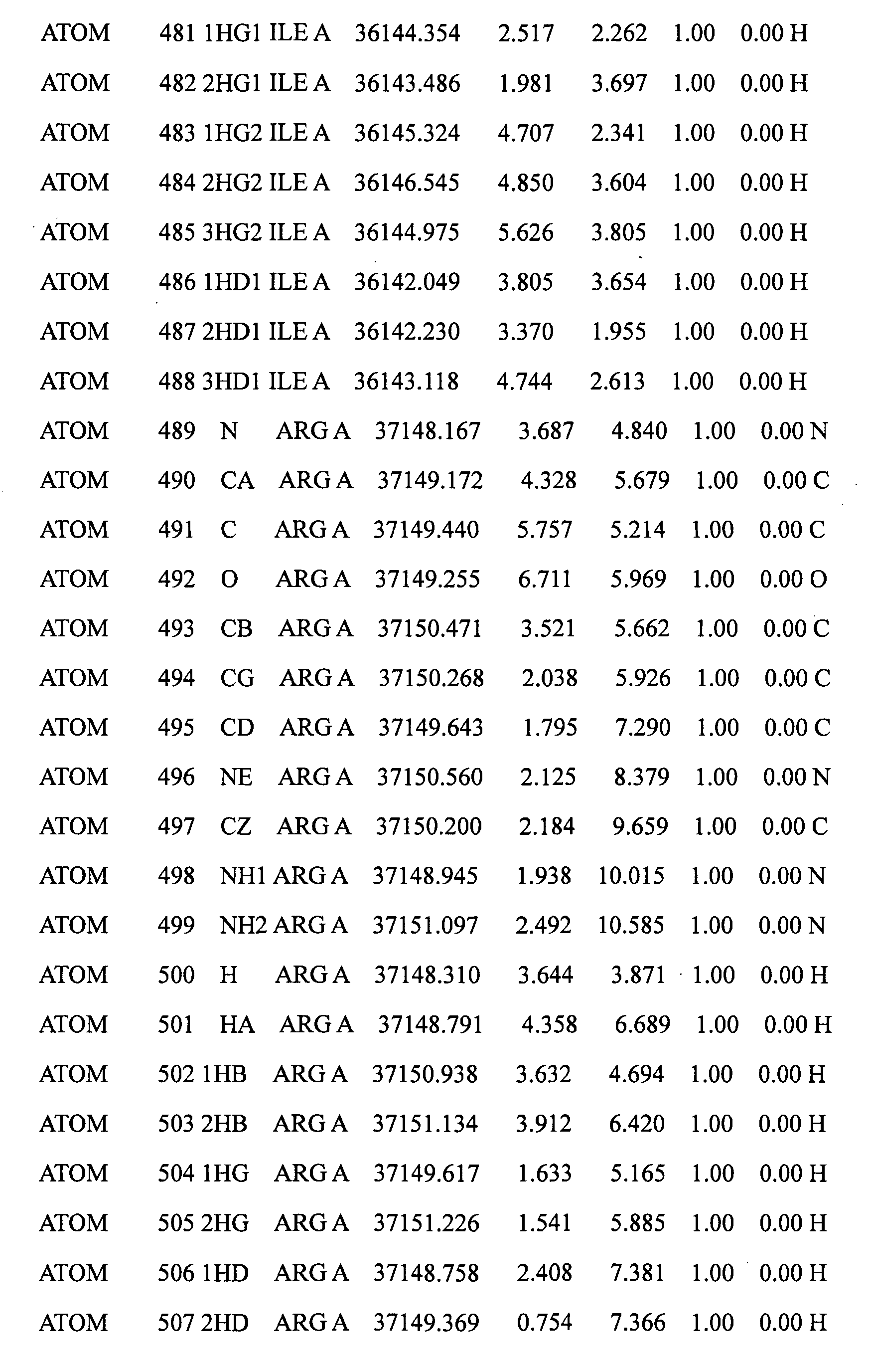

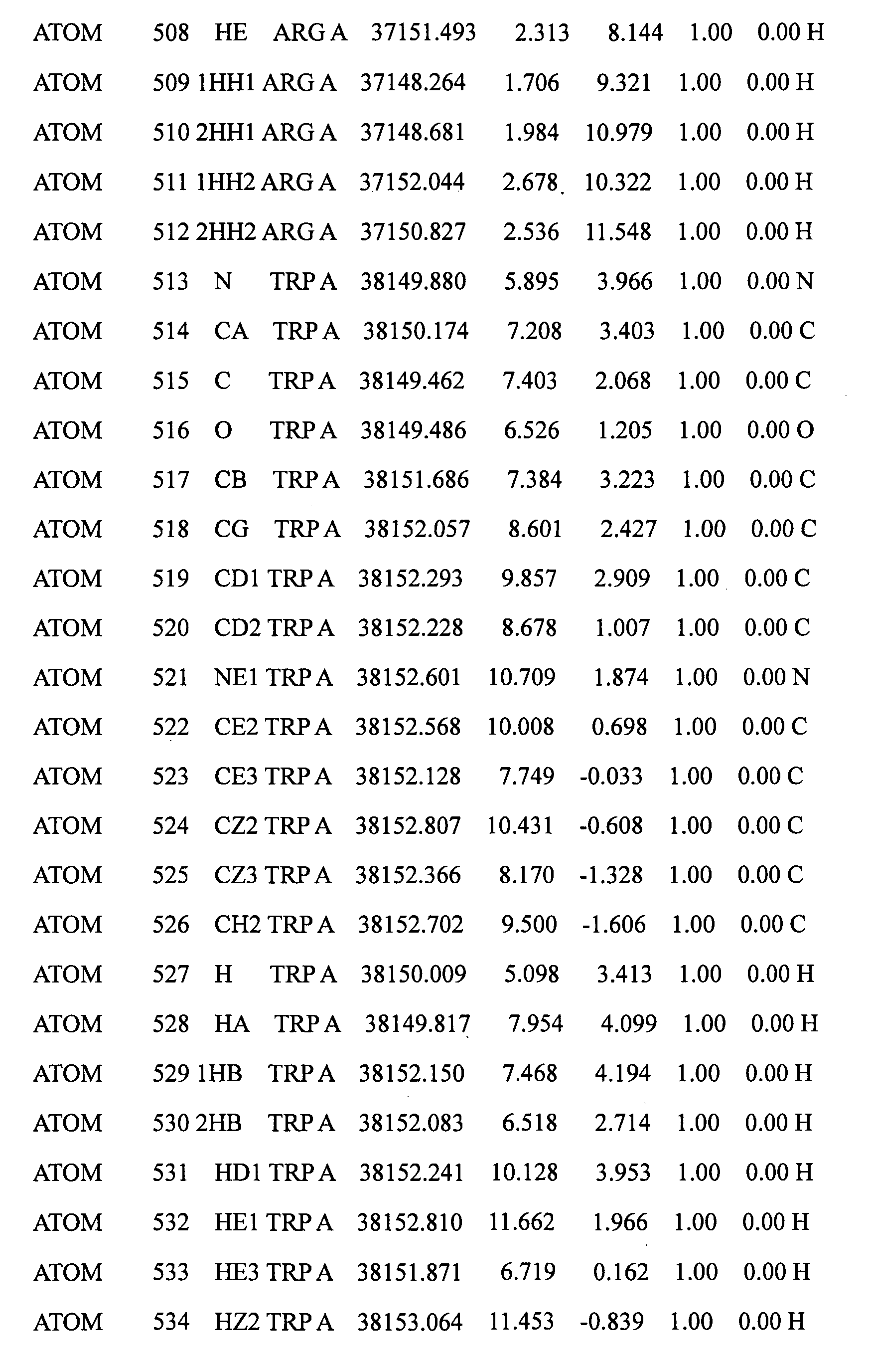

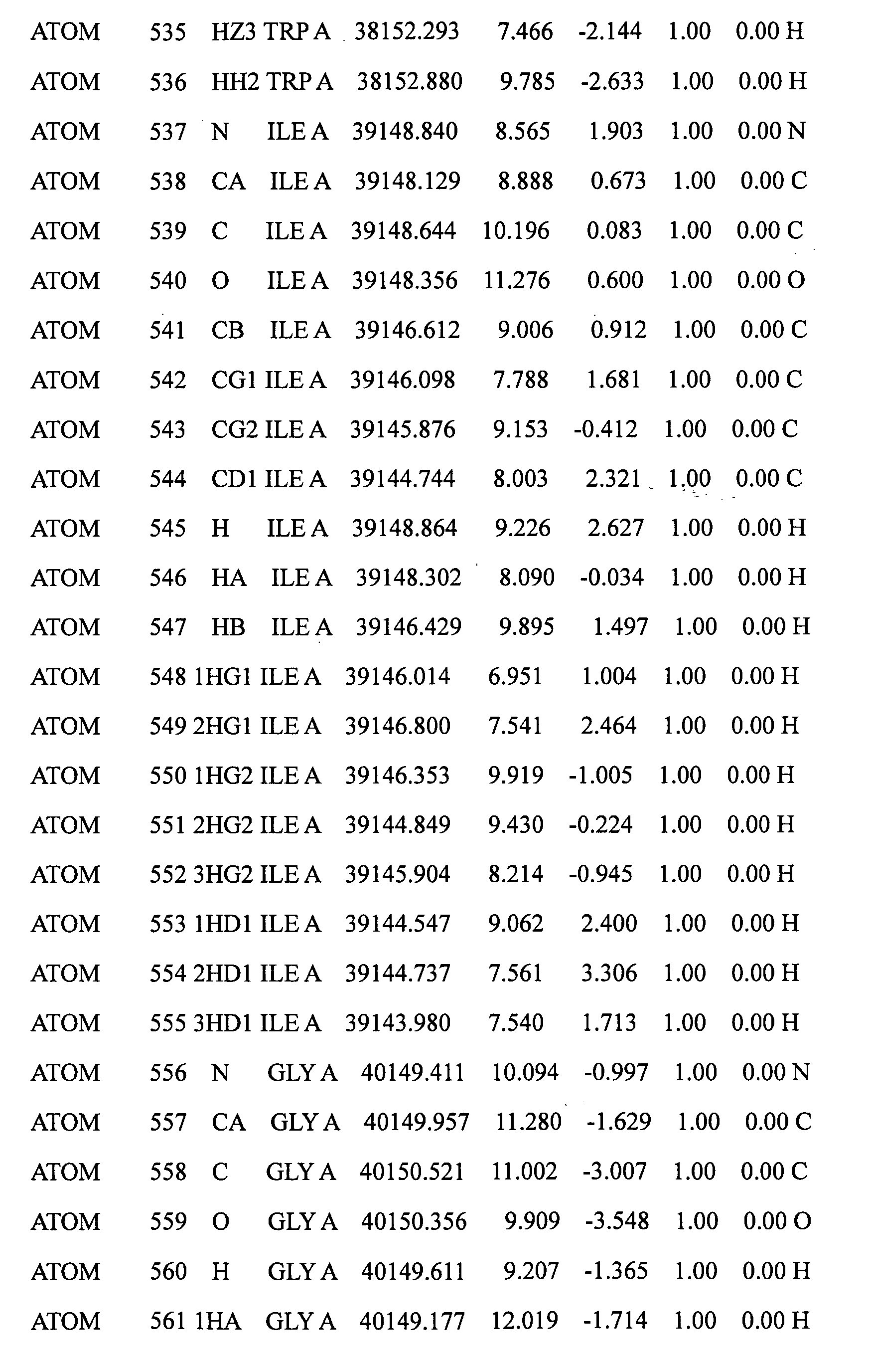

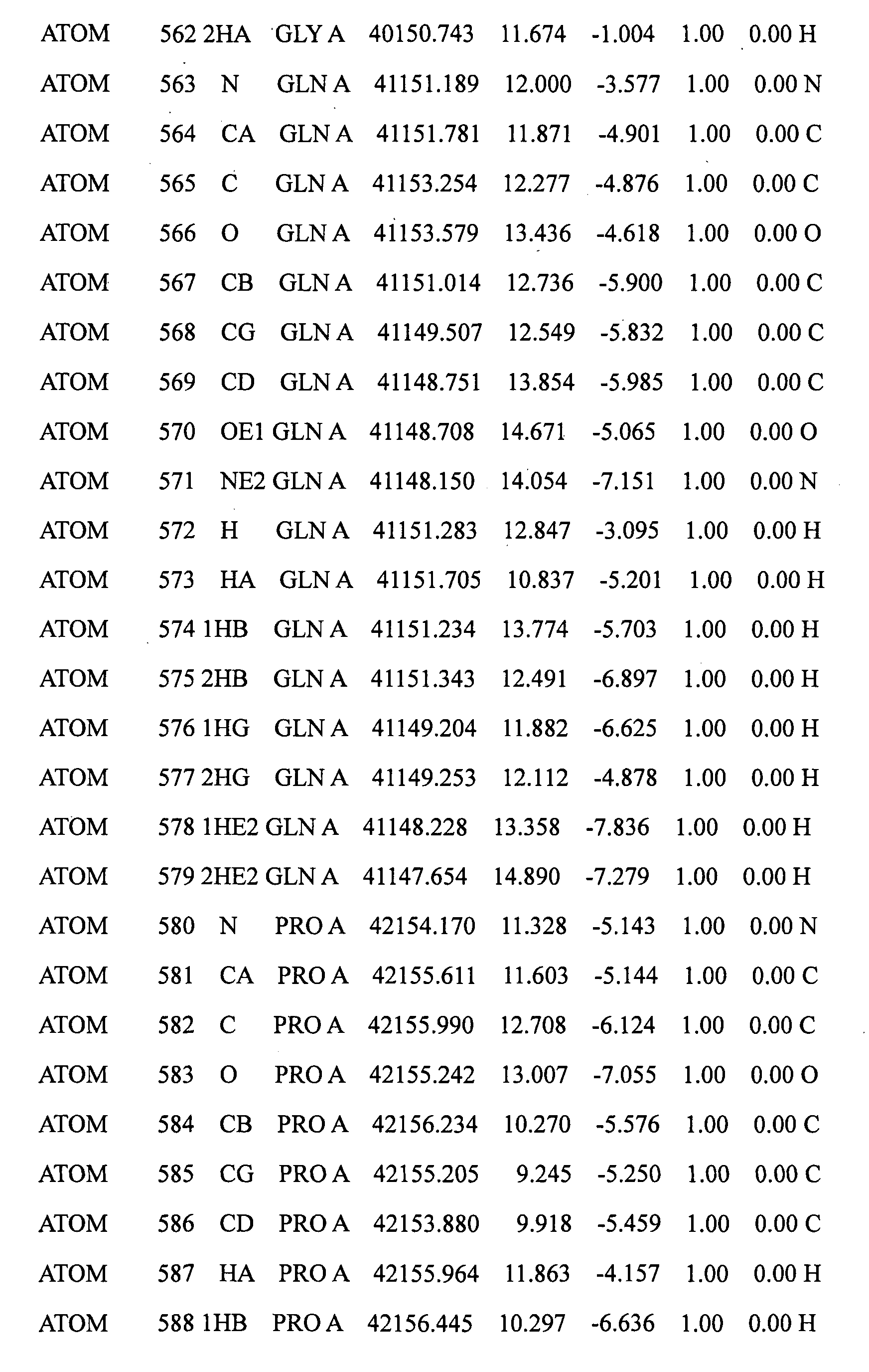

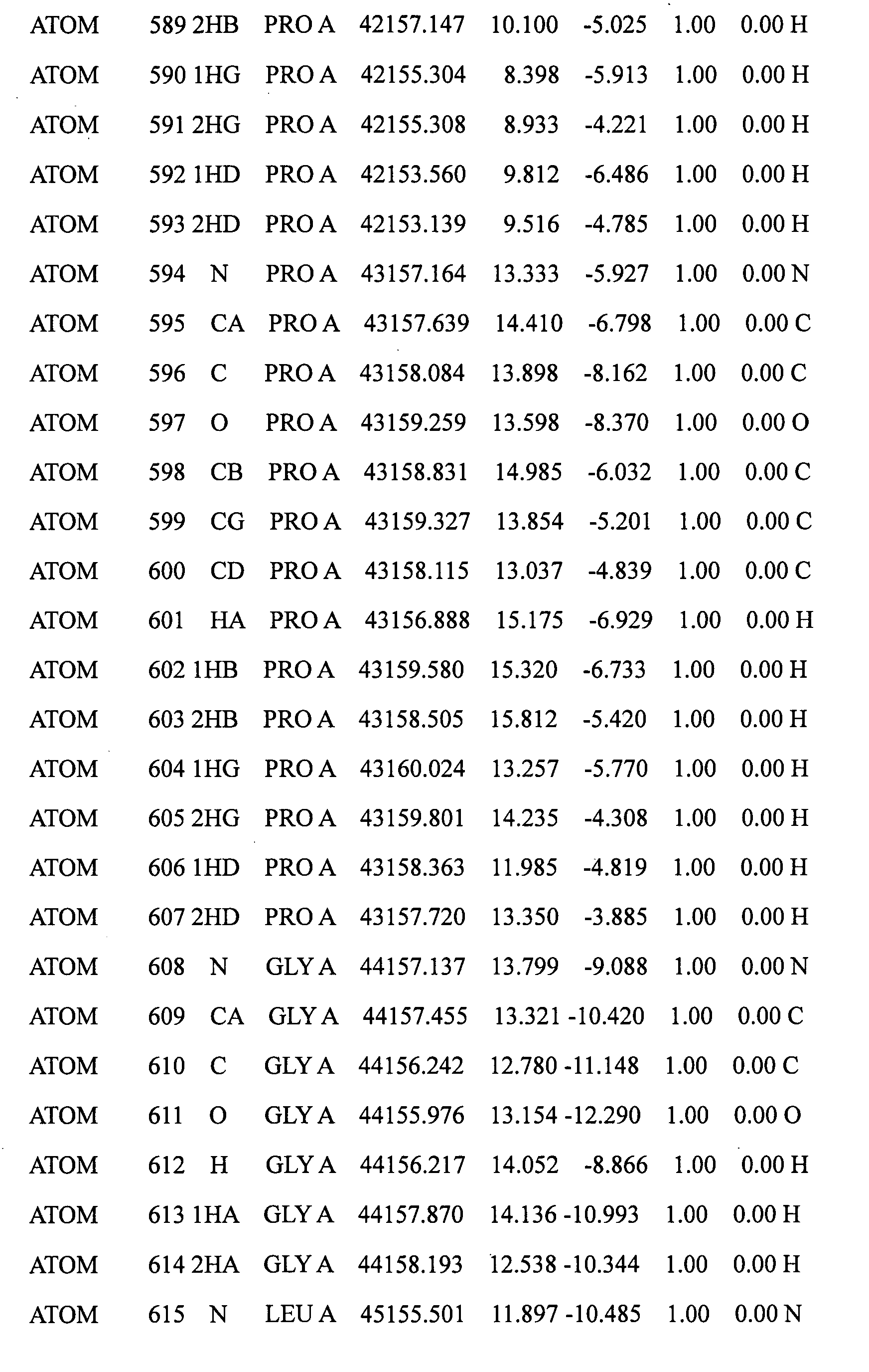

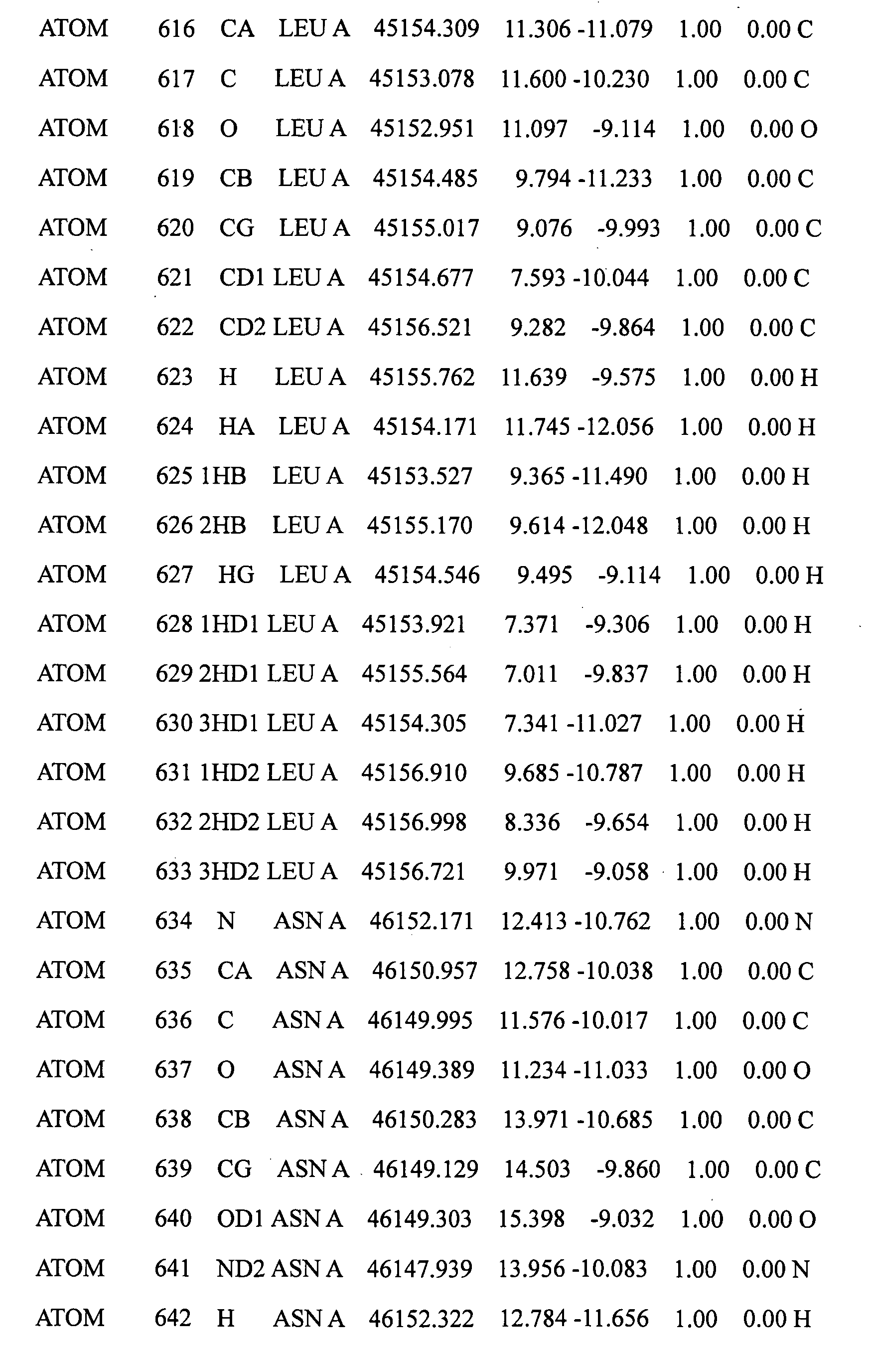

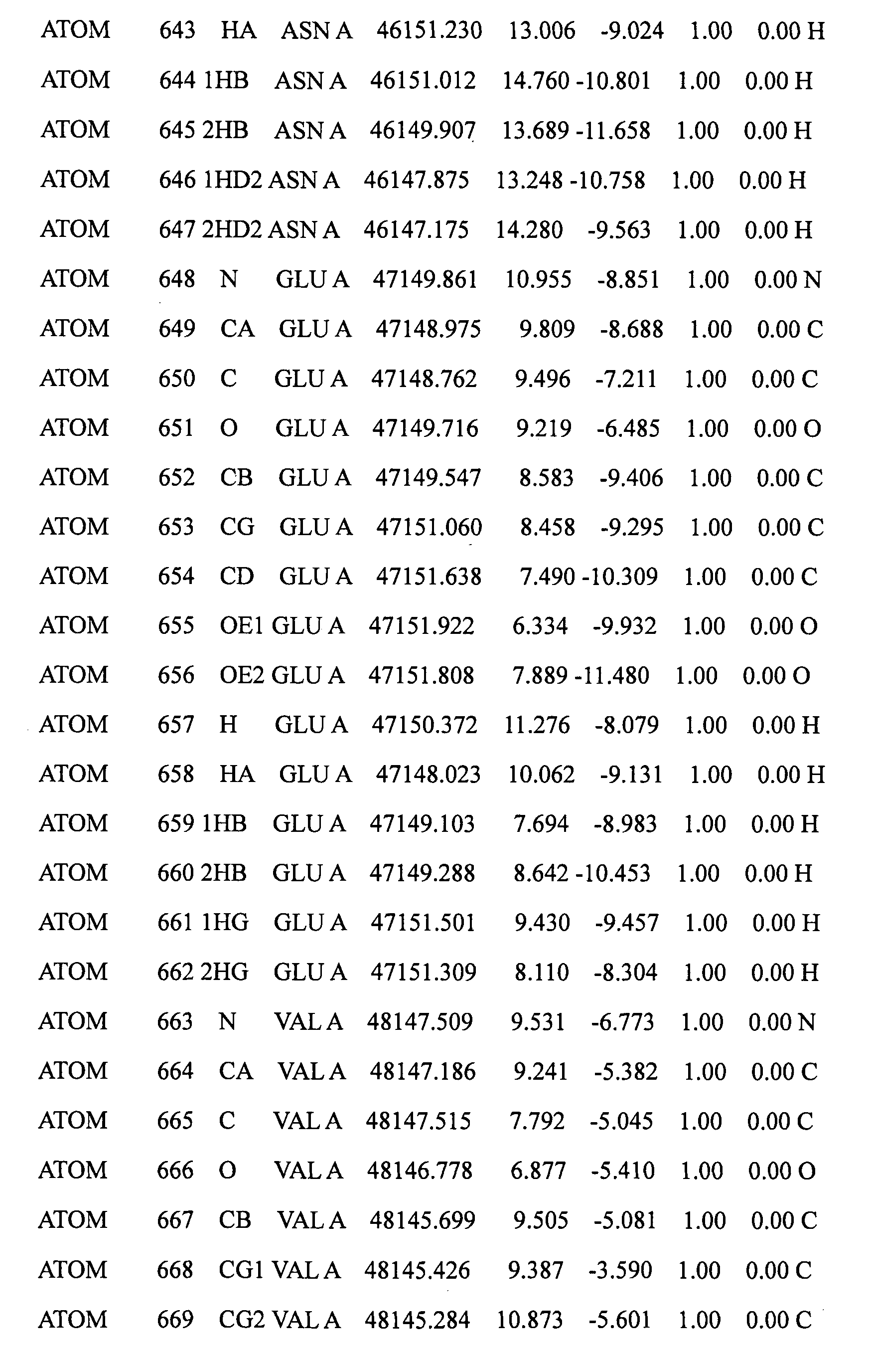

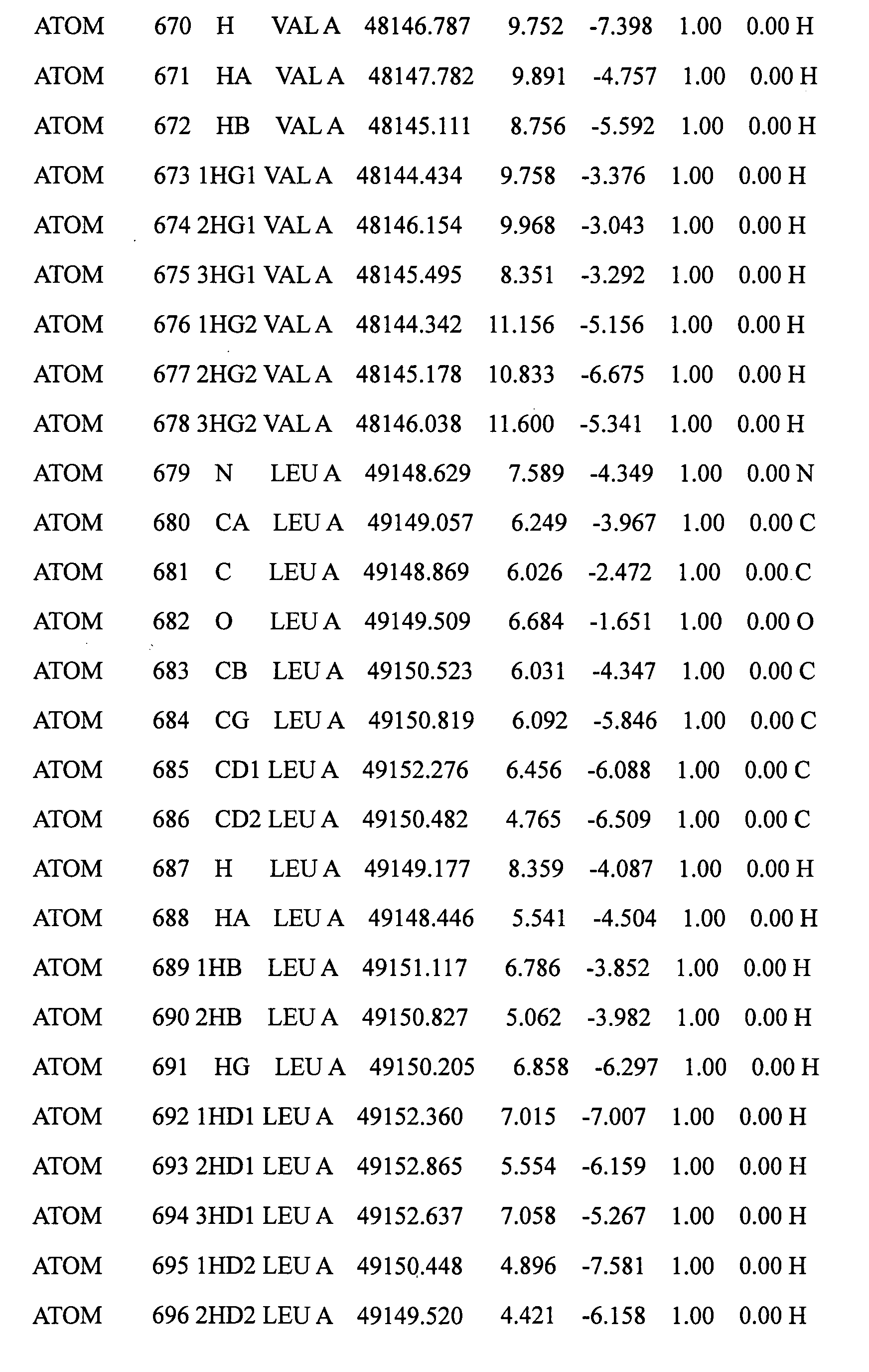

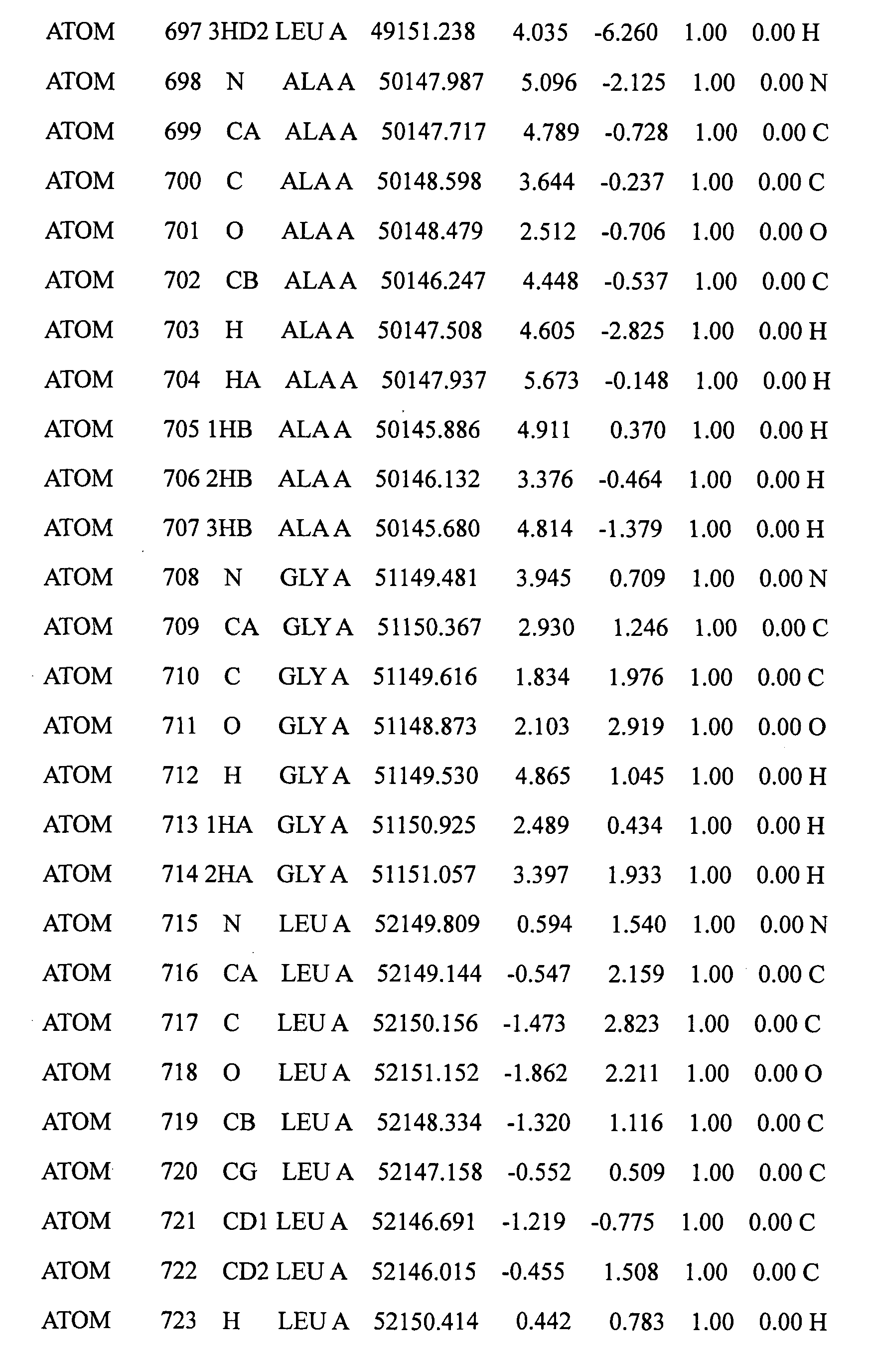

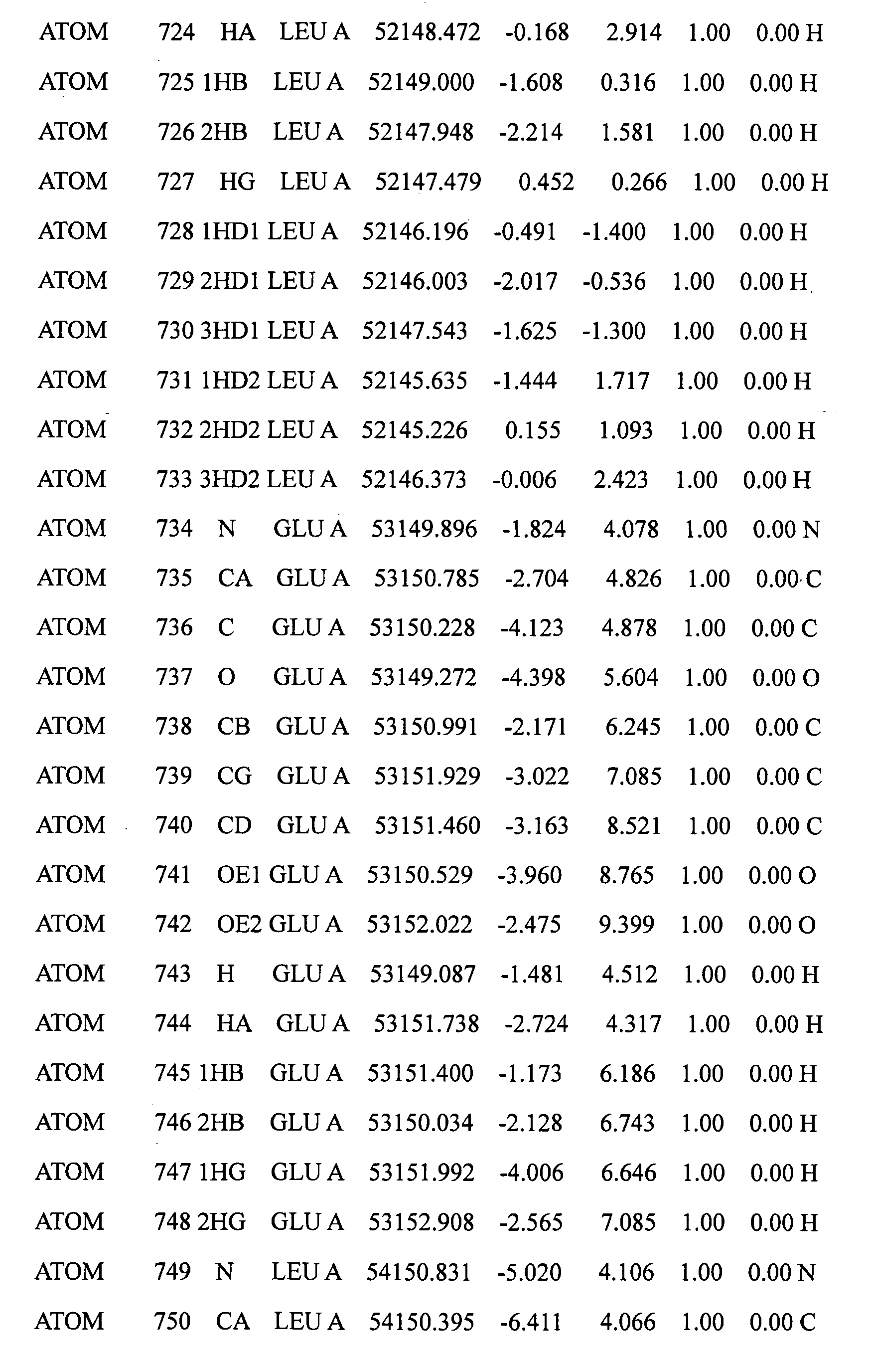

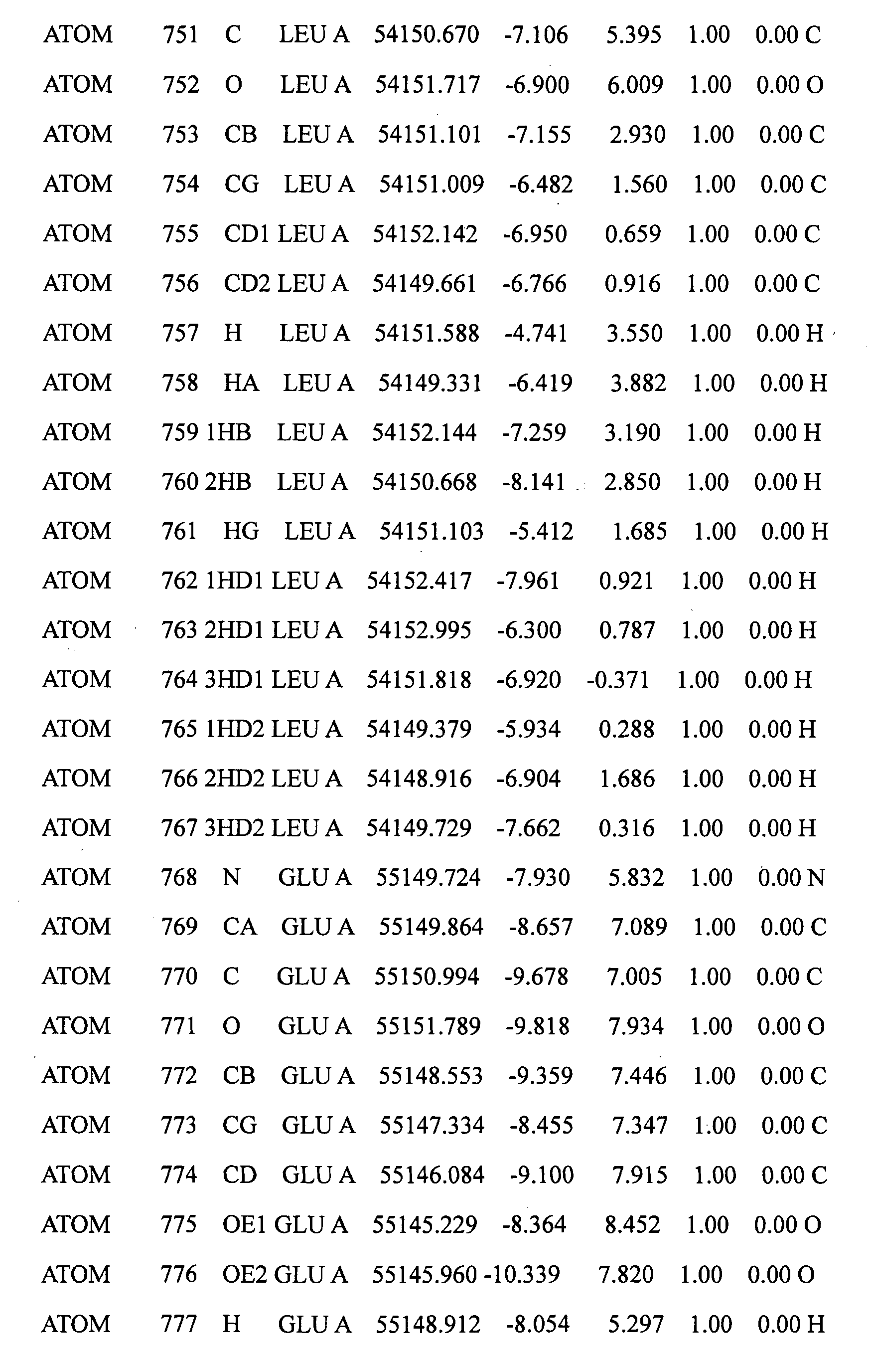

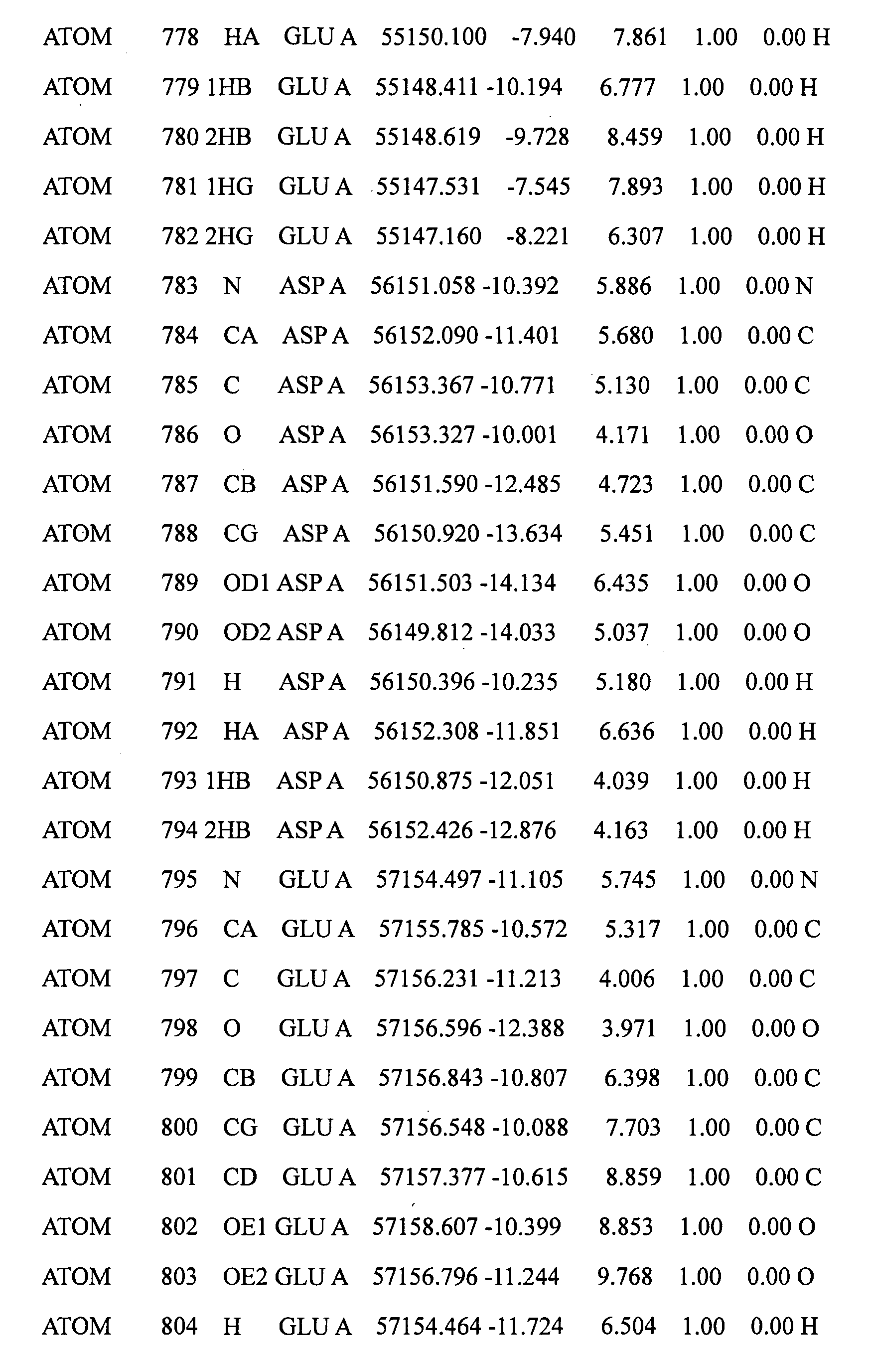

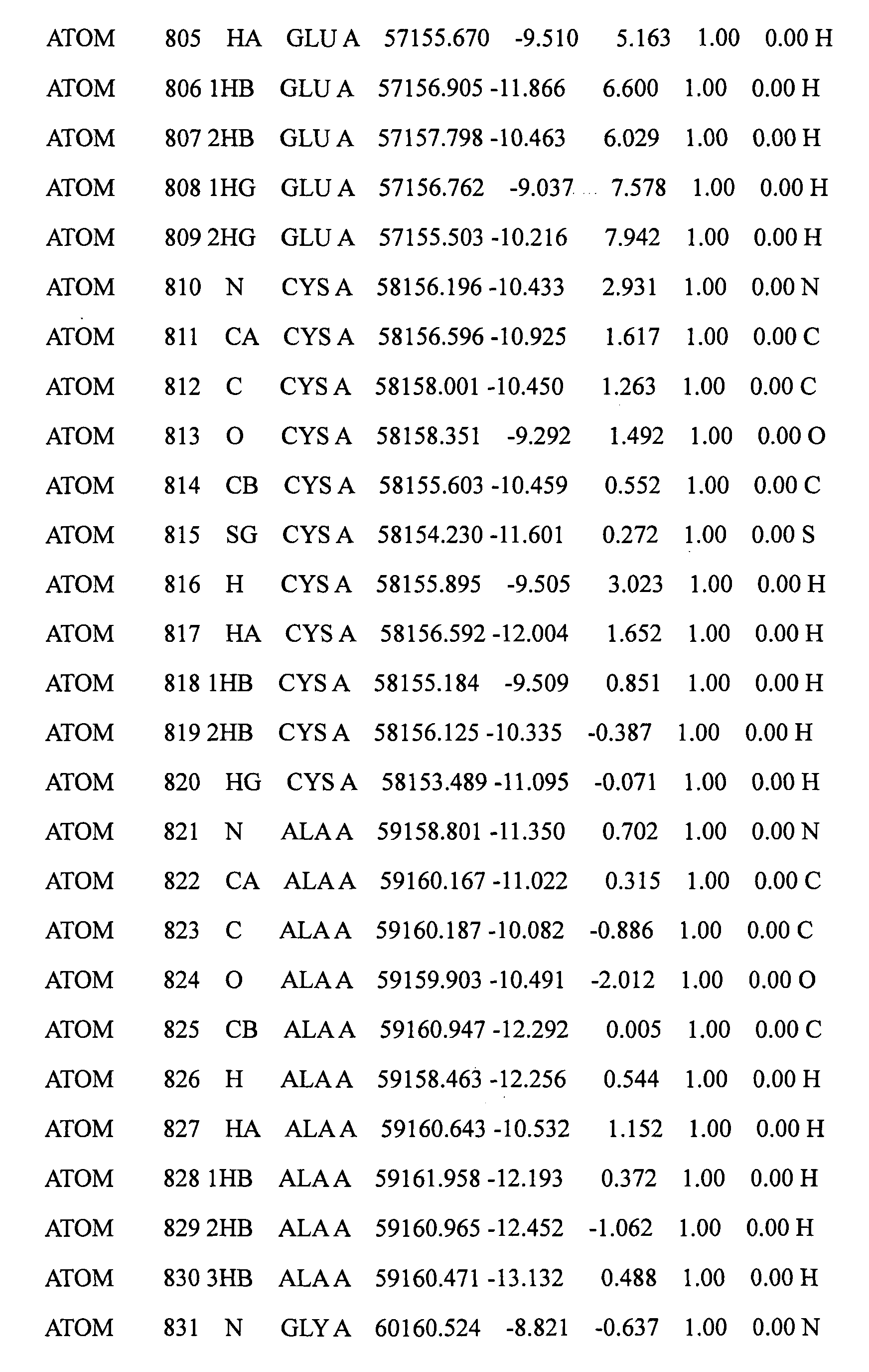

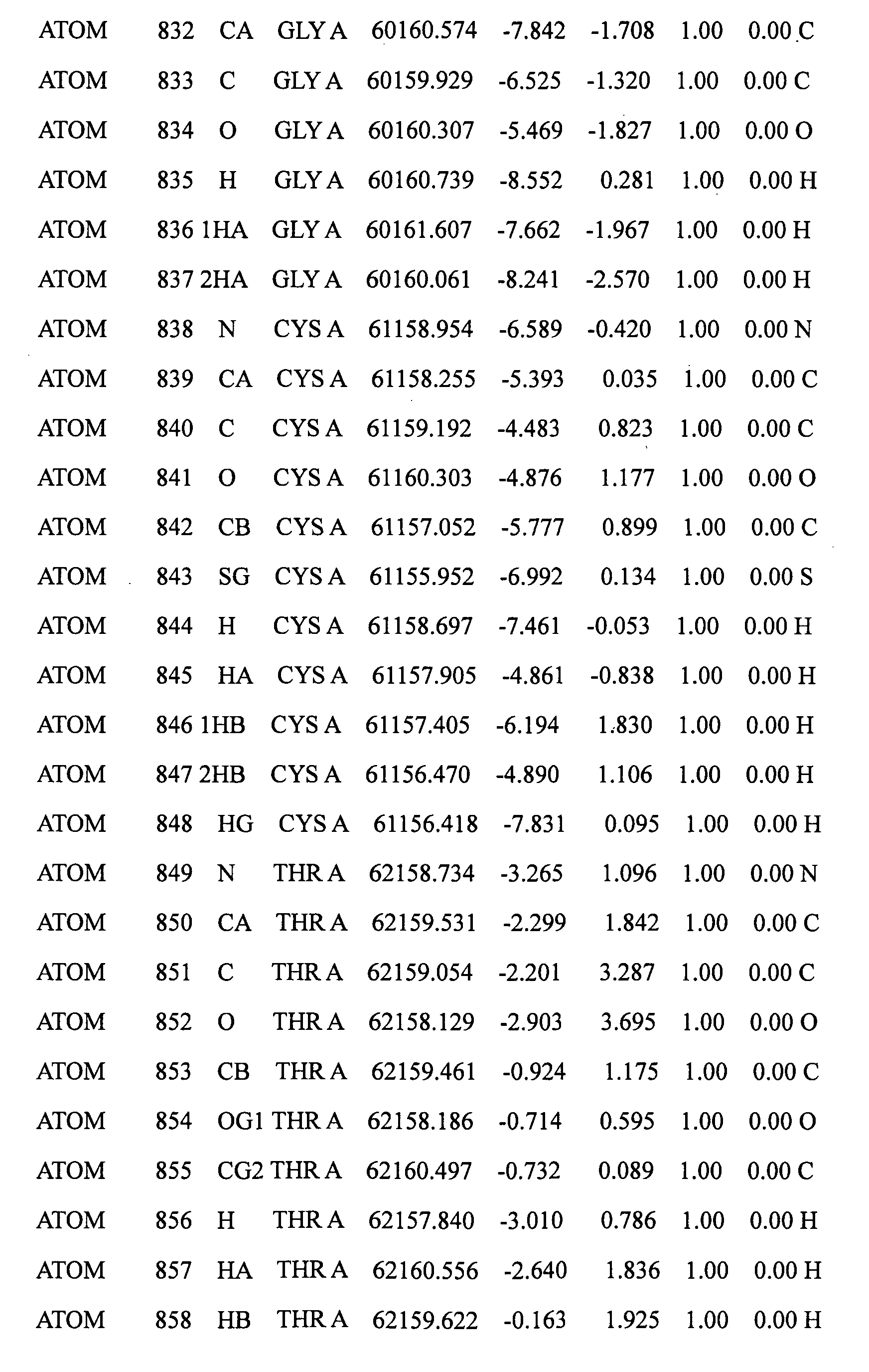

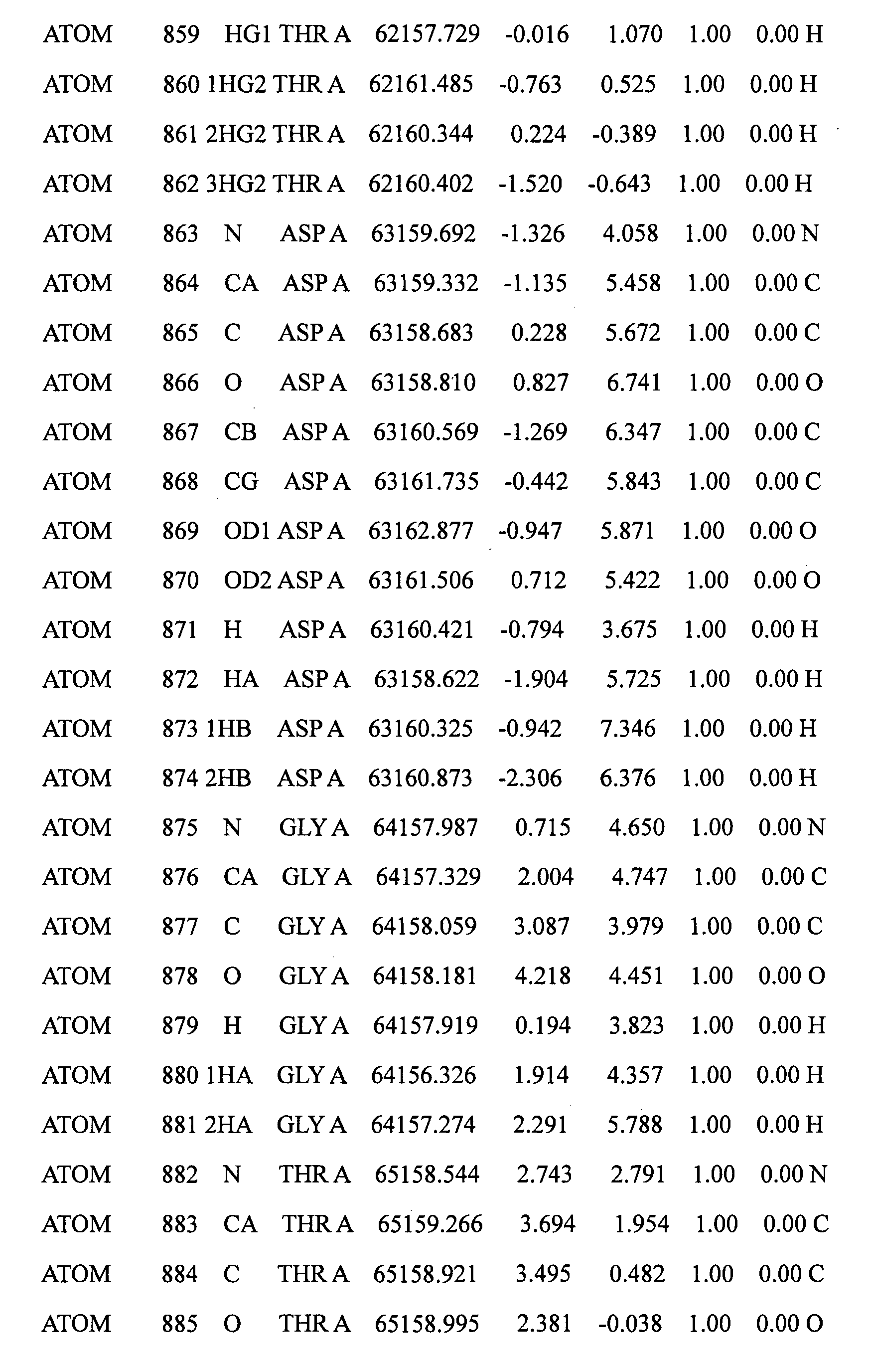

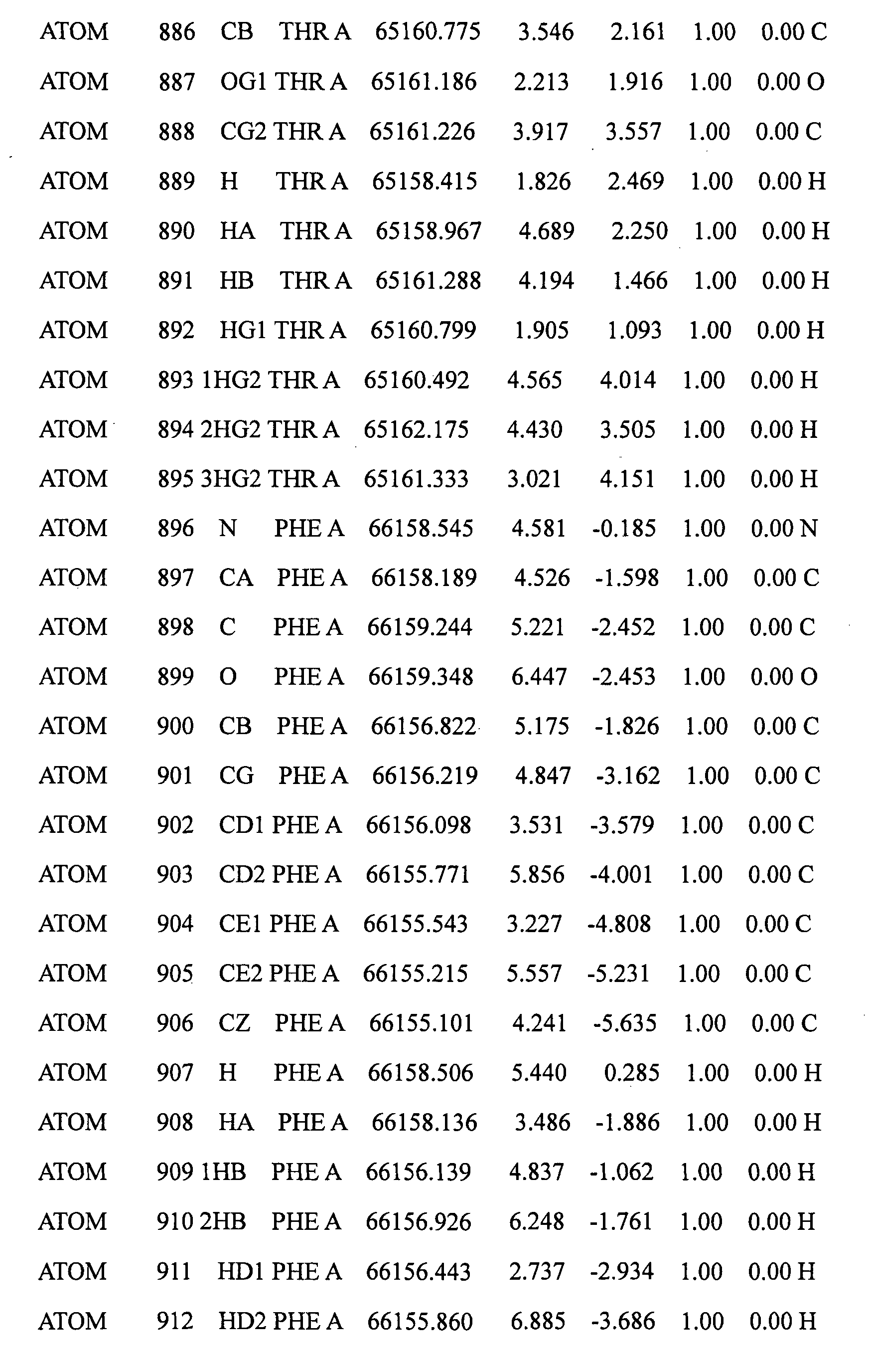

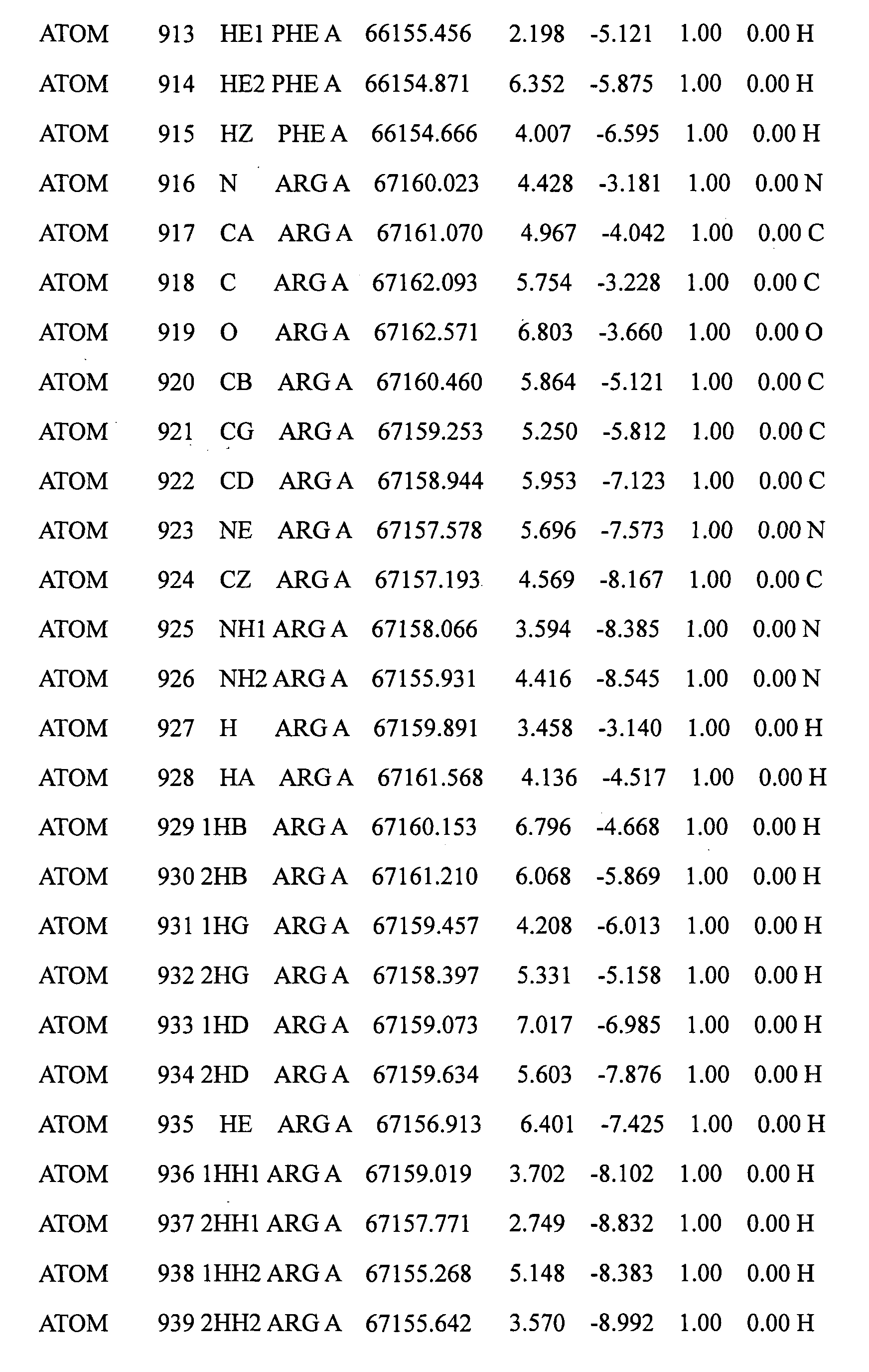

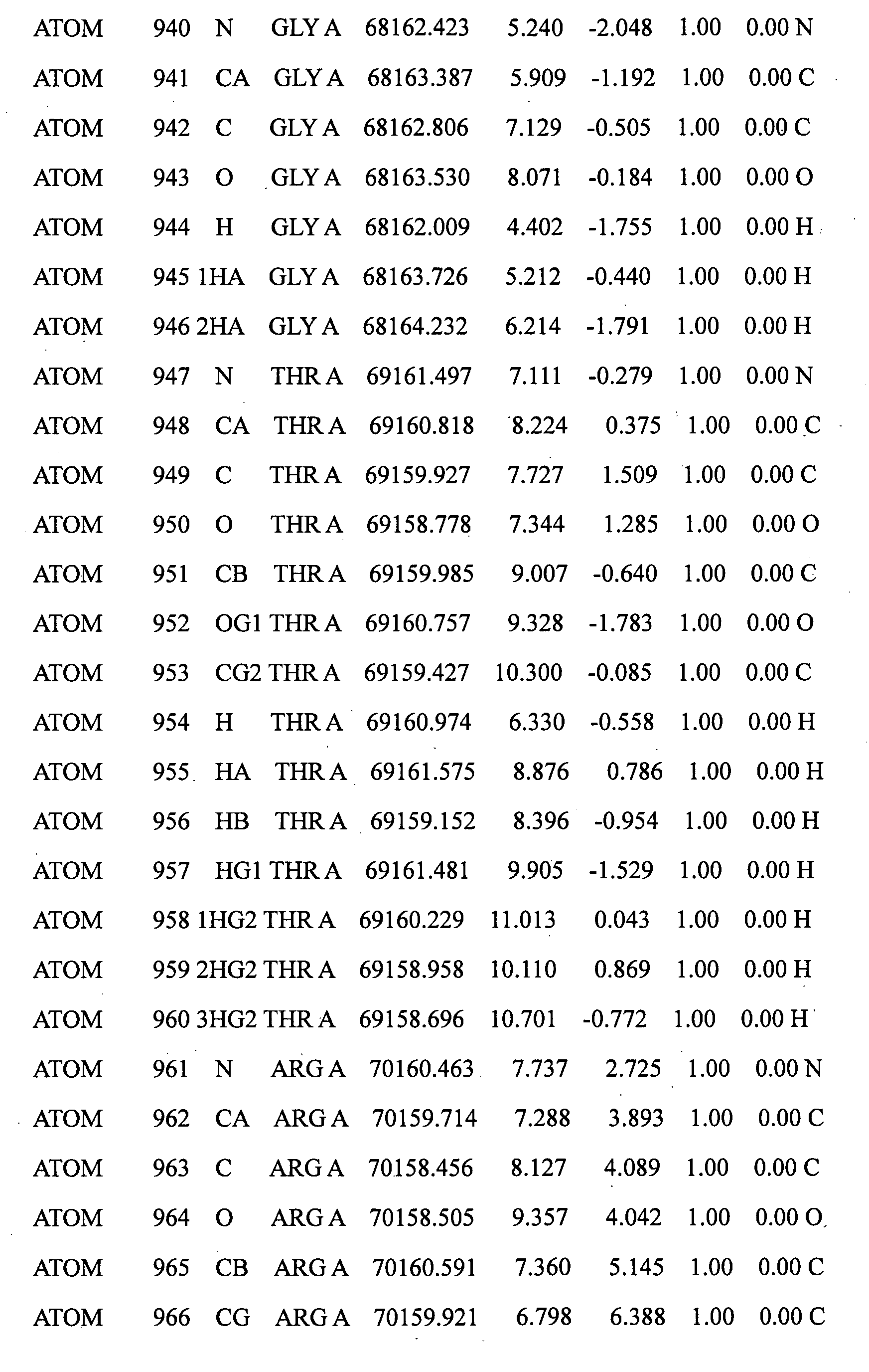

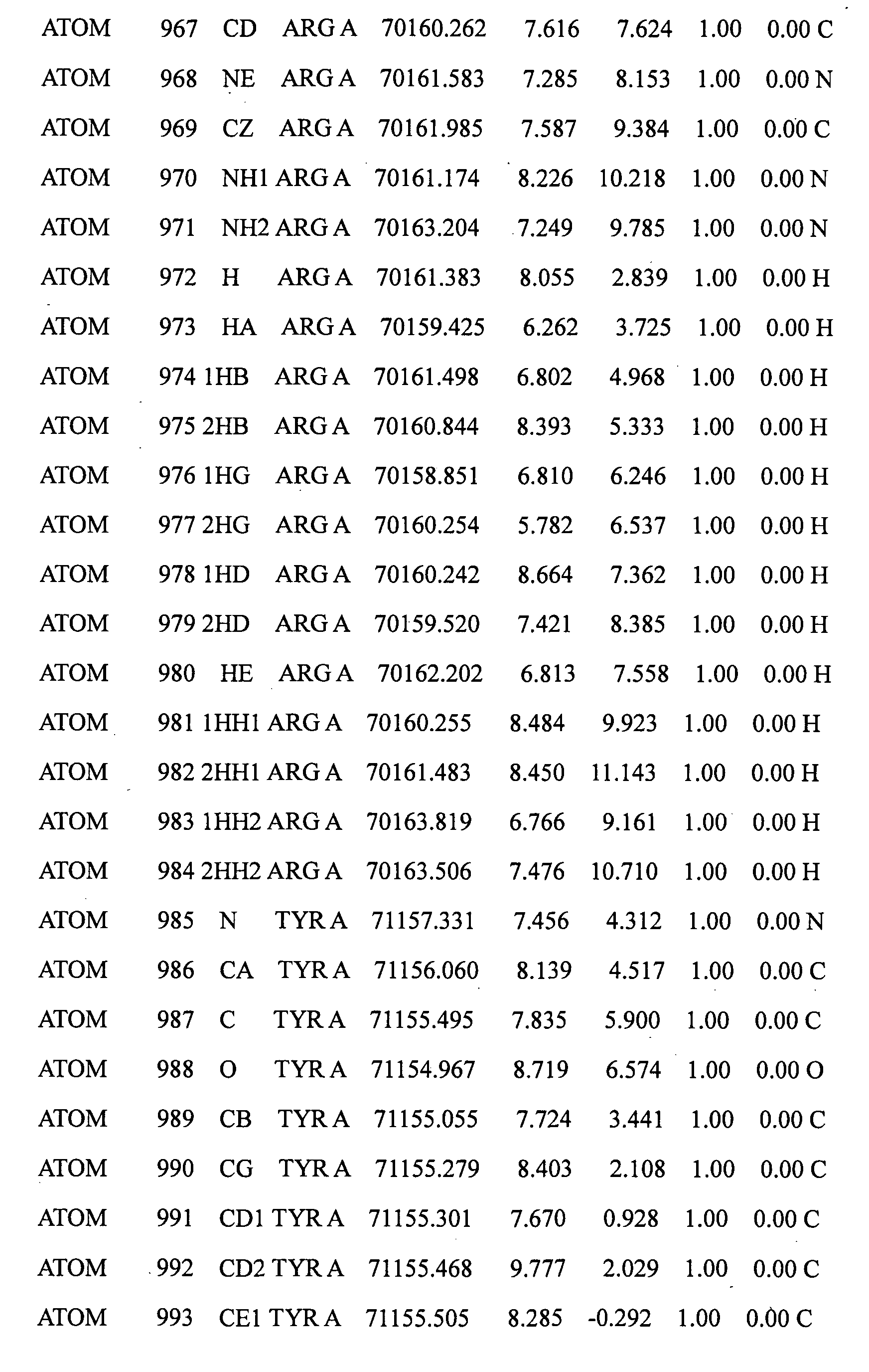

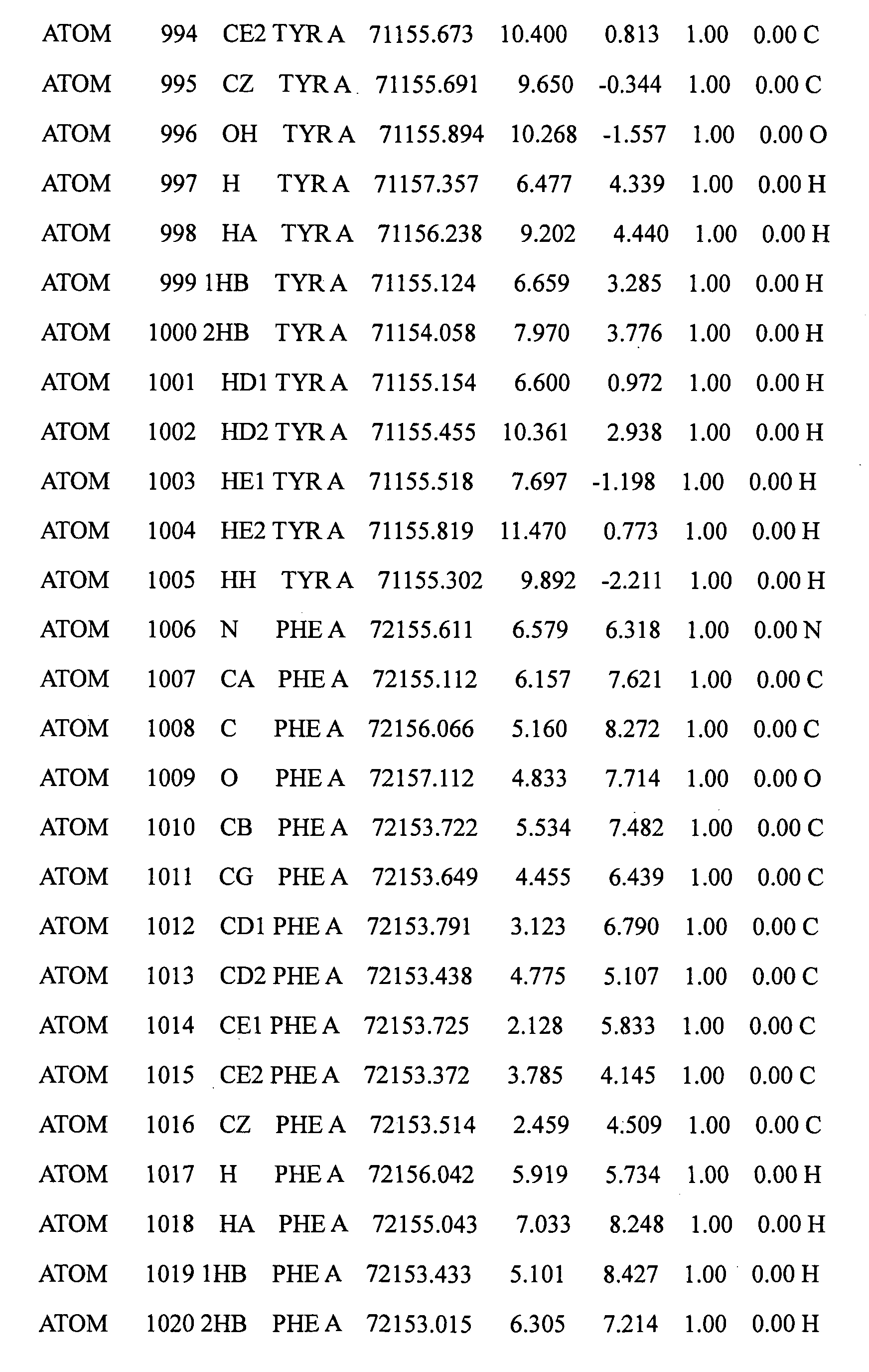

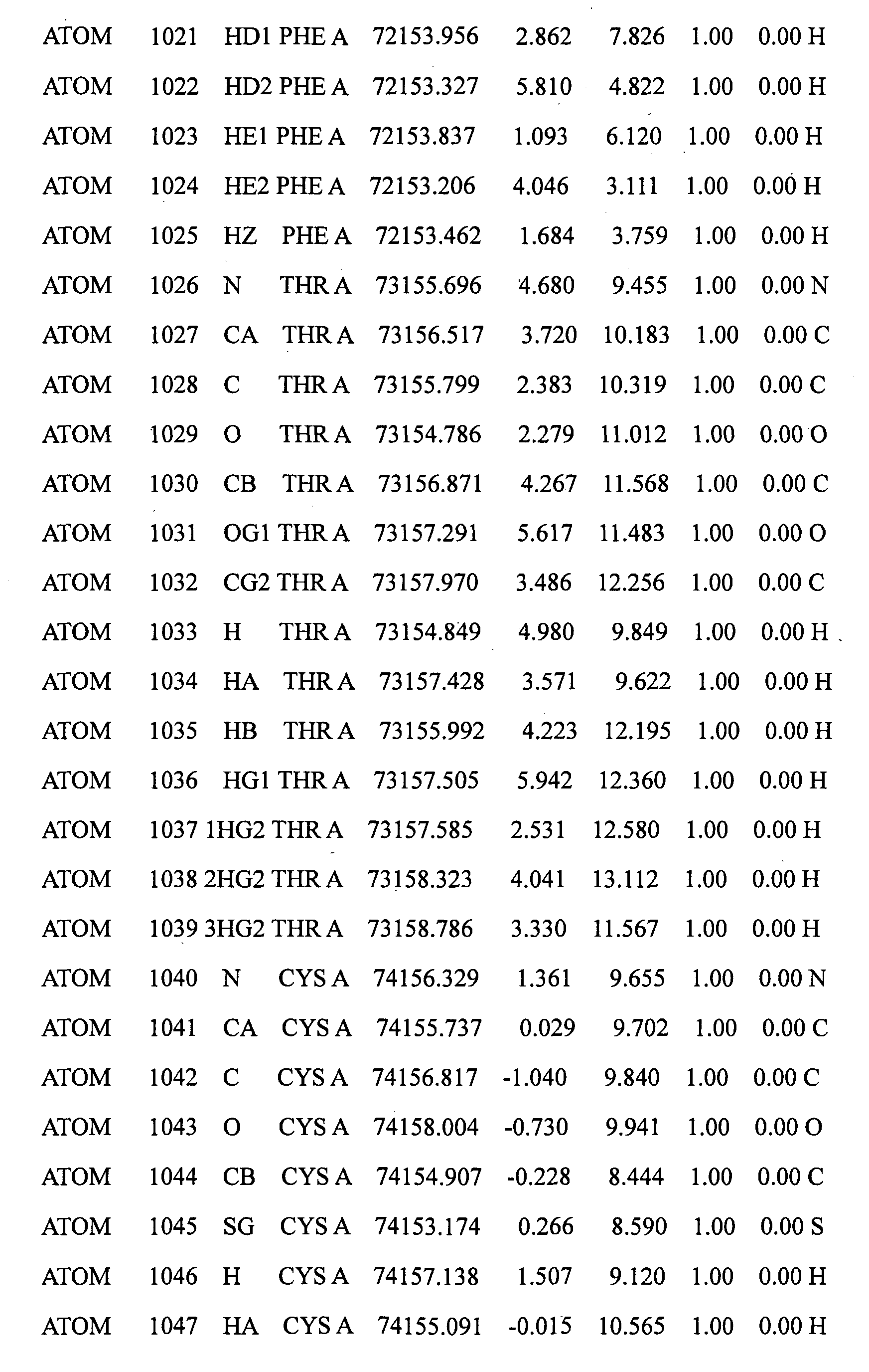

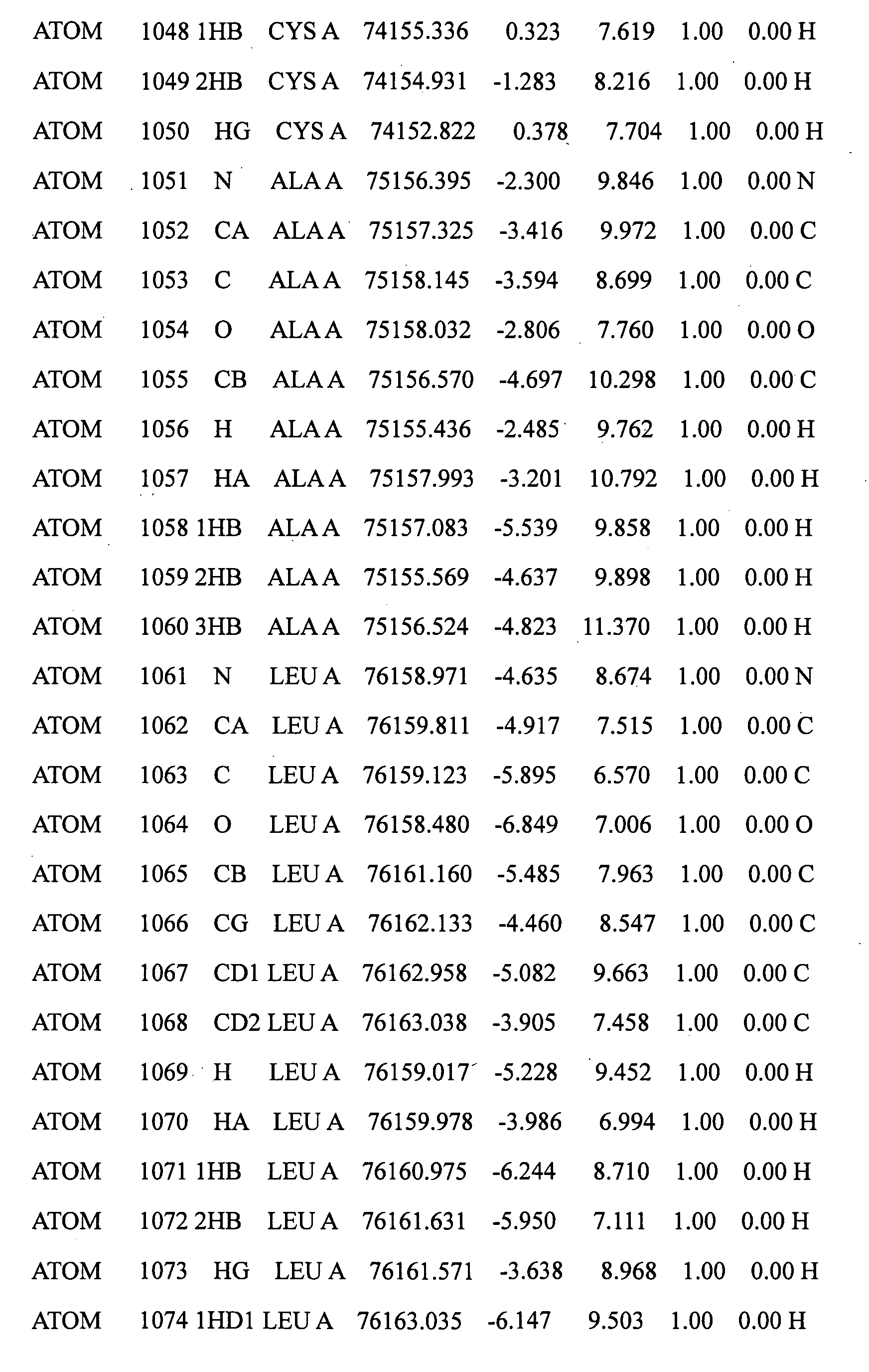

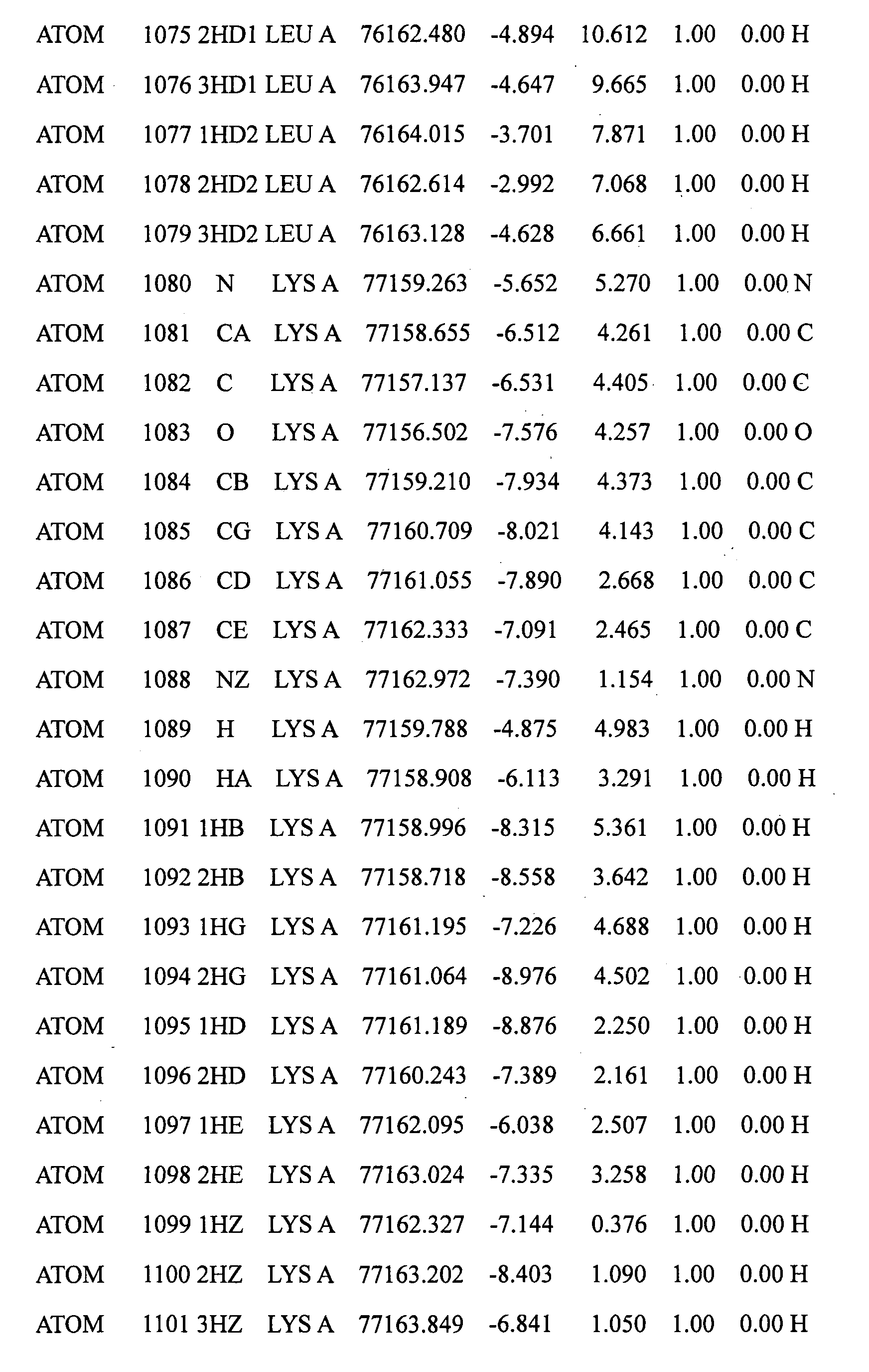

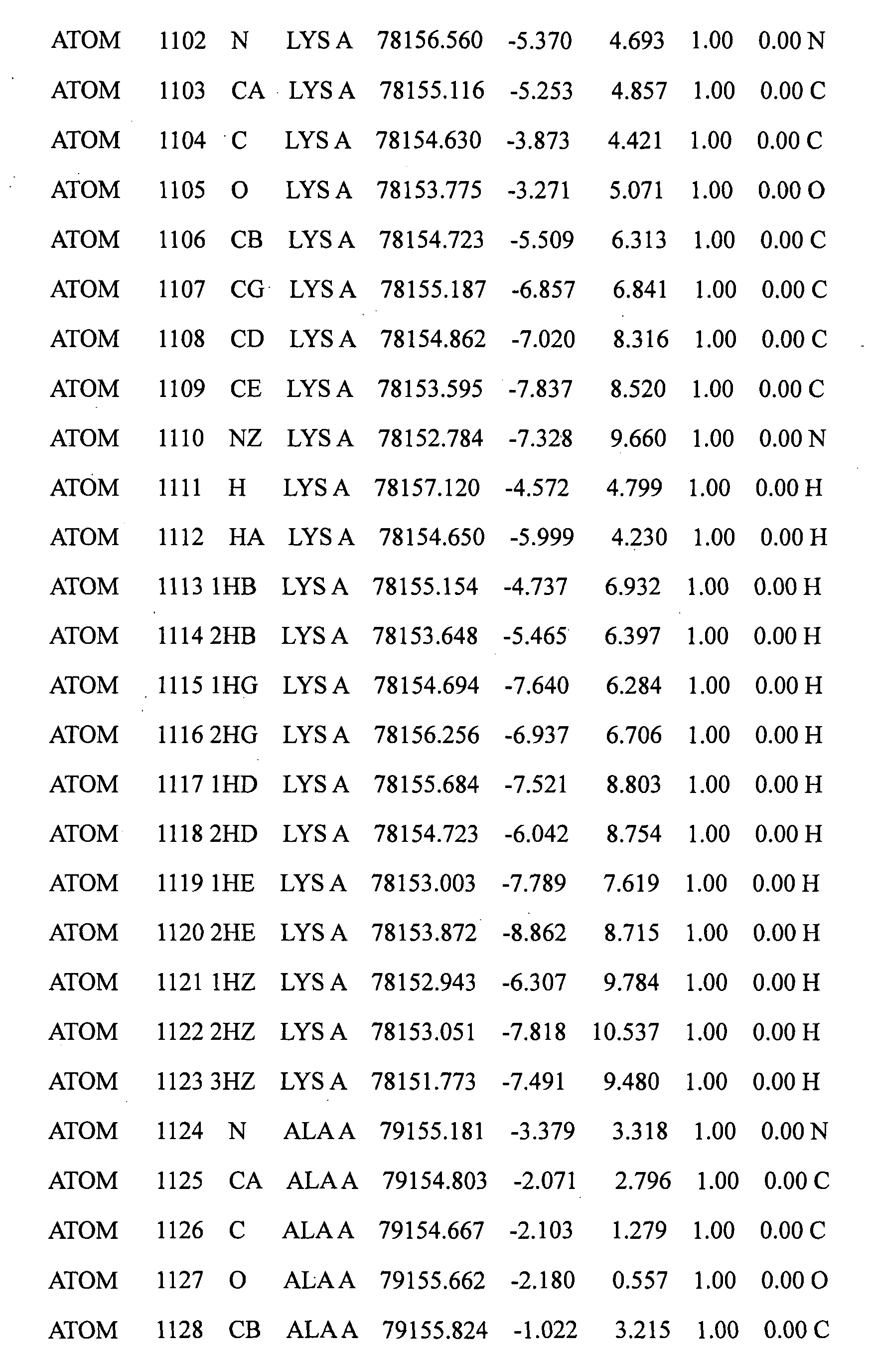

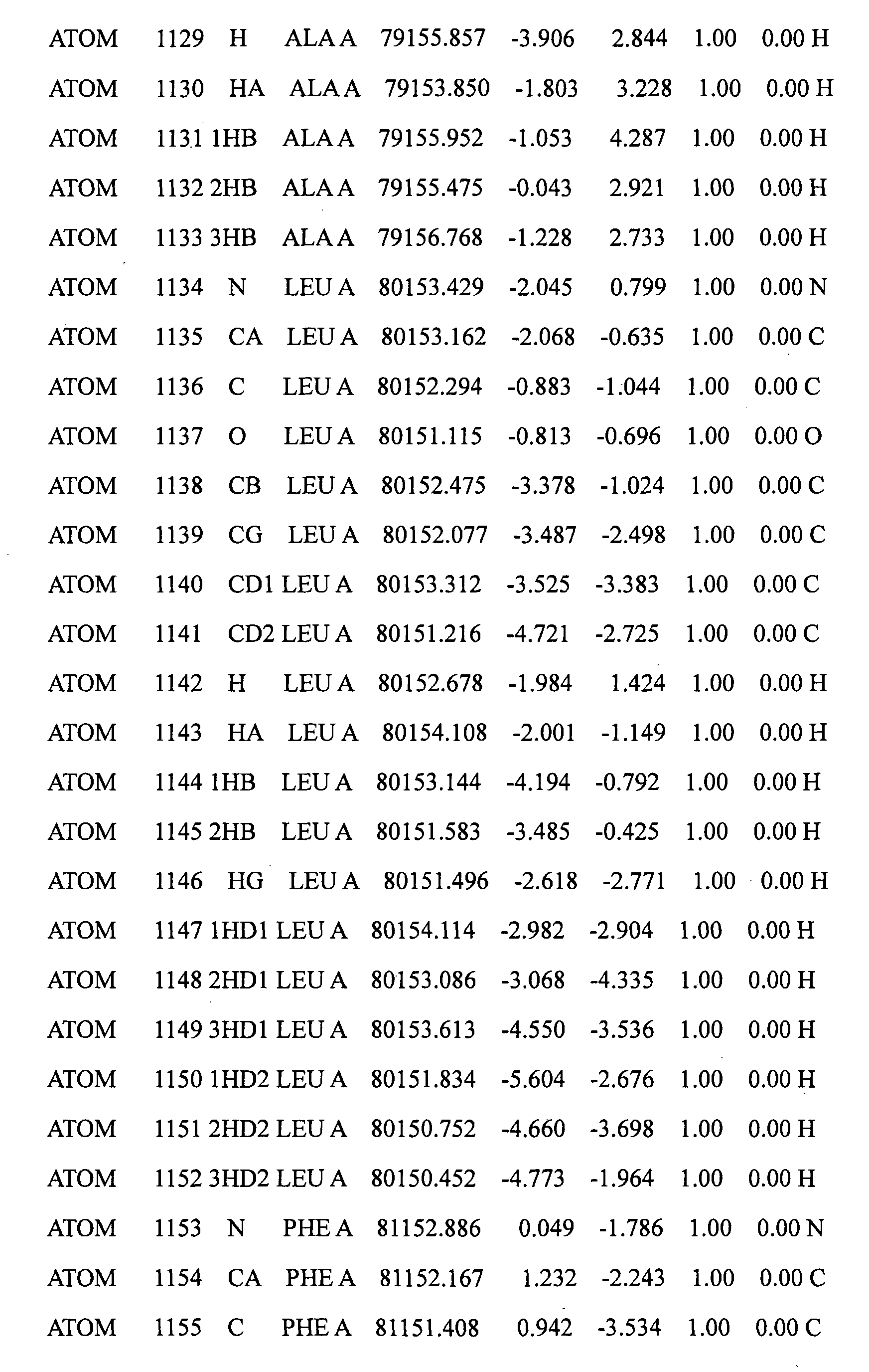

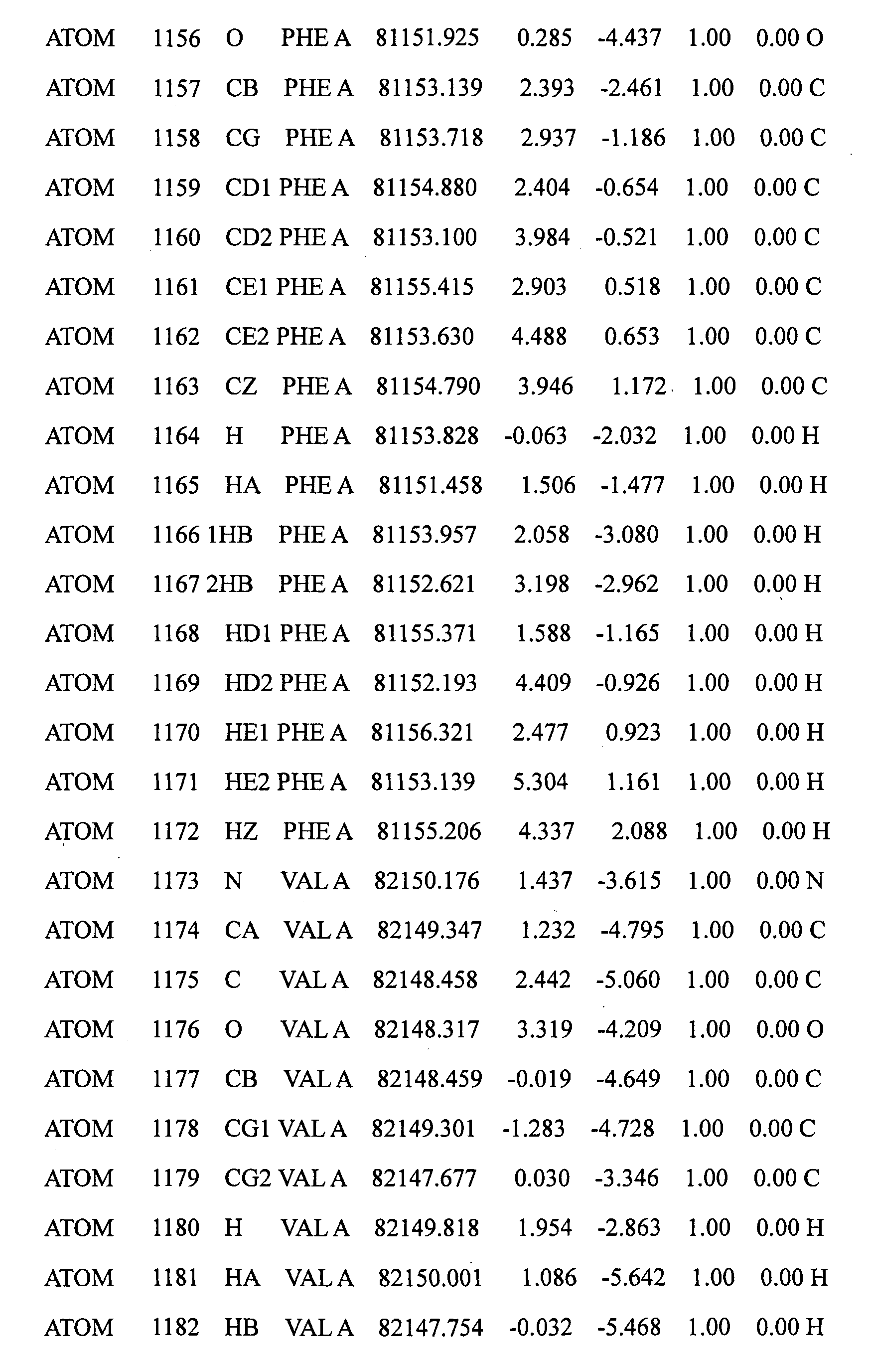

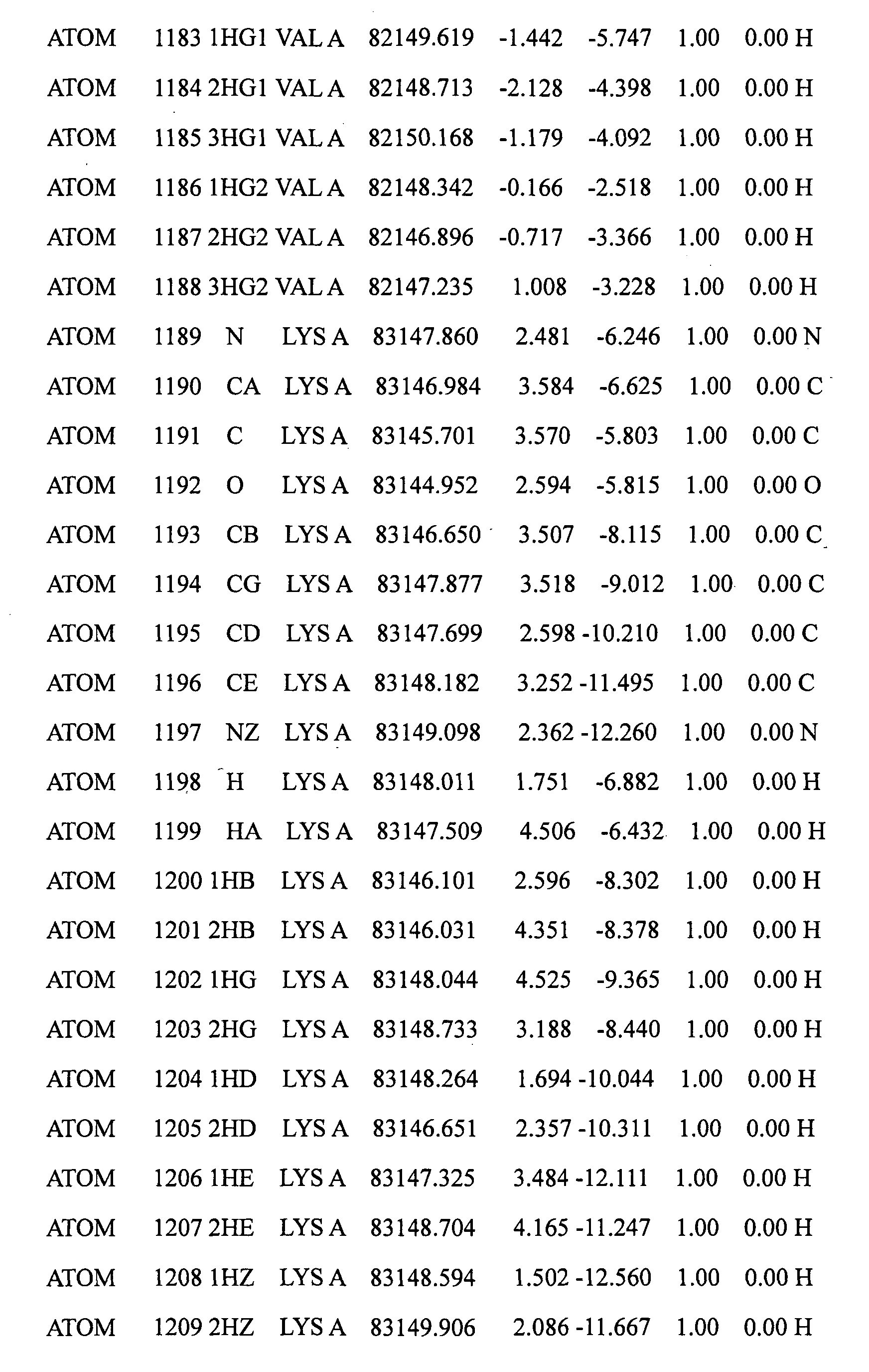

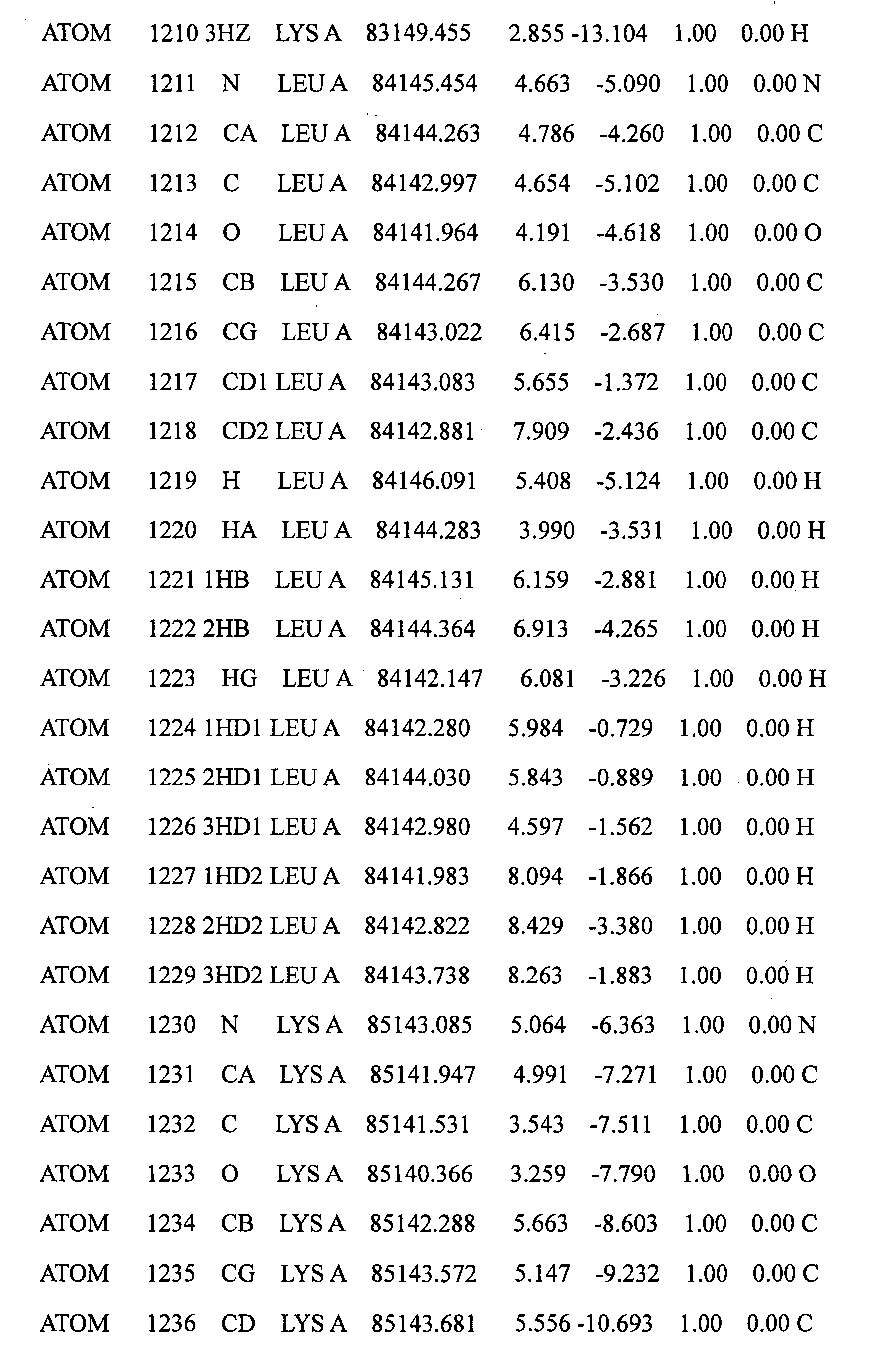

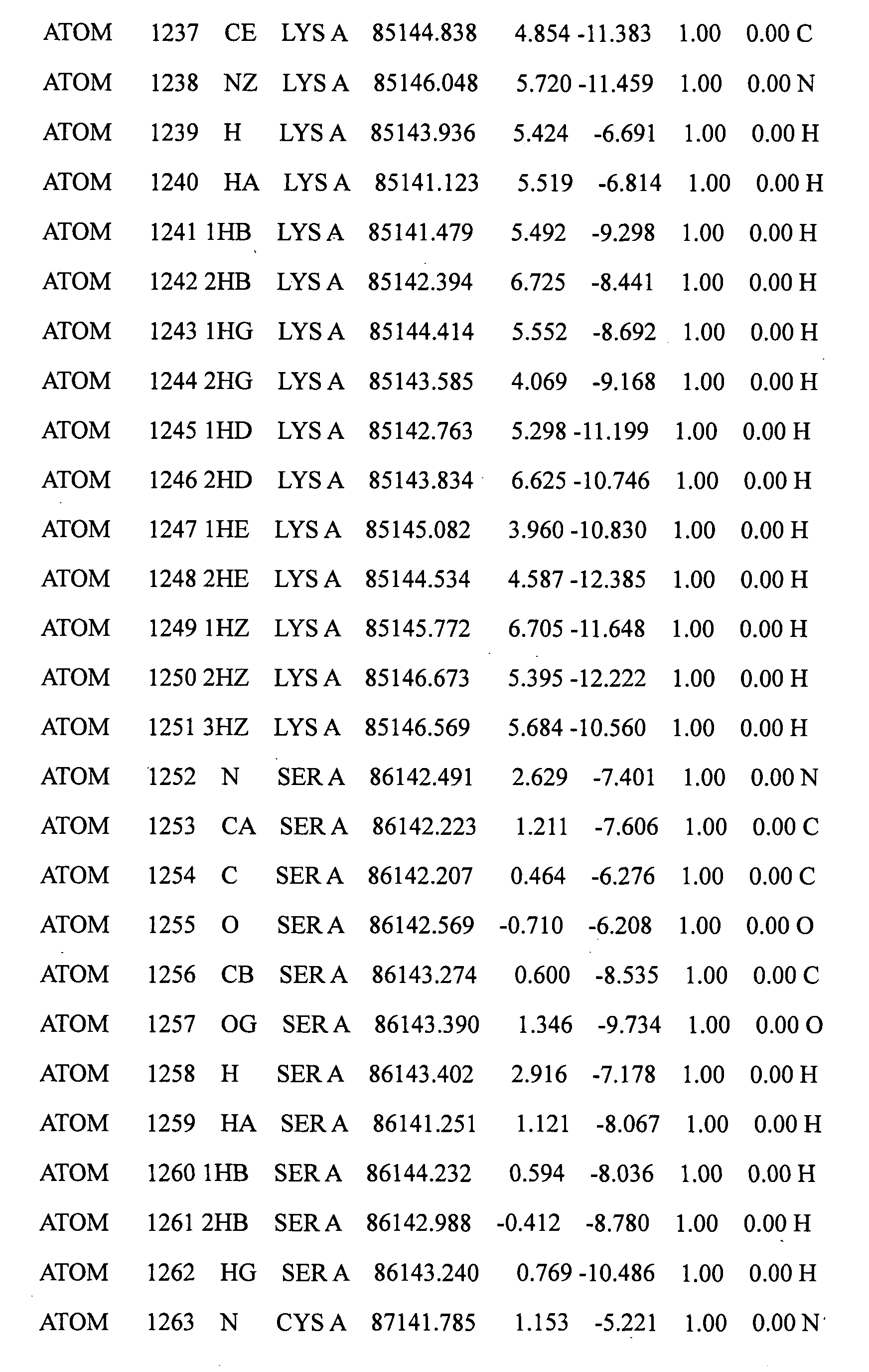

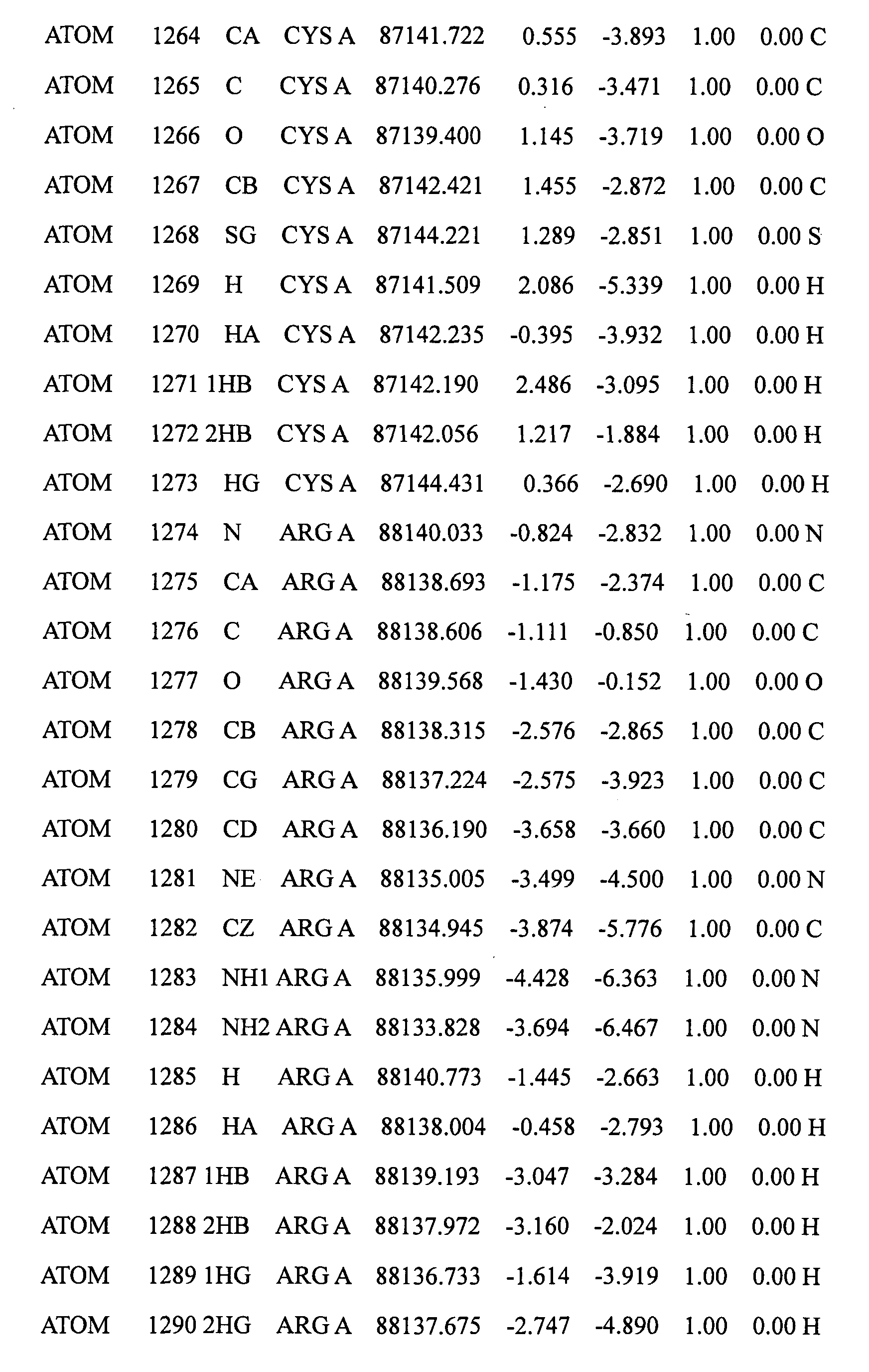

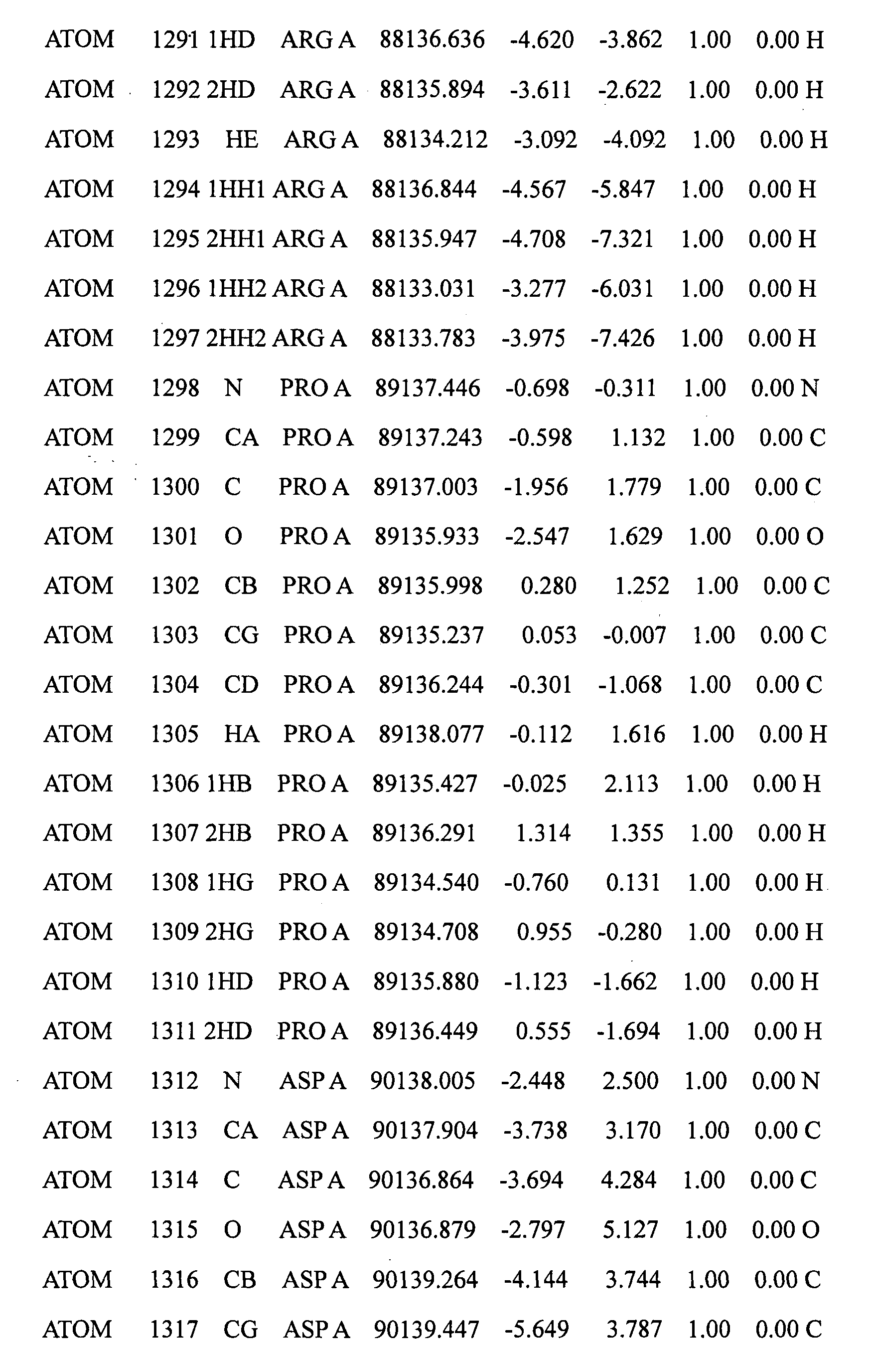

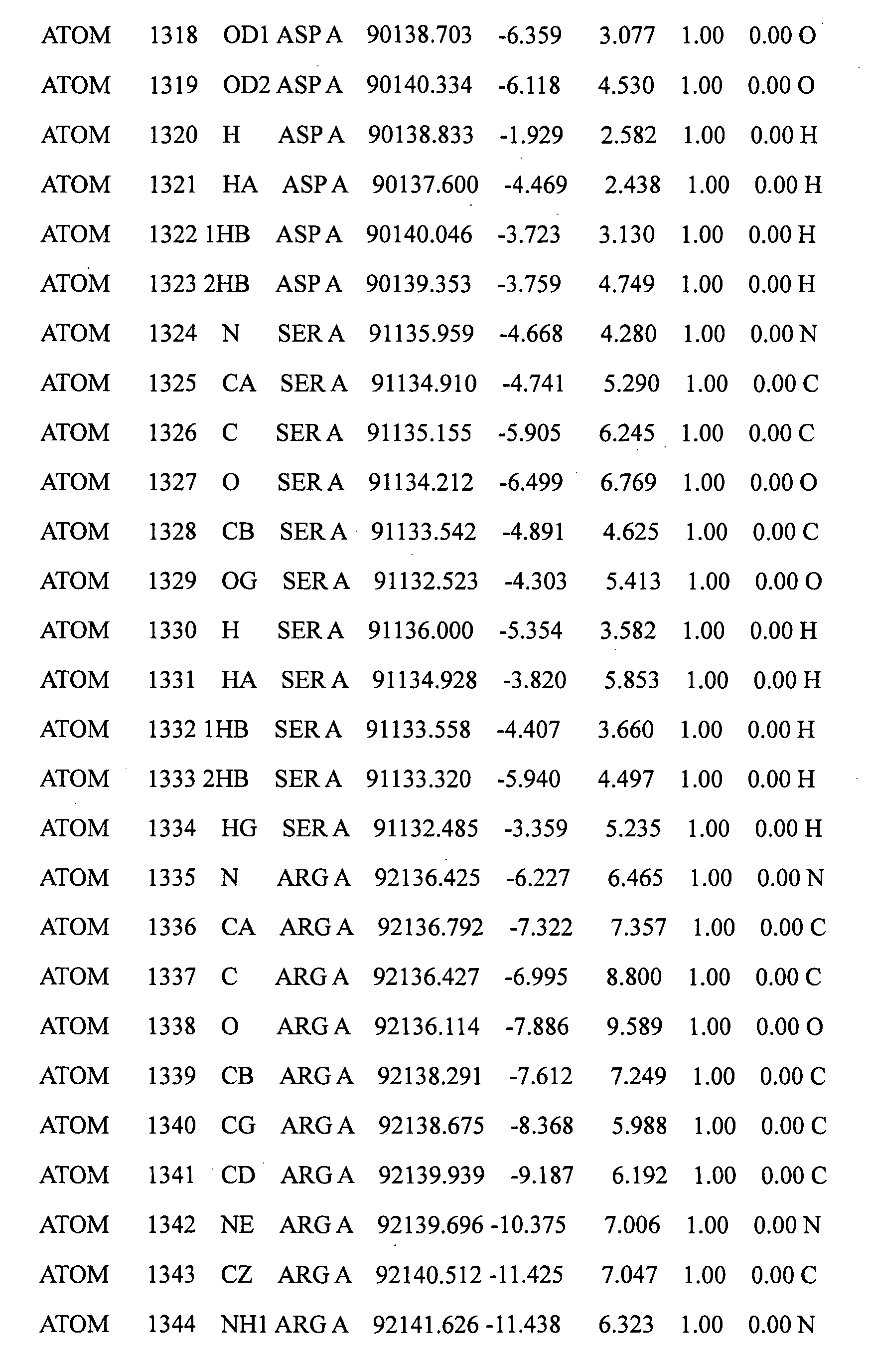

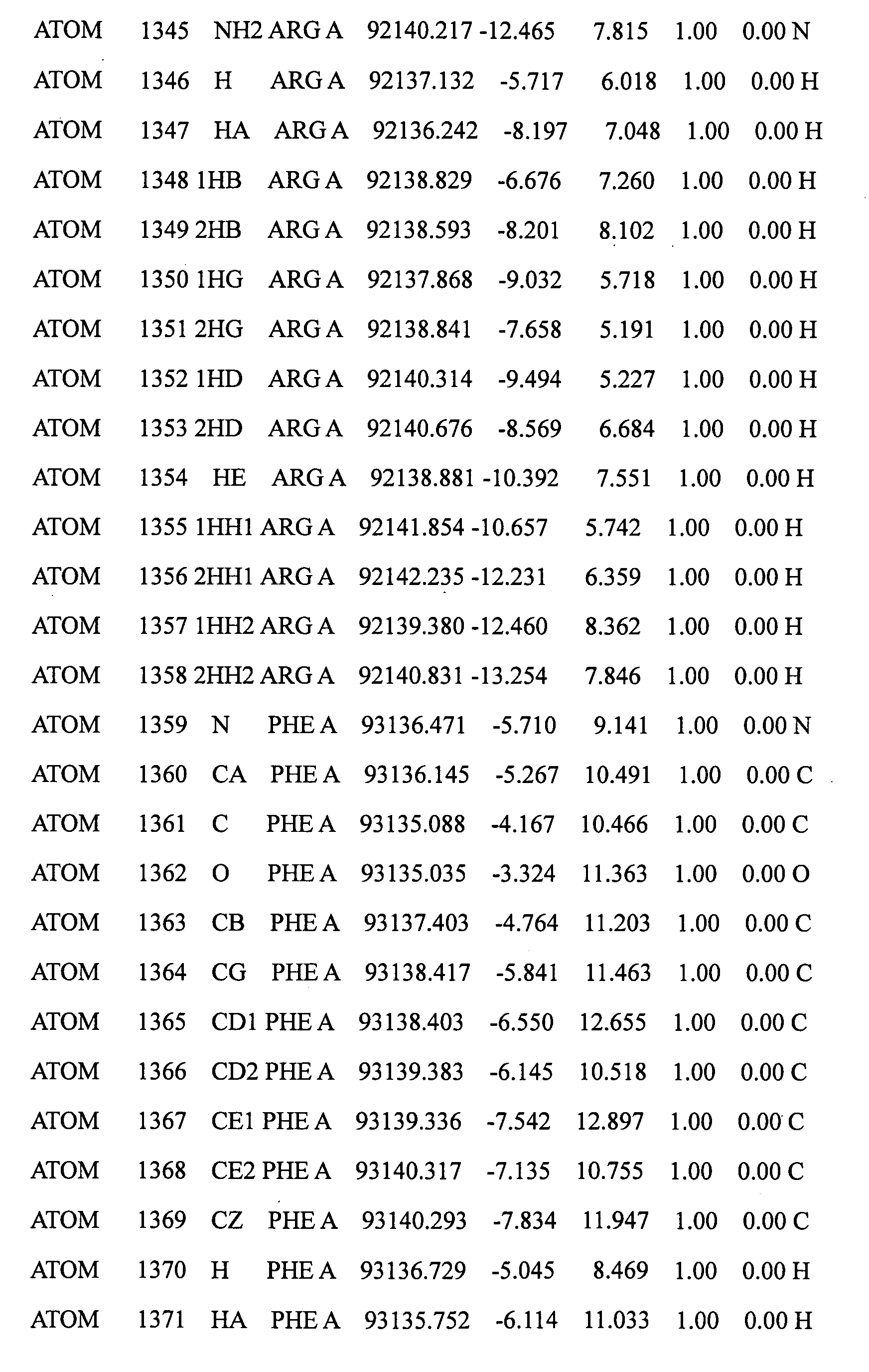

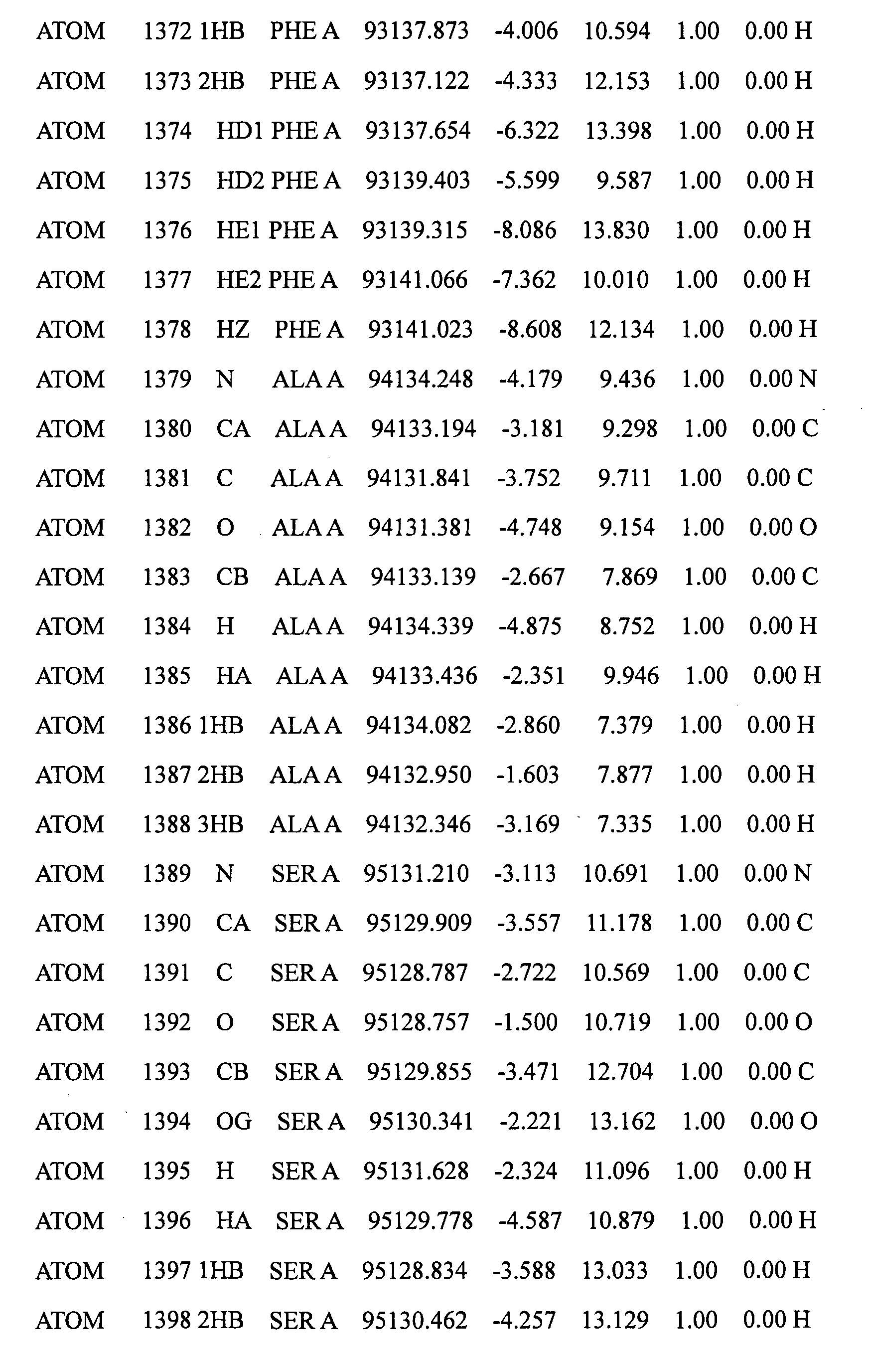

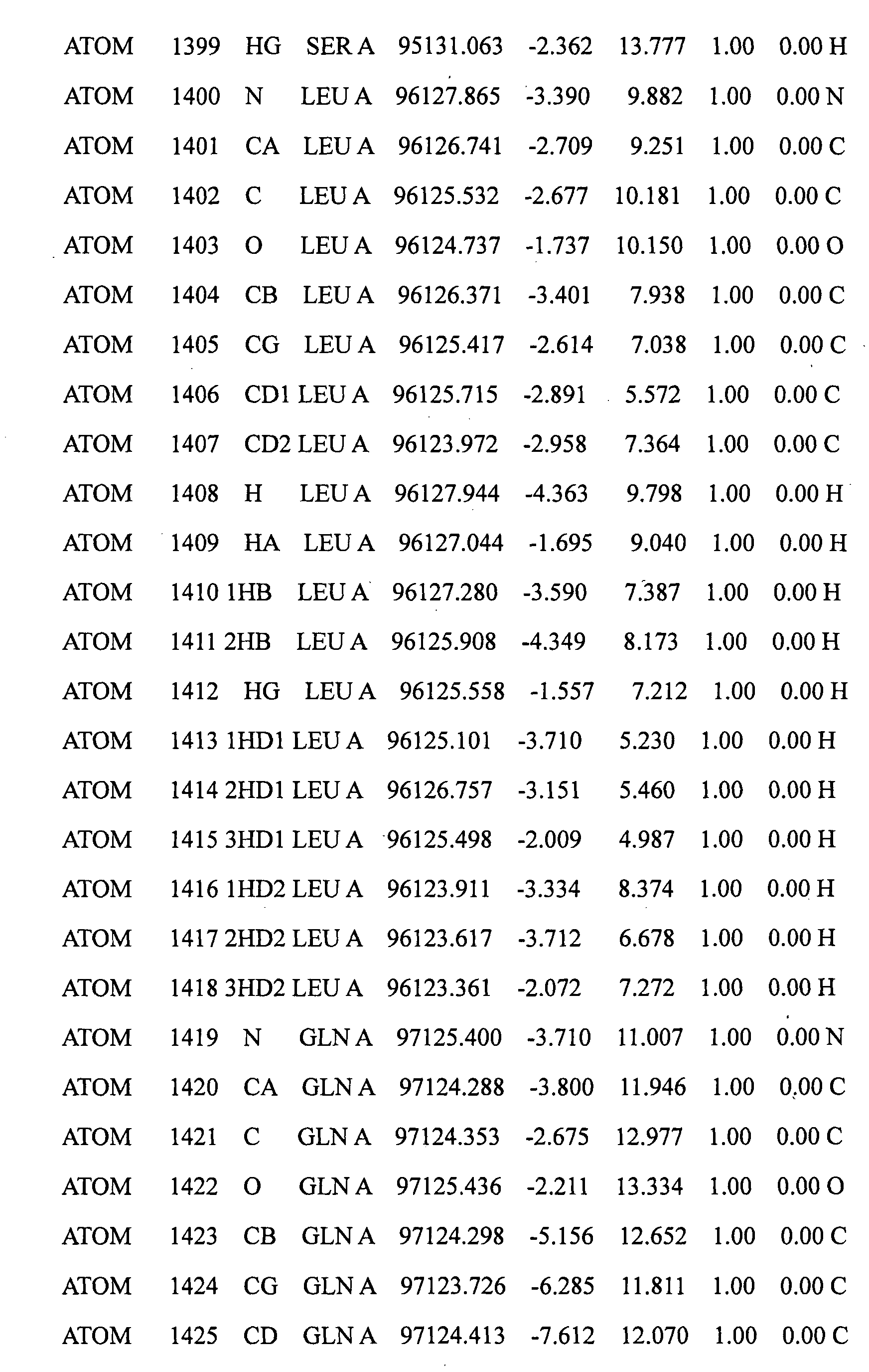

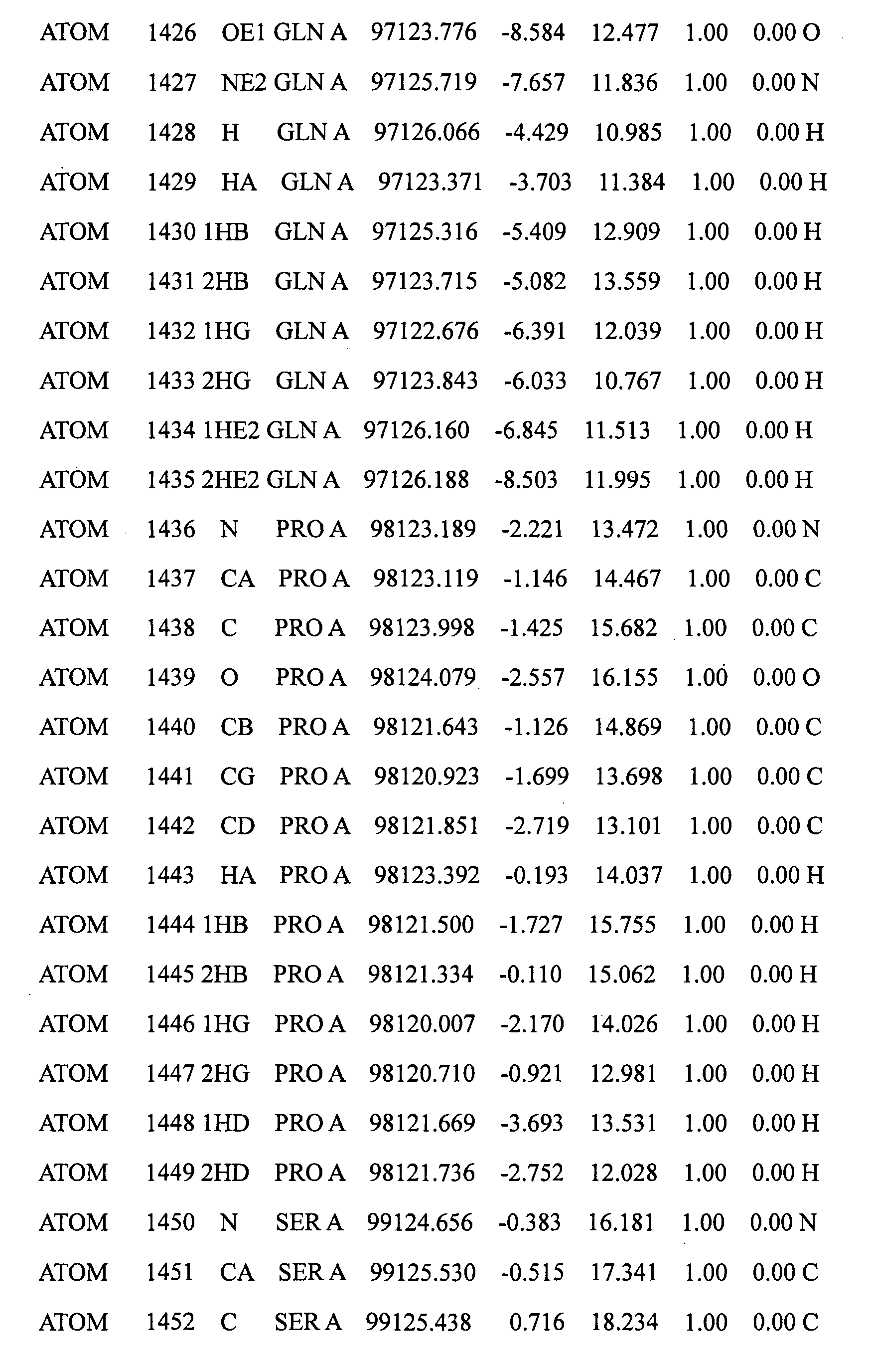

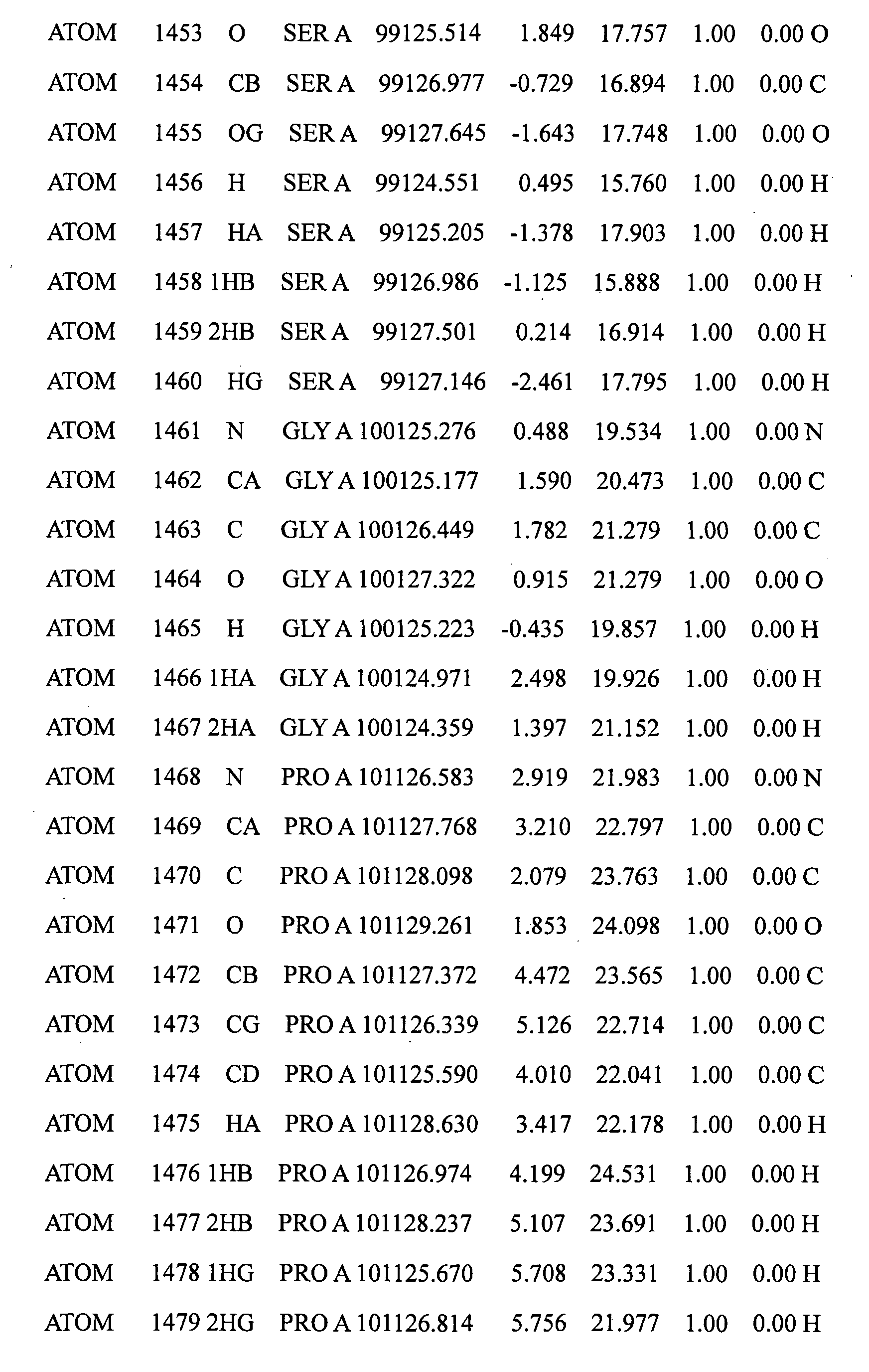

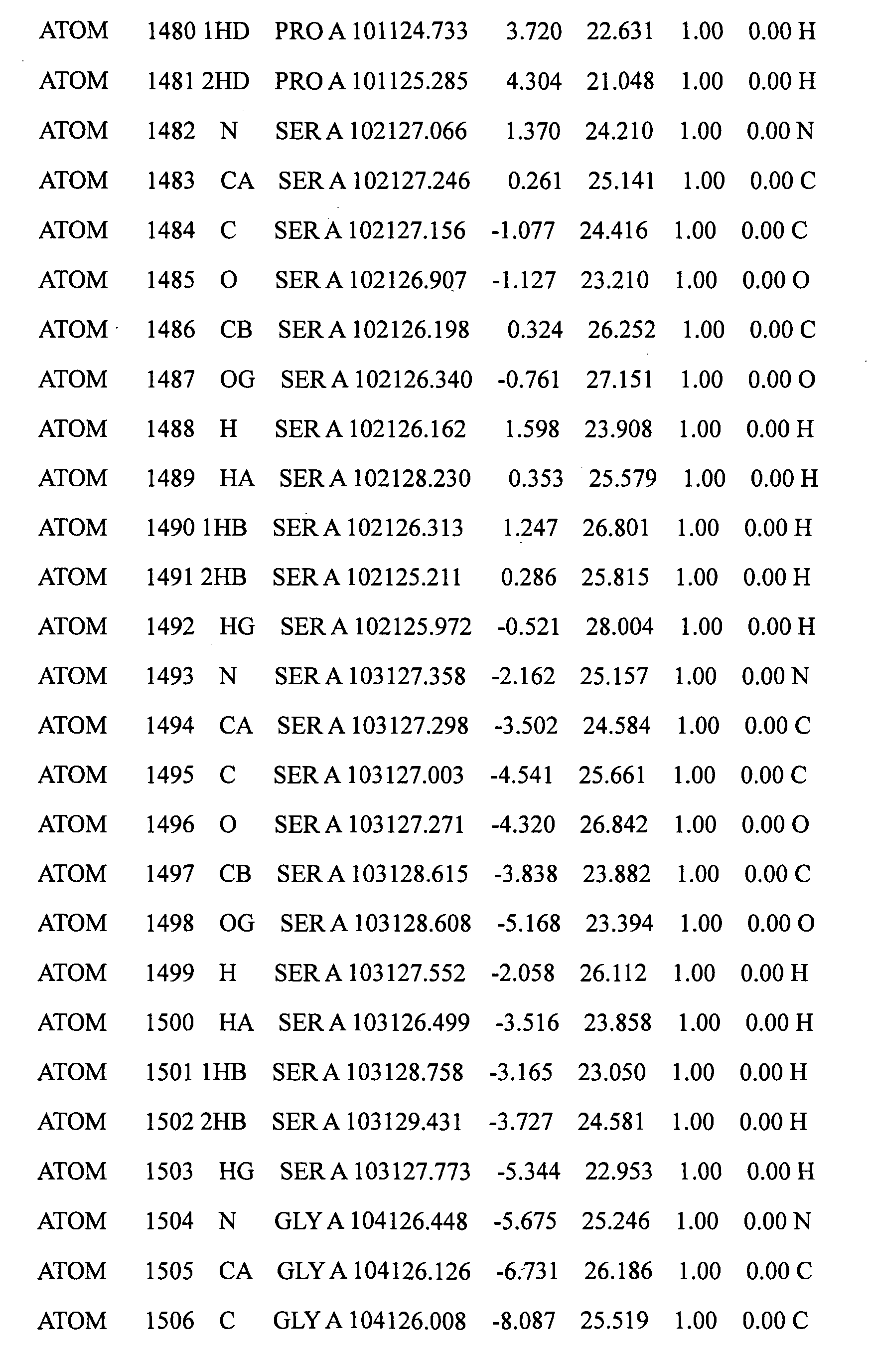

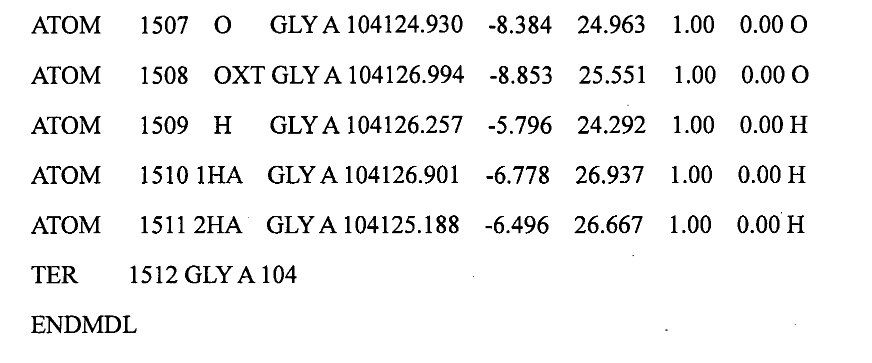

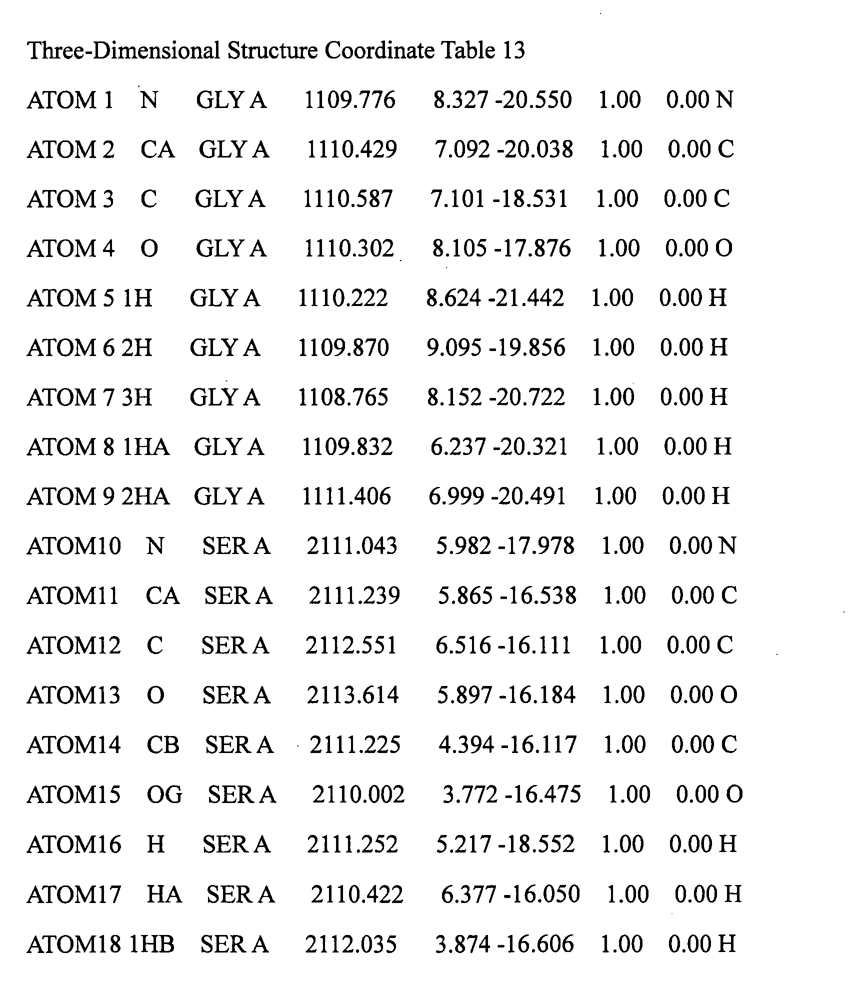

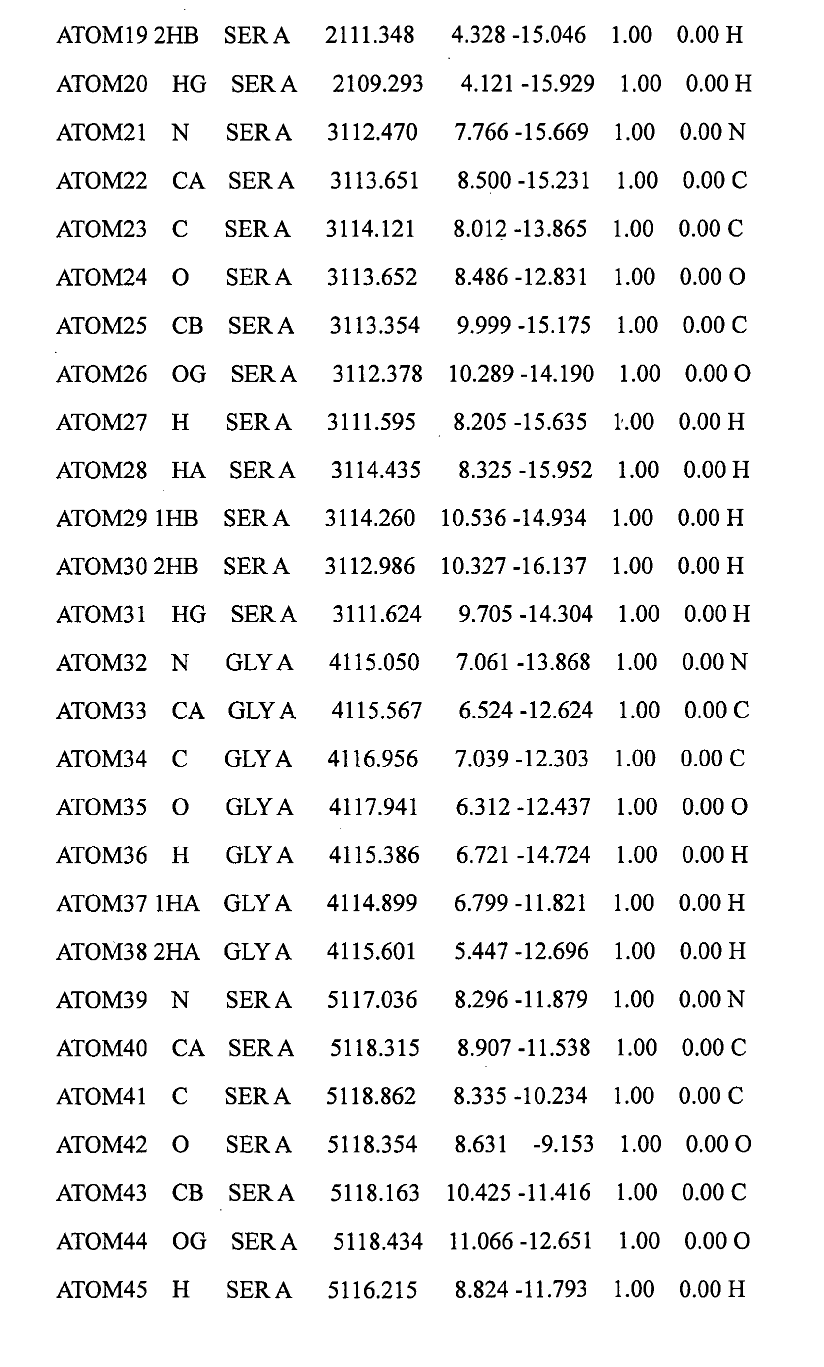

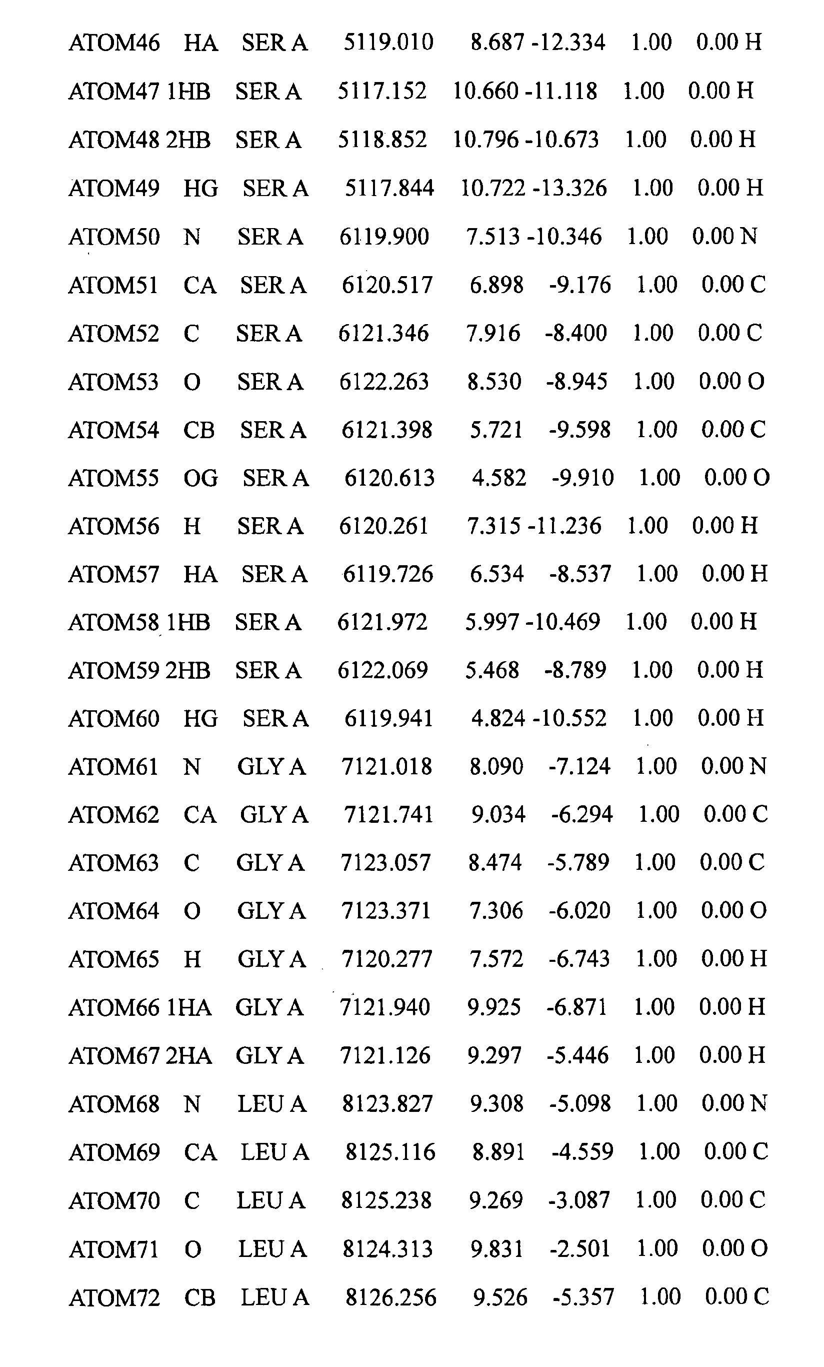

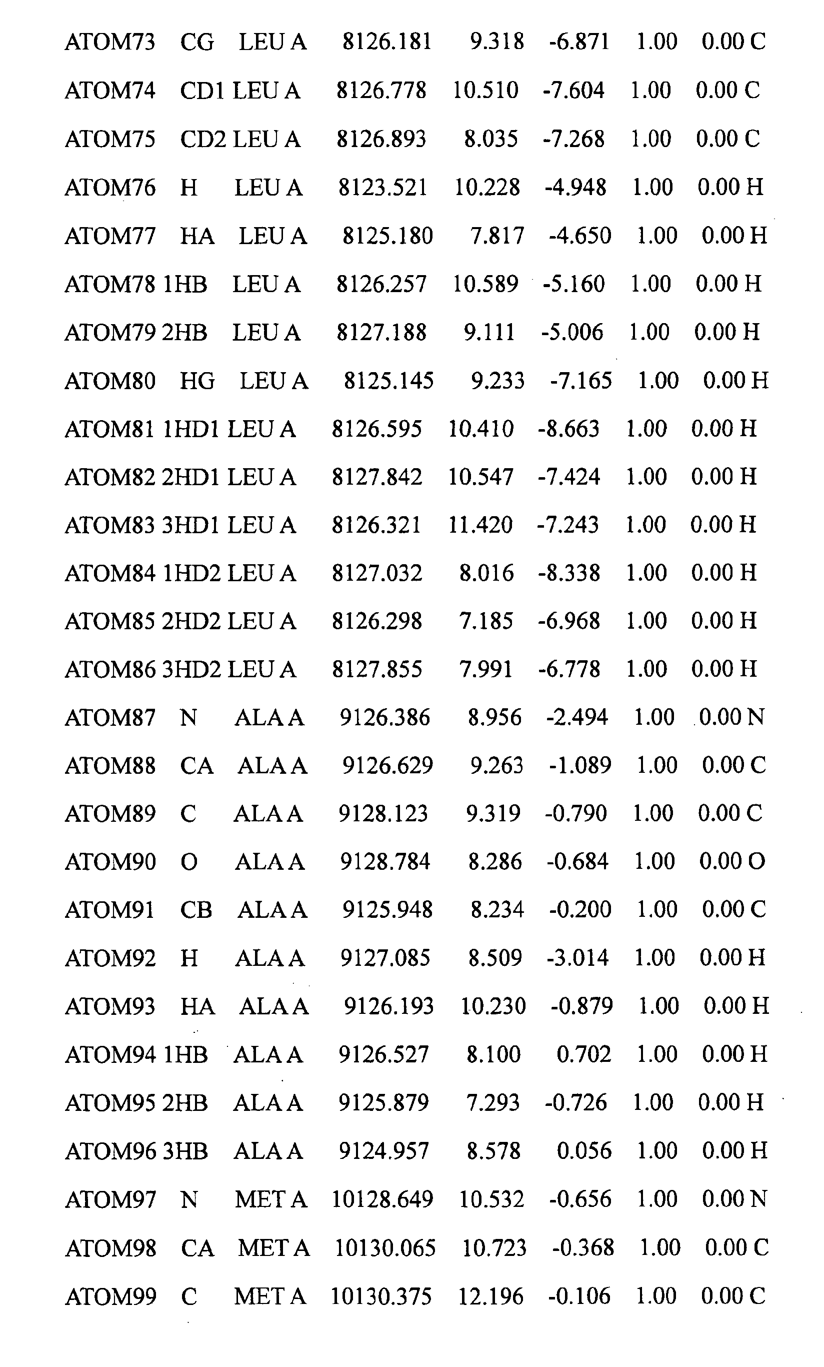

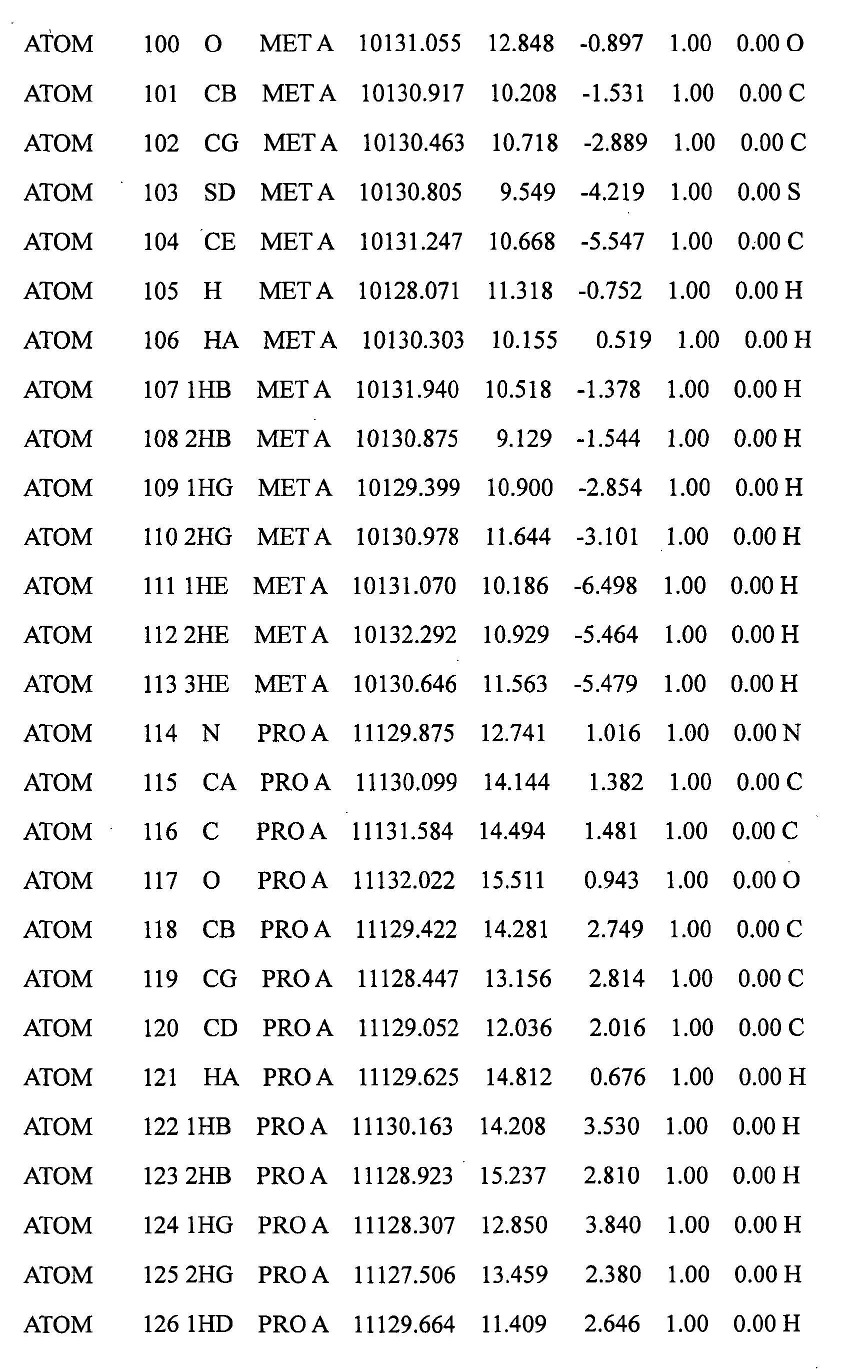

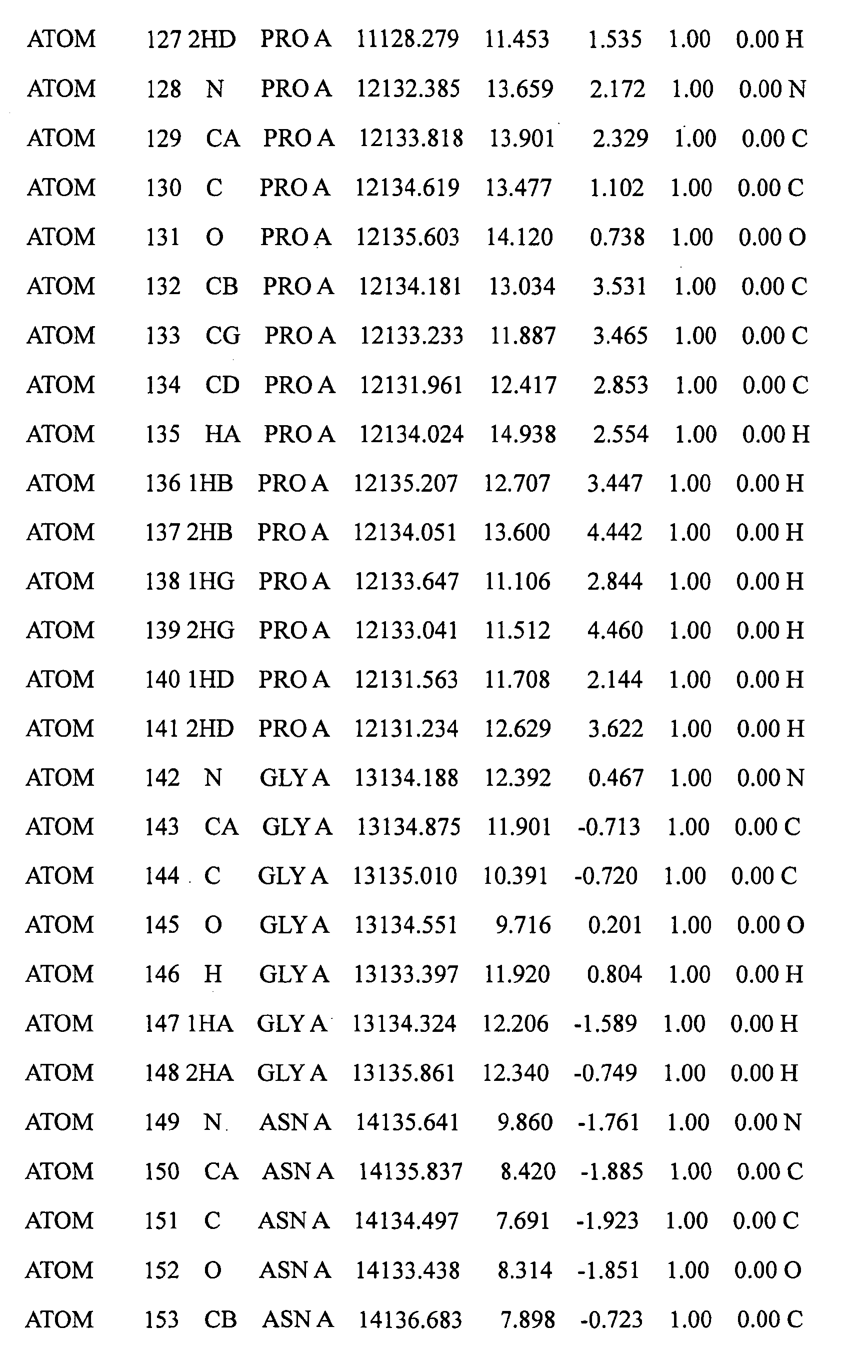

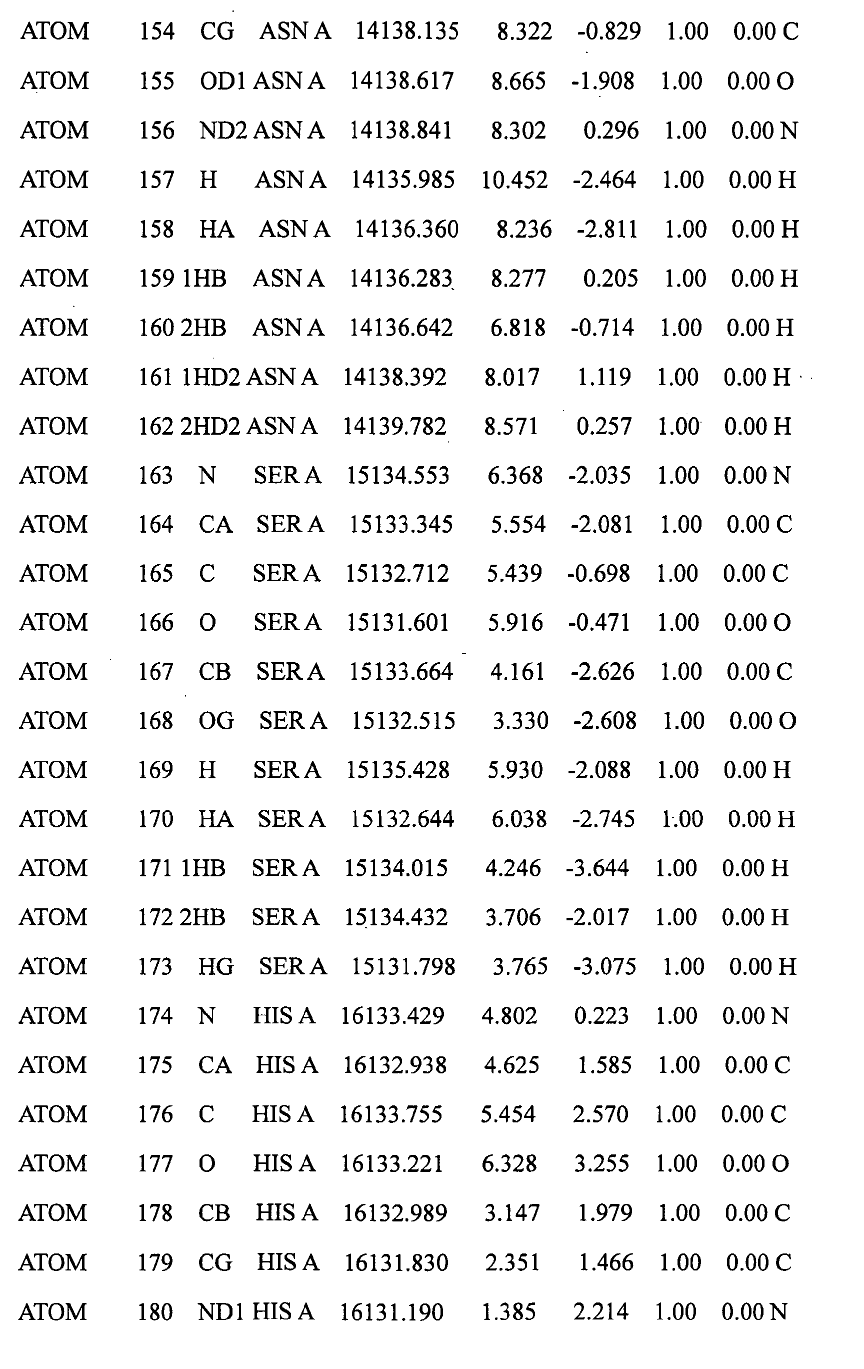

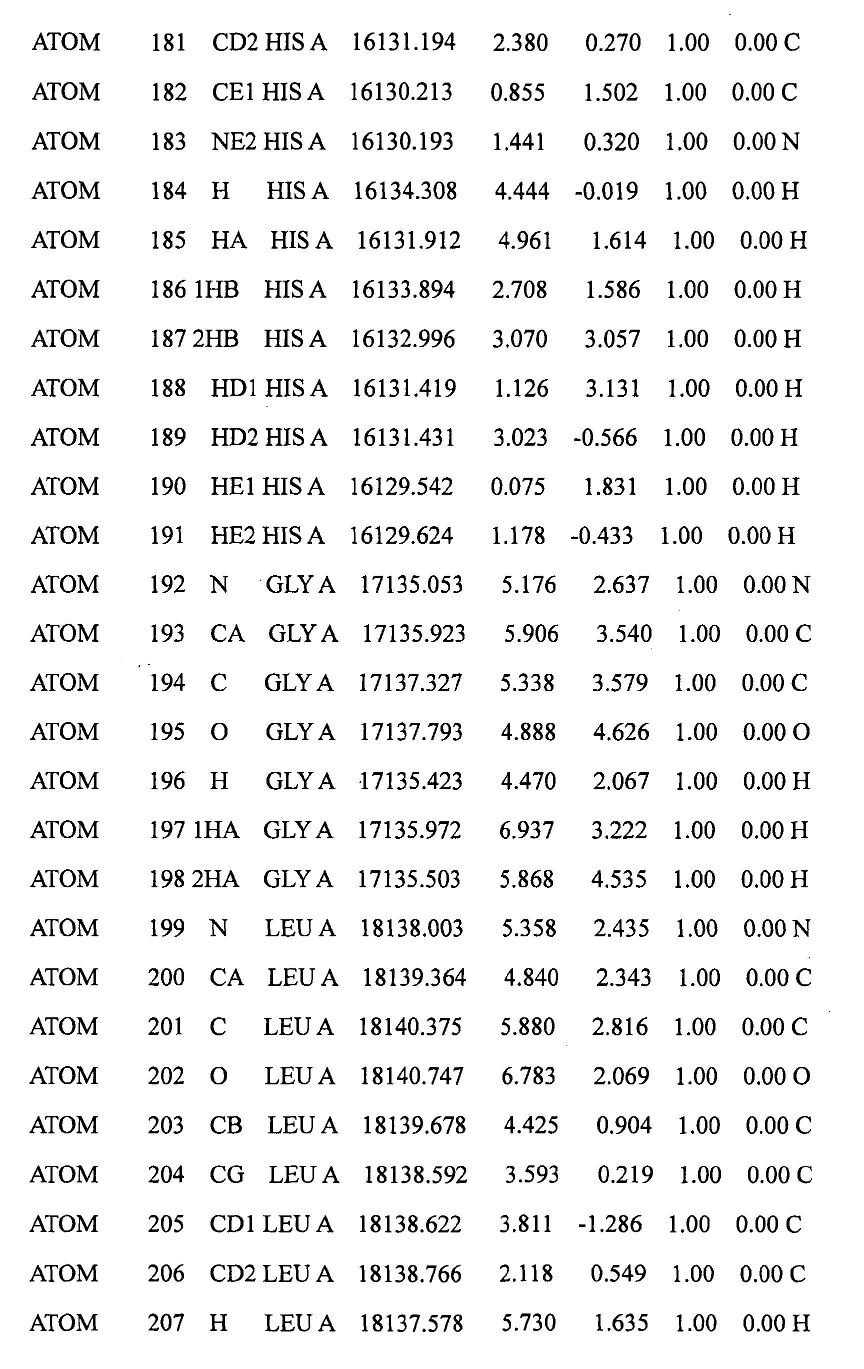

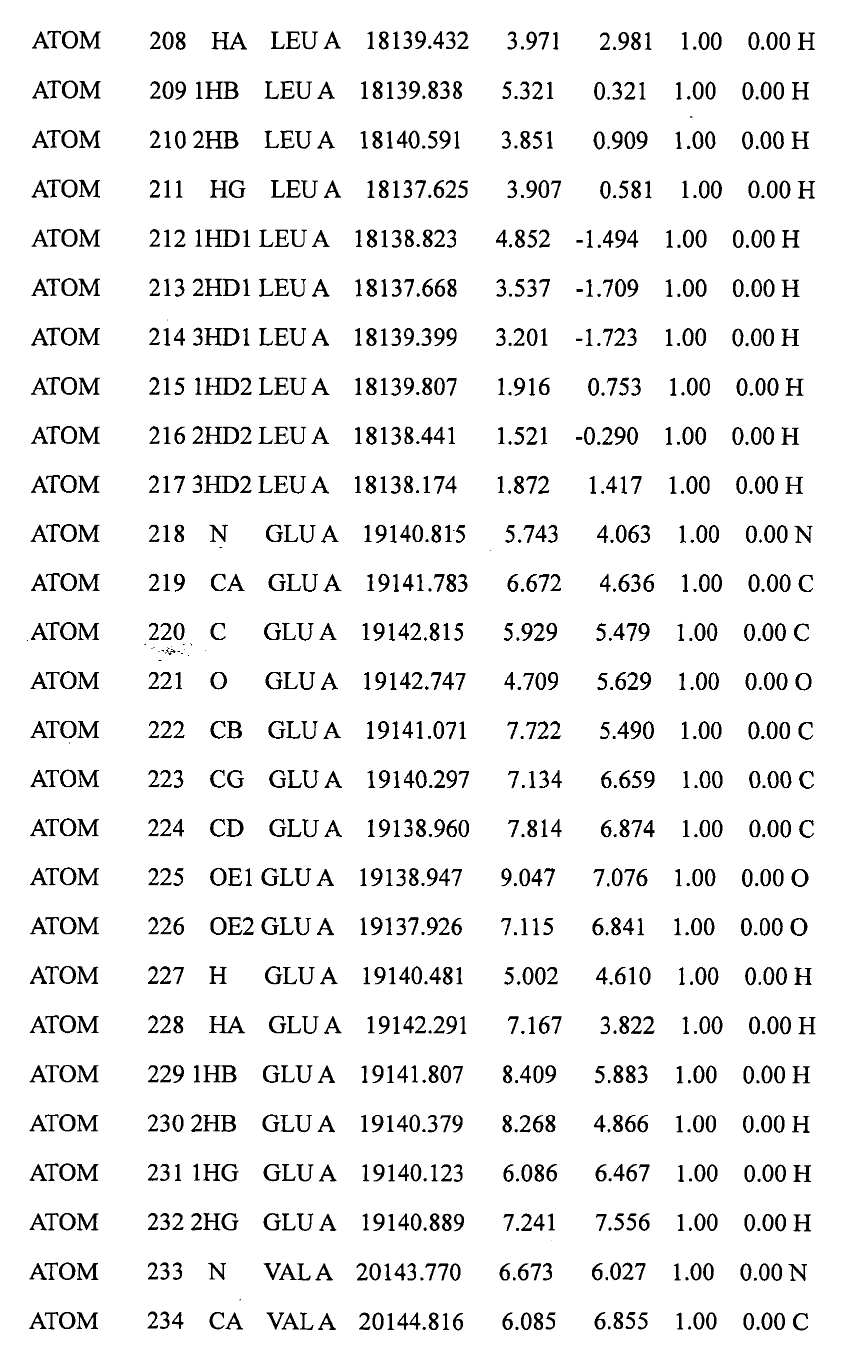

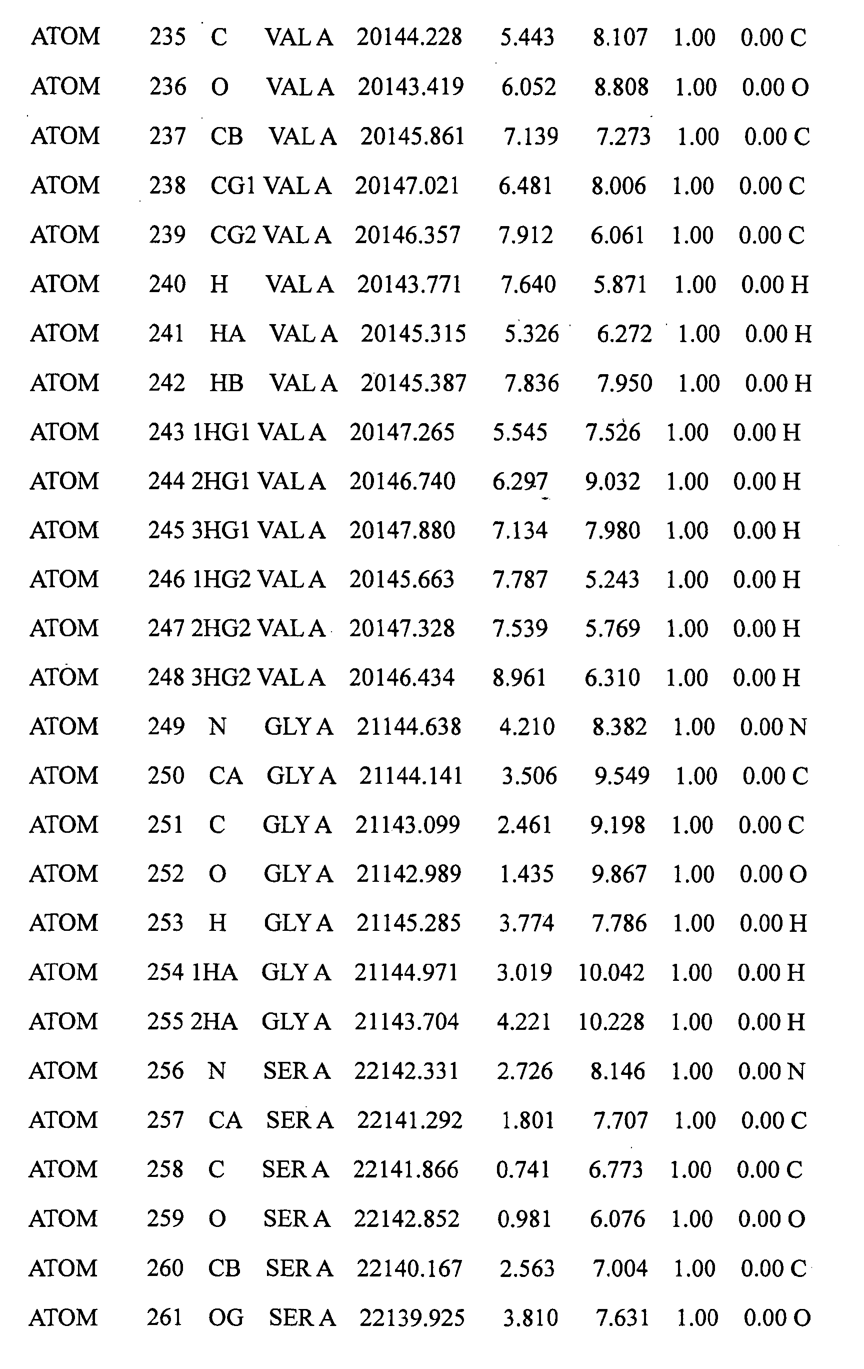

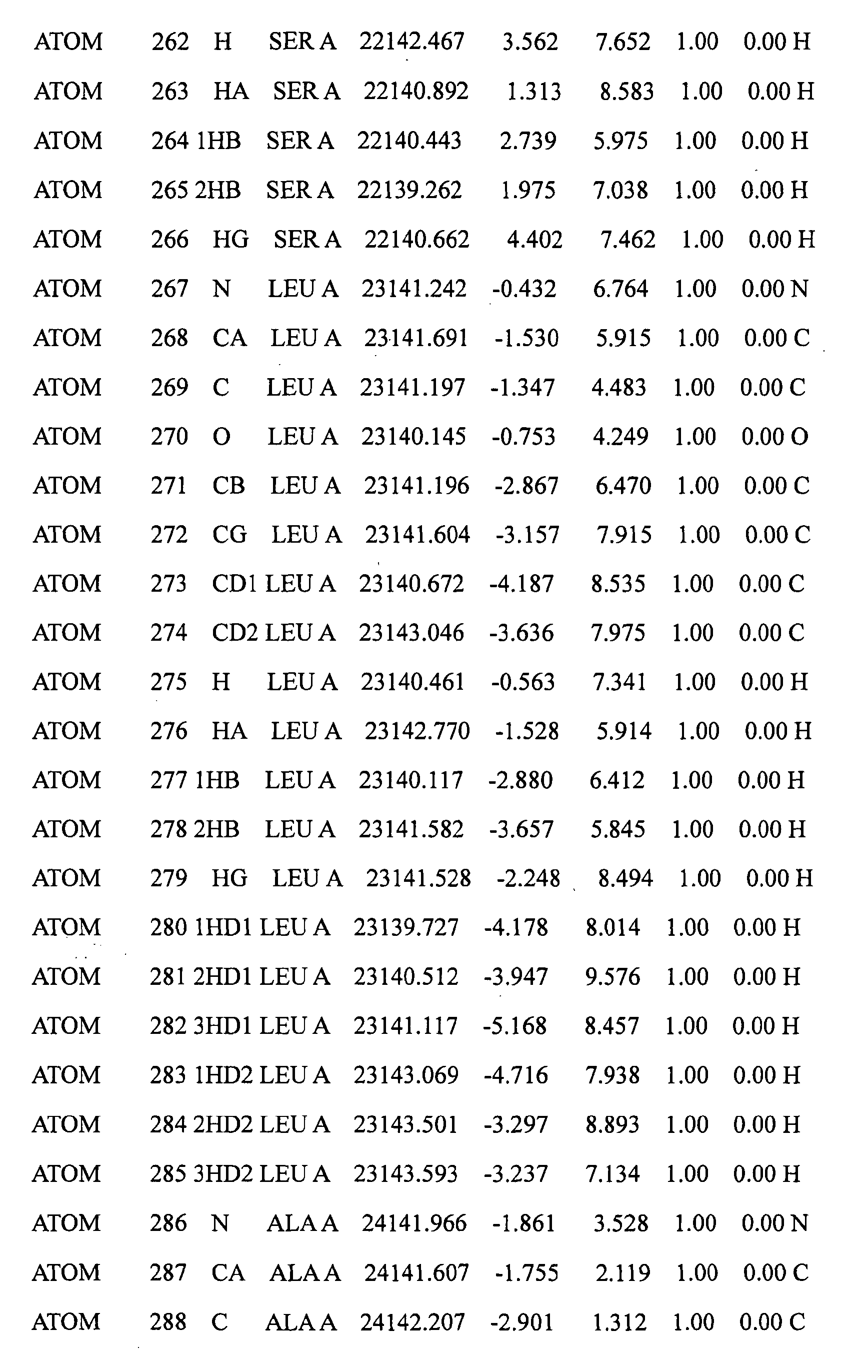

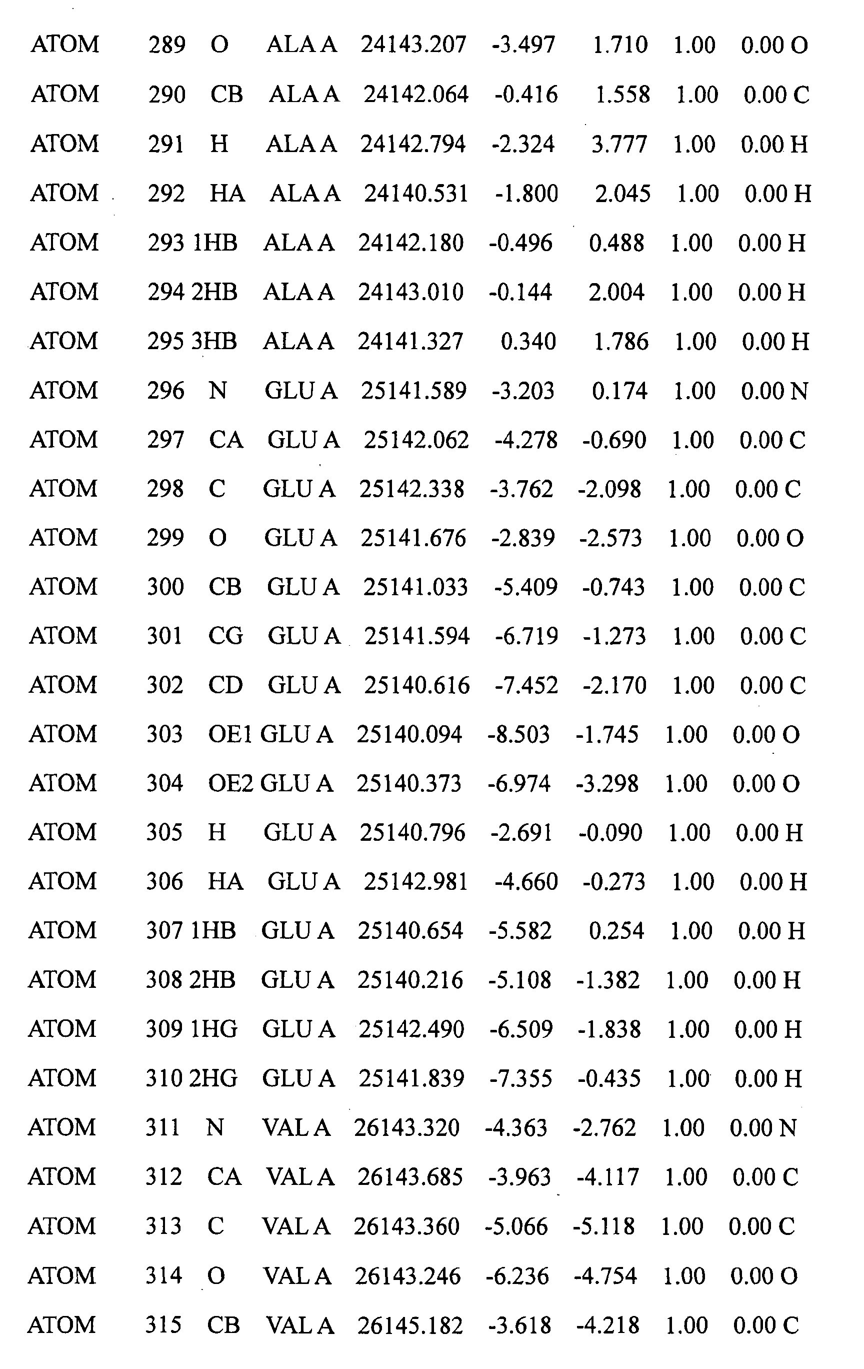

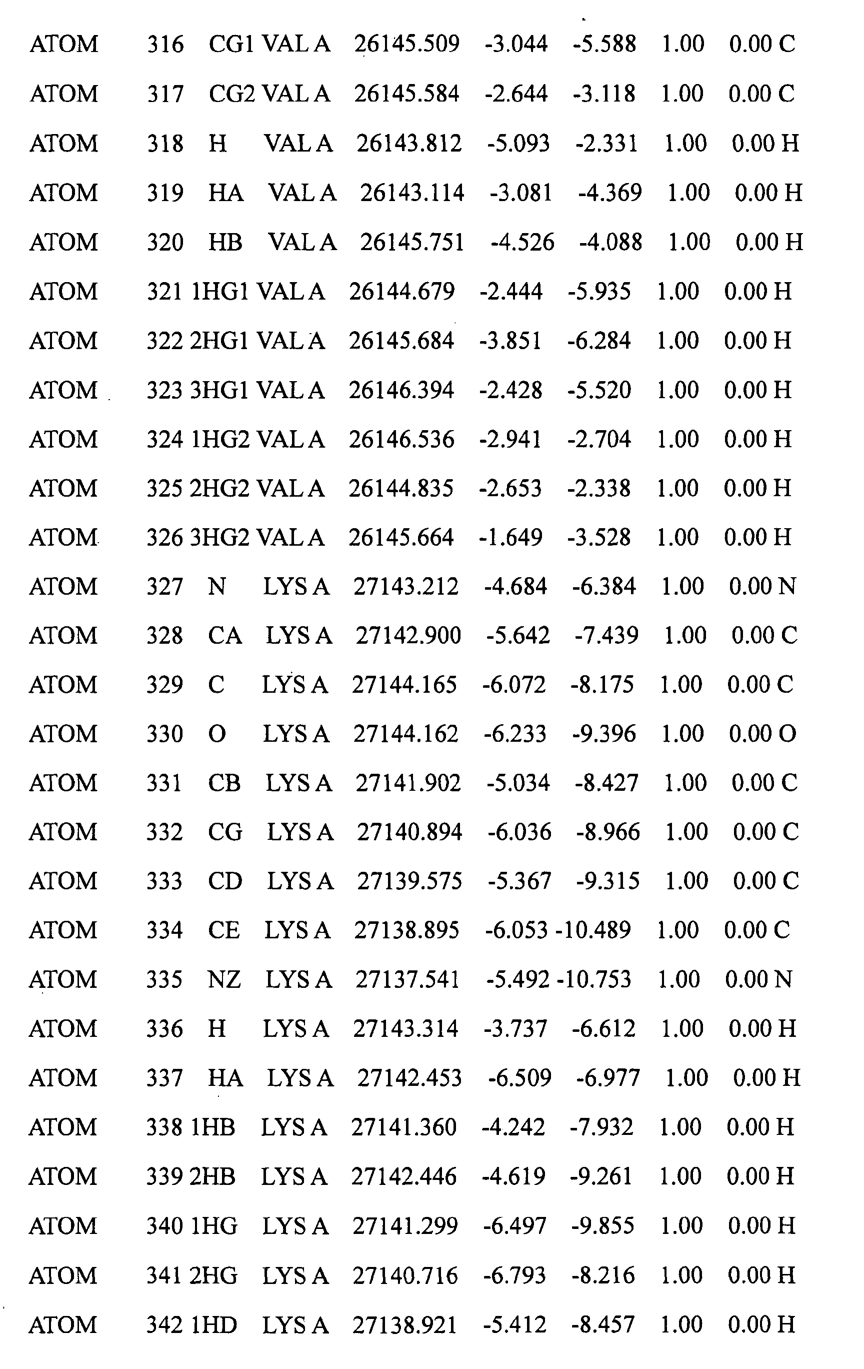

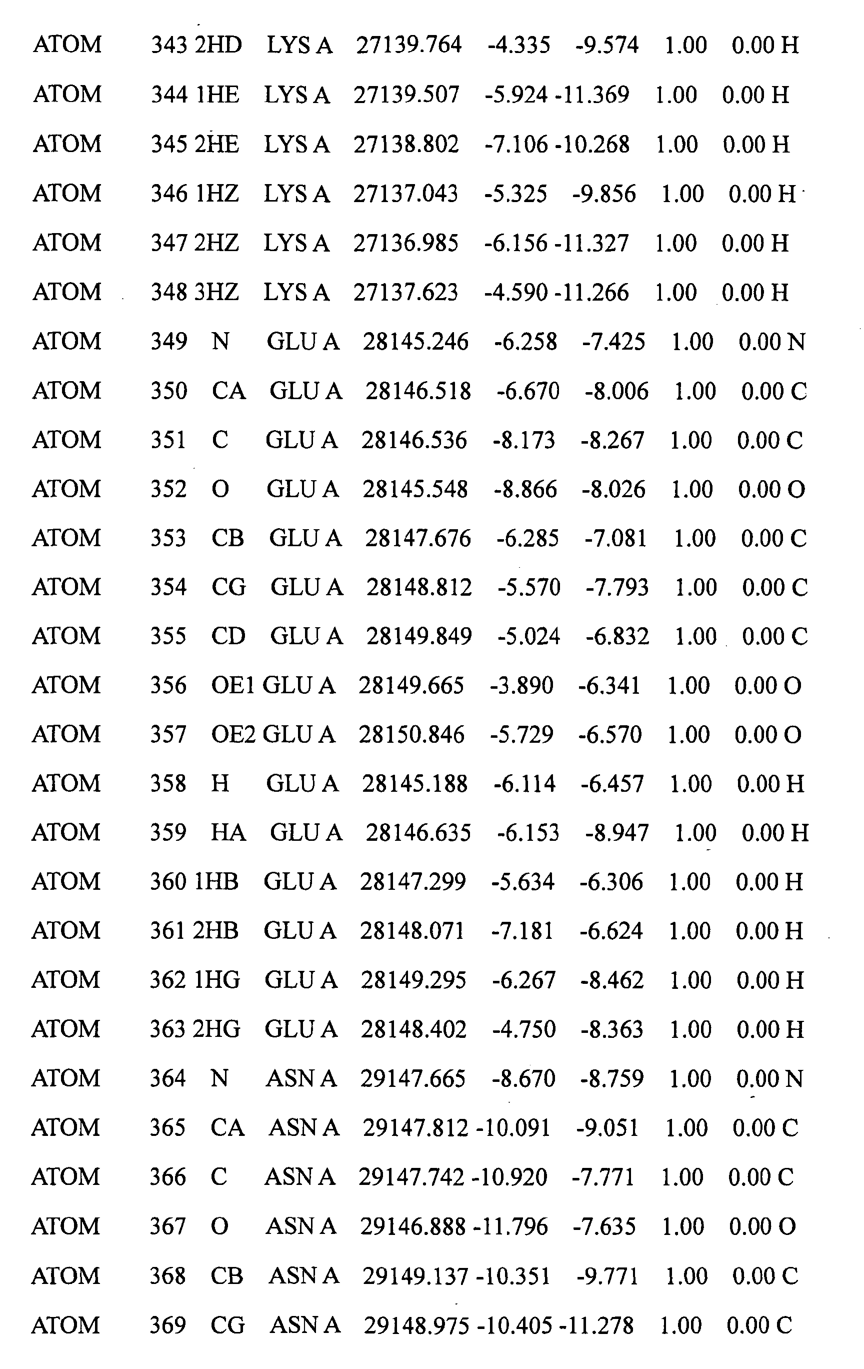

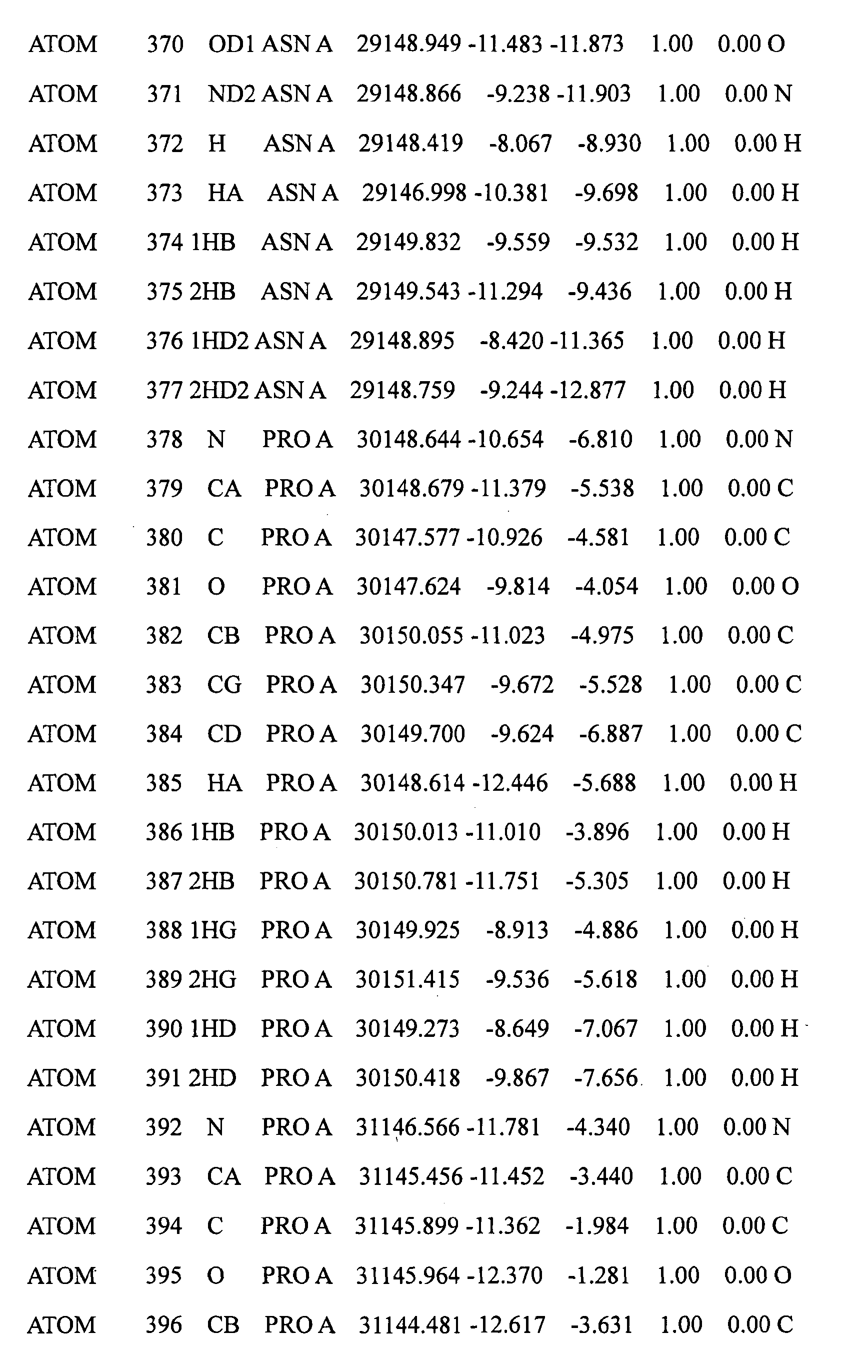

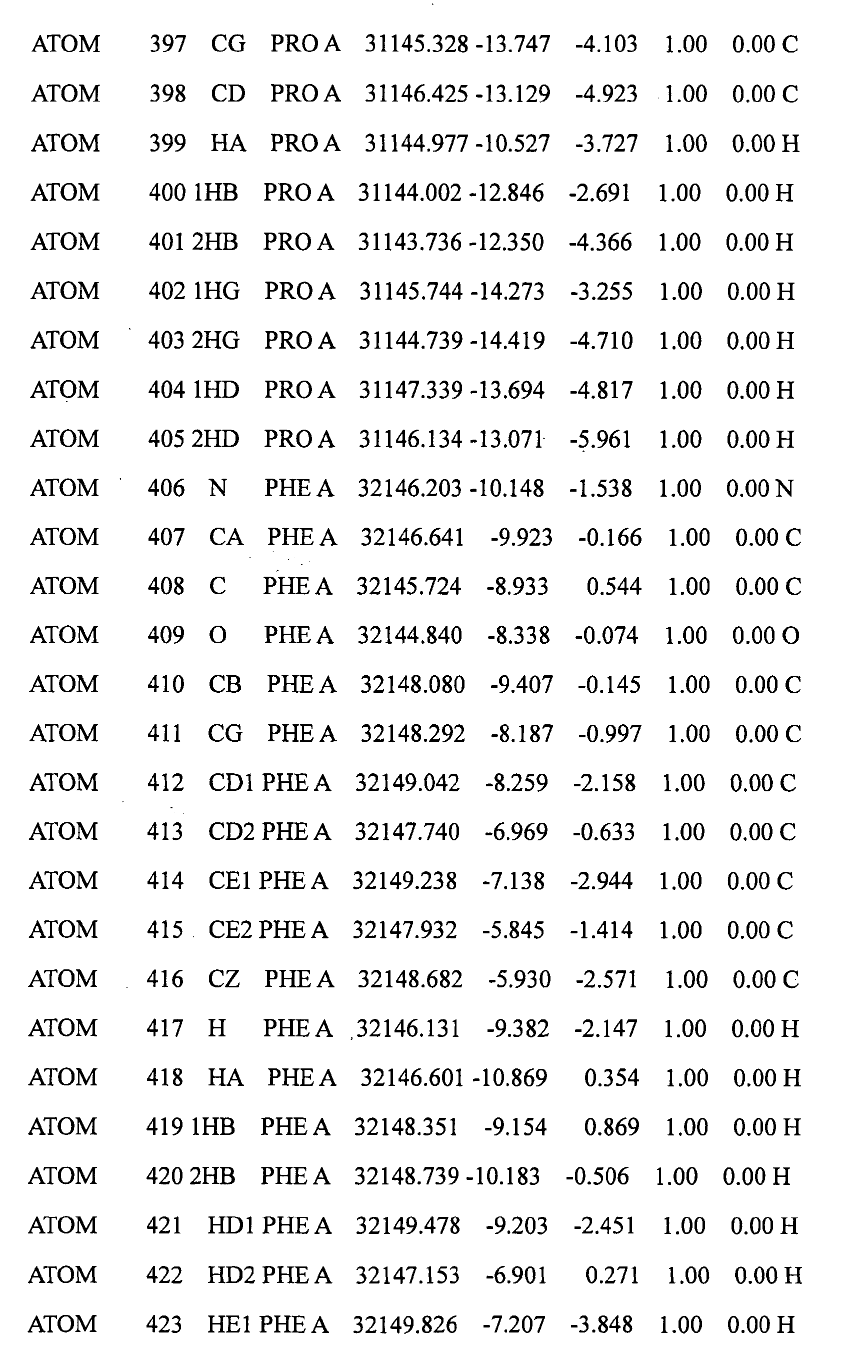

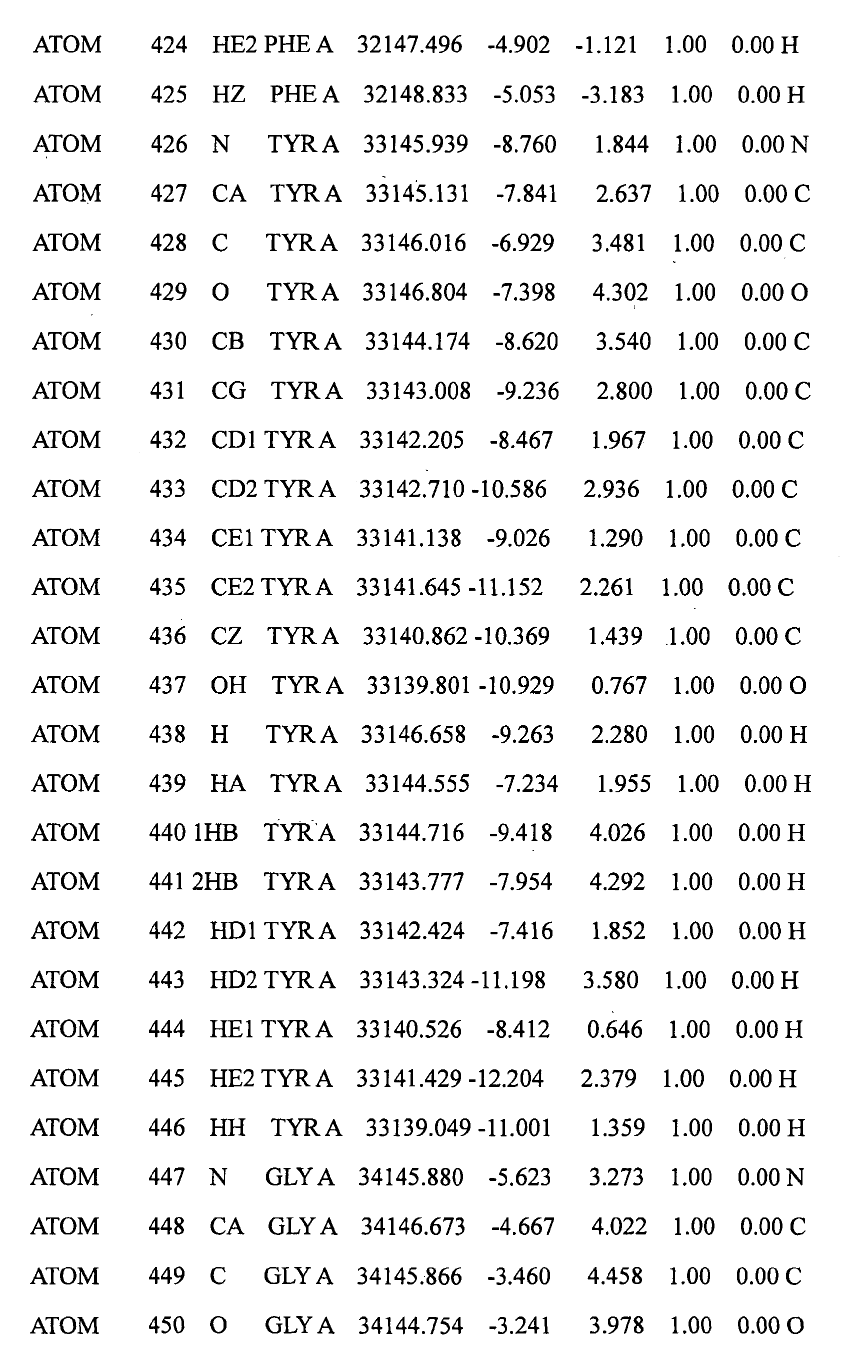

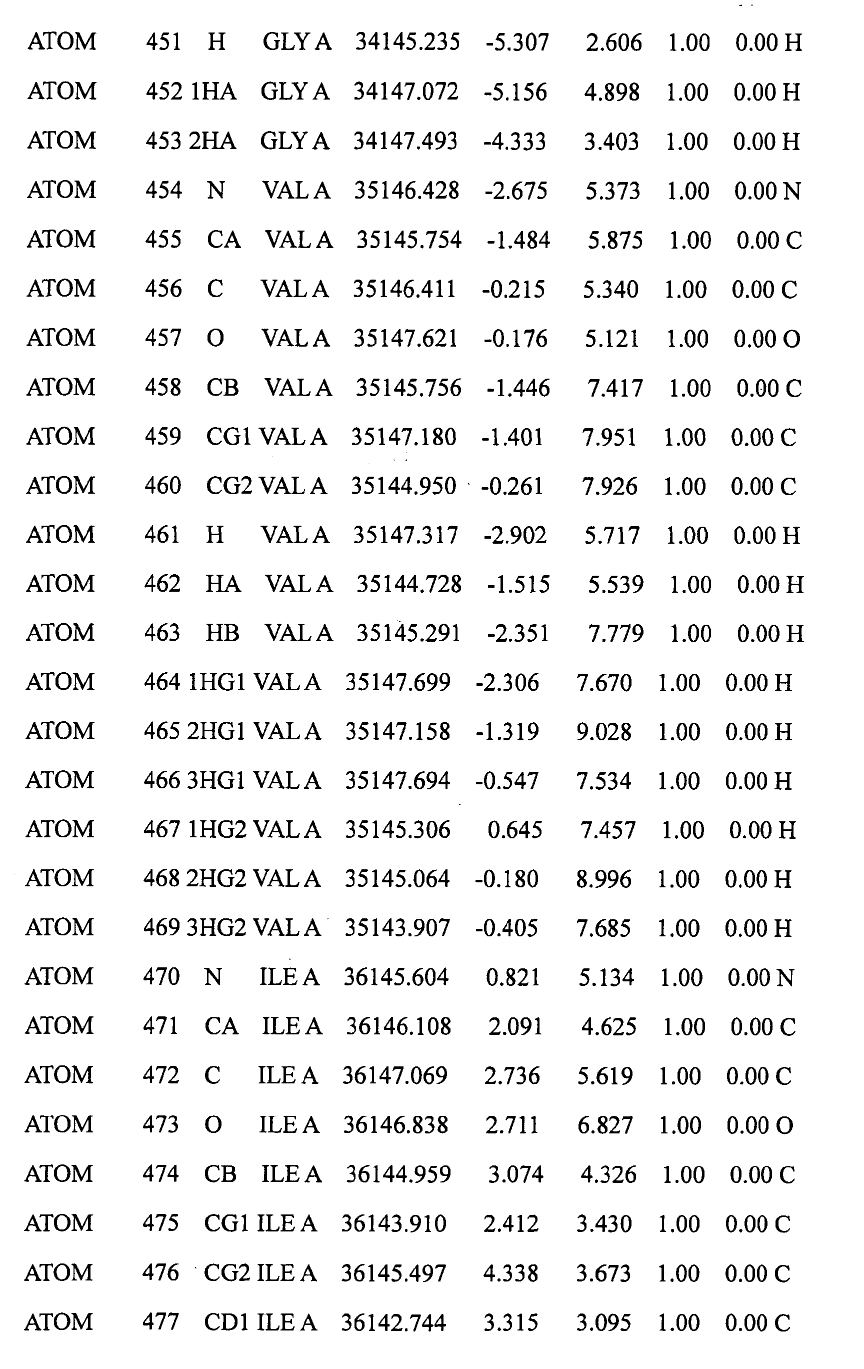

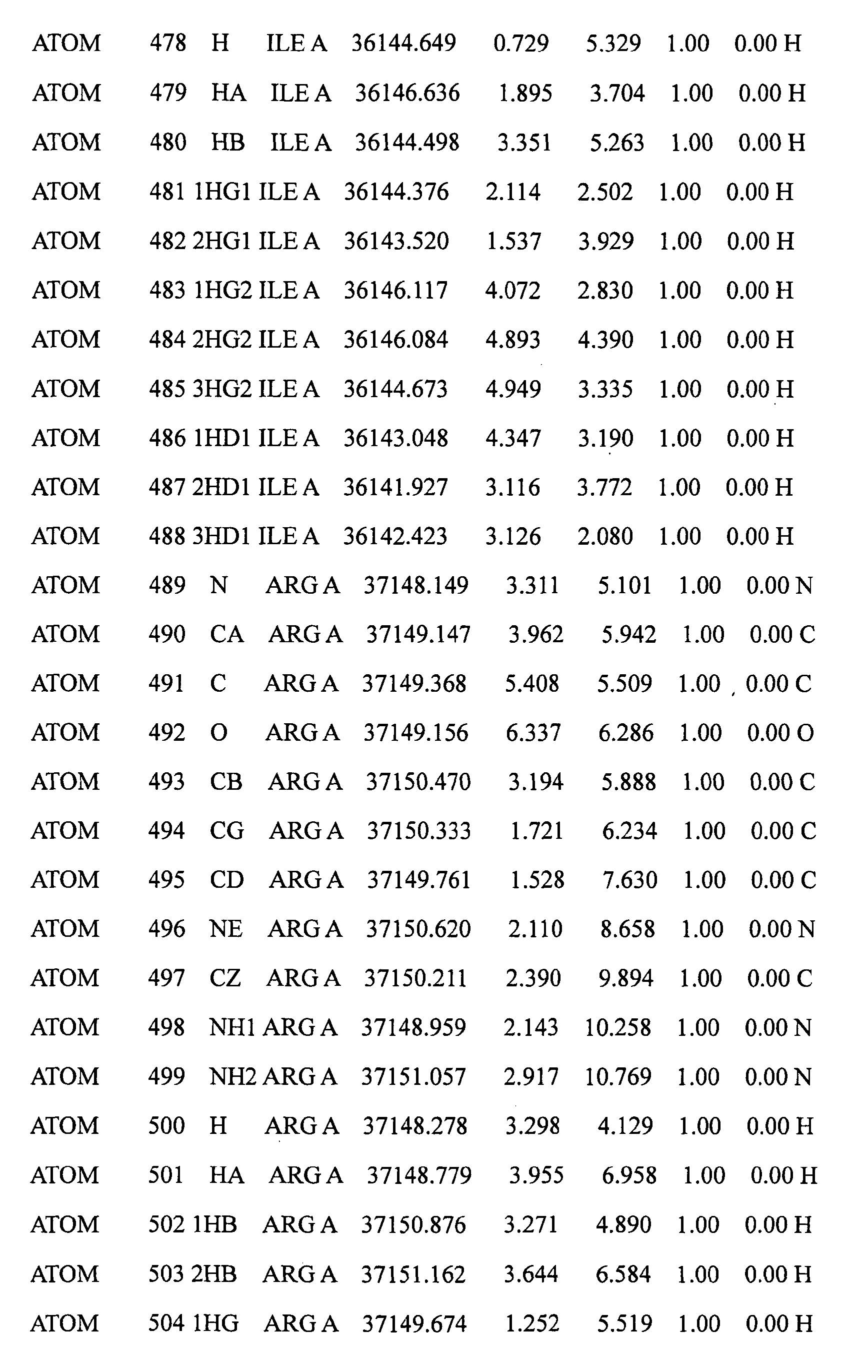

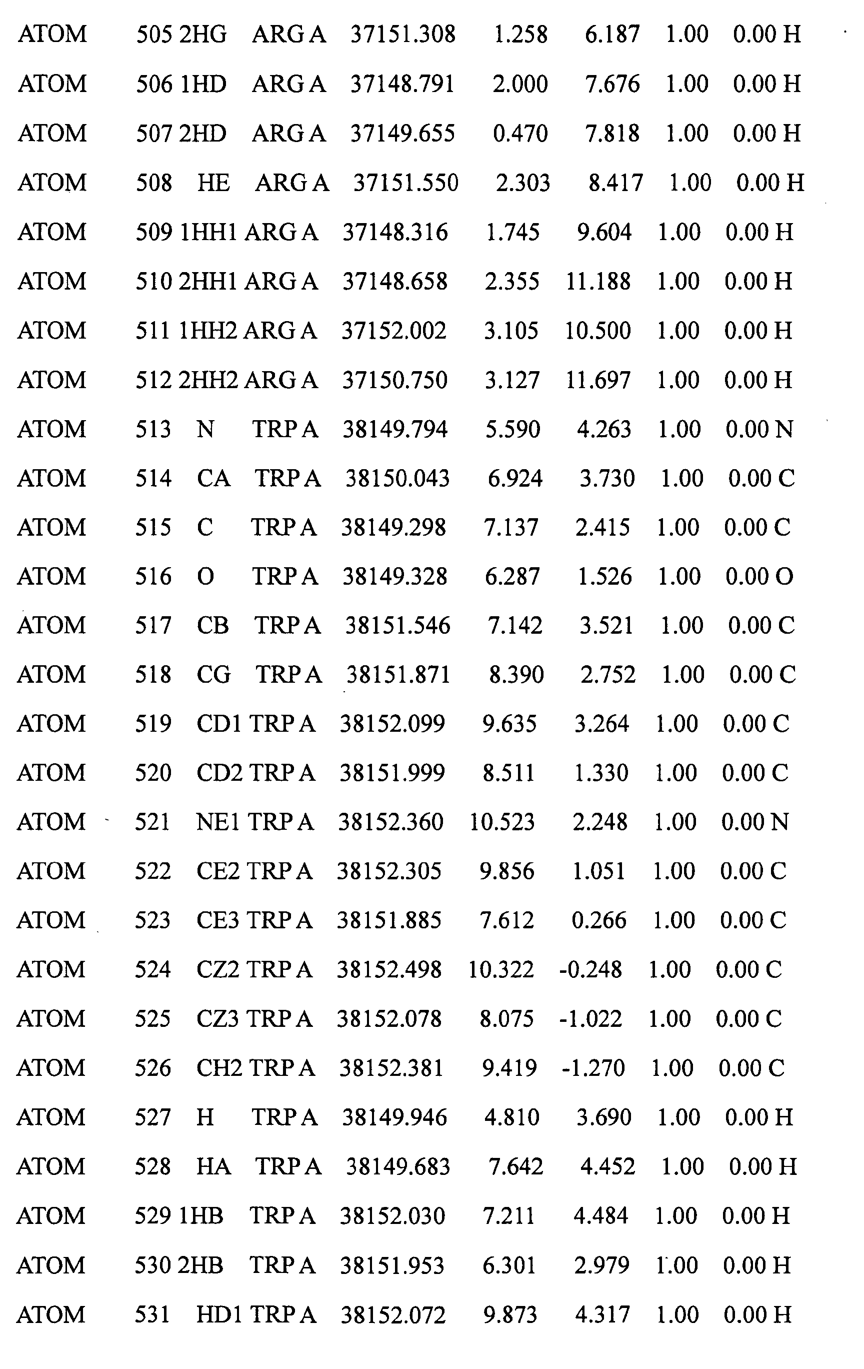

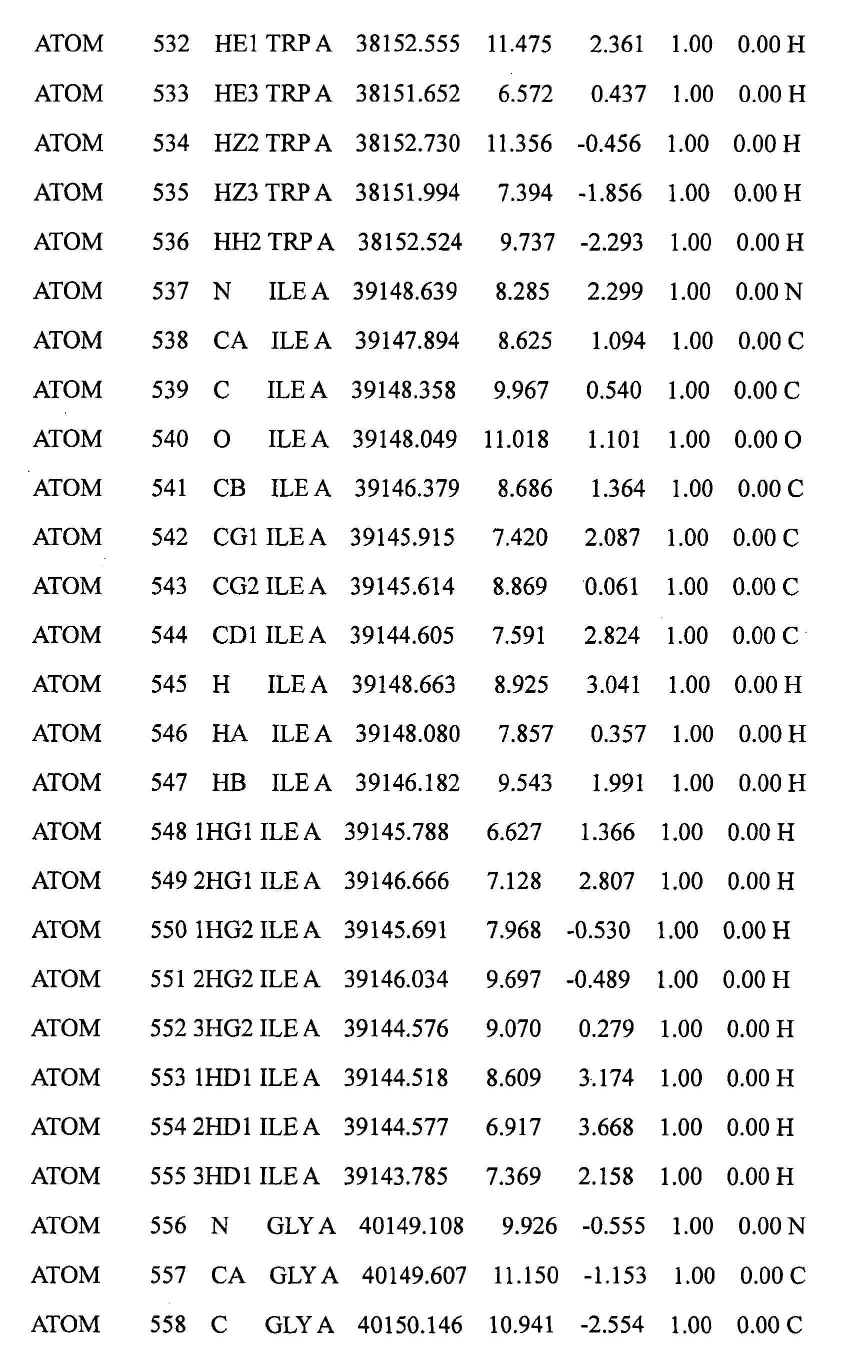

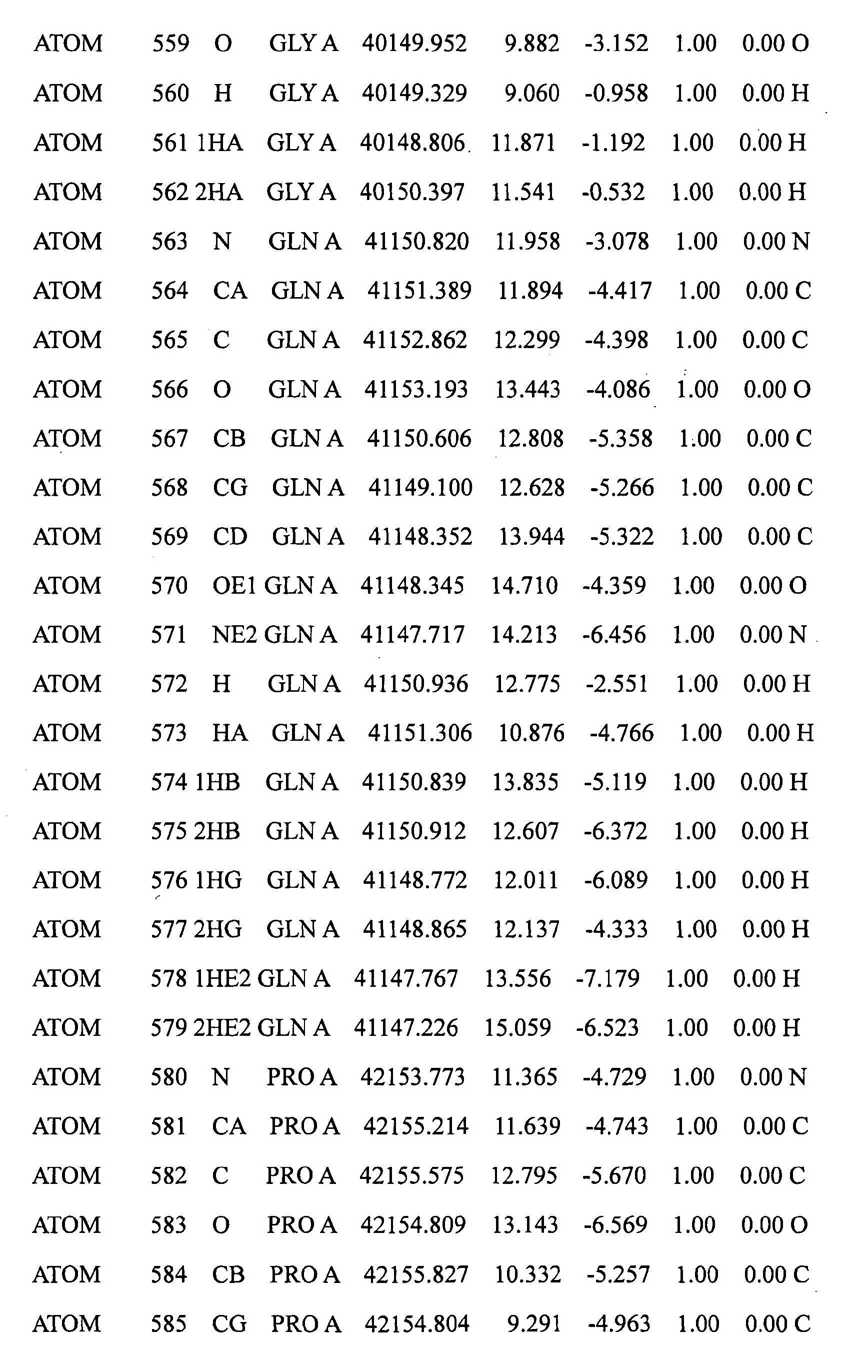

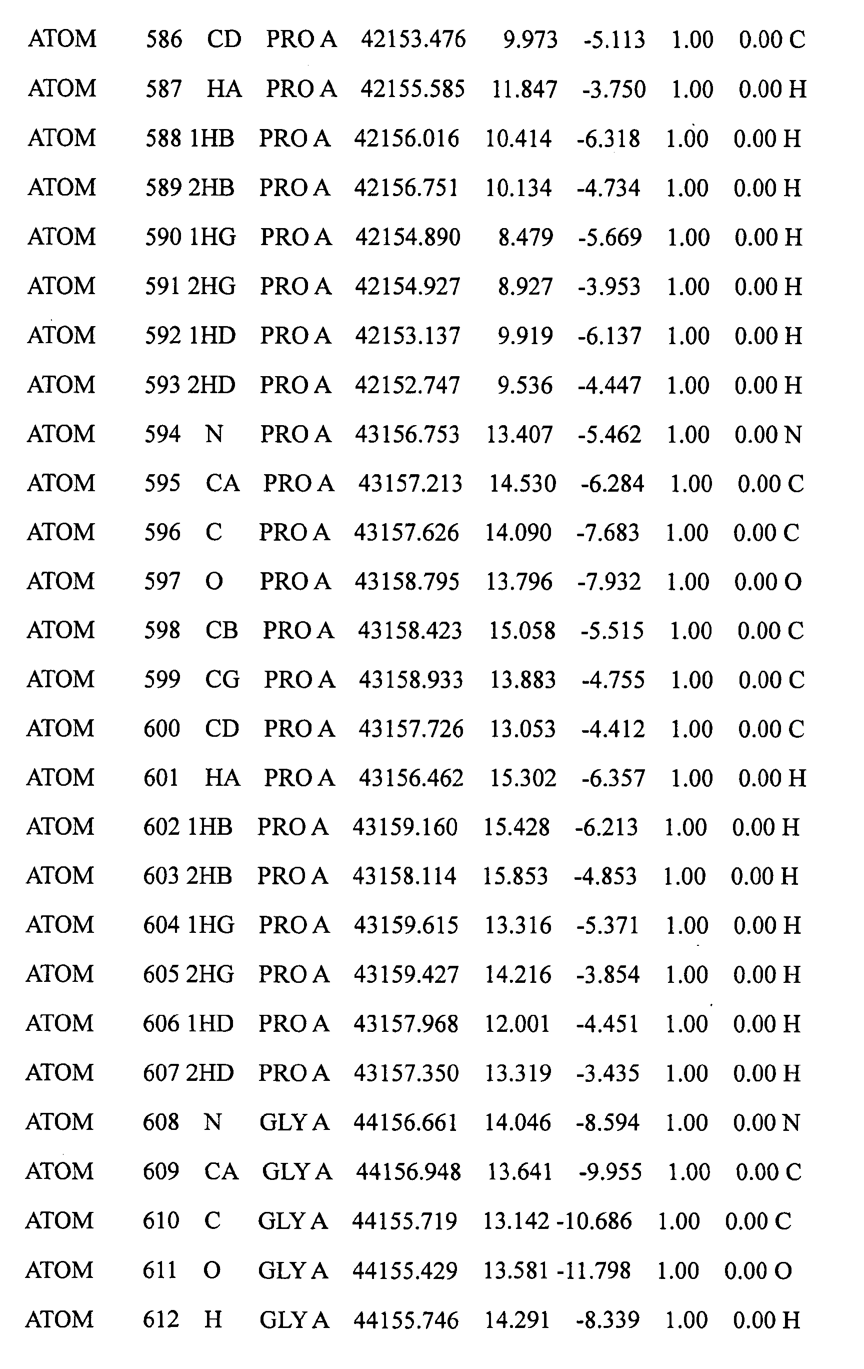

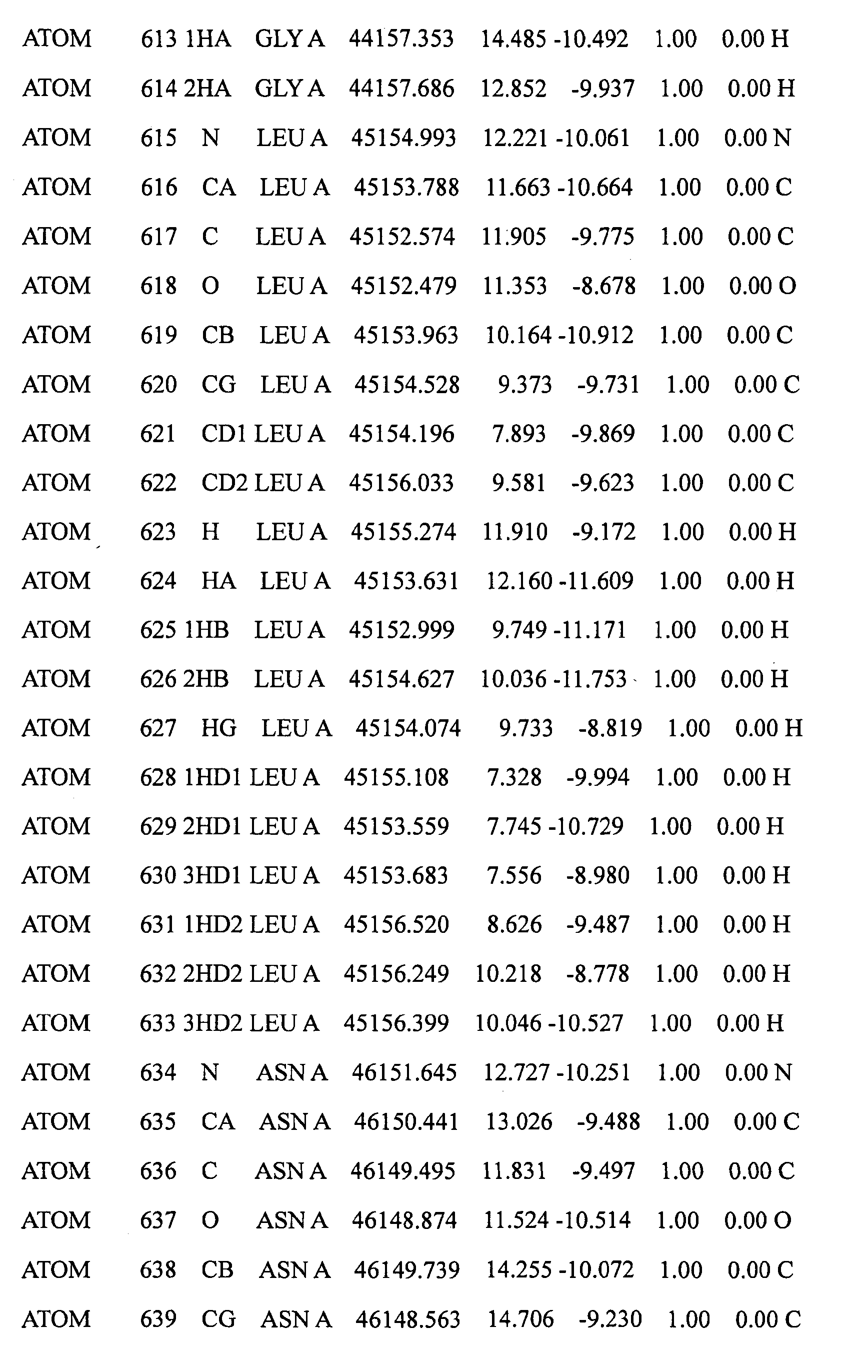

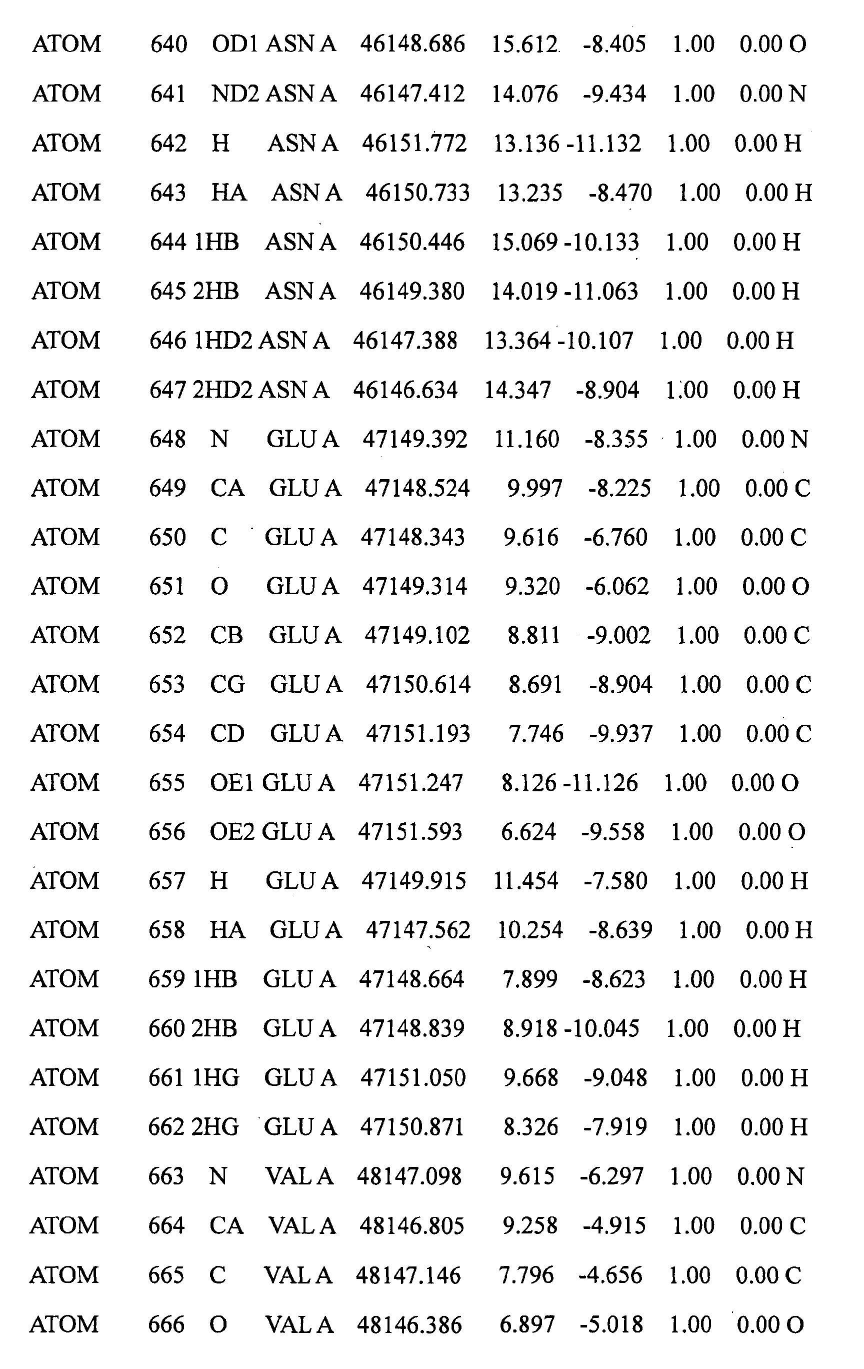

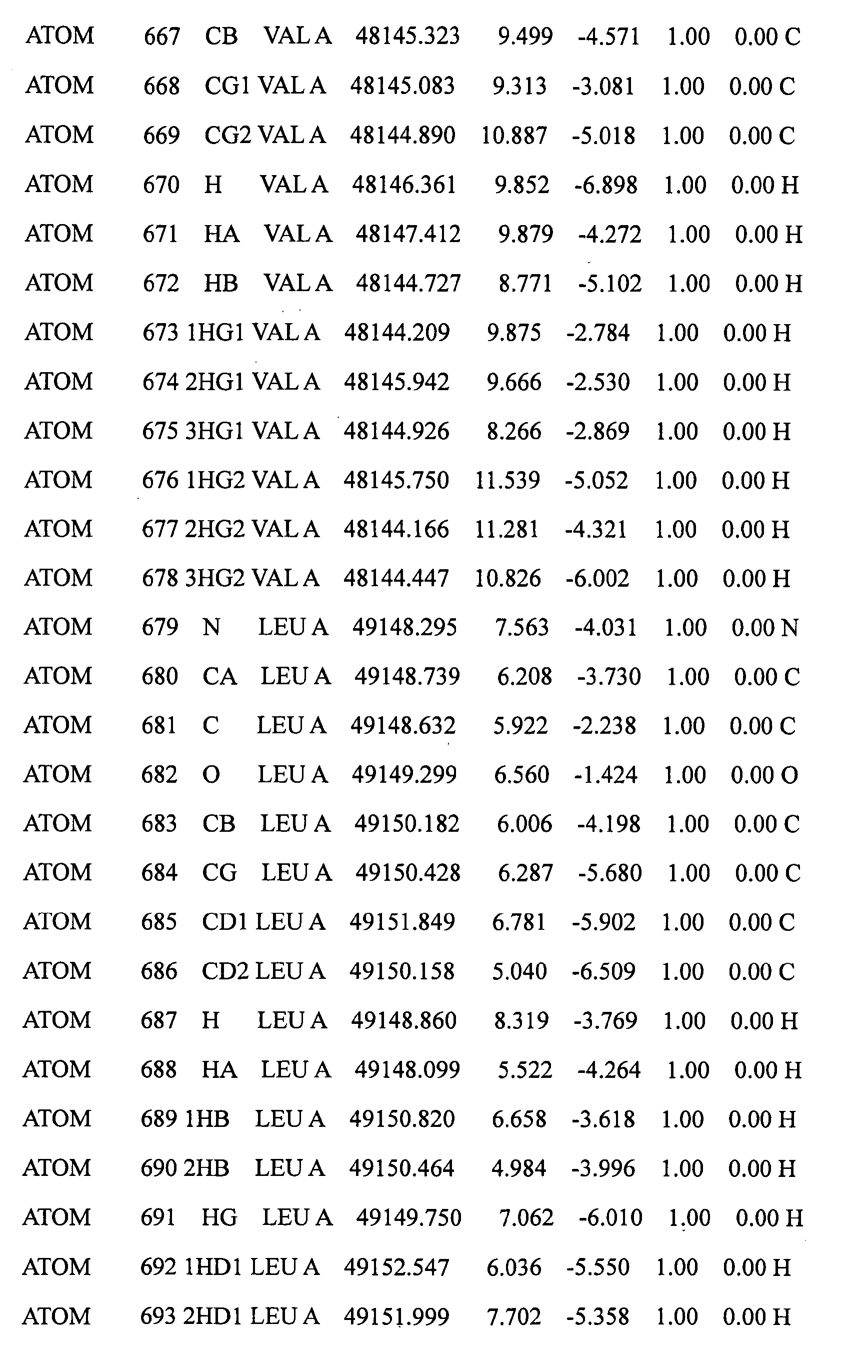

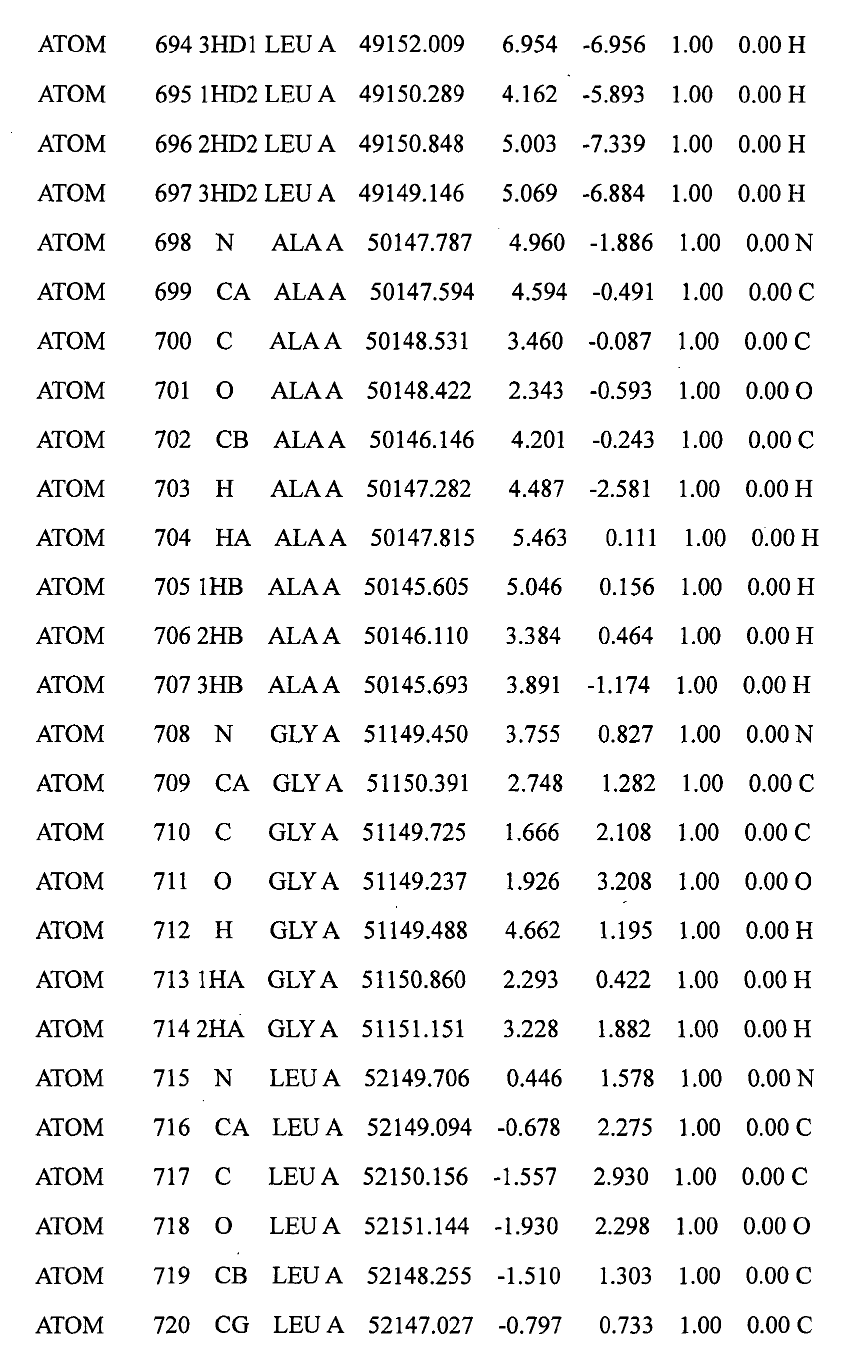

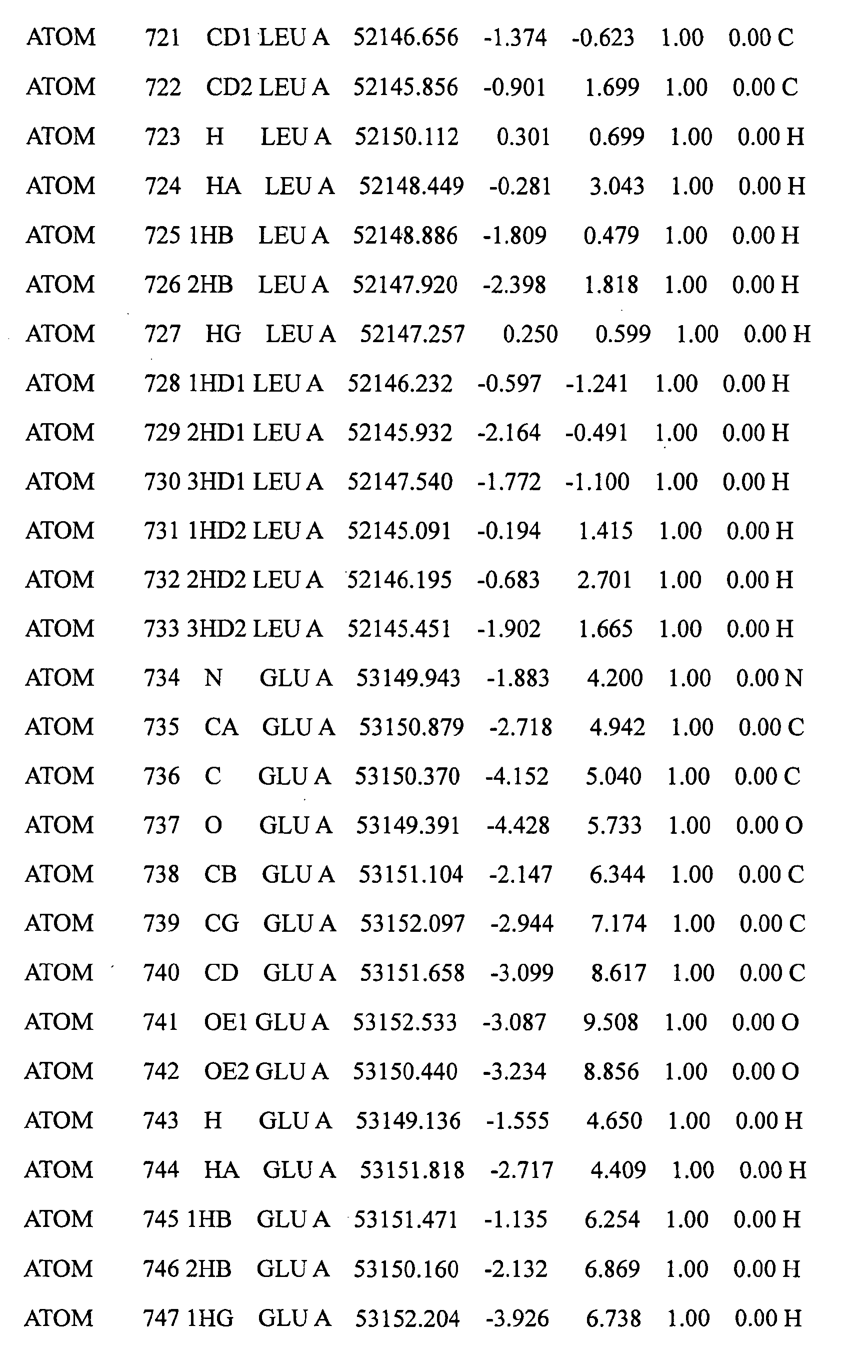

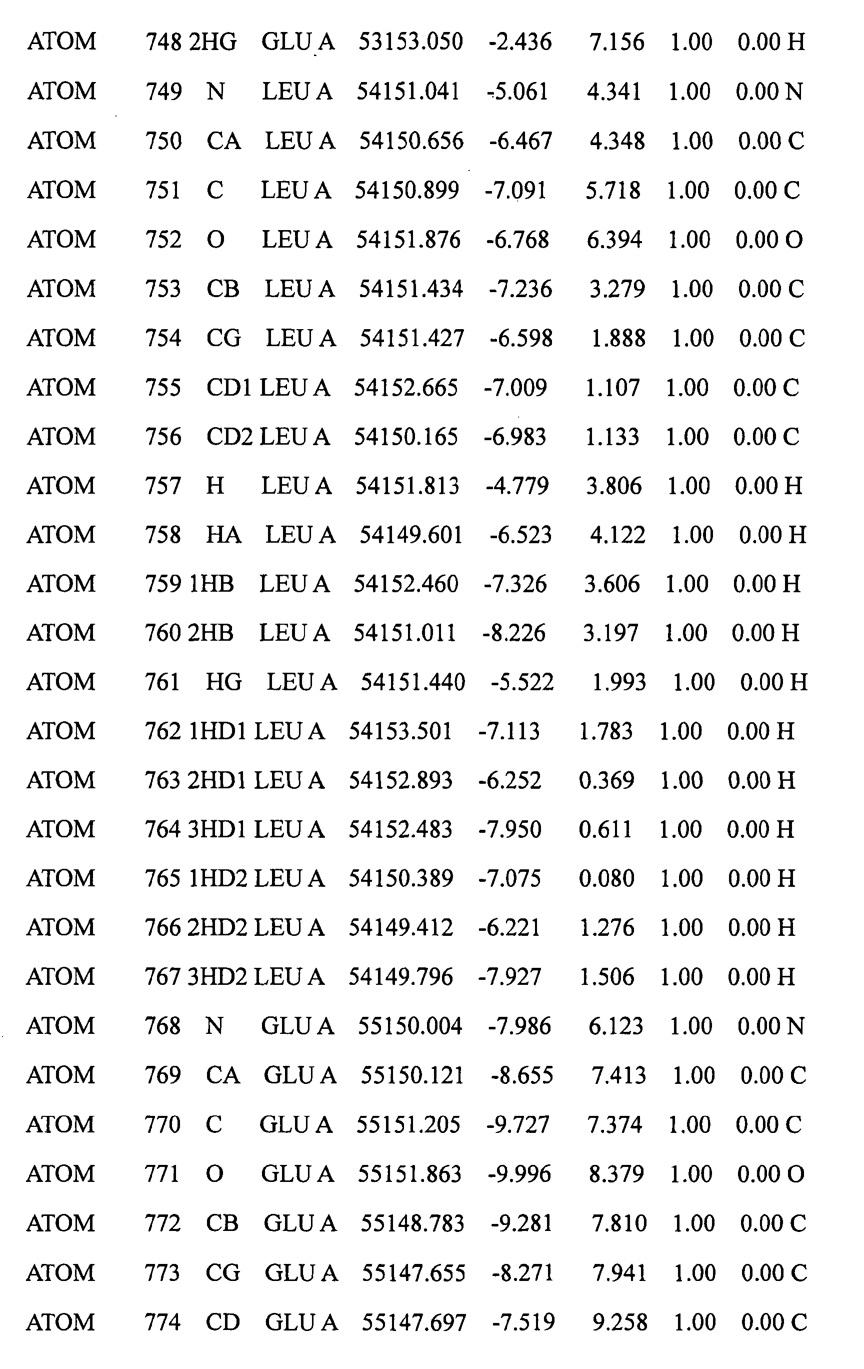

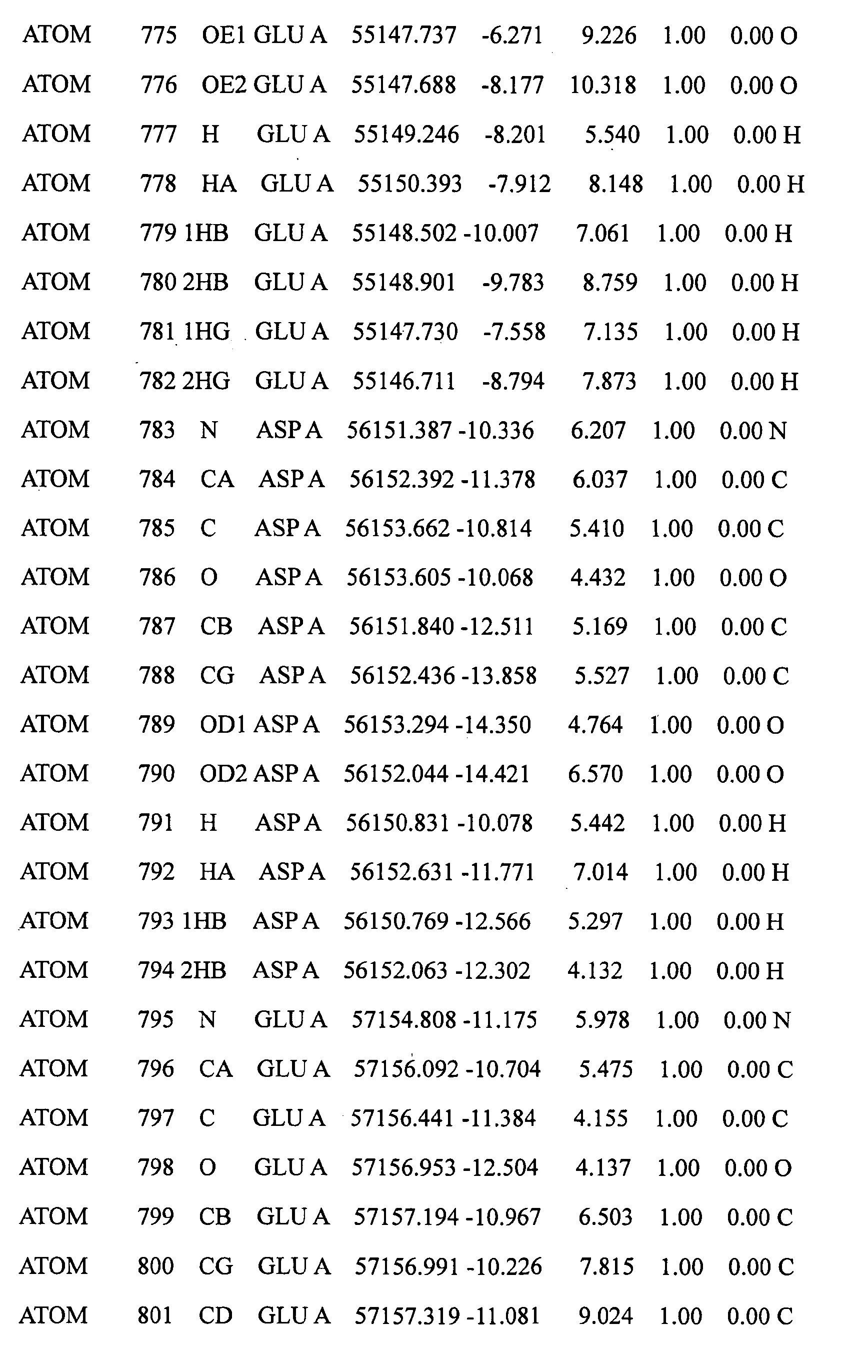

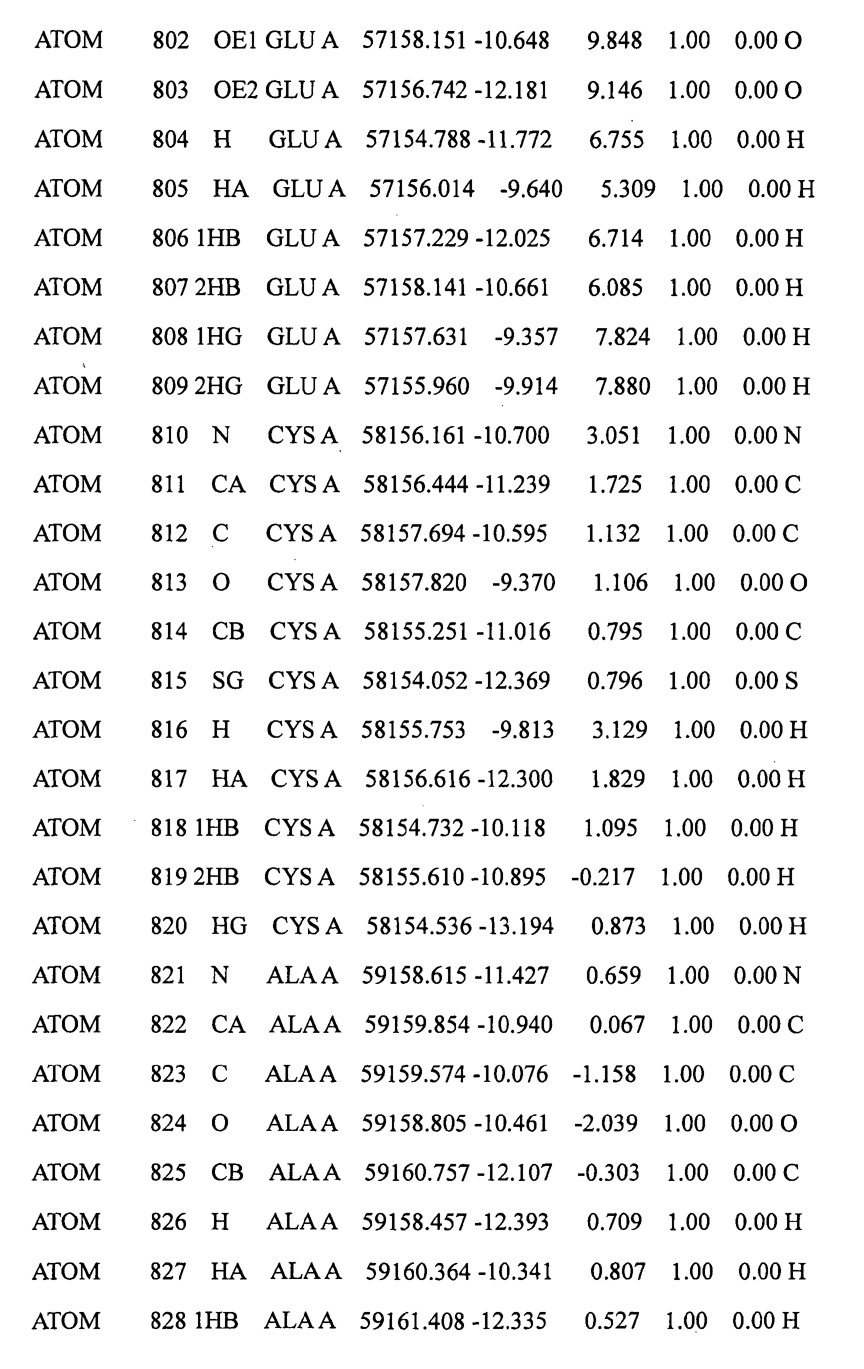

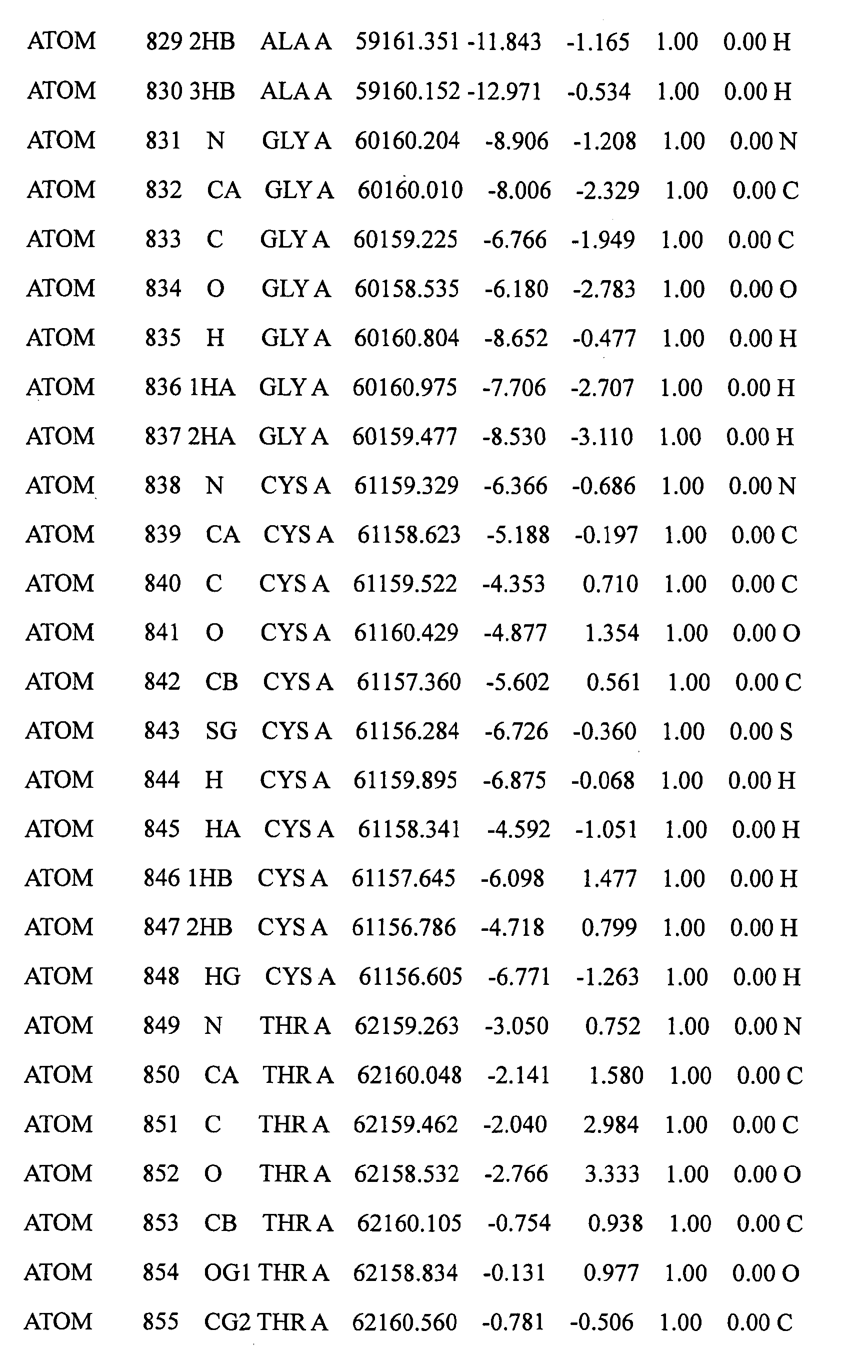

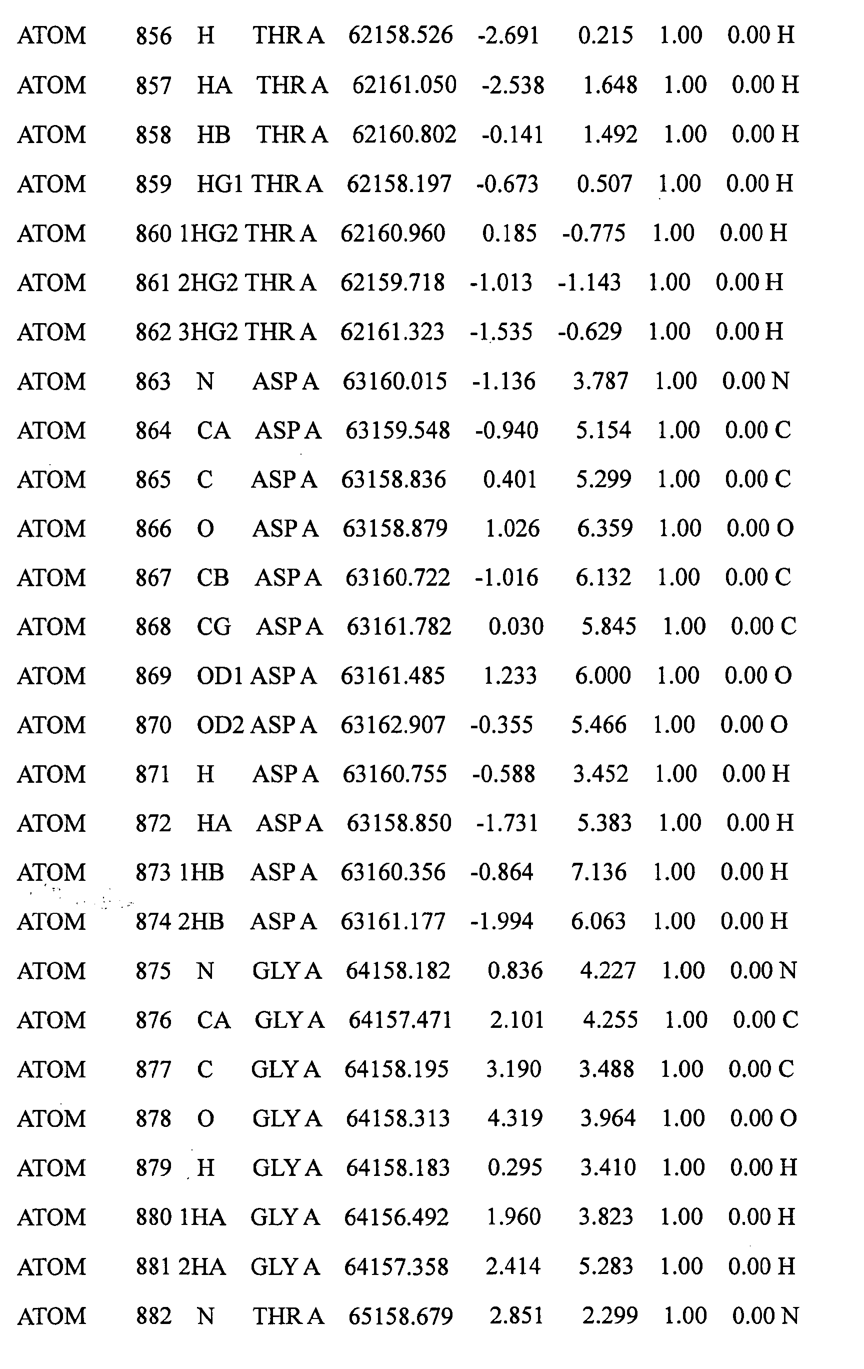

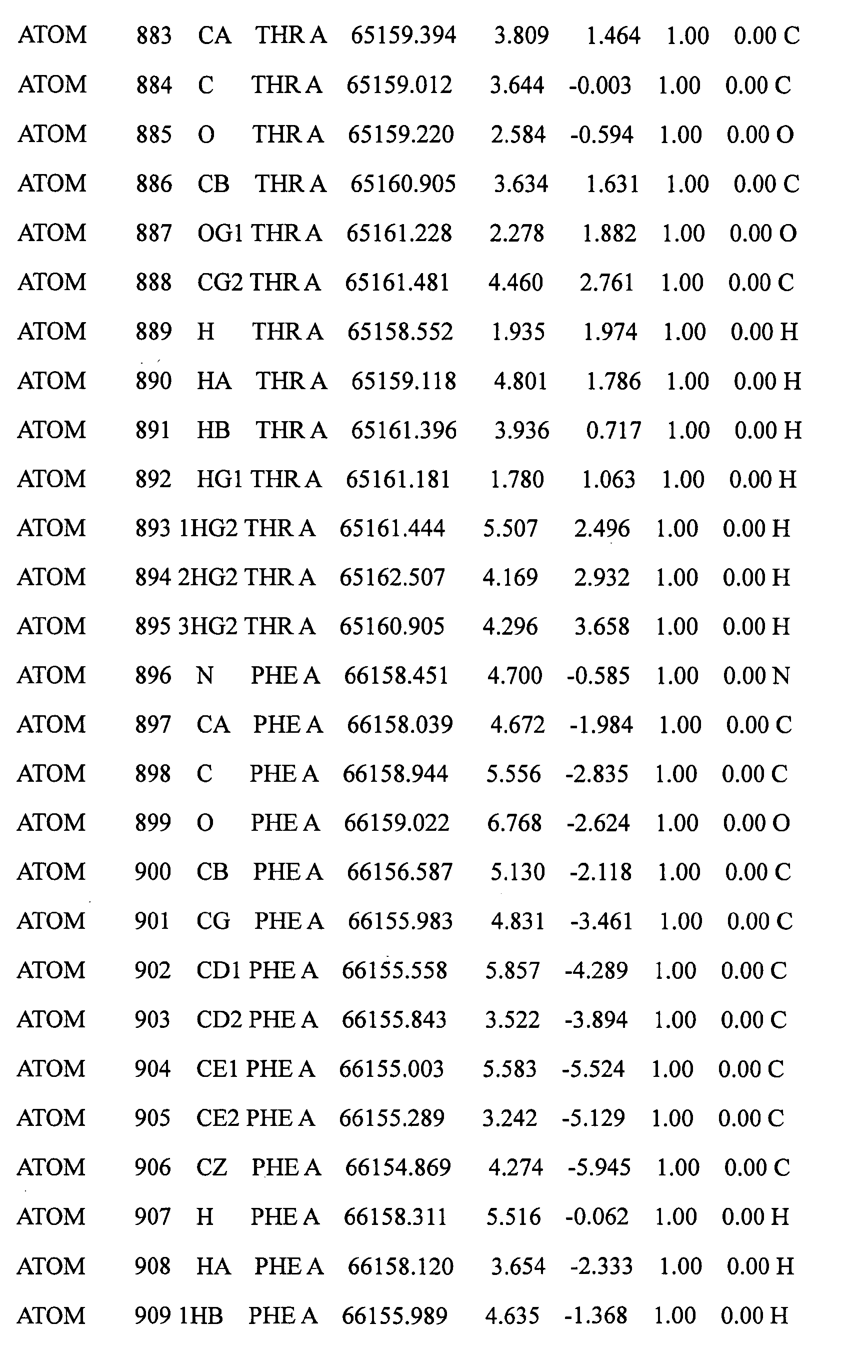

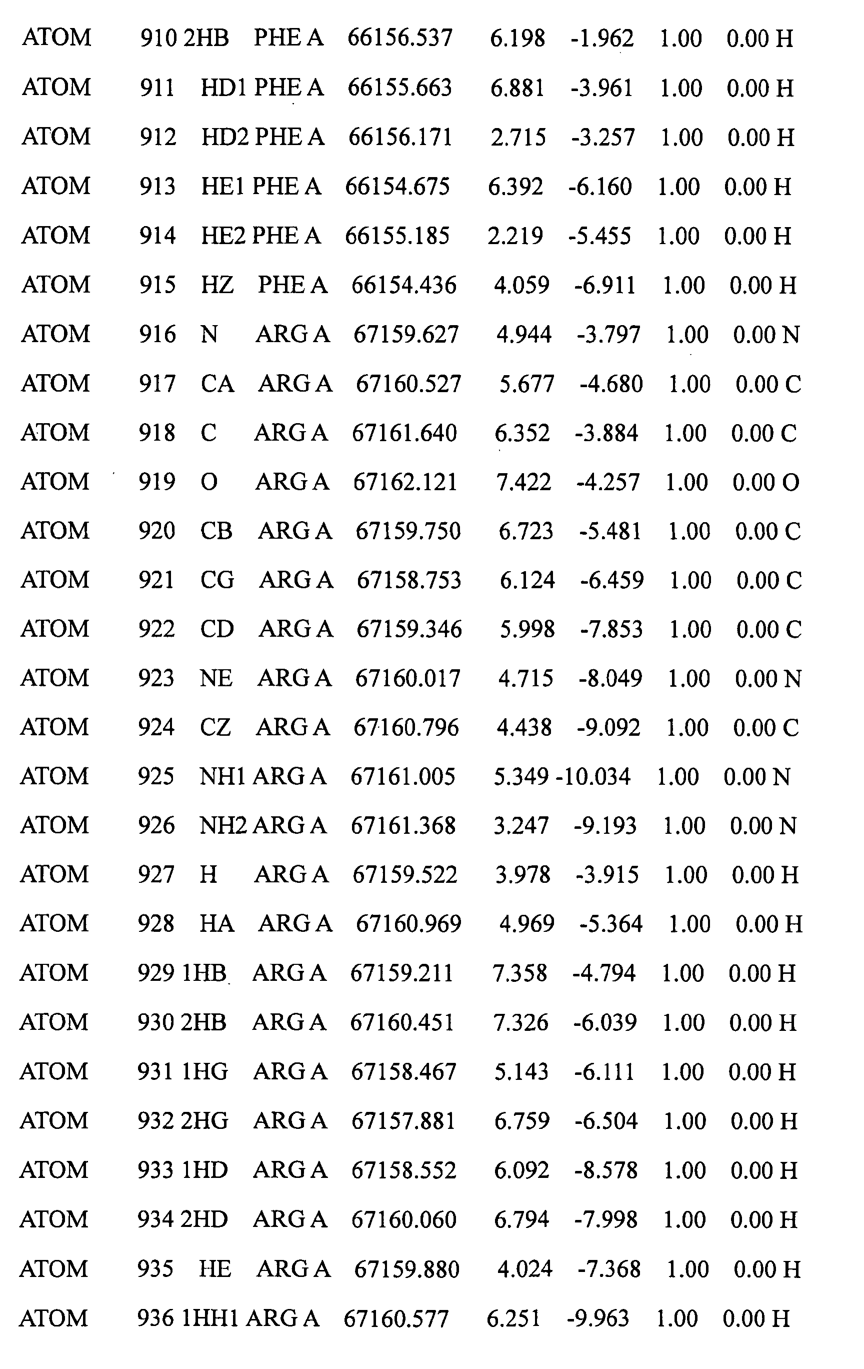

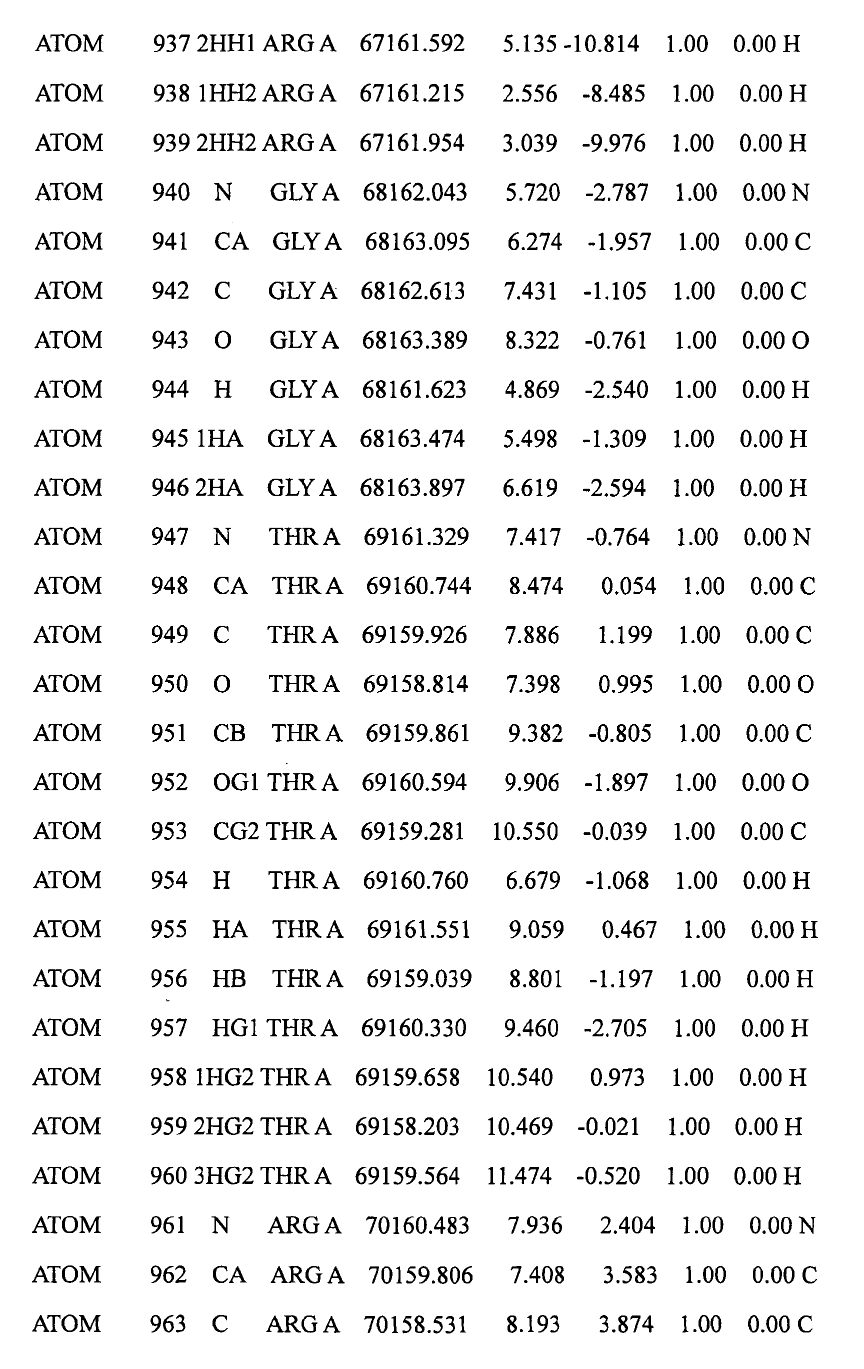

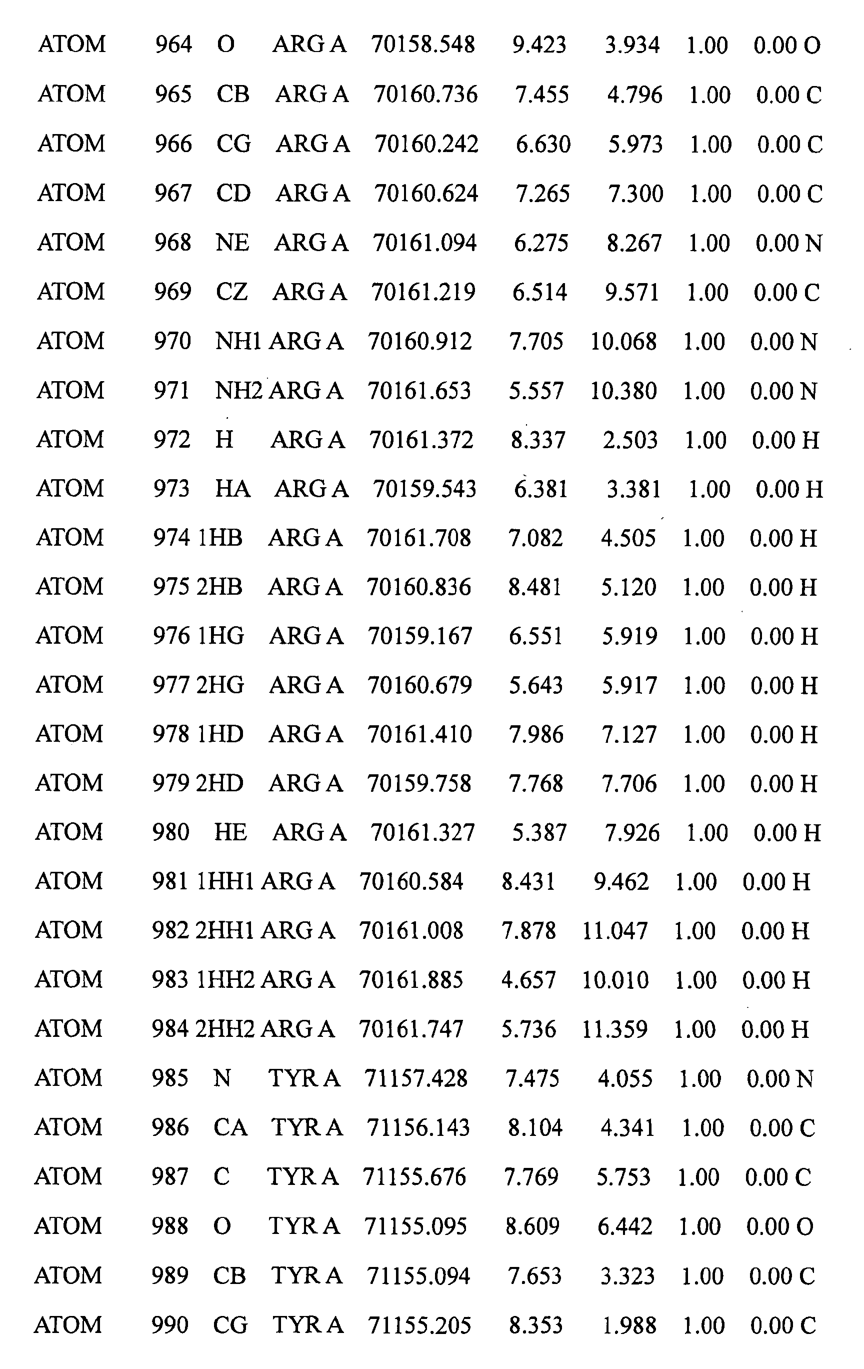

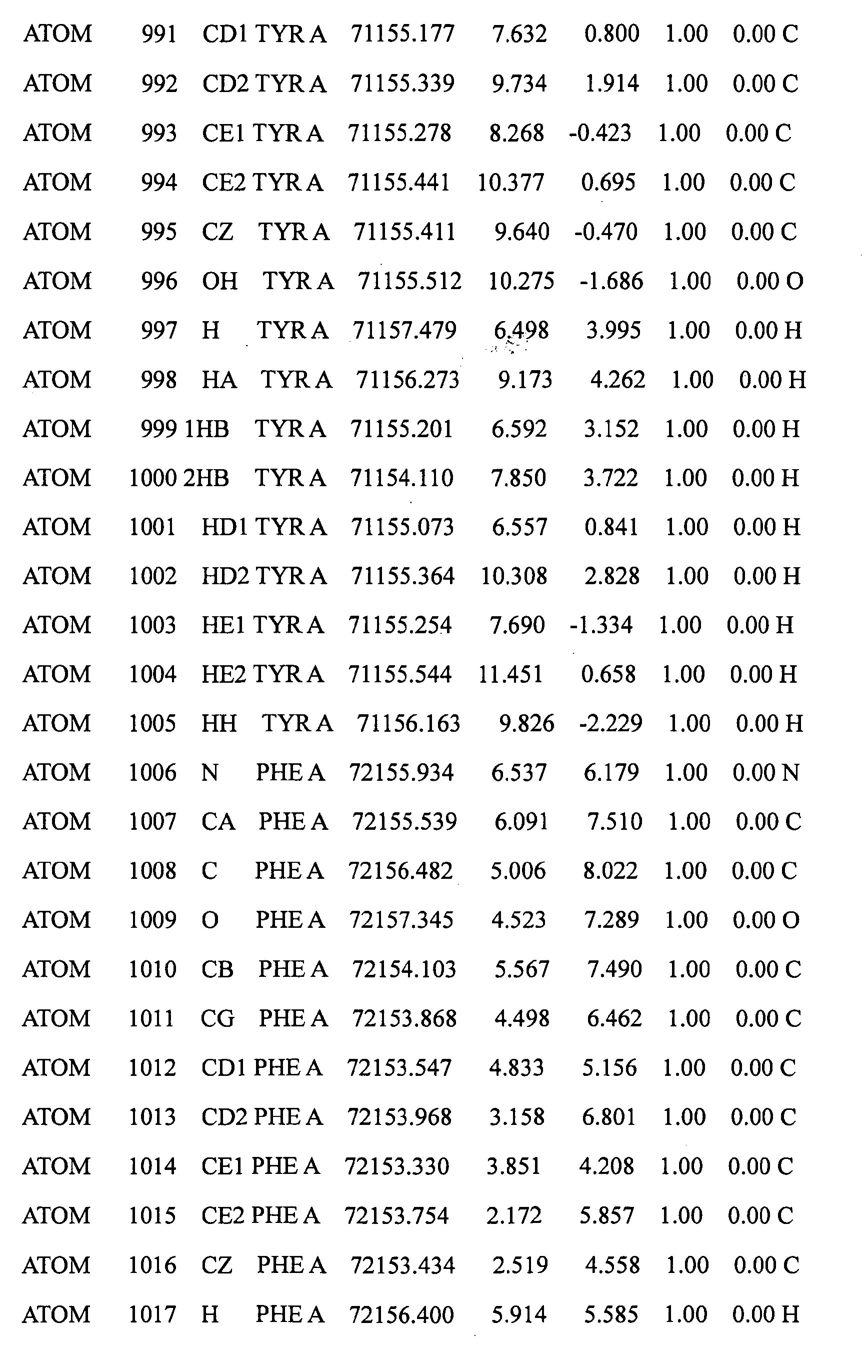

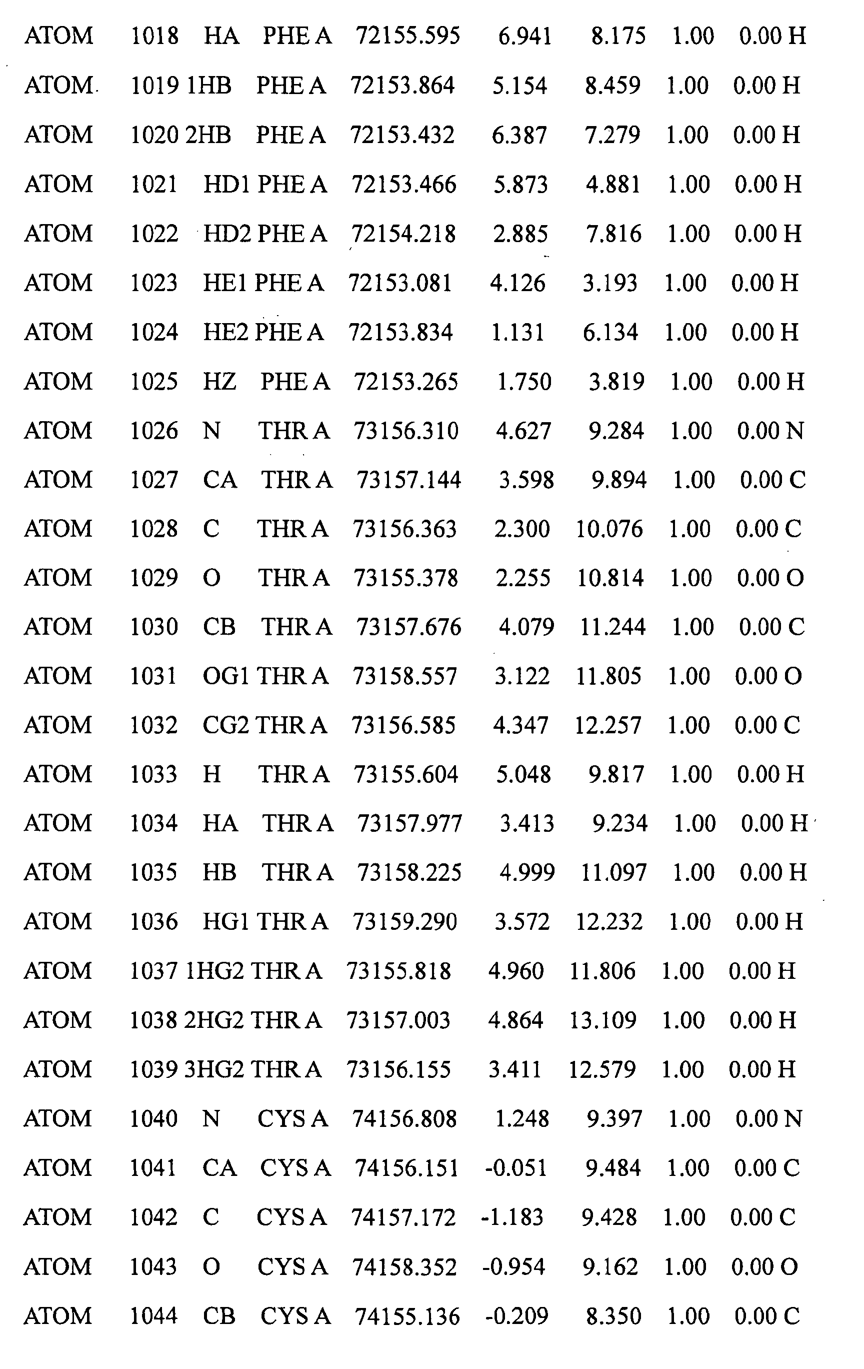

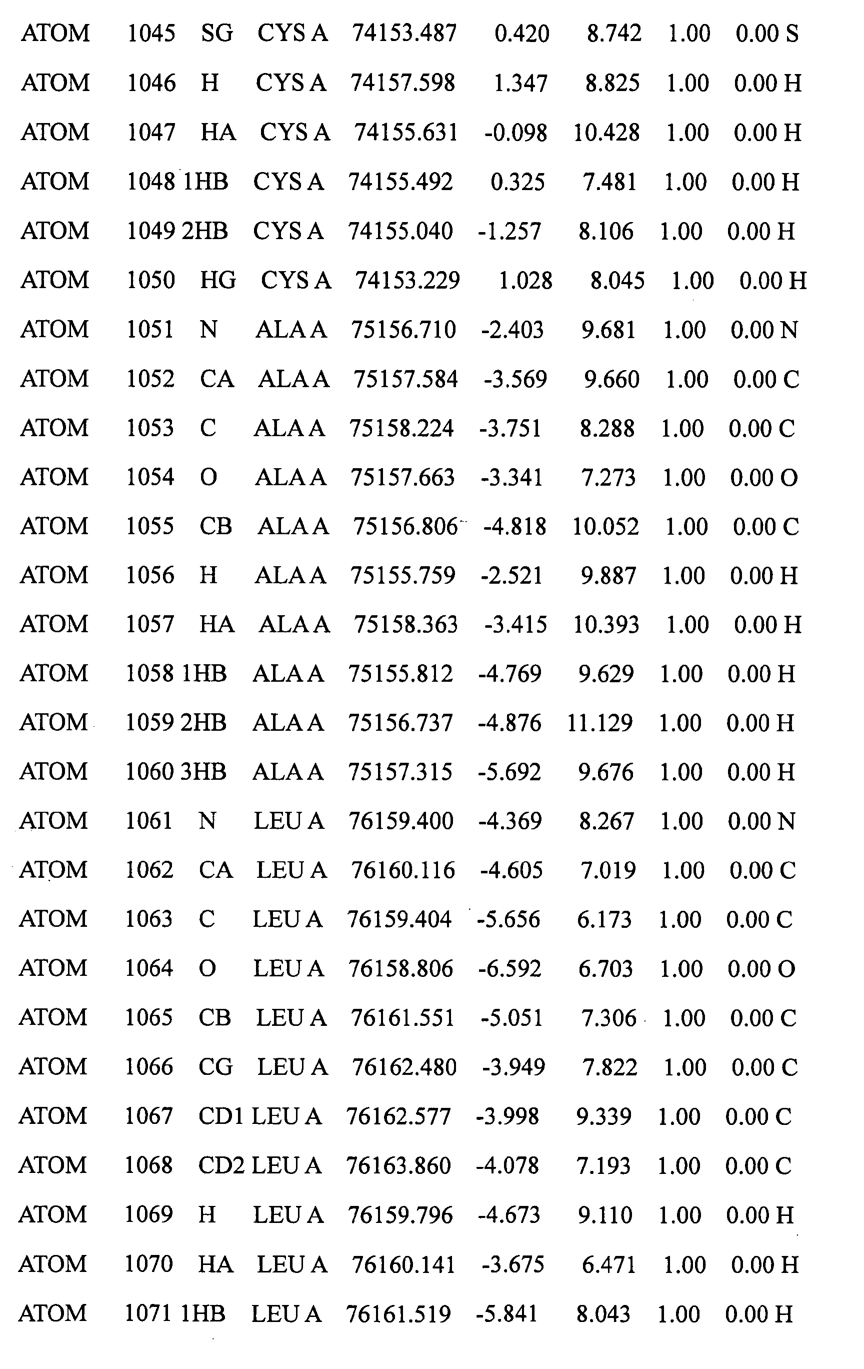

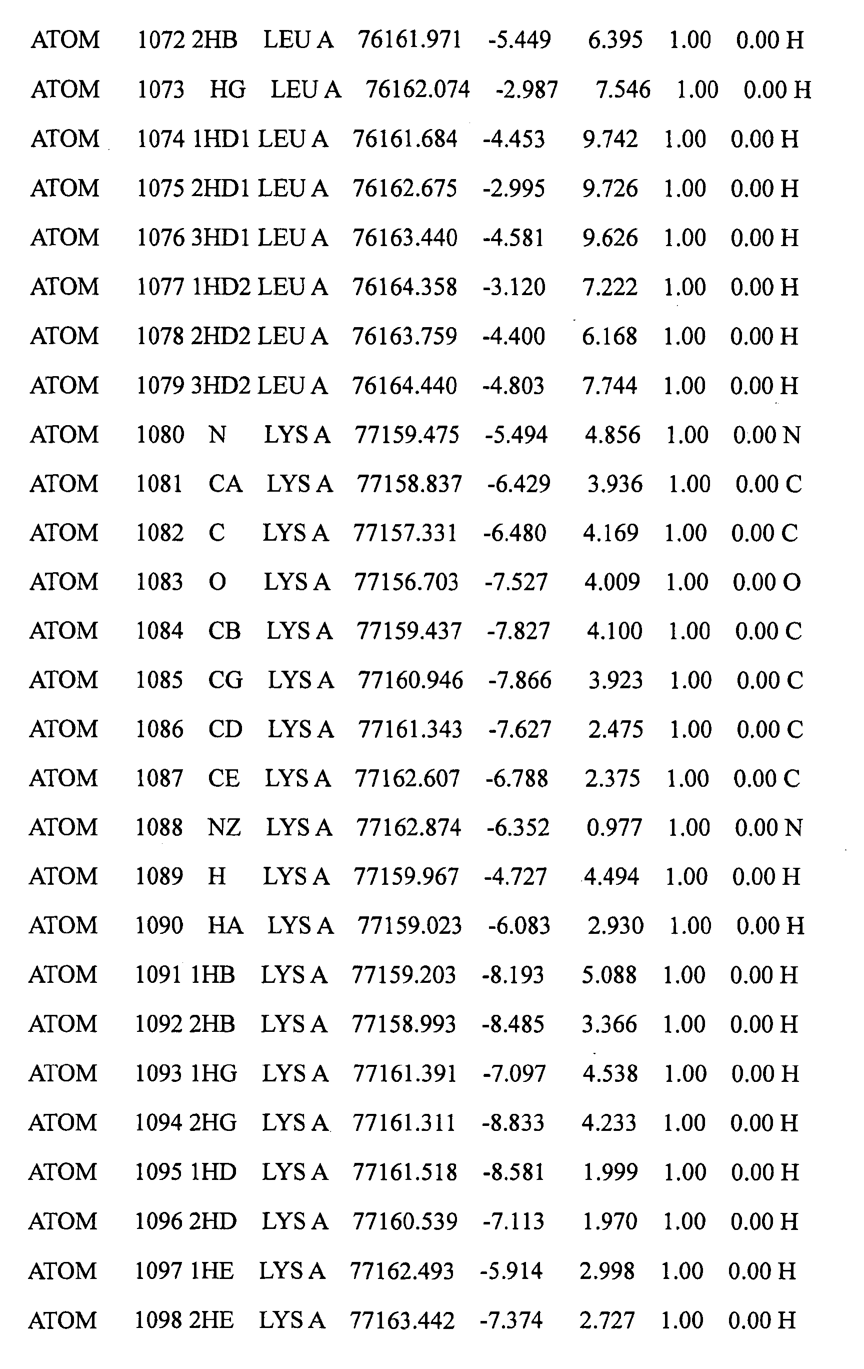

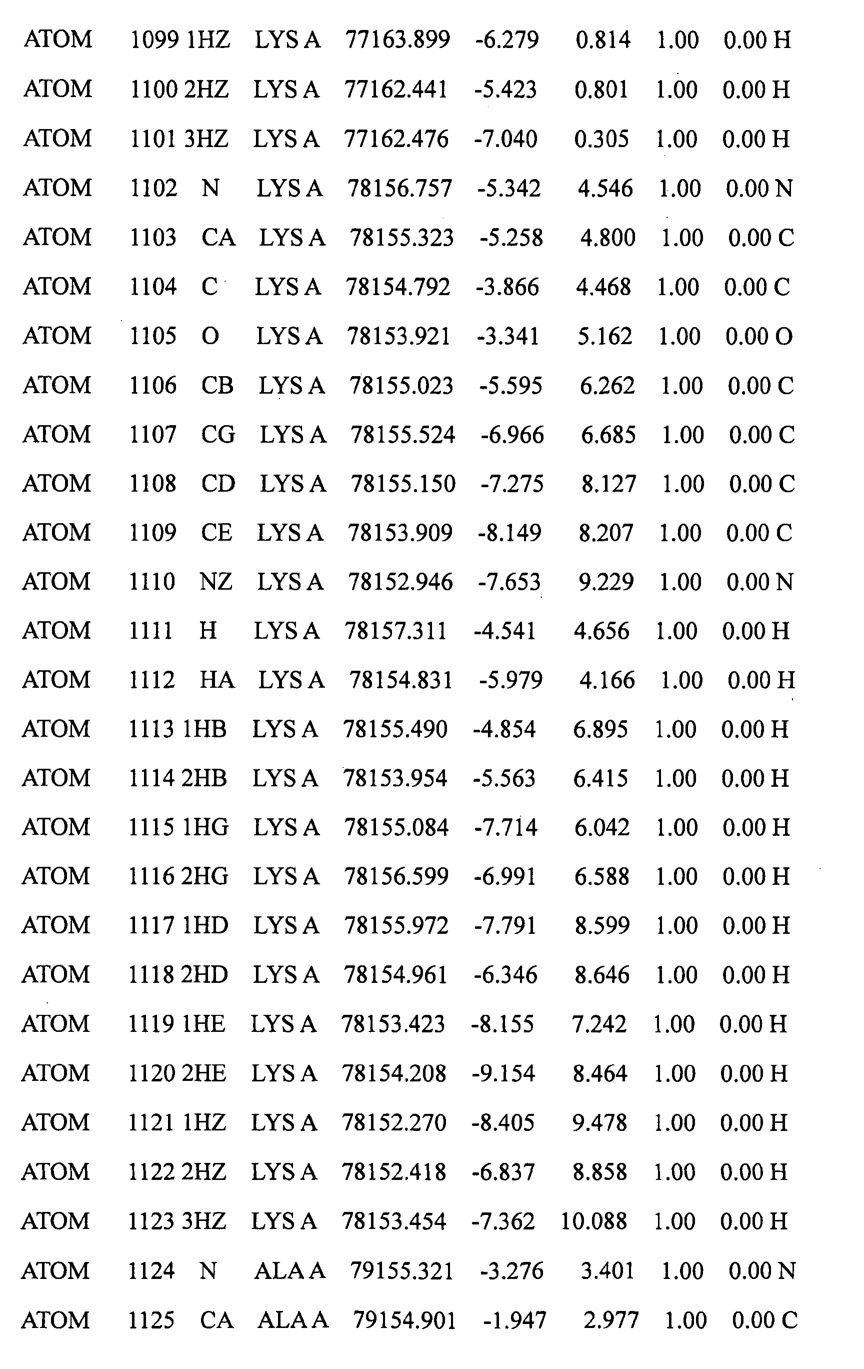

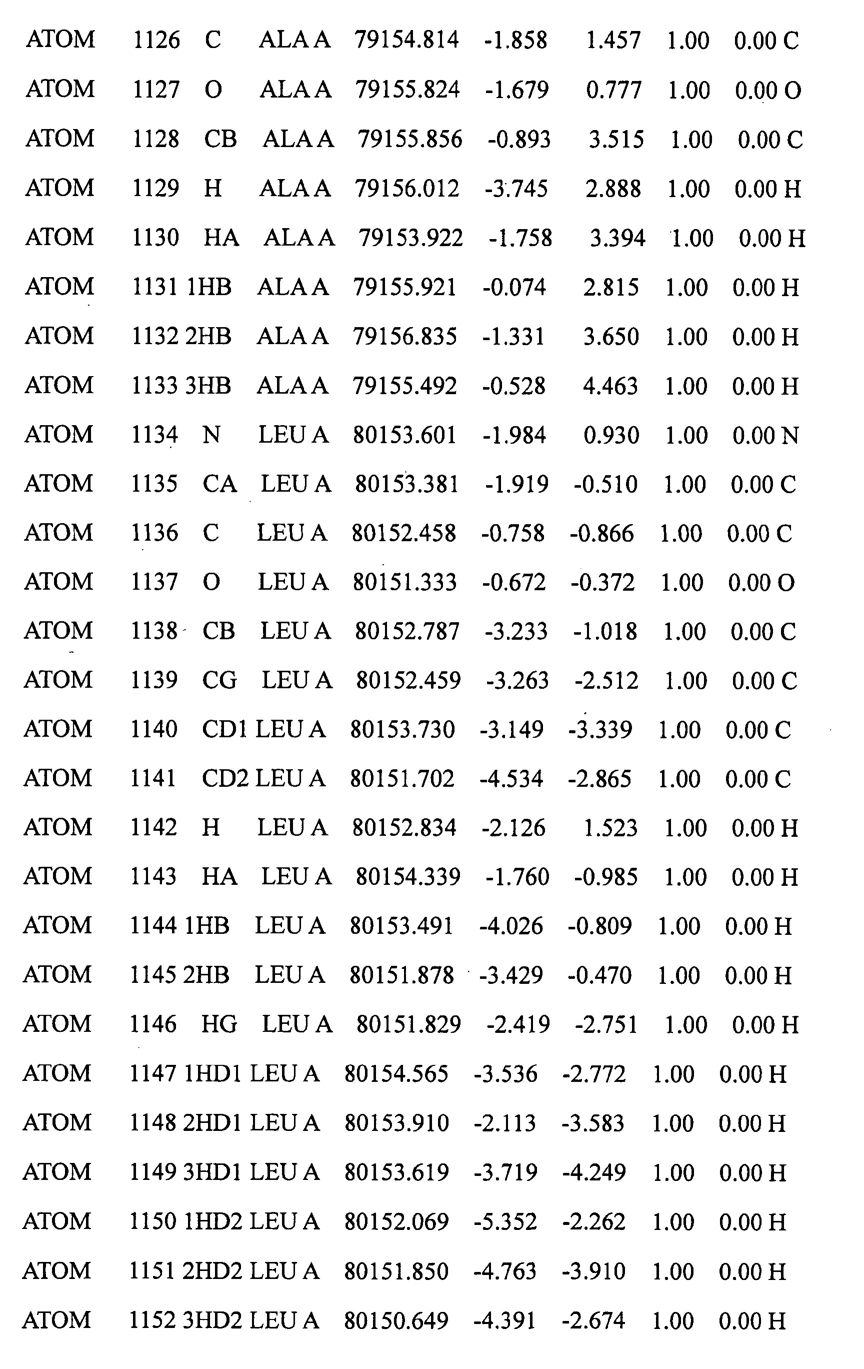

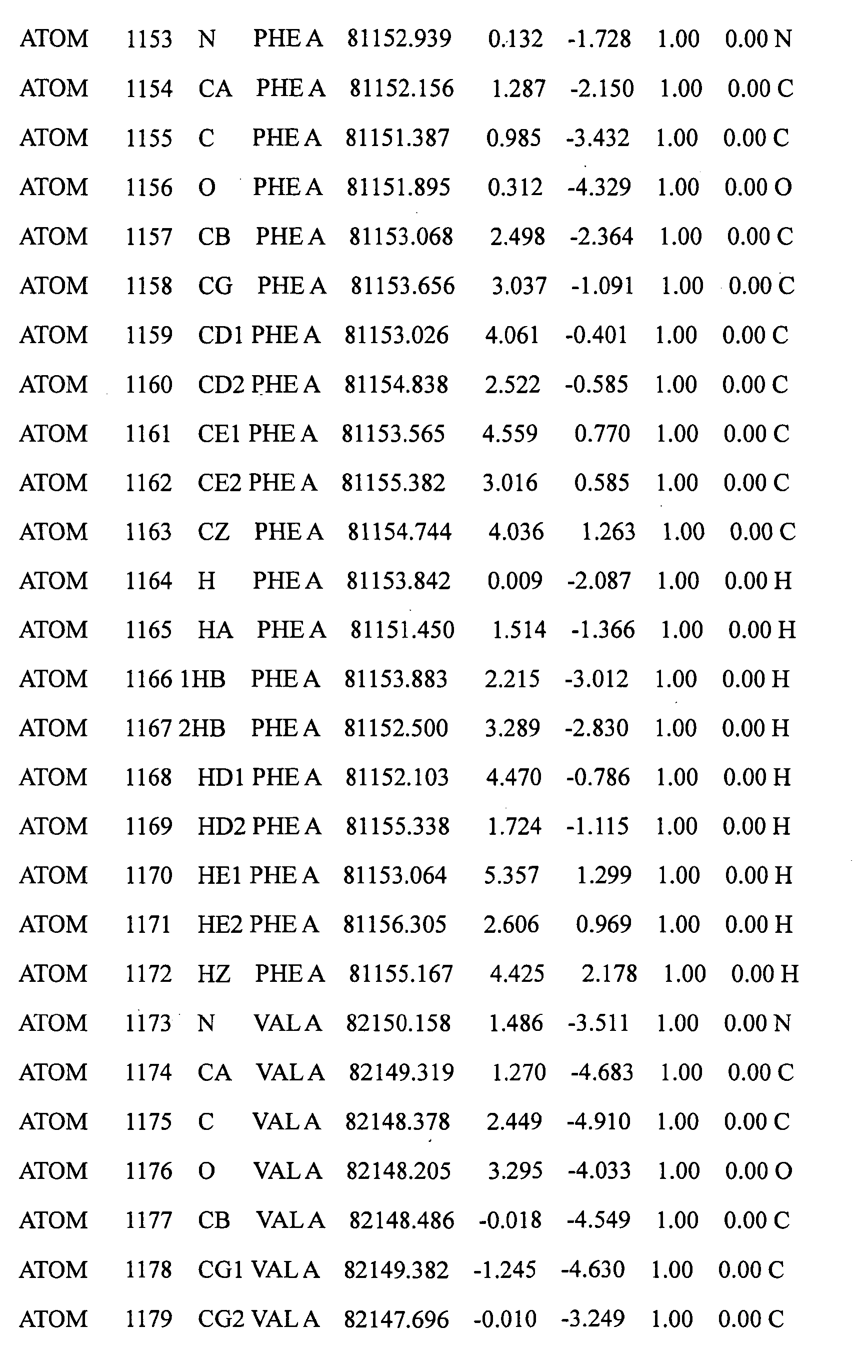

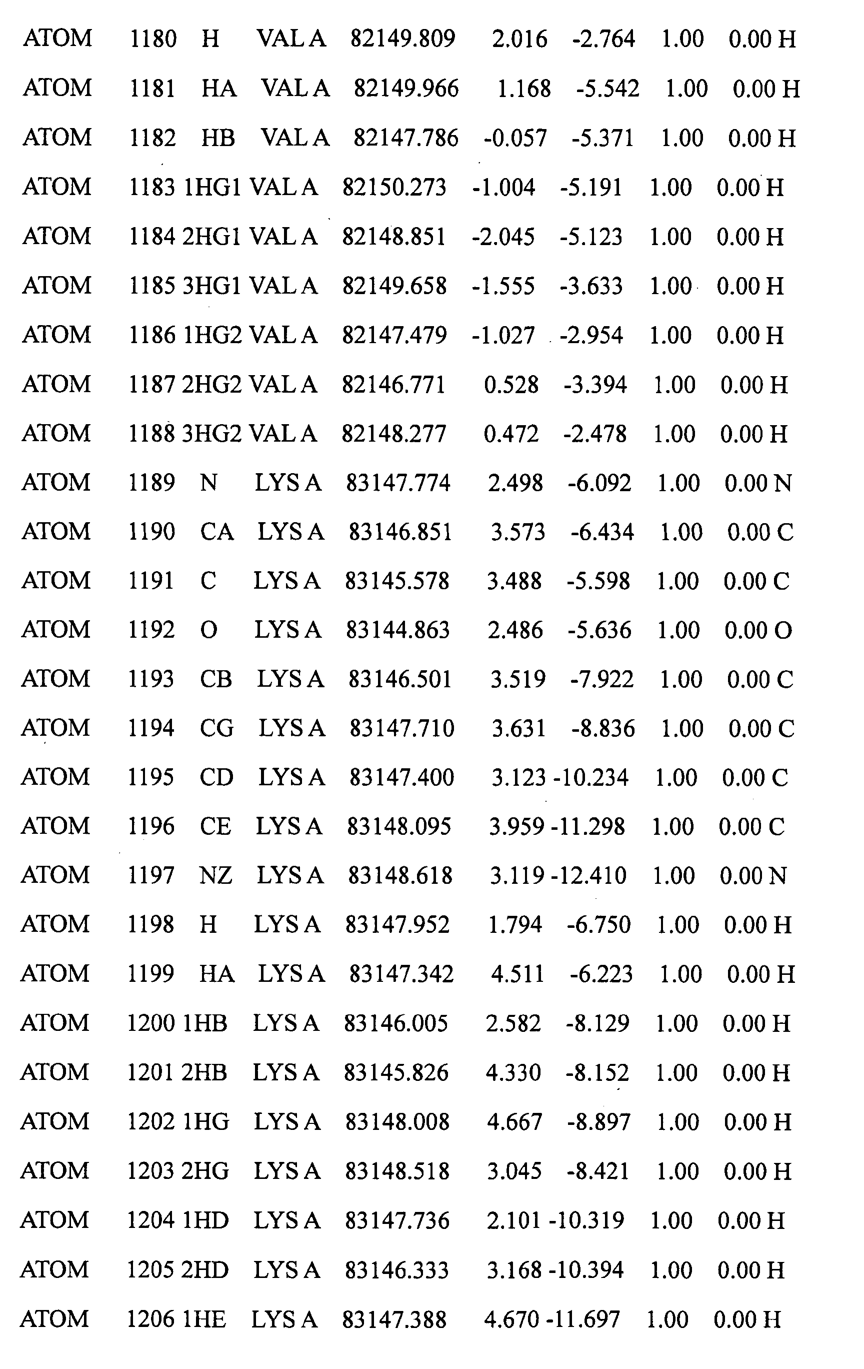

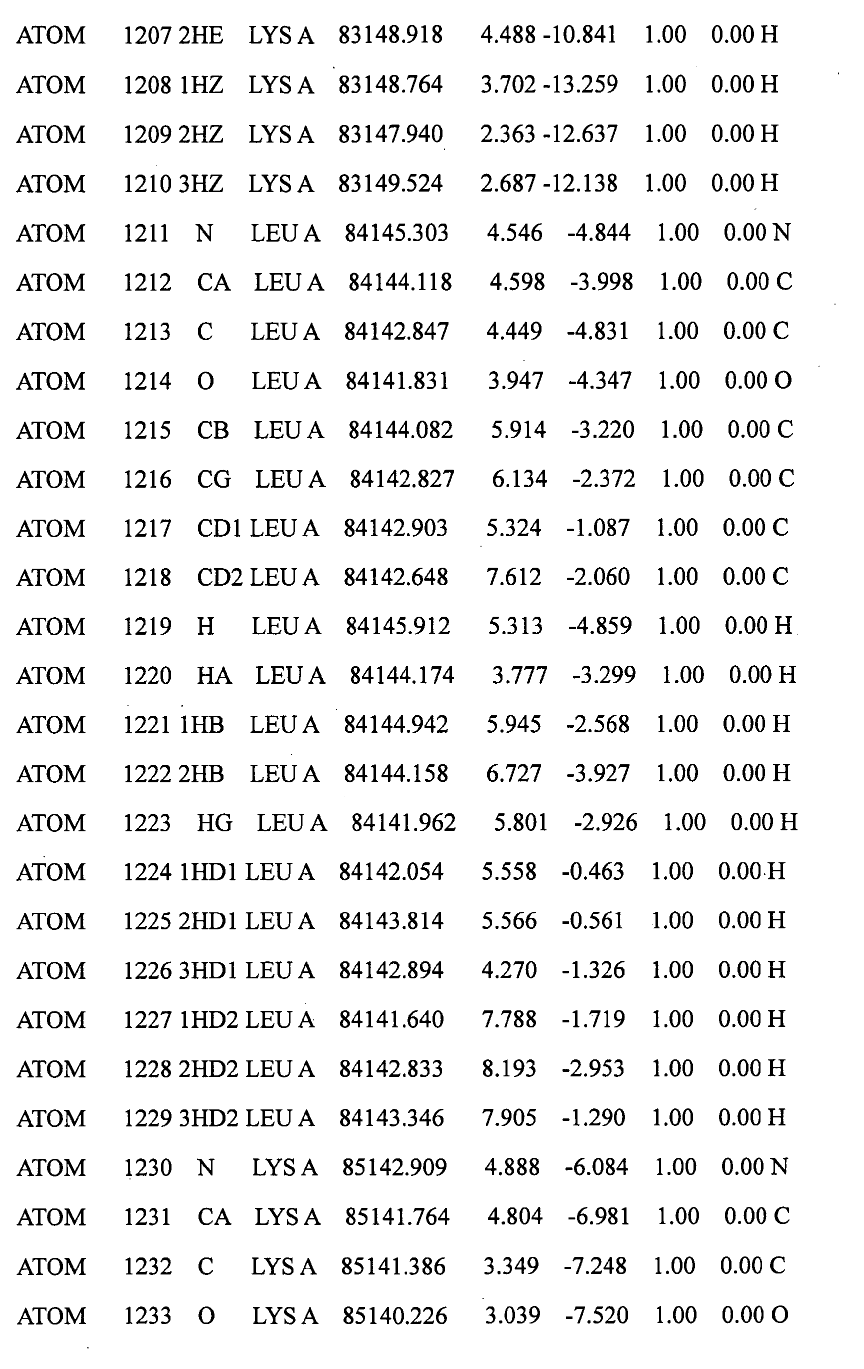

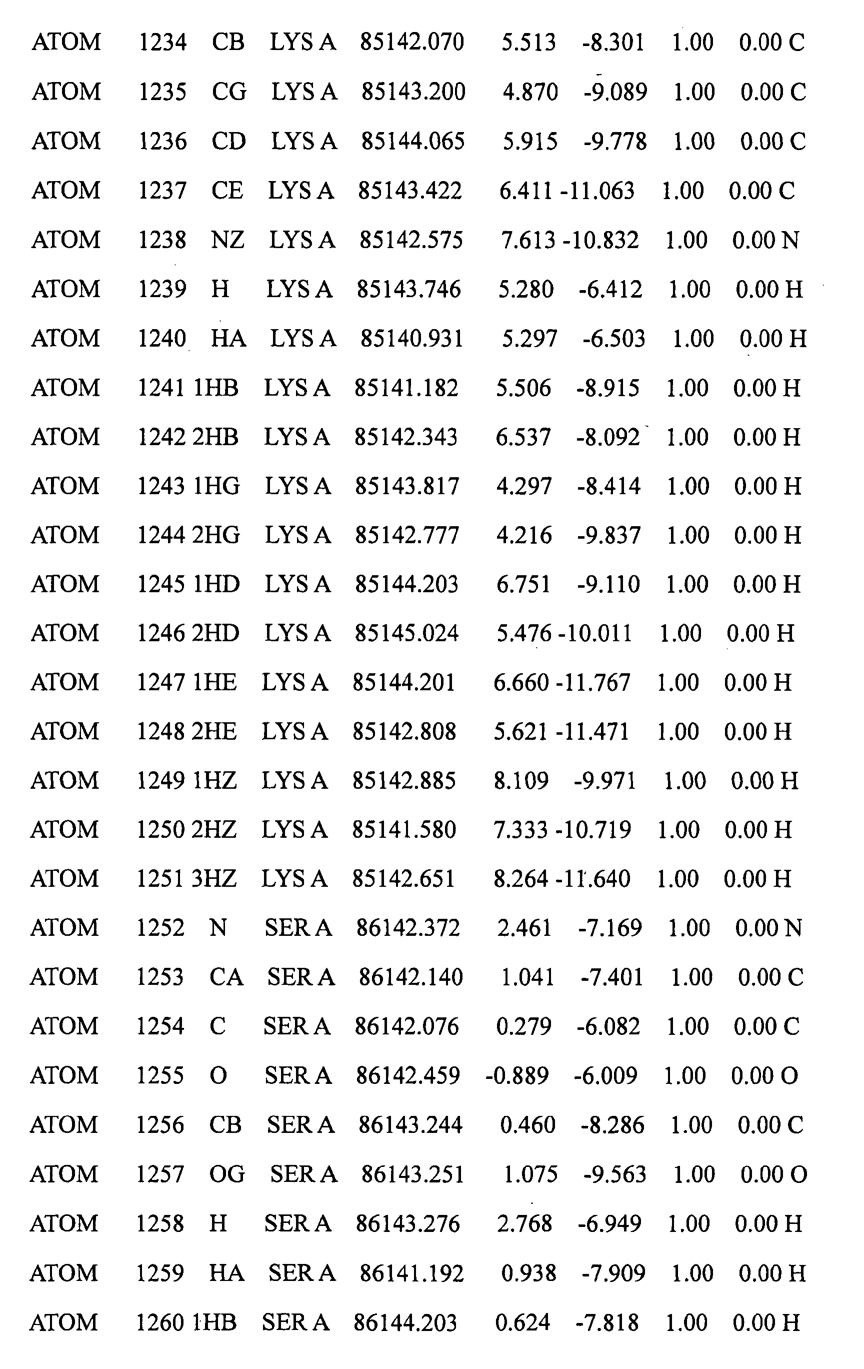

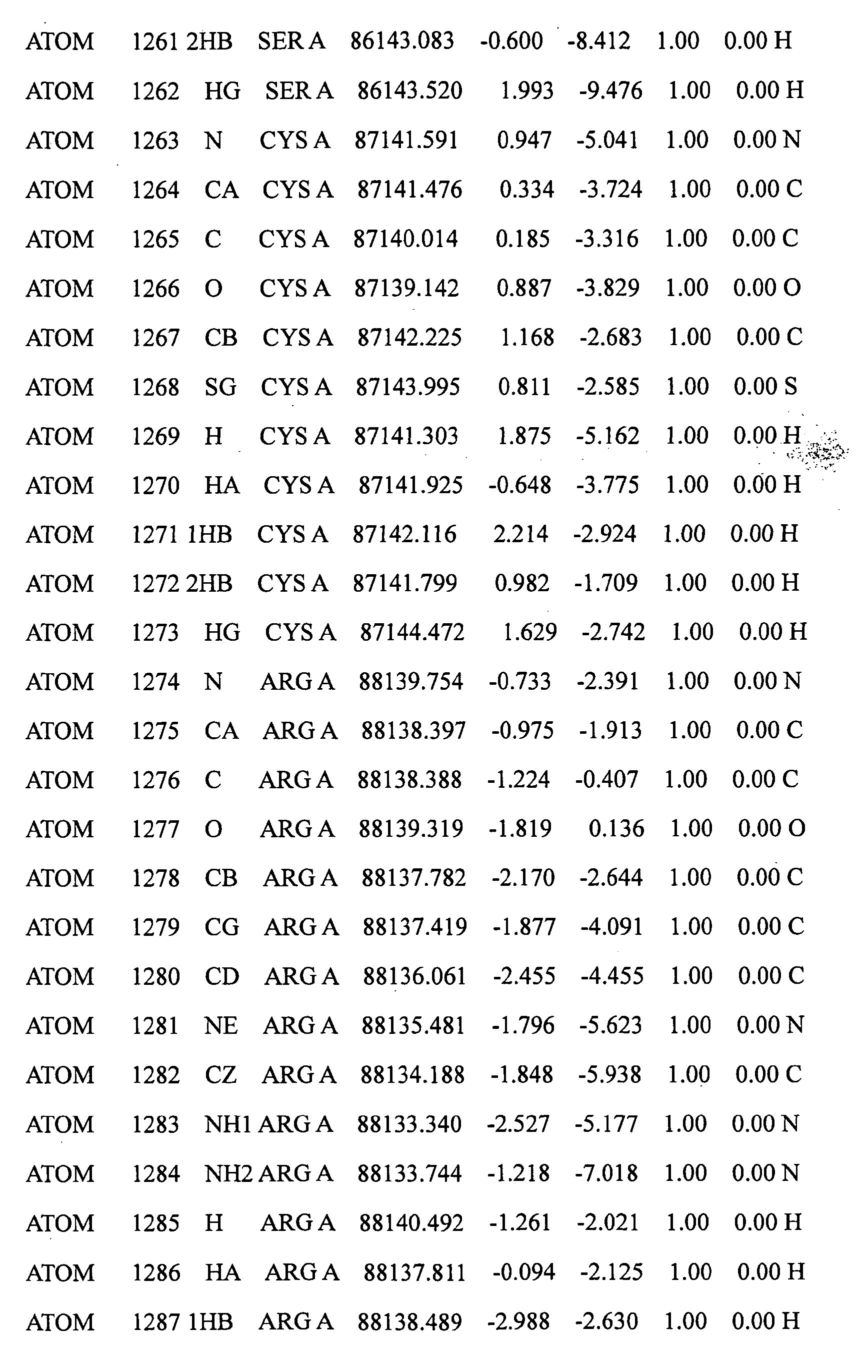

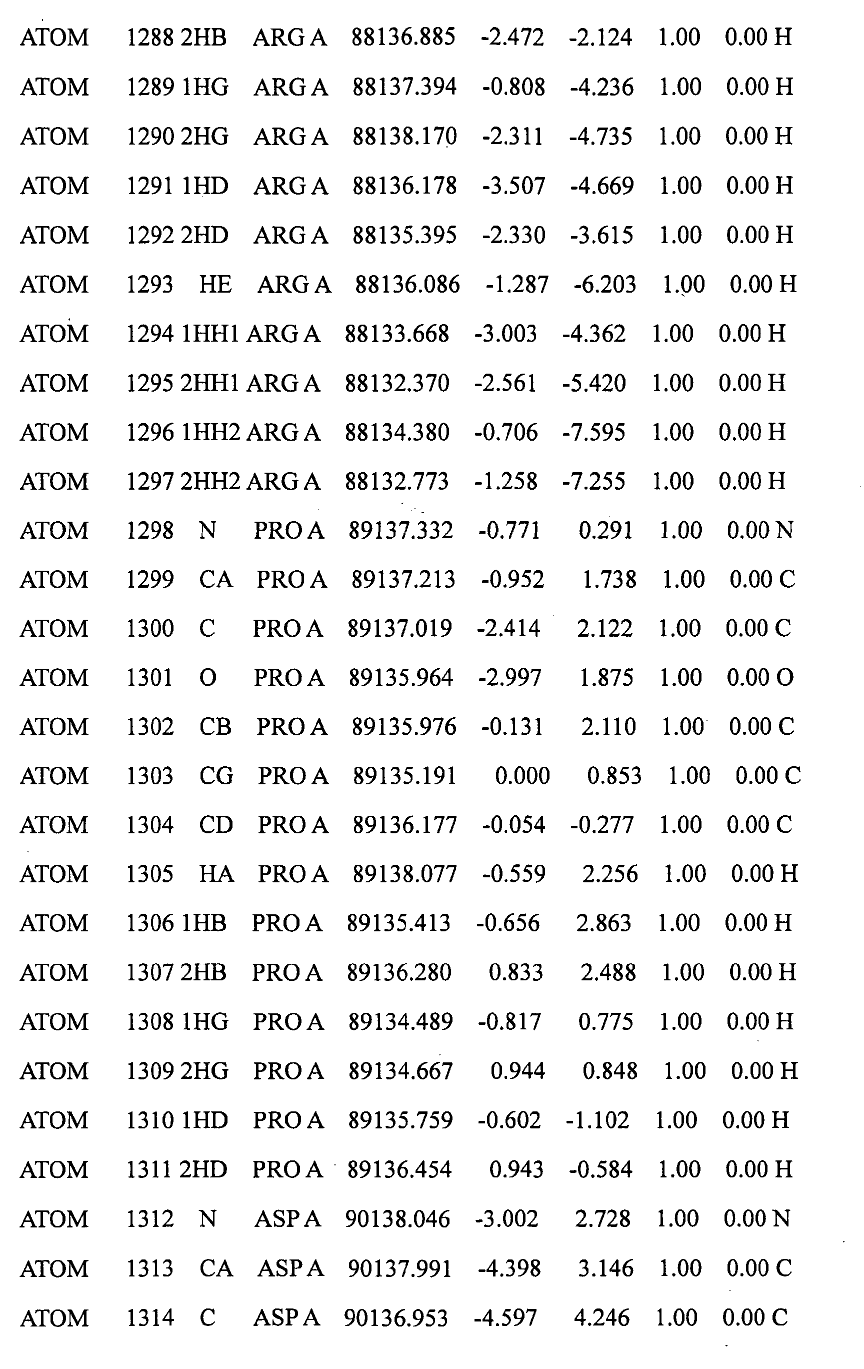

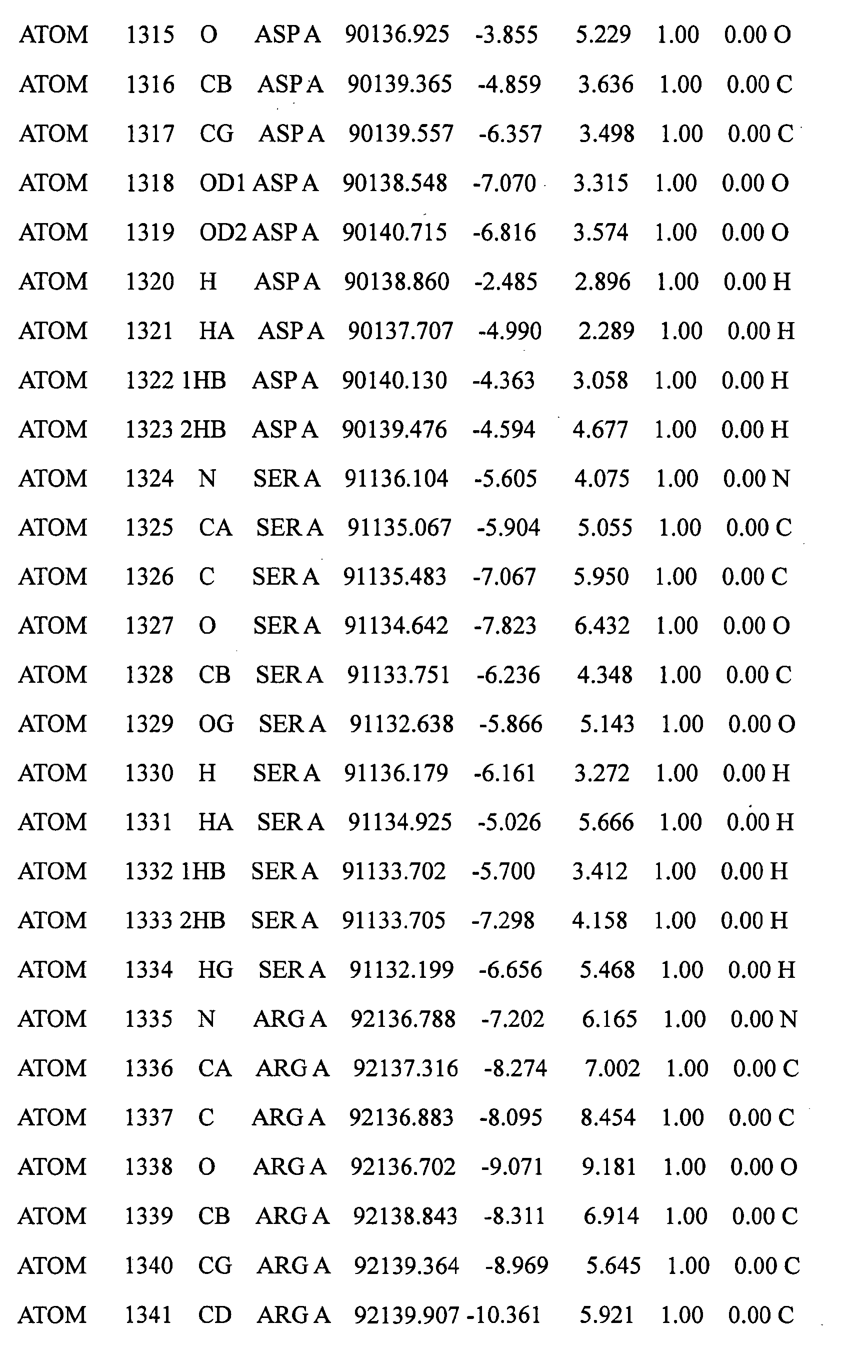

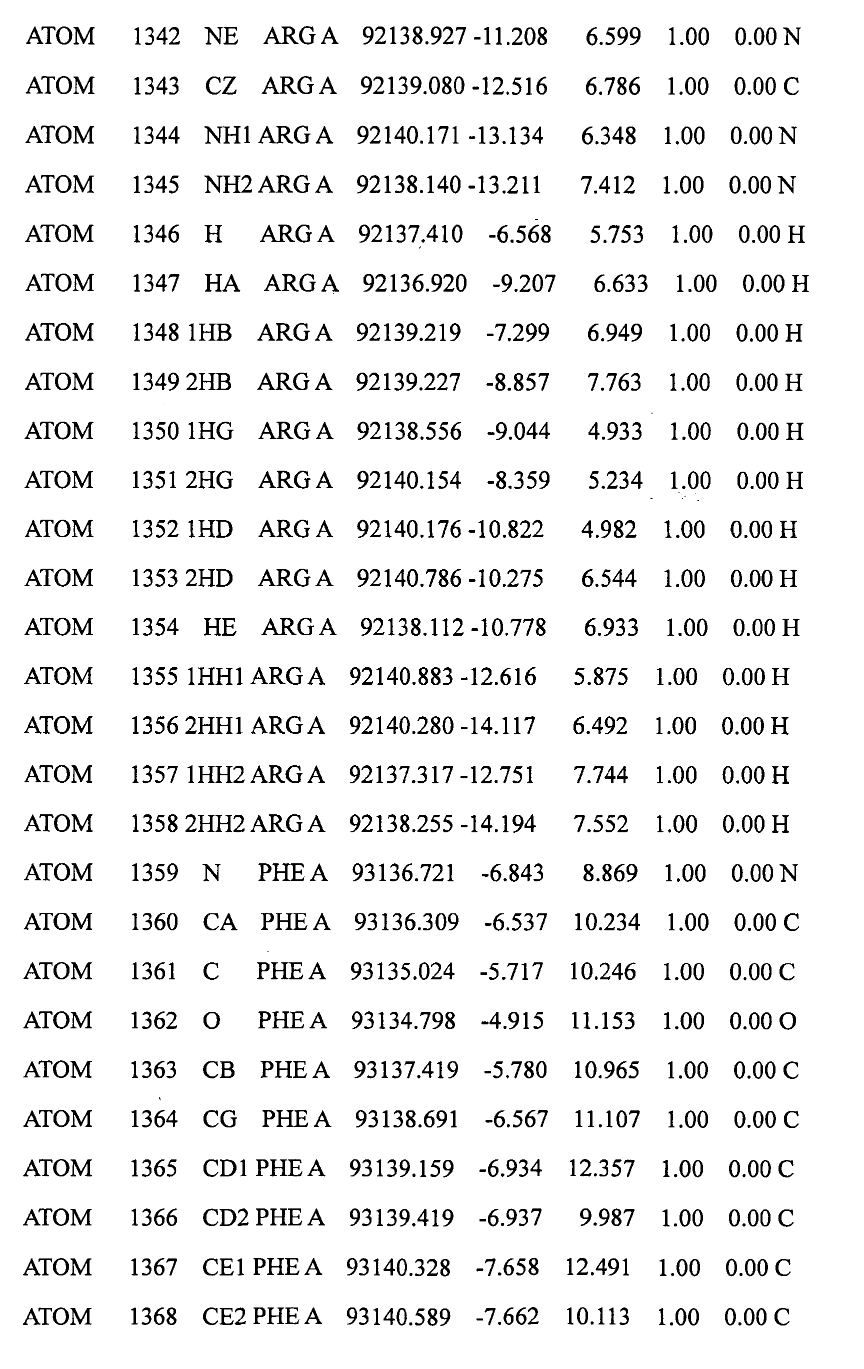

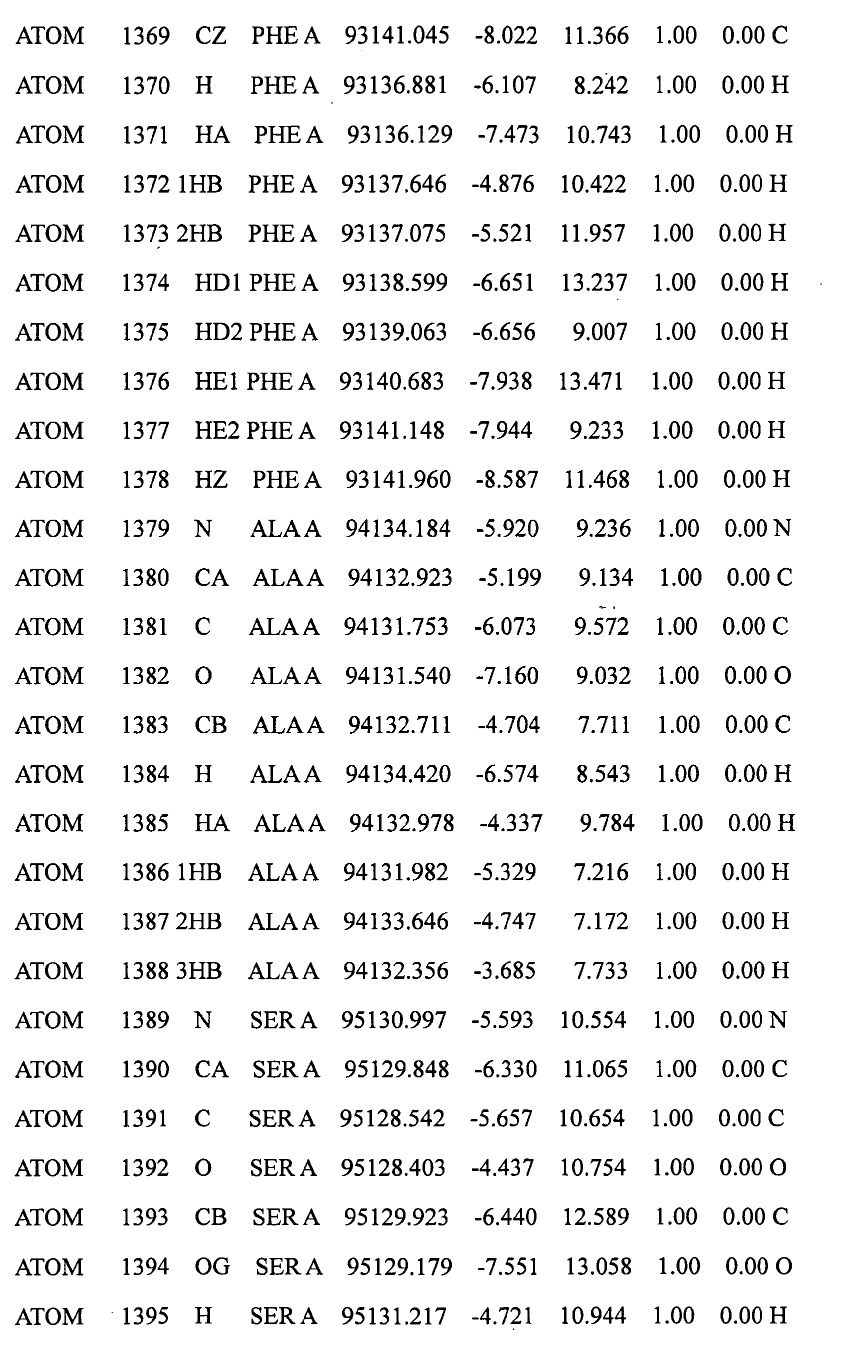

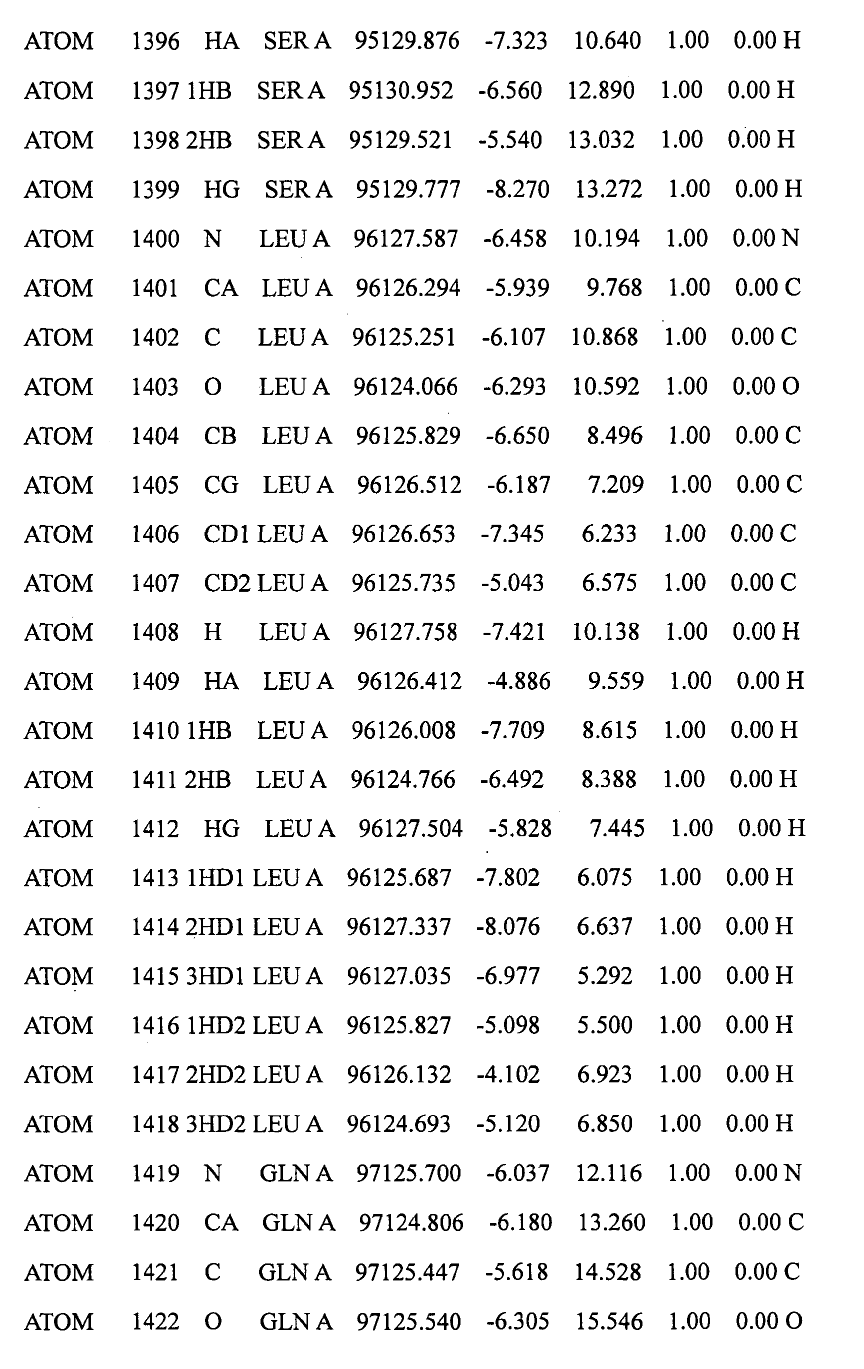

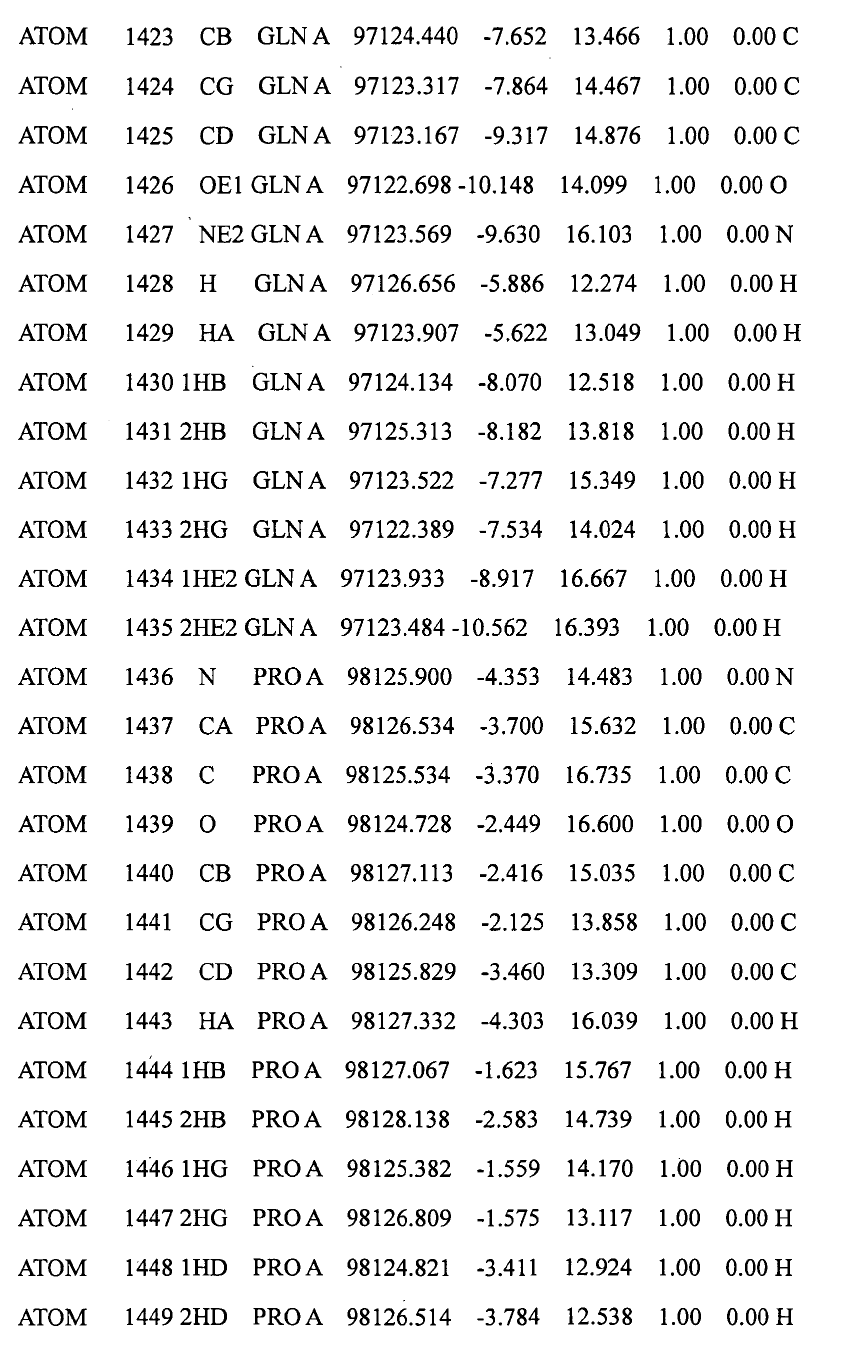

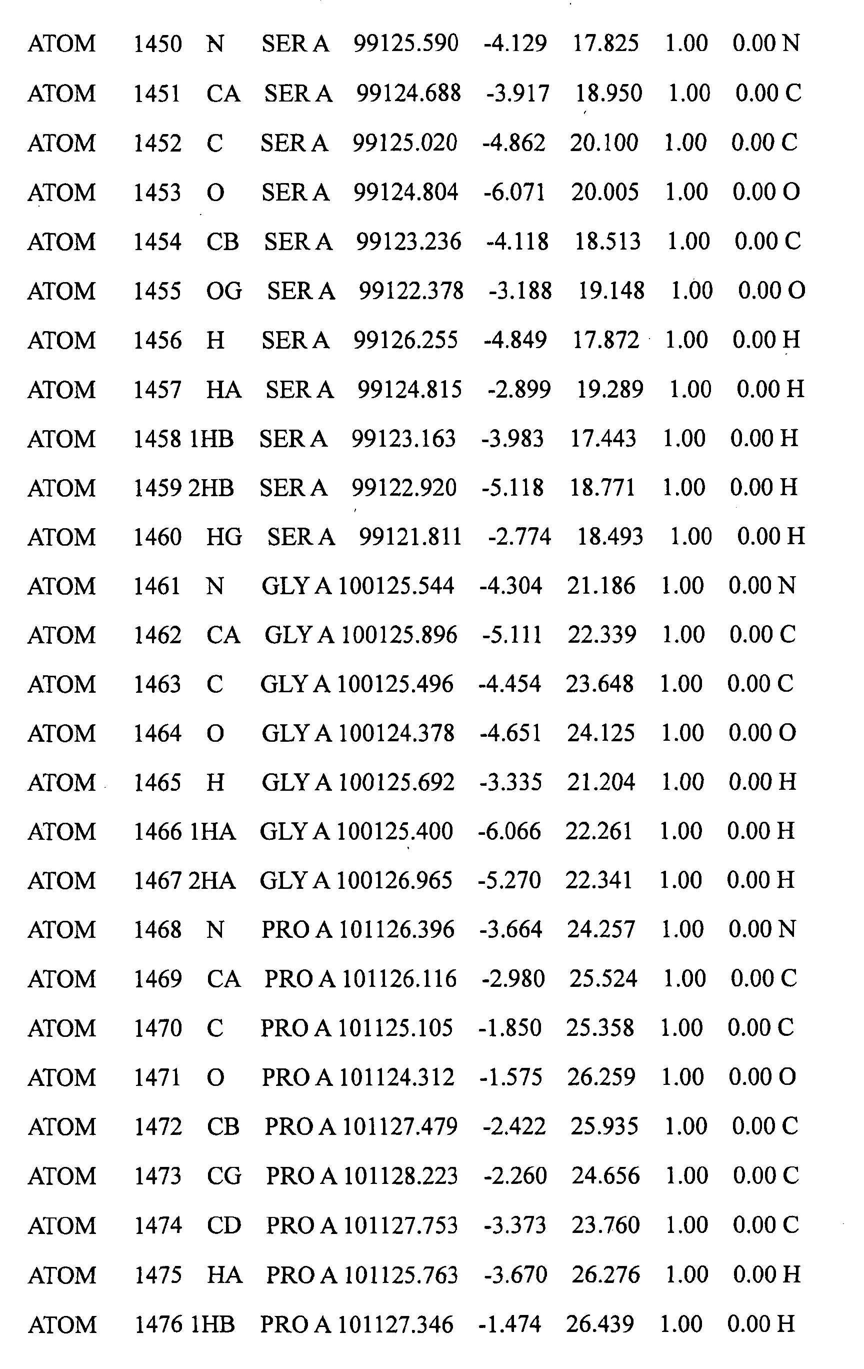

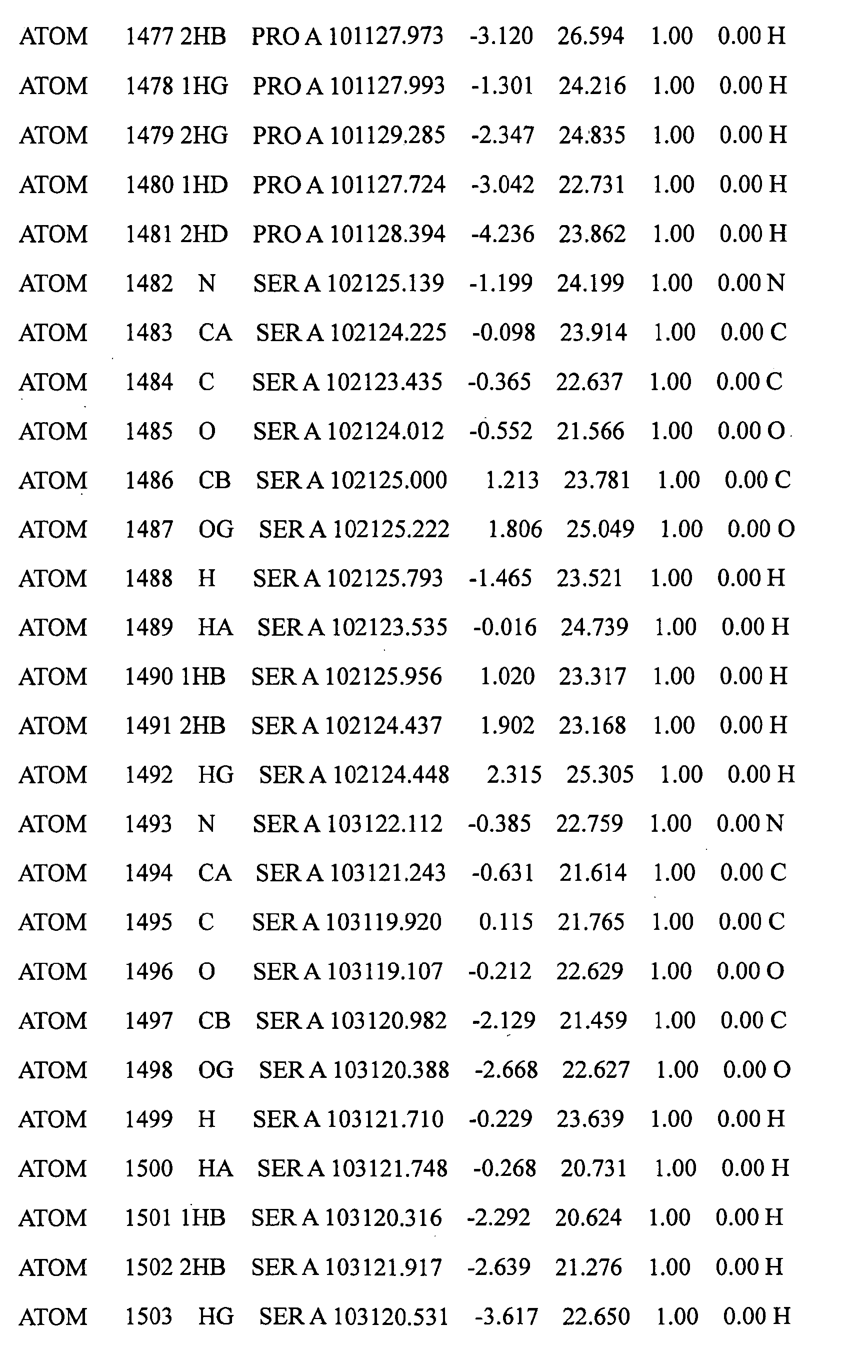

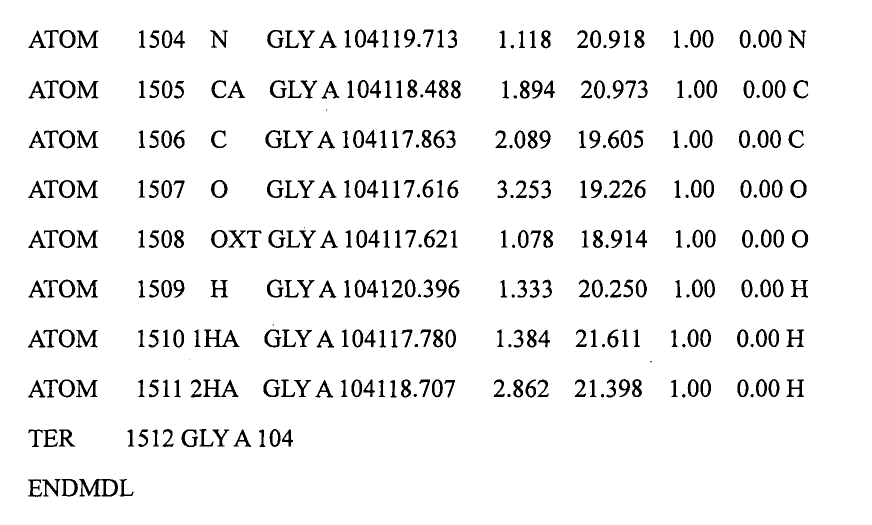

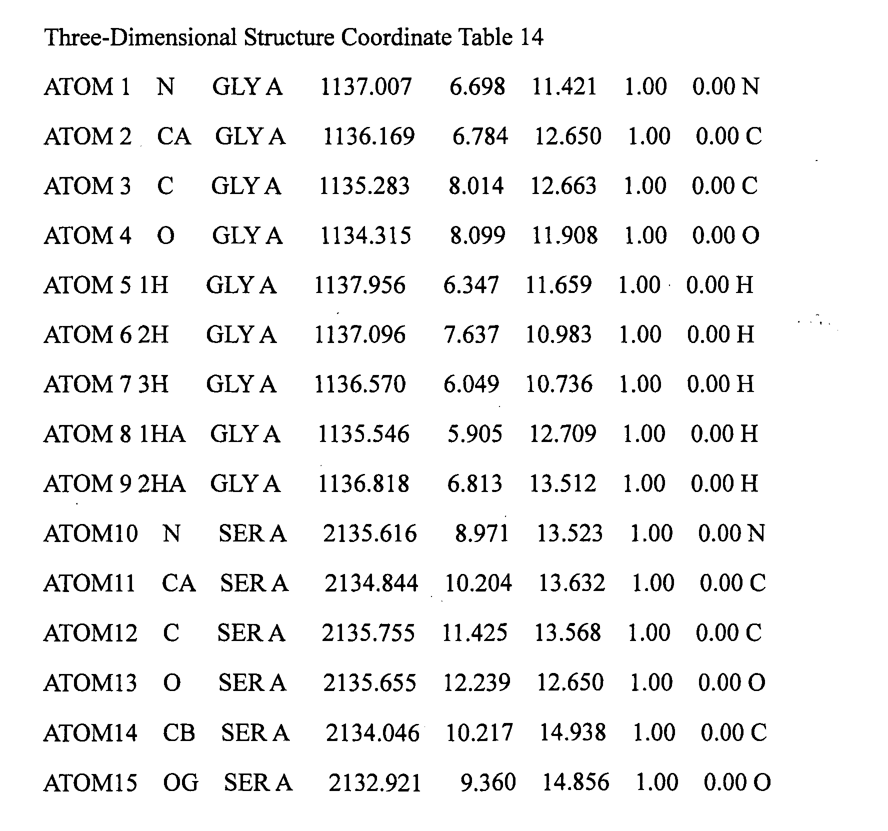

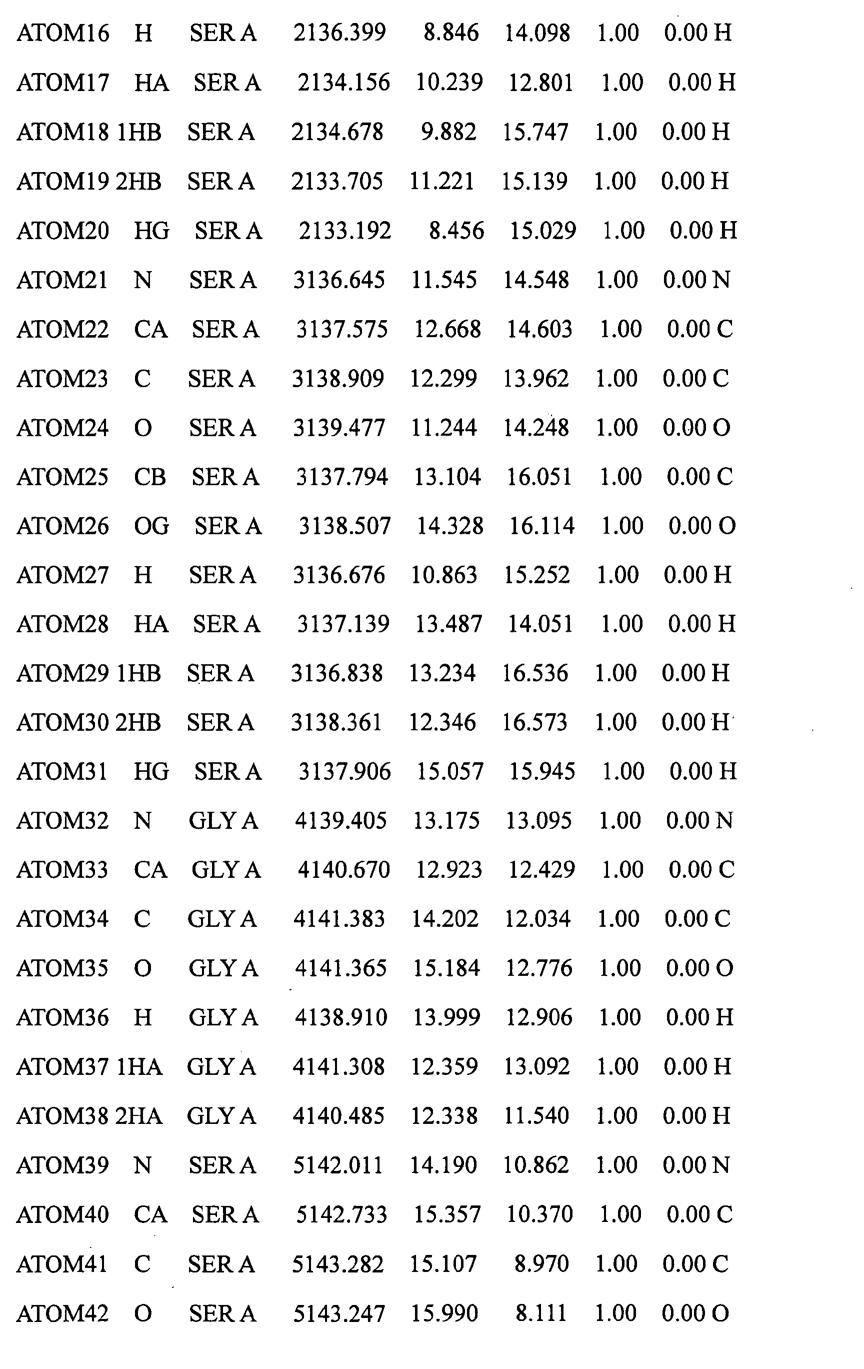

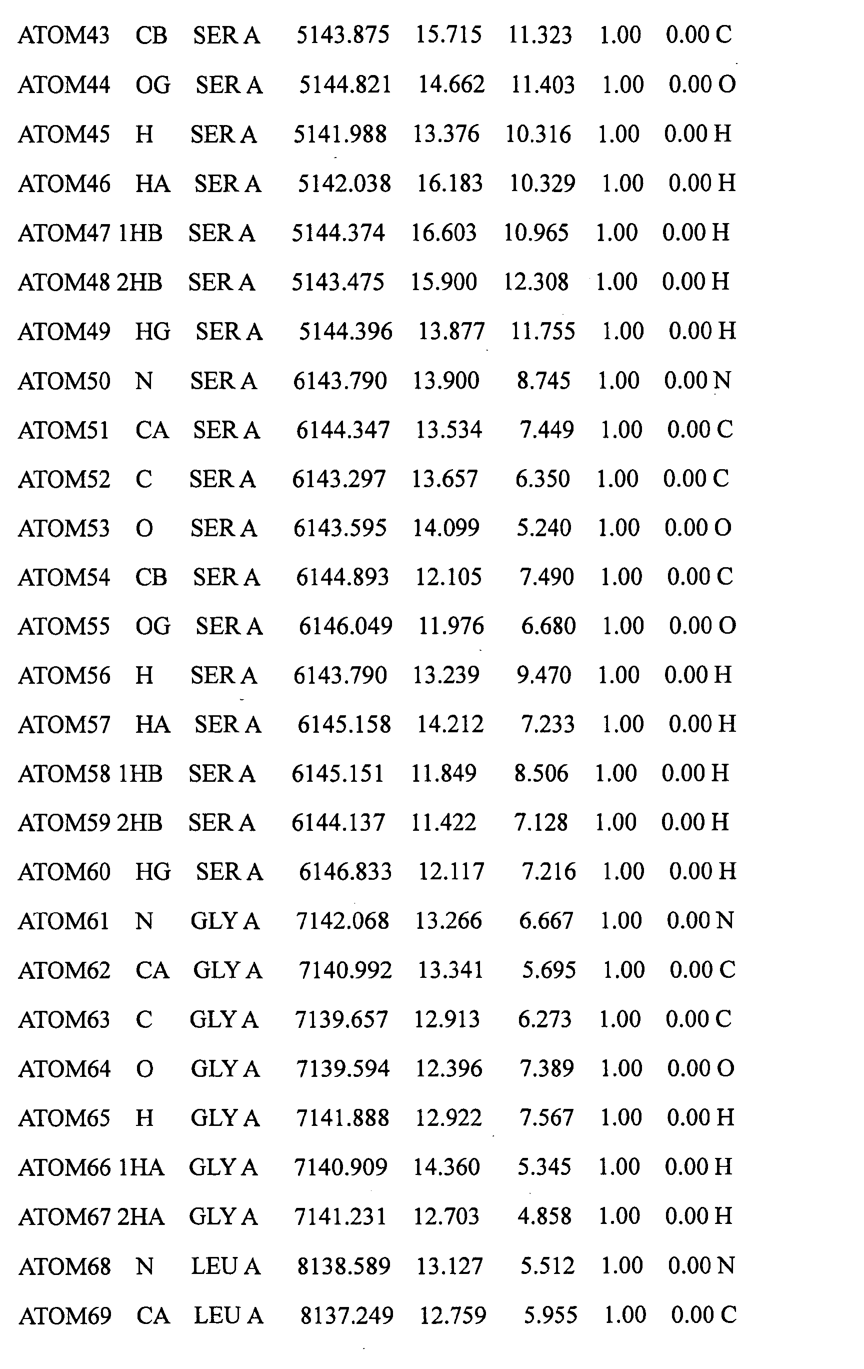

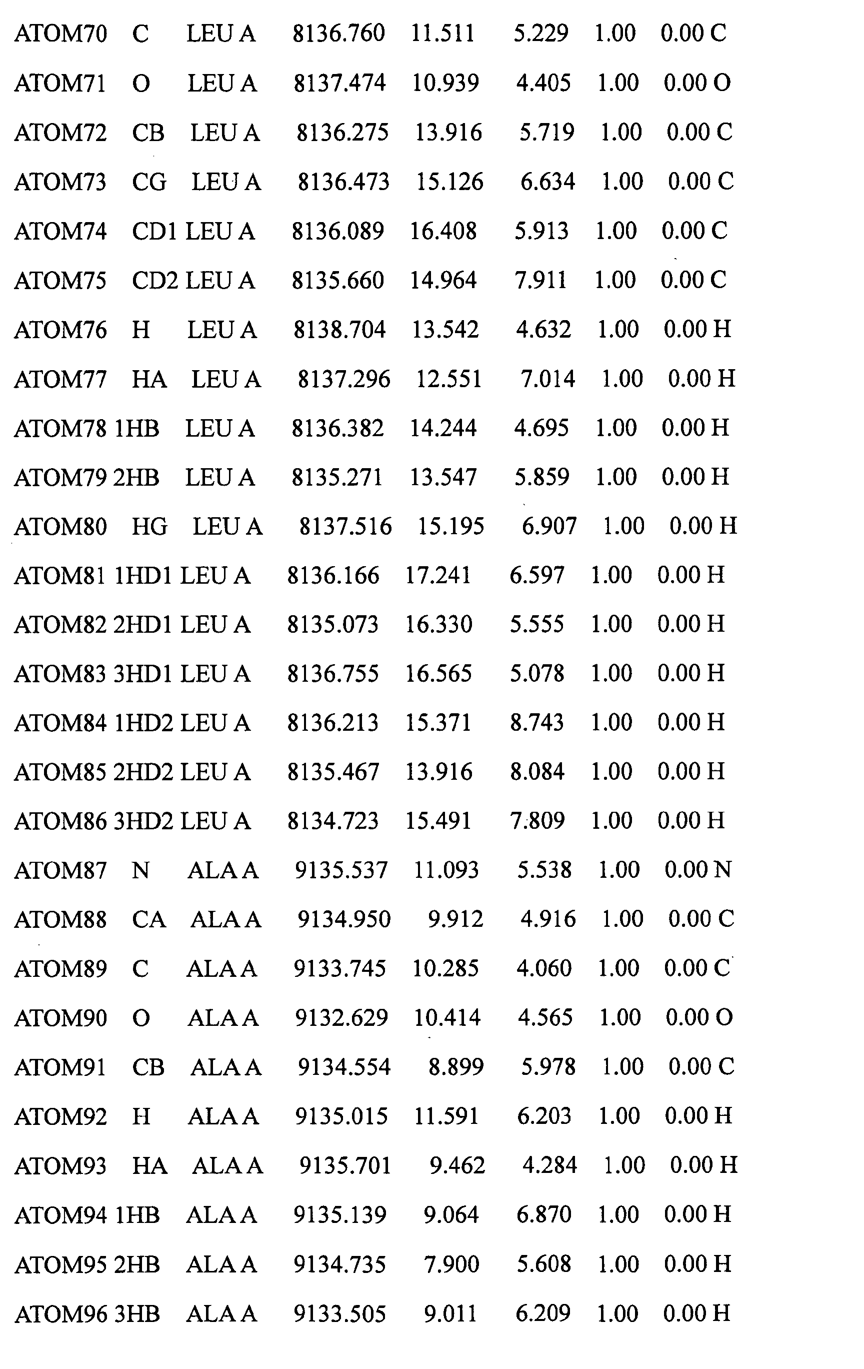

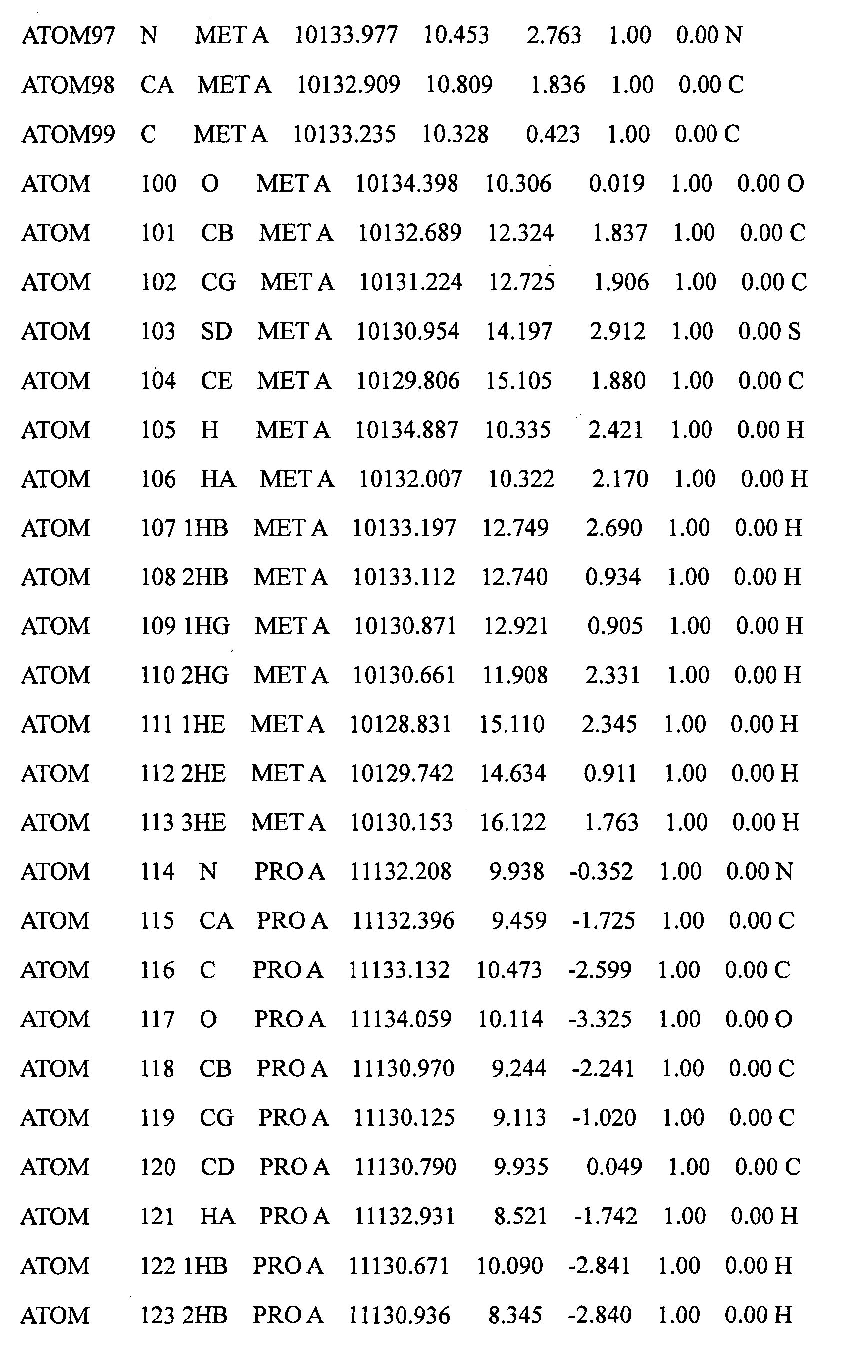

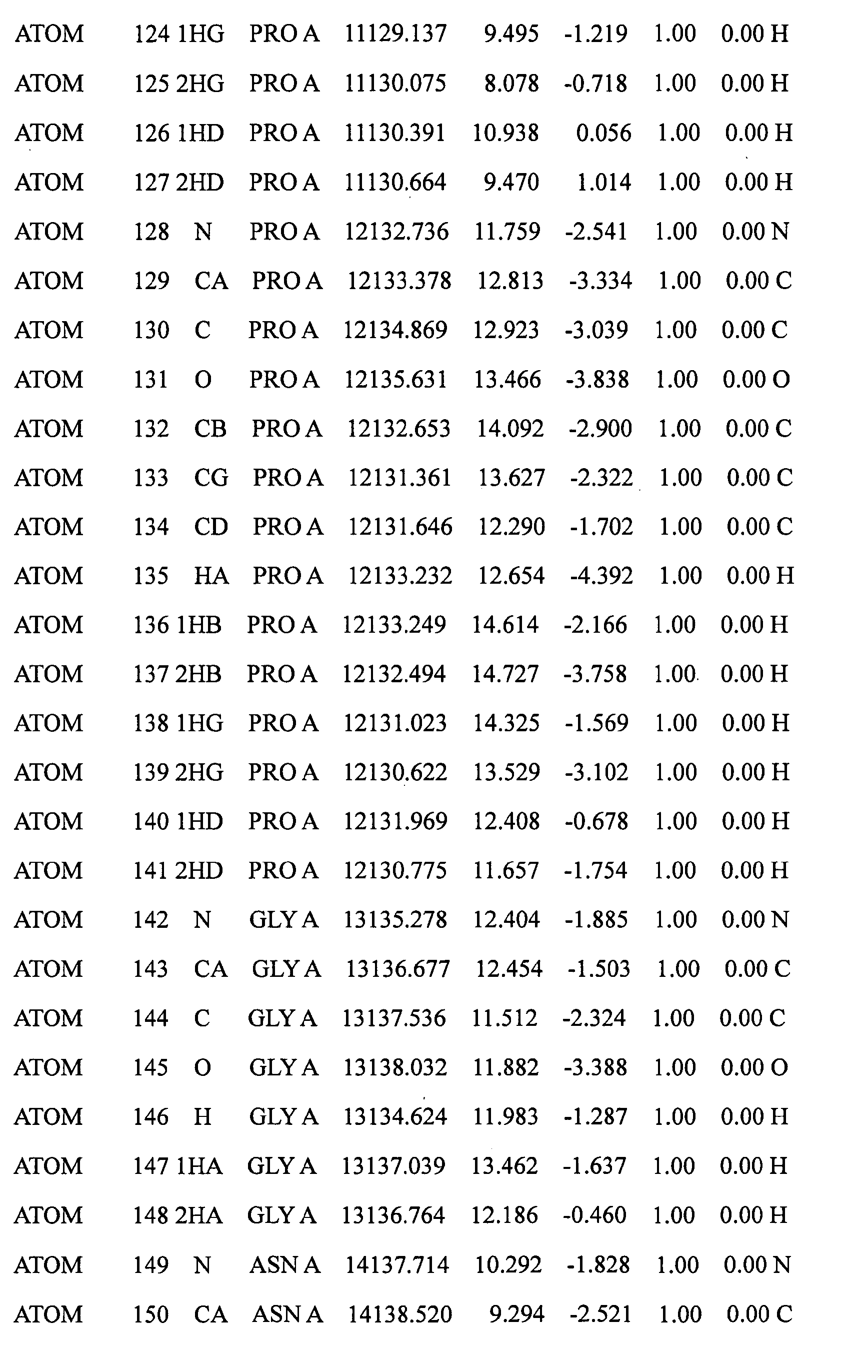

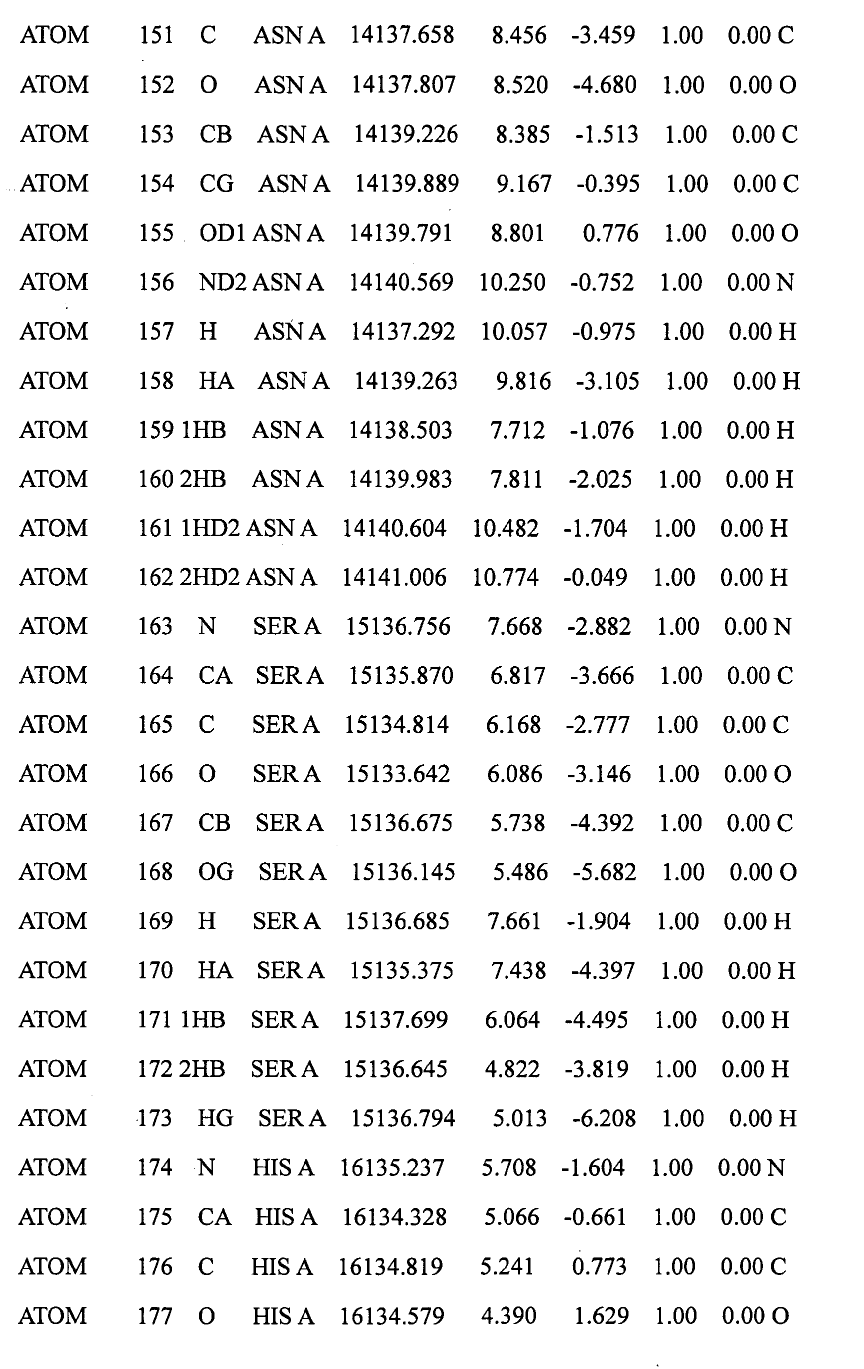

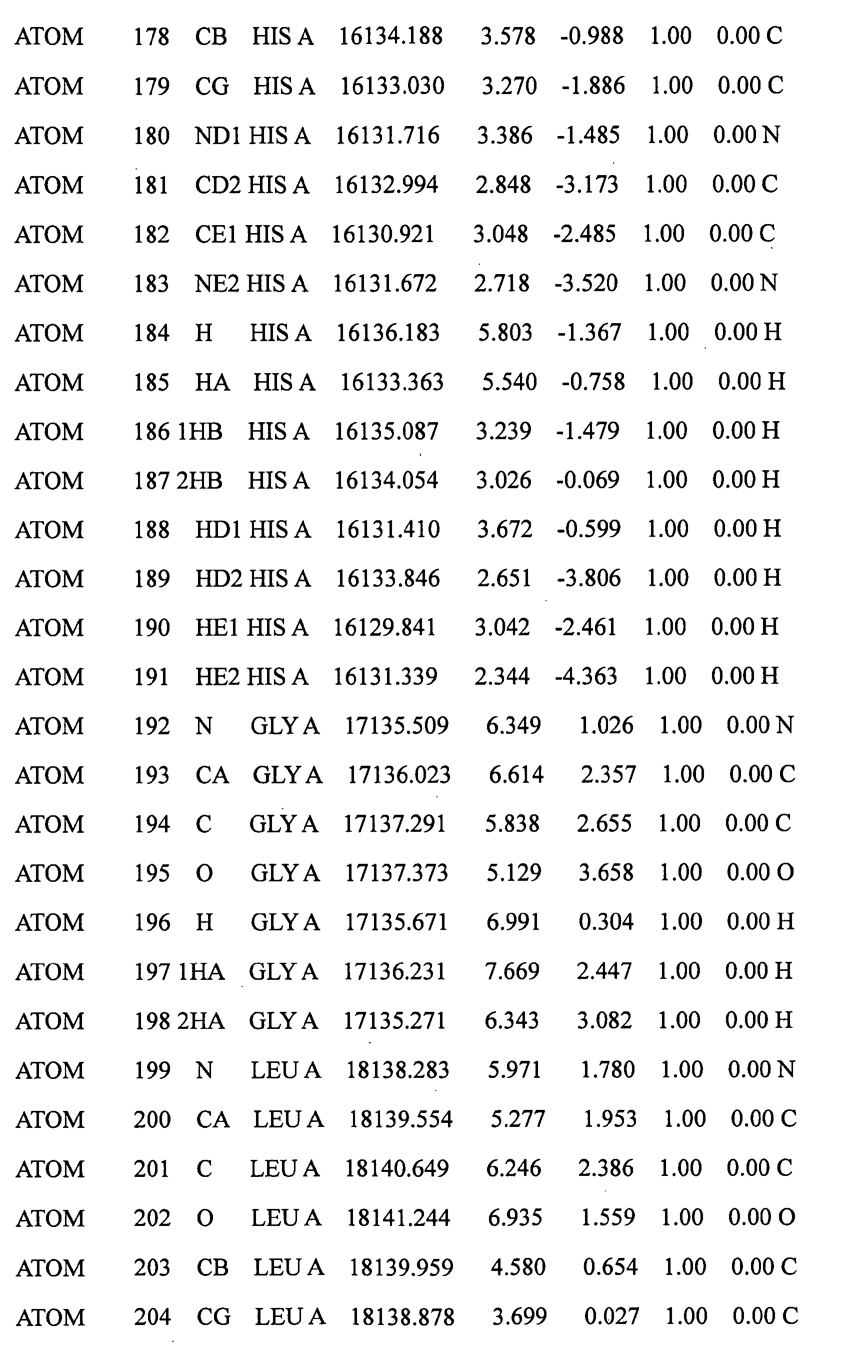

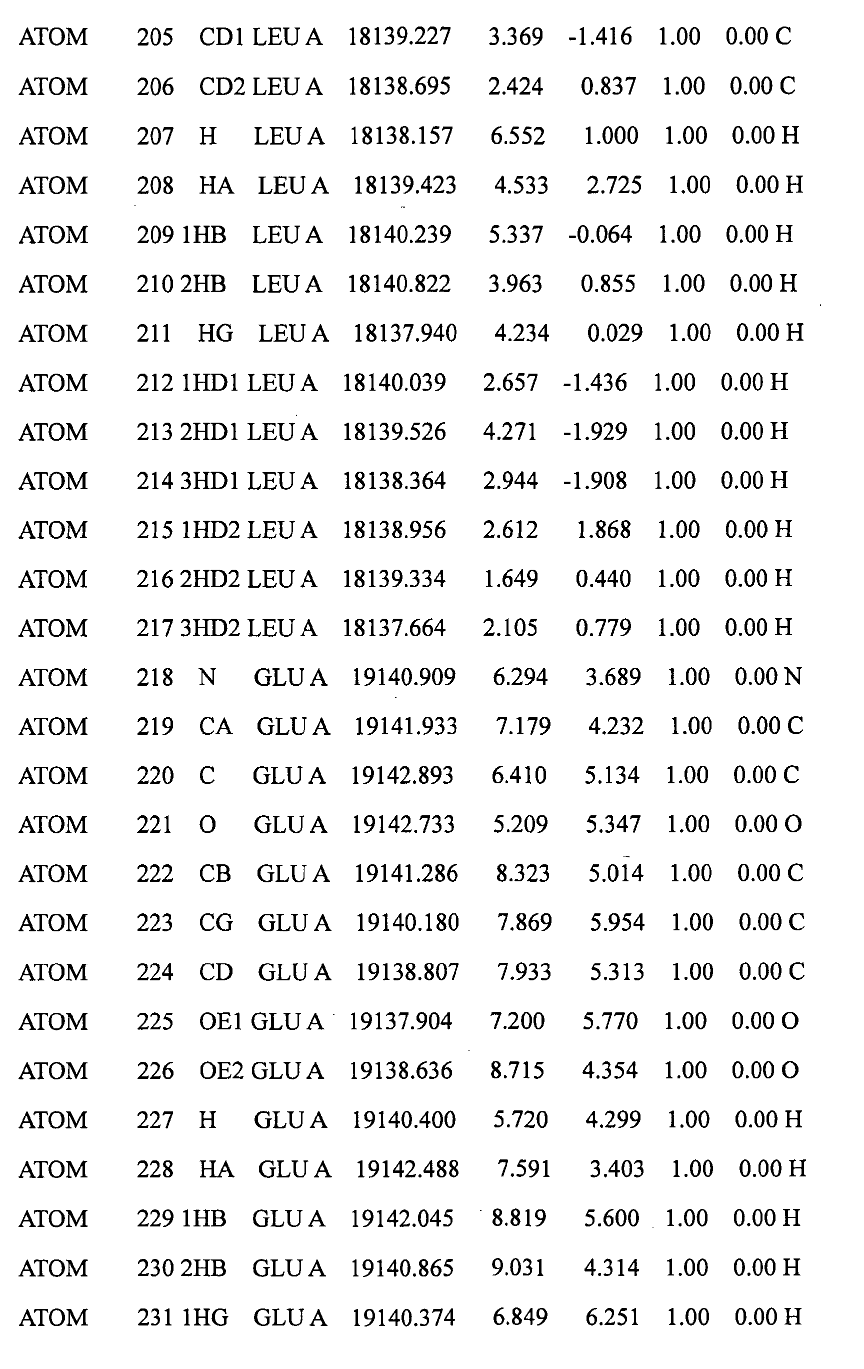

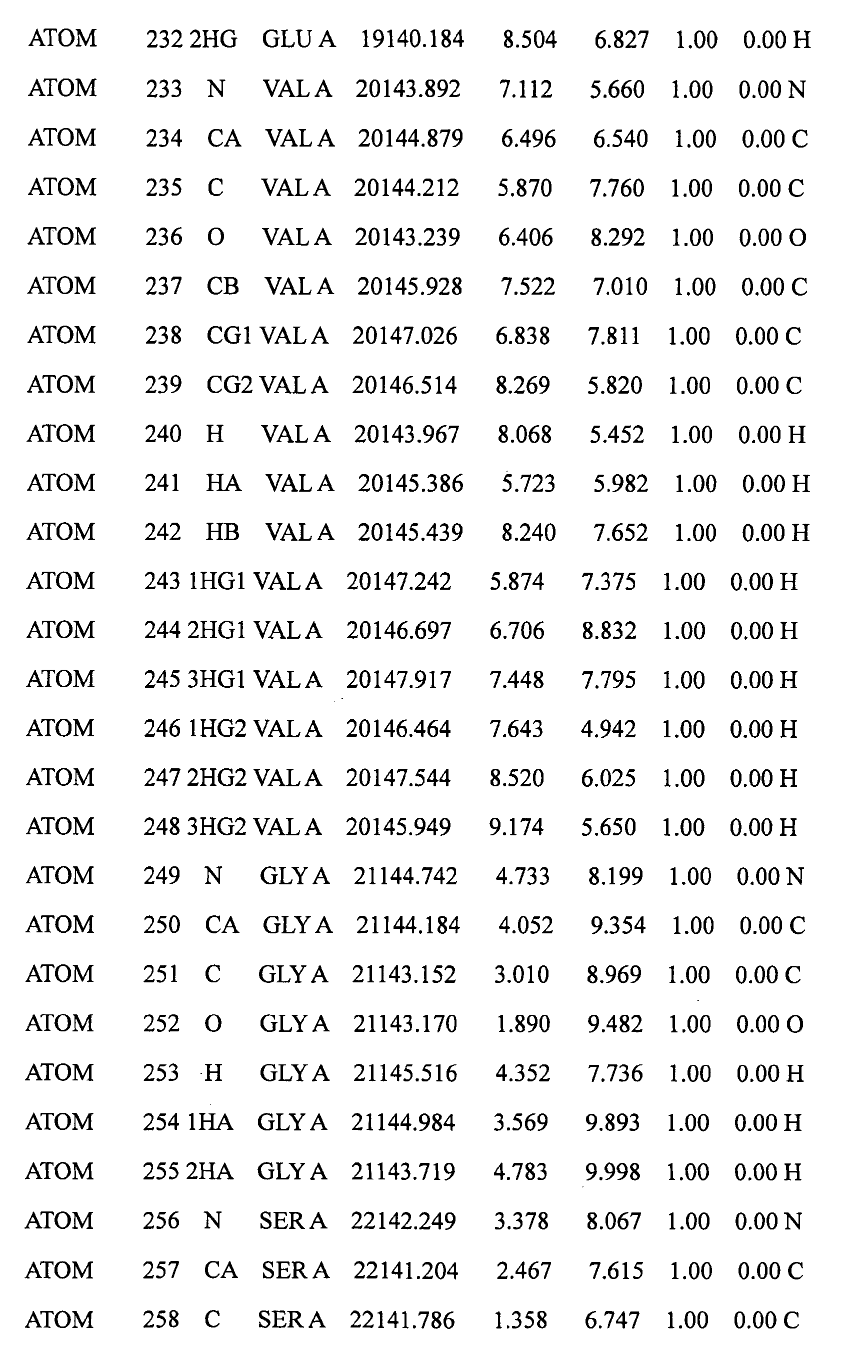

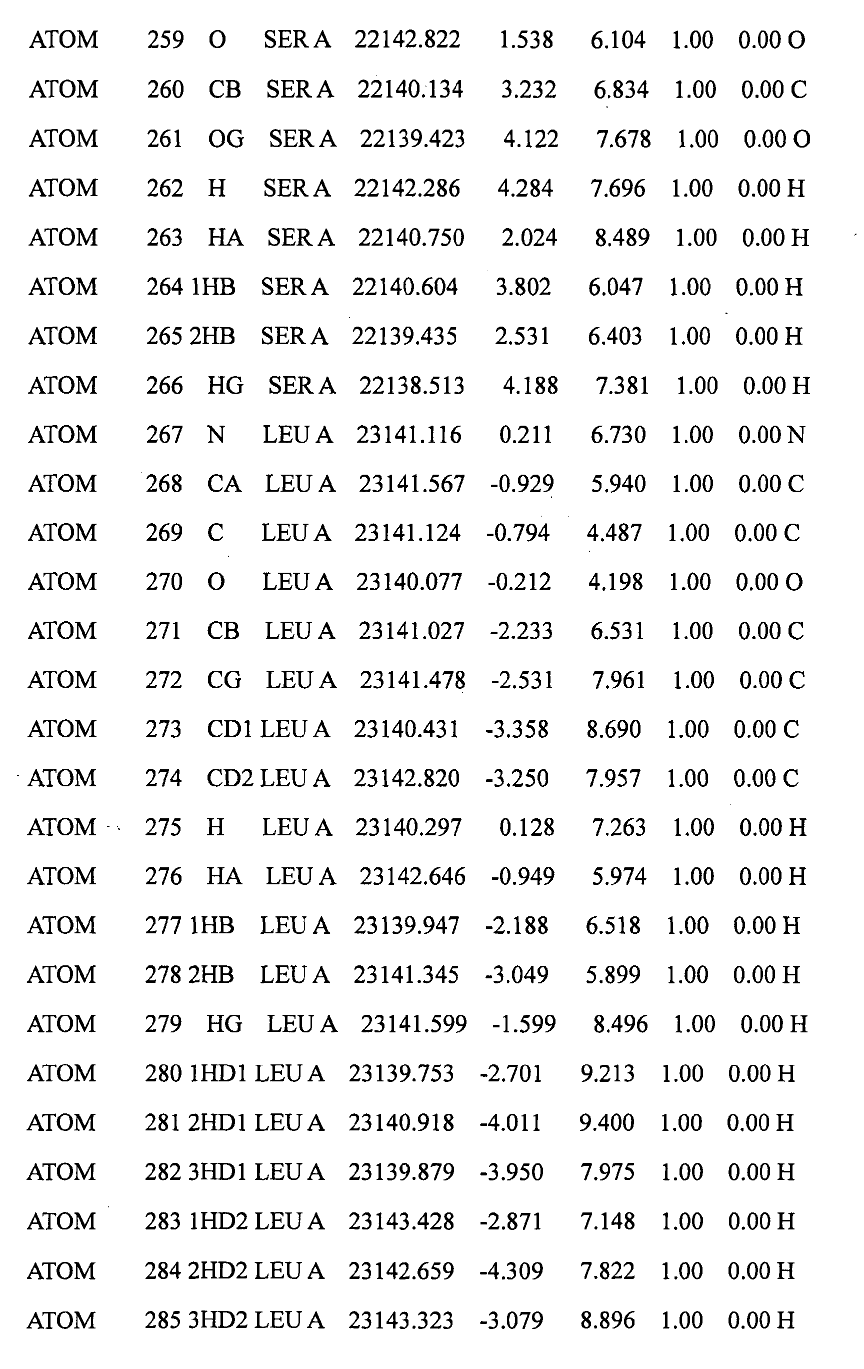

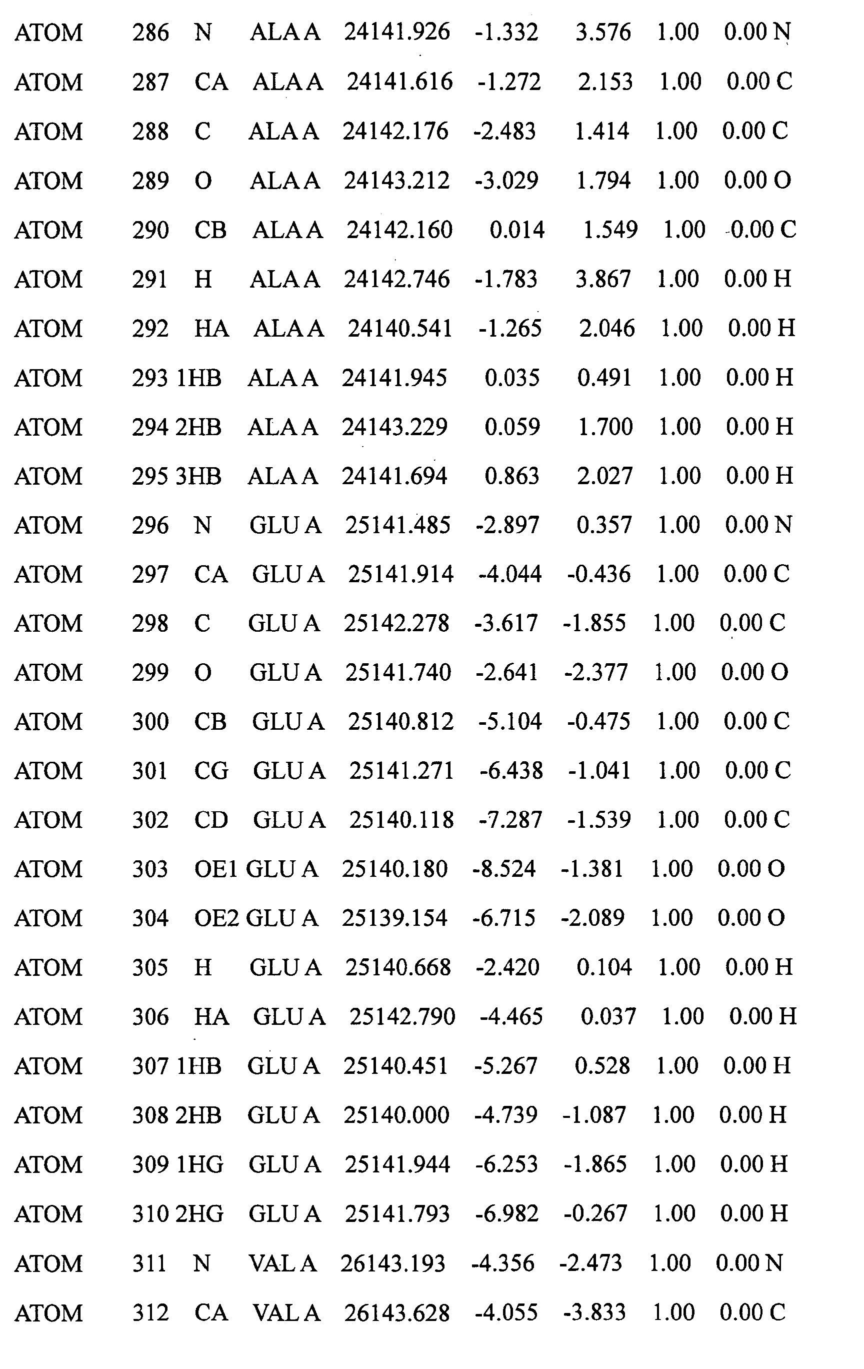

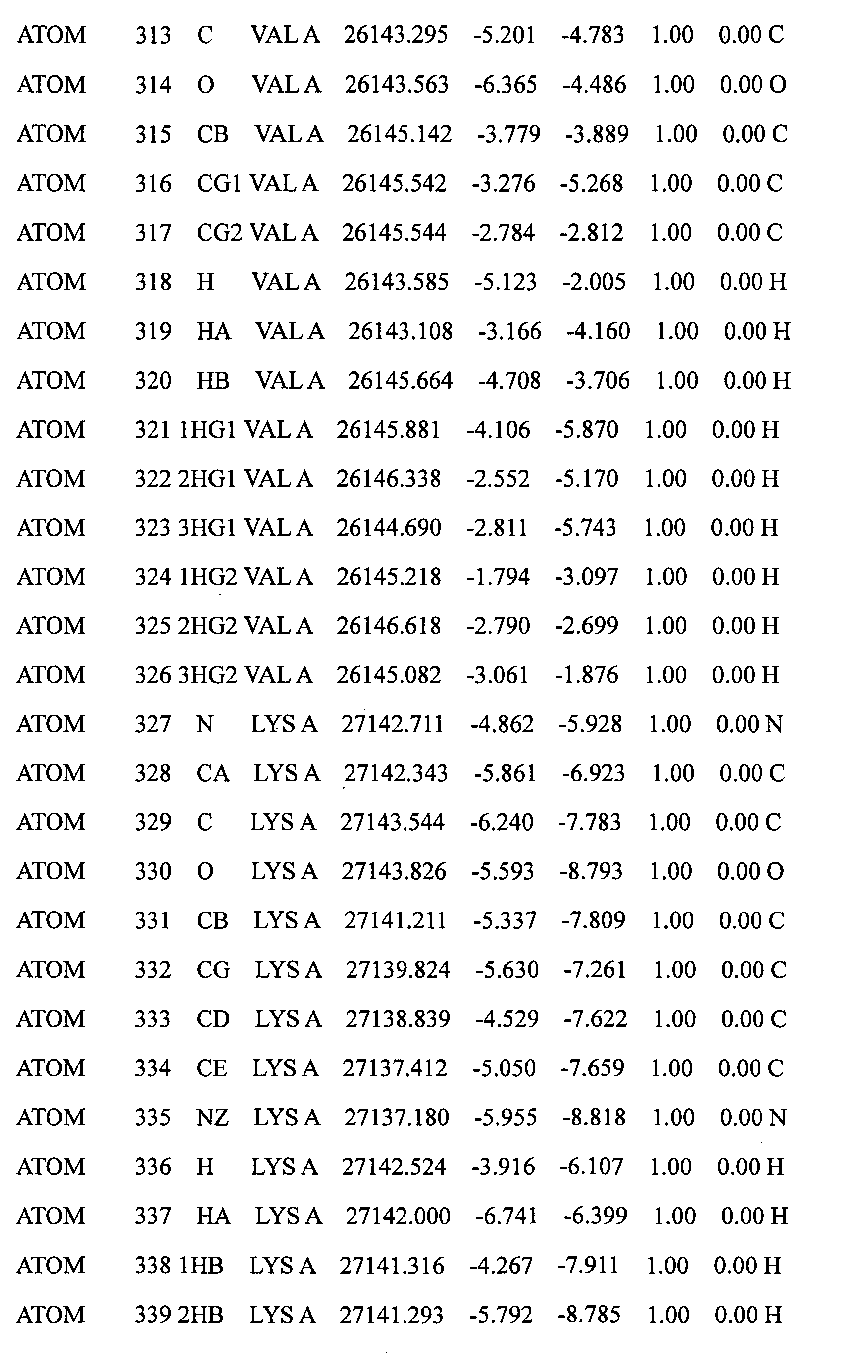

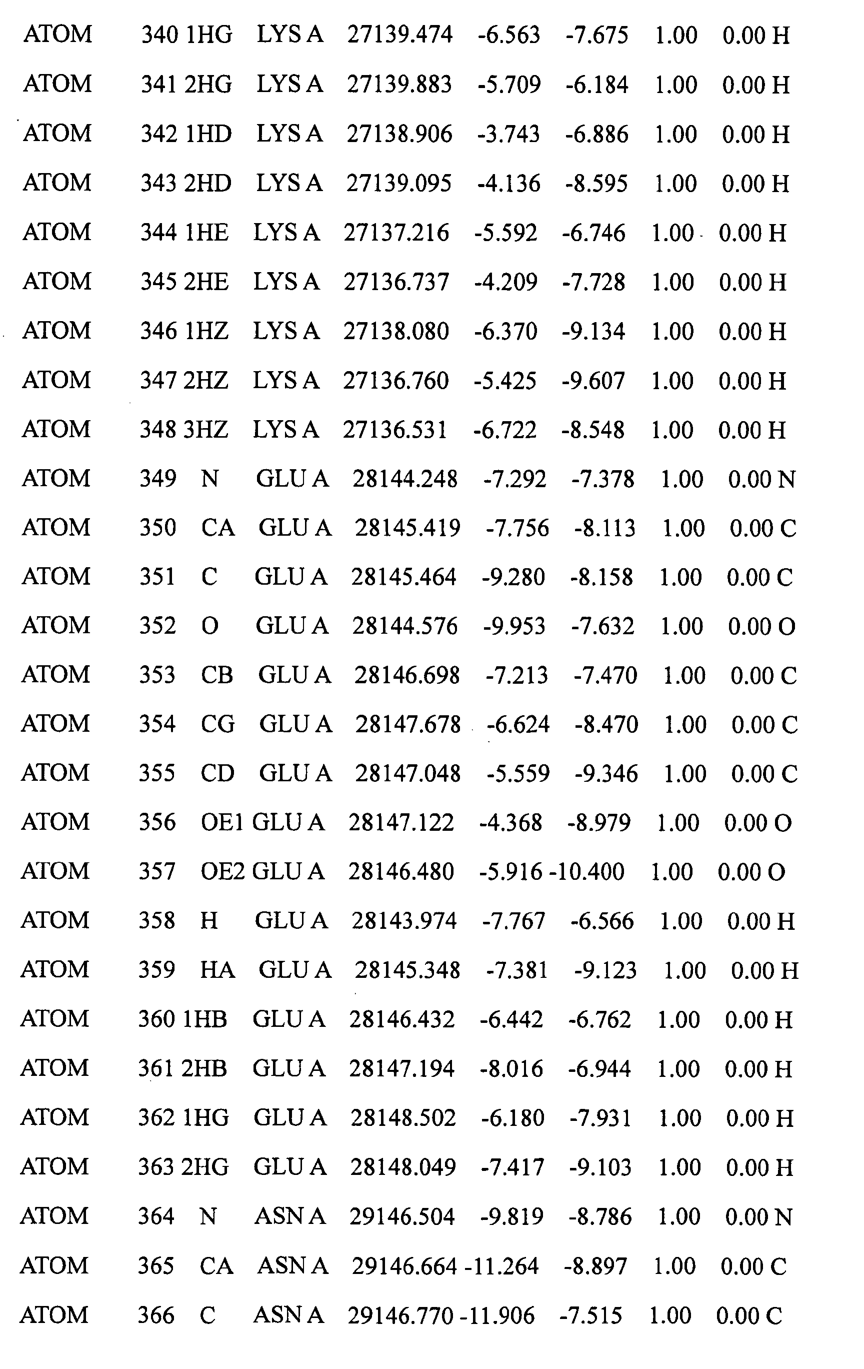

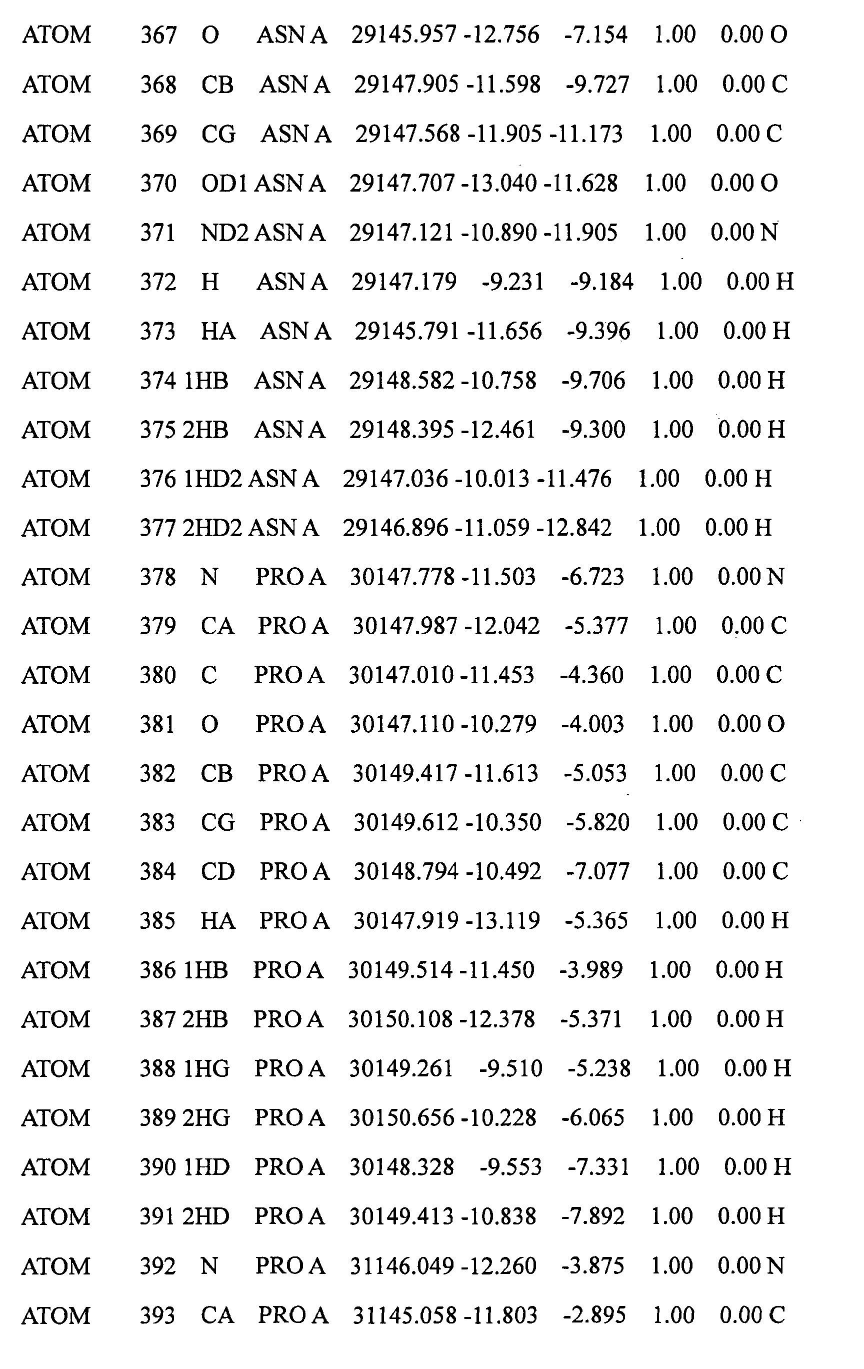

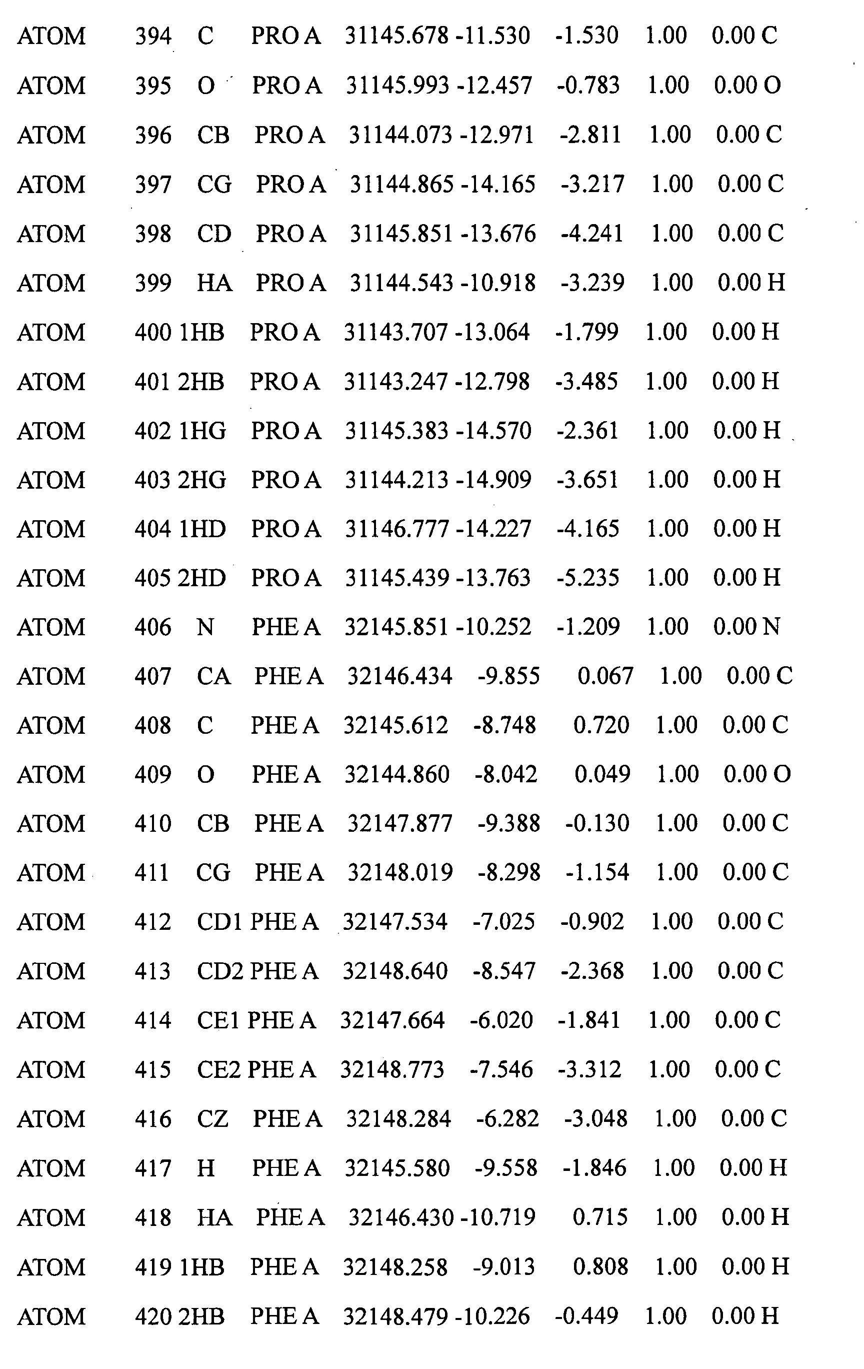

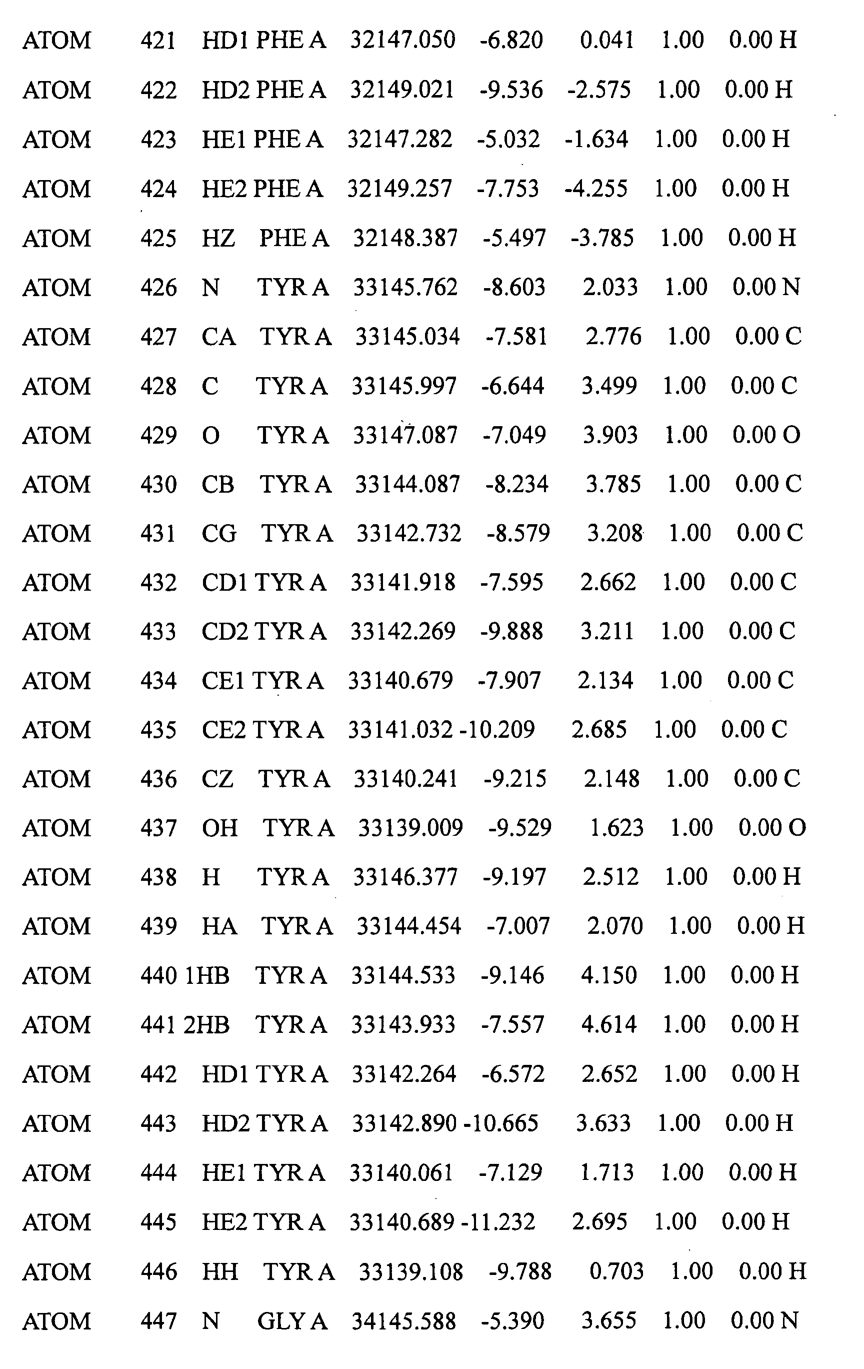

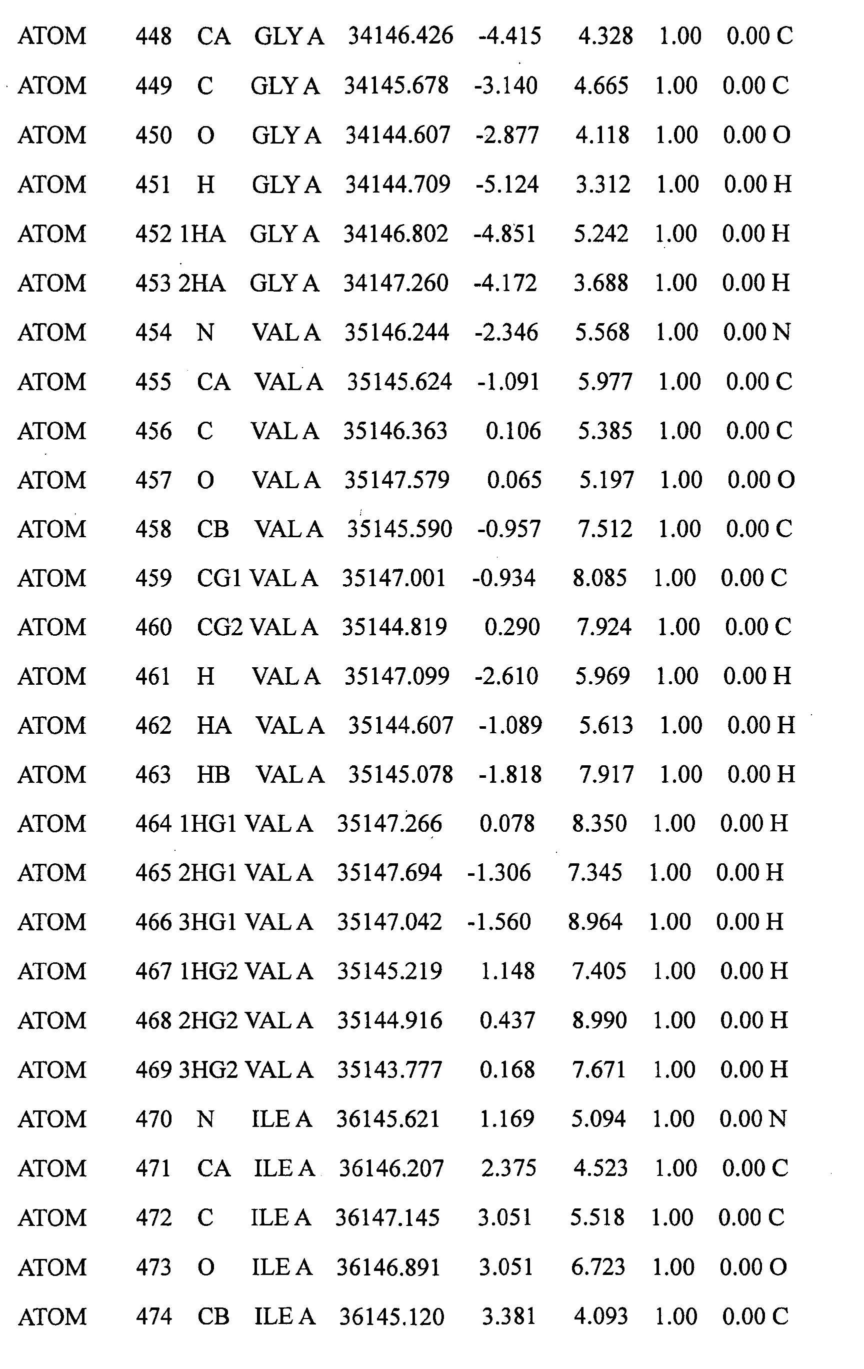

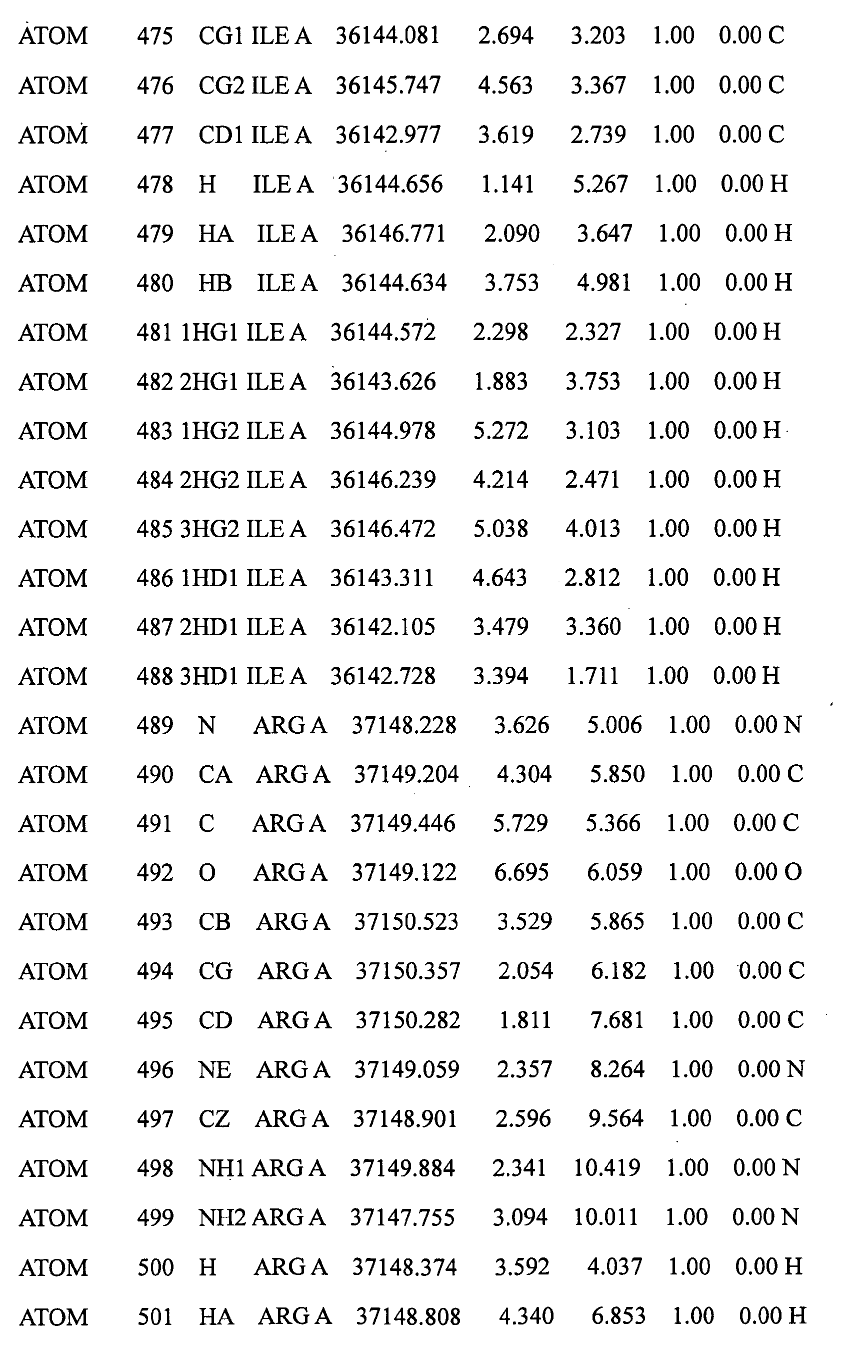

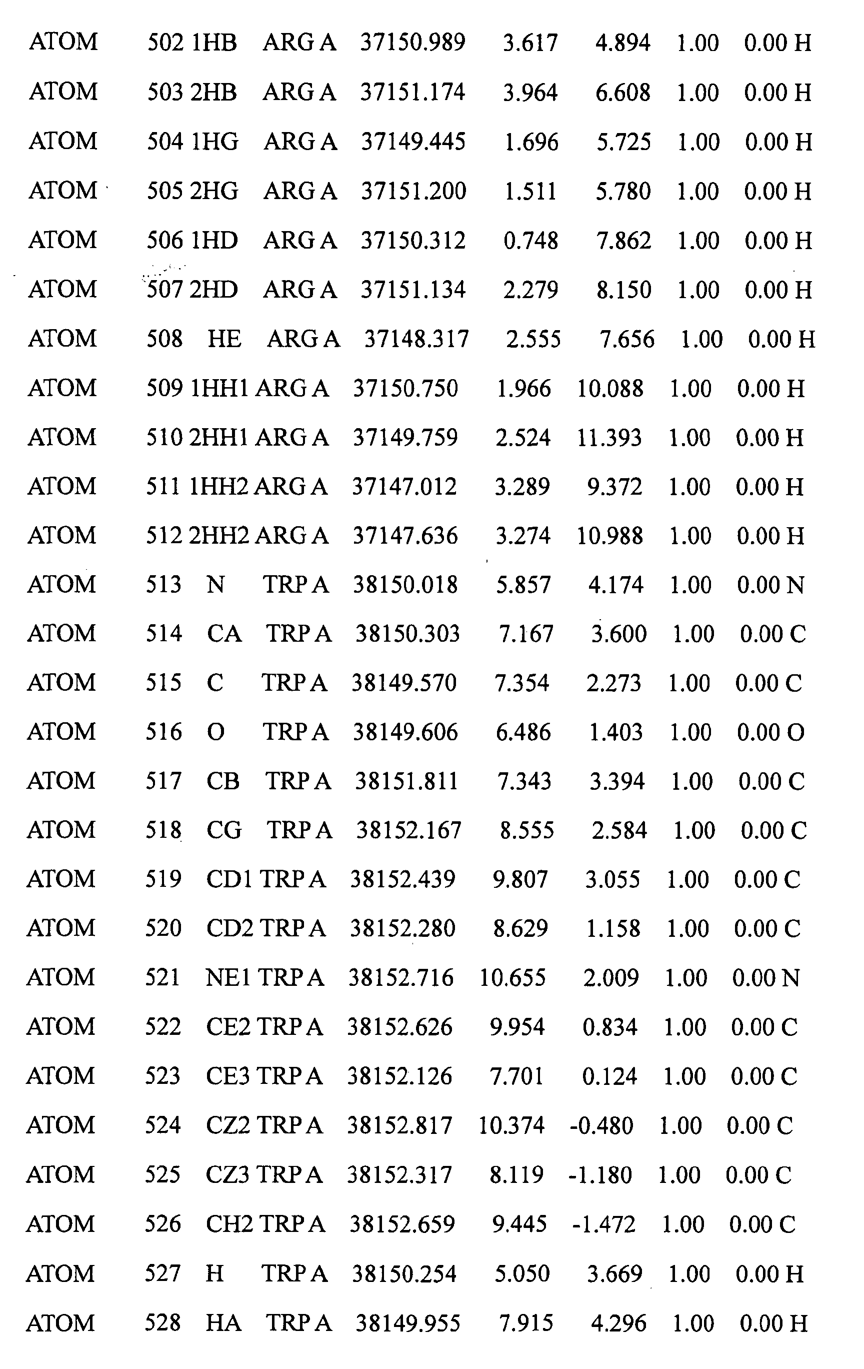

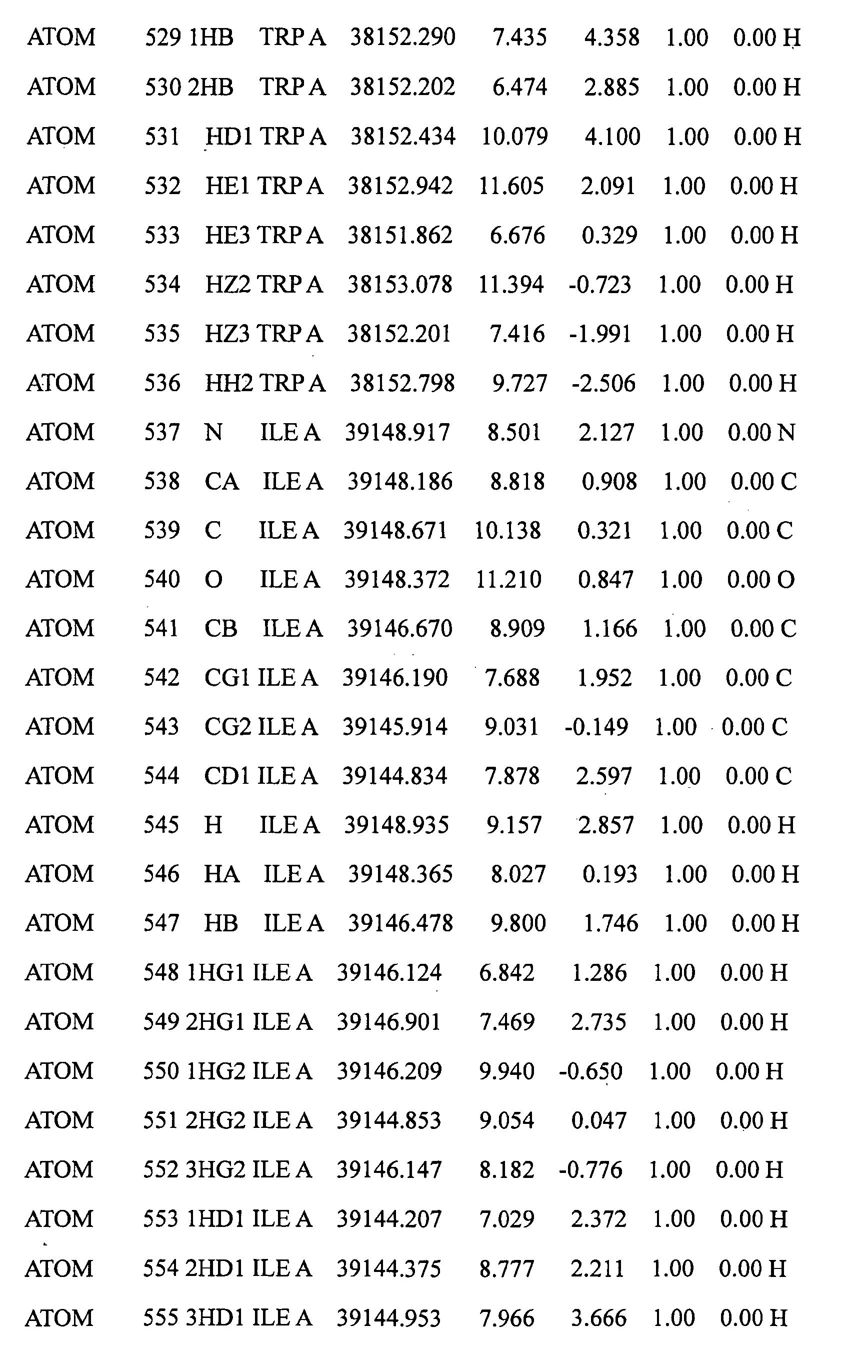

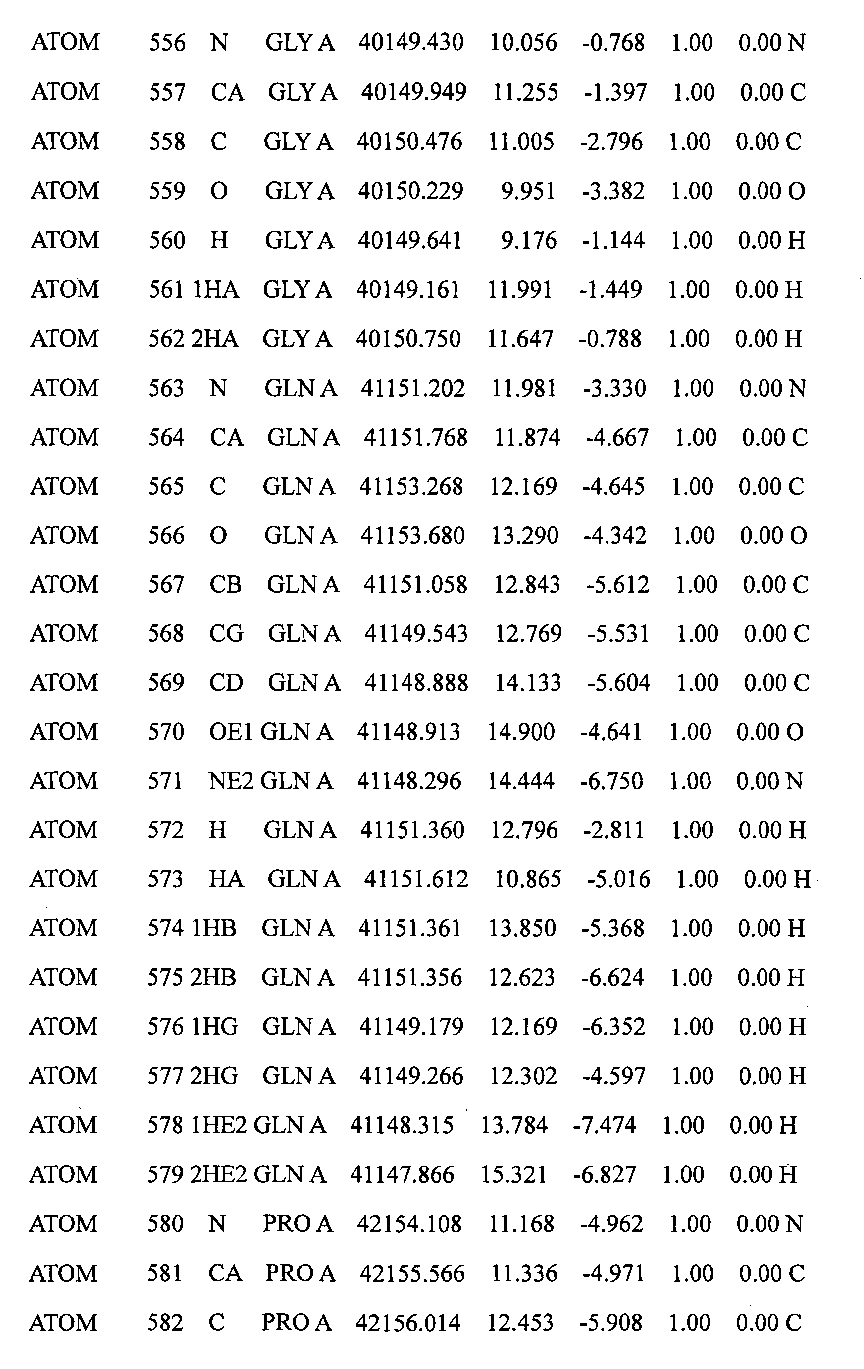

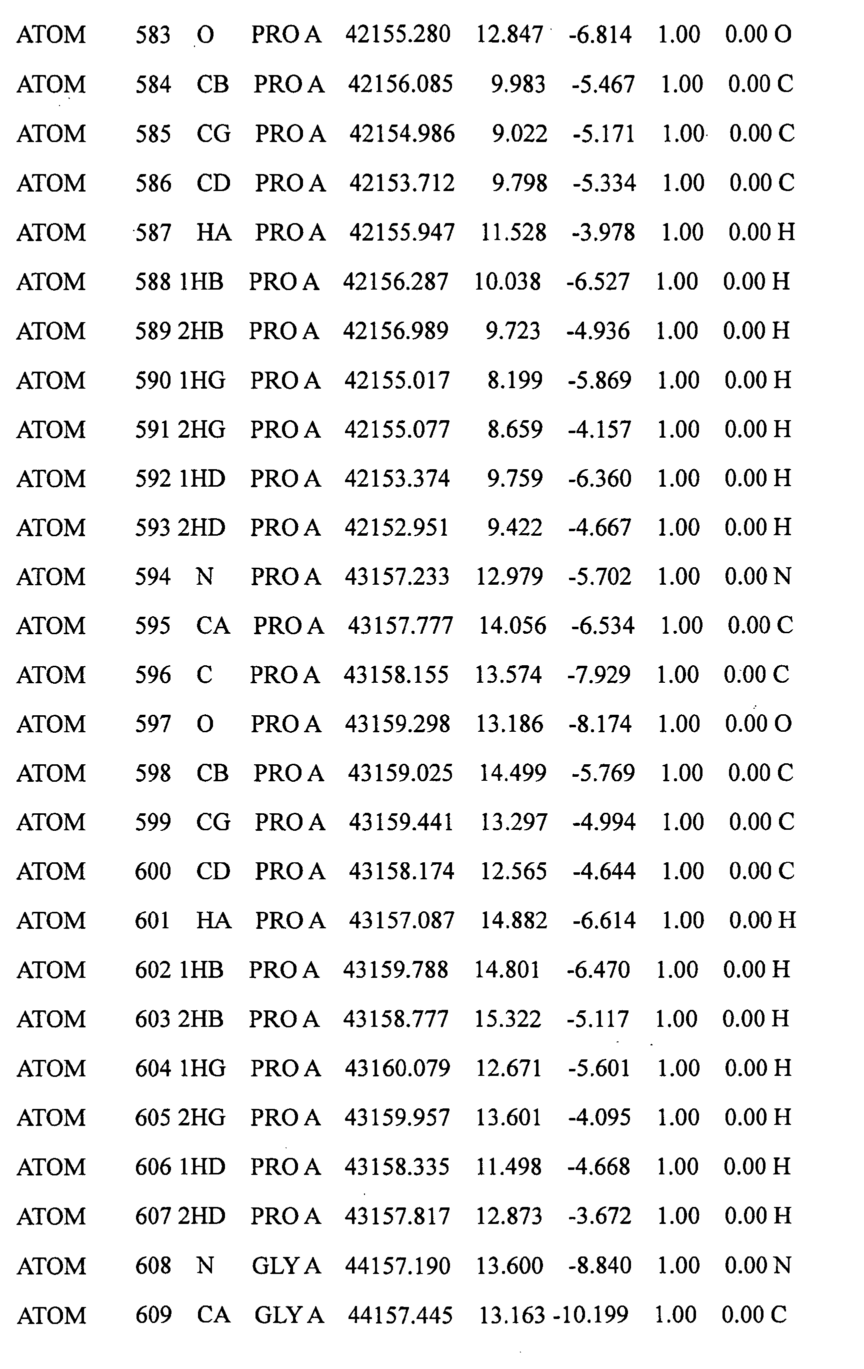

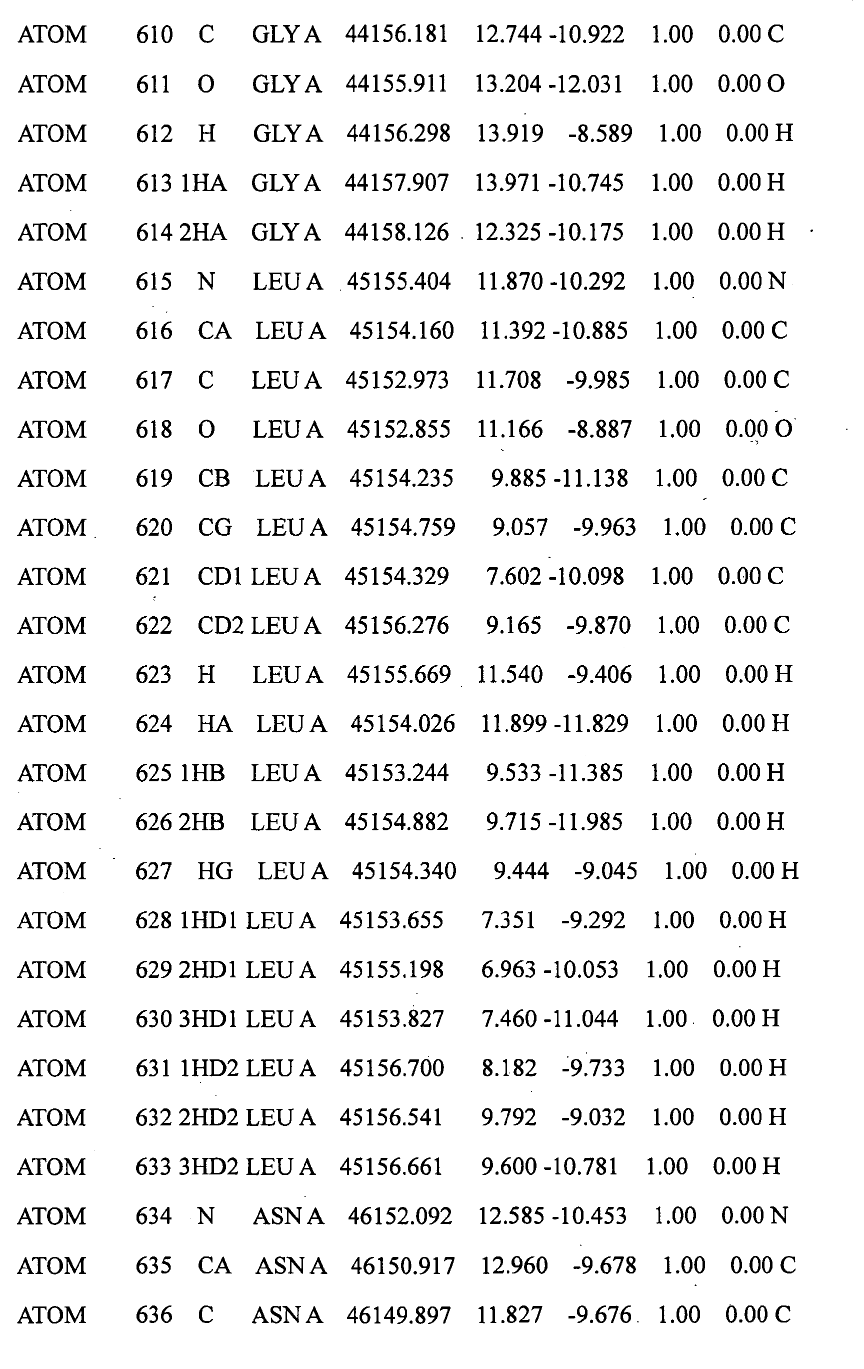

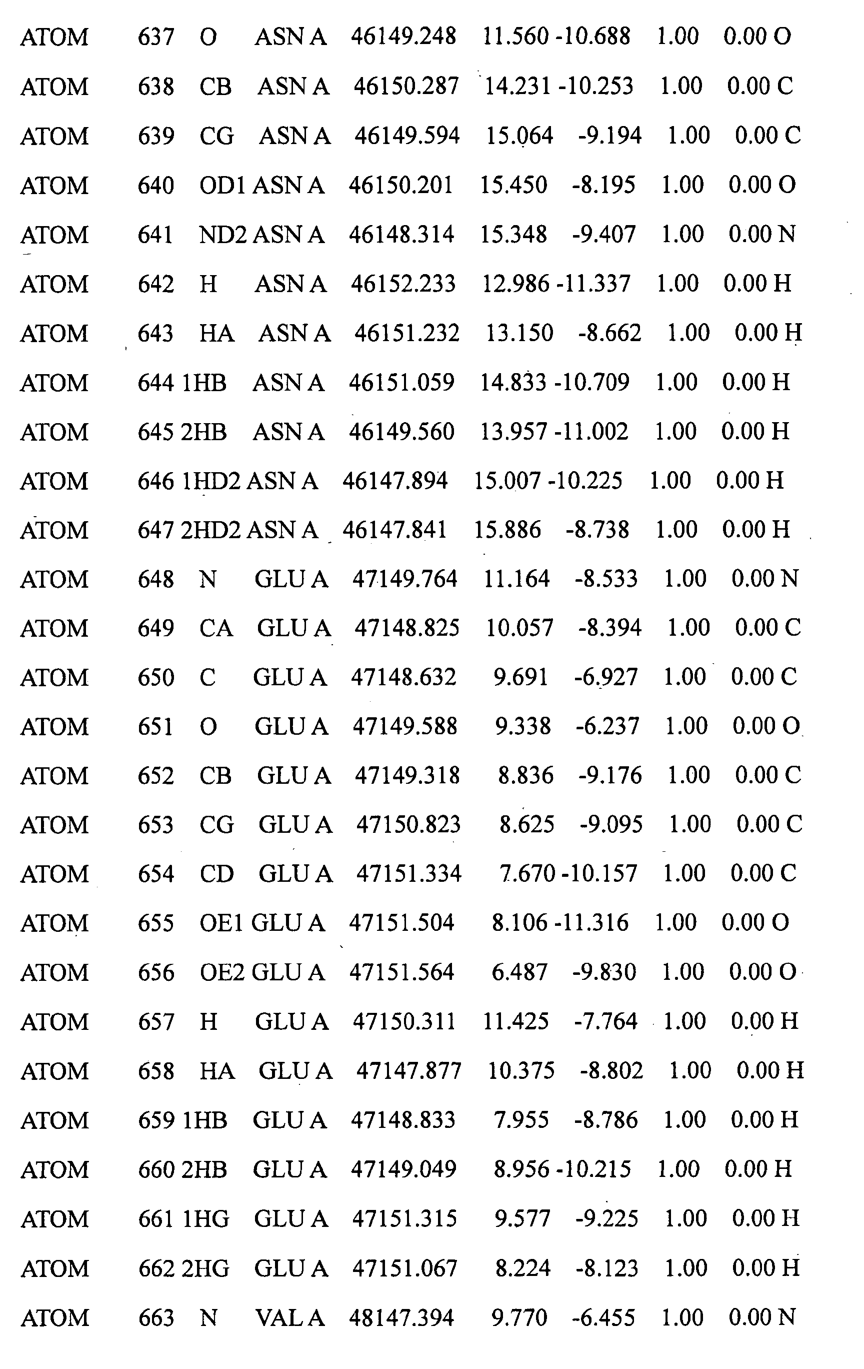

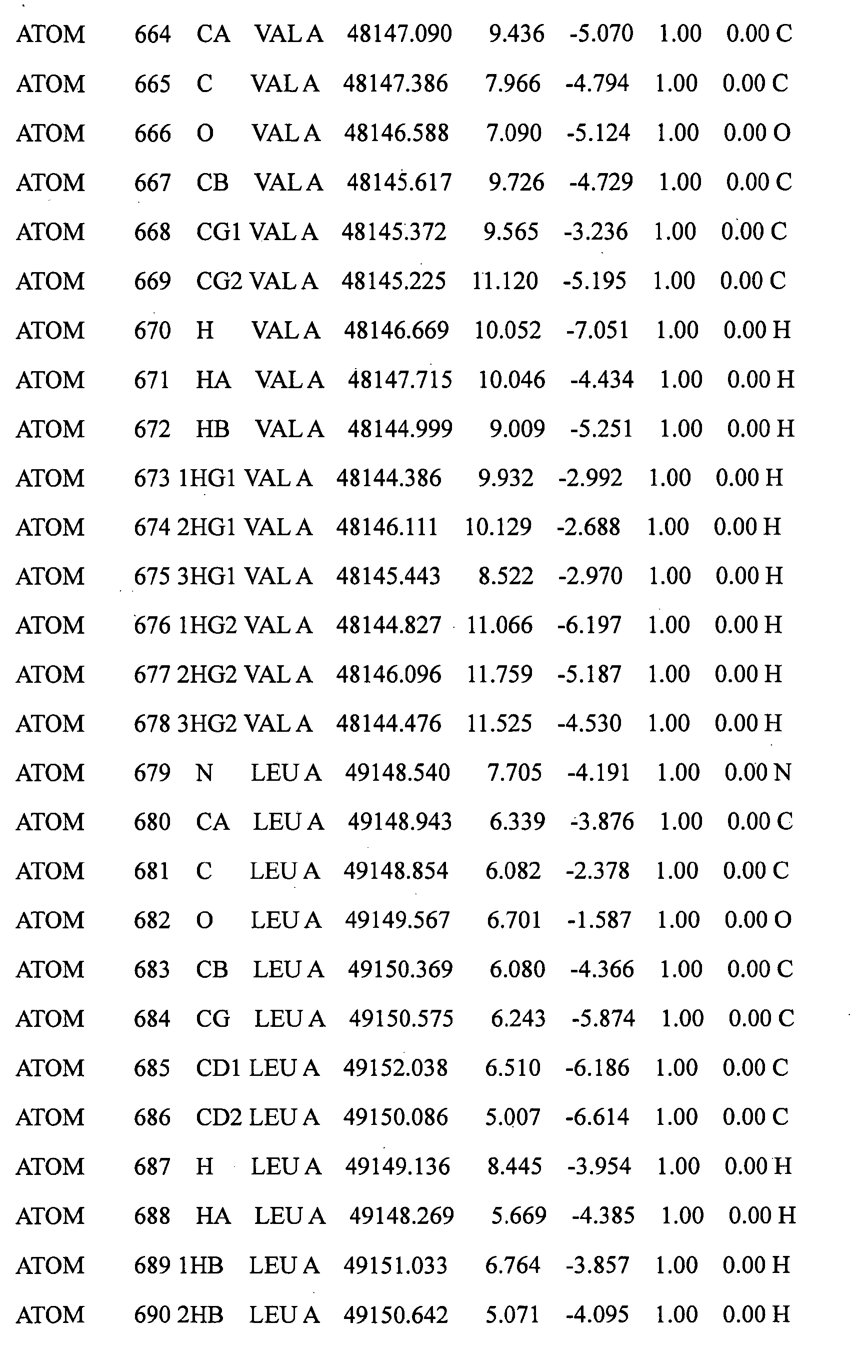

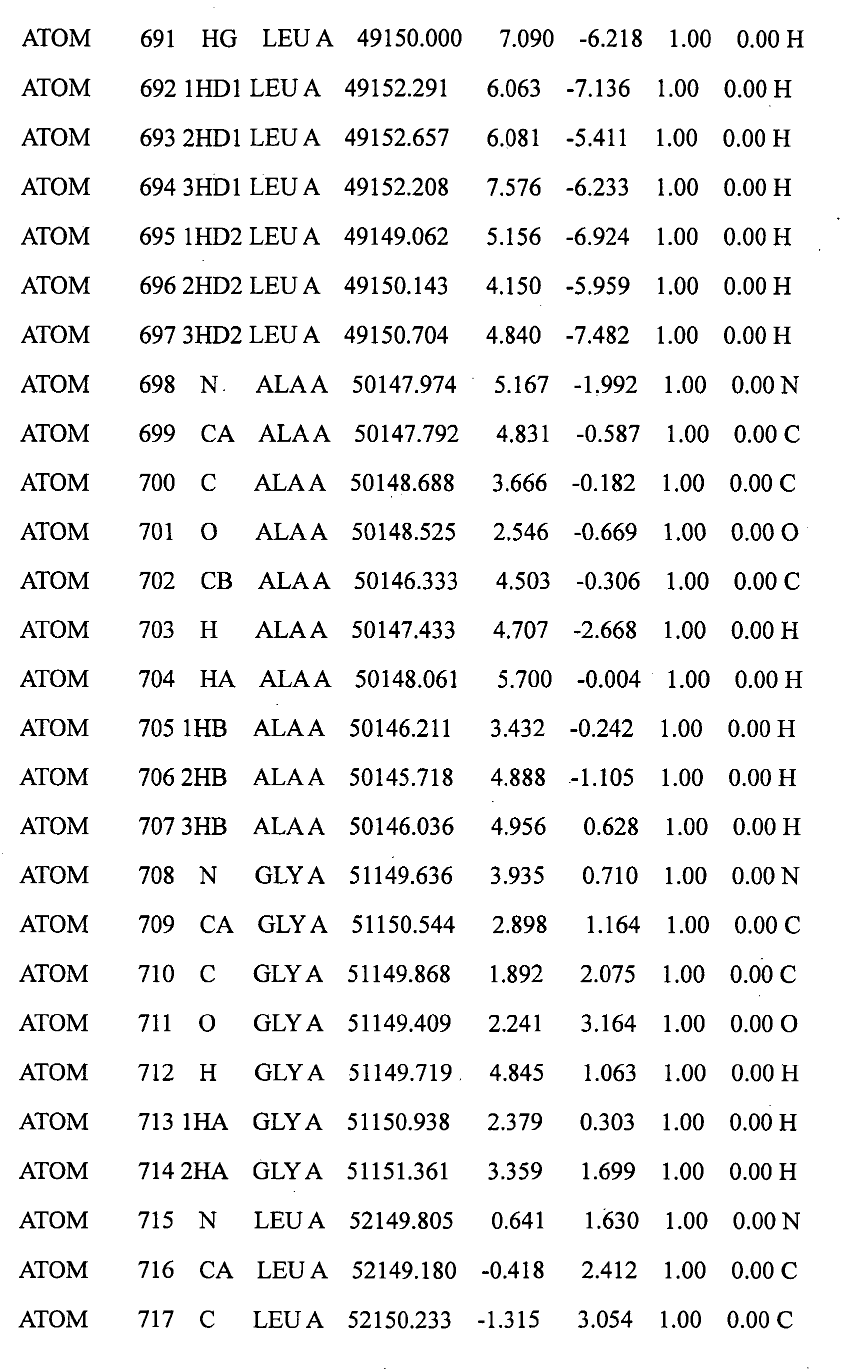

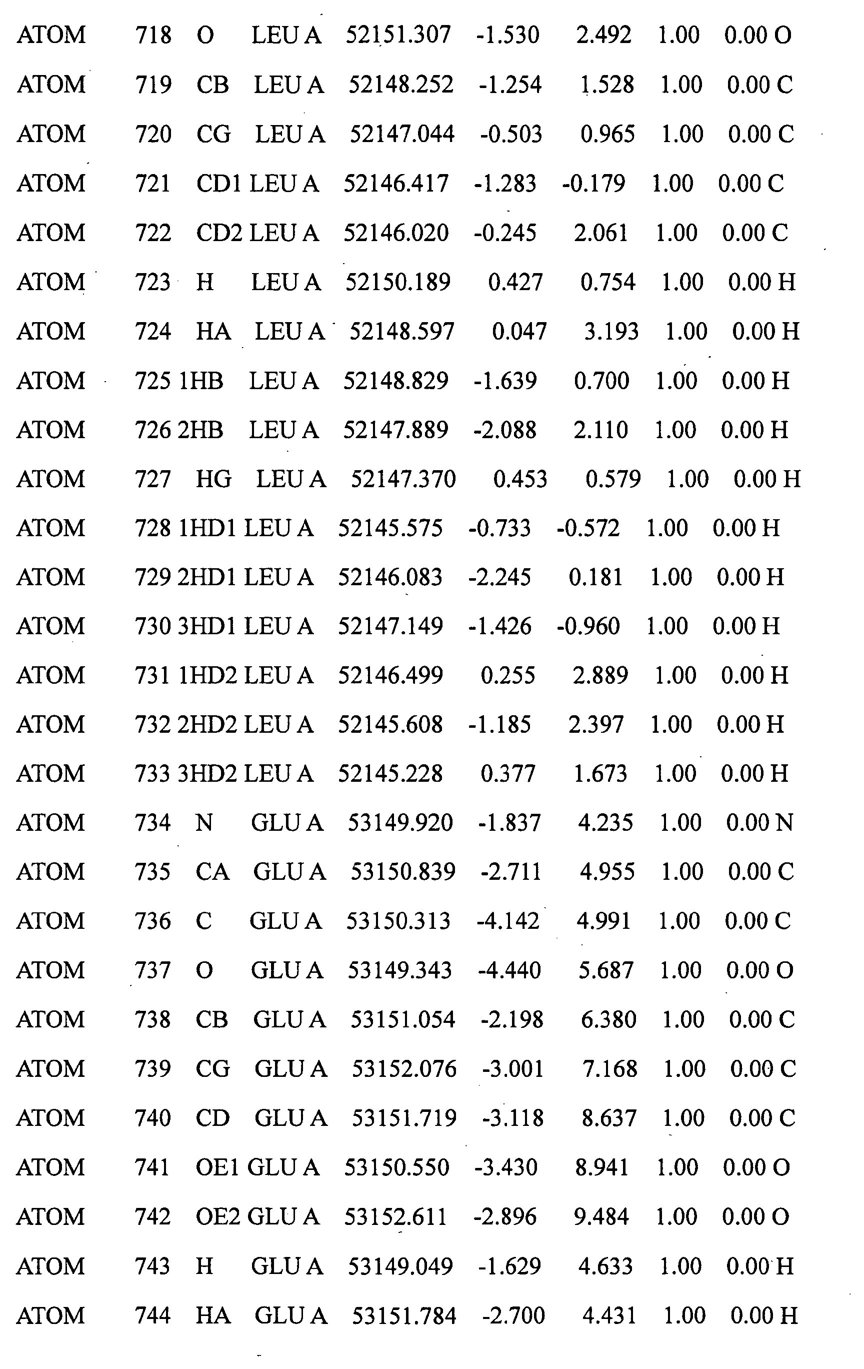

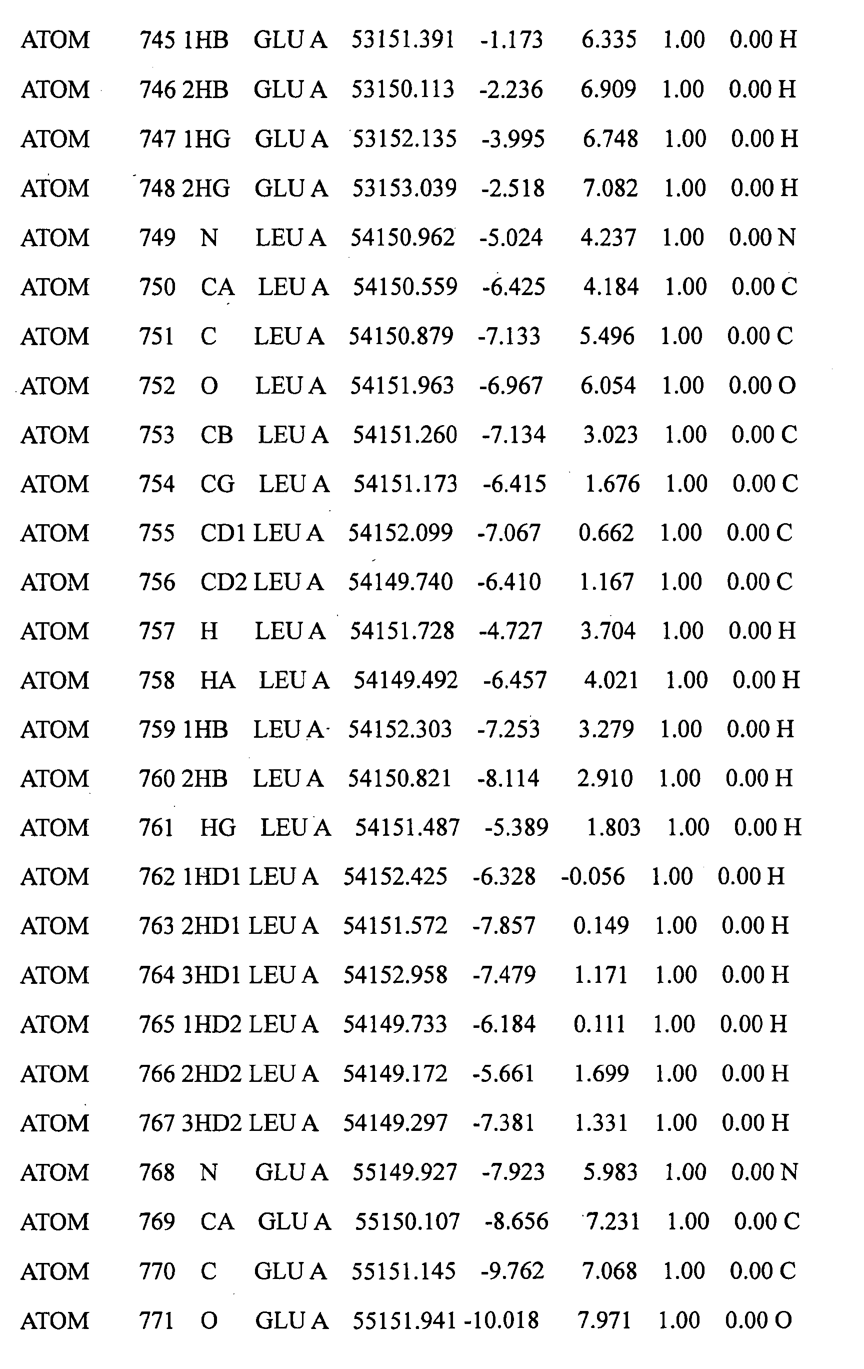

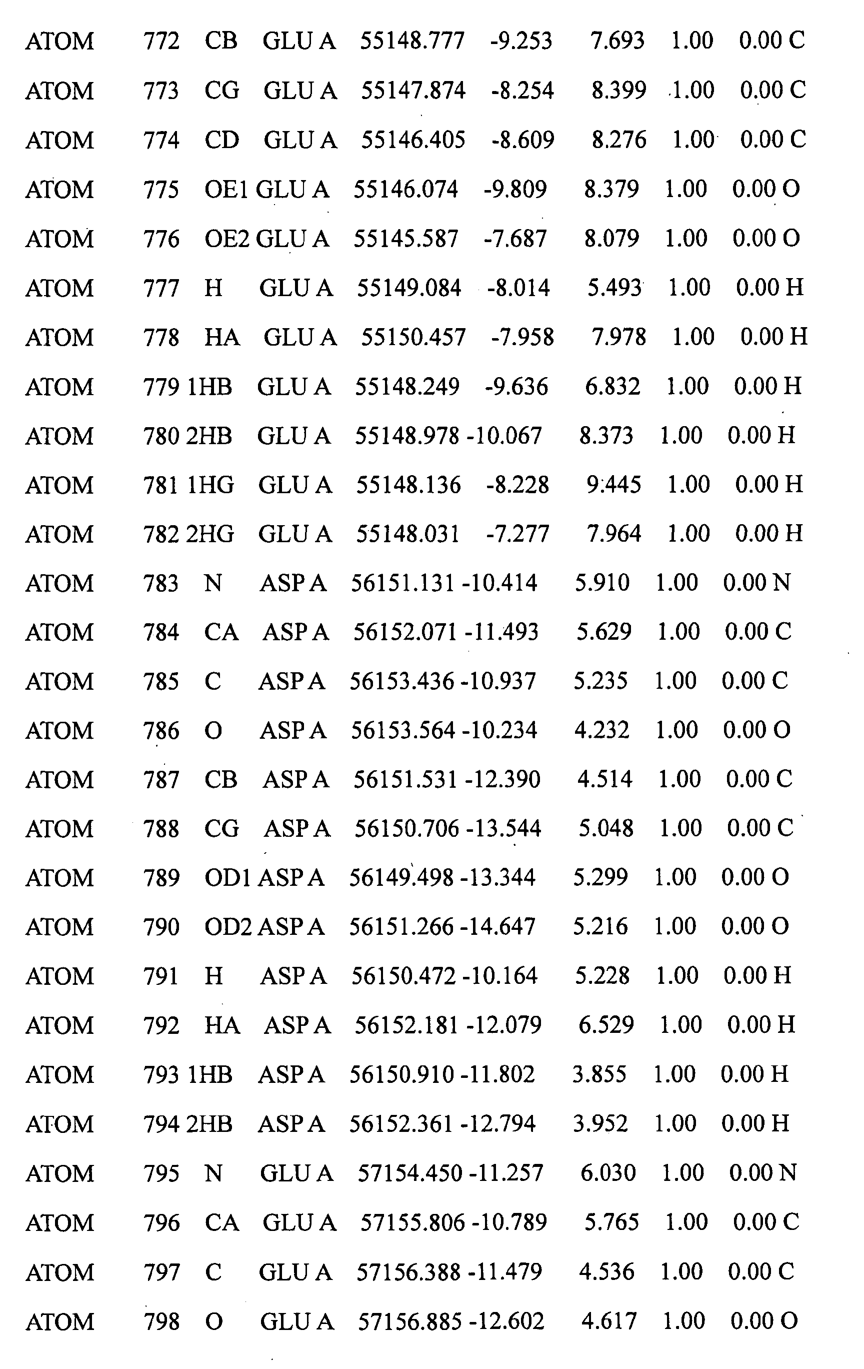

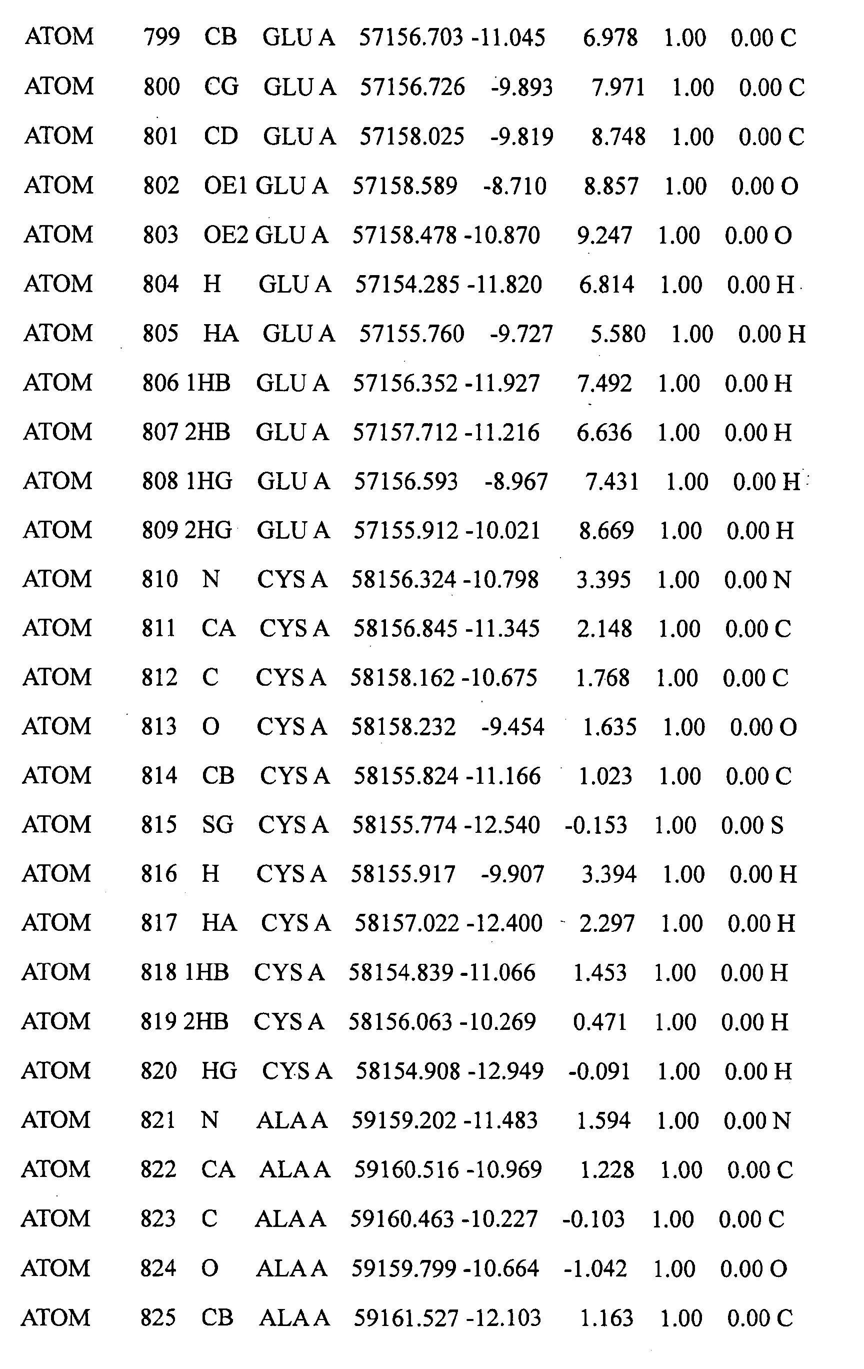

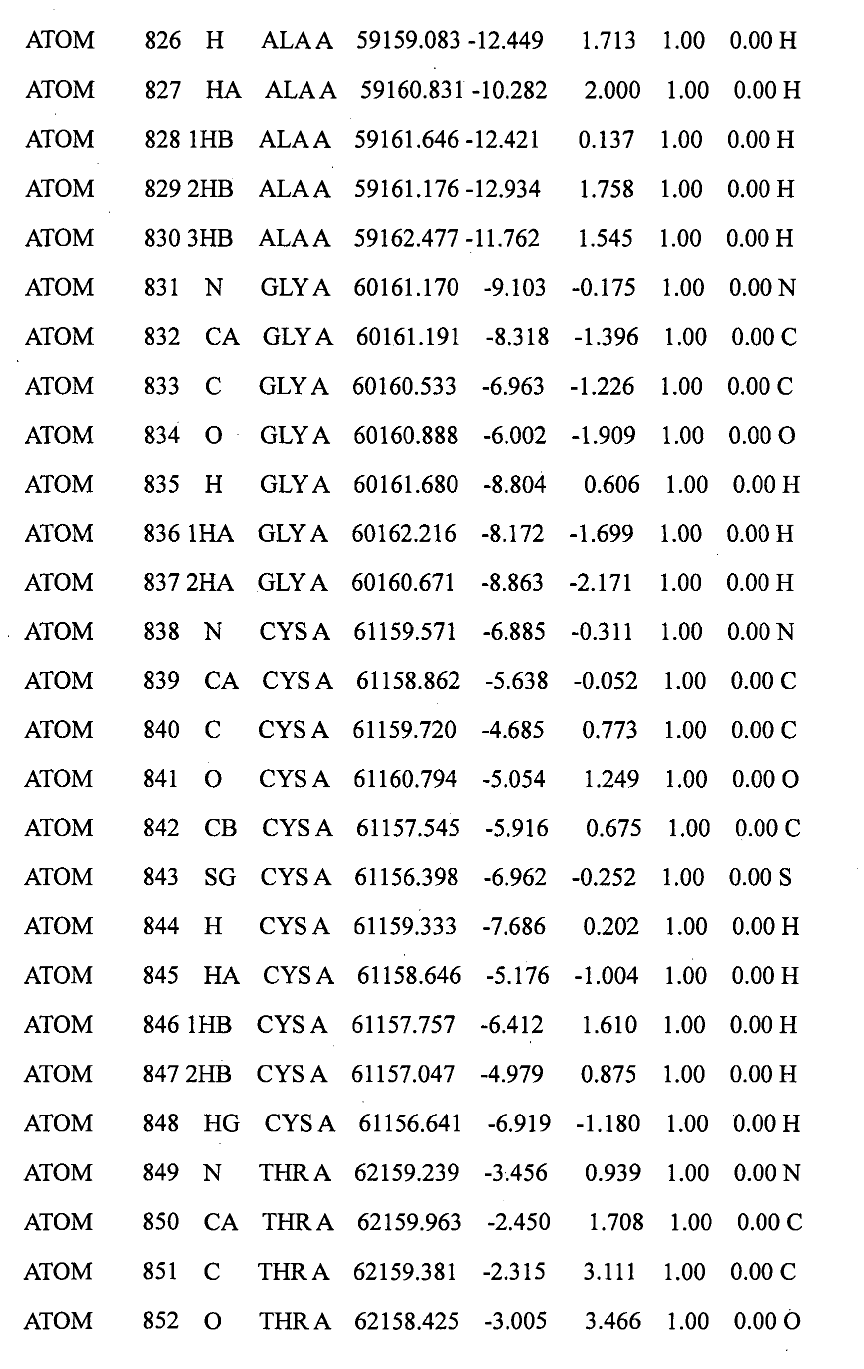

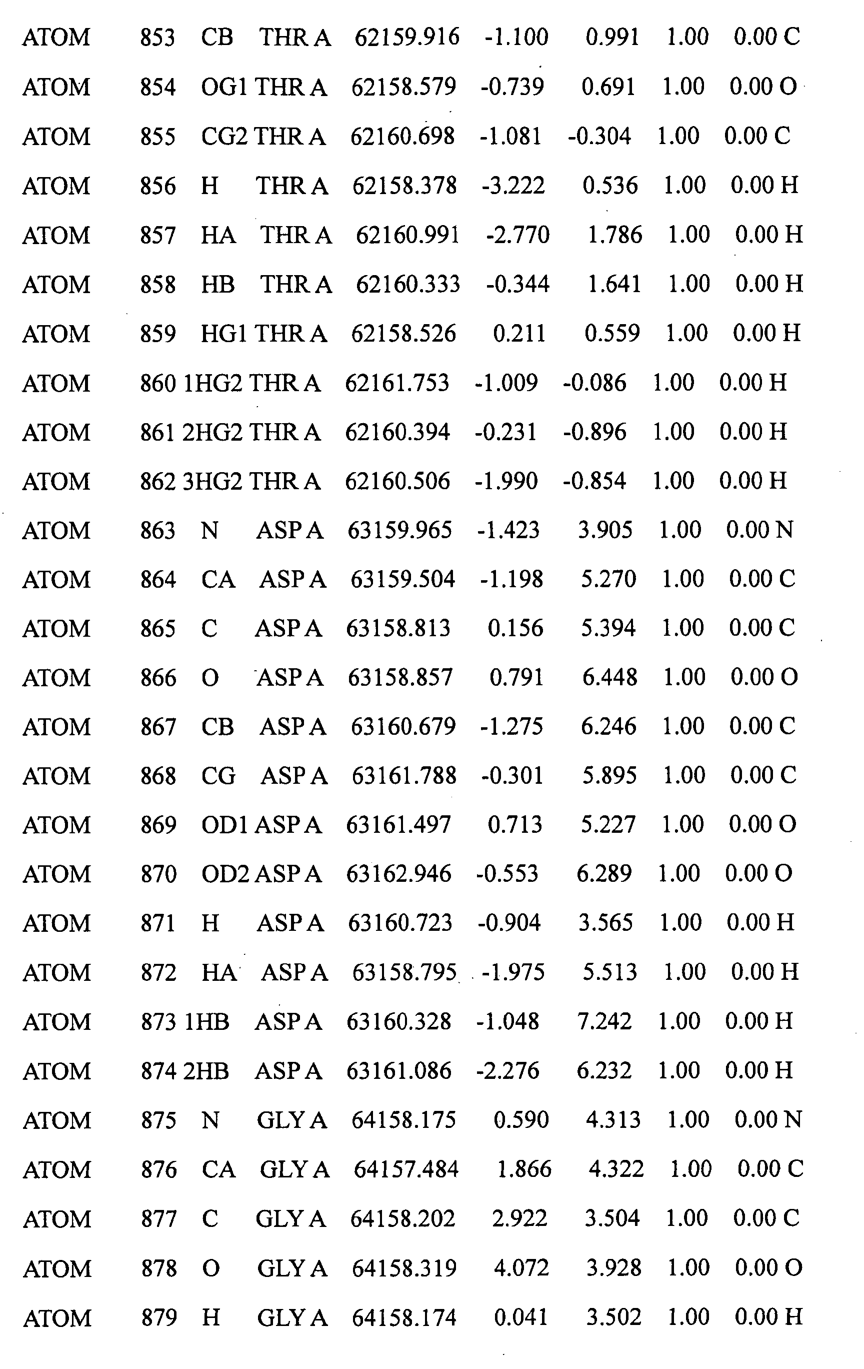

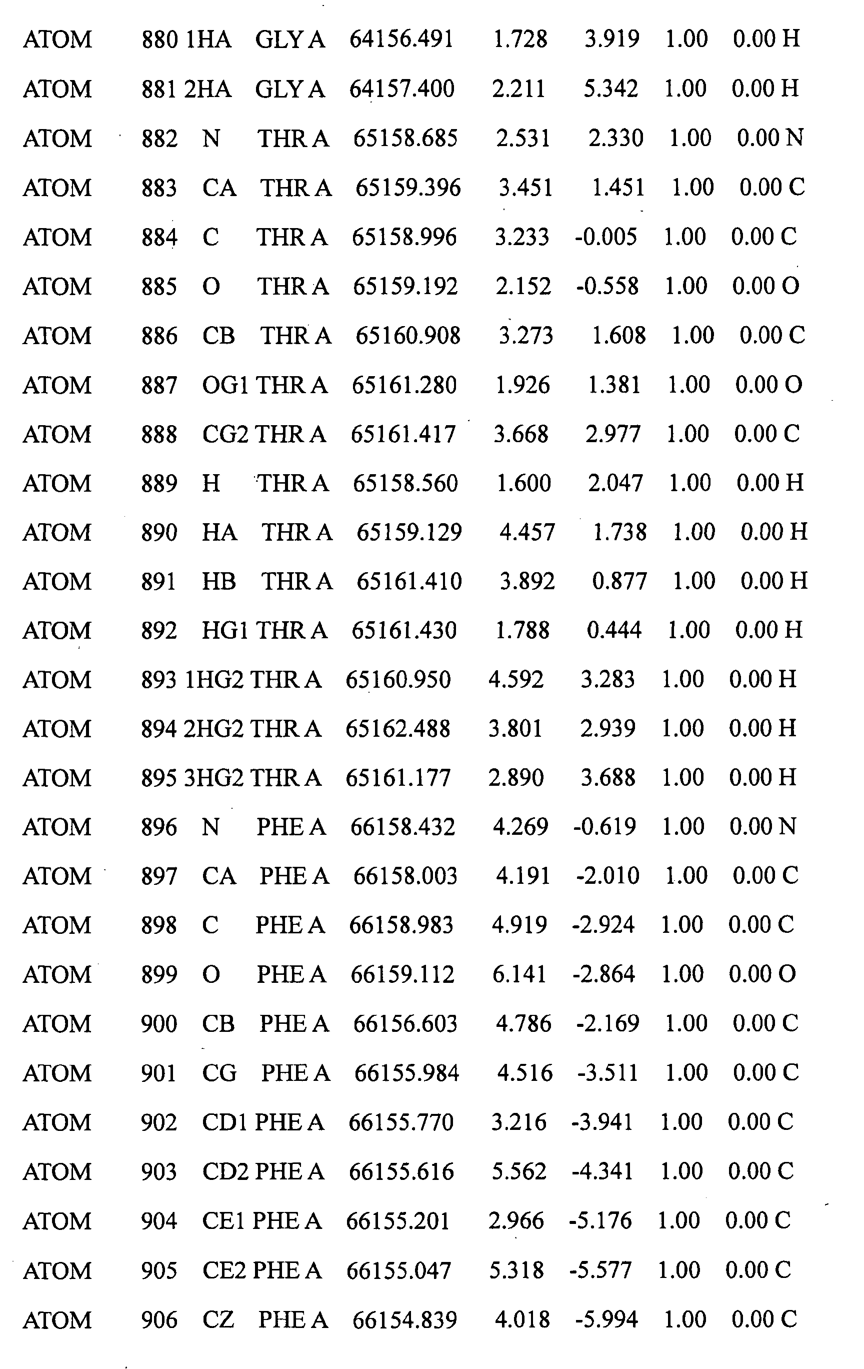

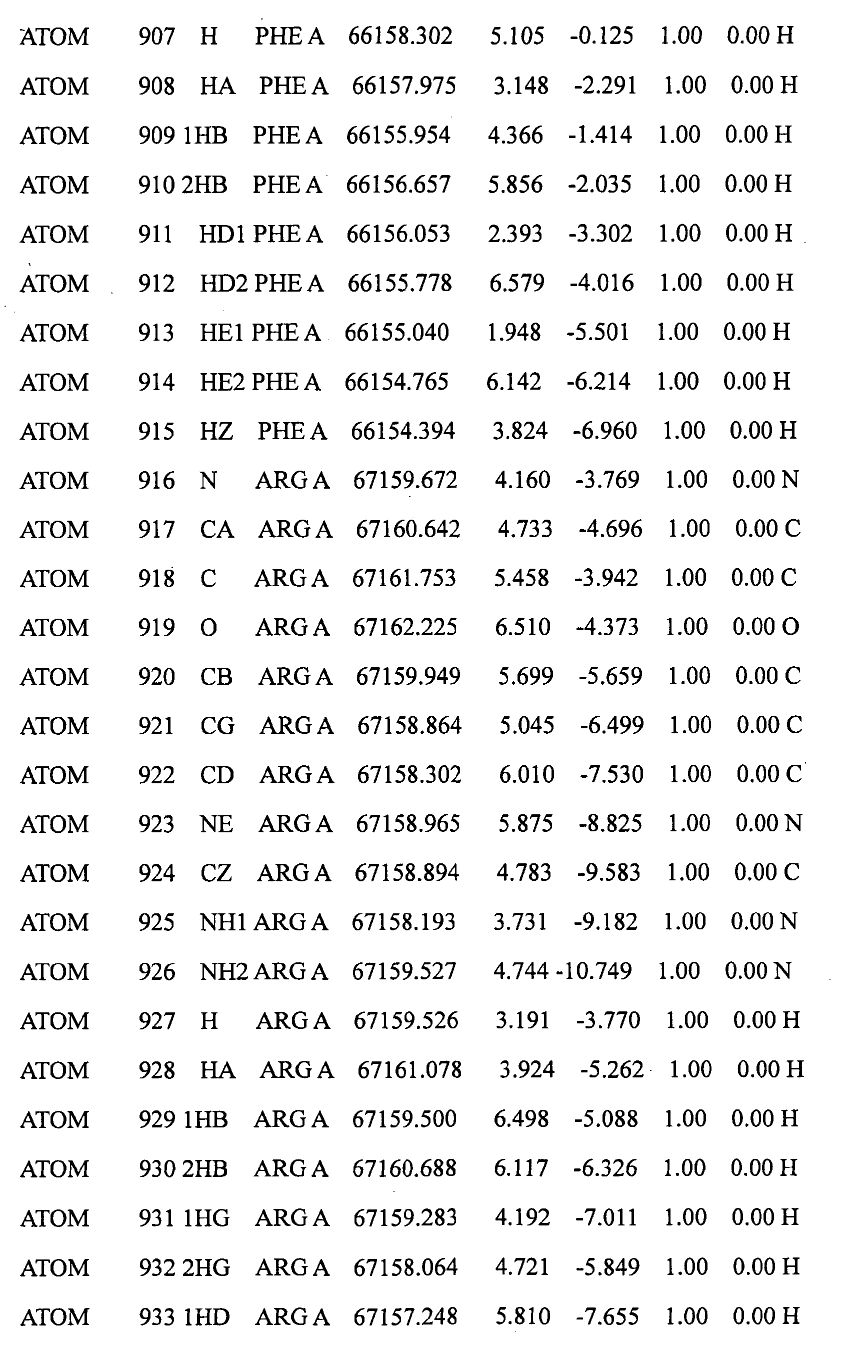

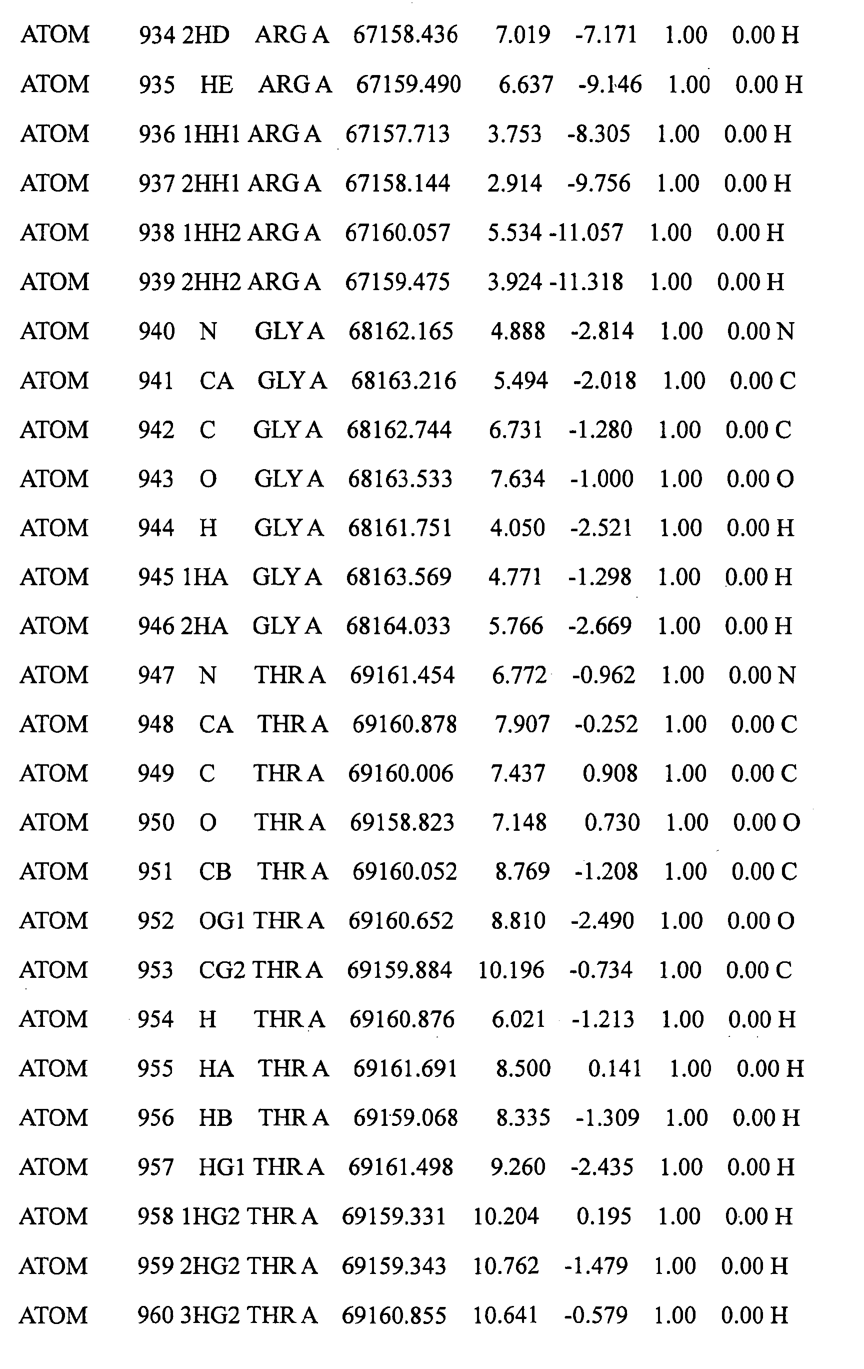

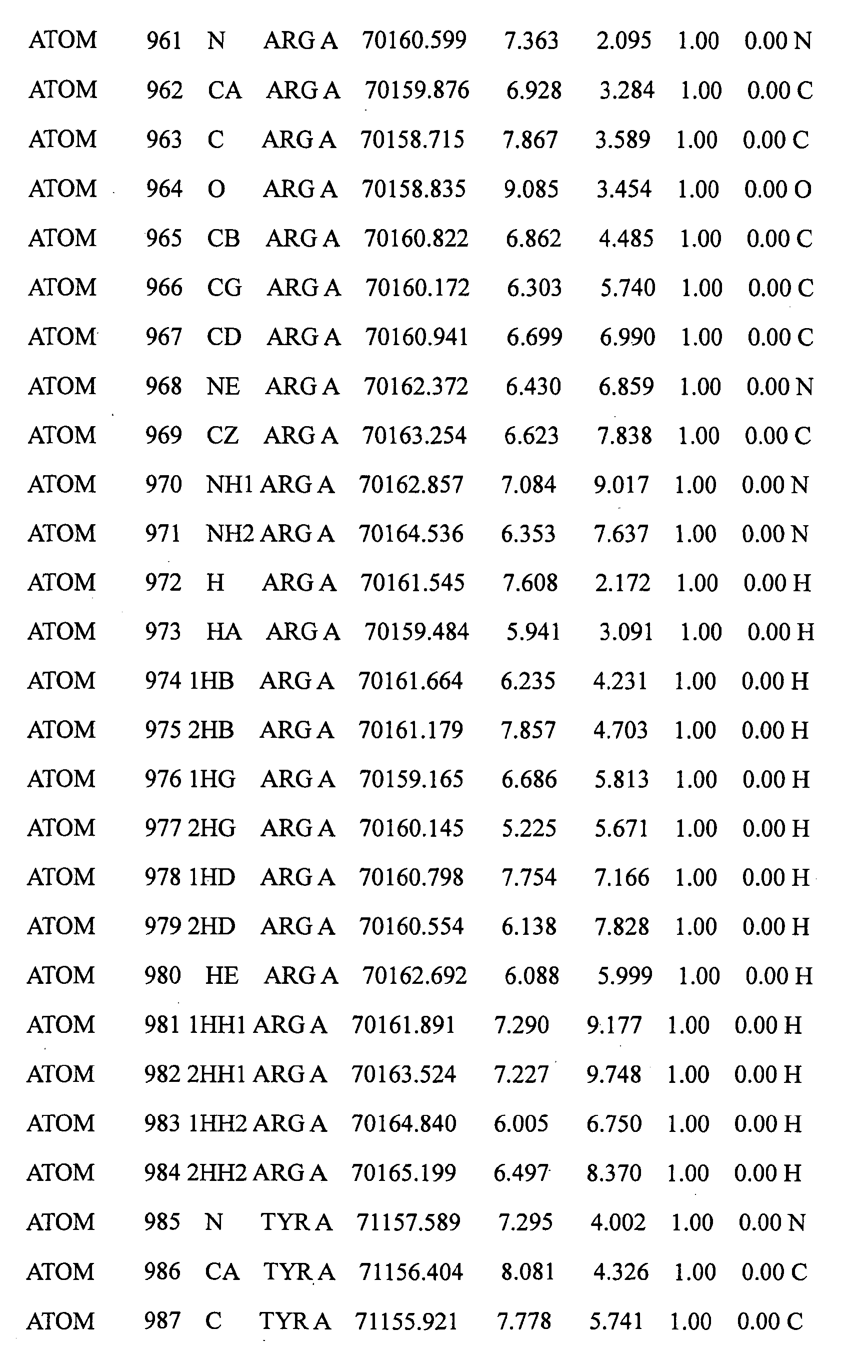

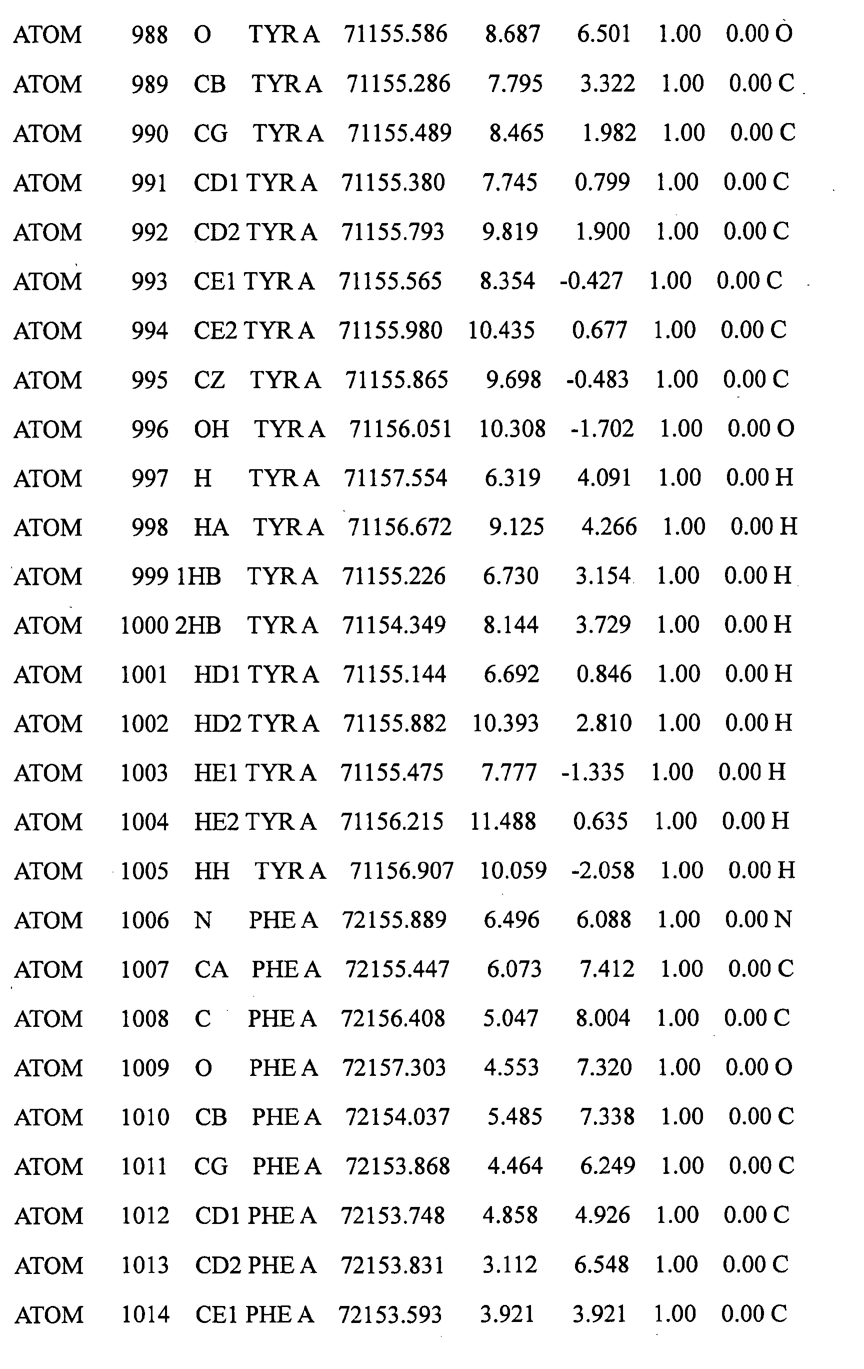

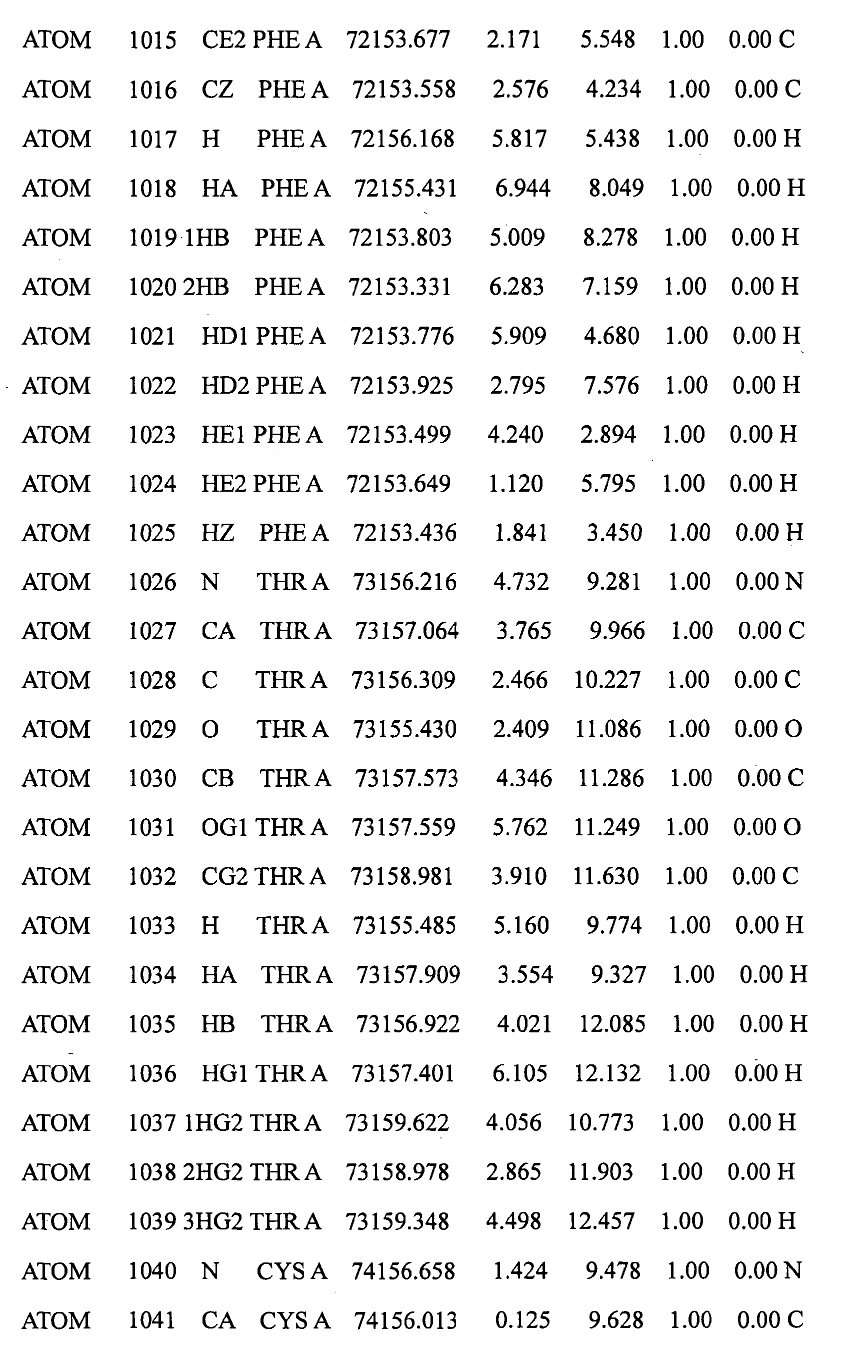

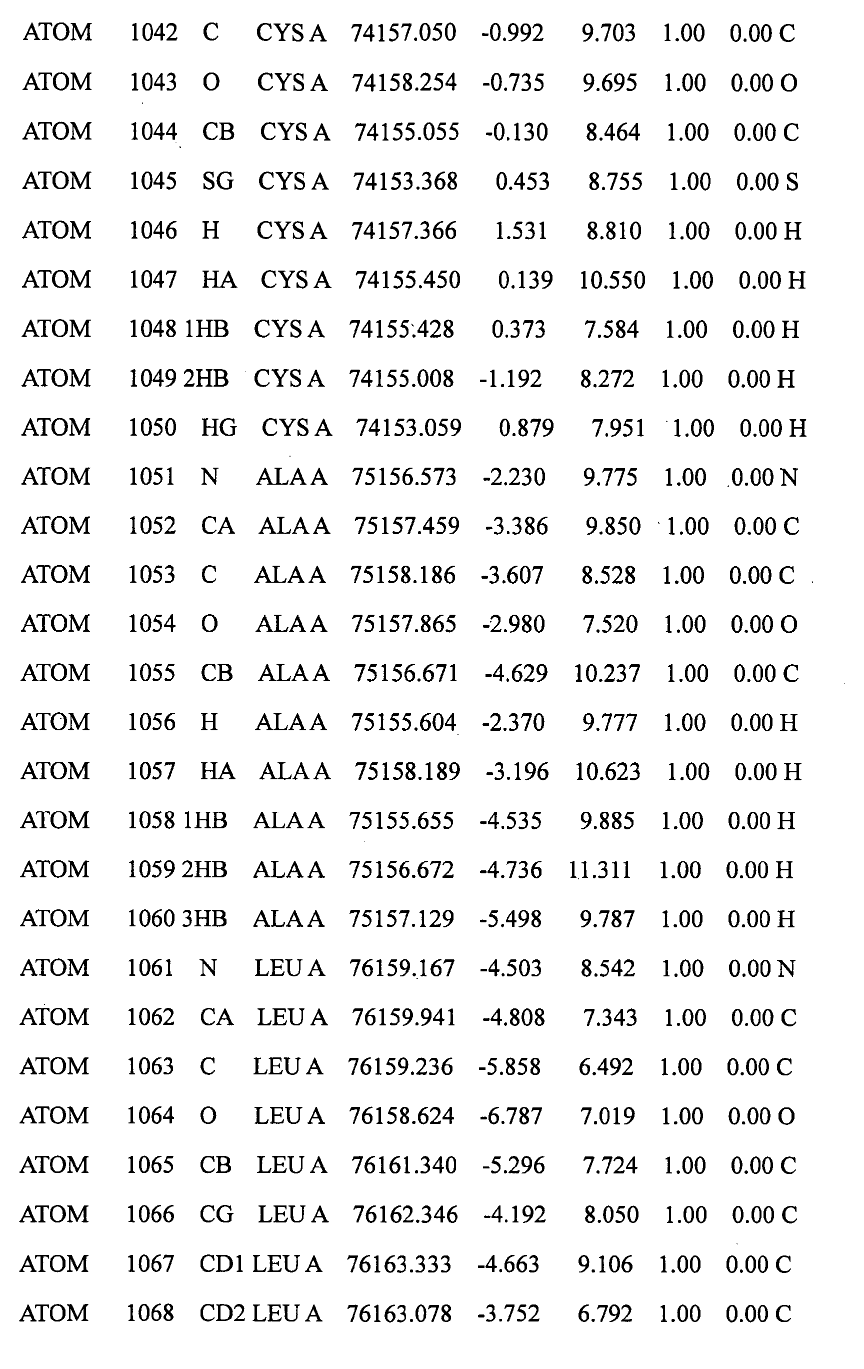

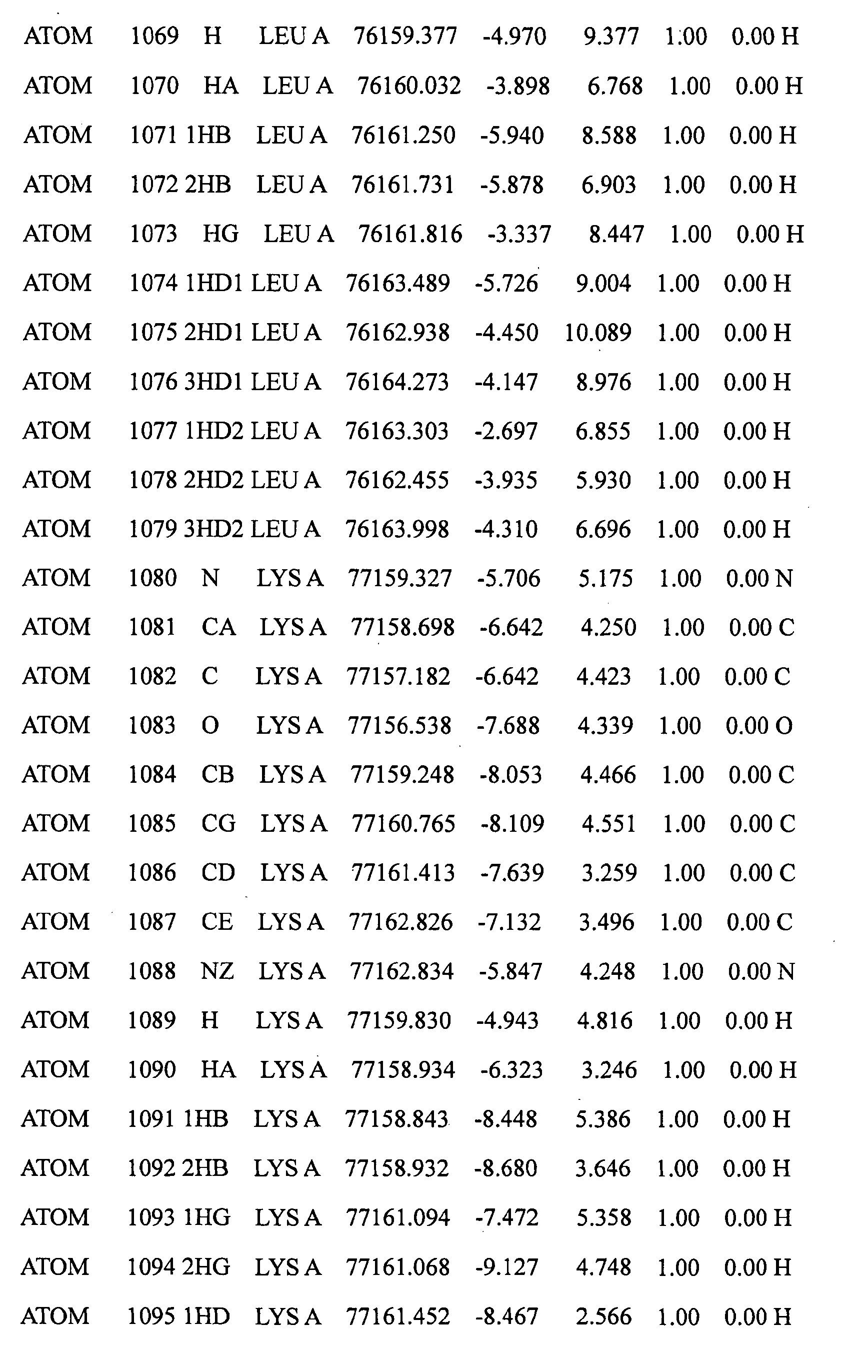

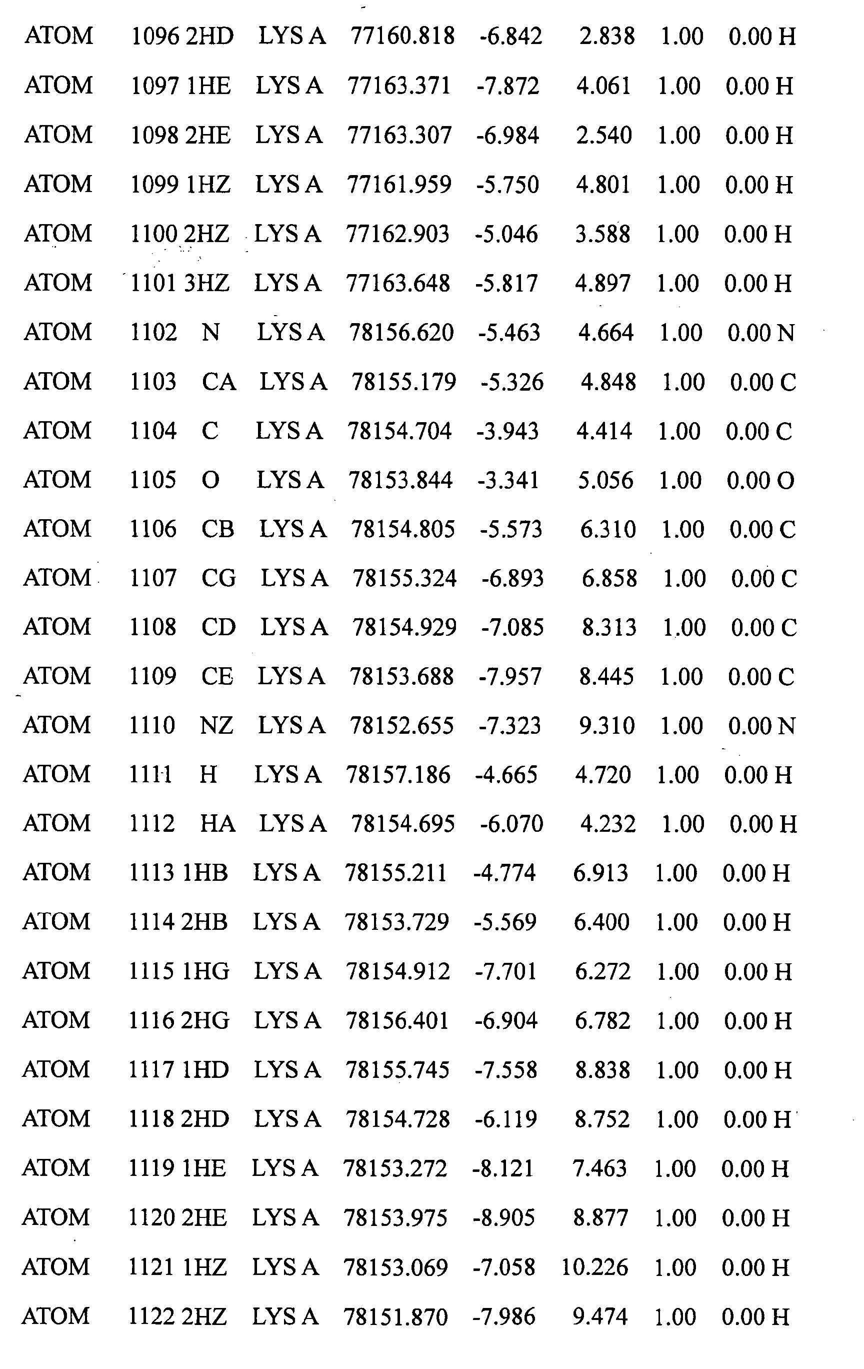

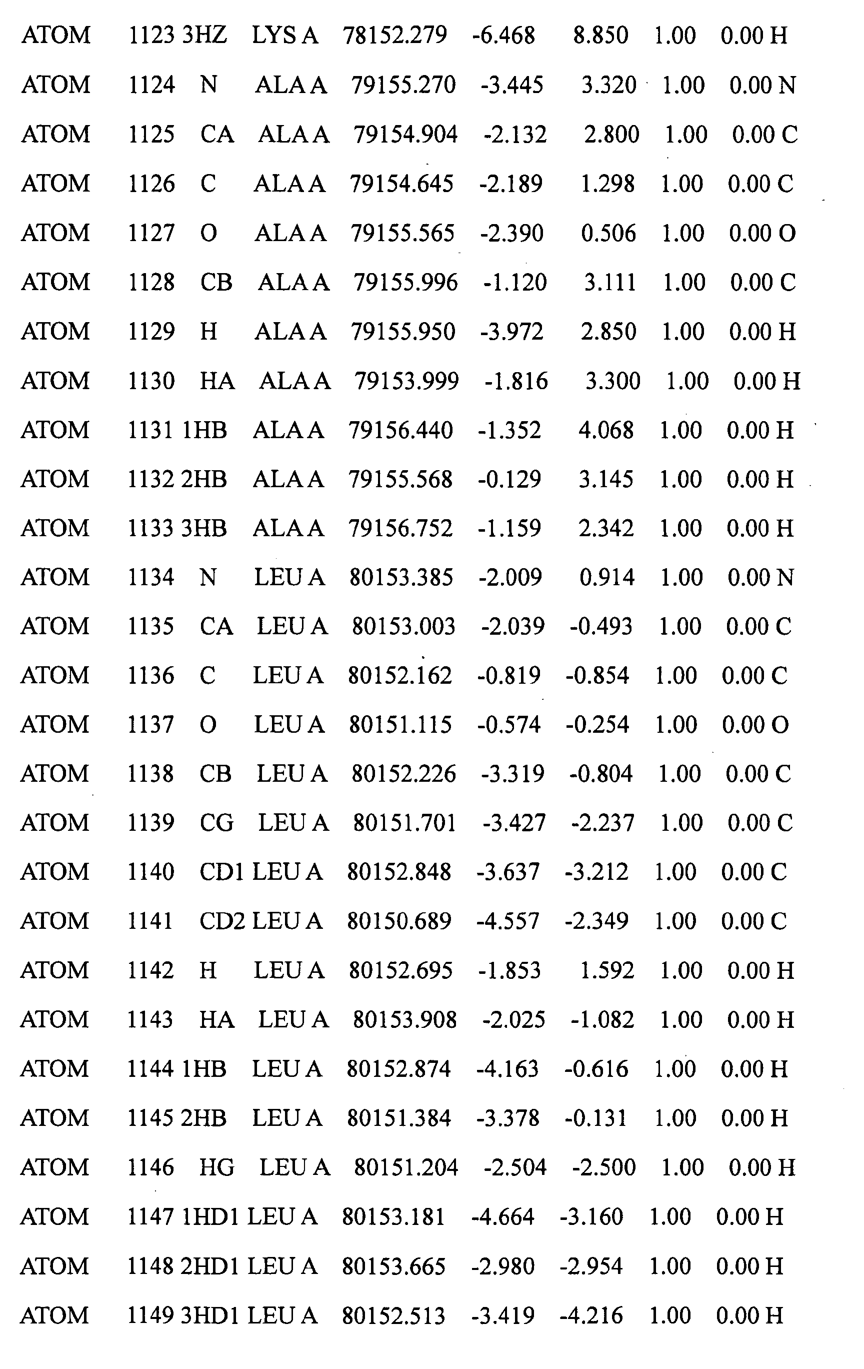

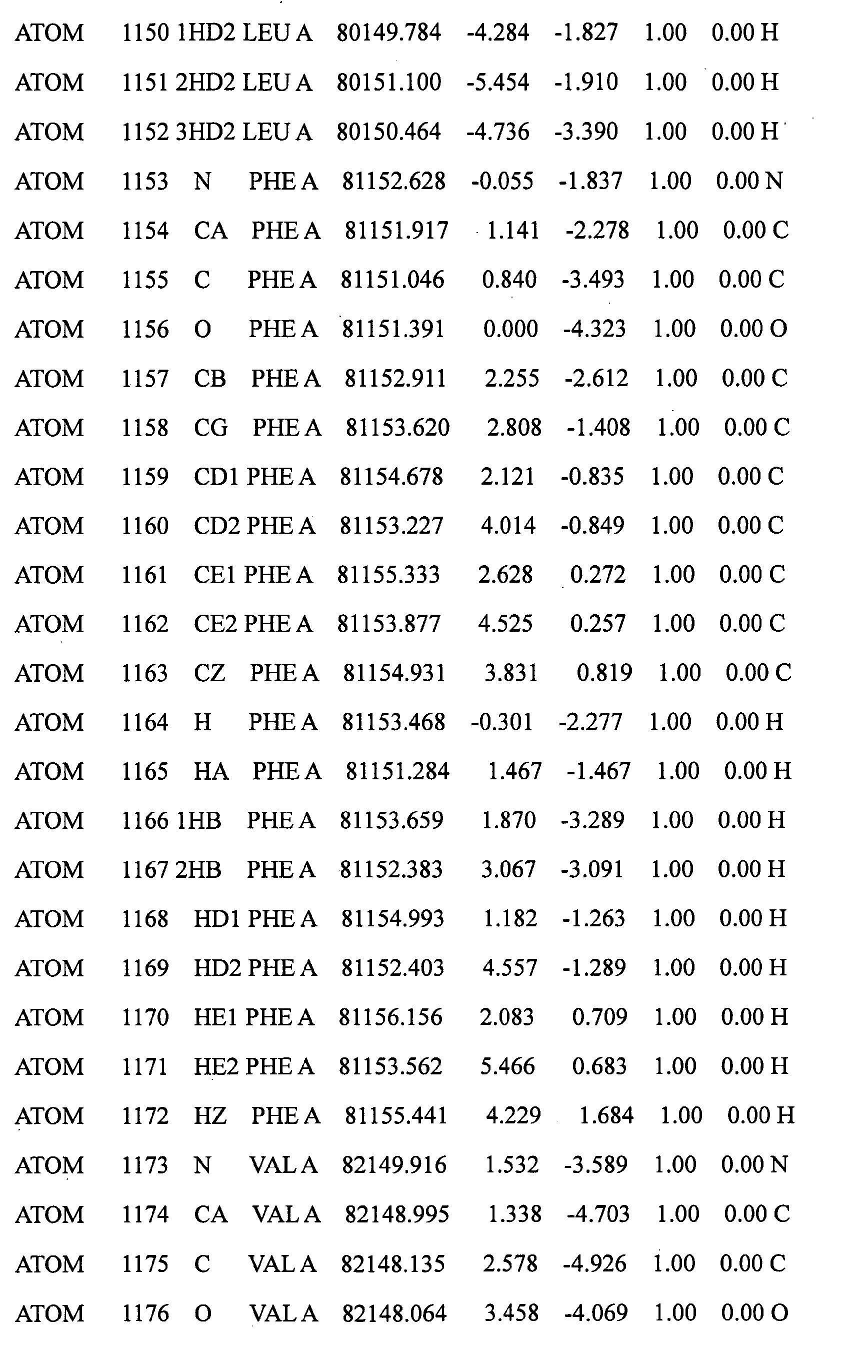

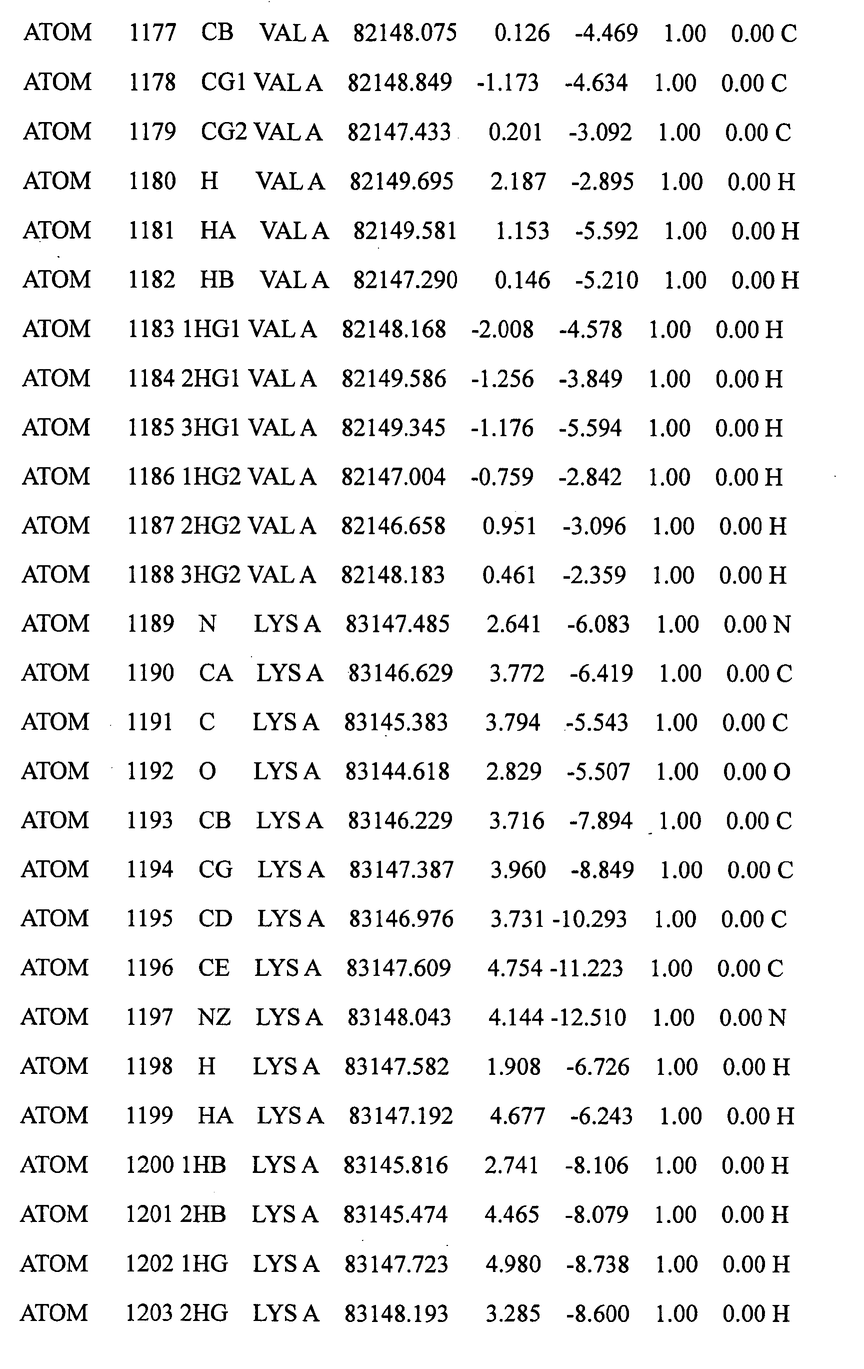

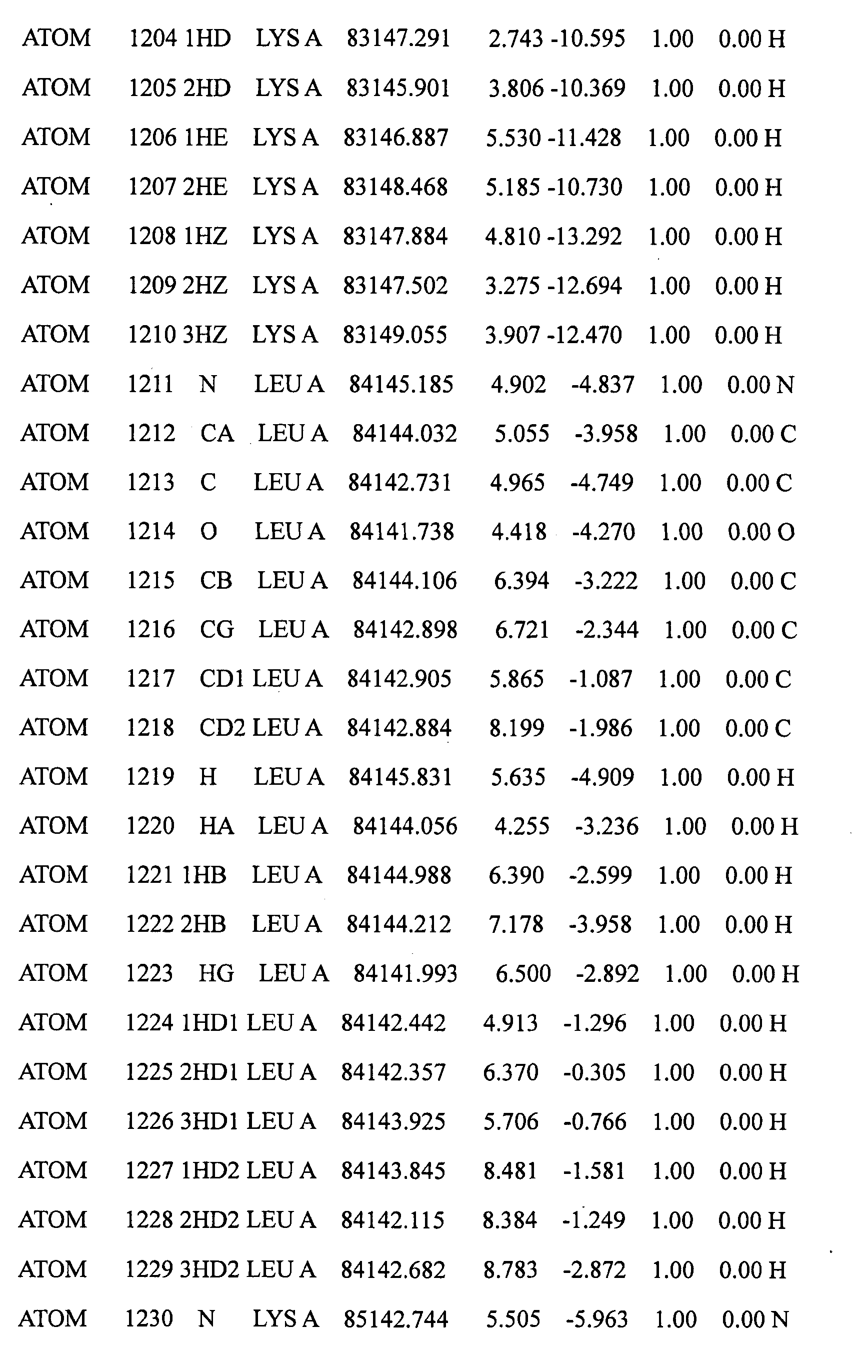

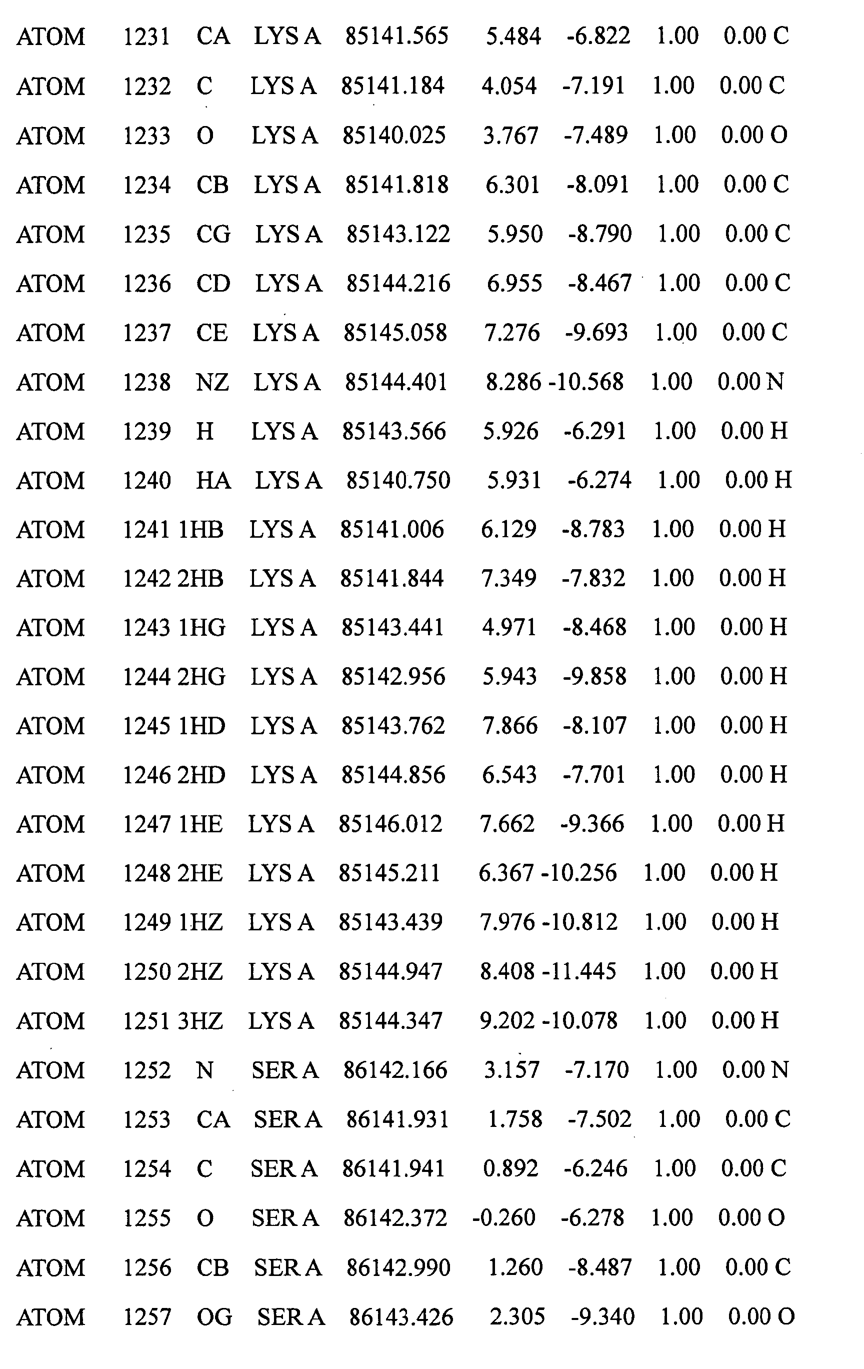

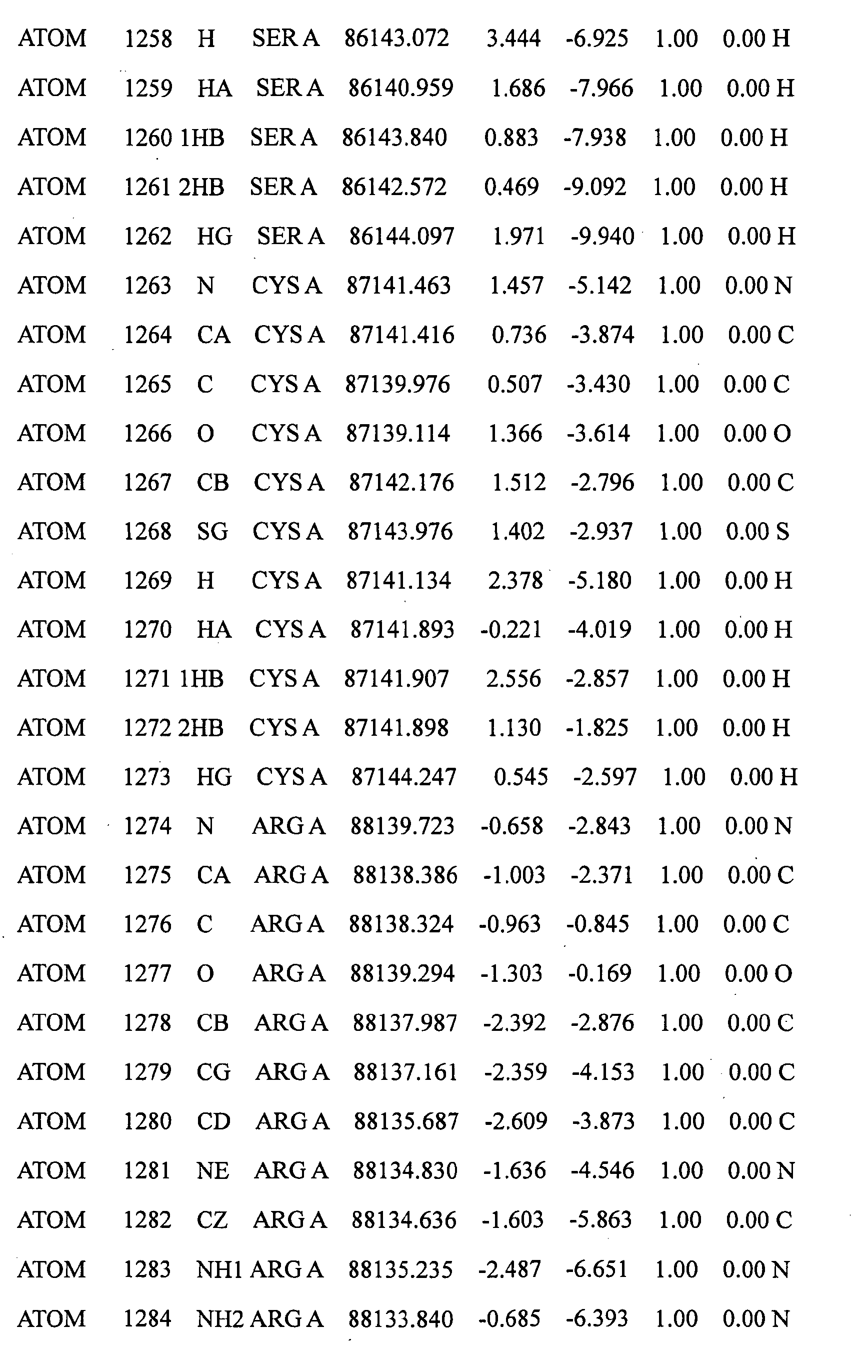

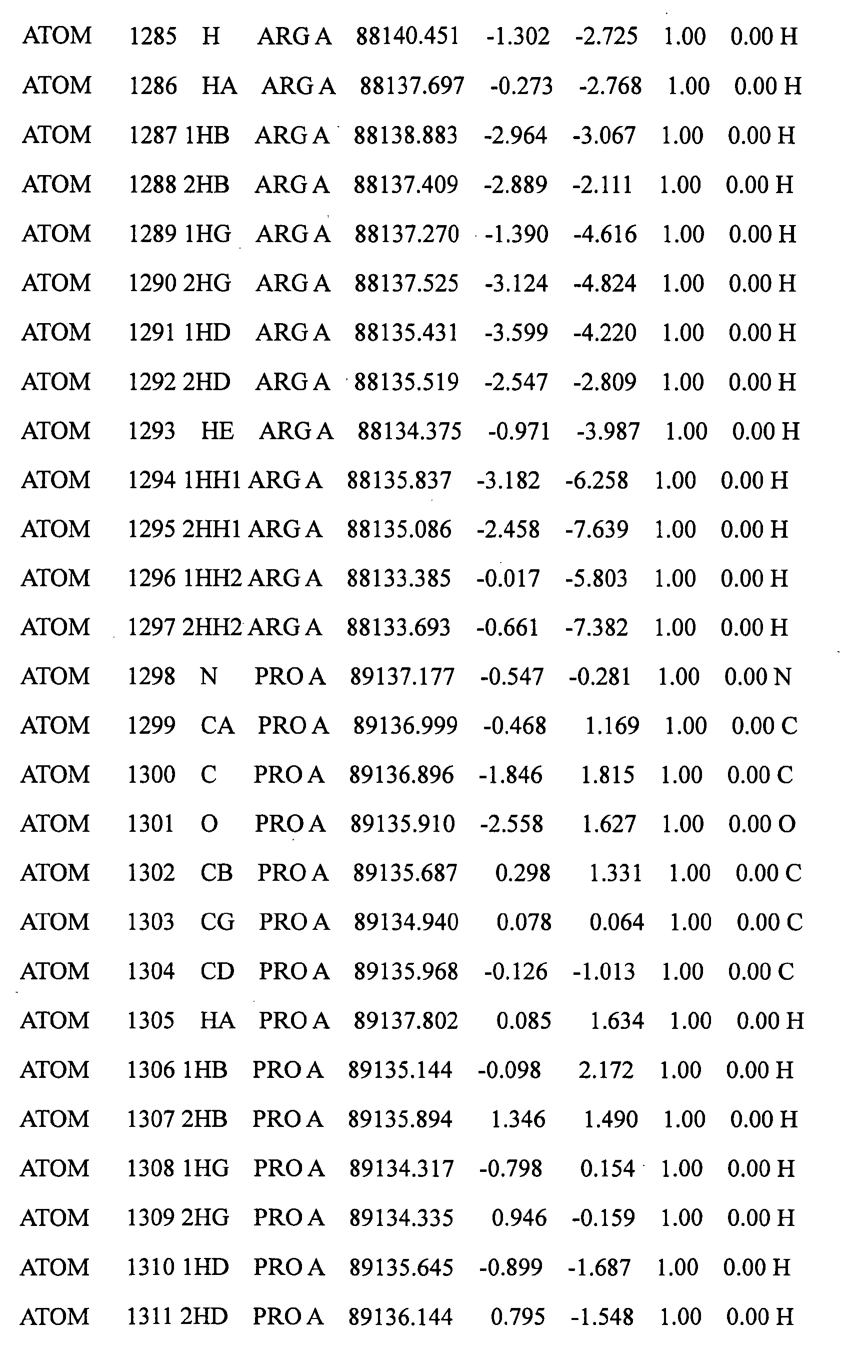

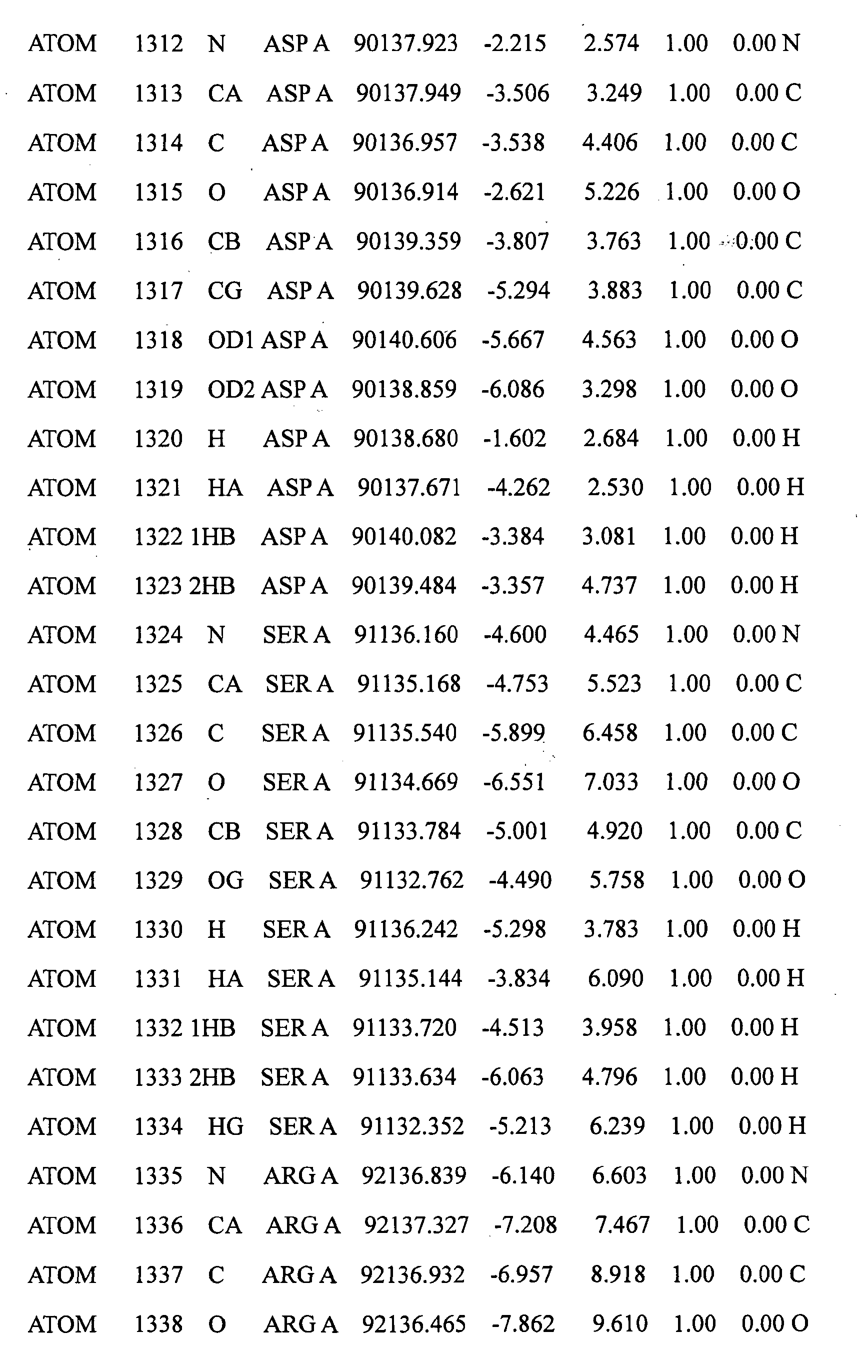

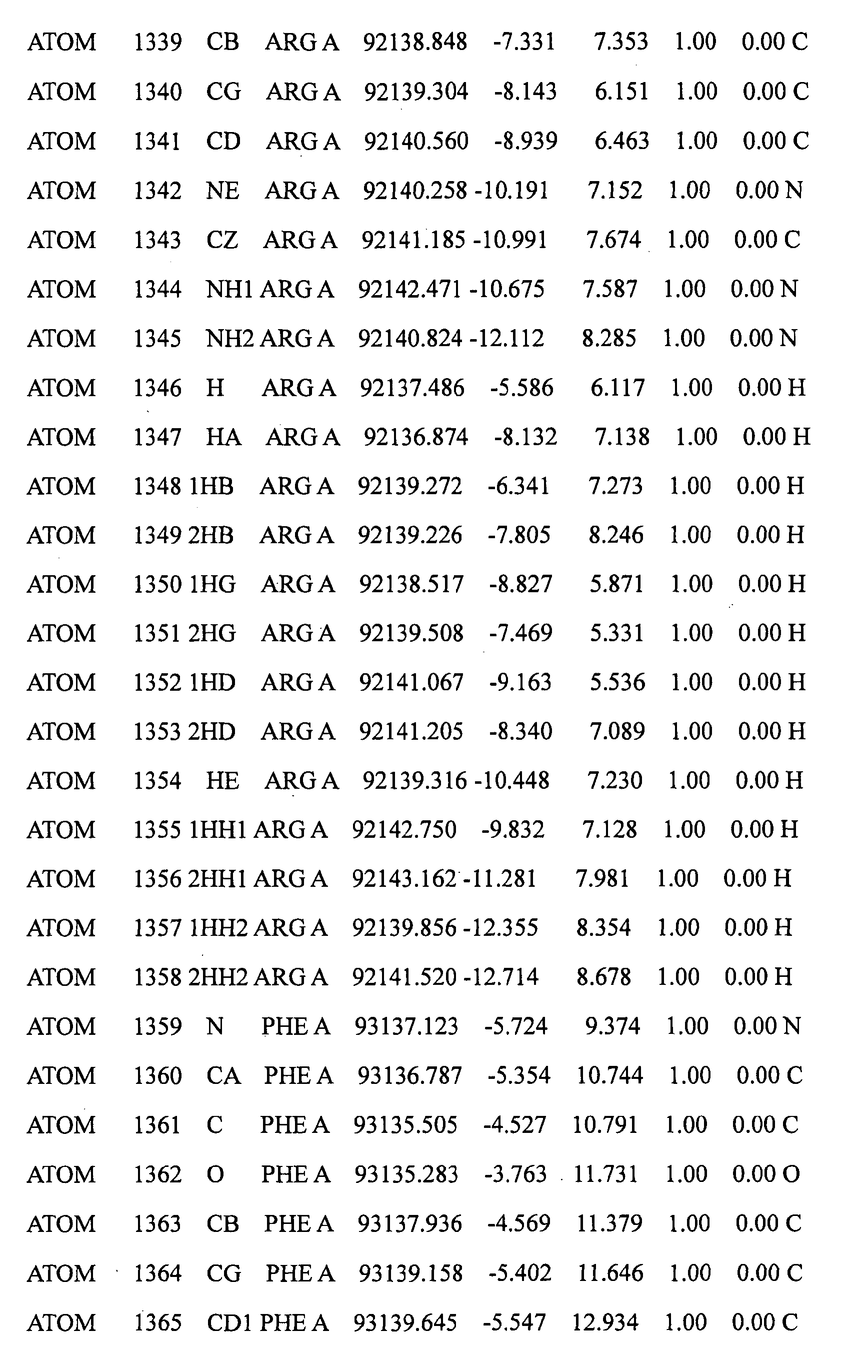

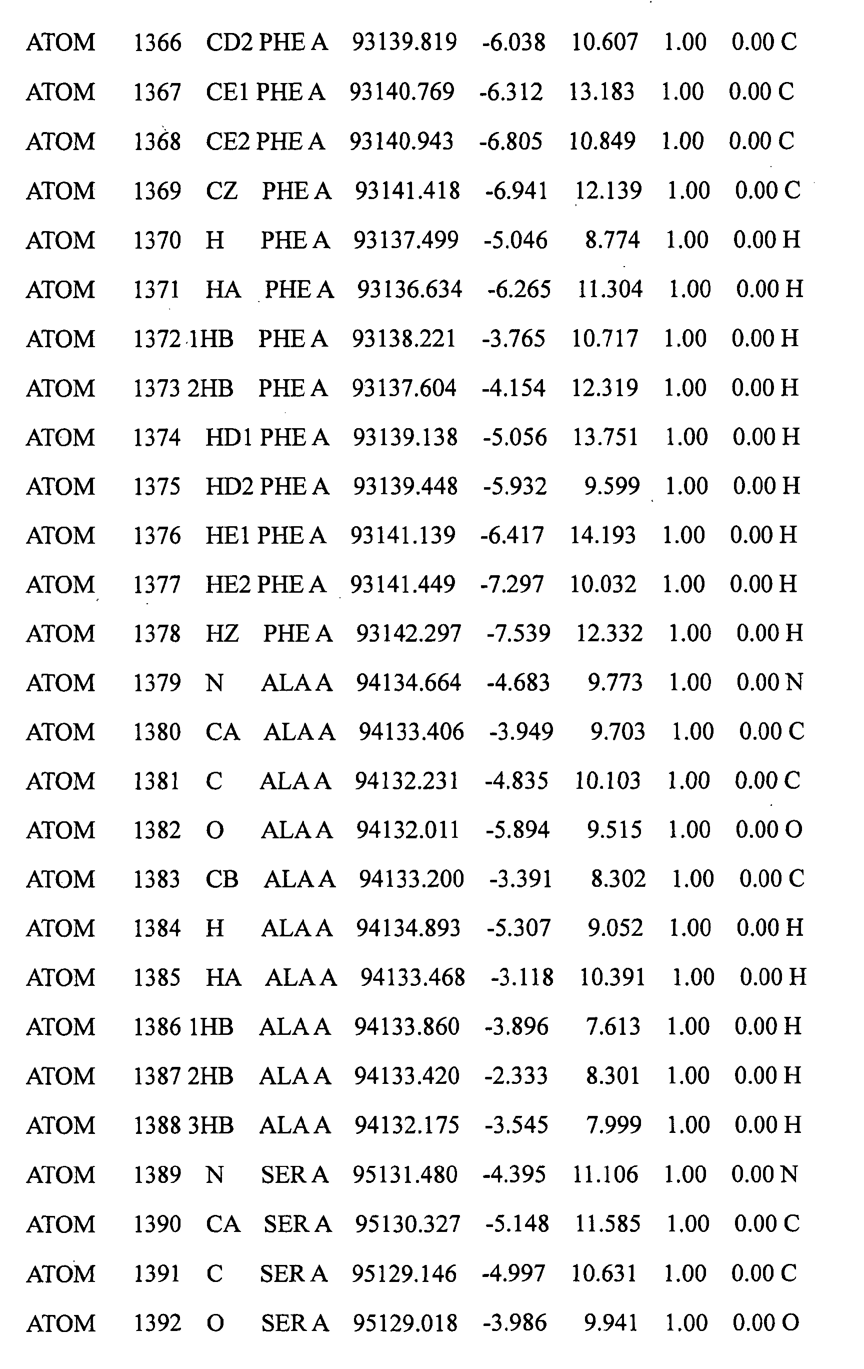

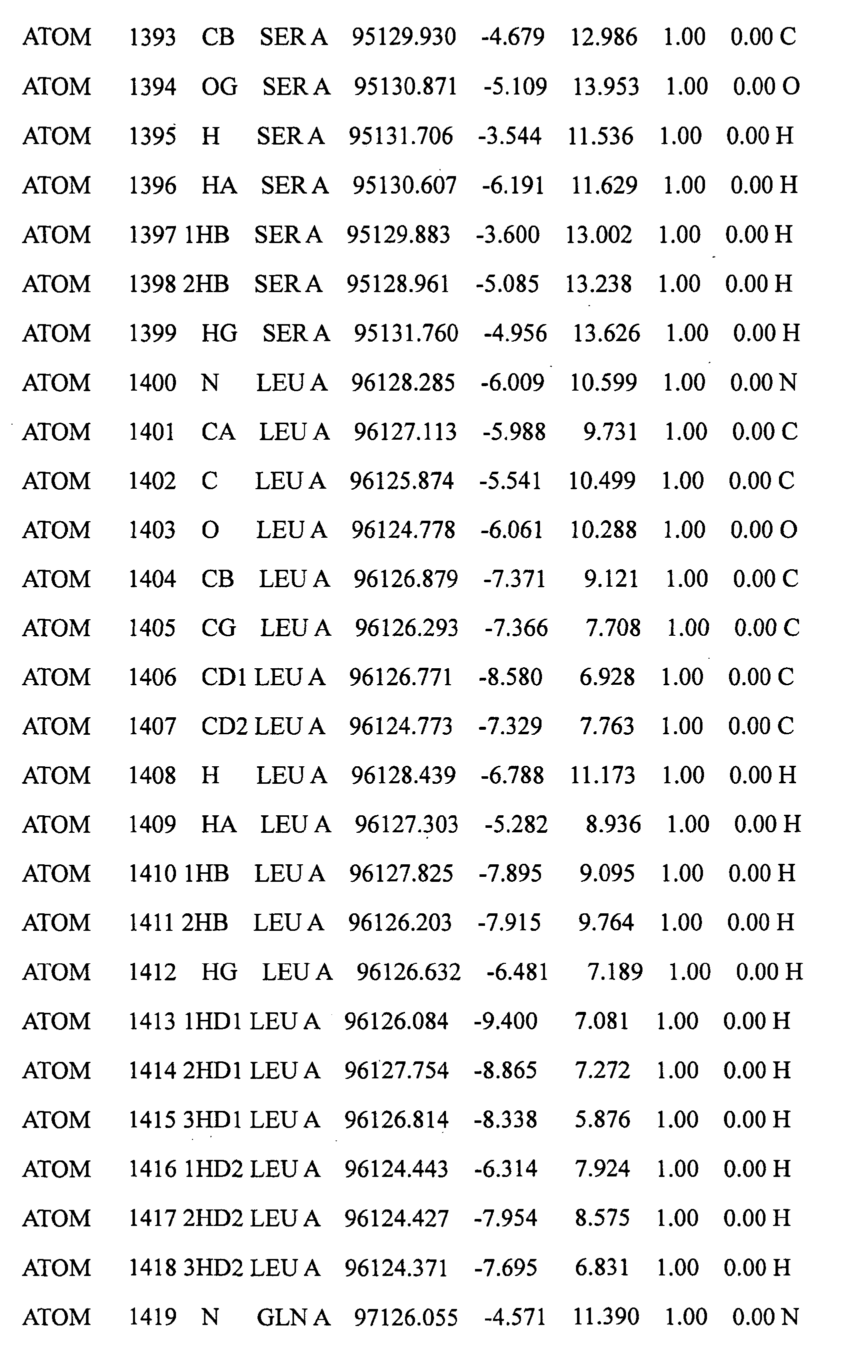

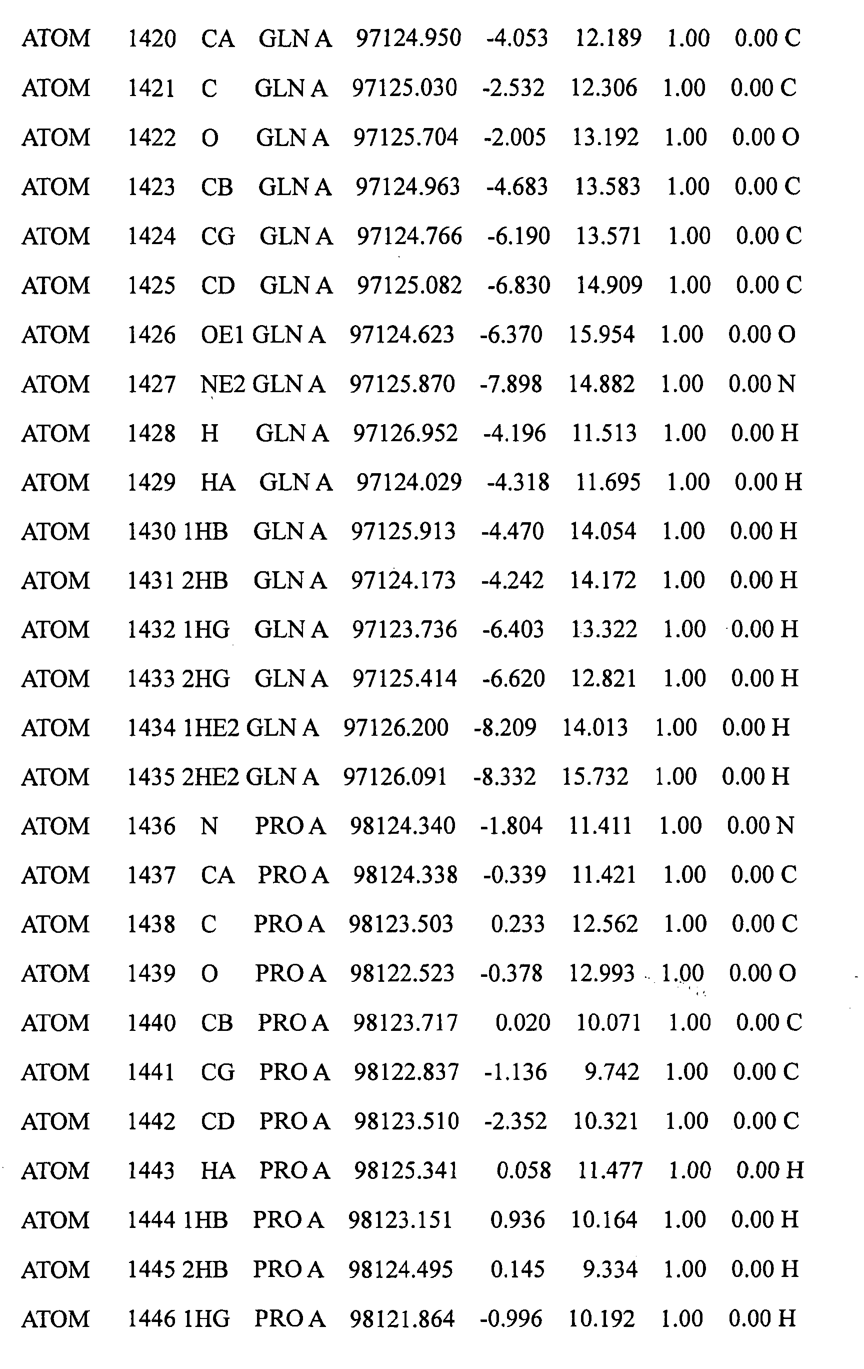

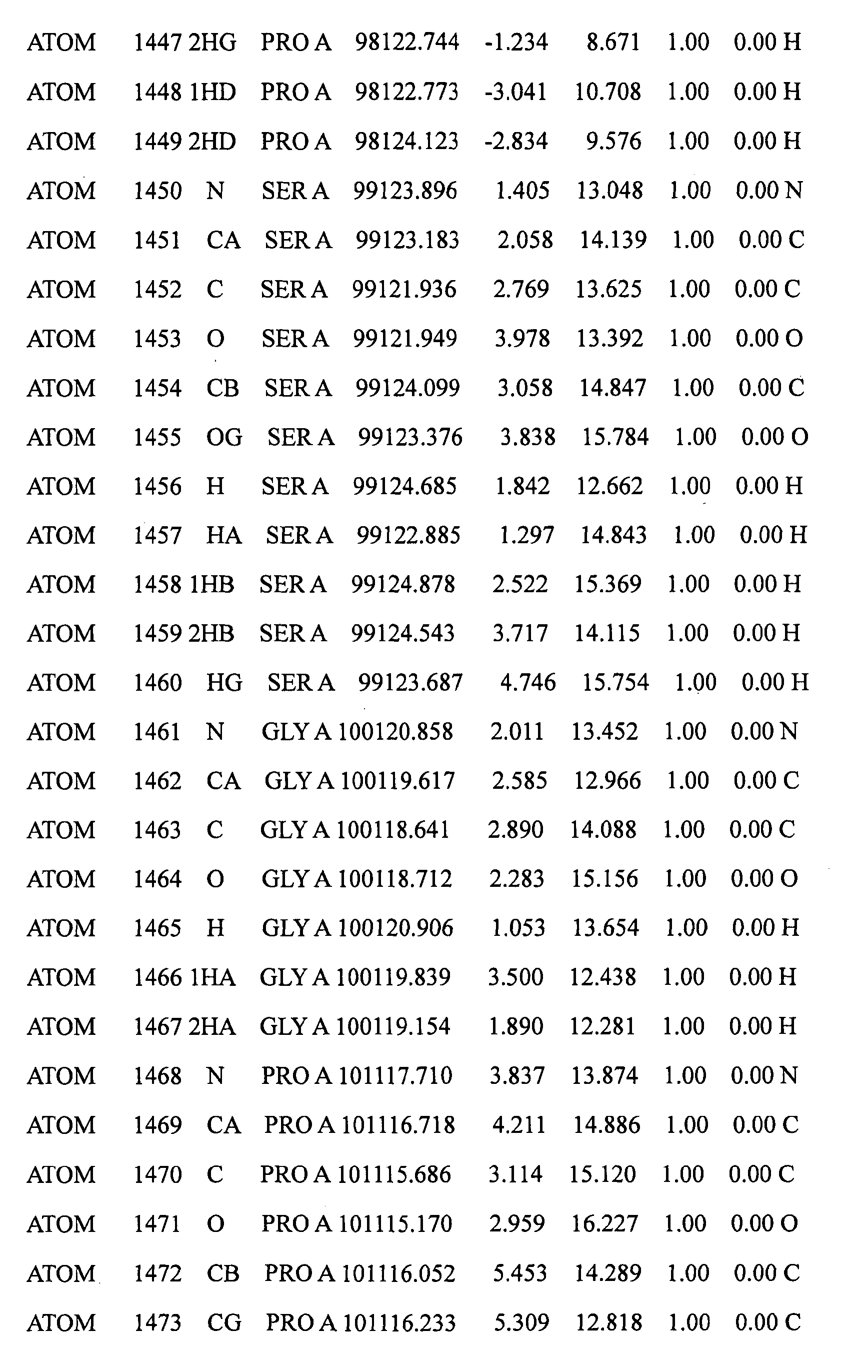

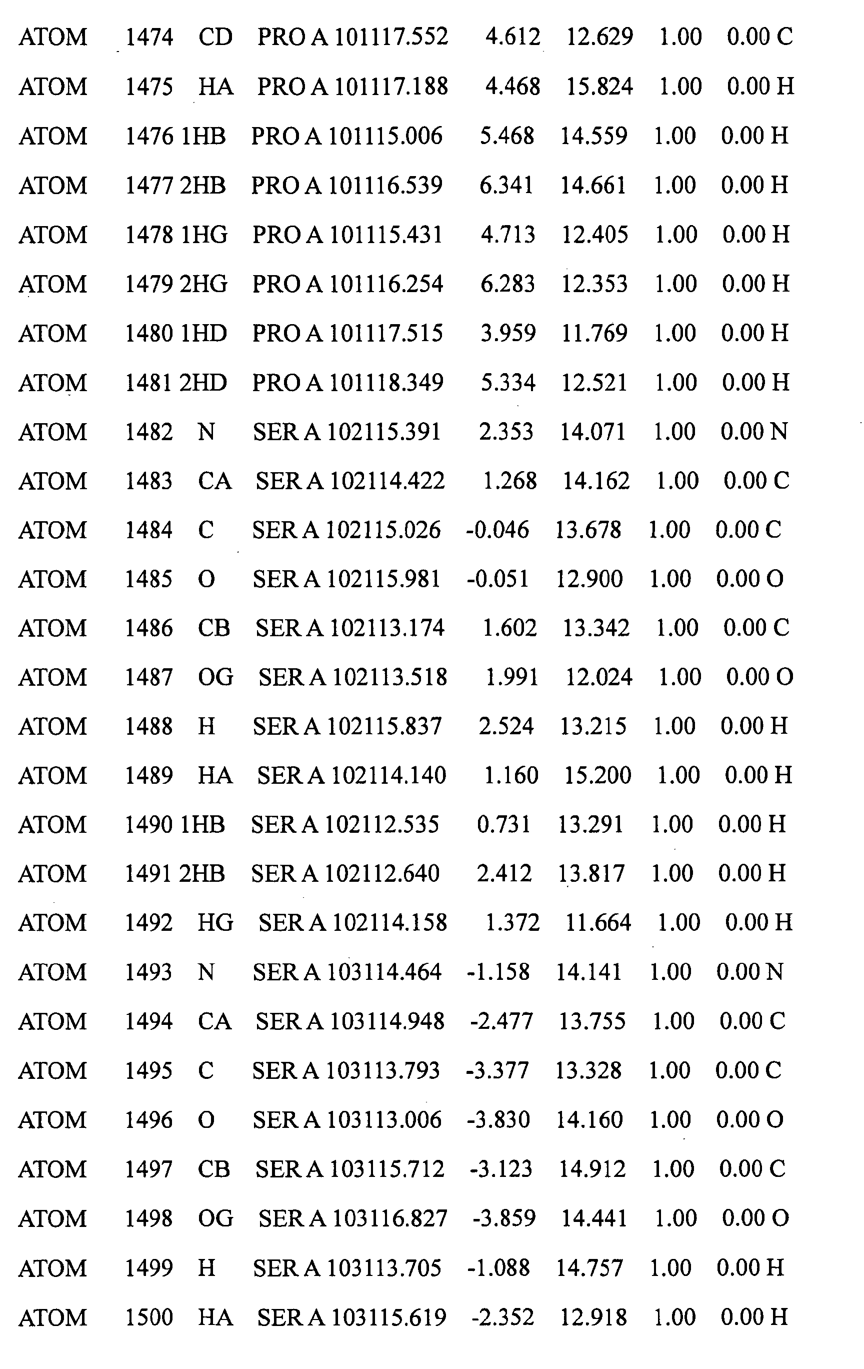

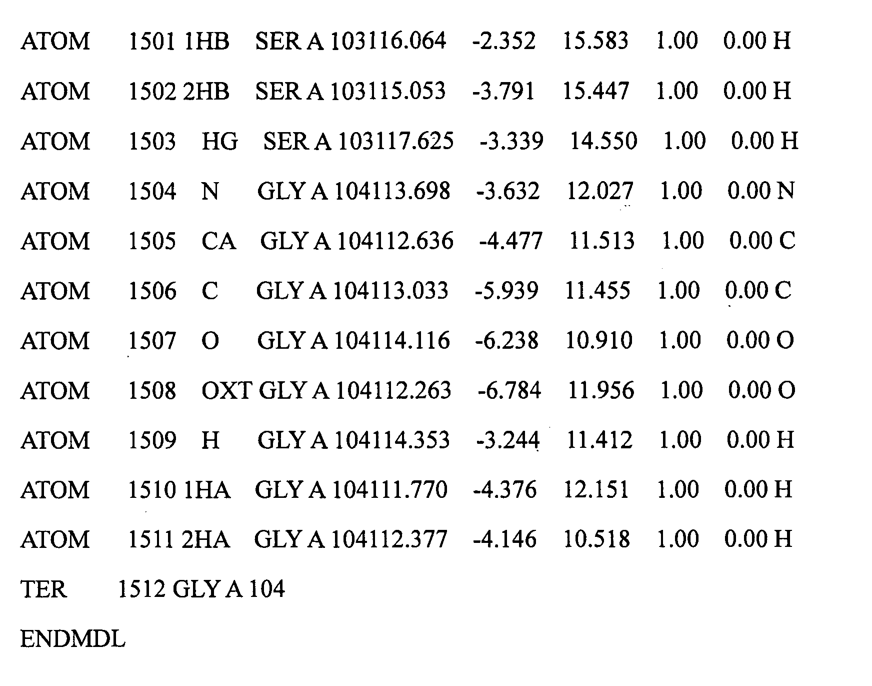

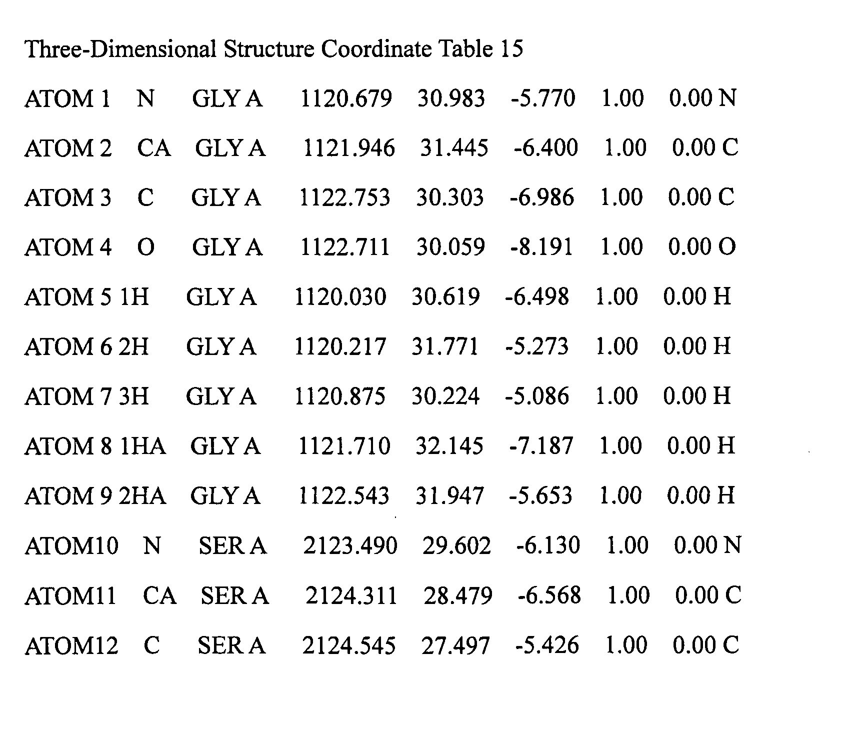

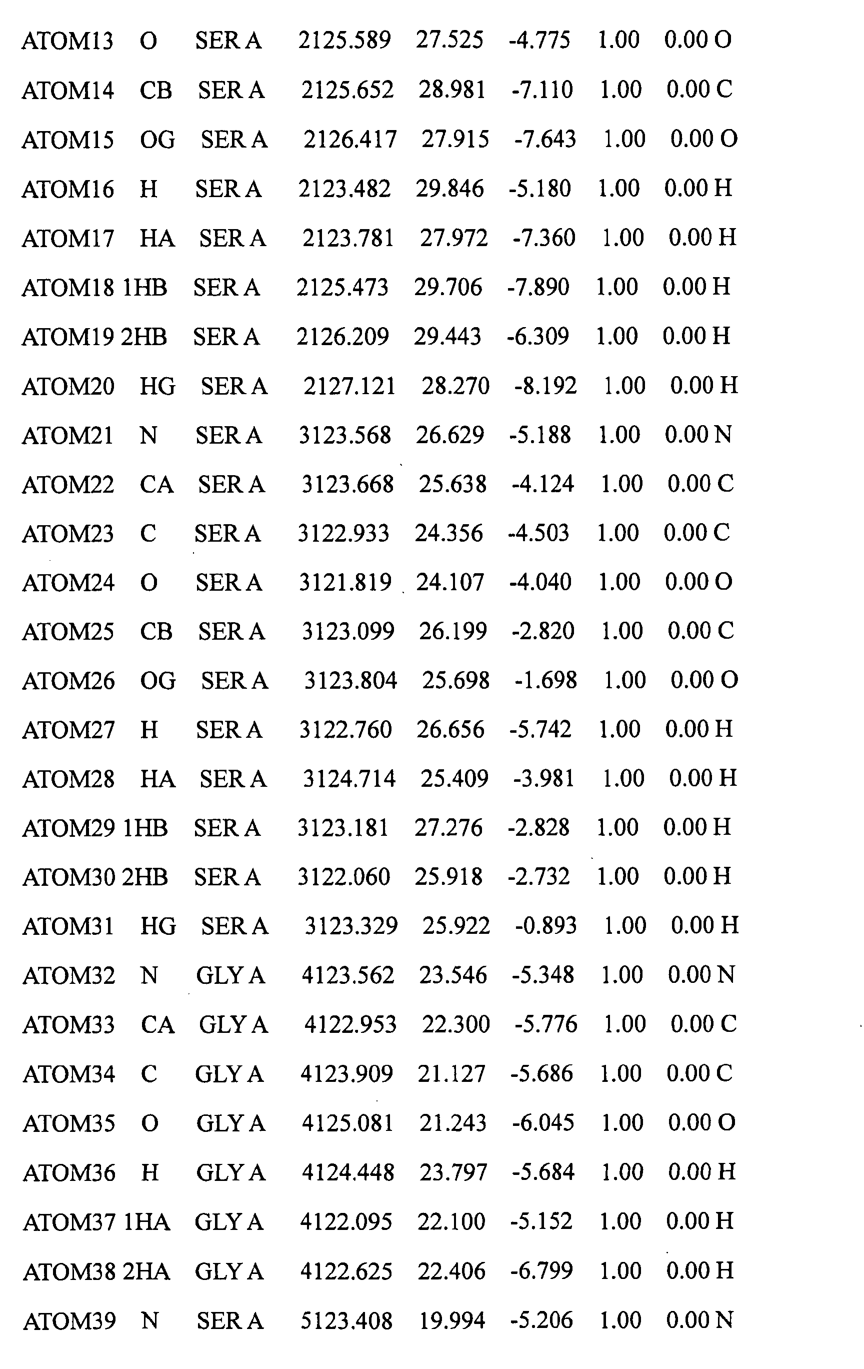

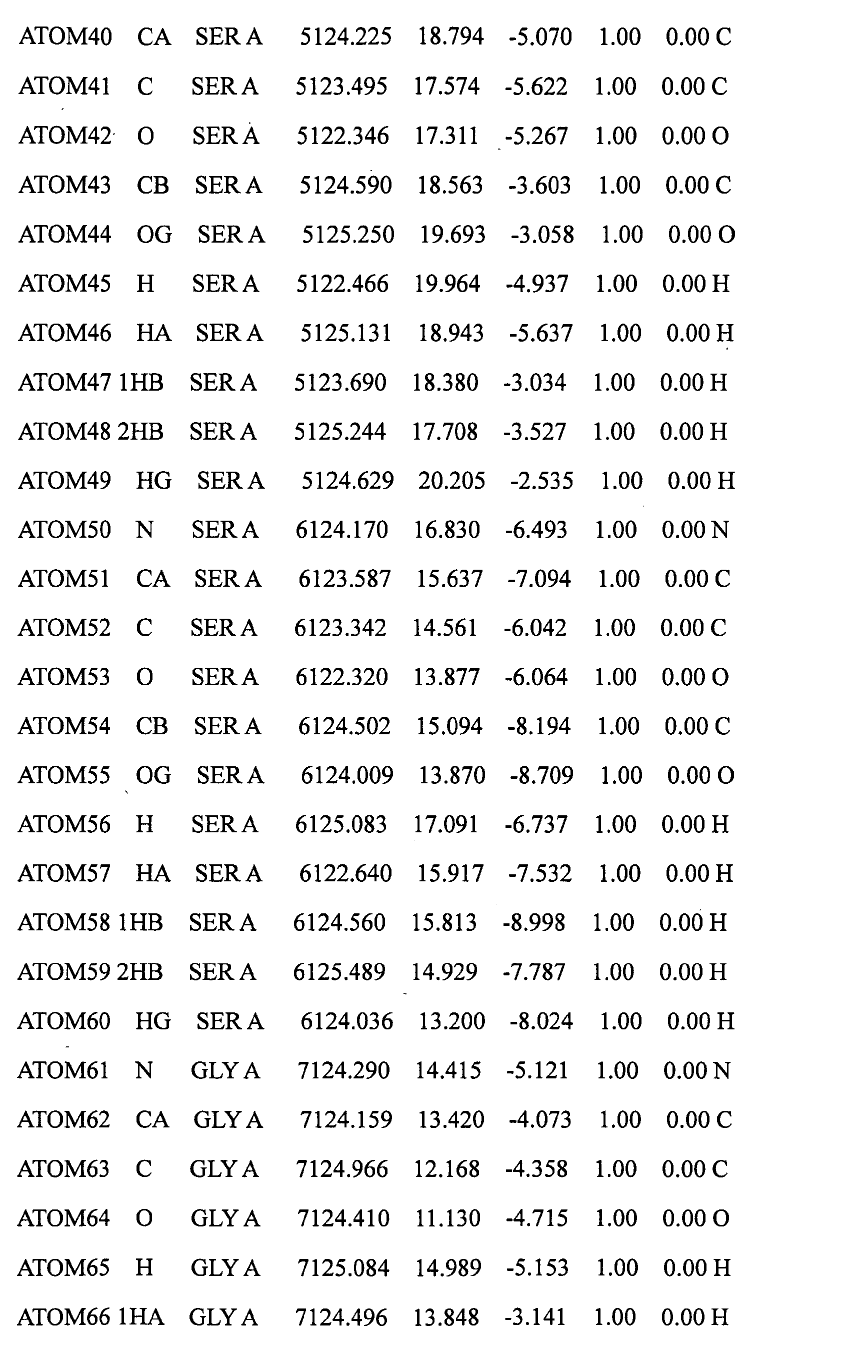

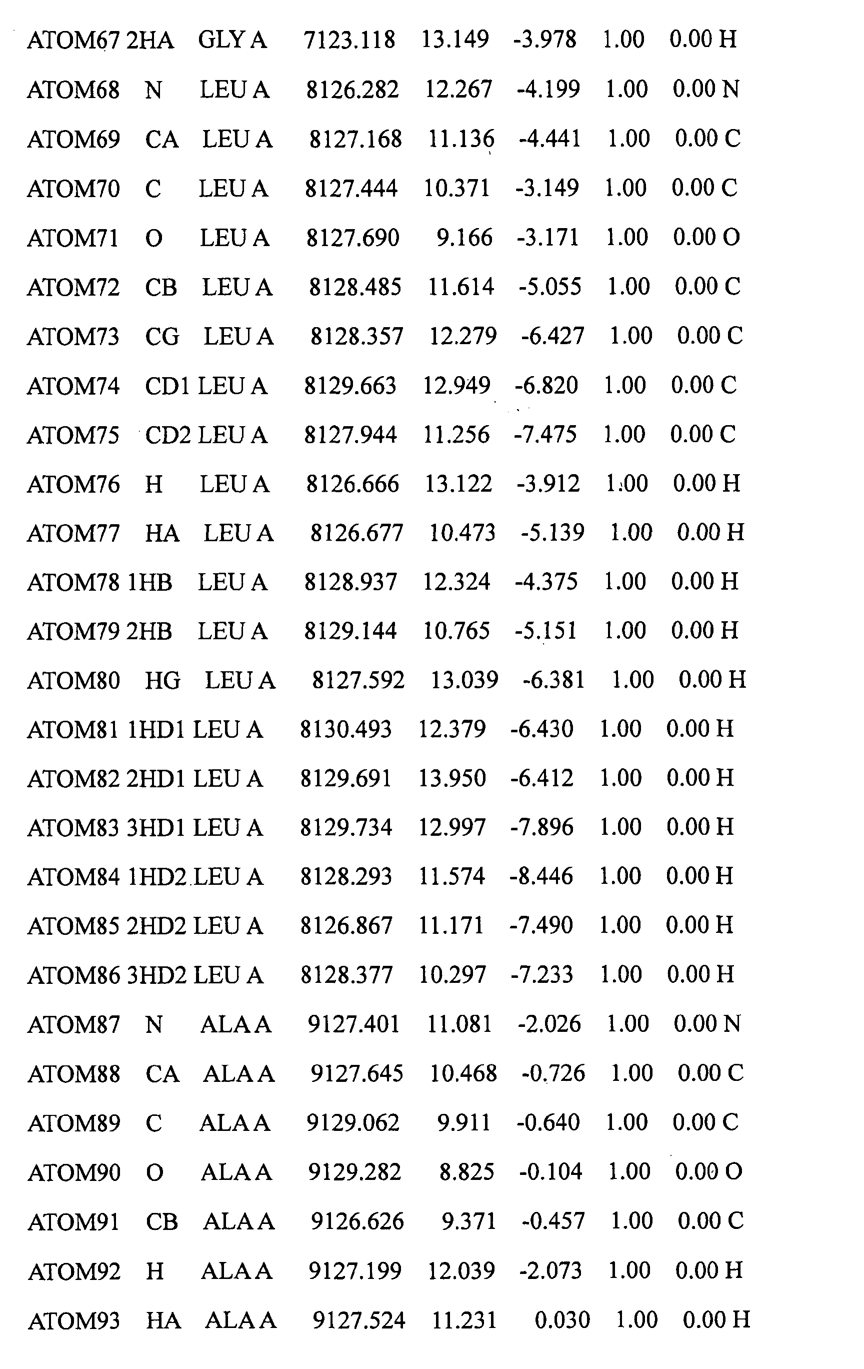

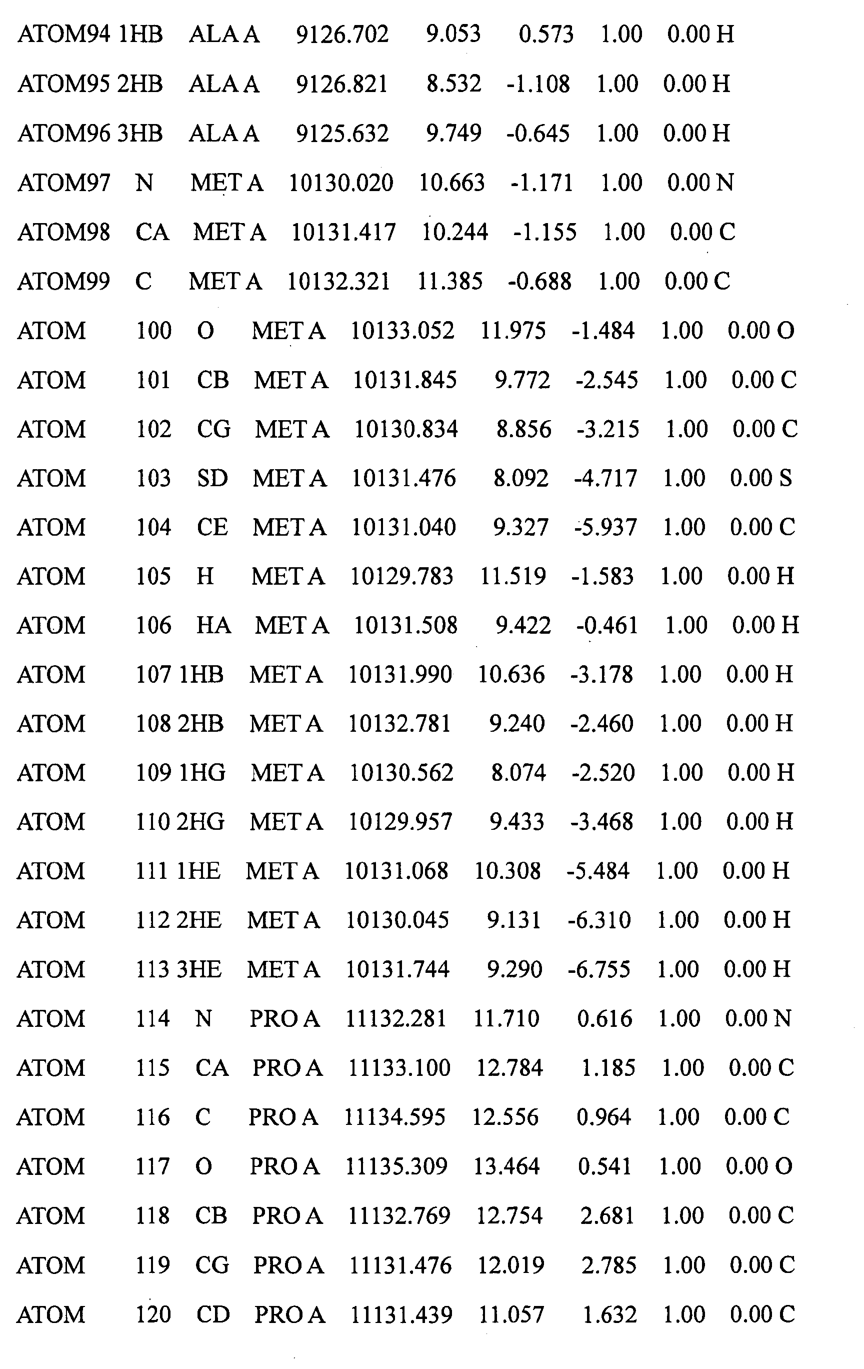

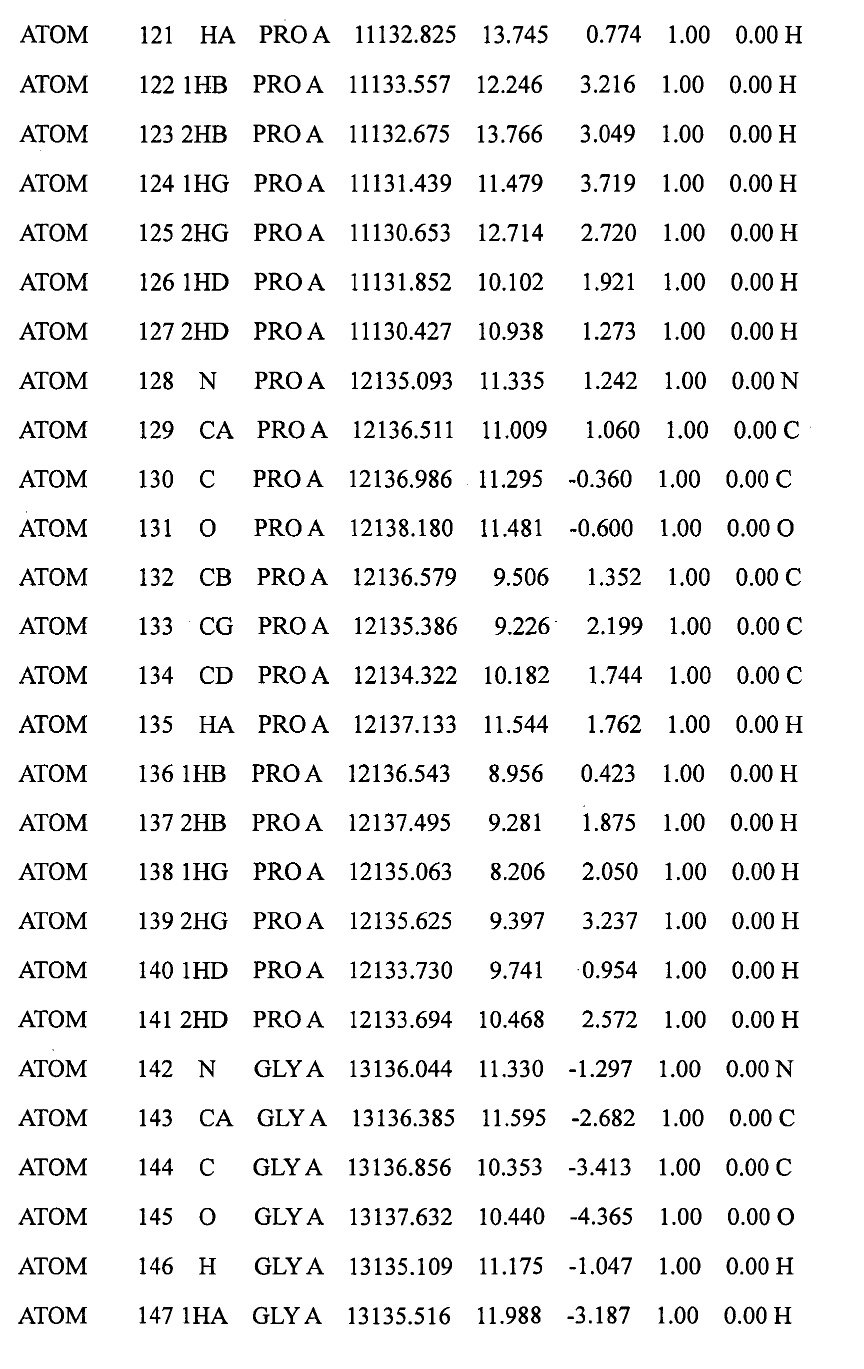

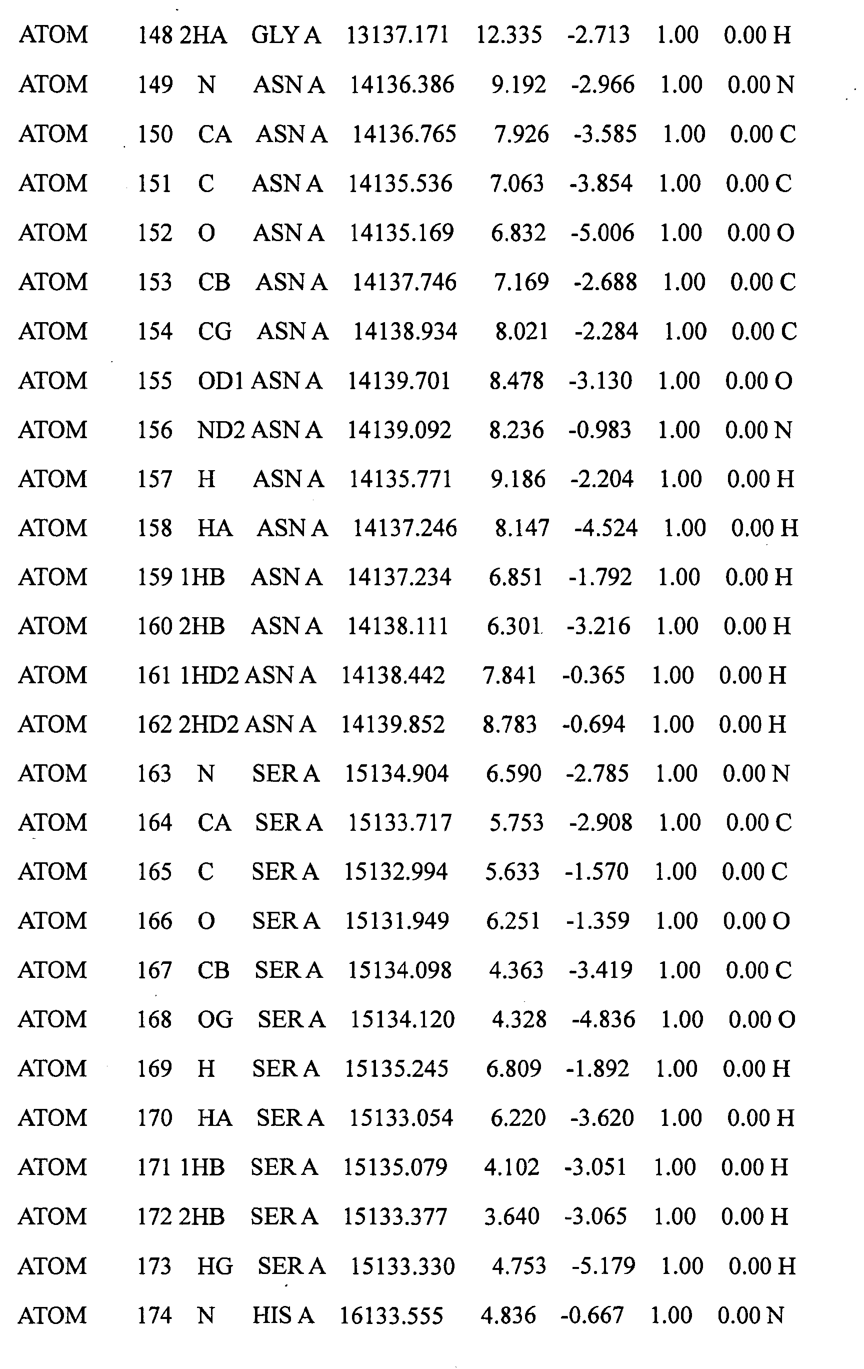

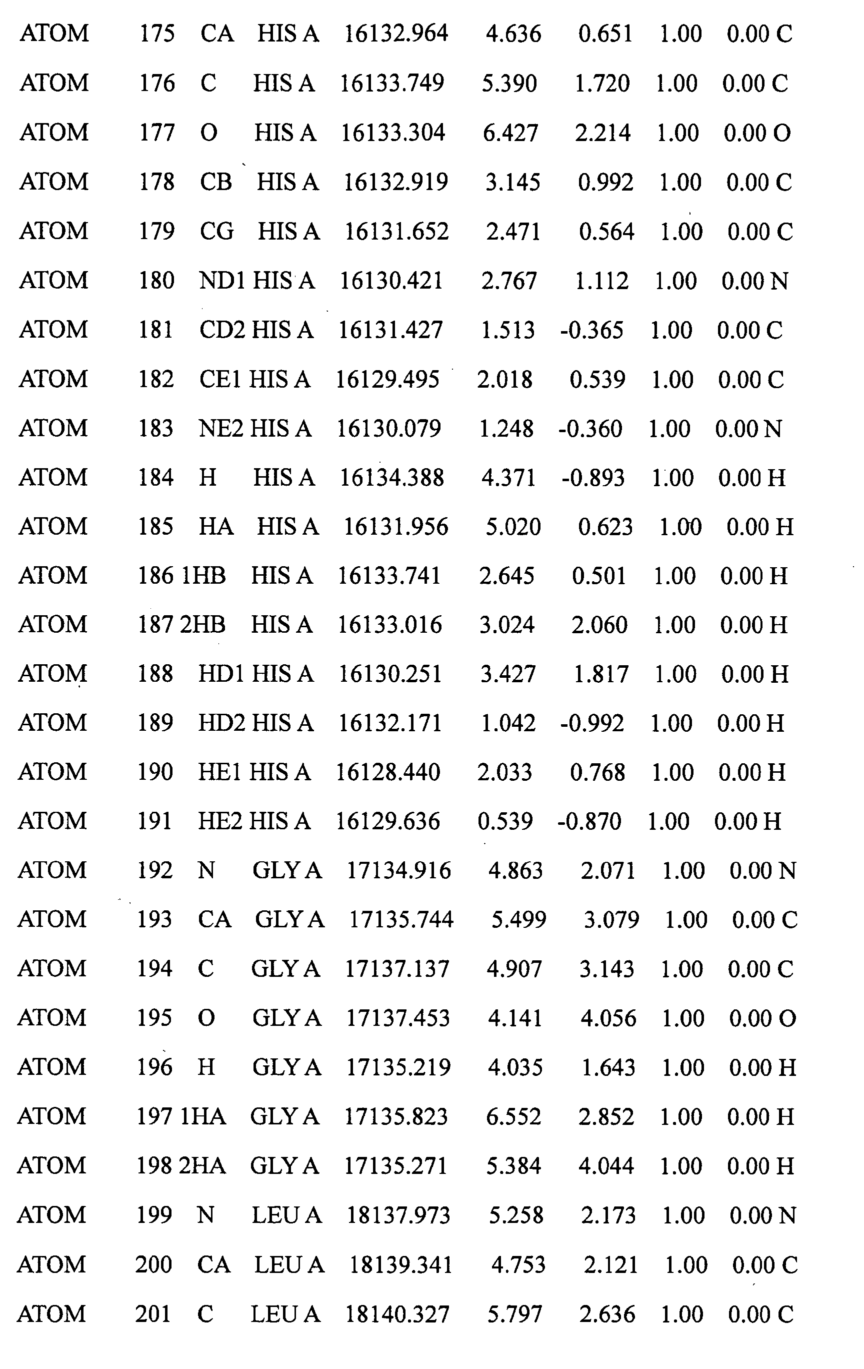

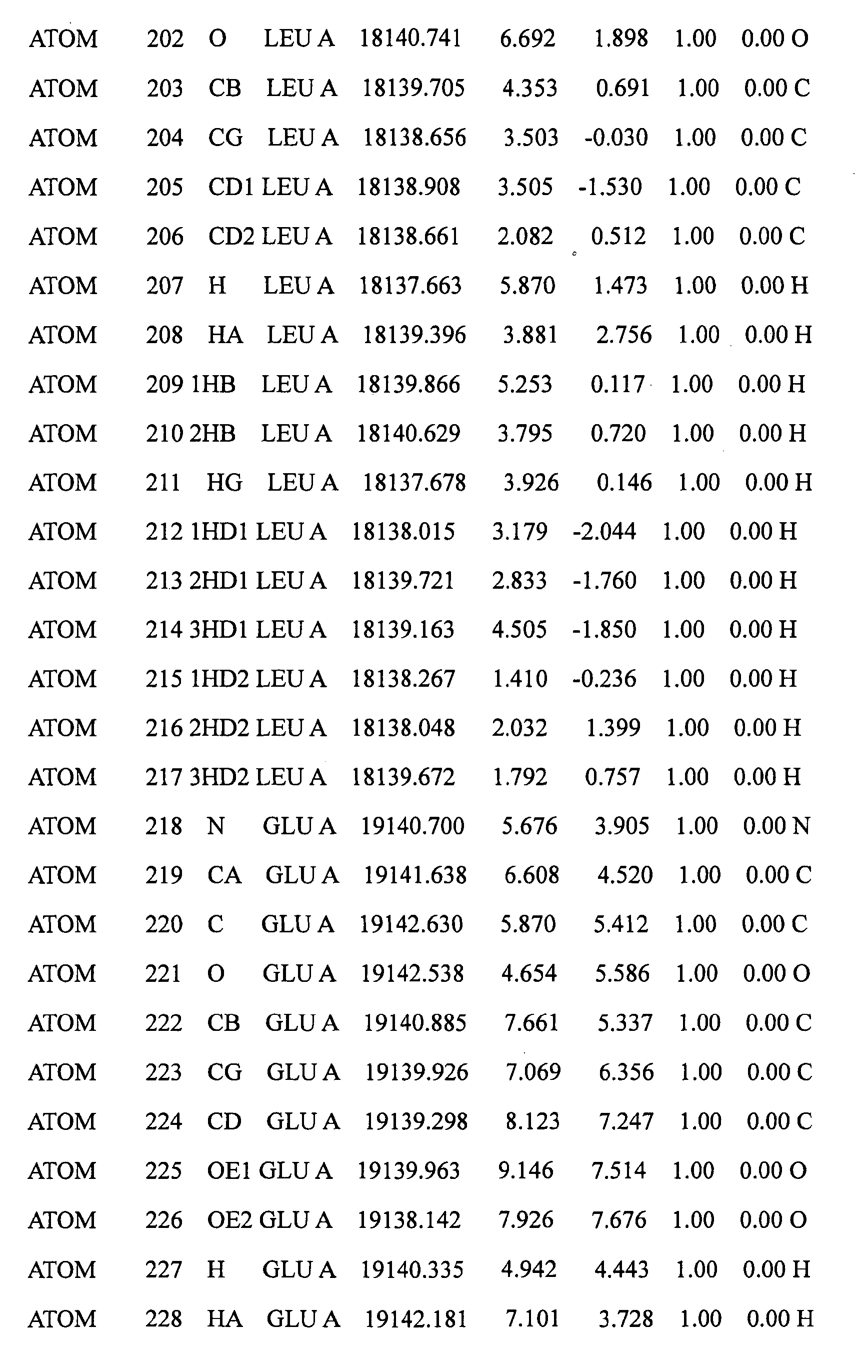

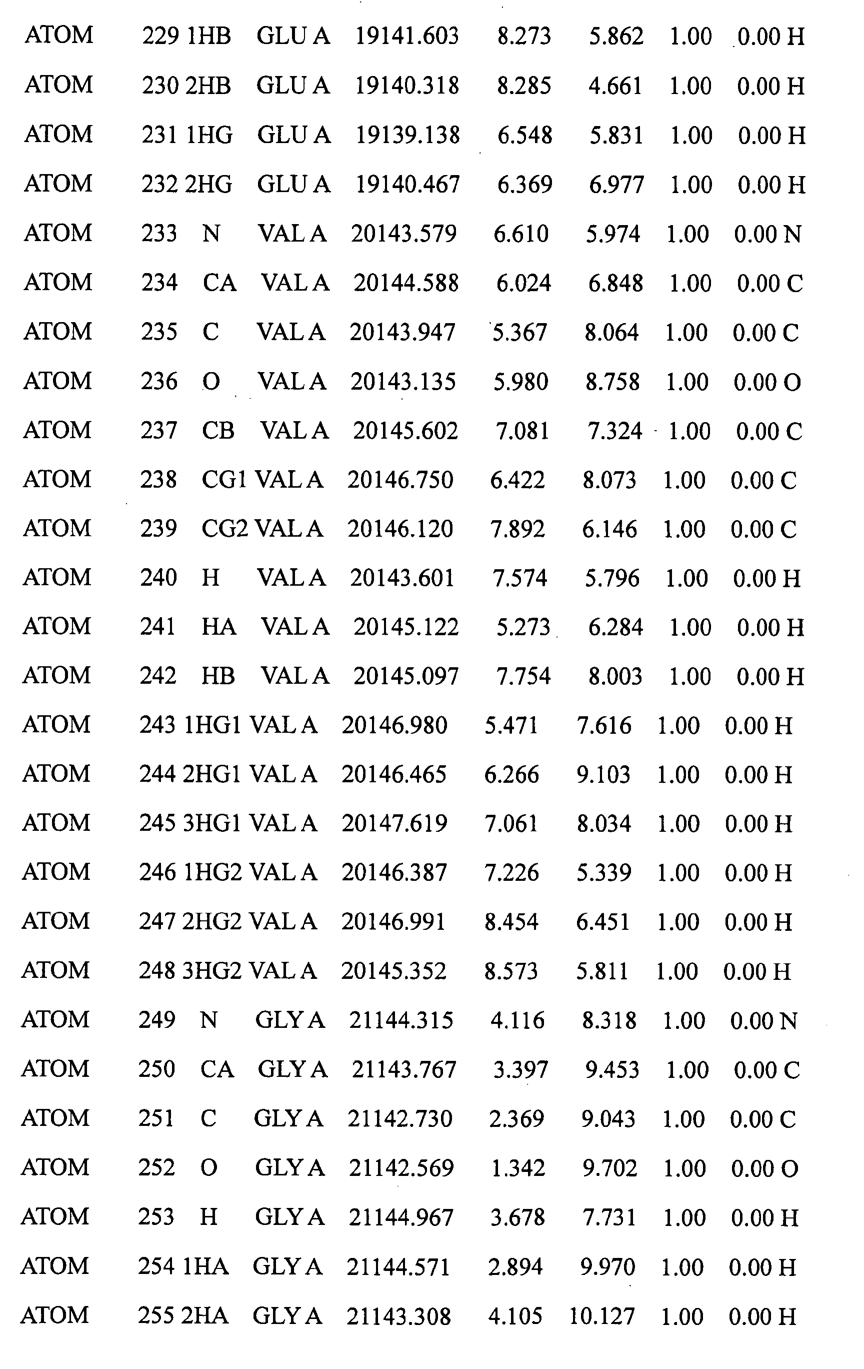

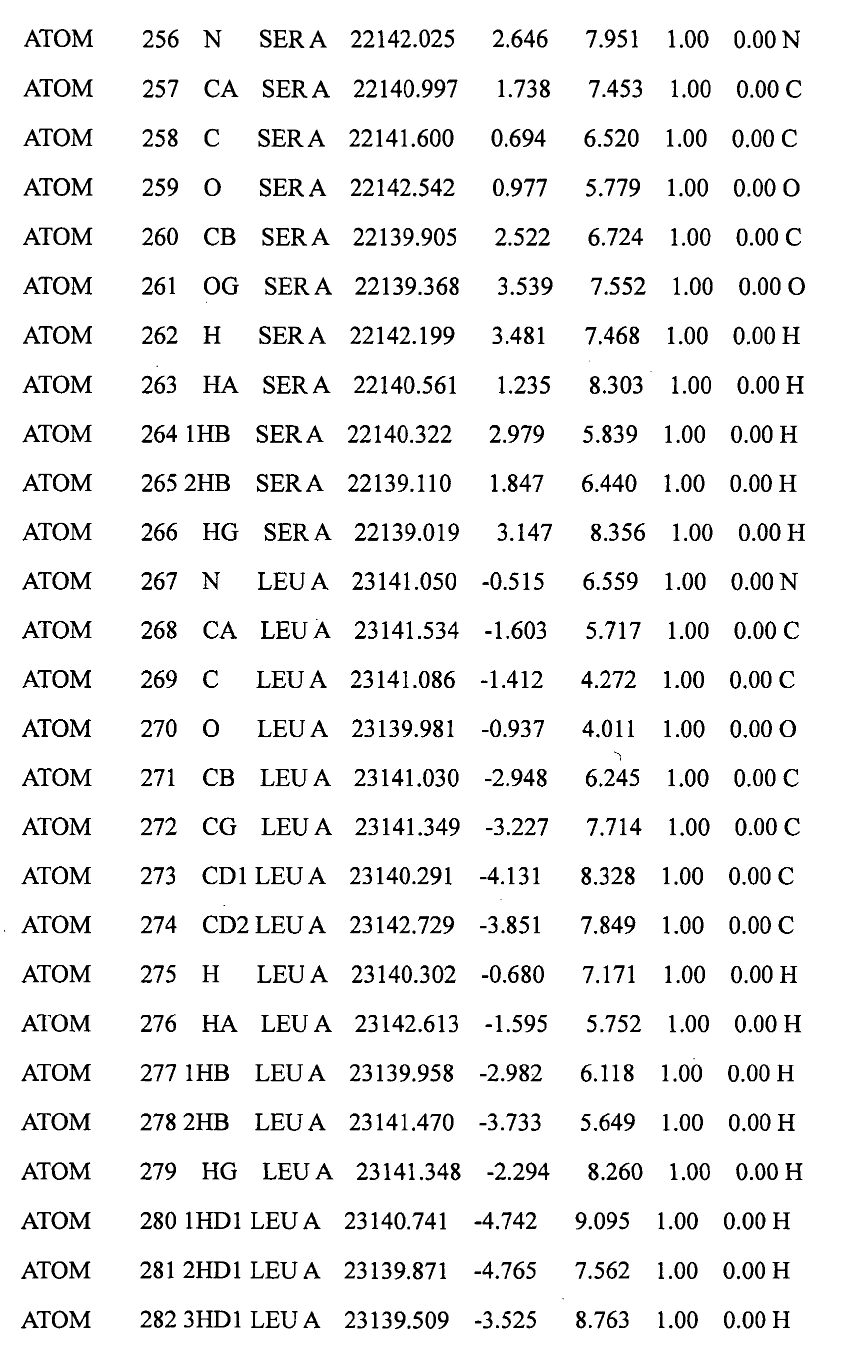

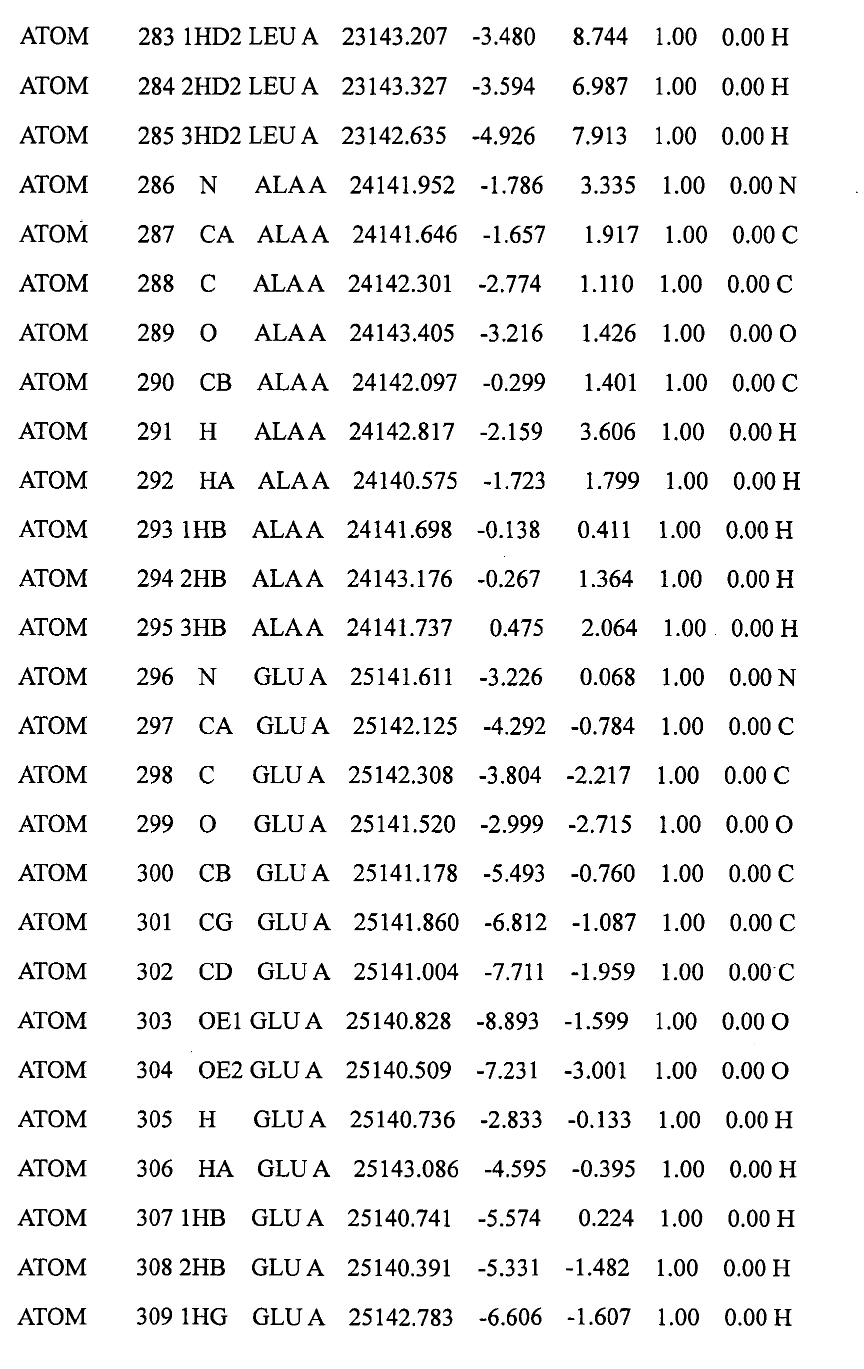

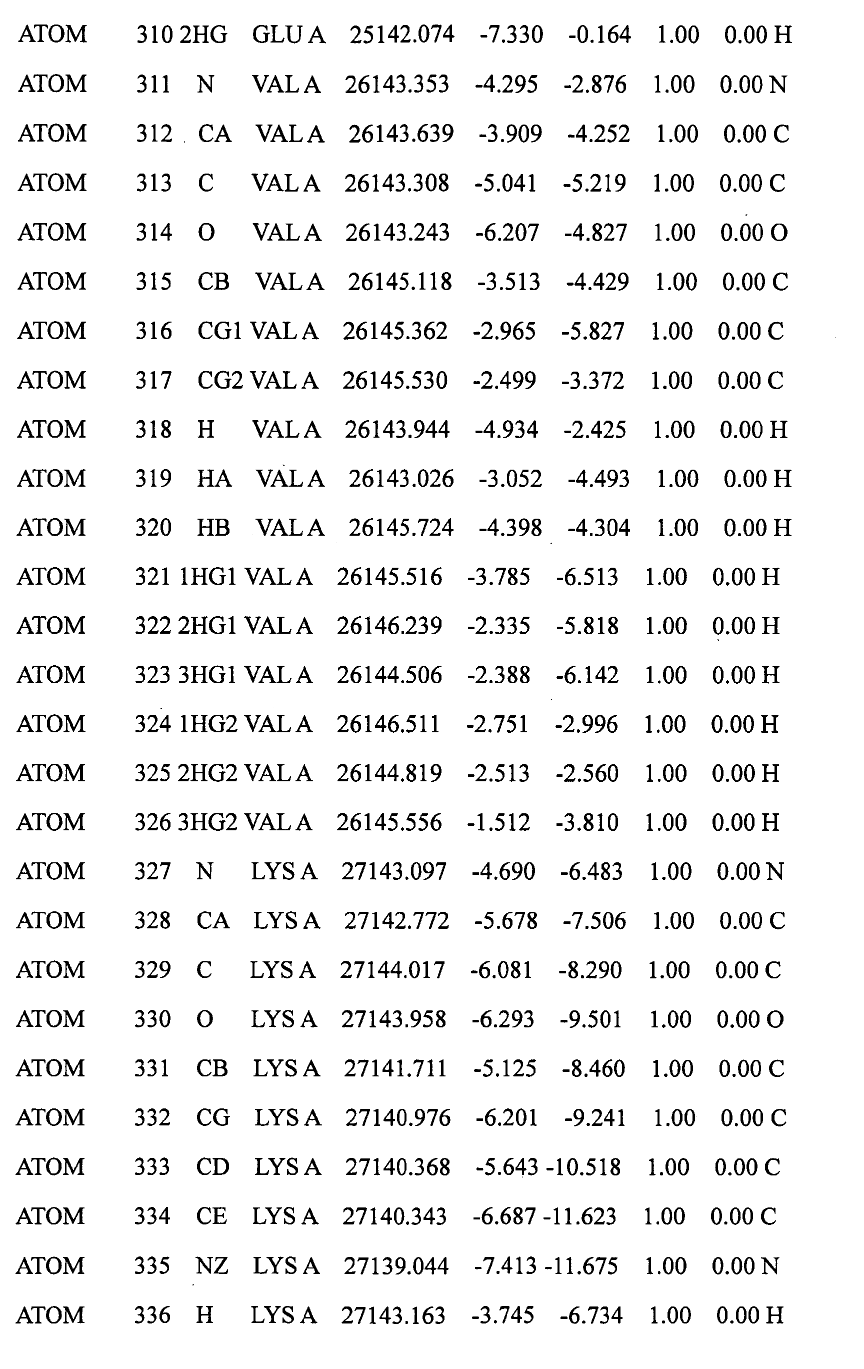

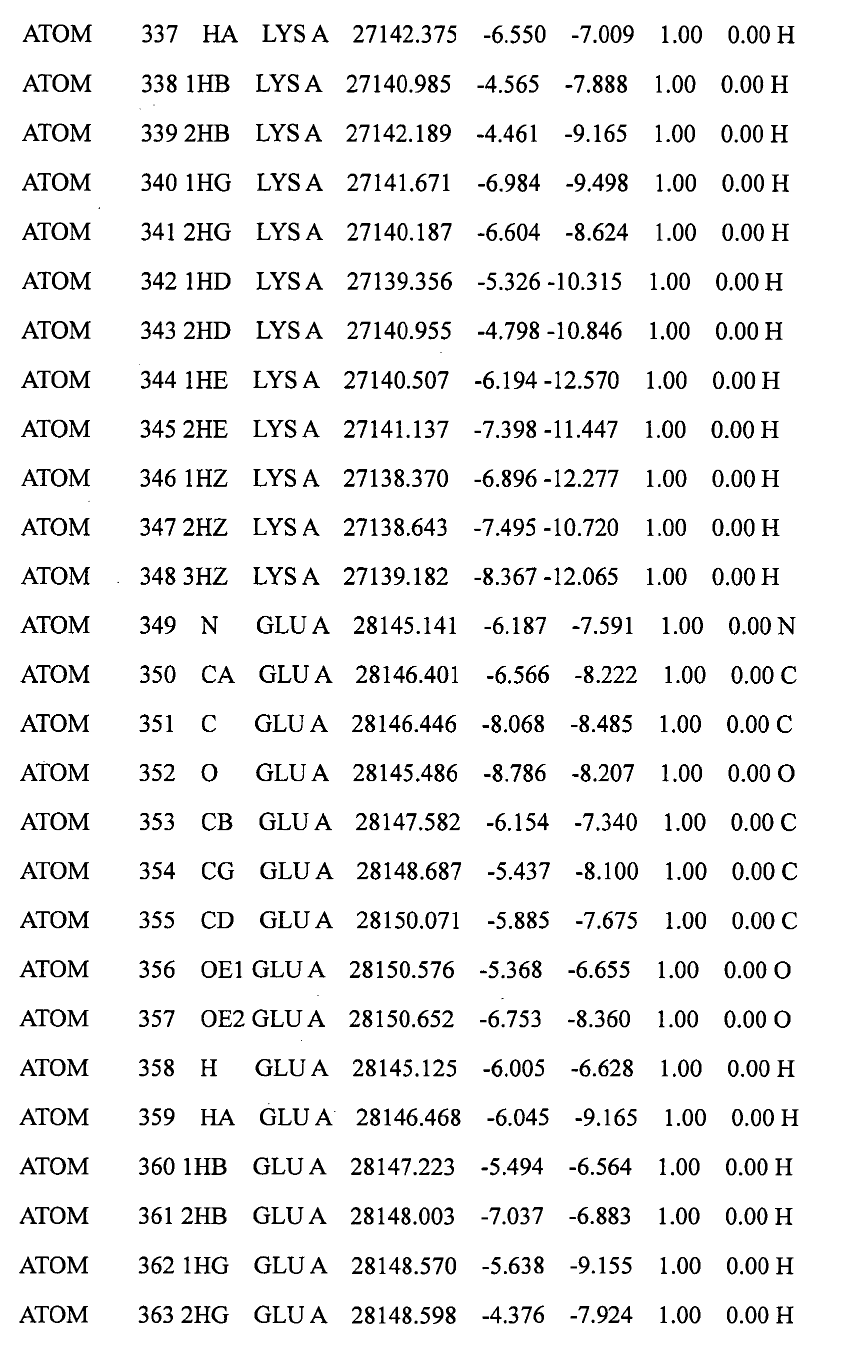

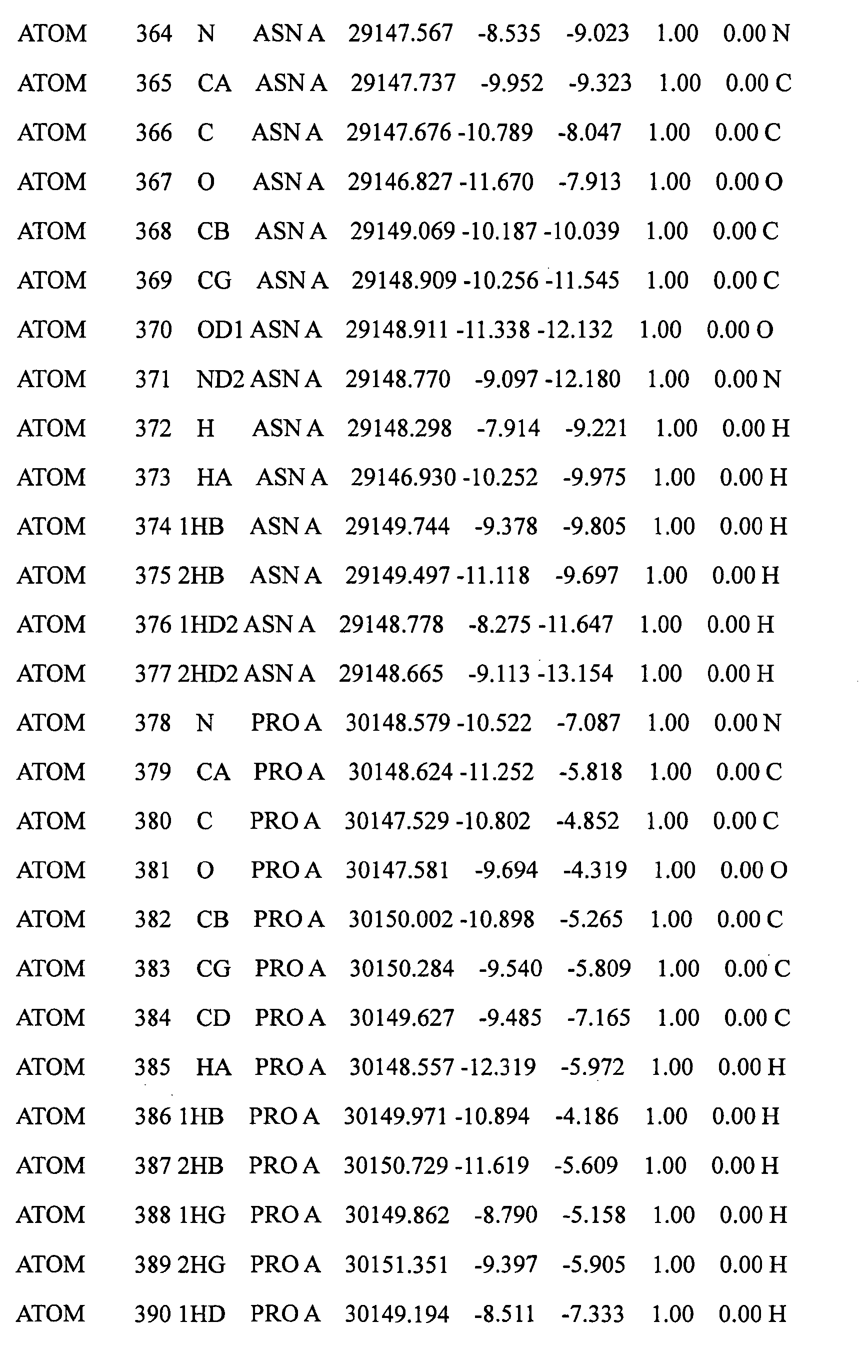

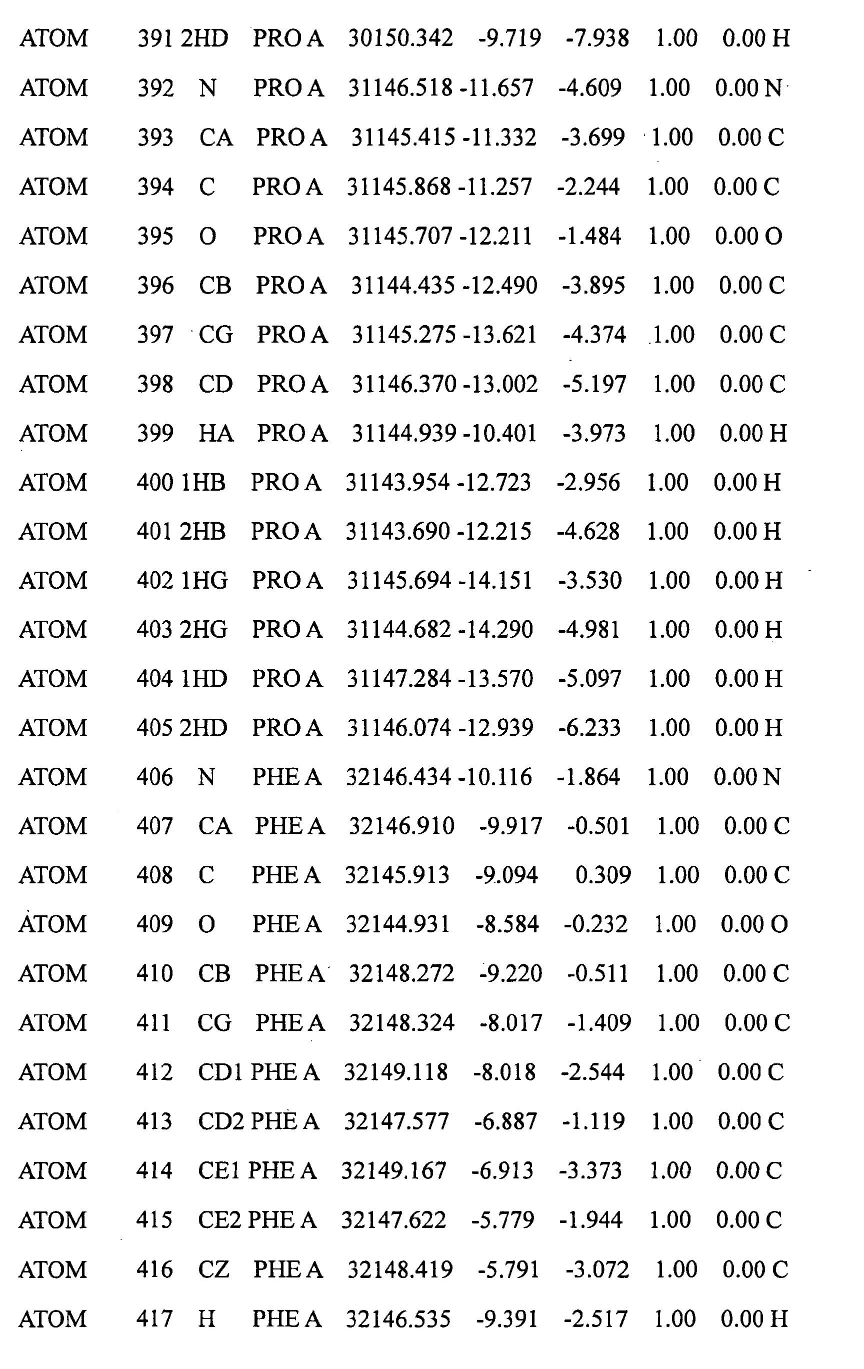

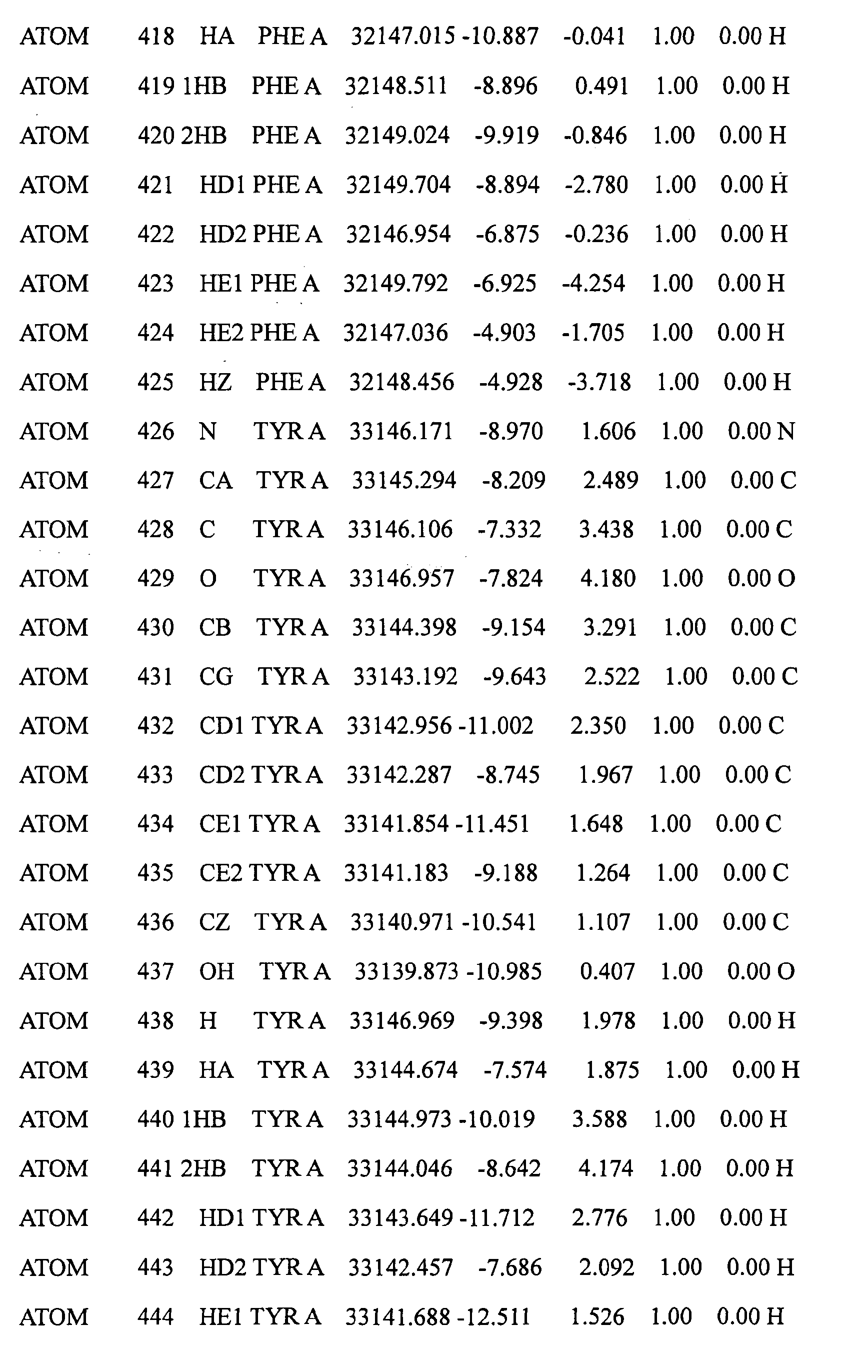

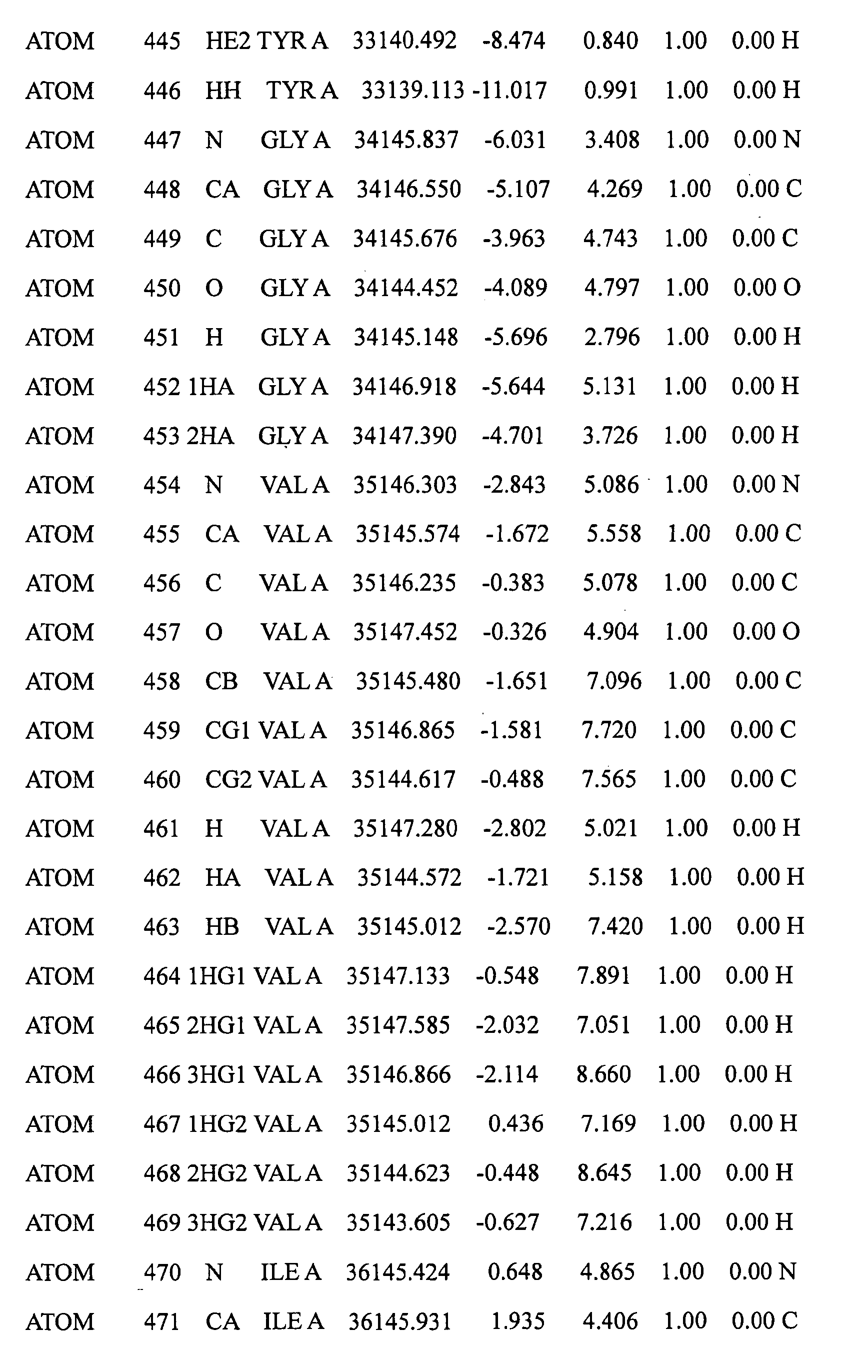

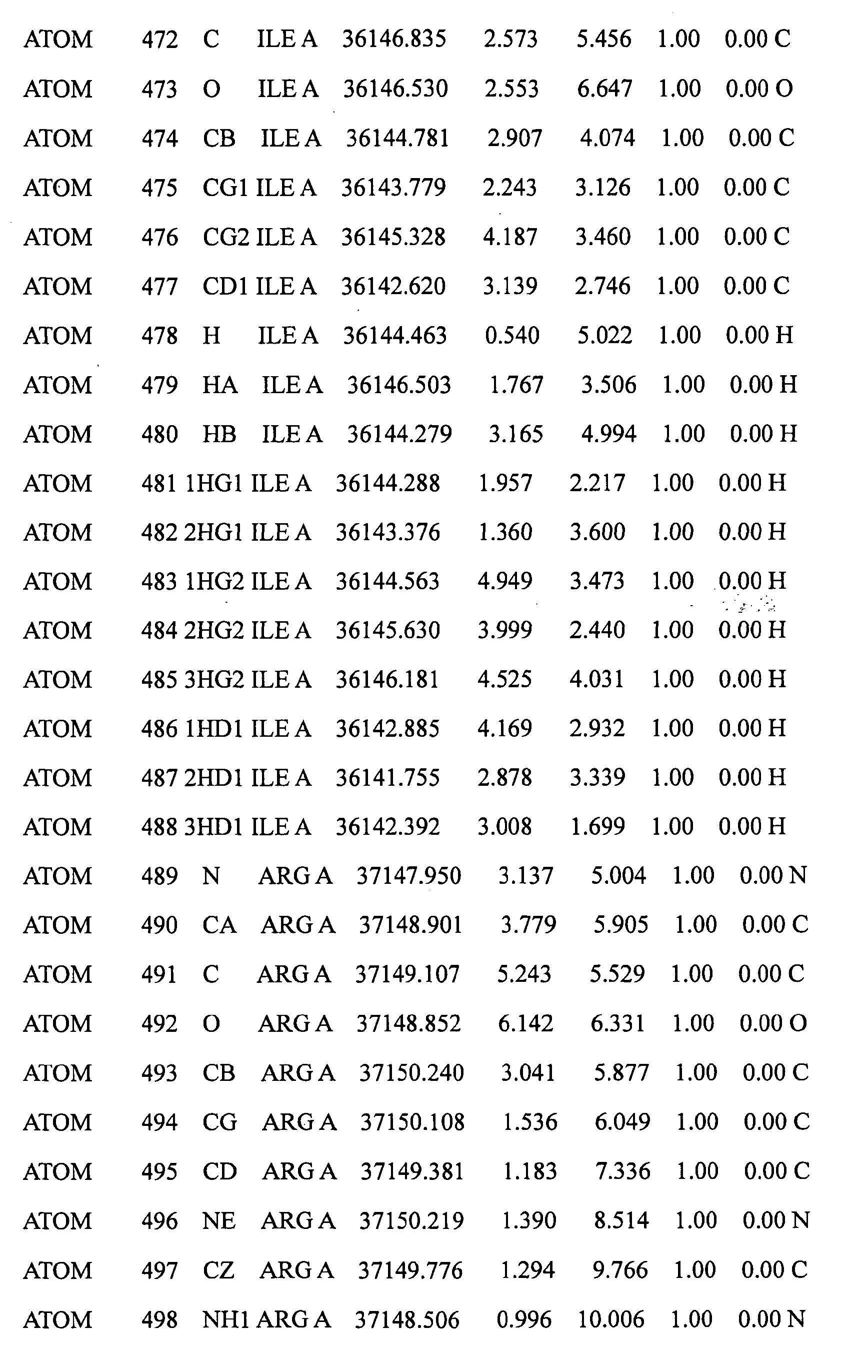

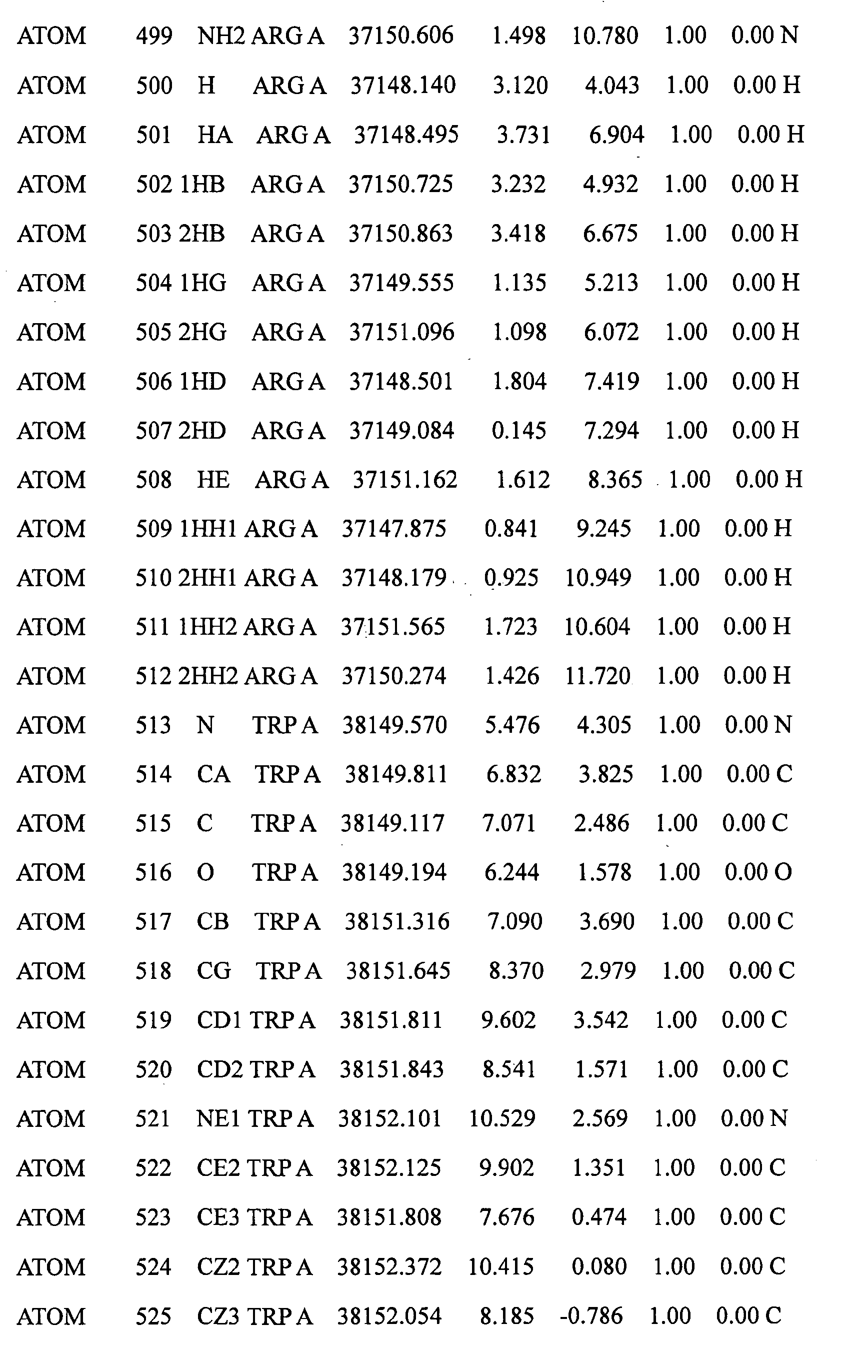

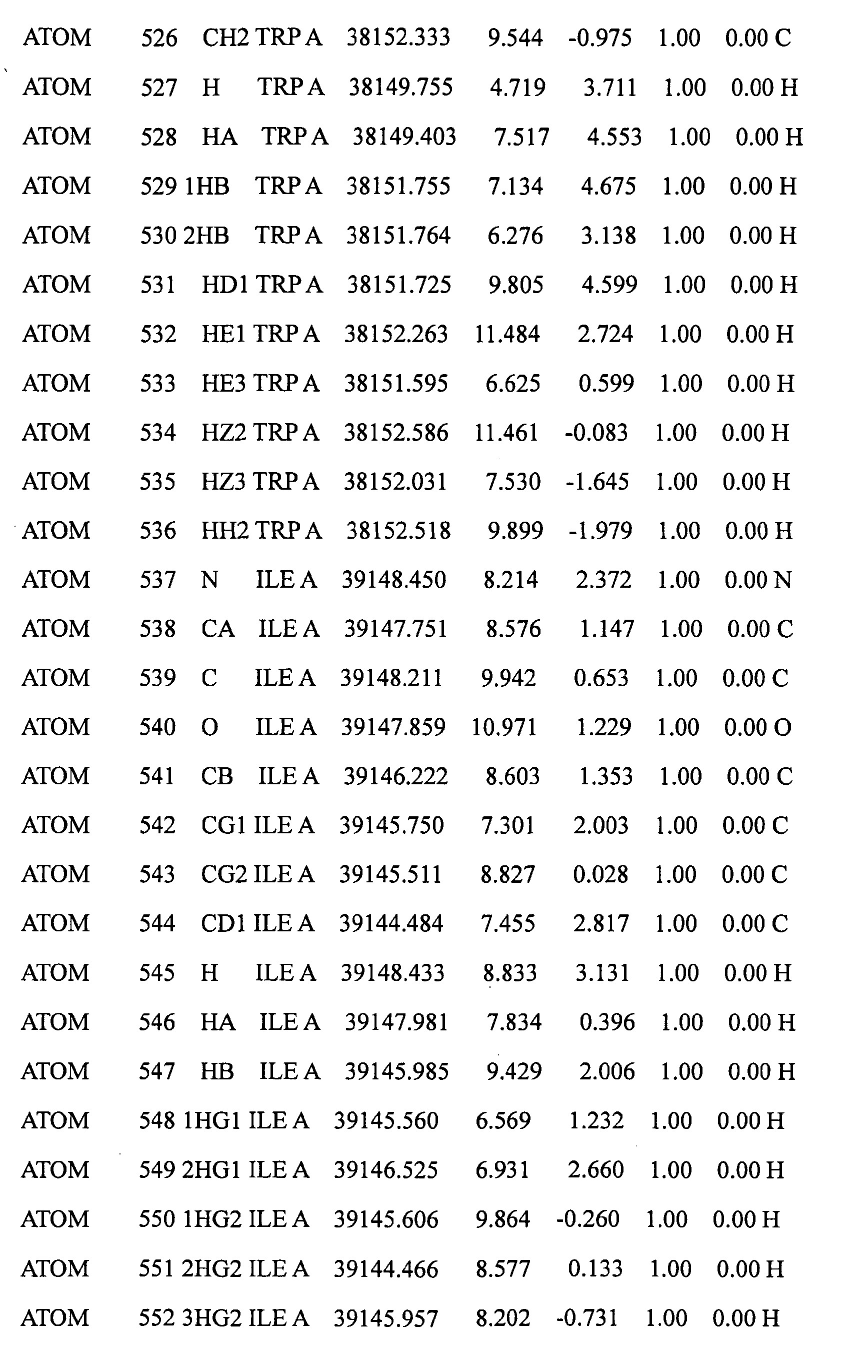

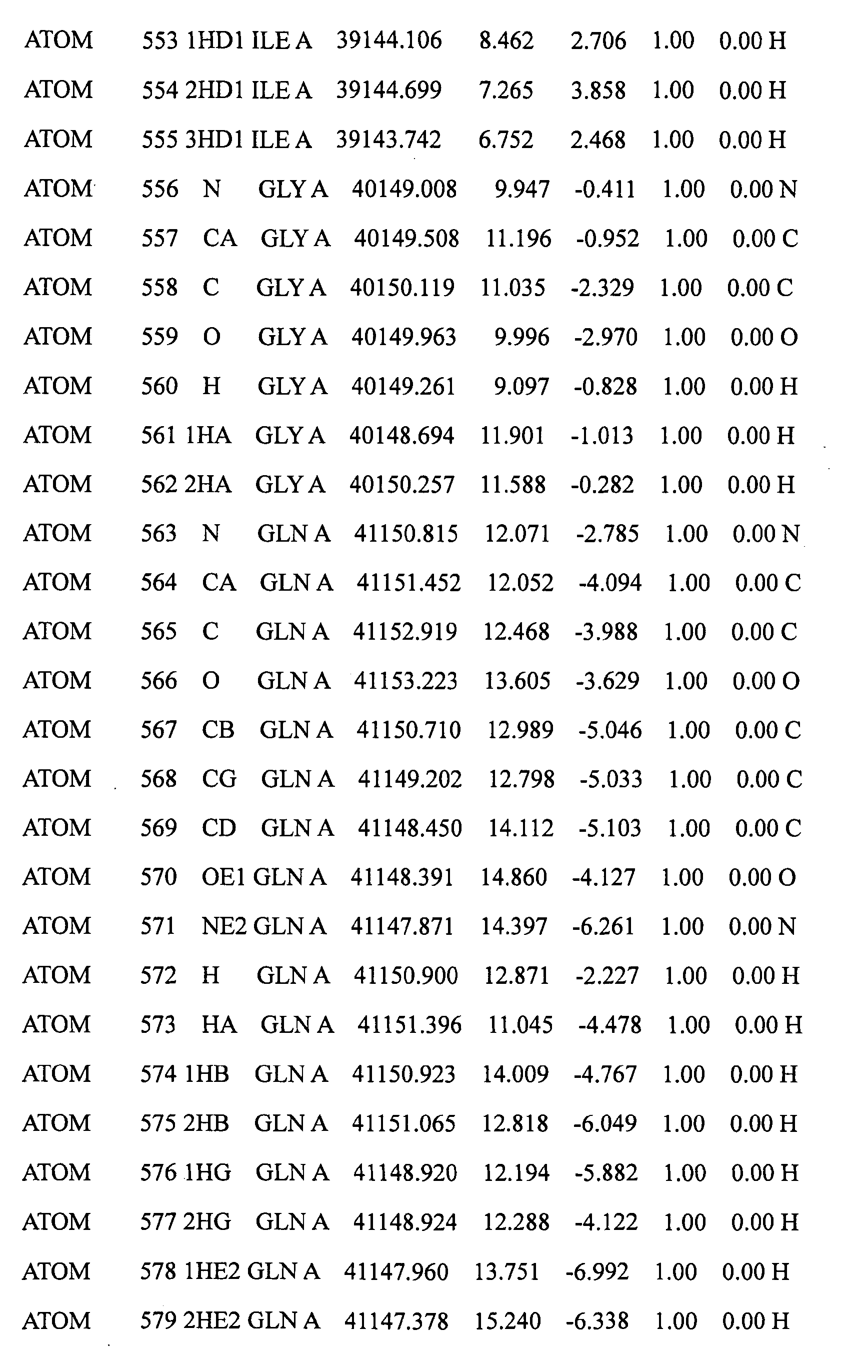

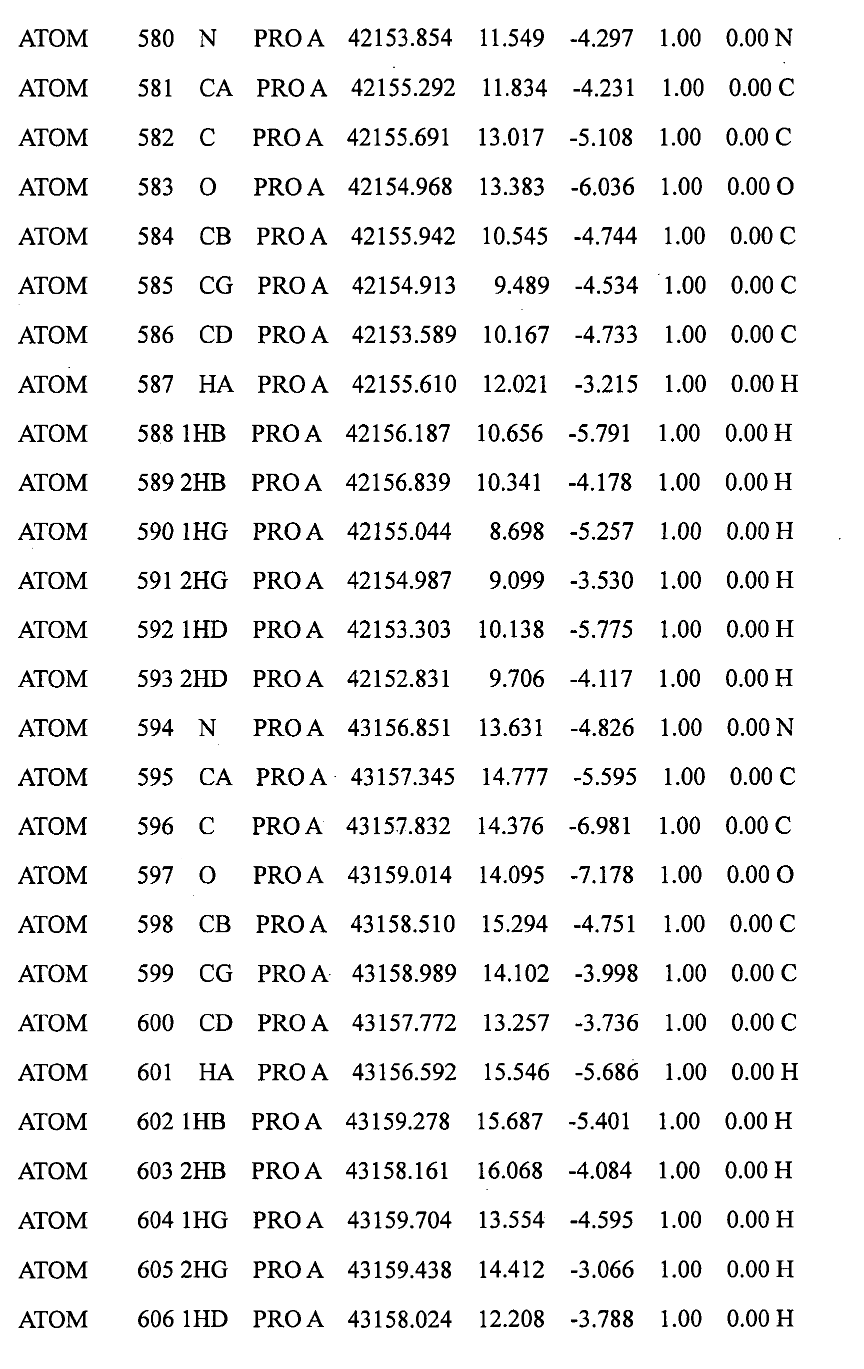

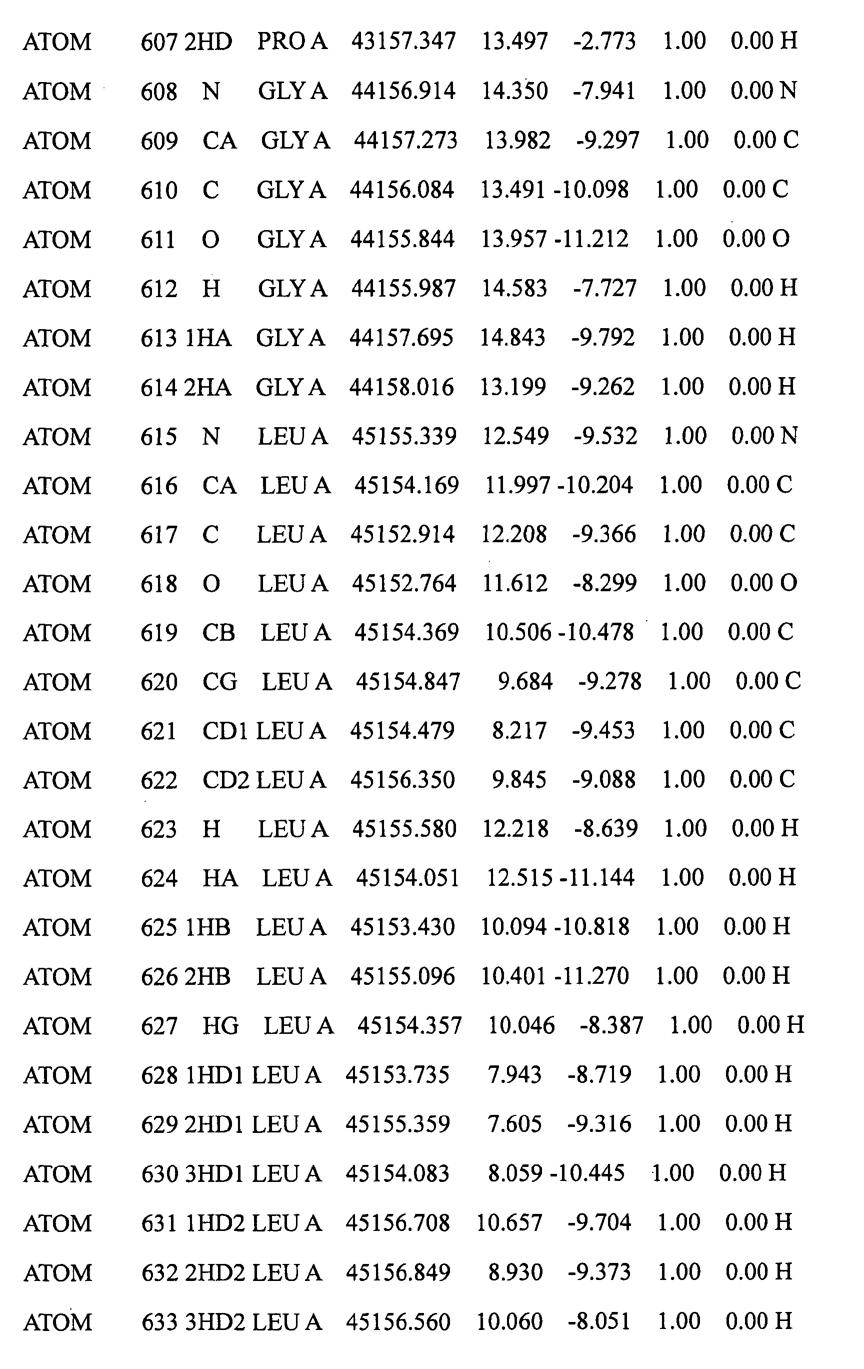

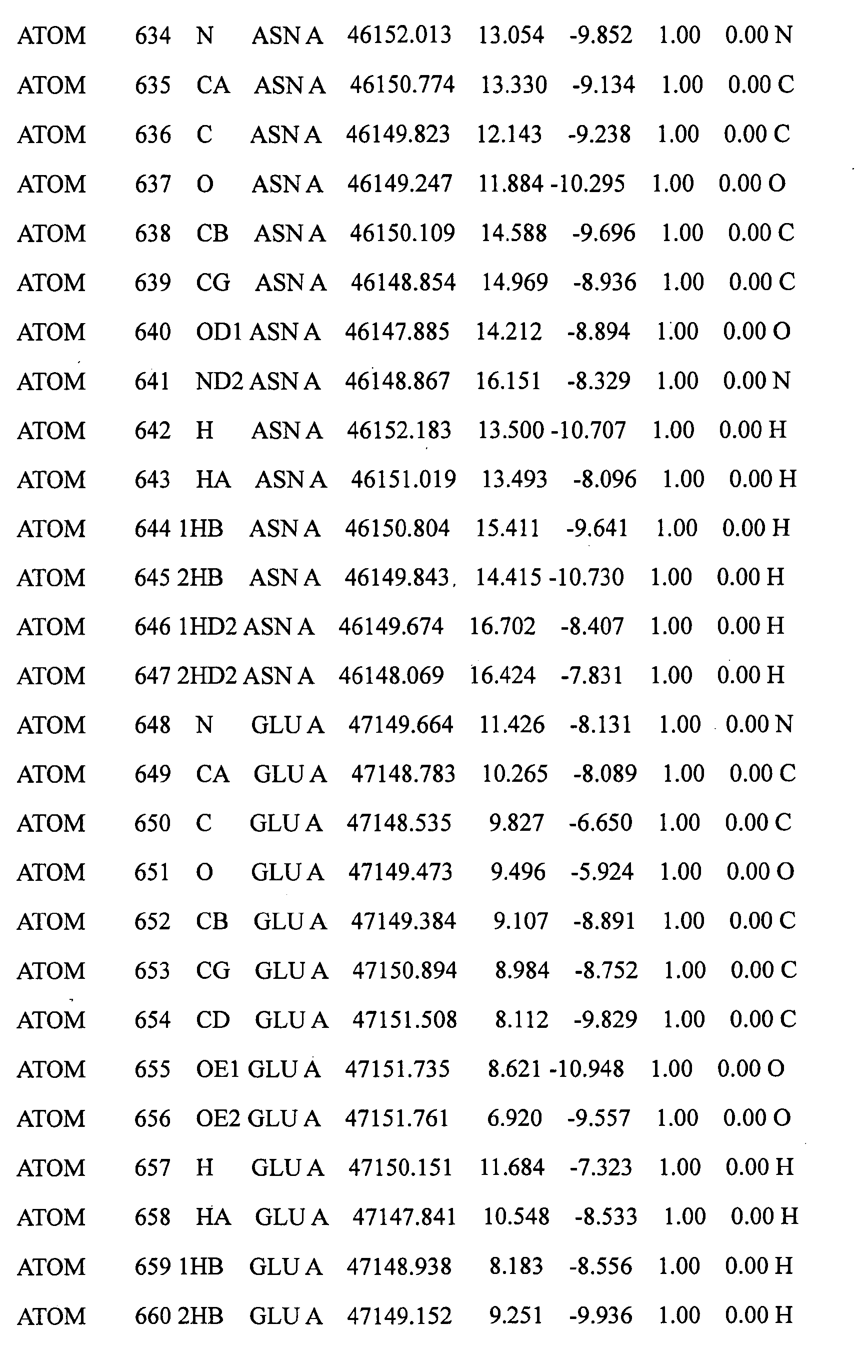

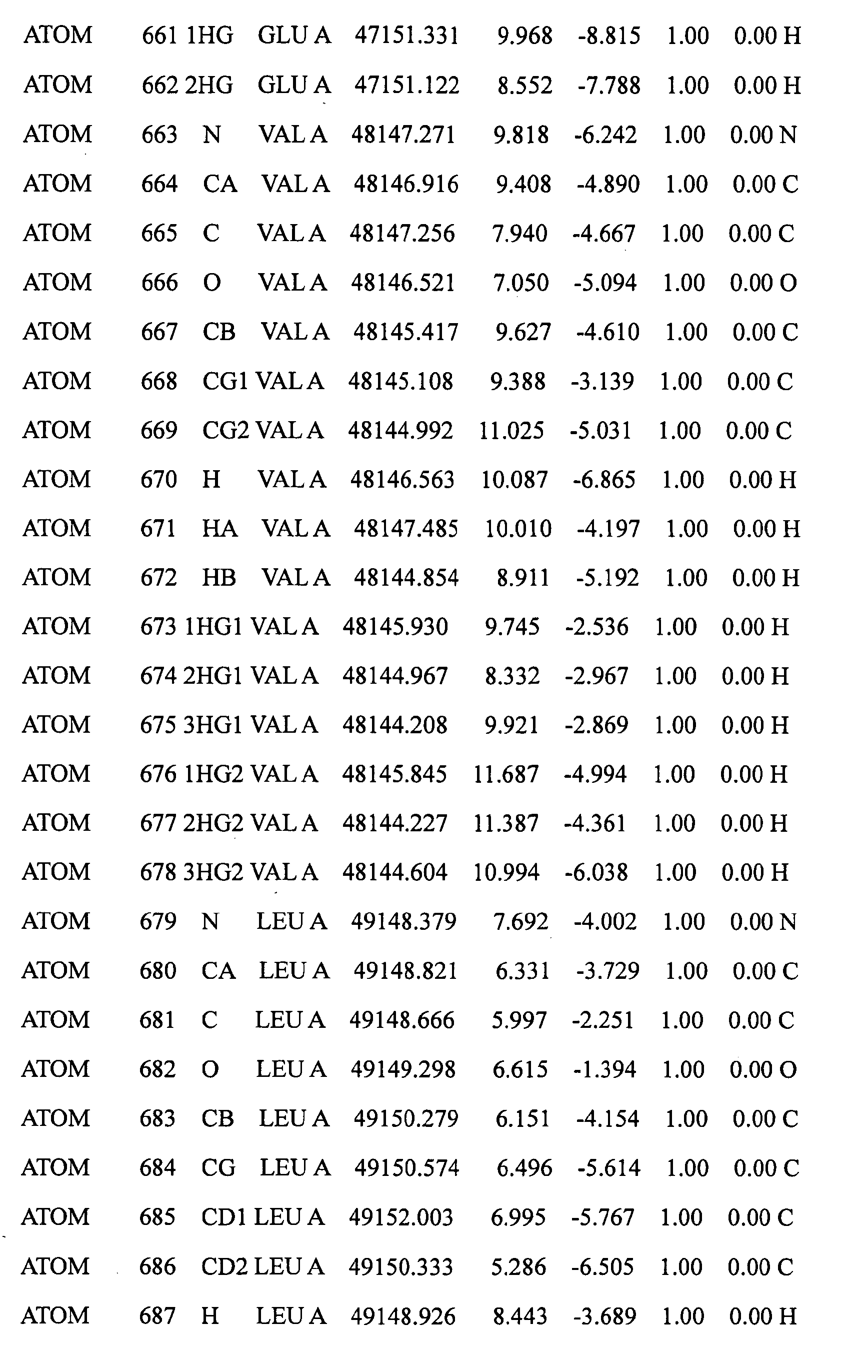

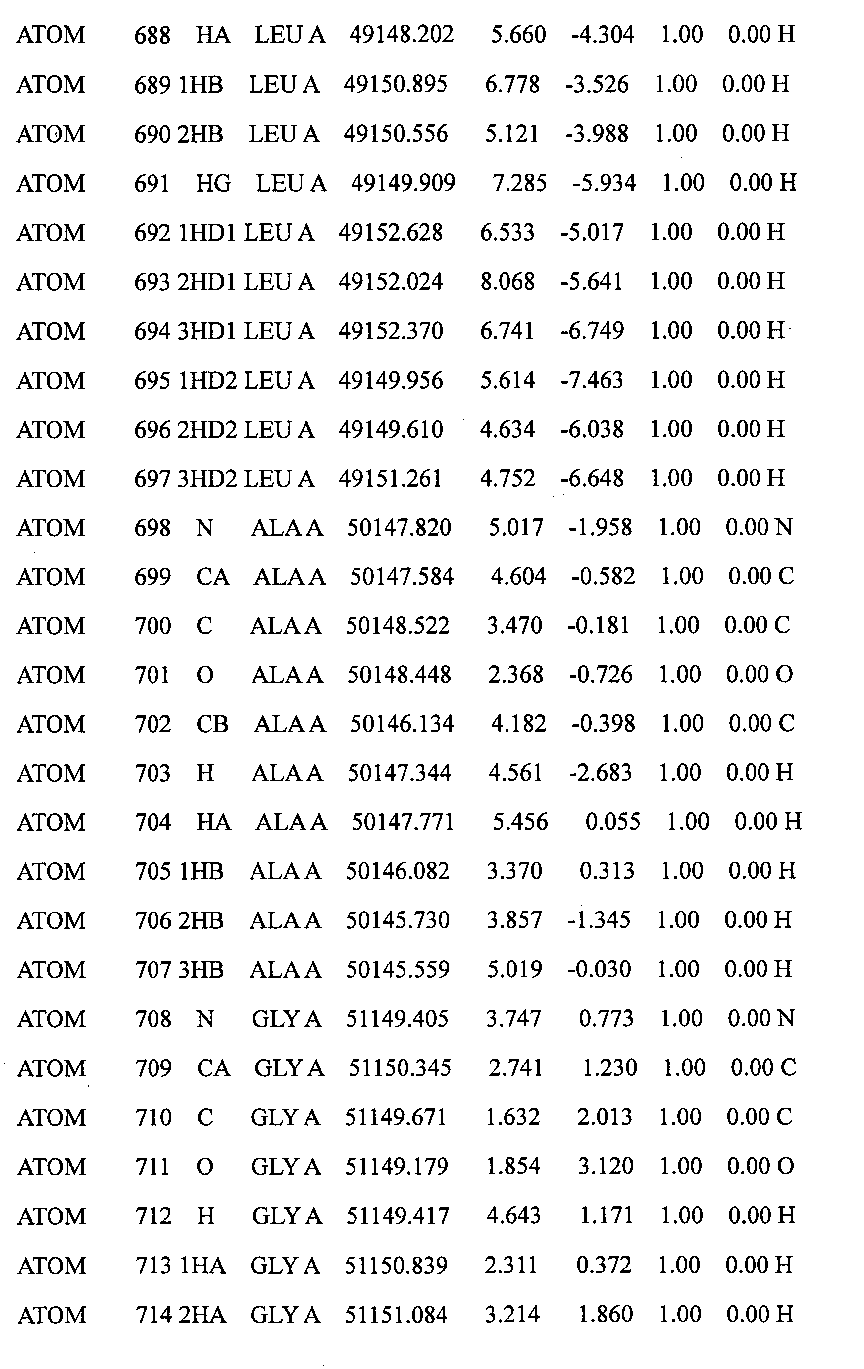

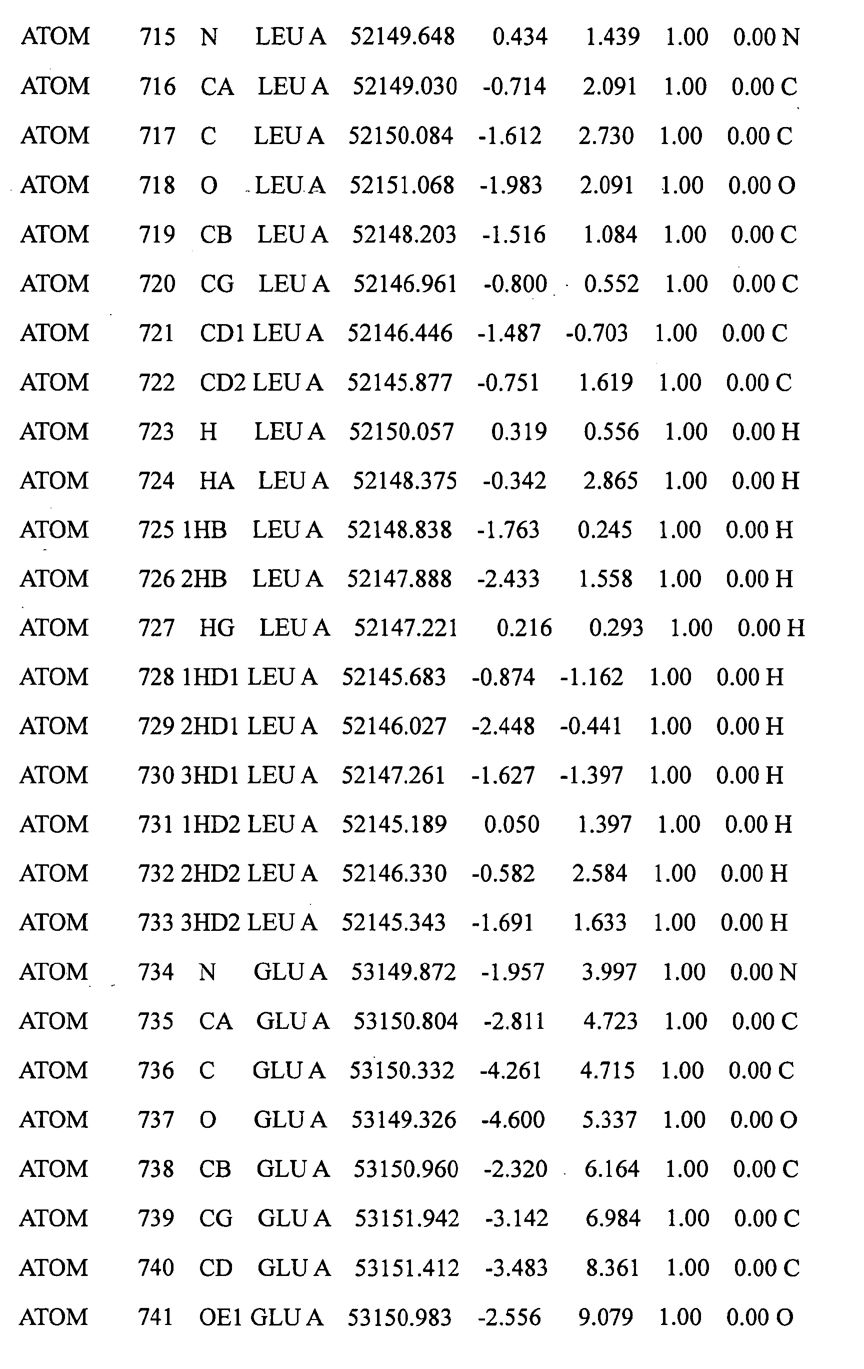

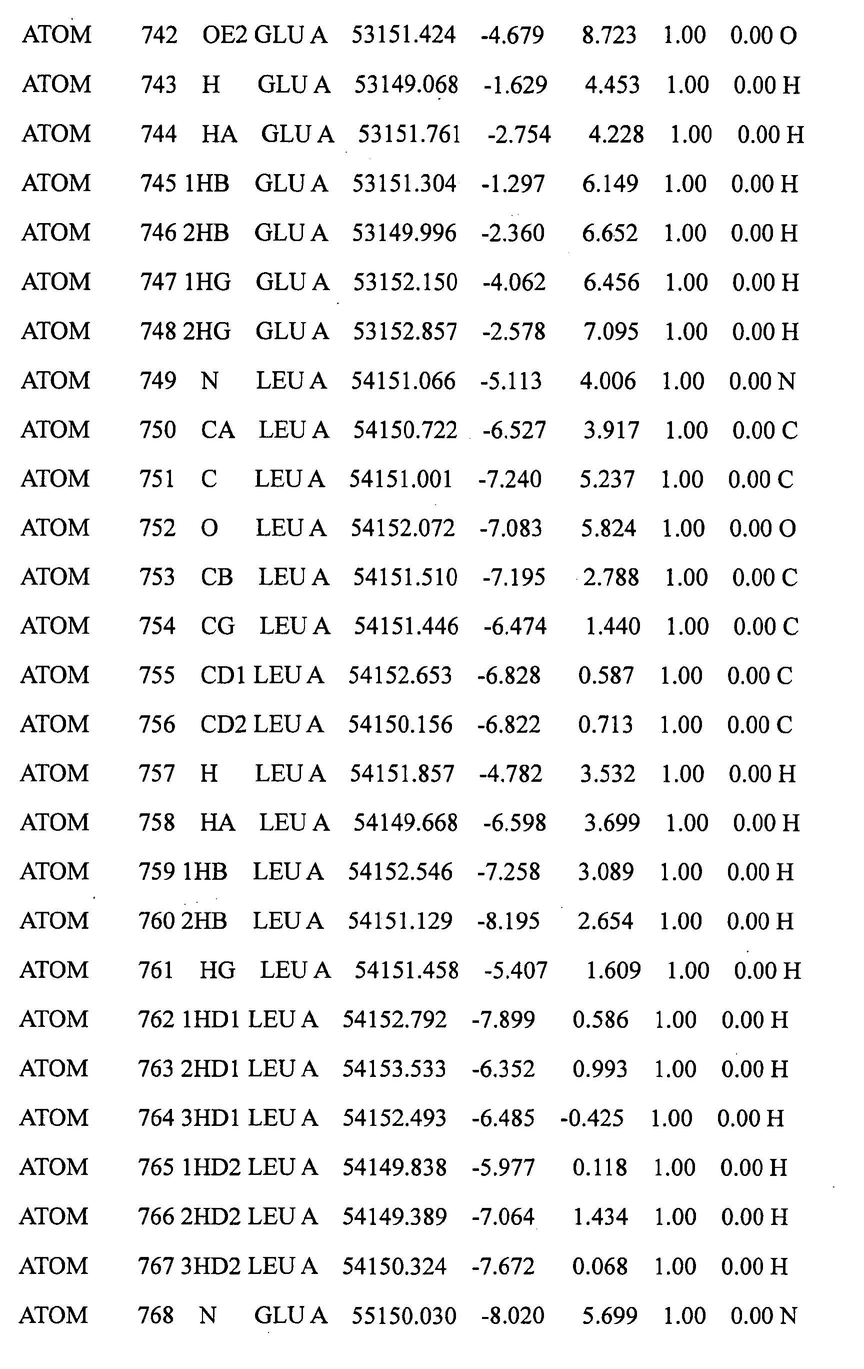

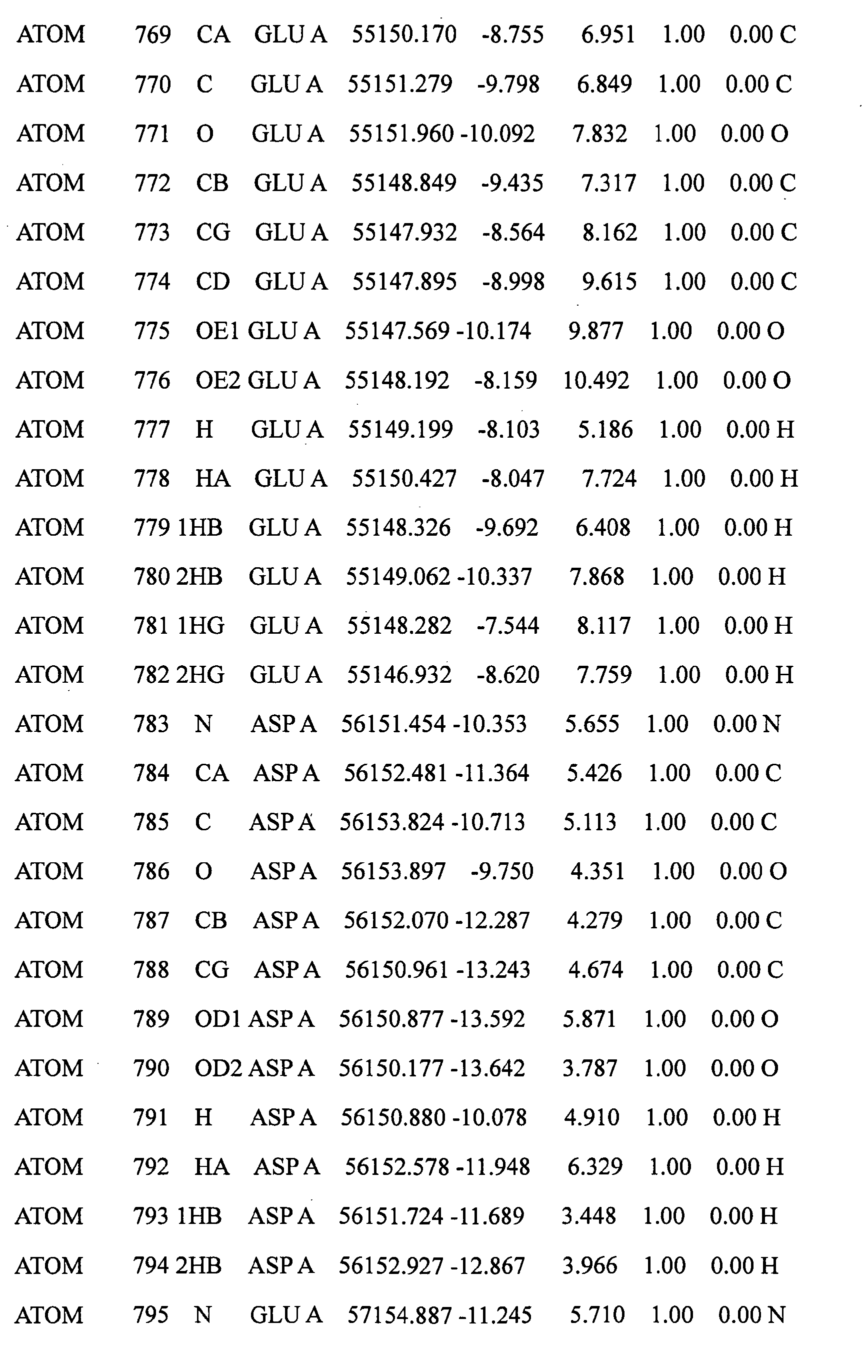

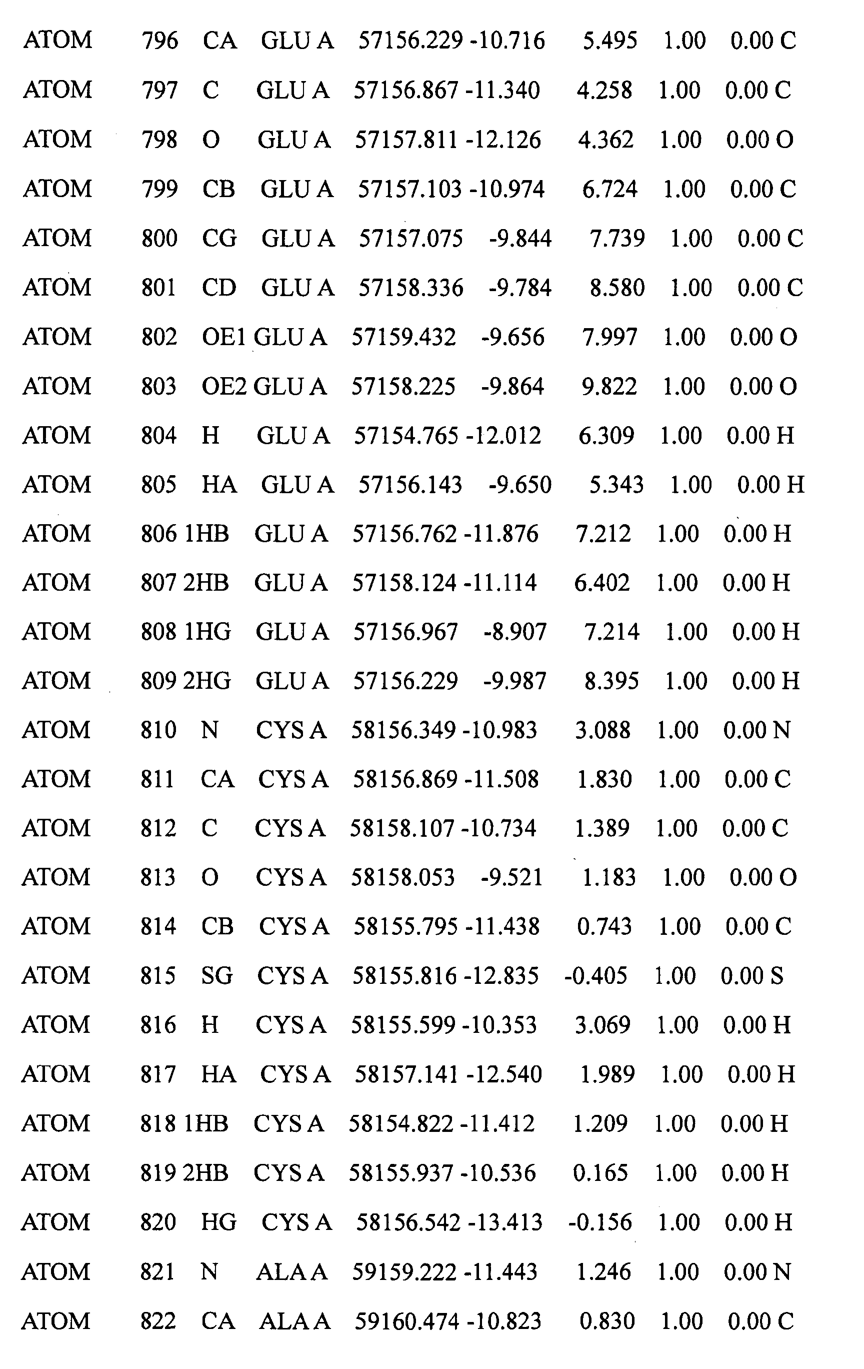

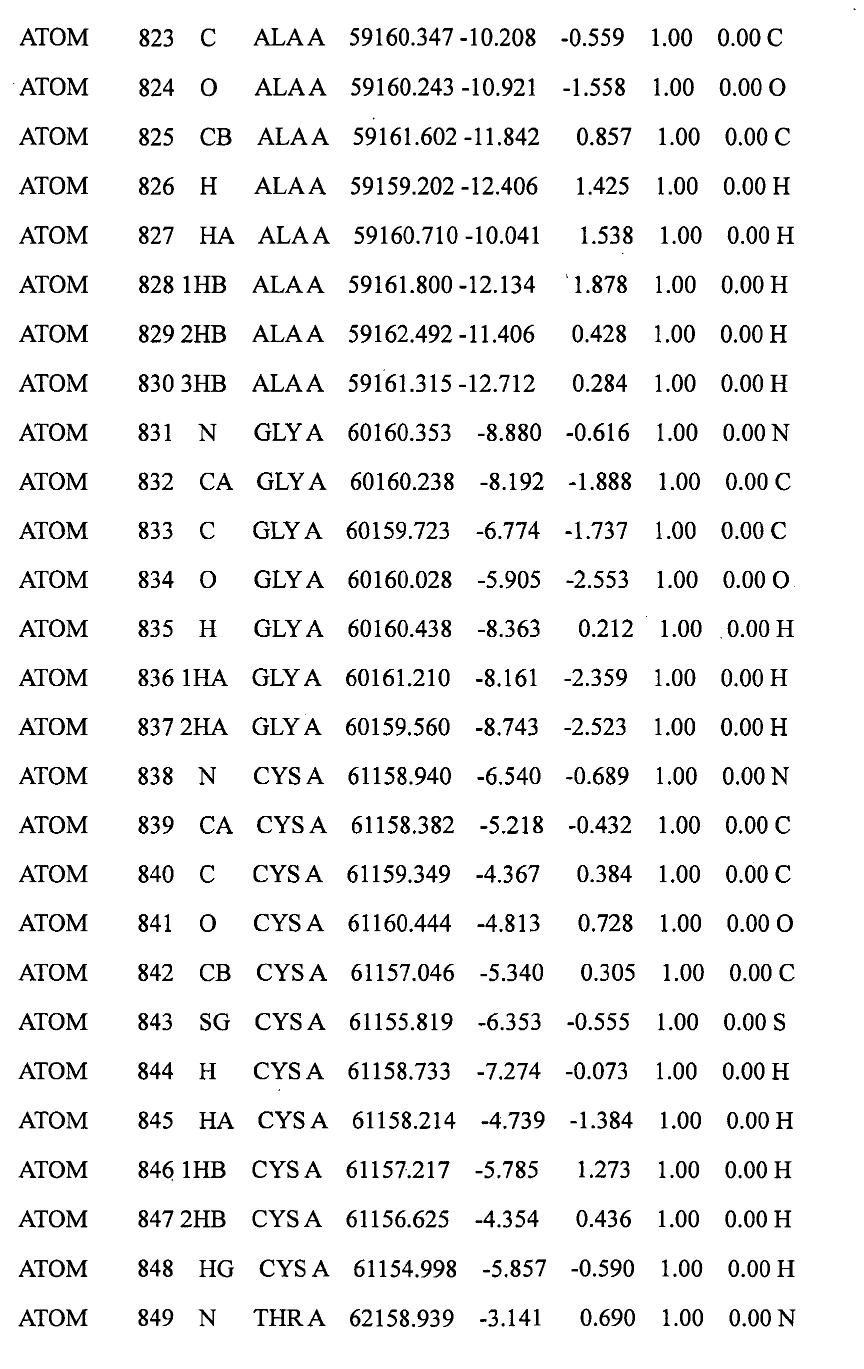

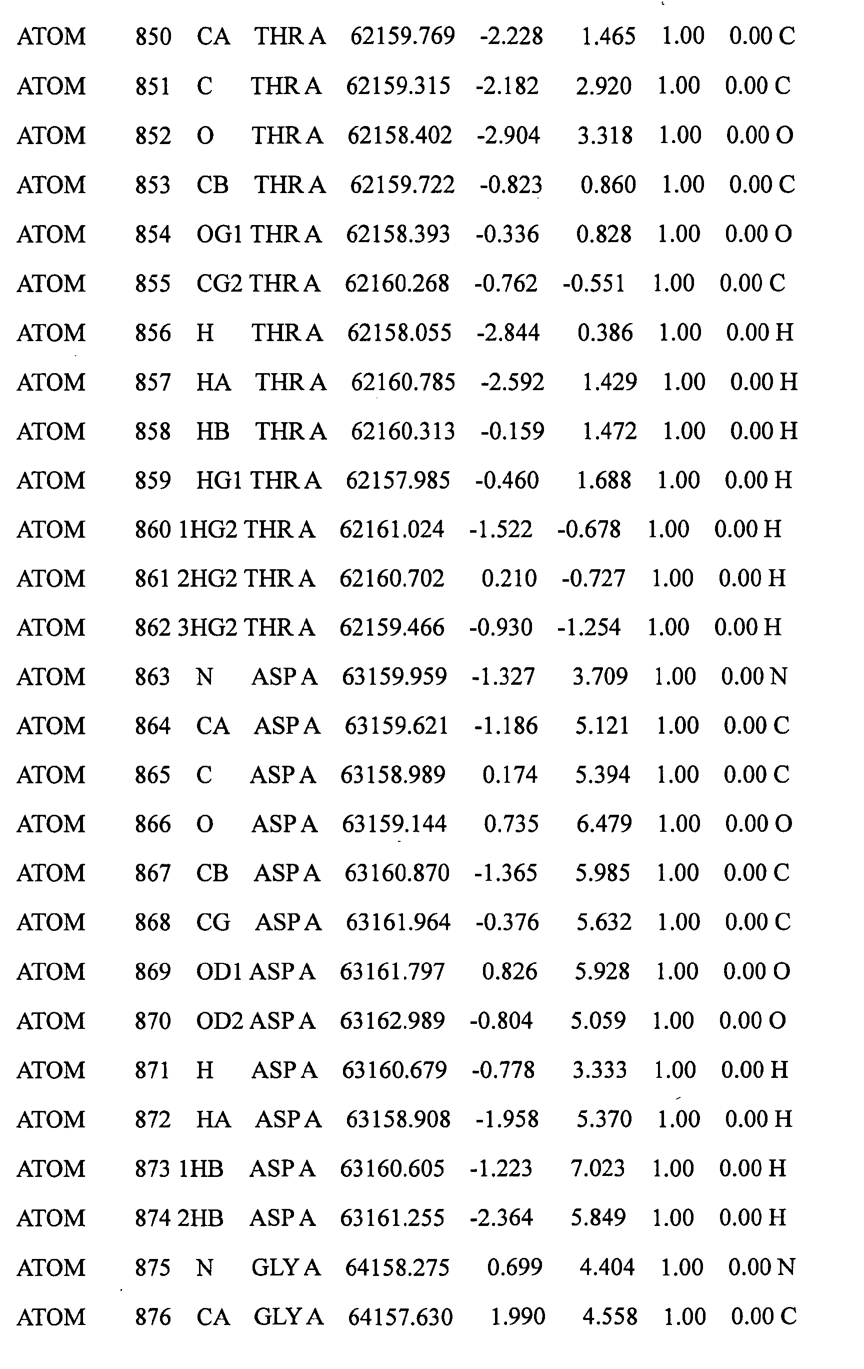

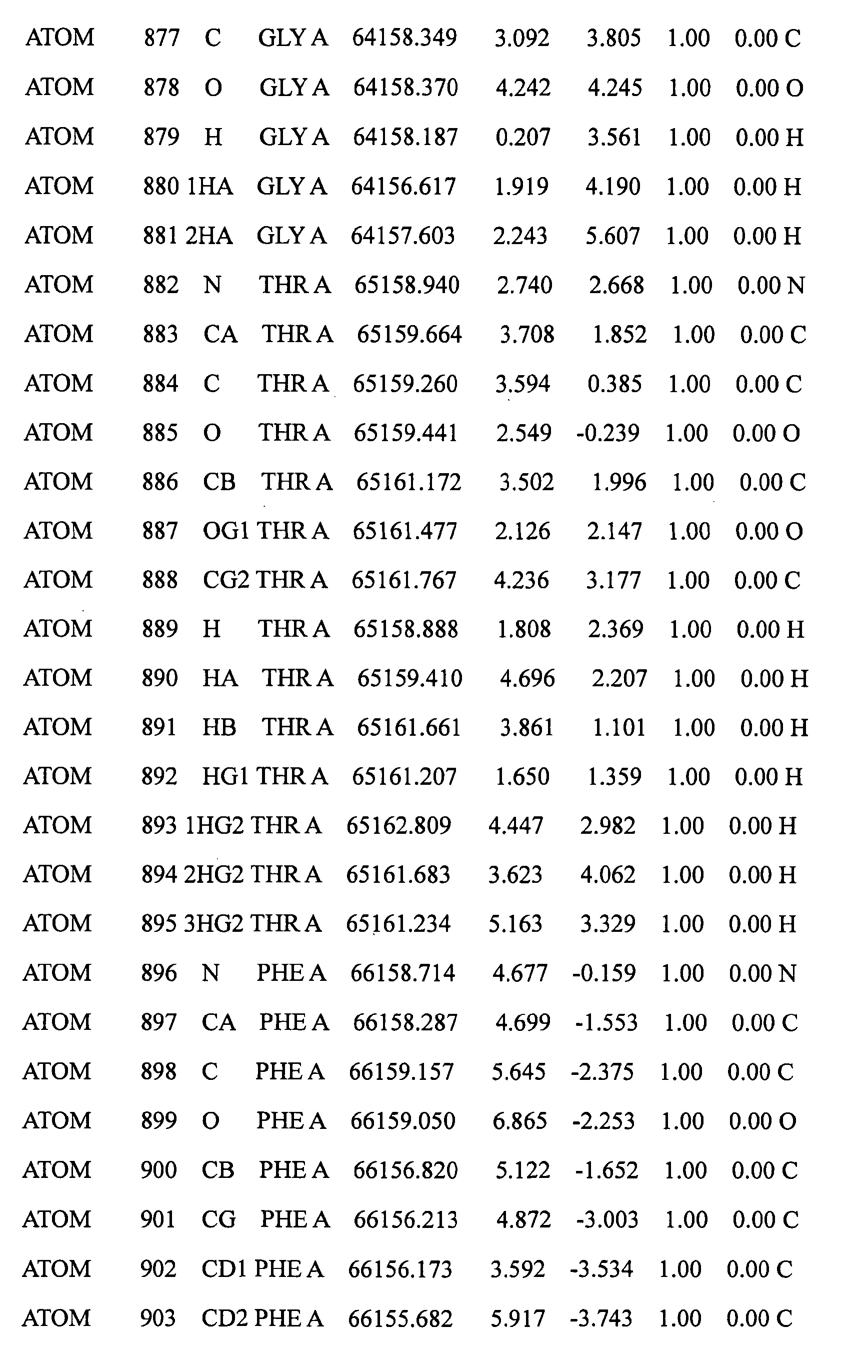

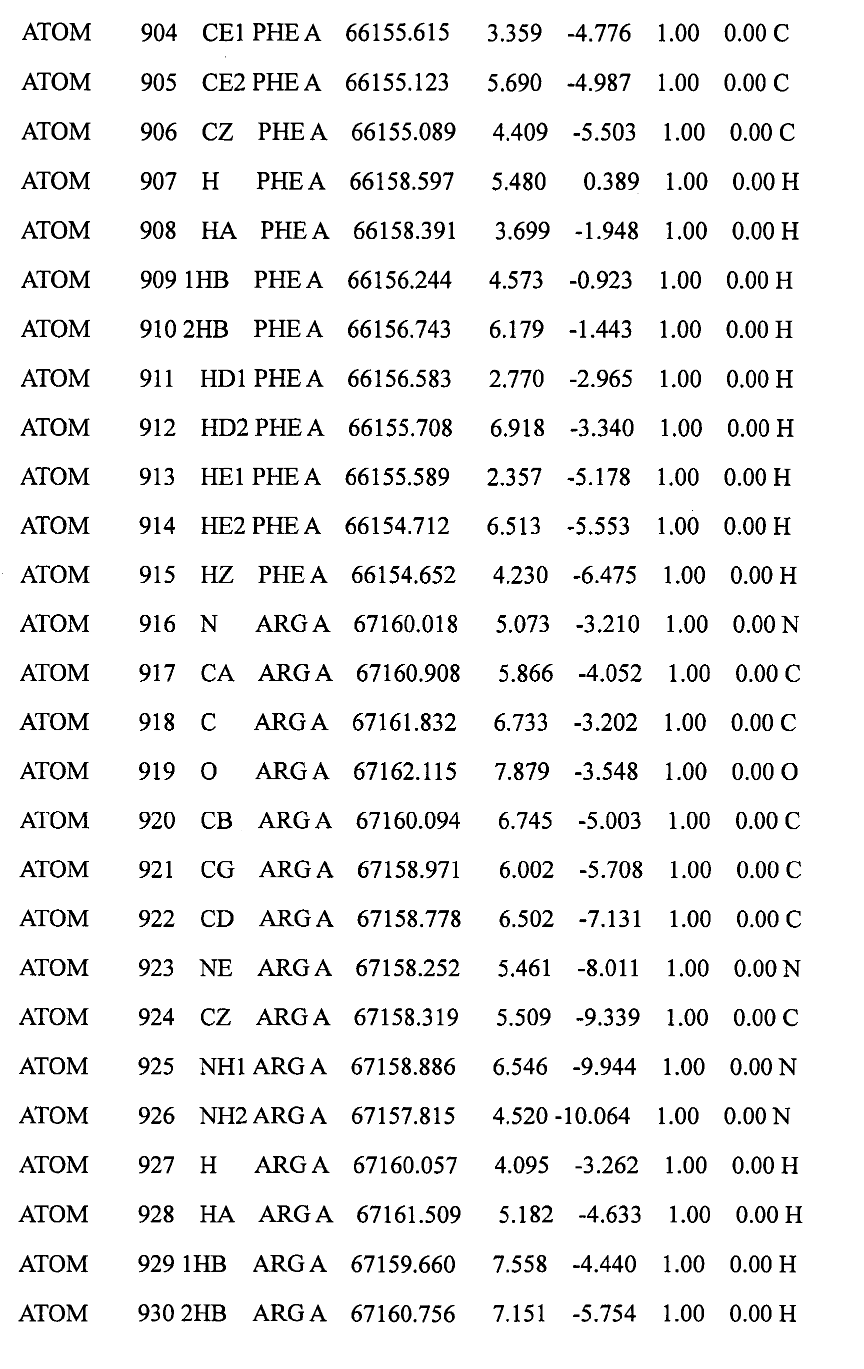

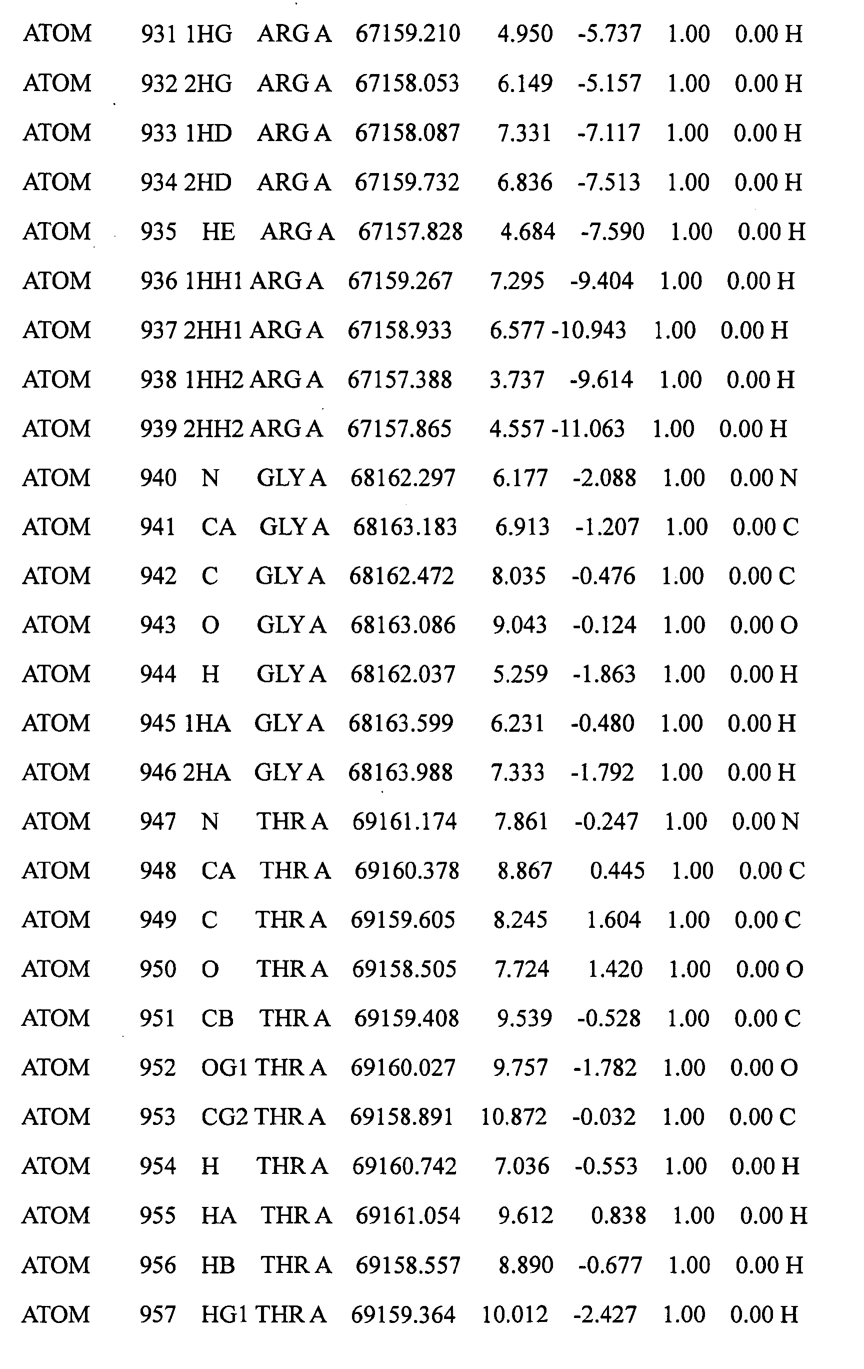

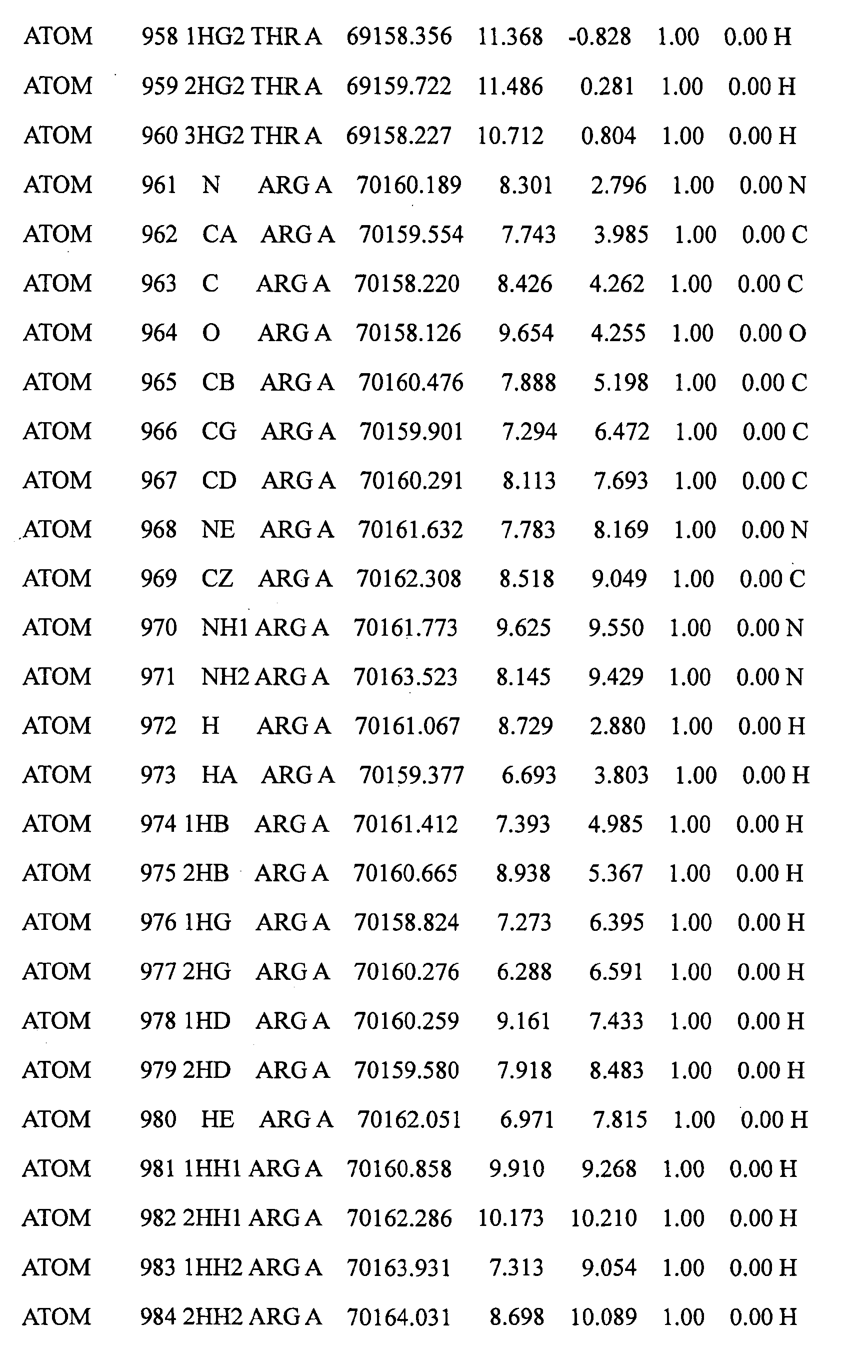

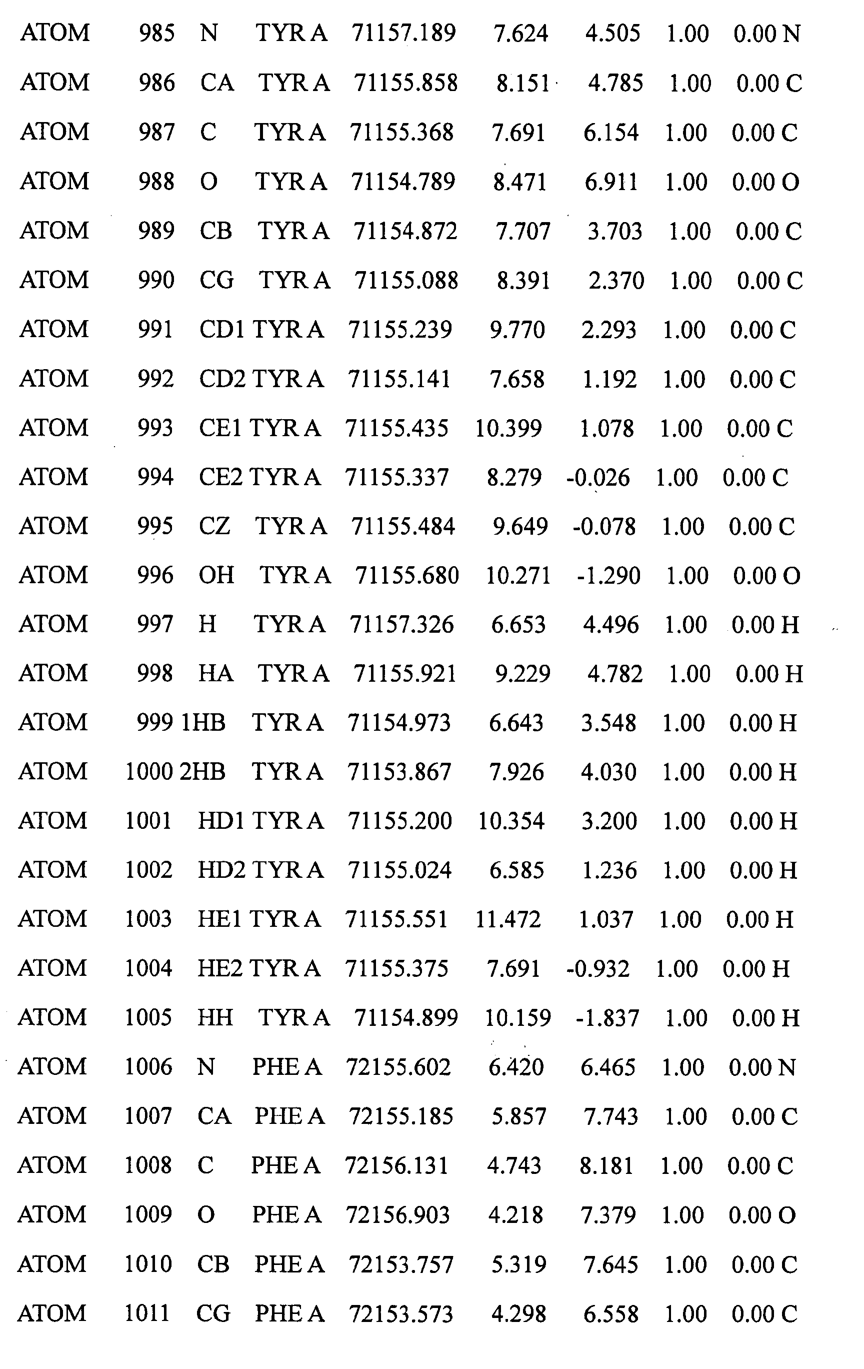

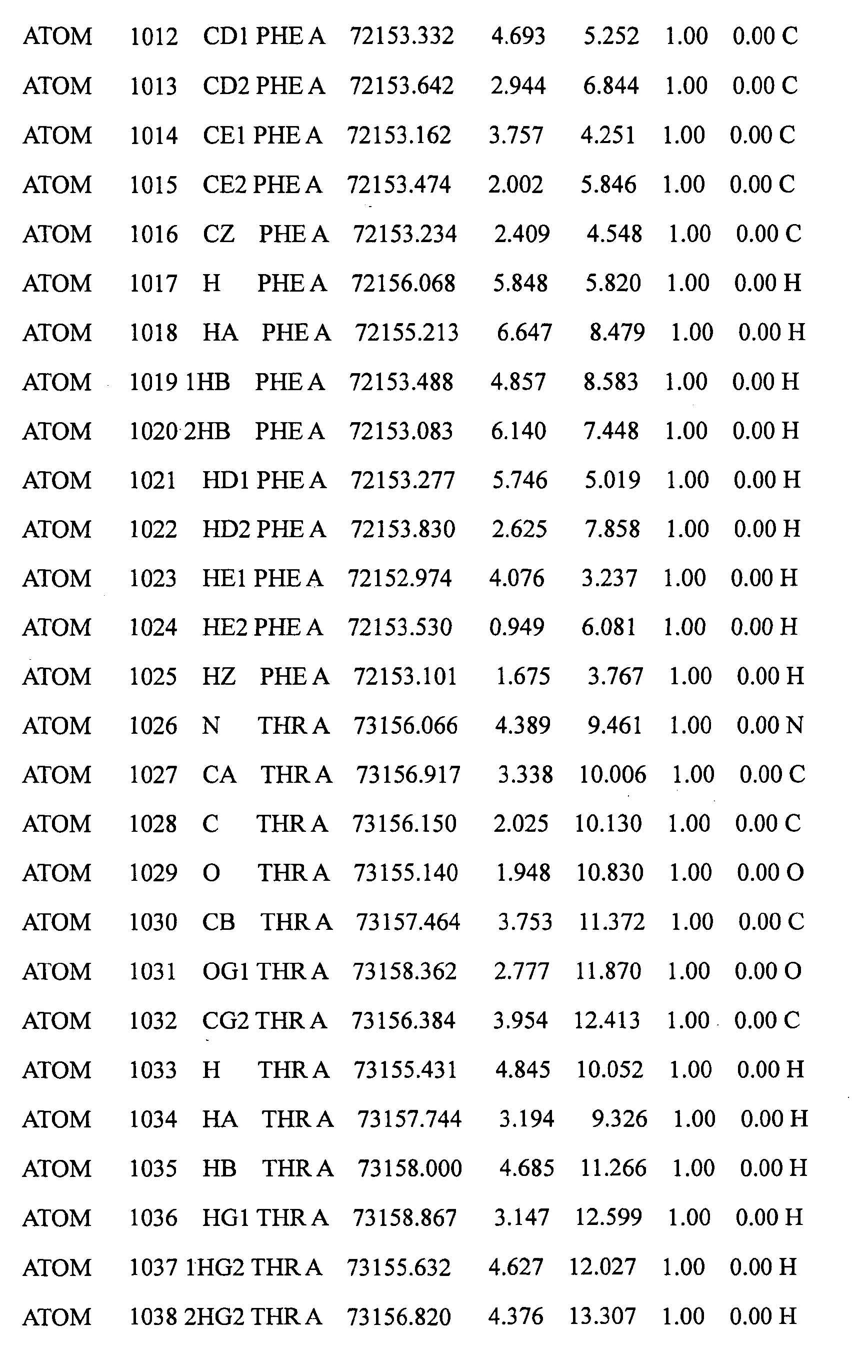

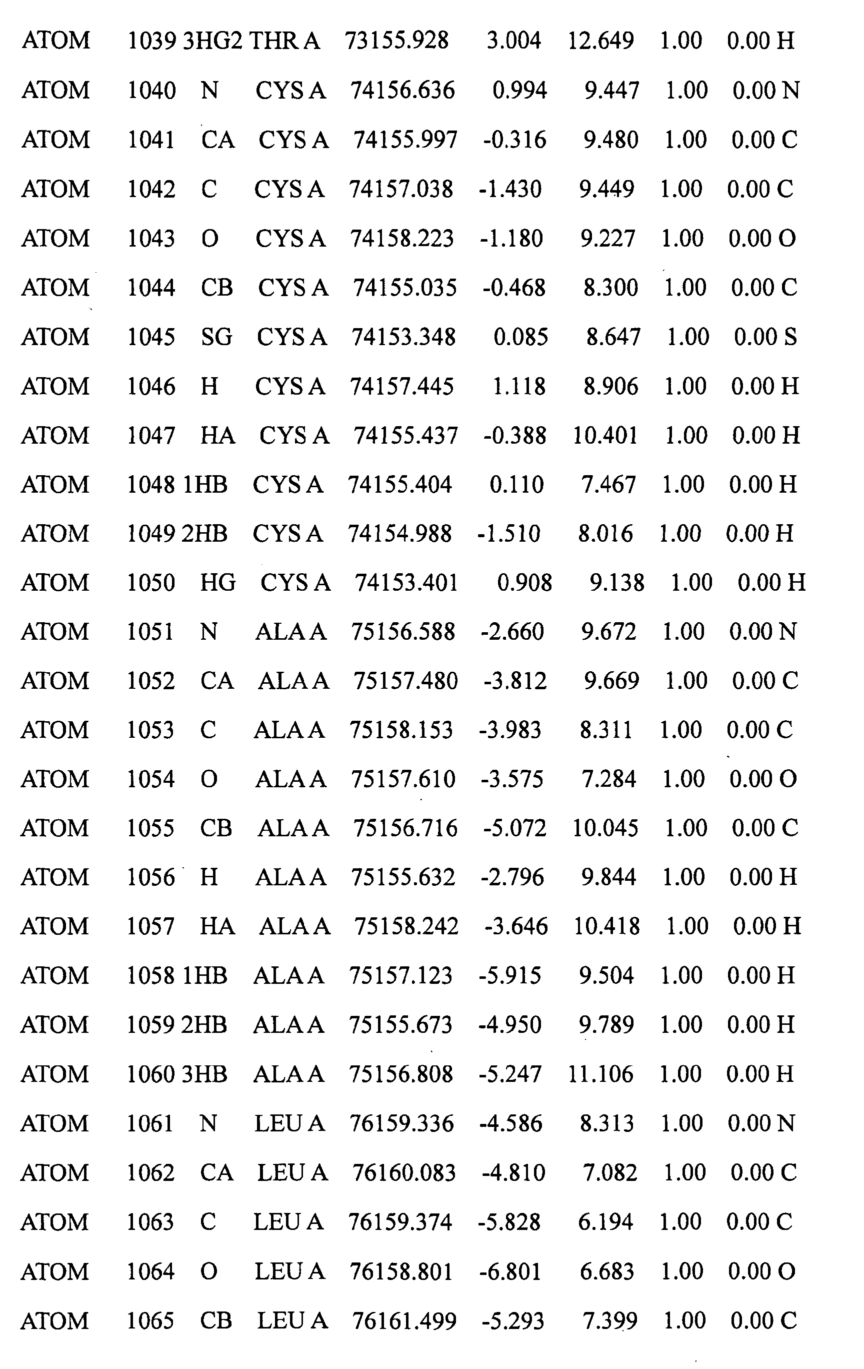

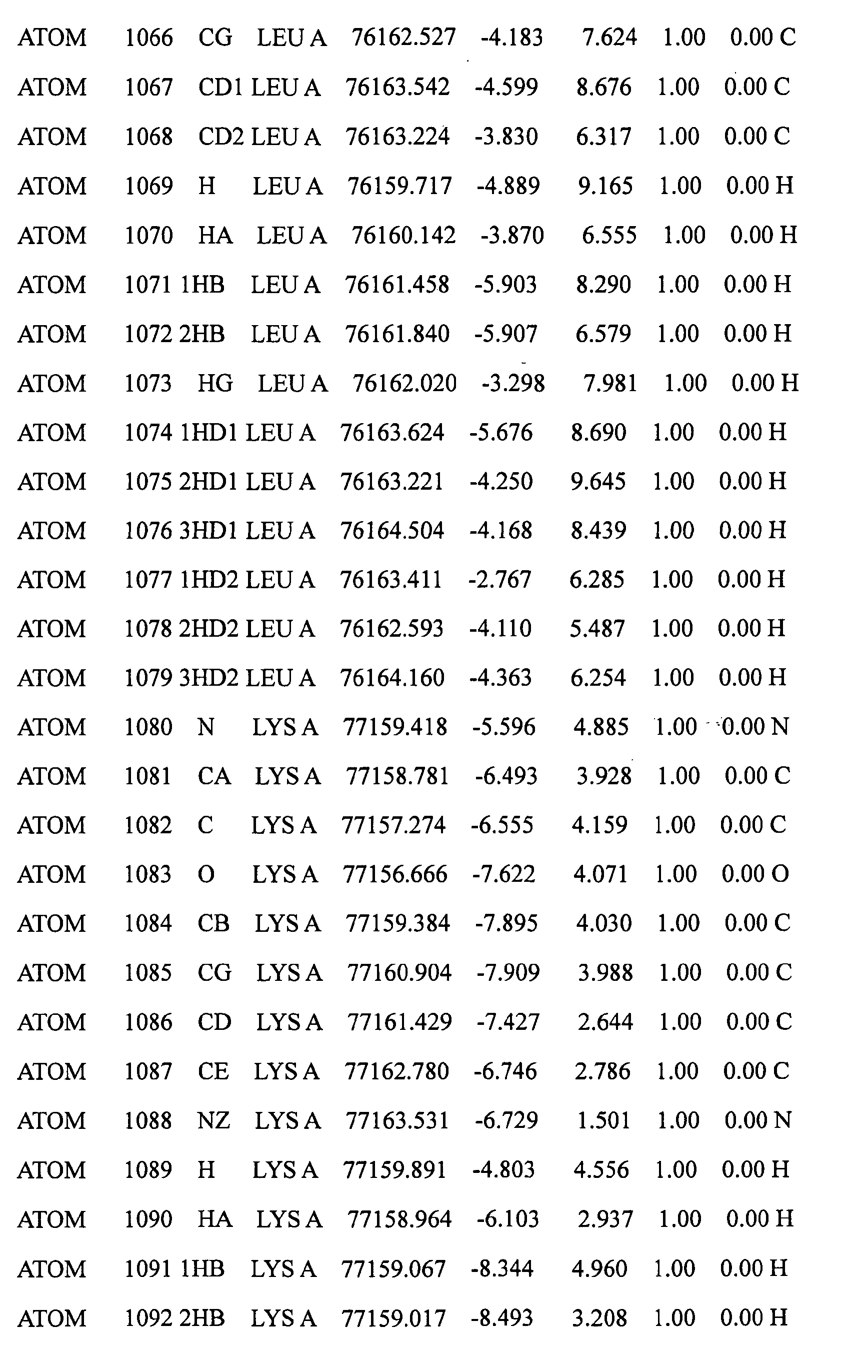

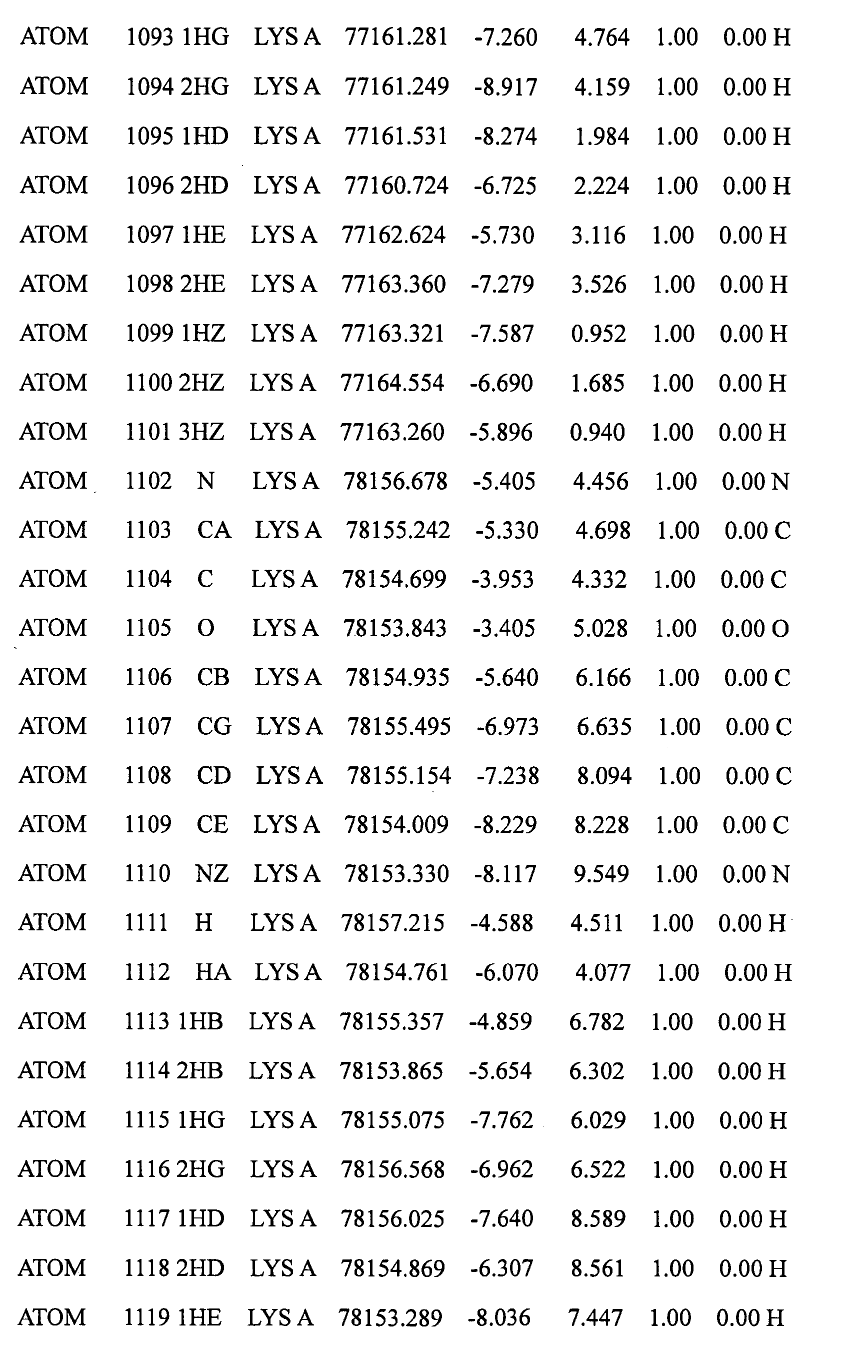

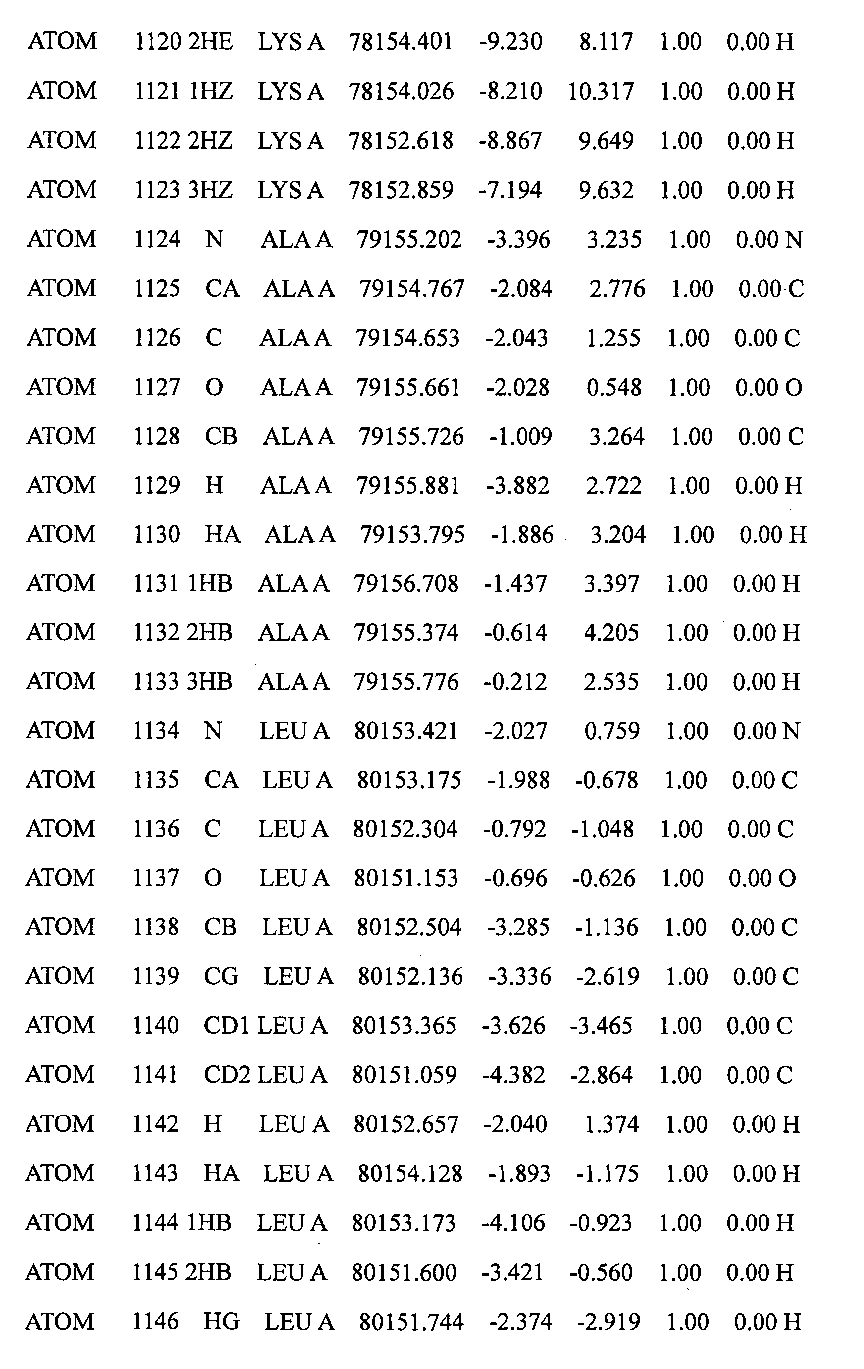

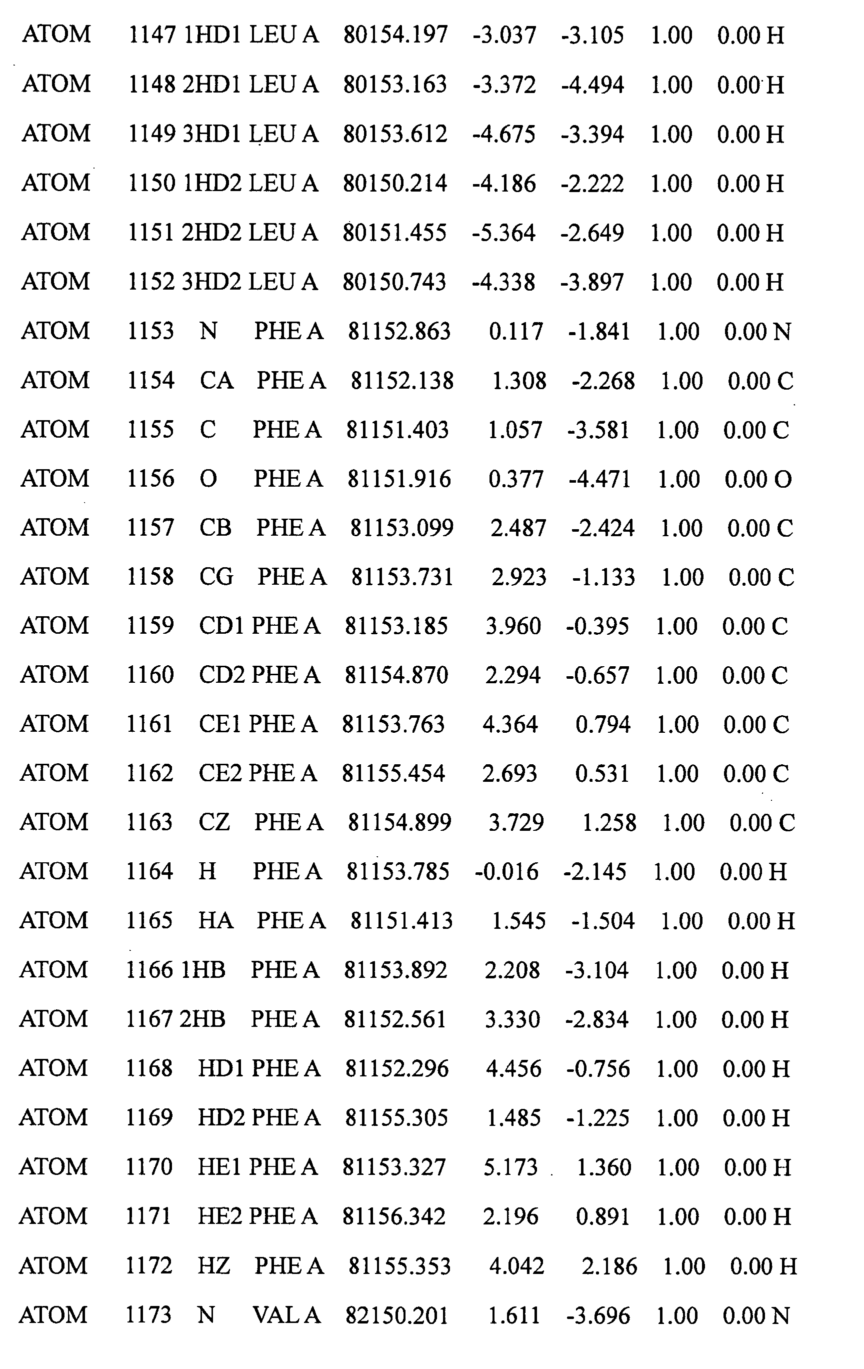

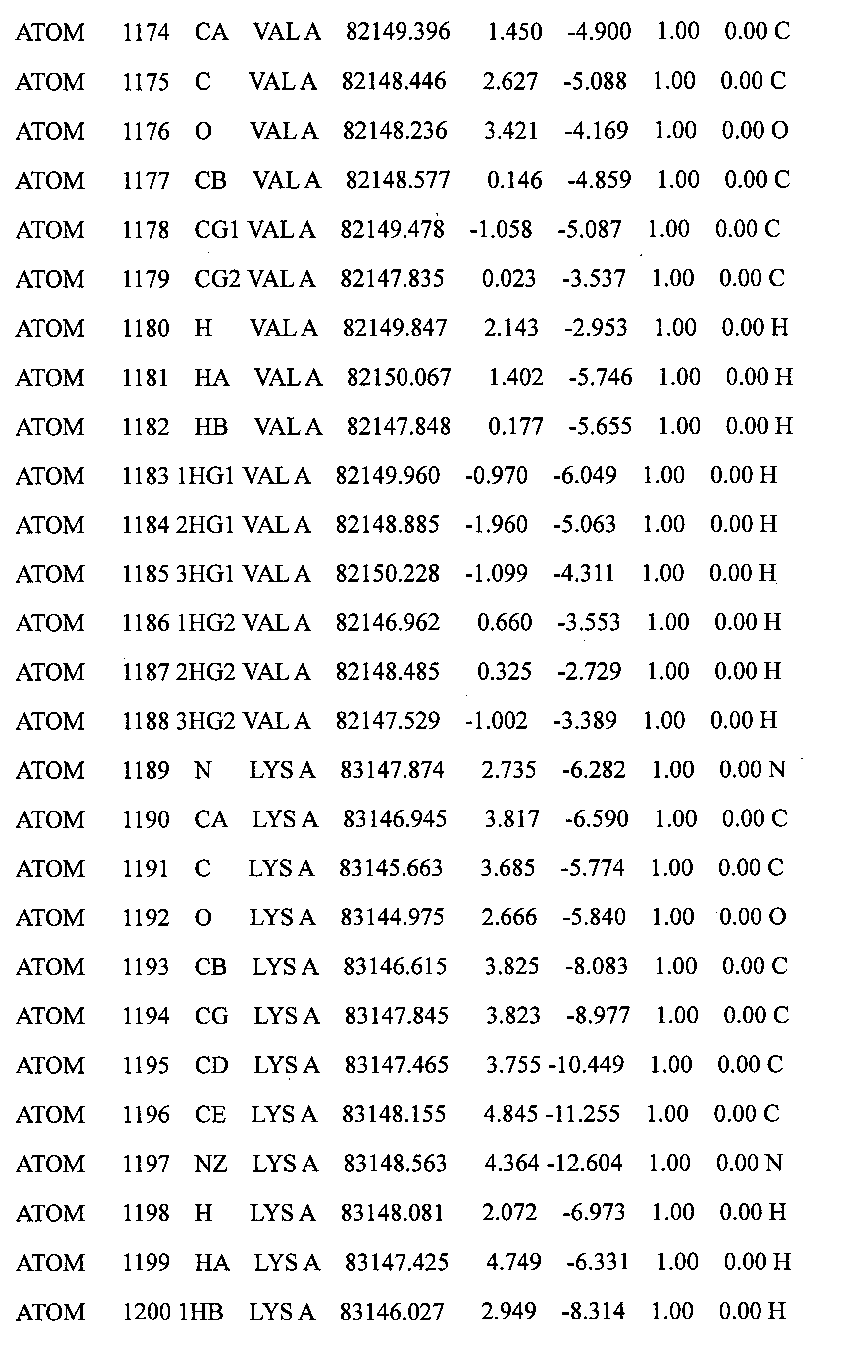

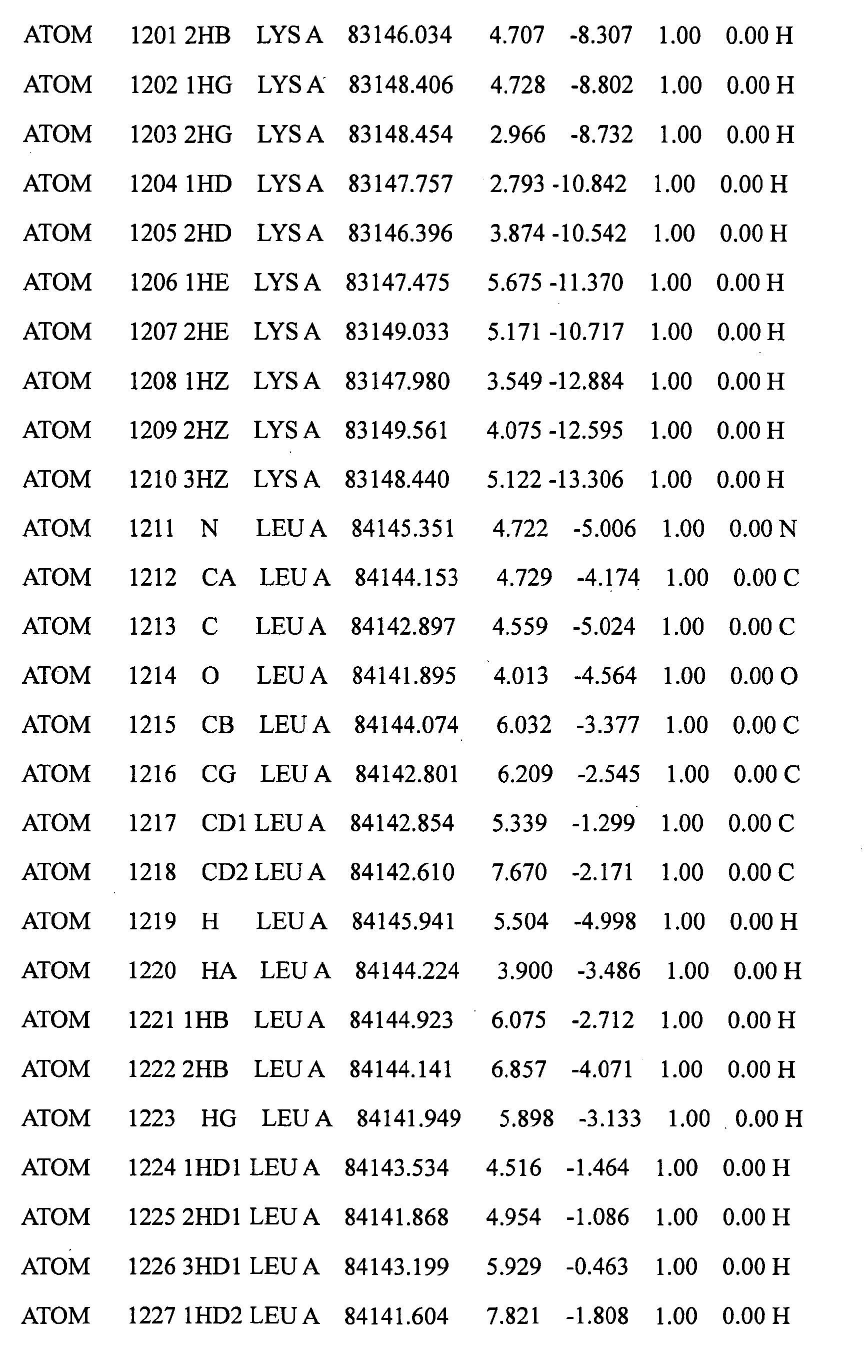

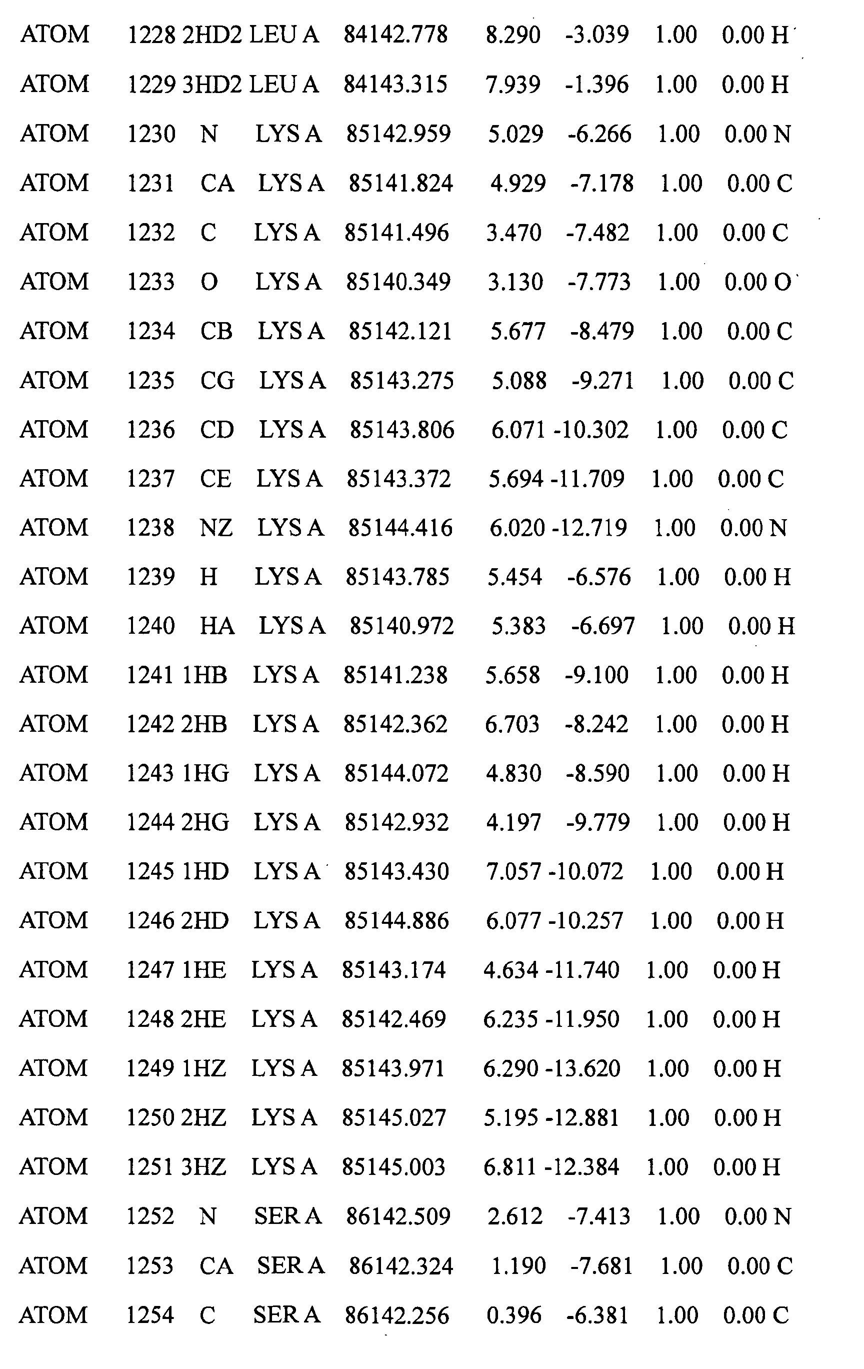

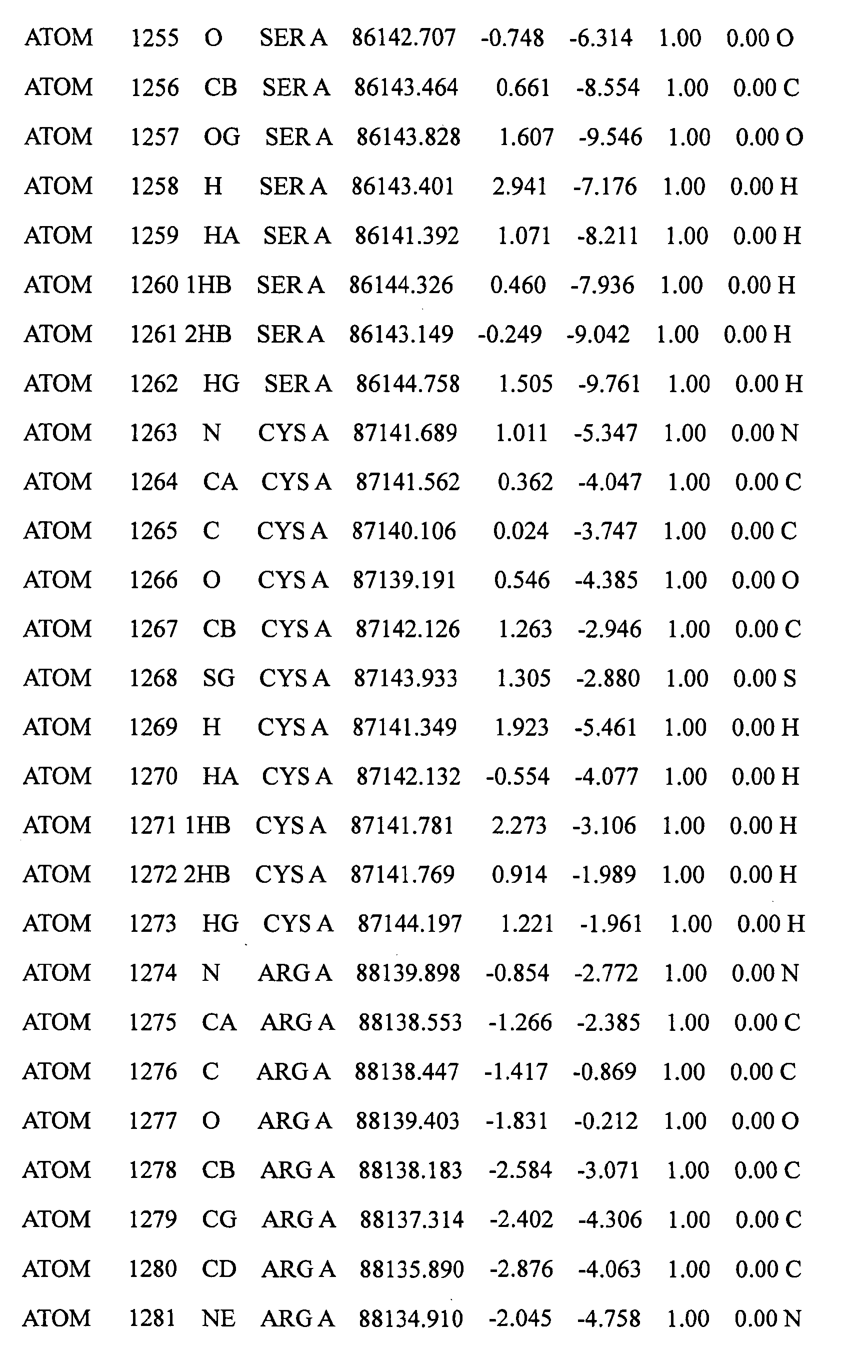

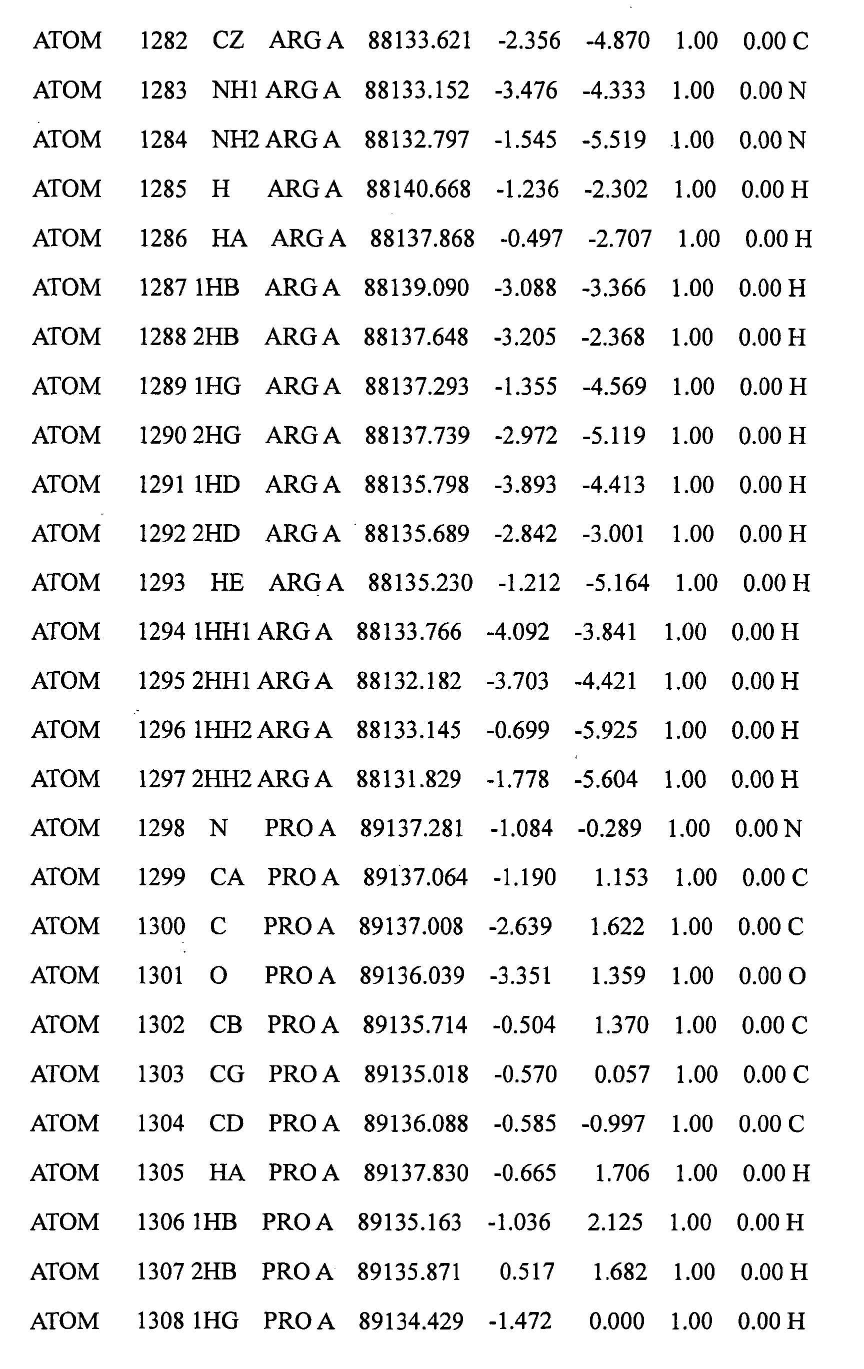

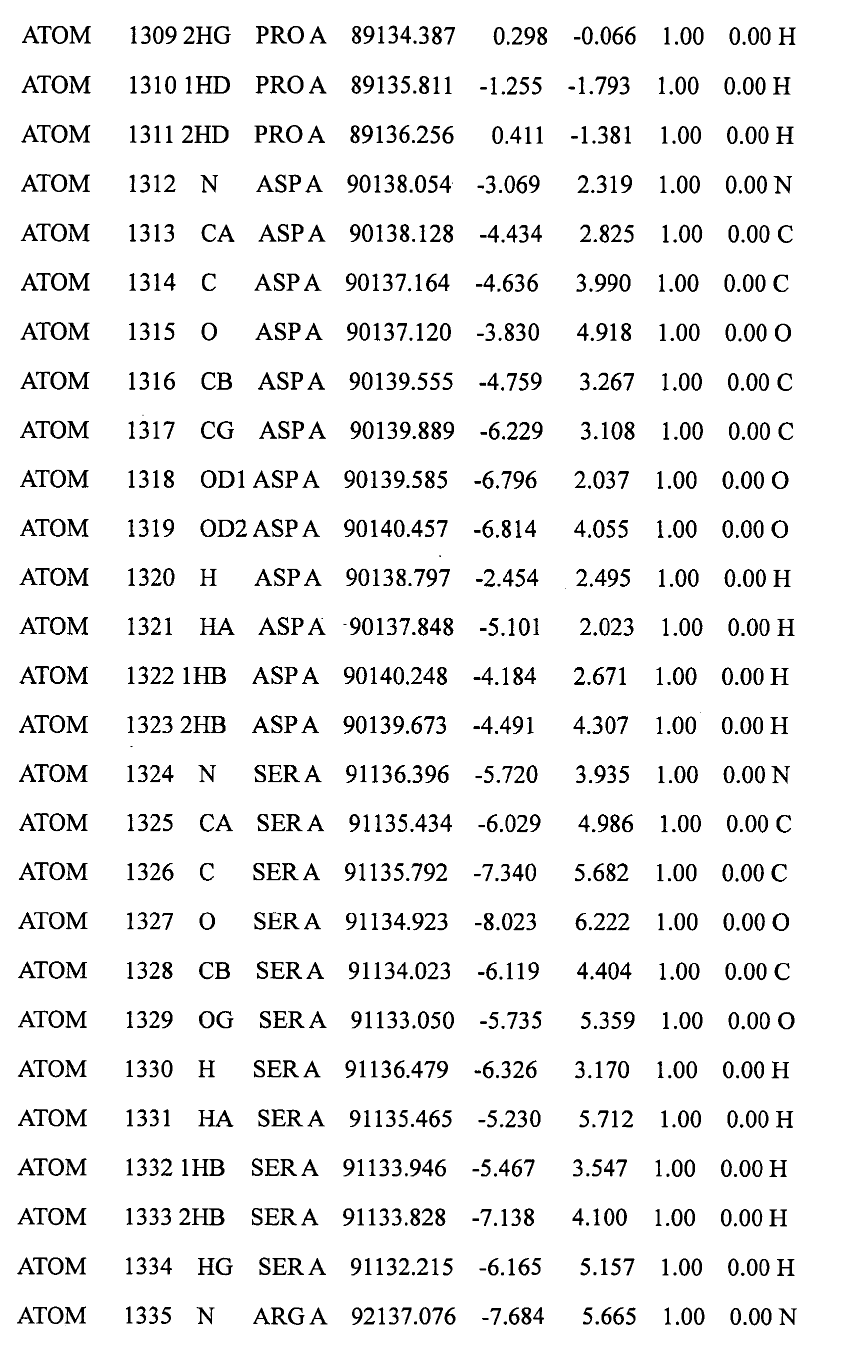

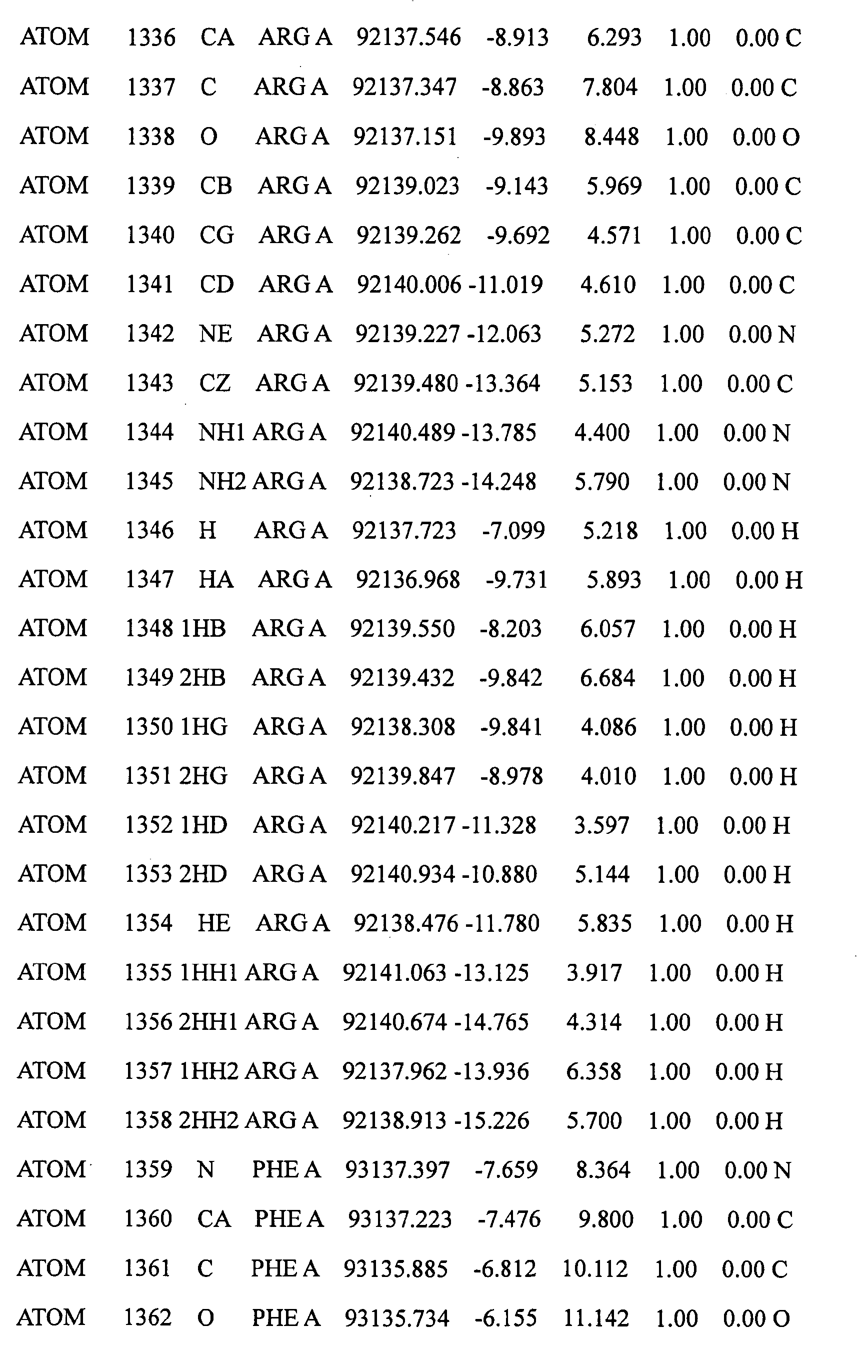

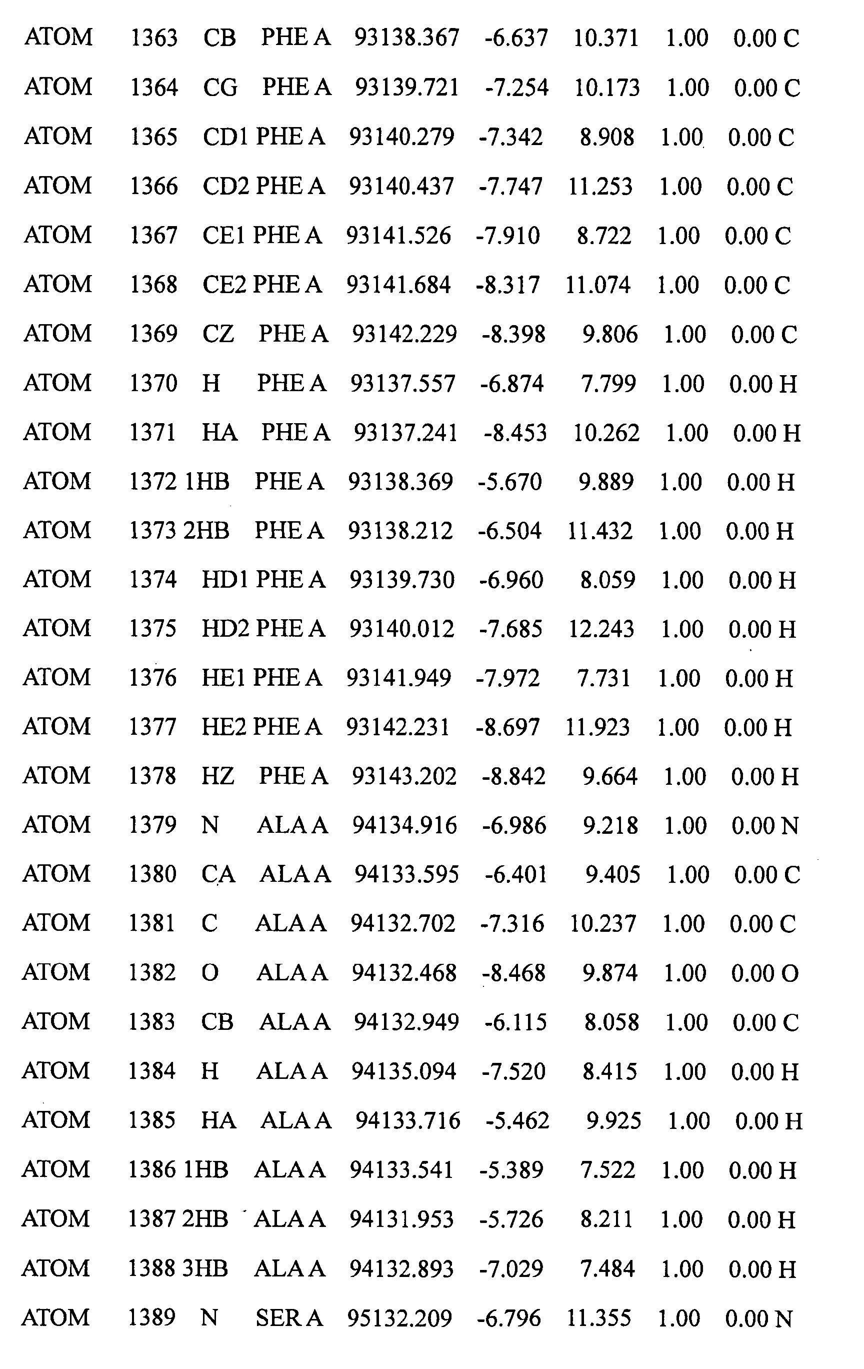

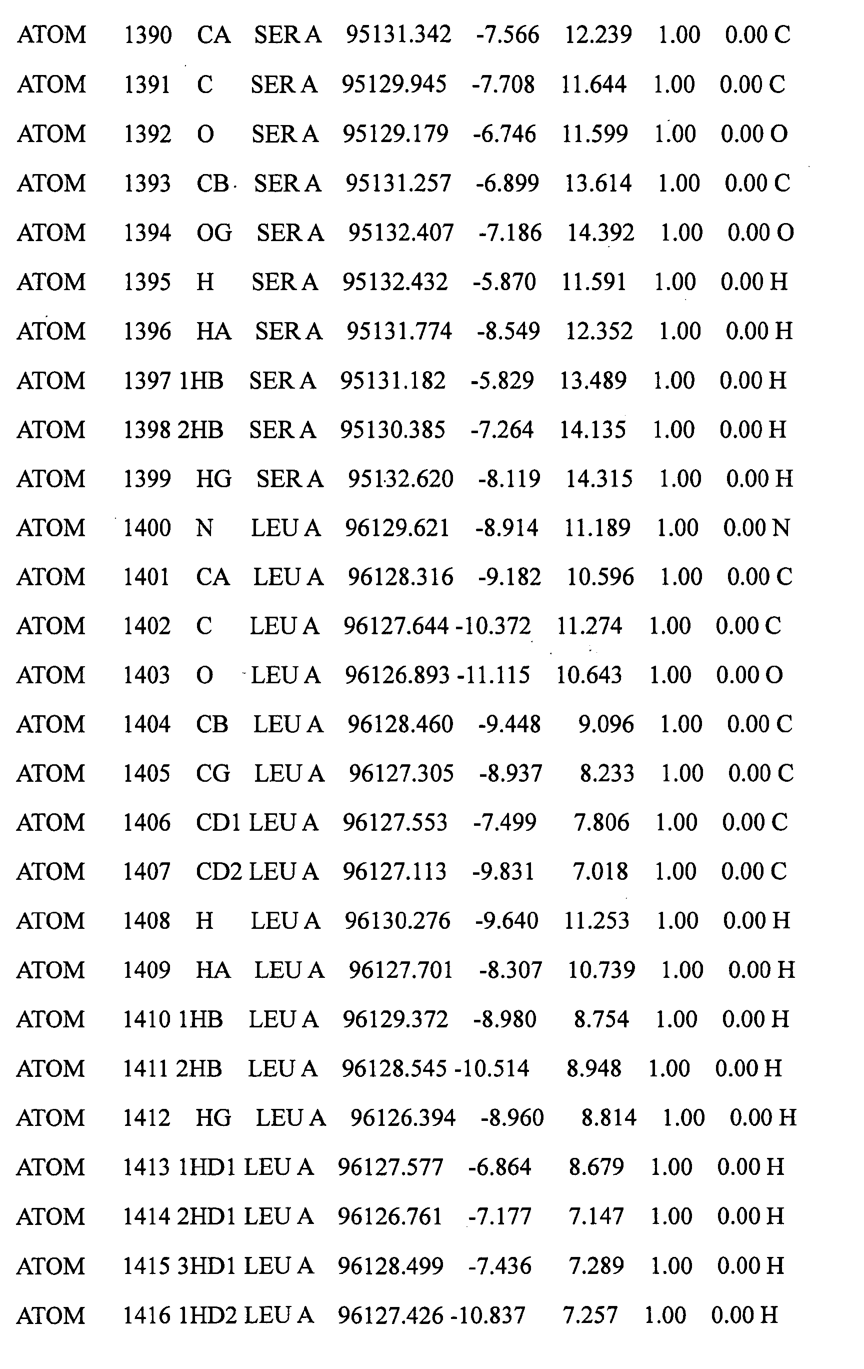

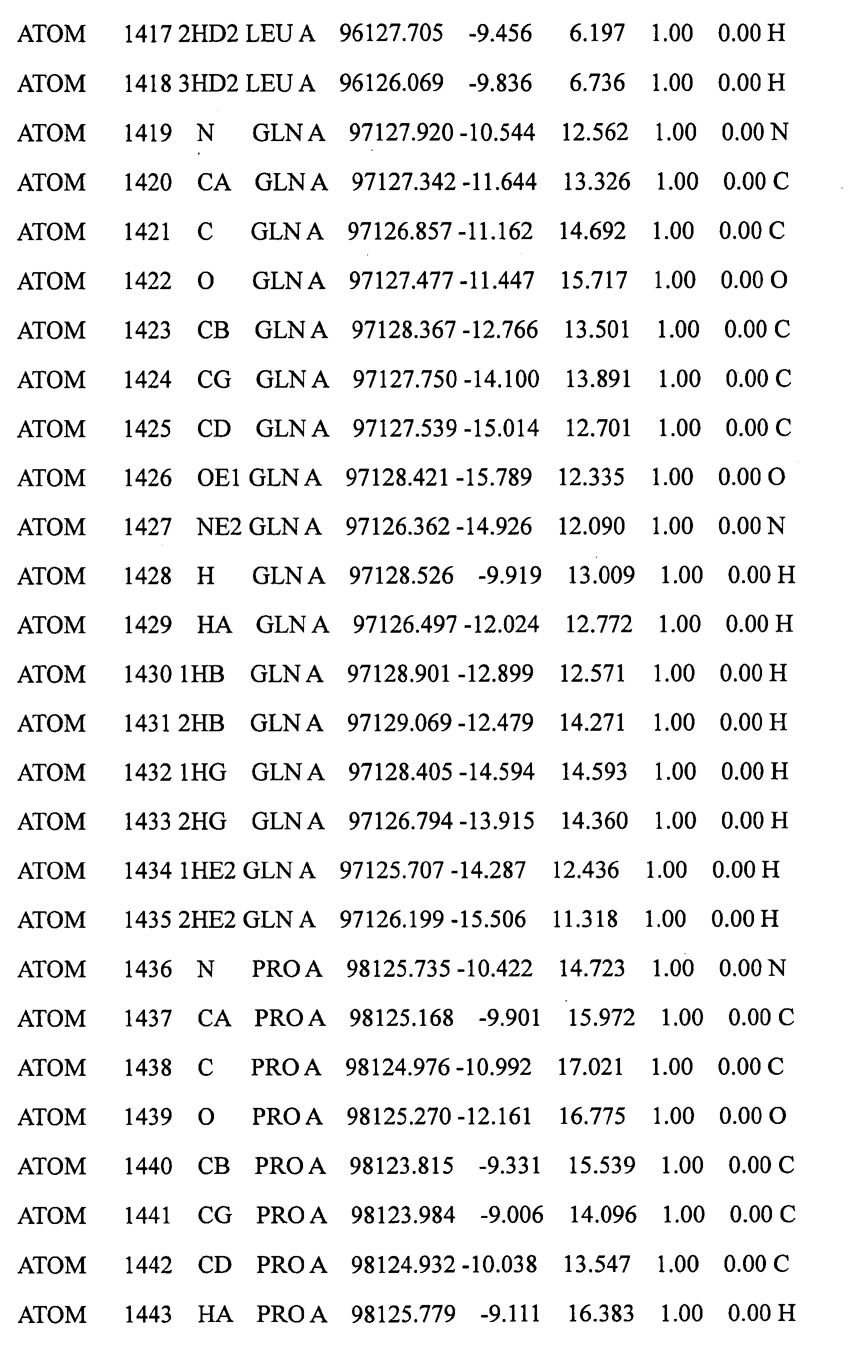

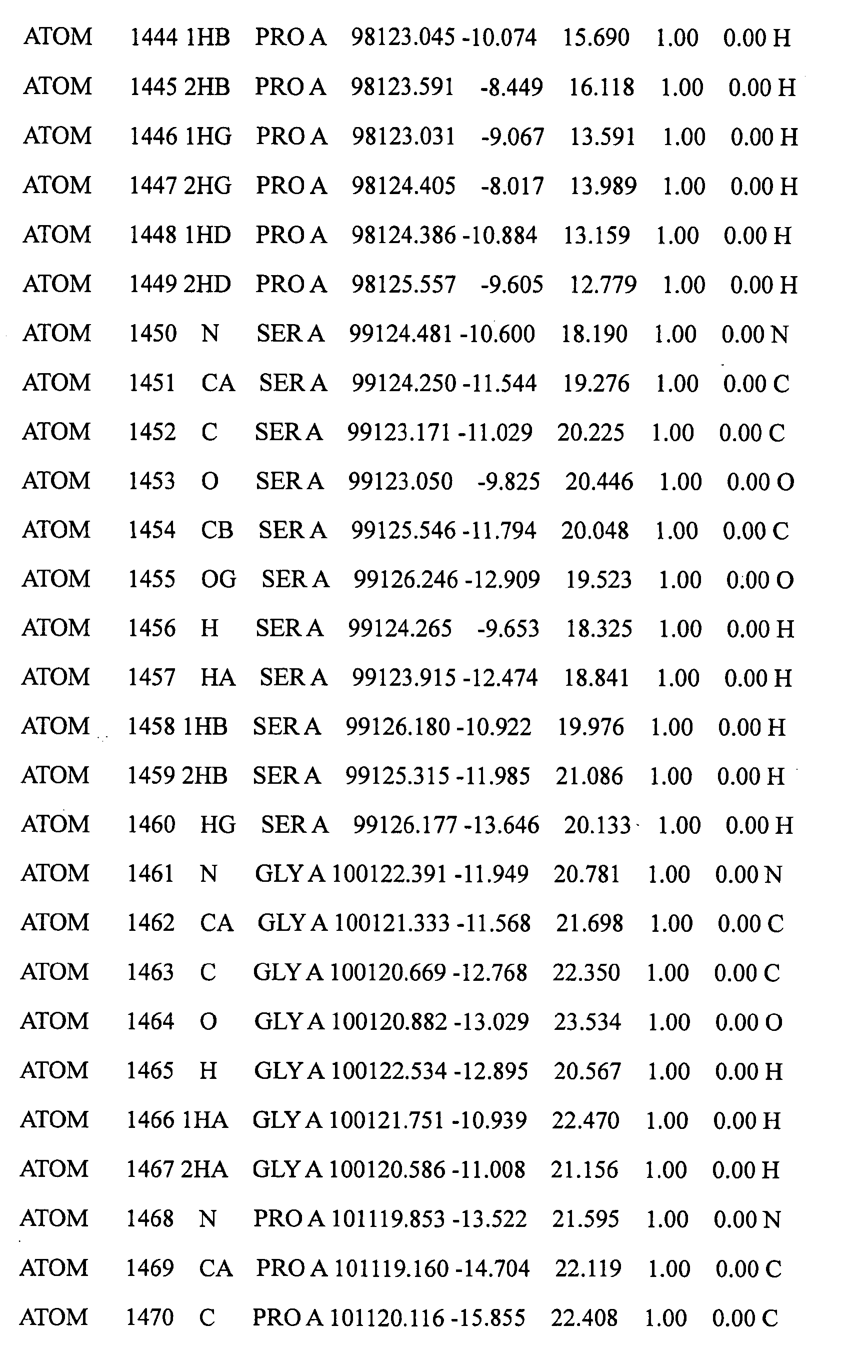

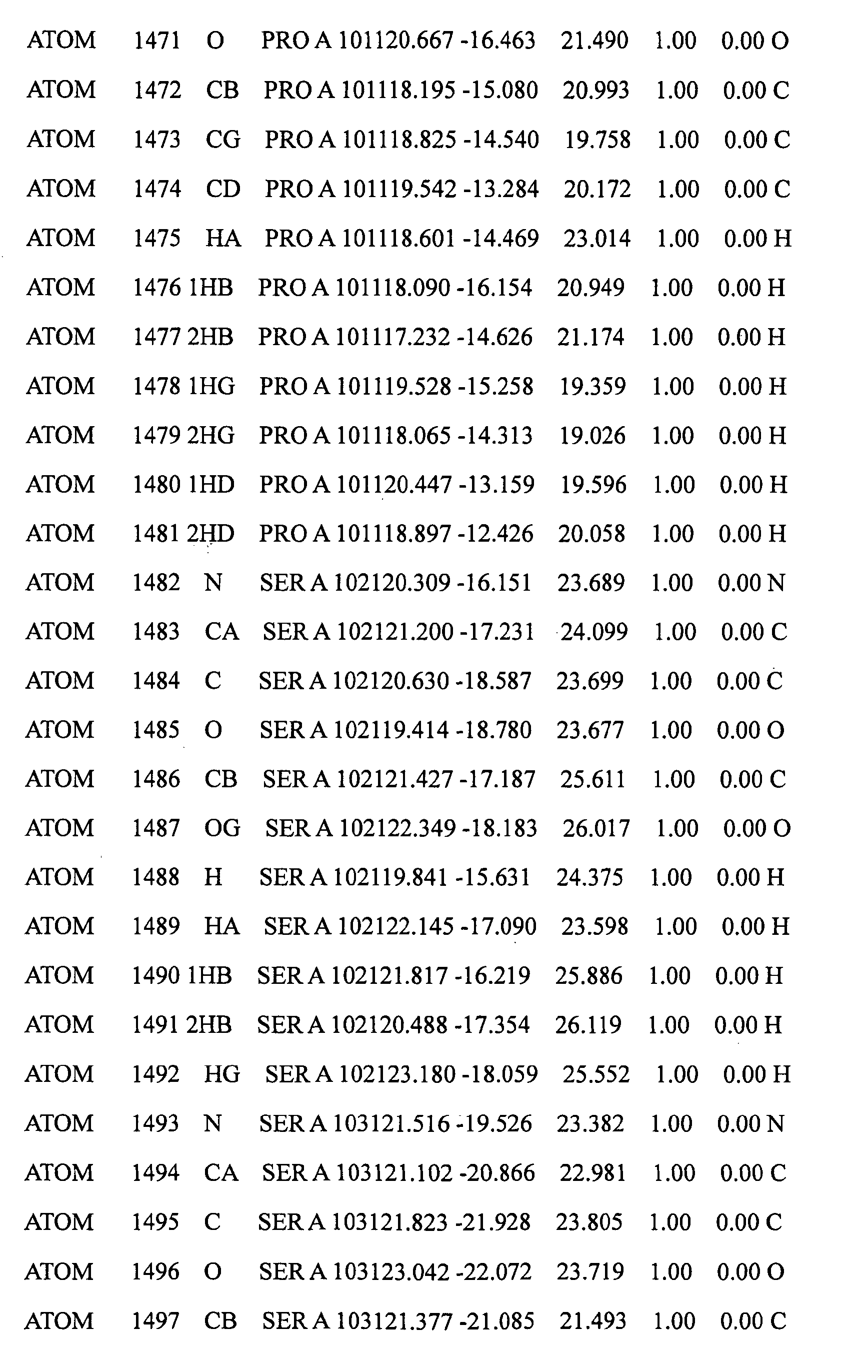

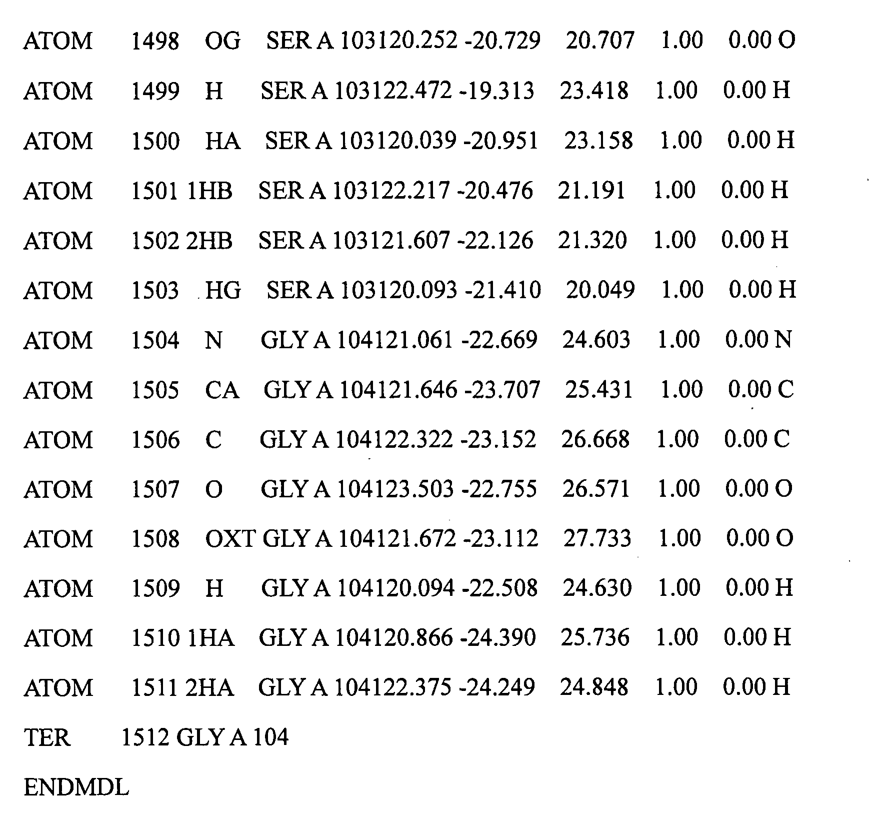

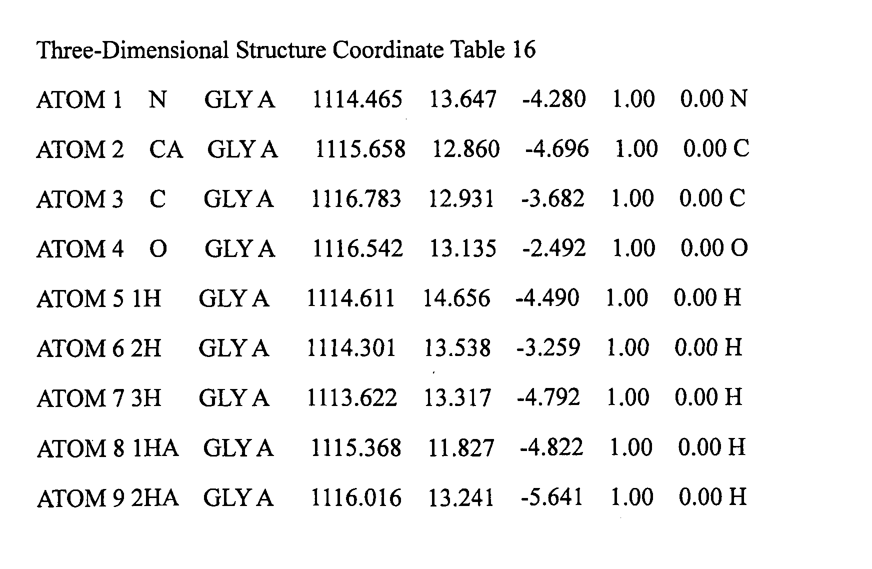

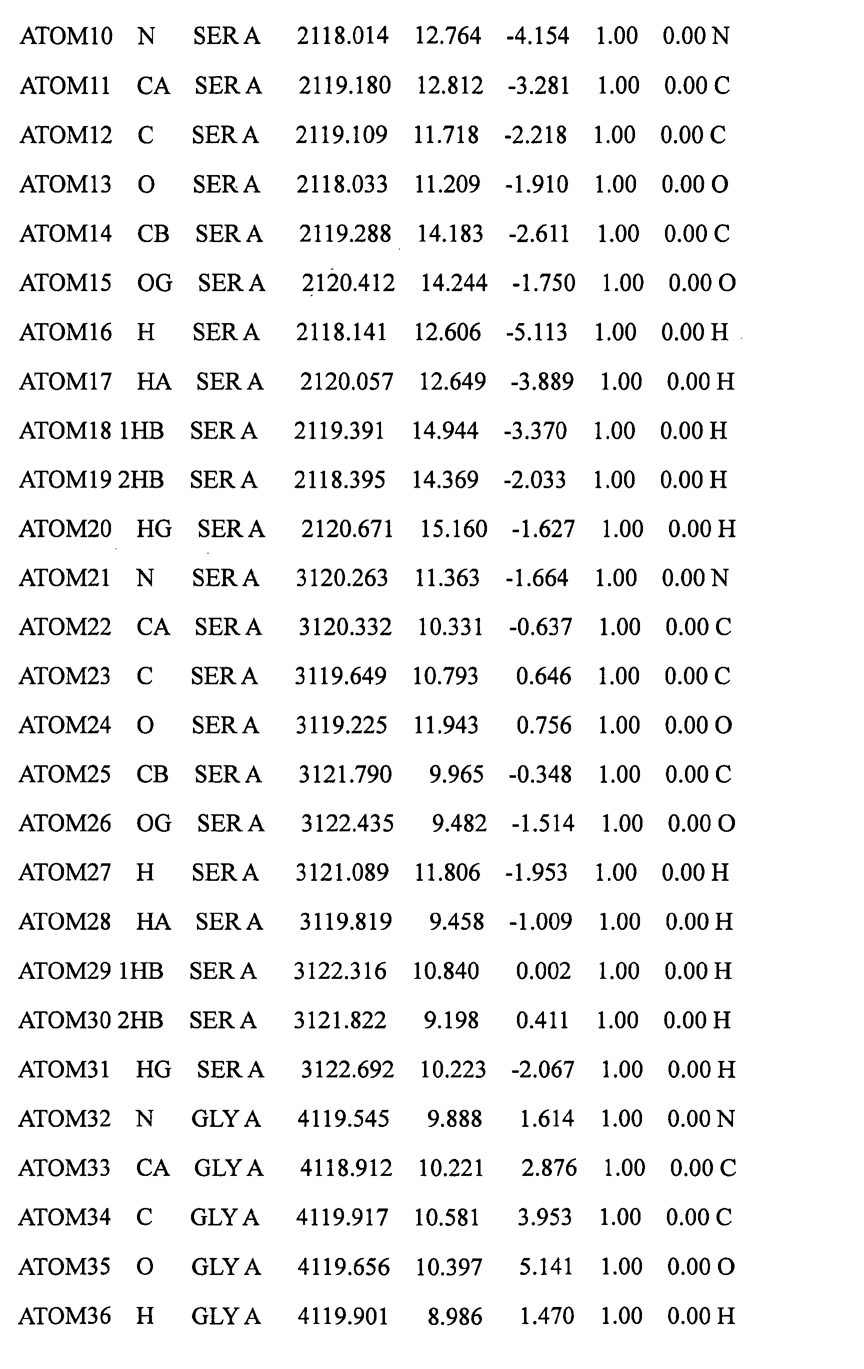

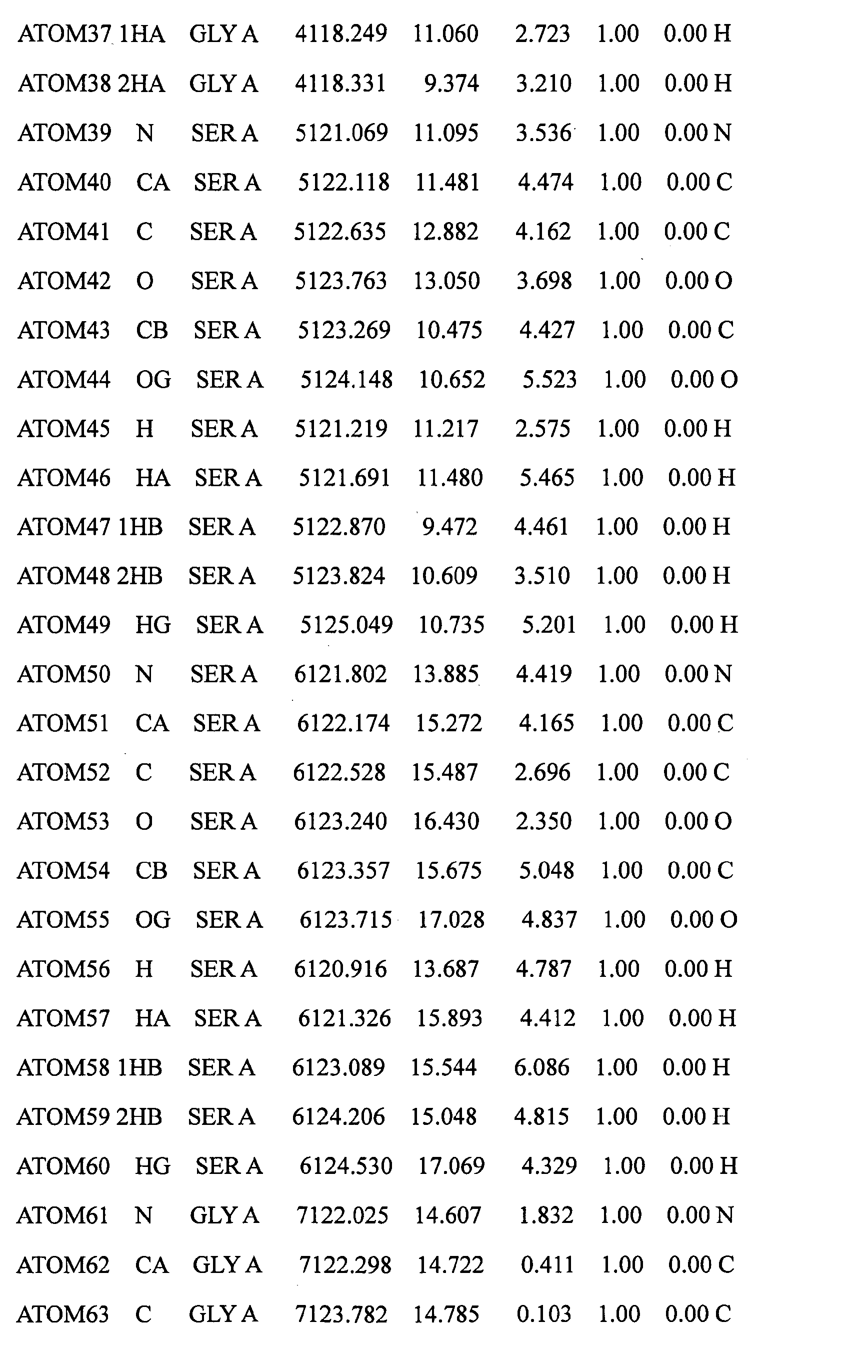

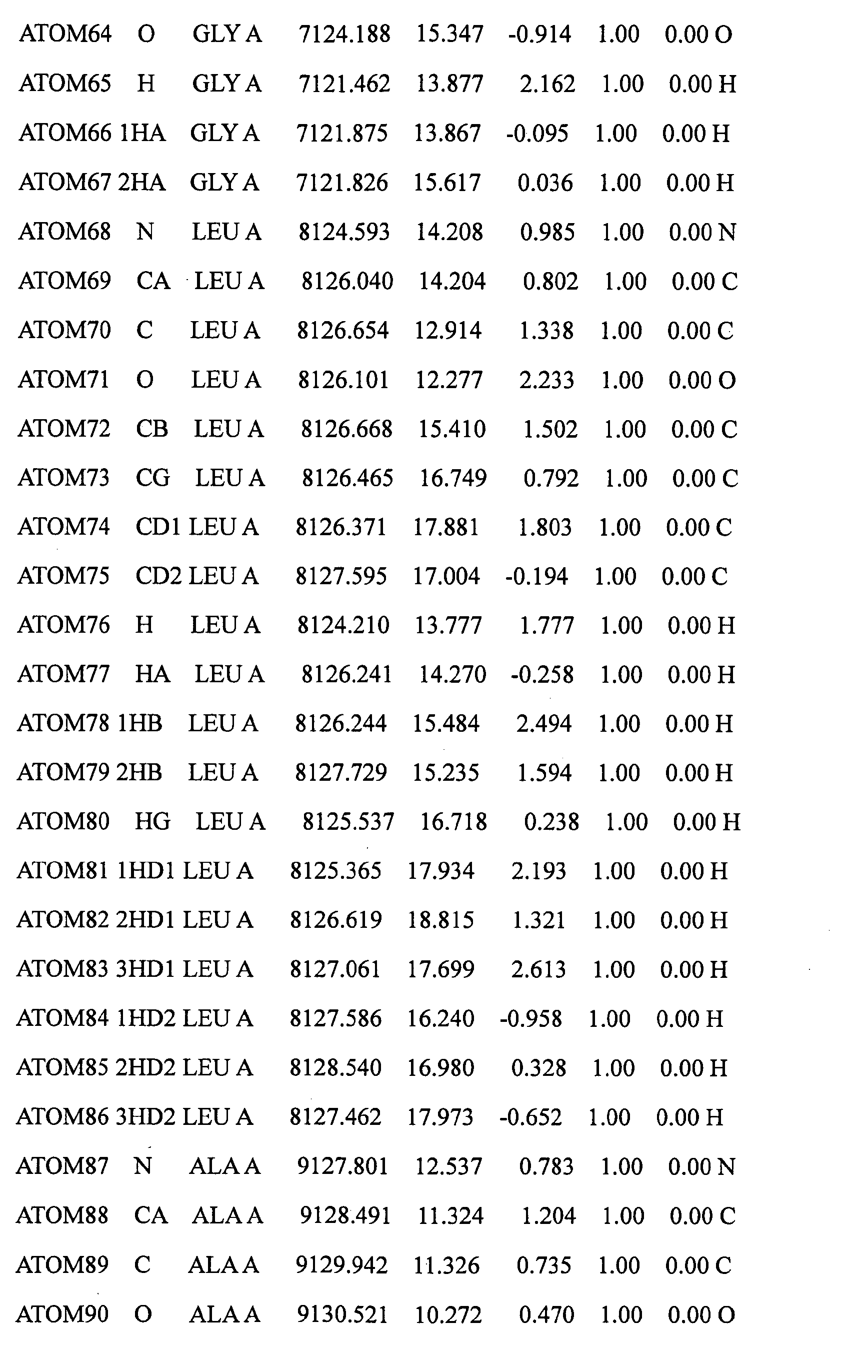

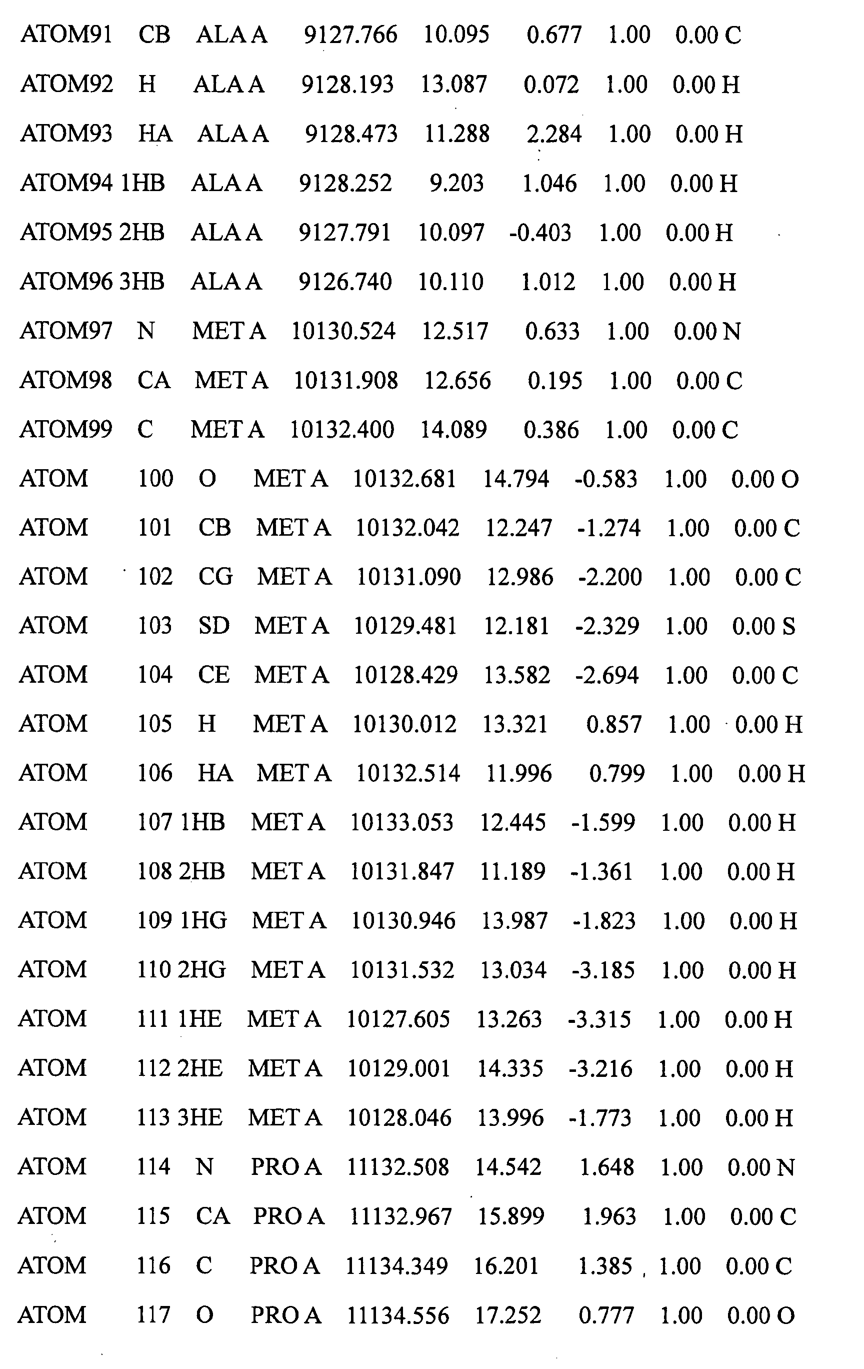

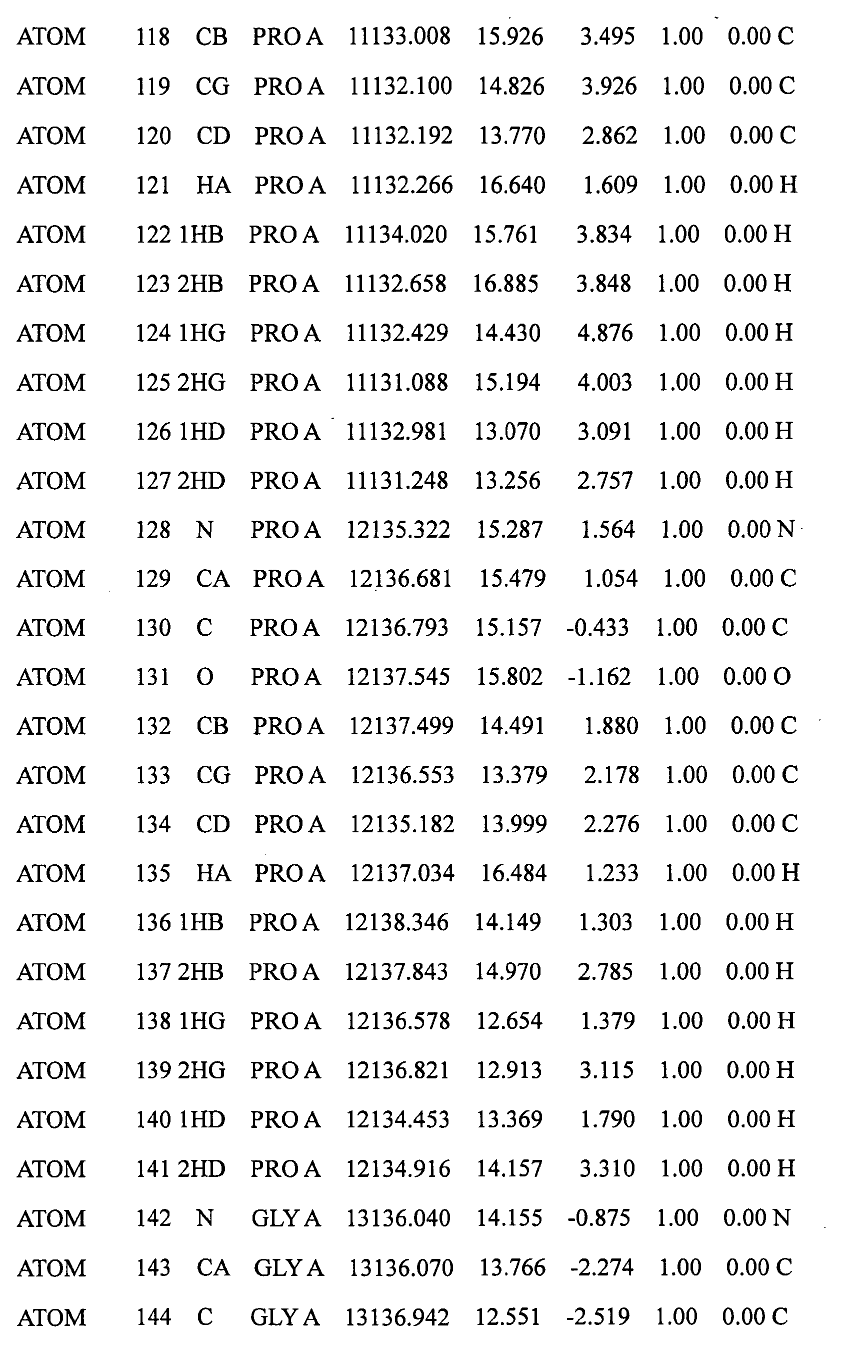

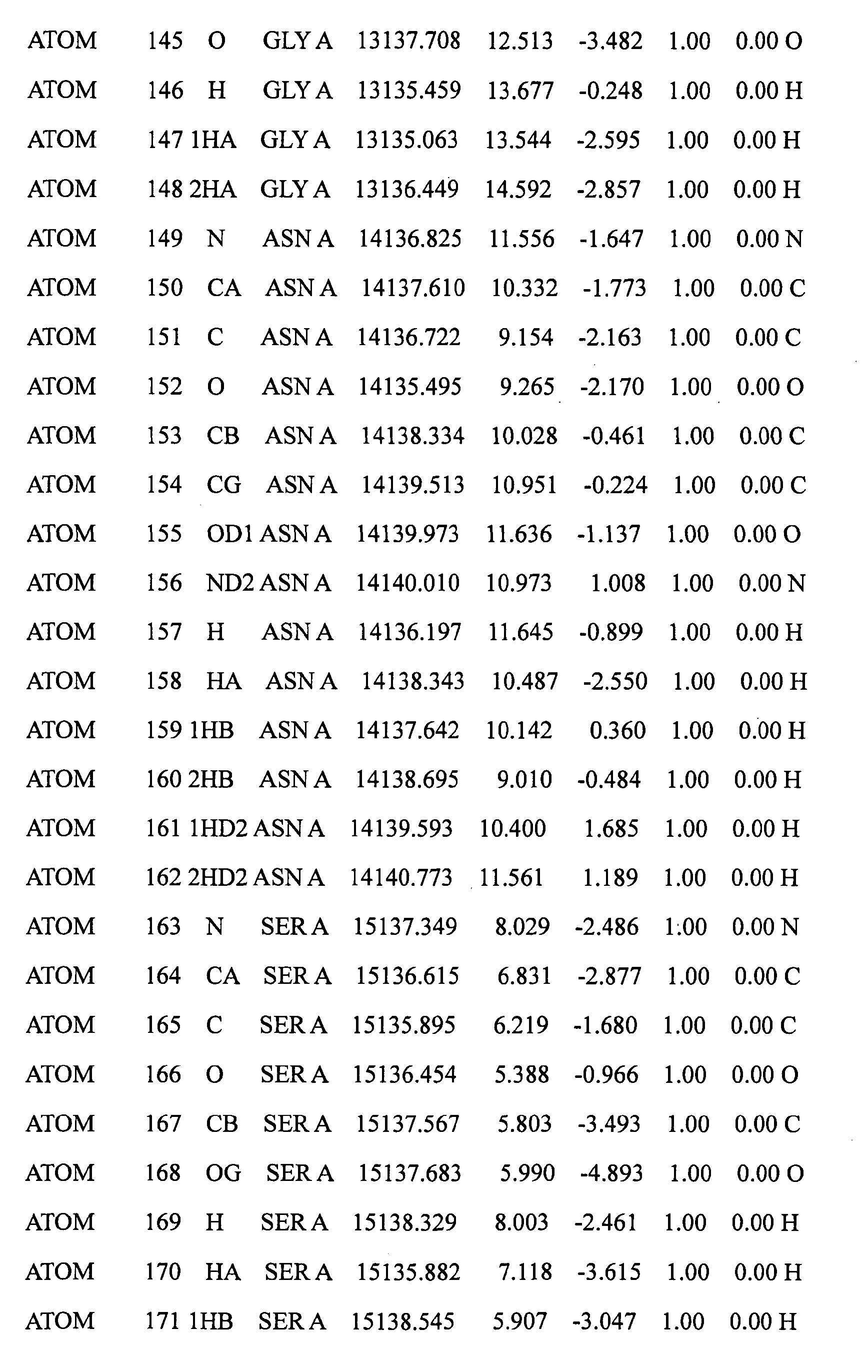

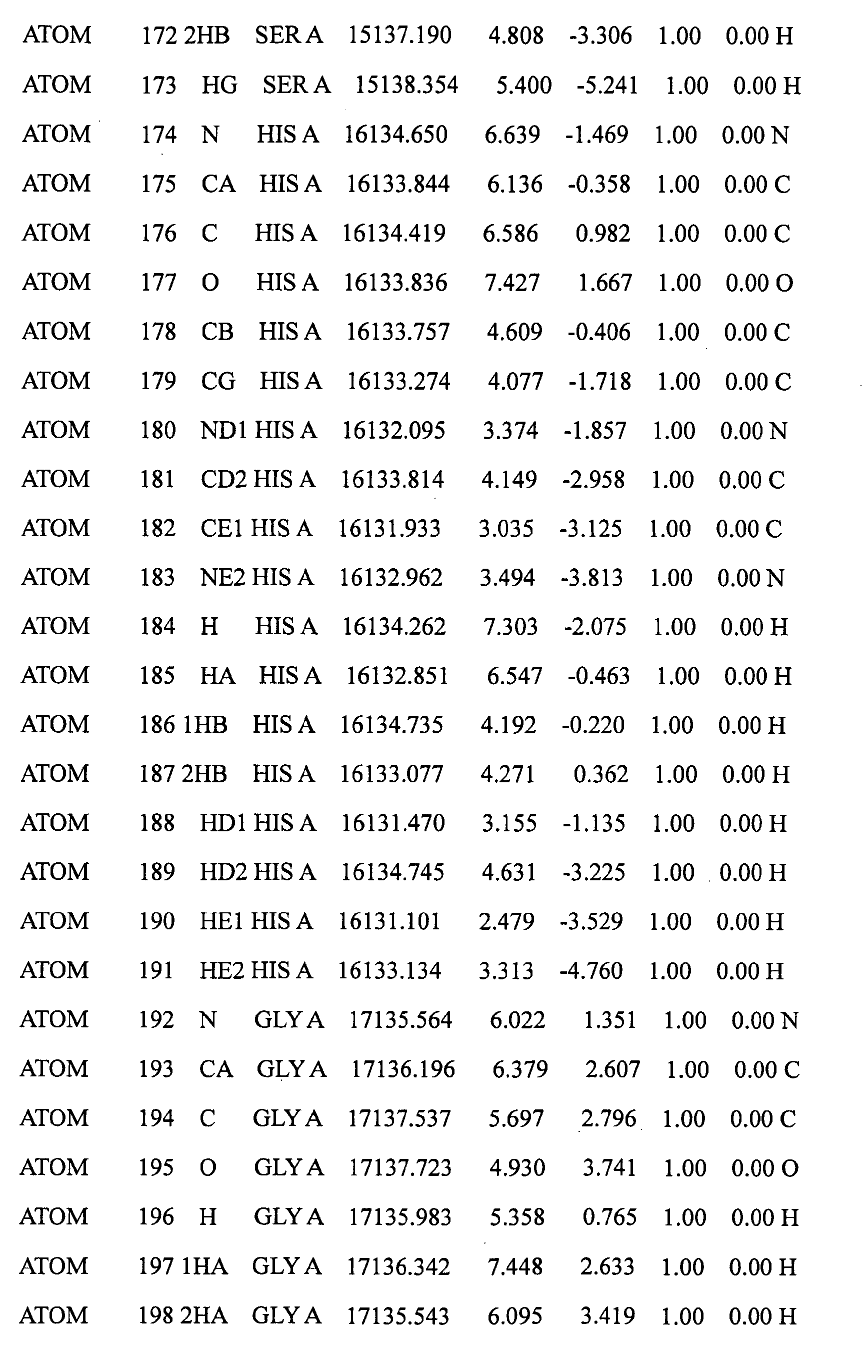

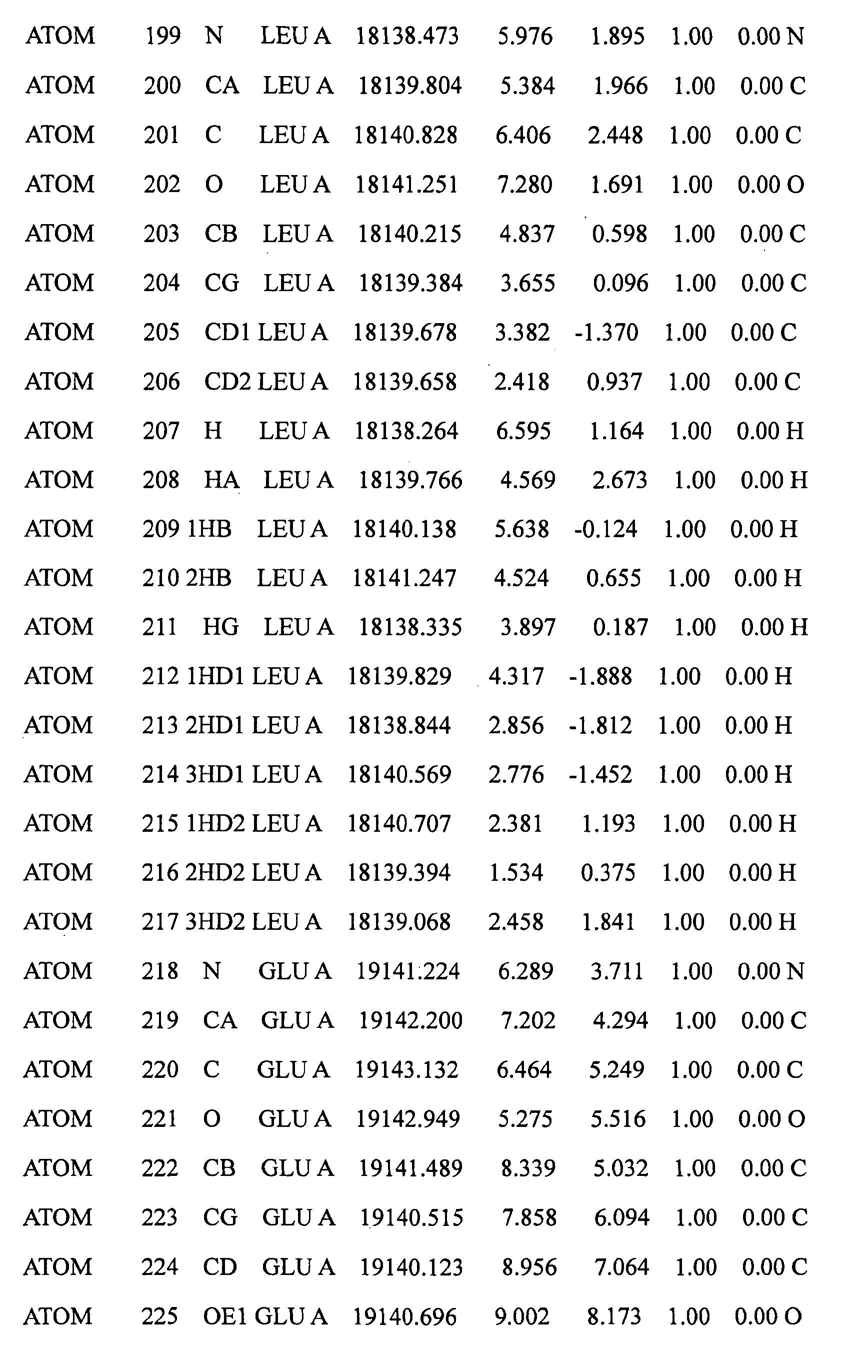

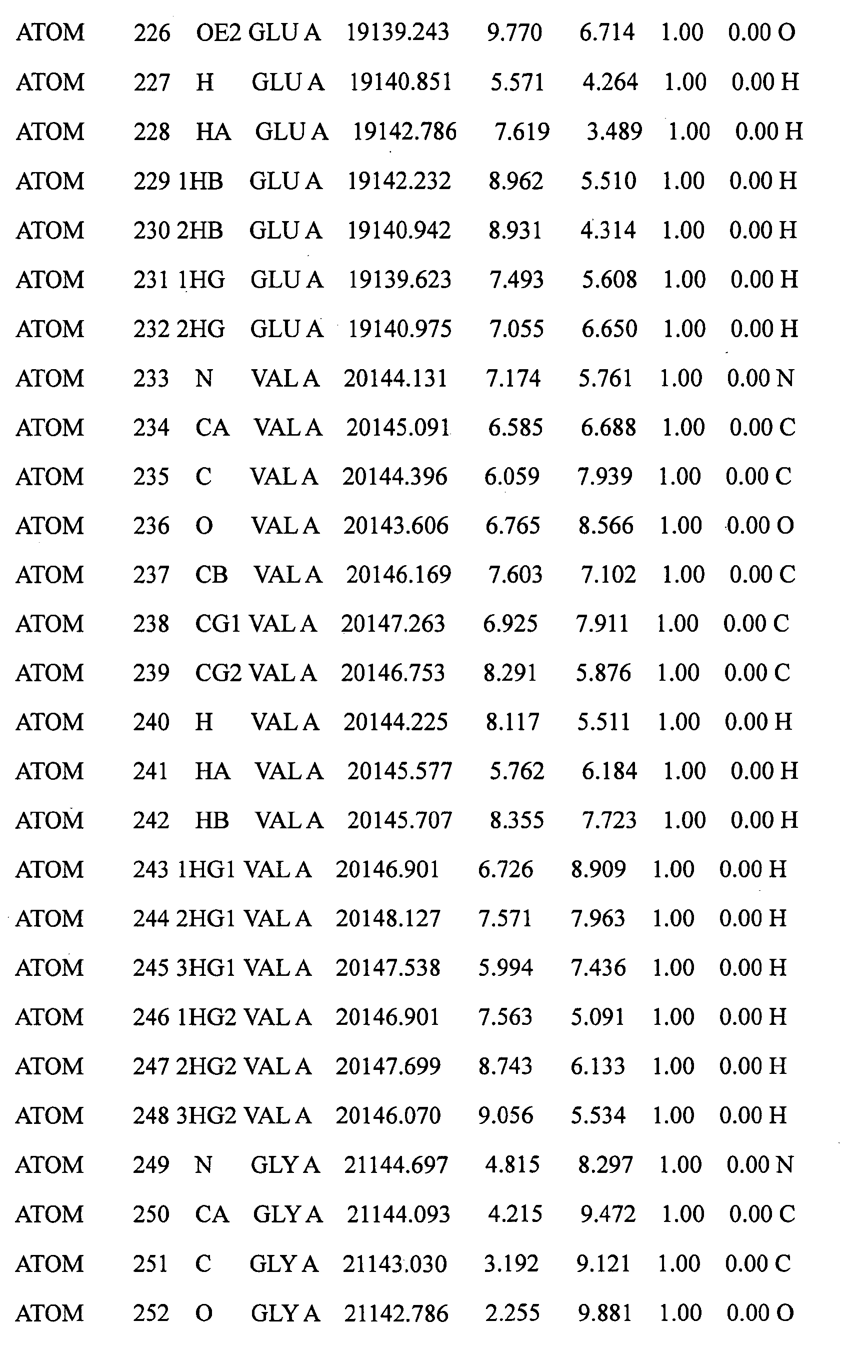

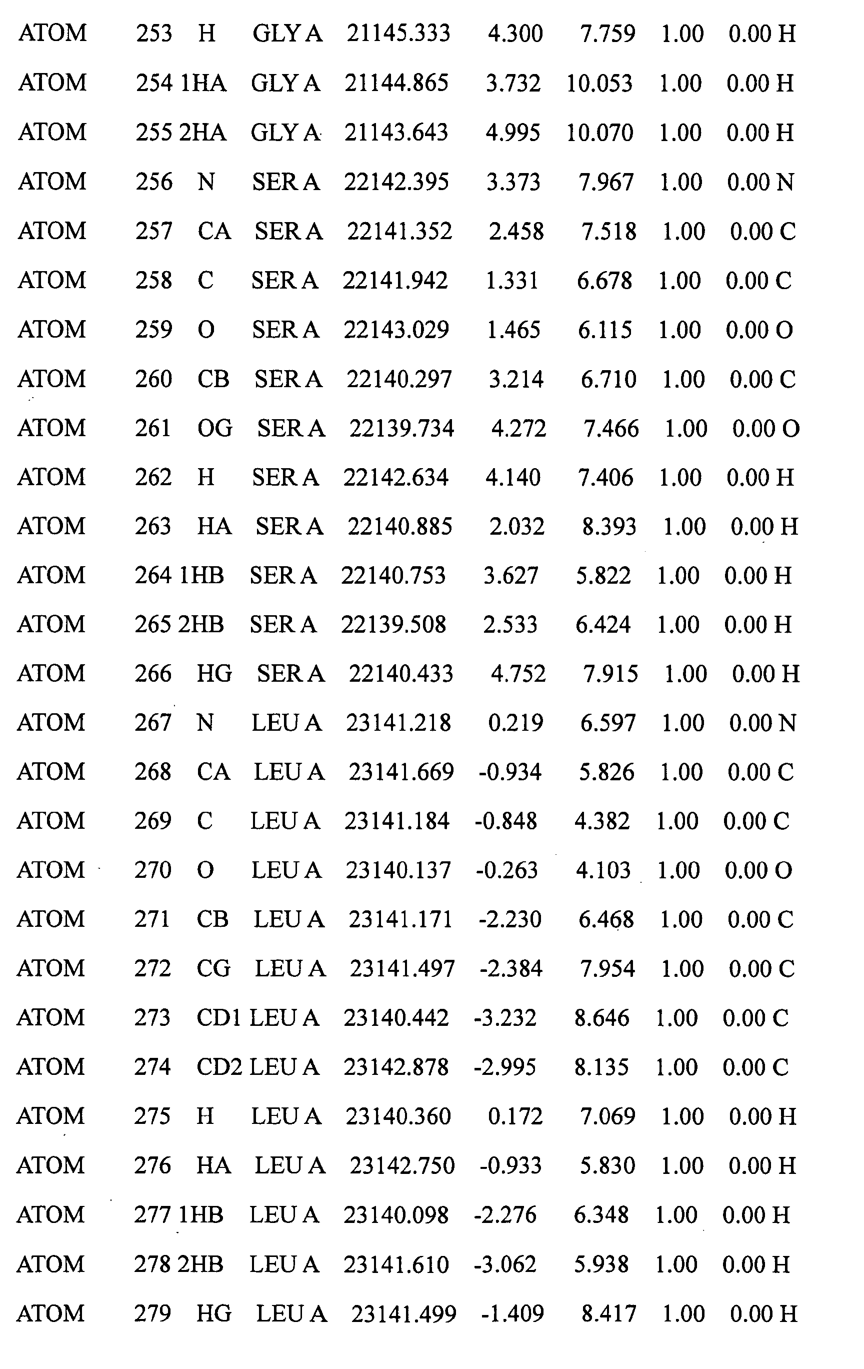

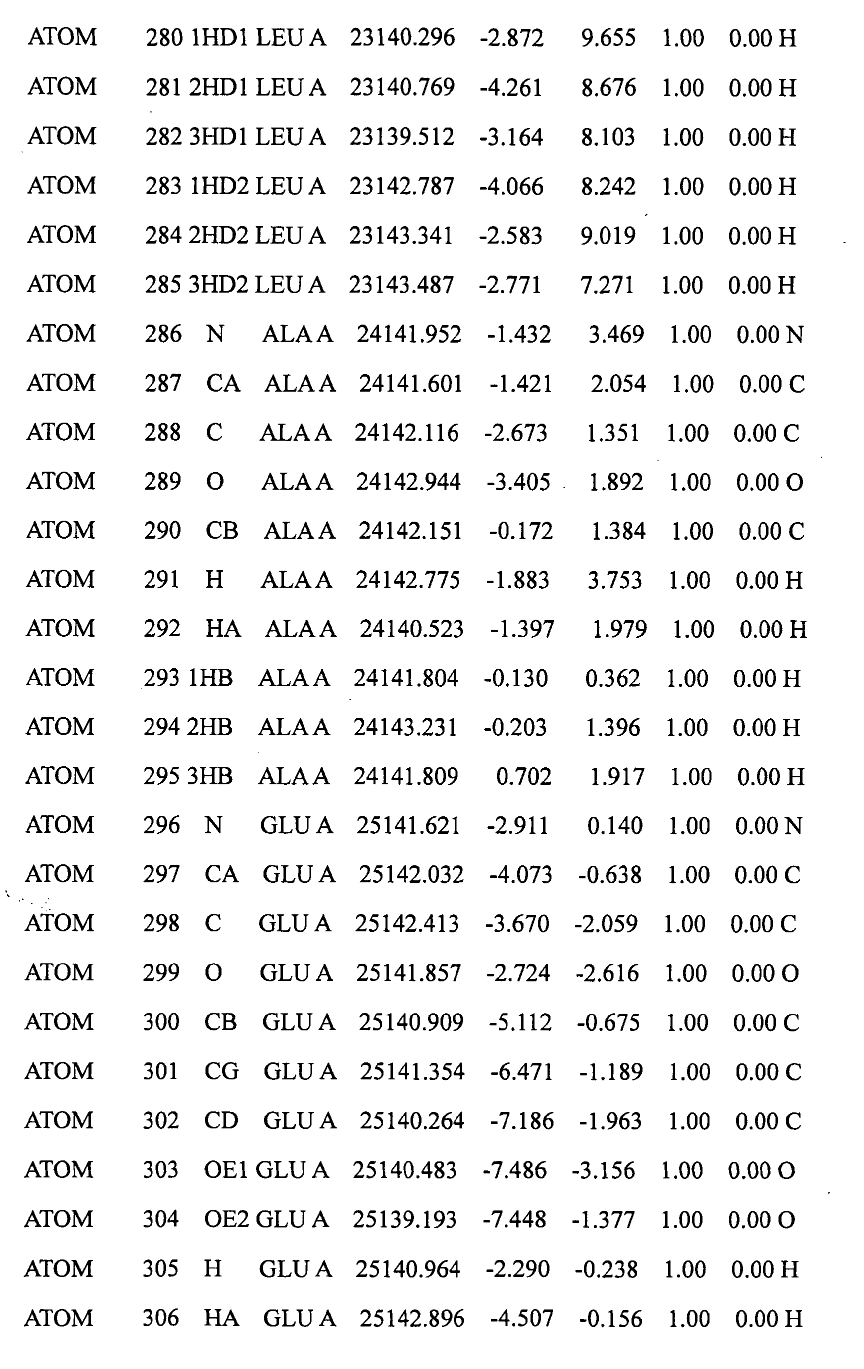

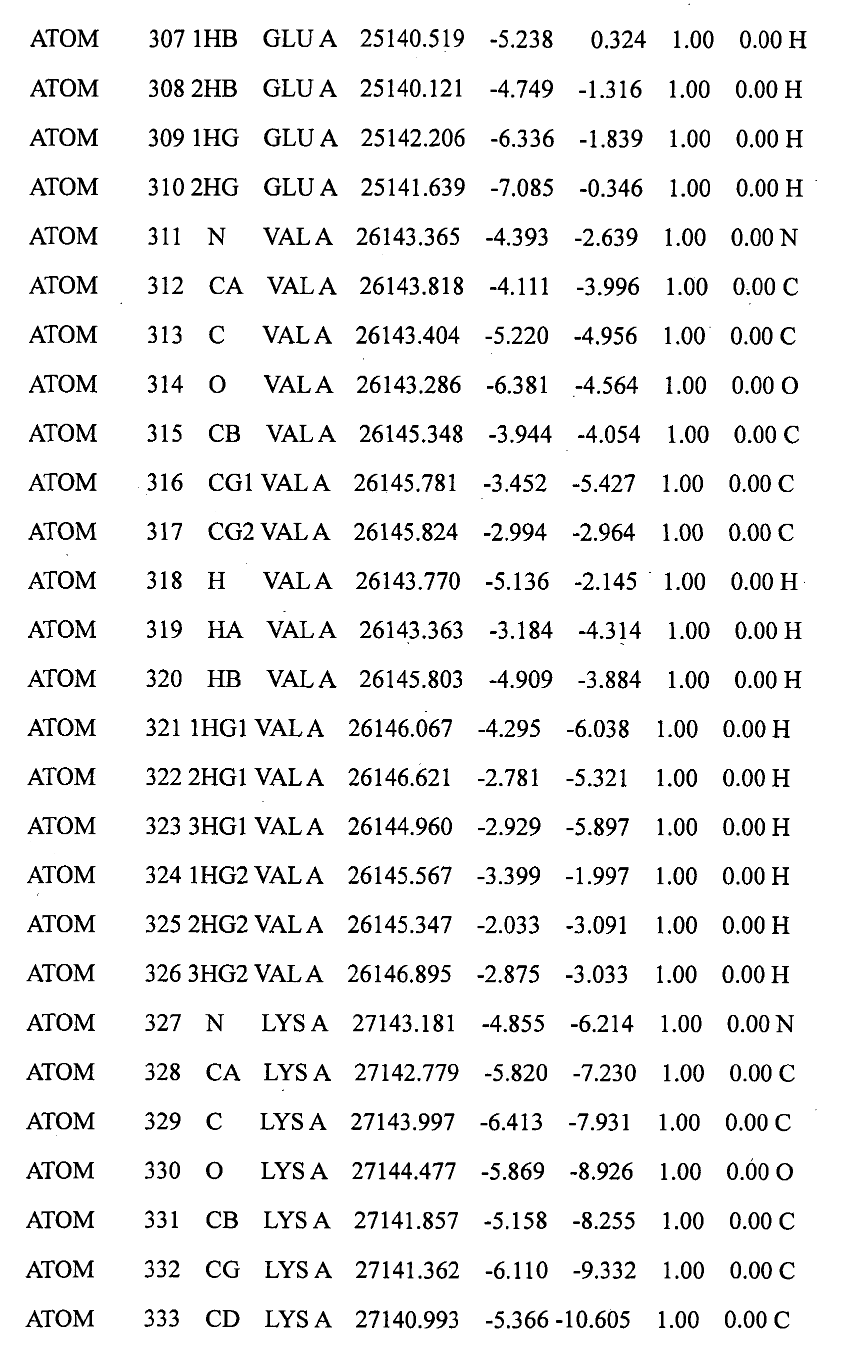

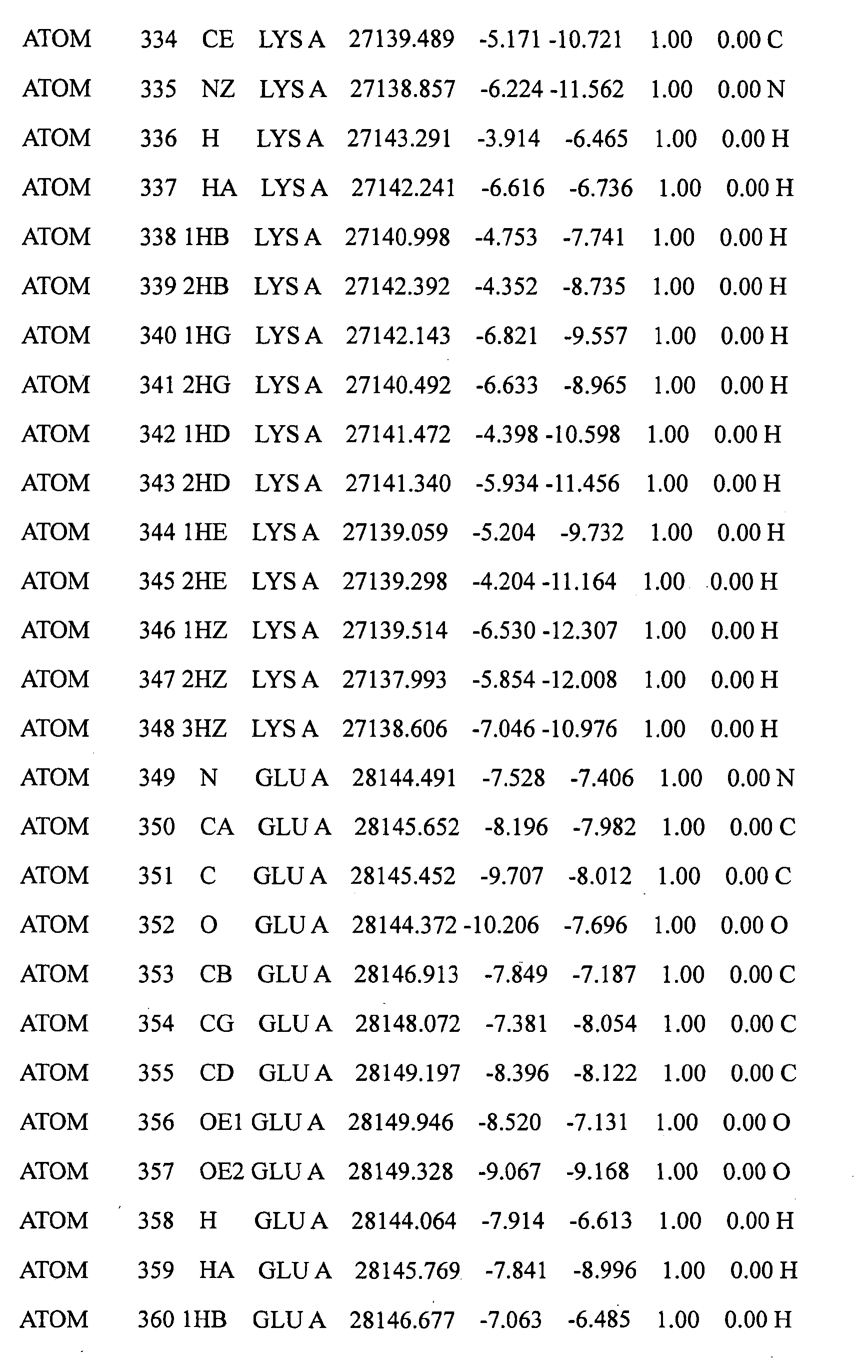

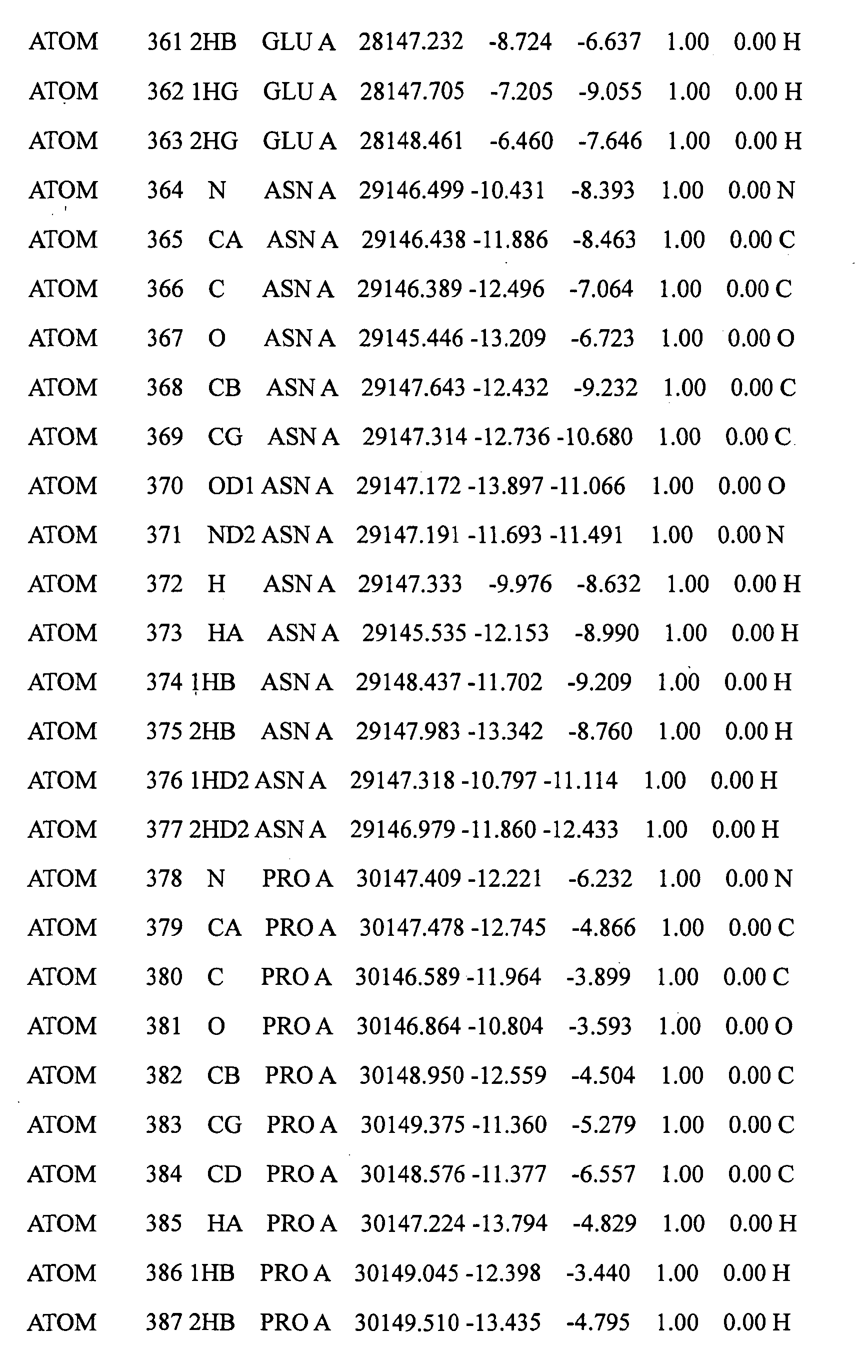

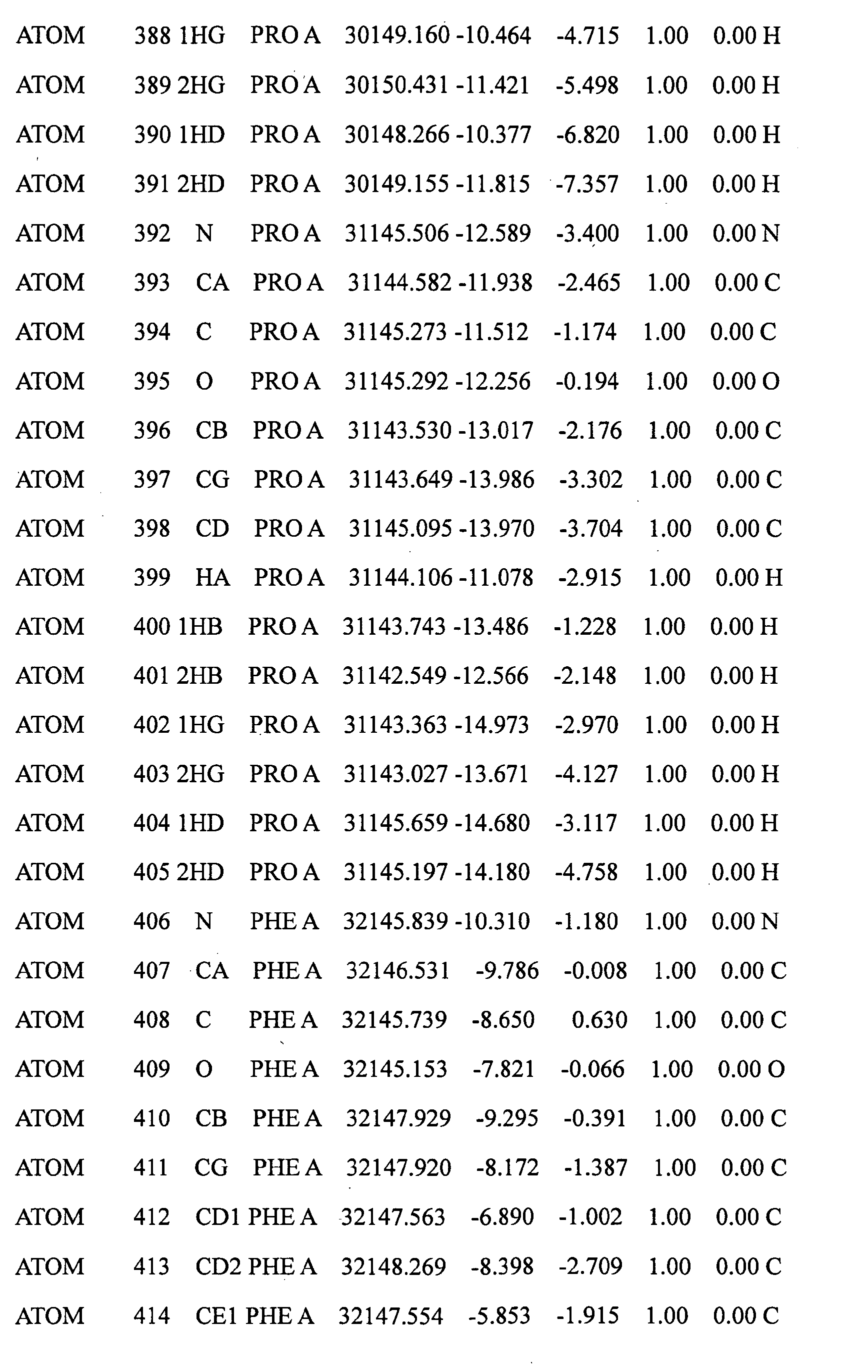

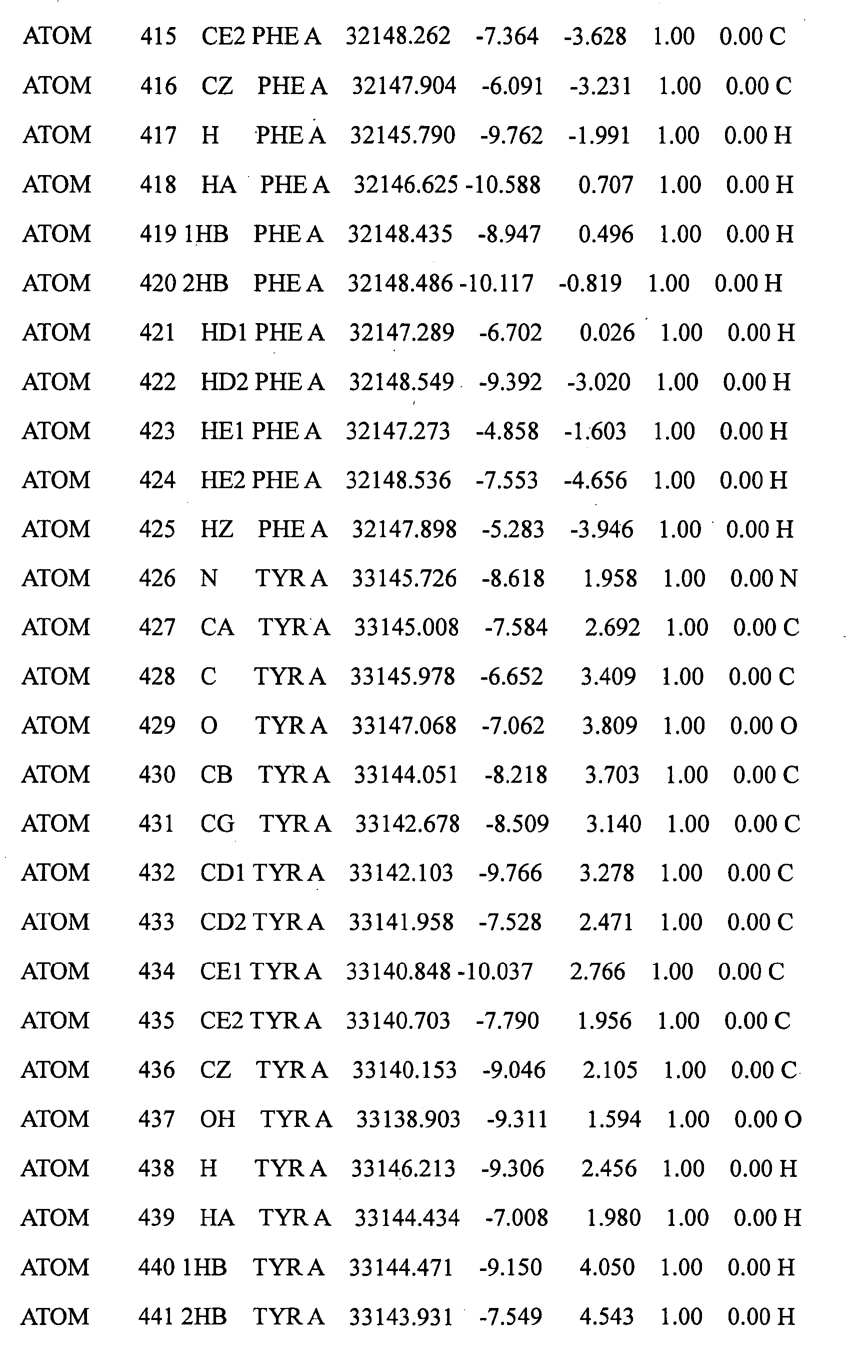

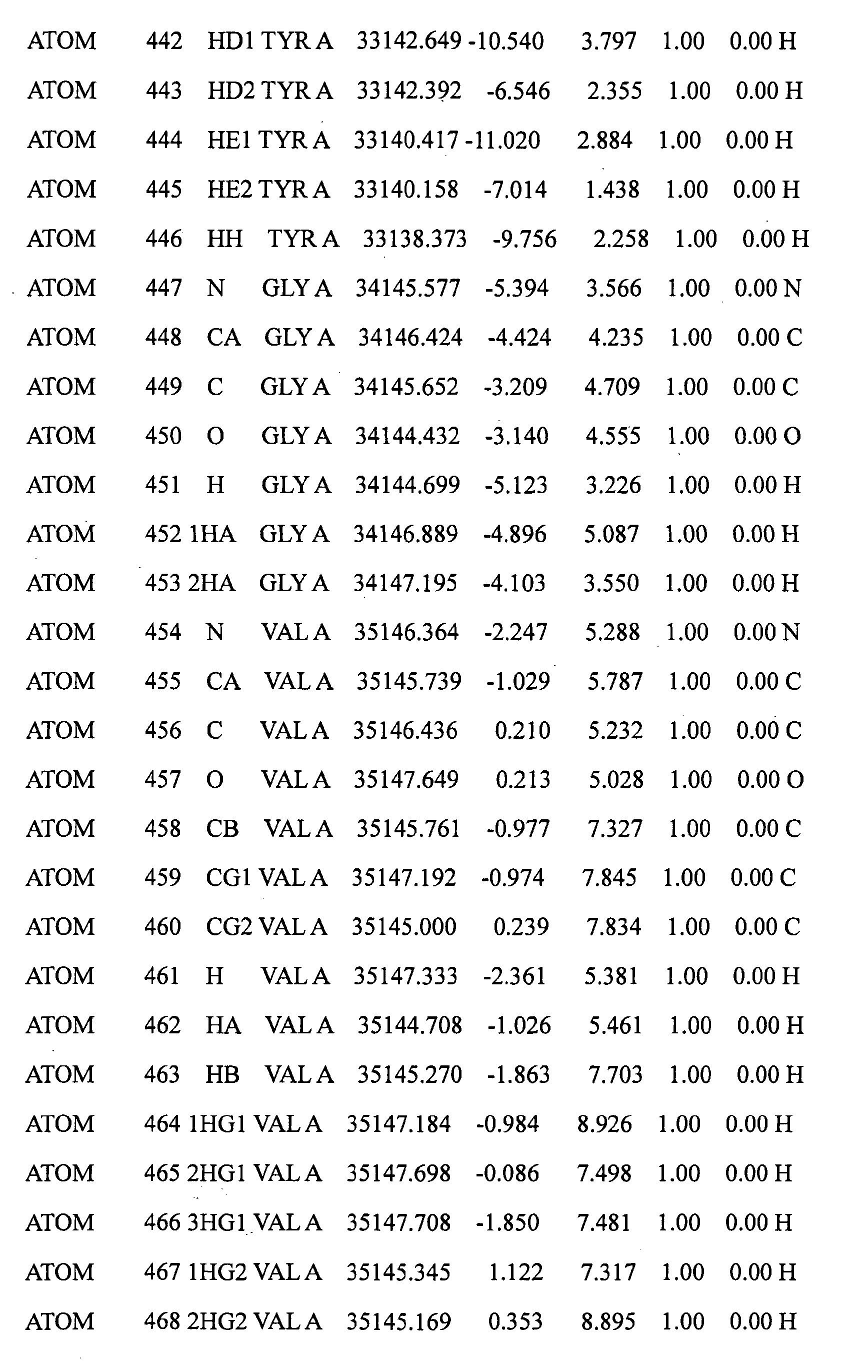

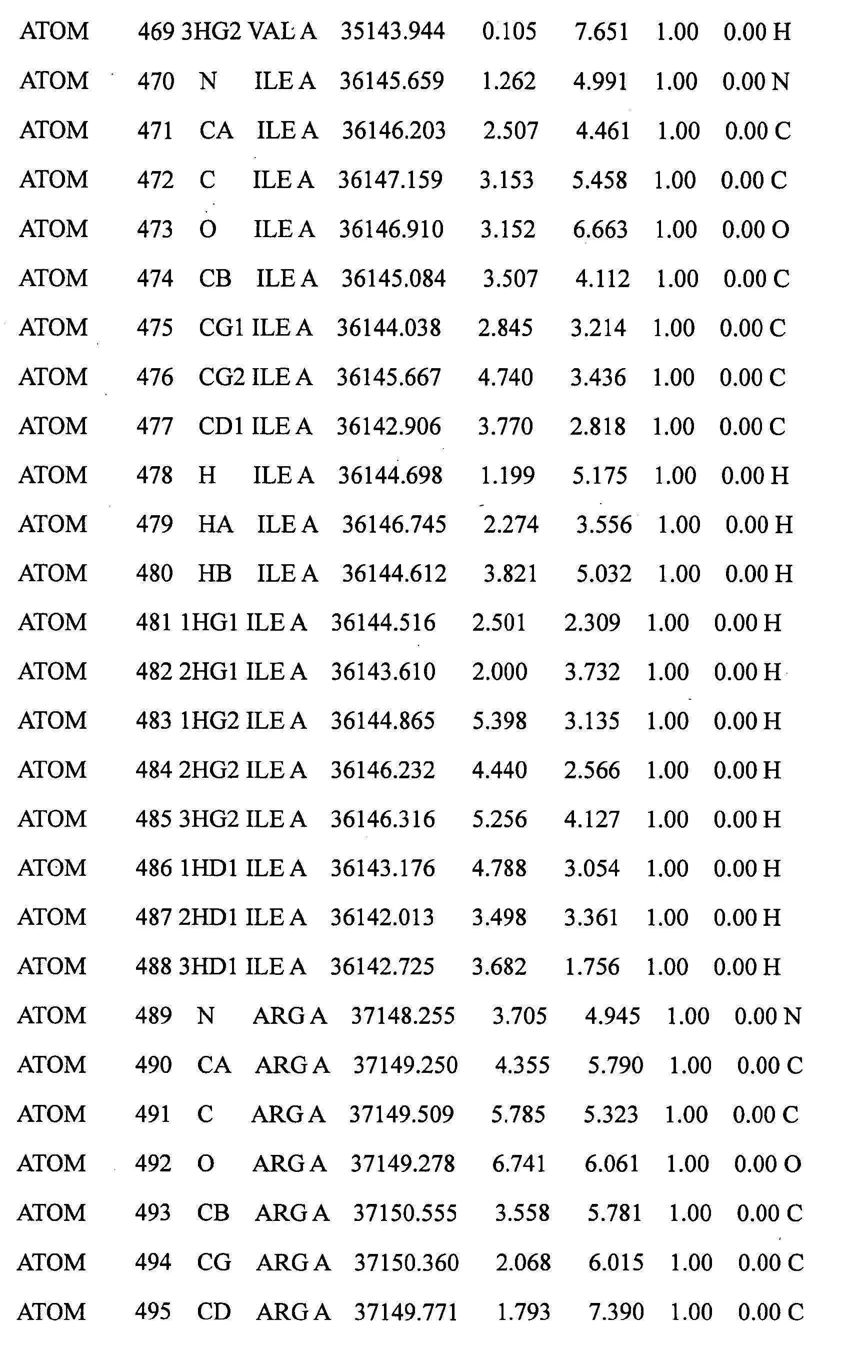

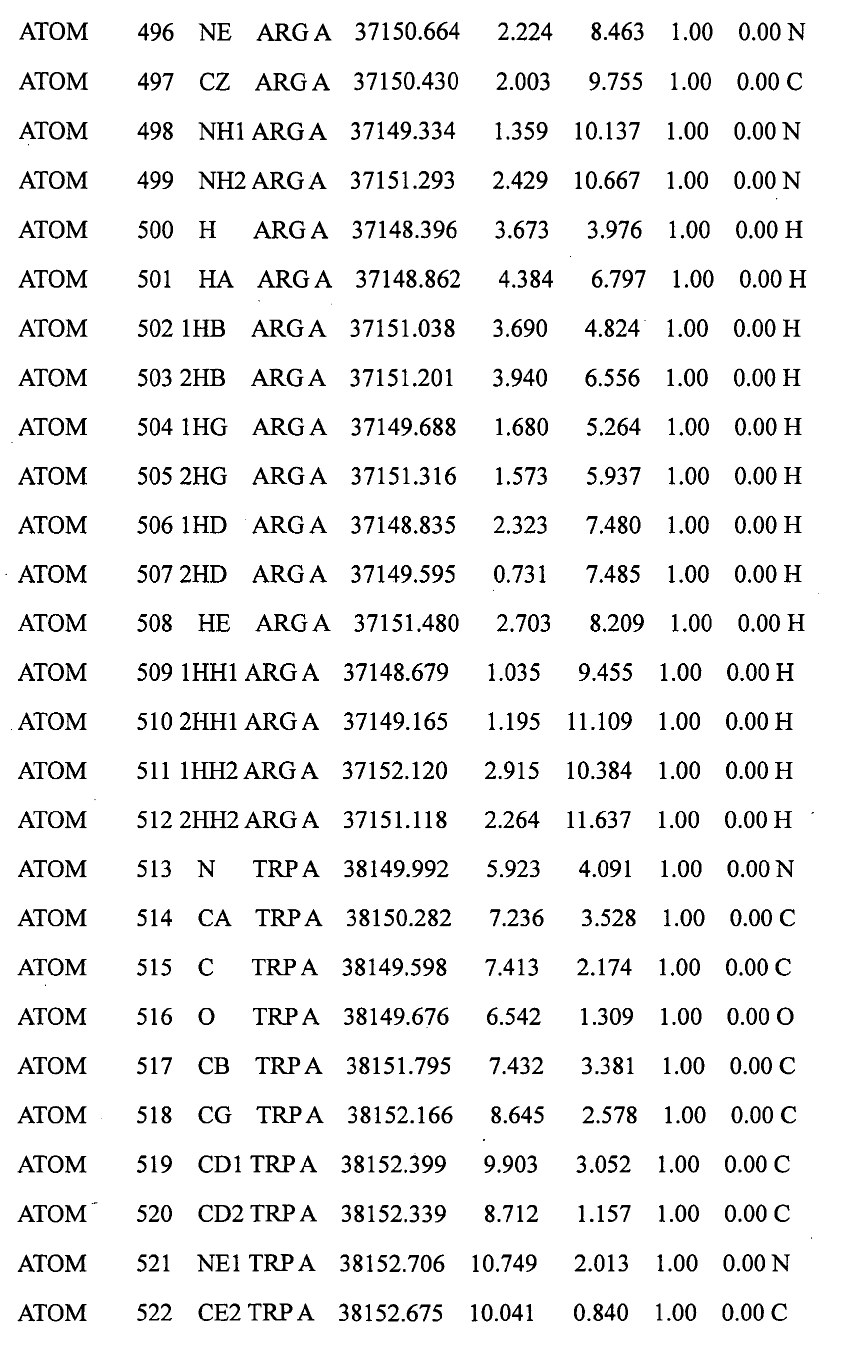

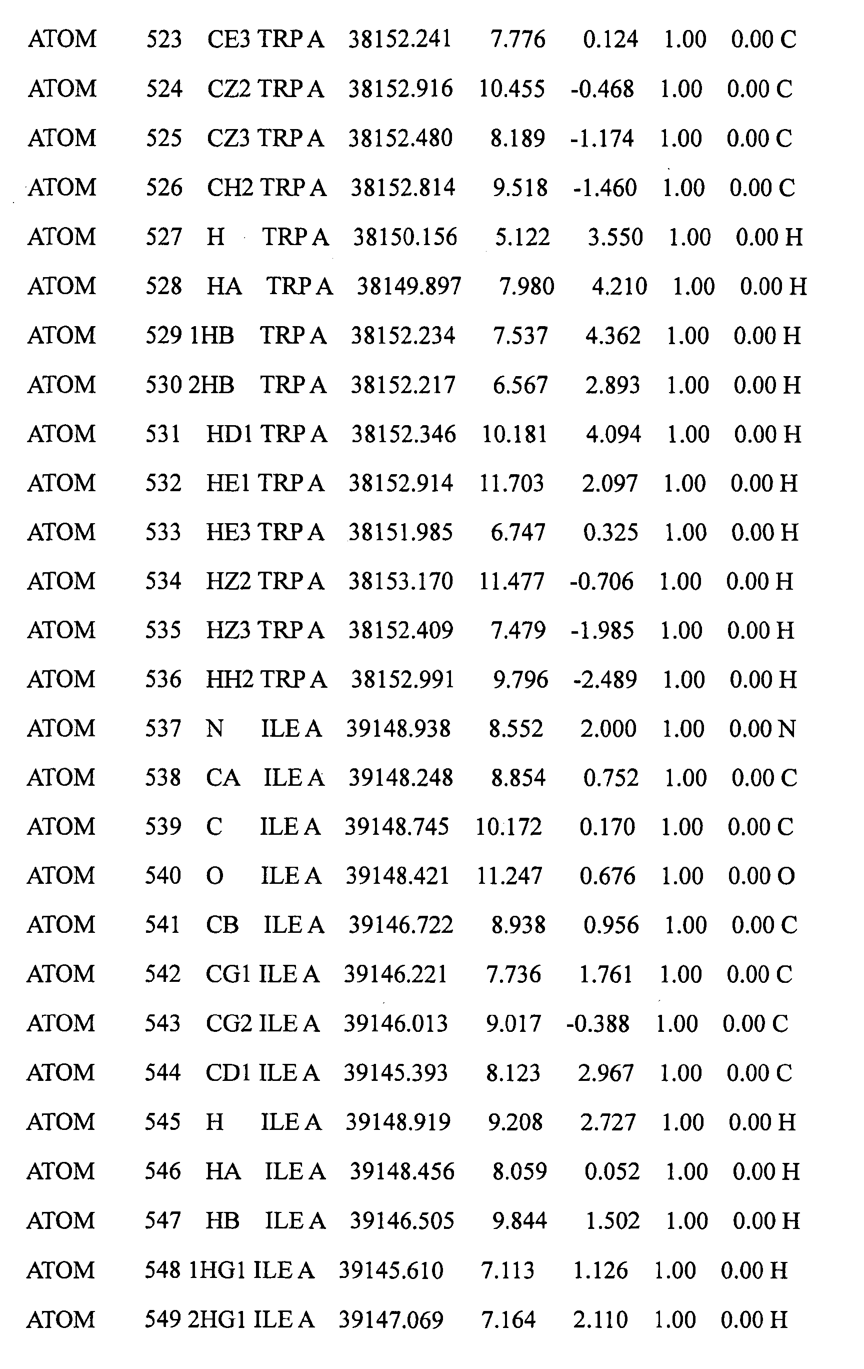

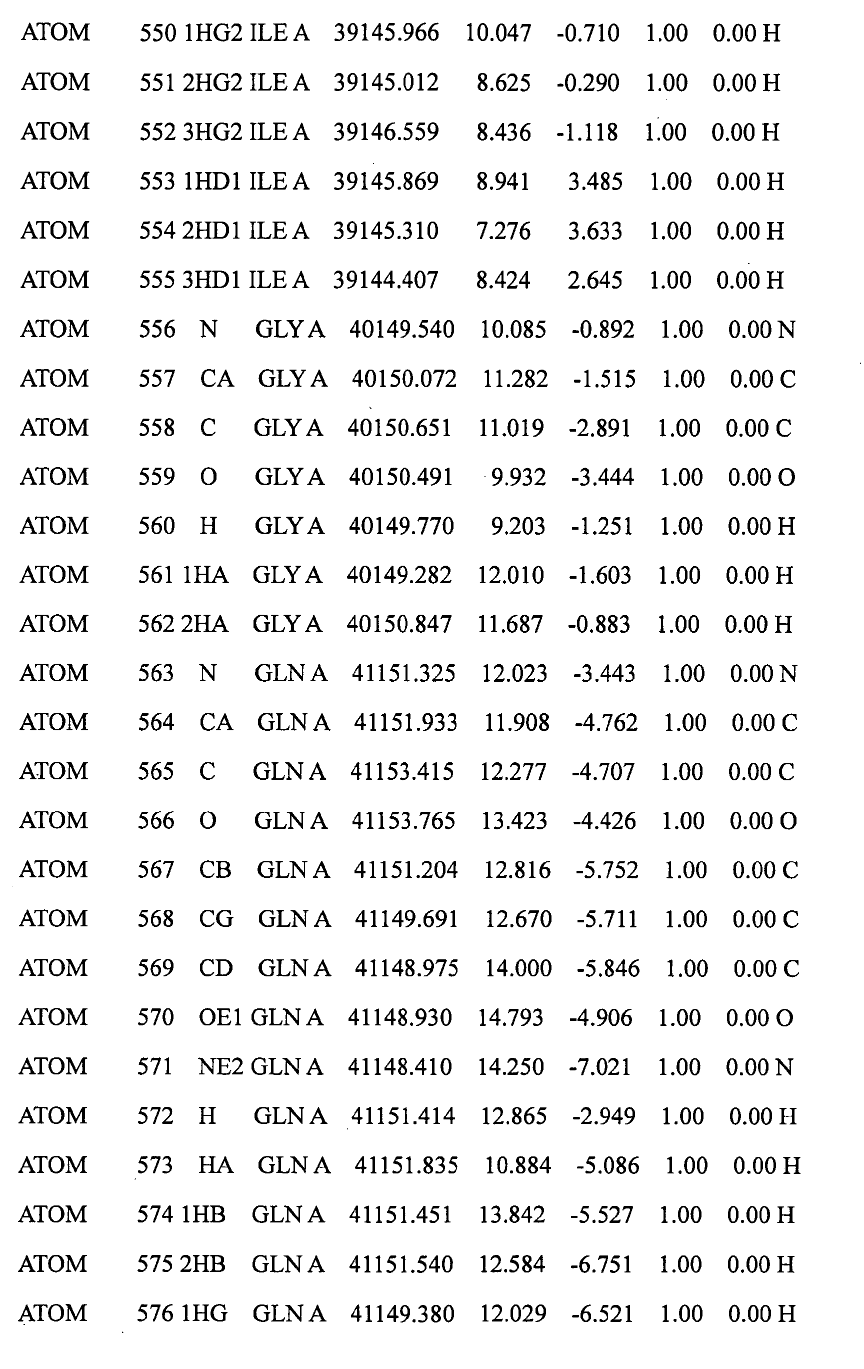

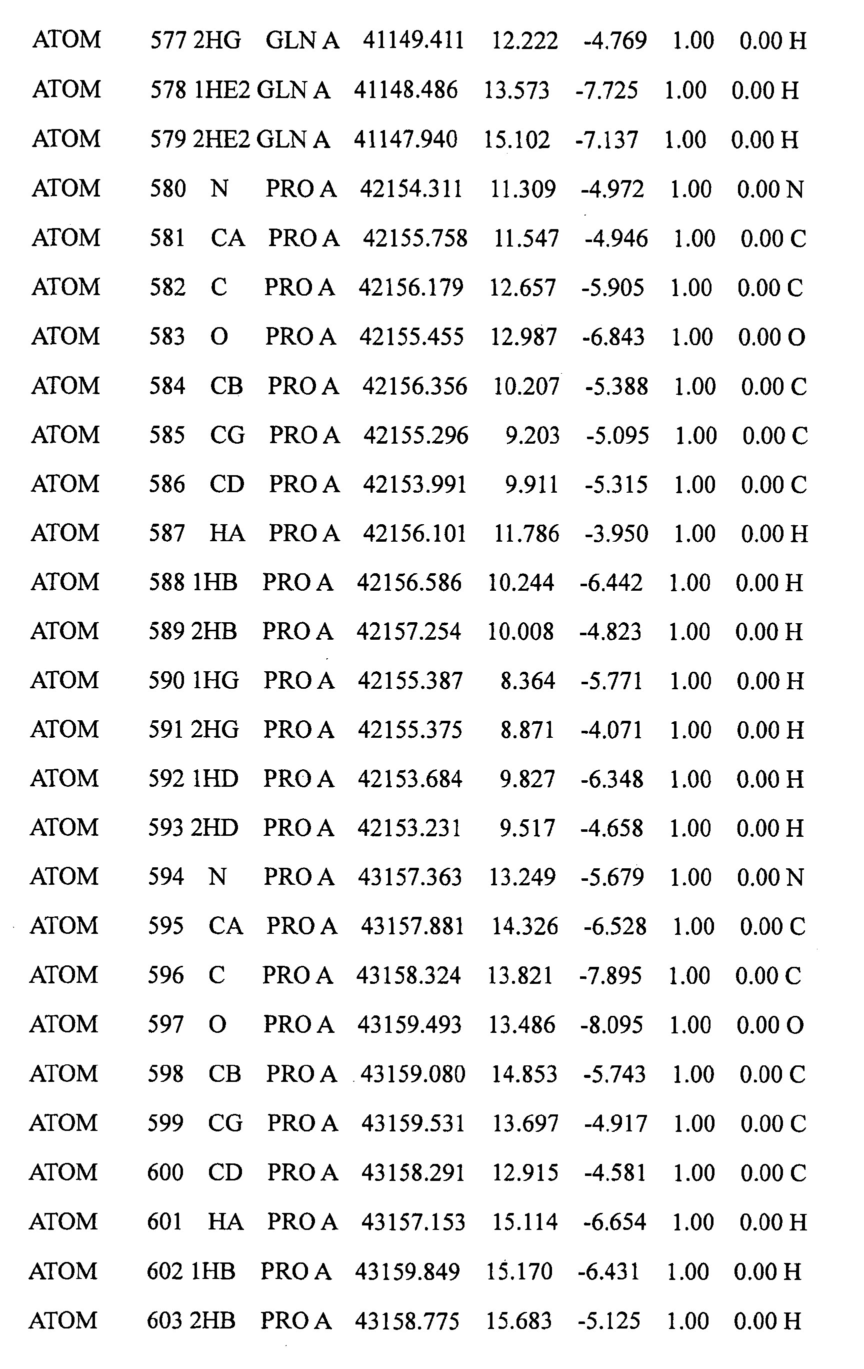

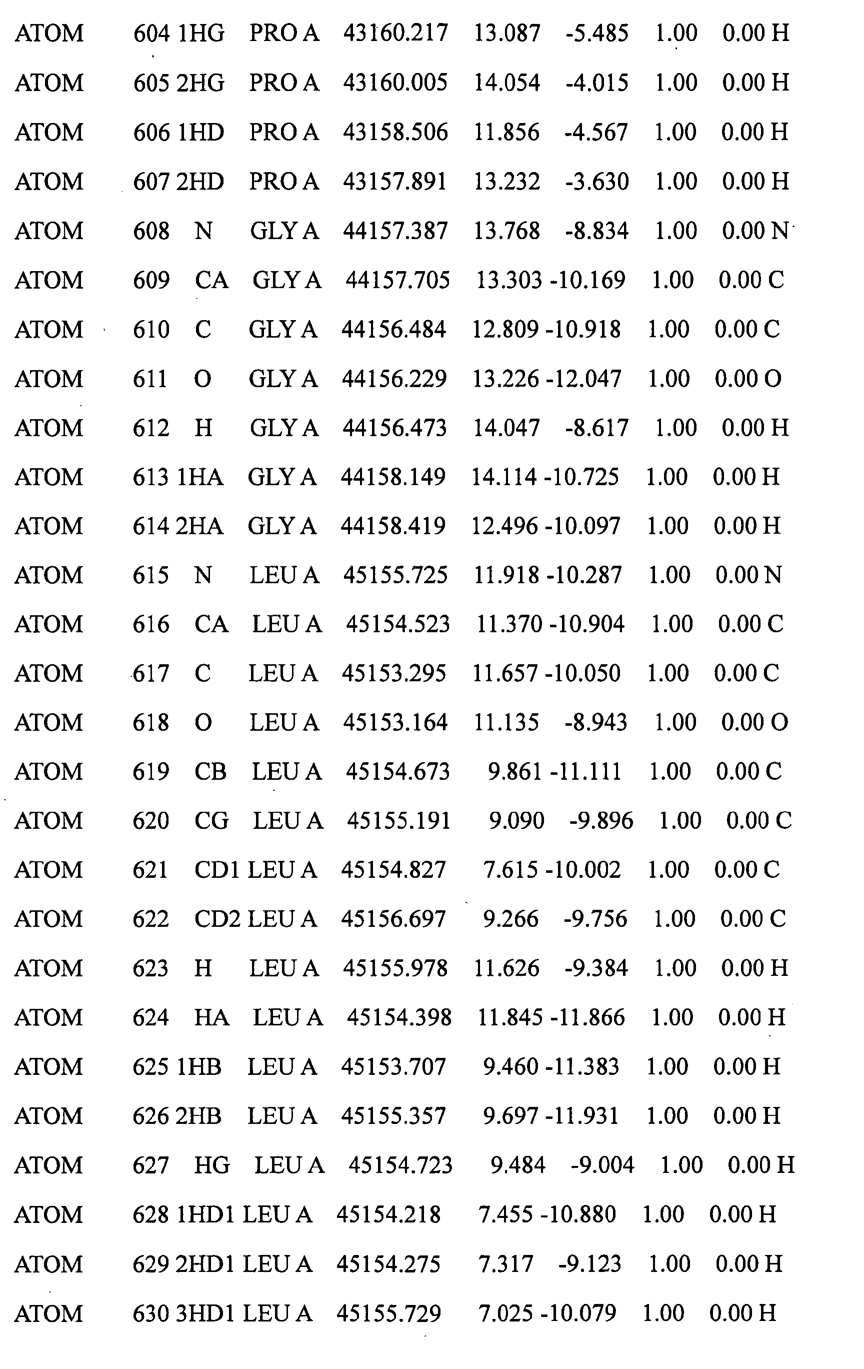

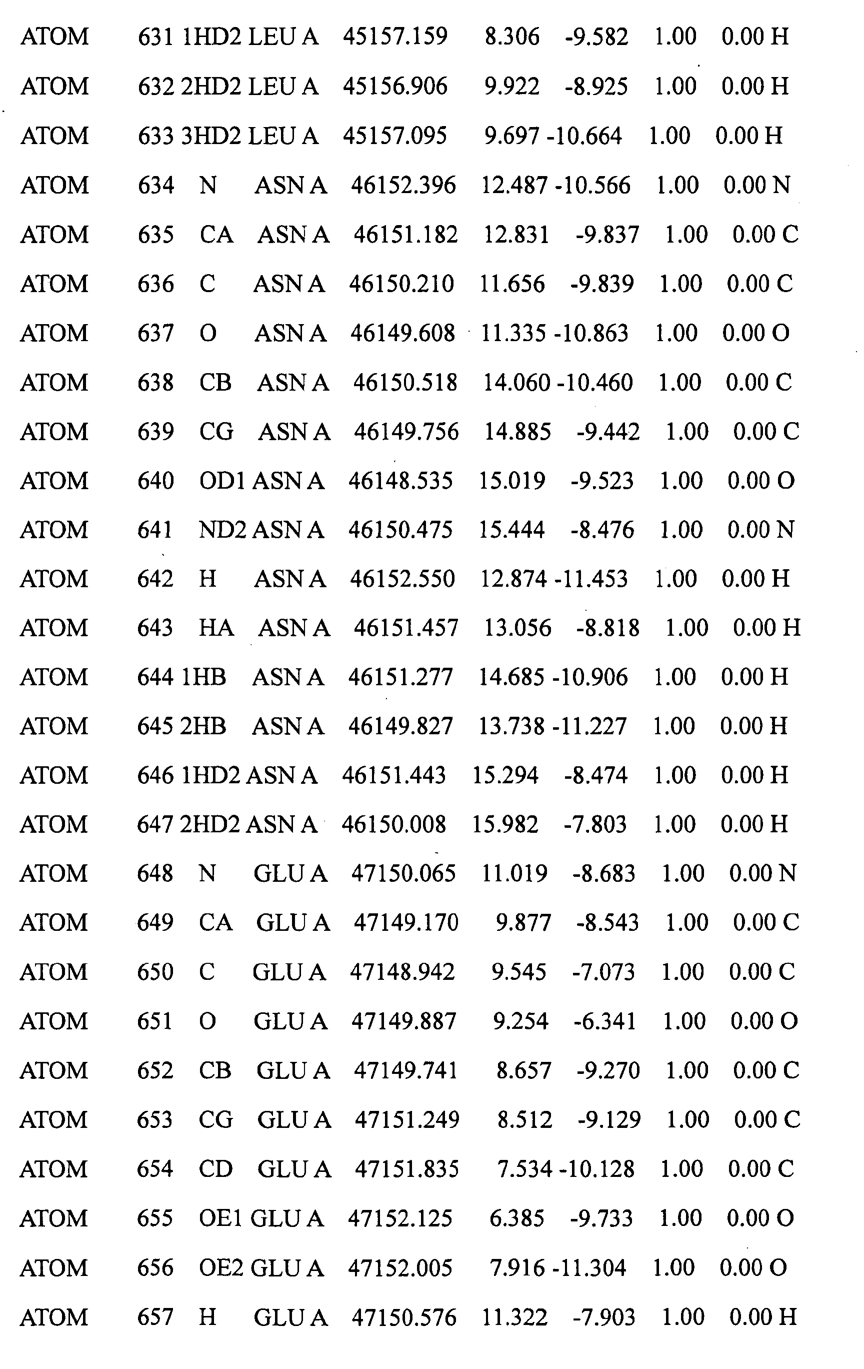

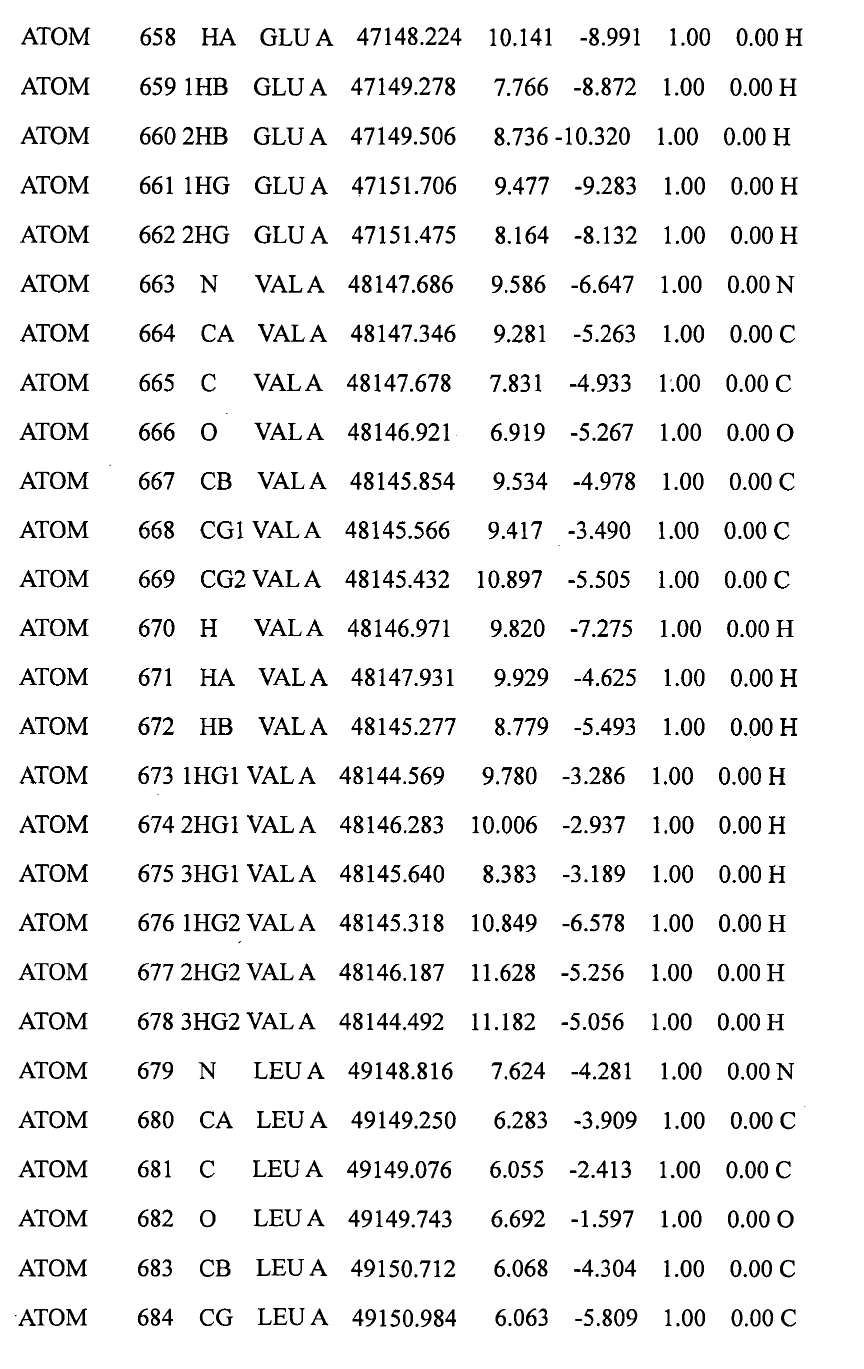

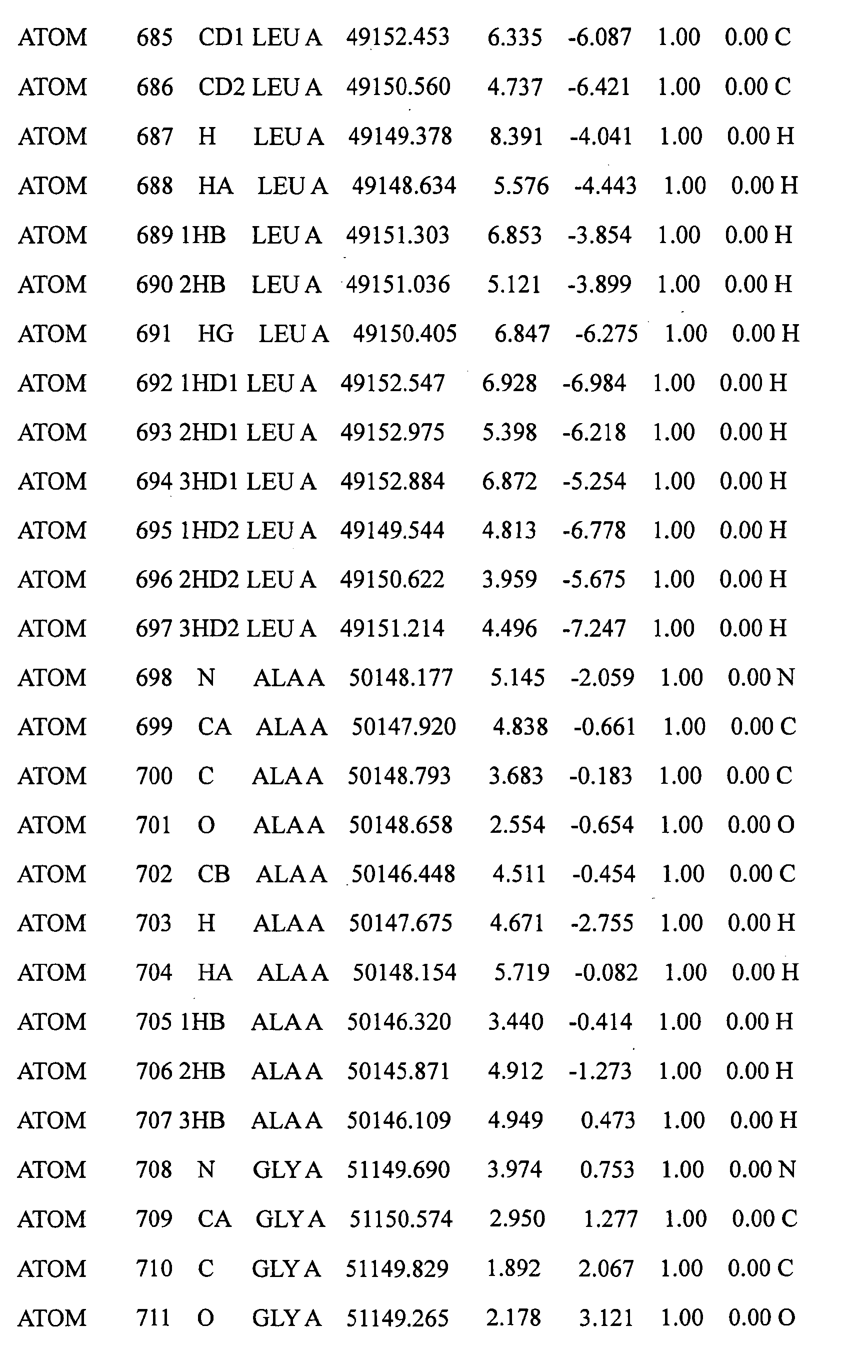

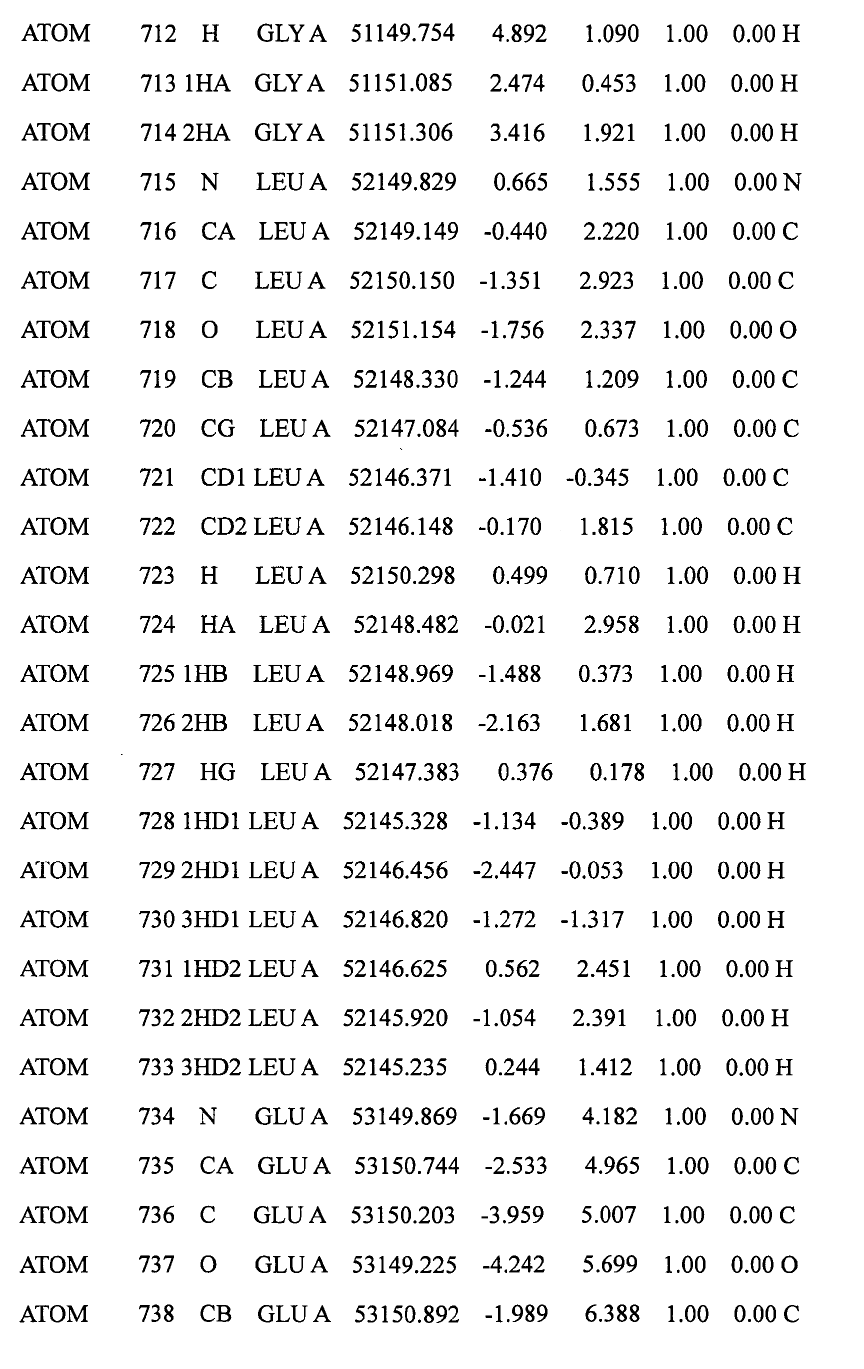

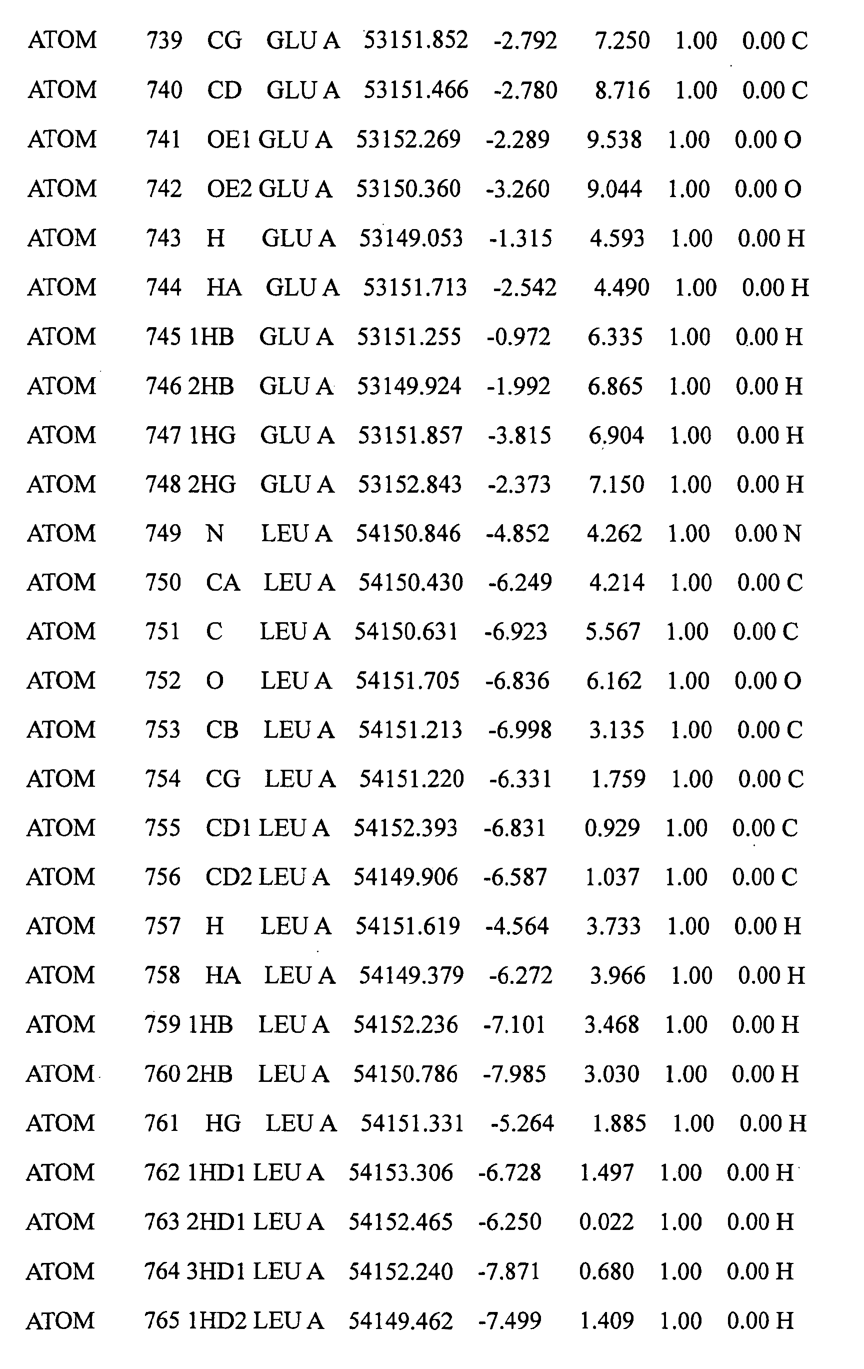

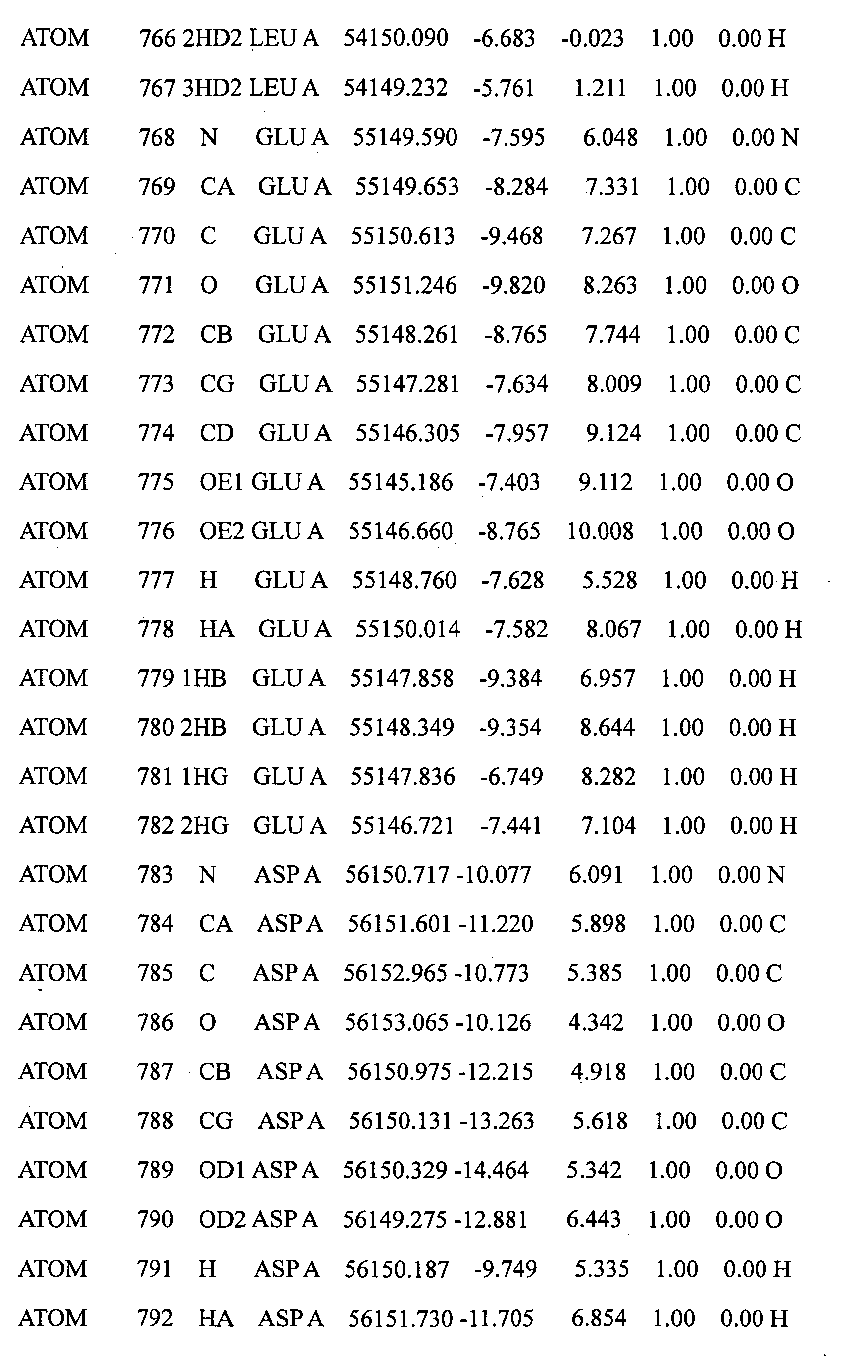

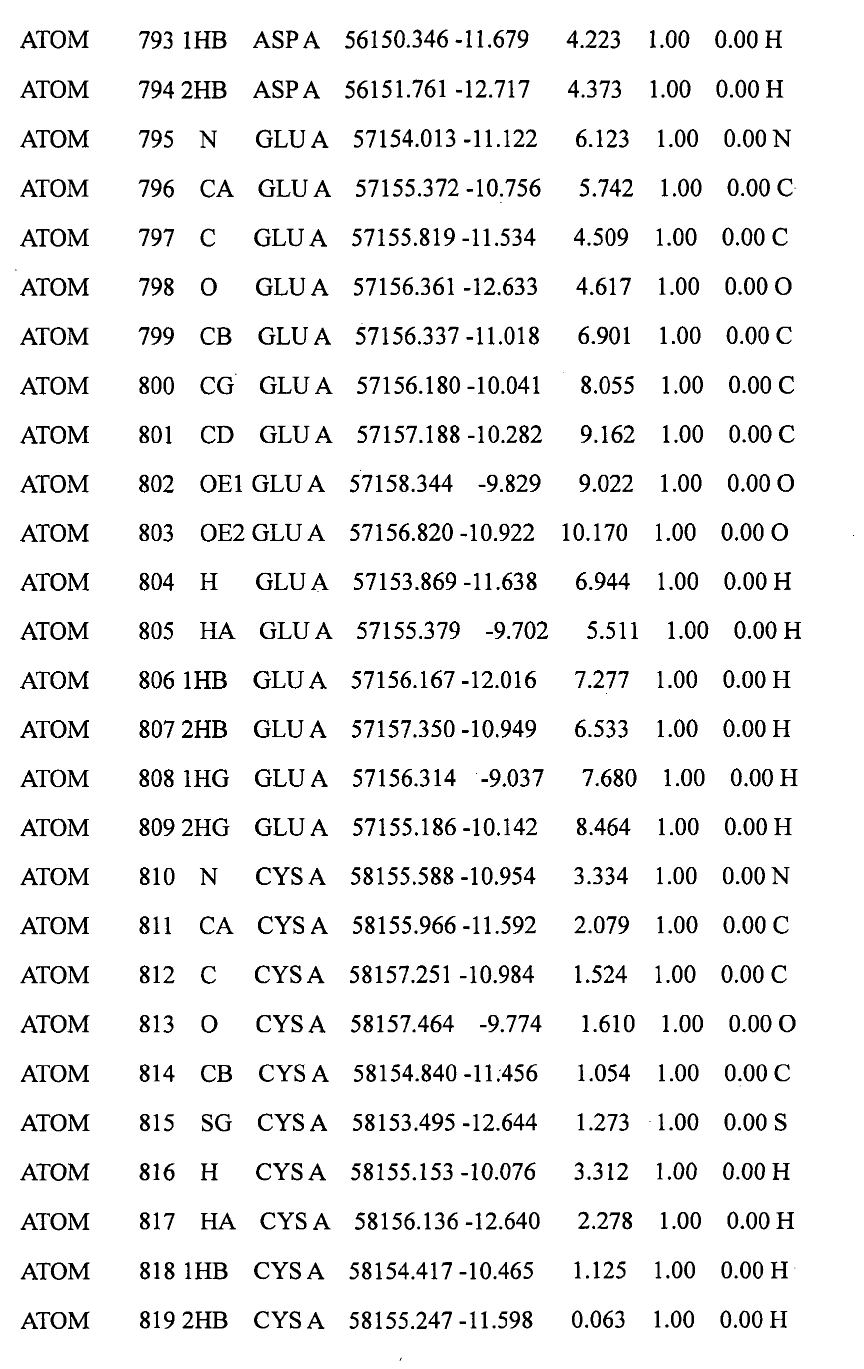

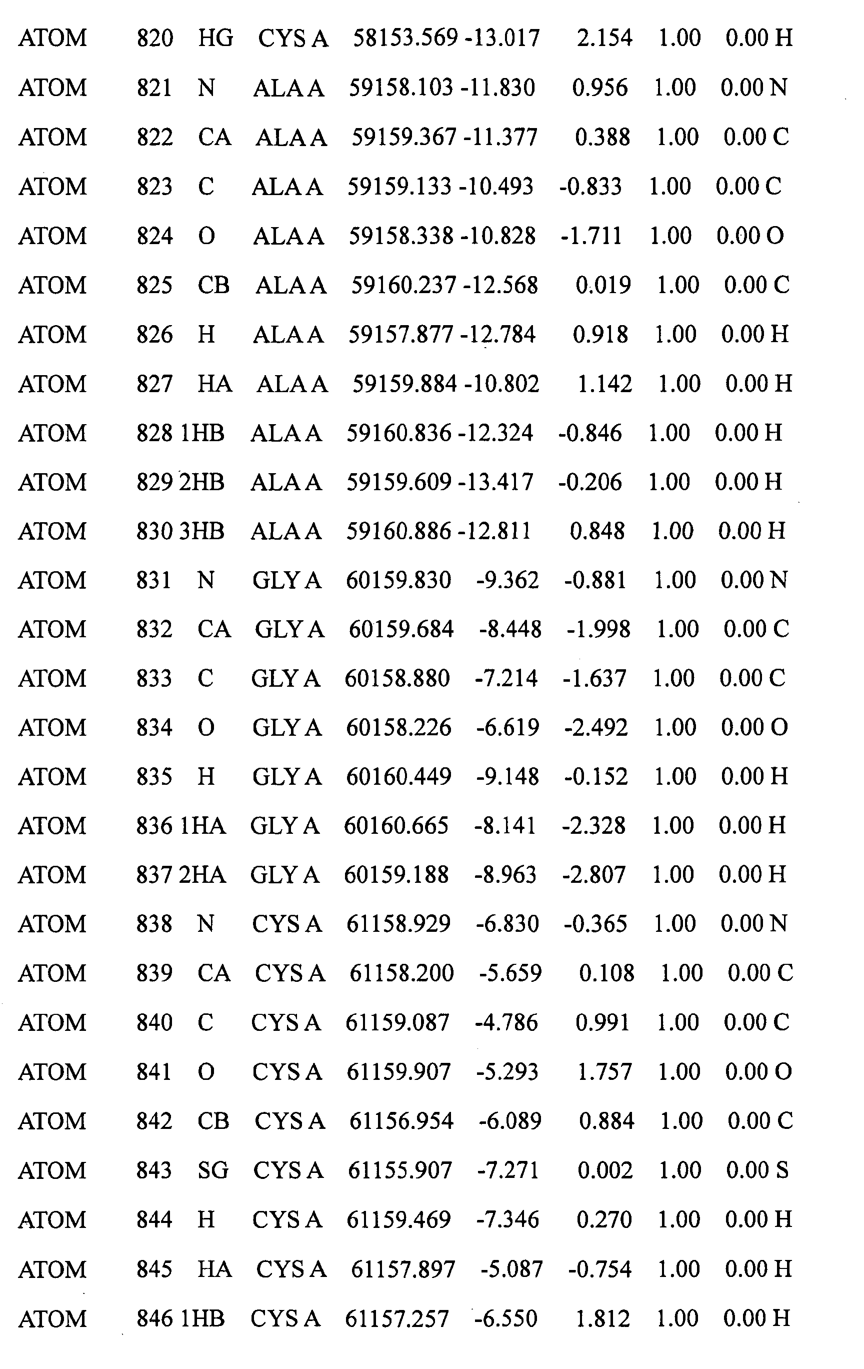

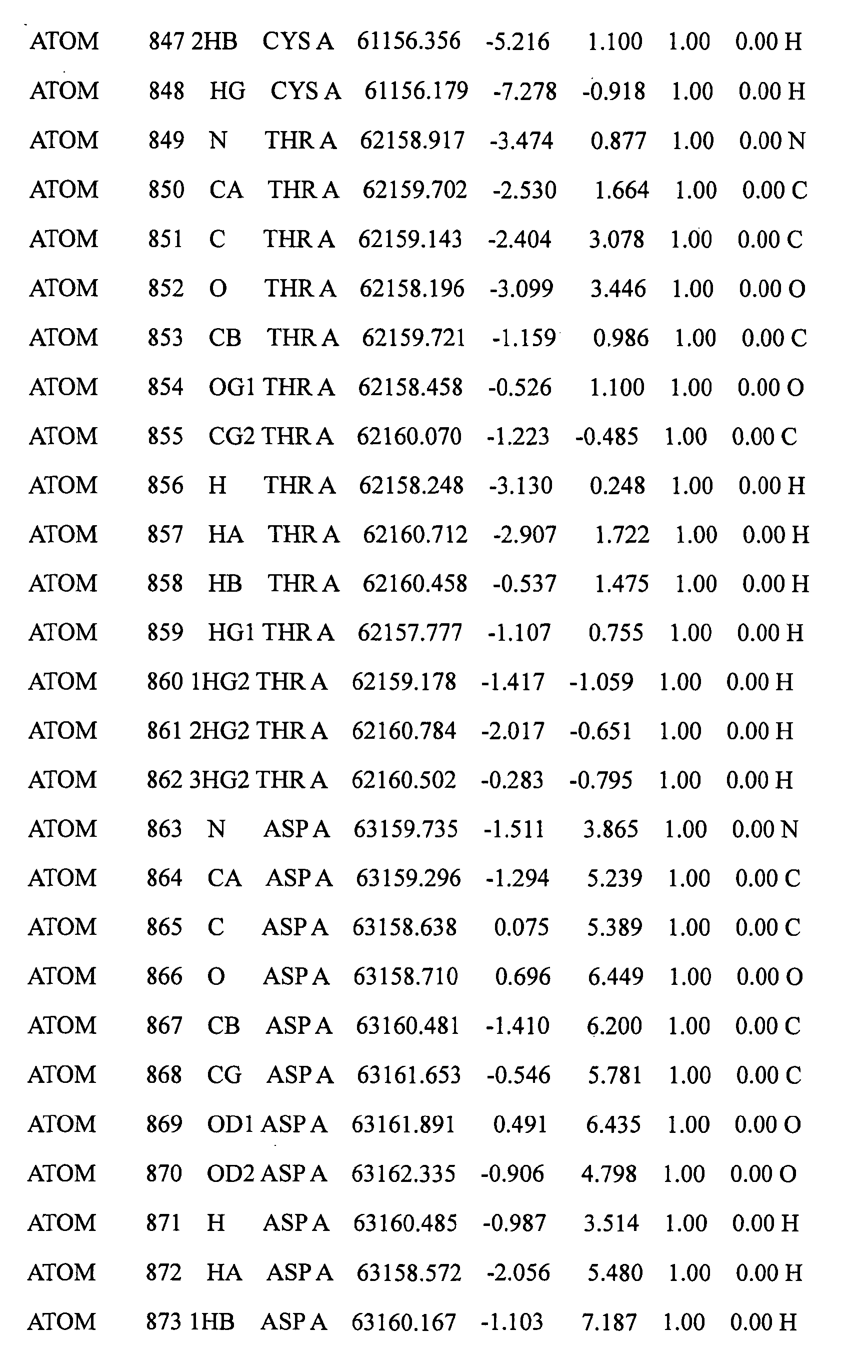

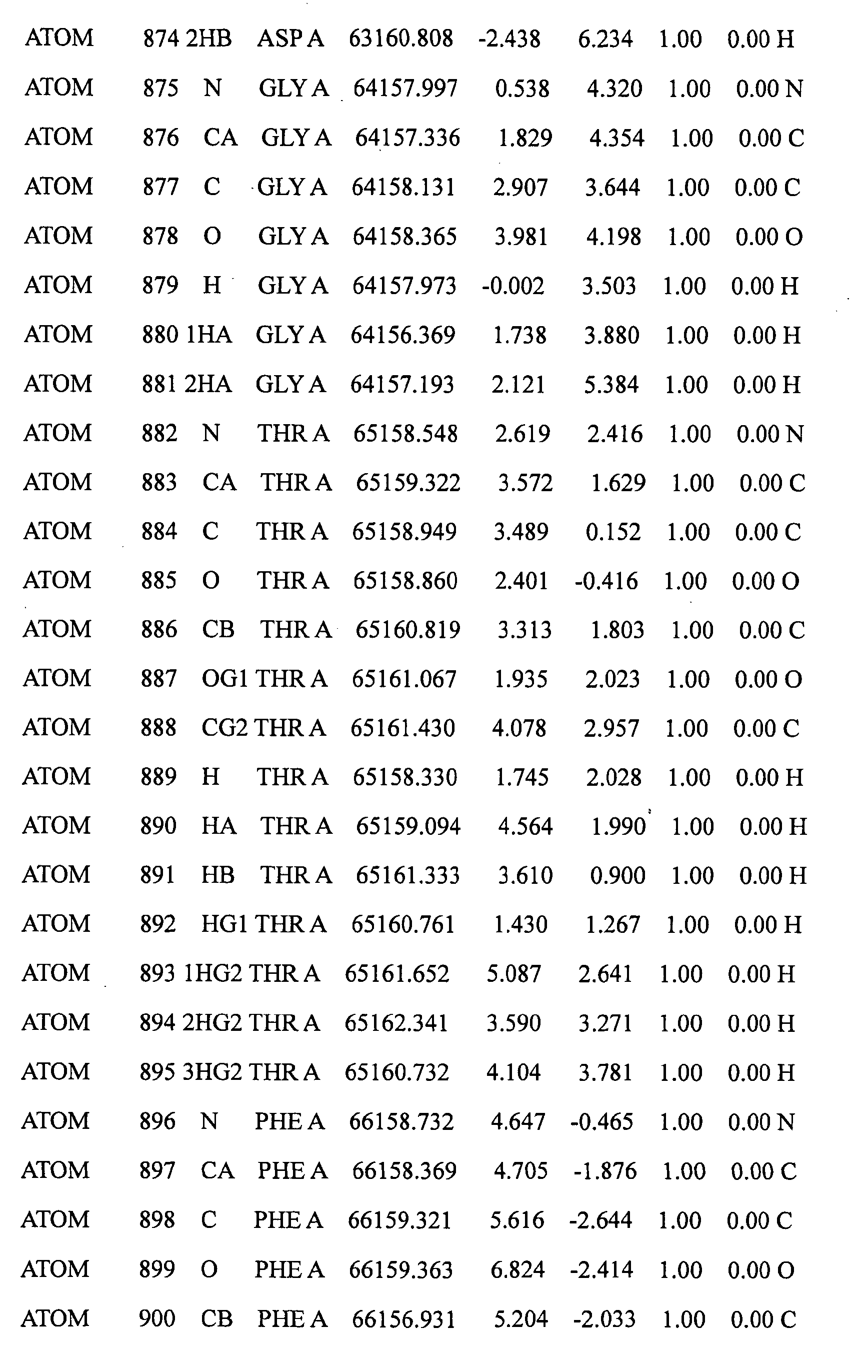

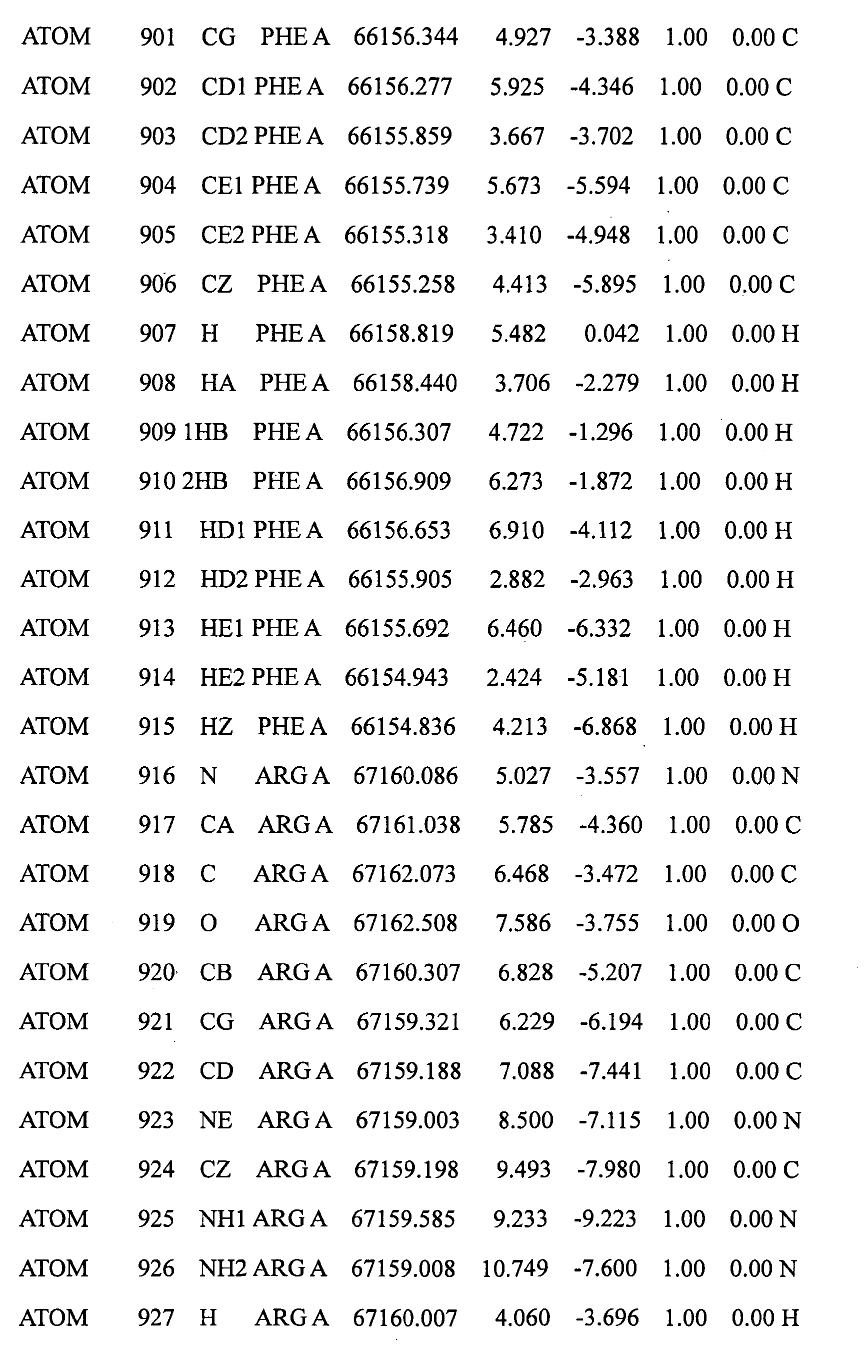

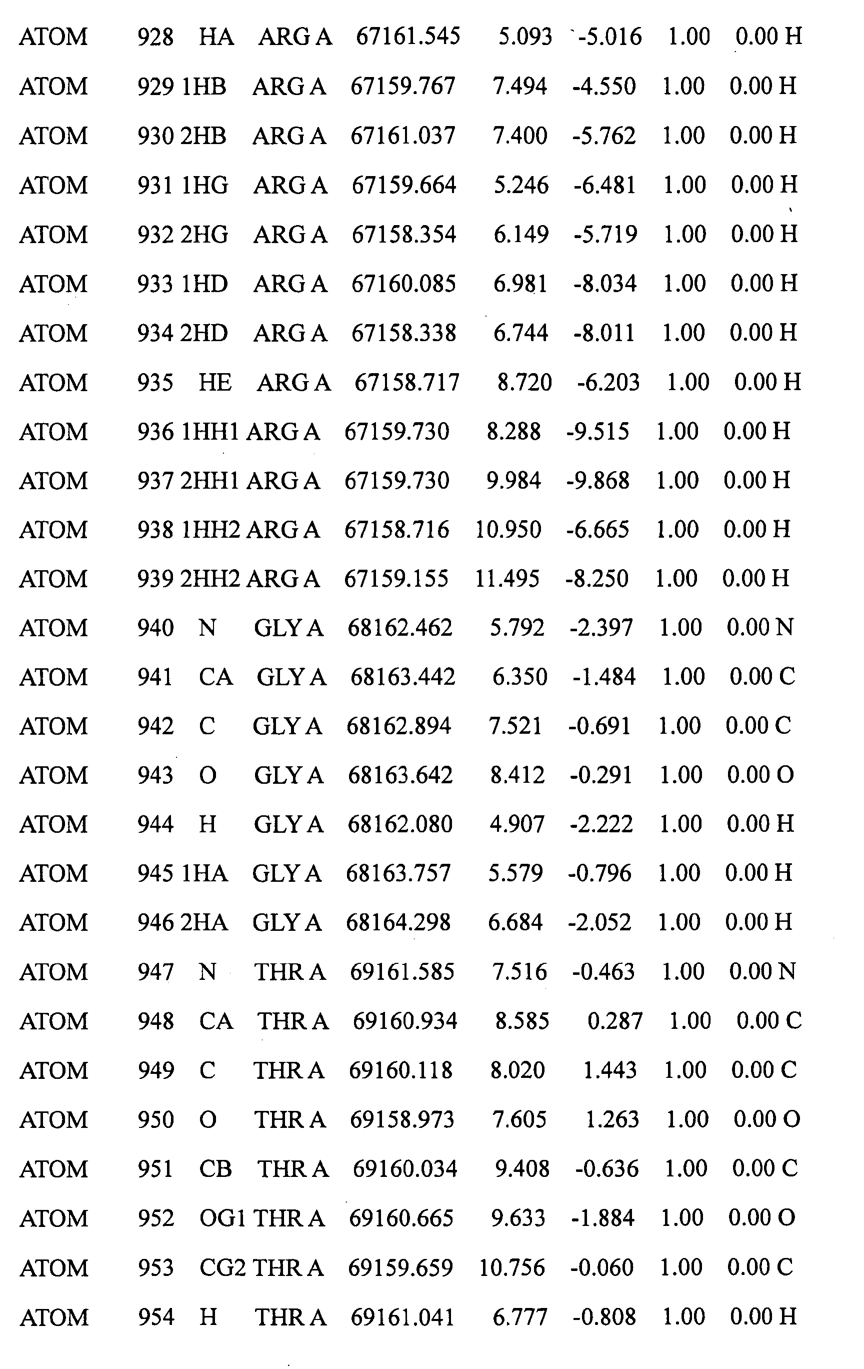

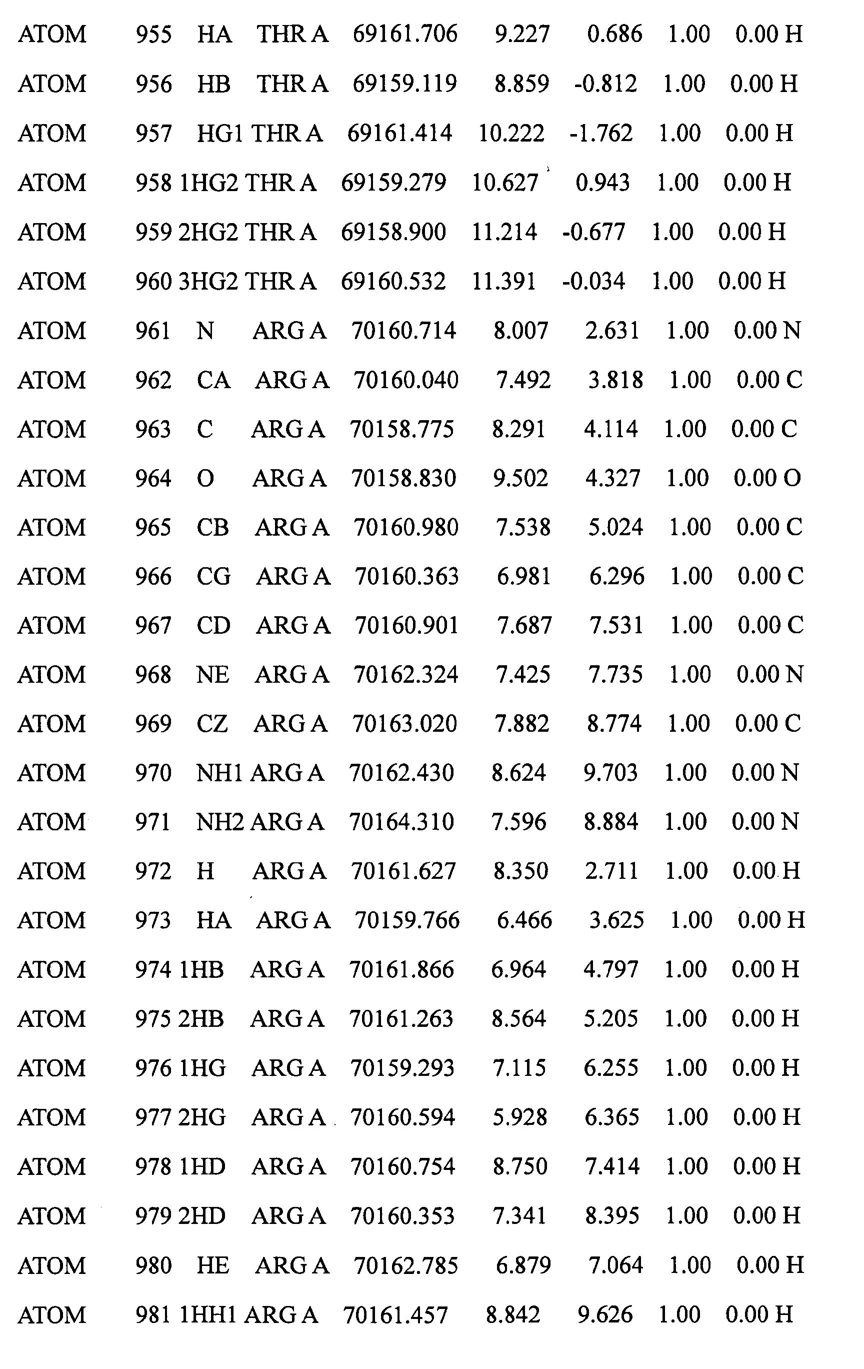

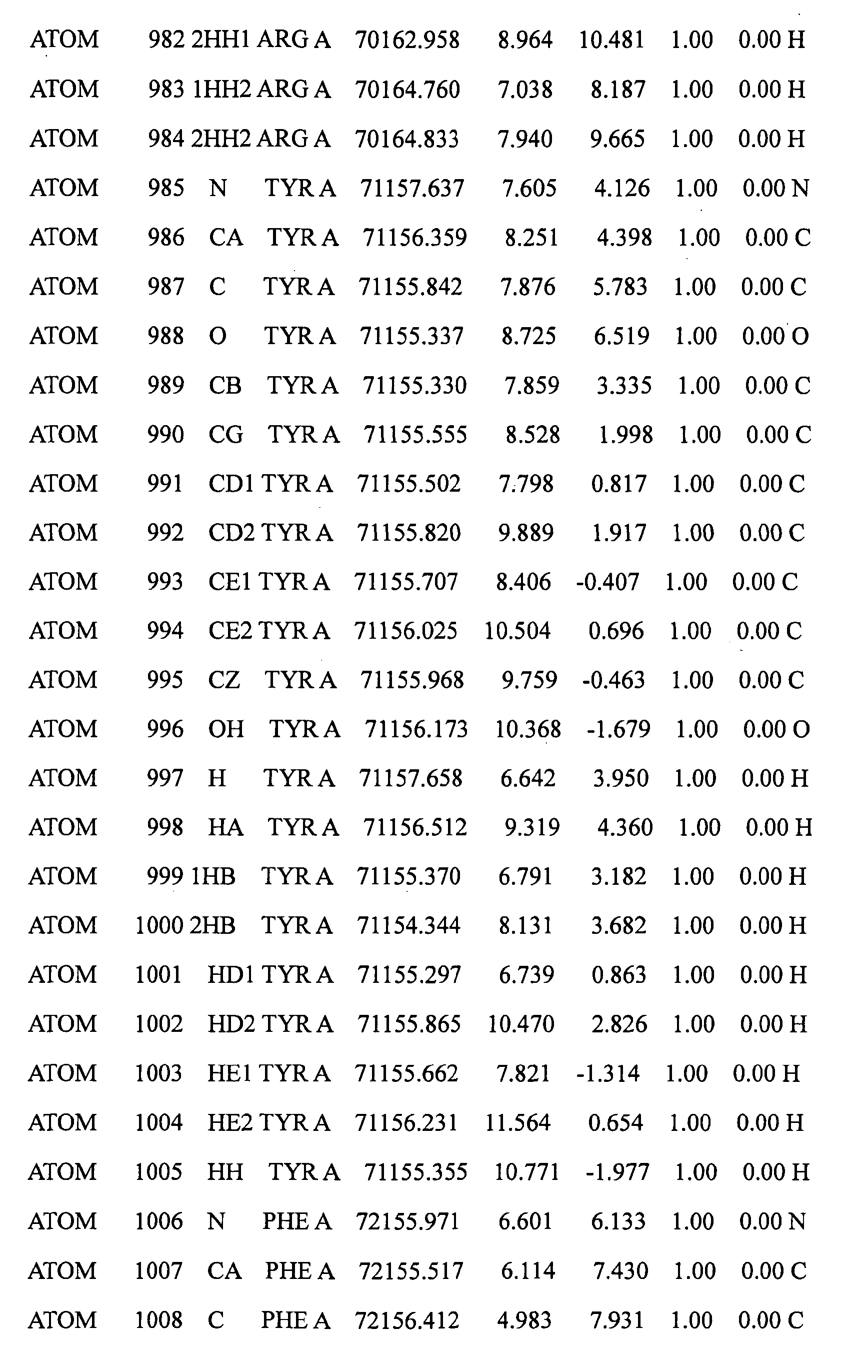

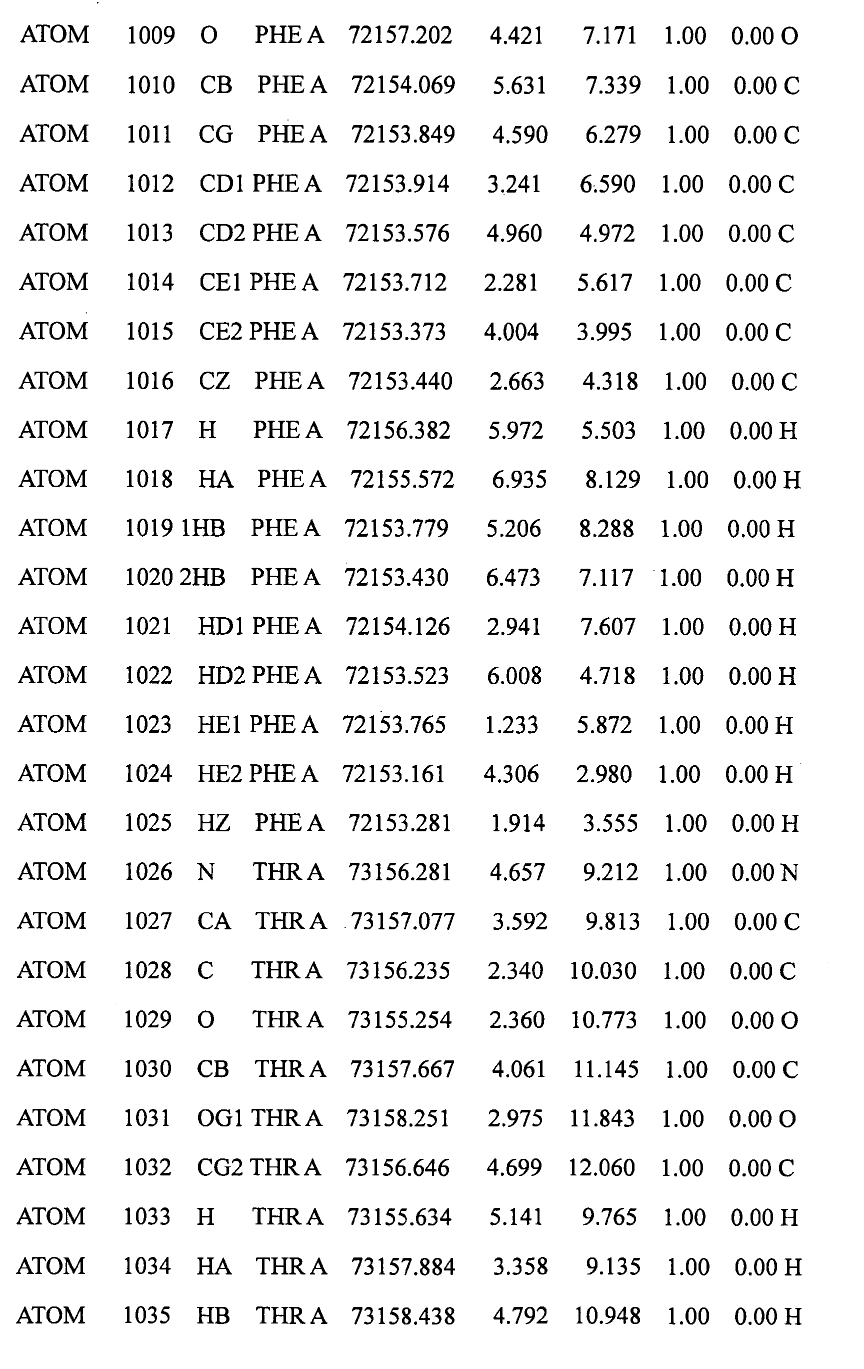

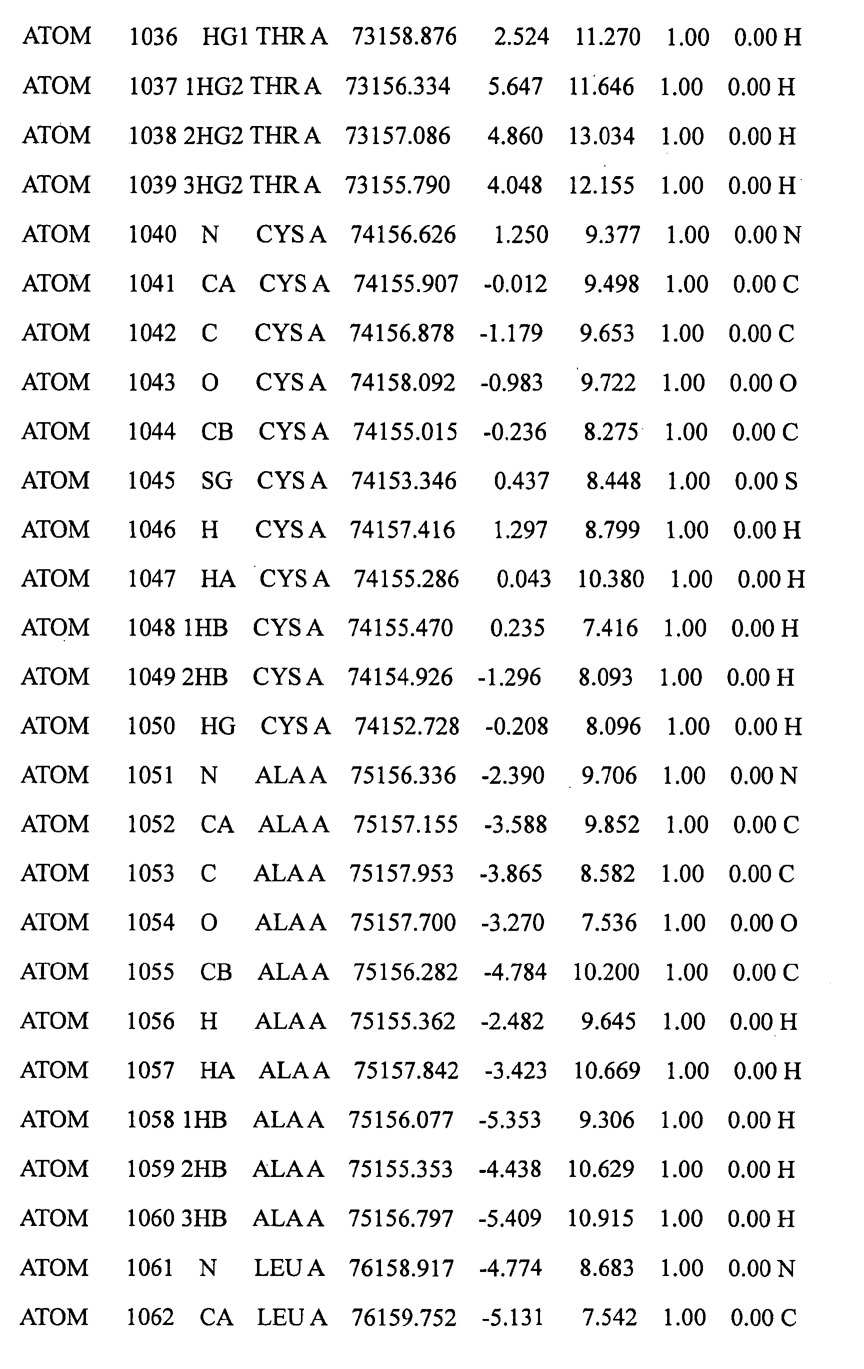

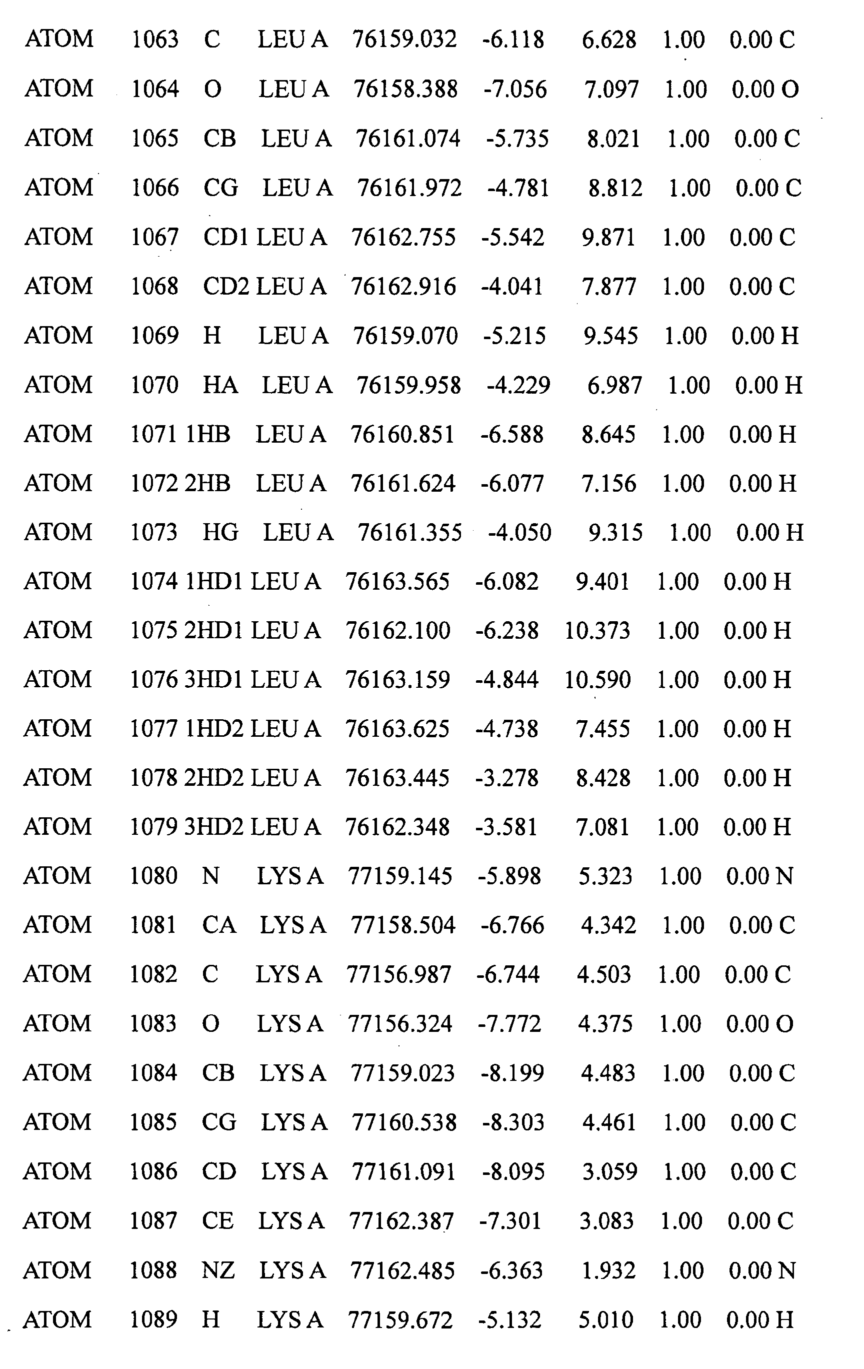

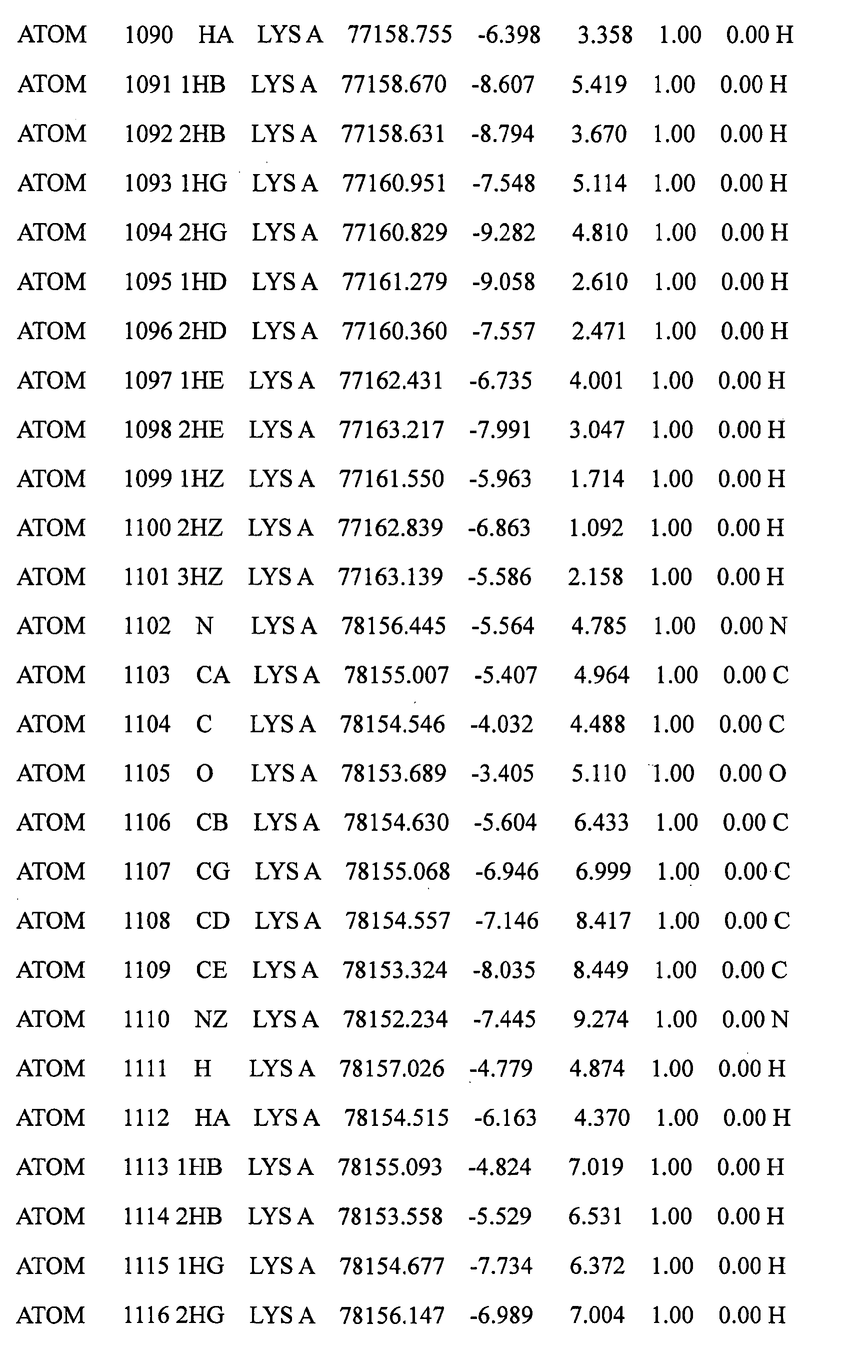

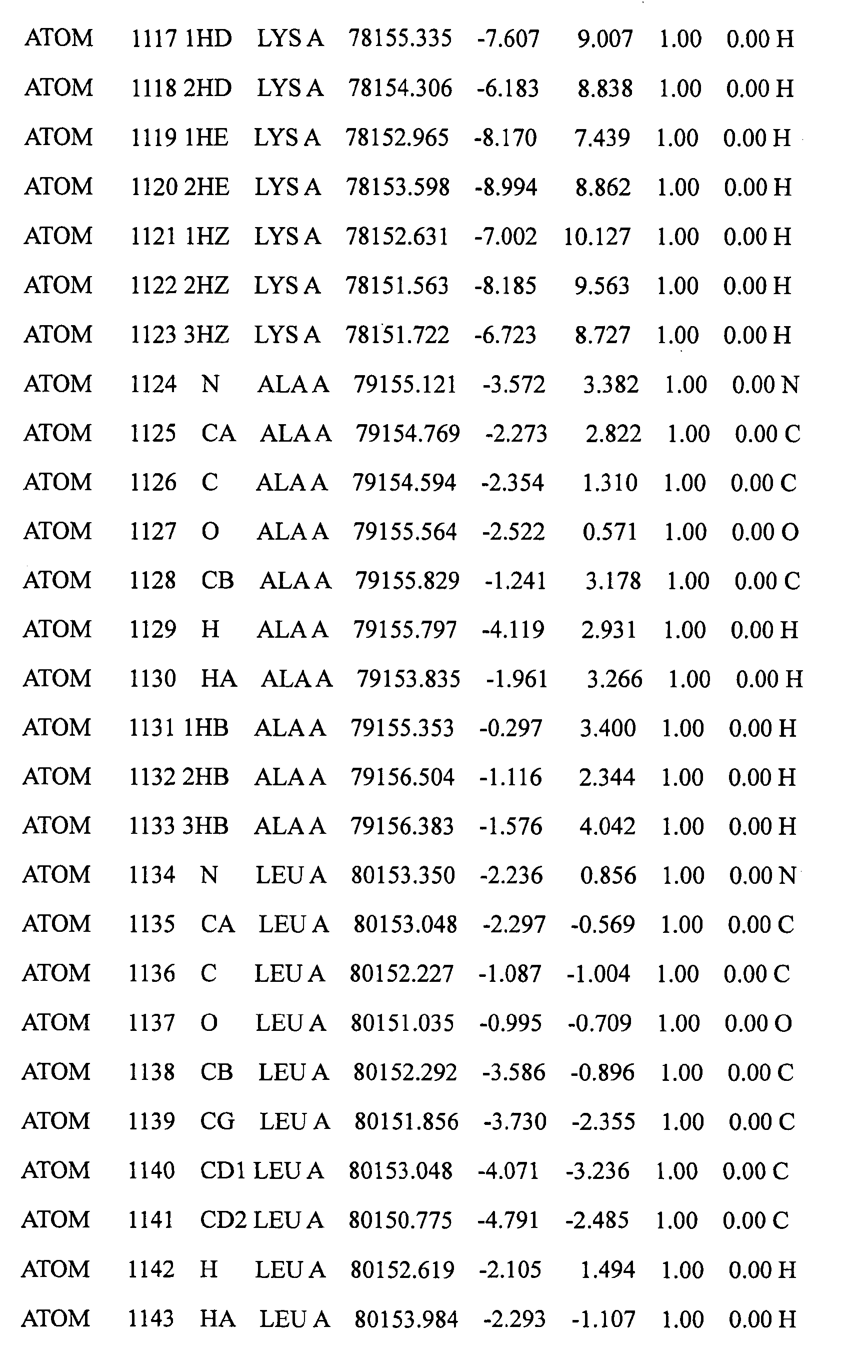

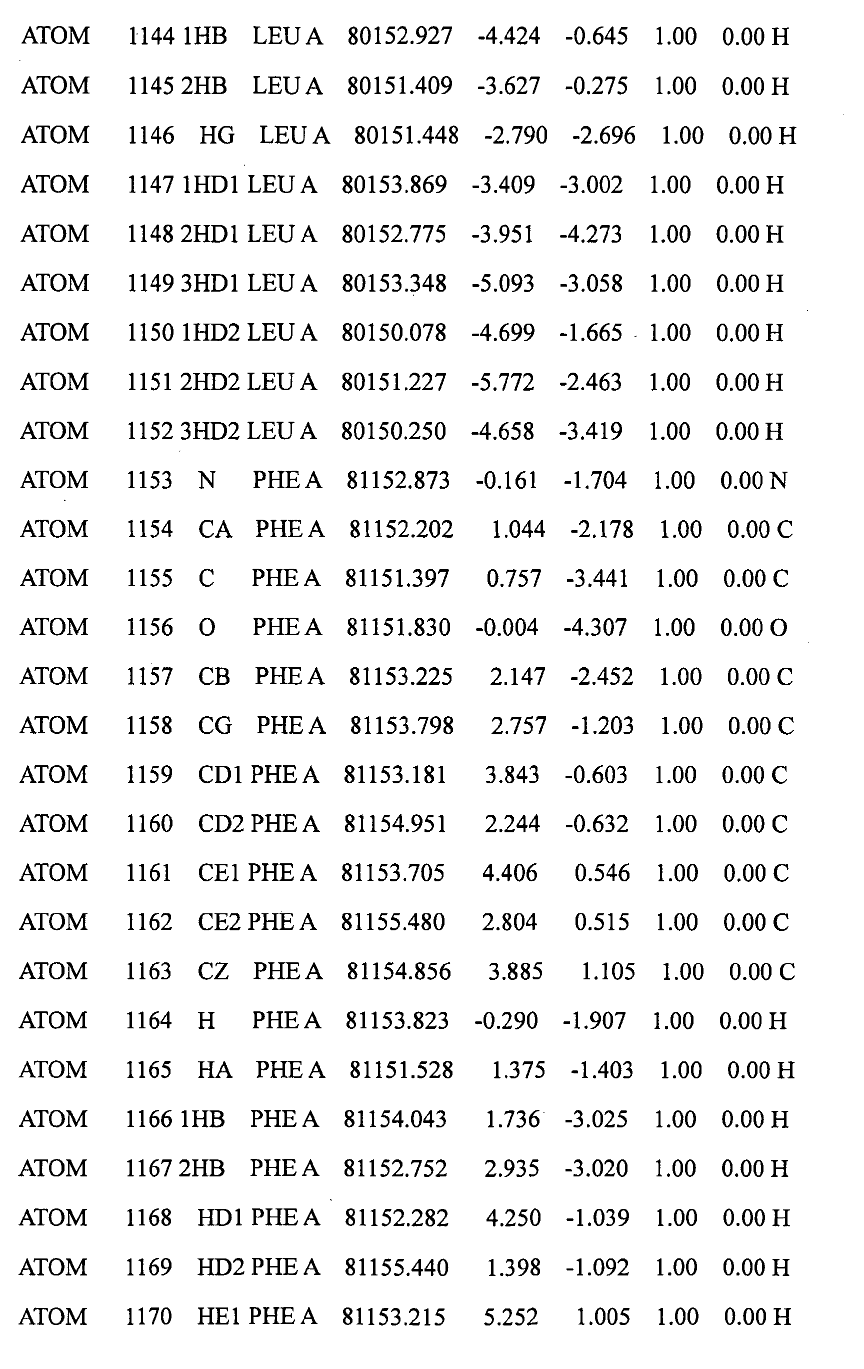

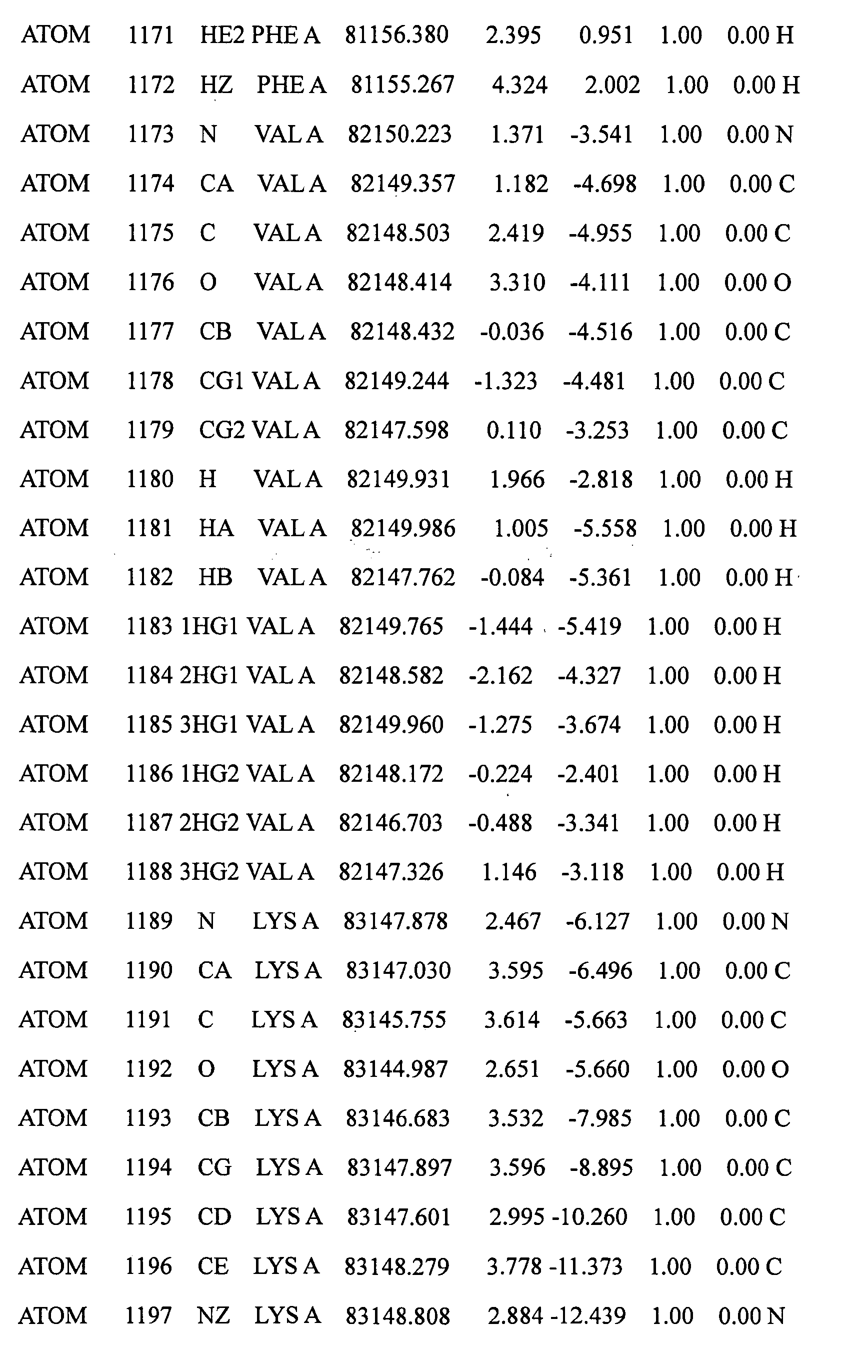

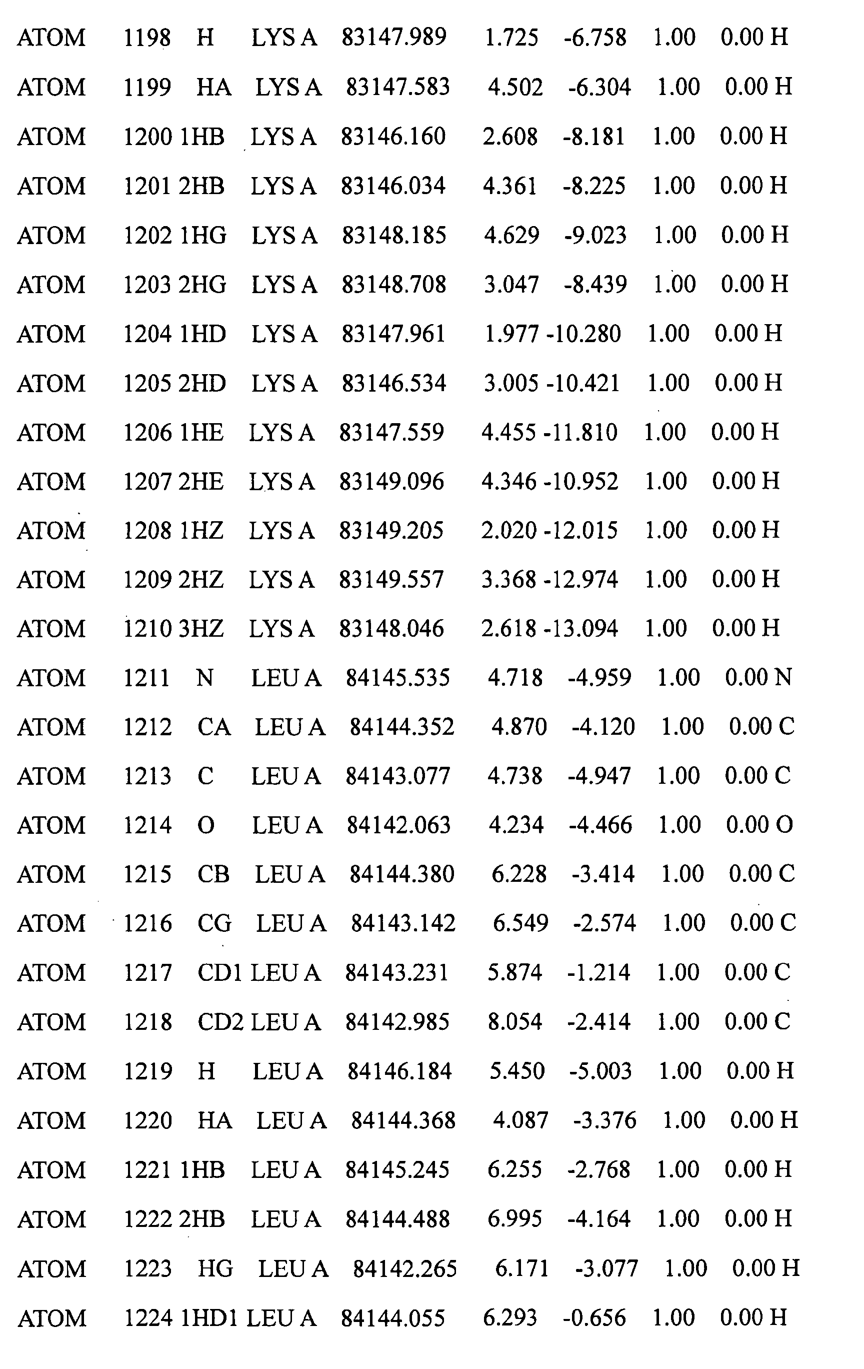

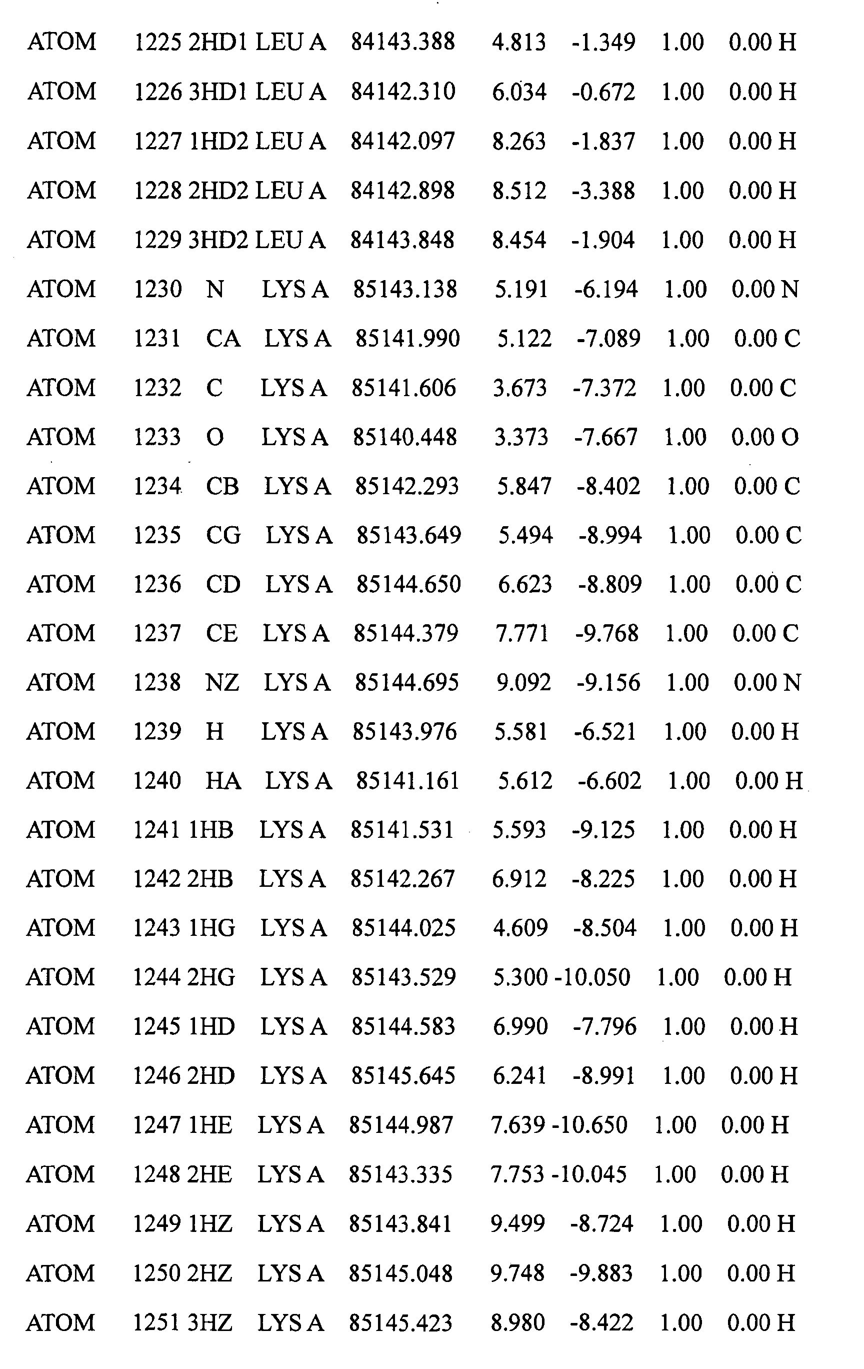

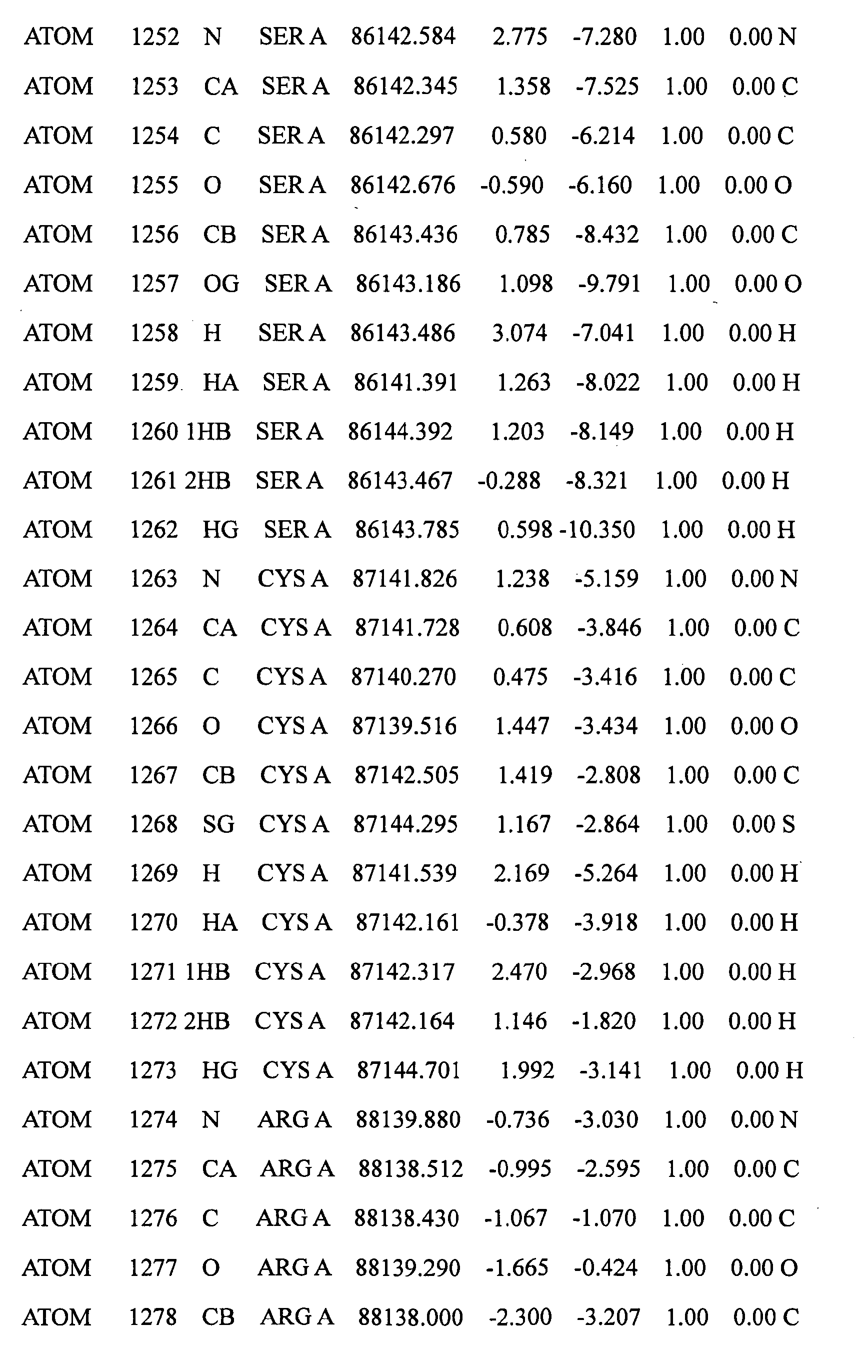

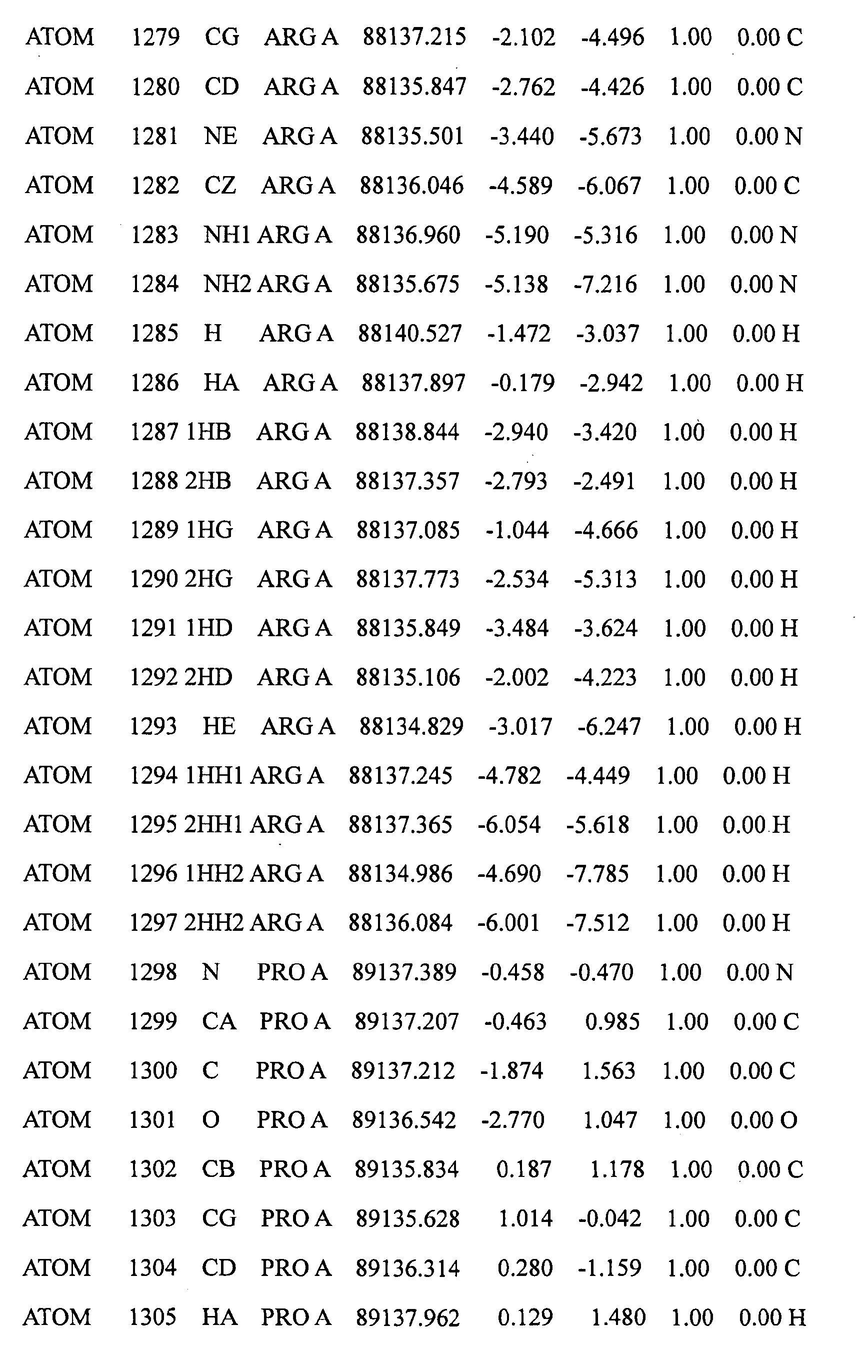

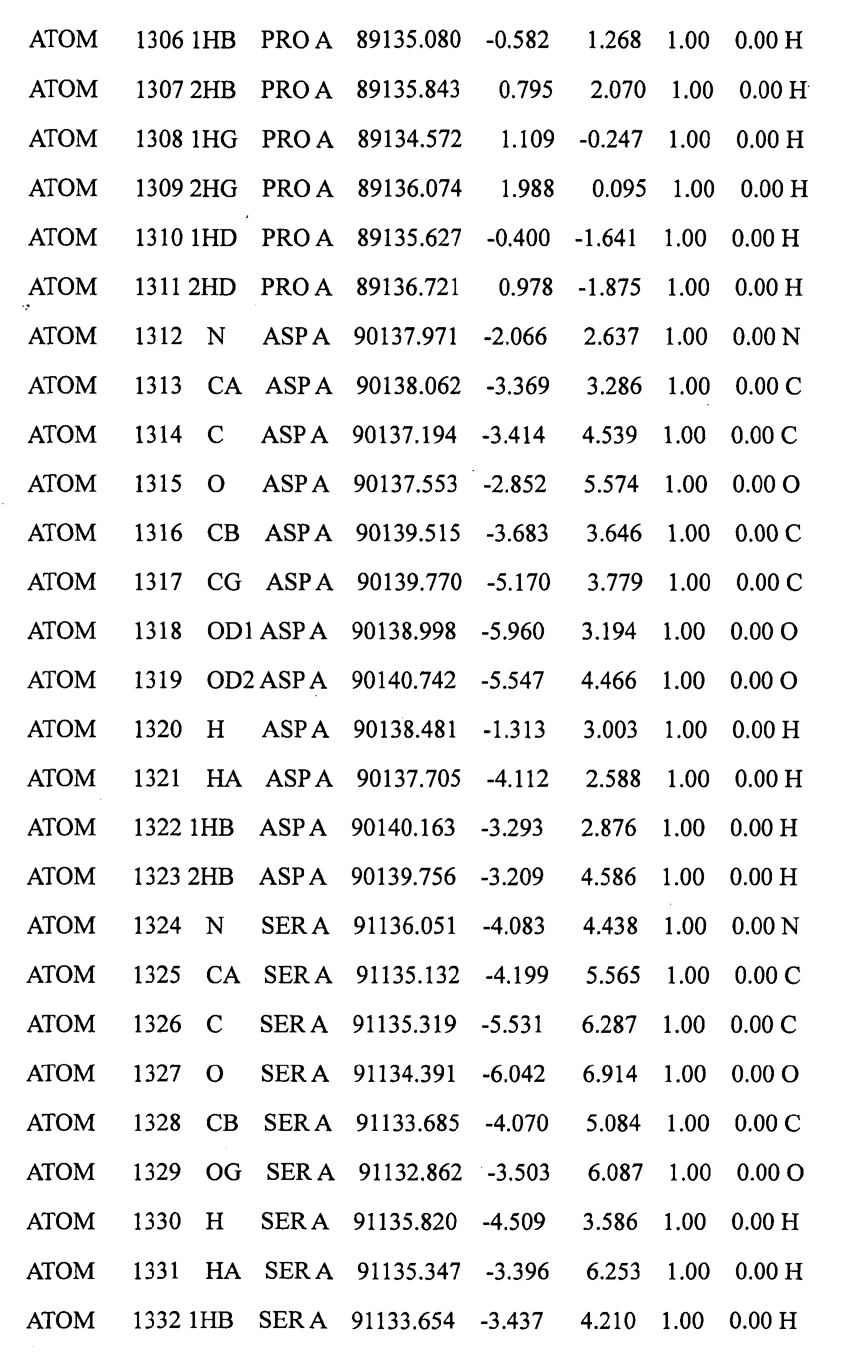

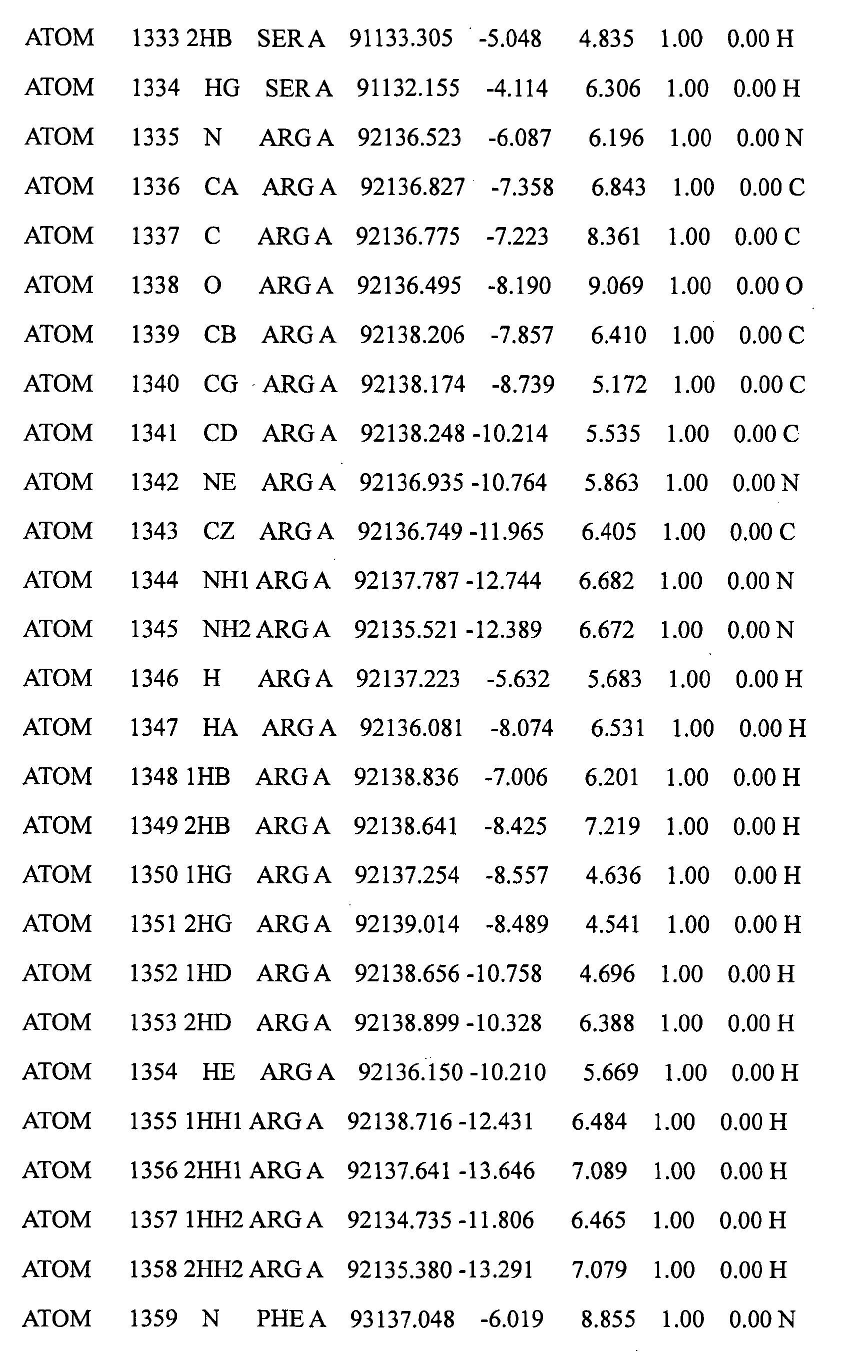

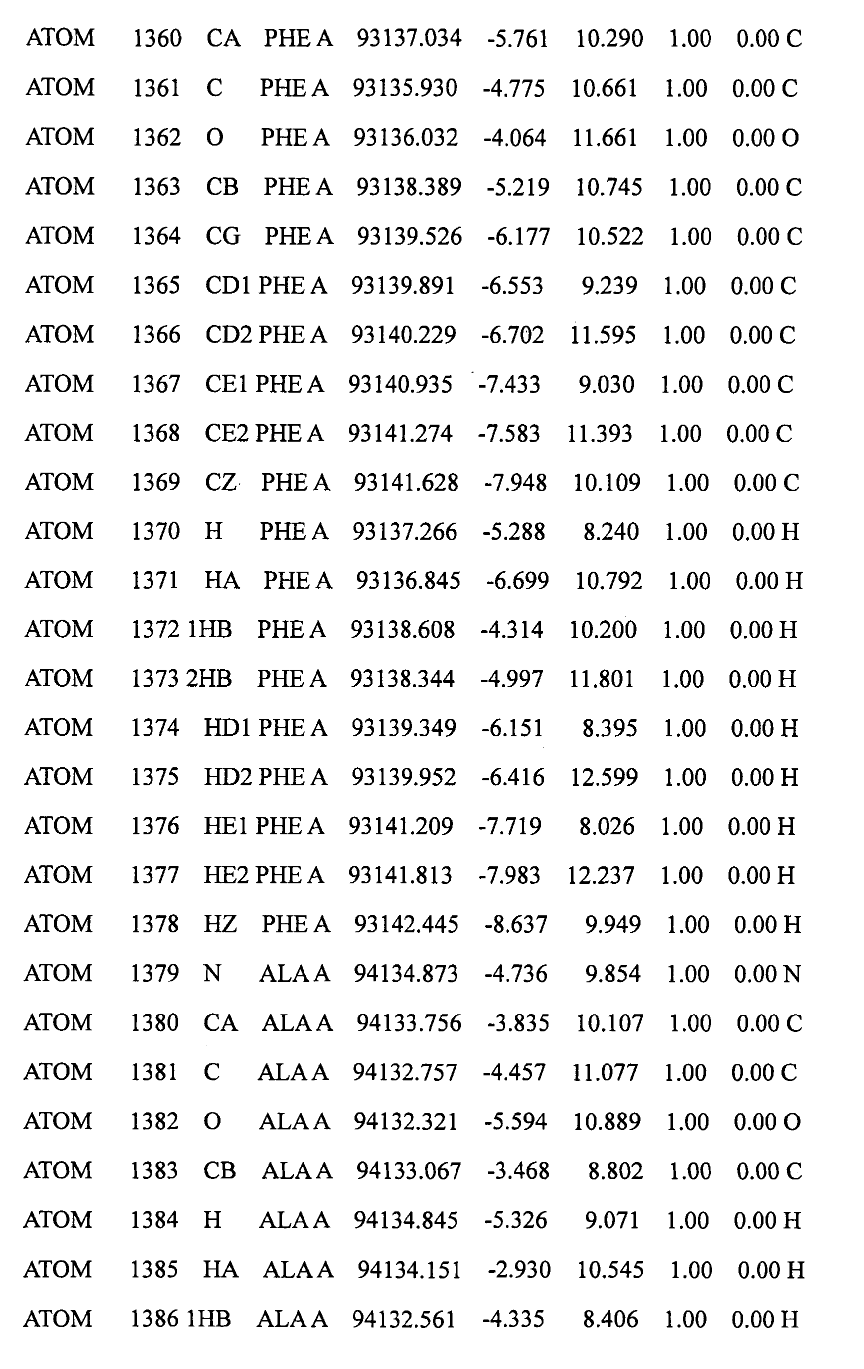

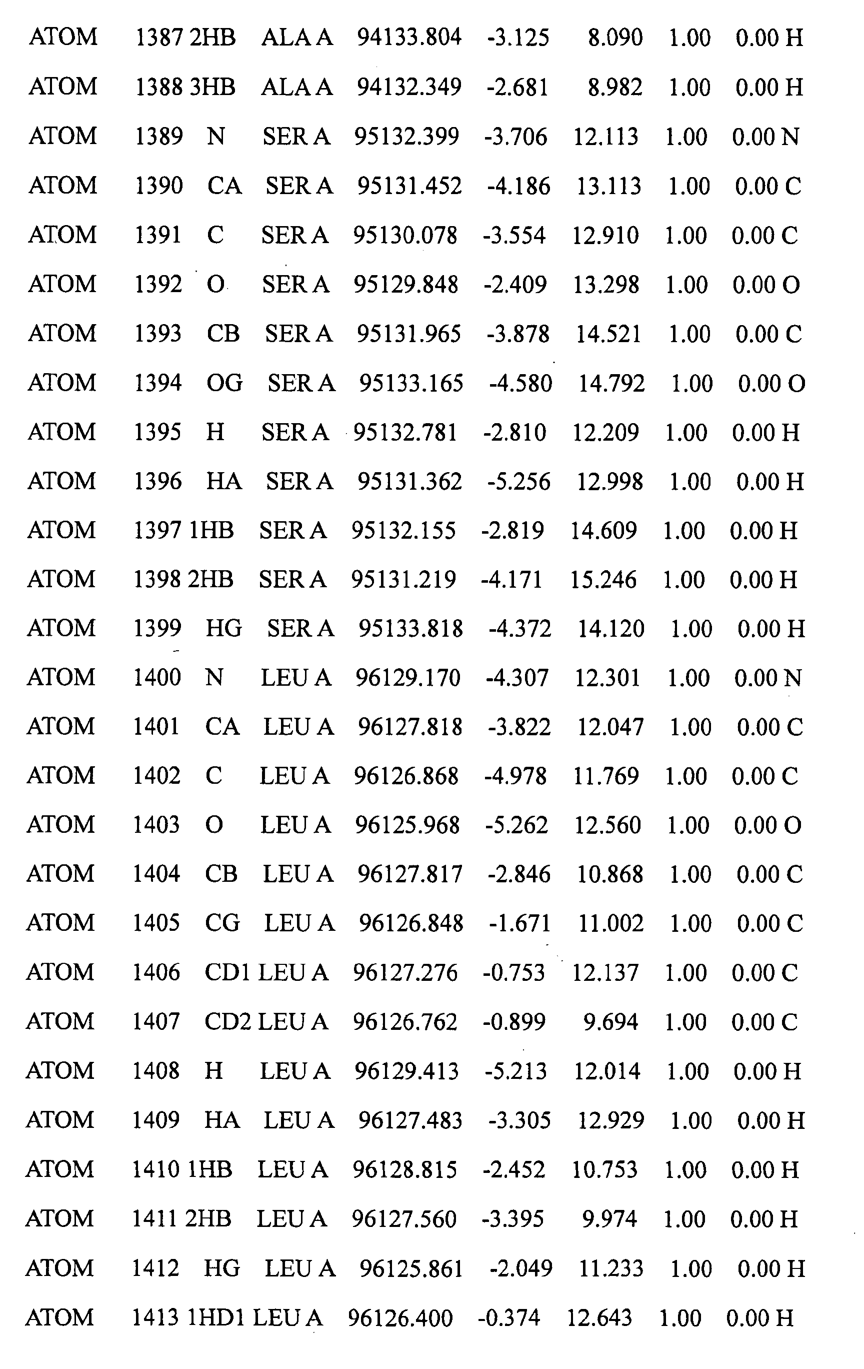

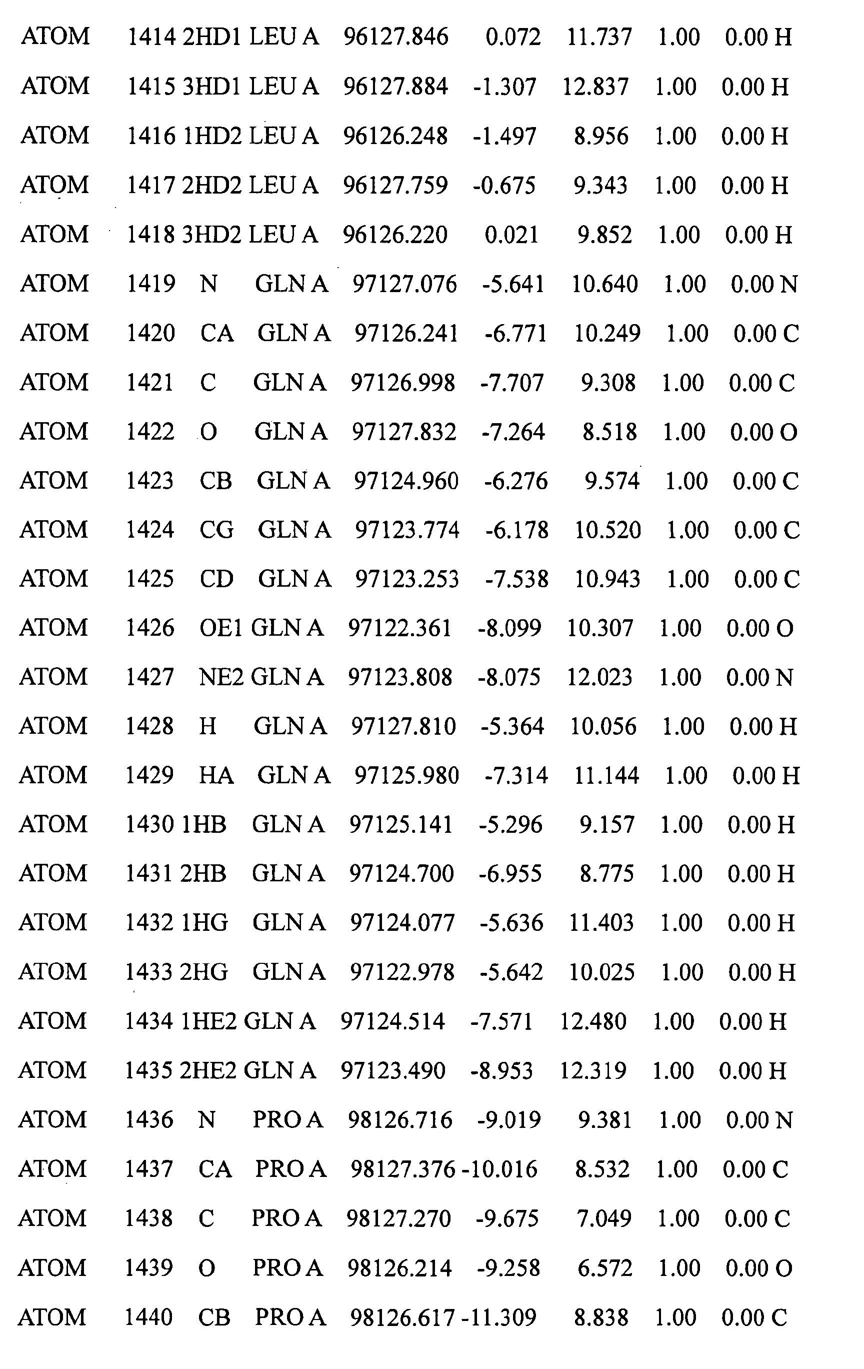

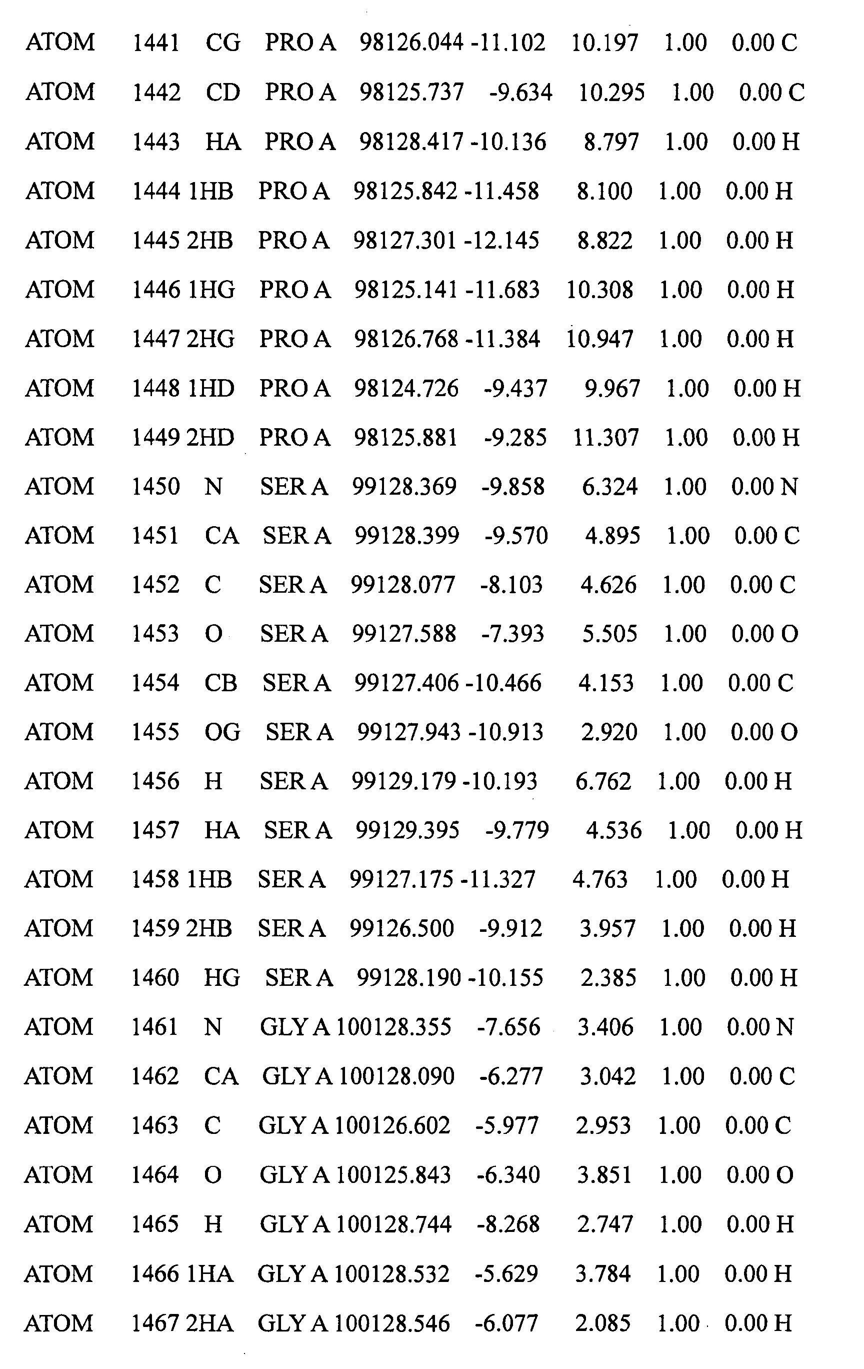

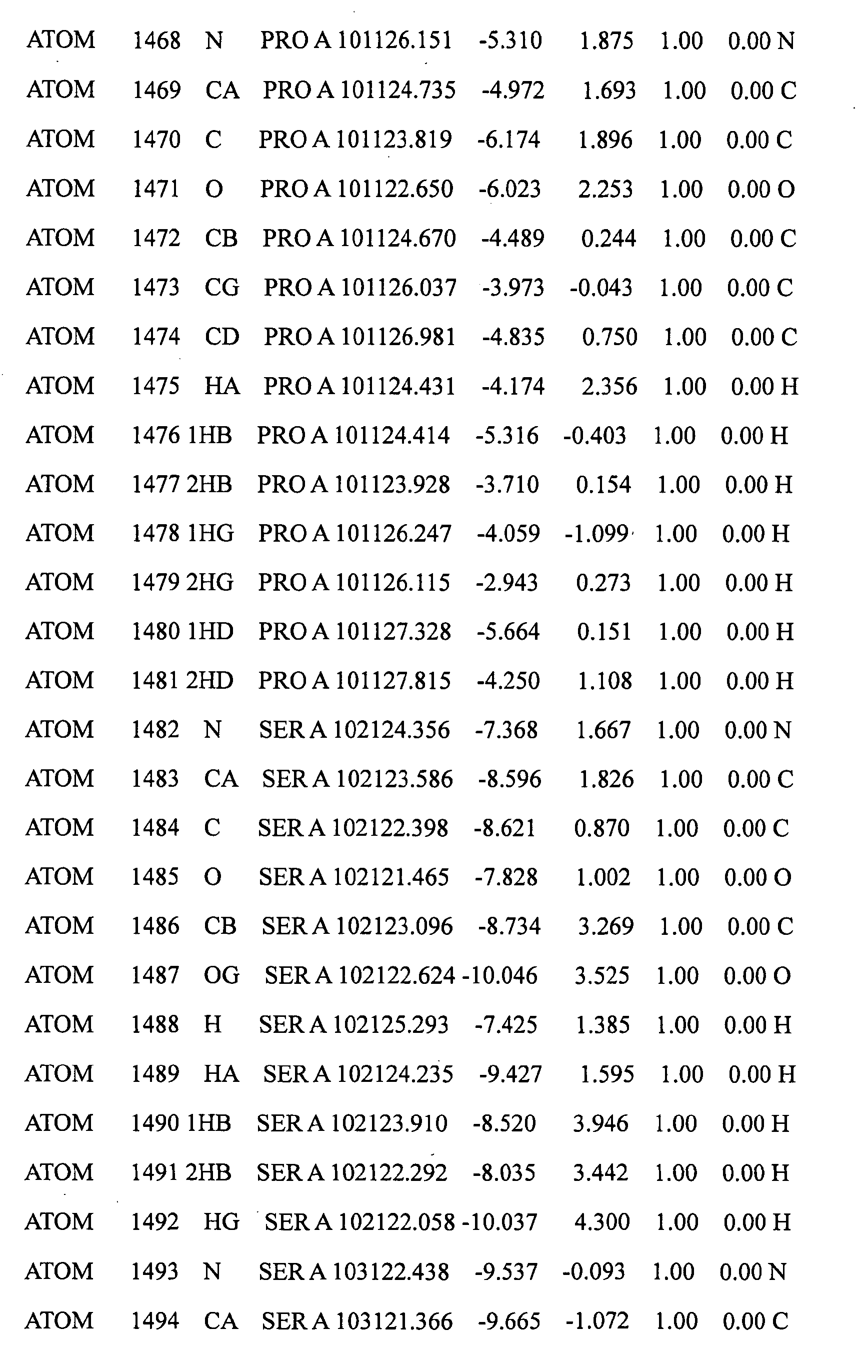

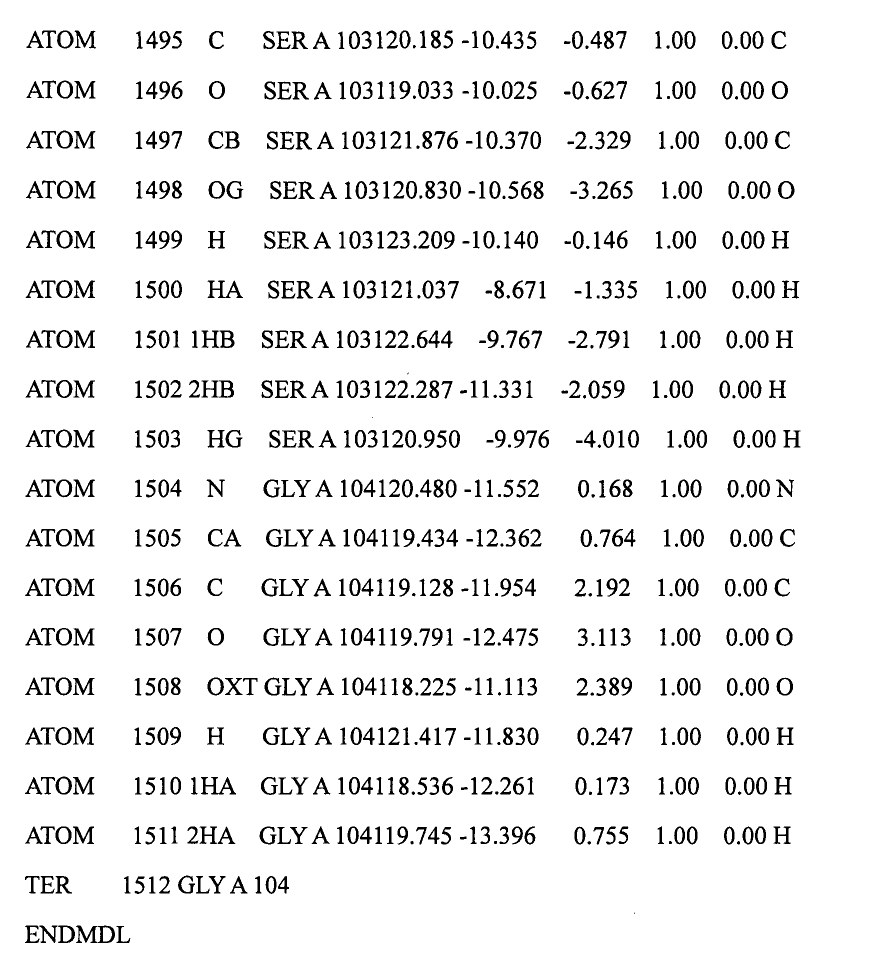

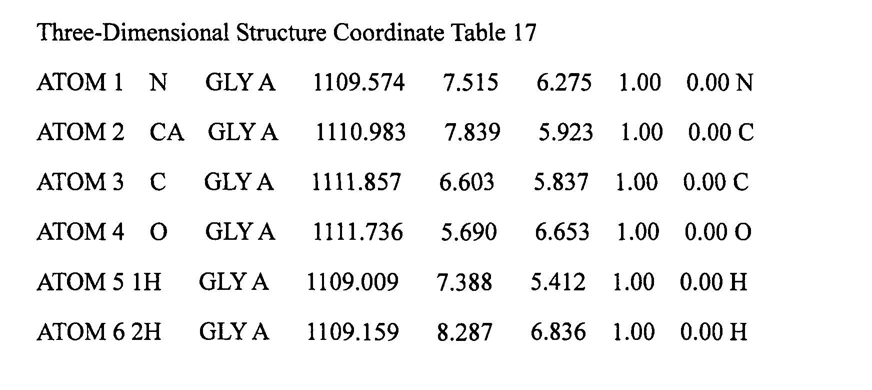

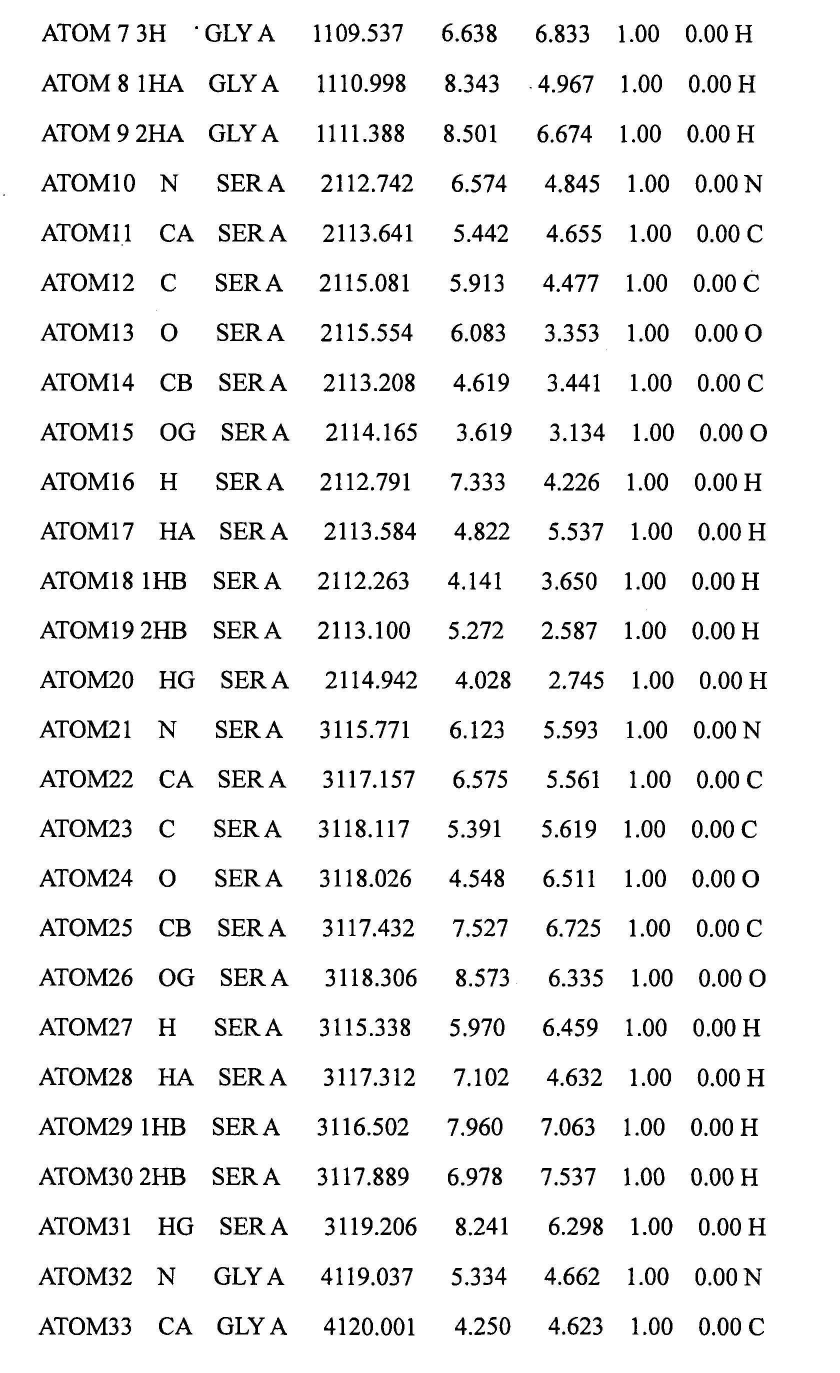

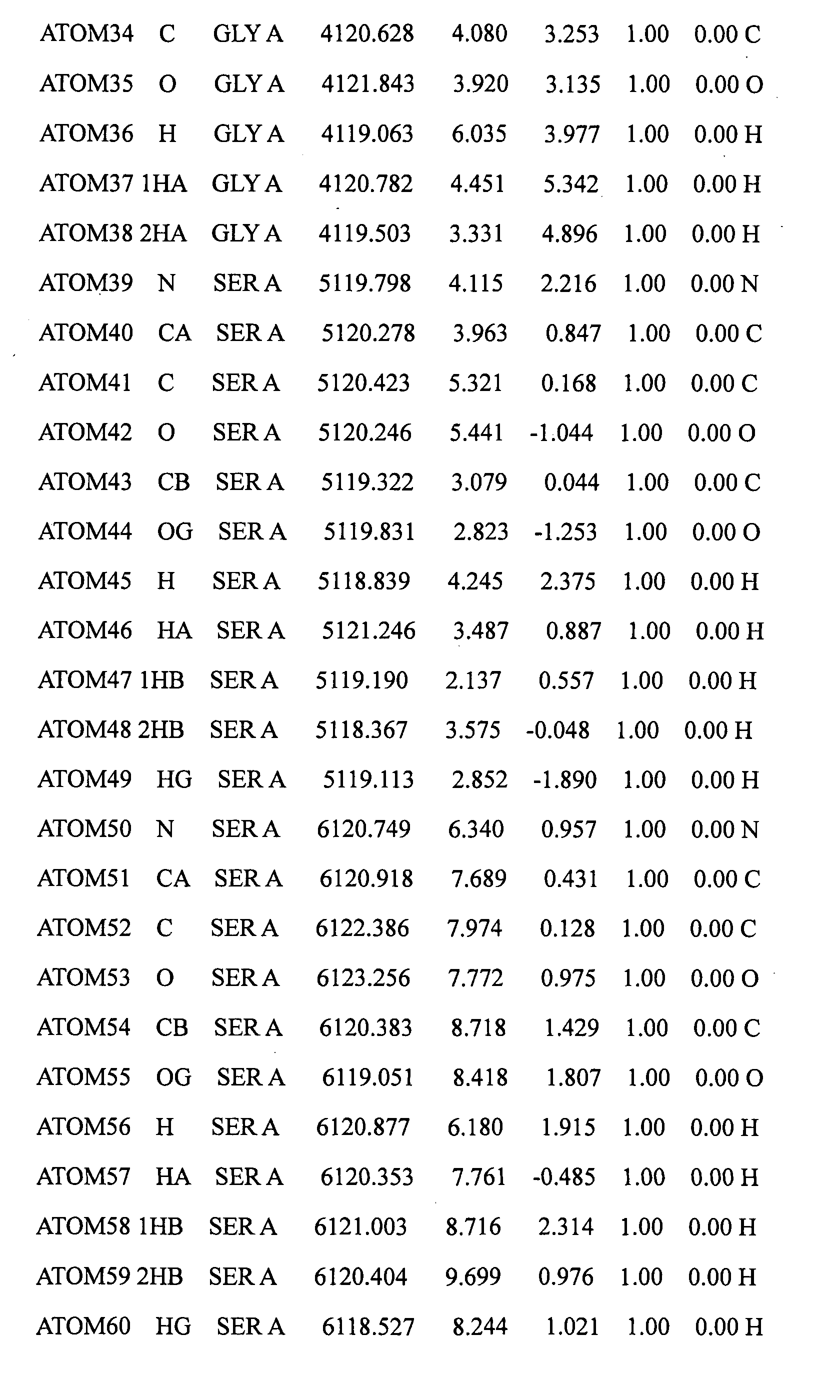

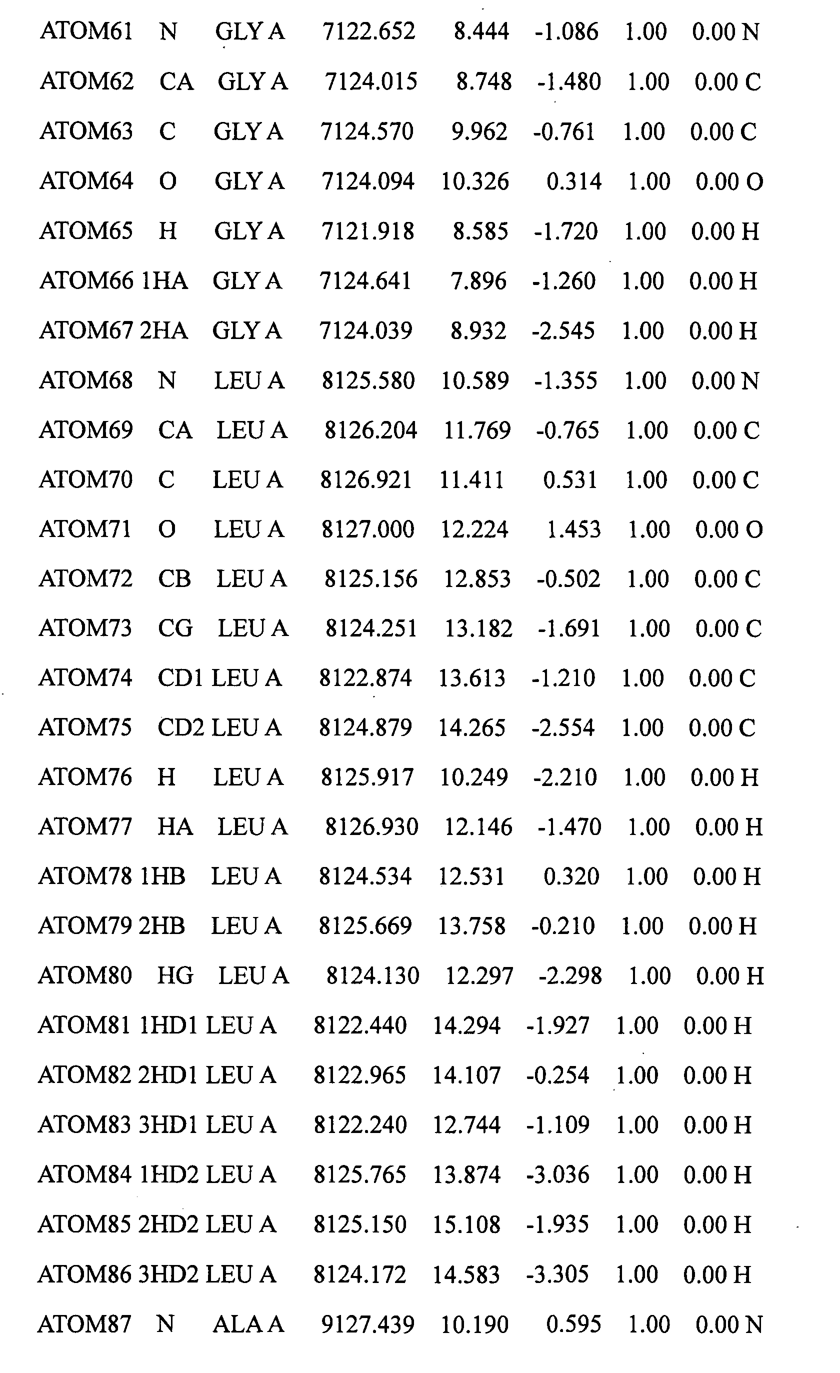

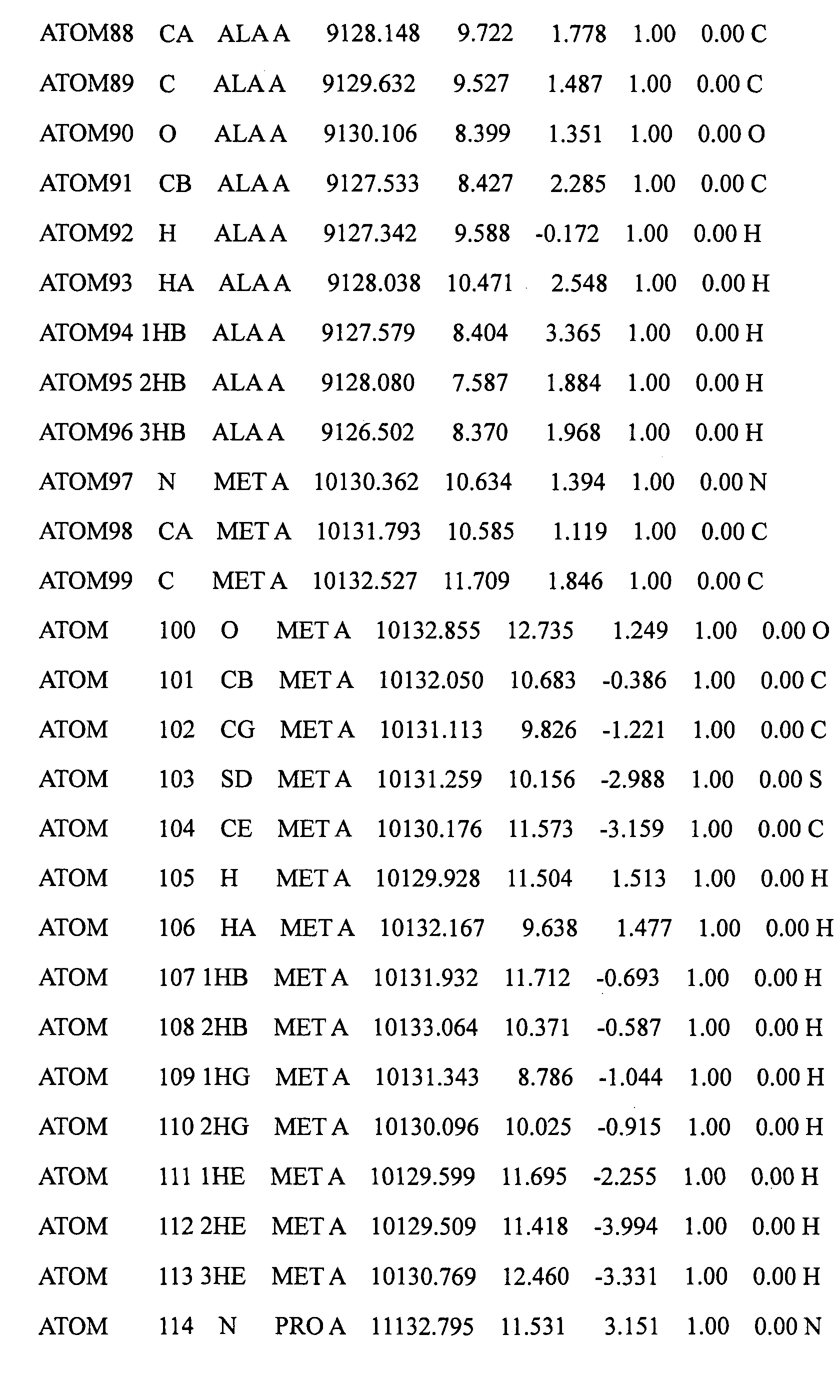

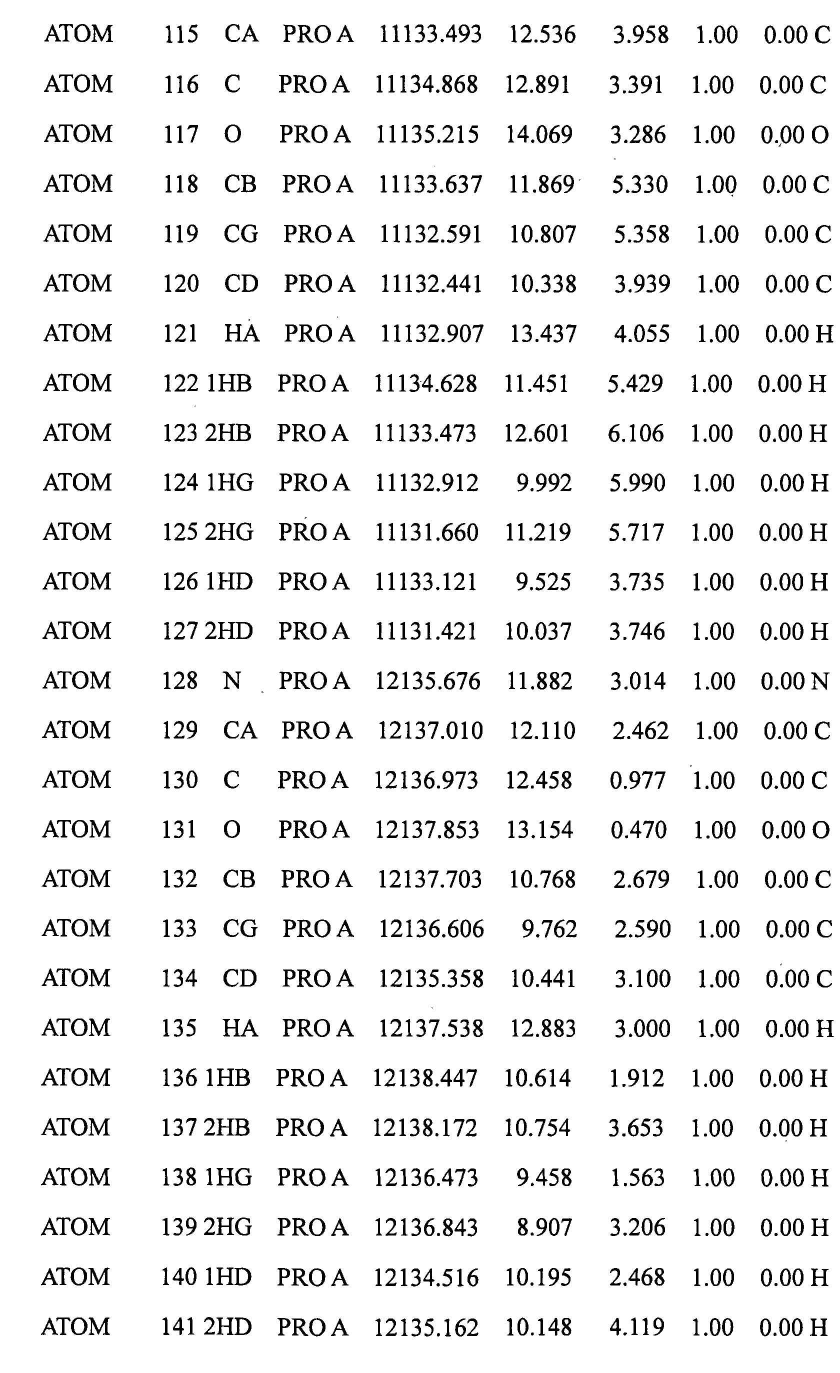

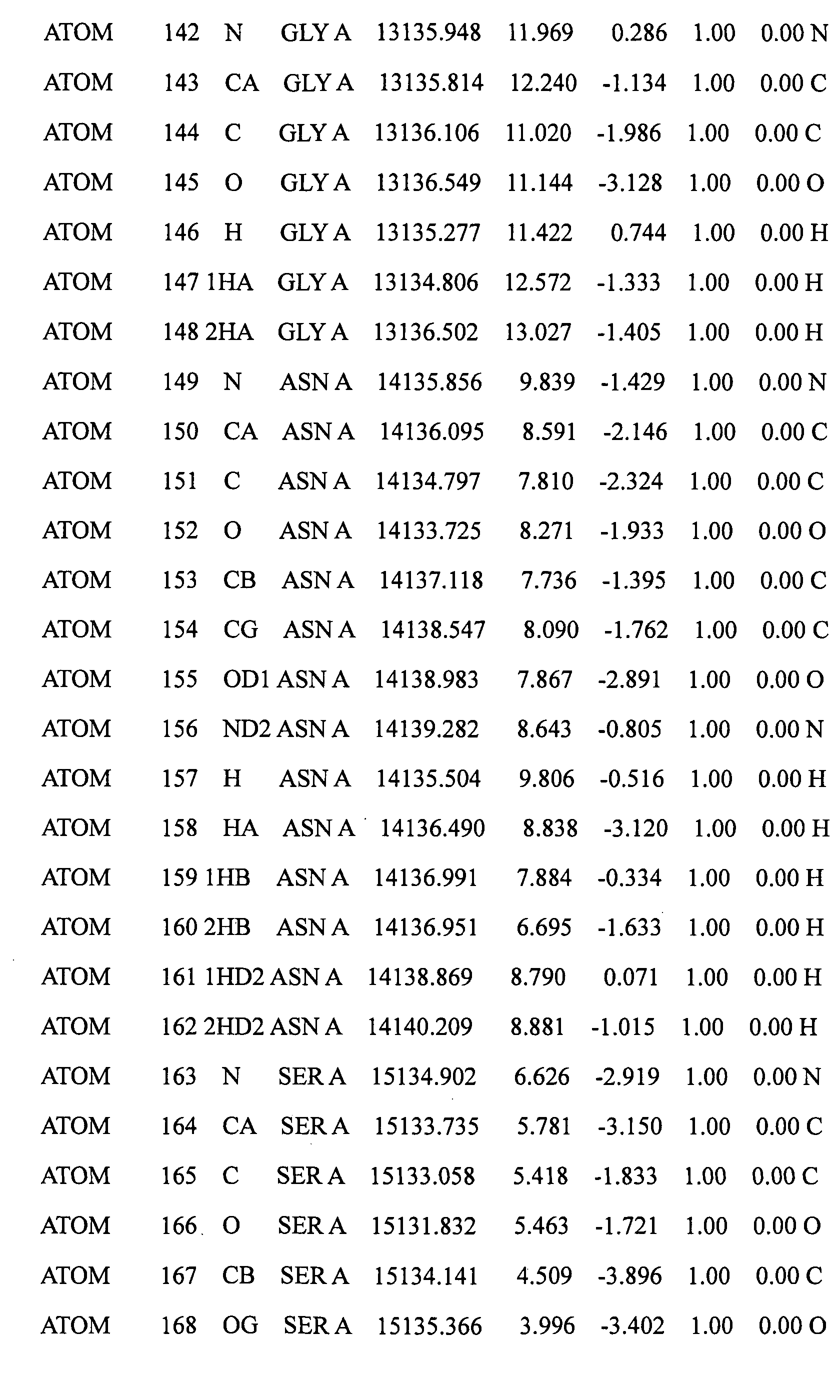

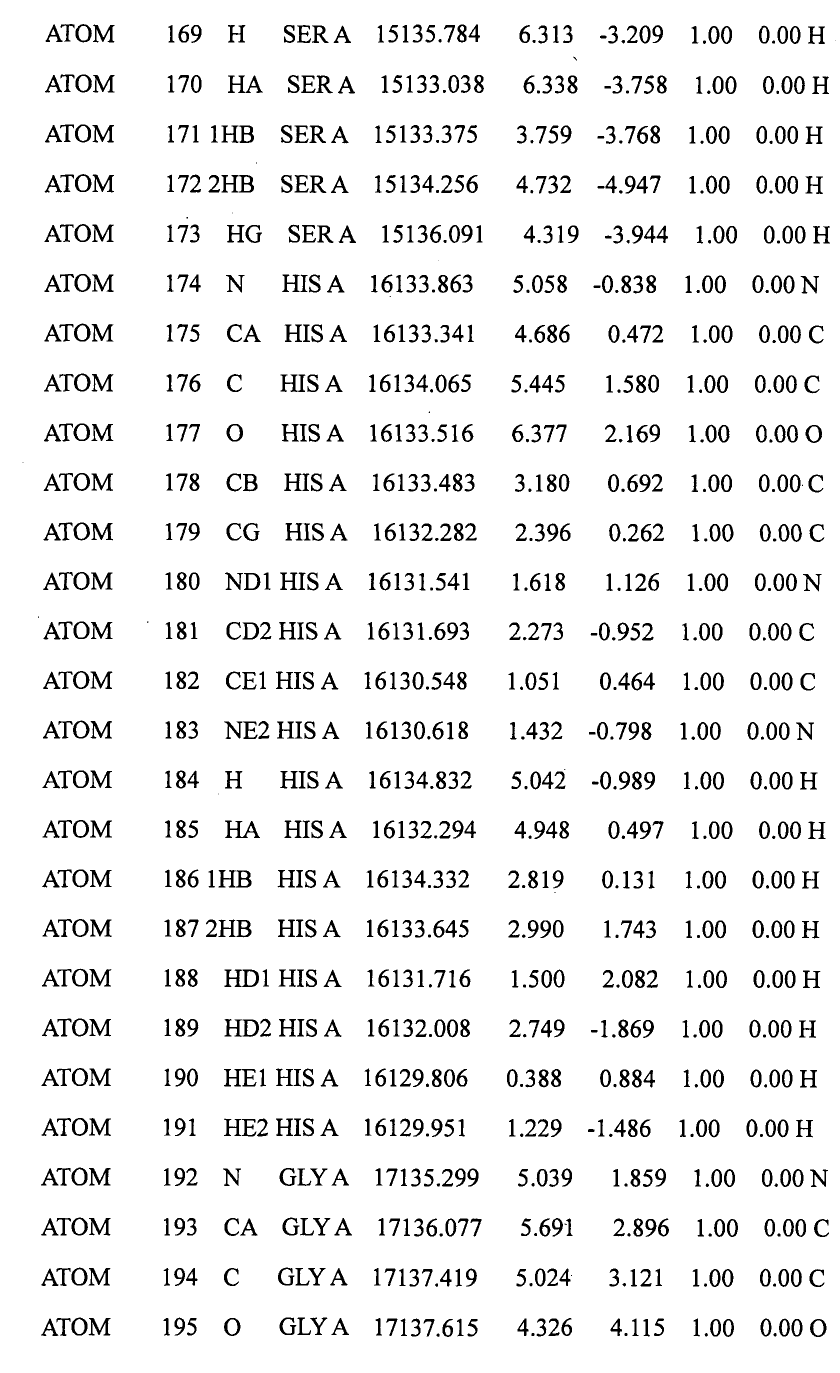

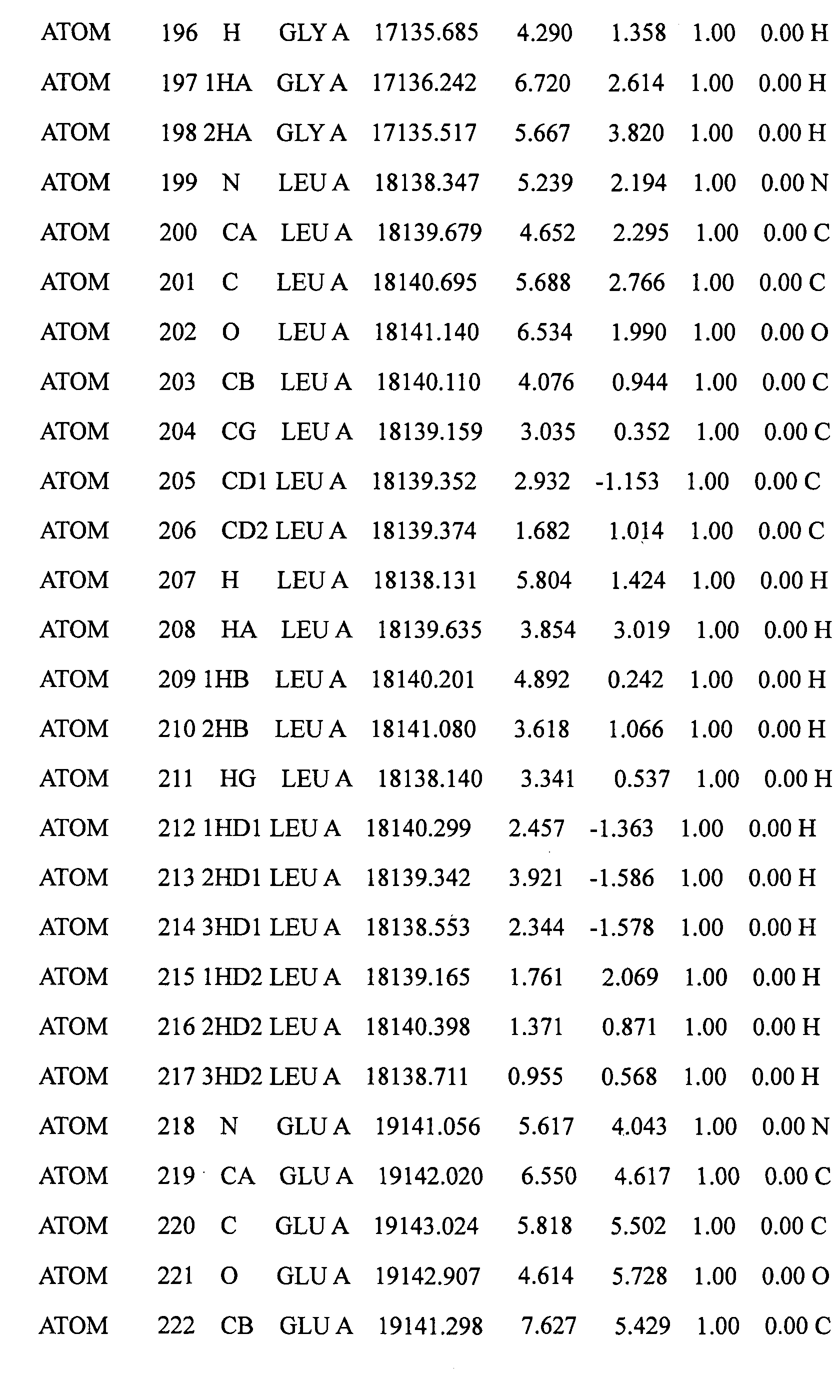

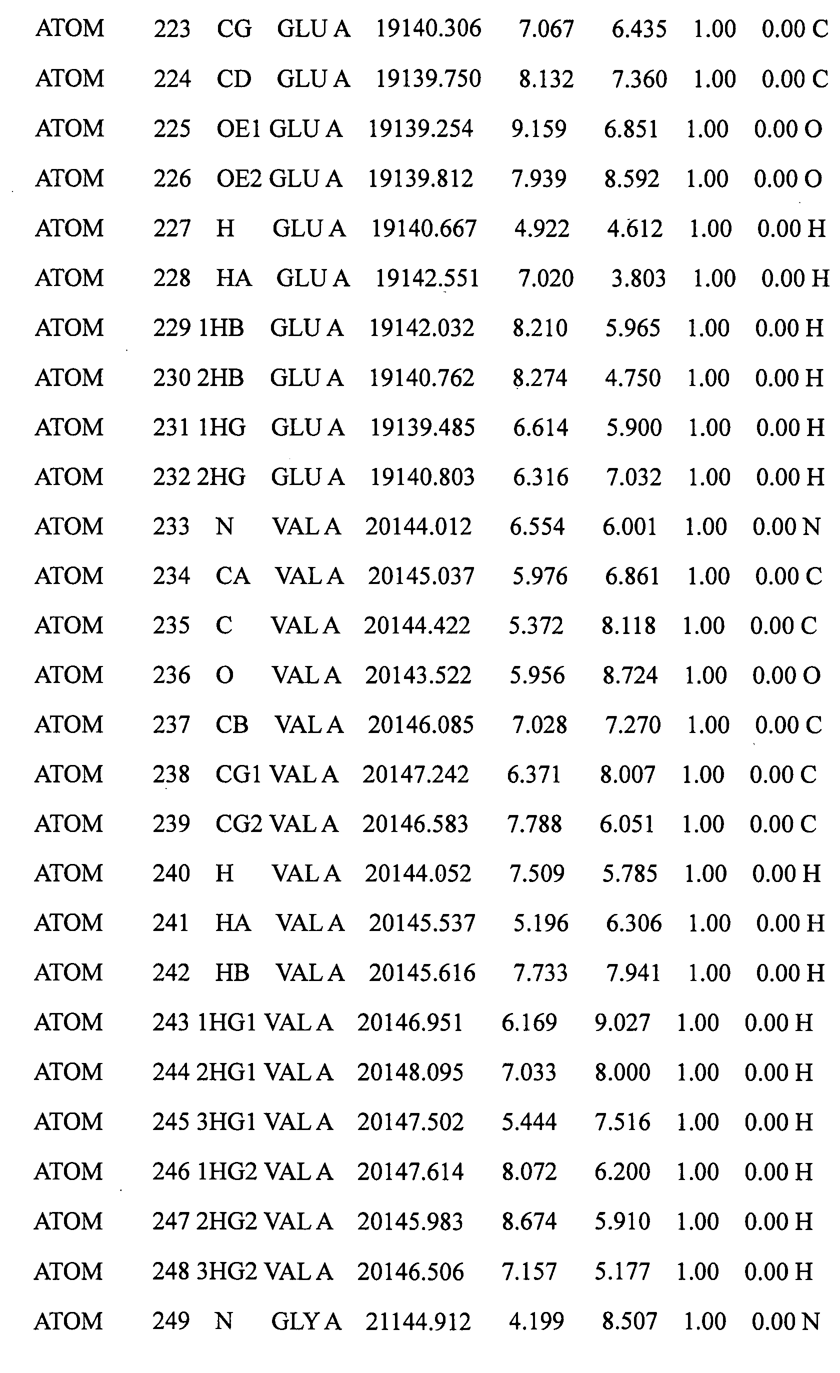

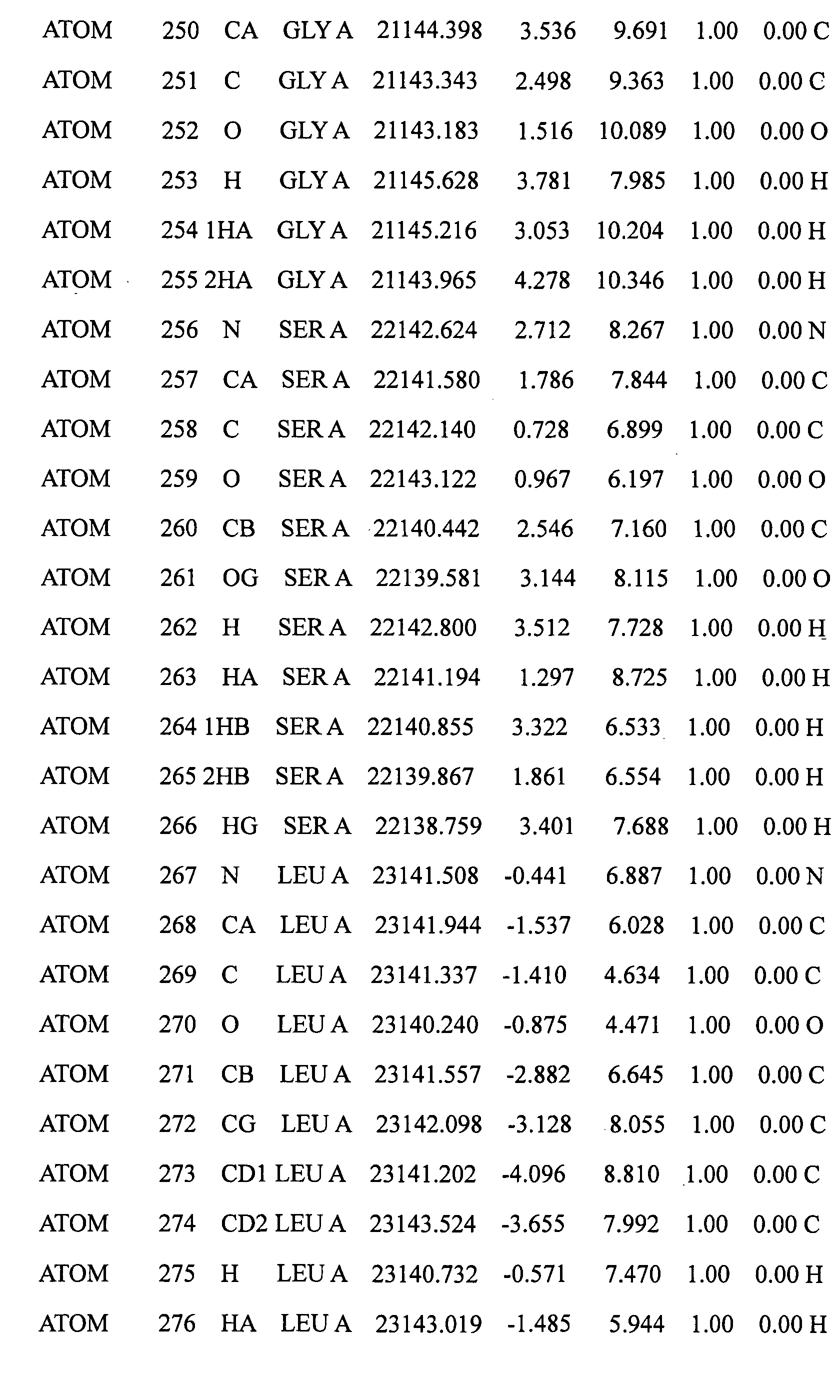

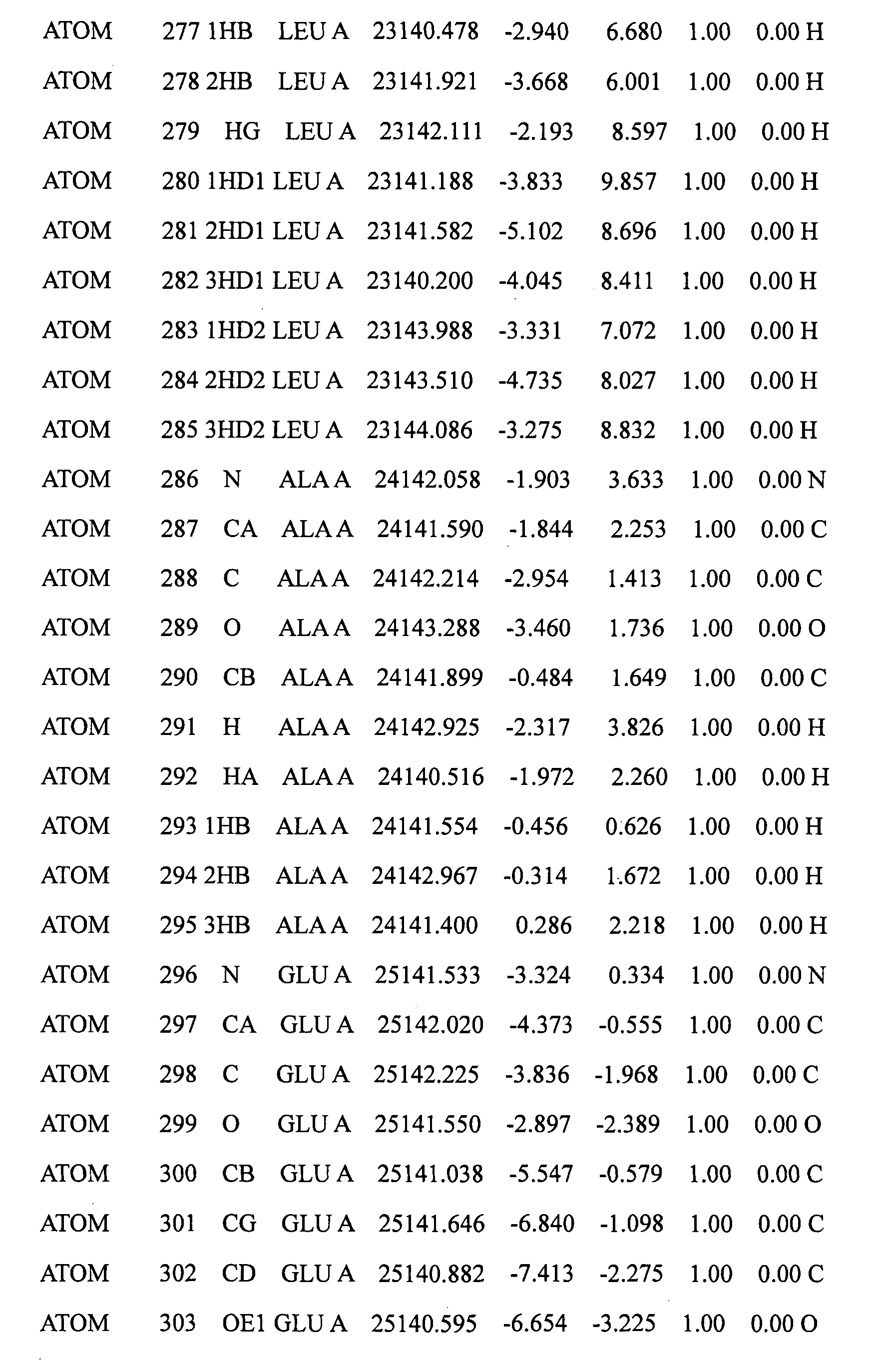

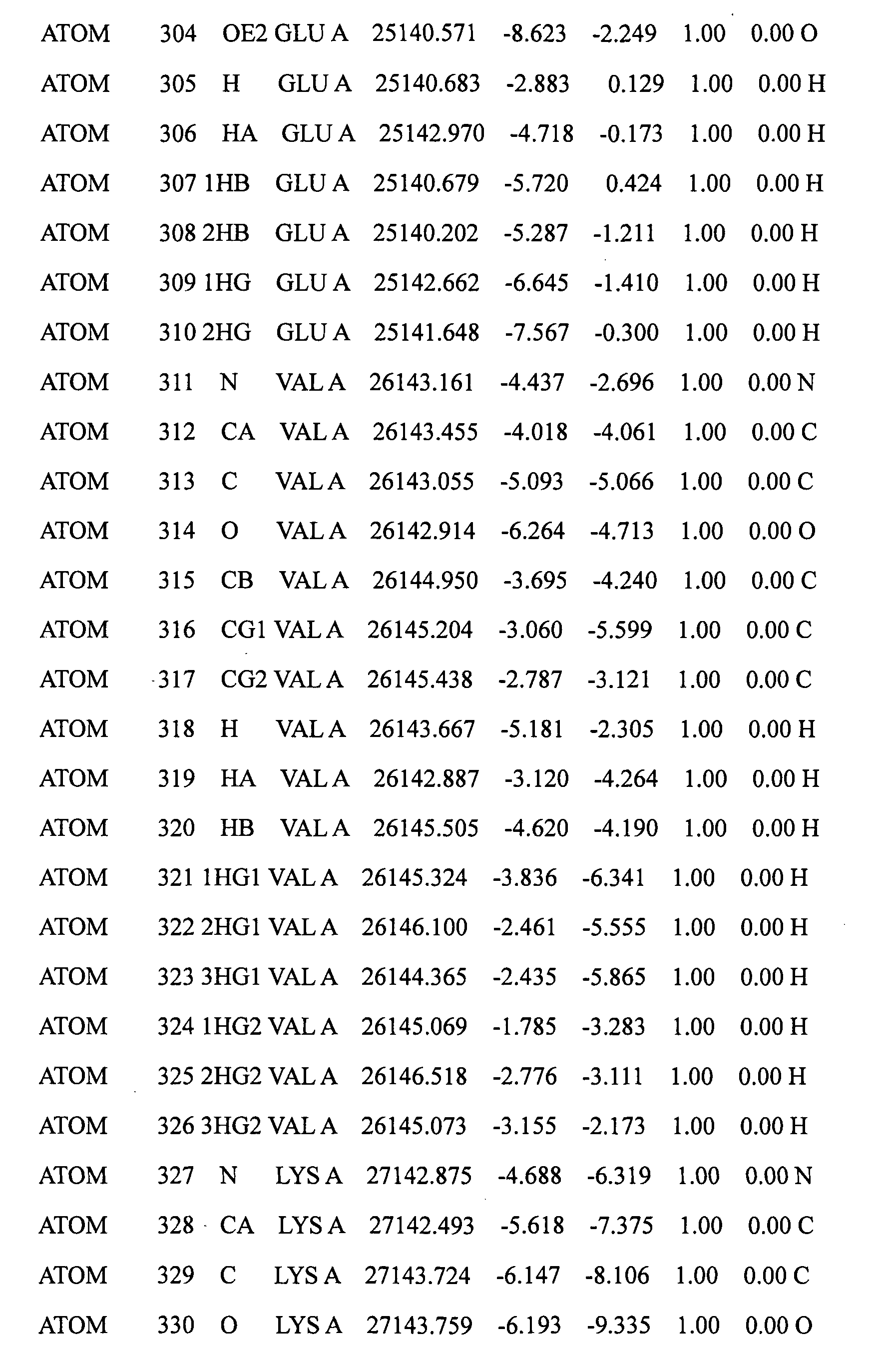

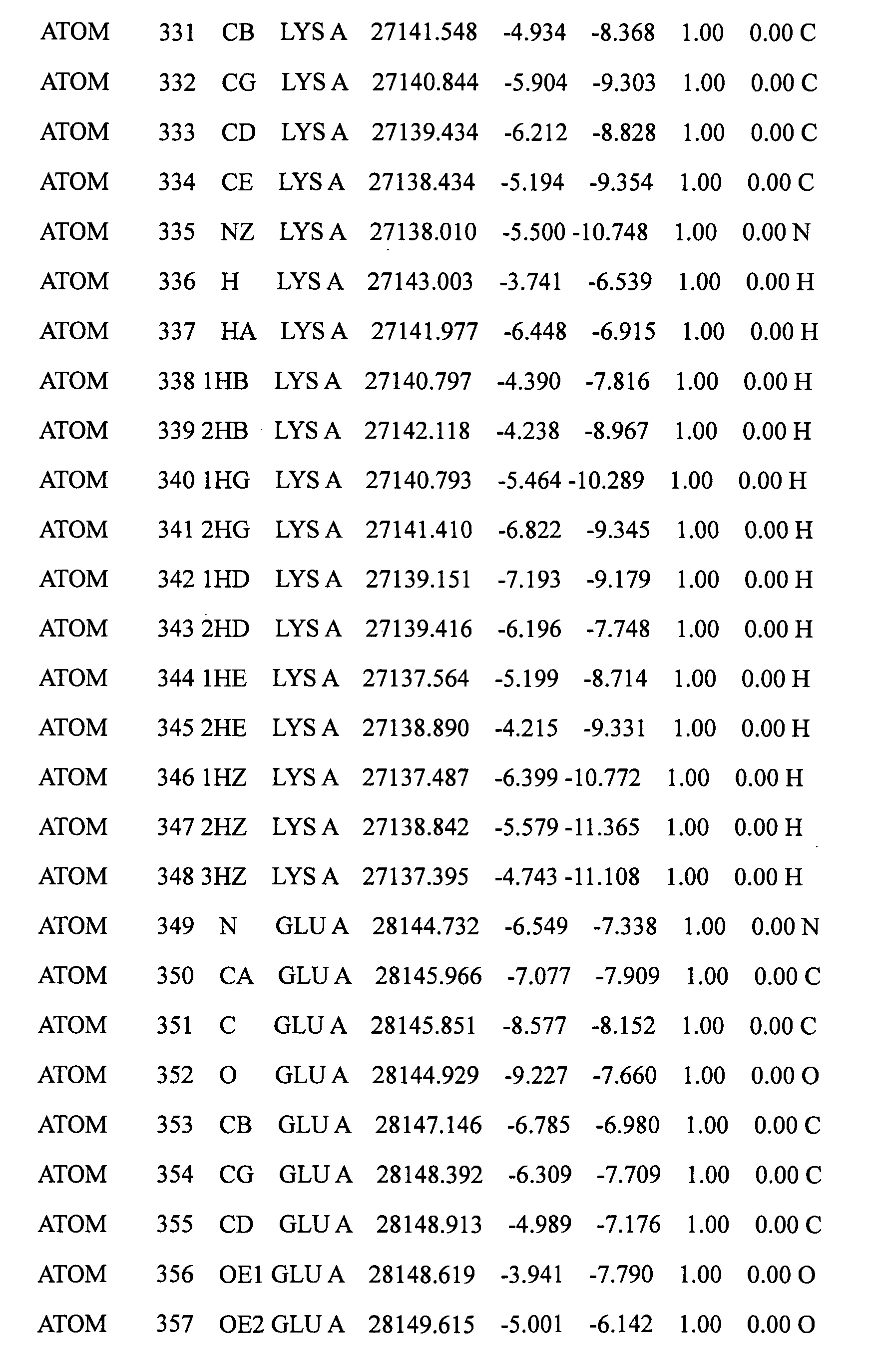

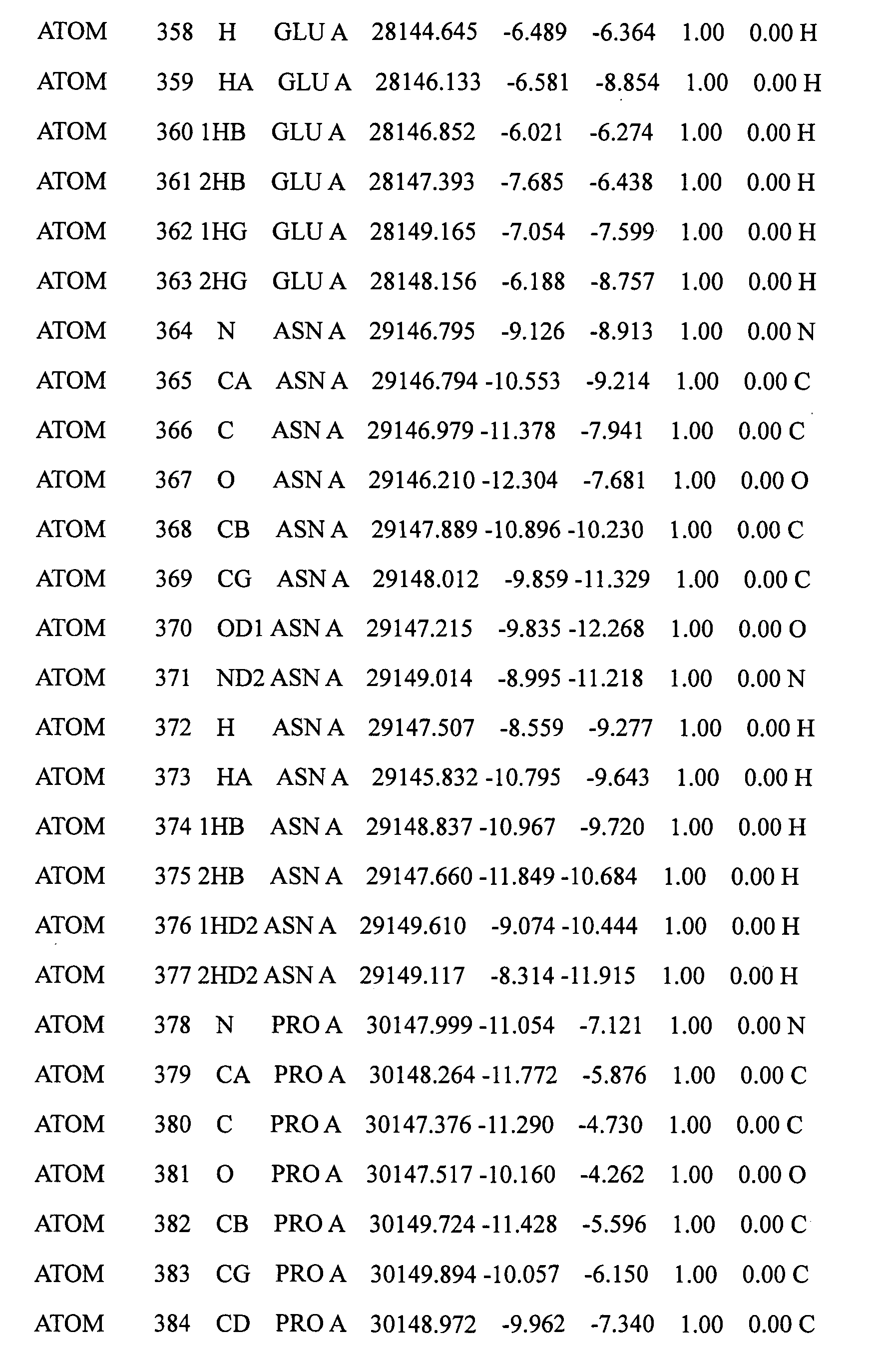

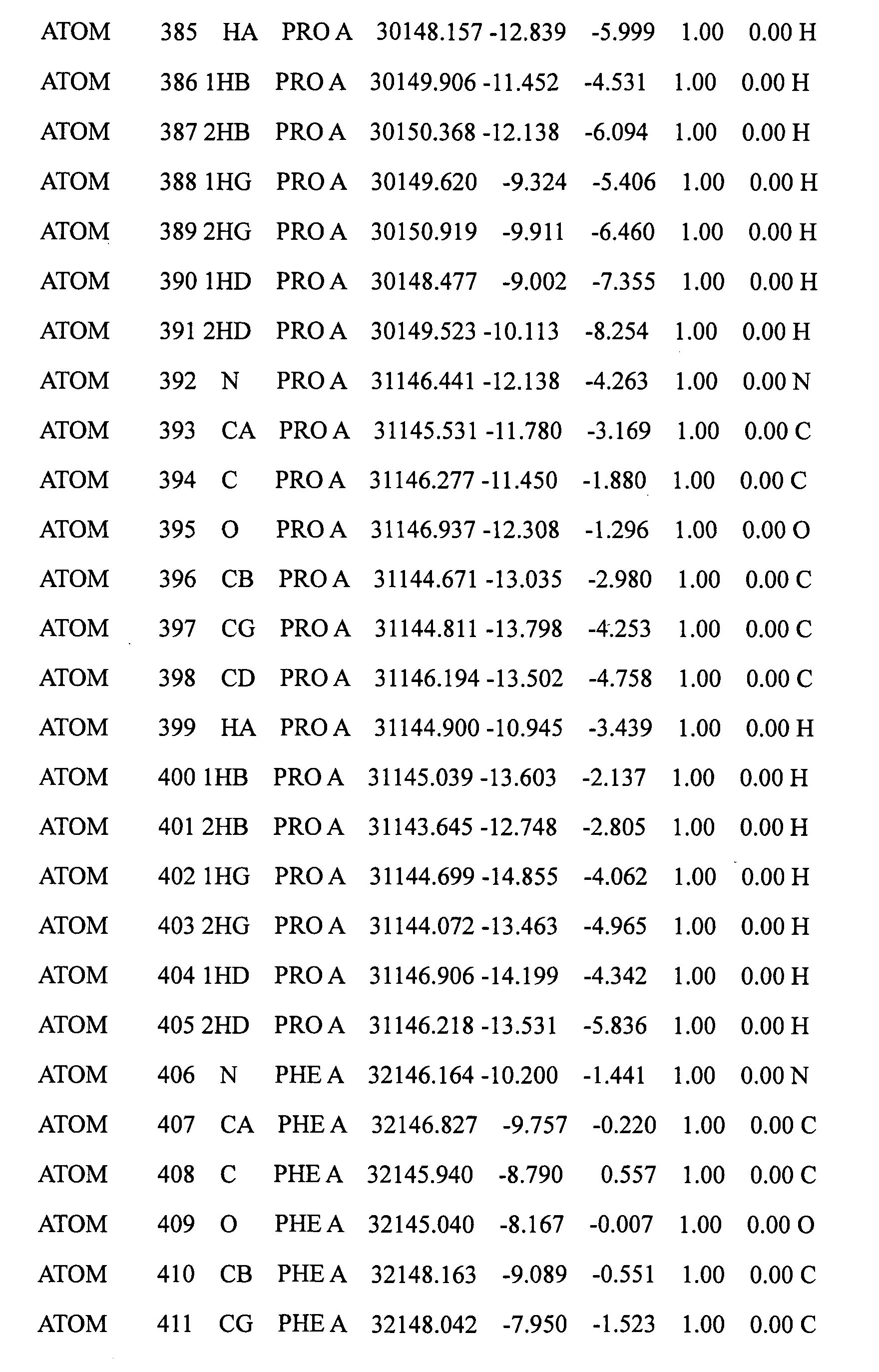

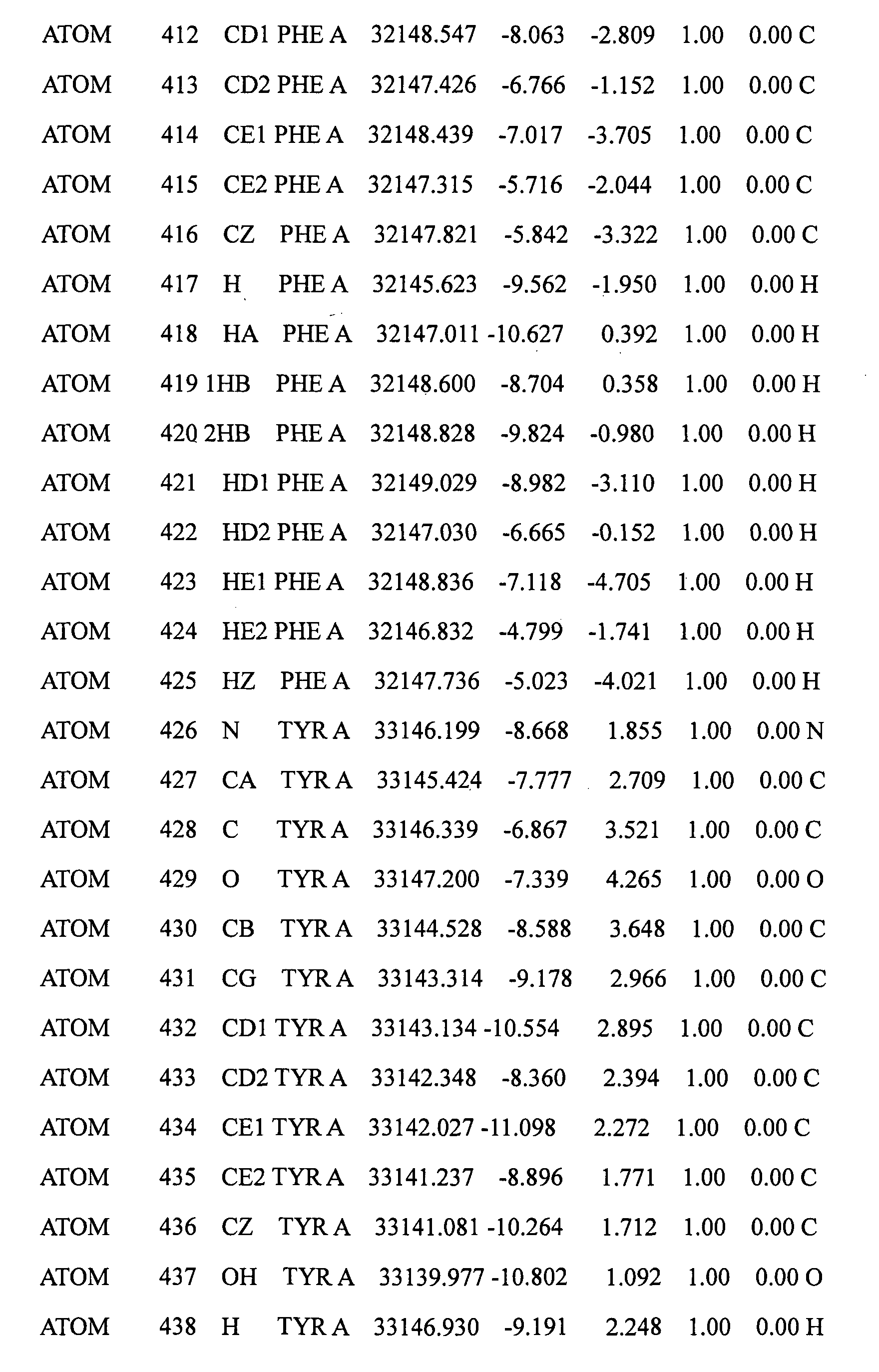

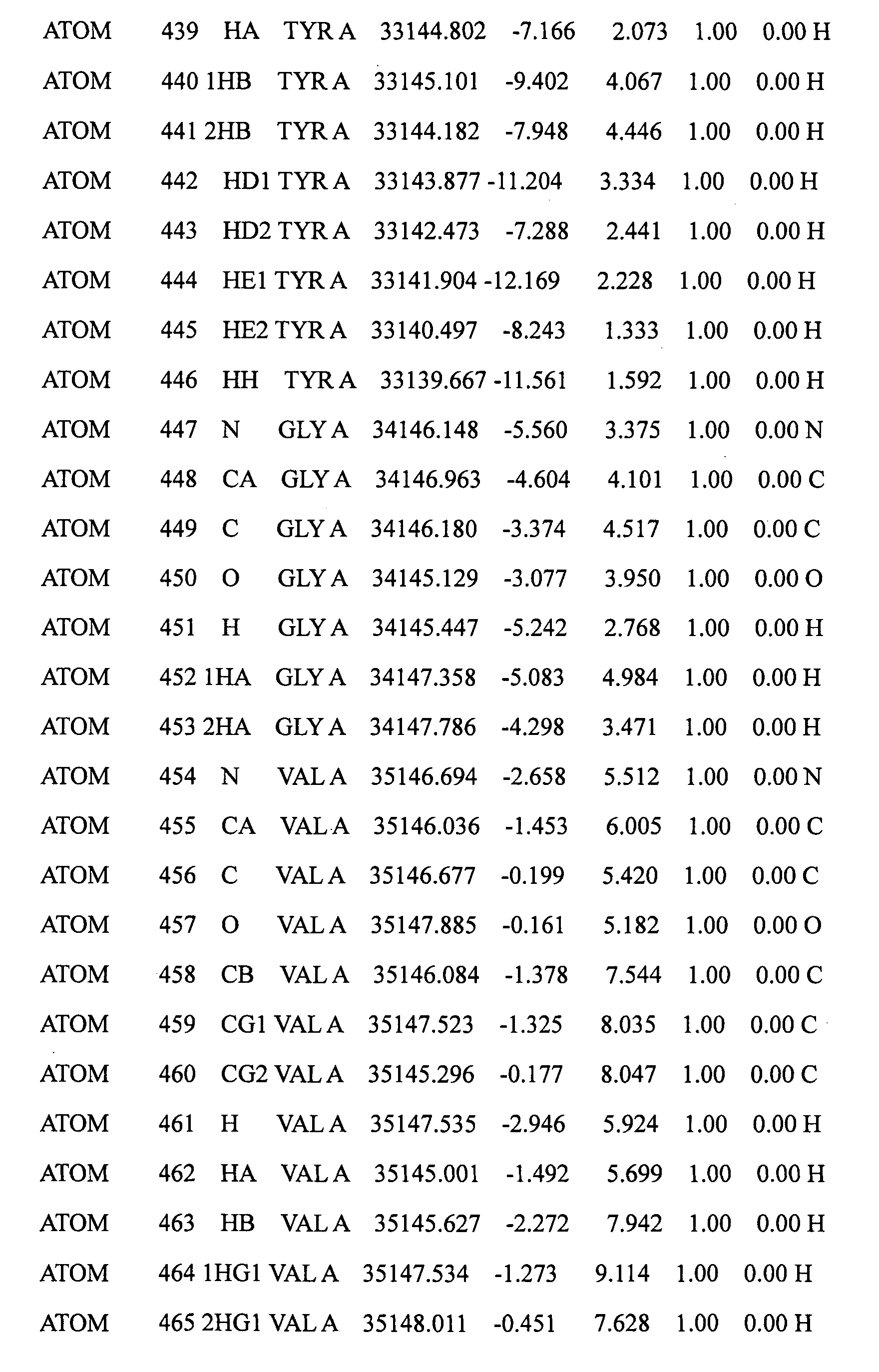

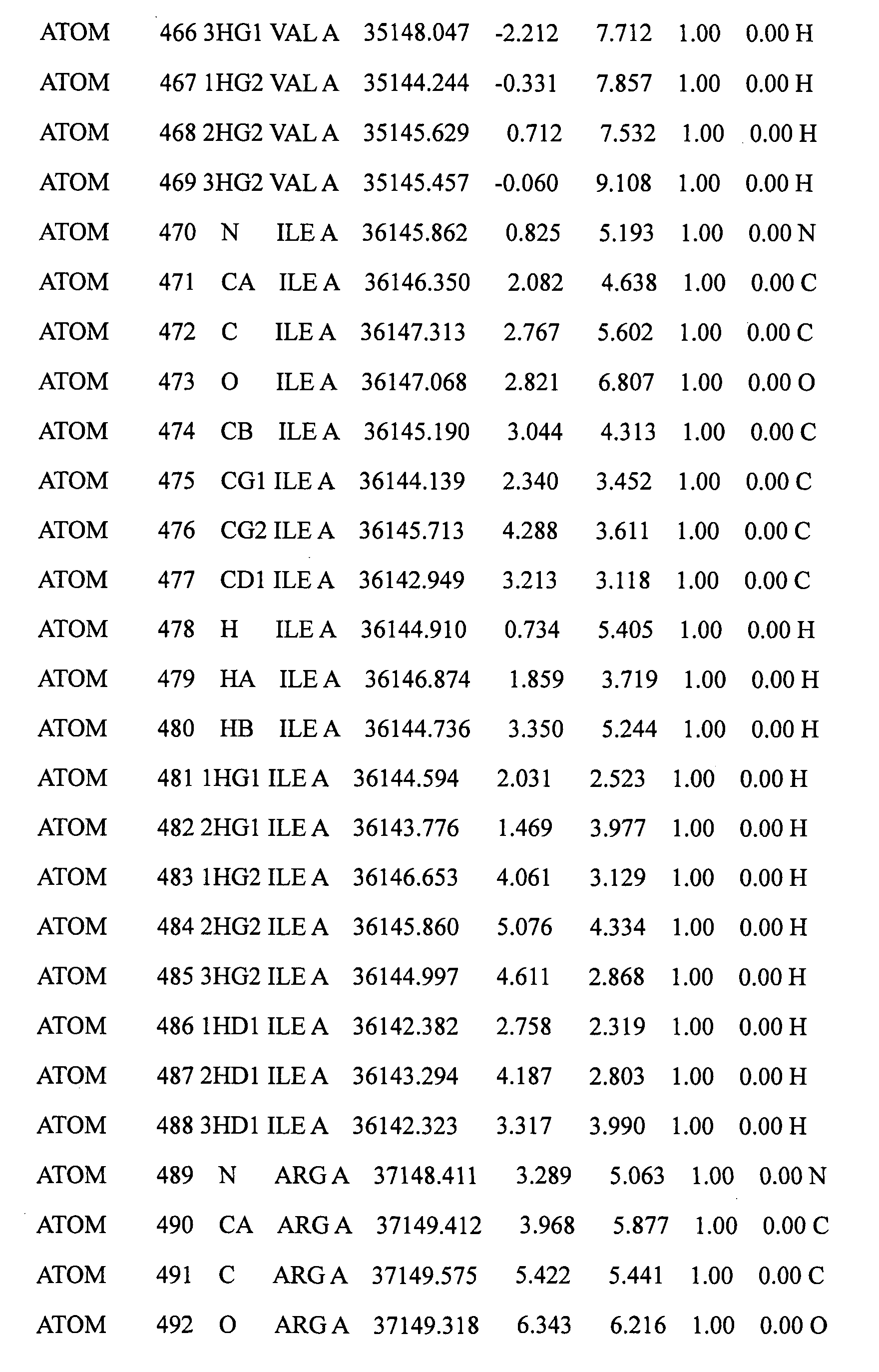

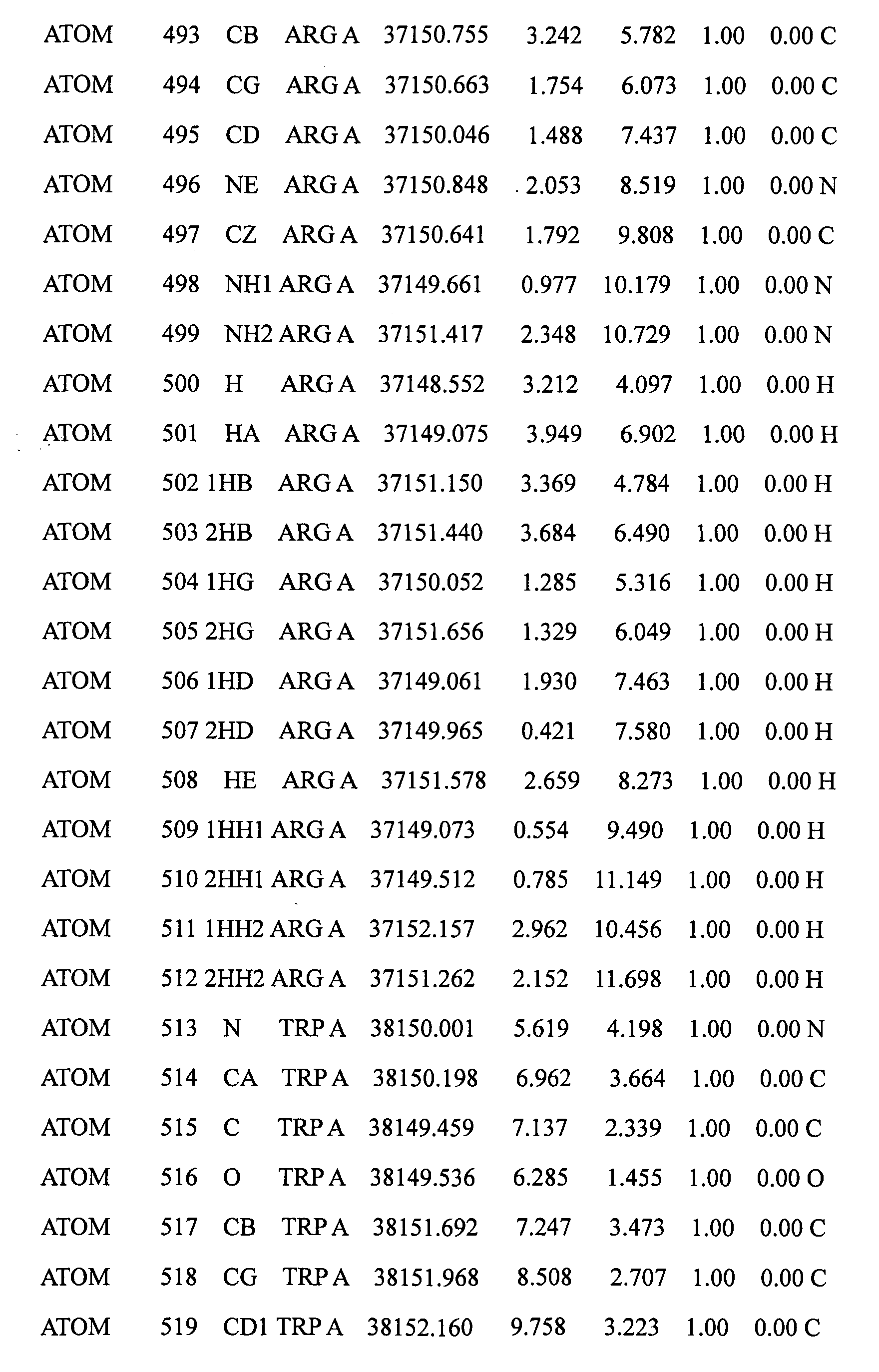

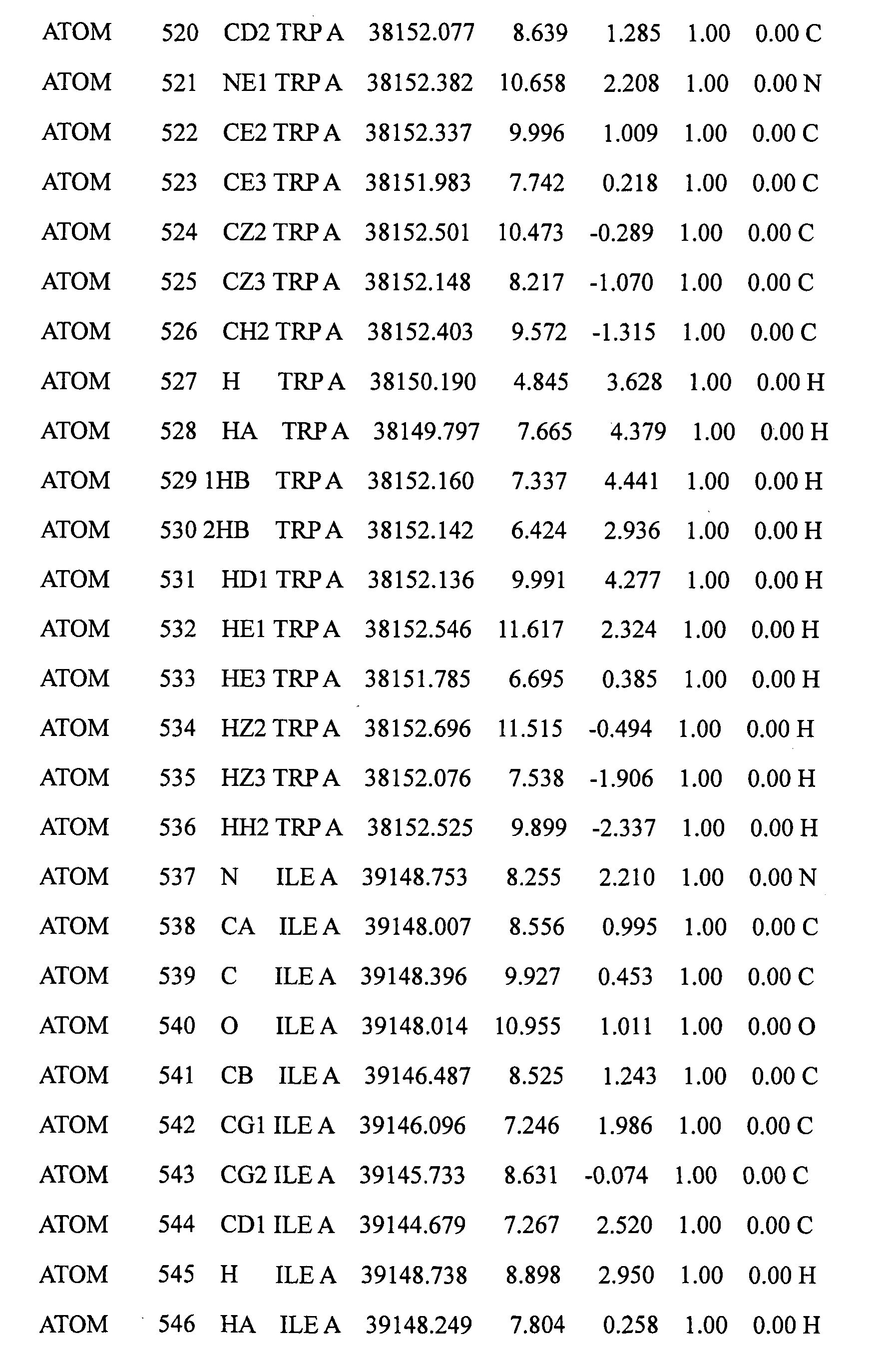

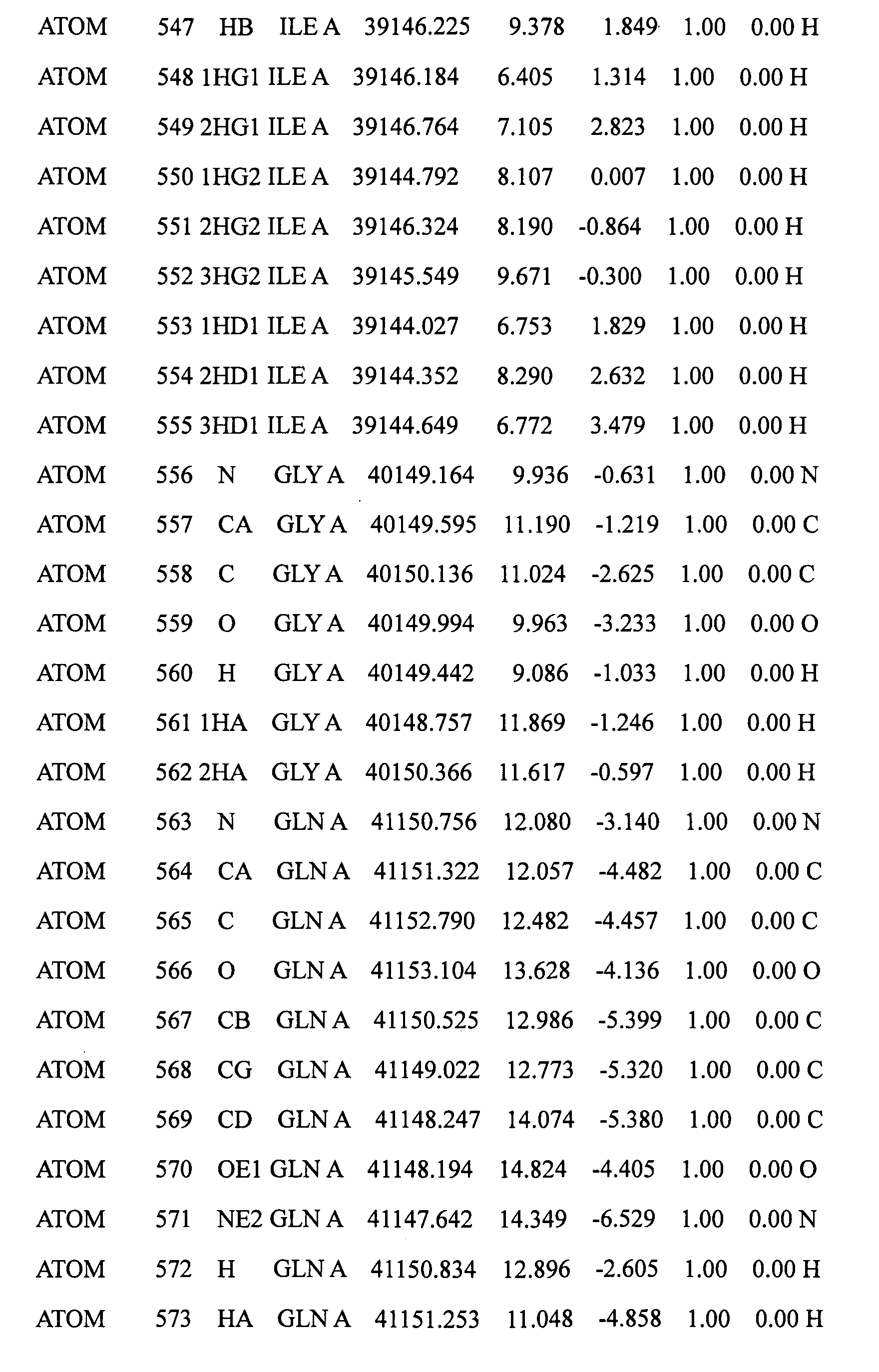

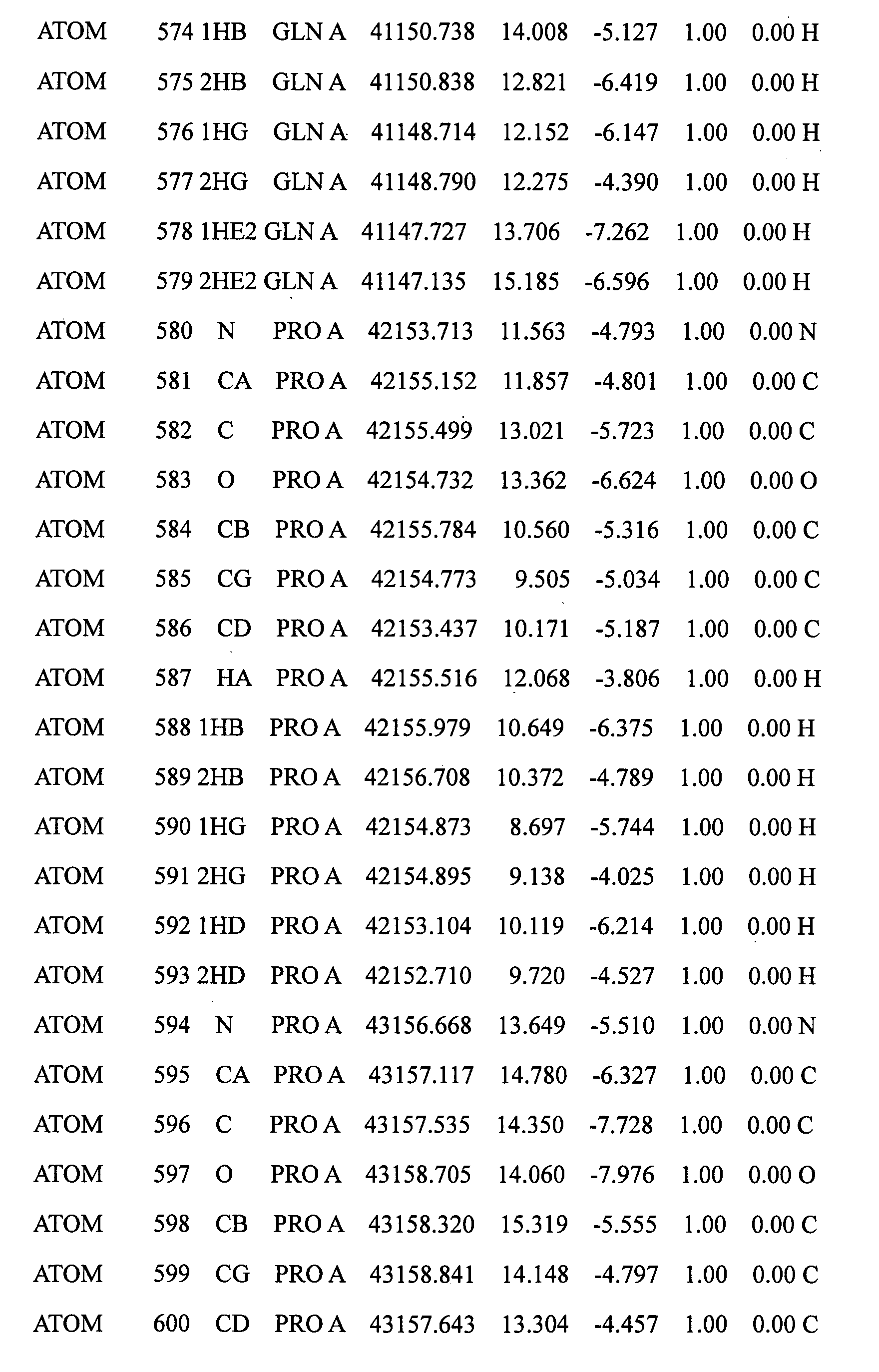

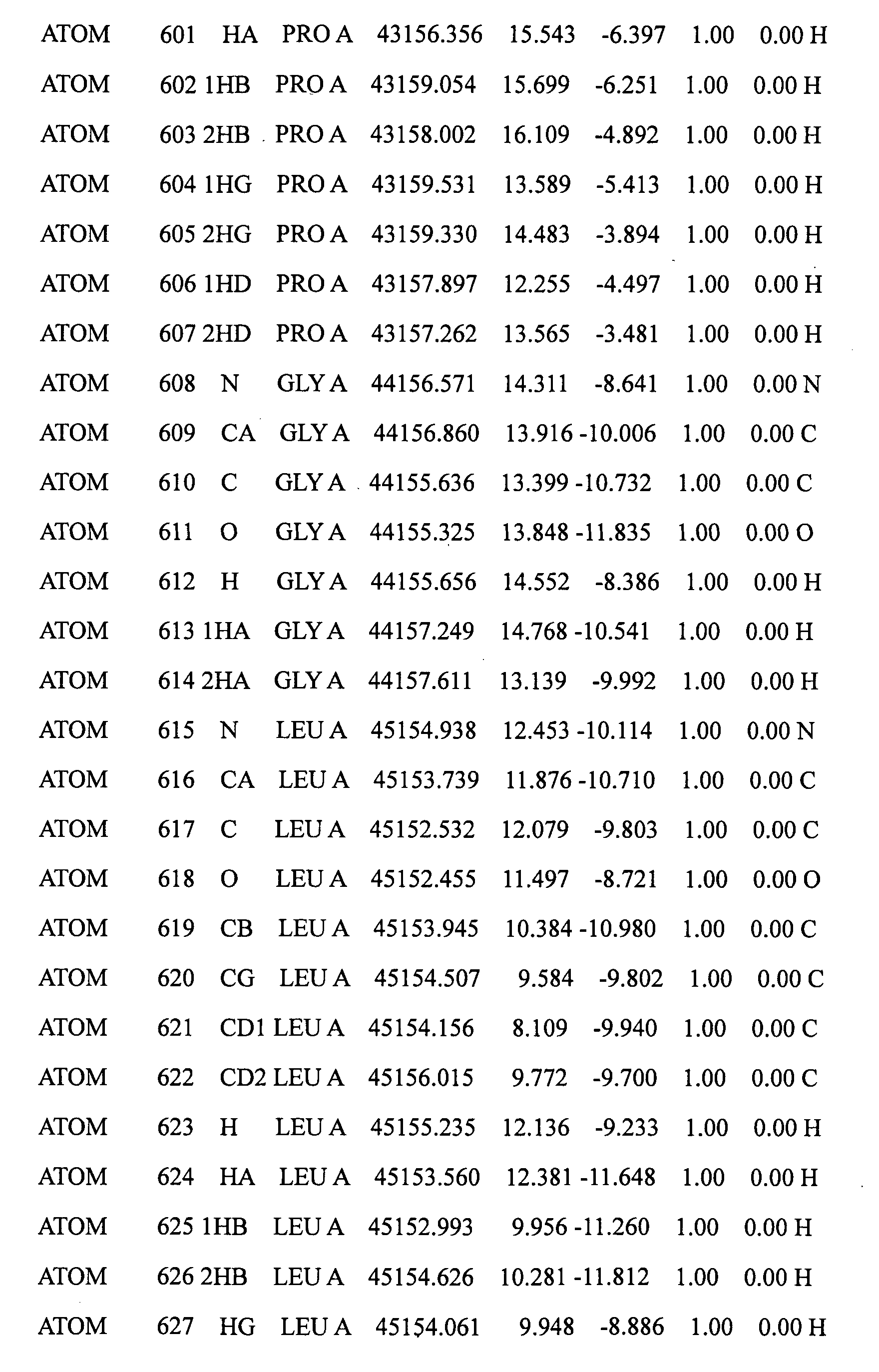

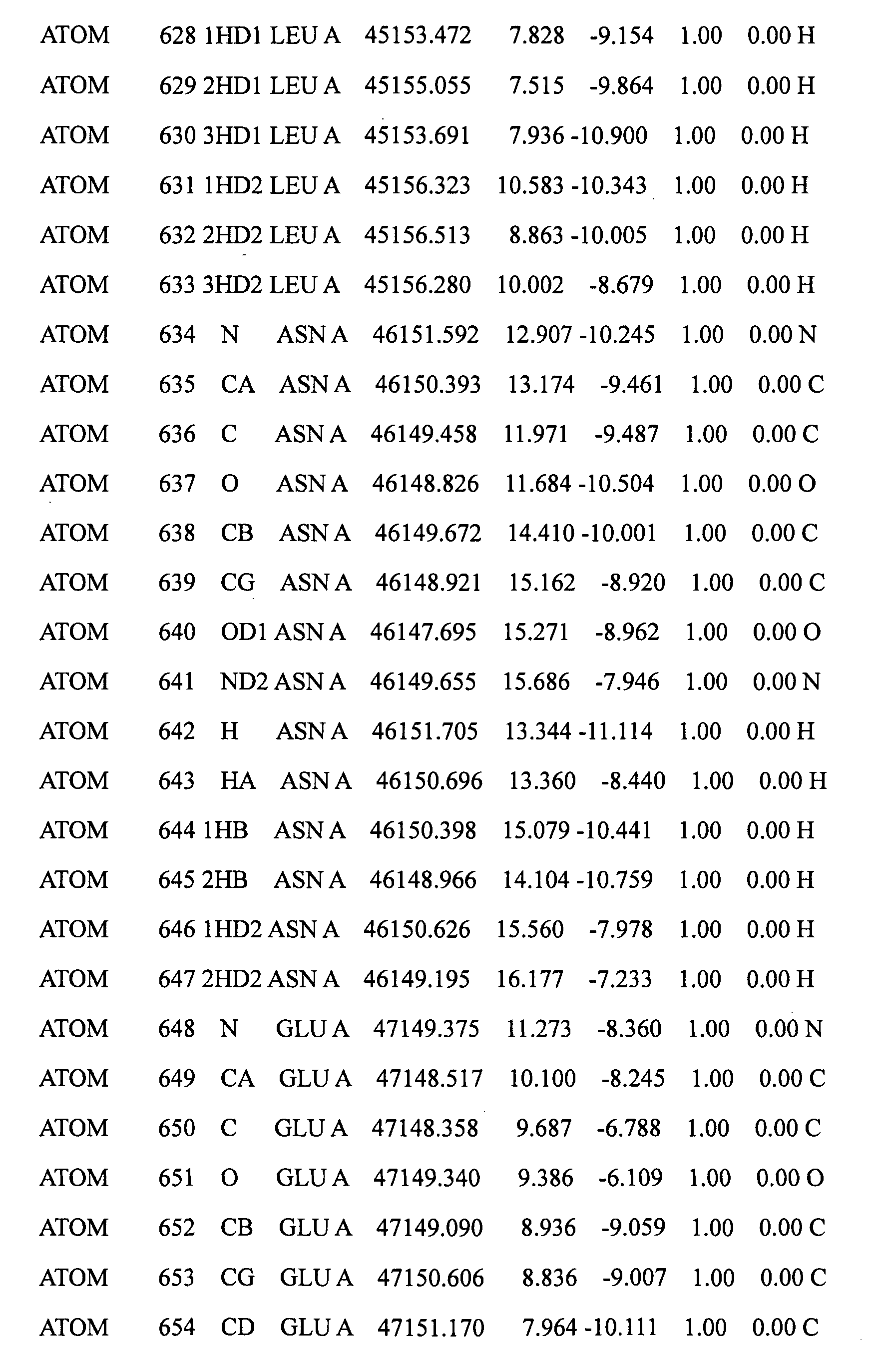

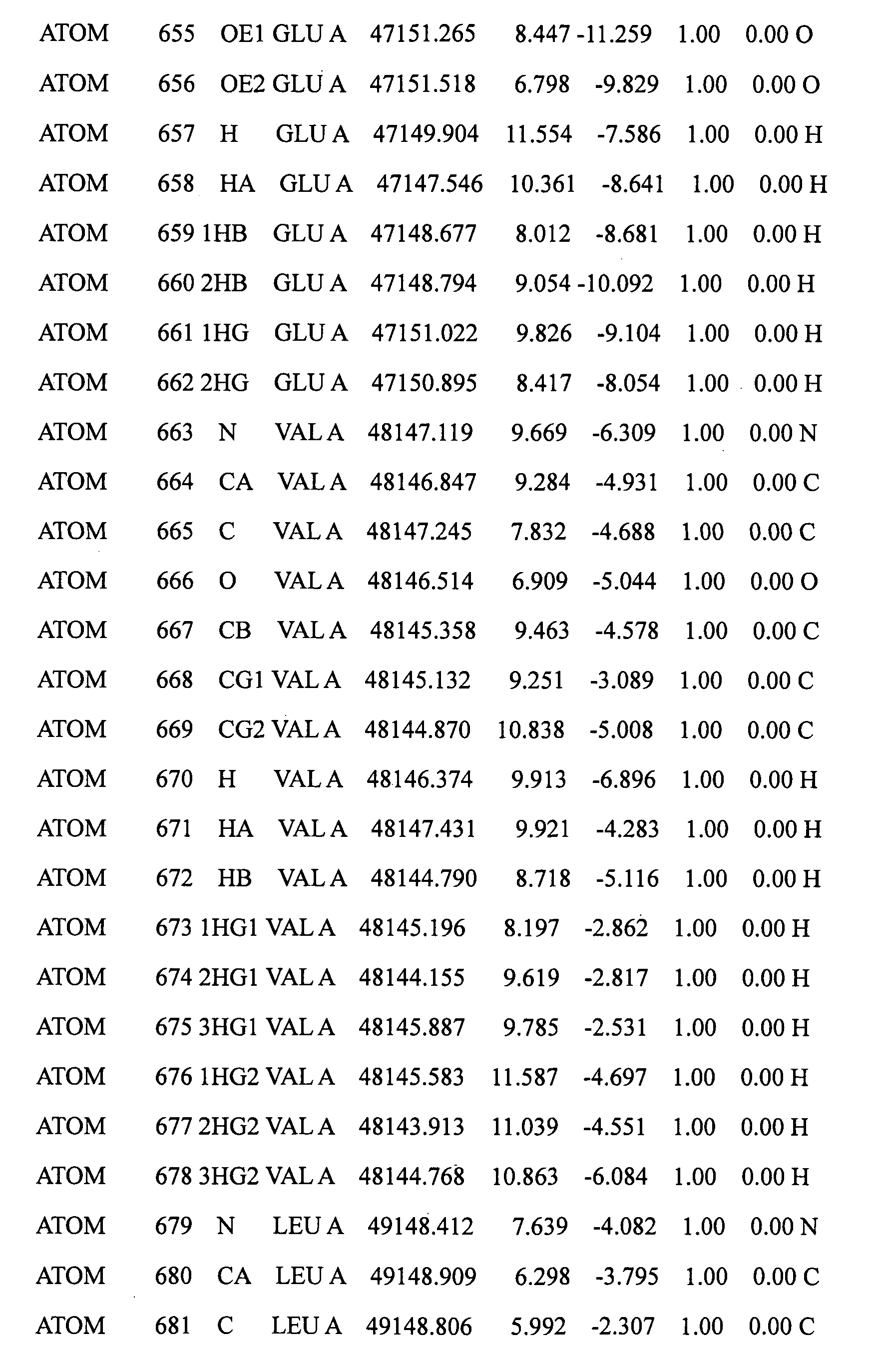

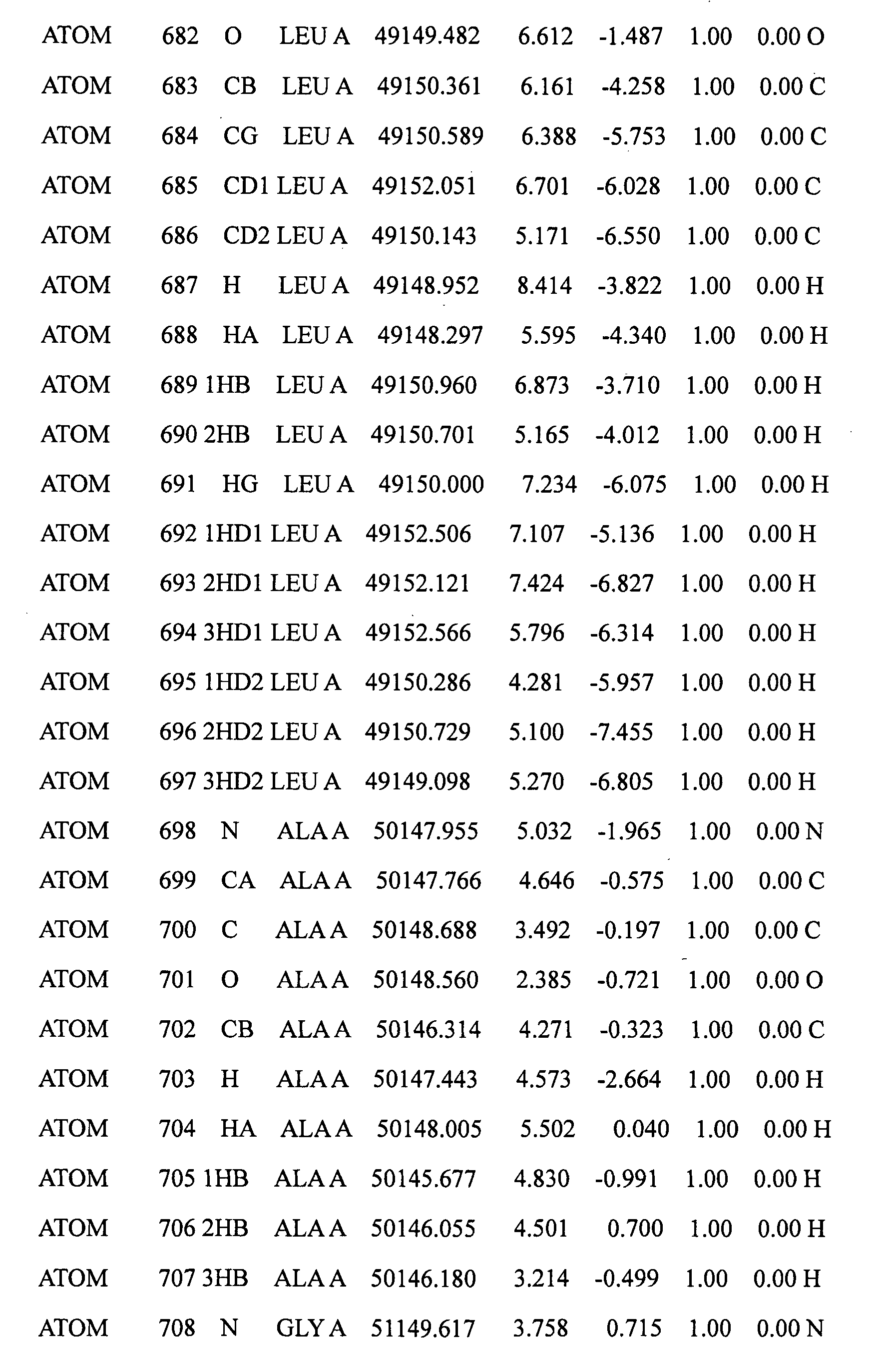

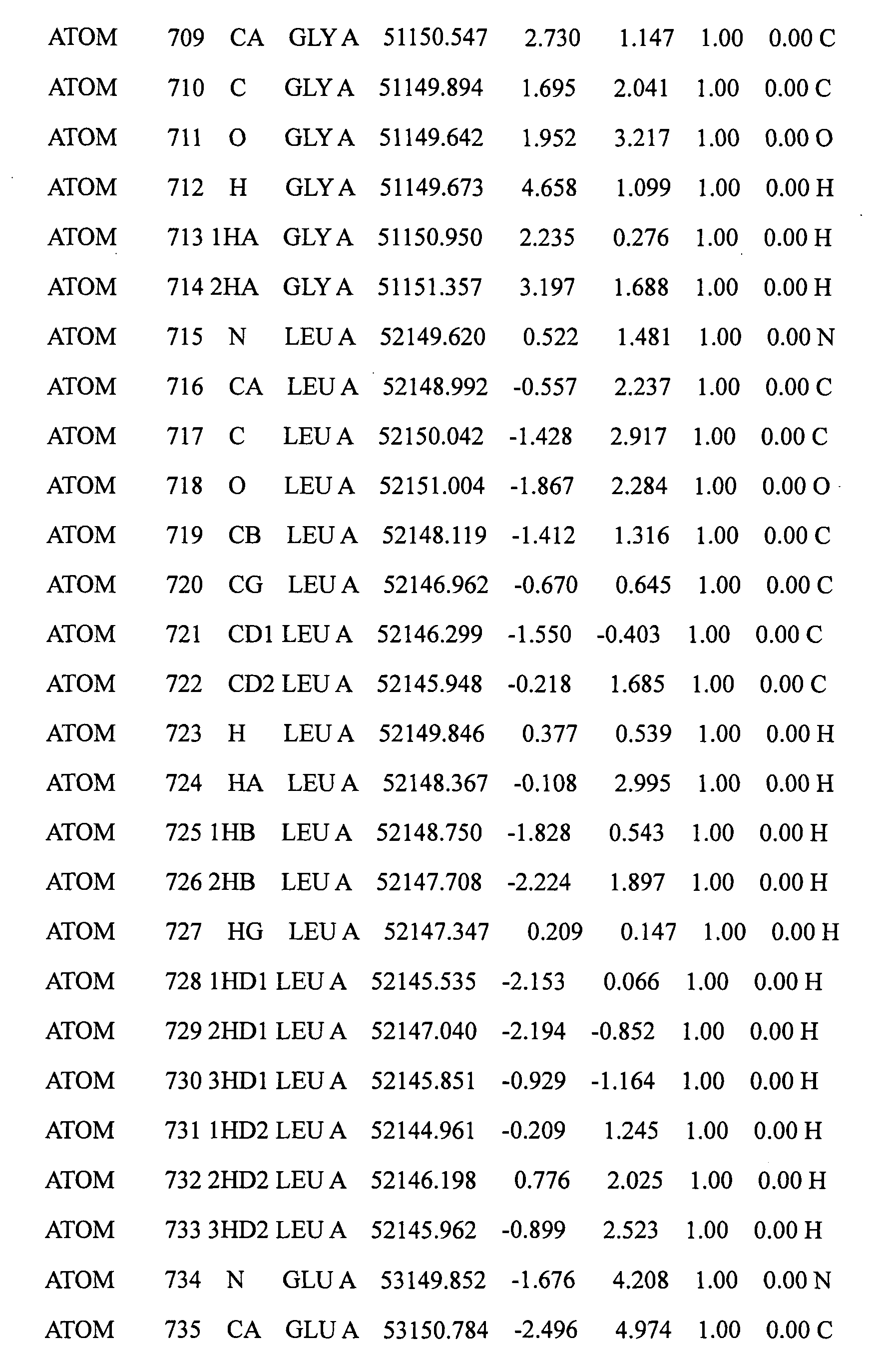

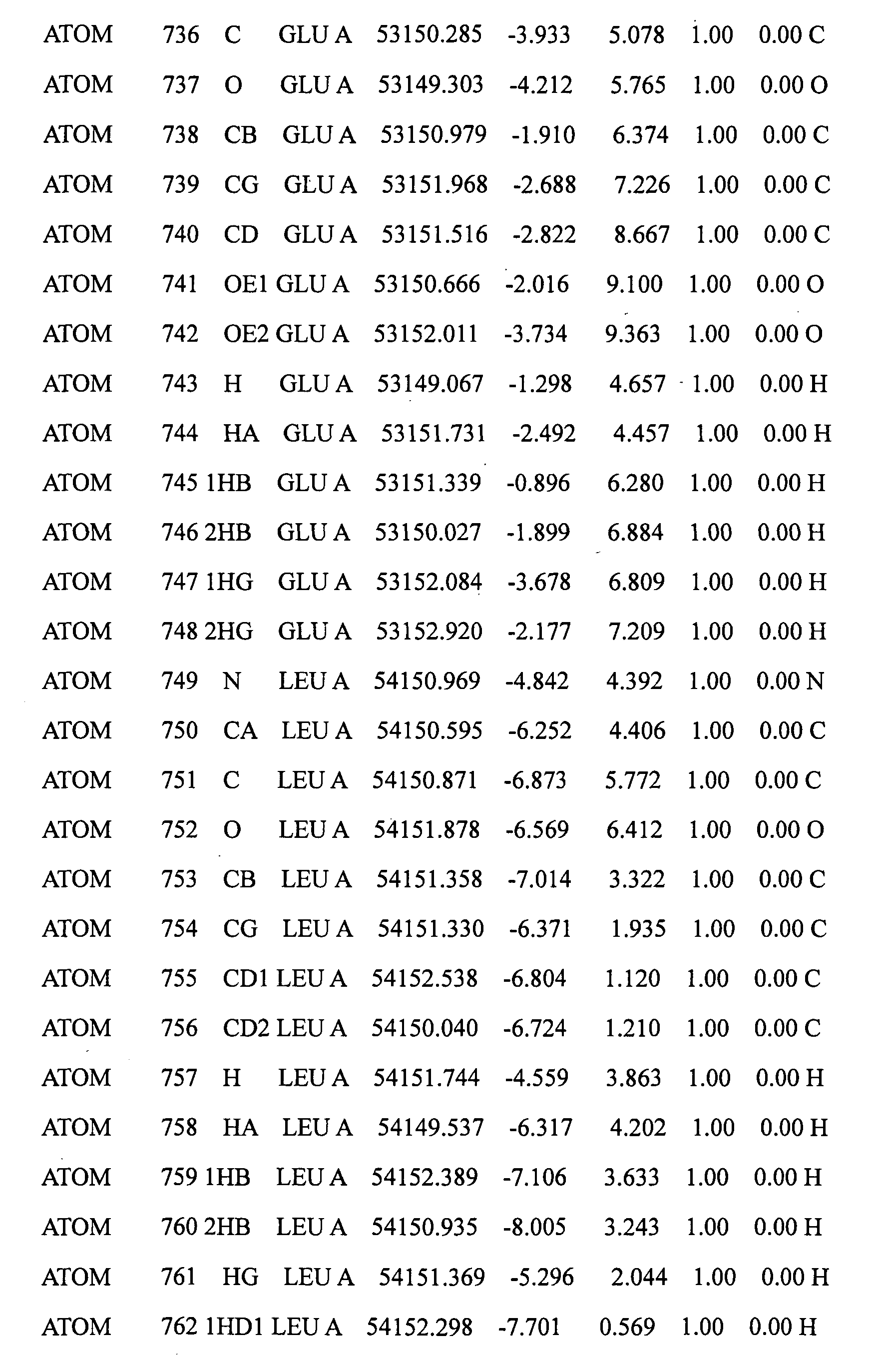

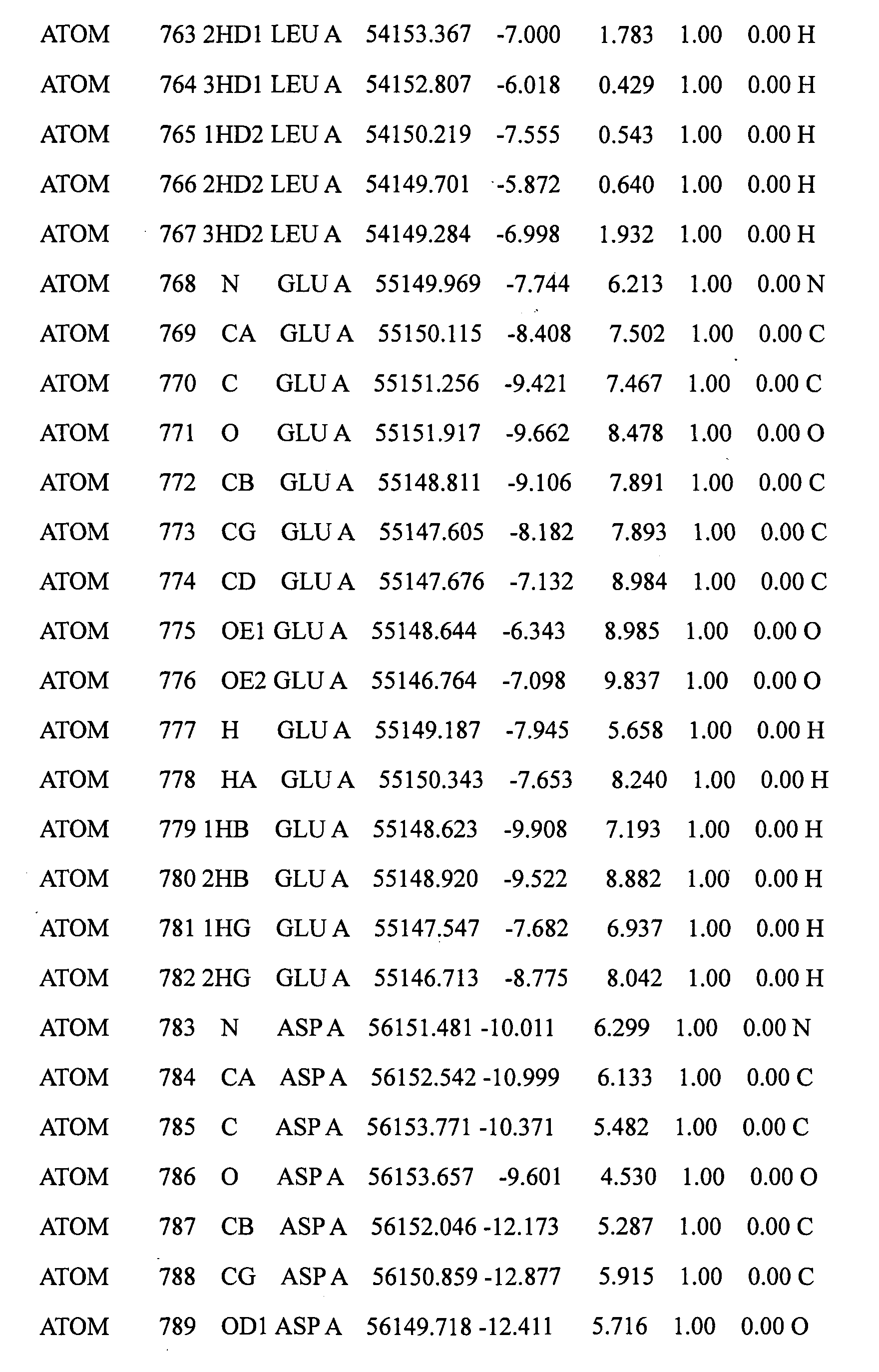

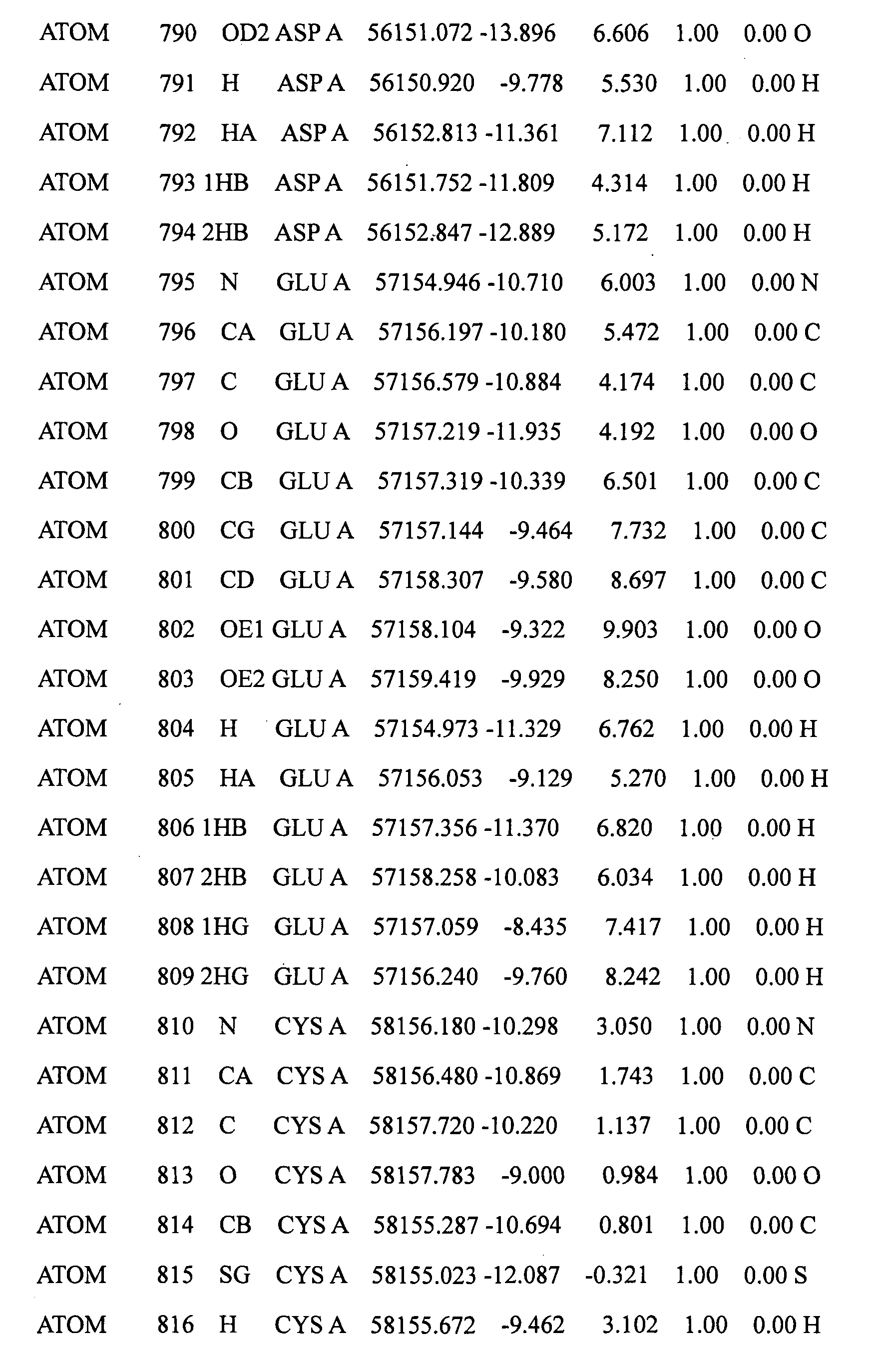

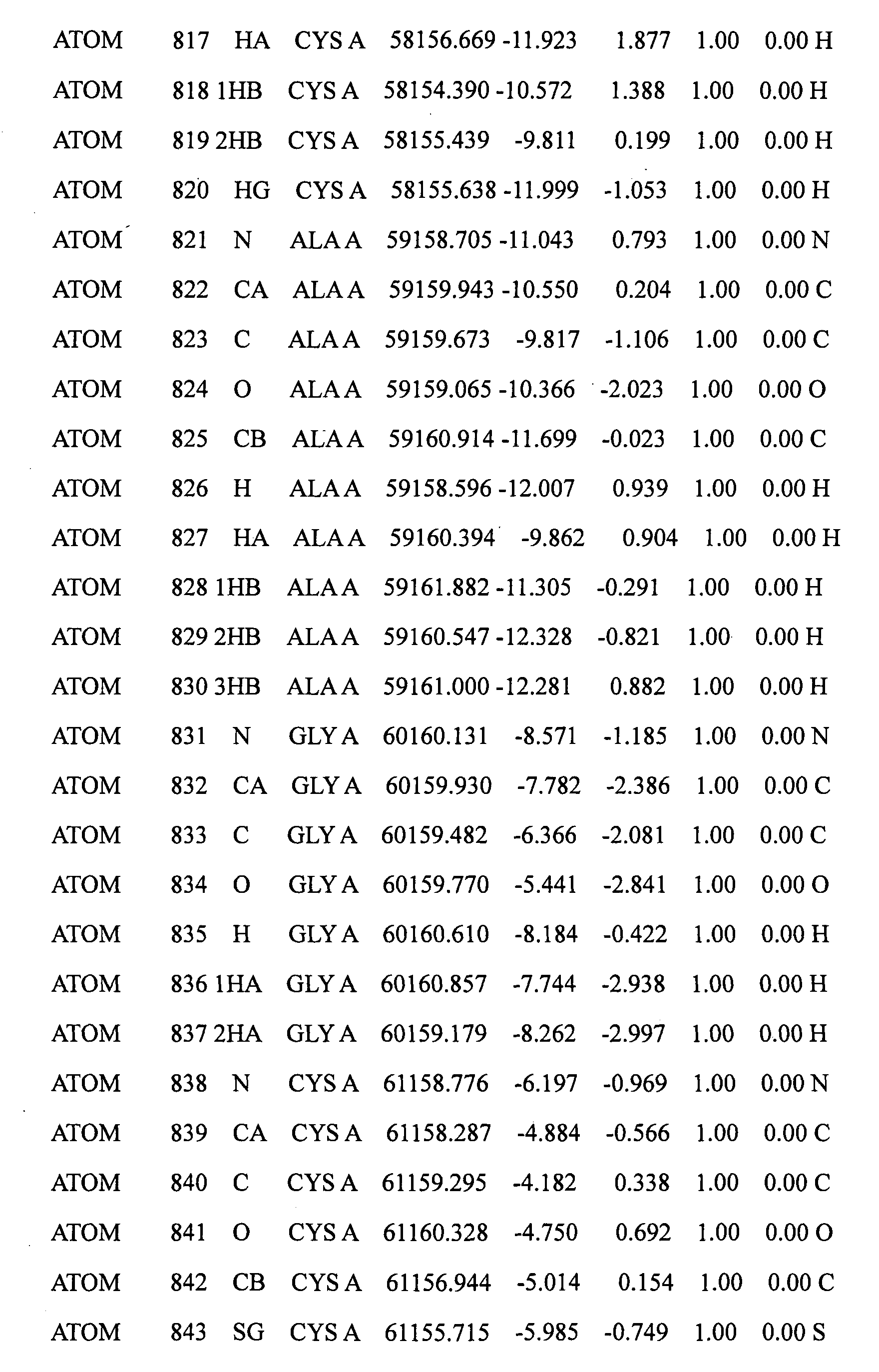

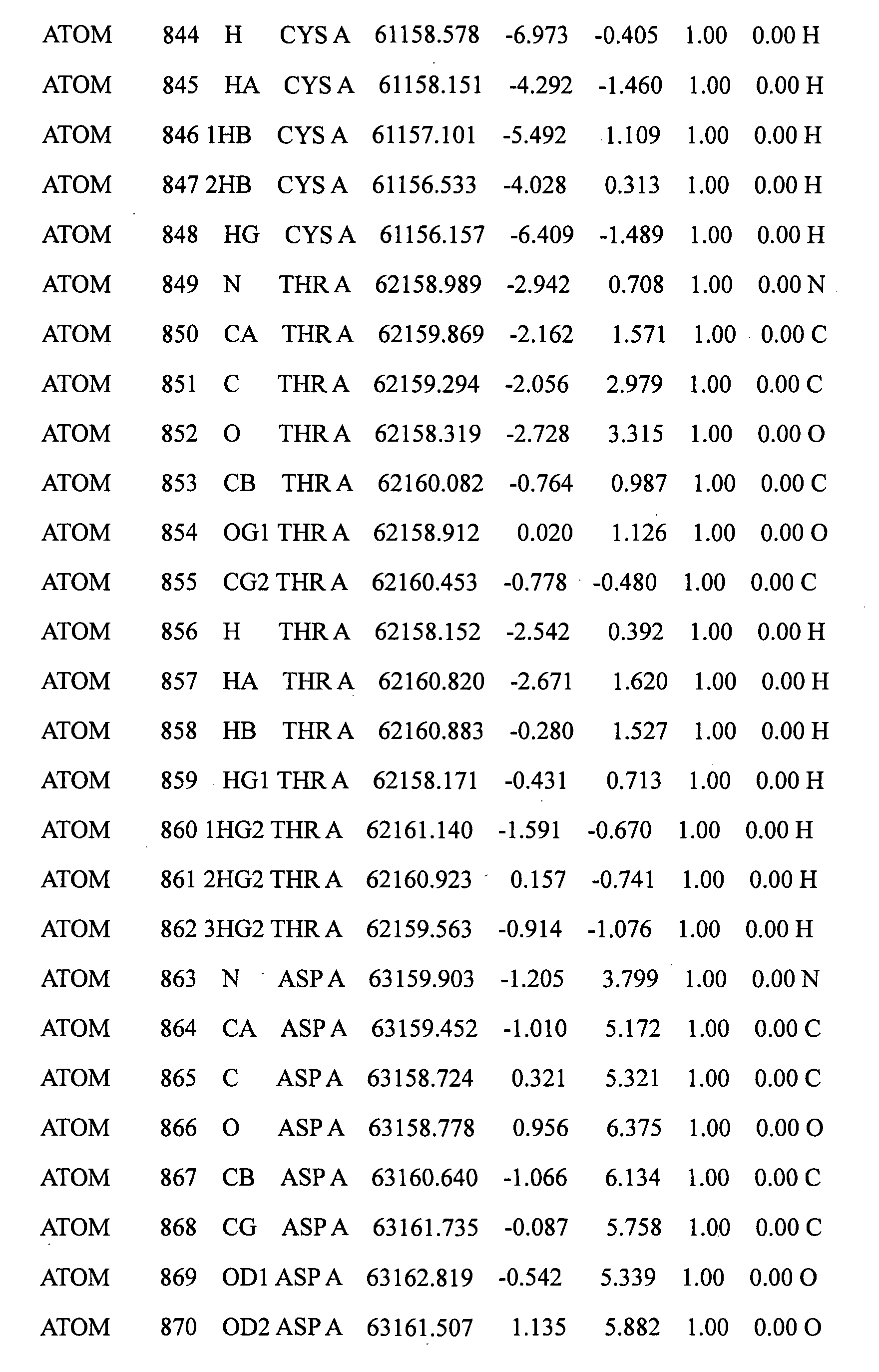

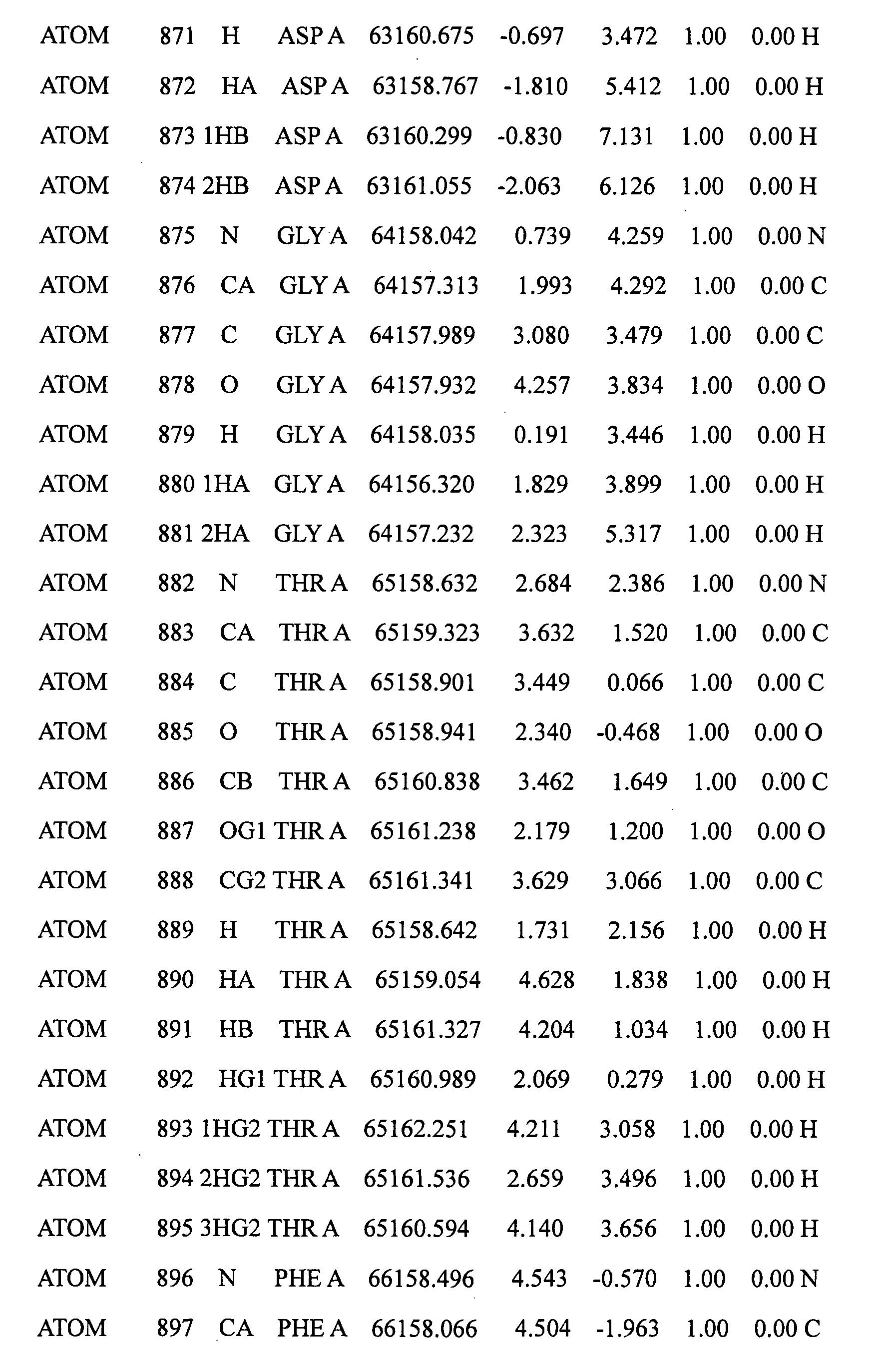

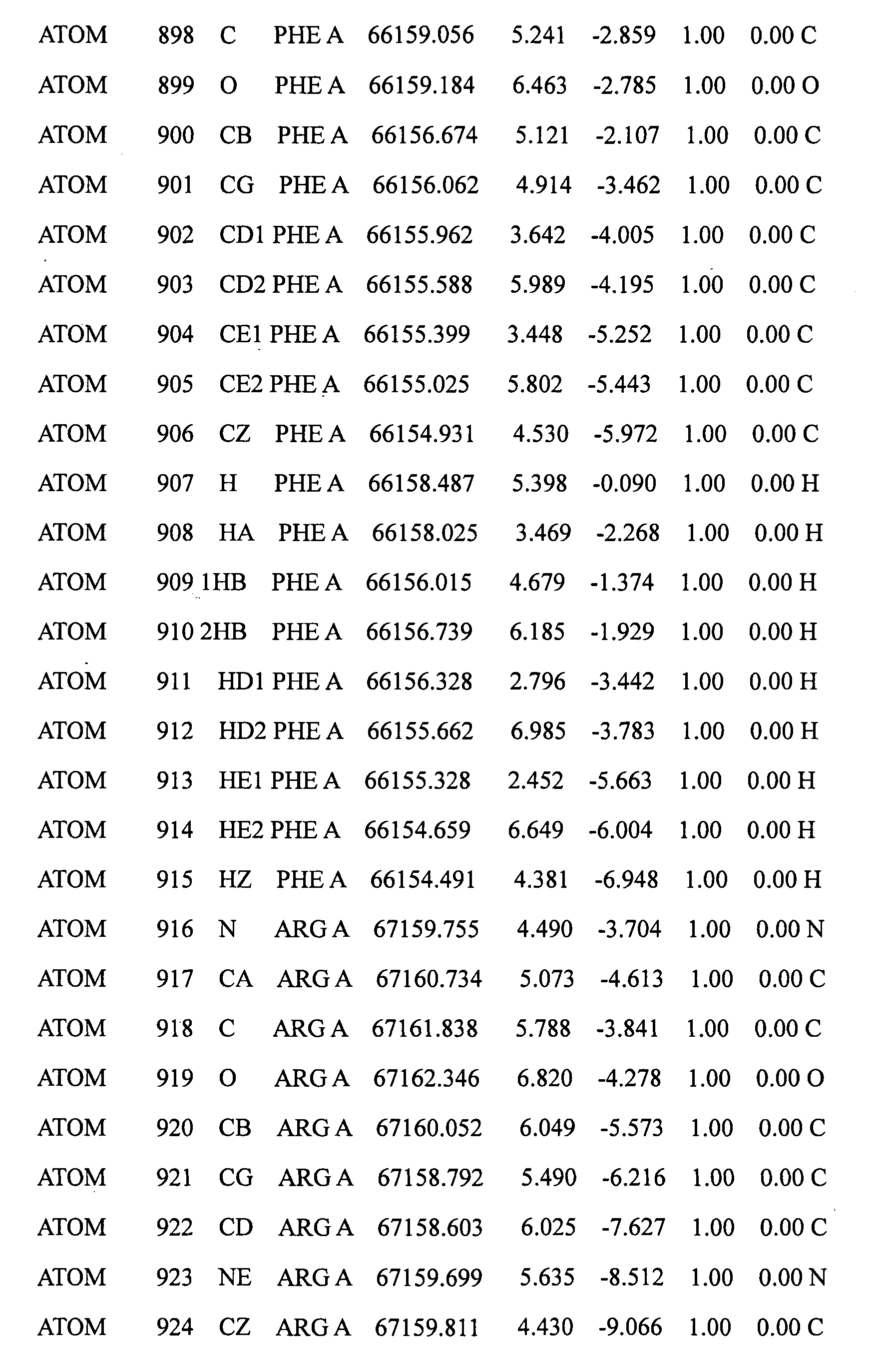

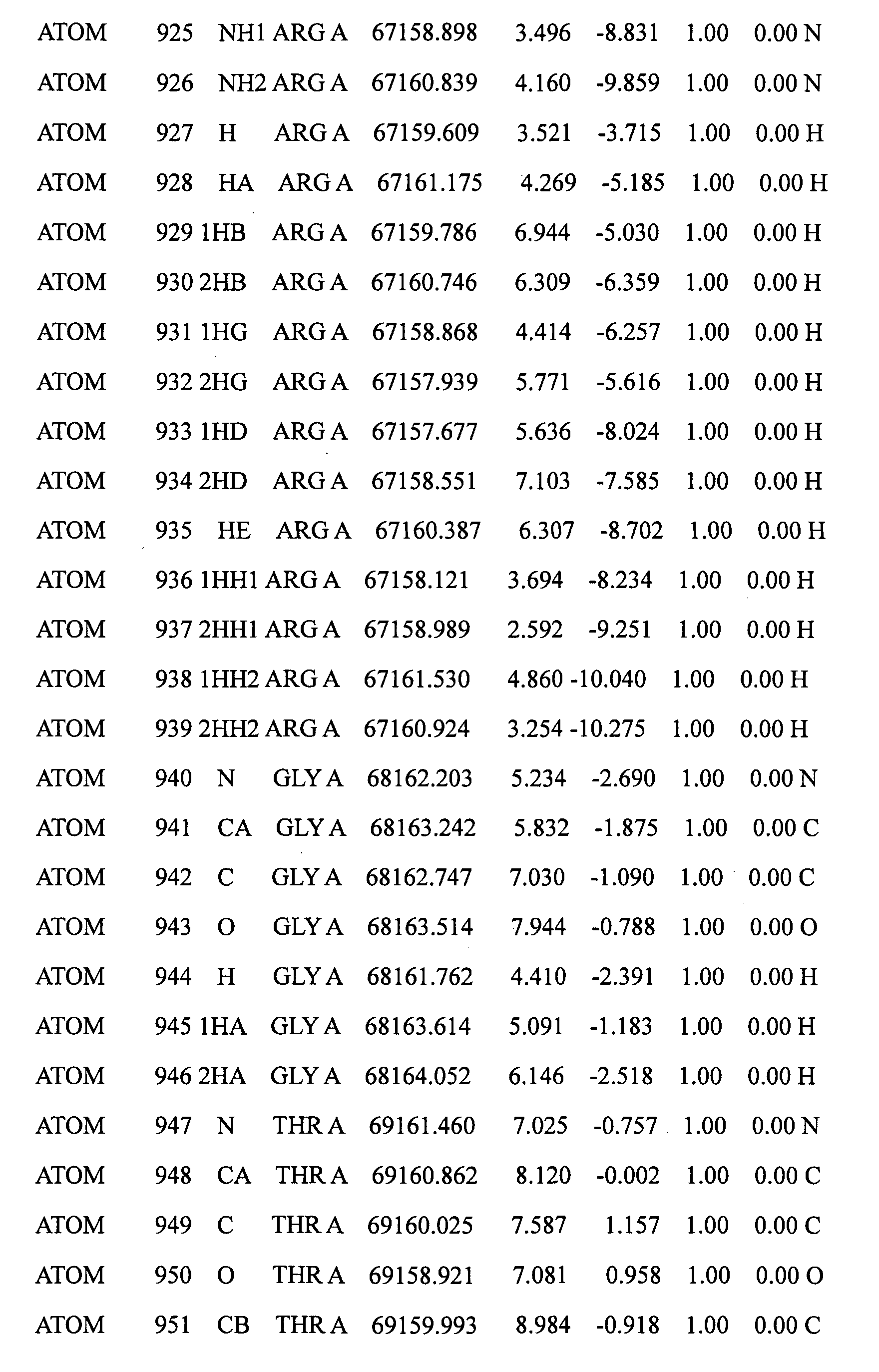

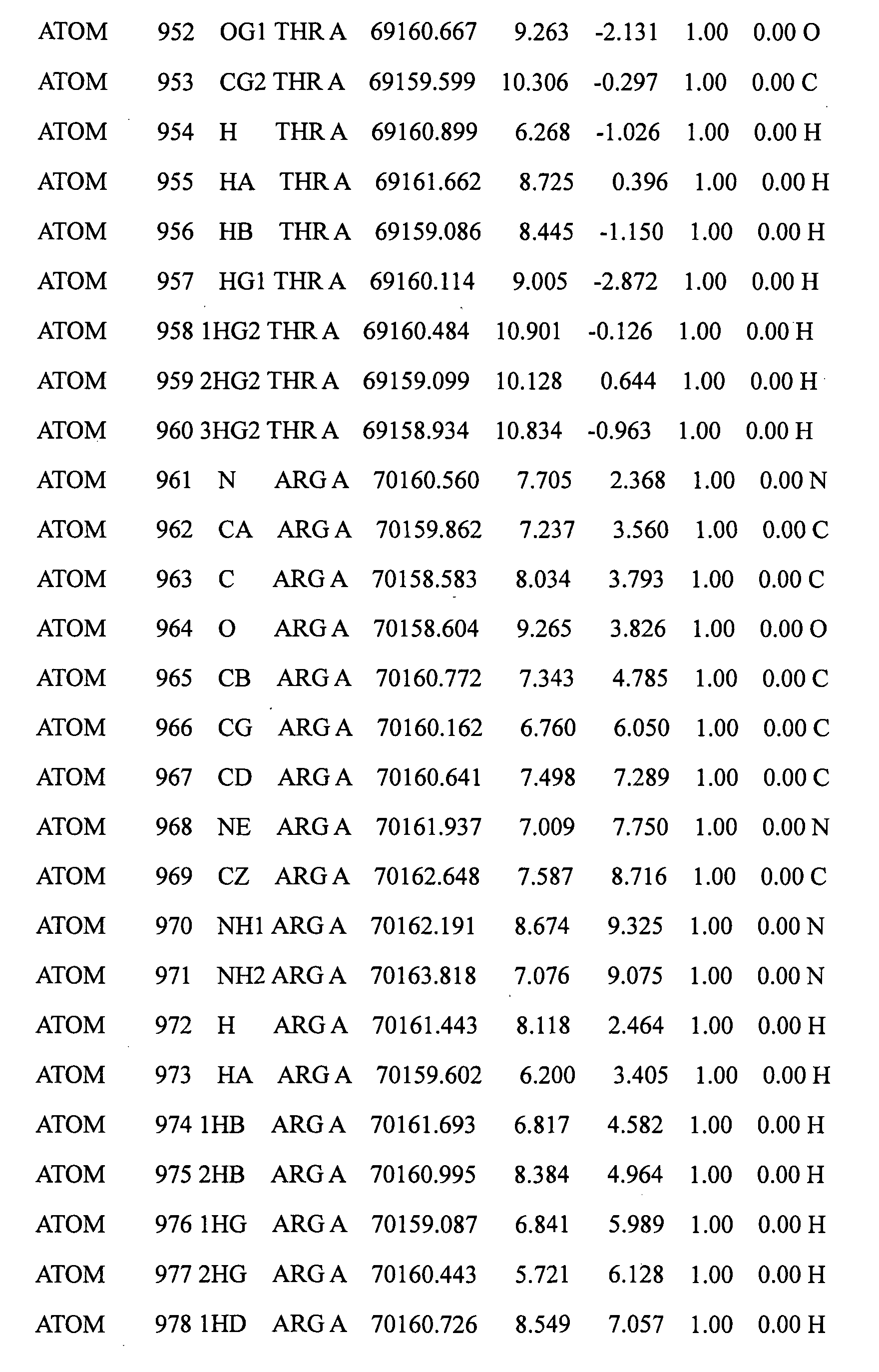

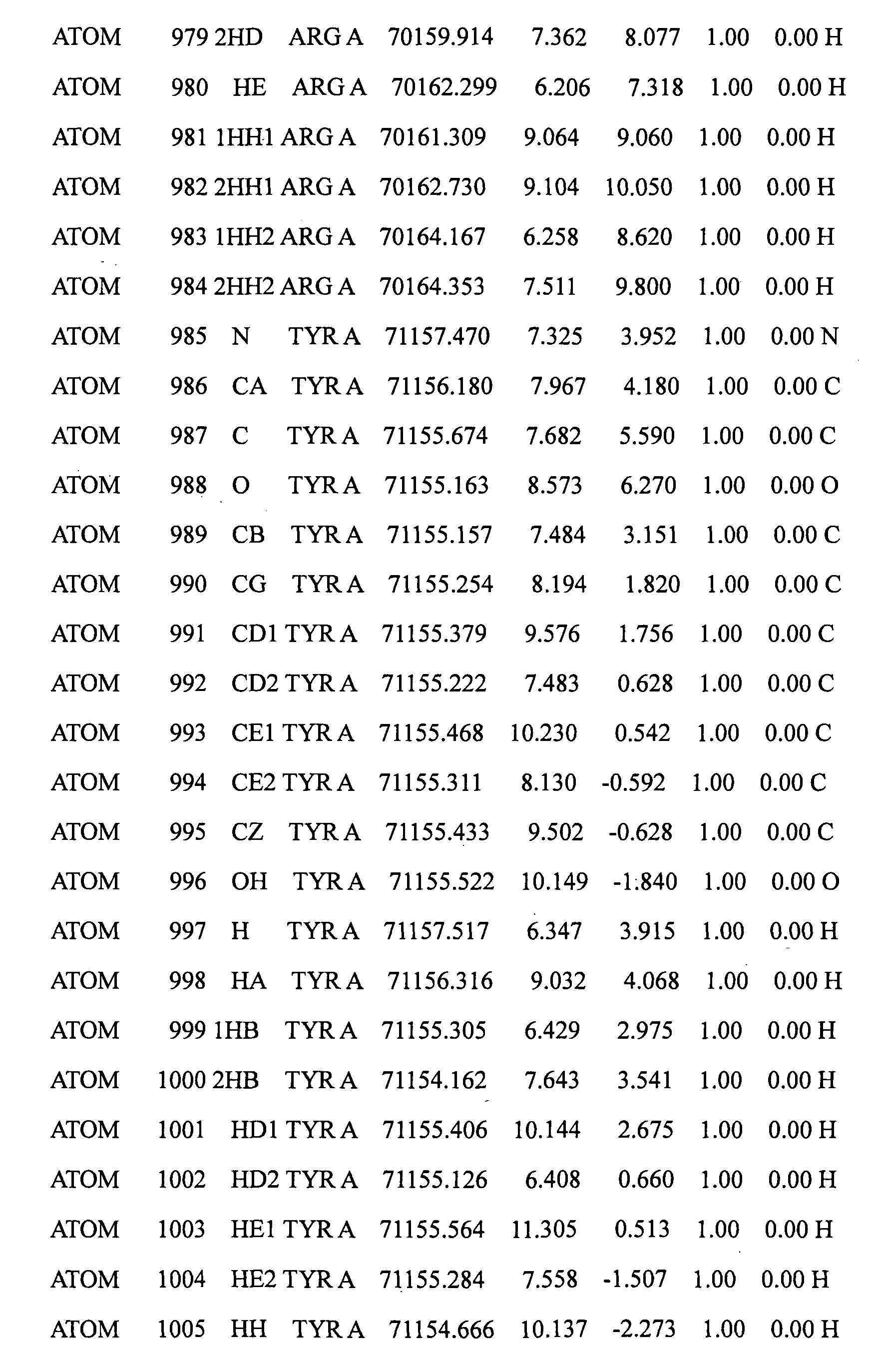

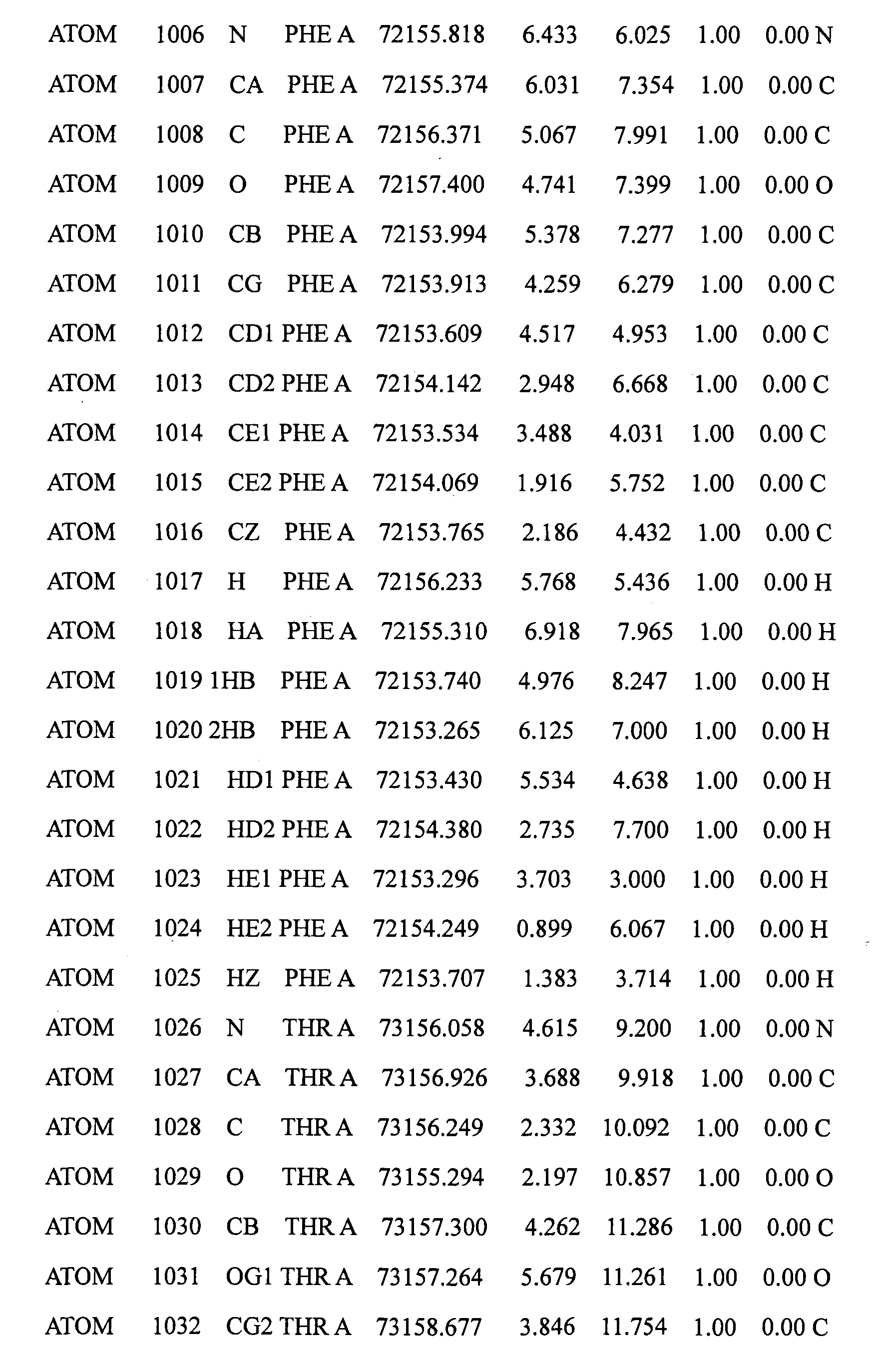

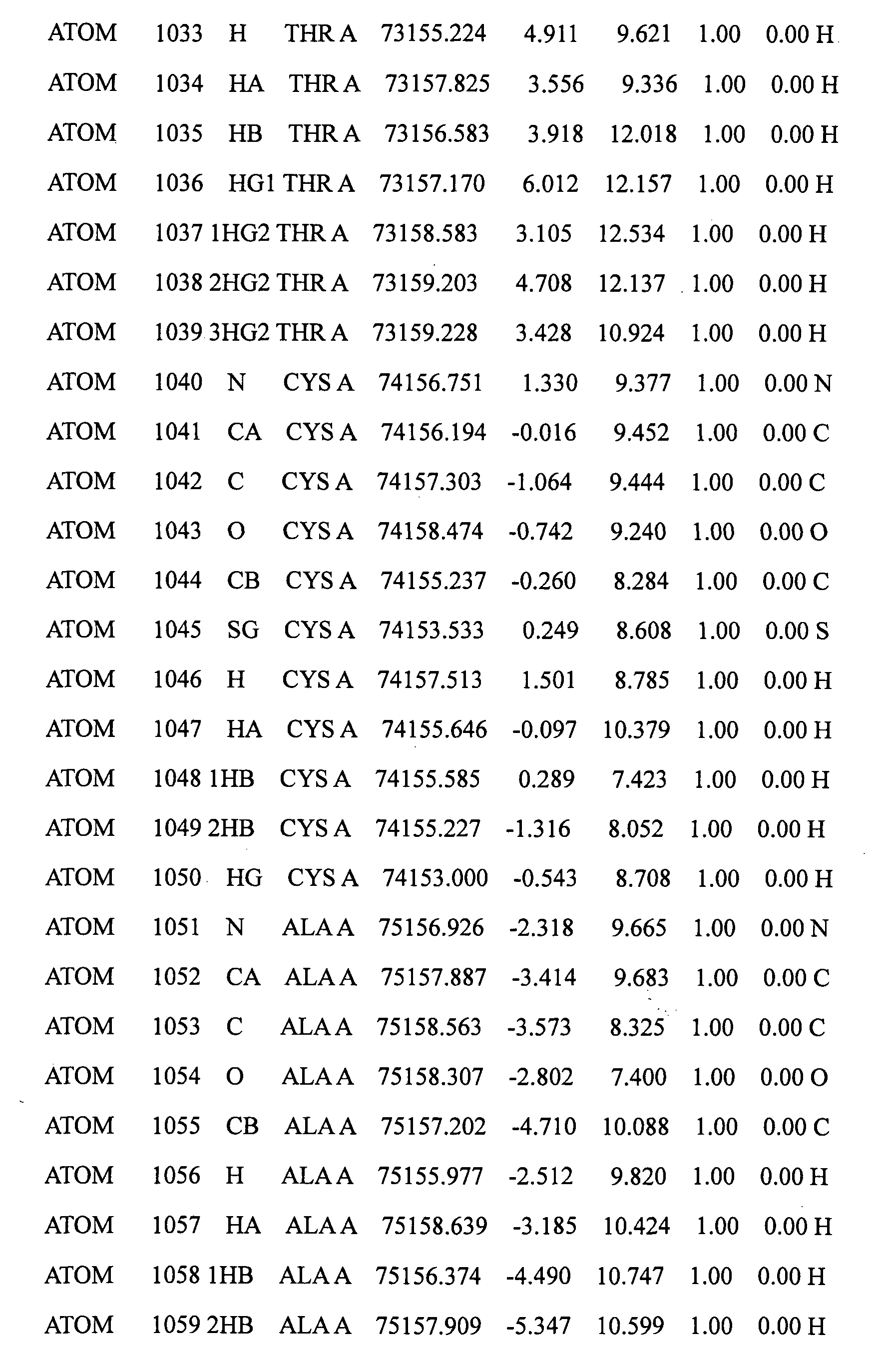

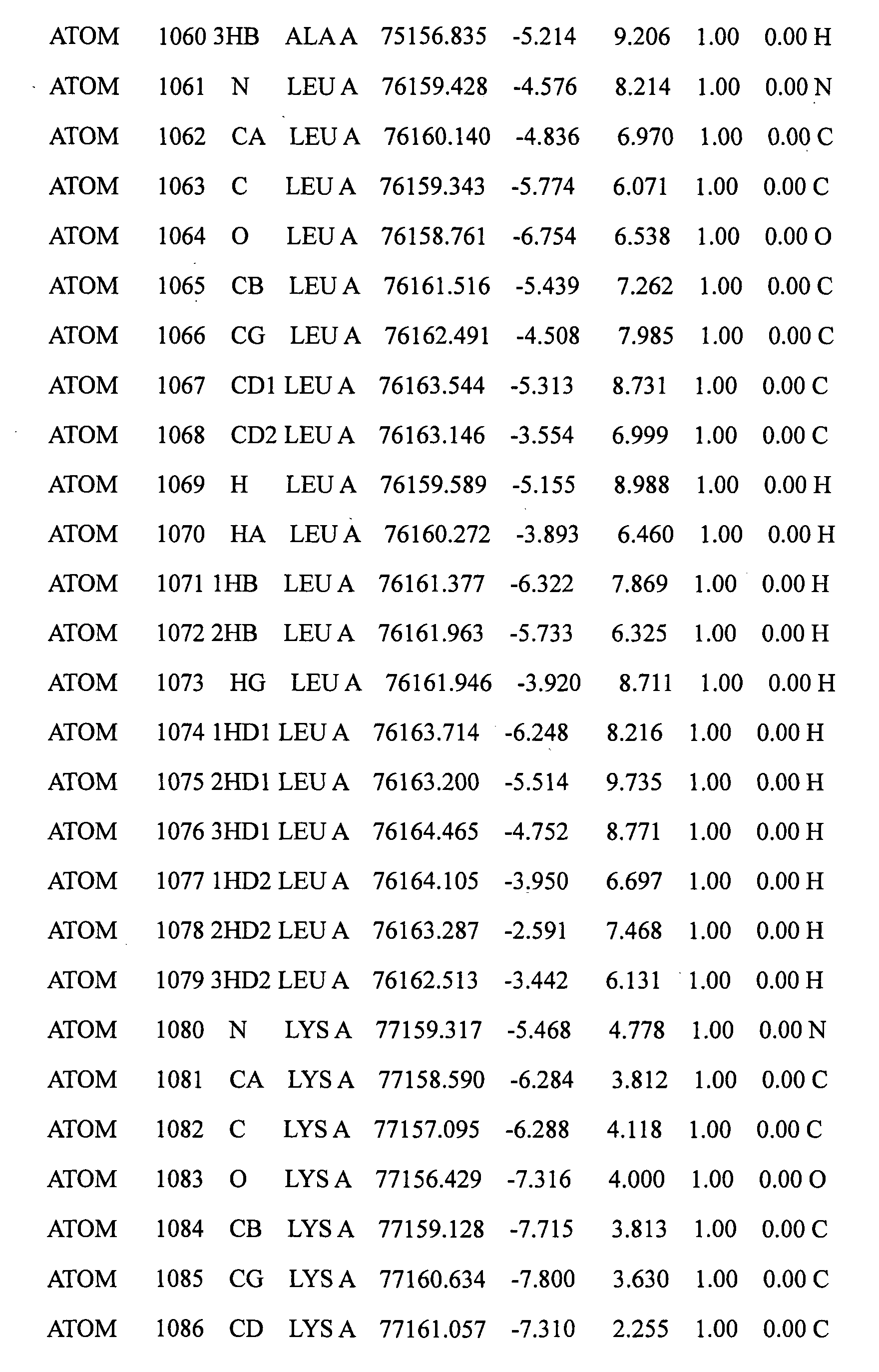

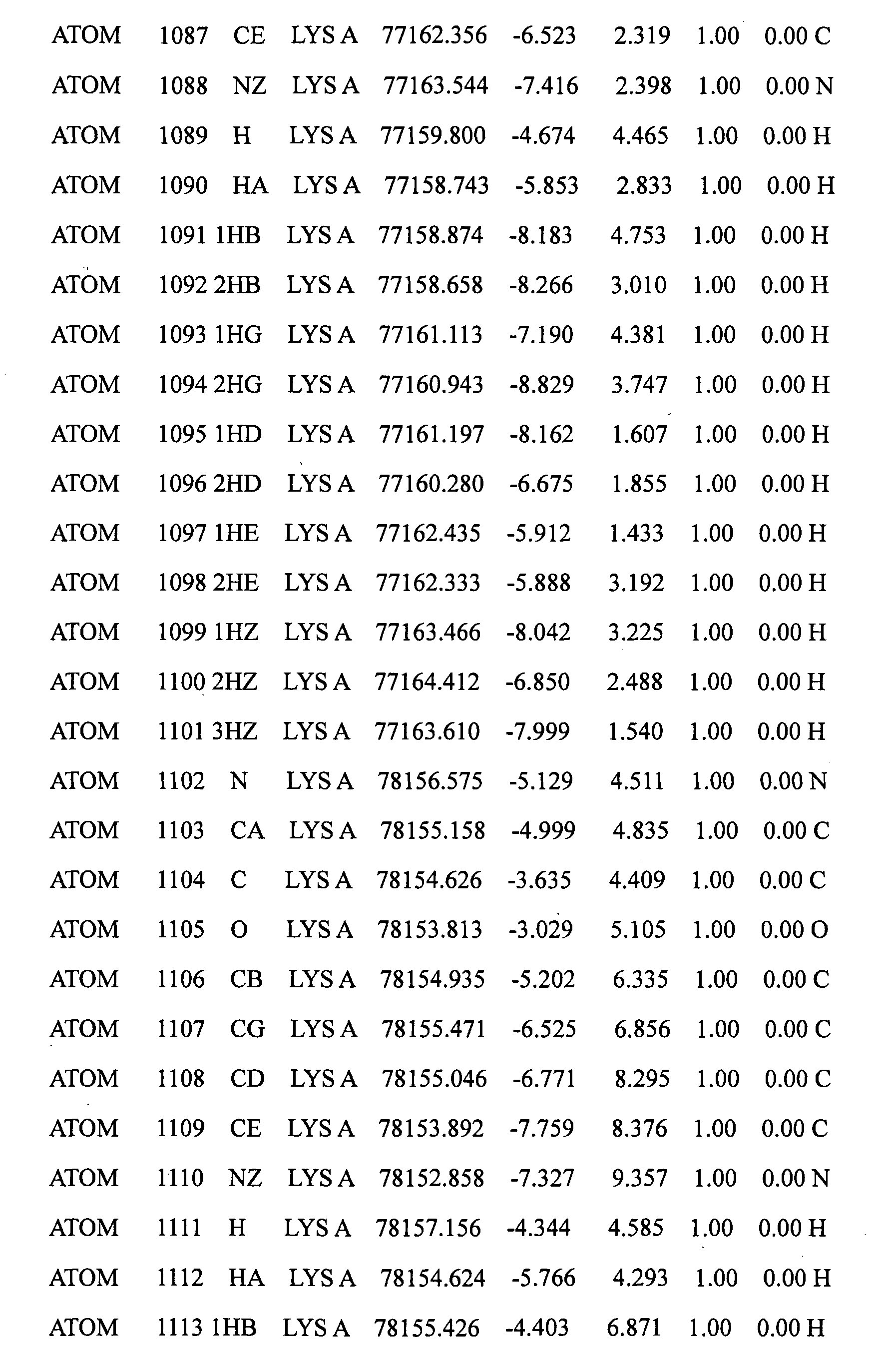

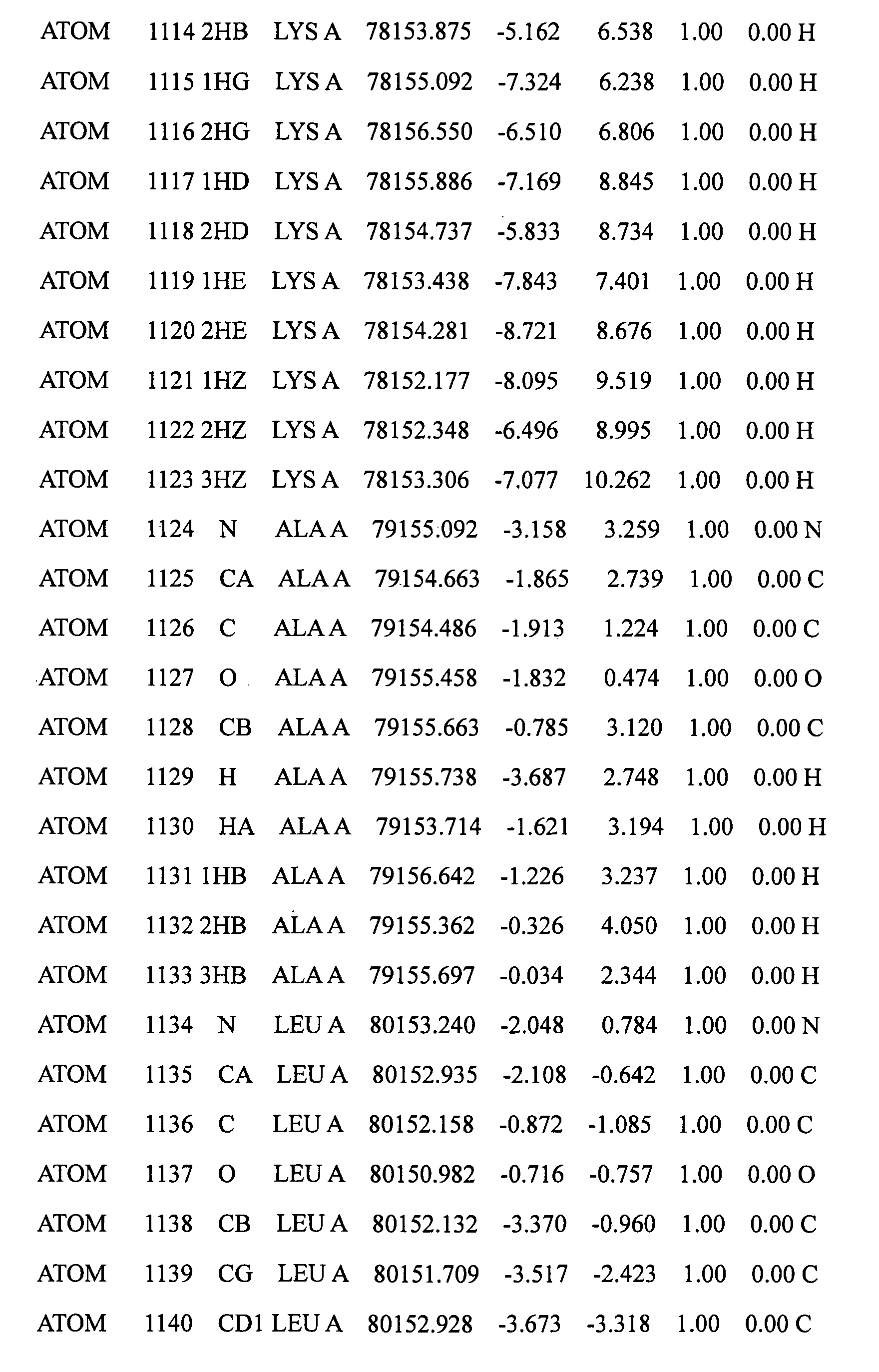

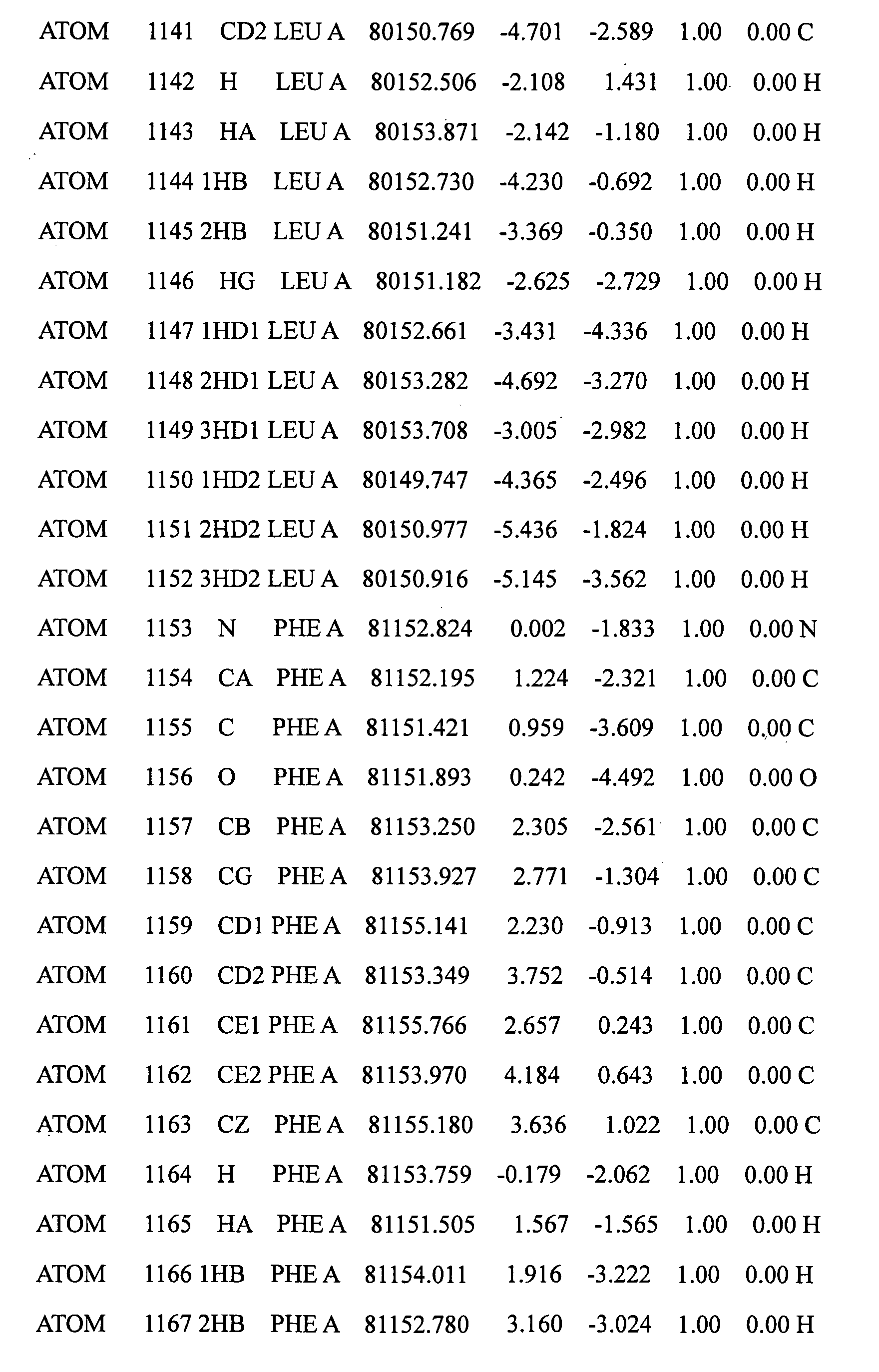

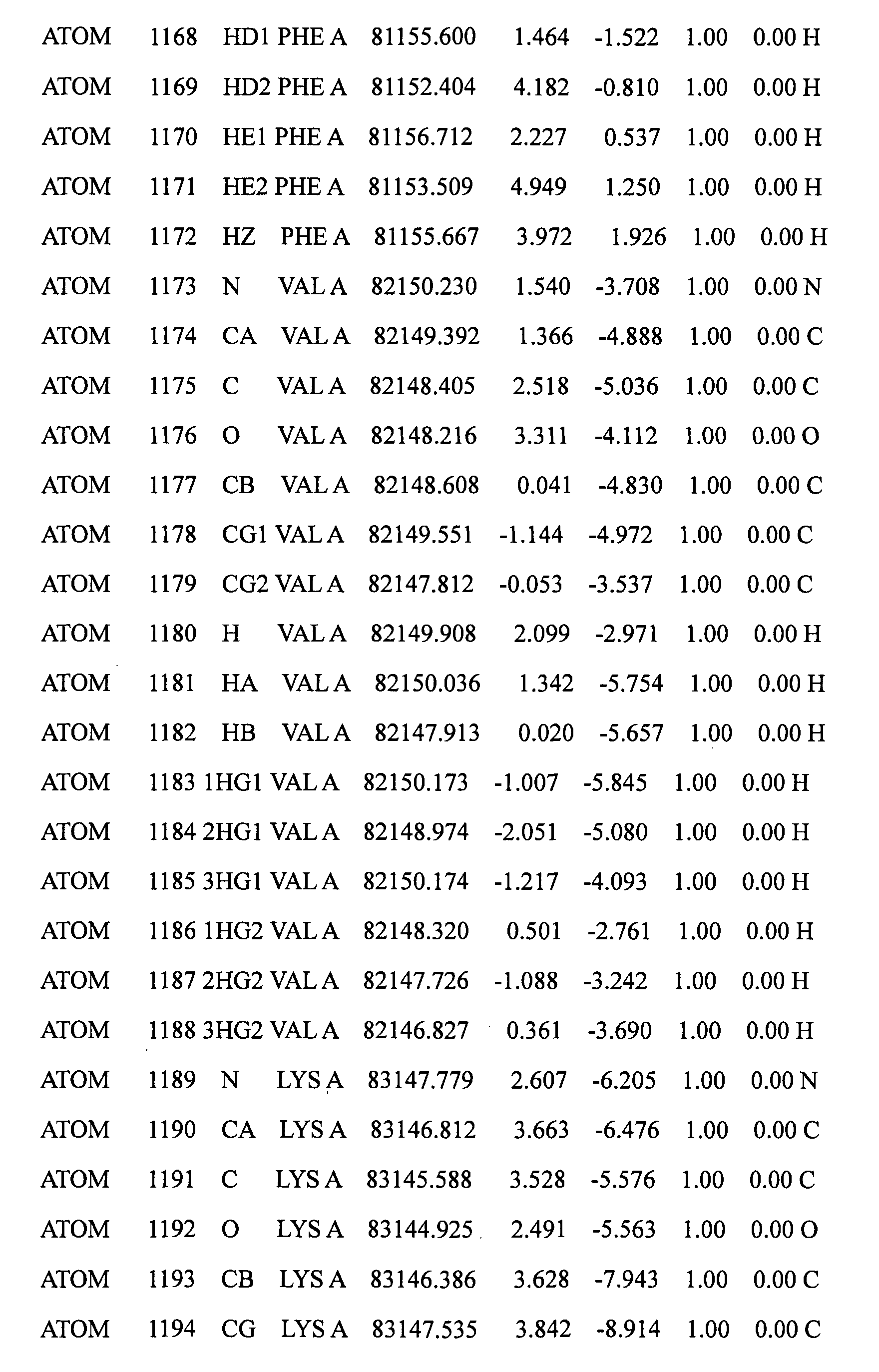

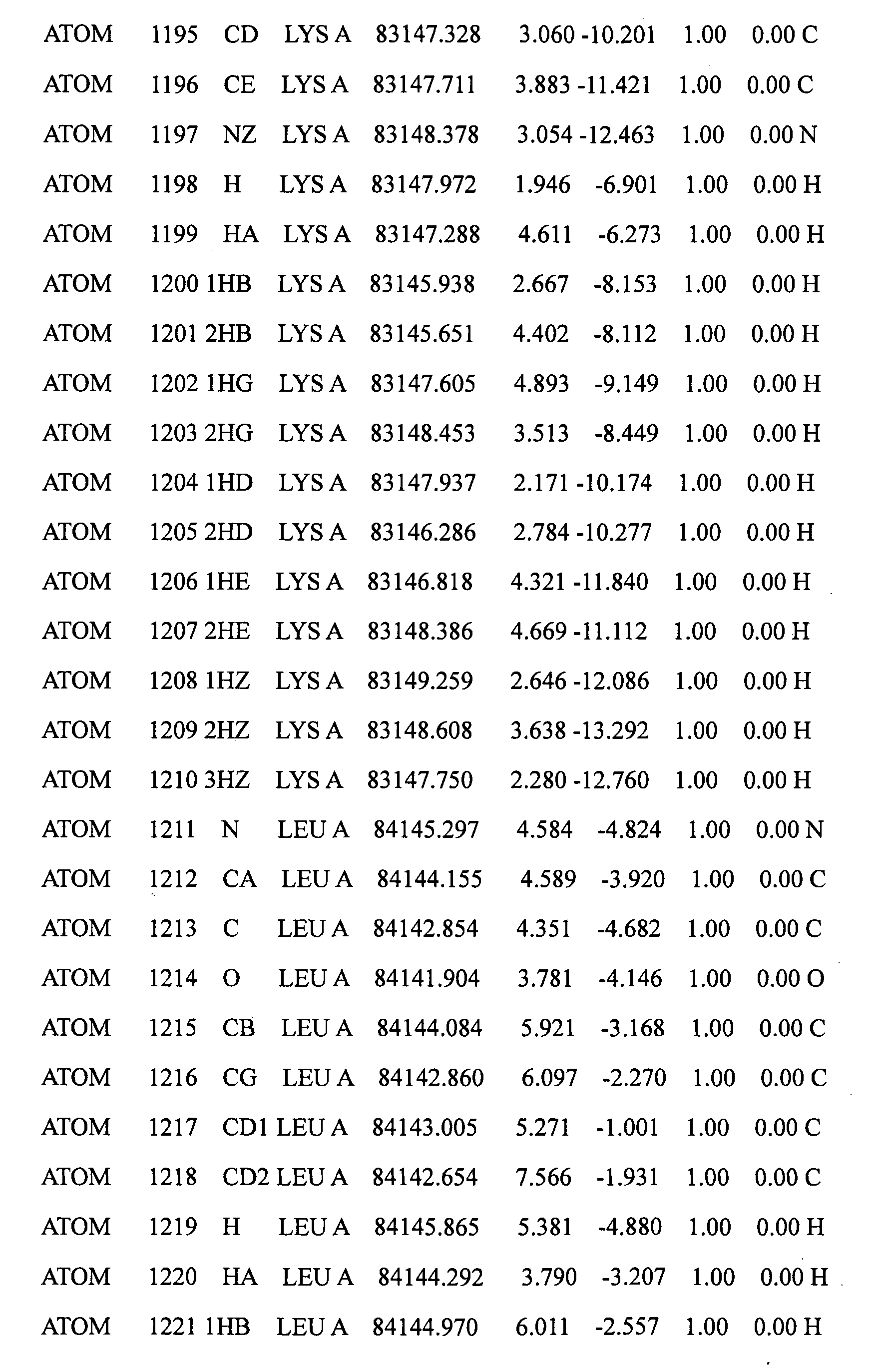

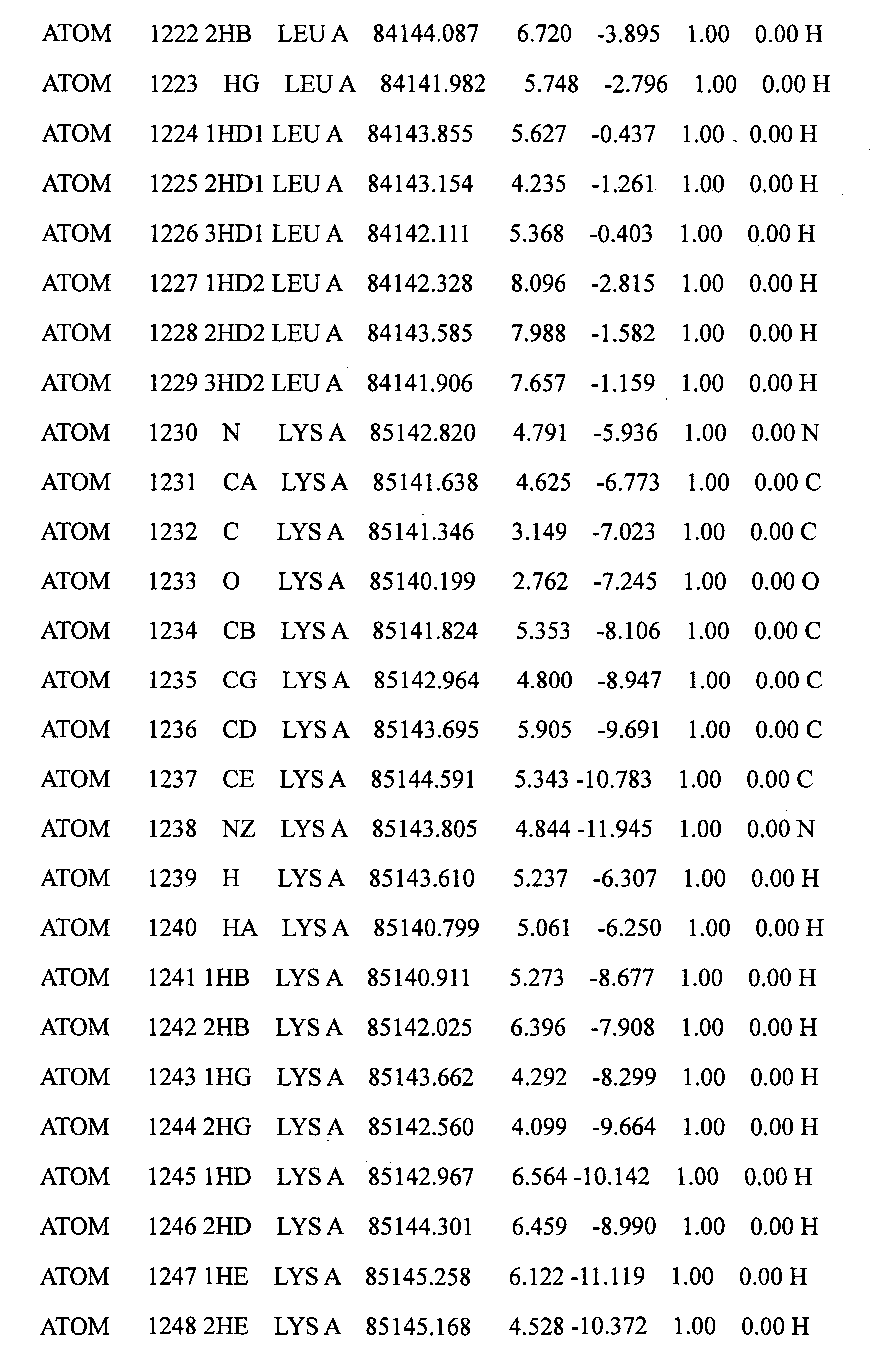

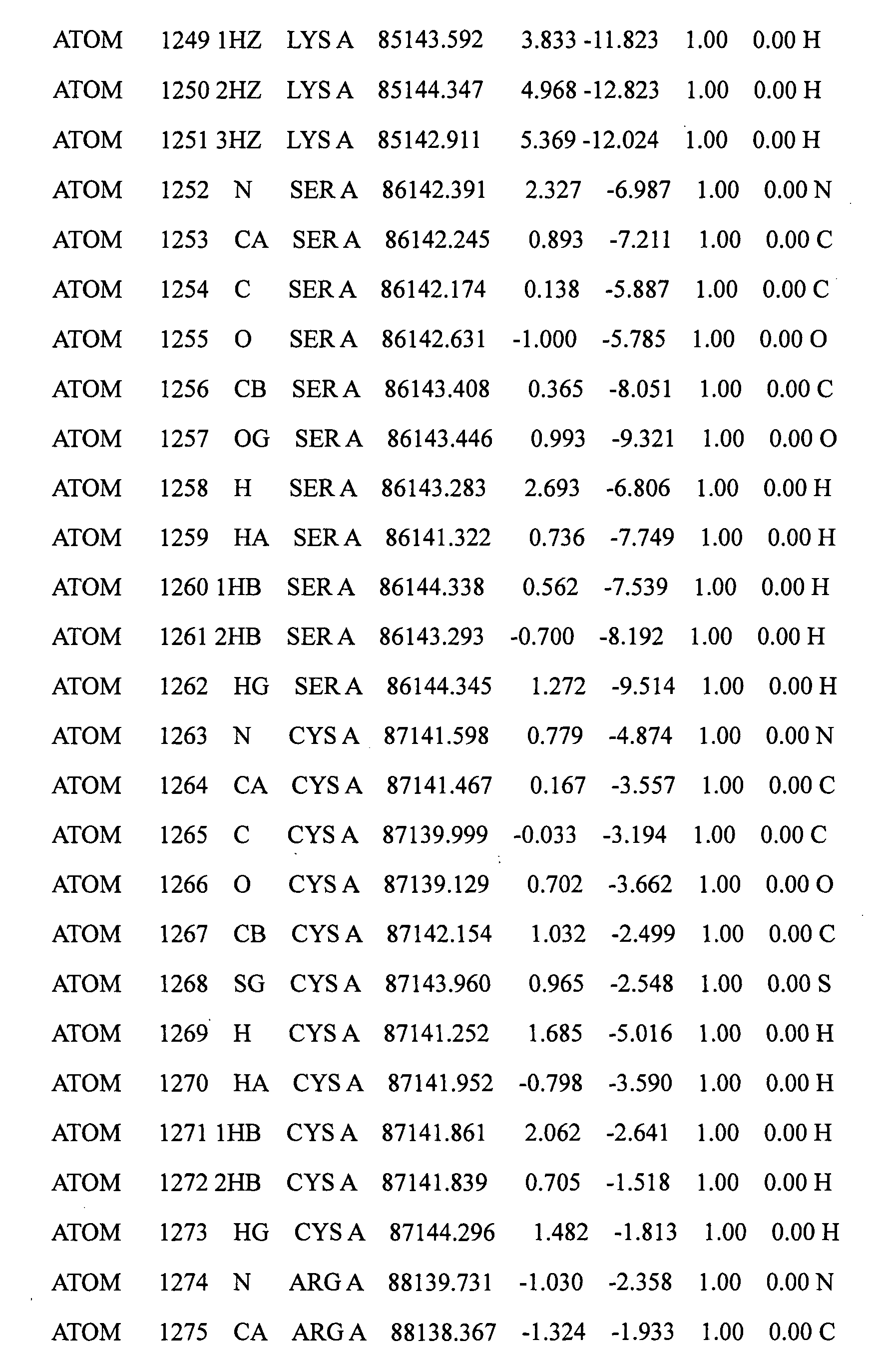

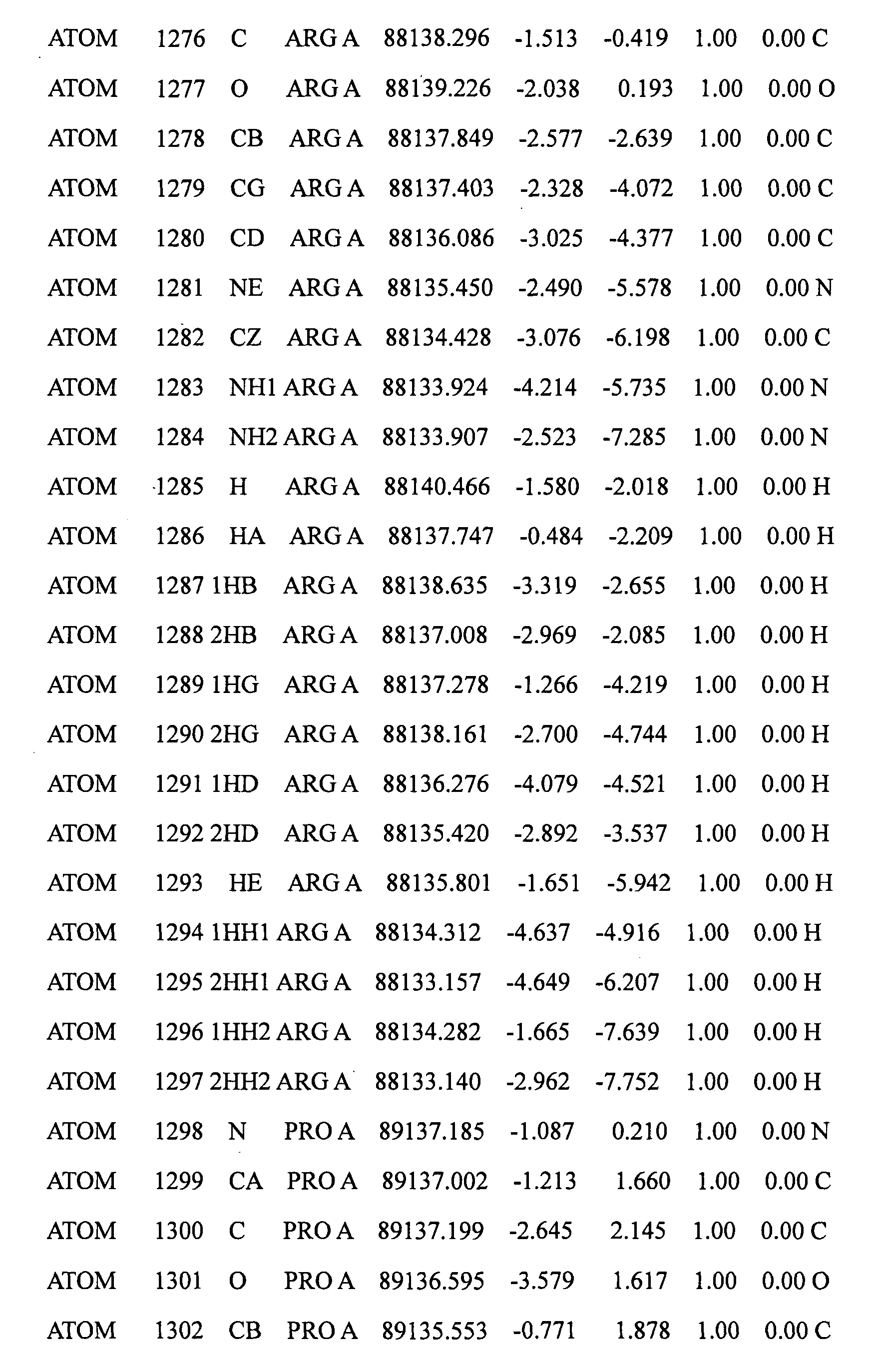

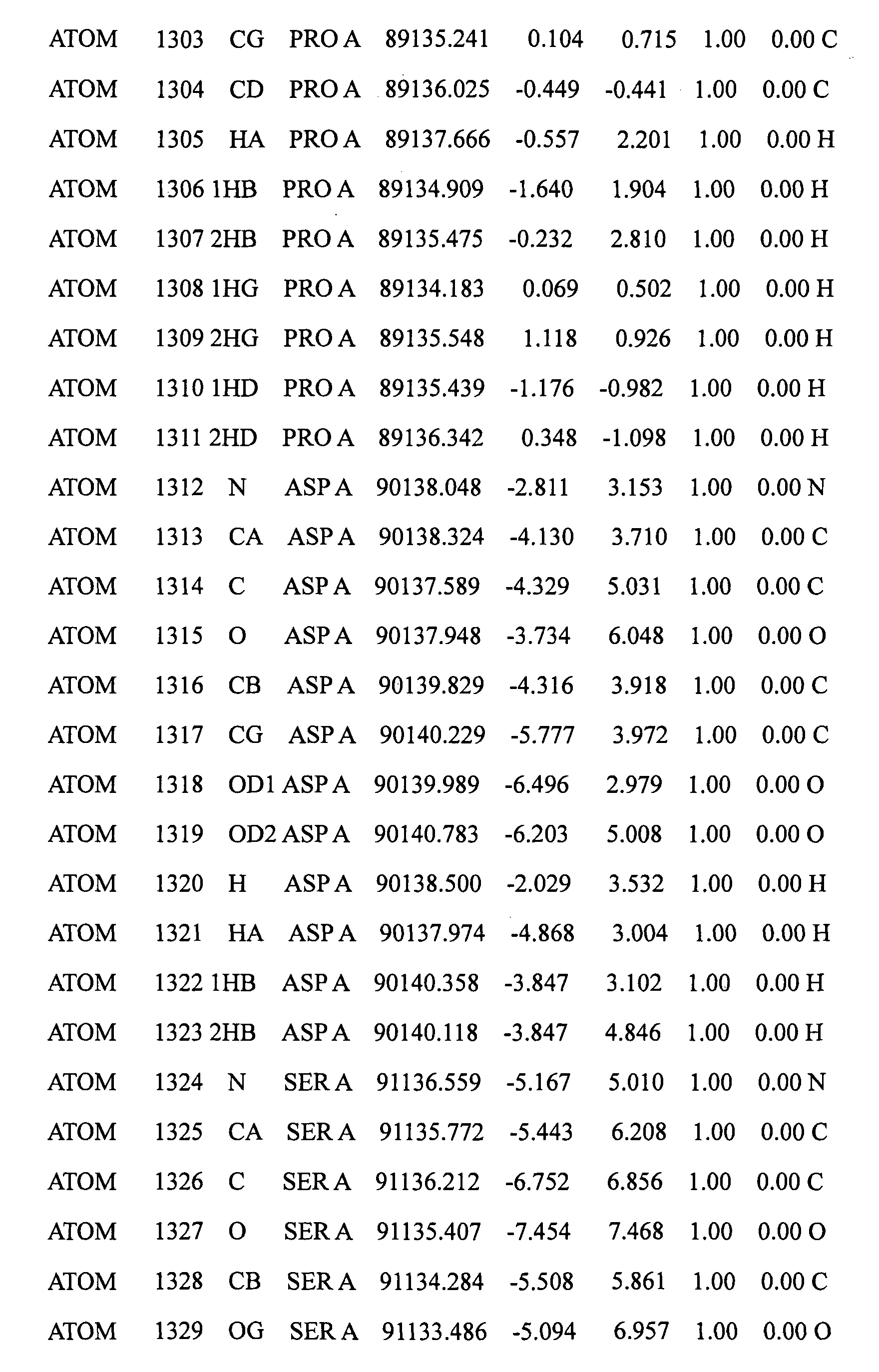

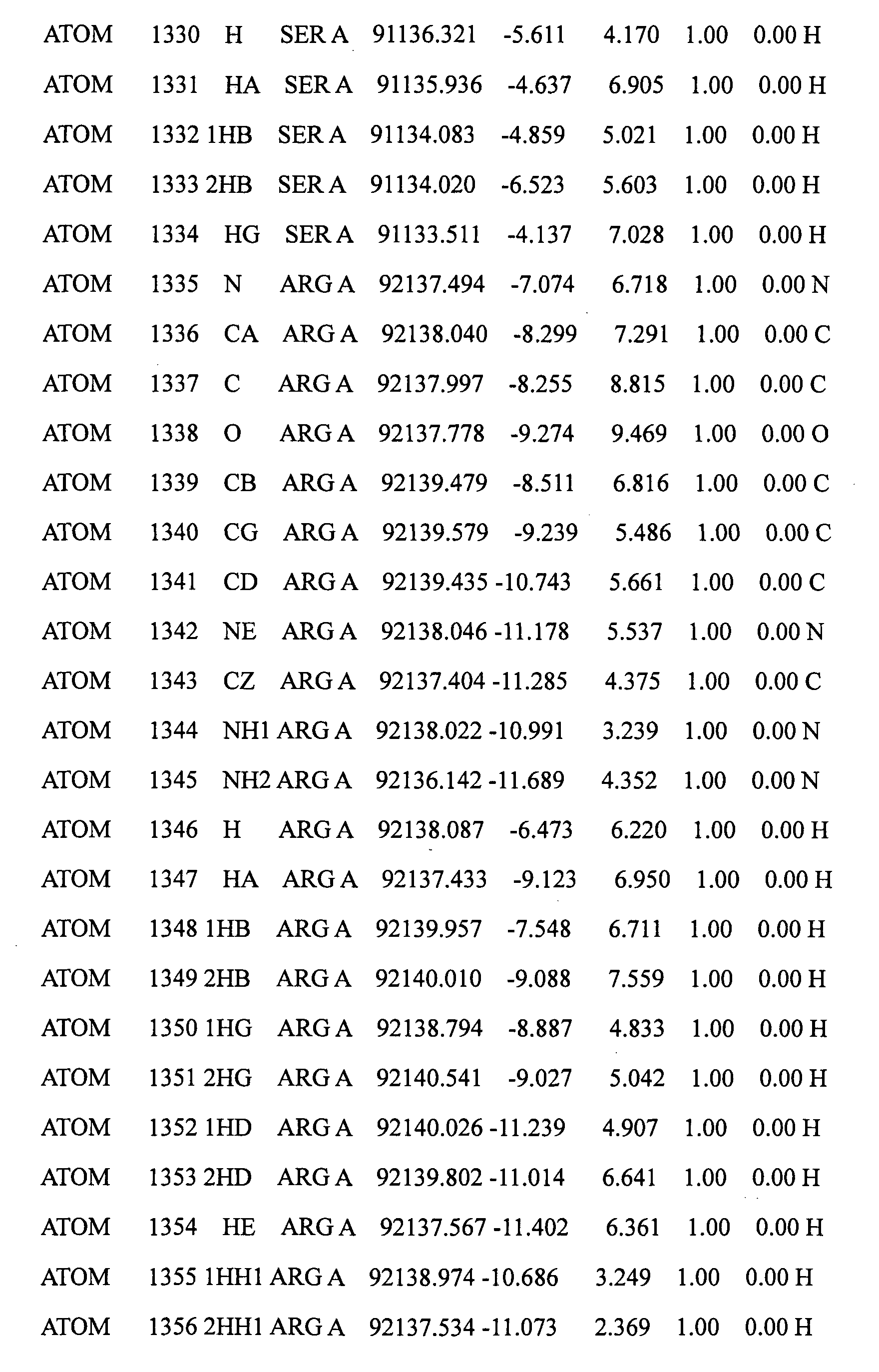

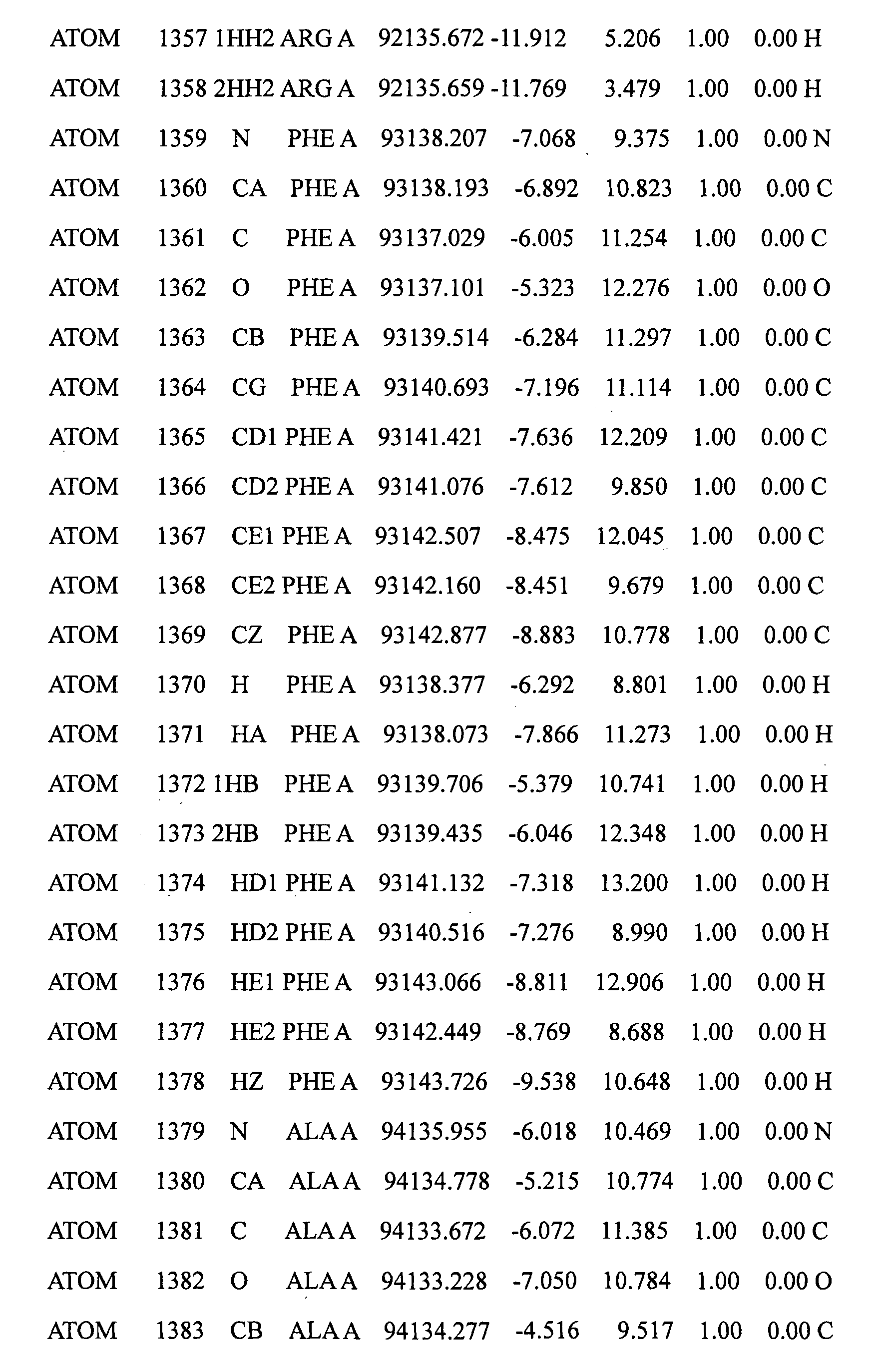

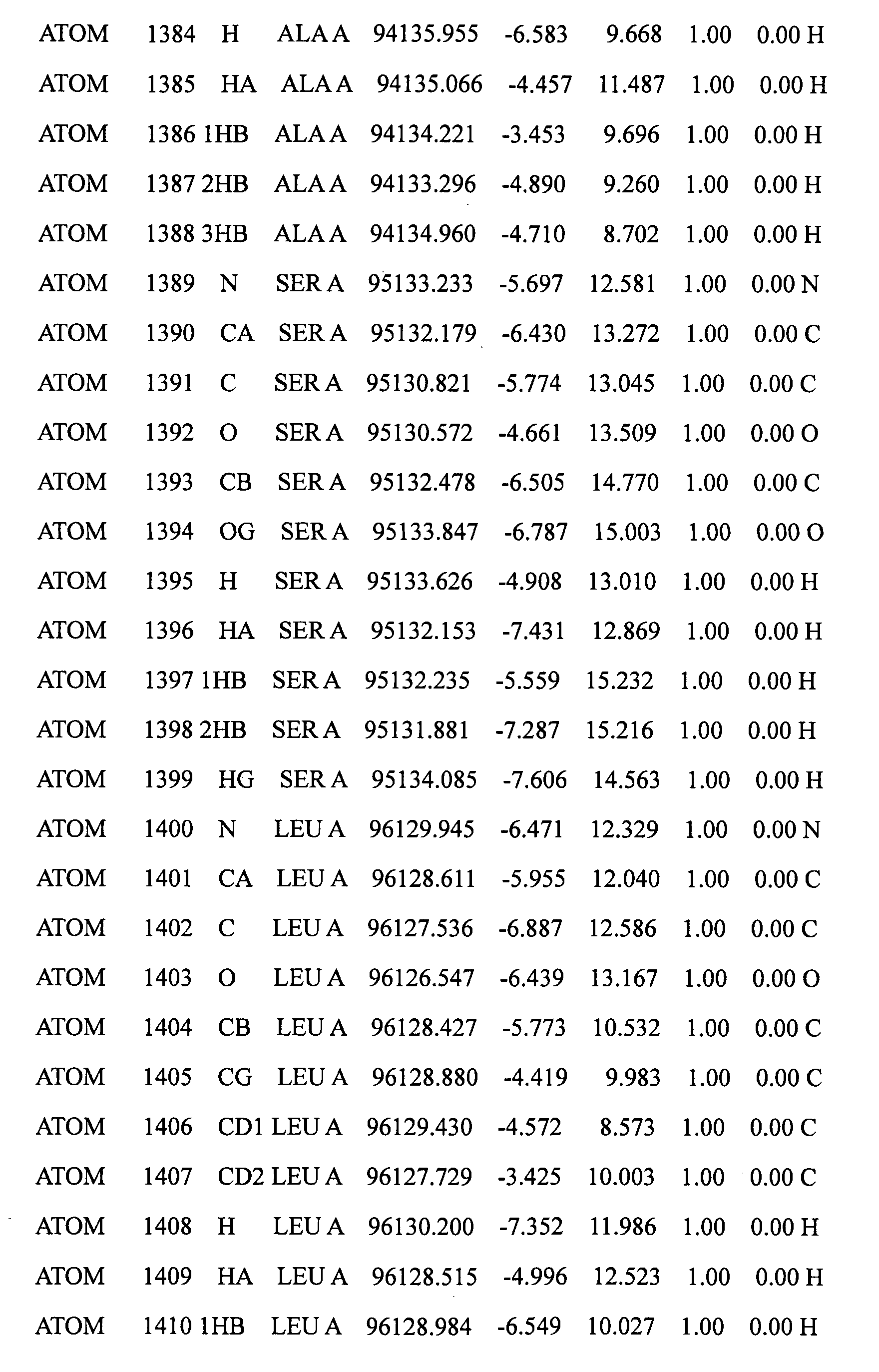

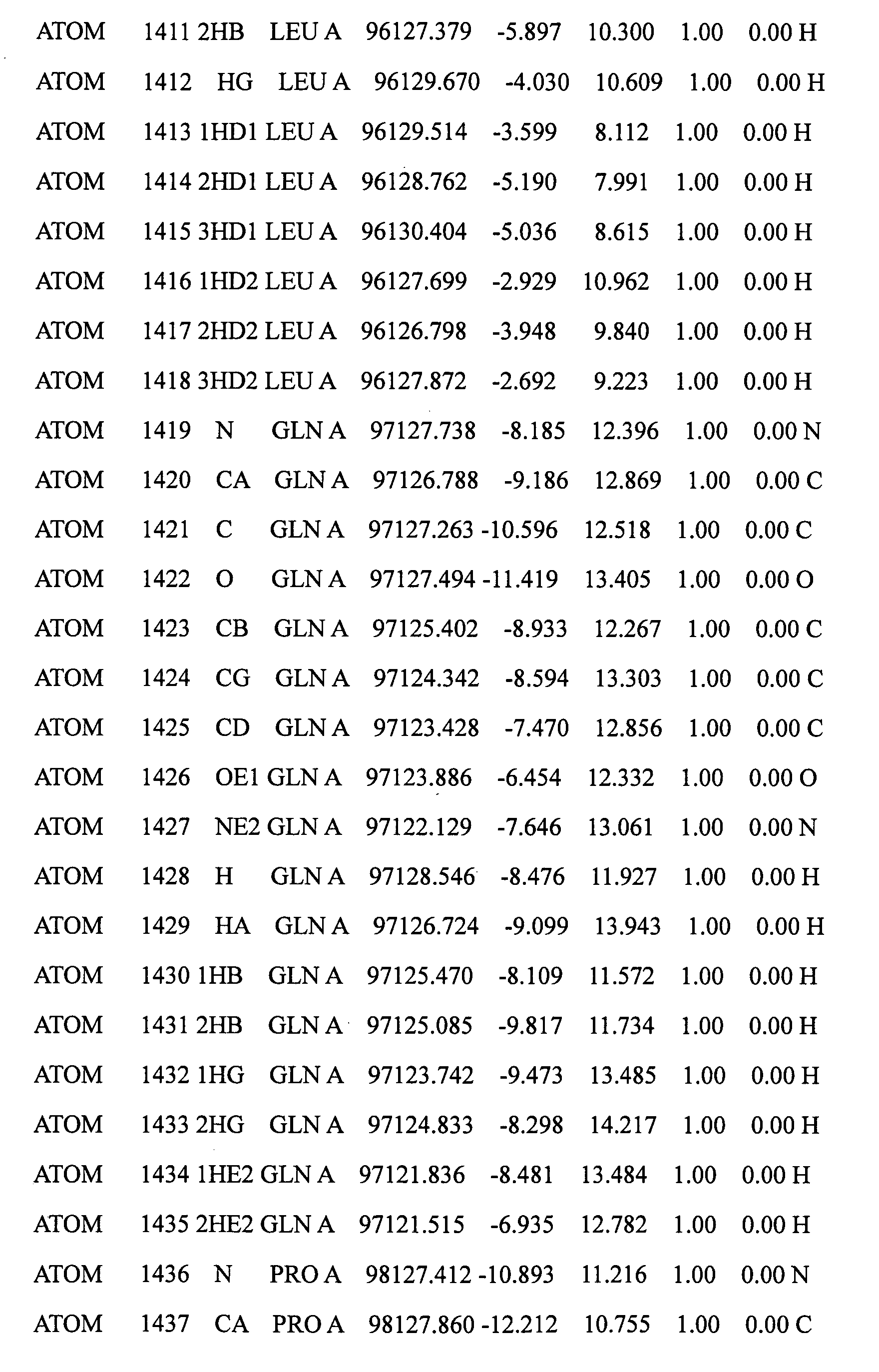

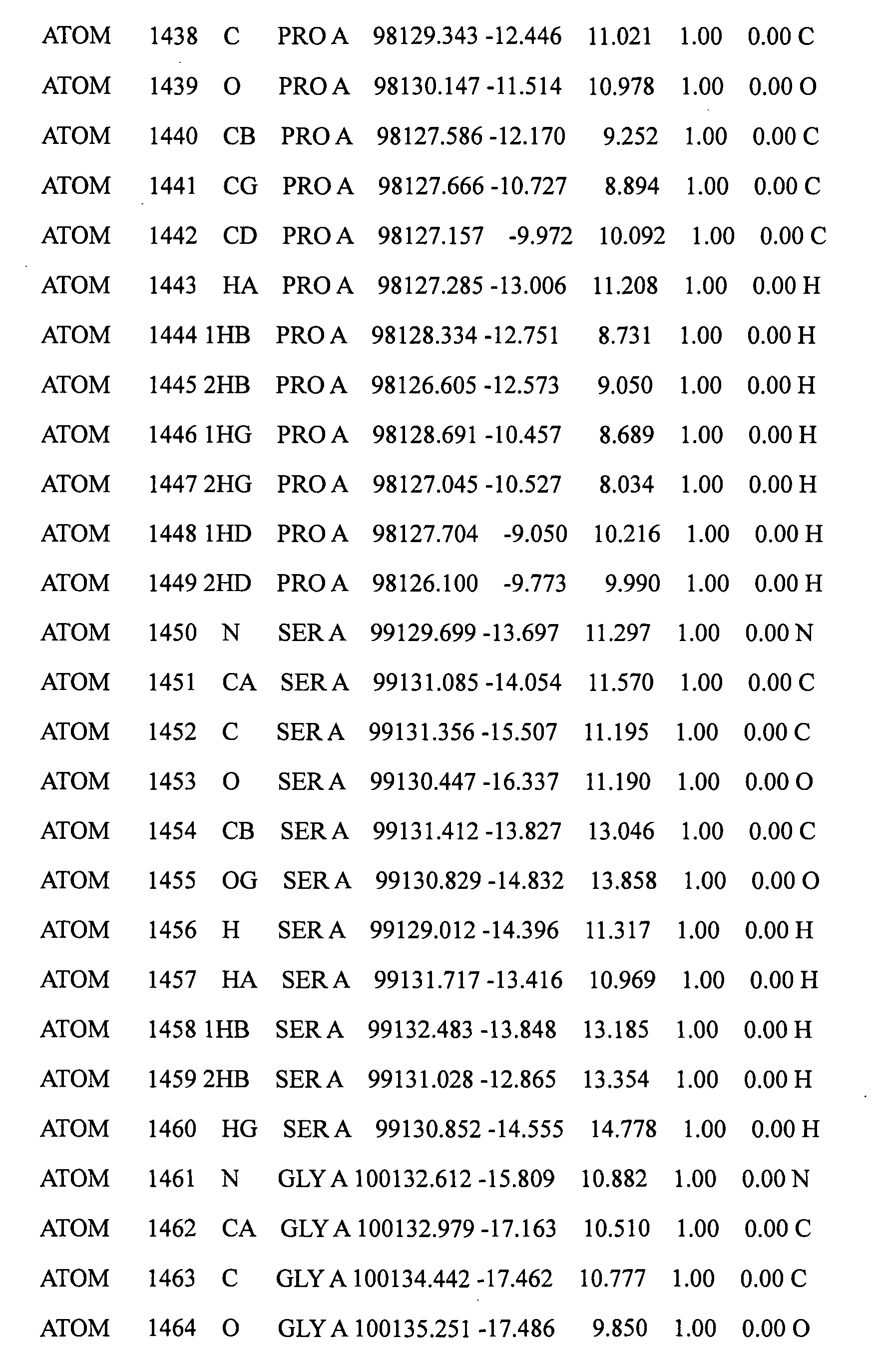

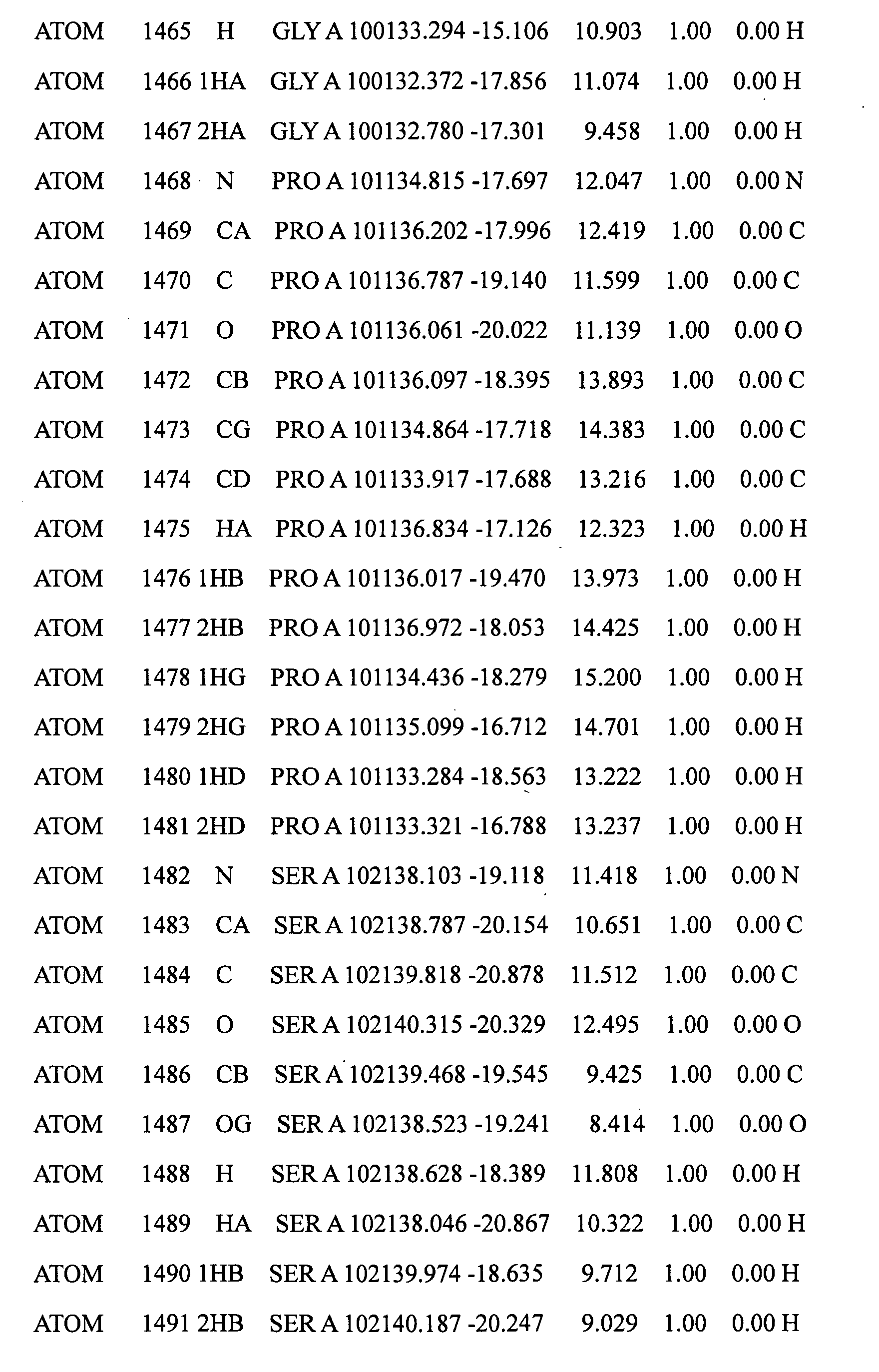

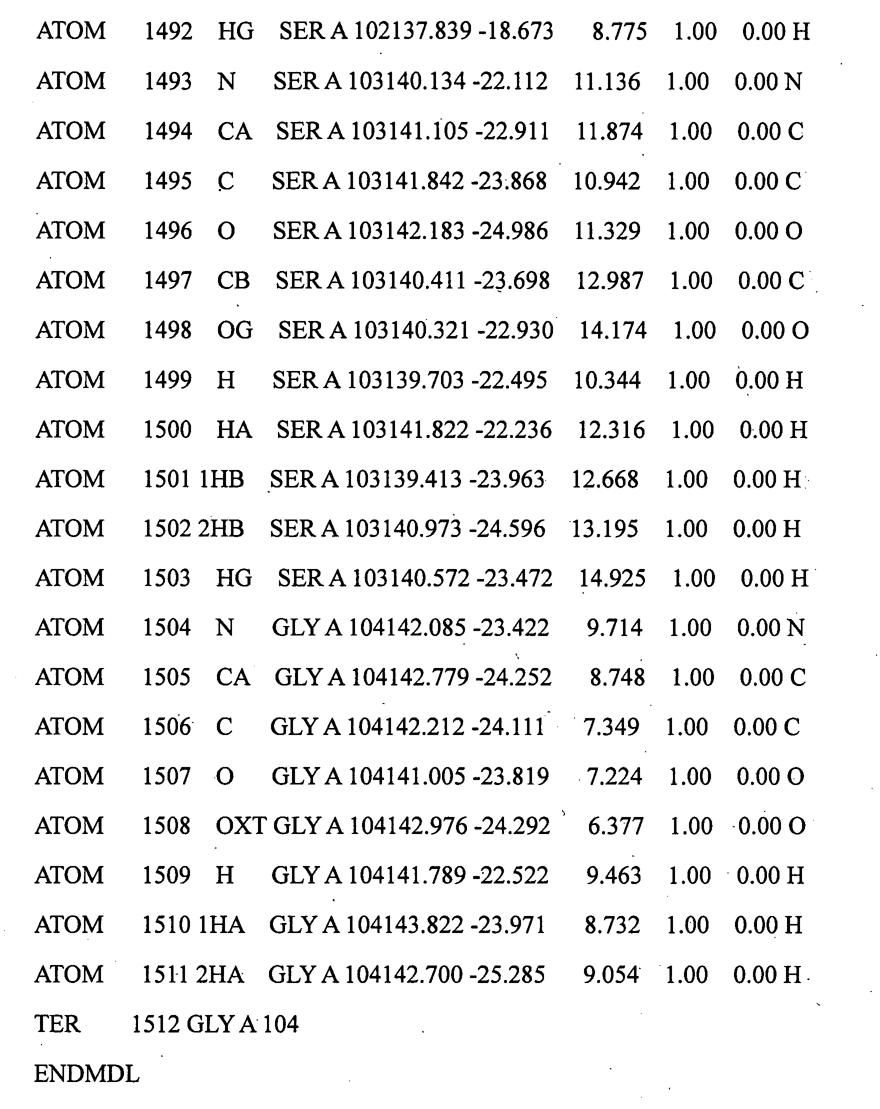

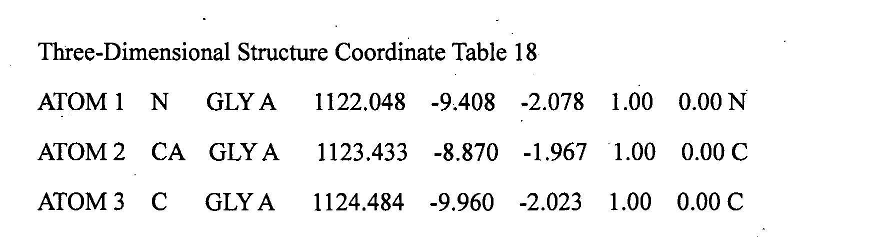

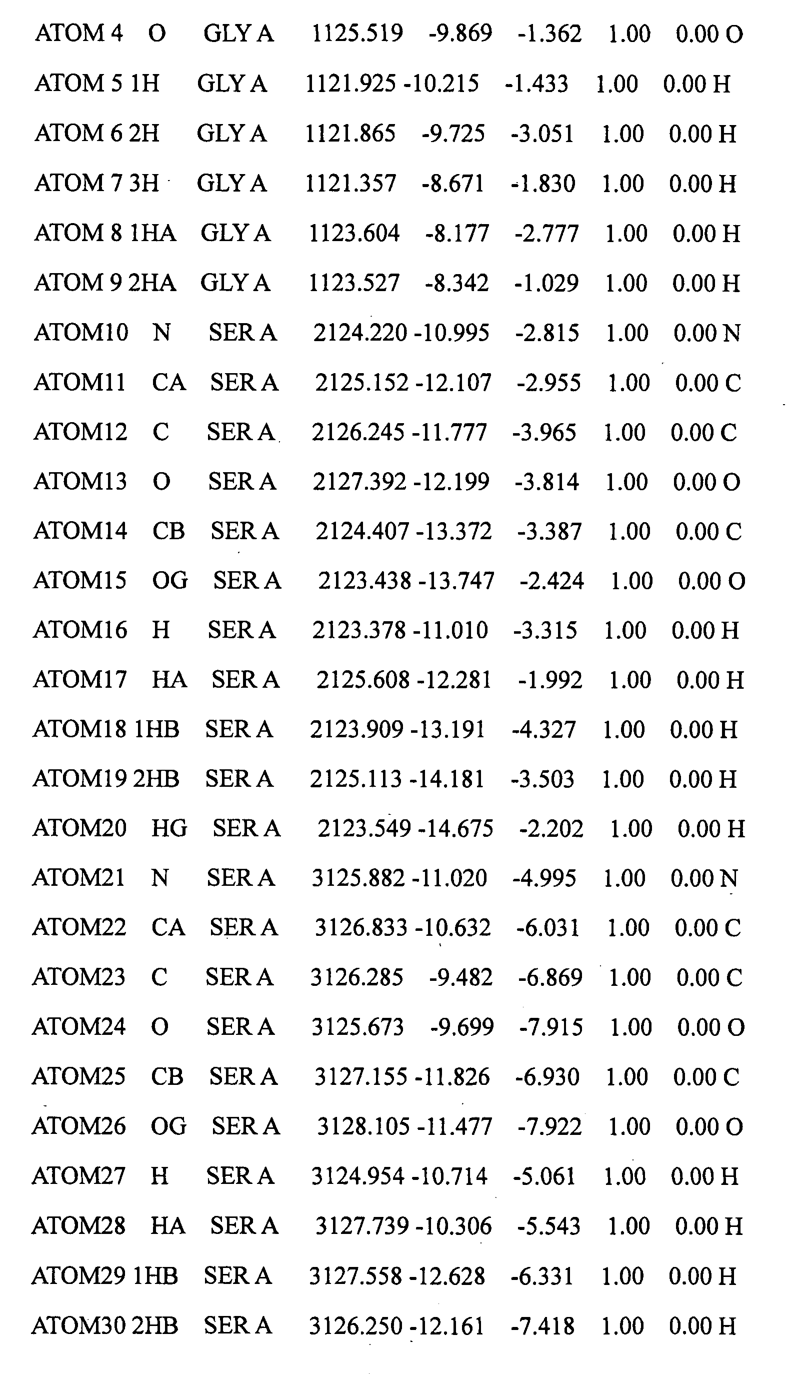

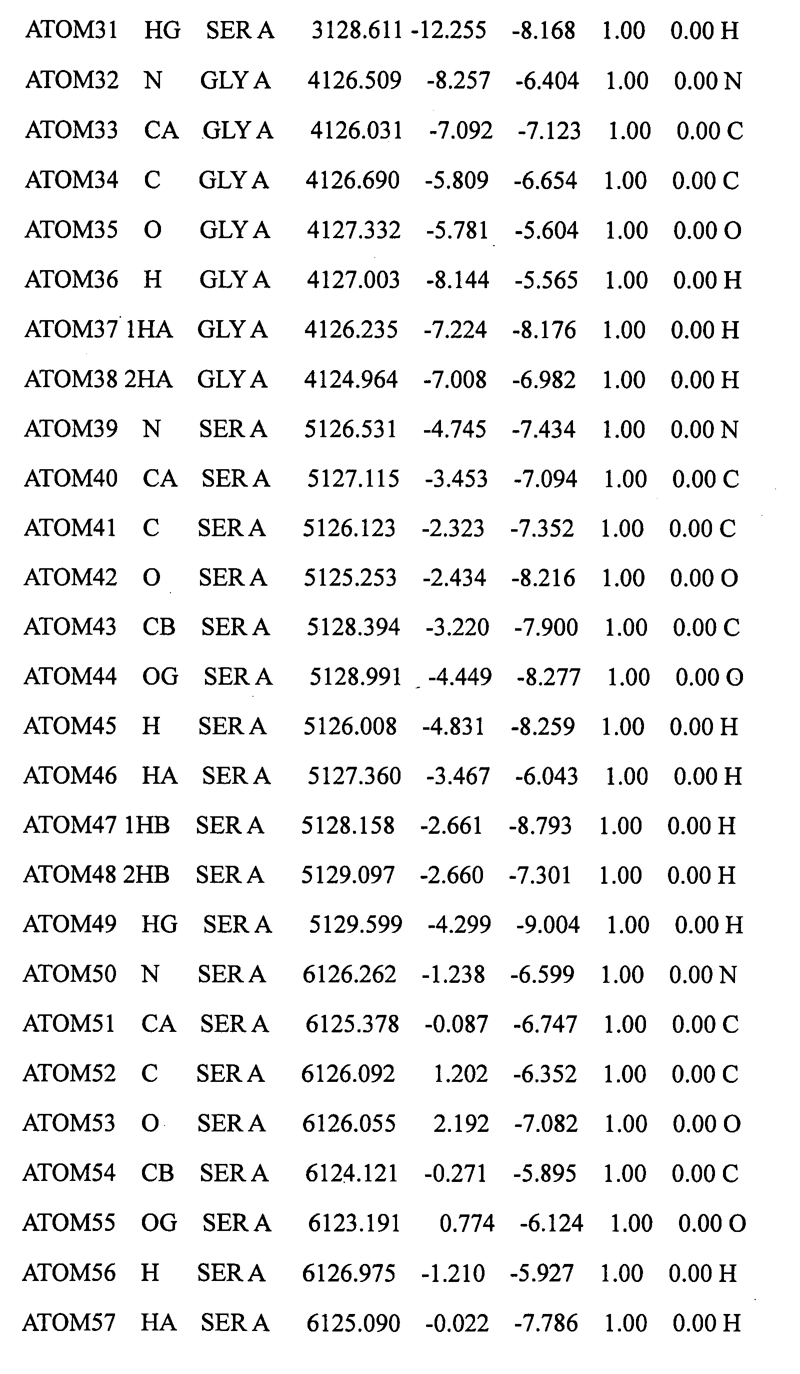

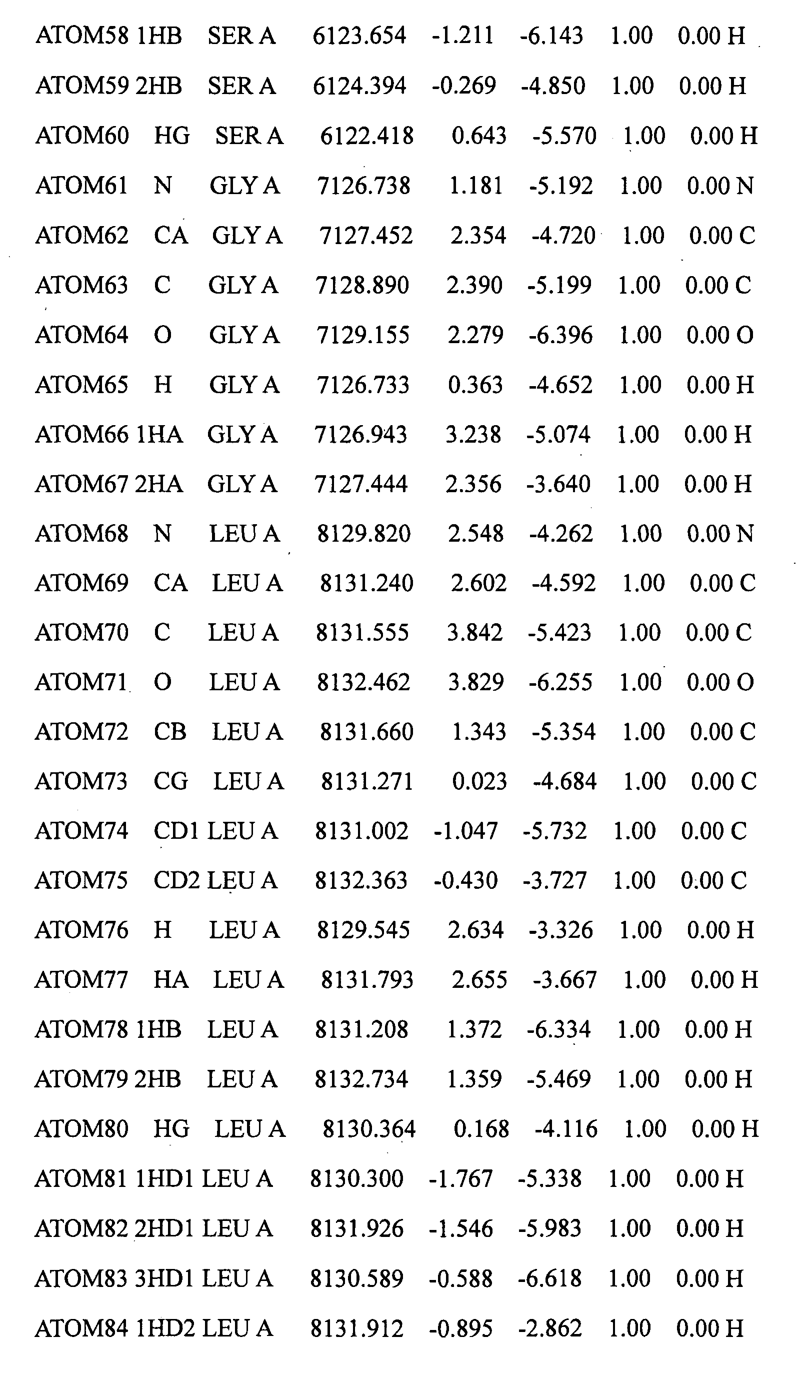

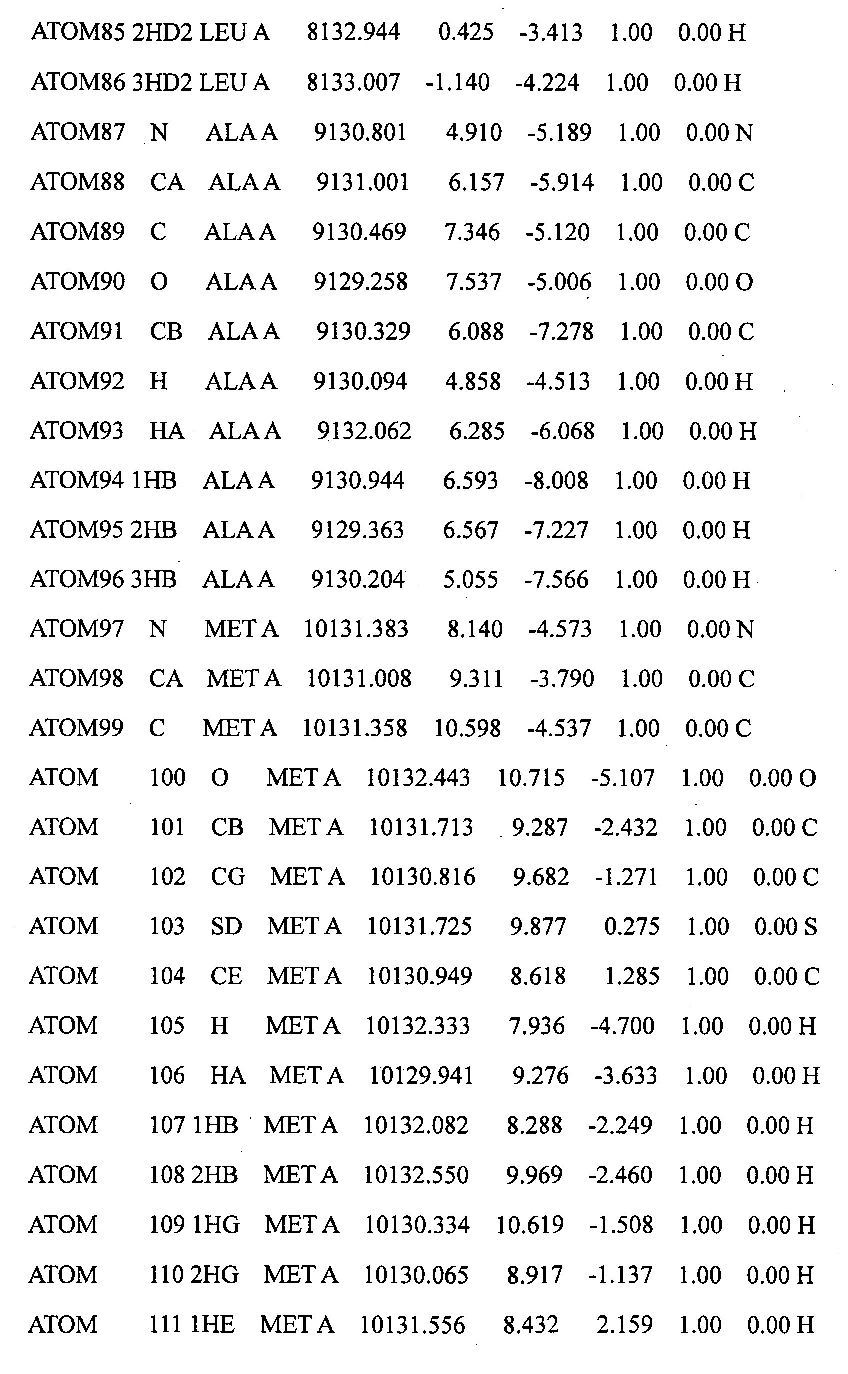

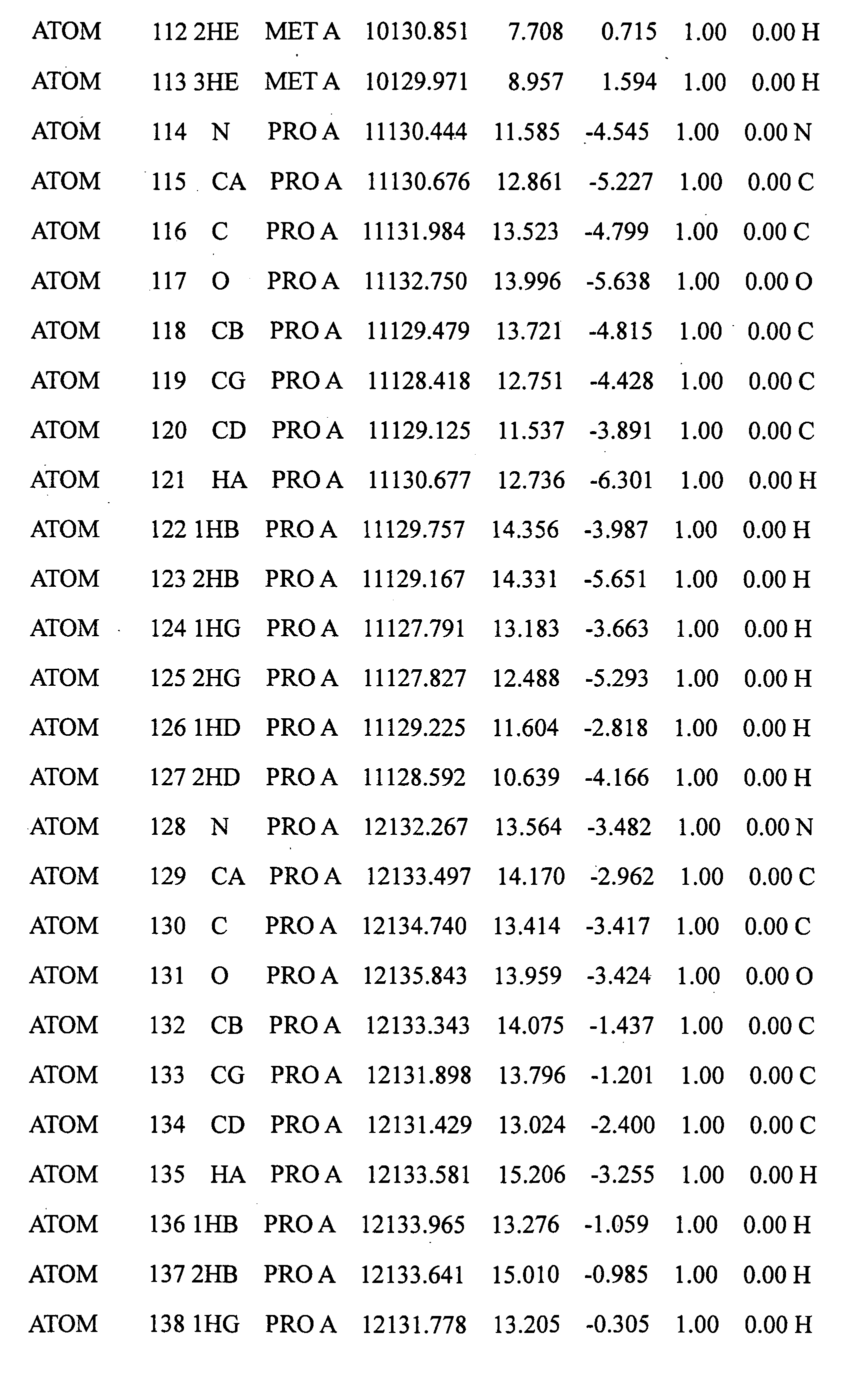

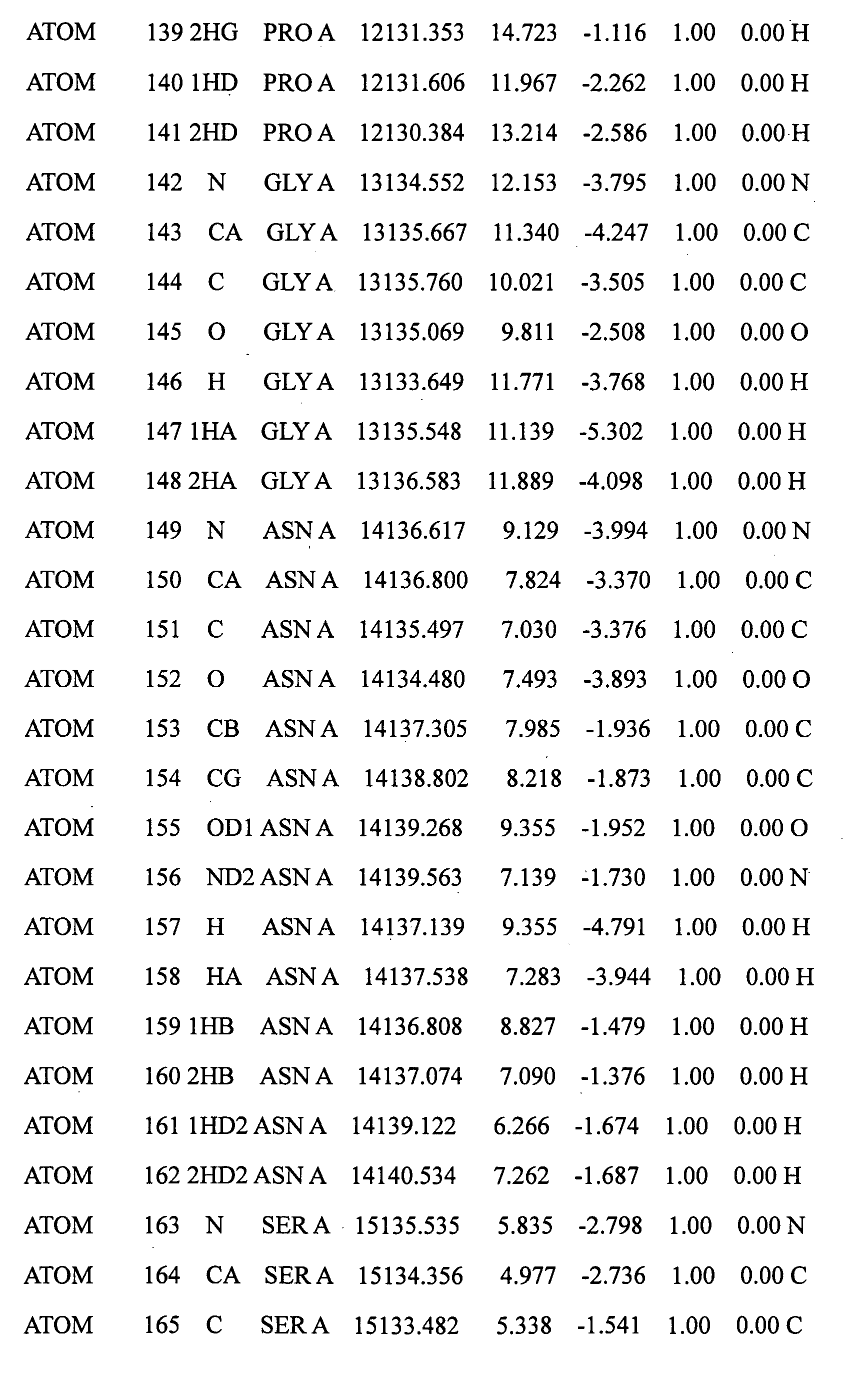

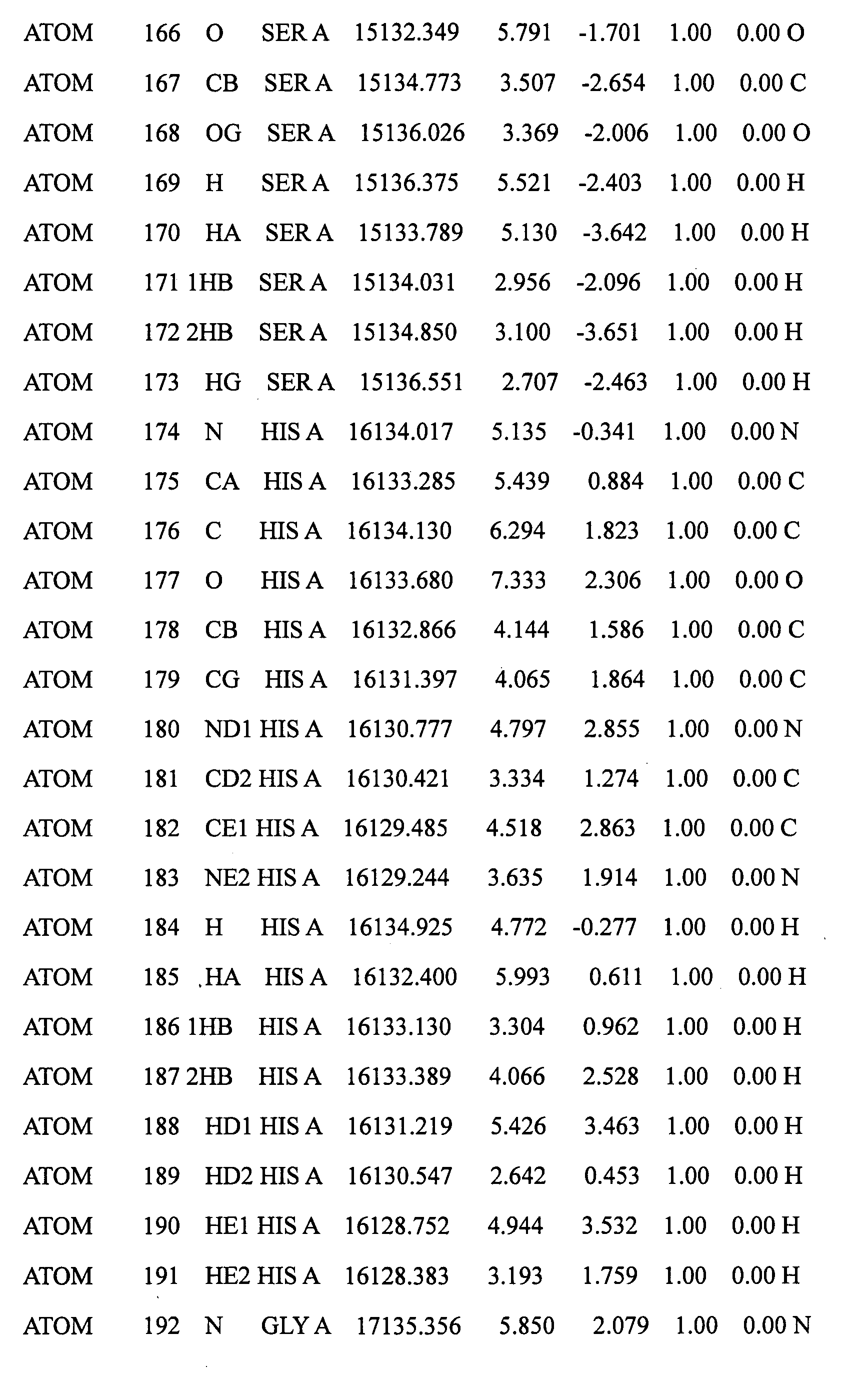

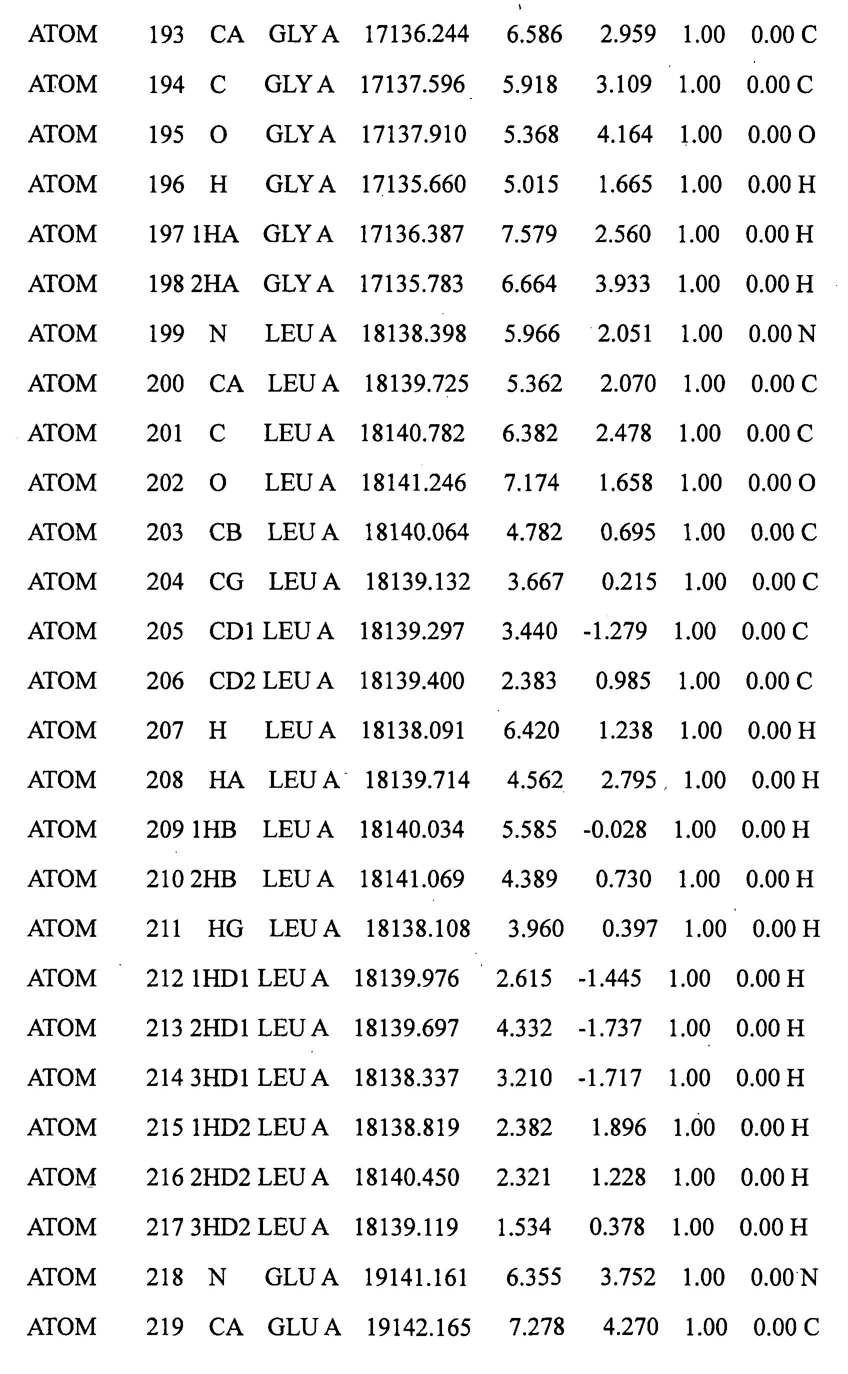

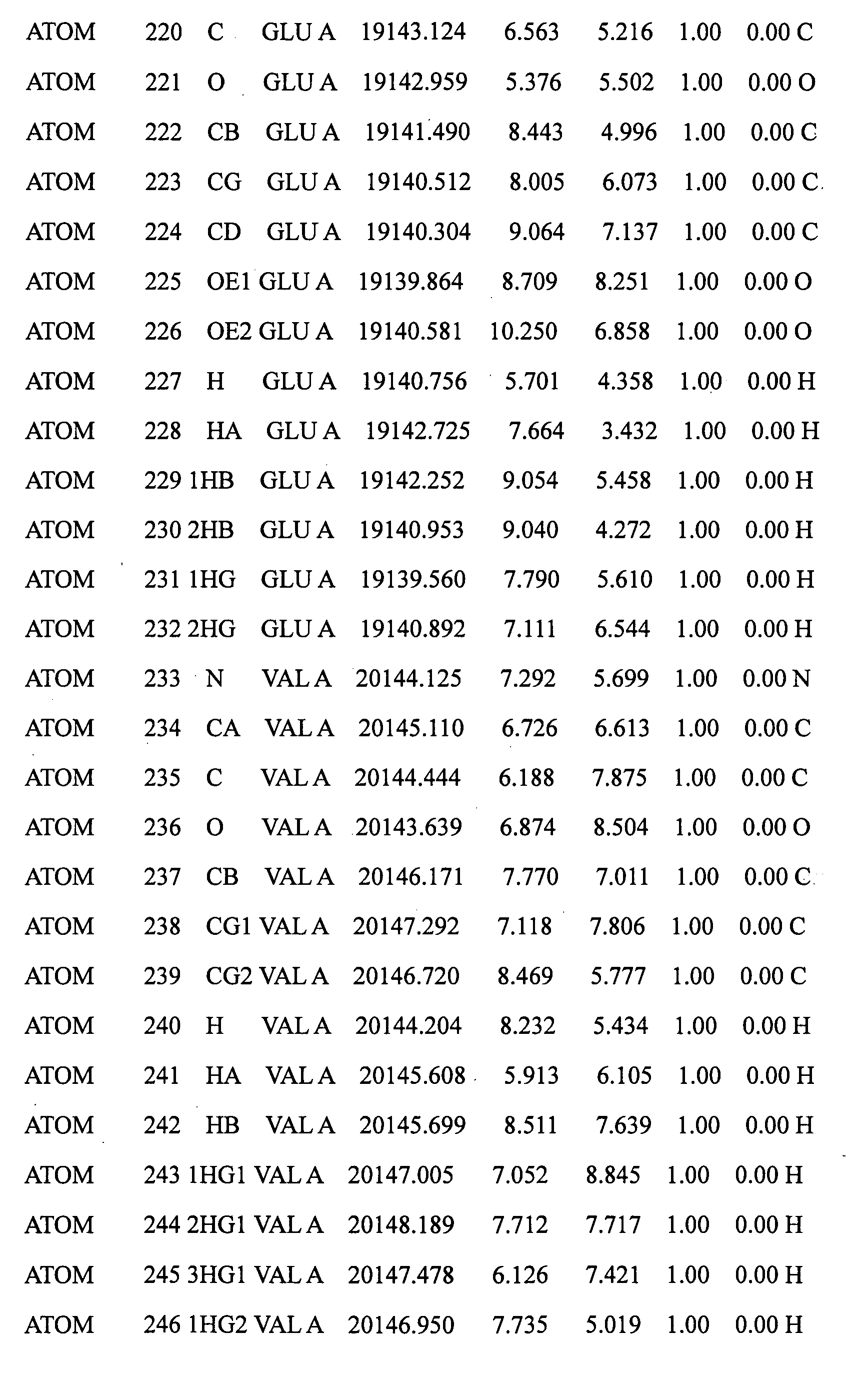

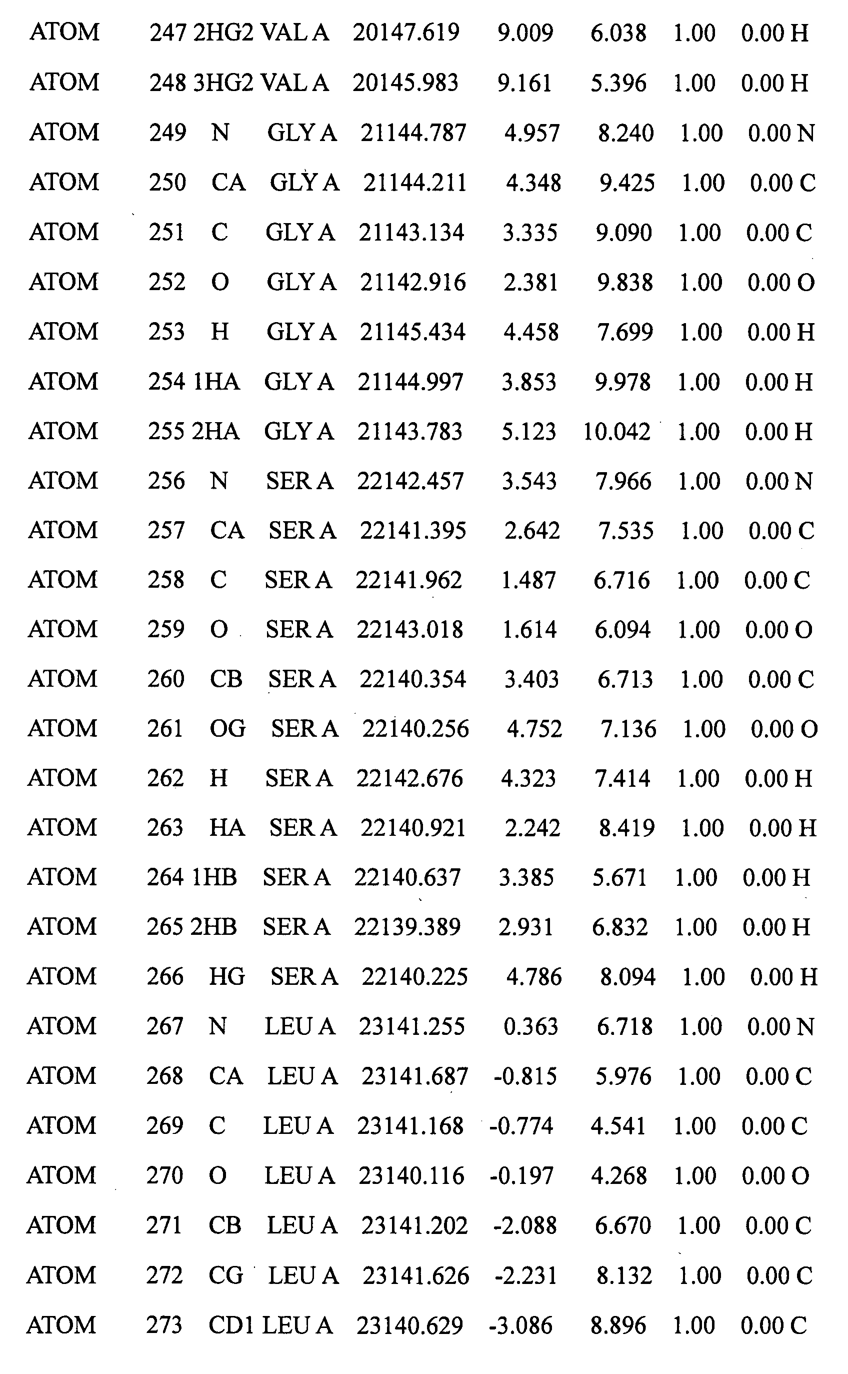

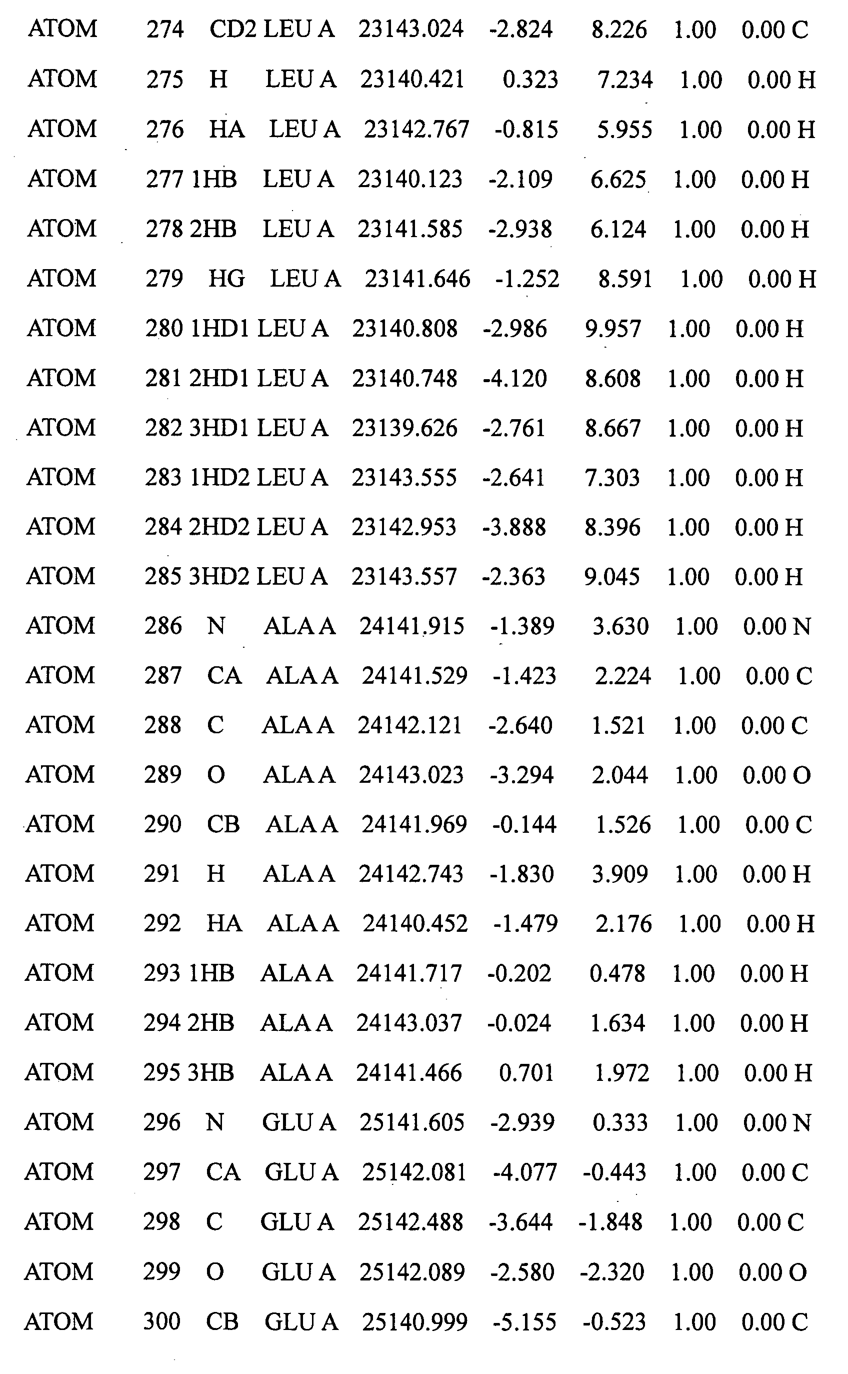

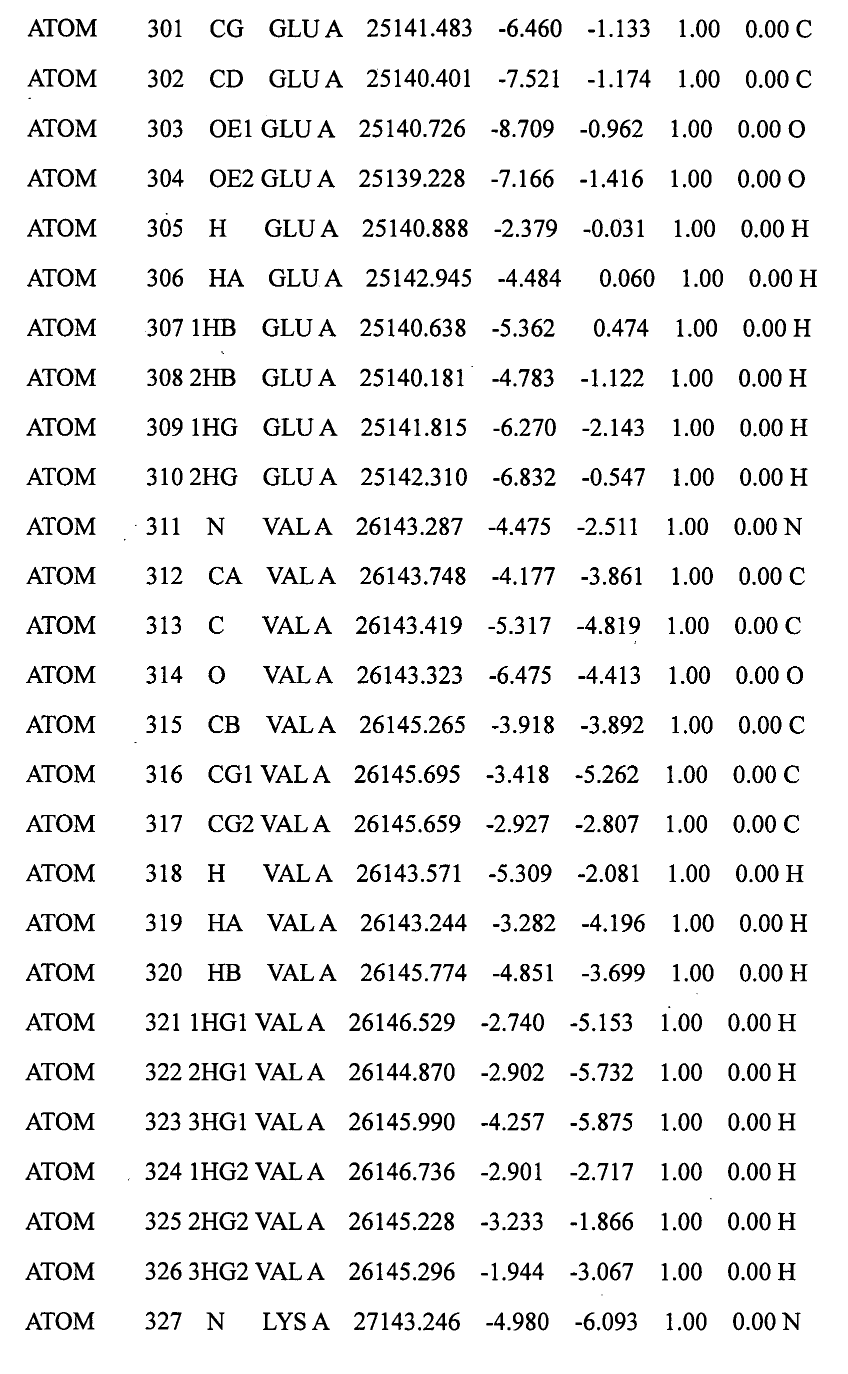

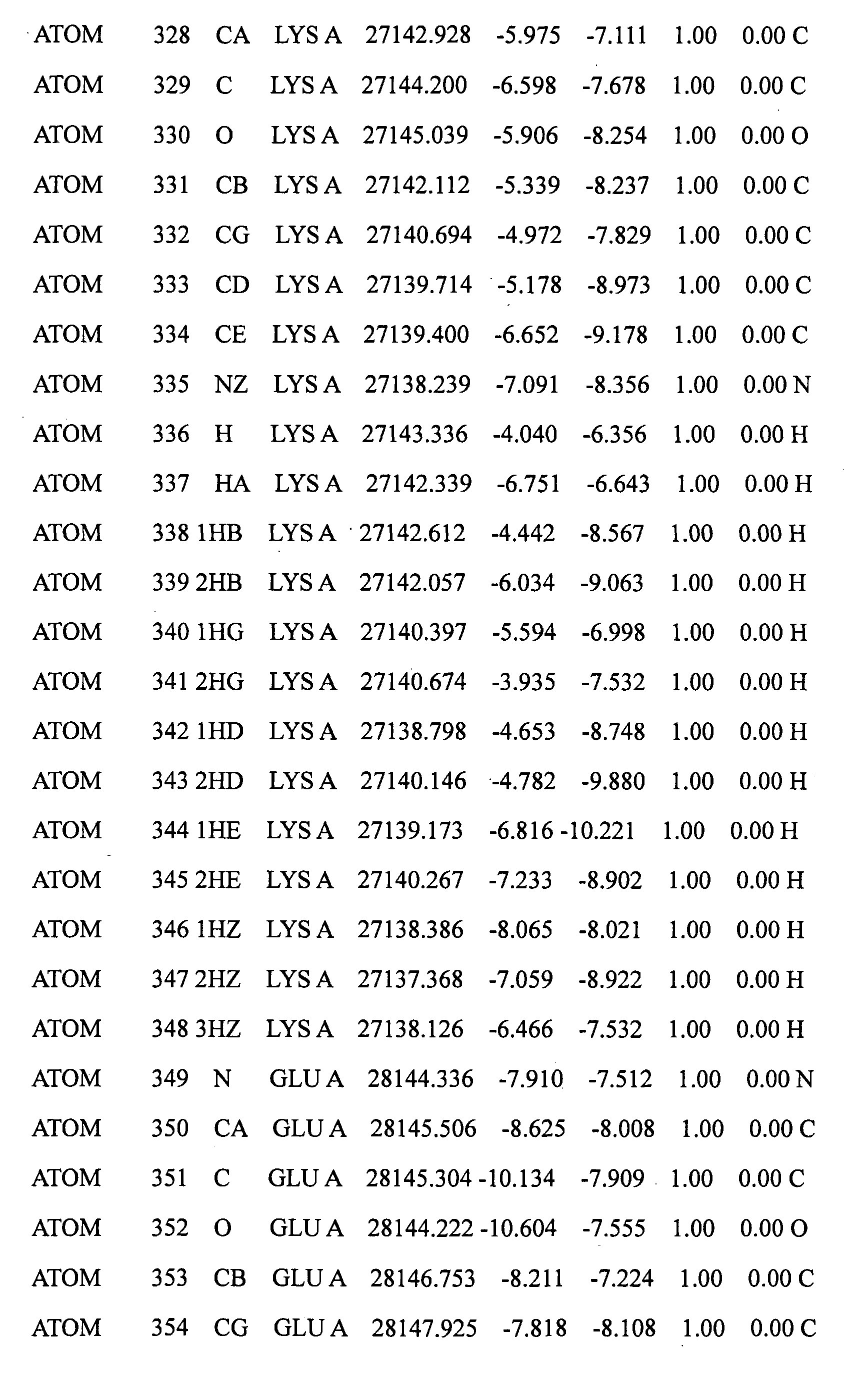

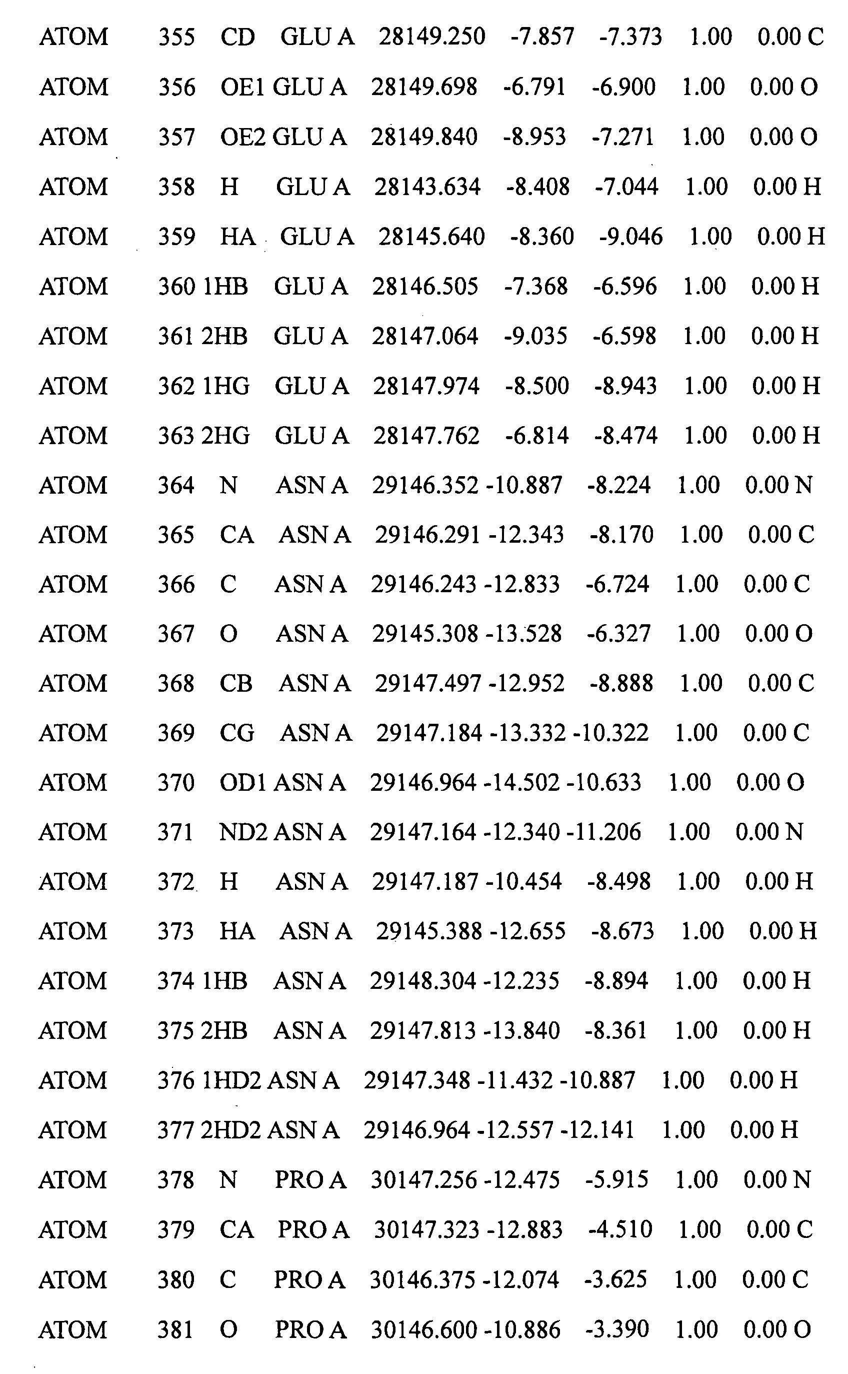

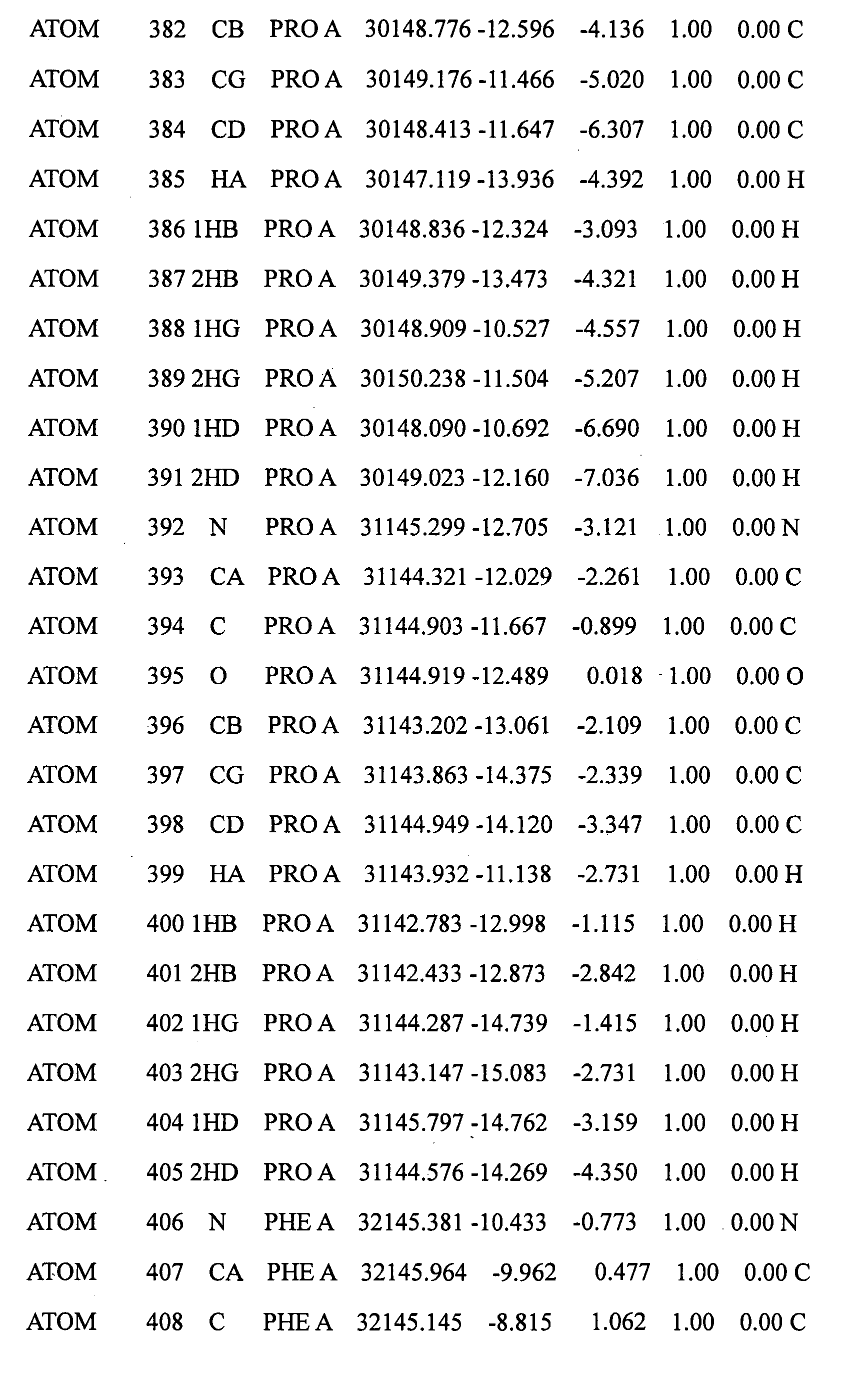

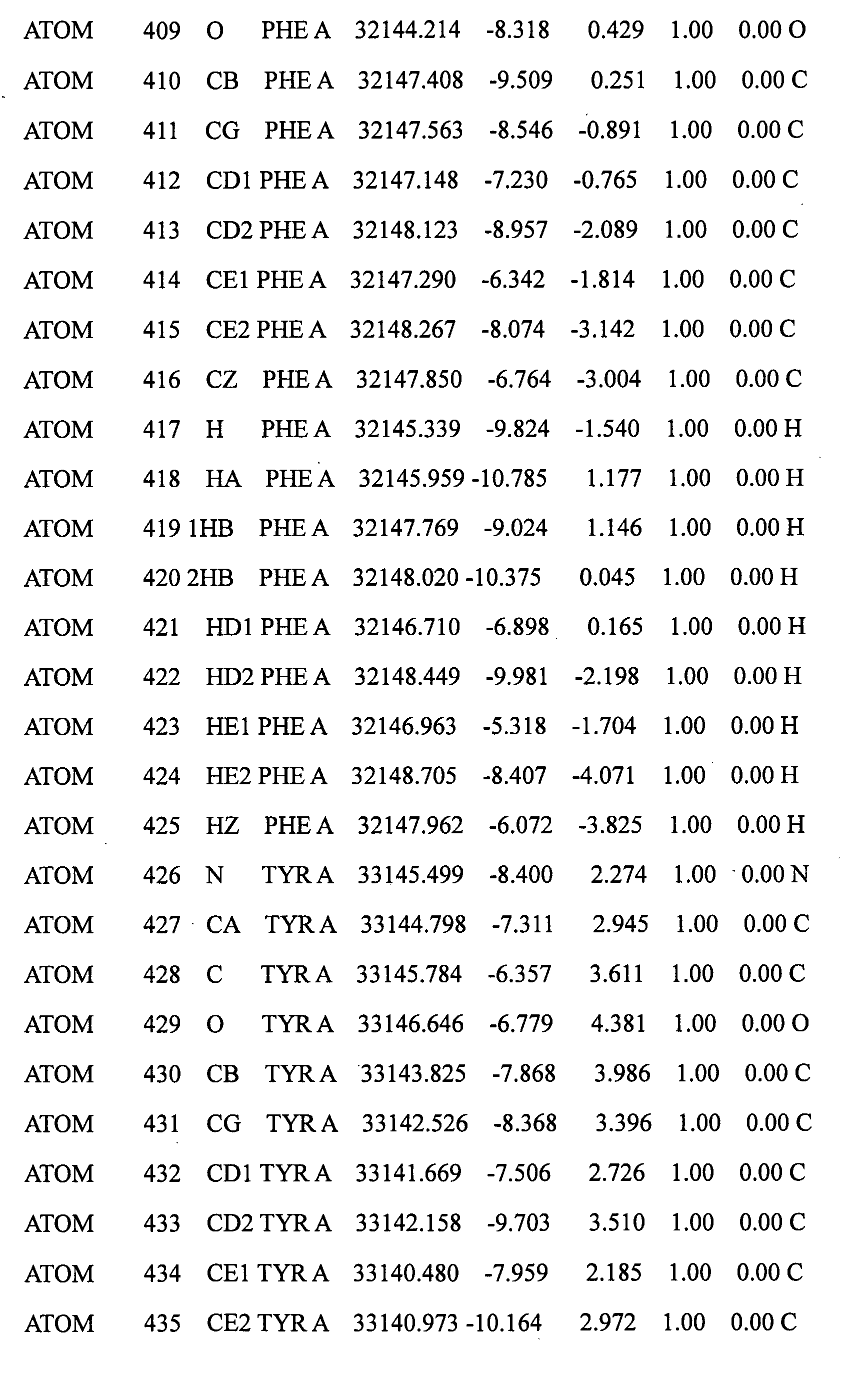

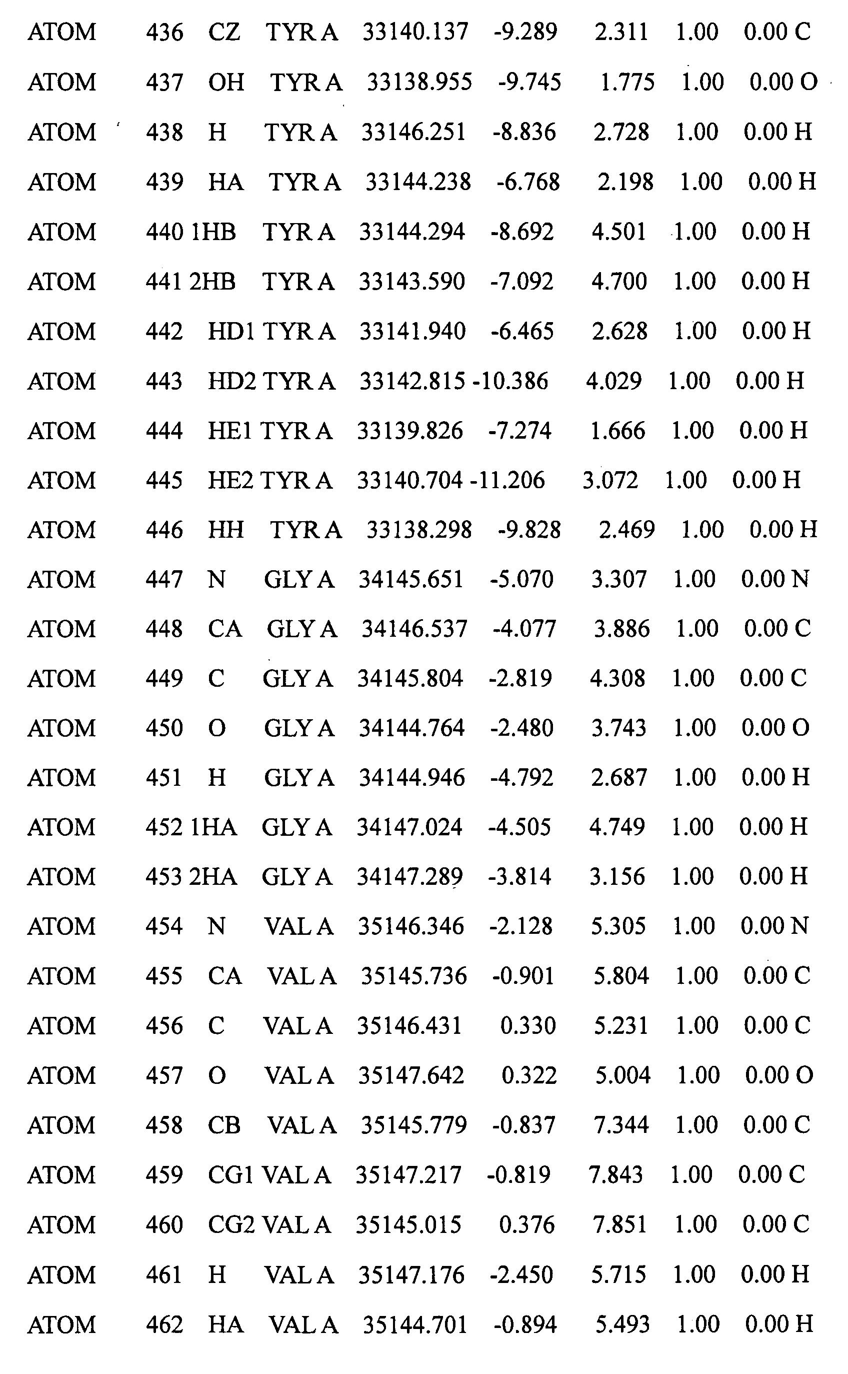

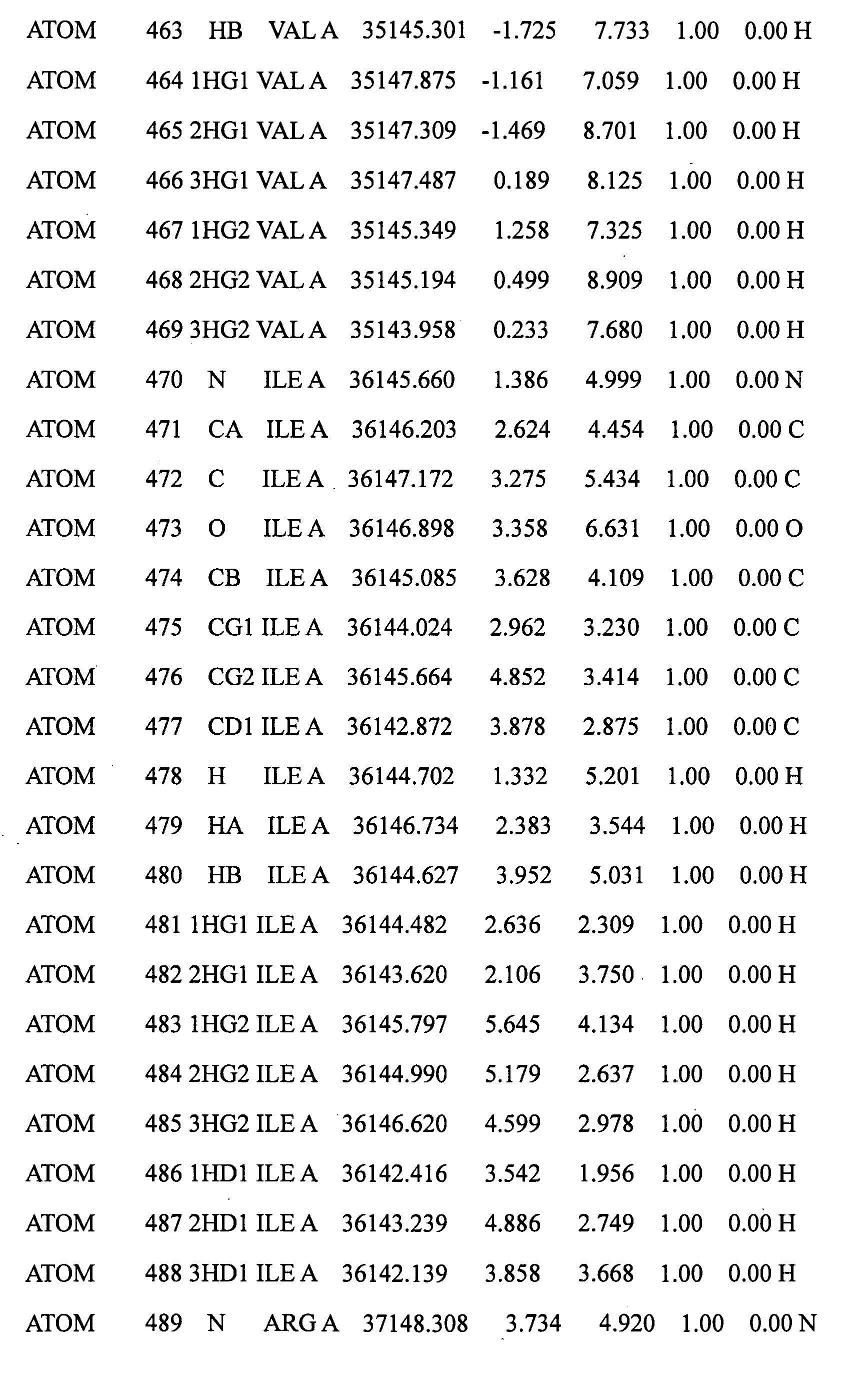

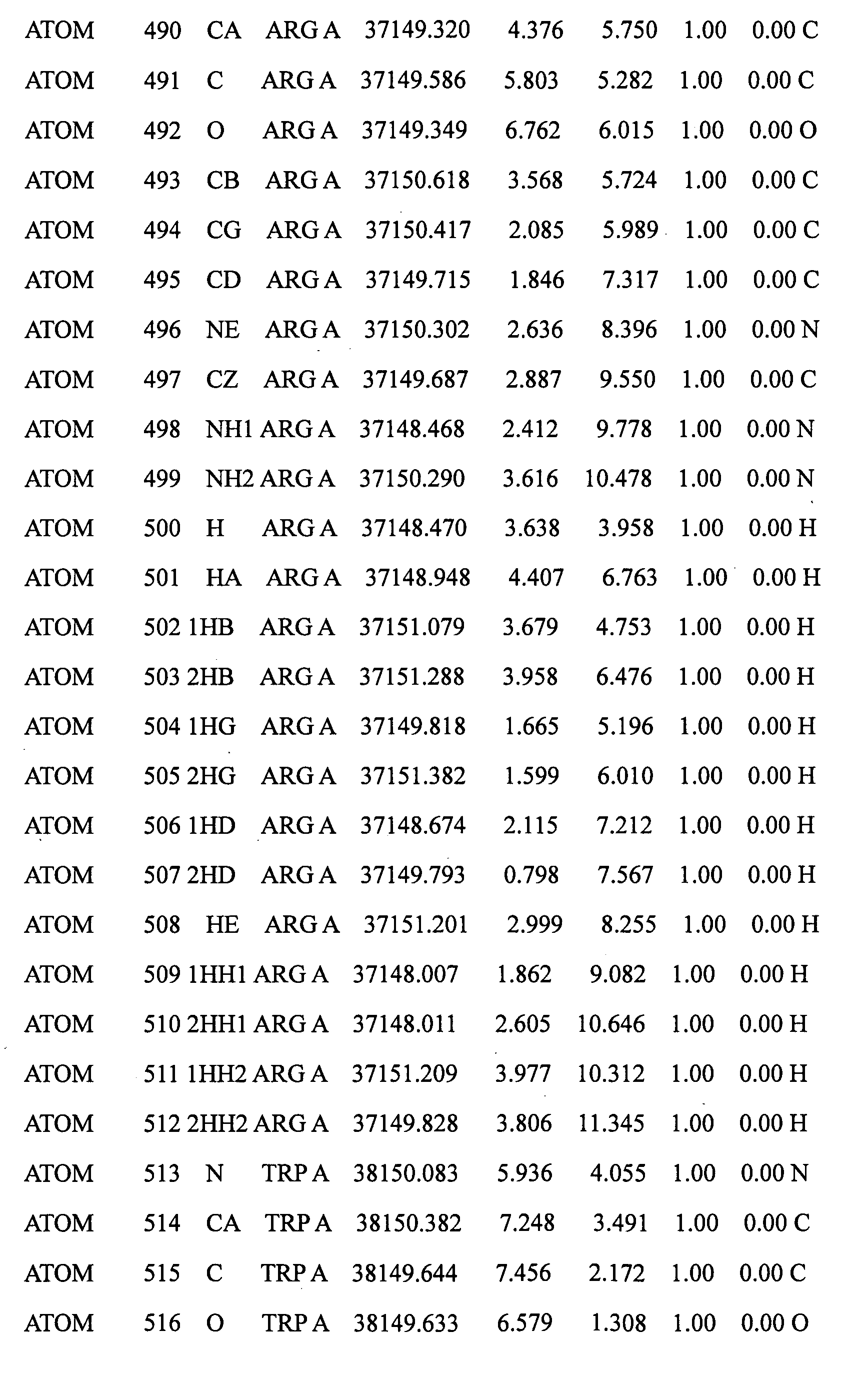

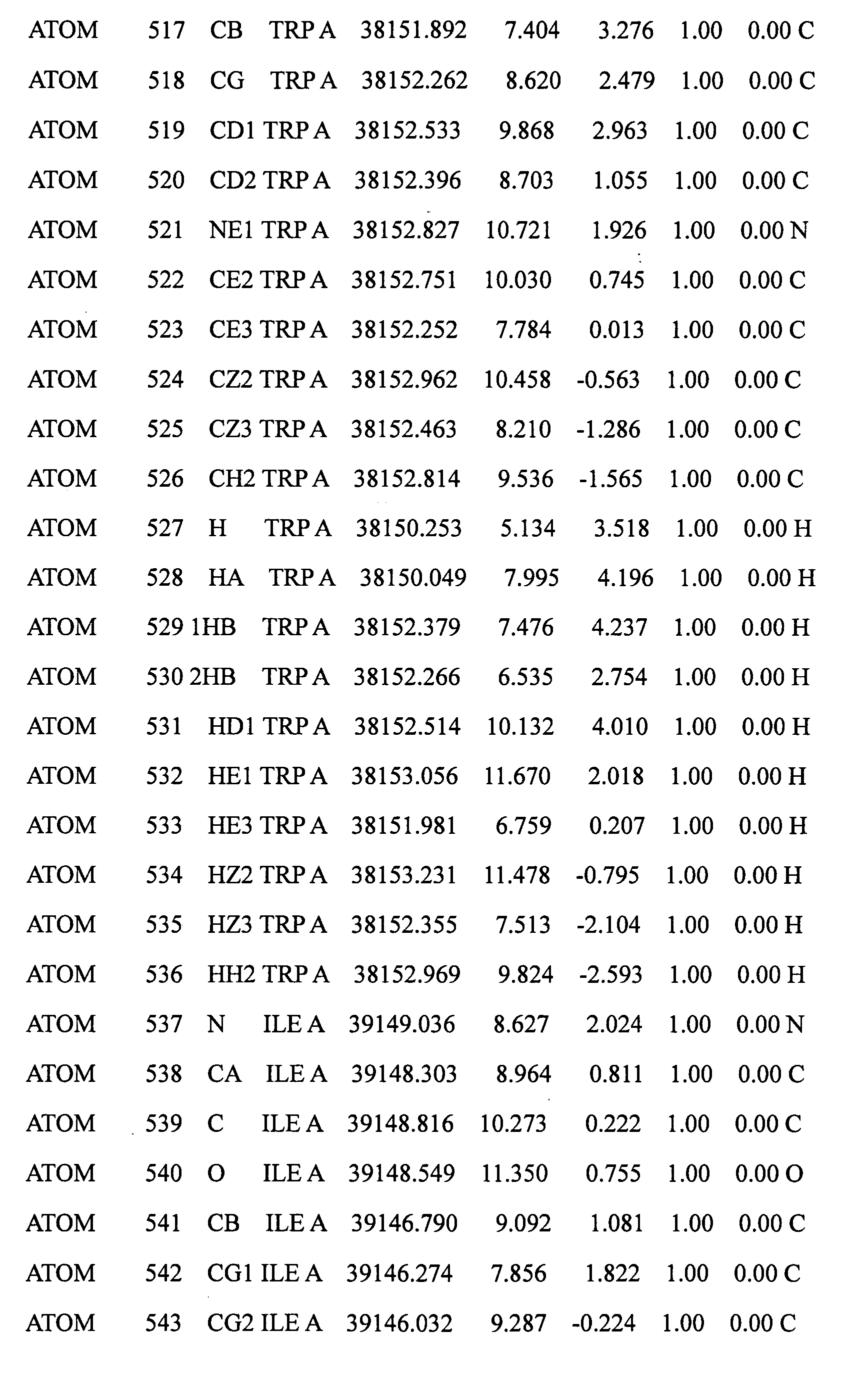

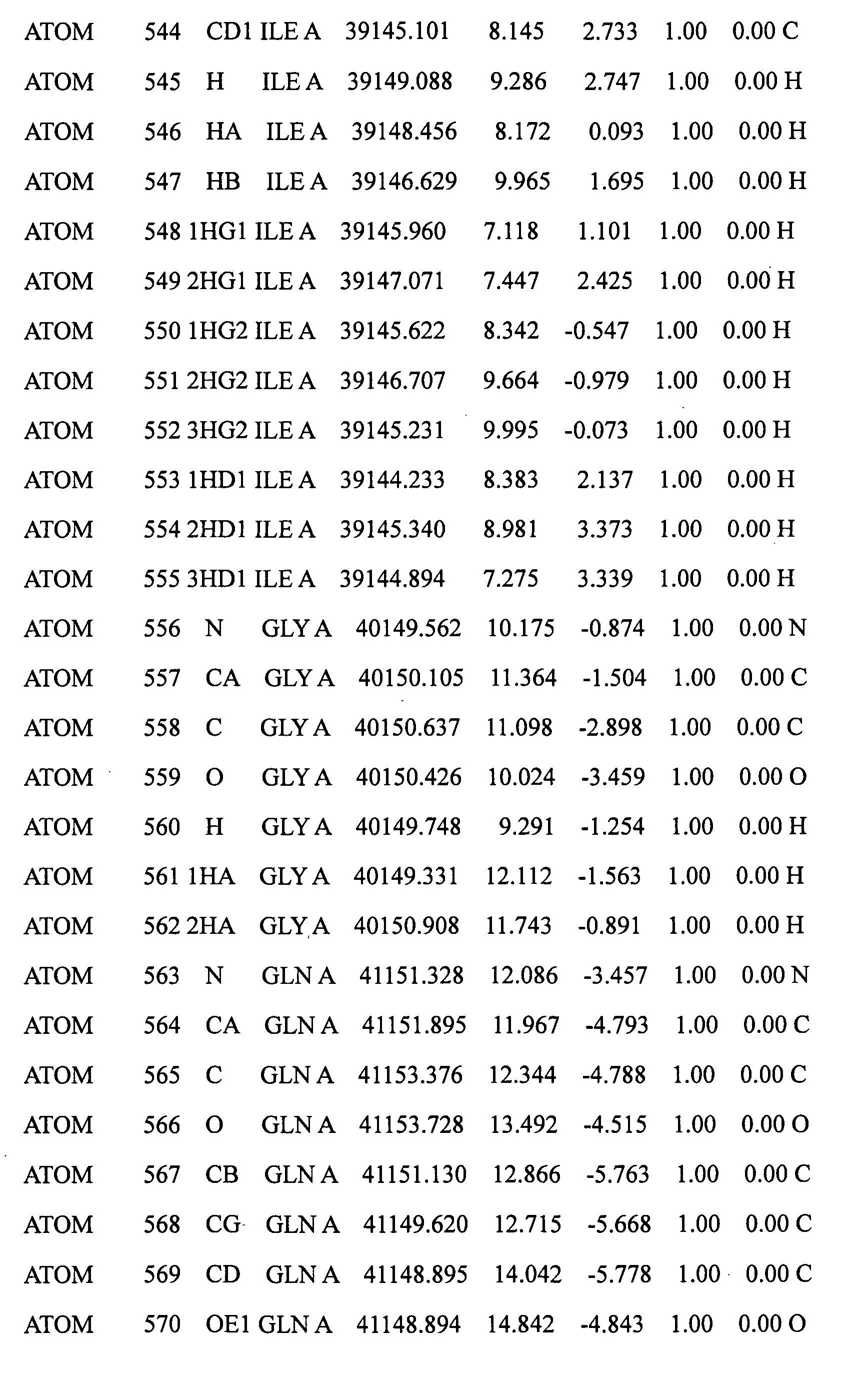

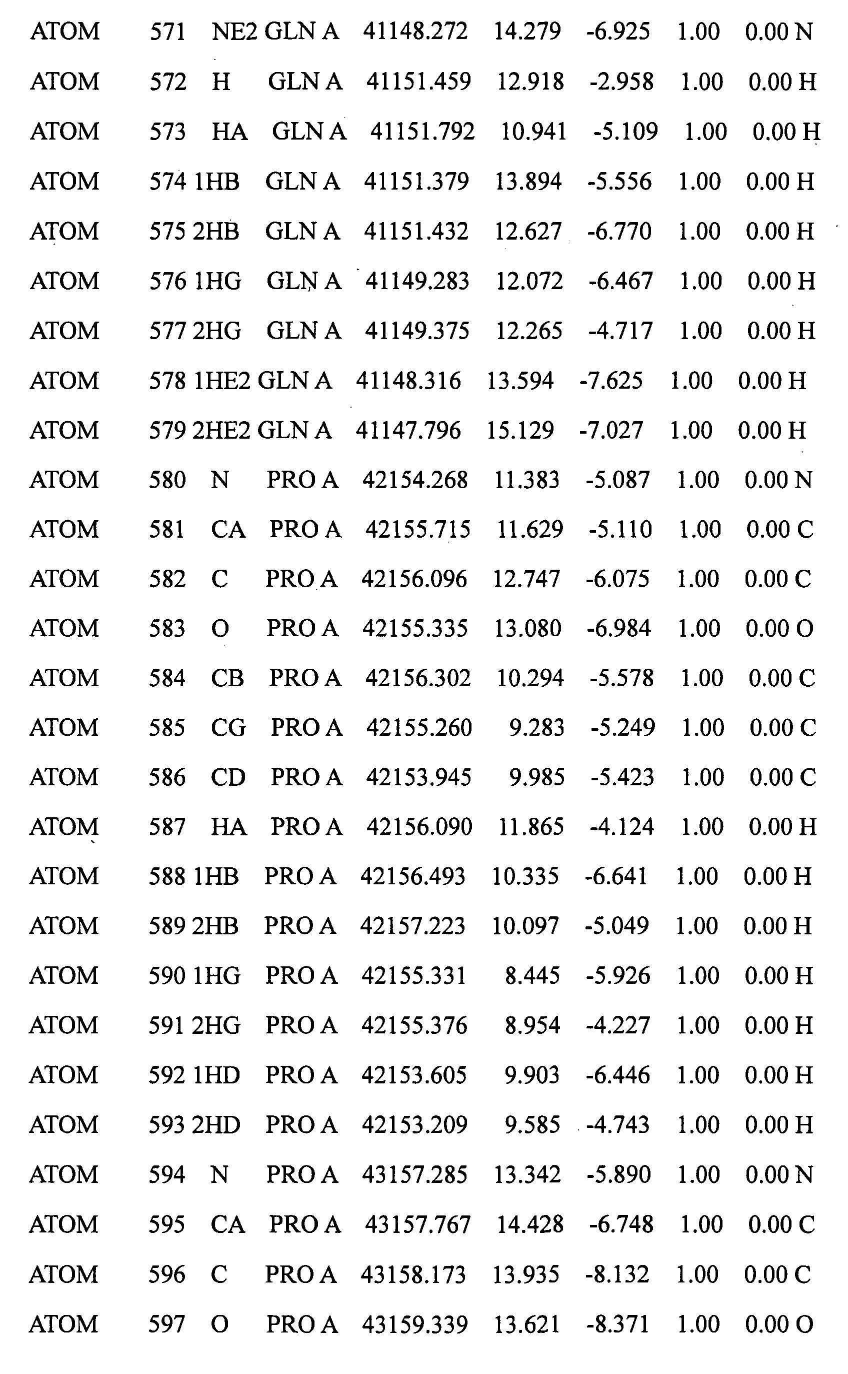

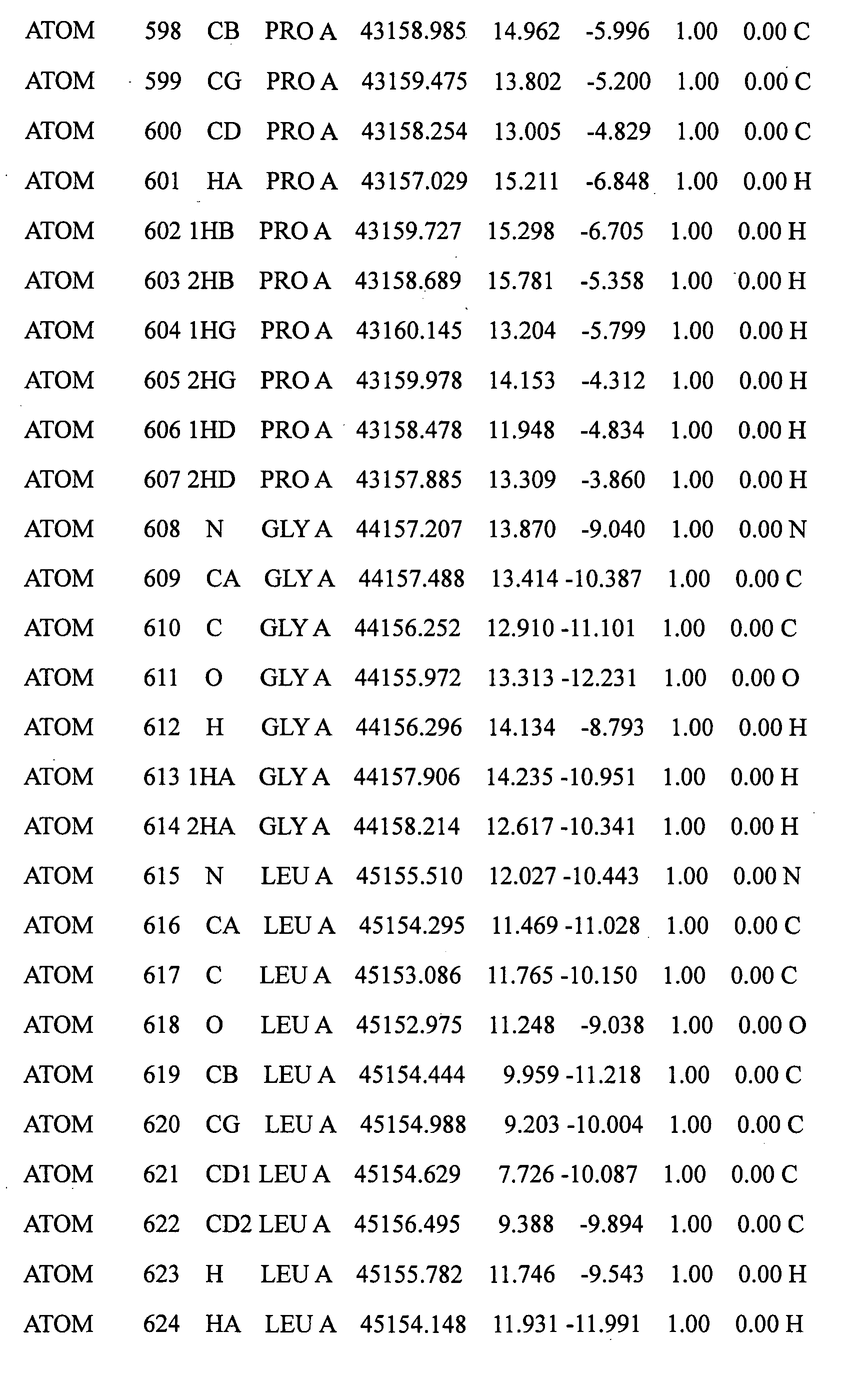

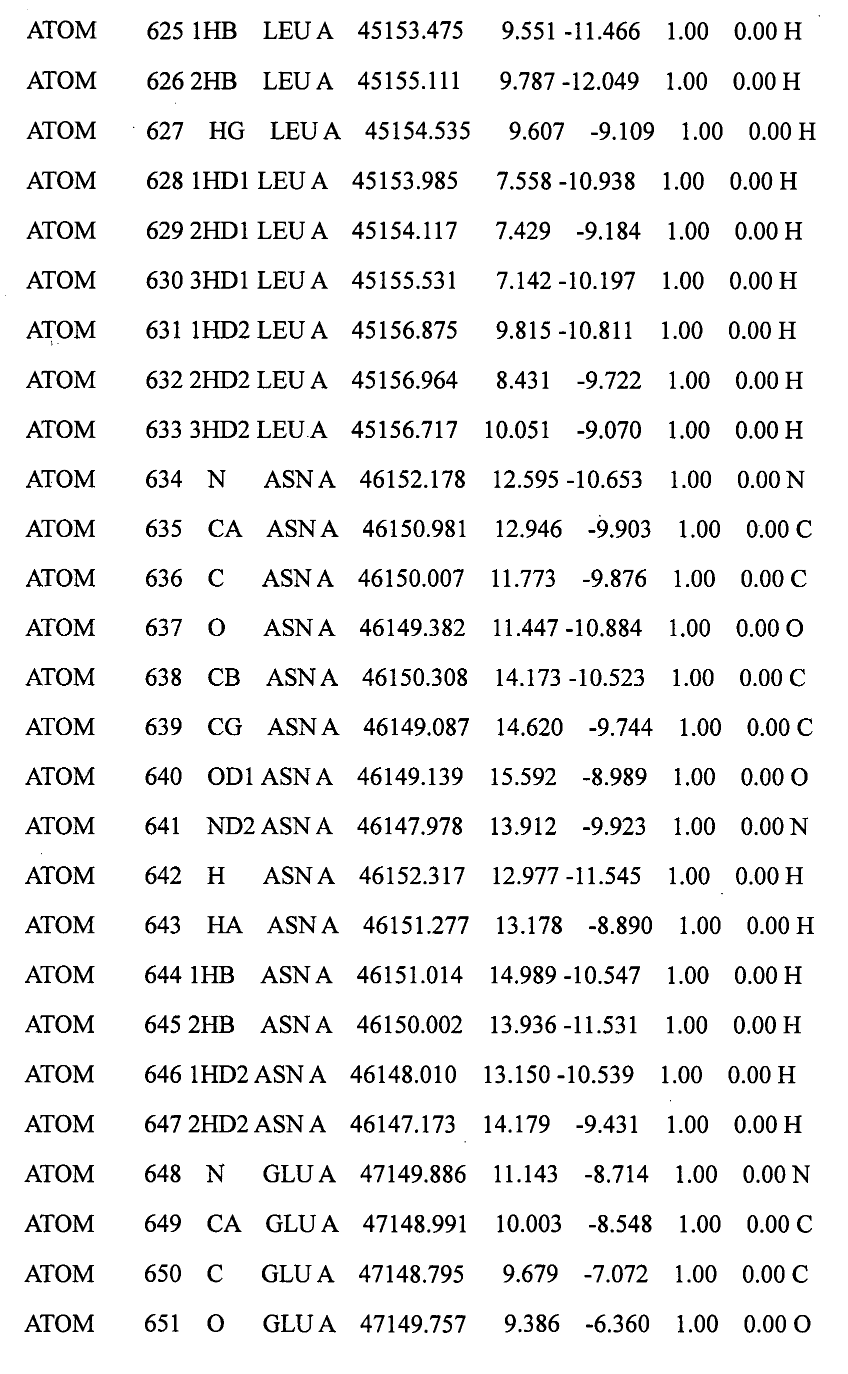

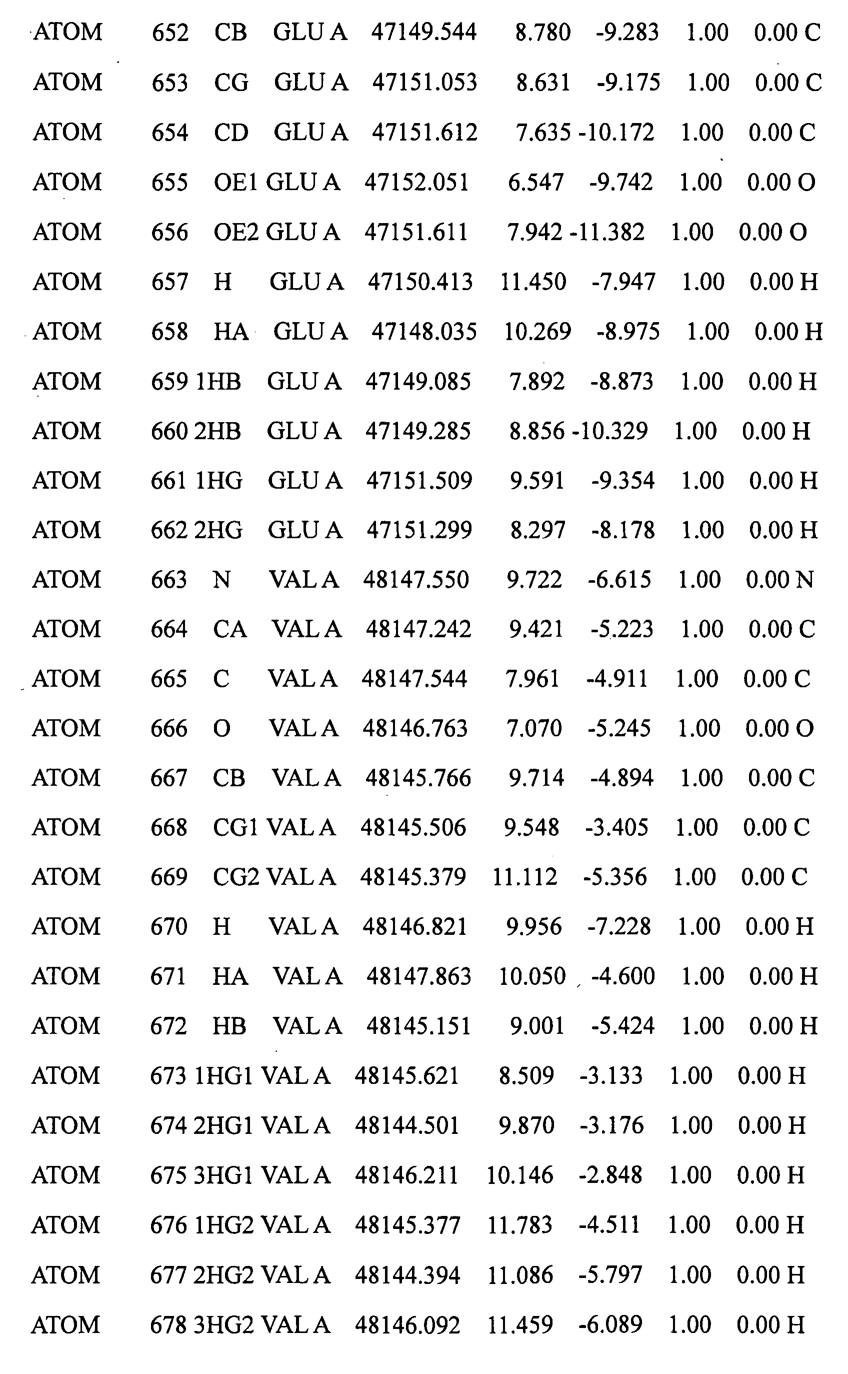

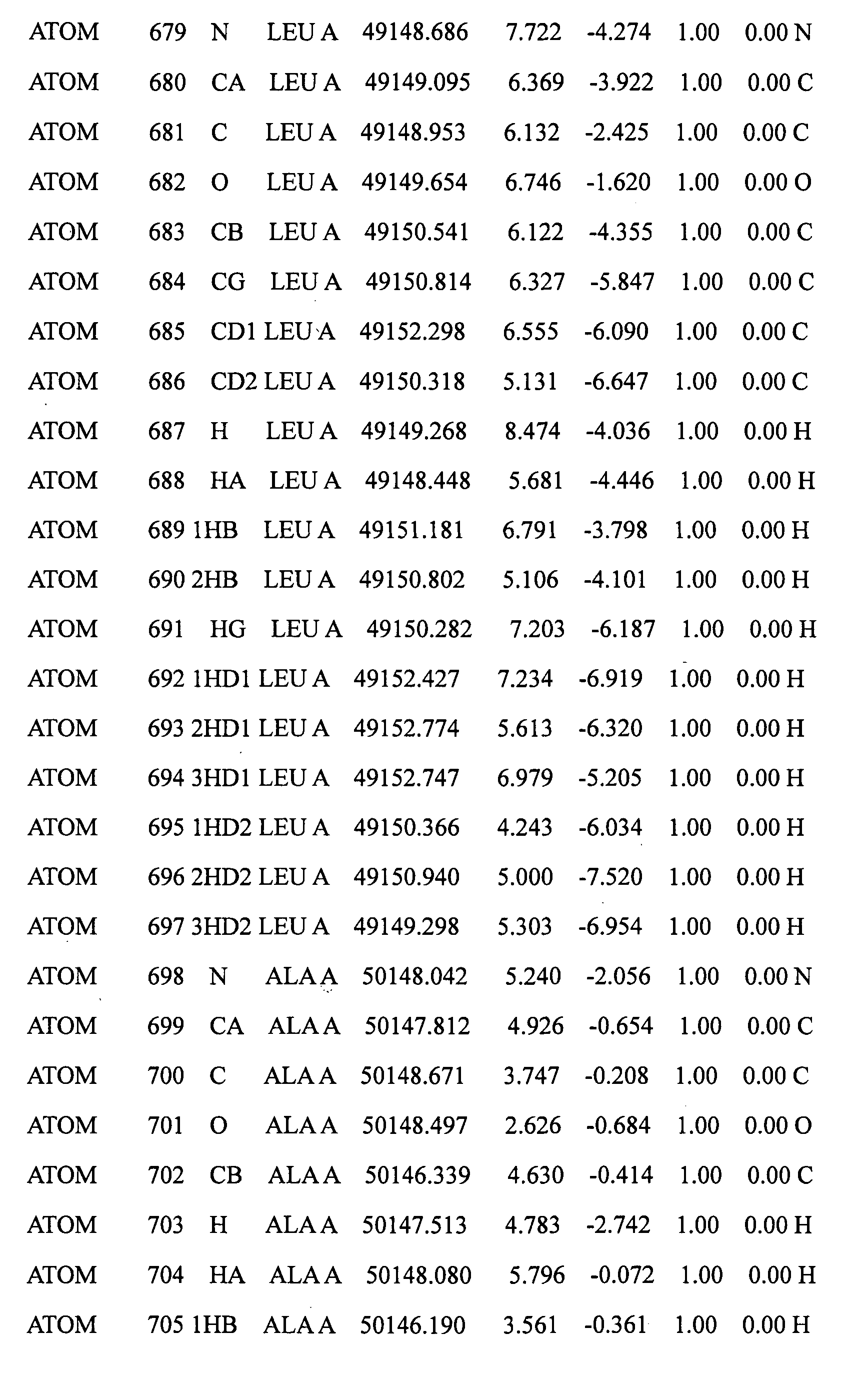

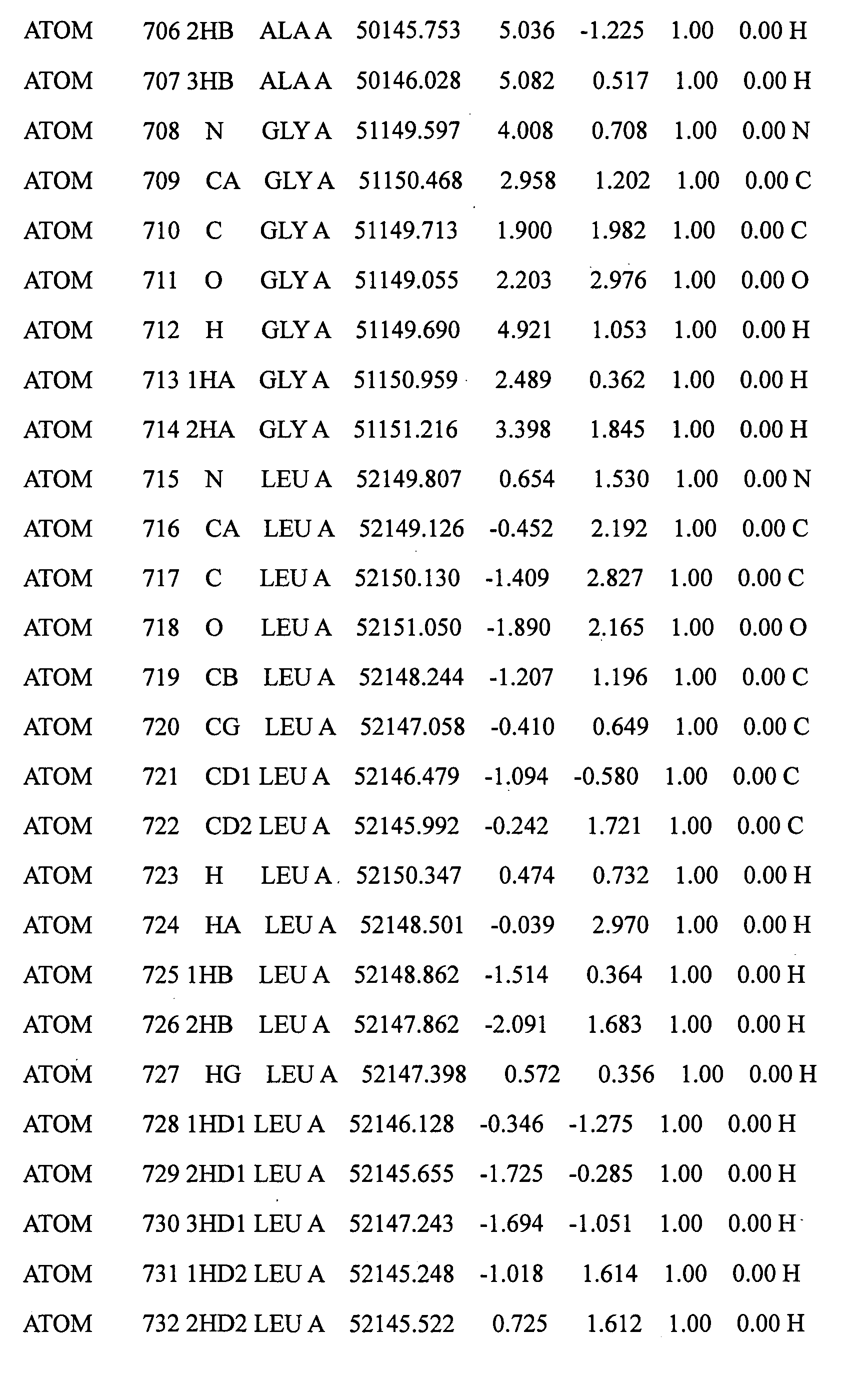

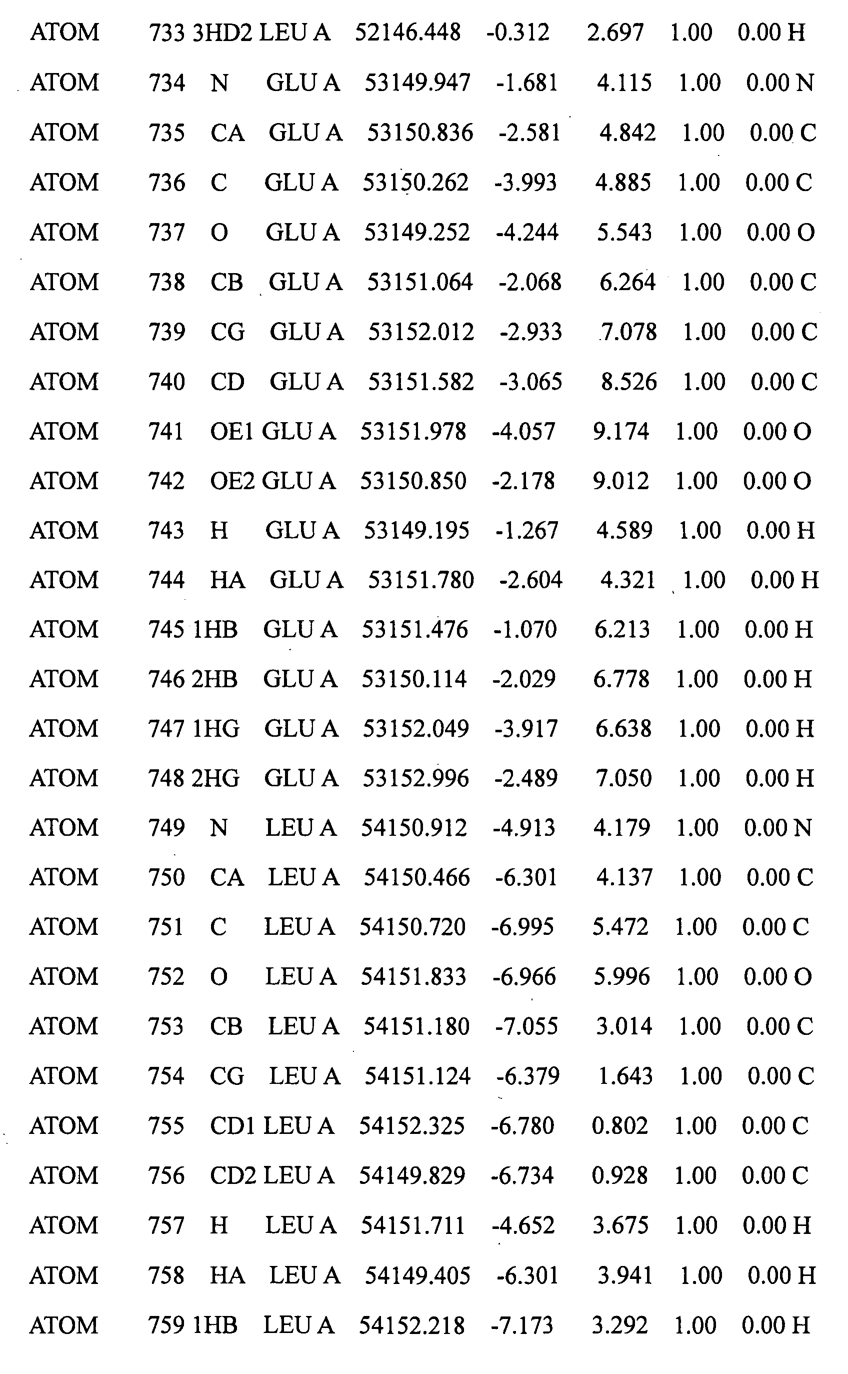

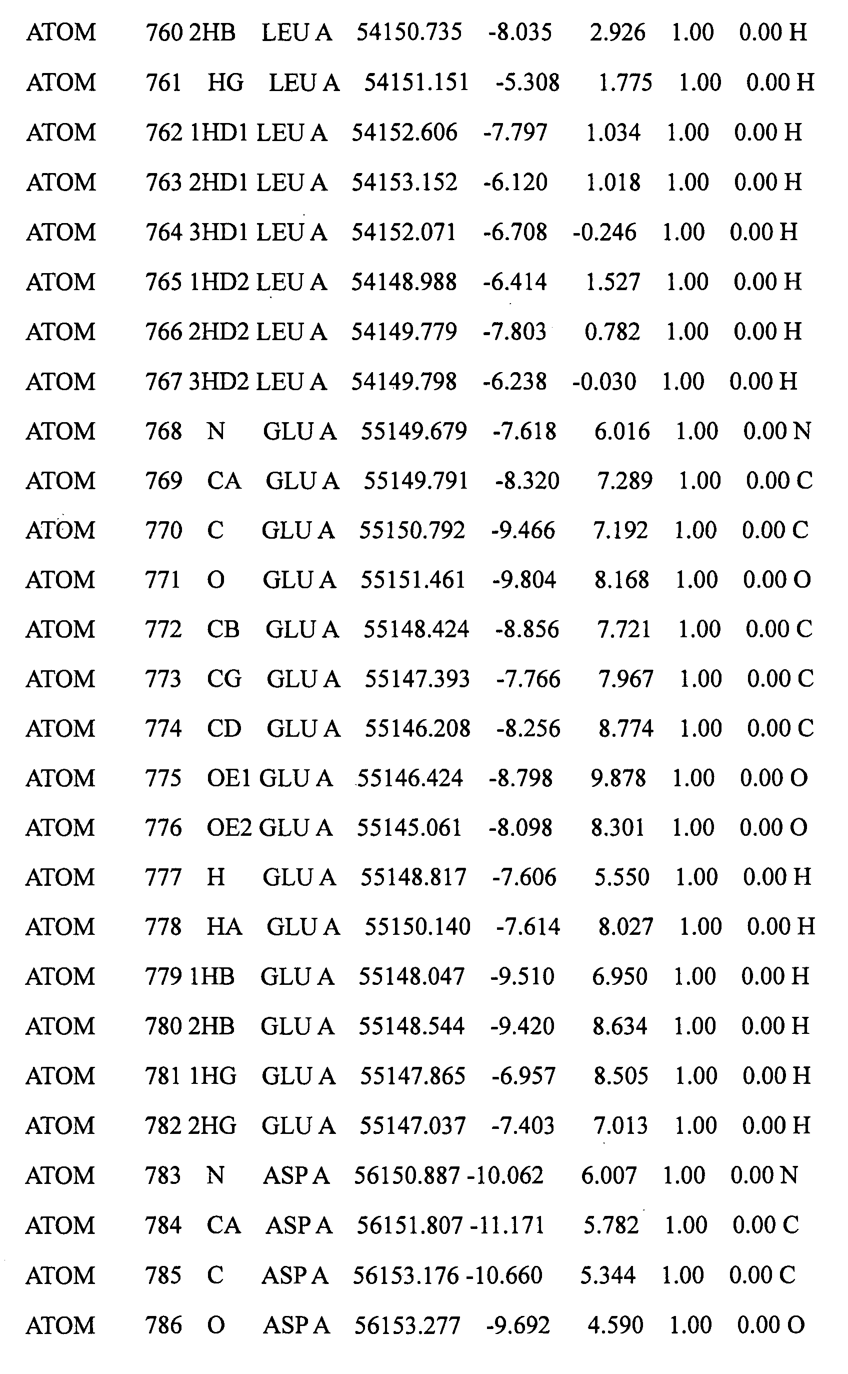

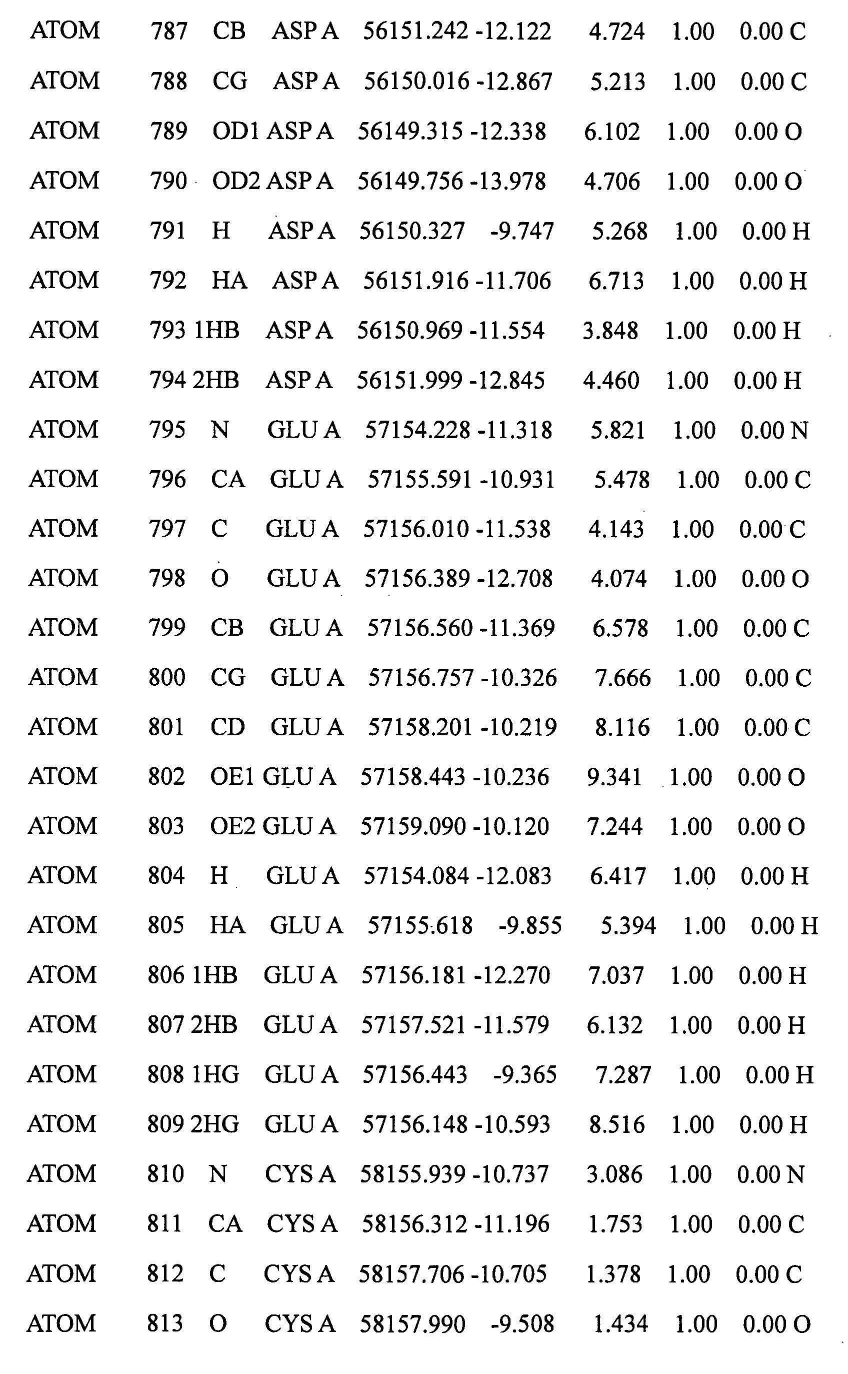

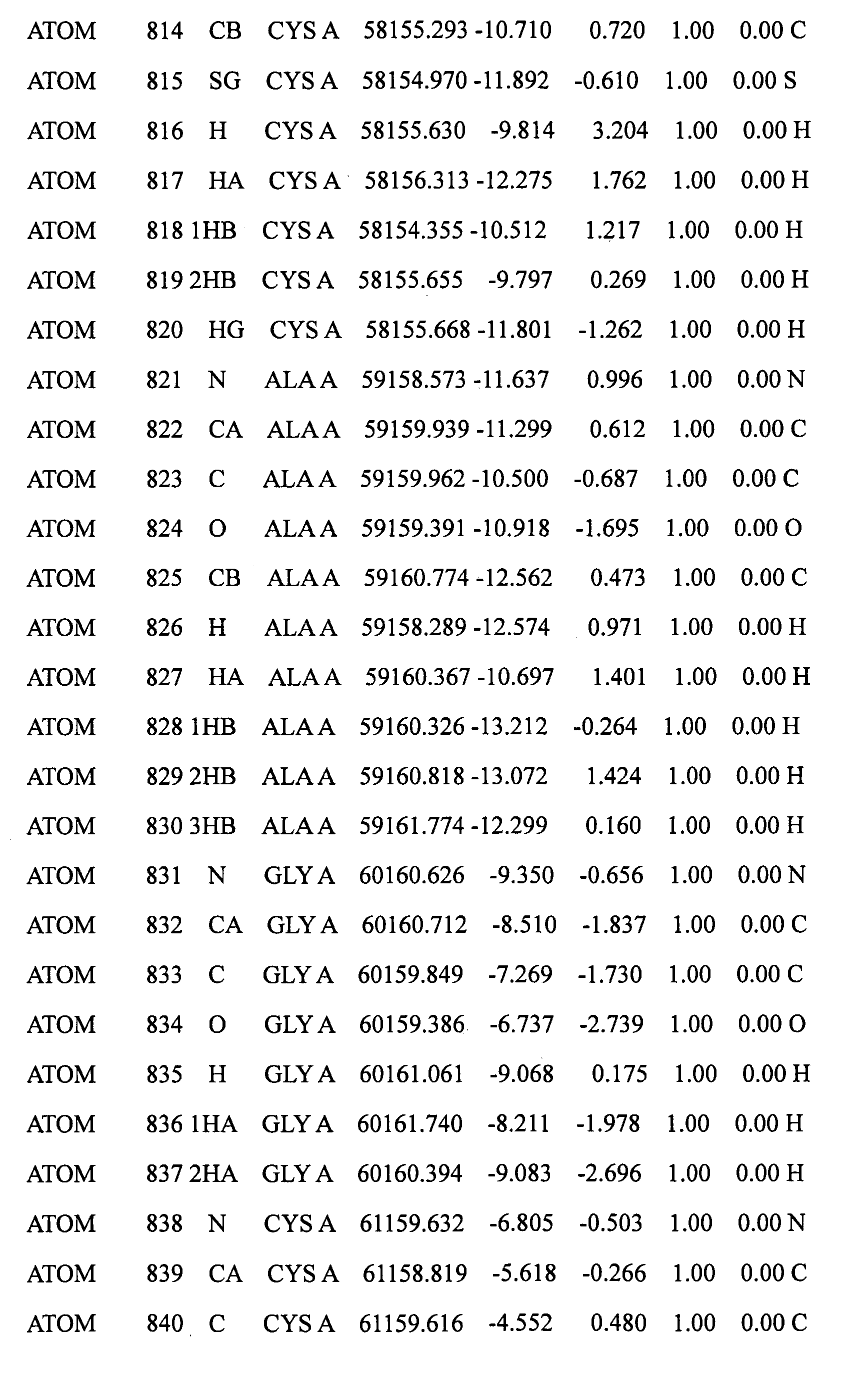

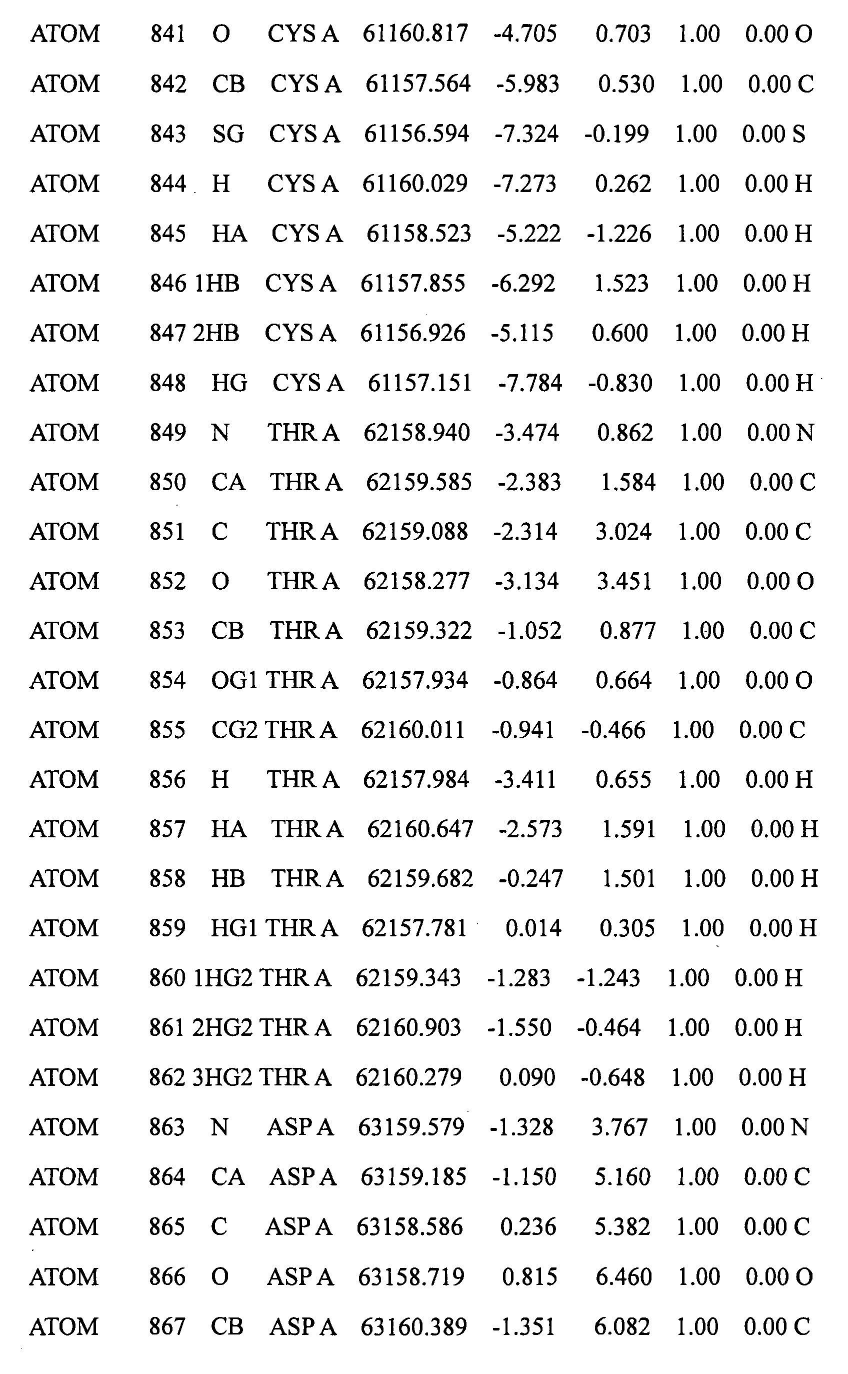

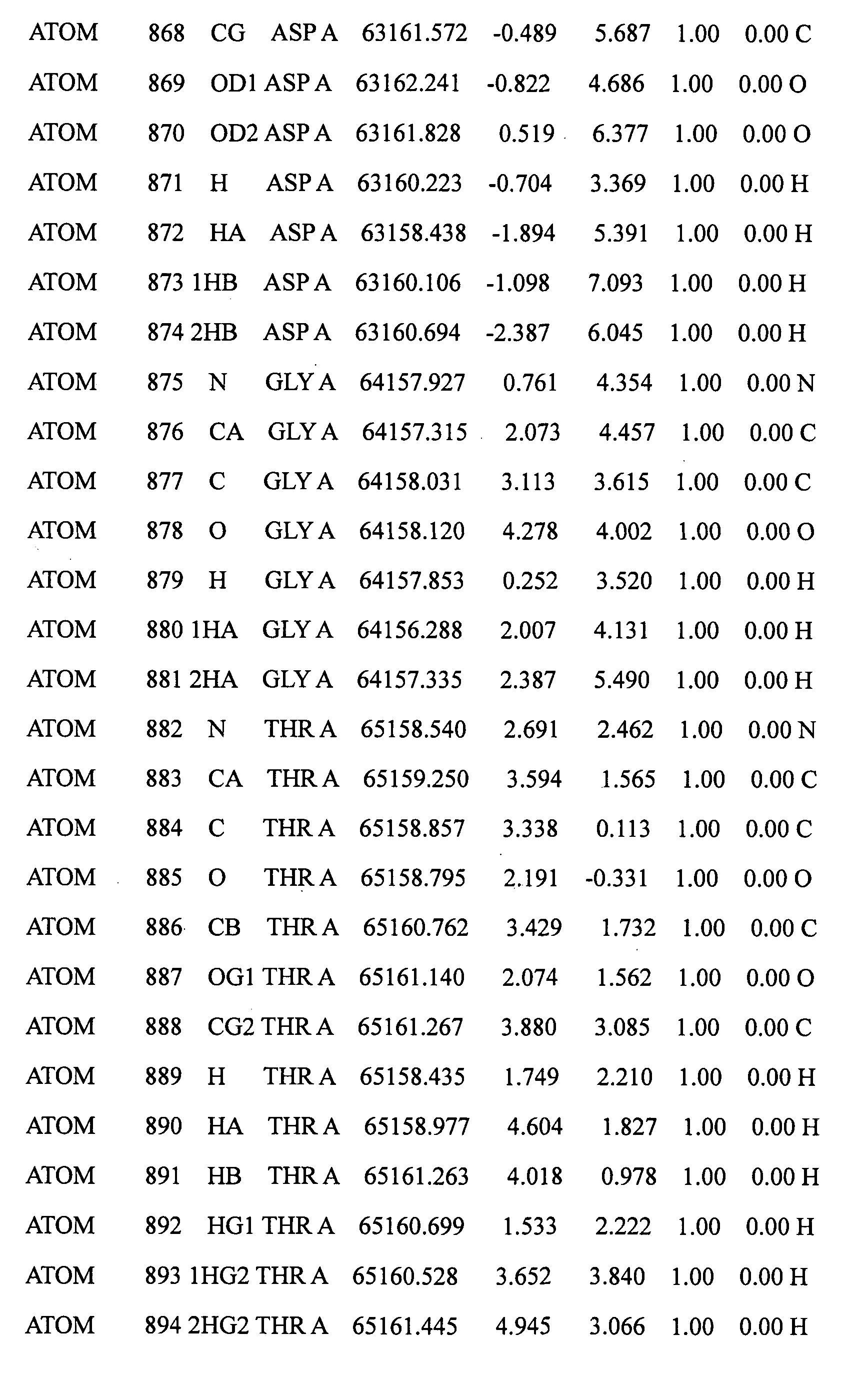

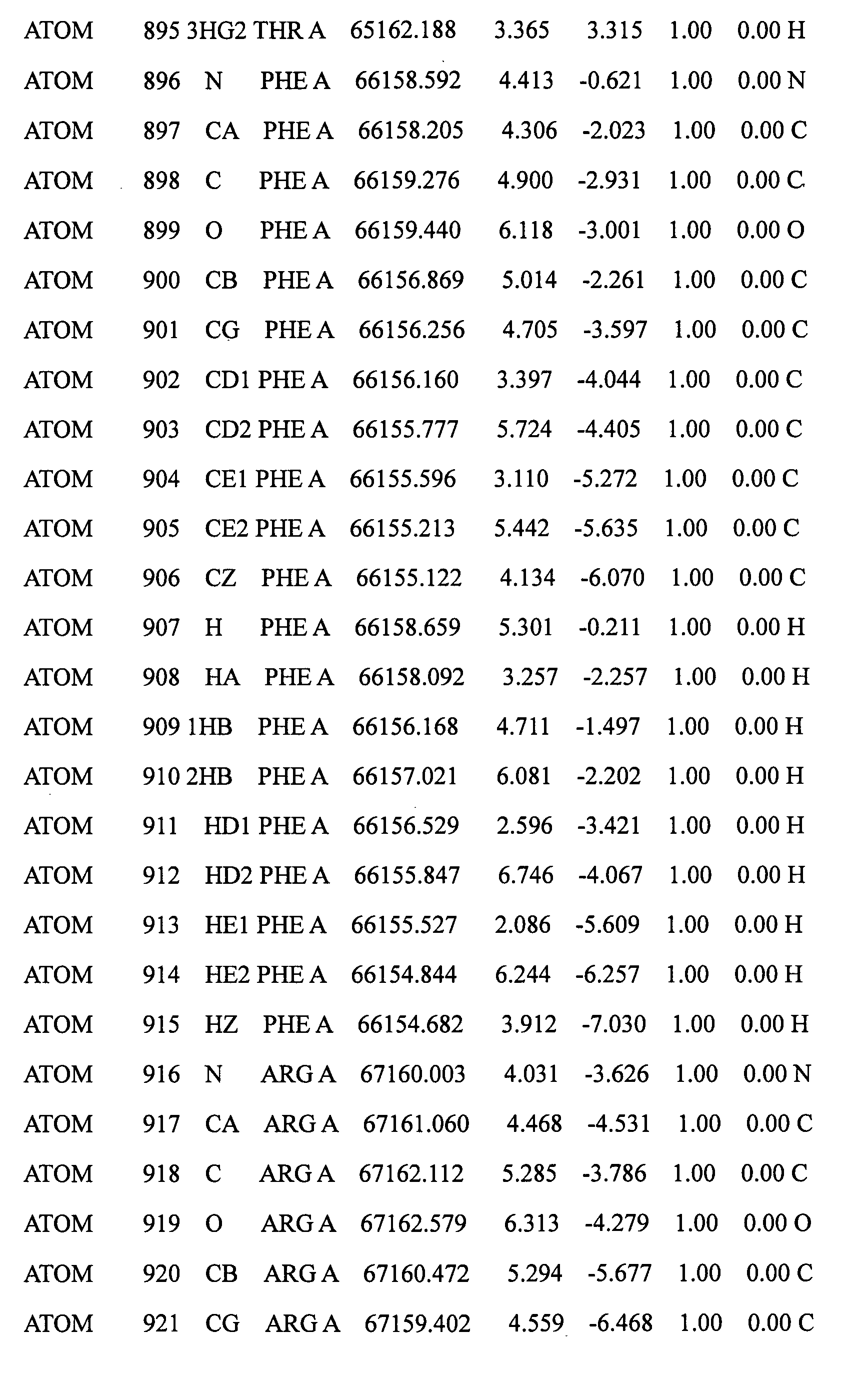

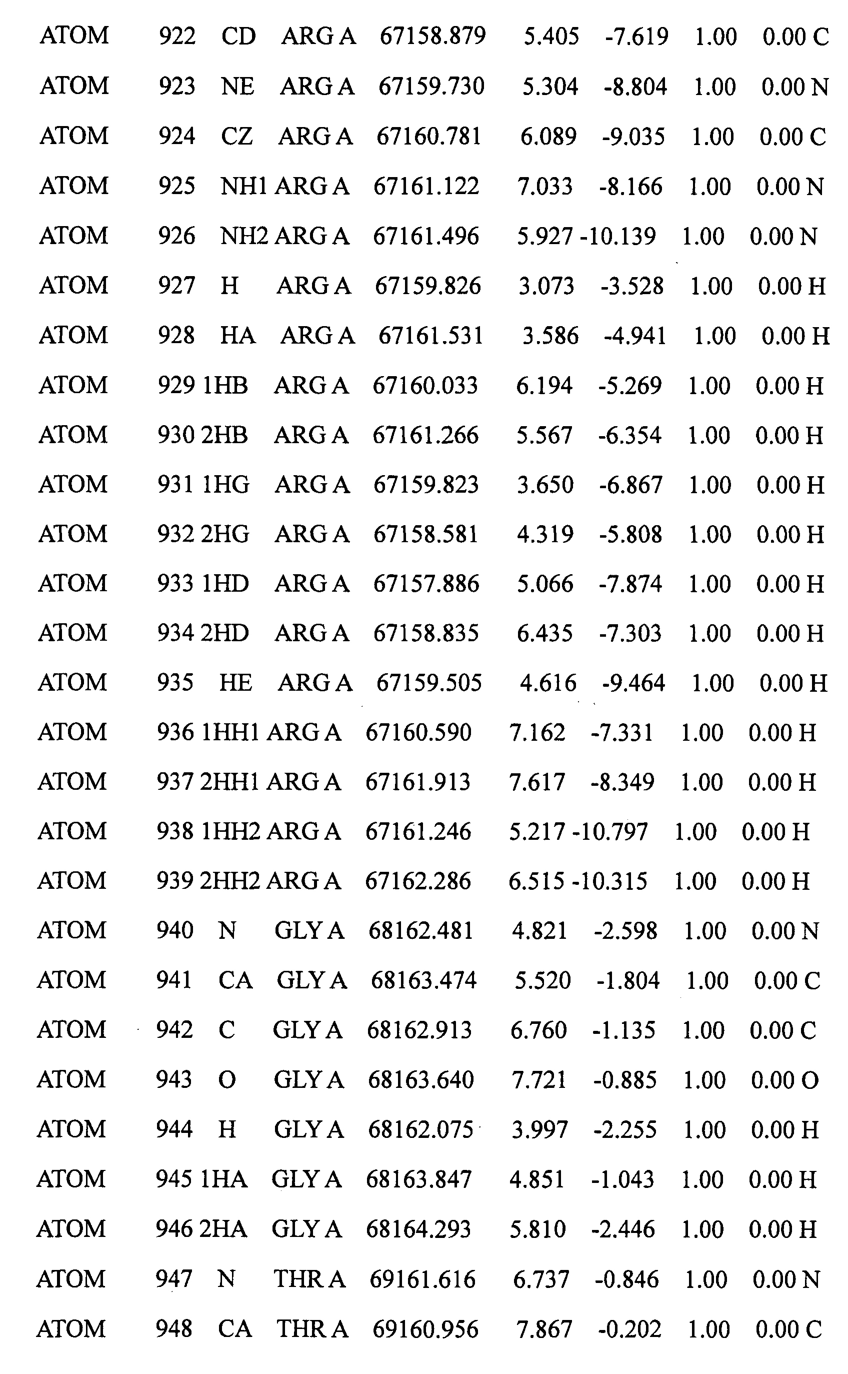

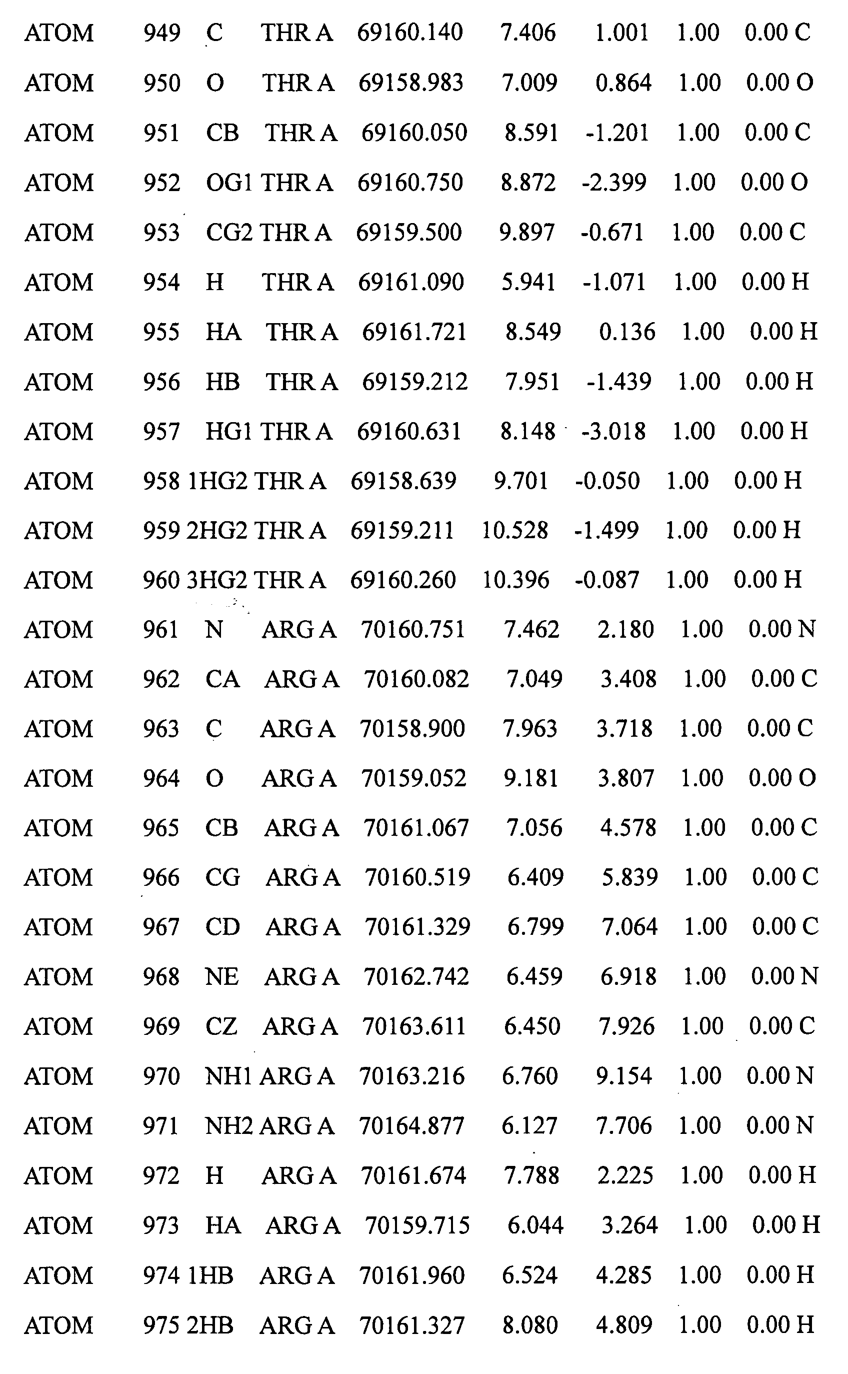

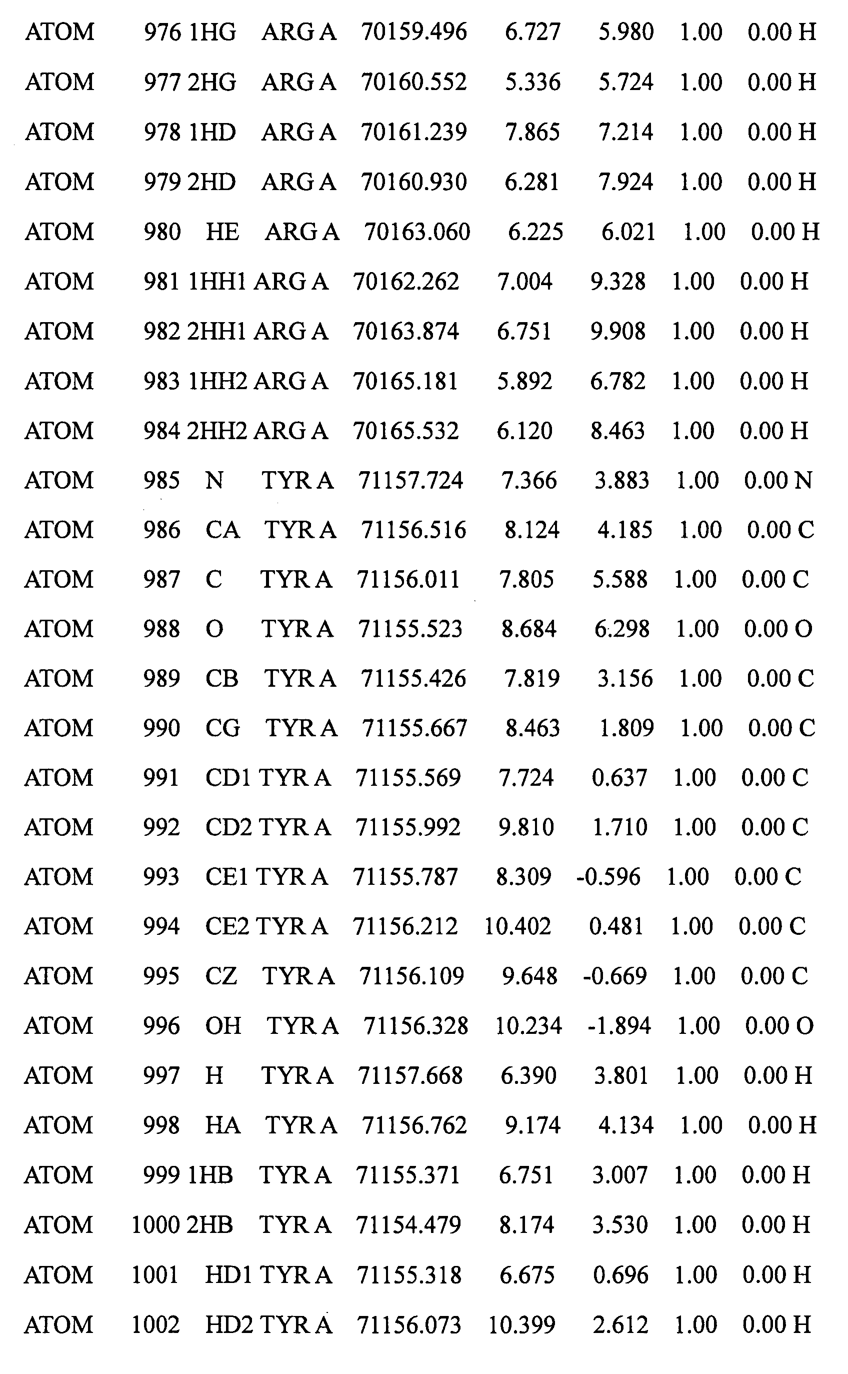

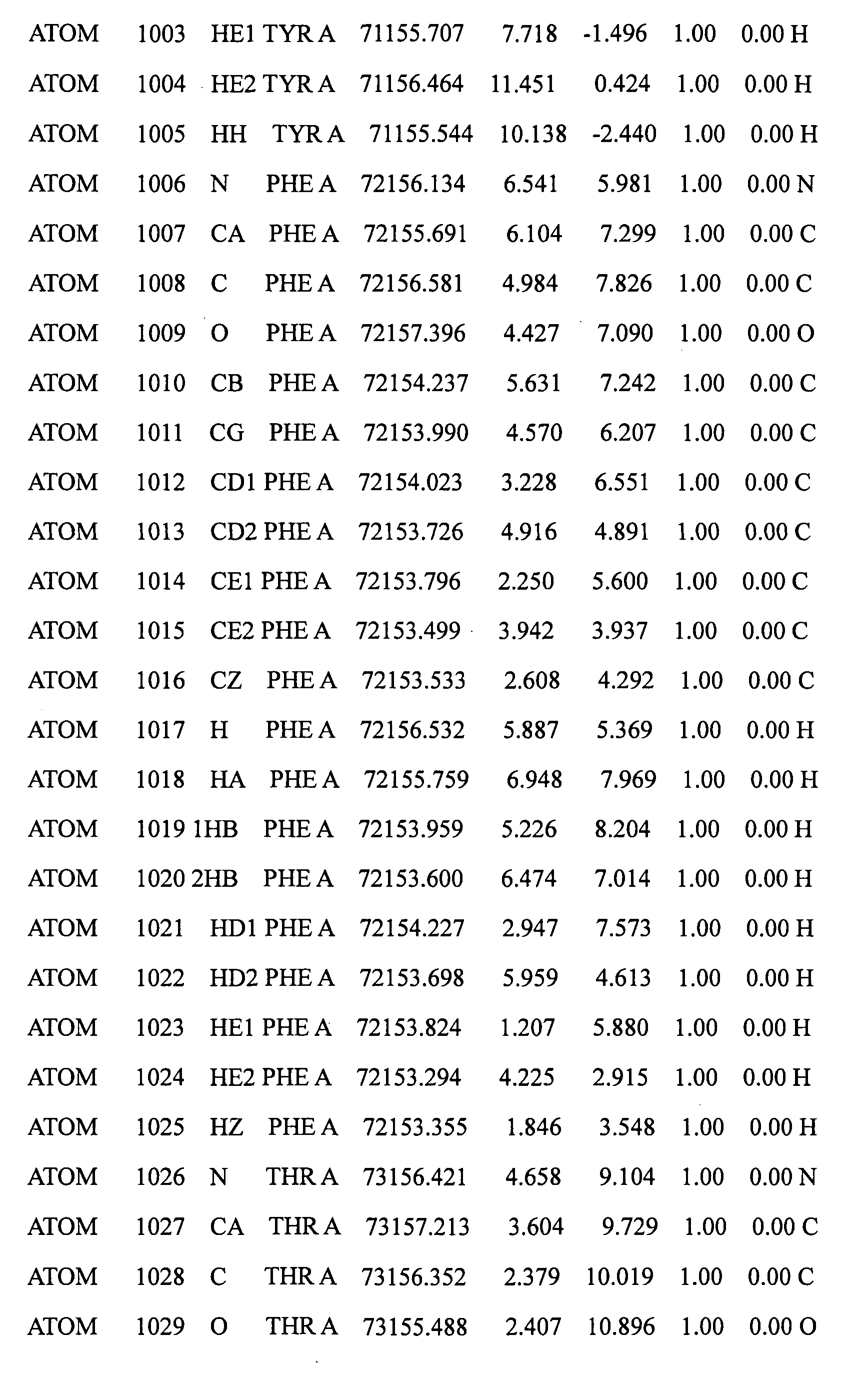

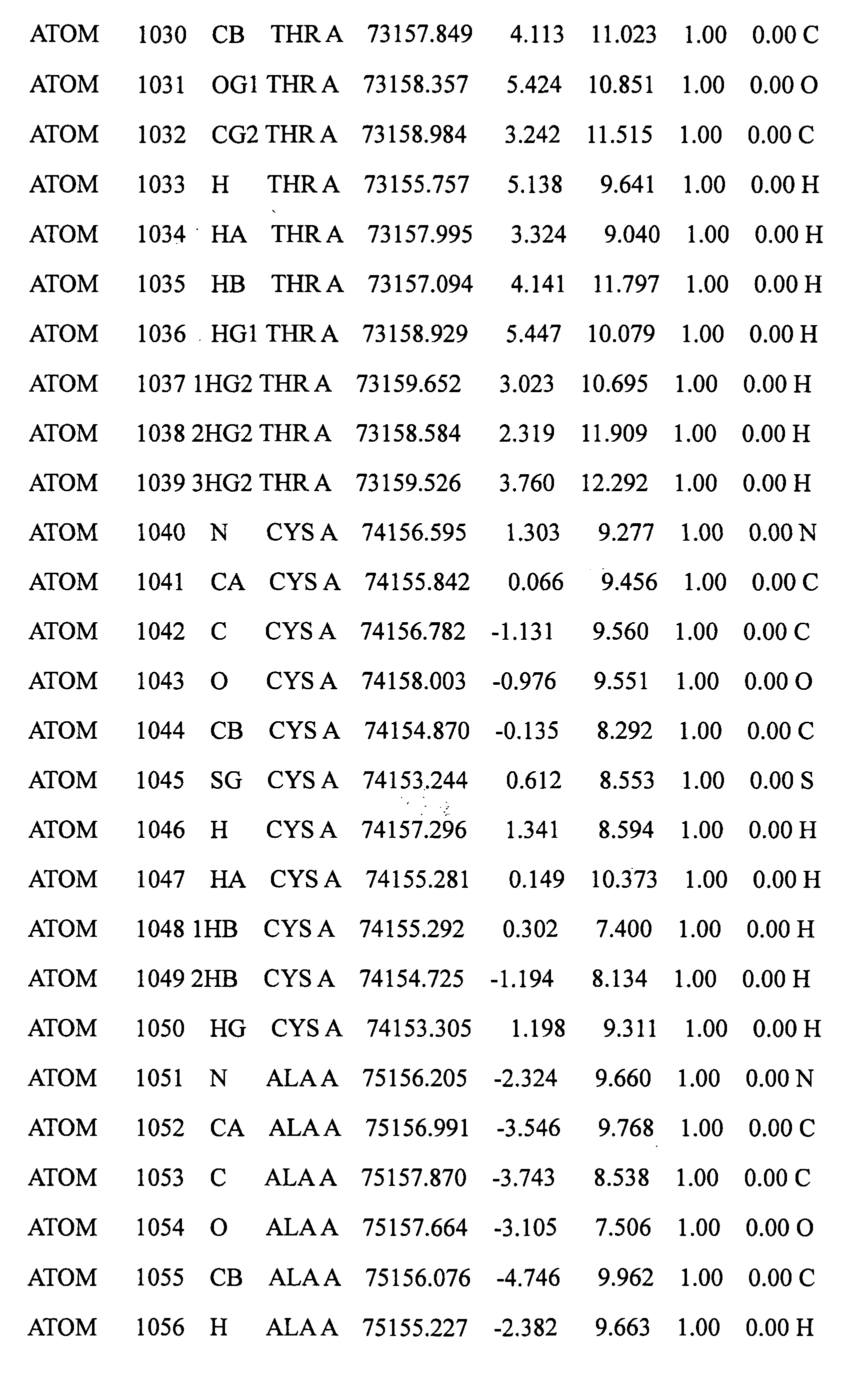

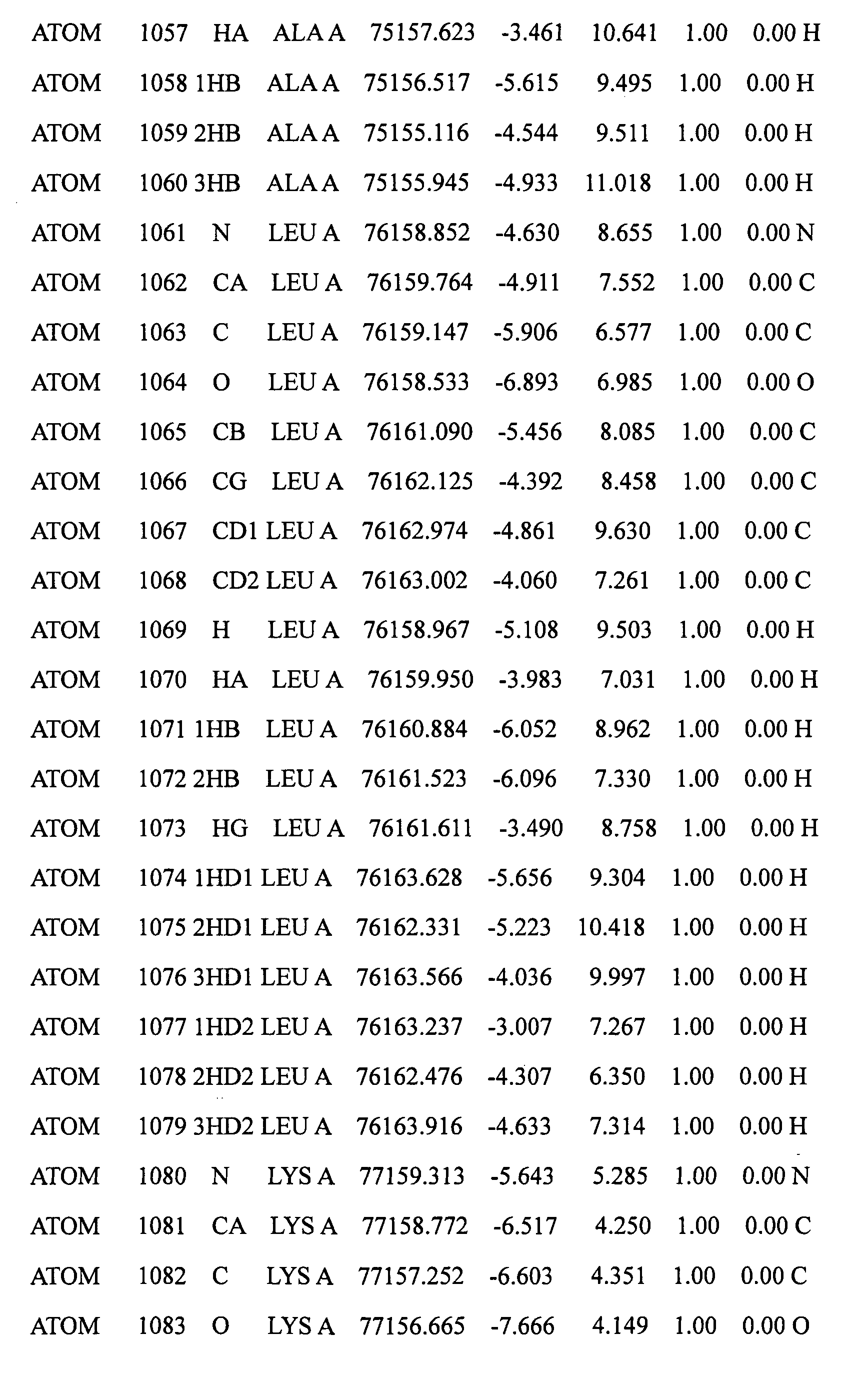

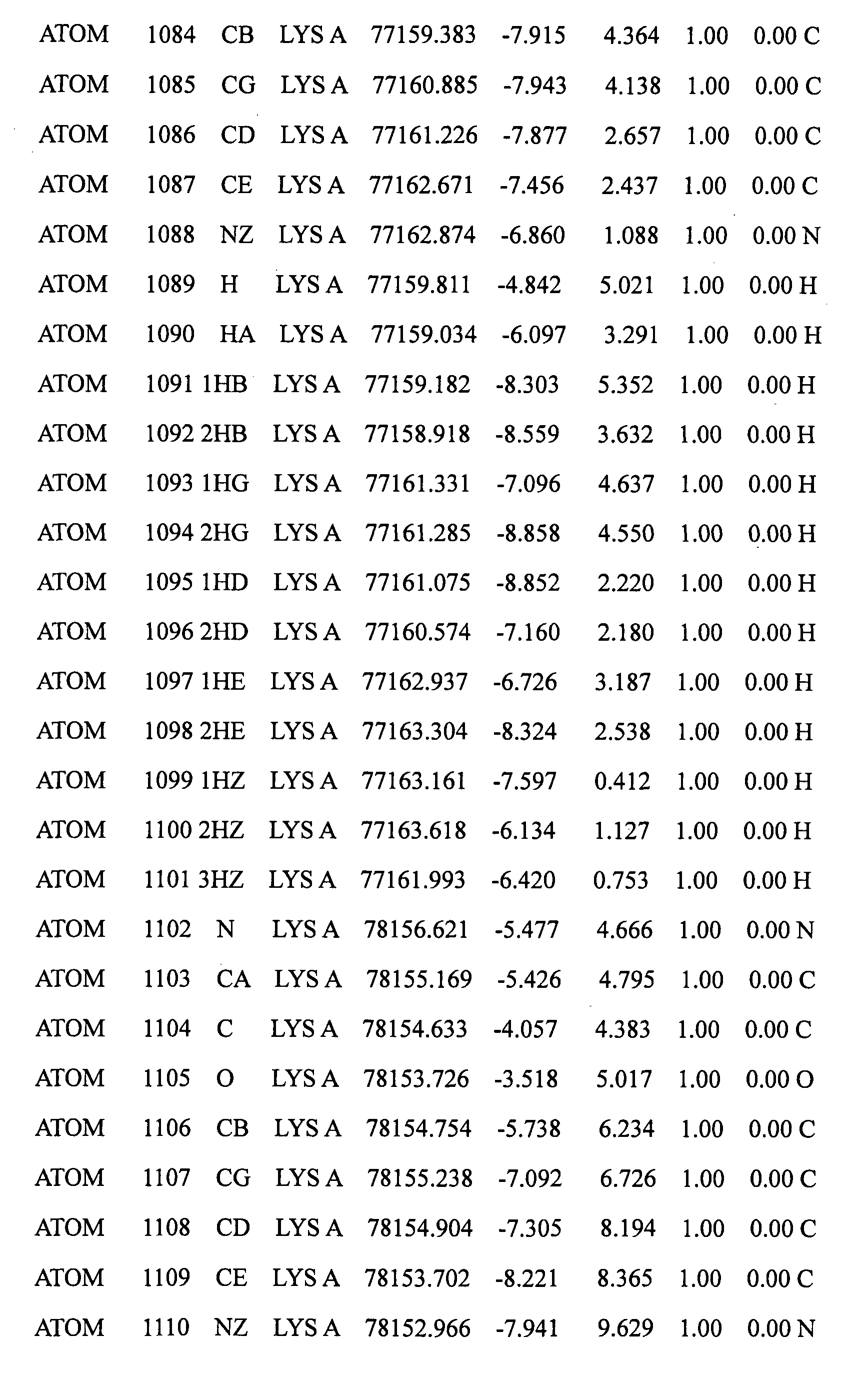

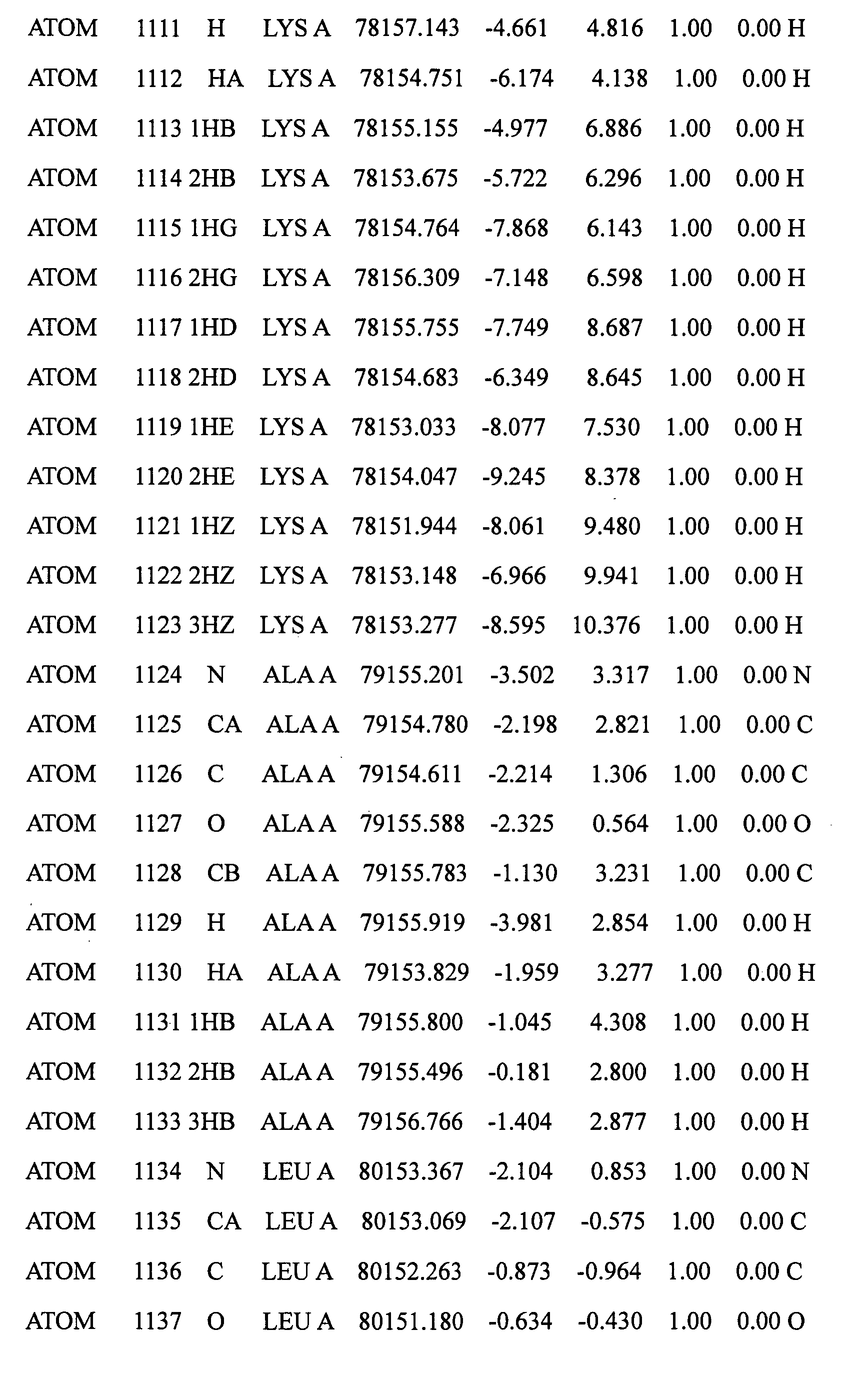

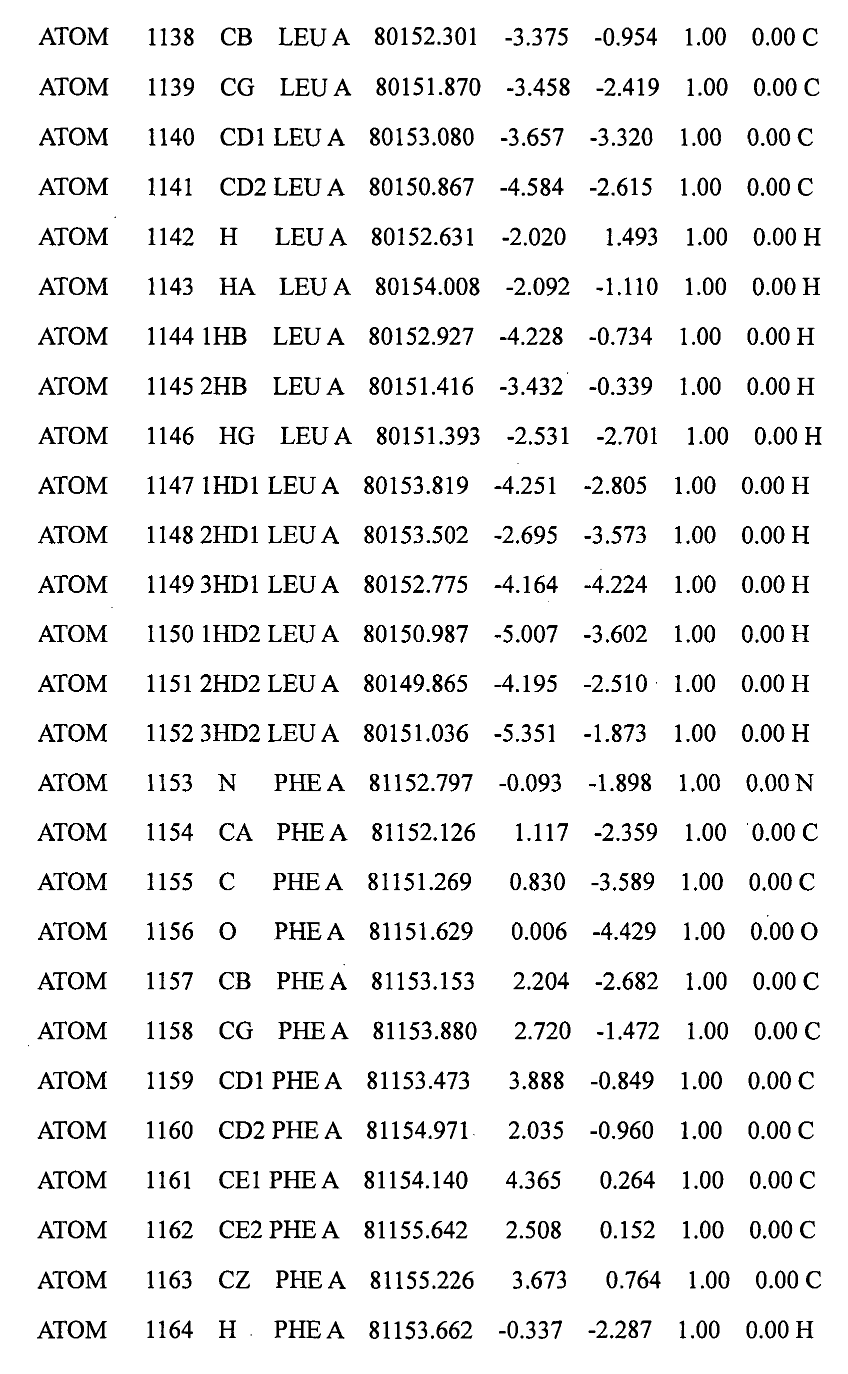

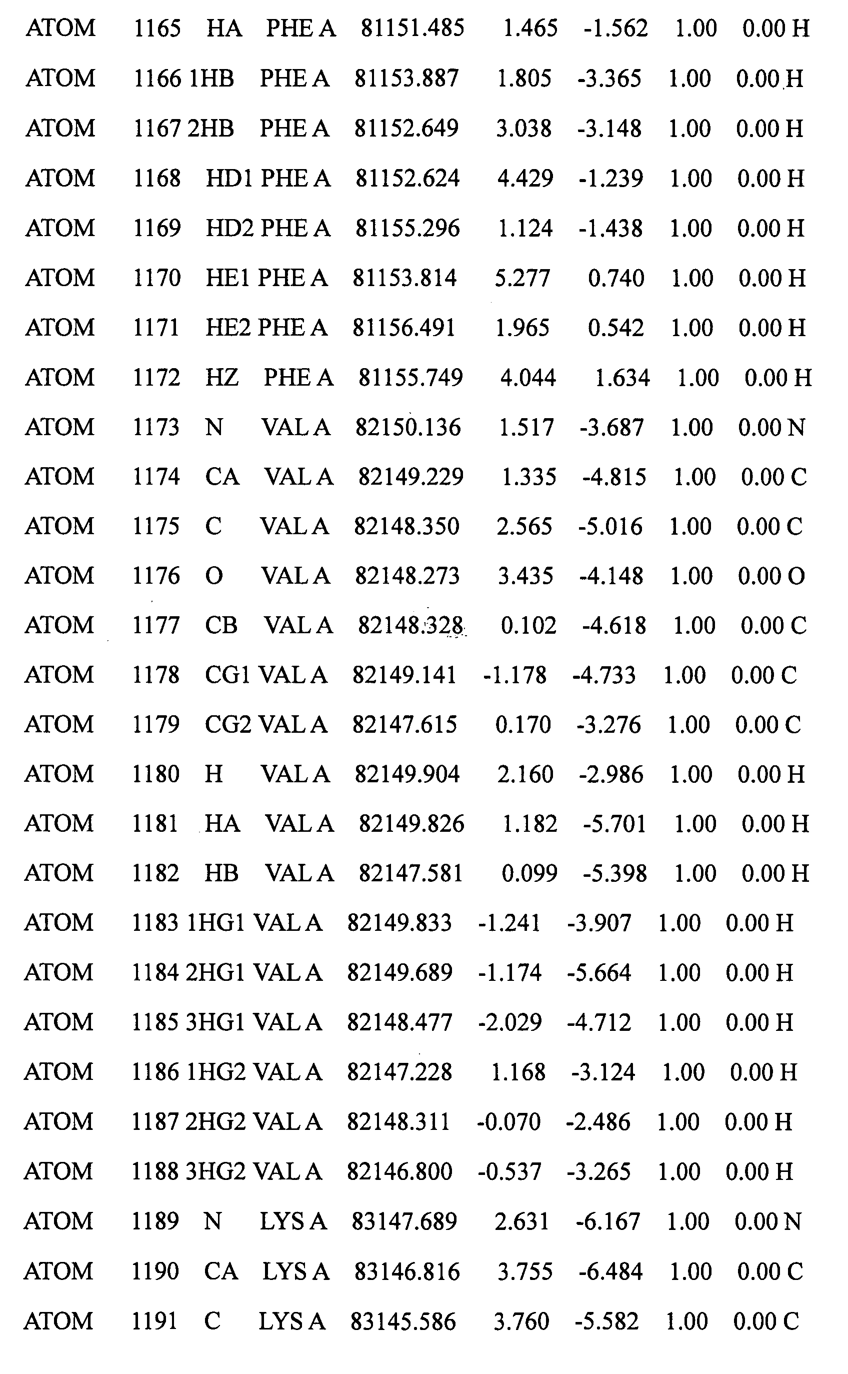

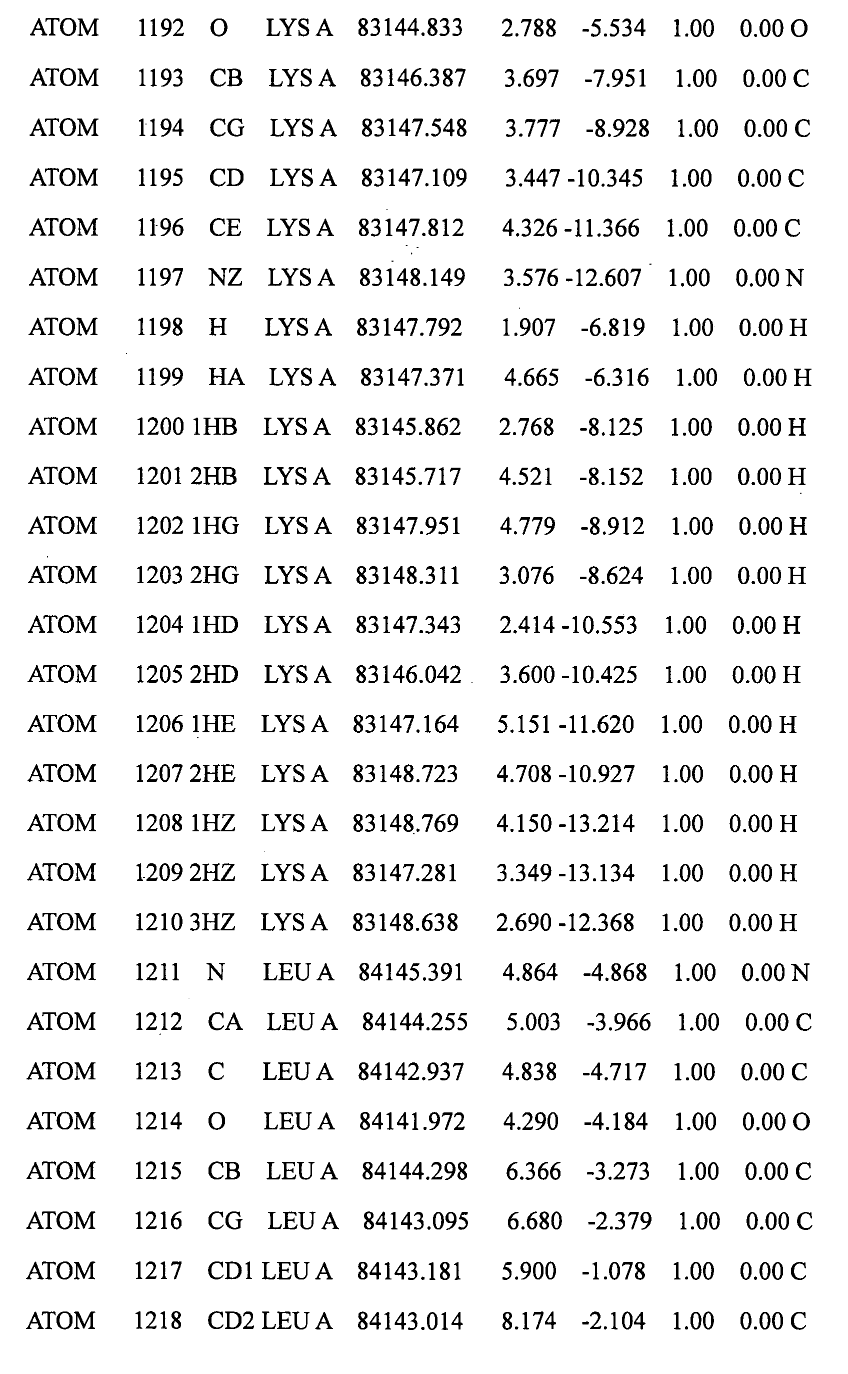

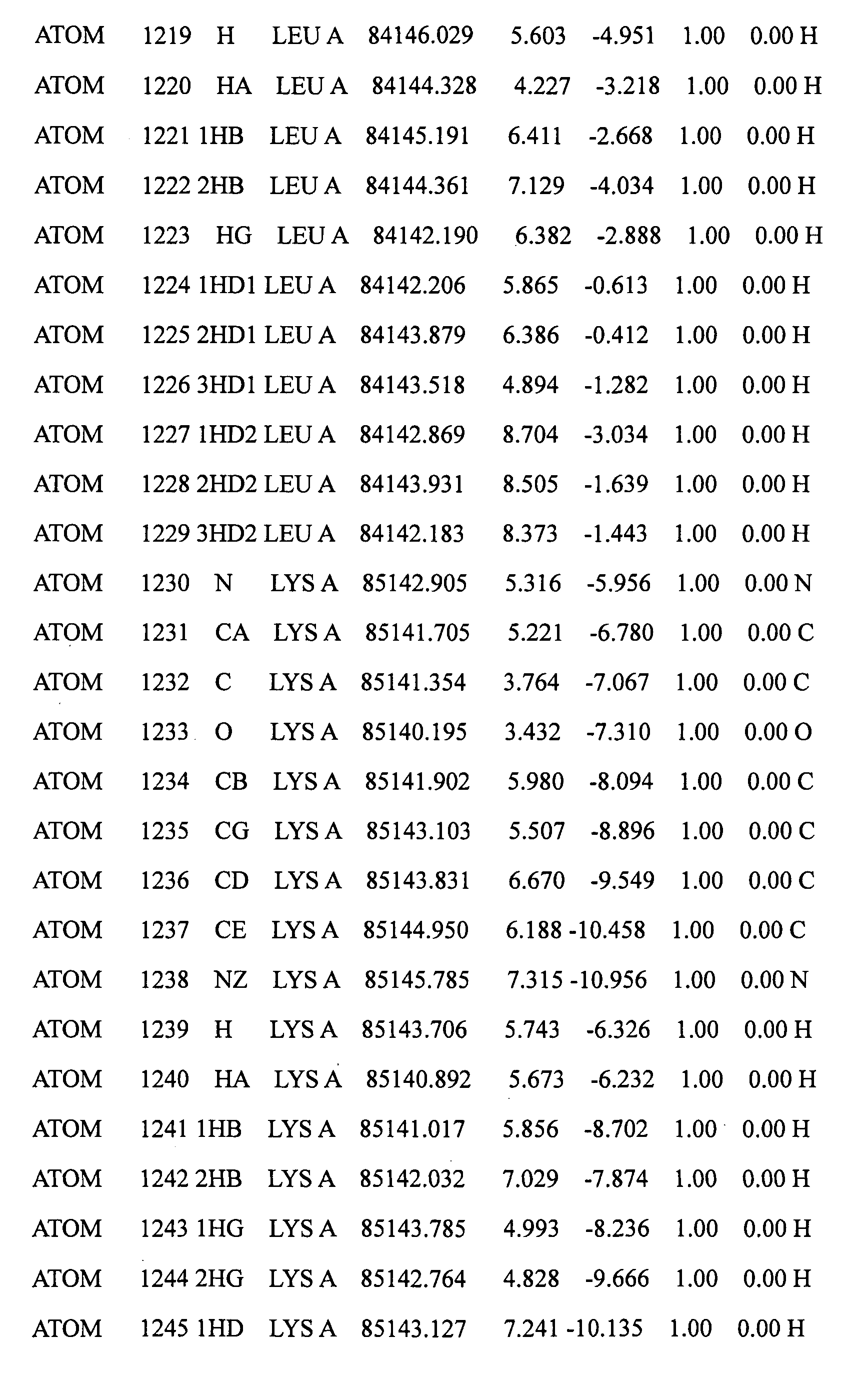

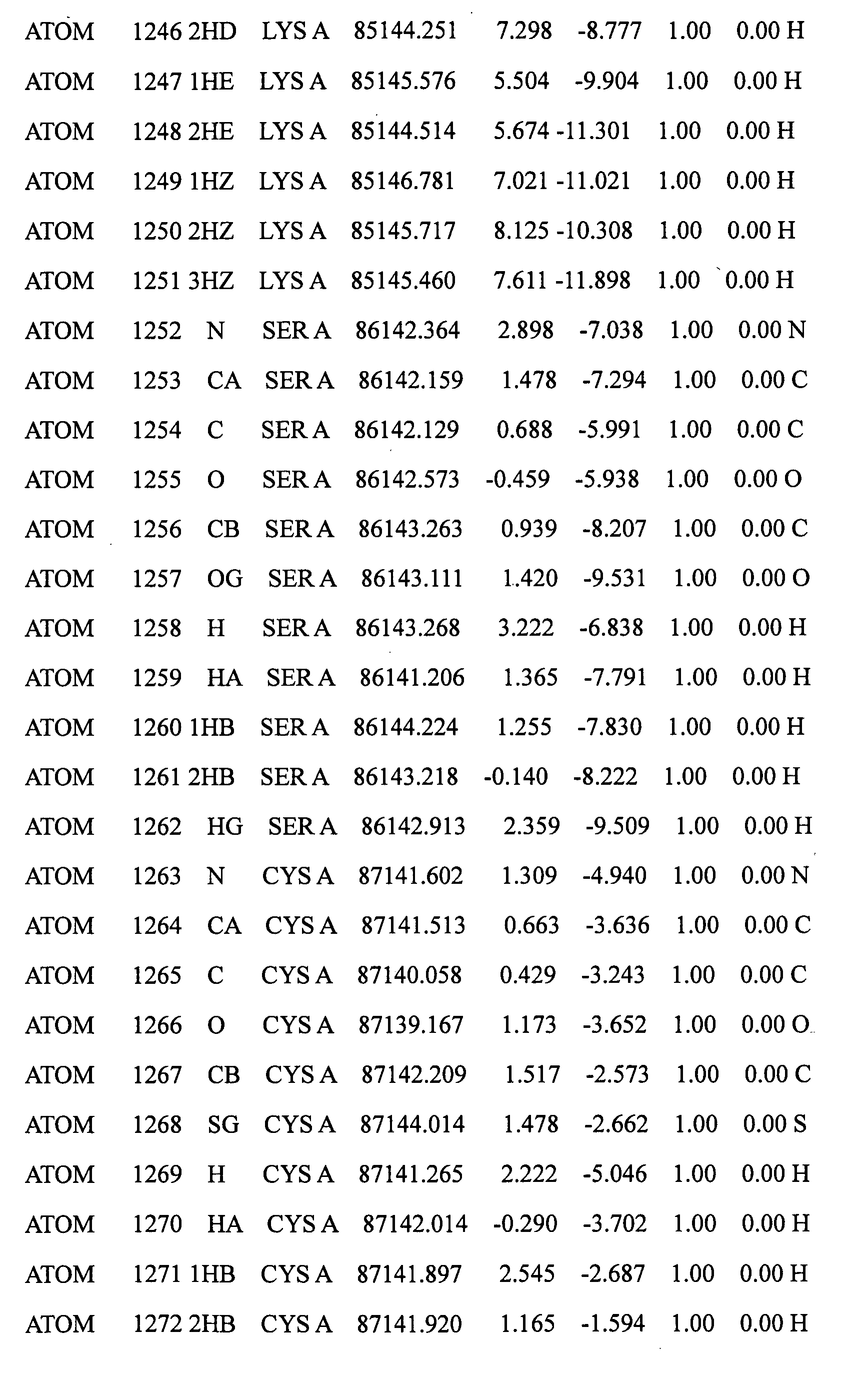

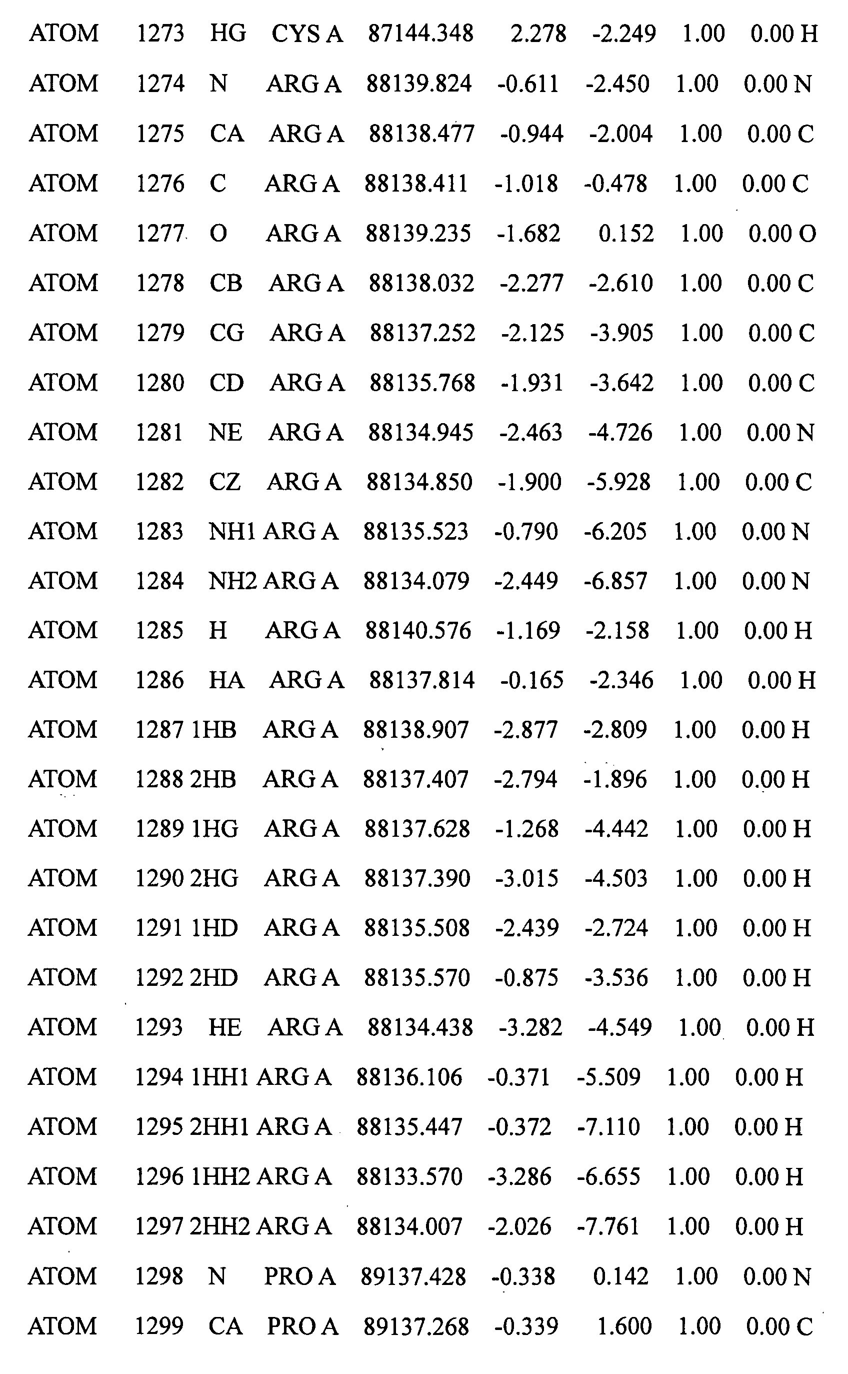

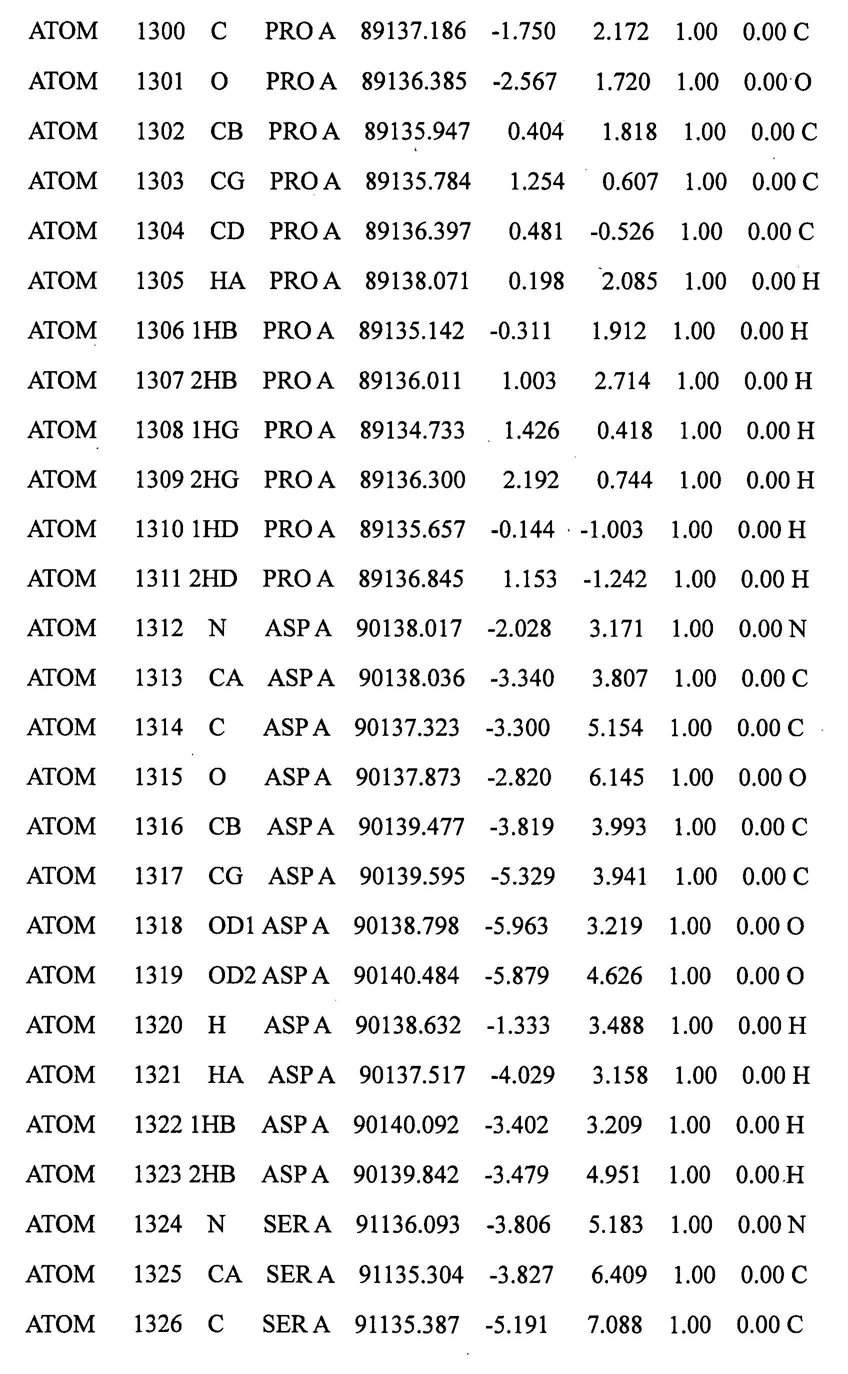

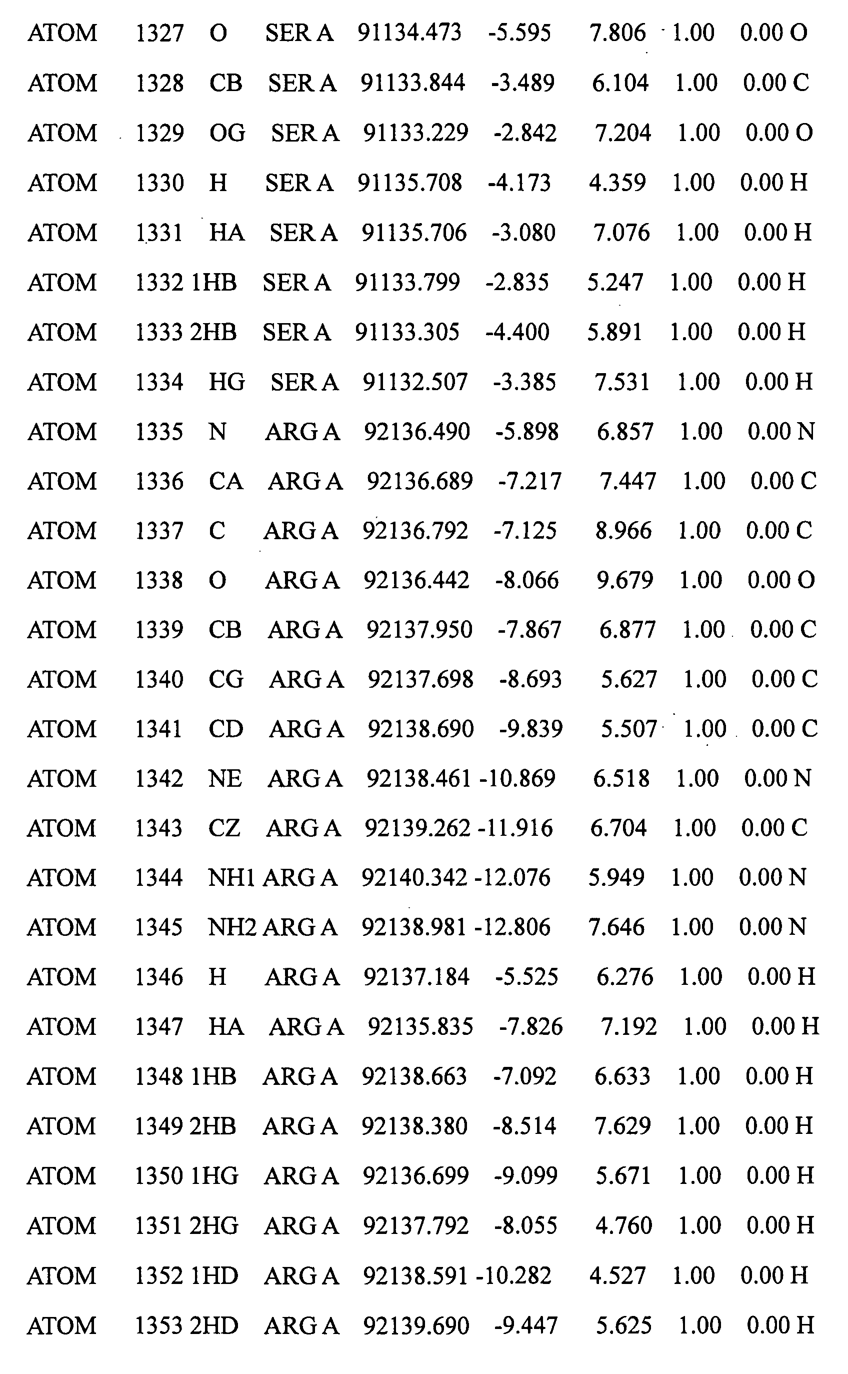

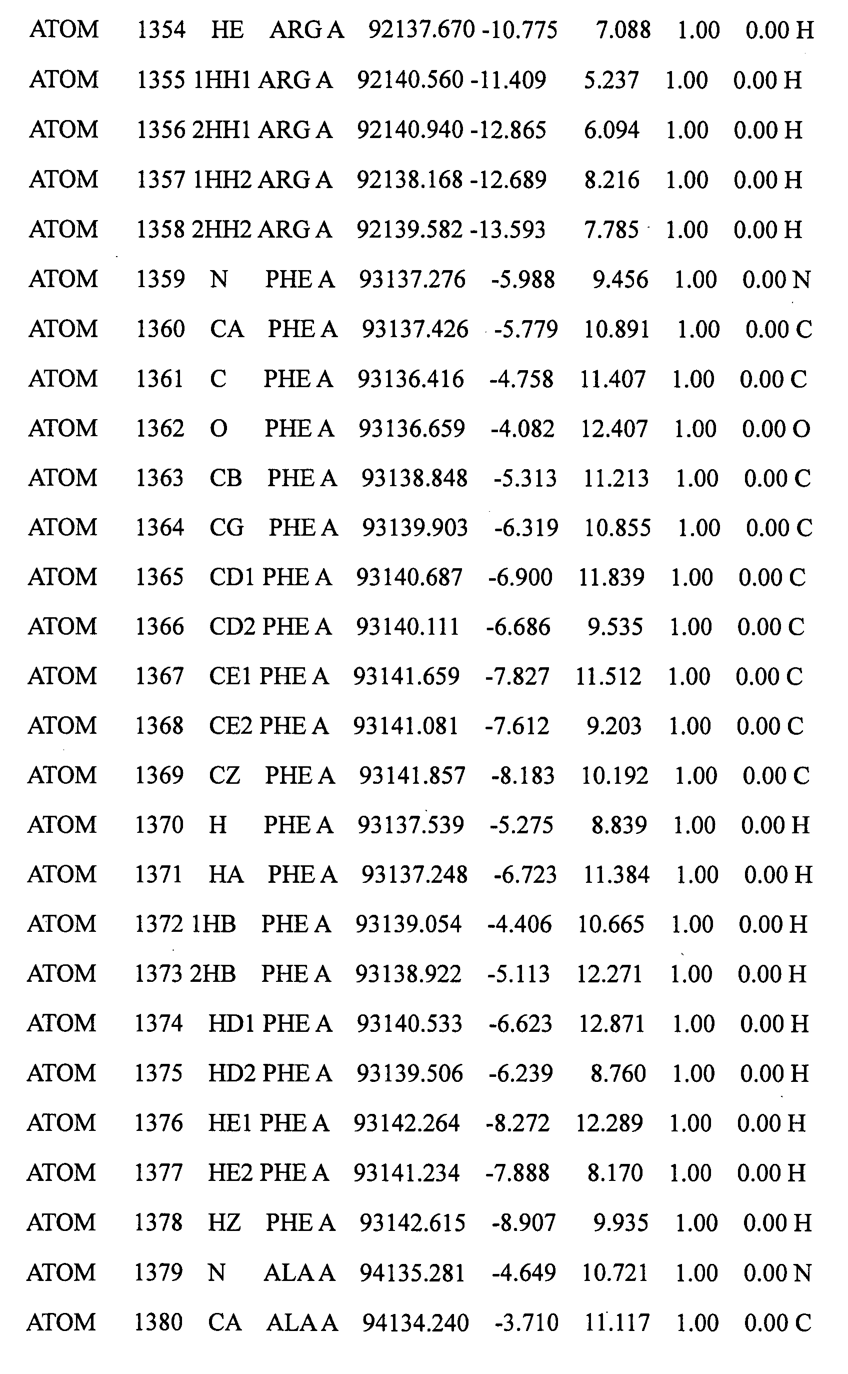

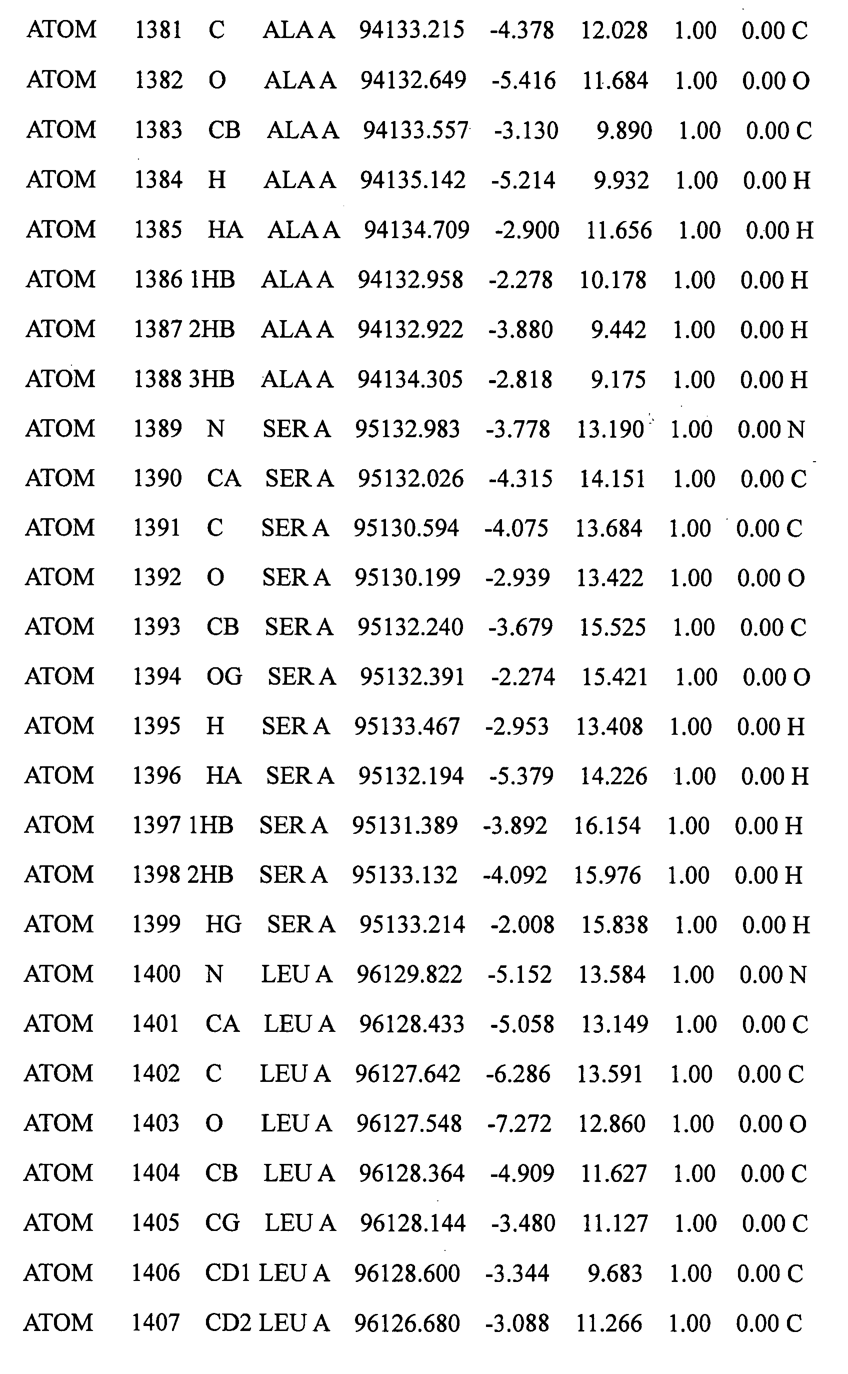

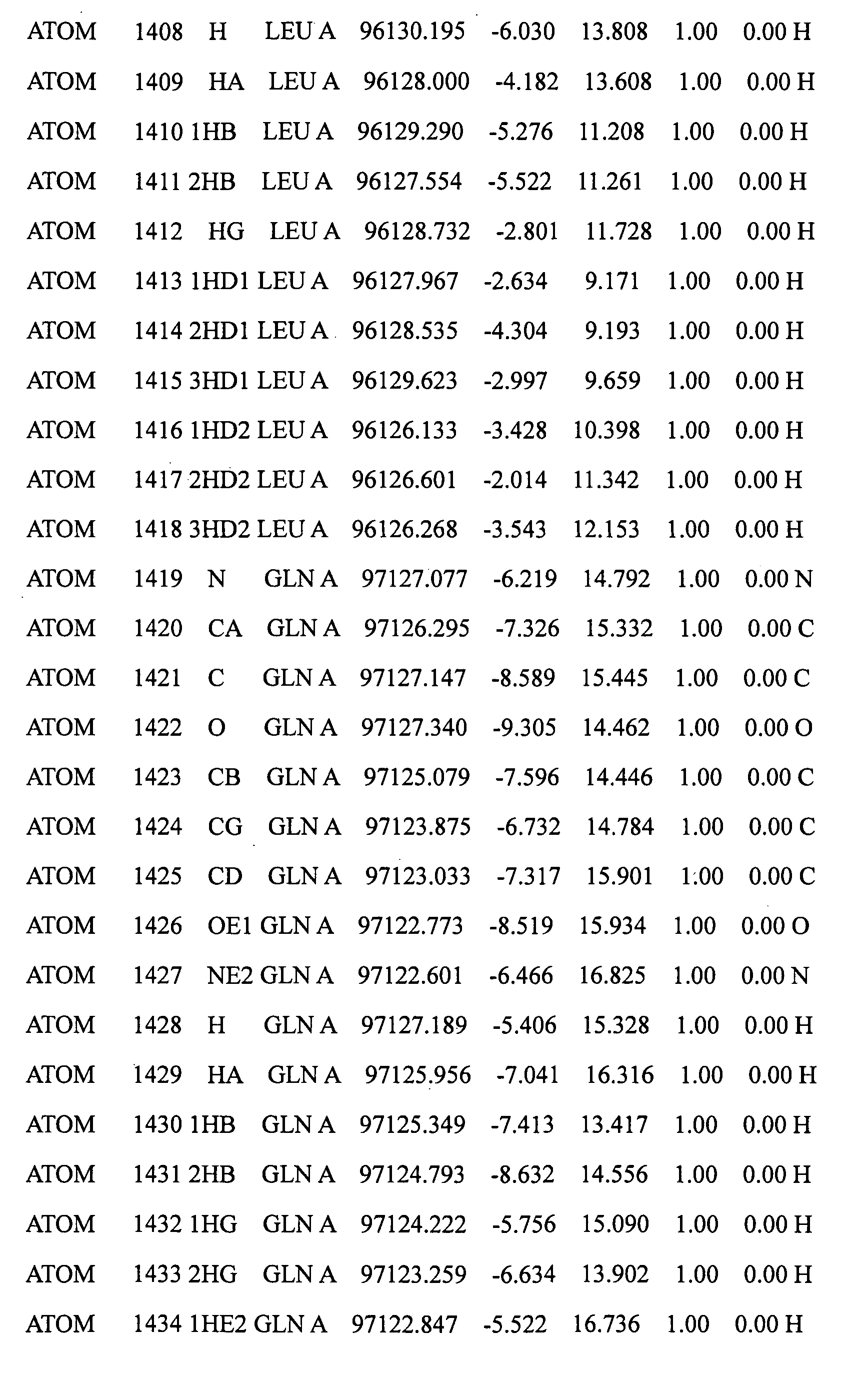

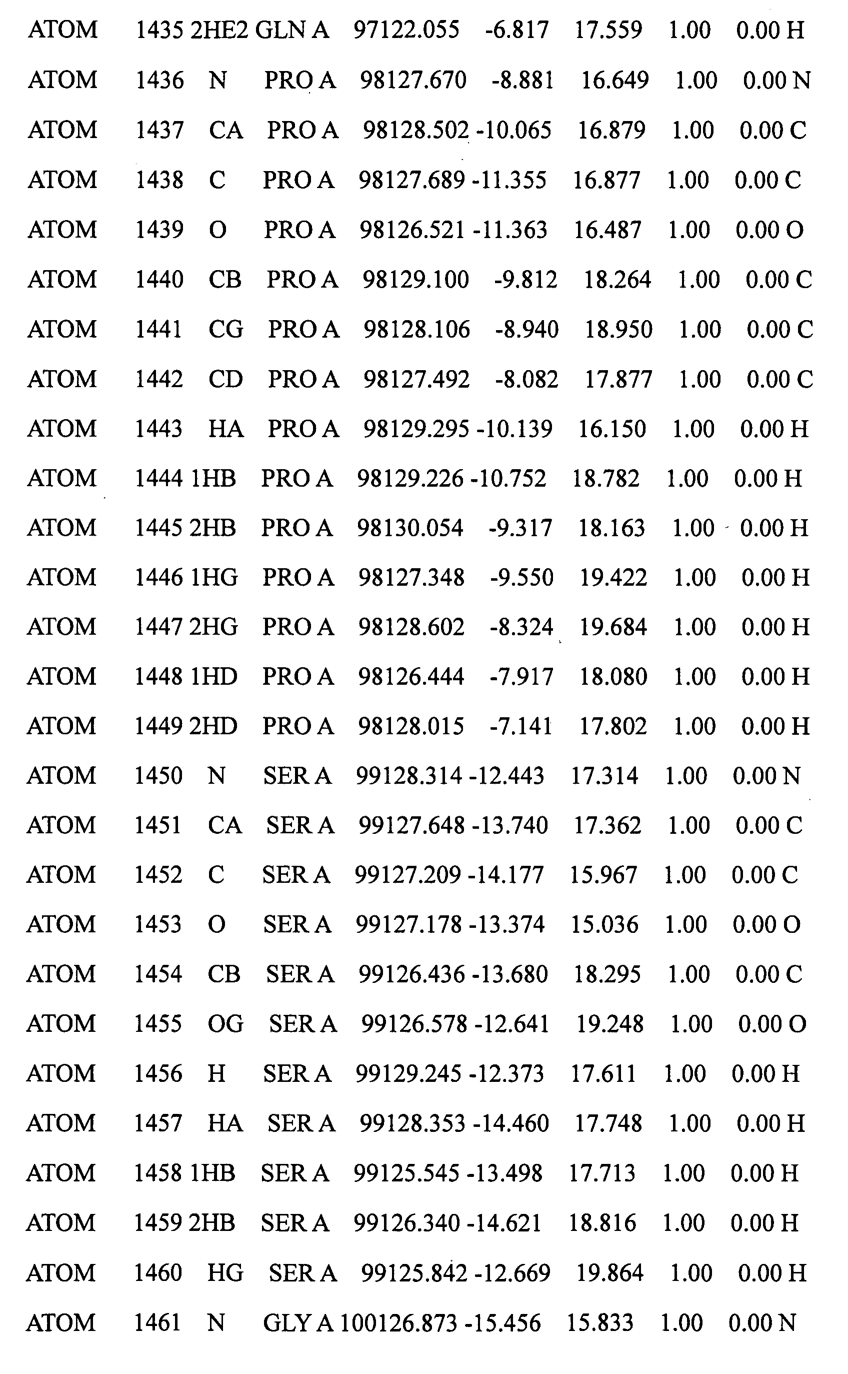

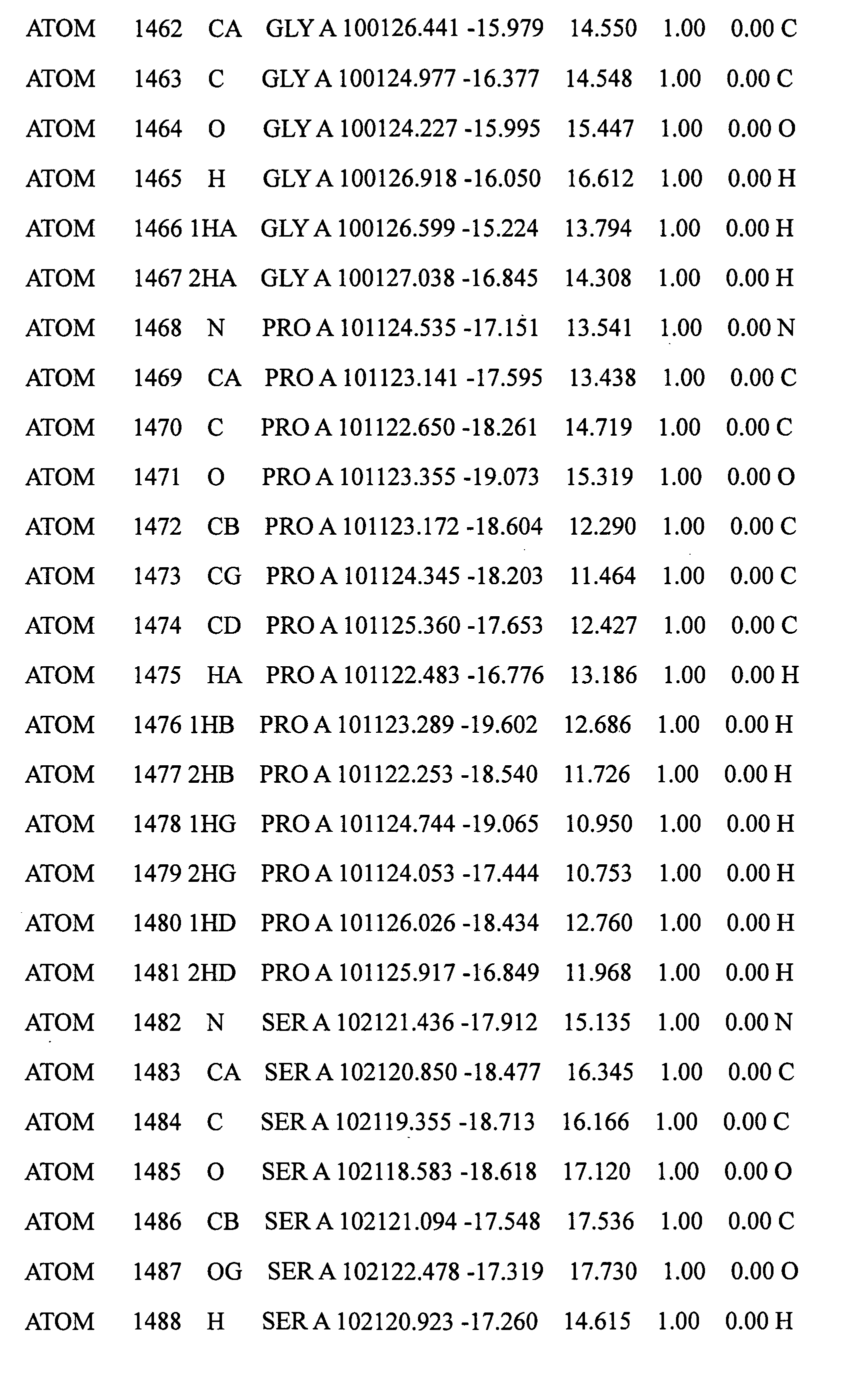

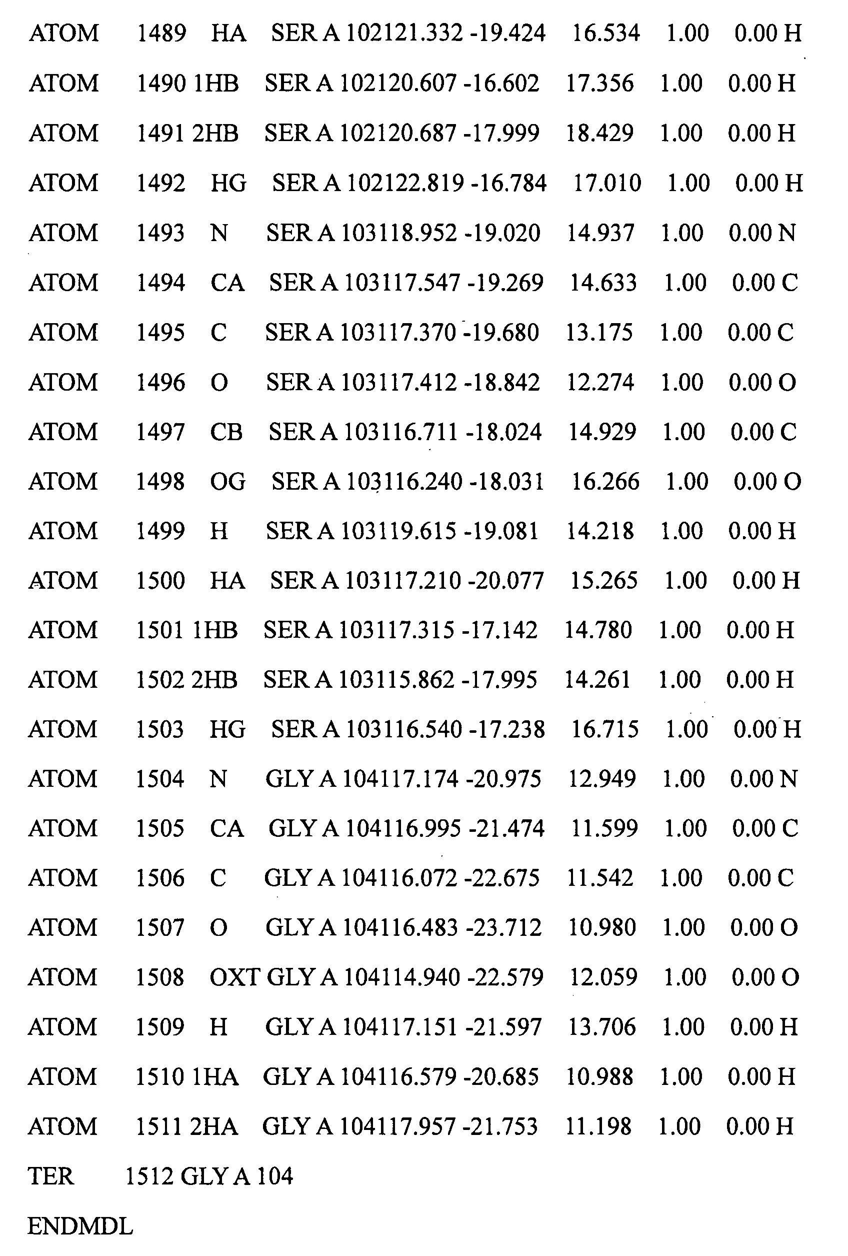

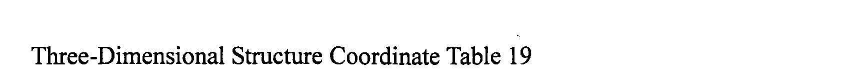

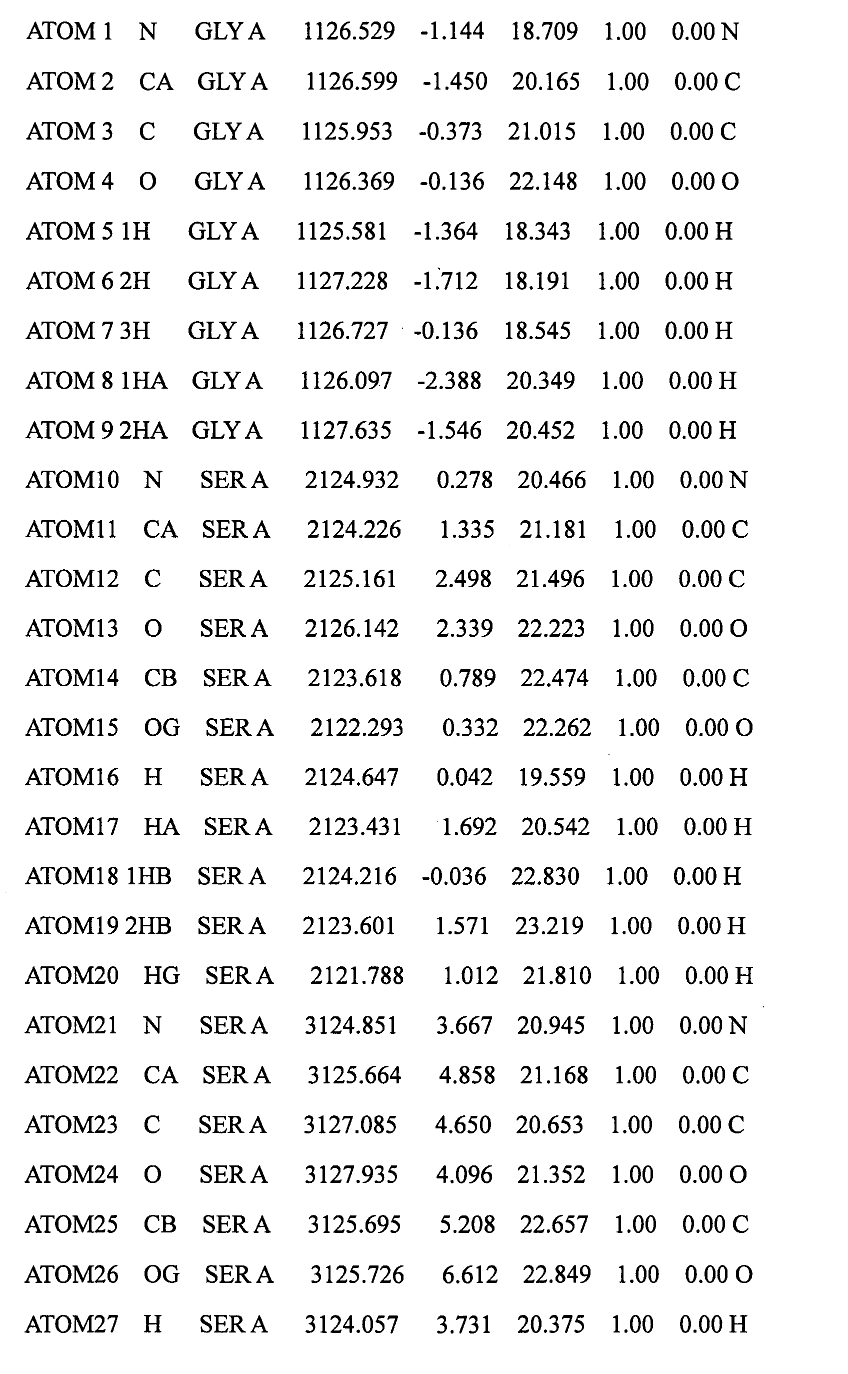

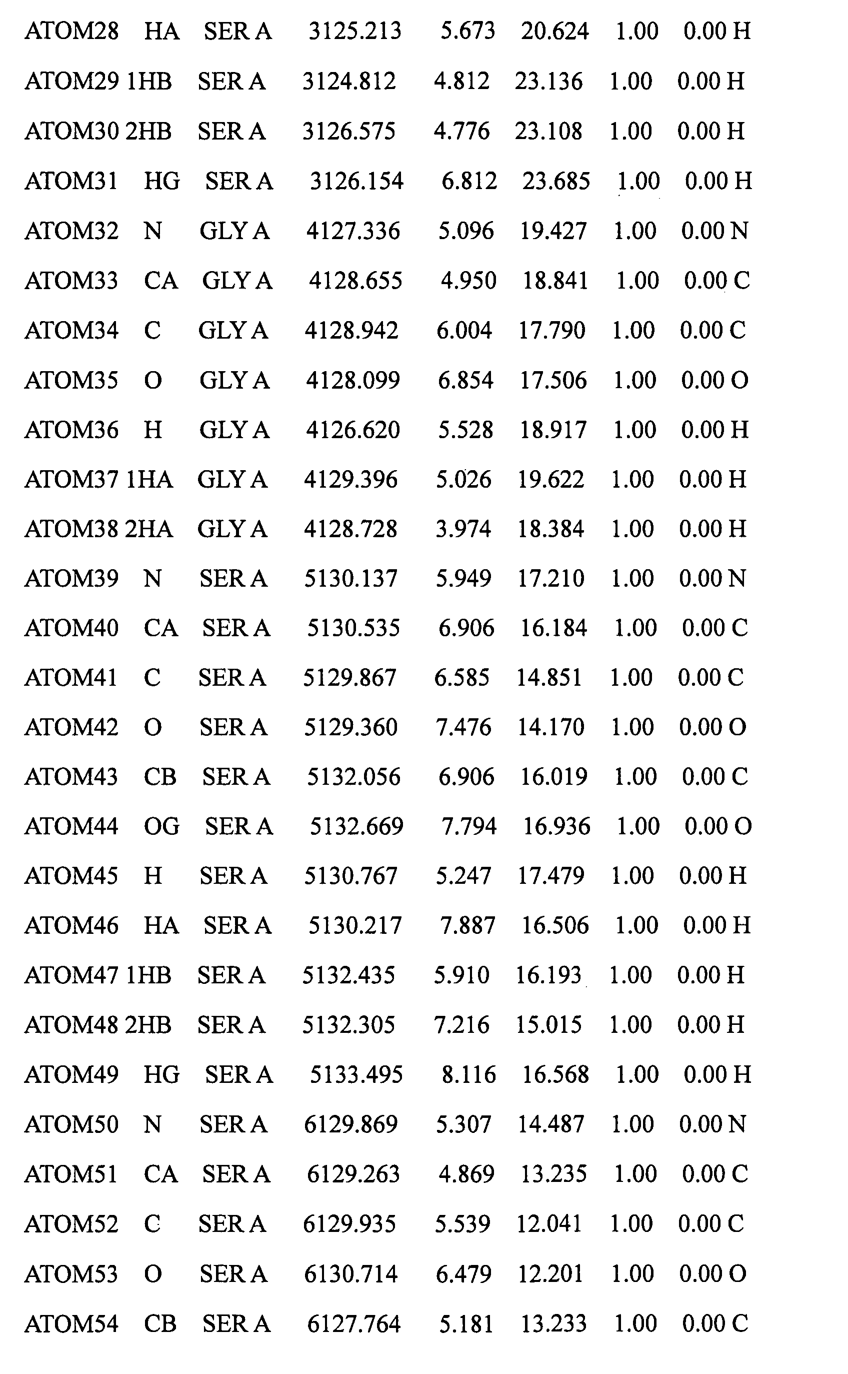

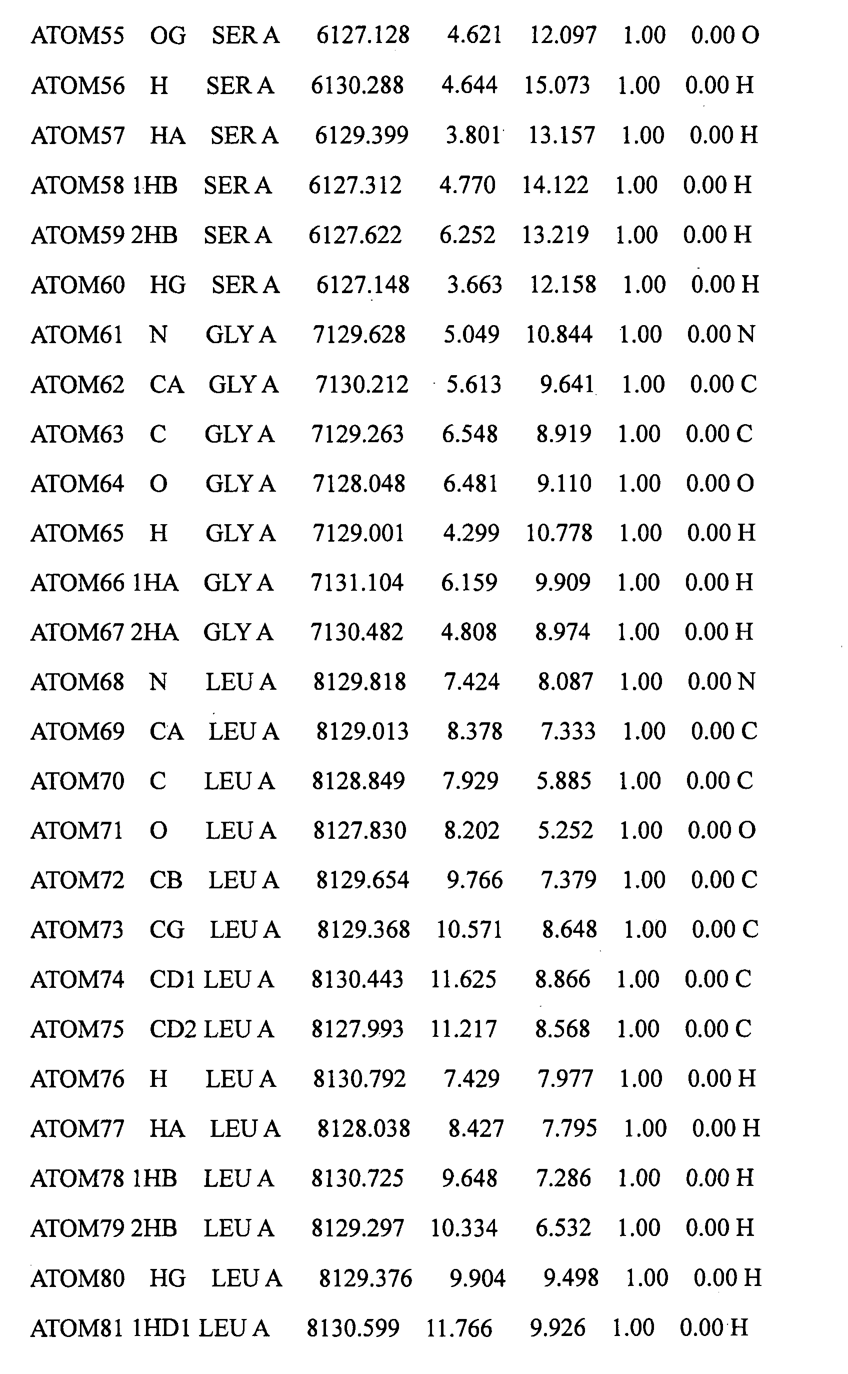

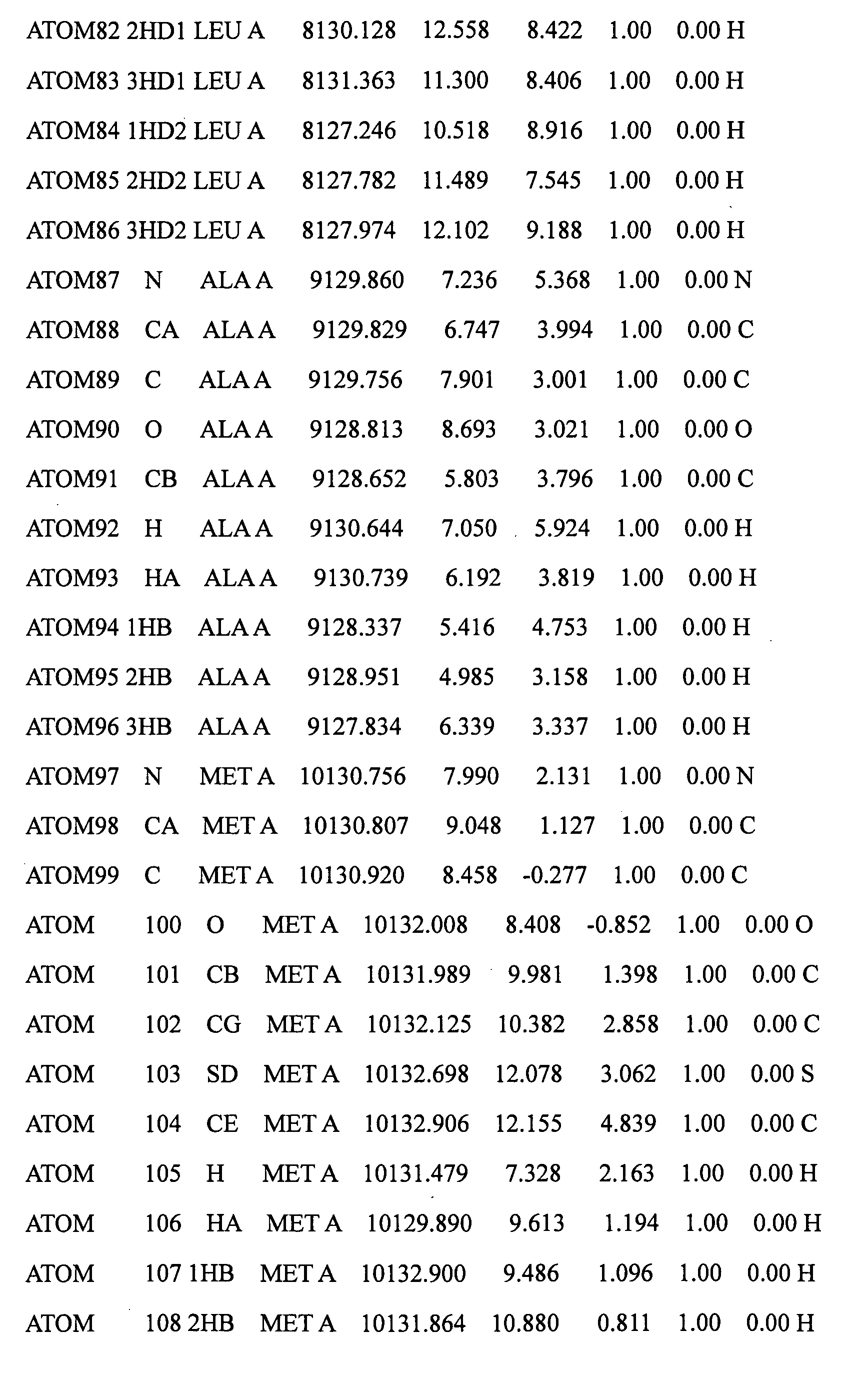

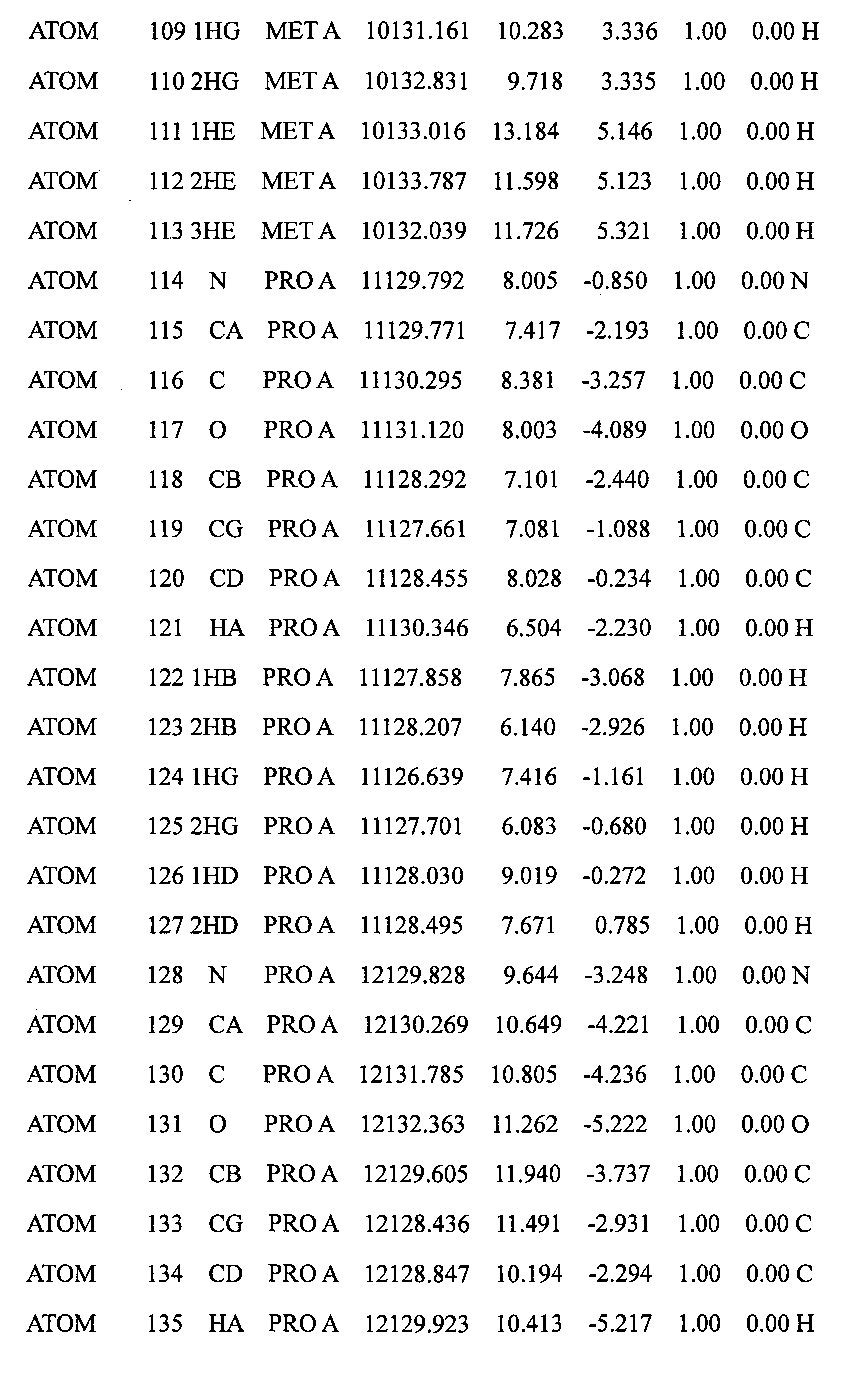

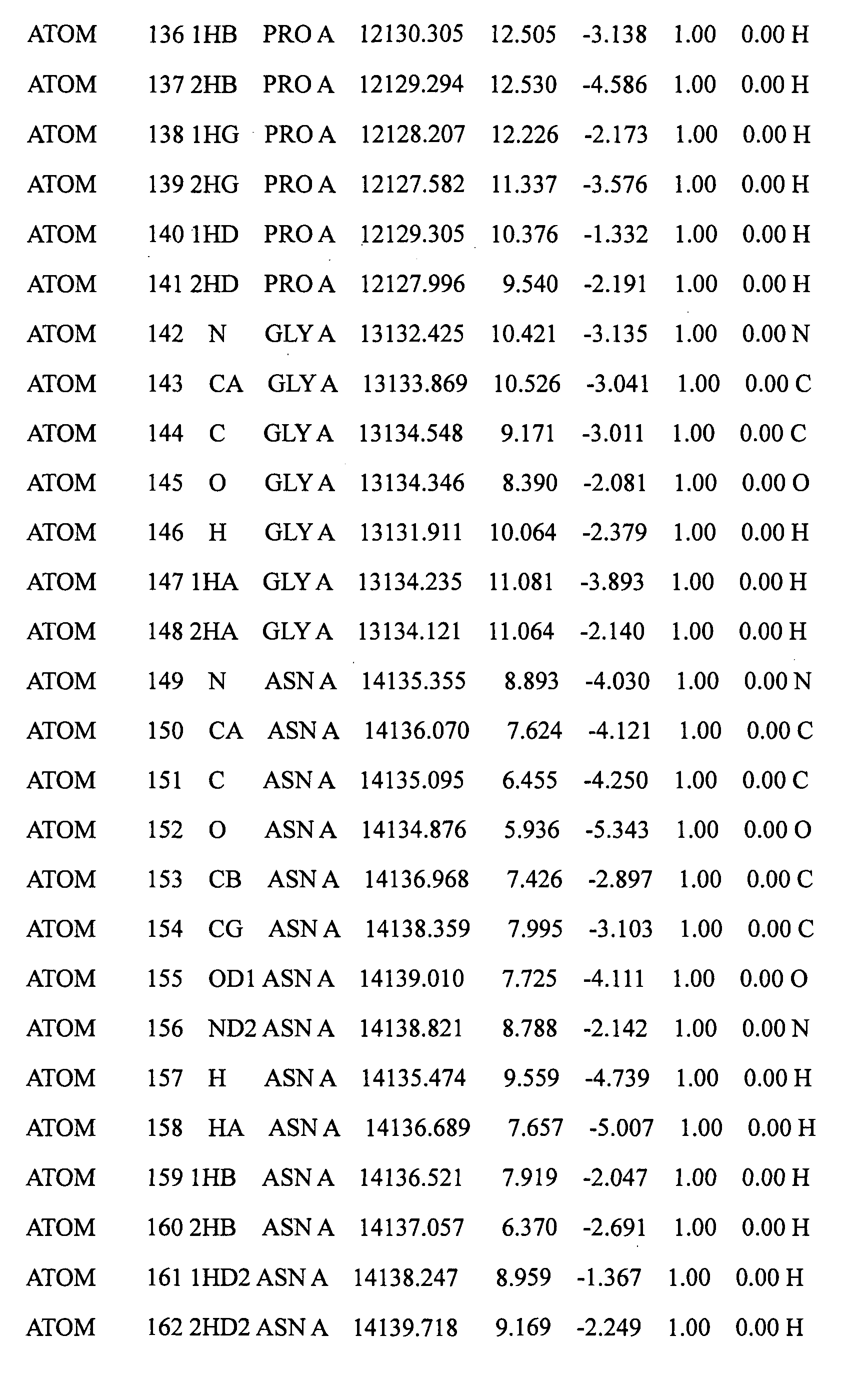

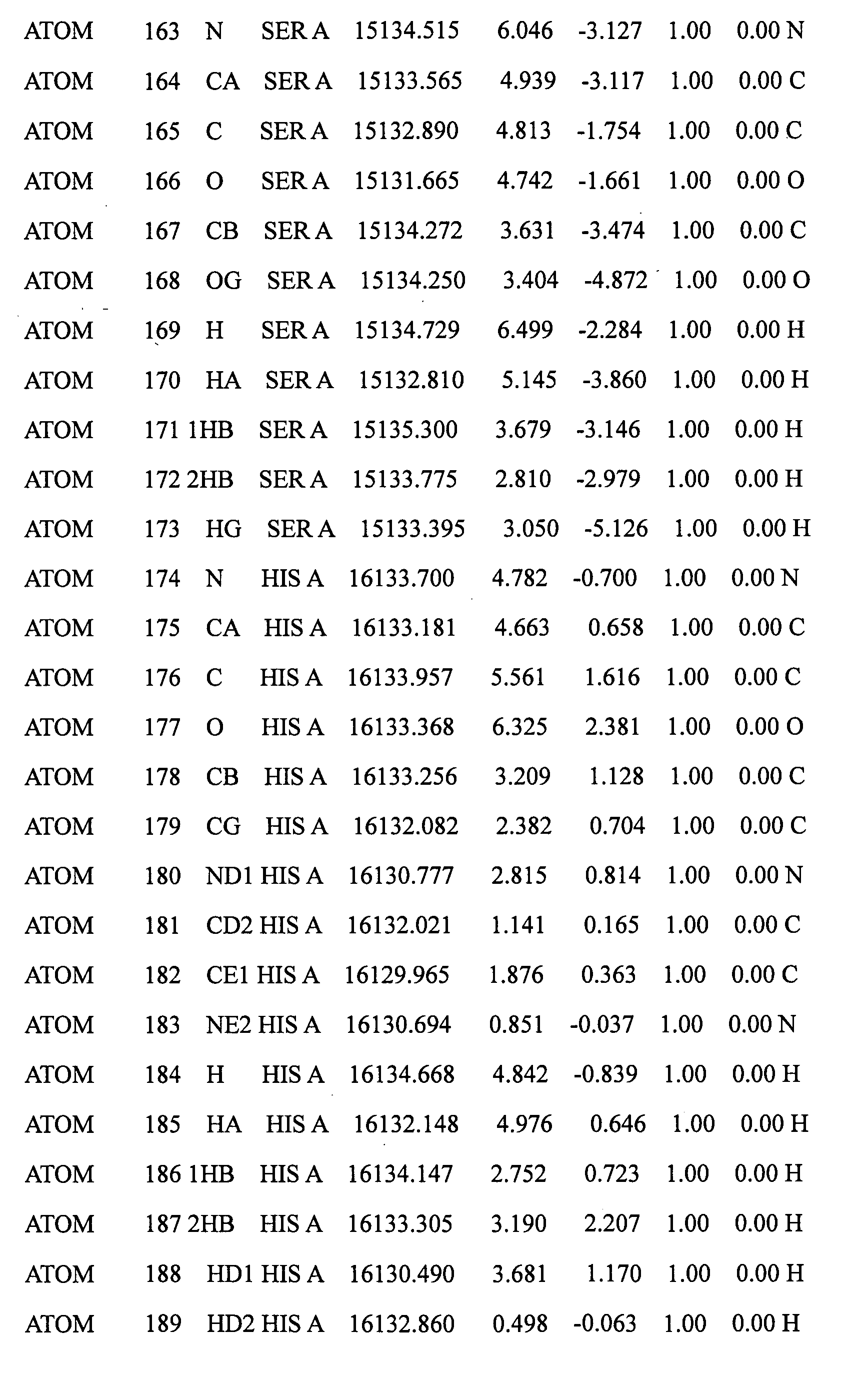

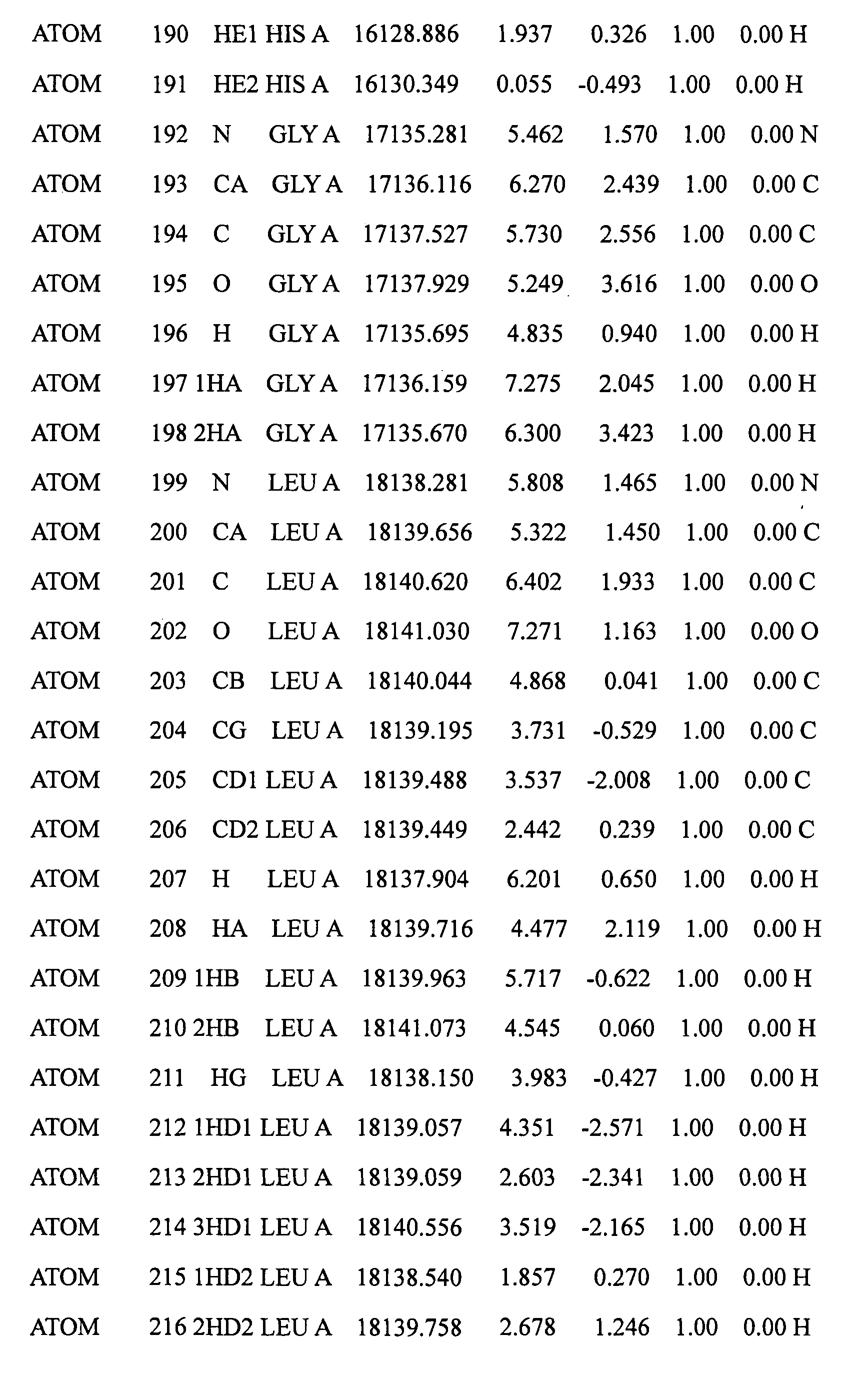

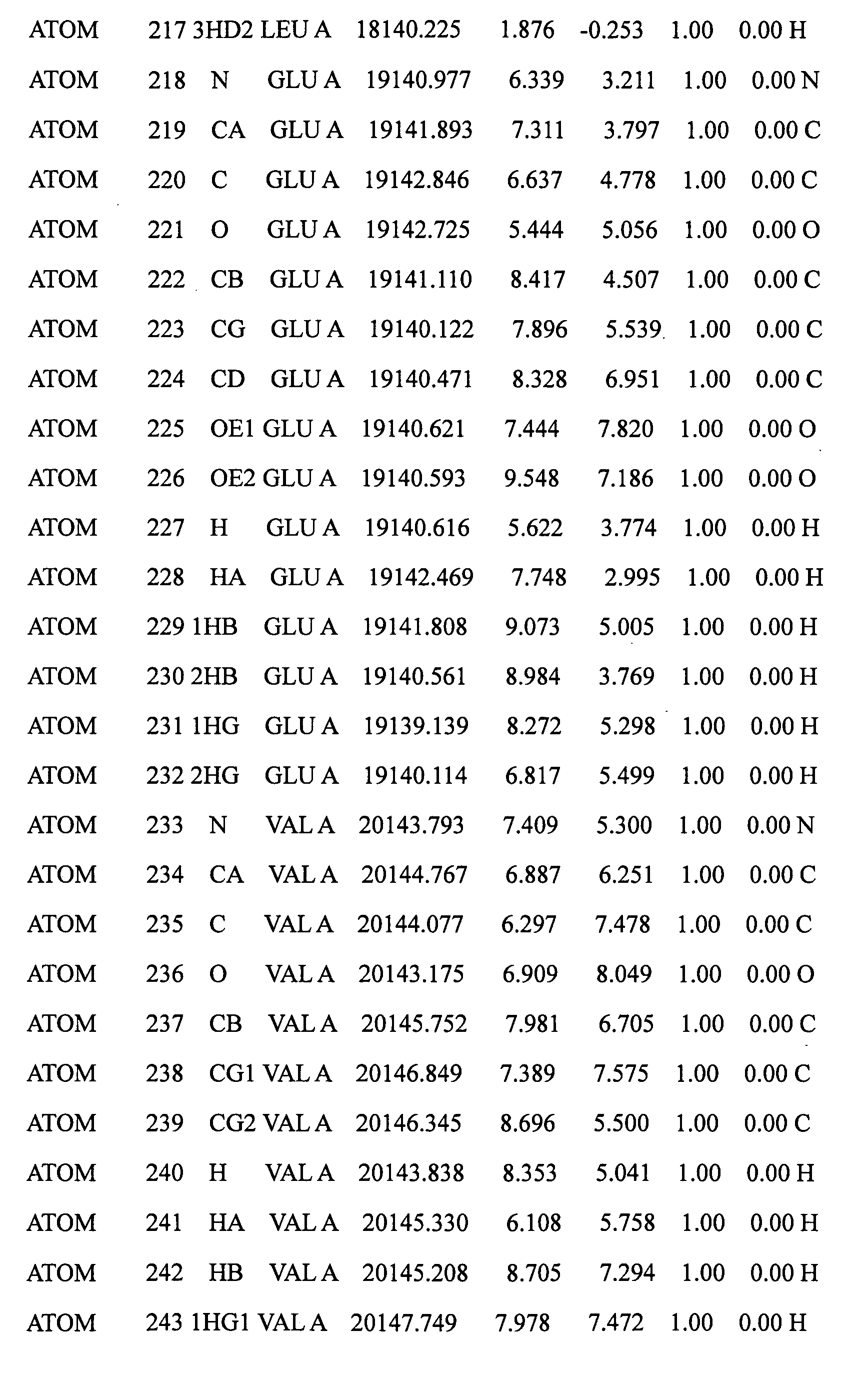

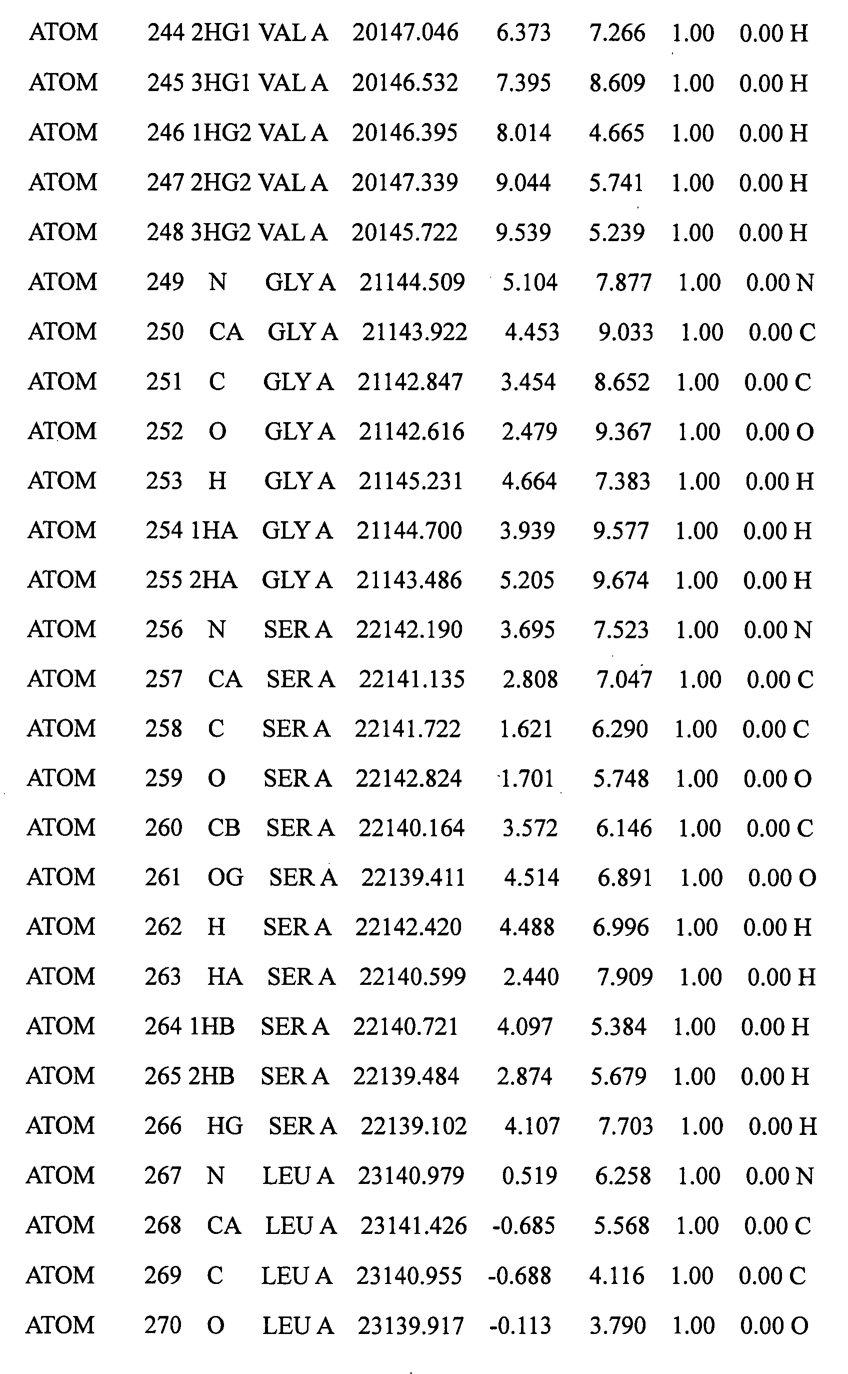

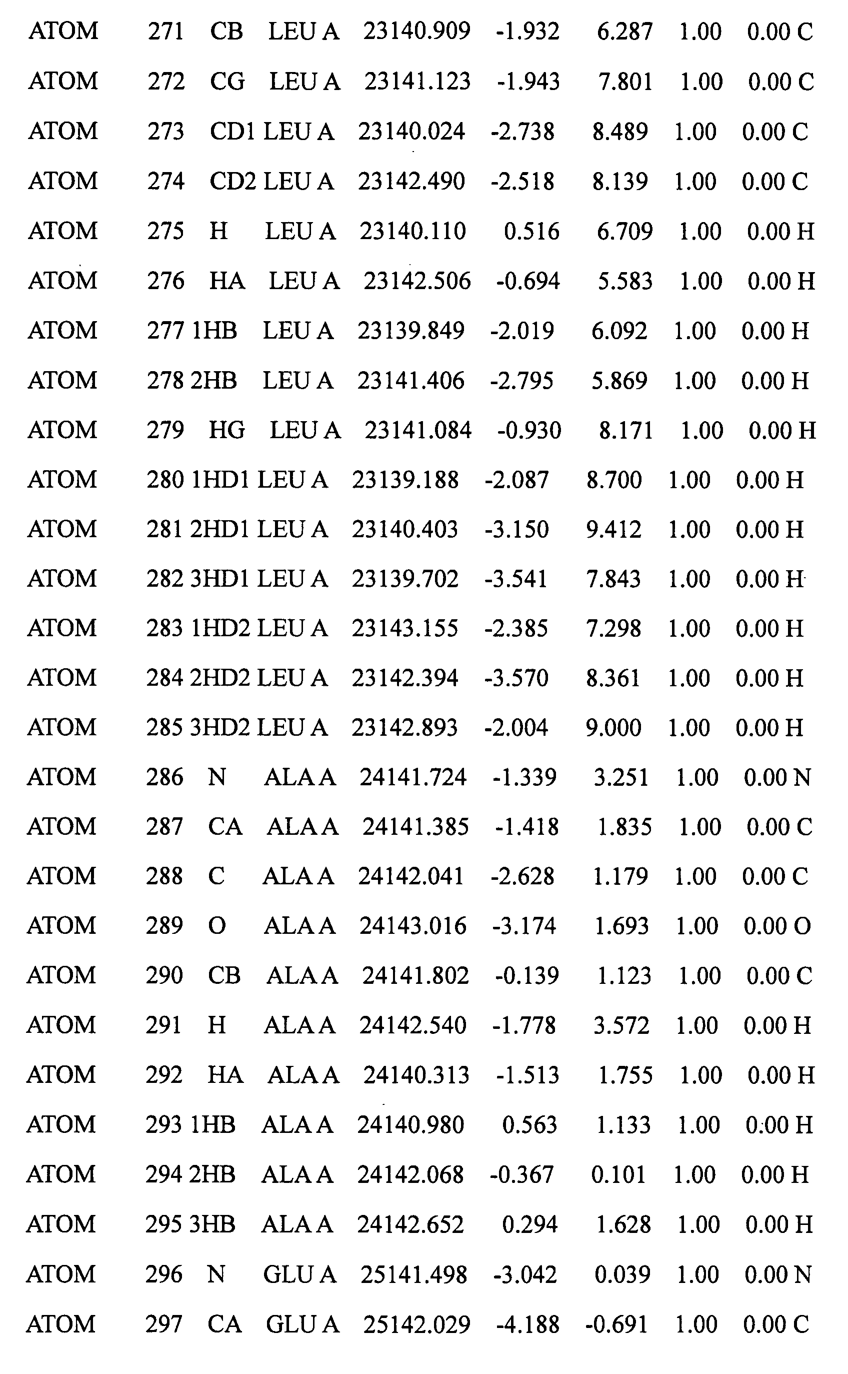

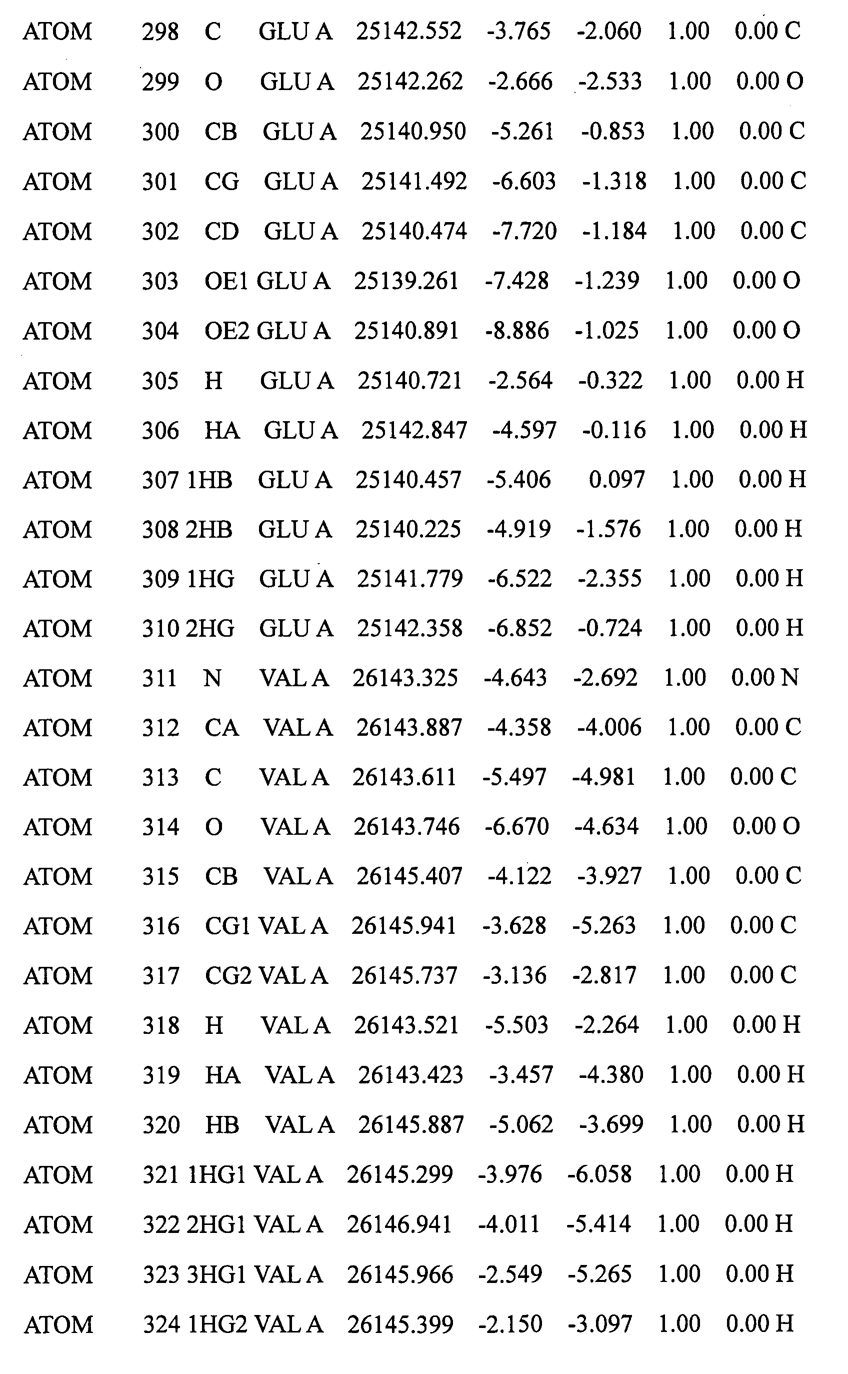

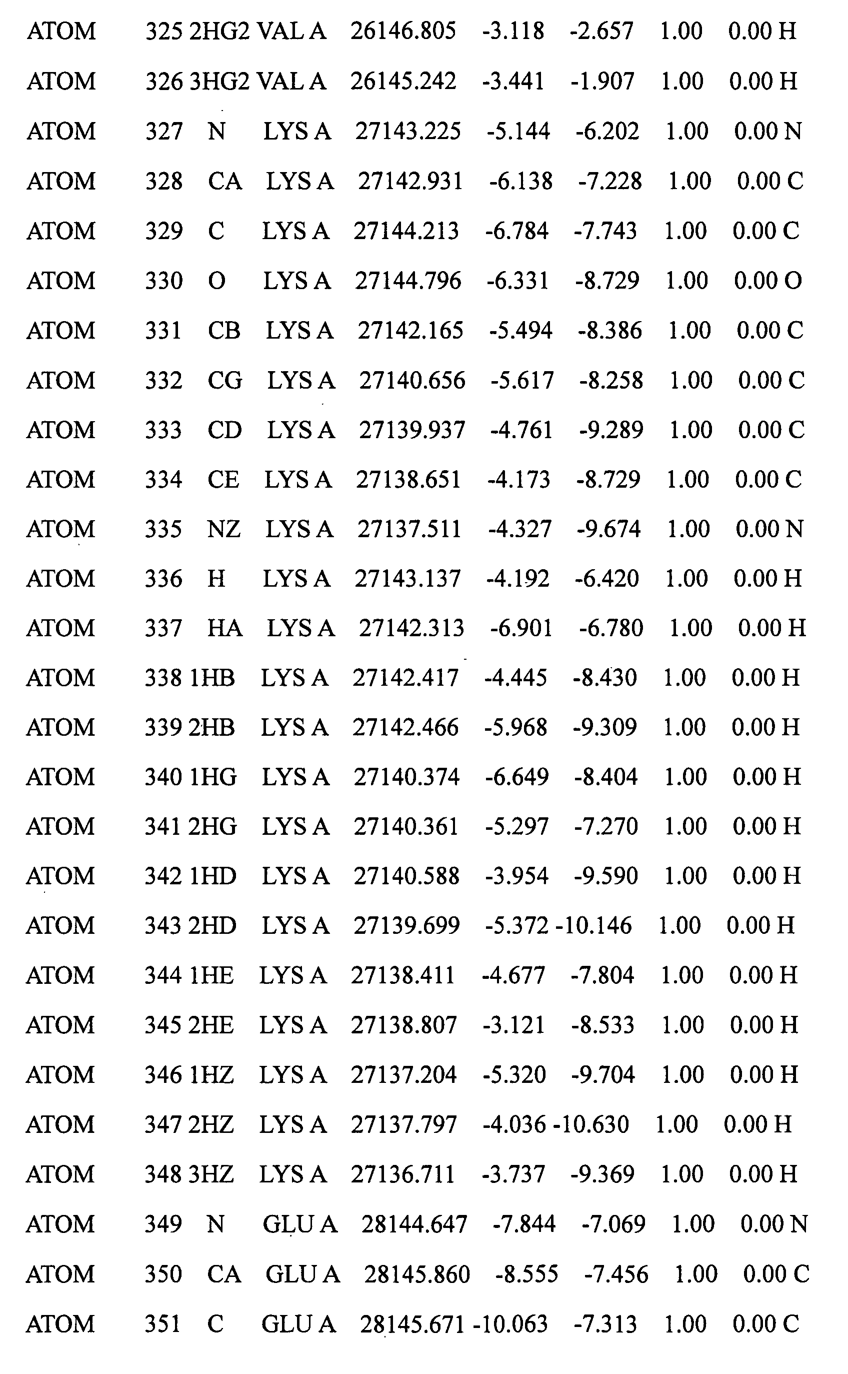

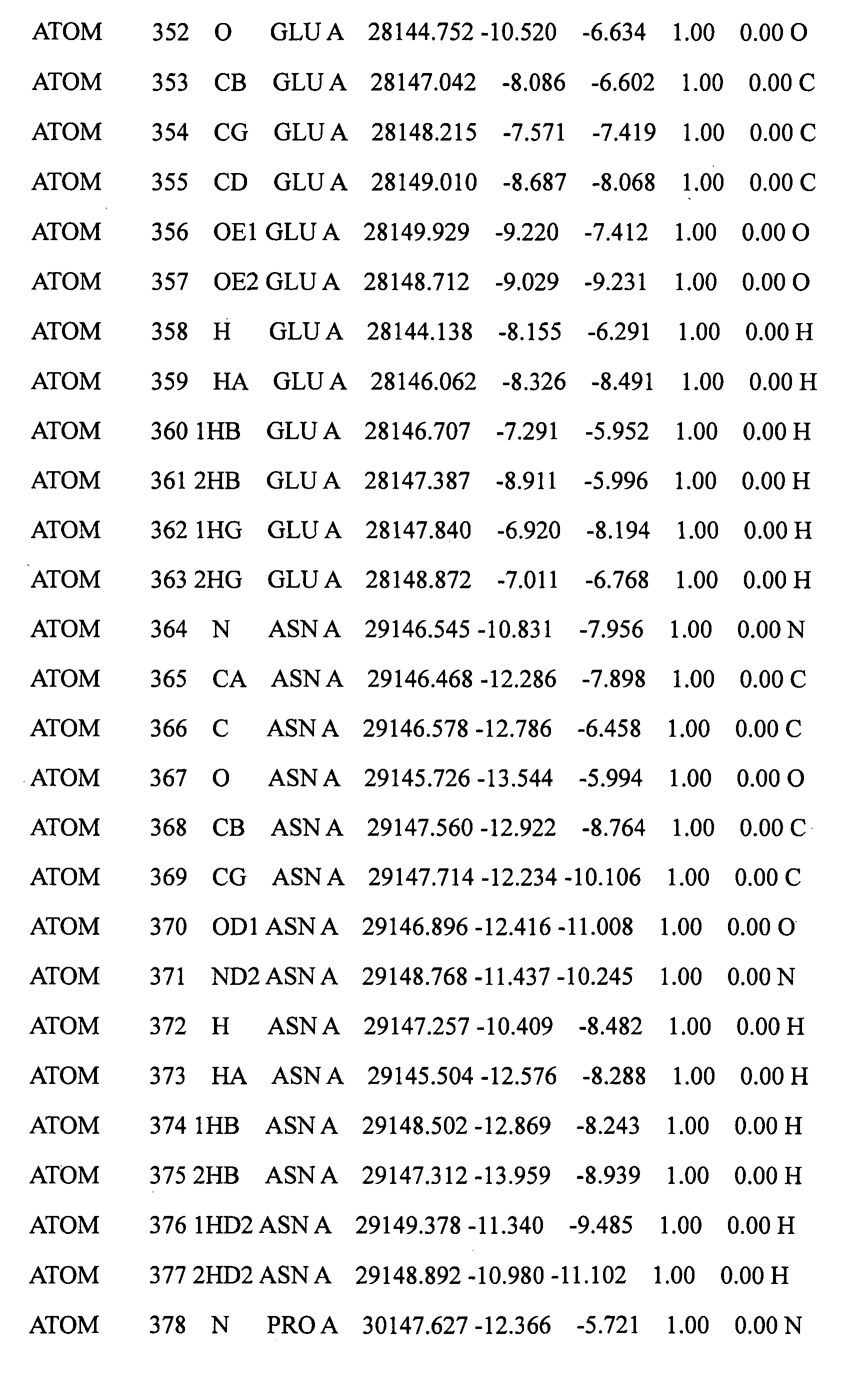

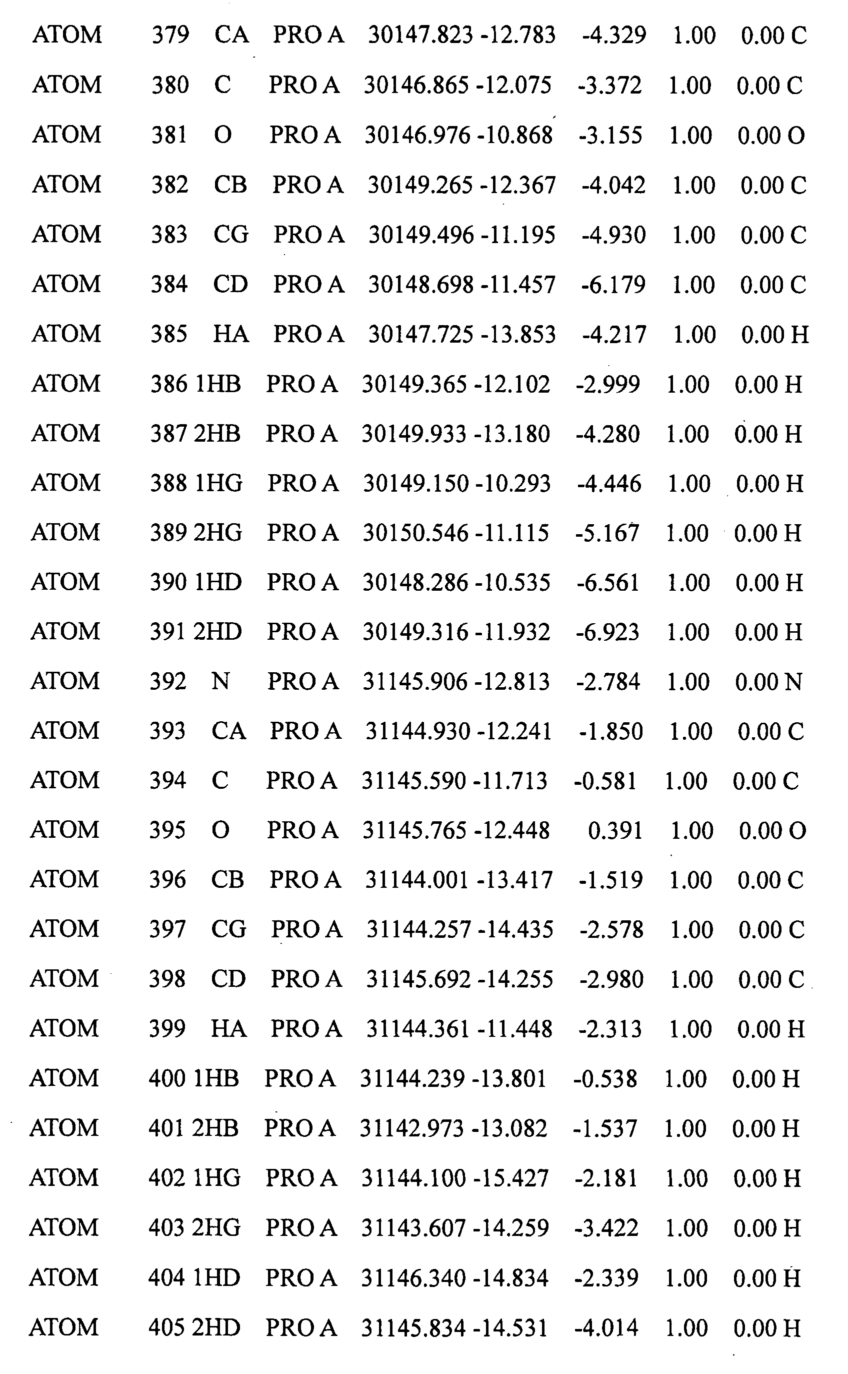

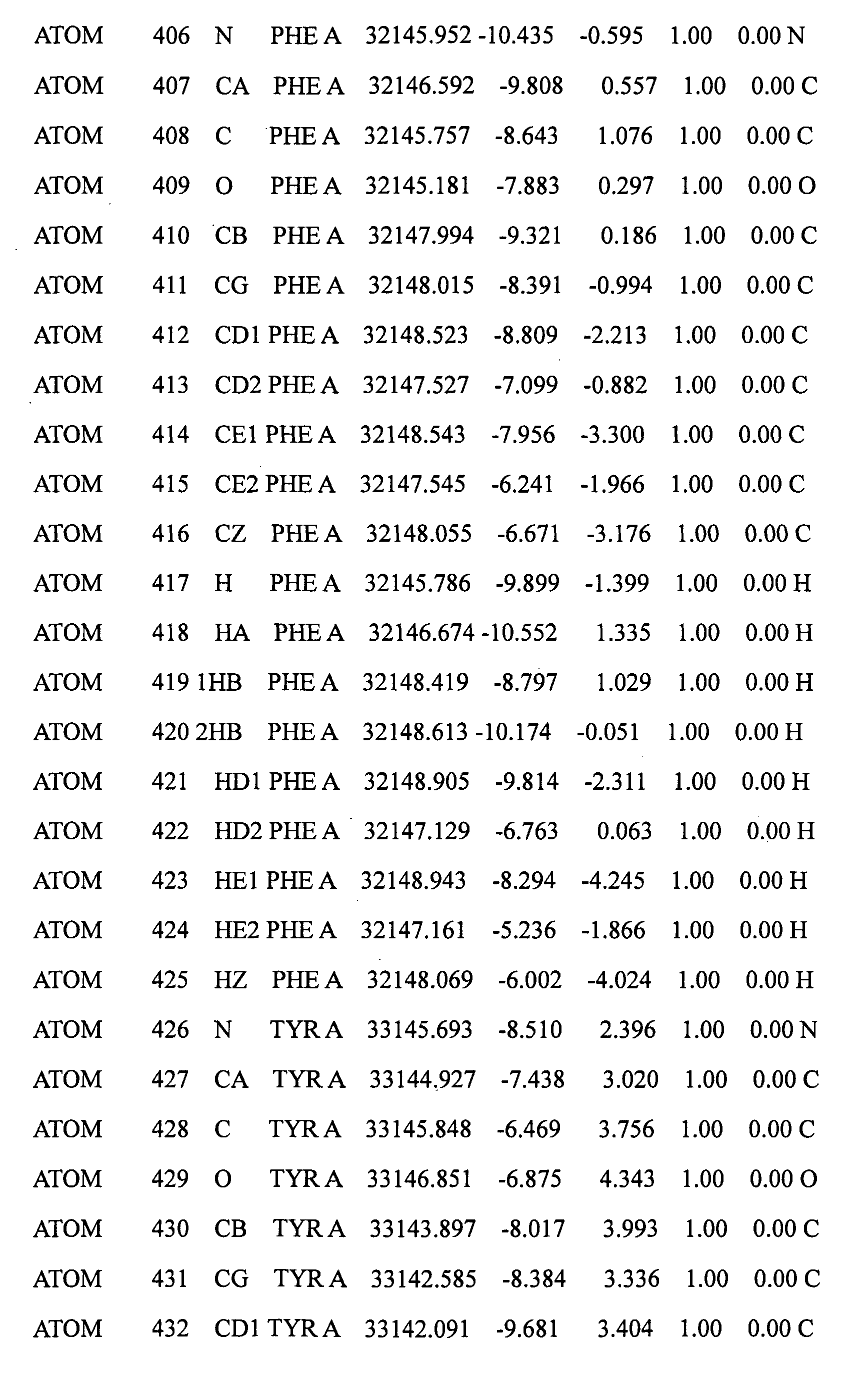

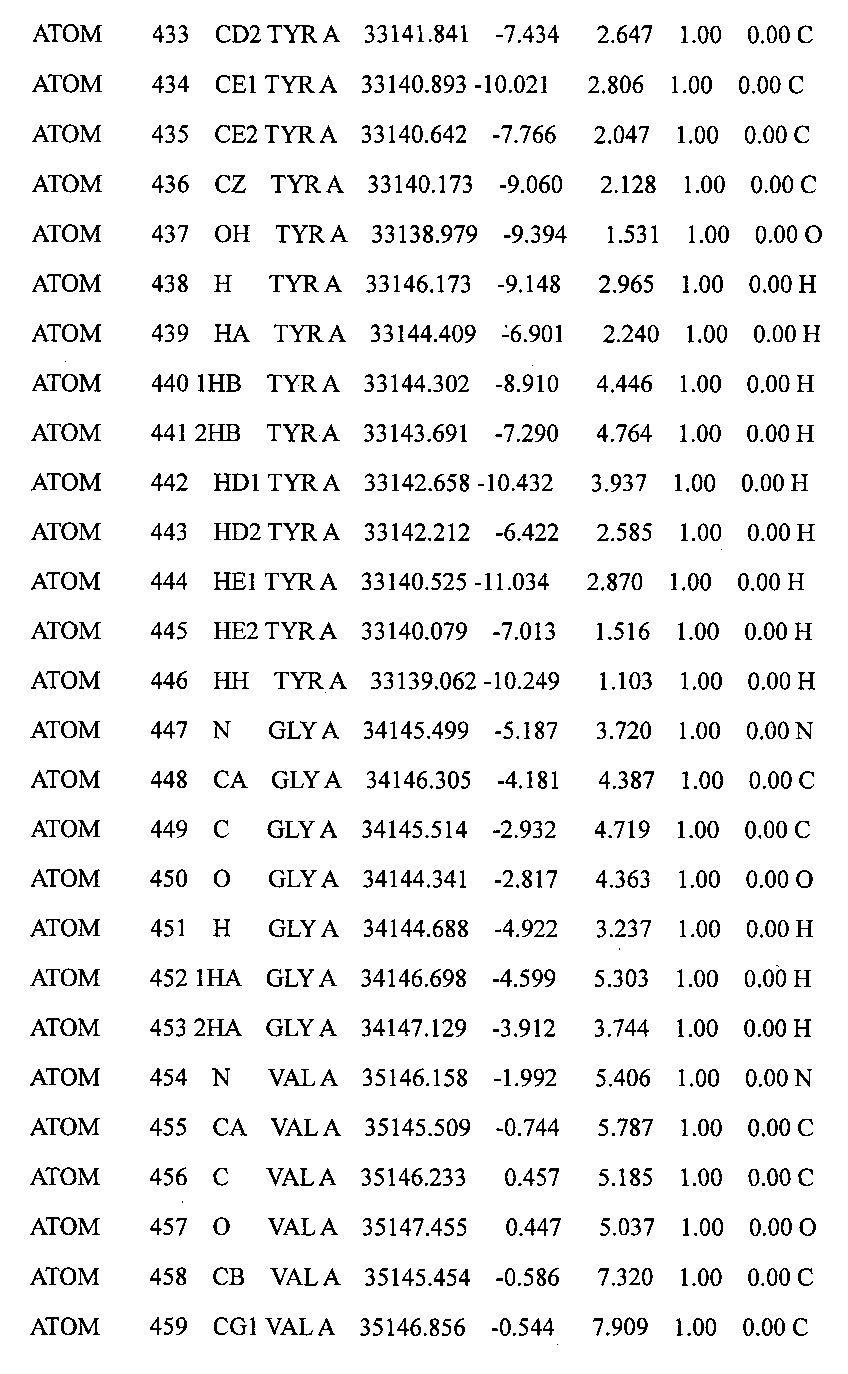

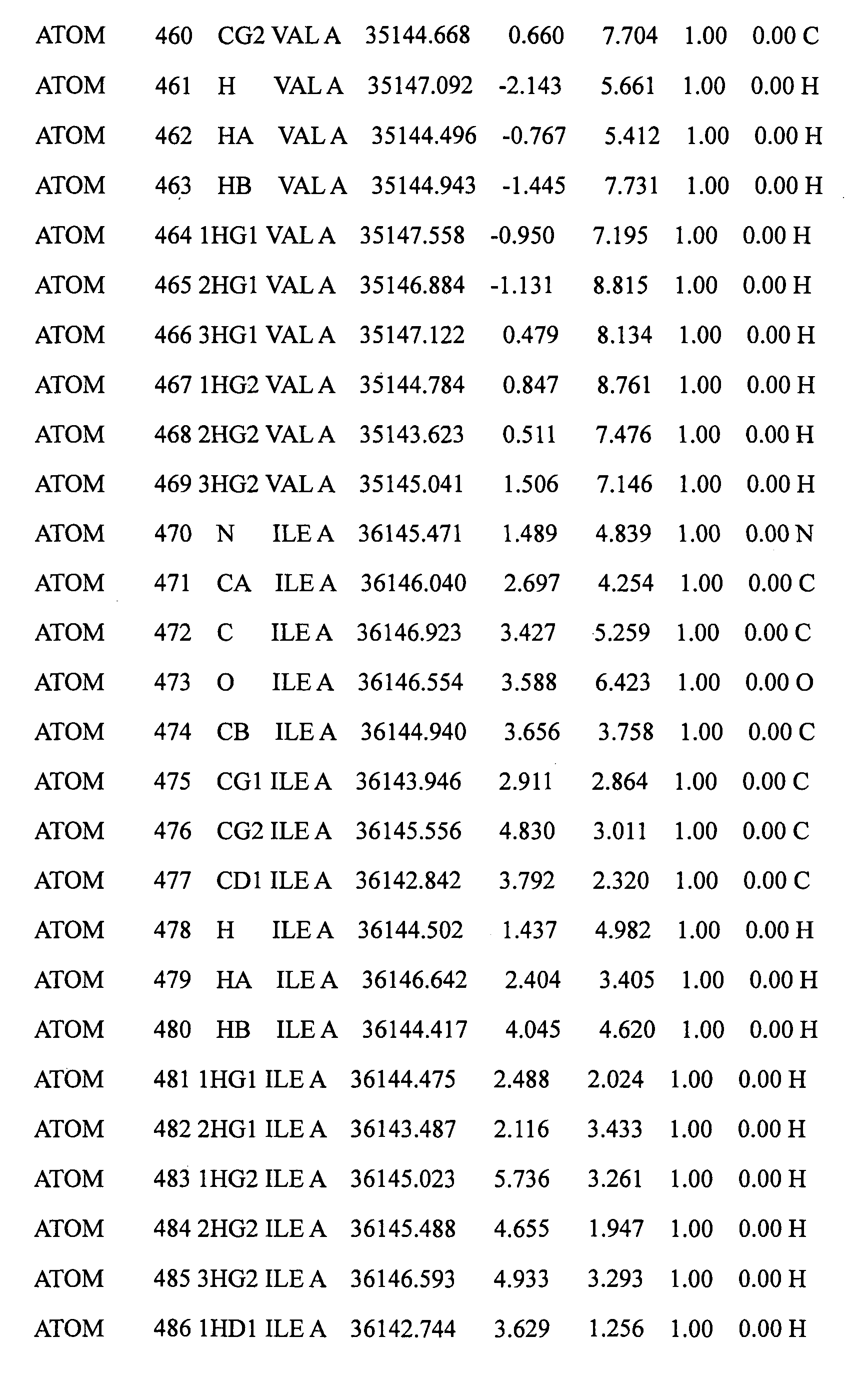

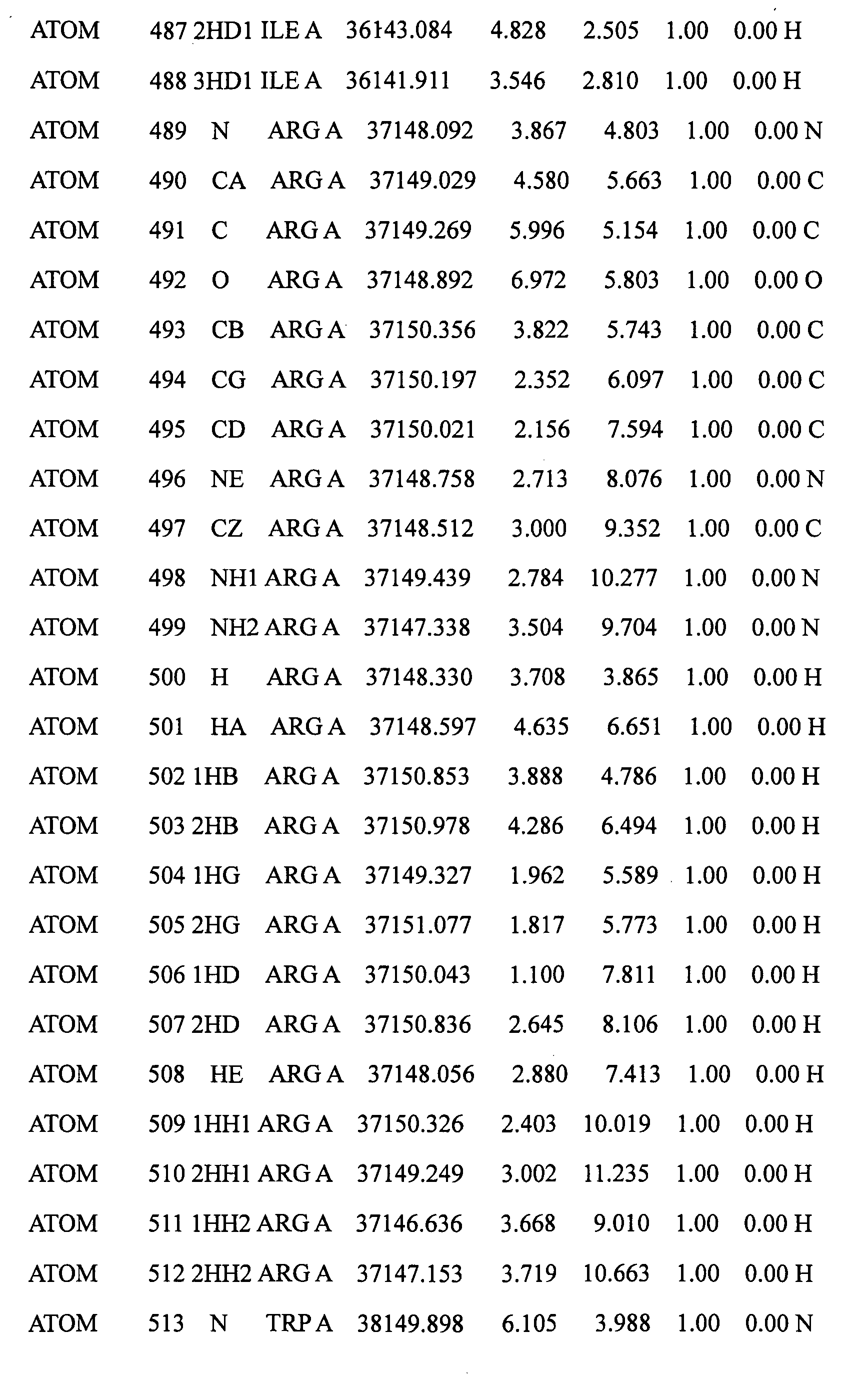

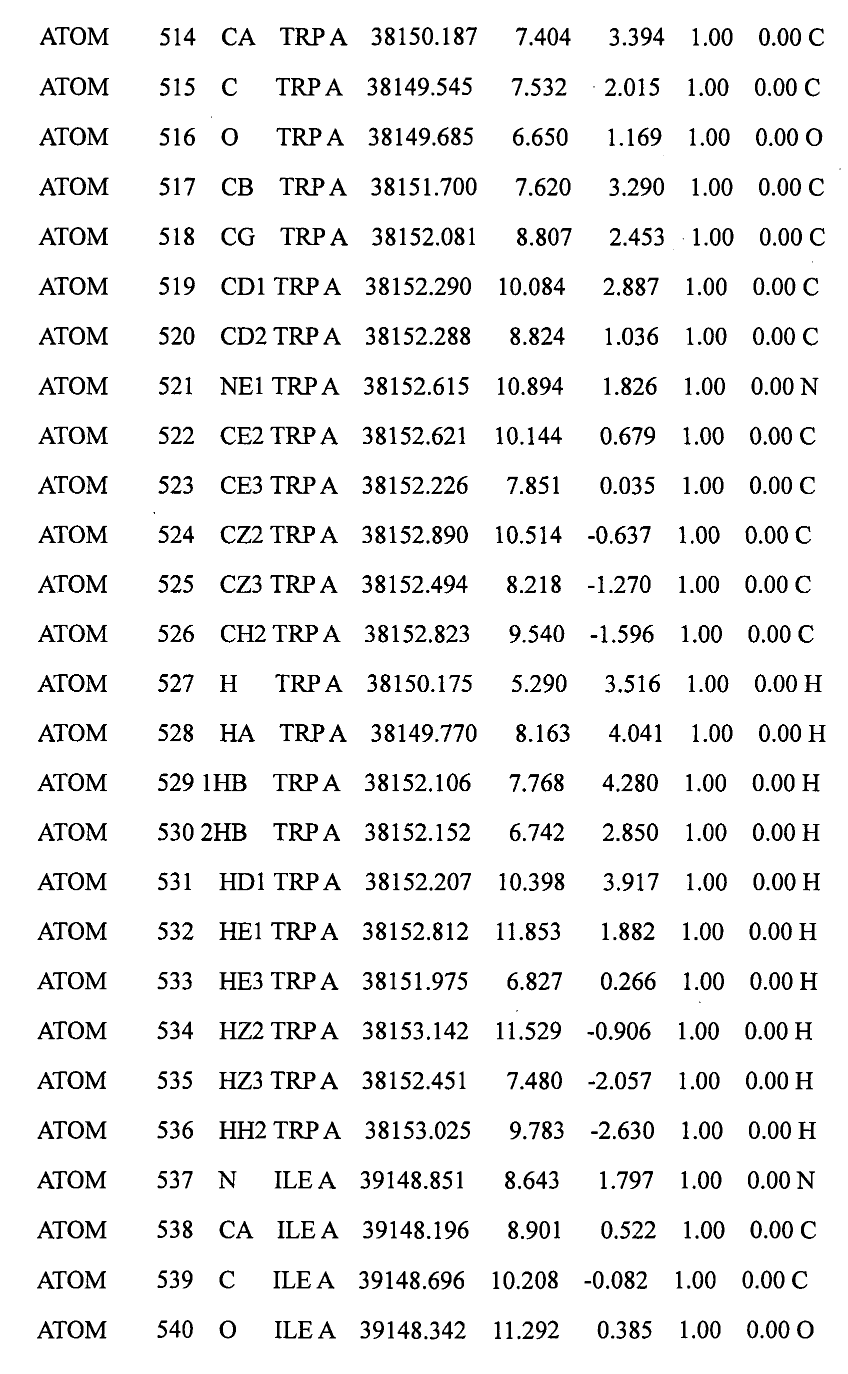

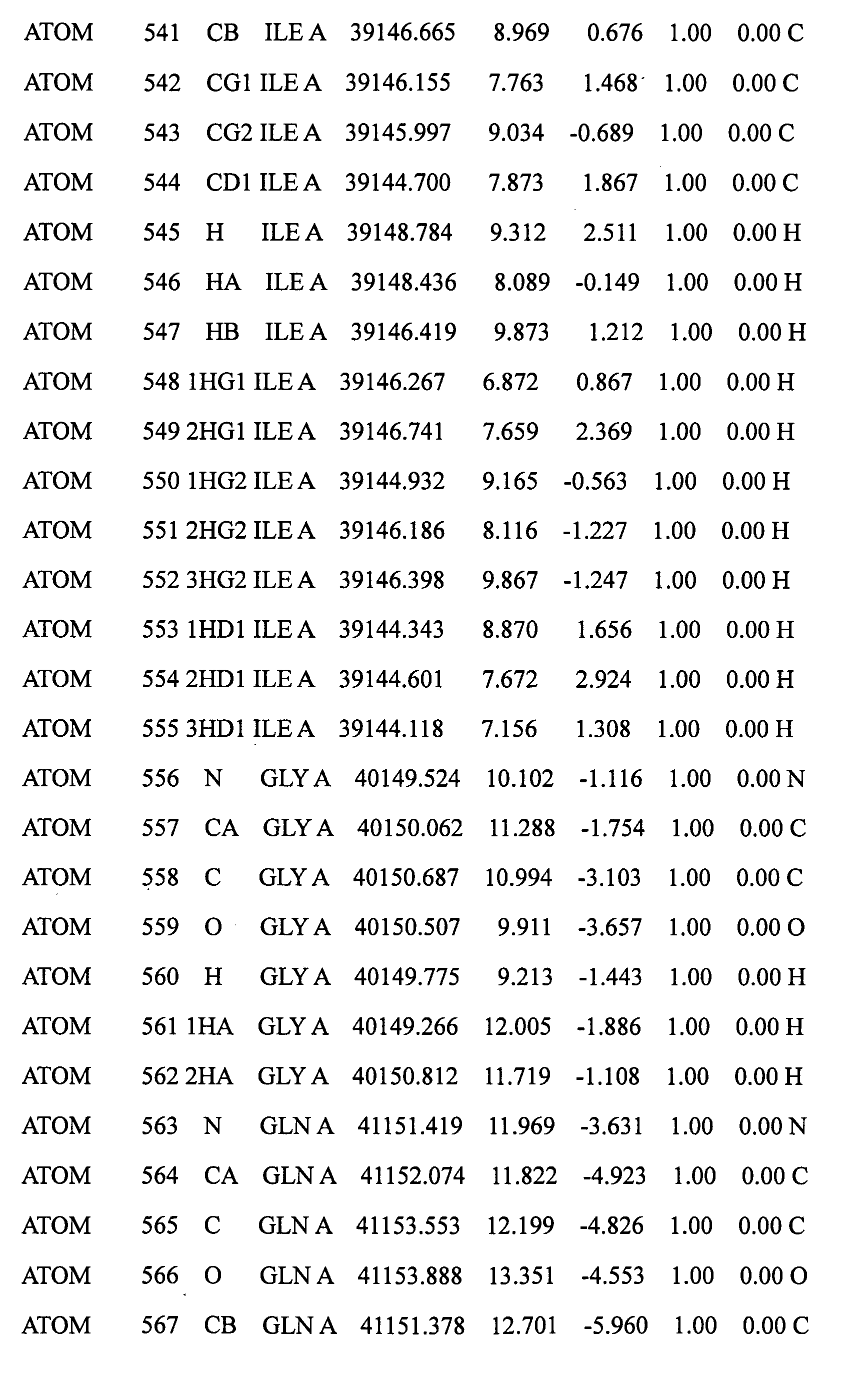

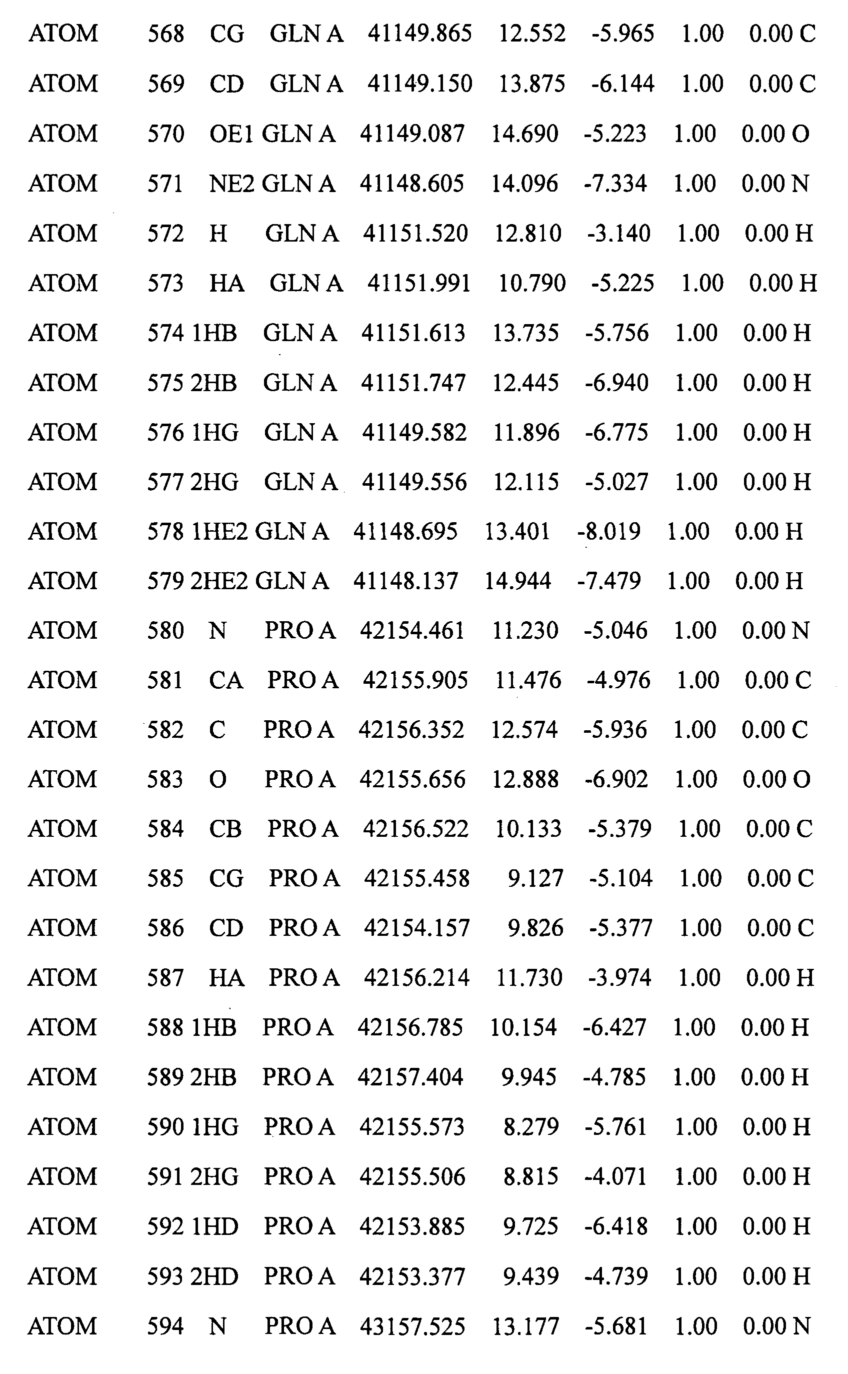

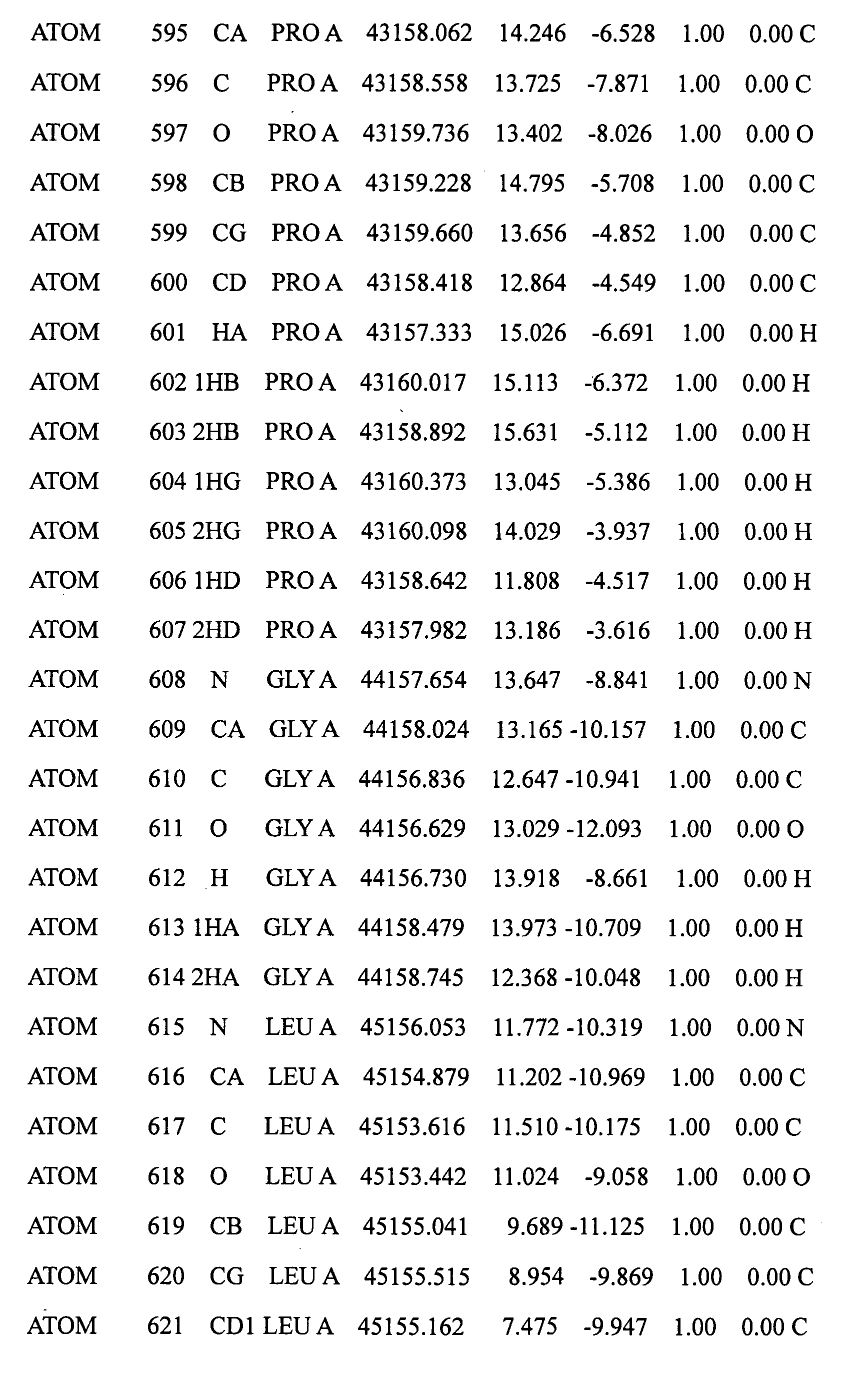

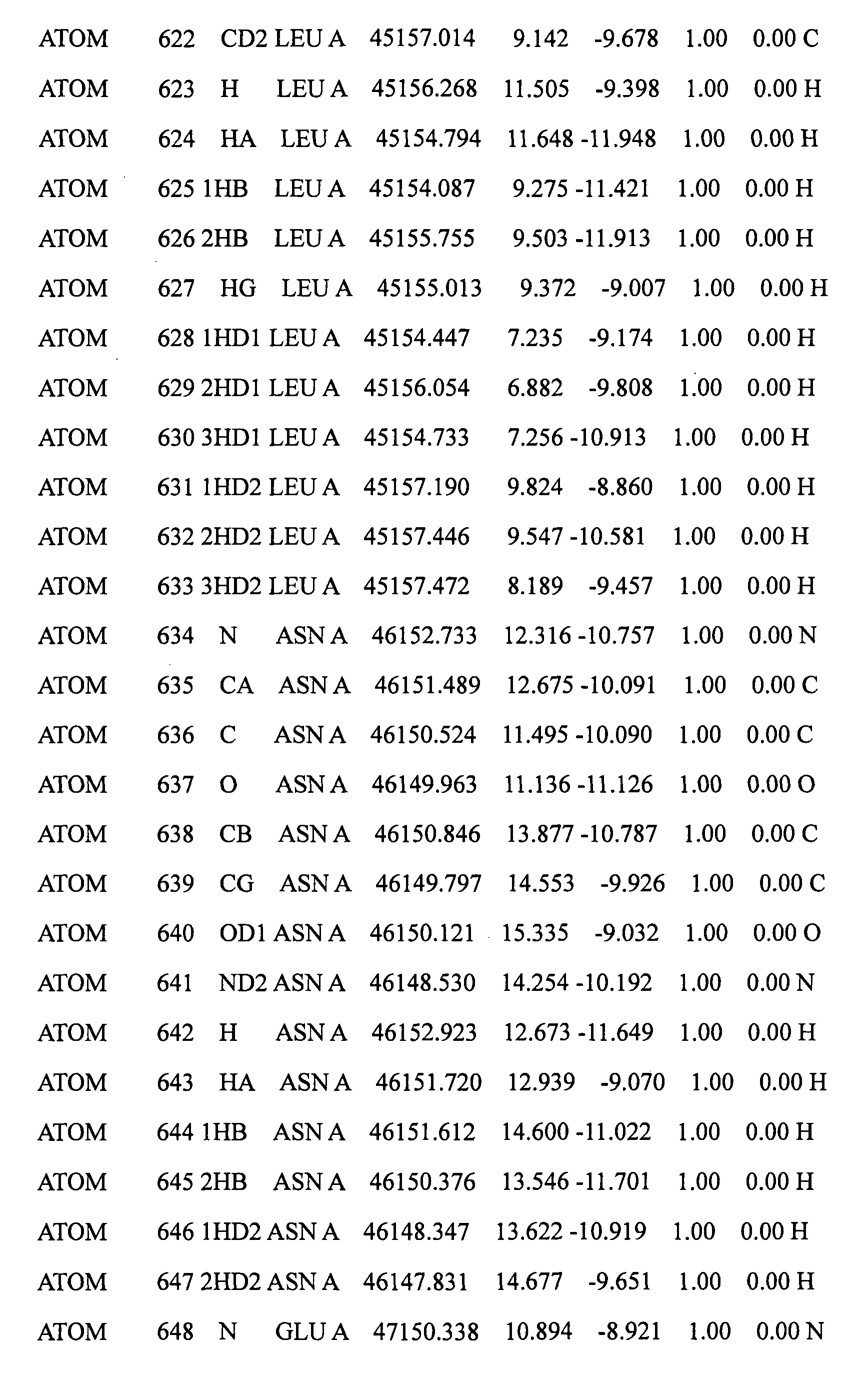

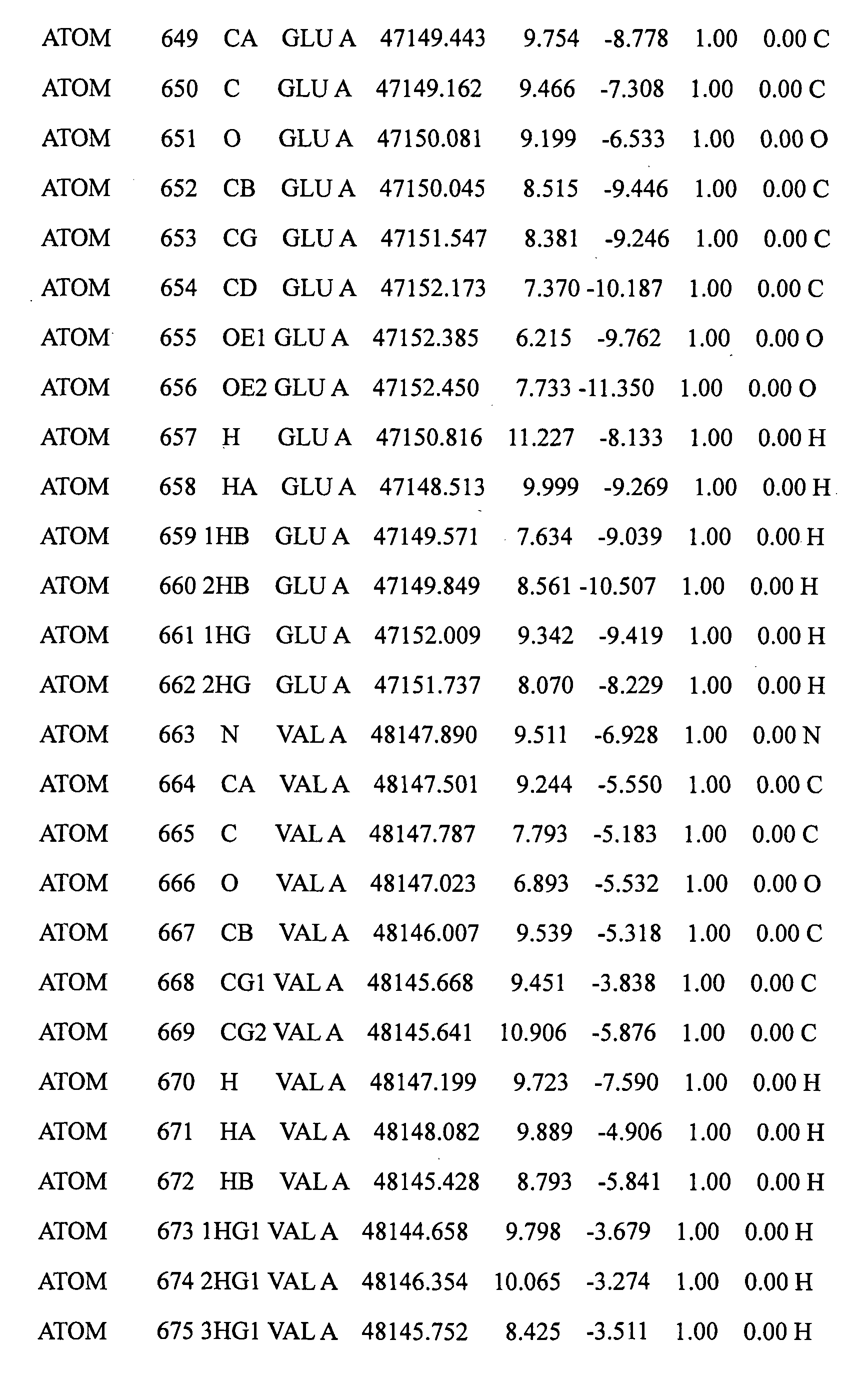

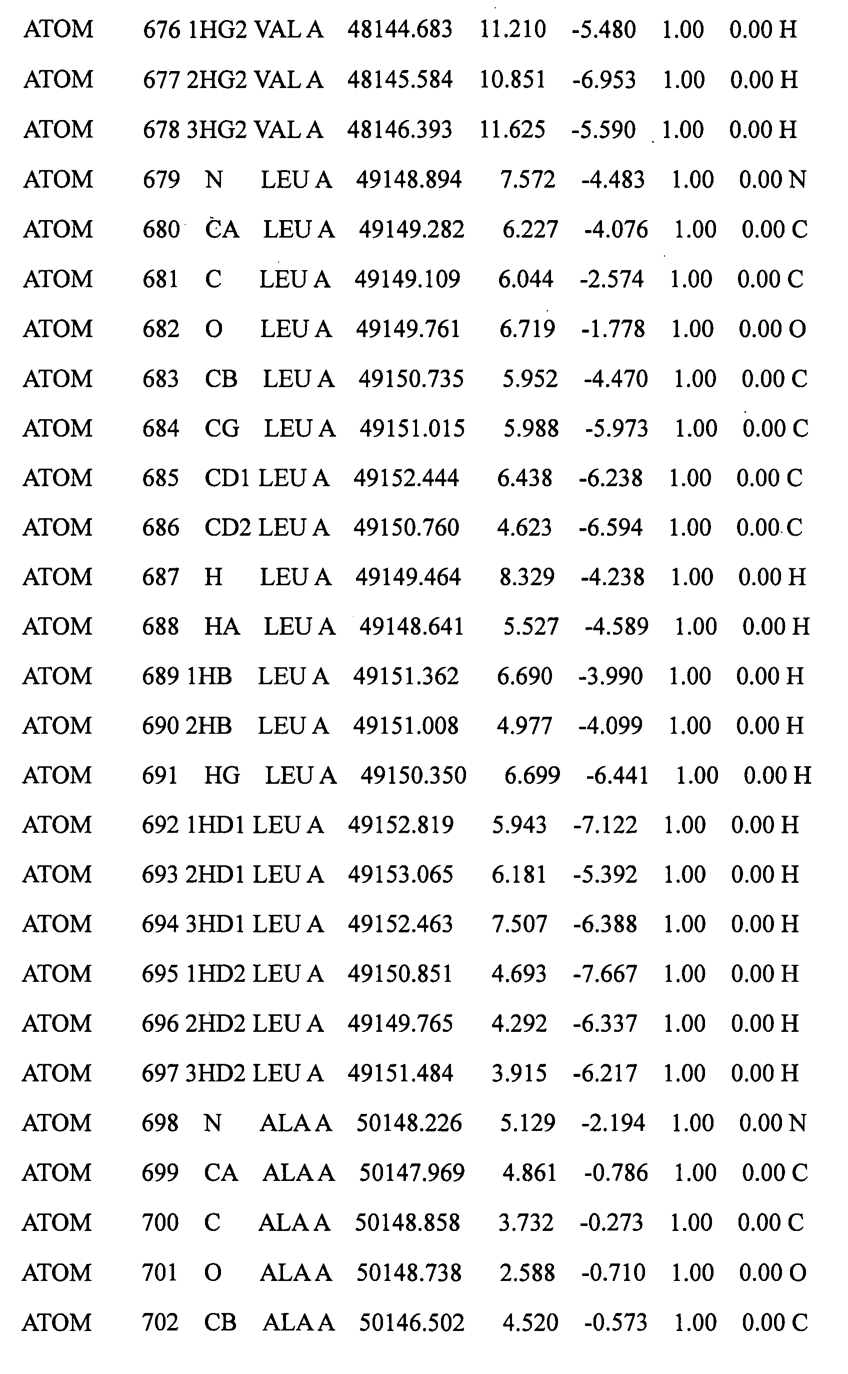

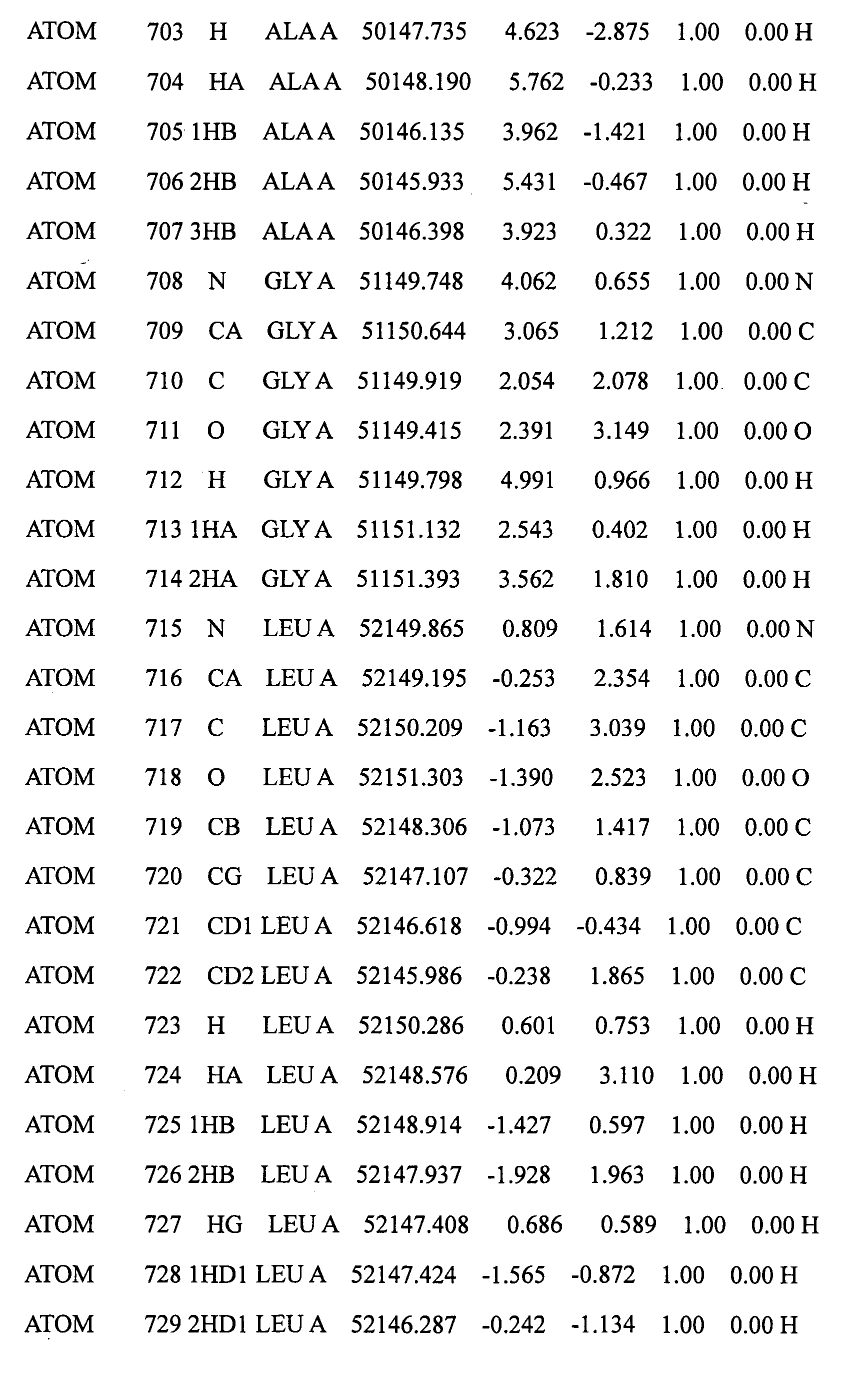

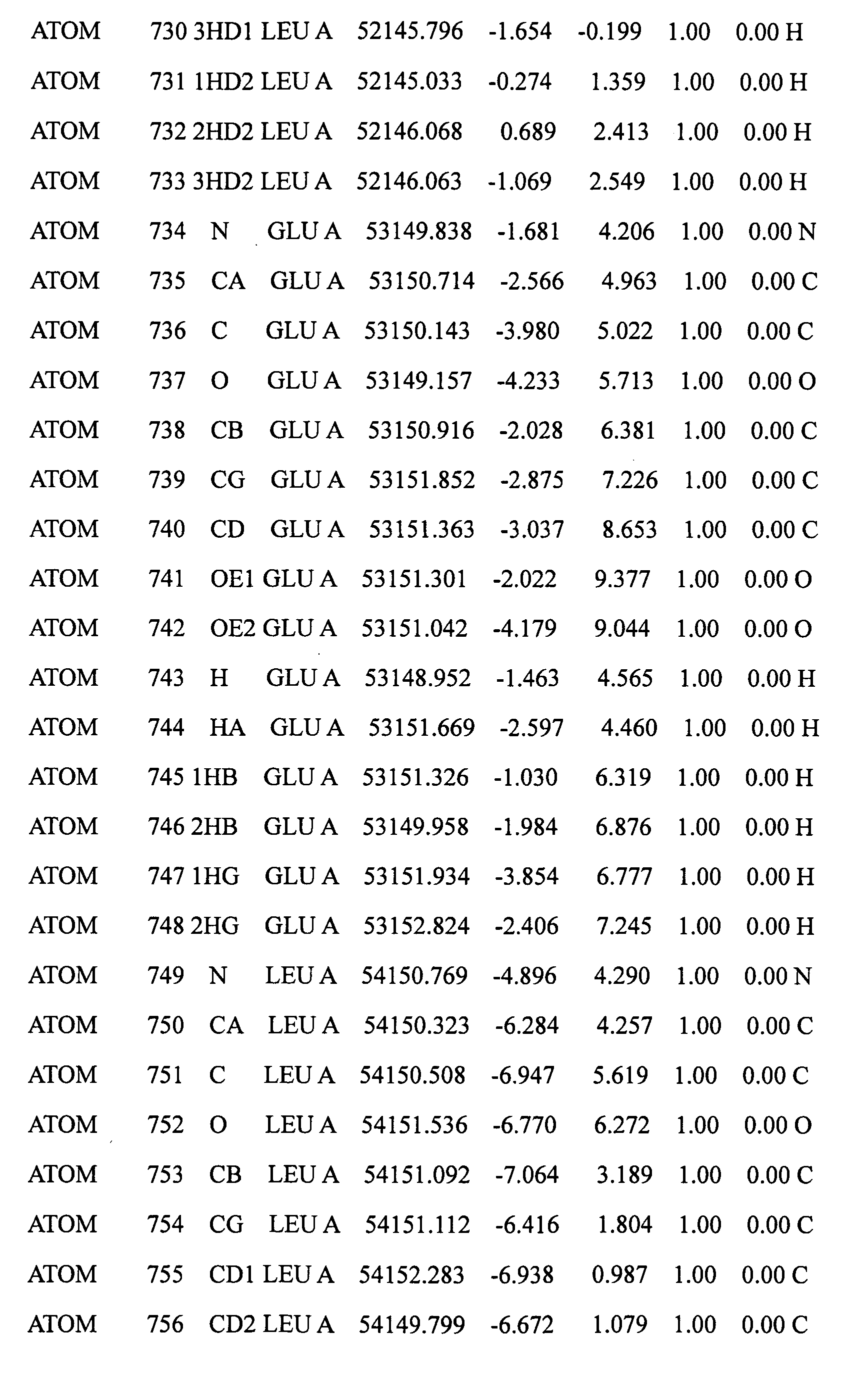

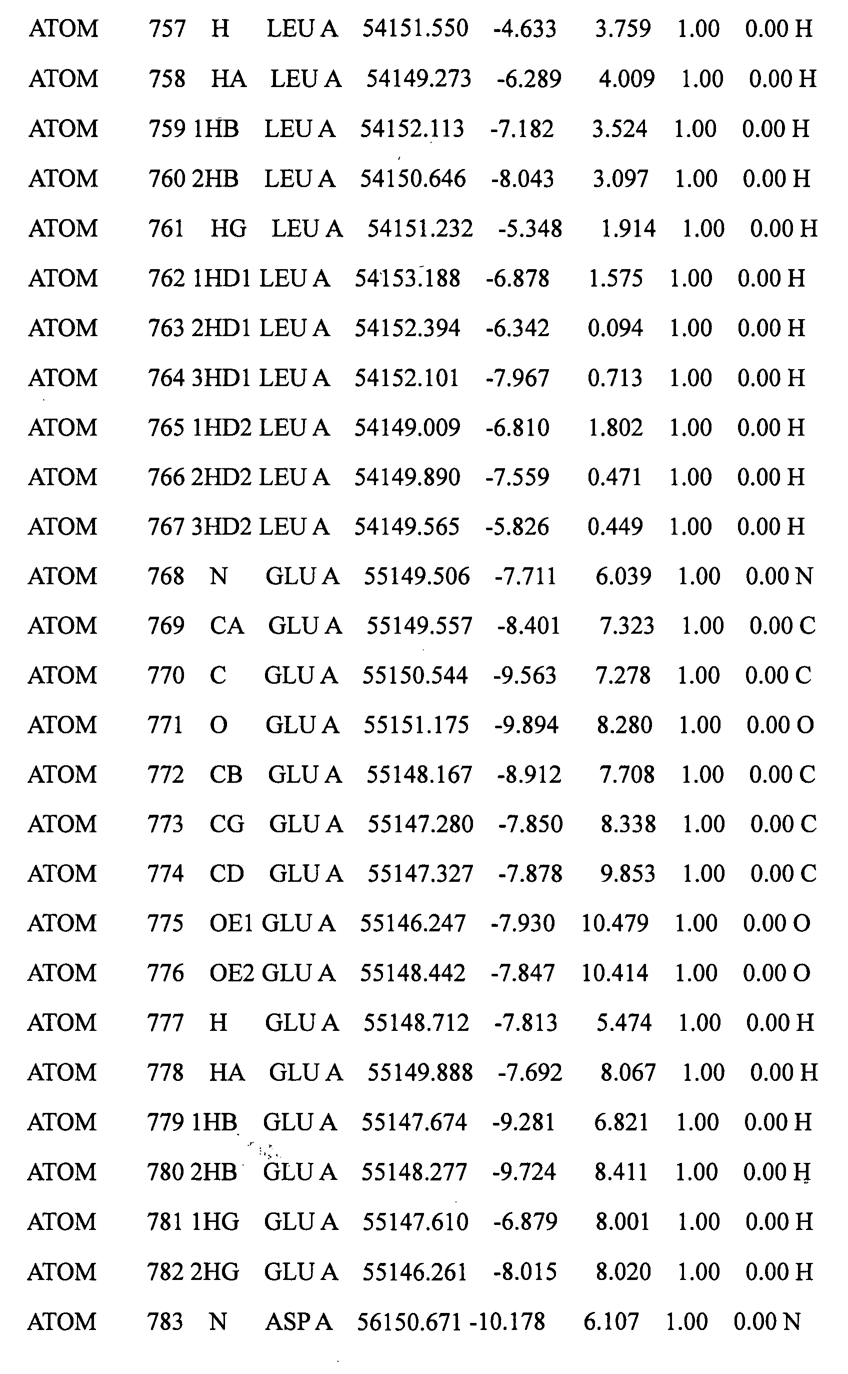

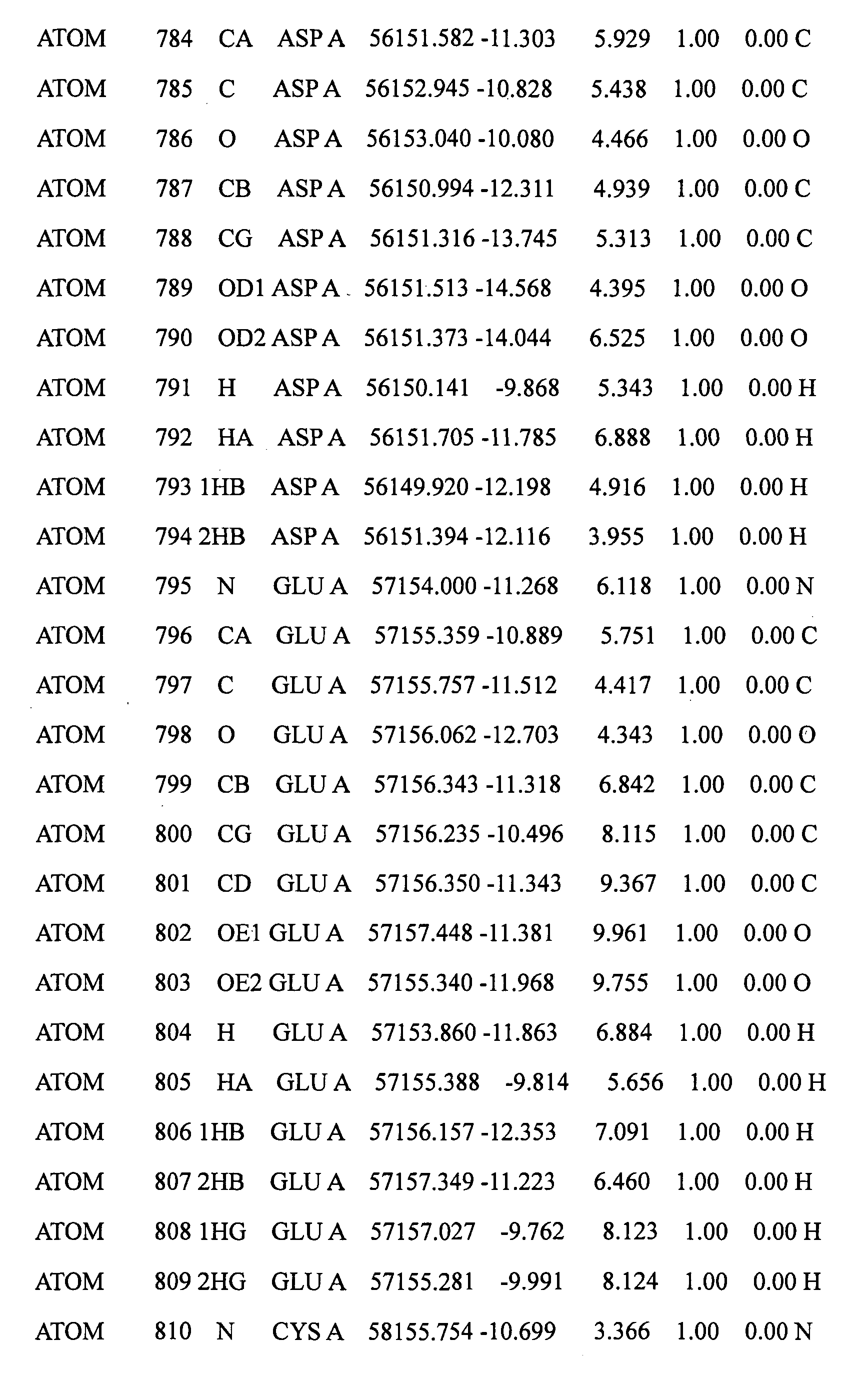

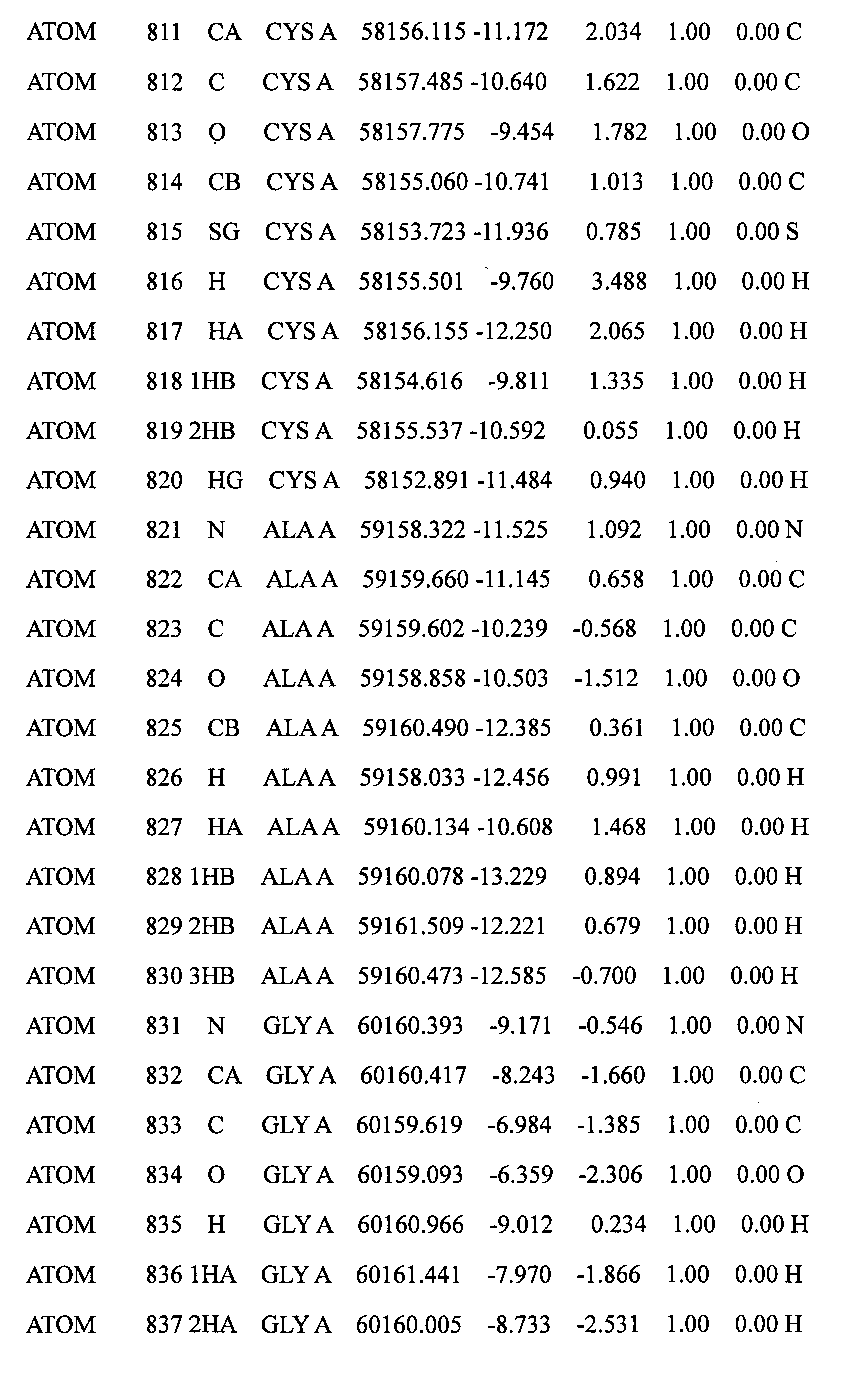

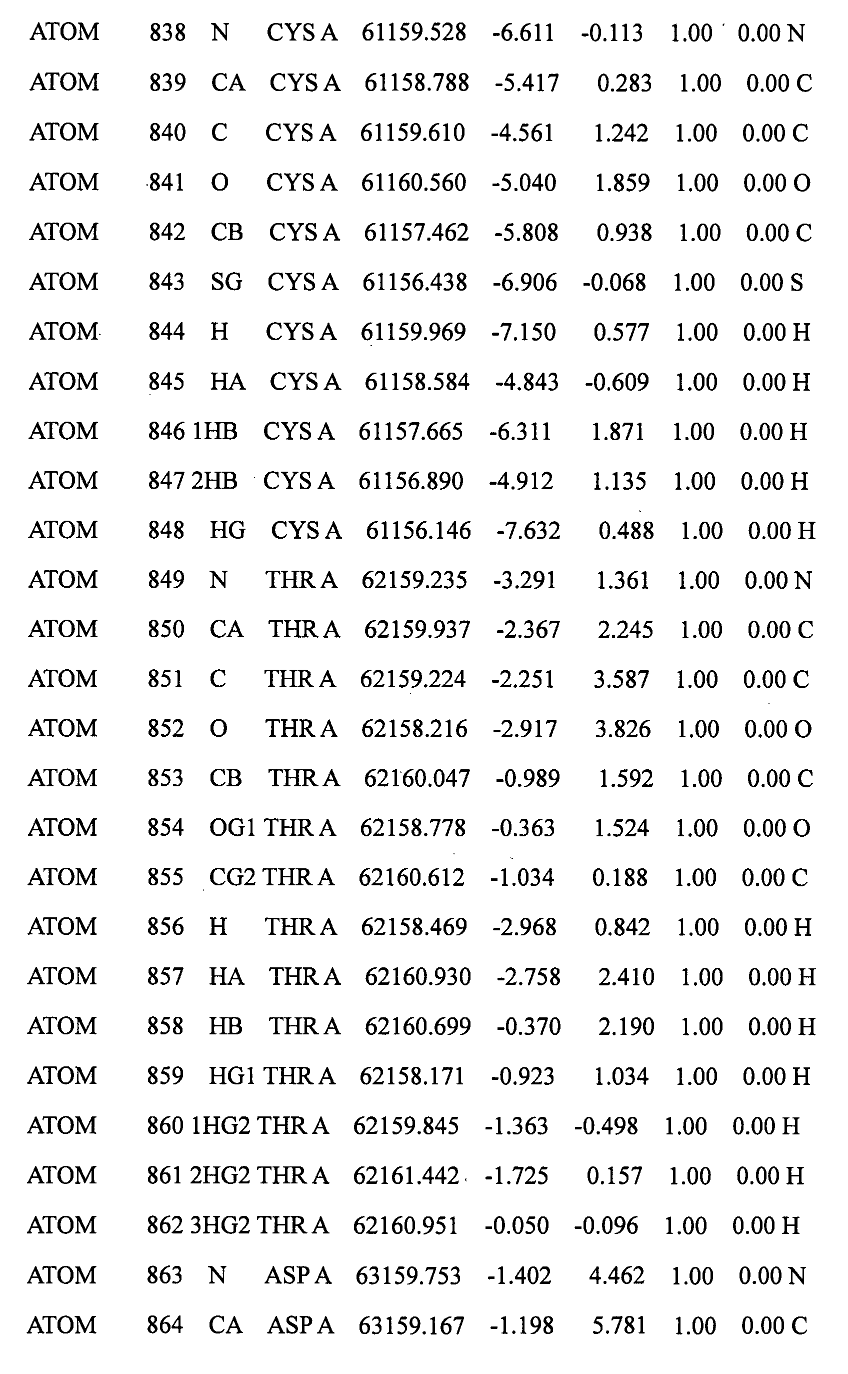

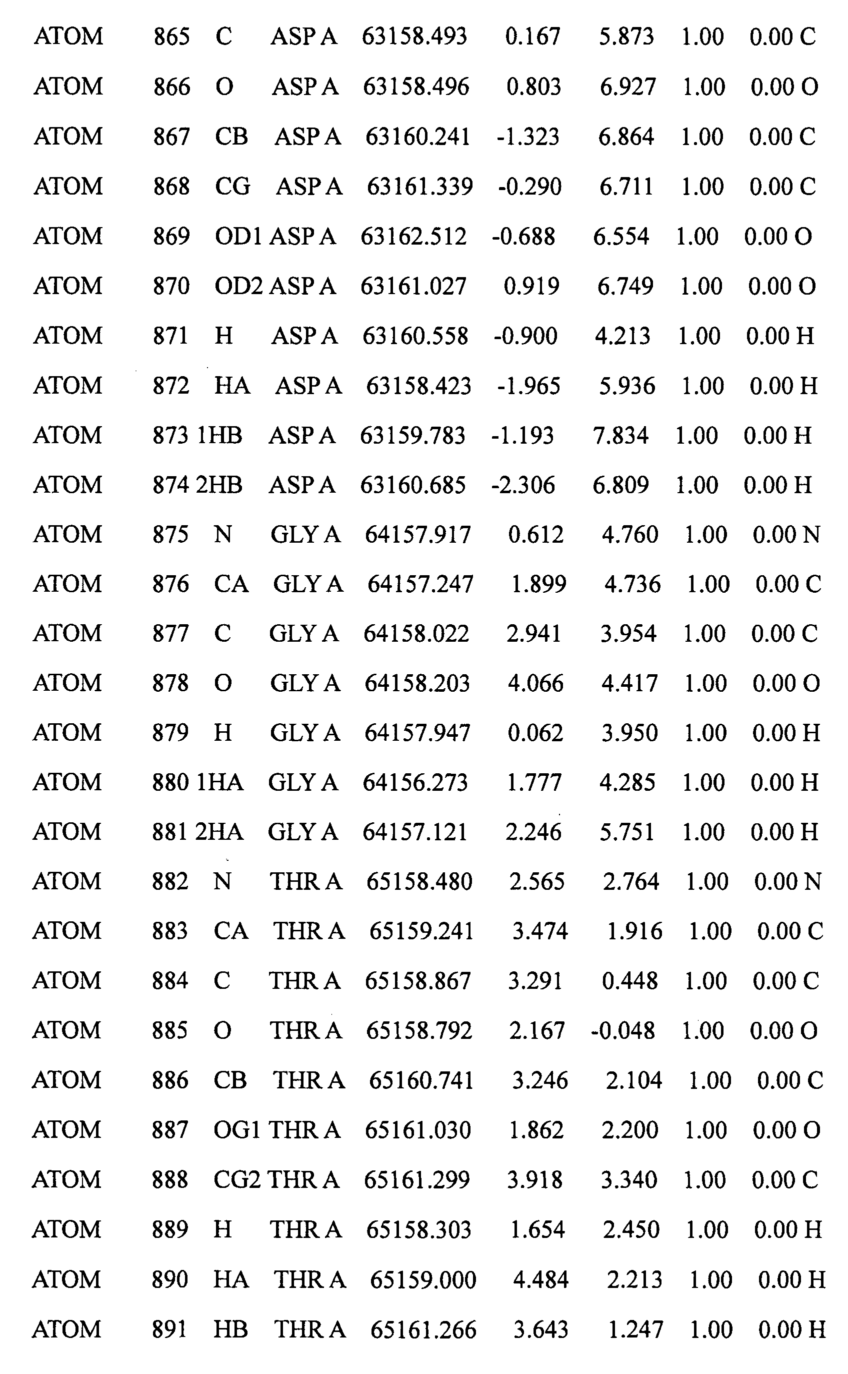

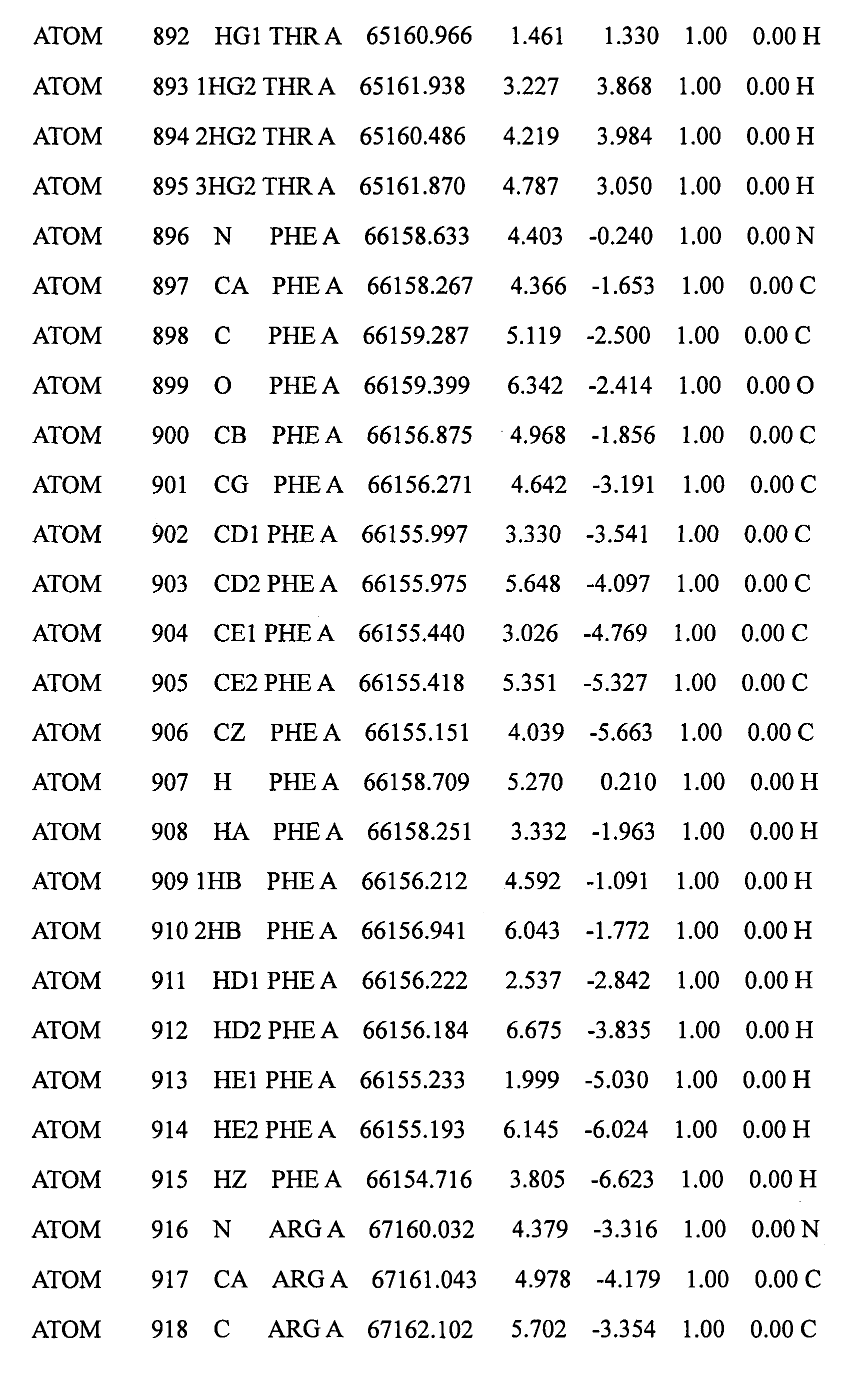

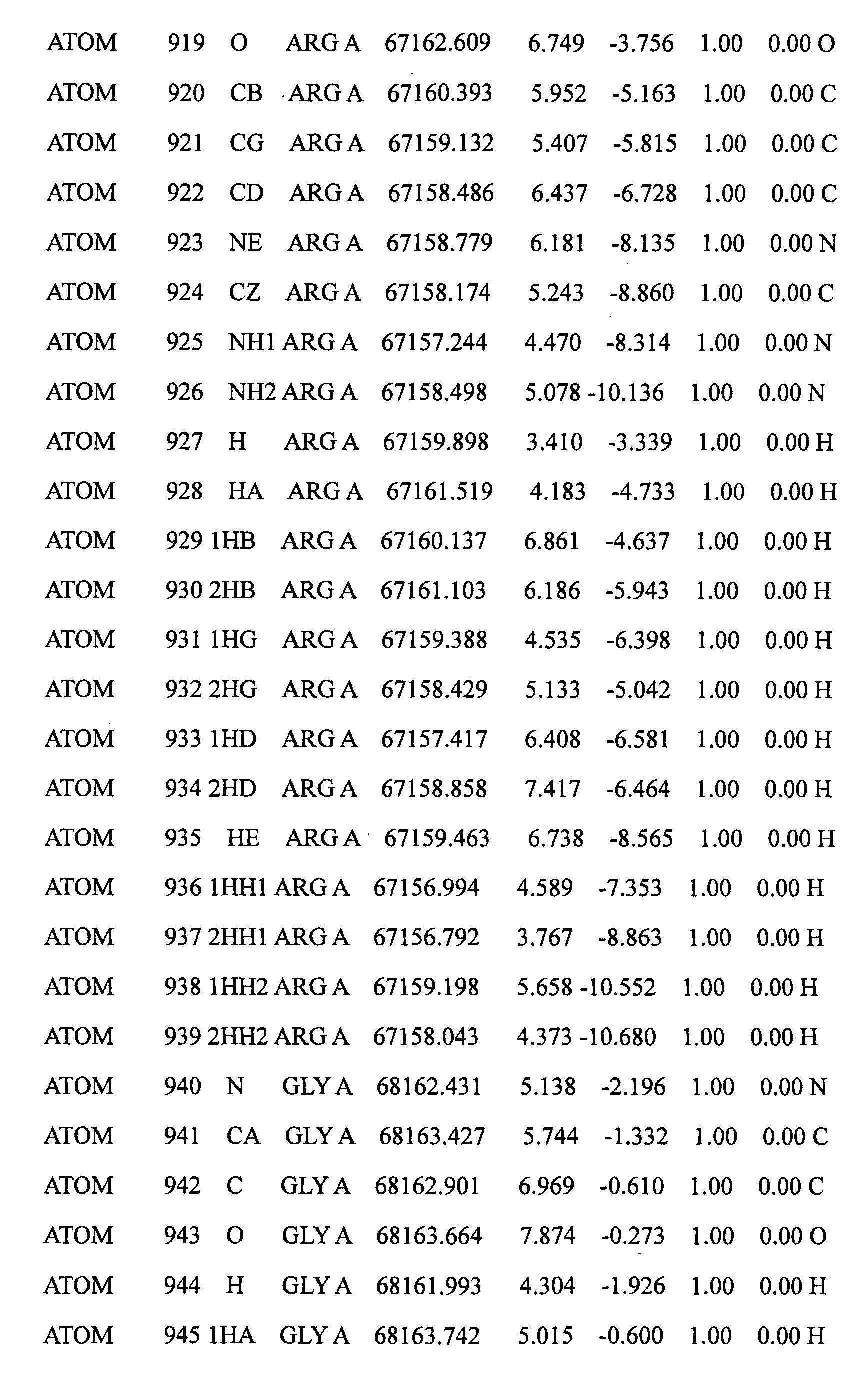

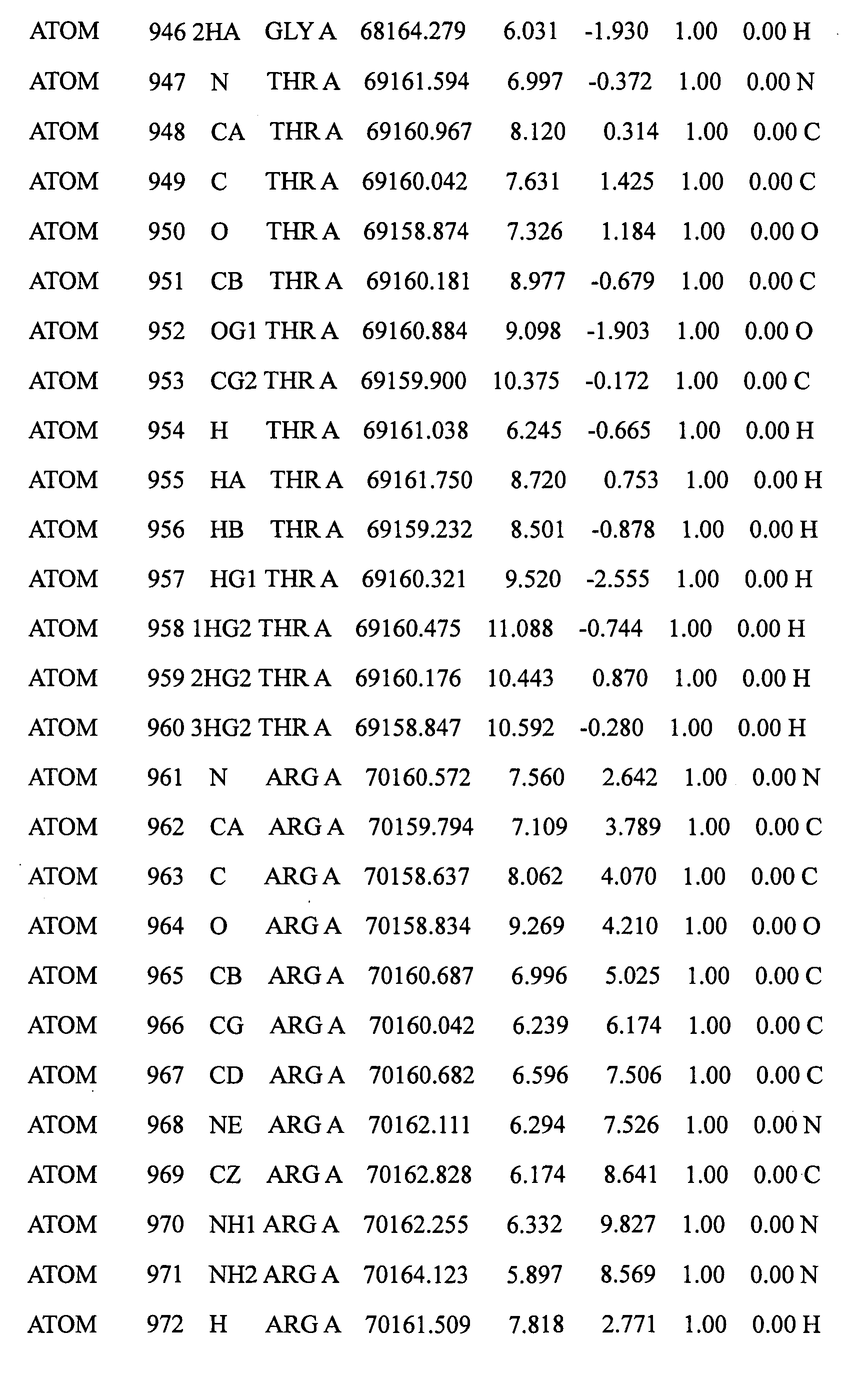

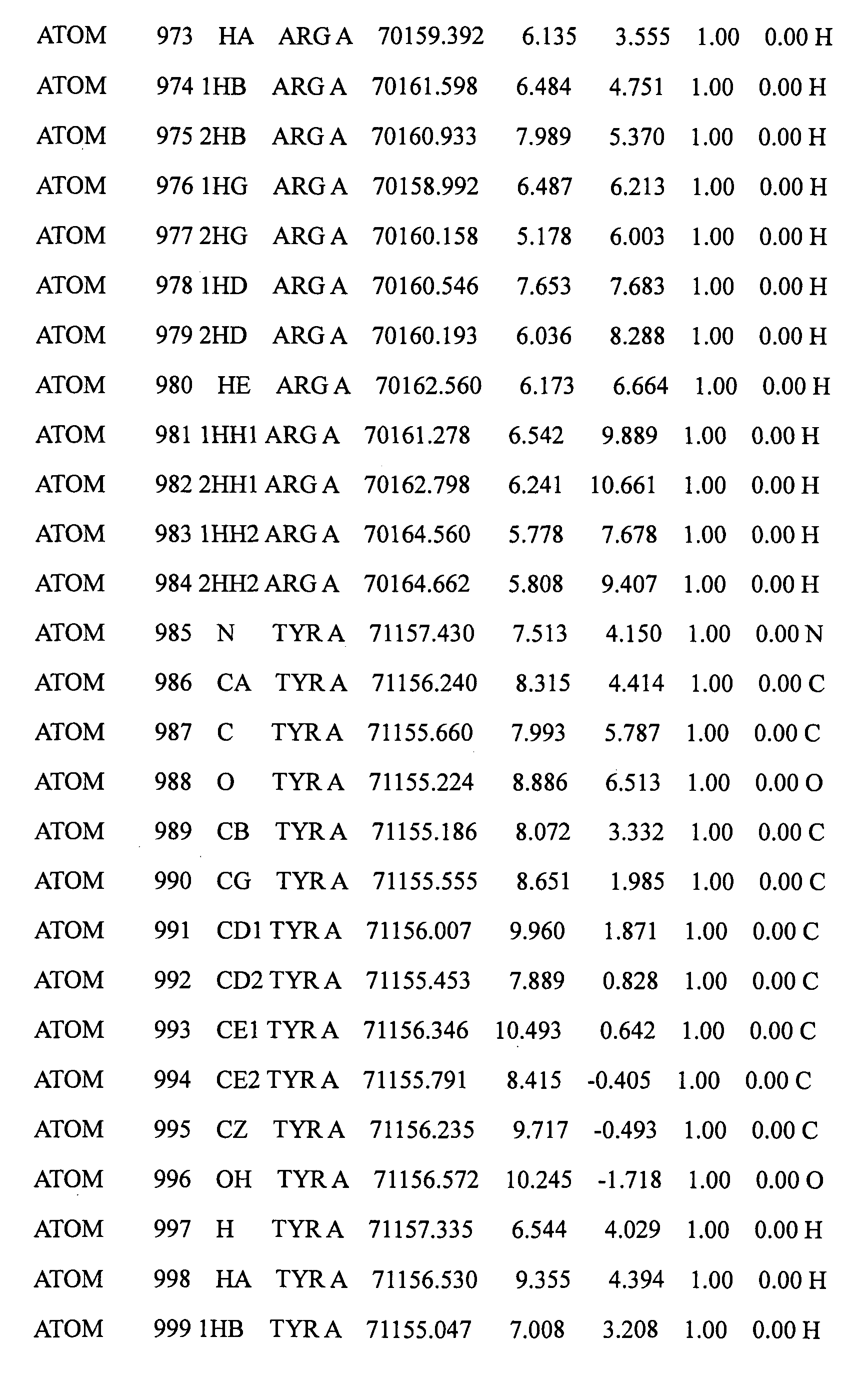

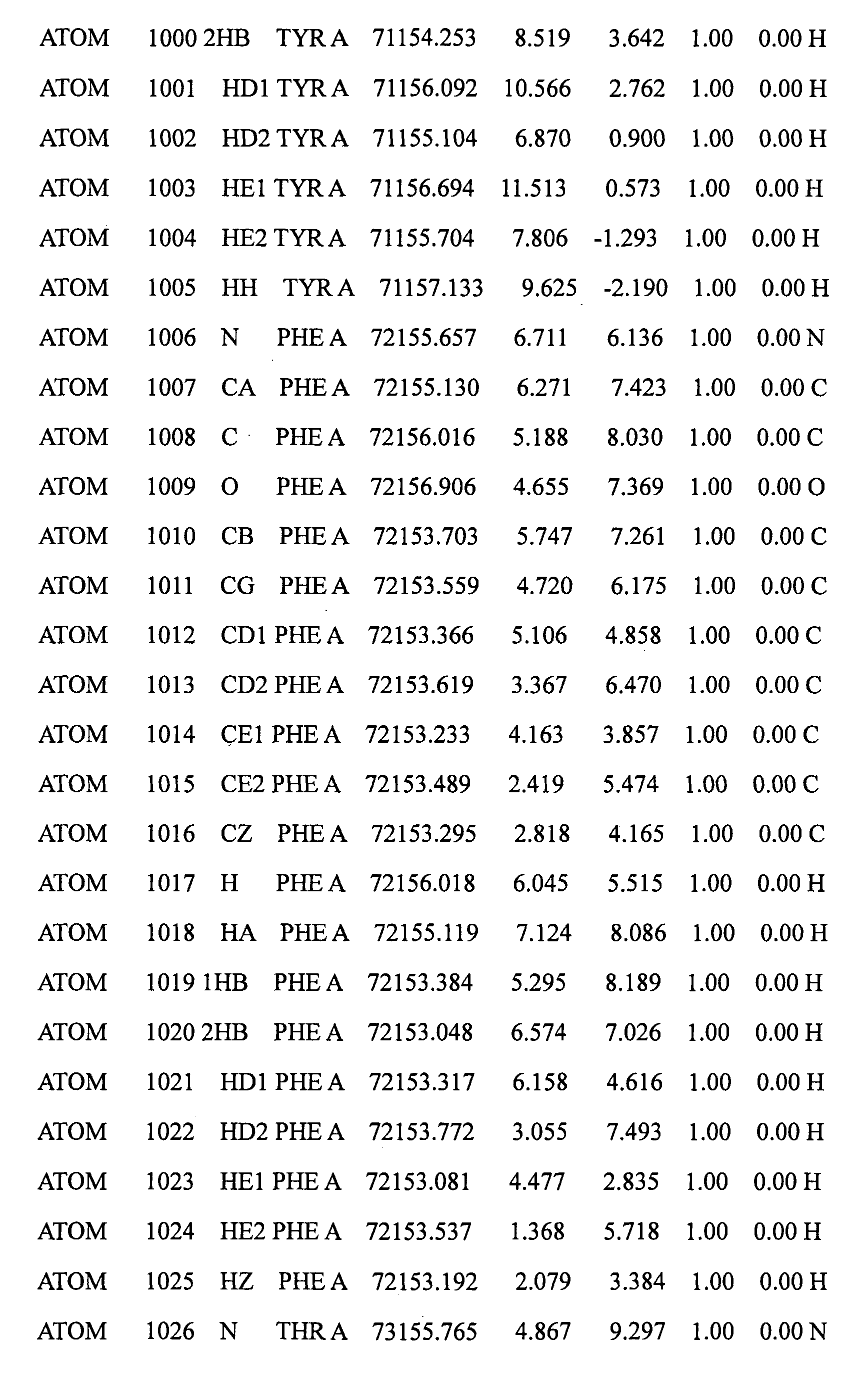

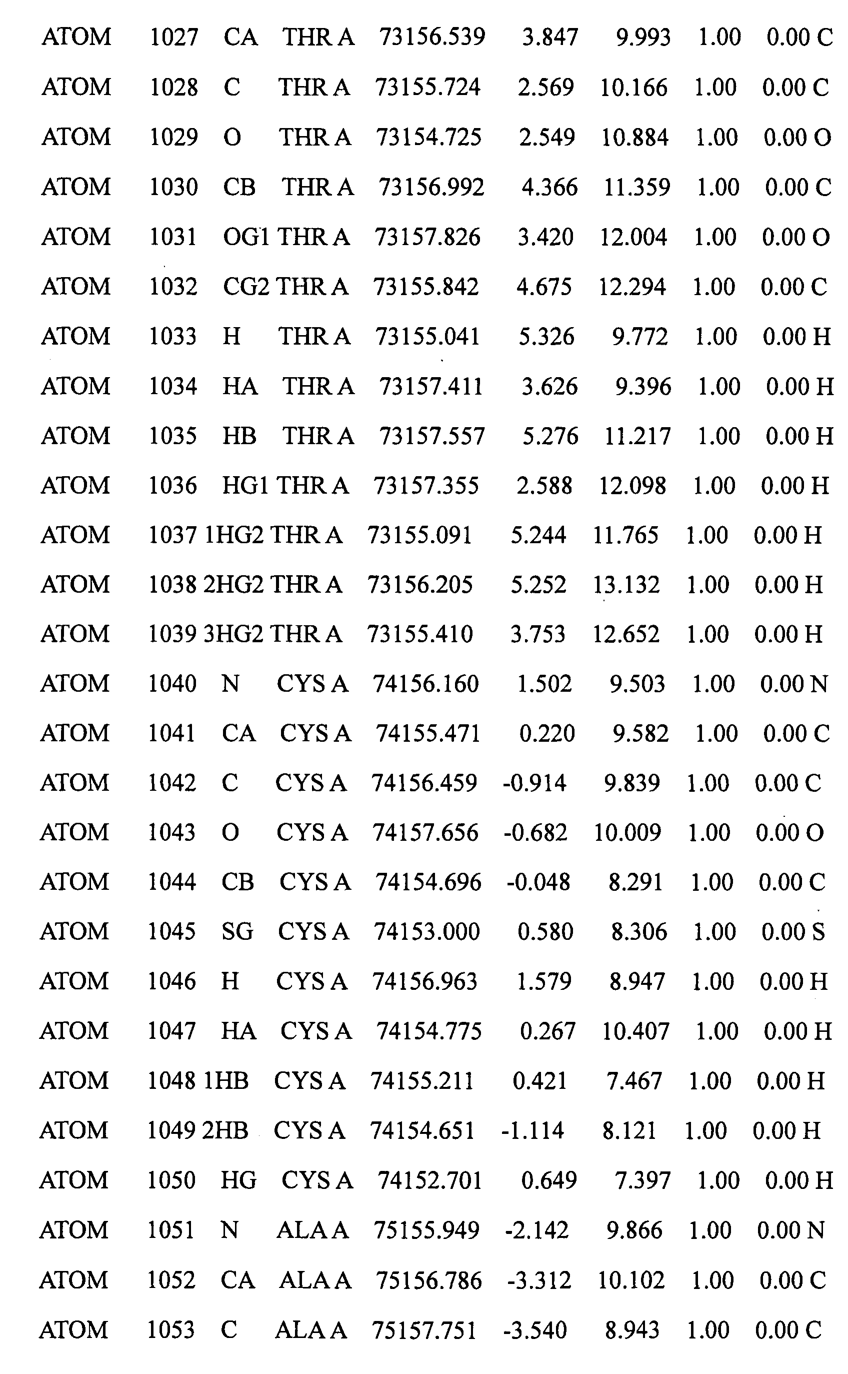

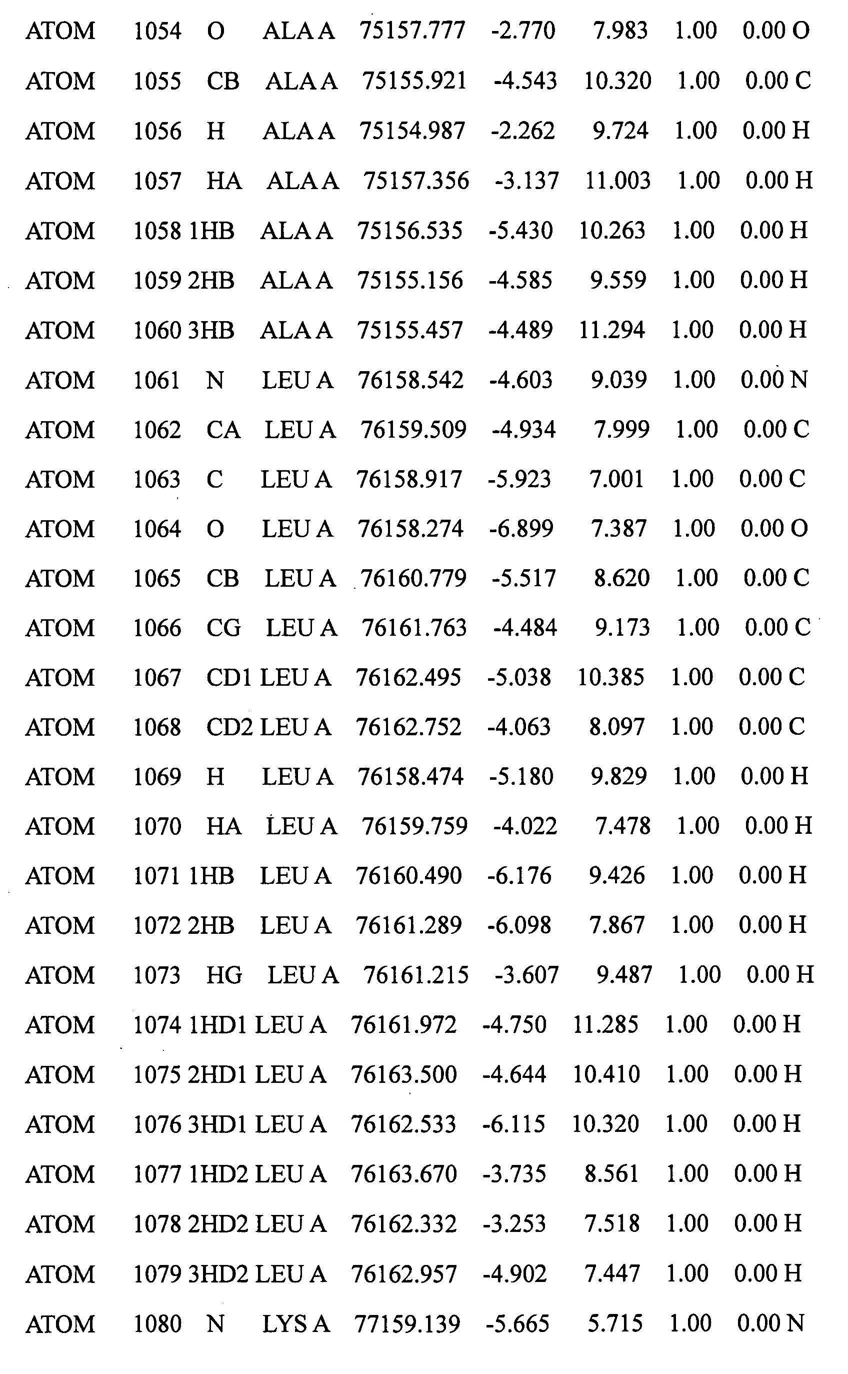

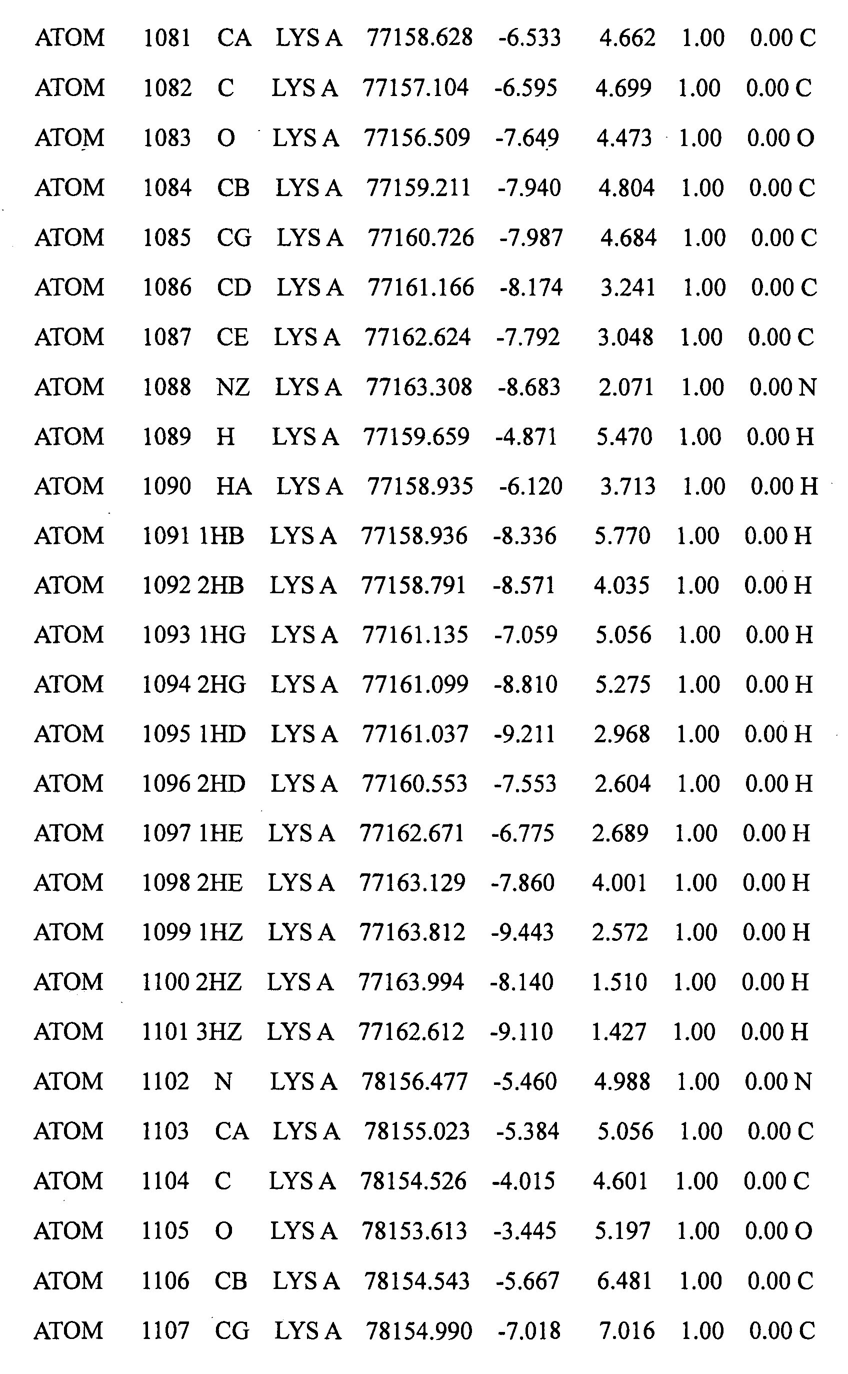

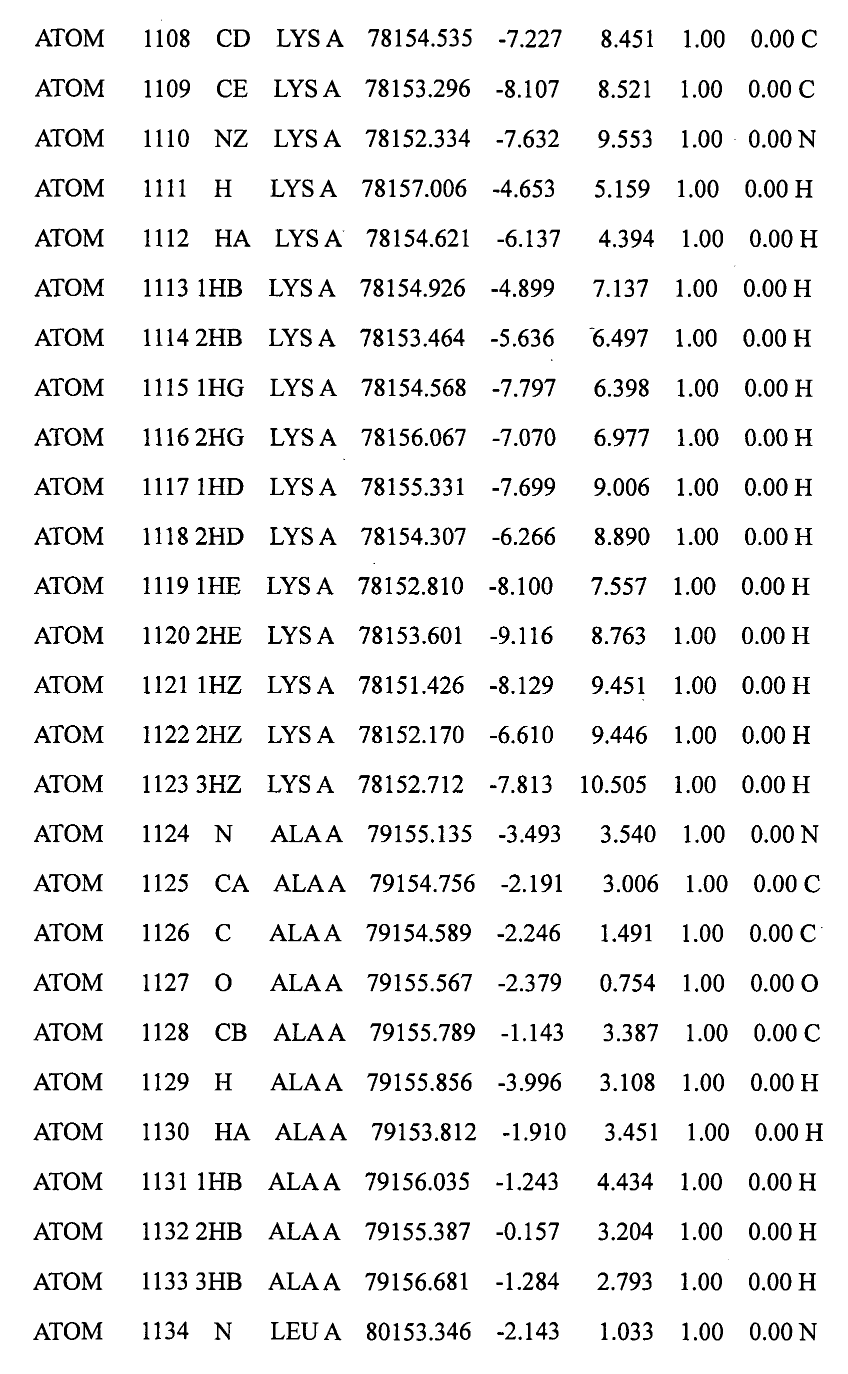

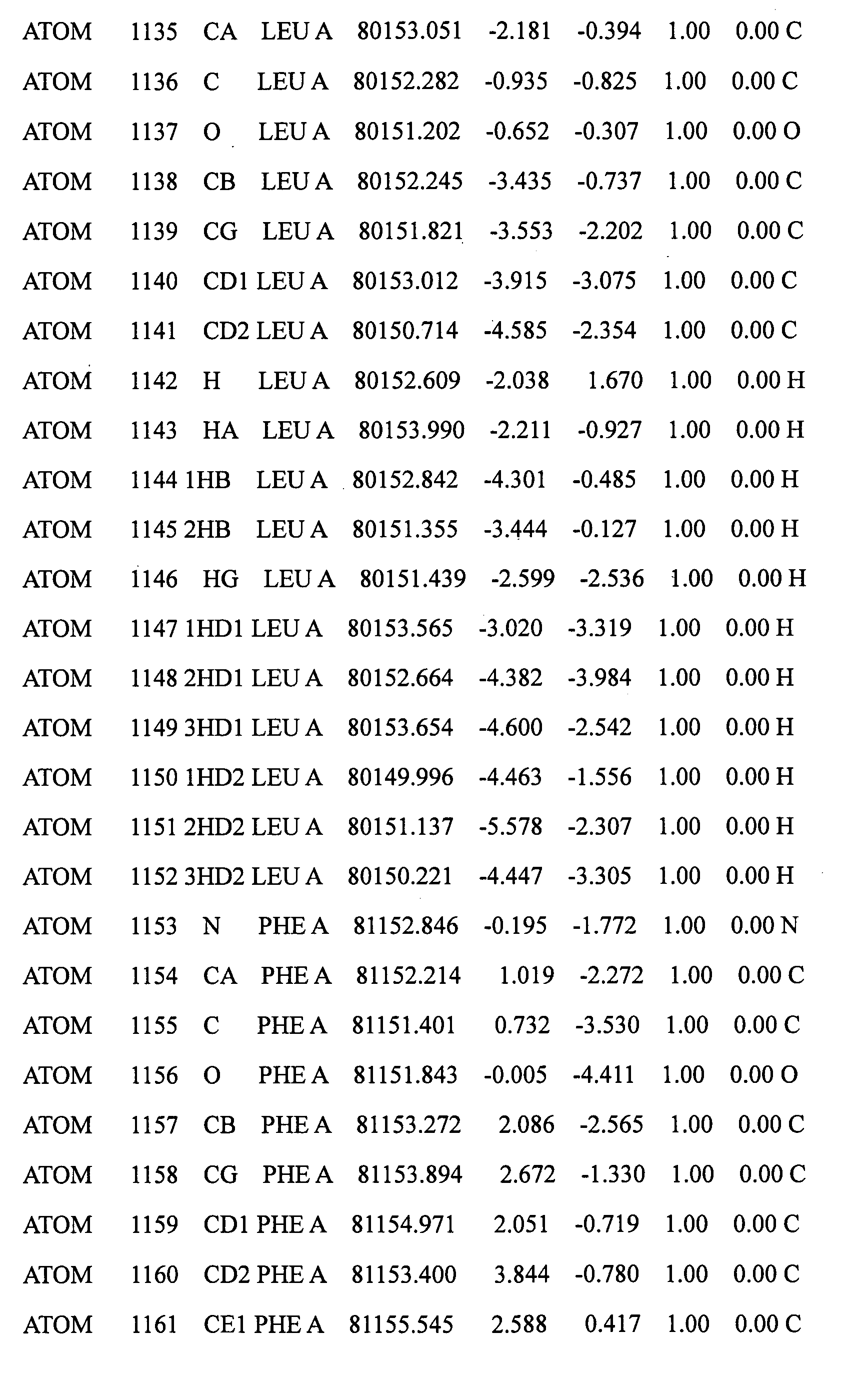

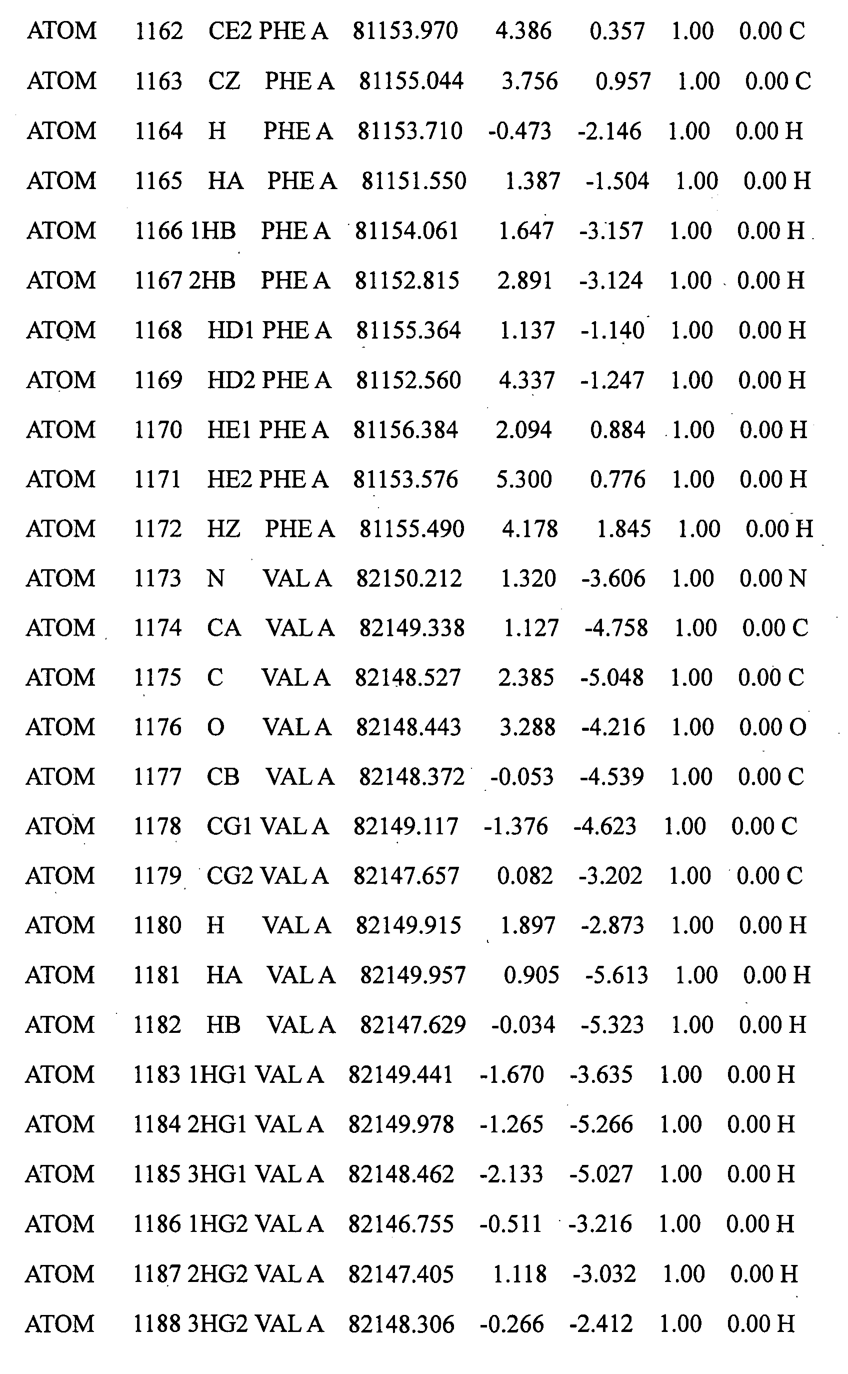

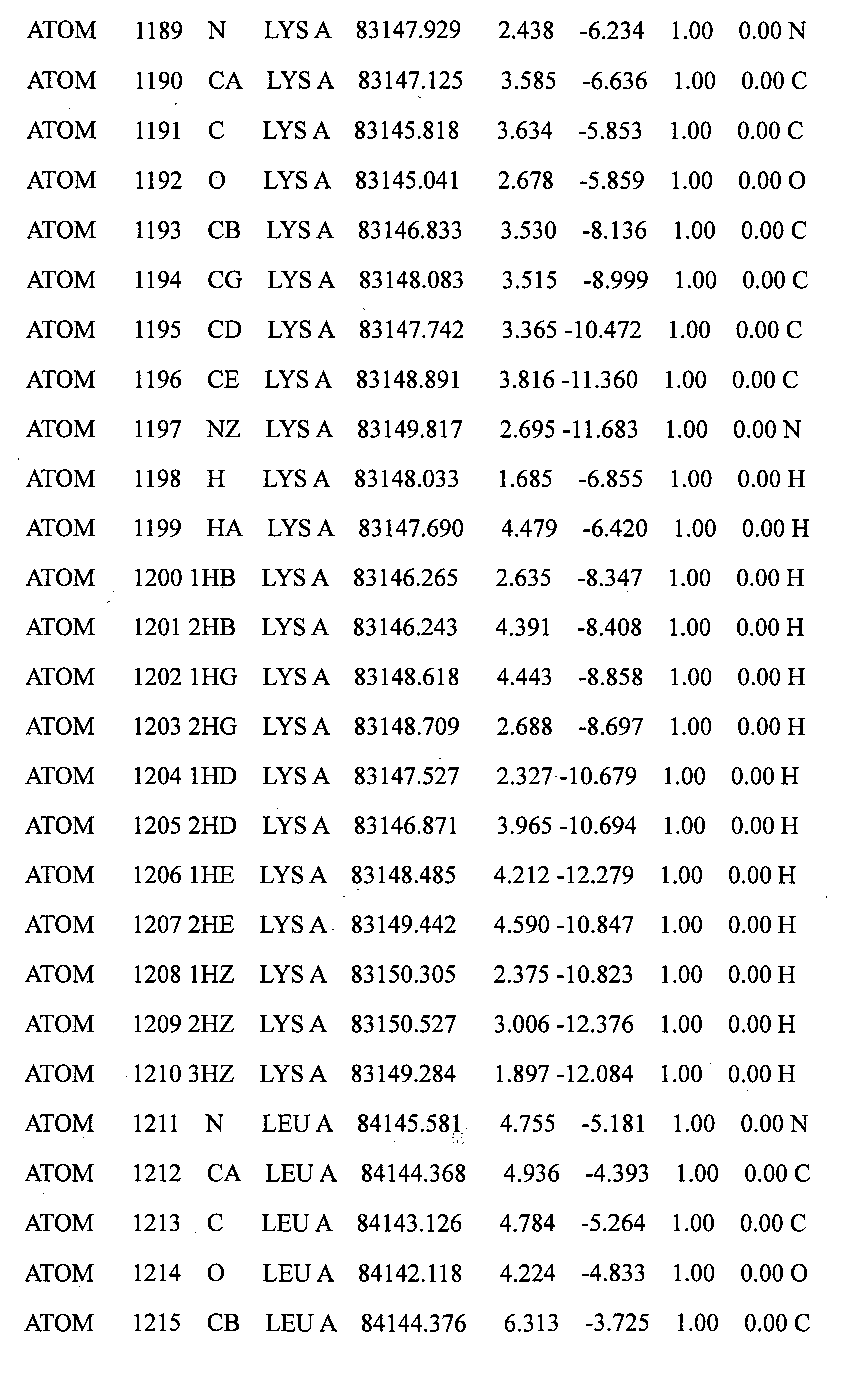

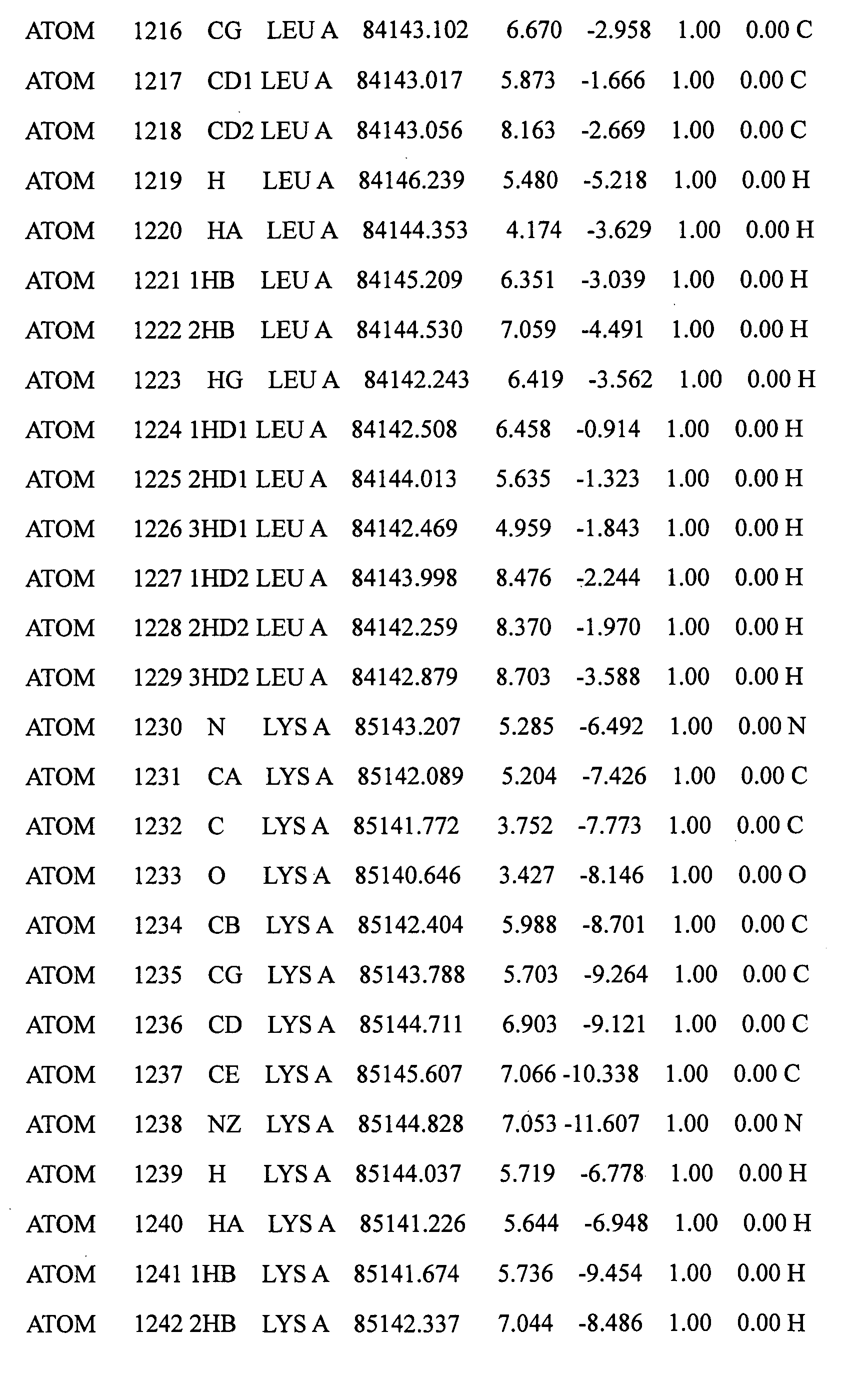

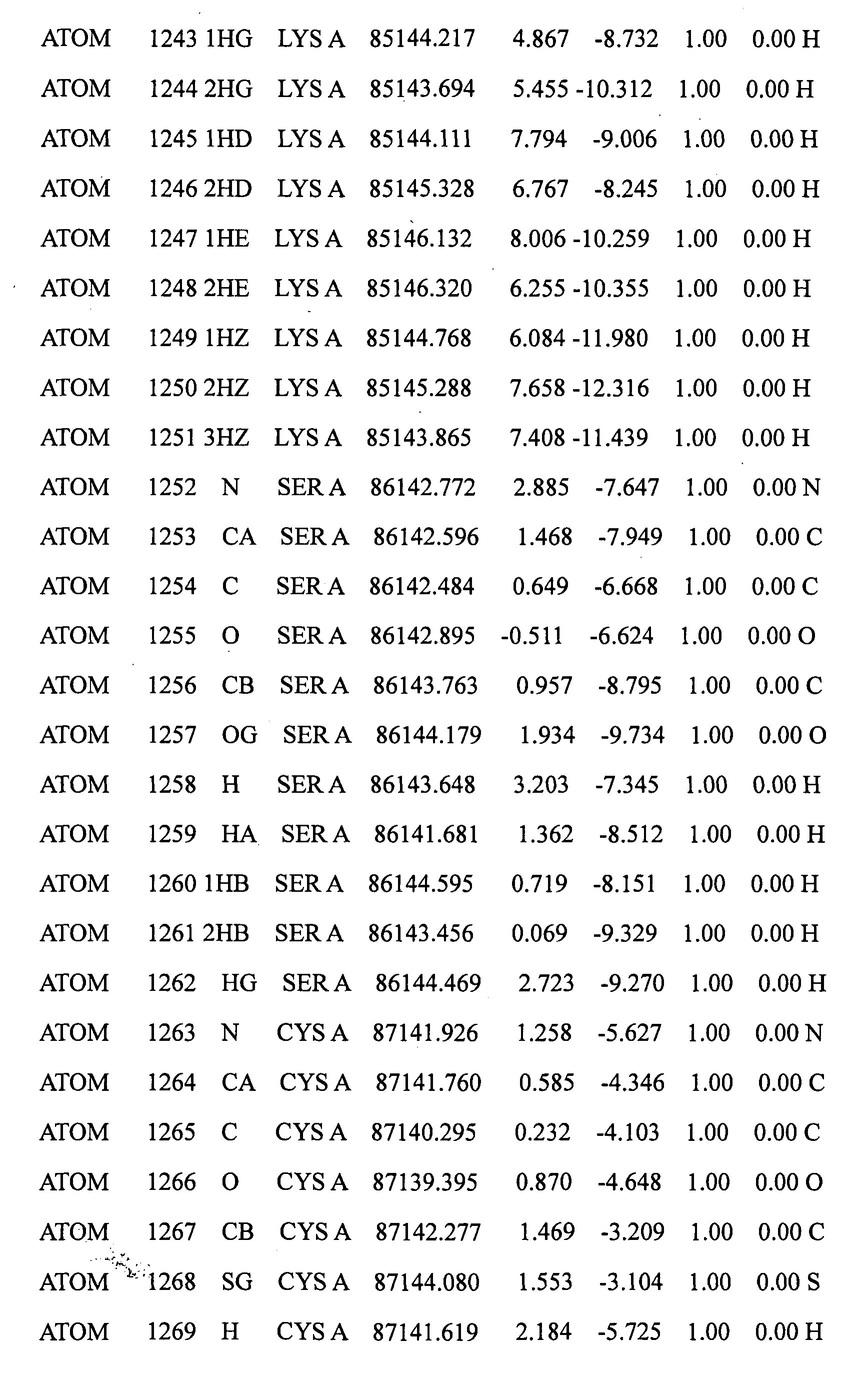

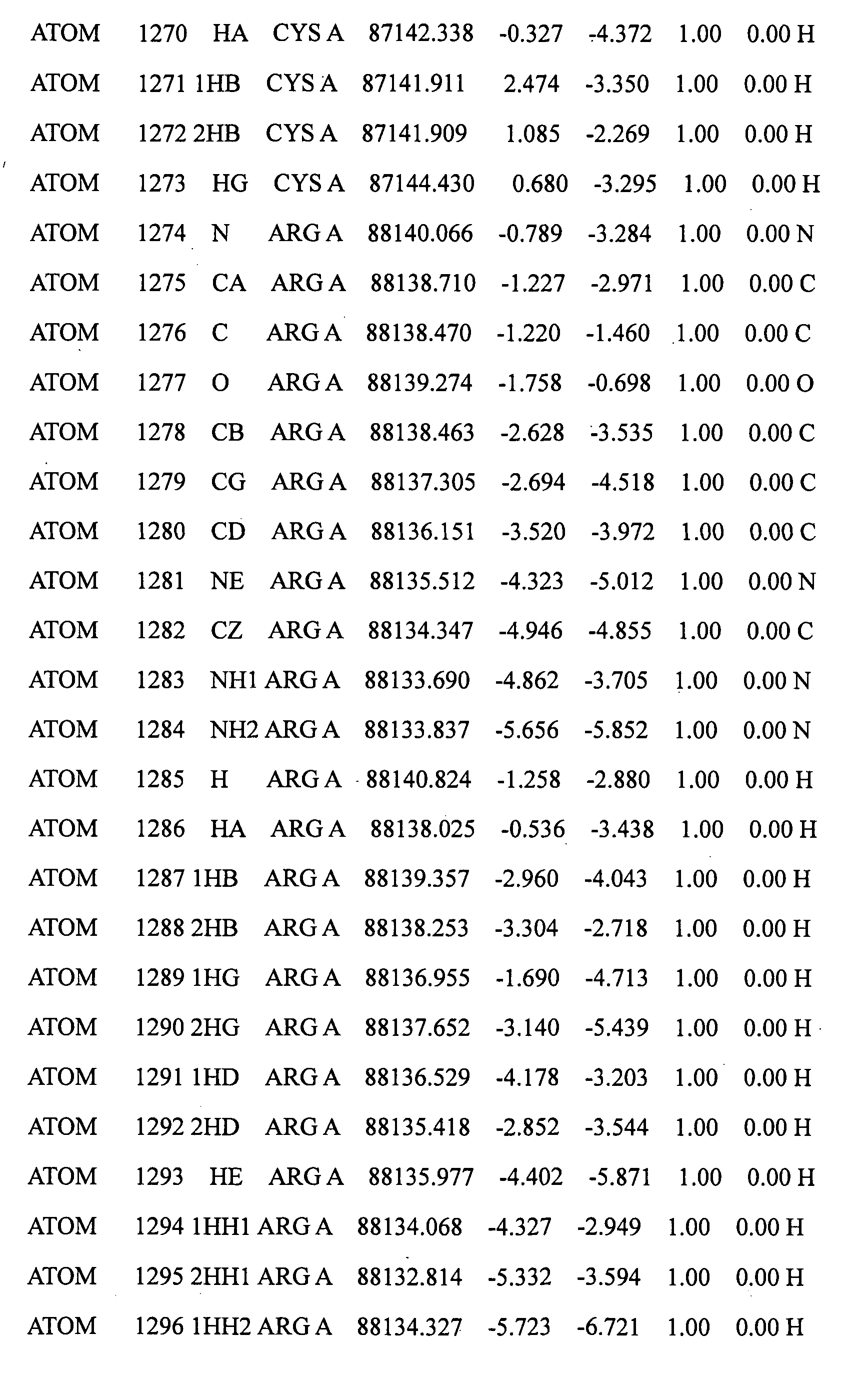

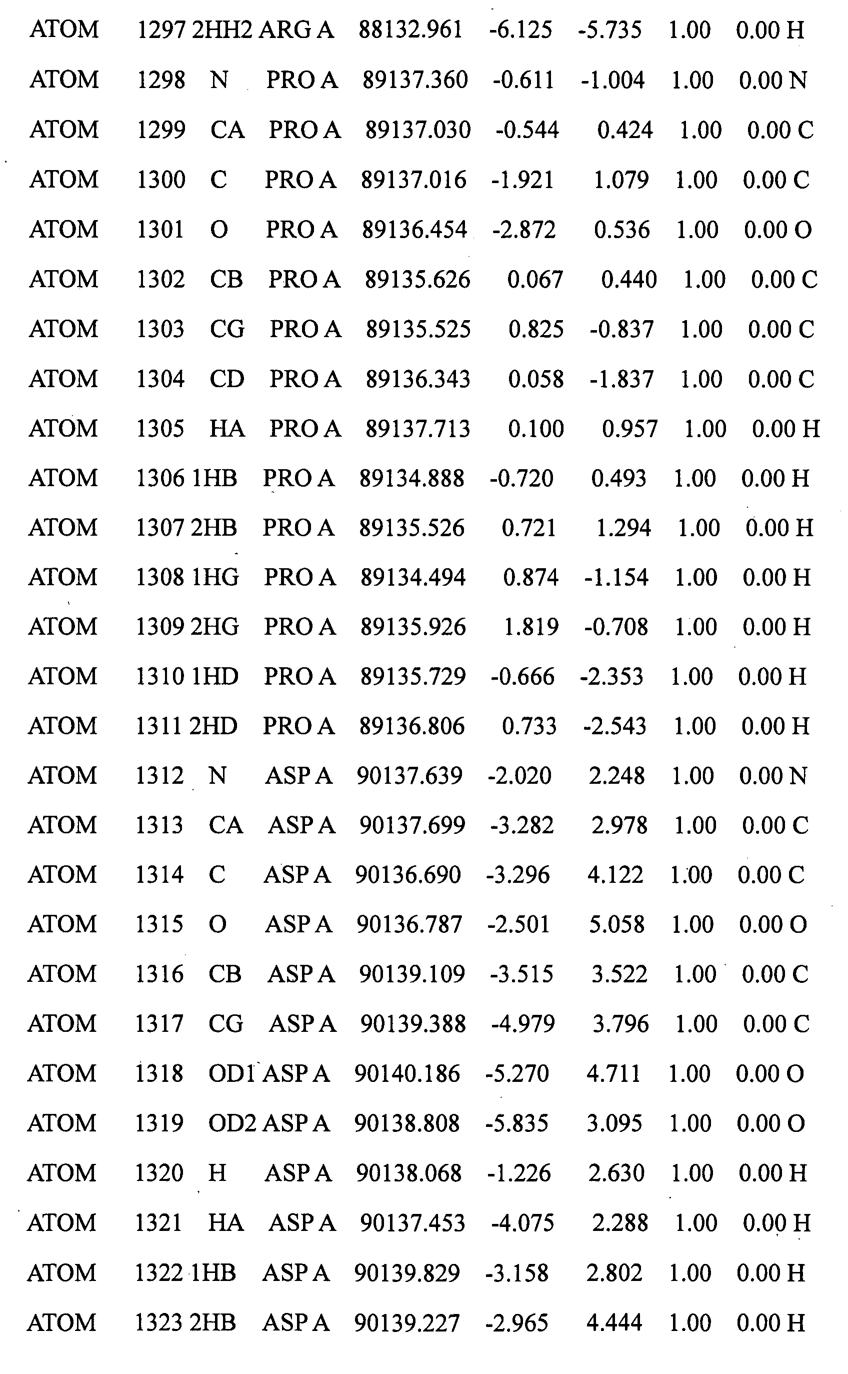

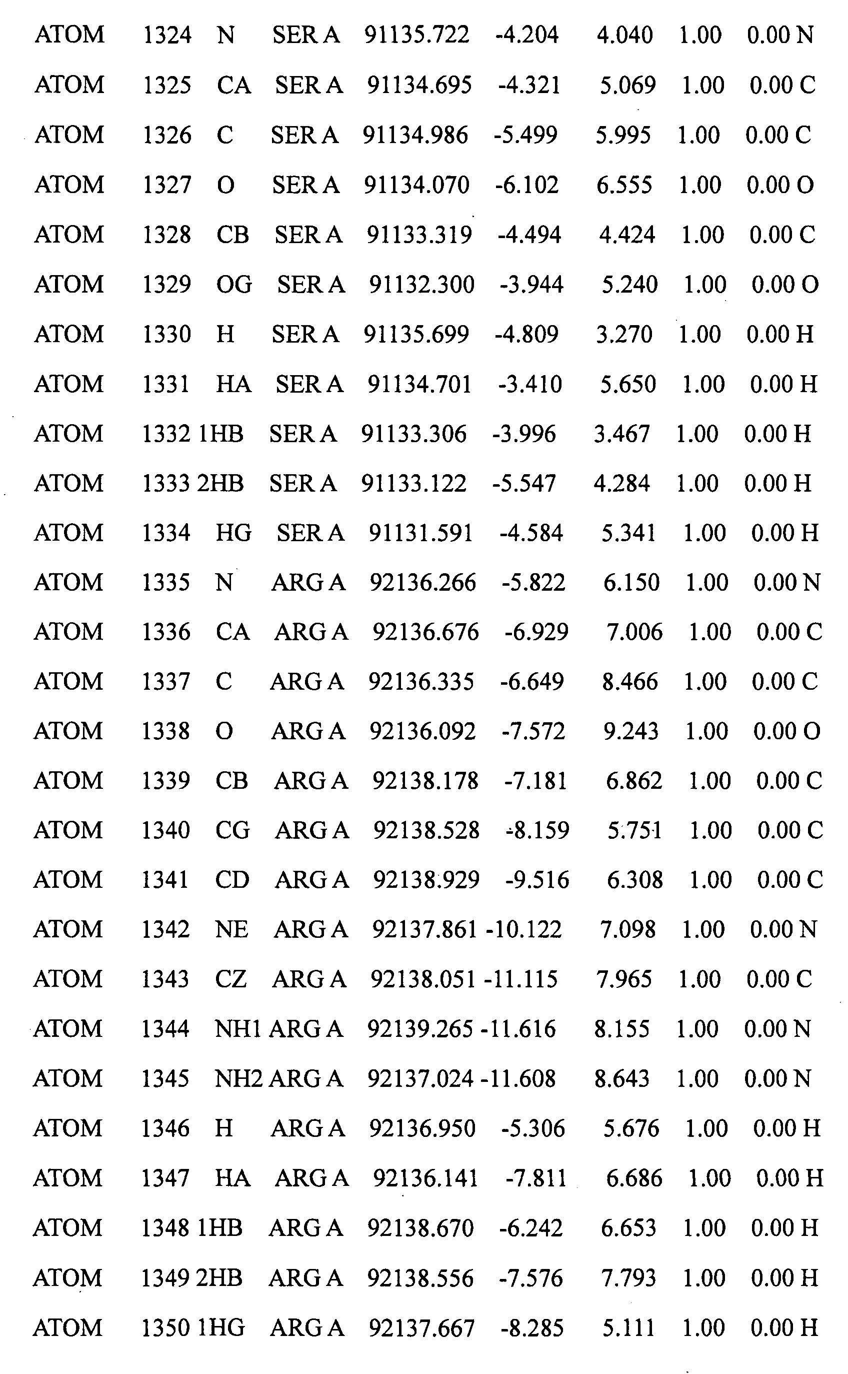

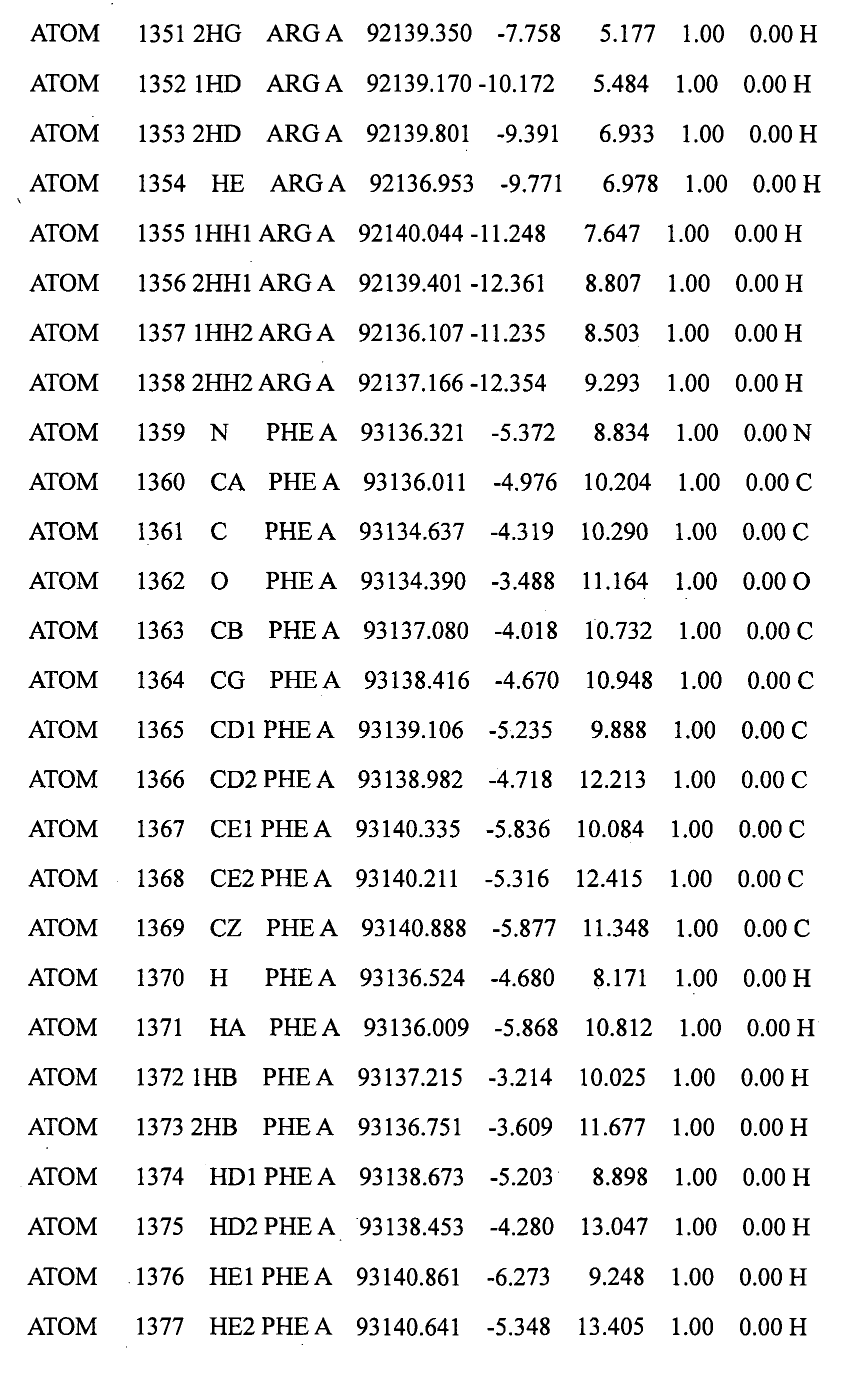

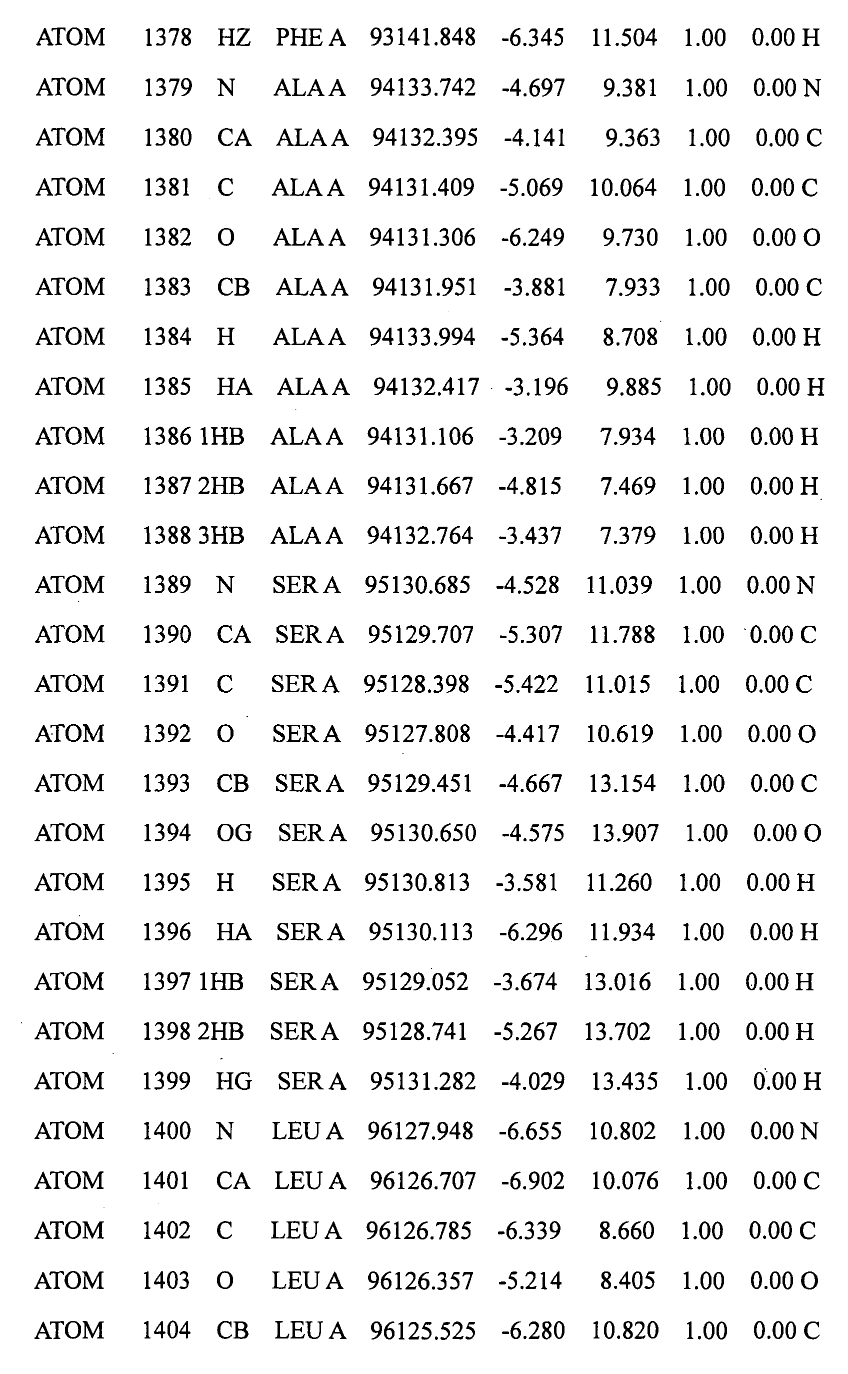

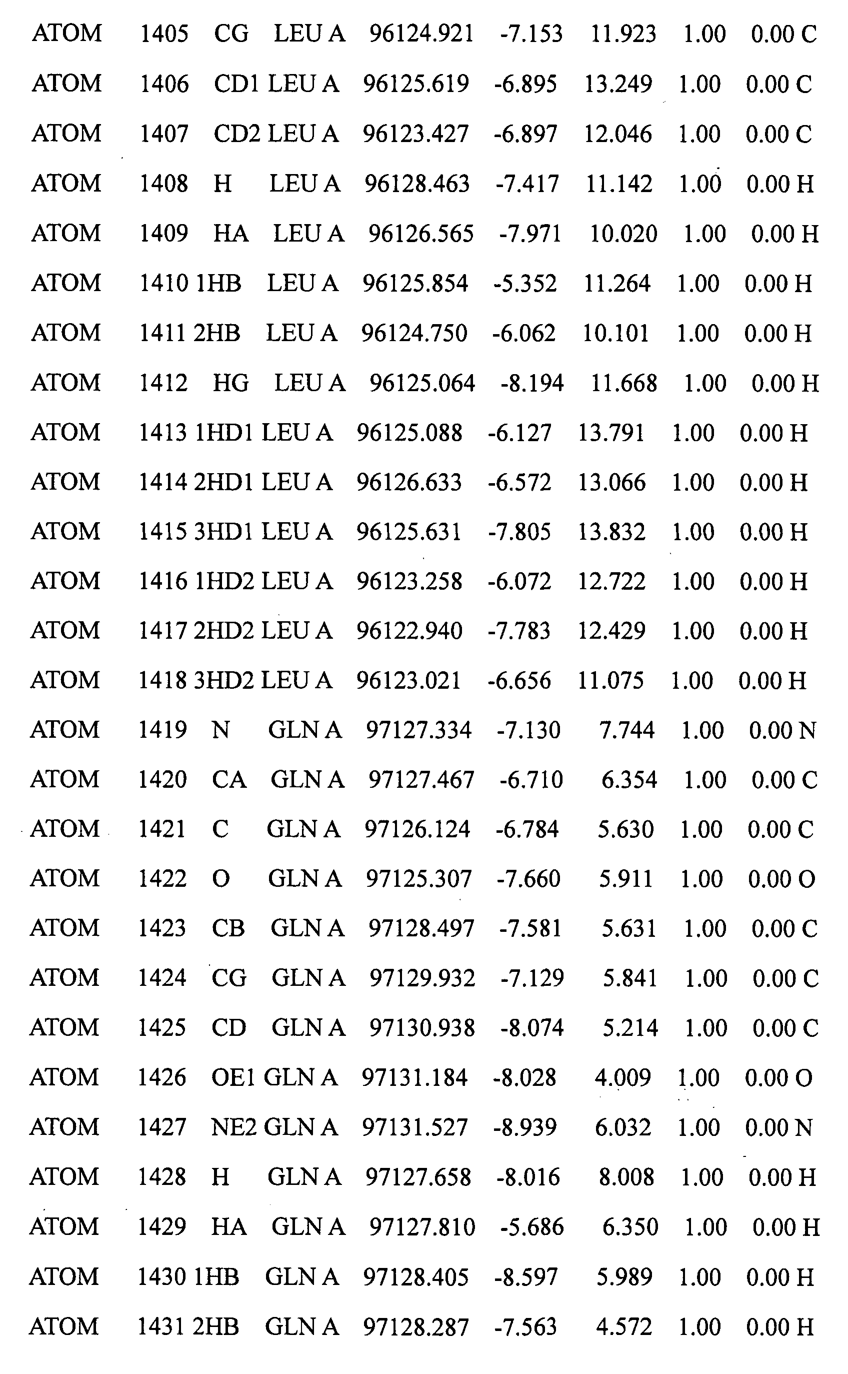

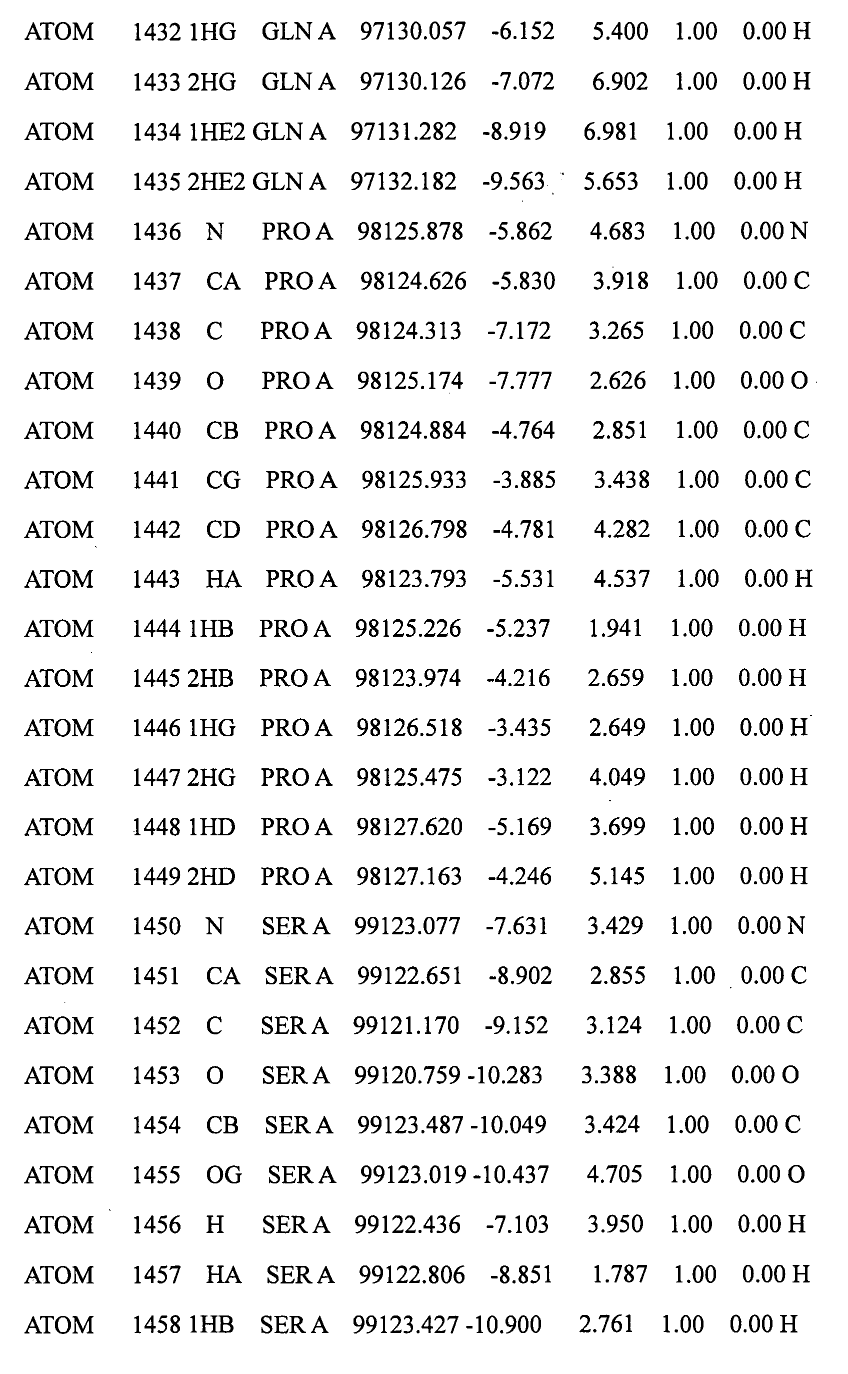

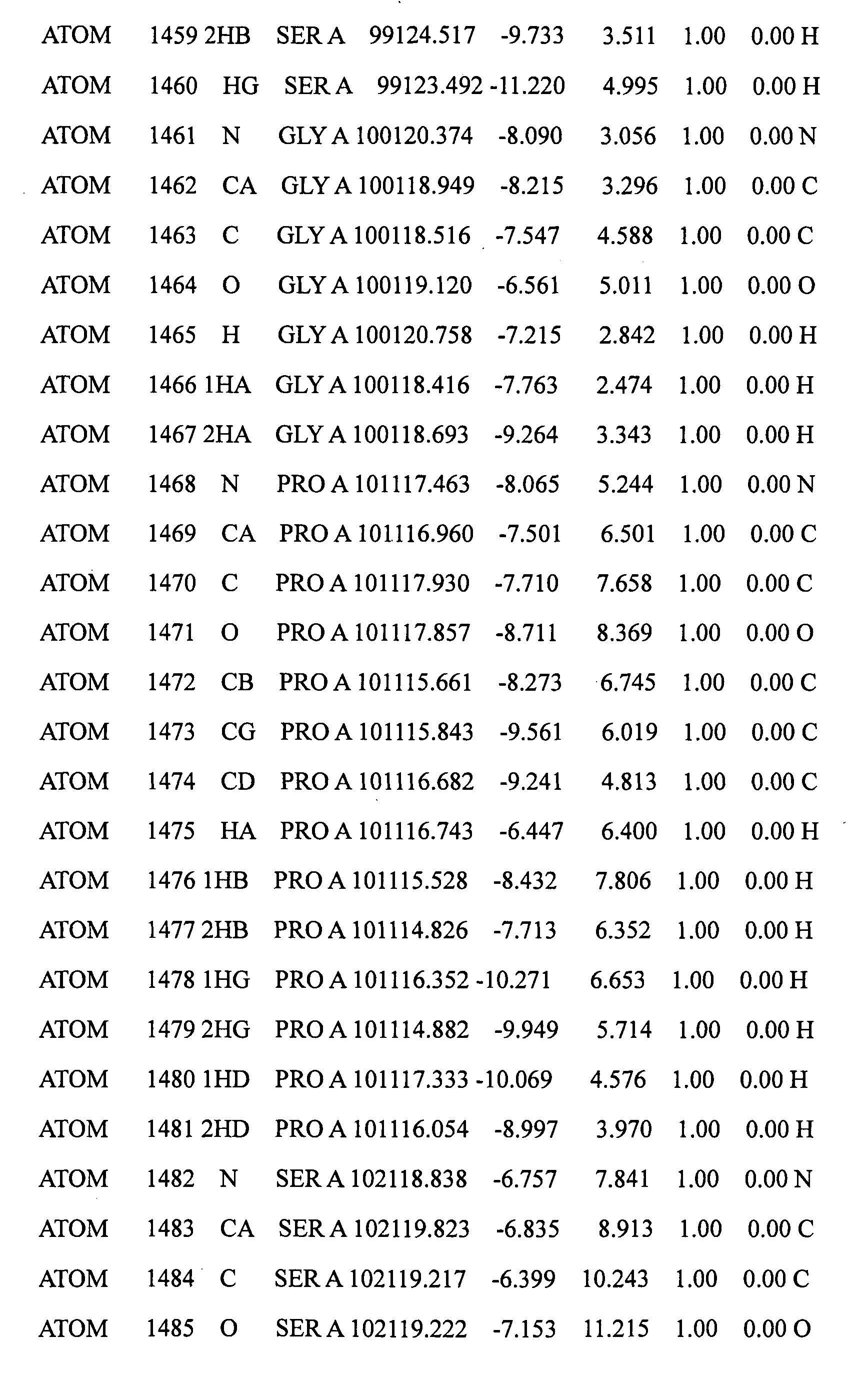

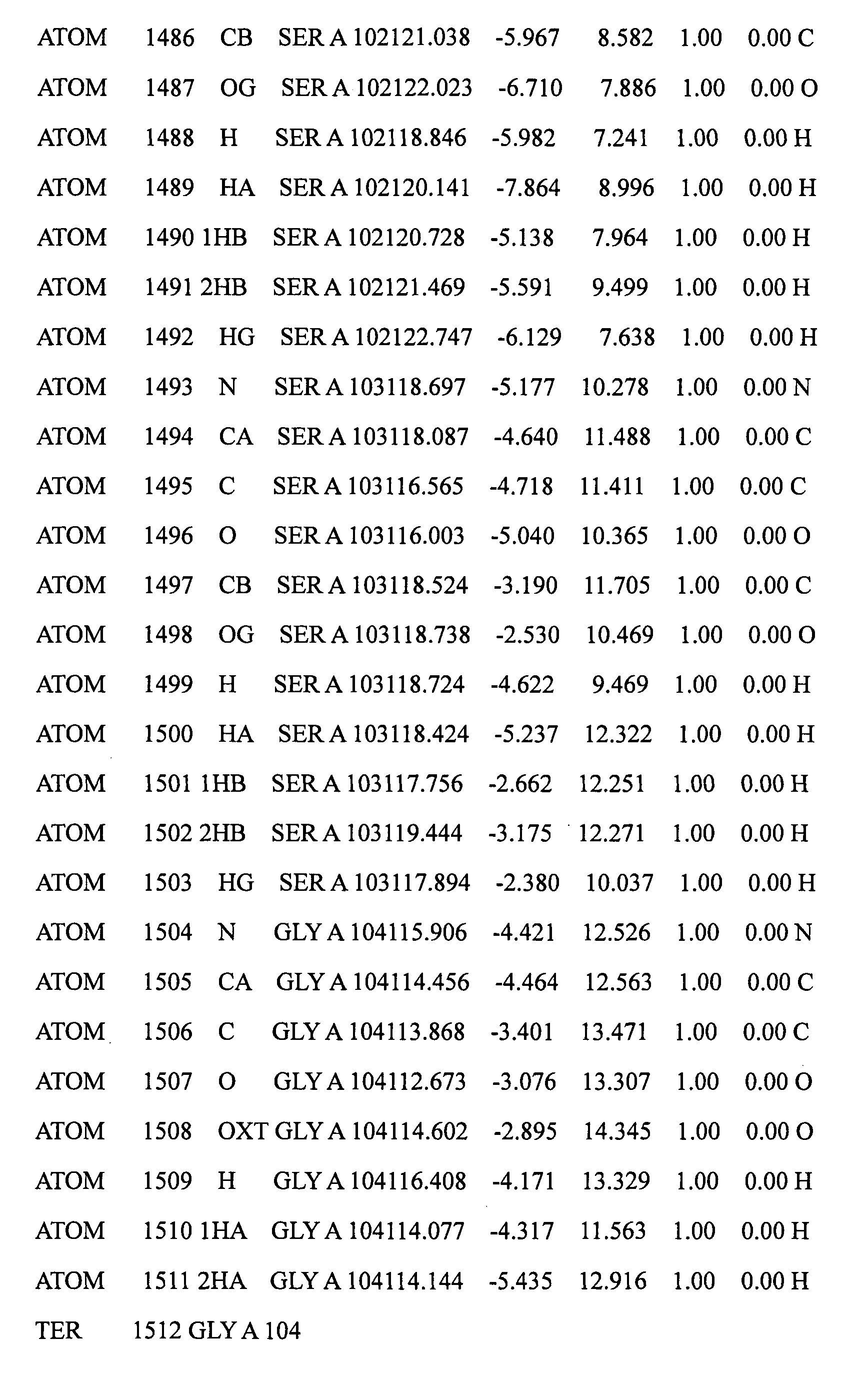

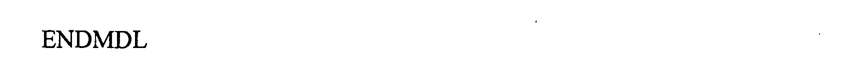

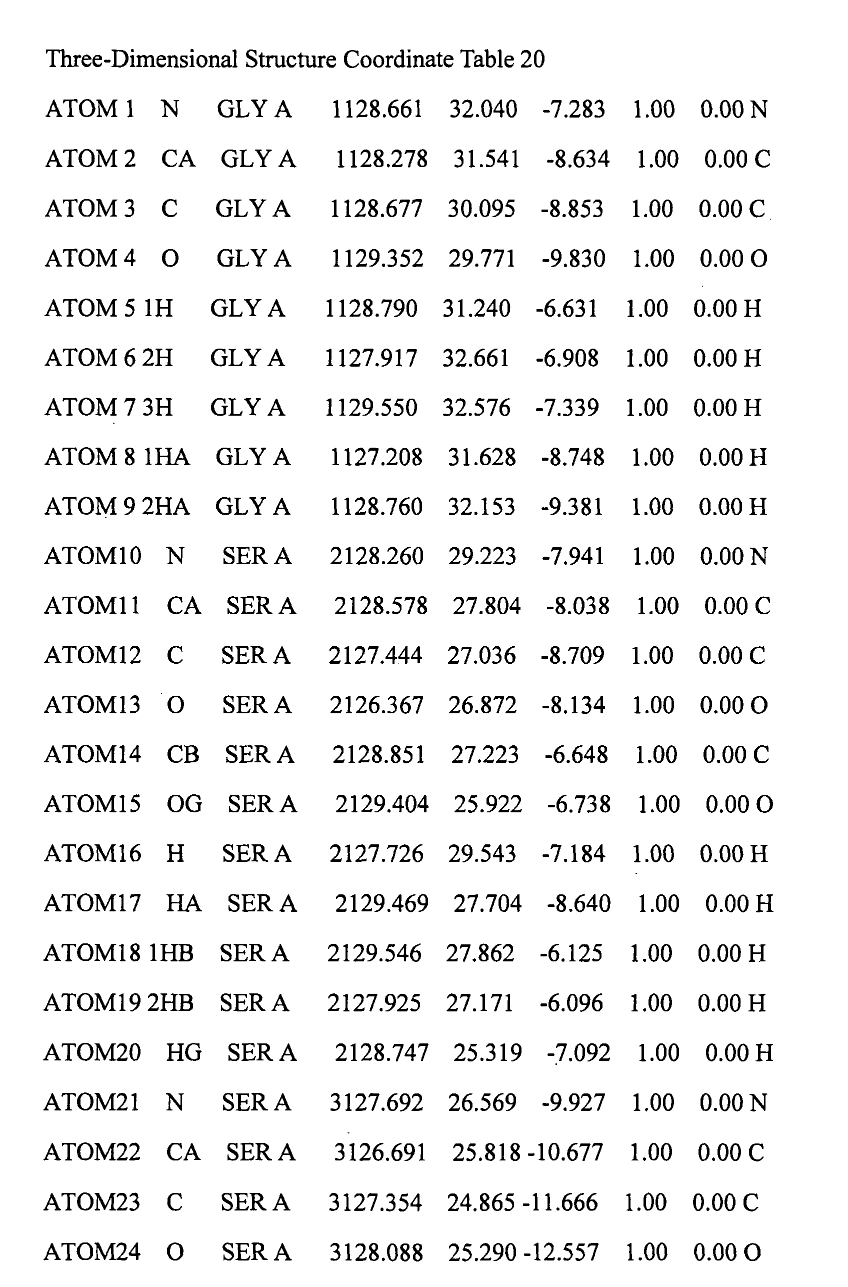

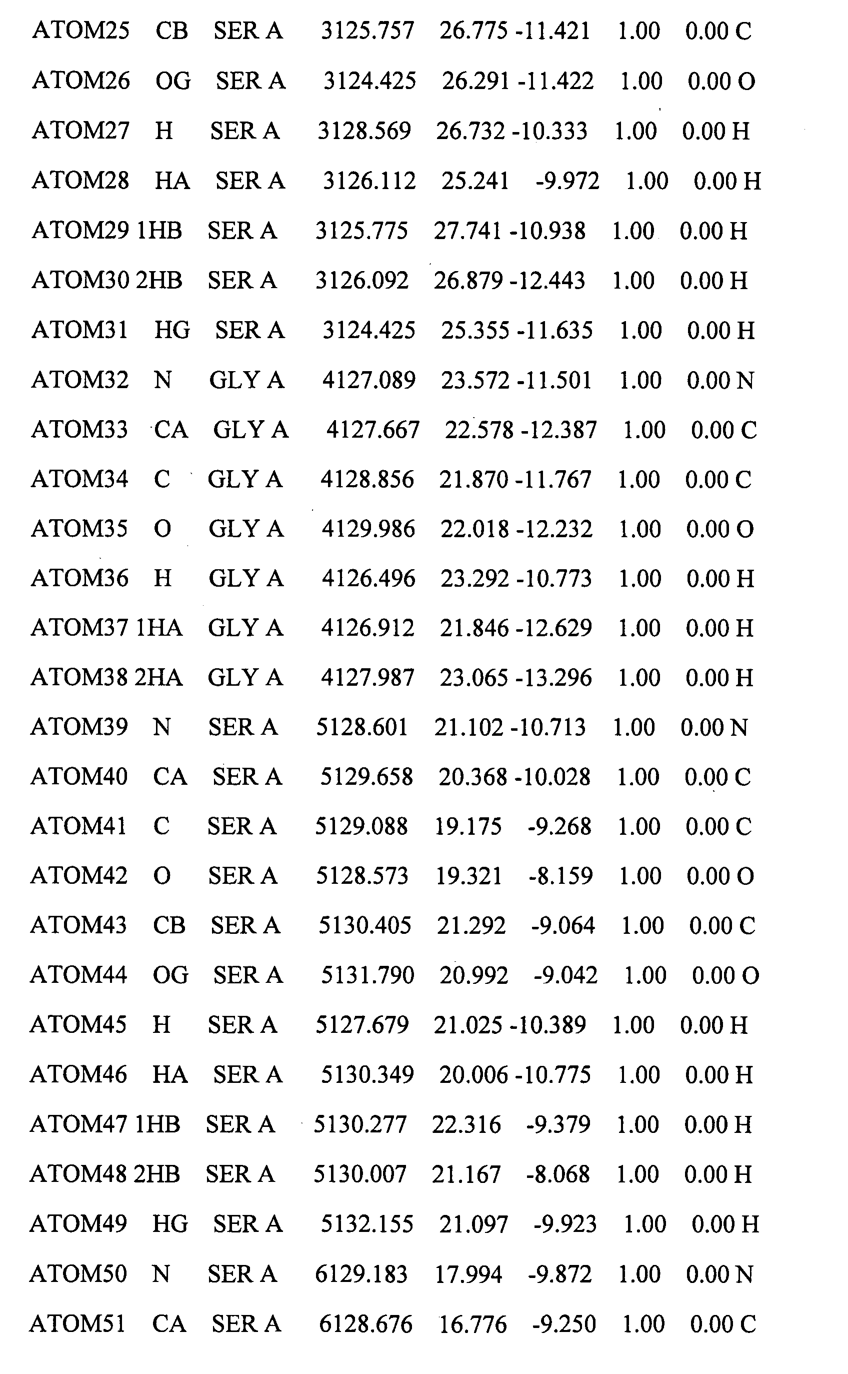

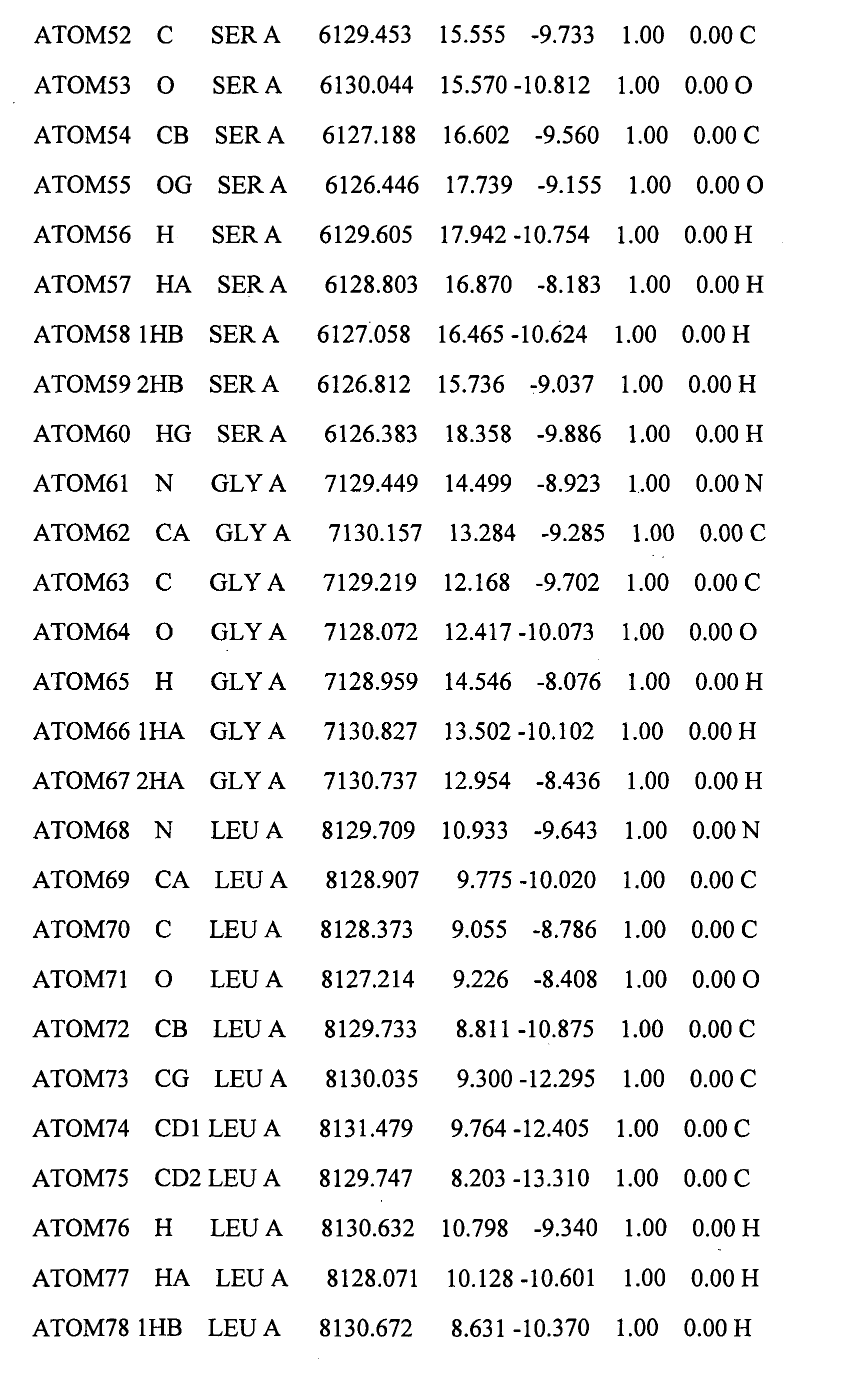

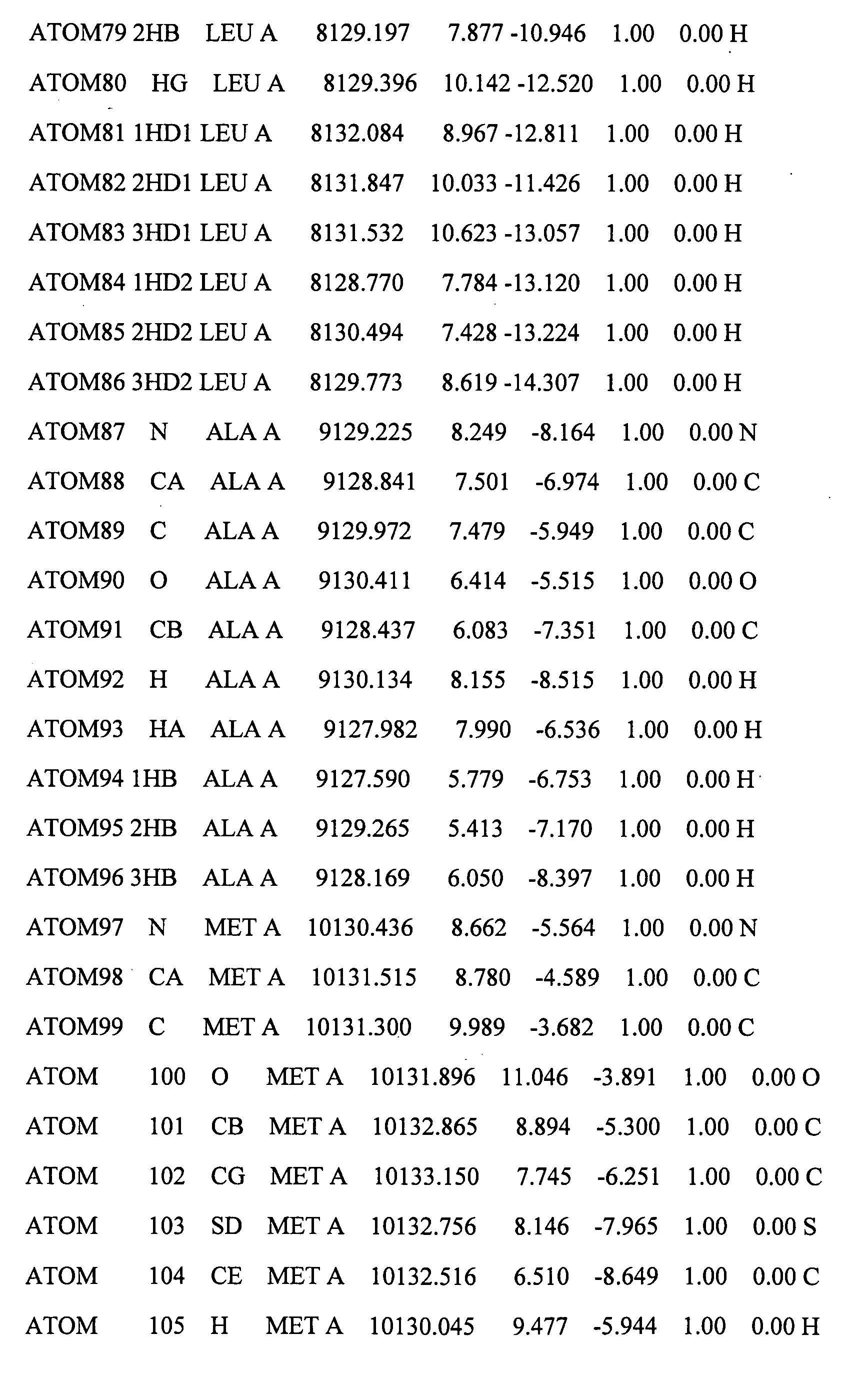

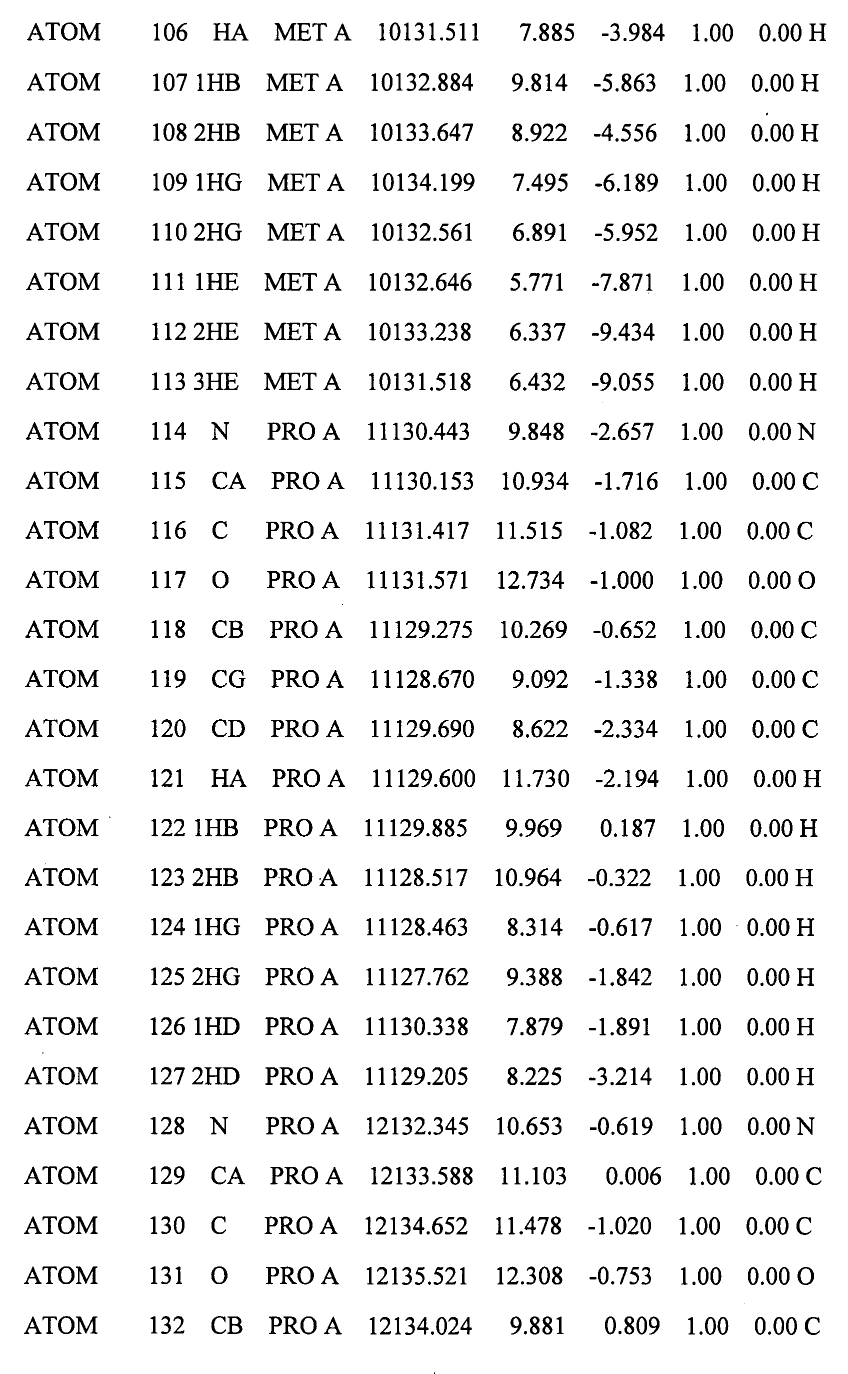

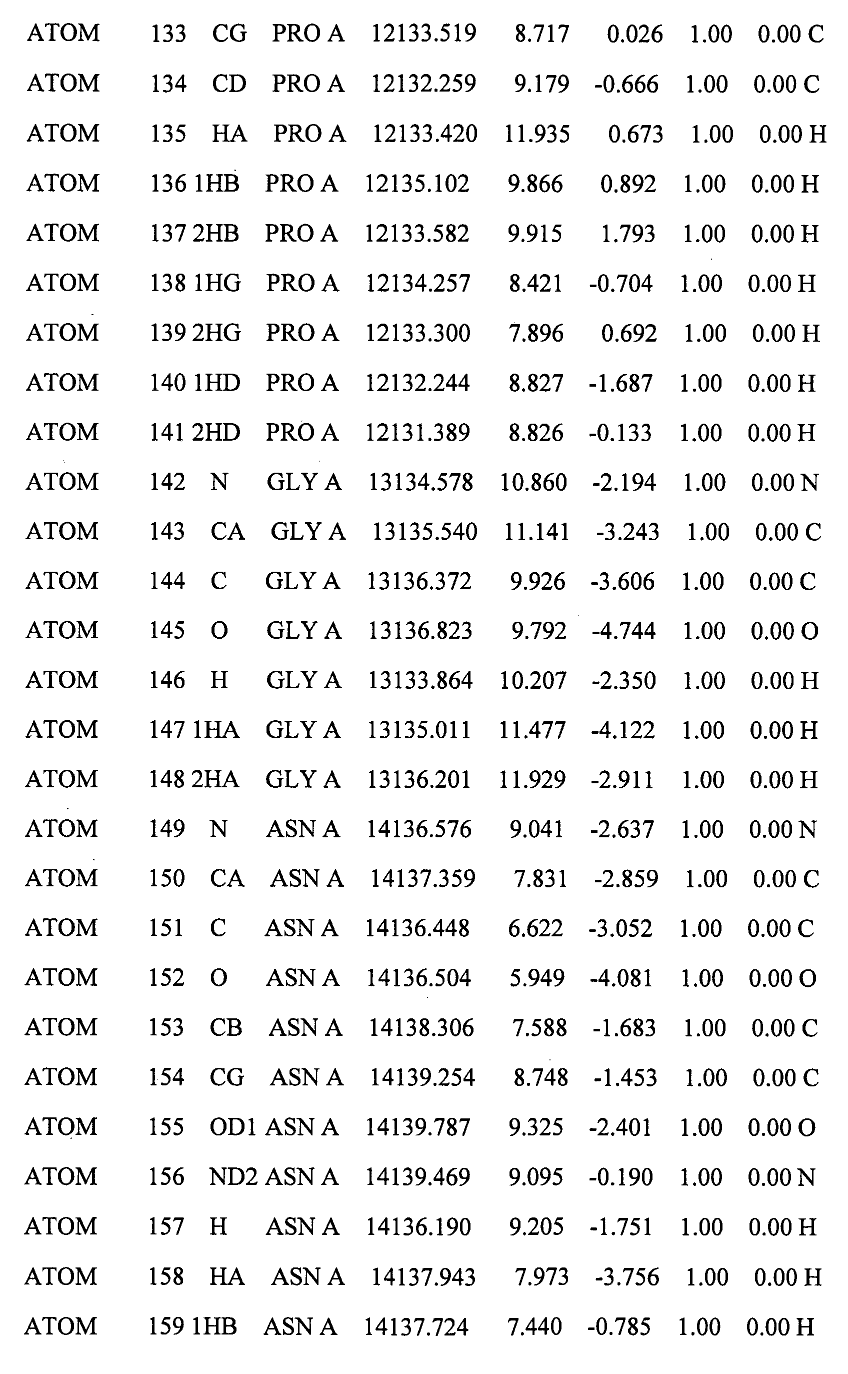

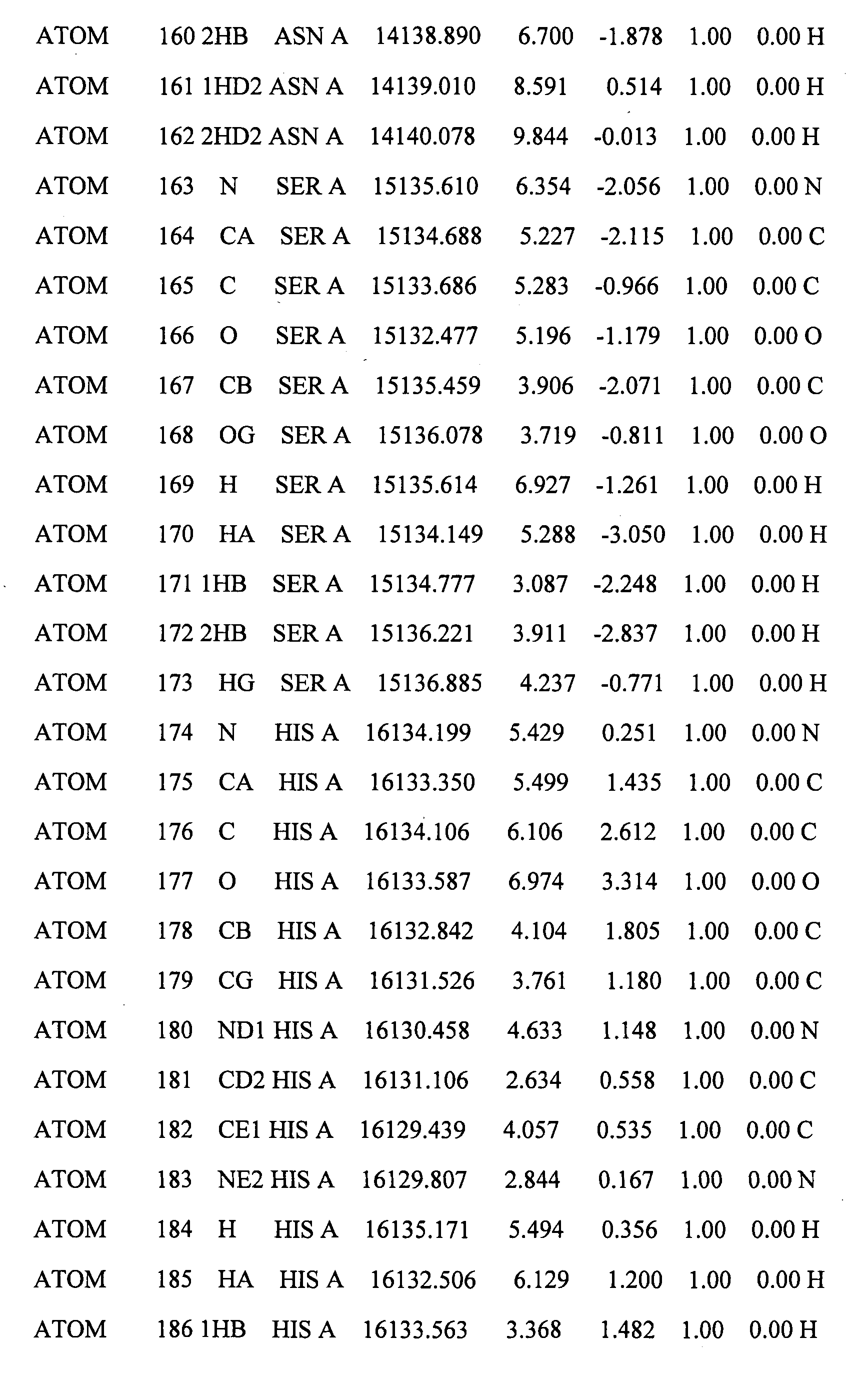

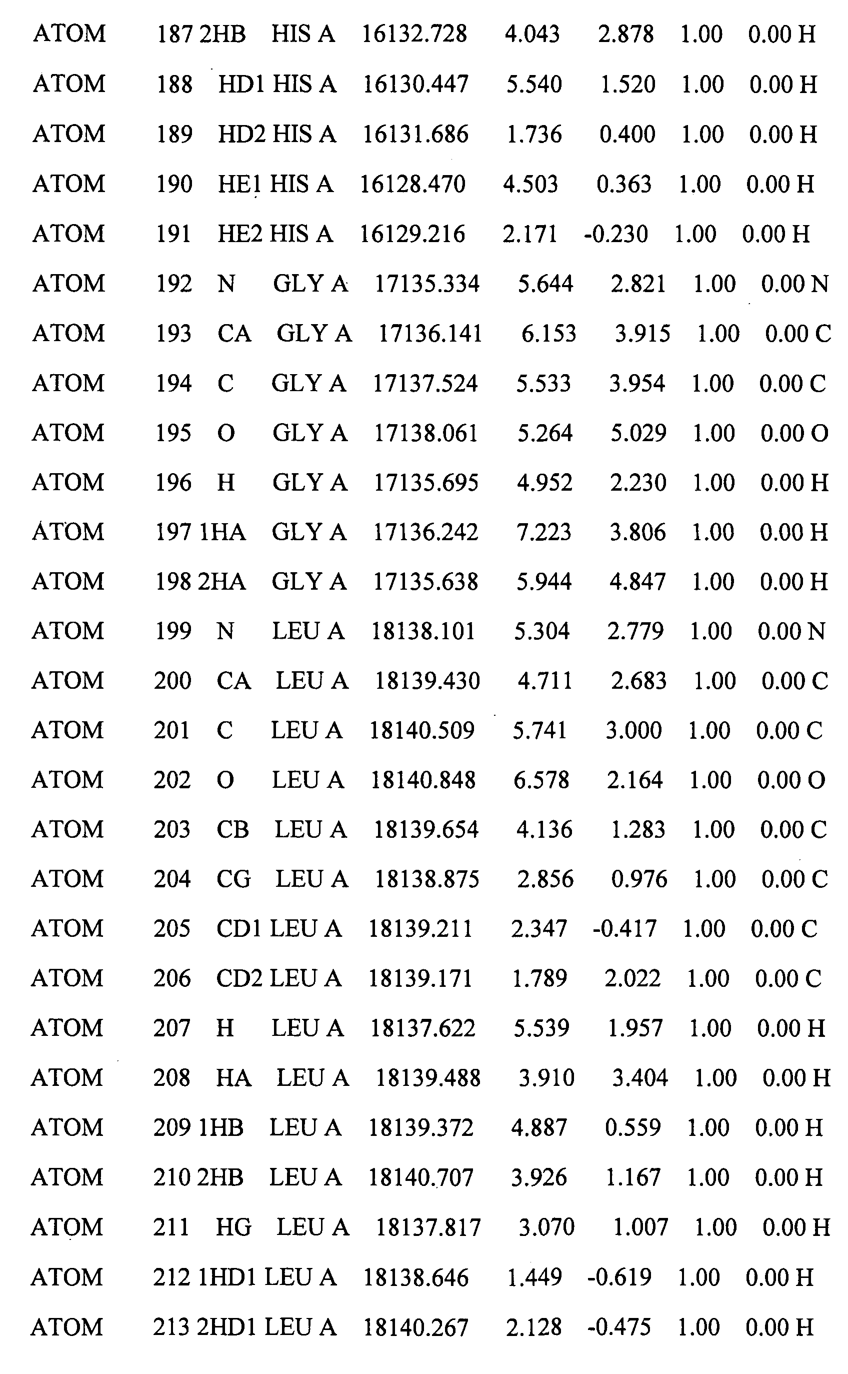

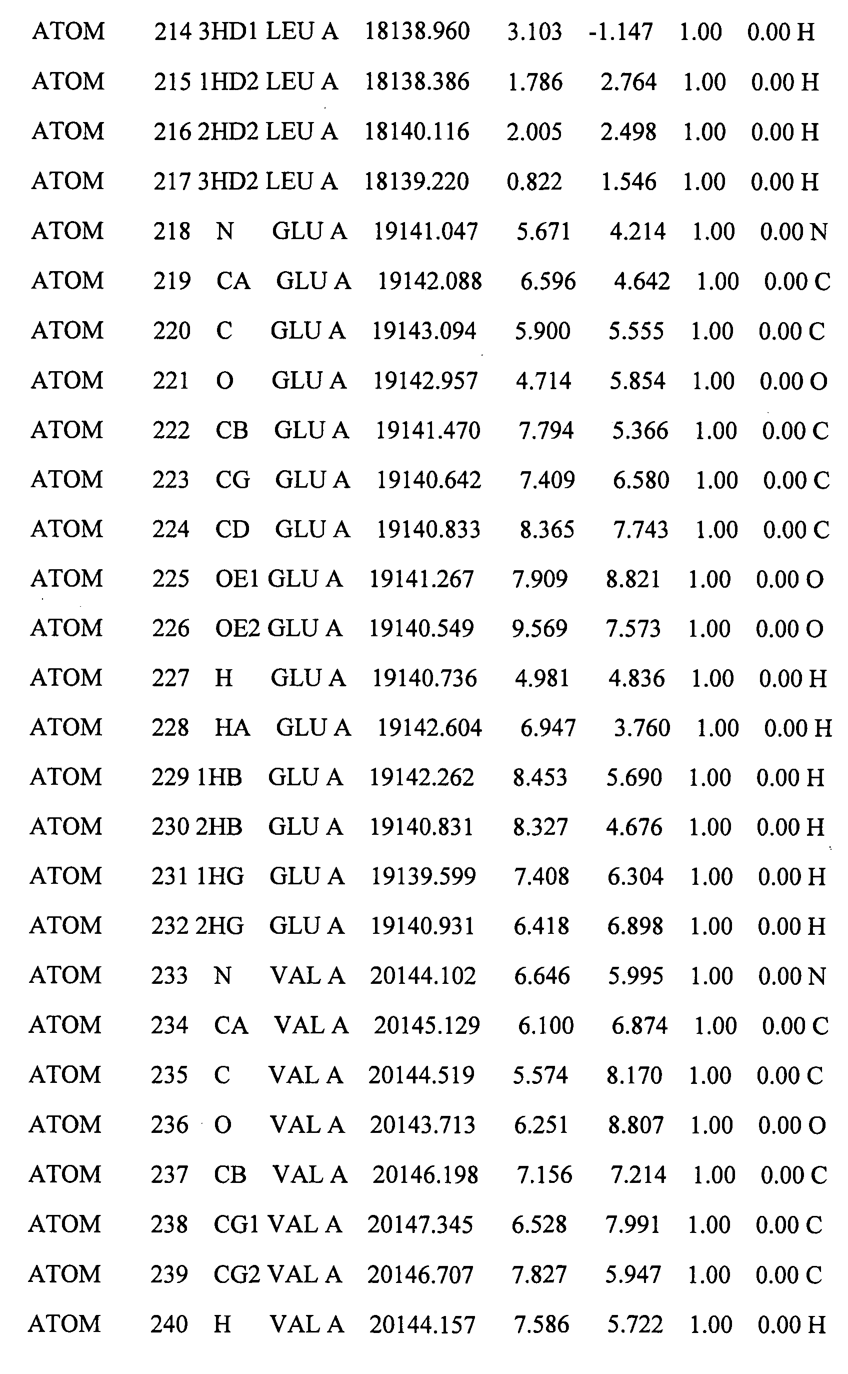

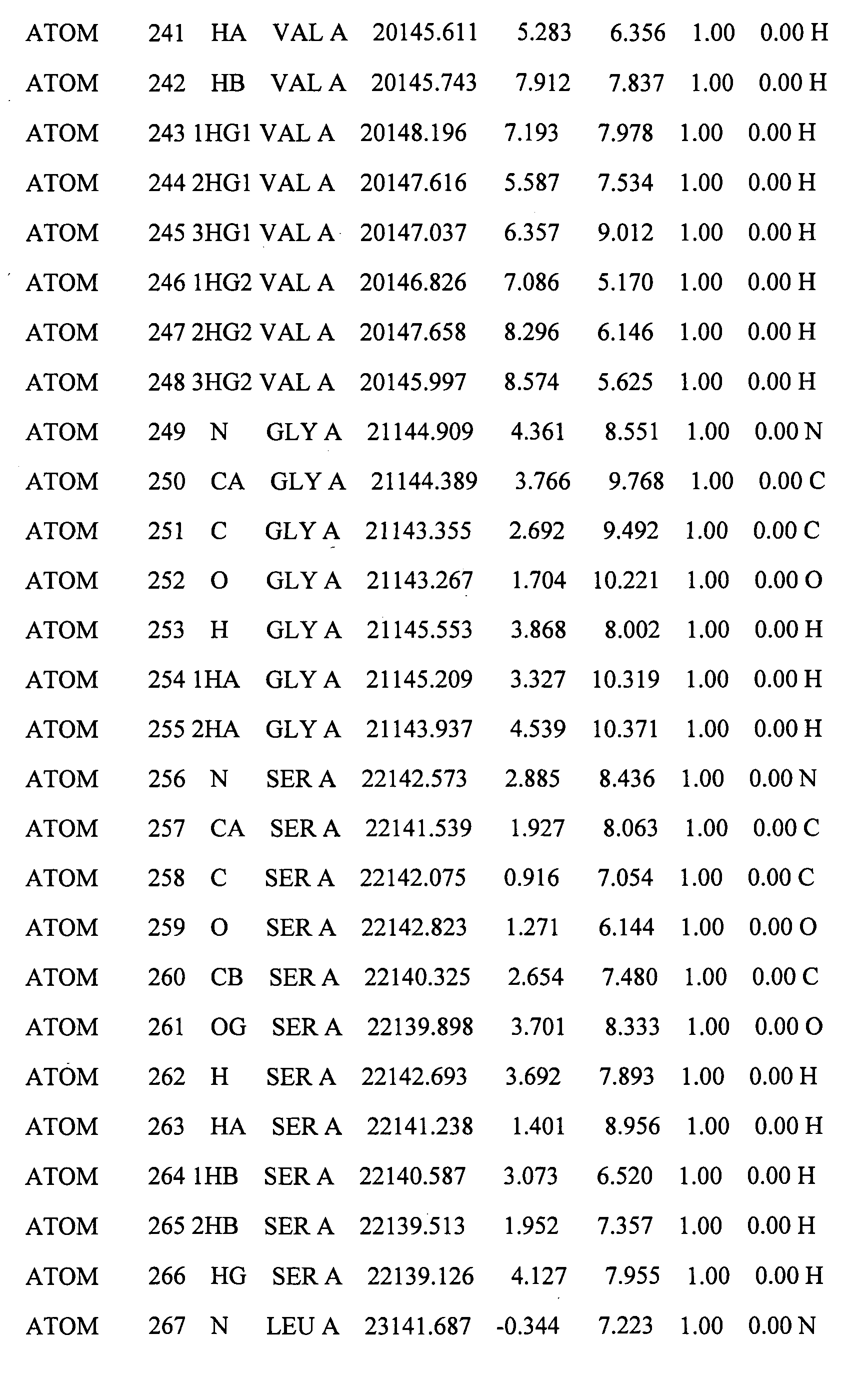

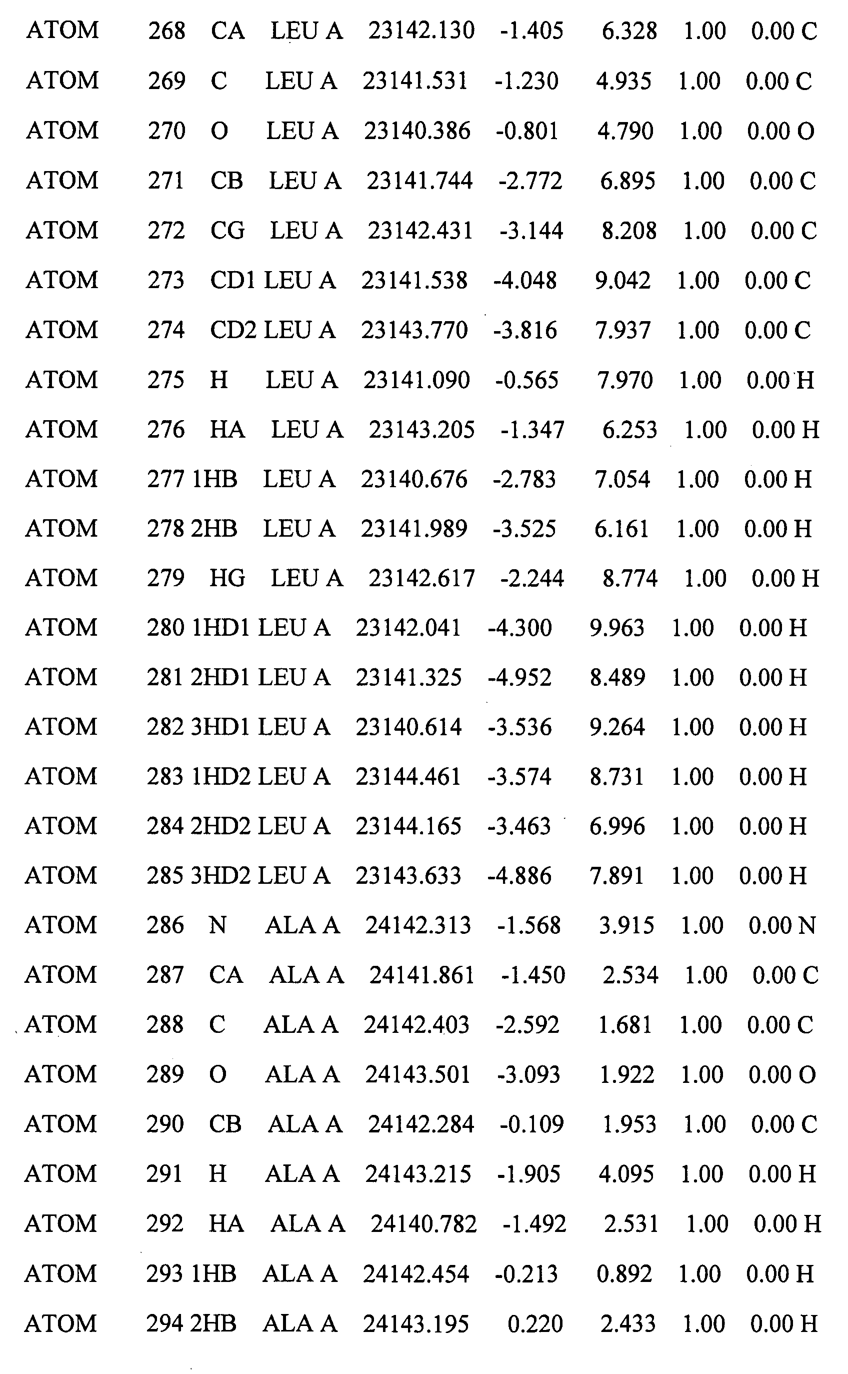

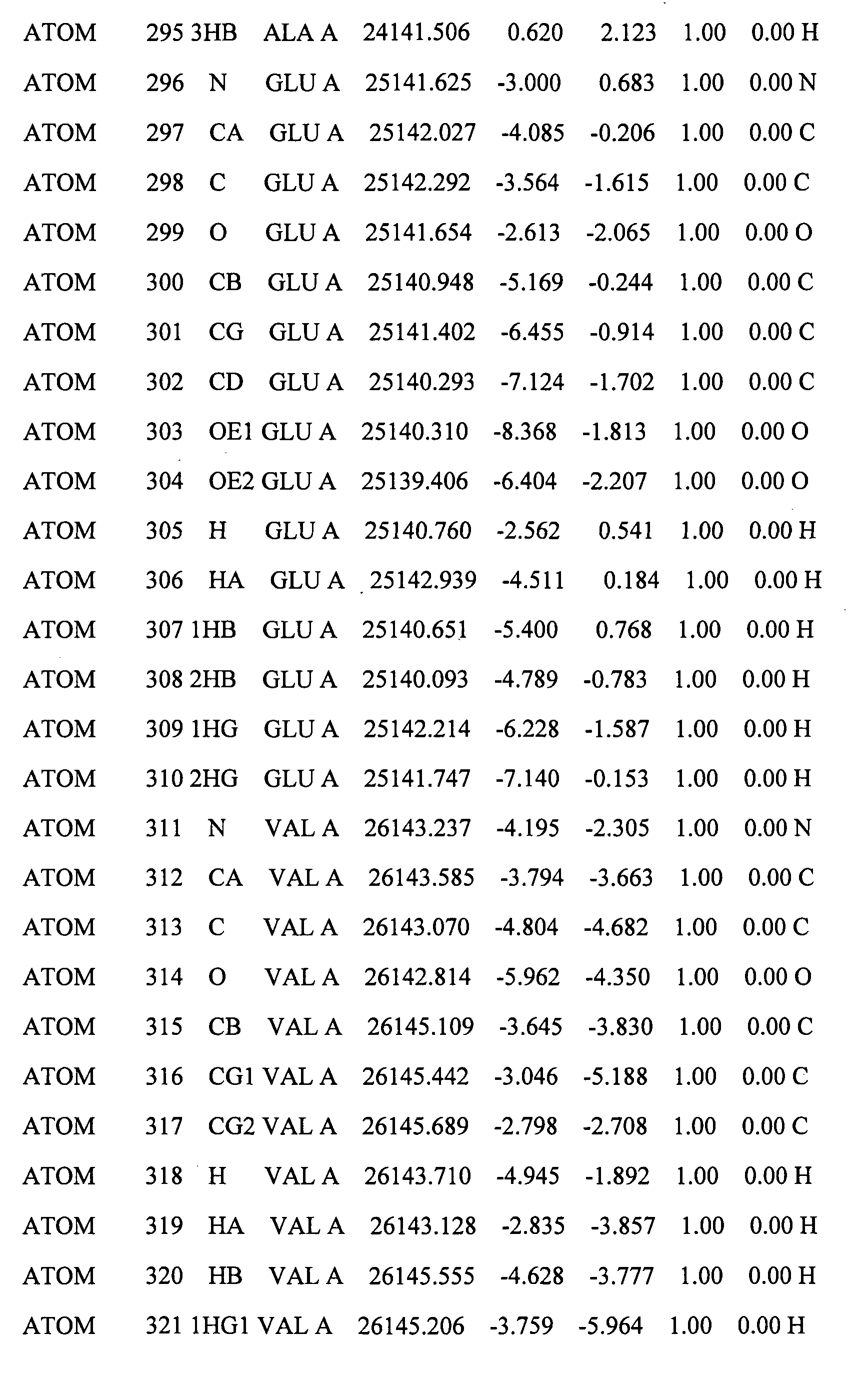

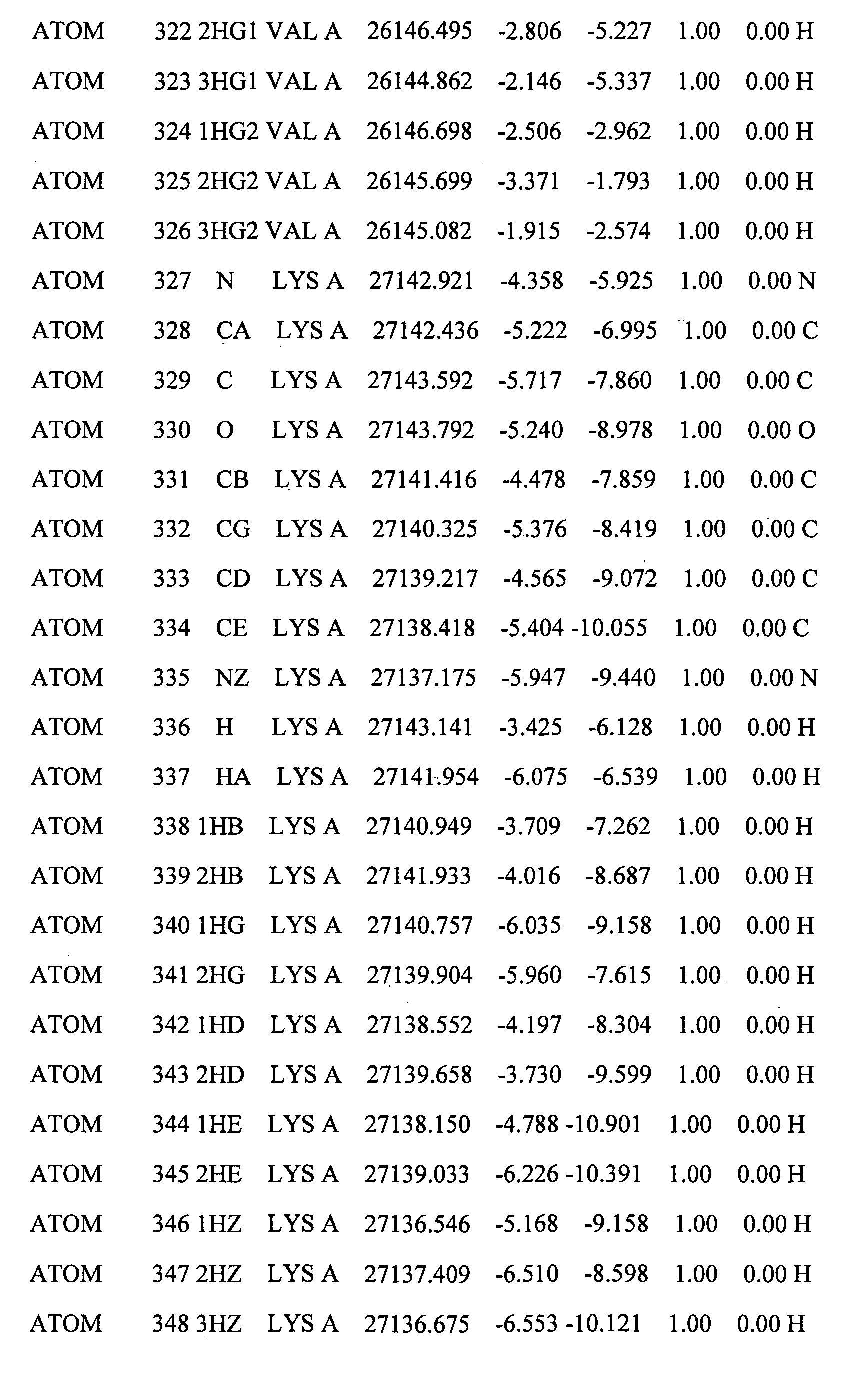

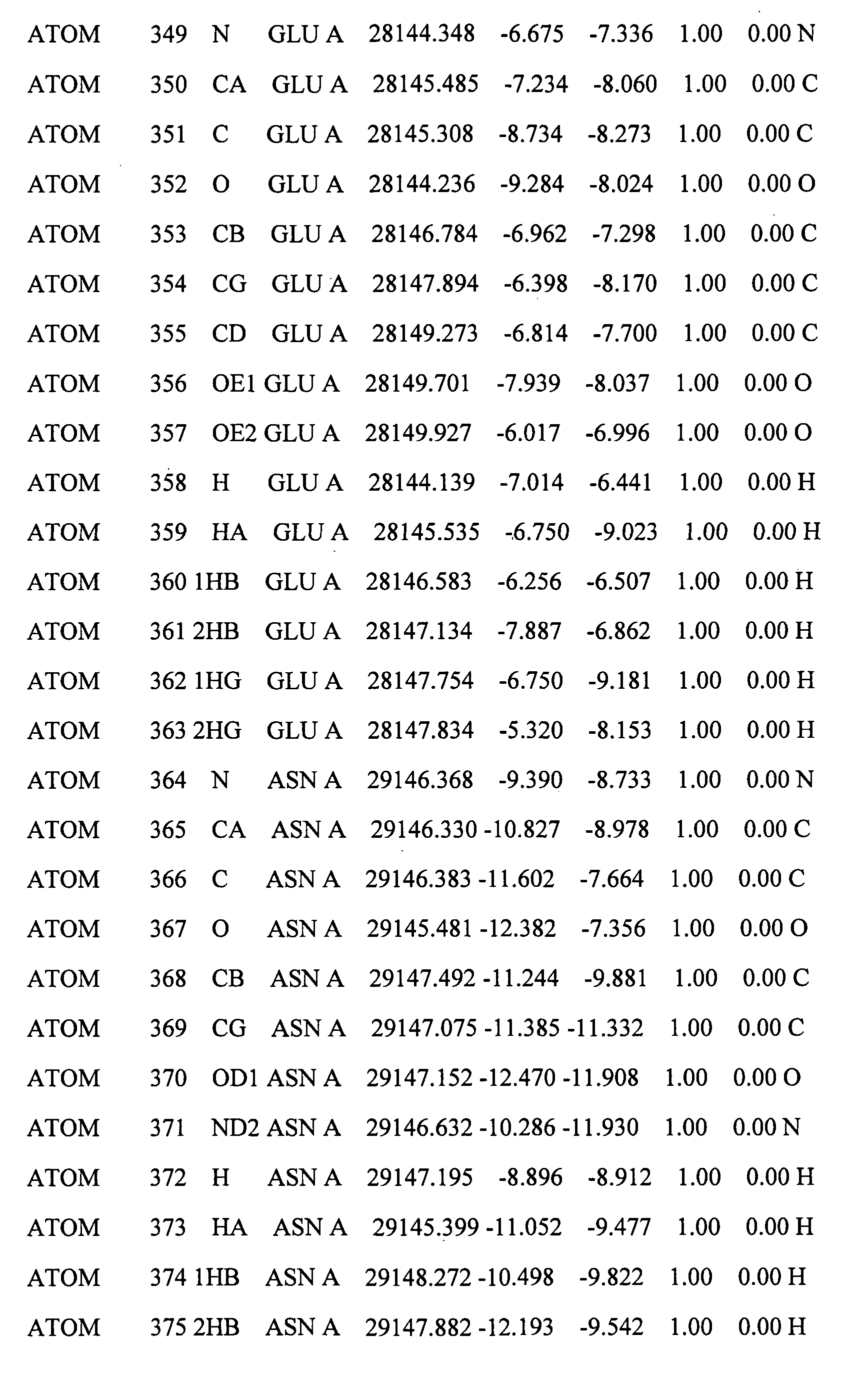

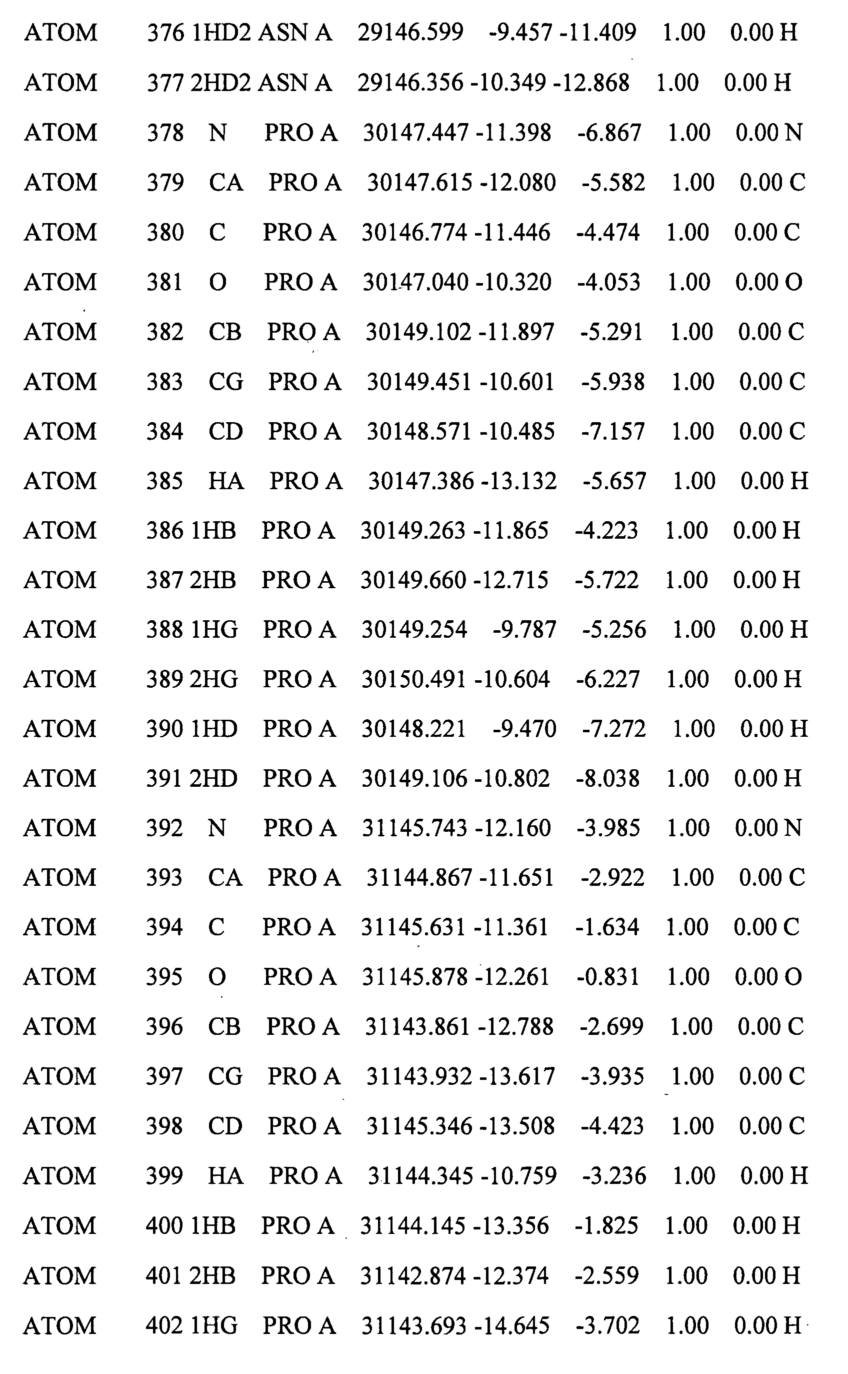

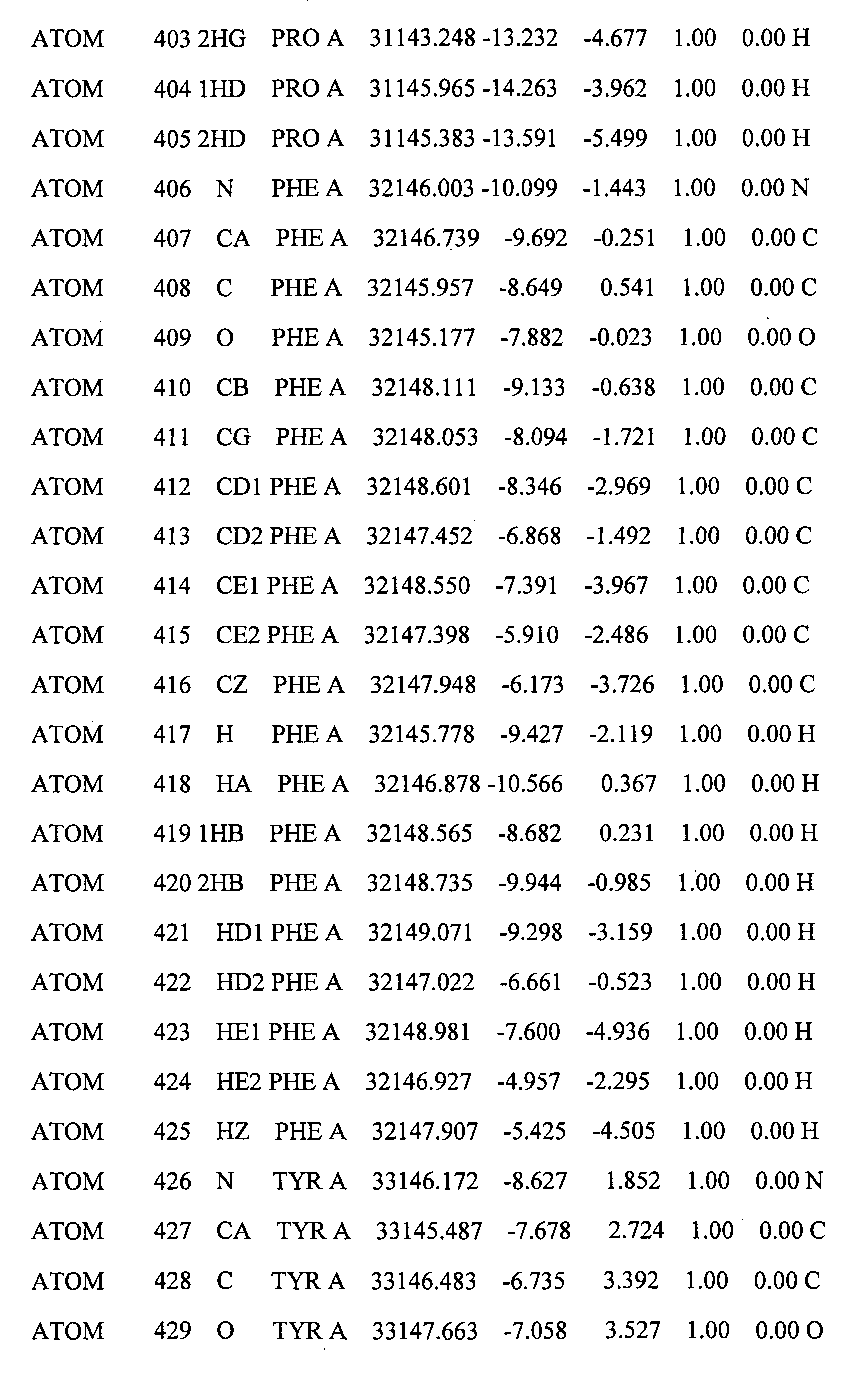

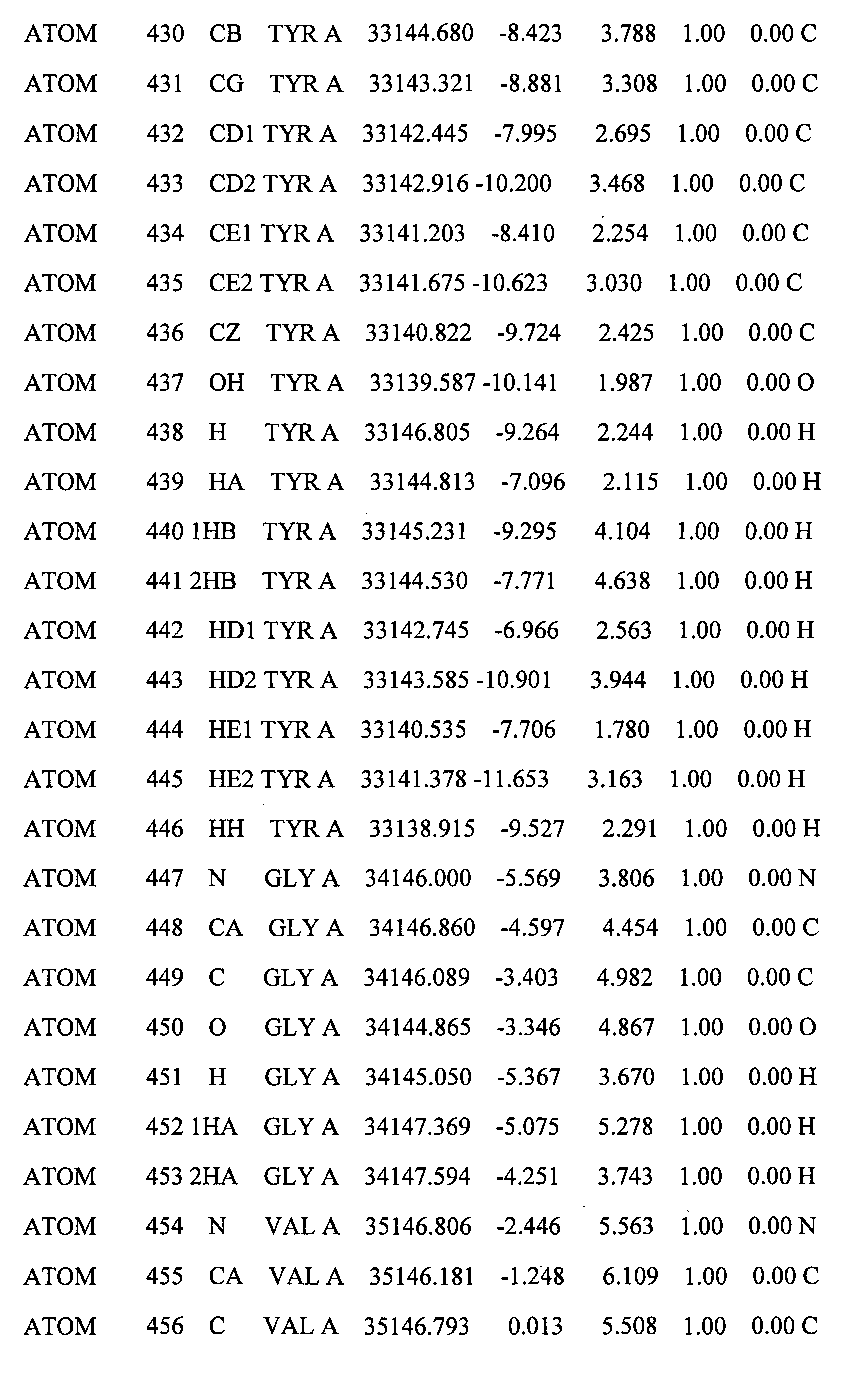

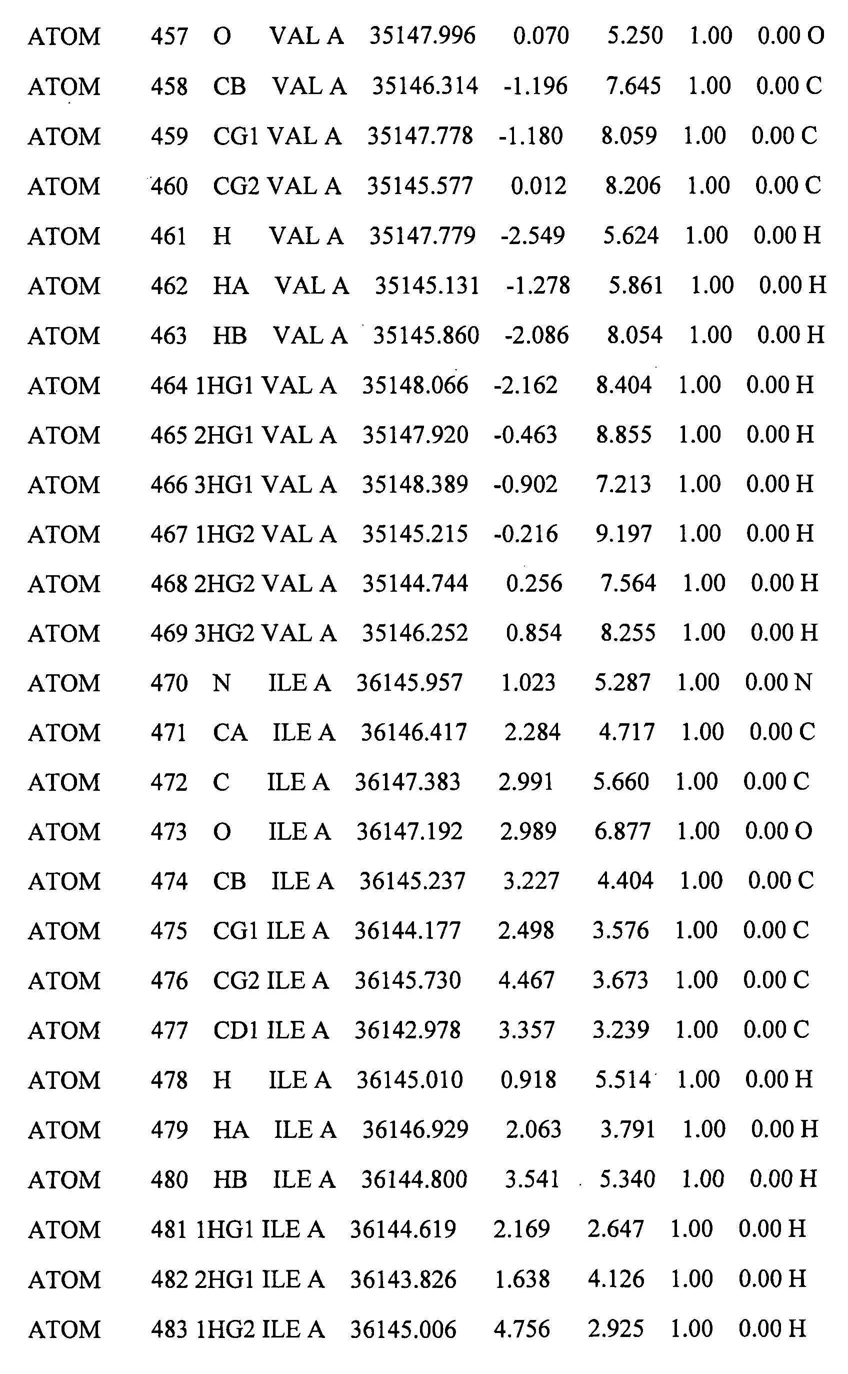

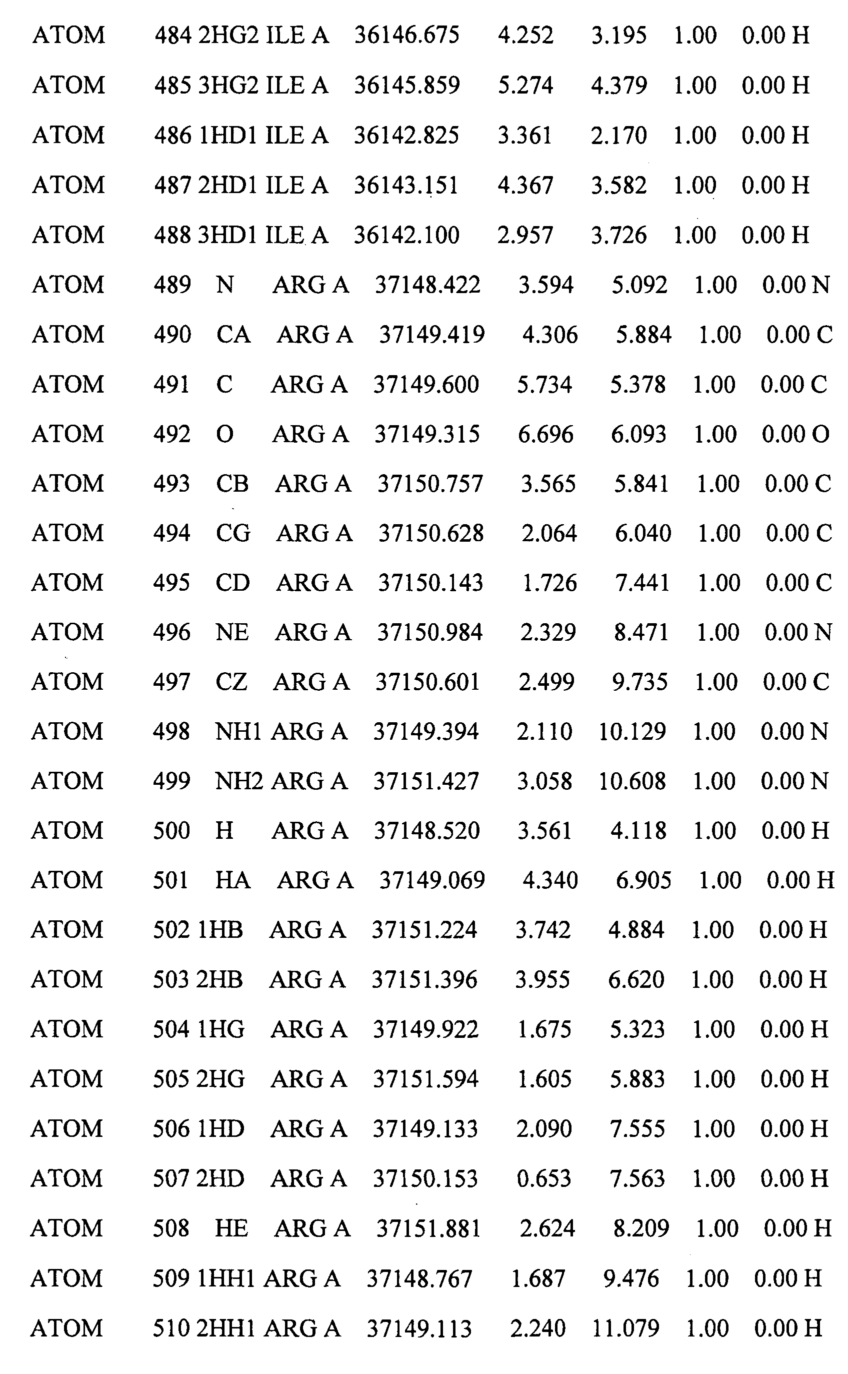

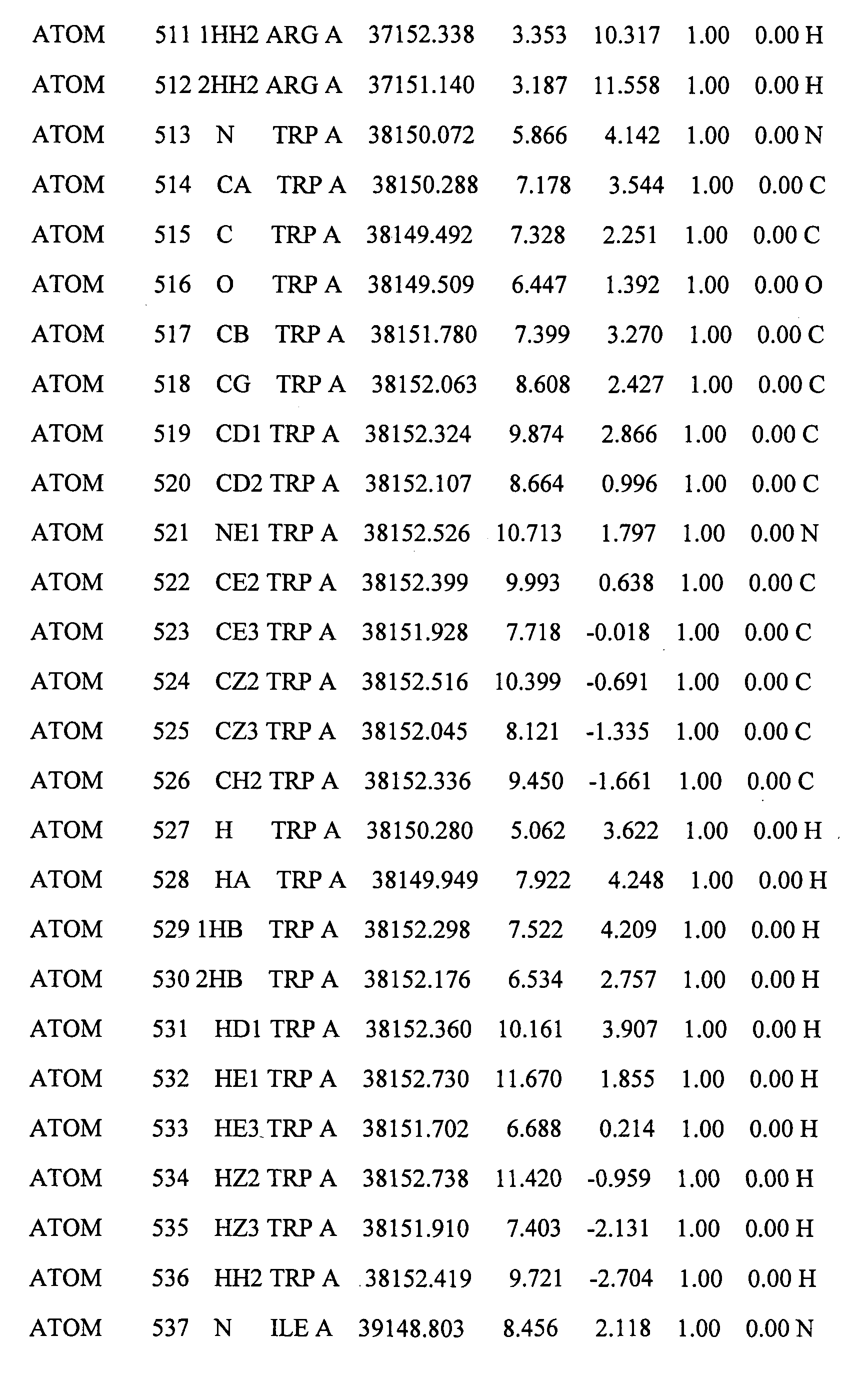

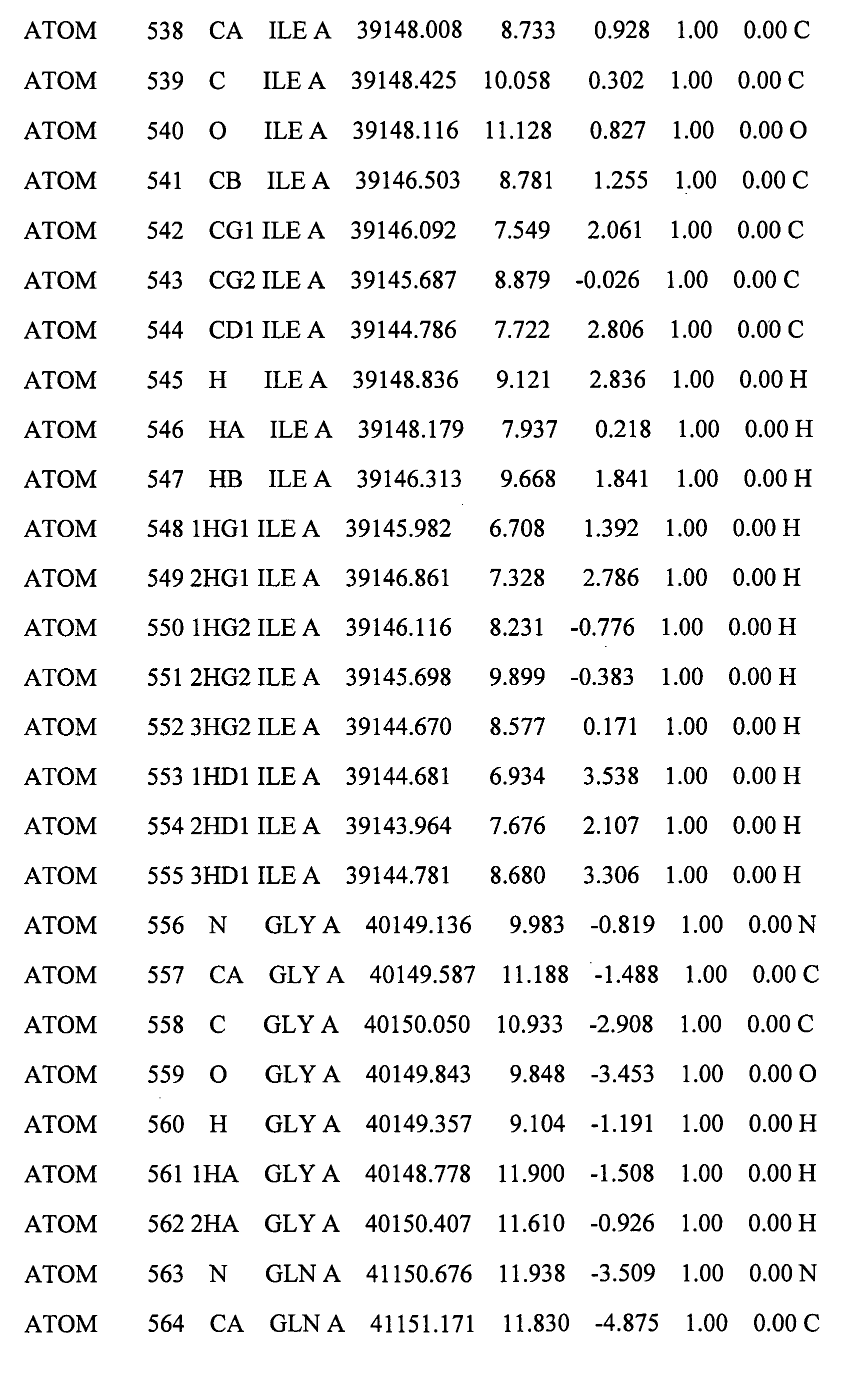

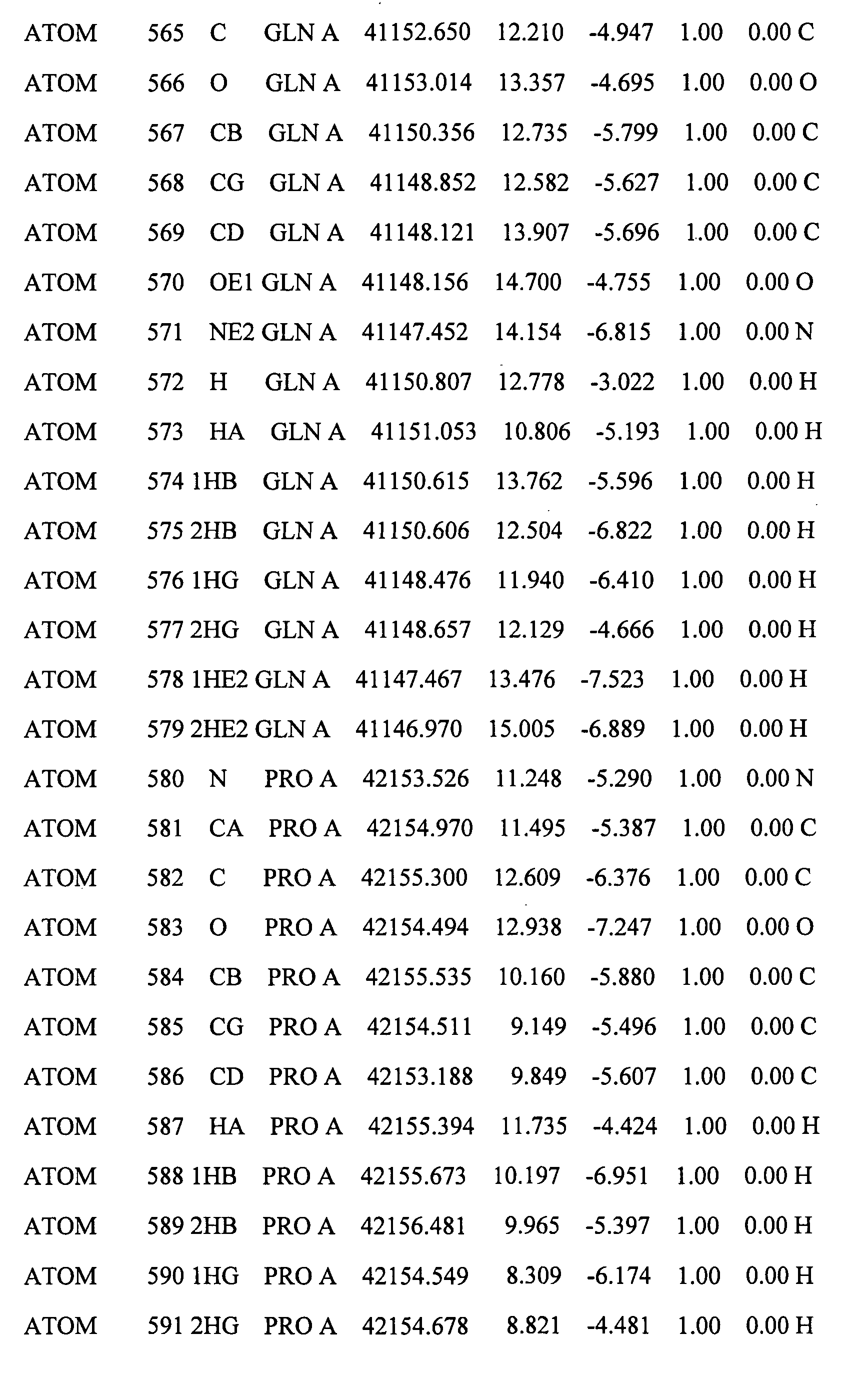

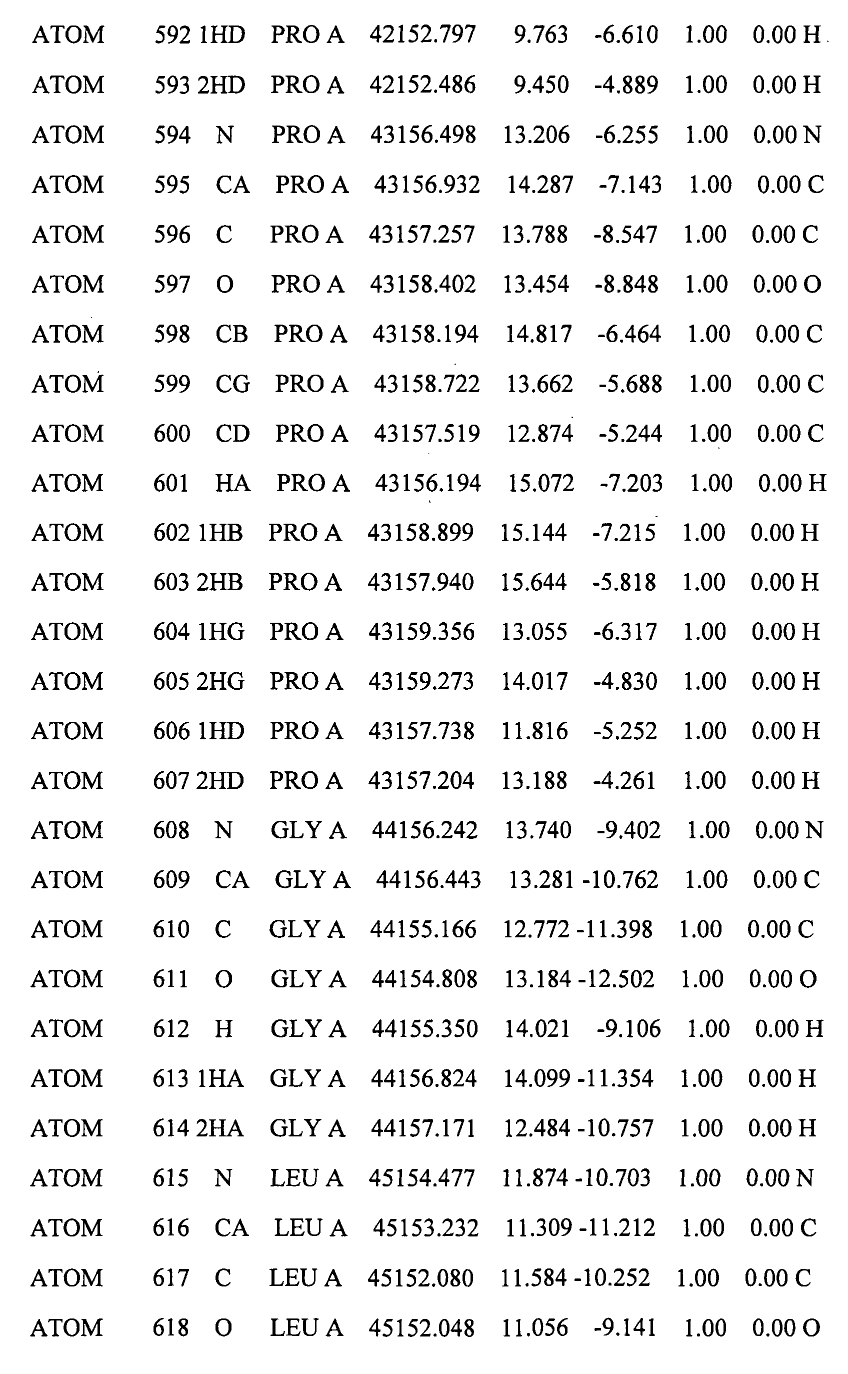

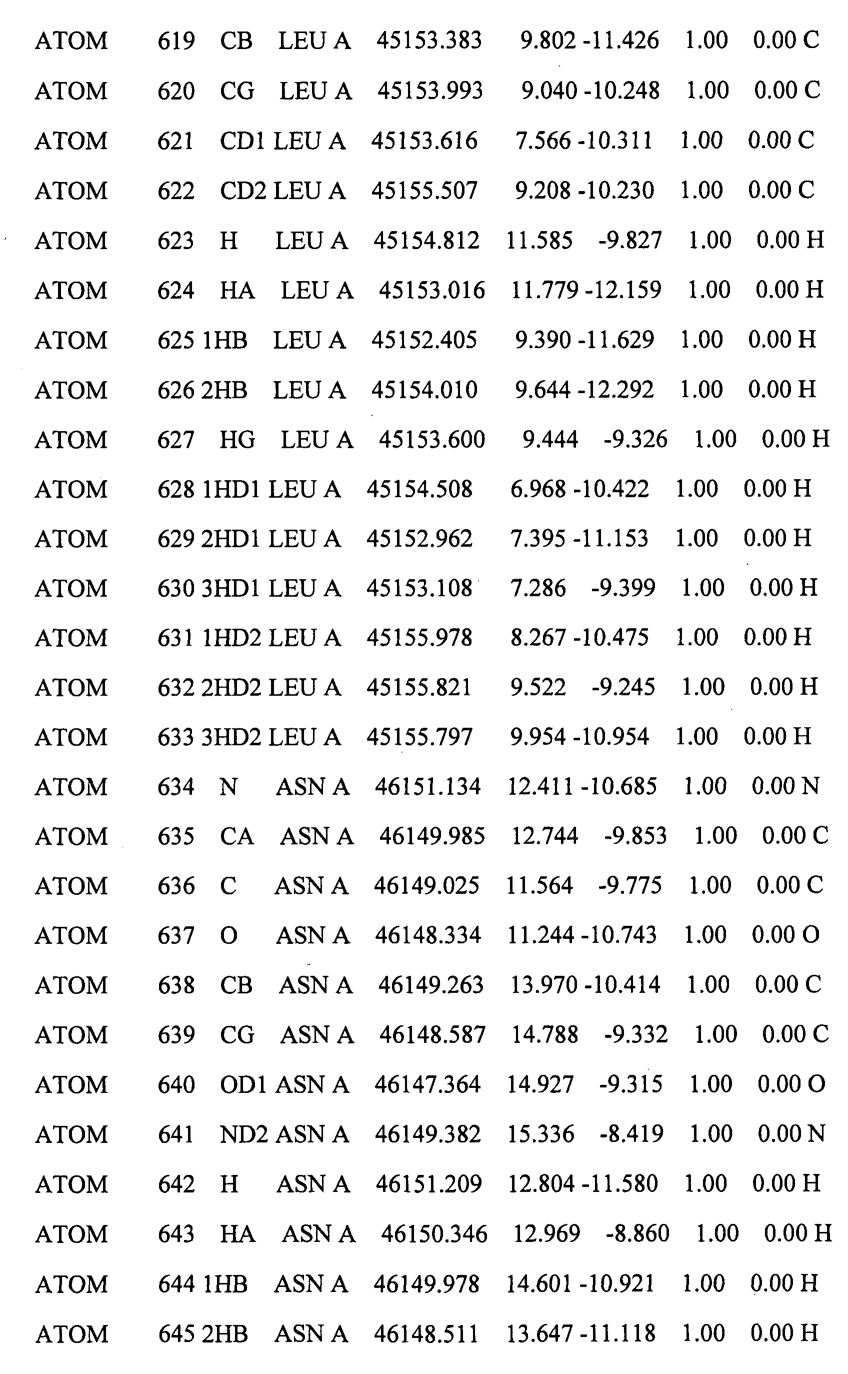

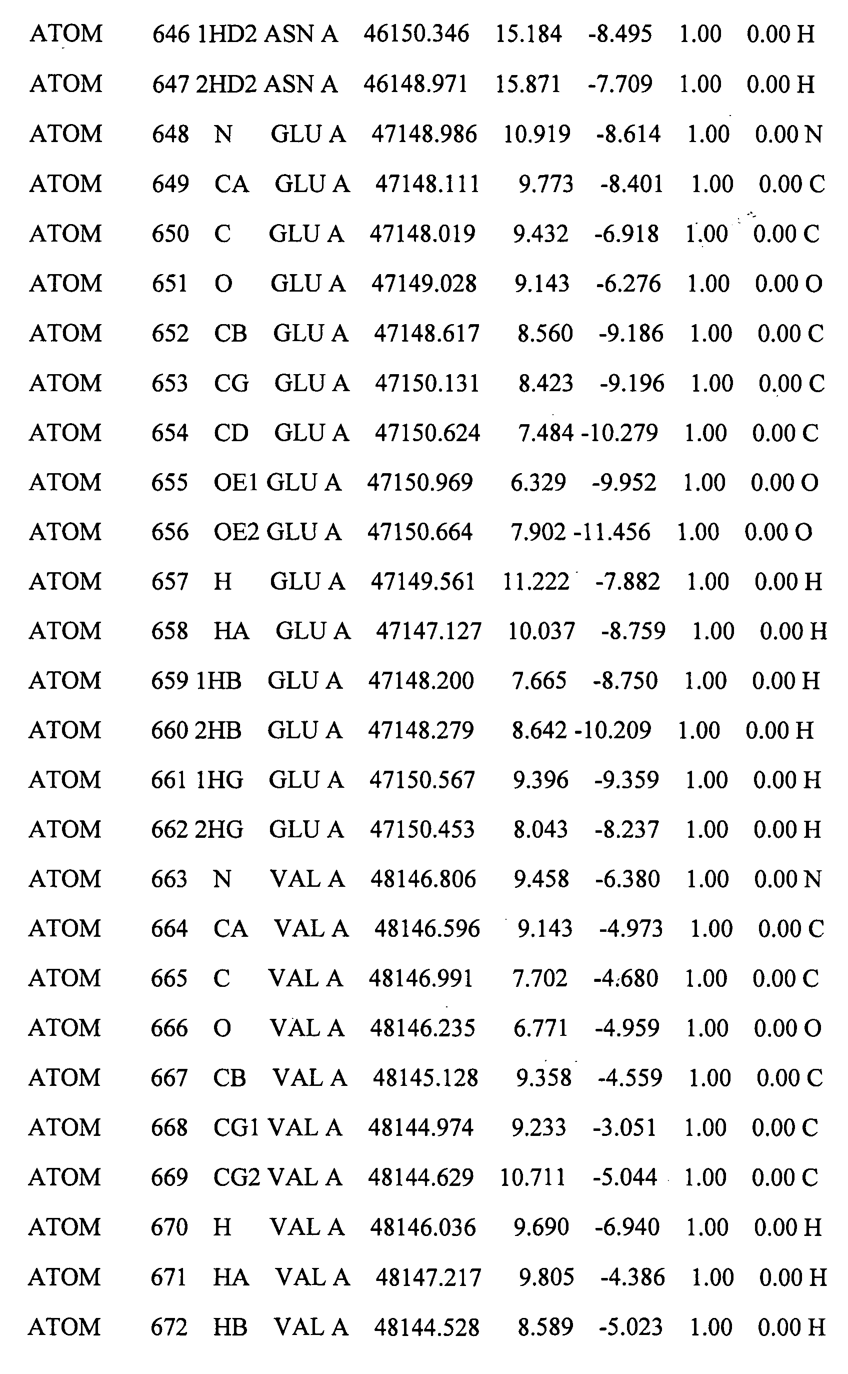

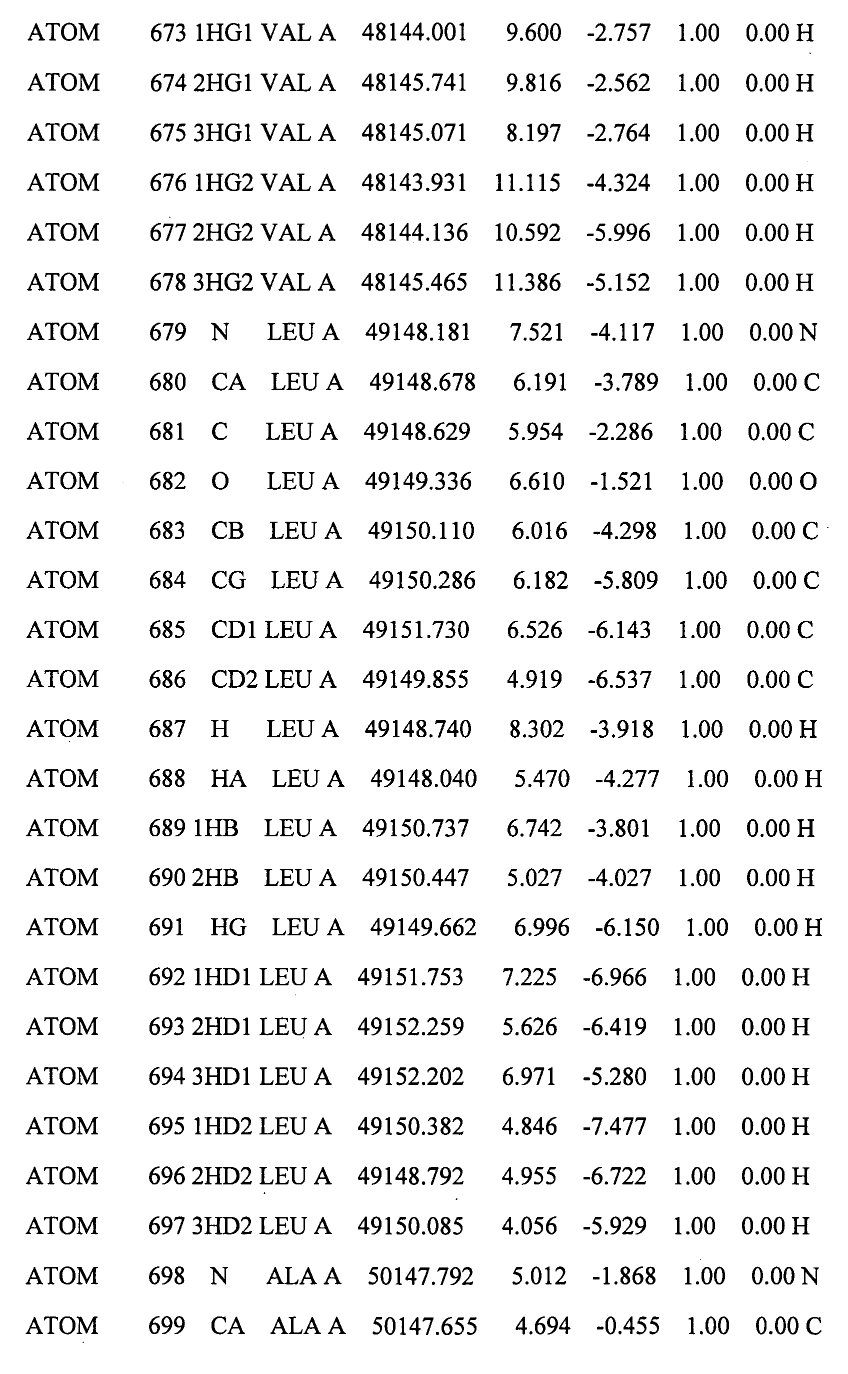

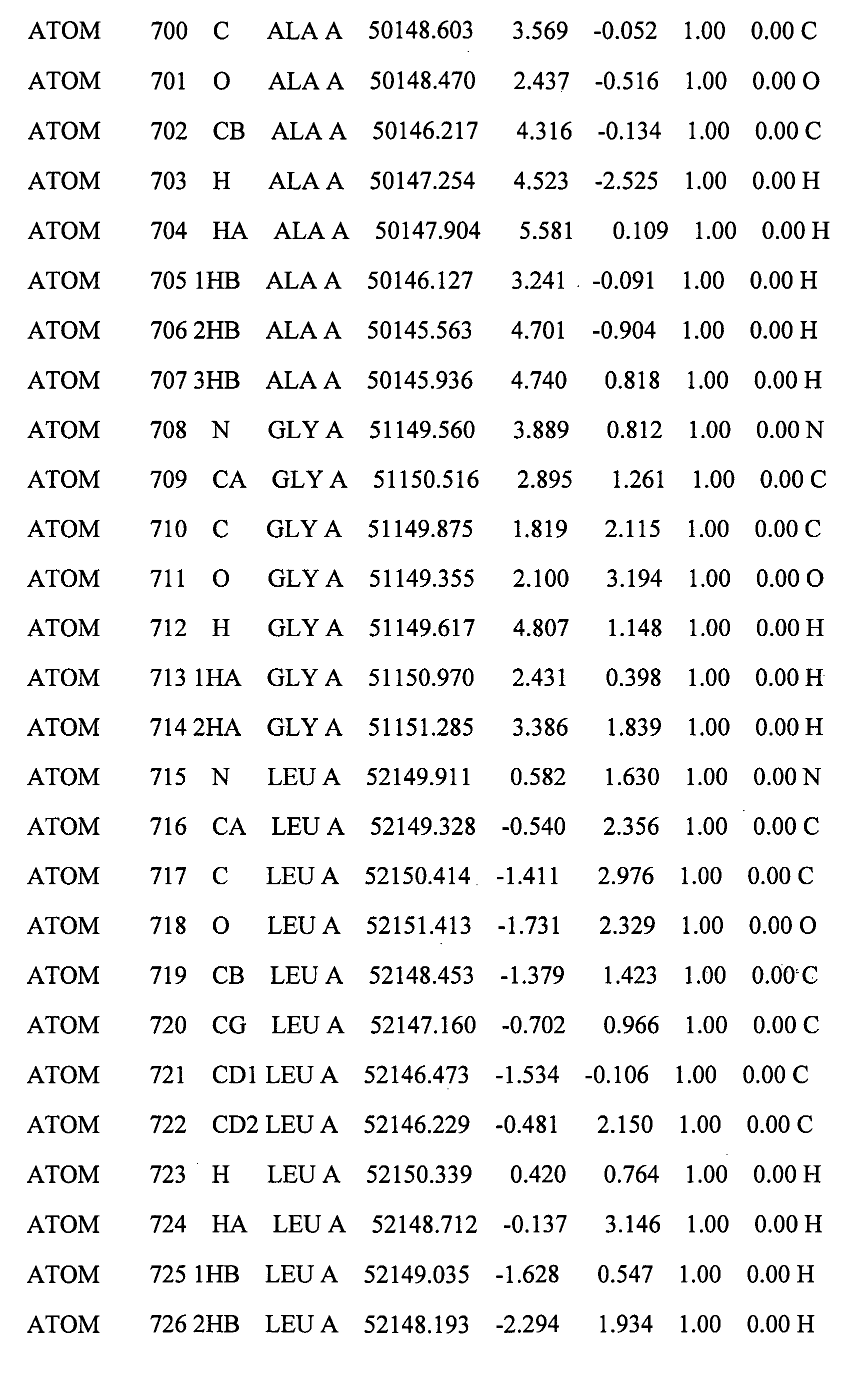

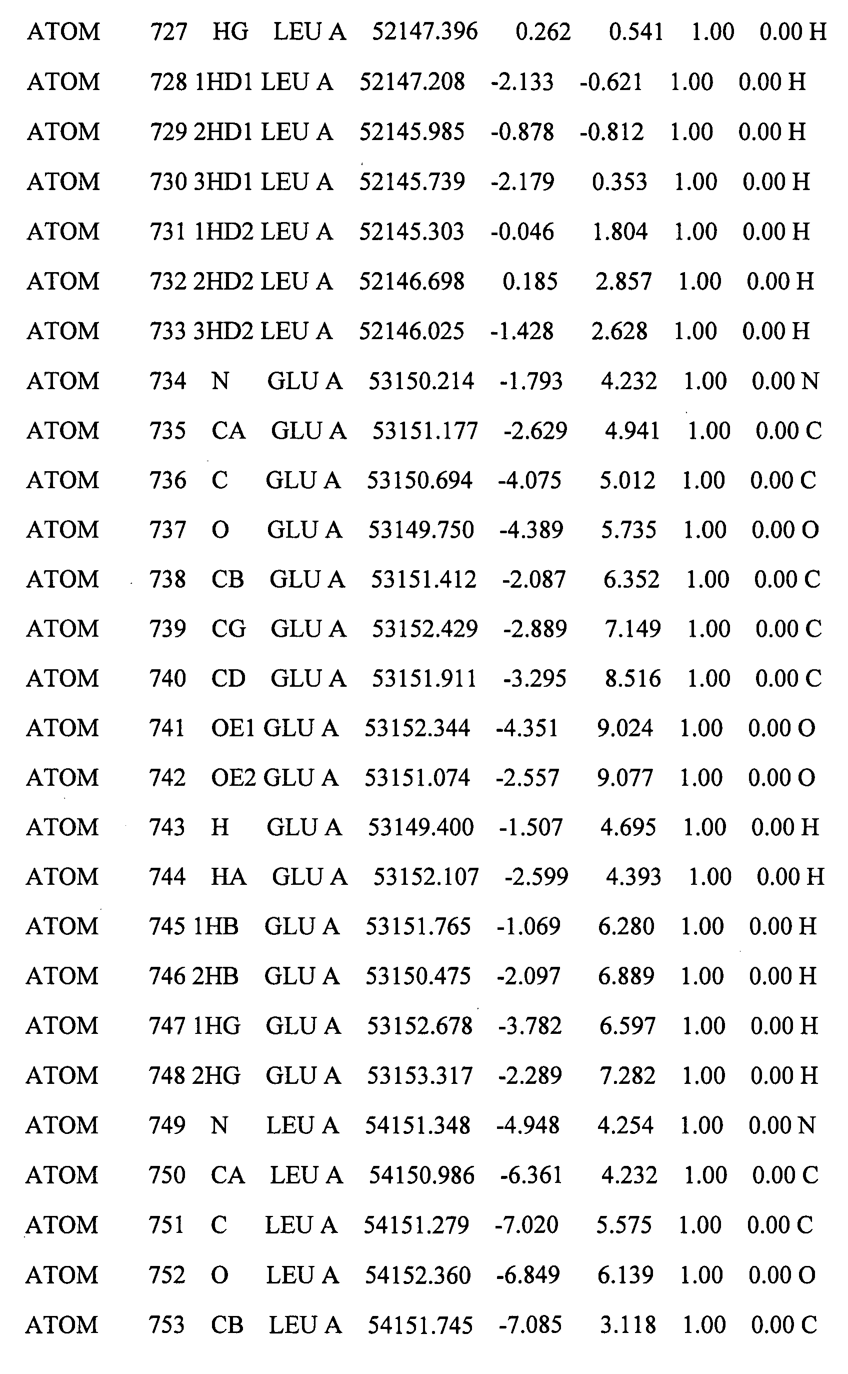

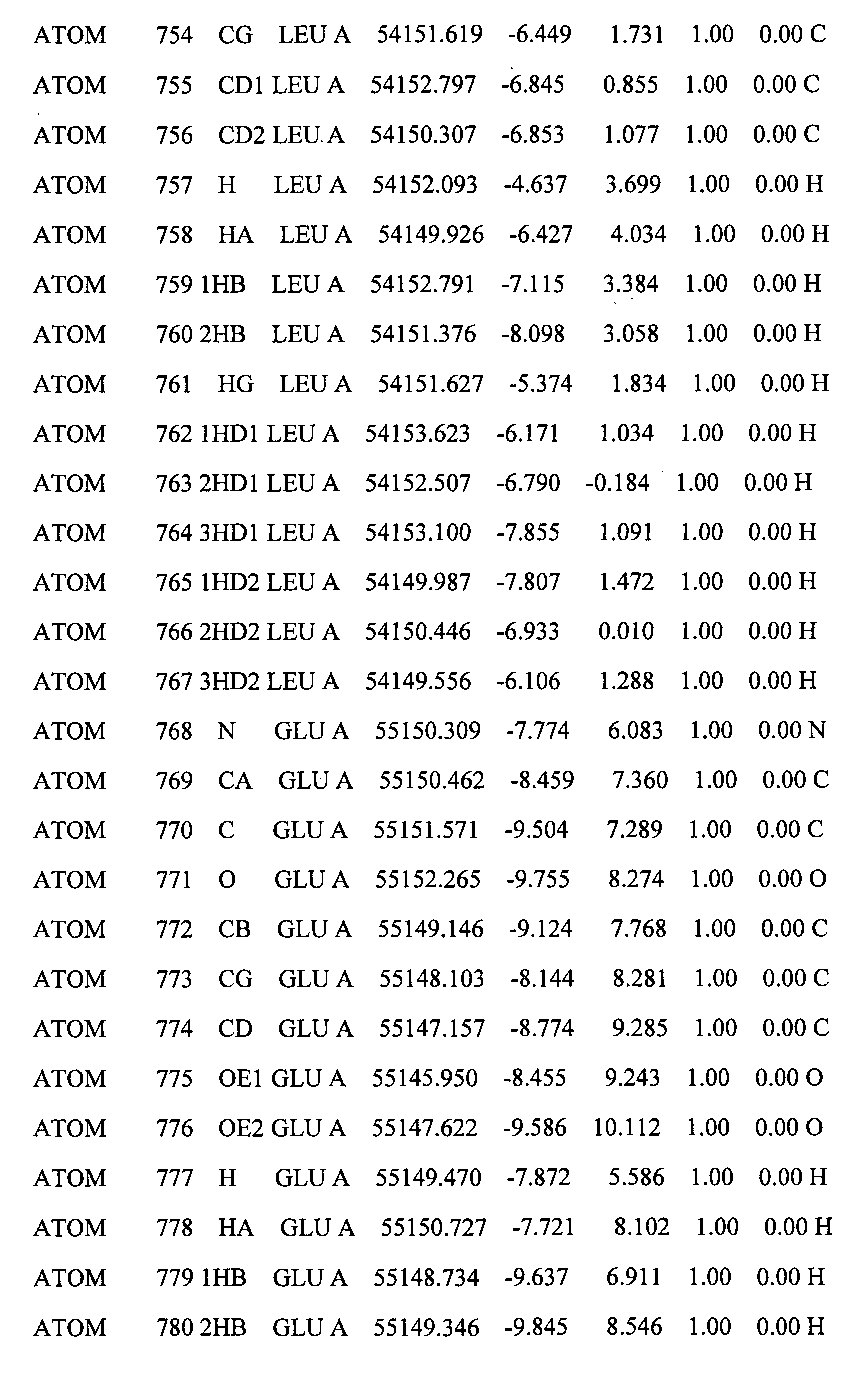

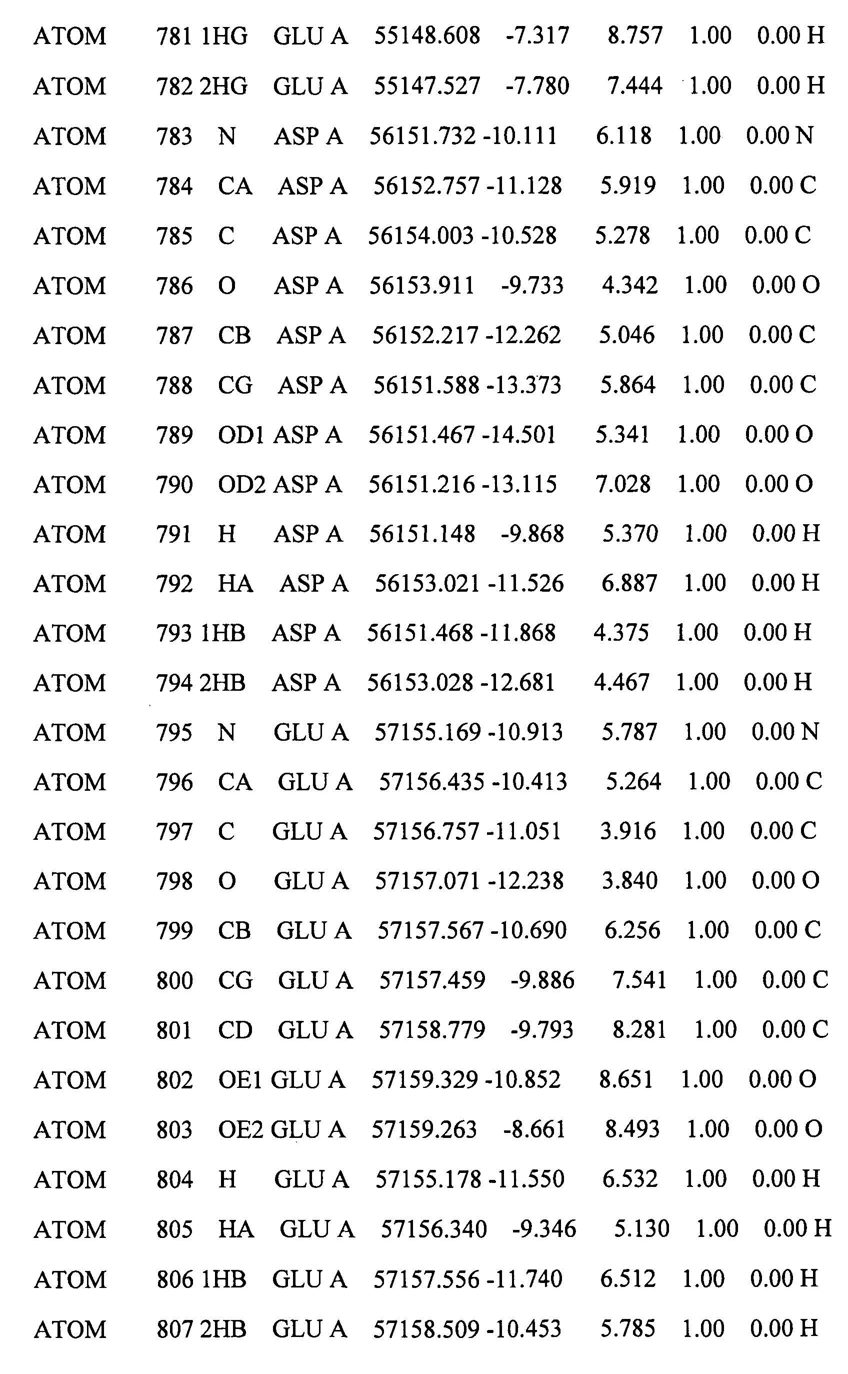

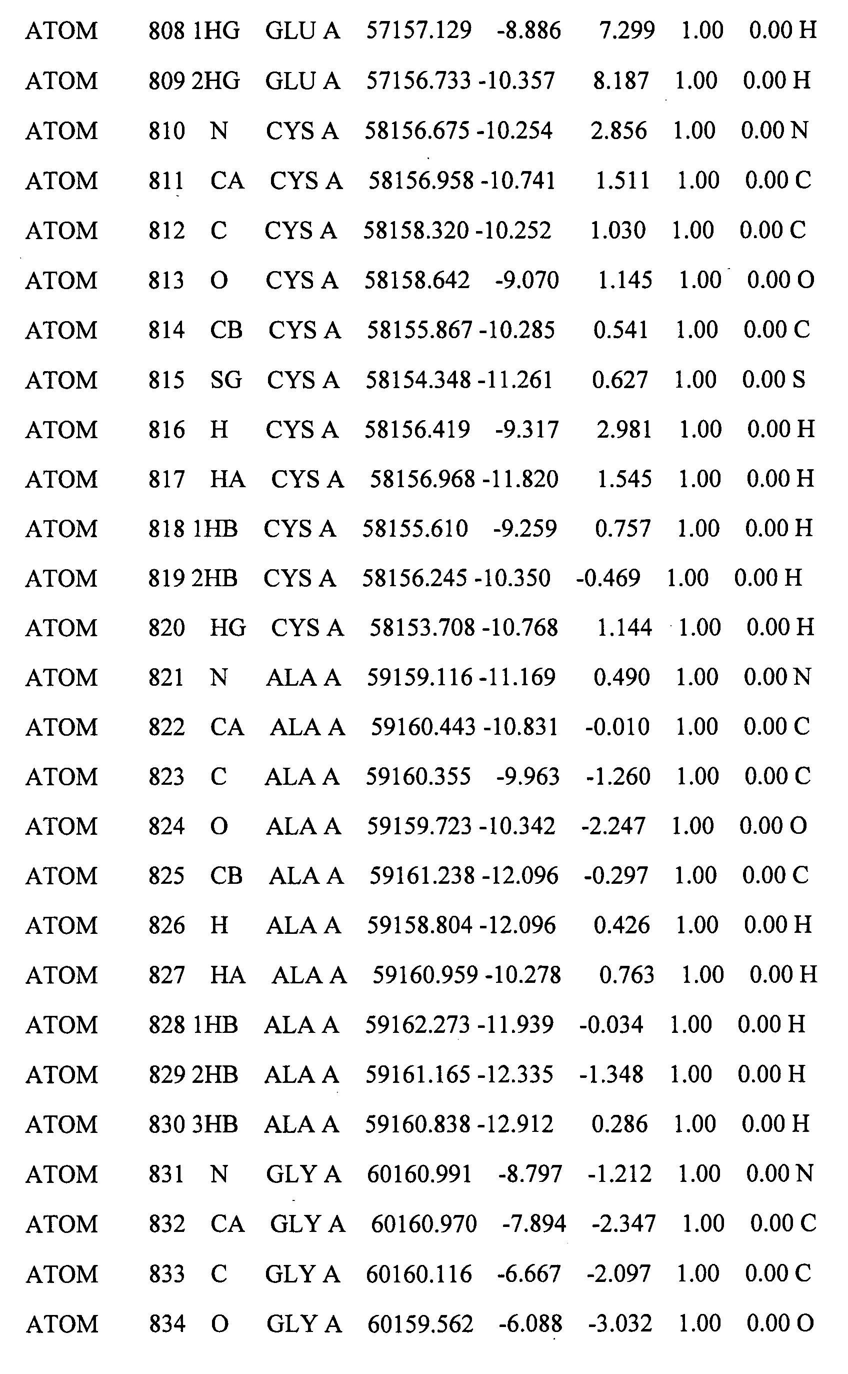

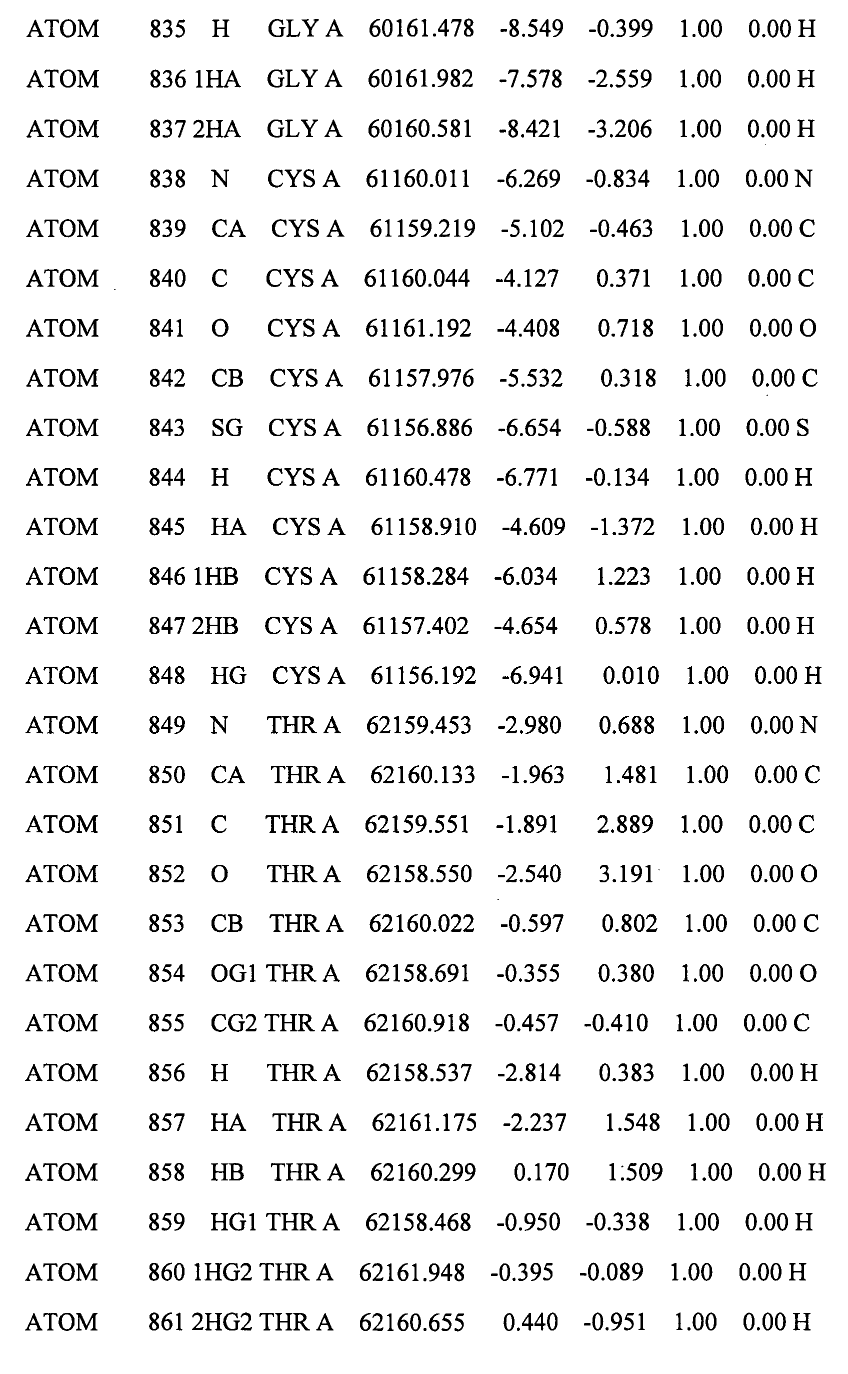

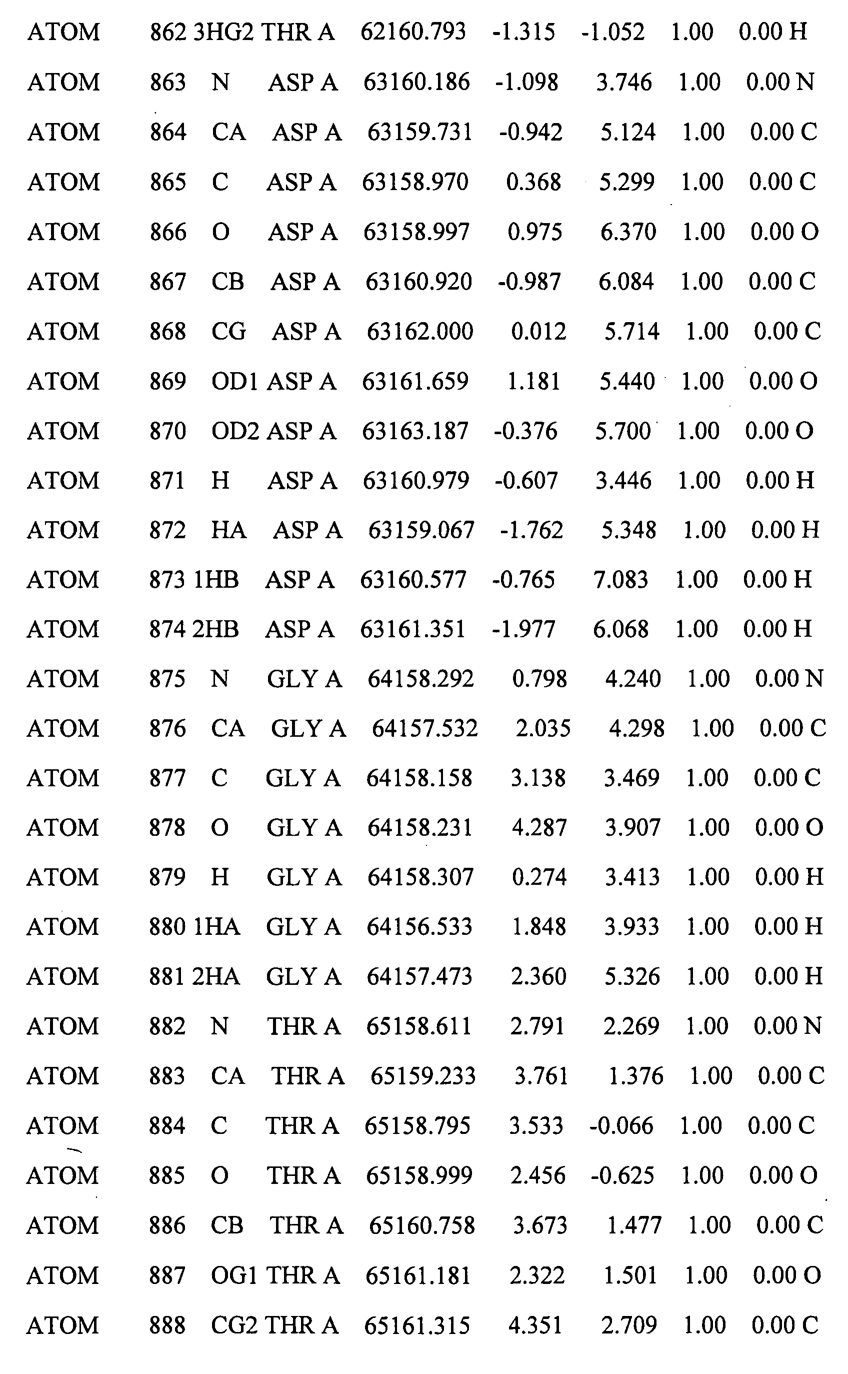

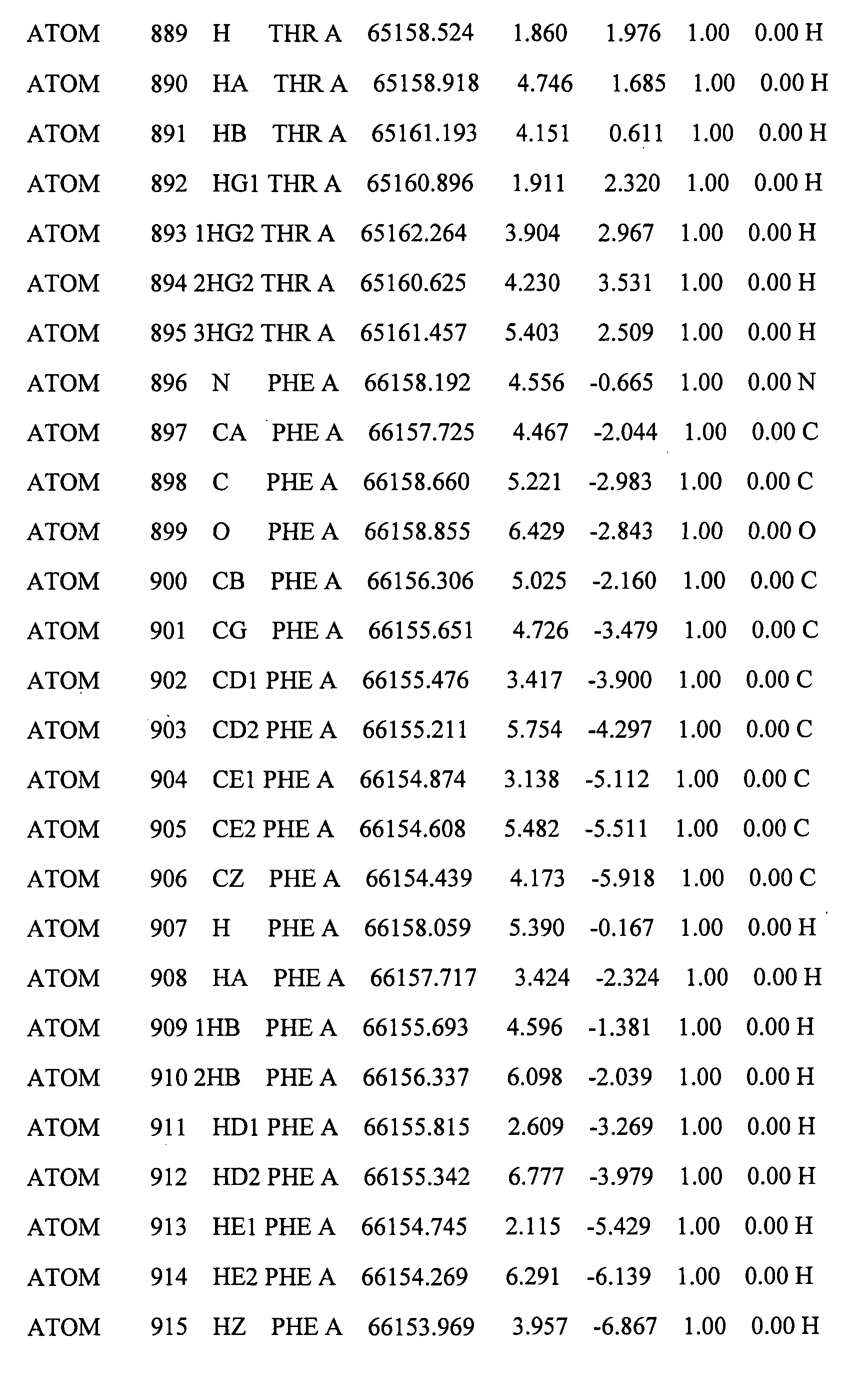

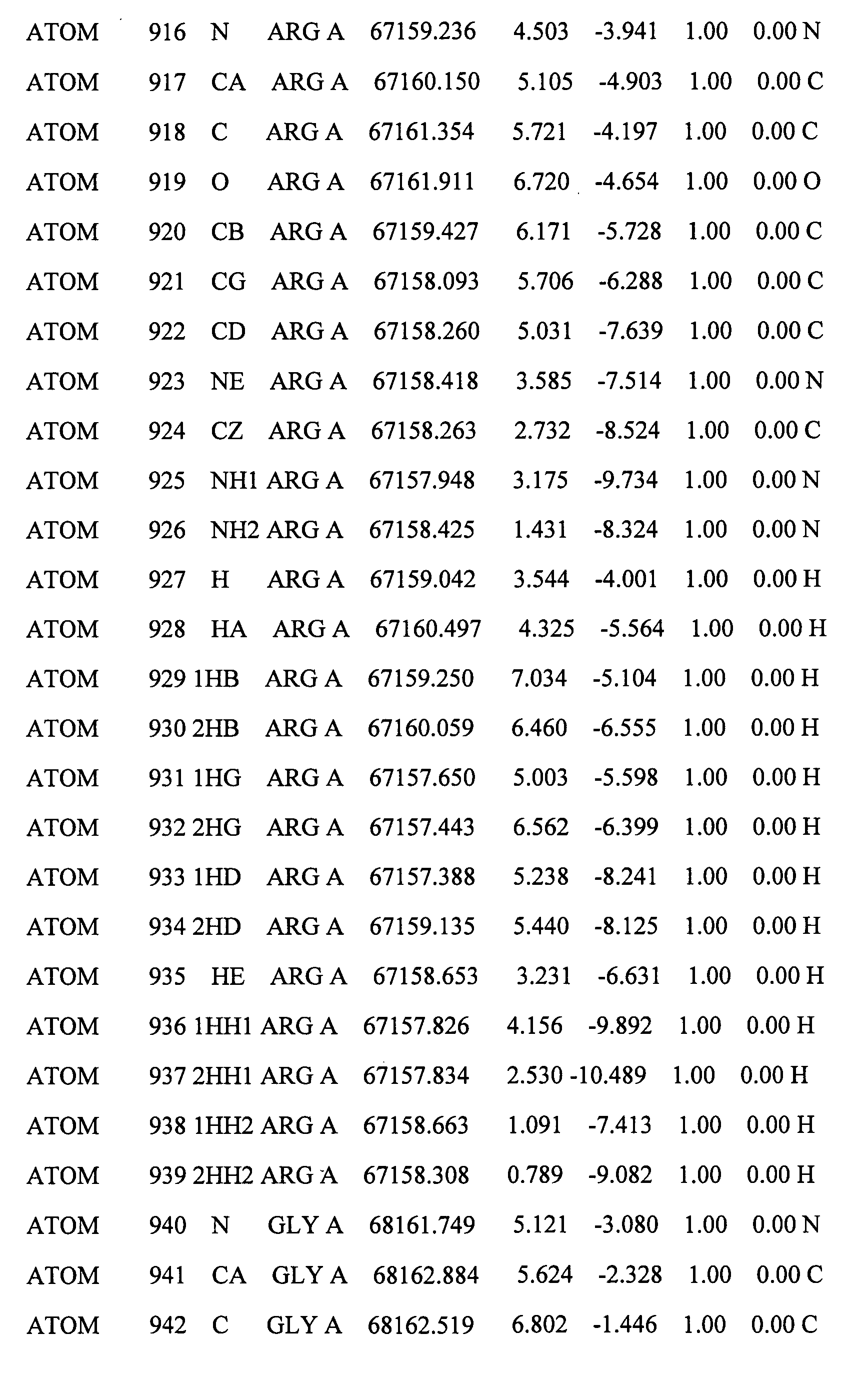

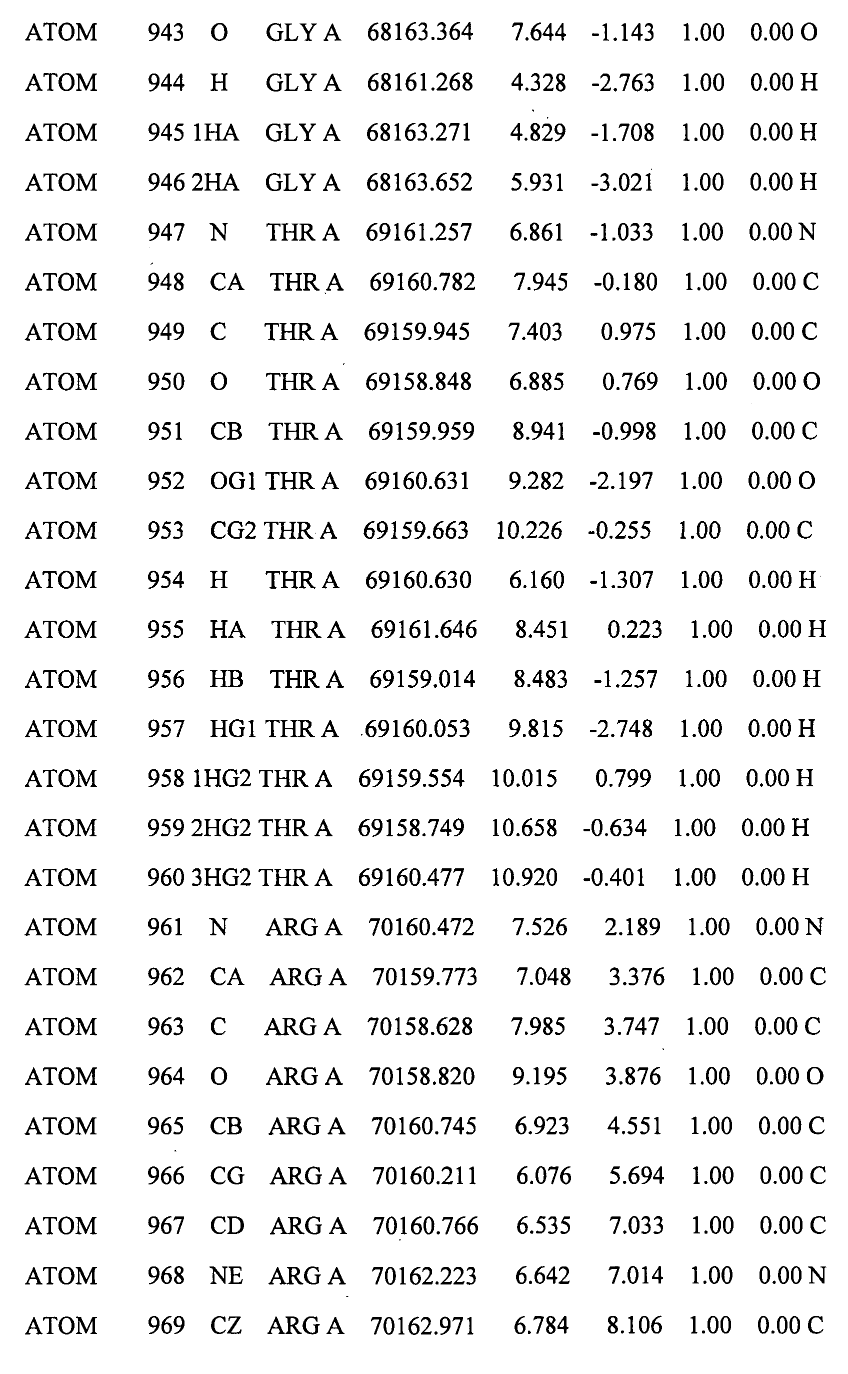

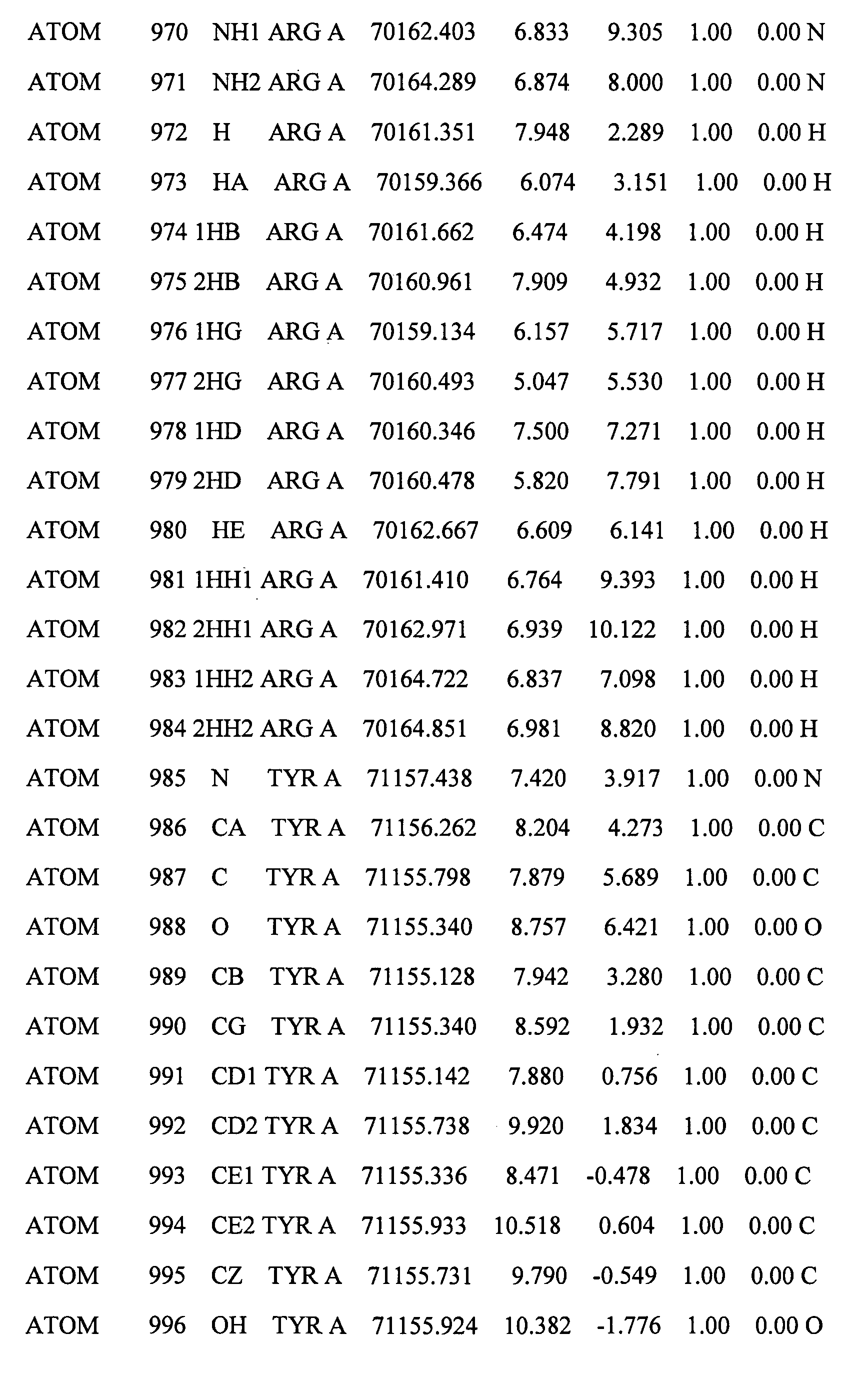

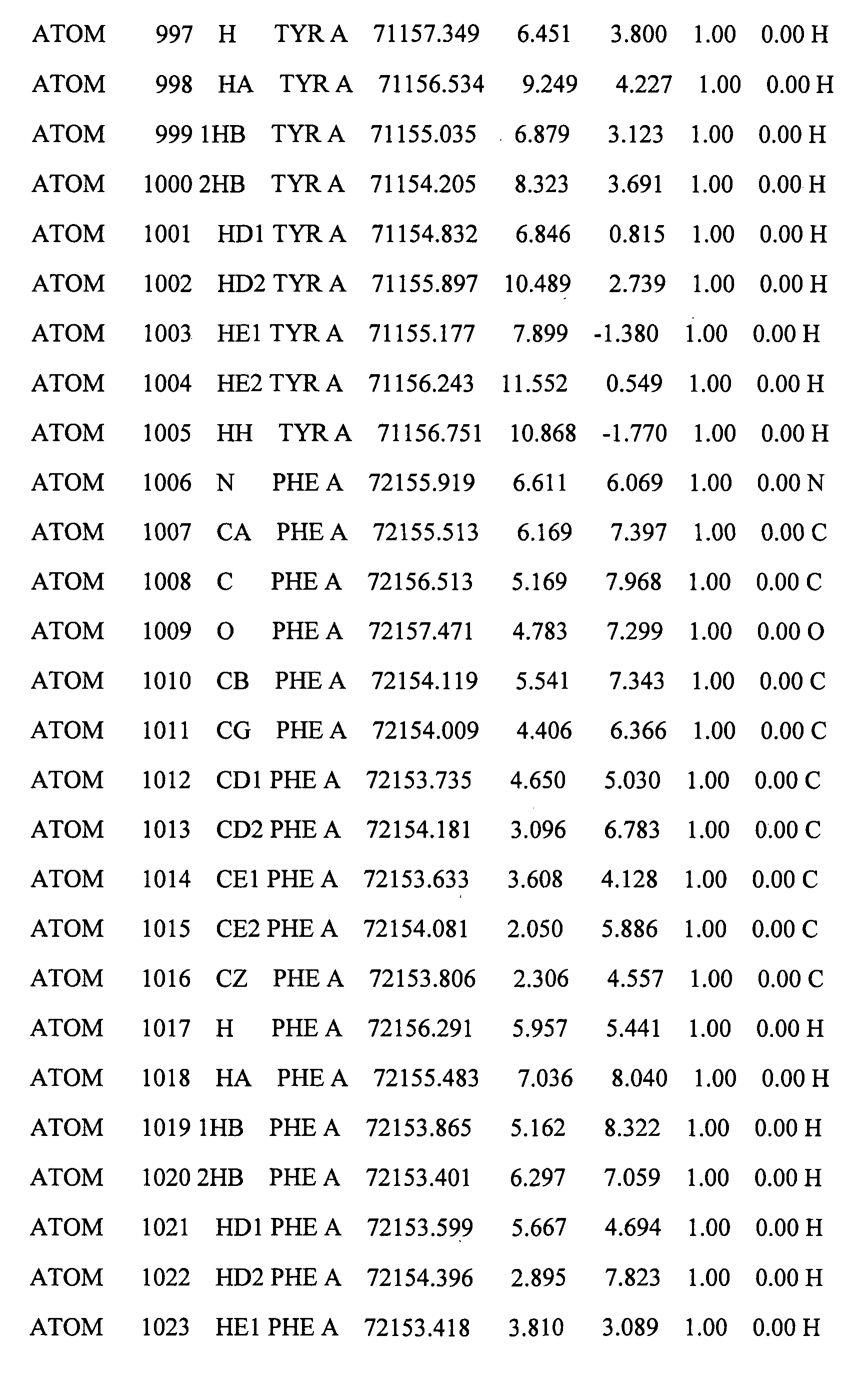

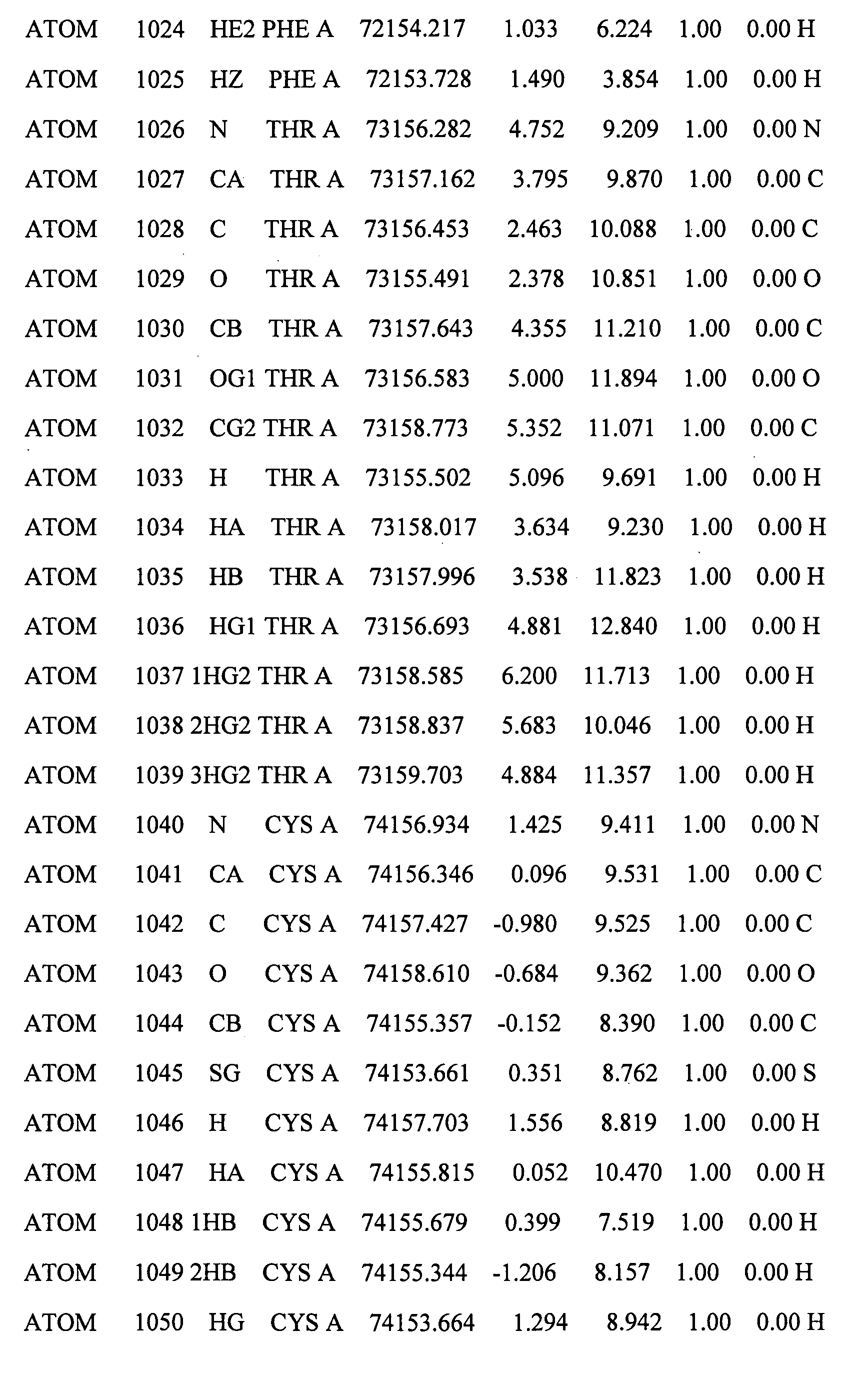

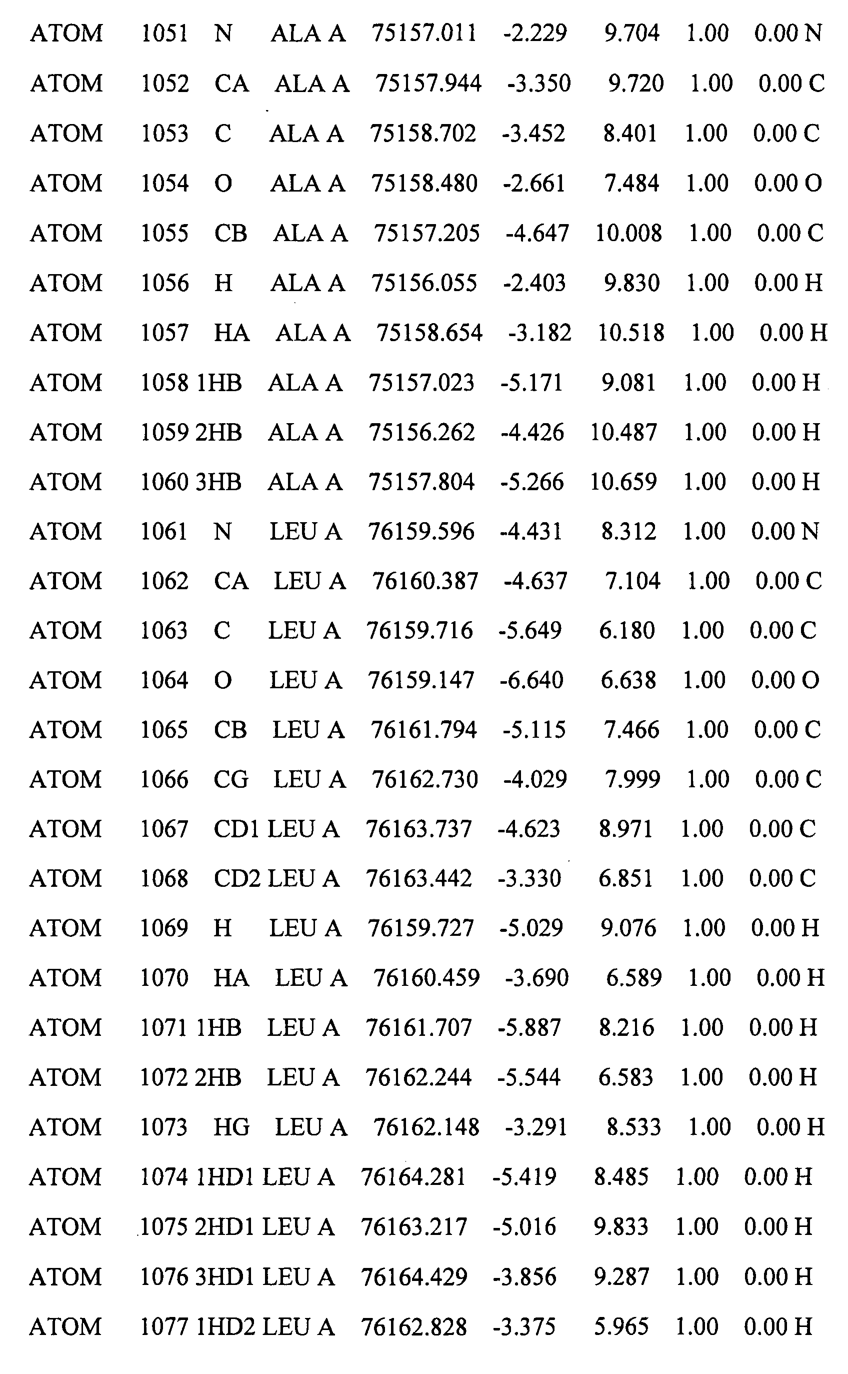

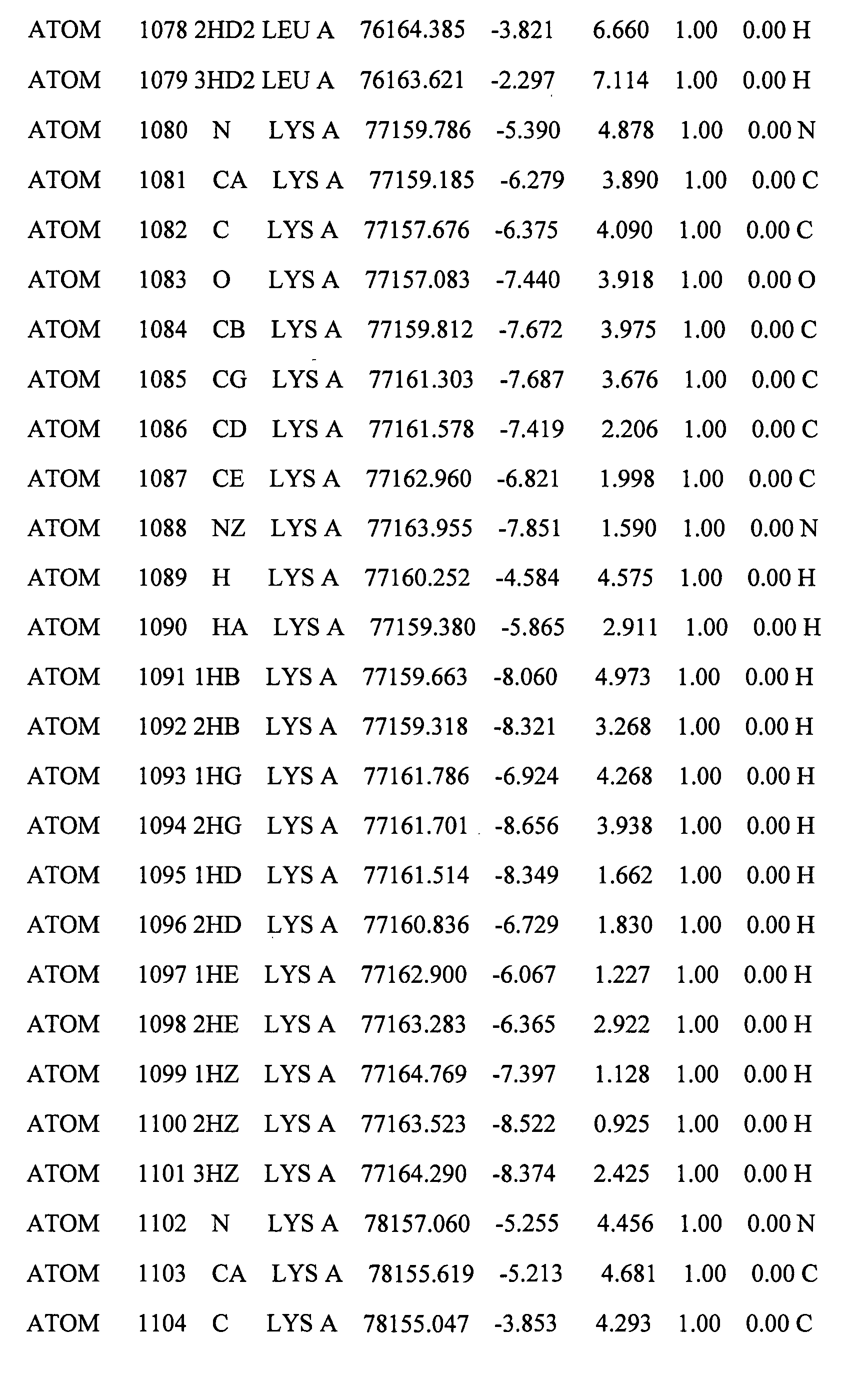

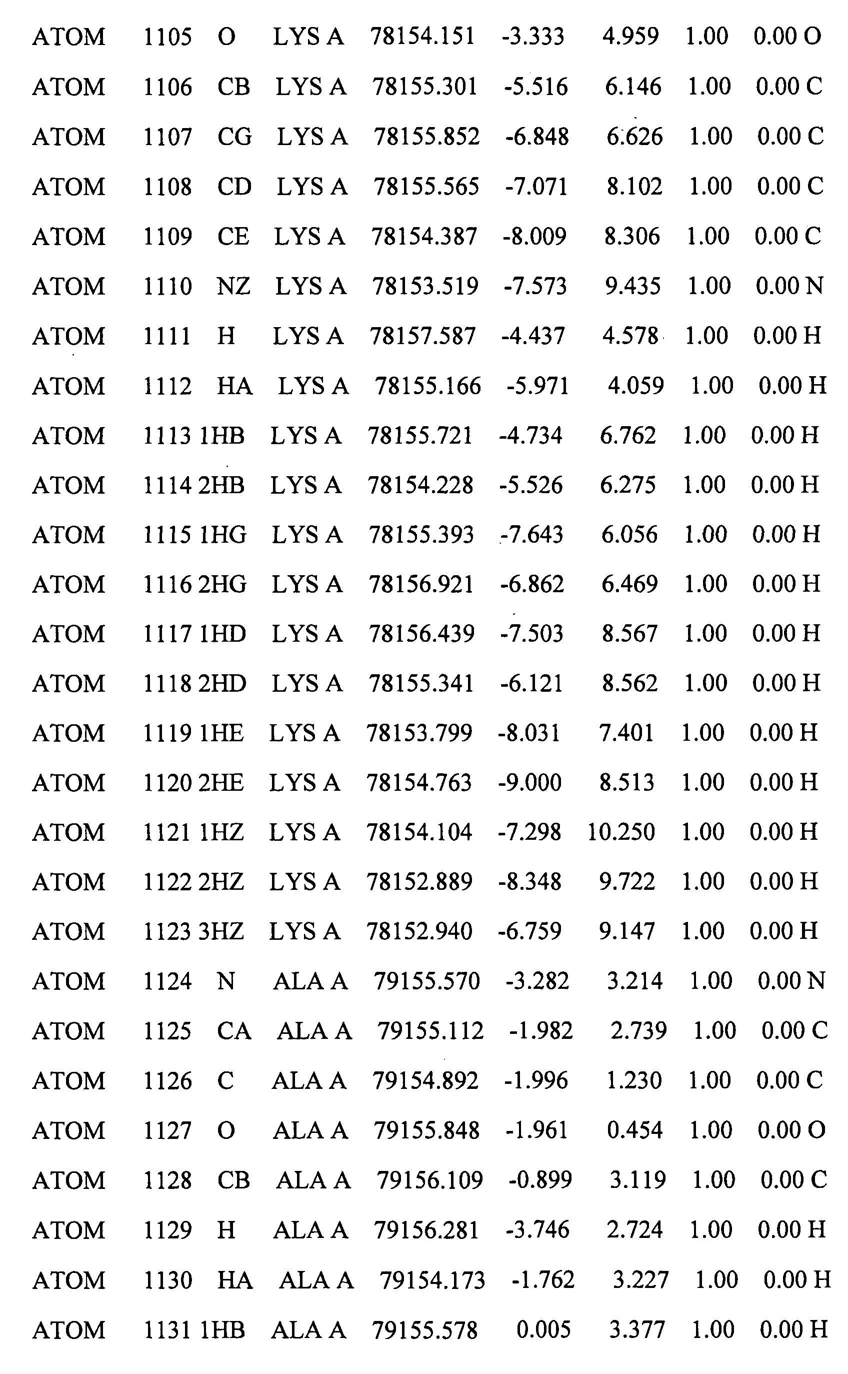

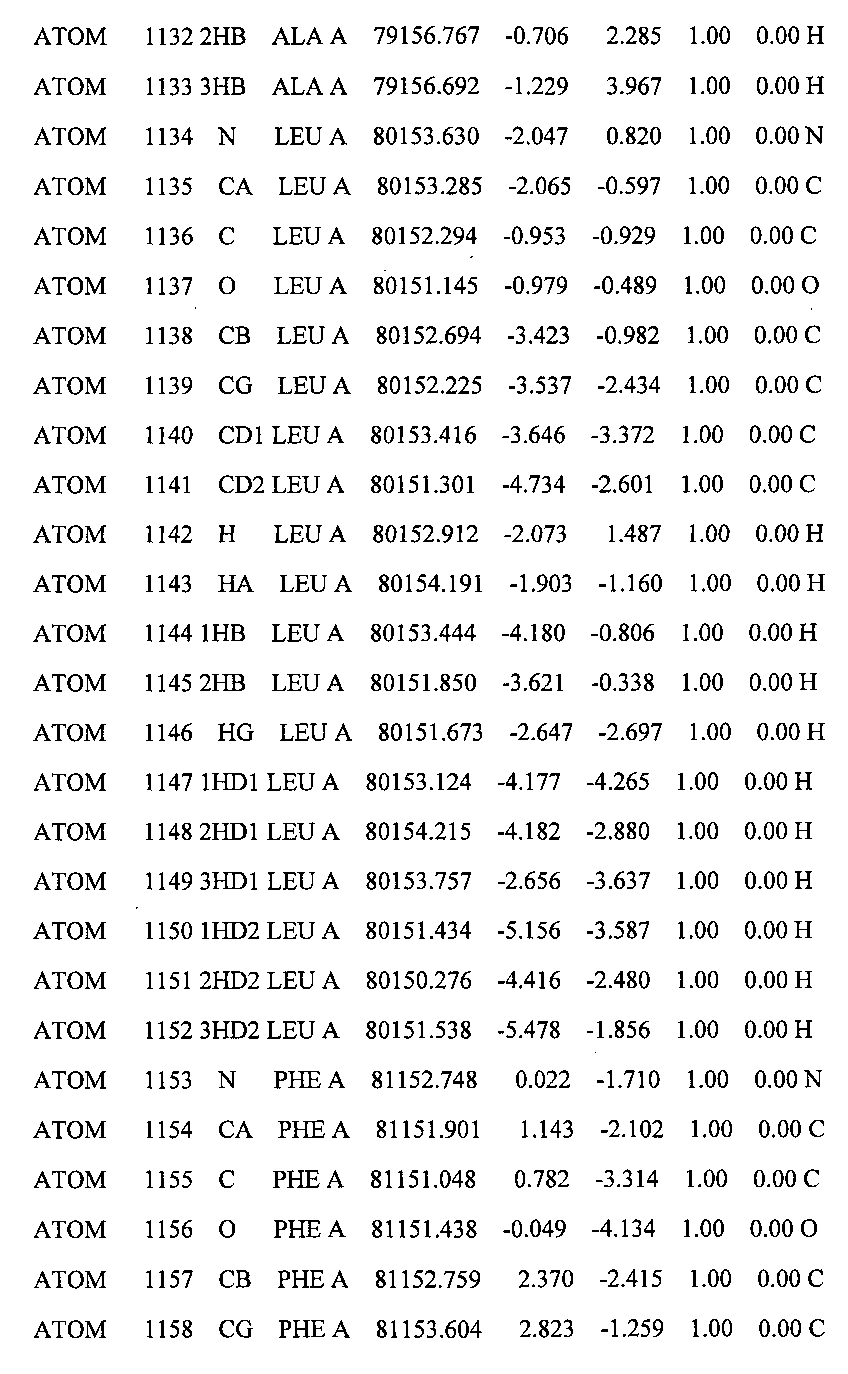

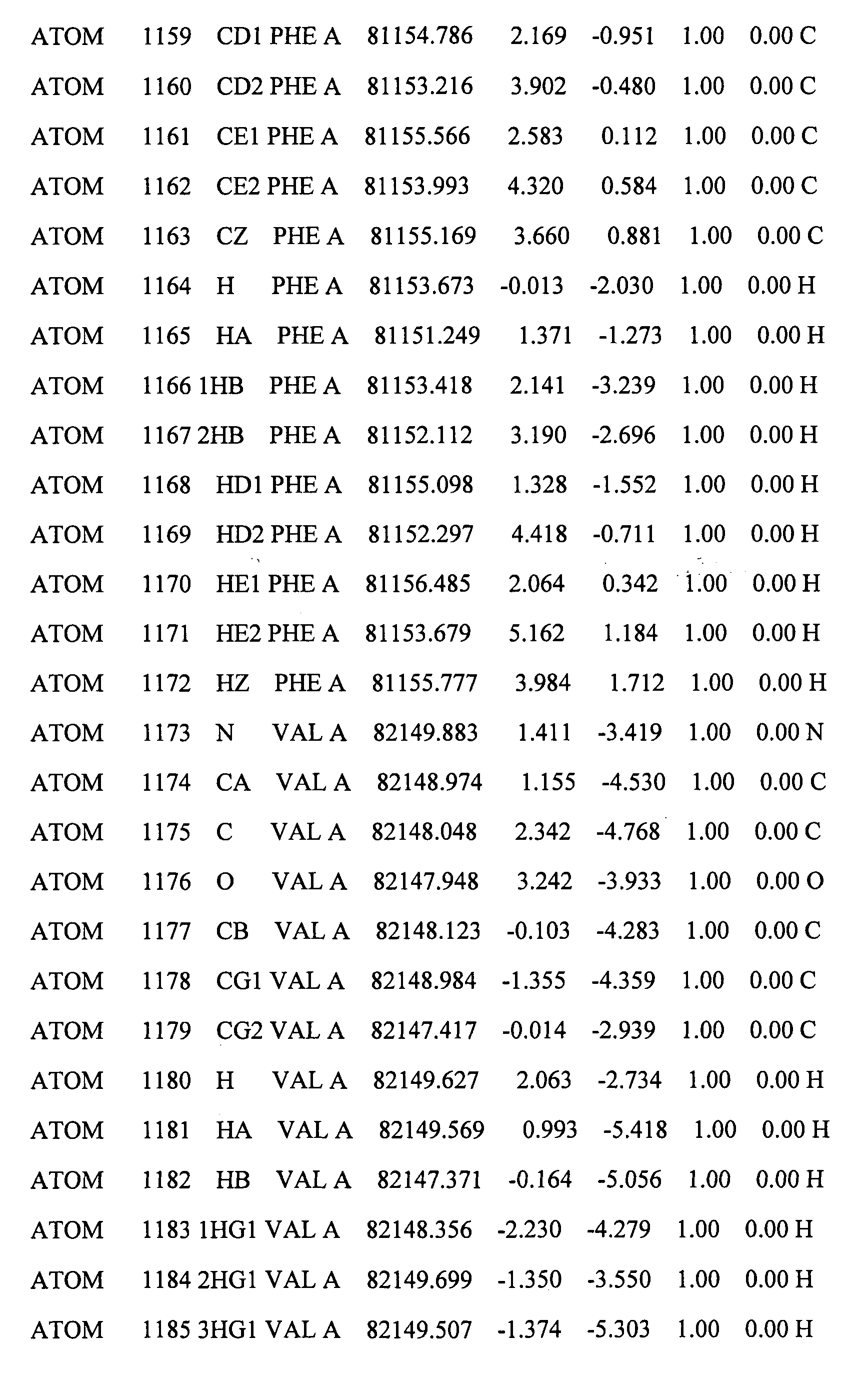

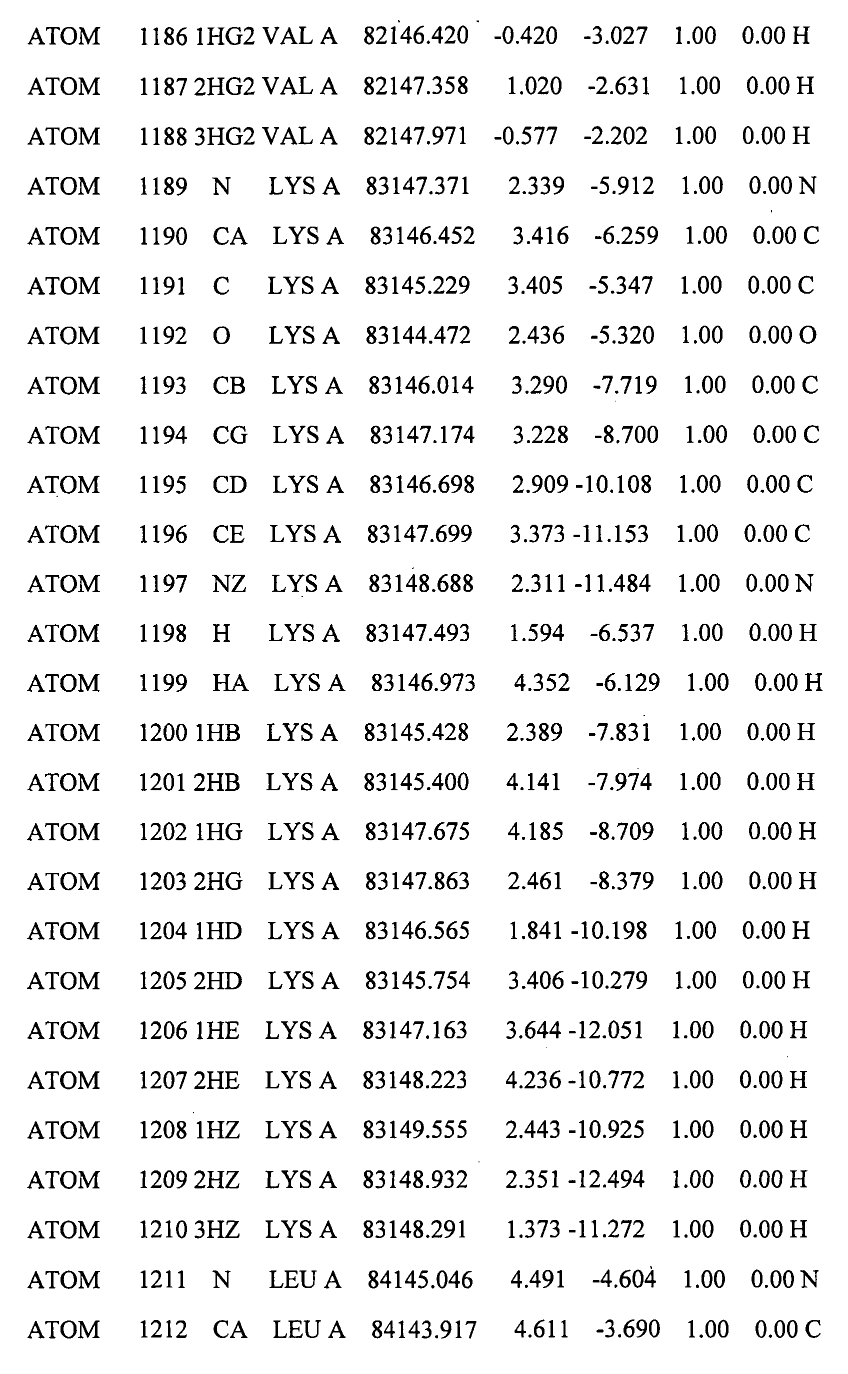

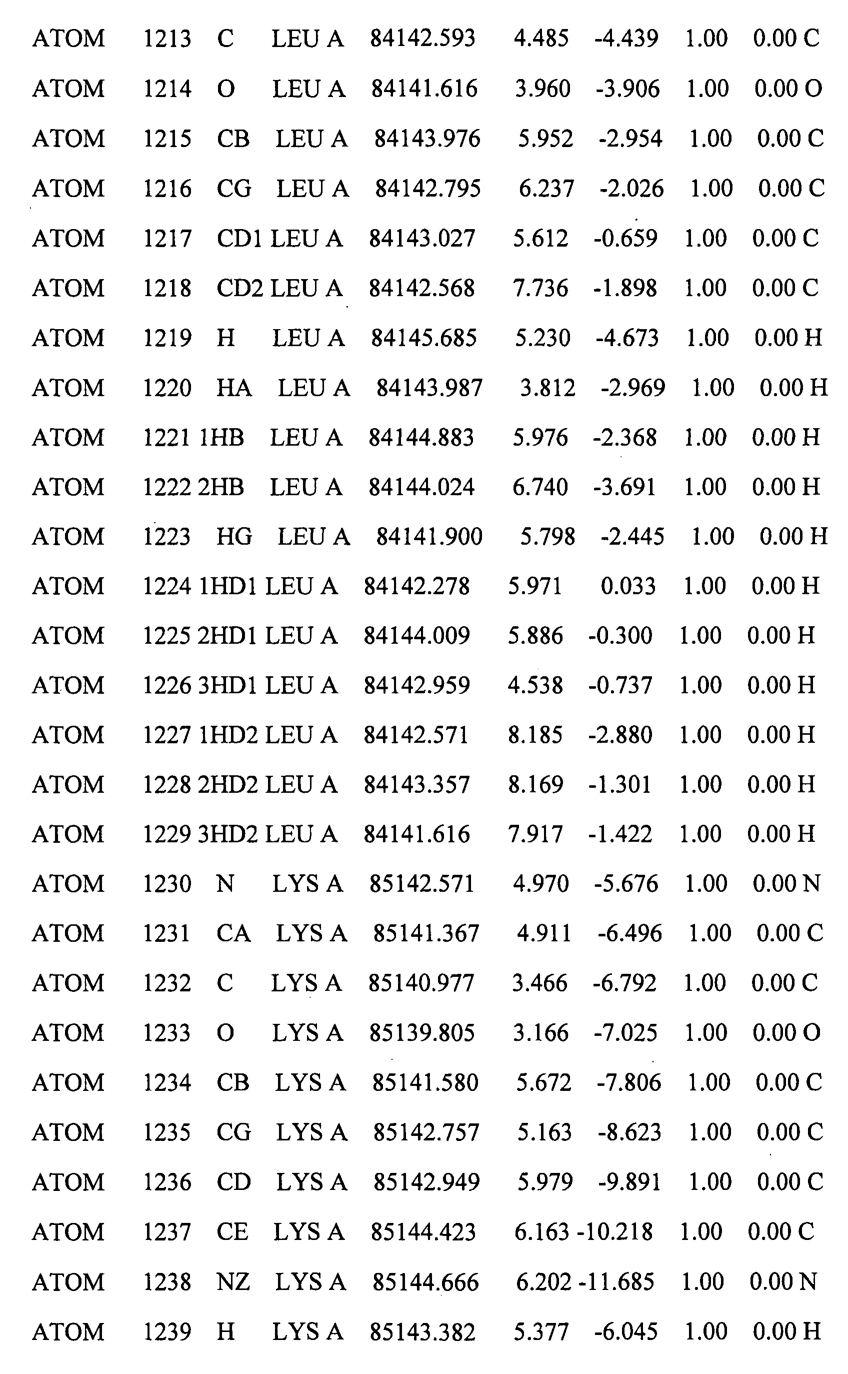

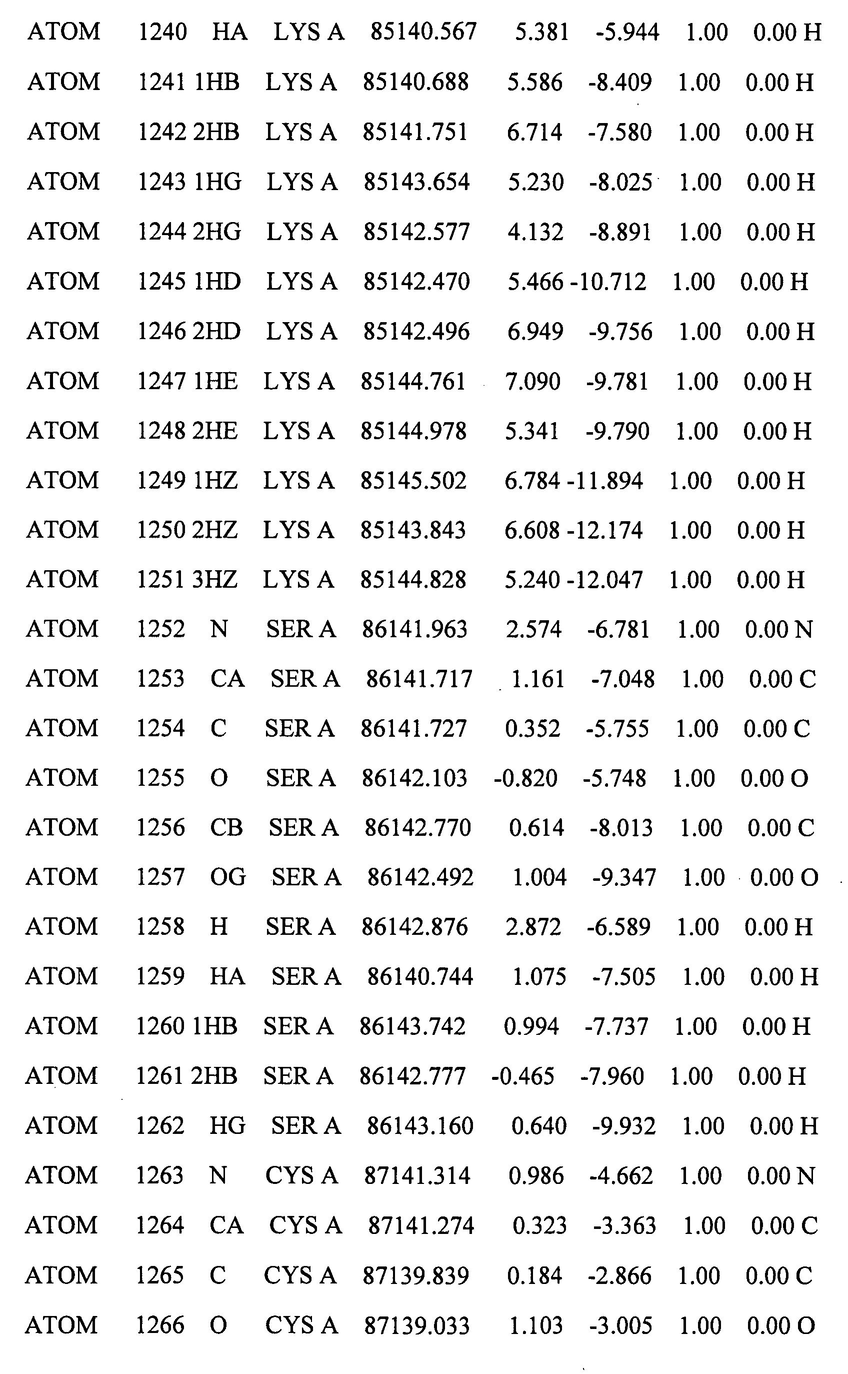

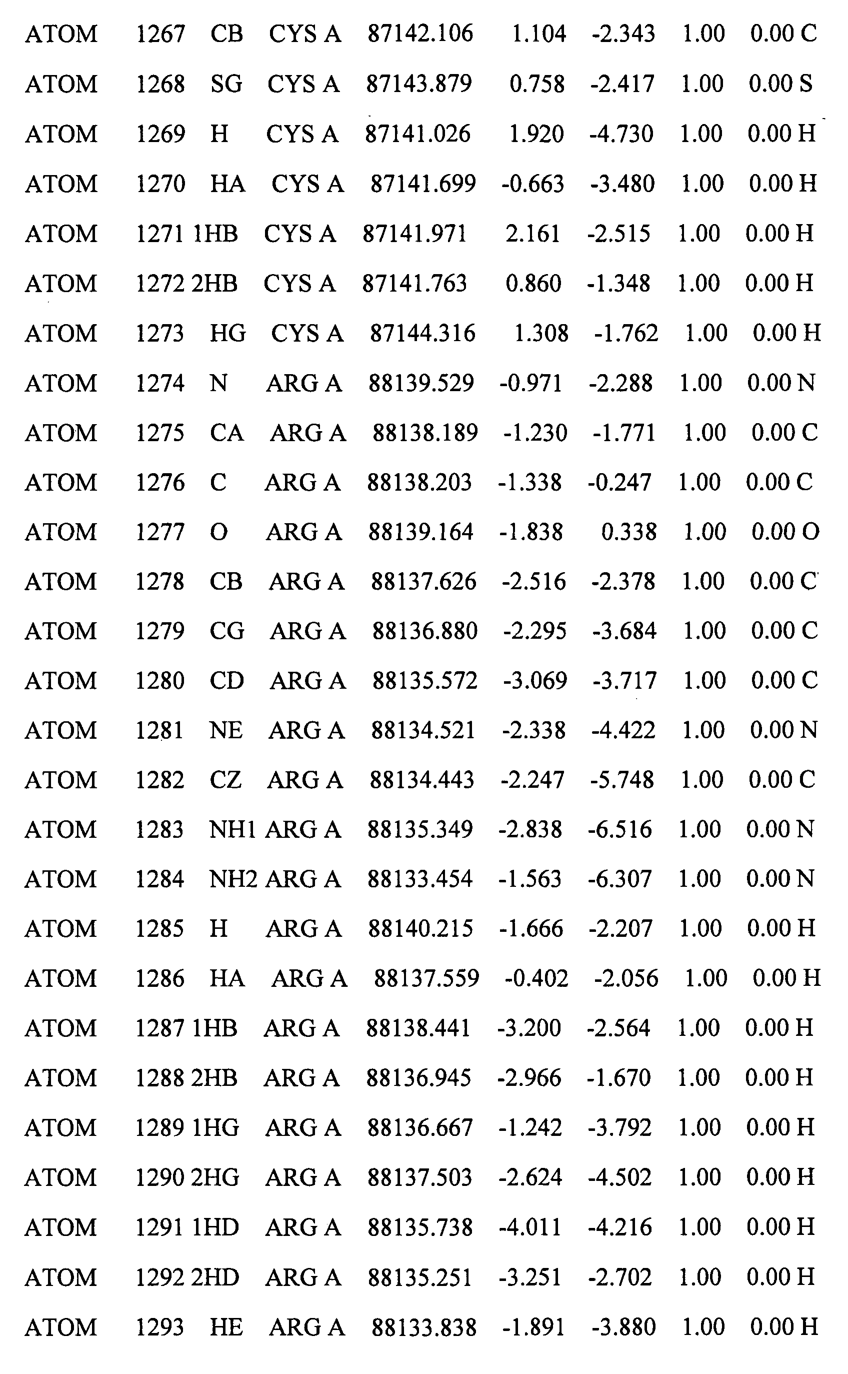

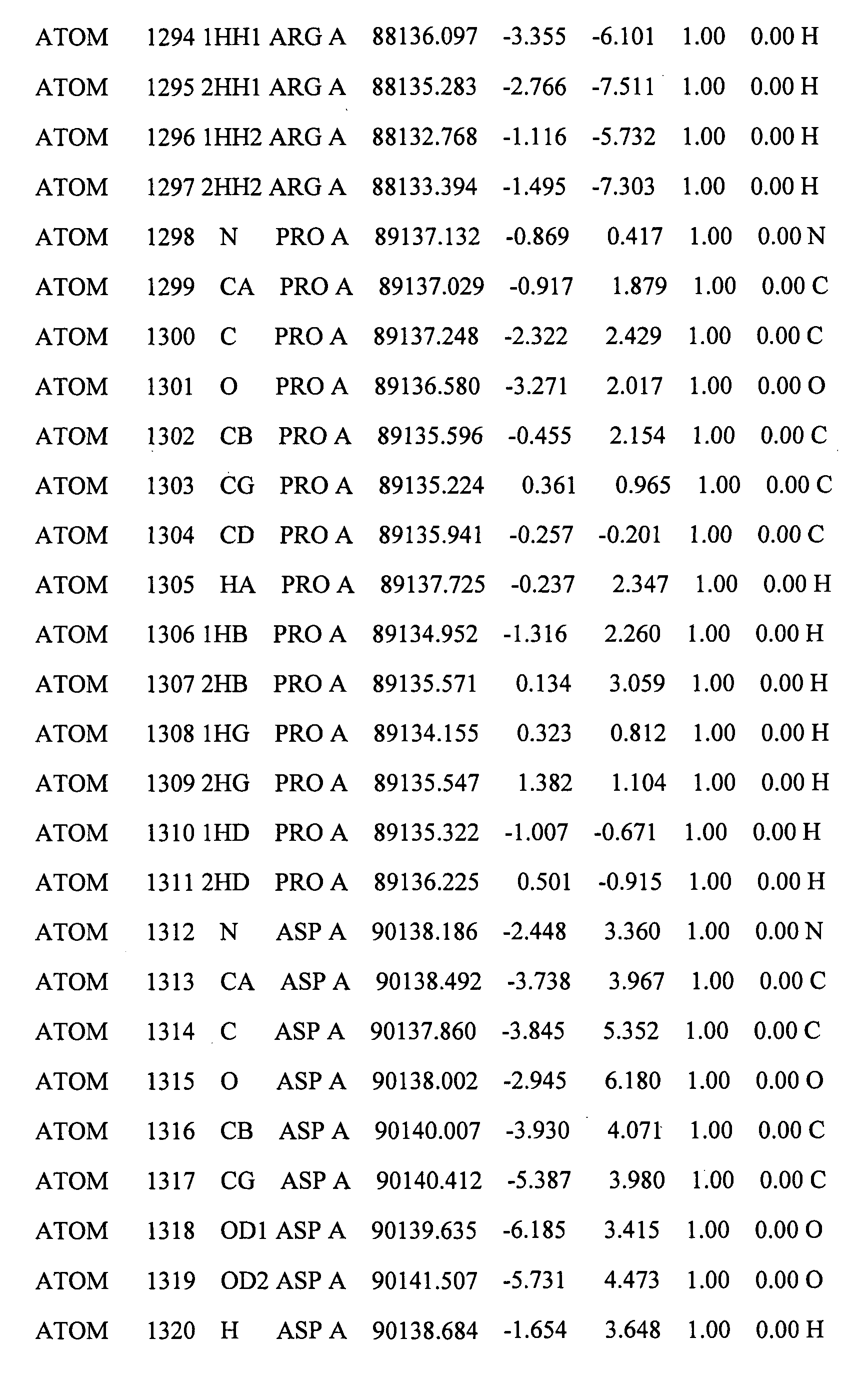

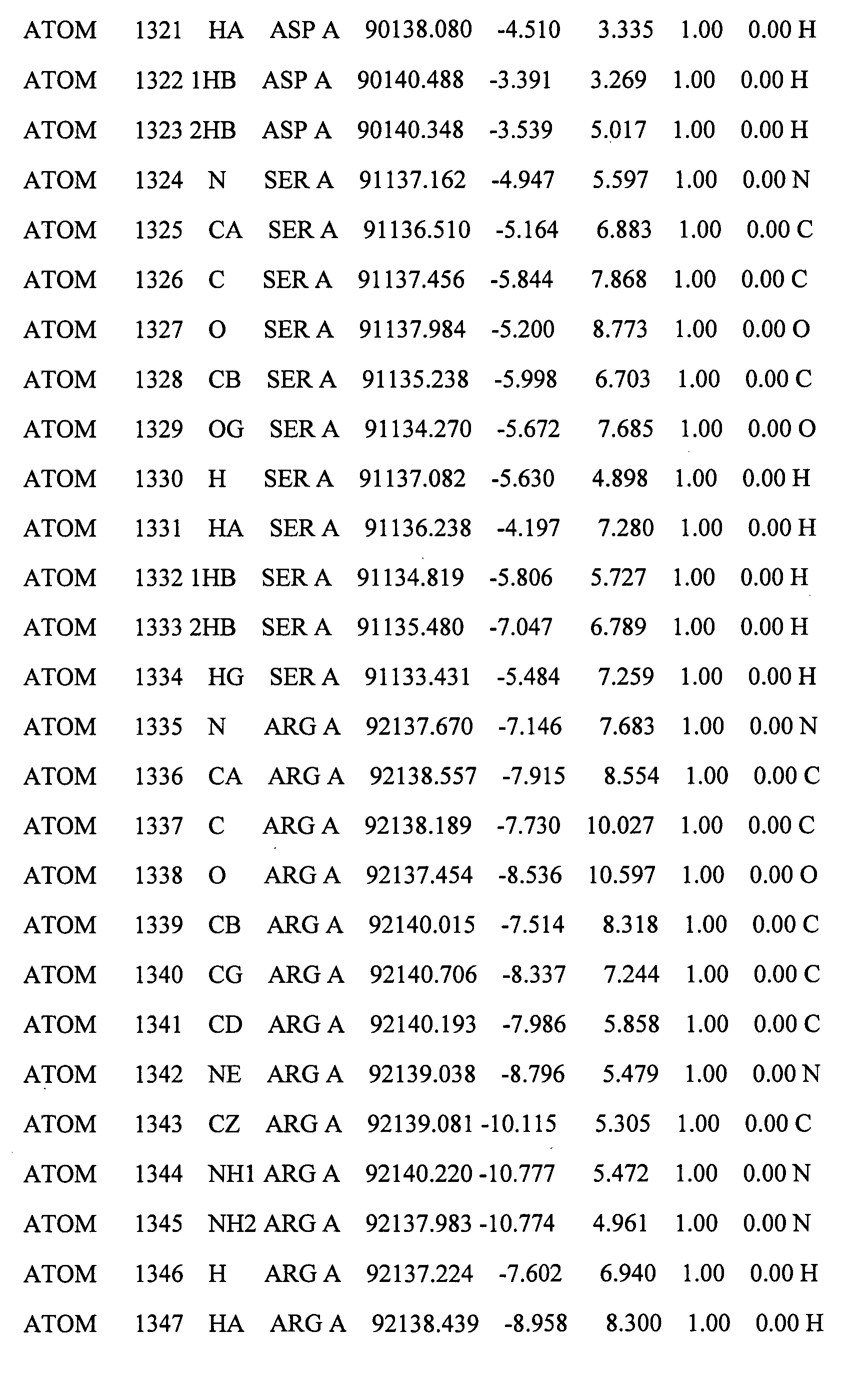

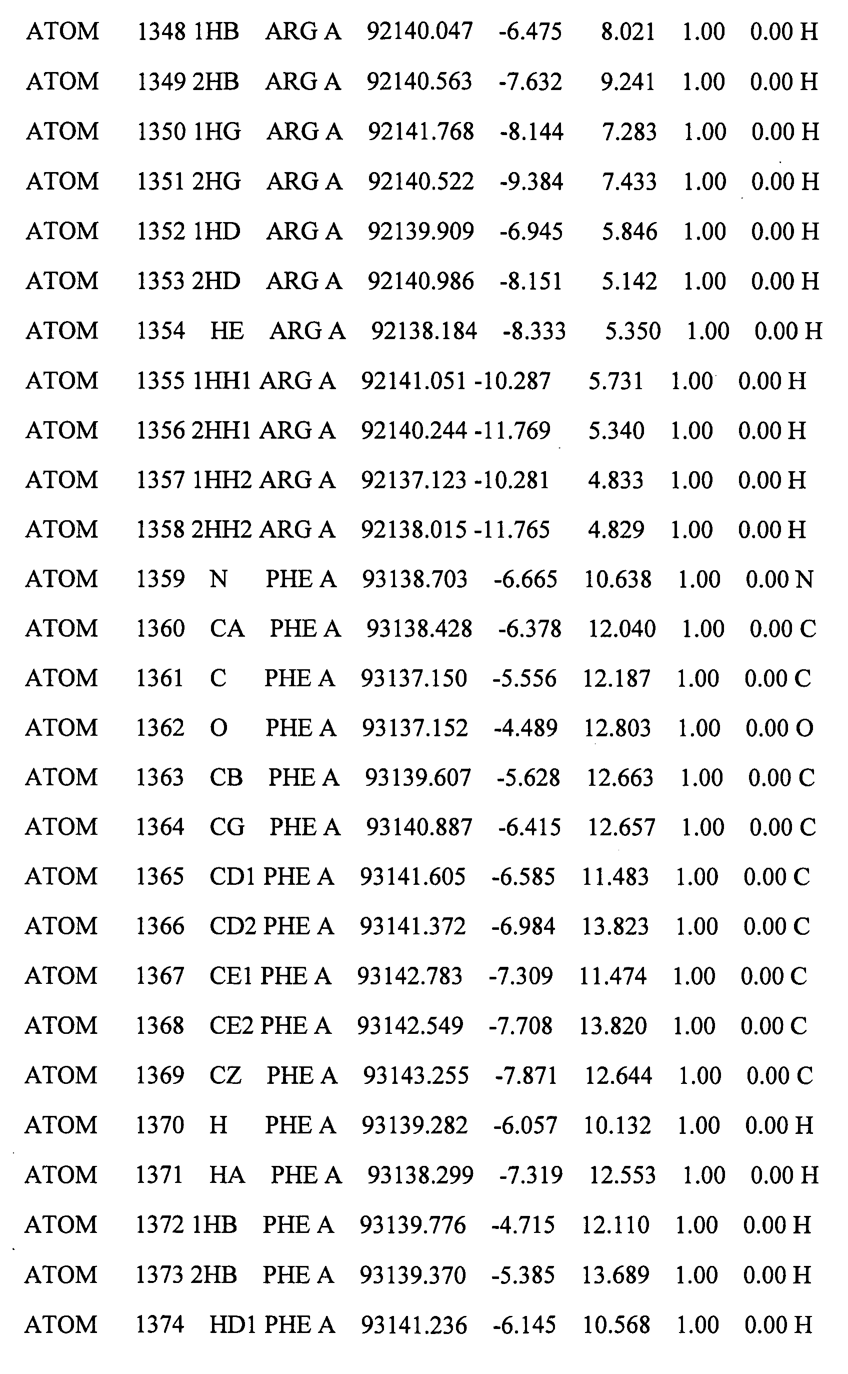

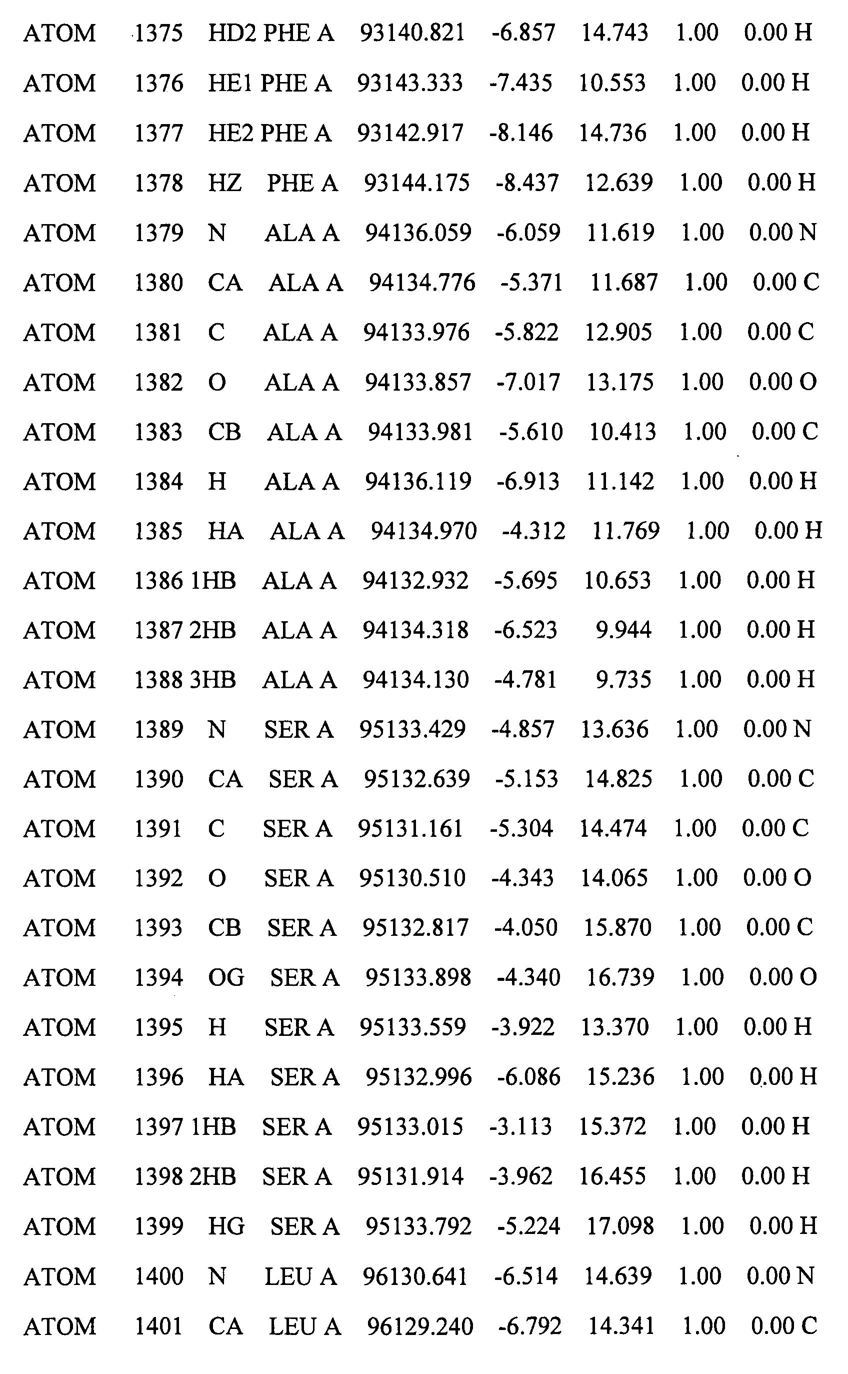

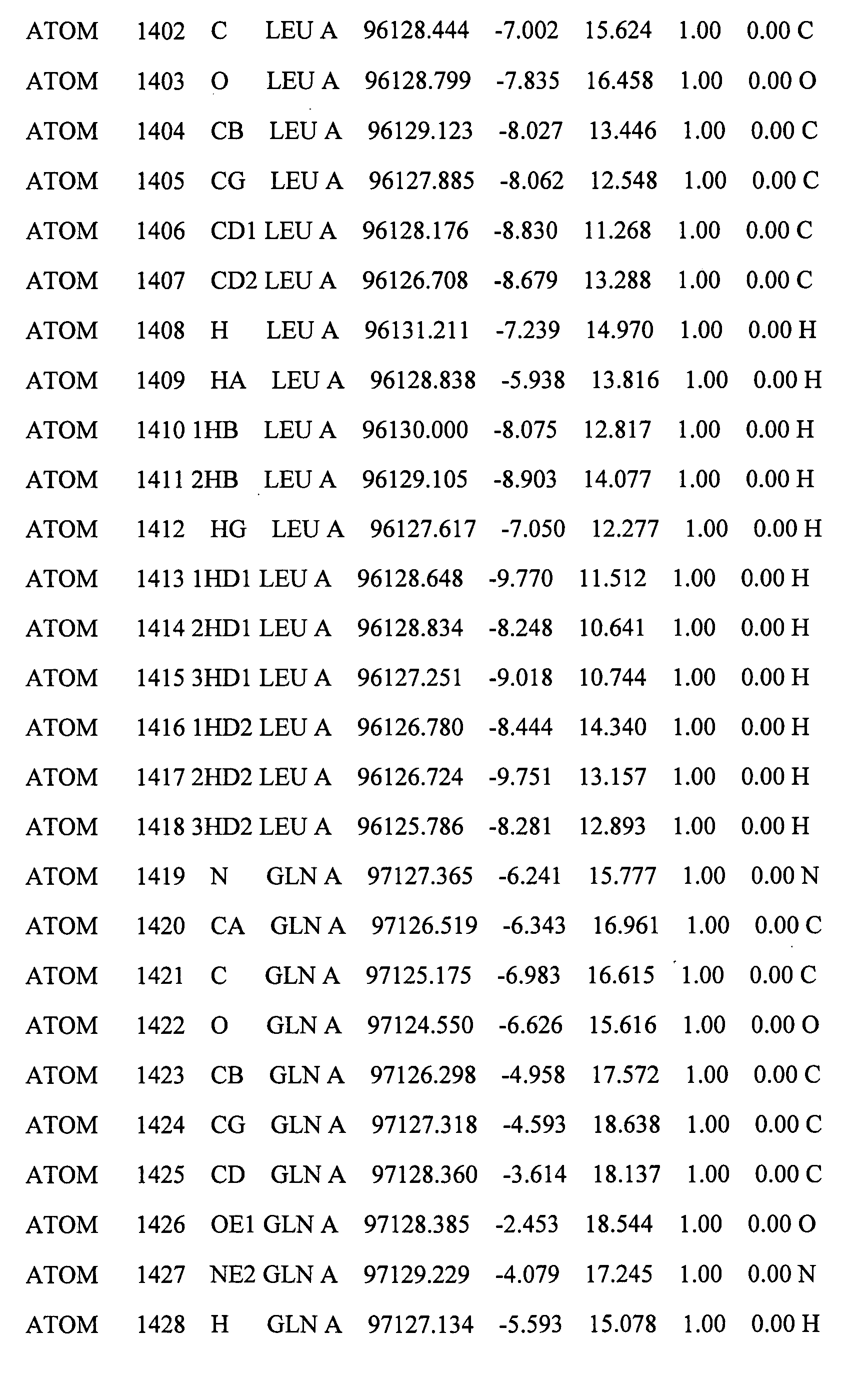

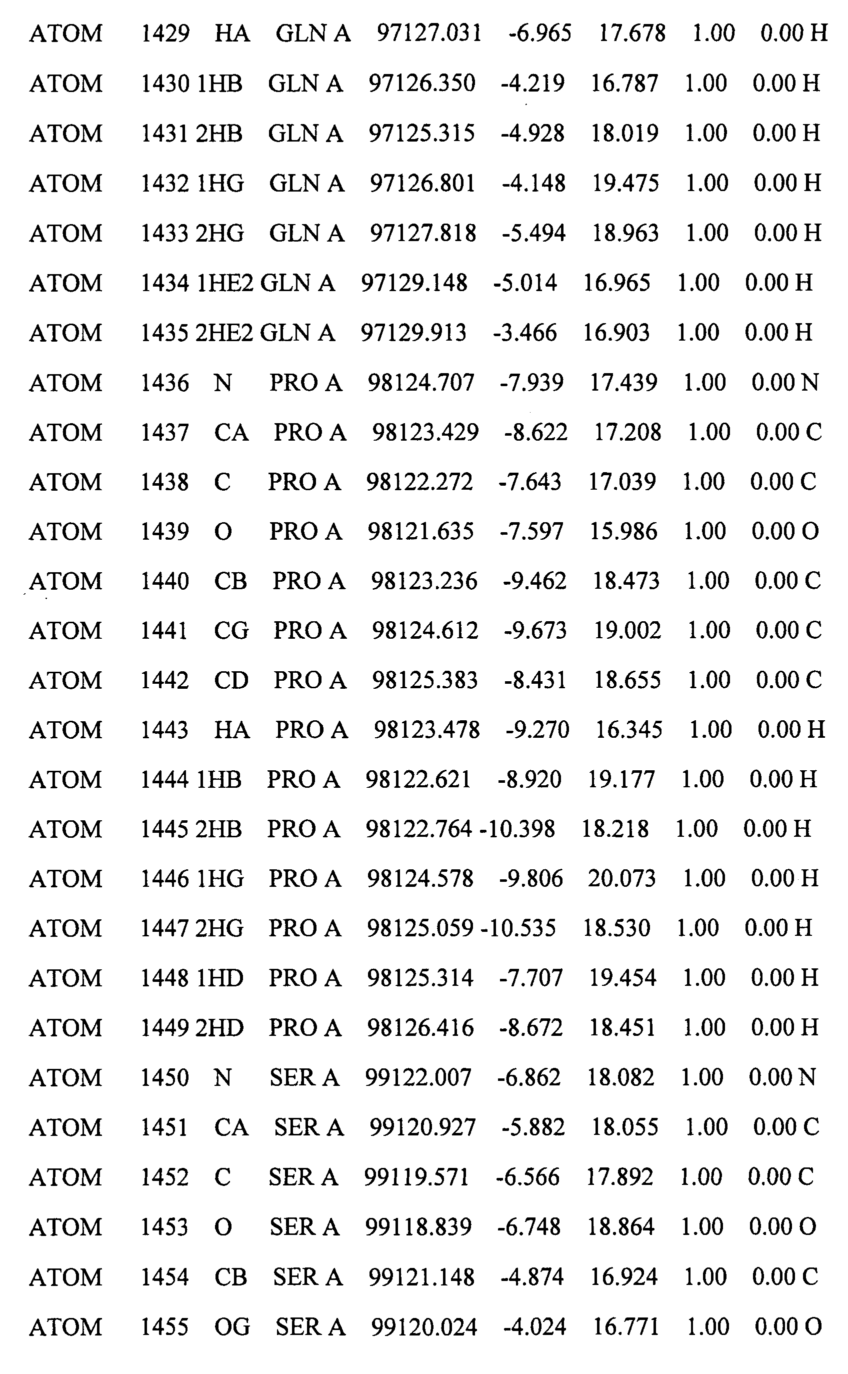

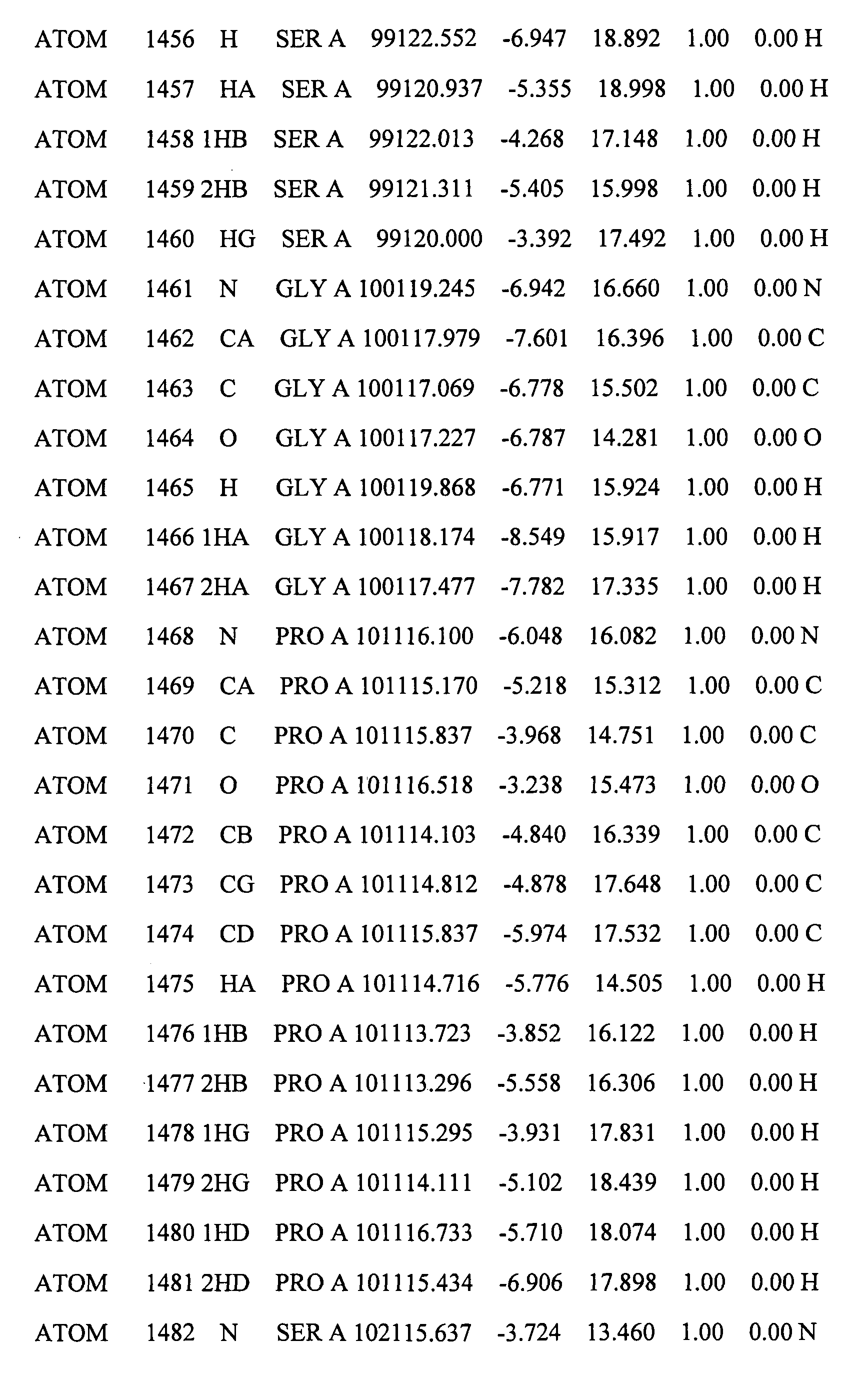

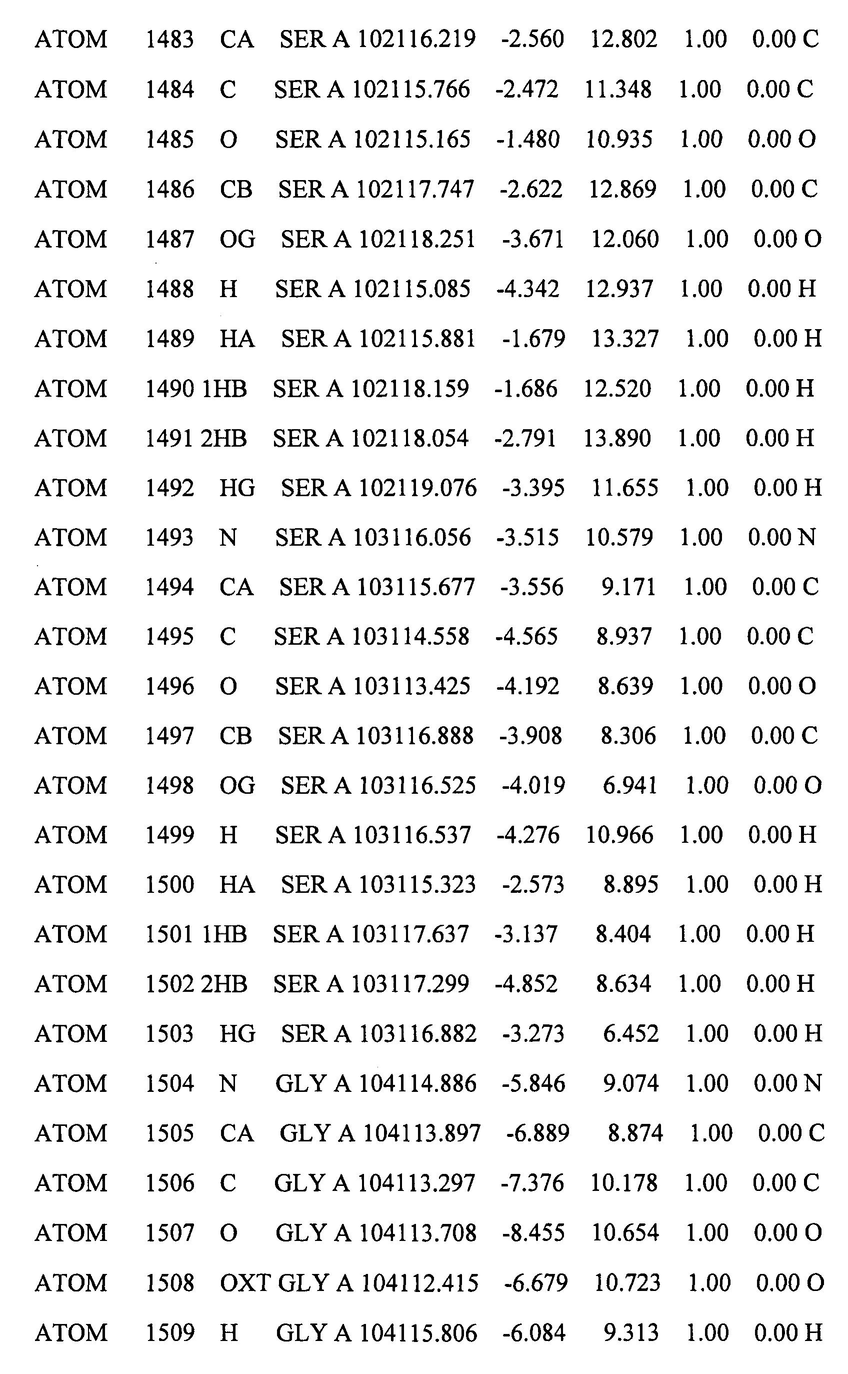

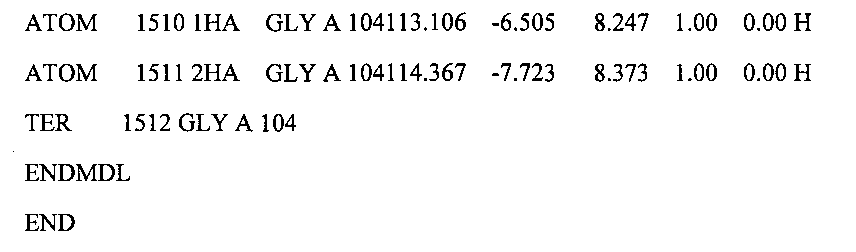

As described above, a constituent element (domain) having protein structure and function was predicted from the full length protein by a computer, and with a focus of the predicted region, extension or cutting was performed to the N- and C-terminals to produce various constructs. These proteins were expressed, and the actually obtained domain proteins were subjected to SDS-PAGE to confirm their expressions. Further, HSQC measurement was conducted using NMR, and all the individual domains were examined to confirm whether they have a structure or not. Therefore, it was confirmed that a position of a domain actually having a structure (folding) was precisely determined on the protein sequence. Additionally, using the domain protein with such low molecular weight, it was confirmed that three-dimensional structure analysis can be easily conducted.

### [Example 7]

Based on the results of the above three-dimensional structural analysis, in silico screening was conducted to search for a ligand candidate and a pharmacophore (binding site) of a CAP-Gly-like protein.

### (1) Database optimization

Low molecular compound catalog database was used, which was provided by SPECS Inc. as a database intended for low molecular compound database. When one entry contains a plurality of molecules, it was divided into one by one molecule. Then, a library without overlaps was used as the population for screening. It contained 152323 molecules.

Based on "Lipinski's Rule of 5," target independent optimization was conducted regarding the low molecular compound database. Herein, the following conditions for refining were used.
1. Molecular weight was not less than 100 and not greater than 500
2. Calculated LogP value (o/w) was 5 or less (XLOGP-1 algorithm was used)
3. The number of hydrogen binding acceptor atoms (the number of N and O contained in a low molecular compound) was 10 or less.
4. The number of hydrogen binding donor atoms (the number NH and OH contained in a low molecular compound) was 5 or less.
Further, the following molecules were eliminated for properly conducting docking calculation.
1. The number of rotatable single bindings is 21 or more.
2. A molecule containing a radical.
3. A molecule containing elements other than H, C, N, O, F, S, P, Cl, Br, and I.

After refining, the number of candidate molecules was 103773.

### (2) Binding site prediction

### (i) Three-dimensional structure analogy search

It was suggested that the CAP-Gly-like protein domain of the present invention had structural analogy with SH3 domain by three-dimensional structure analogy search. Therefore, distance matrices comparison [Holm L, Sander C, J. Mol. Biol. (1993), 233:123-138] was conducted, and three-dimensional structure analogy search with 1 AON, 1 BBZ, 1 CKA, and 1 GBQ (all expressed by PDB code) was conducted. For this three-dimensional structure analogy search, three dimensional coordinate table 1 was used.

### (ii) Predicting binding site.

Although there was a little gap, it was indicated that there was structural analogy between a target and SH3 domain except fragments at the N-terminal and C-terminal side. On the other hand, a structure containing SH3 domain was bound to a peptide in the vicinity of a common hydrophobic pocket. Since this pocket was common with the CAP-Gly-like protein domain of the present invention, this site vicinity was selected as a screening target.

### (3) Screening

### (i) Primary screening

Using Dock 4.0, docking was conducted, regarding binding site, on the entire low molecular compound library, which had been optimized in advance. Based on the energy score of Dock, low molecular compounds were ranked.

### (ii) Secondary screening

Regarding highly ranked 10619 molecules obtained as a result of the primary screening, detailed docking was conducted using AutoDock 3.0.5. Further refining was performed to compounds within 4 Å from the sphere used in docking, and molecules for which calculation was abnormally terminated were eliminated. As a result, docking structures of 9938 molecules were finally obtained. Further, among the selected compounds, further selection was conducted using ΔG (binding free energy change)=-6.89 and Kd=about 1 µM or less as standards. Then, 1,000 compounds having higher binding ability than the standards were regarded as ligand candidates.

### (4) Search for important residue in pharmacophore definition

From three dimensional coordinate information of a target CAP-Gly-like domain protein, a spherical probe called sphere was produced on the target surface by Dock-attached program SPHGEN. Resides within 4 Å from HPD residue were selected, and thereby important amino acids (Val26, Lys27, Glu47, Arg67, Lys83, and Ser86) in pharmacophore definition were obtained.

### [Example 8]

### Measurement of cell proliferation

### (1) Cell culture

### (i) HeLa cell

Using a Dulbecco's MEM medium (Dulbecco's modified essential medium; Sigma) containing 10% FBS (fetal bovine serum; available from ICN) for cell culture, 2 mM of L-glutamine (Sigma), 100 U/ml of penicillin, and 100 µg/ml of streptomycin (Sigma), HeLa cells were cultured in a humidified atmosphere with 5% CO₂ at 37°C.

### (ii) 293 cell

Using a Dulbecco's MEM medium (Dulbecco's modified essential medium; Sigma) containing 10% FBS (fetal bovine serum; available from ICN) for cell culture, 2 mM of L-glutamine (Sigma), 0.1 mM of non-essential amino acids (Sigma), 1 mM of sodium pyruvate (Sigma), 100 U/ml of penicillin, and 100 µg/ml of streptomycin (Sigma), 293 cells were cultured in a humidified atmosphere with 5% CO₂ at 37°C.

### (2) Gene transfer of the protein of the invention into cultured cells

cDNA (SEQ ID NO:2) was amplified by PCR, which was a gene fragment (corresponding to amino acids 464-554) derived from KIAA0849 containing an amino acid sequence represented by SEQ ID NO:1. The amplified cDNA (SEQ ID NO:2) was introduced into a BamHI/NotI multicloning site of a vector (Gateway system; Clontech) and subcloned. Thereafter, the introduction part was introduced by ligation reaction into pDEST26 (Clontech), an expression vector of eukaryote. Onto a dish with a diameter of 60 mm, 10⁵ of subcloned cells were plated, and then incubated for about 24 hours. Using LipoFectamine 2000 (Invitrogen), the gene was transiently transfected into the cells. In addition, a cell into which only pDEST26 was transferred was prepared as control.

### (3) Measurement of cell proliferation

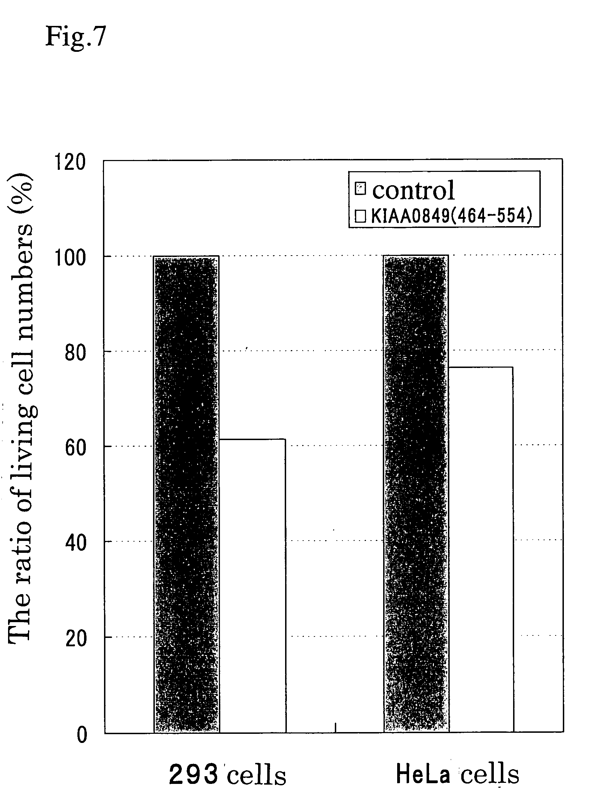

To measure cell proliferation, the following control experiment was conducted. First, prepared were a 293 cell and a HeLa cell described above, into each of which a plasmid pDEST26 having cDNA with a nucleotide sequence represented by SEQ ID NO:2 introduced thereinto was introduced, and these cells having no such plasmid introduced thereinto. Then, the effect of CAP-Gly-like domain on cell proliferation was examined. After 24 hours from the gene transfer, adherent cells were removed by trypsin treatment, and cells were collected. The obtained cells were resuspended in phosphate buffered saline (PBS). Then, 0.4% of trypanblue (Sigma) was mixed at the same volume of the resuspended cells, and the mixture was incubated at room temperature for 10 minutes. The number of unstained living cells was measured by a standard hemocytometer (Sunlead Glass Co.) Measurements were performed independently three times, and their average and standard deviation (SE) were obtained. Results thereof are shown in Fig. 7. According to Fig. 7, it is found that, in either case of 293 cells and HeLa cells, cells having cDNA represented by SEQ ID NO:2 transfected thereinto decreased their living cell numbers compared with cells having no such transfection.

In view of the foregoing, it is found that the expression of the protein of the present invention in a cultured animal cell allows proliferation inhibition of the cell to be controlled.

### [Example 9]

### Binding experiment of CAP-Gly-like domain protein (464-554) and IKK-gamma (1-419)

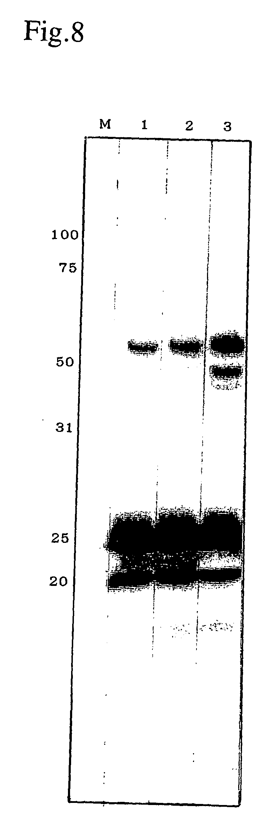

Hereinafter, it has been experimentally confirmed that the domain protein of the present invention is bound to IKK-gamma (1-419).
(1) Using a DMEM medium containing 10% fetal calf serum (FCS), HeLa cells were cultured in a humidified atmosphere with 5% CO₂ at 37°C. Using LipoFectamine 2000 reagent (Invitrogen), cDNA (SEQ ID NO:22) of IKK-gamma (1-419) and cDNA (SEQ ID NO:2) of a CAP-Gly-like domain (464-554) were transferred and expressed in the cultured HeLa cell. The IKK-gamma (1-419) has myc tag added to its N-terminal, but it does not have particular effect in this experiment. After 24 hours from the gene transfer, washing with PBS was performed three times. Using a lysis buffer and 200 µl of an inhibitor, cells were collected and cell membranes were broken. The lysis buffer contained 30 mM oftris-HCl (pH 7.4), 150 mM of NaCl, 5 mM of EDTA, and 1% Triton X100. The inhibitor contained 10 µg/ml of leupeptin, 1 µg/ml of aprotinin, 1 µg/ml of pepstain, 1 mM of Na₃VO₄, 40 mM of β-glycerophosphate, and 1 mM of PMSF. The collected extract was allowed to stand at 4°C for 10 minutes, and then centrifuged at 4°C for 30 minutes to precipitate unnecessary components of the cell extract, thereby obtaining a supernatant. The revolution speed of the centrifuge was 1500 rpm. After the centrifugation, 30 µg of protein G gel (Sigma) was added to the obtained supernatant, and the mixture was allowed to stand at 4°C for 30 minutes. Thereafter, 10 µg of anti-FLAG-M2 affinity gel was added to the supernatant, and the mixture was allowed to stand at 4°C for 2 hours. After standing, washing was performed three times with 0.5% of TBS and 500 µl of Triton X100. Then, washing was performed further twice with TBS. After washing, cells were eluted with 20 µl of sample buffer, and the obtained cells were heated at 98°C for 5 minutes.
   A sample was subjected to SDS gel electrophoresis for separation. Here, PAG mini 10/20 (Daiichi Pure Chemicals Co., Ltd.) was used as a gel. Thereafter, proteins in the gel were electrically transcribed to a PVDF membrane (transcription conditions: at 80 mA for one hour). Using 1000-times diluted mouse anti-IKK-gamma antibodies (Pharmingen) as primary antibodies, the PVDF membrane was reacted with the antibodies for one hour. Further one hour reaction was conducted using 1000-times diluted mouse IgG horseradish peroxidase (HRP)-conjugated whole antibodies (Amersham) as secondary antibodies.
(2) Also as controls, prepared were a cultured HeLa cell having cDNA (SEQ ID NO:22) of IKK-gamma (1-419) transferred thereinto and expressed therein, and a cultured HeLa cell haying no gene transferred thereinto.
(3) A detection image of western blotting was obtained using ECLplus (Amersham Pharmacia) and LAS-1000 (Fuji Film) as a detection reagent and a chemiluminescent detector, respectively. Thus obtained detection image of western blotting is shown in Fig. 8. Fig. 8 is an image detected by chemiluminescence of substrate decomposition of horseradish peroxidase after the purification by anti-FLAG M2 affinity gel and the transcription to PVDF. In Fig. 8, lane 1 is a lane of only HeLa cells (control), lane 2 is a lane of a product wherein IKK-gamma (1-419): SEQ ID NO:21 and a FLAG sequence were expressed in a HeLa cell, and lane 3 is a lane of a product wherein IKK-gamma (1-419) and addition of a FLAG sequence to a CAP-Gly-like domain protein (464-554) were expressed in a HeLa cell. In the lane 3, IKK-gamma was detected by chemiluminescence. This detection is considered to occur because the CAP-Gly-like domain protein (464-554) and IKK-gamma (1-419) were bound with each other to form a complex; and in spite of anti-FLAG M2 affinity gel purification, IKK-gamma remained as a complex. As a result thereof, it was confirmed that the GAP-Gly-like domain protein (464-554) and IKK-gamma (1-419) were bound with each other.

### Industrial Applicability

As described above, since the domain protein of the present invention has physiologically significant structure and protein molecular function, this domain protein can be used for screening a physiologically active substance interacting with this domain protein. In addition, three-dimensional structure analysis of the domain protein of the present invention allows a compound affection the domain to be searched for and designed on a computer.

Moreover, in searching for a compound by wet testing, the domain protein of the present invention is a minimum protein cassette having only a specific molecular function, and thus screening of an active compound can be effectively carried out without being affected by undesired protection structure.

According to these results, it is possible to conduct screening of a compound having interaction with the domain protein and/or a natural protein containing the domain protein.

Hence, the provision of the domain protein according to the present invention enables effective genome drug discovery on the basis of protein structure-function analysis.

Further, the protein of the present invention can be used for screening a drug for Turban tumor syndrome or a drug can be optimized by use of three-dimensional structure information. In other words, a compound having interaction with a domain protein with CAP-Gly function can be effectively used for the prevention and treatment of various cancer-related diseases. In addition, it was found that the protein of the present invention is bound with an IKK-gamma protein. The IKK-gamma protein interacts with NF-kB, a cancer-related protein and inhibits transcription enhancing function of NF-kB. Therefore, the protein of the present invention can be effectively used for the prevention and treatment of cancer-related diseases that particularly involve NF-kB.

## Claims

1. A protein consisting of an amino acid sequence represented by SEQ ID NO:1, or a salt thereof.

2. A protein consisting of an amino acid sequence represented by any one of SEQ ID NOS:3, 5, and 7, or a salt thereof.

3. A protein having an amino acid sequence derived from an amino acid sequence represented by SEQ ID NO:5 by deletion of 0 to 10 amino acid residues from the N-terminal and deletion of 0 to 5 amino acid residues from the C-terminal and having 92 to 106 amino acid residues, or a salt thereof.

4. A protein consisting of an amino acid sequence derived from an amino acid sequence of a proteins according to any one of claims 1, 2, and 3 and having deletion, substitution or addition of one to several amino acids and having a function substantially identical with that of the protein according to claim 1, 2, or 3, or a salt thereof.

5. A polynucleotide comprising a polynucleotide encoding an amino acid sequence of any one of proteins according to claims 1 to 4.

6. The polynucleotide according to claim 5, containing a nucleotide sequence represented by any one of SEQ ID NOS: 2, 4, 6, and 8.

7. A recombinant vector containing a polynucleotide according to claim 5 or 6.

8. A transformant which is transformed with a polynucleotide according to claim 5 or 6.

9. An antibody against a protein according to any one of claims 1 to 4.

10. A method for producing a protein or a salt thereof according to any one of claims 1 to 4, comprising the steps of culturing the transformant of claim 8 and producing the protein.

11. A method for producing a protein or a salt thereof according to any one of claims 1 to 4, **characterized by** using a cell-free protein synthesis system.

12. A method for screening a substance interacting with a protein or a salt thereof according to any one of claims 1 to 4 and/or a naturally existing protein or a salt thereof containing an amino acid sequence of a protein according to any one of claims 1 to 4, comprising the steps of bringing a candidate substance into contact with the protein of any one of claims 1 to 4; and confirming whether the candidate substance interacts with the protein.

13. A method for assaying a protein or a salt thereof according to any one of claims 1 to 4 using an antibody of claim 9.

14. A method for screening a substance interacting with a protein or a salt thereof according to any one of claims 1 to 4 using an assay method of claim 13.

15. A method for specifying a gene associated with a protein according to any one of claims 1 to 4, comprising the steps of expressing the protein according to any one of claims 1 to 4 in a cell; and examining an expression status of the gene in the cell.

16. A method for screening a substance interacting with a protein or a salt thereof according to any one of claims 1 to 4 and/or a naturally existing protein or a salt thereof containing an amino acid sequence of a protein according to any one of claims 1 to 4, comprising the steps of determining an active site of the protein using information concerning three-dimensional structure of the protein according to any one of claims 1 to 4; and specifying a compound interacting with the active site on a computer.

17. The screening method according to claim 16, wherein the information concerning three-dimensional structure of the protein is three-dimensional structure information of a protein consisting of amino acid residues from amino acid 8 to amino acid 98 among three-dimensional structure information described in any of three-dimensional structure coordinate tables 1 to 20.

18. The screening method according to claim 17, wherein, among three-dimensional structure information described in three-dimensional structure coordinate table 1, a part of information corresponding to amino acid residues (Va126, Lys27, Glu47, Arg67, Lys83 and Ser86) is used.

19. A method for screening a substance interacting with a protein or a salt thereof according to any one of claims 1 to 4 and/or a naturally existing protein or a salt thereof containing an amino acid sequence of a protein according to any one of claims 1 to 4, wherein a compound interacting with a specified active site is prepared as a candidate compound by a screening method according to any one of claims 16 to 18, the method comprising the steps of bringing the candidate substance into contact with a protein according to any one of claims 1 to 4; and confirming whether the candidate substance has interaction with the protein.

20. A method for presuming a three-dimensional structure of a protein with an unknown structure, wherein homology modeling is conducted on the protein with an unknown structure comprising an amino acid sequence having 30% or more homology with an amino acid sequence of a protein according to any one of claims 1 to 4, by using information concerning three-dimensional structure information of a protein having amino acid residues from amino acid 8 to amino acid 98 among three-dimensional structures of a protein described in any of three-dimensional structure coordinate tables 1 to 20.
